# EUROPEAN PATENT APPLICATION

(11) **EP 4 678 638 A1**
(43) Date of publication of application: **14.01.2026**
(21) Application number: 24769902.8
(22) Date of filing: 11.03.2024
(51) Int. Cl.: C07D 405/12, C07D 403/12, C07D 401/12, C07D 471/00, C07D 487/00, A61K 31/395, A61P 35/00

(54) **NEW E3 UBIQUITIN LIGASE LIGAND, PROTEIN DEGRADATION AGENT AND USE THEREOF**

(30) Priority: 10.03.2023 CN 202310232768
(71) Applicant: Gluetacs Therapeutics (Shanghai) Co., Ltd., Shanghai 201306 (CN)
(72) Inventor: YANG, Xiaobao, Shanghai 201306 (CN); SUN, Renhong, Shanghai 201306 (CN); LI, Yan, Shanghai 201306 (CN); ZHAO, Baoyin, Shanghai 201306 (CN)
(74) Representative: Dragsted Partners A/S
(86) International application number: PCT/CN2024/080926
(87) International publication number: WO 2024/188209

(57) **Abstract**

The present disclosure provides compounds of Formula (I) and (II) or salts, enantiomers, stereoisomers, solvates, isotopically enriched analogs, prodrugs, or polymorphs thereof, and uses thereof. The present disclosure also provides pharmaceutical compositions comprising, as an active ingredient, the compounds of Formula (I) or salts, enantiomers, stereoisomers, solvates, isotopically enriched analogs, prodrugs, or polymorphs thereof, and uses thereof.

PBM-LIN-ULM Formula (I)

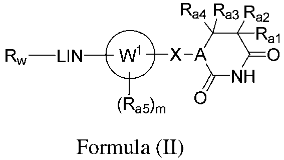

## Description

### Technical Field

The present disclosure relates to protein degrading compounds of Formula (I), cereblon (CRBN) E3 ubiquitin ligase ligand compounds of Formula (II), and use thereof. The protein degrading compounds of Formula (I) can effectively treat or prevent diseases or disorders associated with the degraded protein. The CRBN E3 ubiquitin ligase ligand compounds of Formula (II) are capable of binding to the CRBN E3 ligase and are useful in the preparation of the protein degrading compounds of Formula (I).

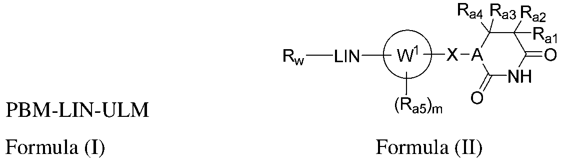

### Background

Bifunctional proteolysis-targeting drugs consist of three moieties, one end of which is a ligand warhead that can bind to a specific target protein, and the other end is a small molecule ligand of E3 ubiquitin ligase, both of which are connected together through linkers of different lengths and categories located in the middle. The bifunctional proteolysis-targeting small molecules can utilize the binding effects of the ligands at both ends to simultaneously bind to a specific target protein and E3 ubiquitin ligase, thereby recruiting E3 ubiquitin ligase to the vicinity of the specific target protein and enabling E3 ubiquitin ligase to ubiquitinate the target protein. The polyubiquitinated target protein will be recognized and degraded by proteasomes in the body. The biggest difference between the bifunctional proteolysis-targeting drugs and traditional small molecule-based inhibitor drugs is that the former mobilize the entire cell as a drug effector unit. This drug action mode only requires small molecule drugs to briefly bind to the target protein and tag it for degradation. Therefore, a low drug dose can meet the requirements, greatly reduce the risk of off-target effects, and potentially eliminate tumor progression caused by abnormal expression of driver genes and drug resistance caused by acquired mutations in driver genes.

Over 600 distinct E3 ubiquitin ligases are involved in the design of proteolysis-targeting drugs; however, only a few have been successfully utilized in preclinical research on degraders. Among them, Cereblon (CRBN) E3 ubiquitin ligase is one of the most widely used E3 ubiquitin ligases. The known ligands for CRBN-type E3 ubiquitin ligase (CRBN ligands) include thalidomide and its analogs pomalidomide and lenalidomide, all of which possess a phthalimide backbone. Currently, several bifunctional protein degraders in clinical stages-such as ARV-110 targeting AR, ARV-471 targeting ER, NX-2127 targeting BTK, and FHD-609 targeting BRD9-are designed using CRBN ligands of the immunomodulatory imide drug (IMiD) type containing the phthalimide scaffold. However, such ligands suffer from issues such as poor solubility and weak CRBN binding affinity. There is thus an urgent need to develop a new series of novel CRBN ligands with high CRBN binding affinity and favorable drug-like properties. These ligands can be used to design and synthesize corresponding bifunctional protein degraders targeting various proteins for the treatment and/or prevention of diseases mediated by or associated with the target protein(s).

Furthermore, upon binding to CRBN E3 ubiquitin ligase, IMiD-based CRBN ligands can themselves act as molecular glues, inducing CRBN to recognize novel substrate proteins, leading to their ubiquitination and degradation. This results in pharmacological effects such as antitumor and immunomodulatory activities. Molecular glue degraders directly target and eliminate specific proteins and offer potential advantages such as the ability to target "undruggable" proteins. Moreover, they typically exhibit low molecular weight and good drug-like properties, making them a major focus in drug development. In addition to the already marketed IMiD drugs, a range of molecular glue compounds based on the CRBN E3 ubiquitin ligase have been developed. Examples include CC-122, CC-220, CC-90009, CC-99282, and CC-92480 from Bristol Myers Squibb (BMS); DKY709 from Novartis; and CFT7455 from C4 Therapeutics, all of which are currently in clinical trials. The substrate spectrum of these molecular glues has also expanded from initially identified transcription factors IKZF1/3 to include casein kinase 1α (CK1α), zinc finger protein 91 (ZFP91), and translation termination factor GSPT1, among others. Degradation of these substrate proteins enables molecular glues to exert various pharmacological activities-such as immunomodulatory, anti-inflammatory, and antitumor effects-across different indications.

In summary, CRBN ligands can not only serve as components of bifunctional protein degraders to facilitate the effective degradation of specific disease-causing proteins, but can also function as molecular glue degraders inducing the degradation of diverse substrate proteins. Therefore, designing diverse molecular scaffolds and developing novel CRBN ligands with drug-like properties for application in bifunctional protein degrader development has become a major research focus in the field.

### Summary of Invention

In view of the above, an object of the present disclosure is to provide novel small molecule ligands for E3 ubiquitin ligases, bifunctional protein degraders designed based on such novel E3 ubiquitin ligase ligands and various target protein ligands, as well as their applications and usage methods. The E3 ligands provided herein exhibit stronger CRBN binding affinity and improved drug-like properties, and themselves demonstrate enhanced activity and/or selectivity as molecular glues for inducing substrate protein degradation. Furthermore, bifunctional protein degraders designed using the E3 ligands provided herein, targeting different proteins, show stronger degradation/inhibition effects against various classes or families of pathogenic proteins, favorable pharmacokinetic properties, and consequently elicit broad pharmacological activities.

To achieve the above objectives and other related purposes, in one aspect, the present disclosure provides novel CRBN E3 ubiquitin ligase ligands, which can function as molecular glues binding to the CRBN E3 ligase. The novel E3 ubiquitin ligase ligands of the present disclosure exhibit CRBN binding affinity, demonstrate antitumor activity, and can further be applied in the design of bifunctional protein degraders.

In some embodiments, the novel E3 ubiquitin ligase ligands of the present disclosure may be the compound of Formula (II): 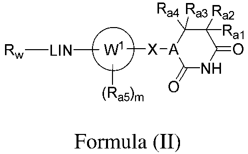 or salts, stereoisomers (including enantiomers), solvates, isotopically enriched analogs, prodrugs, or polymorphs thereof,
wherein A represents N or CH;
Rₐ₁, Rₐ₂, Rₐ₃, and Rₐ₄ are the same or different and independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
ring W¹ represents heteroarylene, and the ring W¹ is optionally substituted with m Rₐ₅, with each Rₐ₅ being the same or different and independently representing halogen, hydroxy, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl or C₂₋₆ alkynyl;
m represents an integer of 0, 1, 2, 3 or 4;
X represents a bond or -R_{b1}-N(Rₐ₆)-R_{b2}-, where Rₐ₆ represents hydrogen or C₁₋₆ alkyl, and R_{b1} and R_{b2}each independently represent optionally substituted C₀₋₂ alkylene;
LIN represents optionally substituted methylene, or optionally substituted linear or branched C₂₋₁₅ alkylene, wherein one or more groups selected from Rₐ, R_{b}, and any combination thereof are optionally inserted between one or more pairs of adjacent carbon atoms in the backbone carbon chain of the linear or branched C₂₋₁₅ alkylene group; wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and in case that two or more groups Rₐ are inserted into the backbone carbon chain of the linear or branched C₂₋₃₀ alkylene group, the two or more groups Rₐ are not directly connected to each other; and wherein each R_{b} is independently selected from the group consisting of optionally substituted cycloalkylene, optionally substituted arylene, optionally substituted heterocyclylene, optionally substituted heteroarylene, alkynylene, alkenylene, or any combination thereof; and
R_{w} represents hydrogen, deuterium, leaving group, halogen, hydroxy, COOH, NH₂, N₃, CHO, methanesulfonyloxy, trifluoromethanesulfonyloxy or p-toluenesulfonyloxy.

In another aspect, the bifunctional protein degraders designed based on the novel E3 ubiquitin ligase ligands and various target protein ligands may be the compound of Formula (I):

PBM-LIN-ULM Formula (I)

or salts, stereoisomers (including enantiomers), solvates, isotopically enriched analogs, prodrugs, or polymorphs thereof,
wherein PBM represents a protein-binding moiety capable of binding to a target protein, and is covalently linked to ULM through LIN;
ULM represents a ligand moiety capable of binding to an E3 ubiquitin ligase, and represents the structure of the following Formula (ULM): 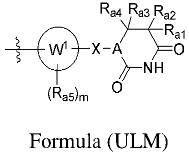
wherein A represents N or CH;
Rₐ₁, Rₐ₂, Rₐ₃, and Rₐ₄ are the same or different and independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
ring W¹ represents arylene or heteroarylene, wherein the ring W¹ is optionally substituted with m Rₐ₅, with each Rₐ₅ being the same or different and independently representing halogen, hydroxy, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, or C₂₋₆ alkynyl;
m represents an integer of 0, 1, 2, 3 or 4;
X represents a bond or -R_{b1}-N(Rₐ₆)-R_{b2}-, where Rₐ₆ represents hydrogen or C₁₋₆ alkyl, and R_{b1} and R_{b2} each independently represent optionally substituted C₀₋₂ alkylene;
LIN is a linker moiety, and represents a bond, optionally substituted methylene, or optionally substituted linear or branched C₂₋₁₅ alkylene, wherein one or more groups selected from Rₐ, R_{b}, and any combination thereof are optionally inserted between one or more pairs of adjacent carbon atoms in the backbone carbon chain of the linear or branched C₂₋₁₅ alkylene group; wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and in case that two or more groups Rₐ are inserted into the backbone carbon chain of the linear or branched C₂₋₃₀ alkylene group, the two or more groups Rₐ are not directly connected to each other; and wherein each R_{b} is independently selected from the group consisting of optionally substituted cycloalkylene, optionally substituted arylene, optionally substituted heterocyclylene, optionally substituted heteroarylene, alkynylene, alkenylene, or any combination thereof.

In a further aspect, the present disclosure provides a pharmaceutical composition comprising the compound of Formula (I) or salts, stereoisomers (including enantiomers), solvates, isotopically enriched analogs, prodrugs, or polymorphs thereof, and at least one pharmaceutically acceptable carrier.

In a further aspect, the present disclosure further provides a medicine kit or reagent kit comprising: the compound of Formula (I) or a pharmaceutically acceptable salt, or the pharmaceutical composition comprising the same.

In a further aspect, the present disclosure provides the compound of Formula (I) or salts, stereoisomers (including enantiomers), solvates, isotopically enriched analogs, prodrugs, or polymorphs thereof, or the pharmaceutical composition comprising the same as active ingredient for use as a medicament.

In a further aspect, the present disclosure provides the compound of Formula (I) or salts, stereoisomers (including enantiomers), solvates, isotopically enriched analogs, prodrugs, or polymorphs thereof, or the pharmaceutical composition comprising the same as active ingredient for use in the treatment and/or prevention of a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

In a further aspect, the present disclosure provides a method for treating or preventing a disease or disorder in a subject, comprising administering to the subject a therapeutically effective amount of the compound of Formula (I) or salts, enantiomers, stereoisomers, solvates, isotopically enriched analogs, prodrugs, or polymorphs thereof, or the pharmaceutical composition comprising the same, wherein the disease or disorder comprises tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

In another aspect, the present disclosure provides a use of the compound of Formula (II), or salts, stereoisomers (including enantiomers), solvates, isotopically enriched analogs, prodrugs, or polymorphs thereof for the manufacture of a Cereblon (CRBN) protein-binding agent.

In a further aspect, the present disclosure also provides a use of the compound of Formula (II) or salts, stereoisomers (including enantiomers), solvates, isotopically enriched analogs, prodrugs, or polymorphs thereof for the preparation of the compound of Formula (I) or salts, enantiomers, stereoisomers, solvates, isotopically enriched analogs, prodrugs, or polymorphs thereof of the present disclosure.

In a further aspect, the present disclosure also provides a method for preparing the compound of Formula (I) or salts, enantiomers, stereoisomers, solvates, isotopically enriched analogs, prodrugs, or polymorphs thereof of the present disclosure by using the compound of Formula (II) or salts, stereoisomers (including enantiomers), solvates, isotopically enriched analogs, prodrugs, or polymorphs thereof.

In a further aspect, the present disclosure provides the compound of Formula (II) or salts, stereoisomers (including enantiomers), solvates, isotopically enriched analogs, prodrugs, or polymorphs thereof, or the pharmaceutical composition comprising the same as active ingredient for use in the manufacture of a medicament for the prevention and/or treatment of a disease or disorder associated with a cereblon protein.

In a further aspect, the present disclosure provides a method for treating or preventing a disease or disorder associated with a cereblon protein in a subject, comprising administering to the subject a therapeutically effective amount of the compound of Formula (II) or salts, enantiomers, stereoisomers, solvates, isotopically enriched analogs, prodrugs, or polymorphs thereof, or the pharmaceutical composition comprising the same.

### Description of Drawings

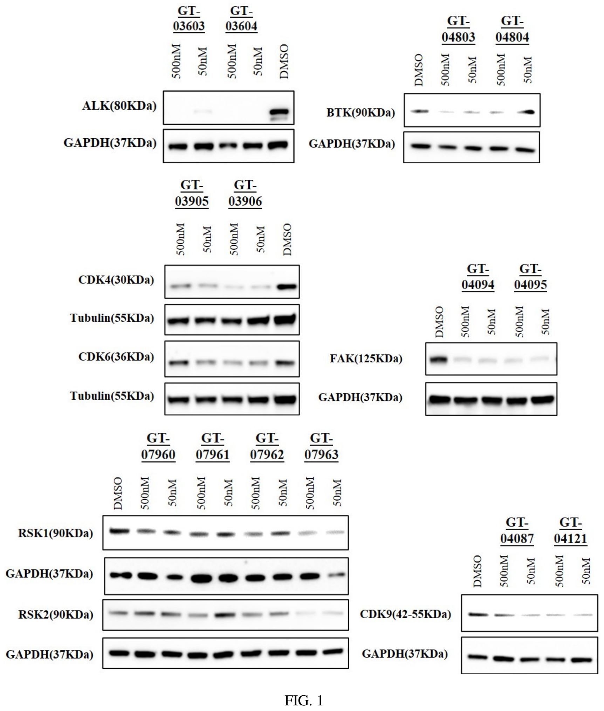

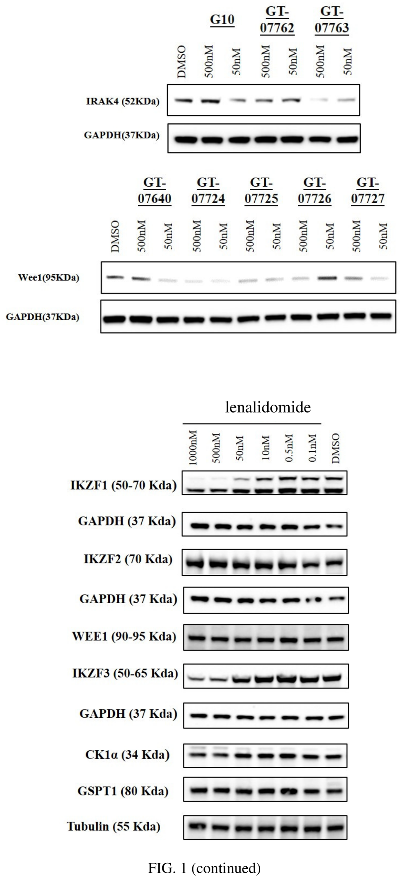

Figure 1 shows the Western blot experimental results of the degradation of the target substrate protein by the compounds of the present invention in cells.

### Detailed Description of the Invention

The following detailed description is provided as exemplary specific embodiments to assist those skilled in the art in understanding and practicing the present disclosure. It should be appreciated, however, that such description is not intended to limit the scope of the present disclosure, and that various modifications and changes may be made to the specific embodiments described in the present disclosure without departing from the spirit and scope of the present disclosure. Such changes and modifications are to be understood as being included within the scope of the present invention as defined by the appended claims.

### I. Compounds

### Compounds of Formula (I)

The compound of Formula (I) of the present disclosure, or salts (including pharmaceutically acceptable salts), stereoisomers (including enantiomers), solvates, isotopically enriched analogs, prodrugs, or polymorphs thereof, can bind to the target protein, recruit the target protein to an E3 ubiquitin ligase for ubiquitination, and subsequently lead to its degradation. The compound of Formula (I) of the present disclosure can specifically target particular classes or families of target proteins, and exhibit a broad spectrum of pharmacological activities by degrading/inhibiting diverse classes or families of target proteins.

PBM-LIN-ULM Formula (I)

wherein PBM, LIN, and ULM are as defined in the compounds of Formula (I) above.

In some embodiments, the PBM may be a small molecule ligand that binds to specific target proteins, including but not limited to: epidermal growth factor receptor (EGFR); Cyclin-dependent kinase 4/6 (CDK4/6); Cyclin-dependent kinase 2 (CDK2); anaplastic lymphoma kinase (ALK); activin receptor-like kinase 2 (ALK2); fibroblast growth factor receptors (FGFRs); proto-oncogene tyrosine-protein kinase receptor RET (RET); Focal adhesion kinase (FAK); breakpoint cluster region (BCR)-Abelson leukemia virus (ABL) (BCR-ABL) tyrosine kinase; Bruton tyrosine kinase (BTK); Androgen receptor (AR); Estrogen receptor (ER); Bromodomain and extra-terminal domain protein (BET); Interleukin-1 receptor-associated kinase 4 (IRAK4); Cyclin-dependent kinase 9 (CDK9); Histone-lysine N-methyltransferase EZH2 (EZH2); neurotrophic receptor tyrosine kinase (NTRK); Src homology 2 domain containing protein tyrosine phosphatase (SHP2); Poly (ADP-ribose) polymerase (PARP); signal transducers and activators of transcription 3 (STAT3); FMS-like tyrosine kinase 3 (FLT3); B cell lymphoma-2 (BCL-2) family proteins; SOS Ras/Rac guanine nucleotide exchange factor 1 (SOS1); GTPase Kras (KRAS); SWI/SNF related, matrix associated, actin dependent regulator of chromatin, subfamily a, member 2/4 (SMARCA2/4); or ribosomal protein S6 kinase (RSK).

In some embodiments, PBM comprises the structures of the following formula: 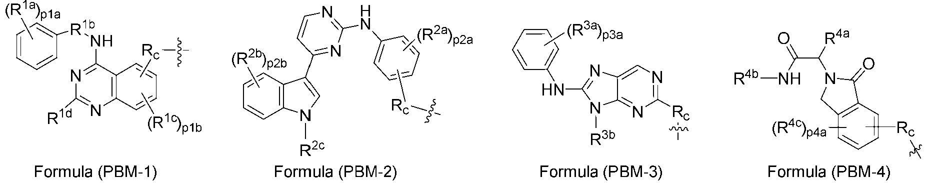 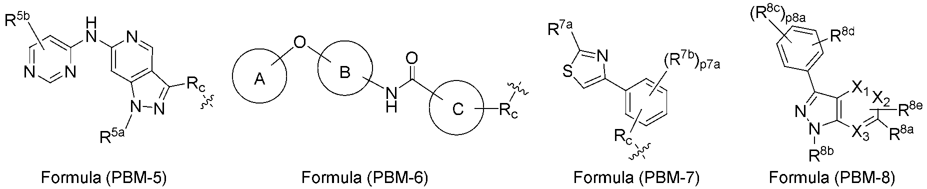 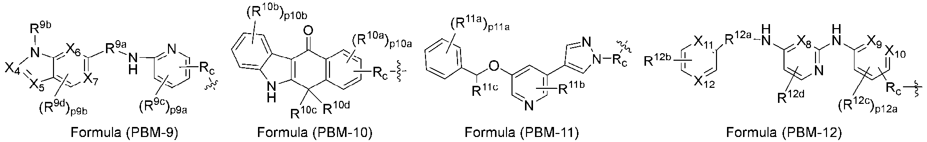 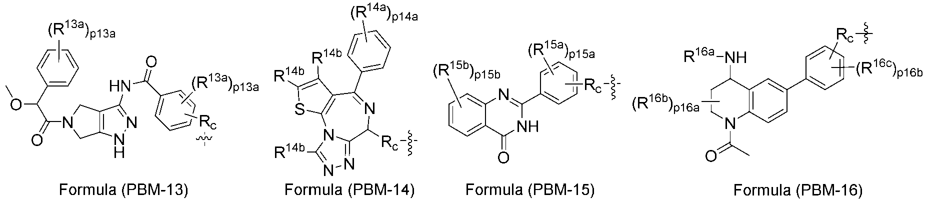 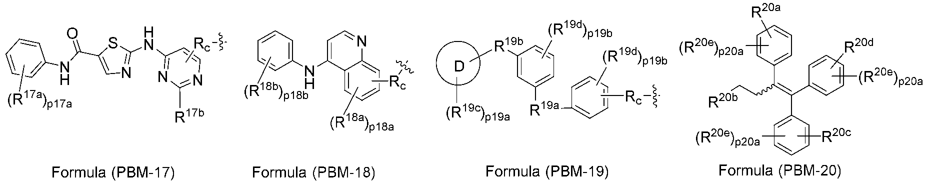 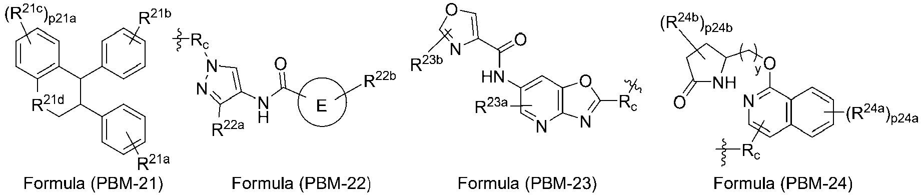 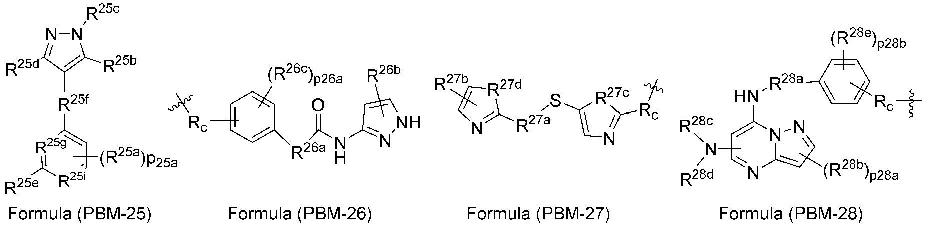 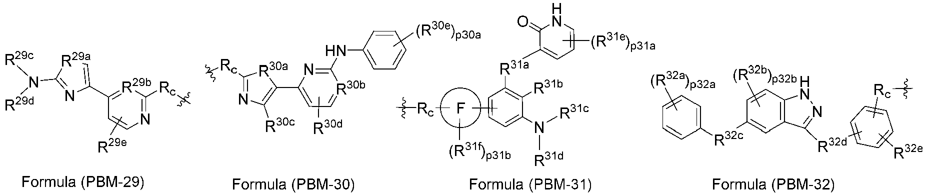 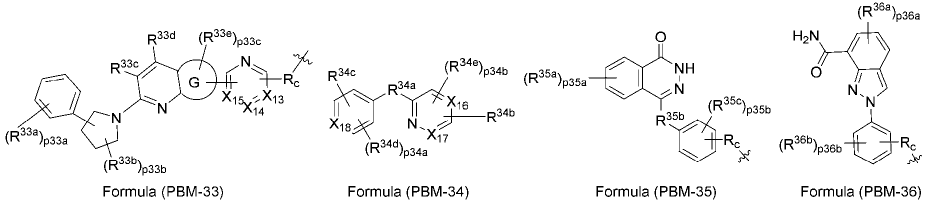 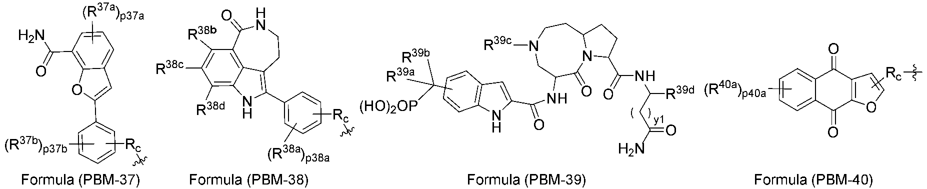 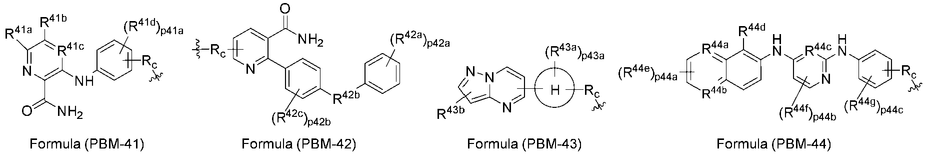 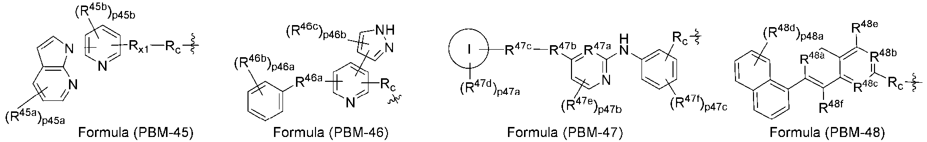 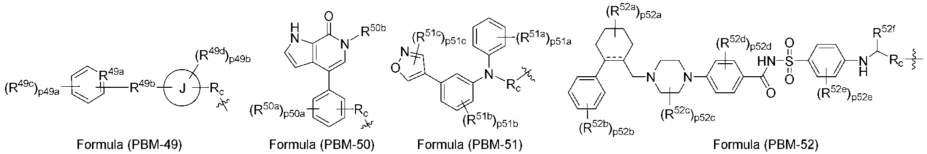 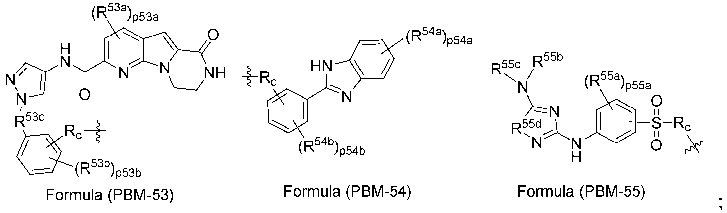
wherein (R^{1a})ₚ₁ₐ in Formula (PBM-1) indicates that the benzene ring to which it is attached is substituted with p1a R^{1a} groups, and each R^{1a} is the same or different and each independently represents deuterium, halogen, hydroxy, NH₂, NO₂, cyano, C₁₋₆ alkyl, halogenated C₁₋₆ alkyl, deuterated C₁₋₆ alkyl, C₁₋₆ alkoxy, or halogenated C₁₋₆ alkoxy;
p1a represents an integer of 1, 2, 3, 4 or 5;
R^{1b} represents a bond, or C₁₋₂ alkylene optionally substituted with a substituent selected from the group consisting of C₁₋₃ alkyl, deuterated C₁₋₃ alkyl, halogenated C₁₋₃ alkyl, and halogen;
(R^{1c})_{p1b} indicates that the quinazoline ring in Formula (PBM-1) is substituted with p1b R^{1c}, with each R^{1c} being the same or different and each independently representing deuterium, halogen, NO₂, cyano, halogenated C₁₋₆ alkyl, C₁₋₁₀ alkyl, deuterated C₁₋₁₀ alkyl, C₁₋₁₀ alkoxy, halogenated C₁₋₁₀ alkoxy, -O-optionally substituted heterocyclyl, or -NHC(O)R^{1c}, where R^{1c} represents C₁₋₁₀ alkyl, deuterated C₁₋₁₀ alkyl or C₂₋₁₀ alkenyl, e.g., R^{1c} represents methyl, methoxy, 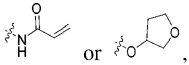 such as 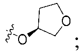
p1b represents an integer of 1, 2, or 3;
R^{1d} represents hydrogen, deuterium or C₁₋₃ alkyl;
(R^{2a})ₚ₂ₐ in Formula (PBM-2) indicates that the benzene ring to which it is attached is substituted with p2a R^{2a}, with each R^{2a} being the same or different and each independently representing deuterium, halogen, nitro, cyano, halogenated C₁₋₆ alkyl, C₁₋₁₀ alkyl, deuterated C₁₋₁₀ alkyl, C₁₋₁₀ alkoxy or -NHC(O)R^{2d}, where R^{2d} represents C₁₋₁₀ alkyl, deuterated C₁₋₁₀ alkyl or C₂₋₁₀ alkenyl, e.g., R^{2a} represents methyl, methoxy, nitro or 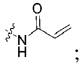
p2a represents an integer of 1, 2, 3 or 4;
(R^{2b})_{p2b} indicates that the 1H-indole ring in Formula (PBM-2) is substituted with p2b R^{2b}, where p2b represents an integer of 0, 1, 2 or 3, and each R^{2b} is identical or different and independently represents deuterium, deuterated C₁₋₁₀ alkyl, C₁₋₁₀ alkyl, halogen or halogenated C₁₋₁₀ alkyl;
R^{2c} represents hydrogen, C₁₋₁₀ alkyl, deuterated C₁₋₁₀ alkyl, halogen or halogenated C₁₋₁₀ alkyl;
(R^{3a})ₚ₃ₐ in Formula (PBM-3) indicates that the benzene ring to which it is attached is substituted with p3a R^{3a}, with each R^{3a} being the same or different and each independently representing deuterium, halogen, hydroxy, C₁₋₆ alkyl, deuterated C₁₋₆ alkyl, halogenated C₁₋₆ alkyl, C₁₋₆ alkoxy, halogenated C₁₋₆ alkoxy, NO₂, NH₂ or cyano;
p3a represents an integer of 0, 1, 2, 3, 4 or 5;
R^{3b} represents substituted or unsubstituted C₃₋₁₅ cycloalkyl, e.g., cyclopentyl;
R^{4a} in Formula (PBM-4) represents C₆₋₁₀ aryl or 5- to 20-membered heteroaryl, where the C₆₋₁₀ aryl and 5- to 20-membered heteroaryl are each independently optionally substituted with one or more (e.g., 1-10, 1-8, 2-6, 1-5, 1-4, 1-3 or 2-5) substituents, wherein each substituent is independently selected from the group consisting of deuterium, halogen, hydroxy, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy or any combination thereof;
R^{4b} represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), wherein the C₆₋₁₀ aryl and the 5- to 20-membered heteroaryl are each independently optionally substituted with one or more (e.g., 1-10, 1-8, 2-6, 1-5, 1-4, 1-3 or 2-5) R^{4d}, with each R^{4d} being the same or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
(R^{4c})ₚ₄ₐ indicates that the isoindoline ring in Formula (PBM-4) is optionally substituted with p4a R^{4c}, with each R^{4c} being the same or different and each independently representing deuterium, halogen, hydroxy, C₁₋₆ alkyl, halogenated C₁₋₆ alkyl, deuterated C₁₋₆ alkyl, NO₂, NH₂, C₁₋₆ alkoxy, halogenated C₁₋₆ alkoxy, or cyano;
p4a represents an integer of 0, 1, 2 or 3;
R^{5a} in Formula (PBM-5) represents hydrogen, C₁₋₁₀ alkyl (e.g., 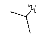 ), deuterated C₁₋₁₀ alkyl or halogenated C₁₋₁₀ alkyl;
R^{5b} represents 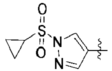 or 4- to 12-membered nitrogen-containing heterocyclyl, wherein the 4- to 12-membered nitrogen-containing heterocyclyl is optionally substituted with one or more (e.g., 1-10, 1-8, 2-6, 1-5, 1-4, 1-3 or 2-5) substituents, wherein each substituent is independently selected from the group consisting of deuterium, halogen, hydroxy, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, NO₂, NH₂, cyano or any combination thereof;
ring A in Formula (PBM-6) represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), and the ring A is optionally substituted with one or more (e.g., 1-10, 1-8, 2-6, 1-5, 1-4, 1-3 or 2-5) R^{6a}, with each R^{6a} being the same or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkenyl or C₂₋₆ alkynyl;
ring B in Formula (PBM-6) represents C₃₋₁₅ cycloalkyl or 4- to 20-membered heterocyclyl (e.g., 4- to 20-membered heterocyclyl containing 1 to 4 heteroatoms selected from N, O and S), wherein the ring B is optionally substituted with one or more (e.g., 1-10, 1-8, 2-6, 1-5, 1-4, 1-3 or 2-5) R^{6b}, with each R^{6b} being the same or different and each independently representing deuterium, halogen, C₁₋₆ alkyl, halogenated C₁₋₆ alkyl, deuterated C₁₋₆ alkyl, C₁₋₆ alkoxy, or halogenated C₁₋₆ alkoxy;
ring C in Formula (PBM-6) represents 5- to 20-membered heteroaryl (e.g., 5- to 20-membered monocyclic or bicyclic heteroaryl containing 1 to 4 heteroatoms selected from N, O and S), wherein the ring C is optionally substituted with one or more (e.g., 1-10, 1-8, 2-6, 1-5, 1-4, 1-3 or 2-5) R^{6c}, with each R^{6c} being the same or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
R^{7a} in Formula (PBM-7) represents 4- to 20-membered heterocyclyl (e.g., 4- to 20-membered heterocyclyl containing 1 to 4 heteroatoms selected from N, O and S), wherein the 4- to 20-membered heterocyclyl is optionally substituted with one or more (e.g., 1-10, 1-8, 2-6, 1-5, 1-4, 1-3 or 2-5) R^{7c}, with each R^{7c} being the same or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
(R^{7b})ₚ₇ₐ in Formula (PBM-7) indicates that the benzene ring to which it is attached is substituted with p7a R^{7b}, with each R^{7b} being the same or different and independently representing deuterium, halogen, C₁₋₆ alkyl, halogenated C₁₋₆ alkyl, or deuterated C₁₋₆ alkyl, and p7a represents an integer of 1, 2, 3 or 4;
X₁ in Formula (PBM-8) represents N or CR^{8f}, where R^{8f} represents hydrogen, deuterium or amino, and X₂ represents N or CR^{8g}, where R^{8g} represents hydrogen or a bond, and X₃ represents CH or N;
R^{8a} and R^{8b} each independently represent hydrogen or a bond;
(R^{8c})ₚ₈ₐ in Formula (PBM-8) indicates that the benzene ring to which it is attached is substituted with p8a R^{8c}, with each R^{8c} being the same or different and independently representing -O-aryl, -O-heteroaryl, 4- to 20-membered heterocyclyl, -C₁₋₃ alkylene-NHC(O)-heteroaryl, -C₁₋₃ alkylene-C(O)NH-heteroaryl, C₁₋₆ alkyl, deuterium, deuterated C₁₋₆ alkyl, halogen, or halogenated C₁₋₆ alkyl, wherein the 4- to 20-membered heterocyclyl, aryl, and heteroaryl are each independently optionally substituted with one or more (e.g., 1-10, 1-8, 2-6, 1-5, 1-4, 1-3 or 2-5) R^{8h}, with each R^{8h} being the same or different and each independently representing deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p8a represents an integer of 1, 2, 3 or 4;
R^{8d} represents a bond, hydrogen, halogen, C₁₋₆ alkyl, halogenated C₁₋₆ alkyl, deuterated C₁₋₆ alkyl, halogenated C₁₋₆ alkoxy, or C₁₋₆ alkoxy;
R^{8c} represents hydrogen, halogen, cyano, C₁₋₆ alkyl, halogenated C₁₋₆ alkyl, or amino;
wherein R^{8g}, R^{8a}, R^{8b} and R^{8d} are not simultaneously hydrogen, and only one of R^{8g}, R^{8a}, R^{8b} and R^{8d} represents a bond, wherein
   when R^{8a} represents a bond, the ring carbon atom connected to R^{8a} is directly bonded to R_{c}, X₂ represents CH or N, and R^{8b} is hydrogen, and R^{8d} is not a bond;
   when X₂ represents CR^{8g}, where R^{8g} represents a bond, the ring carbon atom connected to R^{8g} is directly bonded to R_{c}, and R^{8a} and R^{8b} are hydrogen, and R^{8d} is not a bond;
   when R^{8b} represents a bond, the ring nitrogen atom connected to R^{8b} is directly bonded to R_{c}, X₂ represents N or CH, and R^{8a} is hydrogen, R^{8d} is not a bond; and
   when R^{8d} represents a bond, the ring carbon atom connected to R^{8d} is directly bonded to R_{c}, and X₂ represents N or CH, and R^{8a} and R^{8b} are hydrogen;
X₄ in Formula (PBM-9) represents CR^{9c} or a fragment 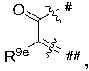 wherein symbol # indicates the point of attachment to N atom adjacent to X₄, symbol ## indicates the point of attachment to X₅, and each R^{9c} independently represents deuterium, hydrogen, halogen, C₁₋₆ alkyl, halogenated C₁₋₆ alkyl, deuterated C₁₋₆ alkyl, -C(O)-C₁₋₆ alkyl, -C(O)-deuterated C₁₋₆ alkyl or -C(O)NR^{9f}R^{9g}, where R^{9f} and R^{9g} are the same or different and each independently represent hydrogen, C₁₋₆ alkyl, or deuterated C₁₋₆ alkyl;
X₅ in Formula (PBM-9) represents N or CR^{9h}, where R^{9h} represents hydrogen, deuterium, halogen, C₁₋₆ alkyl, deuterated C₁₋₆ alkyl or halogenated C₁₋₆ alkyl;
X₆ and X₇ each independently represent CH or N;
R^{9a} represents a bond or optionally substituted heteroarylene (e.g., heteroarylene substituted with halogen or deuterium);
R^{9b} represents hydrogen, C₁₋₆ alkyl, halogenated C₁₋₆ alkyl, deuterated C₁₋₆ alkyl, or optionally substituted C₃₋₁₂ cycloalkyl;
(R^{9c})ₚ₉ₐ in Formula (PBM-9) indicates that the pyridine ring to which it is attached is optionally substituted with p9a R^{9c}, with each R^{9c} being the same or different and each independently representing deuterium, halogen, C₁₋₆ alkyl, C₁₋₆ alkoxy, halogenated C₁₋₆ alkoxy, deuterated C₁₋₆ alkyl or halogenated C₁₋₆ alkyl, and p9a represents an integer of 0, 1, 2 or 3;
(R^{9d})_{p9b} in Formula (PBM-9) indicates that the nitrogen-containing bicyclic heteroaryl ring to which it is attached is optionally substituted with p9b R^{9d}, with each R^{9d} being the same or different and each independently representing deuterium, halogen, C₁₋₆ alkyl, C₁₋₆ alkoxy, halogenated C₁₋₆ alkoxy, deuterated C₁₋₆ alkyl or halogenated C₁₋₆ alkyl, and p9b represents an integer of 0 or 1;
(R^{10a})ₚ₁₀ₐ in Formula (PBM-10) indicates that the benzene ring to which it is attached is optionally substituted with p10a R^{10a}, with each R^{10a} being the same or different and each independently representing deuterium, cyano, halogen, C₁₋₆ alkyl, C₁₋₆ alkoxy, halogenated C₁₋₆ alkoxy, deuterated C₁₋₆ alkyl or halogenated C₁₋₆ alkyl, and p10a represents an integer of 0, 1, 2 or 3;
(R^{10b})_{p10b} indicates that the benzene ring to which it is attached is optionally substituted with p10b R^{10b}, with each R^{10b} being the same or different and each independently representing deuterium, cyano, halogen, C₁₋₆ alkyl, C₁₋₆ alkoxy, halogenated C₁₋₆ alkoxy, deuterated C₁₋₆ alkyl or halogenated C₁₋₆ alkyl, and p10b represents an integer of 0, 1, 2, 3, or 4;
R^{10c} and R^{10d} are the same or different and each independently represent deuterium, C₁₋₆ alkyl, halogen, halogenated C₁₋₆ alkyl, deuterated C₁₋₆ alkyl, C₁₋₆ alkoxy, or halogenated C₁₋₆ alkoxy;
(R^{11a})ₚ₁₁ₐ in Formula (PBM-11) indicates that the benzene ring to which it is attached is substituted with p11a R^{11a}, with each R^{11a} being the same or different and each independently representing deuterium, halogen, hydroxy, amino, cyano, C₁₋₆ alkyl, C₁₋₆ alkoxy, halogenated C₁₋₆ alkoxy, halogenated C₁₋₆ alkyl, or deuterated C₁₋₆ alkyl;
p11a represents an integer of 1, 2, 3, 4 or 5;
R^{11b} represents NR^{11d}R^{11c}, where R^{11d} and R^{11c} are the same or different and each independently represent hydrogen, C₁₋₆ alkyl, halogenated C₁₋₆ alkyl, or deuterated C₁₋₆ alkyl;
R^{11c} represents deuterium, hydrogen, halogen, hydroxy, amino, cyano, C₁₋₆ alkyl, C₁₋₆ alkoxy, halogenated C₁₋₆ alkoxy, halogenated C₁₋₆ alkyl, or deuterated C₁₋₆ alkyl;
X₈, X₉, X₁₀, X₁₁ and X₁₂ in Formula (PBM-12) are the same or different and each independently represent N or CH;
R^{12a} represents a bond or optionally substituted methylene (e.g., halogenated methylene);
R^{12b} represents -C(O)N(R^{12f})R^{12e}, -N(R^{12f})C(O)R^{12e}, -S(O)₂N(R^{12f})R^{12e}, -N(R^{12f})S(O)₂R^{12e}, -S(O)₂R^{12e} or - P(O)(R^{12e})₂, wherein each R^{12e} is the same or different and each independently represents C₁₋₆ alkyl or deuterated C₁₋₆ alkyl, and R^{12f} represents hydrogen or C₁₋₆ alkyl;
(R^{12c})ₚ₁₂ₐ in Formula (PBM-12) indicates that the 6-membered ring containing X₉ and X₁₀ to which it is attached is optionally substituted with p12a R^{12c}, with each R^{12c} being the same or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p12a represents an integer of 0, 1, 2, 3, or 4;
R^{12d} represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
each (R^{13a})ₚ₁₃ₐ in Formula (PBM-13) independently indicates that the benzene ring to which it is attached is optionally substituted with p13a R^{13a}, with each R^{13a} being the same or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
each p13a is the same or different and each independently represents an integer of 0, 1, 2, 3, or 4;
(R^{14a})ₚ₁₄ₐ in Formula (PBM-14) indicates that the benzene ring to which it is attached is substituted with p14a R^{14a}, wherein p14a represents an integer of 0, 1, 2, 3, 4 or 5, and each R^{14a} is the same or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
each R^{14b} is the same or different and each independently represents halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
(R^{15a})ₚ₁₅ₐ in Formula (PBM-15) indicates that the benzene ring to which it is attached is optionally substituted with p15a R^{15a}, with each R^{15a} being the same or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p15a represents an integer of 0, 1, 2, 3, or 4 ;
(R^{15b})_{p15b} indicates that the 4-oxo-3,4-dihydroquinazoline ring to which it is attached is substituted with p15b R^{15b}, with each R^{15b} being the same or different and each independently representing halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p15b represents an integer of 1, 2, 3 or 4 ;
R^{16a} in Formula (PBM-16) represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), wherein the C₆₋₁₀ aryl and 5- to 20-membered heteroaryl are each independently optionally substituted with one or more (e.g., 1-10, 1-8, 2-6, 1-5, 1-4, 1-3 or 2-5) R^{16d}, wherein each R^{16d} is identical or different and independently represents deuterium, halogen, hydroxy, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkenyl or C₂₋₆ alkynyl;
(R^{16b})ₚ₁₆ₐ in Formula (PBM-16) indicates that 1,2,3,4-tetrahydronaphthalene ring to which it is attached is substituted with p16a R^{16b}, with each R^{16b} being the same or different and each independently representing deuterium, halogen, C₁₋₆ alkoxy, halogenated C₁₋₆ alkoxy, C₁₋₆ alkyl, deuterated C₁₋₆ alkyl, or halogenated C₁₋₆ alkyl;
p16a represents an integer of 0, 1, 2, 3, or 4 ;
(R^{16c})_{p16b} in Formula (PBM-16) indicates that the benzene ring to which it is attached is substituted with p16b R^{16c}, with each R^{16c} being the same or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p16b represents an integer of 0, 1, 2, 3, or 4 ;
(R^{17a})ₚ₁₇ₐ in Formula (PBM-17) indicates that the benzene ring to which it is attached is substituted with p17a R^{17a}, with each R^{17a} being the same or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p17a represents an integer of 1, 2, 3, 4 or 5 ;
R^{17b} represents halogen, C₁₋₆ alkyl, deuterated C₁₋₆ alkyl, or halogenated C₁₋₆ alkyl;
(R^{18a})ₚ₁₈ₐ in Formula (PBM-18) indicates that the quinazoline ring to which it is attached is substituted with p18a R^{18a}, with each R^{18a} being the same or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p18a represents an integer of 1, 2, 3 or 4 ;
(R^{18b})_{p18b} in Formula (PBM-18) indicates that the benzene ring to which it is attached is substituted with p18b R^{18b}, with each R^{18b} being the same or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p18b represents an integer of 1, 2, 3, 4 or 5 ;
R^{19a} in Formula (PBM-19) represents -NHC(O)- or -C(O)NH-;
R^{19b} represents -NH-, -N(C₁₋₆ alkyl)- or ethynylene;
ring D in Formula (PBM-19) represents a 5- to 20-membered monocyclic, bicyclic, or polycyclic heteroaryl group containing 1 to 4 heteroatoms selected from N, O, and S, and (R^{19c})ₚ₁₉ₐ indicates that the ring D is optionally substituted with p19a R^{19c}, with each R^{19c} being the same or different and each independently representing optionally substituted 5- to 15-membered heteroaryl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p19a represents an integer of 0, 1, 2, or 3;
each (R^{19d})_{p19b} in Formula (PBM-19) independently indicates that the benzene ring to which it is attached is independently optionally substituted with p19b R^{19d}, with each R^{19d} being the same or different and each independently representing deuterium, halogen, hydroxy, cyano, amino, C₁₋₆ alkoxy, halogenated C₁₋₆ alkoxy, C₁₋₆ alkyl, deuterated C₁₋₆ alkyl, or halogenated C₁₋₆ alkyl;
each p19b is the same or different and each independently represents an integer of 0, 1, 2, 3, or 4 ;
R^{20a}, R^{20b}, R^{20c} and R^{20d} in Formula (PBM-20) each independently represent a bond, hydrogen, deuterium, halogen, hydroxy, cyano, amino, C₁₋₆ alkoxy, halogenated C₁₋₆ alkoxy, C₁₋₆ alkyl, deuterated C₁₋₆ alkyl, or halogenated C₁₋₆ alkyl, wherein only one of R^{20a}, R^{20b}, R^{20c} and R^{20d} represents a bond, and the remaining three are the same or different and each independently represents hydrogen, halogen, or hydroxy, wherein when one of R^{20a}, R^{20b}, R^{20c} and R^{20d} represents a bond, the benzene ring or methylene group in Formula (PBM-20) to which it is attached is directly bonded to R_{c};
symbol " 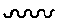 " attached to the double bond in Formula (PBM-20) represents a bond that may be in stereo-configuration (cis or trans configuration, or E- or Z-configuration);
each (R^{20c})ₚ₂₀ₐ in Formula (PBM-20) independently indicates that the benzene ring to which it is attached is independently optionally substituted with p20a R^{20e}, with each R^{20e} being the same or different and each independently representing deuterium, halogen, hydroxy, cyano, amino, C₁₋₆ alkoxy, halogenated C₁₋₆ alkoxy, C₁₋₆ alkyl, deuterated C₁₋₆ alkyl, or halogenated C₁₋₆ alkyl;
each p20a is the same or different and each independently represents an integer of 0, 1, 2, 3, or 4;
R^{21d} in Formula (PBM-21) represents O, S or CH₂;
(R^{21c})ₚ₂₁ₐ represents p21a R^{21c} groups, with each R^{21c} being the same or different and each independently representing deuterium, halogen, hydroxy, cyano, amino, C₁₋₆ alkoxy, halogenated C₁₋₆ alkoxy, C₁₋₆ alkyl, deuterated C₁₋₆ alkyl, or halogenated C₁₋₆ alkyl;
p21a represents an integer of 0, 1, 2, 3, or 4 ;
only one of R^{21a} and R^{21b} represents a bond, and the other represents hydrogen, halogen, hydroxy, cyano, amino, C₁₋₆ alkoxy, halogenated C₁₋₆ alkoxy, C₁₋₆ alkyl, deuterated C₁₋₆ alkyl, or halogenated C₁₋₆ alkyl, wherein when one of R^{21a} and R^{21b} represents a bond, the benzene ring carbon atom in Formula (PBM-21) to which it is attached is directly bonded to R_{c} ;
ring E in Formula (PBM-22) represents a 5- to 20-membered monocyclic, bicyclic, or polycyclic heteroarylene group containing 1 to 4 heteroatoms selected from N, O, and S;
R^{22b} represents optionally substituted 5- to 15-membered heteroaryl, optionally substituted 5- to 15-membered heterocyclyl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R^{22a} represents hydrogen, halogen, C₁₋₆ alkyl, halogenated C₁₋₆ alkyl, deuterated C₁₋₆ alkyl, C₁₋₆ alkoxy, or halogenated C₁₋₆ alkoxy;
R^{23a} in Formula (PBM-23) represents optionally substituted 5- to 15-membered heterocyclyl, optionally substituted 5- to 15-membered heteroaryl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R^{23b} represents a 5- to 20-membered monocyclic, bicyclic, or polycyclic heteroaryl group containing 1 to 4 heteroatoms selected from N, O, and S, and the heteroaryl is optionally substituted with one or more (e.g., 1-10, 1-8, 2-6, 1-5, 1-4, 1-3 or 2-5) substituents selected from the group consisting of deuterium, halogen, hydroxy, cyano, amino, C₁₋₆ alkyl, halogenated C₁₋₆ alkyl, deuterated C₁₋₆ alkyl, C₁₋₆ alkoxy, halogenated C₁₋₆ alkoxy or any combination thereof;
(R^{24a})ₚ₂₄ₐ indicates that the isoquinoline ring in Formula (PBM-24) is optionally substituted with p24a R^{24a}, with each R^{24a} being the same or different and each independently representing deuterium, halogen, hydroxy, cyano, amino, C₁₋₆ alkyl, C₁₋₆ alkoxy, halogenated C₁₋₆ alkoxy, -C(O)NH₂, deuterated C₁₋₆ alkyl or halogenated C₁₋₆ alkyl;
p24a represents an integer of 0, 1, 2, 3, or 4 ;
(R^{24b})_{p24b} indicates that the pyrrolidinone ring in Formula (PBM-24) is optionally substituted with p24b R^{24b}, with each R^{24b} being the same or different and each independently representing deuterium, halogen, hydroxy, cyano, amino, C₁₋₆ alkyl, C₁₋₆ alkoxy, halogenated C₁₋₆ alkoxy, -C(O)NH₂, deuterated C₁₋₆ alkyl or halogenated C₁₋₆ alkyl;
p24b represents an integer of 0, 1, 2, 3, or 4 ;
y in Formula (PBM-24) represents an integer of 1, 2 or 3;
R^{25g} in Formula (PBM-25) represents N or CR^{25h}, where R^{25h} represents hydrogen or halogen, R²⁵ⁱ represents N or CR^{25j}, where R^{25j} represents hydrogen or halogen;
R^{25f} represents a bond, -C(O)NH-, -NHC(O)- , -S(O)₂NH- or -NHS(O)₂-;
(R^{25a})ₚ₂₅ₐ indicates that the 6-membered ring containing R^{25g} and R²⁵ⁱ in Formula (PBM-25) to which it is attached is optionally substituted with p25a R^{25a}, with each R^{25a} being the same or different and each independently representing deuterium, halogen, hydroxy, cyano, amino, C₁₋₆ alkyl, C₁₋₆ alkoxy, halogenated C₁₋₆ alkoxy, deuterated C₁₋₆ alkyl, or halogenated C₁₋₆ alkyl;
p25a represents an integer of 0, 1 or 2 ;
R^{25b} represents hydrogen, halogen, C₁₋₆ alkyl, deuterated C₁₋₆ alkyl, halogenated C₁₋₆ alkyl or -C₁₋₃ alkylene-C₃₋₆ cycloalkyl;
R^{25c} represents hydrogen, C₁₋₆ alkyl, deuterated C₁₋₆ alkyl or halogenated C₁₋₆ alkyl; or
R^{25b} and R^{25c} together with their respective attached nitrogen and carbon atoms form an optionally substituted 5- to 7-membered nitrogen-containing heterocycle;
R^{25d} represents hydrogen or a bond, R^{25c} represents hydrogen or a bond, wherein R^{25d} and R^{25c} are not simultaneously hydrogen, and only one of R^{25d} and R^{25e} represents a bond, and the other represents hydrogen, wherein when R^{25d} represents a bond, the ring carbon atom connected to R^{25d} is directly connected to R_{c} , and when R^{25c} represents a bond, the ring carbon atom connected to R^{25c} is directly connected to R_{c} ;
R^{26a} in Formula (PBM-26) represents a bond, C₁₋₃ alkylene or halogenated C₁₋₃ alkylene;
R^{26b} represents halogen, optionally substituted C₁₋₆ alkyl, C₁₋₆ alkoxy, halogenated C₁₋₆ alkoxy, deuterated C₁₋₆ alkyl, halogenated C₁₋₆ alkyl, optionally substituted C₃₋₆ cycloalkyl, or optionally substituted C₁₋₆ alkyl (e.g., C₁₋₆ alkyl which is optionally substituted with an optionally substituted aryl);
(R^{26e})ₚ₂₆ₐ indicates that the benzene ring in Formula (PBM-26) is optionally substituted with p26a R^{26c}, with each R^{26c} being the same or different and each independently representing deuterium, halogen, hydroxy, cyano, amino, C₁₋₆ alkyl, C₁₋₆ alkoxy, halogenated C₁₋₆ alkoxy, deuterated C₁₋₆ alkyl, or halogenated C₁₋₆ alkyl;
p26a represents an integer of 0, 1, 2 , 3 or 4;
R^{27a} in Formula (PBM-27) represents C₁₋₃ alkylene or halogenated C₁₋₃ alkylene;
R^{27b} represents deuterium, C₁₋₆ alkyl, deuterated C₁₋₆ alkyl, halogenated C₁₋₆ alkyl, or optionally substituted C₃₋₆ cycloalkyl;
R^{27c} and R^{27d} each independently represent S or O;
R^{28a} in Formula (PBM-28) represents C₁₋₃ alkylene or halogenated C₁₋₃ alkylene;
(R^{28b})ₚ₂₈ₐ indicates that the pyrazolo[1,5-a]pyrimidinyl ring to which it is attached is optionally substituted with p28a R^{28b}, with each R^{28b} being the same or different and each independently representing hydroxy, halogen, C₁₋₆ alkyl, C₁₋₆ alkoxy, halogenated C₁₋₆ alkoxy, deuterated C₁₋₆ alkyl, or halogenated C₁₋₆ alkyl;
p28a represents an integer of 0, 1, 2 or 3;
R^{28c} and R^{28d} are the same or different and each independently represent hydrogen, optionally substituted C₁₋₁₀ alkyl or optionally substituted C₃₋₆ cycloalkyl;
(R^{28e})_{p28b} indicates that the benzene ring to which it is attached is optionally substituted with p28b R^{28e} , with each R^{28e} being the same or different and each independently representing halogen, C₁₋₆ alkyl, C₁₋₆ alkoxy, halogenated C₁₋₆ alkoxy, deuterated C₁₋₆ alkyl, or halogenated C₁₋₆ alkyl;
p28b represents an integer of 0, 1, 2 , 3 or 4;
R^{29a} in Formula (PBM-29) represents -S- or a fragment 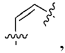 wherein when R^{29a} represents -S-, heteroaryl containing R^{29a} and nitrogen atom in Formula (PBM-29) is thiazolyl, and when R^{29a} represents the fragment 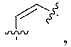 the heteroaryl containing R^{29a} and nitrogen atom in Formula (PBM-29) is pyridyl;
R^{29b} represents N or CH;
R^{29c} represents hydrogen oroptionally substituted C₁₋₁₀ alkyl;
R^{29d} represents hydrogen, -C₁₋₃ alkylene-optionally substituted 4- to 10-membered heterocyclyl, or optionally substituted 4- to 10-membered heterocyclyl;
R^{29e} represents hydroxy, halogen, C₁₋₆ alkyl, C₁₋₆ alkoxy, halogenated C₁₋₆ alkoxy, deuterated C₁₋₆ alkyl, or halogenated C₁₋₆ alkyl;
R^{30a} in Formula (PBM-30) represents O or S;
R^{30b} represents N or CH;
R^{30c} and R^{30d} are the same or different and each independently represent hydroxy, halogen, C₁₋₆ alkyl, C₁₋₆ alkoxy, halogenated C₁₋₆ alkoxy, deuterated C₁₋₆ alkyl, or halogenated C₁₋₆ alkyl;
(R^{30e})ₚ₃₀ₐ indicates that the benzene ring to which it is attached is optionally substituted with p30a R^{30e}, with each R^{30e} being the same or different and each independently representing deuterium, hydroxy, halogen, amino, cyano, C₁₋₆ alkoxy, halogenated C₁₋₆ alkoxy, C₁₋₆ alkyl, deuterated C₁₋₆ alkyl, halogenated C₁₋₆ alkyl, - S(O)₂NH₂ or -C(O)NH₂;
p30a represents an integer of 0, 1, 2 , 3, 4 or 5;
R^{31a} in Formula (PBM-31) represents C(O)NH, NHC(O), (CH₂)₁₋₂C(O)NH, (CH₂)₁₋₂NHC(O), C(O)NH(CH₂)₁₋₂, NHC(O)(CH₂)₁₋₂, S(O)₂NH or NHS(O)₂;
R^{31b} represents hydrogen, hydroxy, halogen, cyano, C₁₋₆ alkoxy, halogenated C₁₋₆ alkoxy, C₁₋₆ alkyl, deuterated C₁₋₆ alkyl, or halogenated C₁₋₆ alkyl;
R^{31c} represents hydrogen, C₁₋₆ alkyl, deuterated C₁₋₆ alkyl, or halogenated C₁₋₆ alkyl; or
R^{31b} and R^{31c}, together with their respective attached carbon and nitrogen atoms and the carbon atom on the benzene ring attached to the nitrogen atom, form an optionally substituted 4- to 6-membered heteroaromatic ring or an optionally substituted 4- to 6-membered heterocycle ring;
R^{31d} represents optionally substituted C₃₋₆ cycloalkyl, C₁₋₆ alkyl, deuterated C₁₋₆ alkyl, halogenated C₁₋₆ alkyl or optionally substituted 4- to 20-membered heterocyclyl;
(R^{31e})ₚ₃₁ₐ incicates that pyridin-2(1H)-one ring to which it is attached is substituted with p31a R^{31c}, with each R^{31c} being the same or different and each independently representing hydroxy, halogen, cyano, C₁₋₆ alkyl, C₁₋₆ alkoxy, halogenated C₁₋₆ alkoxy, deuterated C₁₋₆ alkyl, or halogenated C₁₋₆ alkyl;
p31a represents an integer of 0, 1, 2 or 3;
ring F in Formula (PBM-31) represents arylene or 5- to 20-membered monocyclic or bicyclic heteroarylene containing from 1 to 4 heteroatoms selected from the group consisting of oxygen, nitrogen, and sulfur; and (R^{31f})_{p31b} indicates that the ring F is optionally substituted with p31b R^{31f}, with each R^{31f} being the same or different and each independently representing deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p31b represents an integer of 0, 1, 2, 3, or 4;
R^{32c} in Formula (PBM-32) represents optionally substituted C₁₋₃ alkylene;
(R^{32a})ₚ₃₂ₐ indicates that the benzene ring to which it is attached is substituted with p32a R^{32a}, with each R^{32a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p32a represents an integer of 0, 1, 2, 3, 4 or 5;
(R^{32b})_{p32b} indicates that the 1H-indazole ring to which it is attached is substituted with p32b R^{32b}, with each R^{32b} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p32b represents an integer of 0, 1, 2, 3, 4 or 5;
R^{32d} represents C(O)NH, NHC(O), S(O)₂NH or NHS(O)₂;
R^{32e} represents NR^{32f}R^{32g}, where R^{32f} and R^{32g} are the same or different and each independently represent hydrogen, C₁₋₆ alkyl, deuterated C₁₋₆ alkyl, halogenated C₁₋₆ alkyl, optionally substituted C₃₋₆ cycloalkyl or optionally substituted 4- to 8-membered heterocyclyl;
X₁₃, X₁₄ and X₁₅ in Formula (PBM-33) are the same or different and each independently represent CH or N, wherein X₁₃, X₁₄ and X₁₅ are not simultaneously N, and the 6-membered ring containing X₁₃, X₁₄ and X₁₅ is optionally substituted with 1 or 2 substituents selected from the group consisting of halogen, hydroxy, cyano, amino, C₁₋₆ alkyl, C₁₋₆ alkoxy, halogenated C₁₋₆ alkoxy, deuterated C₁₋₆ alkyl, halogenated C₁₋₆ alkyl or any combination thereof;
ring G in Formula (PBM-33) represents pyrrole ring, imidazole ring, pyrazole ring, benzene ring, pyridine ring, pyrimidine ring, or pyrazine ring;
(R^{33a})ₚ₃₃ₐ indicates that the benzene ring to which it is attached is optionally substituted with p33a R^{33a}, with each R^{33a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p33a represents an integer of 0, 1, 2, 3, 4 or 5;
(R^{33b})_{p33b} indicates that the pyrrolidine ring to which it is attached is optionally substituted with p33b R^{33b}, with each R^{33b} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p33b represents an integer of 0, 1, 2, 3, 4, 5 or 6;
R^{33c} and R^{33d} are the same or different and each independently represent hydrogen, halogen, hydroxy, amino, cyano, C₁₋₆ alkyl, C₁₋₆ alkoxy, halogenated C₁₋₆ alkoxy, deuterated C₁₋₆ alkyl, or halogenated C₁₋₆ alkyl;
(R^{33c})_{p33c} indicates that the ring G to which it is attached is optionally substituted with p33c R^{33c}, with each R^{33c} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p33c represents an integer of 0 or 1;
X₁₆, X₁₇ and X₁₈ in Formula (PBM-34) each independently represent CH or N;
R^{34a} represents a bond, S or NH;
R^{34b} represents a bond, optionally substituted cycloalkyl or optionally substituted 4- to 15-membered nitrogen-containing heterocyclyl;
R^{34c} represents a bond, halogen, amino, hydroxy, cyano, C₁₋₆ alkyl, C₁₋₆ alkoxy, halogenated C₁₋₆ alkoxy, deuterated C₁₋₆ alkyl, halogenated C₁₋₆ alkyl, optionally substituted cycloalkyl or optionally substituted 4- to 15-membered nitrogen-containing heterocyclyl;
wherein R^{34b} and R^{34c} do not simultaneously represent a bond, and only one of R^{34b} and R^{34c} represents a bond, wherein when R^{34b} represents a bond, the ring carbon atom connected to R^{34b} is directly bonded to R_{c}, and when R^{34c} represents a bond, the ring carbon atom connected to R^{34c} is directly bonded to R_{c} ;
(R^{34d})ₚ₃₄ₐ indicates that the 6-membered ring to which it is attached is optionally substituted with p34a R^{34d}, with each R^{34d} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p34a represents an integer of 0, 1, 2, 3, or 4;
(R^{34e})_{p34b} indicates that the 6-membered nitrogen-containing heteroaryl ring to which it is attached is optionally substituted with p34b R^{34e}, with each R^{34e} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p34b represents an integer of 0, 1 or 2;
(R^{35a})ₚ₃₅ₐ in Formula (PBM-35) indicates that the phthalazinone ring to which it is attached is optionally substituted with p35a R^{35a}, with each R^{35a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p35a represents an integer of 0, 1, 2, 3, or 4;
R^{35b} represents optionally substituted C₁₋₃ alkylene;
(R^{35c})_{p35b} indicates that the benzene ring to which it is attached is optionally substituted with p35b R^{35c}, with each R^{35c} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p35b represents an integer of 0, 1, 2, 3, or 4;
(R^{36a})ₚ₃₆ₐ in Formula (PBM-36) indicates that the 2H-indazole ring to which it is attached is optionally substituted with p36a R^{36a}, with each R^{36a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p36a represents an integer of 0, 1, 2 or 3;
(R^{36b})_{p36b} indicates that the benzene ring to which it is attached is optionally substituted with p36b R^{36b}, with each R^{36b} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p36b represents an integer of 0, 1, 2, 3, or 4;
(R^{37a})ₚ₃₇ₐ in Formula (PBM-37) indicates that the benzofuran ring to which it is attached is optionally substituted with p37a R^{37a}, with each R^{37a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p37a represents an integer of 0, 1, 2 or 3;
(R^{37b})_{p37b} indicates that the benzene ring to which it is attached is optionally substituted with p37b R^{37b}, with each R^{37b} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p37b represents an integer of 0, 1, 2 or 3;
(R ^{38a})ₚ₃₈ₐ in Formula (PBM-38) indicates that the benzene ring to which it is attached is optionally substituted with p38a R^{38a}, with each R^{38a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p38a represents an integer of 0, 1, 2, 3, or 4;
R^{38b}, R^{38c}, and R^{38d} are the same or different and each independently represent hydrogen, deuterium, hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
R^{39a} and R^{39b} in Formula (PBM-39) are the same or different and each independently represent hydrogen, halogen, C₁₋₃ alkyl or halogenated C₁₋₃ alkyl;
R^{39c} represents a bond, hydrogen, C₁₋₆ alkyl, deuterated C₁₋₆ alkyl, or halogenated C₁₋₆ alkyl;
yl represents an integer of 1, 2 or 3;
R^{39d} represents the structure of the following formula: 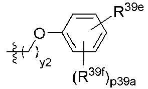
wherein R^{39c} represents a bond or hydrogen; (R^{39f})ₚ₃₉ₐ indicates that the benzene ring to which it is attached is optionally substituted with p39a R^{39f}, with each R^{39f} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl, and p39a represents an integer of 0, 1, 2, 3, or 4; and y2 represents an integer of 1, 2 or 3; or
R^{39d} represents the structure of the following formula: 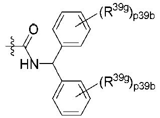
wherein each (R^{39g})_{p39b} independently indicates that the benzene ring to which it is attached is optionally substituted with p39b R^{39g}, with each R^{39g} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl, and p39b represents an integer of 0, 1, 2, 3, 4 or 5;
wherein R^{39c} and R^{39e} are not simultaneously a bond, and only one of R^{39c} and R^{39e} represents a bond, wherein when R^{39c} represents a bond, the ring nitrogen atom connected to R^{39c} is directly connected to R_{c}, and when R^{39e} represents a bond, the ring carbon atom connected to R^{39e} is directly connected to R_{c} ;
(R^{40a})ₚ₄₀ₐ in Formula (PBM-40) indicates that the benzene ring to which it is attached is optionally substituted with p40a R^{40a}, with each R^{40a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p40a represents an integer of 0, 1, 2, 3, or 4;
Rₓ₃ in Formula (PBM-40) represents a bond or C(O);
R^{41a} in Formula (PBM-41) represents hydrogen, C₁₋₆ alkyl, deuterated C₁₋₆ alkyl, halogenated C₁₋₆ alkyl, optionally substituted C₃₋₁₅ cycloalkyl, or optionally substituted 4- to 15-membered nitrogen-containing heterocyclyl;
R^{41b} represents hydrogen, C₁₋₆ alkyl, deuterated C₁₋₆ alkyl, halogenated C₁₋₆ alkyl, optionally substituted C₃₋₁₅ cycloalkyl, optionally substituted 4- to 15-membered nitrogen-containing heterocyclyl or NR^{41e}R^{41f}, where R^{41e} and R^{41f} are the same or different and each independently represent hydrogen, C₁₋₆ alkyl, deuterated C₁₋₆ alkyl, halogenated C₁₋₆ alkyl or optionally substituted 4- to 15-membered heterocyclyl;
R^{41c} represents N or CH;
(R^{41d})ₚ₄₁ₐ indicates that the benzene ring to which it is attached is optionally substituted with p41a R^{41d}, with each R^{41d} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p41a represents an integer of 0, 1, 2, 3, or 4;
(R^{42a})ₚ₄₂ₐ in Formula (PBM-42) indicates that the benzene ring to which it is attached is optionally substituted with p42a R^{42a}, with each R^{42a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p42a represents an integer of 0, 1, 2, 3, 4 or 5;
R^{42b} represents O, S, C₁₋₃ alkylene or halogenated C₁₋₃ alkylene;
(R^{42c})_{p42c} indicates that the benzene ring to which it is attached is optionally substituted with p42b R^{42c}, with each R^{42c} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p42b represents an integer of 0, 1, 2, 3, or 4;
ring H in Formula (PBM-43) represents C₆₋₁₅ arylene or 5- to 15-membered monocyclic, bicyclic, or polycyclic heteroarylene, and (R^{43a})ₚ₄₃ₐ indicates that the ring H is optionally substituted with p43a R^{43a}, with each R^{43a} being the same or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p43a represents an integer of 0, 1, 2, 3, or 4;
R^{43b} represents an optionally substituted 5- to 20-membered monocyclic, bicyclic, or polycyclic heteroaryl group containing 1 to 4 heteroatoms selected from N, O, and S, which is optionally substituted with a substituent selected from the group consisting of hydroxy, amino, cyano, halogen, nitro, C₁₋₆ alkyl, C₁₋₆ alkoxy, halogenated C₁₋₆ alkoxy, deuterated C₁₋₆ alkyl, or halogenated C₁₋₆ alkyl;
R^{44a}, R^{44b}, and R^{44c} in Formula (PBM-44) each independently represent CH or N;
R^{44d} represents -C(O)NHR^{44h}, -NHC(O)R^{44h}, -S(O)₂NHR^{44h}, -NHS(O)₂R^{44h}, -S(O)₂R ^{44h} or -P(O)(R^{44h})₂, wherein each R^{44h} is the same or different and each independently represents C₁₋₆ alkyl, deuterated C₁₋₆ alkyl, or halogenated C₁₋₆ alkyl;
(R^{44c})ₚ₄₄ₐ represents p44a R^{44c} groups, wherein each R^{44c} is the same or different and each independently represents halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p44a represents an integer of 0, 1, 2, 3, or 4;
(R^{44f})_{p44b} represents p44b R^{44f} groups, wherein each R^{44f} is the same or different and each independently represents halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p44b represents an integer of 0, 1 or 2;
(R^{44g})_{p44c} indicates that the benzene ring to which it is attached is optionally substituted with p44c R^{44g}, with each R^{44g} independently representing halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p44c represents an integer of 0, 1, 2, 3, or 4;
R_{c1} in Formula (PBM-45) represents a bond, or -C(O)NH-R_{c2}-C(O)-***, wherein symbol *** indicates the point of attachment to R_{c}, and R_{c2} represents optionally substituted C₃₋₁₅ cycloalkyl;
(R^{45a})ₚ₄₅ₐ indicates that the 1H-pyrrolo[2,3-b]pyridine ring to which it is attached is optionally substituted with p45a R^{45a}, wherein each R^{45a} is independently cyano, halogen, hydroxy, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p45a represents an integer of 0, 1, 2, 3, 4 or 5;
(R^{45b})_{p45b} indicates that the pyridine ring to which it is attached is optionally substituted with p45b R^{45b} wherein each R^{45b} is independently cyano, halogen, hydroxy, nitro, C₁₋₆ alkyl, halogenated C₁₋₆ alkyl, deuterated C₁₋₆ alkyl, C₁₋₆ alkoxy, halogenated C₁₋₆ alkoxy or NR^{45c}R^{45d}, where R^{45c} and R^{45d} are the same or different and each independently represent hydrogen, C₁₋₆ alkyl, deuterated C₁₋₆ alkyl, halogenated C₁₋₆ alkyl, optionally substituted C₃₋₁₅ cycloalkyl or optionally substituted 4- to 15-membered heterocyclyl, and p45b represents an integer of 0, 1, 2 or 3;
R^{46a} in Formula (PBM-46) represents NHC(O), C(O)NH, NHS(O)₂ or S(O)₂NH;
(R^{46b})ₚ₄₆ₐ indicates that the benzene ring to which it is attached is optionally substituted with p46a R^{46b}, wherein each R^{46b} is independently halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p46a represents an integer of 0, 1, 2, 3, 4 or 5;
(R^{46c})_{P46b} indicates that the 1H-pyrazole ring to which it is attached is optionally substituted with p46b R^{46c}, wherein each R^{46c} is independently halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p46b represents an integer of 0, 1 or 2;
R^{47a} in Formula (PBM-47) represents N or CH, and R^{47b} represents O, S, NH, CH₂, CH(F) or C(F)₂;
R^{47c} represents a bond or optionally substituted methylene (e.g., halogenated methylene);
ring I represents C₆₋₁₅ aryl, 4- to 15-membered heterocyclyl, or 5- to 15-membered heteroaryl, and (R^{47d})ₚ₄₇ₐ indicates that the ring I to which it is attached is optionally substituted with p47a R^{47d}, with each R^{47d} being the same or different and each independently representing deuterium, halogen, hydroxy, cyano, amino, nitro, oxo, C₁₋₆ alkyl, halogenated C₁₋₆ alkyl, deuterated C₁₋₆ alkyl, C₁₋₆ alkoxy, or halogenated C₁₋₆ alkoxy, and p47a represents an integer of 0, 1, 2, 3, or 4;
(R^{47e})_{p47b} indicates that the 6-membered nitrogen-containing heteroaromatic ring containing R^{47a} to which it is attached is optionally substituted with p47b R^{47e}, with each R^{47e} independently representing deuterium, halogen, hydroxy, cyano, amino, nitro, C₁₋₆ alkoxy, halogenated C₁₋₆ alkoxy, C₁₋₆ alkyl, deuterated C₁₋₆ alkyl, or halogenated C₁₋₆ alkyl, and p47b represents an integer of 0, 1 or 2;
(R^{47f})_{p47c} indicates that the benzene ring to which it is attached is optionally substituted with p47c R^{47f}, with each R^{47f} being the same or different and each independently representing deuterium, halogen, hydroxy, cyano, amino, nitro, C₁₋₆ alkoxy, halogenated C₁₋₆ alkoxy, C₁₋₆ alkyl, deuterated C₁₋₆ alkyl, or halogenated C₁₋₆ alkyl, and p47c represents an integer of 0, 1, 2, 3, or 4;
R^{48a}, R^{48b} and R^{48c} in Formula (PBM-48) independently represent N or CH;
(R^{48d})ₚ₄₈ₐ indicates that the naphthyl to which it is attached is optionally substituted with p48a R^{48d}, wherein each R^{48d} is independently halogen, hydroxy, amino, mercapto, nitro, cyano, C₂₋₆ alkynyl, C₂₋₆ alkenyl, C₁₋₆ alkoxy, halogenated C₁₋₆ alkoxy, C₁₋₆ alkyl, deuterated C₁₋₆ alkyl, or halogenated C₁₋₆ alkyl, and p48a represents an integer of 0, 1, 2, 3, 4, 5, 6 or 7;
R^{48e} represents optionally substituted C₃₋₁₅ cycloalkyl or optionally substituted 4- to 15-membered heterocyclyl containing from 1 to 4 heteroatoms independently selected from sulfur, oxygen, and nitrogen;
R^{43f} represents hydrogen, halogen, hydroxy, cyano, amino, mercapto, nitro, C₂₋₆ alkynyl, C₂₋₆ alkenyl, C₁₋₆ alkoxy, halogenated C₁₋₆ alkoxy, C₁₋₆ alkyl, deuterated C₁₋₆ alkyl, or halogenated C₁₋₆ alkyl;
R^{49a} in Formula (PBM-49) represents N or CH;
_{R}^{49b} represents NHC(O), C(O)NH, NHS(O)₂ or S(O)₂NH;
(R^{49c})ₚ₄₉ₐ indicates that the 6-membered ring containing R^{49a} is optionally substituted with p49a R^{49c}, with each R^{49c} being the same or different and each independently representing halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p49a represents an integer of 0, 1, 2, 3, or 4;
ring J represents 5- to 15-membered heteroarylene, and (R^{49d})_{P49b} indicates that the ring J to which it is attached is optionally substituted with p49b R^{49d}, with each R^{49d} being the same or different and each independently representing halogen, hydroxy, mercapto, cyano, amino, nitro, C₁₋₆ alkyl, halogenated C₁₋₆ alkyl, deuterated C₁₋₆ alkyl, hydroxy-substituted C₁₋₆ alkyl, amino-substituted C₁₋₆ alkyl, C₁₋₆ alkoxy, halogenated C₁₋₆ alkoxy or optionally substituted C₃₋₁₅ cycloalkyl, and p49b represents an integer of 0, 1, 2 or 3;
(R^{50a})ₚ₅₀ₐ in Formula (PBM-50) indicates that the benzene ring to which it is attached is substituted with p50a R^{50a}, with each R^{50a} being the same or different and each independently representing halogen, hydroxy, mercapto, cyano, amino, nitro, C₁₋₆ alkyl, halogenated C₁₋₆ alkyl, deuterated C₁₋₆ alkyl, C₁₋₆ alkyl-S(O)₂, halogenated C₁₋₆ alkyl-S(O)₂, halogenated C₁₋₆ alkyl-NHC(O), halogenated C₁₋₆ alkyl-C(O)NH, halogenated C₁₋₆ alkyl-NHS(O)₂, halogenated C₁₋₆ alkyl-S(O)₂NH, C₁₋₆ alkoxy, or halogenated C₁₋₆ alkoxy, and p50a represents an integer of 0, 1, 2, 3, or 4;
R^{50b} represents hydrogen, C₁₋₆ alkyl, halogenated C₁₋₆ alkyl, or deuterated C₁₋₆ alkyl;
(R^{51a})ₚ₅₁ₐ in Formula (PBM-51) indicates that the benzene ring to which it is attached is substituted with p51a R^{51a}, with each R^{51a} being the same or different and each independently representing halogen, hydroxy, mercapto, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy, optionally substituted C₃₋₁₅ cycloalkyl or optionally substituted 4- to 15-membered heterocyclyl containing from 1 to 4 heteroatoms selected from the group consisting of oxygen, nitrogen, and sulfur, and p51a represents an integer of 0, 1, 2, 3, 4 or 5;
(R^{51b})_{p51b} indicates that the benzene ring to which it is attached is substituted with p51b R^{51b}, with each R^{51d} being the same or different and each independently representing halogen, hydroxy, mercapto, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy, optionally substituted C₃₋₁₅ cycloalkyl or optionally substituted 4- to 15-membered heterocyclyl containing from 1 to 4 heteroatoms selected from the group consisting of oxygen, nitrogen, and sulfur, and p51b represents an integer of 0, 1, 2, 3, or 4;
(R^{51c})_{p51c} indicates that the isoxazole ring to which it is attached is optionally substituted with p51c R^{51c} with each R^{51c} being the same or different and each independently representing halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p51c represents an integer of 0, 1 or 2;
the dashed line in Formula (PBM-52) indicates the optional presence of a double bond;
(R^{52a})ₚ₅₂ₐ indicates that the 6-membered cycloalkyl ring to which it is attached is optionally substituted with p52a R^{52a}, with each R^{52a} being the same or different and each independently representing halogen, hydroxy, mercapto, cyano, amino, nitro, oxo, C₁₋₆ alkyl, halogenated C₁₋₆ alkyl, deuterated C₁₋₆ alkyl, C₁₋₆ alkoxy, or halogenated C₁₋₆ alkoxy, and p52a represents an integer of 0, 1, 2, 3, 4, 5, 6, 7 or 8;
(R^{52b})_{p52b} indicates that the benzene ring to which it is attached is optionally substituted with p52b R^{52b}, with each R^{52b} being the same or different and each independently representing deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p52b represents an integer of 0, 1, 2, 3, 4 or 5;
(R^{52c})_{p52c} indicates that the 6-membered cycloalkyl ring to which it is attached is optionally substituted with p52c R^{52c}, with each R^{52c} being the same or different and each independently representing halogen, cyano, oxo, C₁₋₆ alkyl, halogenated C₁₋₆ alkyl, deuterated C₁₋₆ alkyl, C₁₋₆ alkoxy, or halogenated C₁₋₆ alkoxy, and p52c represents an integer of 0, 1, 2, 3, 4, 5, 6, 7 or 8;
(R^{52d})_{p52d} indicates that the benzene ring to which it is attached is optionally substituted with p52d R^{52d}, with each R^{52d} being the same or different and each independently representing halogen, hydroxy, mercapto, C₁₋₆ alkyl, halogenated C₁₋₆ alkyl, deuterated C₁₋₆ alkyl, C₁₋₆ alkoxy, halogenated C₁₋₆ alkoxy, or 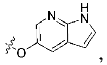 and p52d represents an integer of 0, 1 or 2;
(R^{52e})ₚ₅₂ₑ indicates that the benzene ring to which it is attached is optionally substituted with p52e R^{52e}, with each R^{52e} being the same or different and each independently representing deuterium, halogen, hydroxy, mercapto, cyano, amino, nitro, -SO₂CF₃, C₁₋₆ alkyl, halogenated C₁₋₆ alkyl, deuterated C₁₋₆ alkyl, C₁₋₆ alkoxy, or halogenated C₁₋₆ alkoxy, and p52e represents an integer of 0, 1, 2, 3, or 4;
R^{52f} represents hydrogen, deuterium, halogen, hydroxy, mercapto, cyano, amino, nitro,C₁₋₆ alkyl, halogenated C₁₋₆ alkyl, deuterated C₁₋₆ alkyl, C₁₋₆ alkoxy, halogenated C₁₋₆ alkoxy, or 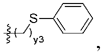 where y3 represents an integer of 1, 2 or 3;
(R^{53a})ₚ₅₃ₐ in Formula (PBM-53) represents p53a R^{53a} groups, with each R^{53a} being the same or different and each independently representing halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p53a represents an integer of 0, 1, 2, 3, 4 or 5;
(R^{53b})_{p53b} indicates that the benzene ring to which it is attached is optionally substituted with p53b R^{53b}, with each R^{53b} being the same or different and each independently representing deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p53b represents an integer of 0, 1, 2, 3, or 4; and
R^{53c} represents C₁₋₆ alkylene or halogenated C₁₋₆ alkylene;
(R^{54a})ₚ₅₄ₐ in Formula (PBM-54) represents p54a R^{54a} groups, wherein each R^{54a} is the same or different and each independently represents halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p54a represents an integer of 0, 1, 2, 3, or 4;
(R^{54b})_{p54b} indicates that the benzene ring to which it is attached is optionally substituted with p54b R^{54b}, with each R^{54b} being the same or different and each independently representing deuterium, halogen, hydroxy, mercapto, cyano, amino, nitro,C₁₋₆ alkyl, halogenated C₁₋₆ alkyl, deuterated C₁₋₆ alkyl, C₁₋₆ alkoxy, or halogenated C₁₋₆ alkoxy, and p54b represents an integer of 0, 1, 2, 3, or 4;
(R^{55a})ₚ₅₅ₐ in Formula (PBM-55) represents p55a R^{55a} groups, wherein each R^{55a} is the same or different and each independently represents halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p55a represents an integer of 0, 1, 2, 3, or 4;
R^{55b} and R^{55c} are the same or different and each independently represent hydrogen, C₁₋₆ alkyl, halogenated C₁₋₆ alkyl, deuterated C₁₋₆ alkyl, or optionally substituted aryl;
R^{55d} in Formula (PBM-55) represents a fragment 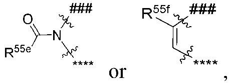 wherein symbol ### indicates the point of attachment to the ring carbon atom, symbol **** indicates the point of attachment to the ring nitrogen atom, R^{55e} represents optionally substituted aryl or optionally substituted heteroaryl, and R^{55f} represents hydrogen, halogen, C₁₋₆ alkyl, halogenated C₁₋₆ alkyl or deuterated C₁₋₆ alkyl; and
R_{c} in each general formula independently represents a bond, -O-, -N(R_{d})-, -NHC(O)-*, -C(O)NH-*, - N(R_{d})-R_{f}-N(Rₑ)-*, -R_{f}-C(O)NH-*, -R_{f}-NHC(O)-*, -R_{f}-C(O)O-*, -R_{f}-OC(O)-*, -C(O)O-*, -OC(O)-*, -R_{f}-, - R_{f}-N(R_{d})-*, -O-R_{f}-N(R_{d})-*, 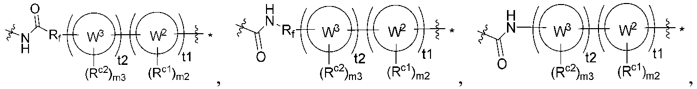 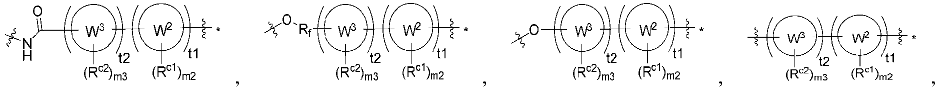 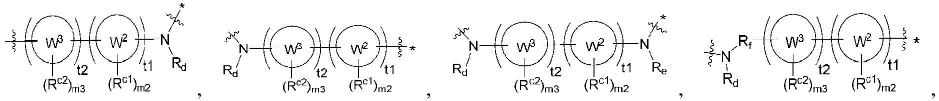 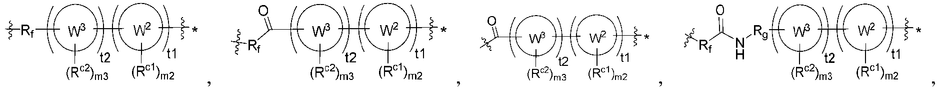 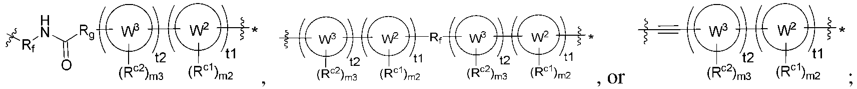
   wherein each ring W² is the same or different and each independently represents nitrogen-containing heterocyclylene, t1 represents an integer of 1 or 2, and (R^{cl})ₘ₂ indicates that each ring W² is independently optionally substituted with m2 R^{c1} groups, with each R^{c1} being independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c4}-R^{c3}-, where R^{c3} is optionally substituted C₁₋₆ alkylene or optionally substituted C₂₋₆ alkenylene, and R^{c4} represents halogen, R^{c5}R^{c6}N-, hydroxy, carboxyl, ethynyl, or vinyl, wherein R^{c5} and R^{c6} are the same or different and each independently represent hydrogen or C₁₋₃ alkyl, and m2 represents an integer of 0-20;
   each ring W³ is the same or different and each independently represents heterocyclylene, cycloalkylene, arylene, or heteroarylene, t2 represents an integer of 0 or 1, and (R^{c2})ₘ₃ indicates that each ring W³ is independently optionally substituted with m3 R^{c2} groups, with each R^{c2} being independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c8}-R^{c7}-, where R^{c7} is optionally substituted C₁₋₆ alkylene or optionally substituted C₂₋₆ alkenylene, and R^{c8} represents halogen, R^{c9}R^{c10}N-, hydroxy, carboxyl, ethynyl, or vinyl, wherein R^{c9} and R^{c10} are the same or different and each independently represent hydrogen or C₁₋₃ alkyl, and m3 represents an integer of 0-20; and
   R_{d} and R_{c} each independently represent hydrogen or C₁₋₆ alkyl, R_{f} and R_{g} each independently represent optionally substituted C₁₋₆ alkylene or optionally substituted C₂₋₆ alkenylene, and symbol * indicates the point of attachment to LIN.

In the embodiments of the present disclosure, the number of substituents is not theoretically limited in any way, or is automatically limited by the size of the building units (i.e., the total number of replaceable hydrogen atoms in the building units), or as clearly defined herein.

In some embodiments, PBM represents a small molecule ligand of EGFR.

In some embodiments, PBM represents a structure of Formula (PBM-1-1A), (PBM-1-1B), (PBM-1-1C) or (PBM-1-1D): 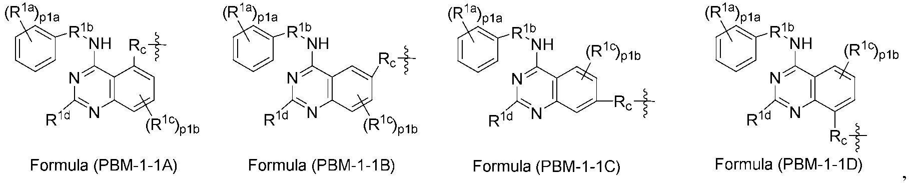 wherein in each of Formulae (PBM-1-1A), (PBM-1-1B), (PBM-1-1C) and (PBM-1-1D),
(R^{1a})ₚ₁ₐ indicates that the benzene ring to which it is attached is substituted with p1a R^{1a} , with each R^{1a} being the same or different and each independently representing deuterium, halogen (e.g., fluorine, chlorine, bromine, or iodine), hydroxy, NH₂, NO₂, cyano, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p1a represents an integer of 1, 2, 3, 4 or 5;
R^{1b} represents a bond, or C₁₋₂ alkylene optionally substituted with a substituent selected from the group consisting of C₁₋₃ alkyl, deuterated C₁₋₃ alkyl, halogenated C₁₋₃ alkyl, and halogen;
(R^{1c})_{p1d} indicates that the quinazoline ring in Formula (PBM-1) is substituted with plb R^{1c} , with each R^{1c} being the same or different and each independently representing deuterium, halogen (e.g., fluorine, chlorine, bromine, or iodine), NO₂, cyano, halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₁₀ alkyl (e.g., C₁₋₆ alkyl or C₁₋₄ alkyl, such as methyl, ethyl, propyl, isopropyl, butyl, sec-butyl, tert-butyl, pentyl, or hexyl), deuterated C₁₋₁₀ alkyl (e.g., perdeuterated C₁₋₆ alkyl or perdeuterated C₁₋₄ alkyl, such as CD₃, CD₃CD₂-, CD₃CD₂CD₂- etc.), C₁₋₁₀ alkoxy (e.g., C₁₋₆ alkoxy or C₁₋₄ alkoxy, such as methoxy, ethoxy, propoxy, isopropoxy, n-butoxy, isobutoxy, or tert-butoxy), halogenated C₁₋₁₀ alkoxy (e.g., halogenated C₁₋₆ alkoxy or halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), -O-optionally substituted heterocyclyl (e.g., -O-optionally substituted 4- to 20-membered heterocyclyl), or -NHC(O)R^{1e}, where R^{1e} represents C₁₋₁₀ alkyl (e.g., C₁₋₈ alkyl, C₁₋₆ alkyl or C₁₋₄ alkyl, such as methyl, ethyl, propyl, isopropyl, butyl, sec-butyl, tert-butyl, pentyl, hexyl, heptyl, or octyl), deuterated C₁₋₁₀ alkyl (e.g., perdeuterated C₁₋₈ alkyl, perdeuterated C₁₋₆ alkyl, or perdeuterated C₁₋₄ alkyl, such as CD₃, CD₃CD₂-, CD₃CD₂CD₂- etc.) or C₂₋₁₀ alkenyl (e.g., C₂₋₆ alkenyl or C₂₋₄ alkenyl, such as vinyl, propenyl, butenyl or pentenyl), e.g., R^{1c} represents methyl, methoxy, 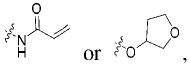 such as 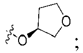
p1b represents an integer of 1, 2, or 3; and
R^{1d} represents hydrogen, deuterium or C₁₋₃ alkyl.

In some embodiments, (R^{1c})_{p1b} indicates that the quinazoline ring in Formula (PBM-1) is substituted with p1b R^{1c} , wherein p1b represents an integer of 1, 2, or 3, and R^{1c} represents -O-optionally substituted heterocyclyl. The heterocyclyl is a 4- to 20-membered (e.g., 4- to 15-membered, 4- to 12-membered, 4- to 10-membered, 4- to 7-membered, or 4- to 6-membered) monocyclic, bicyclic, or polycyclic heterocyclyl group containing one or more (e.g., 1-4, or 1, 2, 3, or 4) heteroatoms selected from nitrogen, oxygen, and sulfur, including but not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl). In some embodiments, the heterocyclyl is optionally substituted with a substituent(s) selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof. In some embodiments, p1b represents an integer of 1.

In some embodiments, R^{1c} represents:
fluorine, chlorine, bromine, or iodine;
methyl, ethyl, propyl, isopropyl, butyl, sec-butyl, tert-butyl, pentyl, hexyl, heptyl, or octyl;
methoxy, ethoxy, propoxy, isopropoxy, n-butoxy, isobutoxy, or tert-butoxy;
-NHC(O)-C₂₋₁₀ alkenyl, e.g., -NHC(O)-vinyl (i.e., 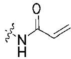 ), -NHC(O)-propenyl or -NHC(O)-butenyl; or

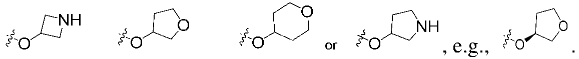

In some embodiments, R^{1b} represents a bond, or C₁₋₂ alkylene optionally substituted with a substituent selected from the group consisting of C₁₋₃ alkyl, deuterated C₁₋₃ alkyl, halogenated C₁₋₃ alkyl, and halogen. In some embodiments, R^{1b} represents a bond, or methylene or ethylene which are optionally substituted with a substituent selected from the group consisting of C₁₋₃ alkyl, deuterated C₁₋₃ alkyl, halogenated C₁₋₃ alkyl and halogen. In some embodiments, R^{1b} represents a bond.

In some embodiments, PBM represents a structure of Formula (PBM-1-1), (PBM-1-2), (PBM-1-3), (PBM-1-4) or (PBM-1-5):

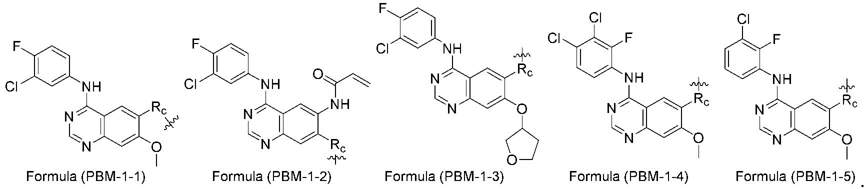

In some embodiments, PBM represents a structure of Formula (PBM-2-1A), (PBM-2-1B), (PBM-2-1C), (PBM-2-1D) or (PBM-2-1E): 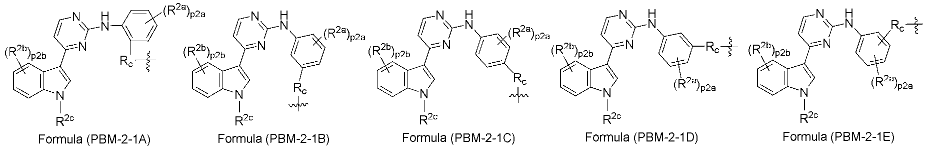 wherein in each of Formulae (PBM-2-1A), (PBM-2-1B), (PBM-2-1C), (PBM-2-1D) and (PBM-2-1E),
(R^{2a})ₚ₂ₐ indicates that the benzene ring to which it is attached is substituted with p2a R^{2a} , with each R^{2a} being the same or different and each independently representing deuterium, halogen (e.g., fluorine, chlorine, bromine, or iodine), nitro, cyano, halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₁₀ alkyl (e.g., C₁₋₈ alkyl, C₁₋₆ alkyl or C₁₋₄ alkyl, such as methyl, ethyl, propyl, isopropyl, butyl, sec-butyl, tert-butyl, pentyl, hexyl, heptyl, or octyl), deuterated C₁₋₁₀ alkyl (e.g., perdeuterated C₁₋₈ alkyl, perdeuterated C₁₋₆ alkyl, or perdeuterated C₁₋₄ alkyl, such as CD₃, CD₃CD₂-, CD₃CD₂CD₂- etc.), C₁₋₁₀ alkoxy (e.g., C₁₋₈ alkoxy, C₁₋₆ alkoxy or C₁₋₄ alkoxy, such as methoxy, ethoxy, propoxy, isopropoxy, n-butoxy, isobutoxy, or tert-butoxy) or -NHC(O)R^{2d}, where R^{2d} represents C₁₋₁₀ alkyl (e.g., C₁₋₈ alkyl, C₁₋₆ alkyl or C₁₋₄ alkyl, such as methyl, ethyl, propyl, isopropyl, butyl, sec-butyl, tert-butyl, pentyl, hexyl, heptyl, or octyl), deuterated C₁₋₁₀ alkyl (e.g., perdeuterated C₁₋₈ alkyl, perdeuterated C₁₋₆ alkyl, or perdeuterated C₁₋₄ alkyl, such as CD₃, CD₃CD₂-, CD₃CD₂CD₂- etc.) or C₂₋₁₀ alkenyl (e.g., C₂₋₆ alkenyl or C₂₋₄ alkenyl, such as vinyl, propenyl, butenyl or pentenyl), e.g., R^{2a} represents methyl, methoxy, nitro or 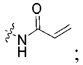
p2a represents an integer of 1, 2, 3 or 4;
(R^{2b})_{p2b} indicates that the 1H-indole ring to which it is attached is substituted with p2b R^{2b} , wherein p2b represents an integer of 0, 1, 2 or 3, and each R^{2b} is the same or different and each independently represents deuterium, deuterated C₁₋₁₀ alkyl (e.g., perdeuterated C₁₋₈ alkyl, perdeuterated C₁₋₆ alkyl, or perdeuterated C₁₋₄ alkyl, such as CD₃, CD₃CD₂-, CD₃CD₂CD₂- etc.), C₁₋₁₀ alkyl (e.g., C₁₋₈ alkyl, C₁₋₆ alkyl or C₁₋₄ alkyl, such as methyl, ethyl, propyl, isopropyl, butyl, sec-butyl, tert-butyl, pentyl, hexyl, heptyl, or octyl), halogen (e.g., fluorine, chlorine, bromine, or iodine) or halogenated C₁₋₁₀ alkyl (e.g., halogenated C₁₋₆ alkyl or halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
R^{2c} represents hydrogen, C₁₋₁₀ alkyl (e.g., C₁₋₈ alkyl, C₁₋₆ alkyl or C₁₋₄ alkyl, such as methyl, ethyl, propyl, isopropyl, butyl, sec-butyl, tert-butyl, pentyl, hexyl, heptyl, or octyl), deuterated C₁₋₁₀ alkyl (e.g., perdeuterated C₁₋₈ alkyl, perdeuterated C₁₋₆ alkyl, or perdeuterated C₁₋₄ alkyl, such as CD₃, CD₃CD₂-, CD₃CD₂CD₂- etc.), halogen (e.g., fluorine, chlorine, bromine, or iodine) or halogenated C₁₋₁₀ alkyl (e.g., halogenated C₁₋₆ alkyl or halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structures of (PBM-2-1) or Formula (PBM-2-2):

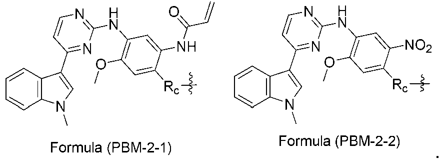

In some embodiments, PBM represents the structure of Formula (PBM-3): 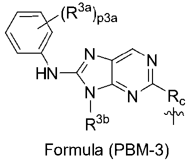
wherein (R^{3a})ₚ₃ₐ indicates that the benzene ring to which it is attached is substituted with p3a R^{3a} , with each R^{3\a} being the same or different and each independently representing deuterium, halogen (e.g., fluorine, chlorine, bromine, or iodine), hydroxy, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), NO₂, NH₂ or cyano;
p3a represents an integer of 0, 1, 2, 3, 4 or 5;
R^{3b} represents substituted or unsubstituted C₃₋₁₅ cycloalkyl (e.g., substituted or unsubstituted C₃₋₁₀ cycloalkyl or C₃₋₆ cycloalkyl, such as cyclopropyl, cyclobutyl, cyclopentyl, or cyclohexyl). In some embodiments, the C₃₋₁₅ cycloalkyl is optionally substituted with one or more (e.g., 1-3, such as 1, 2 or 3) substituents selected from the group consisting of deuterium, hydroxy, amino, mercapto, nitro, halogen (e.g., fluorine, chlorine, bromine, or iodine), cyano, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally halogenated C₃₋₆ cycloalkyl, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl-NH-, NH₂-C₁₋₆ alkylene, C₁₋₆ alkyl-NHC(O)-, C₁₋₆ alkyl-C(O)NH- or any combination thereof.

In some embodiments, PBM represents the structure of Formula (PBM-3-1):

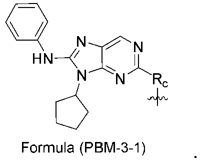

In some embodiments, PBM represents a structure of Formula (PBM-4-1A), (PBM-4-1B), (PBM-4-1C) or (PBM-4-1D): 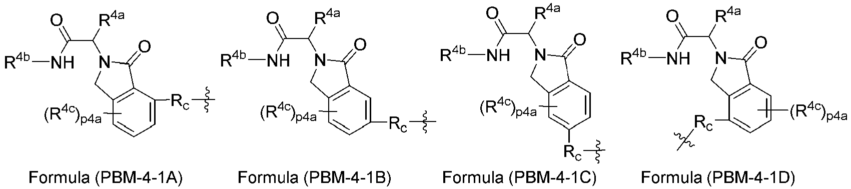 wherein in each of Formulae (PBM-4-1A), (PBM-4-1B), (PBM-4-1C) and (PBM-4-1D),
R^{4a} represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), wherein the C₆-₁₀ aryl and 5- to 20-membered heteroaryl are each independently optionally substituted with one or more (e.g., 1-6, such as 1, 2, 3, 4, 5 or 6) substituents independently selected from the group consisting of deuterium, halogen (e.g., fluorine, chlorine, bromine, or iodine), hydroxy, cyano, amino, nitro, C₁₋₆ alkyl (e.g., C₁₋₆ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof;
R^{4b} represents C₆-₁₀ aryl or 5- to 20-membered heteroaryl (e.g., 5- to 20-membered heteroaryl containing one or two 5- to 7-membered rings and 1 to 4 heteroatoms selected from N, O, and S), wherein the C₆-₁₀ aryl and 5- to 20-membered heteroaryl are each independently optionally substituted with one or more (e.g., 1-6, such as 1, 2, 3, 4, 5 or 6) substituents selected from the group consisting of deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂CICH₂-O-); and
(R^{4c})ₚ₄ₐ indicates that the isoindoline ring to which it is attached is optionally substituted with p4a R^{4c}, with each R^{4c} being the same or different and each independently representing deuterium, halogen (e.g., fluorine, chlorine, bromine, or iodine), hydroxy, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), NO₂, NH₂, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or cyano; and
p4a represents an integer of 0, 1, 2 or 3.

In some embodiments, R^{4a} represents C₆₋₁₀ aryl, such as but not limited to phenyl or naphthyl, each of which is optionally substituted with one or more (e.g., 1-6, such as 1, 2, 3, 4, 5 or 6) substituents independently selected from the group consisting of deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂CICH₂-O-) or any combination thereof.

In some embodiments, R^{4a} represents 5- to 20-membered heteroaryl, e.g., 5- to 20-membered heteroaryl containing one or two 5- to 7-membered rings and 1 to 4 heteroatoms selected from N, O, and S. In a sub-embodiment, R^{4a} represents 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, imidazo[1,2-b]pyridazinyl or 6,7-dihydro-5H-pyrrolo[1,2-c]imidazolyl. The heteroaryl group is optionally substituted with one or more (e.g., 1-6, such as 1, 2, 3, 4, 5 or 6) substituents independently selected from the group consisting of deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof.

In some embodiments, R^{4b} represents C₆₋₁₀ aryl, such as but not limited to phenyl or naphthyl, each of which is optionally substituted with one or more (e.g., 1-6, such as 1, 2, 3, 4, 5 or 6) R^{4d} as defined above.

In some embodiments, R^{4b} represents heteroaryl containing one or two 5- to 7-membered rings and 1 to 4 heteroatoms selected from N, O, and S. In a sub-embodiment, R^{4b} represents 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl group is optionally substituted with one or more (e.g., 1-6, such as 1, 2, 3, 4, 5 or 6) R^{4d} as defined above.

In some embodiments, PBM represents a structure of Formula (PBM-4-1), (PBM-4-2), (PBM-4-3), (PBM-4-4) or (PBM-4-5):

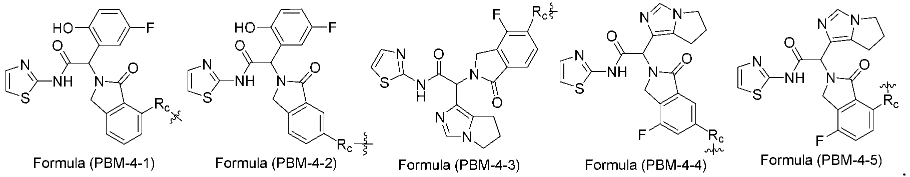

In some embodiments, PBM represents the structure of Formula (PBM-5): 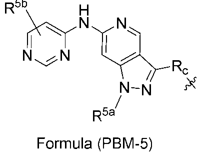
wherein R^{5a} represents hydrogen, C₁₋₁₀ alkyl (e.g., C₁₋₈ alkyl, C₁₋₆ alkyl or C₁₋₄ alkyl, such as methyl, ethyl, propyl, isopropyl 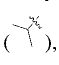 butyl, sec-butyl, tert-butyl, pentyl, hexyl, heptyl, or octyl), deuterated C₁₋₁₀ alkyl (e.g., perdeuterated C₁₋₈ alkyl, perdeuterated C₁₋₆ alkyl, or perdeuterated C₁₋₄ alkyl, such as CD₃, CD₃CD₂-, CD₃CD₂CD₂- etc.) or halogenated C₁₋₁₀ alkyl (e.g., halogenated C₁₋₈ alkyl or halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
R^{5b} represents 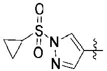 or 4- to 12-membered nitrogen-containing heterocyclyl, wherein the 4- to 12-membered nitrogen-containing heterocyclyl is optionally substituted with one or more substituents, wherein each substituent is independently selected from the group consisting of deuterium, halogen (e.g., fluorine, chlorine, bromine, or iodine), hydroxy, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy (e.g., perdeuterated C₁₋₄ alkoxy, such as CD₃-O-, CD₃CD₂-O-, or CD₃CD₂CD₂-O-), NO₂, NH₂, cyano or any combination thereof. In some sub-embodiments, examples of 4- to 12-membered nitrogen-containing heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl).

In some embodiments, PBM represents a structure of Formula (PBM-5-1) or (PBM-5-2):

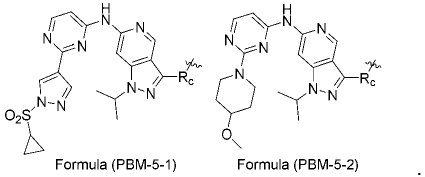

In some embodiments, PBM represents the structure of Formula (PBM-44): 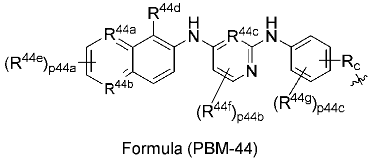
wherein R^{44a}, R^{44b}, and R^{44c} each independently represent CH or N;
R^{44d} represents -C(O)NHR^{44h}, -NHC(O)R^{44h}, -S(O)2NHR^{44h}, -NHS(O)2R^{44h}, -S(O)2R^{44h} or -P(O)(R^{44h})₂, wherein each R^{44h} is the same or different and each independently represents C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.) or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
(R^{44e})ₚ₄₄ₐ represents p44a R^{44e} groups, wherein each R^{44e} is the same or different and each independently represents halogen (e.g., fluorine, chlorine, bromine, or iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.), C₁₋₆ alkoxy (e.g., C₁₋₅ alkoxy, C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as trifluoromethoxy );
p44a represents an integer of 0, 1, 2, 3 or 4;
(R^{44f})_{p44b} represents p44b R^{44f} groups, wherein each R^{44f} is the same or different and each independently represents halogen (e.g., fluorine, chlorine, bromine, or iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.), C₁₋₆ alkoxy (e.g., C₁₋₅ alkoxy, C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as trifluoromethoxy);
p44b represents an integer of 0, 1 or 2;
(R^{44g})_{p44c} indicates that the benzene ring to which it is attached is optionally substituted with p44c R^{44g}, with each R^{44g} independently representing halogen (e.g., fluorine, chlorine, bromine, or iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.), C₁₋₆ alkoxy (e.g., C₁₋₅ alkoxy, C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as trifluoromethoxy ); and
p44c represents an integer of 0, 1, 2, 3 or 4.

In some embodiments, R^{44a} and R^{44b} each independently represent CH or N. In some sub-embodiments, R^{44a} represents CH, and R^{44b} represents N. In some sub-embodiments, R^{44a} represents CH, and R^{44b} represents CH. In some sub-embodiments, R^{44a} represents N, and R^{44b} represents CH. In some sub-embodiments, R^{44a} represents N, and R^{44b} represents N.

In some embodiments, R^{44c} represents CH or N. In some sub-embodiments, R^{44c} represents N. In some sub-embodiments, R^{44c} represents CH.

In some embodiments, R^{44d} represents -C(O)NHR^{44h}, -NHC(O)R^{44h}, -S(O)₂NHR^{44h}, -NHS(O)₂R^{44h},-S(O)₂R^{44h} or -P(O)(R^{44h})₂, wherein each R^{44h} is the same or different and each independently represents C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-). In some sub-embodiments, R^{44d} represents -P(O)(R^{44h})2, wherein each R^{44h} is the same or different and each independently represents C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-). In some sub-embodiments, R^{44d} represents -P(O)(CH₃)₂.

In some embodiments, p44c represents an integer of 0, 1, 2, 3 or 4. In some sub-embodiments, p44c represents an integer of 2.

In some embodiments, PBM represents a structure of Formula (PBM-44-1), (PBM-44-2), (PBM-44-3), (PBM-44-4) or (PBM-44-5):

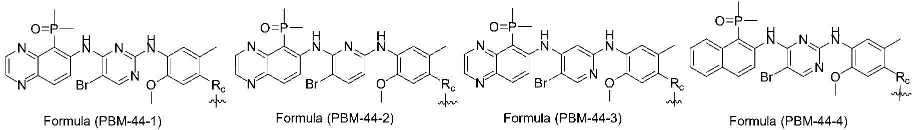

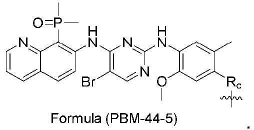

In some embodiments, PBM represents a small molecule ligand of AR.

In some embodiments, PBM represents the structure of Formula (PBM-6): 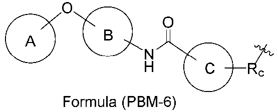
wherein ring A represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S). Ring A is optionally substituted with one or more (e.g., 1-10, 1-8 or 1-6, such as 1, 2, 3, 4, 5, 6, 7, 8, 9 or 10) R^{6a}, and each R^{6a} is the same or different and each independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₂₋₆ alkenyl (e.g., vinyl, propenyl, or butenyl), or C₂₋₆ alkynyl (e.g., ethynyl, propynyl or butynyl);
ring B represents C₃₋₁₅ cycloalkyl or 4- to 20-membered heterocyclyl (e.g., 4- to 20-membered heterocyclyl containing 1 to 4 heteroatoms selected from N, O and S), wherein the ring B is optionally substituted with one or more (e.g., 1-10, 1-8 or 1-6, such as 1, 2, 3, 4, 5, 6, 7, 8, 9 or 10) R^{6b}, and each R^{6b} is the same or different and each independently represents deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
ring C represents 5- to 20-membered heteroaryl (e.g., 5- to 20-membered monocyclic or bicyclic heteroaryl containing 1 to 4 heteroatoms selected from N, O and S), wherein ring C is optionally substituted with one or more (e.g., 1-10, 1-8 or 1-6, such as 1, 2, 3, 4, 5, 6, 7, 8, 9 or 10) R^{6c}, and each R^{6c} is the same or different and each independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, PBM represents the structure of Formula (PBM-6-1A): 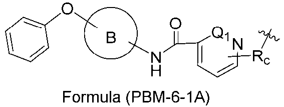
wherein the benzene ring in Formula (PBM-6-1A) is optionally substituted with one or more (e.g., 1-10, 1-8 or 1-6, such as 1, 2, 3, 4, 5, 6, 7, 8, 9 or 10) R^{6a}, and each R^{6a} is the same or different and each independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₂₋₆ alkenyl (e.g., vinyl, propenyl, or butenyl), or C₂₋₆ alkynyl (e.g., ethynyl, propynyl or butynyl);
ring B represents C₃₋₁₅ cycloalkyl or 4- to 20-membered heterocyclyl (e.g., 4- to 20-membered heterocyclyl containing 1 to 4 heteroatoms selected from N, O and S), wherein the ring B is optionally substituted with one or more (e.g., 1-10, 1-8 or 1-6, such as 1, 2, 3, 4, 5, 6, 7, 8, 9 or 10) R^{6b}, and each R^{6b} is the same or different and each independently represents deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
Q₁ represents N or CH; and
the heteroaryl group containing Q₁ and a nitrogen atom in Formula (PBM-6-1A) is optionally substituted with one or more (e.g., 1-3, such as 1, 2, or 3) R^{6c} substituents, and each R^{6c} is the same or different and each independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, ring B in each of Formulae (PBM-6) and (PBM-6-1A) each independently represents C₃₋₁₅ cycloalkyl or 4- to 20-membered heterocyclyl (e.g., 4- to 20-membered heterocyclyl containing 1 to 4 heteroatoms selected from N, O and S). Examples of C₃₋₁₅ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, 2,3-dihydro-1H-indenyl, spiro-cycloalkyl (e.g., C₅₋₁₅ spiro-cycloalkyl), adamantanyl, noradamantanyl, and norbornyl. The optional substituents of C₃₋₁₅ cycloalkyl are optionally selected from the group consisting of deuterium, halogen (e.g., fluorine, chlorine, bromine, or iodine), C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). Examples of 4- to 20-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl). The optional substituents of 4- to 20-membered heterocyclyl are optionally selected from the group consisting of deuterium, halogen (e.g., fluorine, chlorine, bromine, or iodine), C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, PBM represents a structure of Formulae (PBM-6-1), (PBM-6-2), (PBM-6-3) or (PBM-6-4):

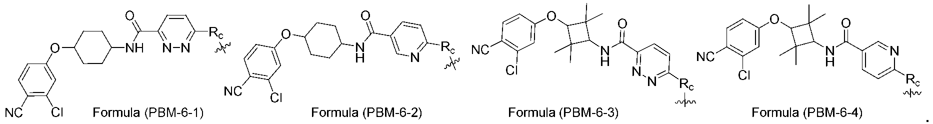

In some embodiments, PBM represents the structure of Formula (PBM-7): 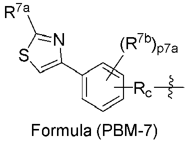
wherein R^{7a} represents 4- to 20-membered heterocyclyl (e.g., 4- to 20-membered heterocyclyl containing 1 to 4 heteroatoms selected from N, O and S), wherein the 4- to 20-membered heterocycly is optionally substituted with one or more (e.g., 1-10, 1-8 or 1-6, such as 1, 2, 3, 4, 5, 6, 7, 8, 9 or 10) R^{7c}, and each R^{7c} is the same or different and each independently represents deuterium, halogen (e.g., fluorine, chlorine, bromine, or iodine), hydroxy, cyano, amino, nitro, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
(R^{7b})ₚ₇ₐ indicates that the benzene ring to which it is attached is substituted with p7a R^{7b}, wherein each R^{7b} is the same or different and each independently represents deuterium, halogen (e.g., fluorine, chlorine, bromine, or iodine), C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), and p7a represents an integer of 1, 2, 3 or 4.

In some embodiments, R^{7a} represents 4- to 20-membered heterocyclyl (e.g., 4- to 20-membered heterocyclyl containing 1 to 4 heteroatoms selected from N, O and S). Examples of 4- to 20-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl). In some embodiments, the 4- to 20-membered heterocyclyl is optionally substituted with one or more (e.g., 1-10, 1-8 or 1-6, such as 1, 2, 3, 4, 5, 6, 7, 8, 9 or 10) R^{7c}, with each R^{7c} being the same or different and as defined above.

In some embodiments, PBM represents a structure of Formulae (PBM-7-1), (PBM-7-2), (PBM-7-3), or (PBM-7-4):

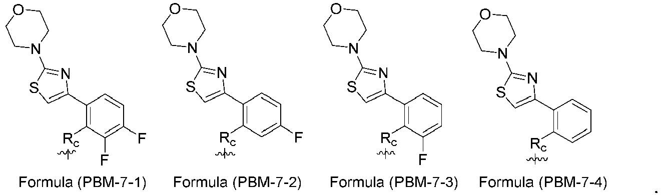

In some embodiments, PBM represents the structure of Formula (PBM-50): 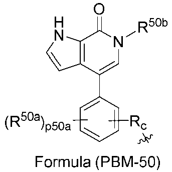
wherein (R^{50a})ₚ₅₀ₐ indicates that the benzene ring to which it is attached is substituted with p50a R^{50a}, and each R^{50a} is the same or different and each independently represents halogen (e.g., fluorine, chlorine, bromine, or iodine), hydroxy, mercapto, cyano, amino, nitro, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkyl-S(O)₂, halogenated C₁₋₆ alkyl-S(O)₂, halogenated C₁₋₆ alkyl-NHC(O), halogenated C₁₋₆ alkyl-C(O)NH, halogenated C₁₋₆ alkyl-NHS(O)₂, halogenated C₁₋₆ alkyl-S(O)₂NH, C₁₋₆ alkoxy (e.g., C₁₋₄ alkoxy, such as methoxy, CD₃-O-, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p50a represents an integer of 0, 1, 2, 3 or 4; and
R^{50b} represents hydrogen, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.).

In some embodiments, PBM represents the structure of Formula (PBM-50-1):

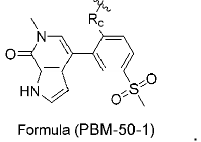

In some embodiments, PBM represents the structure of Formula (PBM-51): 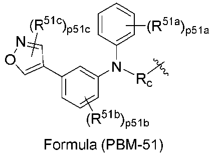
wherein (R^{51a})ₚ₅₁ₐ indicates that the benzene ring to which it is attached is substituted with p51a R^{51a}, and each R^{51a} is the same or different and each independently represents halogen (e.g., fluorine, chlorine, bromine, or iodine), hydroxy, mercapto, cyano, amino, nitro, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), optionally substituted C₃₋₁₅ cycloalkyl or optionally substituted 4- to 15-membered heterocyclyl containing from 1 to 4 heteroatoms selected from the group consisting of oxygen, nitrogen, and sulfur, and p51a represents an integer of 0, 1, 2, 3, 4 or 5;
(R^{51d})_{p51b} indicates that the benzene ring to which it is attached is substituted with p51b R^{51b}, and each R^{51d} is the same or different and each independently represents halogen (e.g., fluorine, chlorine, bromine, or iodine), hydroxy, mercapto, cyano, amino, nitro, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), optionally substituted C₃₋₁₅ cycloalkyl or optionally substituted 4- to 15-membered heterocyclyl containing from 1 to 4 heteroatoms selected from the group consisting of oxygen, nitrogen, and sulfur, and p51b represents an integer of 0, 1, 2, 3 or 4;
(R^{51a})_{p51c} indicates that the isoxazole ring to which it is attached is optionally substituted with p51c R^{51c}, and each R^{51c} is the same or different and each independently represents halogen (e.g., fluorine, chlorine, bromine, or iodine), hydroxy, mercapto, cyano, amino, nitro, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p51c represents an integer of 0, 1 or 2.

In some embodiments, R^{51a} and R^{51b} are the same or different and each independently represent optionally substituted C₃₋₁₅ cycloalkyl or optionally substituted 4- to 15-membered heterocyclyl containing from 1 to 4 heteroatoms selected from the group consisting of oxygen, nitrogen, and sulfur. Examples of C₃₋₁₅ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, 2,3-dihydro-1H-indenyl, spiro-cycloalkyl (e.g., C₅₋₁₅ spiro-cycloalkyl), adamantanyl, noradamantanyl, and norbornyl. The optional substituents of C₃₋₁₅ cycloalkyl are optionally selected from the group consisting of halogen (e.g., fluorine, chlorine, bromine, or iodine), hydroxy, mercapto, cyano, amino, nitro, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), and halogenated C₁₋₆ alkoxy (e.g., halogenated C₁₋₄ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). Examples of 4- to 15-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl). The optional substituents of 4- to 15-membered heterocyclyl are optionally selected from the group consisting of halogen (e.g., fluorine, chlorine, bromine, or iodine), hydroxy, mercapto, cyano, amino, nitro, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, PBM represents the structure of Formula (PBM-51-1):

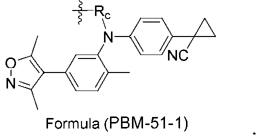

In some embodiments, PBM represents the structure of Formula (PBM-54): 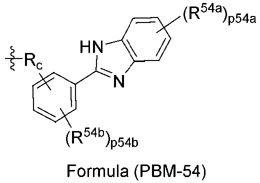
wherein (R^{54a})ₚ₅₄ₐ in Formula (PBM-54) represents p54a R^{54a} groups, wherein each R^{54a} is the same or different and each independently represents halogen (e.g., fluorine, chlorine, bromine, or iodine), hydroxy, mercapto, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂-etc.), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p54a represents an integer of 0, 1, 2, 3 or 4;
(R^{54b})_{p54b} indicates that the benzene ring to which it is attached is optionally substituted with p54b R^{54b}, wherein each R^{54b} is the same or different and each independently represents deuterium, halogen (e.g., fluorine, chlorine, bromine, or iodine), hydroxy, mercapto, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p54b represents an integer of 0, 1, 2, 3 or 4.

In some embodiments, PBM represents the structure of Formula (PBM-54-1):

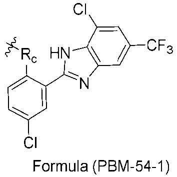

In some embodiments, PBM represents a small molecule ligand of BTK.

In some embodiments, PBM represents the structure of Formula (PBM-8): 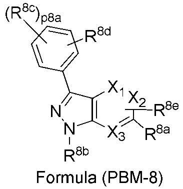
wherein X₁ in Formula (PBM-8) represents N or CR^{8f}, where R^{8f} represents hydrogen, deuterium or amino; and X₂ represents N or CR^{8g}, where R^{8g} represents hydrogen or a bond; and X₃ represents CH or N;
R^{8a} and R^{8b} each independently represent hydrogen or a bond;
(R^{8c})ₚ₈ₐ indicates that the benzene ring to which it is attached is substituted with p8a R^{8c}, and each R^{8c} is the same or different and each independently represents -O-aryl, -O-heteroaryl, 4- to 20-membered heterocyclyl, -C₁₋₃ alkylene-NHC(O)-heteroaryl, -C₁₋₃ alkylene-C(O)NH-heteroaryl, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterium, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogen (e.g., fluorine, chlorine, bromine, or iodine), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), wherein the 4- to 20-membered heterocyclyl, aryl, and heteroaryl are each independently optionally substituted with one or more (e.g., 1-5, such as 1, 2, 3, 4 or 5) R^{8h}, and each R^{8h} is the same or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p8a represents an integer of 1, 2, 3 or 4;
R^{8d} represents a bond, hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy);
R^{8c} represents hydrogen, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or amino;
wherein R^{8g}, R^{8a}, R^{8b} and R^{8d} are not simultaneously hydrogen, and only one of R^{8g}, R^{8a}, R^{8b} and R^{8d} represents a bond, wherein
   when R^{8a} represents a bond, the ring carbon atom connected to R^{8a} is directly bonded to R_{c}, X₂ represents CH or N, and R^{8b} is hydrogen, and R^{8d} is not a bond;
   when X₂ represents CR^{8g}, where R^{8g} represents a bond, the ring carbon atom connected to R^{8g} is directly bonded to R_{c}, and R^{8a} and R^{8b} are hydrogen, and R^{8d} is not a bond;
   when R^{8b} represents a bond, the ring nitrogen atom connected to R^{8b} is directly bonded to R_{c}, X₂ represents N or CH, and R^{8a} is hydrogen, R^{8d} is not a bond; or
   when R^{8d} represents a bond, the ring carbon atom connected to R^{8d} is directly bonded to R_{c}, and X₂ represents N or CH, and R^{8a} and R^{8b} are hydrogen.

In some embodiments, R^{8c} in Formula (PBM-8) represents -O-aryl, -O-heteroaryl, -C₁₋₃ alkylene-NHC(O)-heteroaryl, or -C₁₋₃ alkylene-C(O)NH-heteroaryl, wherein the aryl may be optionally substituted C₆₋₁₀ aryl, including but not limited to e.g., phenyl or naphthyl, and the heteroaryl may be optionally substituted 5- to 14-membered monocyclic, bicyclic, or polycyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, R^{8c} represents -O-phenyl or -O-naphthyl, wherein the phenyl and naphthyl are each optionally substituted with one or more (e.g., 1-5, such as 1, 2, 3, 4 or 5) R^{8h}, and each R^{8h} is the same or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, R^{8c} represents -O-heteroaryl, -C₁₋₃ alkylene-NHC(O)-heteroaryl, or -C₁₋₃ alkylene-C(O)NH-heteroaryl, wherein the heteroaryl is optionally substituted with one or more (e.g., 1-5, such as 1, 2, 3, 4 or 5) R^{8h}, and each R^{8h} is the same or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, or imidazo[2,1-b]thiazolyl.

In some embodiments, R^{8c} in Formula (PBM-8) represents 4- to 20-membered (e.g., 4- to 15-membered, 4- to 12-membered, 4- to 10-membered, 4- to 7-membered, or 4- to 6-membered) monocyclic, bicyclic, or polycyclic heterocyclyl, wherein the 4- to 20-membered monocyclic, bicyclic, or polycyclic heterocyclyl may be optionally substituted. In a sub-embodiment, R^{8c} represents 4- to 20-membered monocyclic, bicyclic, or polycyclic heterocyclyl, which is optionally substituted with one or more (e.g., 1-5, such as 1, 2, 3, 4 or 5) R^{8h}, and each R^{8h} is the same or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of 4- to 20-membered monocyclic, bicyclic, or polycyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl).

In some embodiments, R^{8b} represents a bond; R^{8a} is hydrogen; R^{8d} is not a bond; and X₂ represents N or CH.

In some embodiments, R^{8b} represents a bond; R^{8a} is hydrogen; R^{8d} is not a bond; and X₂ represents N; X₁ represents CR^{8f}, where R^{8f} is hydrogen or amino; and X₃ represents N.

In some embodiments, R^{8b} represents a bond; R^{8a} is hydrogen; R^{8d} is not a bond; and X₂ represents N; X₁ represents CR^{8f}, where R^{8f} is hydrogen or amino; and X₃ represents CH.

In some embodiments, R^{8b} represents a bond; R^{8a} is hydrogen; R^{8d} is not a bond; and X₂ represents N; X₁ represents N; and X₃ represents CH.

In some embodiments, R^{8b} represents a bond; R^{8a} is hydrogen; R^{8d} is not a bond; and X₂ represents N; X₁ represents N; and X₃ represents N.

In some embodiments, R^{8b} represents a bond; R^{8a} is hydrogen; R^{8d} is not a bond; and X₂ represents CH; X₁ represents CR^{8f}, where R^{8f} is hydrogen or amino; and X₃ represents N.

In some embodiments, R^{8b} represents a bond; R^{8a} is hydrogen; R^{8d} is not a bond; and X₂ represents CH; X₁ represents CR^{8f}, where R^{8f} is hydrogen or amino; and X₃ represents CH.

In some embodiments, R^{8b} represents a bond; R^{8a} is hydrogen; R^{8d} is not a bond; and X₂ represents CH; X₁ represents N; and X₃ represents CH.

In some embodiments, R^{8b} represents a bond; R^{8a} is hydrogen; R^{8d} is not a bond; and X₂ represents CH; X₁ represents N; and X₃ represents N.

In some embodiments, when R^{8a} represents a bond, the ring carbon atom connected to R^{8a} is directly bonded to R_{c} ; X₂ represents CH or N; and R^{8b} is hydrogen; and R^{8d} is not a bond.

In some embodiments, R^{8a} represents a bond; R^{8b} is hydrogen; R^{8d} is not a bond; and X₂ represents CH; X₁ represents N; and X₃ represents N.

In some embodiments, R^{8a} represents a bond; R^{8b} is hydrogen; R^{8d} is not a bond; and X₂ represents CH; X₁ represents N; and X₃ represents CH.

In some embodiments, R^{8a} represents a bond; R^{8b} is hydrogen; R^{8d} is not a bond; and X₂ represents CH; X₁ represents CR^{8f}, where R^{8f} is hydrogen or amino; and X₃ represents N.

In some embodiments, R^{8a} represents a bond; R^{8b} is hydrogen; R^{8d} is not a bond; and X₂ represents CH; X₁ represents CR^{8f}, where R^{8f} is hydrogen or amino; and X₃ represents CH.

In some embodiments, R^{8a} represents a bond; R^{8b} is hydrogen; R^{8d} is not a bond; and X₂ represents N; X₁ represents CR^{8f}, where R^{8f} is hydrogen or amino; and X₃ represents N.

In some embodiments, R^{8a} represents a bond; R^{8b} is hydrogen; R^{8d} is not a bond; and X₂ represents N; X₁ represents CR^{8f}, where R^{8f} is hydrogen or amino; and X₃ represents CH.

In some embodiments, R^{8a} represents a bond, R^{8b} is hydrogen; R^{8d} is not a bond; and X₂ represents N; X₁ represents N; and X₃ represents CH.

In some embodiments, R^{8a} represents a bond; R^{8b} is hydrogen; R^{8d} is not a bond; and X₂ represents N; X₁ represents N; and X₃ represents N.

In some embodiments, when X₂ represents CR^{8g}, where R^{8g} represents a bond, the ring carbon atom connected to R^{8g} is directly bonded to R_{c} , and R^{8a} and R^{8b} are both hydrogen, and R^{8d} is not a bond.

In some embodiments, when X₂ represents CR^{8g}, where R^{8g} represents a bond, the ring carbon atom connected to R^{8g} is directly bonded to R_{c} , and R^{8a} and R^{8b} are both hydrogen, and R^{8d} is not a bond, and X₁ represents N, and X₃ represents CH.

In some embodiments, when X₂ represents CR^{8g}, where R^{8g} represents a bond, the ring carbon atom connected to R^{8g} is directly bonded to R_{c} , and R^{8a} and R^{8b} are both hydrogen, and R^{8d} is not a bond, and X₁ represents N, and X₃ represents N.

In some embodiments, when X₂ represents CR^{8g}, where R^{8g} represents a bond, the ring carbon atom connected to R^{8g} is directly bonded to R_{c} , and R^{8a} and R^{8b} are both hydrogen, and R^{8d} is not a bond, and X₁ represents CR^{8f}, where R^{8f} is hydrogen or amino, and X₃ represents CH.

In some embodiments, when X₂ represents CR^{8g}, where R^{8g} represents a bond, the ring carbon atom connected to R^{8g} is directly bonded to R_{c} , and R^{8a} and R^{8b} are both hydrogen, and R^{8d} is not a bond, and X₁ represents CR^{8f}, where R^{8f} is hydrogen or amino, and X₃ represents N.

In some embodiments, when R^{8d} represents a bond, the ring carbon atom connected to R^{8d} is directly bonded to R_{c} , and X₂ represents N or CH, and R^{8a} and R^{8b} are both hydrogen.

In some embodiments, when R^{8d} represents a bond, the ring carbon atom connected to R^{8d} is directly bonded to R_{c} , and R^{8a} and R^{8b} are both hydrogen, and X₂ represents N, and X₁ represents N, and X₃ represents CH.

In some embodiments, when R^{8d} represents a bond, the ring carbon atom connected to R^{8d} is directly bonded to R_{c} , and R^{8a} and R^{8b} are both hydrogen, and X₂ represents N, and X₁ represents N, and X₃ represents N.

In some embodiments, when R^{8d} represents a bond, the ring carbon atom connected to R^{8d} is directly bonded to R_{c} , and R^{8a} and R^{8b} are both hydrogen, and X₂ represents N, and X₁ represents CR^{8f}, where R^{8f} is hydrogen or amino, and X₃ represents N.

In some embodiments, when R^{8d} represents a bond, the ring carbon atom connected to R^{8d} is directly bonded to R_{c} , and R^{8a} and R^{8b} are both hydrogen, and X₂ represents N, and X₁ represents CR^{8f}, where R^{8f} is hydrogen or amino, and X₃ represents CH.

In some embodiments, when R^{8d} represents a bond, the ring carbon atom connected to R^{8d} is directly bonded to R_{c} , and R^{8a} and R^{8b} are both hydrogen, and X₂ represents CH, and X₁ represents N, and X₃ represents CH.

In some embodiments, when R^{8d} represents a bond, the ring carbon atom connected to R^{8d} is directly bonded to R_{c} , and R^{8a} and R^{8b} are both hydrogen, and X₂ represents CH, and X₁ represents N, and X₃ represents N.

In some embodiments, when R^{8d} represents a bond, the ring carbon atom connected to R^{8d} is directly bonded to R_{c} , and R^{8a} and R^{8b} are both hydrogen, and X₂ represents CH, and X₁ represents CR^{8f}, where R^{8f} is hydrogen or amino, and X₃ represents CH.

In some embodiments, when R^{8d} represents a bond, the ring carbon atom connected to R^{8d} is directly bonded to R_{c} , and R^{8a} and R^{8b} are both hydrogen, and X₂ represents CH, and X₁ represents CR^{8f}, where R^{8f} is hydrogen or amino, and X₃ represents N.

In some embodiments, PBM represents the structure of Formula (PBM-8-1A): 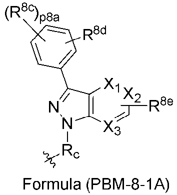
wherein X₁ in Formula (PBM-8-1A) represents N or CR^{8f}, where R^{8f} is hydrogen or amino, and X₂ represents N or CH, and X₃ represents CH or N;
(R^{8c})ₚ₈ₐ in Formula (PBM-8-1A) indicates that the benzene ring to which it is attached is substituted with p8a R^{8c}, and each R^{8c} is the same or different and each independently represents -O-aryl, -O-heteroaryl, 4- to 20-membered heterocyclyl, -C₁₋₃ alkylene-NHC(O)-heteroaryl, or -C₁₋₃ alkylene-C(O)NH-heteroaryl, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterium, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogen (e.g., fluorine, chlorine, bromine, or iodine), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), wherein the 4- to 20-membered heterocyclyl, aryl, and heteroaryl are each independently optionally substituted with one or more (e.g., 1-5, such as 1, 2, 3, 4 or 5) R^{8h}, with each R^{8h} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p8a represents an integer of 1, 2, 3 or 4;
R^{8d} represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkoxy (e.g., halogenated C₁₋₄ alkoxy, such as F₃C-O-, FCH₂-O-, F₂CHO-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy); e.g., R^{8d} represents hydrogen or methyl; and
R^{8c} represents hydrogen, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or amino.

In some embodiments, R^{8c} in Formula (PBM-8-1A) represents -O-aryl, -O-heteroaryl, -C₁₋₃ alkylene-NHC(O)-heteroaryl, or -C₁₋₃ alkylene-C(O)NH-heteroaryl, wherein the aryl may be optionally substituted C₆₋₁₀ aryl, including but not limited to e.g., phenyl or naphthyl, and the heteroaryl may be optionally substituted 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, R^{8c} represents -O-phenyl or -O-naphthyl, wherein the phenyl and naphthyl are each optionally substituted with one or more (e.g., 1-5, such as 1, 2, 3, 4 or 5) R^{8h}, and each R^{8h} is the same or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, R^{8c} represents -O-heteroaryl, -C₁₋₃ alkylene-NHC(O)-heteroaryl, or -C₁₋₃ alkylene-C(O)NH-heteroaryl, wherein the heteroaryl is optionally substituted with one or more (e.g., 1-5, such as 1, 2, 3, 4 or 5) R^{8h}, and each R^{8h} is the same or different and independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, or imidazo[2,1-b]thiazolyl.

In some embodiments, R^{8c} in Formula (PBM-8-1A) represents 4- to 20-membered (e.g., 4- to 15-membered, 4- to 12-membered, 4- to 10-membered, 4- to 7-membered, or 4- to 6-membered) monocyclic, bicyclic, or polycyclic heterocyclyl, wherein the 4- to 20-membered monocyclic, bicyclic, or polycyclic heterocyclyl may be optionally substituted. In a sub-embodiment, R^{8c} represents 4- to 20-membered monocyclic, bicyclic, or polycyclic heterocyclyl, which is optionally substituted with one or more (e.g., 1-5, such as 1, 2, 3, 4 or 5) R^{8h}, and each R^{8h} is the same or different and independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of 4- to 20-membered monocyclic, bicyclic, or polycyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl).

In some embodiments, X₁ in Formula (PBM-8-1A) represents N; X₂ represents N; and X₃ represents CH.

In some embodiments, X₁ in Formula (PBM-8-1A) represents N; X₂ represents N; and X₃ represents N.

In some embodiments, X₁ in Formula (PBM-8-1A) represents N; X₂ represents CH; and X₃ represents CH.

In some embodiments, X₁ in Formula (PBM-8-1A) represents N; X₂ represents CH; and X₃ represents N.

In some embodiments, X₁ in Formula (PBM-8-1A) represents CR^{8f}, where R^{8f} is hydrogen or amino; X₂ represents N; and X₃ represents N.

In some embodiments, X₁ in Formula (PBM-8-1A) represents CR^{8f}, where R^{8f} is hydrogen or amino; X₂ represents N; and X₃ represents CH.

In some embodiments, X₁ in Formula (PBM-8-1A) represents CR^{8f}, where R^{8f} is hydrogen or amino; X₂ represents CH; and X₃ represents N.

In some embodiments, X₁ in Formula (PBM-8-1A) represents CR^{8f}, where R^{8f} is hydrogen or amino; X₂ represents CH; and X₃ represents CH.

In some embodiments, PBM represents the structure of Formula (PBM-8-1):

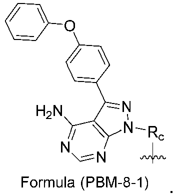

In some embodiments, PBM represents the structure of Formula (PBM-8-1B): 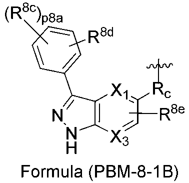
wherein X₁ in Formula (PBM-8-1B) represents N or CR^{8f}, where R^{8f} is hydrogen or amino, and X₃ represents CH or N;
(R^{8c})ₚ₈ₐ in Formula (PBM-8-1B) indicates that the benzene ring to which it is attached is substituted with p8a R^{8c}, with each R^{8c} being the same or different and each independently representing -O-aryl, -O-heteroaryl, 4- to 20-membered heterocyclyl, -C₁₋₃ alkylene-NHC(O)-heteroaryl, or -C₁₋₃ alkylene-C(O)NH-heteroaryl, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterium, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogen (e.g., fluorine, chlorine, bromine, or iodine), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), wherein the 4- to 20-membered heterocyclyl, aryl, and heteroaryl are each independently optionally substituted with one or more (e.g., 1-5, such as 1, 2, 3, 4 or 5) R^{8h}, with each R^{8h} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-);
p8a represents an integer of 1, 2, 3 or 4;
R^{8d} represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkoxy (e.g., halogenated C₁₋₄ alkoxy, such as F₃C-O-, FCH₂-O-, F₂CHO-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), e.g., R^{8d} represents hydrogen or methyl; and
R^{8c} represents hydrogen, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or amino.

In some embodiments, R^{8c} in Formula (PBM-8-1B) represents -O-aryl, -O-heteroaryl, -C₁₋₃ alkylene-NHC(O)-heteroaryl, or -C₁₋₃ alkylene-C(O)NH-heteroaryl, wherein the aryl may be optionally substituted C₆₋₁₀ aryl, including but not limited to e.g., phenyl or naphthyl, and the heteroaryl may be optionally substituted 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, R^{8c} represents -O-phenyl or -O-naphthyl, wherein the phenyl and naphthyl are each optionally substituted with one or more (e.g., 1-5, such as 1, 2, 3, 4 or 5) R^{8h}, and each R^{8h} is the same or different and independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, R^{8c} represents -O-heteroaryl, -C₁₋₃ alkylene-NHC(O)-heteroaryl, or -C₁₋₃ alkylene-C(O)NH-heteroaryl, wherein the heteroaryl is optionally substituted with one or more (e.g., 1-5, such as 1, 2, 3, 4 or 5) R^{8h}, and each R^{8h} is the same or different and independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, or imidazo[2,1-b]thiazolyl.

In some embodiments, R^{8c} in Formula (PBM-8-1B) represents 4- to 20-membered (e.g., 4- to 15-membered, 4- to 12-membered, 4- to 10-membered, 4- to 7-membered, or 4- to 6-membered) monocyclic, bicyclic, or polycyclic heterocyclyl, wherein the 4- to 20-membered monocyclic, bicyclic, or polycyclic heterocyclyl may be optionally substituted. In a sub-embodiment, R^{8c} represents 4- to 20-membered monocyclic, bicyclic, or polycyclic heterocyclyl, which is optionally substituted with one or more (e.g., 1-5, such as 1, 2, 3, 4 or 5) R^{8h}, and each R^{8h} is the same or different and independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of 4- to 20-membered monocyclic, bicyclic, or polycyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl).

In some embodiments, X₁ in Formula (PBM-8-1B) represents N, and X₃ represents CH.

In some embodiments, X₁ in Formula (PBM-8-1B) represents N, and X₃ represents N.

In some embodiments, X₁ in Formula (PBM-8-1B) represents CR^{8f}, where R^{8f} is hydrogen or amino, and X₃ represents CH.

In some embodiments, X₁ in Formula (PBM-8-1B) represents CR^{8f}, where R^{8f} is hydrogen or amino, and X₃ represents N.

In some embodiments, PBM represents the structure of Formula (PBM-8-2):

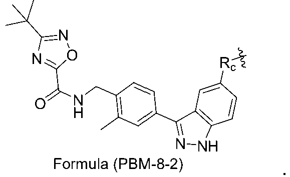

In some embodiments, PBM represents the structure of Formula (PBM-41): 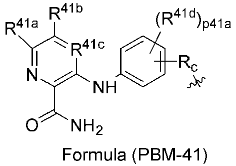
wherein R^{41a} represents hydrogen, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CHO-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₁₅ cycloalkyl (e.g., optionally substituted C₃₋₁₁ cycloalkyl) or optionally substituted 4- to 15-membered nitrogen-containing heterocyclyl;
R^{41b} represents hydrogen, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₁₅ cycloalkyl (e.g., optionally substituted C₃₋₁₁ cycloalkyl), optionally substituted 4- to 15-membered nitrogen-containing heterocyclyl or NR^{41c}R^{41f}, where R^{41e} and R^{41f} are the same or different and each independently represent hydrogen, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or optionally substituted 4- to 15-membered heterocyclyl;
R^{41c} represents N or CH;
(R^{41d})ₚ₄₁ₐ indicates that the benzene ring to which it is attached is optionally substituted with p41a R^{41d}, with each R^{41d} being the same or different and each independently representing hydroxy, amino, cyano, halogen (e.g., fluorine, chlorine, bromine, or iodine), C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CHO-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p41a represents an integer of 0, 1, 2, 3 or 4.

In some embodiments, R^{41a} represents C₁₋₆ alkyl, such as, but not limited to, methyl, ethyl, propyl, isopropyl, butyl, sec-butyl, tert-butyl.

In some embodiments, R^{41a} represents hydrogen.

In some embodiments, R^{41a} represents optionally substituted C₃₋₁₅ cycloalkyl (e.g., optionally substituted C₃₋₁₁ cycloalkyl) or optionally substituted 4- to 15-membered nitrogen-containing heterocyclyl. Examples of C₃₋₁₁ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, 2,3-dihydro-1H-indenyl, C₅₋₁₁ spiro-cycloalkyl, adamantanyl, noradamantanyl, and norbornyl. The optional substituents of C₃₋₁₁ cycloalkyl are optionally selected from the group consisting of deuterium, hydroxy, amino, mercapto, nitro, halogen, cyano, C₁₋₆ alkyl, halogenated C₁₋₆ alkyl, deuterated C₁₋₆ alkyl, C₃₋₆ cycloalkyl, C₁₋₆ alkoxy, halogenated C₁₋₆ alkoxy, 2-oxoimidazolidinyl which is optionally substituted by C₁₋₃ alkyl or halogen (e.g., 3-methyl-2-oxoimidazolidin-1-yl) or any combination thereof. In some embodiments, the optionally substituted 4- to 15-membered nitrogen-containing heterocyclyl is, for example, an optionally substituted 4- to 8-membered (e.g., 4- to 7-membered, 4- to 6-membered, 5- to 7-membered, or 5- to 6-membered) monocyclic or bicyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 15-membered nitrogen-containing heterocyclyl is optionally substituted with a substituent selected from the group consisting of deuterium, hydroxy, amino, mercapto, nitro, halogen, cyano, C₁₋₆ alkyl, halogenated C₁₋₆ alkyl, deuterated C₁₋₆ alkyl, C₃₋₆ cycloalkyl, C₁₋₆ alkoxy, halogenated C₁₋₆ alkoxy, 2-oxoimidazolidinyl which is optionally substituted by C₁₋₃ alkyl or halogen (e.g., 3-methyl-2-oxoimidazolidin-1-yl) or any combination thereof.

In some embodiments, R^{41b} represents optionally substituted C₃₋₁₅ cycloalkyl (e.g., optionally substituted C₃₋₁₁ cycloalkyl) or optionally substituted 4- to 15-membered nitrogen-containing heterocyclyl. Examples of C₃₋₁₁ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, 2,3-dihydro-1H-indenyl, C₅₋₁₁ spiro-cycloalkyl, adamantanyl, noradamantanyl, and norbornyl. The optional substituents of C₃₋₁₁ cycloalkyl are optionally selected from the group consisting of deuterium, hydroxy, amino, mercapto, nitro, halogen, cyano, C₁₋₆ alkyl, halogenated C₁₋₆ alkyl, deuterated C₁₋₆ alkyl, C₃₋₆ cycloalkyl, C₁₋₆ alkoxy, halogenated C₁₋₆ alkoxy, 2-oxoimidazolidinyl which is optionally substituted by C₁₋₃ alkyl or halogen (e.g., 3-methyl-2-oxoimidazolidin-1-yl) or any combination thereof. In some embodiments, the optionally substituted 4- to 15-membered nitrogen-containing heterocyclyl is, for example, an optionally substituted 4- to 15-membered (e.g., 4- to 12-membered, 4- to 10-membered, 4- to 7-membered, 4- to 6-membered, 5- to 7-membered, or 5- to 6-membered) monocyclic or bicyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 15-membered nitrogen-containing heterocyclyl is optionally substituted with a substituent selected from the group consisting of deuterium, hydroxy, amino, mercapto, nitro, halogen, cyano, C₁₋₆ alkyl, halogenated C₁₋₆ alkyl, deuterated C₁₋₆ alkyl, C₃₋₆ cycloalkyl, C₁₋₆ alkoxy, halogenated C₁₋₆ alkoxy, 2-oxoimidazolidinyl which is optionally substituted by C₁₋₃ alkyl or halogen (e.g., 3-methyl-2-oxoimidazolidin-1-yl) or any combination thereof. In some embodiments, R^{41b} represents piperidinyl which is optionally with 3-methyl-2-oxoimidazolidin-1-yl.

In some embodiments, R^{41b} represents NR^{41e}R^{41f}, wherein R^{41e} and R^{41f} are the same or different and each independently represent hydrogen, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or optionally substituted 4- to 15-membered heterocyclyl. In some embodiments, the optionally substituted 4- to 15-membered heterocyclyl is, for example, an optionally substituted 4- to 15-membered (e.g., 4- to 12-membered, 4- to 10-membered, 4- to 7-membered, 4- to 6-membered, 5- to 7-membered, or 5- to 6-membered) monocyclic or bicyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl). In some embodiments, the 4- to 15-membered heterocyclyl is optionally substituted with a substituent selected from the group consisting of deuterium, hydroxy, amino, mercapto, nitro, halogen, cyano, C₁₋₆ alkyl, halogenated C₁₋₆ alkyl, deuterated C₁₋₆ alkyl, C₃₋₆ cycloalkyl, C₁₋₆ alkoxy, halogenated C₁₋₆ alkoxy, 2-oxoimidazolidinyl which is optionally substituted by C₁₋₃ alkyl or halogen (e.g., 3-methyl-2-oxoimidazolidin-1-yl) or any combination thereof. In some embodiments, R^{41b} represents NR^{41e}R^{41f}, where R^{41e} represents hydrogen, and R^{41f} represents tetrahydropyranyl.

In some embodiments, p41a represents an integer of 0.

In some embodiments, p41a represents 1, and R^{41d} represents methoxy, methyl or fluorine.

In some embodiments, PBM represents a structure of Formulae (PBM-41-2) or (PBM-41-3):

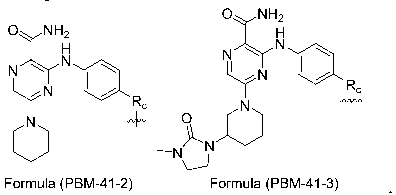

In some embodiments, PBM represents a structure of Formulae (PBM-42-1A), (PBM-42-1B), or (PBM-42-1C): 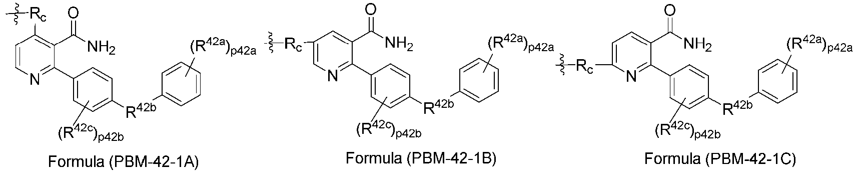 wherein in each of Formulae (PBM-42-1A), (PBM-42-1B), and (PBM-42-1C),
(R^{42a})ₚ₄₂ₐ indicates that the benzene ring to which it is attached is optionally substituted with p42a R^{42a}, with each R^{42a} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p42a represents an integer of 0, 1, 2, 3, 4 or 5;
R^{42b} represents O, S, C₁₋₃ alkylene, or halogenated C₁₋₃ alkylene;
(R^{42c})_{p42b} indicates that the benzene ring to which it is attached is optionally substituted with p42b R^{42c}, with each R^{42c} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p42b represents an integer of 0, 1, 2, 3 or 4.

In some embodiments, R^{42b} in Formula (PBM-42) represents O.

In some embodiments, R^{42b} in Formula (PBM-42) represents S.

In some embodiments, R^{42b} in Formula (PBM-42) represents C₁₋₃ alkylene. In some sub-embodiments, R^{42b} represents methylene, ethylene, or propylene.

In some embodiments, R^{42b} in Formula (PBM-42) represents halogenated C₁₋₃ alkylene. In some sub-embodiments, R^{42b} represents fluoromethylene, or difluoromethylene.

In some embodiments, PBM represents the structure of Formula (PBM-42-1):

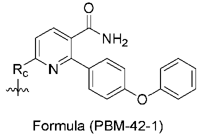

In some embodiments, PBM represents a small molecule ligand of CDK4/6.

In some embodiments, PBM represents a structure of Formulae (PBM-9-1A), (PBM-9-1B), (PBM-9-1C) or (PBM-9-1D): 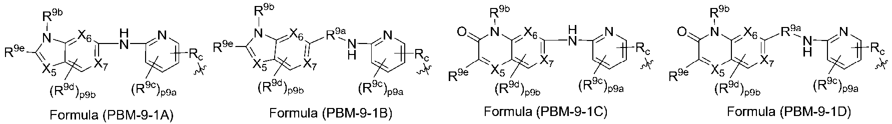
wherein each R^{9e} independently represents deuterium, hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), -C(O)-C₁₋₆ alkyl (e.g., -C(O)-C₁₋₅ alkyl or -C(O)-C₁₋₃ alkyl, such as -C(O)-CH₃, -C(O)-CH₂CH₃), -C(O)-deuterated C₁₋₆ alkyl (e.g., -C(O)-deuterated C₁₋₅ alkyl or -C(O)-deuterated C₁₋₃ alkyl, such as -C(O)-CD₃) or -C(0)NR^{9f}R^{9g}, where R^{9f} and R^{9g} are the same or different and each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl) or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), and R^{9f} and R^{9g} are not simultaneously hydrogen;
each X₅ independently represents N or CR^{9h}, where R^{9h} represents hydrogen, deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
X₆ and X₇ each independently represent CH or N;
R^{9a} in each of Formulae (PBM-9-1B) and (PBM-9-1D) each independently represents an optionally substituted heteroarylene (e.g., optionally substituted 5- to 20-membered monocyclic, bicyclic, or polycyclic aromatic ring group containing one or more (e.g., 1-6, 1-5, 1-4, or 1-3) heteroatoms independently selected from the group consisting of oxygen, nitrogen, and sulfur), wherein the heteroarylene is optionally substituted with 1-2 substituents each independently selected from the group consisting of halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof;
each R^{9b} independently represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or optionally substituted C₃₋₁₂ cycloalkyl (e.g., cyclopropyl, cyclobutyl, cyclopentyl, cyclopentenyl, cyclohexyl, cyclohexenyl, or cycloheptyl, which are optionally substituted with a substituent selected from the group consisting of e.g., halogen, C₁₋₆ alkyl, halogenated C₁₋₆ alkyl or deuterated C₁₋₆ alkyl);
each (R^{9c})ₚ₉ₐ independently indicates that the pyridine ring to which it is attached is optionally substituted with p9a R^{9c}, with each R^{9c} being the same or different and each independently representing deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p9a represents an integer of 0, 1, 2 or 3; and
each (R^{9d})_{p9b} independently indicates that the nitrogen-containing bicyclic heteroaryl ring to which it is attached is optionally substituted with p9b R^{9d}, with each R^{9d} being the same or different and each independently representing deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p9b represents an integer of 0 or 1.

In some embodiments, R^{9a} in each of Formulae (PBM-9-1B) and (PBM-9-1D) independently represents optionally substituted heteroarylene, e.g., optionally substituted 5- to 14-membered monocyclic, bicyclic, or polycyclic heteroarylene group containing one or more (e.g., 1-6, 1-5, 1-4, or 1-3) heteroatoms independently selected from the group consisting of oxygen, nitrogen, and sulfur. Examples of heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-furo[3,2-b]pyrrolylene, pyrrolo[2,1-b]thiazolylene, and imidazo[2,1-b]thiazolylene. The heteroarylene is optionally substituted with one or more (e.g., 1-4, such as 1, 2, 3, or 4) substituents selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, PBM represents a structure of Formulae (PBM-9-1), (PBM-9-2) or (PBM-9-3):

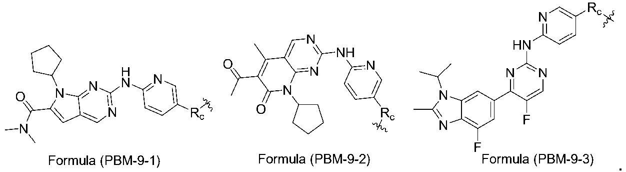

In some embodiments, PBM represents a small molecule ligand of ALK.

In some embodiments, PBM represents the structure of Formula (PBM-10): 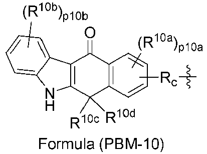
wherein (R^{10a})ₚ₁₀ₐ in Formula (PBM-10) indicates that the benzene ring to which it is attached is optionally substituted with p10a R^{10a}, wherein each R^{10a} independently represents deuterium, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), and p10a represents an integer of 0, 1, 2 or 3; and
(R^{10b})_{p10b} indicates that the benzene ring to which it is attached is optionally substituted with p10b R^{10b}, wherein each R^{10b} independently represents deuterium, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), and p10b represents an integer of 0, 1, 2, 3 or 4; and
R^{10e} and R^{10d} are the same or different and each independently represent deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogen (e.g., fluorine, chlorine, bromine, or iodine), halogenated C₁₋₆ alkyl (e.g., fluorine, chlorine, bromine, or iodine), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, PBM represents the structure of Formula (PBM-10-1):

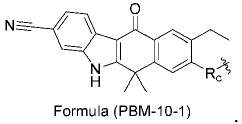

In some embodiments, PBM represents the structure of Formula (PBM-11): 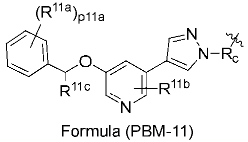
wherein (R^{11a})ₚ₁₁ₐ in Formula (PBM-11) indicates that the benzene ring to which it is attached is substituted with p11a R^{11a}, with each R^{11a} being the same or different and each independently representing deuterium, halogen (e.g., fluorine, chlorine, bromine, or iodine), hydroxy, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.);
p11a represents an integer of 1, 2, 3, 4 or 5; or
R^{11b} represents NR^{11d}R^{11c}, wherein R^{11d} and R^{11c} are the same or different and each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.); and
R^{11c} represents deuterium, hydrogen, halogen (e.g., fluorine, chlorine, bromine, or iodine), hydroxy, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.).

In some embodiments, PBM represents a structure of Formulae (PBM-11-1), (PBM-11-2) or (PBM-11-3):

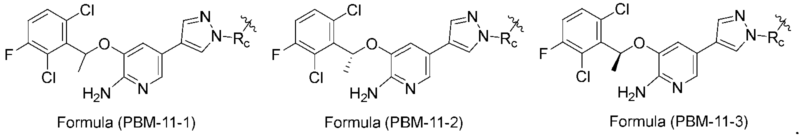

In some embodiments, PBM represents the structure of Formula (PBM-12): 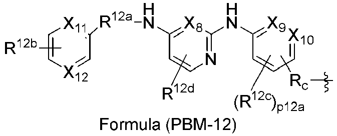
wherein X₈, X₉, X₁₀, X₁₁, and X₁₂ in Formula (PBM-12) are the same or different and each independently represent N or CH;
R^{12a} represents a bond or optionally substituted methylene (e.g., halogenated methylene);
R^{12b} represents -C(O)N(R^{12f})R^{12c}, -N(R^{12f})C(O)R^{12c}, -S(O)₂N(R^{12f})R^{12c}, -N(R^{12f})S(O)₂R^{12c}, -S(O)₂R^{12c} or - P(O)(R^{12c})₂, wherein each R^{12c} is the same or different and each independently represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), and R^{12f} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R^{12c})ₚ₁₂ₐ in Formula (PBM-12) indicates that the 6-membered ring containing X₉ and X₁₀ to which it is attached is optionally substituted with p12a R^{12c}, with each R^{12c} being the same or different and each independently representing deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p12a represents an integer of 0, 1, 2, 3 or 4; and
R^{12d} represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{12a} in Formula (PBM-12) represents a bond. In other words, the 6-membered ring containing X₁₁ and X₁₂ in Formula (PBM-12) is directly bonded to the -NH- group.

In some embodiments, R^{12a} in Formula (PBM-12) represents optionally substituted methylene (e.g., halogenated methylene).

In some embodiments, PBM represents the structure of Formula (PBM-12-1A): 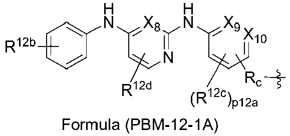
wherein X₈, X₉, and X₁₀ are the same or different and each independently represent N or CH;
R^{12b} represents -C(O)N(R^{12f})R^{12c}, -N(R^{12f})C(O)R^{12c}, -S(O)₂N(R^{12f})R^{12c}, -N(R^{12f})S(O)₂R^{12c}, -S(O)₂R^{12c} or - P(O)(R^{12c})₂, wherein each R^{12c} is the same or different and each independently represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), and R^{12f} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R^{12c})ₚ₁₂ₐ indicates that the 6-membered ring containing X₉ and X₁₀ to which it is attached is optionally substituted with p12a R^{12c} , with each R^{12c} being the same or different and each independently representing deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p12a represents an integer of 0, 1, 2, 3 or 4; and
R^{12d} represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of Formula (PBM-12-1B): 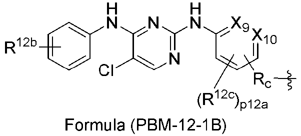
wherein X₉ and X₁₀ are the same or different and each independently represent N or CH;
R^{12b} represents -C(O)N(R^{12f})R^{12c}, -N(R^{12f})C(O)R^{12c}, -S(O)₂N(R^{12f})R^{12c}, -N(R^{12f})S(O)₂R^{12c}, -S(O)₂R^{12c} or - P(O)(R^{12c})₂, wherein each R^{12c} is the same or different and each independently represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), and R^{12f} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R^{12c})ₚ₁₂ₐ indicates that the 6-membered ring containing X₉ and X₁₀ to which it is attached is optionally substituted with p12a R^{12c} , with each R^{12c} being the same or different and each independently representing deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p12a represents an integer of 0, 1, 2, 3 or 4.

In some embodiments, PBM represents a structure of Formulae (PBM-12-1) or (PBM-12-2):

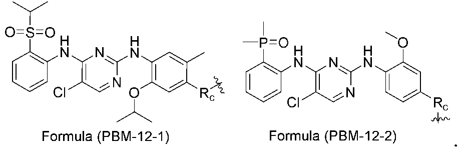

In some embodiments, PBM represents a small molecule ligand of RET.

In some embodiments, PBM represents the structure of Formula (PBM-12-1C): 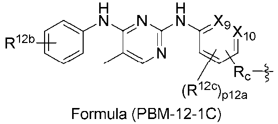
wherein X₉ and X₁₀ are the same or different and each independently represent N or CH;
R^{12b} represents -C(O)N(R^{12f})R^{12c}, -N(R^{12f})C(O)R^{12c}, -S(O)₂N(R^{12f})R^{12c}, -N(R^{12f})S(O)₂R^{12c}, -S(O)₂R^{12c} or - P(O)(R^{12c})₂, wherein each R^{12c} is the same or different and each independently represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), and R^{12f} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R^{12c})ₚ₁₂ₐ indicates that the 6-membered ring containing X₉ and X₁₀ to which it is attached is optionally substituted with p12a R^{12c} , with each R^{12c} being the same or different and each independently representing deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p12a represents an integer of 0, 1, 2, 3 or 4.

In some embodiments, PBM represents a structure of Formulae (PBM-12-3), (PBM-12-4) or (PBM-12-5):

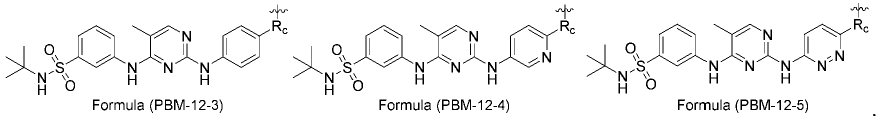

In some embodiments, PBM represents the structure of Formula (PBM-13): 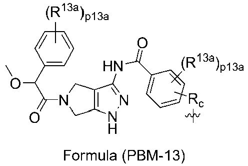
wherein each (R^{13a})ₚ₁₃ₐ independently indicates that the benzene ring to which it is attached is optionally substituted with p13a R^{13a} , with each R^{13a} being the same or different and each independently representing deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
each p13a is the same or different and each independently represents an integer of 0, 1, 2, 3 or 4.

In some embodiments, PBM represents a structure of Formulae (PBM-13-1), (PBM-13-2) or (PBM-13-3):

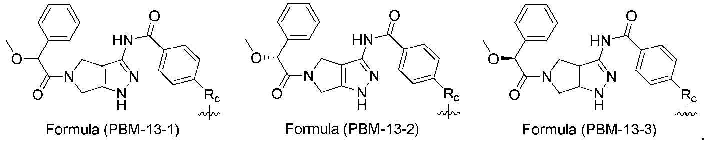

In some embodiments, PBM represents a small molecule ligand of BET.

In some embodiments, PBM represents the structure of Formula (PBM-14): 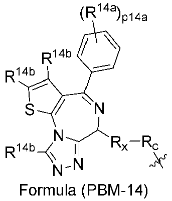
wherein (R^{14a})ₚ₁₄ₐ in Formula (PBM-14) indicates that the benzene ring to which it is attached is substituted with p14a R^{14a}, wherein p14a represents an integer of 0, 1, 2, 3, 4 or 5, and each R^{14a} is the same or different and each independently represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
each R^{14b} is the same or different and each independently represents halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, Rₓ in Formula (PBM-14) represents a bond.

In some embodiments, Rₓ in Formula (PBM-14) represents -C₁₋₃ alkylene-C(O)-.

In some embodiments, PBM represents a structure of Formulae (PBM-14-1) or (PBM-14-2):

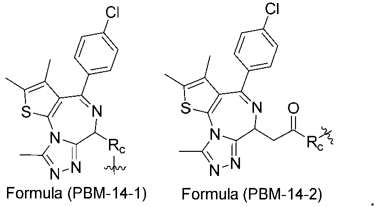

In some embodiments, PBM represents the structure of Formula (PBM-15): 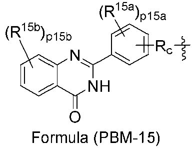
wherein (R^{15a})ₚ₁₅ₐ in Formula (PBM-15) indicates that the benzene ring to which it is attached is optionally substituted with p15a R^{15a} , with each R^{15a} being the same or different and each independently representing deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p15a represents an integer of 0, 1, 2, 3 or 4 ;
(R^{15b})_{p15b} indicates that the 4-oxo-3,4-dihydroquinazoline ring to which it is attached is substituted with p15b R^{15b} , with each R^{15b} being the same or different and each independently representing halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p15b represents an integer of 1, 2, 3 or 4 .

In some embodiments, PBM represents the structure of Formula (PBM-15-1):

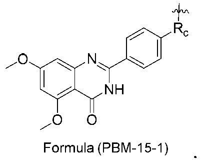

In some embodiments, PBM represents the structure of Formula (PBM-16): 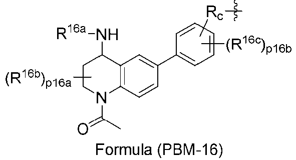
wherein R^{16a} in Formula (PBM-16) represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), wherein the C₆₋₁₀ aryl and 5- to 20-membered heteroaryl are each independently optionally substituted with one or more (e.g., 1-10, 1-8, 2-6, 1-5, 1-4, 1-3, or 2-5) R^{16d}, and each R^{16d} is the same or different and each independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₂₋₆ alkenyl or C₂₋₆ alkynyl;
(R^{16b})ₚ₁₆ₐ in Formula (PBM-16) indicates that 1,2,3,4-tetrahydronaphthalene ring to which it is attached is substituted with p16a R^{16b} , with each R^{16b} being the same or different and each independently representing deuterium, halogen (e.g., fluorine, chlorine, bromine, or iodine), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p16a represents an integer of 0, 1, 2, 3 or 4 ;
(R^{16c})_{p16b} in Formula (PBM-16) indicates that the benzene ring to which it is attached is substituted with p16b R^{16c} , with each R^{16c} being the same or different and each independently representing deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p16b represents an integer of 0, 1, 2, 3 or 4 .

In some embodiments, PBM represents the structure of Formula (PBM-16-1):

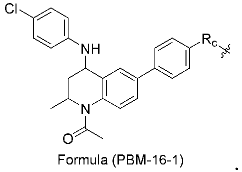

In some embodiments, PBM represents a small molecule ligand of BCR-ABL.

In some embodiments, PBM represents the structure of Formula (PBM-17): 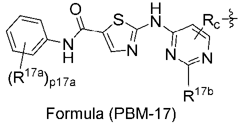
wherein (R^{17a})ₚ₁₇ₐ in Formula (PBM-17) indicates that the benzene ring to which it is attached is substituted with p17a R^{17a} , with each R^{17a} being the same or different and each independently representing deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p17a represents an integer of 1, 2, 3, 4 or 5 ; and
R^{17b} represents halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of Formula (PBM-17-1):

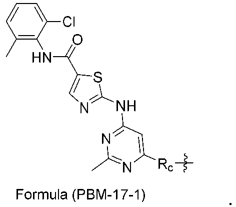

In some embodiments, PBM represents the structure of Formula (PBM-18): 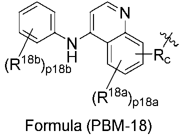
wherein (R^{18a})ₚ₁₈ₐ in Formula (PBM-18) indicates that the quinazoline ring to which it is attached is substituted with p18a R^{18a} , with each R^{18a} being the same or different and each independently representing deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p18a represents an integer of 1, 2, 3 or 4 ;
(R^{18b})_{p18b} in Formula (PBM-18) indicates that the benzene ring to which it is attached is substituted with p18b R^{18b} , with each R^{18b} being the same or different and each independently representing deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p18b represents an integer of 1, 2, 3, 4 or 5.

In some embodiments, PBM represents the structure of Formula (PBM-18-1):

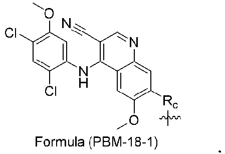

In some embodiments, PBM represents the structure of Formula (PBM-19): 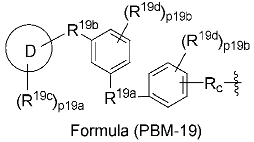
wherein R^{19a} represents -NHC(O)- or -C(O)NH-;
R^{19b} represents -NH-, -N(C₁₋₆ alkyl)-, or ethynylene;
ring D represents a 5- to 20-membered monocyclic, bicyclic, or polycyclic heteroaryl group containing 1 to 4 heteroatoms selected from N, O, and S, and (R^{19c})ₚ₁₉ₐ indicates that the ring D is optionally substituted with p19a R^{19c} , with each R^{19c} being the same or different and each independently representing optionally substituted 5- to 15-membered heteroaryl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p19a represents an integer of 0, 1, 2, or 3;
each (R^{19d})_{p19b} in Formula (PBM-19) independently indicates that the benzene ring to which it is attached is independently optionally substituted with p19b R^{19d}, with each R^{19d} being the same or different and each independently representing deuterium, halogen (e.g., fluorine, chlorine, bromine, or iodine), hydroxy, cyano, amino, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
each p19b is the same or different and each independently represents an integer of 0, 1, 2, 3 or 4 .

In some embodiments, ring D in Formula (PBM-19) represents 5- to 20-membered monocyclic, bicyclic, or polycyclic heteroaryl containing from 1 to 4 heteroatoms selected from the group consisting of oxygen, nitrogen, and sulfur. In a sub-embodiment, examples of 5- to 20-membered monocyclic, bicyclic, or polycyclic heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl is optionally substituted with p19a R^{19c} , wherein p19a represents an integer of 0, 1, 2, or 3, and each R^{19c} is the same or different and each independently represents: optionally substituted 5- to 15-membered heteroaryl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, R^{19c} in Formula (PBM-19) represents optionally substituted 5- to 15-membered heteroaryl. In some embodiments, the 5- to 15-membered heteroaryl is 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl is optionally substituted with one or more (e.g., 1-6, such as 1, 2, 3, 4, 5 or 6) substituents optionally selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof.

In some embodiments, R^{19b} in Formula (PBM-19) represents -NH-.

In some embodiments, R^{19b} in Formula (PBM-19) represents ethynylene.

In some embodiments, PBM represents the structure of Formula (PBM-19-1A): 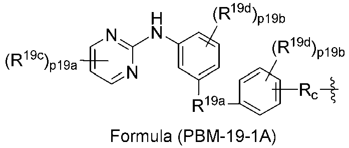
wherein R^{19a} represents -NHC(O)- or -C(O)NH-;
(R^{19c})ₚ₁₉ₐ indicates that the pyrimidinyl to which it is attached is optionally substituted with p19a R^{19c}, with each R^{19c} being the same or different and each independently representing: optionally substituted 5- to 15-membered heteroaryl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p19a represents an integer of 0, 1, 2, or 3;
each (R^{19d})_{p19\b} in Formula (PBM-19-1A) independently indicates that the benzene ring to which it is attached is independently optionally substituted with p19b R^{19d}, with each R^{19d} being the same or different and each independently representing deuterium, halogen (e.g., fluorine, chlorine, bromine, or iodine), hydroxy, cyano, amino, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
each p19b is the same or different and each independently represents an integer of 0, 1, 2, 3 or 4 .

In some embodiments, R^{19c} in Formula (PBM-19-1A) represents optionally substituted 5- to 15-membered heteroaryl. In some embodiments, 5- to 15-membered heteroaryl is 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl is optionally substituted with one or more (e.g., 1-6, such as 1, 2, 3, 4, 5 or 6) substituents optionally selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof.

In some embodiments, PBM represents the structure of Formula (PBM-19-1):

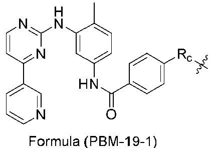

In some embodiments, PBM represents the structure of Formula (PBM-19-1B): 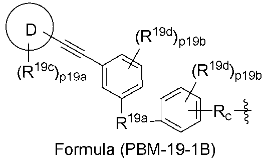
wherein R^{19a} represents -NHC(O)- or -C(O)NH-;
ring D represents a 5- to 20-membered monocyclic, bicyclic, or polycyclic heteroaryl group containing 1 to 4 heteroatoms selected from N, O, and S, and (R^{19c})ₚ₁₉ₐ indicates that the ring D is optionally substituted with p19a R^{19c} , wherein each R^{19c} is the same or different and each independently represents optionally substituted 5- to 15-membered heteroaryl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p19a represents an integer of 0, 1, 2, or 3;
each (R^{19d})_{p19b} in Formula (PBM-19-1B) independently indicates that the benzene ring to which it is attached is independently optionally substituted with p19b R^{19d} , wherein each R^{19d} is the same or different and each independently represents deuterium, halogen (e.g., fluorine, chlorine, bromine, or iodine), hydroxy, cyano, amino, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
each p19b is the same or different and each independently represents an integer of 0, 1, 2, 3 or 4 .

In some embodiments, ring Din Formula (PBM-19-1B) represents 5- to 20-membered monocyclic, bicyclic, or polycyclic heteroaryl containing from 1 to 4 heteroatoms selected from the group consisting of oxygen, nitrogen, and sulfur. In a sub-embodiment, examples of 5- to 20-membered monocyclic, bicyclic, or polycyclic heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl is optionally substituted with p19a R^{19c} , wherein p19a represents an integer of 0, 1, 2, or 3, and each R^{19c} is the same or different and each independently represents: optionally substituted 5- to 15-membered heteroaryl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, R^{19c} in Formula (PBM-19-1B) represents optionally substituted 5- to 15-membered heteroaryl. In some embodiments, the 5- to 15-membered heteroaryl is 5- to 14-membered monocyclic, bicyclic, or polycyclic aromatic ring group containing one or more (e.g., from 1 to 6, or from 1 to 5, or from 1 to 4, or from 1 to 3) heteroatoms selected from the group consisting of oxygen, nitrogen, and sulfur. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl is optionally substituted with one or more (e.g., 1-6, such as 1, 2, 3, 4, 5 or 6) substituents optionally selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof.

In some embodiments, PBM represents the structure of Formula (PBM-19-2):

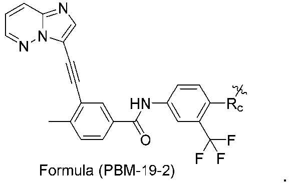

In some embodiments, PBM represents the structure of Formula (PBM-46): 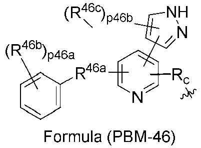
wherein R^{46a} in Formula (PBM-46) represents NHC(O), C(O)NH, NHS(O)₂ or S(O)₂NH;
(R^{46b})ₚ₄₆ₐ indicates that the benzene ring to which it is attached is optionally substituted with p46a R^{46b} , wherein each R^{46b} is independently halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p46a represents an integer of 0, 1, 2, 3, 4 or 5; and
(R^{46c})_{p46b} indicates that the 1H-pyrazole ring to which it is attached is optionally substituted with p46b R^{46c} , wherein each R^{46c} is independently halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p46b represents an integer of 0, 1 or 2.

In some embodiments, p46a in Formula (PBM-46) represents an integer of 1, and R^{46b} is halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In some embodiments, R^{46b} is F₂ClC-O-.

In some embodiments, R^{46a} represents NHC(O). In some embodiments, R^{46a} represents C(O)NH.

In some embodiments, p46b represents an integer of 0.

In some embodiments, PBM represents the structure of Formula (PBM-46-1):

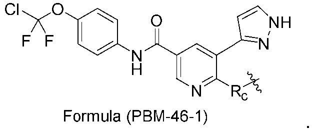

In some embodiments, PBM represents a small molecule ligand of FAK.

In some embodiments, PBM represents the structure of Formula (PBM-12-1D): 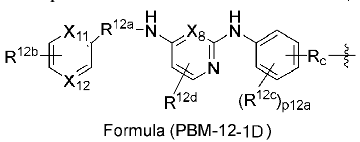
wherein X₈, X₁₁, and X₁₂ in Formula (PBM-12-1D) are the same or different and each independently represent N or CH;
R^{12a} represents a bond or optionally substituted methylene (e.g., halogenated methylene);
R^{12b}) represents -C(O)N(R^{12f})R^{12e}, -N(R^{12f})C(O)R^{12e}, -S(O)₂N(R^{12f})R^{12e}, -N(R^{12f})S(O)₂R^{12e}, -S(O)₂R^{12e} or - P(O)(R^{12e})₂, wherein each R^{12e} is the same or different and each independently represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), and R^{12f} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R^{12c})ₚ₁₂ₐ in (PBM-12-1D) indicates that the benzene ring to which it is attached is optionally substituted with p12a R^{12c}, wherein each R^{12c} is the same or different and each independently represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p12a represents an integer of 0, 1, 2, 3 or 4; and
R^{12d} represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{12a} in Formula (PBM-12-1D) represents a bond. In other words, the 6-membered ring containing X₁₁ and X₁₂ in Formula (PBM-12-1D) is directly bonded to the -NH- group.

In some embodiments, R^{12a} in Formula (PBM-12-1D) represents optionally substituted methylene (e.g., halogenated methylene).

In some embodiments, PBM represents a structure of Formulae (PBM-12-1E) or (PBM-12-1F): 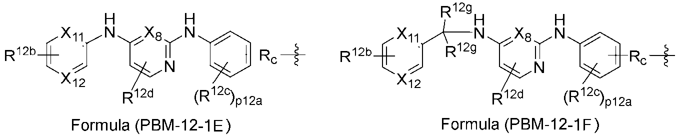
wherein X₈, X₁₁, and X₁₂ are the same or different and each independently represent N or CH;
each R^{12g} is the same or different and each independently represents hydrogen or halogen;
R^{12b} represents -C(O)N(R^{12f})R^{12e}, -N(R^{12f})C(O)R^{12e}, -S(O)₂N(R^{12f})R^{12e}, -N(R^{12f})S(O)₂R^{12e}, -S(O)₂R^{12e} or - P(O)(R^{12e})₂, wherein each R^{12e} is the same or different and each independently represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), and R^{12f} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R^{12c})ₚ₁₂ₐ indicates that the benzene ring to which it is attached is optionally substituted with p12a R^{12c} , wherein each R^{12c} is the same or different and each independently represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p12a represents an integer of 0, 1, 2, 3 or 4; and
R^{12d} represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents a structure of Formulae (PBM-12-6), (PBM-12-7), (PBM-12-8), (PBM-12-9), (PBM-12-10) or (PBM-12-11):

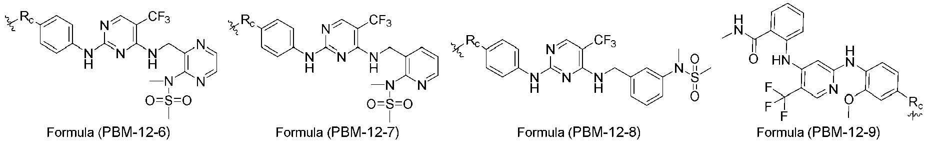

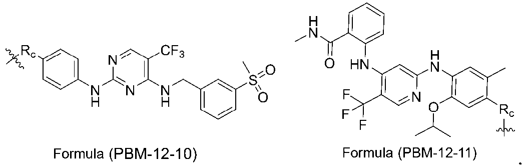

In some embodiments, PBM represents the structure of Formula (PBM-47): 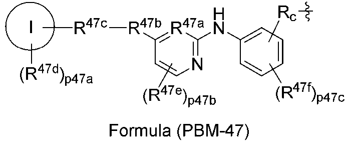
wherein R^{47a} represents N or CH;
R^{47b} represents O, S, NH, CH₂, CH(F) or C(F)₂;
R^{47c} represents a bond or optionally substituted methylene (e.g., halogenated methylene);
ring I represents C₆₋₁₅ aryl, 4- to 15-membered heterocyclyl, or 5- to 15-membered heteroaryl, and (R^{47d})ₚ₄₇ₐ indicates that the ring I to which it is attached is optionally substituted with p47a R^{47d}, wherein each R^{47d} is the same or different and each independently represents deuterium, halogen (e.g., fluorine, chlorine, bromine, or iodine), hydroxy, cyano, amino, nitro, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p47a represents an integer of 0, 1, 2, 3 or 4;
(R^{47e})_{p47b} indicates that the 6-membered nitrogen-containing heteroaromatic ring containing R^{47a} to which it is attached is optionally substituted with p47b R^{47e} , and each R^{47e} represents deuterium, halogen (e.g., fluorine, chlorine, bromine, or iodine), hydroxy, cyano, amino, nitro, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p47b represents an integer of 0, 1 or 2;
(R^{47f})_{p47c} indicates that the benzene ring to which it is attached is optionally substituted with p47c R^{47f}, with each R^{47f} being the same or different and each independently representing deuterium, halogen (e.g., fluorine, chlorine, bromine, or iodine), hydroxy, cyano, amino, nitro, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p47c represents an integer of 0, 1, 2, 3 or 4.

In some embodiments, R^{47a} represents N.

In some embodiments, R^{47a} represents CH.

In some embodiments, R^{47c} represents a bond.

In some embodiments, R^{47c} represents optionally substituted methylene (e.g., methylene which is optionally substituted with halogen (e.g., fluorine, chlorine, bromine, or iodine)).

In some embodiments, ring I represents C₆₋₁₅ aryl, 4- to 15-membered heterocyclyl, or 5- to 15-membered heteroaryl. Examples of C₆₋₁₅ aryl include, but are not limited to, phenyl and naphthyl. Examples of 4- to 15-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl). Examples of 5- to 15-membered heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-furo[3,2-b]pyrrolyl, pyrrolo[2,1-b]thiazolyl, and imidazo[2,1-b]thiazolyl.

In some embodiments, (R^{47d})ₚ₄₇ₐ in Formula (PBM-47) indicates that the ring I to which it is attached is optionally substituted with p47a R^{47d} , wherein each R^{47d} is the same or different and each independently represents deuterium, halogen (e.g., fluorine, chlorine, bromine, or iodine), hydroxy, cyano, amino, nitro, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p47a represents an integer of 0, 1, 2, 3 or 4.

In some embodiments, ring I represents isoindolinyl, which is optionally substituted with p47a R^{47d} , wherein each R^{47d} is the same or different and each independently represents deuterium, halogen (e.g., fluorine, chlorine, bromine, or iodine), hydroxy, cyano, amino, nitro, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p47a represents an integer of 0, 1, 2, 3 or 4. In some embodiments, p47a represents an integer of 0, 1, 2, 3 or 4. In some embodiments, ring I represents isoindolinyl, which is optionally substituted with halogen (e.g., fluorine, chlorine, bromine, or iodine), oxo, and C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl).

In some embodiments, (R^{47e})_{p47b} indicates that the 6-membered nitrogen-containing heteroaromatic ring containing R^{47a} to which it is attached is optionally substituted with p47b R^{47e} , wherein each R^{47c} represents deuterium, halogen (e.g., fluorine, chlorine, bromine, or iodine), hydroxy, cyano, amino, nitro, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p47b represents an integer of 0, 1 or 2. In some embodiments, each R^{47e} independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p47b represents an integer of 0, 1 or 2.

In some embodiments, (R^{47f})_{p47c} indicates that the benzene ring to which it is attached is optionally substituted with p47c R^{47f} , with each R^{47f} being the same or different and each independently representing deuterium, halogen (e.g., fluorine, chlorine, bromine, or iodine), hydroxy, cyano, amino, nitro, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p47c represents an integer of 0, 1, 2, 3 or 4. In some embodiments, each R^{47f} independently represents halogen (e.g., fluorine, chlorine, bromine, or iodine) or C₁₋₆ alkoxy (e.g., C₁₋₄ alkoxy, such as methoxy, ethoxy, propoxy, isopropoxy, n-butoxy, isobutoxy, or tert-butoxy), and p47c represents an integer of 0, **1,** 2, 3 or 4.

In some embodiments, PBM represents a structure of Formulae (PBM-47-1A), (PBM-47-1B), (PBM-47-1C), (PBM-47-2A), (PBM-47-2B) or (PBM-47-2C): 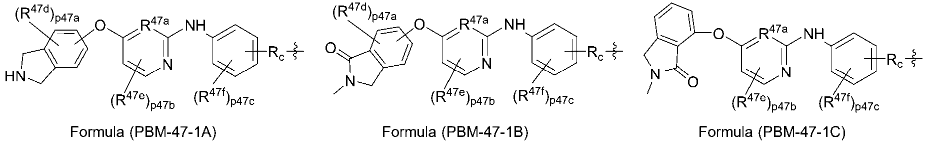 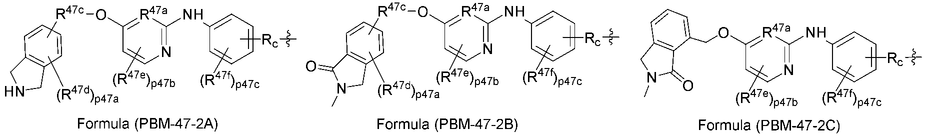 wherein R^{47a}, R^{47c}, (R^{47d})ₚ₄₇ₐ, (R^{47e})_{p47b}, and (R^{47f})_{p47c} are as defined in various embodiments of Formula (PBM-47) and each sub-embodiments thereof.

In some embodiments, PBM represents a structure of Formulae (PBM-47-1), (PBM-47-2), (PBM-47-3) or (PBM-47-4):

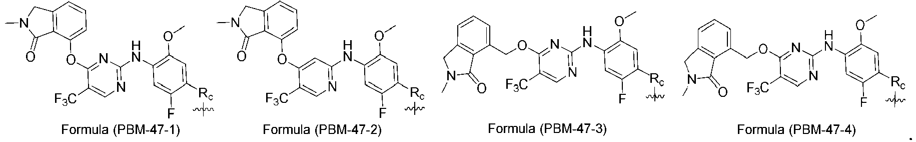

In some embodiments, PBM represents a small molecule ligand of ER.

In some embodiments, PBM represents a structure of Formulae (PBM-20-1A), (PBM-20-1B), (PBM-20-1C) or (PBM-20-1D): 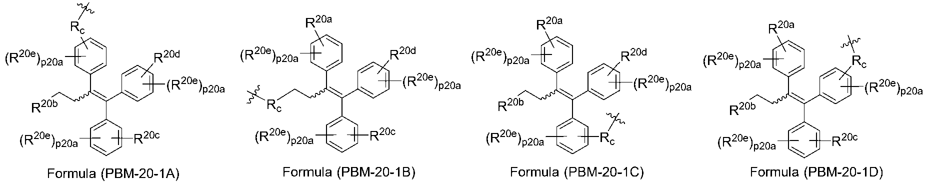
wherein R^{20a}, R^{20b}, R^{20c}, and R^{20d} are the same or different and each independently represent hydrogen, deuterium, halogen (e.g., fluorine, chlorine, bromine, or iodine), hydroxy, cyano, amino, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
symbol " 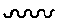 " connecting the double bond in Formulae (PBM-20-1A), (PBM-20-1B), (PBM-20-1C), and (PBM-20-1D) represents a bond that may be in stereo-configuration (cis or trans configuration, or E- or Z-configuration);
each (R^{20e})ₚ₂₀ₐ independently indicates that the benzene ring to which it is attached is independently optionally substituted with p20a R^{20e}, with each R^{20e} being the same or different and each independently representing deuterium, halogen (e.g., fluorine, chlorine, bromine, or iodine), hydroxy, cyano, amino,C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
each p20a is the same or different and each independently represents an integer of 0, 1, 2, 3 or 4 .

In some embodiments, PBM represents a structure of Formulae (PBM-20-1), (PBM-20-2), (PBM-20-3), (PBM-20-4), (PBM-20-5), (PBM-20-6), (PBM-20-7), (PBM-20-8), (PBM-20-9) or (PBM-20-10):

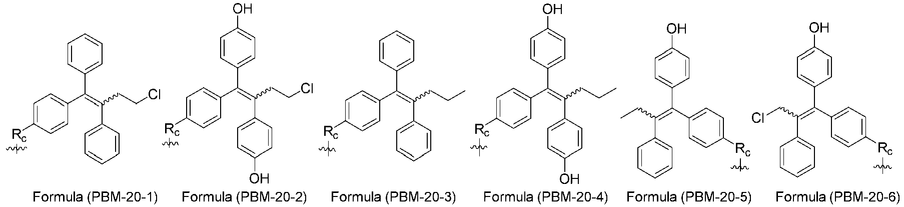

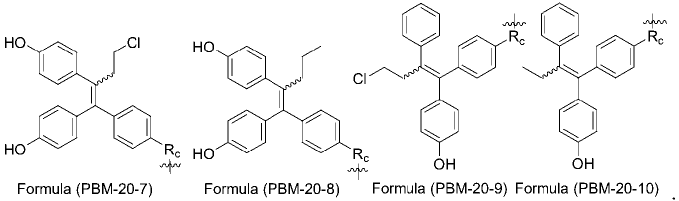

In some embodiments, PBM represents a structure of Formulae (PBM-21-1A) or (PBM-21-1B): 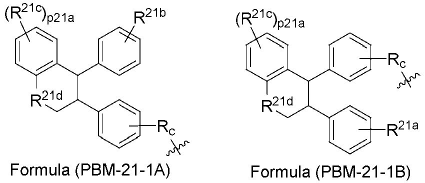
wherein each R^{21d} independently represents O, S or CH₂;
(R^{21c})ₚ₂₁ₐ represents p21a R^{21c} groups; that is, the ten-membered ring containing R^{21d} in Formula (PBM-21) is optionally substituted by p21a R^{21c} substituents, wherein each R^{21c} is the same or different and each independently represents hydrogen, hydroxy, halogen (e.g., fluorine, chlorine, bromine, or iodine), hydroxy, cyano, amino, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p21a represents an integer of 0, 1, 2, 3 or 4 ; and
R^{21a} and R^{21b} each independently represent hydrogen, halogen (e.g., fluorine, chlorine, bromine, or iodine), hydroxy, cyano, amino, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents a structure of Formulae (PBM-21-1), (PBM-21-2), (PBM-21-3), (PBM-21-4), (PBM-21-5), (PBM-21-6), (PBM-21-7), (PBM-21-8), (PBM-21-9), (PBM-21-10), (PBM-21-11) or (PBM-21-12):

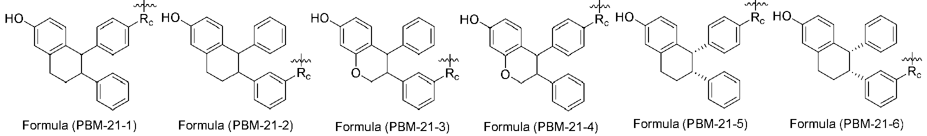

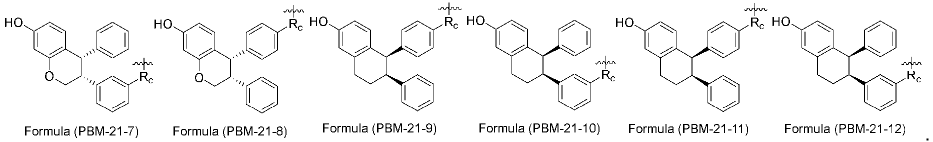

In some embodiments, PBM represents a small molecule ligand of IRAK4.

In some embodiments, PBM represents the structure of Formula (PBM-22): 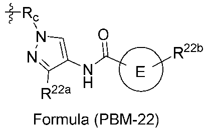
wherein ring E in Formula (PBM-22) represents a 5- to 20-membered monocyclic, bicyclic, or polycyclic heteroarylene group containing 1 to 4 heteroatoms selected from N, O, and S;
R^{22b} represents optionally substituted 5- to 15-membered heteroaryl, optionally substituted 5- to 15-membered heterocyclyl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-,Cl₂CH-O-, CF₃CF₂-O-, CF₃CHF-O-, CHF₂CF₂-O-, CHF₂CHF-O-, CF₃CH₂-O- or CH₂ClCH₂-O-);
R^{22a} represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-,Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, ring E in Formula (PBM-22) represents 5- to 20-membered monocyclic, bicyclic, or polycyclic heteroarylene group containing 1 to 4 heteroatoms selected from N, O, and S. In some embodiments, the 5- to 20-membered monocyclic, bicyclic, or polycyclic heteroarylene is 5- to 14-membered divalent monocyclic, bicyclic, or polycyclic aromatic ring group containing from 1 to 4 (e.g., from 1 to 4, or from 1 to 3, or from 1 to 2, or 1) heteroatoms selected from the group consisting of oxygen, nitrogen, and sulfur. In some sub-embodiments, examples of 5- to 20-membered monocyclic, bicyclic, or polycyclic heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, and imidazo[1,2-b]pyridazinylene. The 5- to 20-membered monocyclic, bicyclic, or polycyclic heteroarylene is optionally substituted with one or more (e.g., 1-3, such as 1, 2, or 3) R^{22b} substituents, wherein each R^{22b} independently represents optionally substituted 5- to 15-membered heteroaryl, optionally substituted 5- to 15-membered heterocyclyl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃CO-, FCH₂-O-, F₂CH-O-, ClCH₂-O-,Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, R^{22b} in Formula (PBM-22) represents optionally substituted 5- to 15-membered heteroaryl. In some embodiments, the 5- to 15-membered heteroaryl is 5- to 15-membered monocyclic, bicyclic, or polycyclic aromatic ring group containing from 1 to 4 (e.g., from 1 to 4, or from 1 to 3, or from 1 to 2, or 1) heteroatoms selected from the group consisting of oxygen, nitrogen, and sulfur. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. Heteroaryl represented by R^{22b} is optionally substituted with one or more (e.g., 1-3, such as 1, 2, or 3) substituents which are optionally selected from the group consisting of: -N(R^{22c})-C₁₋₆ alkylene-optionally substituted C₃₋₈ cycloalkyl, -N(R^{22c})-optionally halogenated C₁₋₆ alkyl, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-,Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof, wherein each R^{22c} independently represents hydrogen or C₁₋₃ alkyl. In some embodiments, the substituent of heteroaryl represented by R^{22b} is -N(R^{22c})-C₁₋₆ alkylene-optionally substituted C₃₋₈ cycloalkyl, wherein R^{22c} represents hydrogen or C₁₋₃ alkyl (e.g., methyl). In some embodiments, examples of C₁₋₆ alkylene include, but are not limited to, C₁₋₃ alkylene. In some embodiments, examples of C₃₋₈ cycloalkyl include, but are not limited to, C₃₋₆ cycloalkyl. In some embodiments, representative examples of C₃₋₈ cycloalkyl include, but are not limited to, cyclopropyl, cyclobutyl, cyclopentyl, cyclopentenyl, cyclohexyl, cyclohexenyl, cycloheptyl, and cyclooctyl. C₃₋₈ cycloalkyl is optionally substituted with one or more (e.g., 1-3, such as 1, 2, or 3) substituents selected from the group consisting of C₁-C₃ alkyl, C₃₋₆ cycloalkyl, hydroxy, amino, mercapto, halogen, C₁-C₃ alkoxy, C₁-C₃ alkylamino, halogenated C₁-C₃ alkyl, amino-substituted C₁₋₃ alkylene, cyano or any combination thereof. In some embodiments, the substituent of heteroaryl represented by R^{22b} is -N(R^{22c})-optionally halogenated C₁₋₆ alkyl, wherein R^{22c} represents hydrogen or C₁₋₃ alkyl (e.g., methyl). In some embodiments, examples of optionally halogenated C₁₋₆ alkyl include, but are not limited to, optionally halogenated C₁₋₄ alkyl, optionally halogenated C₁₋₃ alkyl and optionally halogenated C₁₋₂ alkyl. In some embodiments, representative examples of optionally halogenated C₁₋₆ alkyl include, but are not limited to, -CH₂-CF₃.

In some embodiments, R^{22b} in Formula (PBM-22) represents optionally substituted 5- to 15-membered heterocyclyl. In some sub-embodiments, "5- to 15-membered heterocyclyl" refers to a 5- to 15-membered saturated or partially unsaturated (i.e., containing one or more double bonds, but not having a fully conjugated π-electron system) monocyclic, bicyclic, or tricyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some sub-embodiments, examples of heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl), and diazacyclooctyl. The heterocyclyl is optionally substituted with one or more (e.g., 1 to 5, 1 to 4, 1 to 3, 1 to 2, or 1) substituents selected from the group consisting of deuterium, hydroxy, amino, mercapto, nitro, halogen (e.g., fluorine, chlorine, bromine, or iodine), cyano, C₁₋₆ alkyl, halogenated C₁₋₆ alkyl, deuterated C₁₋₆ alkyl, C₃₋₆ cycloalkyl, C₁₋₆ alkoxy, halogenated C₁₋₆ alkoxy or any combination thereof.

In some embodiments, PBM represents a structure of Formulae (PBM-22-1), (PBM-22-2), (PBM-22-3) or (PBM-22-4):

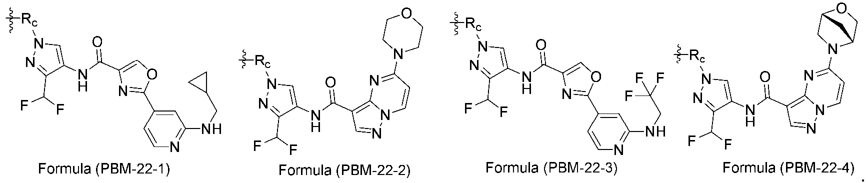

In some embodiments, PBM represents the structure of Formula (PBM-23): 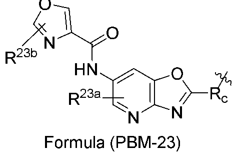
wherein R^{23a} in Formula (PBM-23) represents optionally substituted 5- to 15-membered heterocyclyl, optionally substituted 5- to 15-membered heteroaryl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-,Cl₂CH-O-, CF₃CF₂-O-, CF₃CHF-O-, CHF₂CF₂-O-, CHF₂CHF-O-, CF₃CH₂-O- or CH₂ClCH₂-O-); and
R^{23b} represents a 5- to 20-membered monocyclic, bicyclic, or polycyclic heteroaryl group containing 1 to 4 heteroatoms selected from N, O, and S, and the heteroaryl is optionally substituted with one or more (e.g., 1-6, such as 1, 2, 3, 4, 5 or 6) substituents selected from the group consisting of deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof.

In some embodiments, R^{23a} in Formula (PBM-23) represents optionally substituted 5- to 15-membered heterocyclyl. In some sub-embodiments, "5- to 15-membered heterocyclyl" refers to a 5- to 15-membered saturated or partially unsaturated (i.e., containing one or more double bonds, but not having a fully conjugated π-electron system) monocyclic, bicyclic, or tricyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some sub-embodiments, examples of the heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl), and diazacyclooctyl. The heterocyclyl is optionally substituted with one or more (e.g., 1 to 5, 1 to 4, 1 to 3, 1 to 2, or 1) substituents selected from the group consisting of deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₃₋₆ cycloalkyl, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-,Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof.

In some embodiments, R^{23b} in Formula (PBM-23) represents optionally substituted 5- to 20-membered (e.g., 5- to 15-membered, 5- to 10-membered, or 5- to 6-membered) monocyclic, bicyclic, or polycyclic heteroaryl containing from 1 to 4 heteroatoms selected from the group consisting of oxygen, nitrogen, and sulfur. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl is optionally substituted with one or more (e.g., 1-3, such as 1, 2, or 3) substituents optionally selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof.

In some embodiments, PBM represents a structure of Formulae (PBM-23-1), (PBM-23-2) or (PBM-23-3):

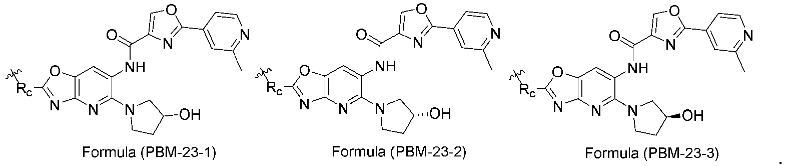

In some embodiments, PBM represents the structure of Formula (PBM-24): 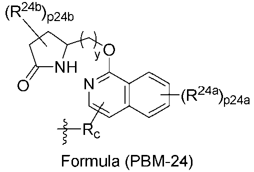
wherein (R^{24a})ₚ₂₄ₐ indicates that the isoquinoline ring in Formula (PBM-24) is optionally substituted with p24a R^{24a}, wherein each R^{24a} is the same or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), -C(O)NH₂, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p24a represents an integer of 0, 1, 2, 3 or 4 ;
(R^{24b})_{p24b} indicates that the pyrrolidinone ring in Formula (PBM-24) is optionally substituted with p24b R^{24b}, wherein each R^{24b} is the same or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), -C(O)NH₂, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p24b represents an integer of 0, 1, 2, 3 or 4 ; and
y in Formula (PBM-24) represents an integer of 1, 2 or 3.

In some embodiments, y in Formula (PBM-24) represents an integer of 1.

In some embodiments, PBM represents a structure of Formulae (PBM-24-1) or (PBM-24-2):

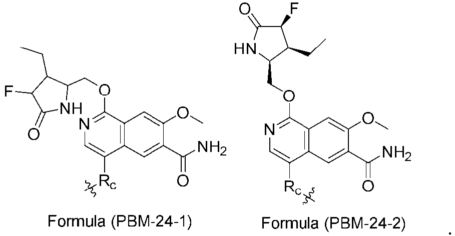

In some embodiments, PBM represents the structure of Formula (PBM-45): 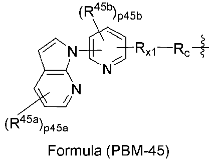
wherein Rₓ₁ in Formula (PBM-45) represents a bond or C(O), or Rₓ₁ represents -C(O)NH-Rₓ₂-C(O)-***, wherein symbol *** indicates the point of attachment to R_{c}, and Rₓ₂ represents optionally substituted C₃₋₁₅ cycloalkyl;
(R^{45a})ₚ₄₅ₐ indicates that the 1H-pyrrolo[2,3-b]pyridine ring to which it is attached is optionally substituted with p45a R^{45a}, wherein each R^{45a} is independently cyano, halogen (e.g., fluorine, chlorine, bromine, or iodine), hydroxy, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p45a represents an integer of 0, 1, 2, 3, 4 or 5;
(R^{45b})_{p45b} indicates that the pyridine ring to which it is attached is optionally substituted with p45b R^{45b}, wherein each R^{45b} is independently cyano, halogen (e.g., fluorine, chlorine, bromine, or iodine), hydroxy, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or NR^{45c}R^{45d}, where R^{45c} and R^{45d} are the same or different and each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₁₅ cycloalkyl or optionally substituted 4- to 15-membered heterocyclyl, and p45b represents an integer of 0, 1, 2 or 3.

**In** some embodiments, Rₓ₁ in Formula (PBM-45) represents a bond.

**In** some embodiments, Rₓ₁ in Formula (PBM-45) represents C(O).

**In** some embodiments, Rₓ₁ in Formula (PBM-45) represents -C(O)NH-Rₓ₂-C(O)-***, wherein symbol *** indicates the point of attachment to R_{c}, and Rₓ₂ represents optionally substituted C₃₋₁₅ cycloalkyl. In some sub-embodiments, examples of the optionally substituted C₃₋₁₅ cycloalkyl include, but are not limited to, optionally substituted C₃₋₁₁ cycloalkyl, and optionally substituted C₃₋₈ cycloalkyl, e.g., optionally substituted cyclopropyl, optionally substituted cyclobutyl, optionally substituted cyclopentyl, optionally substituted cyclohexyl, optionally substituted cycloheptyl, or optionally substituted cyclooctyl. In some embodiments, C₃₋₁₅ cycloalkyl is optionally substituted with one or more (e.g., 1-3, such as 1, 2 or 3) substituents selected from the group consisting of: deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof.

**In** some embodiments, (R^{45a})ₚ₄₅ₐ indicates that the 1H-pyrrolo[2,3-b]pyridine ring in Formula (PBM-45) to which it is attached is optionally substituted with p45a R^{45a} , wherein each R^{45a} is independently cyano, halogen (e.g., fluorine, chlorine, bromine, or iodine), hydroxy, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p45a represents an integer of 0, 1, 2, 3, 4 or 5.

**In** some embodiments, (R^{45a})ₚ₄₅ₐ indicates that the 1H-pyrrolo[2,3-b]pyridine ring in Formula (PBM-45) to which it is attached is optionally substituted with p45a R^{45a} , wherein p45a represents an integer of 1, and R^{45a} is cyano.

**In** some embodiments, (R^{45b})_{p45b} indicates that the pyridine ring to which it is attached is optionally substituted with p45b R^{45b}, wherein each R^{45b} is independently cyano, halogen (e.g., fluorine, chlorine, bromine, or iodine), hydroxy, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF_{3C}H₂-O- or CH₂ClCH₂-O-) or NR^{45c}R^{45d}, wherein R^{45c} and R^{45d} are the same or different and each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₁₅ cycloalkyl (e.g., optionally substituted C₃₋₁₂ cycloalkyl and optionally substituted C₃₋₈ cycloalkyl) or optionally substituted 4- to 15-membered heterocyclyl (e.g., optionally substituted 4- to 12-membered heterocyclyl or optionally substituted 4- to 10-membered heterocyclyl), and p45b represents an integer of 0, 1, 2 or 3. In some sub-embodiments, examples of the optionally substituted C₃₋₁₅ cycloalkyl include, but are not limited to, optionally substituted cyclopropyl, optionally substituted cyclobutyl, optionally substituted cyclopentyl, optionally substituted cyclohexyl, optionally substituted cycloheptyl or optionally substituted cyclooctyl. In some sub-embodiments, examples of the optionally substituted 4- to 15-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl, azacyclooctyl, and diazacyclooctyl. In some sub-embodiments, substituents of the optionally substituted C₃₋₁₅ cycloalkyl and optionally substituted 4- to 15-membered heterocyclyl are optionally selected from the group consisting of: deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof.

**In** some embodiments, (R^{45b})_{p45b} in Formula (PBM-45) indicates that the pyridine ring to which it is attached is optionally substituted with p45b R^{45b}, wherein p45b represents an integer of 1, and R^{45b} is NR^{45c}R^{45d}, where R^{45c} represents hydrogen, and R^{45d} represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl).

**In** some embodiments, PBM represents a structure of Formulae (PBM-45-1), (PBM-45-2), (PBM-45-3) or (PBM-45-4):

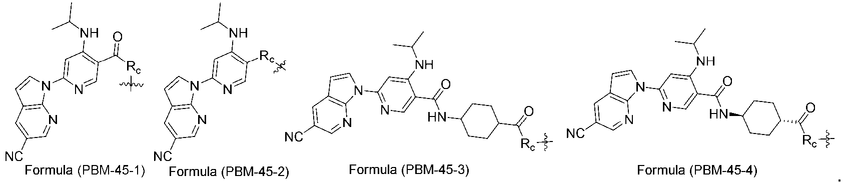

In some embodiments, PBM represents the structure of Formula (PBM-49): 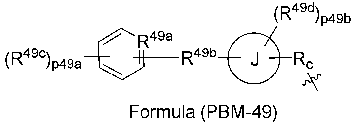
wherein R^{49a} in Formula (PBM-49) represents N or CH;
R^{49b} represents NHC(O), C(O)NH, NHS(O)₂ or S(O)₂NH;
(R^{49c})ₚ₄₉ₐ indicates that the 6-membered ring containing R^{49a} is substituted with p49a R^{49c}, with each R^{49c} being the same or different and each independently representing halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p49a represents an integer of 0, 1, 2, 3 or 4; and
ring J represents 5- to 15-membered heteroarylene, (R^{49d})_{p49b} indicates that the ring J to which it is attached is optionally substituted with p49b R^{49d}, with each R^{49d} being the same or different and each independently representing halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), hydroxy-substituted C₁₋₆ alkyl, amino-substituted C₁₋₆ alkyl, C₁₋₆ alkoxy (e.g., C₁₋₄ alkoxy, such as methoxy, CD₃-O-, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or optionally substituted C₃₋₁₅ cycloalkyl, and p49b represents an integer of 0, 1, 2 or 3.

In some embodiments, R^{49a} represents N. In some embodiments, R^{49a} represents CH.

In some embodiments, (R^{49c})ₚ₄₉ₐ indicates that the 6-membered ring containing R^{49a} is substituted with p49a R^{49c} , wherein each R^{49c} is the same or different and each independently represents halogen (e.g., fluorine, chlorine, bromine, iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., deuterated C₁₋₃ alkyl, such as CD₃), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p49a represents an integer of 0, 1, 2, 3 or 4, and the aromatic ring containing R^{49a} is a pyridine ring or a benzene ring. In some embodiments, the aromatic ring containing R^{49a} is a pyridine ring, and R^{49c} represents F₃C-, and R^{49a} represents an integer of 1.

In some embodiments, ring J represents 5- to 15-membered heteroarylene, e.g., 5- to 12-membered heteroarylene, or 5- to 10-membered heteroarylene. In some embodiments, examples of ring J include, but are not limited to, benzimidazolylene, benzothiazolylene, 2H-indazolylene, benzoxazolylene, isoindolylene, benzofuranylene, isobenzofuranylene, benzothienylene, benzisoxazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, imidazo[1,2-b]pyridazinylene, and 6,7-dihydro-5H-pyrrolo[1,2-c]imidazolylene. Ring J is optionally substituted with p49b R^{49d} , with each R^{49d} being the same or different and each independently representing halogen (e.g., fluorine, chlorine, bromine, iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., deuterated C₁₋₃ alkyl, such as CD₃), hydroxy-substituted C₁₋₆ alkyl (e.g., hydroxy-substituted C₁₋₄ alkyl, such as hydroxypropyl, and hydroxyisopropyl), amino-substituted C₁₋₆ alkyl (e.g., amino-substituted C₁₋₄ alkyl, such as aminopropyl, aminoisopropyl), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or optionally substituted C₃₋₁₅ cycloalkyl, and p49b represents an integer of 0, 1, 2 or 3. In some embodiments, R^{49d} represents hydroxypropyl or hydroxyisopropyl, and p49b represents an integer of 1. In some embodiments, R^{49d} represents optionally substituted C₃₋₁₅ cycloalkyl. Examples of C₃₋₁₅ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, 2,3-dihydro-1H-indenyl, spiro-cycloalkyl (e.g., C₅₋₁₅ spiro-cycloalkyl), adamantanyl, noradamantanyl, and norbornyl. The optional substituents of C₃₋₁₅ cycloalkyl are optionally selected from the group consisting of halogen (e.g., fluorine, chlorine, bromine, or iodine), hydroxy, mercapto, cyano, amino, nitro, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CF-O-, CHF₂CF₂-O-, CHF₂CHF-O-, CF₃CH₂-O- or CH₂ClCH₂-O-).

In some embodiments, PBM represents a structure of Formulae (PBM-49-1), (PBM-49-2), (PBM-49-3), (PBM-49-4), (PBM-49-5), (PBM-49-6), (PBM-49-7), (PBM-49-8), (PBM-49-9), (PBM-49-10), (PBM-49-11) or (PBM-49-12):

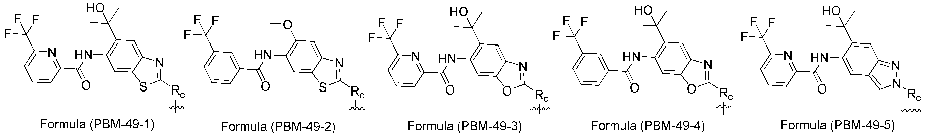

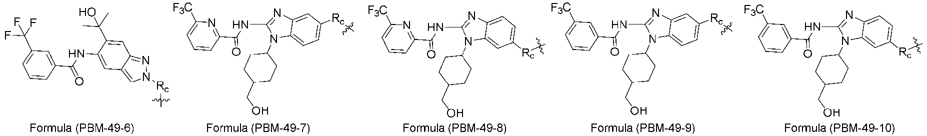

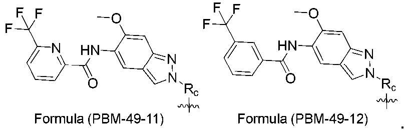

In some embodiments, PBM represents a small molecule ligand of CDK9.

In some embodiments, PBM represents the structure of Formula (PBM-25): 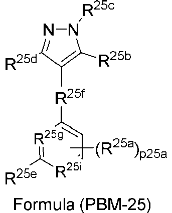
wherein R^{25g} in Formula (PBM-25) represents N or CR^{25h}, wherein R^{25h} represents hydrogen or halogen (e.g., fluorine, chlorine, bromine, or iodine), and R²⁵ⁱ represents N or CR^{25j}, where R^{25j} represents hydrogen or halogen (e.g., fluorine, chlorine, bromine, or iodine);
R^{25f} represents a bond, -C(O)NH-, -NHC(O)- , -S(O)₂NH- or -NHS(O)₂-;
(R^{25a})ₚ₂₅ₐ indicates that the 6-membered ring containing R^{25g} and R²⁵ⁱ in Formula (PBM-25) to which it is attached is optionally substituted with p25a R^{25a}, with each R^{25a} being the same or different and each independently representing deuterium, halogen (e.g., fluorine, chlorine, bromine, or iodine), hydroxy, cyano, amino, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p25a represents an integer of 0, 1 or 2 ;
R^{25b} represents hydrogen, halogen (e.g., fluorine, chlorine, bromine, or iodine), C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or -C₁₋₃ alkylene-C₃₋₆ cycloalkyl;
R^{25c} represents hydrogen, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); or
R^{25b} and R^{25c} together with their respective attached nitrogen and carbon atoms form an optionally substituted 5- to 7-membered nitrogen-containing heterocycle;
R^{25d} represents hydrogen or a bond, and R^{25c} represents hydrogen or a bond, wherein R^{25d} and R^{25e} are not simultaneously hydrogen, and only one of R^{25d} and R^{25e} represents a bond, and the other represents hydrogen, wherein when R^{25d} represents a bond, the ring carbon atom connected to R^{25d} is directly connected to R_{c} , and when R^{25c} represents a bond, the ring carbon atom connected to R^{25c} is directly connected to R_{c}.

In some embodiments, R^{25f} in Formula (PBM-25) represents a bond.

In some embodiments, R^{25f} in Formula (PBM-25) represents -C(O)NH-, -NHC(O)- , -S(O)₂NH- or - NHS(O)₂-.

In some embodiments, R^{25b} in Formula (PBM-25) represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or -C₁₋₃ alkylene-C₃₋₆ cycloalkyl (e.g., -methylene-cyclopropyl); and/or
R^{25c} represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{25b} and R^{25c} together with their respective attached nitrogen and carbon atoms in Formula (PBM-25) form an optionally substituted 5- to 7-membered nitrogen-containing heterocycle. In some embodiments, the 5- to 7-membered nitrogen-containing heterocycle includes, but is not limited to, 5- to 7-membered heterocycle (e.g., 5- to 6-membered or 5-membered heterocycle) containing one nitrogen atom and optionally containing heteroatoms selected from nitrogen, oxygen, and sulfur. The 5-to 7-membered nitrogen-containing heterocycle is fused with the pyrazole ring in Formula (PBM-25) to form a fused ring system, including, but not limited to, a 5,6-dihydro-4H-pyrrolo[1,2-b]pyrazole ring. The 5- to 7-membered nitrogen-containing heterocycle is optionally substituted with one or more (e.g., 1 to 6, or 1 to 5, or 1 to 4, 1 to 3, or 2) substituents selected from the group consisting of: halogen (e.g., fluorine, chlorine, bromine, or iodine), hydroxy, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.).

In some embodiments, the 5- to 7-membered nitrogen-containing heterocycle, formed by R^{25b} and R^{25c} together with their respective attached nitrogen and carbon atoms, is fused with the adjacent pyrazole ring to form the following groups:

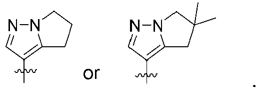

In some embodiments, PBM represents the structure of Formula (PBM-25-1A): 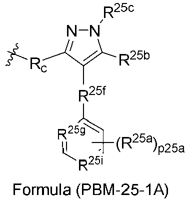
wherein R^{25g} represents N or CR^{25h}, where R^{25h} represents hydrogen or halogen, and R²⁵ⁱ represents N or CR^{25j}, where R^{25j} represents hydrogen or halogen;
R^{25f} represents a bond, -C(O)NH-, -NHC(O)- , -S(O)₂NH- or -NHS(O)₂-;
(R^{25a})ₚ₂₅ₐ indicates that the 6-membered ring containing R^{25g} and R²⁵ⁱ to which it is attached is optionally substituted with p25a R^{25a}, wherein each R^{25a} represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, CD₃-O-, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R^{25b} represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or - C₁₋₃ alkylene-C₃₋₆ cycloalkyl (e.g., -methylene-cyclopropyl);
R^{25c} represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); or
R^{25b} and R^{25c} together with their respective attached nitrogen and carbon atoms form an optionally substituted 5- to 7-membered nitrogen-containing heterocycle.

In some embodiments, PBM represents the structure of Formula (PBM-25-1B): 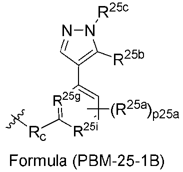
wherein R^{25g} represents N or CR^{25h}, where R^{25h} represents hydrogen or halogen, and R²⁵ⁱ represents N or CR^{25j}, where R^{25j} represents hydrogen or halogen;
(R^{25a})ₚ₂₅ₐ indicates that the 6-membered ring containing R^{25g} and R²⁵ⁱ to which it is attached is optionally substituted with p25a R^{25a}, wherein each R^{25a} represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, CD₃-O-, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R^{25b} represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or - C₁₋₃ alkylene-C₃₋₆ cycloalkyl (e.g., -methylene-cyclopropyl);
R^{25c} represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); or
R^{25b} and R^{25c} together with their respective attached nitrogen and carbon atoms form an optionally substituted 5- to 7-membered nitrogen-containing heterocycle.

In some embodiments, R^{25b} in each of Formulae (PBM-25-1A) and (PBM-25-1B) each independently represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or -C₁₋₃ alkylene-C₃₋₆ cycloalkyl (e.g., -methylene-cyclopropyl).

In some embodiments, R^{25c} in each of Formulae (PBM-25-1A) and (PBM-25-1B) each independently represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{25b} and R^{25c} in Formula (PBM-25-1A) each independently represent hydrogen.

In some embodiments, R^{25b} in Formula (PBM-25-1B) represents -methylene-cyclopropyl, and R^{25c} represents methyl.

In some embodiments of Formulae (PBM-25-1A) and (PBM-25-1B), the pyrazole ring is fused with a nitrogen-containing heterocycle, which is formed by R^{25b} and R^{25c} together with their respective attached nitrogen and carbon atoms, to form the following group:

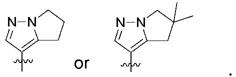

In some embodiments, PBM represents a structure of Formulae (PBM-25-1), (PBM-25-2) or (PBM-25-3):

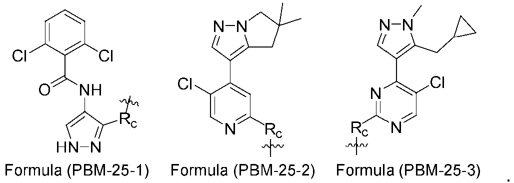

In some embodiments, PBM represents the structure of Formula (PBM-26): 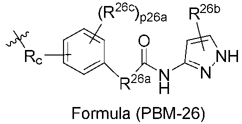
wherein R^{26a} represents a bond, C₁₋₃ alkylene (e.g., methylene, ethylene, or propylene) or halogenated C₁₋₃ alkylene (e.g., halogenated methylene, halogenated ethylene or halogenated propylene);
R^{26b} represents halogen (e.g., fluorine, chlorine, bromine, or iodine), C₁₋₆ alkoxy (e.g., C₁₋₄ alkoxy, such as methoxy, CD₃-O-, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), optionally substituted C₃₋₁₅ cycloalkyl (e.g., optionally substituted cyclopropyl, optionally substituted cyclobutyl, optionally substituted cyclopentyl, or optionally substituted cyclohexyl; or e.g., halogenated C₃₋₆ cycloalkyl, such as halogenated cyclopropyl, halogenated cyclobutyl, halogenated cyclopentyl or halogenated cyclohexyl), or optionally substituted C₁₋₆ alkyl (e.g., optionally substituted C₁₋₅ alkyl, optionally substituted C₁₋₄ alkyl, or optionally substituted C₁₋₃ alkyl);
(R^{26c})ₚ₂₆ₐ indicates that the benzene ring in Formula (PBM-26) is optionally substituted with p26a R^{26c}, with each R^{26c} being the same or different and each independently representing deuterium, halogen, hydroxy, cyano, amino, C₁₋₆ alkyl, C₁₋₆ alkoxy, halogenated C₁₋₆ alkoxy, deuterated C₁₋₆ alkyl, or halogenated C₁₋₆ alkyl; and
p26a represents an integer of 0, 1, 2, 3 or 4.

In some embodiments, R^{26a} in Formula (PBM-26) represents a bond.

In some embodiments, R^{26a} in Formula (PBM-26) represents C₁₋₃ alkylene (e.g., methylene, ethylene, or propylene), or halogenated C₁₋₃ alkylene (e.g., halogenated methylene, halogenated ethylene, or halogenated propylene).

In some embodiments, R^{26b} in Formula (PBM-26) represents optionally substituted C₃₋₁₅ cycloalkyl, e.g., optionally substituted C₃₋₈ cycloalkyl, such as optionally substituted cyclopropyl, optionally substituted cyclobutyl, optionally substituted cyclopentyl, or optionally substituted cyclohexyl. In some embodiments, C₃₋₁₅ cycloalkyl is optionally substituted with one or more (e.g., 1-3, such as 1, 2 or 3) substituents selected from the group consisting of: deuterium, hydroxy, amino, mercapto, nitro, halogen (e.g., fluorine, chlorine, bromine, iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆ cycloalkyl, C₁₋₆ alkoxy (e.g., C₁₋₄ alkoxy, such as methoxy, CD₃-O-, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or any combination thereof.

In some embodiments, R^{26b} in Formula (PBM-26) represents cyclobutyl.

In some embodiments, PBM represents the structure of Formula (PBM-26-1):

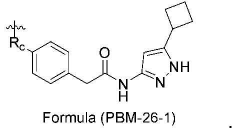

In some embodiments, PBM represents the structure of Formula (PBM-27): 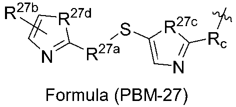
wherein R^{27a} in Formula (PBM-27) represents C₁₋₃ alkylene (e.g., methylene, ethylene, or propylene) or halogenated C₁₋₃ alkylene (e.g., halogenated methylene, halogenated ethylene or halogenated propylene); and
R^{27b} represents deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted C₃₋₆ cycloalkyl; and
R^{27c} and R^{27d} independently represent S or O.

In some embodiments, R^{27b} in Formula (PBM-27) represents optionally substituted C₃₋₆ cycloalkyl, e.g., optionally substituted cyclopropyl, optionally substituted cyclobutyl, optionally substituted cyclopentyl, or optionally substituted cyclohexyl. In some embodiments, C₃₋₆ cycloalkyl is optionally substituted with one or more (e.g., 1-3, such as 1, 2 or 3) substituents selected from the group consisting of: deuterium, hydroxy, amino, mercapto, nitro, cyano, halogen (e.g., fluorine, chlorine, bromine, iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or any combination thereof.

In some embodiments, PBM represents the structure of Formula (PBM-27-1):

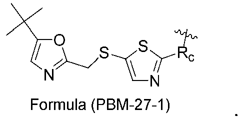

In some embodiments, PBM represents the structure of Formula (PBM-28): 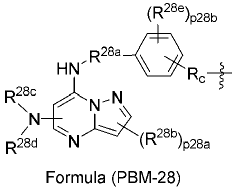
wherein R^{28a} represents C₁₋₃ alkylene (e.g., methylene, ethylene, or propylene) or halogenated C₁₋₃ alkylene (e.g., halogenated methylene, halogenated ethylene, or halogenated propylene);
(R^{28b})ₚ₂₈ₐ indicates that the pyrazolo[1,5-a]pyrimidinyl ring to which it is attached is optionally substituted with p28a R^{28b} , with each R^{28b} being the same or different and each independently representing hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p28a represents an integer of 0, 1, 2 or 3;
R^{28c} and R^{28d} are the same or different and each independently represent hydrogen, optionally substituted C₁₋₁₀ alkyl, or optionally substituted C₃₋₁₅ cycloalkyl;
(R^{28e})_{p28b} indicates that the benzene ring to which it is attached is optionally substituted with p28b R^{28e} , with each R^{28e} being the same or different and each independently representing halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p28b represents an integer of 0, 1, 2 , 3 or 4.

In some embodiments, R^{28c} and R^{28d} are the same or different and each independently represent hydrogen, optionally substituted C₁₋₁₀ alkyl or optionally substituted C₃₋₁₅ cycloalkyl. In some embodiments, examples of the optionally substituted C₁₋₁₀ alkyl include, but are not limited to, optionally substituted C₁₋₈ alkyl, optionally substituted C₁₋₆ alkyl, or optionally substituted C₁₋₄ alkyl. In some embodiments, C₁₋₁₀ alkyl is optionally substituted with one or more (e.g., 1-3, such as 1, 2 or 3) substituents selected from the group consisting of deuterium, hydroxy, amino, mercapto, nitro, halogen, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆ cycloalkyl or any combination thereof. In some embodiments, examples of the optionally substituted C₃₋₁₅ cycloalkyl include, but are not limited to, e.g., optionally substituted cyclopropyl, optionally substituted cyclobutyl, optionally substituted cyclopentyl, or optionally substituted cyclohexyl. In some embodiments, C₃₋₁₅ cycloalkyl is optionally substituted with one or more (e.g., 1-3, such as 1, 2 or 3) substituents selected from the group consisting of: deuterium, hydroxy, amino, mercapto, nitro, halogen, cyano, C₁₋₆ alkyl, halogenated C₁₋₆ alkyl, deuterated C₁₋₆ alkyl, C₃₋₆ cycloalkyl, C₁₋₆ alkoxy, halogenated C₁₋₆ alkoxy, or any combination thereof.

In some embodiments, R^{28c} in Formula (PBM-28) represents hydrogen, and R^{28d} represents

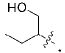

In some embodiments, PBM represents a structure of Formulae (PBM-28-1) or (PBM-28-2):

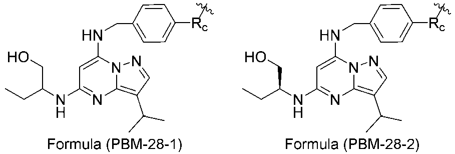

In some embodiments, PBM represents the structure of Formula (PBM-29): 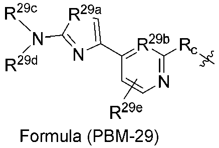
wherein R^{29a} in Formula (PBM-29) represents -S- or a fragment 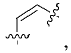 wherein when R^{29a} represents - S-, the heteroaryl ring containing R^{29a} and nitrogen atom in Formula (PBM-29) is thiazolyl ring, and when
R^{29a} represents the fragment 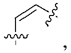 the heteroaryl ring containing R^{29a} and nitrogen atom in Formula (PBM-29) is pyridyl ring;
R^{29b} represents N or CH;
R^{29c} represents hydrogen or optionally substituted C₁₋₁₀ alkyl;
R^{29d} represents hydrogen, -C₁₋₃ alkylene-optionally substituted 4- to 10-membered heterocyclyl, or optionally substituted 4- to 10-membered heterocyclyl; and
R^{29e} represents hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{29a} in Formula (PBM-29) represents -S-, the heteroaryl ring containing R^{29a} and nitrogen atom in Formula (PBM-29) is thiazolyl.

In some embodiments, R^{29a} in Formula (PBM-29) represents the fragment 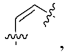 the heteroaryl ring containing R^{29a} and nitrogen atom in Formula (PBM-29) is pyridyl.

**In** some embodiments, R^{29c} in Formula (PBM-29) represents hydrogen or optionally substituted C₁₋₁₀ alkyl. In some embodiments, examples of the optionally substituted C₁₋₁₀ alkyl include, but are not limited to, optionally substituted C₁₋₈ alkyl, optionally substituted C₁₋₆ alkyl, or optionally substituted C₁₋₄ alkyl. In some embodiments, C₁₋₁₀alkyl is optionally substituted with one or more (e.g., 1-3, such as 1, 2 or 3) substituents selected from the group consisting of deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆ cycloalkyl, or any combination thereof.

**In** some embodiments, R^{29d} in Formula (PBM-29) represents hydrogen, -C₁₋₃ alkylene-optionally substituted 4- to 10-membered heterocyclyl, or optionally substituted 4- to 10-membered heterocyclyl. In some embodiments, each optionally substituted 4- to 10-membered heterocyclyl is independently e.g., 4- to 10-membered (e.g., 4- to 9-membered, 4- to 8-membered, 5- to 7-membered, or 4- to 6-membered) monocyclic or bicyclic heterocyclyl group containing one or more (e.g., 1-4, or 1, 2, 3, or 4) heteroatoms selected from nitrogen, oxygen, and sulfur, including but not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl). In some embodiments, the 4- to 10-membered heterocyclyl is optionally substituted with a substituent selected from the group consisting of deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆ cycloalkyl, or any combination thereof.

In some embodiments, PBM represents a structure of Formulae (PBM-29-1A) or (PBM-29-1B): 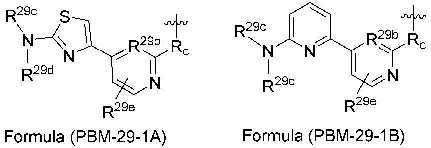
wherein R^{29b} represents N or CH;
R^{29c} represents hydrogen or optionally substituted C₁₋₁₀ alkyl;
R^{29d} represents hydrogen, -C₁₋₃ alkylene-optionally substituted 4- to 10-membered heterocyclyl, or optionally substituted 4- to 10-membered heterocyclyl; and
R^{29c} represents hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

**In** some embodiments, R^{29c} in each of Formulae (PBM-29-1A) and (PBM-29-1B) independently represents hydrogen or optionally substituted C₁₋₁₀ alkyl. In some embodiments, examples of the optionally substituted C₁₋₁₀ alkyl include, but are not limited to, optionally substituted C₁₋₈ alkyl, optionally substituted C₁₋₆ alkyl, or optionally substituted C₁₋₄ alkyl. In some embodiments, C₁₋₁₀ alkyl is optionally substituted with one or more (e.g., 1-3, such as 1, 2 or 3) substituents selected from the group consisting of deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆ cycloalkyl, or any combination thereof.

**In** some embodiments, R^{29d} in each of Formulae (PBM-29-1A) and (PBM-29-1B) independently represents hydrogen, -C₁₋₃ alkylene-optionally substituted 4- to 10-membered heterocyclyl, or optionally substituted 4- to 10-membered heterocyclyl. In some embodiments, each optionally substituted 4- to 10-membered heterocyclyl is independently e.g., 4- to 10-membered (e.g., 4- to 9-membered, 4- to 8-membered, 5- to 7-membered, or 4- to 6-membered) monocyclic, bicyclic, or polycyclic heterocyclyl group containing one or more (e.g., 1-4, or 1, 2, 3, or 4) heteroatoms selected from nitrogen, oxygen, and sulfur, including but not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 10-membered heterocyclyl is optionally substituted with a substituent selected from the group consisting of deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF_{3C}H₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆ cycloalkyl, or any combination thereof.

**In** some embodiments, PBM represents a structure of Formulae (PBM-29-1) or (PBM-29-2):

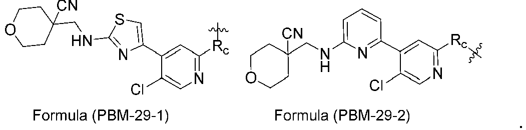

In some embodiments, PBM represents the structure of Formula (PBM-30): 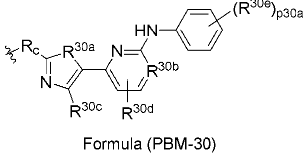
wherein R^{30a} in Formula (PBM-30) represents O or S;
R^{30b} represents N or CH;
R^{30c} and R^{30d} are the same or different and each independently represent hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₅ alkoxy, C₁₋₄ alkoxy, or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
(R^{30c})ₚ₃₀ₐ indicates that the benzene ring to which it is attached is optionally substituted with p30a R^{30c} , with each R^{30c} being the same or different and each independently representing deuterium, hydroxy, halogen (e.g., fluorine, chlorine, bromine, or iodine), amino, cyano, C₁₋₆ alkoxy (e.g., C₁₋₅ alkoxy, C₁₋₄ alkoxy, or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), - S(O)₂NH₂ or -C(O)NH₂;
p30a represents an integer of 0, 1, 2 , 3, 4 or 5.

**In** some embodiments, R^{30c} in Formula (PBM-30) represents -S(O)₂NH₂.

**In** some embodiments, R^{30c} in Formula (PBM-30) represents -C(O)NH₂ .

**In** some embodiments, PBM represents a structure of Formulae (PBM-30-1) or (PBM-30-2):

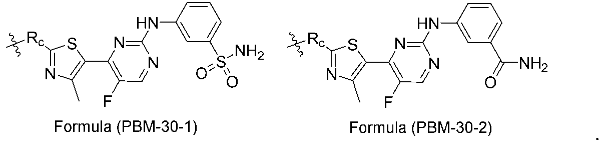

In some embodiments, PBM represents a small molecule ligand of CDK2.

In some embodiments, PBM represents the structure of Formula (PBM-55): 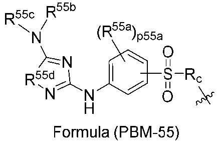
wherein (R^{55a})ₚ₅₅ₐ in Formula (PBM-55) represents p55a R^{55a} groups, wherein each R^{55a} is the same or different and each independently represents halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₅ alkoxy, C₁₋₄ alkoxy, or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p55a represents an integer of 0, 1, 2, 3 or 4;
R^{55b} and R^{55c} are the same or different and each independently represent hydrogen, C₁₋₆ alkyl, halogenated C₁₋₆ alkyl, deuterated C₁₋₆ alkyl, or optionally substituted aryl;
R^{55d} **in** Formula (PBM-55) represents a fragment 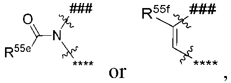 wherein symbol ### indicates the point of attachment to the ring carbon atom; and symbol **** indicates the point of attachment to the ring nitrogen atom; and R^{55c} represents optionally substituted aryl or optionally substituted heteroaryl; and R^{55f} represents hydrogen, halogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.).

In some embodiments, R^{55b} and R^{55c} are the same or different and each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or optionally substituted aryl (e.g., C₅₋₂₀ aryl, C₅₋₁₅ aryl, C₅₋₁₀ aryl, C₅₋₆ aryl, or C₆₋₂₀ aryl). Examples of the aryl include, but are not limited to, phenyl and naphthyl. The aryl is optionally substituted with one or more (e.g., 1-5, or 1-3) substituents selected from the group consisting of halogen (e.g., fluorine, chlorine, bromine, or iodine), C(O)NH₂, hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, R^{55b} and R^{55c} independently represent hydrogen.

In some embodiments, R^{55e} represents optionally substituted aryl (e.g., C₅₋₂₀ aryl, C₅₋₁₅ aryl, C₅₋₁₀ aryl, C₅₋₆ aryl, or C₆₋₂₀ aryl). Examples of the aryl include, but are not limited to, phenyl and naphthyl. The aryl is optionally substituted with one or more (e.g., 1-5, or 1-3) substituents selected from the group consisting of halogen (e.g., fluorine, chlorine, bromine, or iodine), C(O)NH₂, hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

**In** some embodiments, R^{55e} represents optionally substituted heteroaryl. Examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-furo[3,2-b]pyrrolyl, pyrrolo[2,1-b]thiazolyl, and imidazo[2,1-b]thiazolyl. The heteroaryl is optionally substituted with one or more (e.g., 1-5, or 1-3) substituents selected from the group consisting of halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, PBM represents a structure of Formulae (PBM-55-1A) or (PBM-55-1B): 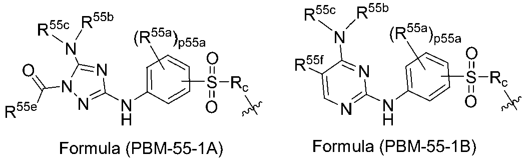 wherein (R^{55a})ₚ₅₅ₐ, R^{55b}, R^{55c}, R^{55e}, and R^{55f} are as defined in the structure of Formula (PBM-55).

In some embodiments, PBM represents a structure of Formulae (PBM-55-1), (PBM-55-2), or (PBM-55-3):

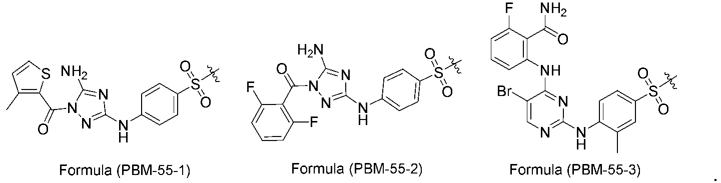

In some embodiments, PBM represents a small molecule ligand of EZH2.

In some embodiments, PBM represents the structure of Formula (PBM-31): 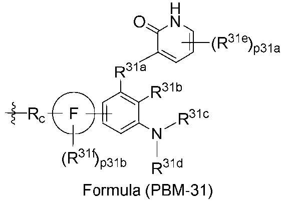
wherein R^{31a} in Formula (PBM-31) represents C(O)NH, NHC(O), (CH₂)₁₋₂C(O)NH, (CH₂)₁₋₂NHC(O), C(O)NH(CH₂)₁₋₂, NHC(O)(CH₂)₁₋₂, S(O)₂NH or NHS(O)₂;
R^{31b} represents hydrogen, hydroxy, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₅ alkoxy, C₁₋₄ alkoxy, or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R^{31c} represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); or
R^{31b} and R^{31c}, together with their respective attached carbon and nitrogen atoms and the carbon atom on the benzene ring attached to the nitrogen atom, form an optionally substituted 4- to 6-membered heteroaromatic ring or an optionally substituted 4- to 6-membered heterocycle ring;
R^{31d} represents optionally substituted C₃₋₆ cycloalkyl (e.g., optionally substituted cyclopropyl, optionally substituted cyclobutyl, optionally substituted cyclopentyl, or optionally substituted cyclohexyl), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 20-membered heterocyclyl;
(R^{31e})ₚ₃₁ₐ incicates that pyridin-2(1H)-one ring to which it is attached is substituted with p31a R^{31c}, with each R^{31c} being the same or different and each independently representing hydroxy, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p31a represents an integer of 0, 1, 2 or 3;
ring F in Formula (PBM-31) represents arylene, or 5- to 20-membered monocyclic or bicyclic heteroarylene group containing 1 to 4 heteroatoms selected from N, O, and S; and (R^{31f})_{p31b} indicates that the ring F is optionally substituted with p31b R^{31f} , with each R^{31f} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF_{3C}H₂-O- or CH₂ClH₂-O-); and
p31b represents an integer of 0, 1, 2, 3 or 4.

**In** some embodiments, R^{31b} in Formula (PBM-31) represents hydrogen, hydroxy, halogen (e.g., fluorine, chlorine, bromine, or iodine), cyano, C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

**In** some embodiments, R^{31c} in Formula (PBM-31) represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

**In** some embodiments, in Formula (PBM-31), R^{31b} and R^{31c}, together with their respective attached carbon and nitrogen atoms and the carbon atom on the benzene ring attached to the nitrogen atom, form an optionally substituted 4- to 6-membered heteroaromatic ring (e.g., optionally substituted 4- to 6-membered nitrogen-containing heteroaromatic ring) or an optionally substituted 4- to 6-membered heterocycle ring (e.g., optionally substituted 4- to 6-membered nitrogen-containing heterocycle ring). In some embodiments, R^{31b} and R^{31c} in Formula (PBM-31) together represent the following fragment: -CH₂-, -CH=CH-, -CH=N-, - N=CH-, -N=N-, -CH₂-CH₂-, -NH-CH₂-, or -CH₂-CH₂-CH₂-. In some embodiments, the 4- to 6-membered nitrogen-containing heterocycle ring includes, but is not limited to, e.g., 4- to 6-membered (e.g., 4-membered, 5-membered or 6-membered) heterocycle containing one nitrogen atom and optionally containing heteroatoms selected from nitrogen, oxygen, and sulfur. The 4- to 6-membered nitrogen-containing heterocycle is fused with the benzene ring in Formula (PBM-31) to form a fused ring system. The 4- to 6-membered nitrogen-containing heterocycle is optionally substituted with one or more (e.g., 1 to 4, 1 to 3, or 2) substituents selected from the group consisting of: halogen (e.g., fluorine, chlorine, bromine, or iodine), hydroxy, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.). In some embodiments, R^{31d} in Formula (PBM-31) represents optionally substituted C₃₋₆ cycloalkyl (e.g., optionally substituted cyclopropyl, optionally substituted cyclobutyl, optionally substituted cyclopentyl or optionally substituted cyclohexyl), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 20-membered heterocyclyl.

In some embodiments, R^{31d} in Formula (PBM-31) represents optionally substituted C₃₋₆ cycloalkyl, e.g., optionally substituted cyclopropyl, optionally substituted cyclobutyl, optionally substituted cyclopentyl, or optionally substituted cyclohexyl. In some embodiments, C₃₋₆ cycloalkyl is optionally substituted with one or more (e.g., 1-3, such as 1, 2 or 3) substituents selected from the group consisting of: deuterium, hydroxy, amino, mercapto, nitro, halogen, cyano, C₁₋₆ alkyl, deuterated C₁₋₆ alkyl, halogenated C₁₋₆ alkyl, C₃₋₆ cycloalkyl, C₁₋₆ alkoxy, halogenated C₁₋₆ alkoxy, or any combination thereof.

In some embodiments, R^{31d} in Formula (PBM-31) represents optionally substituted 4- to 20-membered heterocyclyl. The optionally substituted 4- to 20-membered heterocyclyl is e.g., optionally substituted 4- to 20-membered (e.g., 4- to 15-membered, 4- to 9-membered, 4- to 8-membered, 5- to 7-membered, or 4- to 6-membered) monocyclic, bicyclic, or polycyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 20-membered heterocyclyl is optionally substituted with a subsituent selected from the group consisting of deuterium, hydroxy, amino, mercapto, nitro, halogen, cyano, C₁₋₆ alkyl, deuterated C₁₋₆ alkyl, halogenated C₁₋₆ alkyl, C₃₋₆ cycloalkyl, C₁₋₆ alkoxy, halogenated C₁₋₆ alkoxy or any combination thereof.

In some embodiments, ring F in Formula (PBM-31) represents arylene (e.g., C₅₋₂₀ arylene, C₅₋₁₅ arylene, C₅₋₁₀ arylene, or C₅₋₆ arylene), or 5- to 20-membered (e.g., 5- to 15-membered, 5- to 10-membered, 5- to 9-membered, 5- to 8-membered, 5- to 7-membered, or 6-membered) monocyclic or bicyclic heteroarylene group containing from 1 to 4 (e.g., from 1 to 3, or from 1 to 2) heteroatoms selected from N, O, and S. Examples of the arylene include, but are not limited to, phenylene and naphthylene. Examples of the heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-furo[3,2-b]pyrrolylene, pyrrolo[2,1-b]thiazolylene, and imidazo[2,1-b]thiazolylene. In some embodiments, the ring F is optionally substituted with p31b R^{31f}, with each R^{31f} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClH₂-O-); and p31b represents an integer of 0, 1, 2, 3 or 4.

In some embodiments, PBM represents a structure of Formulae (PBM-31-1A), (PBM-31-1B), (PBM-31-1C), (PBM-31-1D), (PBM-31-1E), (PBM-31-1F), (PBM-31-1G), (PBM-31-1H), (PBM-31-11), (PBM-31-1J), or (PBM-31-1K): 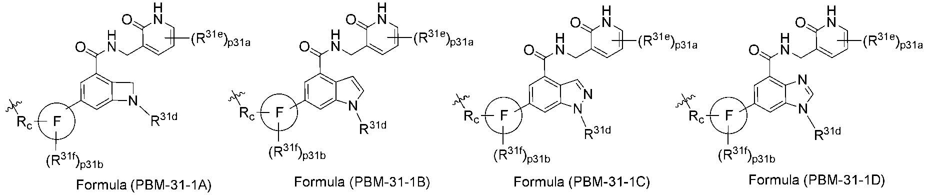 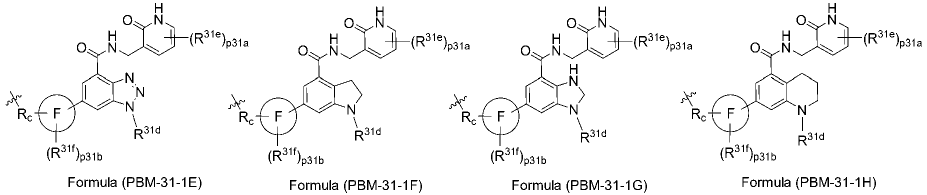 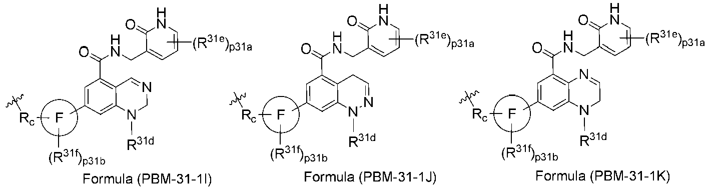
wherein R^{31d} in each of the above formulae independently represents optionally substituted C₃₋₆ cycloalkyl (e.g., optionally substituted cyclopropyl, optionally substituted cyclobutyl, optionally substituted cyclopentyl, or optionally substituted cyclohexyl), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 10-membered heterocyclyl;
(R^{31e})ₚ₃₁ₐ in each of the above formulae independently indicates that pyridin-2(1H)-one ring to which it is attached is substituted with p31a R^{31c}, with each R^{31c} being the same or different and each independently representing hydroxy, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p31a in each of the above formulae independently represents an integer of 0, 1, 2 or 3;
ring F in each of the above formulae independently represents arylene, or 5- to 20-membered monocyclic or bicyclic heteroarylene group containing 1 to 4 heteroatoms selected from N, O, and S; and (R^{31f})_{p31b} indicates that the ring F is optionally substituted with p31b R^{31f} , with each R^{31f} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClH₂-O-); and
p31b in each of the above formulae independently represents an integer of 0, 1, 2, 3 or 4.

In some embodiments, R^{31d} in each of Formulae (PBM-31-1A), (PBM-31-1B), (PBM-31-1C), (PBM-31-1D), (PBM-31-1E), (PBM-31-1F), (PBM-31-1G), (PBM-31-1H), (PBM-31-11), (PBM-31-1J), and (PBM-31-1K) independently represents optionally substituted C₃₋₆ cycloalkyl, e.g., optionally substituted cyclopropyl, optionally substituted cyclobutyl, optionally substituted cyclopentyl, or optionally substituted cyclohexyl. In some embodiments, C₃₋₆ cycloalkyl is optionally substituted with one or more (e.g., 1-3, such as 1, 2 or 3) substituents selected from the group consisting of: deuterium, hydroxy, amino, mercapto, nitro, halogen, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, or any combination thereof.

In some embodiments, R^{31d} in each of Formulae (PBM-31-1A), (PBM-31-1B), (PBM-31-1C), (PBM-31-1D), (PBM-31-1E), (PBM-31-1F), (PBM-31-1G), (PBM-31-1H), (PBM-31-11), (PBM-31-1J), and (PBM-31-1K) independently represents optionally substituted 4- to 20-membered heterocyclyl. The optionally substituted 4- to 20-membered heterocyclyl is e.g., optionally substituted 4- to 20-membered (e.g., 4- to 15-membered, 4- to 12-membered, 4- to 9-membered, 4- to 8-membered, 5- to 7-membered, or 4- to 6-membered) monocyclic, bicyclic, or polycyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 20-membered heterocyclyl is optionally substituted with a substituent selected from the group consisting of deuterium, hydroxy, amino, mercapto, nitro, halogen, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, or any combination thereof.

In some embodiments, ring F of each of Formulae (PBM-31-1A), (PBM-31-1B), (PBM-31-1C), (PBM-31-1D), (PBM-31-1E), (PBM-31-1F), (PBM-31-1G), (PBM-31-1H), (PBM-31-11), (PBM-31-1J), and (PBM-31-1K) independently represents arylene (e.g., C₅₋₂₀ arylene, C₅₋₁₅ arylene, C₅₋₁₀ arylene, or C₅₋₆ arylene), or 5- to 20-membered (e.g., 5- to 15-membered, 5- to 10-membered, 5- to 9-membered, 5- to 8-membered, 5- to 7-membered, or 6-membered) monocyclic or bicyclic heteroarylene group containing from 1 to 4 (e.g., from 1 to 3, or from 1 to 2) heteroatoms selected from N, O, and S. Examples of the arylene include, but are not limited to, phenylene and naphthylene. Examples of the heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-furo[3,2-b]pyrrolylene, pyrrolo[2,1-b]thiazolylene, and imidazo[2,1-b]thiazolylene. In some embodiments, the ring F is optionally substituted with p31b R^{31f} , with each R^{31f} being the same or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and p31b represents an integer of 0, 1, 2, 3 or 4.

In some embodiments, PBM represents a structure of Formulae (PBM-31-1), (PBM-31-2), or (PBM-31-3):

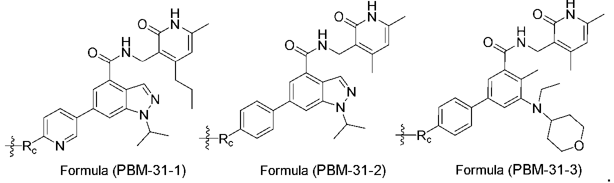

In some embodiments, PBM represents a small molecule ligand of NTRK.

In some embodiments, PBM represents the structure of Formula (PBM-32): 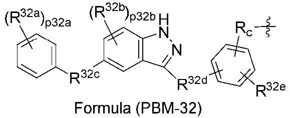
wherein R^{32c} in Formula (PBM-32) represents optionally substituted C₁₋₃ alkylene;
(R^{32a})ₚ₃₂ₐ in each of the above formulae independently indicates that the benzene ring to which it is attached is substituted with p32ₐ R^{32a} , with each R^{32a} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p32a represents an integer of 0, 1, 2, 3, 4 or 5;
(R^{32b})_{p32b} in each of the above formulae independently indicates that the 1H-indazole ring to which it is attached is substituted with p32b R^{32b} , with each R^{32b} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p32b represents an integer of 0, 1, 2, 3, 4 or 5;
R^{32d} represents C(O)NH, NHC(O), S(O)₂NH or NHS(O)₂; and
R^{32c} in each of the above formulae independently represents NR^{32f}R^{32g}, where R^{32f} and R^{32g} are the same or different and each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₆ cycloalkyl, or optionally substituted 4- to 8-membered heterocyclyl group.

In some embodiments, each R^{32f} in Formula (PBM-32) independently represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₆ cycloalkyl or optionally substituted 4- to 8-membered heterocyclyl. In some embodiments, examples of the optionally substituted C₃₋₆ cycloalkyl include, but are not limited to, e.g., optionally substituted cyclopropyl, optionally substituted cyclobutyl, optionally substituted cyclopentyl, or optionally substituted cyclohexyl. In some embodiments, C₃₋₆ cycloalkyl is optionally substituted with one or more (e.g., 1-3, such as 1, 2 or 3) substituents selected from the group consisting of: deuterium, hydroxy, amino, mercapto, nitro, halogen, cyano, C₁₋₆ alkyl, deuterated C₁₋₆ alkyl, halogenated C₁₋₆ alkyl, C₃₋₆ cycloalkyl, C₁₋₆ alkoxy, halogenated C₁₋₆ alkoxy or any combination thereof. In some embodiments, optionally substituted 4- to 8-membered heterocyclyl is e.g., optionally substituted 4- to 8-membered (e.g., 4- to 7-membered, 4- to 6-membered, 5- to 7-membered, or 5-to 6-membered) monocyclic or bicyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, and diazacyclooctyl. In some embodiments, the 4- to 8-membered heterocyclyl is optionally substituted with a substituent selected from the group consisting of deuterium, hydroxy, amino, mercapto, nitro, halogen, cyano, C₁₋₆ alkyl, deuterated C₁₋₆ alkyl, halogenated C₁₋₆ alkyl, C₃₋₆ cycloalkyl, C₁₋₆ alkoxy, halogenated C₁₋₆ alkoxy, or any combination thereof.

In some embodiments, each R^{32g} in Formula (PBM-32) independently represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₆ cycloalkyl, or optionally substituted 4- to 8-membered heterocyclyl. In some embodiments, examples of the optionally substituted C₃₋₆ cycloalkyl include, but are not limited to, e.g., optionally substituted cyclopropyl, optionally substituted cyclobutyl, optionally substituted cyclopentyl, or optionally substituted cyclohexyl. In some embodiments, C₃₋₆ cycloalkyl is optionally substituted with one or more (e.g., 1-3, such as 1, 2 or 3) substituents selected from the group consisting of: deuterium, hydroxy, amino, mercapto, nitro, halogen, cyano, C₁₋₆ alkyl, deuterated C₁₋₆ alkyl, halogenated C₁₋₆ alkyl, C₃₋₆ cycloalkyl, C₁₋₆ alkoxy, halogenated C₁₋₆ alkoxy, or any combination thereof. In some embodiments, the optionally substituted 4- to 8-membered heterocyclyl is e.g., optionally substituted 4- to 8-membered (e.g., 4- to 7-membered, 4- to 6-membered, 5- to 7-membered, or 5-to 6-membered) monocyclic or bicyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 8-membered heterocyclyl is optionally substituted with a substituent selected from the group consisting of deuterium, hydroxy, amino, mercapto, nitro, halogen, cyano, C₁₋₆ alkyl, deuterated C₁₋₆ alkyl, halogenated C₁₋₆ alkyl, C₃₋₆ cycloalkyl, C₁₋₆ alkoxy, halogenated C₁₋₆ alkoxy, or any combination thereof.

In some embodiments, PBM represents the structure of Formula (PBM-32-1):

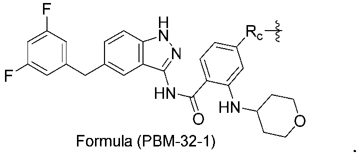

In some embodiments, PBM represents the structure of Formula (PBM-33): 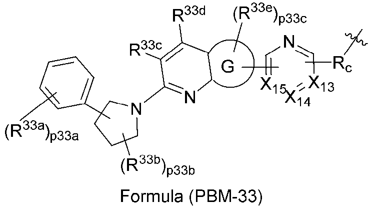
wherein X₁₃, X₁₄, and X₁₅ in Formula (PBM-33) are the same or different and each independently represent CH or N, wherein X₁₃, X₁₄ and X₁₅ are not simultaneously N, and the 6-membered ring containing X₁₃, X₁₄ and X₁₅ is optionally substituted with 1 or 2 substituent selected from the group consisting of halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or any combination thereof;
ring G in Formula (PBM-33) represents pyrrole ring, imidazole ring, pyrazole ring, benzene ring, pyridine ring, pyrimidine ring, or pyrazine ring;
(R^{33a})ₚ₃₃ₐ indicates that the benzene ring to which it is attached is optionally substituted with p33a R^{33a} , with each R^{33a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p33a represents an integer of 0, 1, 2, 3, 4 or 5;
(R^{33b})_{p33b} indicates that the pyrrolidine ring to which it is attached is optionally substituted with p33b R^{33b} , with each R^{33b} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p33b represents an integer of 0, 1, 2, 3, 4, 5 or 6;
R^{33c} and R^{33d} are the same or different and each independently represent hydrogen, halogen (e.g., fluorine, chlorine, bromine, or iodine), hydroxy, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
(R^{33c})_{p33c} indicates that the ring G to which it is attached is optionally substituted with p33c R^{33c} , with each R^{33c} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p33c represents an integer of 0 or 1.

In some embodiments, X₁₃, X₁₄, and X₁₅ in Formula (PBM-33) represent CH.

In some embodiments, X₁₃ in Formula (PBM-33) represents N, and X₁₄ and X₁₅ represent CH.

In some embodiments, X₁₄ in Formula (PBM-33) represents N, and X₁₃ and X₁₅ represent CH.

In some embodiments, X₁₅ in Formula (PBM-33) represents N, and X₁₃ and X₁₄ represent CH.

In some embodiments, X₁₅ in Formula (PBM-33) represents CH, and X₁₃ and X₁₄ represent N.

In some embodiments, PBM represents a structure of Formulae (PBM-33-1A), (PBM-33-1B), (PBM-33-1C), (PBM-33-1D), (PBM-33-1E), (PBM-33-1F), (PBM-33-1G), (PBM-33-1H), (PBM-33-1I), or (PBM-33-1J): 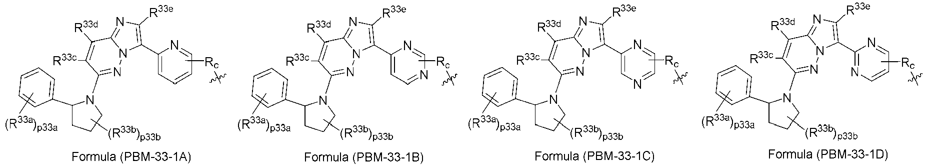 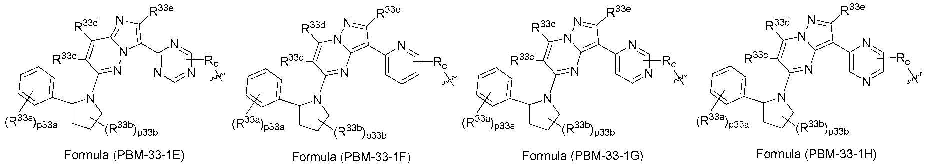 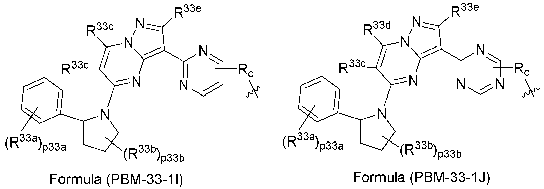
wherein (R^{33a})ₚ₃₃ₐ in each of the above formulae independently indicates that the benzene ring to which it is attached is optionally substituted with p33a R^{33a} , with each R^{33a} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p33a represents an integer of 0, 1, 2, 3, 4 or 5;
(R^{33b})_{p33b} in each of the above formulae independently indicates that the pyrrolidine ring to which it is attached is optionally substituted with p33b R^{33b} , with each R^{33b} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p33b represents an integer of 0, 1, 2, 3, 4, 5 or 6;
R^{33c} and R^{33a} in each of the above formulae are the same or different and each independently represent hydrogen, halogen (e.g., fluorine, chlorine, bromine, or iodine), hydroxy, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R^{33c} represents hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents a structure of Formulae (PBM-33-1), (PBM-33-2), (PBM-33-3), (PBM-33-4), (PBM-33-5), (PBM-33-6) or (PBM-33-7):

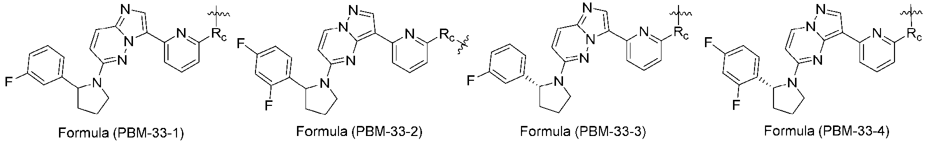

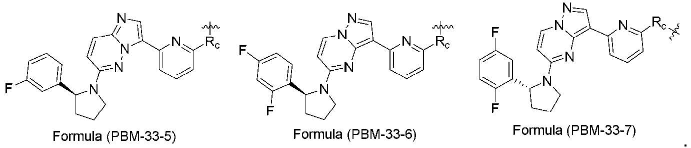

In some embodiments, PBM represents a small molecule ligand of SHP2.

In some embodiments, PBM represents the structure of Formula (PBM-34): 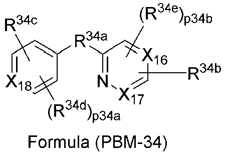
wherein X₁₆, X₁₇ and X₁₈ in Formula (PBM-34) each independently represent CH or N;
R^{34a} represents a bond, S or NH;
R^{34b} represents a bond, optionally substituted cycloalkyl, or optionally substituted 4- to 15-membered nitrogen-containing heterocyclyl;
R^{34c} represents a bond, halogen (e.g., fluorine, chlorine, bromine, or iodine), amino, hydroxy, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted cycloalkyl (e.g., optionally substituted C₃₋₁₅ cycloalkyl), or optionally substituted 4- to 15-membered nitrogen-containing heterocyclyl;
wherein R^{34b} and R^{34c} do not simultaneously represent a bond, and only one of R^{34b} and R^{34c}represents a bond, wherein when R^{34b} represents a bond, the ring carbon atom connected to R^{34b} is directly bonded to R_{c}, and when R^{34c} represents a bond, the ring carbon atom connected to R^{34c} is directly bonded to R_{c} ;
(R^{34d})ₚ₃₄ₐ indicates that the 6-membered ring to which it is attached is optionally substituted with p34a R^{34d} , with each R^{34d} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p34a represents an integer of 0, 1, 2, 3 or 4;
(R^{34c})_{p34b} indicates that the 6-membered nitrogen-containing heteroaryl ring to which it is attached is optionally substituted with p34b R^{34c} , with each R^{34c} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p34b represents an integer of 0, 1 or 2.

In some embodiments, R^{34b} in Formula (PBM-34) represents a bond, and R^{34c} represents halogen (e.g., fluorine, chlorine, bromine, or iodine), amino, hydroxy, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted cycloalkyl (e.g., optionally substituted C₃₋₁₅ cycloalkyl), or optionally substituted 4- to 15-membered nitrogen-containing heterocyclyl. In some embodiments, examples of the optionally substituted cycloalkyl include, but are not limited to, optionally substituted C₃₋₁₅ cycloalkyl, optionally substituted C₃₋₁₀ cycloalkyl or C₃₋₆ cycloalkyl, e.g., cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, 2,3-dihydro-1H-indenyl, spiro-cycloalkyl (e.g., C₅₋₁₅ spiro-cycloalkyl), adamantanyl, noradamantanyl, and norbornyl. The optional substituents of the optionally substituted cycloalkyl are optionally selected from the group consisting of halogen (e.g., fluorine, chlorine, bromine, or iodine), hydroxy, mercapto, cyano, amino, nitro, oxo, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In some embodiments, the optionally substituted 4- to 15-membered nitrogen-containing heterocyclyl is e.g., optionally substituted 4- to 15-membered (e.g., 4- to 10-membered, 4- to 9-membered, 4- to 8-membered, 5- to 7-membered, or 4- to 6-membered) monocyclic, bicyclic, or polycyclic heterocycle containing one nitrogen atom and optionally containing one or more (e.g., 1-3, or 1, 2, or 3) heteroatoms selected from nitrogen, oxygen, and sulfur, including but not limited to the following optionally substituted groups: azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 15-membered nitrogen-containing heterocyclyl is optionally substituted with a substituent selected from the group consisting of deuterium, hydroxy, amino, mercapto, nitro, halogen (e.g., fluorine, chlorine, bromine, or iodine), cyano, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆ cycloalkyl (e.g., cyclopropyl, cyclobutyl, cyclopentyl, or cyclohexyl), or any combination thereof.

In some embodiments, R^{34c} in Formula (PBM-34) represents a bond, and R^{34b}) represents an optionally substituted 4- to 15-membered nitrogen-containing heterocyclyl. In some embodiments, the optionally substituted 4- to 15-membered nitrogen-containing heterocyclyl is e.g., optionally substituted 4- to 15-membered (e.g., 4- to 10-membered, 4- to 9-membered, 4- to 8-membered, 5- to 7-membered, or 4- to 6-membered) monocyclic, bicyclic, or polycyclic heterocycle containing one nitrogen atom and optionally containing one or more (e.g., 1-3, or 1, 2, or 3) heteroatoms selected from nitrogen, oxygen, and sulfur, including but not limited to the following optionally substituted groups: azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 15-membered nitrogen-containing heterocyclyl is optionally substituted with a substituent selected from the group consisting of deuterium, hydroxy, amino, mercapto, nitro, halogen (e.g., fluorine, chlorine, bromine, or iodine), cyano, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆ cycloalkyl (e.g., cyclopropyl, cyclobutyl, cyclopentyl, or cyclohexyl), or any combination thereof.

In some embodiments, R^{34a} in Formula (PBM-34) represents a bond.

In some embodiments, R^{34a} in Formula (PBM-34) represents -S-.

In some embodiments, R^{34a} in Formula (PBM-34) represents NH.

In some embodiments, X₁₈ in Formula (PBM-34) represents CH.

In some embodiments, X₁₈ in Formula (PBM-34) represents N.

In some embodiments, PBM represents a structure of Formulae (PBM-34-1A), (PBM-34-1B), (PBM-34-1C), (PBM-34-1D), (PBM-34-1E), (PBM-34-1F), (PBM-34-1G), or (PBM-34-1H): 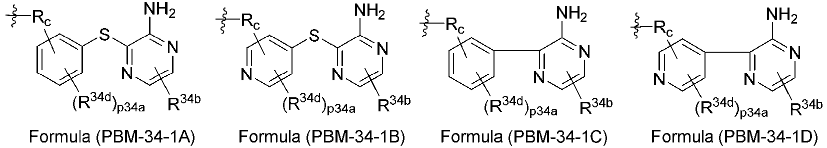 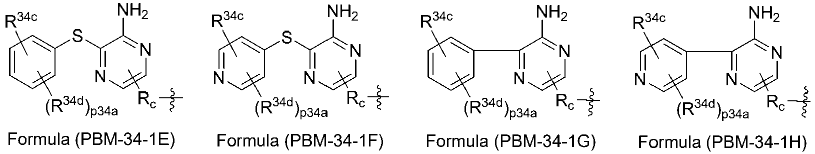
wherein R^{34b} in each of the above formulae independently represents optionally substituted cycloalkyl (e.g., optionally substituted C₃₋₁₅ cycloalkyl), or optionally substituted 4- to 15-membered nitrogen-containing heterocyclyl;
R^{34c} in each of the above formulae independently represents halogen (e.g., fluorine, chlorine, bromine, or iodine), amino, hydroxy, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted cycloalkyl (e.g., optionally substituted C₃₋₁₅ cycloalkyl), or optionally substituted 4- to 15-membered nitrogen-containing heterocyclyl;
(R^{34d})ₚ₃₄ₐ in each of the above formulae independently indicates that the 6-membered ring to which it is attached is optionally substituted with p34a R^{34d} , with each R^{34d} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p34a represents an integer of 0, 1, 2, 3 or 4.

In some embodiments, each R^{34c} in each of Formulae (PBM-34-1E), (PBM-34-1F), (PBM-34-1G), and (PBM-34-1H) independently represents halogen (e.g., fluorine, chlorine, bromine, or iodine), amino, hydroxy, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted cycloalkyl (e.g., optionally substituted C₃₋₁₅ cycloalkyl), or optionally substituted 4- to 15-membered nitrogen-containing heterocyclyl. In some embodiments, examples of the optionally substituted cycloalkyl include, but are not limited to, optionally substituted C₃₋₁₅ cycloalkyl, optionally substituted C₃₋₁₀ cycloalkyl or C₃₋₆ cycloalkyl, e.g., cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, 2,3-dihydro-1H-indenyl, spiro-cycloalkyl (e.g., C₅₋₁₅ spiro-cycloalkyl), adamantanyl, noradamantanyl, and norbornyl. The optional substituents of the optionally substituted cycloalkyl are optionally selected from the group consisting of halogen (e.g., fluorine, chlorine, bromine, or iodine), hydroxy, mercapto, cyano, amino, nitro, oxo, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, isobutoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In some embodiments, the optionally substituted 4- to 15-membered nitrogen-containing heterocyclyl is e.g., optionally substituted 4- to 15-membered (e.g., 4- to 10-membered, 4- to 9-membered, 4- to 8-membered, 5- to 7-membered, or 4- to 6-membered) monocyclic, bicyclic, or polycyclic heterocycle containing one nitrogen atom and optionally containing one or more (e.g., 1-3, or 1, 2, or 3) heteroatoms selected from nitrogen, oxygen, and sulfur, including but not limited to the following optionally substituted groups: azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 15-membered nitrogen-containing heterocyclyl is optionally substituted with a substituent selected from the group consisting of deuterium, hydroxy, amino, mercapto, nitro, halogen (e.g., fluorine, chlorine, bromine, or iodine), cyano, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆ cycloalkyl (e.g., cyclopropyl, cyclobutyl, cyclopentyl, or cyclohexyl), or any combination thereof.

In some embodiments, each R^{34b} in each of Formulae (PBM-34-1A), (PBM-34-1B), (PBM-34-1C), and (PBM-34-1D) independently represents optionally substituted 4- to 15-membered nitrogen-containing heterocyclyl. In some embodiments, the optionally substituted 4- to 15-membered nitrogen-containing heterocyclyl is e.g., optionally substituted 4- to 15-membered (e.g., 4- to 10-membered, 4- to 9-membered, 4-to 8-membered, 5- to 7-membered, or 4- to 6-membered) monocyclic, bicyclic, or polycyclic heterocyclyl group containing one or more (e.g., 1-3, or 1, 2, or 3) heteroatoms selected from nitrogen, oxygen, and sulfur, including but not limited to the following optionally substituted groups: azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 15-membered nitrogen-containing heterocyclyl is optionally substituted with a substituent selected from the group consisting of deuterium, hydroxy, amino, mercapto, nitro, halogen (e.g., fluorine, chlorine, bromine, or iodine), cyano, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆ cycloalkyl (e.g., cyclopropyl, cyclobutyl, cyclopentyl, or cyclohexyl), or any combination thereof.

In some embodiments, PBM represents a structure of Formulae (PBM-34-1), (PBM-34-2), (PBM-34-3), (PBM-34-4), (PBM-34-5), (PBM-34-6), (PBM-34-7), (PBM-34-8), (PBM-34-9) or (PBM-34-12):

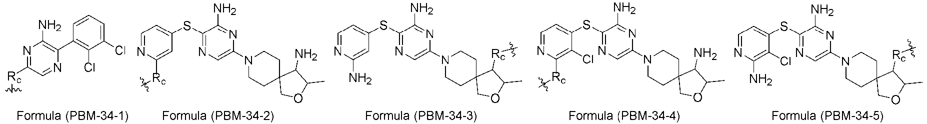

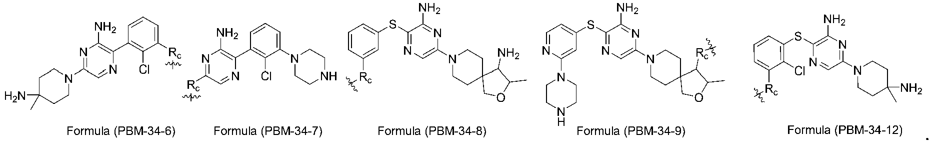

In some embodiments, PBM represents the structure of Formula (PBM-8-1C): 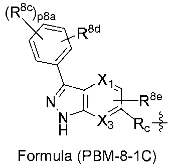
wherein X₁ in Formula (PBM-8-1C) represents N or CR^{8f}, where R^{8f} is hydrogen or amino; and X₃ represents CH or N;
(R^{8c})ₚ₈ₐ in Formula (PBM-8-1C) indicates that the benzene ring to which it is attached is substituted with p8a R^{8c} , with each R^{8c} being the same or different and each independently representing -O-aryl, -O-heteroaryl, 4- to 20-membered heterocyclyl, -C₁₋₃ alkylene -NHC(O)-heteroaryl, -C₁₋₃ alkylene-C(O)NH-heteroaryl, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterium, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogen (e.g., fluorine, chlorine, bromine, or iodine), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), wherein the 4- to 20-membered heterocyclyl, aryl, and heteroaryl are each independently optionally substituted with one or more (e.g., 1-5, such as 1, 2, 3, 4 or 5) R^{8h} , and each R^{8h} is the same or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p8a represents an integer of 1, 2, 3 or 4;
R^{8d} represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkoxy (e.g., halogenated C₁₋₄ alkoxy, such as F₃C-O-, FCH₂-O-, F₂CHO-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy); e.g., R^{8d} represents hydrogen or methyl; and
R^{8c} represents hydrogen, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or amino.

In some embodiments, R^{8c} in Formula (PBM-8-1C) represents -O-aryl, -O-heteroaryl, -C₁₋₃ alkylene-NHC(O)-heteroaryl, or -C₁₋₃ alkylene-C(O)NH-heteroaryl, wherein the aryl may be optionally substituted C₆₋₁₀ aryl, including but not limited to e.g., phenyl and naphthyl; and the heteroaryl may be optionally substituted 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, R^{8c} represents -O-phenyl or -O-naphthyl, wherein the phenyl and naphthyl are each optionally substituted with one or more (e.g., 1-5, such as 1, 2, 3, 4 or 5) R^{8h} , and each R^{8h} is the same or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In some embodiments, R^{8c} represents -O-heteroaryl, -C₁₋₃ alkylene-NHC(O)-heteroaryl, or -C₁₋₃ alkylene-C(O)NH-heteroaryl, wherein the heteroaryl is optionally substituted with one or more (e.g., 1-5, such as 1, 2, 3, 4 or 5) R^{8h}, and each R^{8h} is the same or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In some embodiments, examples of the heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, and imidazo[2,1-b]thiazolyl.

In some embodiments, R^{8c} in Formula (PBM-8-1C) represents 4- to 20-membered (e.g., 4- to 15-membered, 4- to 12-membered, 4- to 10-membered, 4- to 7-membered, or 4- to 6-membered) monocyclic, bicyclic, or polycyclic heterocyclyl, which is optionally substituted. In a sub-embodiment, R^{8c} represents 4-to 20-membered monocyclic, bicyclic, or polycyclic heterocyclyl, wherein the 4- to 20-membered monocyclic, bicyclic, or polycyclic heterocyclyl is optionally substituted with one or more (e.g., 1-5, such as 1, 2, 3, 4 or 5) R^{8h}, and each R^{8h} is the same or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of the the 4-to 20-membered monocyclic, bicyclic, or polycyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl).

In some embodiments, PBM represents a structure of Formulae (PBM-8-3), (PBM-8-4) or (PBM-8-*5):*

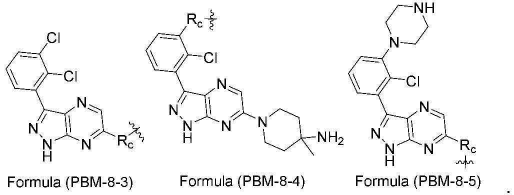

In some embodiments, PBM represents a small molecule ligand of PARP.

In some embodiments, PBM represents the structure of Formula (PBM-35): 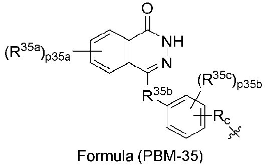
wherein (R^{35a})ₚ₃₅ₐ indicates that the phthalazinone ring to which it is attached is optionally substituted with p35a R^{35a} , with each R^{35a} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p35a represents an integer of 0, 1, 2, 3 or 4;
R^{35b} represents optionally substituted C₁₋₃ alkylene;
(R^{35c})_{p35b} indicates that the benzene ring to which it is attached is optionally substituted with p35b R^{35c}, with each R^{35c} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p35b represents an integer of 0, 1, 2, 3 or 4.

In some embodiments, PBM represents the structure of Formula (PBM-35-1):

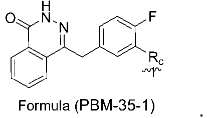

In some embodiments, PBM represents the structure of Formula (PBM-36): 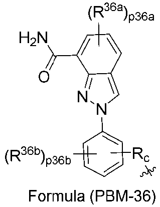
wherein (R^{36a})ₚ₃₆ₐ indicates that the 2H-indazole ring to which it is attached is optionally substituted with p36a R^{36a} , with each R^{36a} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p36a represents an integer of 0, 1, 2 or 3;
(R^{36b})_{p36b} indicates that the benzene ring to which it is attached is optionally substituted with p36b R^{36b}, with each R^{36b} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p36b represents an integer of 0, 1, 2, 3 or 4.

In some embodiments, PBM represents the structure of Formula (PBM-36-1):

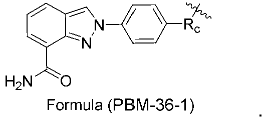

In some embodiments, PBM represents the structure of Formula (PBM-37): 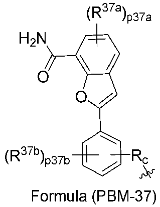
wherein (R^{37a})ₚ₃₇ₐ indicates that the benzofuran ring to which it is attached is optionally substituted with p37a R^{37a} , with each R^{37a} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p37a represents an integer of 0, 1, 2 or 3;
(R^{37b})_{p37b} indicates that the benzene ring to which it is attached is optionally substituted with p37b R^{37b}, with each R^{37b} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p37b represents an integer of 0, 1, 2 or 3.

In some embodiments, PBM represents the structure of Formula (PBM-37-1):

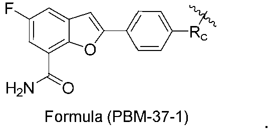

In some embodiments, PBM represents the structure of Formula (PBM-38): 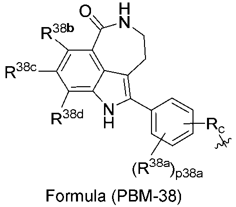
wherein (R^{38a})ₚ₃₈ₐ indicates that the benzene ring to which it is attached is optionally substituted with p38a R^{38a} , with each R^{38a} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p38a represents an integer of 0, 1, 2, 3 or 4; and
R^{33b}, R^{38c}, and R^{38d} are the same or different and each independently represent hydrogen, deuterium, hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of Formula (PBM-38-1):

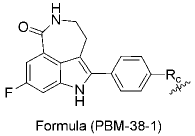

In some embodiments, PBM represents a small molecule ligand of STAT3.

In some embodiments, PBM represents the structure of Formula (PBM-39): 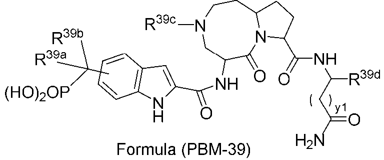
wherein R^{39a} and R^{39b} are the same or different and each independently represent hydrogen, halogen (e.g., fluorine, chlorine, bromine, or iodine), C₁₋₃ alkyl (e.g., methyl, ethyl, or propyl), or halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R^{39c} represents a bond, hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
y1 represents an integer of 1, 2 or 3;
R^{39d} represents the structure of the following formula: 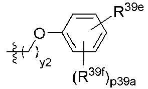
wherein R^{39e} represents a bond or hydrogen; and (R^{39f})ₚ₃₉ₐ indicates that the benzene ring to which it is attached is optionally substituted with p39a R^{39f} , with each R^{39f} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p39a represents an integer of 0, 1, 2, 3 or 4;
y2 represents an integer of 1, 2 or 3; or
R^{39d} represents the structure of the following formula: 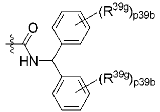
wherein (R^{39g})_{p39b} each independently indicates that the benzene ring to which it is attached is optionally substituted with p39b R^{39g} , with each R^{39g} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such asF₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and p39b represents an integer of 0, 1, 2, 3, 4 or 5; and
wherein R^{39c} and R^{39e} are not simultaneously a bond, and only one of R^{39c} and R^{39e} represents a bond, wherein when R^{39c} represents a bond, the ring nitrogen atom connected to R^{39c} is directly connected to R_{c}, and when R^{39e} represents a bond, the ring carbon atom connected to R^{39e} is directly connected to R_{c}.

In some embodiments, R^{39c} in Formula (PBM-39) represents a bond. In this case, the ring nitrogen atom connected to R^{39c} is directly bonded to R_{c}. R^{39d} represents the structure of the following formula: 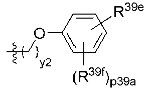 wherein R^{39c} represents hydrogen, and (R^{39f})ₚ₃₉ₐ indicates that the benzene ring to which it is attached is optionally substituted with p39a R^{39f} , with each R^{39f} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and p39a represents an integer of 0, 1, 2, 3 or 4; and y2 represents an integer of 1, 2 or 3.

In some embodiments, R^{39c} in Formula (PBM-39) represents a bond. In this case, the ring nitrogen atom connected to R^{39c} is directly bonded to R_{c}. R^{39d} represents the structure of the following formula: 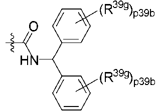 wherein (R^{39g})_{p39b} independently indicates that the benzene ring to which it is attached is optionally substituted with p39b R^{39g} , with each R^{39g} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p39b represents an integer of 0, 1, 2, 3, 4 or 5.

In some embodiments, when R^{39d} represents the structure of the following formula: 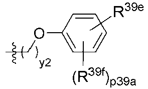 wherein R^{39c} represents a bond (i.e., the ring carbon atom connected to R^{39c} is directly connected to R_{c} ) and (R^{39f})ₚ₃₉ₐ is as defined above, R^{39c} represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of Formula (PBM-39-1A): 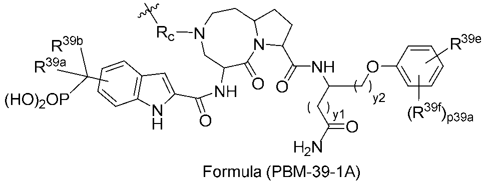
wherein R^{39a} and R^{39b} are the same or different and each independently represent hydrogen, halogen (e.g., fluorine, chlorine, bromine, or iodine), C₁₋₃ alkyl (e.g., methyl, ethyl, or propyl), or halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
y1 represents an integer of 1, 2 or 3;
y2 represents an integer of 1, 2 or 3;
R^{39c} represents hydrogen; and
(R^{39f})ₚ₃₉ₐ indicates that the benzene ring to which it is attached is optionally substituted with p39a R^{39f} , with each R^{39f} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p39a represents an integer of 0, 1, 2, 3 or 4.

In some embodiments, PBM represents the structure of Formula (PBM-39-1B): 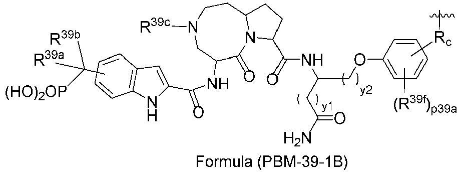
wherein R^{39a} and R^{39b} are the same or different and each independently represent hydrogen, halogen (e.g., fluorine, chlorine, bromine, or iodine), C₁₋₃ alkyl (e.g., methyl, ethyl, or propyl), or halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R^{39c} represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
y1 represents an integer of 1, 2 or 3;
y2 represents an integer of 1, 2 or 3; and
(R^{39f})ₚ₃₉ₐ indicates that the benzene ring to which it is attached is optionally substituted with p39a R^{39f} , with each R^{39f} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p39a represents an integer of 0, 1, 2, 3 or 4.

In some embodiments, PBM represents the structure of Formula (PBM-39-1C): 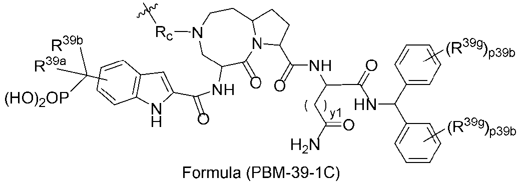
wherein R^{39a} and R^{39b} are the same or different and each independently represent hydrogen, halogen (e.g., fluorine, chlorine, bromine, or iodine), C₁₋₃ alkyl (e.g., methyl, ethyl, or propyl), or halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
y1 represents an integer of 1, 2 or 3; and
each (R^{39g})_{p39b} independently indicates that the benzene ring to which it is attached is optionally substituted with p39b R^{39g}, with each R^{39g} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p39b represents an integer of 0, 1, 2, 3, 4 or 5.

In some embodiments, PBM represents a structure of Formulae (PBM-39-1), (PBM-39-2) or (PBM-39-3):

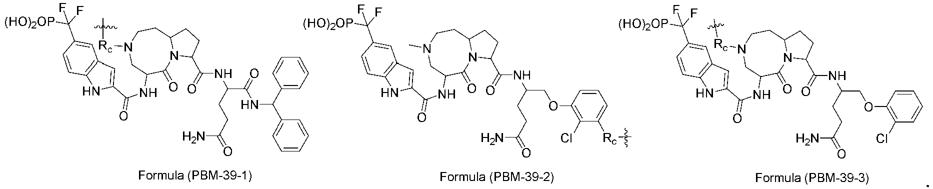

In some embodiments, PBM represents the structure of Formula (PBM-40): 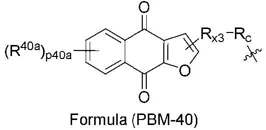
sherein (R^{40a})ₚ₄₀ₐ indicates that the benzene ring to which it is attached is optionally substituted with p40a R^{40a} , with each R^{40a} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₅ alkoxy, C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p40a represents an integer of 0, 1, 2, 3 or 4; and
Rₓ₃ in Formula (PBM-40) represents a bond or C(O).

In some embodiments, PBM represents a structure of Formulae (PBM-40-1) or (PBM-40-2):

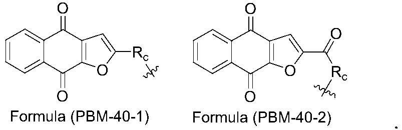

In some embodiments, PBM represents a small molecule ligand of FLT3.

In some embodiments, PBM represents the structure of Formula (PBM-41): 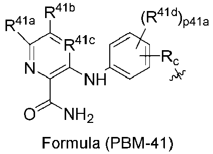
wherein R^{41a} represents hydrogen, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₁₅ cycloalkyl (e.g., optionally substituted cyclopropyl, optionally substituted cyclobutyl, optionally substituted cyclopentyl or optionally substituted cyclohexyl) or optionally substituted 4- to 15-membered nitrogen-containing heterocyclyl;
R^{41b}) represents hydrogen, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₁₅ cycloalkyl (e.g., optionally substituted cyclopropyl, optionally substituted cyclobutyl, optionally substituted cyclopentyl, or optionally substituted cyclohexyl), optionally substituted 4-to 15-membered nitrogen-containing heterocyclyl, or NR^{41e}R^{41f}, wherein R^{41e} and R^{41f} are the same or different and each independently represent hydrogen, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 15-membered heterocyclyl;
R^{41c} represents N or CH;
(R^{41d})ₚ₄₁ₐ indicates that the benzene ring to which it is attached is optionally substituted with p41a R^{41d} , with each R^{41d} being the same or different and each independently representing hydroxy, amino, cyano, halogen (e.g., fluorine, chlorine, bromine, or iodine), C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CHO-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p41a represents an integer of 0, 1, 2, 3 or 4.

In some embodiments, R^{41a} represents ethyl.

In some embodiments, R^{41a} represents hydrogen.

In some embodiments, R^{41b} represents NR^{41c}R^{41f}, where R^{41c} and R^{41f} are the same or different and each independently represent hydrogen, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 15-membered heterocyclyl. In some embodiments, the optionally substituted 4- to 15-membered heterocyclyl is e.g., optionally substituted 4- to 15-membered (e.g., 4- to 12-membered, 4- to 10-membered, 4- to 7-membered, 4- to 6-membered, 5- to 7-membered, or 5-to 6-membered) monocyclic, bicyclic, or polycyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 15-membered heterocyclyl is optionally substituted with a substituent selected from the group consisting of deuterium, hydroxy, amino, mercapto, nitro, halogen, cyano, oxo, C₁₋₆ alkyl, deuterated C₁₋₆ alkyl, halogenated C₁₋₆ alkyl, C₃₋₆ cycloalkyl, C₁₋₆ alkoxy, halogenated C₁₋₆ alkoxy or any combination thereof. In some embodiments, R^{41b} represents NR^{41c}R^{41f}, where R^{41e} represents hydrogen, and R^{41f} represents optionally substituted tetrahydropyranyl, wherein the tetrahydropyranyl is optionally substituted with a substituent selected from the group consisting of deuterium, hydroxy, amino, mercapto, nitro, halogen, cyano, oxo, C₁₋₆ alkyl, deuterated C₁₋₆ alkyl, halogenated C₁₋₆ alkyl, C₃₋₆ cycloalkyl, C₁₋₆ alkoxy, halogenated C₁₋₆ alkoxy or any combination thereof.

In some embodiments, R^{41b} represents optionally substituted C₃₋₁₅ cycloalkyl, e.g., optionally substituted cyclopropyl, optionally substituted cyclobutyl, optionally substituted cyclopentyl, or optionally substituted cyclohexyl, optionally substituted cycloheptyl, or optionally substituted cyclooctyl. In some embodiments, C₃₋₁₅ cycloalkyl is optionally substituted with one or more (e.g., 1-3, such as 1, 2 or 3) substituents selected from the group consisting of: deuterium, hydroxy, amino, mercapto, nitro, halogen, cyano, oxo, C₁₋₆ alkyl, deuterated C₁₋₆ alkyl, halogenated C₁₋₆ alkyl, C₃₋₆ cycloalkyl, C₁₋₆ alkoxy, halogenated C₁₋₆ alkoxy, or any combination thereof.

In some embodiments, R^{41b} represents optionally substituted 4- to 15-membered heterocyclyl. In some embodiments, the optionally substituted 4- to 15-membered heterocyclyl is e.g., optionally substituted e.g., optionally substituted 4- to 15-membered (e.g., 4- to 12-membered, 4- to 10-membered, 4- to 7-membered, 4- to 6-membered, 5- to 7-membered, or 5- to 6-membered) monocyclic, bicyclic, or polycyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 15-membered heterocyclyl is optionally substituted with a substituent selected from the group consisting of deuterium, hydroxy, amino, mercapto, nitro, halogen, cyano, oxo, C₁₋₆ alkyl, deuterated C₁₋₆ alkyl, halogenated C₁₋₆ alkyl, C₃₋₆ cycloalkyl, C₁₋₆ alkoxy, halogenated C₁₋₆ alkoxy, optionally substituted 4- to 11-membered heterocyclyl (e.g., 3-methyl-2-oxoimidazolidin-1-yl) or any combination thereof. The substituents of the optionally substituted 4- to 11-membered heterocyclyl are optionally selected from the group consisting of deuterium, hydroxy, amino, mercapto, nitro, halogen, cyano, oxo, C₁₋₆ alkyl, deuterated C₁₋₆ alkyl, halogenated C₁₋₆ alkyl or any combination thereof. In some embodiments, R^{41b} represents piperidinyl, and the piperidinyl is optionally substituted with 3-methyl-2-oxoimidazolidin-1-yl.

In some embodiments, p41a represents an integer of 0. In some embodiments, p41a represents 1, and R^{41d} represents methoxy.

In some embodiments, PBM represents the structure of Formula (PBM-41-1):

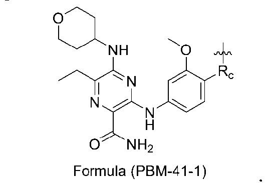

In some embodiments, PBM represents a small molecule ligand of FGFR.

In some embodiments, R^{26a} in the structure of Formula (PBM-26) as defined above represents a bond, meaning that the C(O) group is directly linked to the benzene ring.

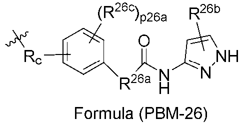

In some embodiments, R^{26b} in Formula (PBM-26) represents optionally substituted C₁₋₆ alkyl. The substituents of the optionally substituted C₁₋₆ alkyl include, but are not limited to, e.g., deuterium, hydroxy, amino, mercapto, nitro, halogen (e.g., fluorine, chlorine, bromine, iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), optionally substituted C₅₋₁₀ aryl (e.g., optionally substituted phenyl or optionally substituted naphthyl), or any combination thereof. Examples of the optionally substituted C₁₋₆ alkyl include, but are not limited to, e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl; deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.); halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); or -optionally substituted C₁₋₆ alkylene-optionally substituted C₅₋₁₀ aryl . In some embodiments, examples of -optionally substituted C₁₋₆ alkylene-optionally substituted C₆₋₁₀ aryl include, but are not limited to, e.g., - optionally substituted C₁₋₆ alkylene-optionally substituted C₆₋₁₀ aryl, or -optionally substituted C₁₋₆ alkylene-optionally substituted phenyl, wherein the aryl and phenyl are each optionally substituted with one or more (e.g., 1-4, or 2-3) substituents selected from the group consisting of e.g., deuterium, hydroxy, amino, mercapto, nitro, halogen (e.g., fluorine, chlorine, bromine, iodine), cyano, C₁₋₄ alkyl (e.g., C₁₋₃ alkyl, such as methyl, ethyl, propyl, isopropyl, butyl, sec-butyl, or tert-butyl), halogenated C₁₋₄ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₄ alkyl (e.g., perdeuterated C₁₋₄ alkyl, perdeuterated C₁₋₃ alkyl or perdeuterated C₁₋₂ alkyl, such as CD₃, CD₃CD₂-, CD₃CD₂CD₂- etc.), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or any combination thereof; and the C₁₋₆ alkylene is optionally substituted with one or more (e.g., 1-4, or 2-3) substituents selected from the group consisting of e.g., deuterium, hydroxy, amino, mercapto, nitro, halogen (e.g., fluorine, chlorine, bromine, iodine), cyano, C₁₋₄ alkyl (e.g., C₁₋₃ alkyl, such as methyl, ethyl, propyl, isopropyl, butyl, sec-butyl, or tert-butyl), halogenated C₁₋₄ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₄ alkyl (e.g., perdeuterated C₁₋₄ alkyl, perdeuterated C₁₋₃ alkyl or perdeuterated C₁₋₂ alkyl, such as CD₃, CD₃CD₂-, CD₃CD₂CD₂- etc.), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or any combination thereof.

In some embodiments, R^{26b} in Formula (PBM-26) represents ethylene-dimethoxyphenyl.

In some sub-embodiments, R^{26b} in Formula (PBM-26) represents

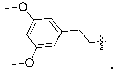

In some sub-embodiments, PBM represents the structure of Formula (PBM-26-2):

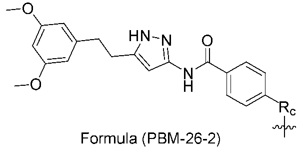

In some embodiments, PBM represents a small molecule ligand of BCL-2.

In some embodiments, PBM represents the structure of Formula (PBM-52): 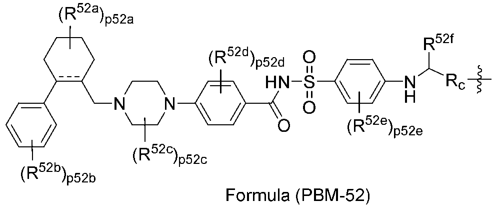
wherein the dashed line --- in Formula (PBM-52) indicates the optional presence of a double bond;
(R^{52a})ₚ₅₂ₐ indicates that the 6-membered cycloalkyl ring to which it is attached is optionally substituted with p52a R^{52a} , with each R^{52a} being the same or different and each independently representing halogen (e.g., fluorine, chlorine, bromine, iodine), hydroxy, mercapto, cyano, amino, nitro, oxo, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p52a represents an integer of 0, 1, 2, 3, 4, 5, 6, 7 or 8;
(R^{52b})_{p52b} indicates that the benzene ring to which it is attached is optionally substituted with p52b R^{52b} , with each R^{52b} being the same or different and each independently representing deuterium, halogen (e.g., fluorine, chlorine, bromine, iodine), hydroxy, mercapto, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p52b represents an integer of 0, 1, 2, 3, 4 or 5;
(R^{52c})_{p52c} indicates that the 6-membered cycloalkyl ring to which it is attached is optionally substituted with p52c R^{52c} , with each R^{52c} being the same or different and each independently representing halogen (e.g., fluorine, chlorine, bromine, iodine), cyano, oxo, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CHO-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p52c represents an integer of 0, 1, 2, 3, 4, 5, 6, 7 or 8;
(R^{52d})_{p52d} indicates that the benzene ring to which it is attached is optionally substituted with p52d R^{52d} , with each R^{52d} being the same or different and each independently representing halogen (e.g., fluorine, chlorine, bromine, iodine), hydroxy, mercapto, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CHO-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or 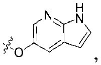 and p52d represents an integer of 0, 1 or 2;
(R^{52e})ₚ₅₂ₑ indicates that the benzene ring to which it is attached is optionally substituted with p52e R^{52e} , with each R^{52e} being the same or different and each independently representing deuterium, halogen (e.g., fluorine, chlorine, bromine, iodine), hydroxy, mercapto, cyano, amino, nitro, -SO₂CF₃, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p52e represents an integer of 0, 1, 2, 3 or 4; and
R^{52f} represents hydrogen, deuterium, halogen (e.g., fluorine, chlorine, bromine, iodine), hydroxy, mercapto, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or 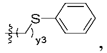 where y3 represents an integer of 1, 2 or 3.

In some sub-embodiments, the dashed line --- in Formula (PBM-52) indicates the presence of a double bond; that is, the six-membered ring group containing this dashed line represents

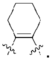

In some sub-embodiments, the dashed line --- in Formula (PBM-52) indicates the absence of a double bond; that is, the six-membered ring group containing this dashed line represents

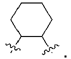

In some sub-embodiments, R^{52d} in Formula (PBM-52) represents 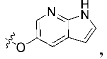 and p52d represents an integer of 1. In some sub-embodiments, p52d represents an integer of 0.

In some sub-embodiments, p52e in Formula (PBM-52) represents an integer of 1, and R^{52e} represents nitro (NO₂) or SO₂CF₃.

In some sub-embodiments, R^{52f} in Formula (PBM-52) represents hydrogen or 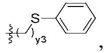 where y3 represents an integer of 1, 2 or 3.

In some sub-embodiments, PBM represents a structure of Formulae (PBM-52-1), (PBM-52-2), (PBM-52-3), (PBM-52-4) or (PBM-52-5):

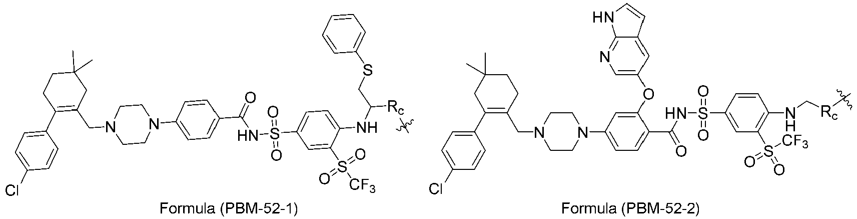

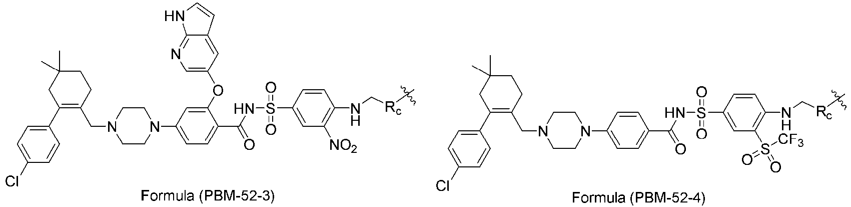

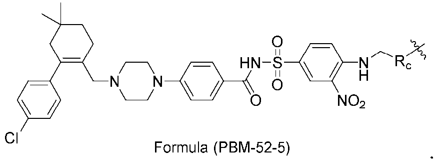

In some embodiments, PBM represents a small molecule ligand of ALK2.

In some embodiments, PBM represents the structure of Formula (PBM-43): 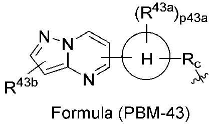
ring H in Formula (PBM-43) represents C₆₋₁₅ arylene or 5- to 15-membered monocyclic, bicyclic, or polycyclic heteroarylene, and (R^{43a})ₚ₄₃ₐ indicates that the ring H is optionally substituted with p43a R^{43a}, with each R^{43a} being the same or different and each independently representing deuterium, halogen (e.g., fluorine, chlorine, bromine, iodine), hydroxy, cyano, amino, nitro, C₁₋₆ alkyl(e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p43a represents an integer of 0, 1, 2, 3 or 4; and
R^{43b} represents optionally substituted 5- to 20-membered monocyclic, bicyclic, or polycyclic heteroaryl containing from 1 to 4 heteroatoms selected from N, O, and S, which is optionally substituted with a substituent selected from the group consisting of hydroxy, amino, cyano, halogen (e.g., fluorine, chlorine, bromine, iodine), nitro, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, ring H in Formula (PBM-43) represents phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, or imidazo[1,2-b]pyridazinylene. In some embodiments, ring H is optionally substituted with p43a R^{43a} , wherein p43a represents an integer of 0, 1, 2, 3 or 4, and each R^{43a} is the same or different and each independently represents deuterium, halogen (e.g., fluorine, chlorine, bromine, iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, ring H in Formula (PBM-43) represents phenylene, and the phenylene is optionally substituted with p43a R^{43a}, wherein p43a represents an integer of 0, 1, 2, 3 or 4, and each R^{43a} is the same or different and each independently represents deuterium, halogen (e.g., fluorine, chlorine, bromine, iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, R^{43b} in Formula (PBM-43) represents optionally substituted 5- to 20-membered monocyclic, bicyclic, or polycyclic heteroaryl group containing 1 to 4 heteroatoms selected from N, O, and S. In some embodiments, R^{43b} represents furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, and imidazo[1,2-b]pyridazinyl. In some embodiments, R^{43b} is optionally substituted with one or more (e.g., 1-6, such as 1, 2, 3, 4, 5 or 6) substituents selected from the group consisting of hydroxy, amino, cyano, halogen (e.g., fluorine, chlorine, bromine, iodine), nitro, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{43b} in Formula (PBM-43) represents quinolinyl, and the quinolinyl is optionally substituted with one or more (e.g., 1-6, such as 1, 2, 3, 4, 5 or 6) substituents selected from the group consisting of hydroxy, amino, cyano, halogen (e.g., fluorine, chlorine, bromine, iodine), nitro, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents a structure of Formulae (PBM-43-1) or (PBM-43-2):

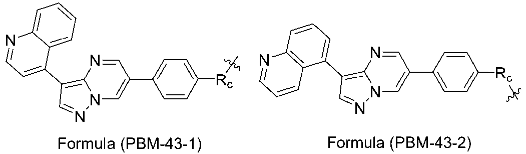

In some embodiments, PBM represents a small molecule ligand of SOS1.

In some embodiments, PBM represents the structure of Formula (PBM-1): 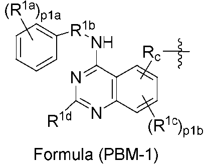
wherein (R^{1a})ₚ₁ₐ indicates that the benzene ring to which it is attached is substituted with p1a R^{1a} , with each R^{1a} being the same or different and each independently representing deuterium, halogen (e.g., fluorine, chlorine, bromine, or iodine), hydroxy, NH₂, NO₂, cyano, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), or halogenated C₁₋₆ alkoxy (e.g., halogenated C₁₋₄ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
pla represents an integer of 1, 2, 3, 4 or 5;
R^{1b}represents C₁₋₂ alkylene optionally substituted with a substituent selected from the group consisting of C₁₋₃ alkyl, deuterated C₁₋₃ alkyl, halogenated C₁₋₃ alkyl and halogen;
(R^{1c})_{p1b} indicates that the quinazoline ring in Formula (PBM-1) is substituted with plb R^{1c} , with each R^{1c} being the same or different and each independently representing deuterium, halogen (e.g., fluorine, chlorine, bromine, or iodine), NO₂, cyano, halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₁₀ alkyl (e.g., C₁₋₆ alkyl or C₁₋₄ alkyl, such as methyl, ethyl, propyl, isopropyl, butyl, sec-butyl, tert-butyl, pentyl, or hexyl), deuterated C₁₋₁₀ alkyl (e.g., perdeuterated C₁₋₁₀ alkyl or perdeuterated C₁₋₆ alkyl or perdeuterated C₁₋₄ alkyl, such as CD₃, CD₃CD₂-, CD₃CD₂CD₂- etc.), C₁₋₁₀ alkoxy (e.g., C₁₋₆ alkoxy or C₁₋₄ alkoxy, such as methoxy, ethoxy, propoxy, isopropoxy, n-butoxy, isobutoxy, or tert-butoxy), halogenated C_{1-1O} alkoxy (e.g., halogenated C₁₋₆ alkoxy or halogenated C₁₋₄ alkoxy, such as F₃C-O-, FCH₂-O-, F₂CH-O-, ClCH₂-O-, Cl₂CHO-, CF₃CF₂-O-, CF₃CHF-O-, CHF₂CF₂-O-, CHF₂CHF-O-, CF₃CH₂-O- or CH₂ClCH₂-O-), -O-optionally substituted heterocyclyl, or -NHC(O)R^{1c}, where R^{1c} represents C₁₋₁₀ alkyl (e.g., C₁₋₈ alkyl, C₁₋₆ alkyl or C₁₋₄ alkyl, such as methyl, ethyl, propyl, isopropyl, butyl, sec-butyl, tert-butyl, pentyl, hexyl, heptyl, or octyl), deuterated C_{1-1O} alkyl (e.g., perdeuterated C₁₋₈ alkyl, perdeuterated C₁₋₆ alkyl, or perdeuterated C₁₋₄ alkyl, such as CD₃, CD₃CD₂-, CD₃CD₂CD₂- etc.) or C₂₋₁₀ alkenyl (e.g., C₂₋₆ alkenyl or C₂₋₄ alkenyl, such as vinyl, propenyl, butenyl or pentenyl); e.g., R^{1c} represents methyl, methoxy, 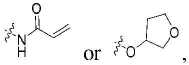 such as 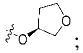
plb represents an integer of 1, 2, or 3; and
R^{1d} represents hydrogen, deuterium or C₁₋₃ alkyl.

In some embodiments, R^{1b} represents methylene or ethylene which are optionally substituted with a substituent selected from the group consisting of C₁₋₃ alkyl, deuterated C₁₋₃ alkyl, halogenated C₁₋₃ alkyl, and halogen. In some embodiments, R^{1b} represents methylene. In some embodiments, R^{1b} represents trifluoromethyl-substituted methylene.

In some embodiments, R^{1d} represents hydrogen. In some embodiments, R^{1d} represents deuterium. In some embodiments, R^{1d} represents methyl.

In some embodiments, R^{1c} represents C₁₋₁₀ alkoxy (e.g., C₁₋₆ alkoxy or C₁₋₄ alkoxy, such as methoxy, ethoxy, propoxy, isopropoxy, n-butoxy, isobutoxy, or tert-butoxy), or -O-optionally substituted heterocyclyl. The heterocyclyl is a 4- to 20-membered (e.g., 4- to 15-membered, 4- to 12-membered, 4- to 10-membered, 4- to 7-membered, or 4- to 6-membered) monocyclic, bicyclic, or polycyclic heterocyclyl group containing one or more (e.g., 1-4, or 1, 2, 3, or 4) heteroatoms selected from nitrogen, oxygen, and sulfur, including but not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl). In some embodiments, the heterocyclyl is optionally substituted with a substituent(s) selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof. In some embodiments, plb represents an integer of 1, R^{1c} represents methoxy. In some embodiments, plb represents an integer of 1, and R^{1c} represents

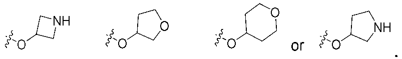

In some embodiments, PBM represents a structure of Formula (PBM-1-6), (PBM-1-7), (PBM-1-8), (PBM-1-9) or (PBM-1-10):

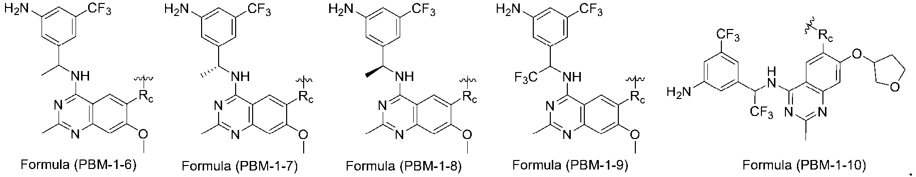

In some embodiments, PBM represents a small molecule ligand of RSK.

In some embodiments, PBM represents the structure of Formula (PBM-53): 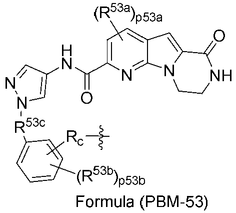
wherein (R^{53a})ₚ₅₃ₐ in Formula (PBM-53) represents p53a R^{53a} groups, and each R^{53a} is the same or different and each independently represents halogen (e.g., fluorine, chlorine, bromine, or iodine), hydroxy, mercapto, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p53a represents an integer of 0, 1, 2, 3, 4 or 5;
(R^{53b})_{p53b} indicates that the benzene ring to which it is attached is optionally substituted with p53b R^{53b} , with each R^{53b} being the same or different and each independently representing deuterium, halogen (e.g., fluorine, chlorine, bromine, or iodine), hydroxy, mercapto, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p53b represents an integer of 0, 1, 2, 3 or 4; and
R^{53c} represents C₁₋₆ alkylene or halogenated C₁₋₆ alkylene.

In some embodiments, R^{53c} represents C₁₋₆ alkylene, e.g., methylene or ethylene.

In some embodiments, R^{53c} represents halogenated C₁₋₆ alkylene, e.g., difluoromethylene.

In some embodiments, PBM represents the structure of Formula (PBM-53-1):

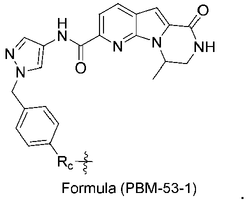

In some embodiments, PBM represents a small molecule ligand of SMARCA2/4.

In some embodiments, PBM represents the structure of Formula (PBM-34-11): 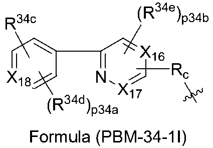
wherein X₁₆, X₁₇, and X₁₈ in Formula (PBM-34-11) each independently represent CH or N;
R^{34c} represents halogen (e.g., fluorine, chlorine, bromine, or iodine), amino, hydroxy, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted cycloalkyl (e.g., optionally substituted C₃₋₁₅ cycloalkyl), or optionally substituted 4- to 15-membered nitrogen-containing heterocyclyl;
(R^{34d})ₚ₃₄ₐ indicates that the 6-membered ring to which it is attached is optionally substituted with p34a R^{34d} , with each R^{34d} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p34a represents an integer of 0, 1, 2, 3 or 4;
(R^{34c})_{p34b} indicates that the 6-membered nitrogen-containing heteroaryl ring to which it is attached is optionally substituted with p34b R^{34c} , with each R^{34c} being the same or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p34b represents an integer of 0, 1 or 2.

In some embodiments, in Formula (PBM-34-11), X₁₆ represents CH, and X₁₇ represents CH. In some embodiments, in Formula (PBM-34-11), X₁₆ represents CH, and X₁₇ represents N. In some embodiments, in Formula (PBM-34-11), X₁₆ represents N, and X₁₇ represents N. In some embodiments, in Formula (PBM-34-1I), X₁₆ represents N, and X₁₇ represents CH.

In some embodiments, in Formula (PBM-34-11), X₁₆ represents CH, and X₁₇ represents CH, and X₁₈ represents CH. In some embodiments, in Formula (PBM-34-11), X₁₆ represents CH, and X₁₇ represents N, and X₁₈ represents CH. In some embodiments, in Formula (PBM-34-11), X₁₆ represents CH, and X₁₇ represents N, and X₁₈ represents N. In some embodiments, in Formula (PBM-34-11), X₁₆ represents CH, and X₁₇ represents CH, and X₁₈ represents N. In some embodiments, in Formula (PBM-34-11), X₁₆ represents N, and X₁₇ represents N, and X₁₈ represents N. In some embodiments, in Formula (PBM-34-11), X₁₆ represents N, and X₁₇ represents CH, and X₁₈ represents N. In some embodiments, in Formula (PBM-34-11), X₁₆ represents N, and X₁₇ represents CH, and X₁₈ represents CH. In some embodiments, in Formula (PBM-34-11), X₁₆ represents N, and X₁₇ represents N, and X₁₈ represents CH.

In some embodiments, PBM represents a structure of Formulae (PBM-34-10) or (PBM-34-11):

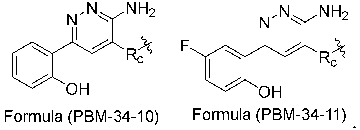

In some embodiments, PBM represents a small molecule ligand of KRAS.

In some embodiments, PBM represents the structure of Formula (PBM-48): 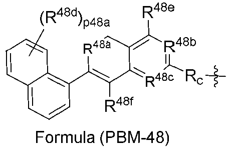
wherein R^{43a}, R^{43b}, and R^{43c} in Formula (PBM-48) each independently represent N or CH;
(R^{48d})ₚ₄₈ₐ indicates that the naphthyl to which it is attached is optionally substituted with p48a R^{48d} , with each R^{48d} independently representing halogen (e.g., fluorine, chlorine, bromine, or iodine), hydroxy, amino, mercapto, nitro, cyano, C₂₋₆ alkynyl, C₂₋₆ alkenyl, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p48a represents an integer of 0, 1, 2, 3, 4, 5, 6 or 7;
R^{48c} represents optionally substituted C₃₋₁₅ cycloalkyl, or optionally substituted 4- to 15-membered heterocyclyl containing from 1 to 4 heteroatoms selected from the group consisting of oxygen, nitrogen, and sulfur; and
R^{48f} represents hydrogen, halogen (e.g., fluorine, chlorine, bromine, or iodine), hydroxy, cyano, amino, mercapto, nitro, C₂₋₆ alkynyl, C₂₋₆ alkenyl, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{48a}, R^{43b}, and R^{48c} in Formula (PBM-48) each independently represent N or CH. In some embodiments, one of R^{43a}, R^{43b}, and R^{48c} in Formula (PBM-48) represents N, and the remaining ones each independently represent N or CH. In some embodiments, two of R^{43a}, R^{43b}, and R^{48c} in Formula (PBM-48) represent N, and the remaining one represents N or CH. In some embodiments, all of R^{43a}, R^{43b}, and R^{43c} in Formula (PBM-48) represent N. In some embodiments, all of R^{43a}, R^{43b}, and R^{48c} in Formula (PBM-48) represent CH.

In some embodiments, (R^{48d})ₚ₄₈ₐ indicates that the naphthyl in Formula (PBM-48) to which it is attached is optionally substituted with p48a R^{48d} , wherein each R^{48d} independently represents halogen (e.g., fluorine, chlorine, bromine, or iodine), hydroxy, amino, mercapto, nitro, cyano, C₂₋₆ alkynyl (e.g., ethynyl, propynyl, butynyl, pentynyl, or hexynyl), C₂₋₆ alkenyl (e.g., vinyl, propenyl, butenyl, pentenyl or hexenyl), C₁₋₆ alkoxy (e.g., C₁₋₄ alkoxy, such as methoxy, CD₃-O-, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p48a represents an integer of 0, 1, 2, 3, 4, 5, 6 or 7. In some embodiments, p48a represents an integer of 2, 3 or 4, and each R^{48d} independently represents halogen (e.g., fluorine, chlorine, bromine, or iodine), hydroxy or C₂₋₆ alkynyl (e.g., ethynyl, propynyl, butynyl, pentynyl, or hexyny).

In some embodiments, R^{48c} represents optionally substituted C₃₋₁₅ cycloalkyl, or optionally substituted 4- to 15-membered heterocyclyl containing from 1 to 4 heteroatoms selected from the group consisting of oxygen, nitrogen, and sulfur. In some embodiments, examples of the optionally substituted C₃₋₁₅ cycloalkyl include, but are not limited to, e.g., optionally substituted C₃₋₁₂ cycloalkyl, or optionally substituted C₃₋₉ cycloalkyl, such as cyclopropyl, cyclobutyl, cyclopentyl, cyclohexyl, cycloheptyl, cyclooctyl, noradamantanyl, adamantanyl, bornyl, and norbornyl, wherein the C₃₋₁₅ cycloalkyl is optionally substituted with one or more (e.g., 1-4, or 2-3) substituents selected from the group consisting of e.g., deuterium, oxo, hydroxy, amino, mercapto, nitro, halogen (e.g., fluorine, chlorine, bromine, iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl (e.g., cyclopropyl, cyclobutyl, cyclopentyl, or cyclohexyl) or any combination thereof. In some embodiments, examples of the optionally substituted 4- to 15-membered heterocyclyl containing from 1 to 4 heteroatoms selected from the group consisting of oxygen, nitrogen, and sulfur include, but are not limited to, e.g., optionally substituted 4- to 12-membered heterocyclyl containing from 1 to 4 heteroatoms selected from the group consisting of oxygen, nitrogen, and sulfur, or optionally substituted 4- to 11-membered heterocyclyl containing from 1 to 4 heteroatoms selected from the group consisting of oxygen, nitrogen, and sulfur, e.g., azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl), diazacyclooctyl, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, and azaspirocycloalkyl (e.g., 3-azaspiro[5.5]undecan-3-yl). The 4- to 15-membered heterocyclyl is optionally substituted with one or more (e.g., 1-4, or 2-3) substituents selected from the group consisting of: deuterium, oxo, hydroxy, amino, mercapto, nitro, halogen (e.g., fluorine, chlorine, bromine, iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl (e.g., cyclopropyl, cyclobutyl, cyclopentyl, or cyclohexyl), or any combination thereof.

In some embodiments, R^{48f} represents hydrogen, halogen (e.g., fluorine, chlorine, bromine, iodine), hydroxy, C₂₋₆ alkynyl (e.g., ethynyl, propynyl, butynyl, pentynyl, or hexynyl), C₂₋₆ alkenyl (e.g., vinyl, propenyl, butenyl, pentenyl or hexenyl), C₁₋₆ alkoxy (e.g., C₁₋₄ alkoxy, such as methoxy, CD₃-O-, ethoxy, propoxy, isopropoxy, n-butoxy, isobutoxy, or tert-butoxy), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of Formula (PBM-48-1):

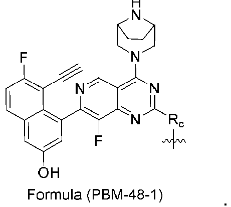

In some embodiments, R_{c} in each of the general formulae of PBM represents a bond, -O-, -NHC(O)-*, -C(O)NH-*, or -NH-.

In some embodiments, R_{c} in each of the general formulae of PBM represents the structure of the following formula:
-N(R_{d})-, -N(R_{d})-R_{f}-N(R_{c})-*, -R_{f}-C(O)NH-*, -R_{f}-NHC(O)-*, -R_{f}-C(O)O-*, -R_{f}-OC(O)-*, -R_{f}-, -R_{f}-N(R_{d})-*, -O-R_{f}-N(R_{d})-*, 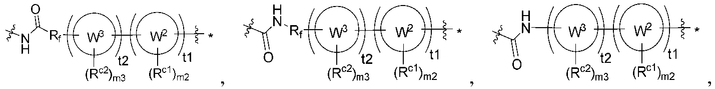 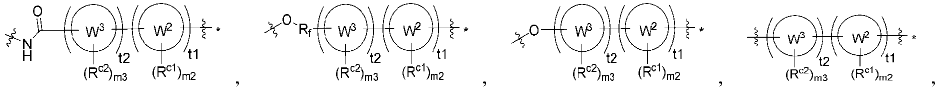 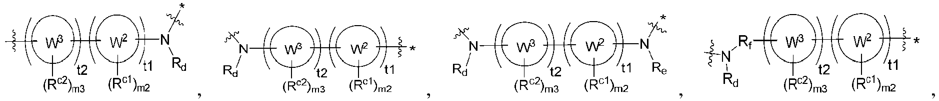 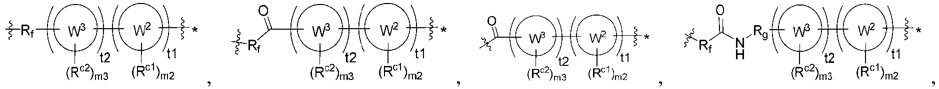 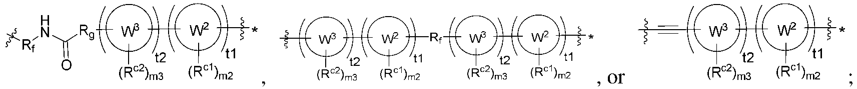
wherein each ring W² is the same or different and each independently represents 4- to 20-membered nitrogen-containing heterocyclylene; t1 represents an integer of 1 or 2; and (R^{c1})ₘ₂ indicates that each ring W² is independently optionally substituted with m2 R^{c1} groups, with each R^{c1} being independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c4}-R^{c3}-, where R^{c3} is optionally substituted C₁₋₆ alkylene or optionally substituted C₂₋₆ alkenylene, and R^{c4} represents halogen, R^{c5}R^{c6}N-, hydroxy, carboxyl, ethynyl, or vinyl, wherein R^{c5} and R^{c6} are the same or different and each independently represent hydrogen or C₁₋₃ alkyl, and m2 represents an integer of 0-20;
each ring W³ is the same or different and each independently represents 4- to 20-membered nitrogen-containing heterocyclylene, C₃₋₂₀ cycloalkylene, C₆₋₂₀ arylene, or 5- to 20-membered heteroarylene, t2 represents an integer of 0 or 1, and (R^{c2})ₘ₃ indicates that each ring W³ is independently optionally substituted with m3 R^{c2} groups, with each R^{c2} being independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c8}-R^{c7}-, where R^{c7} is optionally substituted C₁₋₆ alkylene or optionally substituted C₂₋₆ alkenylene, and R^{c8} represents halogen, R^{c9}R^{c10}N-, hydroxy, carboxyl, ethynyl, or vinyl, wherein R^{c9} and R^{c10} are the same or different and each independently represent hydrogen or C₁₋₃ alkyl, and m3 represents an integer of 0-20; and
R_{d} and R_{c} each independently represent hydrogen or C₁₋₆ alkyl, R_{f} and R_{g} each independently represent optionally substituted C₁₋₆ alkylene or optionally substituted C₂₋₆ alkenylene;
the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy; and
symbol * indicates the point of attachment to LIN.

In some embodiments, R_{d} and R_{c} each independently represent hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl).

In some embodiments, R_{f} and R_{g} each independently represent C₁₋₆ alkylene and C₂₋₆ alkenylene. C₁₋₆ alkylene and C₂₋₆ alkenylene are optionally substituted with one or more (e.g., 1-5, such as 1, 2, 3, 4 or 5) substituents selected from the group consisting of deuterium, halogen, hydroxy, mercapto, cyano, amino, nitro, C₁₋₆ alkyl, halogenated C₁₋₃ alkyl, deuterated C₁₋₆ alkyl, C₁₋₆ alkoxy, or halogenated C₁₋₆ alkoxy, or any combination thereof.

In some embodiments, t1 represents an integer of 1.

In some embodiments, t1 represents an integer of 2.

In some embodiments, each ring W² is the same or different and each independently represents a 4-to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered , or 5- to 9-membered) nitrogen-containing heterocyclylene, e.g., a 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclylene containing one nitrogen atom and optionally containing a heteroatom(s) selected from nitrogen, oxygen, and sulfur. Examples of the nitrogen-containing heterocyclylene include, but are not limited to, e.g., piperidinylene, piperazinylene, morpholinylene, azetidinylene, pyrrolidinylene, imidazolidinylene, pyrazolidylene, oxazolidinylene, thiazolidinylene, thiomorpholinylene, azacycloheptylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decylene, 2-oxa-8-azaspiro[4.5]decylene, or 3-methyl-2-oxa-8-azaspiro[4.5]decylene. Each ring W² is independently optionally substituted with m2 R^{c1} groups, wherein each R^{c1} is independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), or R^{c4}-R^{c3}-, where R^{c3} is optionally substituted C₁₋₆ alkylene (e.g., optionally substituted C₁₋₄ alkylene, such as optionally substituted methylene, ethylene, or propylene) or optionally substituted C₂₋₆ alkenylene (e.g., optionally substituted C₂₋₄ alkenylene, such as optionally substituted vinylene, propenylene, butenylene), and R^{c4} represents halogen (e.g., fluorine, chlorine, bromine, or iodine), R^{c5}R^{c6}N-, hydroxy, carboxyl, ethynyl, or vinyl, wherein R^{c5} and R^{c6} are the same or different and each independently represent hydrogen or C₁₋₃ alkyl (e.g., methyl, ethyl, or propyl); and the C₁₋₆ alkylene and C₂₋₆ alkenylene are optionally substituted with one or more (e.g., 1-5, such as 1, 2, 3, 4 or 5) substituents selected from the group consisting of deuterium, halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₃ alkyl(e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₃ alkoxy (e.g., methoxy, ethoxy, propoxy, or isopropoxy), halogenated C₁₋₃ alkoxy (e.g., halogenated C₁₋₂ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof. m2 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, each ring W² is the same or different and each independently represents the following optionally substituted groups: 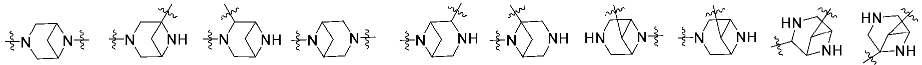 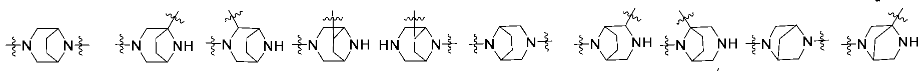 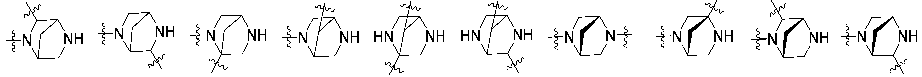 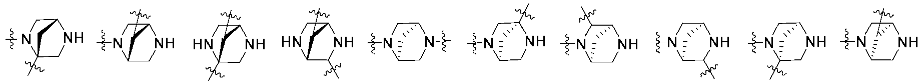 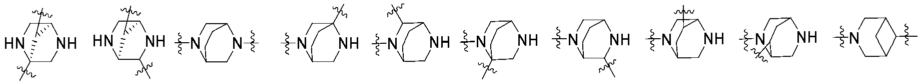 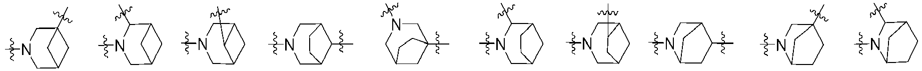 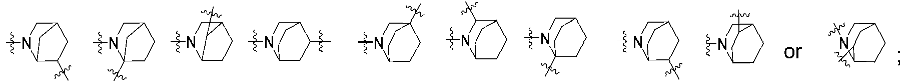 each ring W² is independently optionally substituted with m2 R^{c1} groups, with each R^{c1} being independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl, or butynyl), C₂₋₆ alkenyl (e.g., C₂₋₄ alkenyl, such as vinyl, vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), or R^{c4}-R^{c3}-, where R^{c3} is optionally substituted C₁₋₆ alkylene (e.g., optionally substituted C₁₋₄ alkylene, such as optionally substituted methylene, ethylene, or propylene) or optionally substituted C₂₋₆ alkenylene (e.g., optionally substituted C₂₋₄ alkenylene, such as optionally substituted vinylene, propenylene, and butenylene), and R^{c4} represents halogen (e.g., fluorine, chlorine, bromine, or iodine), R^{c5}R^{c6}N-, hydroxy, carboxyl, ethynyl, or vinyl, wherein R^{c5} and R^{c6} are the same or different and each independently represent hydrogen or C₁₋₃ alkyl (e.g., methyl, ethyl, or propyl); and the C₁₋₆ alkylene and C₂₋₆ alkenylene are optionally substituted with one or more substituents selected from the group consisting of deuterium, halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₃ alkoxy (e.g., methoxy, ethoxy, propoxy, or isopropoxy), halogenated C₁₋₃ alkoxy (e.g., halogenated C₁₋₂ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof. m2 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, each ring W³ is the same or different and each independently represents a 4-to 15-membered (e.g., 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) nitrogen-containing heterocyclylene, C₃₋₁₅ cycloalkylene, a 6- to 15-membered (e.g., 6- to 10-membered, 6- to 8-membered, or 6- to 7-membered) arylene, or a 5- to 15-membered (e.g., 5- to 10-membered, 5- to 9-membered, 5- to 8-membered, 5- to 7-membered, or 5- to 6-membered) heteroarylene. In some embodiments, the 4- to 15-membered nitrogen-containing heterocyclylene is a 4- to 15-membered (e.g., 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclylene containing one nitrogen atom and optionally containing a heteroatom(s) selected from nitrogen, oxygen, and sulfur. Examples of the 4- to 15-membered nitrogen-containing heterocyclylene include, but are not limited to, e.g., piperidinylene, piperazinylene, morpholinylene, azetidinylene, pyrrolidinylene, imidazolidinylene, pyrazolidylene, oxazolidinylene, thiazolidinylene, thiomorpholinylene, azacycloheptylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decylene, 2-oxa-8-azaspiro[4.5]decylene, or 3-methyl-2-oxa-8-azaspiro[4.5]decylene. In some embodiments, examples of the C₃₋₁₅ cycloalkylene include, but are not limited to, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene, adamantanylene, noradamantanylene, and norbornylene. In some embodiments, examples of the 6- to 15-membered arylene include, but are not limited to, e.g., phenylene or naphthylene. In some embodiments, the 5- to 15-membered heteroarylene is a 5- to 15-membered (e.g., 5- to 12-membered , 5- to 9-membered , 5- to 8-membered, 5- to 7-membered , or 5- to 6-membered ) heteroarylene containing one nitrogen atom and optionally containing a heteroatom(s) selected from nitrogen, oxygen, and sulfur. In some embodiments, examples of the 5- to 15-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. Each ring W³ is independently optionally m3 R^{c2} groups, with each R^{c2} being independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), or R^{c8}-R^{c7}-, wherein R^{c7} is optionally substituted C₁₋₆ alkylene (e.g., optionally substituted C₁₋₄ alkylene, such as optionally substituted methylene, ethylene, or propylene) or optionally substituted C₂₋₆ alkenylene (e.g., optionally substituted C₂₋₄ alkenylene, such as optionally substituted vinylene, propenylene, and butenylene), and R^{c8} represents halogen (e.g., fluorine, chlorine, bromine, or iodine), R^{c9}R^{c10}N-, hydroxy, carboxyl, ethynyl, or vinyl, wherein R^{c9} and R^{c10} are the same or different and each independently represent hydrogen or C₁₋₃ alkyl (e.g., methyl, ethyl, or propyl); and the C₁₋₆ alkylene and C₂₋₆ alkenylene are each independently optionally substituted with one or more substituents selected from the group consisting of (e.g., 1-5, such as 1, 2, 3, 4 or 5) deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy. m3 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, each ring W³ is the same or different and each independently represents: 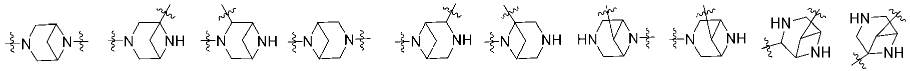 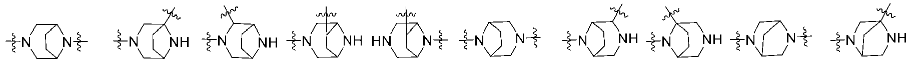 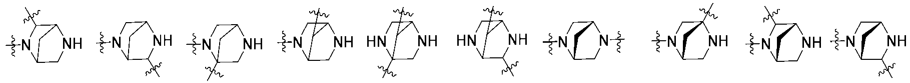 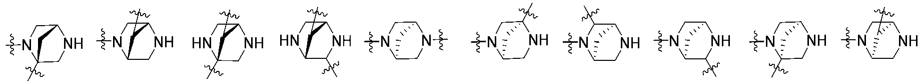 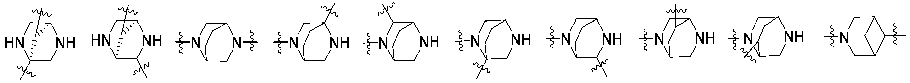 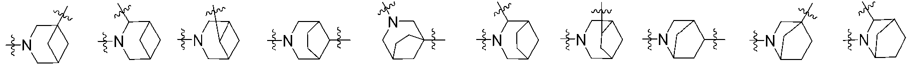 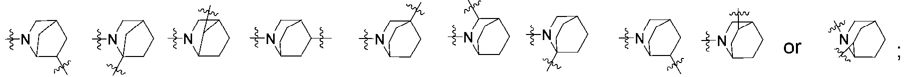 each ring W³ is independently optionally substituted with m3 R^{c2} groups, with each R^{c2} being independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), or R^{c8}-R^{c7}-, where R^{c7} is optionally substituted C₁₋₆ alkylene (e.g., optionally substituted C₁₋₄ alkylene, such as optionally substituted methylene, ethylene, or propylene) or optionally substituted C₂₋₆ alkenylene (e.g., optionally substituted C₂₋₄ alkenylene, such as optionally substituted vinylene, propenylene, and butenylene), and R^{c8} represents halogen (e.g., fluorine, chlorine, bromine, or iodine), R^{c9}R^{c10}N-, hydroxy, carboxyl, ethynyl, or vinyl, wherein R^{c9} and R^{c10} are the same or different and each independently represent hydrogen or C₁₋₃ alkyl (e.g., methyl, ethyl, or propyl); and the C₁₋₆ alkylene and C₂₋₆ alkenylene are each independently optionally substituted with one or more substituents selected from the group consisting of (e.g., 1-5, such as 1, 2, 3, 4 or 5)deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy. m3 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, t2 represents an integer of 0.

In some embodiments, t2 represents an integer of 1.

In some embodiments, the moiety 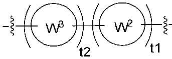 of in each of the general formulae of R_{c} represents the following groups: 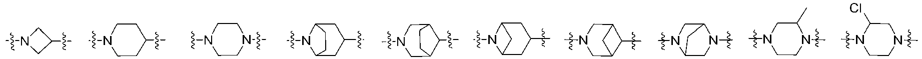 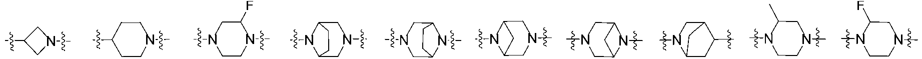 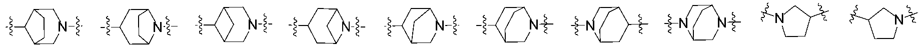 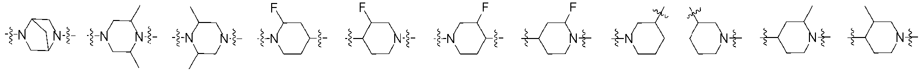 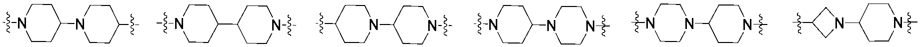 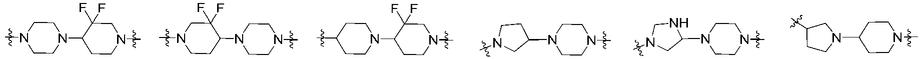 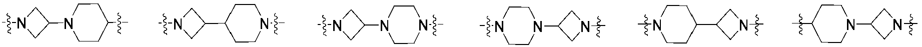 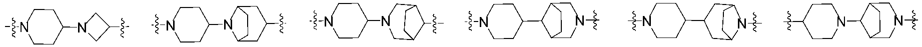 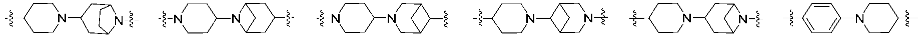 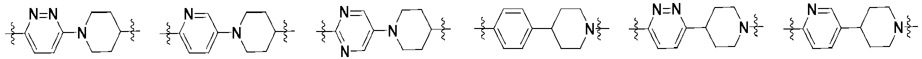 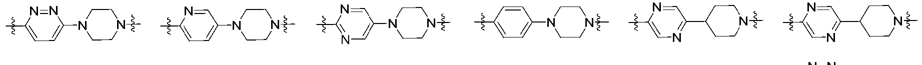 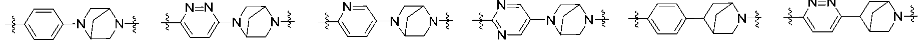 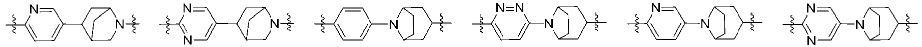 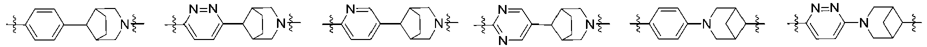 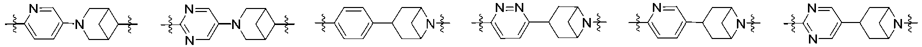 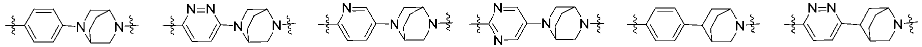 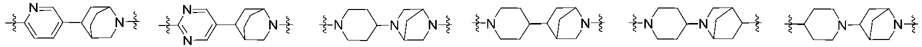 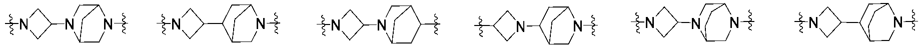 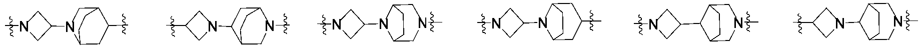 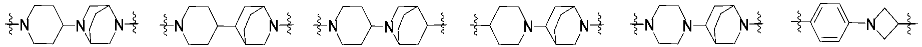 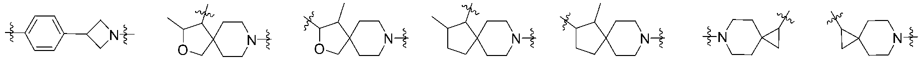 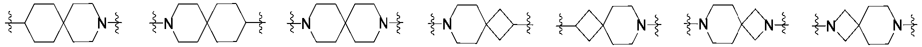 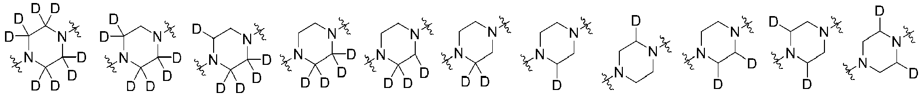 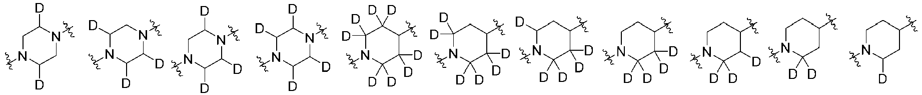 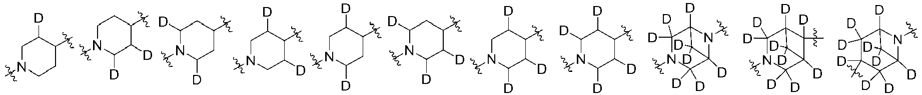 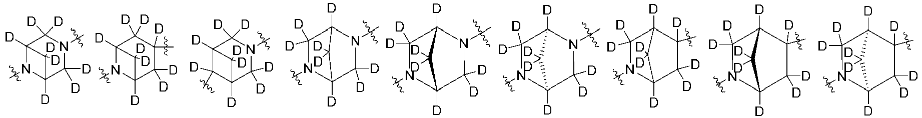 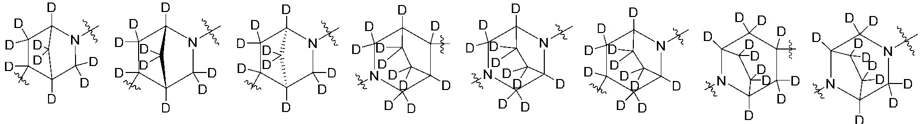 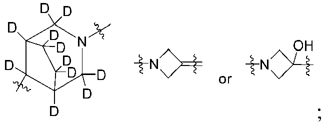 wherein the groups are independently optionally substituted with one or more (e.g., 1-5, such as 1, 2, 3, 4 or 5) substituents selected from the group consisting of deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy (e.g., perdeuterated C₁₋₄ alkoxy, such as CD₃-O-, CD₃CD₂-O-, or CD₃CD₂CD₂-O-), halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), R^{c12}-R^{c11}, or any combination thereof; wherein R^{c11} is optionally substituted C₁₋₆ alkylene (e.g., optionally substituted C₁₋₄ alkylene, such as optionally substituted methylene, ethylene, or propylene) or optionally substituted C₂₋₆ alkenylene (e.g., optionally substituted C₂₋₄ alkenylene, such as optionally substituted vinylene, propenylene, and butenylene), and R^{c12} represents halogen (e.g., fluorine, chlorine, bromine, or iodine), R^{c13}R^{c14}N-, hydroxy, carboxyl, ethynyl, or vinyl, wherein R^{c13} and R^{c14} are the same or different and each independently represent hydrogen or C₁₋₃ alkyl (e.g., methyl, ethyl, or propyl); and the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy.

In some embodiments, the R_{c} represents the following groups:
a bond, -O-, -NHC(O)-*, -C(O)NH-*, -C(O)O-*, -OC(O)-*, -CH₂C(O)O-*, -CH₂OC(O)-*, -NH-, - N(CH₃)-, -N(CH₃)-(CH₂)₂-N(CH₃)-*, -N(CH₃)-(CH₂)₃-N(CH₃)-*, -NH-(CH₂)₂-N(CH₃)-*, -N(CH₃)-(CH₂)₃-NH-*, -CH₂-NHC(O)-*, -CH₂-C(O)NH-*, -CH₂-NH-*, -O-(CH₂)₂-N(CH₃)-*, -O-(CH₂)₂-NH-*, or -CH₂-N(CH₃)-*, or 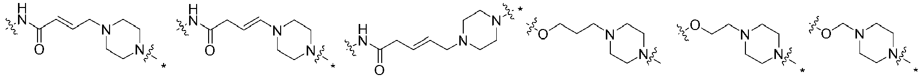 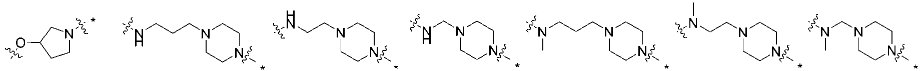 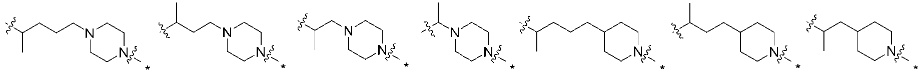 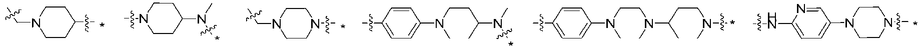 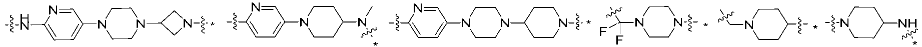 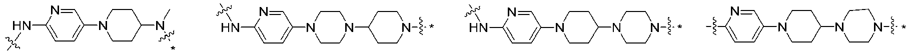 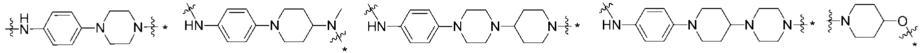 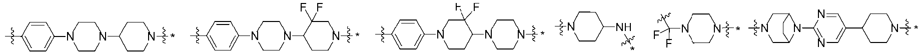 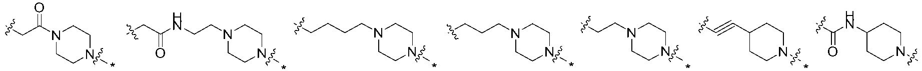 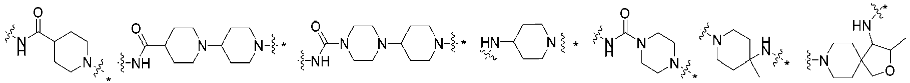 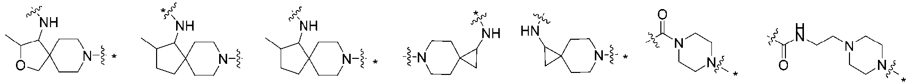 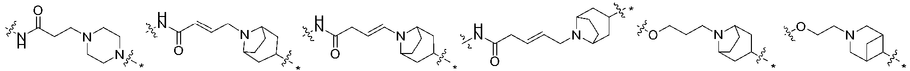 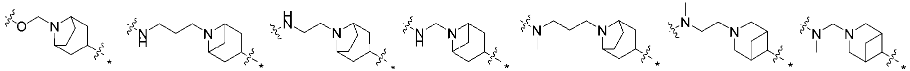 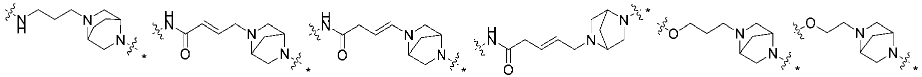 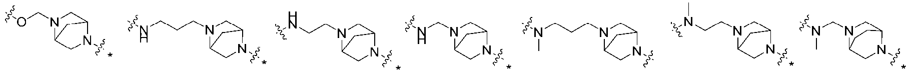 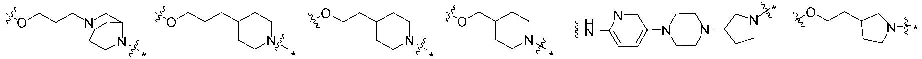 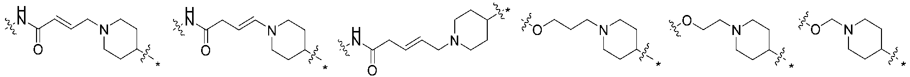 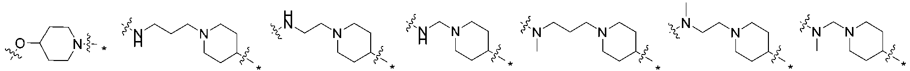 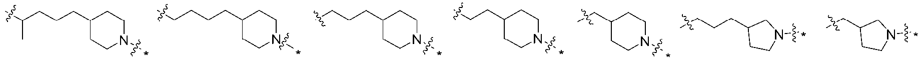 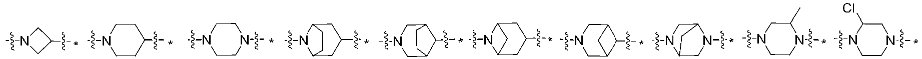 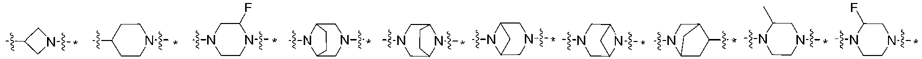 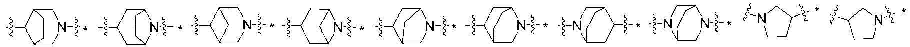 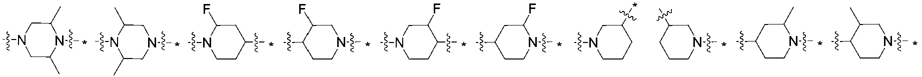 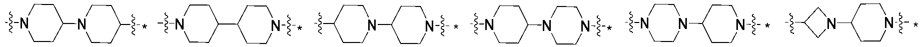 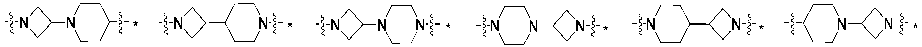 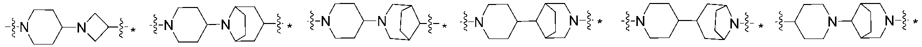 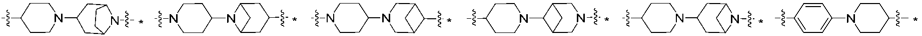 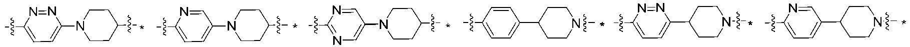 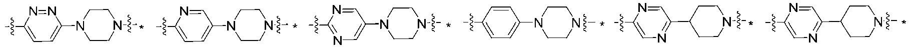 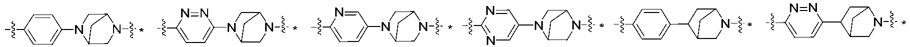 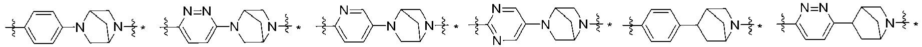 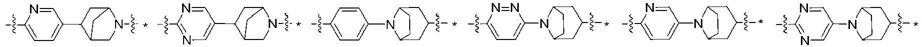 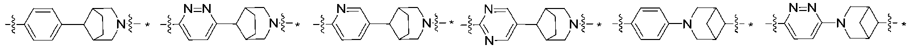 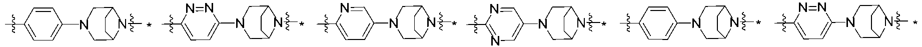 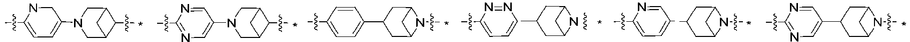 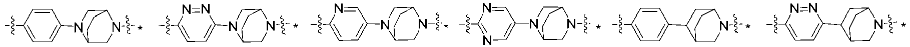 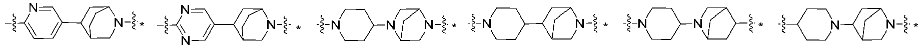 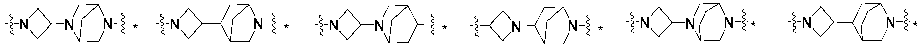 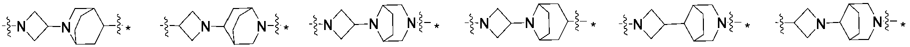 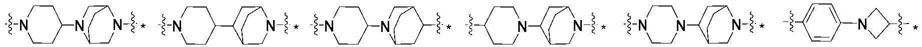 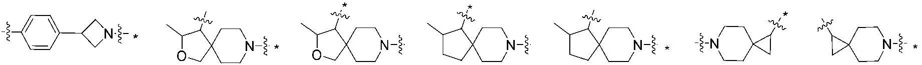 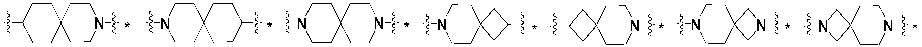 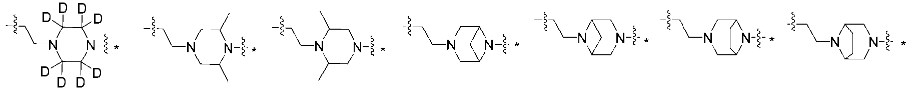 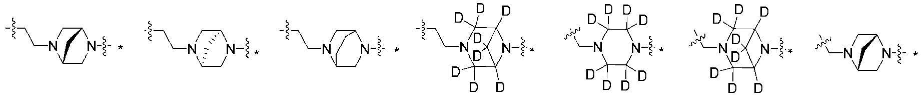 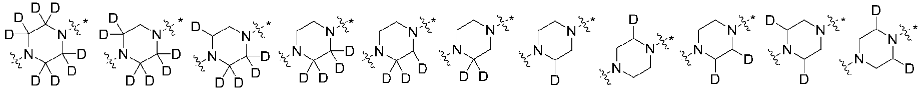 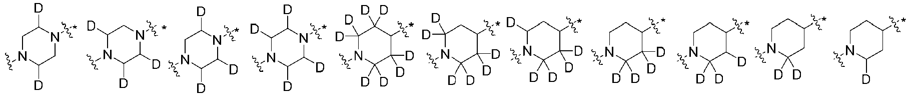 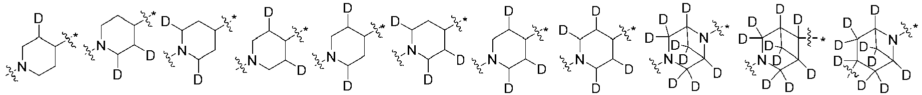 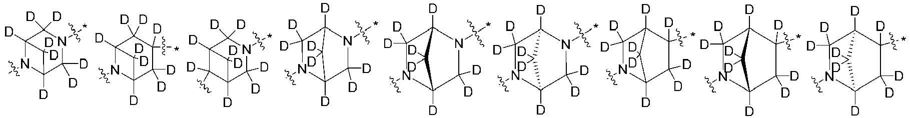 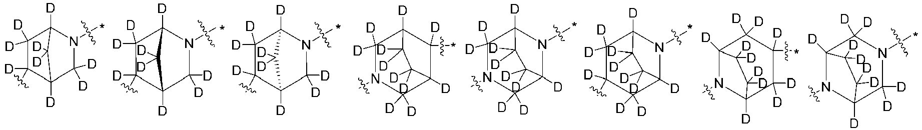 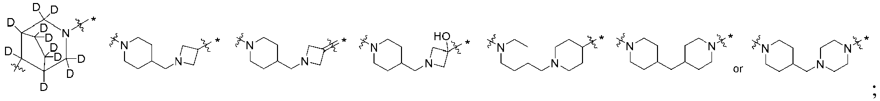
wherein the groups are independently optionally substituted with one or more (e.g., 1-5, such as 1, 2, 3, 4 or 5) substituents selected from the group consisting of deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), halogen (e.g., fluorine, chlorine, bromine, or iodine), amino, hydroxy, mercapto, cyano, C₂₋₆ alkynyl (e.g., C₂₋₄ alkynyl, such as ethynyl, propynyl, or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl or optionally perdeuterated cyclohexyl), R^{c8}-R^{c7}-, or any combination thereof; wherein R^{c7} is optionally substituted C₁₋₆ alkylene (e.g., optionally substituted C₁₋₄ alkylene, such as optionally substituted methylene, ethylene, or propylene) or optionally substituted C₂₋₆ alkenylene (e.g., optionally substituted C₂₋₄ alkenylene, such as optionally substituted vinylene, propenylene, and butenylene), and R^{c8} represents halogen (e.g., fluorine, chlorine, bromine, or iodine), R^{c9}R^{c10}N-, hydroxy, carboxyl, ethynyl, or vinyl, wherein R^{c9} and R^{c10} are the same or different and each independently represent hydrogen or C₁₋₃ alkyl (e.g., methyl, ethyl, or propyl); and the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy; and
symbol * indicates the point of attachment to LIN.

In some embodiments, the ULM represents a ligand moiety capable of binding to an E3 ubiquitin ligase, and represents the structure of the following Formula (ULM): 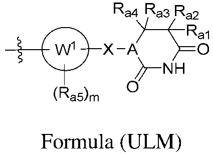
wherein A represents N or CH;
Rₐ₁, Rₐ₂, Rₐ₃, and Rₐ₄ are the same or different and independently represent hydrogen, deuterium, halogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or deuterated C₁₋₆ alkoxy;
ring W¹ represents arylene (e.g., C₆₋₂₀ arylene) or heteroarylene (e.g., 5- to 20-membered monocyclic, bicyclic, or polycyclic heteroarylene), and the ring W¹ is optionally substituted with m Rₐ₅, with each Rₐ₅ being the same or different and independently representing halogen, hydroxy, mercapto, nitro, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, C₂₋₆ alkenyl or C₂₋₆ alkynyl;
m represents an integer of 0, 1, 2, 3 or 4;
X represents a bond or -R_{b1}-N(Rₐ₆)-R_{b2}-, where Rₐ₆ represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), and R_{b1} and R_{b2} each independently represent optionally substituted C₀₋₂ alkylene.

In some embodiments, A represents N. In some embodiments, A represents CH.

In some embodiments, Rₐ₁, Rₐ₂, Rₐ₃ and Rₐ₄ represents hydrogen.

In some embodiments, ring W¹ represents: C₆₋₂₀ arylene or 5- to 20-membered monocyclic, bicyclic, or polycyclic heteroarylene, wherein the C₆₋₂₀ arylene and the 5- to 20-membered monocyclic, bicyclic, or polycyclic heteroarylene are each independently optionally substituted with m identical or different Rₐ₅ groups; preferably, wherein the ring W¹ represents the following groups which are optionally substituted with m identical or different Rₐ₅ groups: phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene; wherein each Rₐ₅ independently represents halogen, hydroxy, mercapto, nitro, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, C₂₋₆ alkenyl or C₂₋₆ alkynyl; and m represents an integer of 0, 1, 2, 3 or 4.

In some embodiments, X represents a bond.

In some embodiments, X represents -R_{b1}-N(Rₐ₆)-R_{b2}-, wherein Rₐ₆ represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), and R_{b1} and R_{b2} each independently represent optionally substituted C₀₋₂ alkylene. In some embodiments, substituents of the optionally substituted C₀₋₂ alkylene are optionally selected from the group consisting of deuterium, halogen, hydroxy, mercapto, nitro, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, or any combination thereof.

In some embodiments, X represents -NH-, -N(CH₃)-, -CH₂NH-, -NHCH₂- or -CH₂NHCH₂-.

In some embodiments, the ULM represents a structure of Formulae (ULM-1) or (ULM-2): 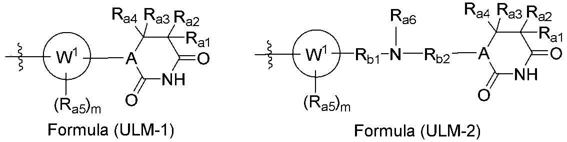 wherein the A, Rₐ₁, Rₐ₂, Rₐ₃, Rₐ₄, (Rₐ₅)ₘ, Rₐ₆, R_{b1}, R_{b2} and ring W¹ are as defined above.

In some embodiments, the ULM represents a structure of the following formulae:

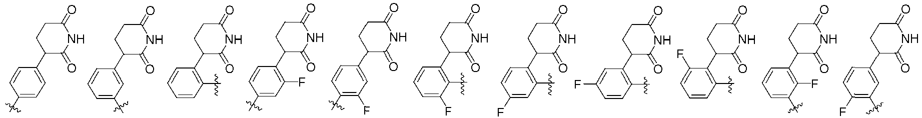

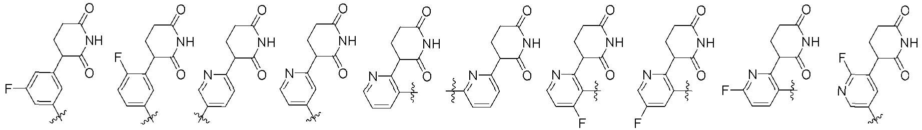

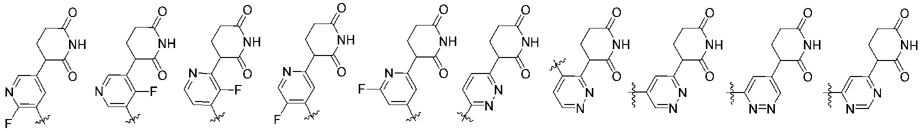

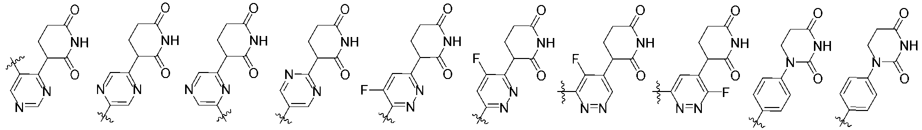

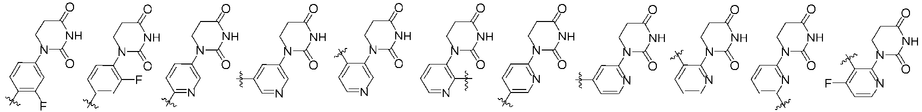

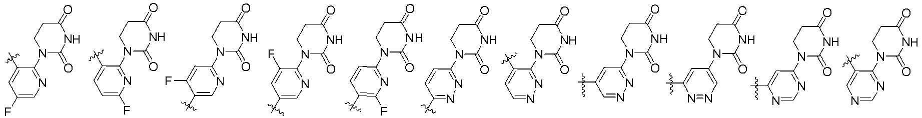

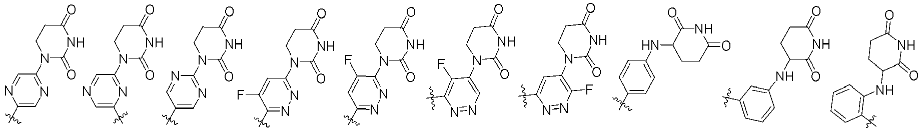

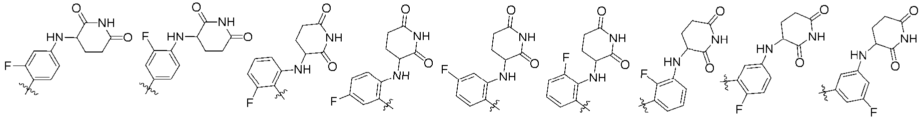

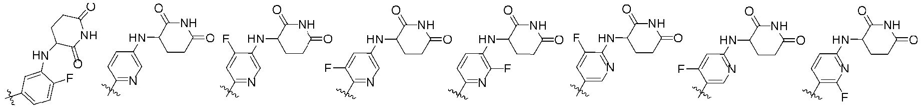

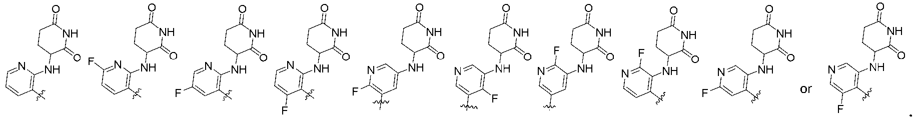

In some embodiments, LIN of the compounds of Formula (I) represents methylene, which is optionally substituted with 1-2 substituents independently selected from the group consisting of deuterium, hydroxy, amino, mercapto, nitro, halogen, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl or any combination thereof.

In some embodiments, LIN of the compounds of Formula (I) represents optionally substituted linear or branched C₂₋₁₅ alkylene, wherein one or more groups Rₐ and/or one or more groups R_{b} and/or any combination of one or more groups Rₐ and R_{b} are optionally inserted between one or more pairs of adjacent carbon atoms in the backbone carbon chain of the linear or branched C₂₋₁₅ alkylene group; wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and in case that two or more groups Rₐ are inserted into the backbone carbon chain of the linear or branched C₂₋₁₅ alkylene group, the two or more groups Rₐ are not directly connected to each other; and wherein each R_{b} is independently selected from the group consisting of optionally substituted cycloalkylene (e.g., optionally substituted C₃₋₂₀ cycloalkylene), optionally substituted arylene (e.g., optionally substituted C₃₋₂₀ arylene), optionally substituted heterocyclylene (e.g., optionally substituted 4- to 20-membered heterocyclylene), optionally substituted heteroarylene (e.g., optionally substituted 5- to 20-membered heteroarylene), alkynylene (e.g., C₂₋₆ alkynylene), alkenylene (e.g., C₂₋₆ alkenylene), and any combination thereof, wherein the cycloalkylene, arylene, heterocyclylene, and heteroarylene are independently optionally substituted with a substituent selected from the group consisting of deuterium, hydroxy, amino, mercapto, nitro, halogen, cyano, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl and any combination thereof; and wherein one or more hydrogens of said linear or branched C₂₋₁₅ alkylene are optionally further replaced by a substituent selected from deuterium, hydroxy, amino, mercapto, nitro, halogen, cyano, oxo, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl, and any combination thereof.

In some embodiments, LIN of the compounds of Formula (I) represents:

-C₁₋₁₅ alkylene-;

-(C(Rₐ₈)(Rₐ₉))ₙ₁-(Rₐ(C(Rₐ₁₀)(Rₐ₁₁))ₙ₂)ₘ₁-;

-(C(Rₐ₈)(Rₐ₉))ₙ₁-(Rₐ(C(Rₐ₁₀)(Rₐ₁₁))ₙ₂)ₘ₁-(Rₐ(C(Rₐ₁₂)(Rₐ₁₃))ₙ₃)ₘ₂-;

-(C(Rₐ₈)(Rₐ₉))ₙ₁-(Rₐ(C(Rₐ₁₀)(Rₐ₁₁))ₙ₂)ₘ₁-(Rₐ(C(Rₐ₁₂)(Rₐ₁₃))ₙ₃)ₘ₂-(Rₐ(C(Rₐ₁₄)(Rₐ₁₅))ₙ₄)ₘ₃-;

-(C(Rₐ₈)(Rₐ₉))ₙ₁-(R_{b}(C(Rₐ₁₀)(Rₐ₁₁))ₙ₂)ₘ₁-;

-(C(Rₐ₈)(Rₐ₉))ₙ₁-(R_{b}(C(Rₐ₁₀)(Rₐ₁₁))ₙ₂)ₘ₁-(R_{b}(C(Rₐ₁₂)(Rₐ₁₃))ₙ₃)ₘ₂-;

-(C(Rₐ₈)(Rₐ₉))ₙ₁-(R_{b}(C(Rₐ₁₀)(Rₐ₁₁))ₙ₂)ₘ₁-(R_{b}(C(Rₐ₁₂)(Rₐ₁₃))ₙ₃)ₘ₂-(R_{b}(C(Rₐ₁₄)(Rₐ₁₅))ₙ₄)ₘ₃-;

-(C(Rₐ₈)(Rₐ₉))ₙ₁-(Rₐ-R_{b}-(C(Rₐ₁₀)(Rₐ₁₁))ₙ₂)ₘ₁-;

-(C(Rₐ₈)(Rₐ₉))ₙ₁-(Rₐ(C(Rₐ₁₀)(Rₐ₁₁))ₙ₂)ₘ₁-(R_{b}(C(Rₐ₁₂)(Rₐ₁₃))ₙ₃)ₘ₂-;

-(C(Rₐ₈)(Rₐ₉))ₙ₁-(R_{b}-Rₐ-(C(Rₐ₁₀)(Rₐ₁₁))ₙ₂)ₘ₁-;

or

-(C(Rₐ₈)(Rₐ₉))ₙ₁-(R_{b}(C(Rₐ₁₀)(Rₐ₁₁))ₙ₂)ₘ₁-(Rₐ(C(Rₐ₁₂)(Rₐ₁₃))ₙ₃)ₘ₂-;

wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇) and N(Rₐ₇)C(O)N(Rₐ₇), wherein each Rₐ₇ independently represents H or C₁₋₃ alkyl; and each R_{b} is independently selected from the group consisting of optionally substituted cycloalkylene (e.g., optionally substituted C₃₋₂₀ cycloalkylene), optionally substituted arylene (e.g., optionally substituted C₃₋₂₀ arylene), optionally substituted heterocyclylene (e.g., optionally substituted 4- to 20-membered heterocyclylene), optionally substituted heteroarylene (e.g., 5- to 20-membered heteroarylene), alkynylene (e.g., C₂₋₆ alkynylene), alkenylene (e.g., C₂₋₆ alkenylene), and any combination thereof, wherein the cycloalkylene, arylene, heterocyclylene, and heteroarylene are are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof;
Rₐ₈, Rₐ₉, Rₐ₁₀, Rₐ₁₁, Rₐ₁₂, Rₐ₁₃, Rₐ₁₄ and Rₐ₁₅ each independently represent H, deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof; and
n1, n2, n3, n4, m1, m2, and m3 each independently represent an integer of 1, 2, 3, 4, 5, 6, 7, 8, 9 or 10; and
any one or more hydrogens of said C₁₋₁₅ alkylene are optionally further replaced by a substituent selected from: deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

In some embodiments, LIN of the compounds of Formula (I) represents -C₁₋₁₅ alkylene-, wherein any one or more hydrogens of said C₁₋₁₅ alkylene are optionally further replaced by a substituent selected from: deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

In some embodiments, LIN of the compounds of Formula (I) represents the following groups:
-CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, -(CH₂)₁₃-, -(CH₂)₁₄- or -(CH₂)₁₅-;
wherein any one or more hydrogens of the groups are optionally further replaced by a substituent selected from: deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

In some embodiments, LIN of the compounds of Formula (I) represents the following groups:

-(C(Rₐ₈)(Rₐ₉))ₙ₁-(O(C(Rₐ₁₀)(Rₐ₁₁))ₙ₂)ₘ₁-;

-(C(Rₐ₈)(Rₐ₉))ₙ₁-(O(C(Rₐ₁₀)(Rₐ₁₁))ₙ₂)ₘ₁-(O(C(Rₐ₁₂)(Rₐ₁₃))ₙ₃)ₘ₂-;

-(C(Rₐ₈)(Rₐ₉))ₙ₁-(O(C(Rₐ₁₀)(Rₐ₁₁))ₙ₂)ₘ₁-(O(C(Rₐ₁₂)(Rₐ₁₃))ₙ₃)ₘ₂- (O(C(Rₐ₁₄)(Rₐ₁₅))ₙ₄)ₘ₃-;

-(C(Rₐ₈)(Rₐ₉))ₙ₁-(N(Rₐ₇)(C(Rₐ₁₀)(Rₐ₁₁))ₙ₂)ₘ₁-;

-(C(Rₐ₈)(Rₐ₉))ₙ₁-(N(Rₐ₇)(C(Rₐ₁₀)(Rₐ₁₁))ₙ₂)ₘ₁-(N(Rₐ₇)(C(Rₐ₁₂)(Rₐ₁₃))ₙ₃)ₘ₂-;

-(C(Rₐ₈)(Rₐ₉))ₙ₁-(N(Rₐ₇)(C(Rₐ₁₀)(Rₐ₁₁))ₙ₂)ₘ₁-(N(Rₐ₇)(C(Rₐ₁₂)(Rₐ₁₃))ₙ₃)ₘ₂-(N(Rₐ₇)(C(Rₐ₁₄)(Rₐ₁₅))ₙ₄)ₘ₃-;

-(C(Rₐ₈)(Rₐ₉))ₙ₁-(C(O)N(Rₐ₇)-(C(Rₐ₁₀)(Rₐ₁₁))ₙ₂)ₘ₁-;

-(C(Rₐ₈)(Rₐ₉))ₙ₁-(C(O)N(Rₐ₇)-(C(Rₐ₁₀)(Rₐ₁₁))ₙ₂)ₘ₁-(C(O)N(Rₐ₇)-(C(Rₐ₁₂)(Rₐ₁₃))ₙ₃)ₘ₂-;

-(C(Rₐ₈)(Rₐ₉))ₙ₁-(C(O)N(Rₐ₇)-(C(Rₐ₁₀)(Rₐ₁₁))ₙ₂)ₘ₁-(C(O)N(Rₐ₇)-(C(Rₐ₁₂)(Rₐ₁₃))ₙ₃)ₘ₂-(C(O)N(Rₐ₇)-(C(Rₐ₁₄)(Rₐ₁₅))ₙ₄)ₘ₃-;

-(C(Rₐ₈)(Rₐ₉))ₙ₁-(C(O)N(Rₐ₇)-(C(Rₐ₁₀)(Rₐ₁₁))ₙ₂)ₘ₁-(O-(C(Rₐ₁₂)(Rₐ₁₃))ₙ₃)ₘ₂-;

-(C(Rₐ₈)(Rₐ₉))ₙ₁-C(O)N(Rₐ₇)-(C(Rₐ₁₀)(Rₐ₁₁))ₙ₂-(O(C(Rₐ₁₂)(Rₐ₁₃))ₙ₃)ₘ₁-;

-(C(Rₐ₈)(Rₐ₉))ₙ₁-(N(Rₐ₇)C(O)-(C(Rₐ₁₀)(Rₐ₁₁))ₙ₂)ₘ₁-;

-(C(Rₐ₈)(Rₐ₉))ₙ₁-(N(Rₐ₇)C(O)-(C(Rₐ₁₀)(Rₐ₁₁))ₙ₂)ₘ₁-(O-(C(Rₐ₁₂)(Rₐ₁₃)))ₙ₃)ₘ₂-;

-(C(Rₐ₈)(Rₐ₉))ₙ₁-(N(Rₐ₇)C(O)-(C(Rₐ₁₀)(Rₐ₁₁))ₙ₂)ₘ₁-(O-(C(Rₐ₁₂)(Rₐ₁₃))ₙ₃)ₘ₂-(O-(C(Rₐ₁₄)(Rₐ₁₅))ₙ₄)ₘ₃-;

-(C(Rₐ₈)(Rₐ₉))ₙ₁-N(Rₐ₇)C(O)-(C(Rₐ₁₀)(Rₐ₁₁))ₙ₂-(O(C(Rₐ₁₂)(Rₐ₁₃))ₙ₃)ₘ₁-;

-(C(Rₐ₈)(Rₐ₉))ₙ₁-N(Rₐ₇)C(O)N(Rₐ₇)-(C(Rₐ₁₀)(Rₐ₁₁))ₙ₂-;

-(C(Rₐ₈)(Rₐ₉))ₙ₁-C(O)-(C(Rₐ₁₀)(Rₐ₁₁))ₙ₂-;

-(C(Rₐ₈)(Rₐ₉))ₙ₁-(arylene-(C(Rₐ₁₀)(Rₐ₁₁))ₙ₂)ₘ₁-;

-(C(Rₐ₈)(Rₐ₉))ₙ₁-(arylene-(C(Rₐ₁₀)(Rₐ₁₁))ₙ₂)ₘ₁-arylene-(C(Rₐ₁₂)(Rₐ₁₃))ₙ₃-;

-(C(Rₐ₈)(Rₐ₉))ₙ₁-(heterocyclylene-(C(Rₐ₁₀)(Rₐ₁₁))ₙ₂)ₘ₁-;

-(C(Rₐ₈)(Rₐ₉))ₙ₁-(heterocyclylene-(C(Rₐ₁₀)(Rₐ₁₁))ₙ₂)ₘ₁-(heterocyclylene-(C(Rₐ₁₂)(Rₐ₁₃))ₙ₃)ₘ₂-;

-(C(Rₐ₈)(Rₐ₉))ₙ₁-(heteroarylene-(C(Rₐ₁₀)(Rₐ₁₁))ₙ₂)ₘ₁-;

-(C(Rₐ₈)(Rₐ₉))ₙ₁(heteroarylene-(C(Rₐ₁₀)(Rₐ₁₁))n₂)ₘ₁-(heteroarylene-(C(Rₐ₁₂)(Rₐ₁₃))ₙ₃)ₘ₂-;

-(C(Rₐ₈)(Rₐ₉))ₙ₁-(cycloalkylene-(C(Rₐ₁₀)(Rₐ₁₁))ₙ₂)ₘ₁-;

or

-(C(Rₐ₈)(Rₐ₉))ₙ₁-(cycloalkylene-(C(Rₐ₁₀)(Rₐ₁₁))ₙ₂)ₘ₁-(cycloalkylene-(C(Rₐ₁₂)(Rₐ₁₃))ₙ₃)ₘ₂-;

wherein each Rₐ₇ independently represents H or C₁₋₃ alkyl;
the cycloalkylene (e.g., C₃₋₂₀ cycloalkylene), the arylene (e.g., C₃₋₂₀ arylene), the heterocyclylene (e.g., 4-to 20-membered heterocyclylene) and the heteroarylene (e.g., 5- to 20-membered heteroarylene) are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof;
Rₐ₈, Rₐ₉, Rₐ₁₀, Rₐ₁₁, Rₐ₁₂, Rₐ₁₃, Rₐ₁₄, and Rₐ₁₅ each independently represent H, deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof; and
n1, n2, n3, n4, m1, m2, and m3 each independently represent an integer of 1, 2, 3, 4, 5, 6, 7, 8, 9 or 10.

In some embodiments, LIN of the compounds of Formula (I) represents the following groups:
-CH₂-O-CH₂-, -CH₂-O-(CH₂)₂-, -(CH₂)₁-O-(CH₂)₃-, -(CH₂)₁-O-(CH₂)₄-, -(CH₂)₁-O-(CH₂)₅-, -(CH₂)₁-O-(CH₂)₆-, -(CH₂)₁-O-(CH₂)₇-, -(CH₂)₁-O-(CH₂)₈-, -(CH₂)₁-O-(CH₂)₉-, -(CH₂)₁-O-(CH₂)₁₀-, -(CH₂)₂-O-(CH₂)₁-, -(CH₂)₂-O-(CH₂)₂-, -(CH₂)₂-O-(CH₂)₃-, -(CH₂)₂-O-(CH₂)₄-, -(CH₂)₂-O-(CH₂)₅-, -(CH₂)₂-O-(CH₂)₆-, -(CH₂)₂-O-(CH₂)₇-, -(CH₂)₂-O-(CH₂)₈-, -(CH₂)₂-O-(CH₂)₉-, -(CH₂)₂-O-(CH₂)₁₀-, -(CH₂)₃-O-(CH₂)₁-, -(CH₂)₃-O-(CH₂)₂-, -(CH₂)₃-O-(CH₂)₃-, -(CH₂)₃-O-(CH₂)₄-, -(CH₂)₃-O-(CH₂)₅-, -(CH₂)₃-O-(CH₂)₆-, -(CH₂)₃-O-(CH₂)₇-, - (CH₂)₄-O-(CH₂)₁-, -(CH₂)₄-O-(CH₂)₂-, -(CH₂)₄-O-(CH₂)₃-, -(CH₂)₄-O-(CH₂)₄-, -(CH₂)₄-O-(CH₂)₅-, -(CH₂)₄-O-(CH₂)₆-, -(CH₂)₅-O-(CH₂)₁-, -(CH₂)₅-O-(CH₂)₂-, -(CH₂)₅-O-(CH₂)₃-, -(CH₂)₅-O-(CH₂)₄-, -(CH₂)₅-O-(CH₂)₅-, - (CH₂)₆-O-(CH₂)₁-, -(CH₂)₆-O-(CH₂)₂-, -(CH₂)₆-O-(CH₂)₃-, -(CH₂)₆-O-(CH₂)₄-, -(CH₂)₇-O-(CH₂)₁-, -(CH₂)₇-O-(CH₂)₂-, -(CH₂)₇-O-(CH₂)₃-, -(CH₂)₈-O-(CH₂)₁-, -(CH₂)₈-O-(CH₂)₂-, -CH(CH₃)-O-(CH₂)₁-, -CH(CH₃)-O-(CH₂)₂-, -CH(CH₃)-O-(CH₂)₃-, -CH(CH₃)-O-(CH₂)₄-, -CH(CH₃)-O-(CH₂)₅-, -CH(CH₃)-O-(CH₂)₆-, - CH(CH₃)-O-(CH₂)₇-, -CH(CH₃)-O-(CH₂)₈-, -CH(CH₃)-O-(CH₂)₉-, -CH(CH₃)-O-(CH₂)₁₀-, -CH₂-(O(CH₂)₂)₂-, -CH₂-(O(CH₂)₂)₃-, -CH₂-(O(CH₂)₂)₄-, -CH₂-(O(CH₂)₂)₅-, -CH₂-(O(CH₂)₂)₆-, -CH₂-(O(CH₂)₂)₇-, -CH₂-(O(CH₂)₂)₁-OCH₂-, -CH₂-(O(CH₂)₂)₂-OCH₂-, -CH₂-(O(CH₂)₂)₃-OCH₂-, -CH₂-(O(CH₂)₂)₄-OCH₂-, -CH₂-(O(CH₂)₂)₅-OCH₂-, -CH₂-(O(CH₂)₂)₆-OCH₂-, -CH₂-(O(CH₂)₂)₇-OCH₂-, -(CH₂)₂-(O(CH₂)₂)₂-, -(CH₂)₂-(O(CH₂)₂)₃-, -(CH₂)₂-(O(CH₂)₂)₄-, -(CH₂)₂-(O(CH₂)₂)₅-, -(CH₂)₂-(O(CH₂)₂)₆-, -(CH₂)₂-(O(CH₂)₂)₇-, -(CH₂)₃-(O(CH₂)₂)₂-, -(CH₂)₃-(O(CH₂)₂)₃-, -(CH₂)₃-(O(CH₂)₂)₄-, -(CH₂)₃-(O(CH₂)₂)₅-, -(CH₂)₃-(O(CH₂)₂)₆-, -(CH₂)₃-(O(CH₂)₂)₇-, -(CH₂)₄-(O(CH₂)₂)₂-, -(CH₂)₄-(O(CH₂)₂)₃-, -(CH₂)₄-(O(CH₂)₂)₄-, -(CH₂)₄-(O(CH₂)₂)₅-, -(CH₂)₄-(O(CH₂)₂)₆-, -(CH₂)₄-(O(CH₂)₂)₇-, -CH₂-(O(CH₂)₃)₂-, -CH₂-(O(CH₂)₃)₃-, -CH₂-(O(CH₂)₃)₄-, -(CH₂)₂-(O(CH₂)₃)₂-, -(CH₂)₂-(O(CH₂)₃)₃-, -(CH₂)₂-(O(CH₂)₃)₄-, -(CH₂)₃-(O(CH₂)₃)₂-, -(CH₂)₃-(O(CH₂)₃)₃-, -(CH₂)₃-(O(CH₂)₃)₄-, -CH₂-O-(CH₂)₂-O-(CH₂)₃-, -CH₂-(O(CH₂)₂)₂-(O(CH₂)₃)₂-, -CH₂-(O(CH₂)₂)₃-(O(CH₂)₃)₃-, -CH₂-(O(CH₂)₂)₄-(O(CH₂)₃)₄-, (CH₂)₂-O-(CH₂)₂-O-(CH₂)₃-, -(CH₂)₂-(O(CH₂)₂)₂-(O(CH₂)₃)₂-, -(CH₂)₃-O-(CH₂)₂-O-(CH₂)₃-, -(CH₂)₃-(O(CH₂)₂)₂-(O(CH₂)₃)₂-, -CH₂-O-(CH₂)₃-O-(CH₂)₂-, -CH₂-(O(CH₂)₃)₂-(O(CH₂)₂)₂-, -(CH₂)₂-O-(CH₂)₃-O-(CH₂)₂-, -(CH₂)₂-(O(CH₂)₃)₂-(O(CH₂)₂)₂-, -(CH₂)₃-O-(CH₂)₃-O-(CH₂)₂-, -(CH₂)₃-(O(CH₂)₃)₂-(O(CH₂)₂)₂-, -CH₂-O-(CH₂)₂-O-CH₂-, -(CH₂)₂-O-(CH₂)₂-O-CH₂-, -(CH₂)₂-(O(CH₂)₂)₂-O-(CH₂)₃-, -(CH₂)₂-(O(CH₂)₂)₃-O-(CH₂)₃-, -(CH₂)₂-(O(CH₂)₂)₄-O-(CH₂)₃-, -(CH₂)₅-(O(CH₂)₂)₂-O-(CH₂)₅-, -(CH₂)₅-(O(CH₂)₂)₂-O-(CH₂)₆-, -(CH₂)₁-N(Rₐ₇)-(CH₂)₁-, -(CH₂)₁-N(Rₐ₇)-(CH₂)₂-, -(CH₂)₁-N(Rₐ₇)-(CH₂)₃-, -(CH₂)₁-N(Rₐ₇)-(CH₂)₄-, - (CH₂)₁-N(Rₐ₇)-(CH₂)₅-, -(CH₂)₁-N(Rₐ₇)-(CH₂)₆-, -(CH₂)₁-N(Rₐ₇)-(CH₂)₇-, -(CH₂)₁-N(Rₐ₇)-(CH₂)₈-, -(CH₂)₁-N(Rₐ₇)-(CH₂)₉-, -(CH₂)₁-N(Rₐ₇)-(CH₂)₁₀-, -(CH₂)₂-N(Rₐ₇)-(CH₂)₁-, -(CH₂)₂-N(Rₐ₇)-(CH₂)₂-, -(CH₂)₂-N(Rₐ₇)-(CH₂)₃-, -(CH₂)₂-N(Rₐ₇)-(CH₂)₄-, -(CH₂)₂-N(Rₐ₇)-(CH₂)₅-, -(CH₂)₂-N(Rₐ₇)-(CH₂)₆-, -(CH₂)₂-N(Rₐ₇)-(CH₂)₇-, - (CH₂)₂-N(Rₐ₇)-(CH₂)₈-, -(CH₂)₂-N(Rₐ₇)-(CH₂)₉-, -(CH₂)₂-N(Rₐ₇)-(CH₂)₁₀-, -(CH₂)₃-N(Rₐ₇)-(CH₂)₁-, -(CH₂)₃-N(Rₐ₇)-(CH₂)₂-, -(CH₂)₃-N(Rₐ₇)-(CH₂)₃-, -(CH₂)₄-N(Rₐ₇)-(CH₂)₁-, -(CH₂)₄-N(Rₐ₇)-(CH₂)₂-, -(CH₂)₄-N(Rₐ₇)-(CH₂)₃-, -(CH₂)₄-N(Rₐ₇)-(CH₂)₄-, -(CH₂)₅-N(Rₐ₇)-(CH₂)₁-, -(CH₂)₅-N(Rₐ₇)-(CH₂)₂-, -(CH₂)₅-N(Ra₇)-(CH₂)₃-, - (CH₂)₅-N(Rₐ₇)-(CH₂)₄-, -(CH₂)₅-N(Rₐ₇)-(CH₂)₅-, -(CH₂)₆-N(Rₐ₇)-(CH₂)₁-, -(CH₂)₆-N(Rₐ₇)-(CH₂)₂-, -(CH₂)₆-N(Rₐ₇)-(CH₂)₃-, -(CH₂)₇-N(Rₐ₇)-(CH₂)₁-, -(CH₂)₇-N(Rₐ₇)-(CH₂)₂-, -(CH₂)₇-N(Rₐ₇)-(CH₂)₃-, -(CH₂)₈-N(Rₐ₇)-(CH₂)₁-, -(CH₂)₈-N(Rₐ₇)-(CH₂)₂-, -(CH₂)₈-N(Rₐ₇)-(CH₂)₃-, -CH(CH₃)-N(Rₐ₇)-(CH₂)₁-, -CH(CH₃)-N(Rₐ₇)-(CH₂)₂-, -CH(CH₃)-N(Rₐ₇)-(CH₂)₃-, -CH(CH₃)-N(Rₐ₇)-(CH₂)₄-, -CH(CH₃)-N(Rₐ₇)-(CH₂)₅-, -CH(CH₃)-N(Rₐ₇)-(CH₂)₆-, -CH(CH₃)-N(Rₐ₇)-(CH₂)₇-, -CH(CH₃)-N(Rₐ₇)-(CH₂)₈-, -CH(CH₃)-N(Rₐ₇)-(CH₂)₉-, -CH(CH₃)-N(Rₐ₇)-(CH₂)₁₀-, -CH₂C(O)NHCH₂-, -(CH₂)₂C(O)NH(CH₂)₂-, -(CH₂)₂C(O)NH(CH₂)₃-, -(CH₂)₂C(O)NH(CH₂)₄-, - (CH₂)₂C(O)NH(CH₂)₅-, -(CH₂)₃C(O)NH(CH₂)₃-, -(CH₂)₃C(O)NH(CH₂)₄-, -(CH₂)₄C(O)NH(CH₂)₄-, - (CH₂)₅C(O)NH(CH₂)₅-, -(CH₂)₆C(O)NH(CH₂)₇-, -(CH₂)₆C(O)NH(CH₂)₆-, -(CH₂)₇C(O)NH(CH₂)₇-, - (CH₂)₂C(O)NH(CH₂)₂-O-(CH₂)₂-, -CH₂NHC(O)CH₂-, -(CH₂)₂NHC(O)(CH₂)₂-, -(CH₂)₂NHC(O)(CH₂)₃-, - (CH₂)₂NHC(O)(CH₂)₄-, -(CH₂)₂NHC(O)(CH₂)₅-, -(CH₂)₃NHC(O)(CH₂)₃-, -(CH₂)₃NHC(O)(CH₂)₄-, - (CH₂)₄NHC(O)(CH₂)₄-, -(CH₂)₅NHC(O)(CH₂)₅-, -(CH₂)₆NHC(O)(CH₂)₇-, -(CH₂)₆NHC(O)(CH₂)₆-, - (CH₂)₇NHC(O)(CH₂)₇-, -(CH₂)₄NHC(O)(CH₂)₈-, -(CH₂)₂NHC(O)(CH₂)₂-O-(CH₂)₂-, -(CH₂)₄NHC(O)CH₂-, - CH₂-phenylene-CH₂-, -CH₂-phenylene-(CH₂)₂-, -CH₂-phenylene-(CH₂)₃-, -CH₂-phenylene-(CH₂)₄-, -CH₂-phenylene-(CH₂)₅-, -CH₂-phenylene-(CH₂)₆-, -CH₂-phenylene-(CH₂)₇-, -CH₂-phenylene-(CH₂)₈-, -(CH₂)₂-phenylene-(CH₂)₁-, -(CH₂)₂-phenylene-(CH₂)₂-, -(CH₂)₂-phenylene-(CH₂)₃-, -(CH₂)₂-phenylene-(CH₂)₄-, - (CH₂)₂-phenylene-(CH₂)₅-, -(CH₂)₂-phenylene-(CH₂)₆-, -(CH₂)₂-phenylene-(CH₂)₇-, -(CH₂)₂-phenylene-(CH₂)₈-, -(CH₂)₃-phenylene-CH₂-, -(CH₂)₃-phenylene-(CH₂)₂-, -(CH₂)₃-phenylene-(CH₂)₃-, -(CH₂)₃-phenylene-(CH₂)₄-, -(CH₂)₃-phenylene-(CH₂)₅-, -(CH₂)₃-phenylene-(CH₂)₆-, -(CH₂)₃-phenylene-(CH₂)₇-, - (CH₂)₃-phenylene-(CH₂)₈-, -(CH₂)₄-phenylene-CH₂-, -(CH₂)₄-phenylene-(CH₂)₂-, -(CH₂)₄-phenylene-(CH₂)₃-, -(CH₂)₄-phenylene-(CH₂)₄-, -(CH₂)₄-phenylene-(CH₂)₅-, -(CH₂)₄-phenylene-(CH₂)₆-, -(CH₂)₄-phenylene-(CH₂)₇-, -(CH₂)₄-phenylene-(CH₂)₈-, -(CH₂)₅-phenylene-(CH₂)₁-, -(CH₂)₅-phenylene-(CH₂)₂-, -(CH₂)₅-phenylene-(CH₂)₃-, -(CH₂)₅-phenylene-(CH₂)₄-, -(CH₂)₅-phenylene-(CH₂)₅-, -(CH₂)₅-phenylene-(CH₂)₆-, - (CH₂)₅-phenylene-(CH₂)₇-, -(CH₂)₅-phenylene-(CH₂)₈-, -(CH₂)₆-phenylene-(CH₂)₁-, -(CH₂)₆-phenylene-(CH₂)₂-, -(CH₂)₆-phenylene-(CH₂)₃-, -(CH₂)₆-phenylene-(CH₂)₄-, -(CH₂)₆-phenylene-(CH₂)₅-, -(CH₂)₆-phenylene-(CH₂)₆-, -(CH₂)₆-phenylene-(CH₂)₇-, -(CH₂)₆-phenylene-(CH₂)₈-, -(CH₂)₇-phenylene-(CH₂)₁-, - (CH₂)₇-phenylene-(CH₂)₂-, -(CH₂)₇-phenylene-(CH₂)₃-, -(CH₂)₇-phenylene-(CH₂)₄-, -(CH₂)₇-phenylene-(CH₂)₈-, -(CH₂)₈-phenylene-CH₂-, -(CH₂)₈-_{P}henylene-(CH₂)₂-, -(CH₂)₈-phenylene-(CH₂)₃-, -(CH₂)₈-phenylene-(CH₂)₄-, -(CH₂)₈-phenylene-(CH₂)₅-, -(CH₂)₈-phenylene-(CH₂)₆-, -(CH₂)₈-phenylene-(CH₂)₇-, - CH₂-N(Rₐ₇)-CH₂-phenylene-CH₂-, -CH₂-N(Rₐ₇)-CH₂-phenylene-(CH₂)₂-, -CH₂-N(Rₐ₇)-CH₂-phenylene-(CH₂)₃-, -CH₂-N(Rₐ₇)-CH₂-phenylene-(CH₂)₄-, -CH₂-N(Rₐ₇)-CH₂-phenylene-(CH₂)₅-, -CH₂-N(Rₐ₇)-CH₂-phenylene-(CH₂)₆-, -CH₂-N(Rₐ₇)-CH₂-phenylene-(CH₂)₇-, -CH₂-N(Rₐ₇)-CH₂-phenylene-(CH₂)₈-, -CH₂-N(Rₐ₇)-(CH₂)₂-phenylene-CH₂-, -CH₂-N(Rₐ₇)-(CH₂)₂-phenylene-(CH₂)₂-, -CH₂-N(Rₐ₇)-(CH₂)₂-phenylene-(CH₂)₃-, -CH₂-N(Rₐ₇)-(CH₂)₂-phenylene-(CH₂)₄-, -CH₂-N(Rₐ₇)-(CH₂)₂-phenylene-(CH₂)₅-, -CH₂-N(Rₐ₇)-(CH₂)₂-phenylene-(CH₂)₆-, -CH₂-N(Rₐ₇)-(CH₂)₂-phenylene-(CH₂)₇-, -CH₂-N(Rₐ₇)-(CH₂)₂-phenylene-(CH₂)₈-, -(CH₂)₂-N(Rₐ₇)-CH₂-phenylene-CH₂-, -(CH₂)₂-N(Rₐ₇)-CH₂-phenylene-(CH₂)₂-, -(CH₂)₂-N(Rₐ₇)-CH₂-phenylene-(CH₂)₃-, -(CH₂)₂-N(Rₐ₇)-CH₂-phenylene-(CH₂)₄-, -(CH₂)₂-N(Rₐ₇)-CH₂-phenylene-(CH₂)₅-, - (CH₂)₂-N(Rₐ₇)-CH₂-phenylene-(CH₂)₆-, -(CH₂)₂-N(Rₐ₇)-CH₂-phenylene-(CH₂)₇-, -(CH₂)₂-N(Rₐ₇)-CH₂-phenylene-(CH₂)₈-, -(CH₂)₃-N(Rₐ₇)-CH₂-phenylene-CH₂-, -(CH₂)₃-N(Rₐ₇)-CH₂-phenylene-(CH₂)₂-, -(CH₂)₃-N(Rₐ₇)-CH₂-phenylene-(CH₂)₃-, -(CH₂)₃-N(Rₐ₇)-CH₂-phenylene-(CH₂)₈-, -(CH₂)₄-N(Rₐ₇)-CH₂-phenylene-CH₂-, -(CH₂)₄-N(Rₐ₇)-CH₂-phenylene-(CH₂)₂-, -(CH₂)₄-N(Rₐ₇)-CH₂-phenylene-(CH₂)₃-, -(CH₂)₄-N(Rₐ₇)-CH₂-phenylene-(CH₂)₈-, -(CH₂)₅-N(Rₐ₇)-CH₂-phenylene-(CH₂)₃-, -(CH₂)₅-N(Rₐ₇)-CH₂-phenylene-(CH₂)₈-, - (CH₂)₆-N(Rₐ₇)-CH₂-phenylene-(CH₂)₃-, -(CH₂)₆-N(Rₐ₇)-CH₂-phenylene-(CH₂)₈-, -(CH₂)₇-N(Rₐ₇)-CH₂-phenylene-(CH₂)₃-, -(CH₂)₈-N(Rₐ₇)-CH₂-phenylene-CH₂-, -(CH₂)₈-N(Rₐ₇)-CH₂-phenylene-(CH₂)₂-, -(CH₂)₈-N(Rₐ₇)-CH₂-phenylene-(CH₂)₃-, -(CH₂)₈-N(Rₐ₇)-CH₂-phenylene-(CH₂)₄-, -(CH₂)₈-N(Rₐ₇)-CH₂-phenylene-(CH₂)₅-, -(CH₂)₈-N(Rₐ₇)-CH₂-phenylene-(CH₂)₆-, -CH₂-piperidinylene-CH₂-, -CH₂-piperidinylene-(CH₂)₂-, - CH₂-piperidinylene-(CH₂)₃-, -CH₂-piperidinylene-(CH₂)₄-, -CH₂-piperidinylene-(CH₂)₅-, -CH₂-piperidinylene-(CH₂)₆-, -CH₂-piperidinylene-(CH₂)₇-, -CH₂-piperidinylene-(CH₂)₈-, -(CH₂)₂-piperidinylene-(CH₂)₁-, -(CH₂)₂-piperidinylene-(CH₂)₂-, -(CH₂)₂-piperidinylene-(CH₂)₃-, -(CH₂)₂-piperidinylene-(CH₂)₄-, - (CH₂)₂-piperidinylene-(CH₂)₅-, -(CH₂)₂-piperidinylene-(CH₂)₆-, -(CH₂)₂-piperidinylene-(CH₂)₇-, -(CH₂)₂-piperidinylene-(CH₂)₈-, -(CH₂)₃-piperidinylene-CH₂-, -(CH₂)₃-piperidinylene-(CH₂)₂-, -(CH₂)₃-piperidinylene-(CH₂)₃-, -(CH₂)₃-piperidinylene-(CH₂)₄-, -(CH₂)₃-piperidinylene-(CH₂)₅-, -(CH₂)₃-piperidinylene-(CH₂)₆-, -(CH₂)₃-piperidinylene-(CH₂)₇-, -(CH₂)₃-piperidinylene-(CH₂)₈-, -(CH₂)₄-piperidinylene-CH₂-, -(CH₂)₄-piperidinylene-(CH₂)₂-, -(CH₂)₄-piperidinylene-(CH₂)₃-, -(CH₂)₄-piperidinylene-(CH₂)₄-, -(CH₂)₄-piperidinylene-(CH₂)₅-, -(CH₂)₄-piperidinylene-(CH₂)₆-, -(CH₂)₄-piperidinylene-(CH₂)₇-, -(CH₂)₄-piperidinylene-(CH₂)₈-, -(CH₂)₅-piperidinylene-(CH₂)₁-, -(CH₂)₅-piperidinylene-(CH₂)₂-, -(CH₂)₅-piperidinylene-(CH₂)₃-, -(CH₂)₅-piperidinylene-(CH₂)₄-, -(CH₂)₅-piperidinylene-(CH₂)₅-, -(CH₂)₅-piperidinylene-(CH₂)₆-, -(CH₂)₅-piperidinylene-(CH₂)₇-, -(CH₂)₅-piperidinylene-(CH₂)₈-, -(CH₂)₆-piperidinylene-(CH₂)₁-, -(CH₂)₆-piperidinylene-(CH₂)₂-, -(CH₂)₆-piperidinylene-(CH₂)₃-, -(CH₂)₆-piperidinylene-(CH₂)₄-, -(CH₂)₆-piperidinylene-(CH₂)₅-, -(CH₂)₆-piperidinylene-(CH₂)₆-, -(CH₂)₆-piperidinylene-(CH₂)₇-, -(CH₂)₆-piperidinylene-(CH₂)₈-, -(CH₂)₇-piperidinylene-(CH₂)₁-, -(CH₂)₇-piperidinylene-(CH₂)₂-, -(CH₂)₇-piperidinylene-(CH₂)₃-, -(CH₂)₇-piperidinylene-(CH₂)₄-, -(CH₂)₇-piperidinylene-(CH₂)₈-, -(CH₂)₈-piperidinylene-CH₂-, -(CH₂)₈-piperidinylene-(CH₂)₂-, -(CH₂)₈-piperidinylene-(CH₂)₃-, -(CH₂)₈-piperidinylene-(CH₂)₄-, -(CH₂)₈-piperidinylene-(CH₂)₅-, -(CH₂)₈-piperidinylene-(CH₂)₆-, -(CH₂)₈-piperidinylene-(CH₂)₇-, -CH₂-N(Rₐ₇)-CH₂-piperidinylene-CH₂-, -CH₂-N(Rₐ₇)-CH₂-piperidinylene-(CH₂)₂-, -CH₂-N(Rₐ₇)-CH₂-piperidinylene-(CH₂)₃-, - CH₂-N(Rₐ₇)-CH₂-piperidinylene-(CH₂)₄-, -CH₂-N(Rₐ₇)-CH₂-piperidinylene-(CH₂)₅-, -CH₂-N(Rₐ₇)-CH₂-piperidinylene-(CH₂)₆-, -CH₂-N(Rₐ₇)-CH₂-piperidinylene-(CH₂)₇-, -CH₂-N(Rₐ₇)-CH₂-piperidinylene-(CH₂)₈-, -CH₂-N(Rₐ₇)-(CH₂)₂-piperidinylene-CH₂-, -CH₂-N(Rₐ₇)-(CH₂)₂-piperidinylene-(CH₂)₂-, -CH₂-N(Rₐ₇)-(CH₂)₂-piperidinylene-(CH₂)₃-, -CH₂-N(Rₐ₇)-(CH₂)₂-piperidinylene-(CH₂)₄-, -CH₂-N(Rₐ₇)-(CH₂)₂-piperidinylene-(CH₂)₅-, -CH₂-N(Rₐ₇)-(CH₂)₂-piperidinylene-(CH₂)₆-, -CH₂-N(Rₐ₇)-(CH₂)₂-piperidinylene-(CH₂)₇-, -CH₂-N(Rₐ₇)-(CH₂)₂-piperidinylene-(CH₂)₈-, -(CH₂)₂-N(Rₐ₇)-CH₂-piperidinylene-CH₂-, -(CH₂)₂-N(Rₐ₇)-CH₂-piperidinylene-(CH₂)₂-, -(CH₂)₂-N(Rₐ₇)-CH₂-piperidinylene-(CH₂)₃-, -(CH₂)₂-N(Rₐ₇)-CH₂-piperidinylene-(CH₂)₄-, -(CH₂)₂-N(Rₐ₇)-CH₂-piperidinylene-(CH₂)₅-, -(CH₂)₂-N(Rₐ₇)-CH₂-piperidinylene-(CH₂)₆-, -(CH₂)₂-N(Rₐ₇)-CH₂-piperidinylene-(CH₂)₇-, -(CH₂)₂-N(Rₐ₇)-CH₂-piperidinylene-(CH₂)₈-, -(CH₂)₃-N(Rₐ₇)-CH₂-piperidinylene-CH₂-, -(CH₂)₃-N(Rₐ₇)-CH₂-piperidinylene-(CH₂)₂-, -(CH₂)₃-N(Rₐ₇)-CH₂-piperidinylene-(CH₂)₃-, -(CH₂)₃-N(Rₐ₇)-CH₂-piperidinylene-(CH₂)₈-, -(CH₂)₄-N(Rₐ₇)-CH₂-piperidinylene-CH₂-, -(CH₂)₄-N(Rₐ₇)-CH₂-piperidinylene-(CH₂)₂-, -(CH₂)₄-N(Rₐ₇)-CH₂-piperidinylene-(CH₂)₃-, -(CH₂)₄-N(Rₐ₇)-CH₂-piperidinylene-(CH₂)₈-, -(CH₂)₅-N(Rₐ₇)-CH₂-piperidinylene-(CH₂)₃-, -(CH₂)₅-N(Rₐ₇)-CH₂-piperidinylene-(CH₂)₈-, -(CH₂)₆-N(Rₐ₇)-CH₂-piperidinylene-(CH₂)₃-, -(CH₂)₆-N(Rₐ₇)-CH₂-piperidinylene-(CH₂)₈-, -(CH₂)₇-N(Rₐ₇)-CH₂-piperidinylene-(CH₂)₃-, -(CH₂)₈-N(Rₐ₇)-CH₂-piperidinylene-CH₂-, -(CH₂)₈-N(Rₐ₇)-CH₂-piperidinylene-(CH₂)₂-, -(CH₂)₈-N(Rₐ₇)-CH₂-piperidinylene-(CH₂)₃-, -(CH₂)₈-N(Rₐ₇)-CH₂-piperidinylene-(CH₂)₄-, -(CH₂)₈-N(Rₐ₇)-CH₂-piperidinylene-(CH₂)₅-, -(CH₂)₈-N(Rₐ₇)-CH₂-piperidinylene-(CH₂)₆-, -CH₂-piperazinylene-CH₂-, -CH₂-piperazinylene-(CH₂)₂-, -CH₂-piperazinylene-(CH₂)₃-, -CH₂-piperazinylene-(CH₂)₄-, -CH₂-piperazinylene-(CH₂)₅-, -CH₂-piperazinylene-(CH₂)₆-, -CH₂-piperazinylene-(CH₂)₇-, -CH₂-piperazinylene-(CH₂)₈-, -(CH₂)₂-piperazinylene-(CH₂)₁-, -(CH₂)₂-piperazinylene-(CH₂)₂-, -(CH₂)₂-piperazinylene-(CH₂)₃-, -(CH₂)₂-piperazinylene-(CH₂)₄-, -(CH₂)₂-piperazinylene-(CH₂)₅-, -(CH₂)₂-piperazinylene-(CH₂)₆-, -(CH₂)₂-piperazinylene-(CH₂)₇-, -(CH₂)₂-piperazinylene-(CH₂)₈-, -(CH₂)₃-piperazinylene-CH₂-, -(CH₂)₃-piperazinylene-(CH₂)₂-, -(CH₂)₃-piperazinylene-(CH₂)₃-, -(CH₂)₃-piperazinylene-(CH₂)₄-, -(CH₂)₃-piperazinylene-(CH₂)₅-, -(CH₂)₃-piperazinylene-(CH₂)₆-, -(CH₂)₃-piperazinylene-(CH₂)₇-, -(CH₂)₃-piperazinylene-(CH₂)₈-, -(CH₂)₄-piperazinylene-CH₂-, -(CH₂)₄-piperazinylene-(CH₂)₂-, -(CH₂)₄-piperazinylene-(CH₂)₃-, -(CH₂)₄-piperazinylene-(CH₂)₄-, -(CH₂)₄-piperazinylene-(CH₂)₅-, -(CH₂)₄-piperazinylene-(CH₂)₆-, -(CH₂)₄-piperazinylene-(CH₂)₇-, -(CH₂)₄-piperazinylene-(CH₂)₈-, -(CH₂)₅-piperazinylene-(CH₂)₁-, -(CH₂)₅-piperazinylene-(CH₂)₂-, -(CH₂)₅-piperazinylene-(CH₂)₃-, -(CH₂)₅-piperazinylene-(CH₂)₄-, -(CH₂)₅-piperazinylene-(CH₂)₅-, -(CH₂)₅-piperazinylene-(CH₂)₆-, -(CH₂)₅-piperazinylene-(CH₂)₇-, -(CH₂)₅-piperazinylene-(CH₂)₈-, -(CH₂)₆-piperazinylene-(CH₂)₁-, -(CH₂)₆-piperazinylene-(CH₂)₂-, -(CH₂)₆-piperazinylene-(CH₂)₃-, -(CH₂)₆-piperazinylene-(CH₂)₄-, -(CH₂)₆-piperazinylene-(CH₂)₅-, -(CH₂)₆-piperazinylene-(CH₂)₆-, -(CH₂)₆-piperazinylene-(CH₂)₇-, -(CH₂)₆-piperazinylene-(CH₂)₈-, -( CH₂)₇₋piperazinylene-(CH₂)₁-, -(CH₂)₇-piperazinylene-(CH₂)₂-, -(CH₂)₇-piperazinylene-(CH₂)₃-, -(CH₂)₇-piperazinylene-(CH₂)₄-, -(CH₂)₈-piperazinylene-CH₂-, -(CH₂)₈-piperazinylene-(CH₂)₂-, -(CH₂)₈-piperazinylene-(CH₂)₃-, -(CH₂)₈-piperazinylene-(CH₂)₄-, -(CH₂)₈-piperazinylene-(CH₂)₅-, or -(CH₂)₈-piperazinylene-(CH₂)₆-;
wherein each Rₐ₇ independently represents H or C₁₋₃ alkyl;
the phenylene, piperazinylene and piperidinylene are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

In some embodiments, the compound of Formula (I) is also represented by Formula (I-1), Formula (I-2), Formula (I-3), Formula (I-4), Formula (I-5), Formula (I-6), Formula (I-7), Formula (I-8-1), Formula (I-8-2), Formula (I-8-3), Formula (I-8-4), Formula (I-9), Formula (I-10), Formula (I-11), Formula (I-12), Formula (I-13), Formula (I-14), Formula (I-15), Formula (I-16), Formula (I-17), Formula (I-18), Formula (I-19), Formula (I-20-1), Formula (I-20-2), Formula (I-20-3), Formula (I-20-4), Formula (I-21-1), Formula (I-21-2), Formula (I-22), Formula (I-23), Formula (I-24), Formula (I-25-1), Formula (I-25-2), Formula (I-26), Formula (I-27), Formula (I-28), Formula (I-29), Formula (I-30), Formula (I-31), Formula (I-32), Formula (I-33), Formula (I-34-1), Formula (I-34-2), Formula (I-35), Formula (I-36), Formula (I-37), Formula (I-38), Formula (I-39-1), Formula (I-39-2), Formula (I-40), Formula (I-41), Formula (I-42), Formula (I-43), Formula (I-44), Formula (I-45), Formula (I-46), Formula (I-47), Formula (I-48), Formula (I-49), Formula (I-50), Formula (I-51), Formula (I-52), Formula (I-53) , Formula (I-54), or Formula (I-55): 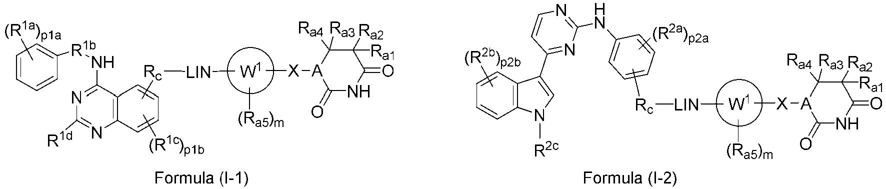 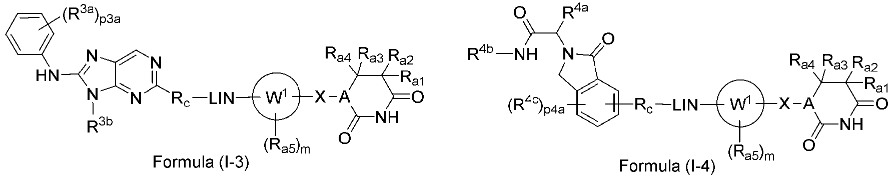 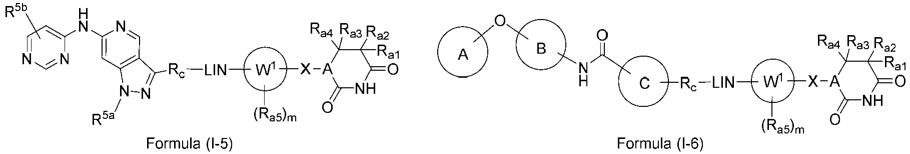 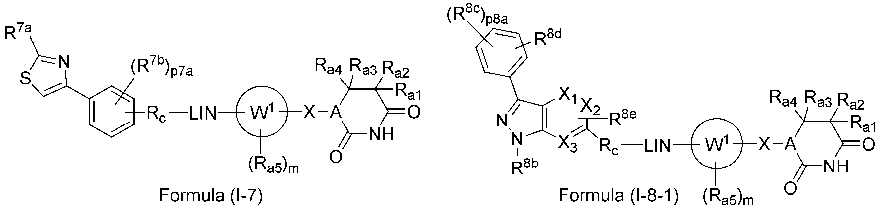 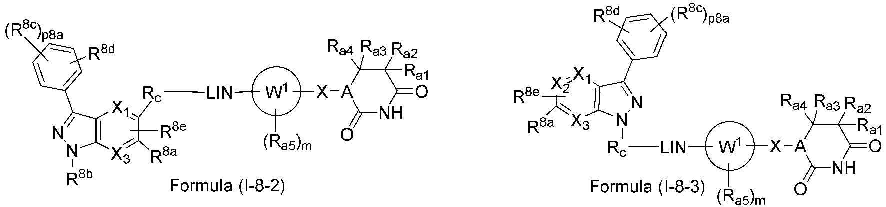 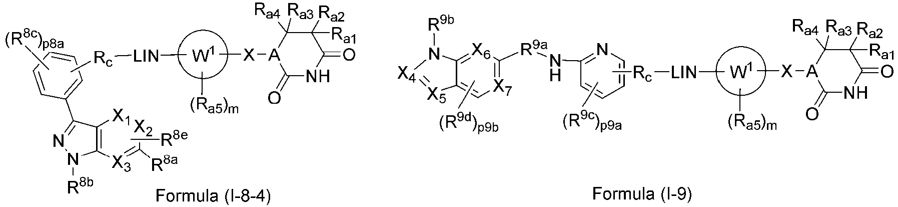 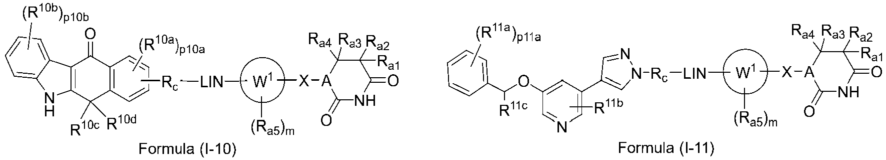 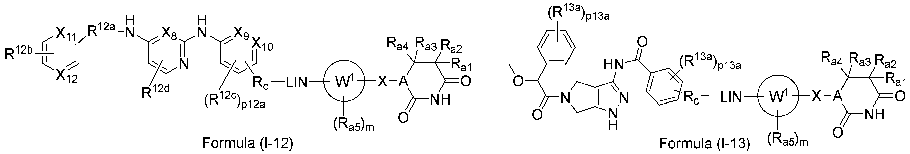 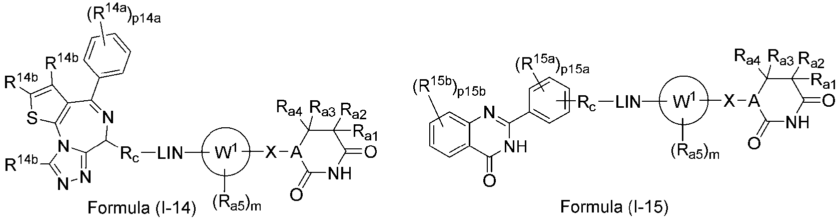 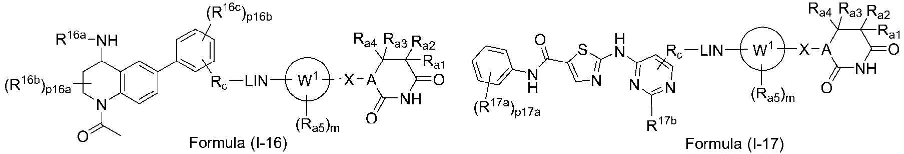 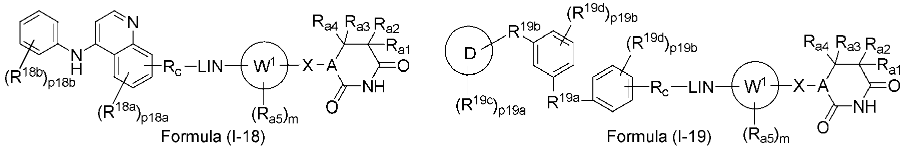 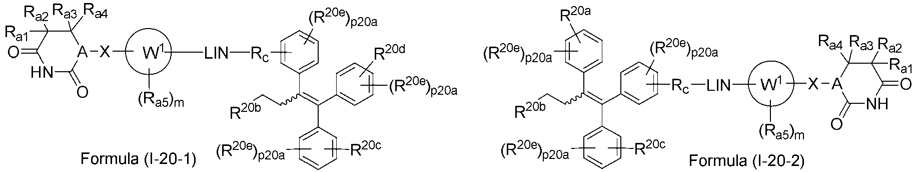 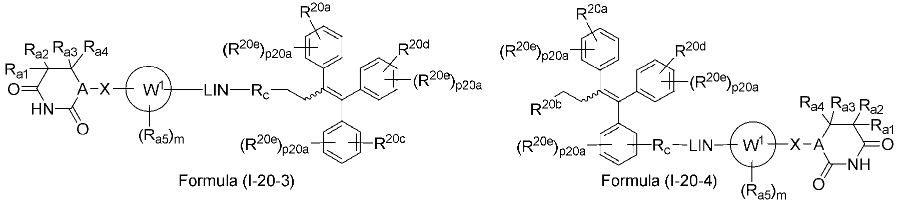 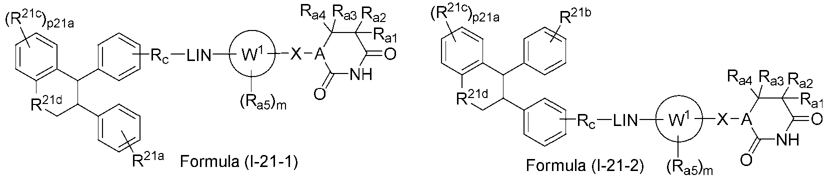 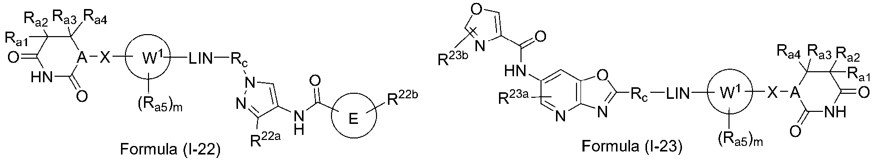 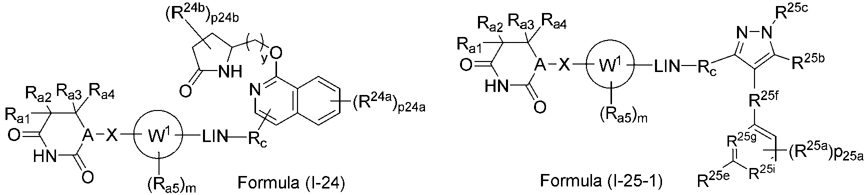 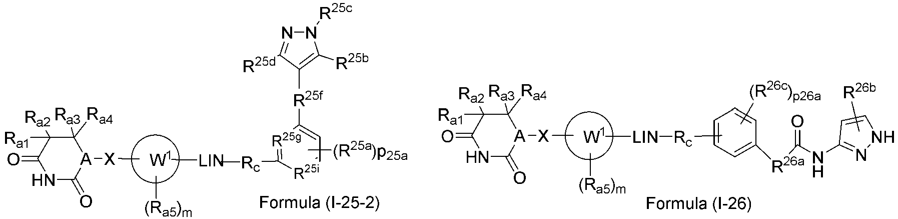 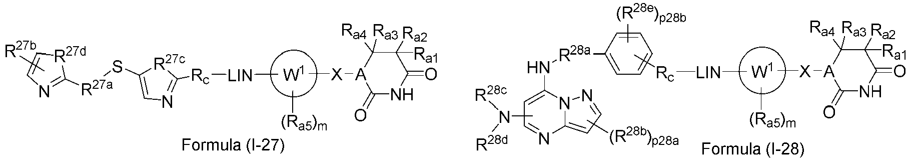 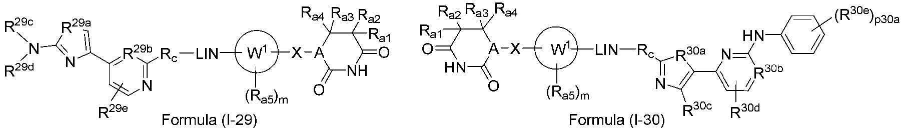 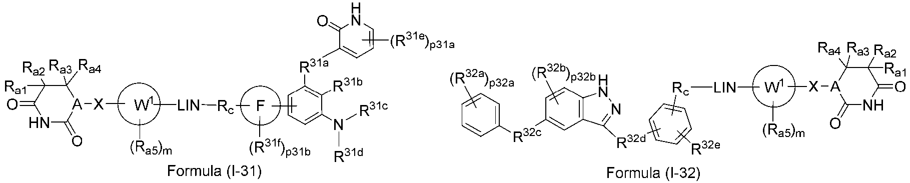 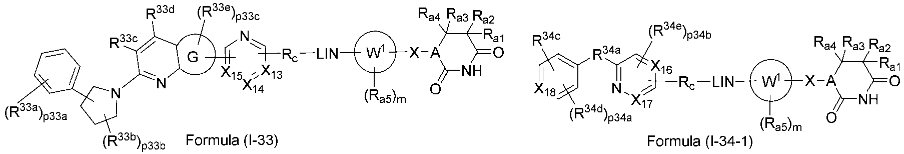 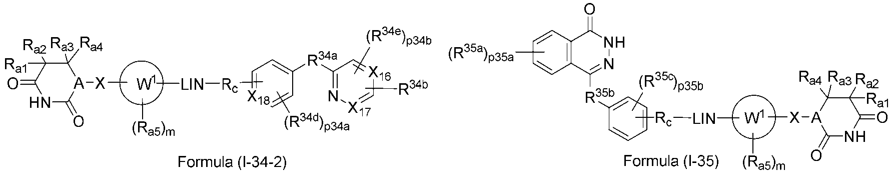 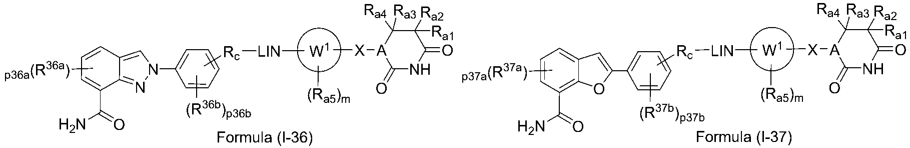 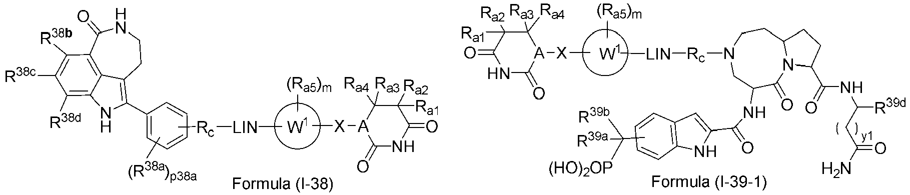 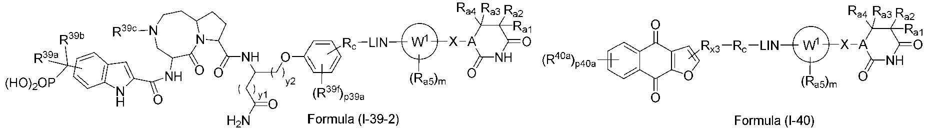 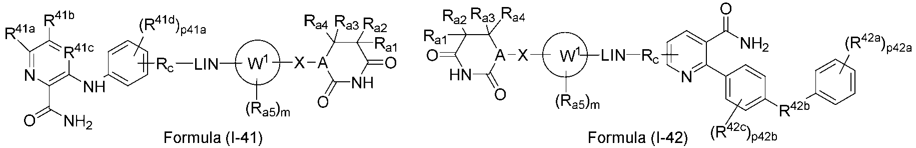 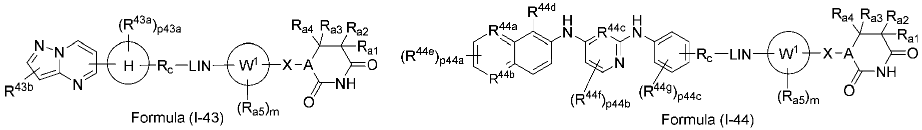 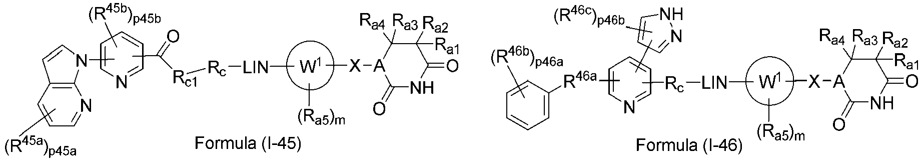 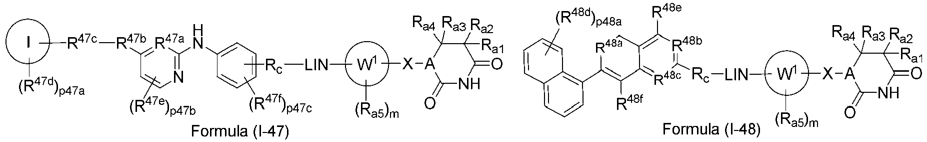 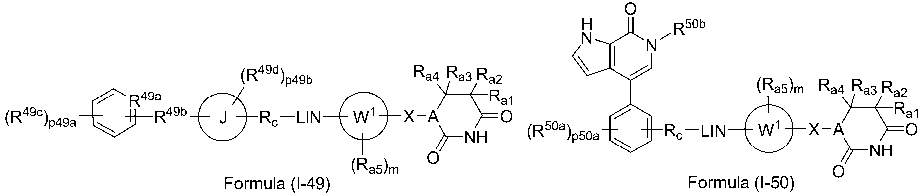 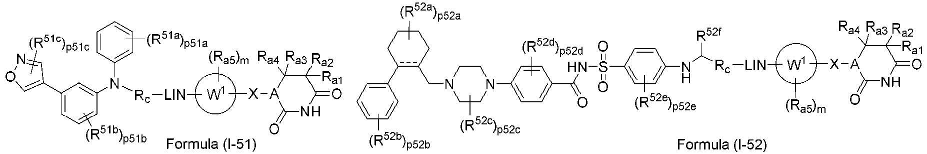 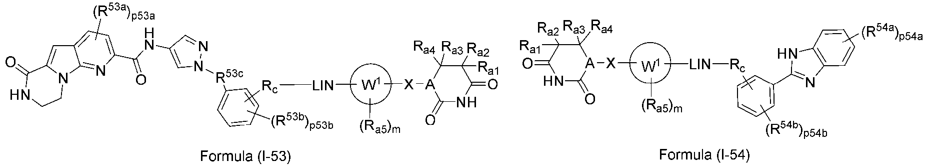 or 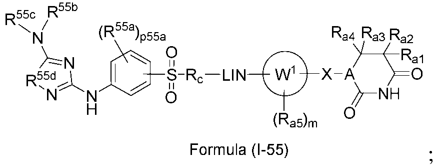 wherein A, Rₐ₁, Rₐ₂, Rₐ₃, Rₐ₄, X, ring W¹, (Rₐ₅)ₘ, LIN, R_{c}, (R^{1a})ₚ₁ₐ, R^{1b}, (R^{1c})_{p1b}, R^{1d}, (R^{2a})ₚ₂ₐ, (R^{2b})_{p2b}, R^{2c}, (R^{3a})ₚ₃ₐ, R^{3b}, R^{4a}, R^{4b}, (R^{4c} )ₚ₄ₐ, R^{5a}, R^{5b}, ring A, ring B, ring C, R^{7a}, (R^{7b})p7a, R^{8a}, R^{3b}, (R^{8c})p₈ₐ, R^{8d}, R^{8c}, X₁, X₂, X₃, R^{9a}, R^{9b}, (R^{9c})p₉ₐ, (R^{9d})p_{9b}, X₄, X₅, X₆, X₇, (R^{10a})ₚ₁₀ₐ, (R^{10b})_{p10b}, R^{10c}, R^{10d}, (R^{11a})ₚ₁₁ₐ, R^{11b}, R^{11c}, R^{12a}, R^{12b}, (R^{12c})ₚ₁₂ₐ, R^{12d}, X₈, X₉, X₁₀, X₁₁, X₁₂, (R^{13a})ₚ₁₃ₐ, (R^{14a})ₚ₁₄ₐ, R^{14b}, (R^{15a})ₚ₁₅ₐ, (R^{15b})_{p15b}, R^{16a}, (R^{16b})ₚ₁₆ₐ, (R^{16c})_{p16b}, (R^{17a})ₚ₁₇ₐ, R^{17b}, (R18^{a})ₚ₁₈ₐ, (R^{18b})_{p18b}, R^{19a}, R^{19b}, (R^{19c})ₚ₁₉ₐ, (R^{19d})_{p19b}, R^{20a}, R^{20b}, R^{20c}, R^{20d}, (R^{20e})ₚ₂₀ₐ, R^{21a}, R^{21b}, (R^{21c})_{p21a,} R^{21d}, R^{22a}, R^{22b}, ring E, R^{23a}, R^{23b}, (R^{24a})ₚ₂₄ₐ, (R^{24b})_{p24b}, y, (R^{25a})ₚ₂₅ₐ, R^{25b}, R^{25c}, R^{25d}, R^{25e}, R^{25f}, R^{25g}, R²⁵ⁱ, R^{26a}, R^{26b}, (R^{26c})ₚ₂₆ₐ, R^{27a}, R^{27b}, R^{27c}, R^{27d}, R^{28a}, (R^{28b})ₚ₂₈ₐ, R^{23c}, R^{28d}, (R^{28e})_{p28b}, R^{29a}, R^{29b}, R^{29c}, R^{29d}, R^{29e}, R^{30a}, R^{30b}, R^{30c}, R^{30d}, (R^{30e})_{p30a,} R^{31a}, R^{31b}, R^{31c}, R^{31d}, (R^{31e})ₚ₃₁ₐ, (R^{31f})_{p31b}, ring F, (R^{32a})ₚ₃₂ₐ, (R^{32b})_{p32b}, R^{32c}, R^{32d}, R^{32e}, (R^{33a})ₚ₃₃ₐ, (R^{33b})_{p33b}, R^{33c}, R^{33d}, (R^{33e})_{p33c}, X₁₃, X₁₄, X₁₅, ring G, R^{34a}, R^{34b}, R^{34c}, (R^{34d})ₚ₃₄ₐ, (R^{34e})_{p34b}, X₁₆, X₁₇, X₁₈, (R^{35a})ₚ₃₅ₐ, R^{35b}, (R^{35c})_{p35b}, (R^{36a})ₚ₃₆ₐ, (R^{36b})p_{36b}, (R^{37a})ₚ₃₇ₐ, (R^{37b})_{p37b}, (R^{38a})ₚ₃₈ₐ, R^{38b}, R^{38c}, R^{38d}, R^{39a}, R^{39b}, R^{39c}, R^{39d}, y1, (R^{40a})ₚ₄₀ₐ, R^{41a}, Rₓ₃, R^{41b}, R^{41c}, (R^{41d})ₚ₄₁ₐ, (R^{42a})ₚ₄₂ₐ, R^{42b}, (R^{42c})_{p42b,} (R^{43a})_{p43b}, ring H, R^{44a}, R^{44b}, R^{44c}, R^{44d}, (R^{44c})ₚ₄₄ₐ, (R^{44f})_{p44b}, (R^{44g})_{p44c}, (R^{45a})ₚ₄₅ₐ, (R^{45b})_{p45b}, R_{c1}, R^{46a}, (R^{46b})ₚ₄₆ₐ, (R^{46c})_{p46b}, R^{47a}, R^{47b}, R^{47c}, (R^{47d})ₚ₄₇ₐ, (R^{47e})_{p47b}, (R^{47f})_{p47c}, ring I, R^{48a}, R^{48b}, R^{48c}, (R^{48d})ₚ₄₈ₐ, R^{48e}, R^{48f}, R^{49a}, R^{49b}, (R^{49c})ₚ₄₉ₐ, (R^{49d})_{p49b}, ring J , (R^{50a})ₚ₅₀ₐ, R^{50b}, (R^{51a})ₚ₅₁ₐ, (R^{51b})_{p51b}, (R^{51c})_{p51c}, (R^{52a})ₚ₅₂ₐ, (R^{52b})_{p52b}, (R^{52c})_{p52c}, (R^{52d})_{p52d}, (R^{52e})ₚ₅₂ₑ, R^{52f}, (R^{53a})ₚ₅₃ₐ, (R^{53b})_{p53b,} R^{53c}, (R^{54a})ₚ₅₄ₐ, (R^{54b})_{p54b}, (R^{55a})ₚ₅₅ₐ, R^{55b}, R^{55c} and R^{55d} are as defined in the compound of Formula (I) and its various embodiments above.

In some embodiments, the compounds of Formula (I) and their salts (including pharmaceutically acceptable salts, such as hydrochloride, etc.), prodrugs, solvates, isotopically enriched analogs, polymorphs, stereoisomers (including enantiomers and diastereomers), or mixture of stereoisomers thereof in Table 1 below are provided:

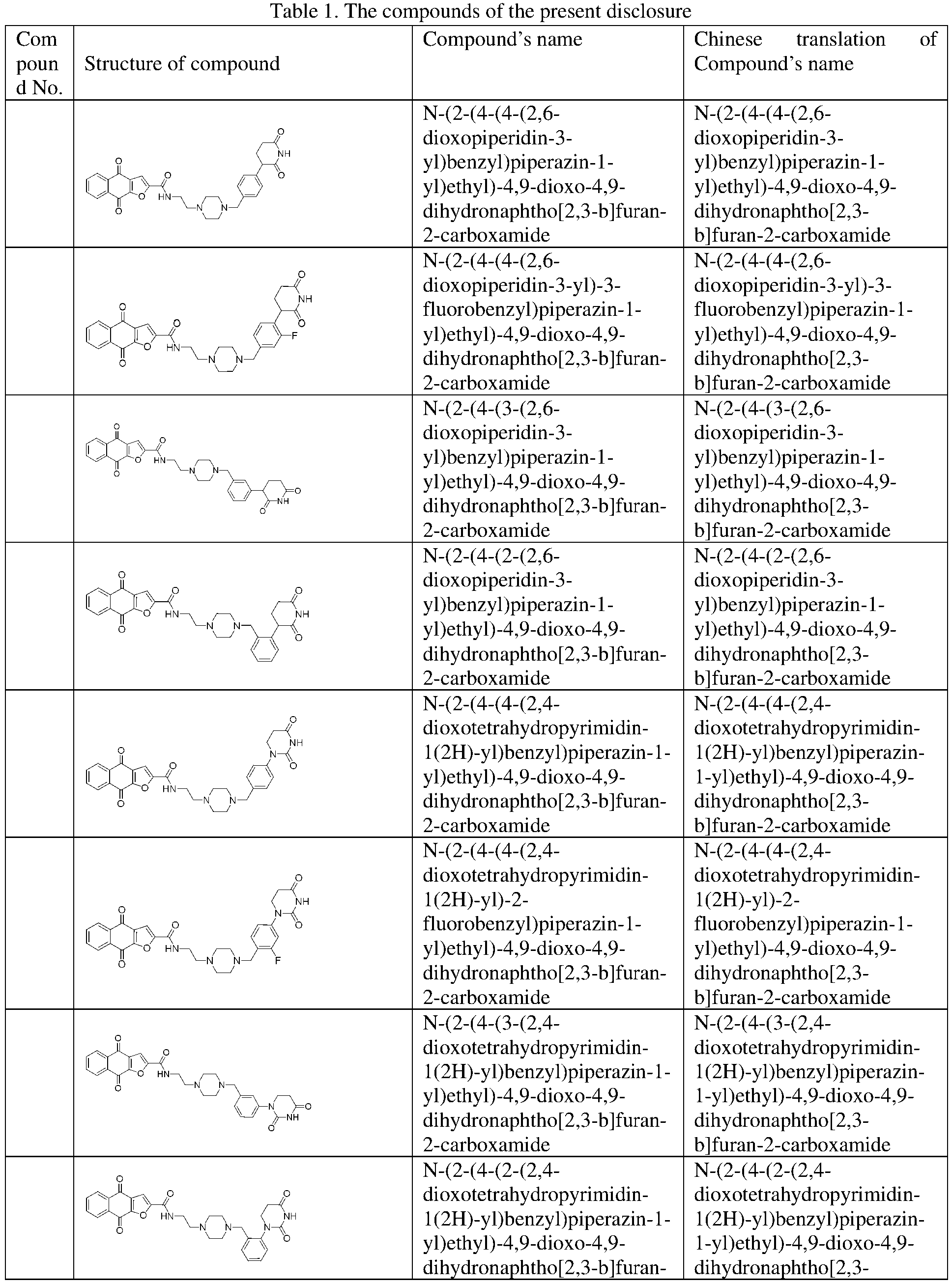

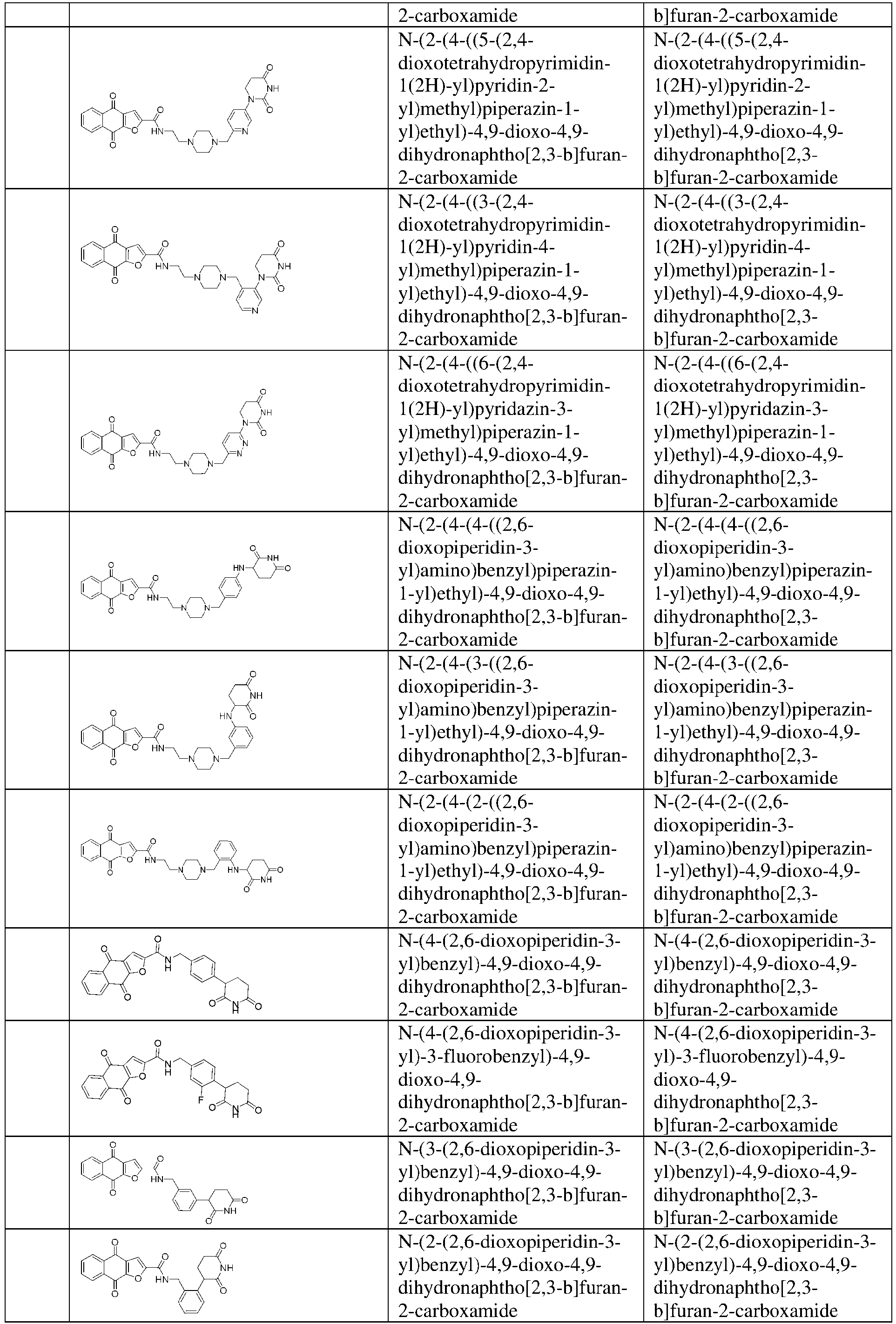

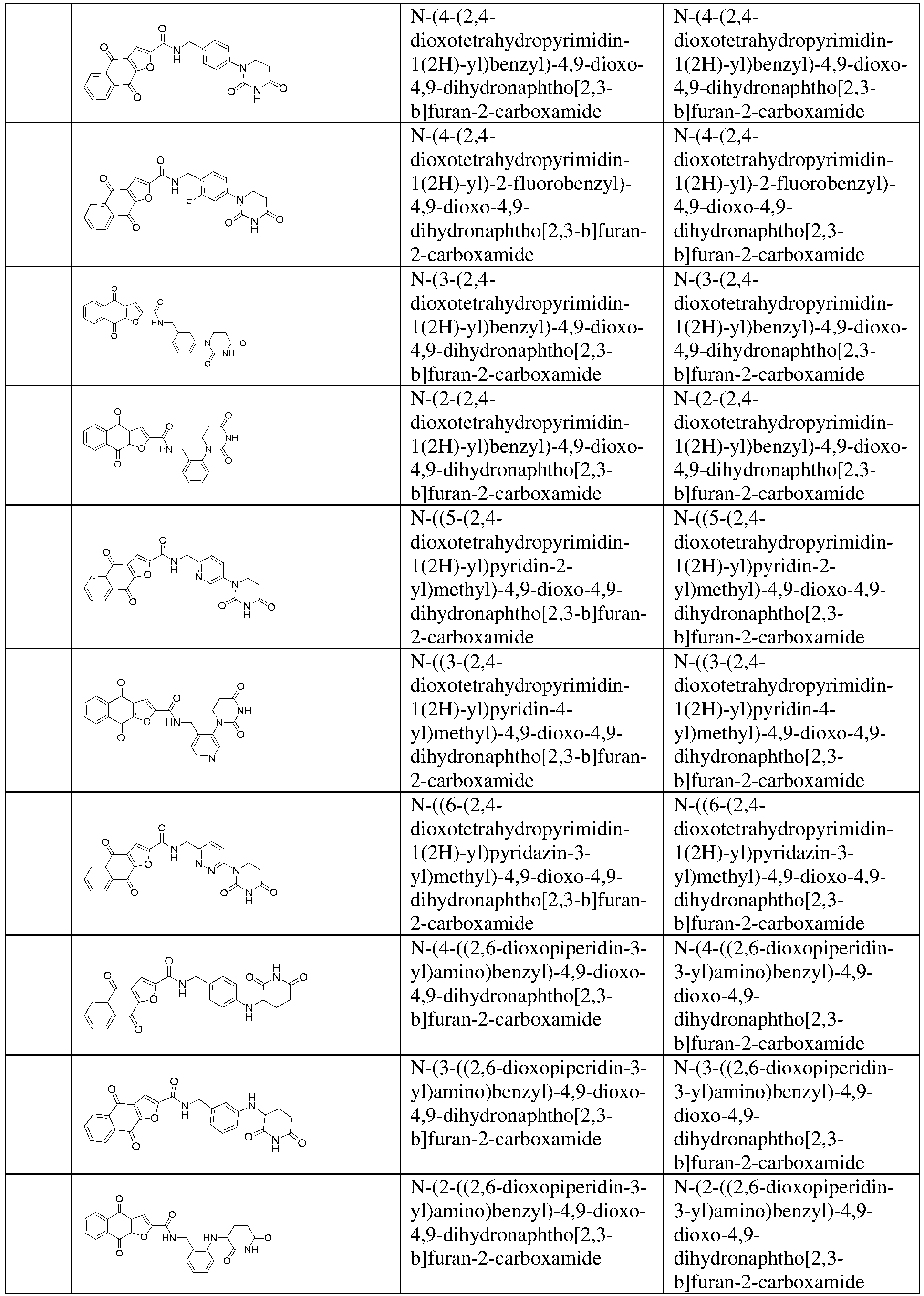

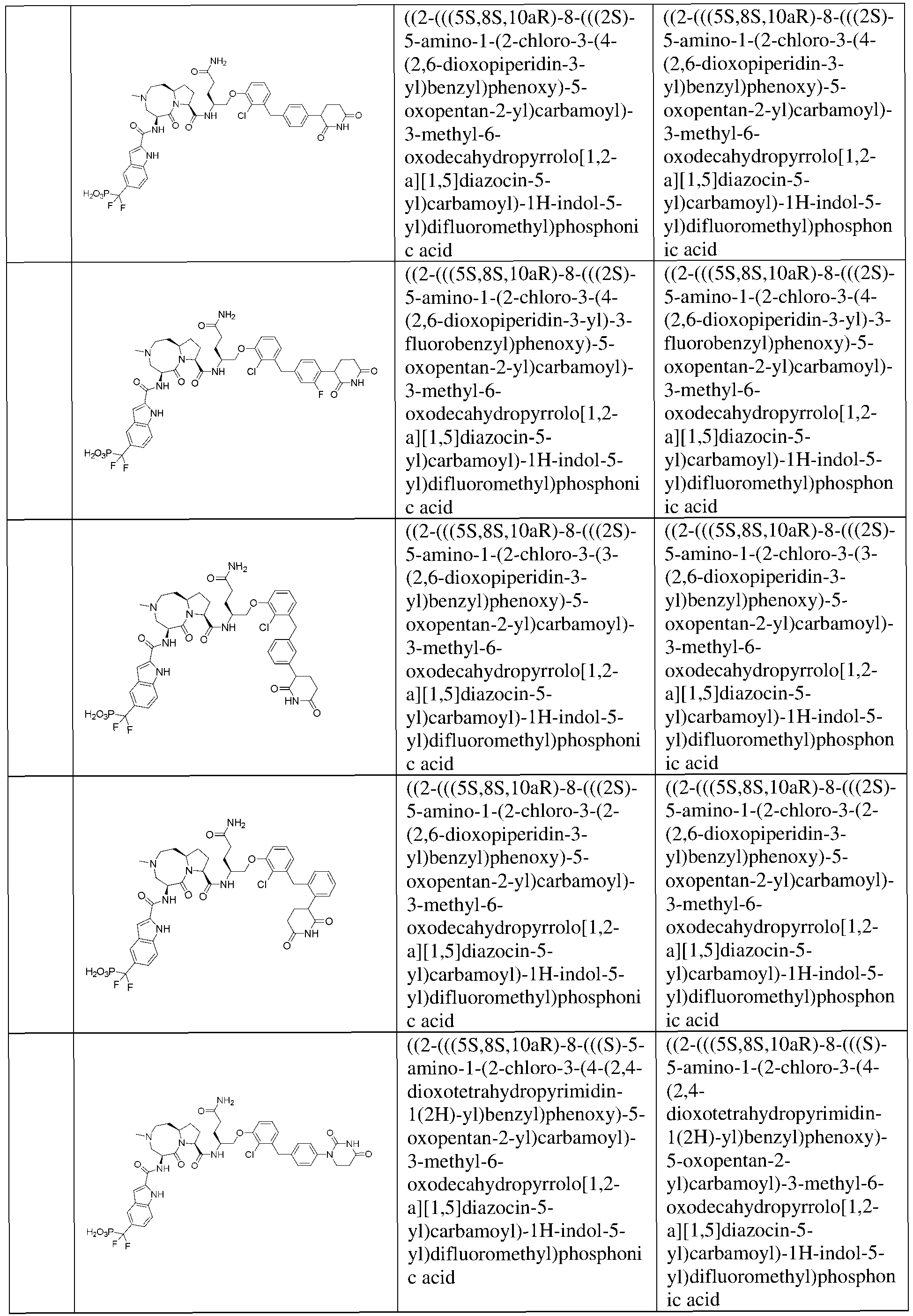

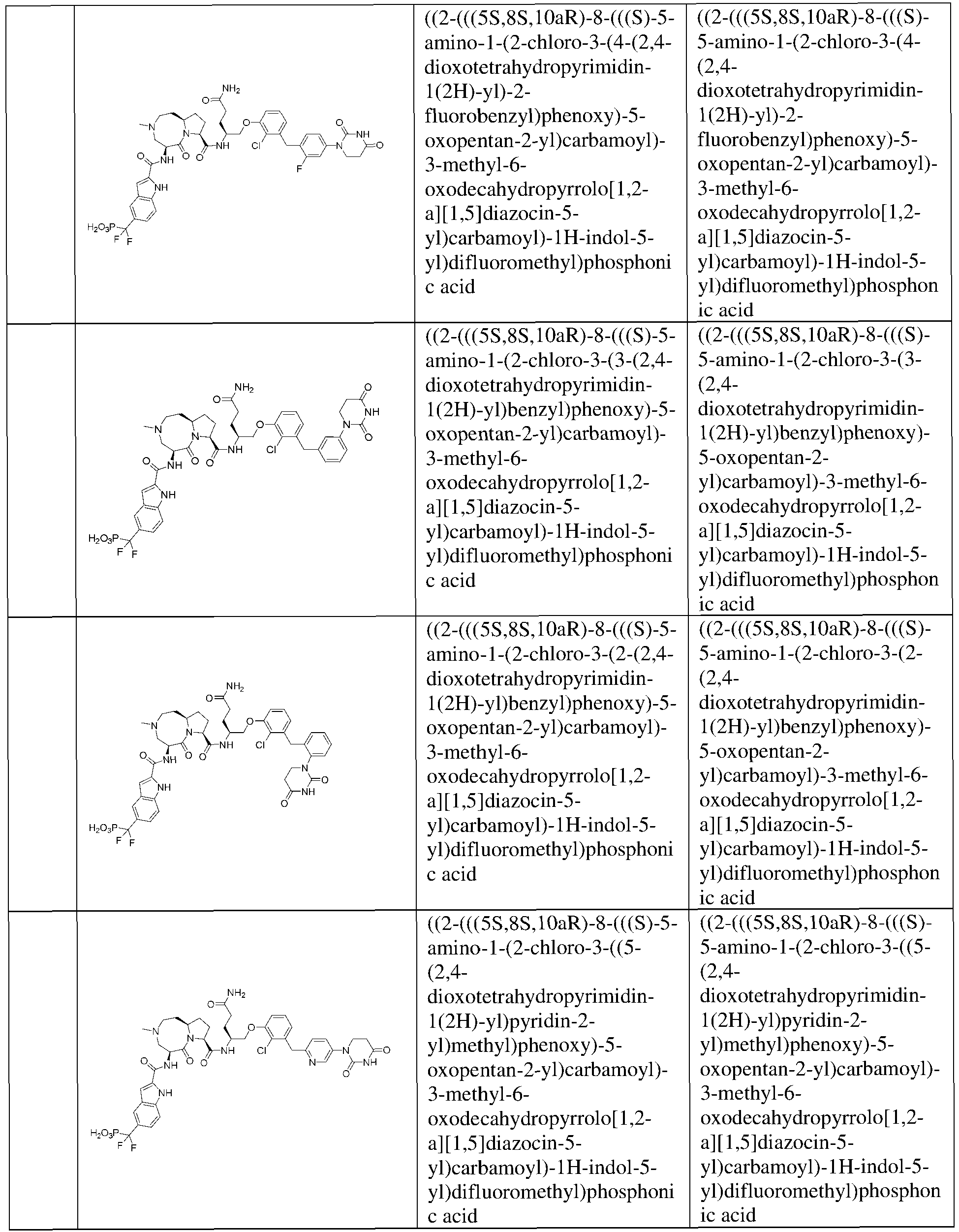

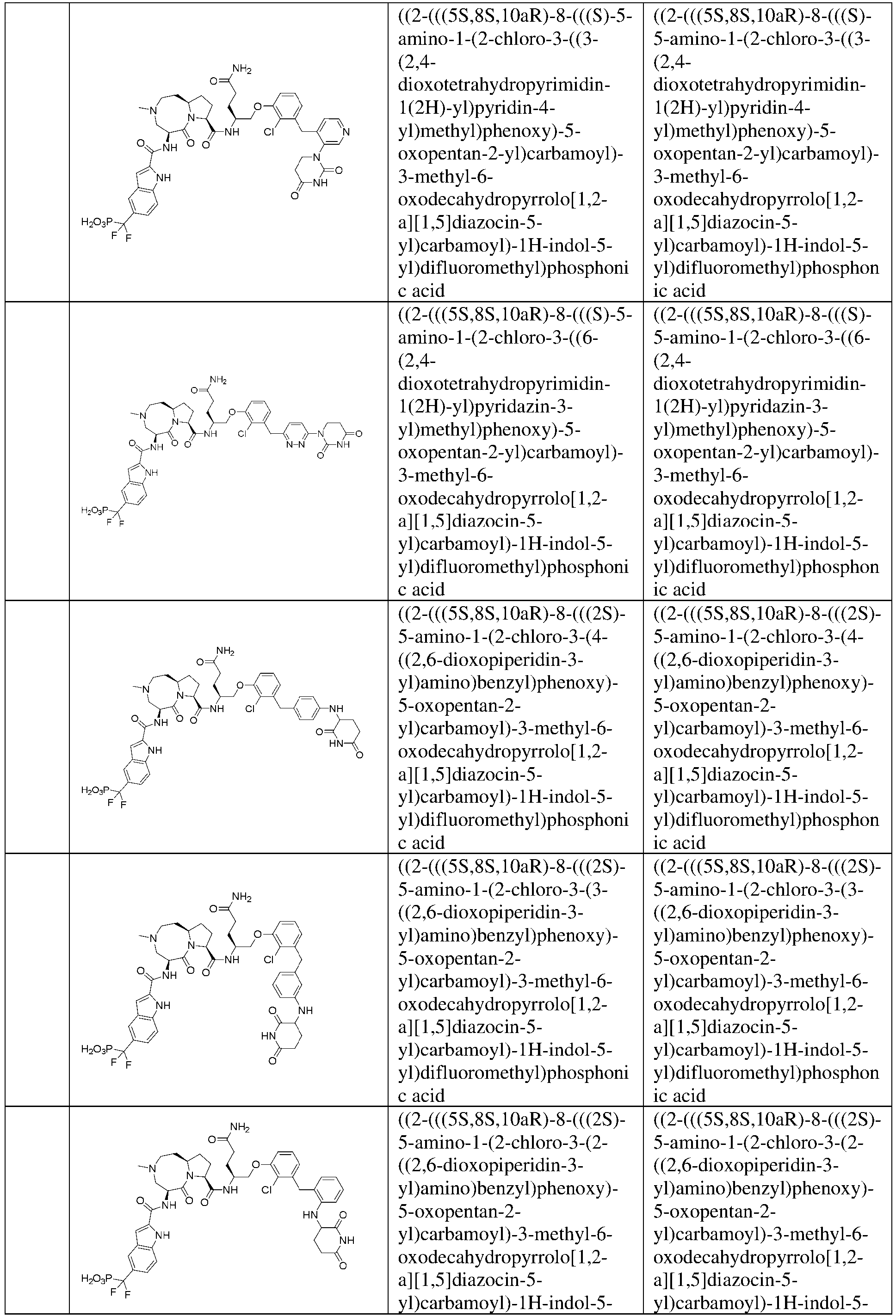

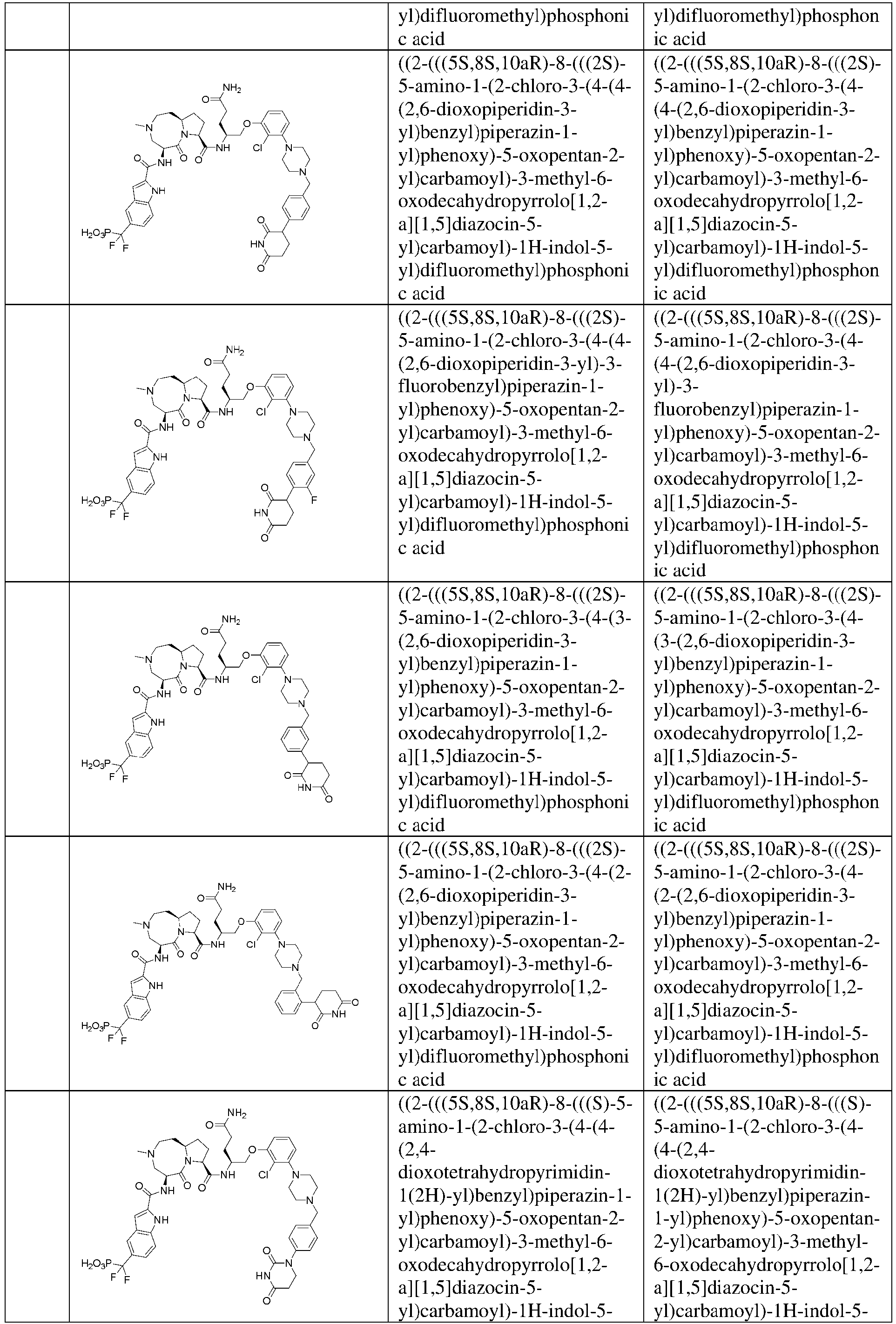

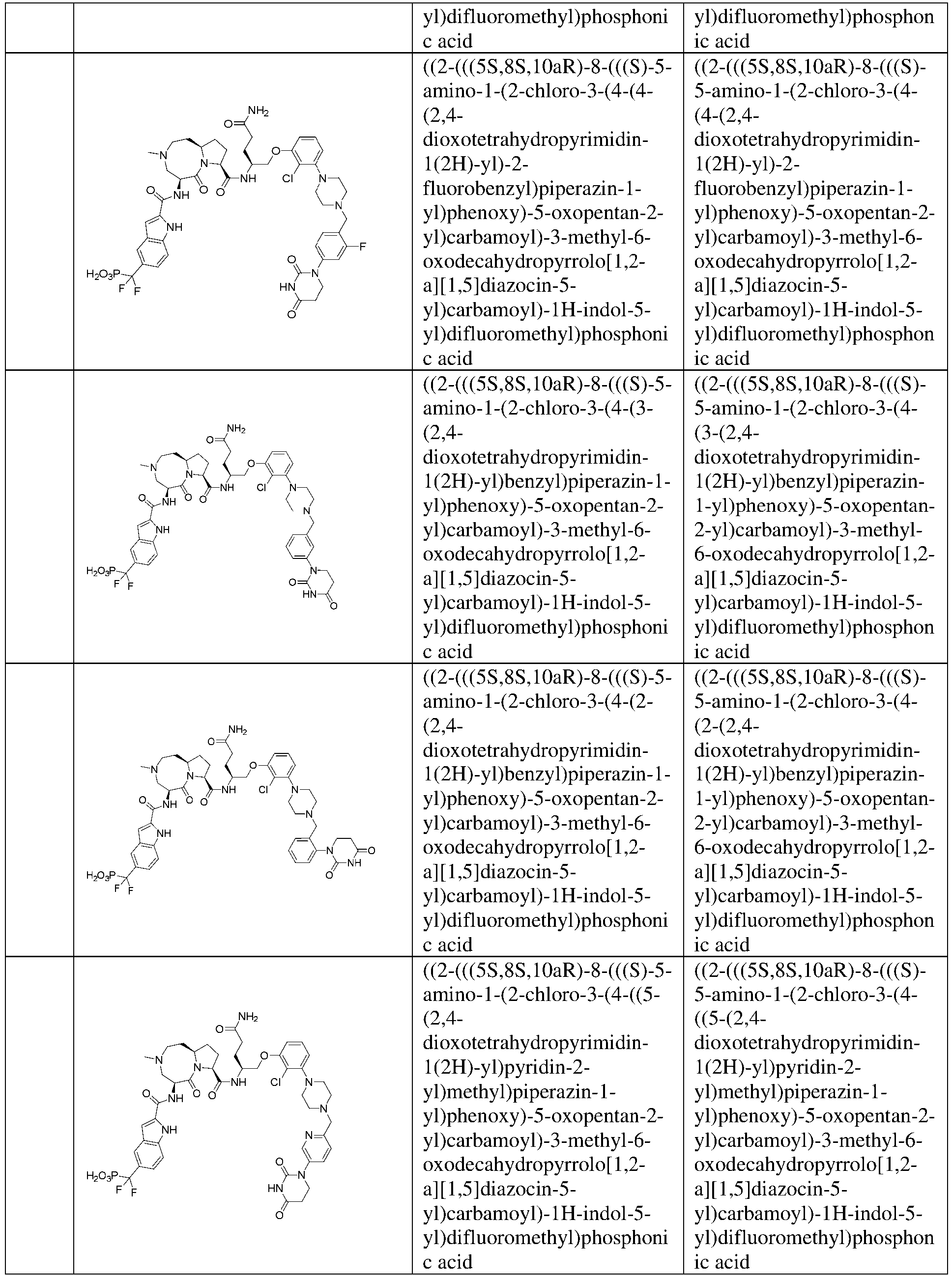

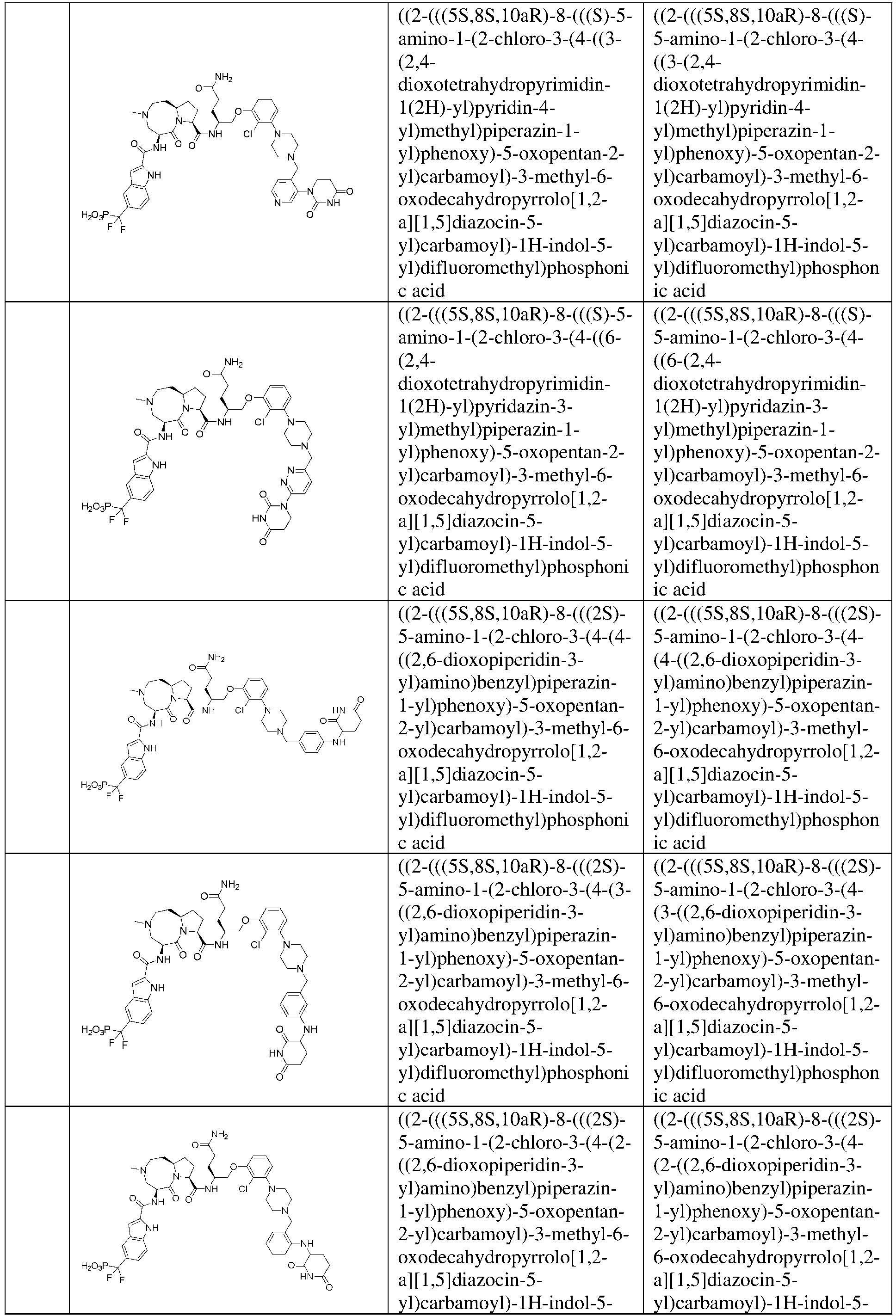

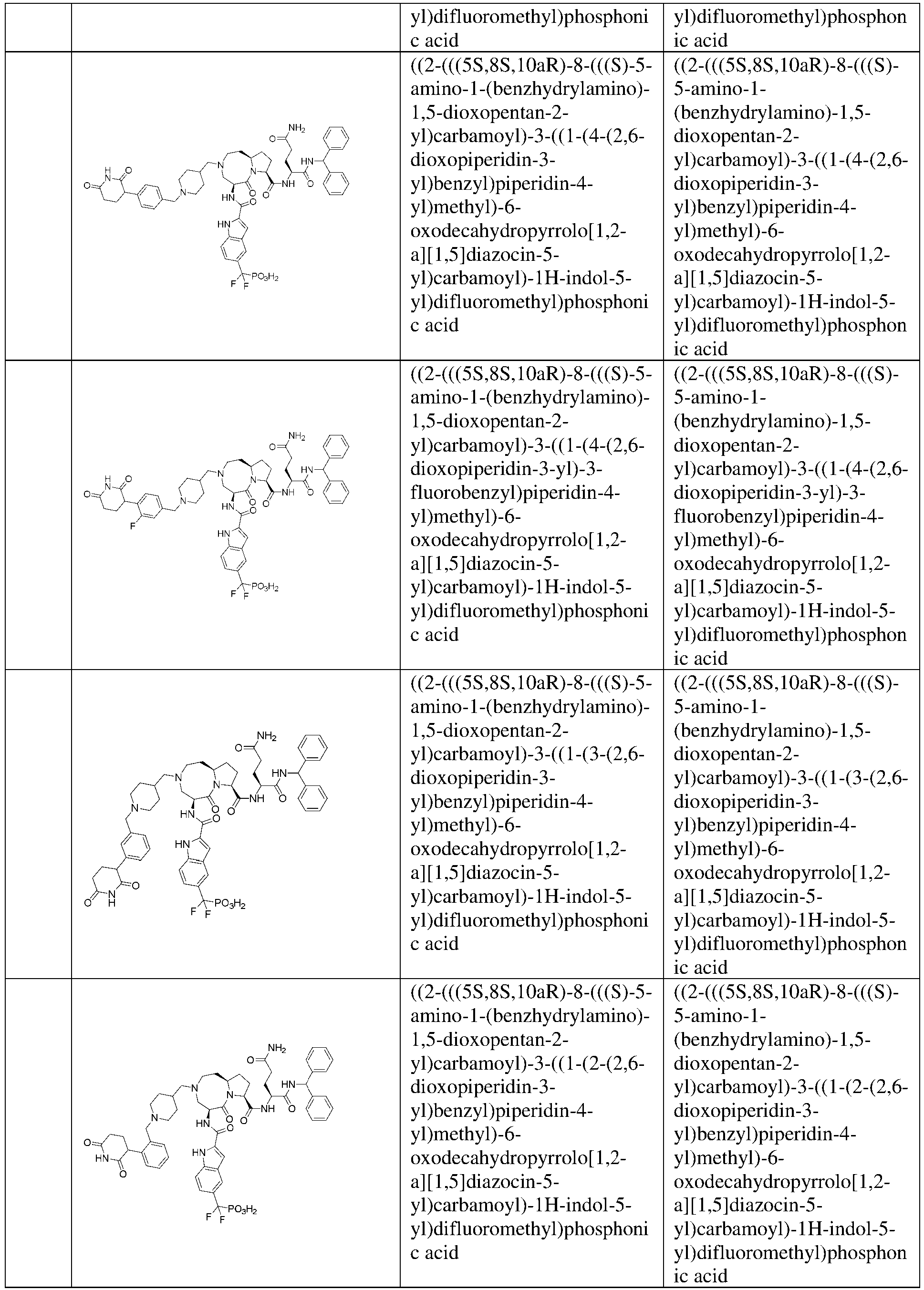

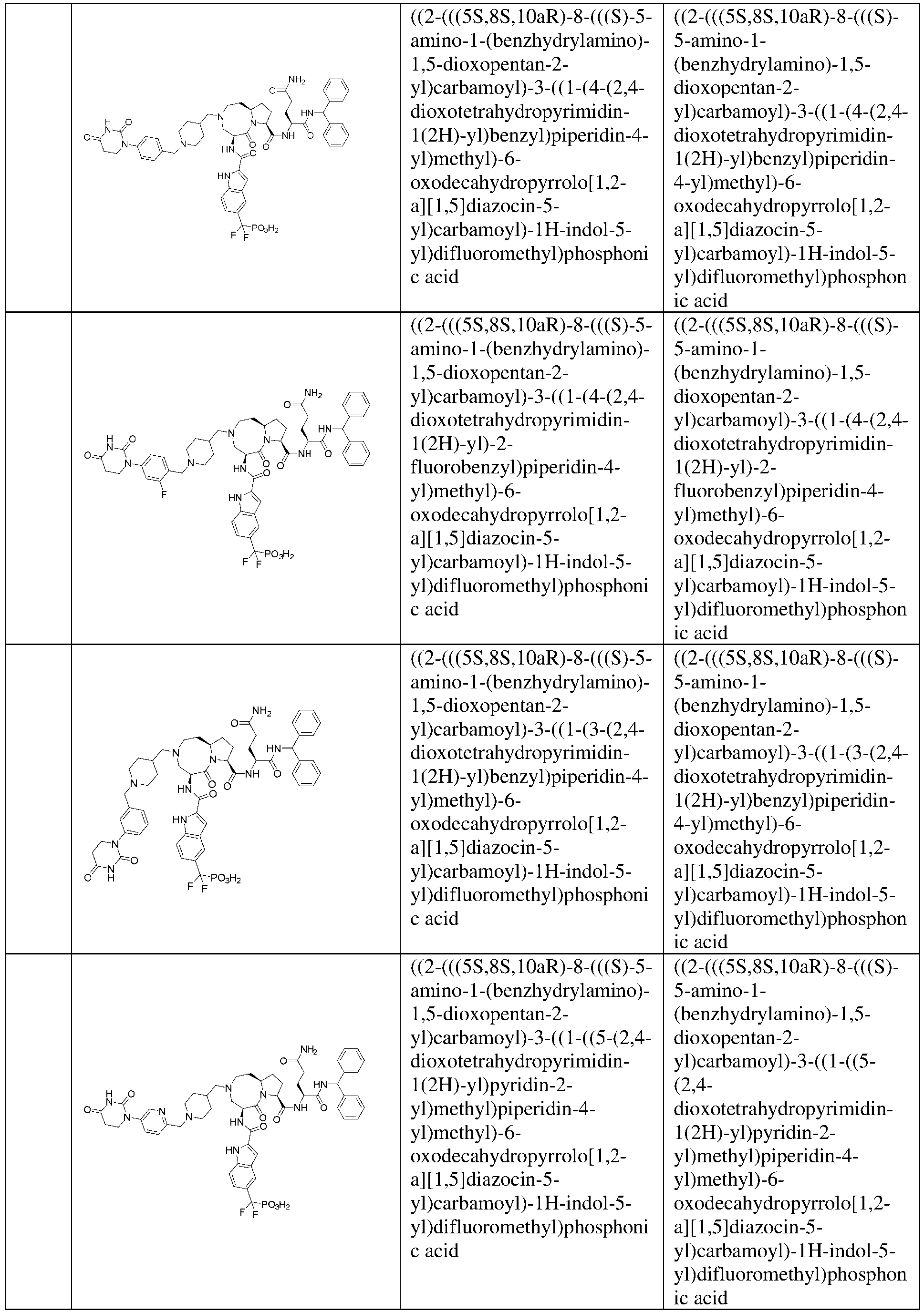

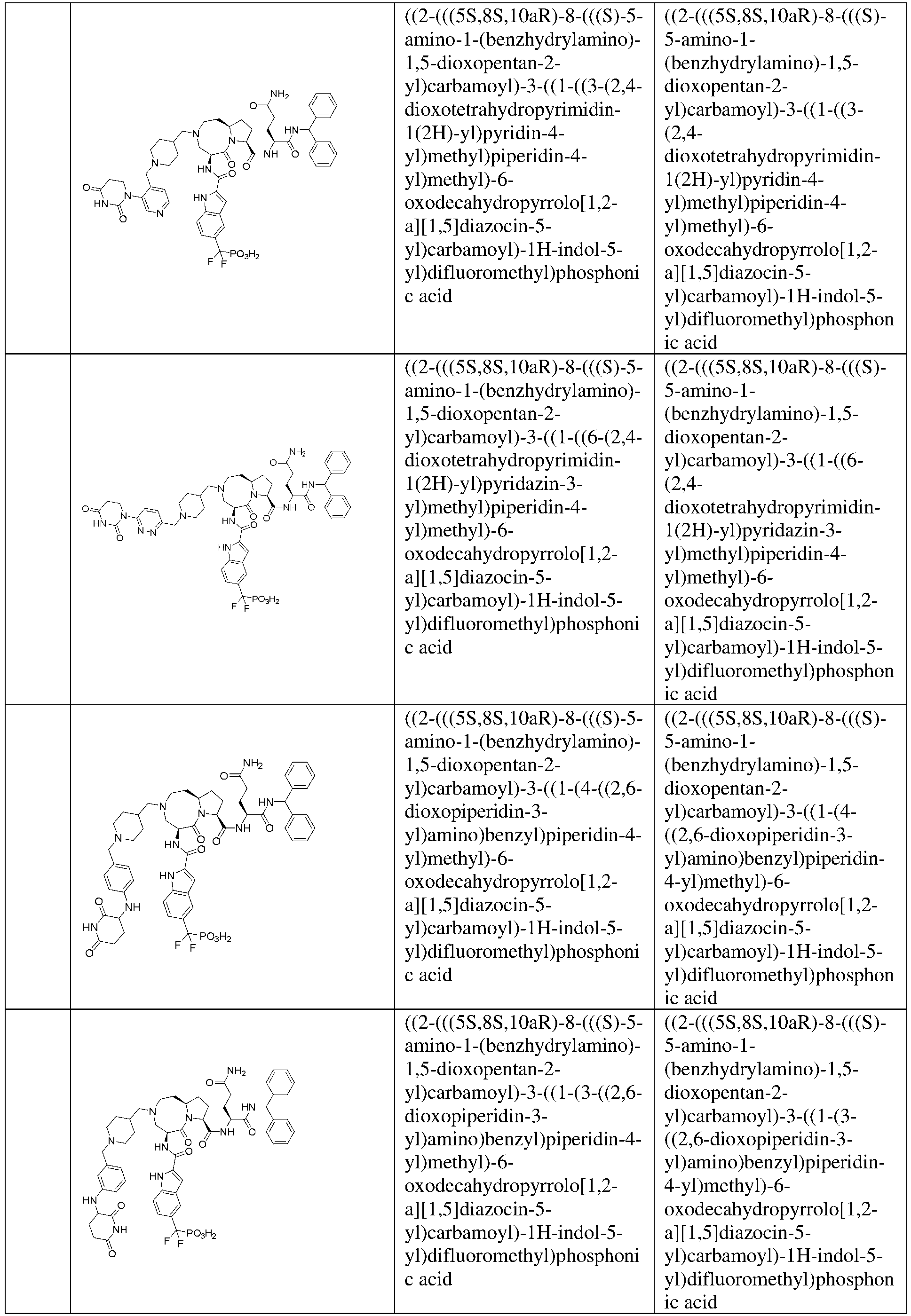

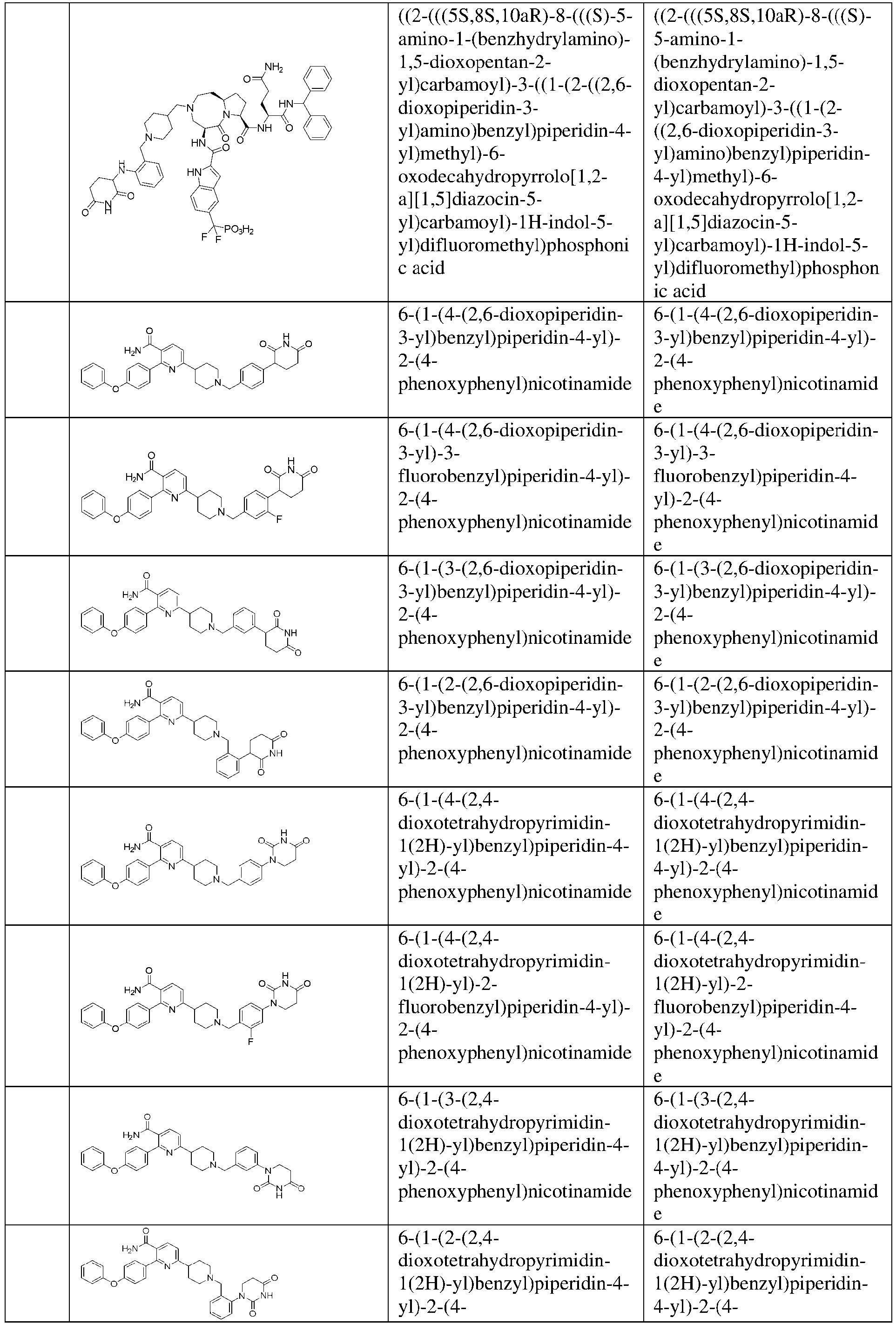

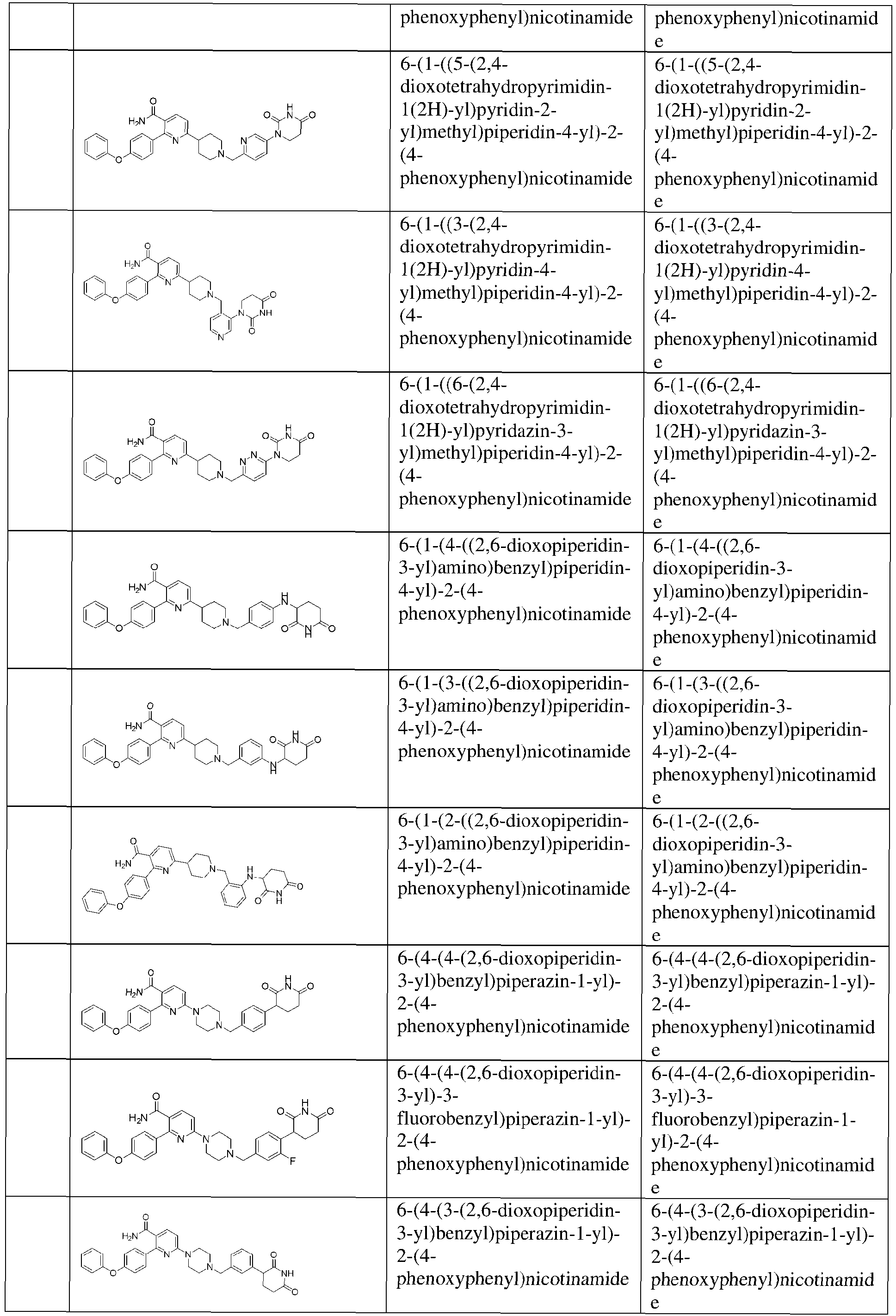

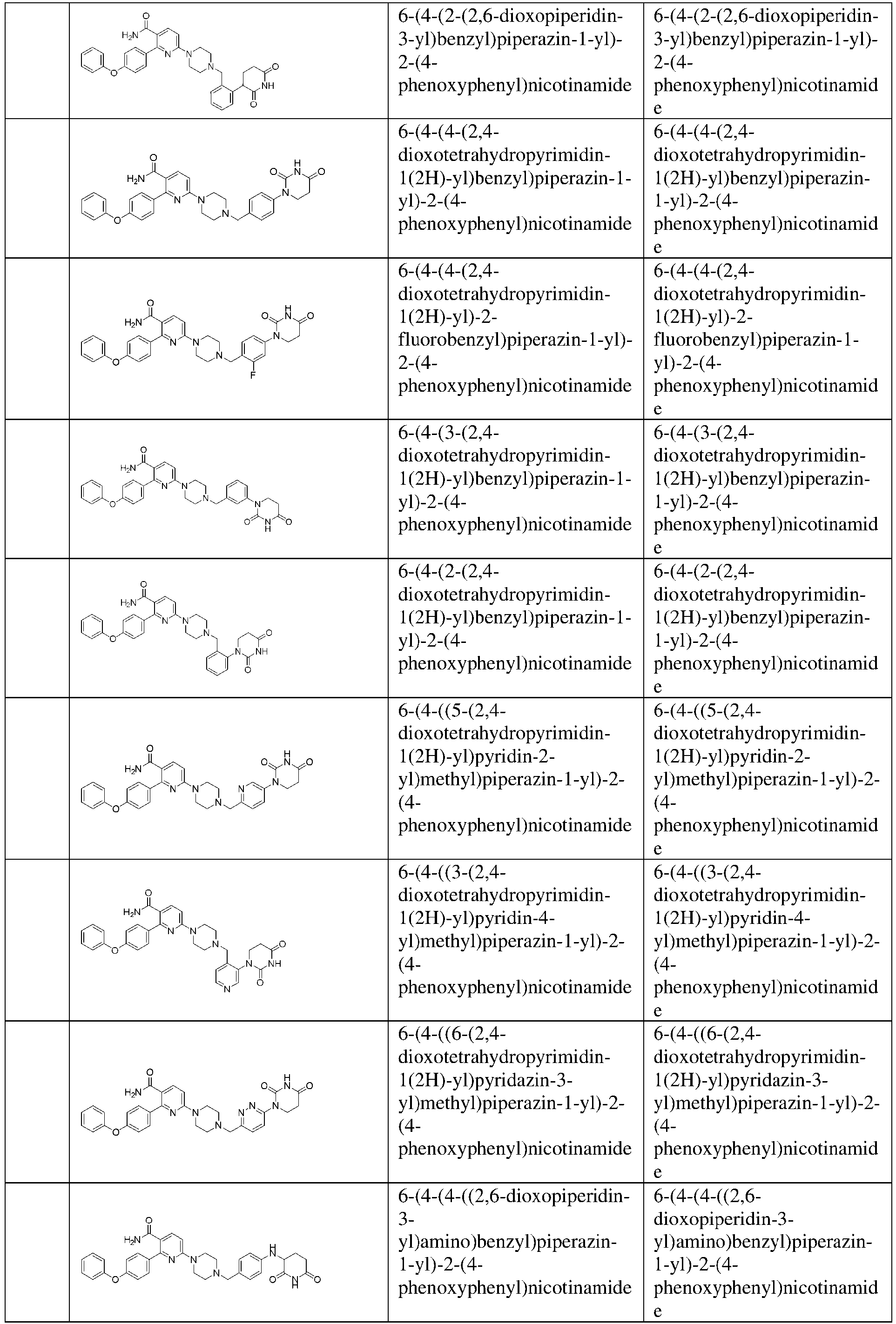

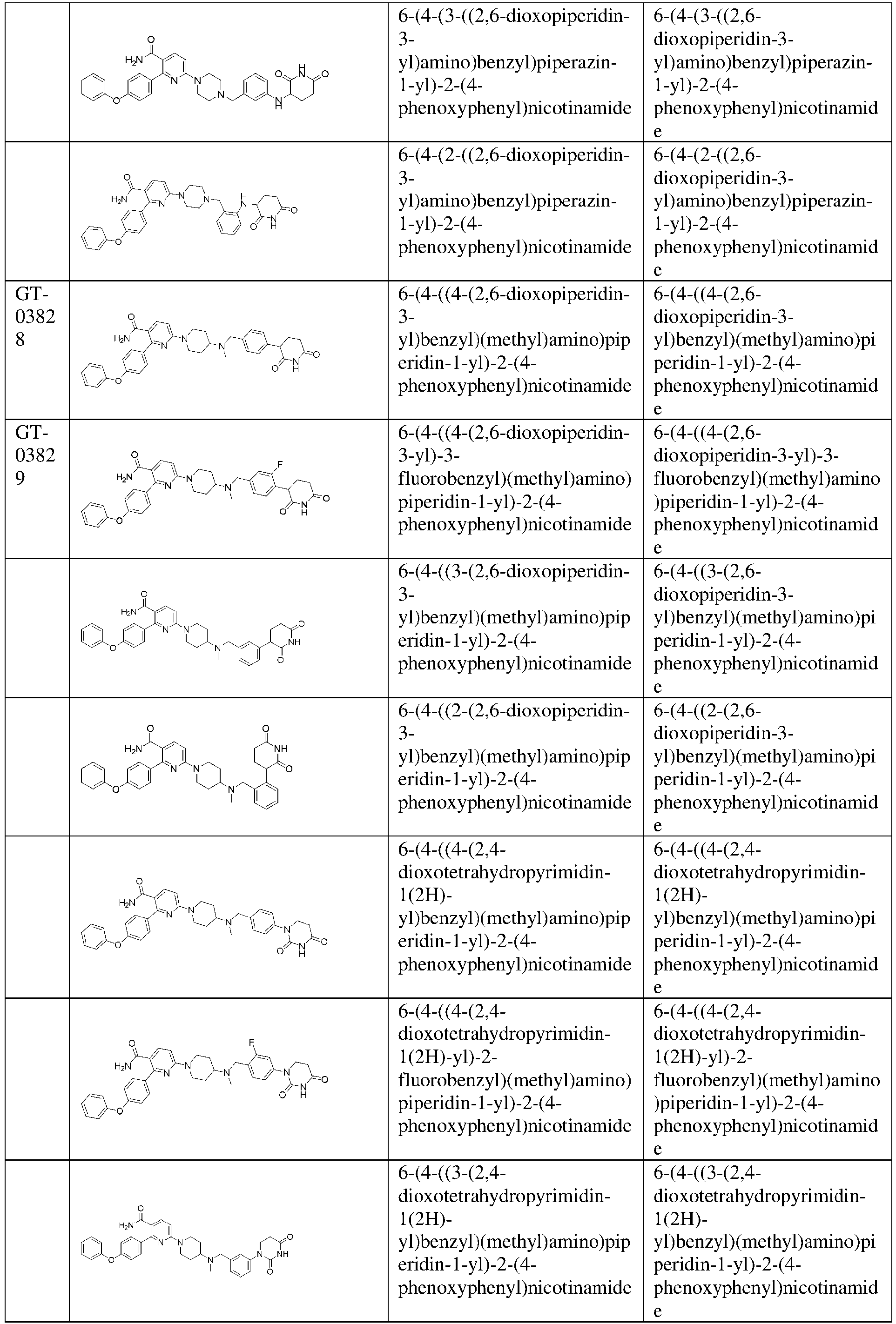

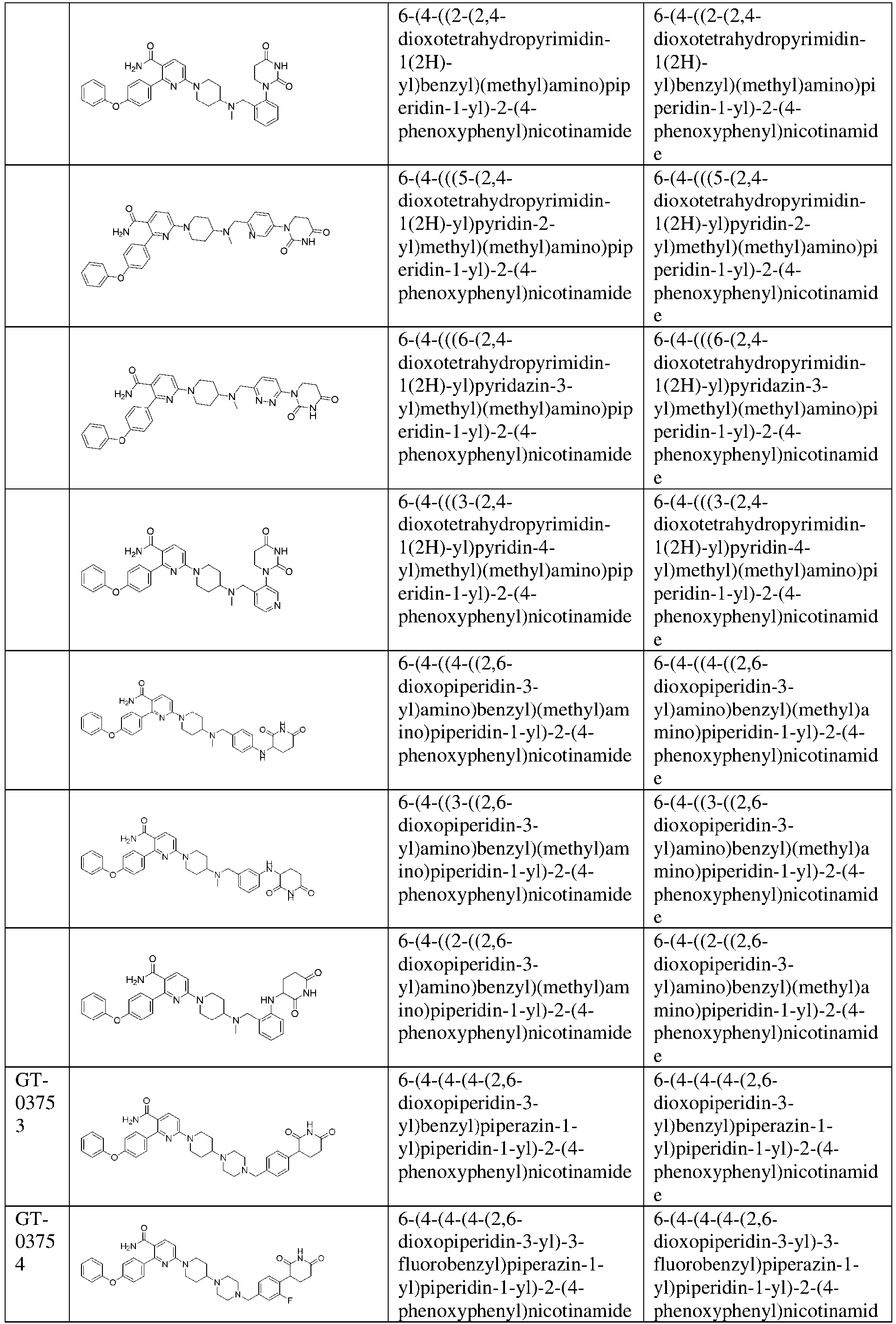

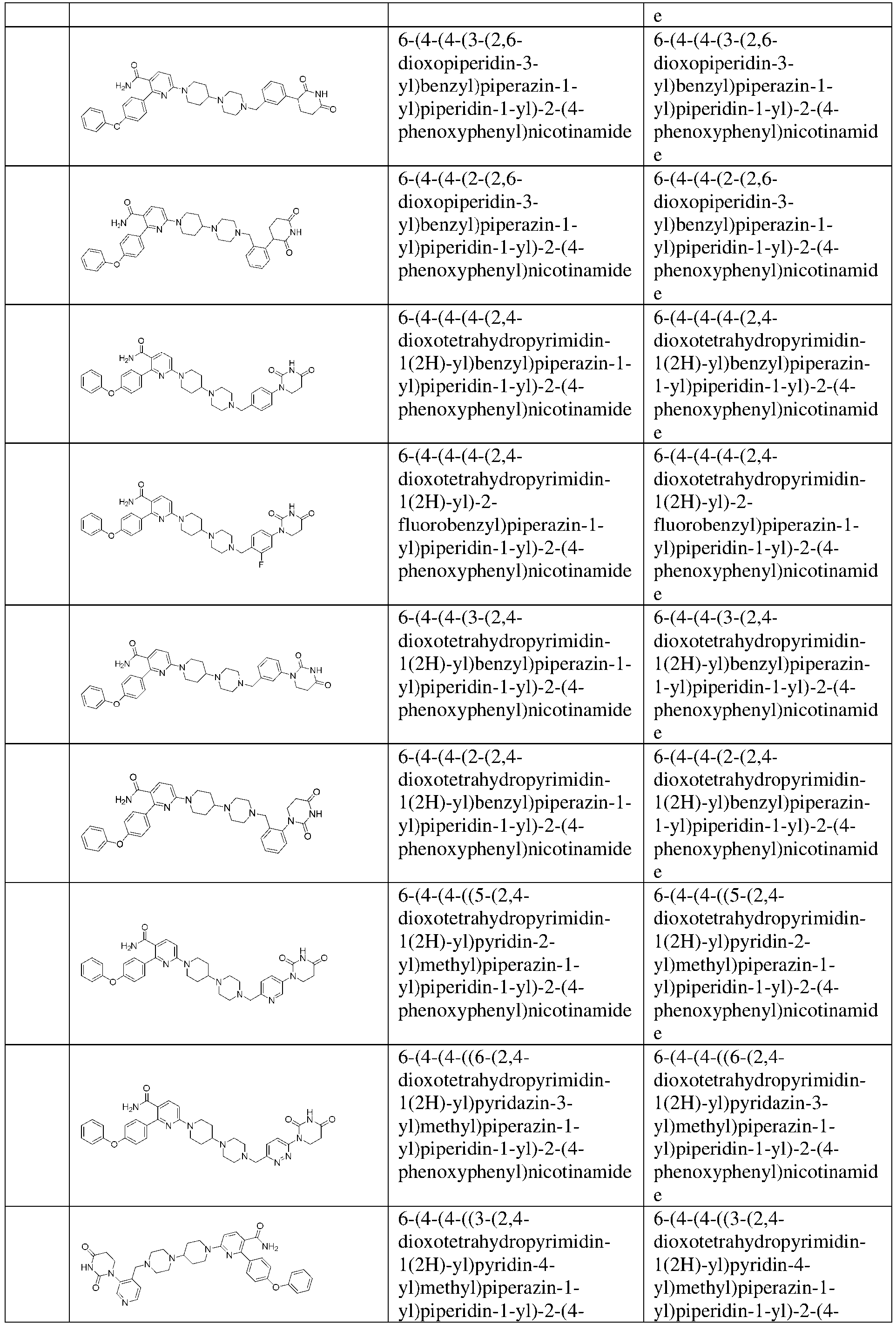

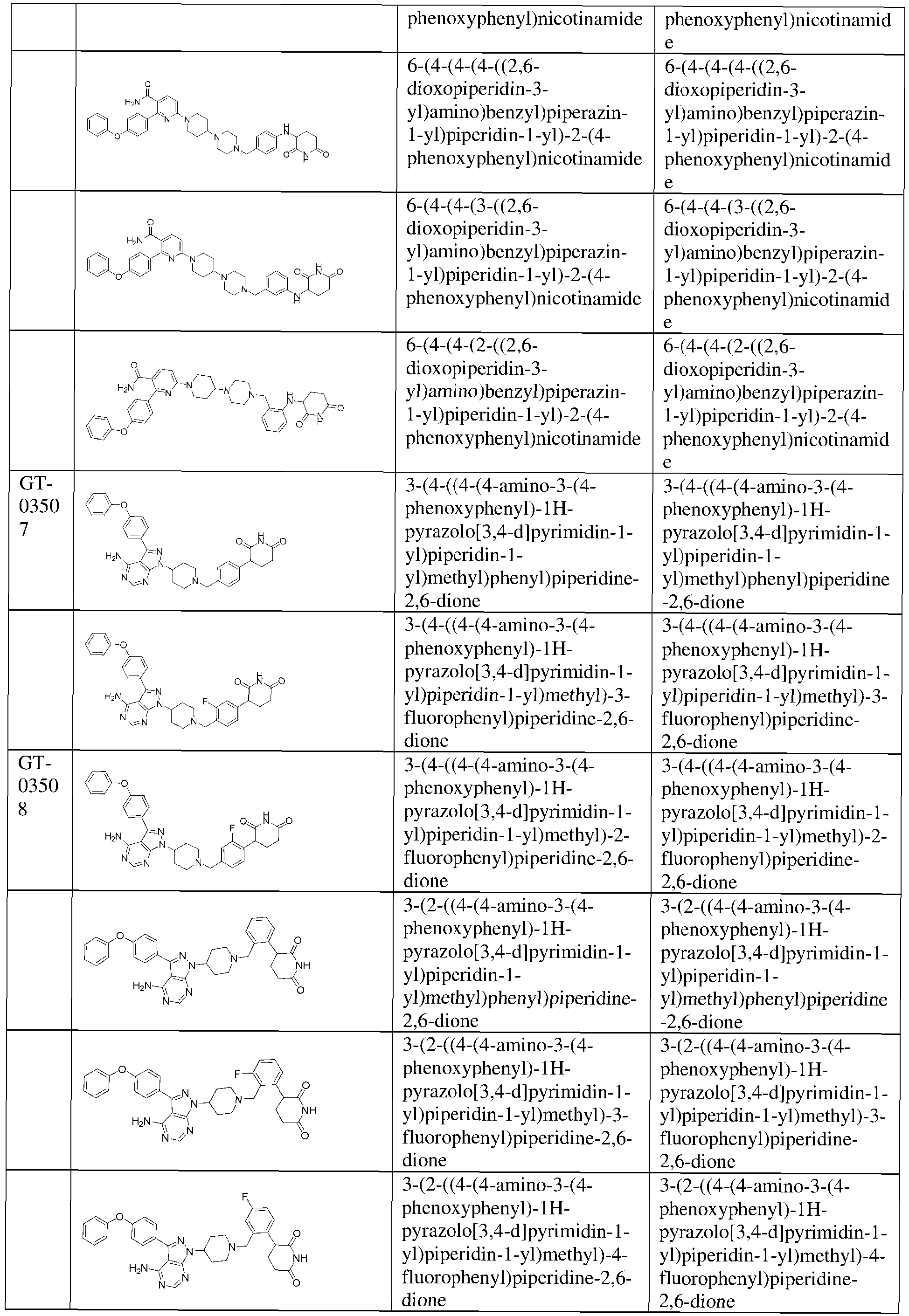

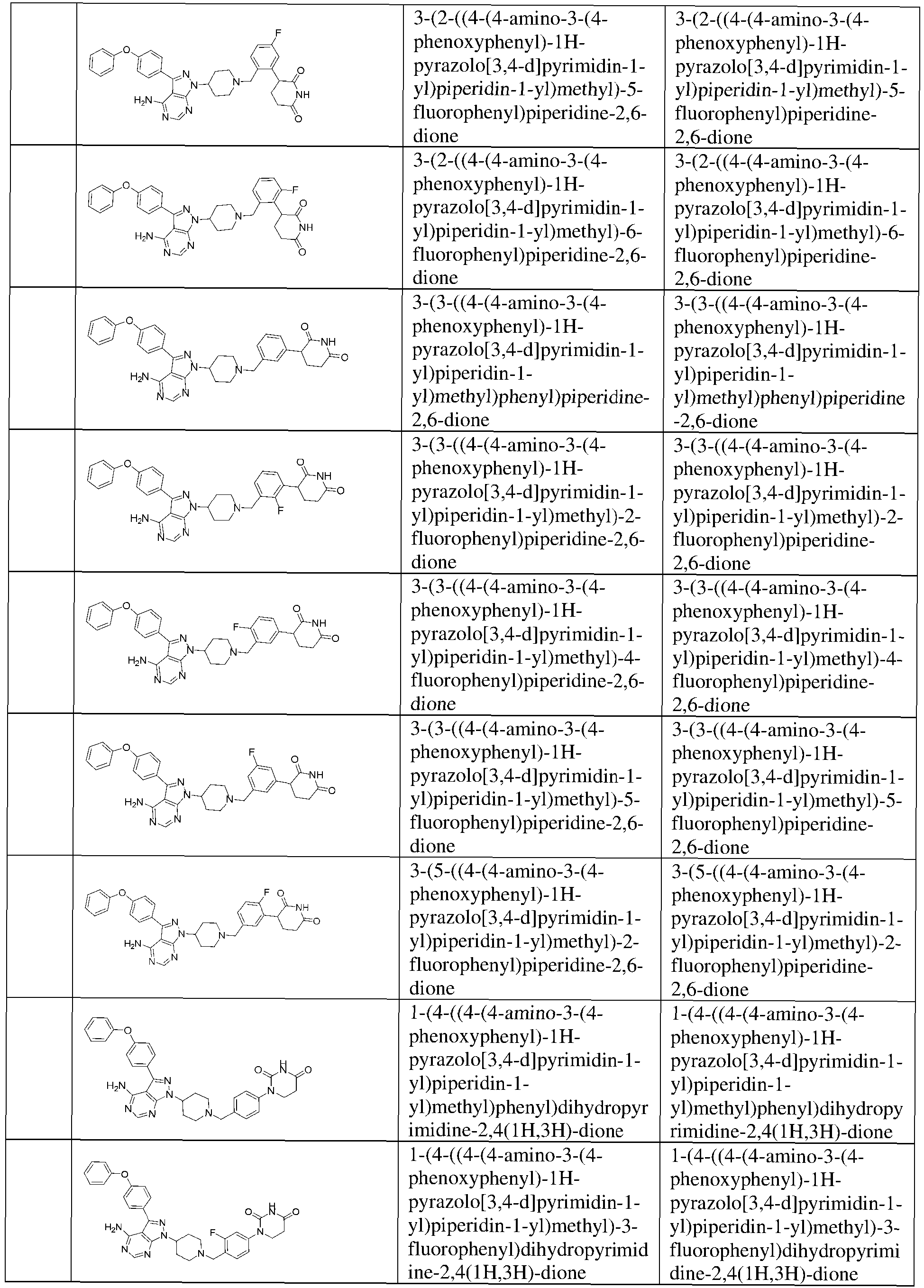

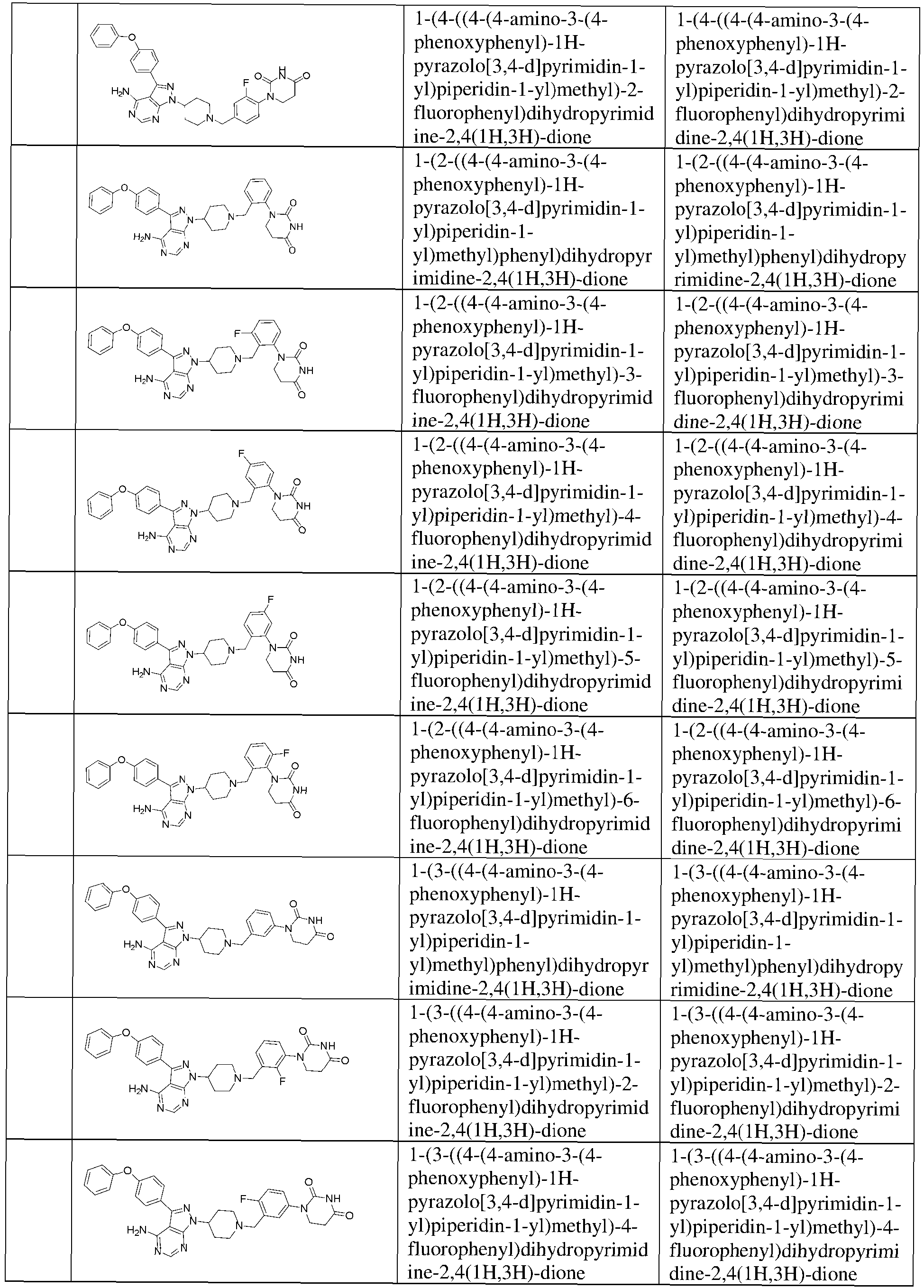

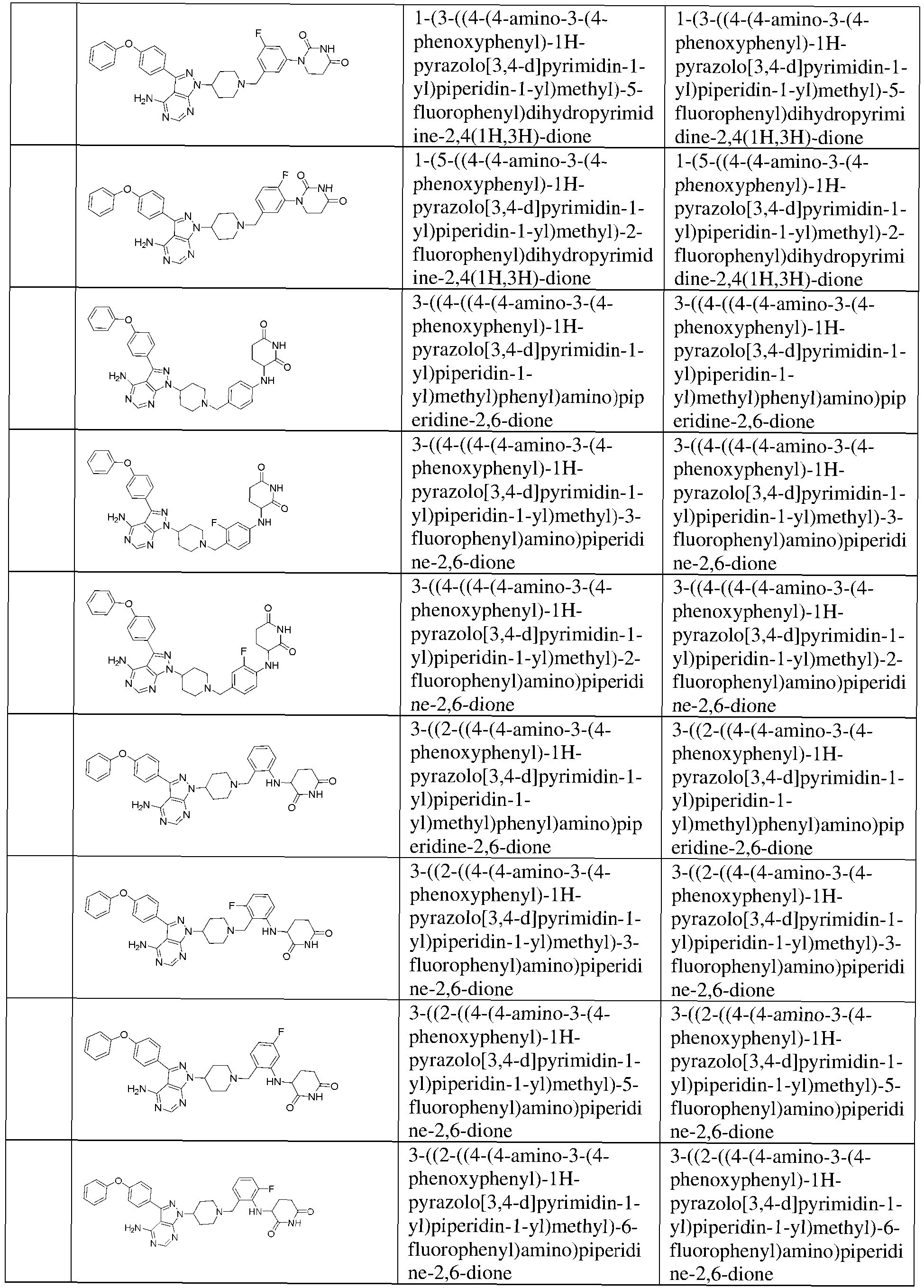

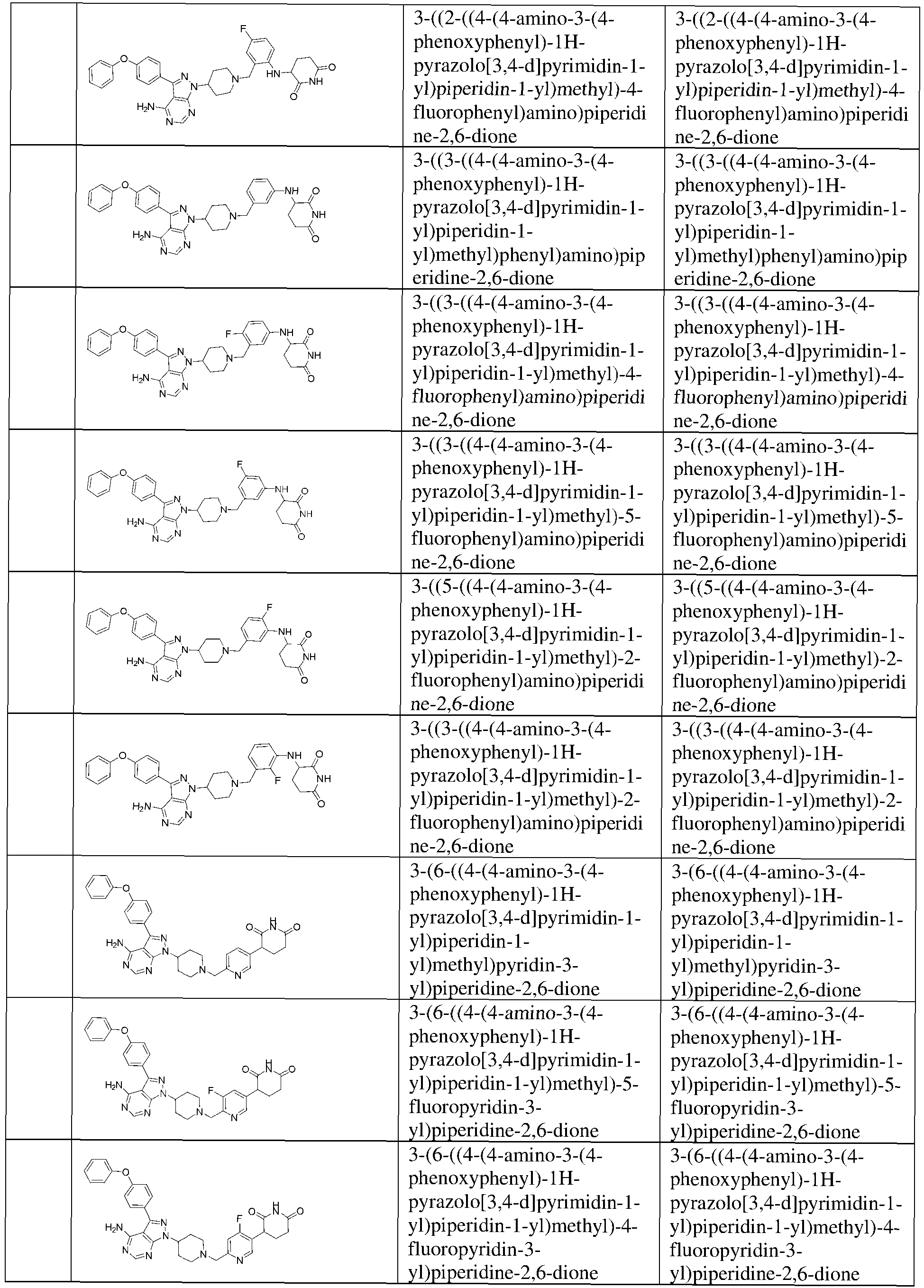

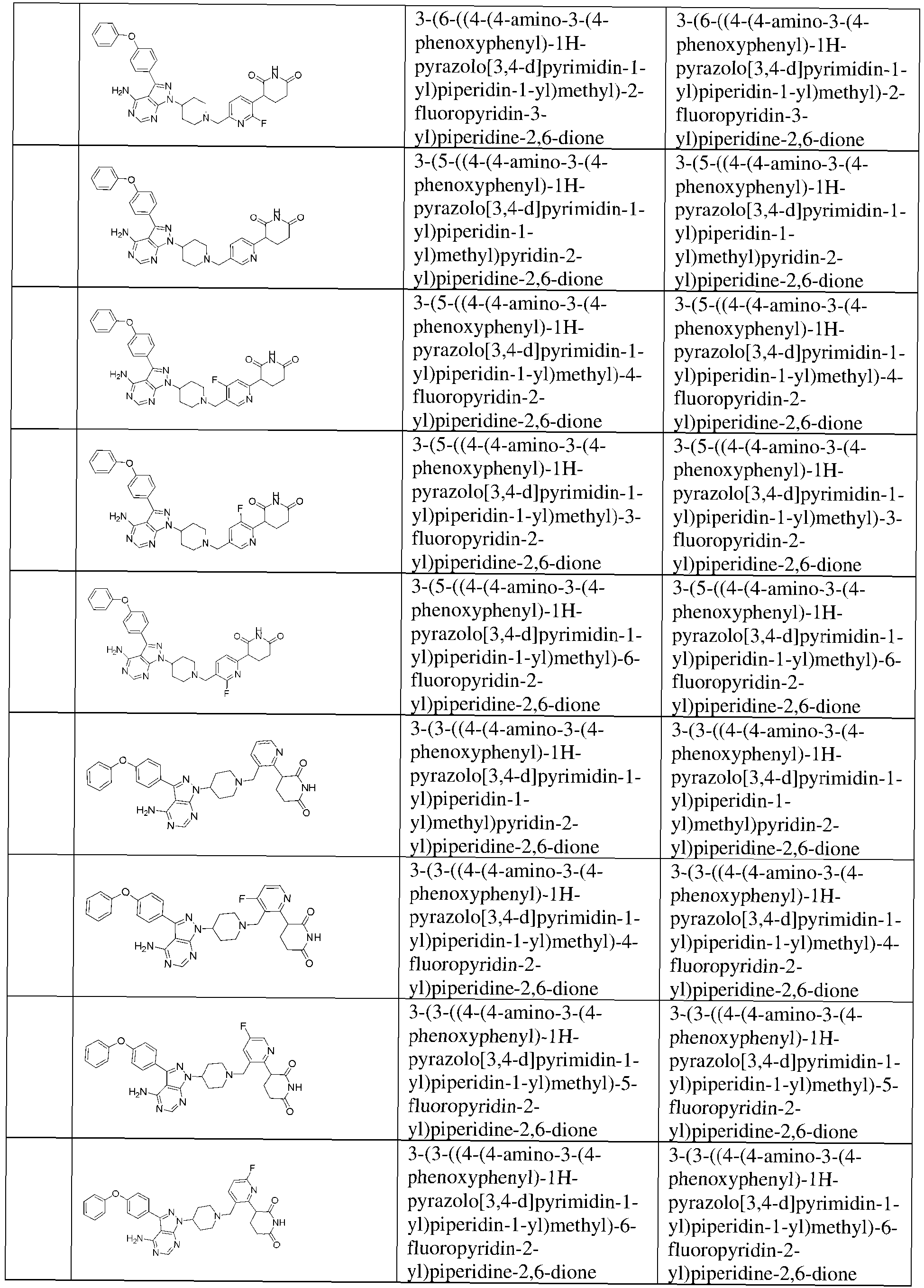

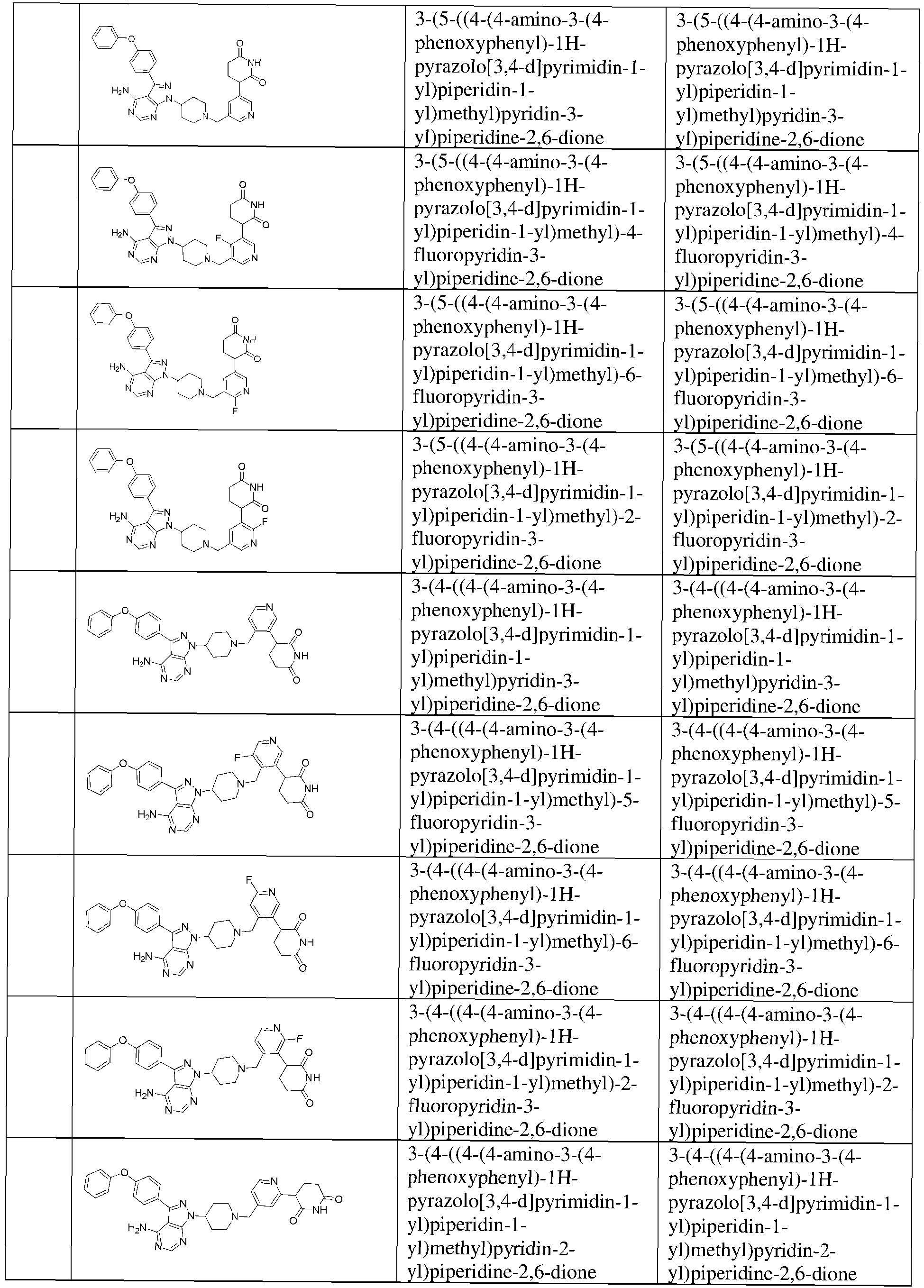

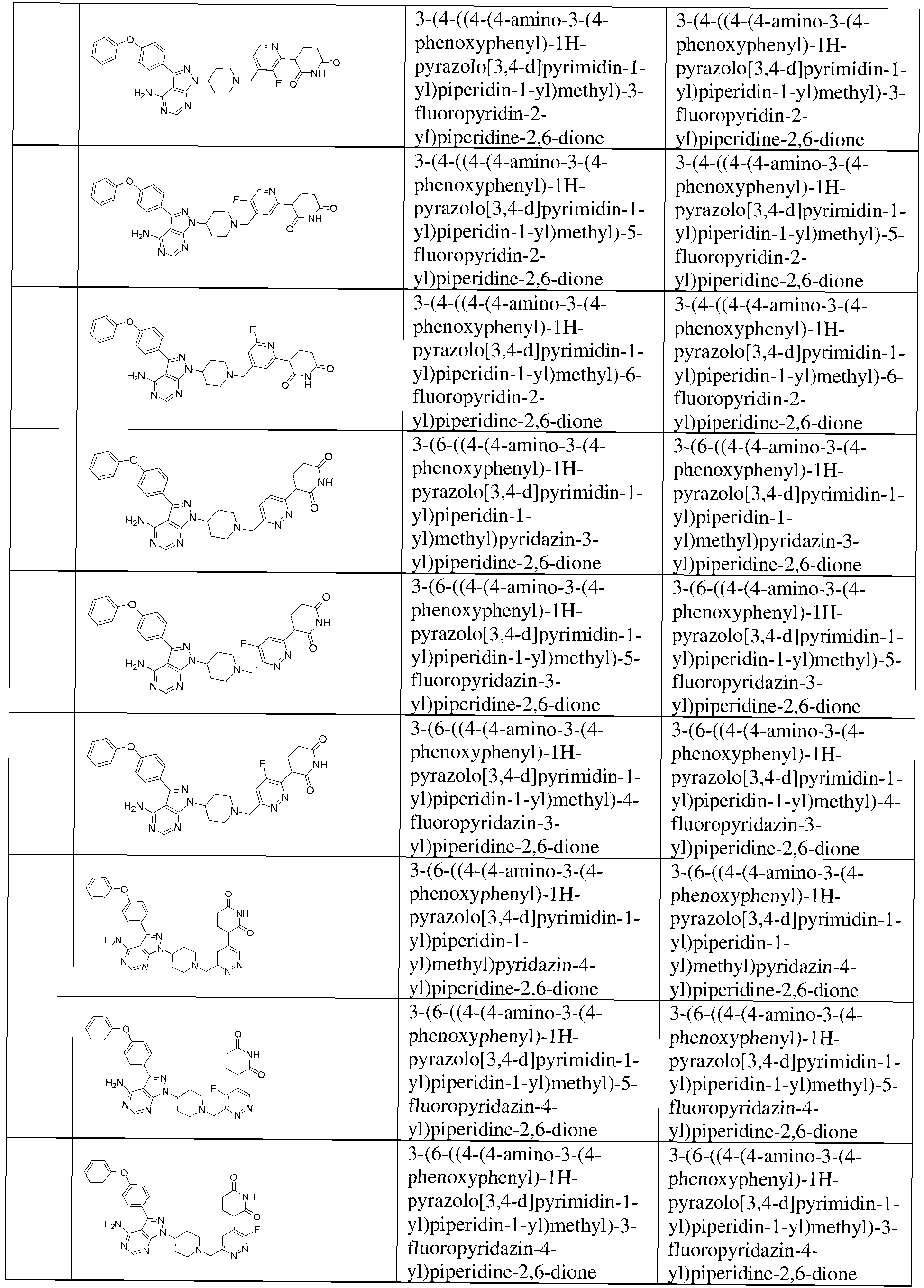

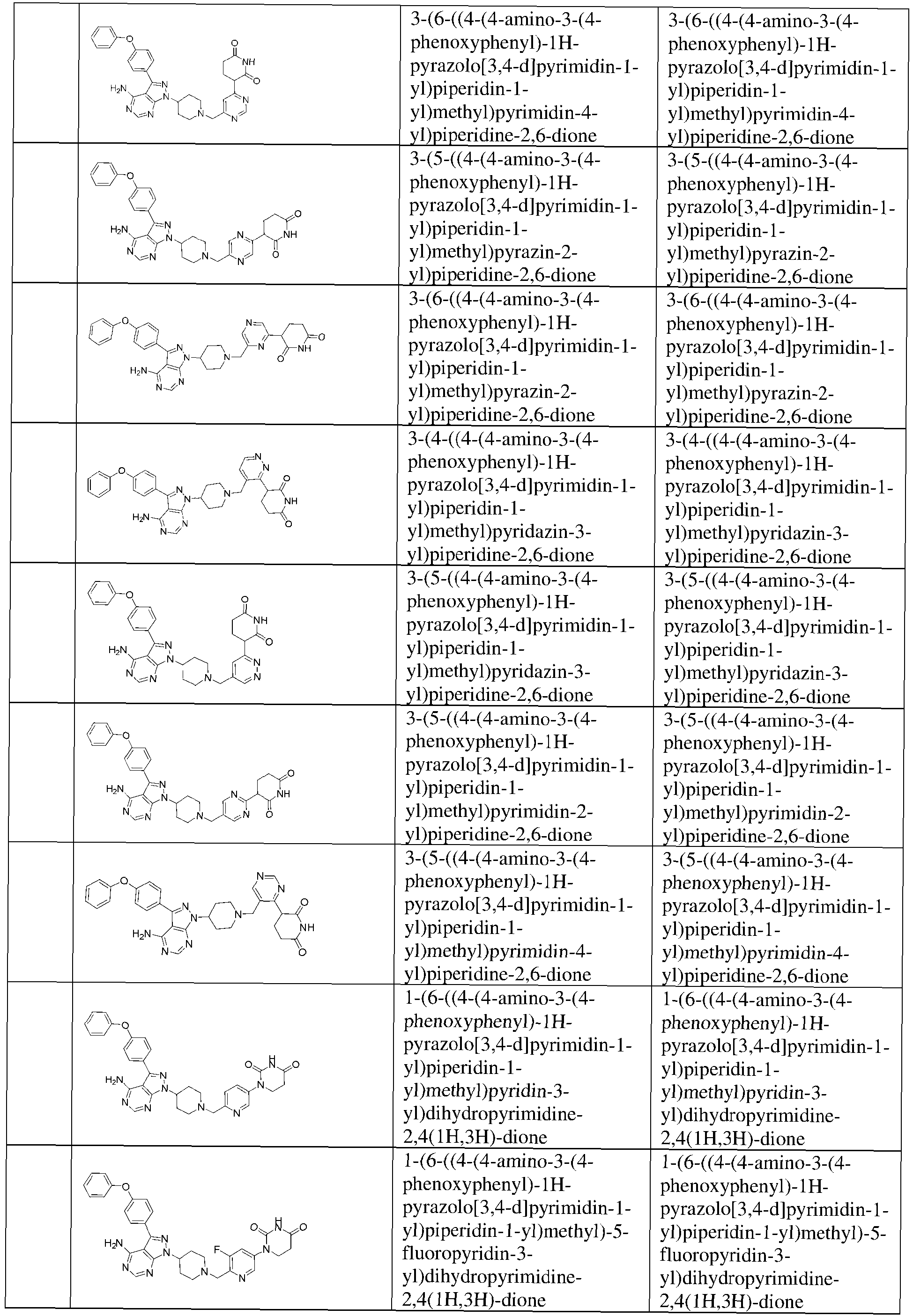

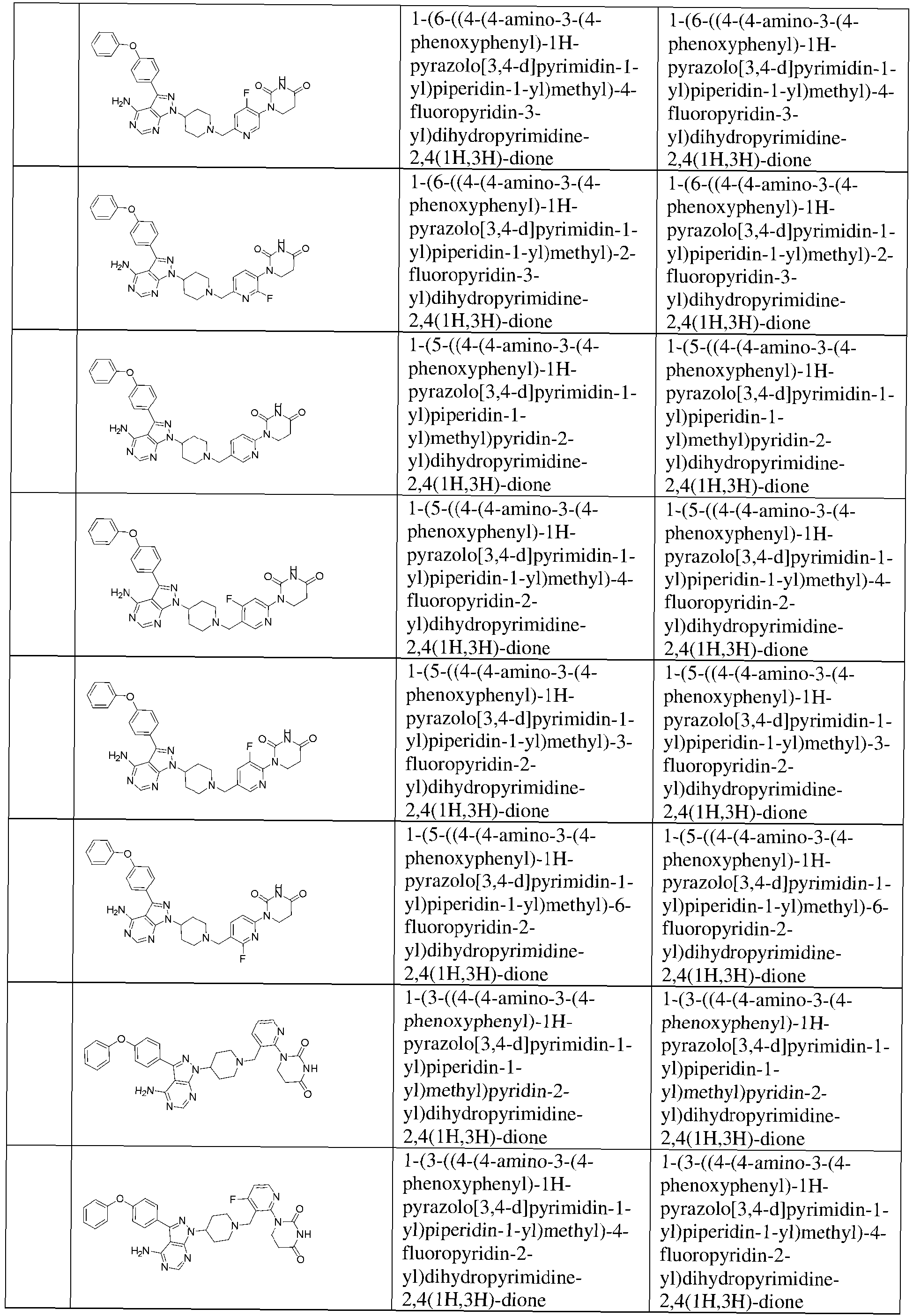

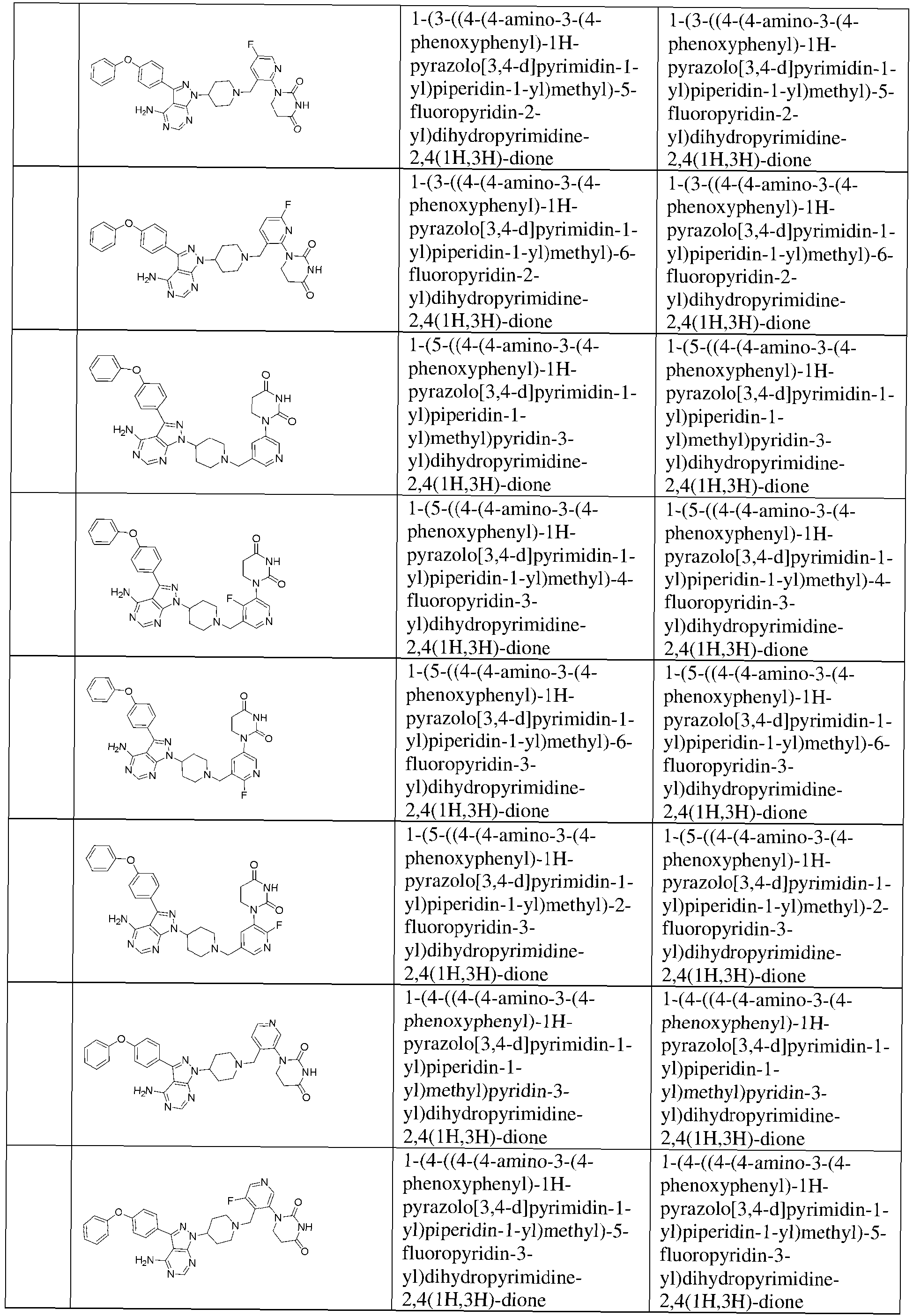

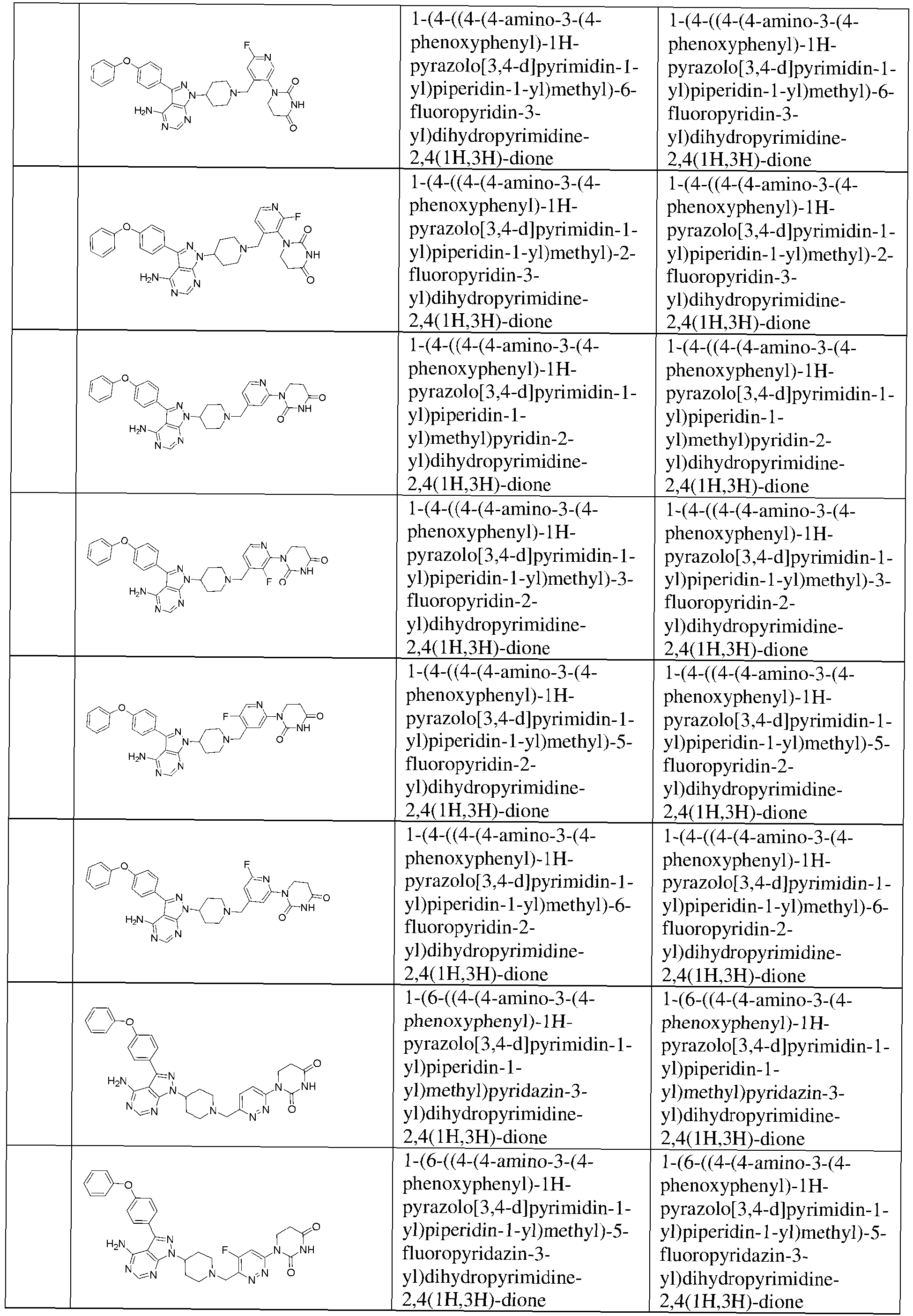

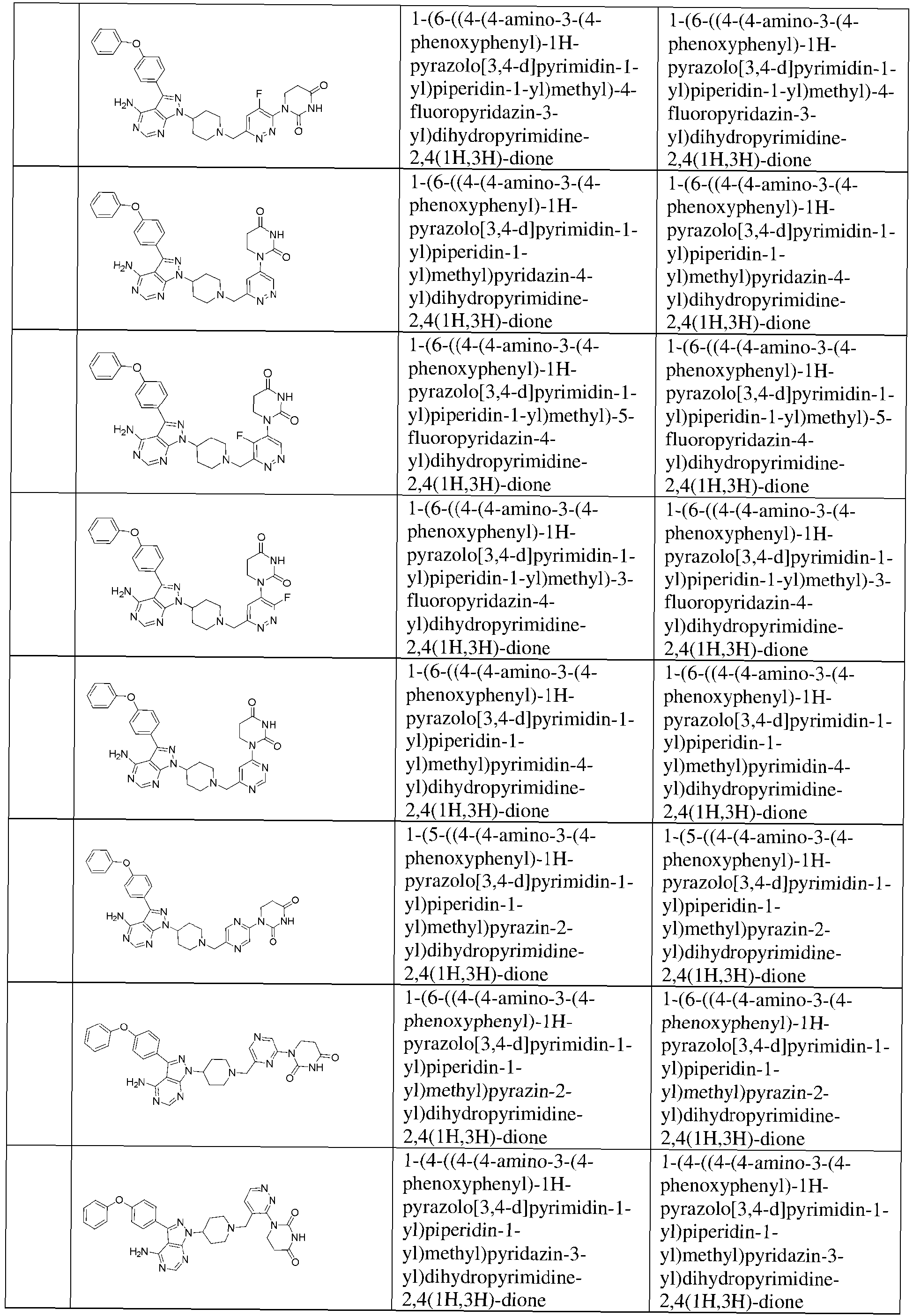

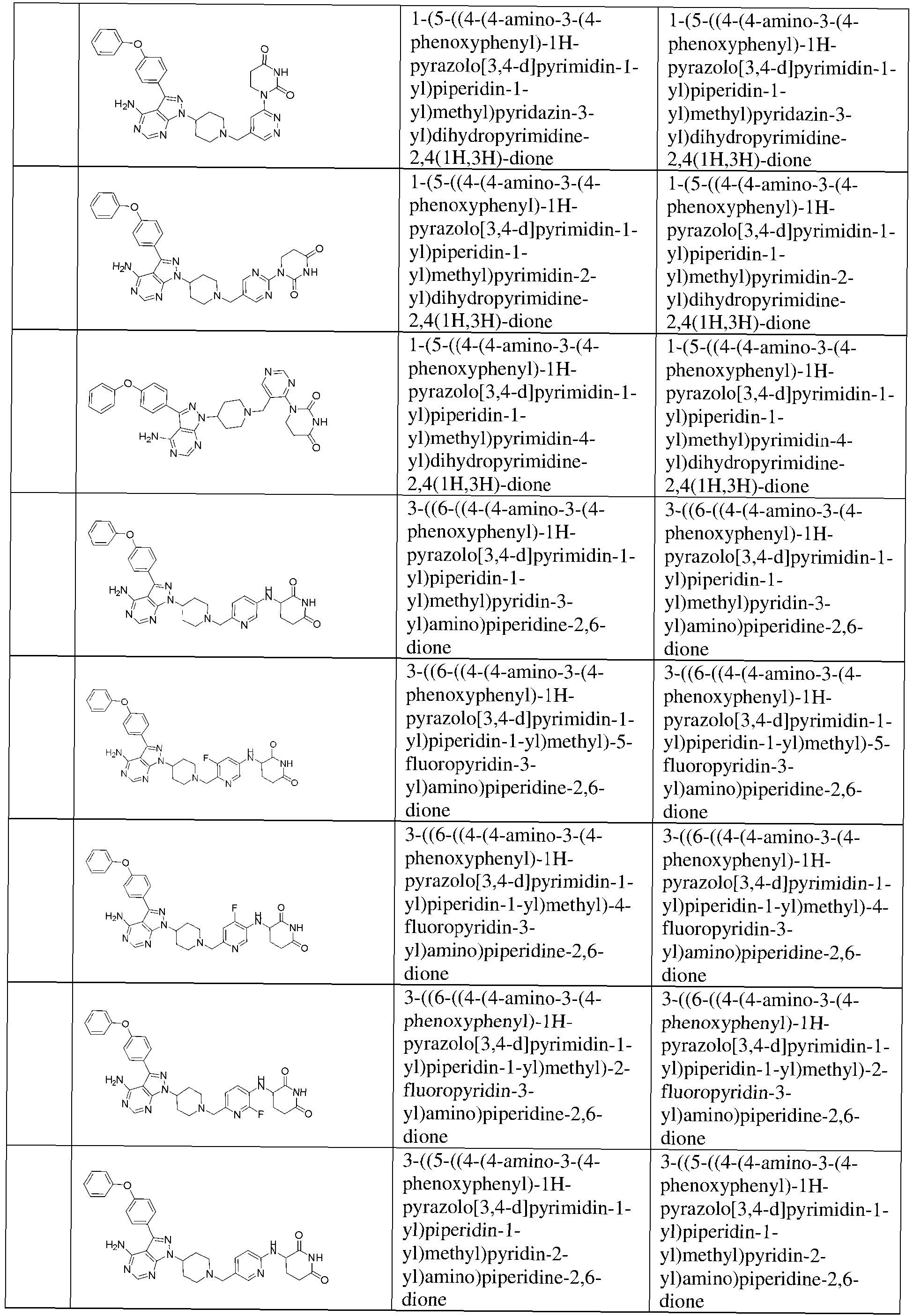

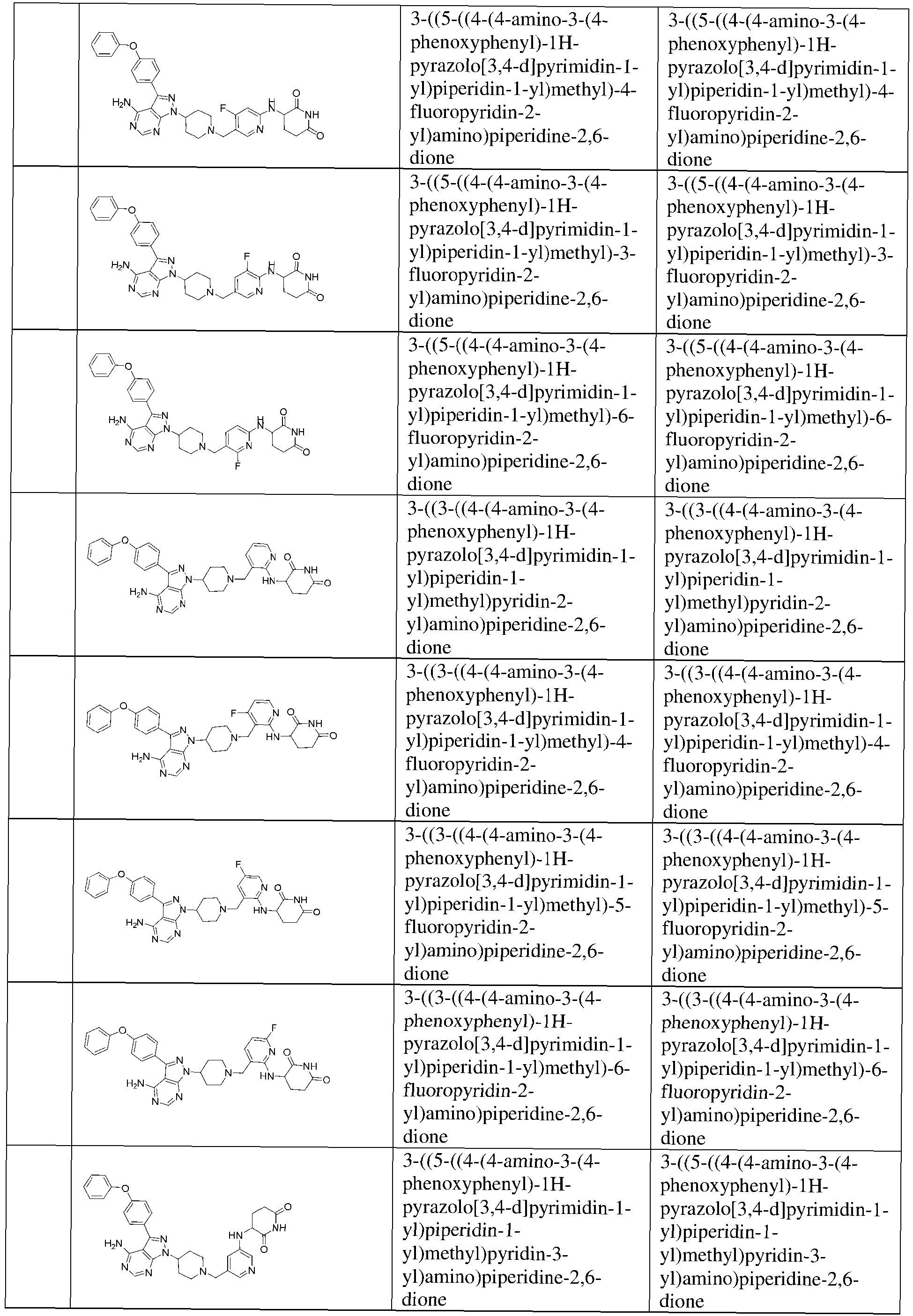

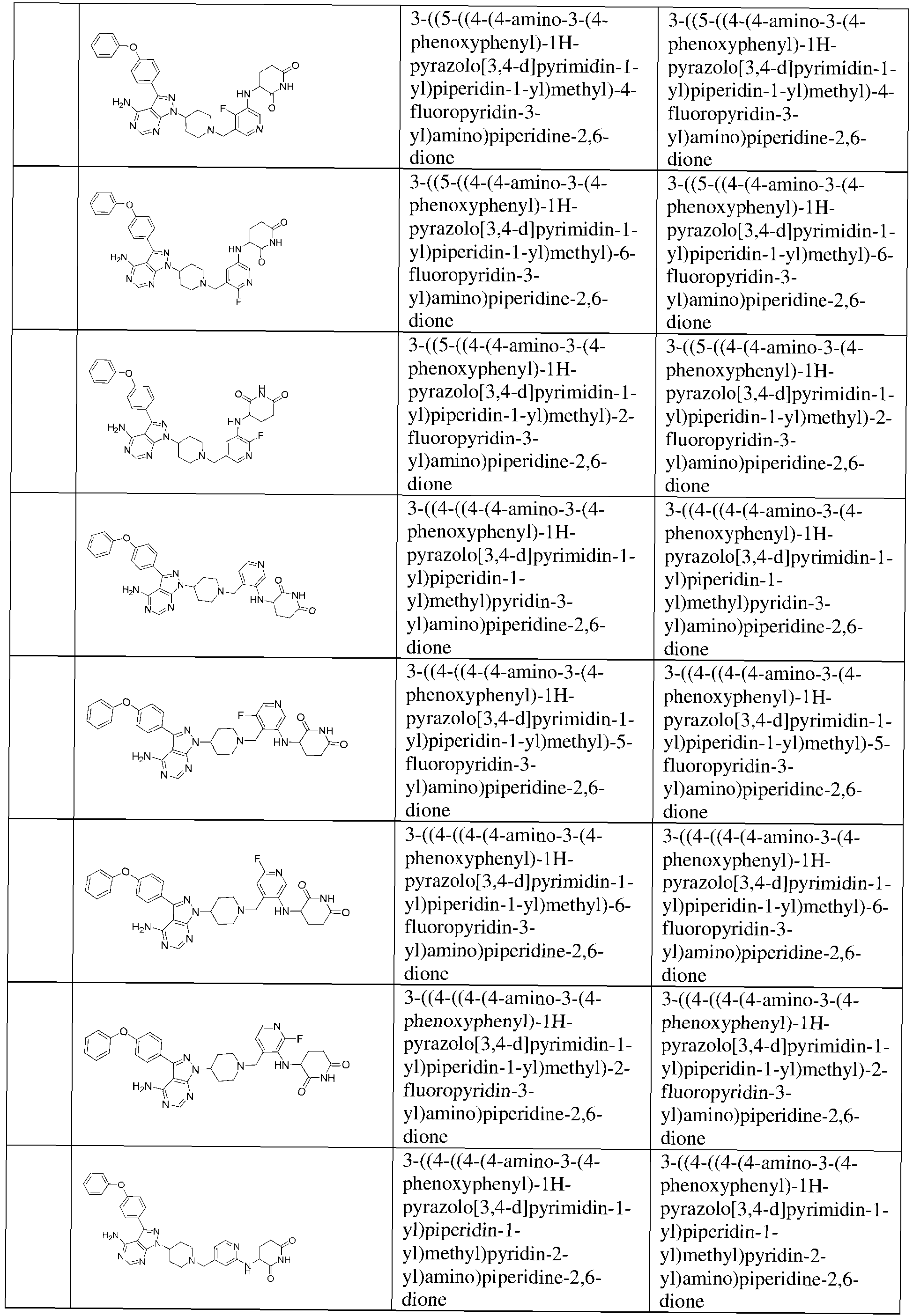

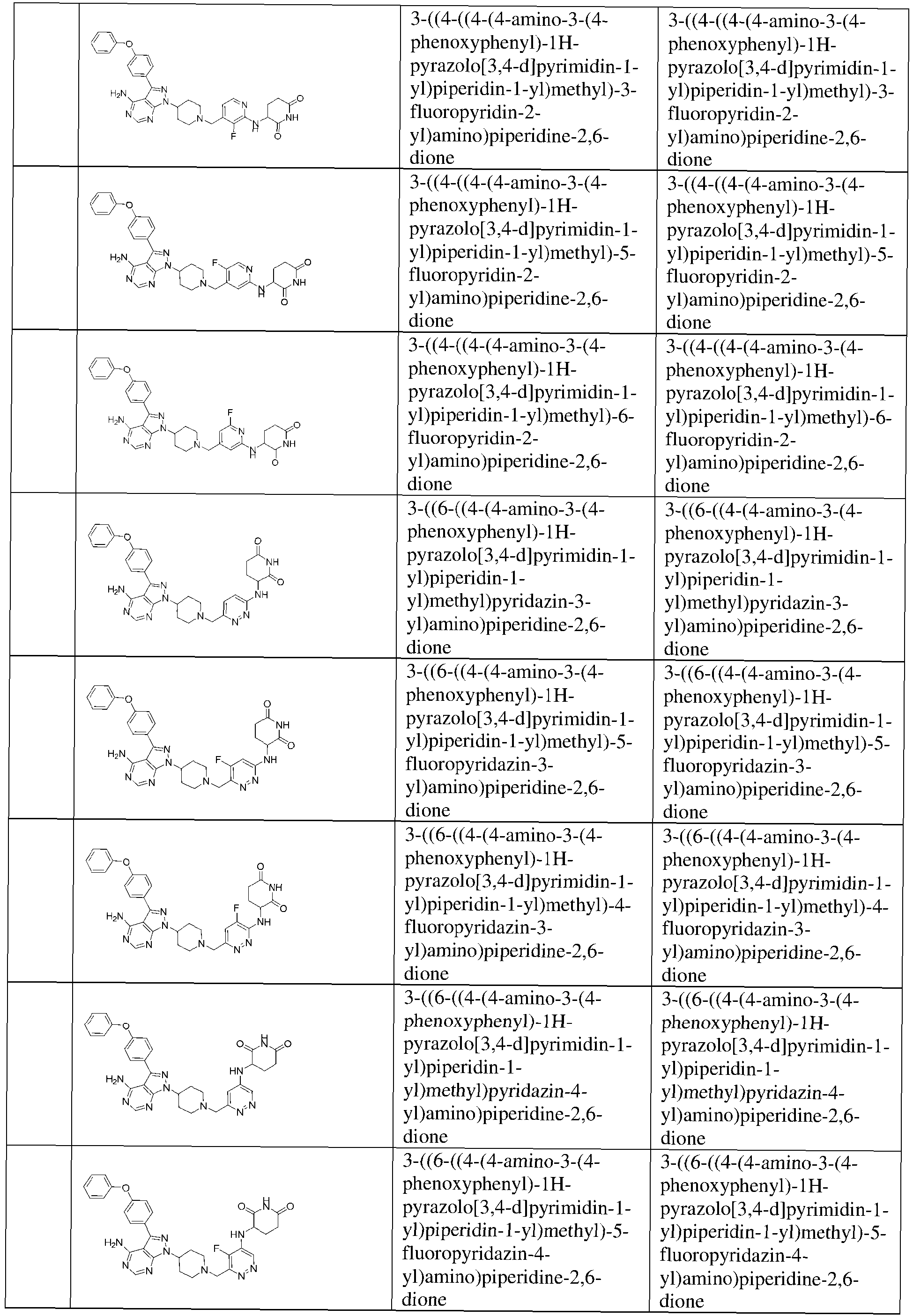

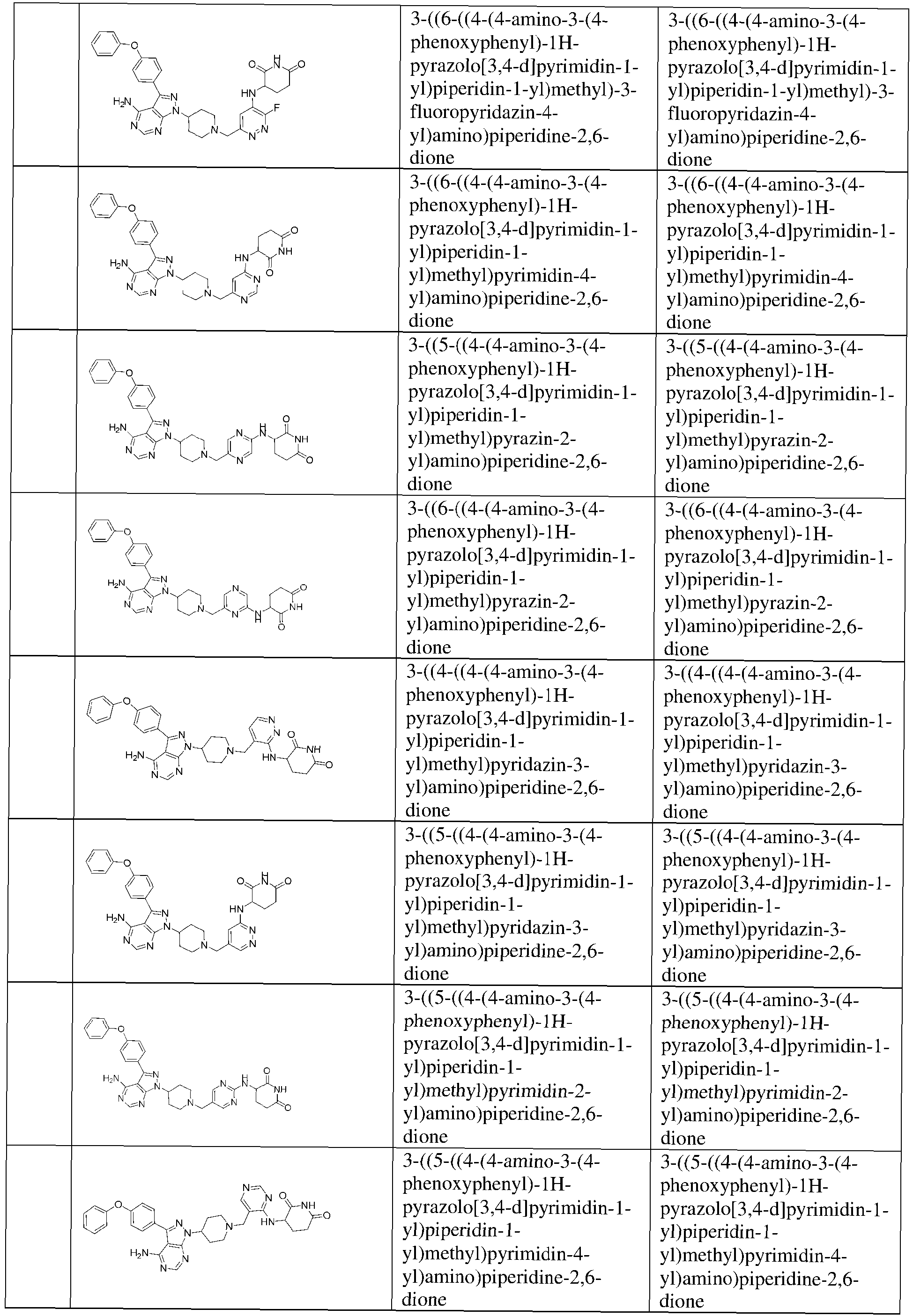

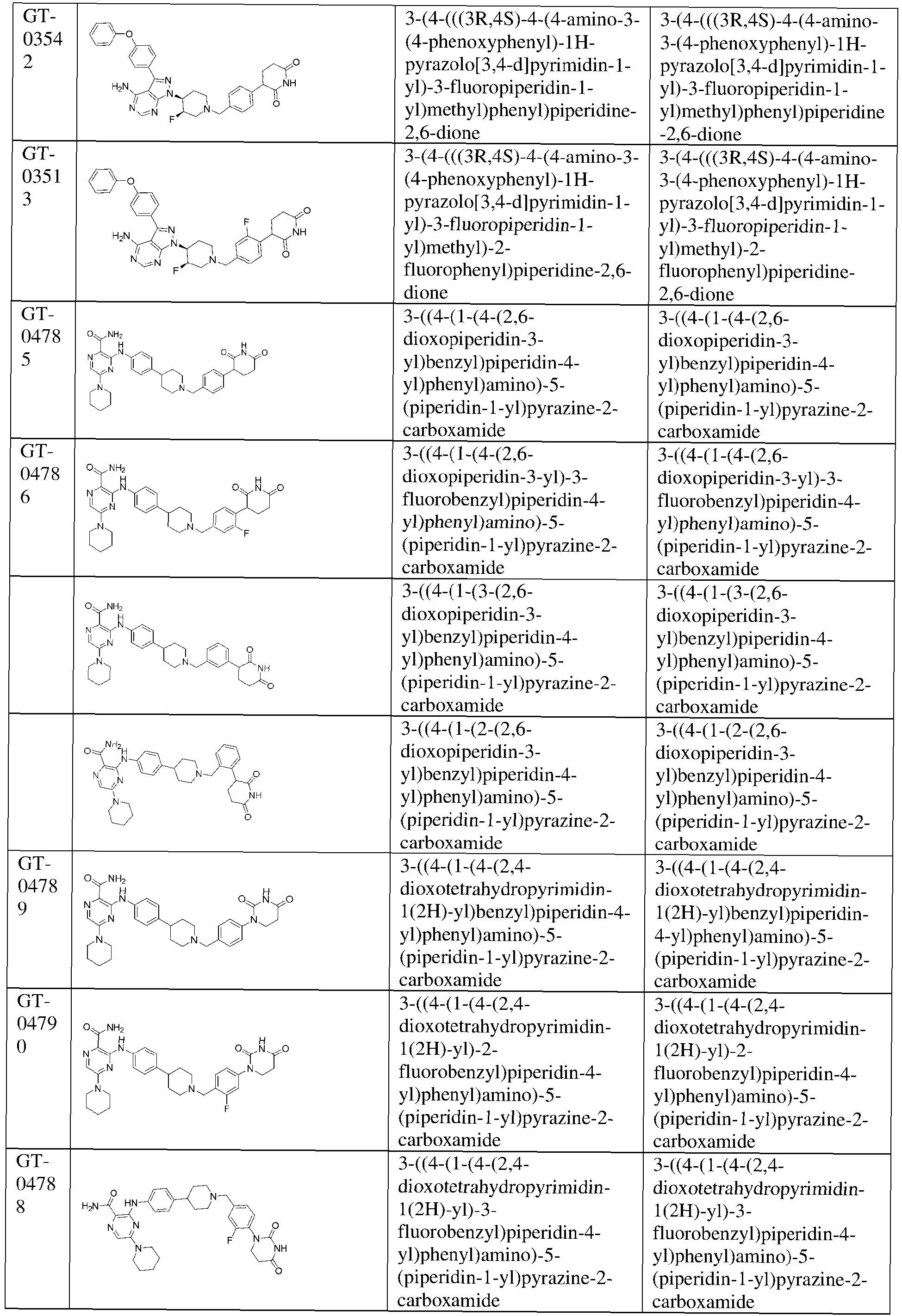

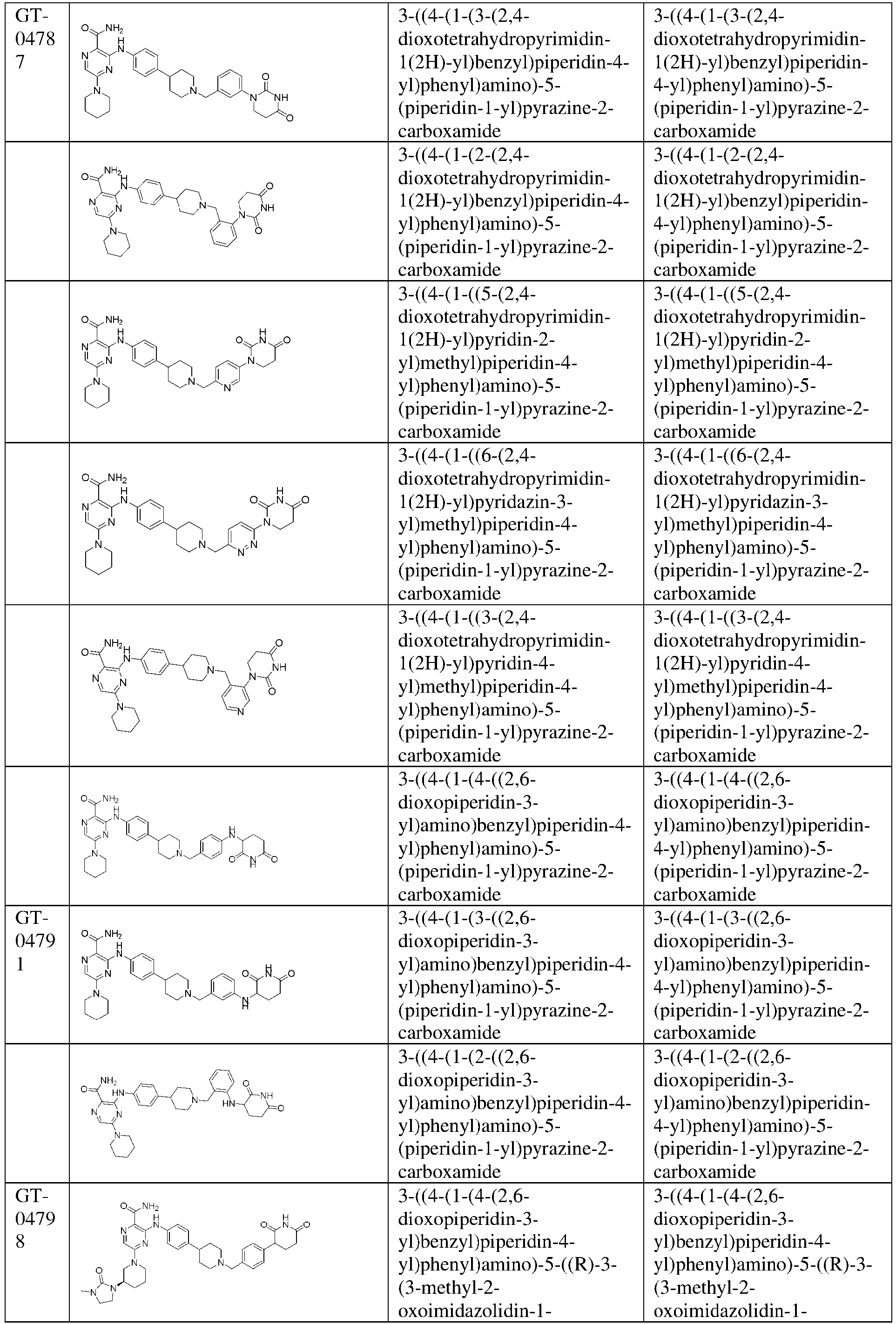

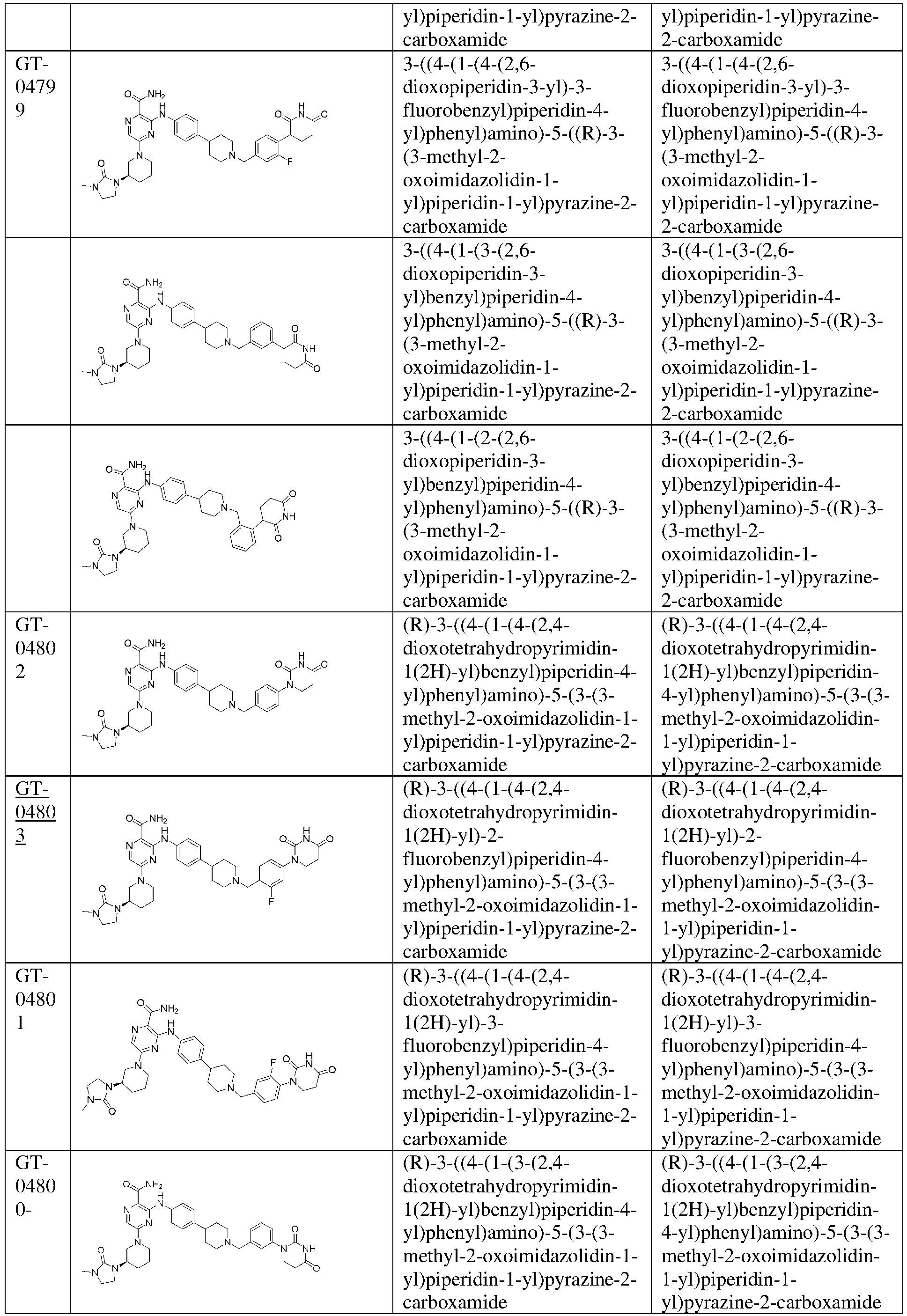

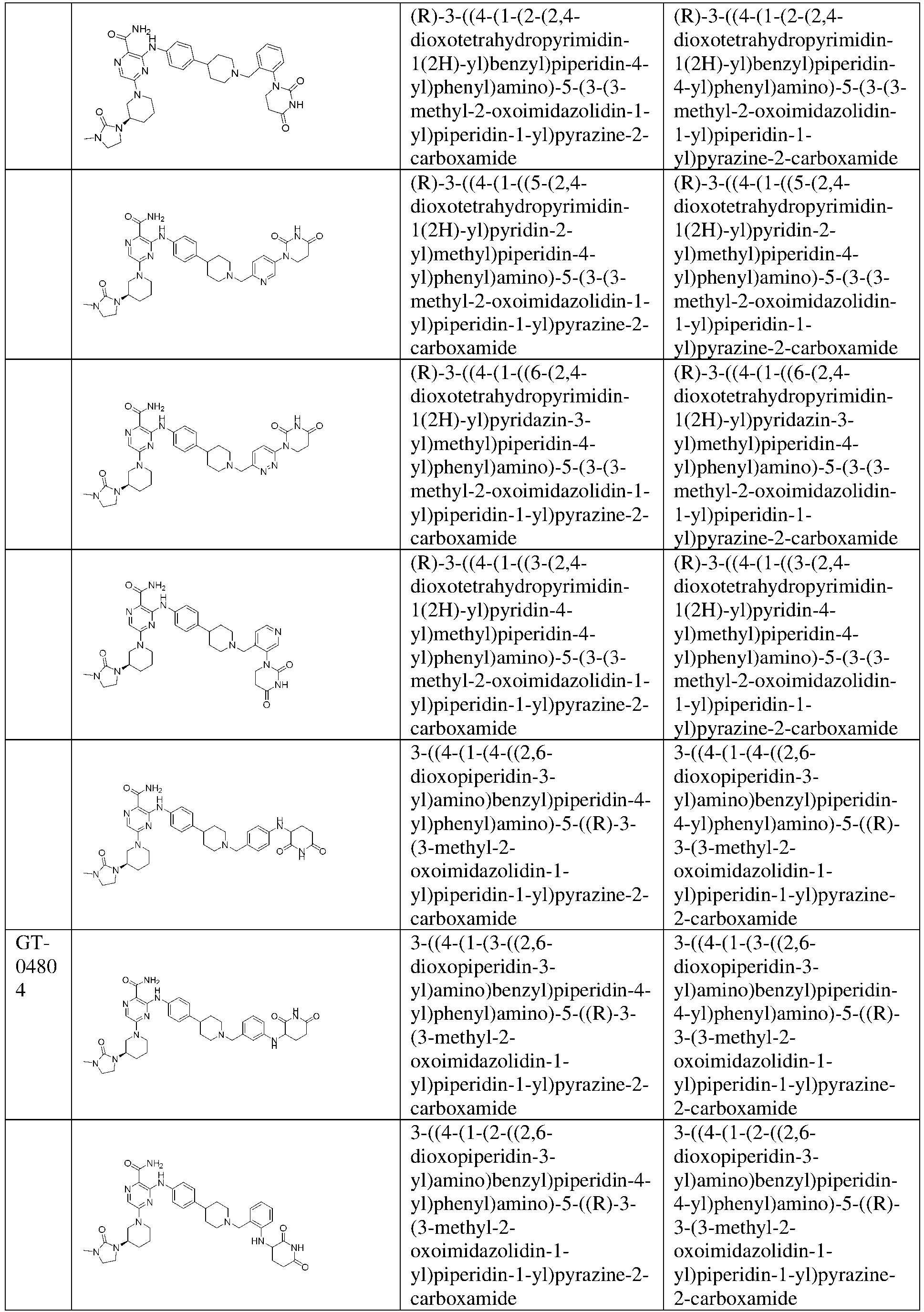

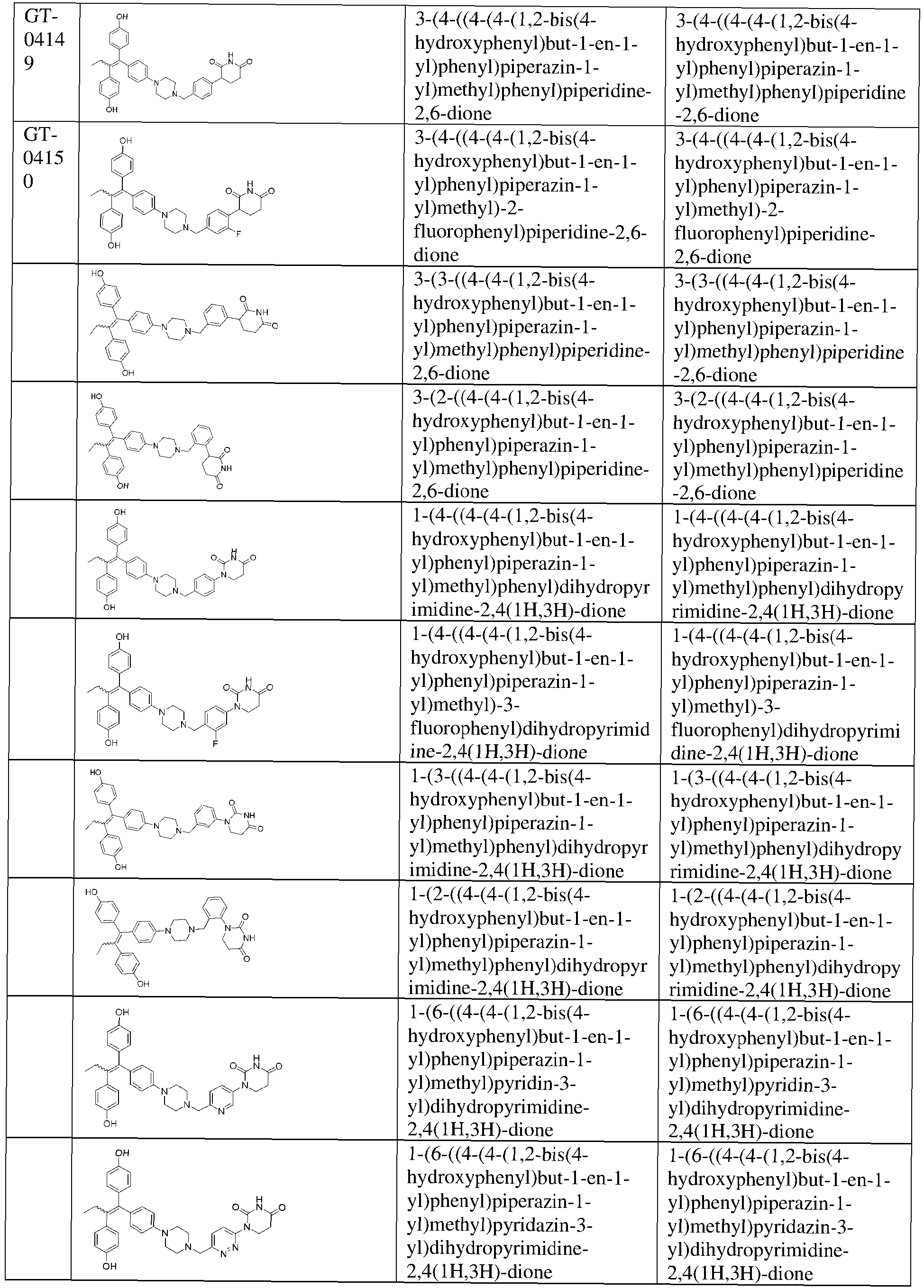

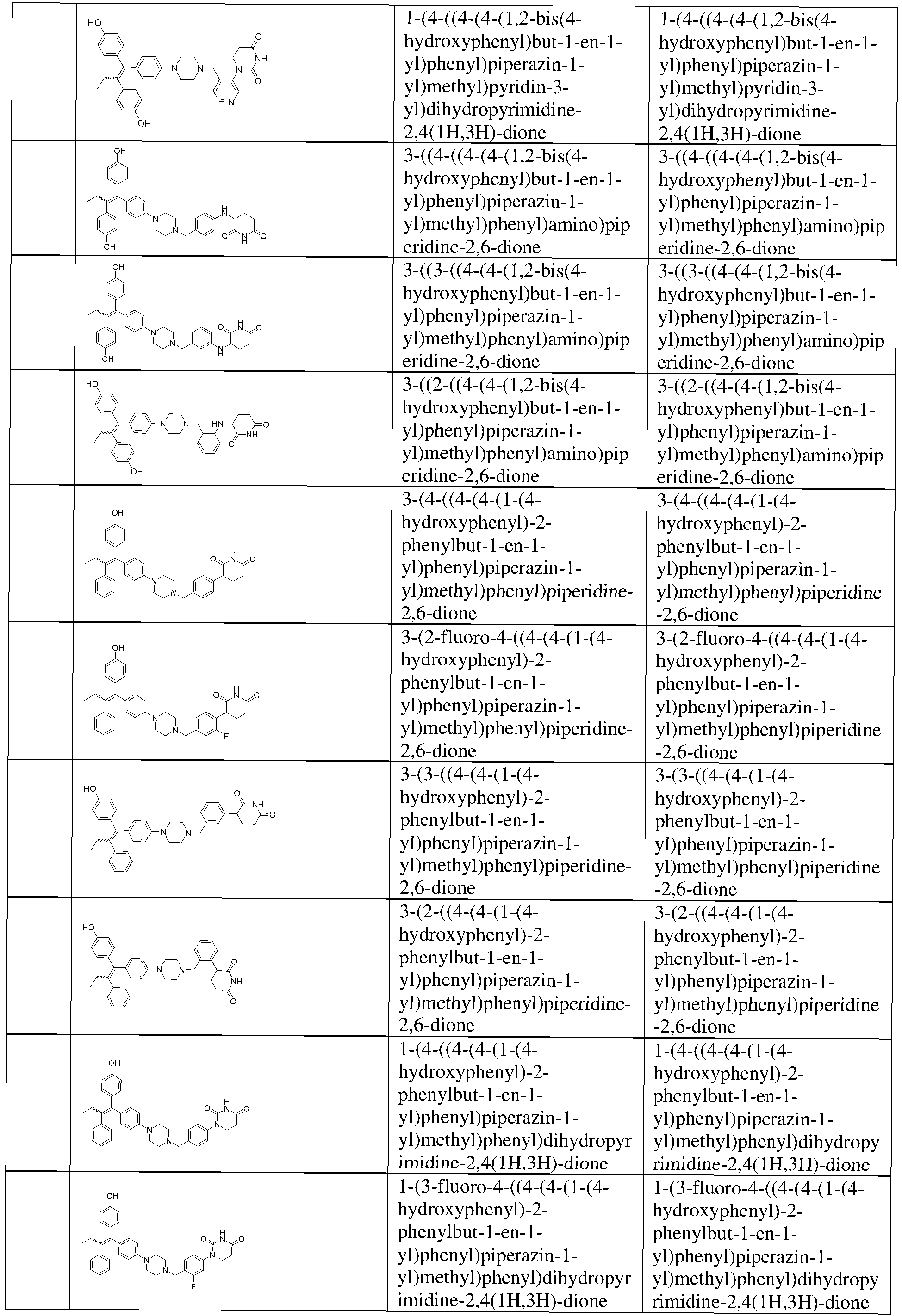

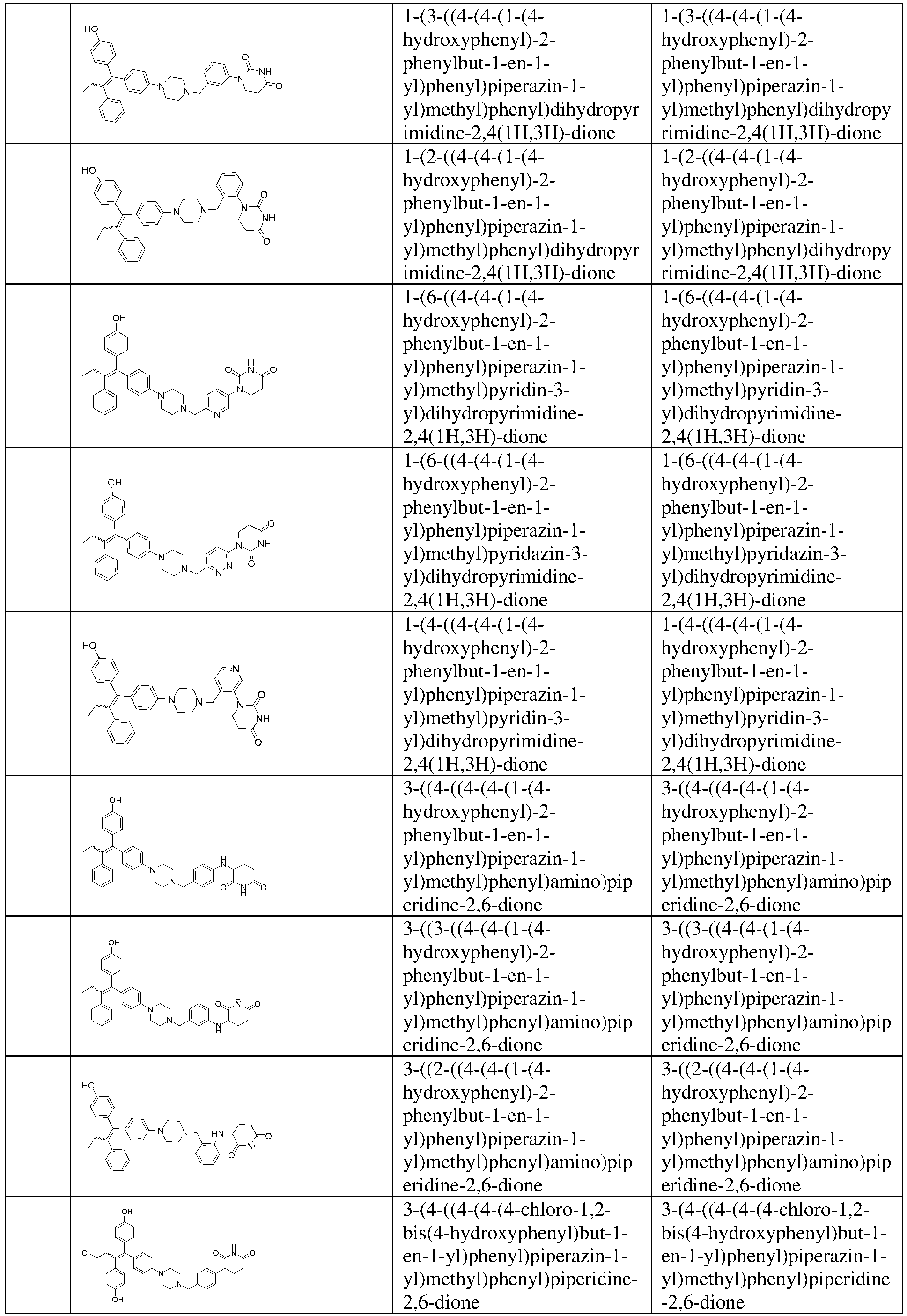

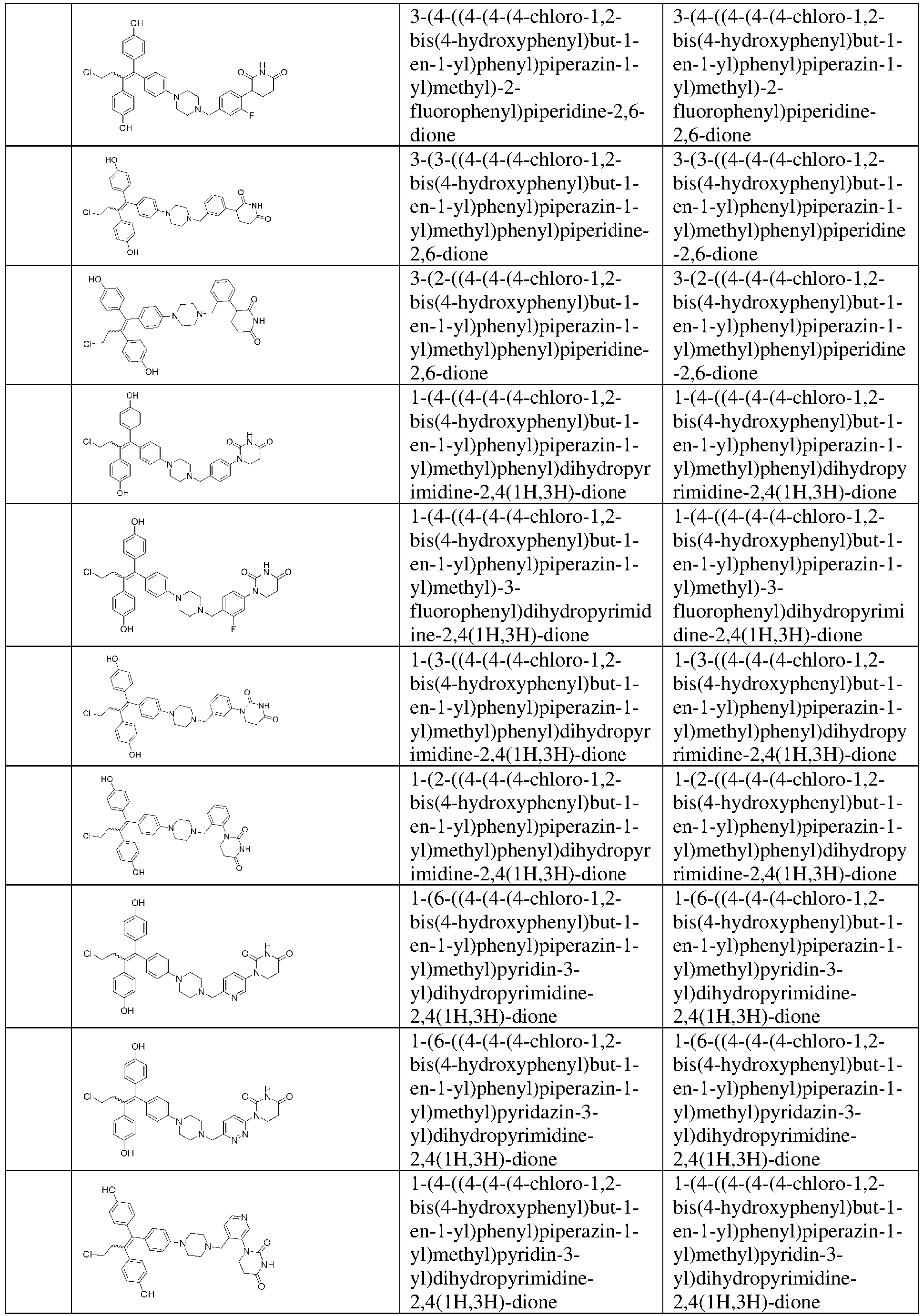

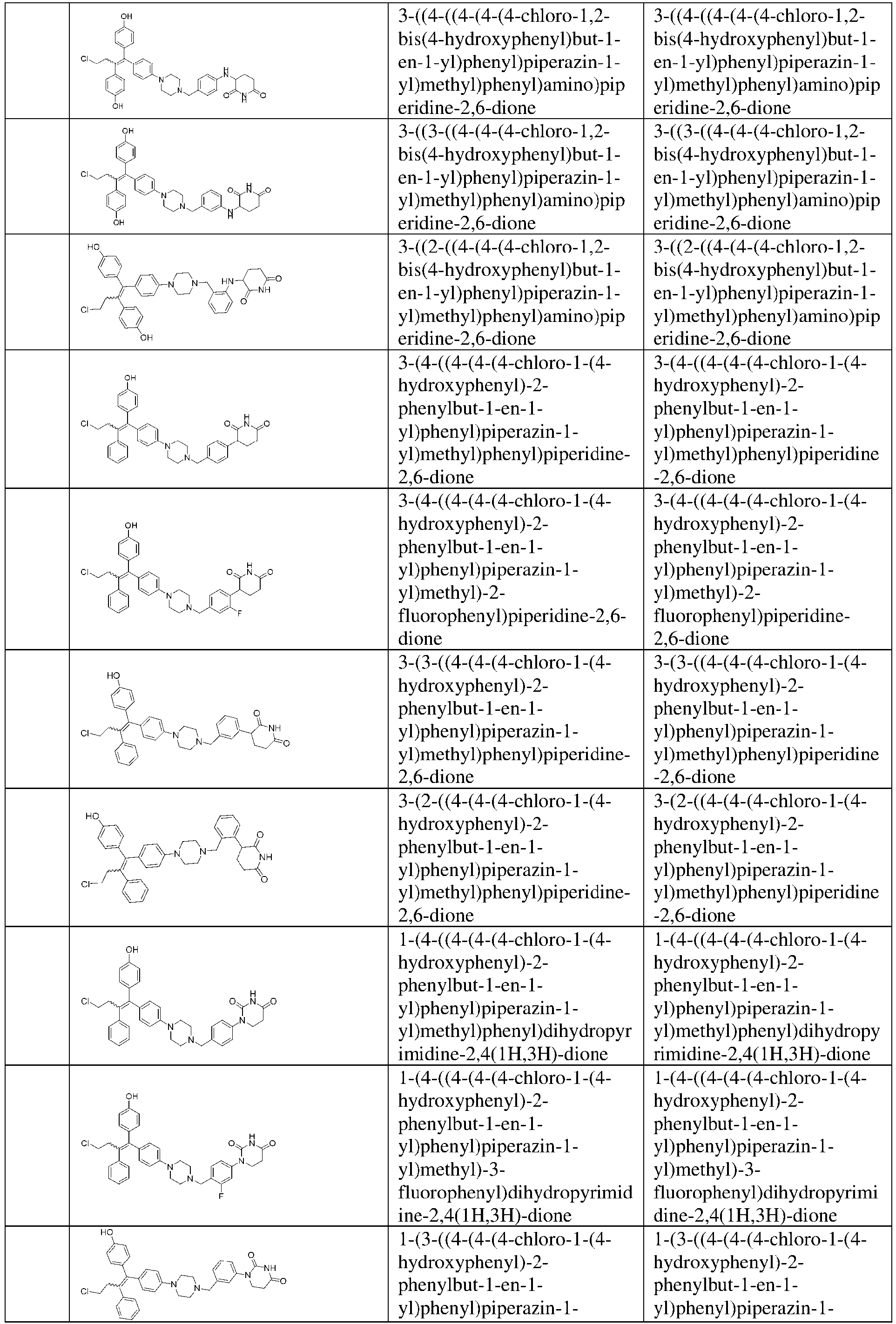

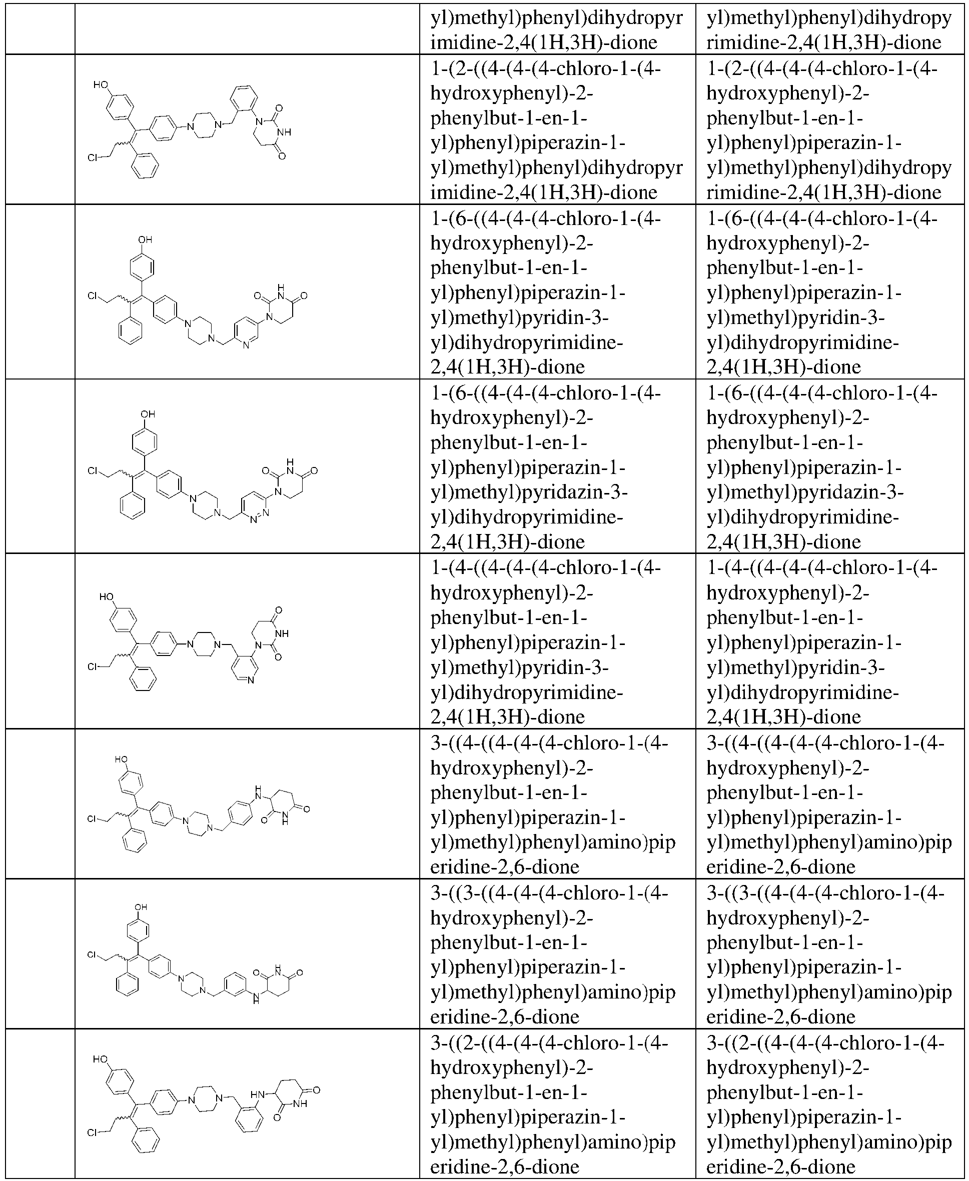

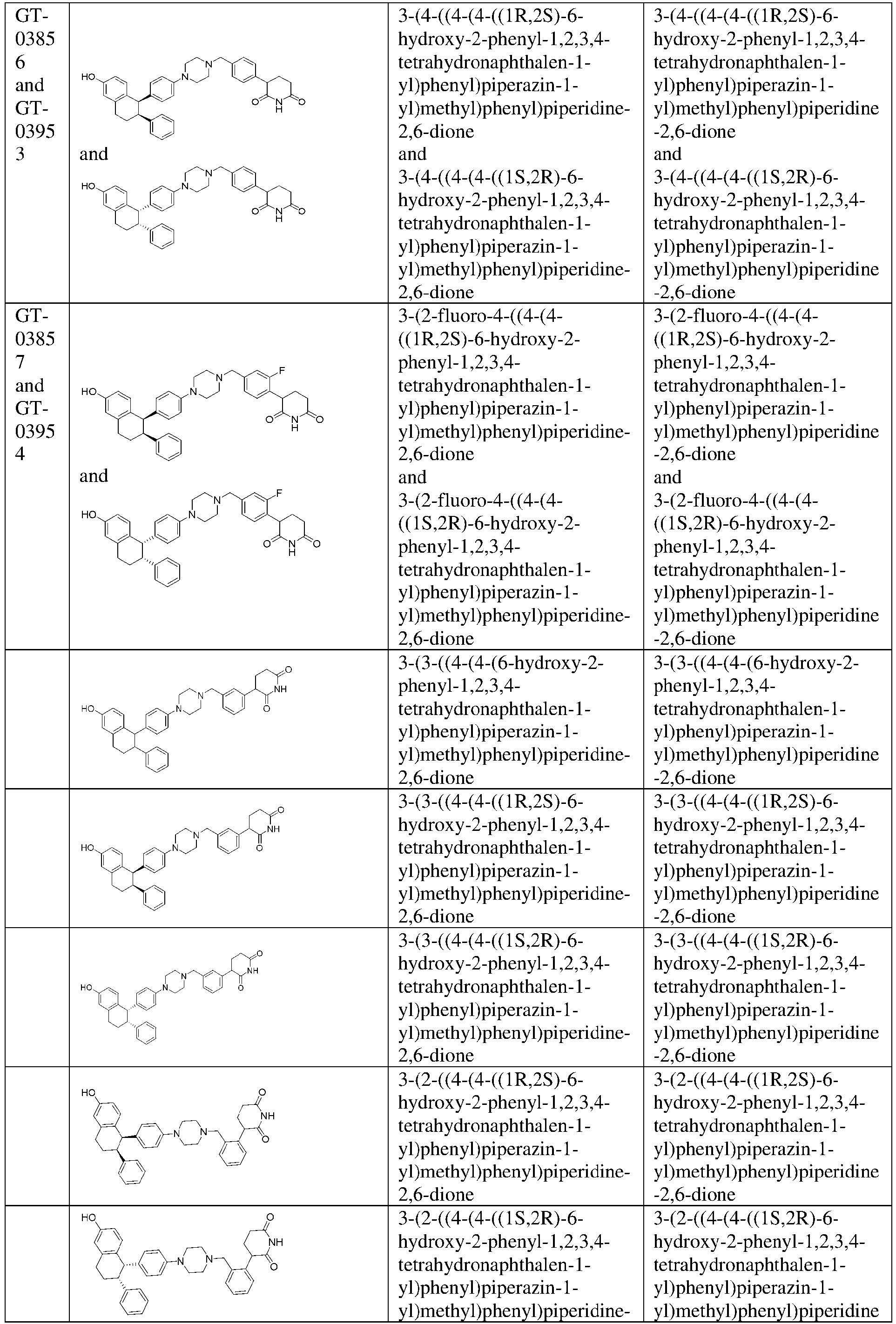

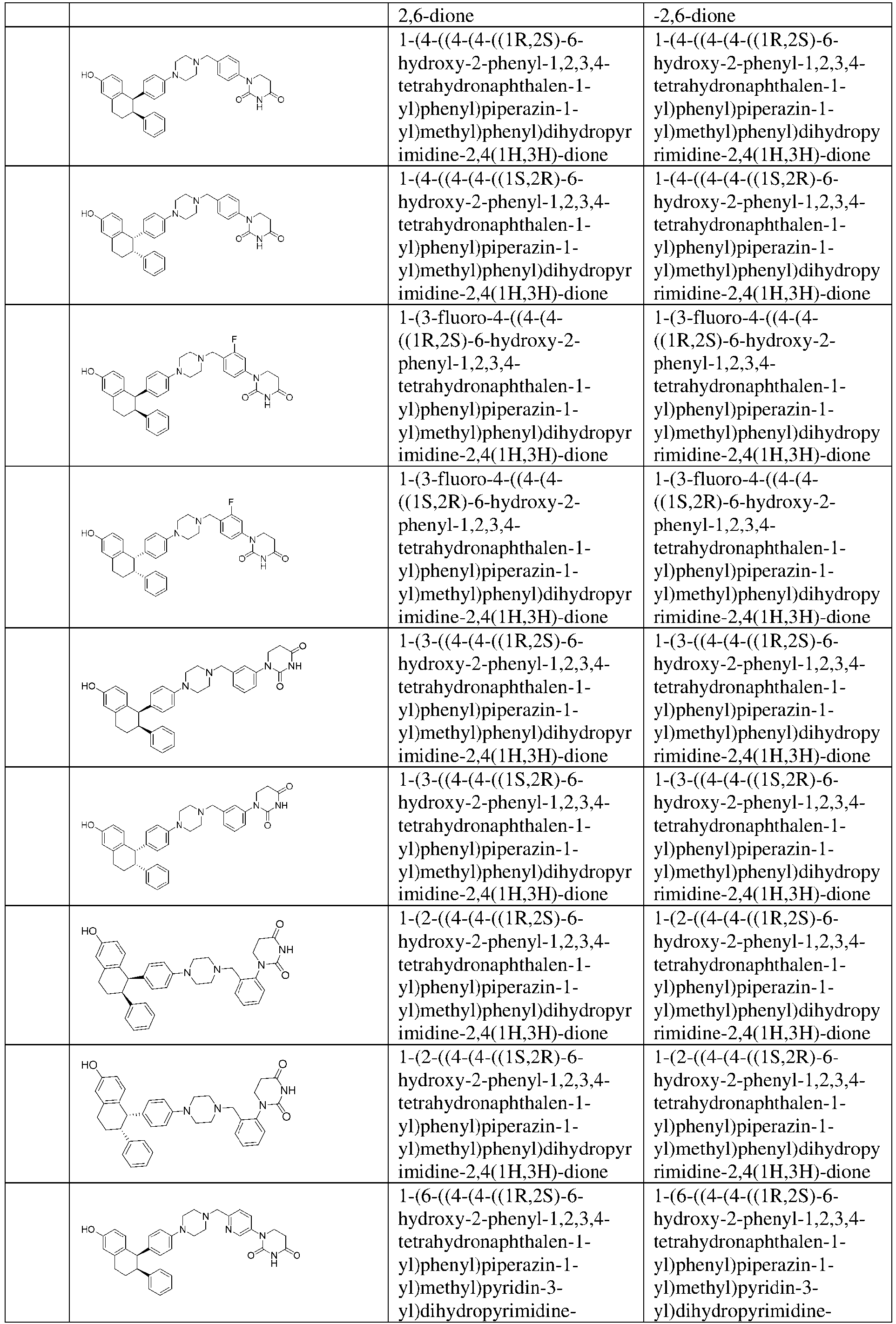

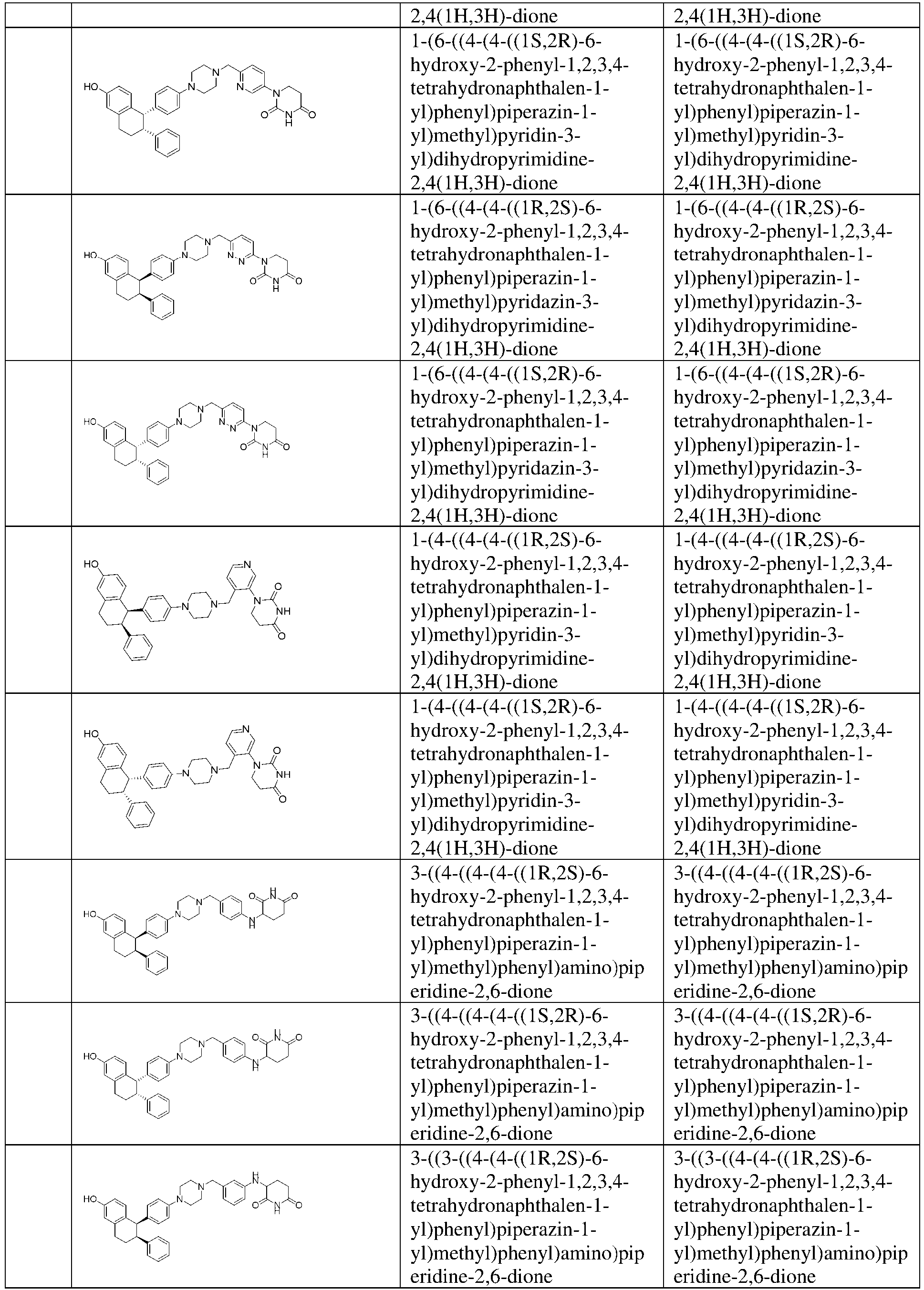

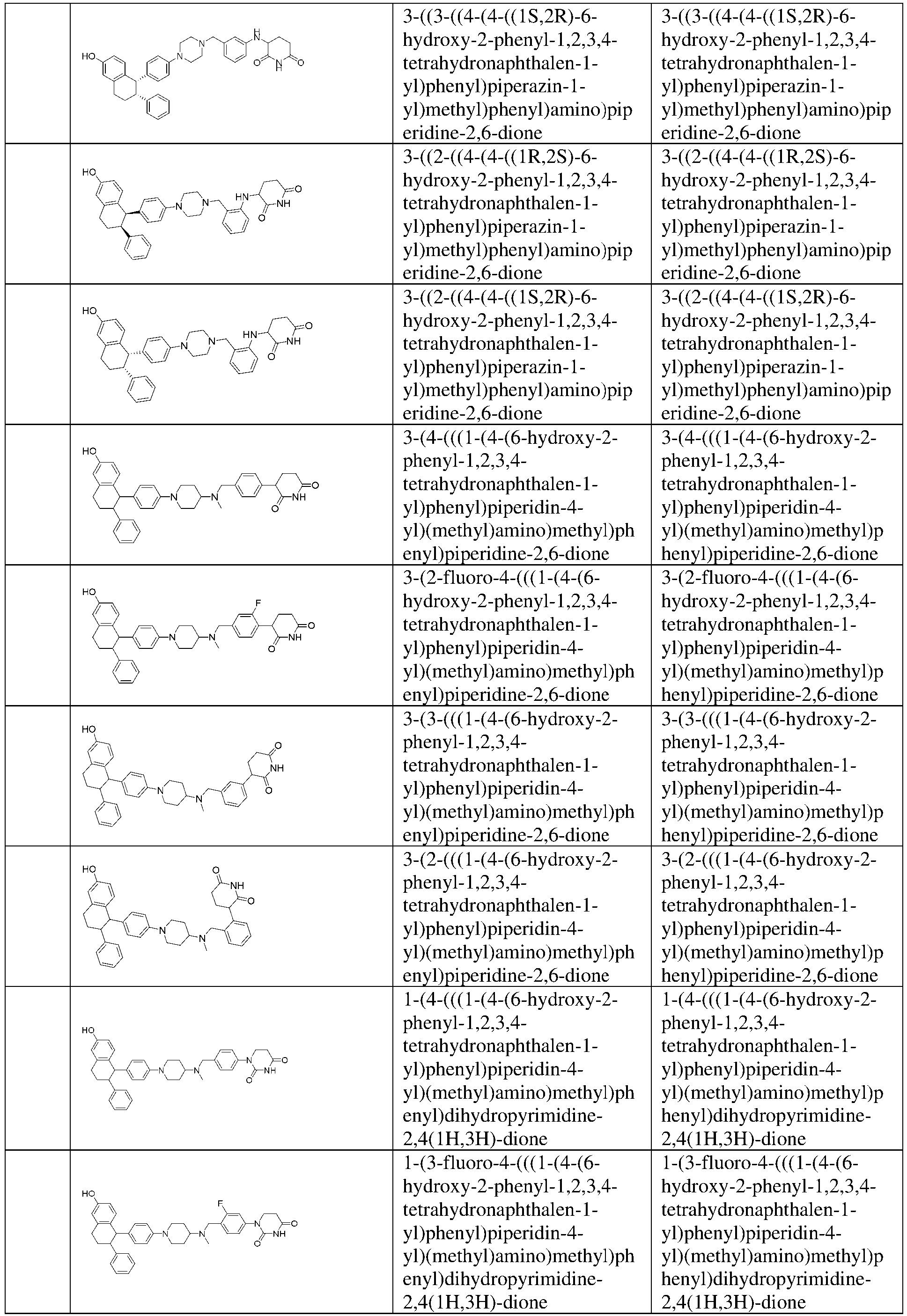

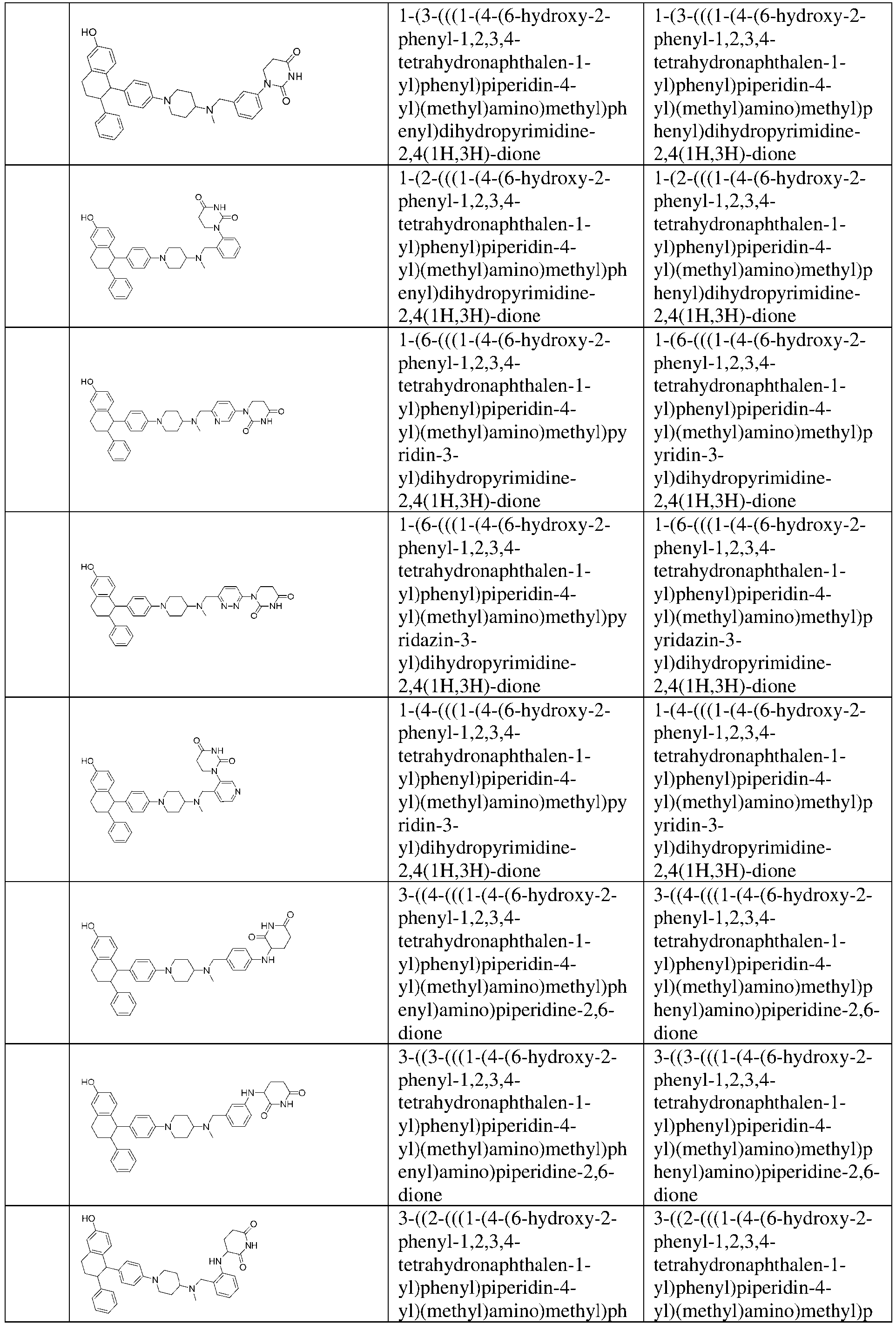

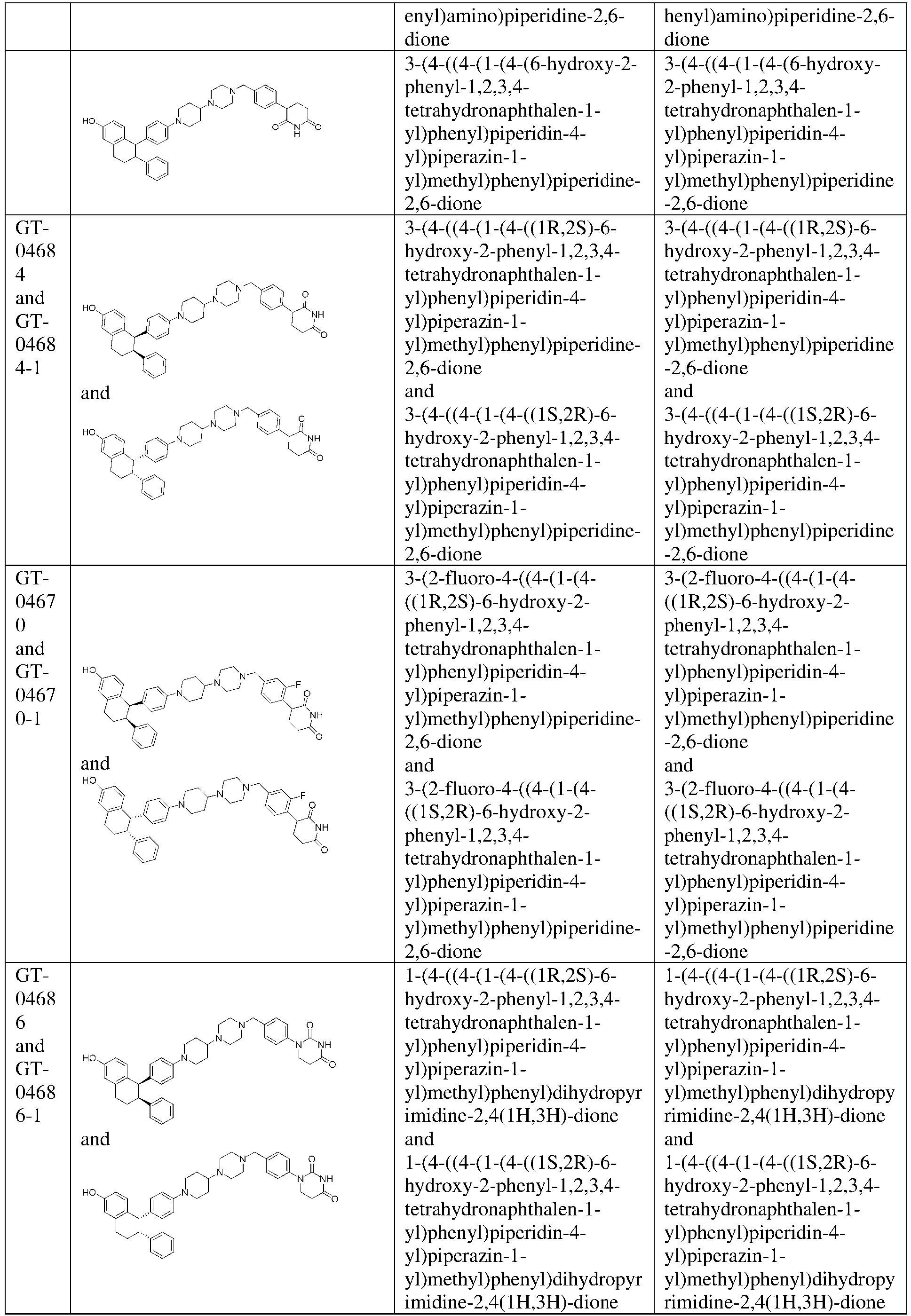

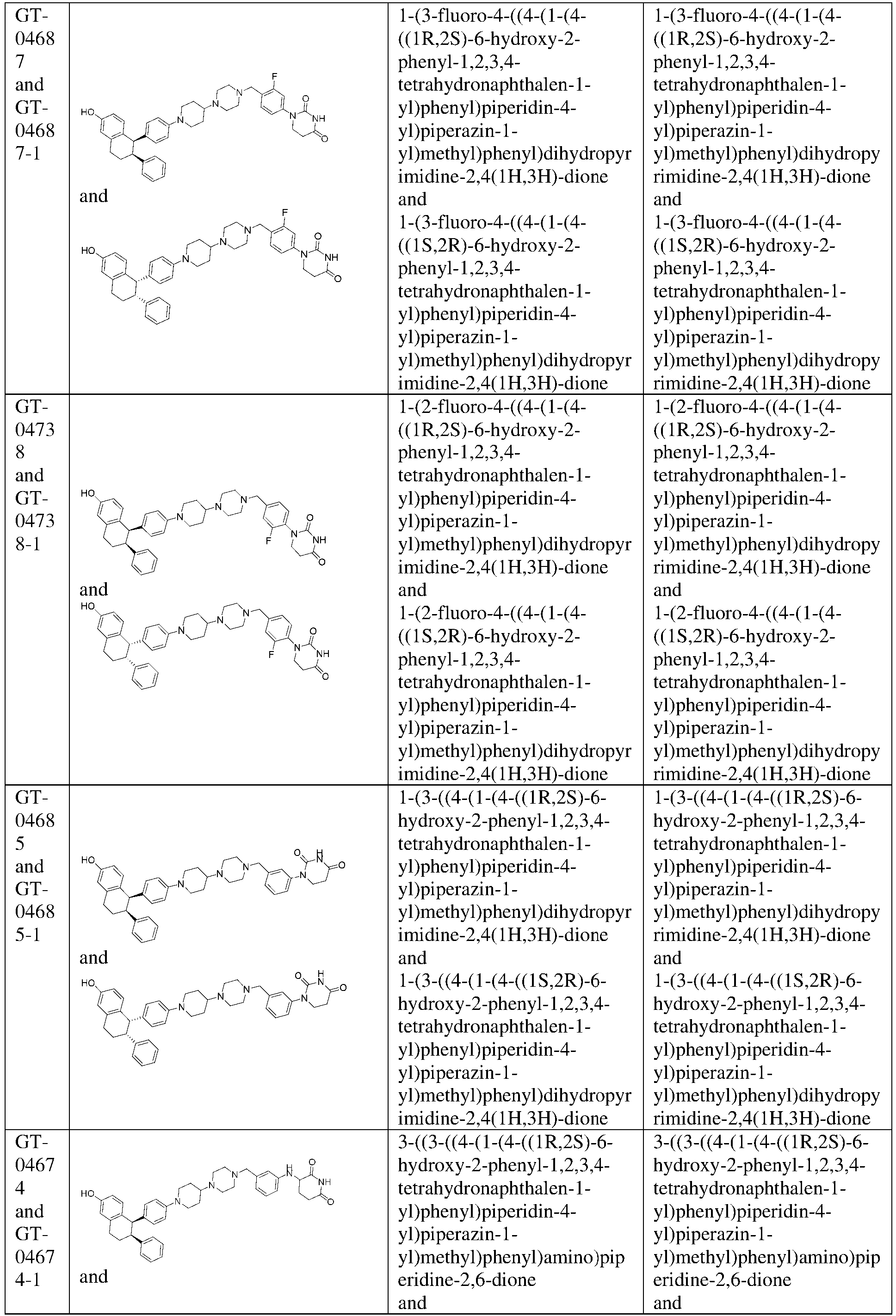

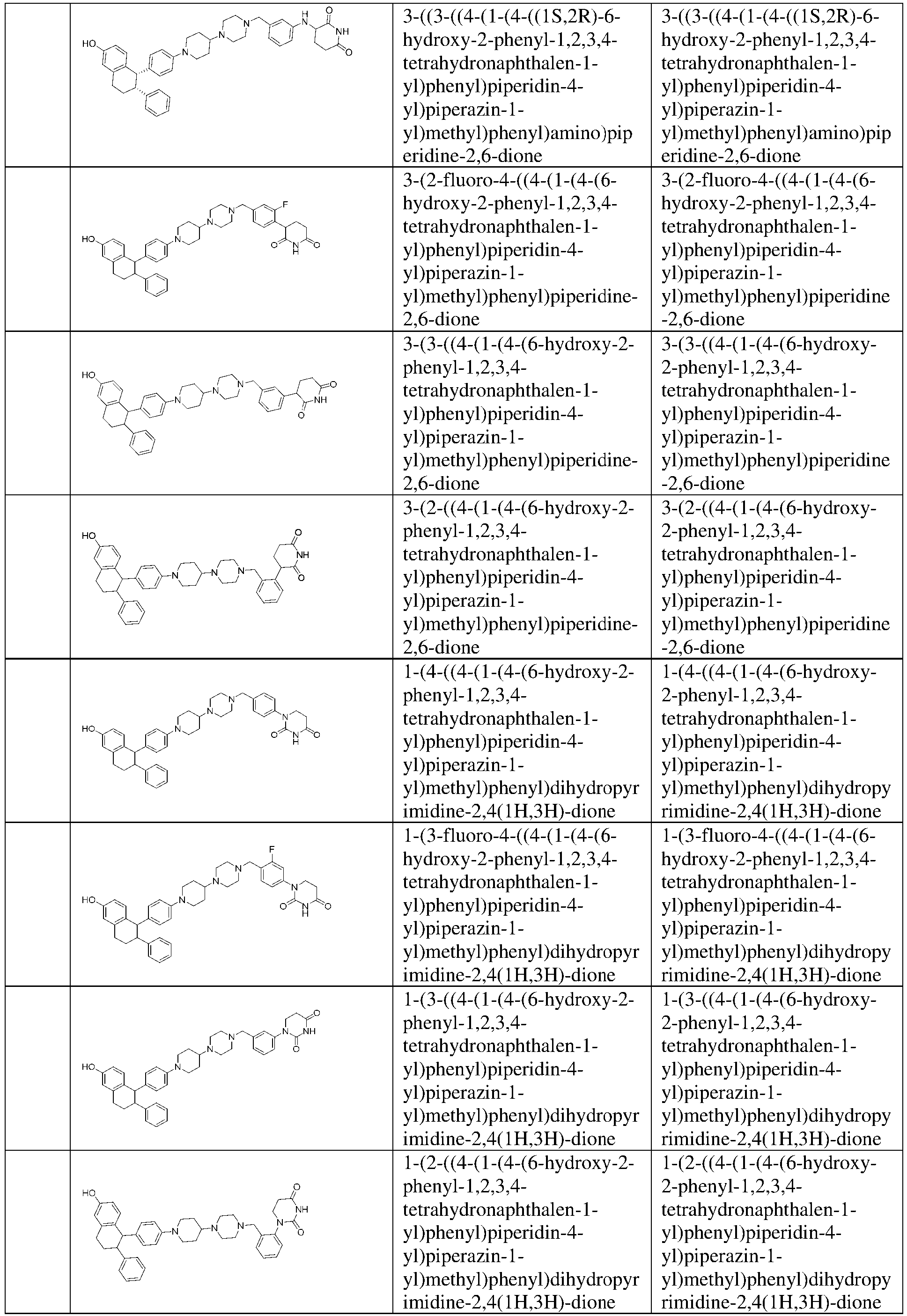

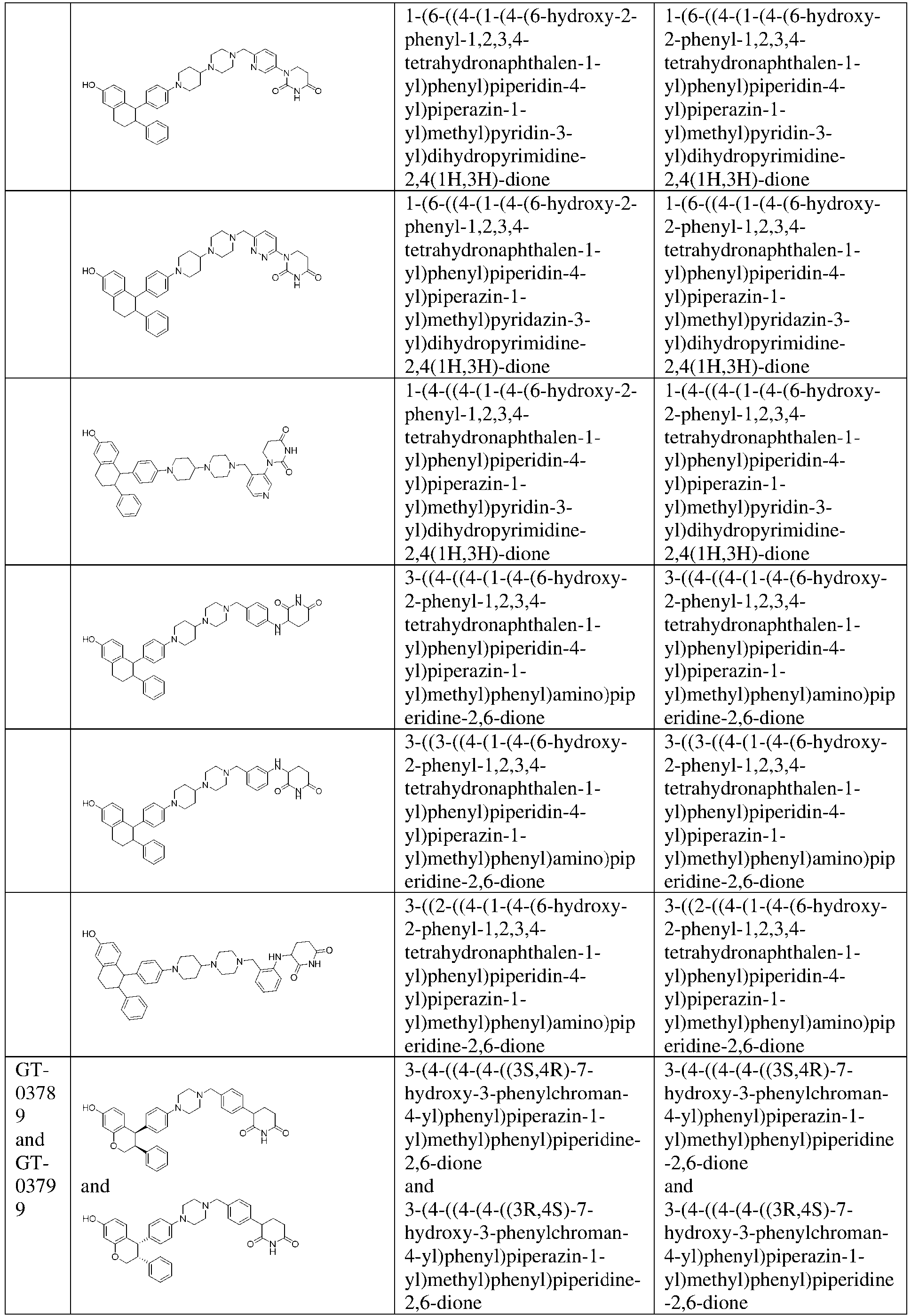

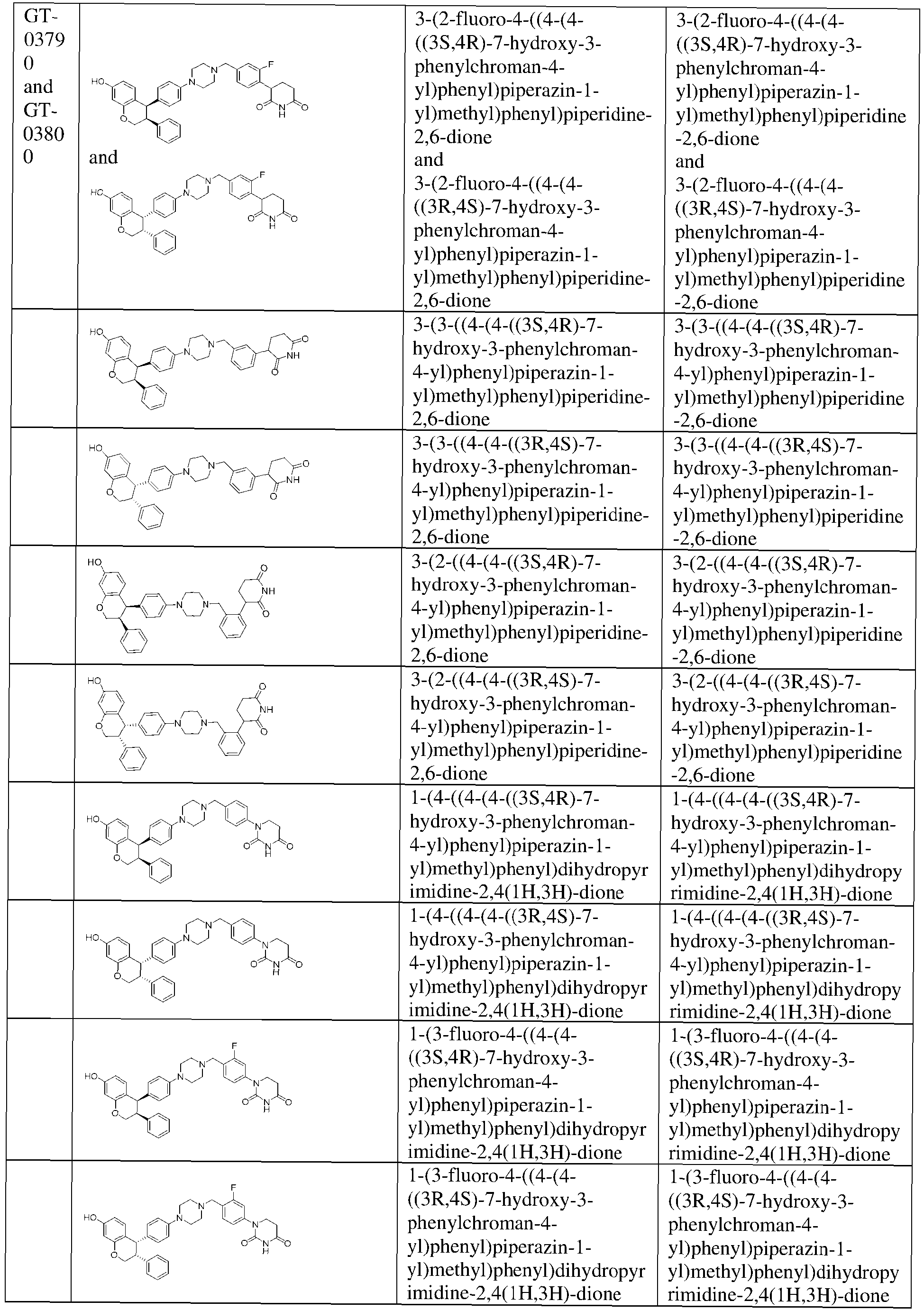

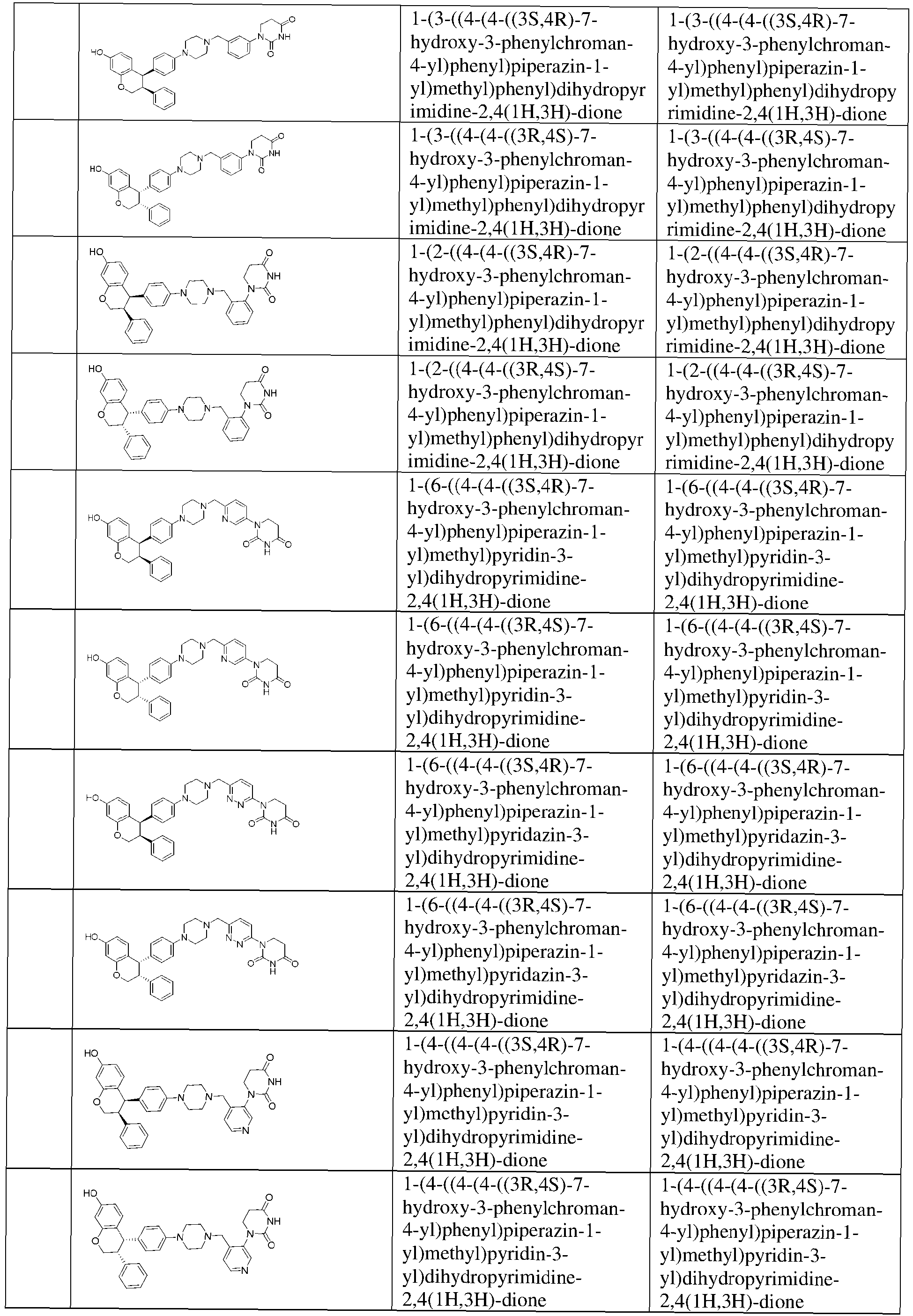

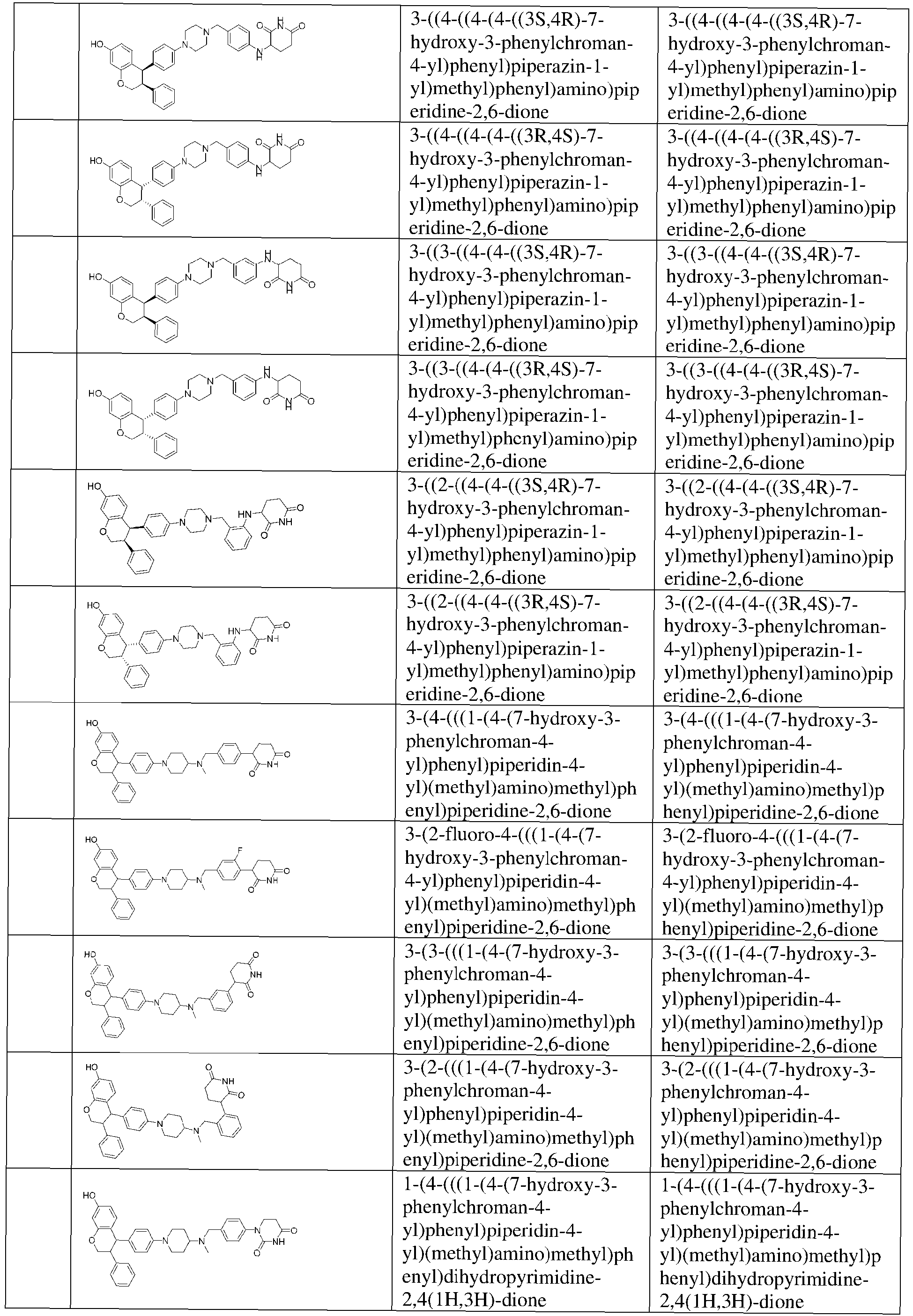

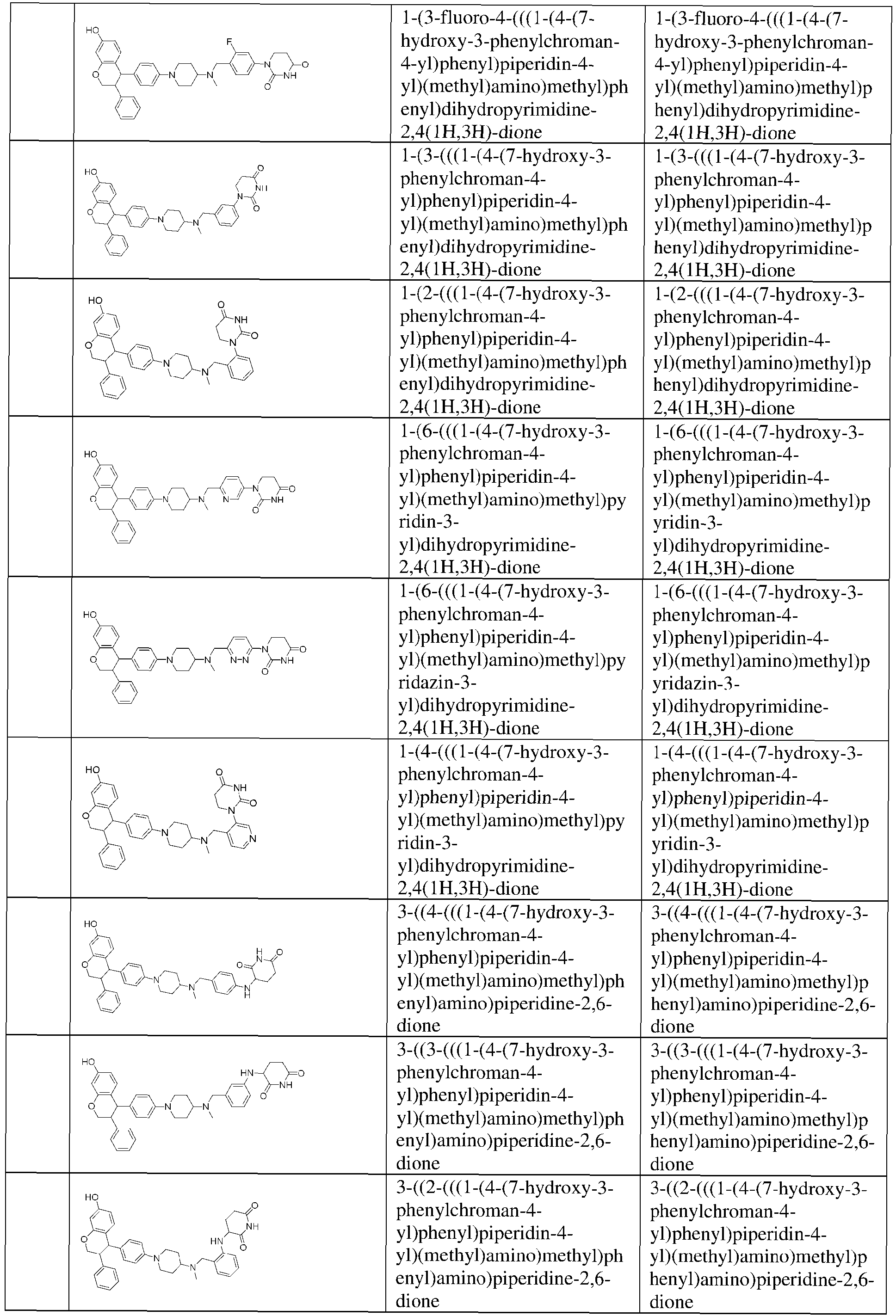

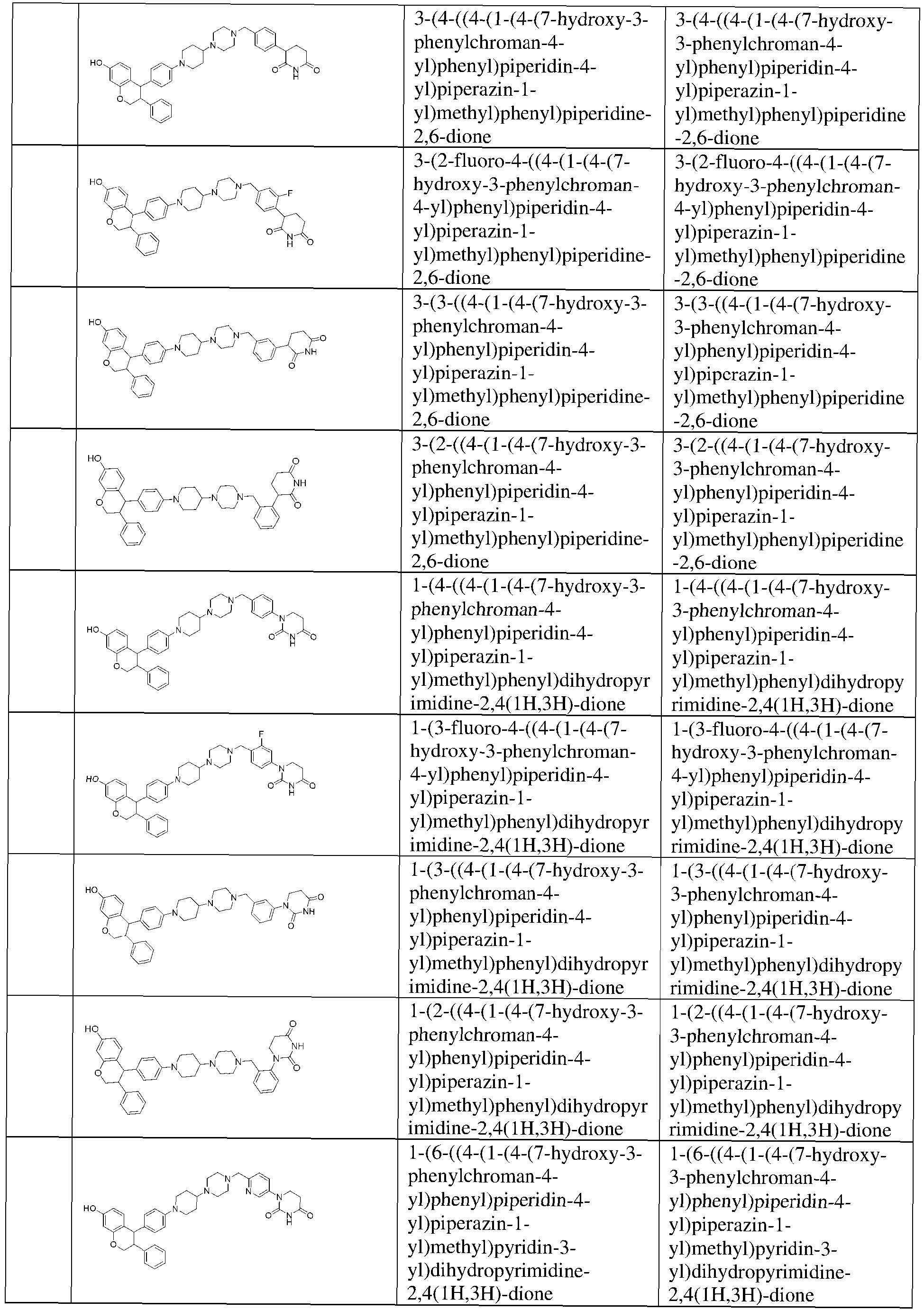

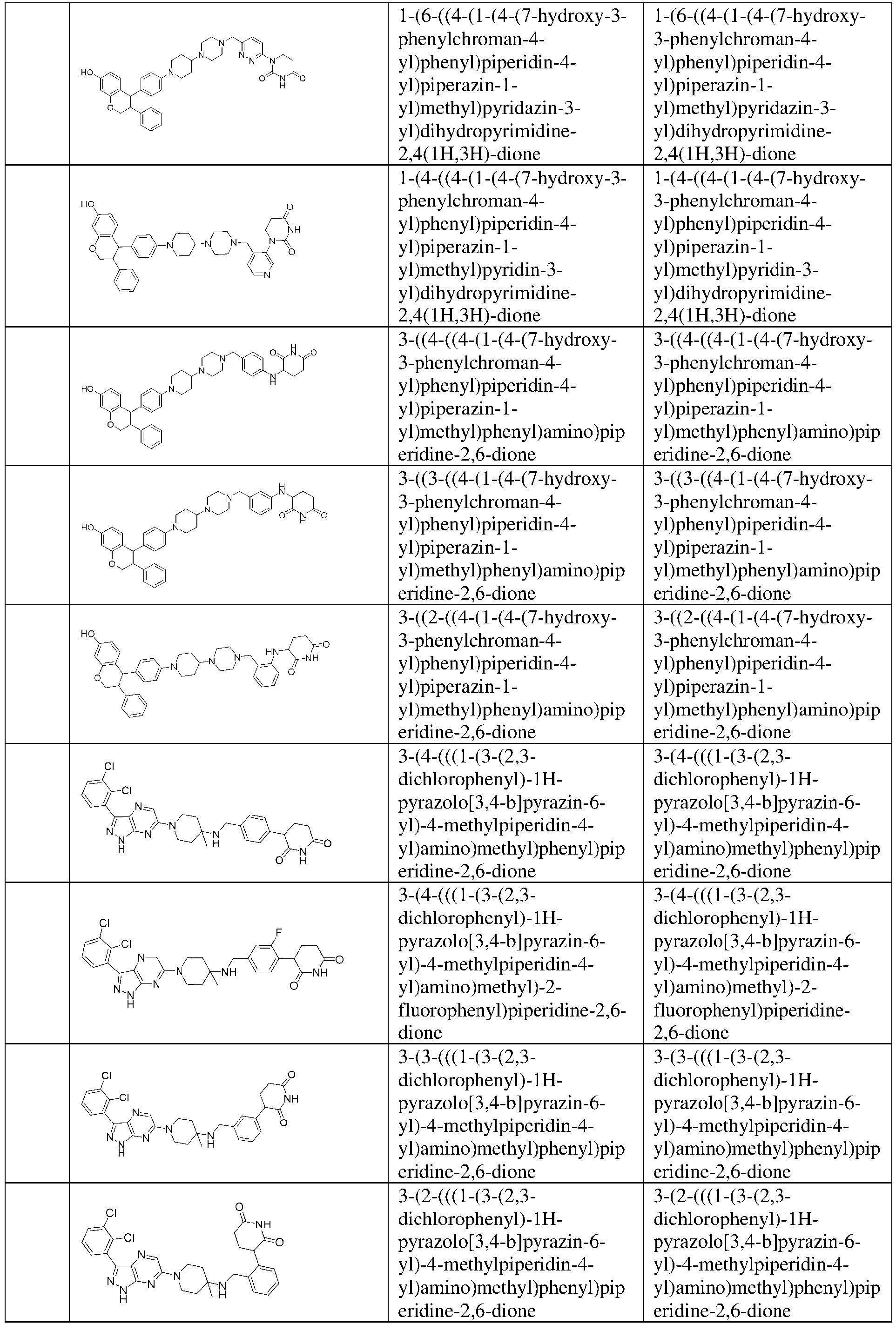

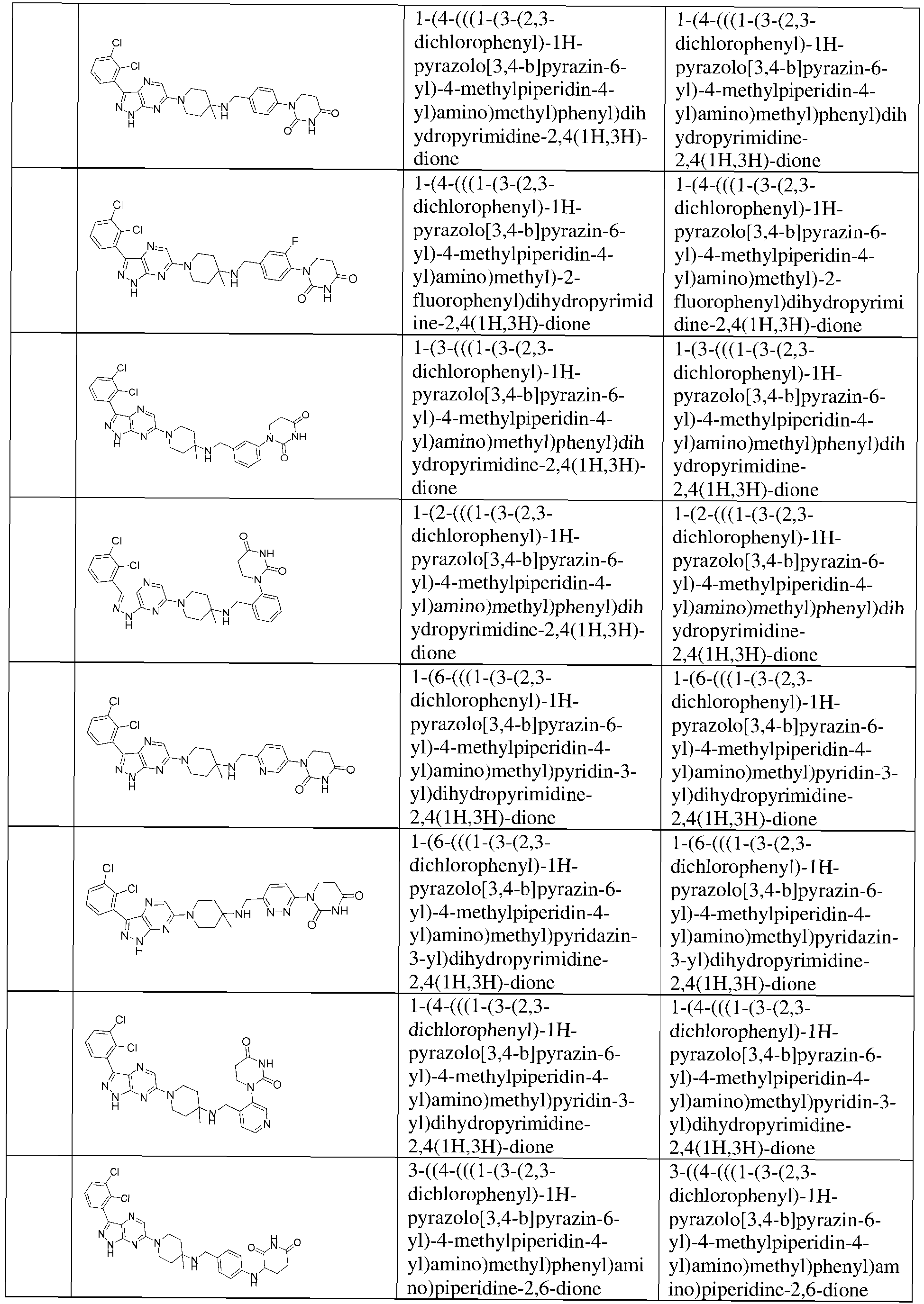

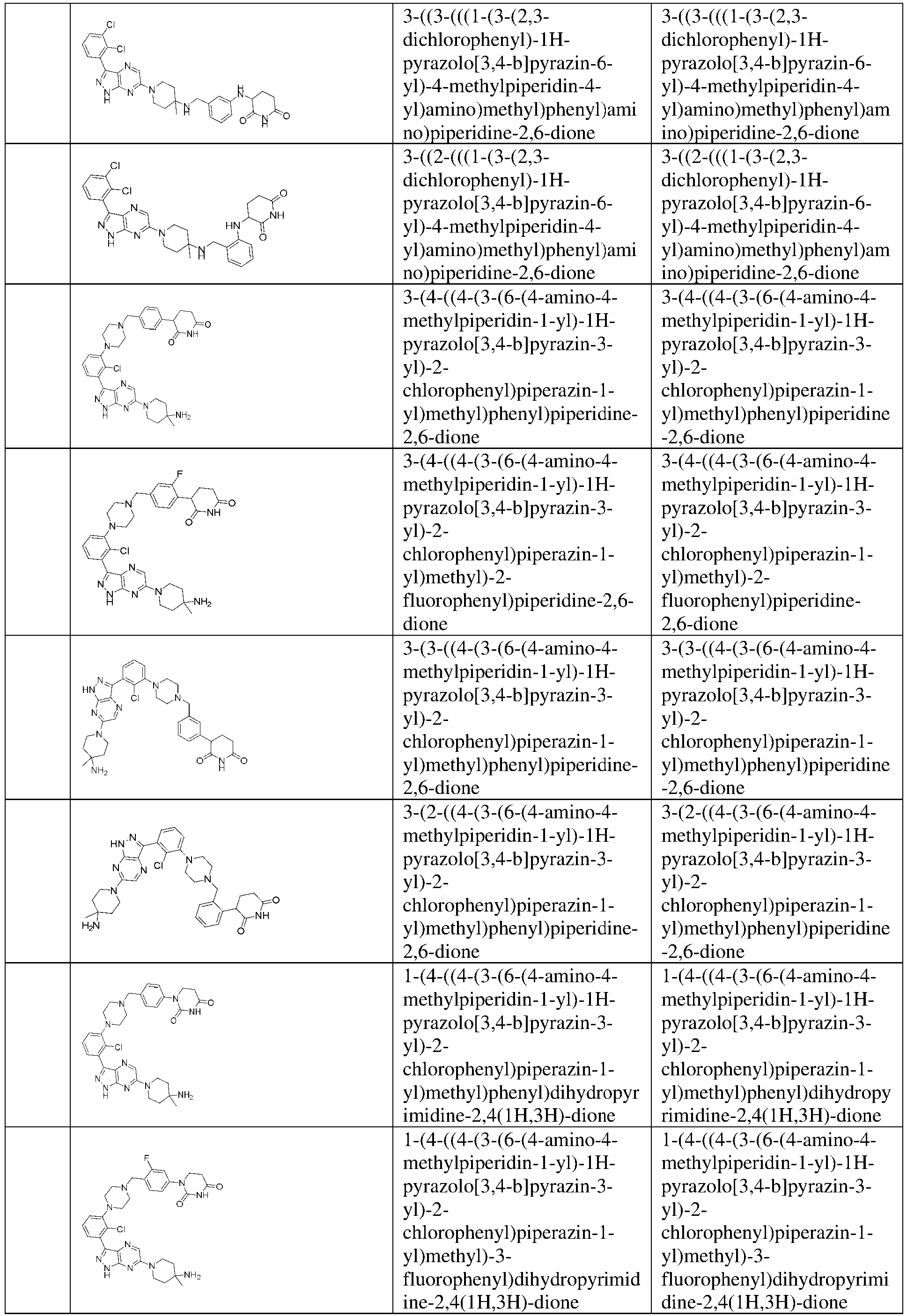

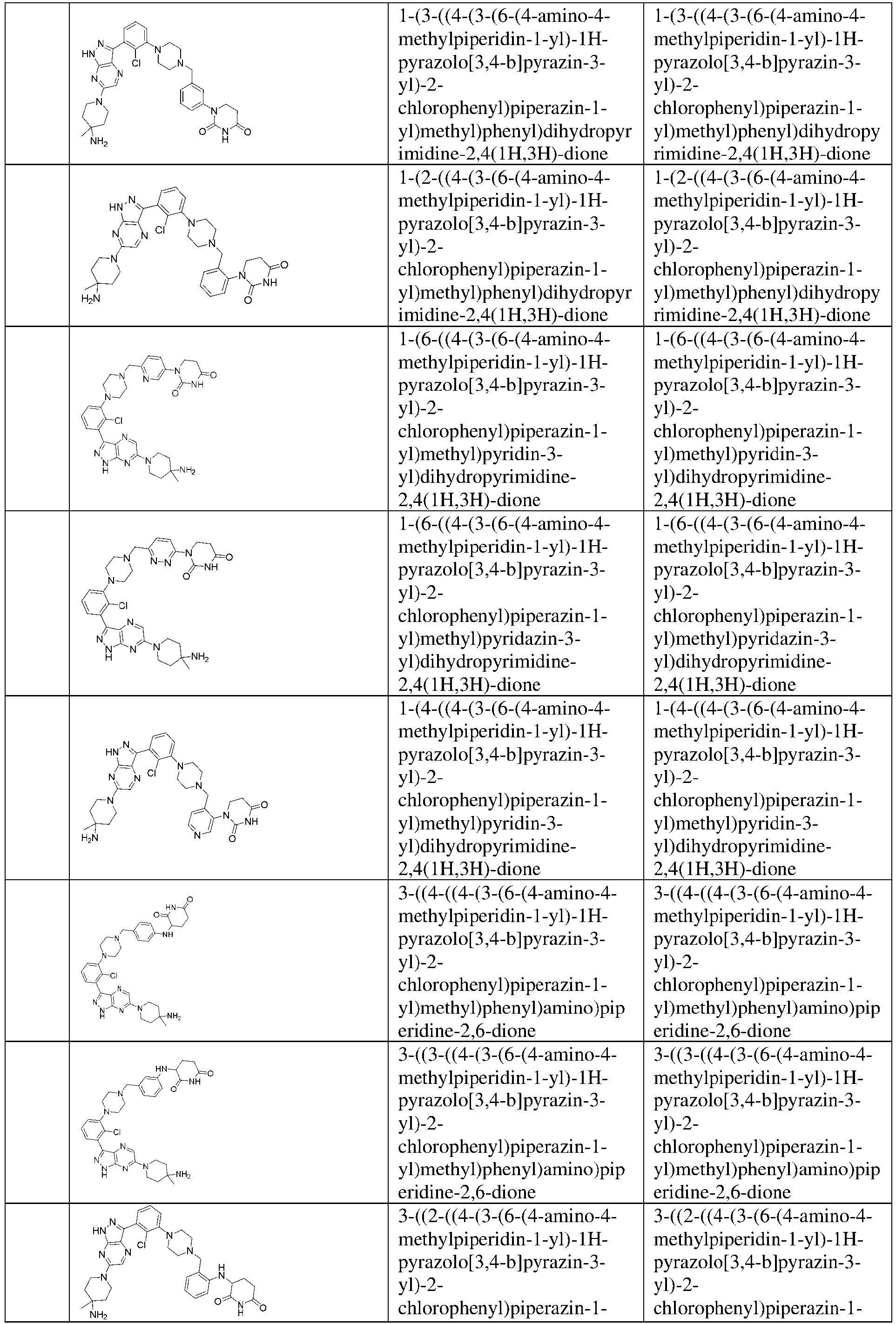

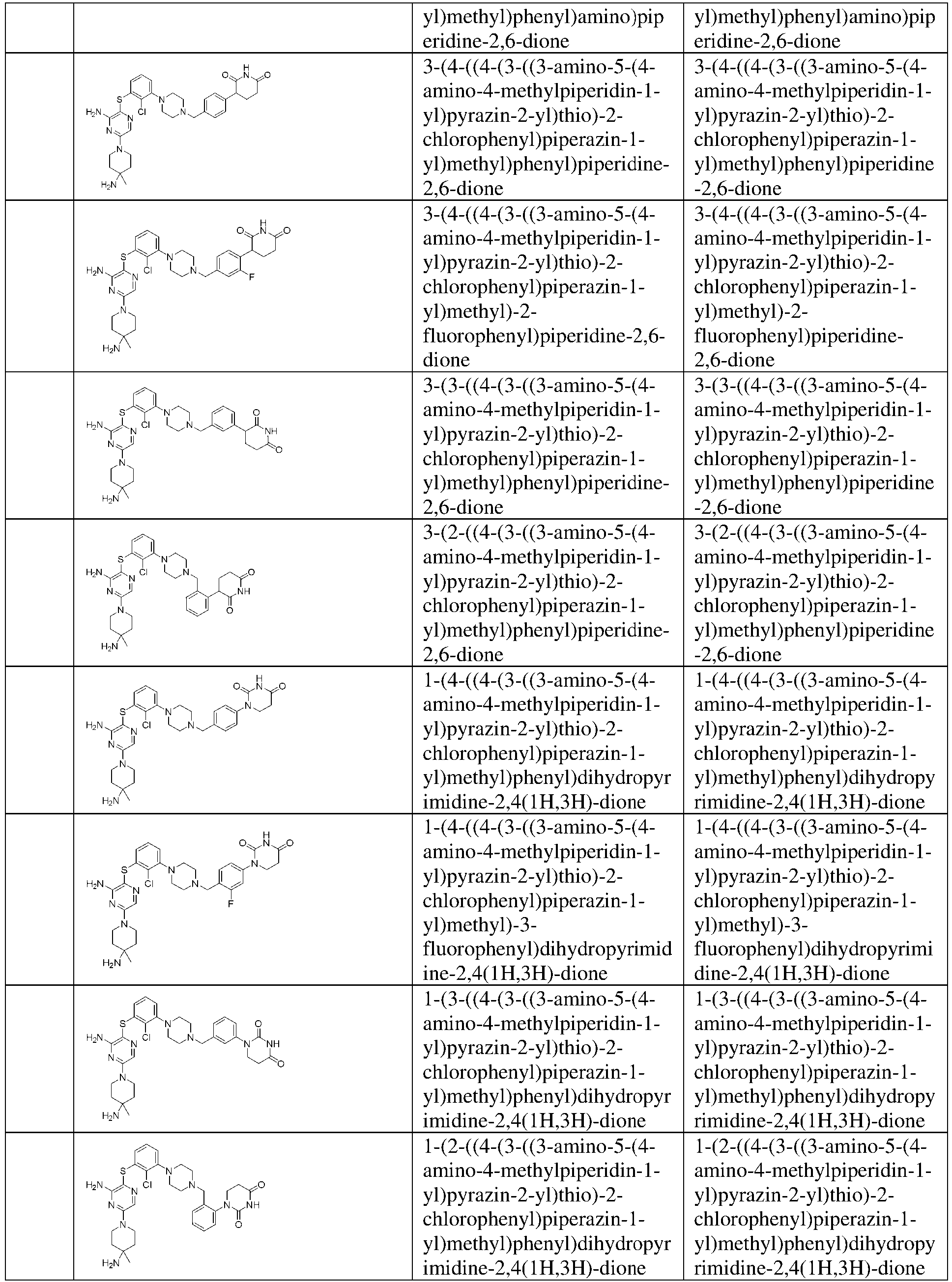

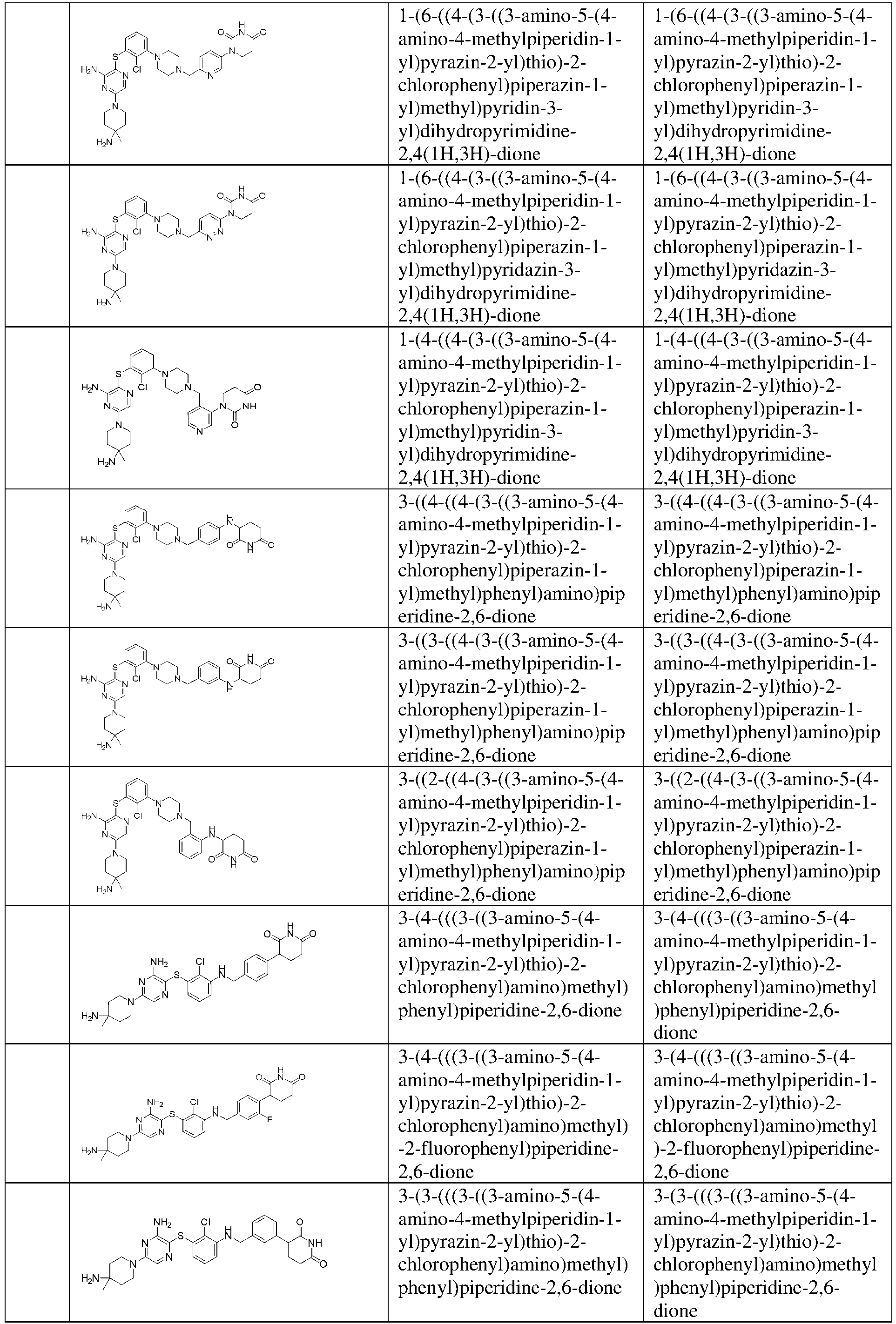

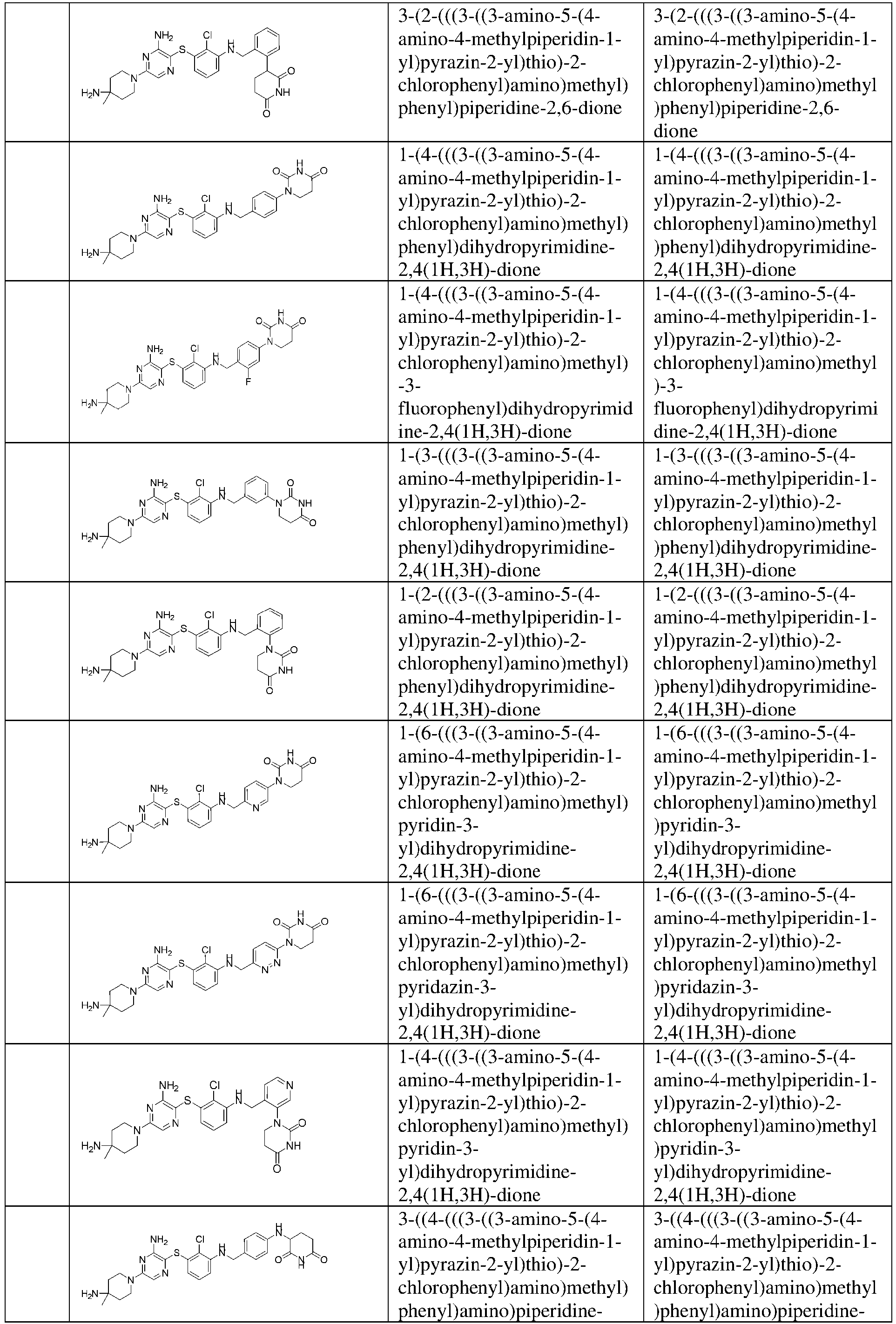

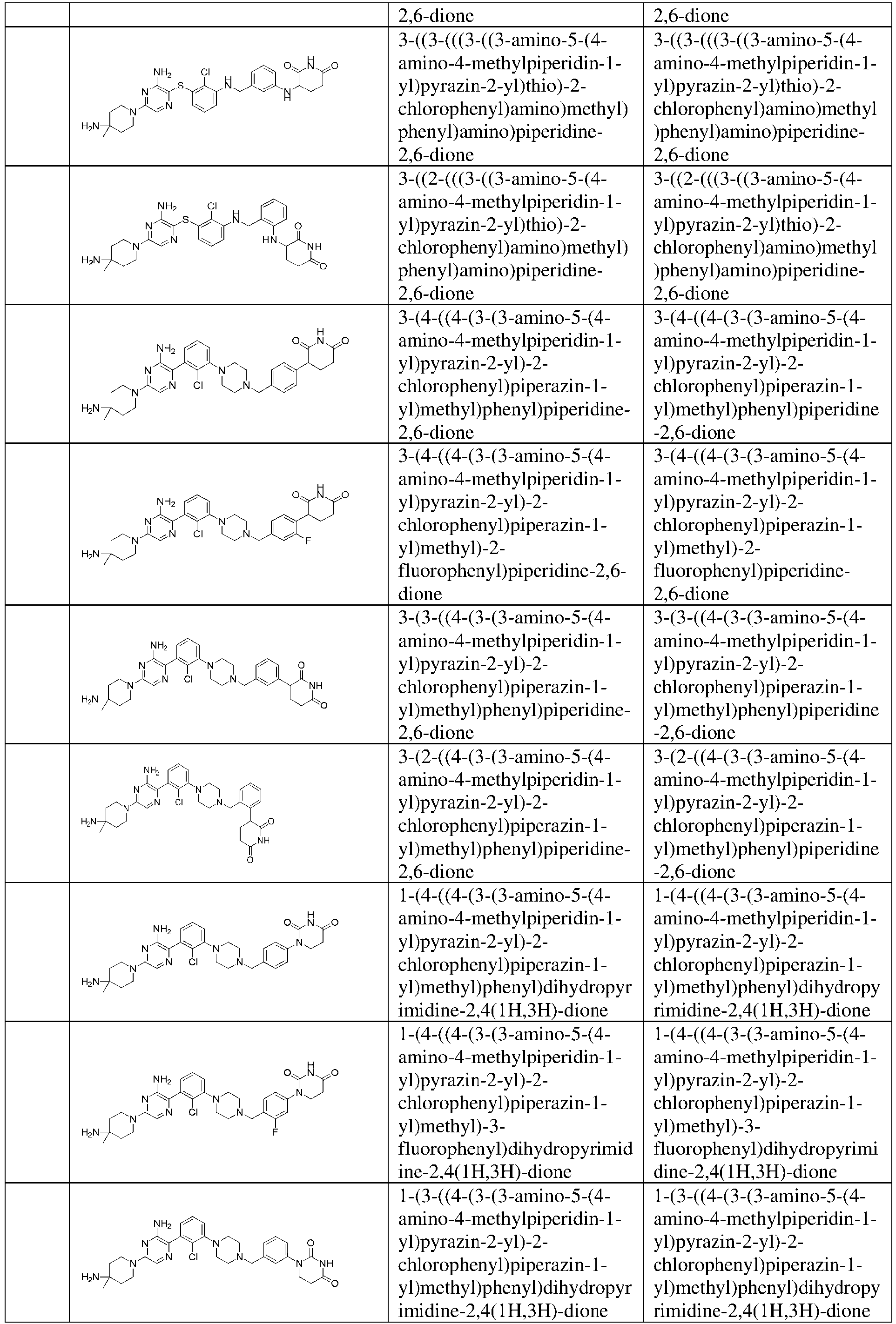

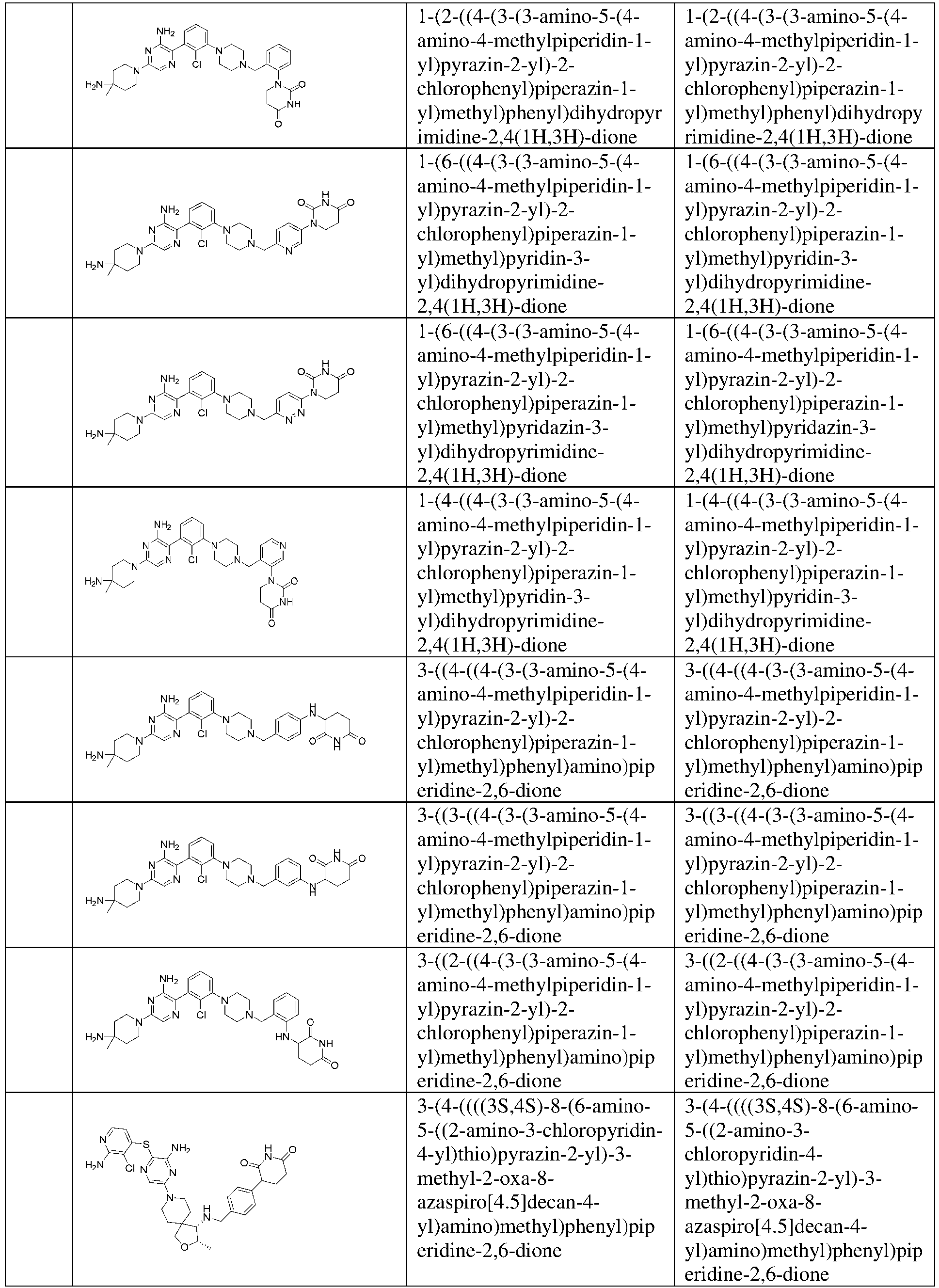

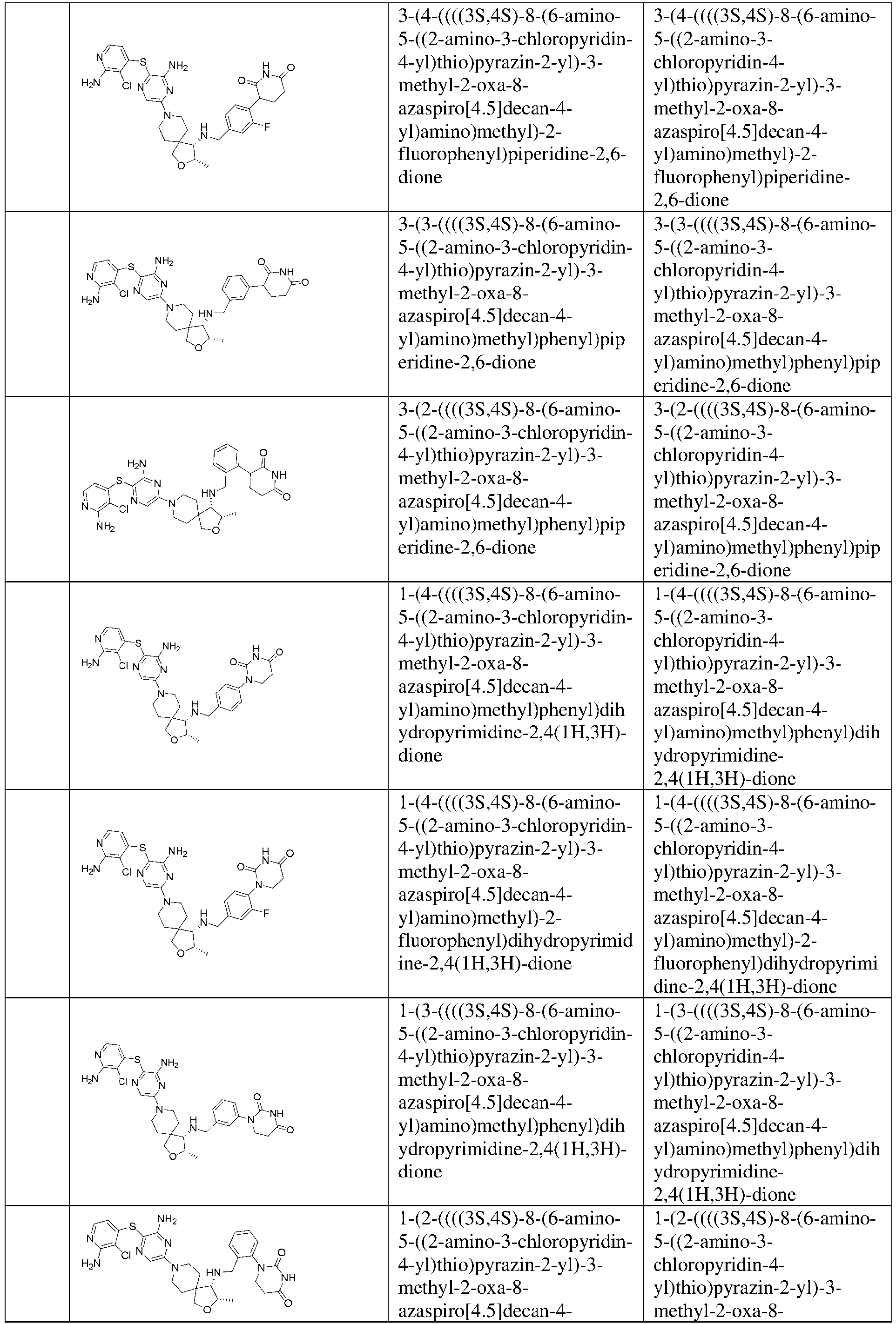

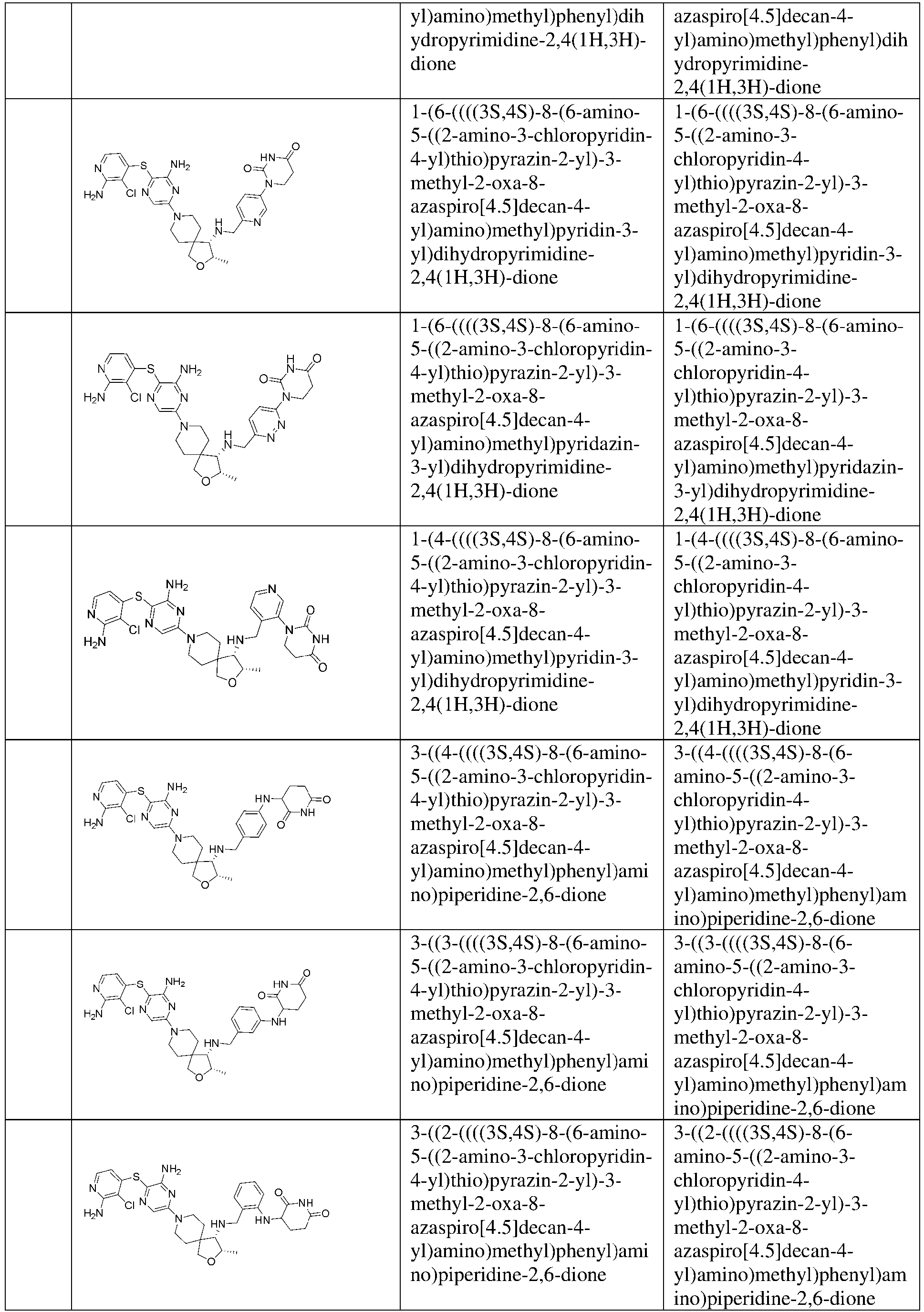

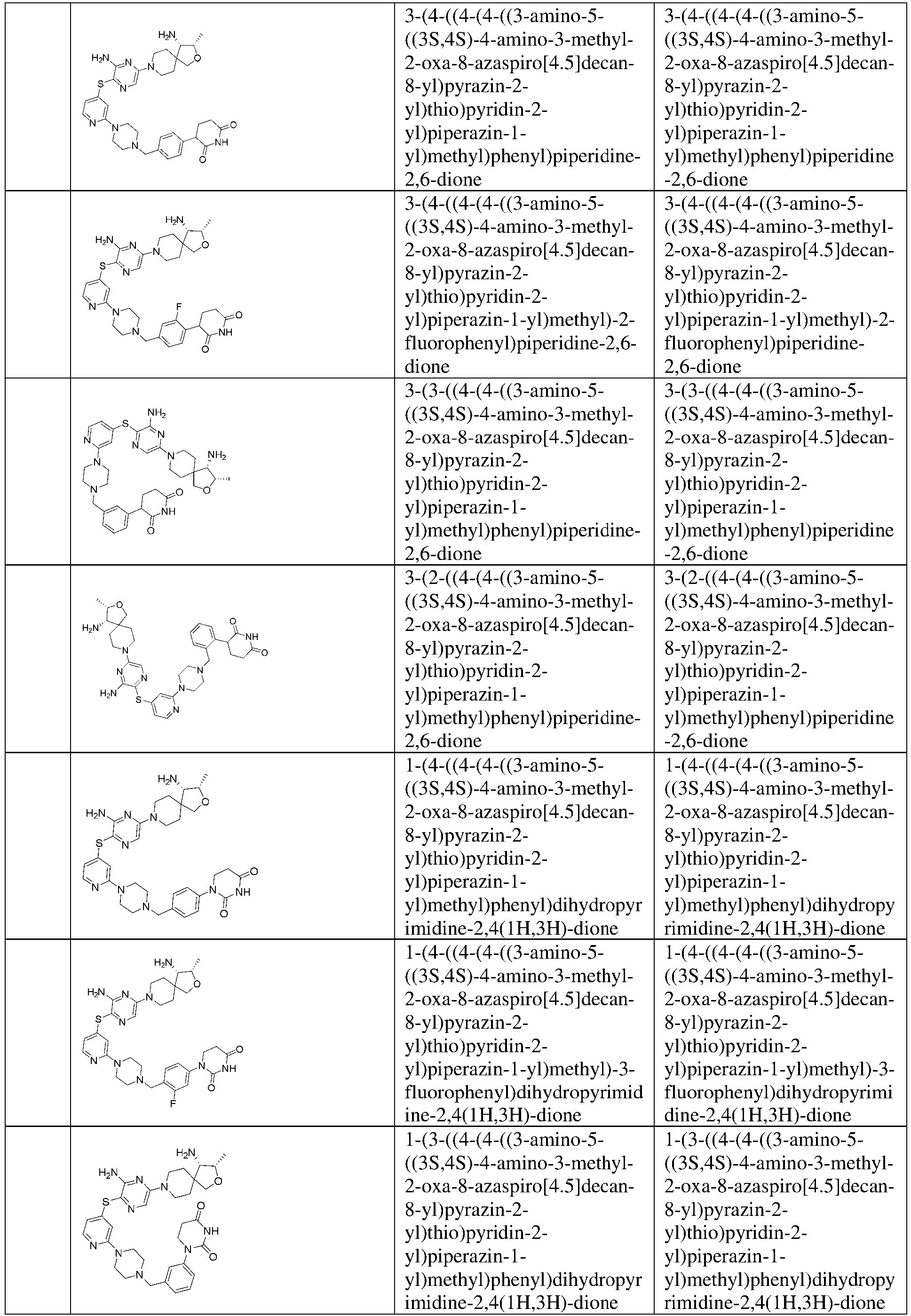

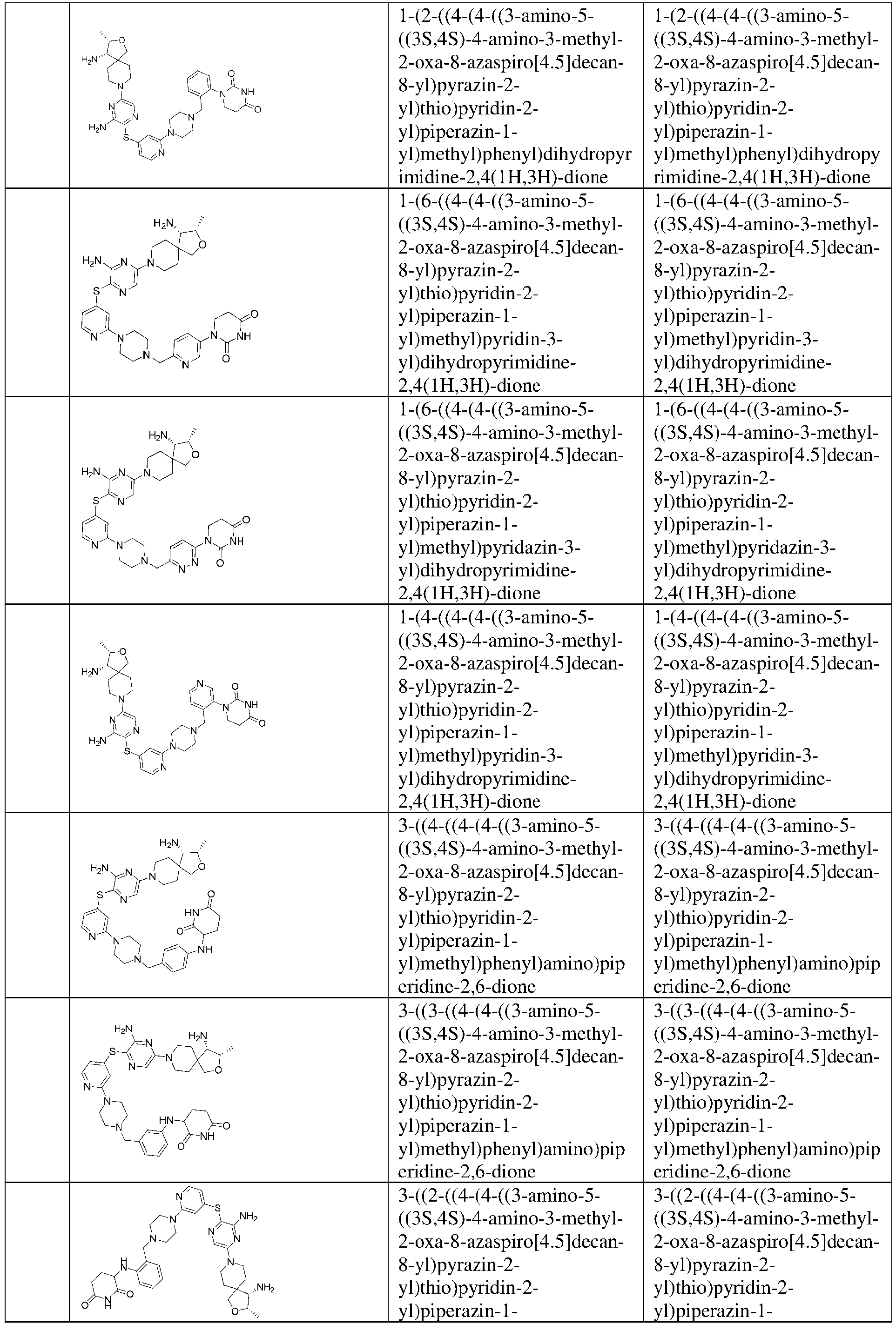

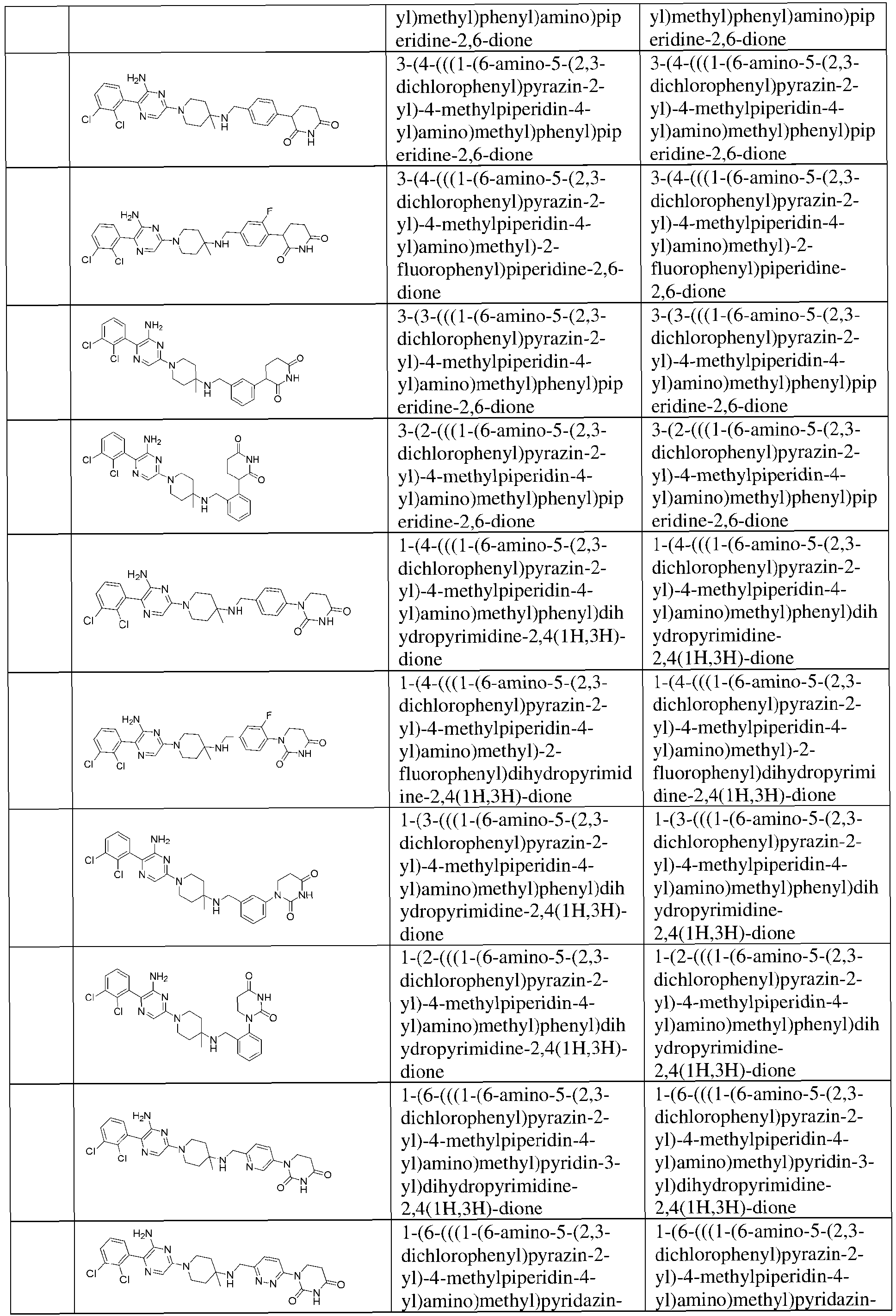

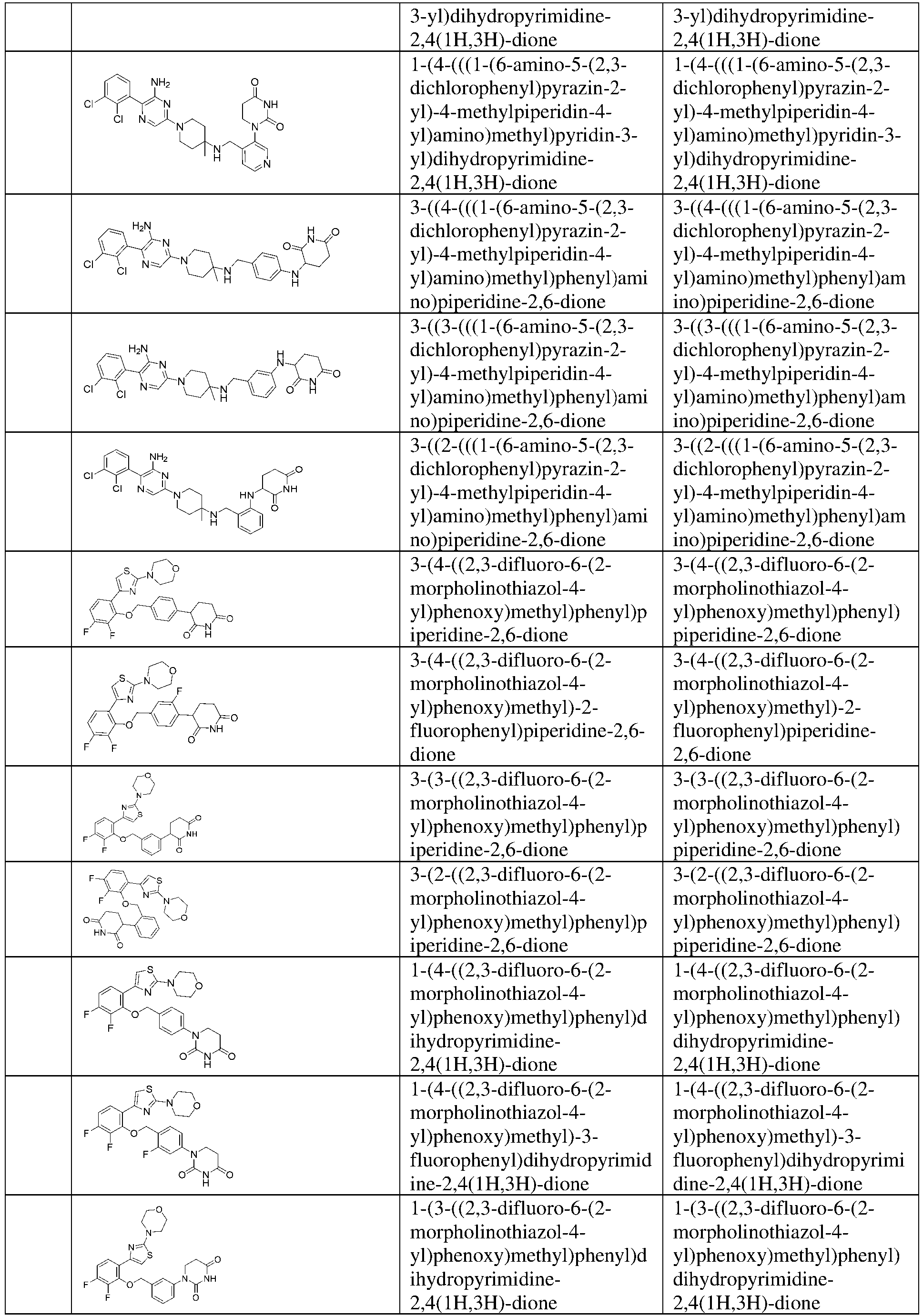

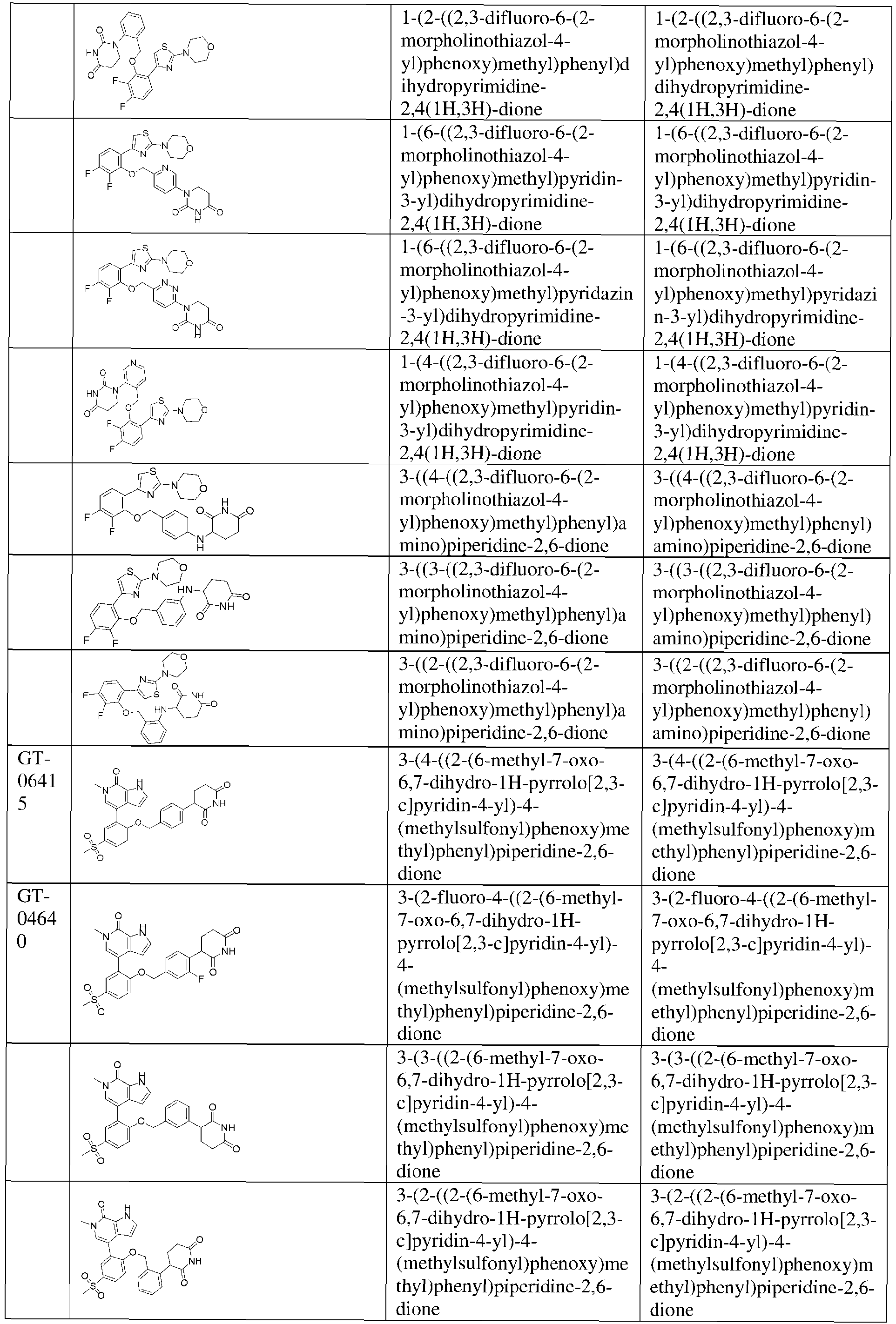

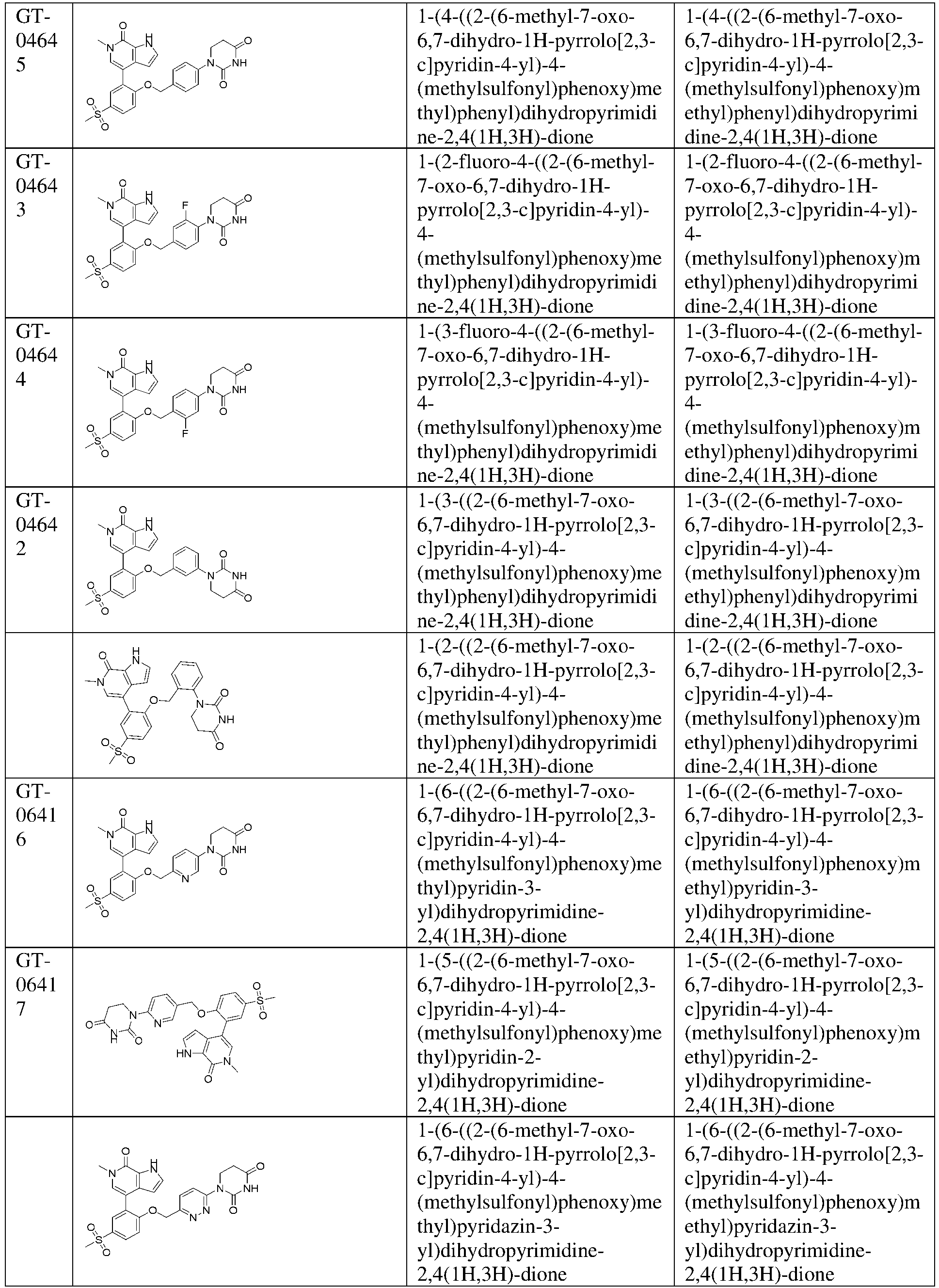

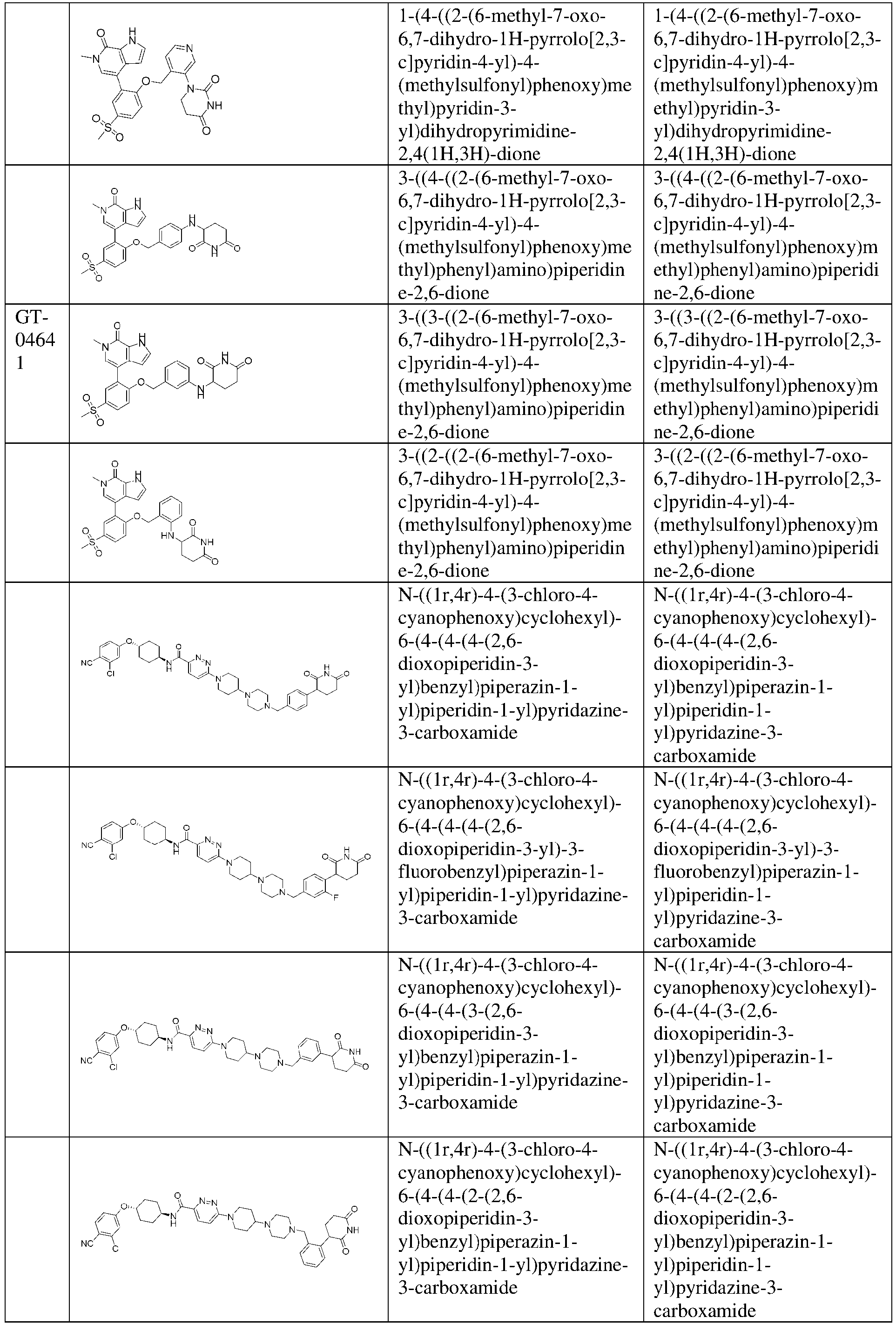

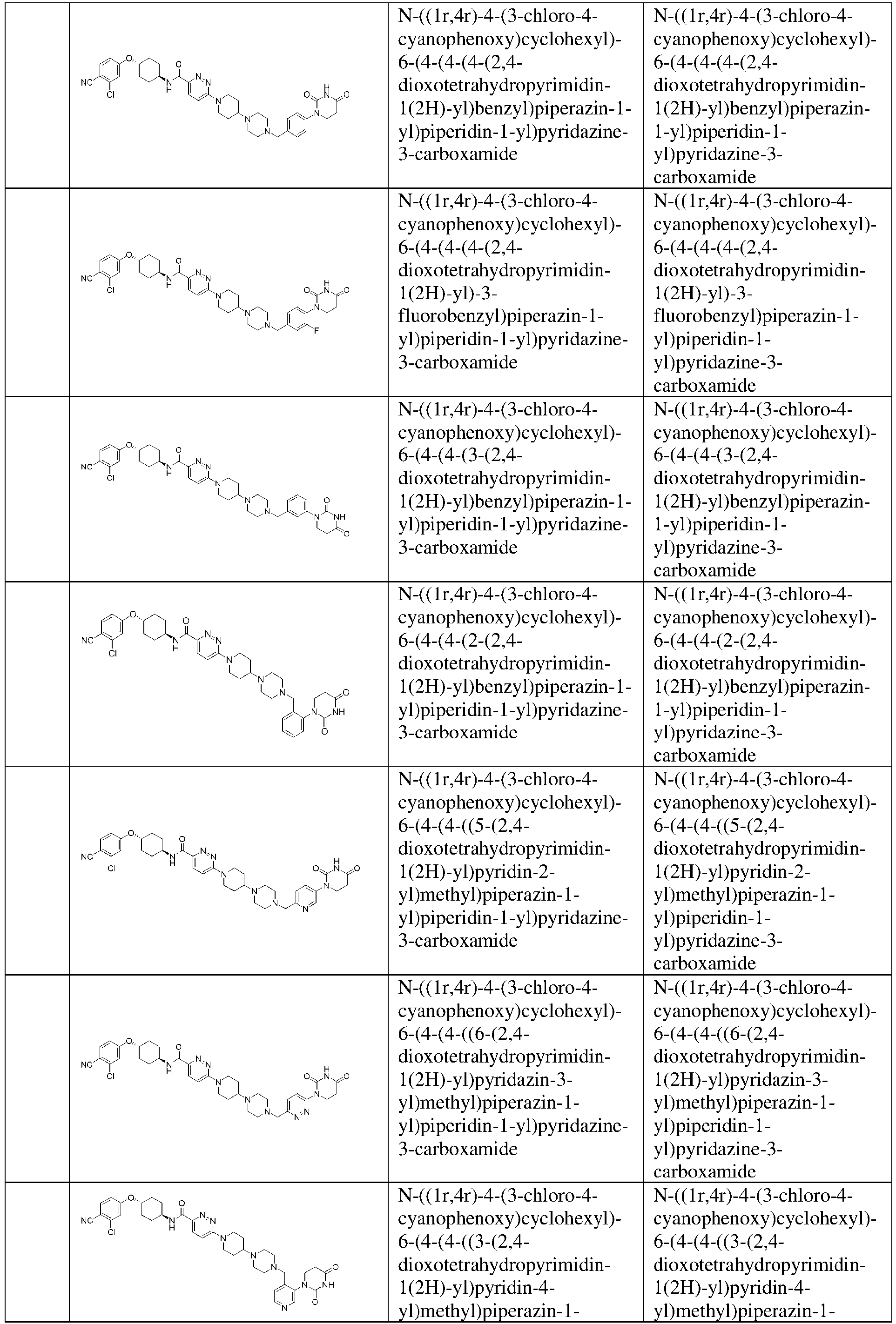

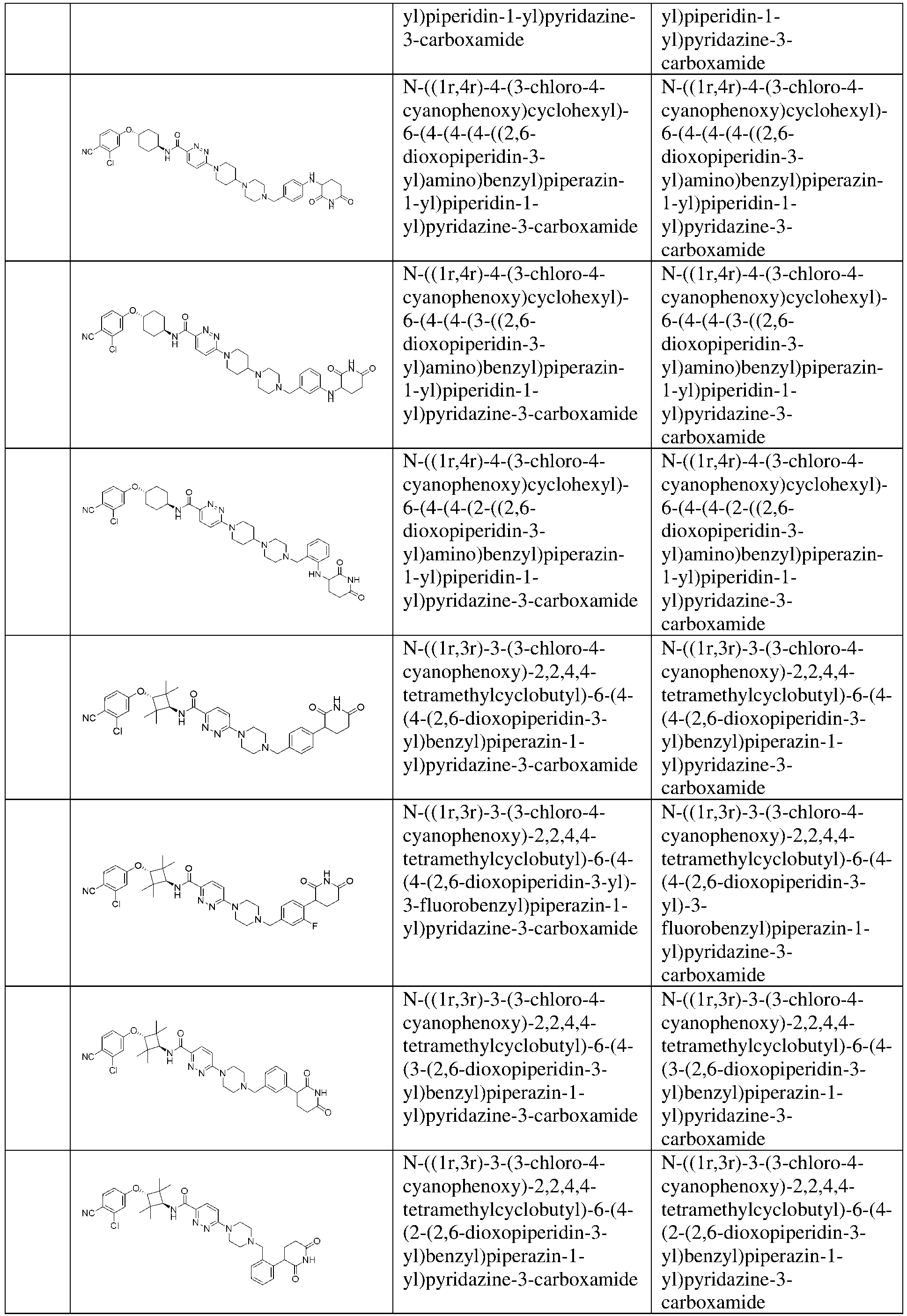

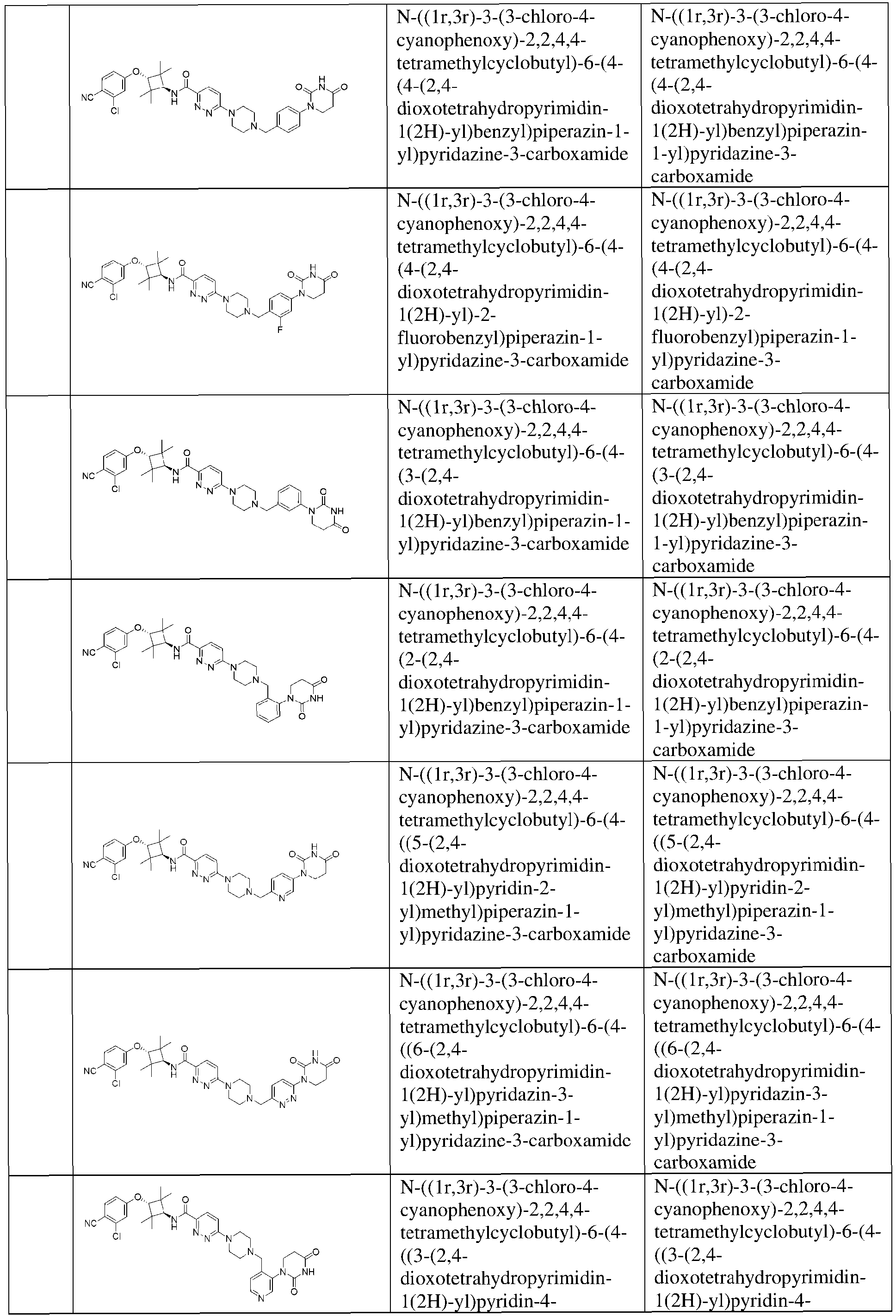

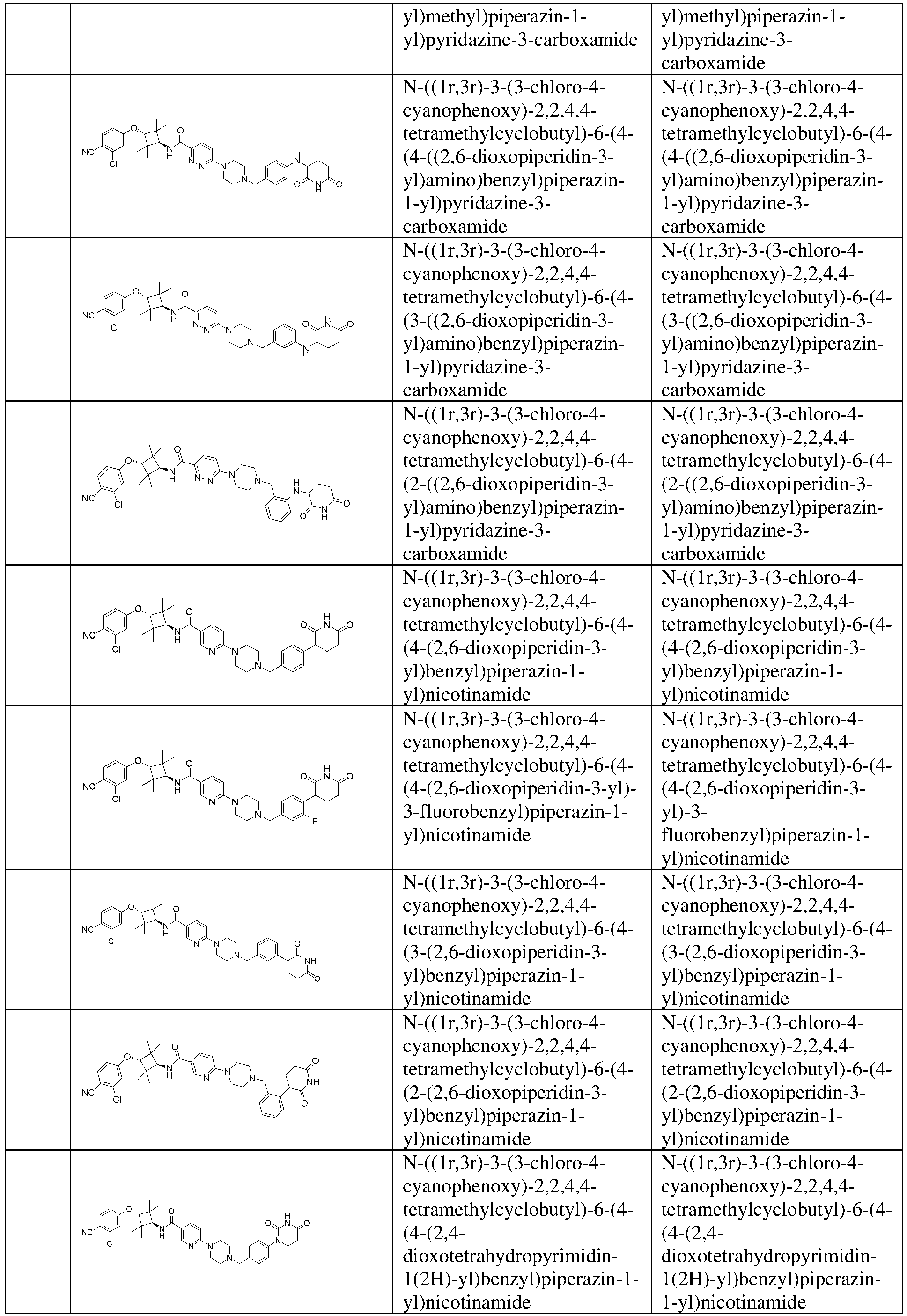

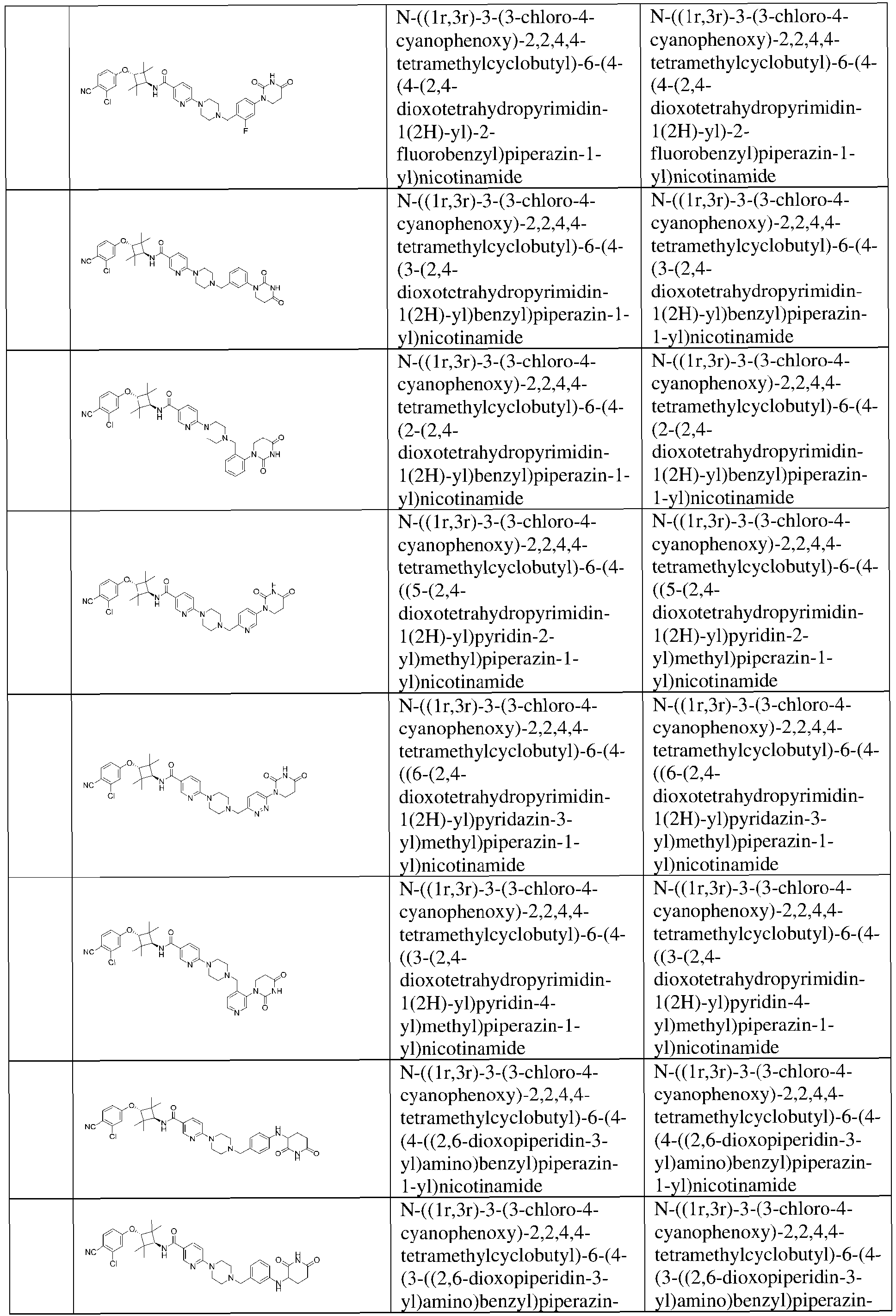

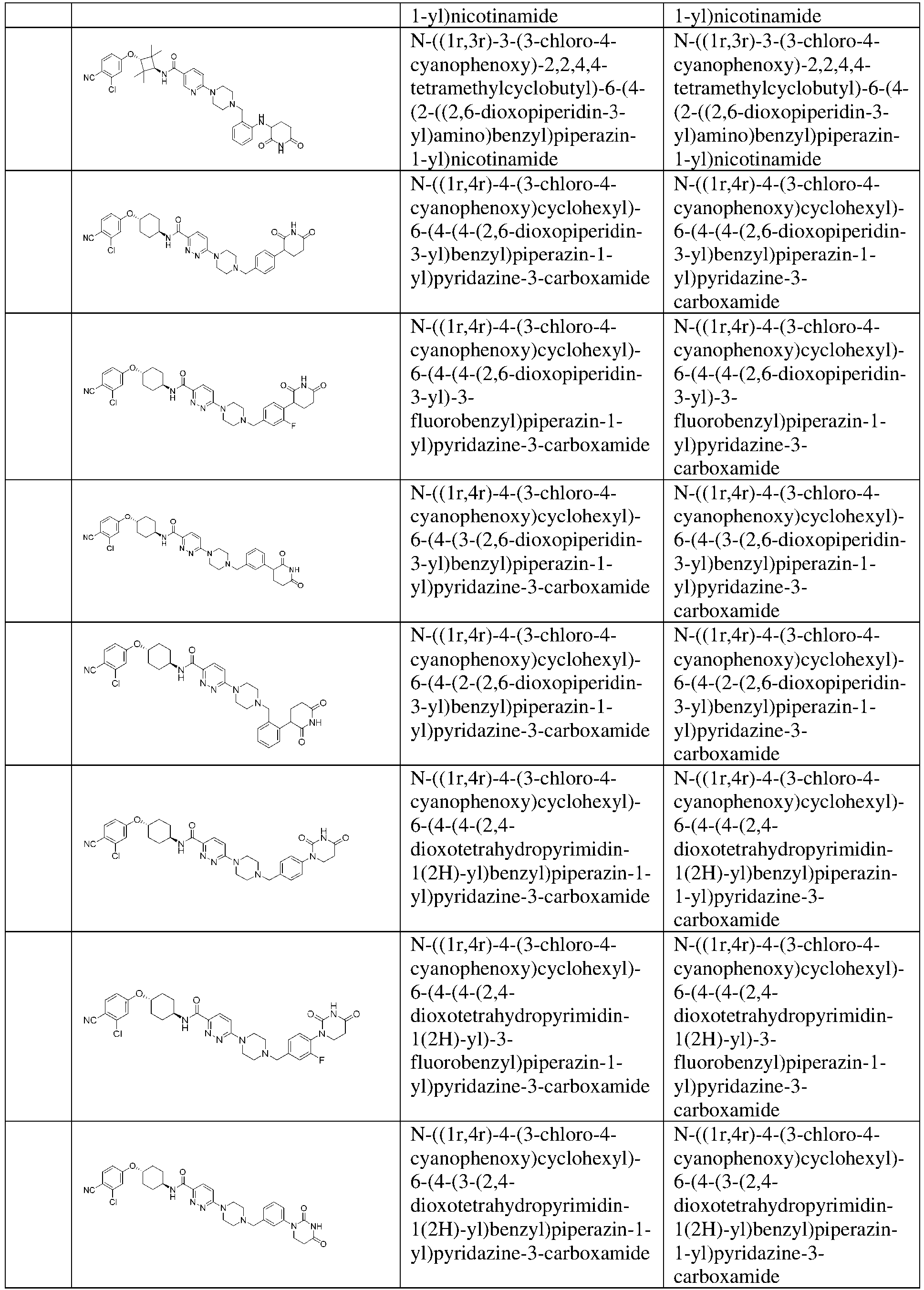

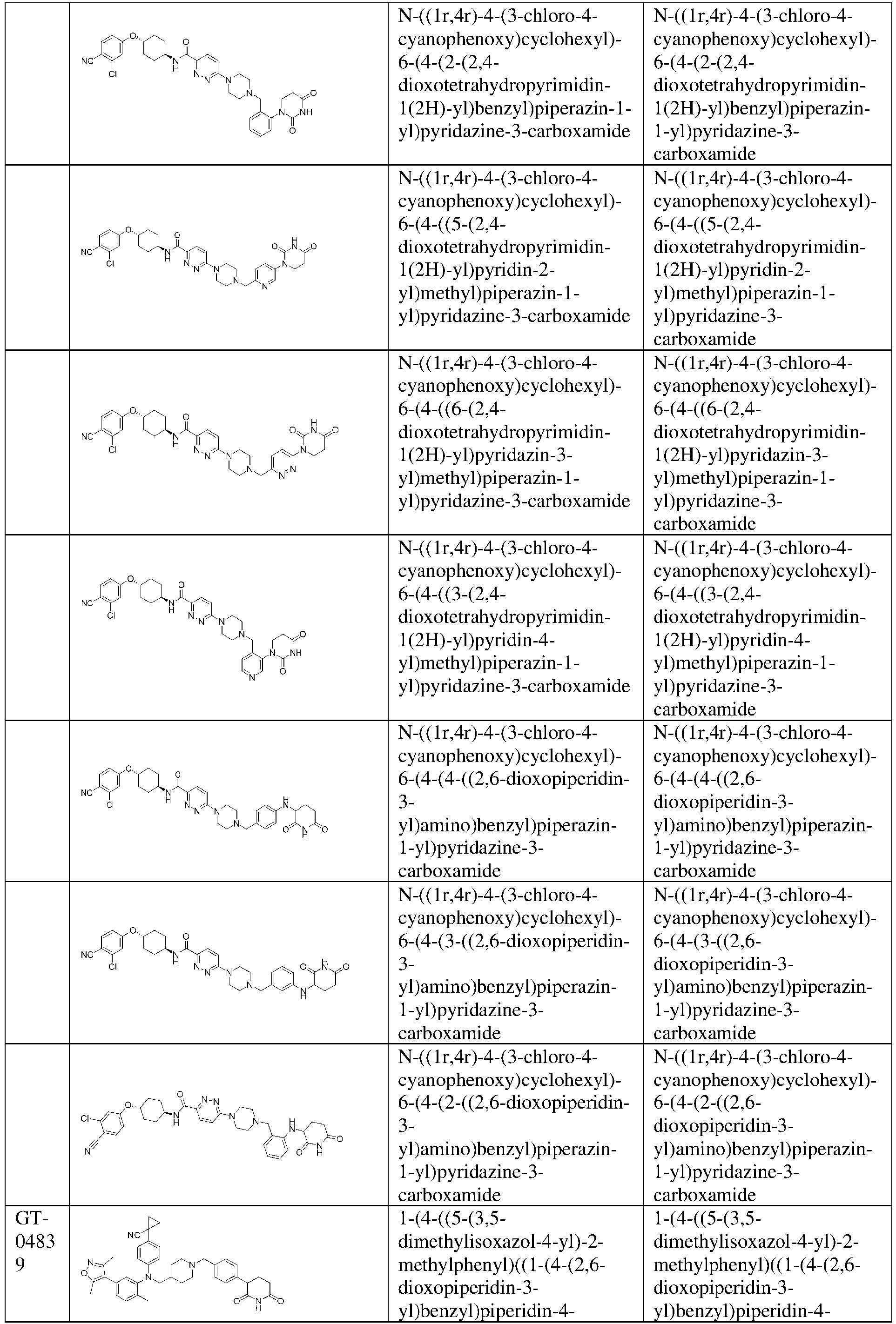

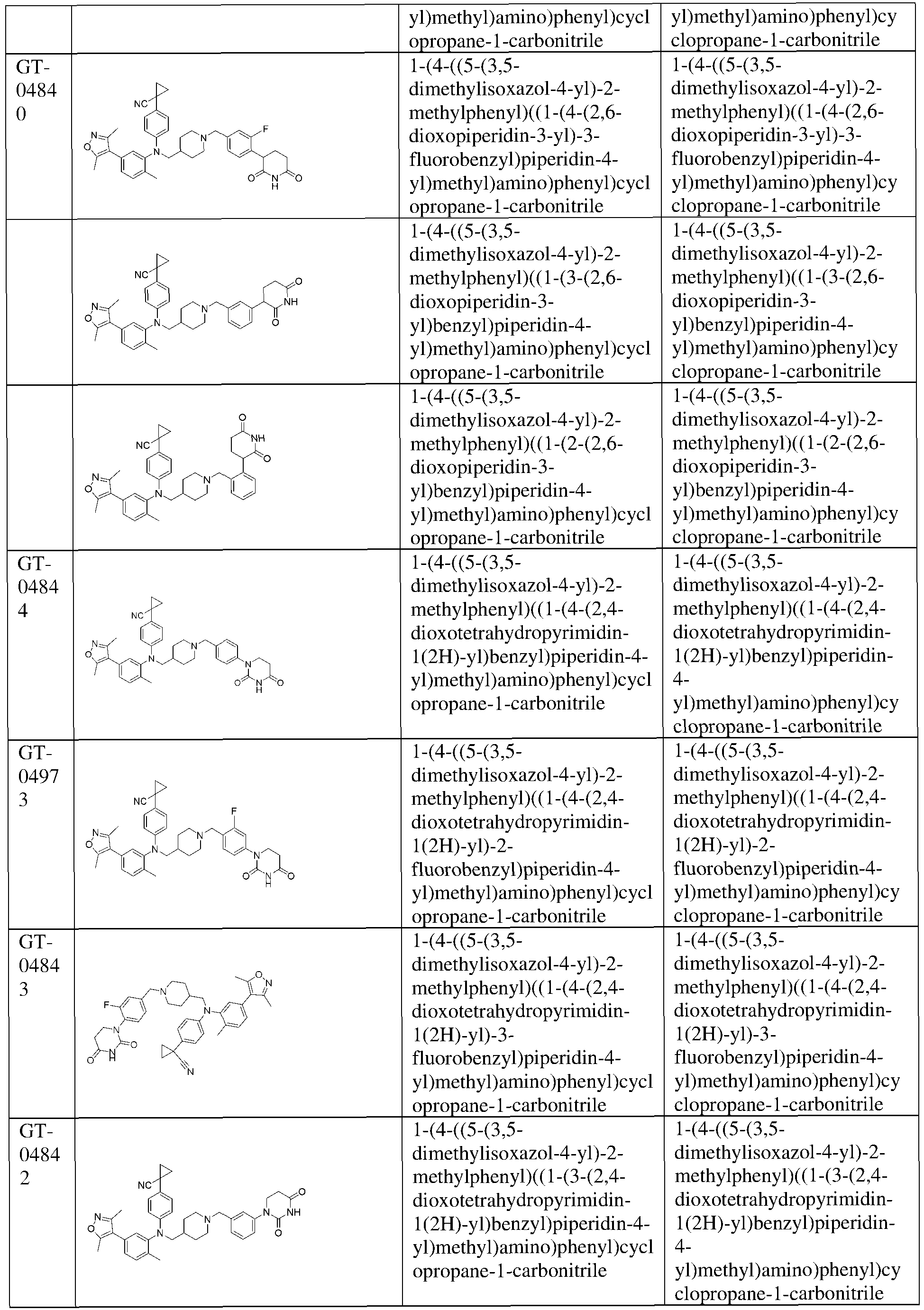

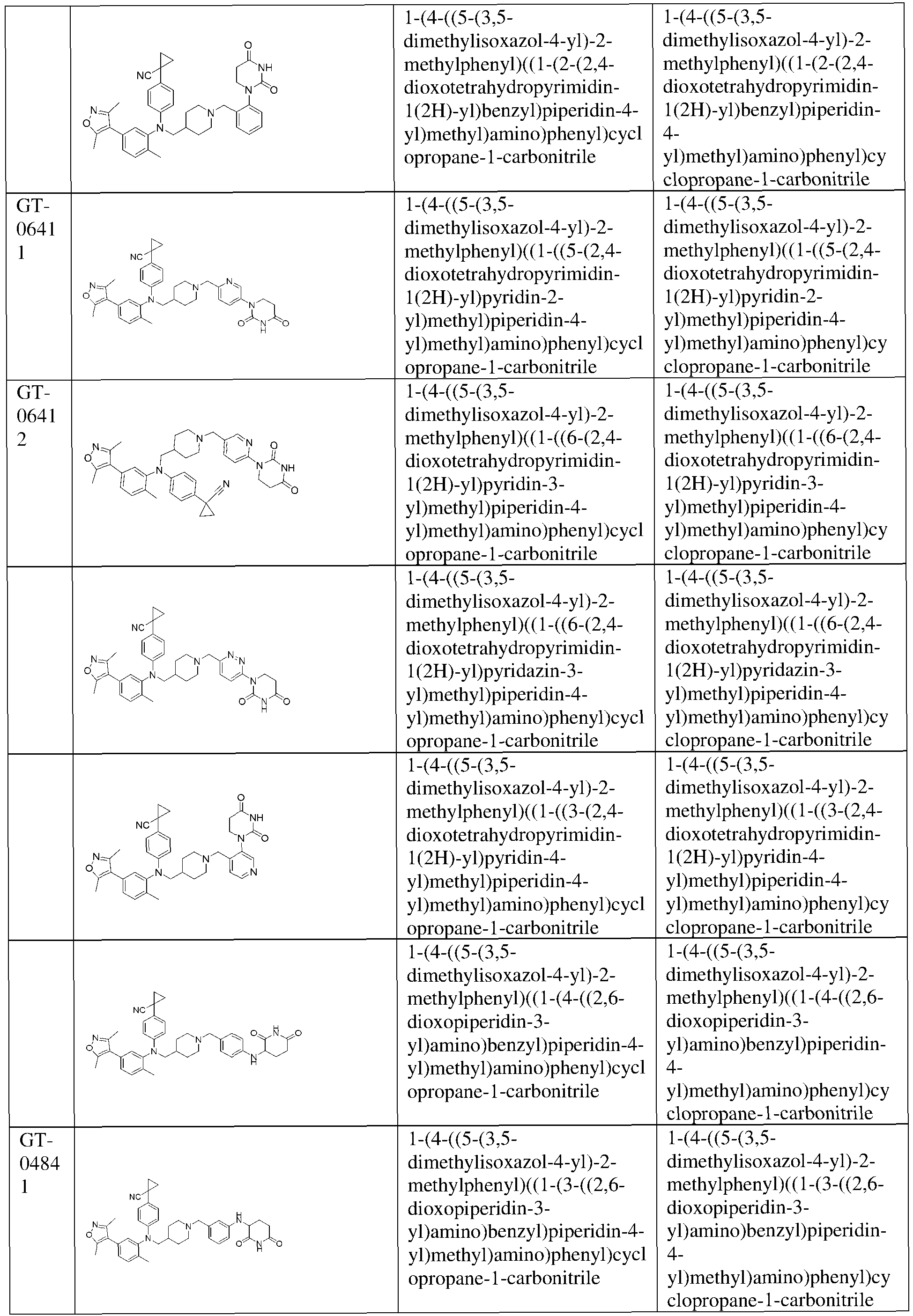

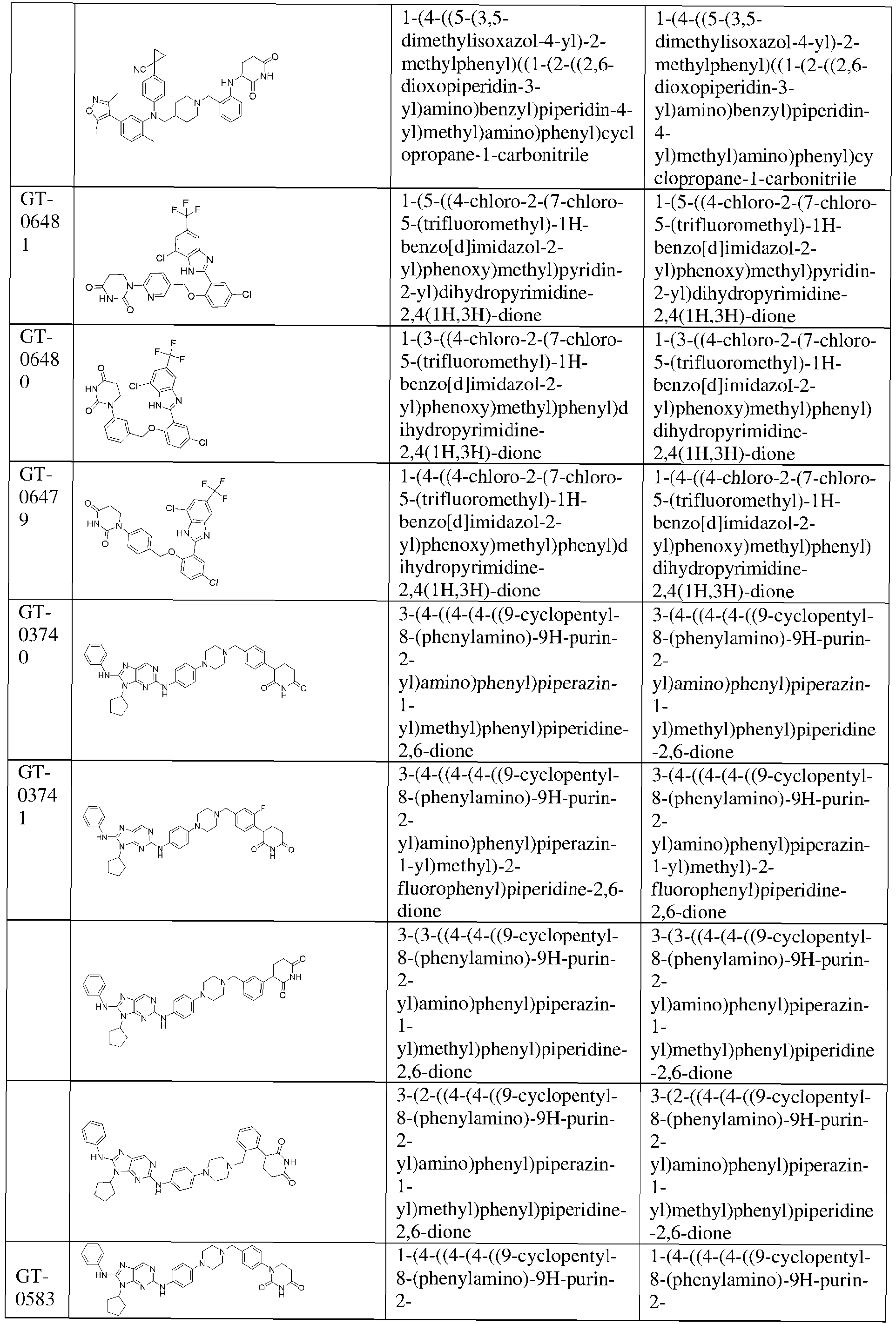

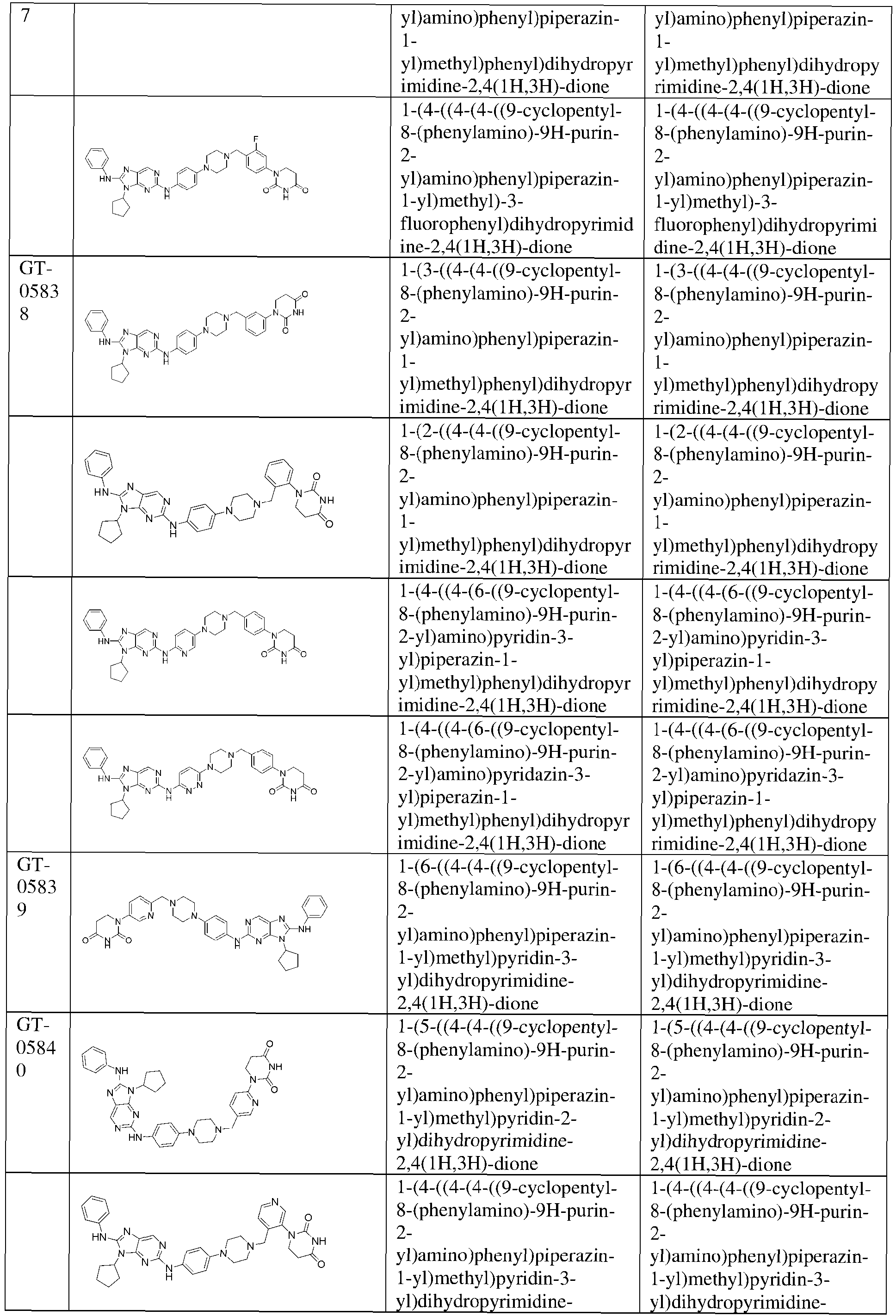

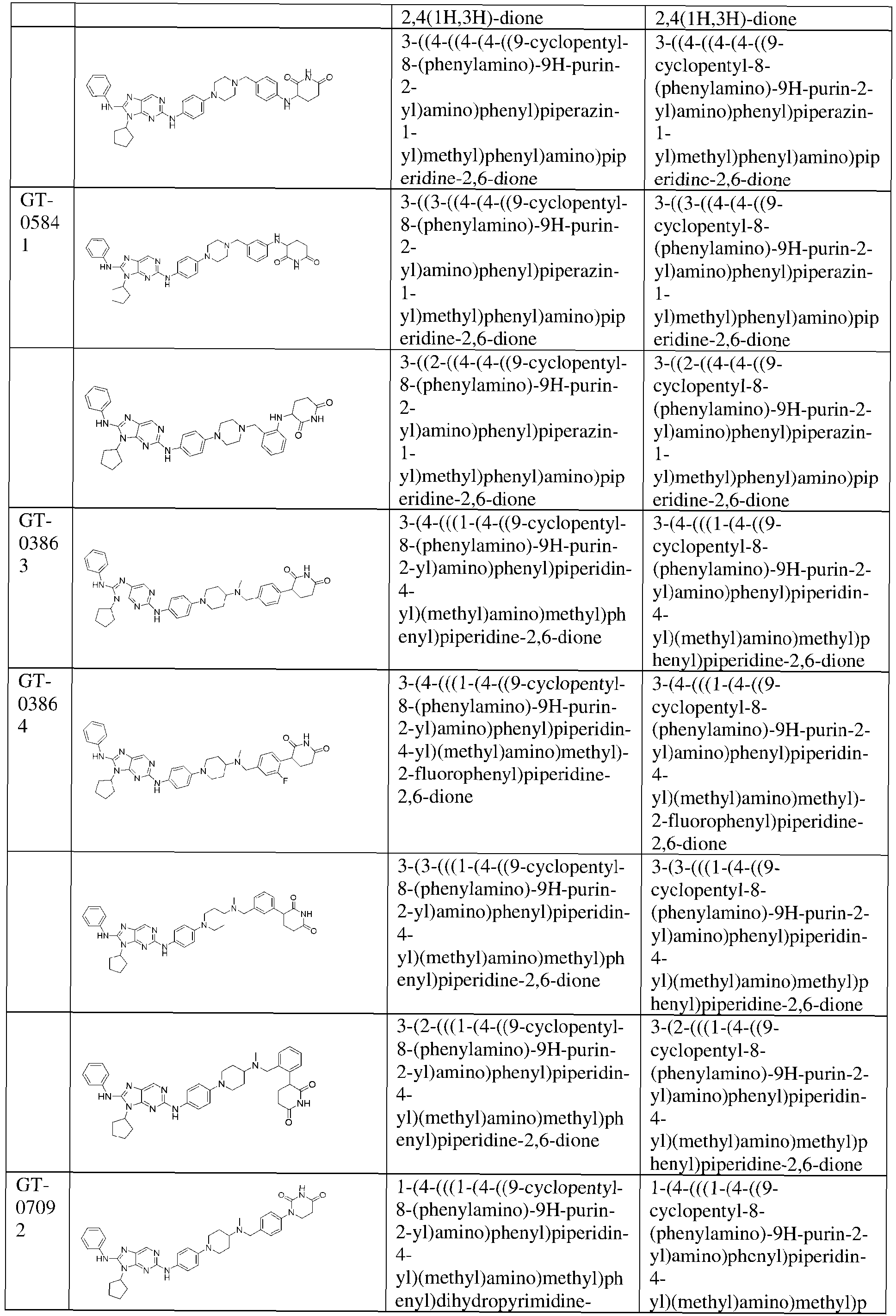

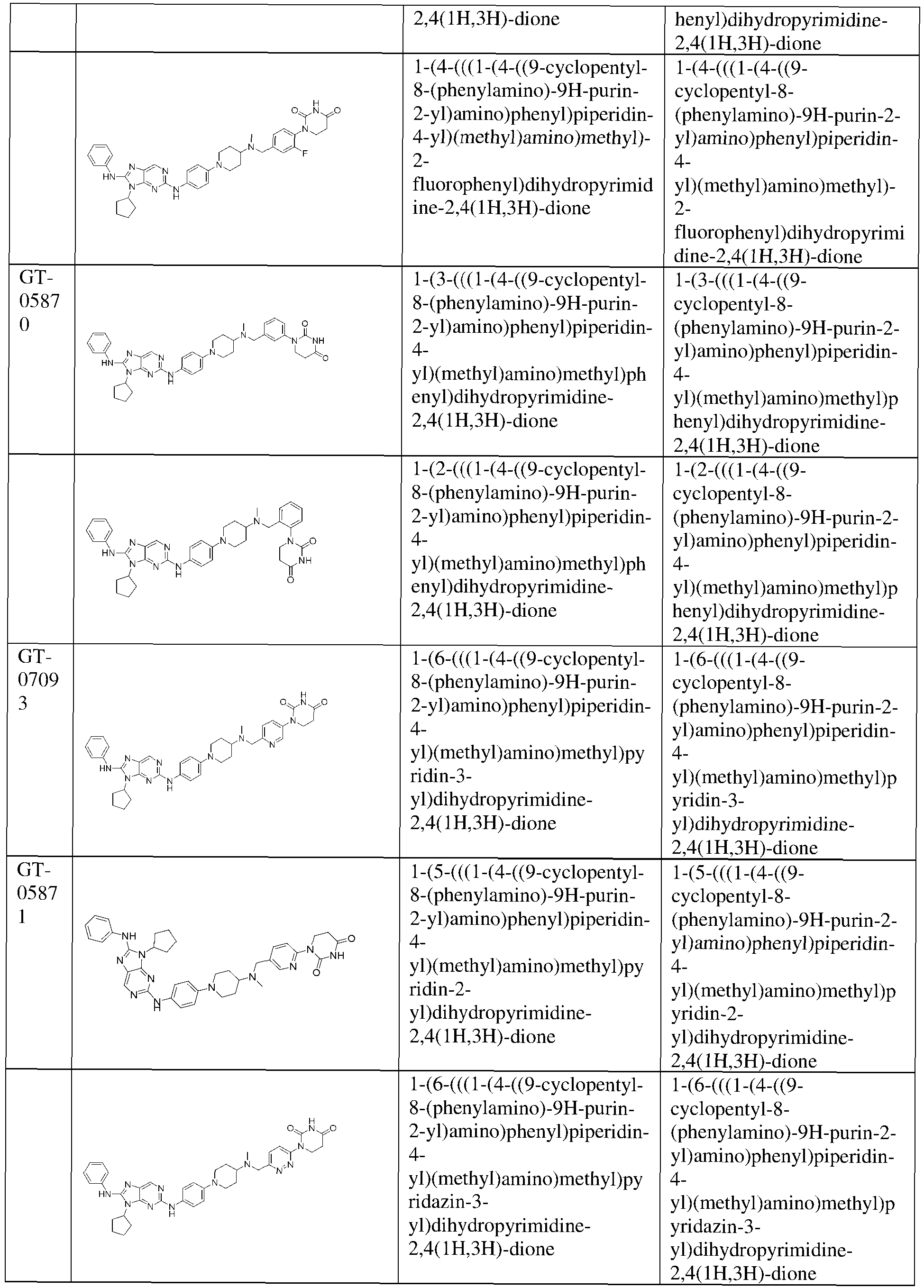

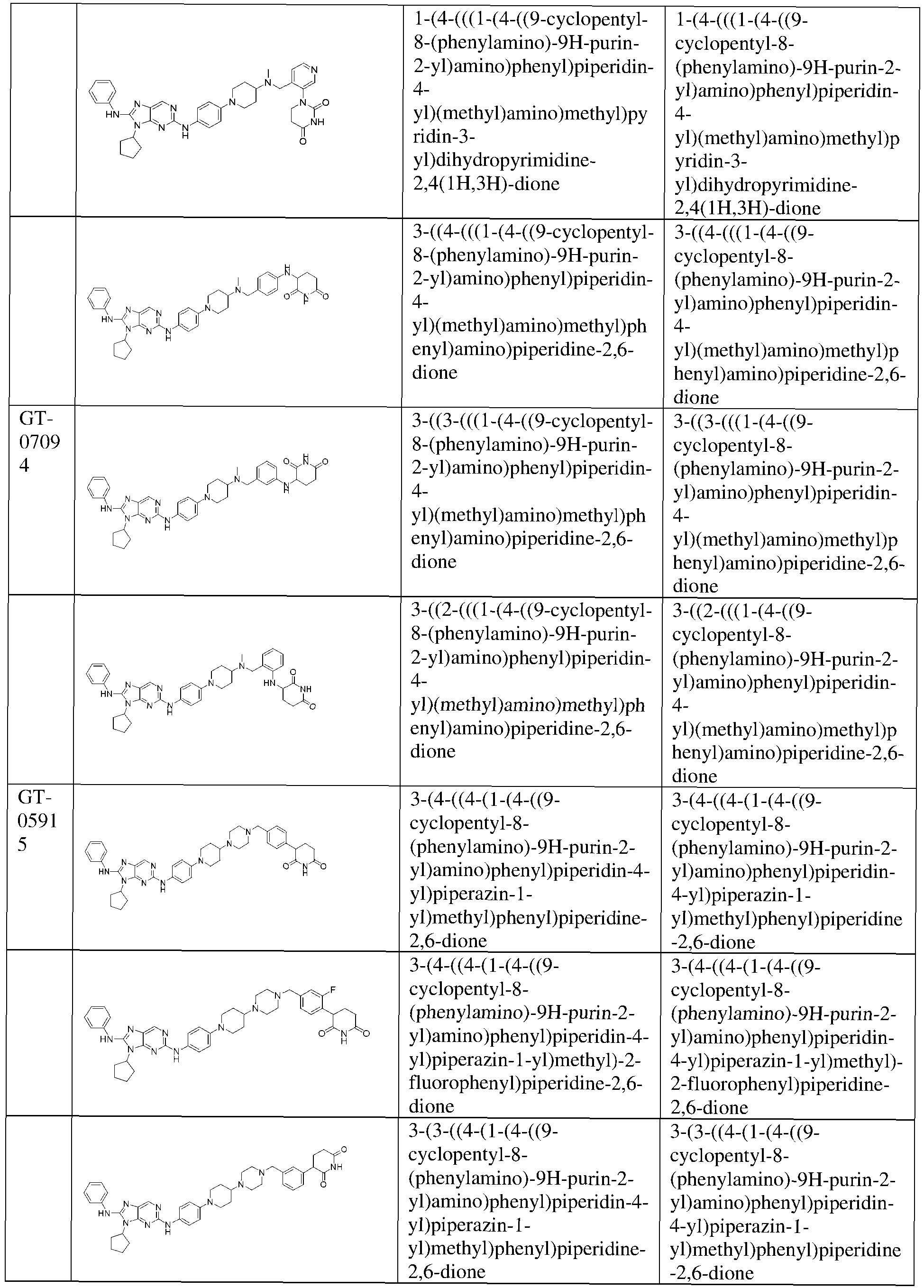

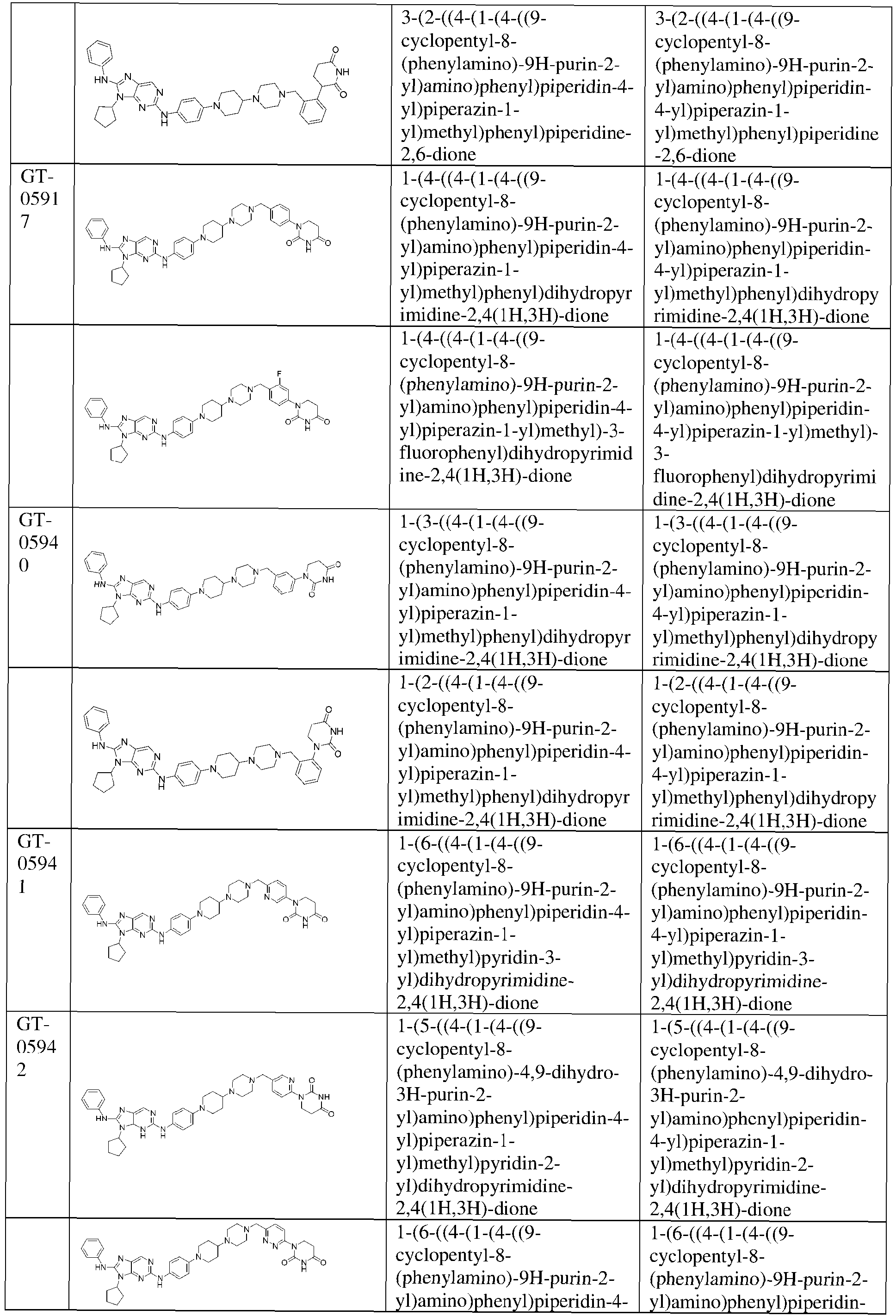

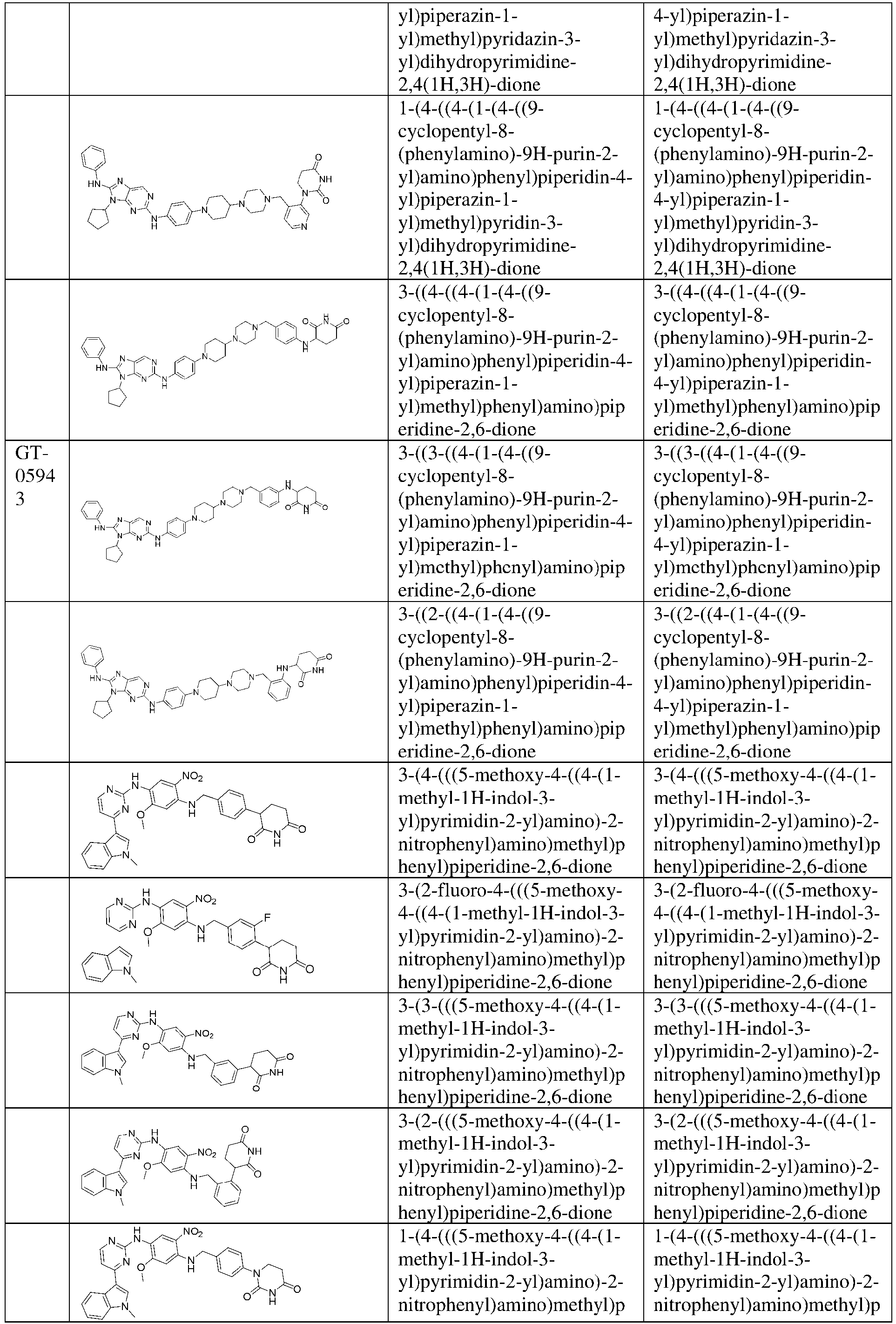

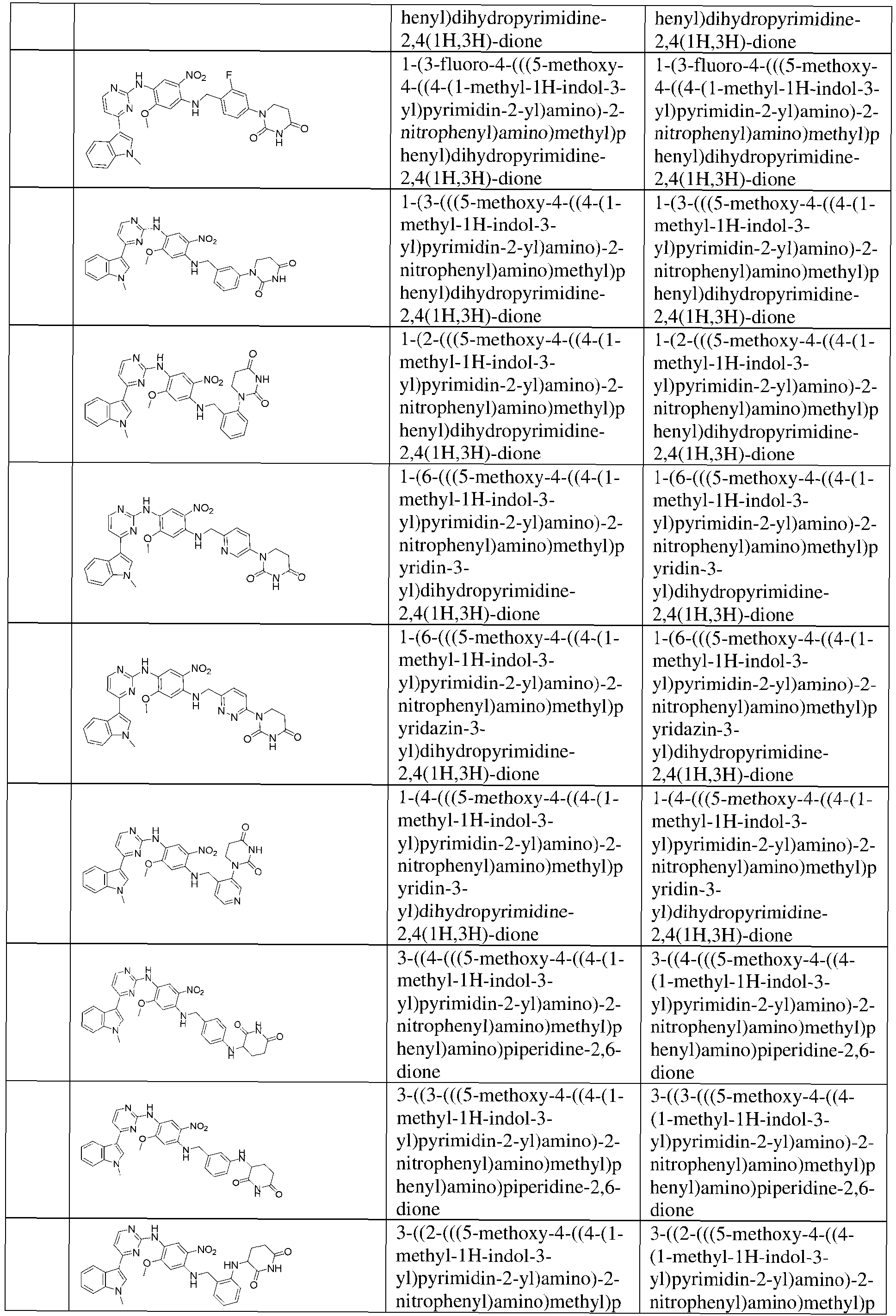

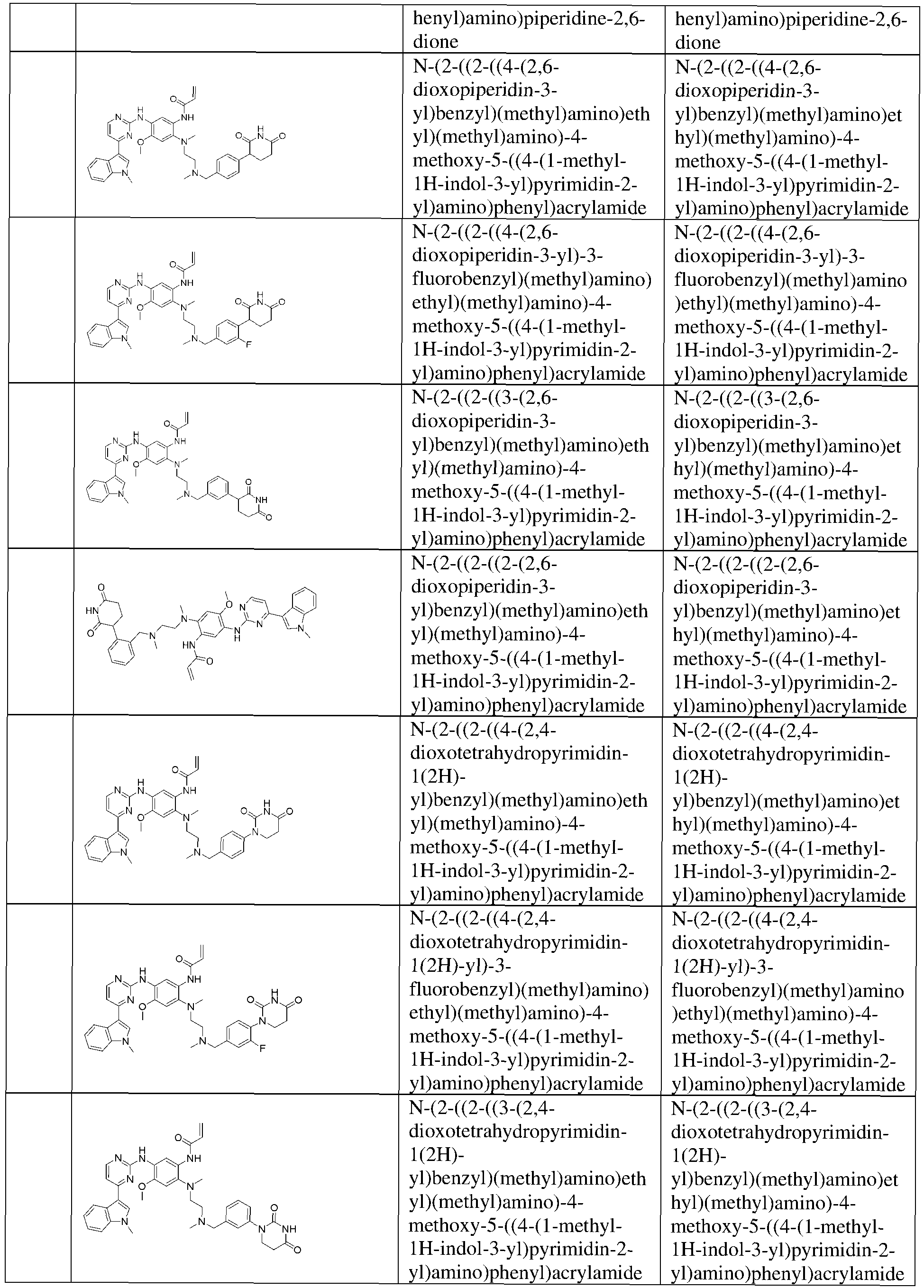

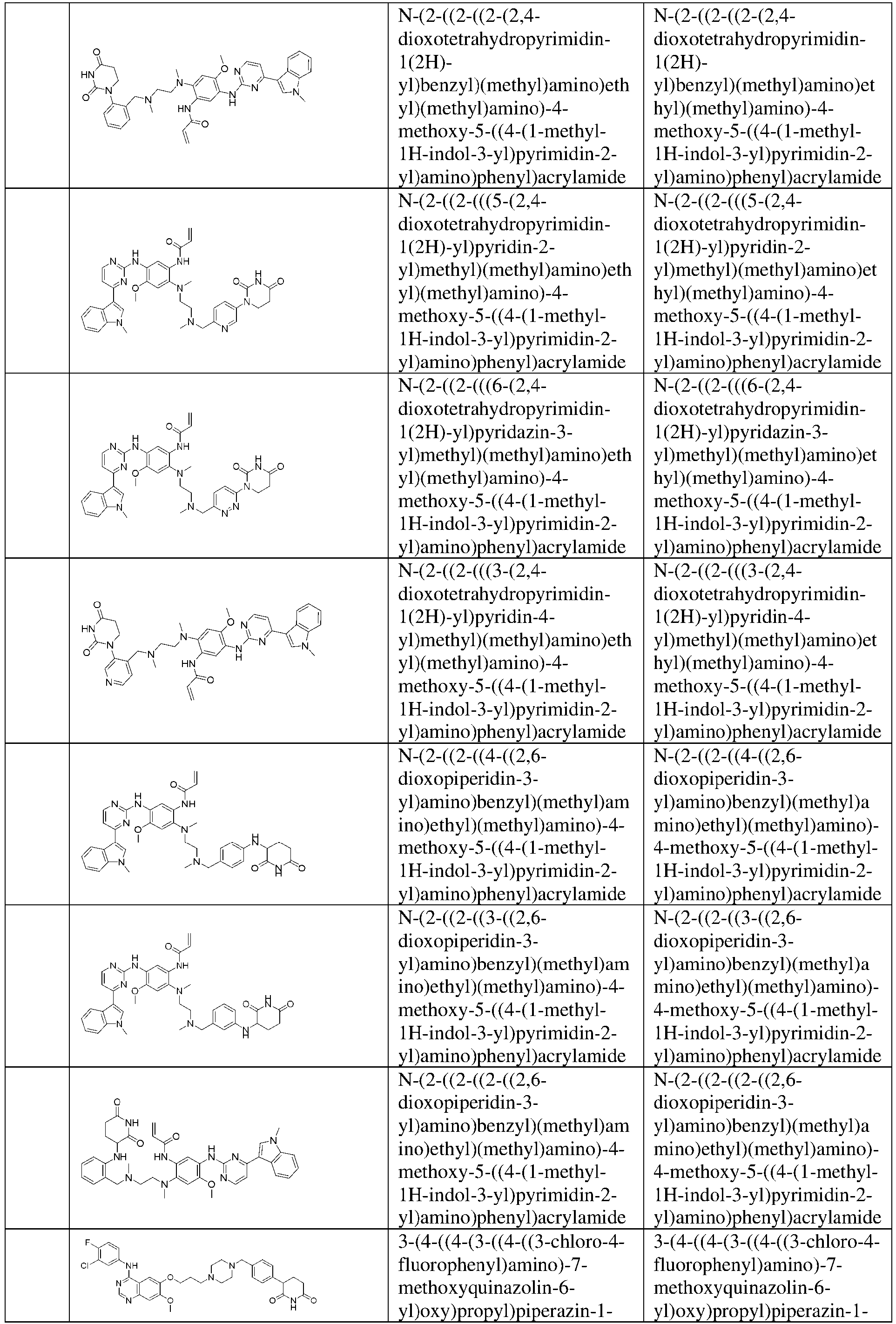

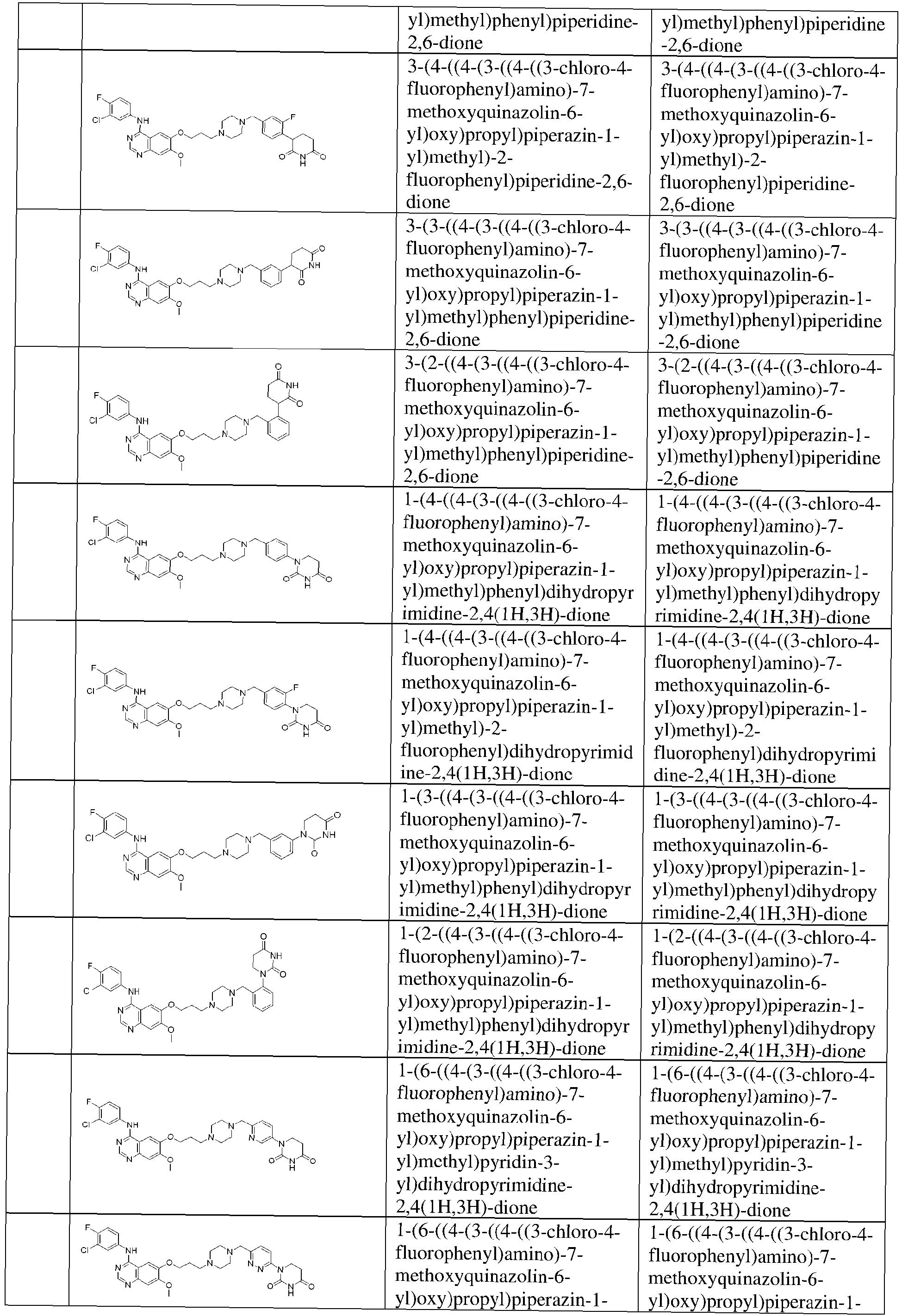

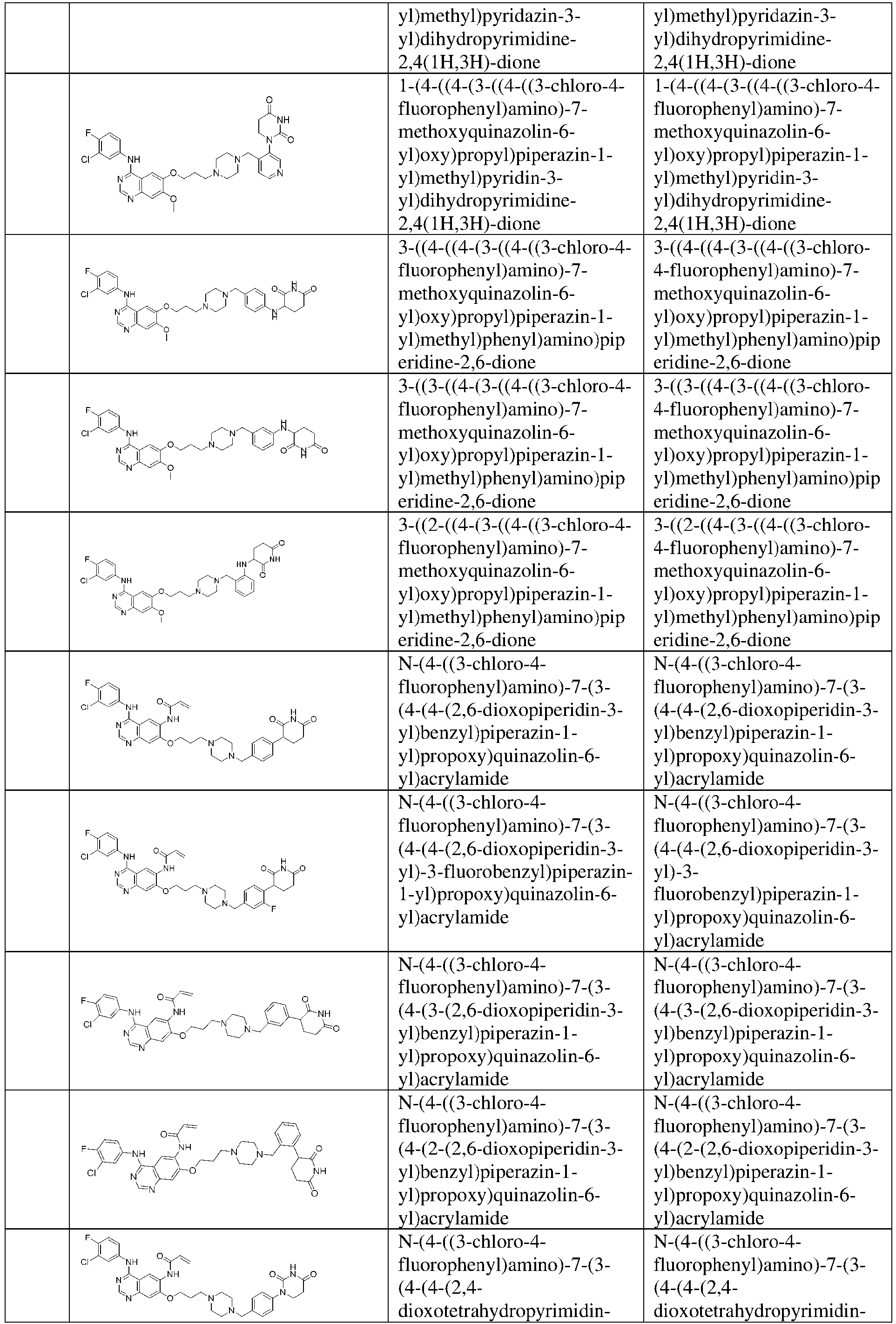

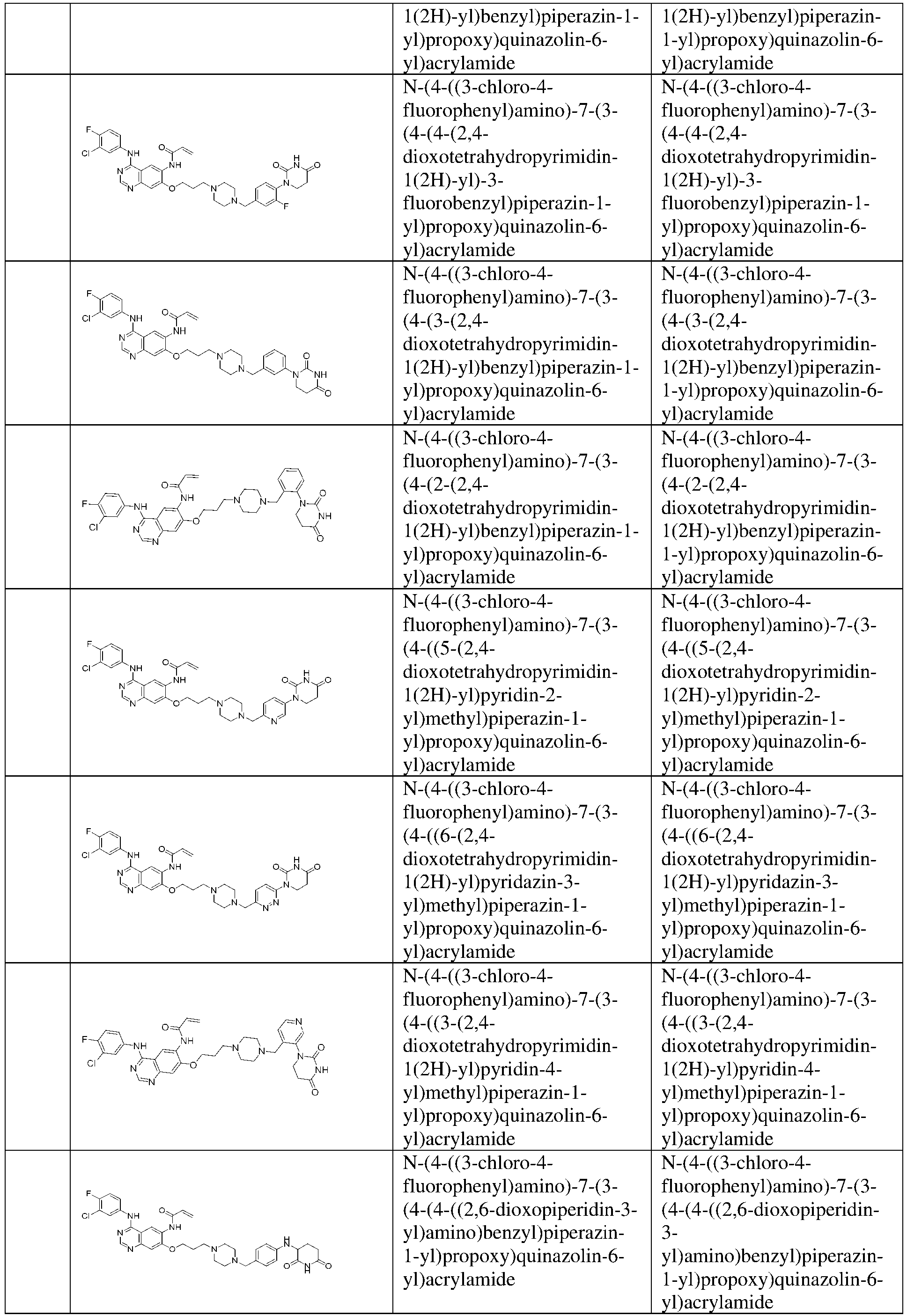

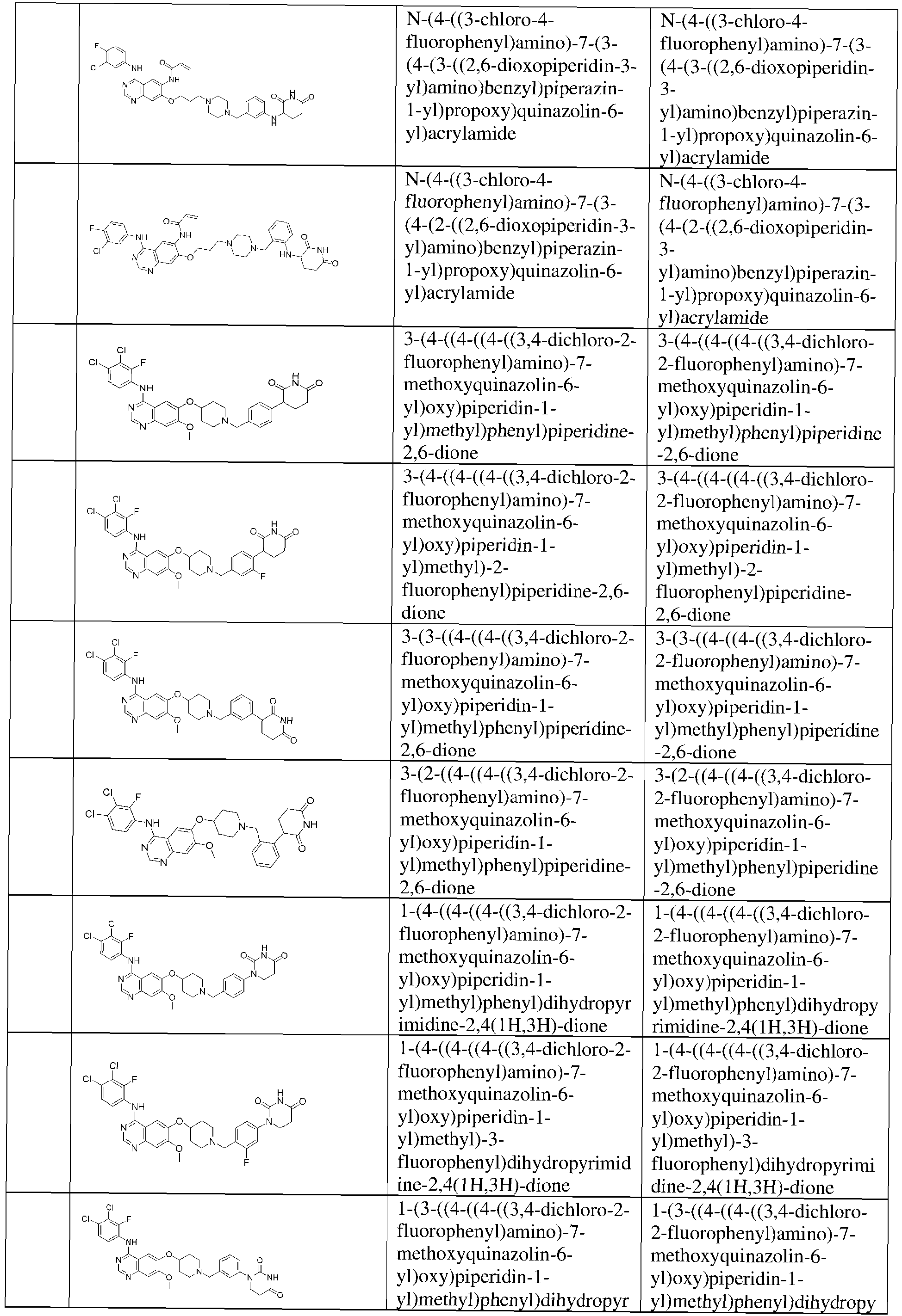

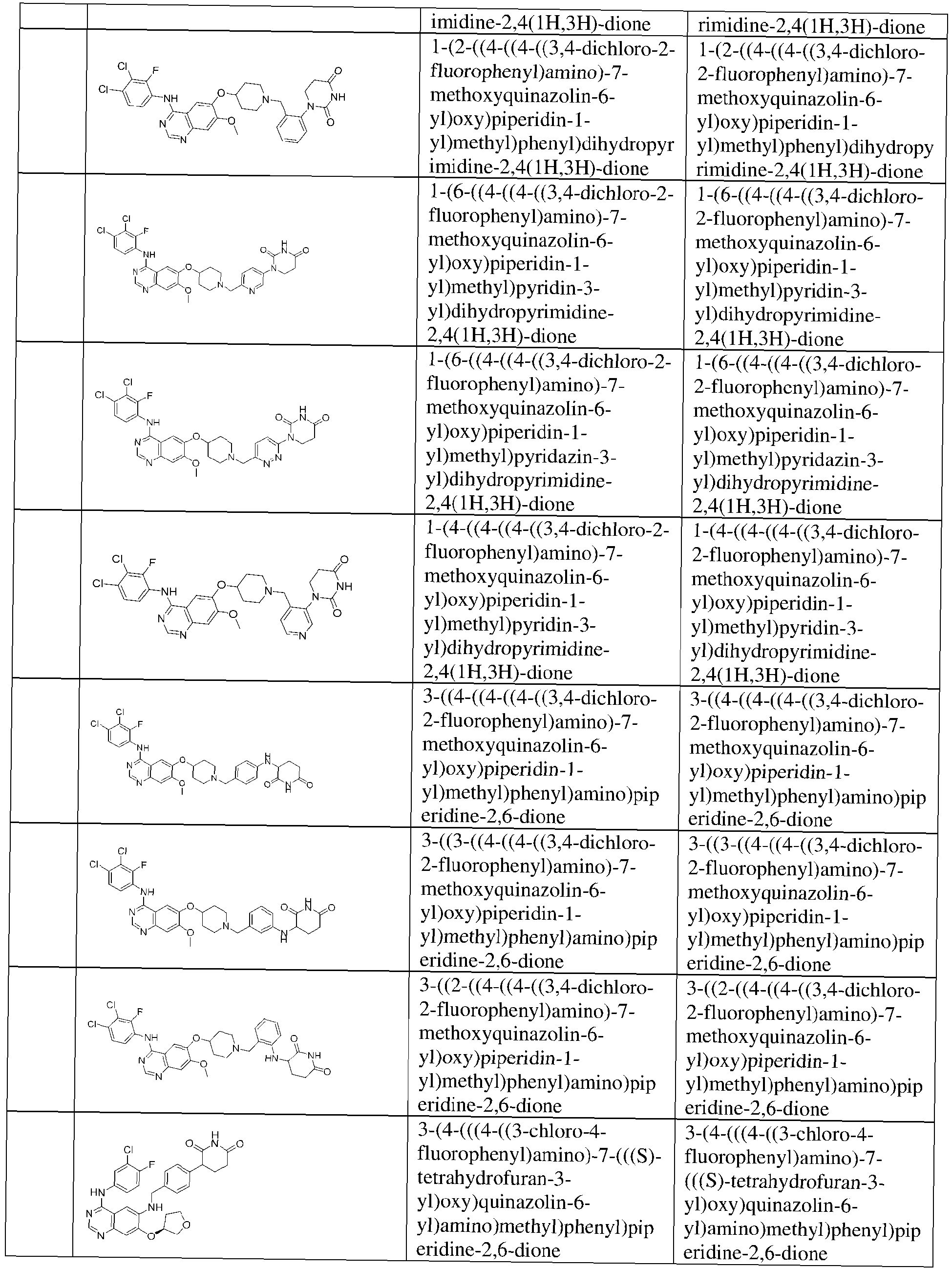

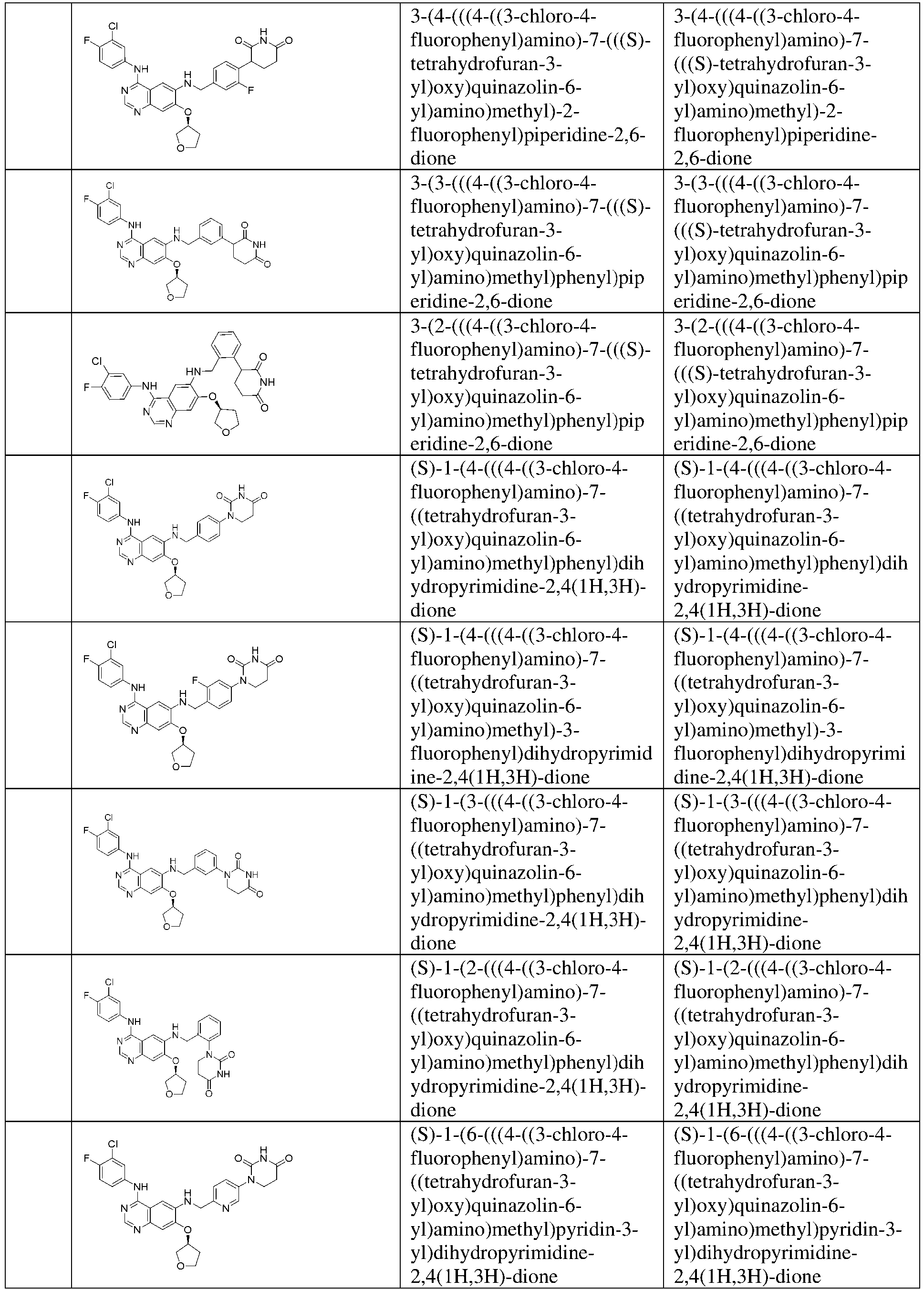

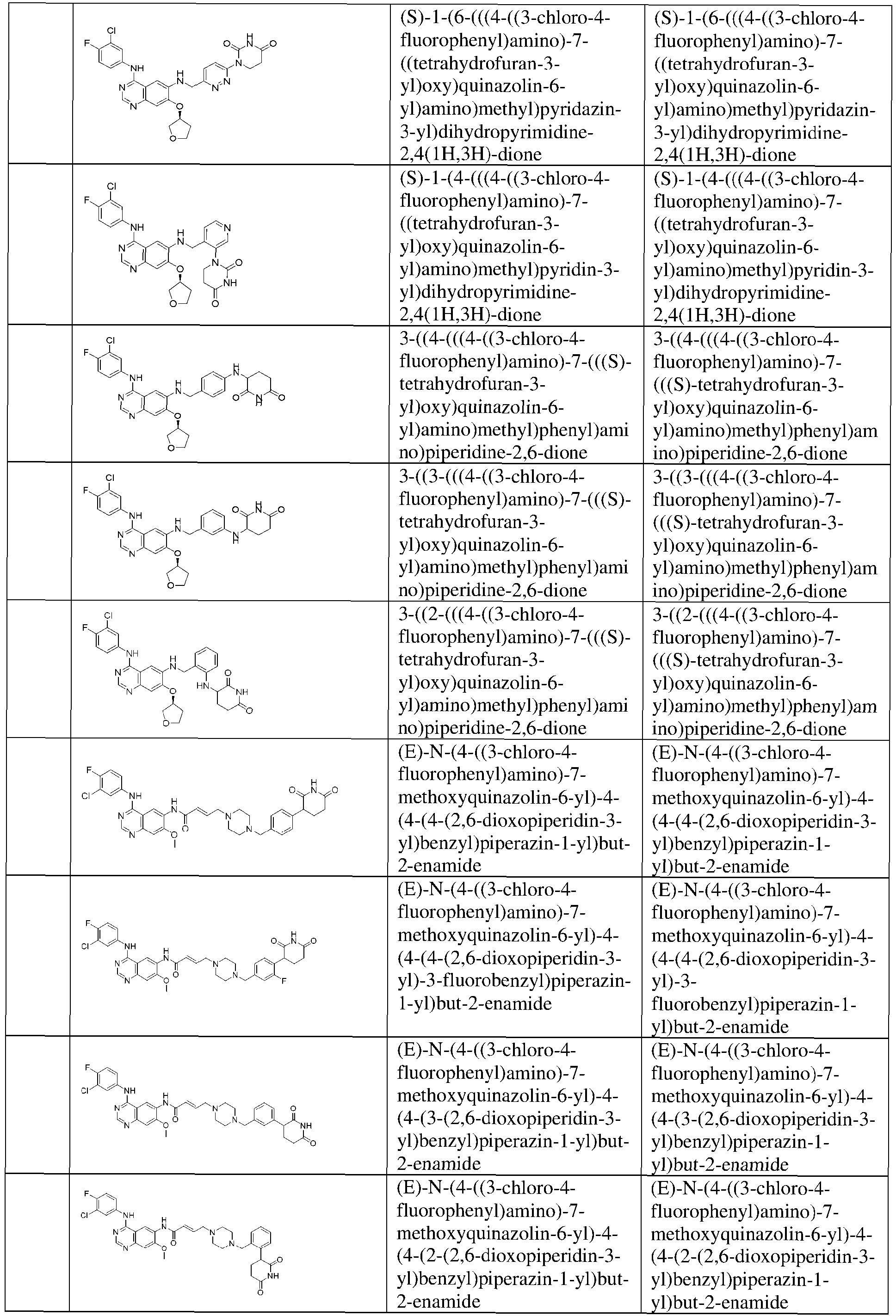

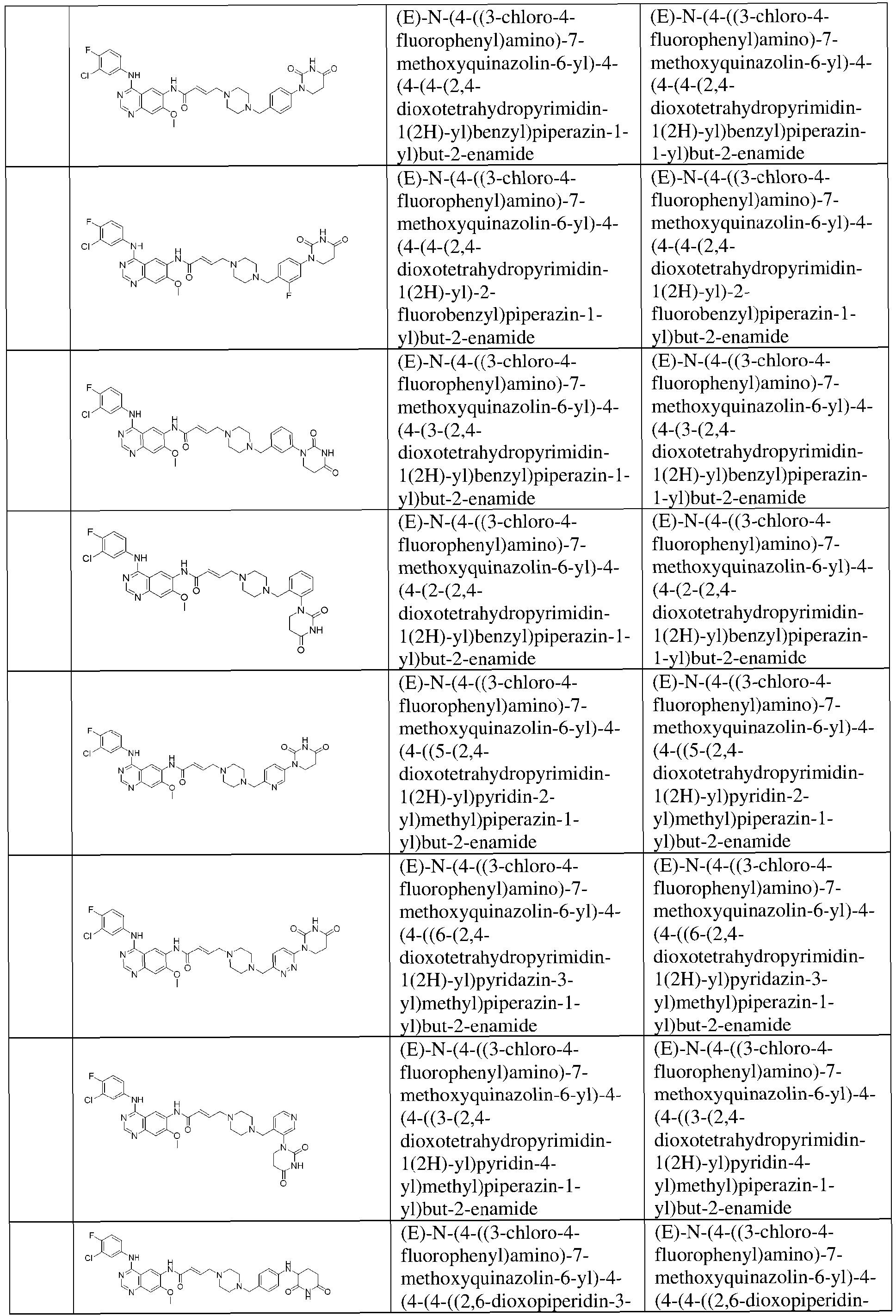

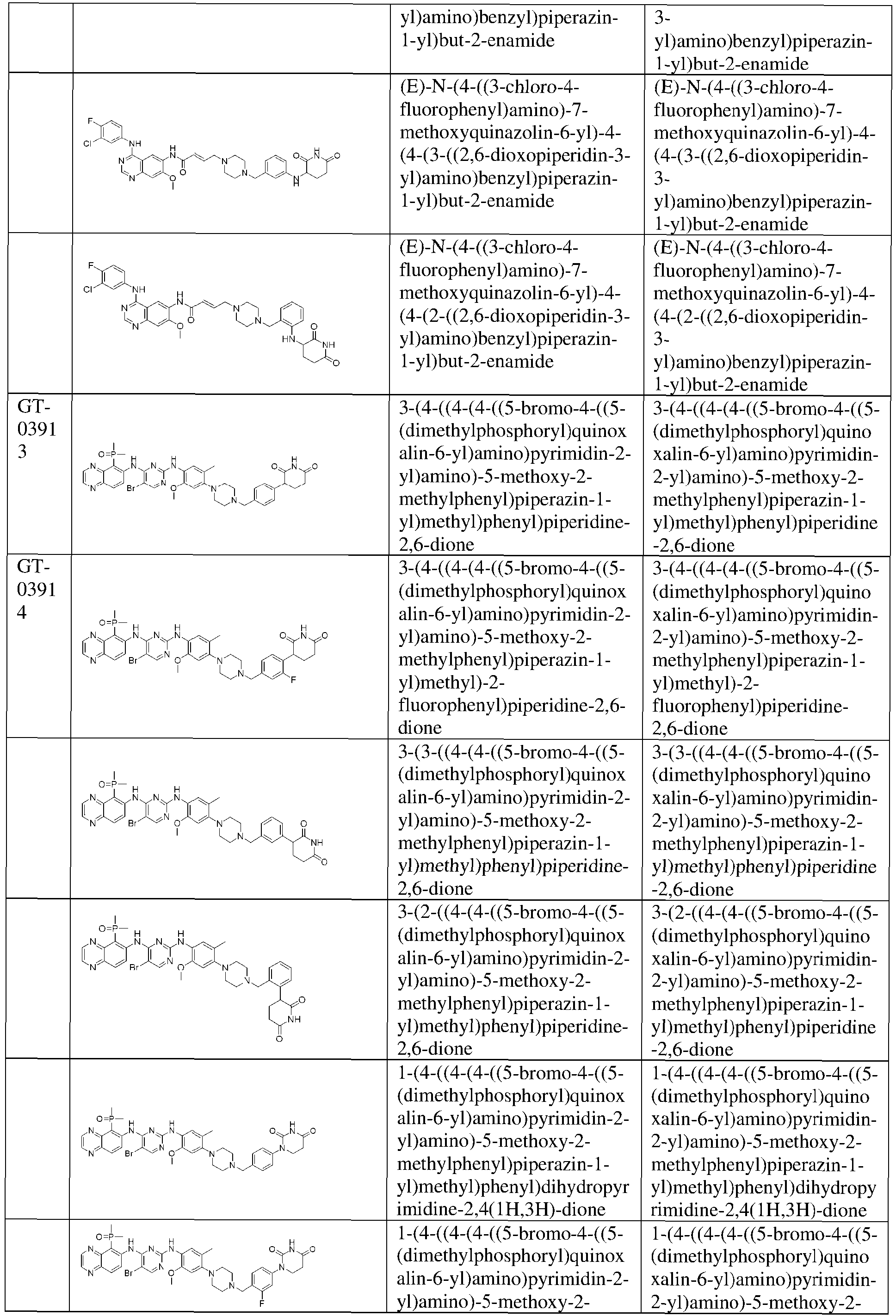

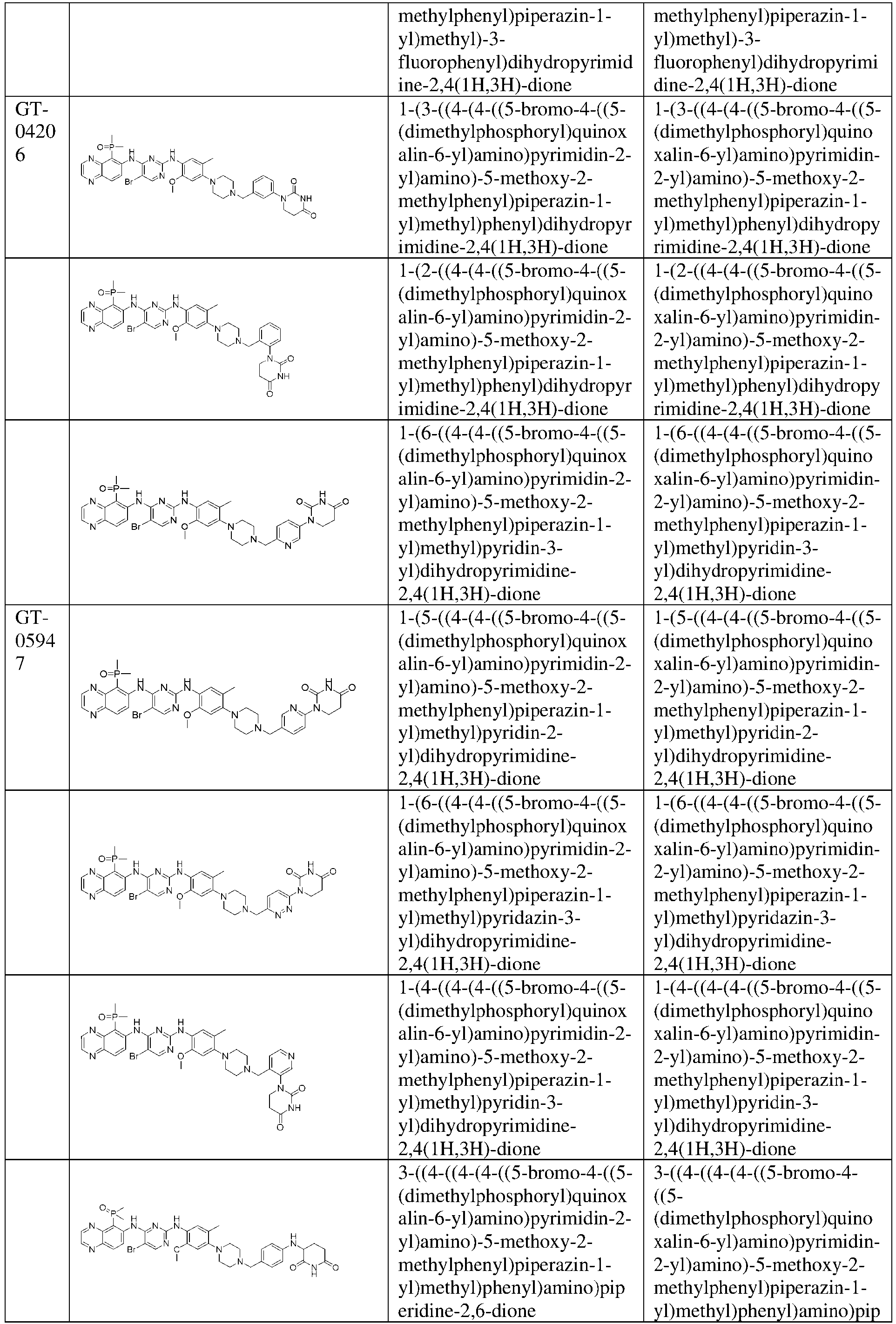

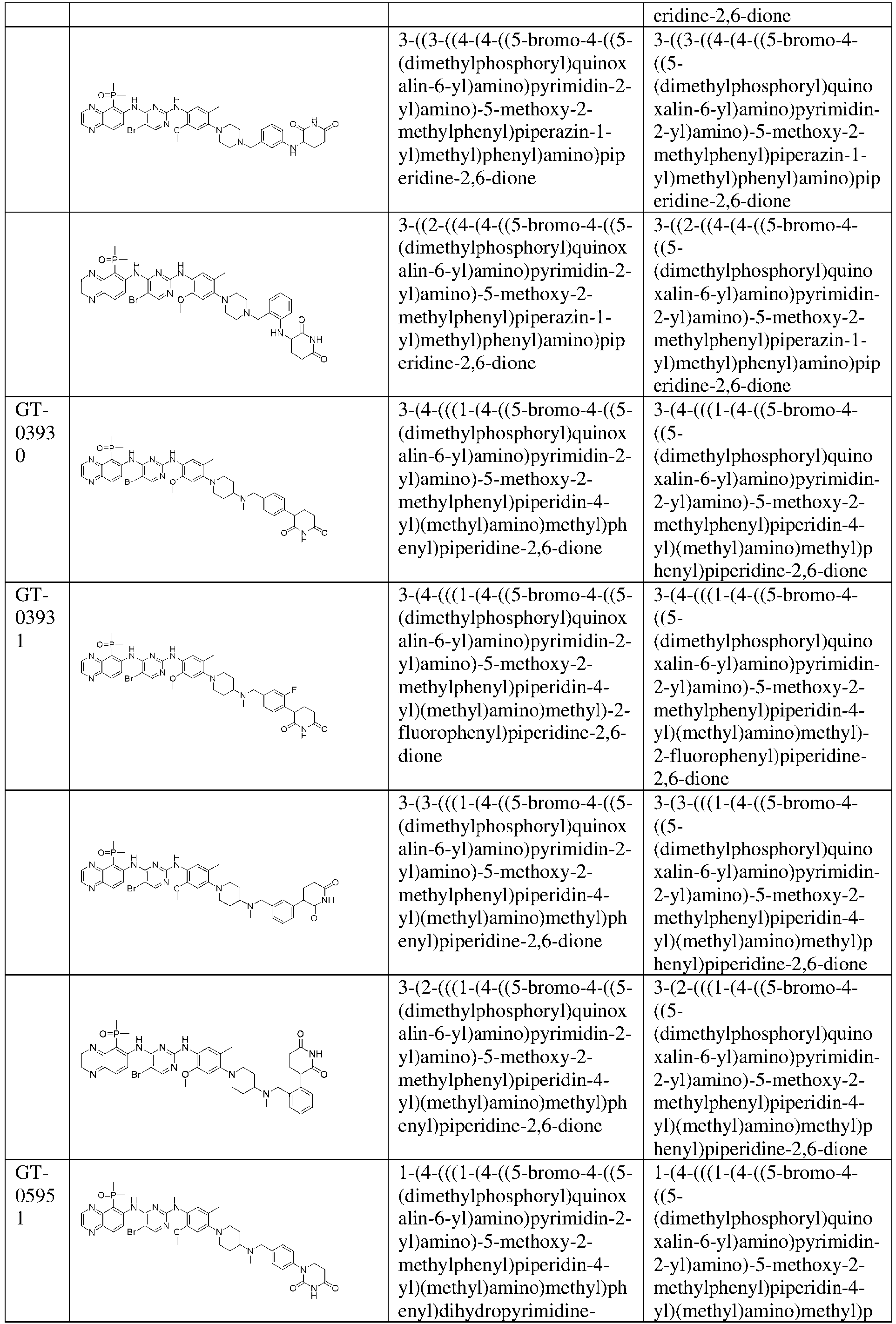

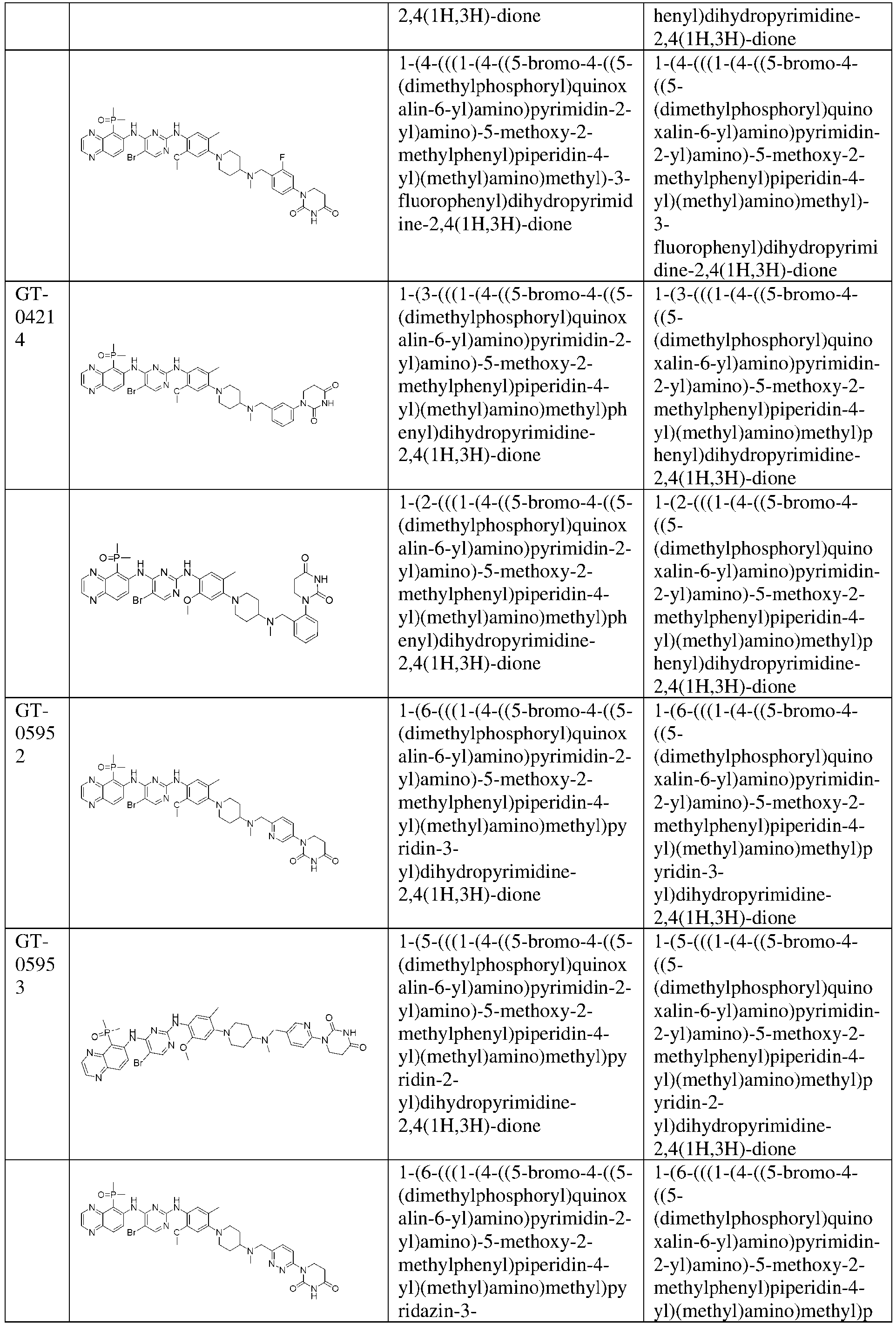

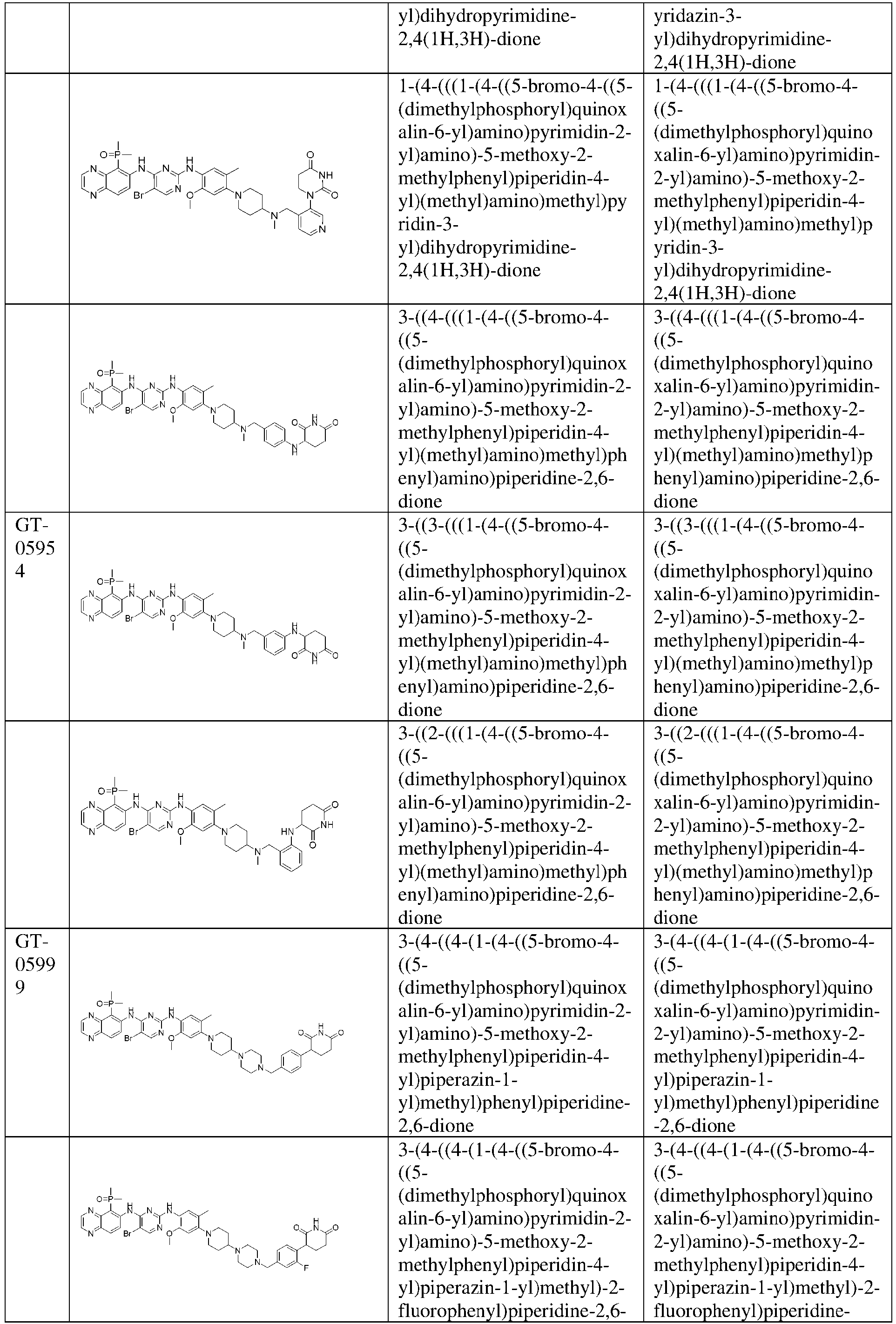

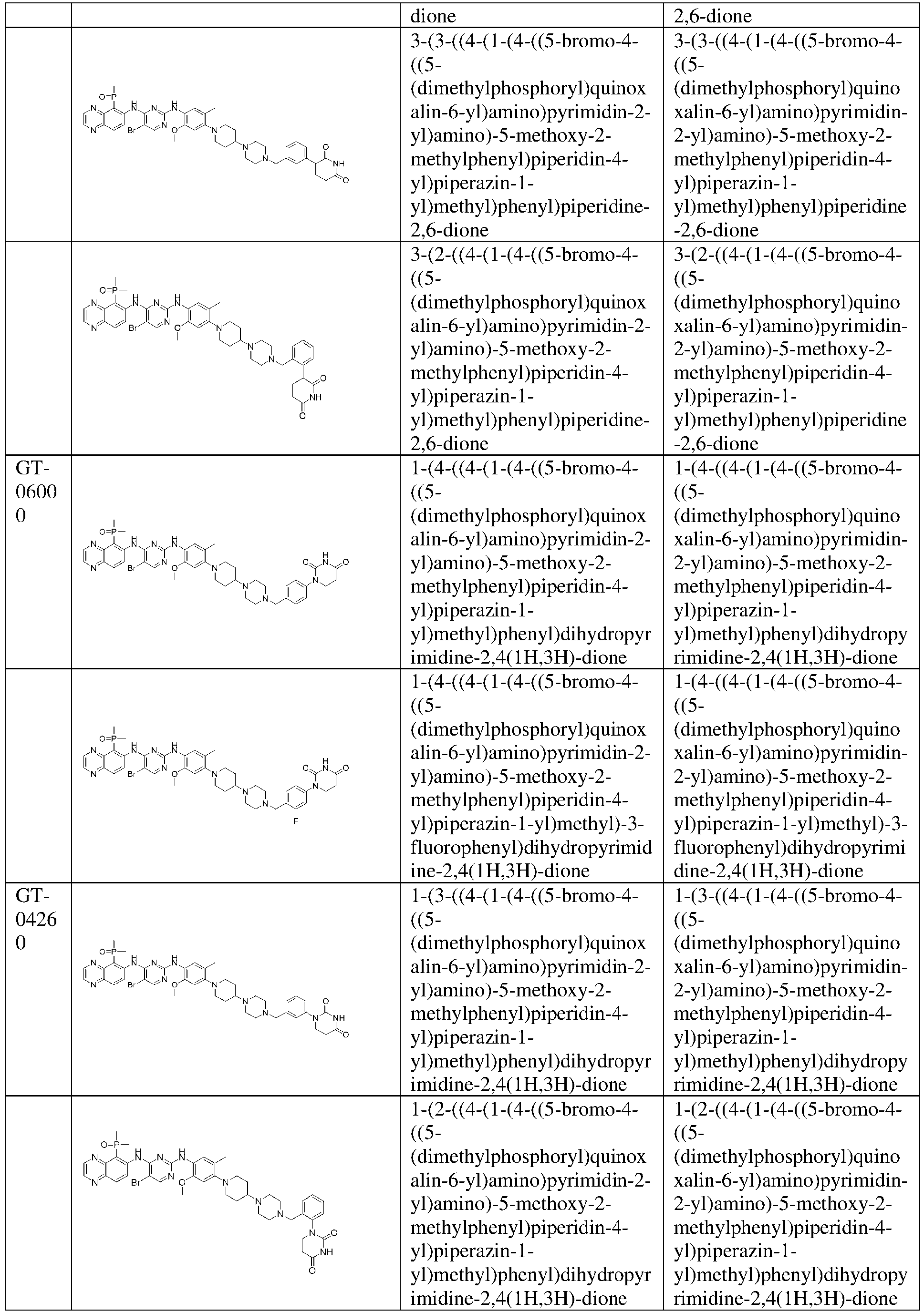

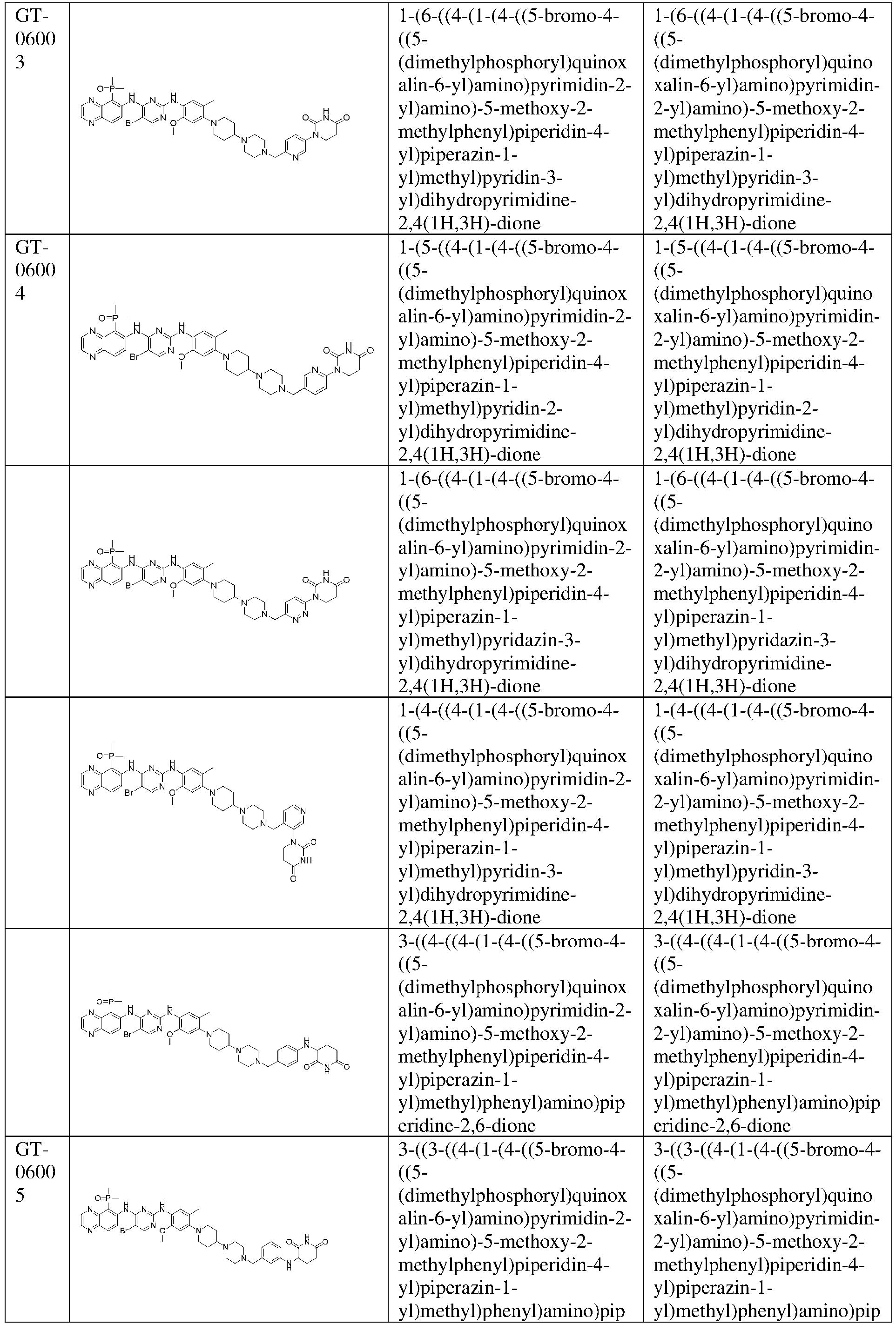

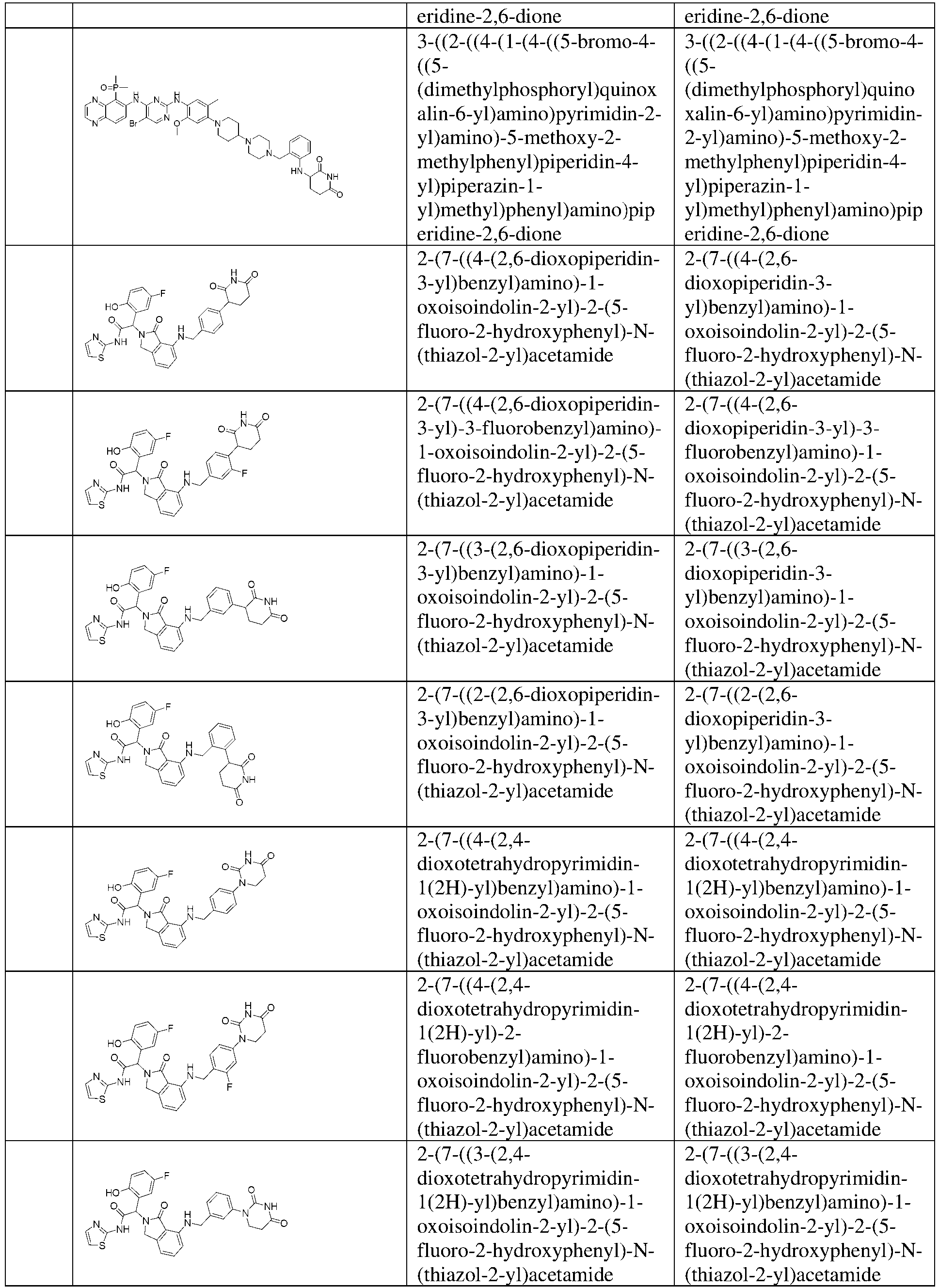

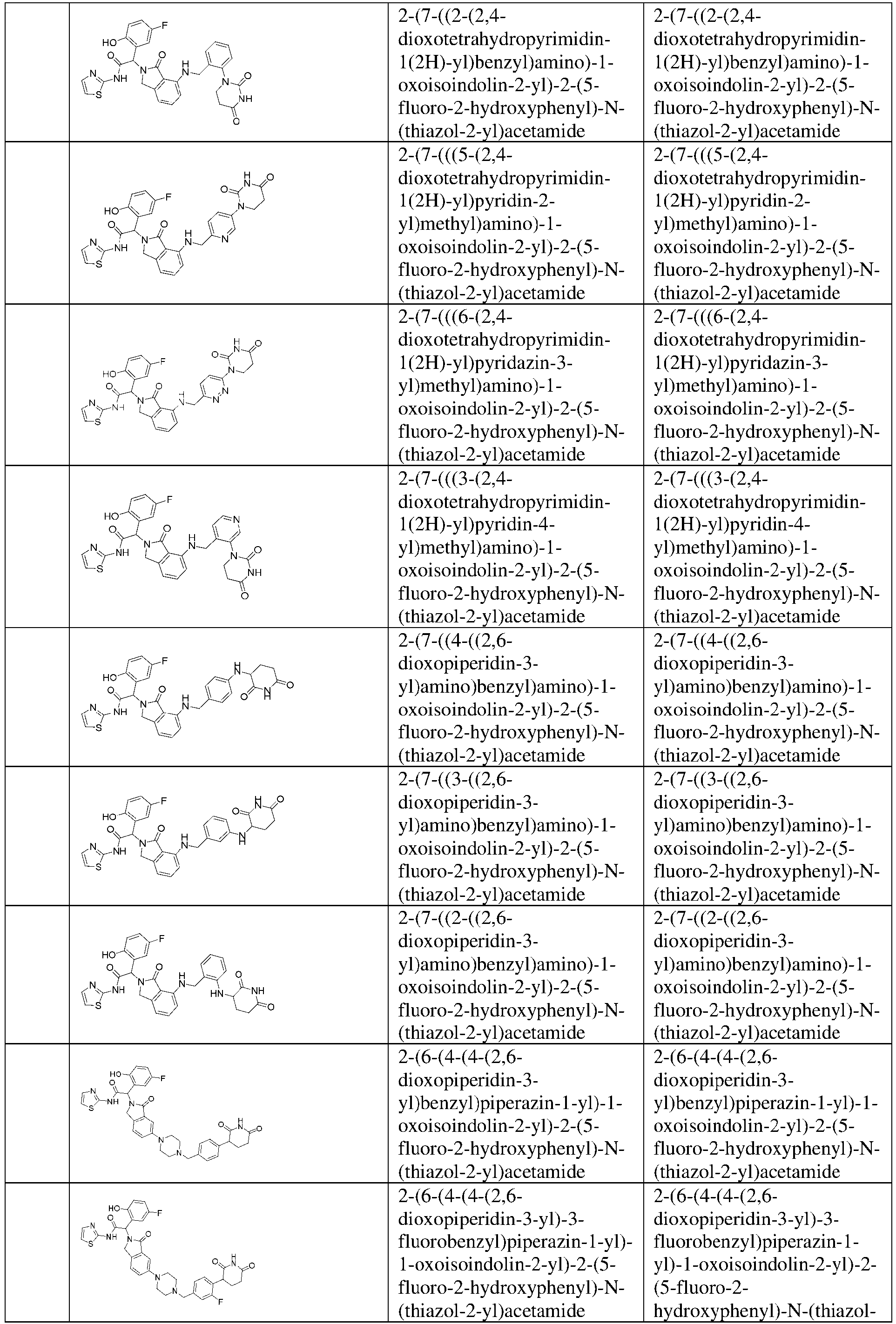

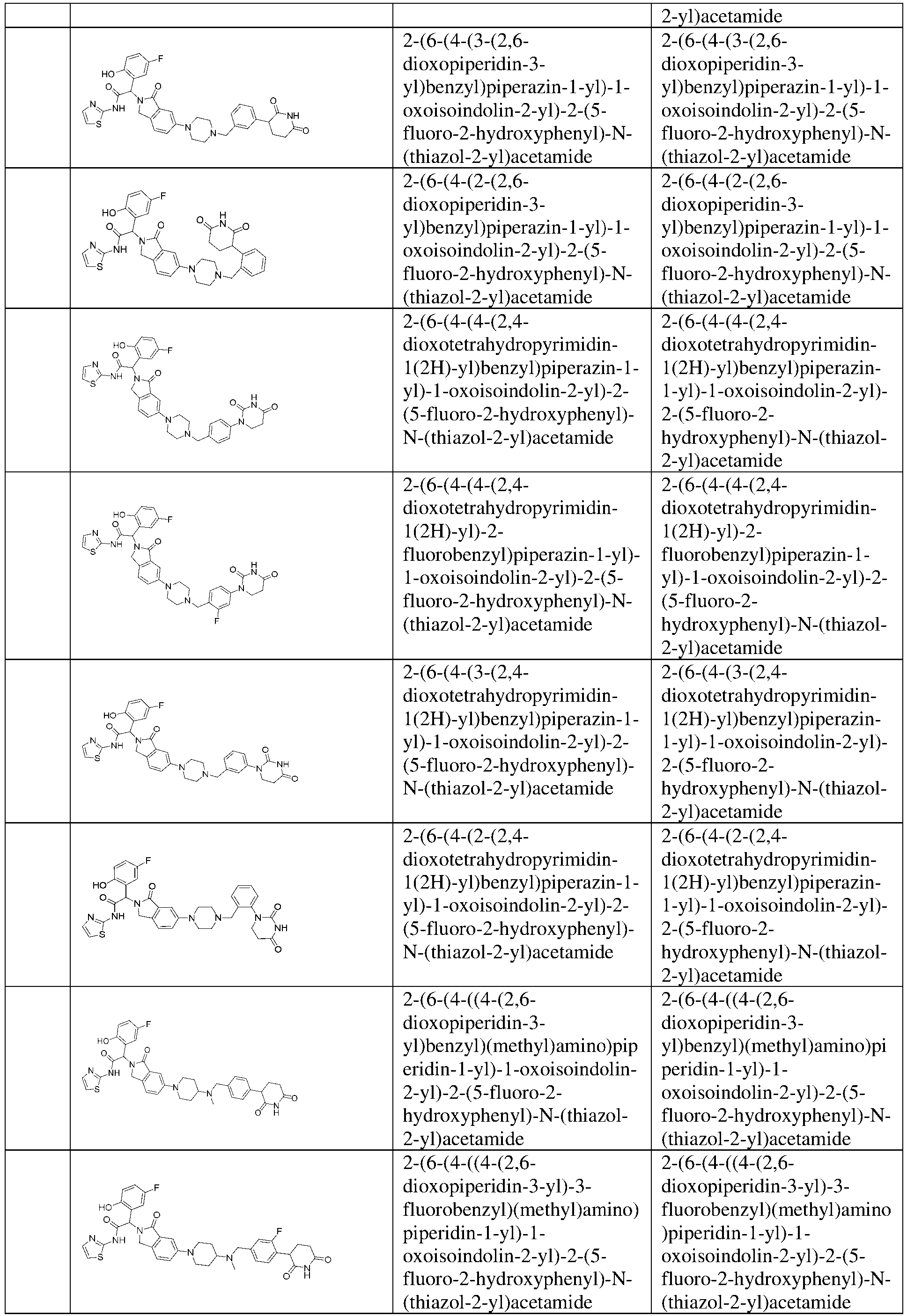

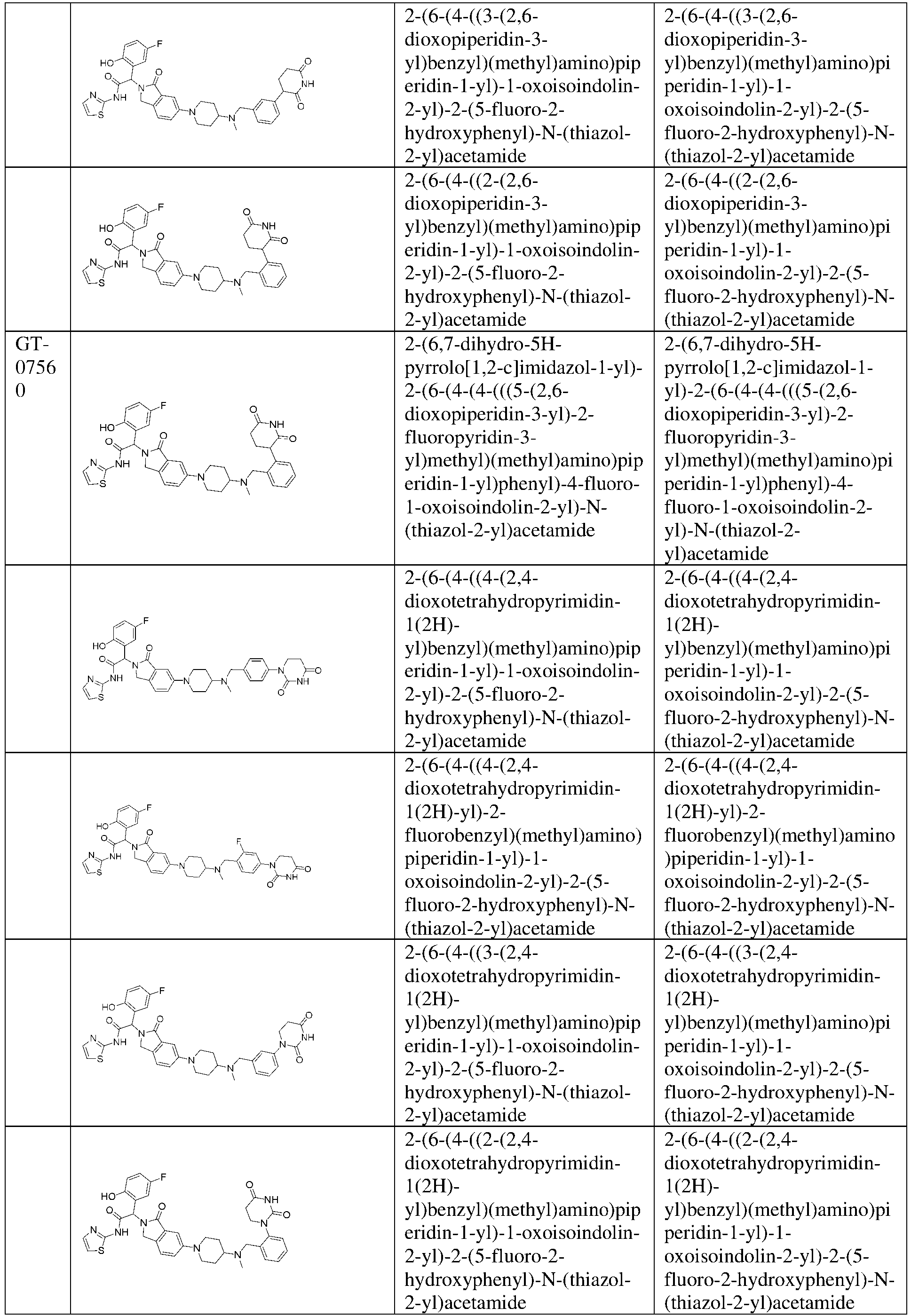

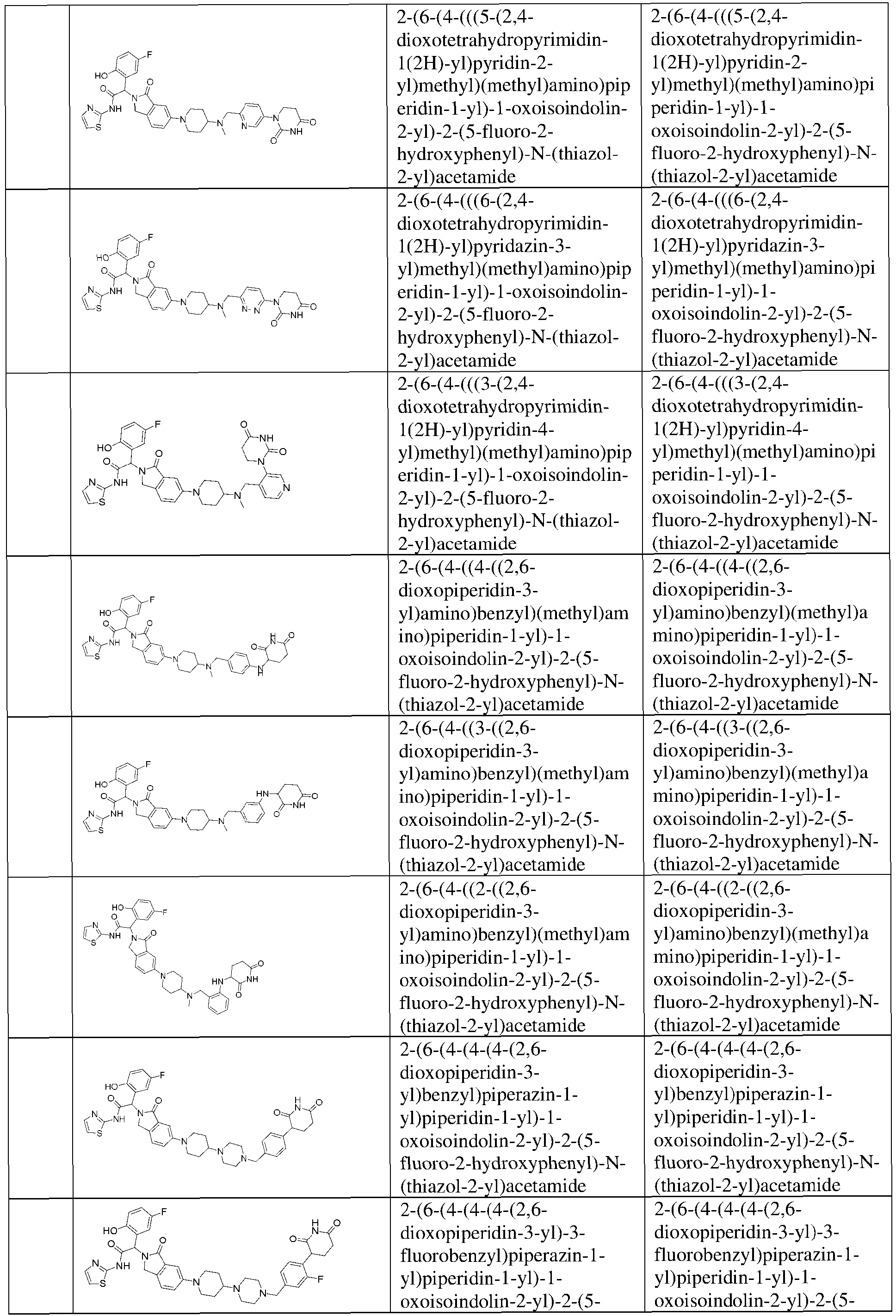

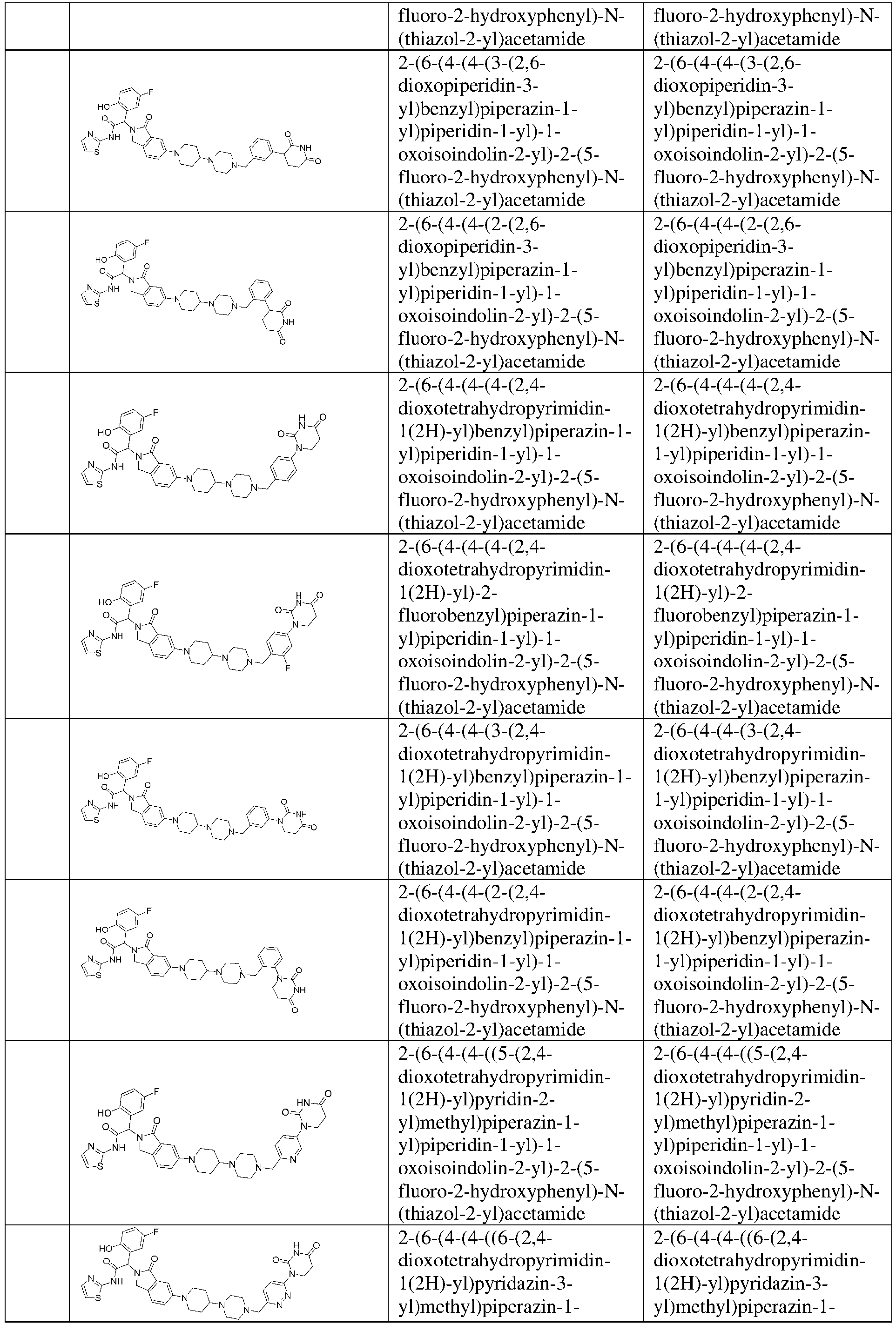

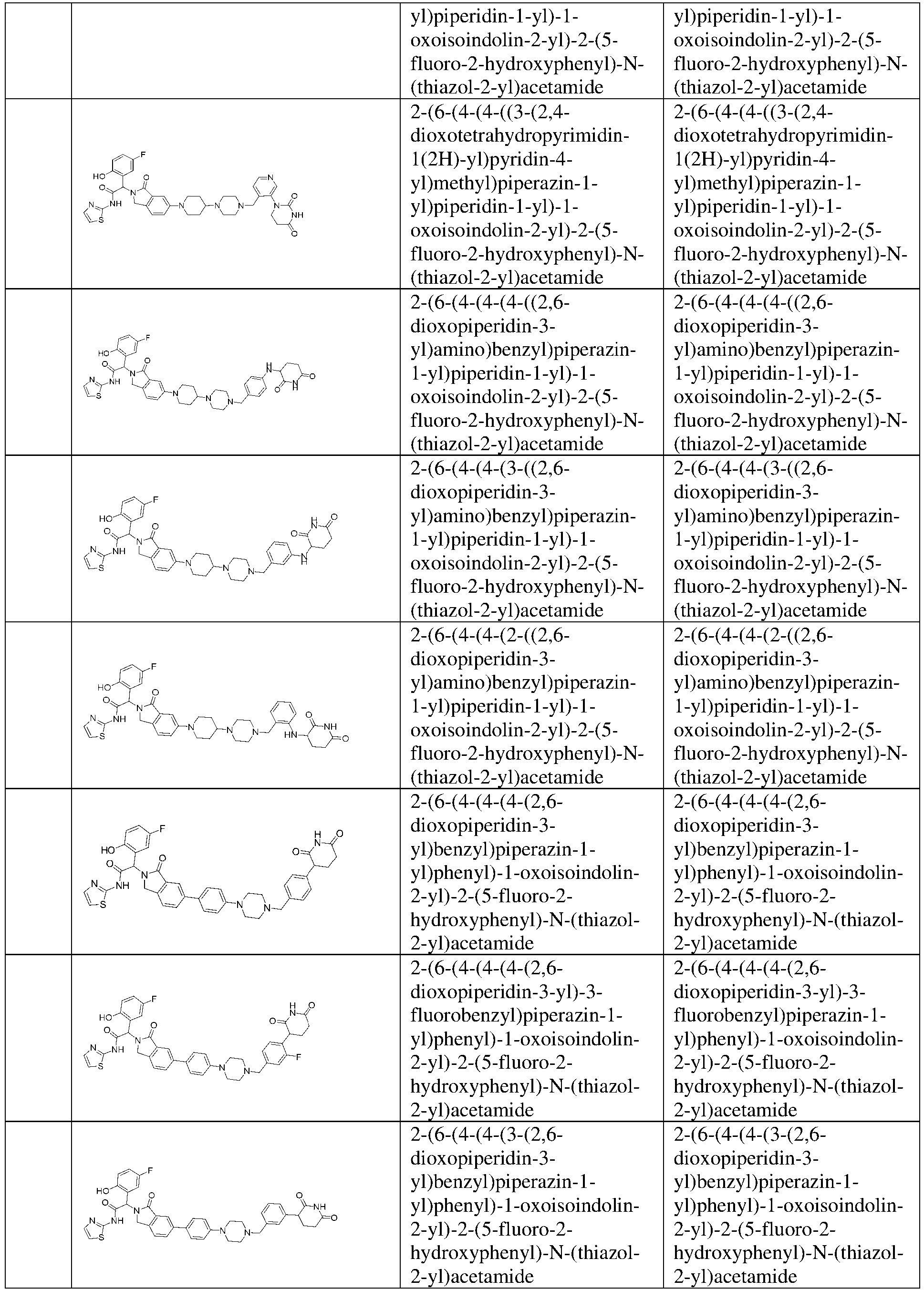

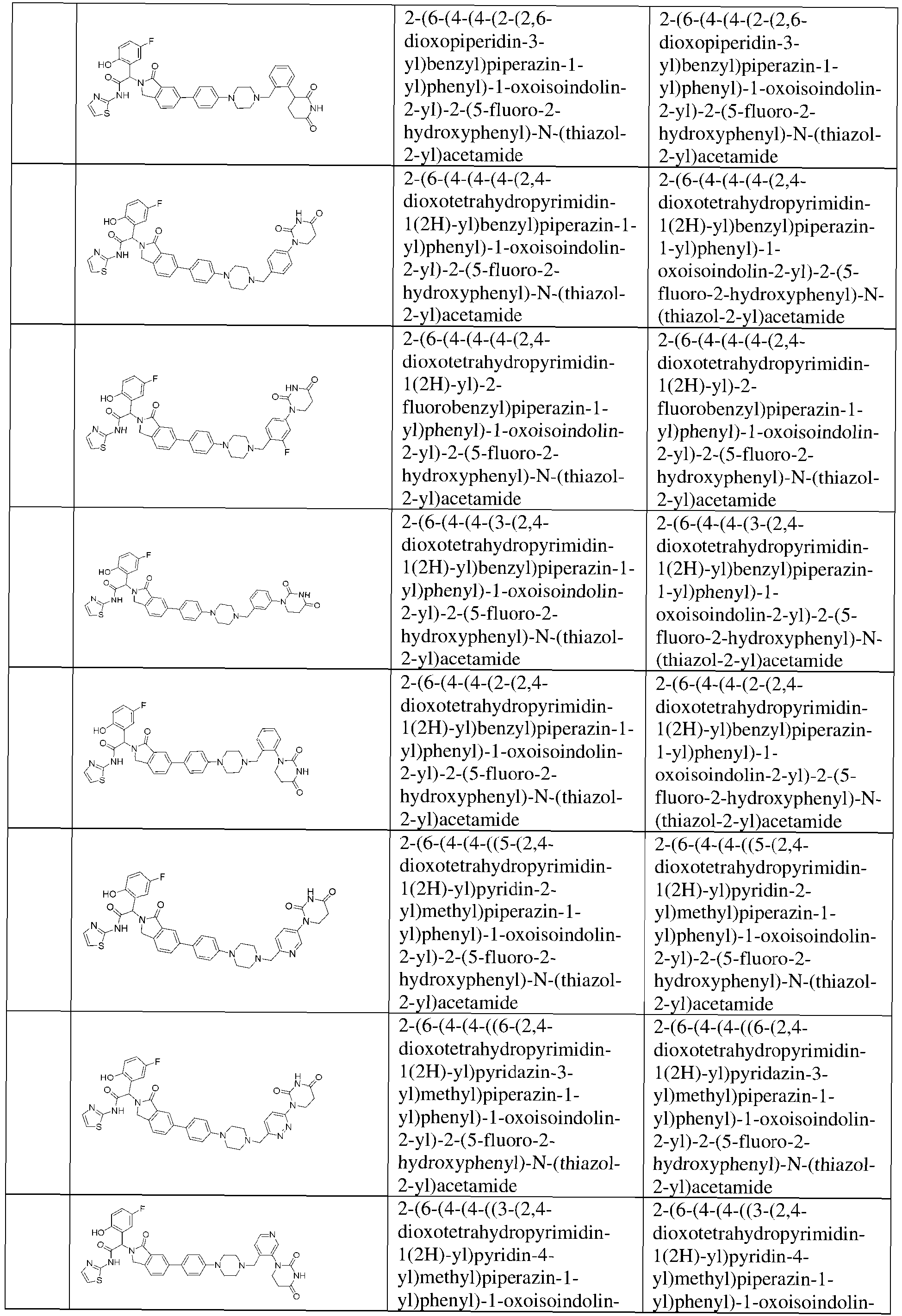

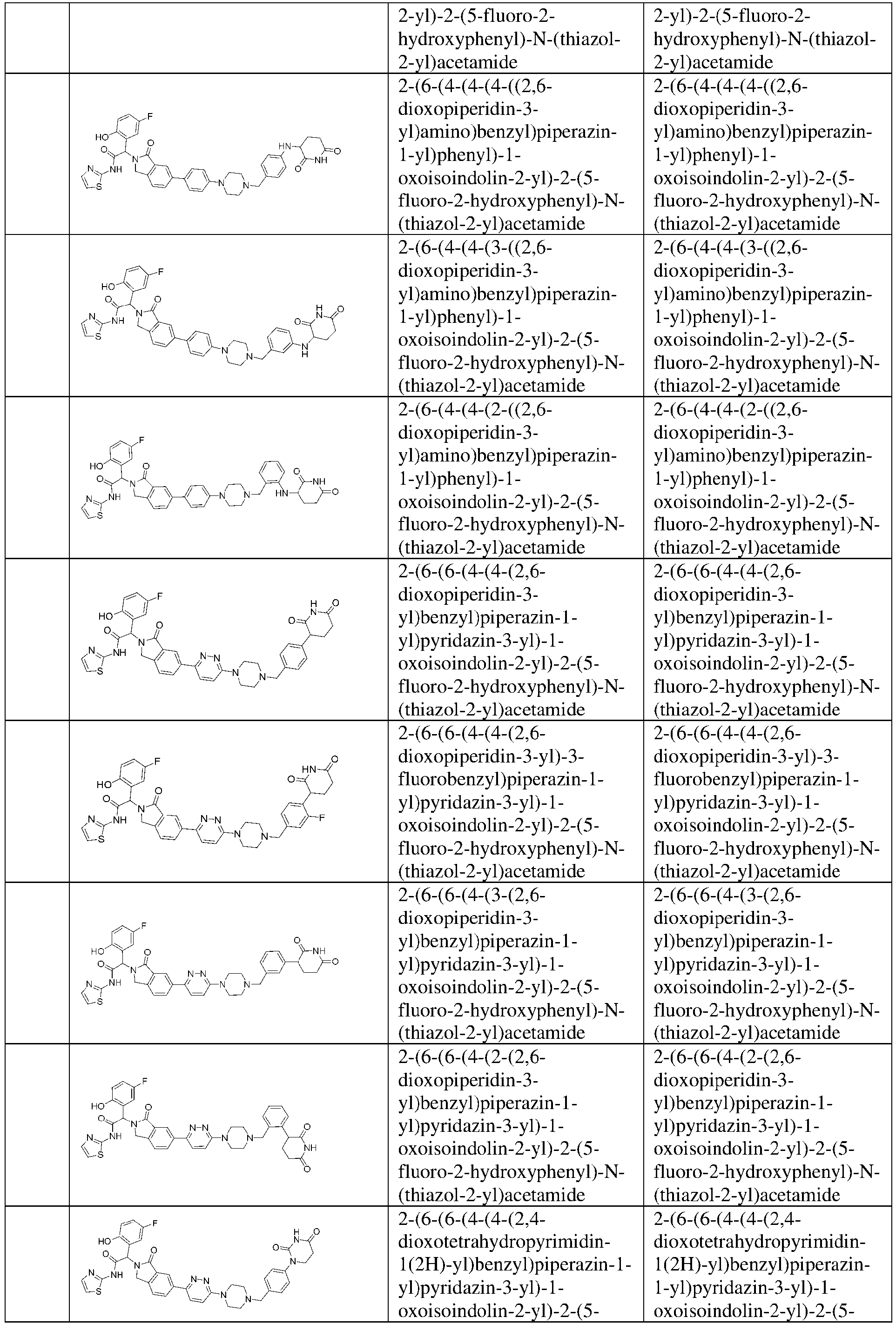

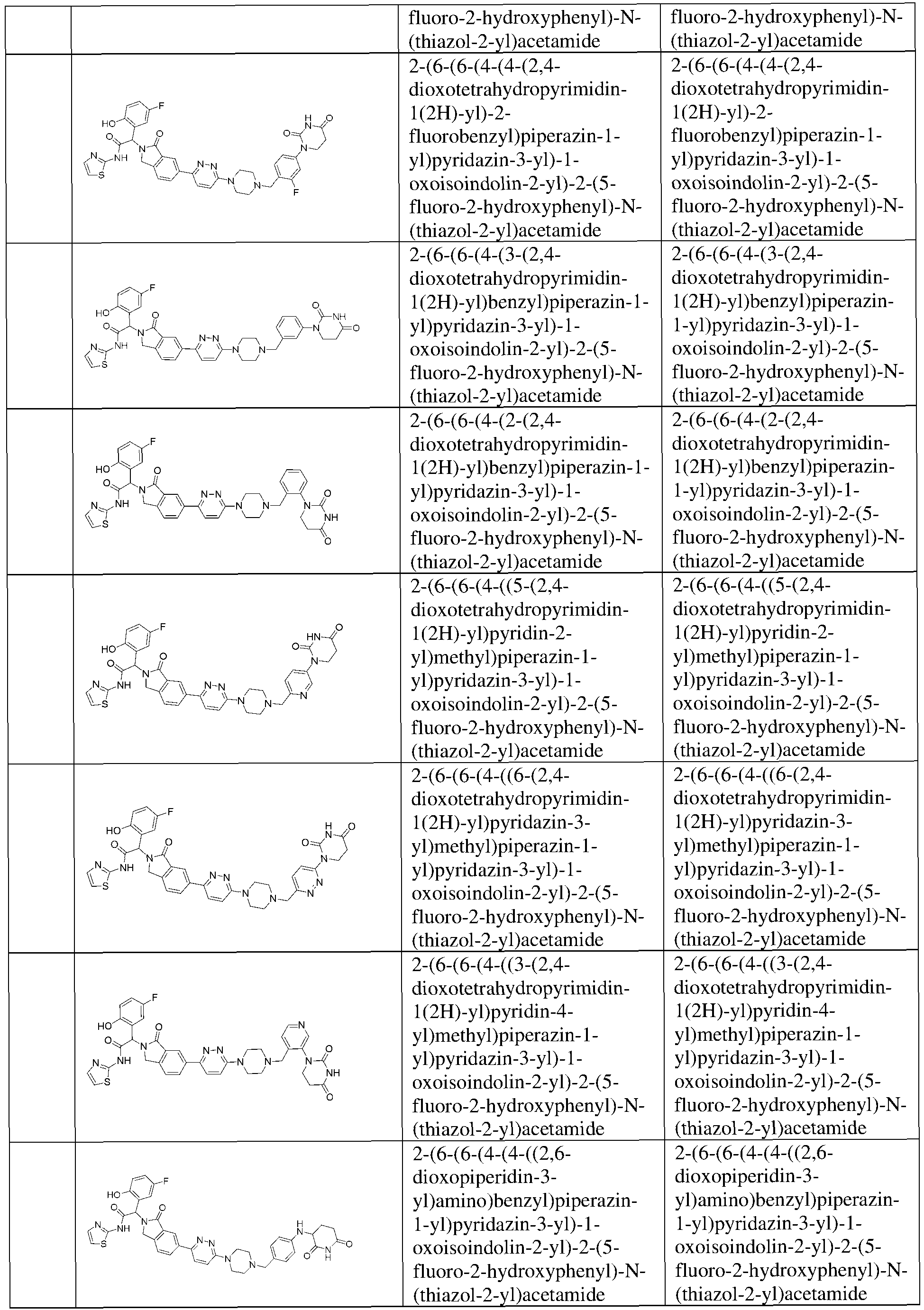

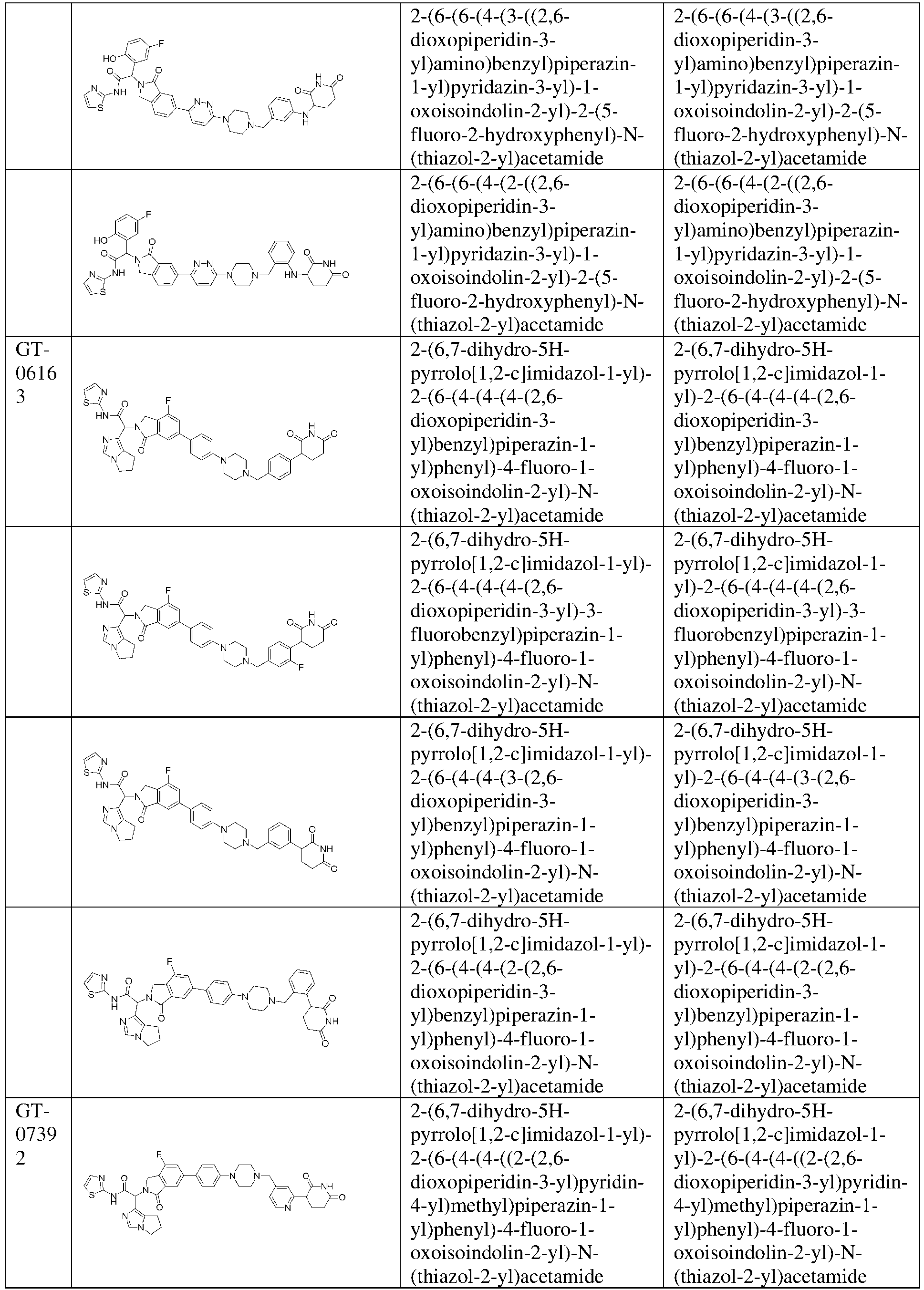

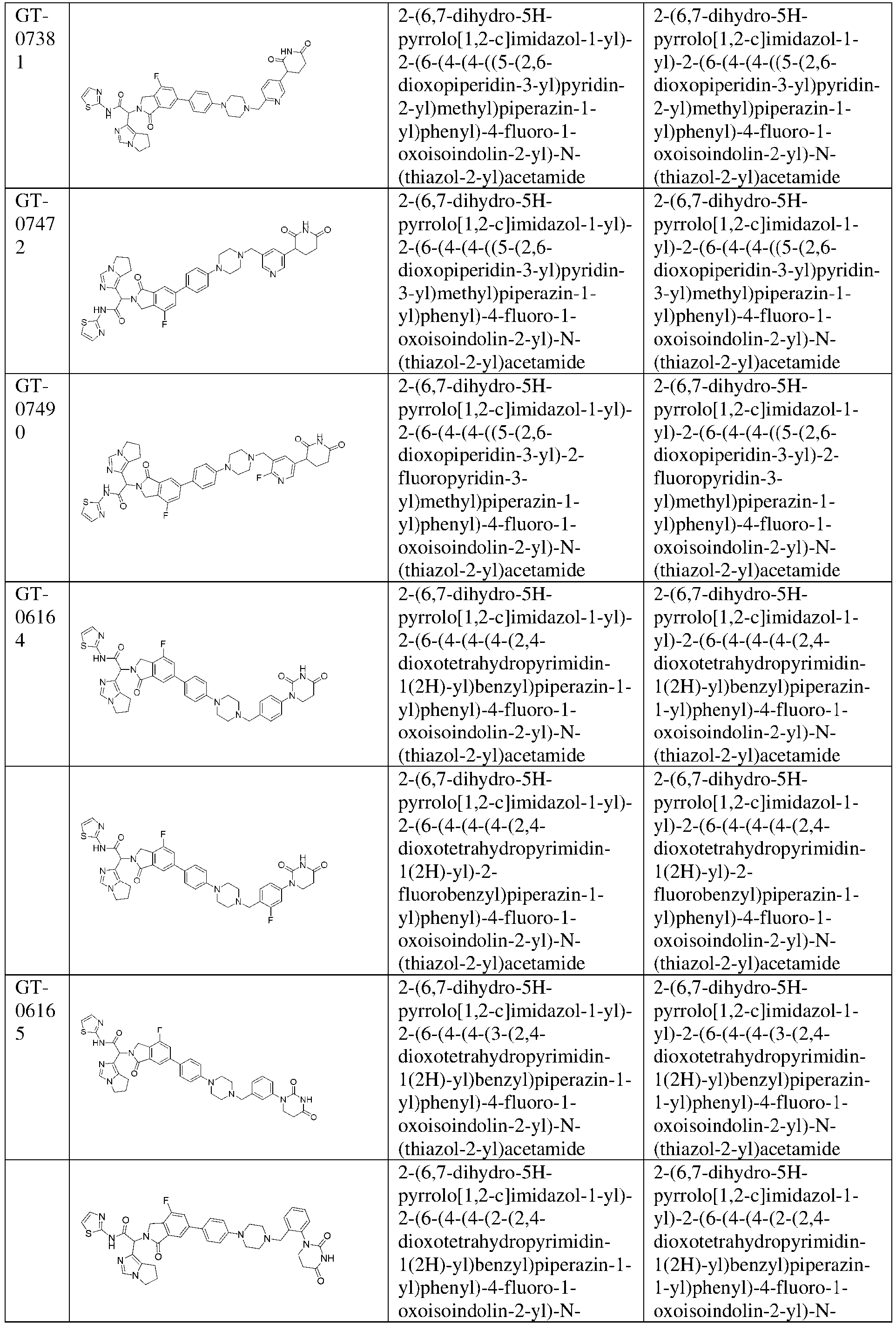

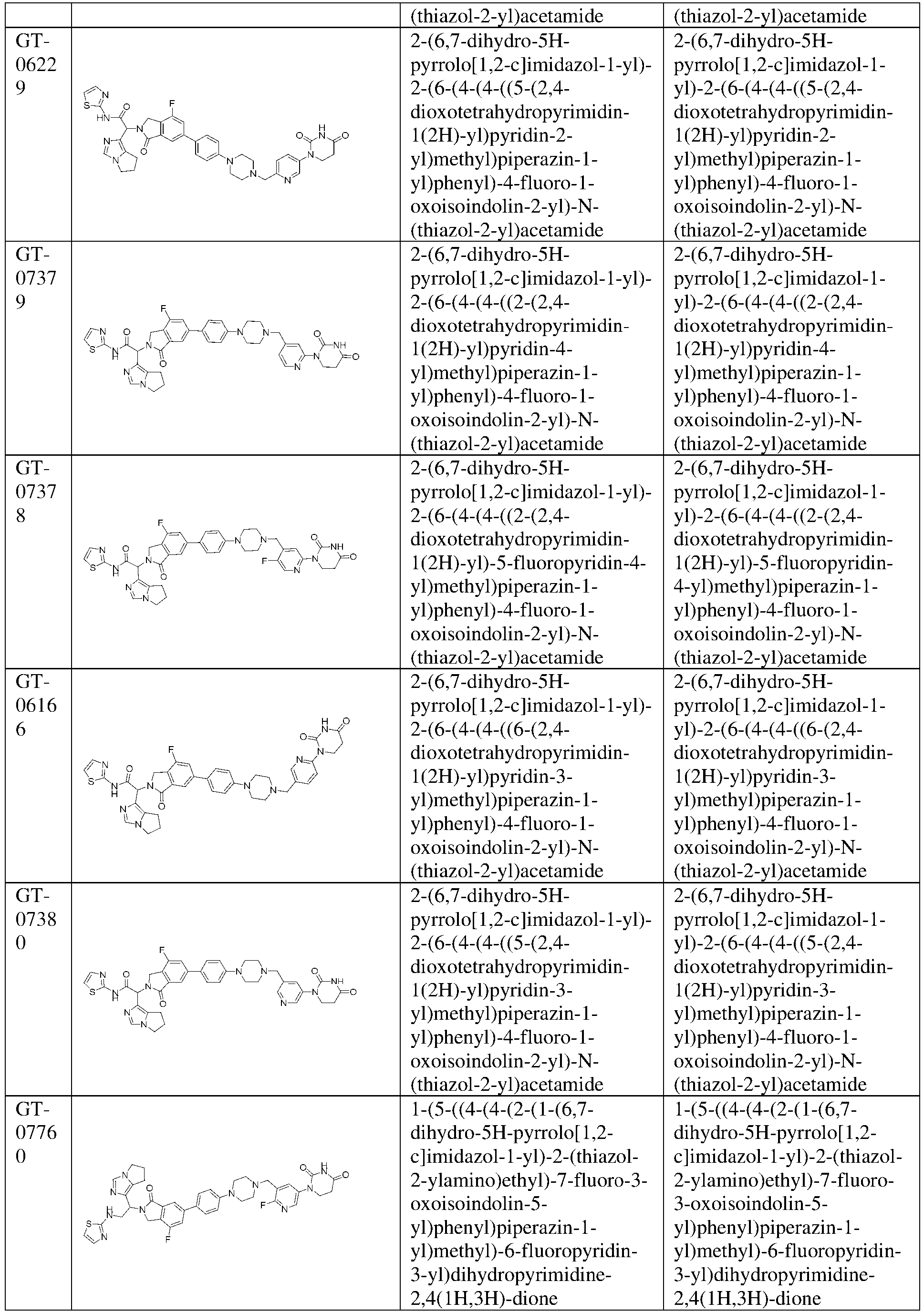

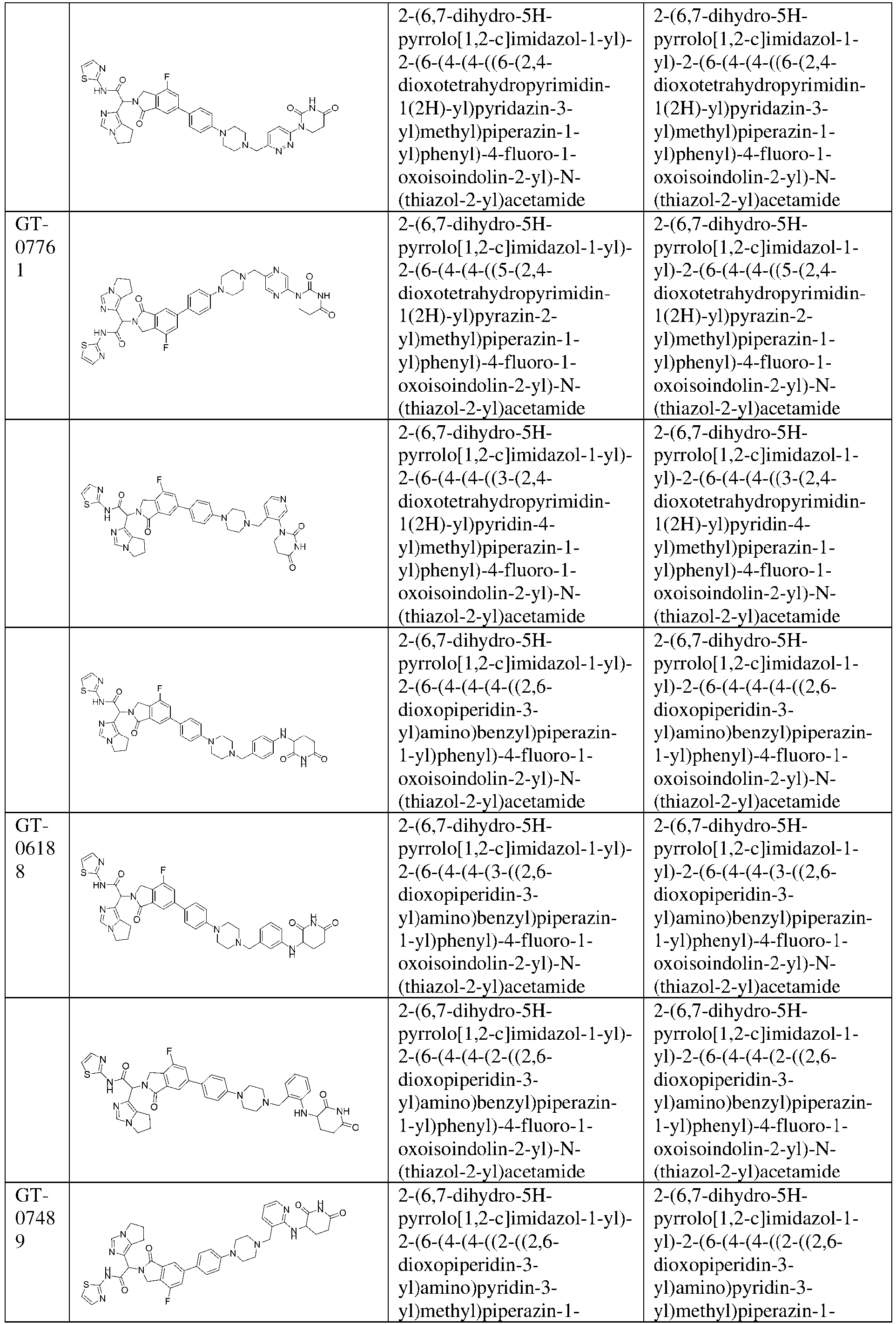

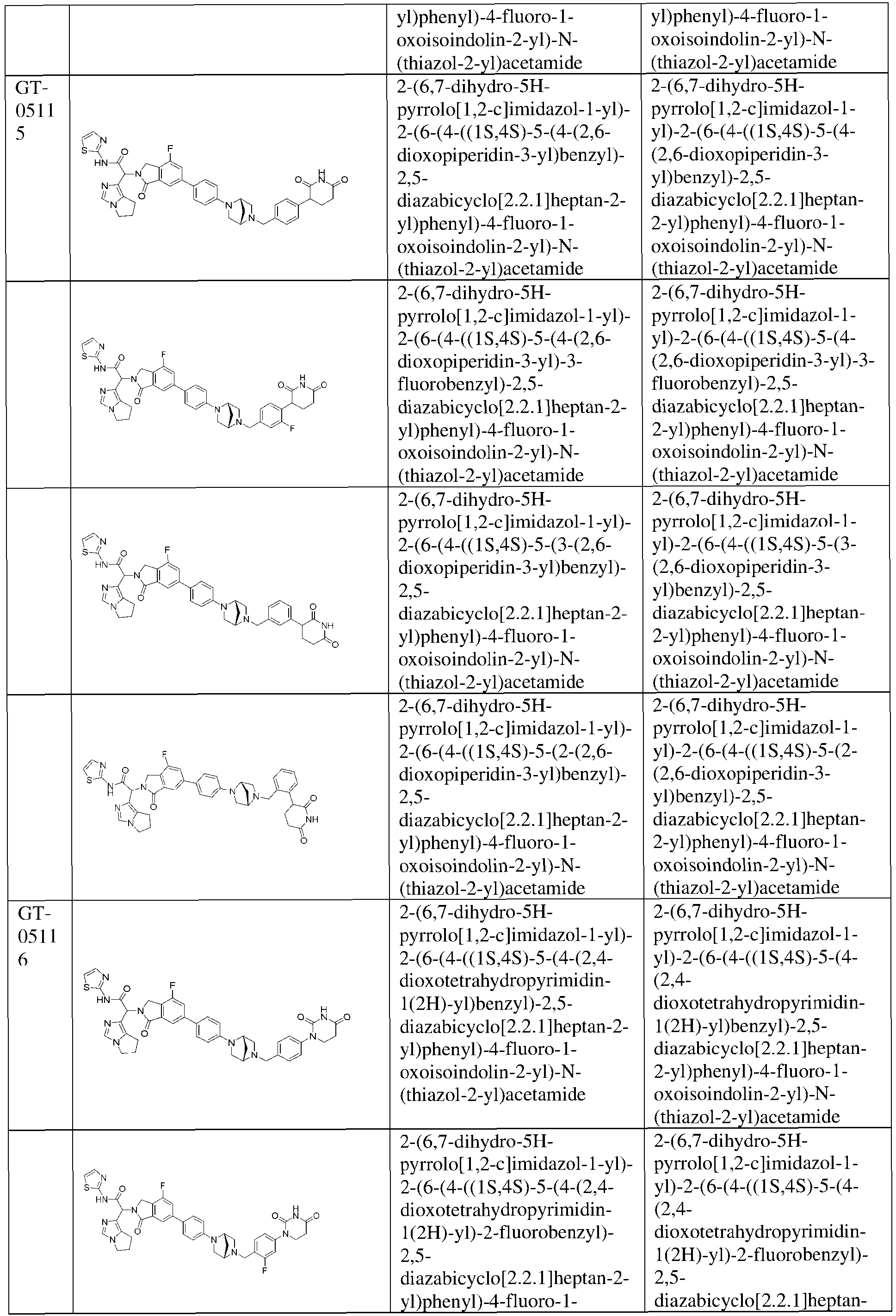

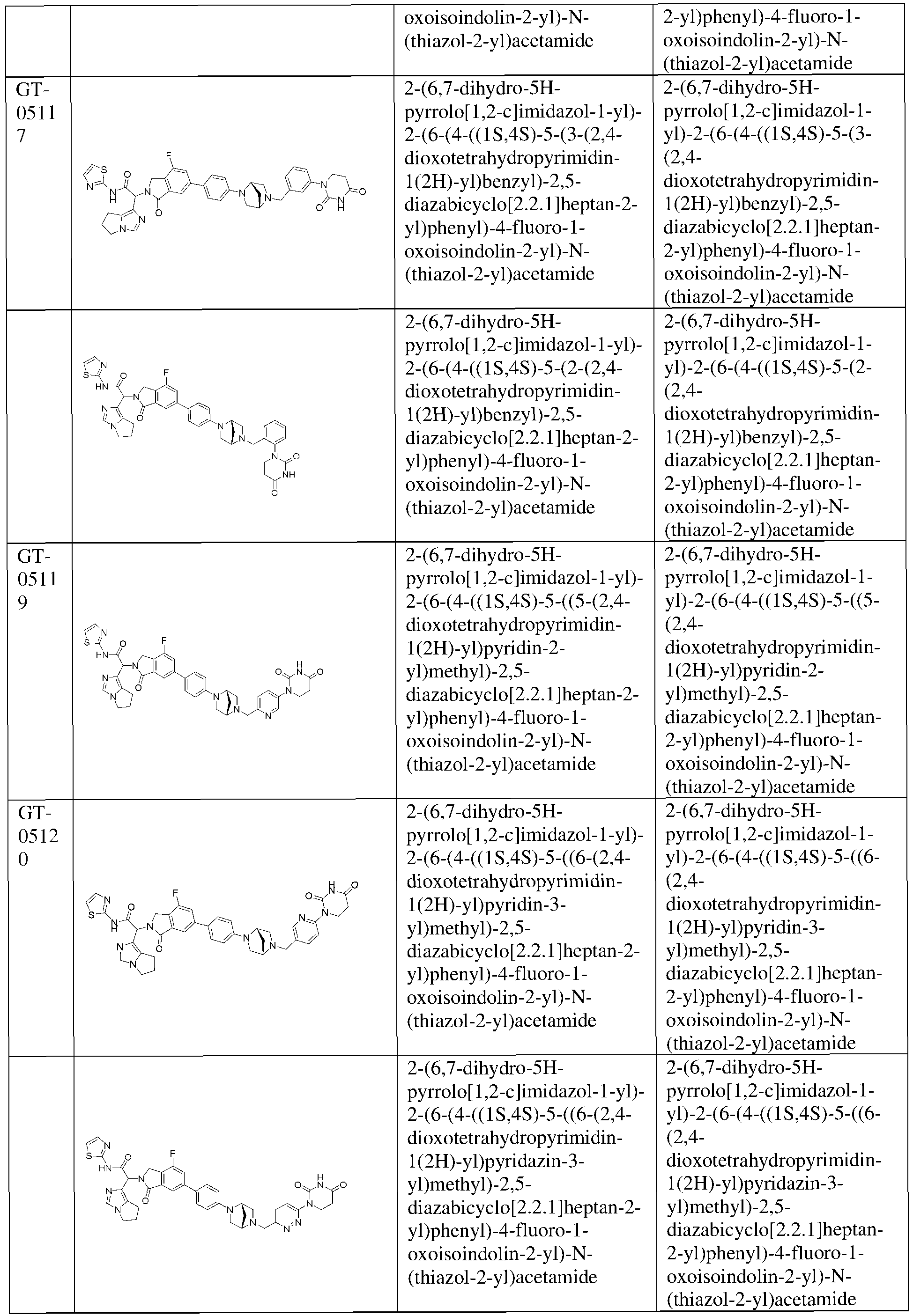

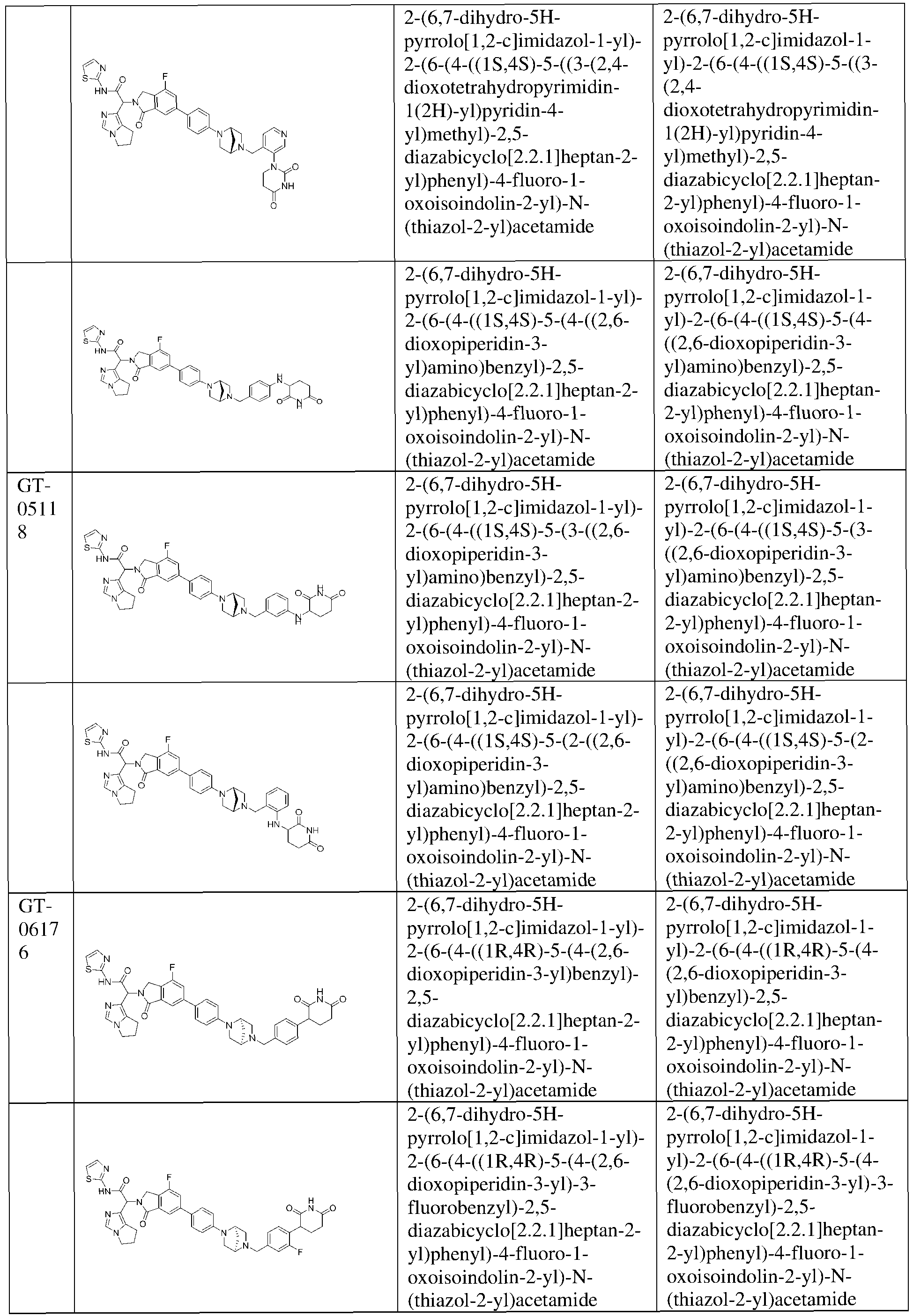

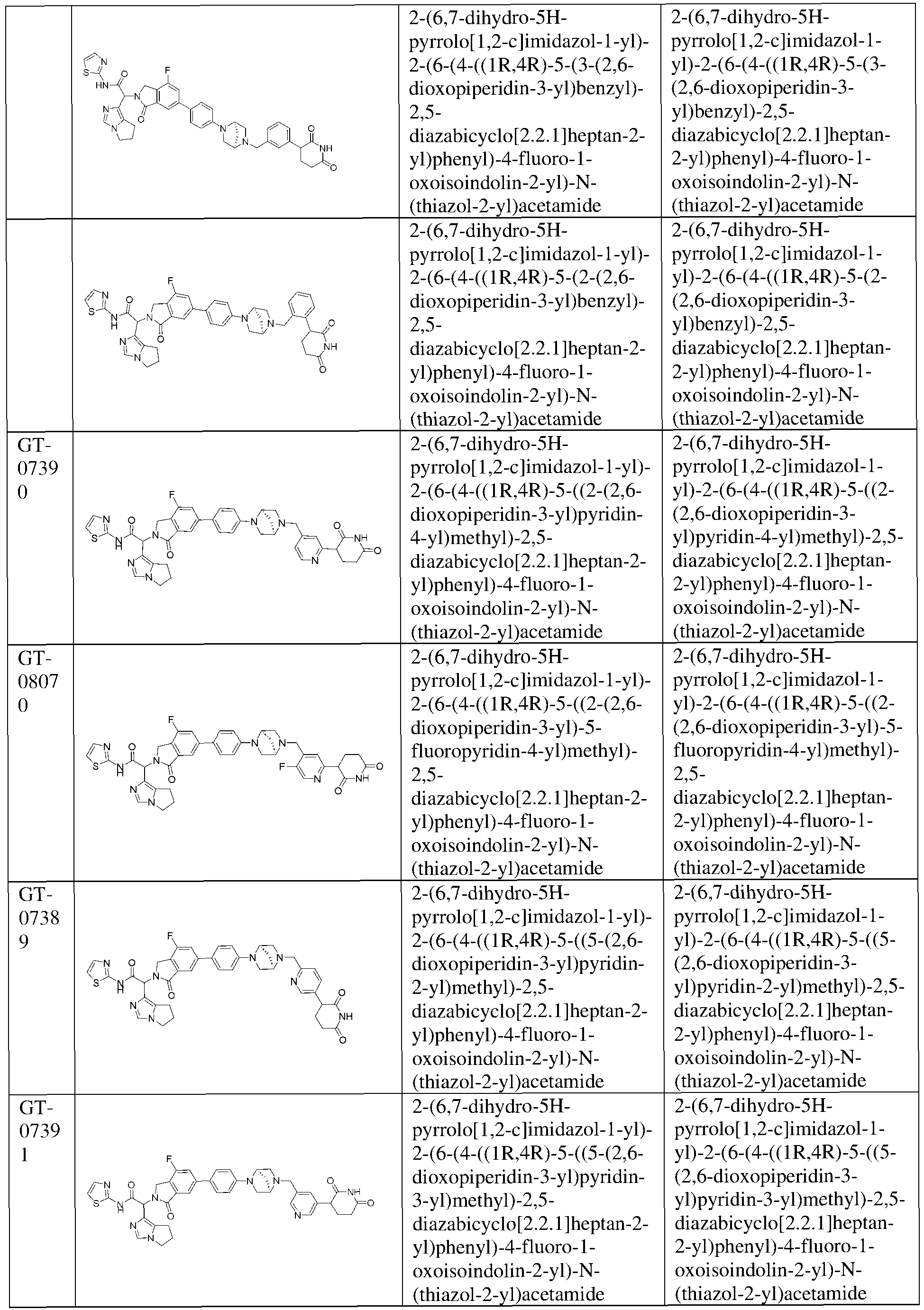

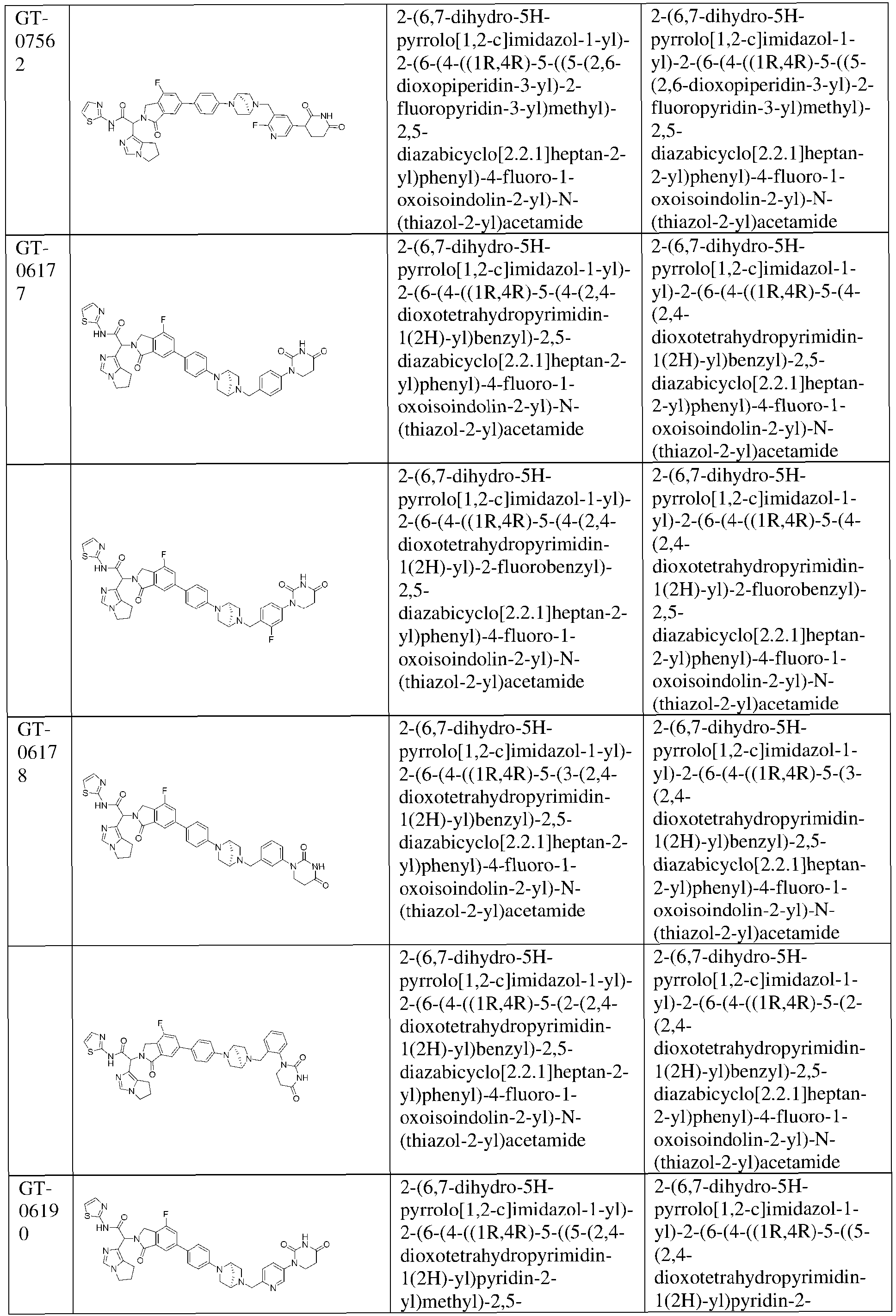

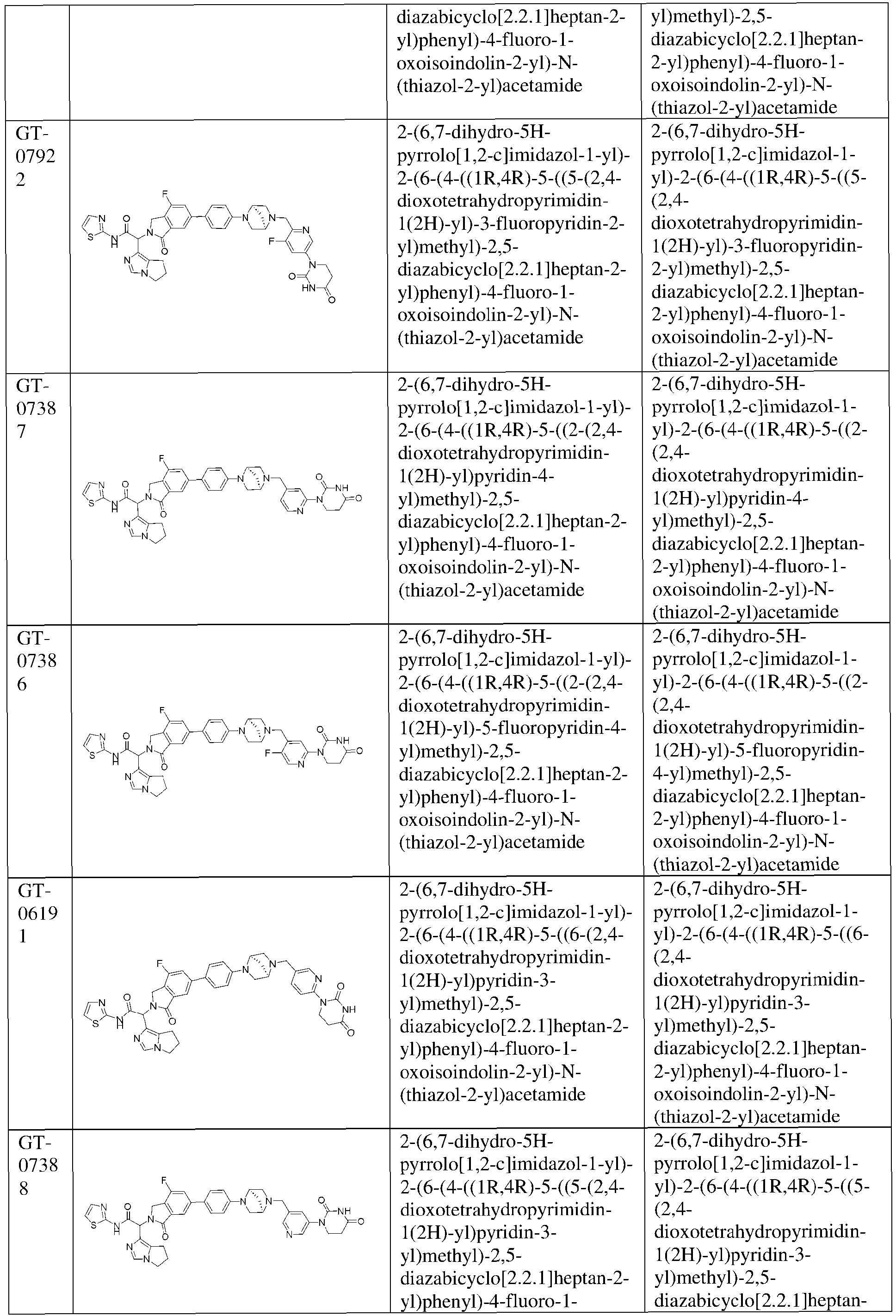

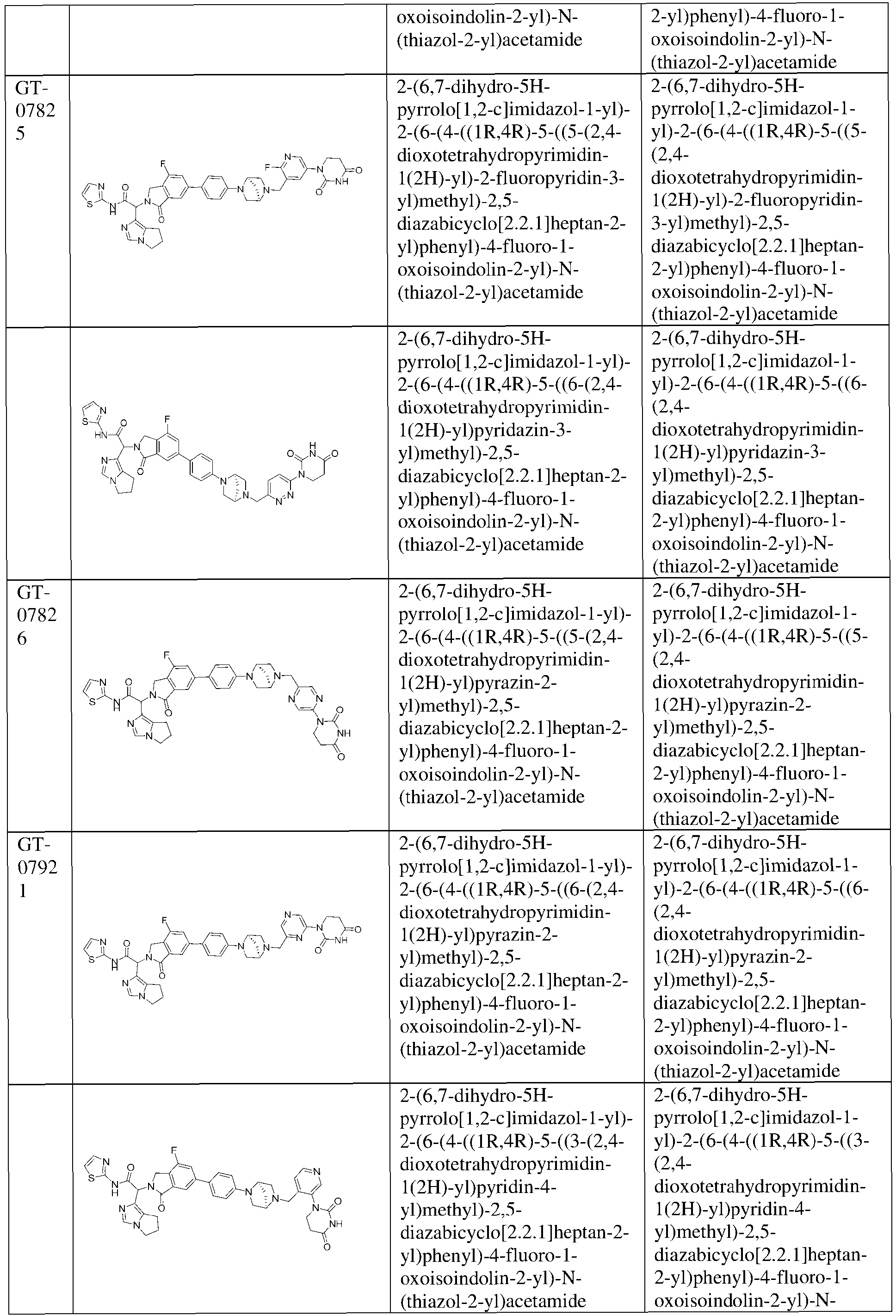

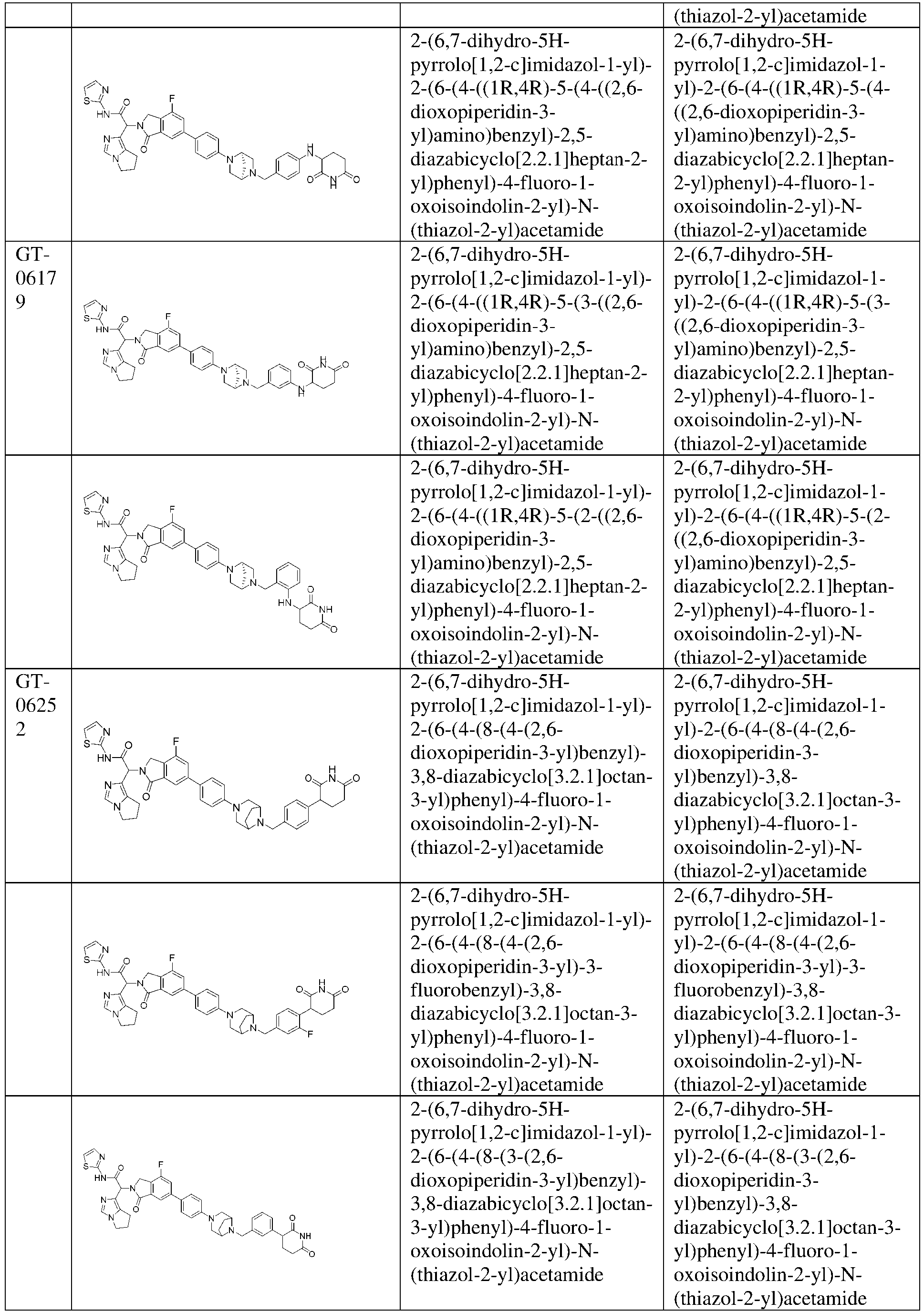

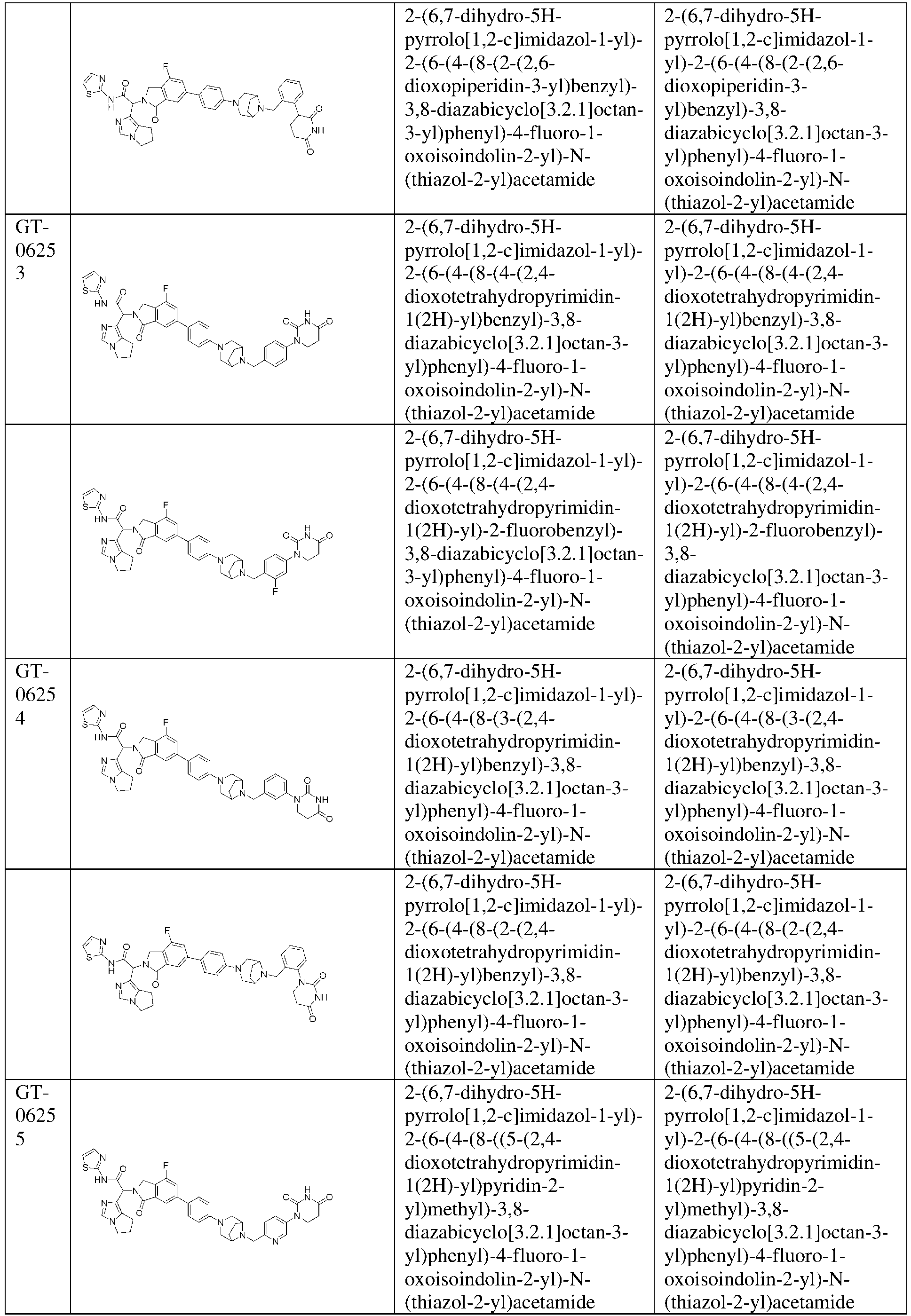

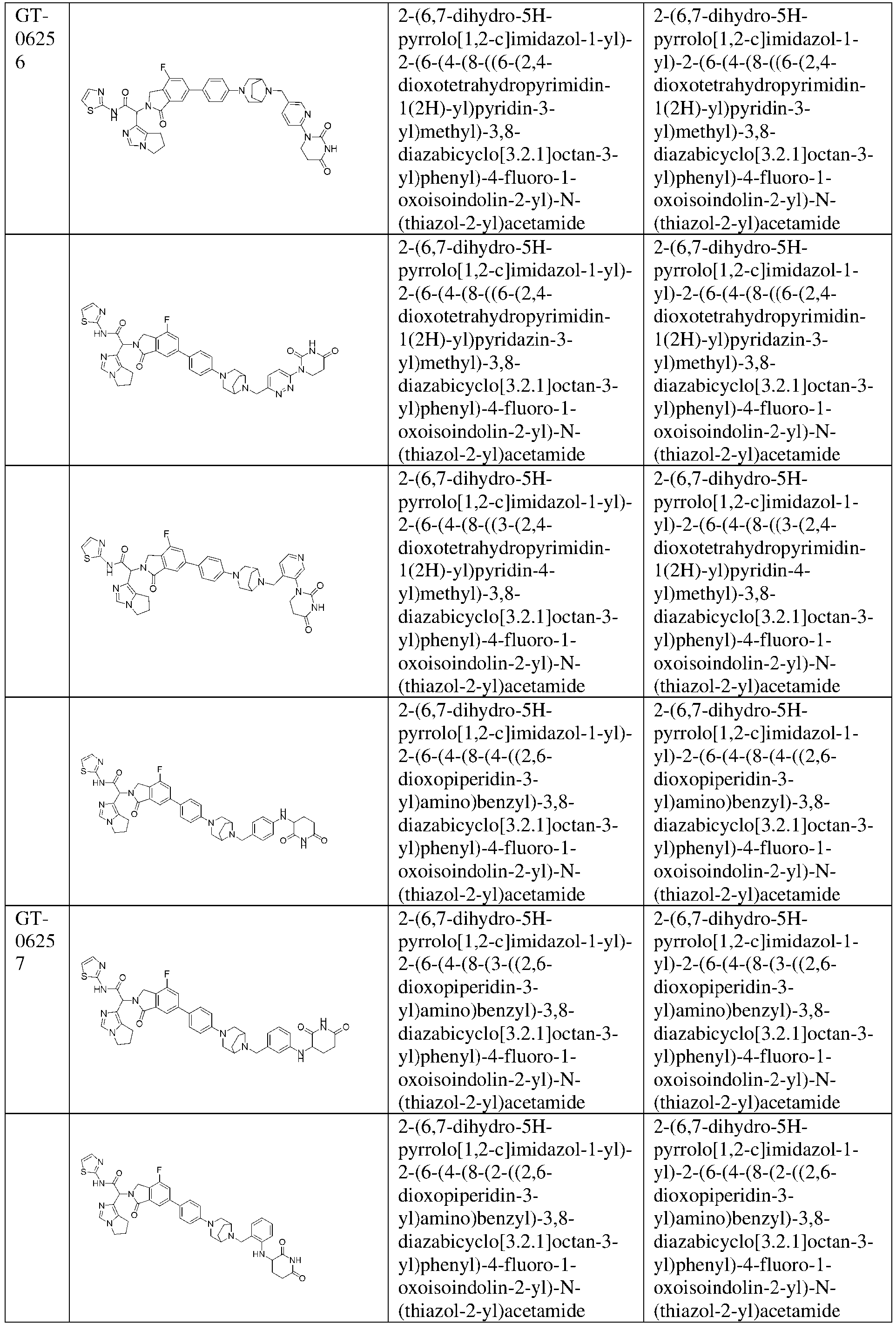

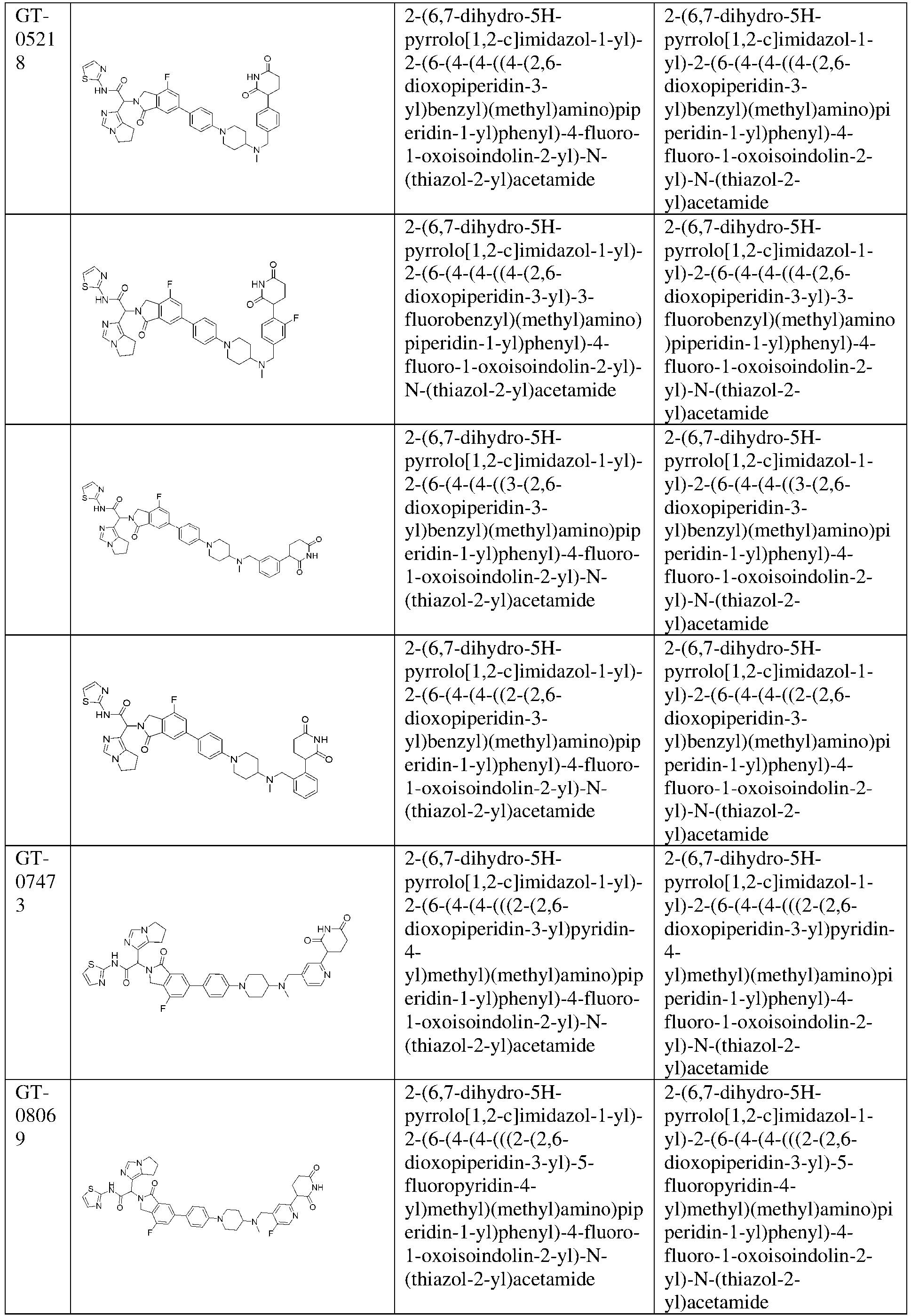

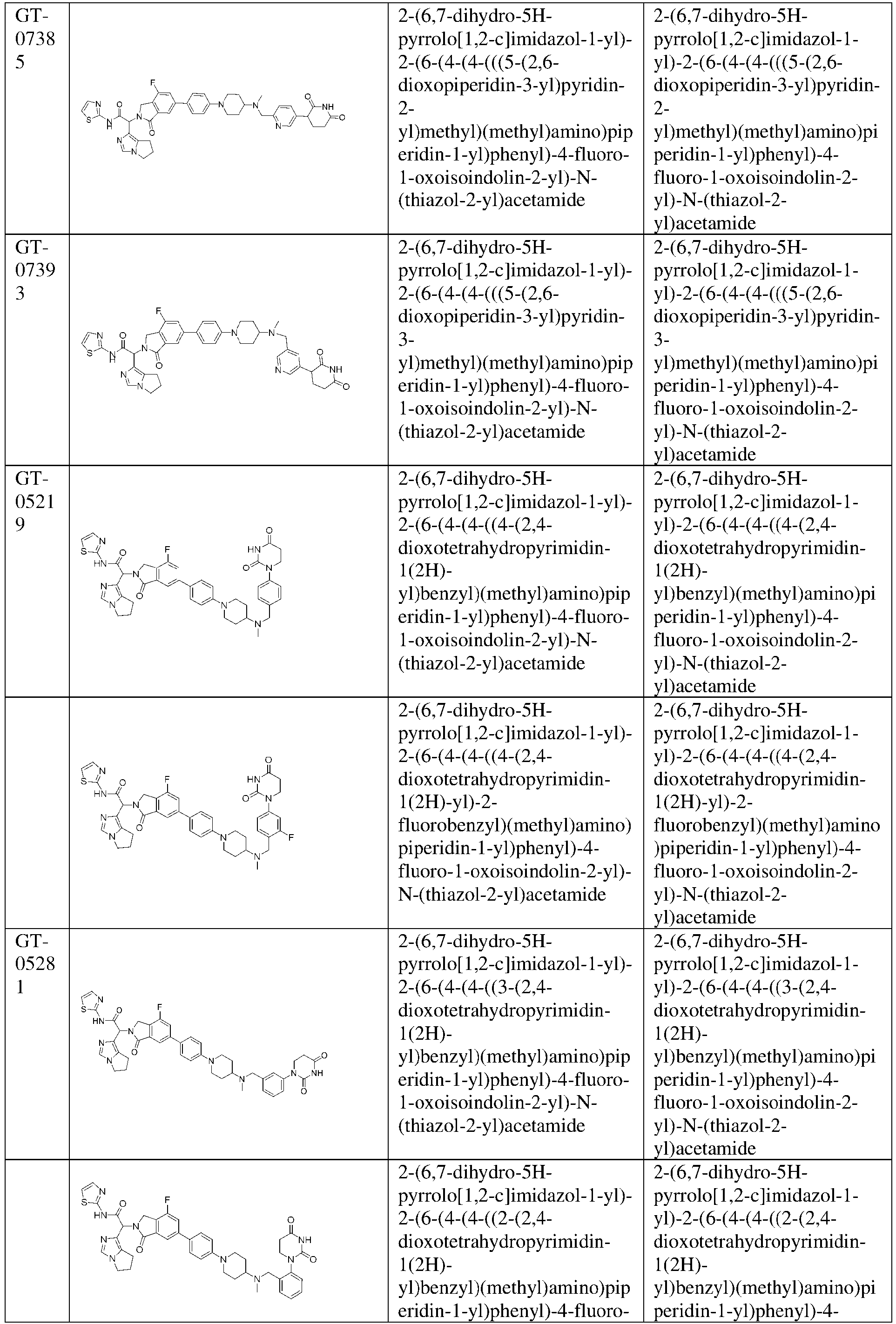

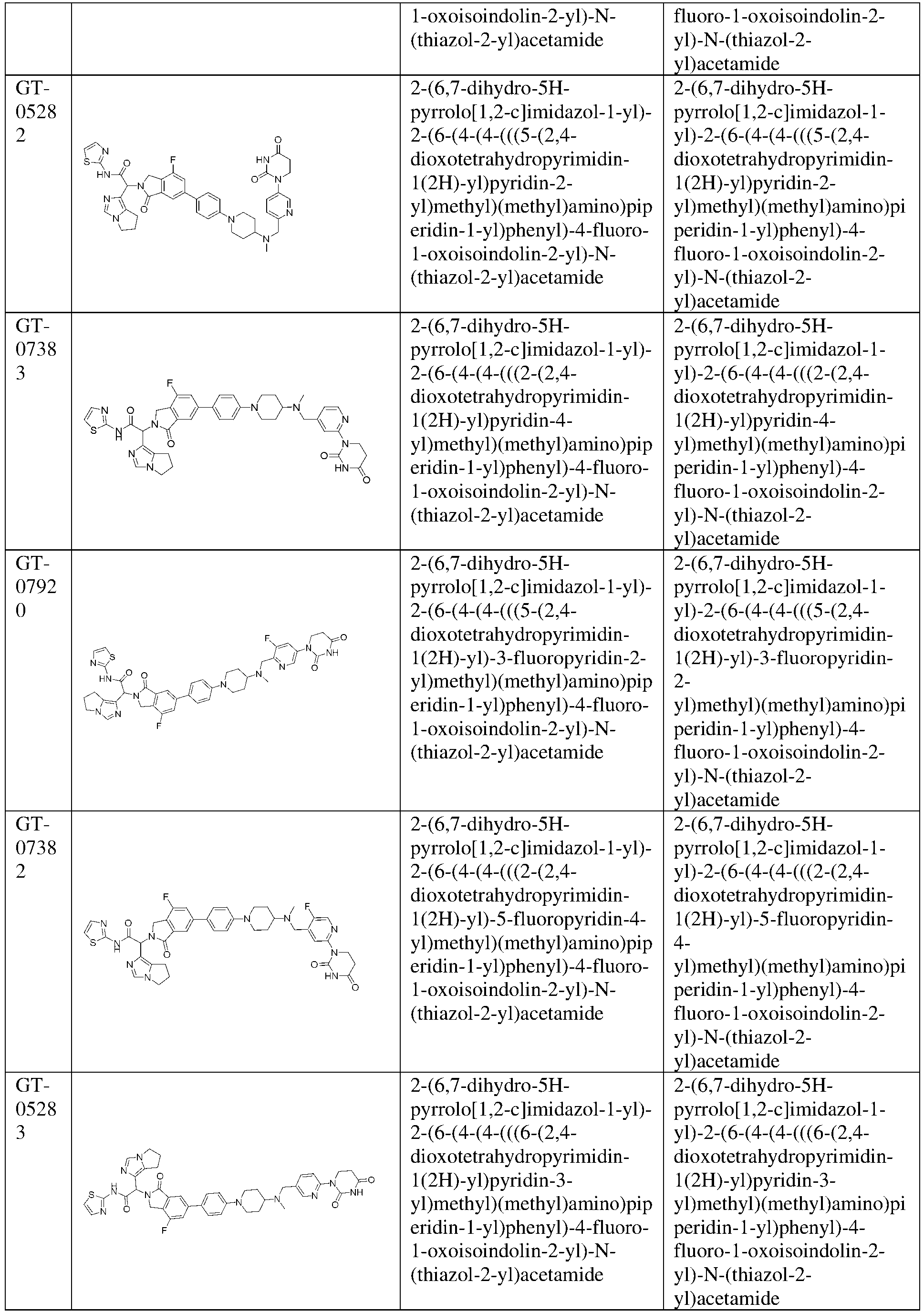

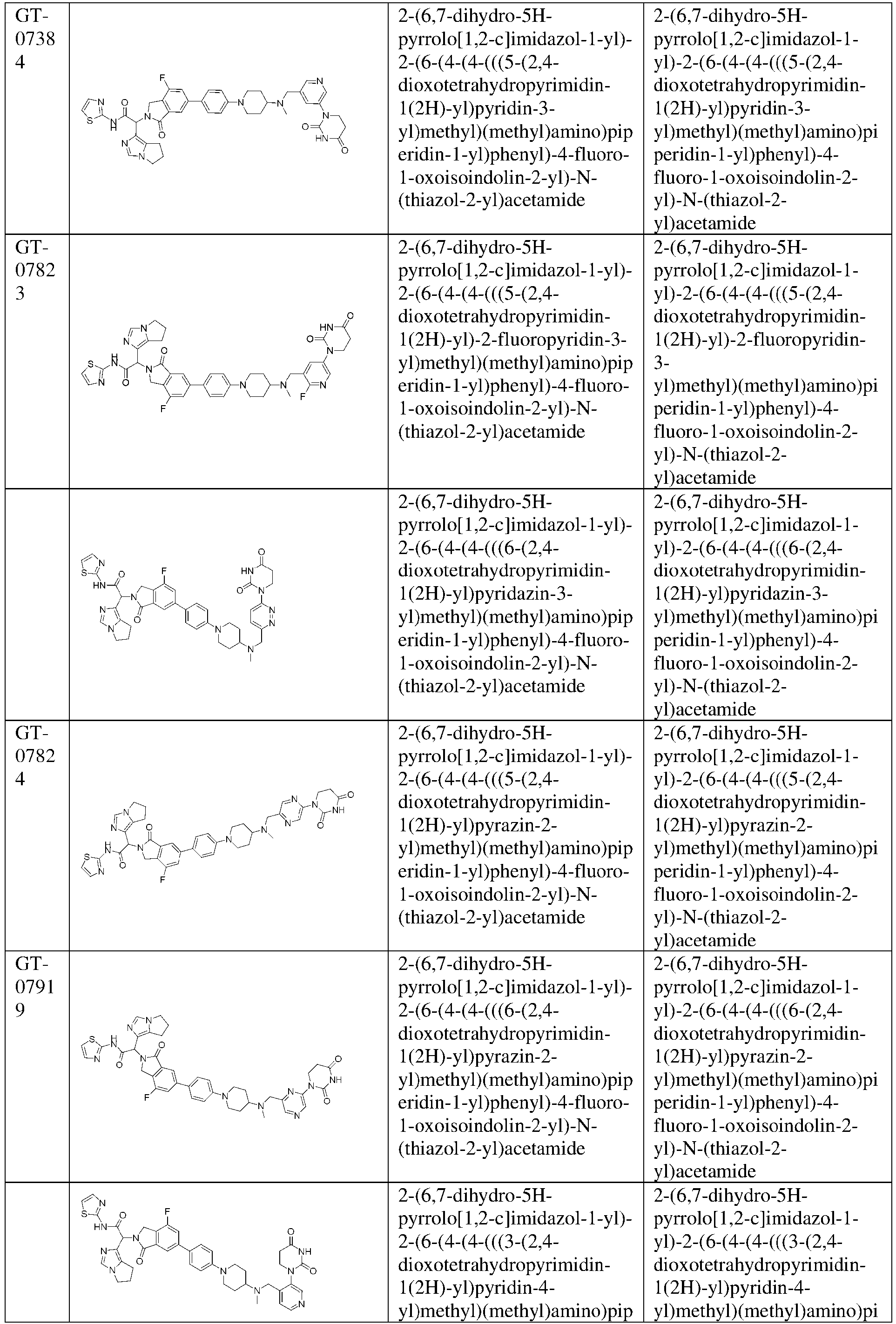

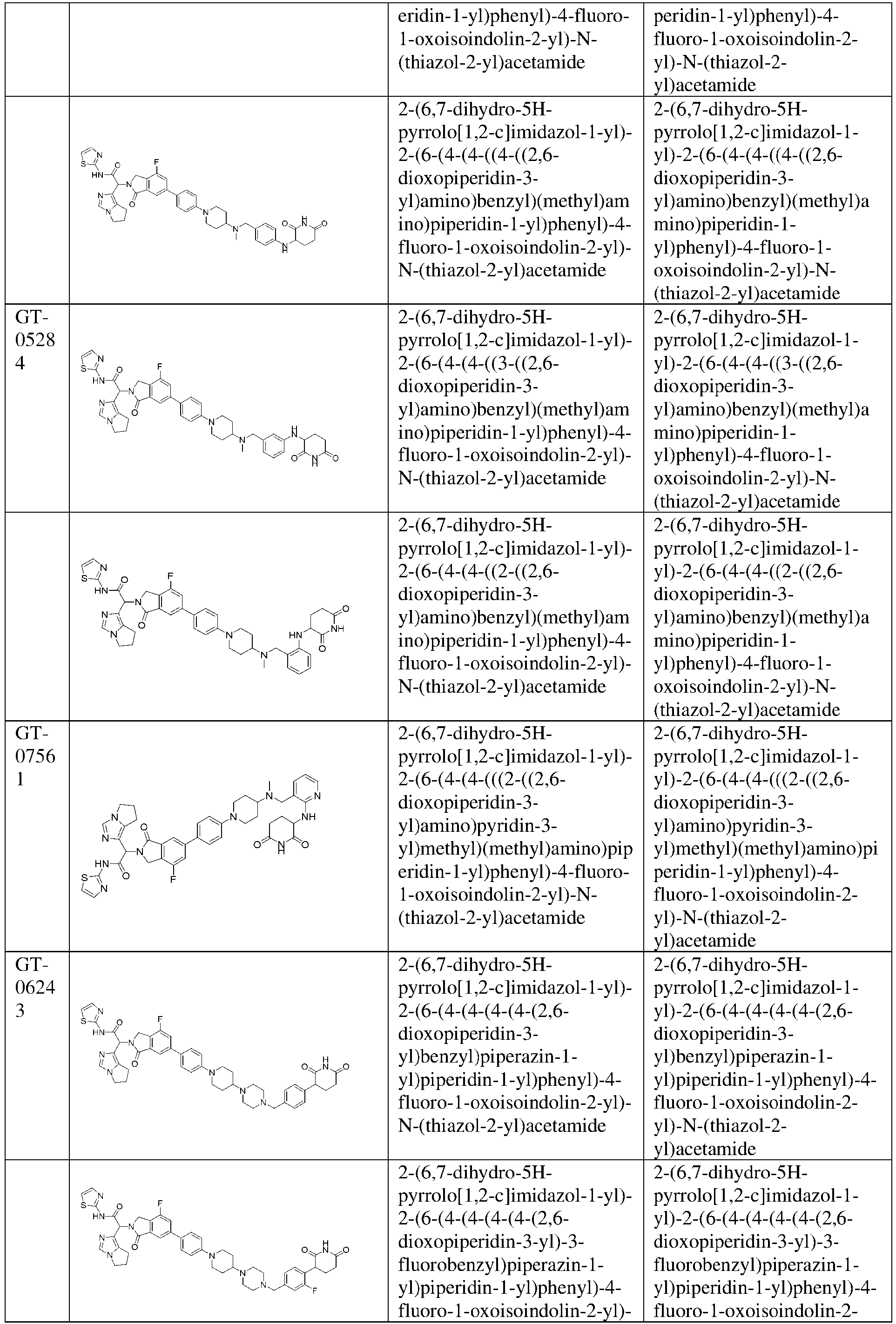

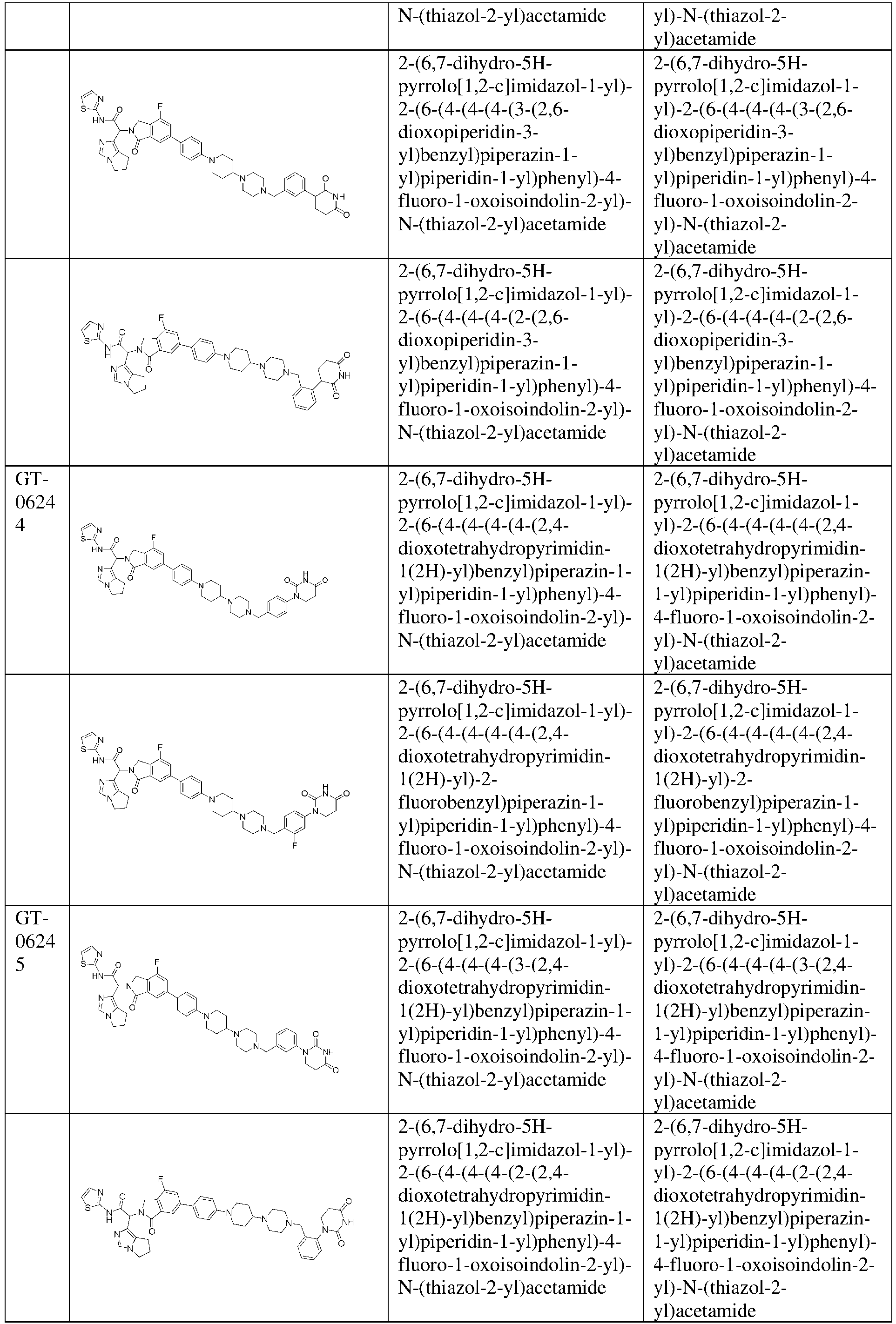

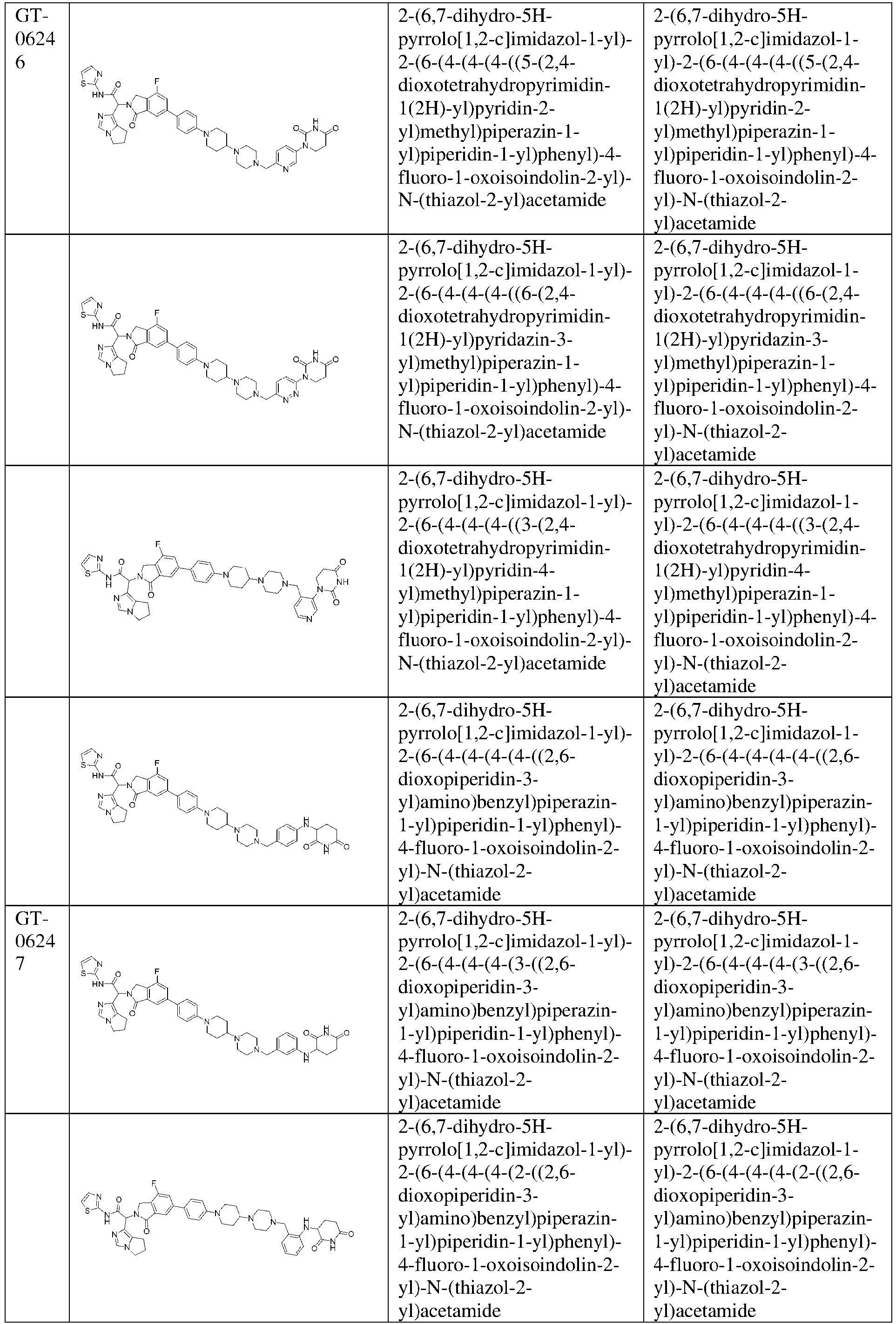

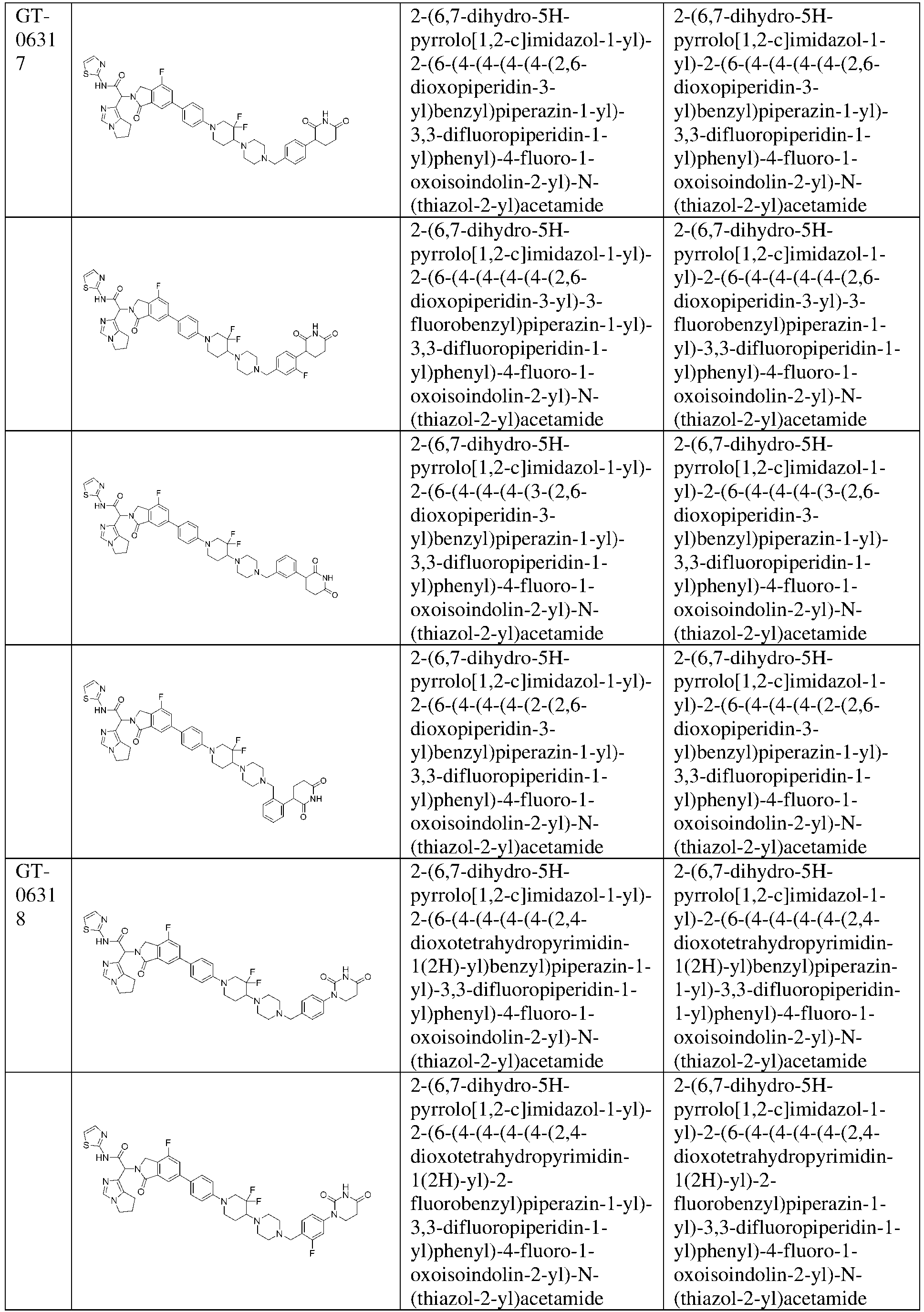

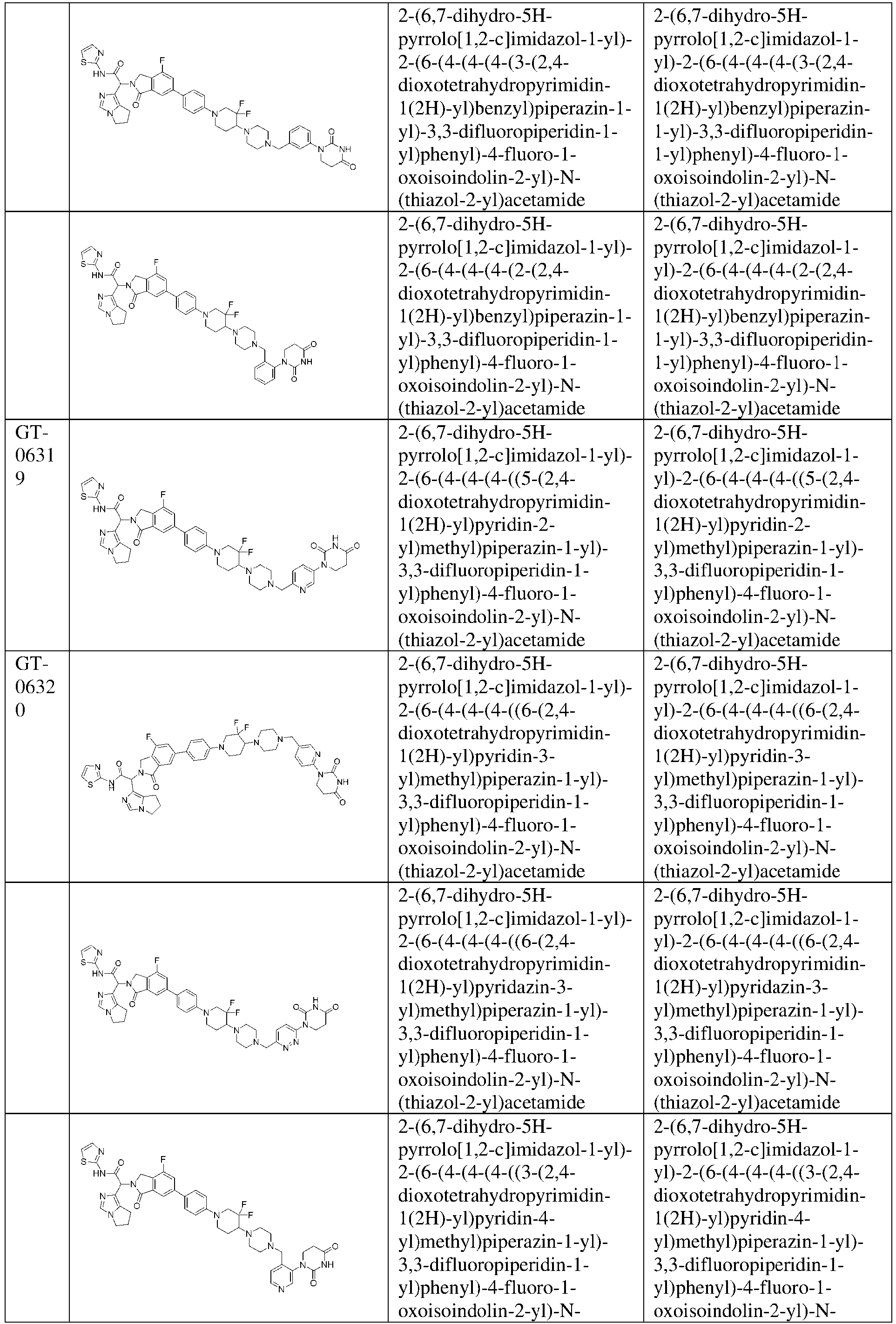

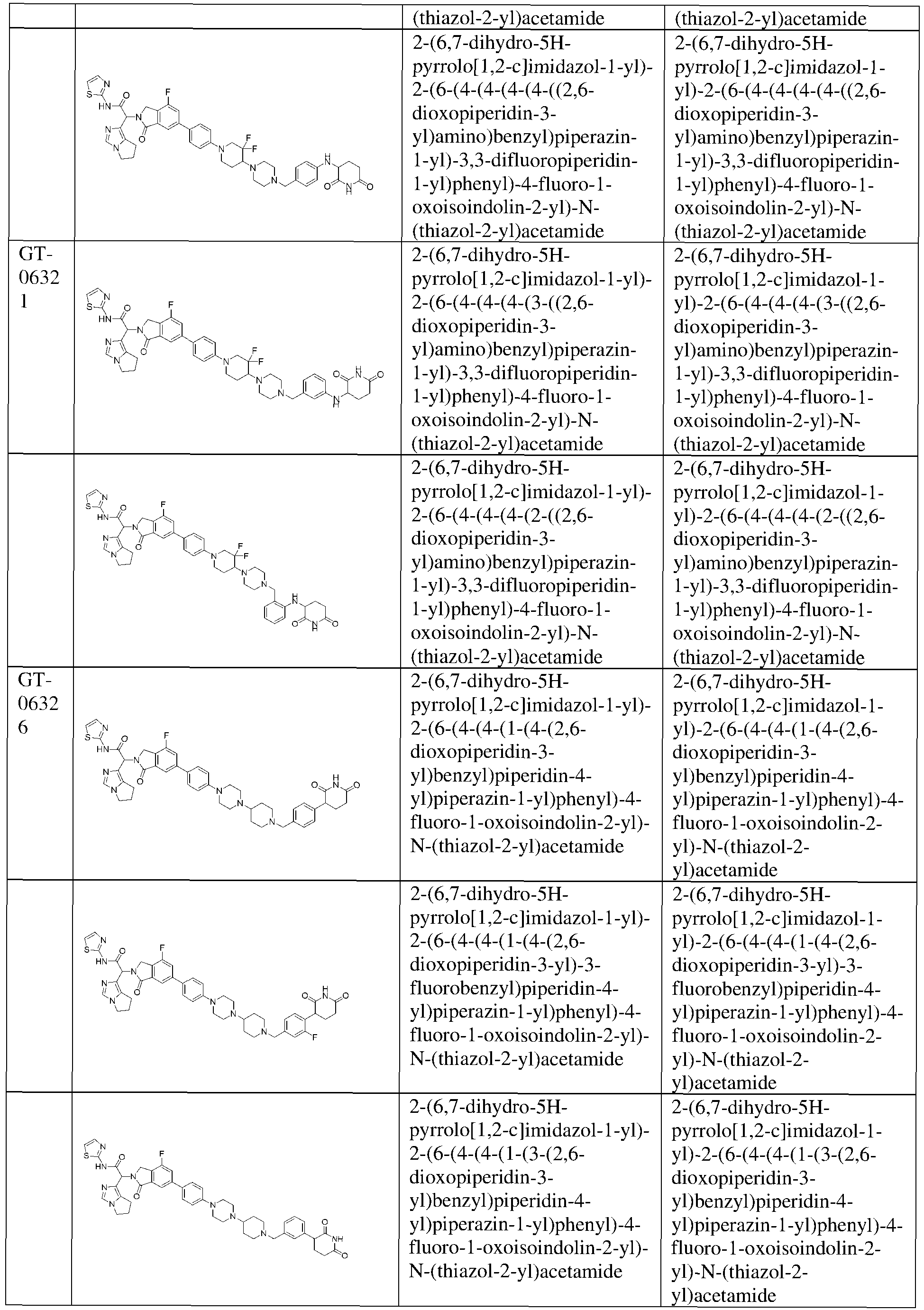

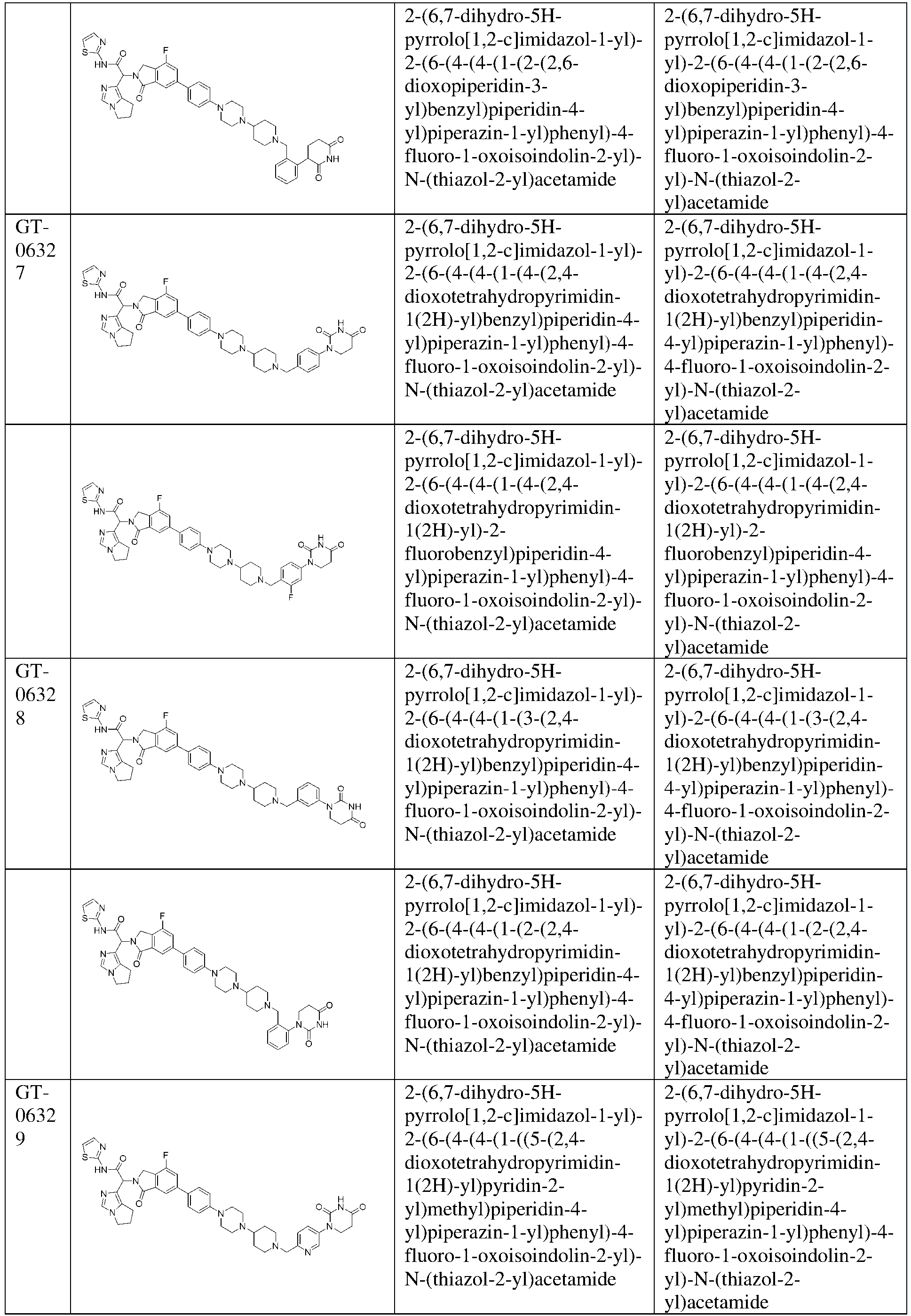

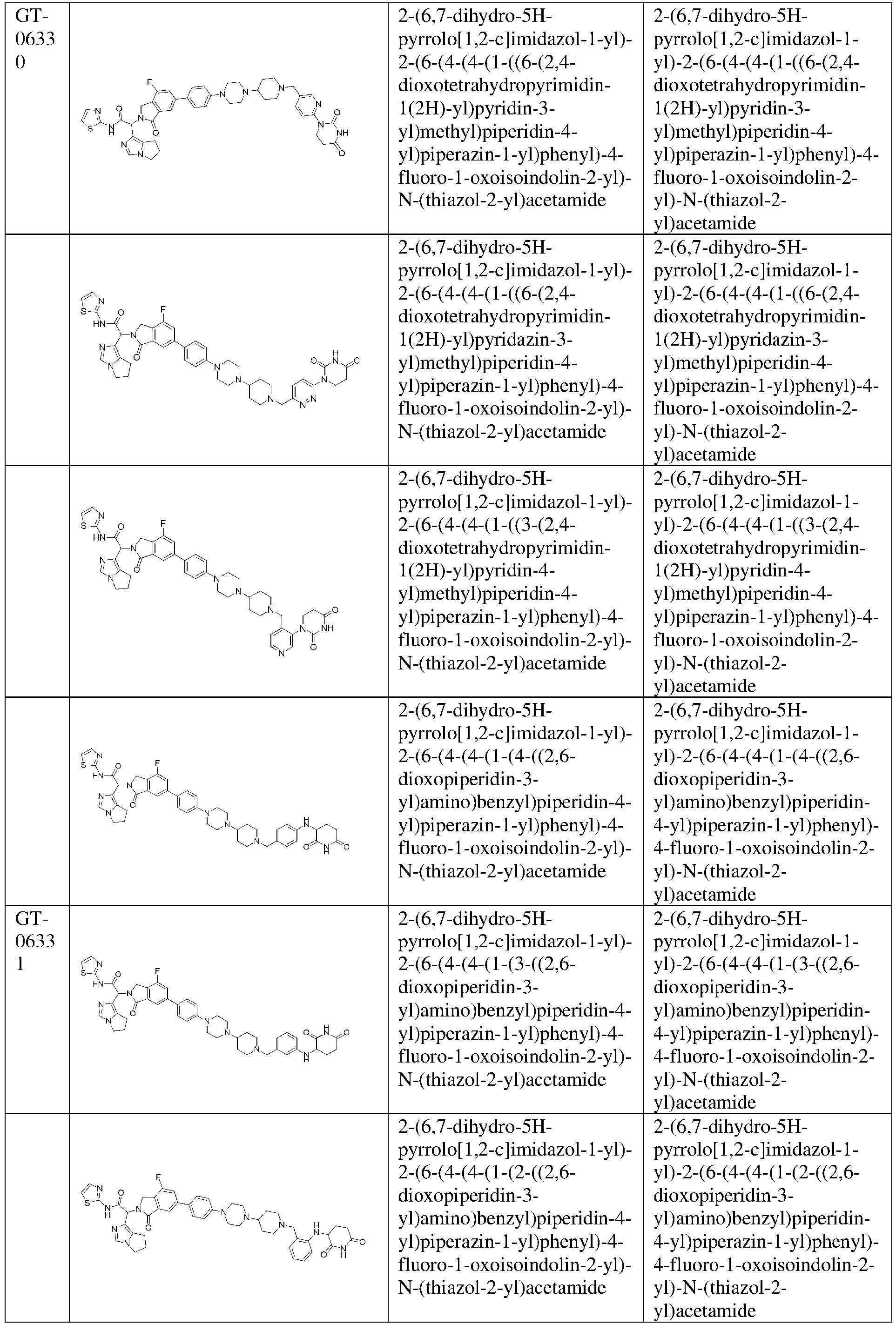

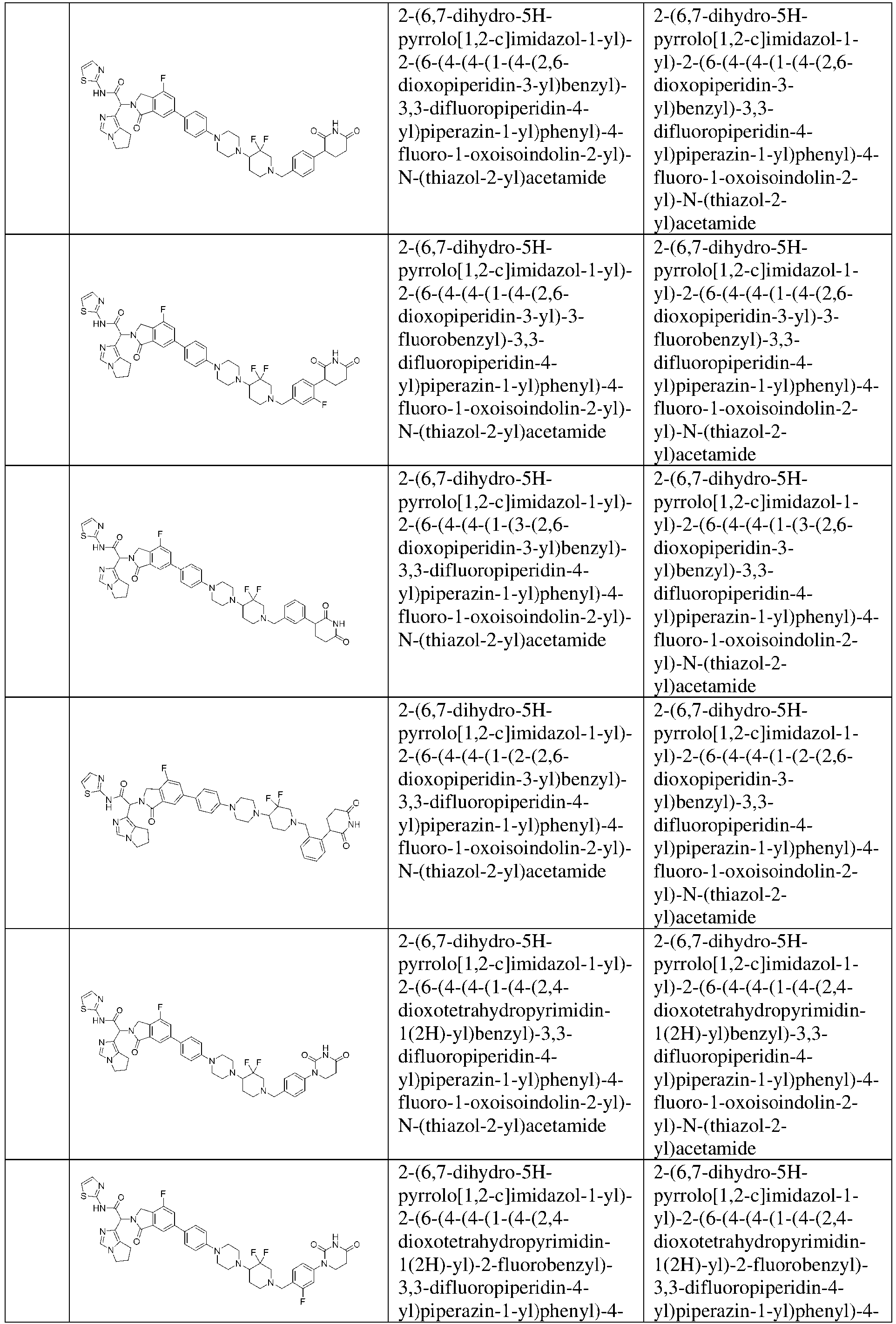

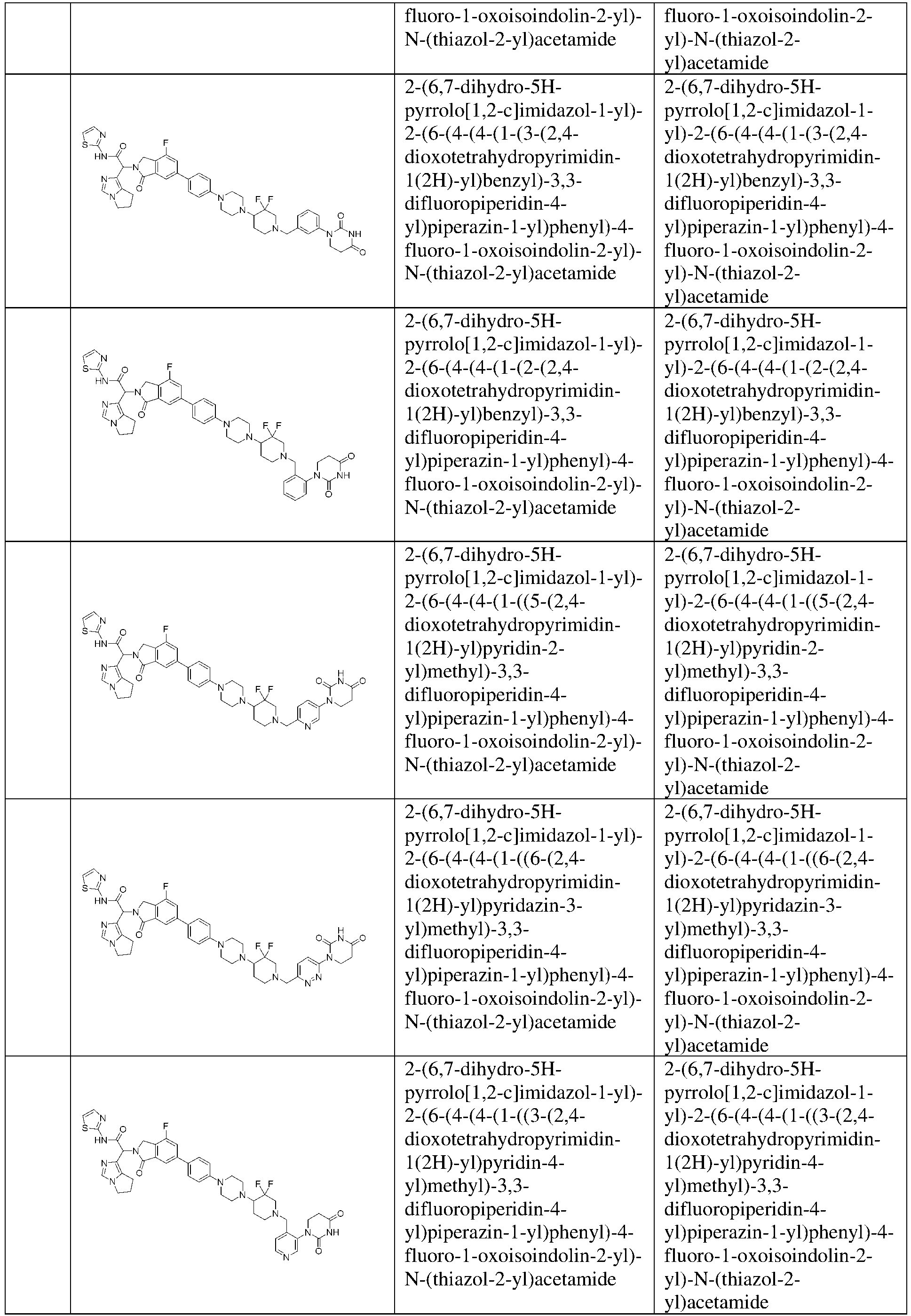

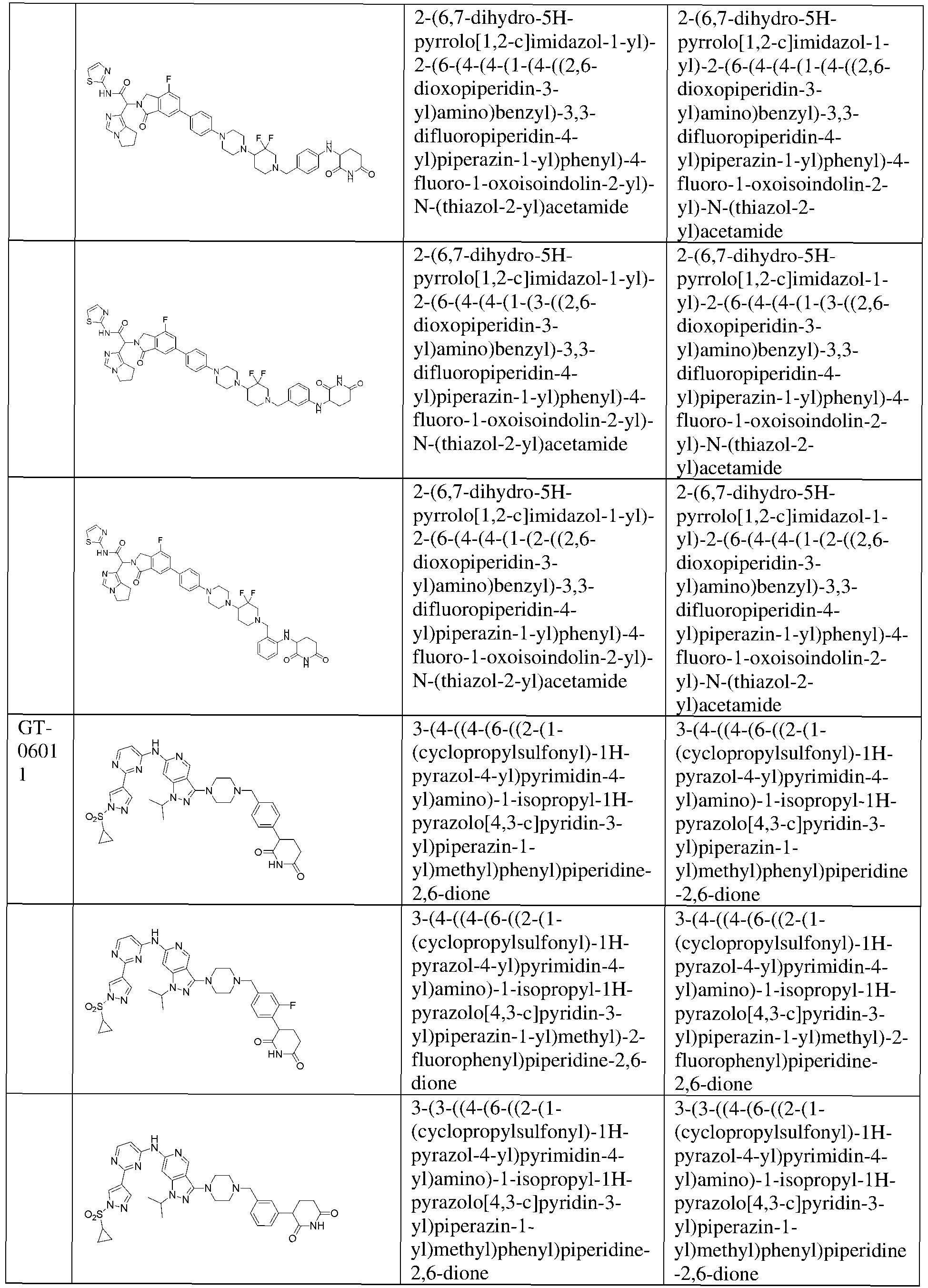

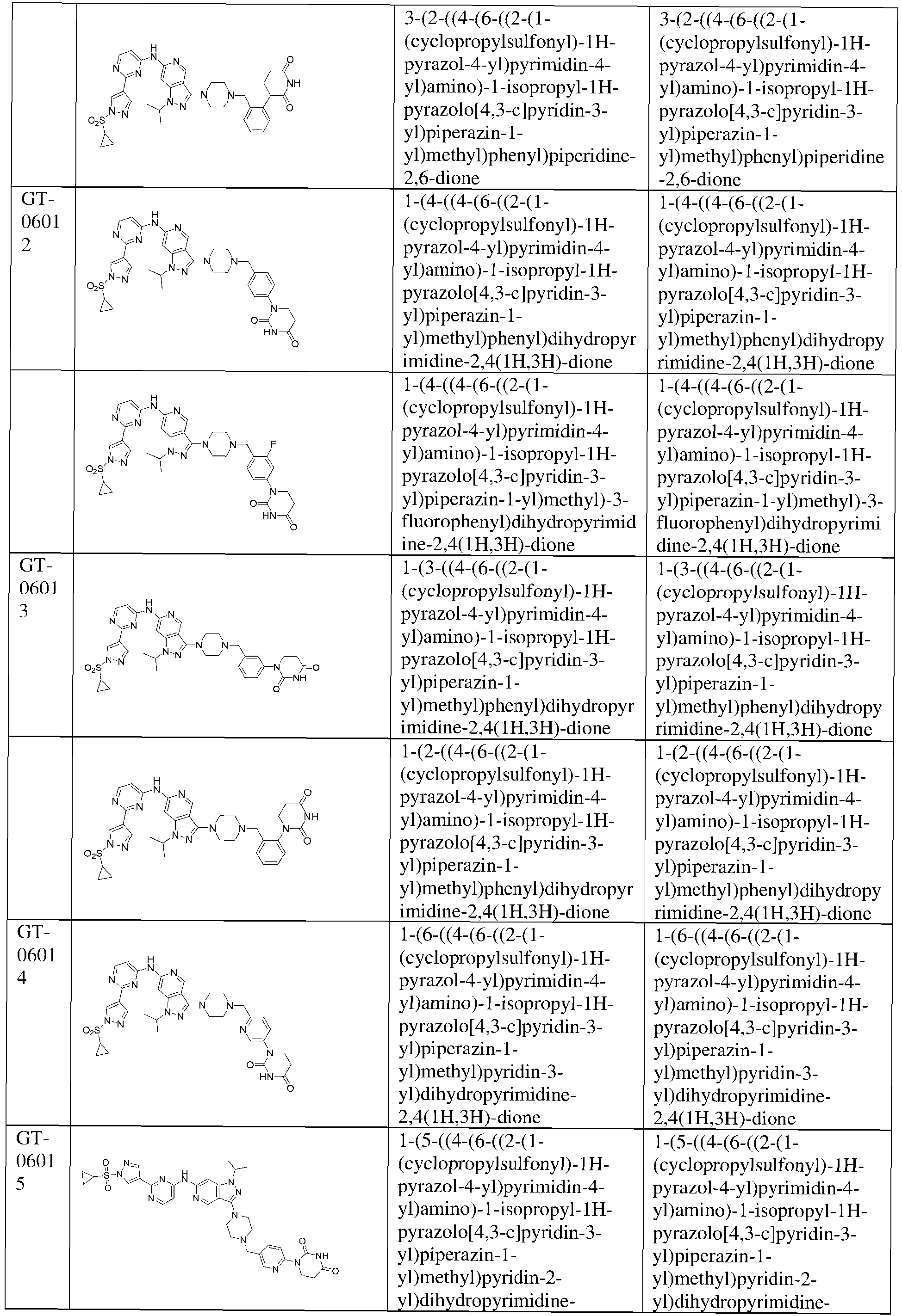

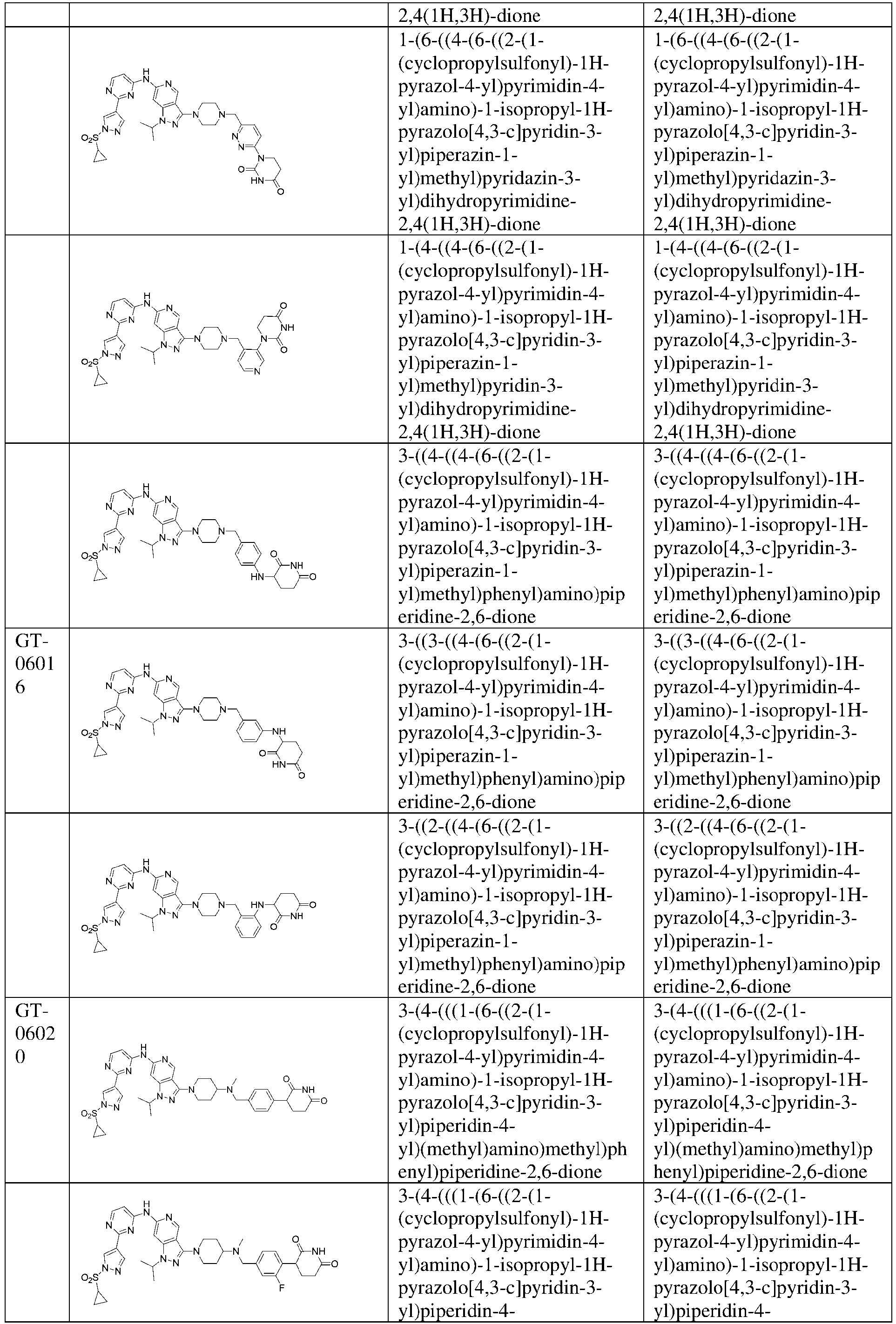

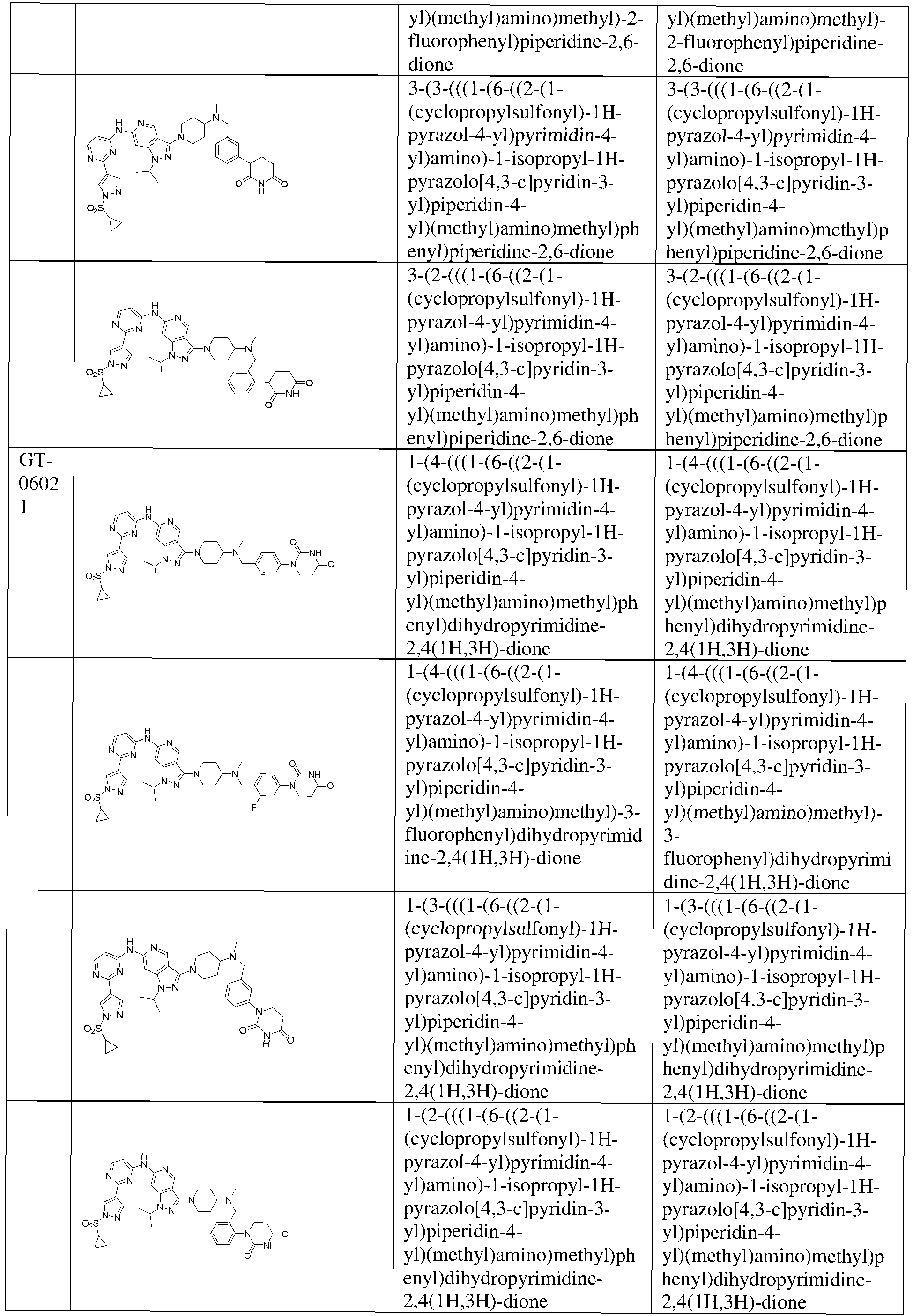

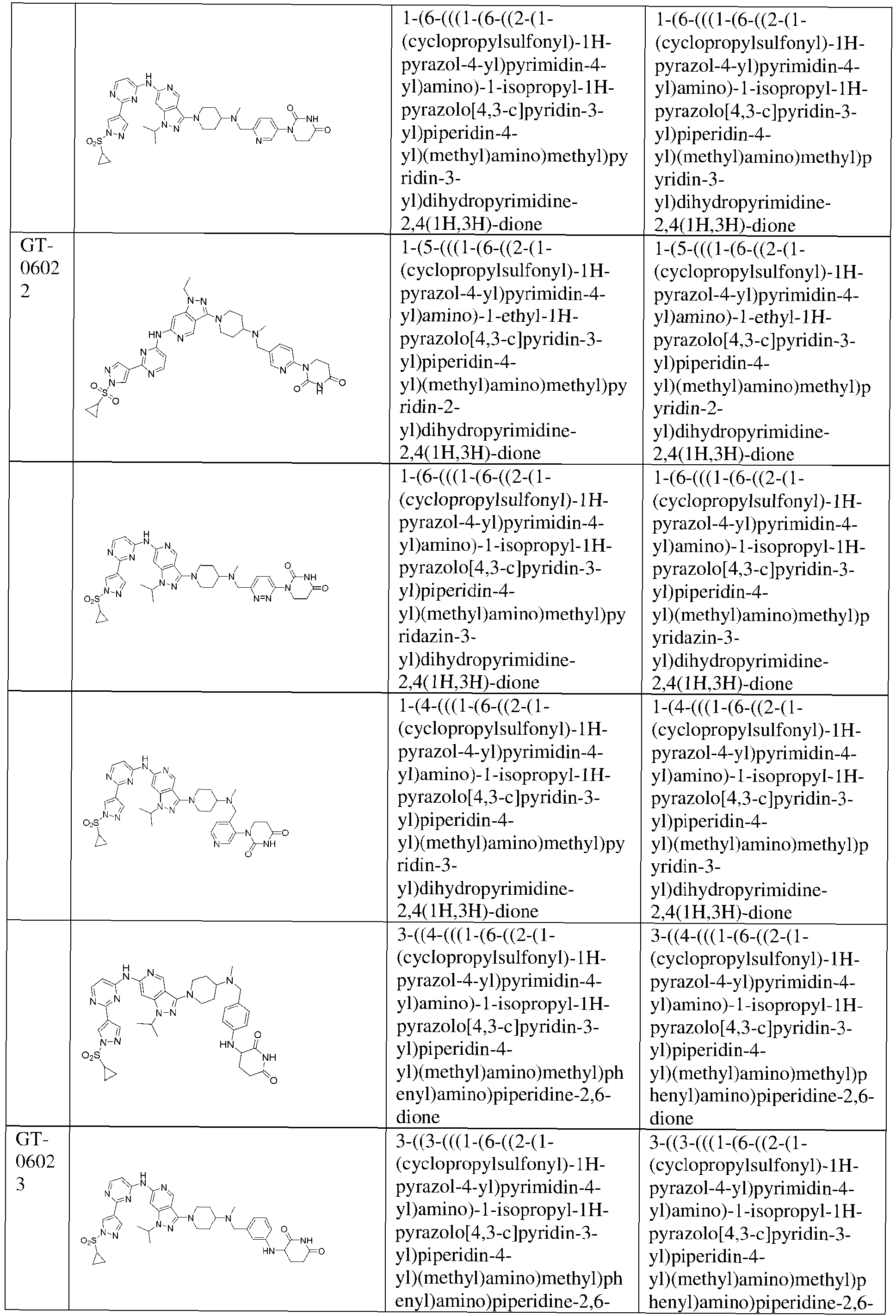

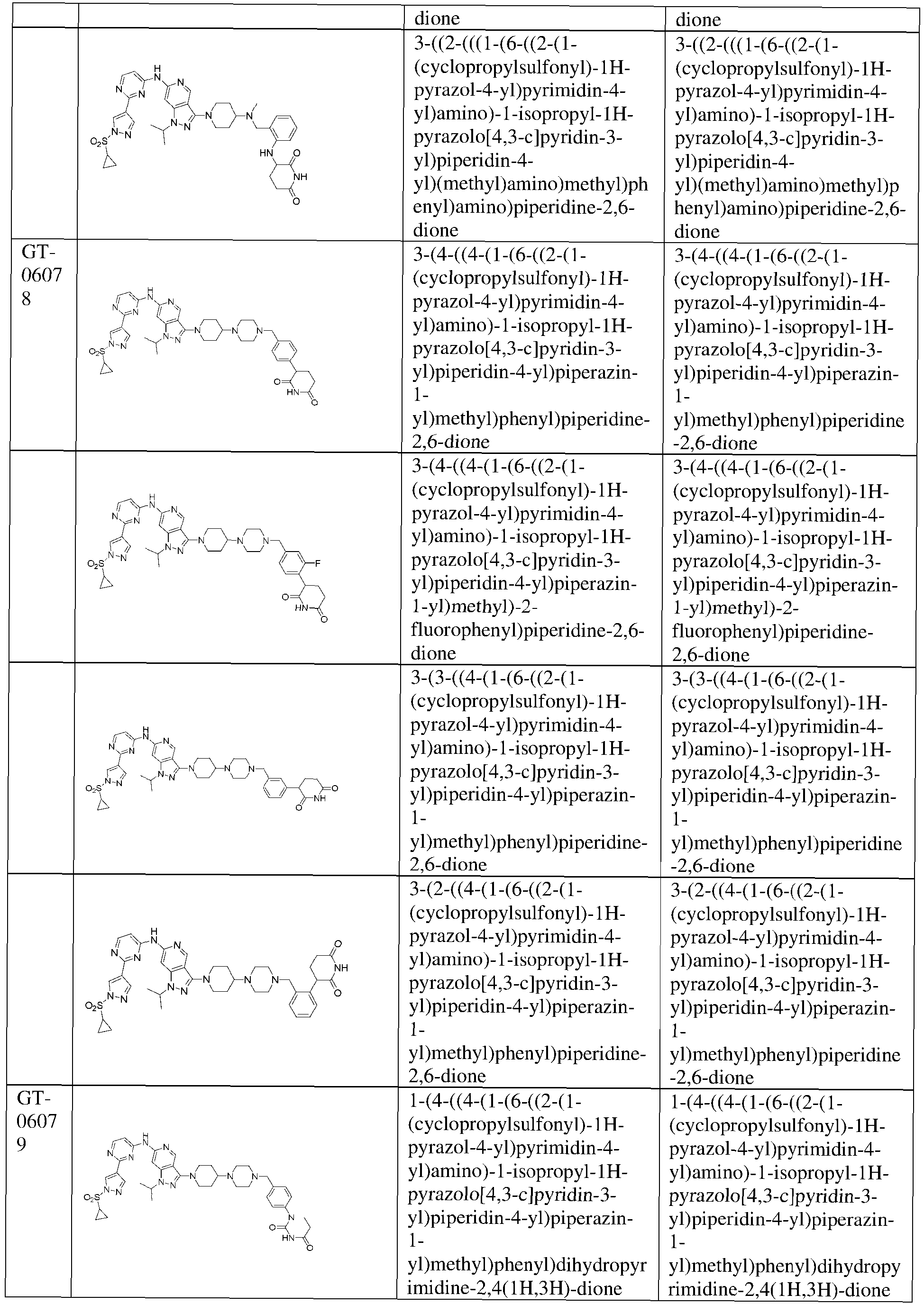

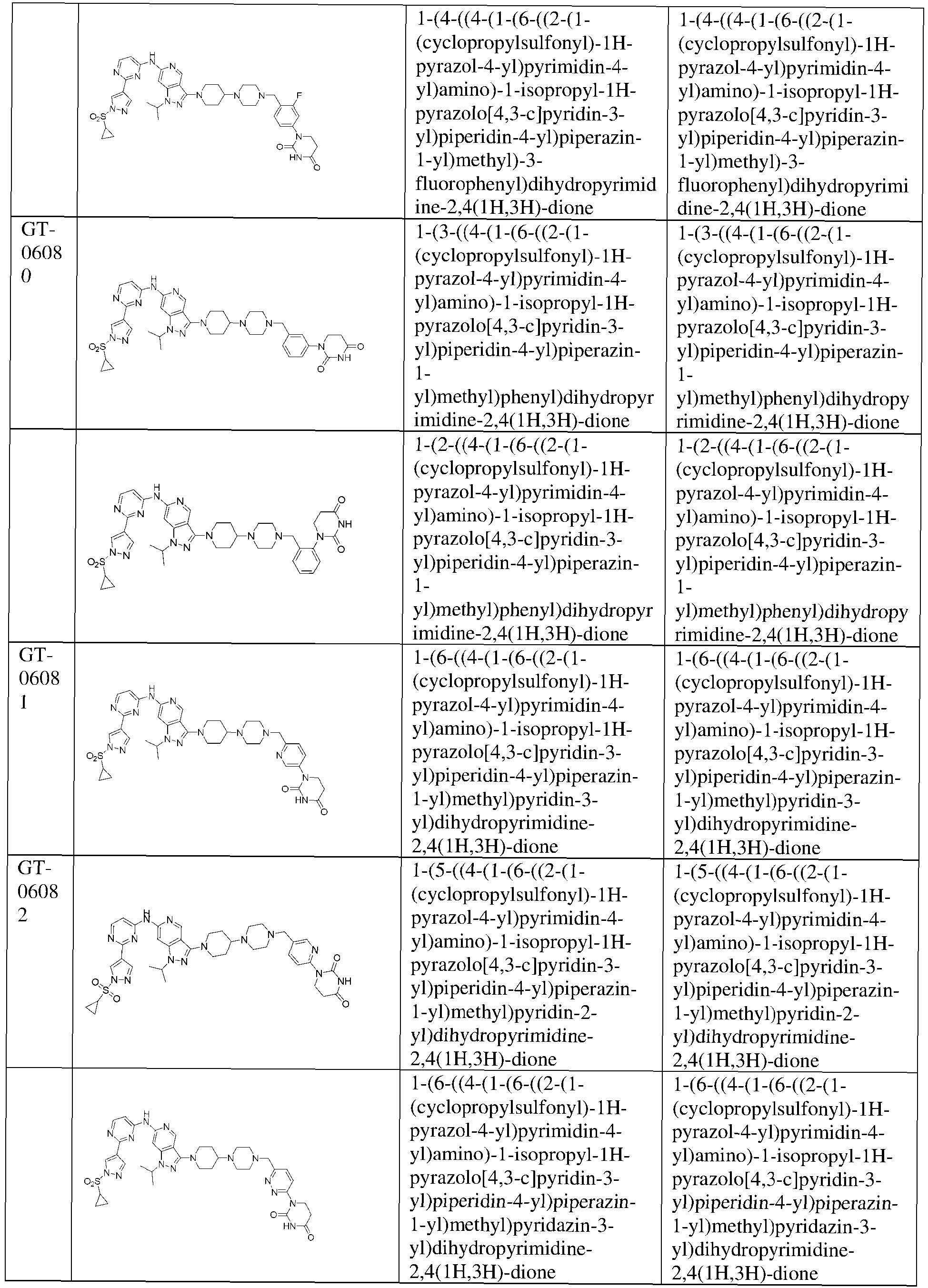

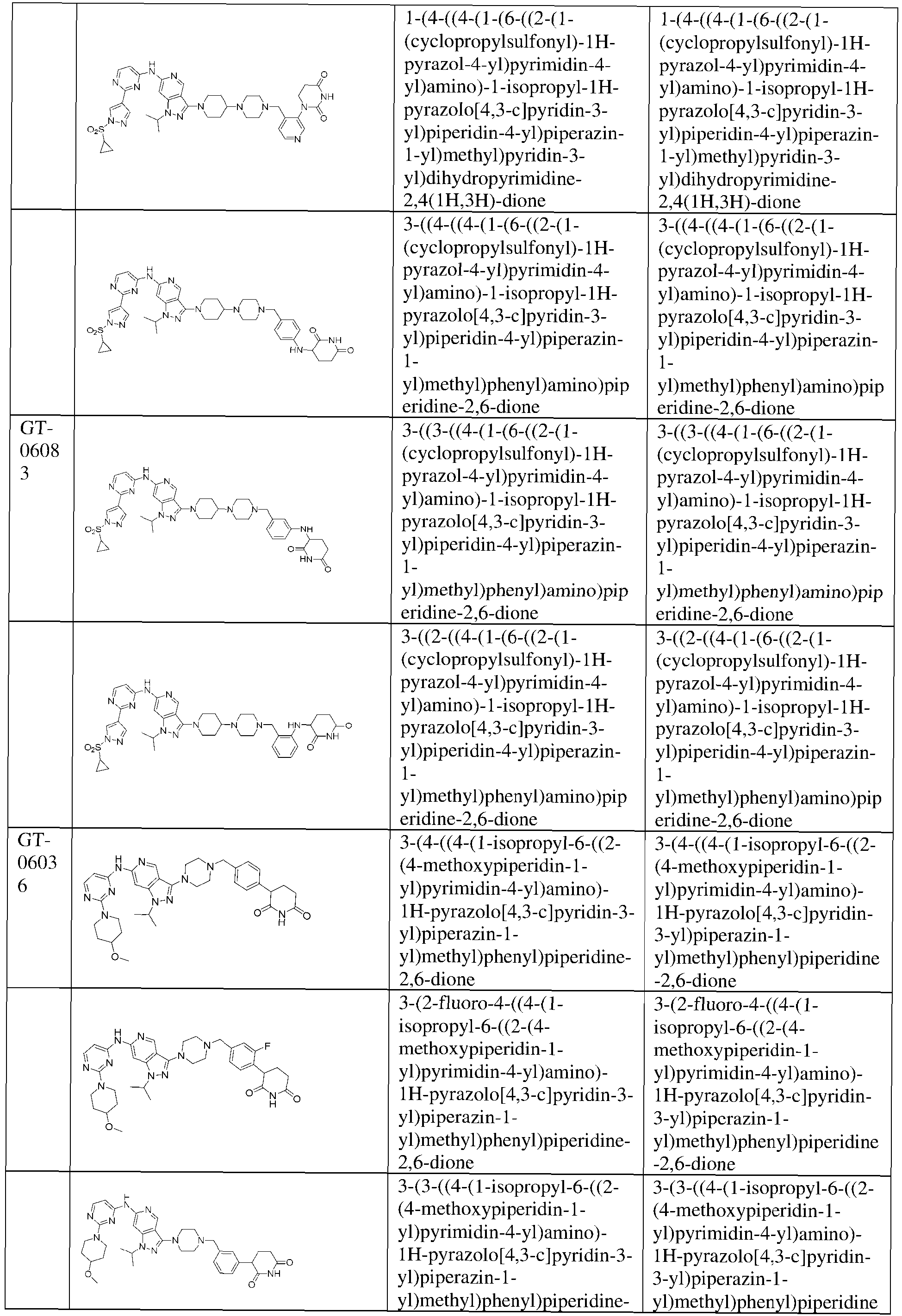

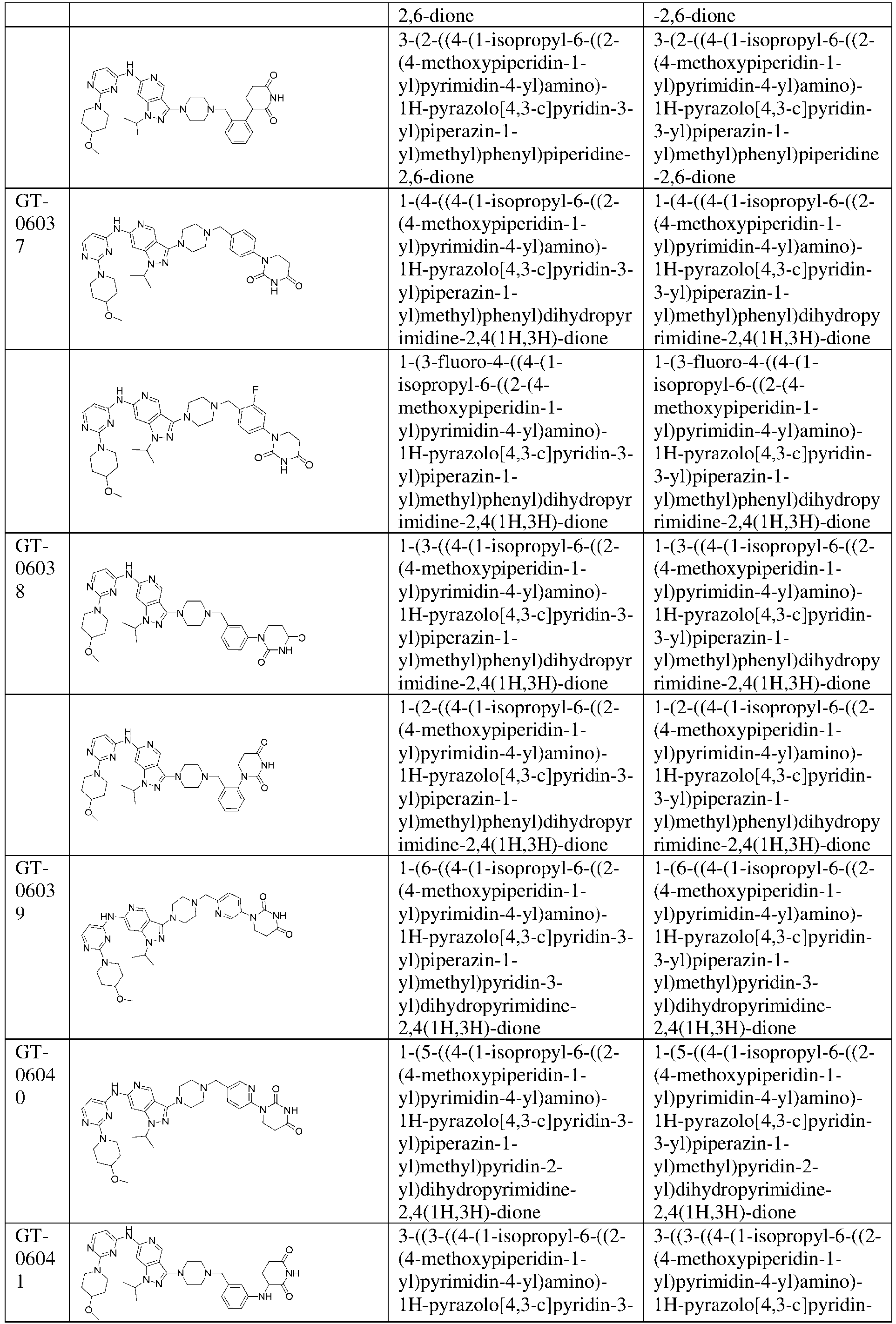

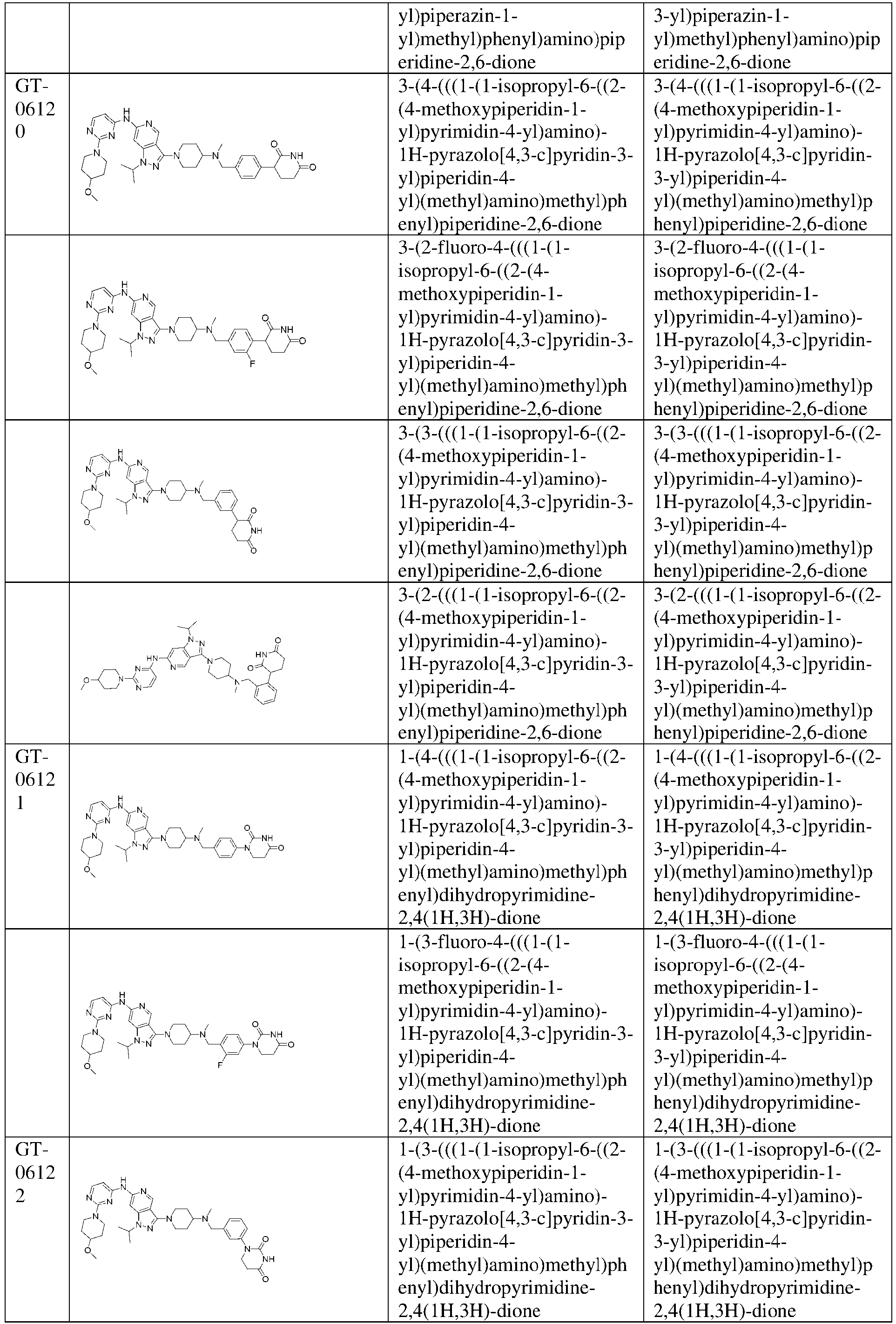

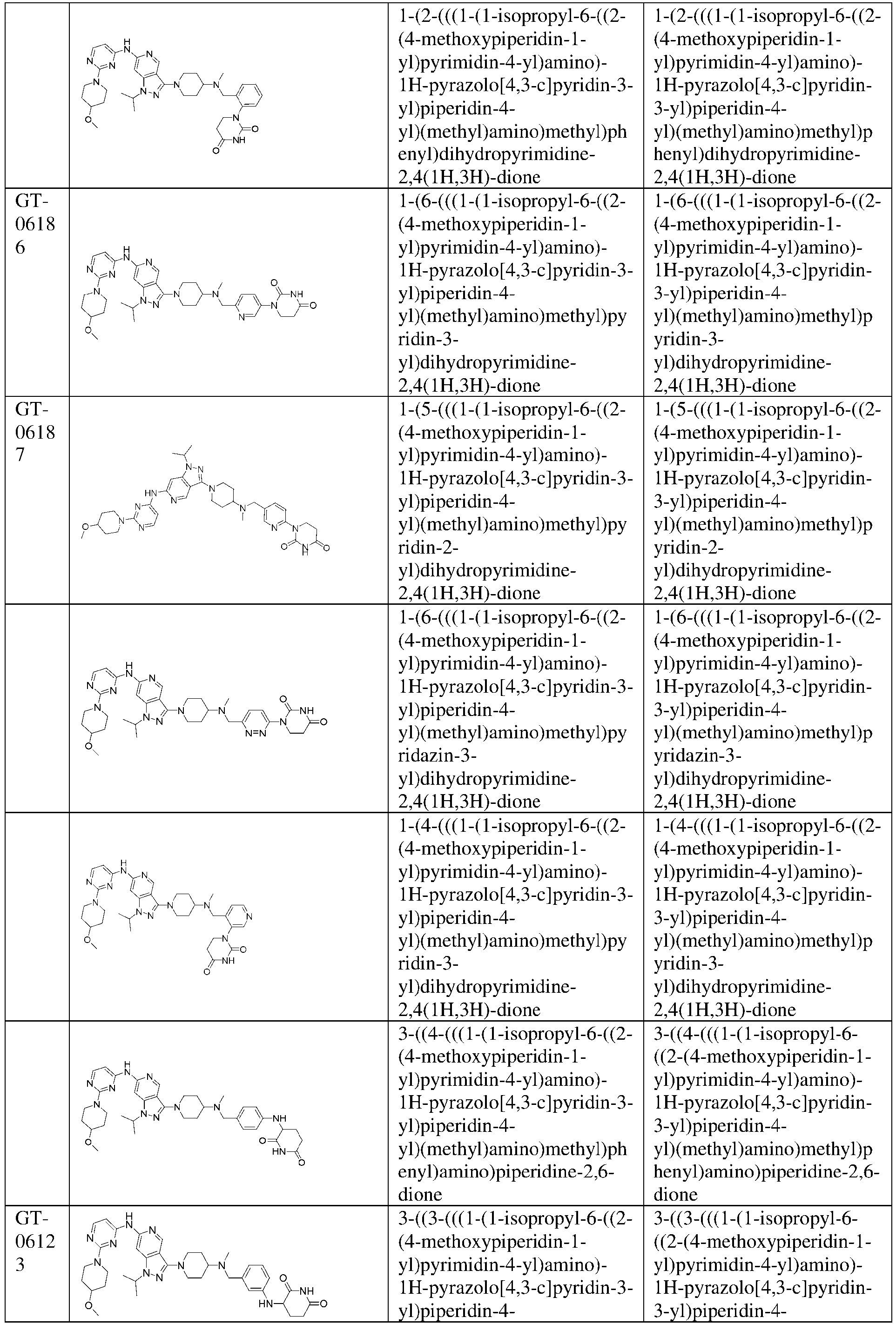

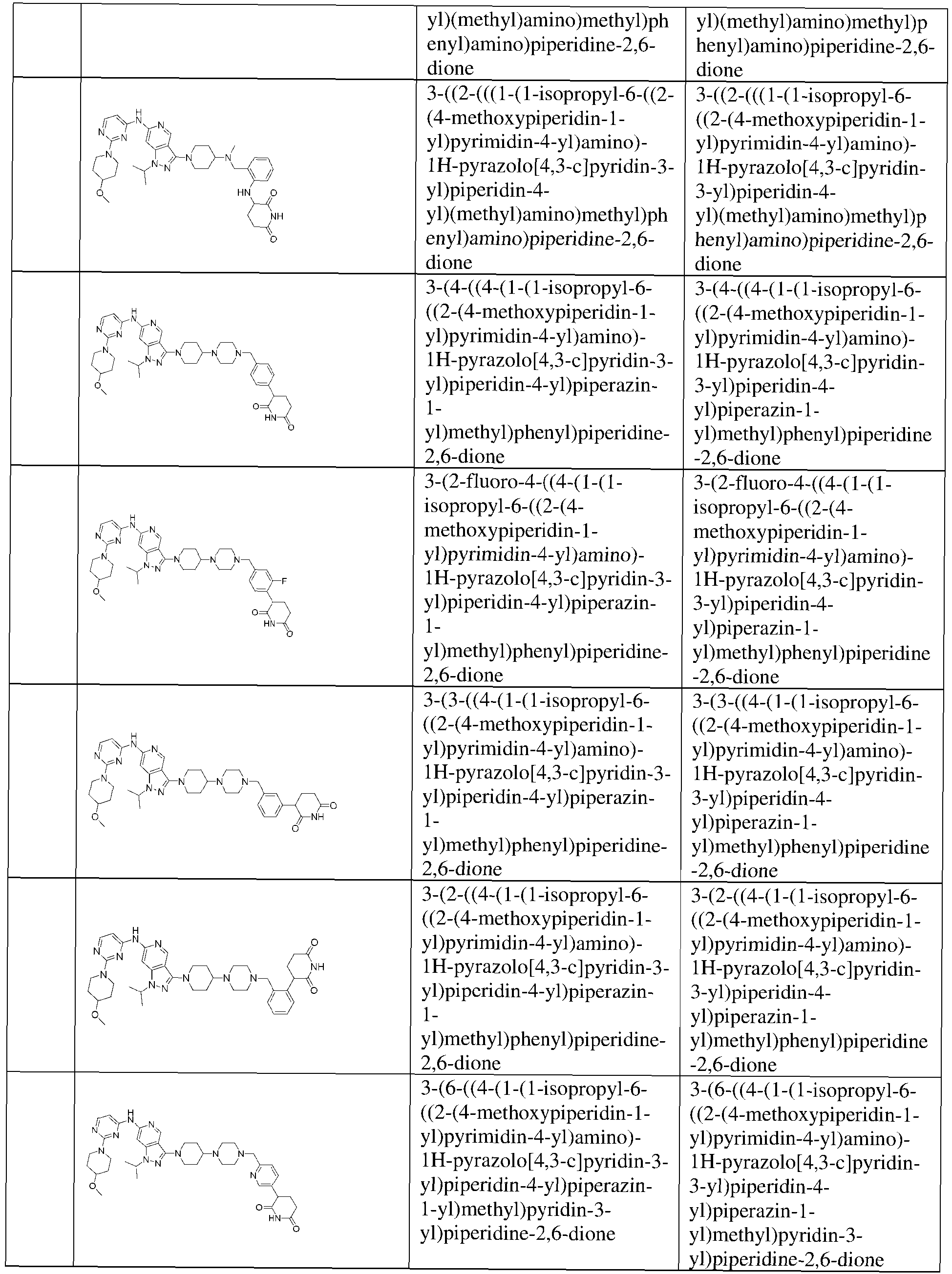

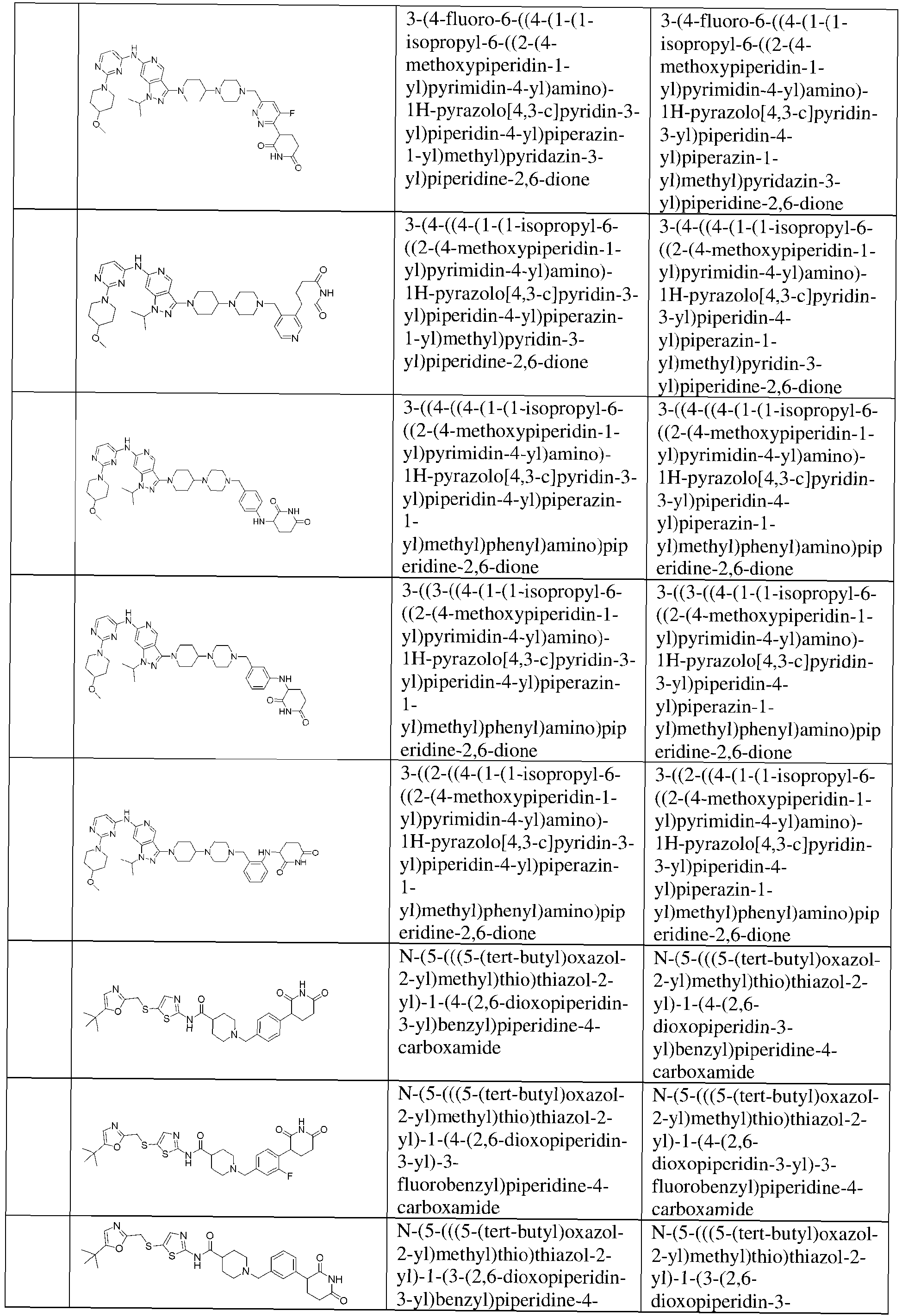

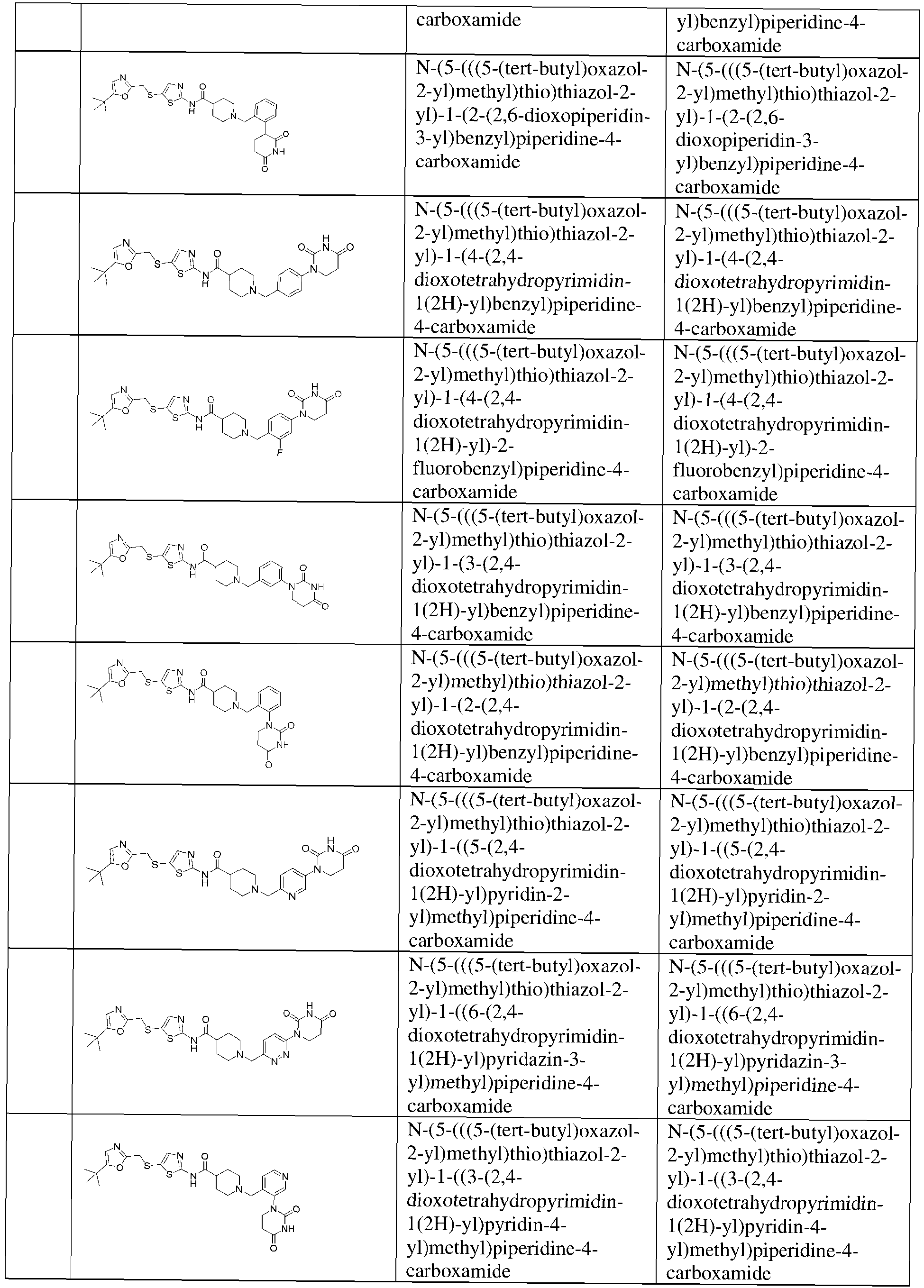

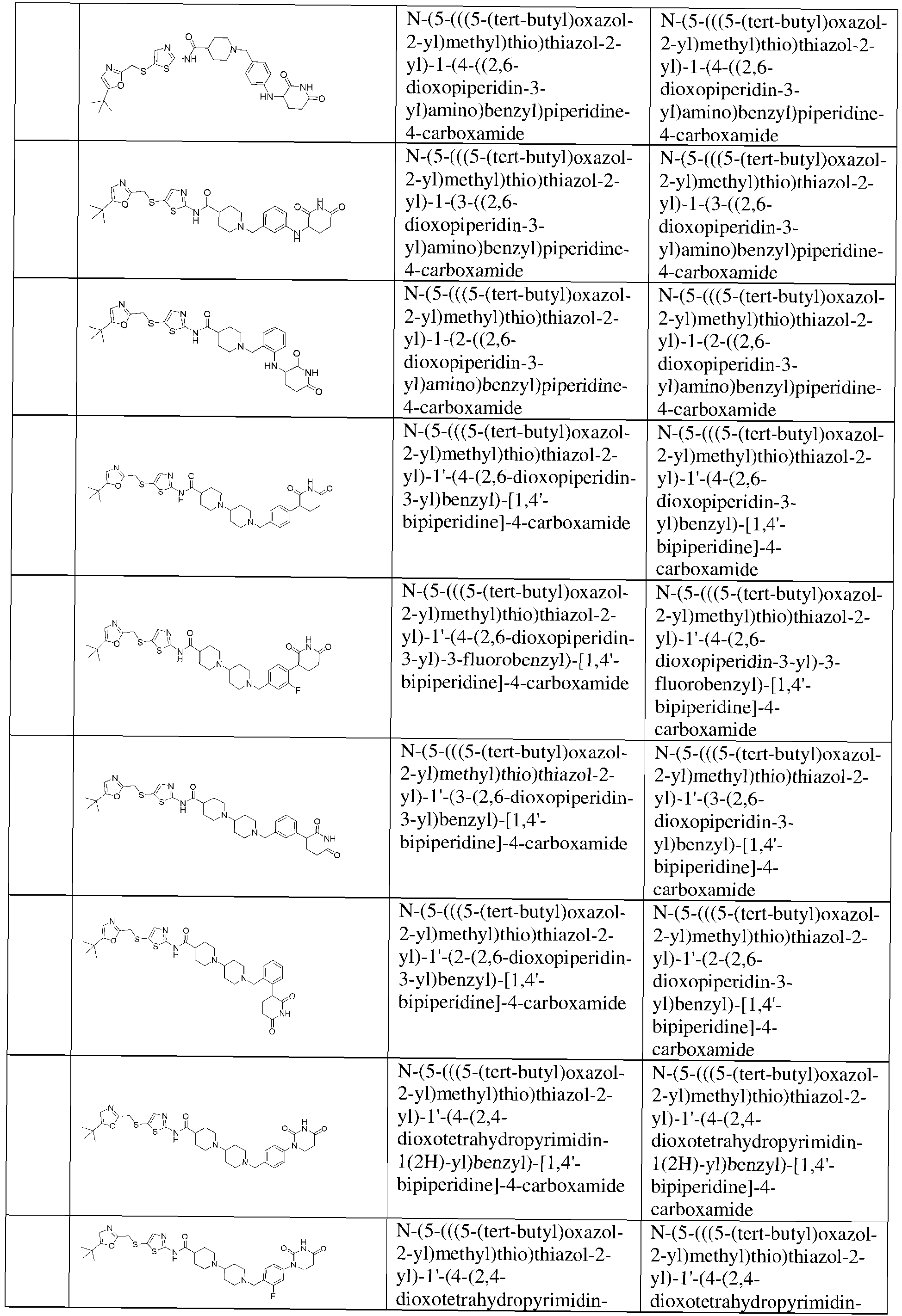

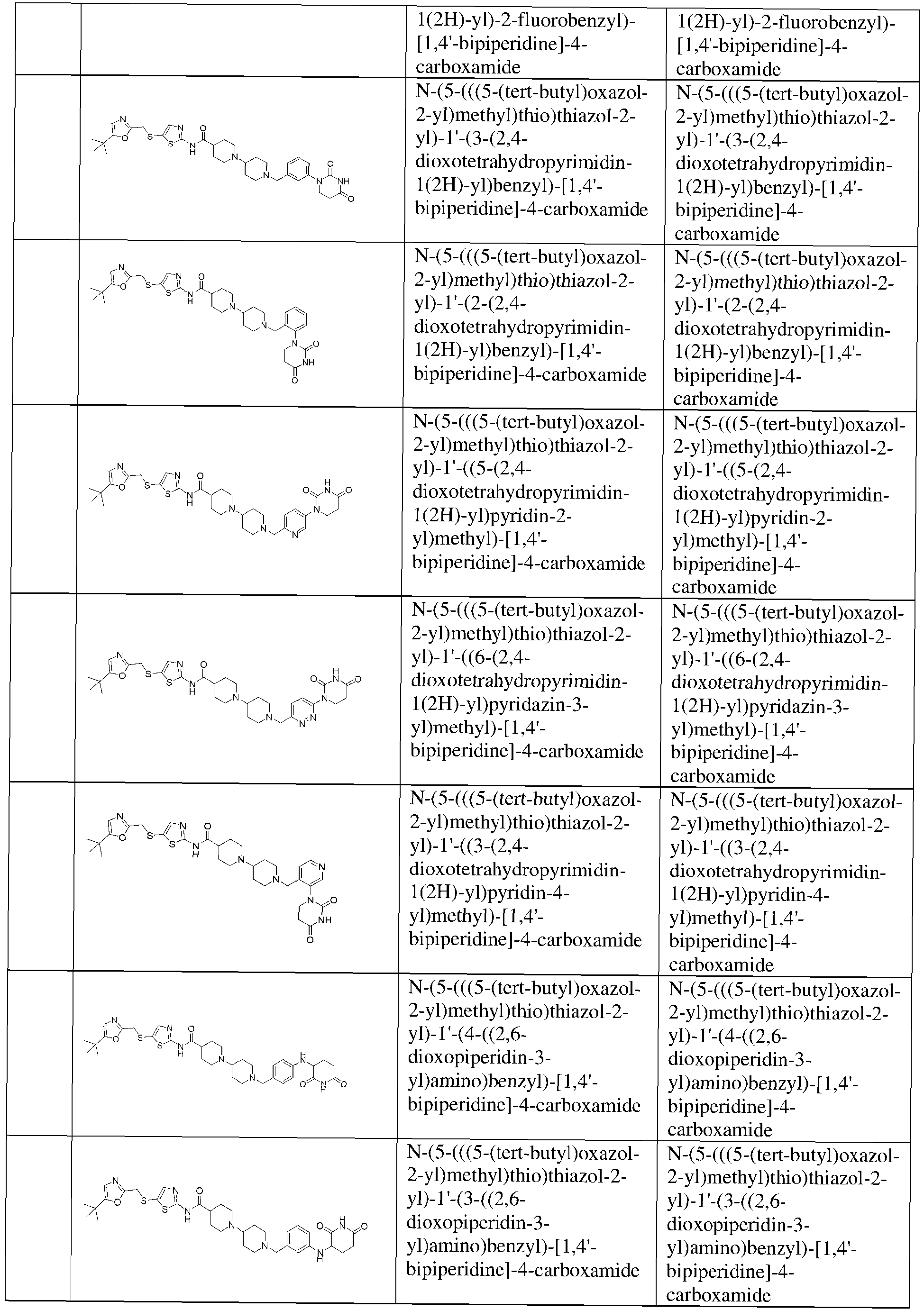

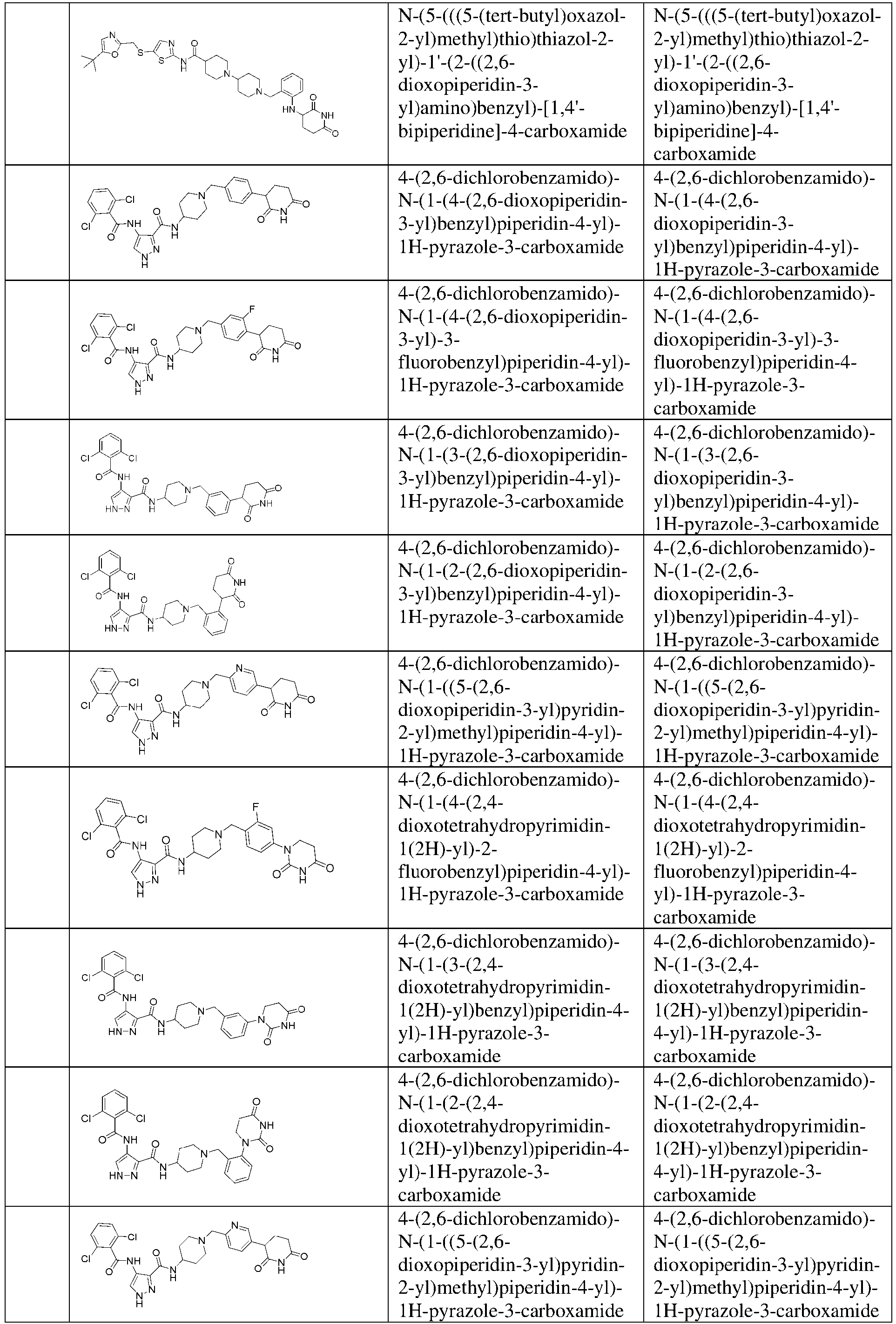

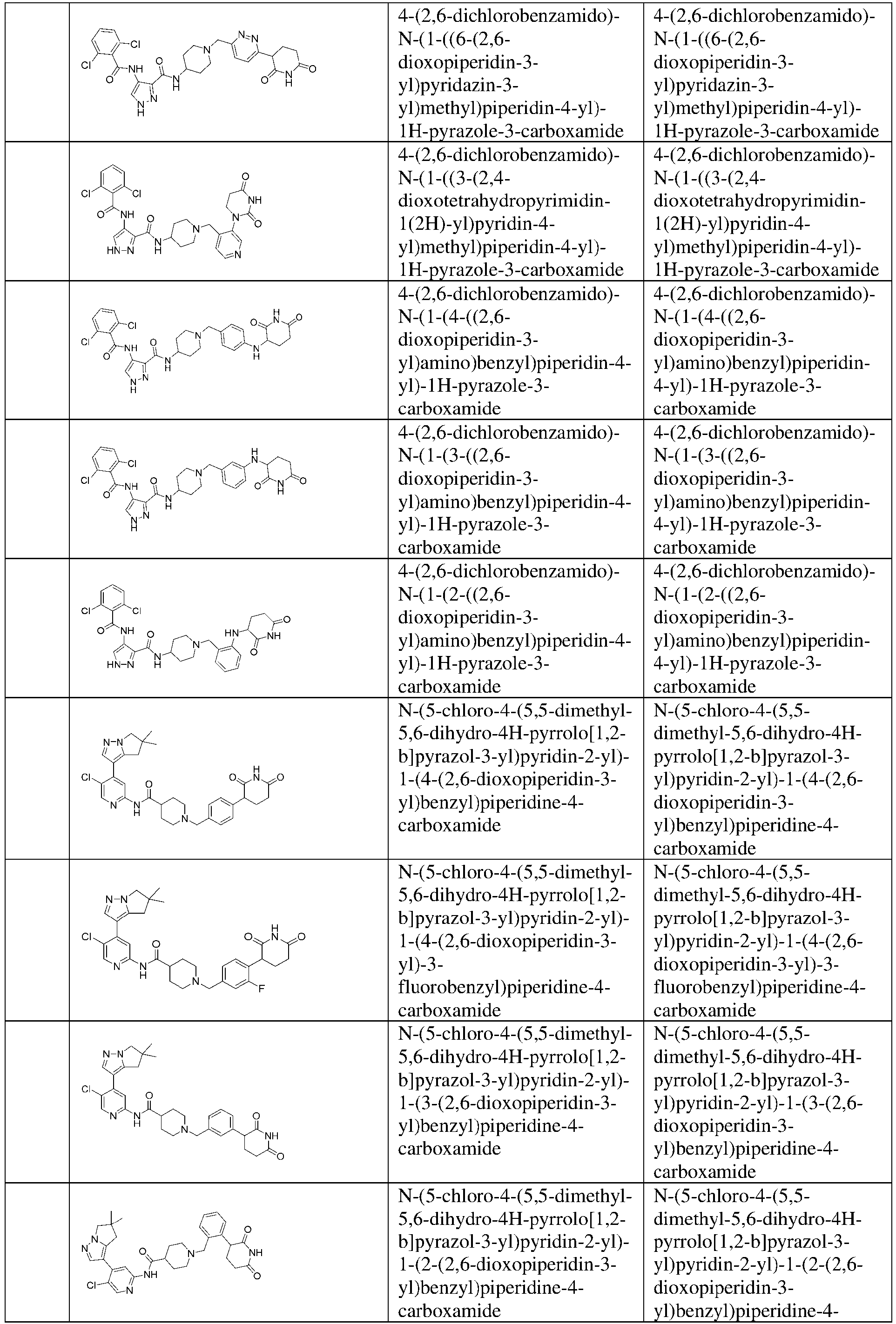

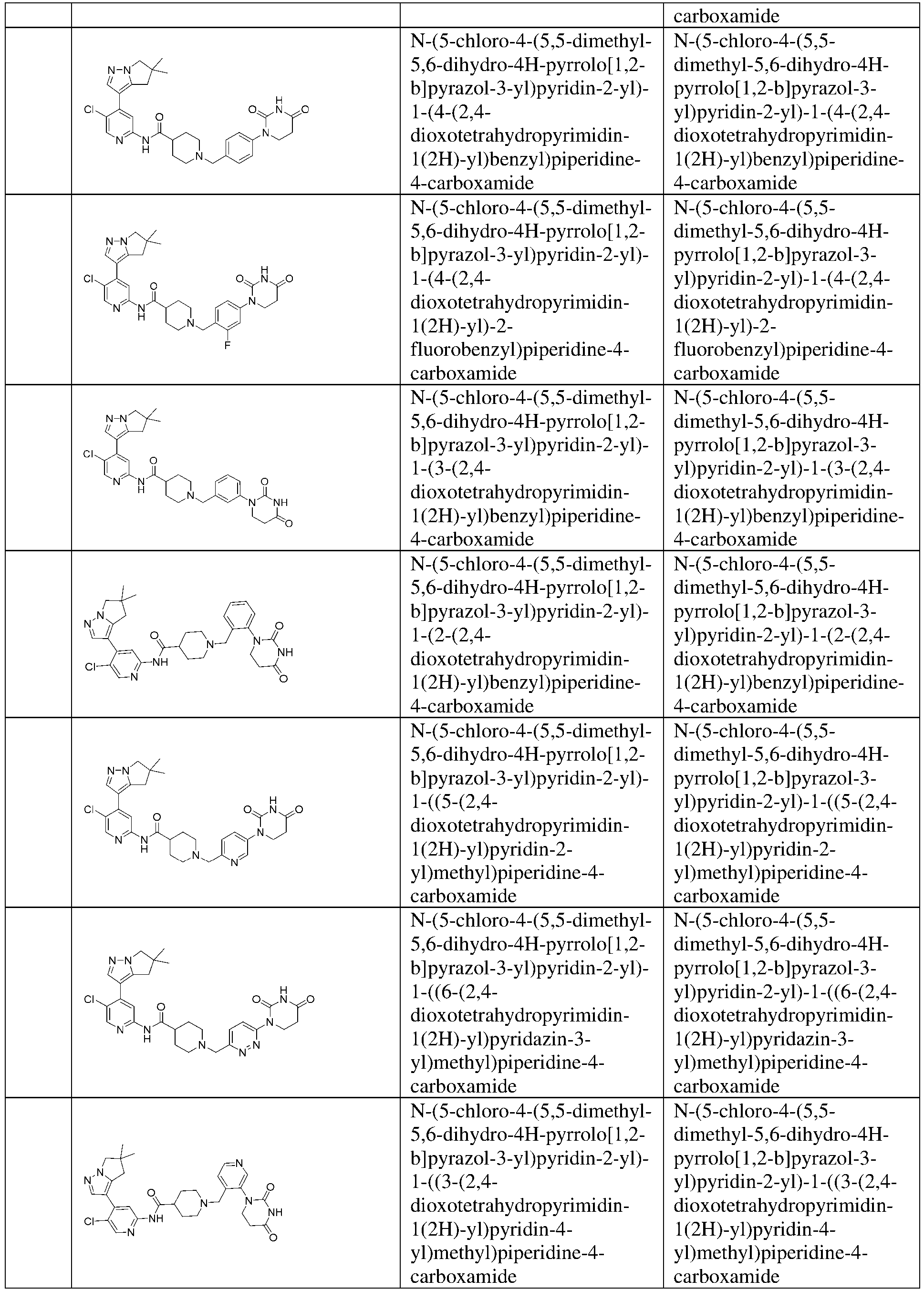

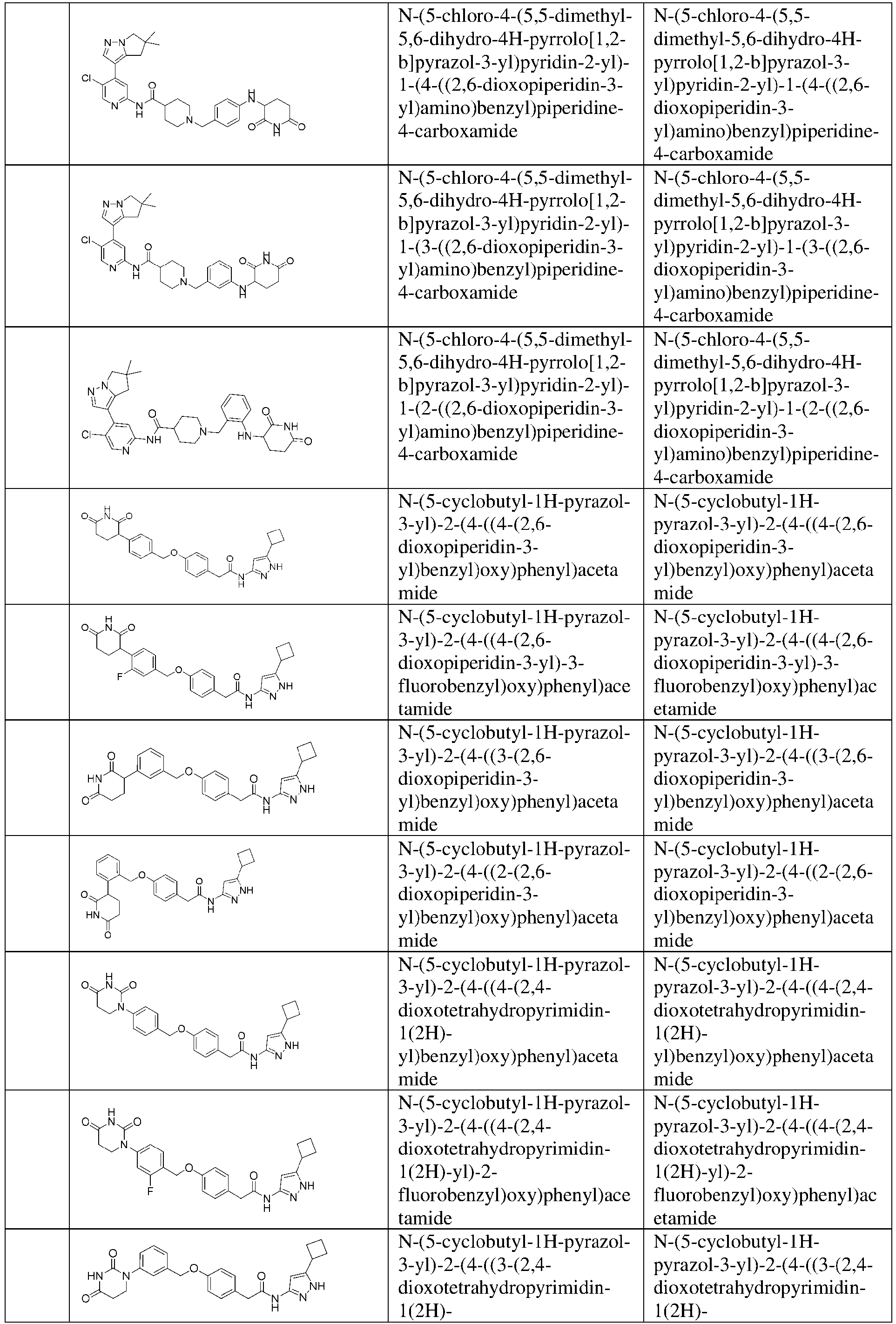

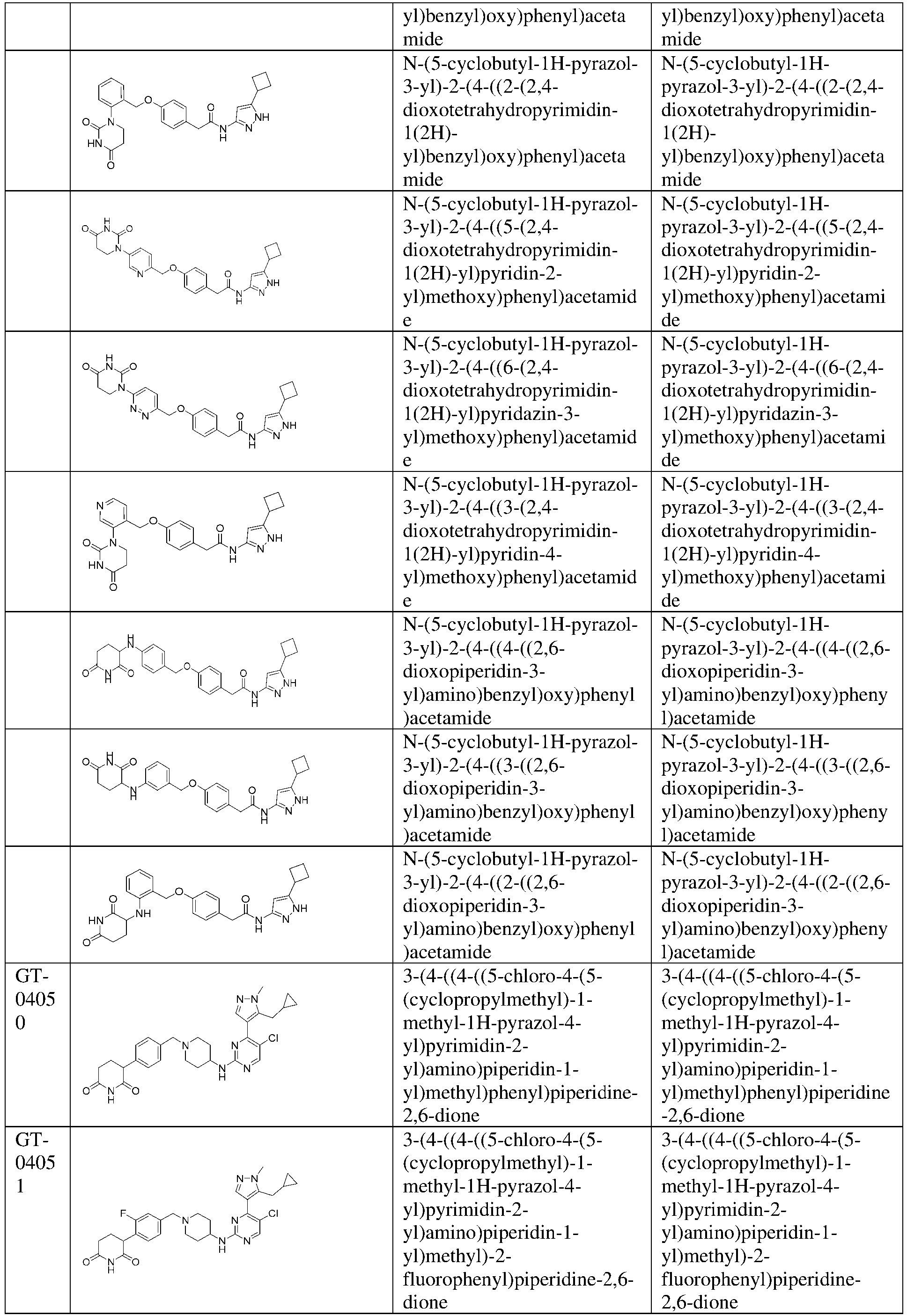

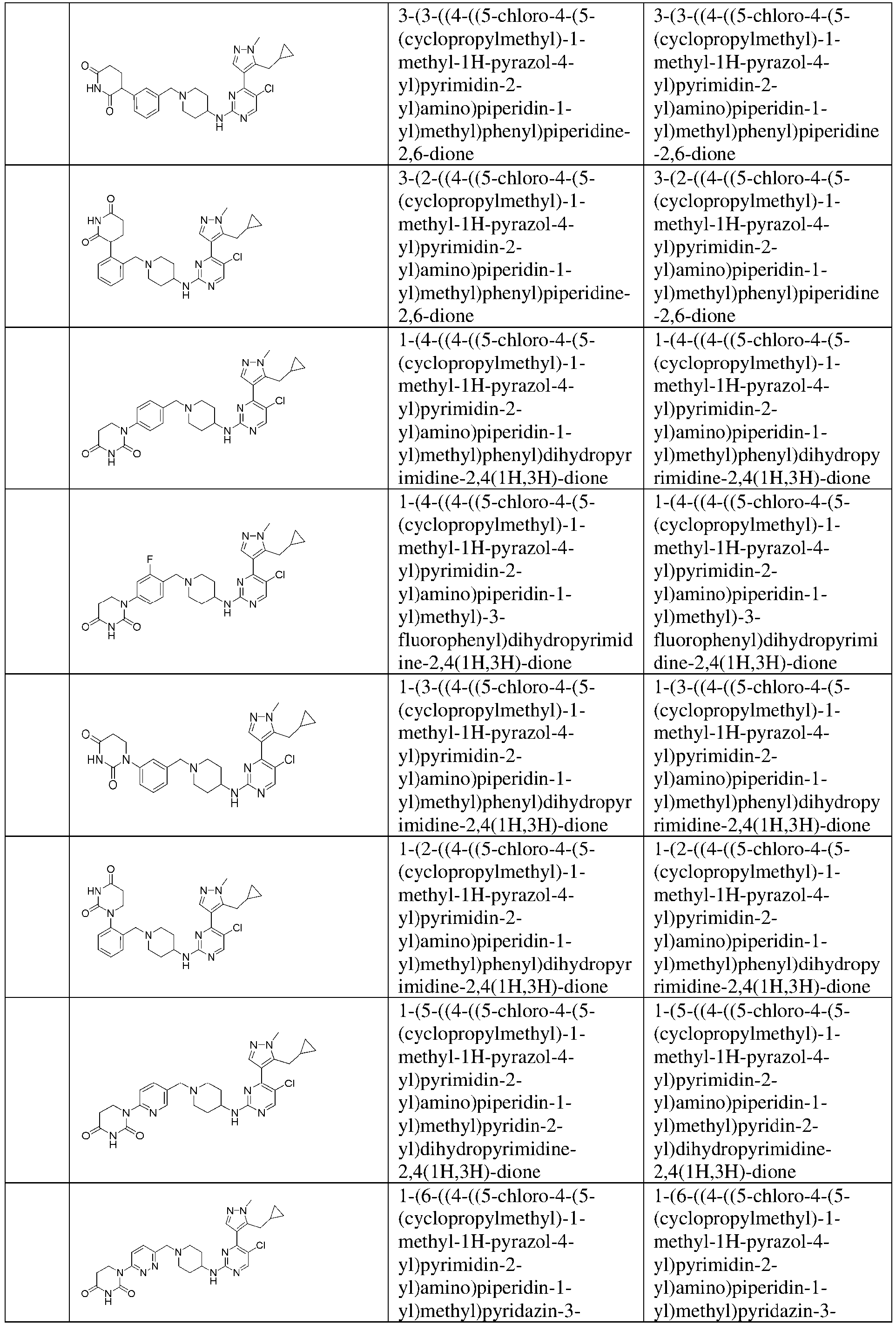

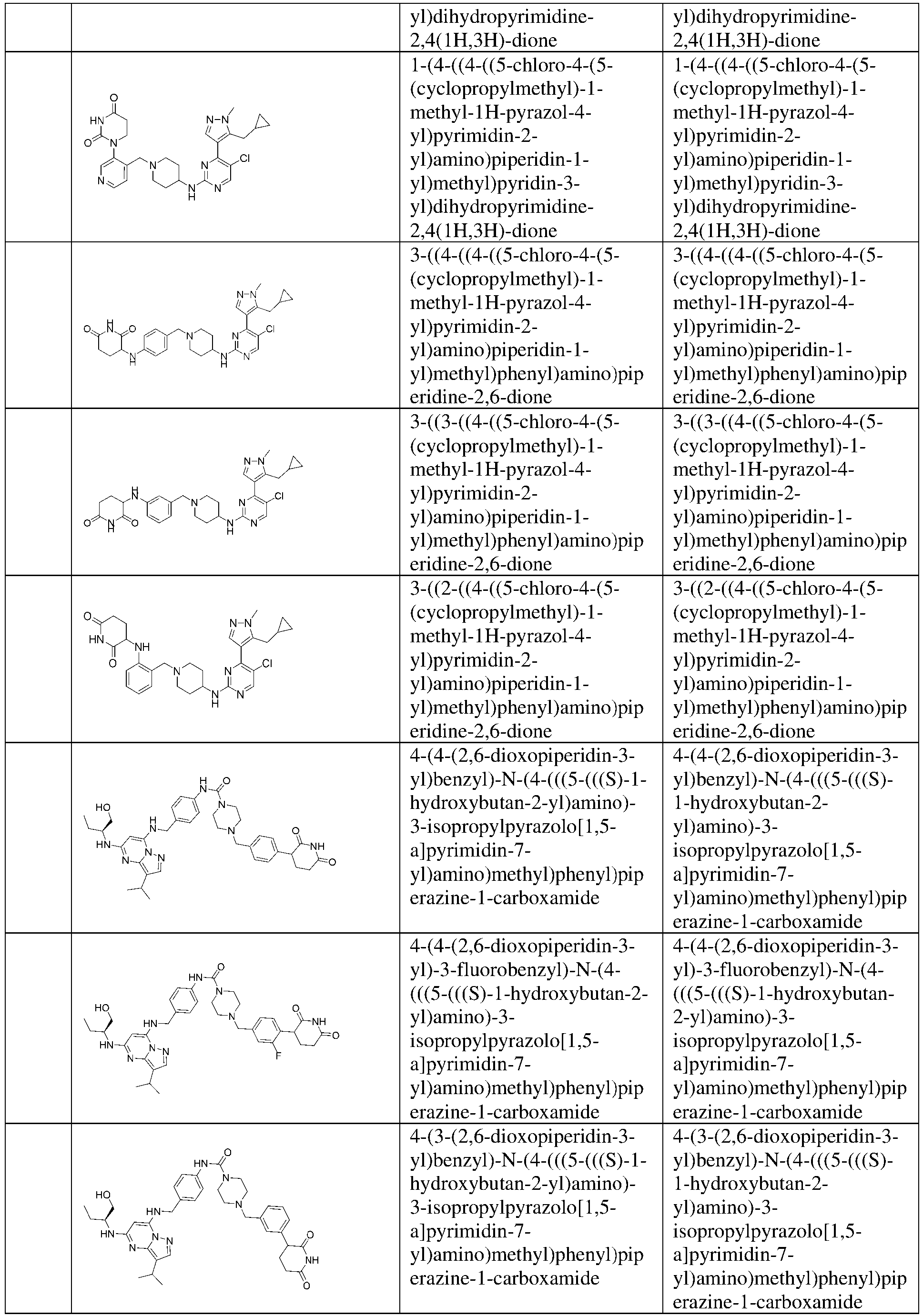

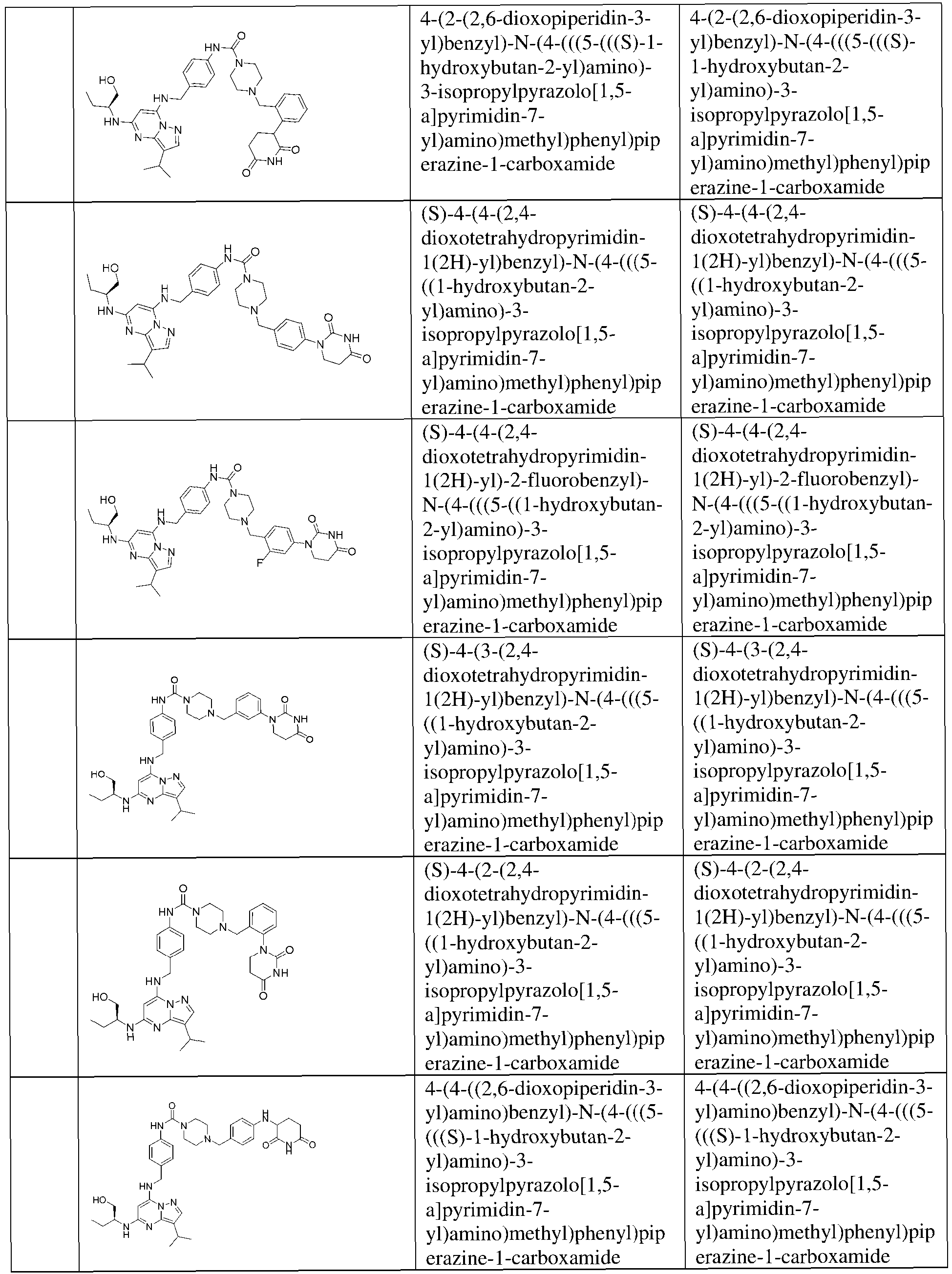

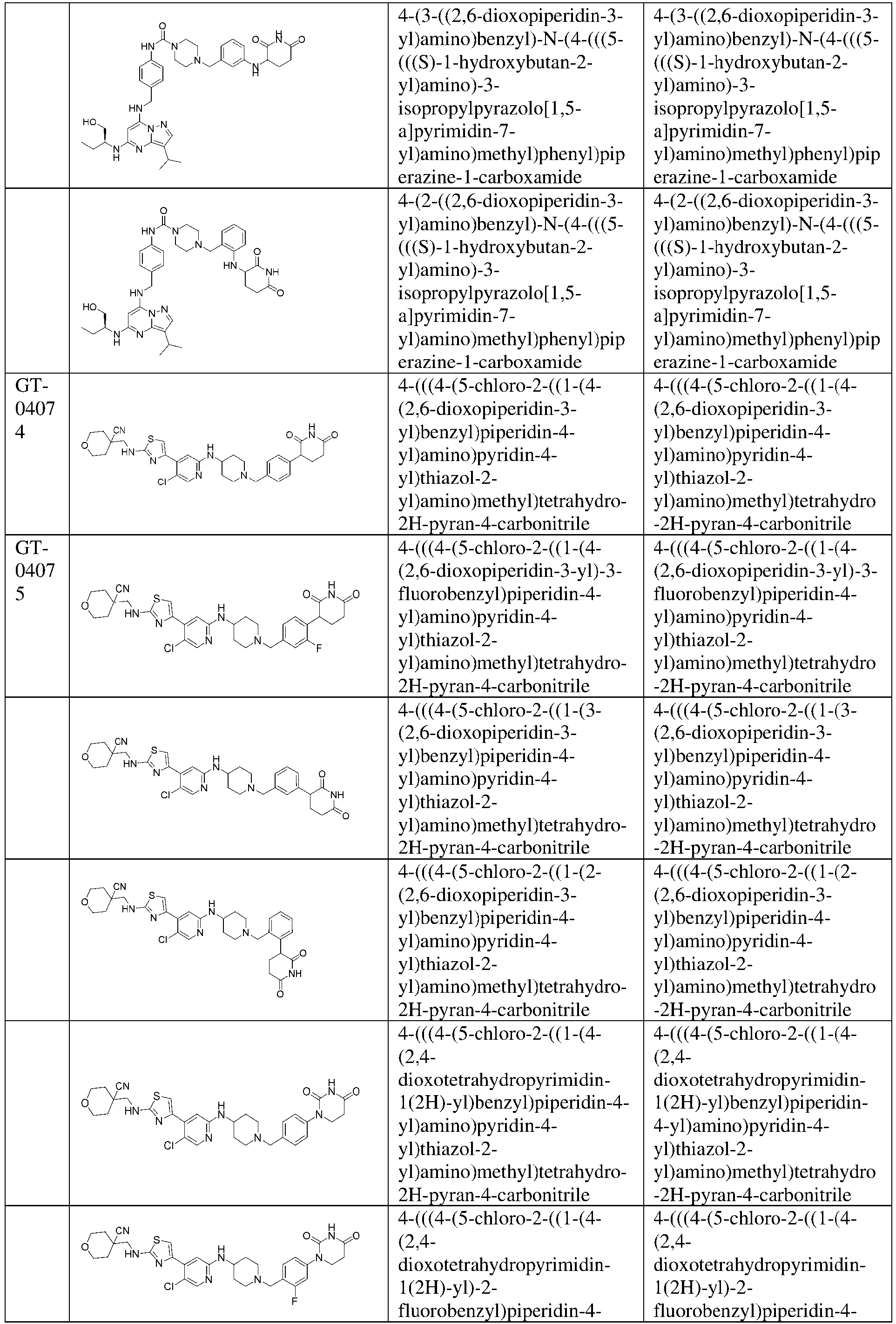

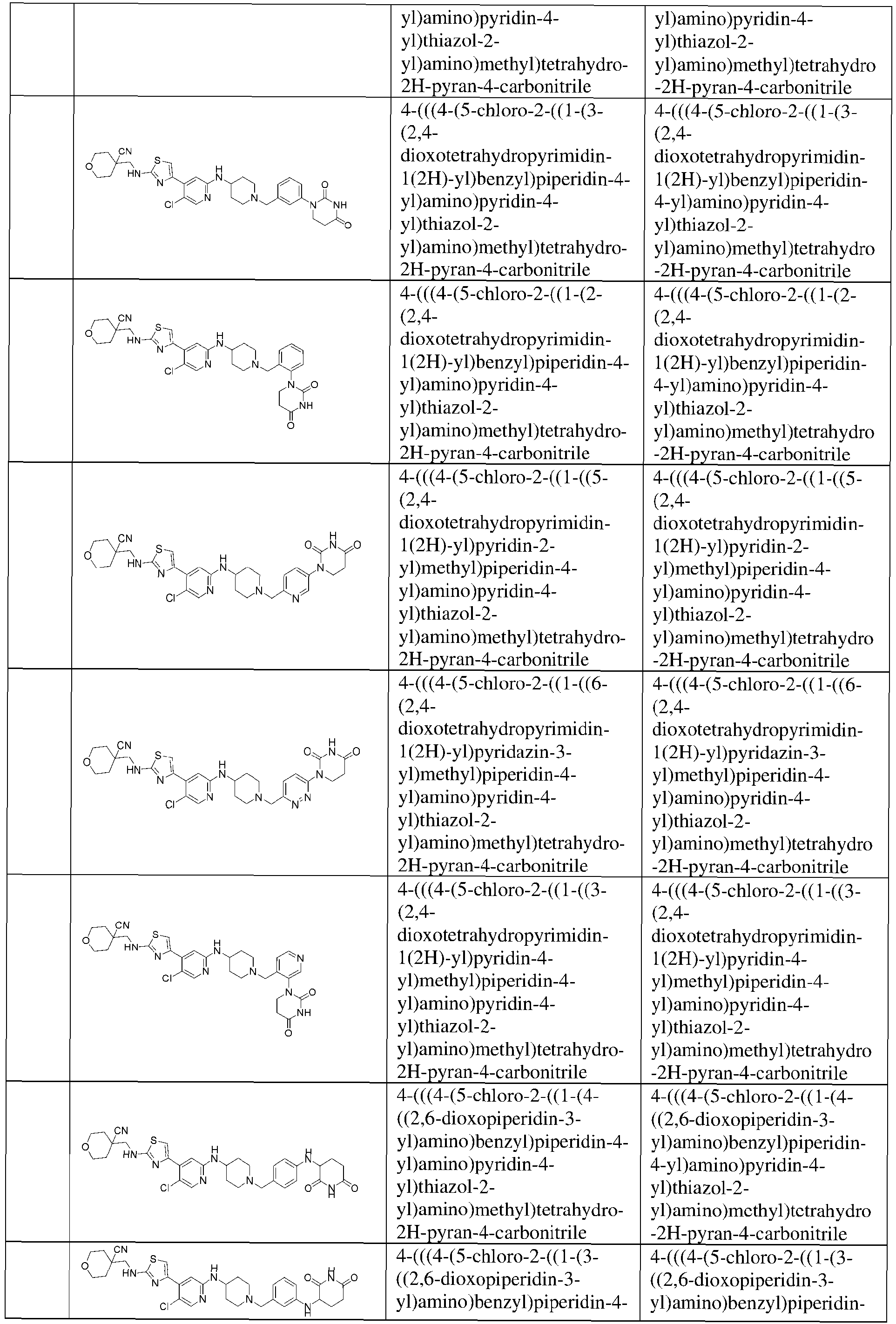

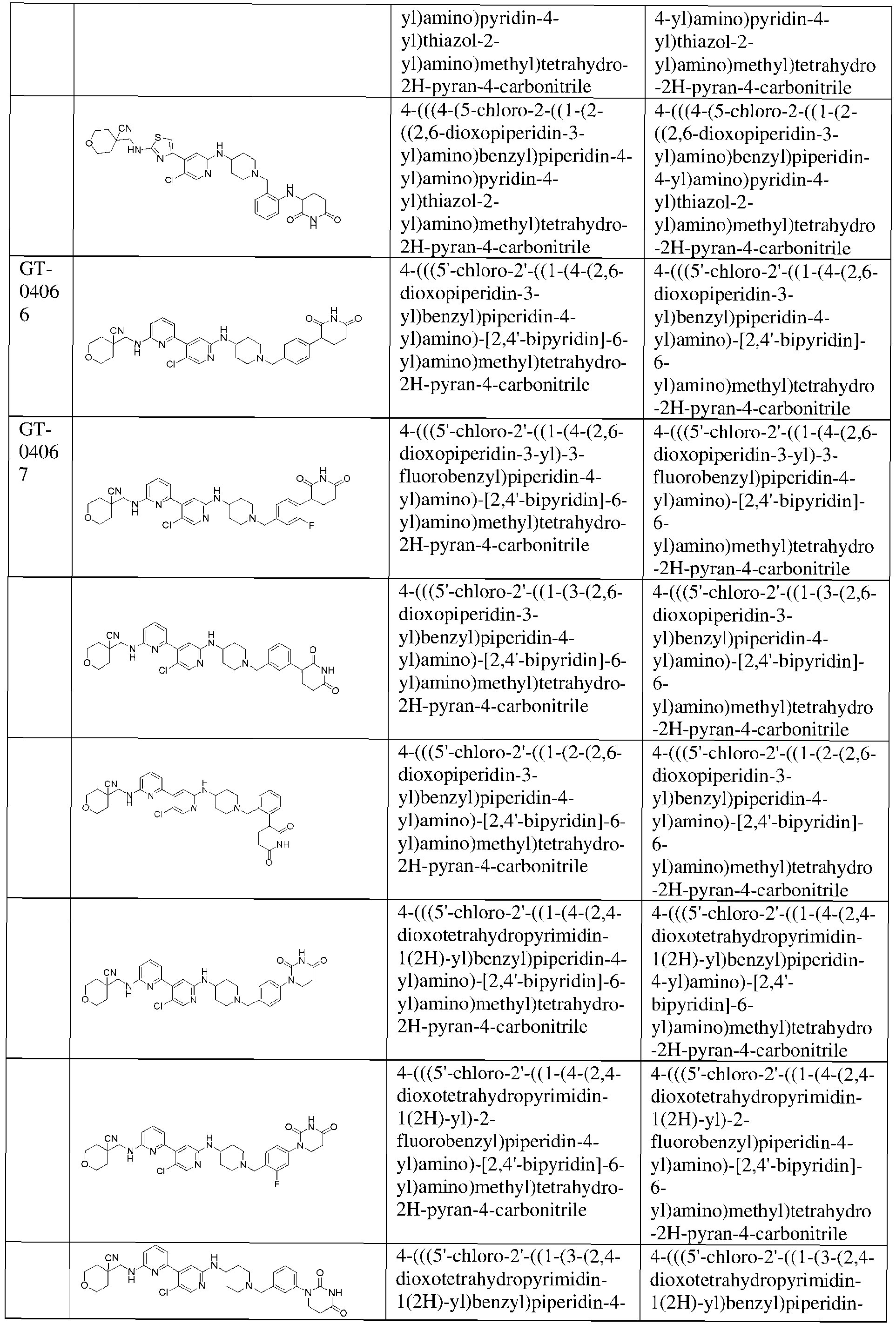

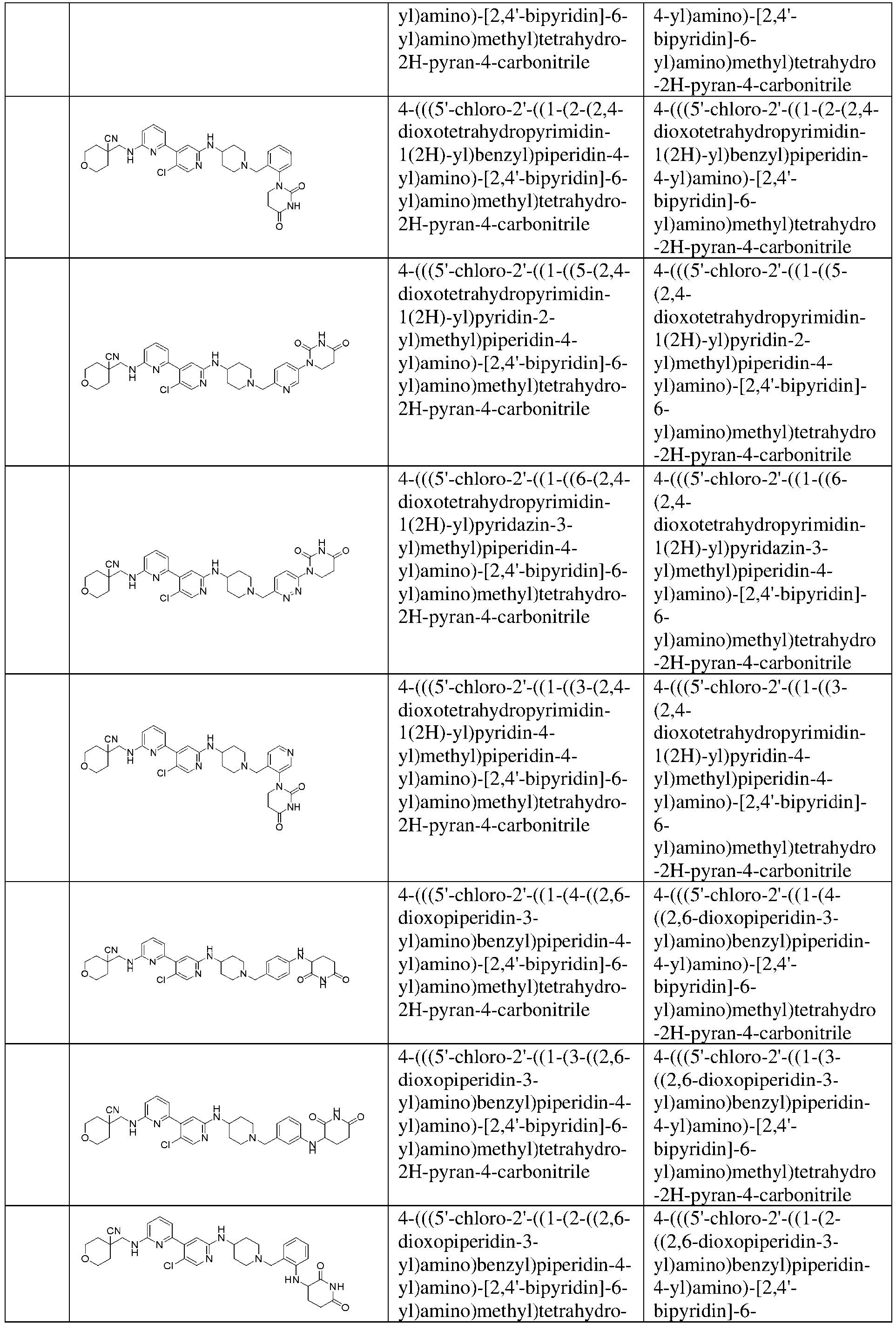

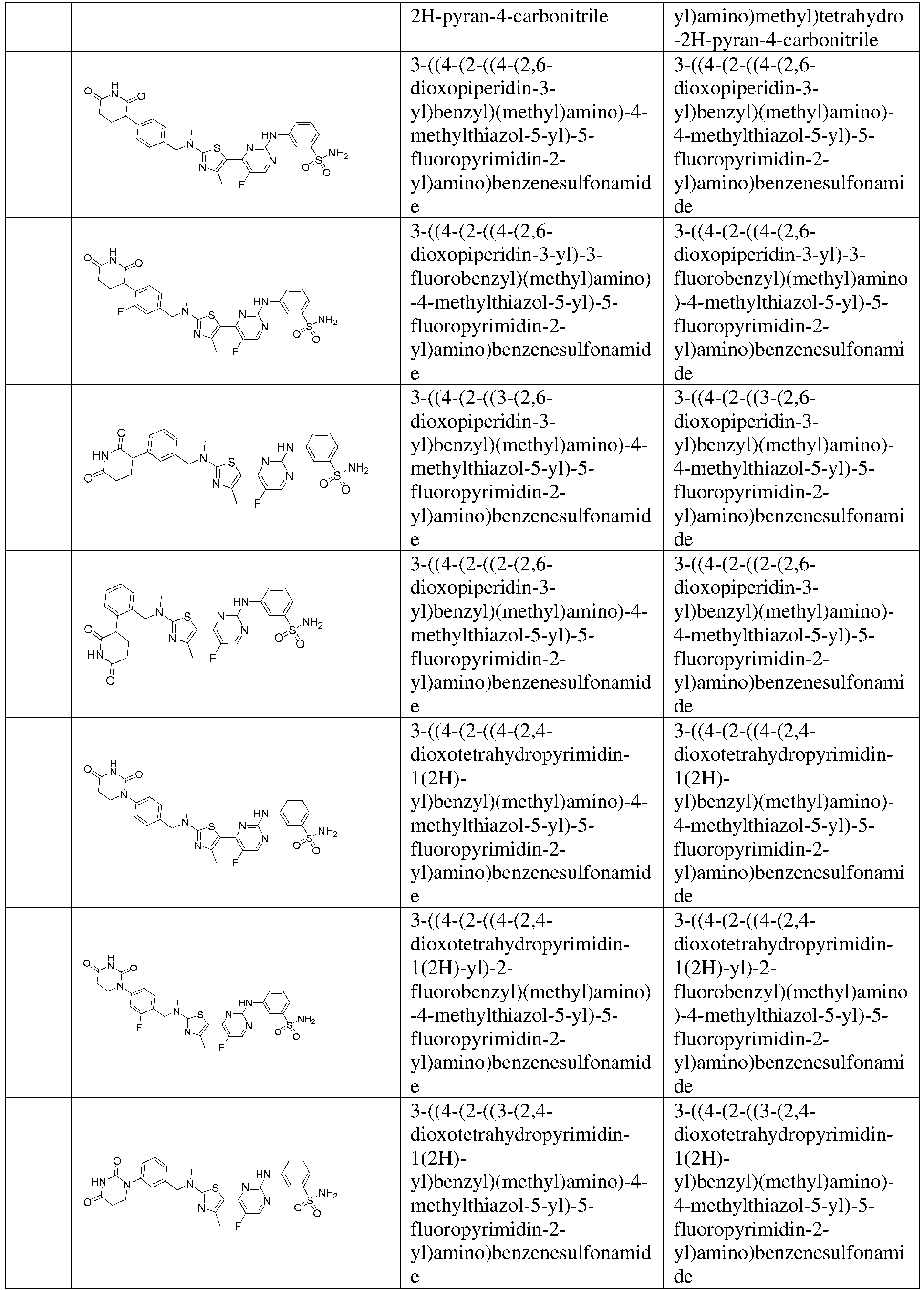

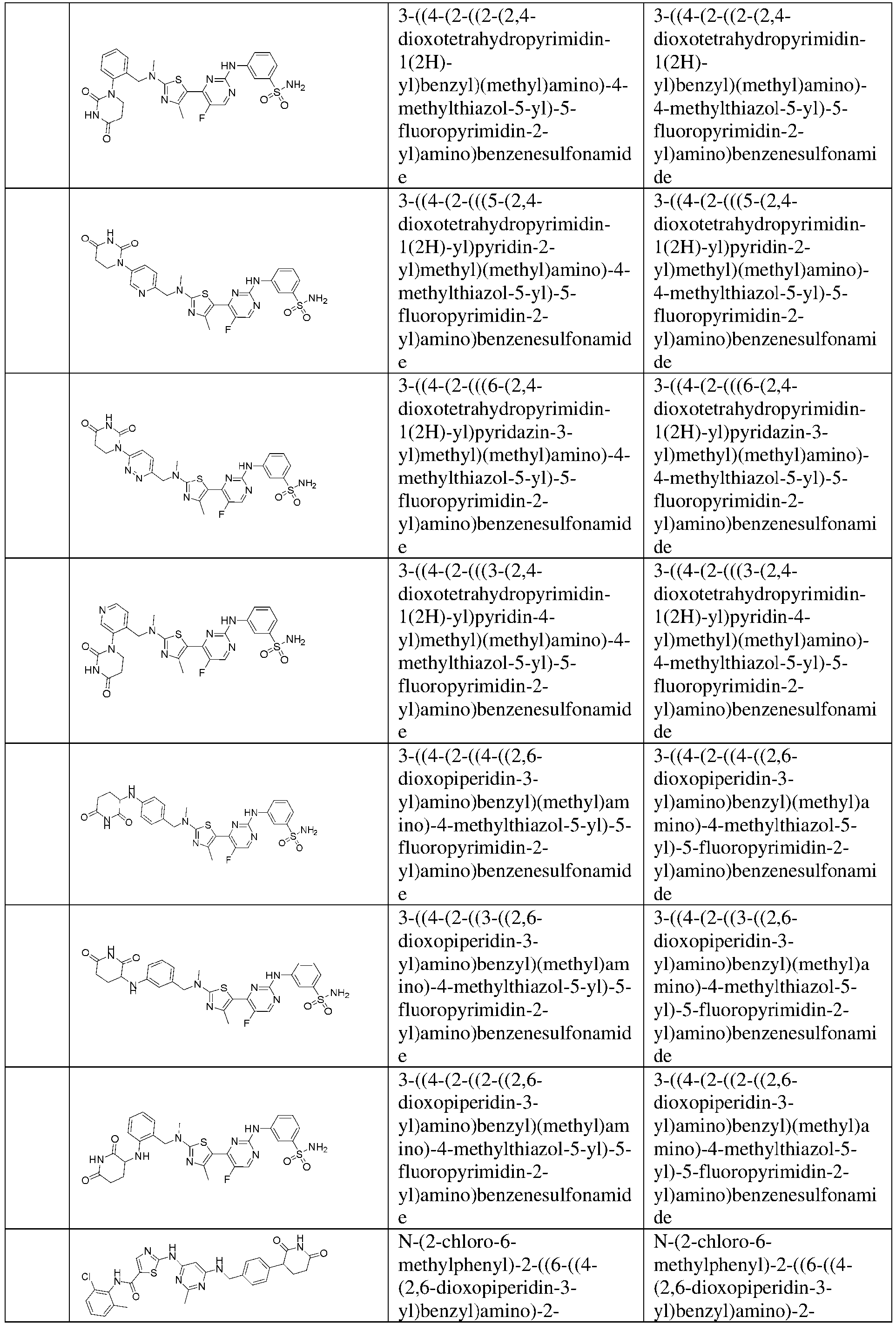

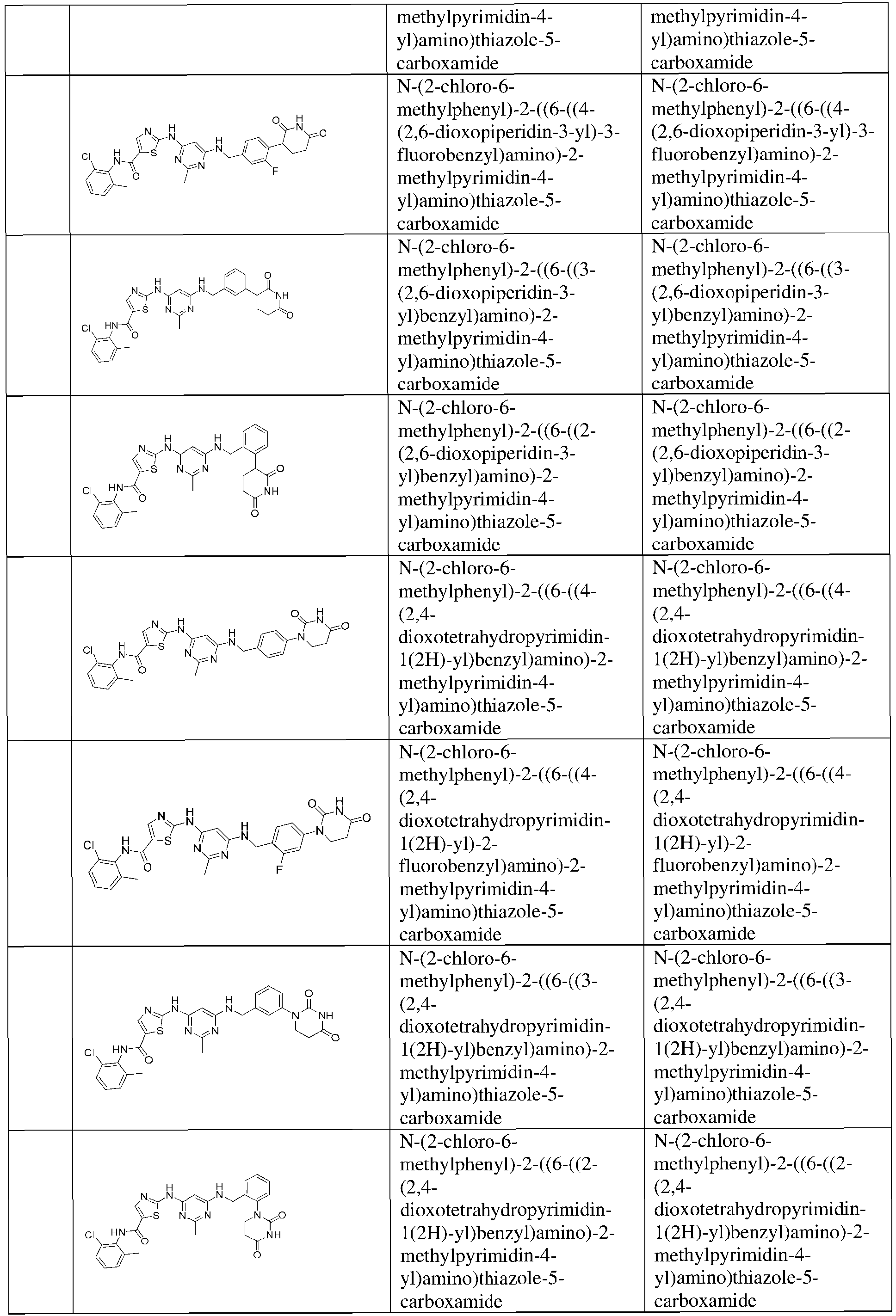

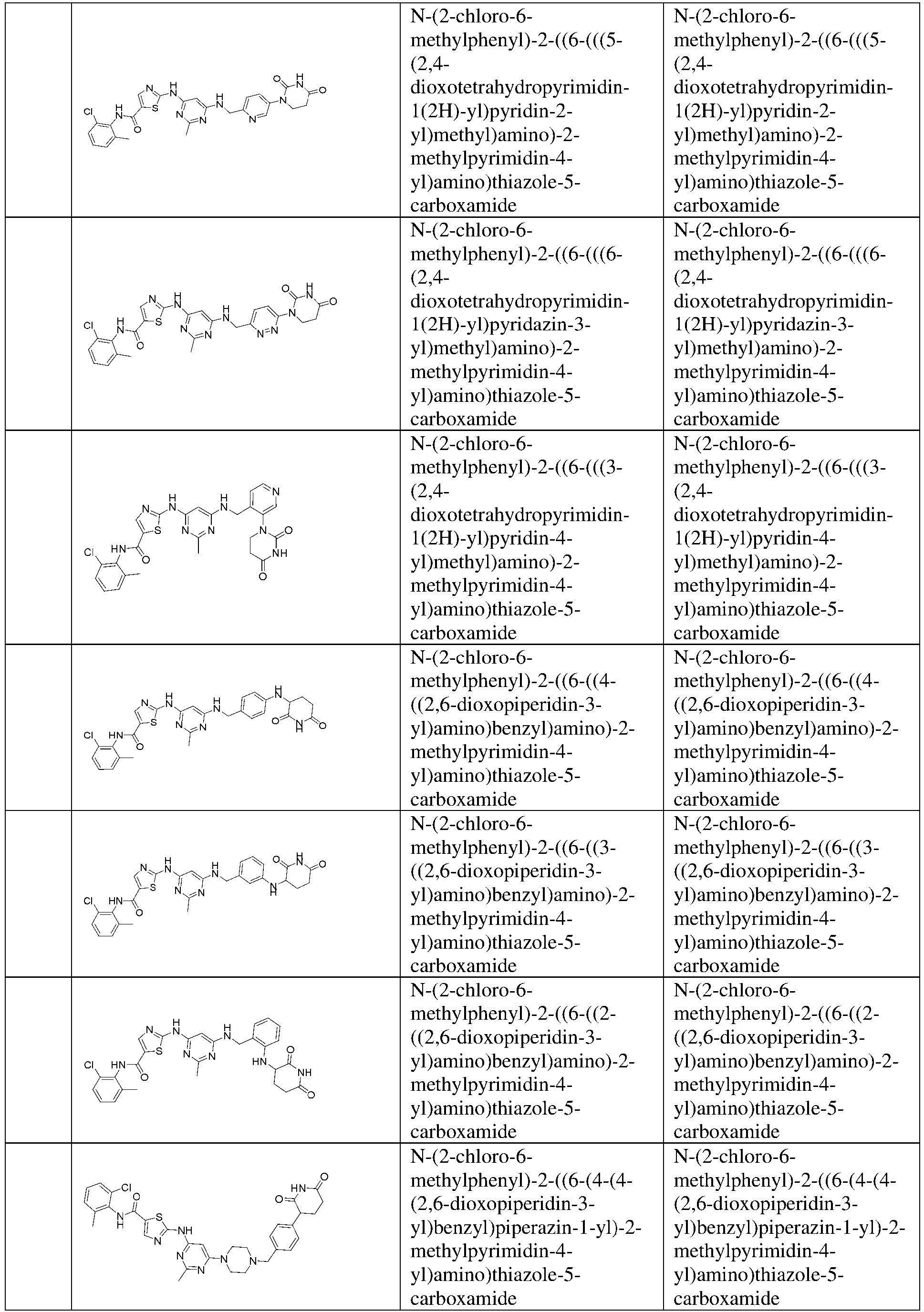

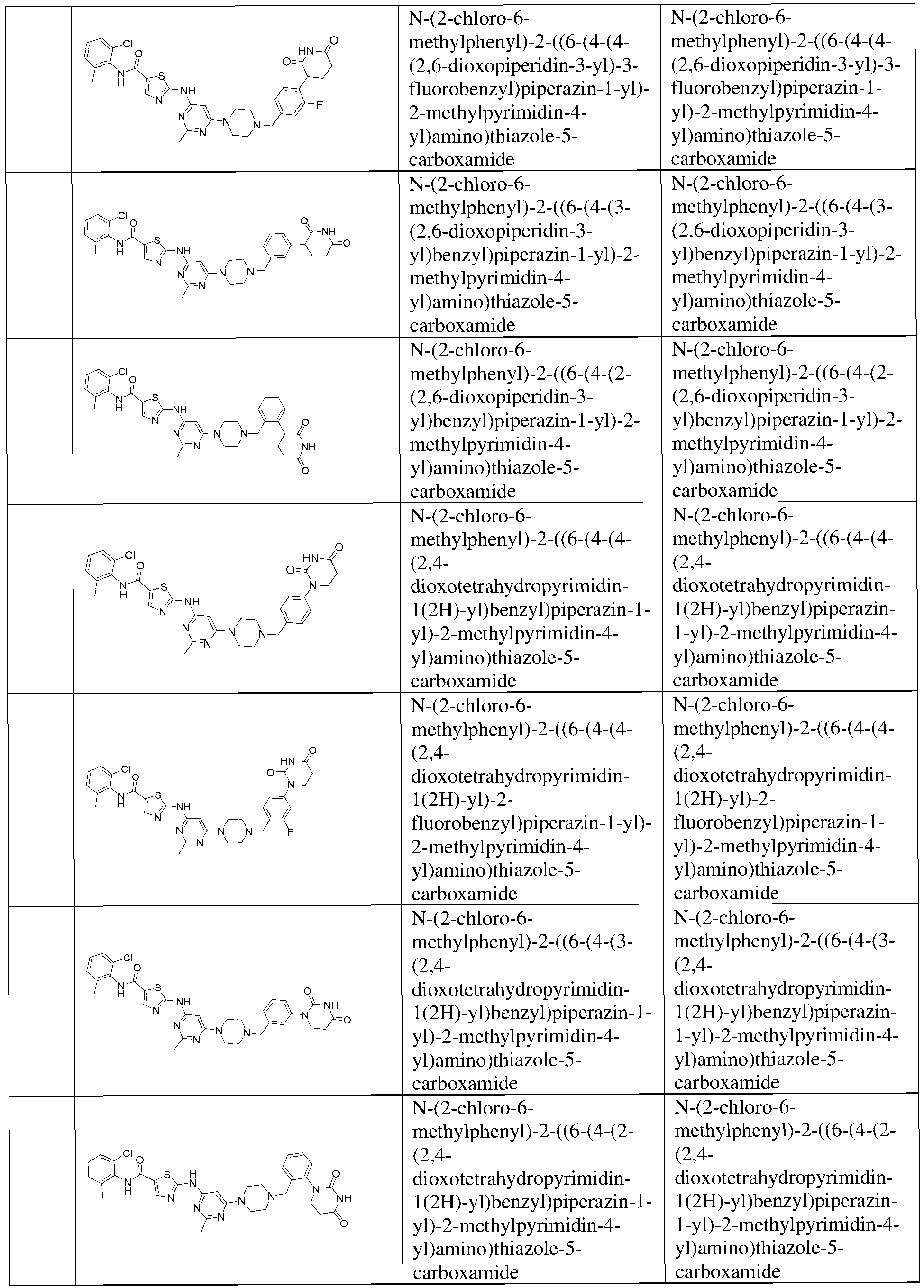

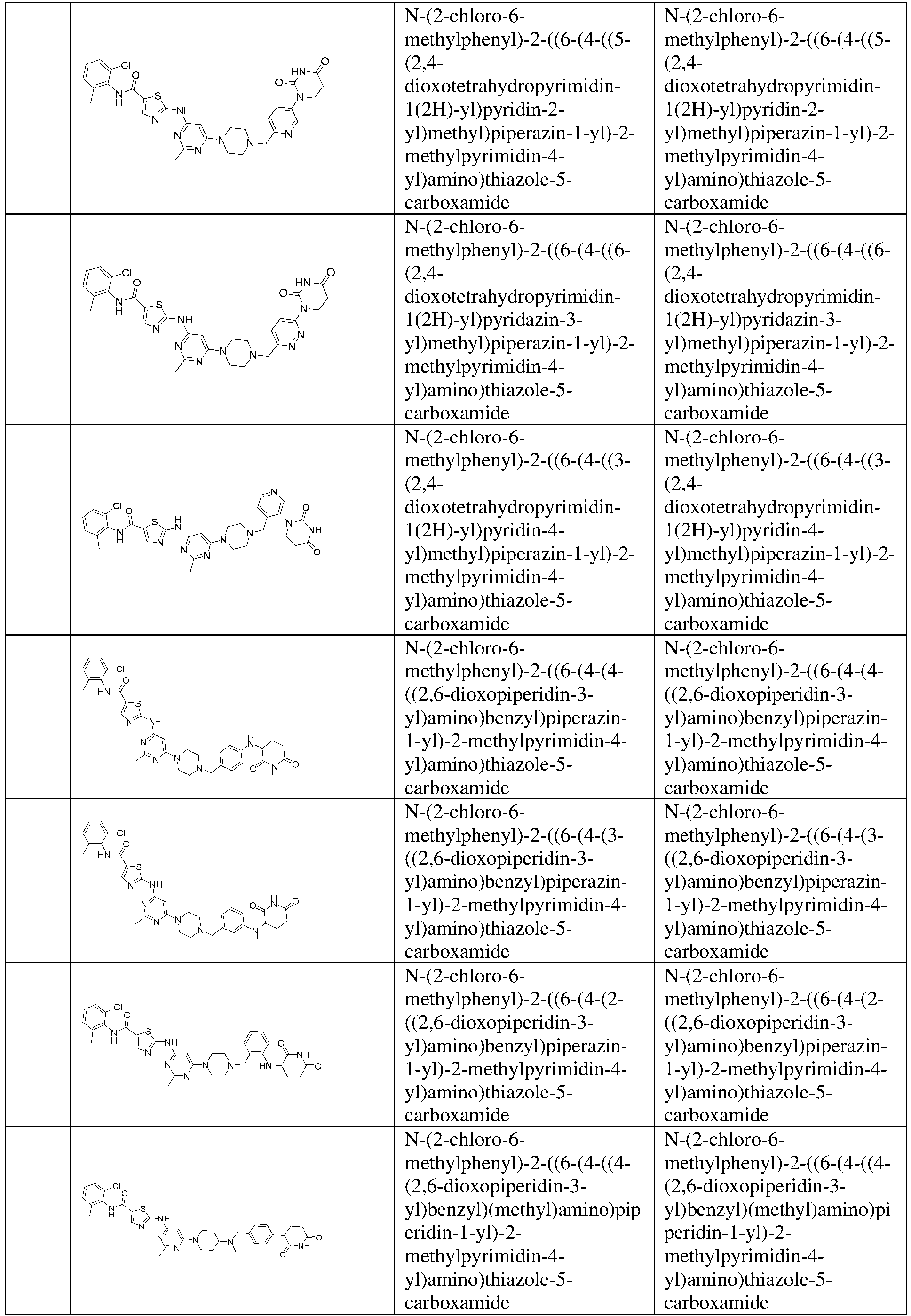

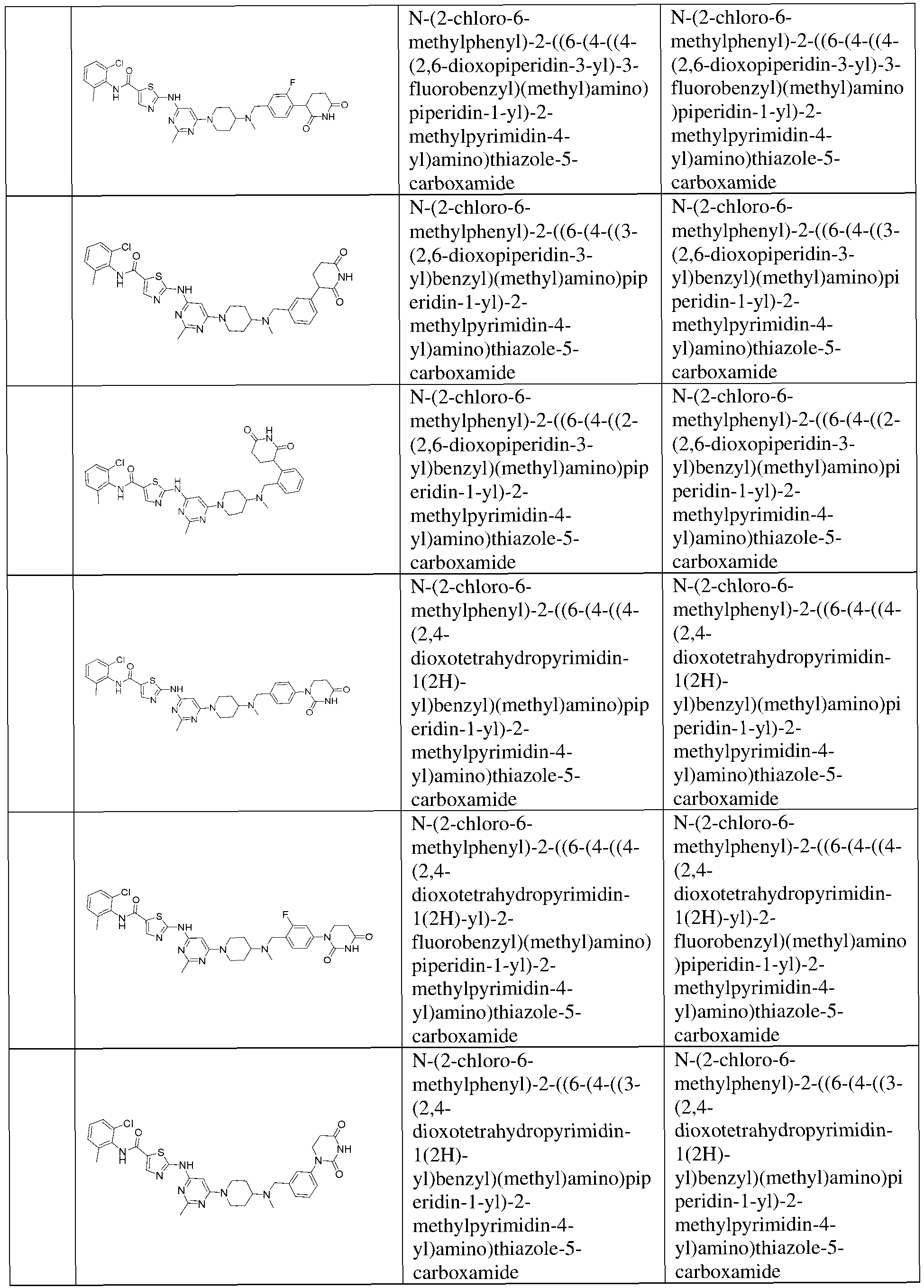

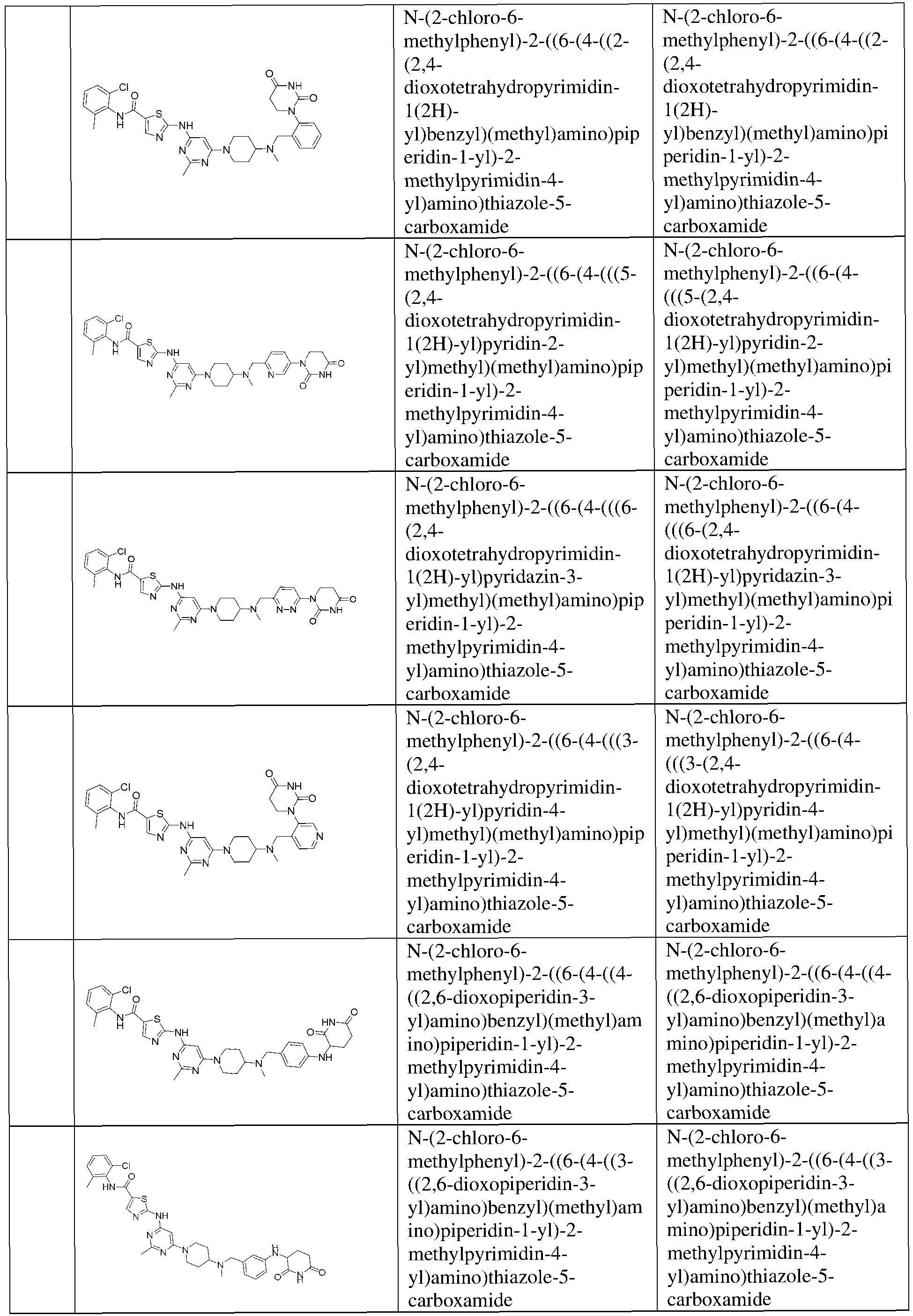

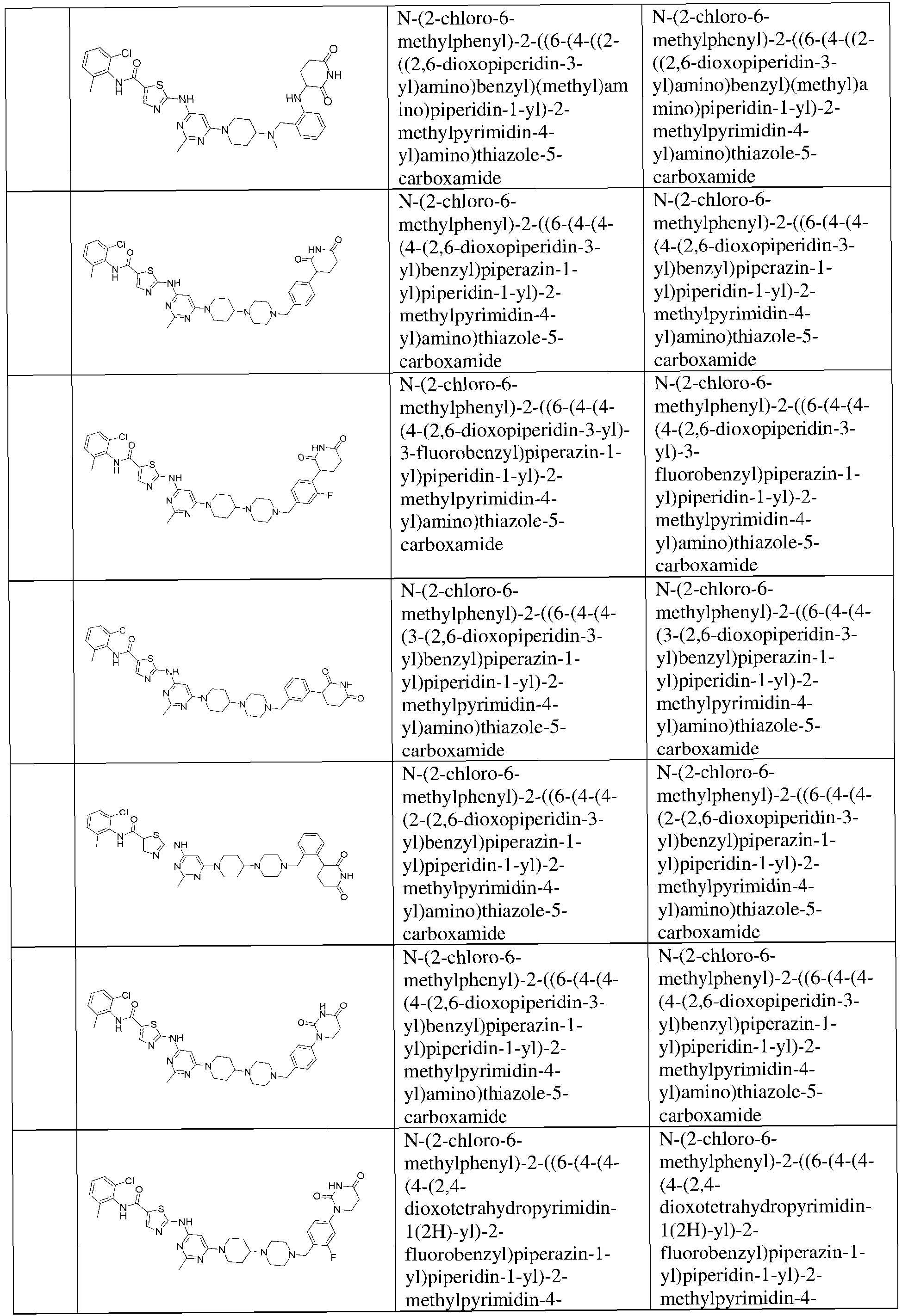

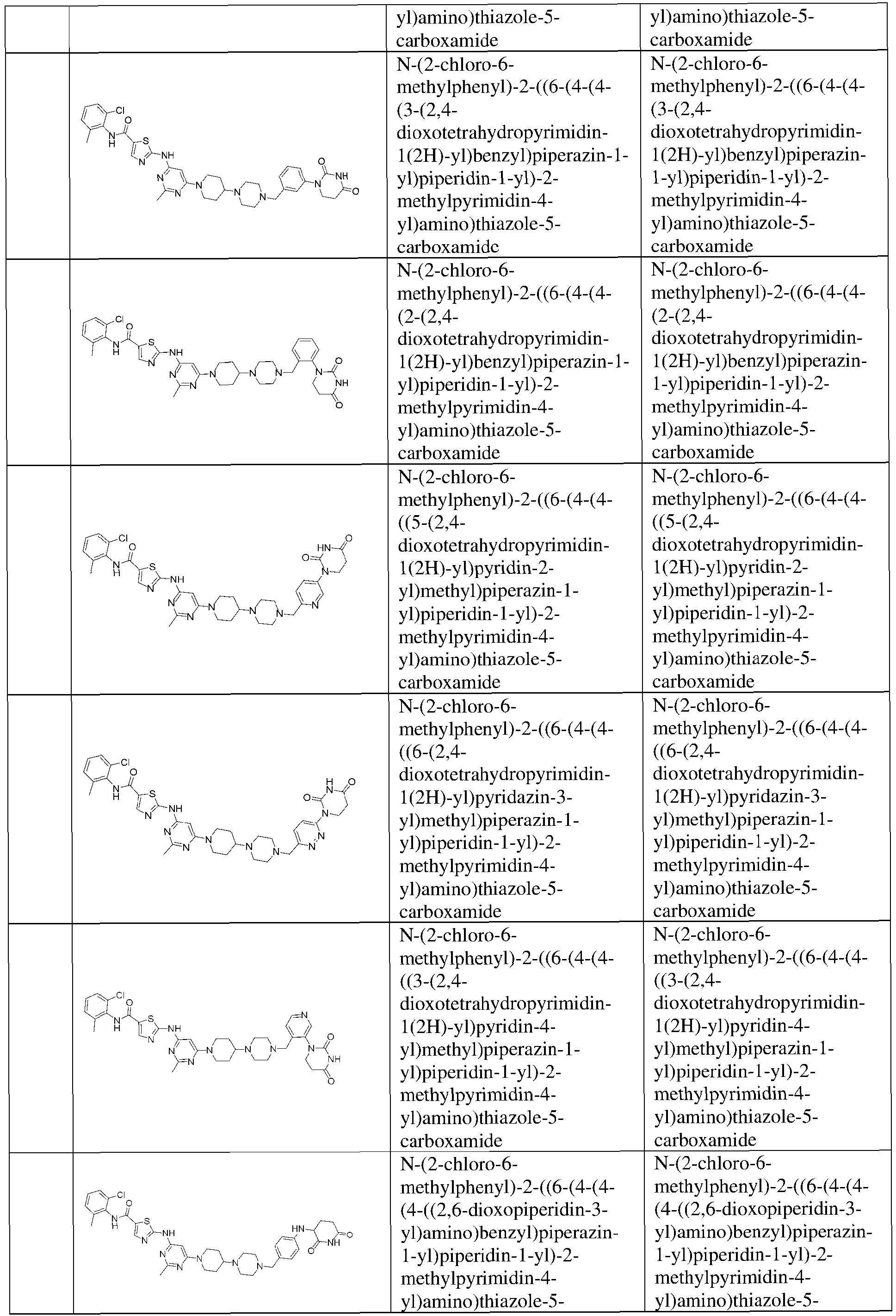

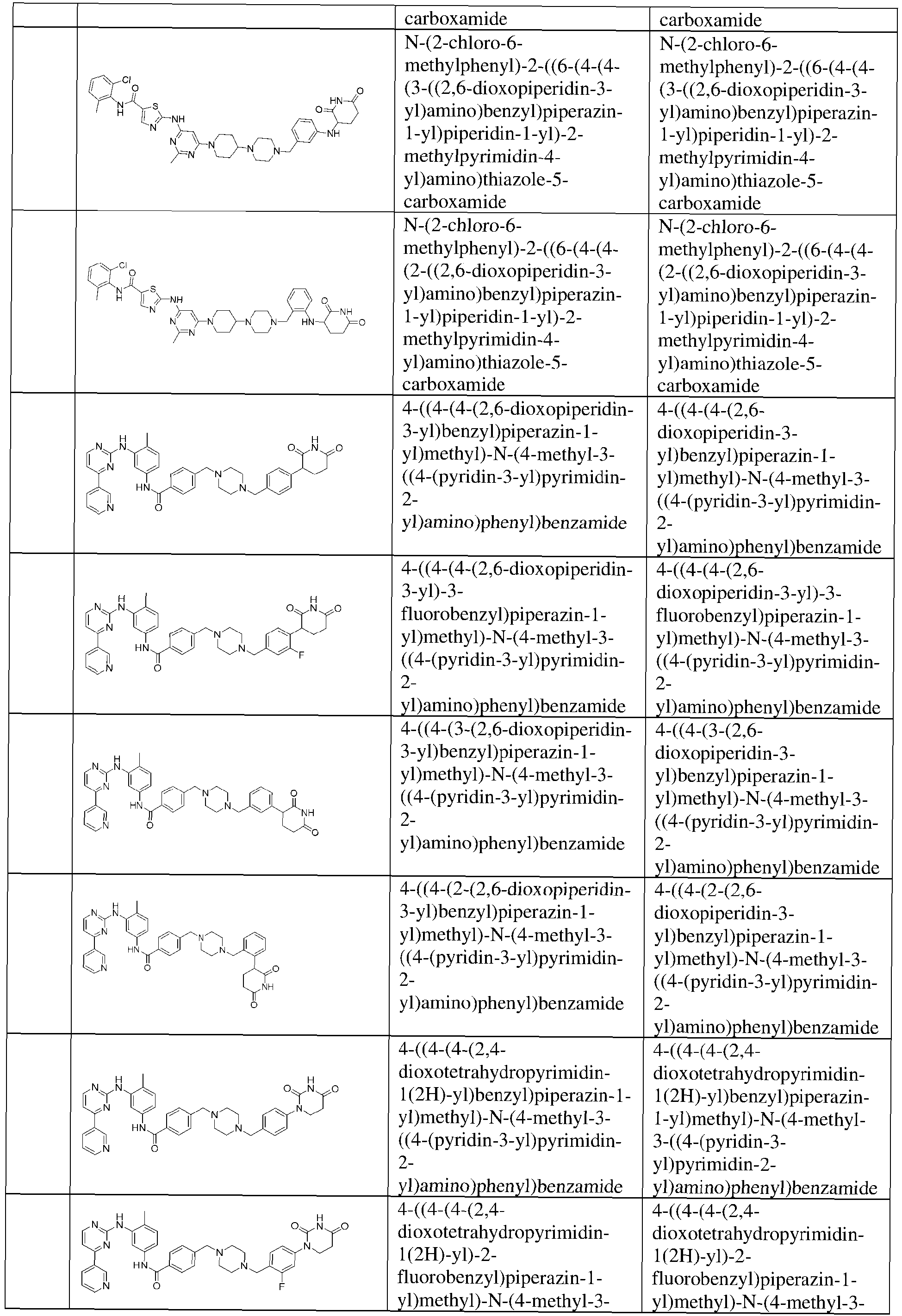

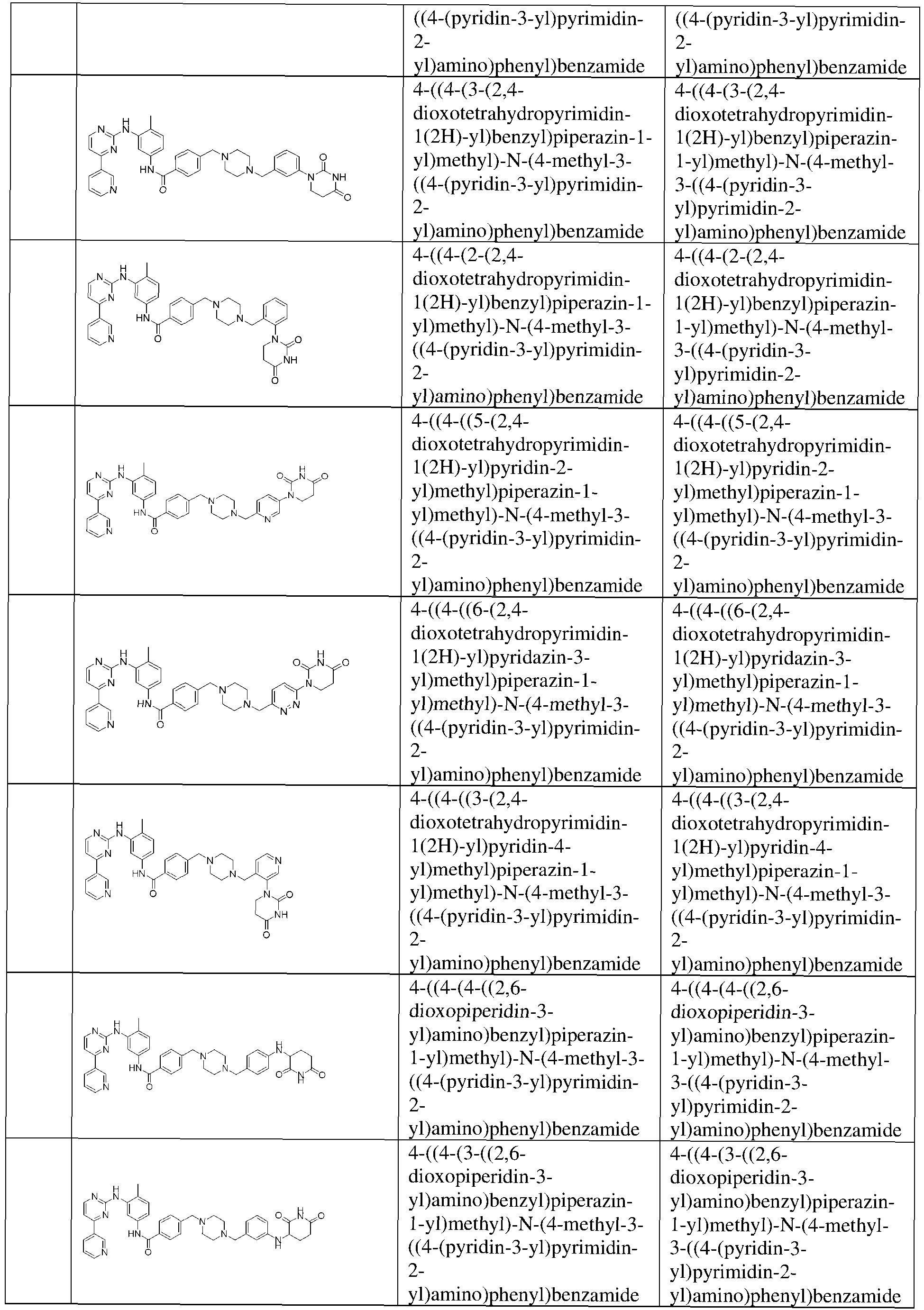

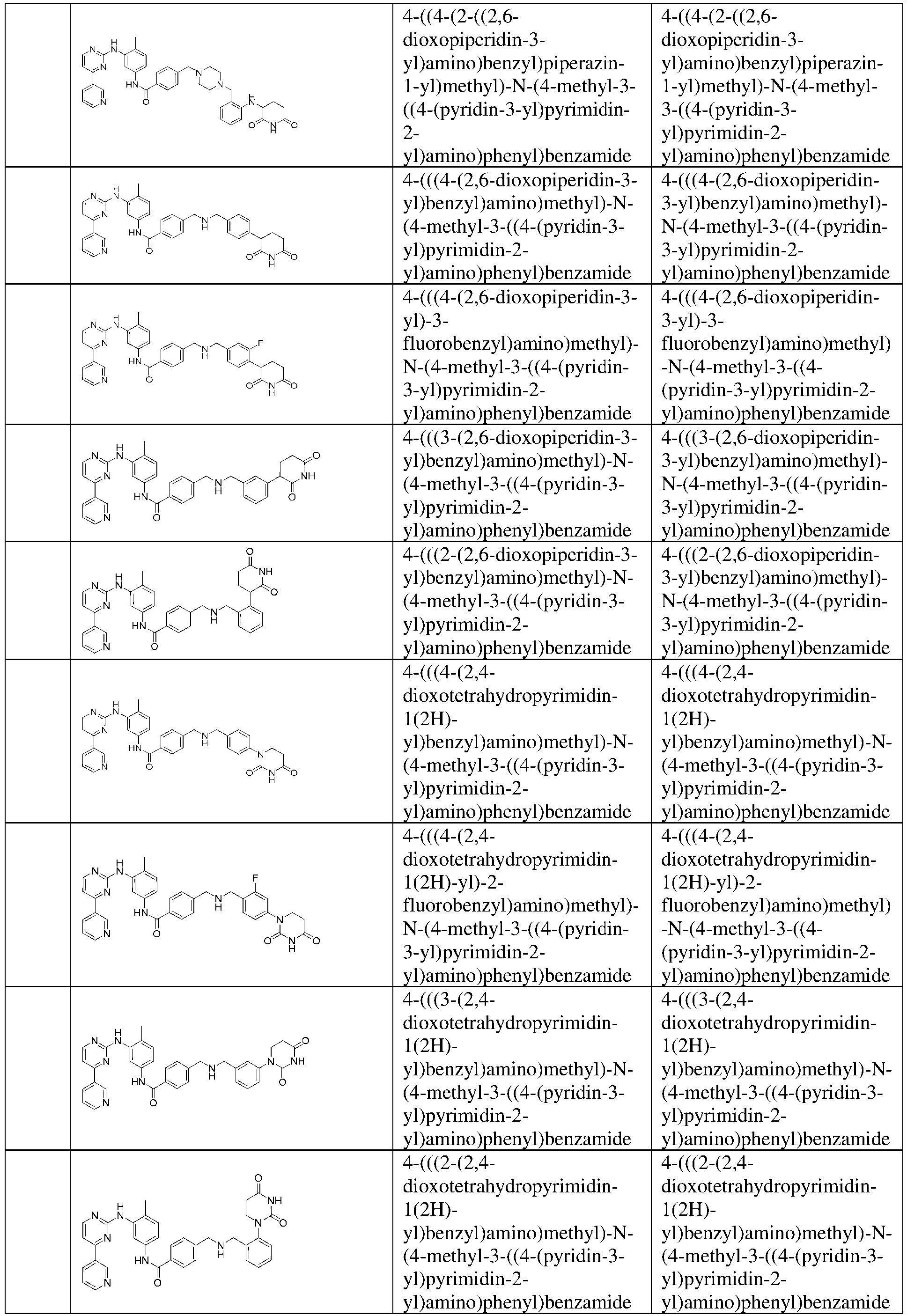

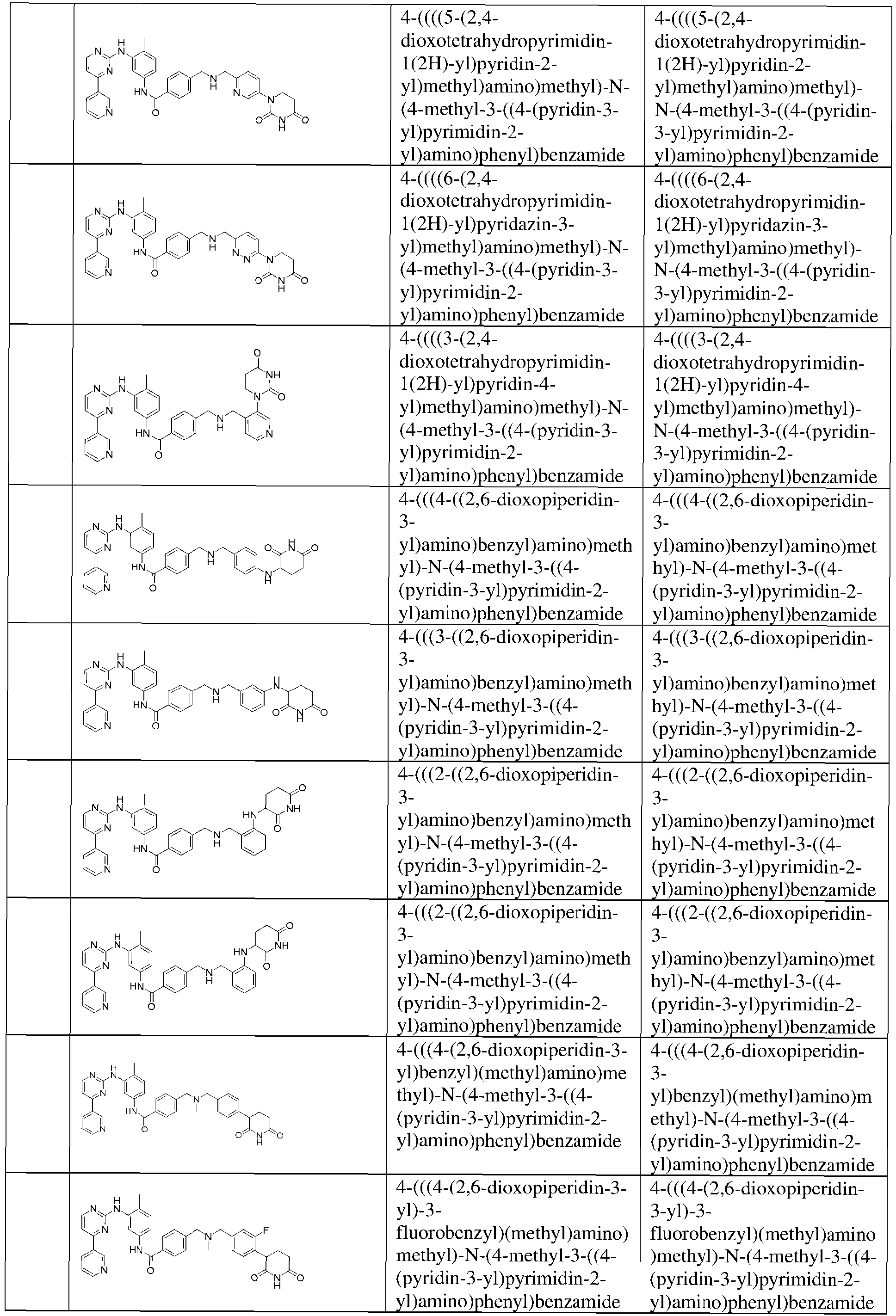

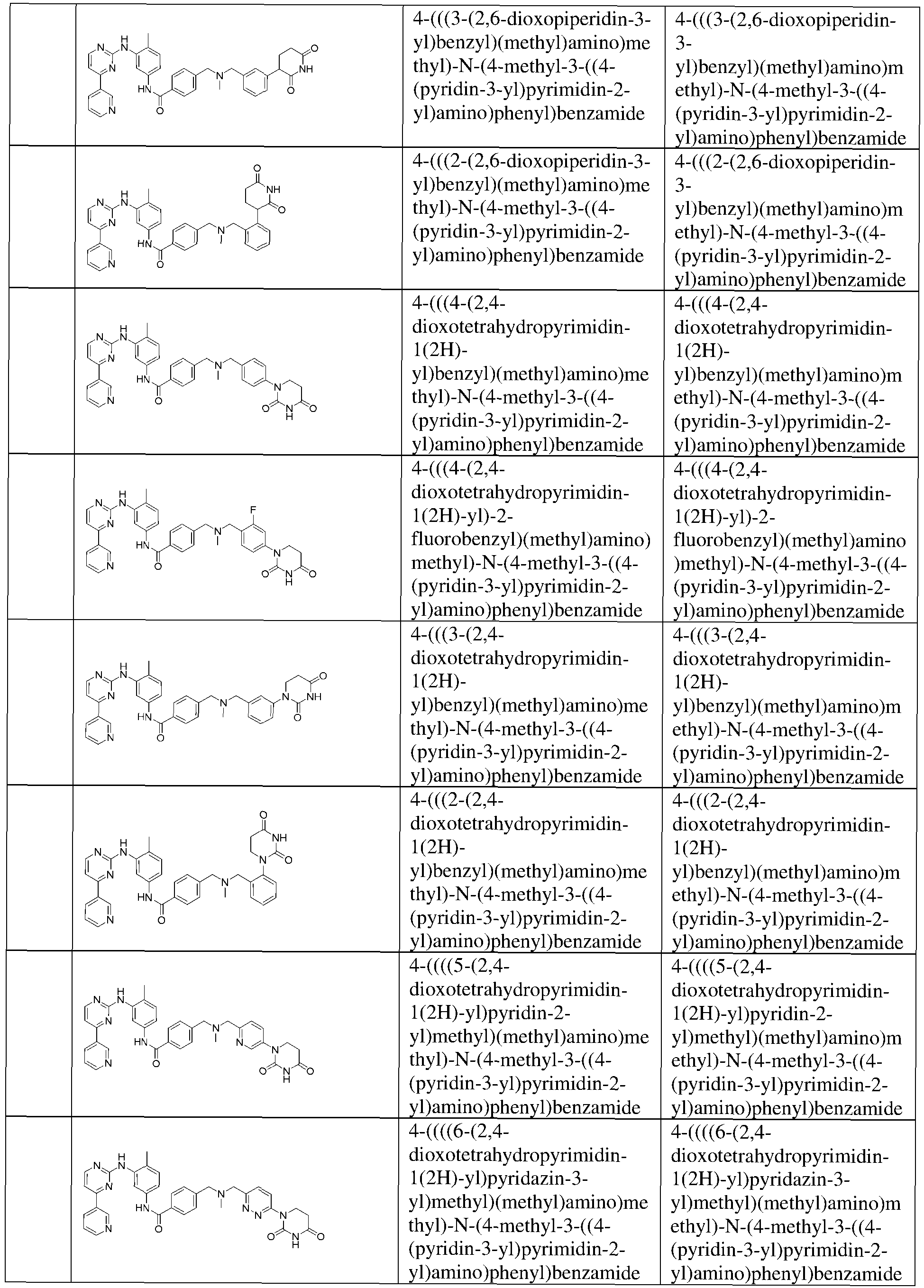

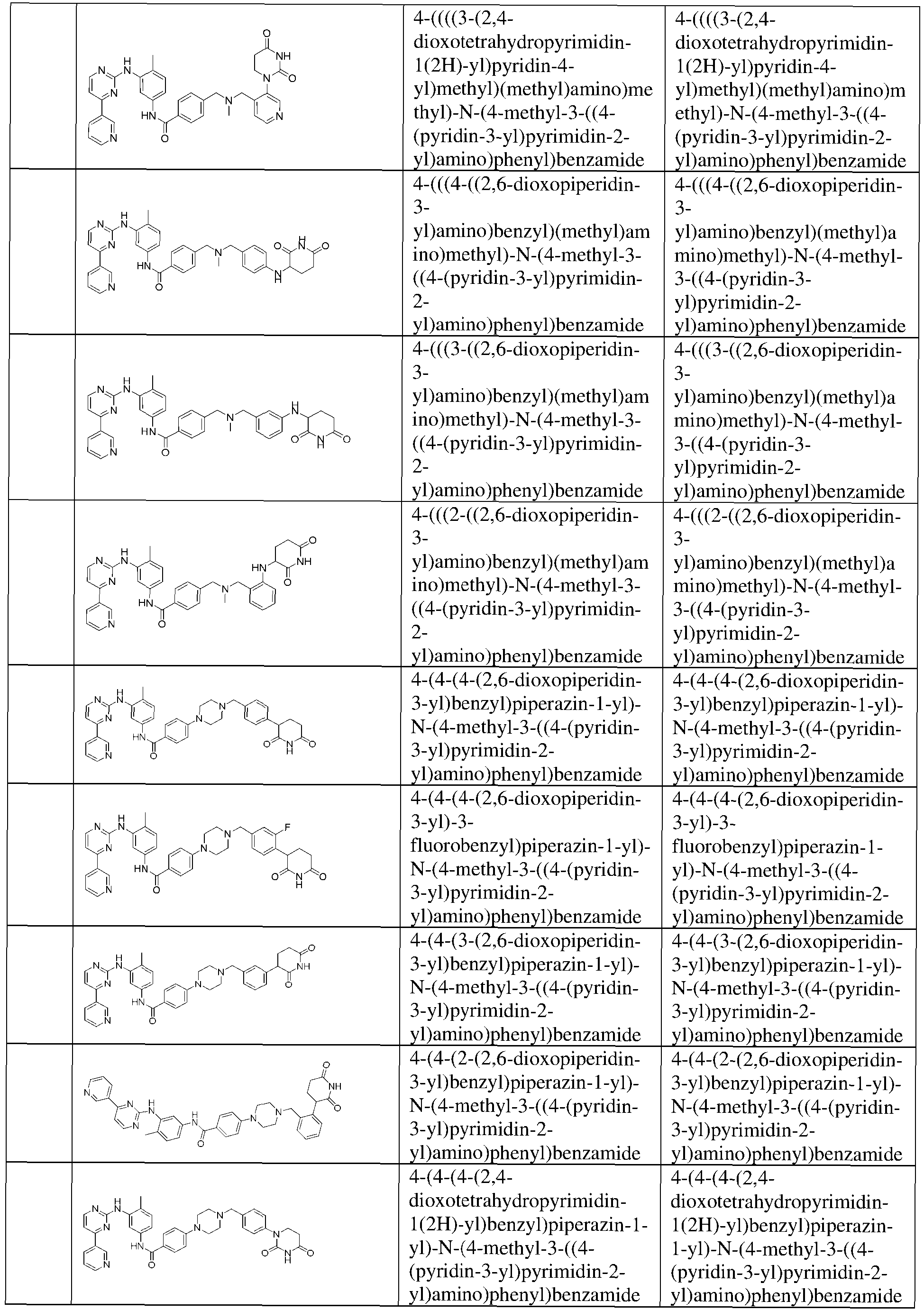

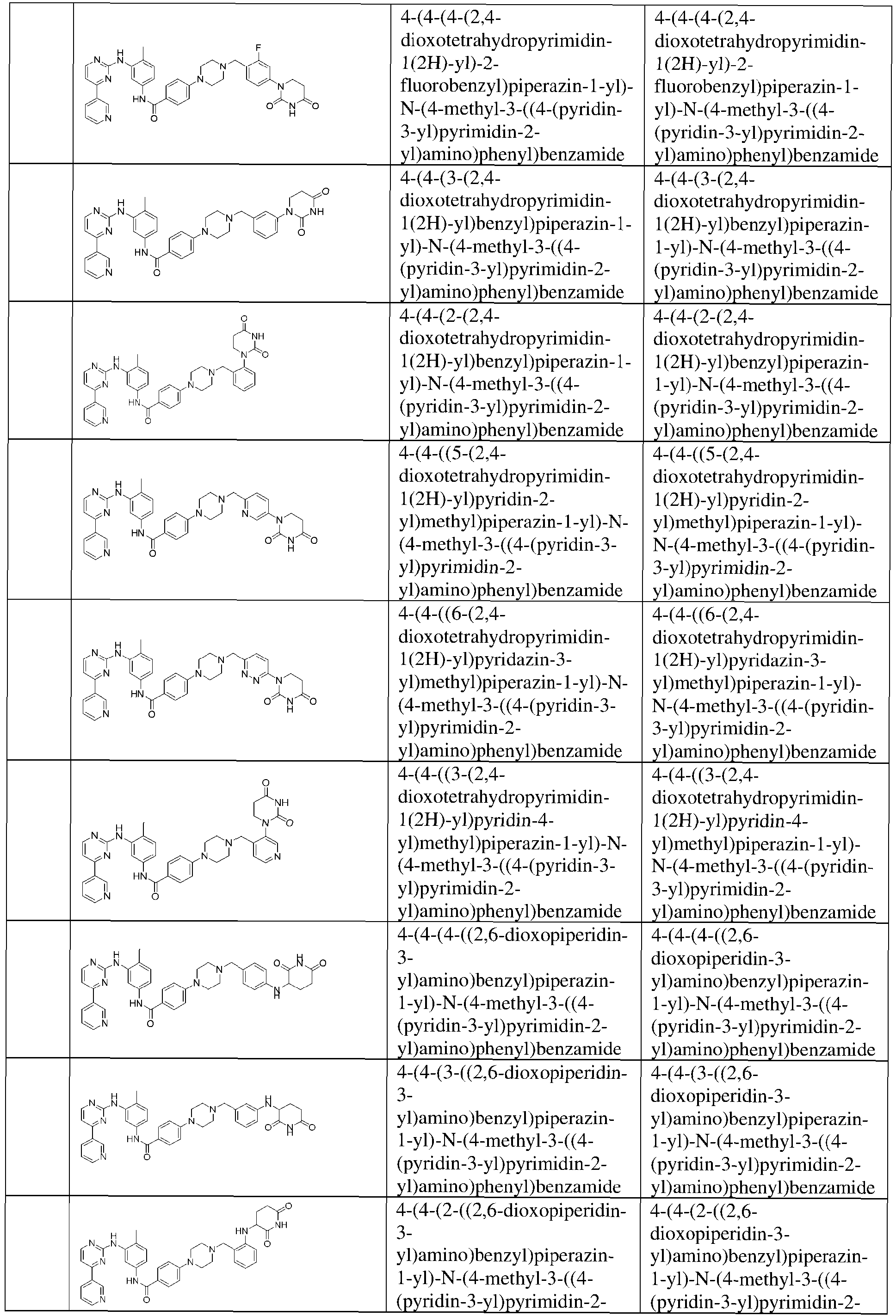

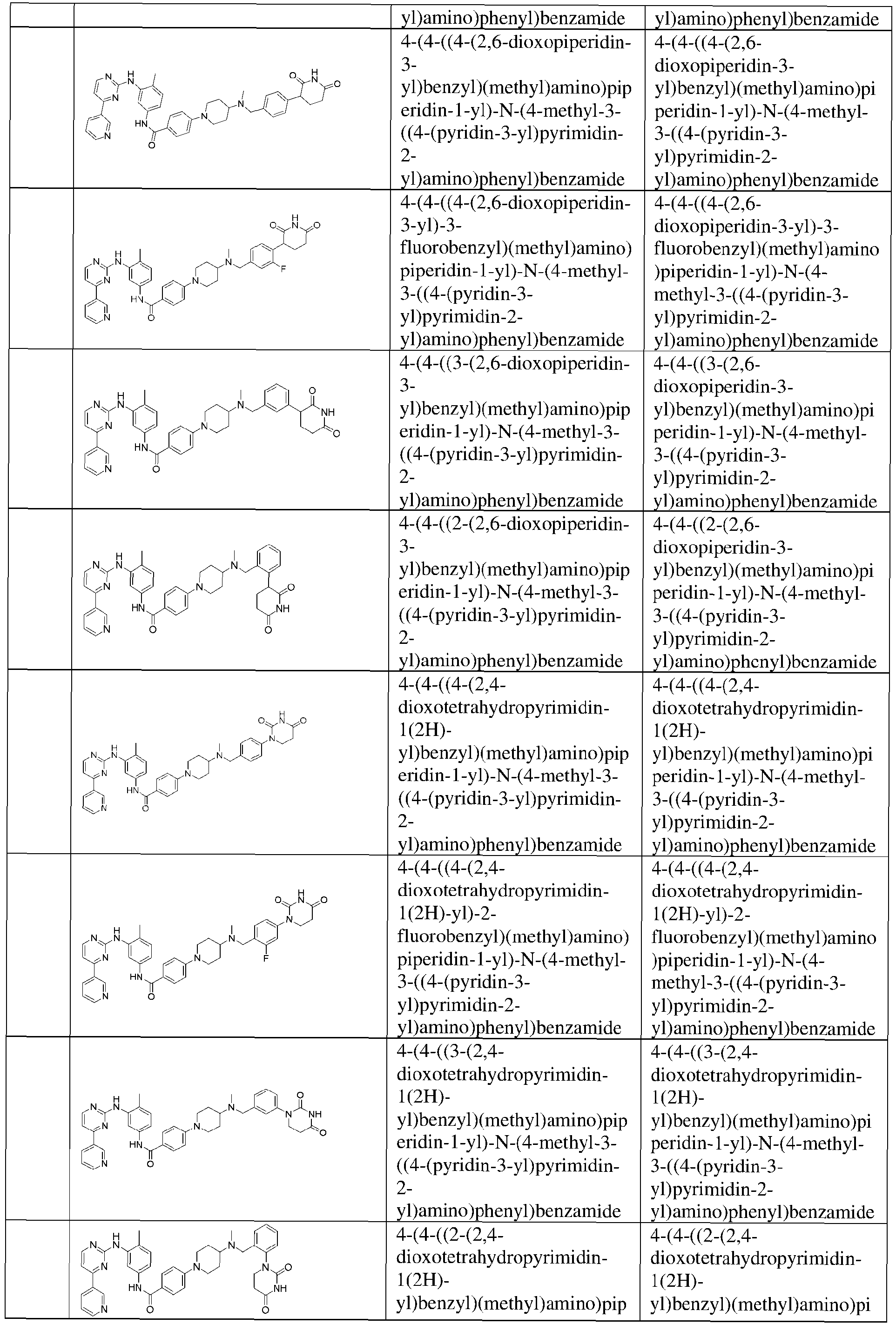

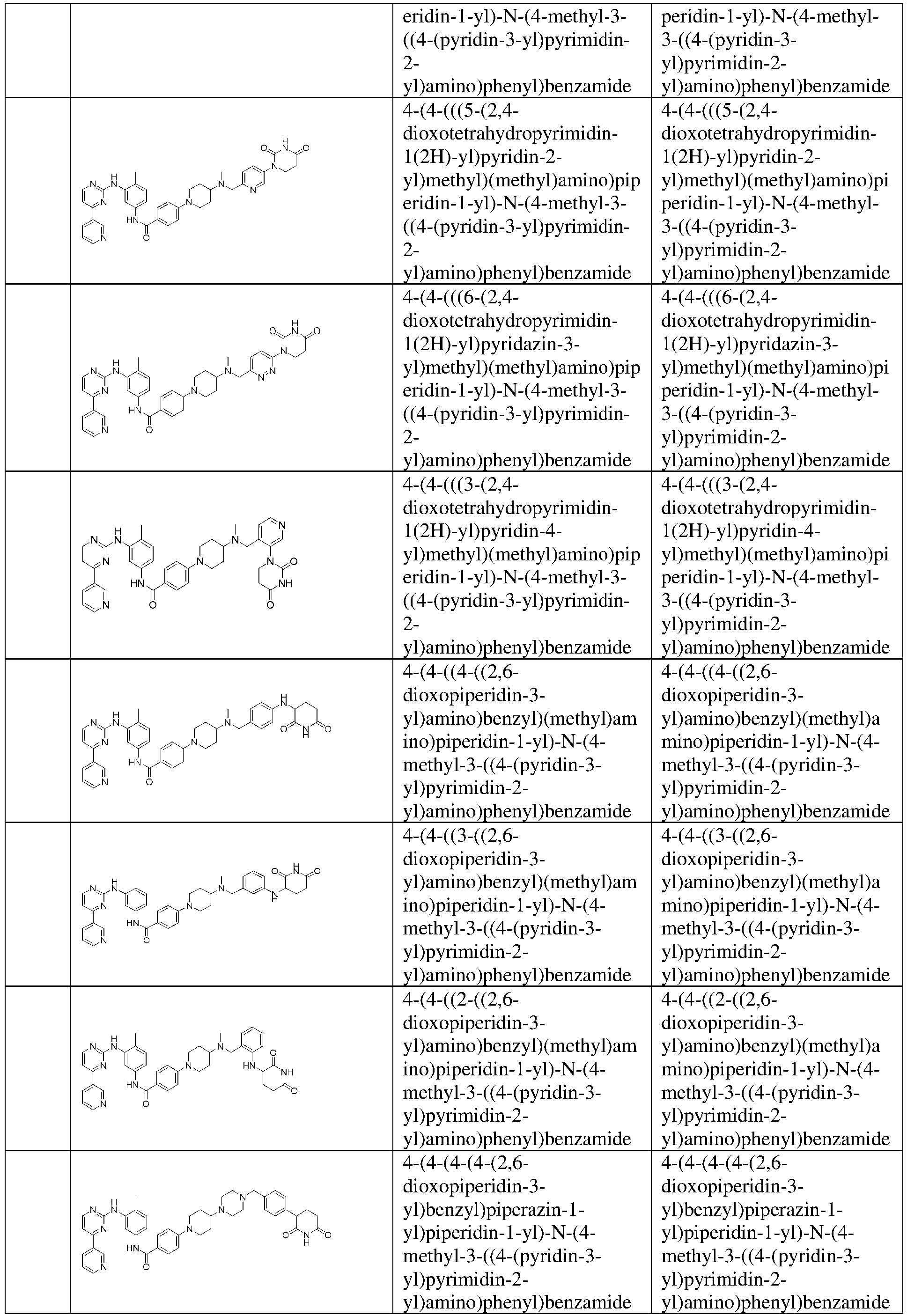

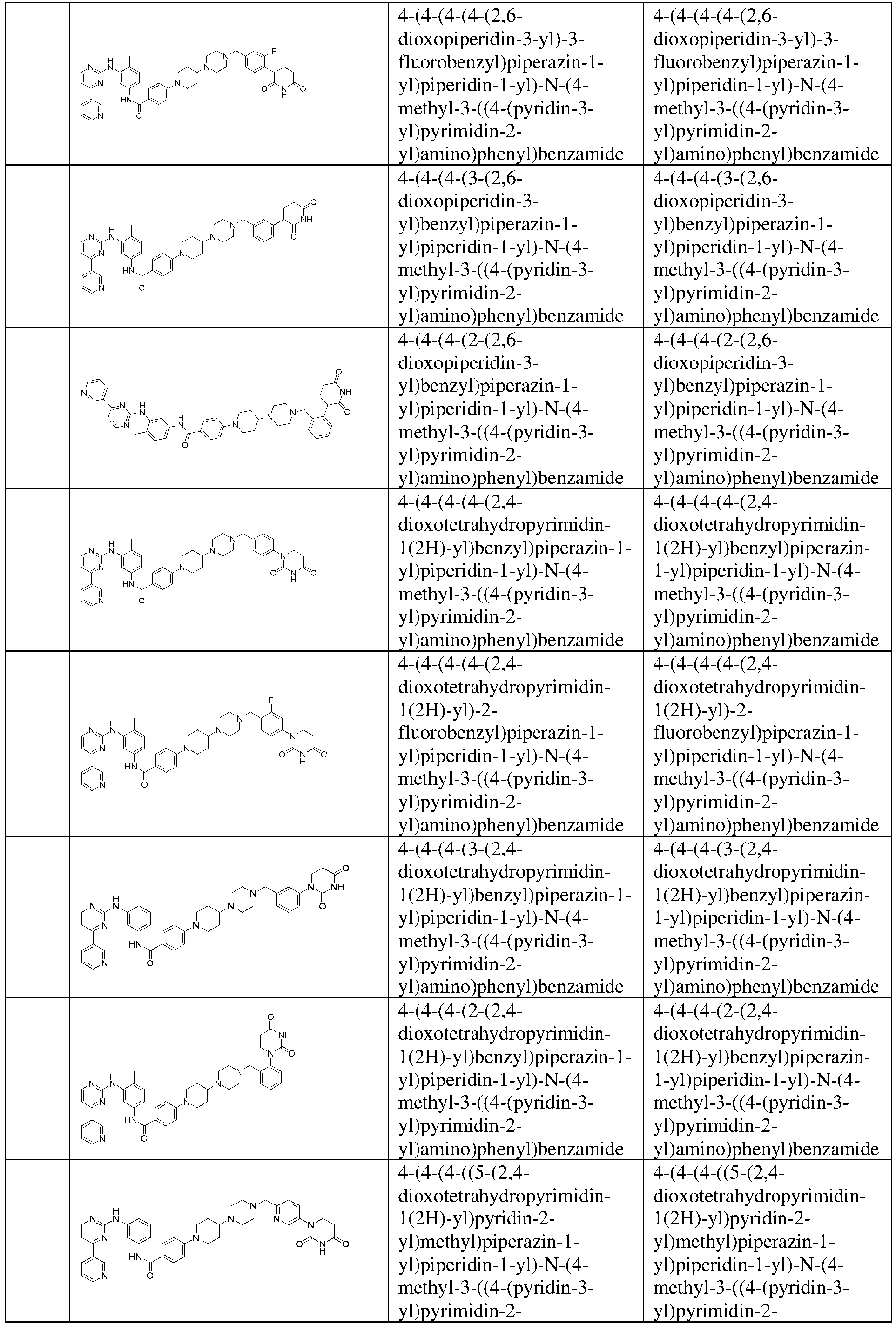

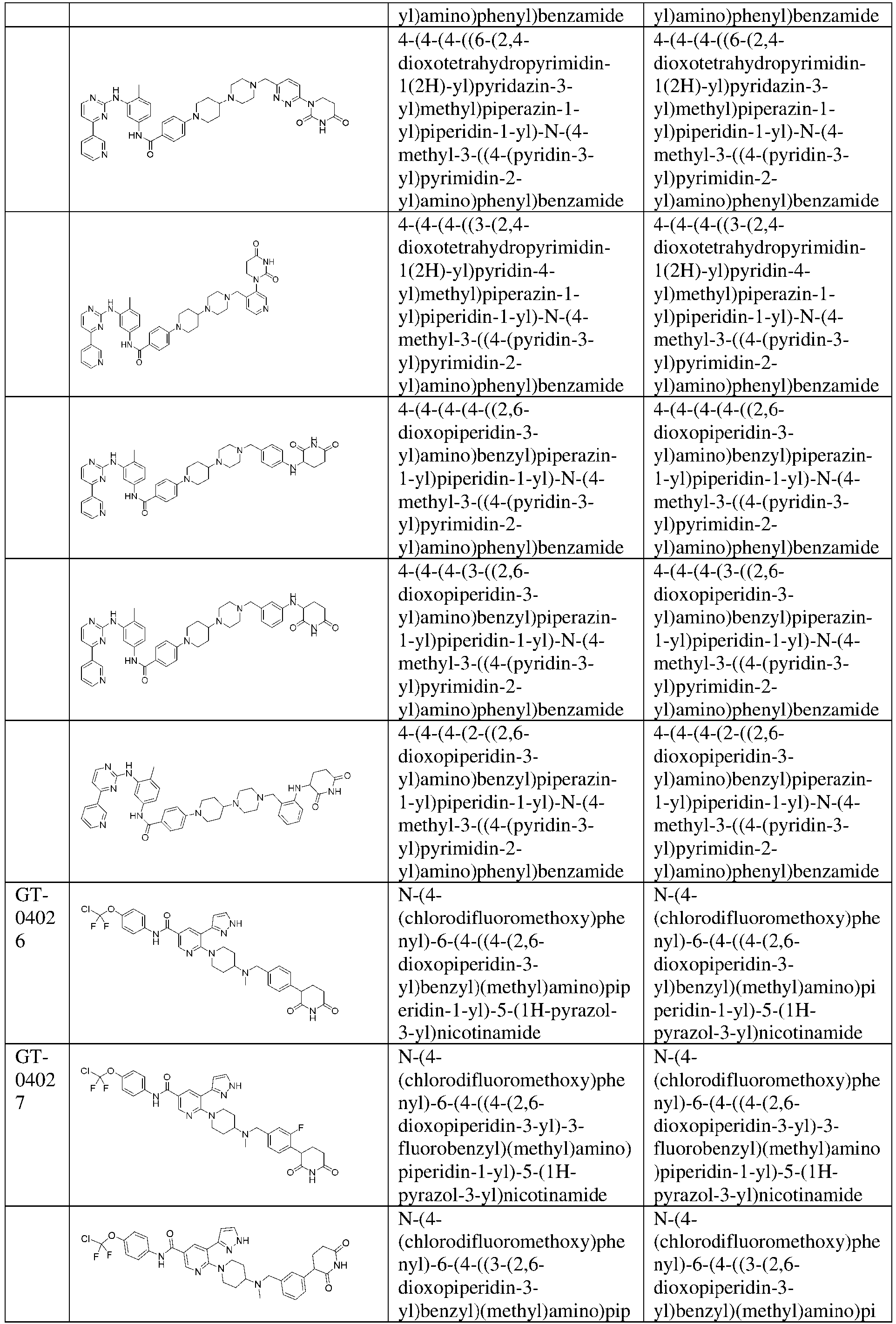

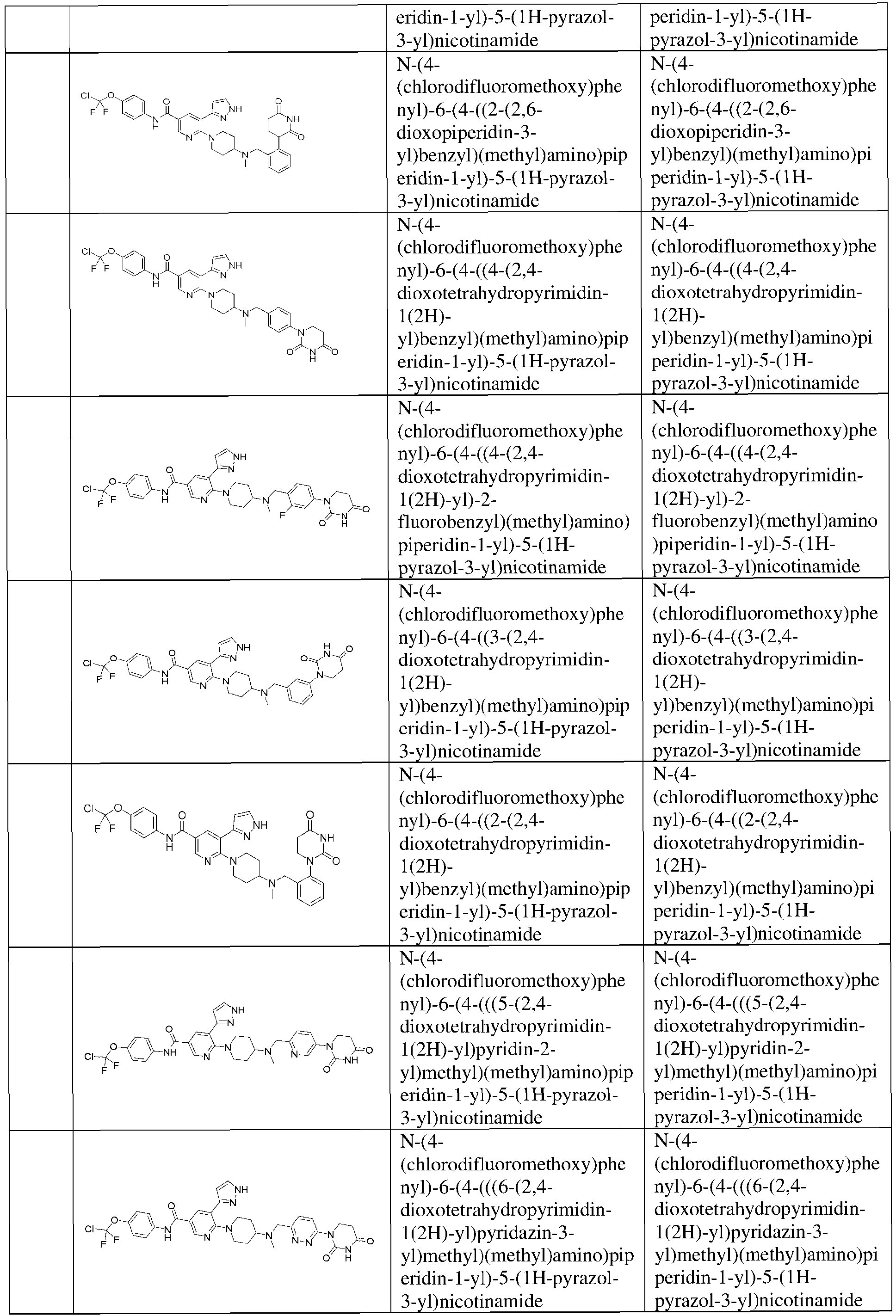

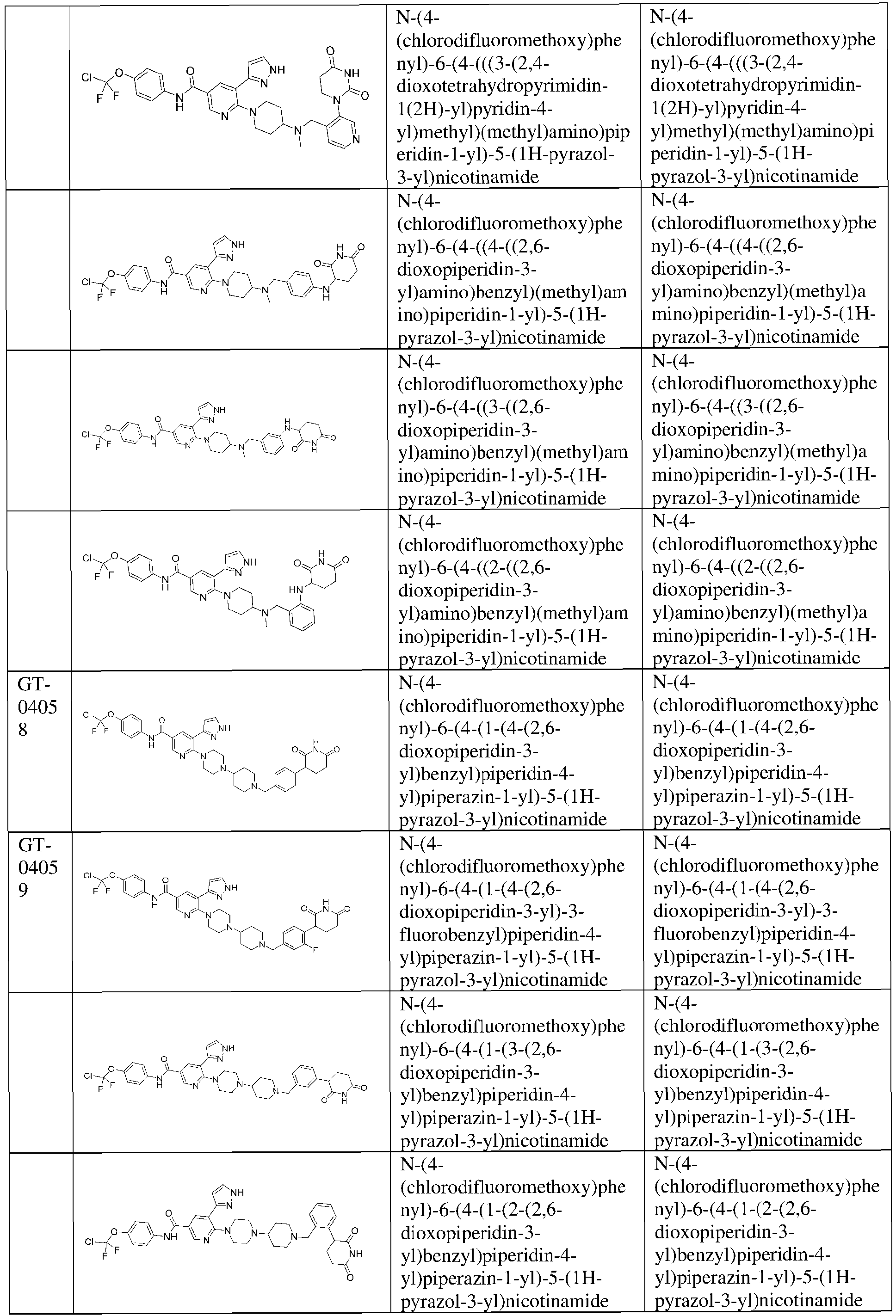

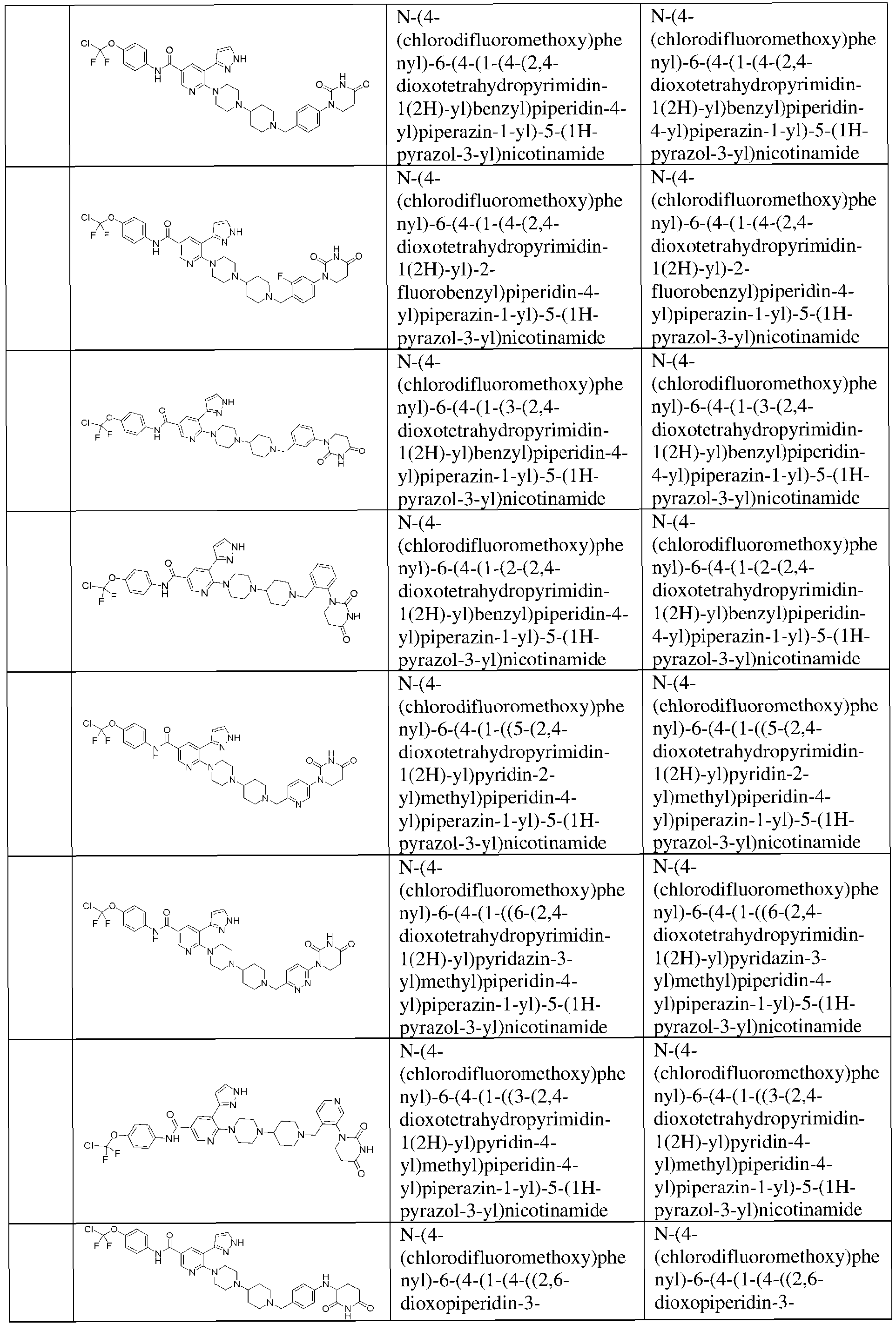

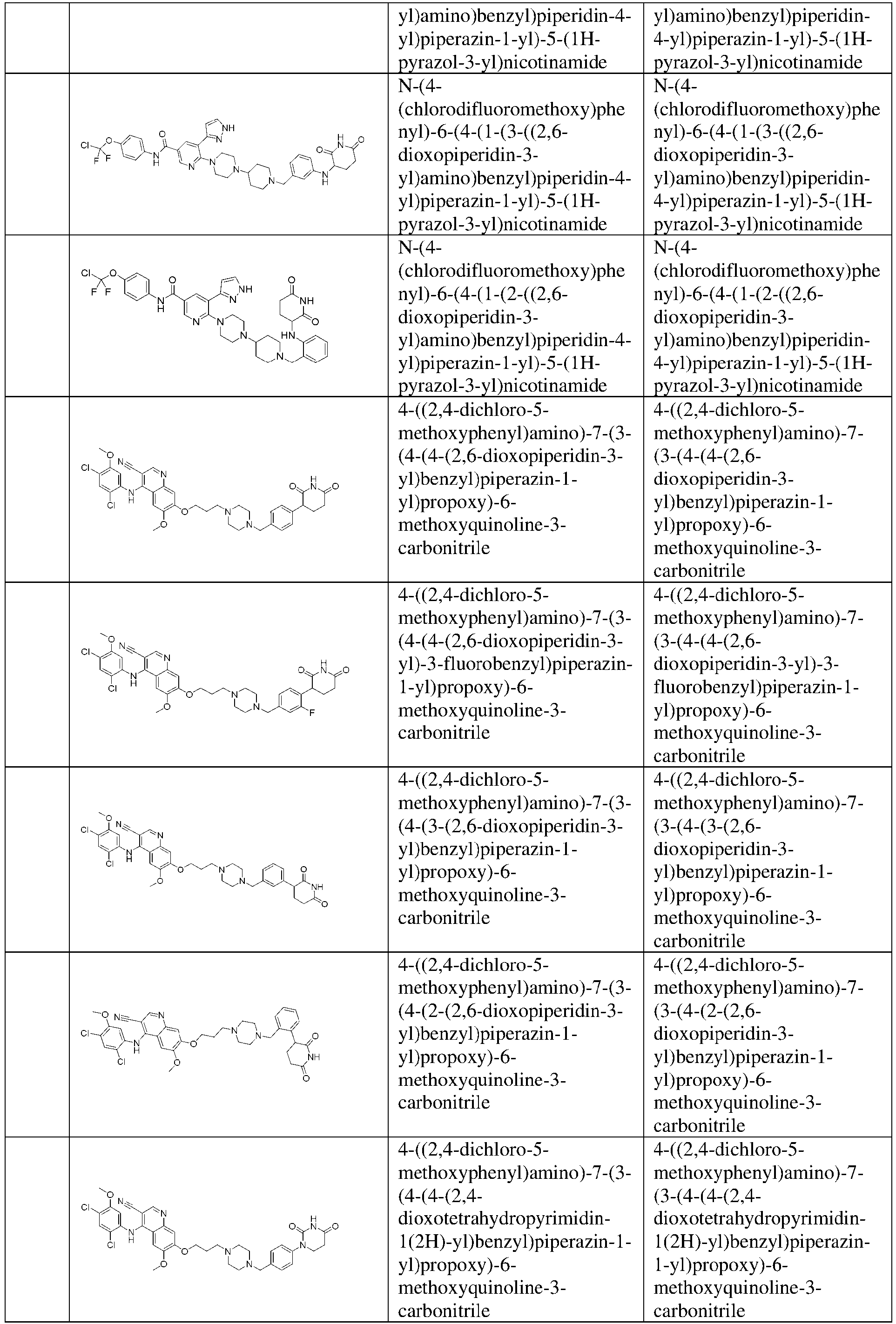

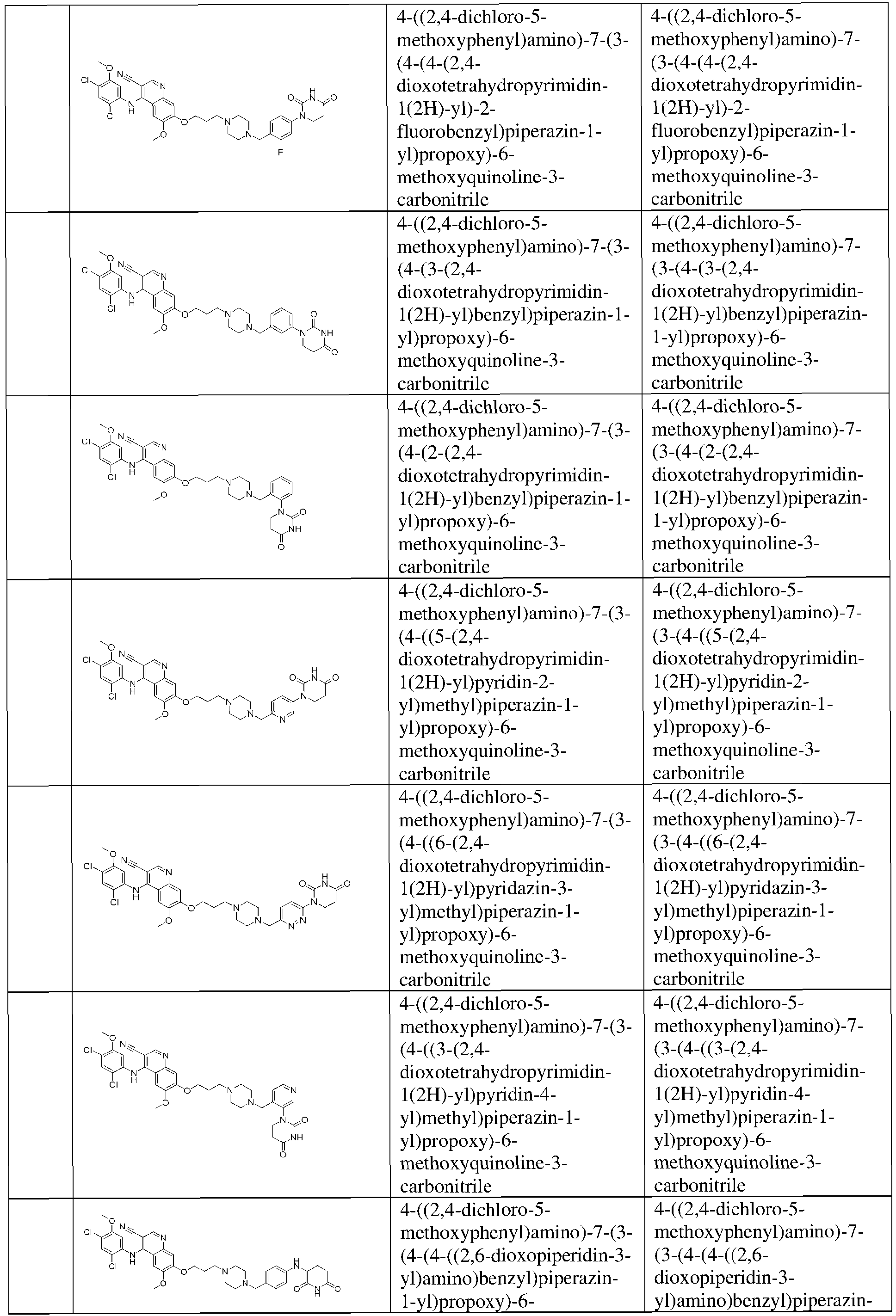

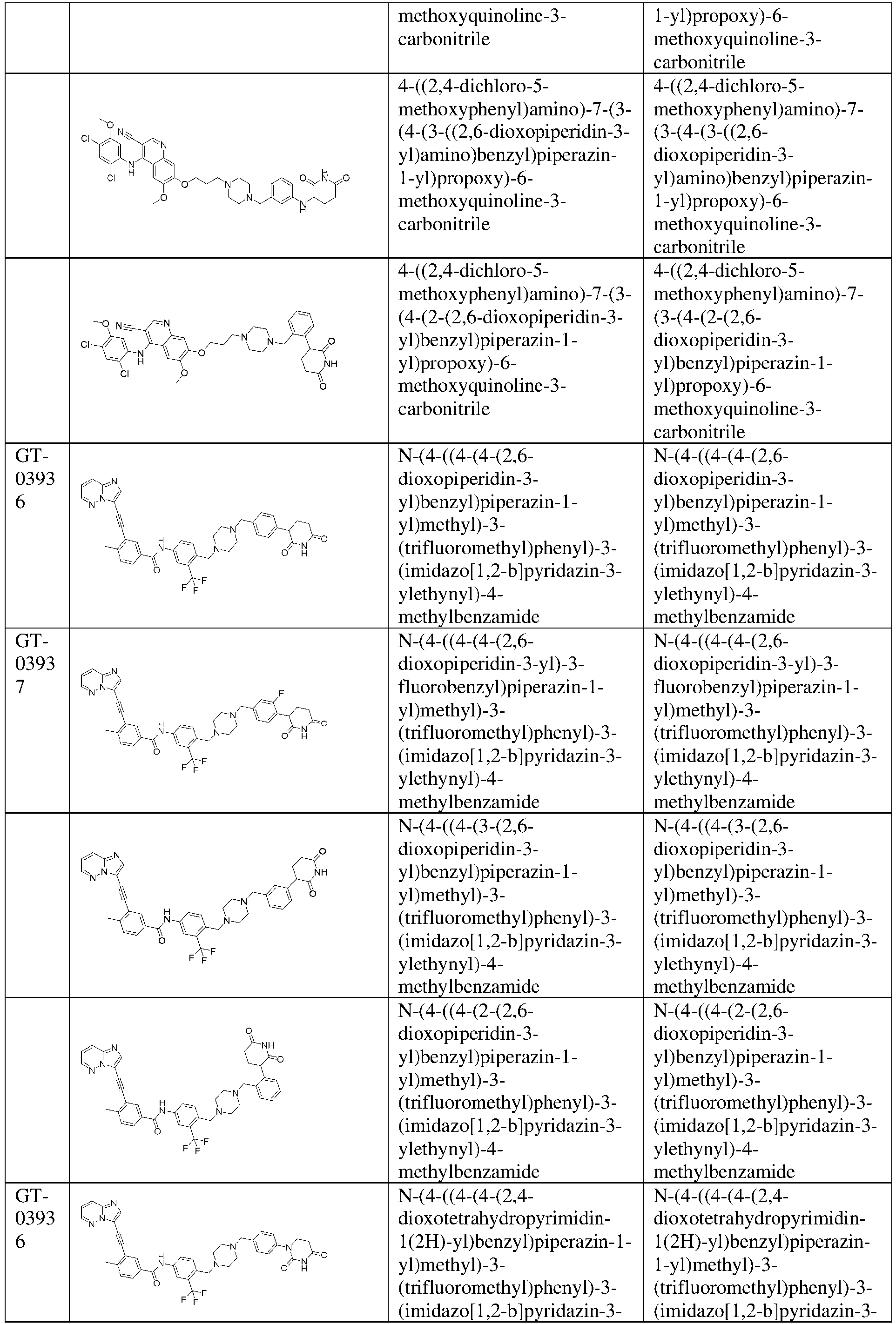

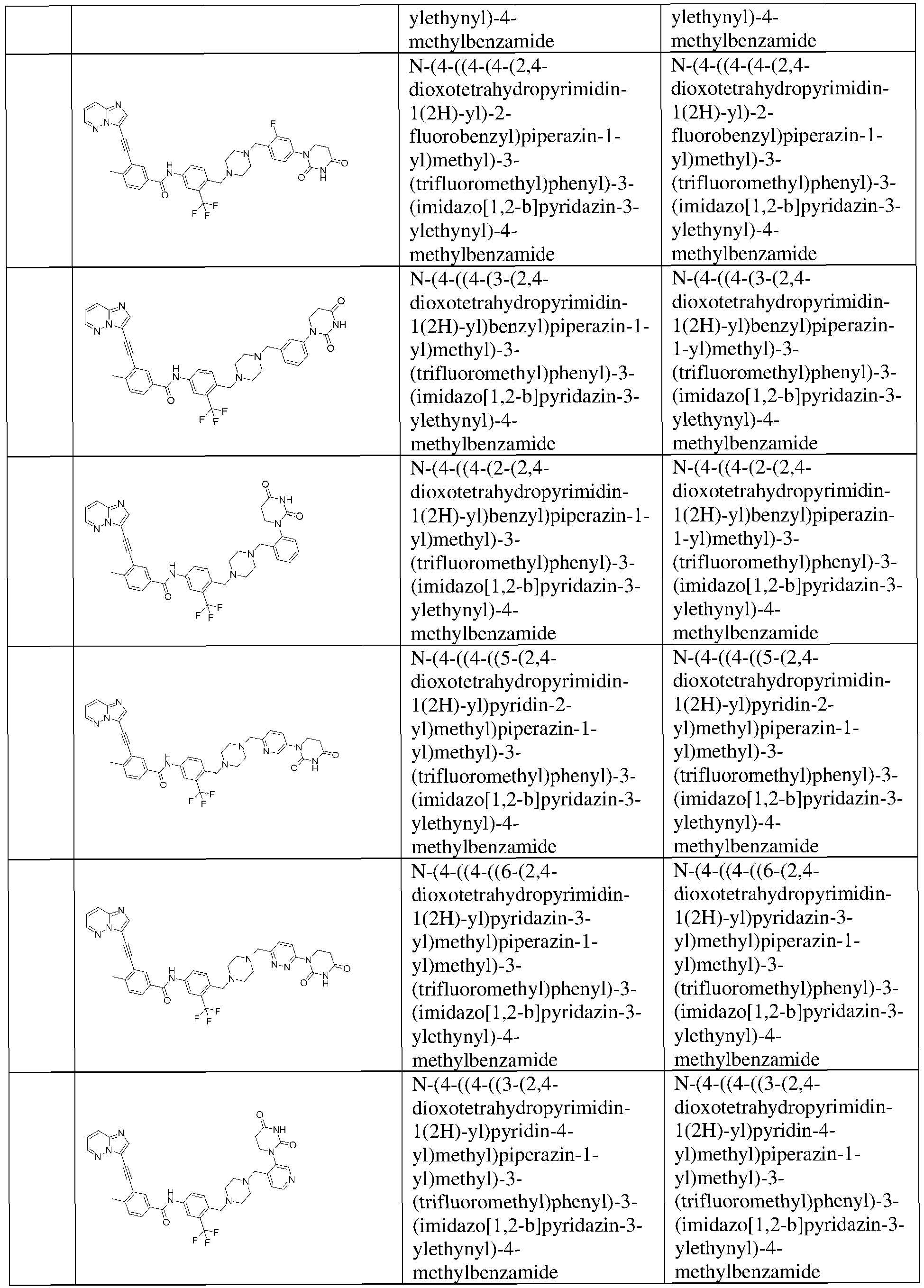

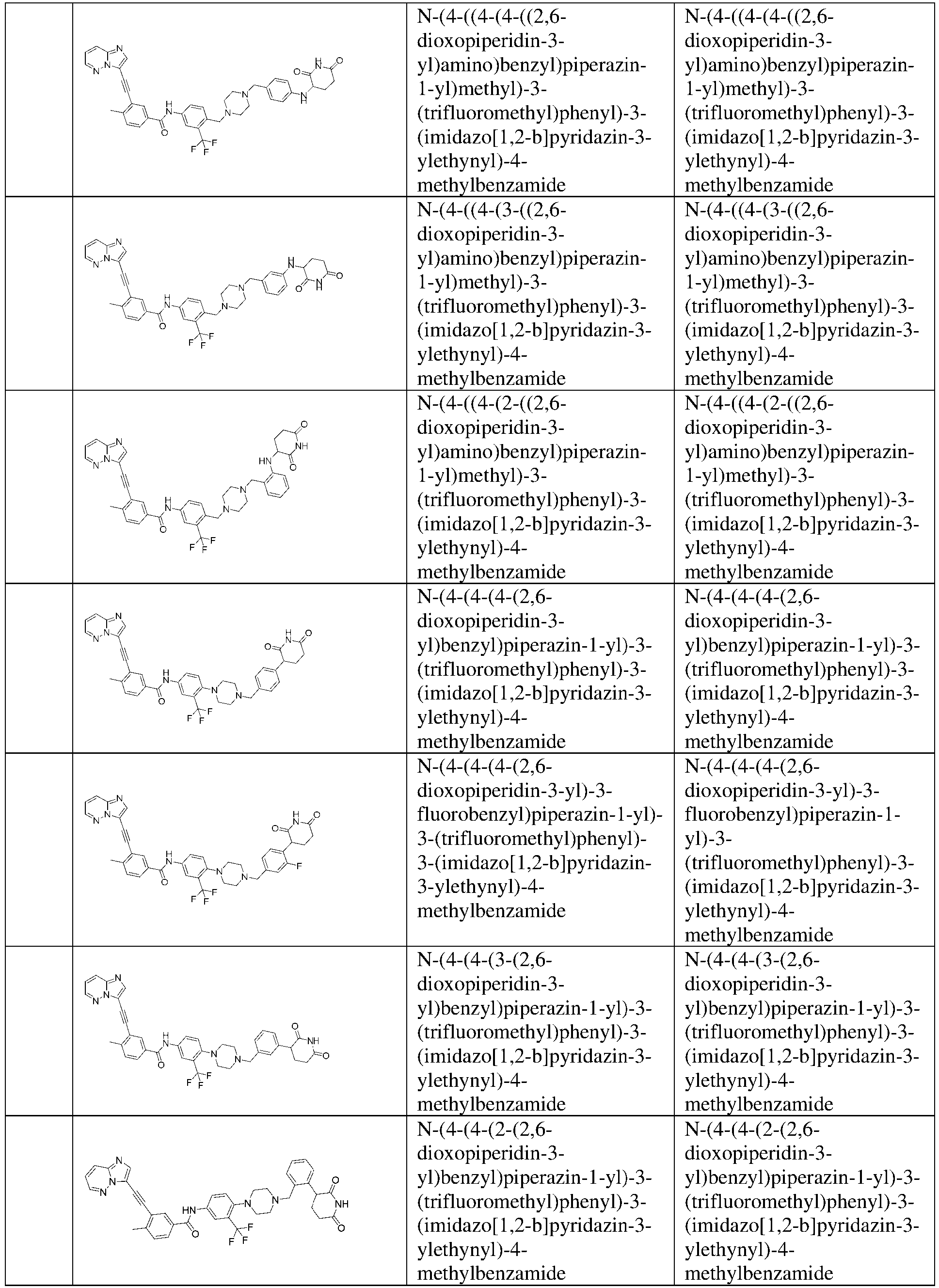

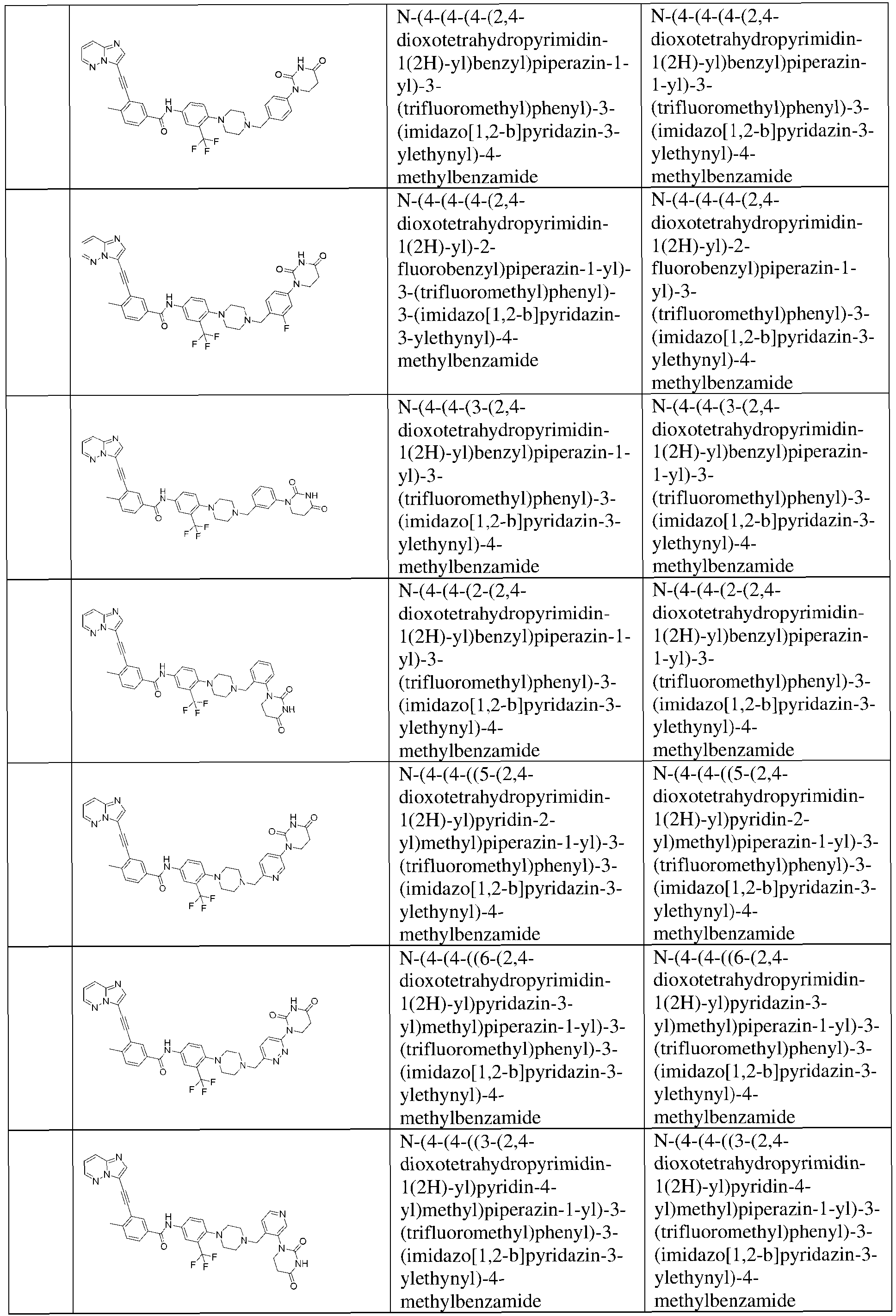

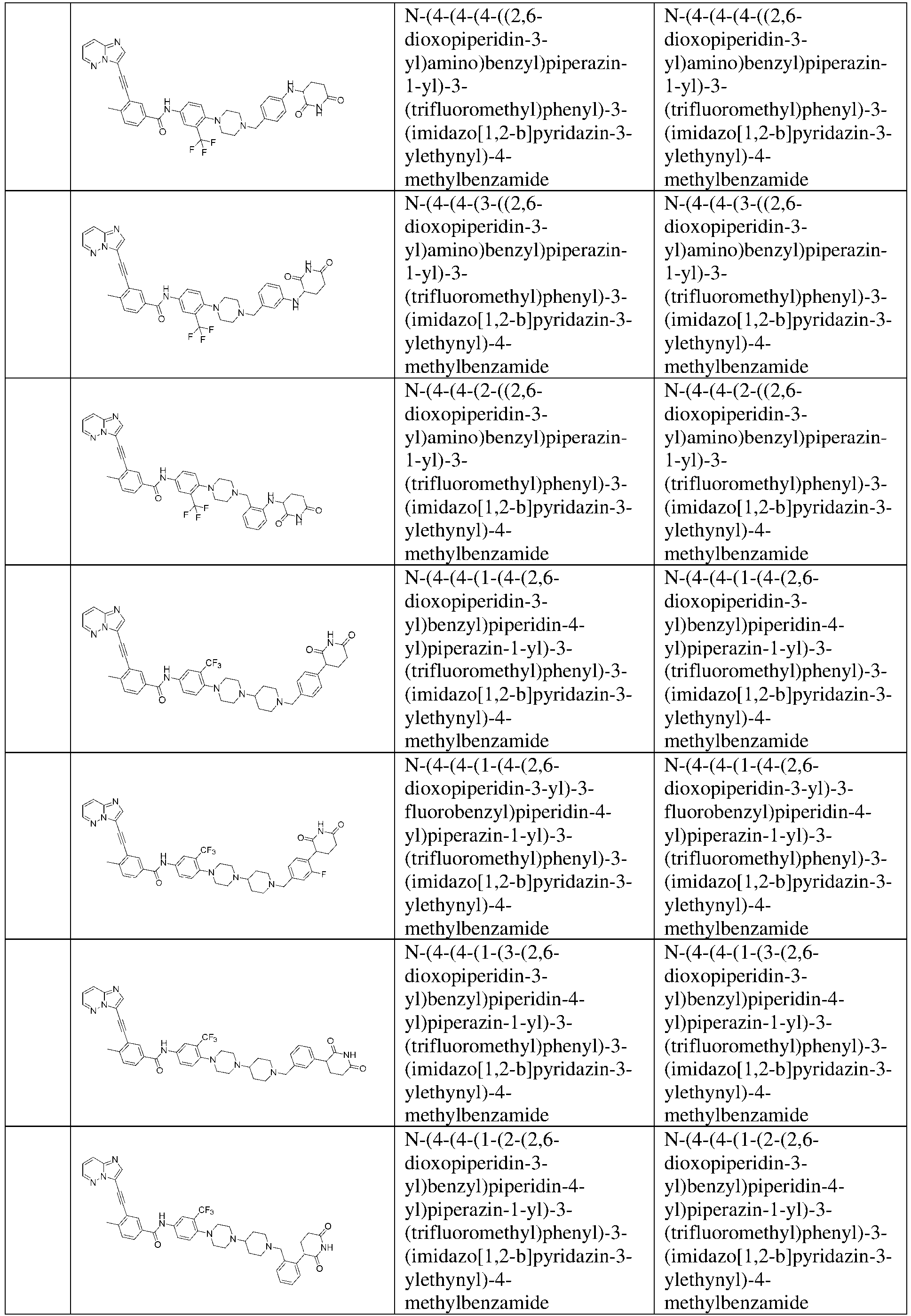

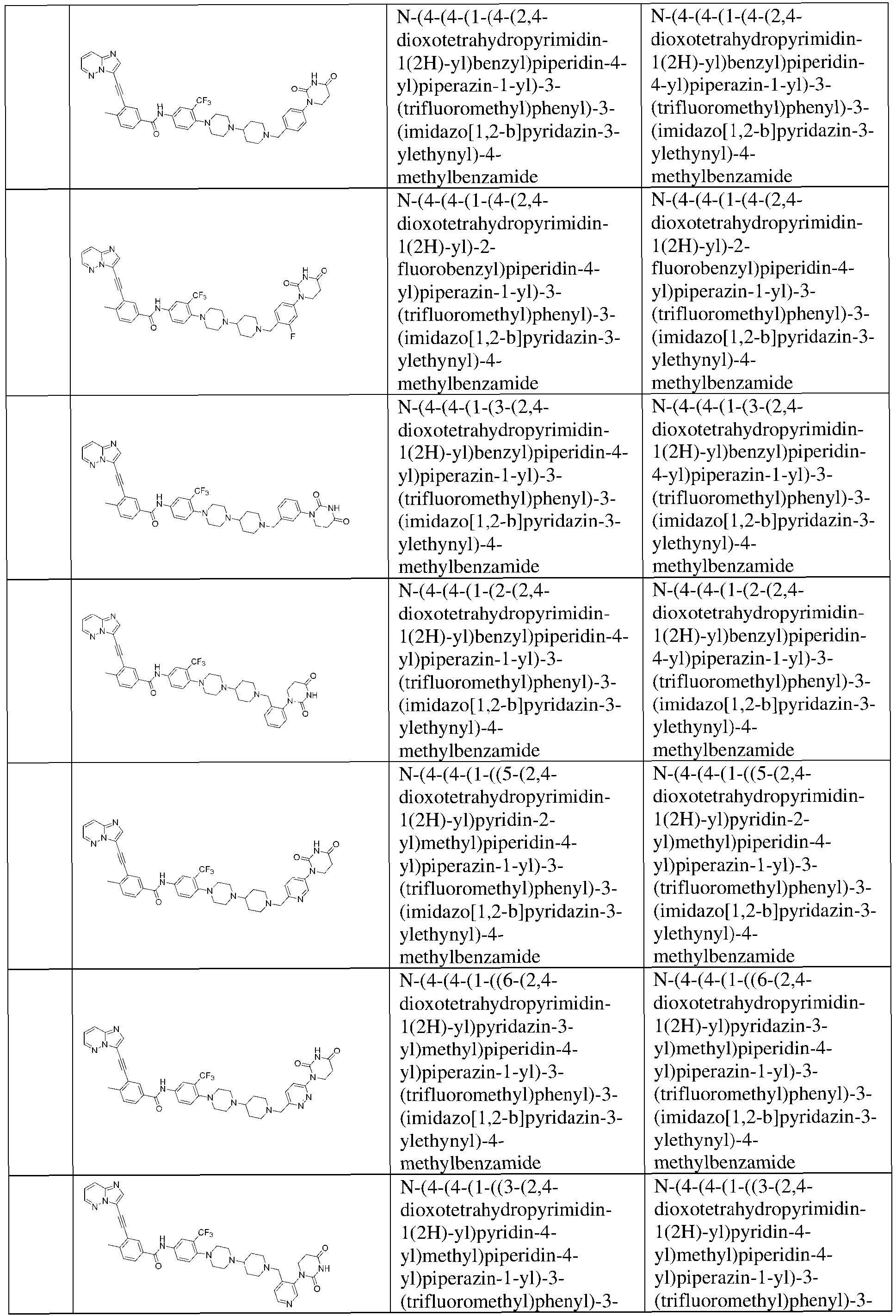

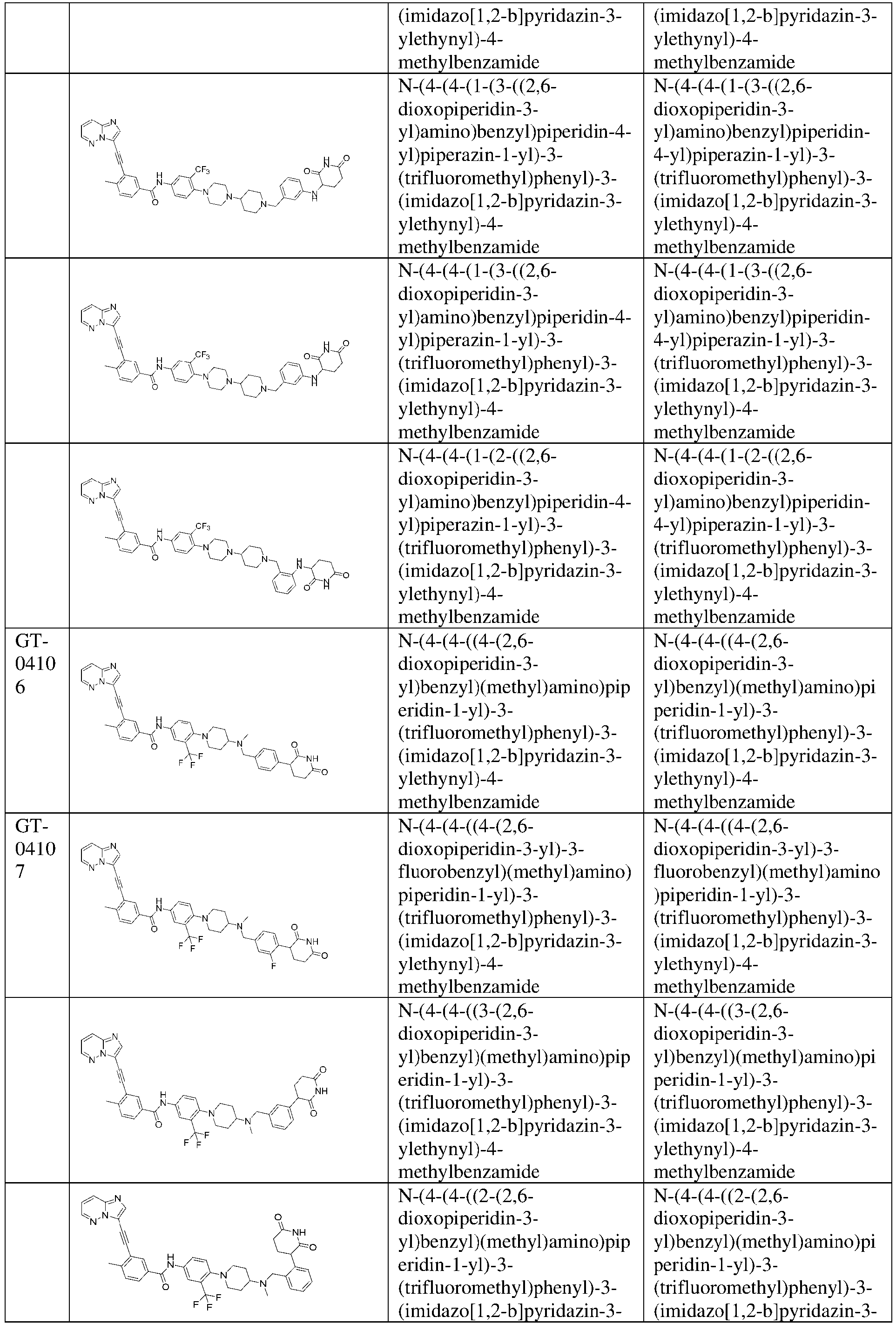

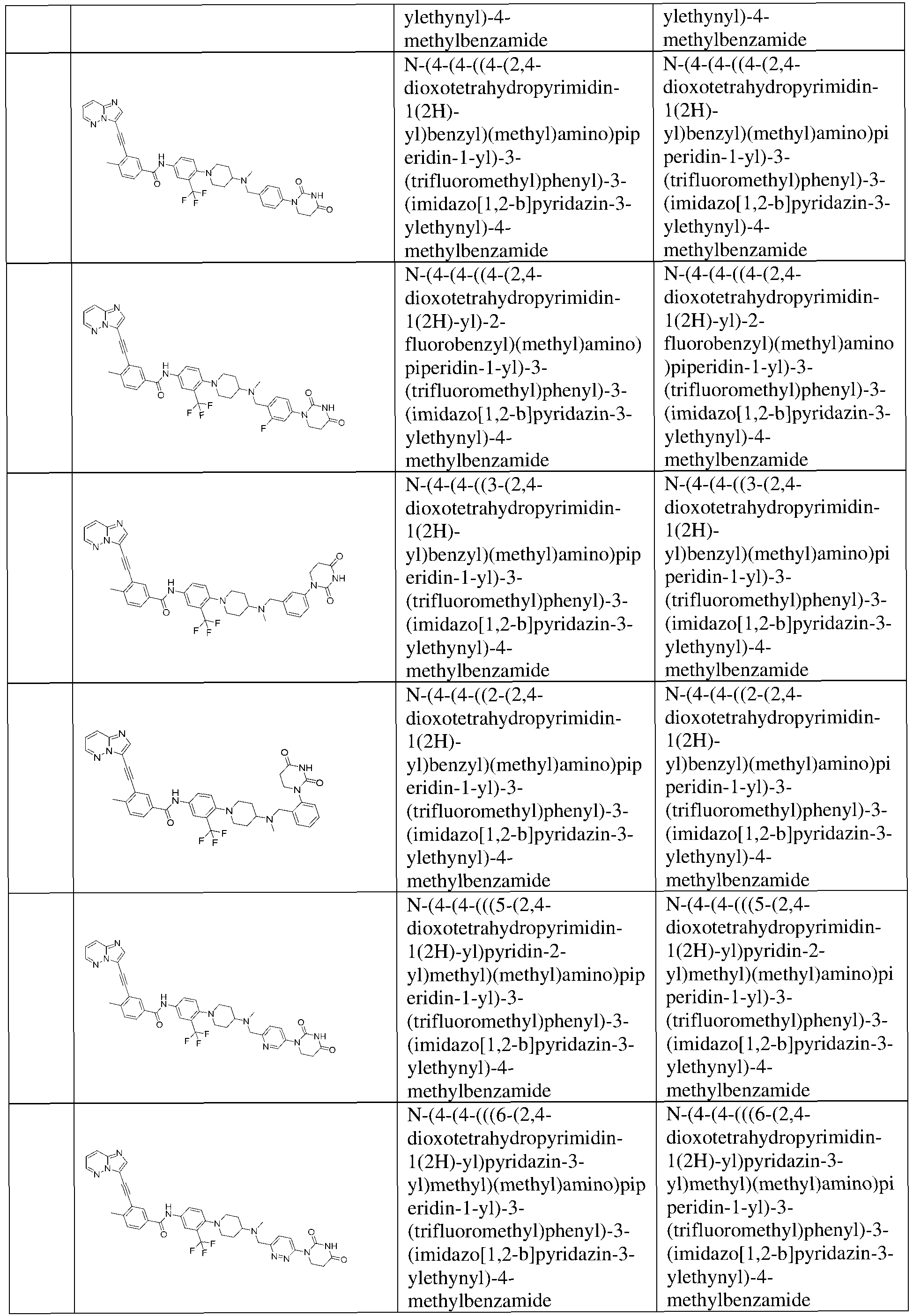

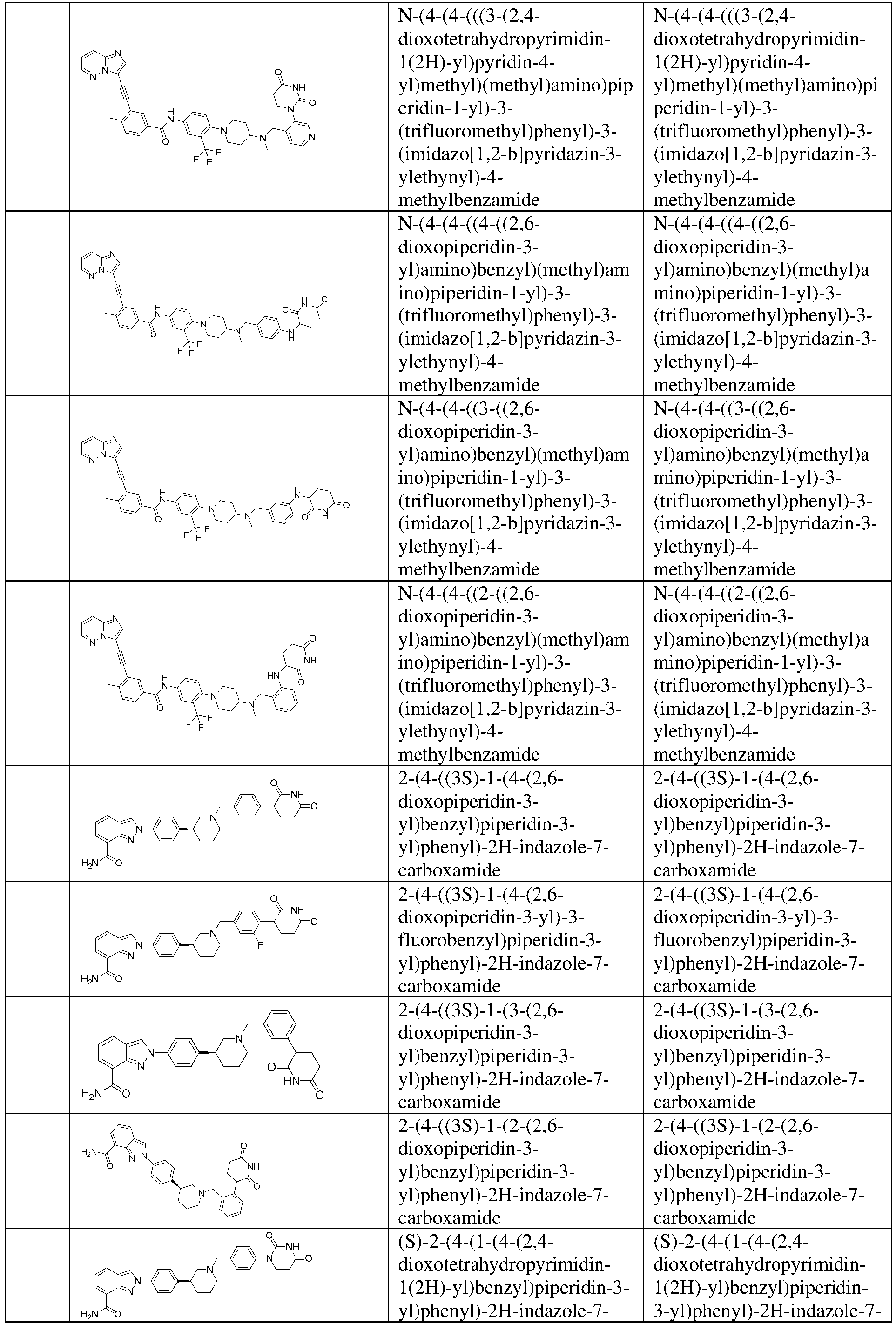

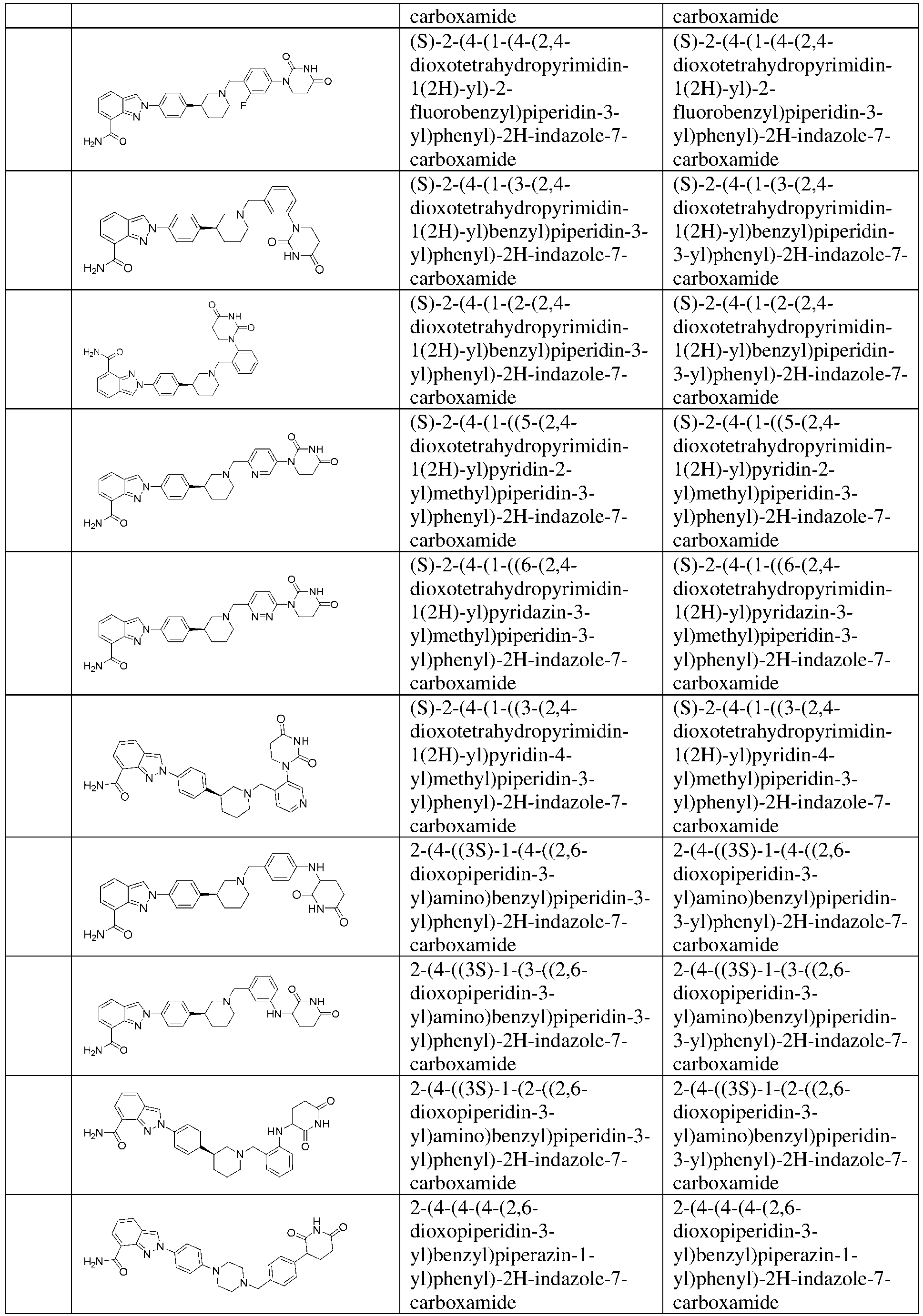

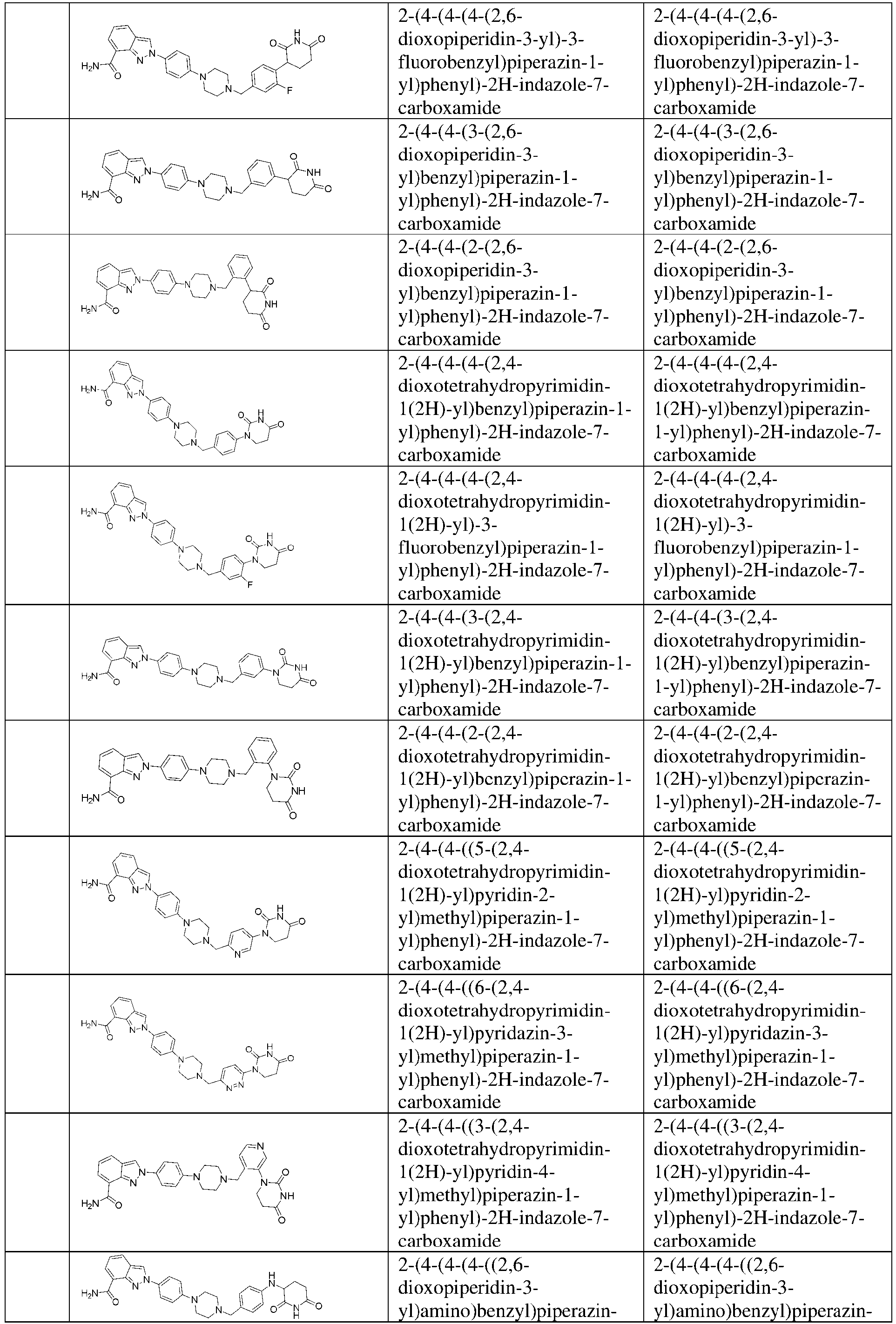

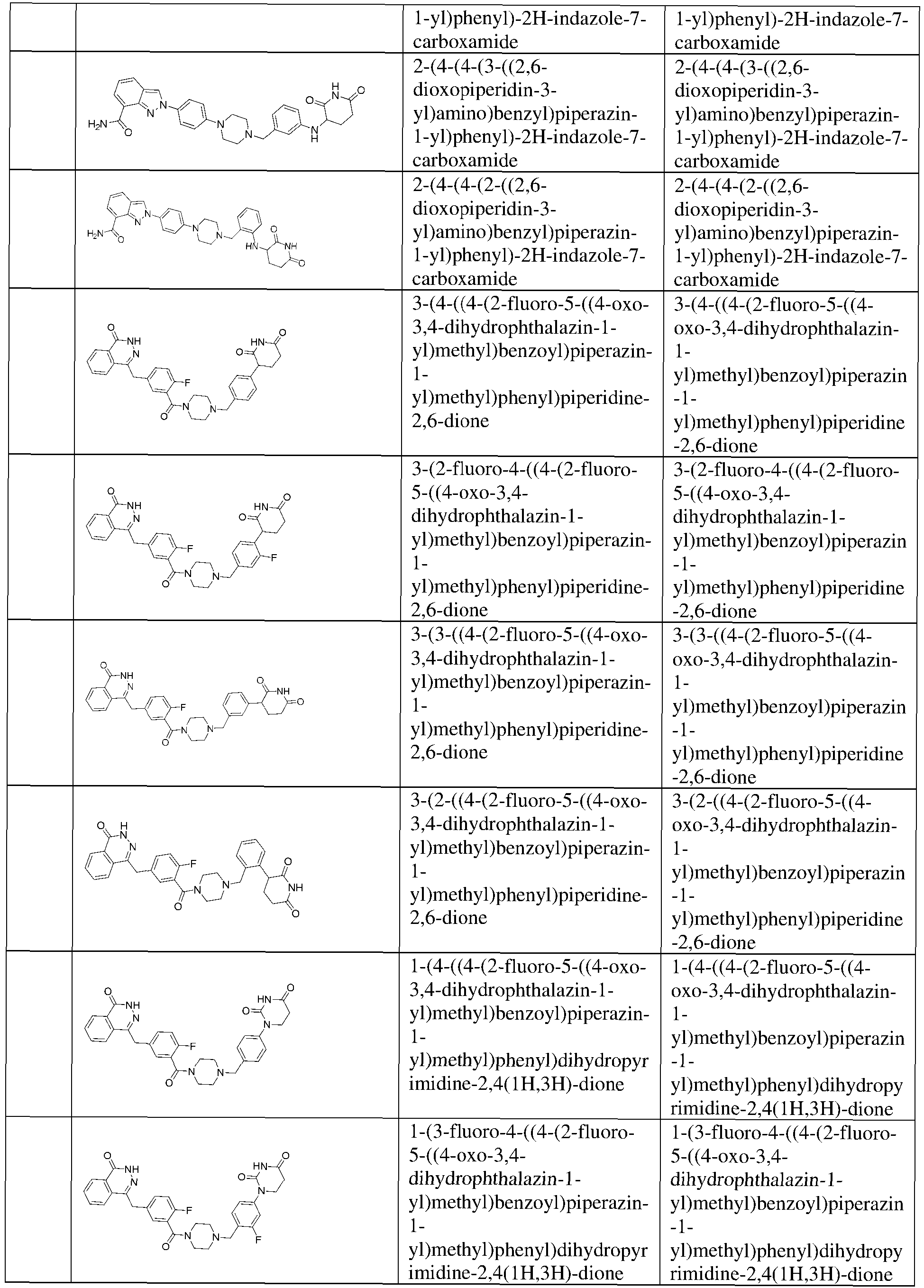

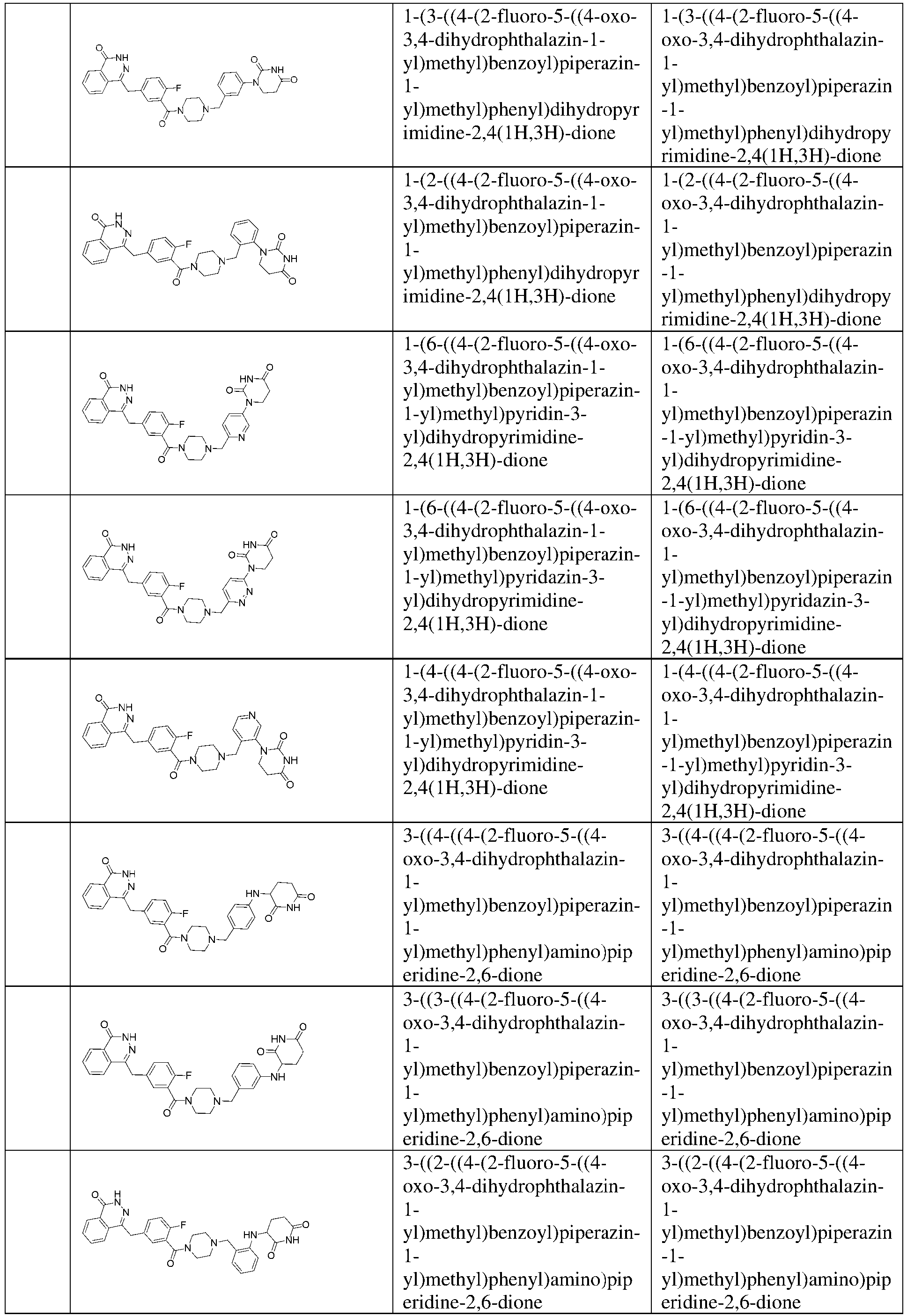

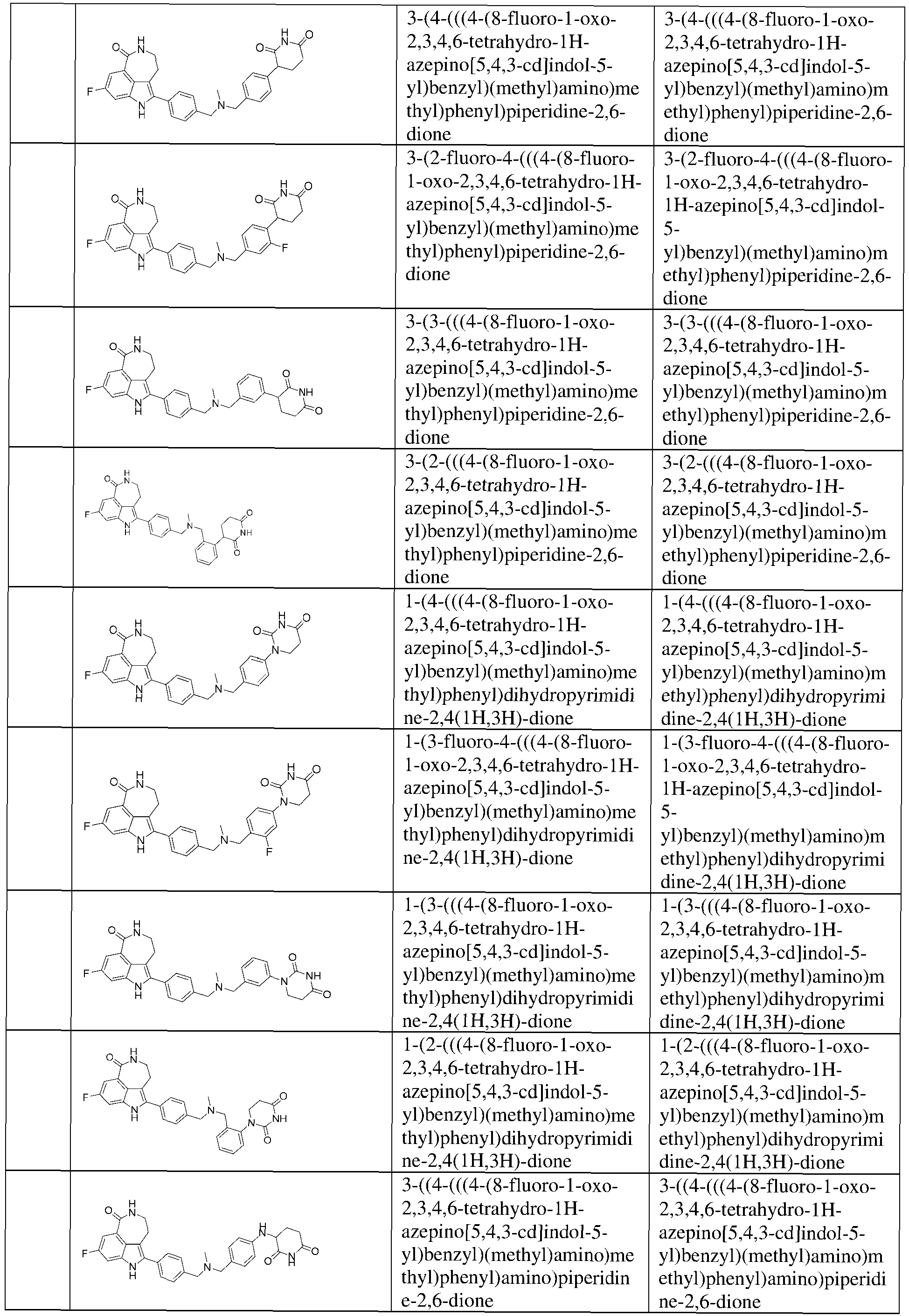

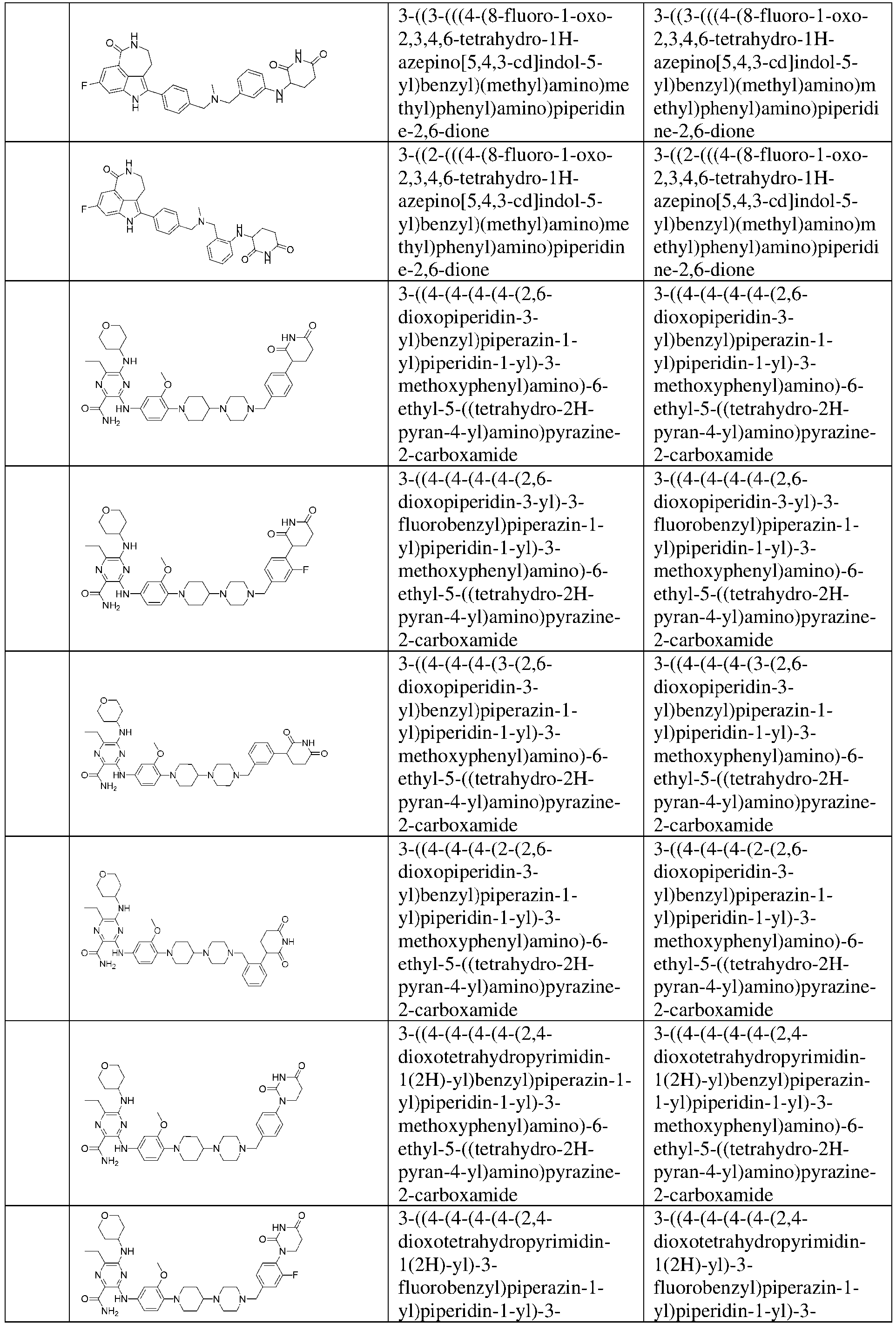

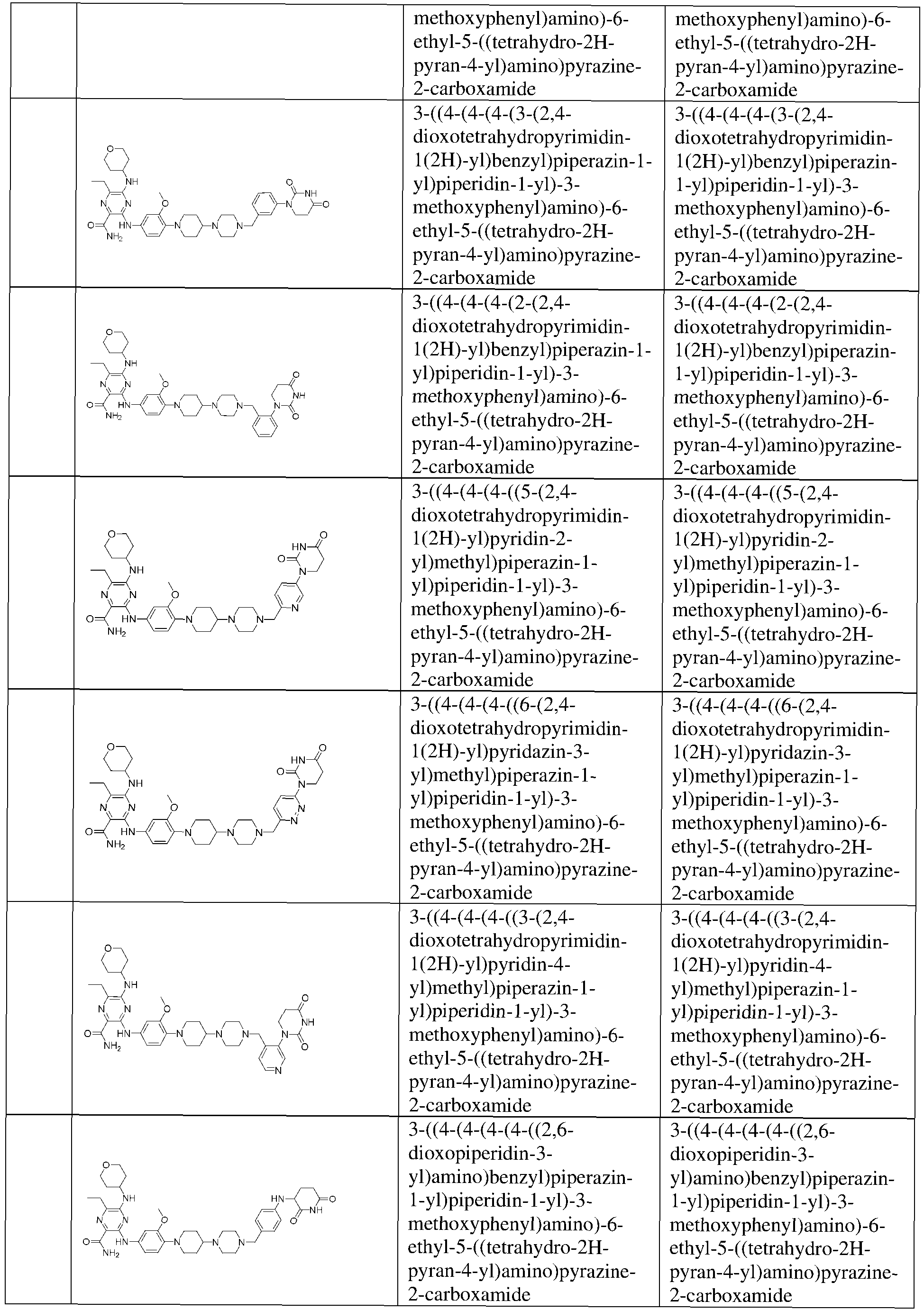

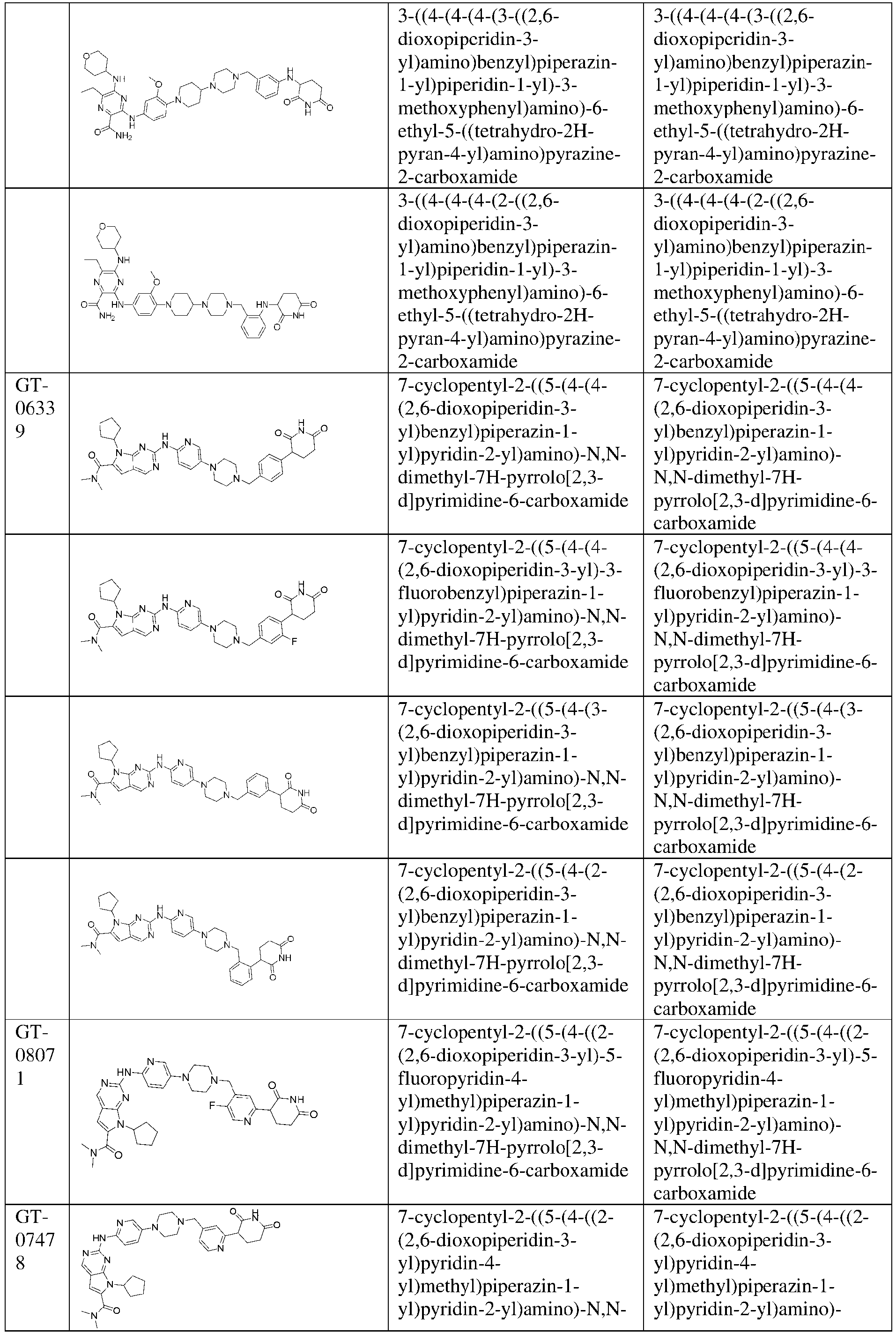

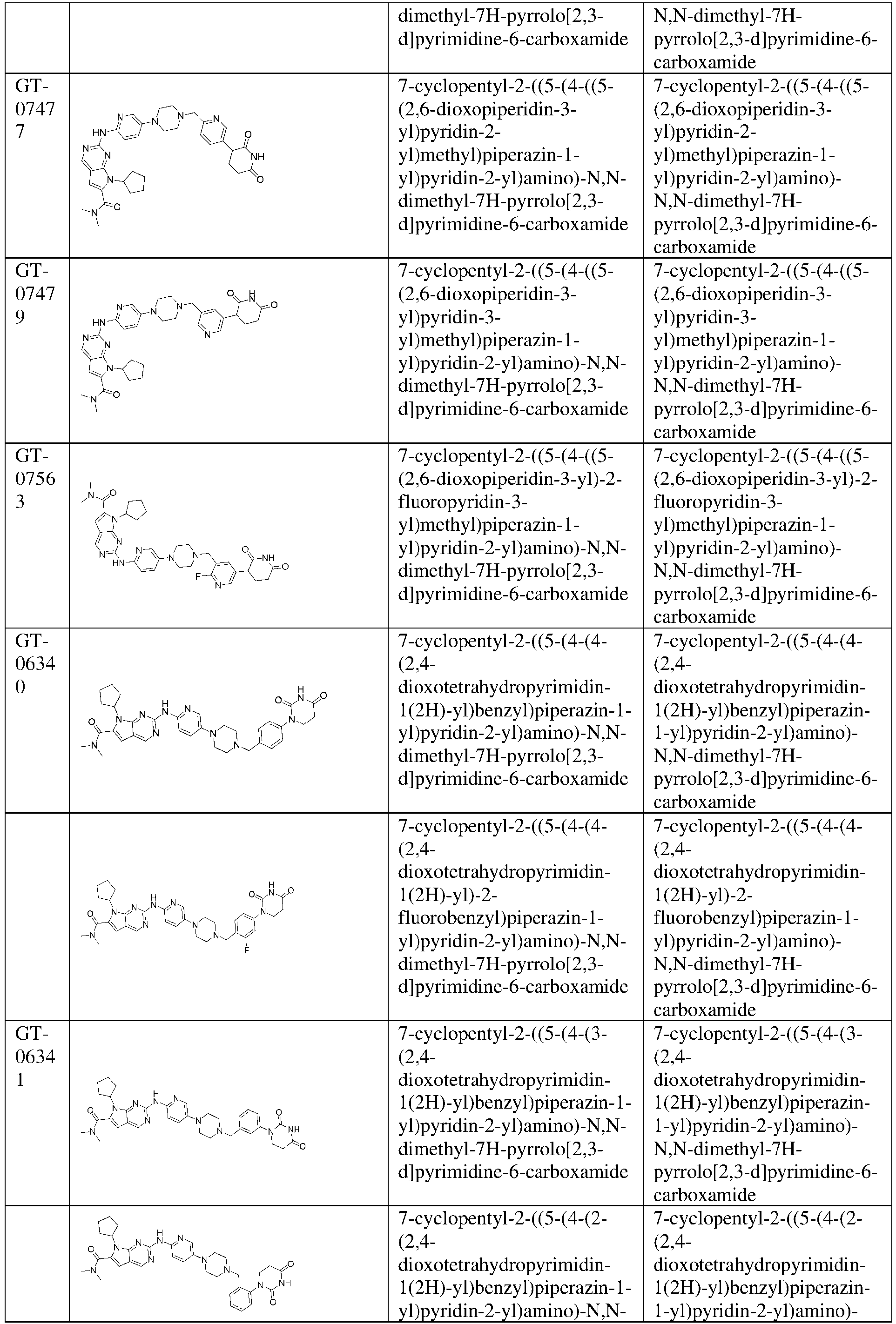

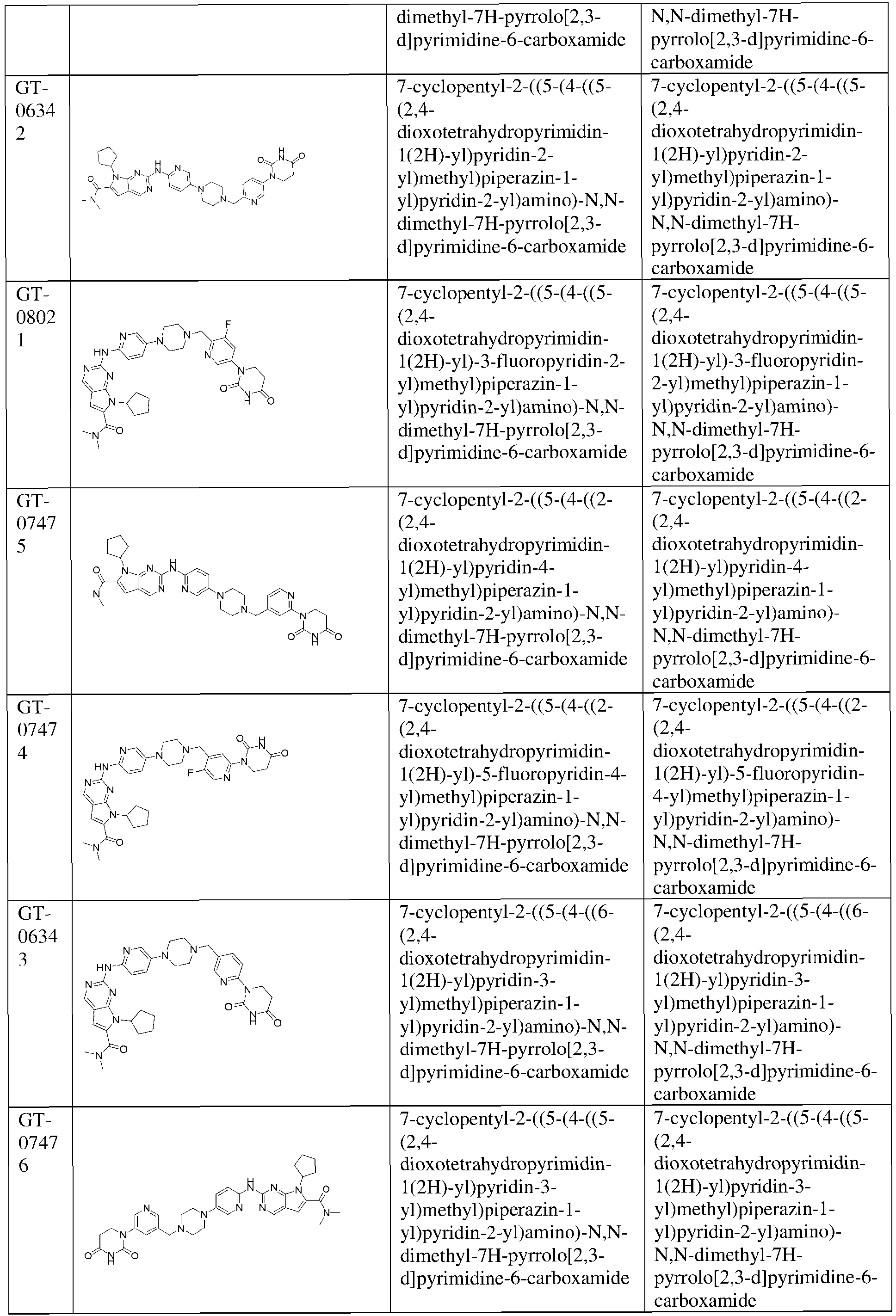

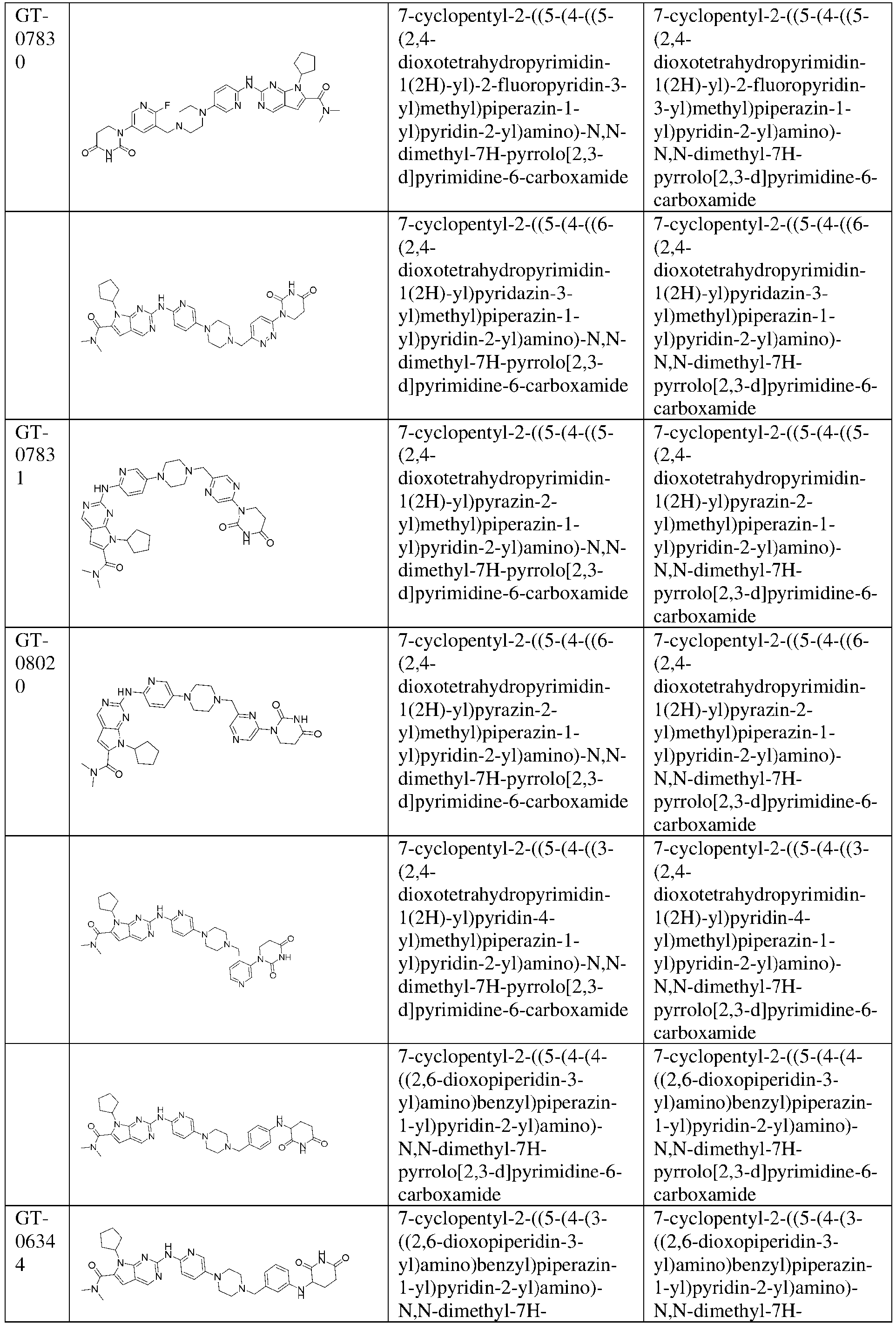

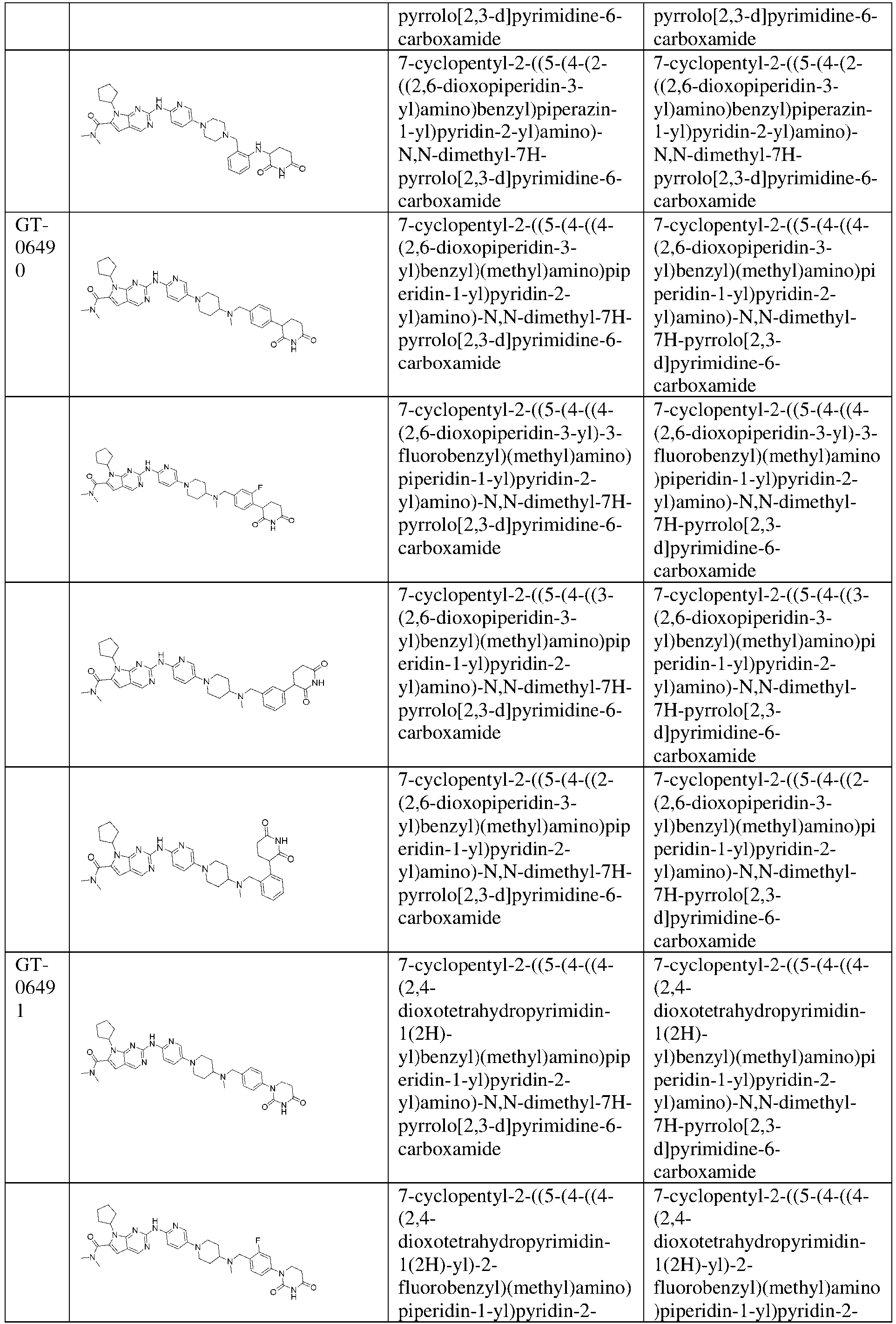

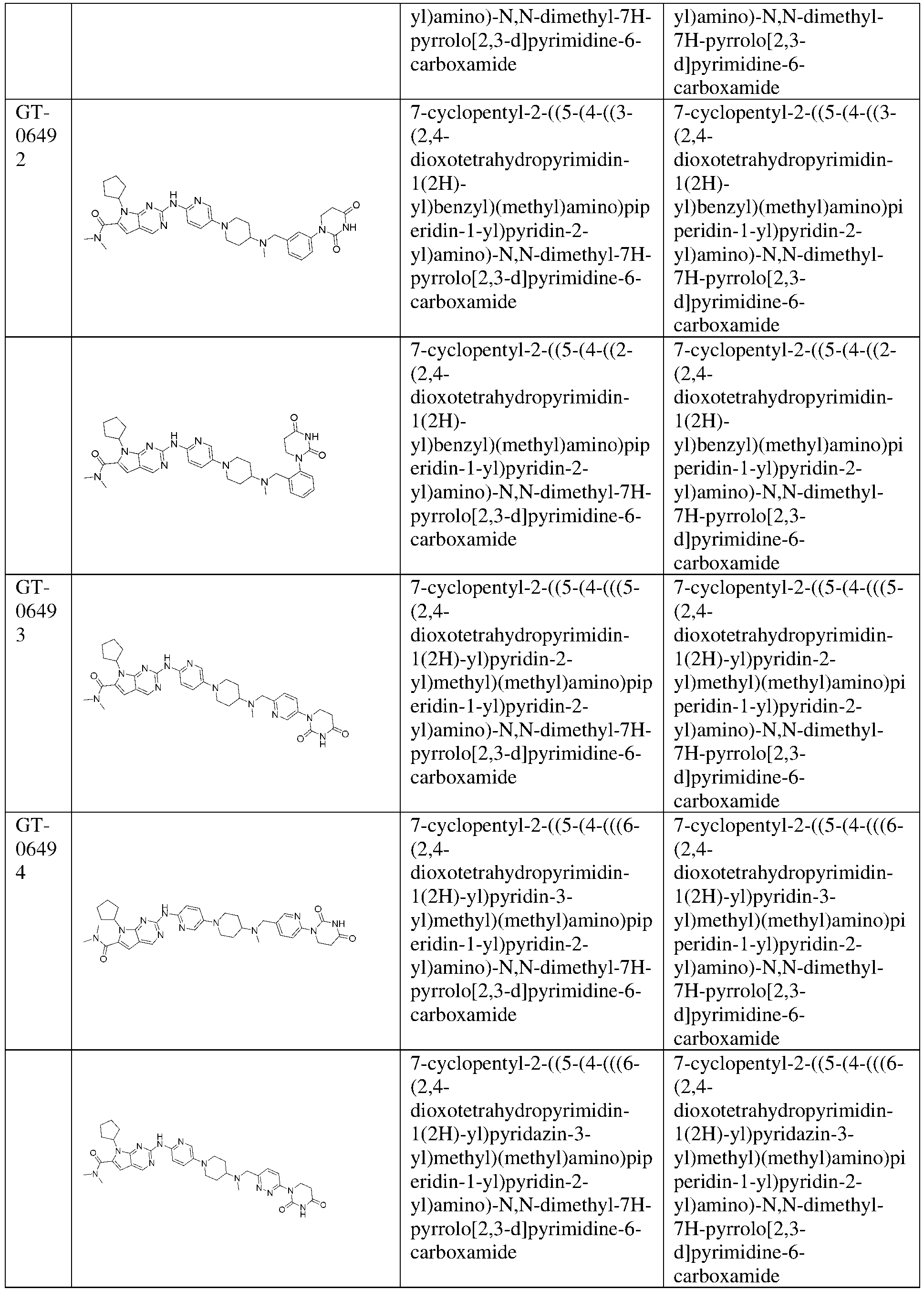

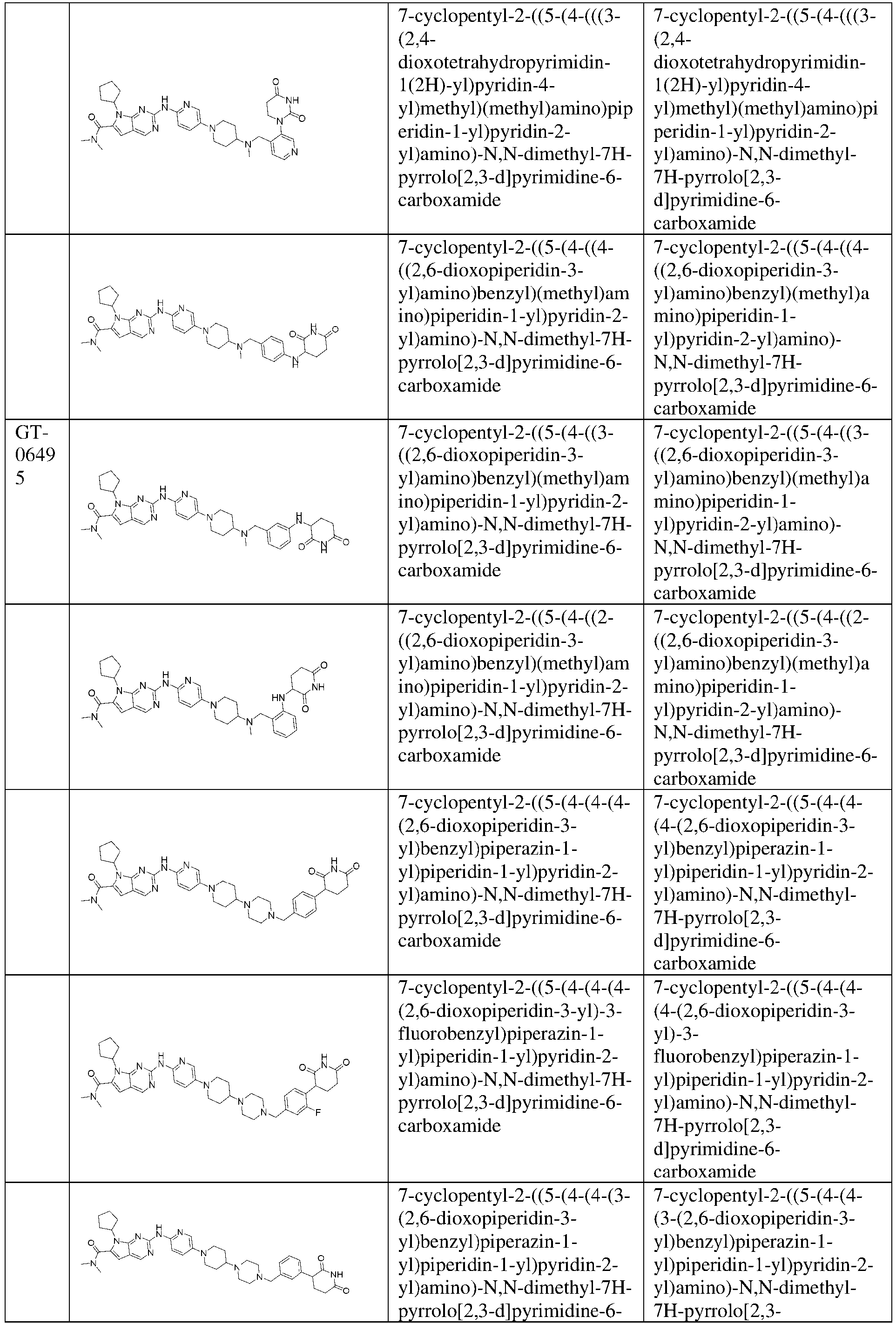

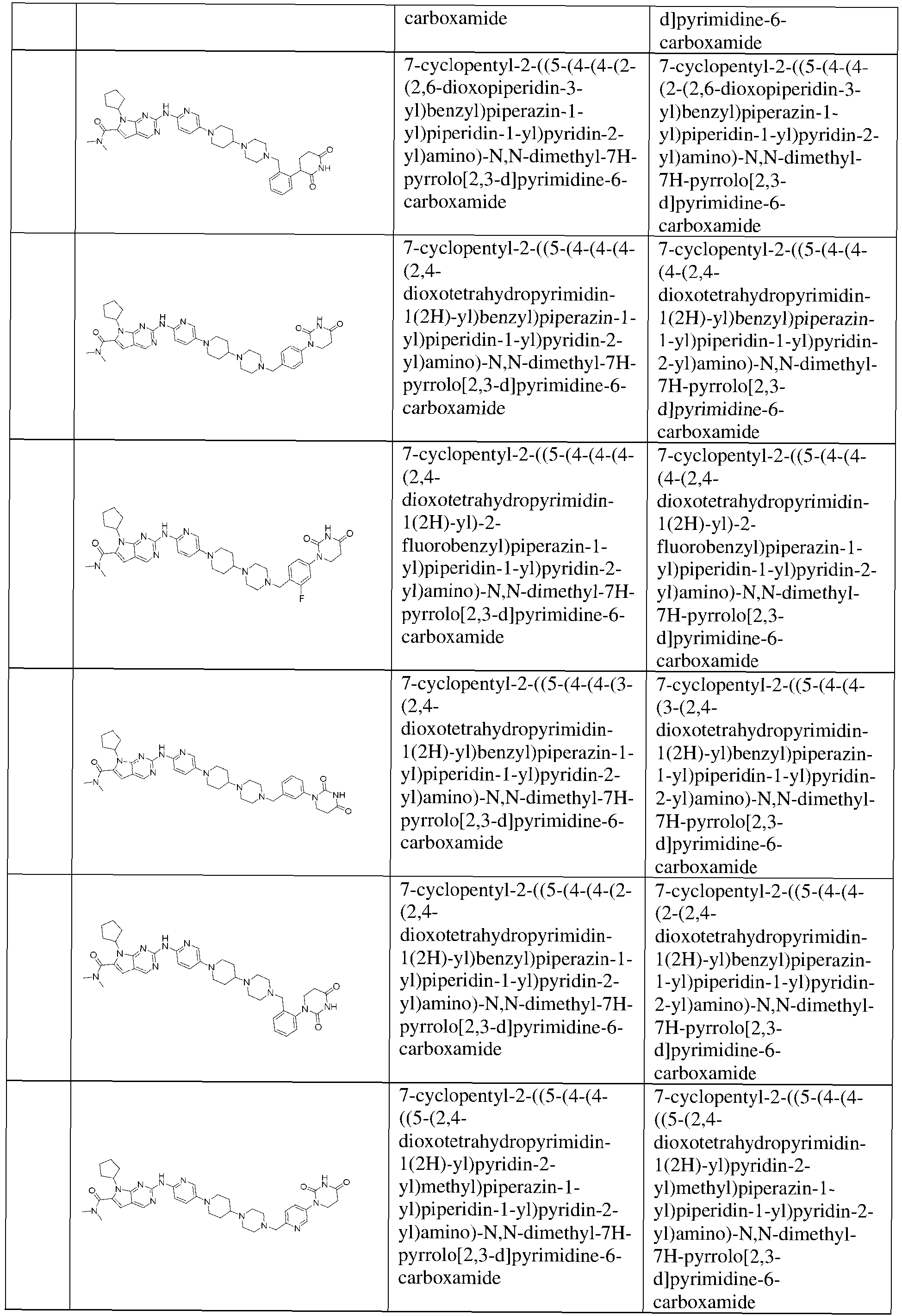

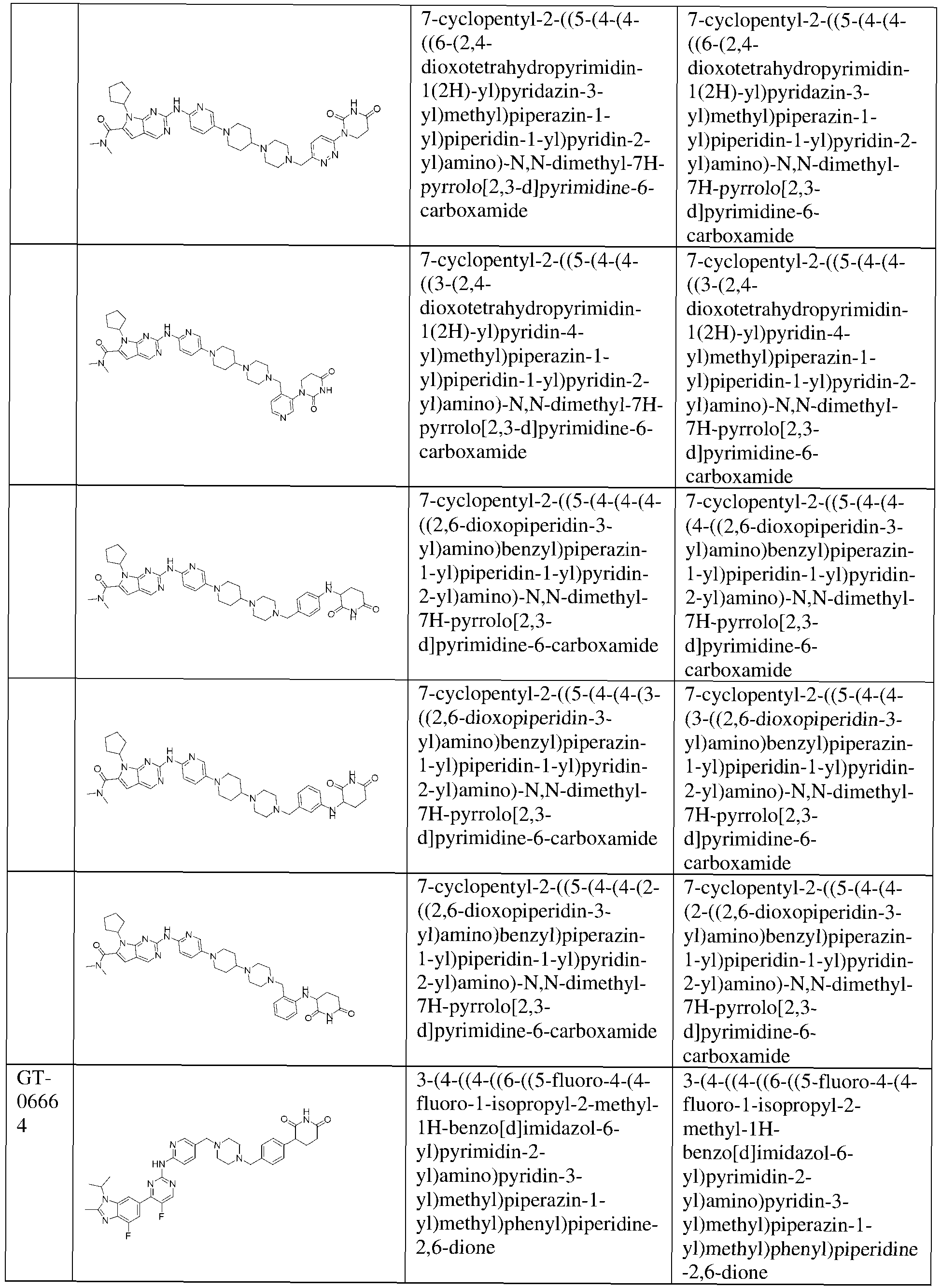

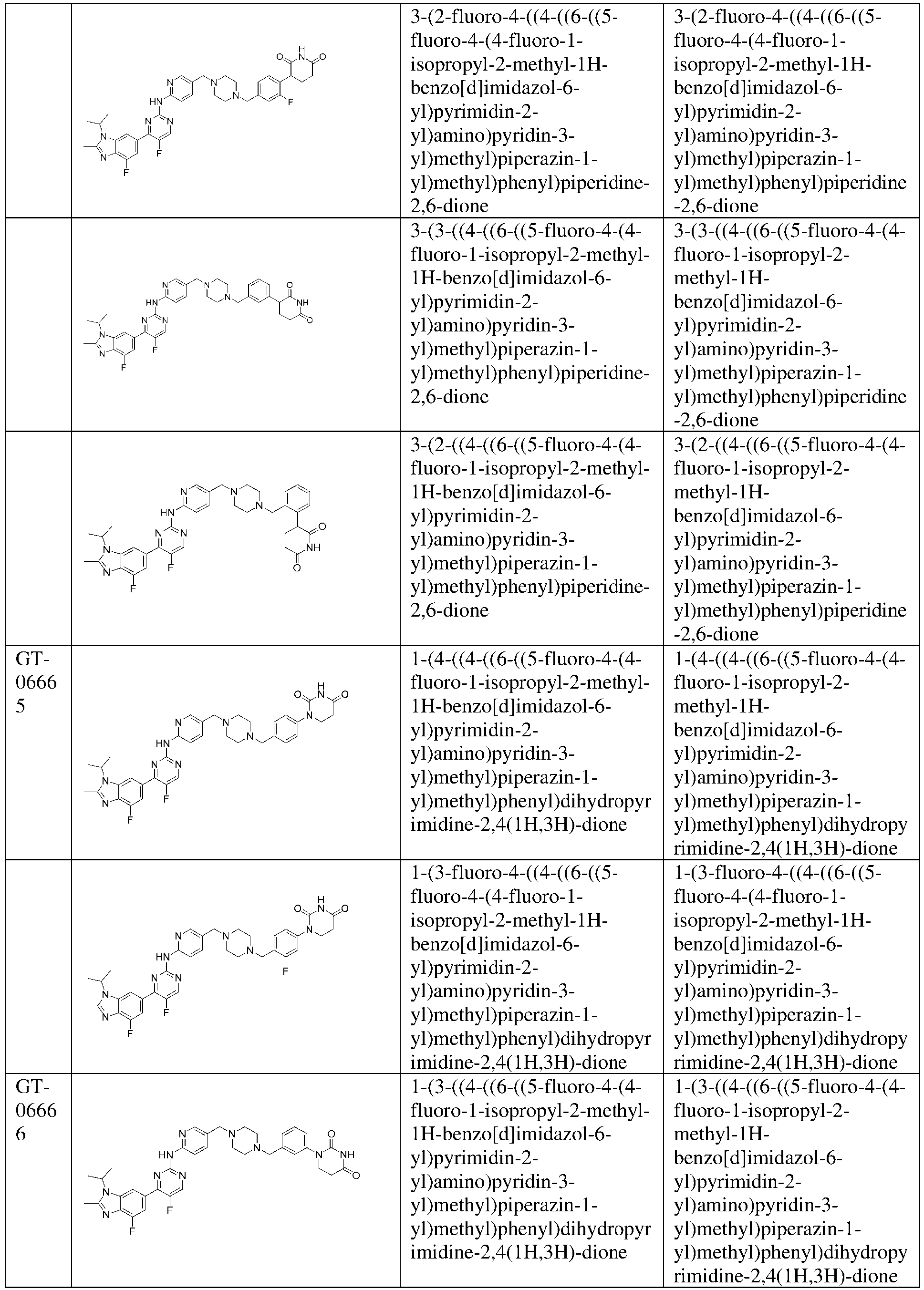

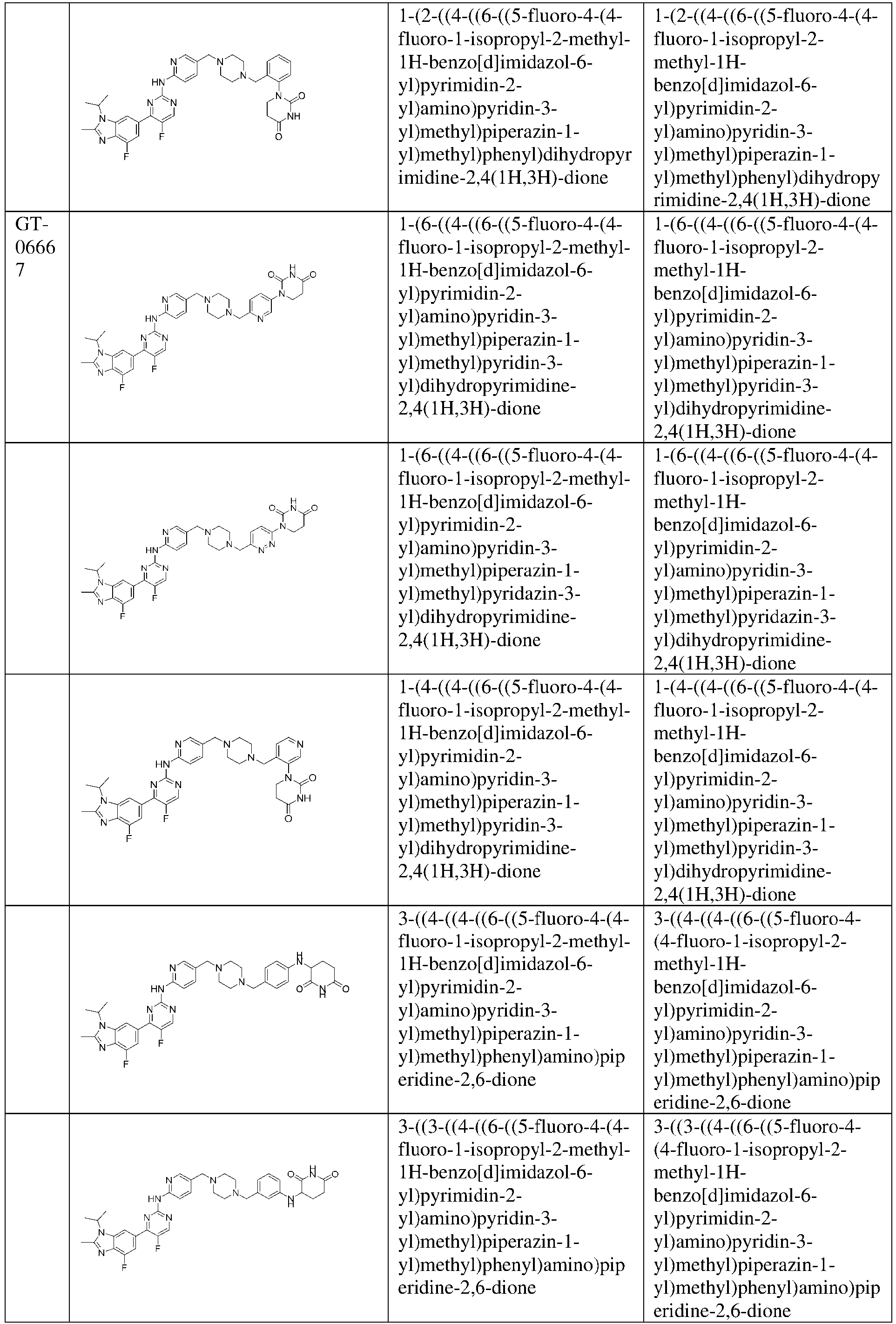

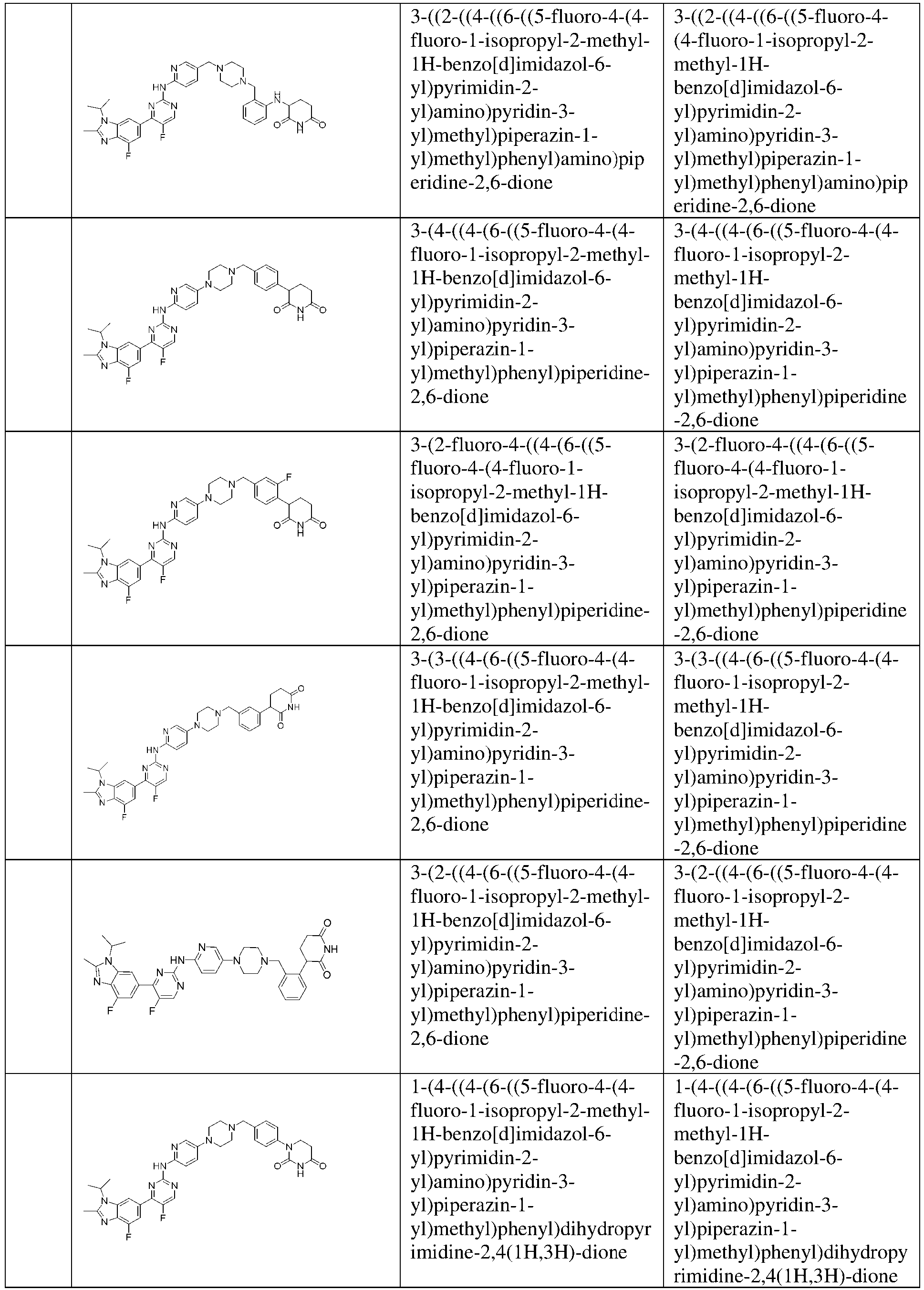

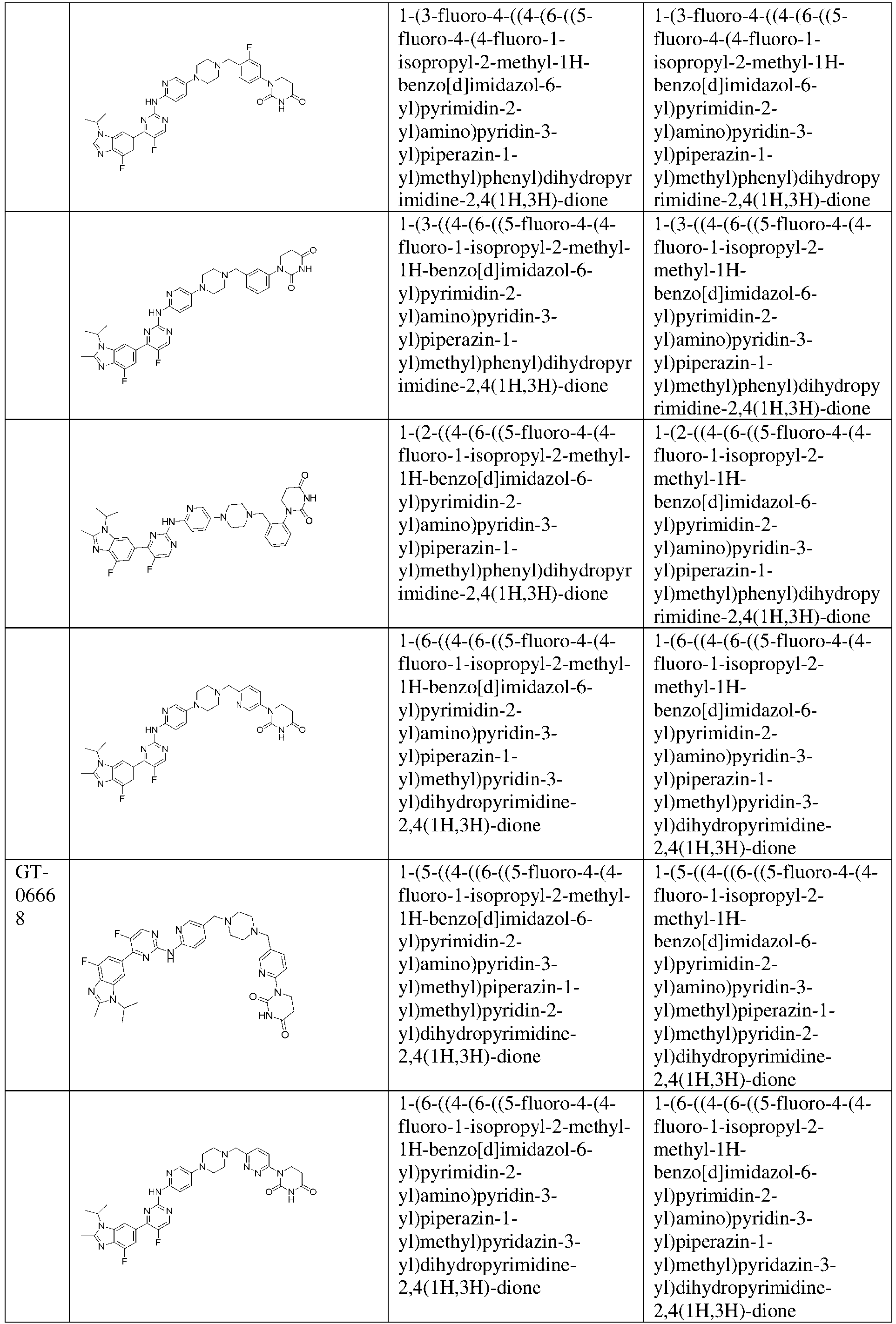

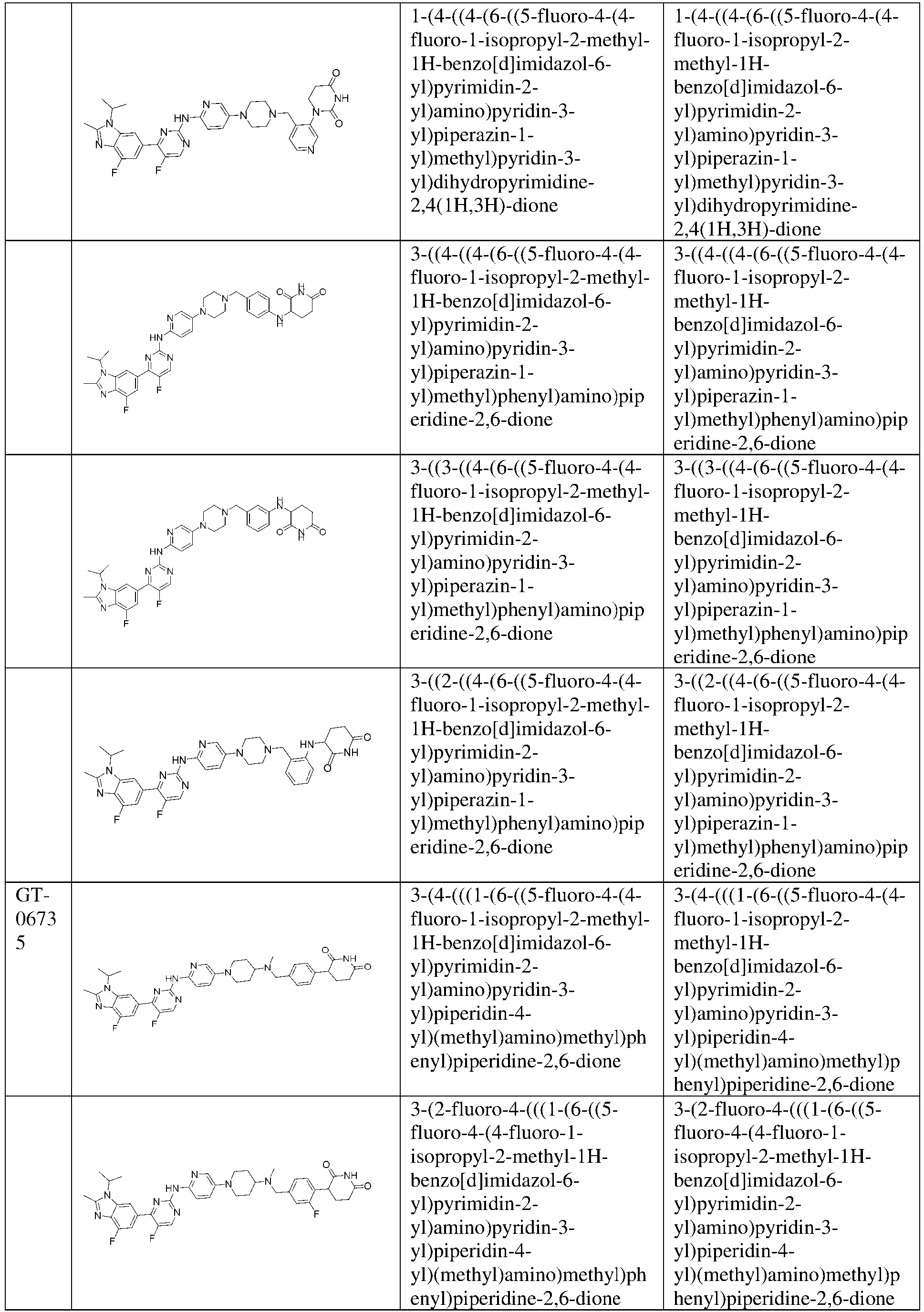

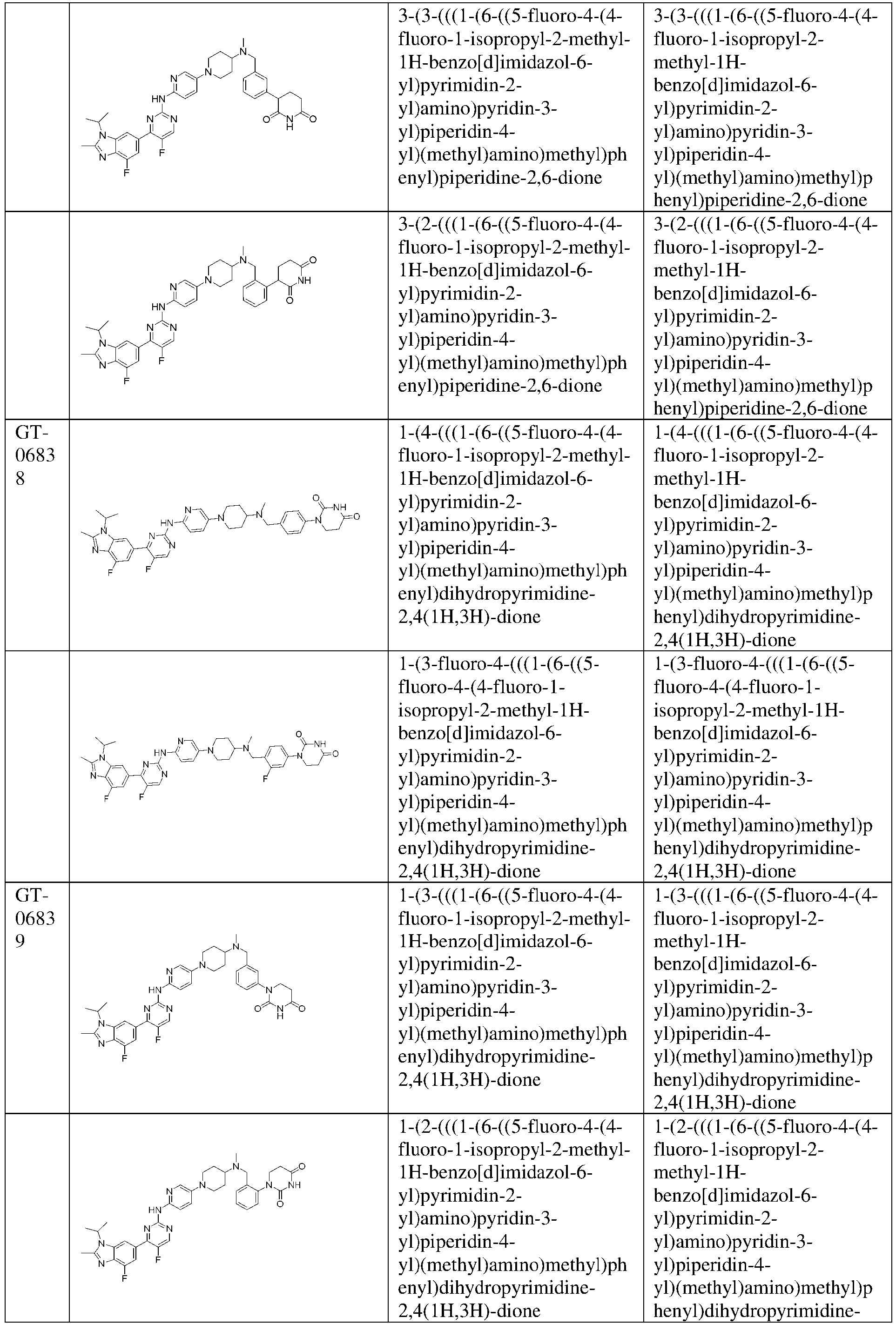

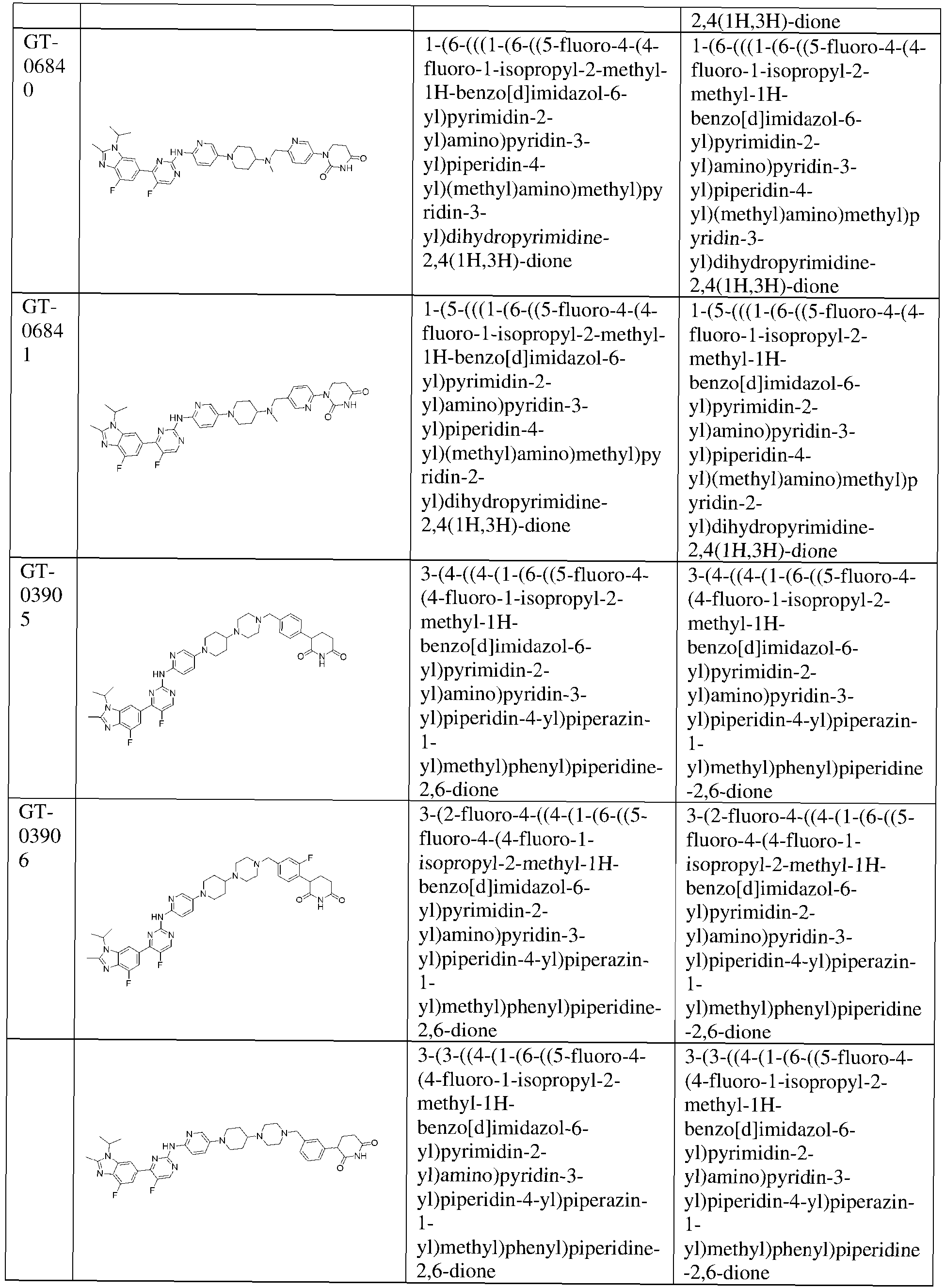

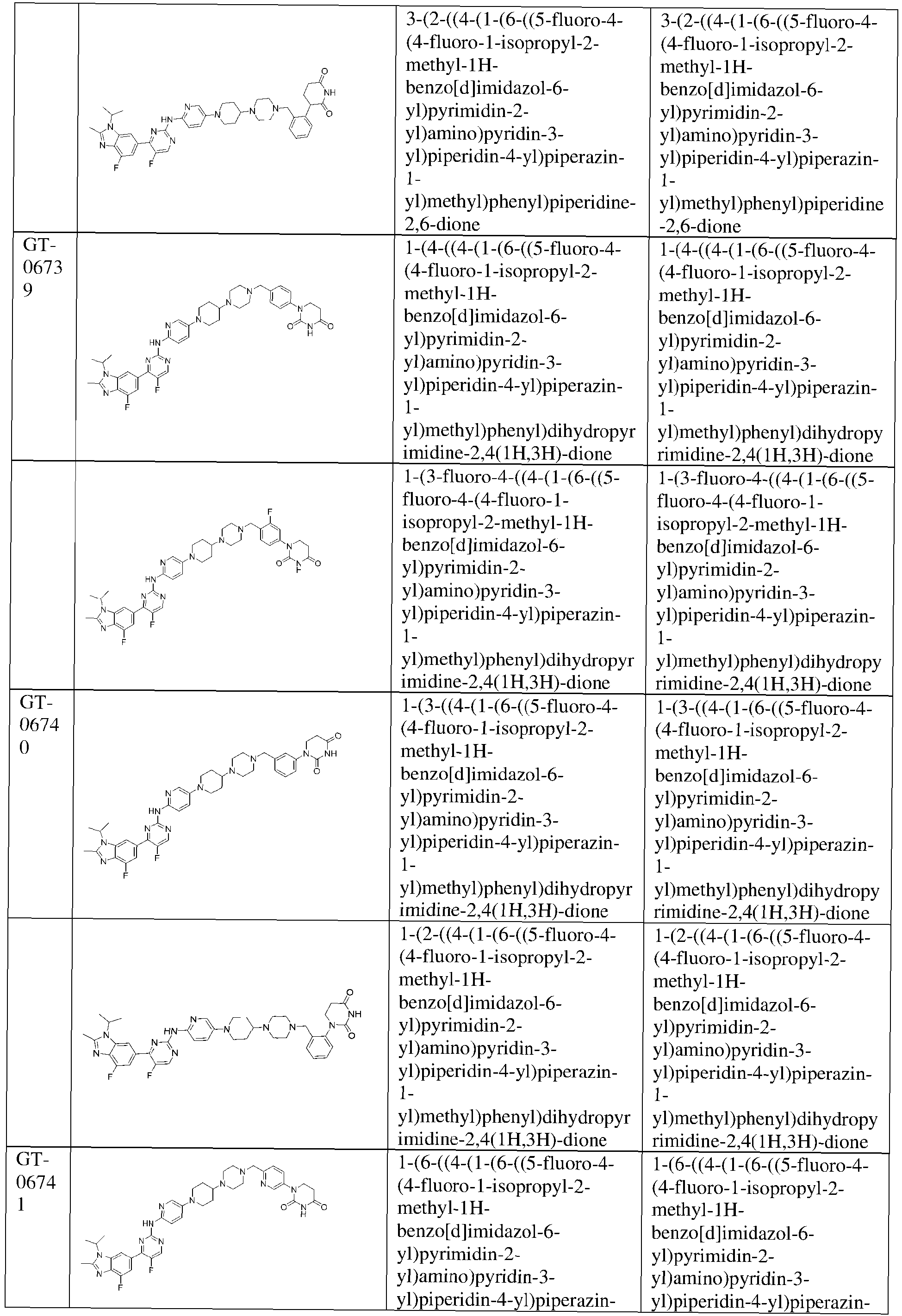

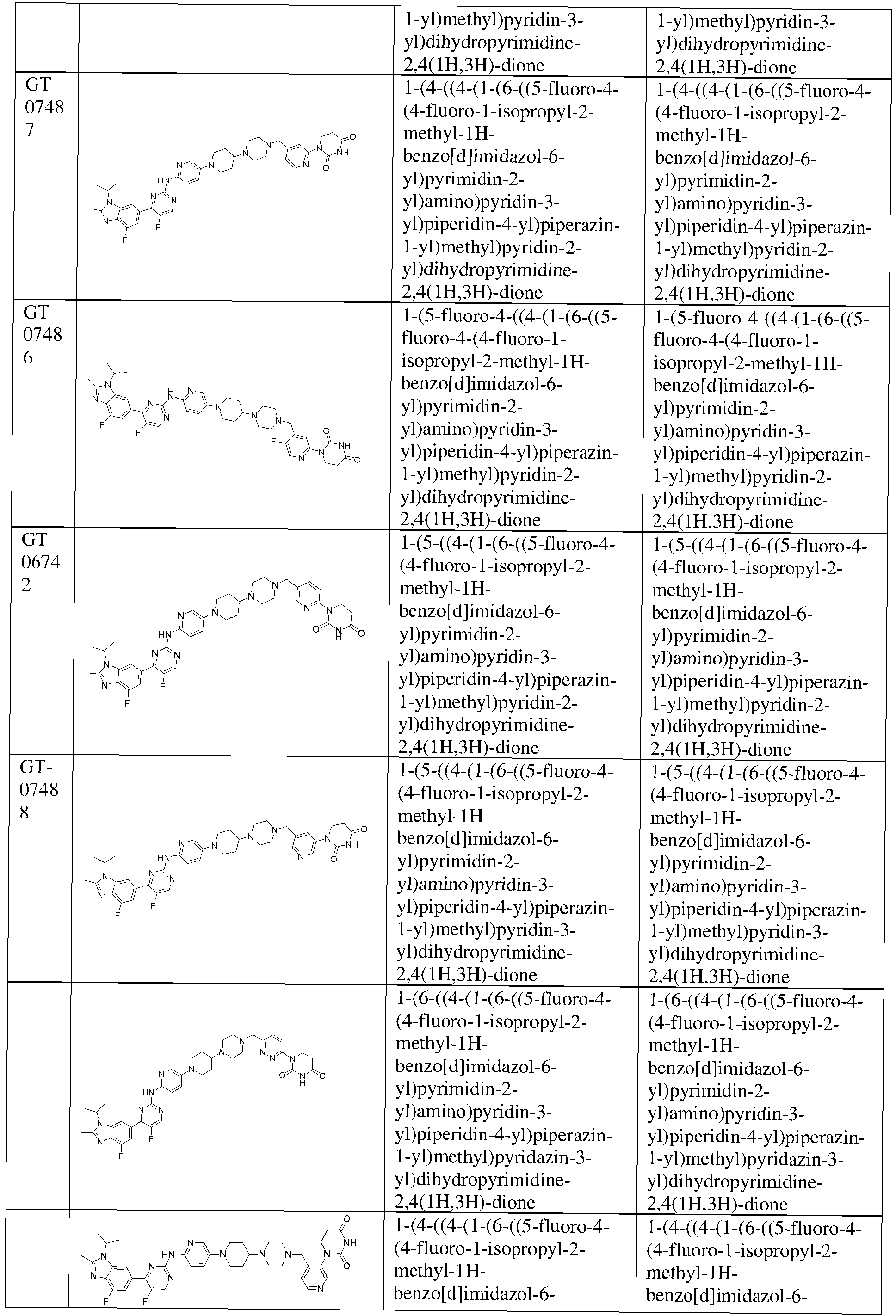

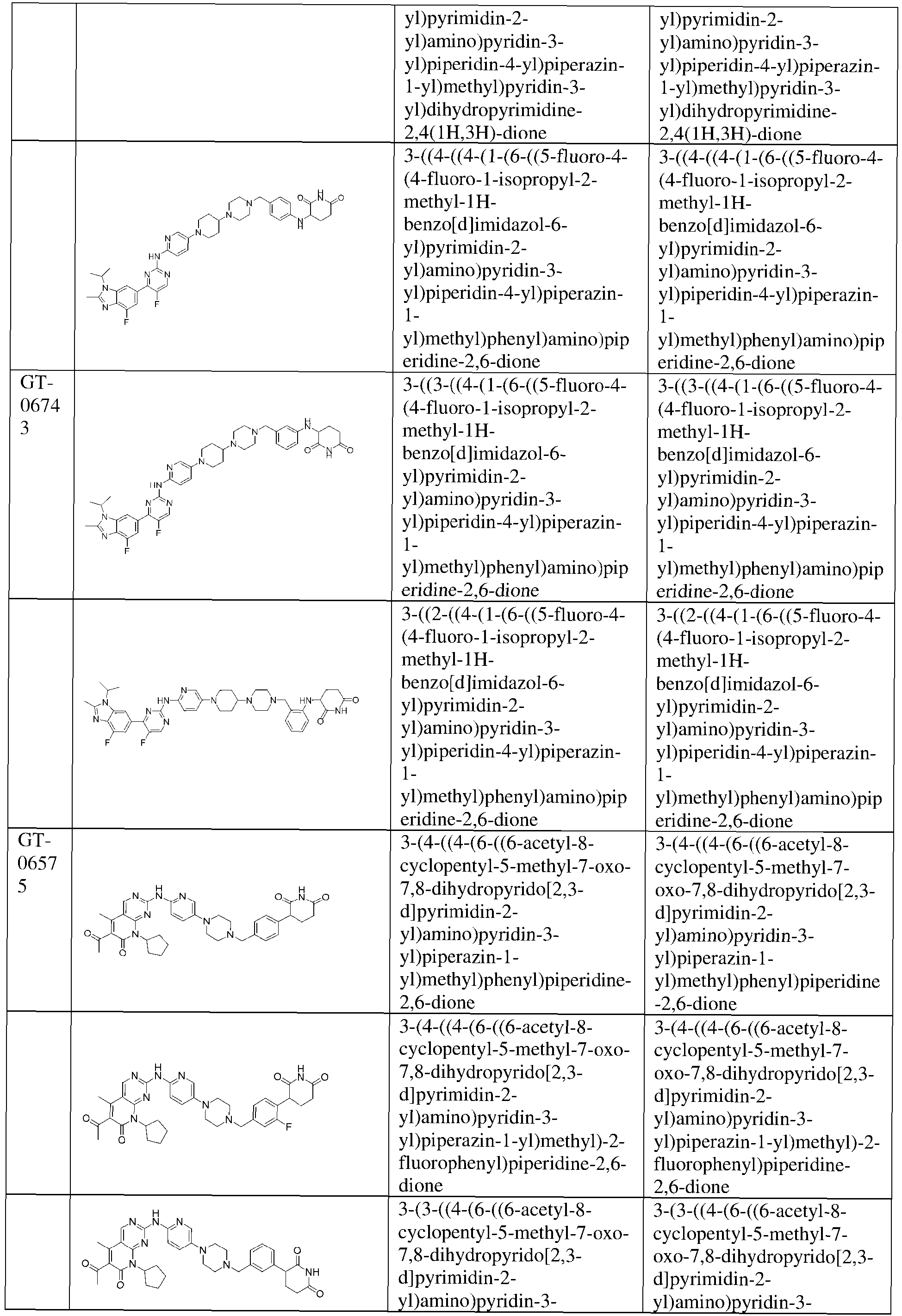

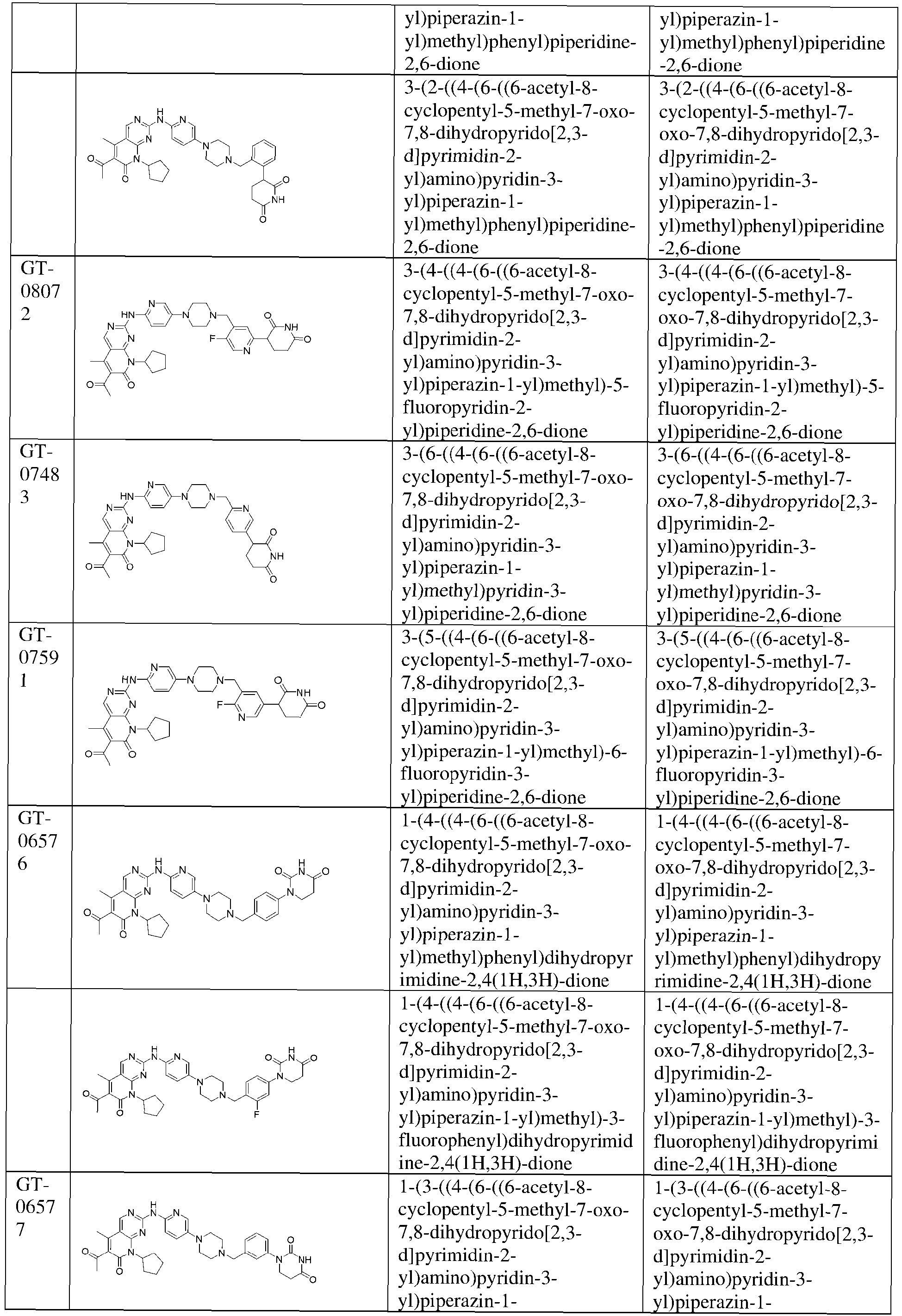

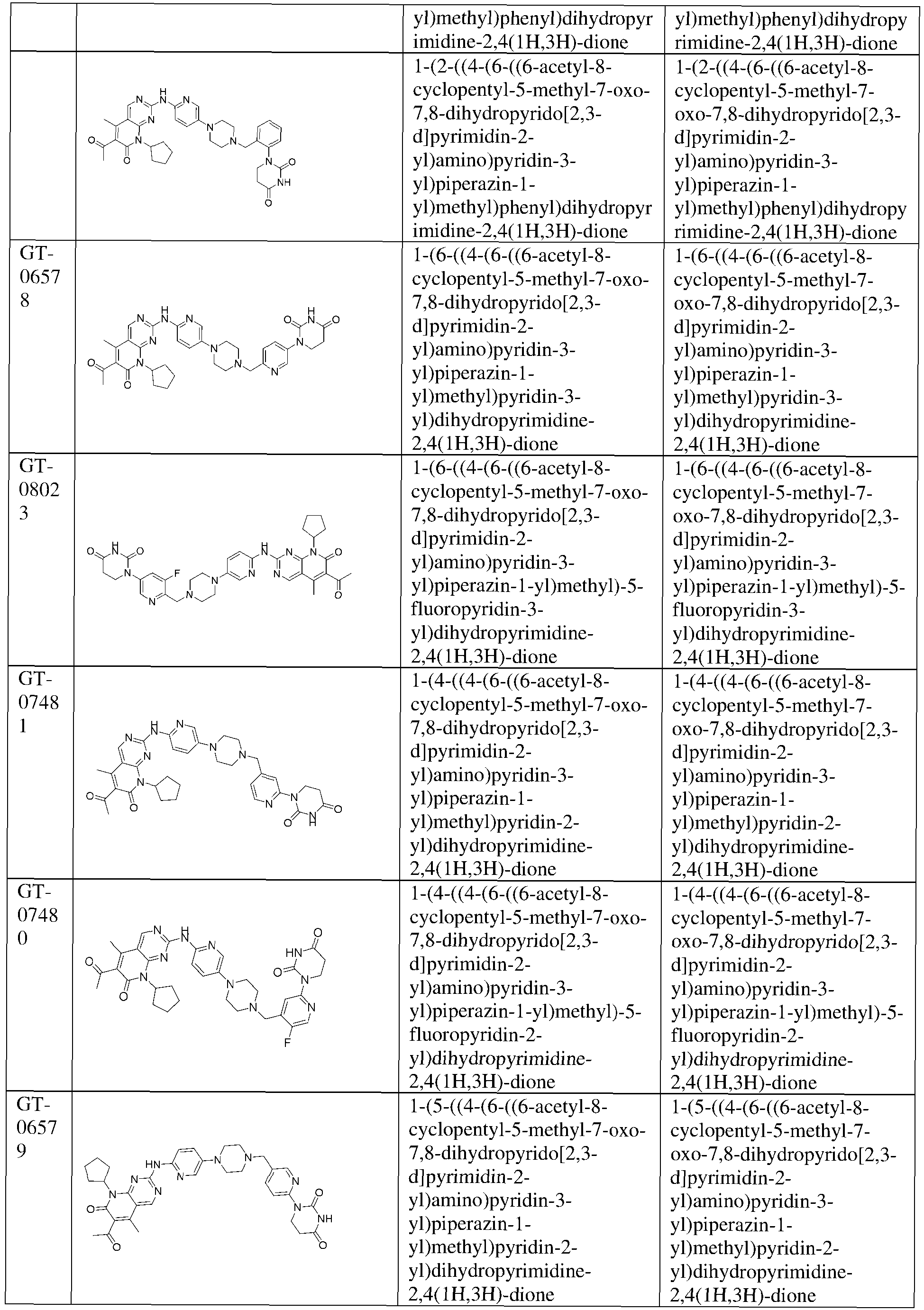

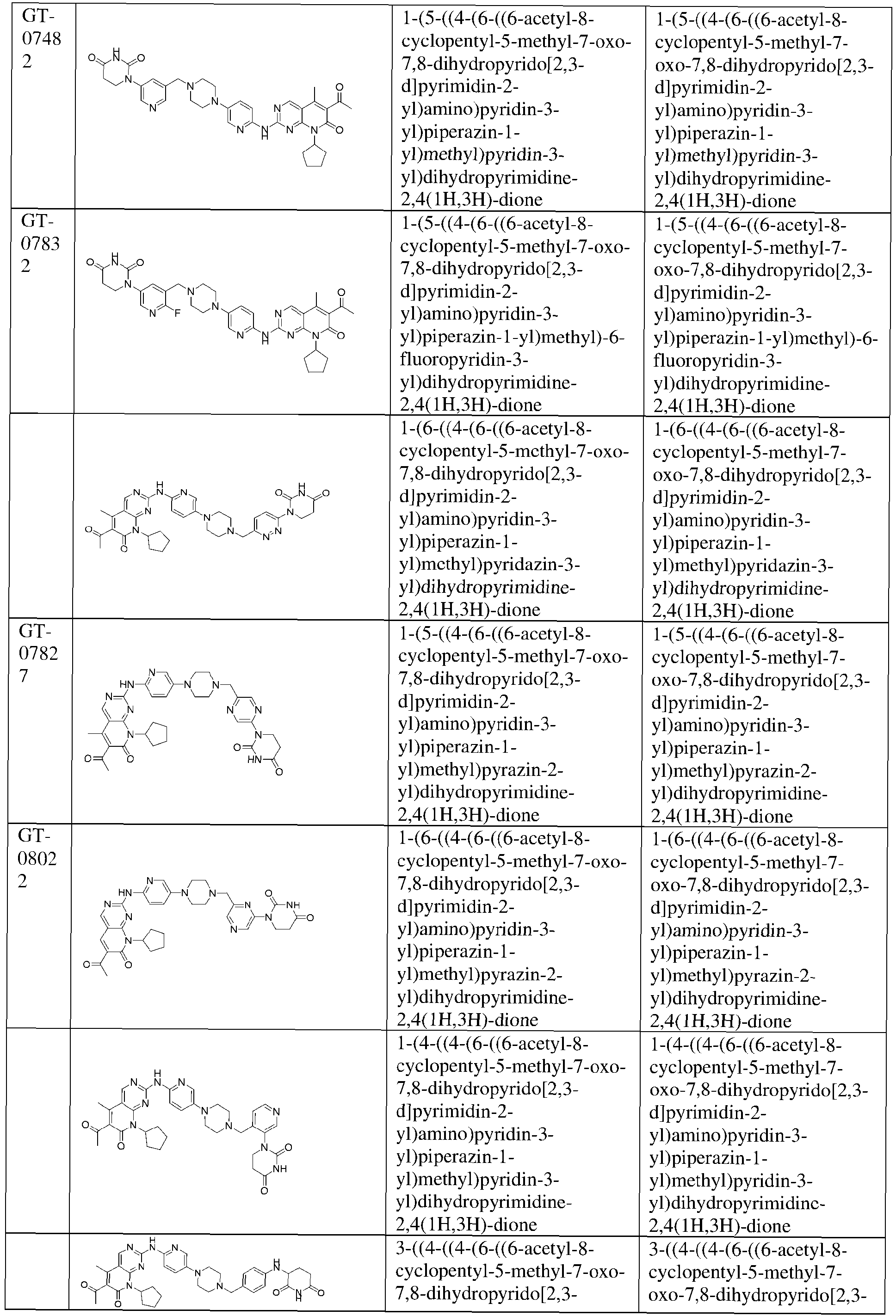

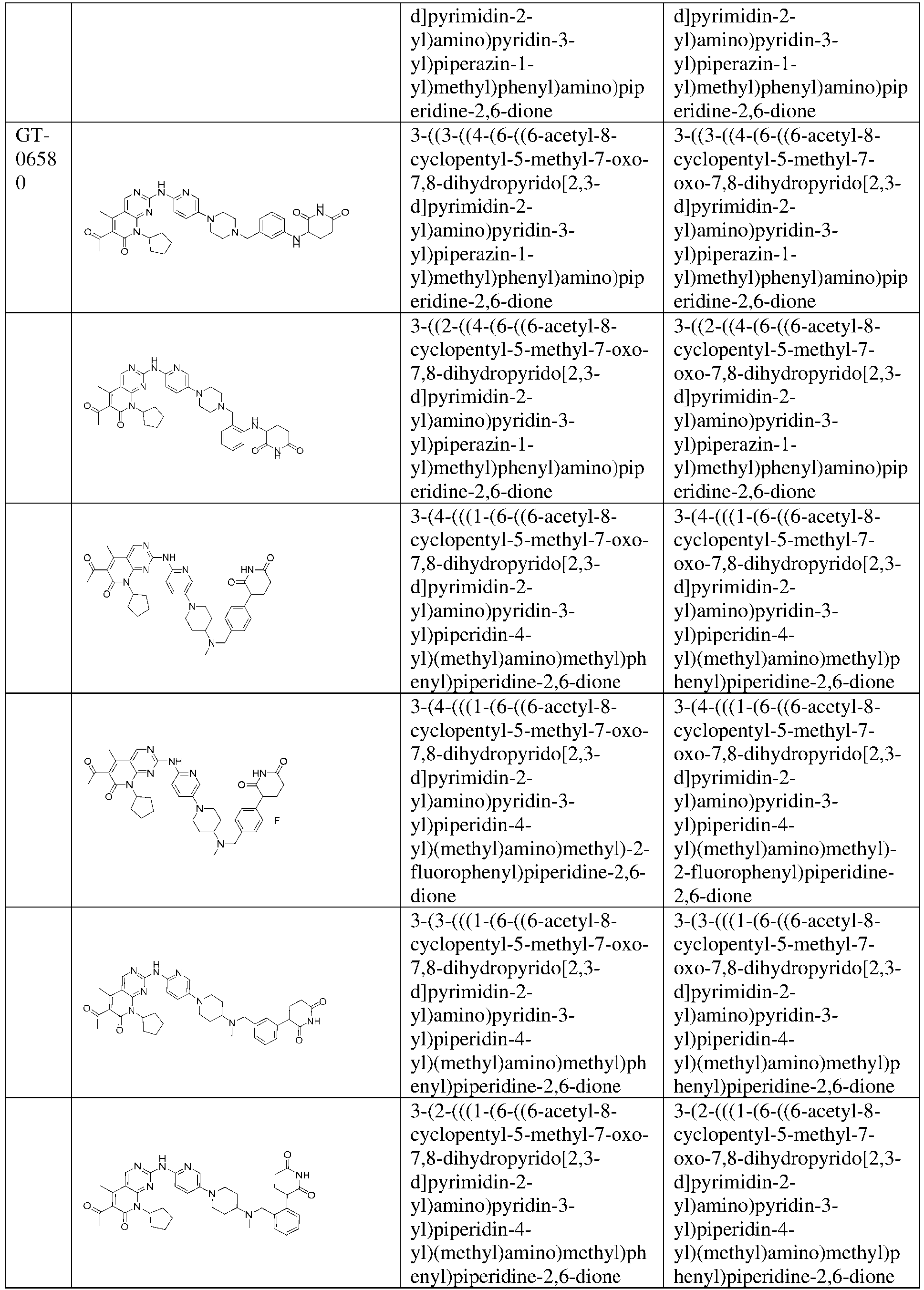

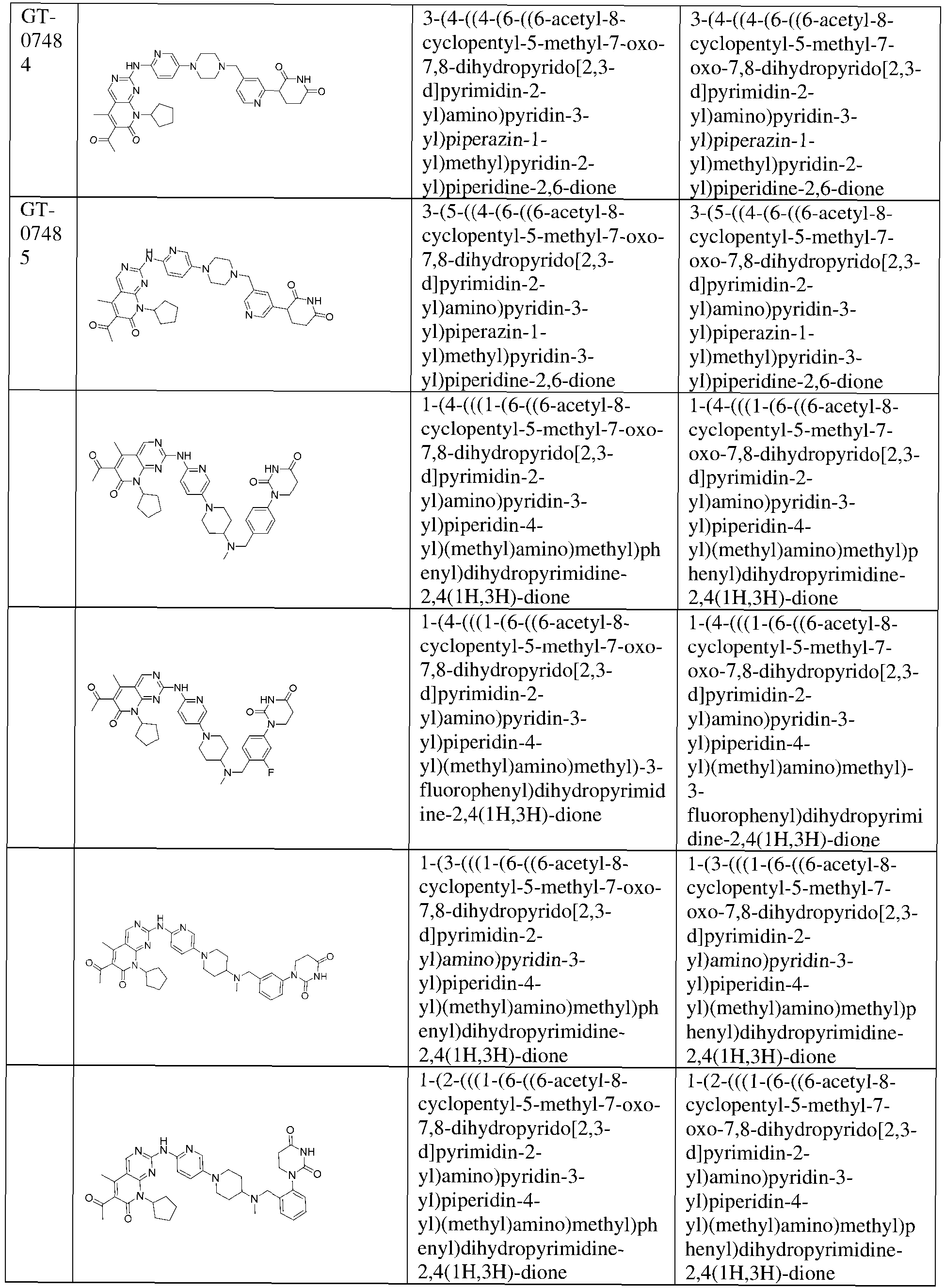

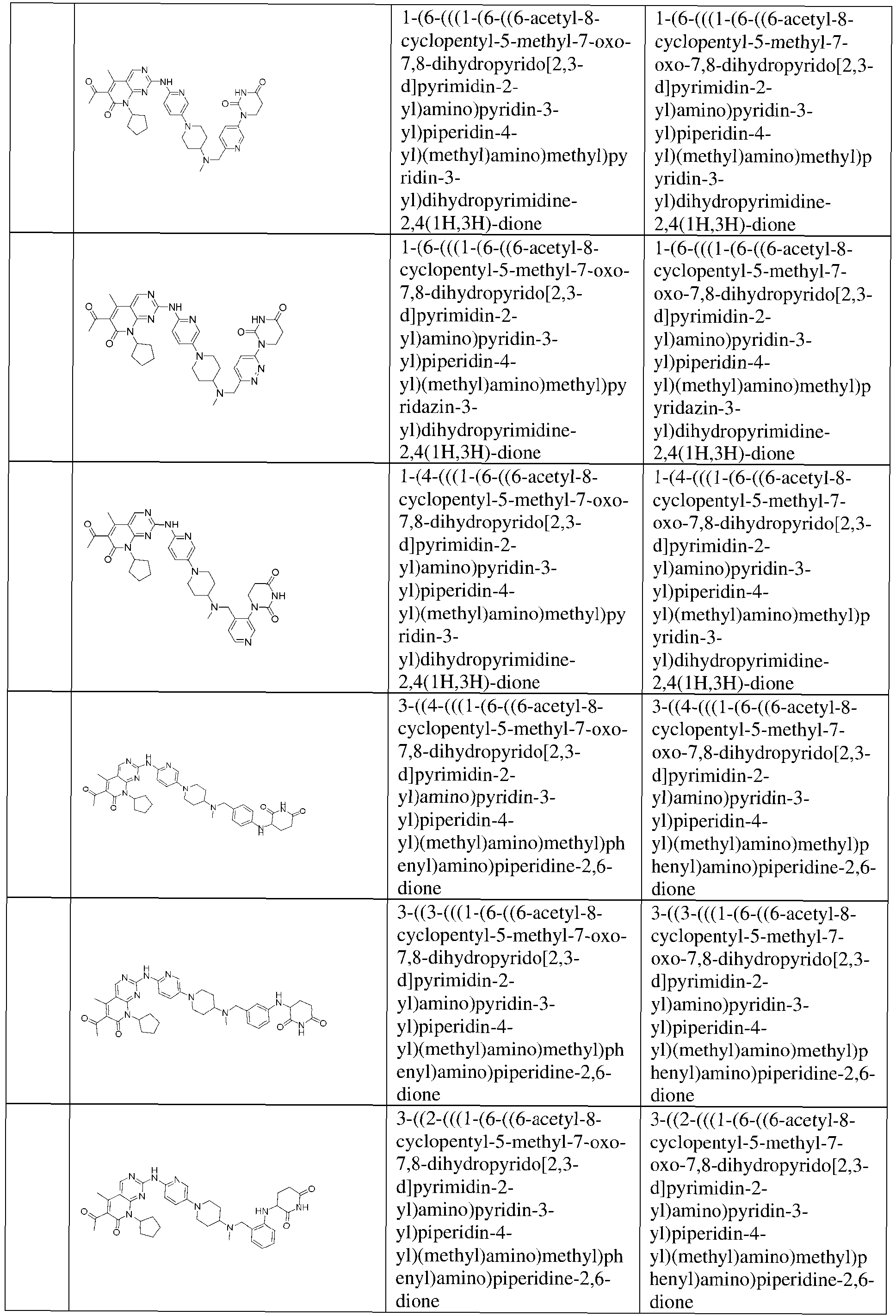

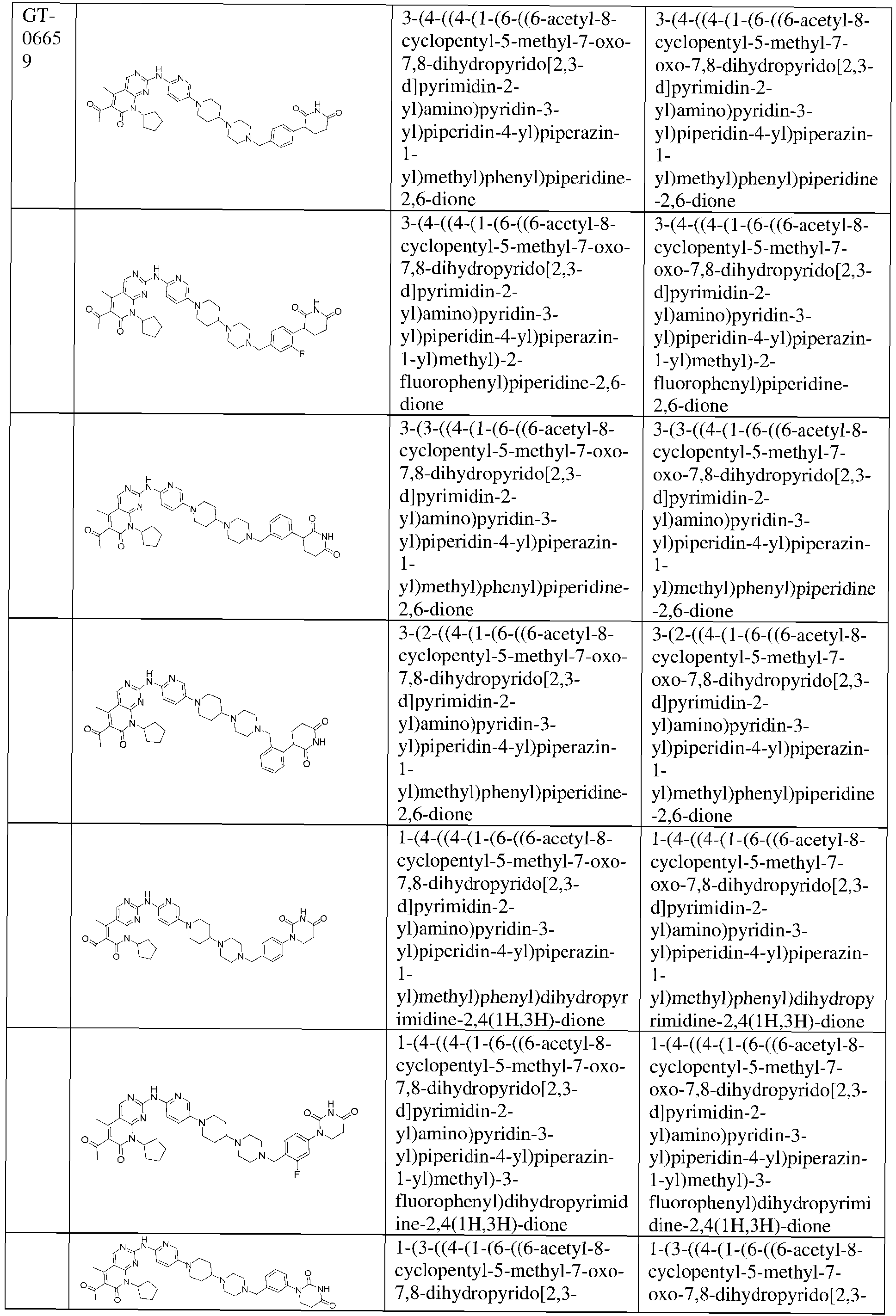

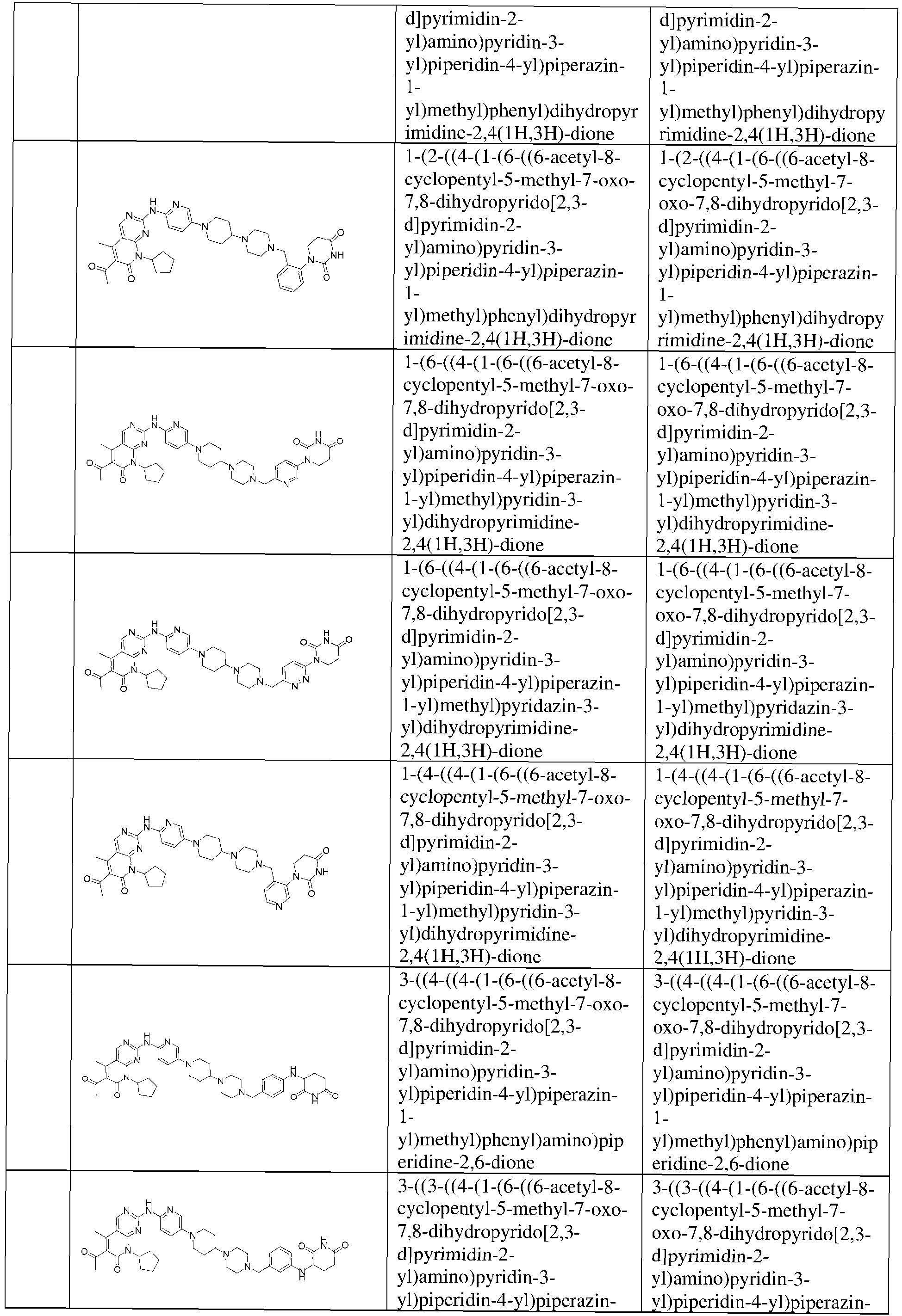

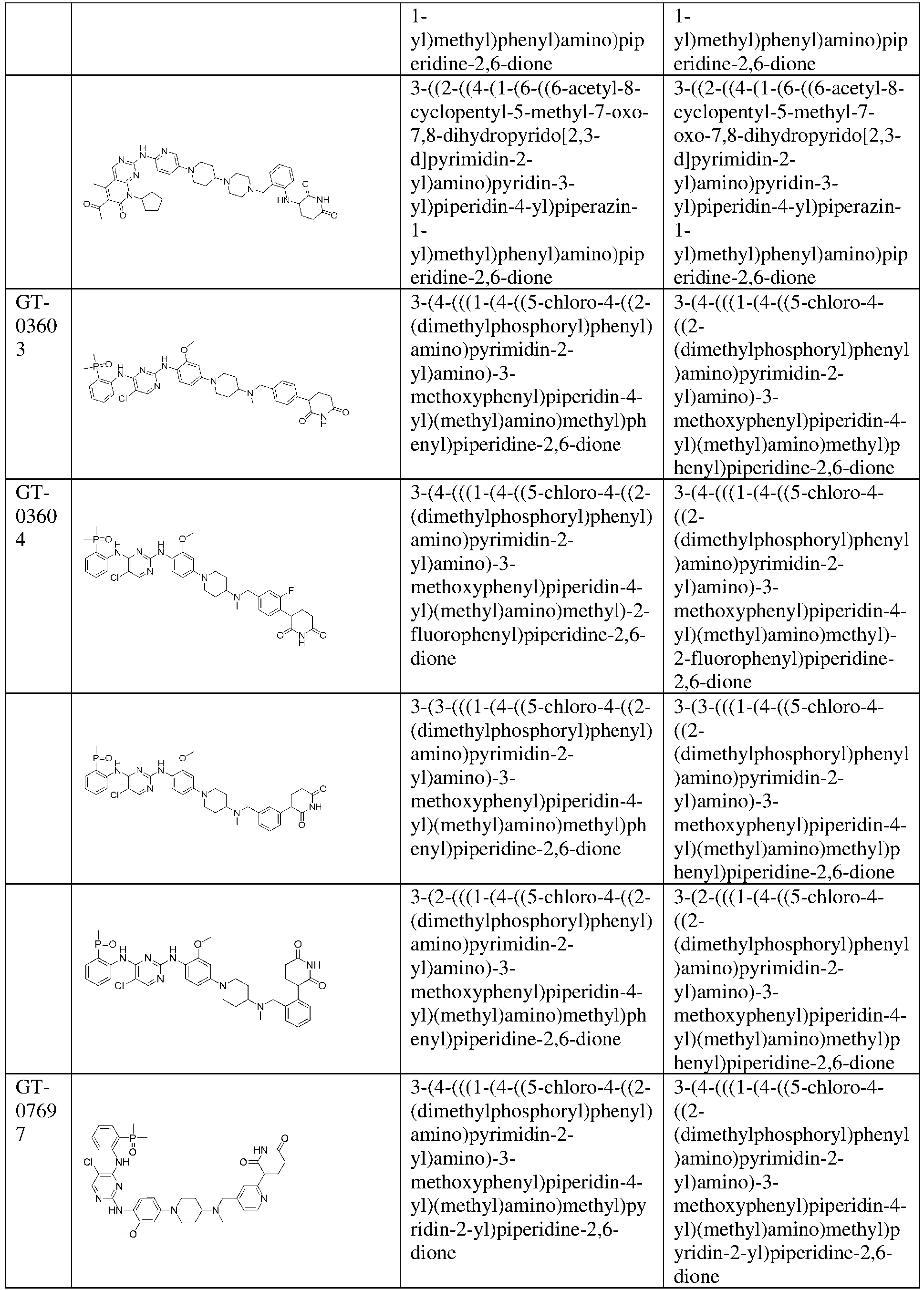

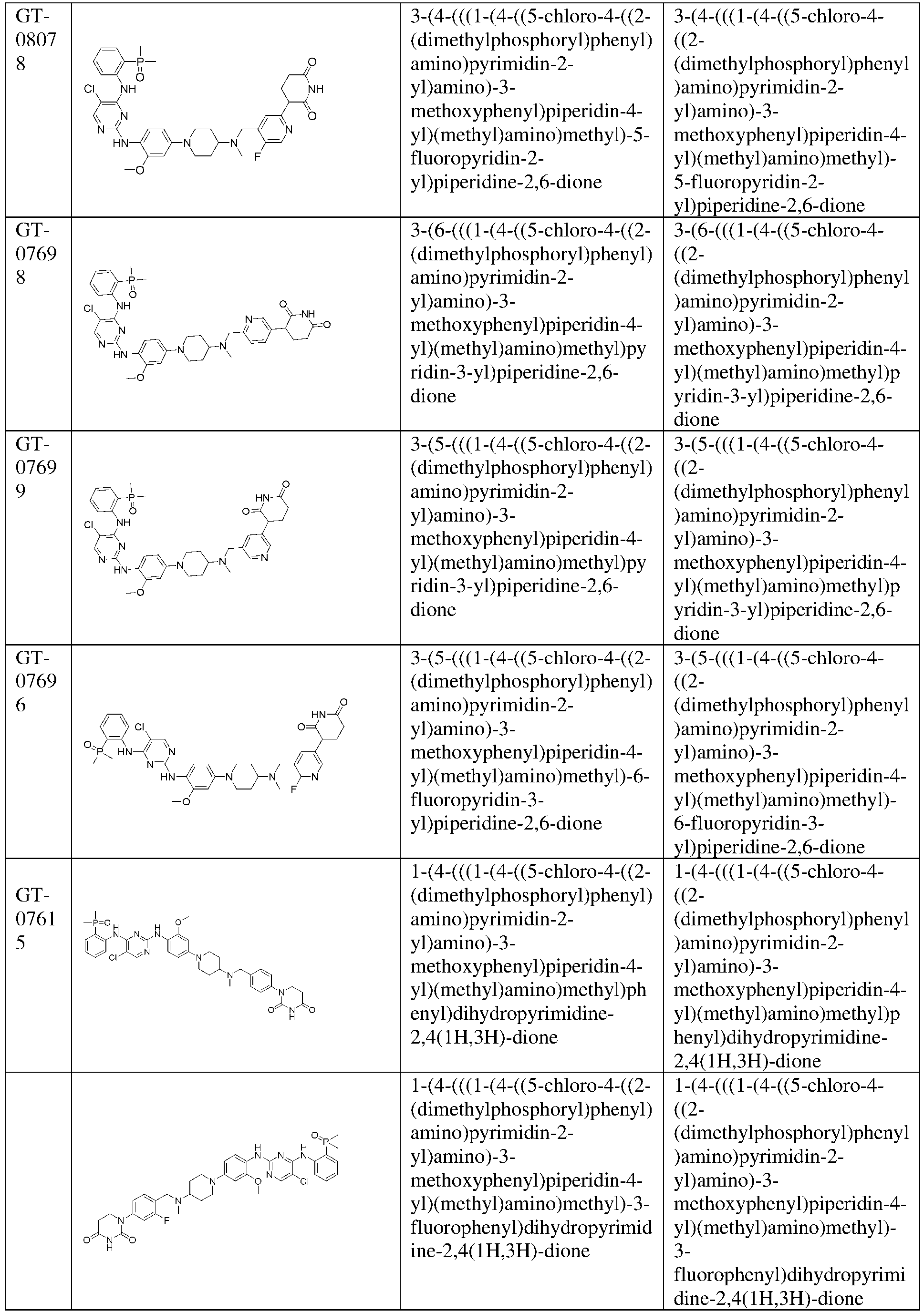

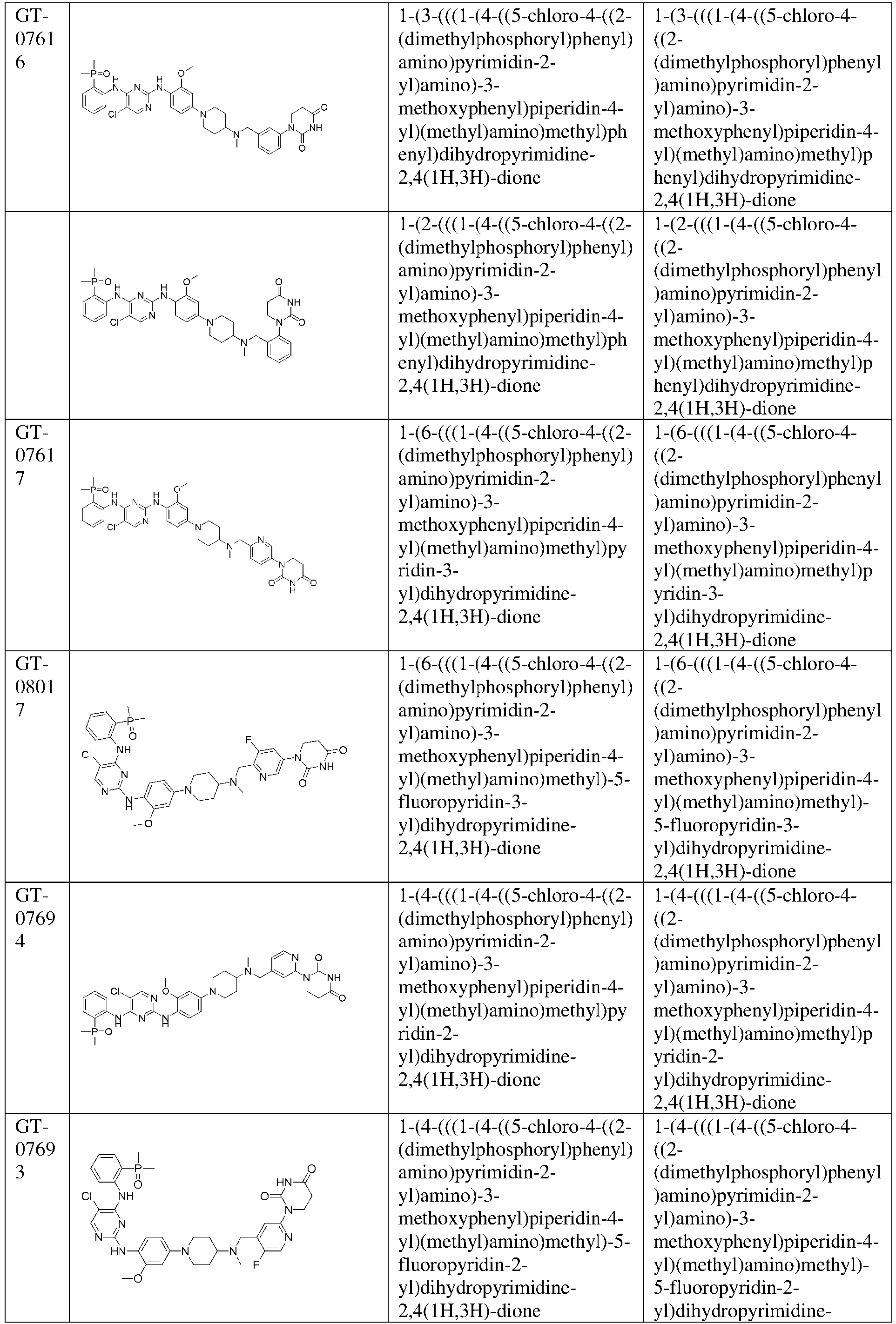

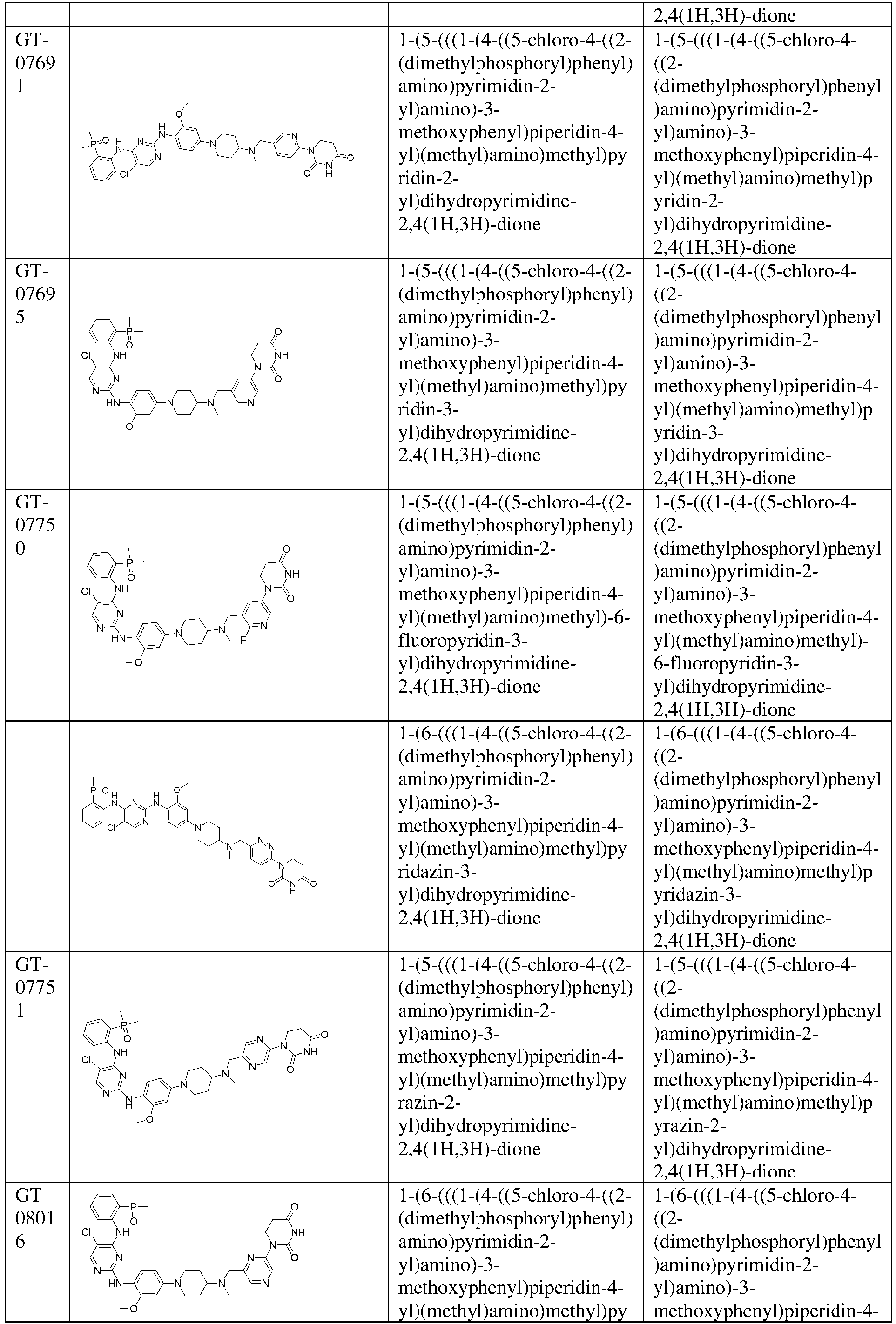

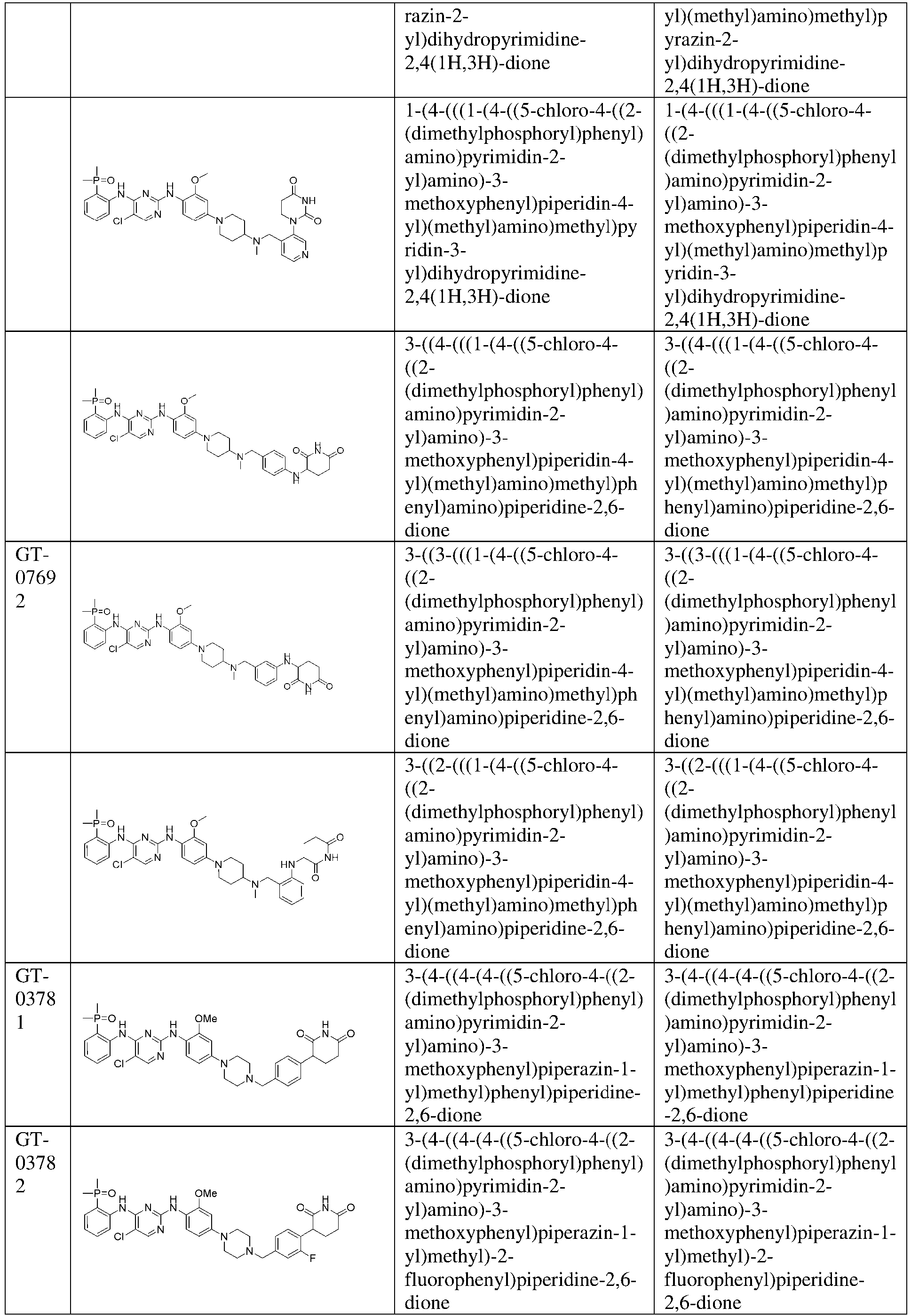

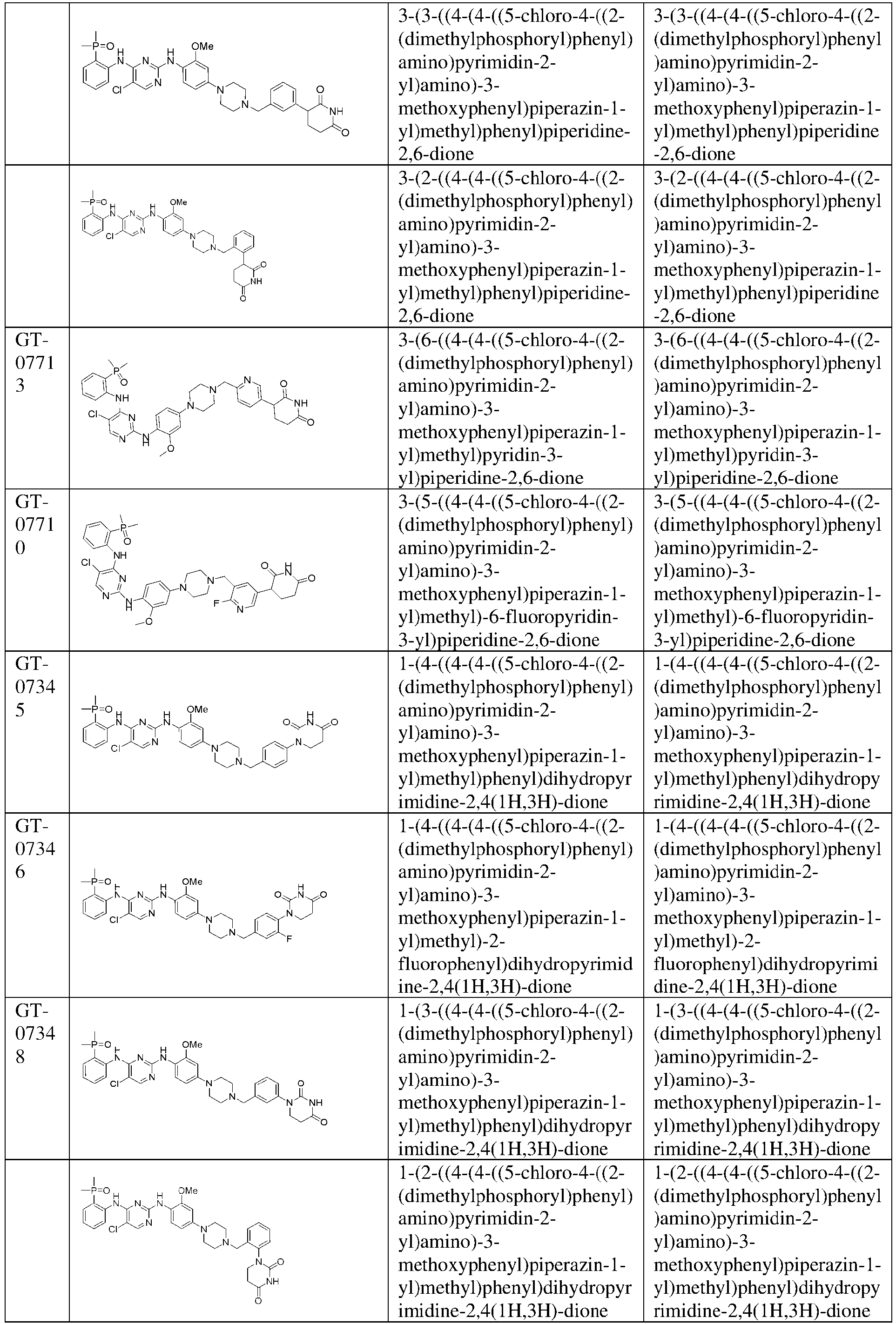

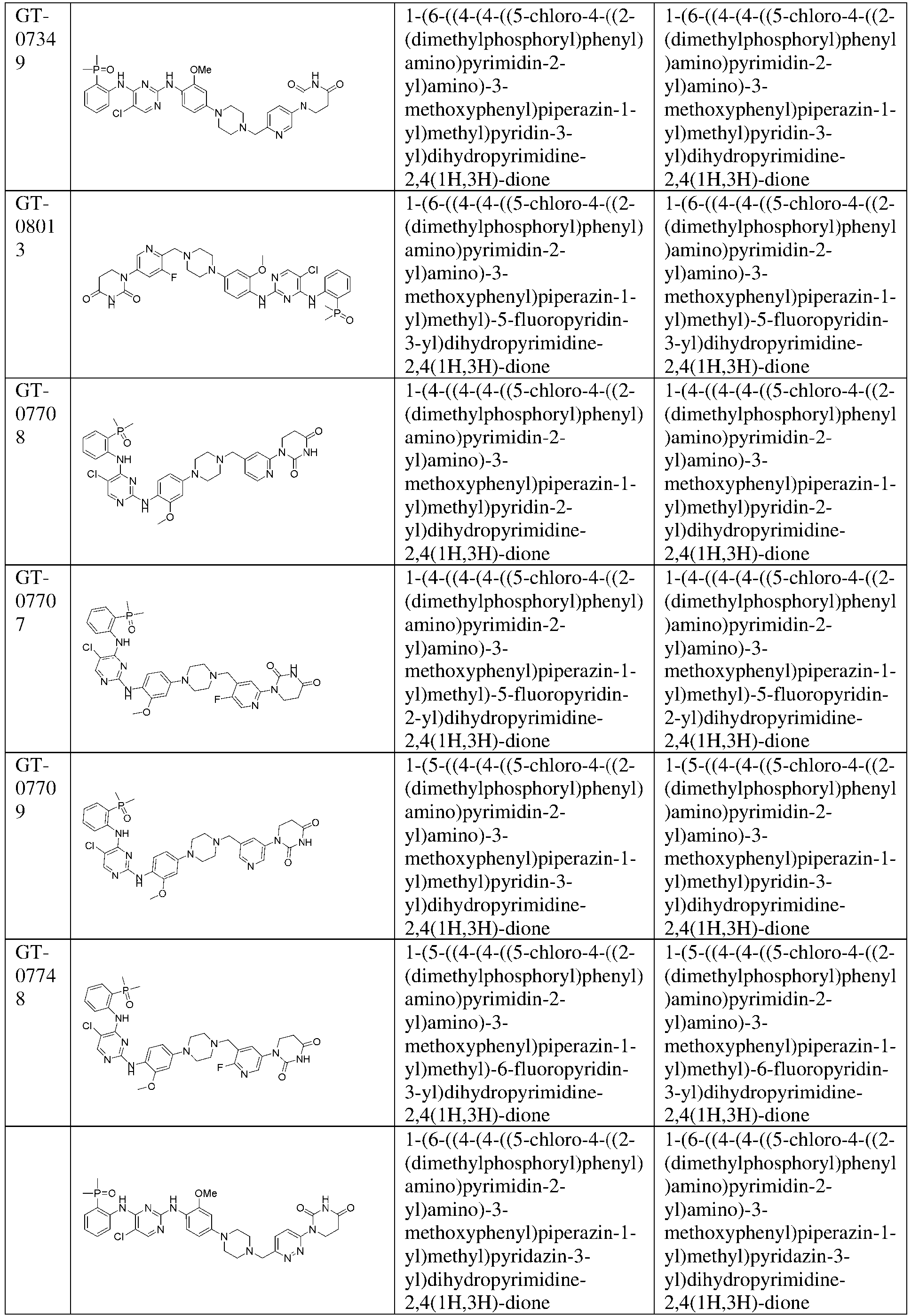

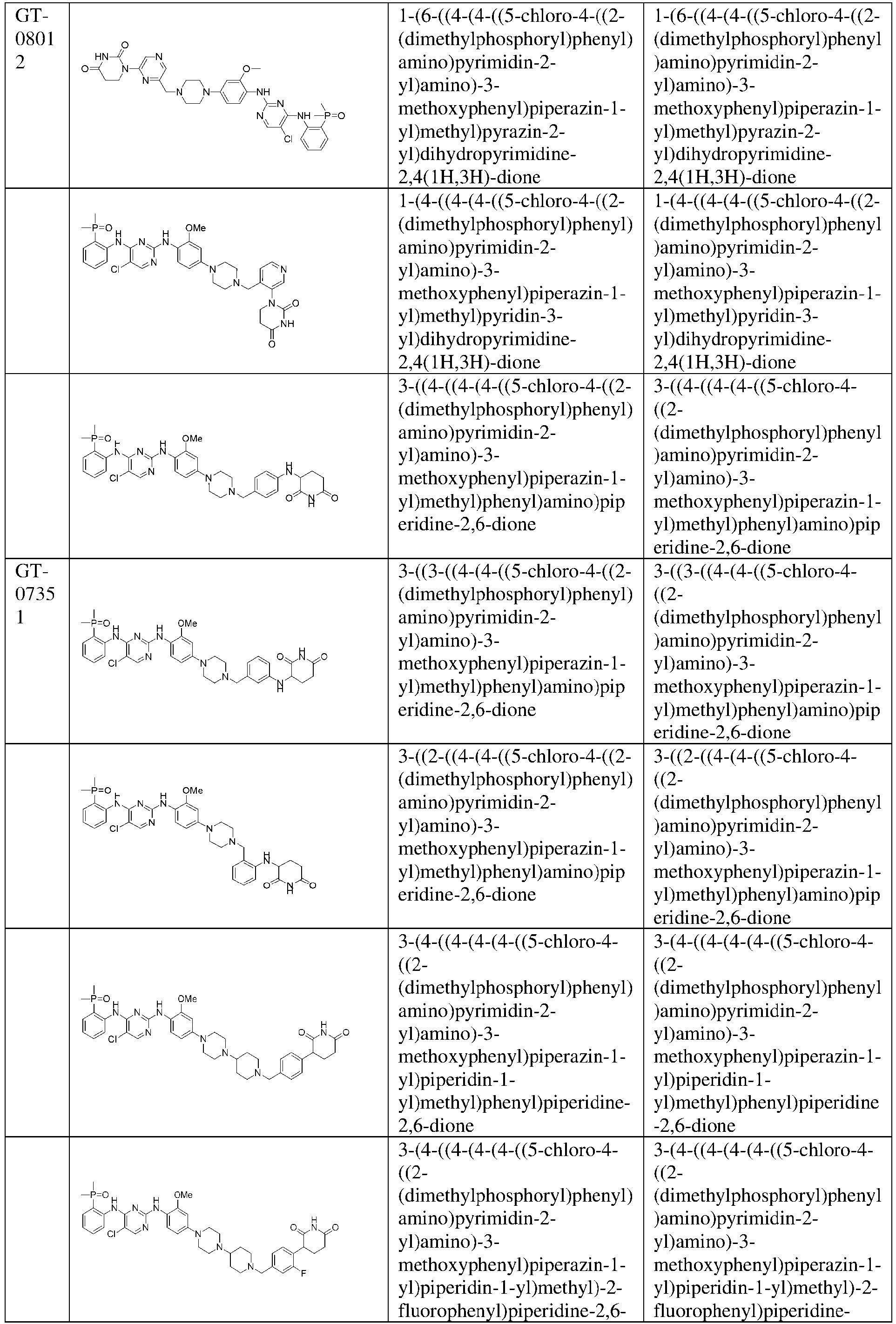

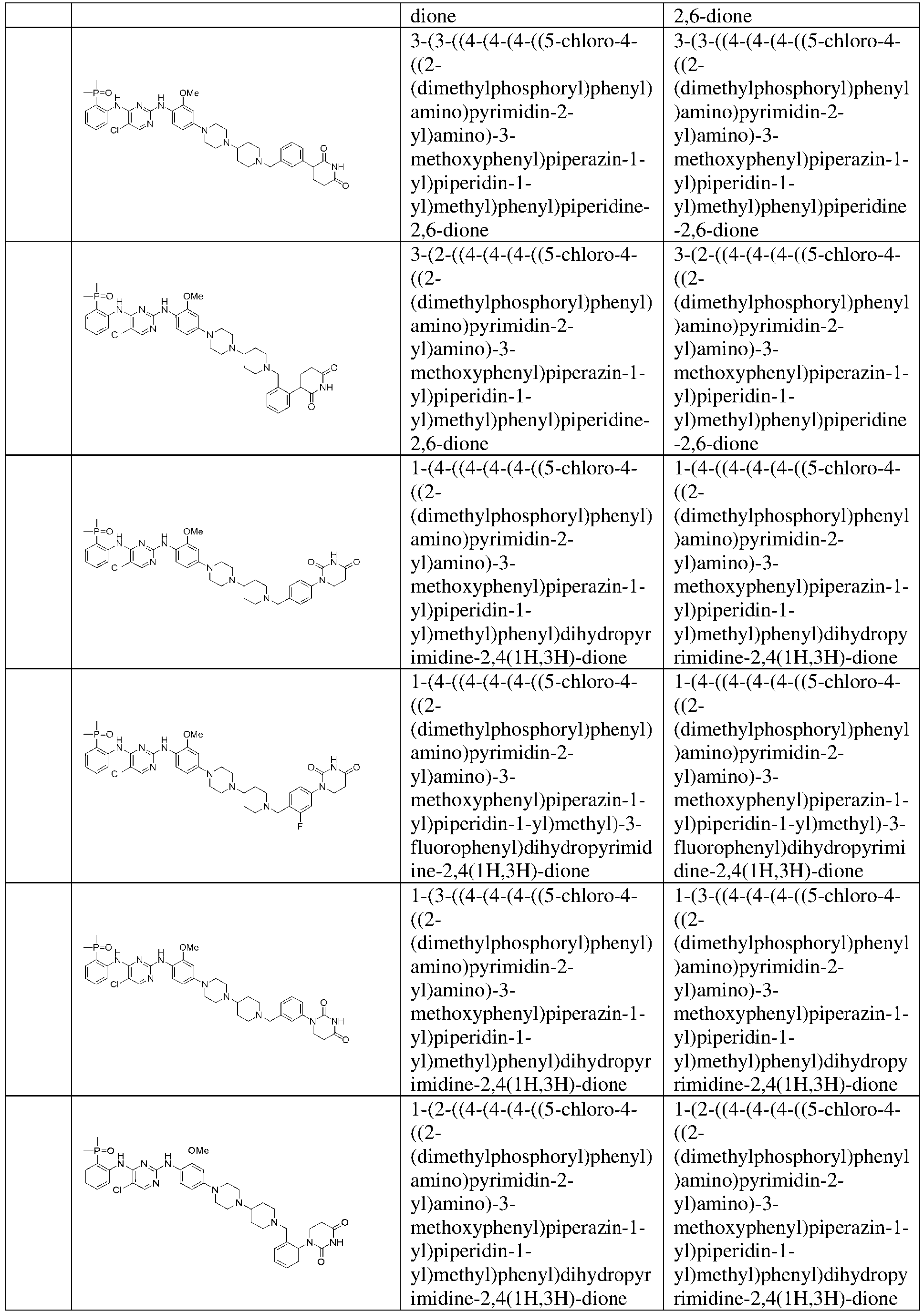

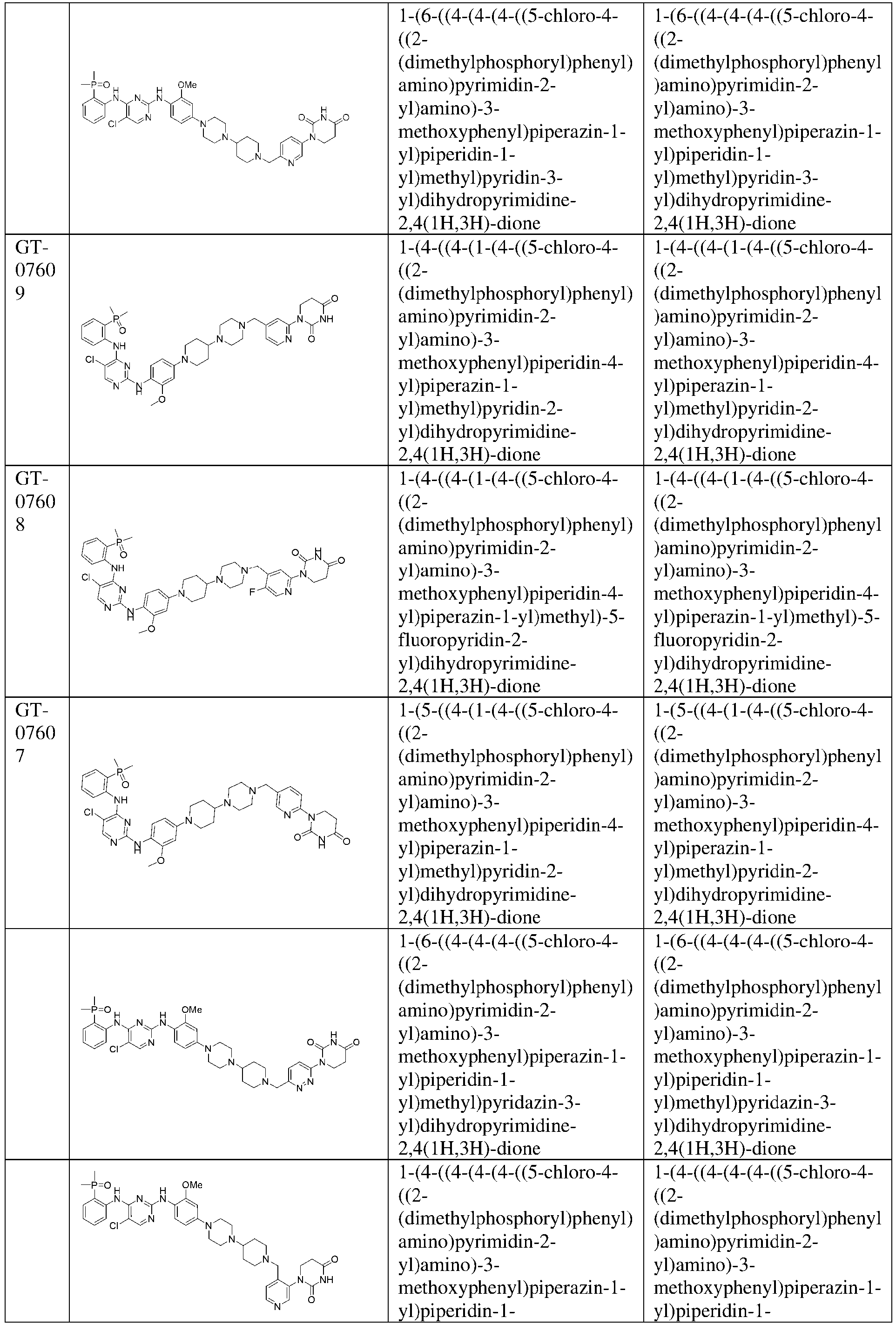

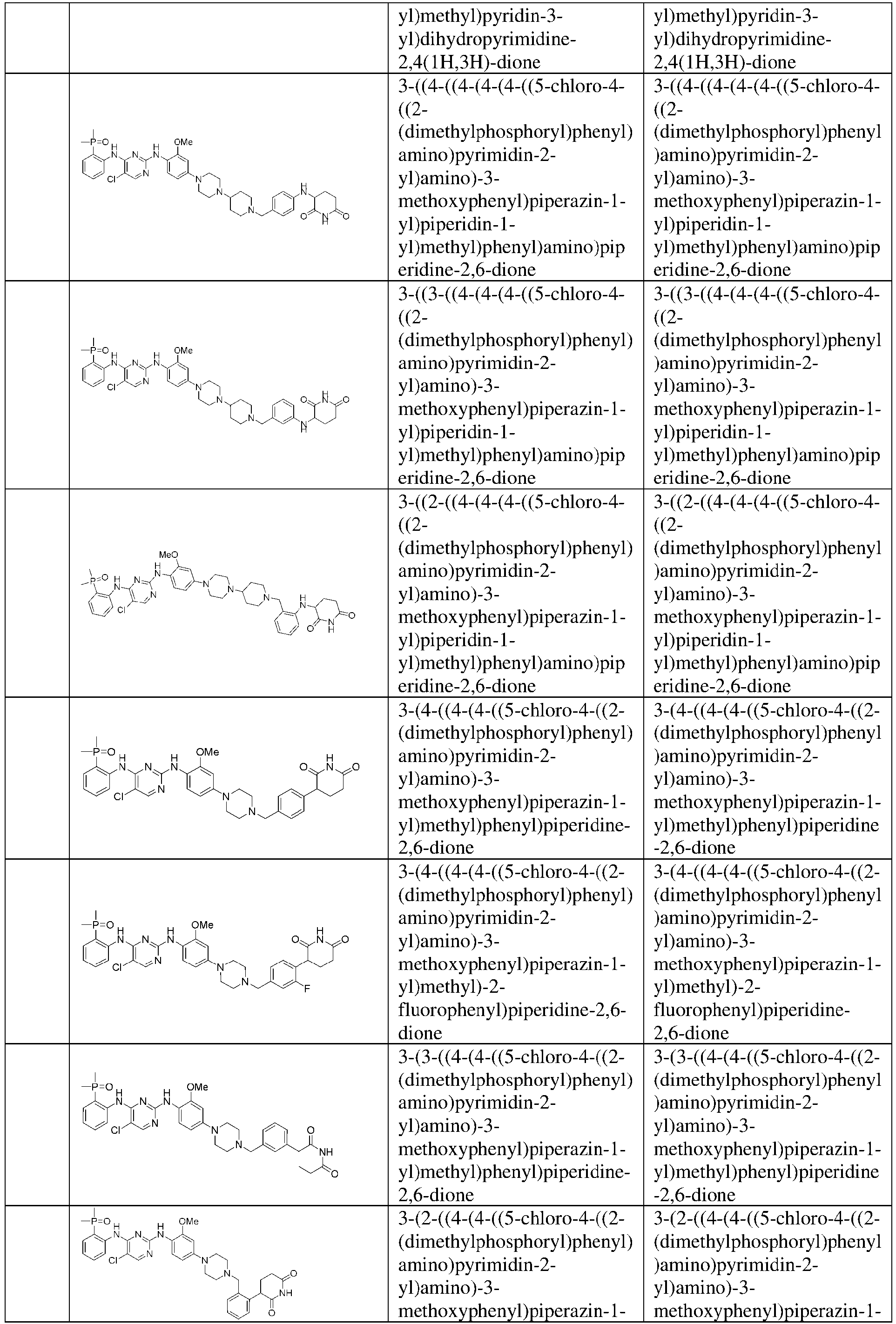

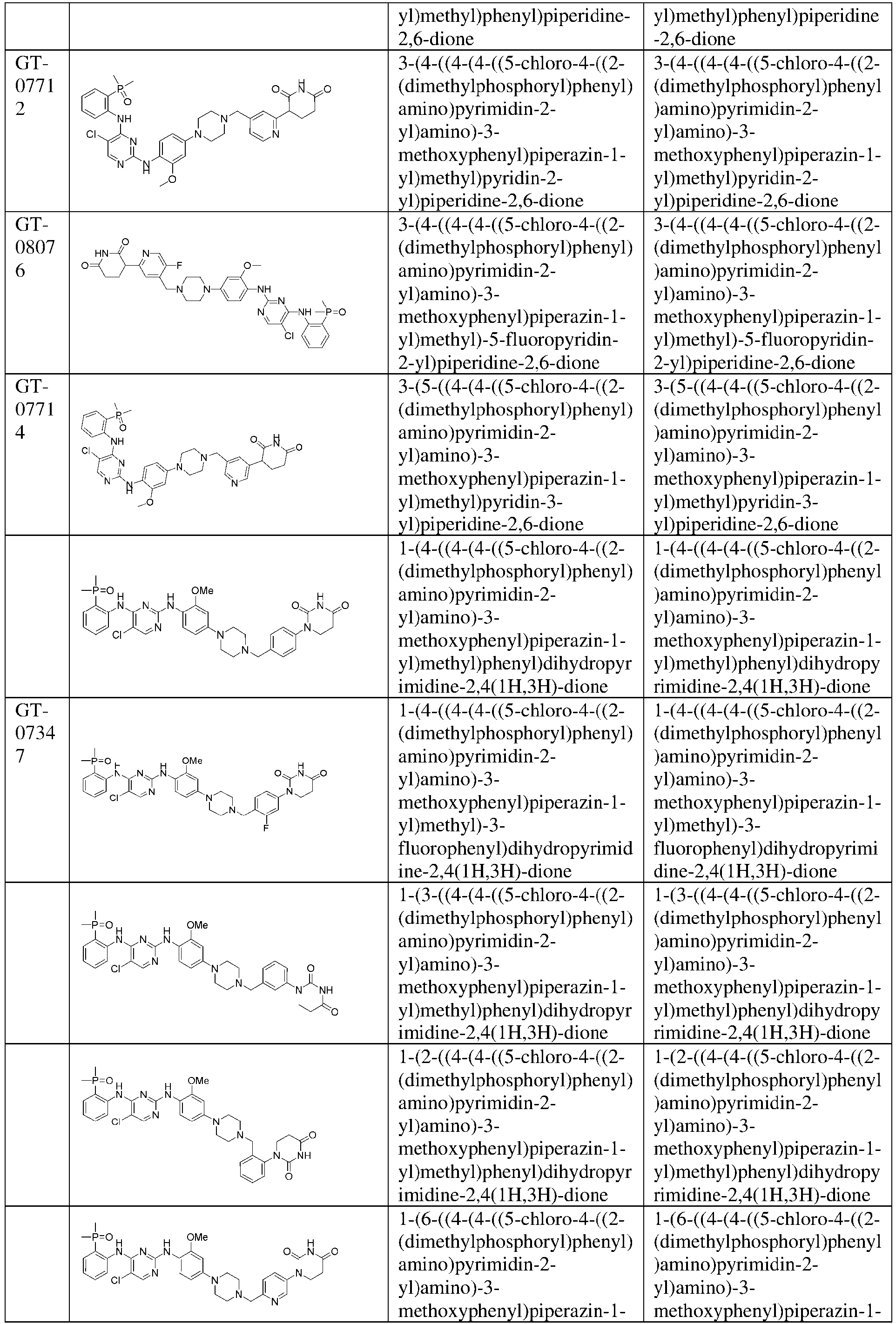

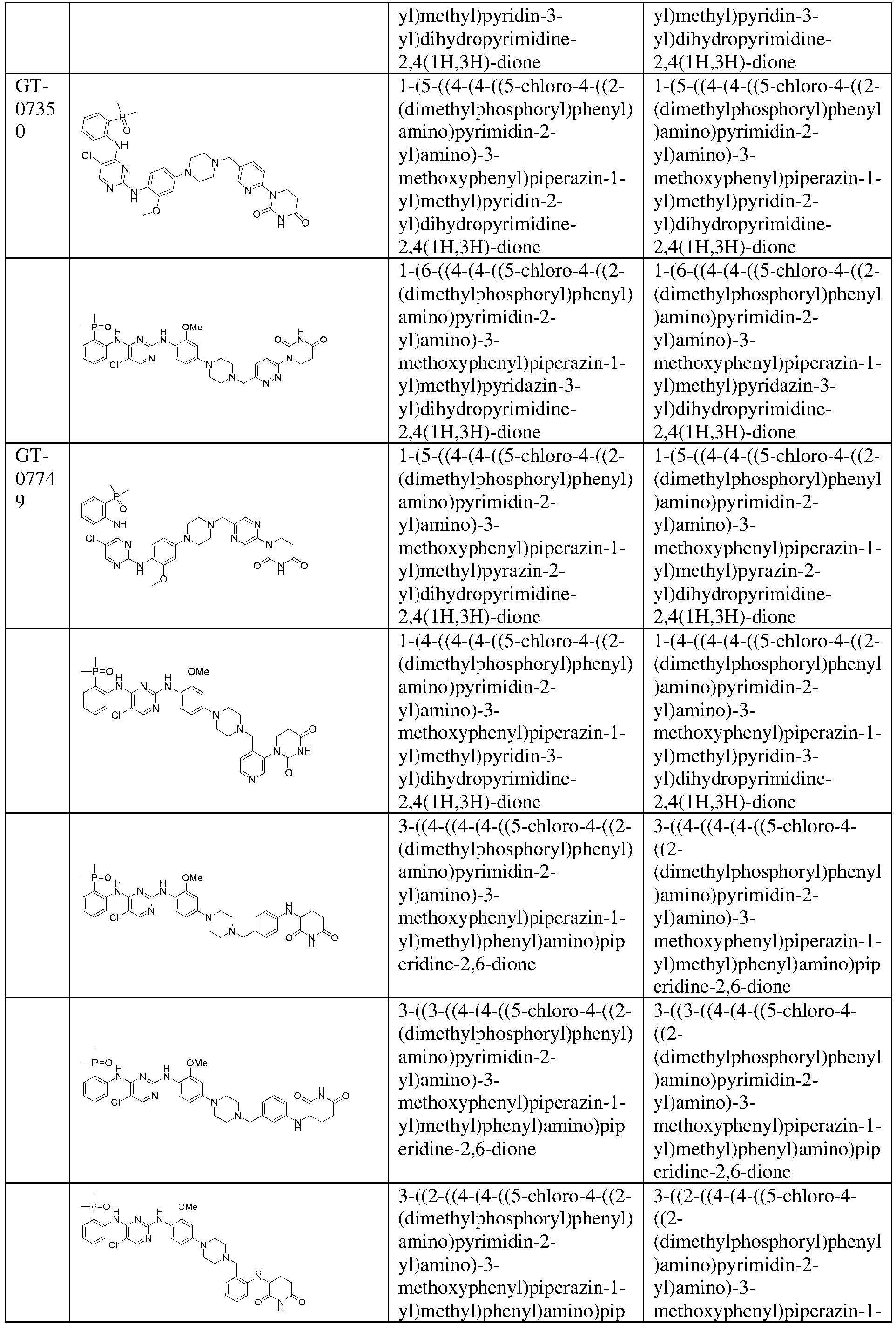

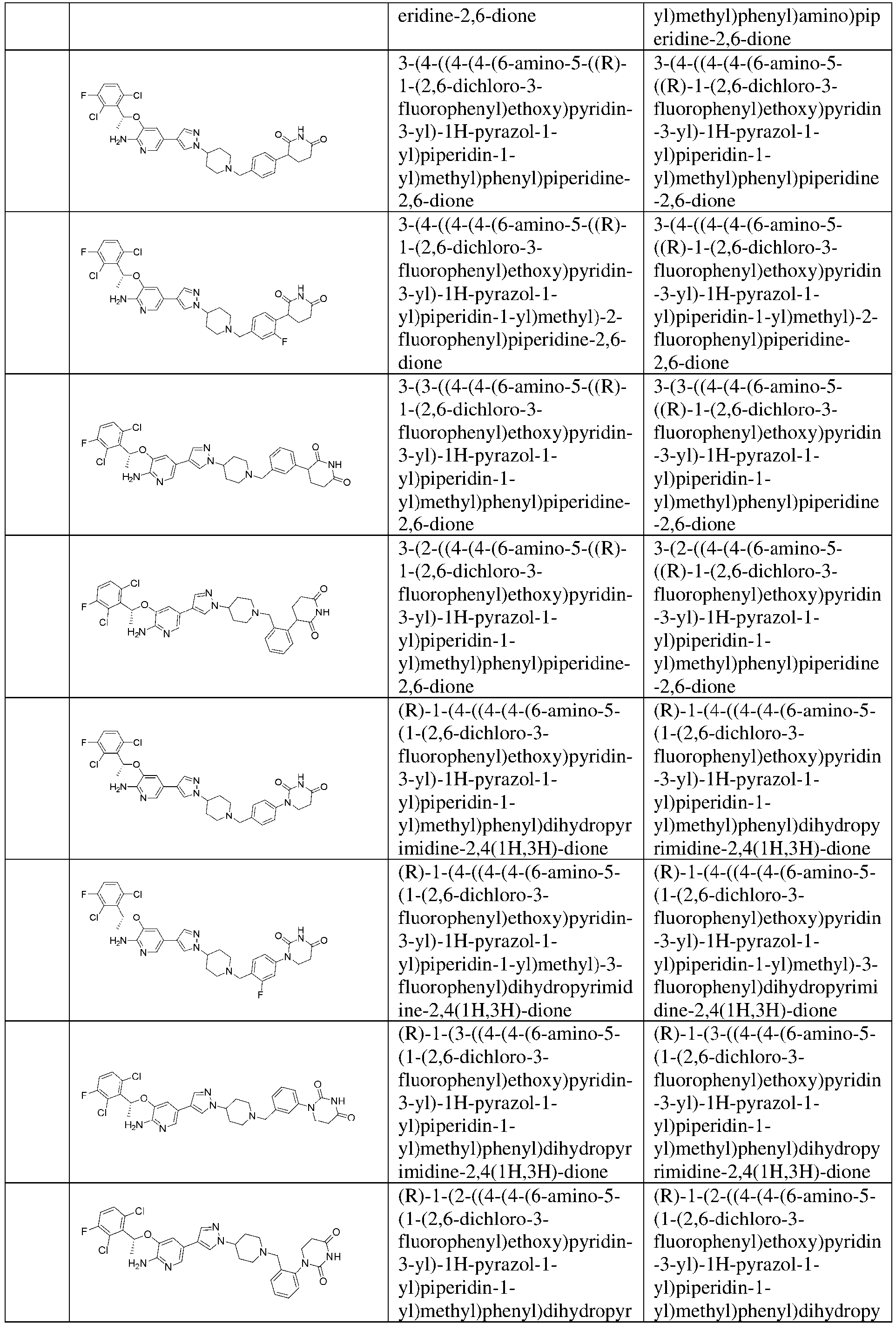

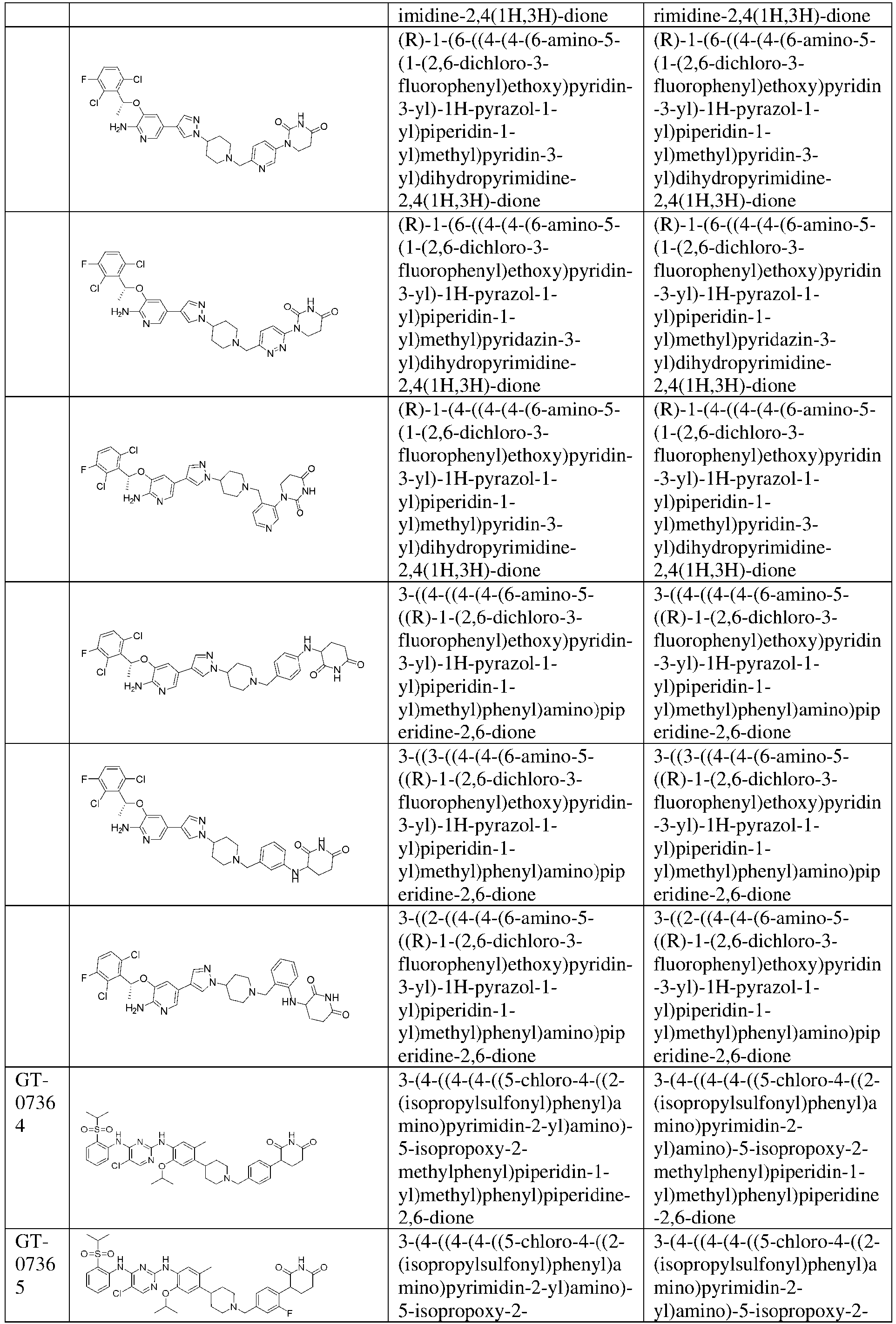

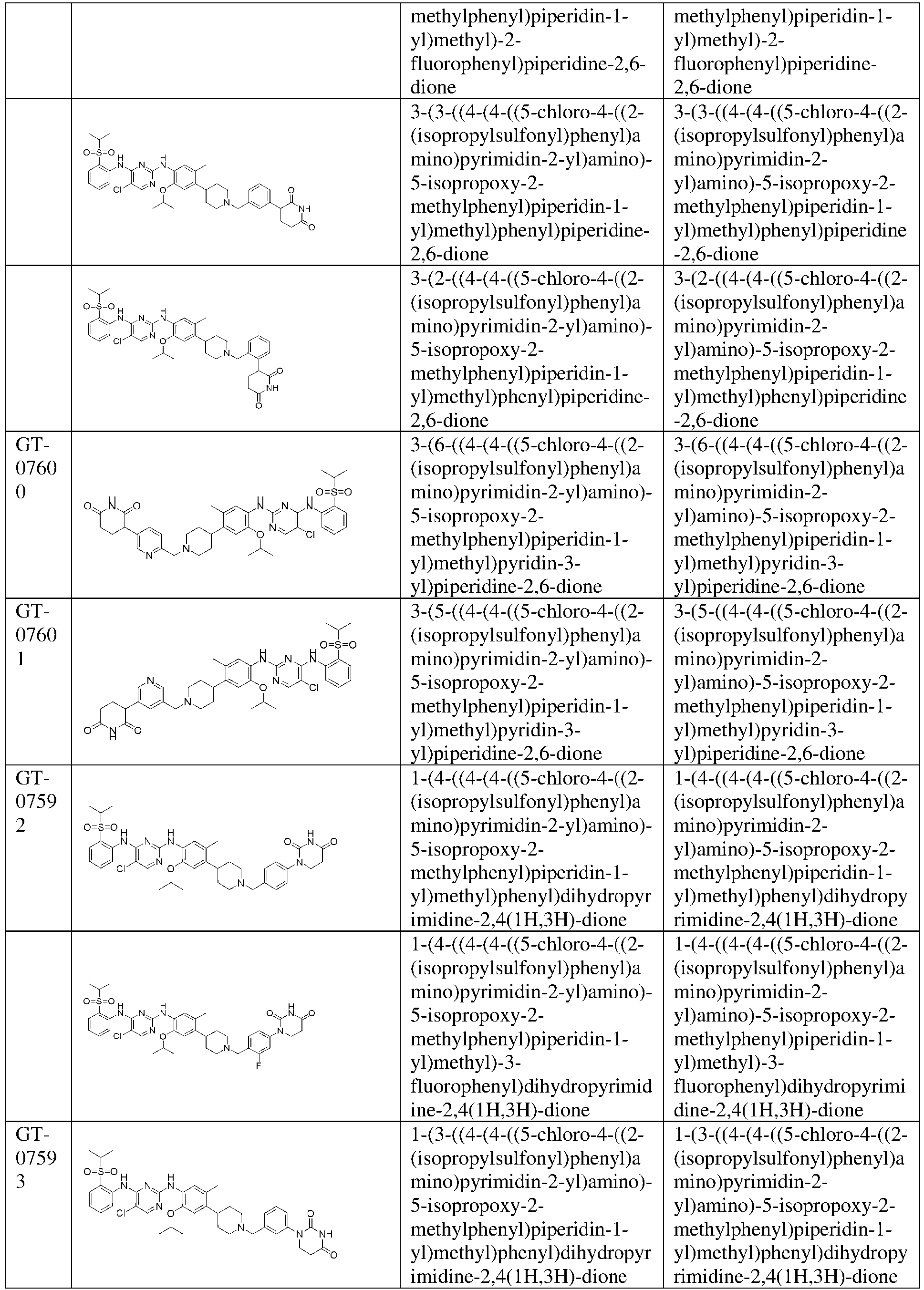

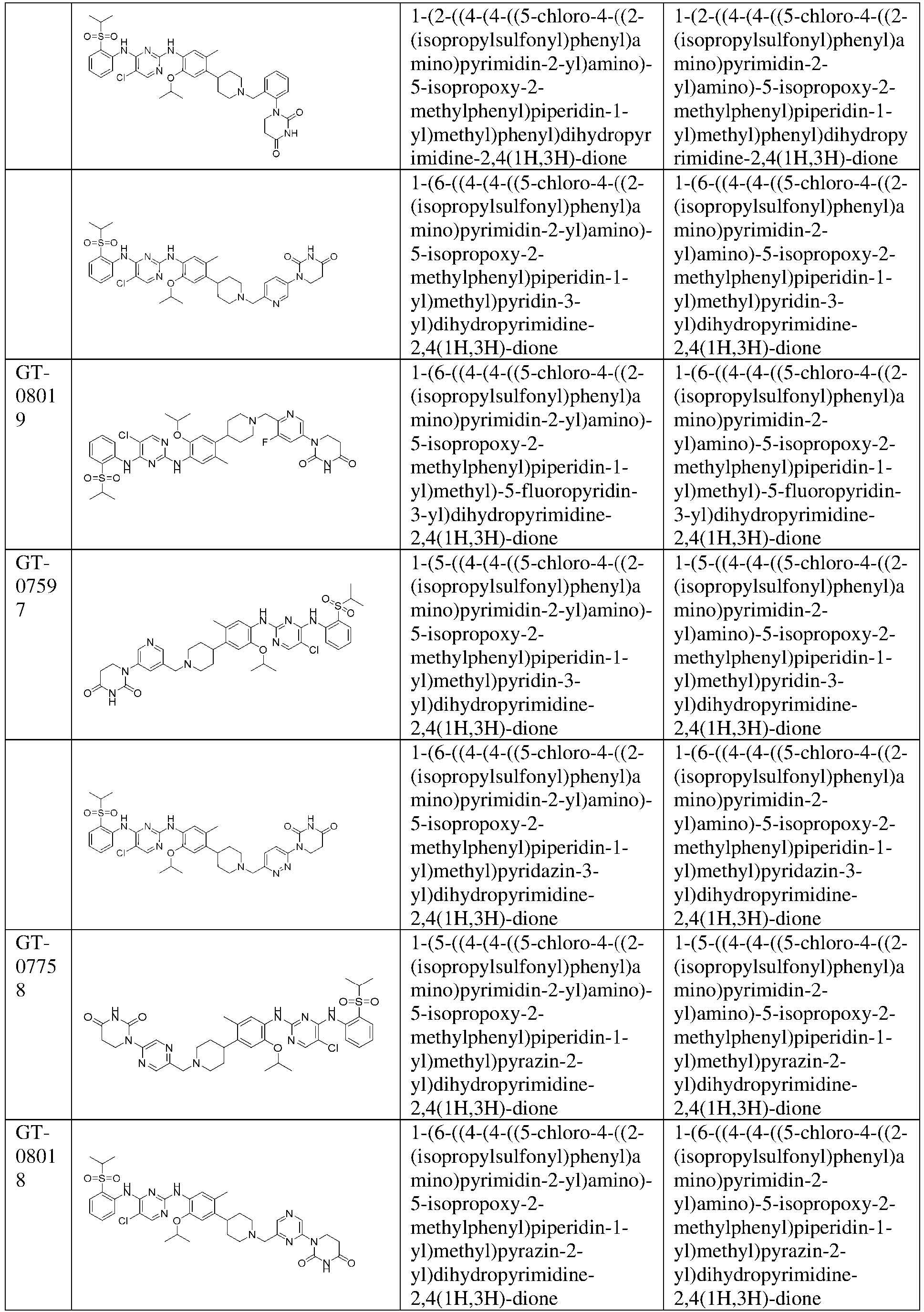

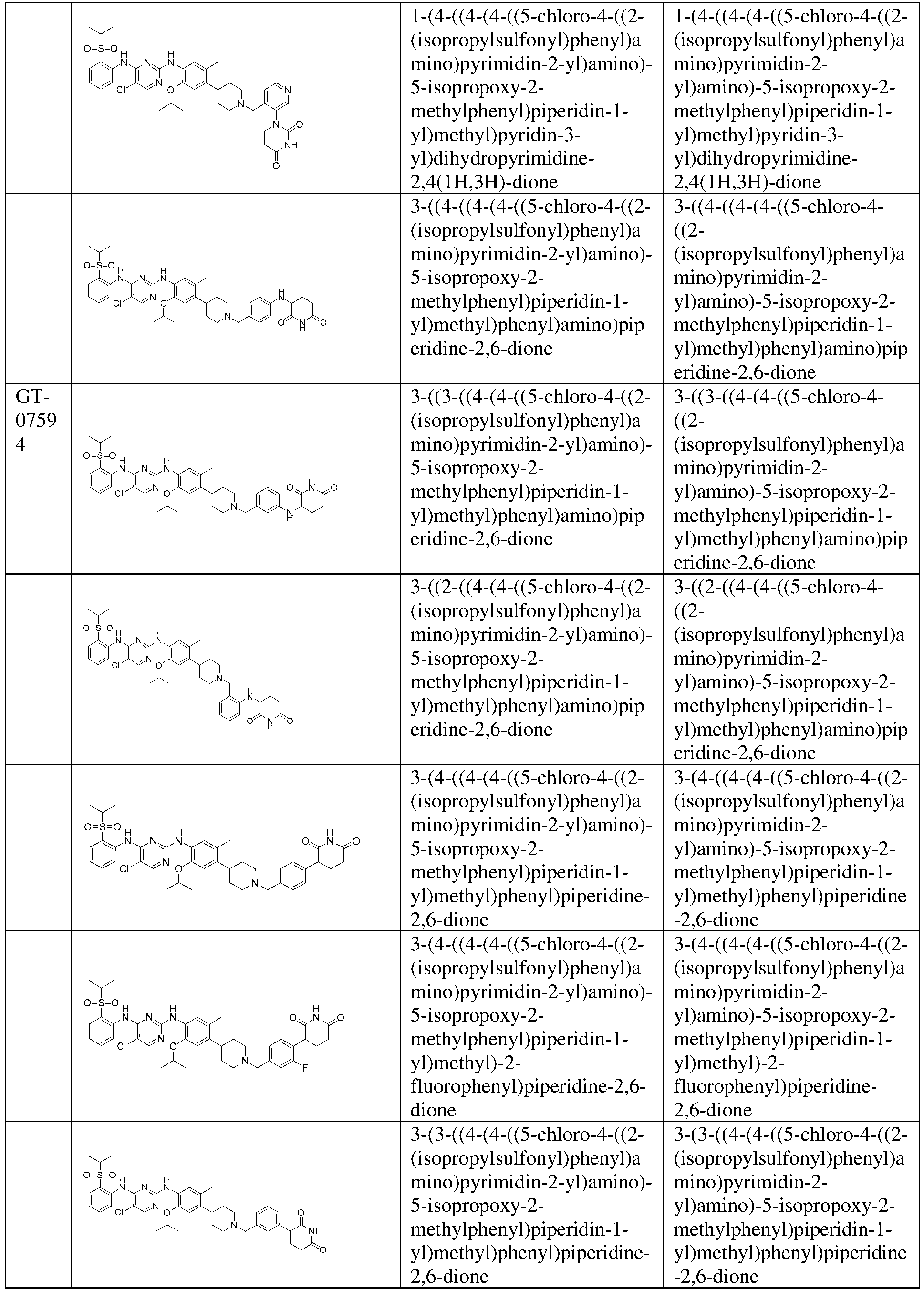

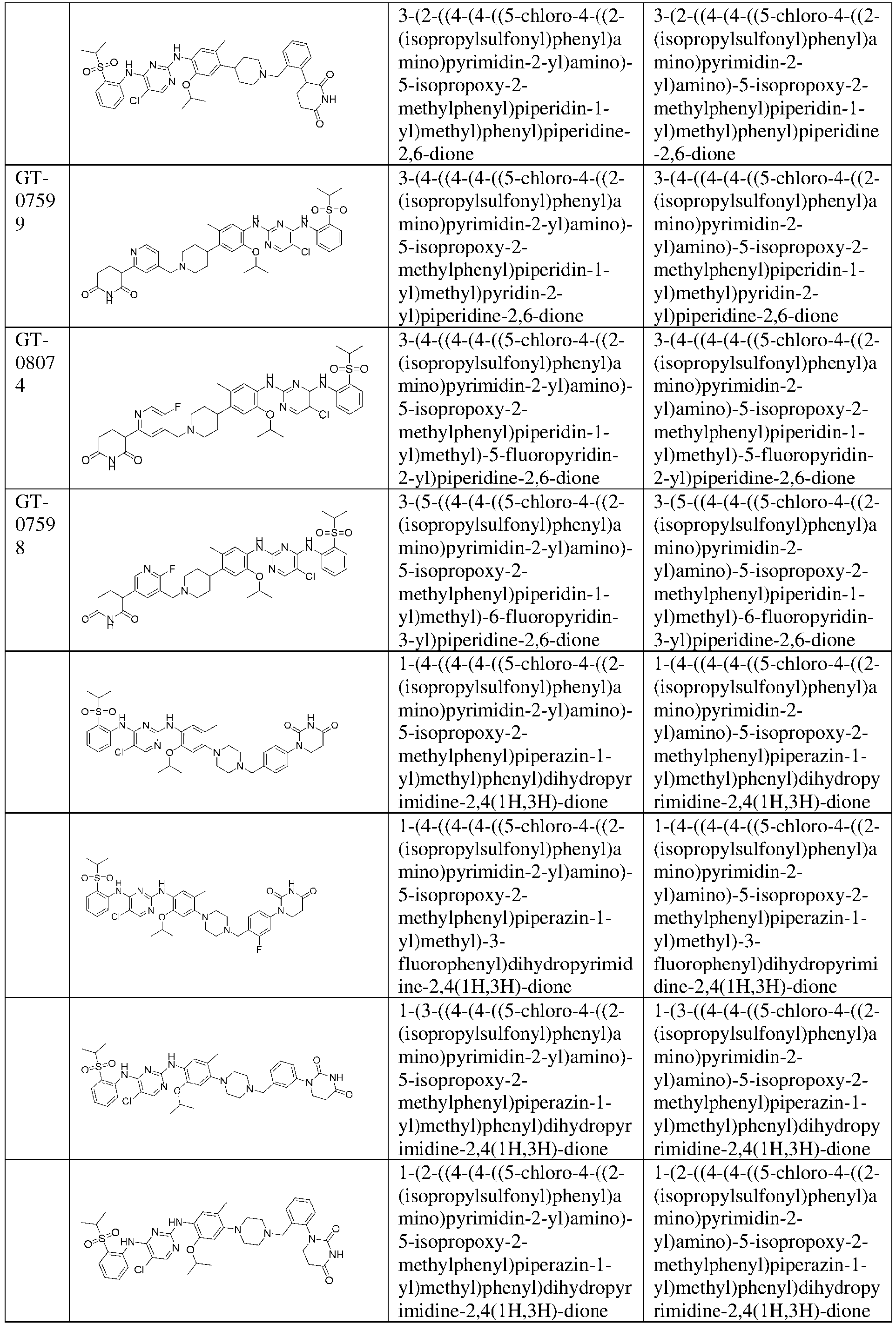

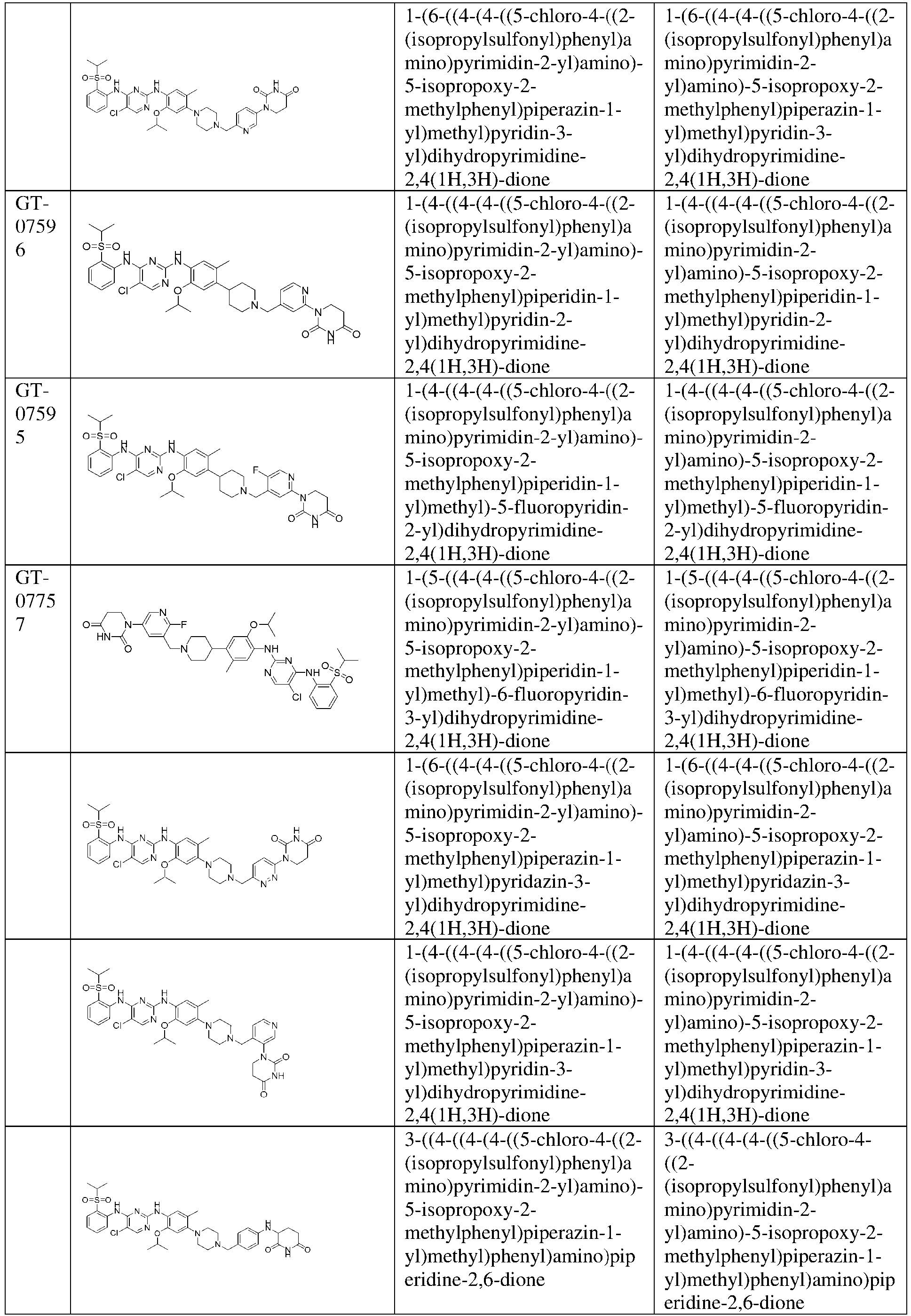

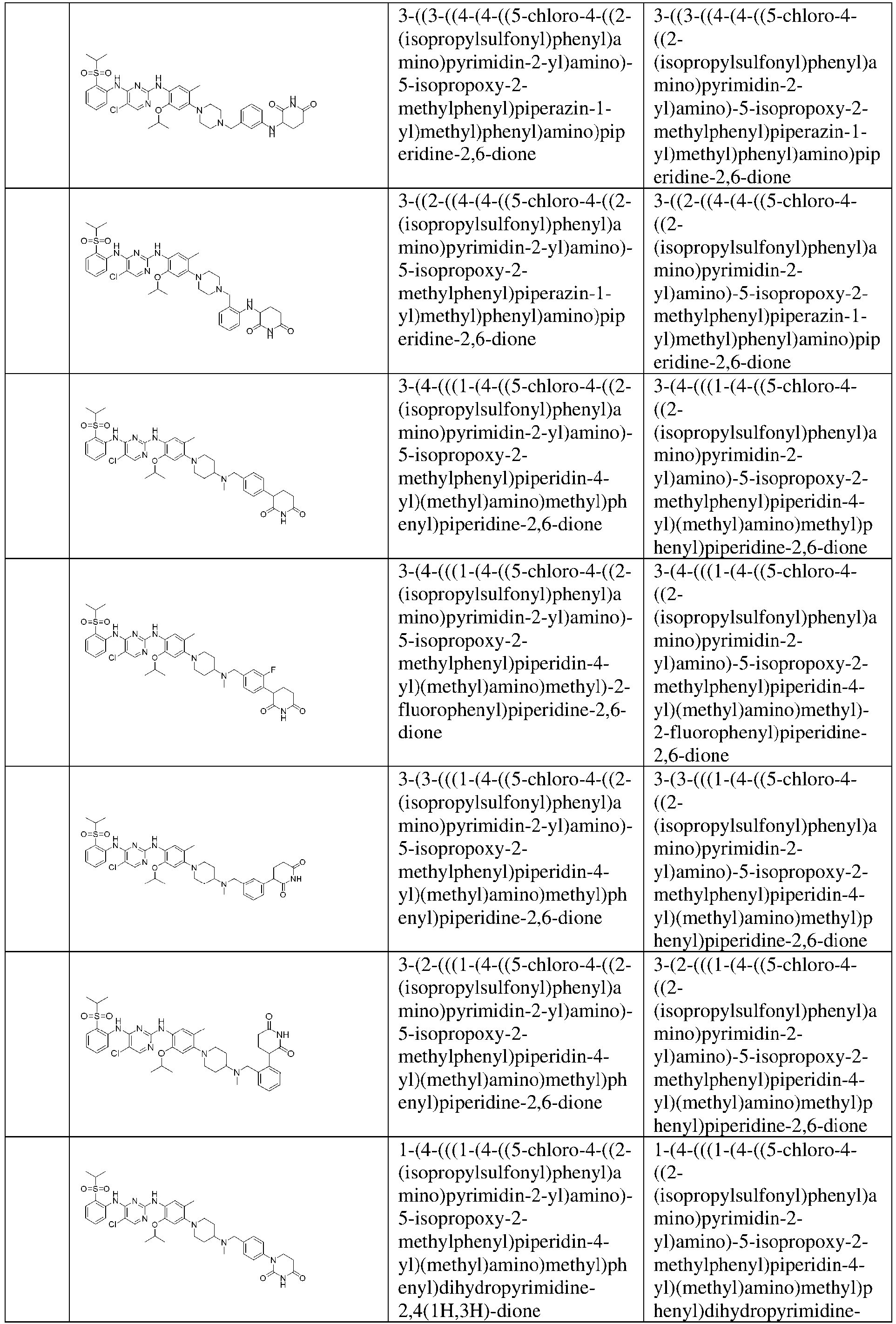

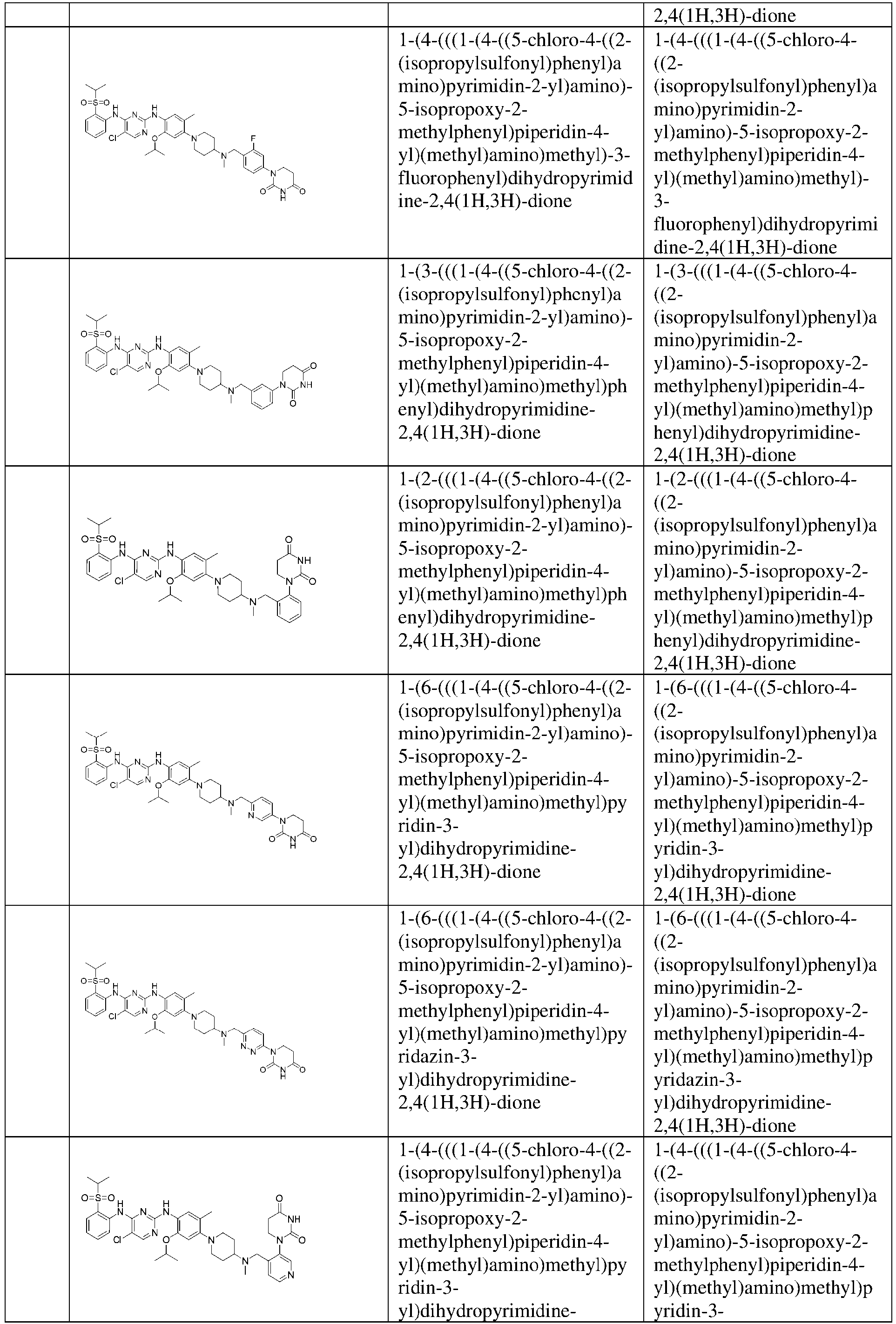

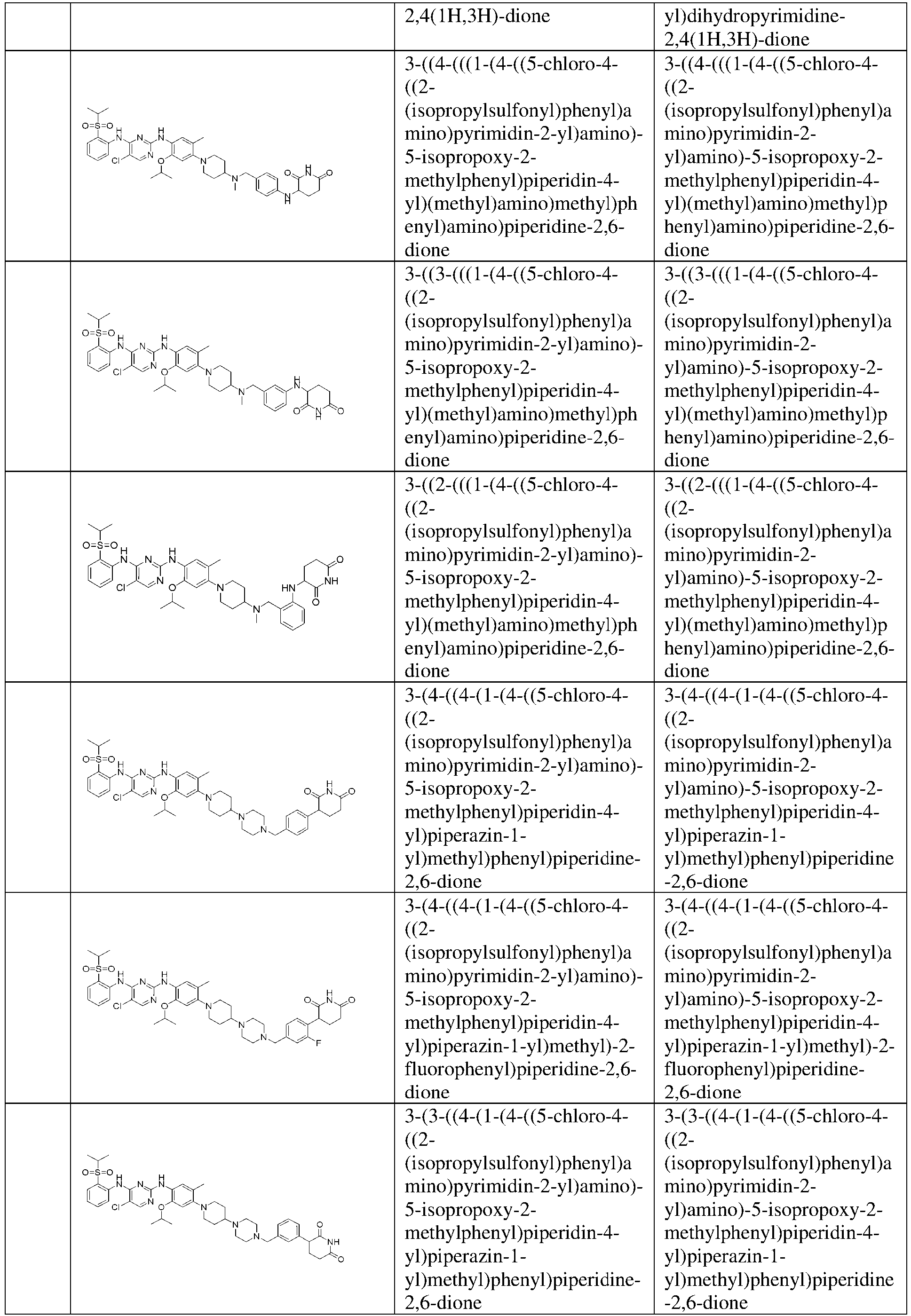

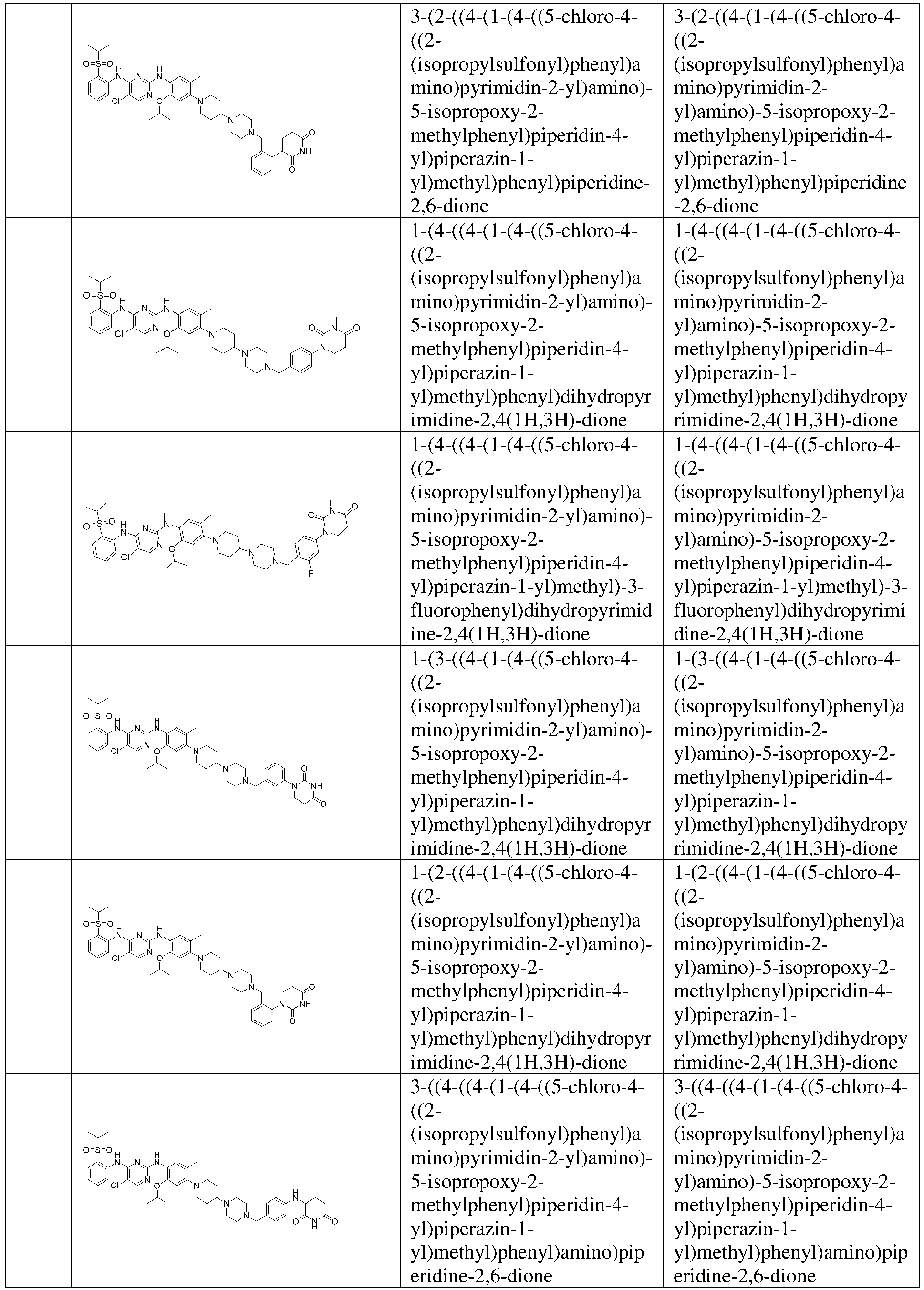

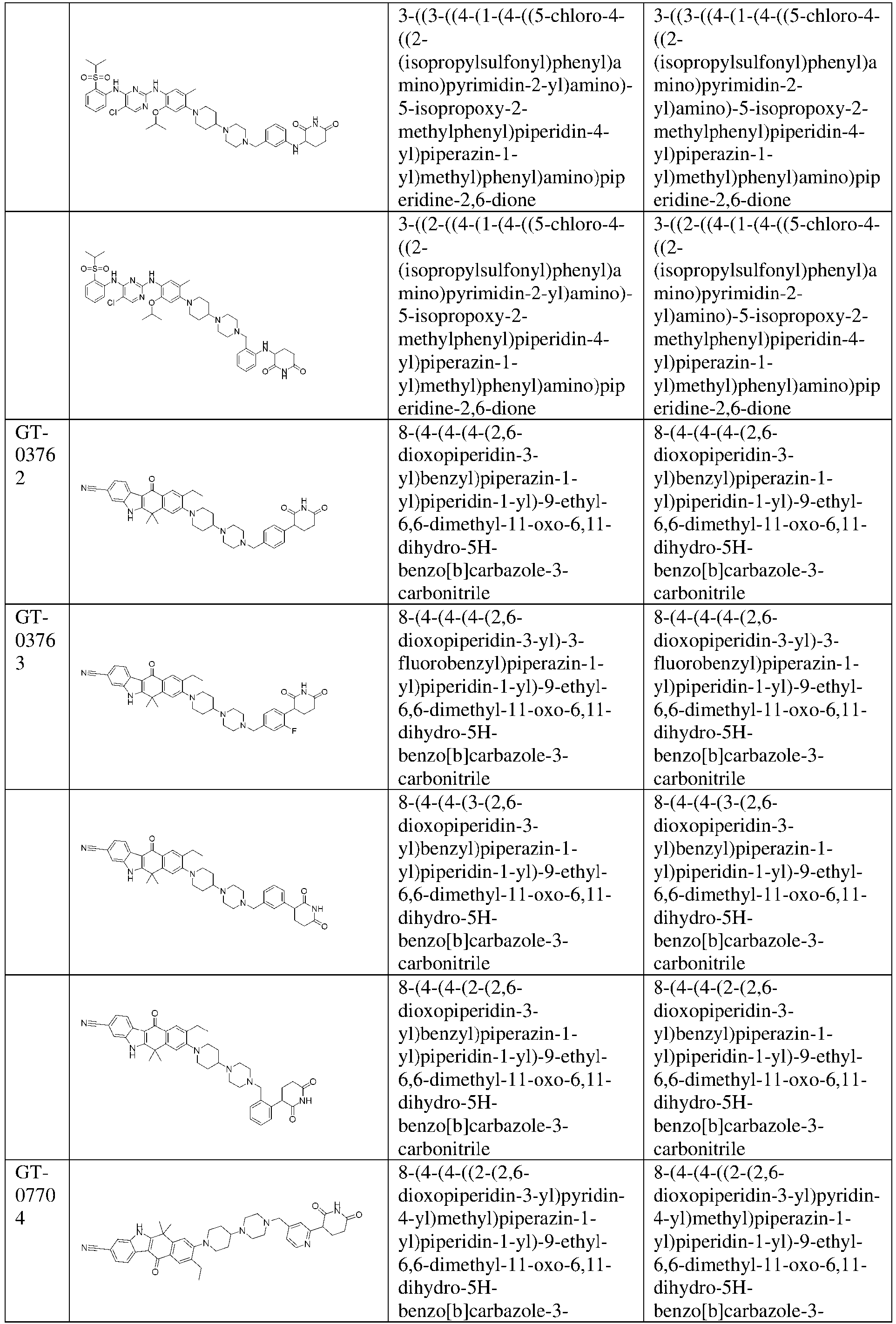

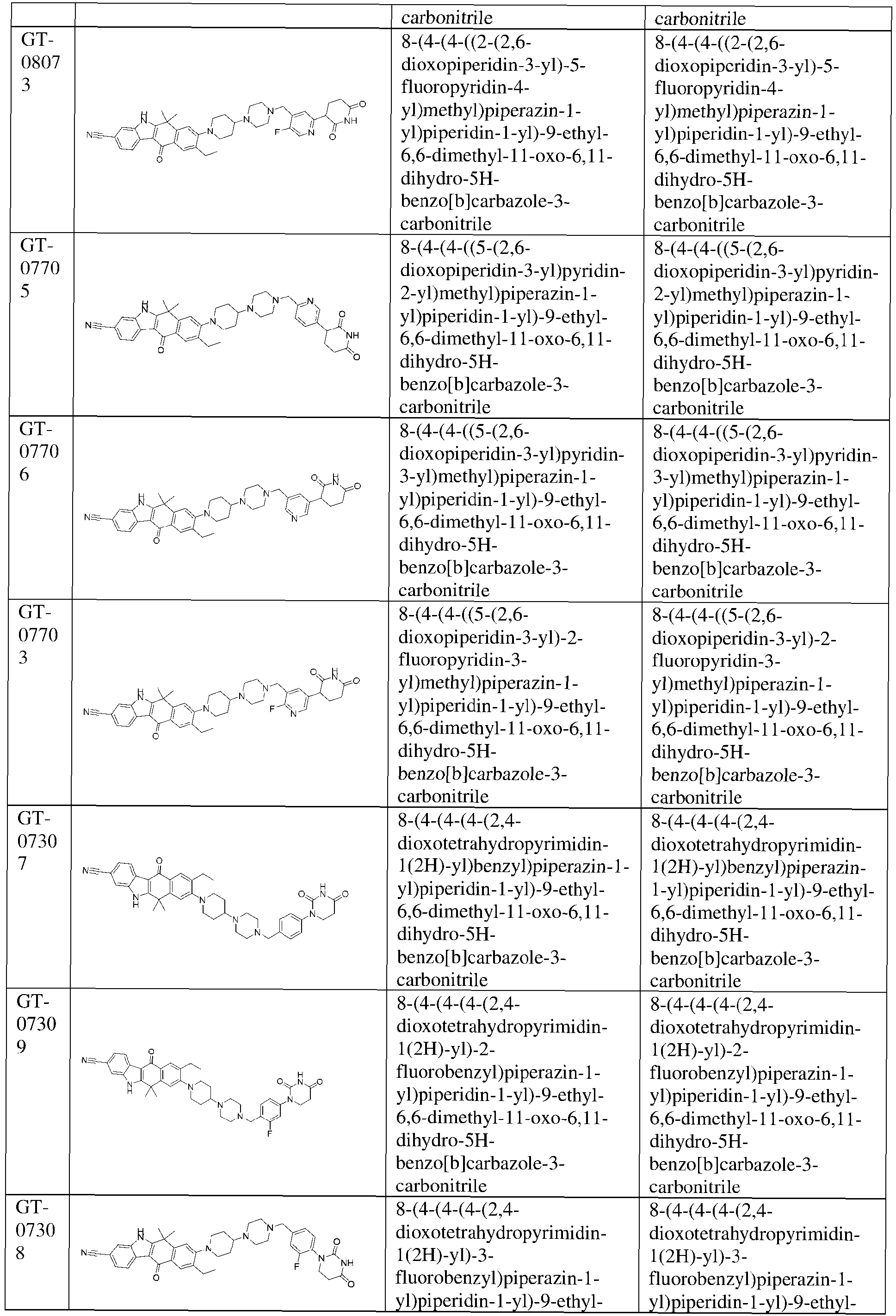

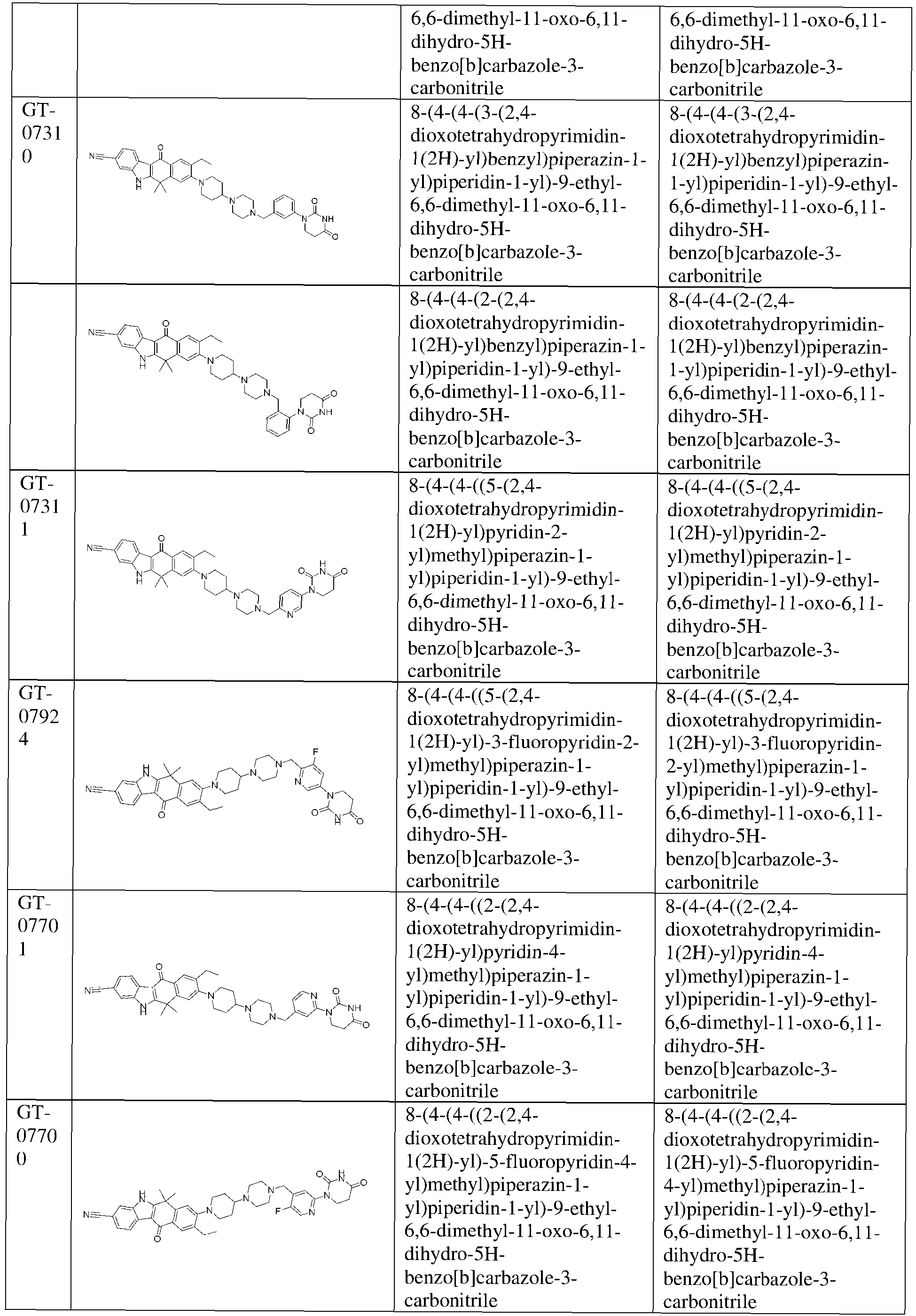

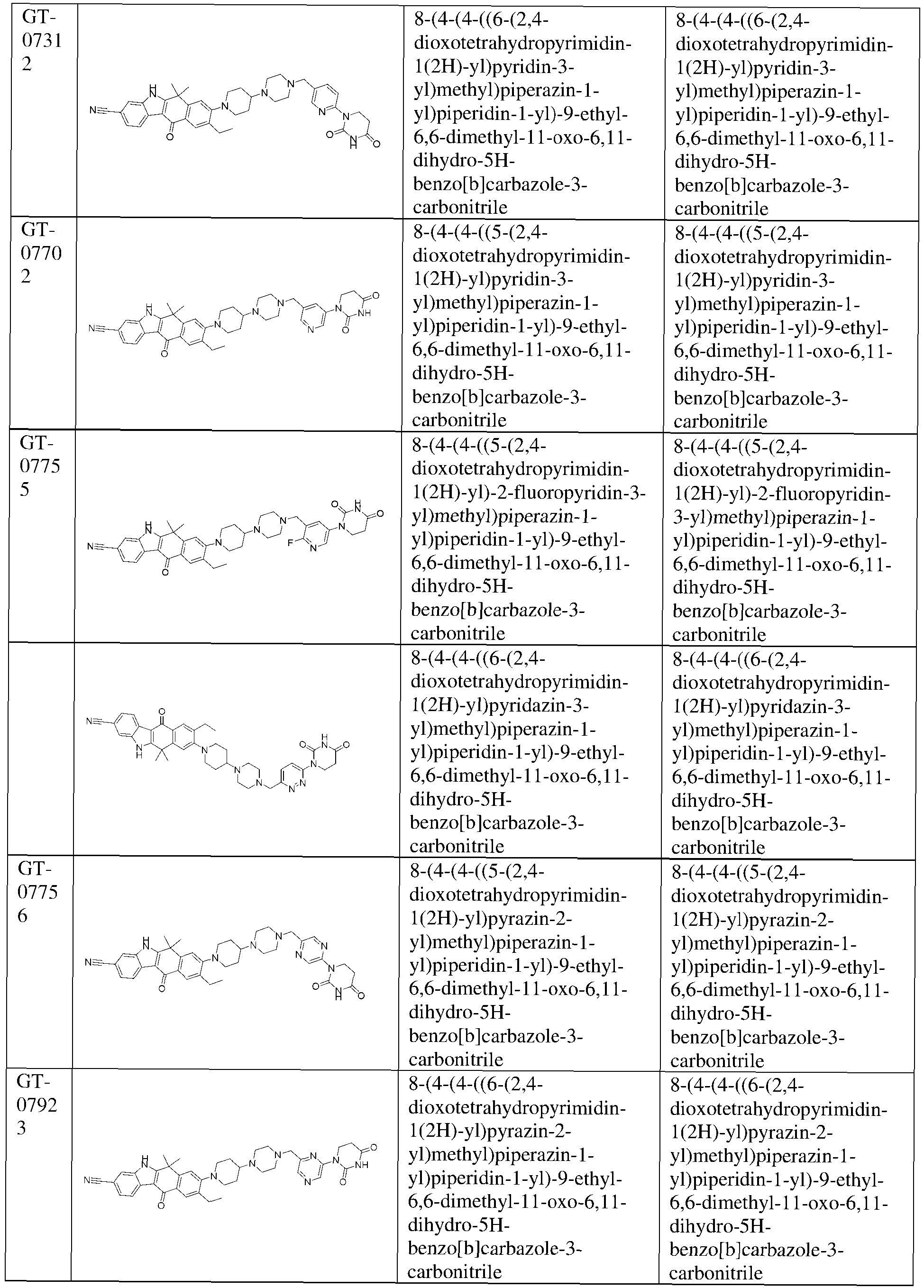

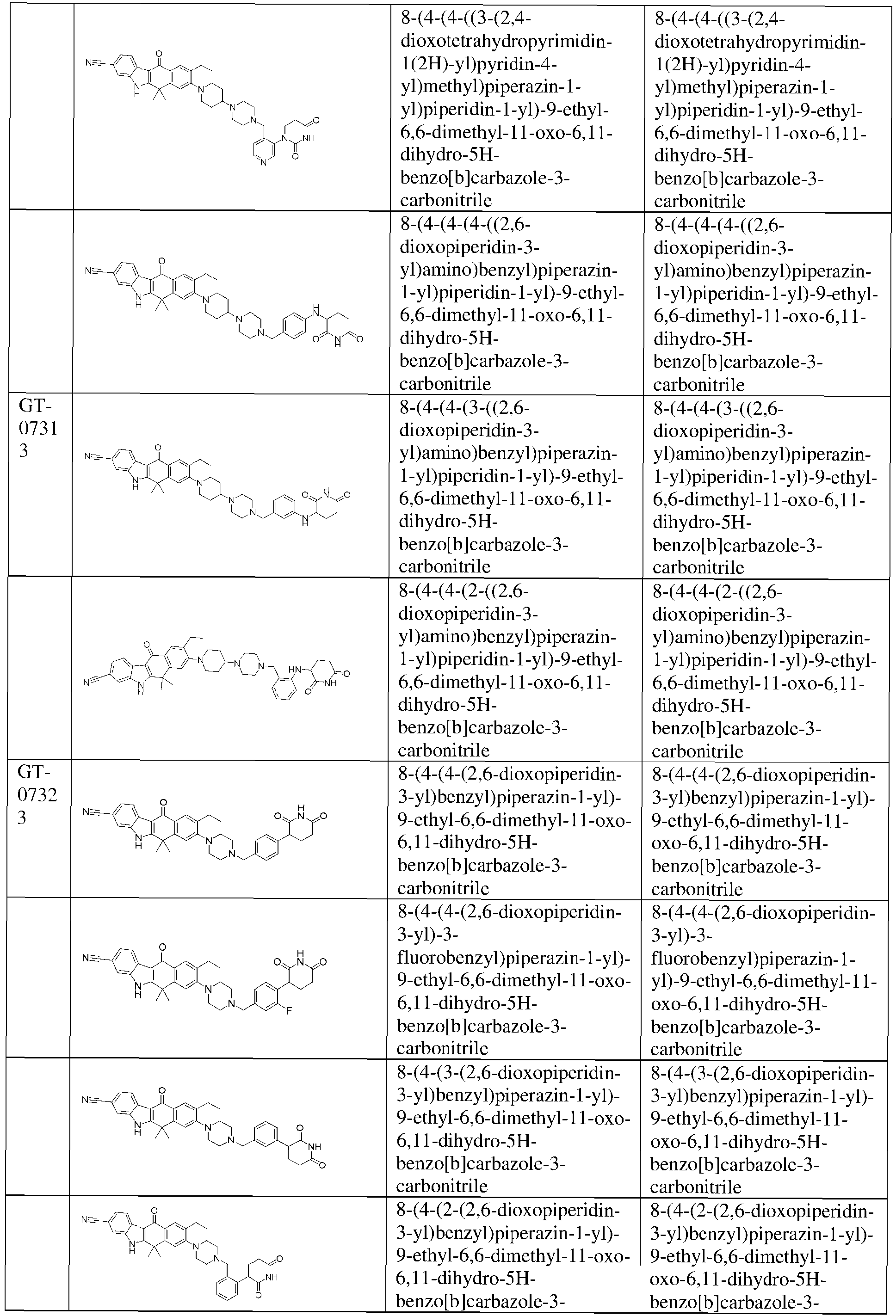

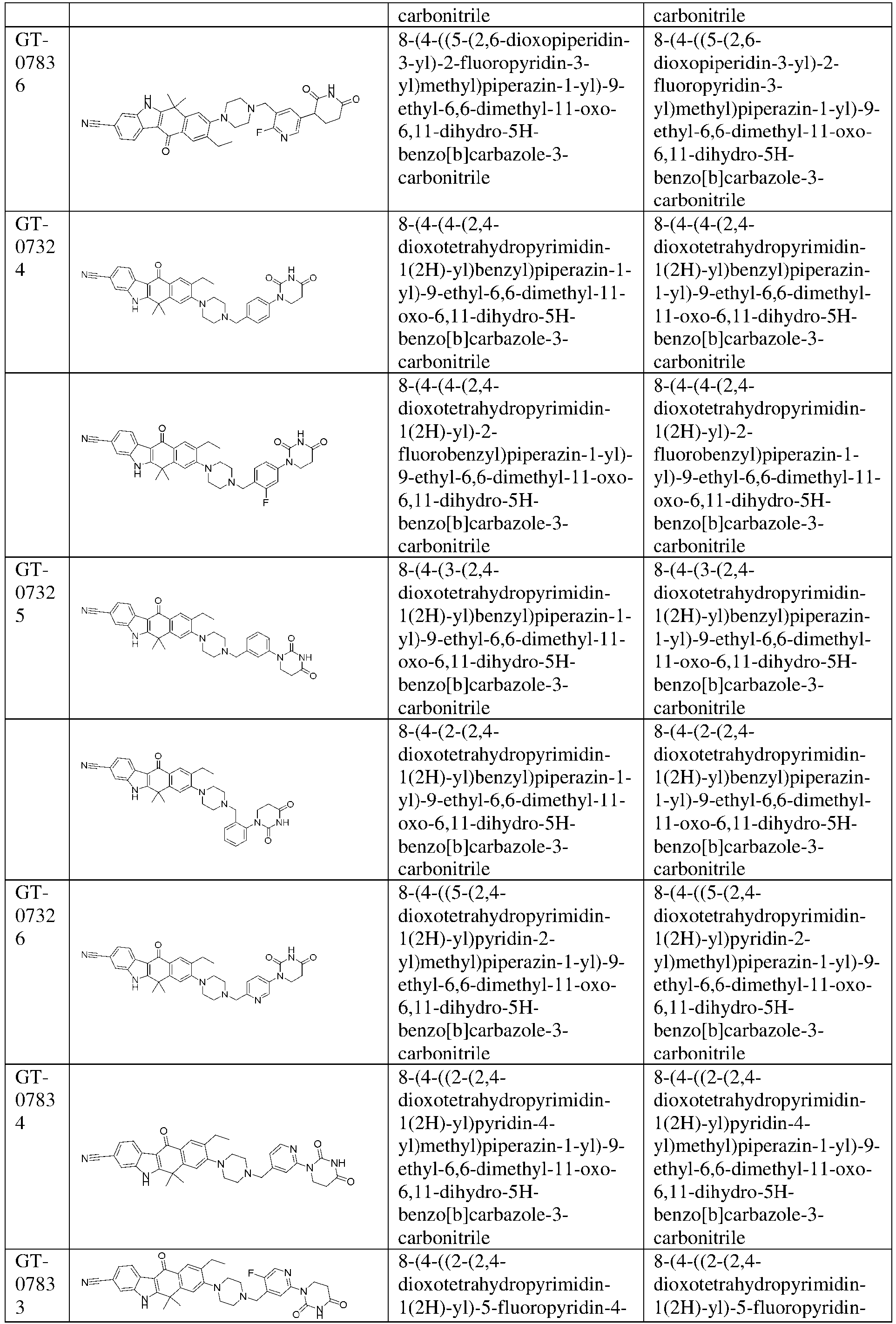

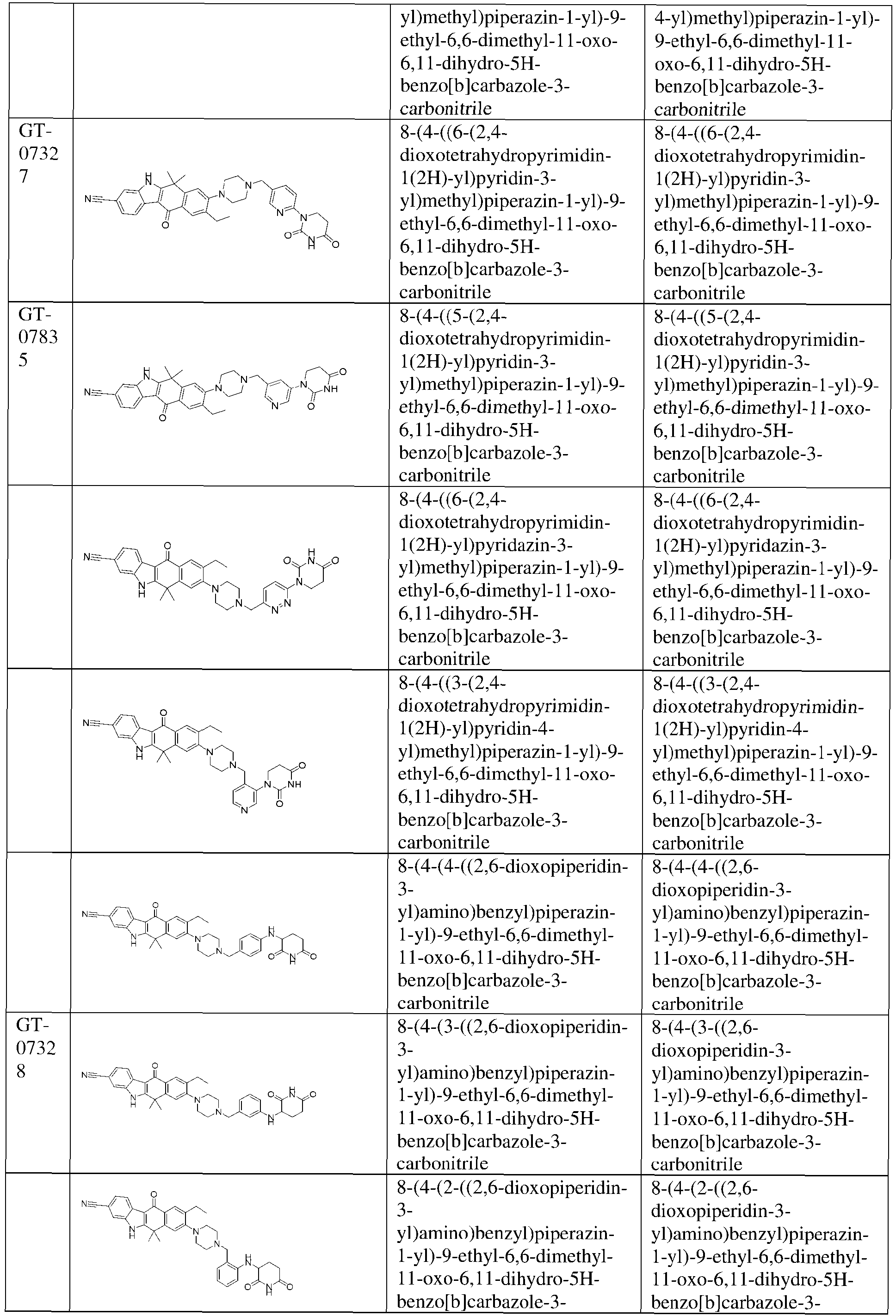

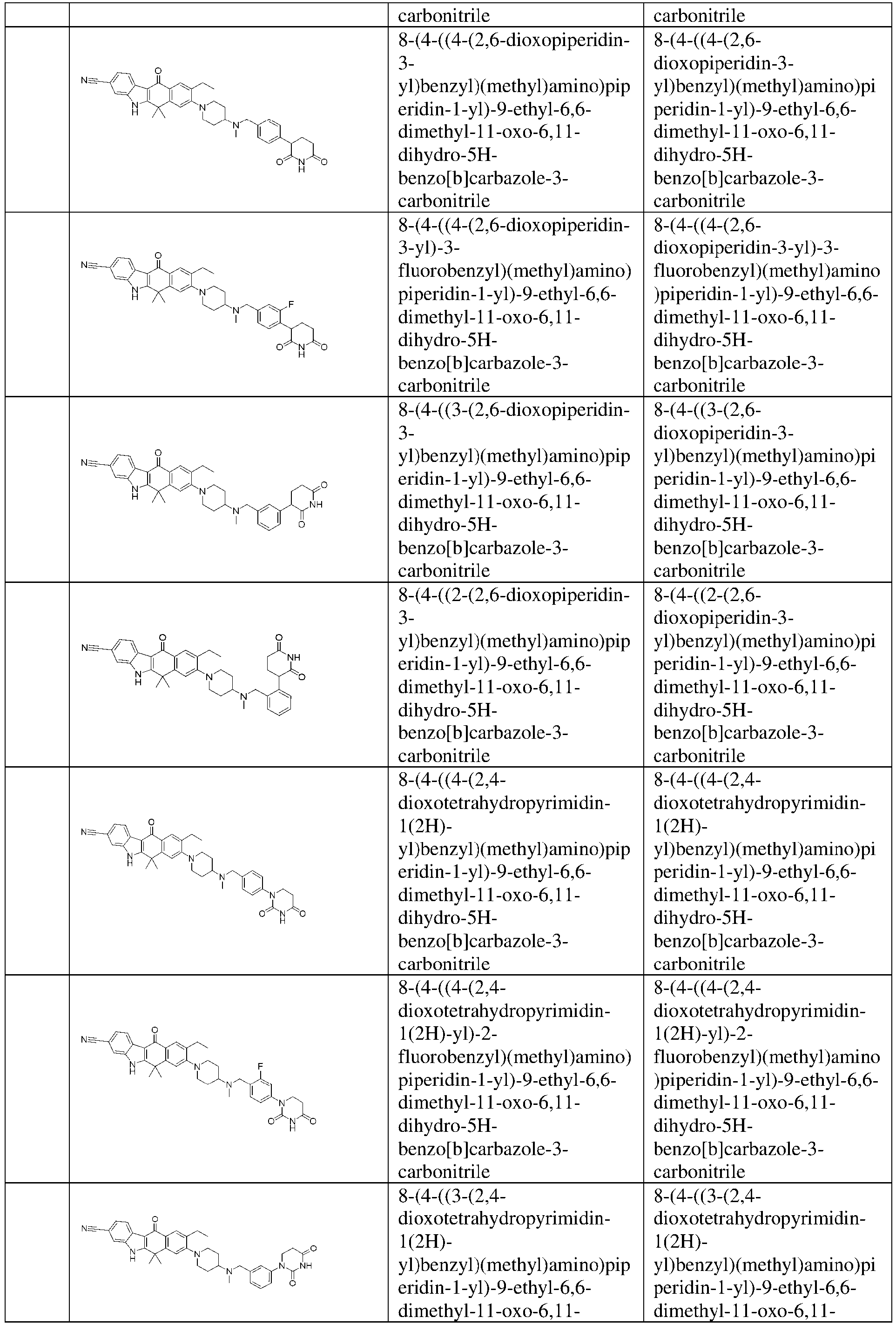

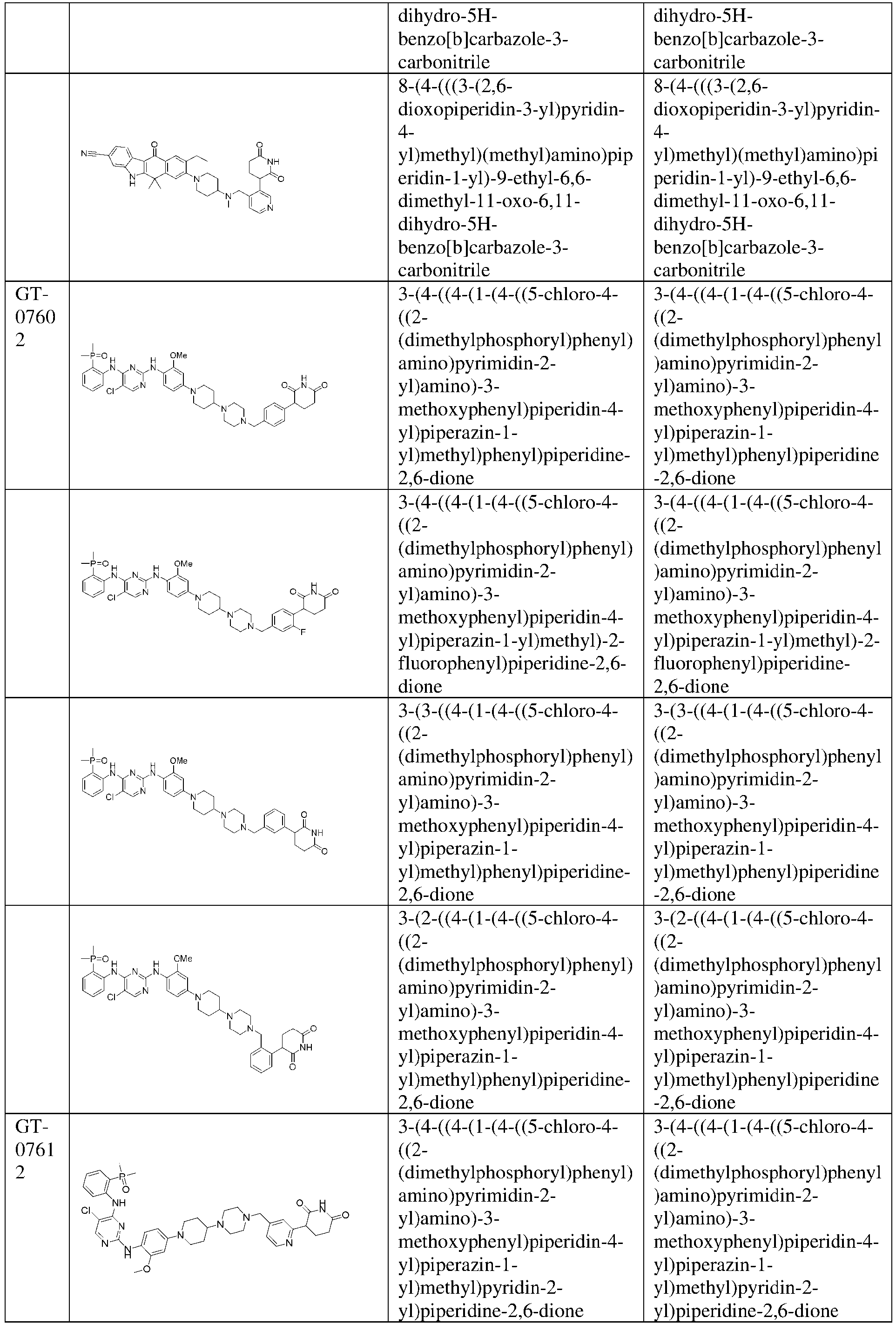

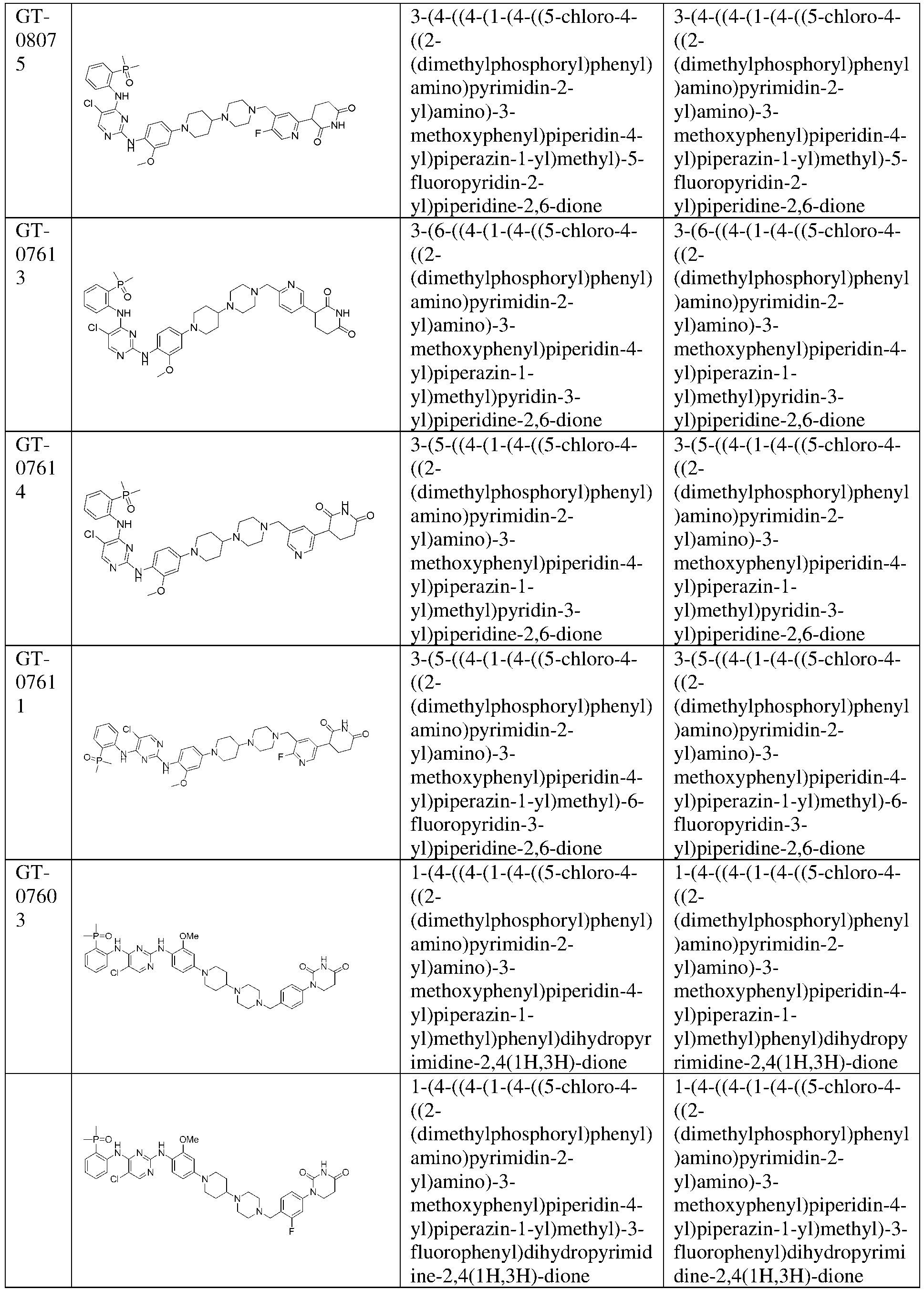

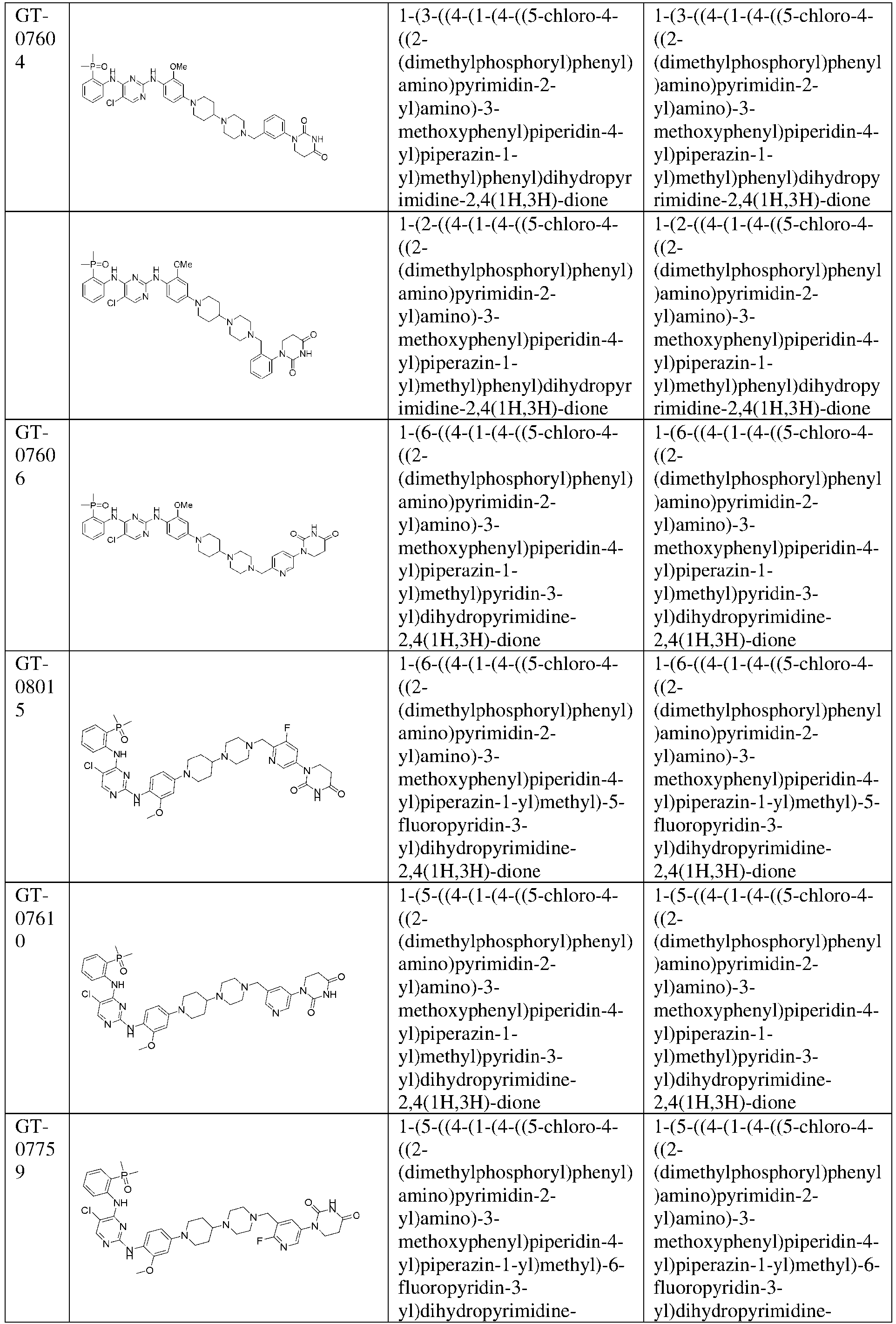

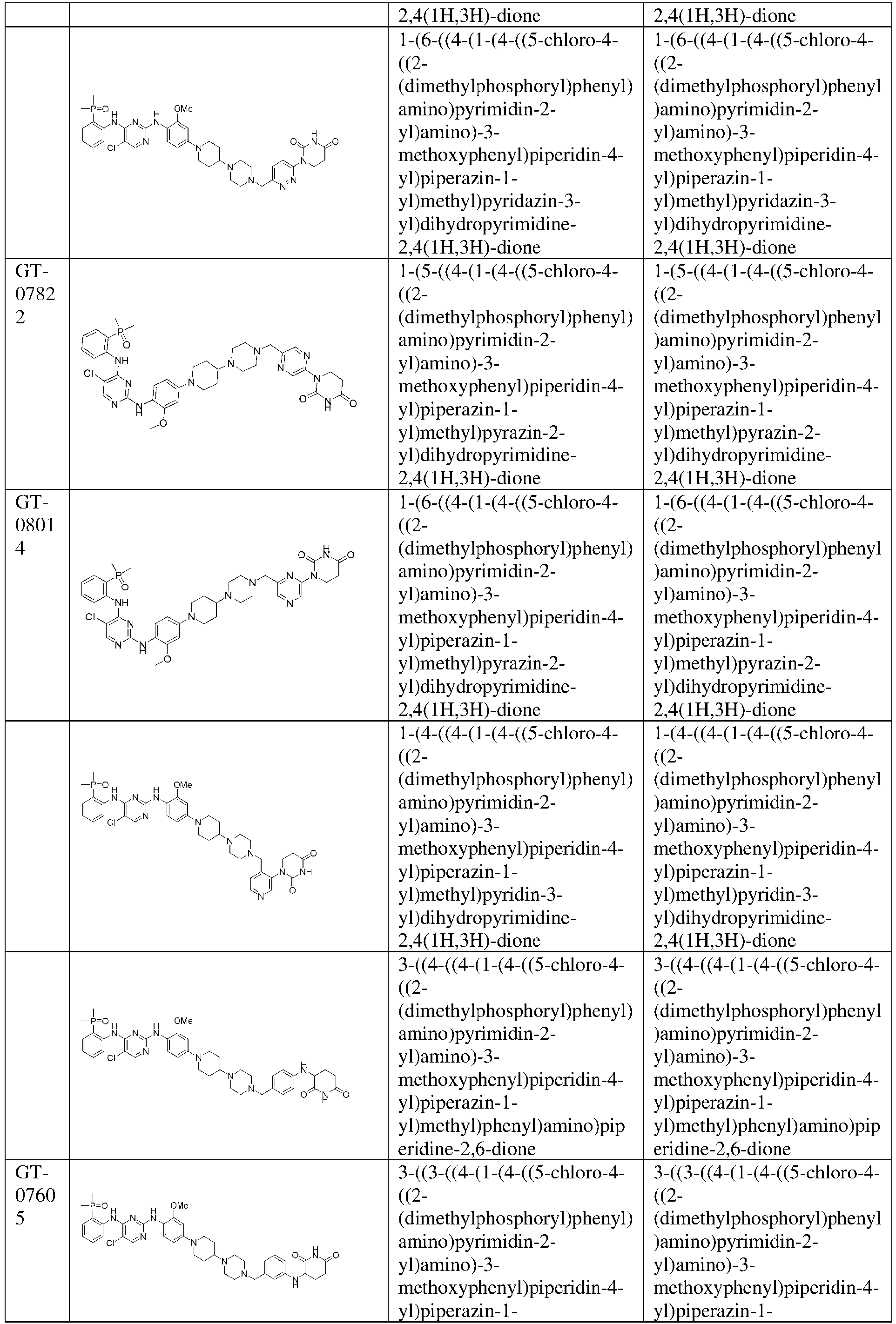

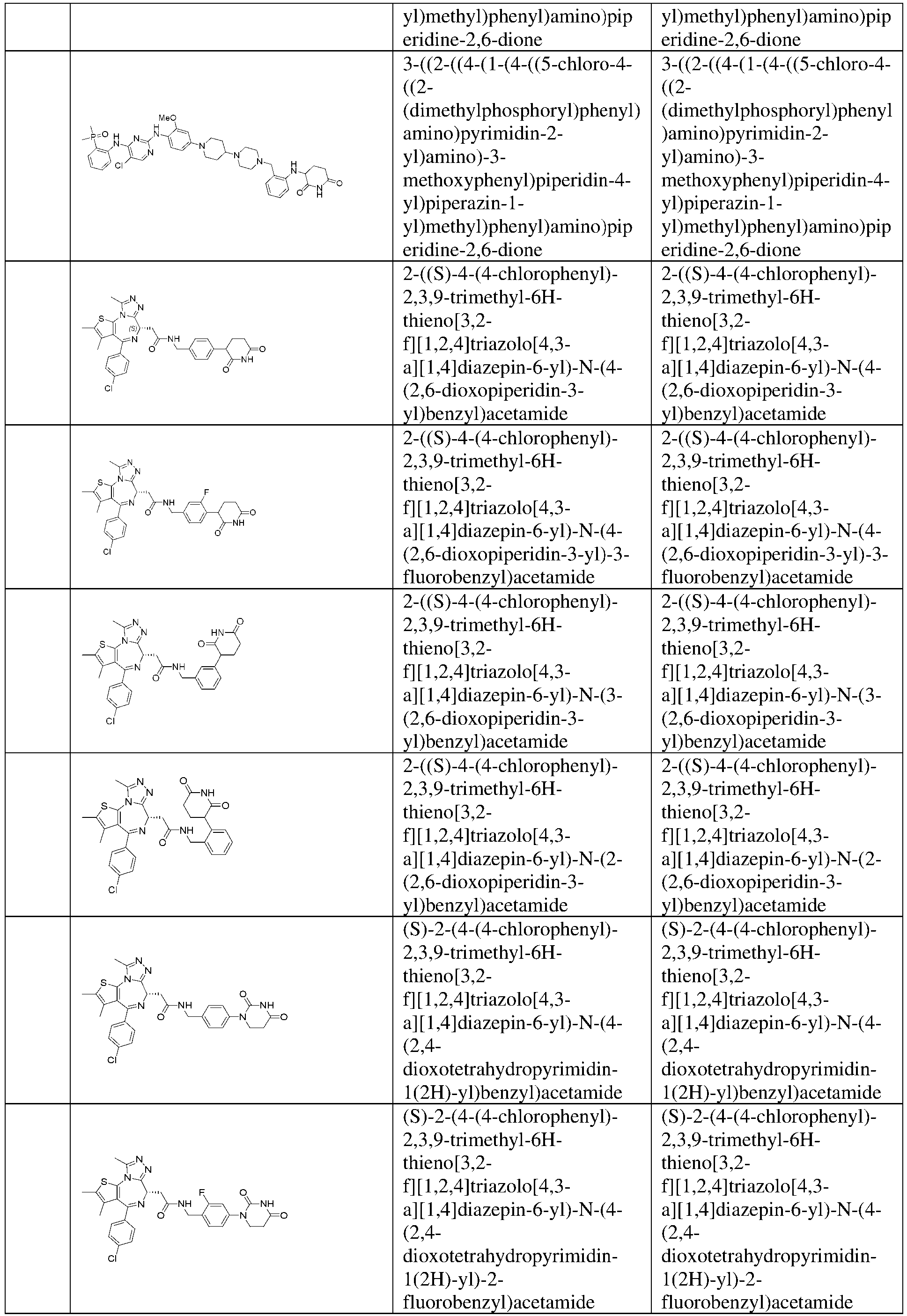

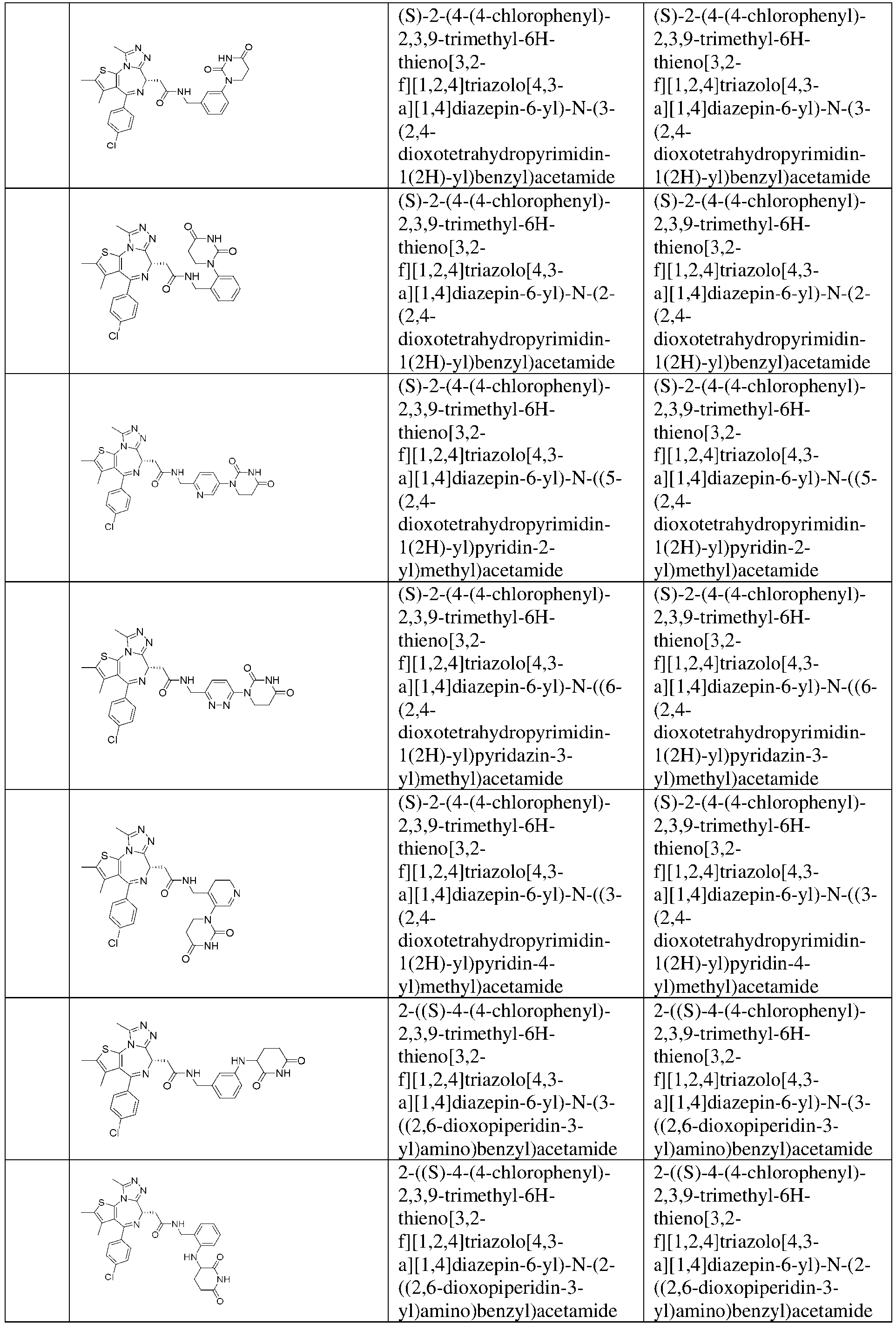

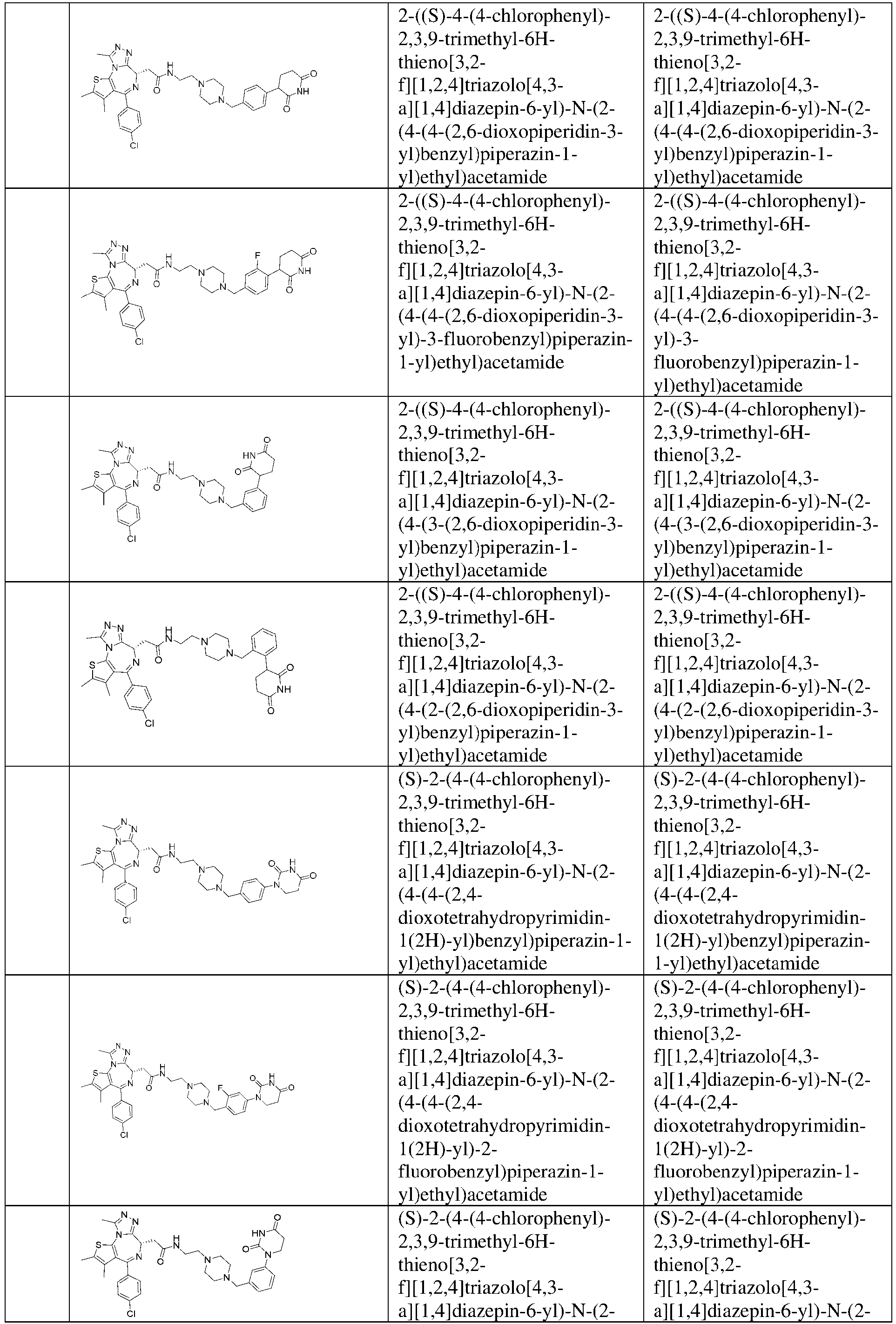

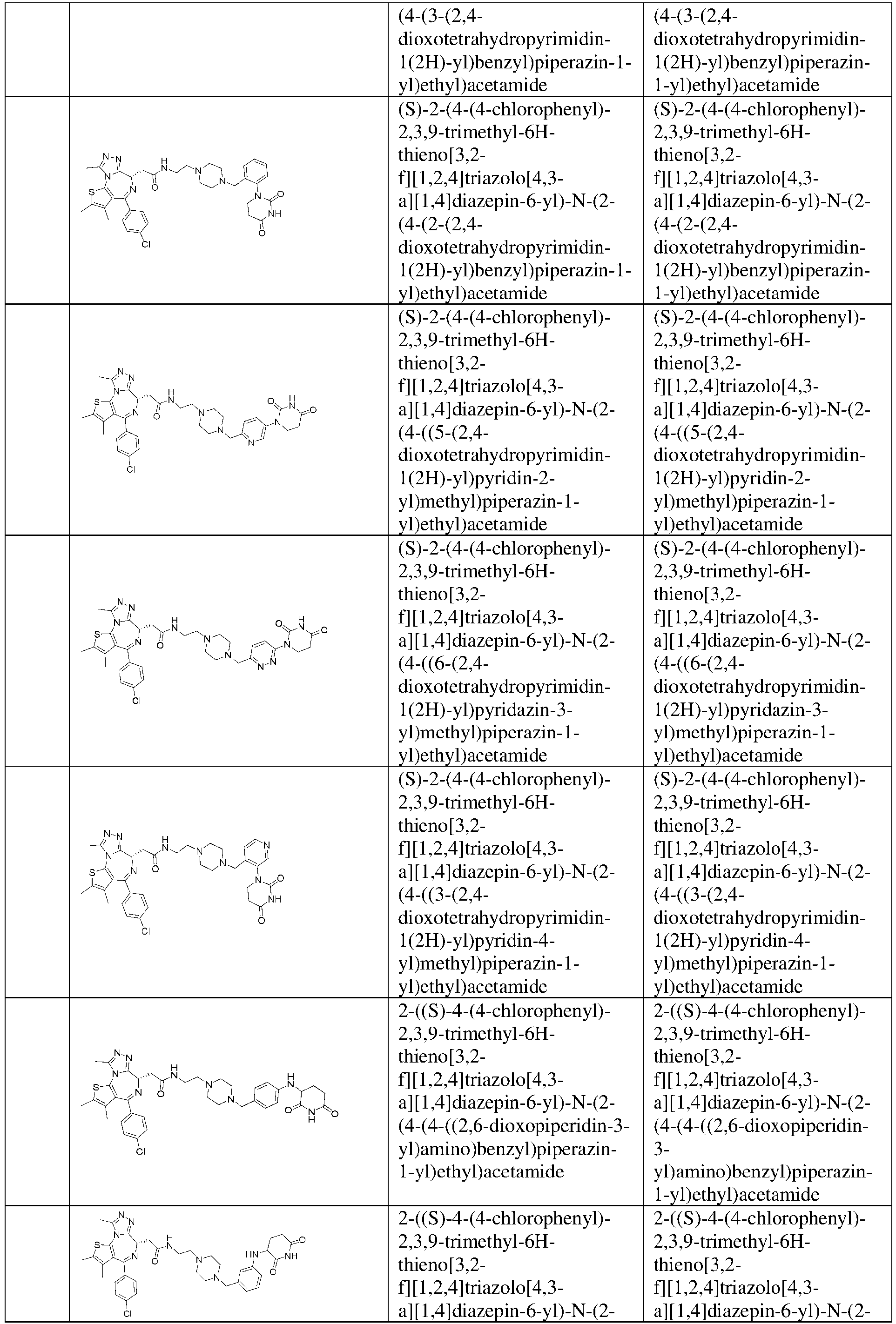

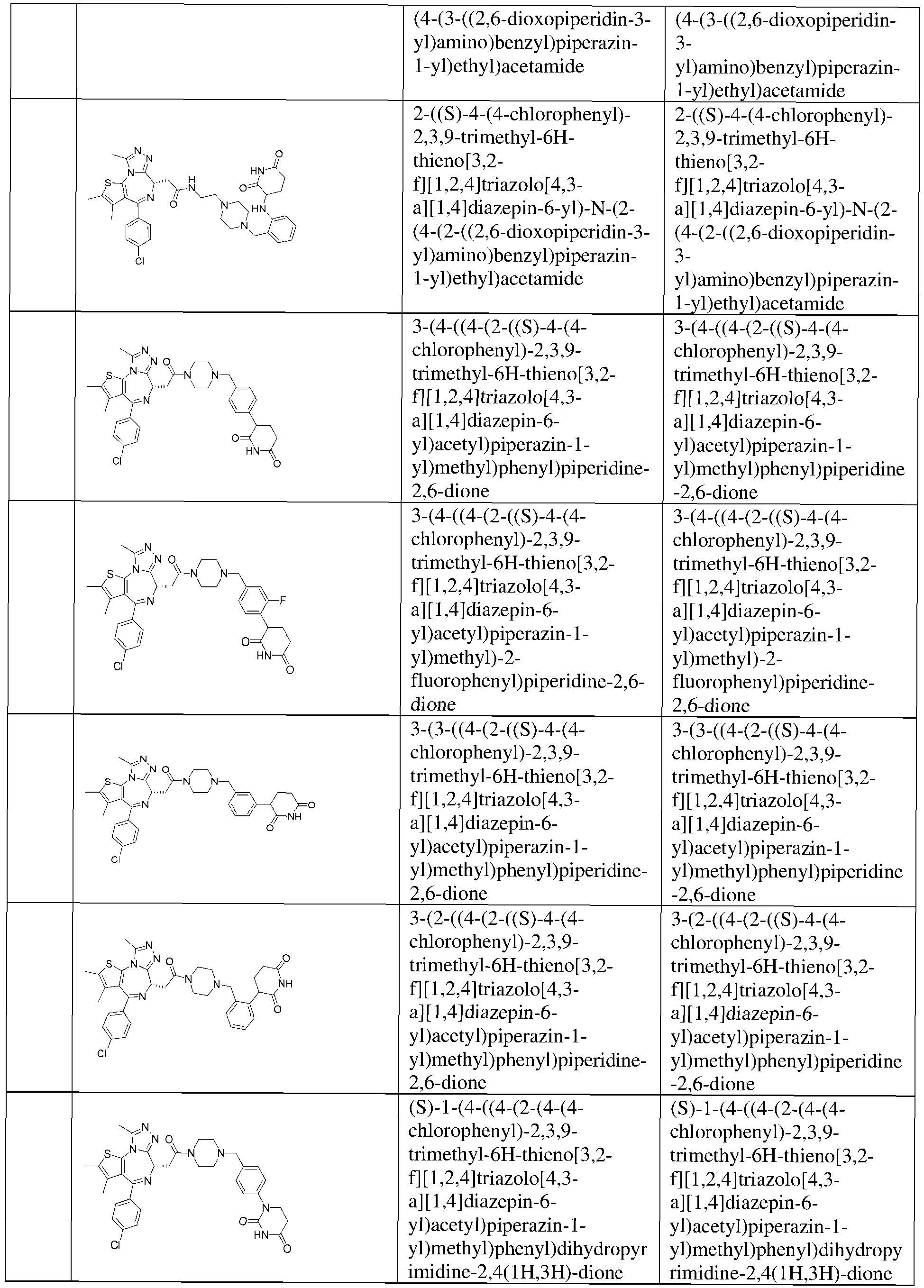

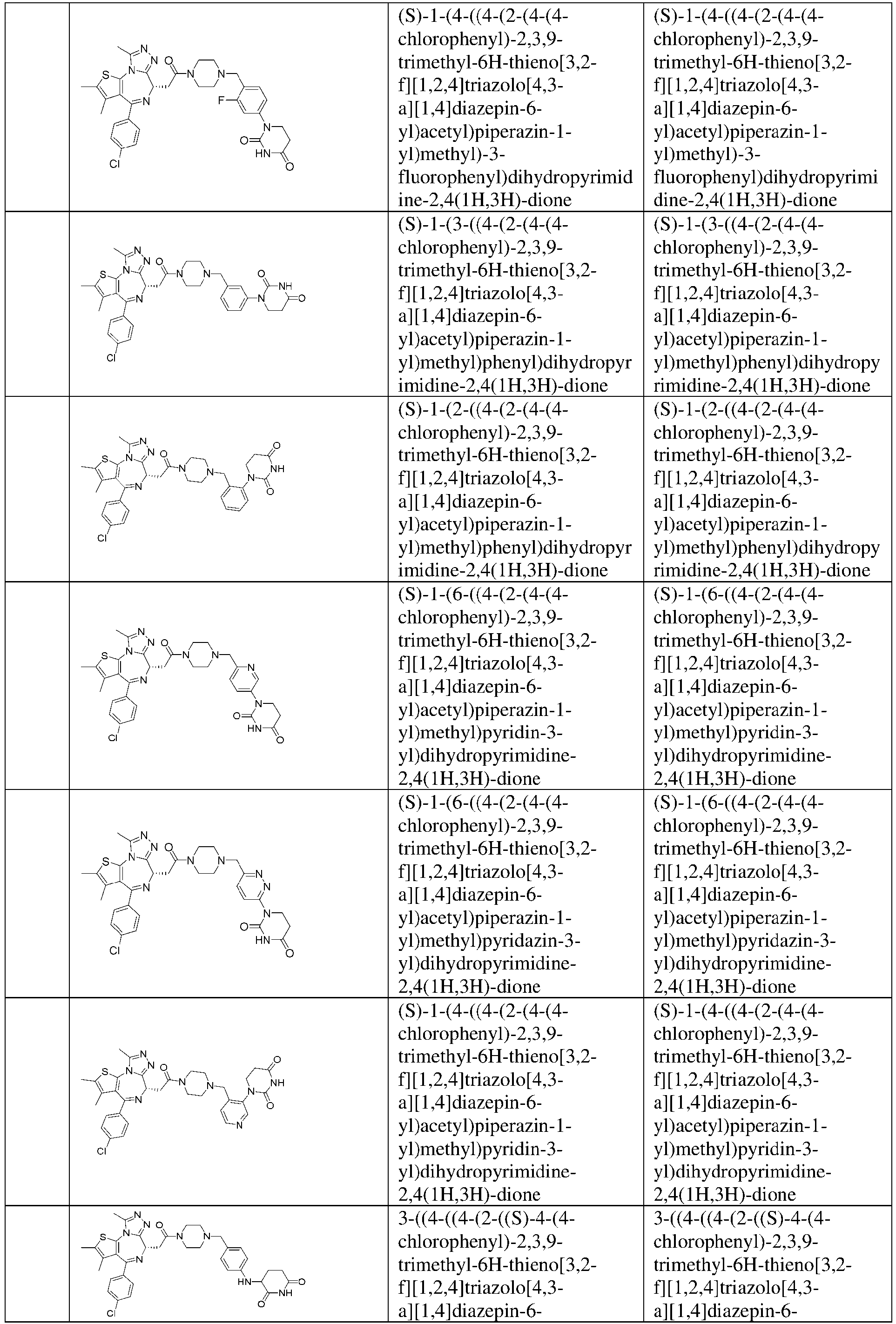

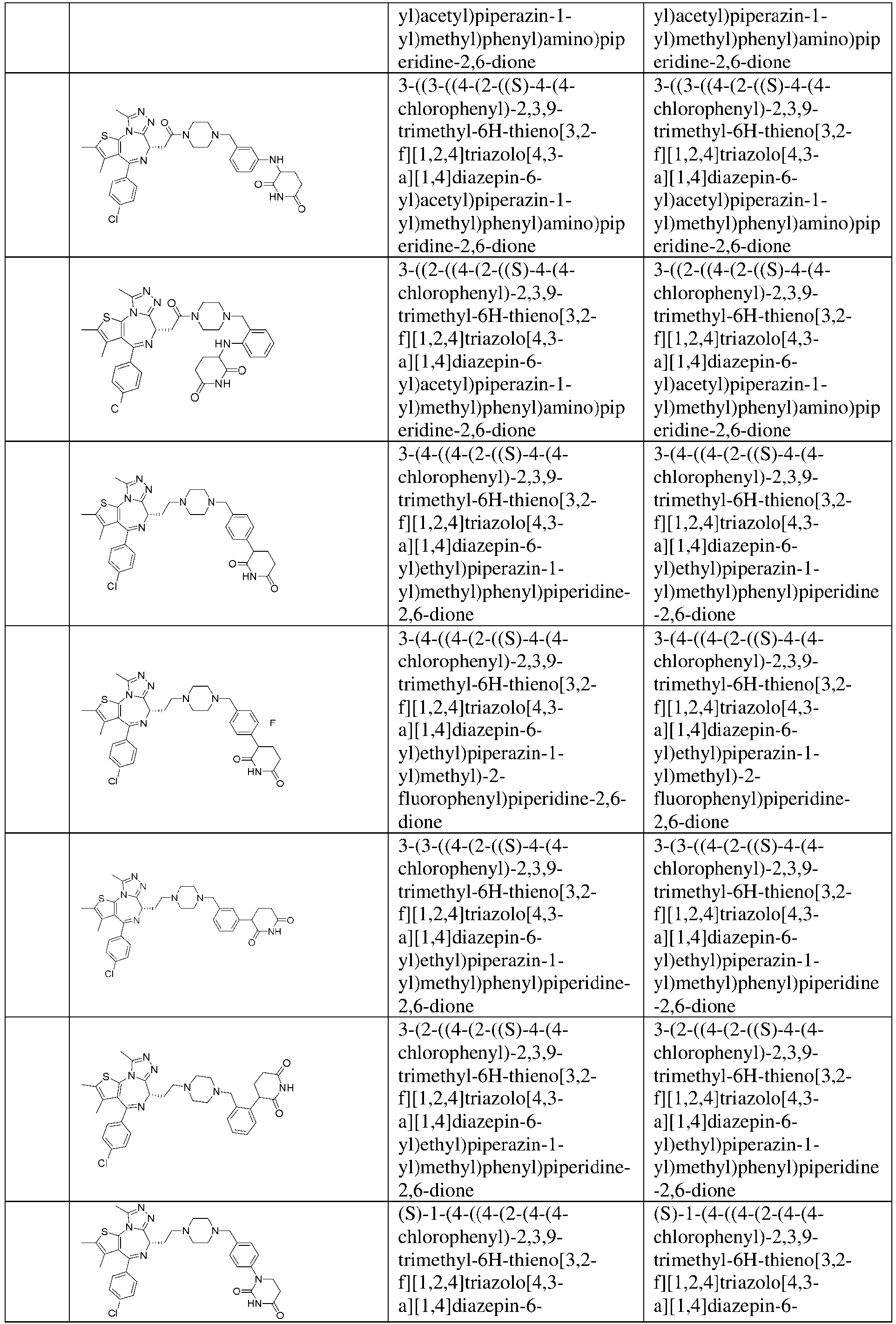

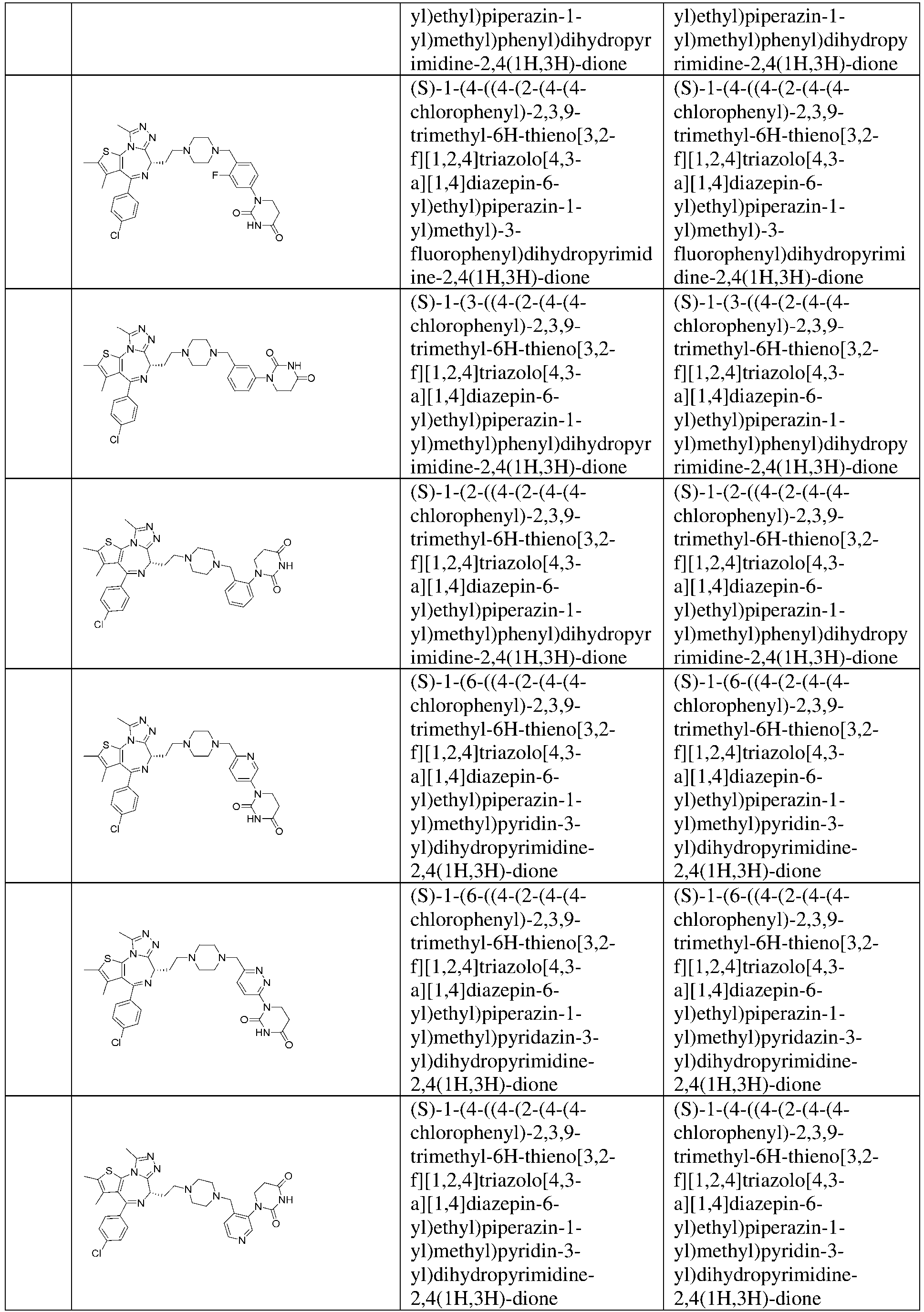

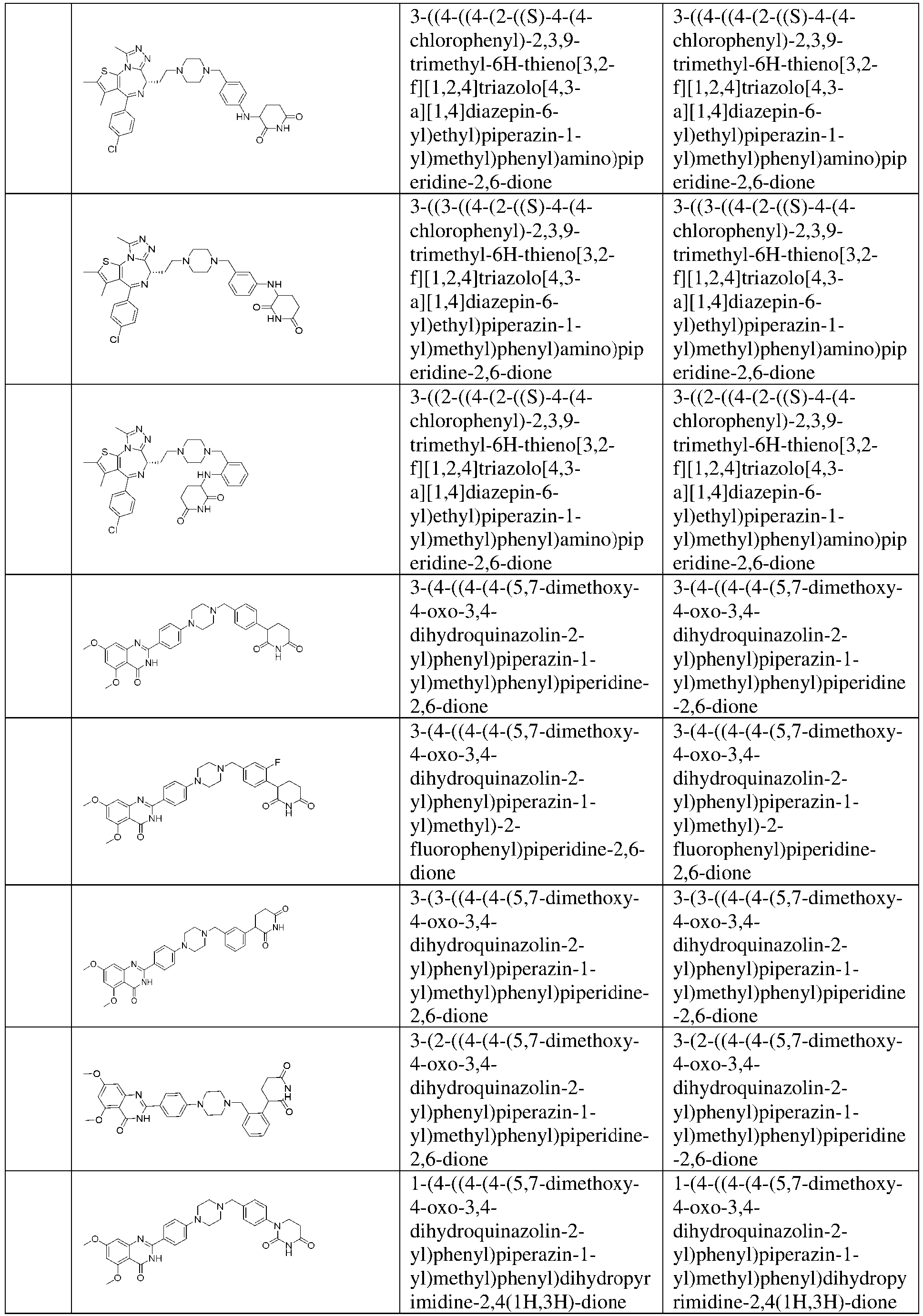

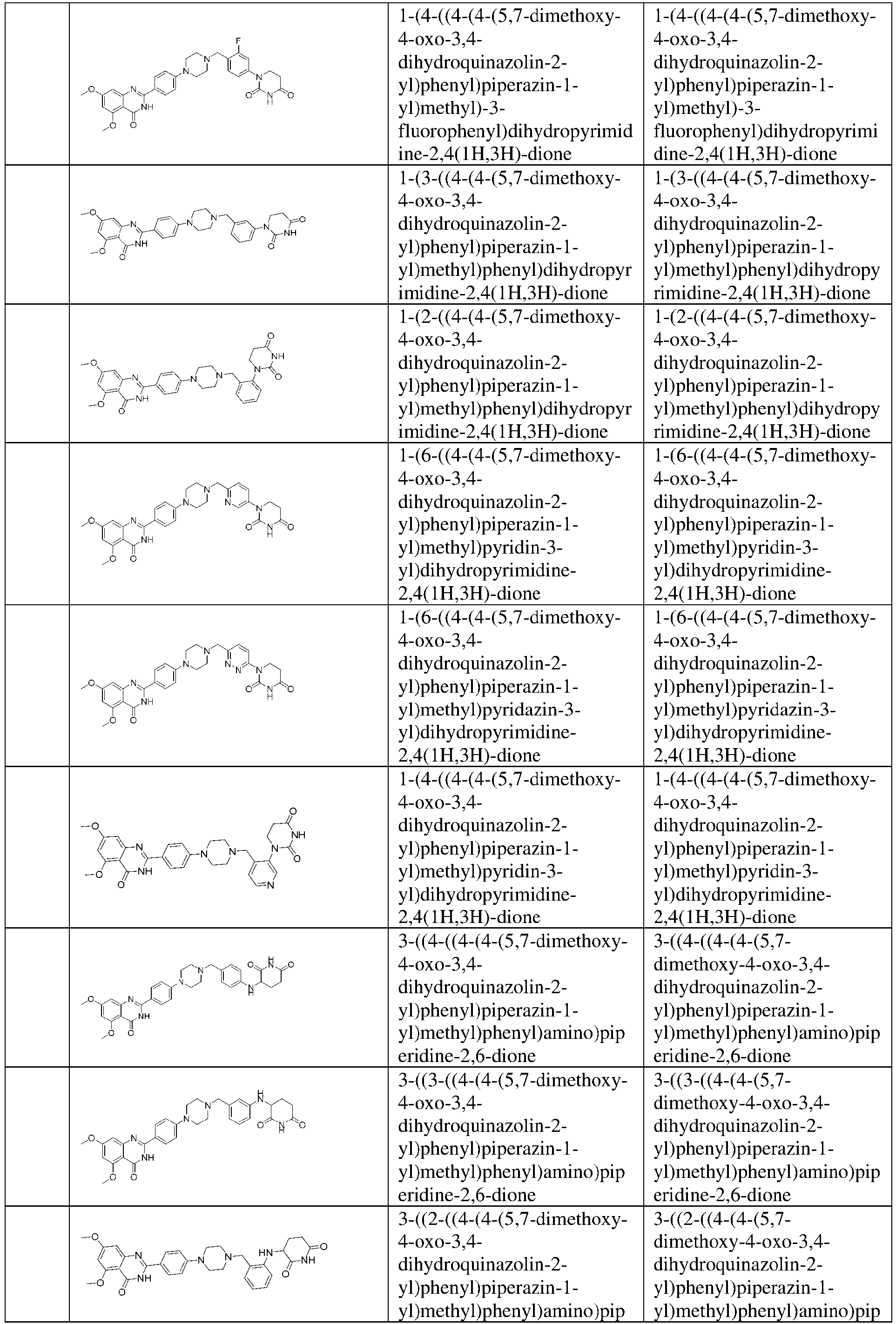

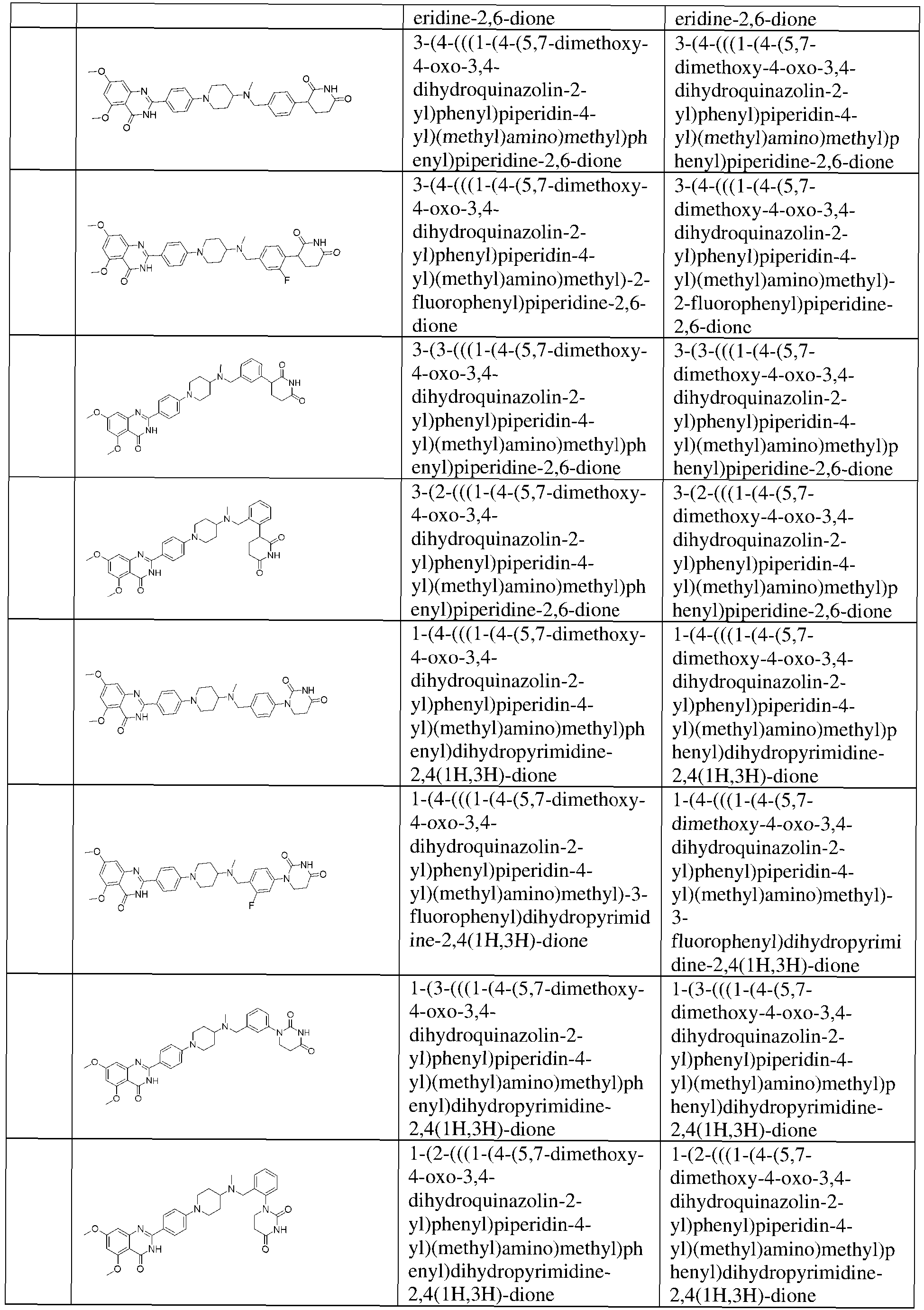

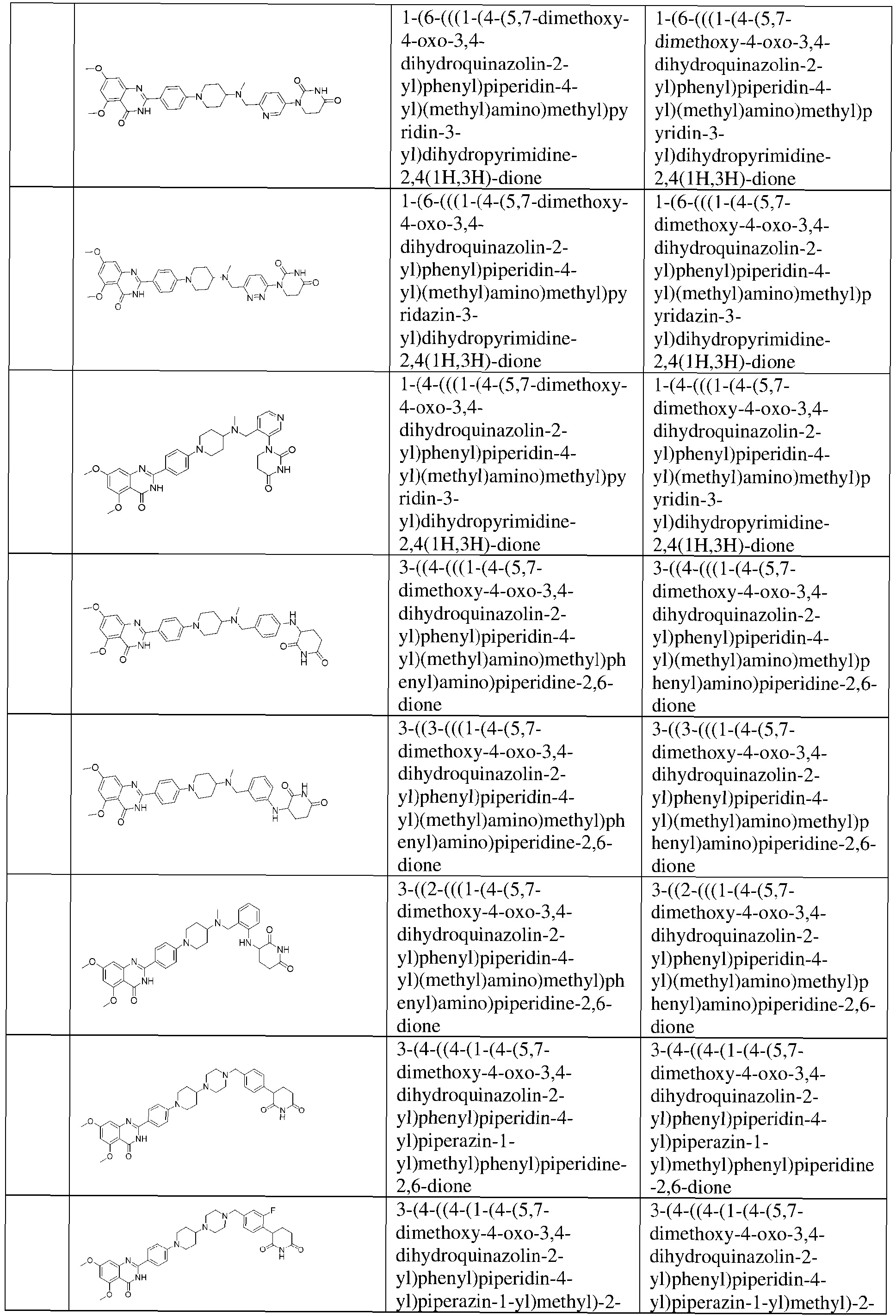

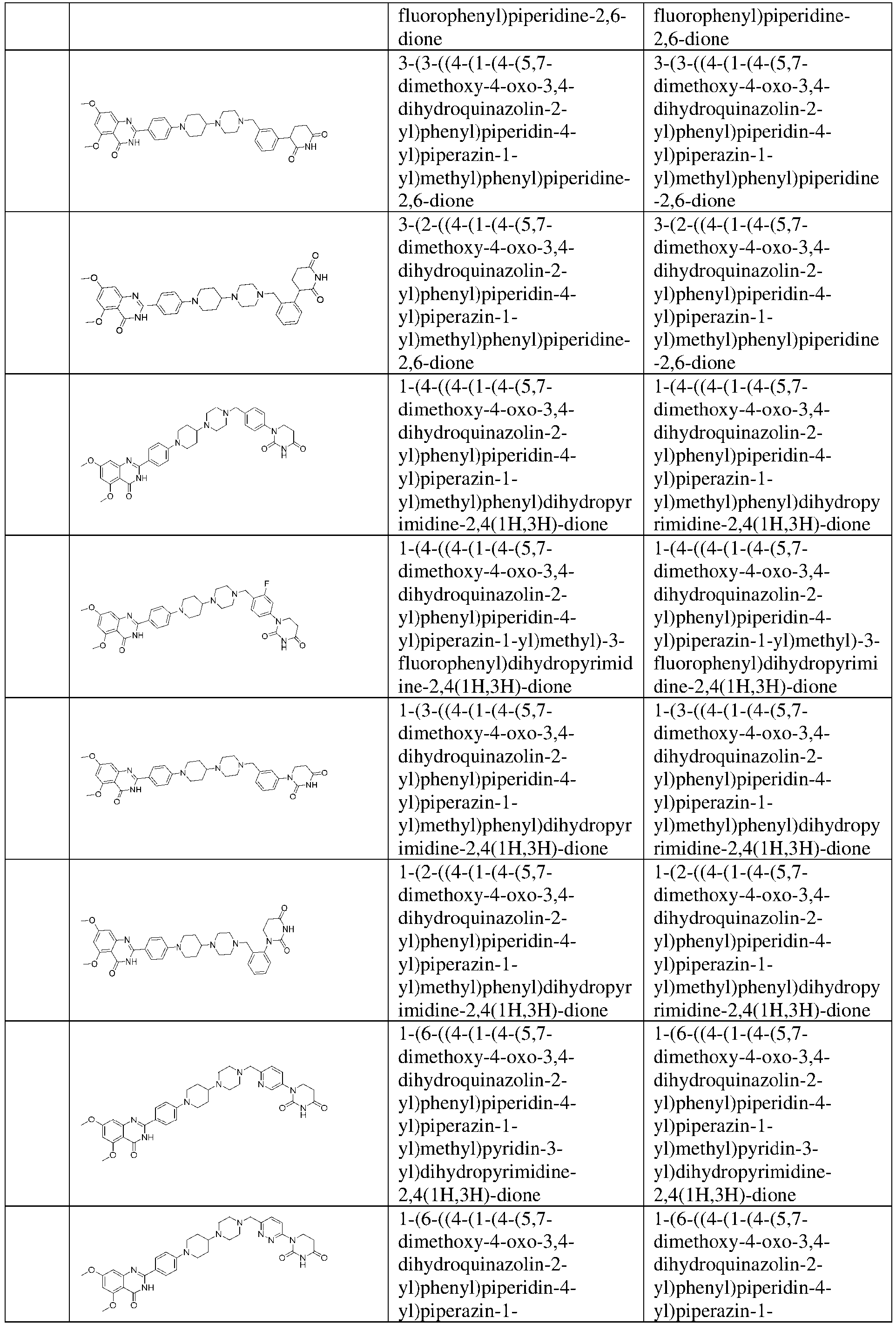

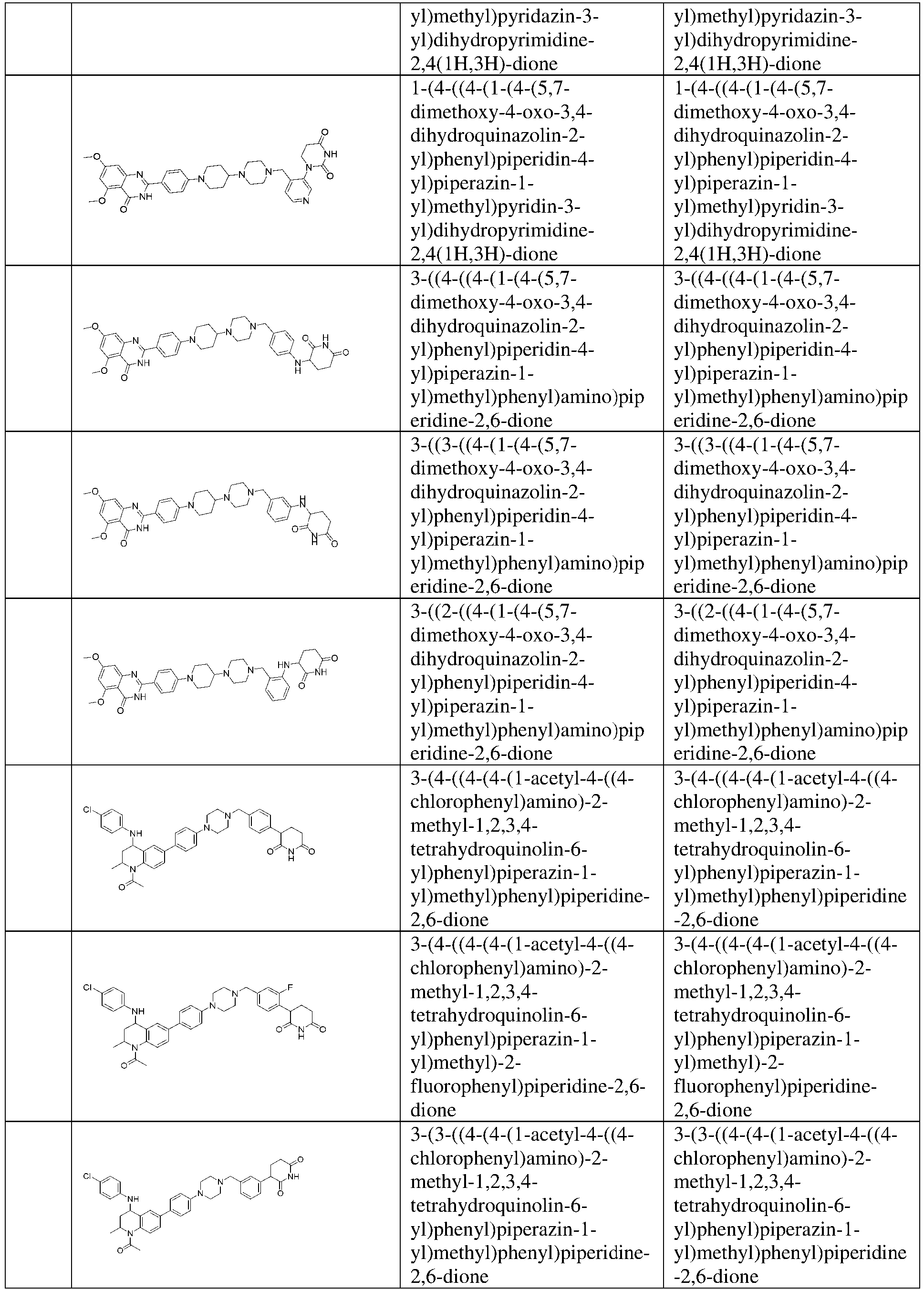

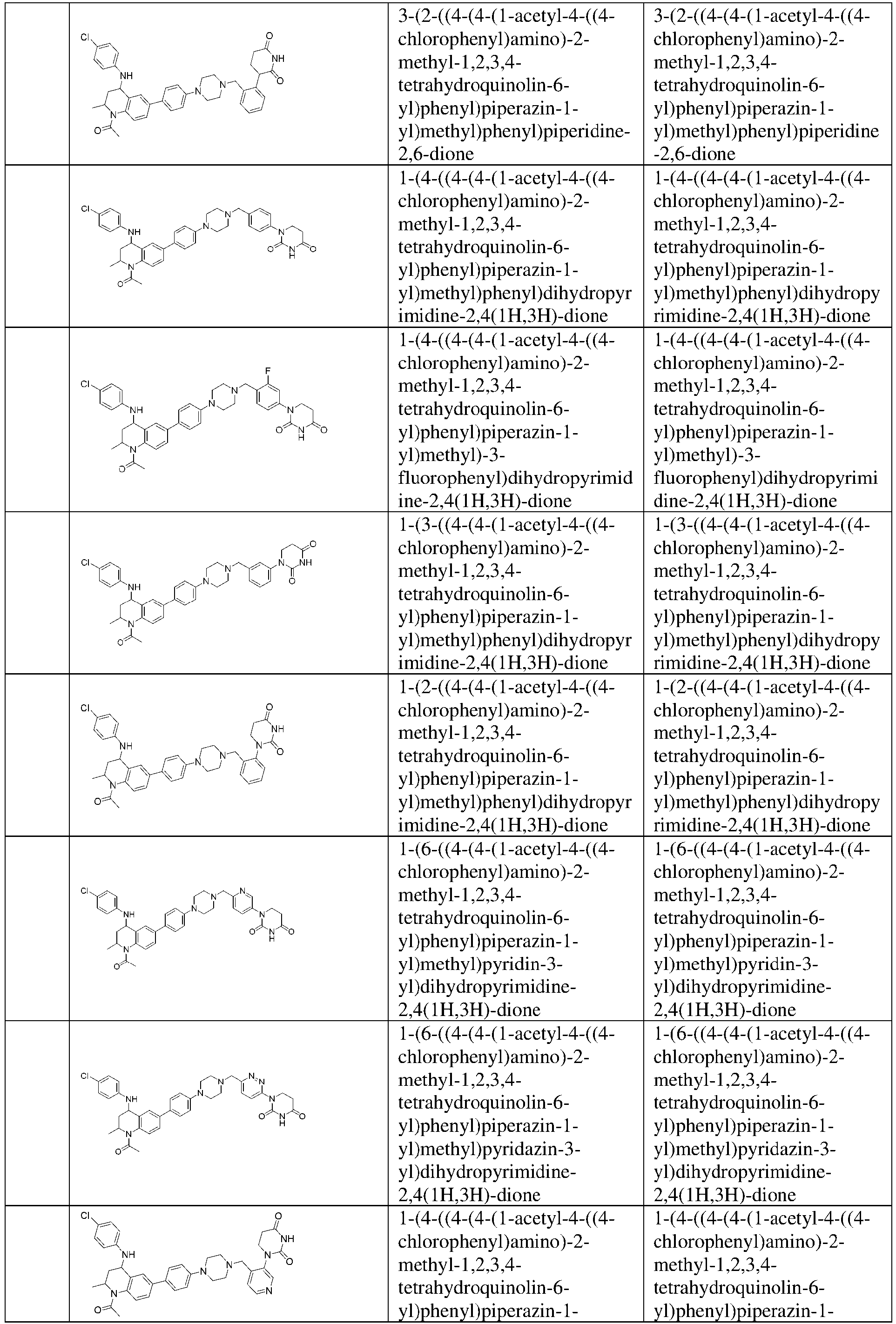

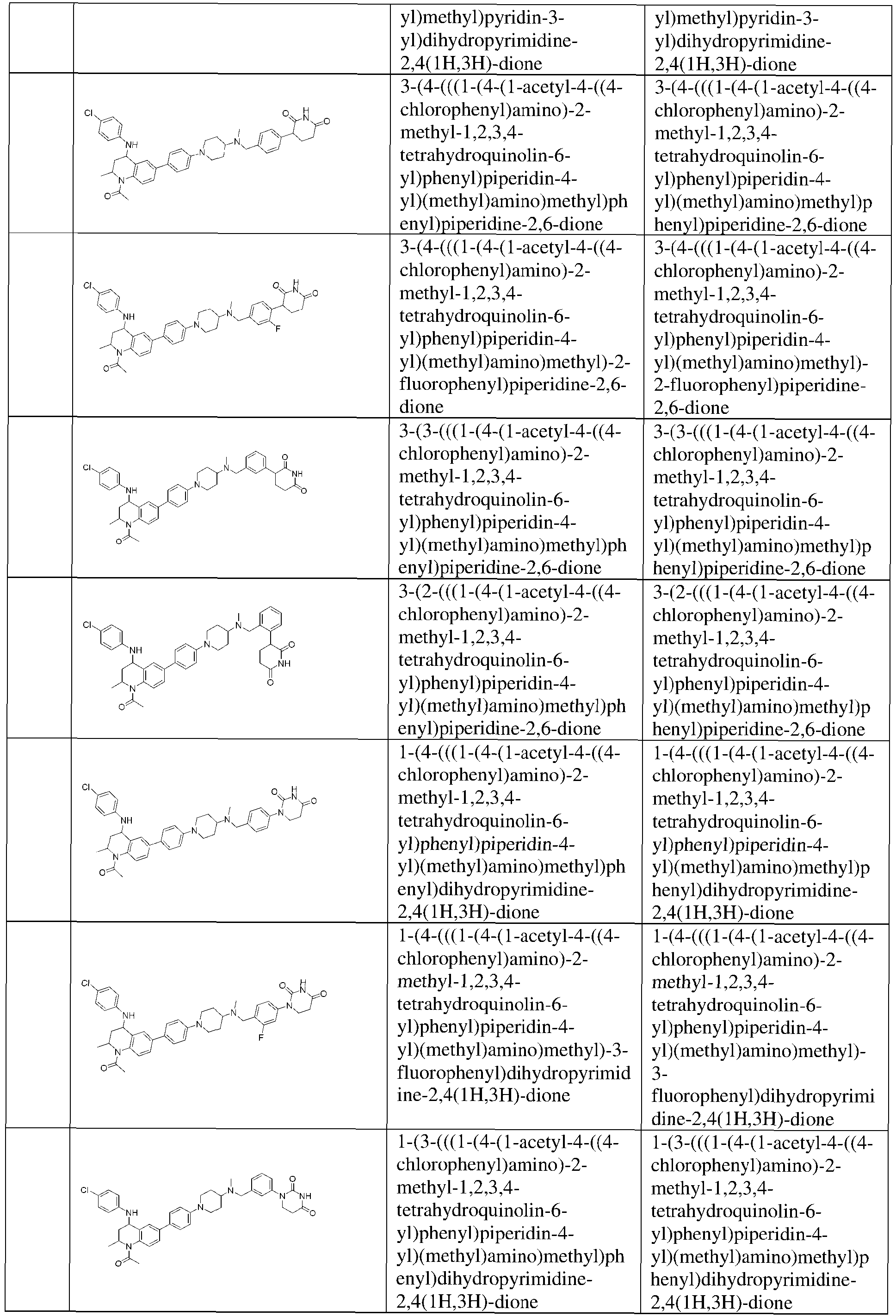

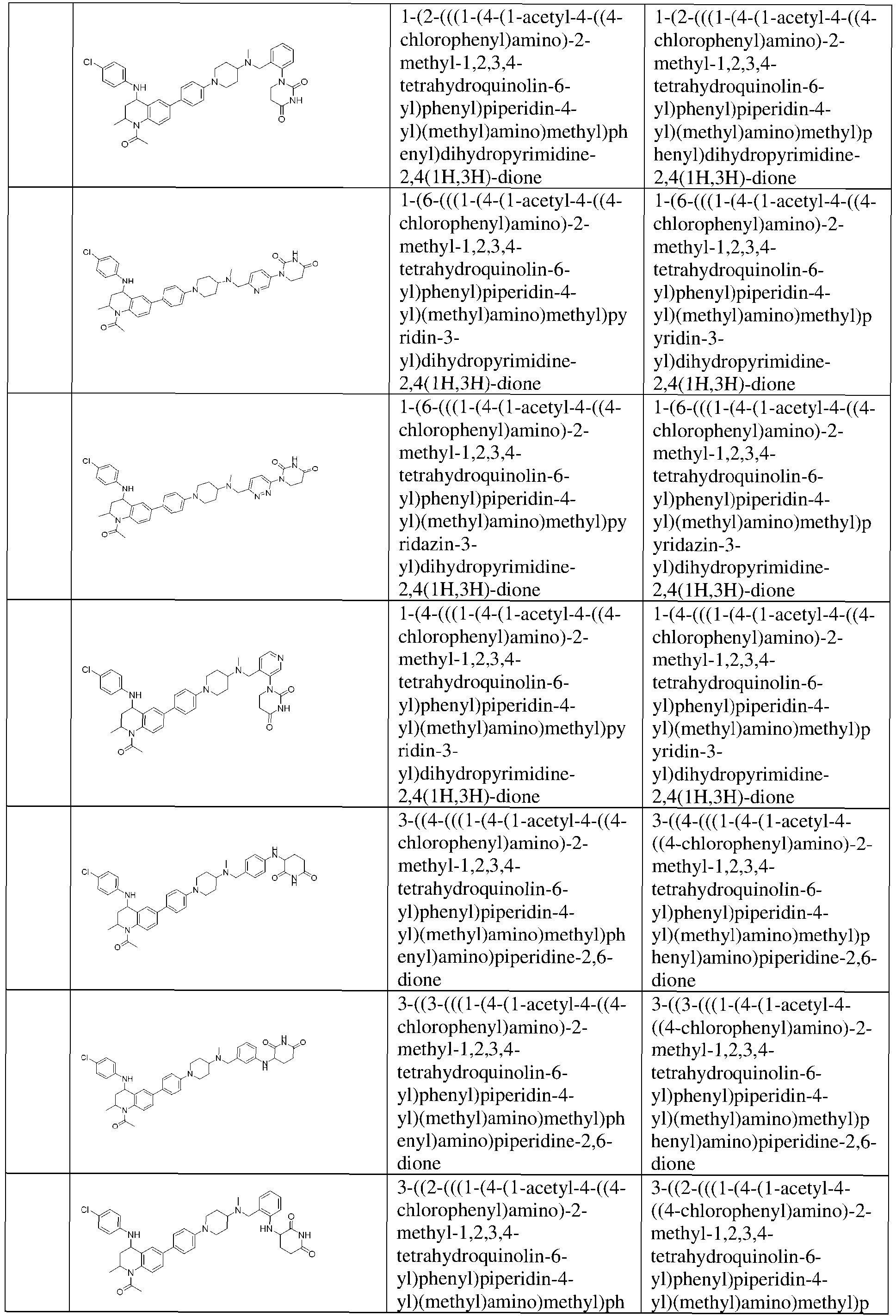

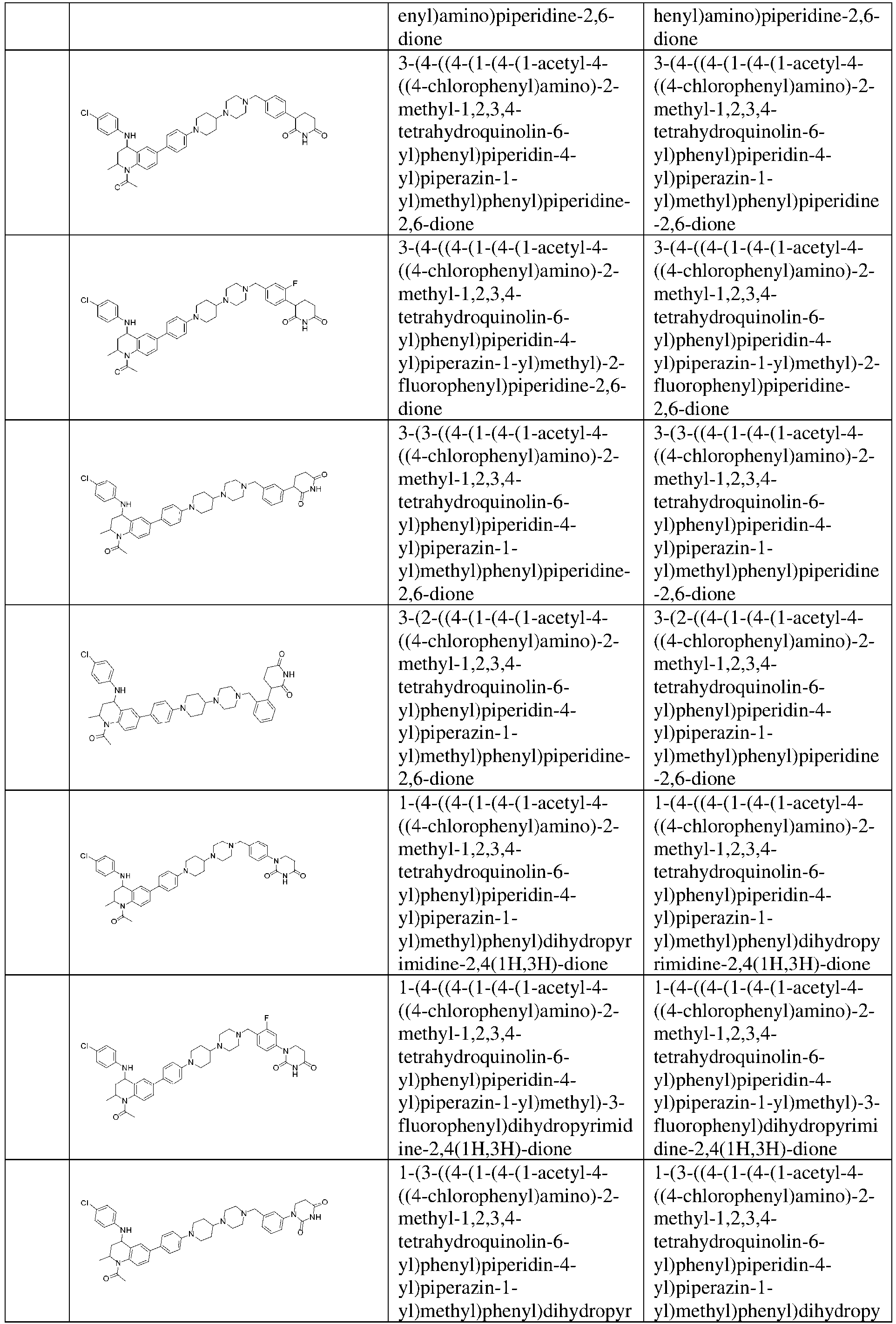

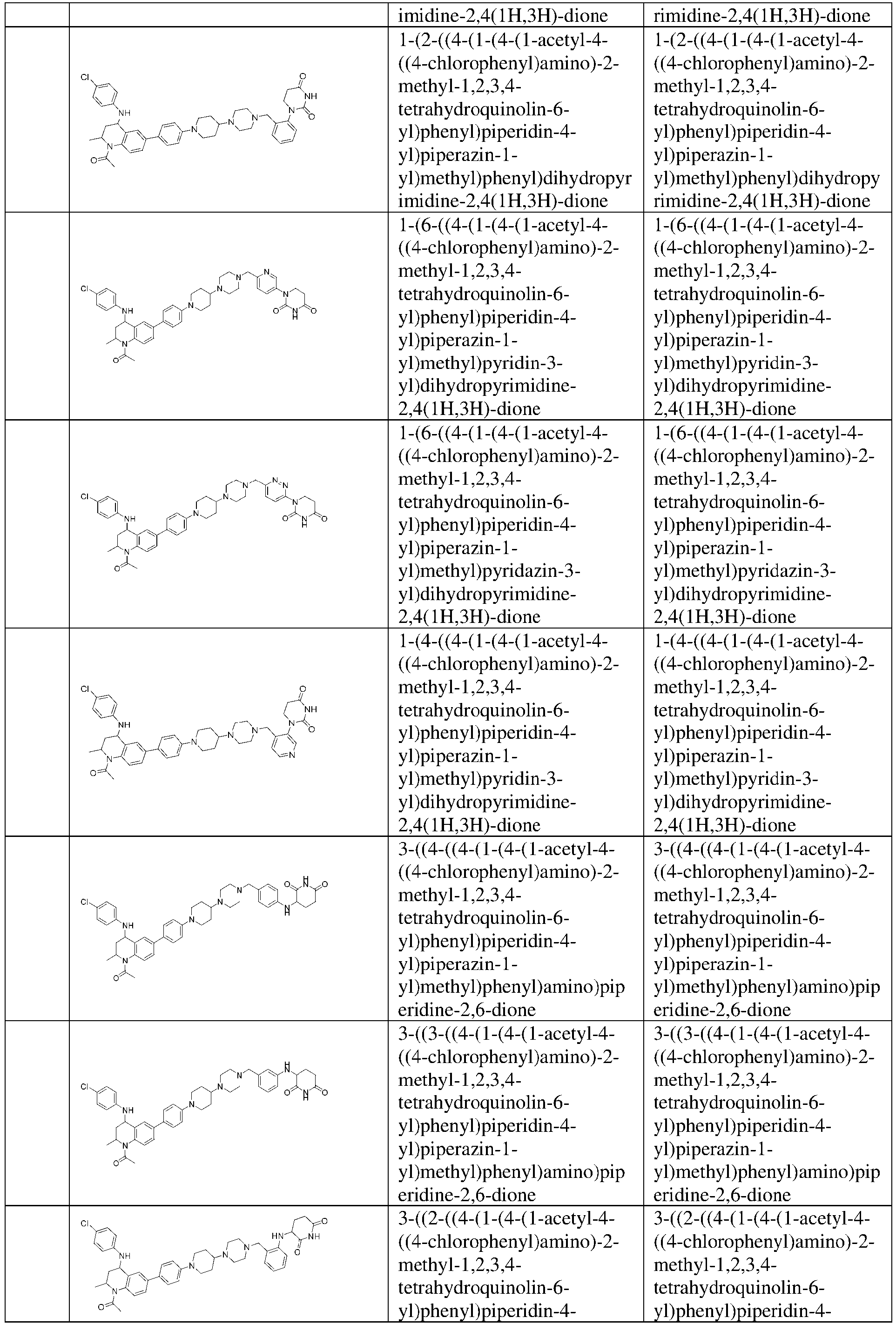

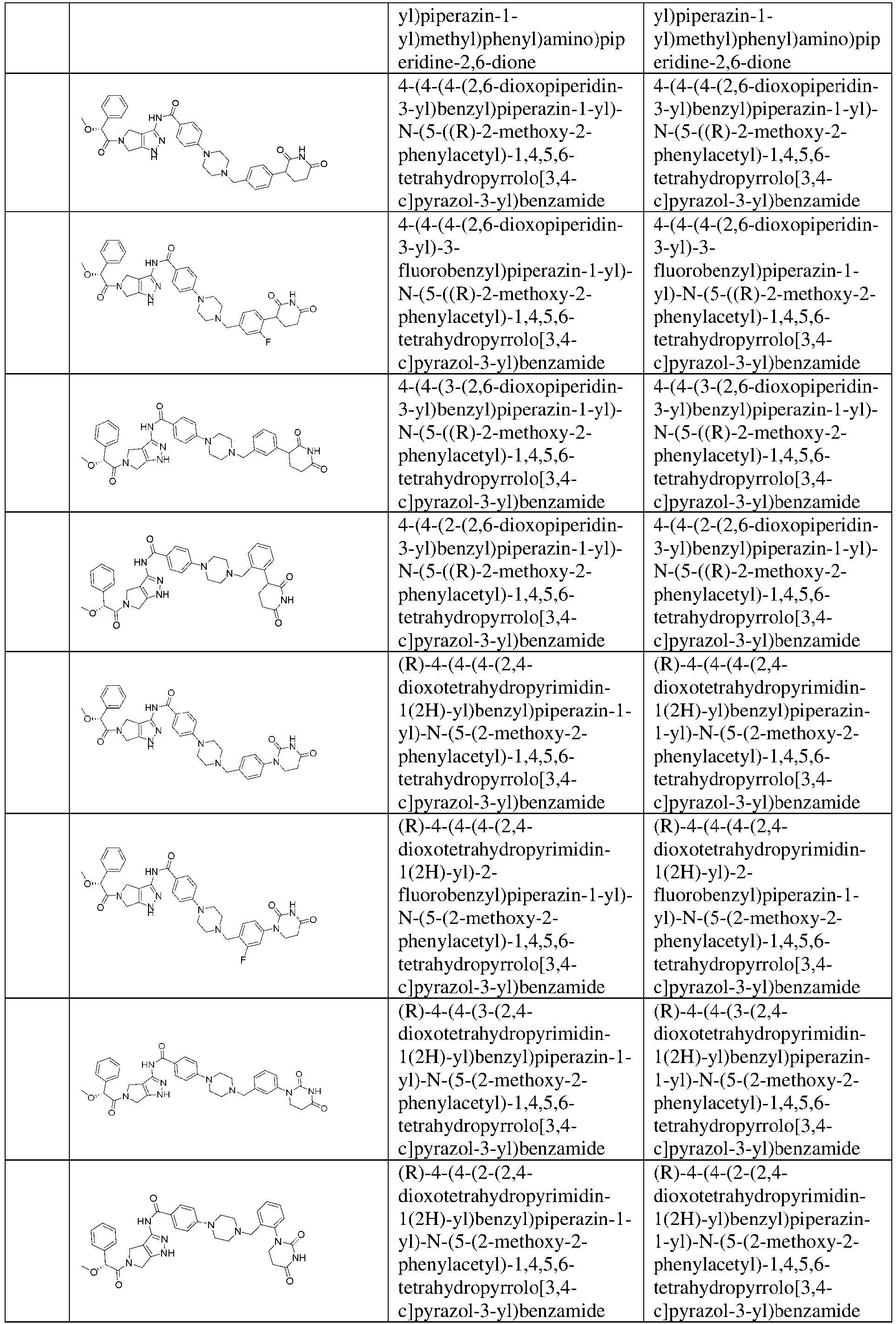

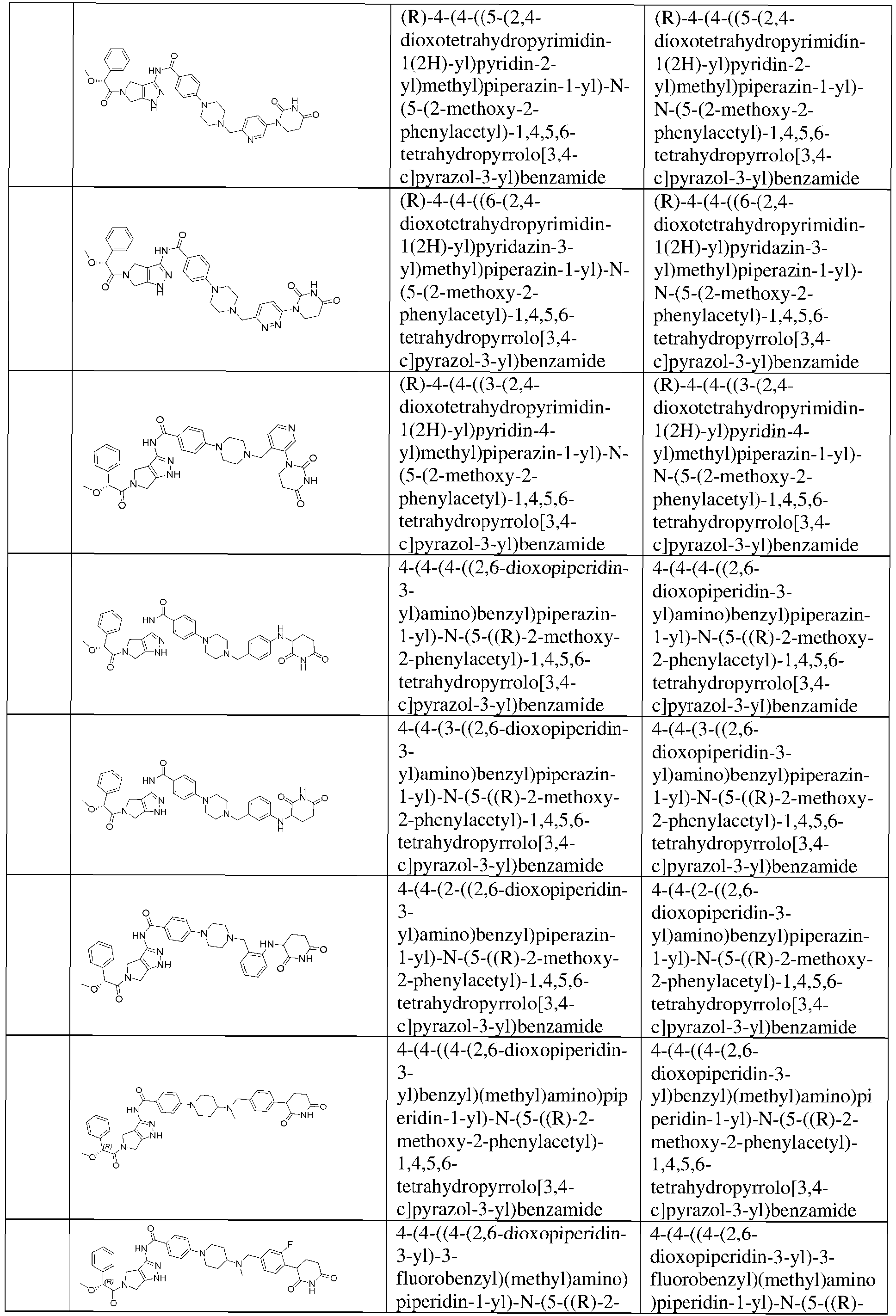

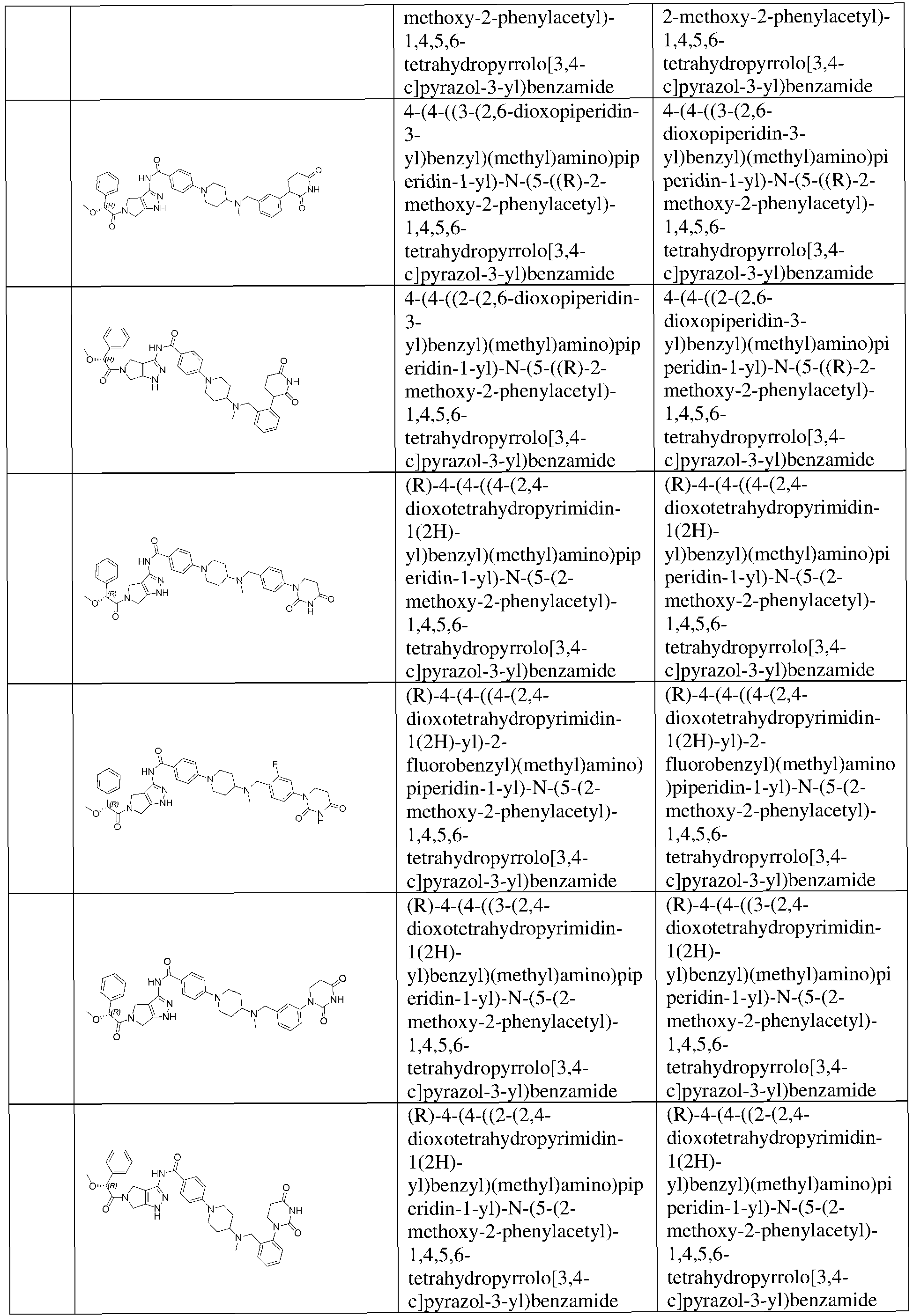

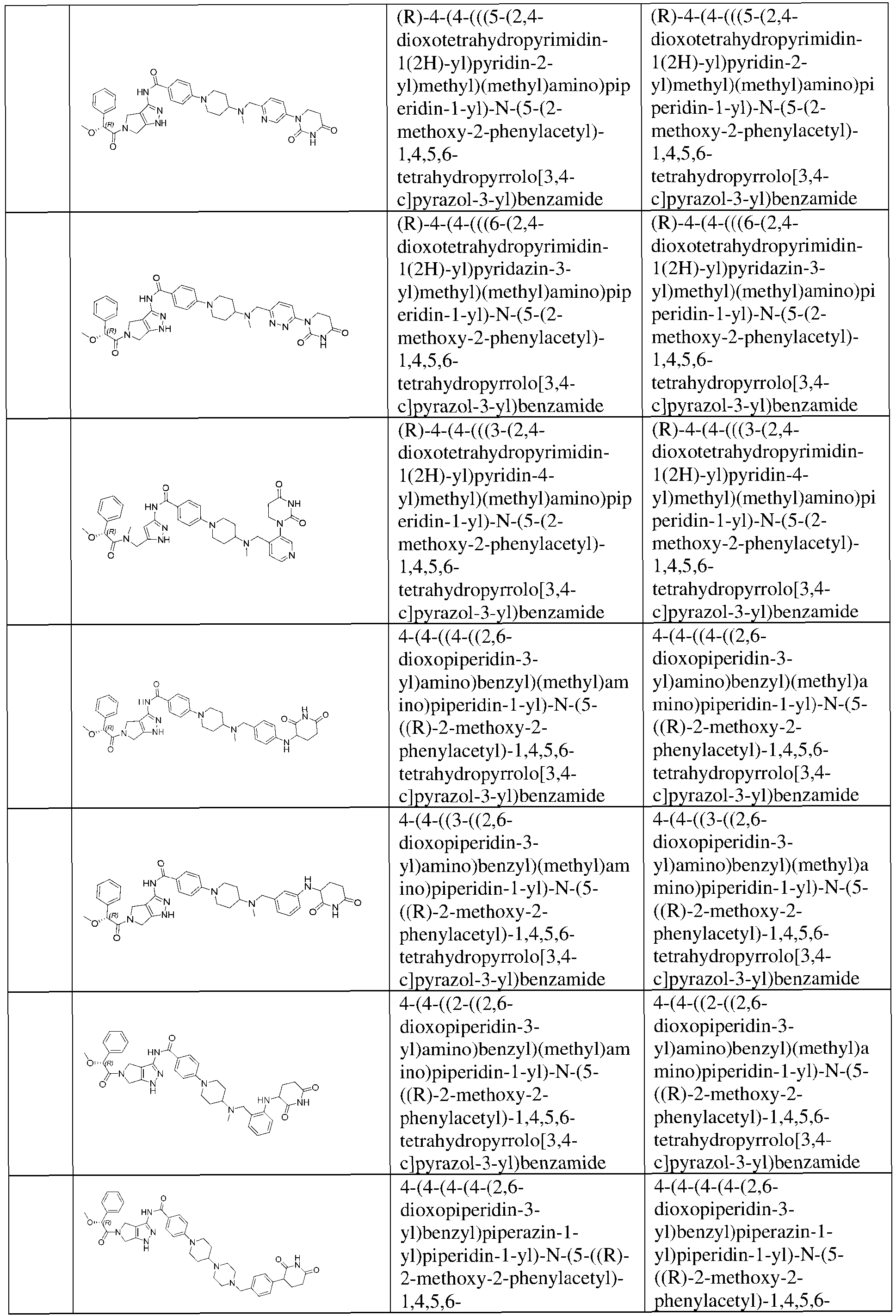

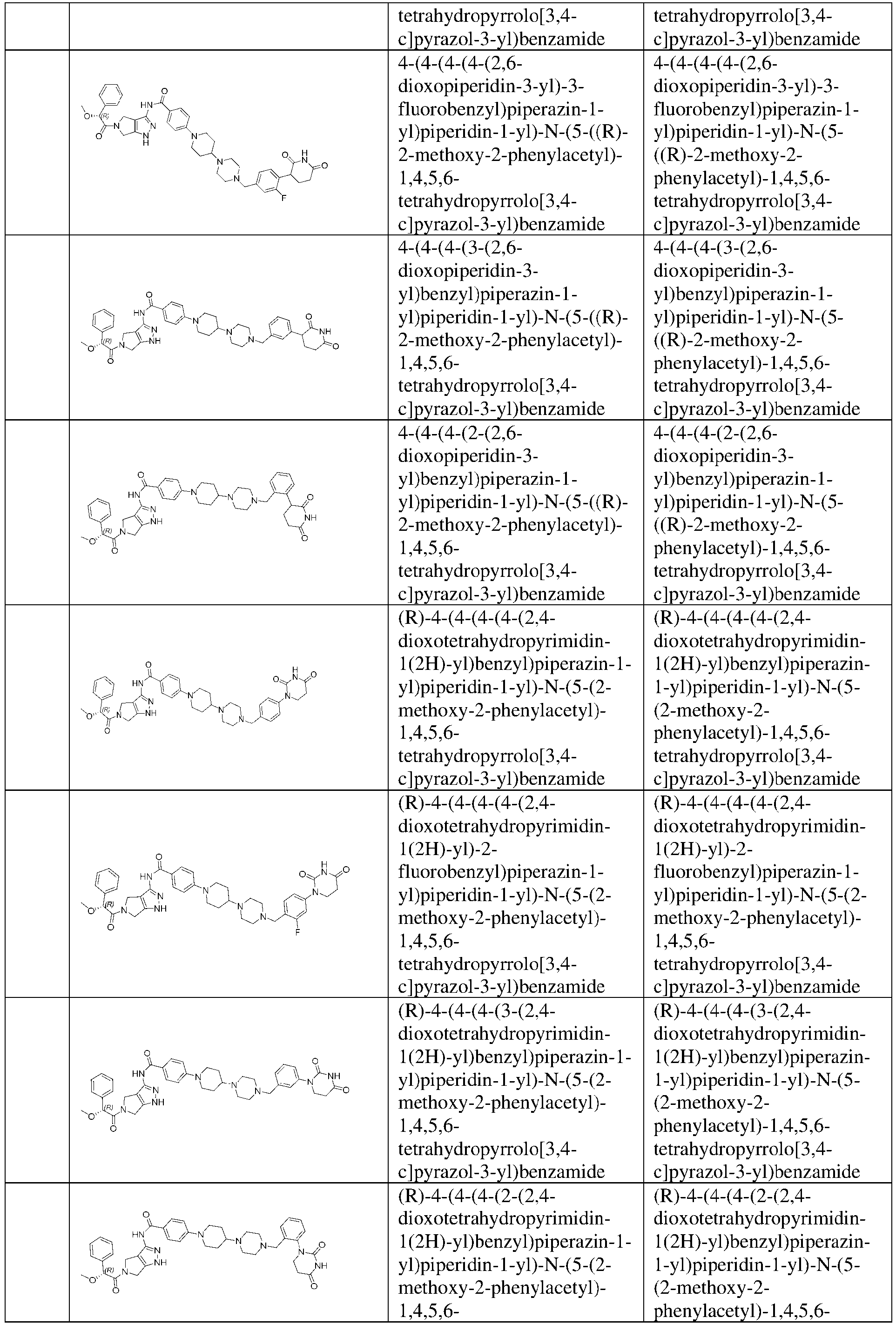

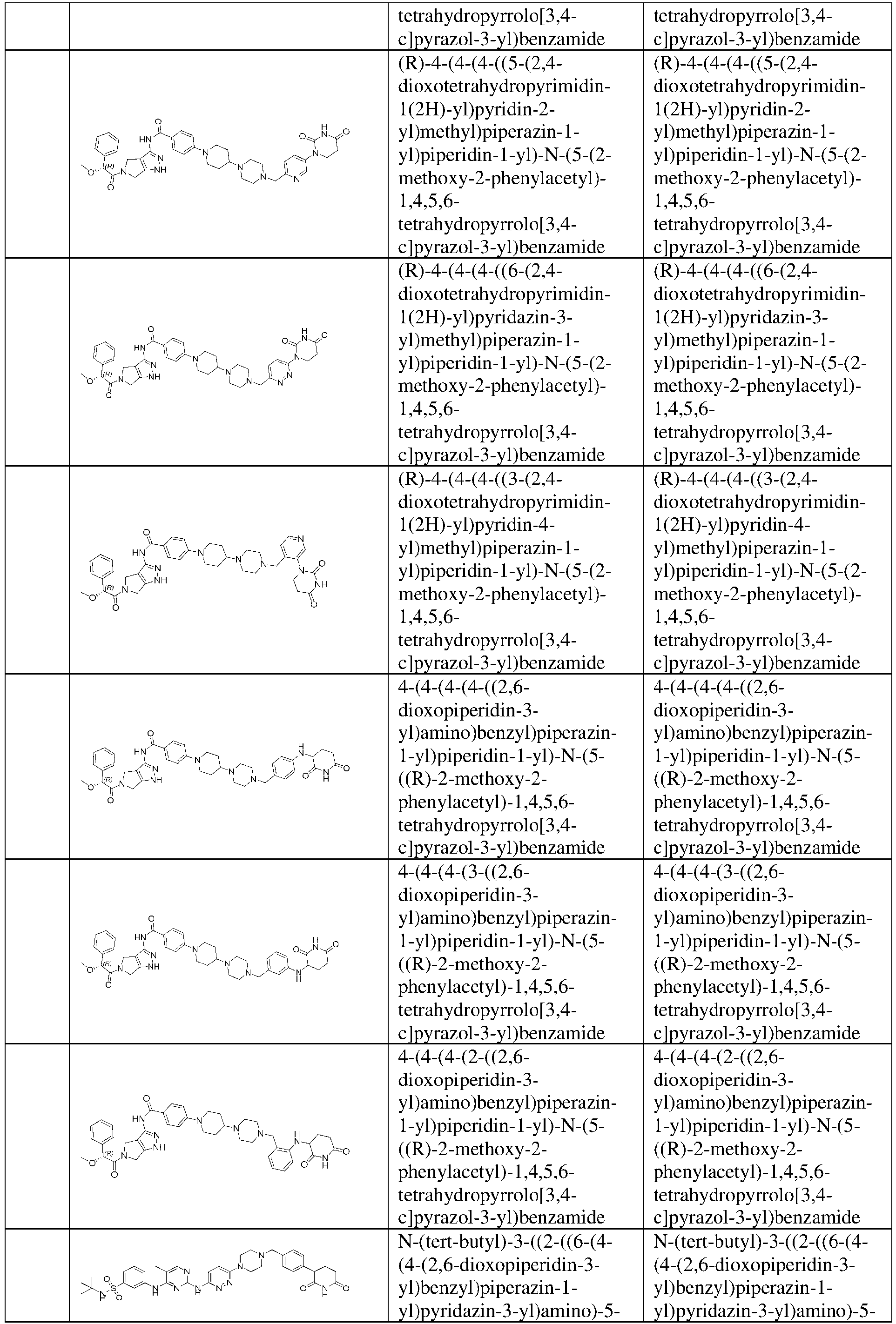

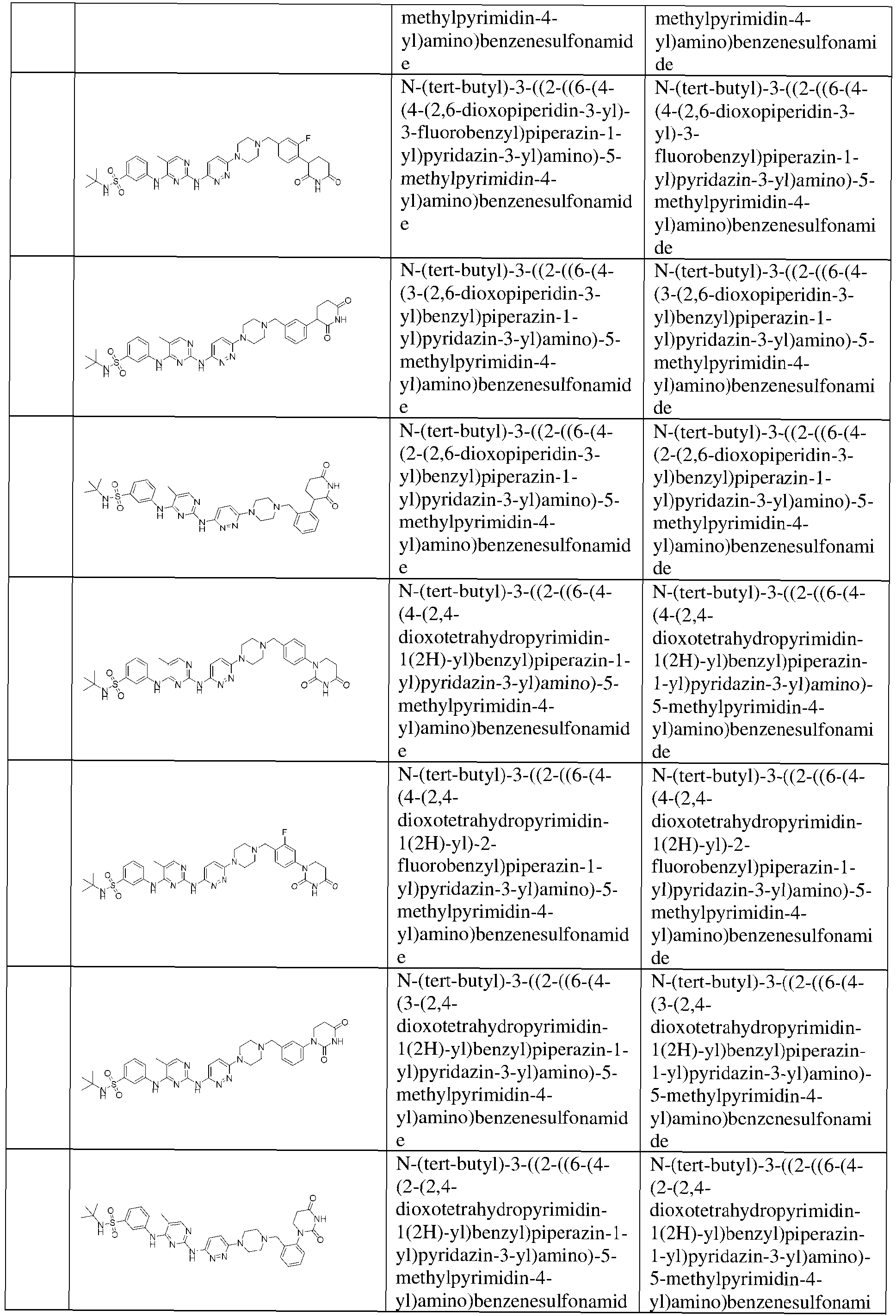

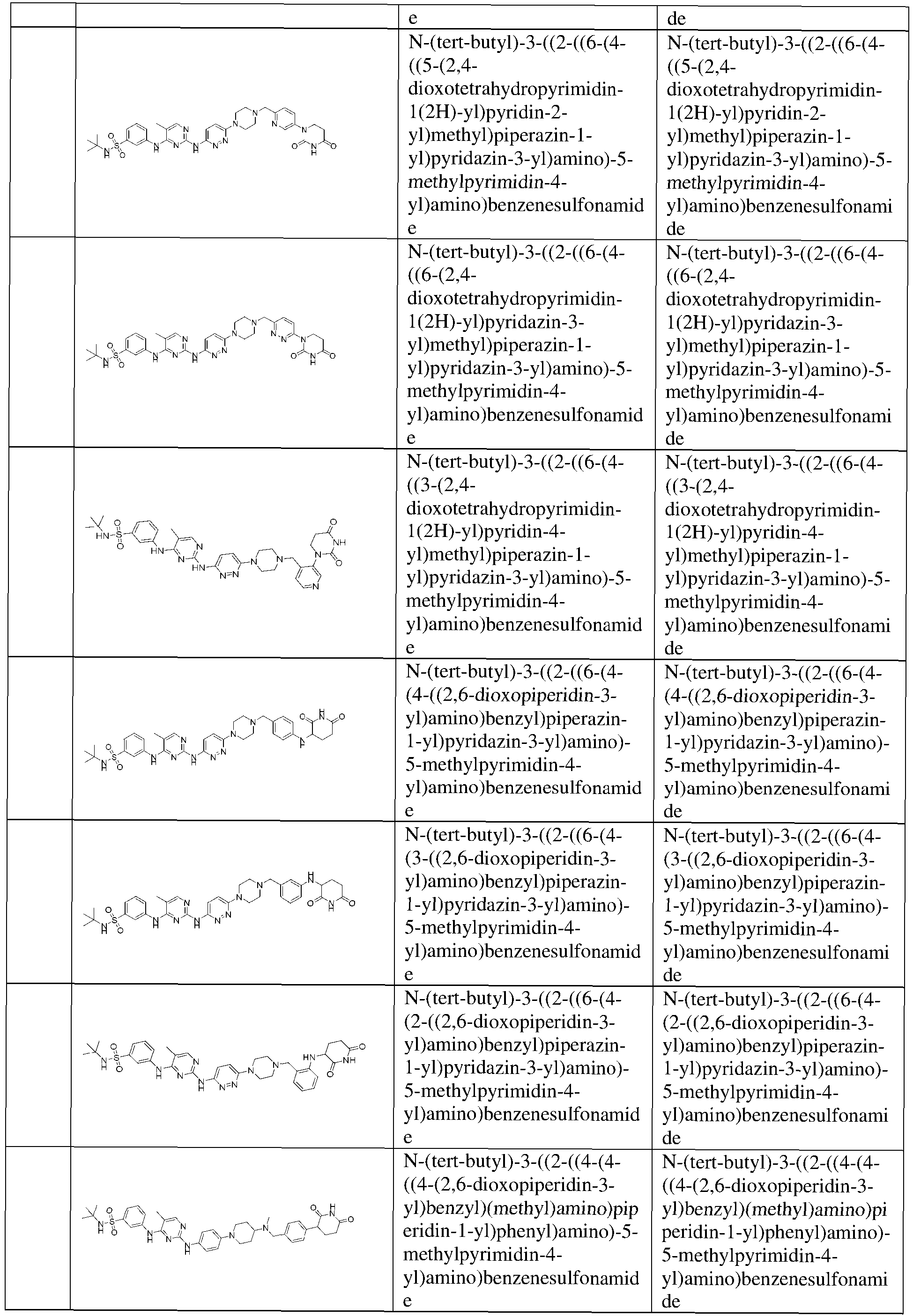

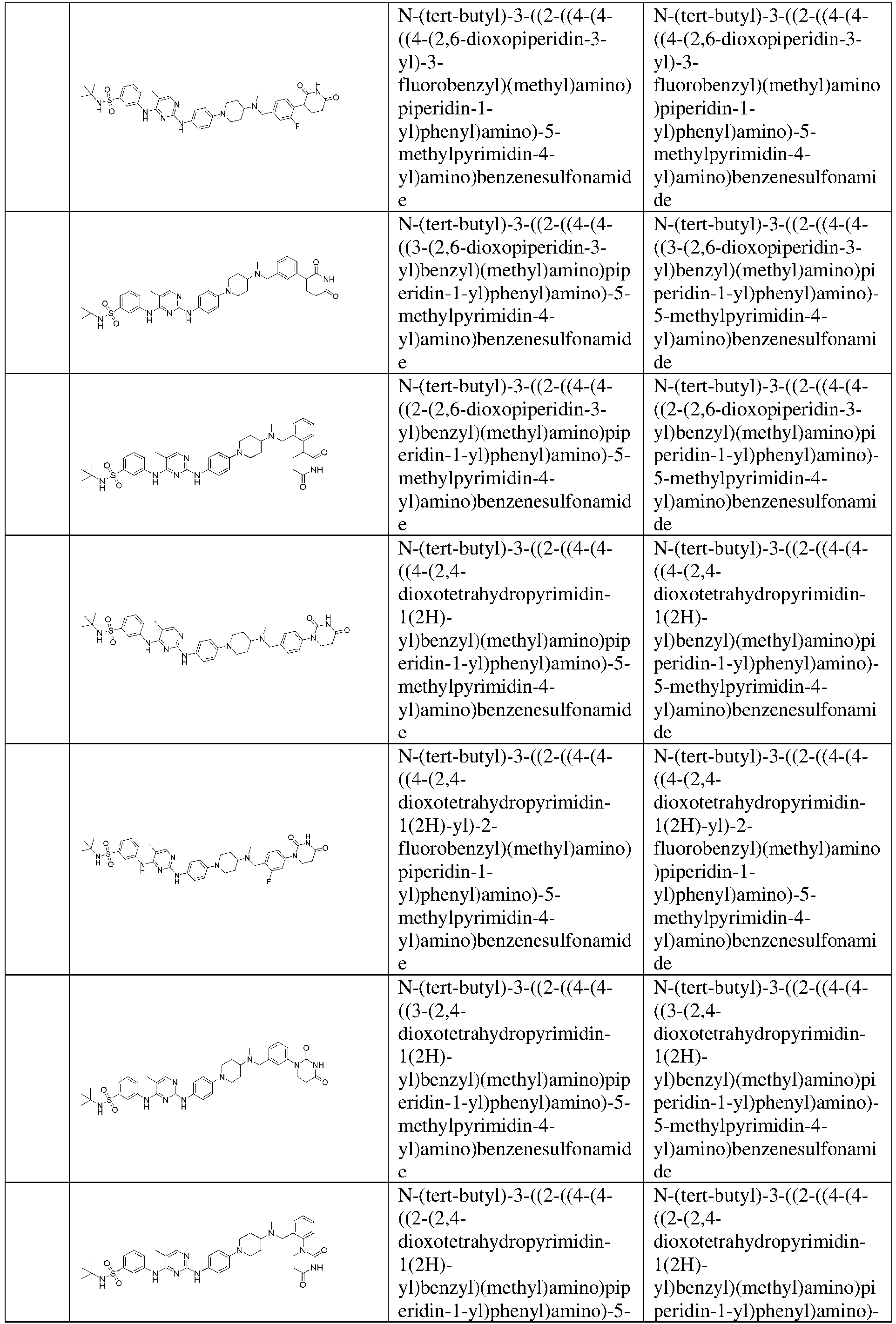

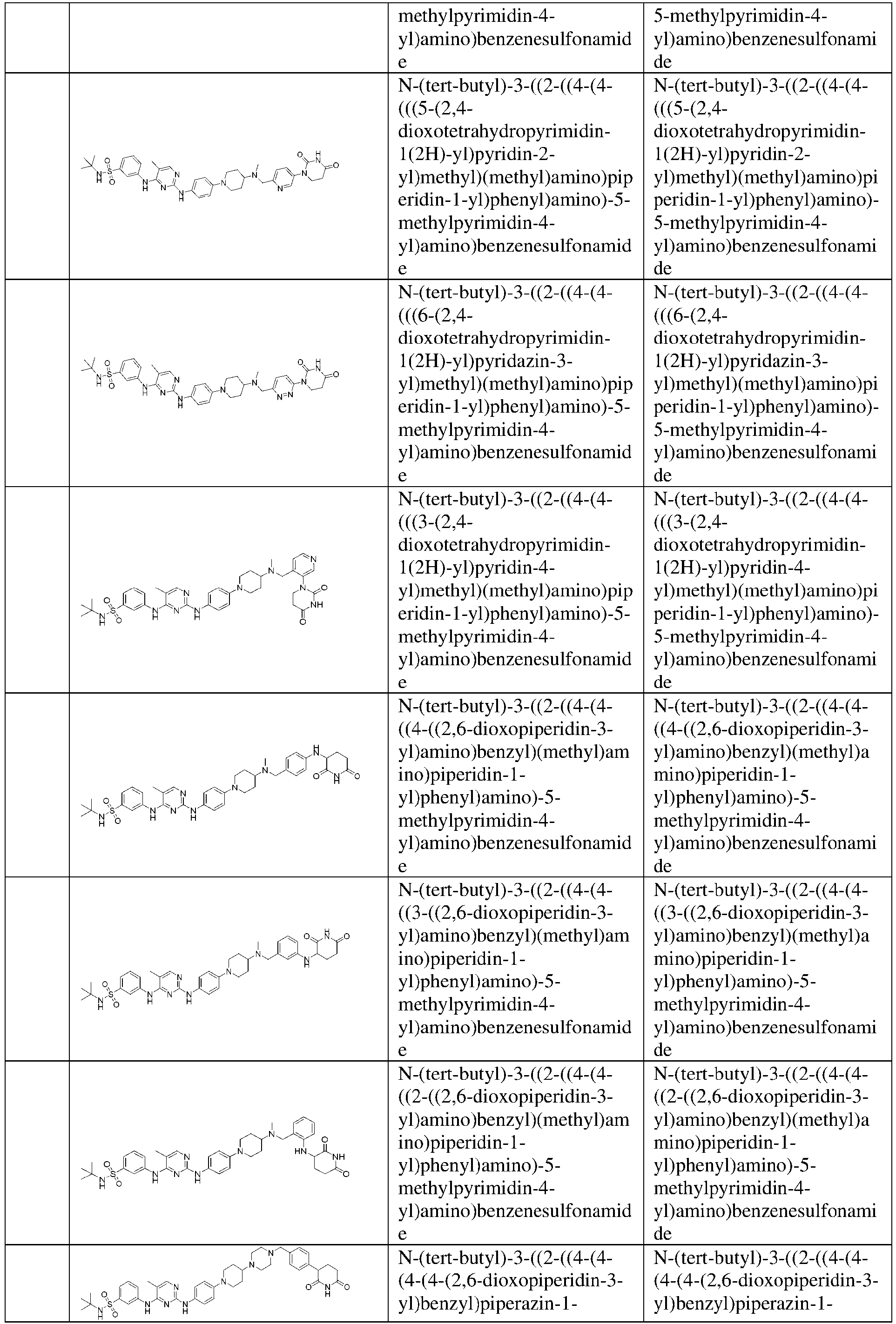

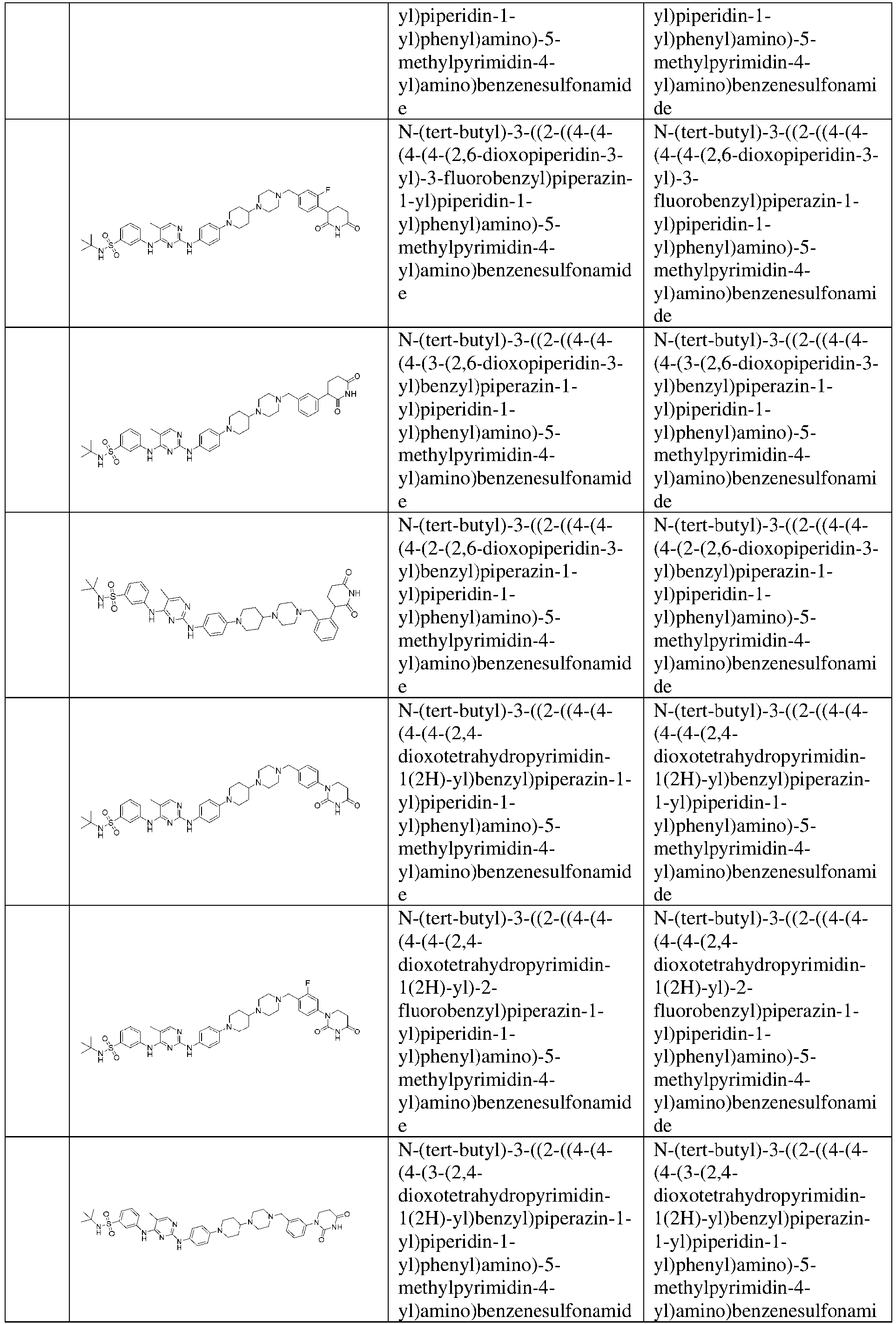

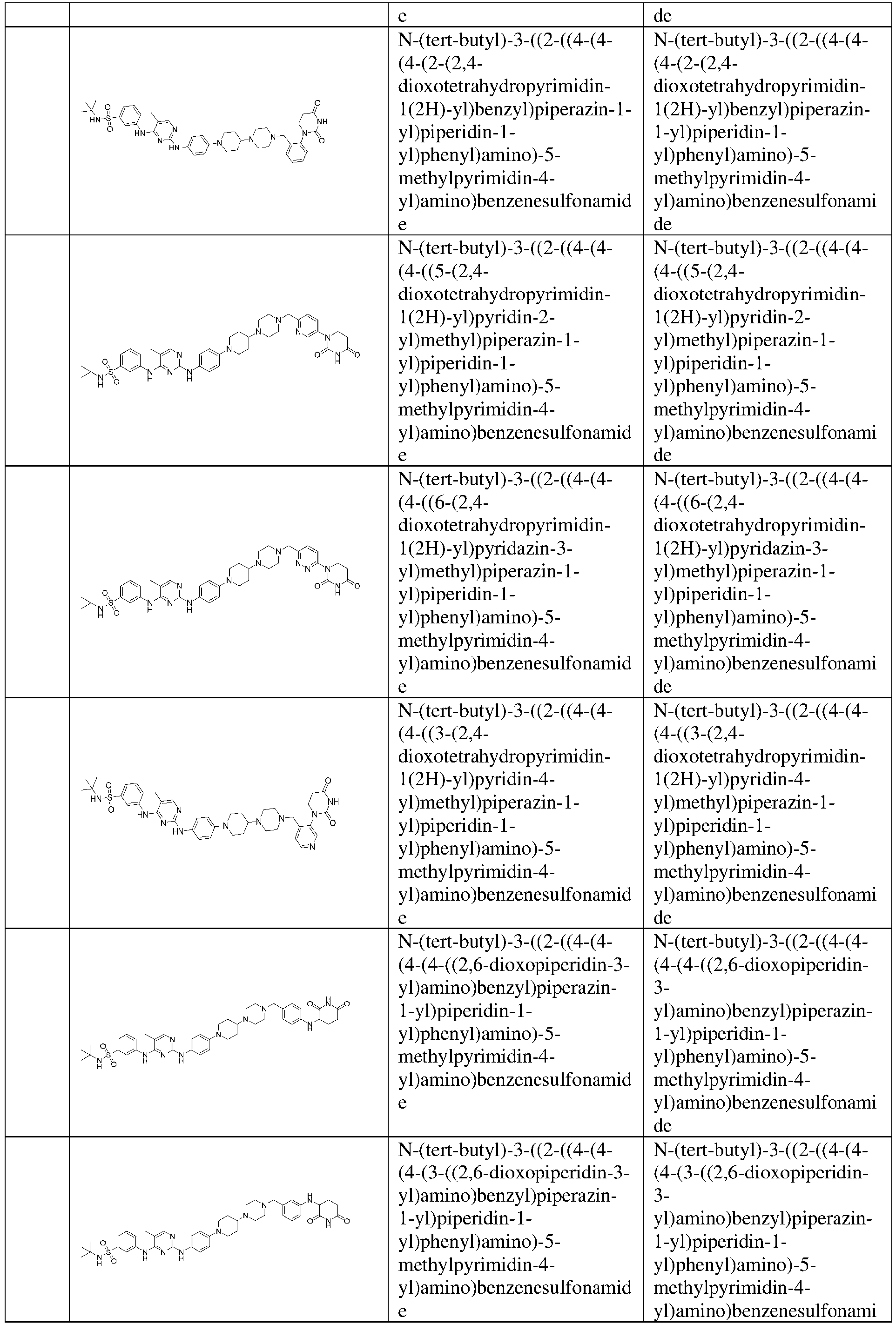

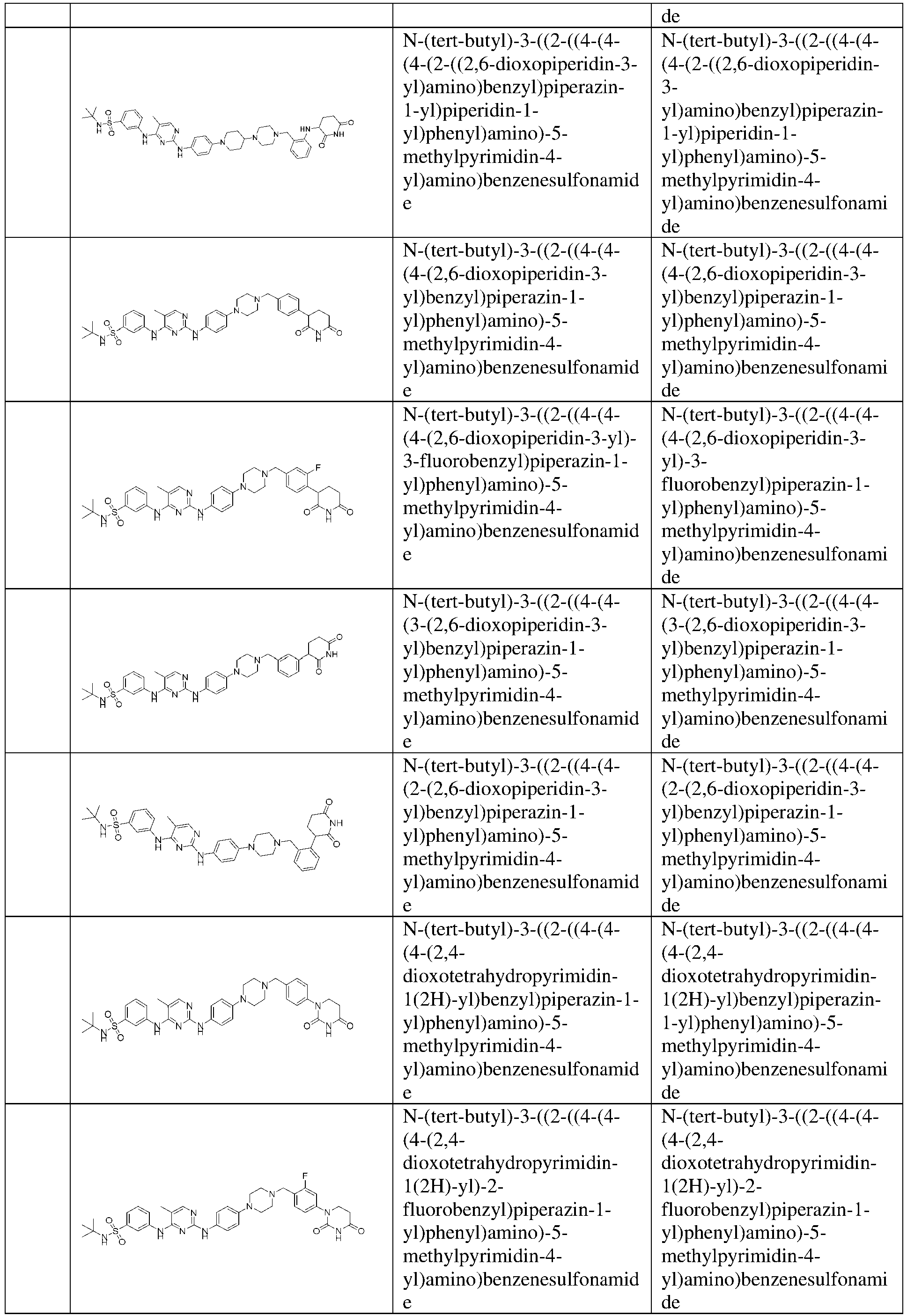

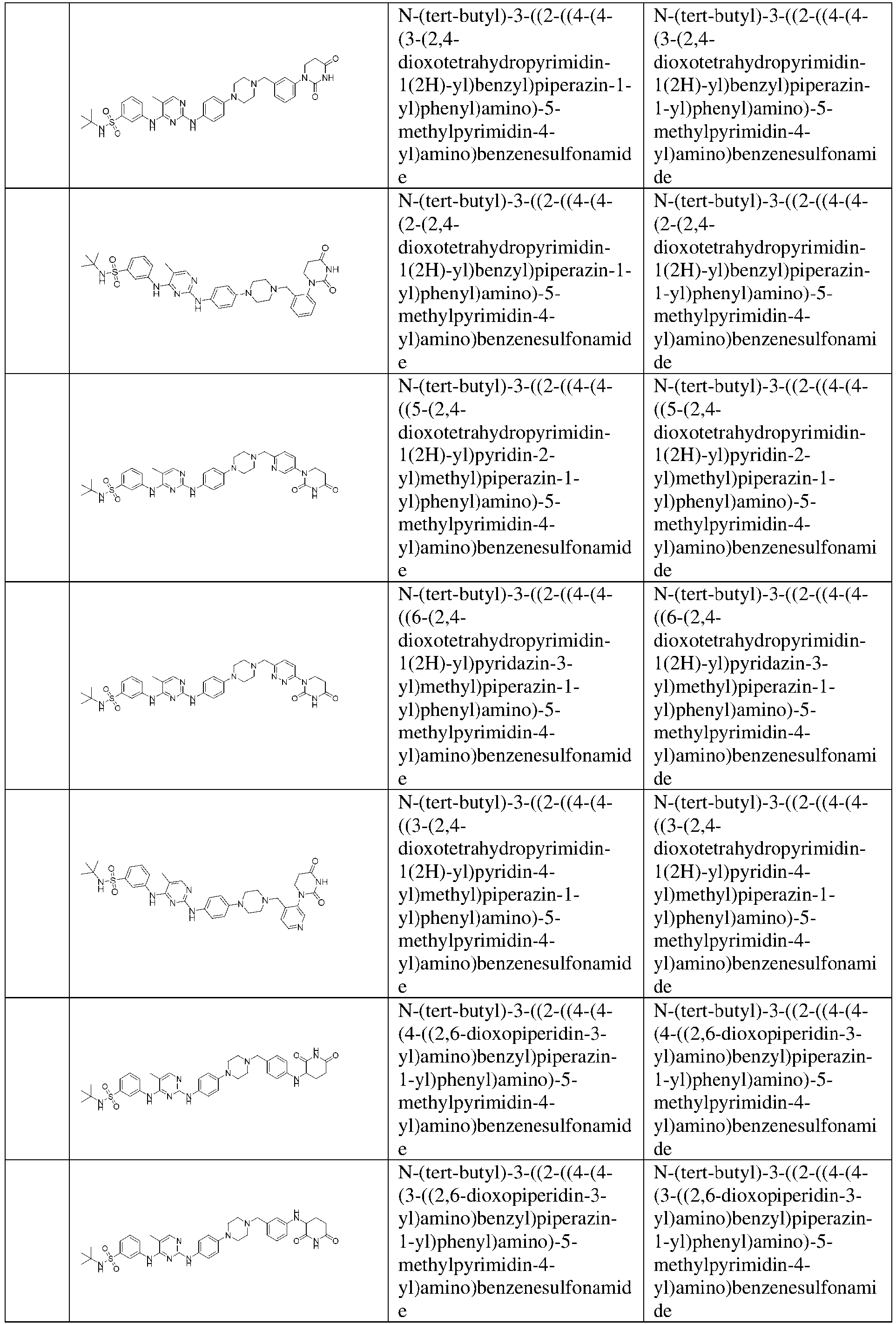

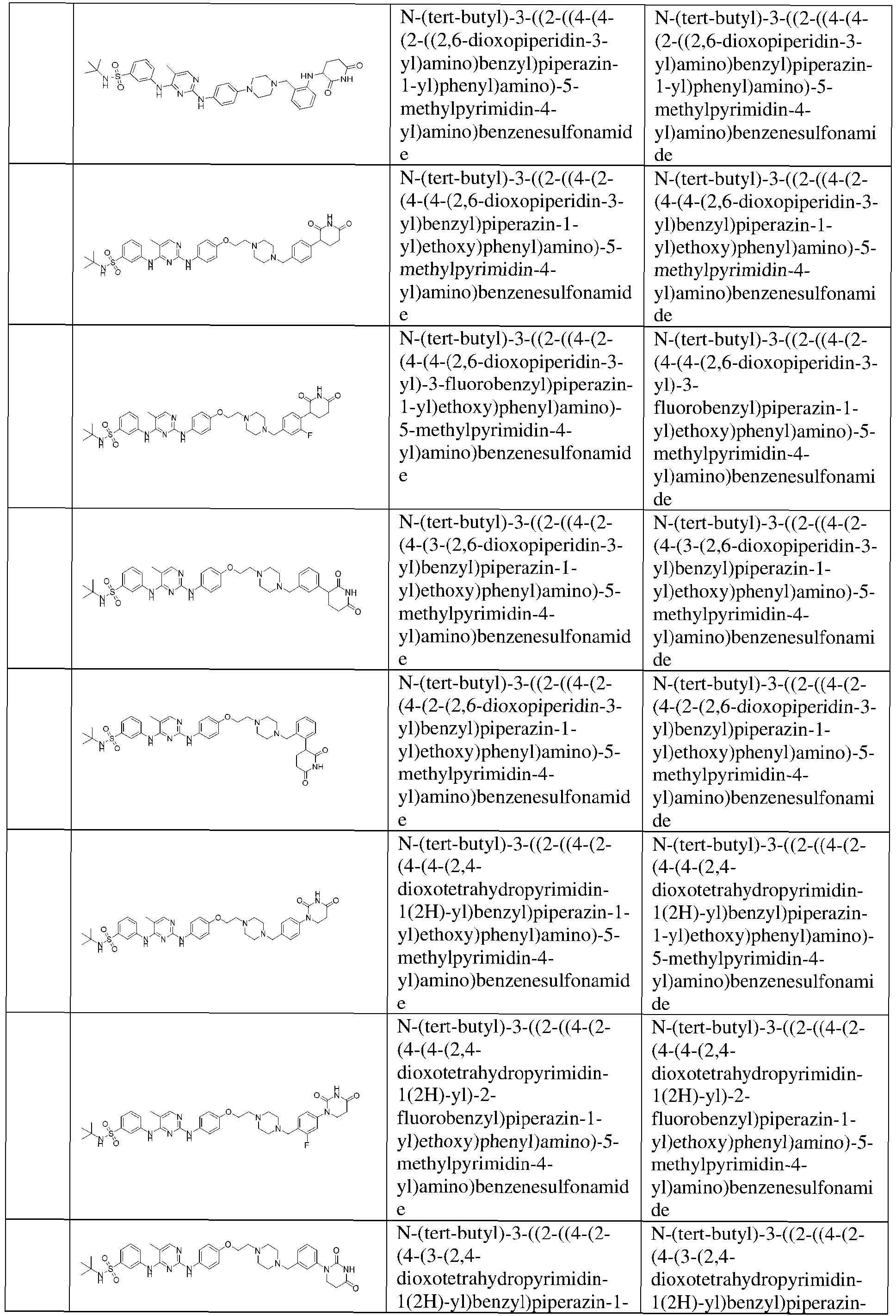

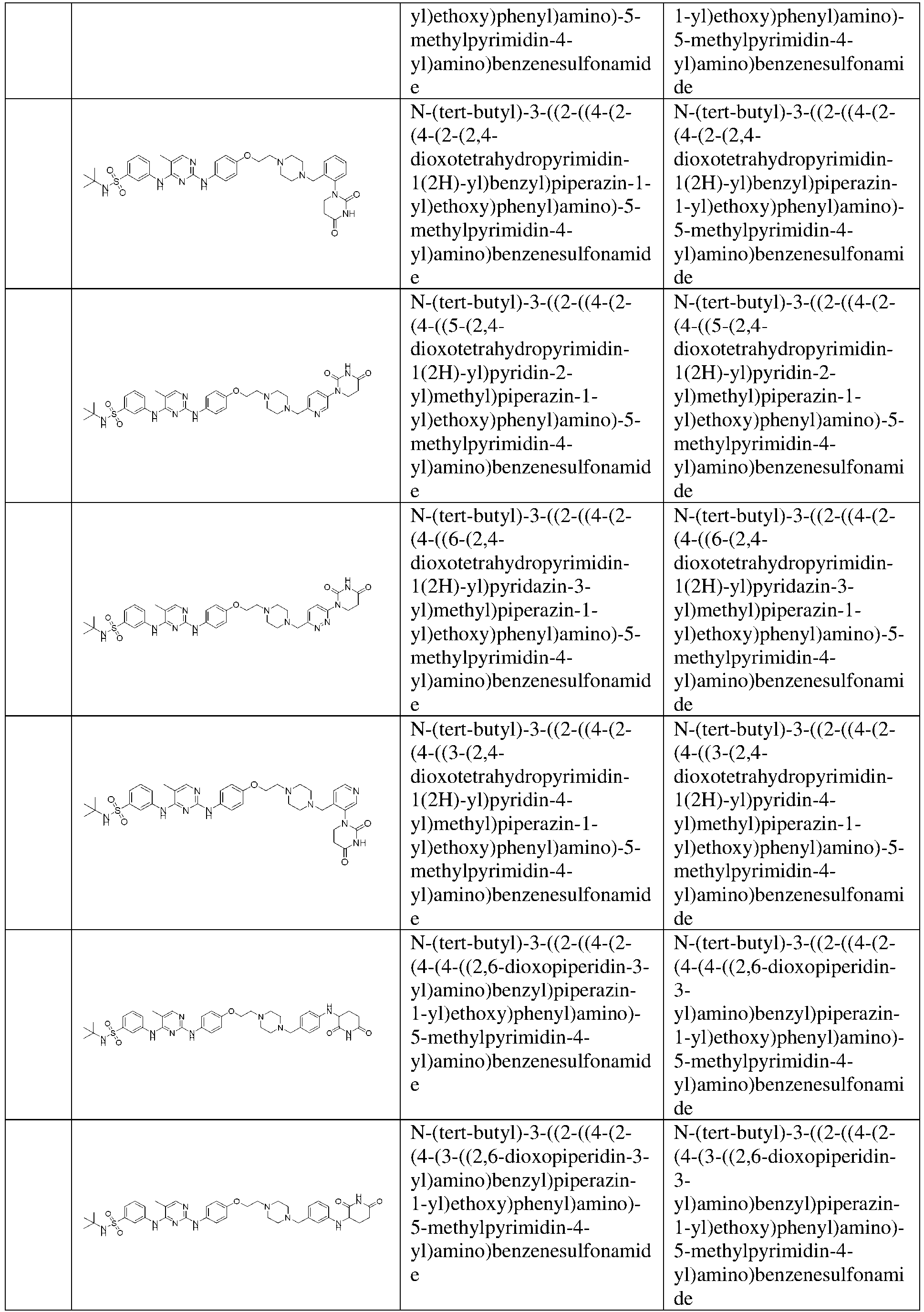

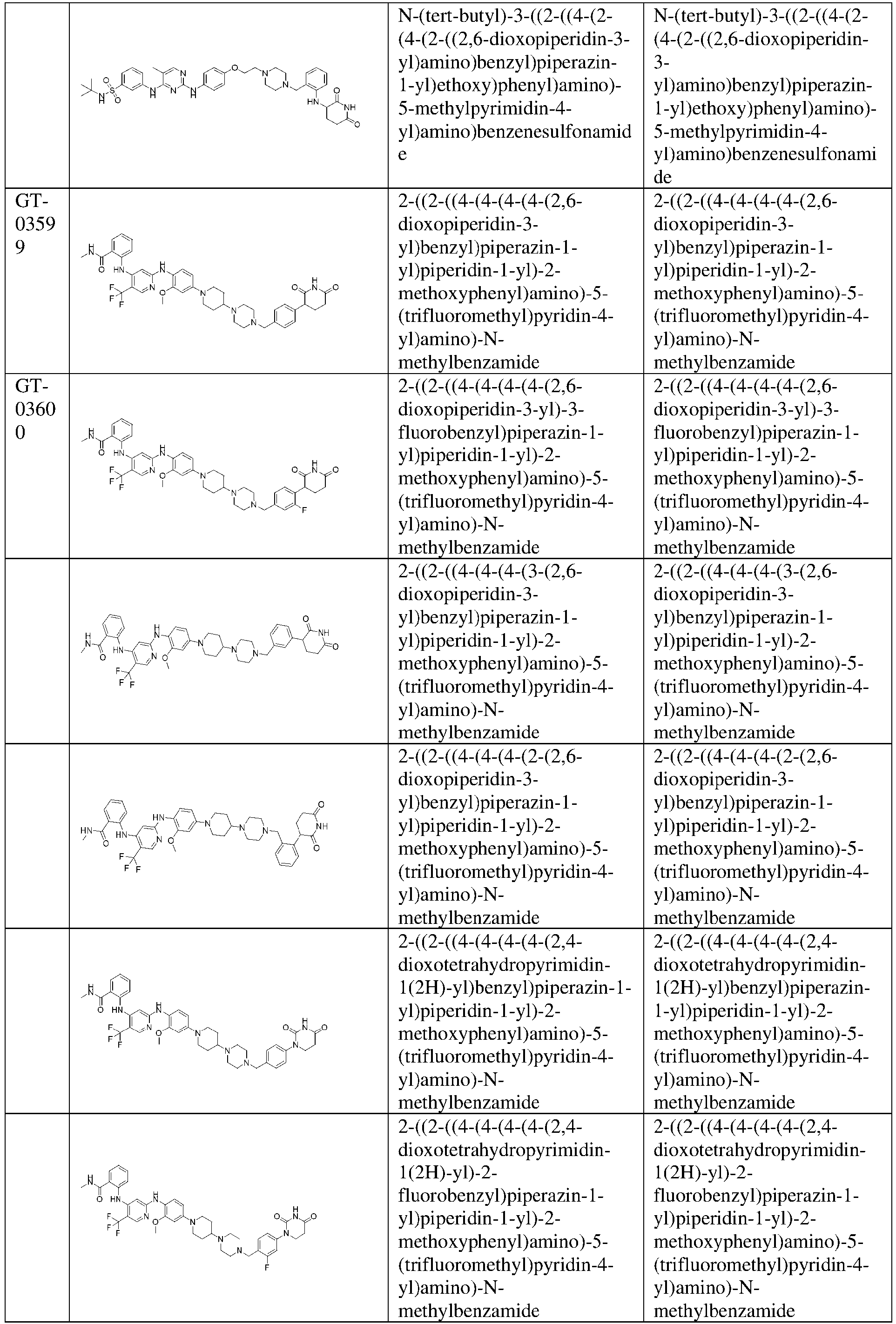

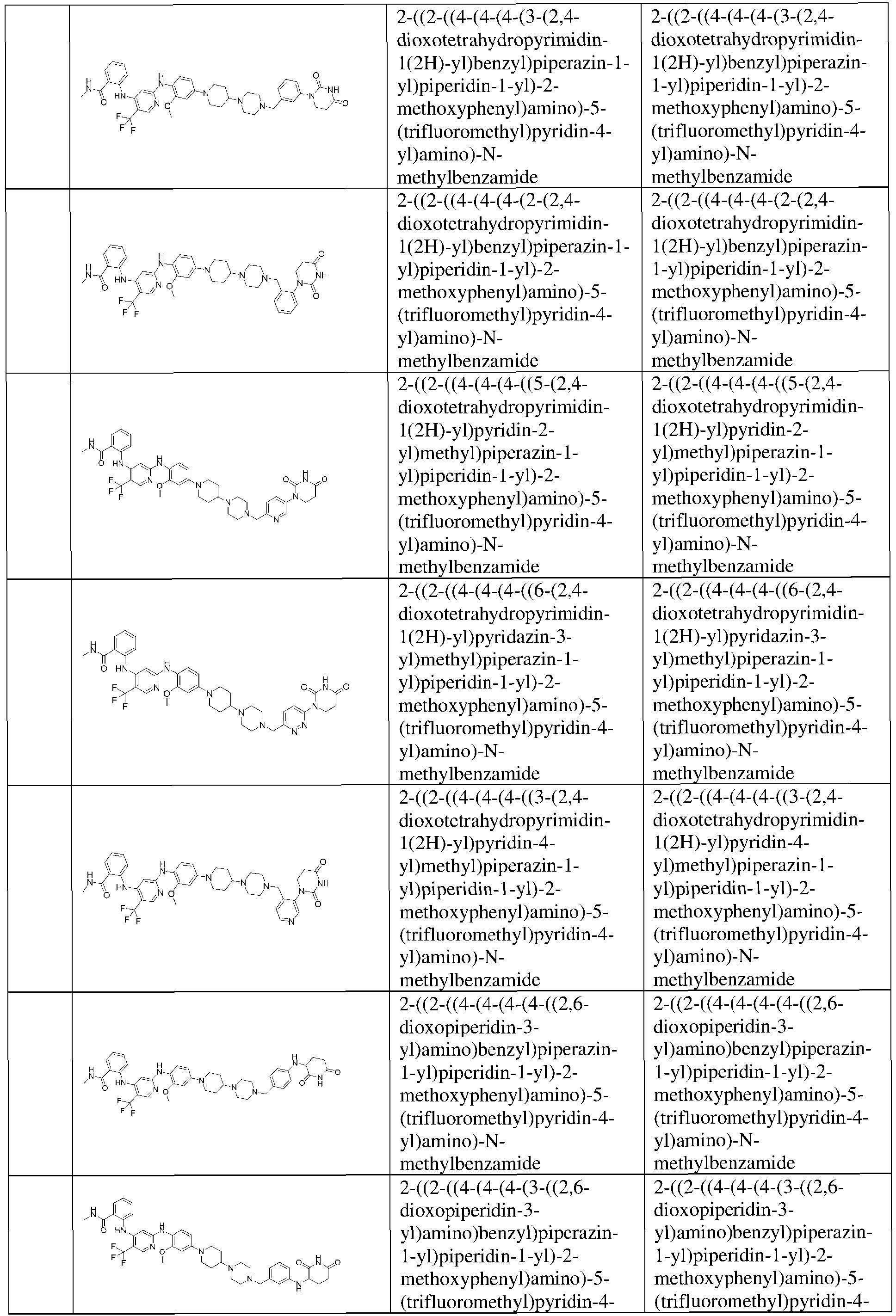

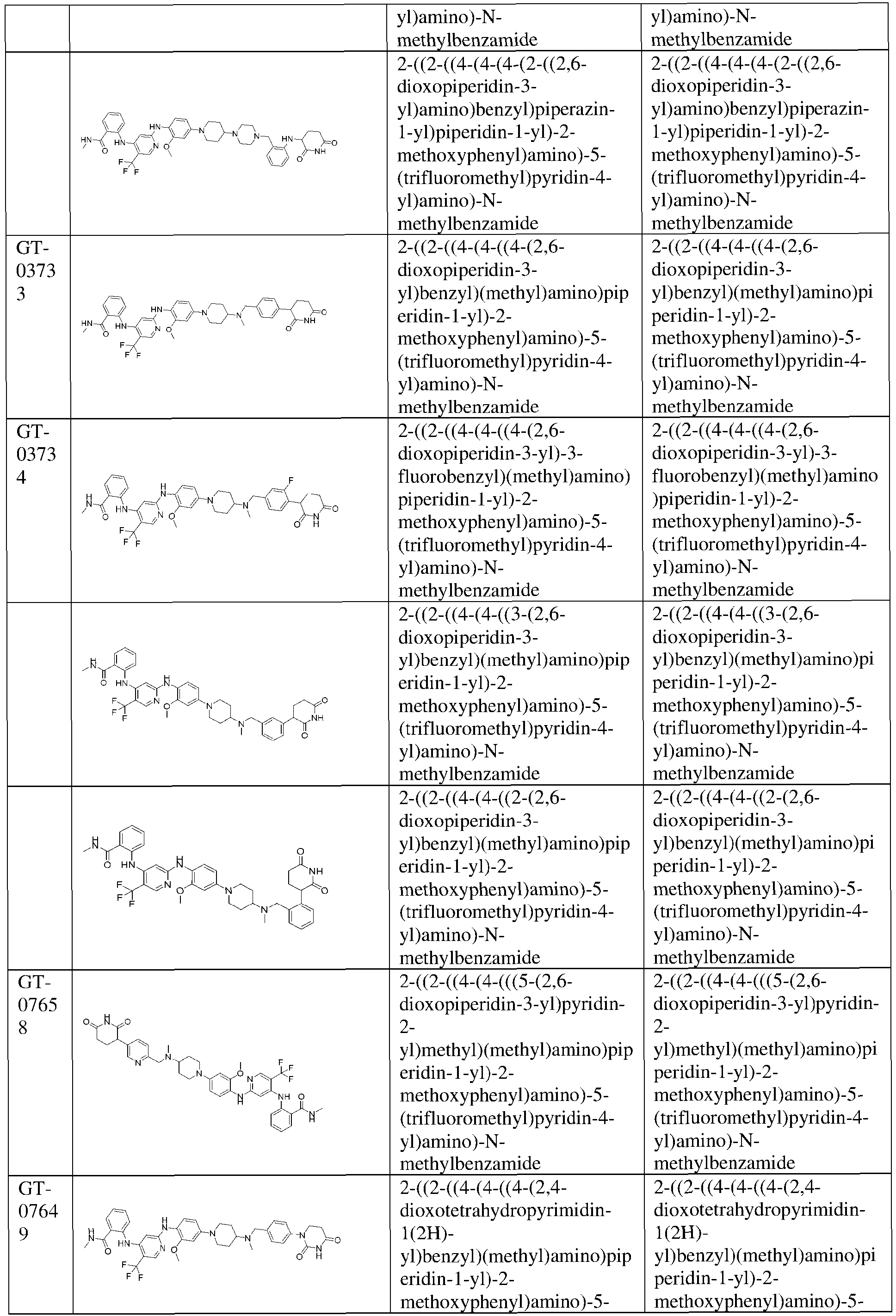

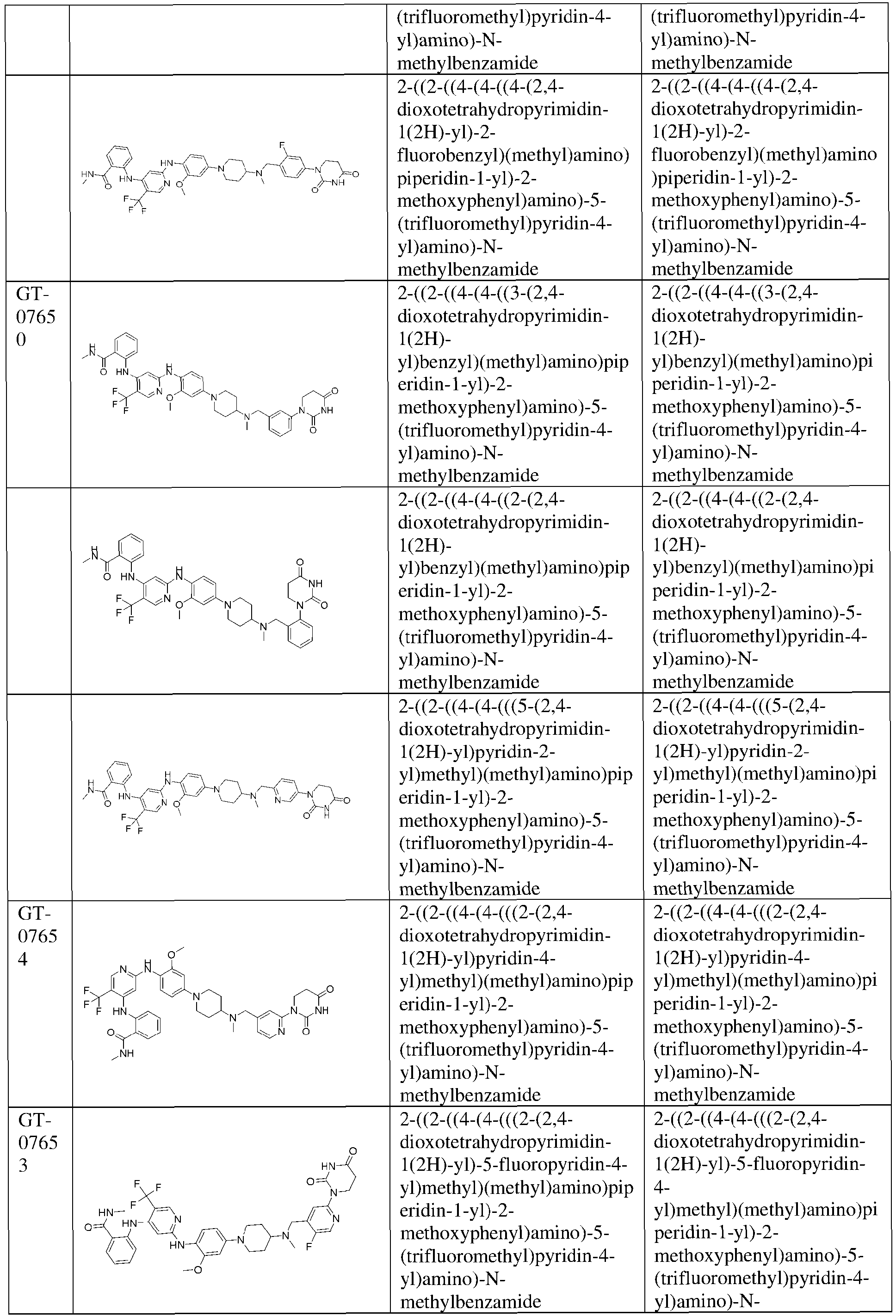

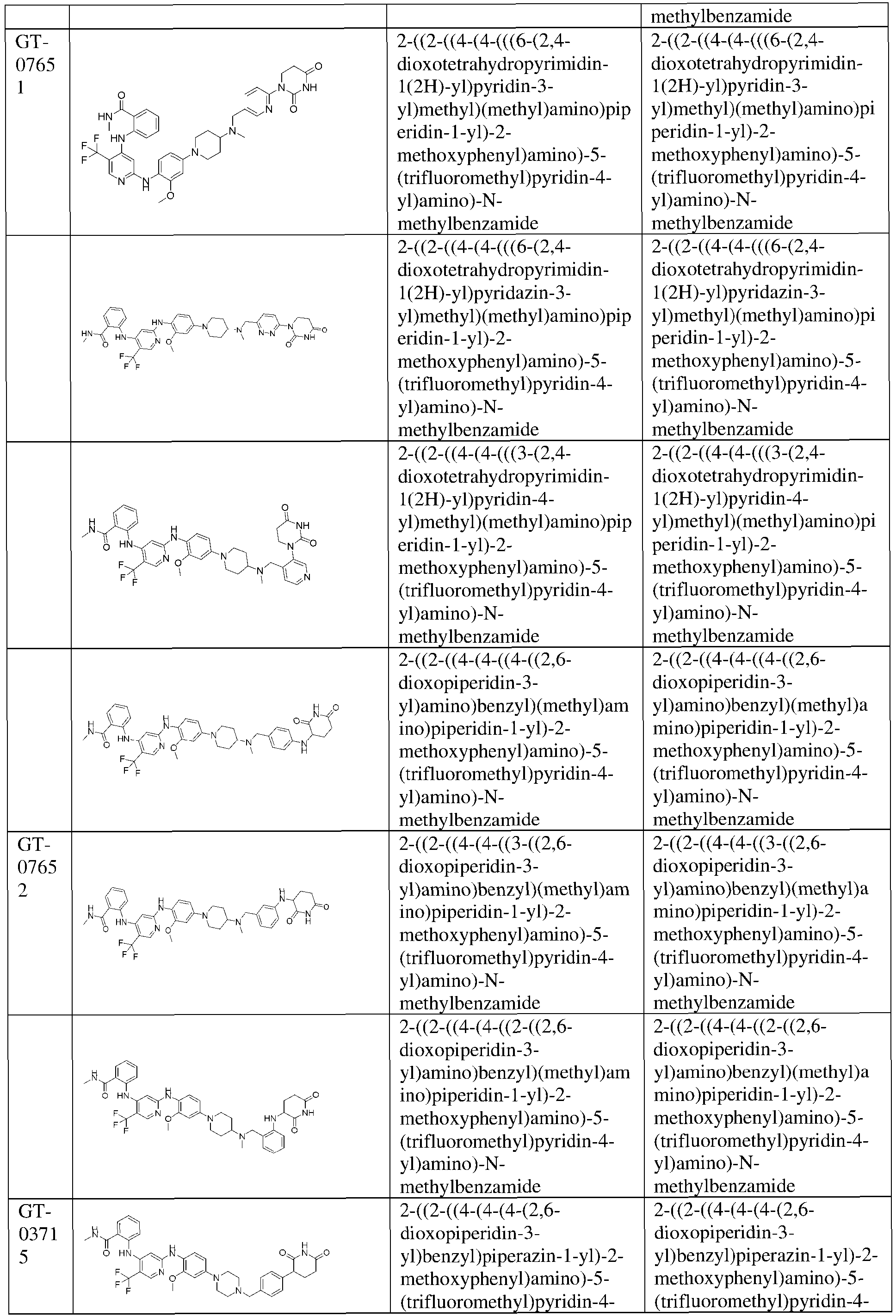

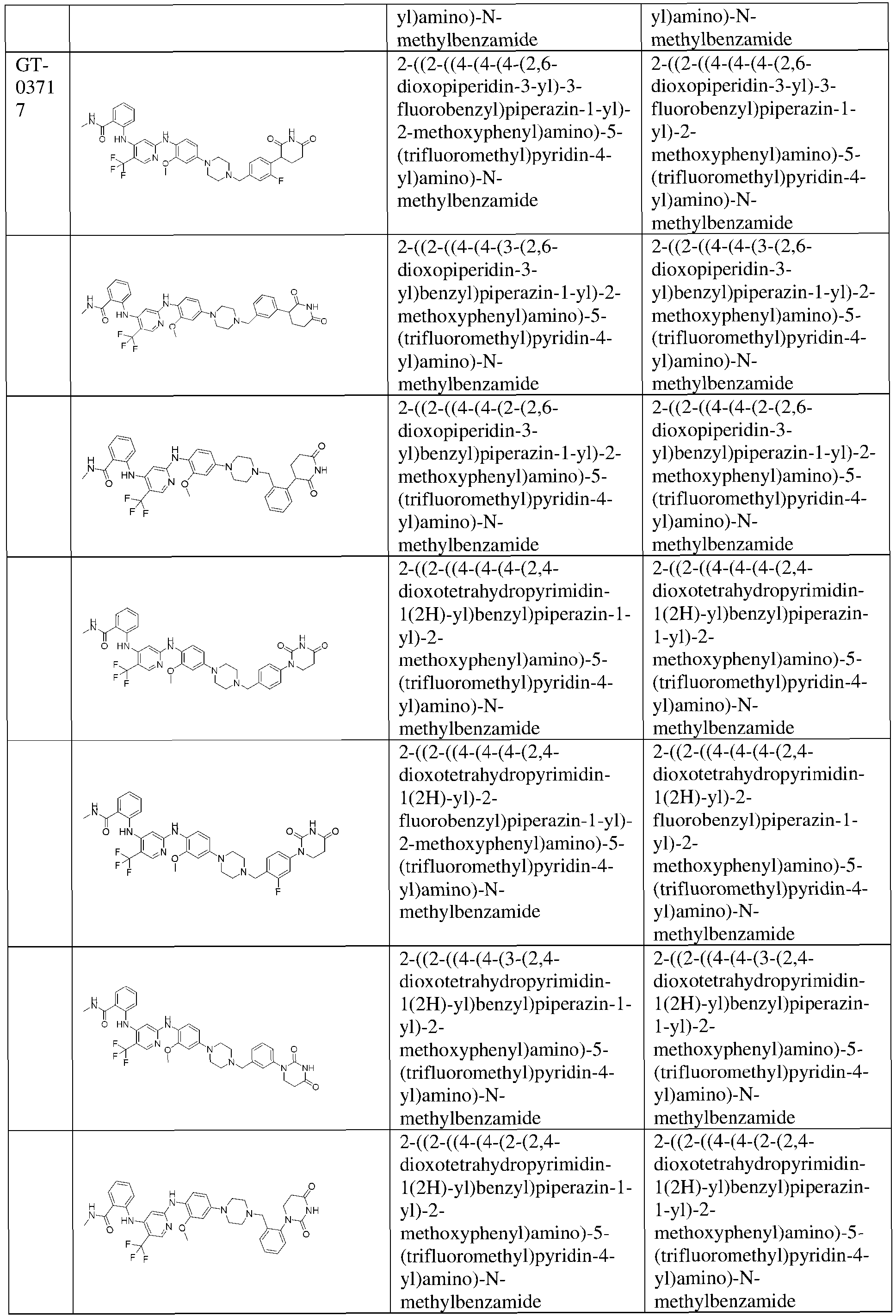

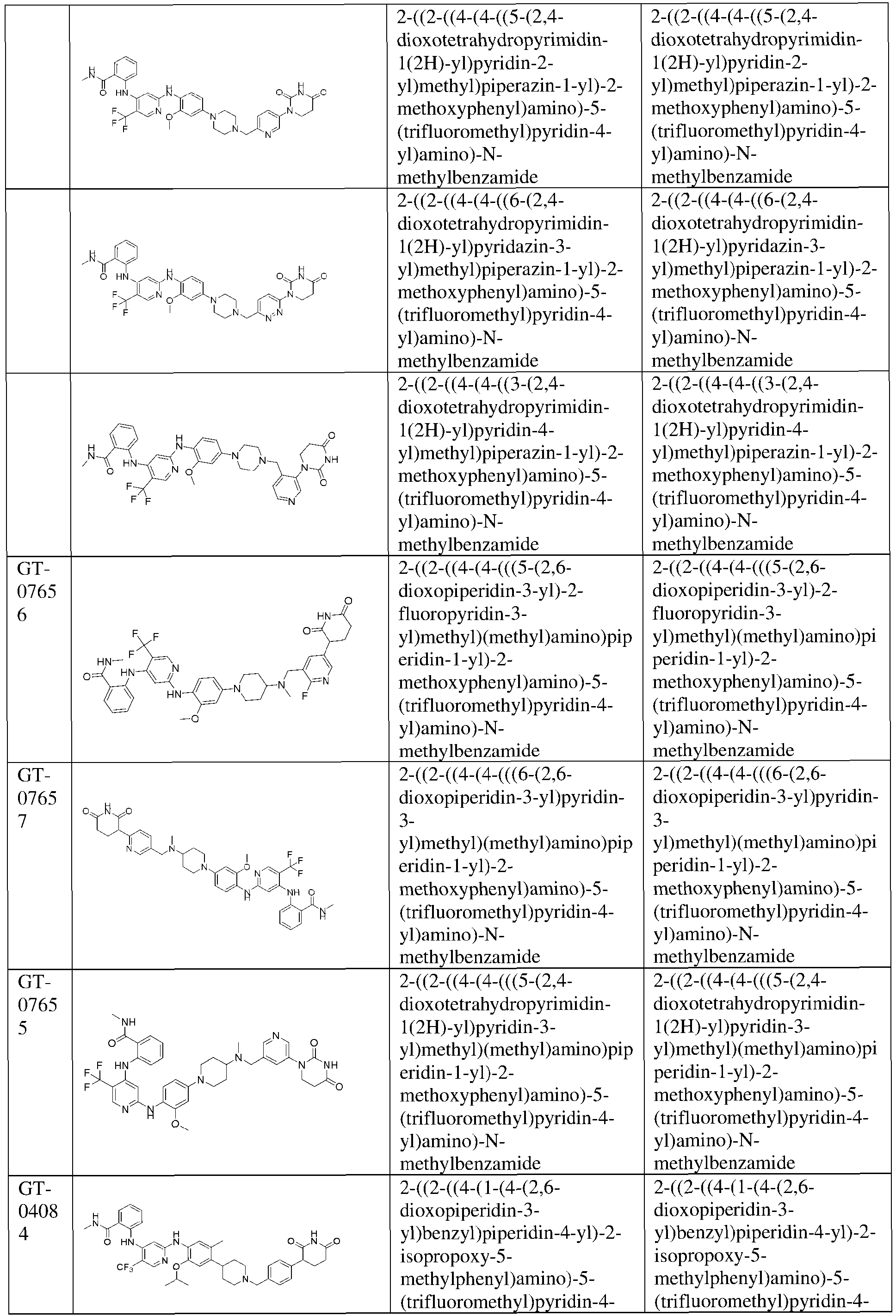

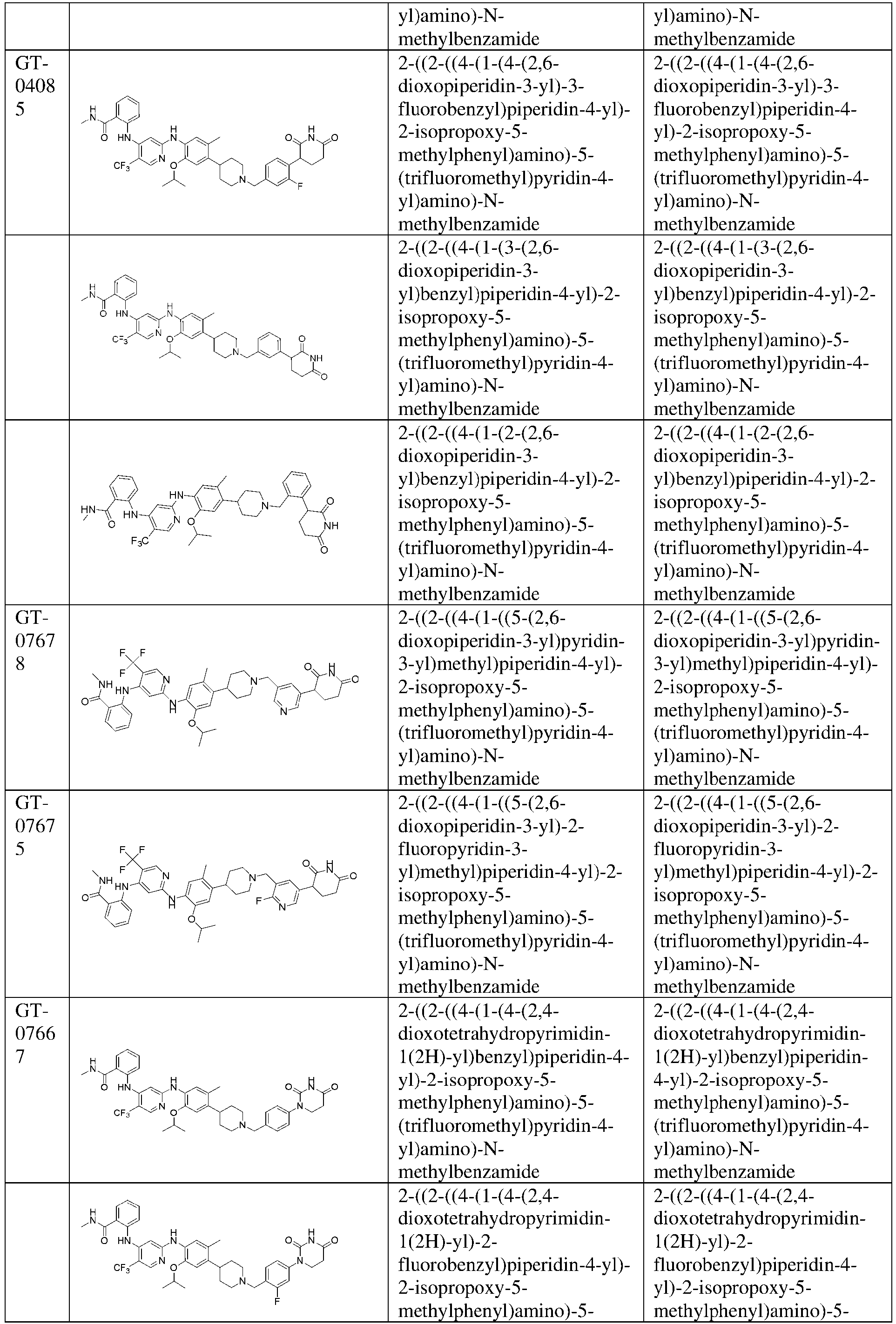

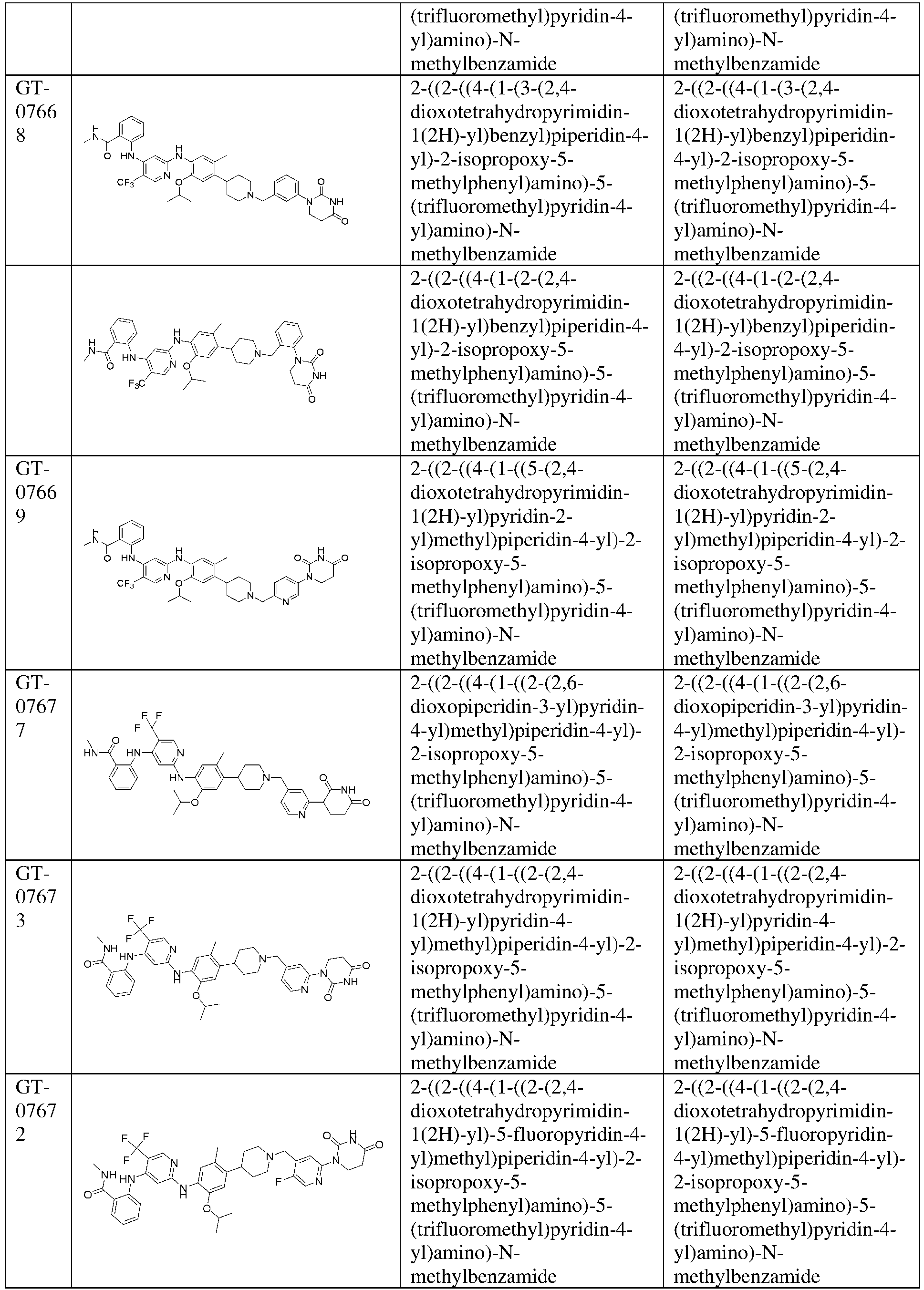

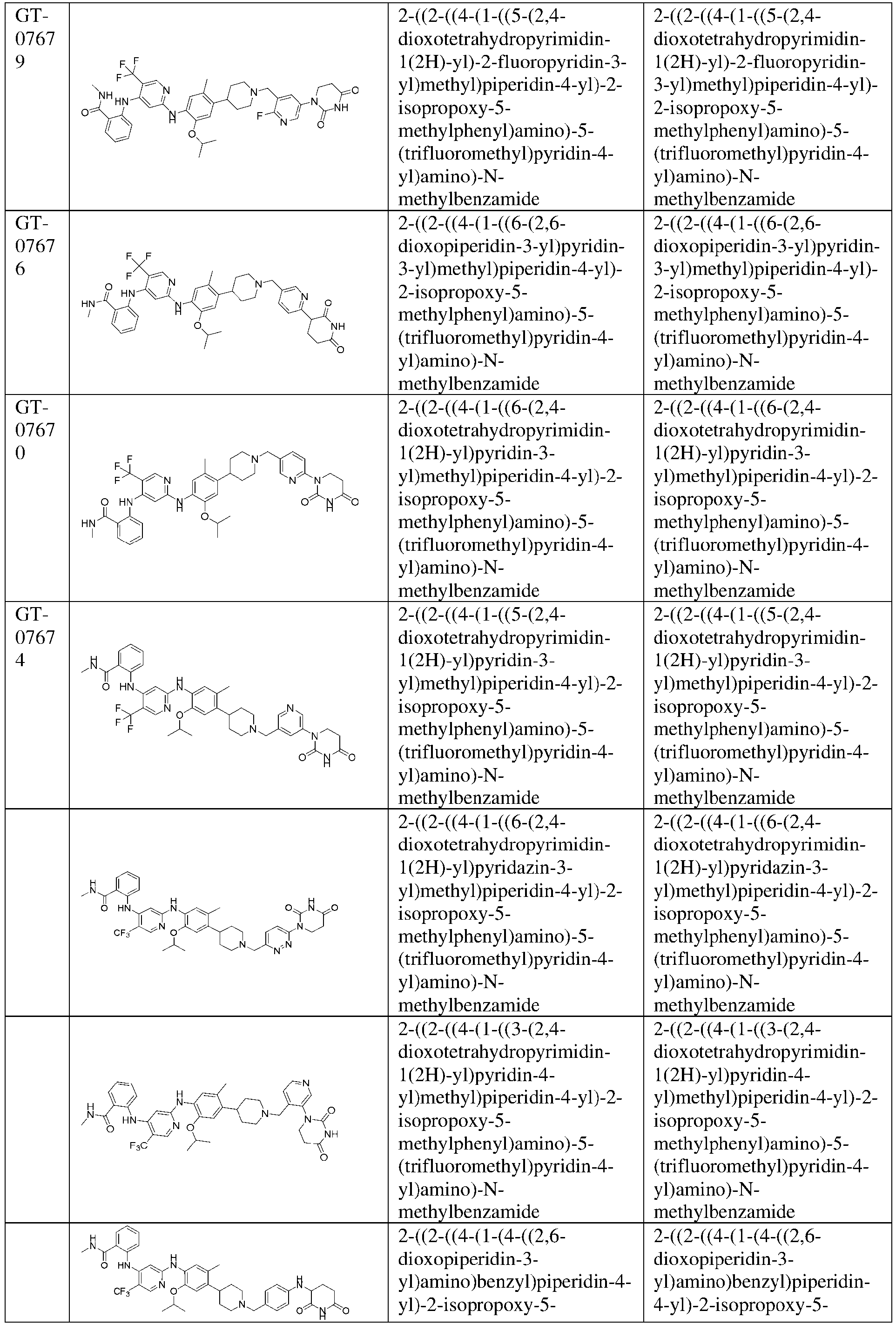

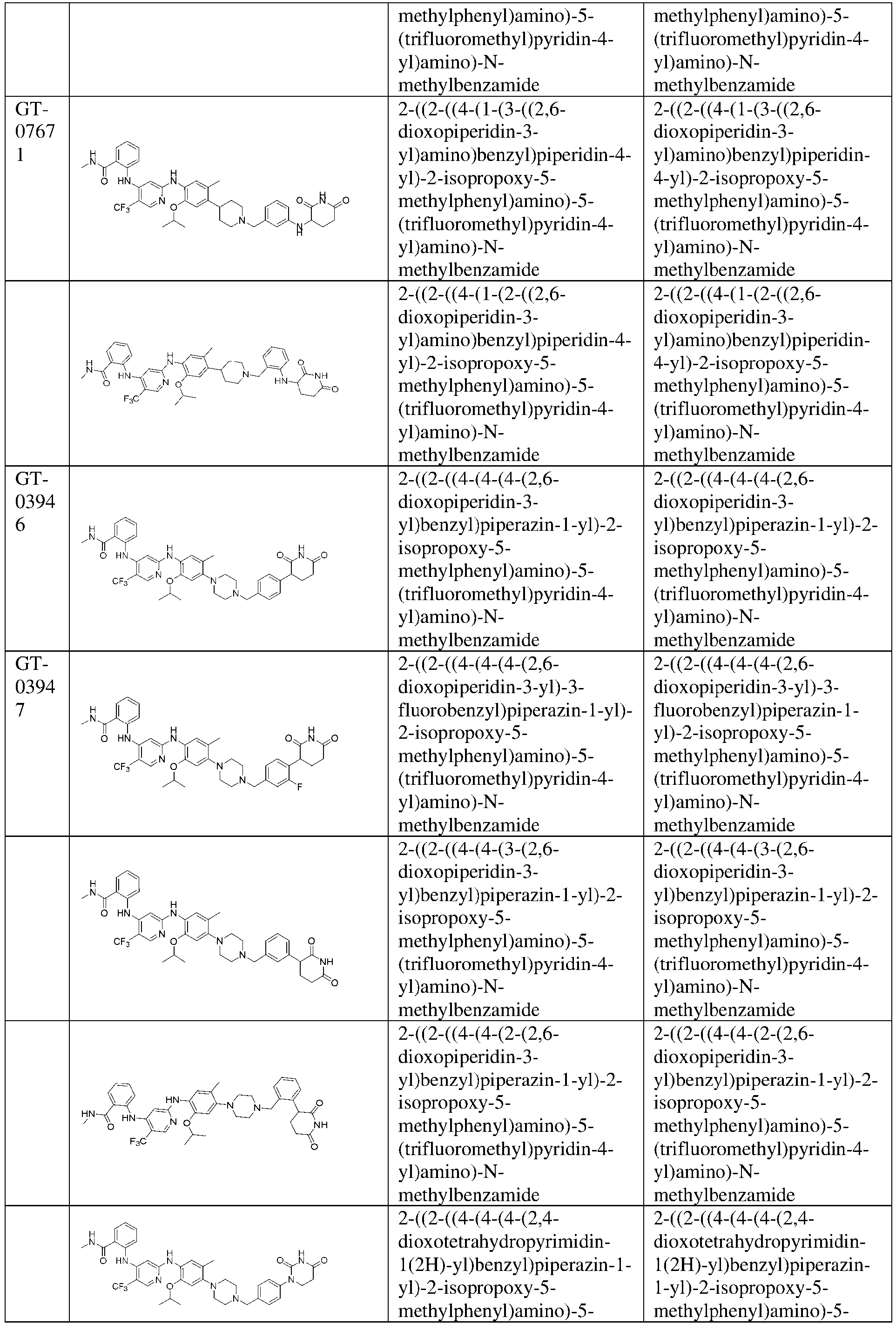

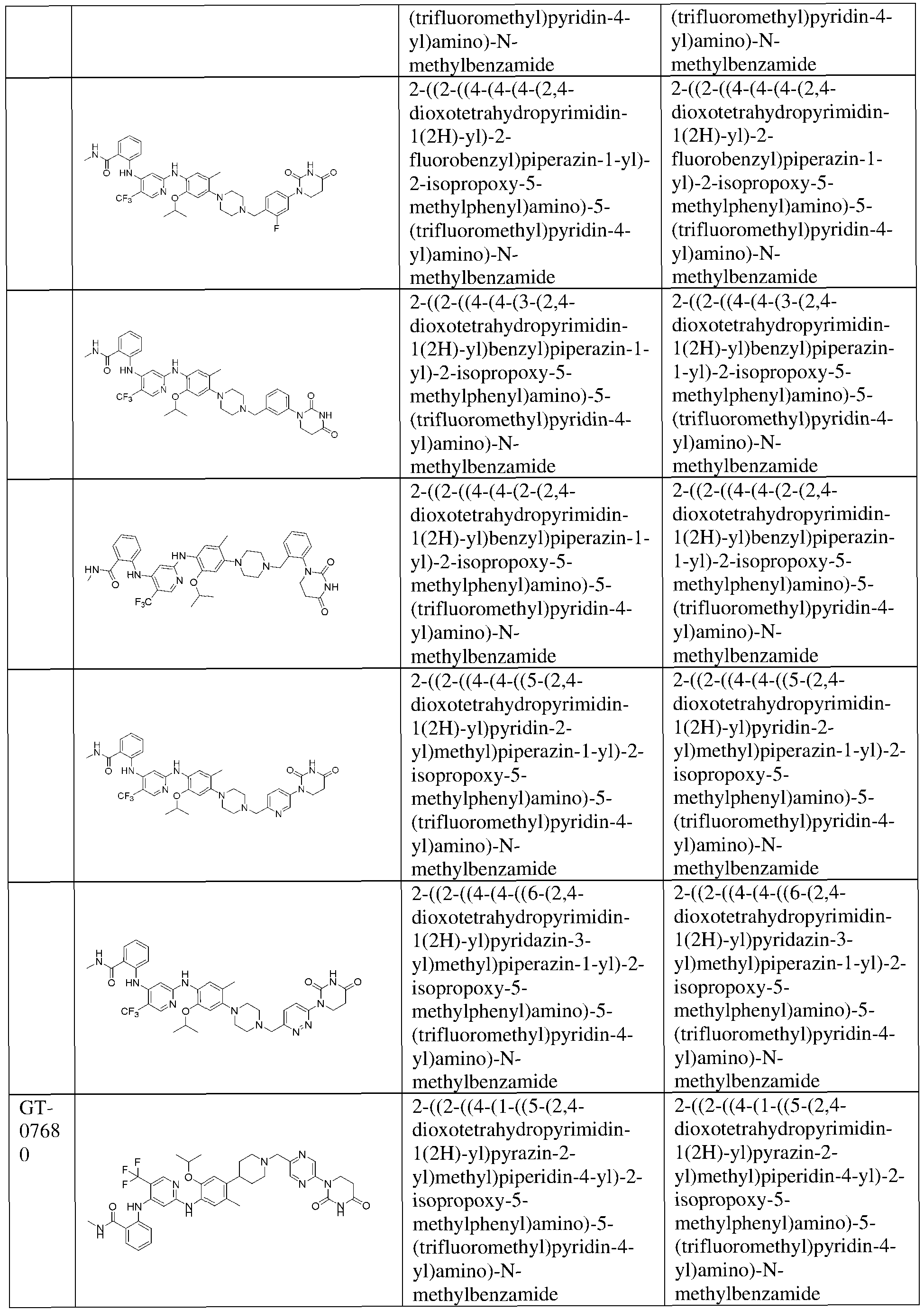

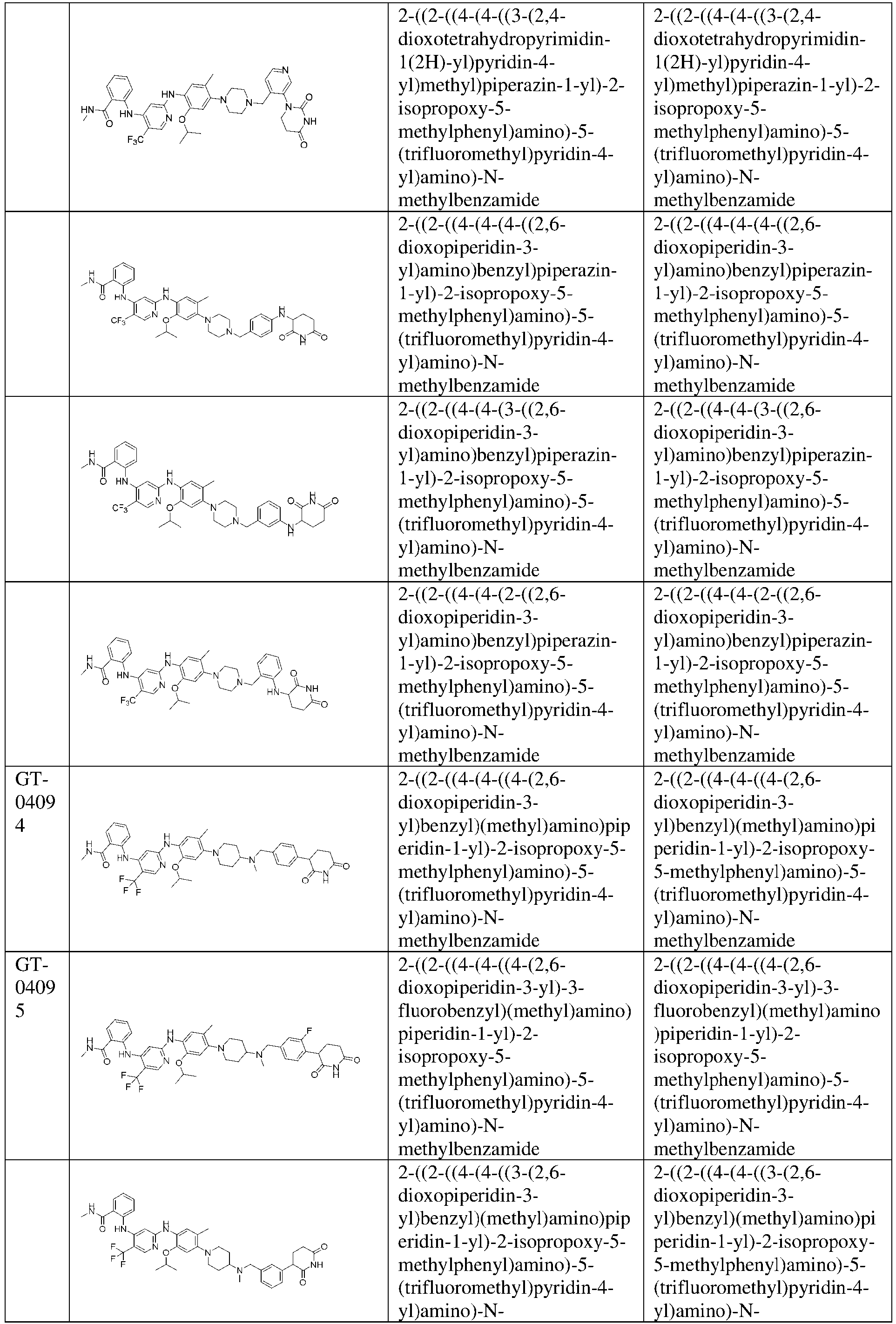

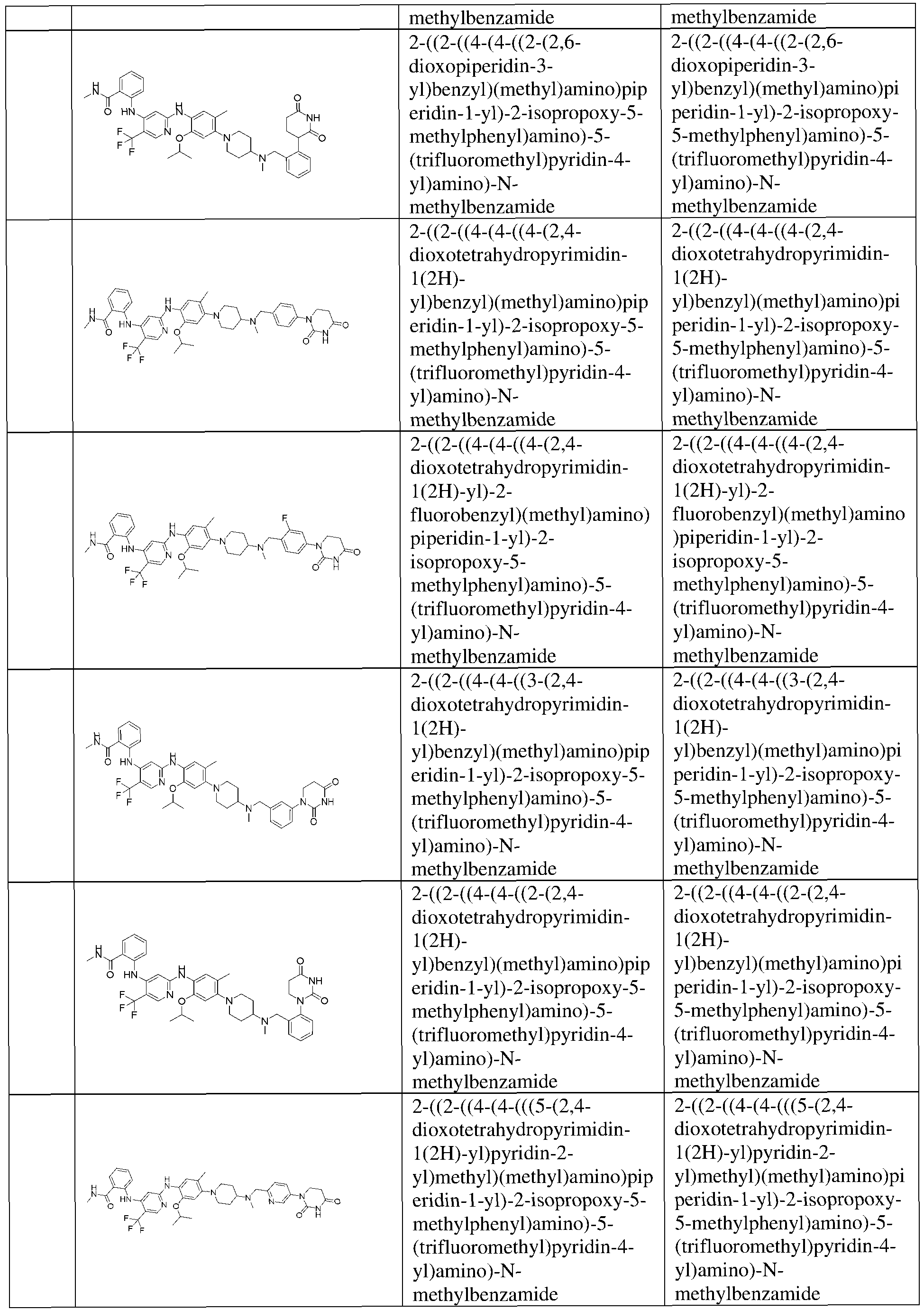

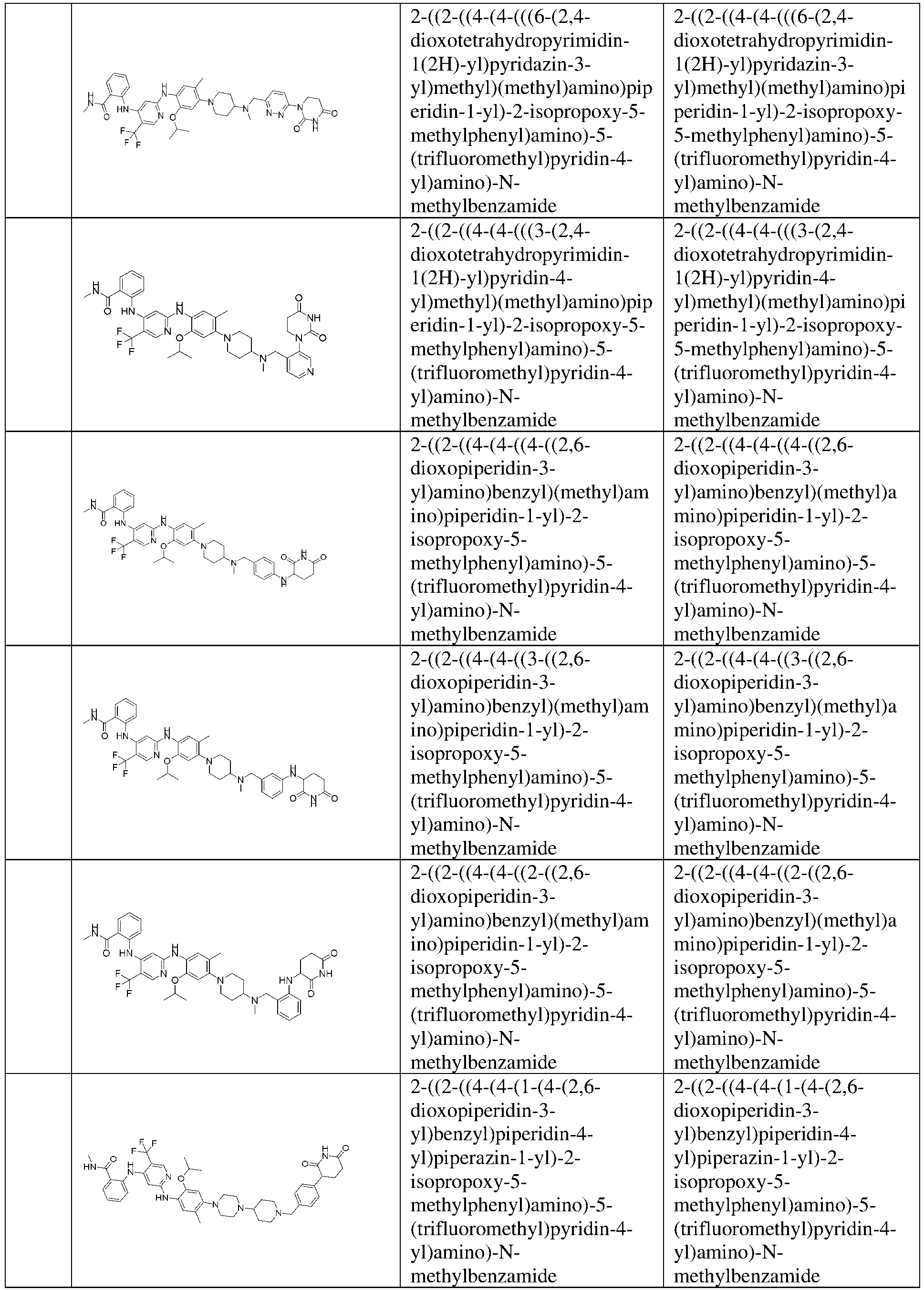

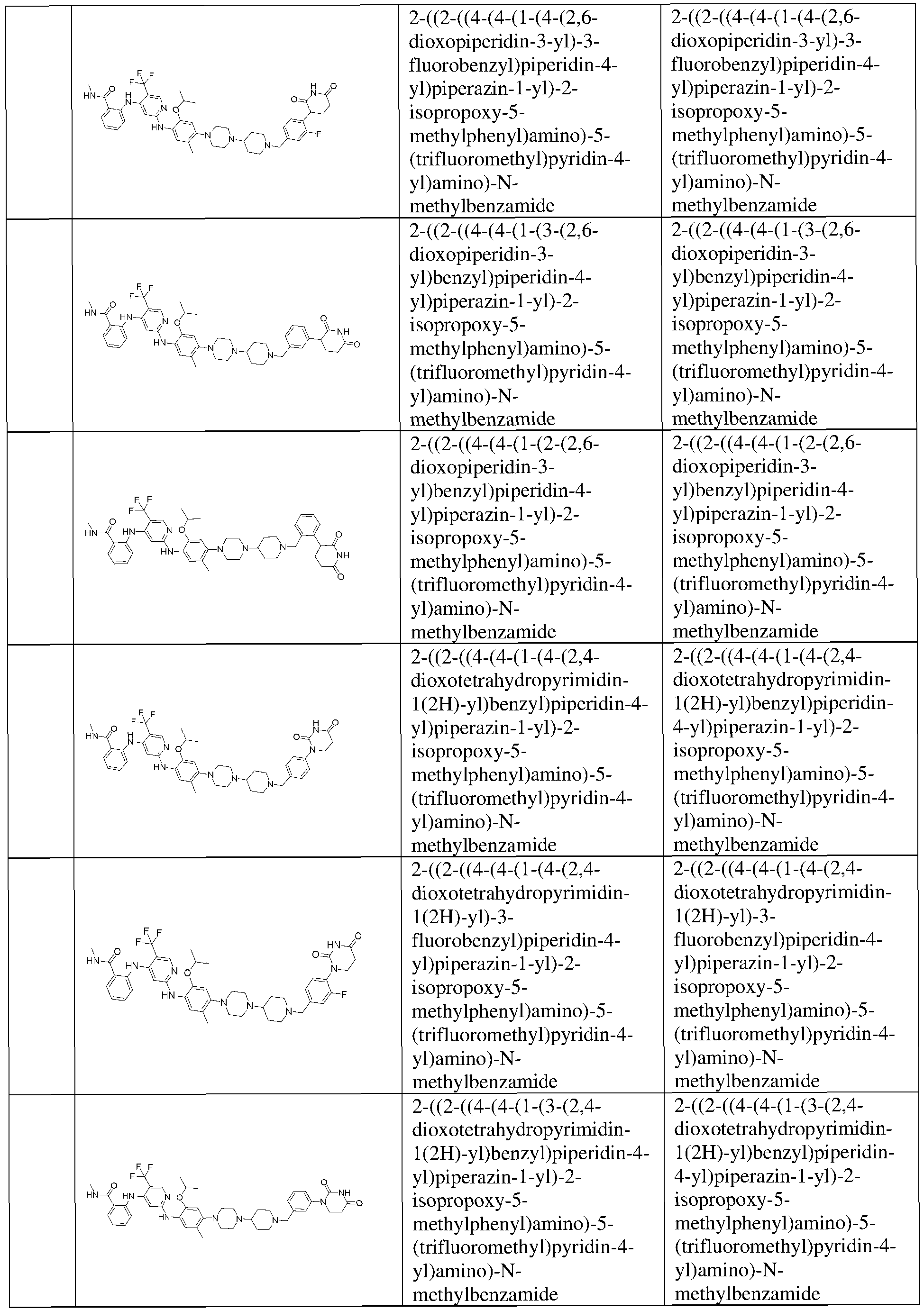

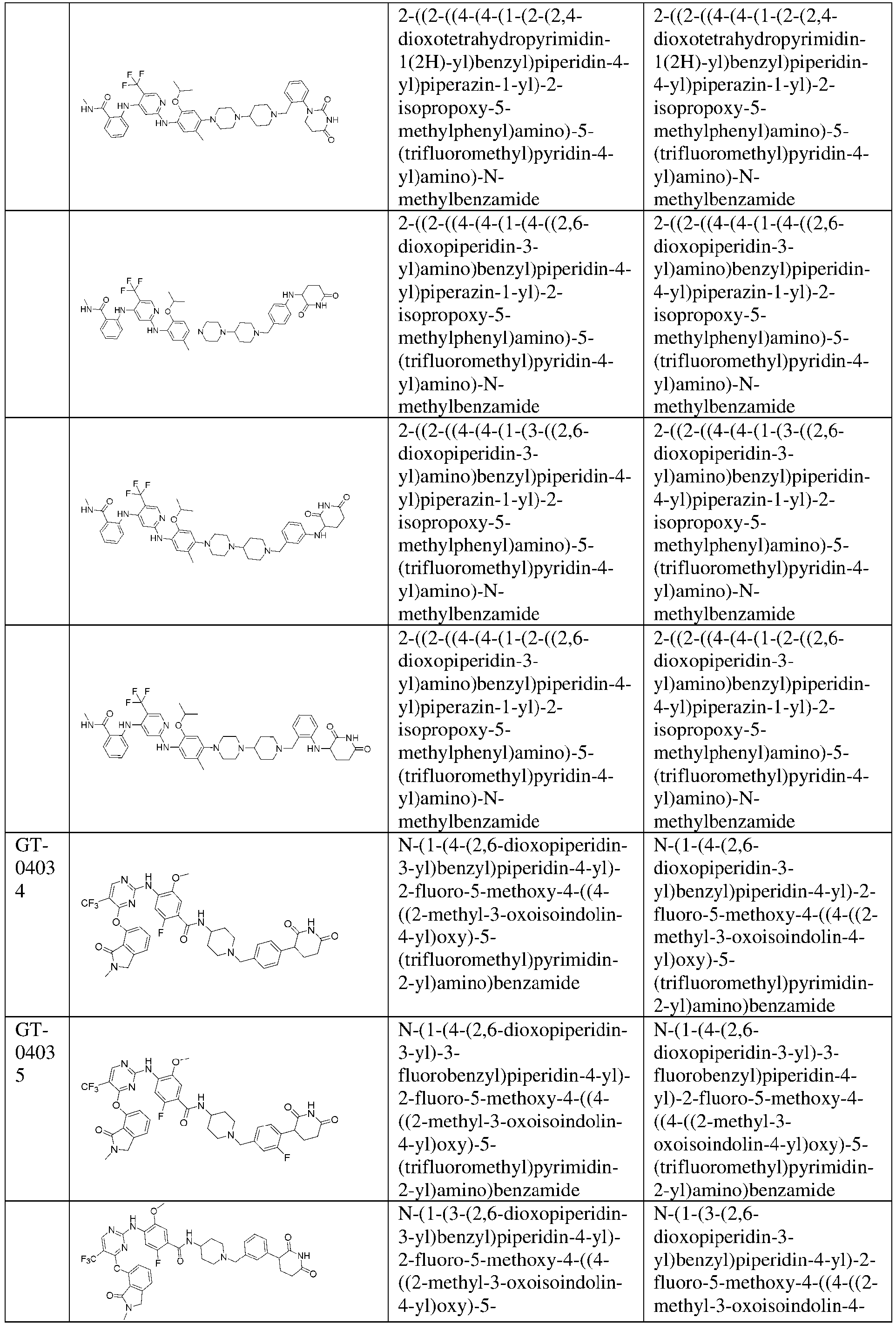

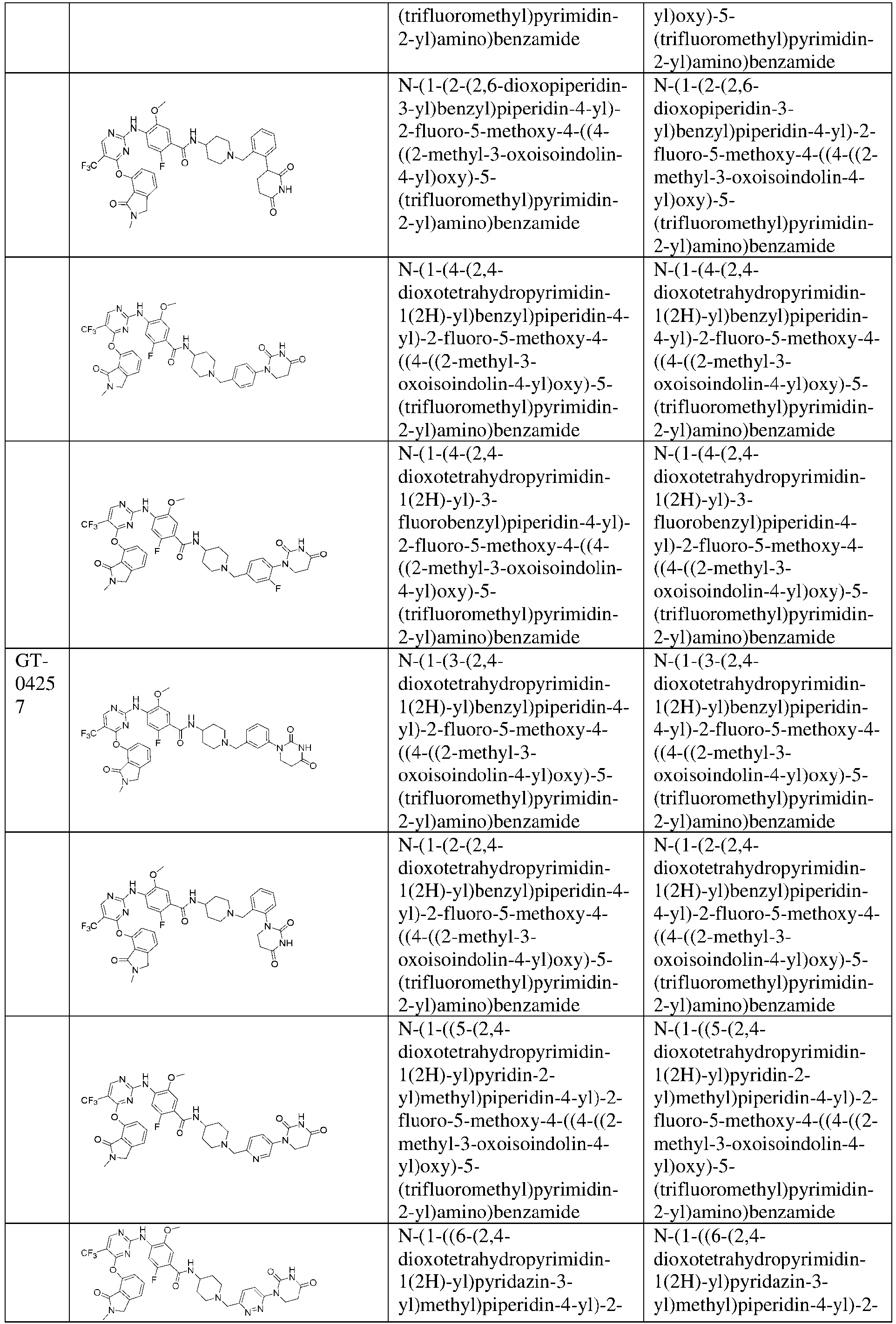

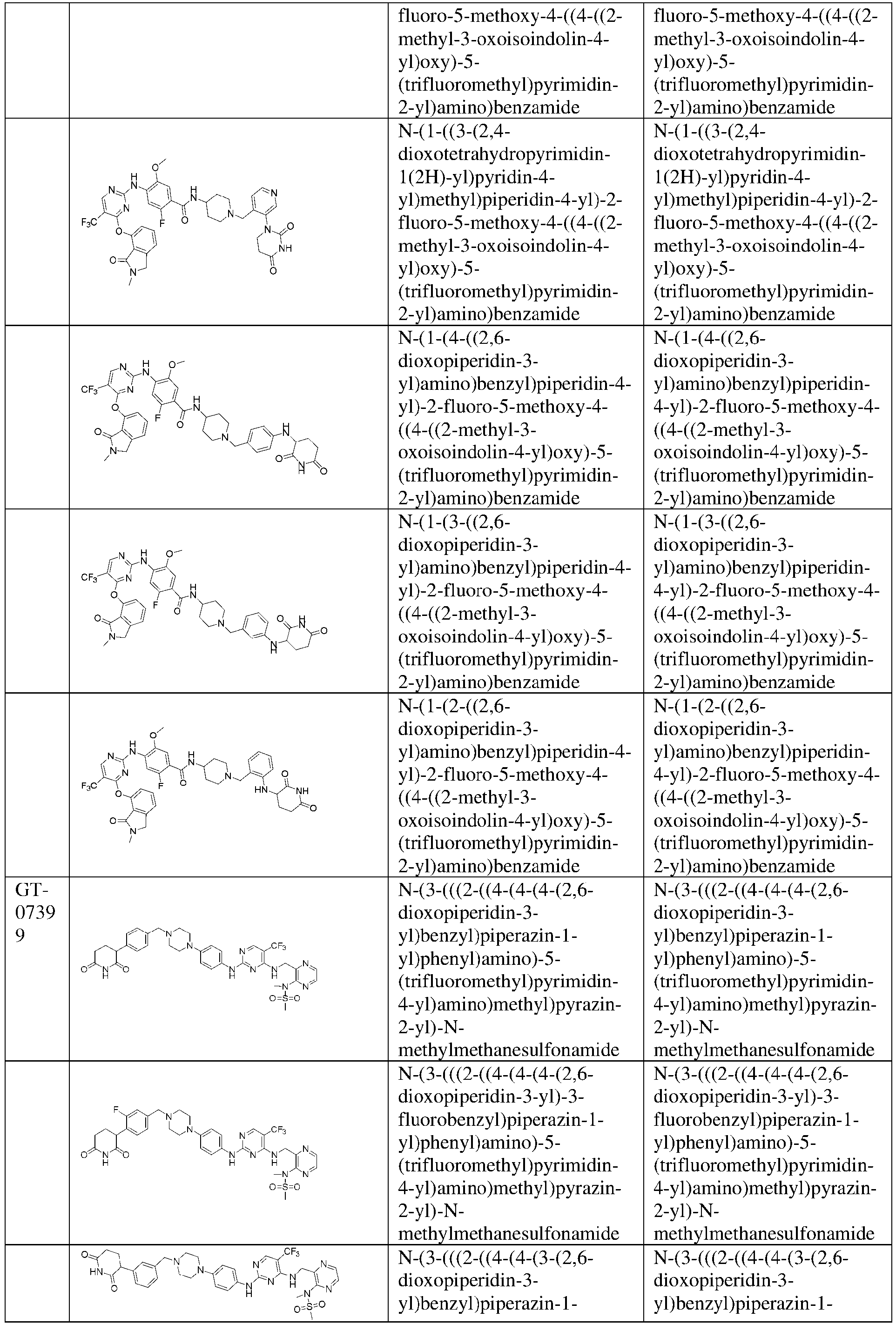

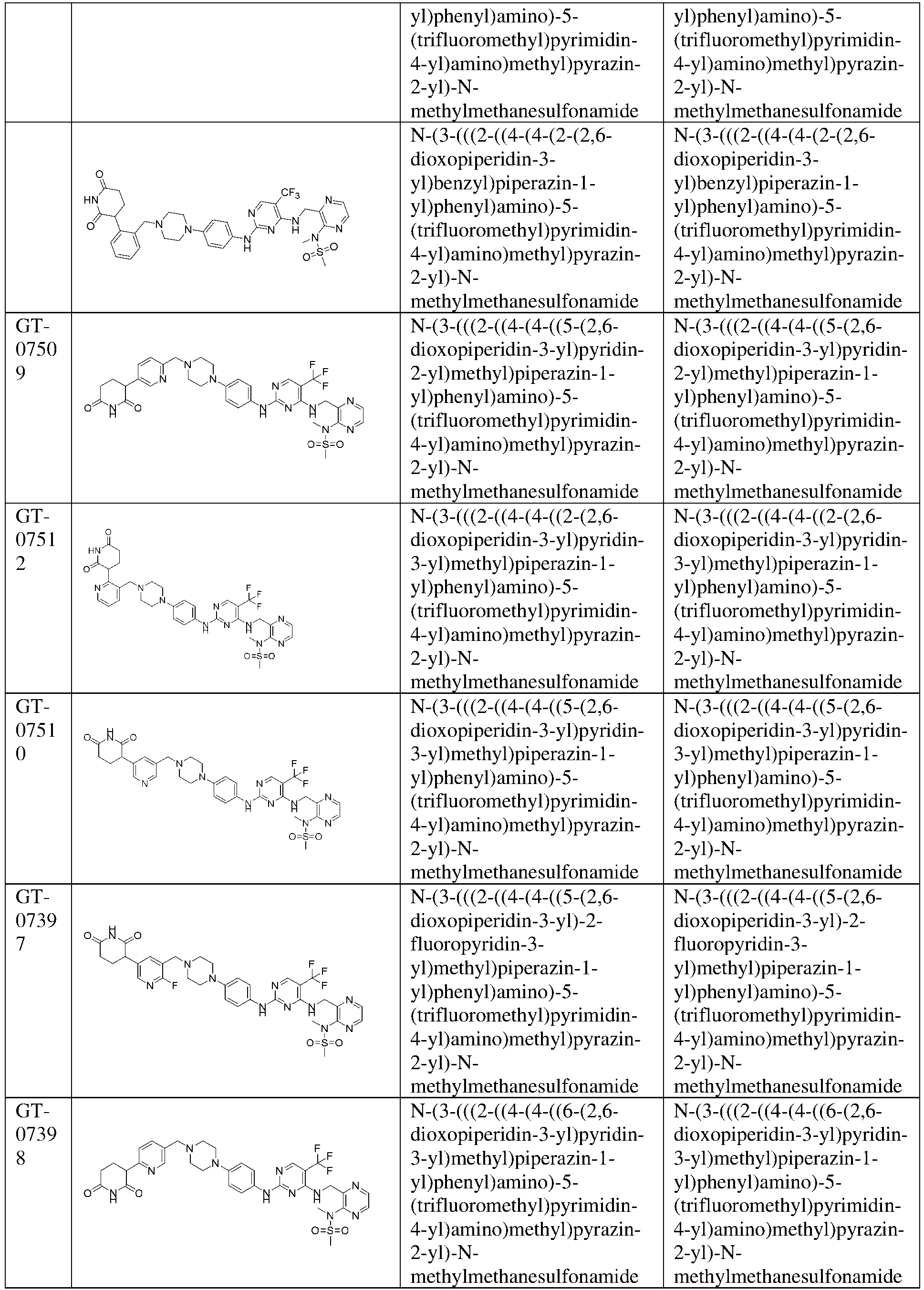

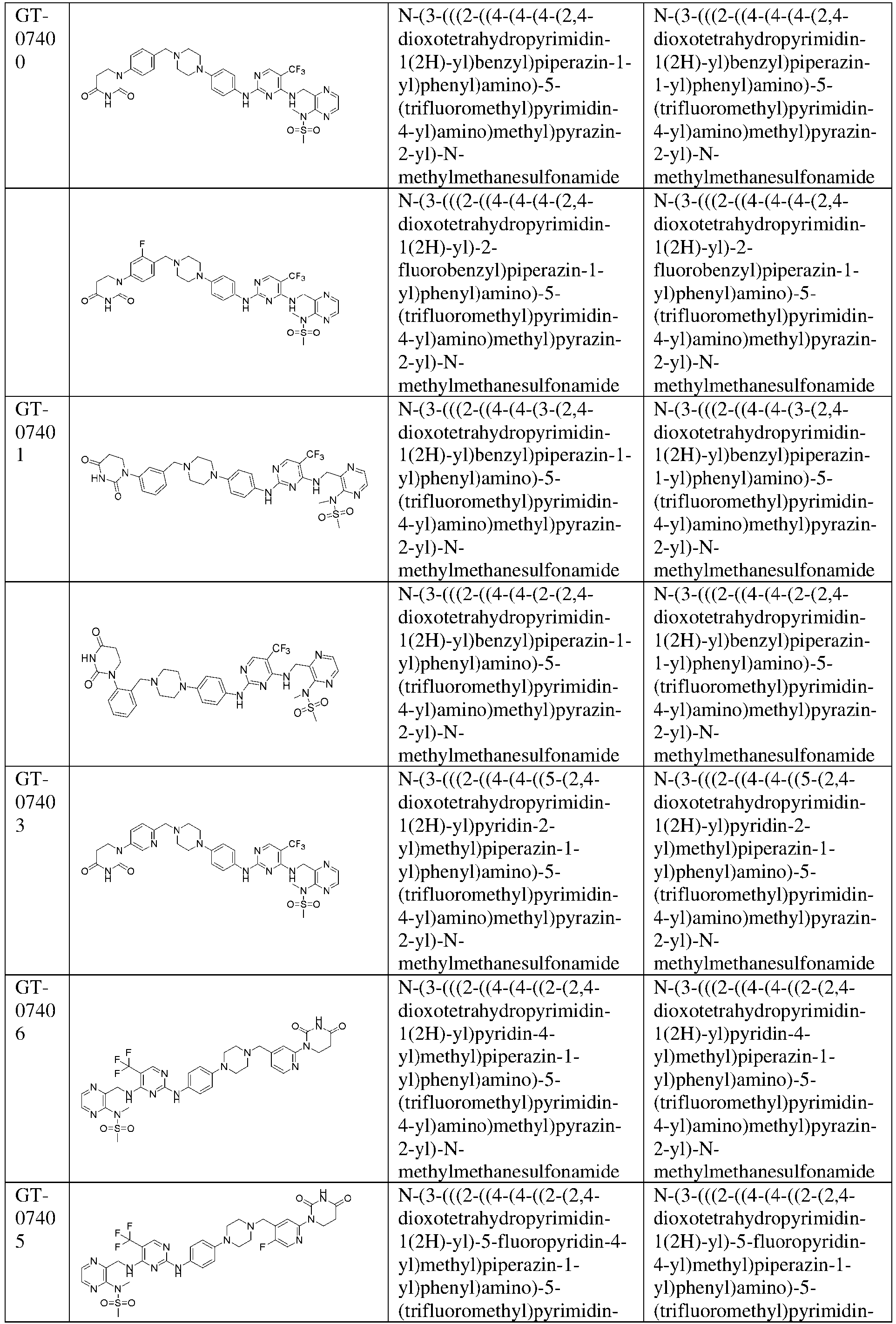

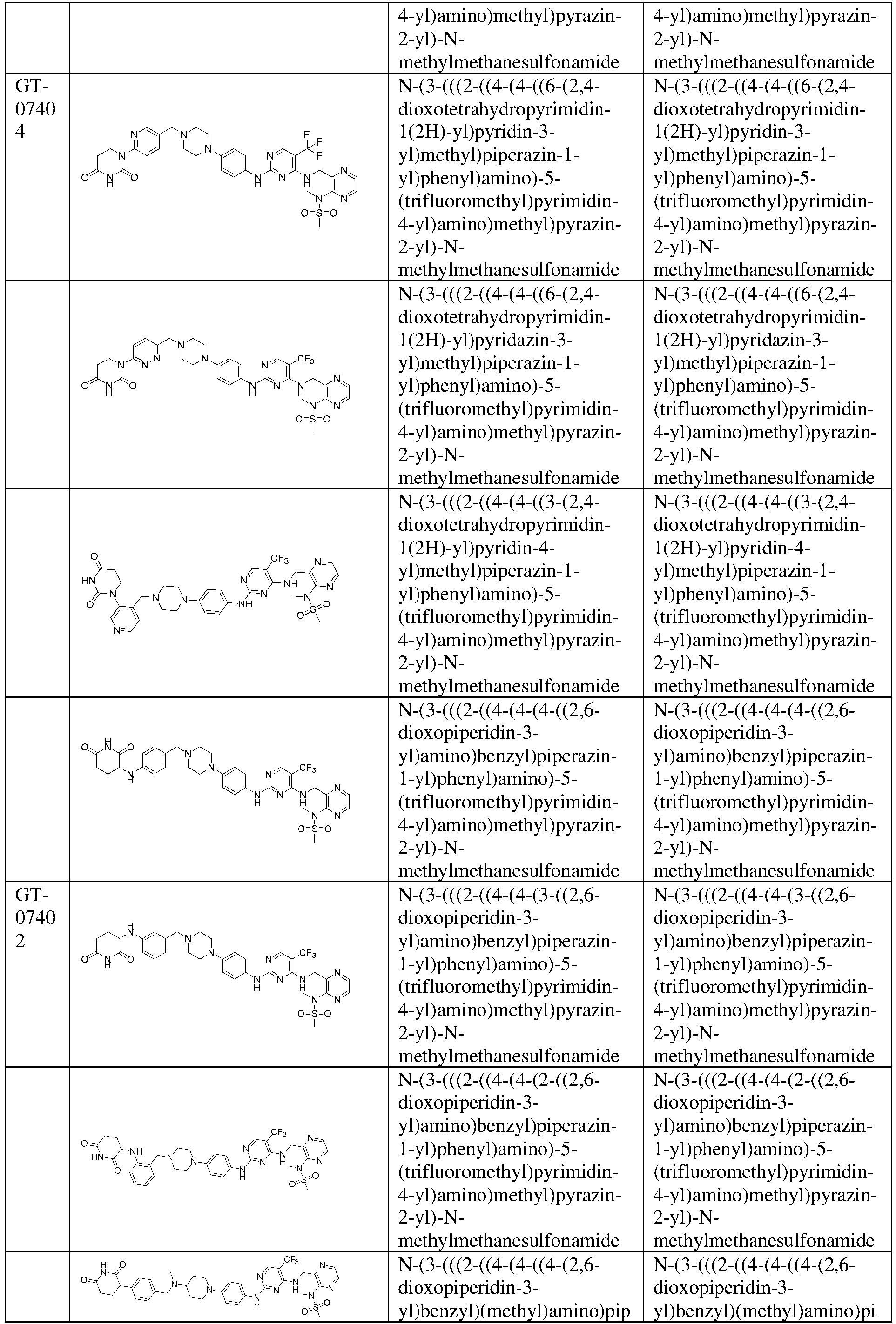

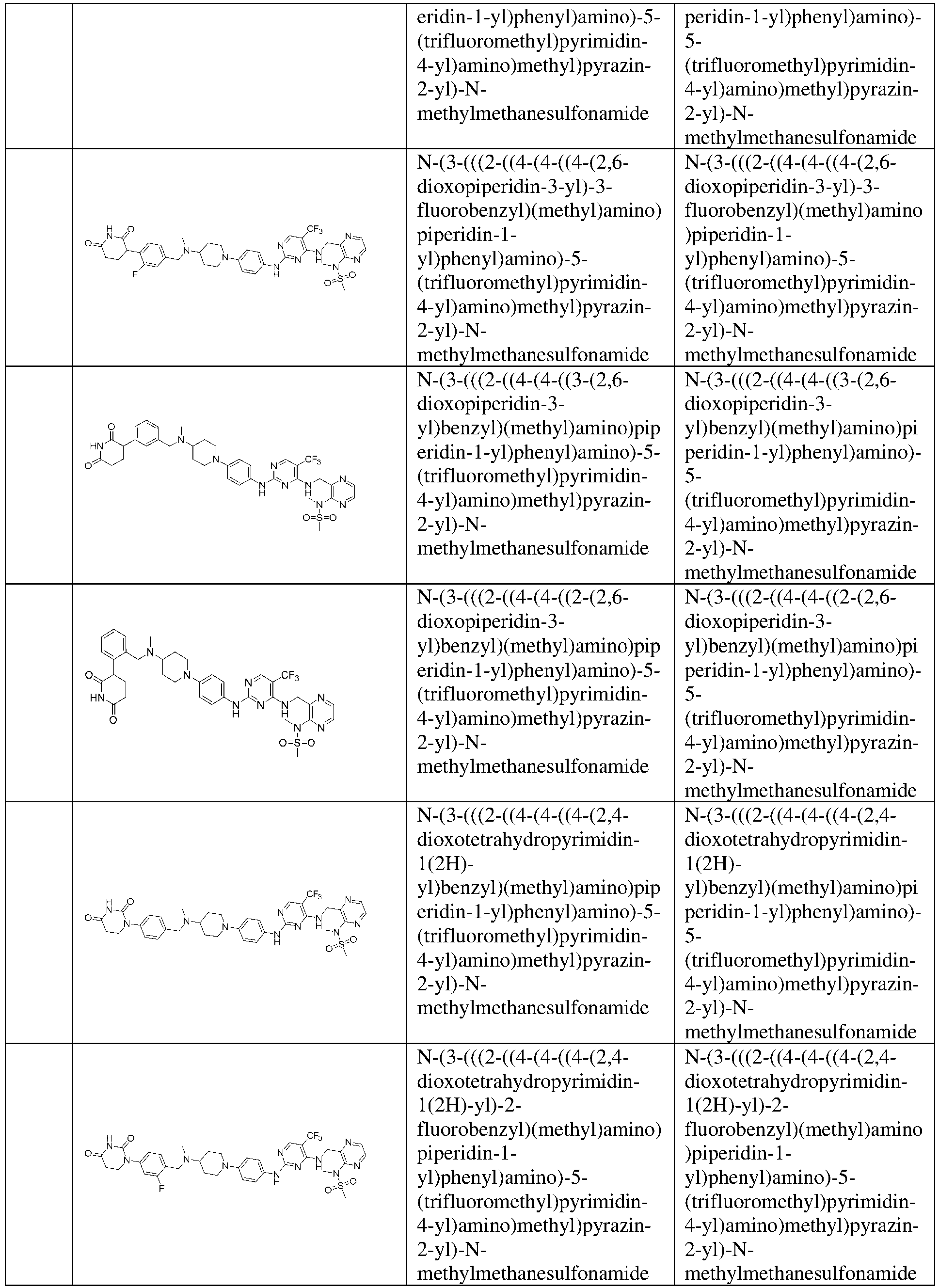

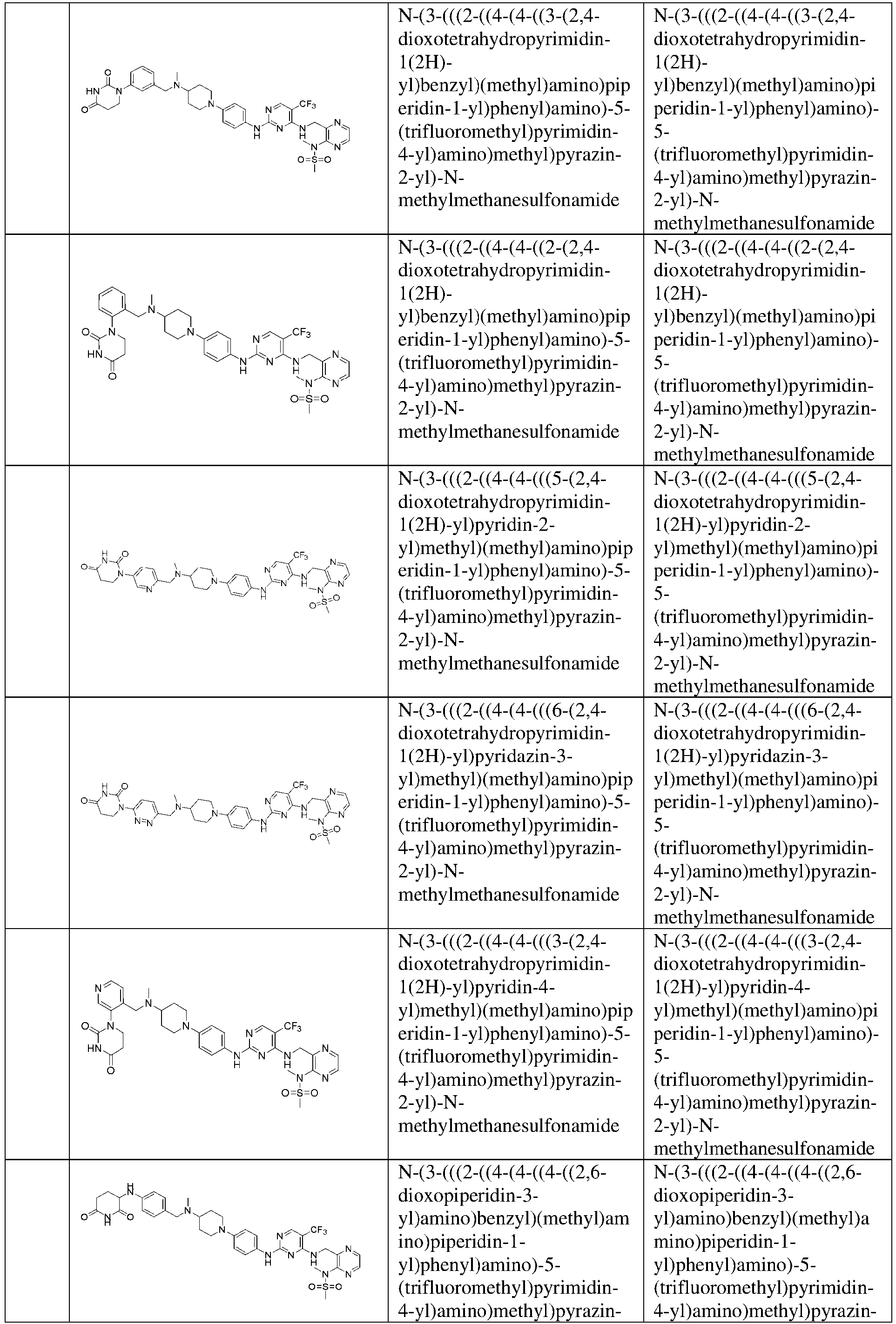

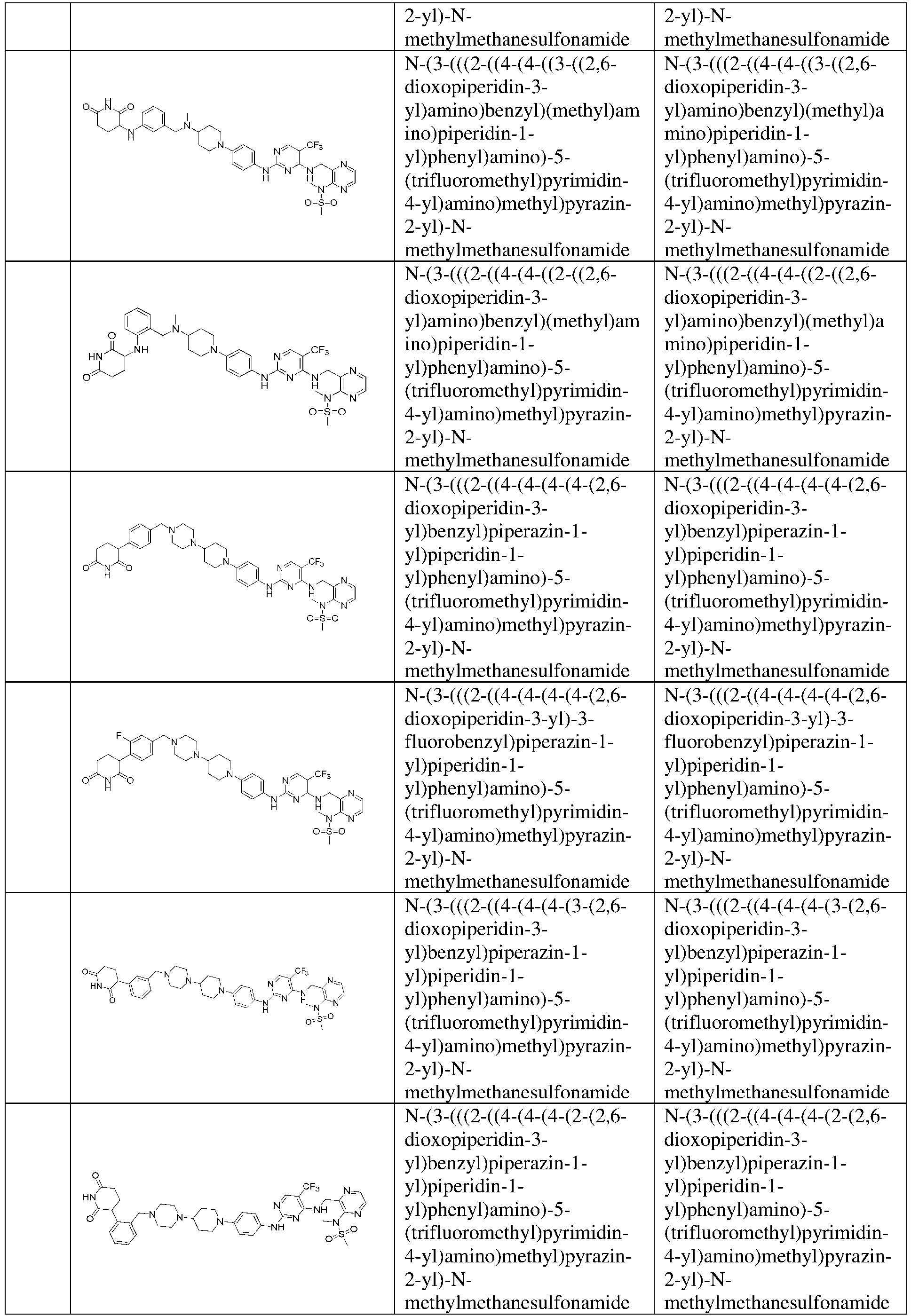

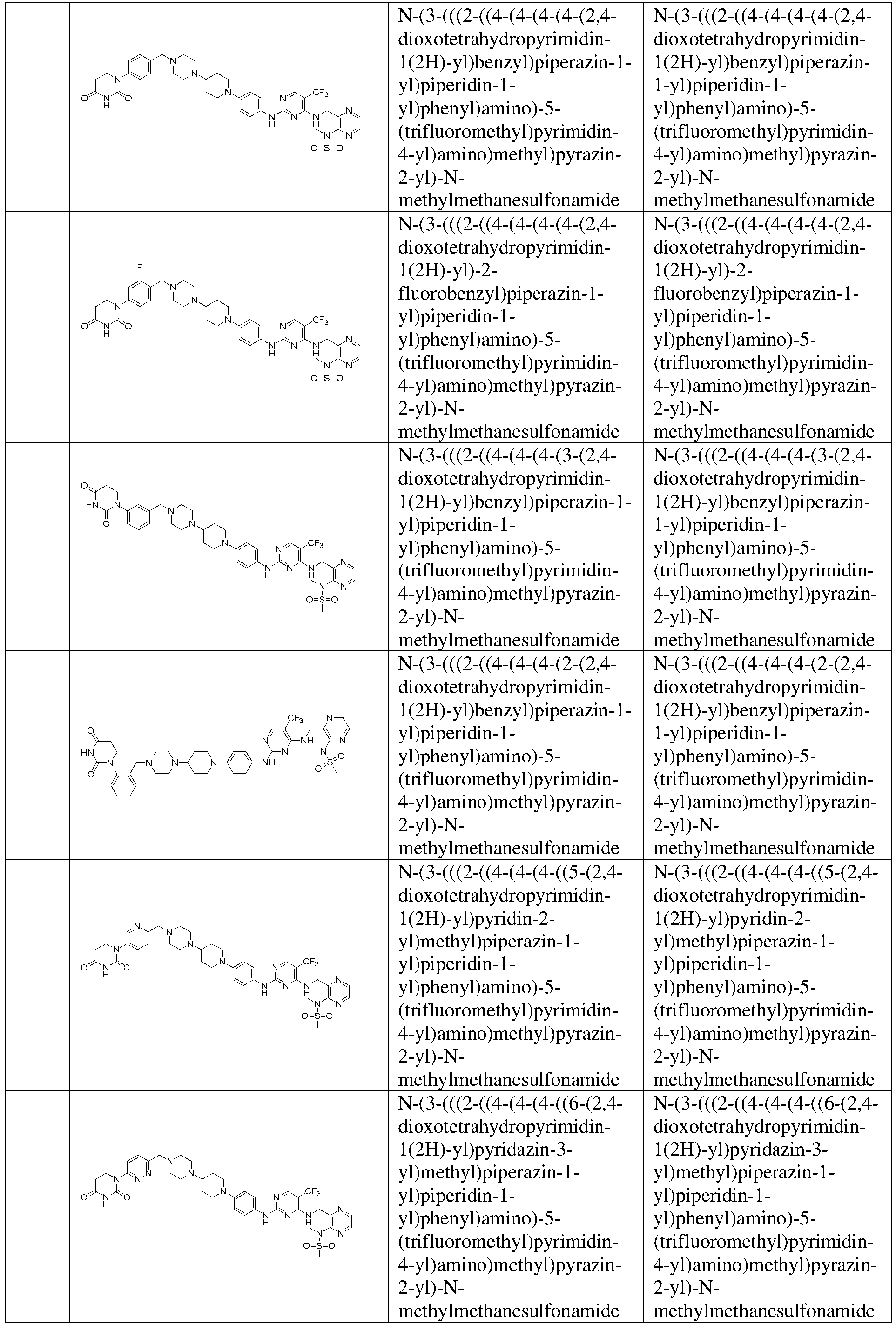

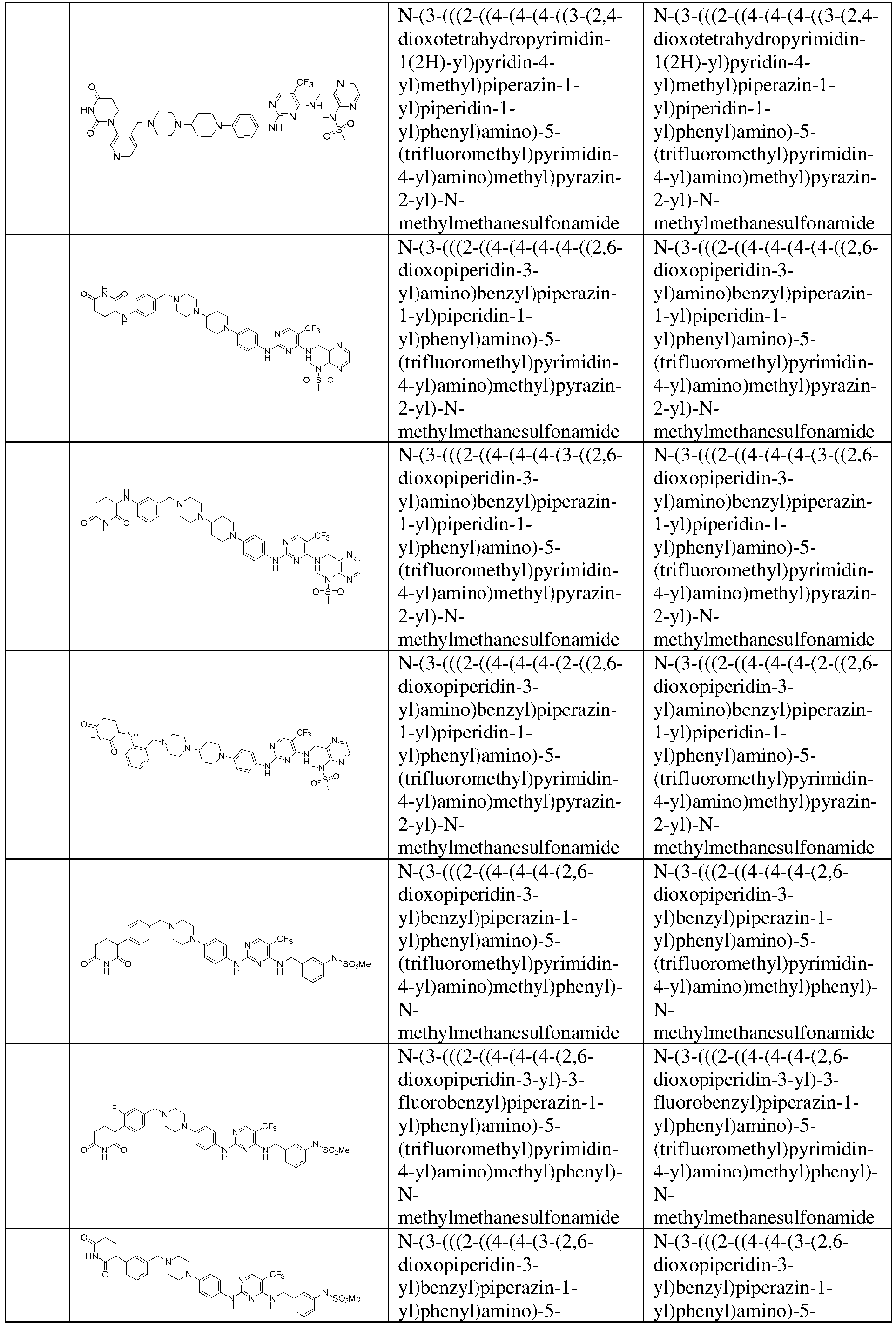

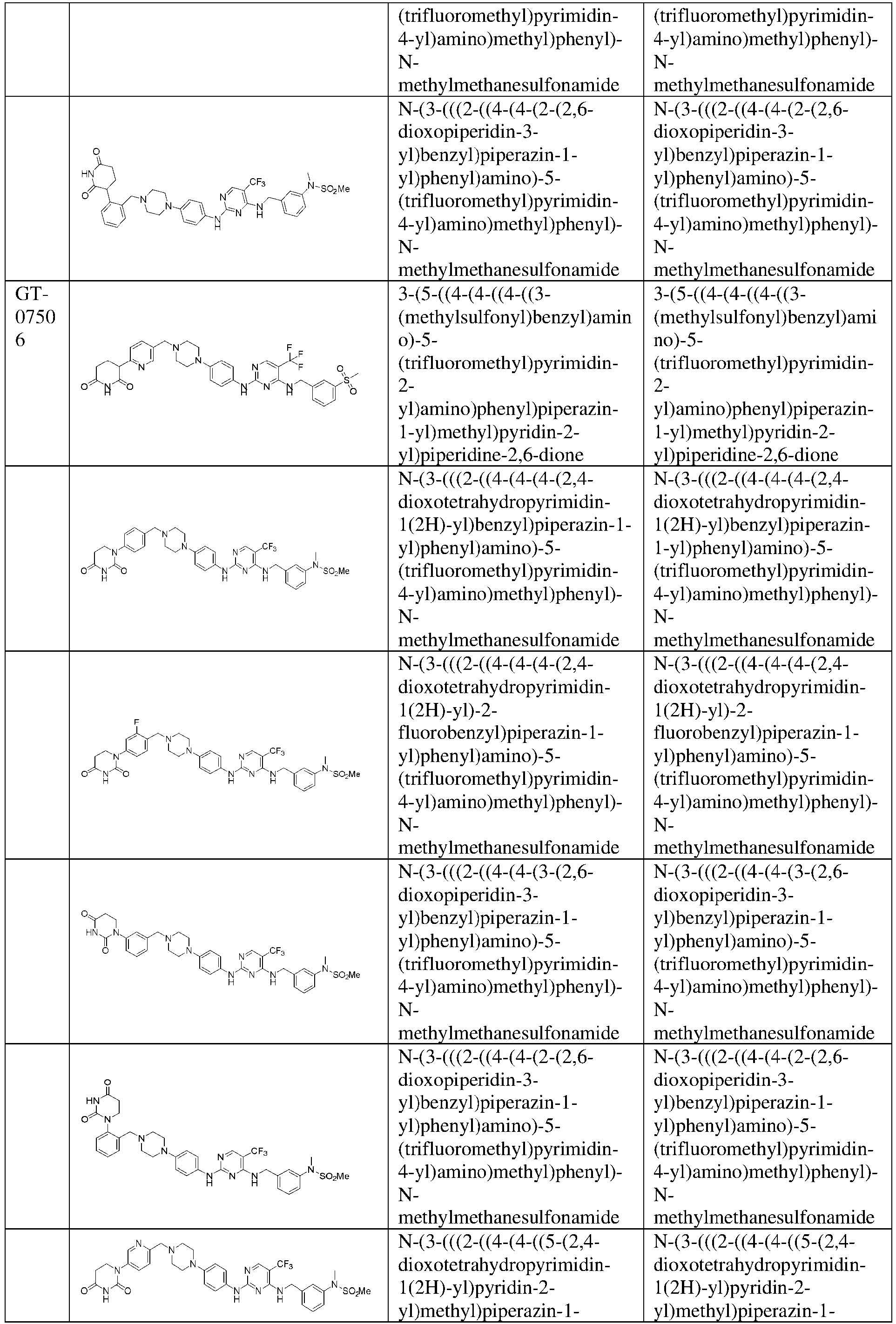

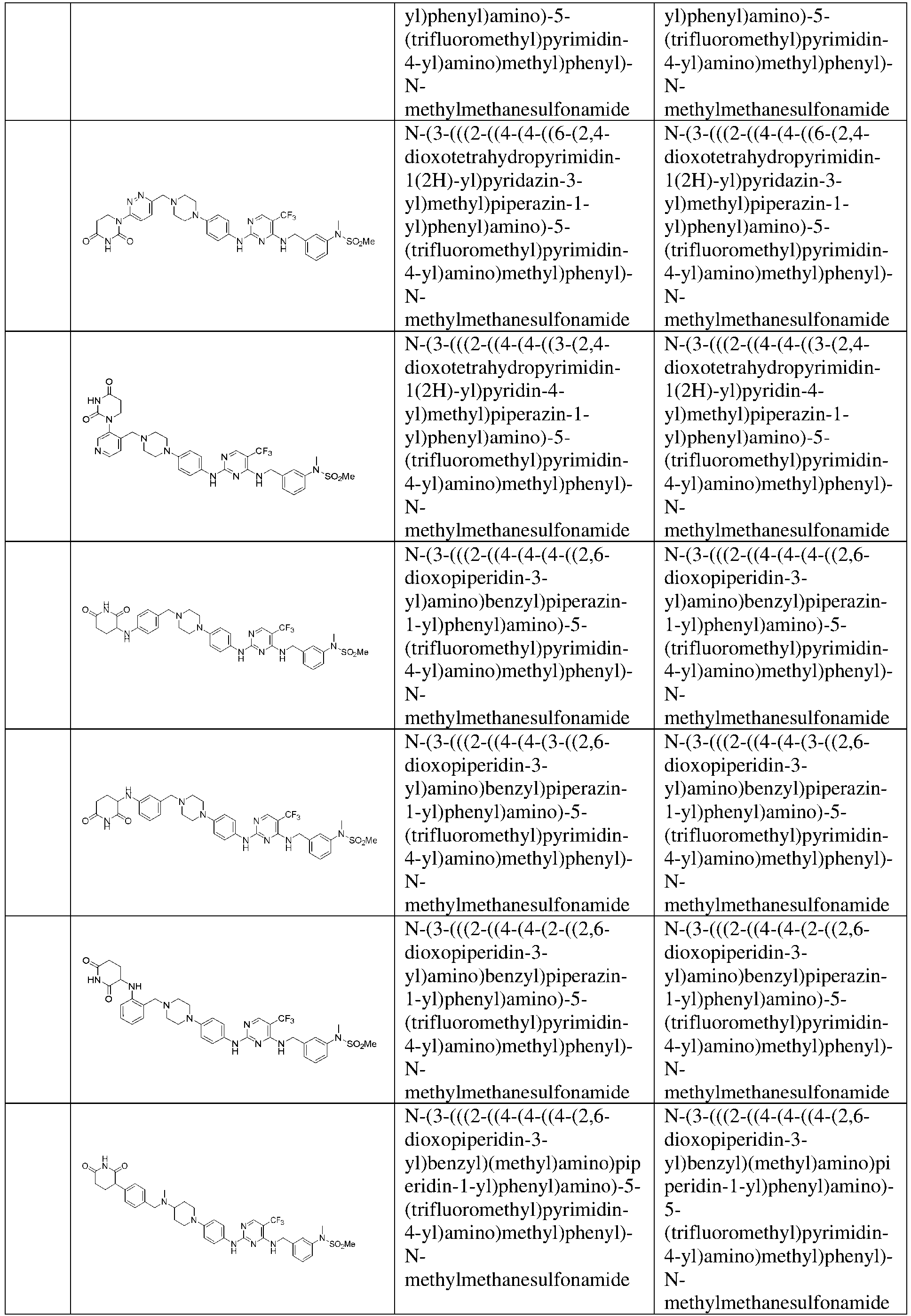

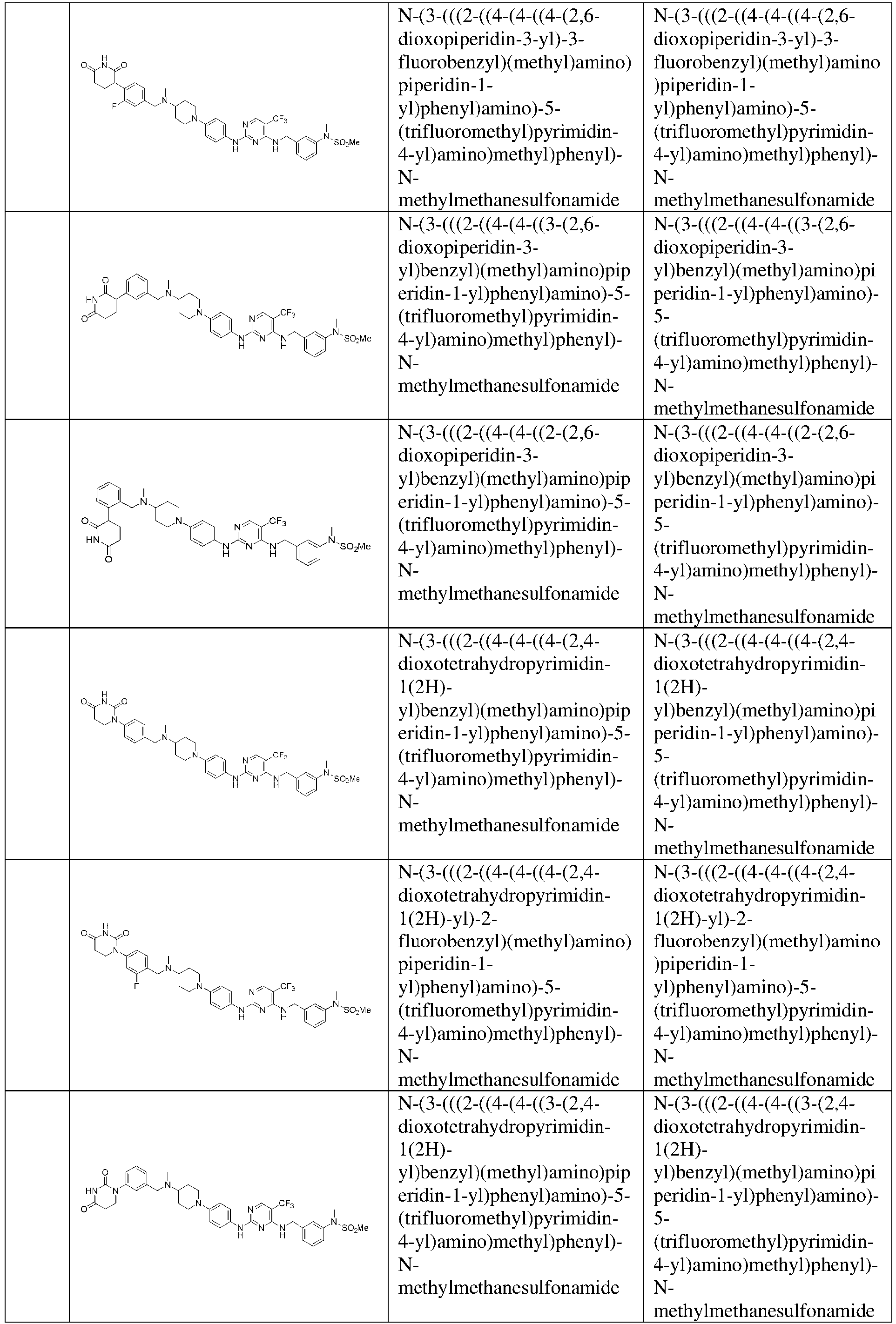

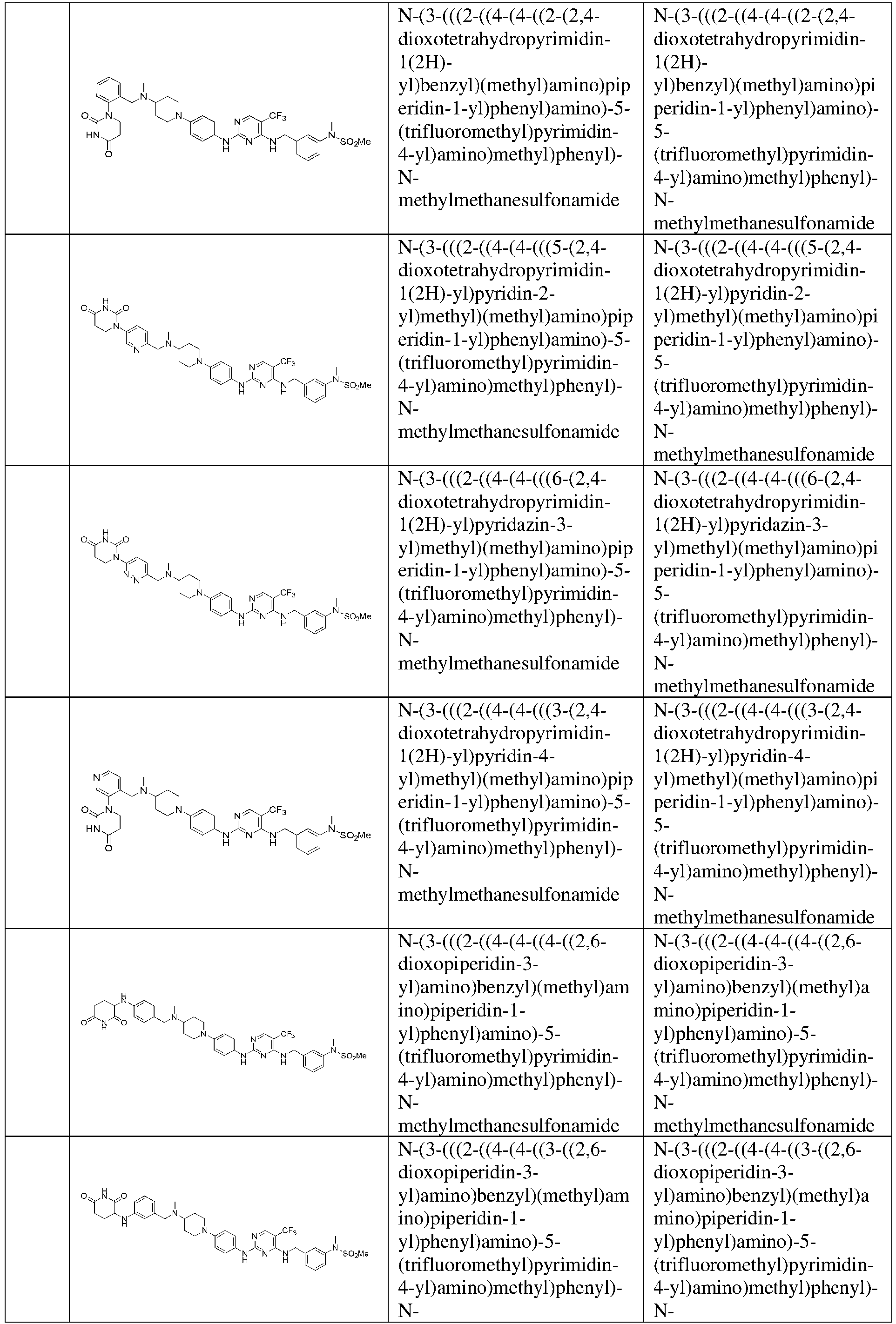

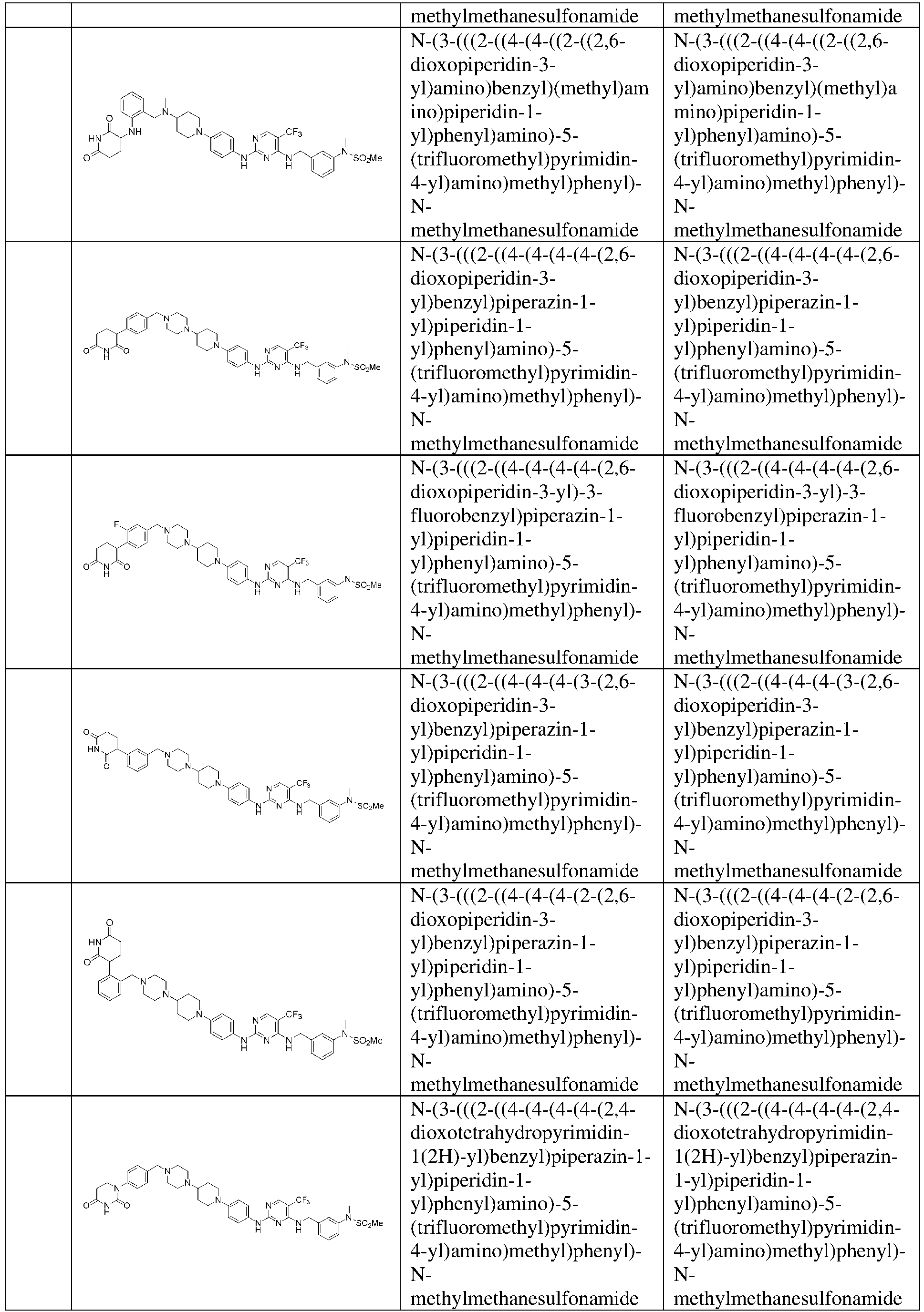

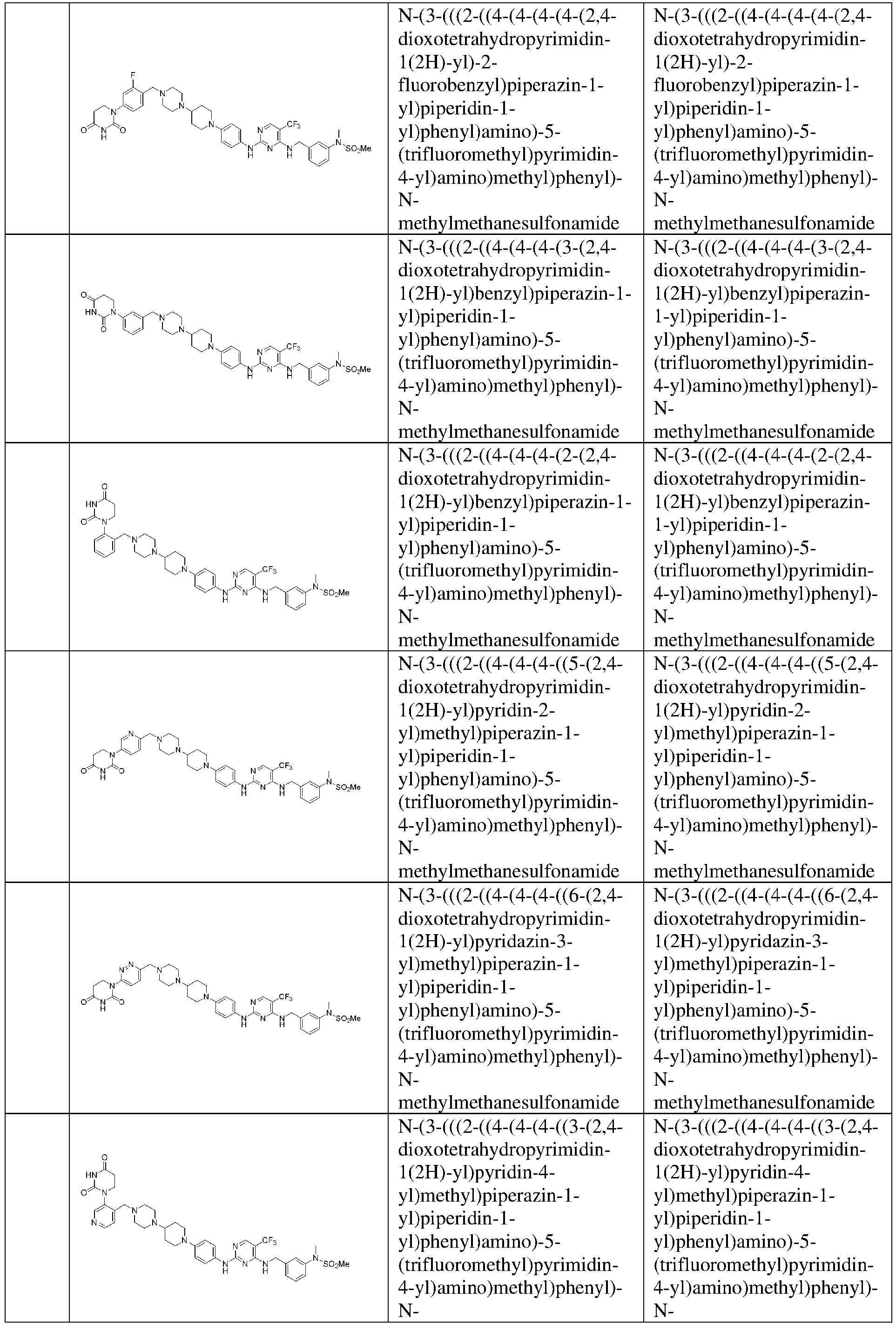

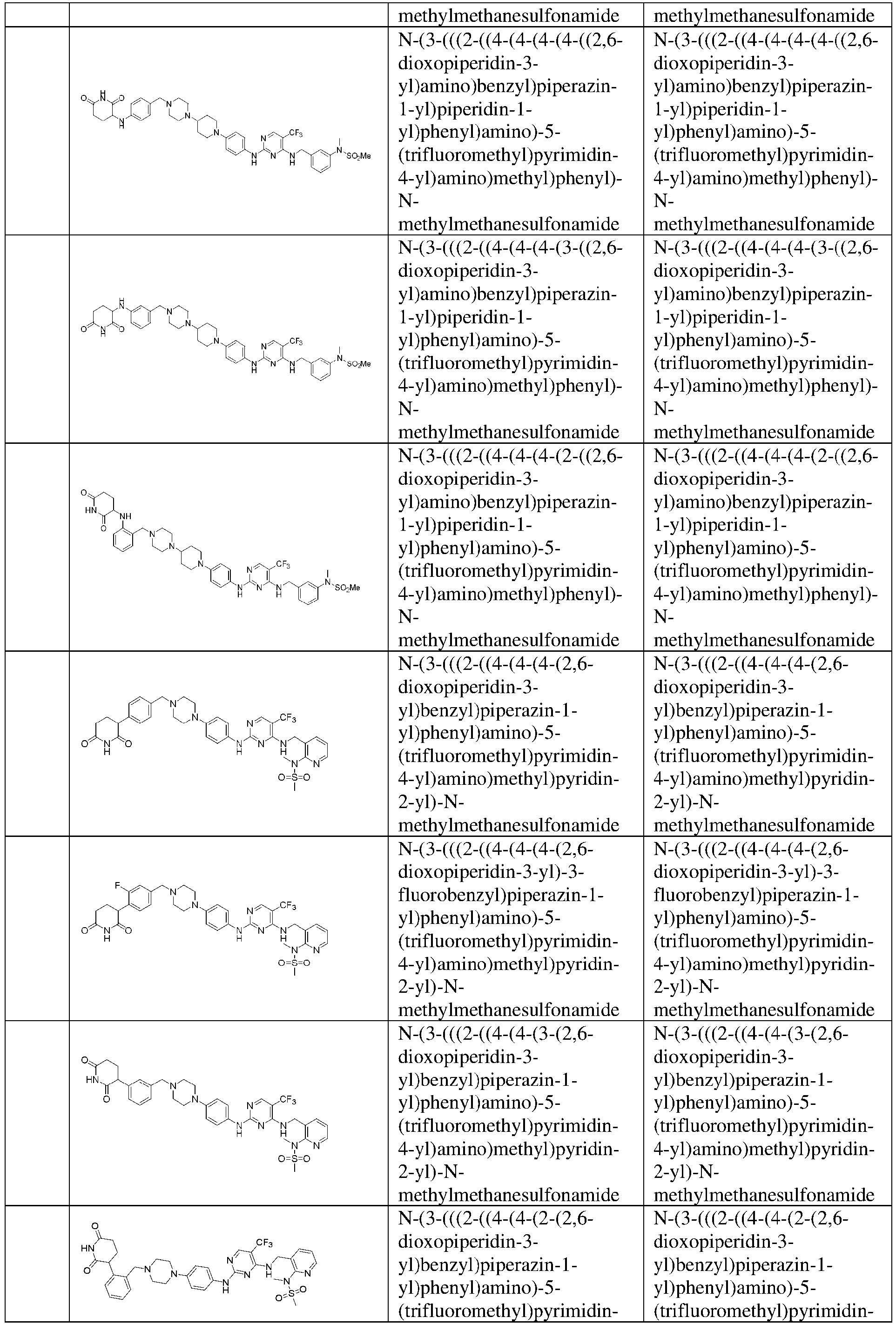

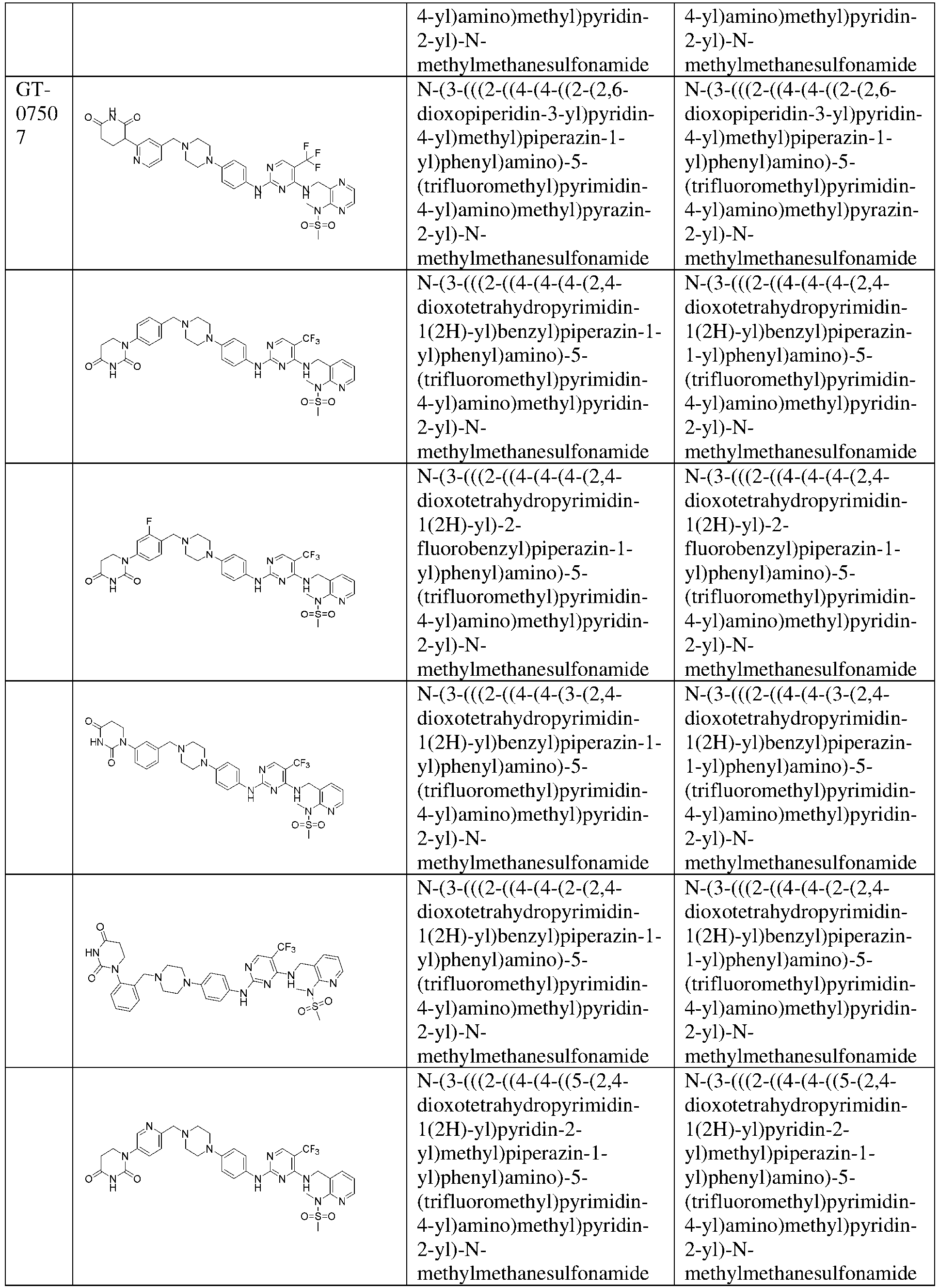

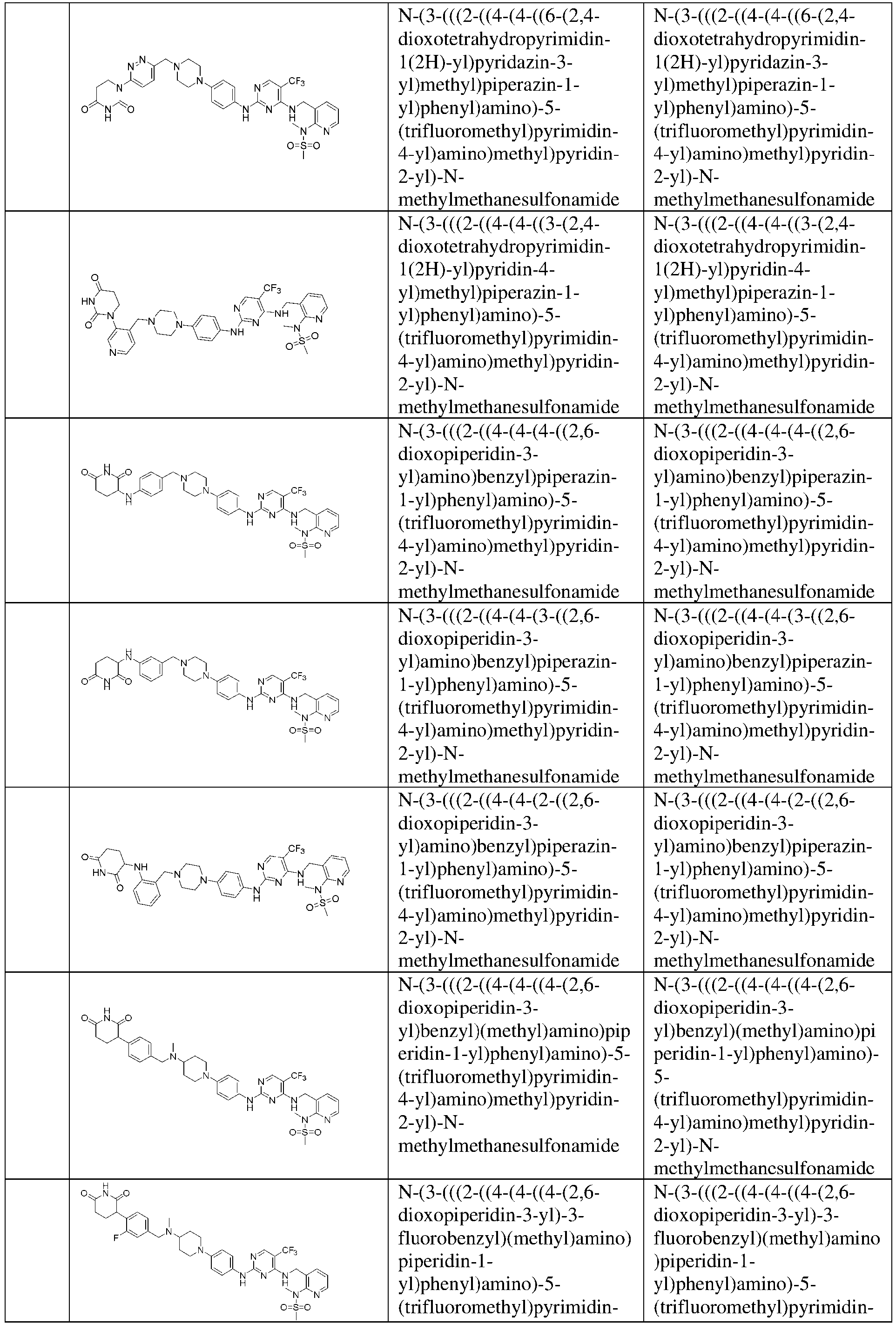

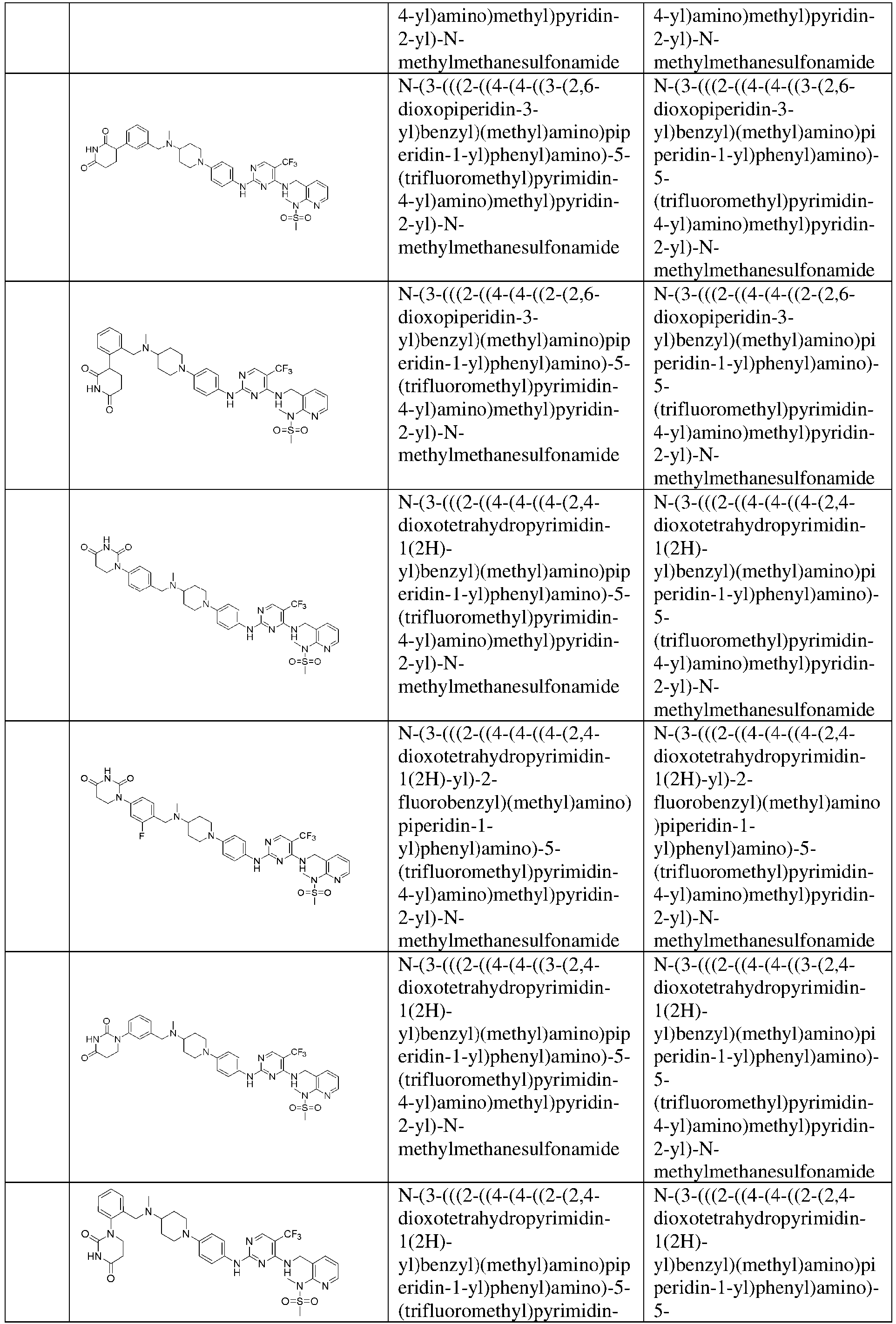

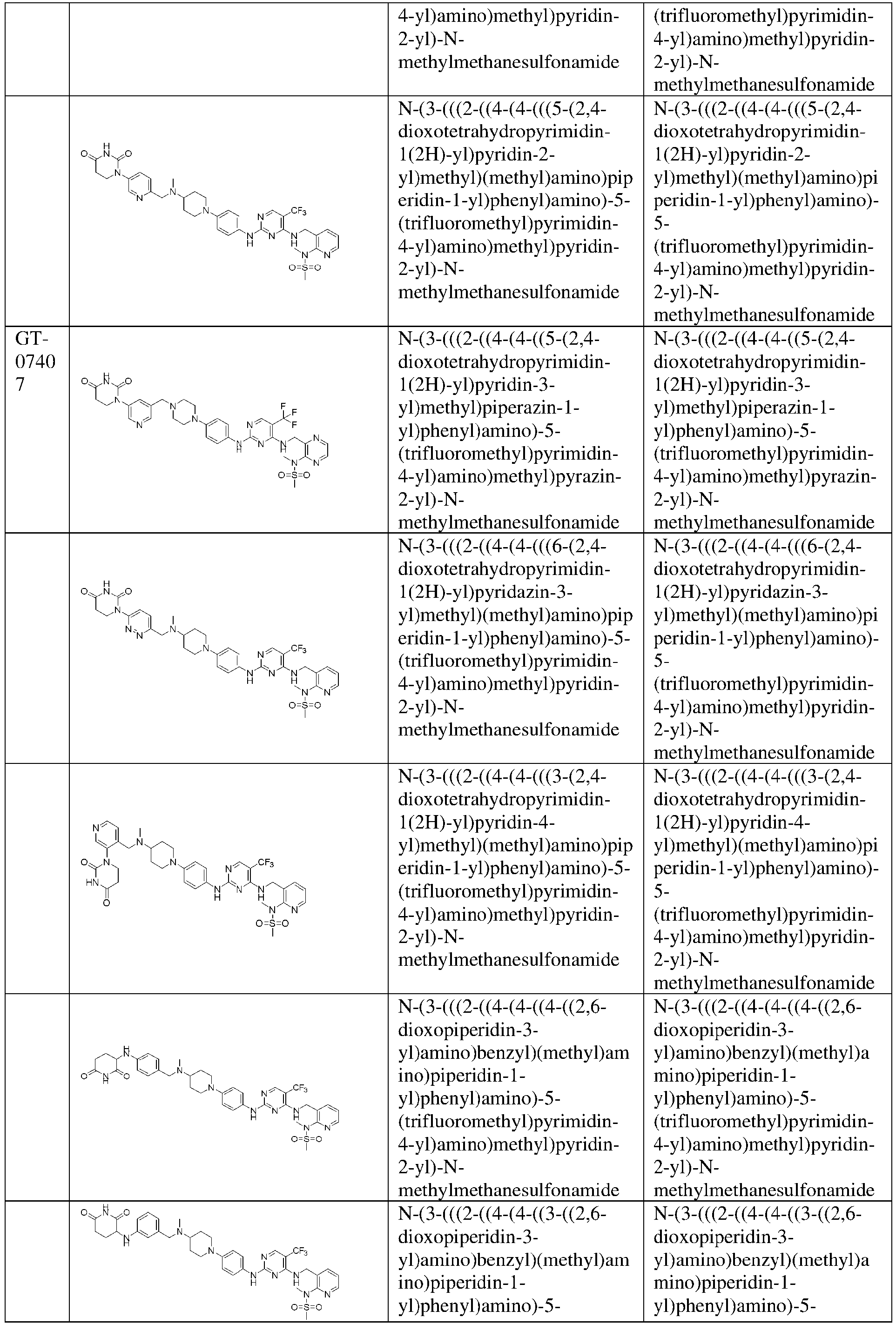

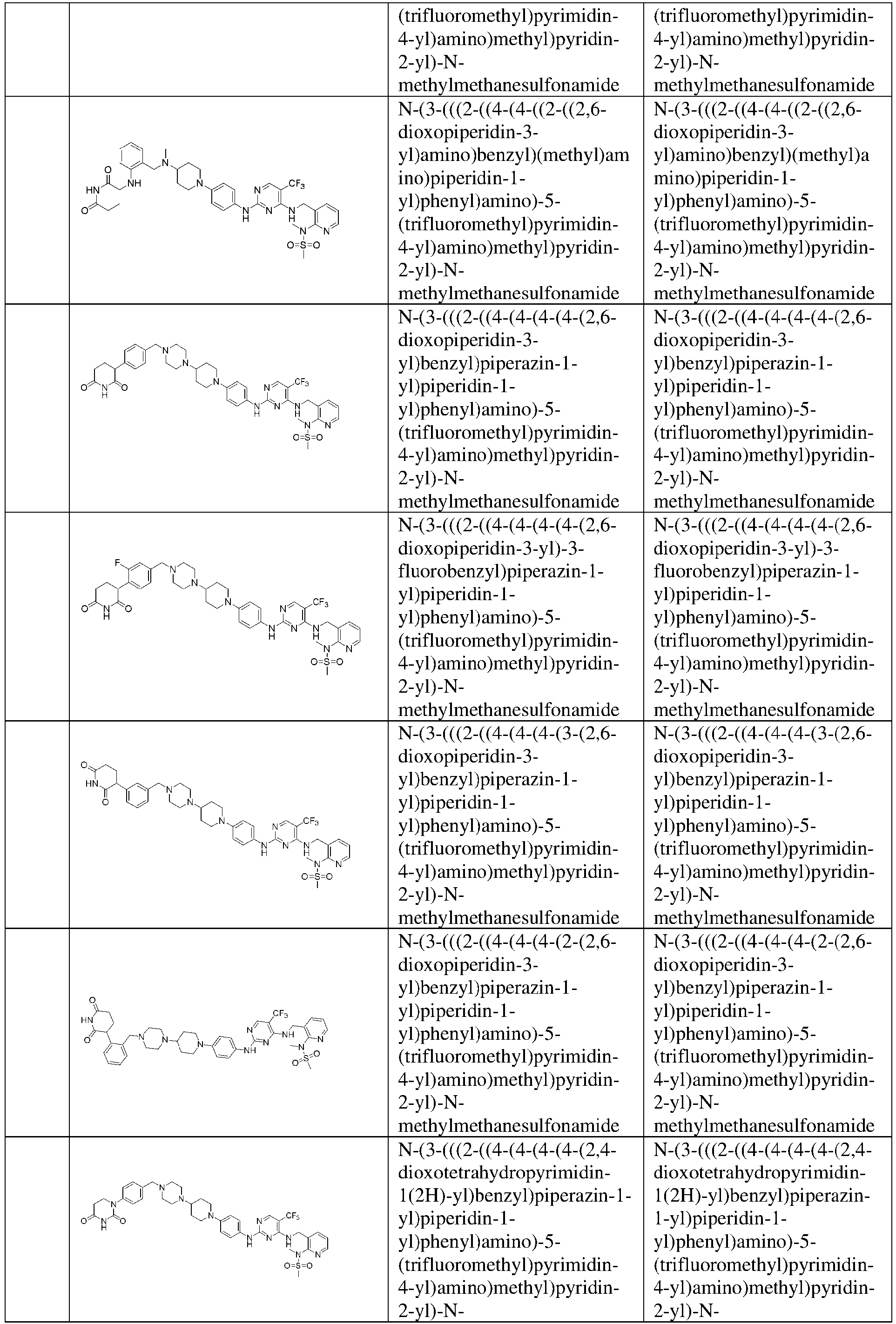

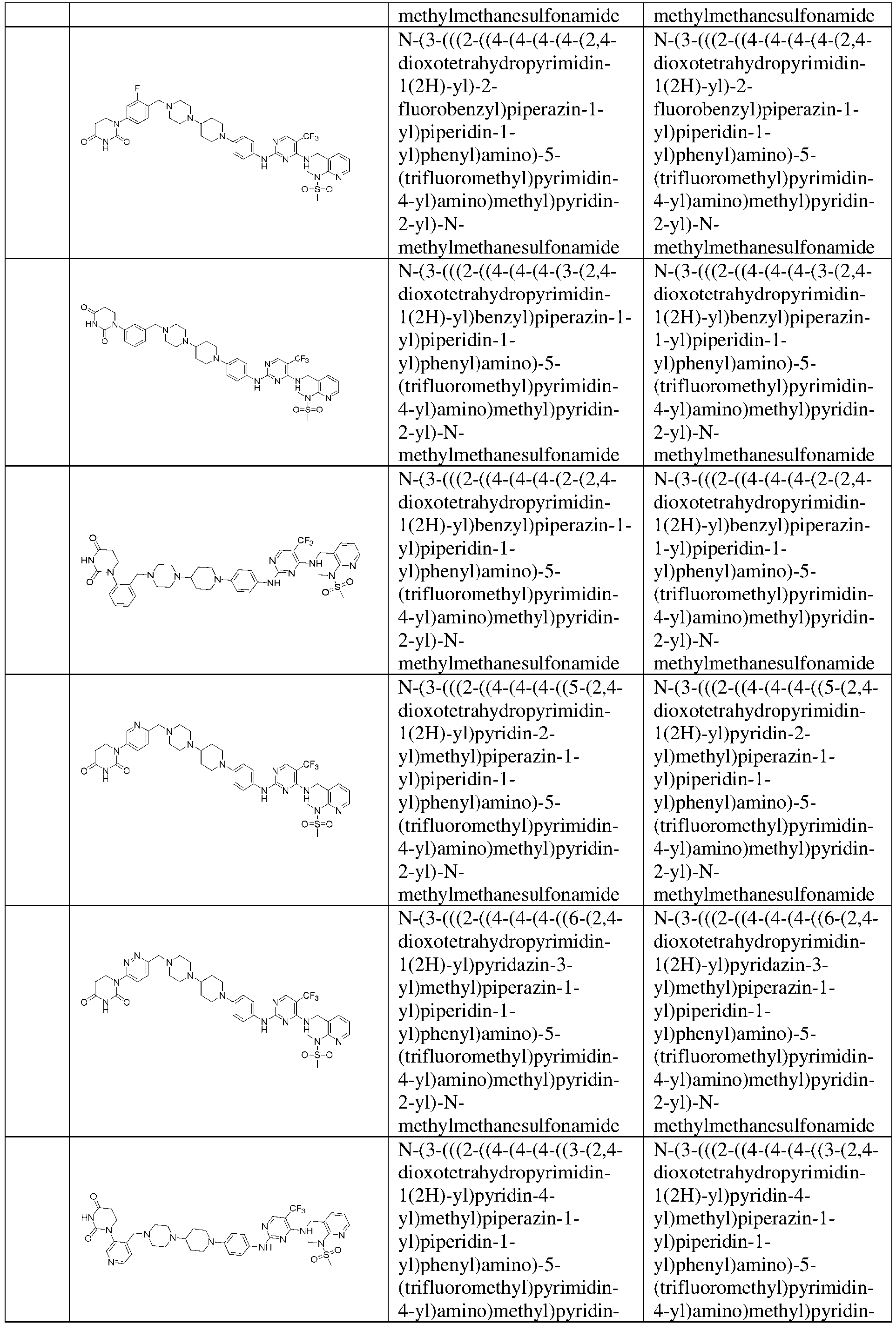

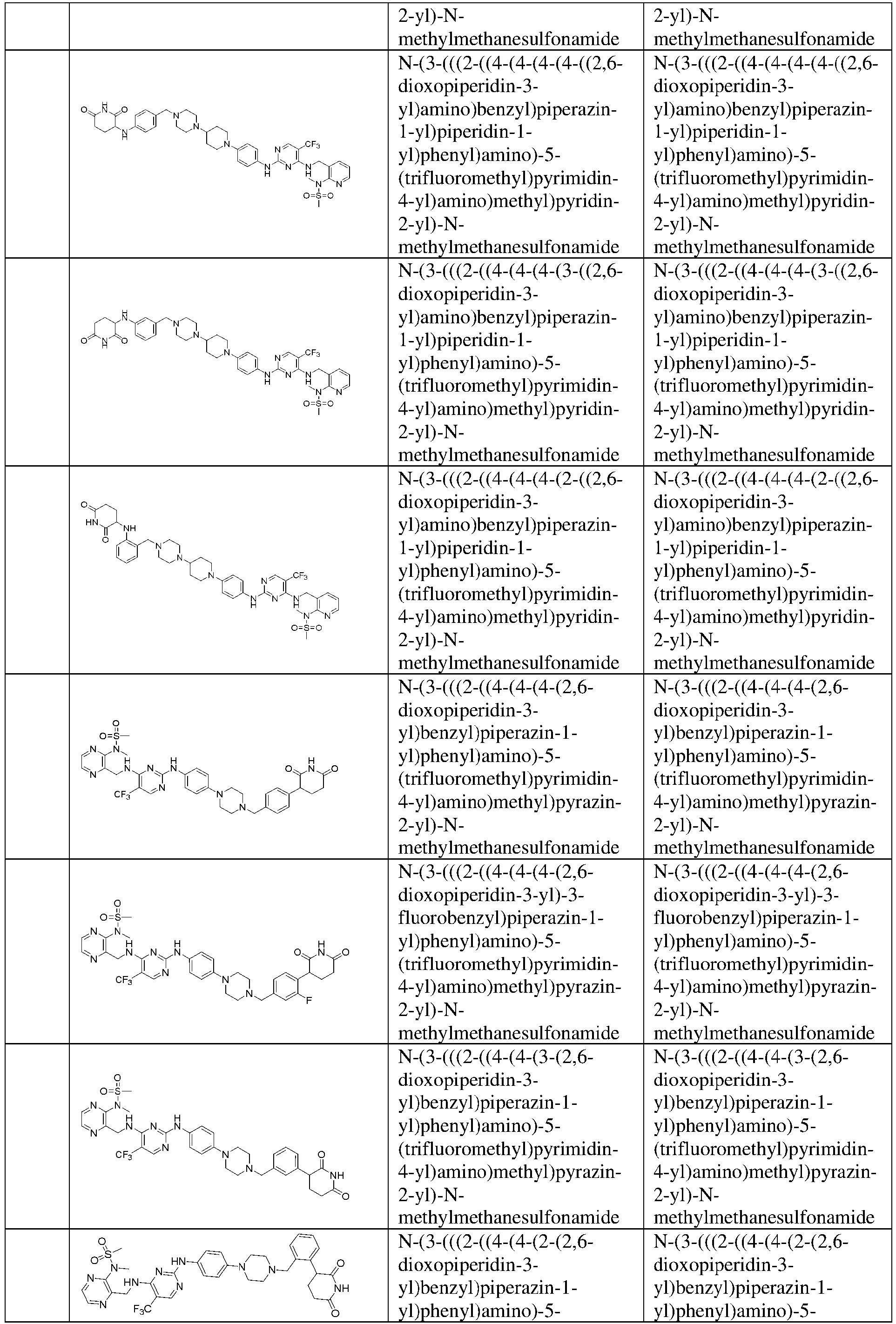

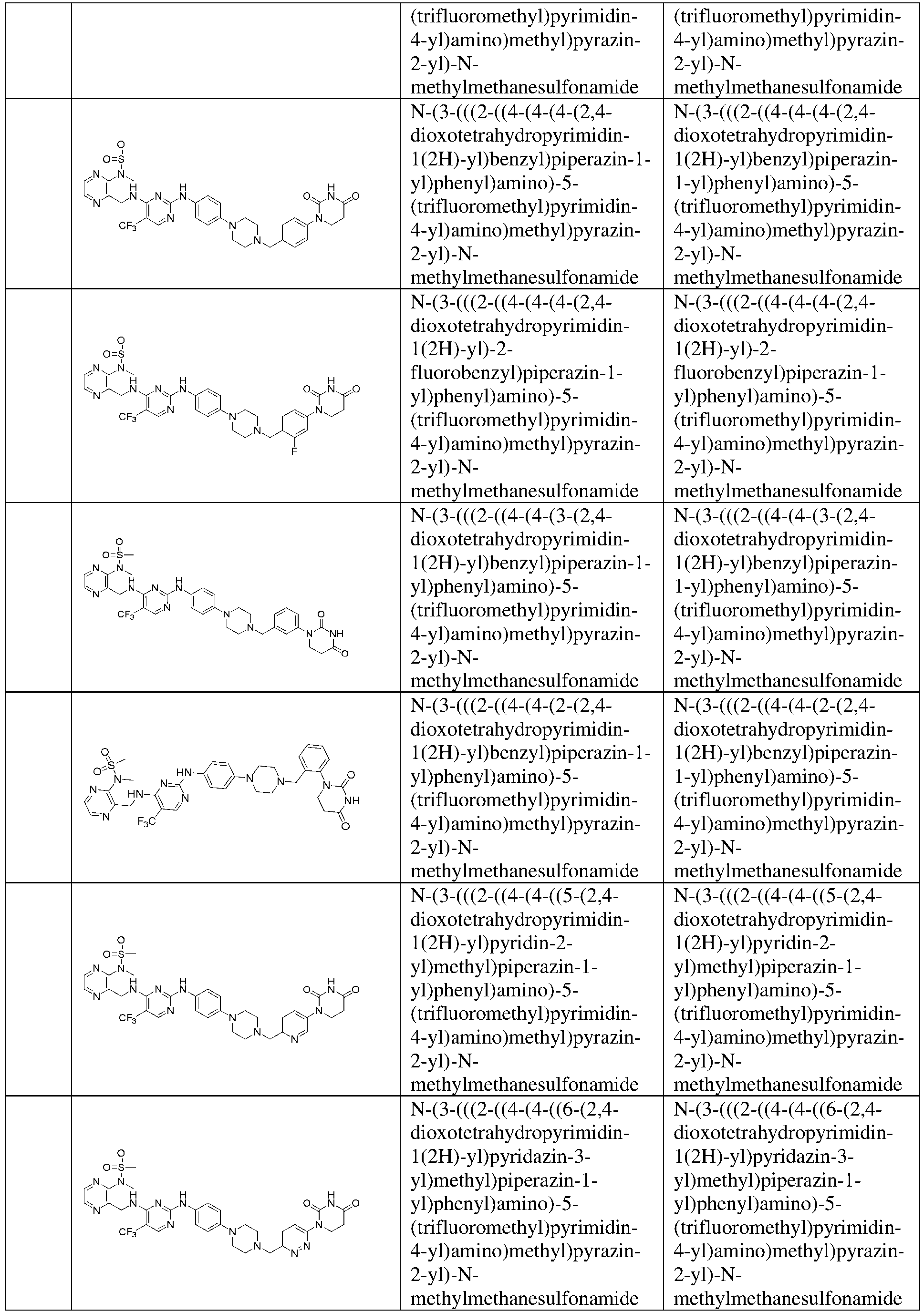

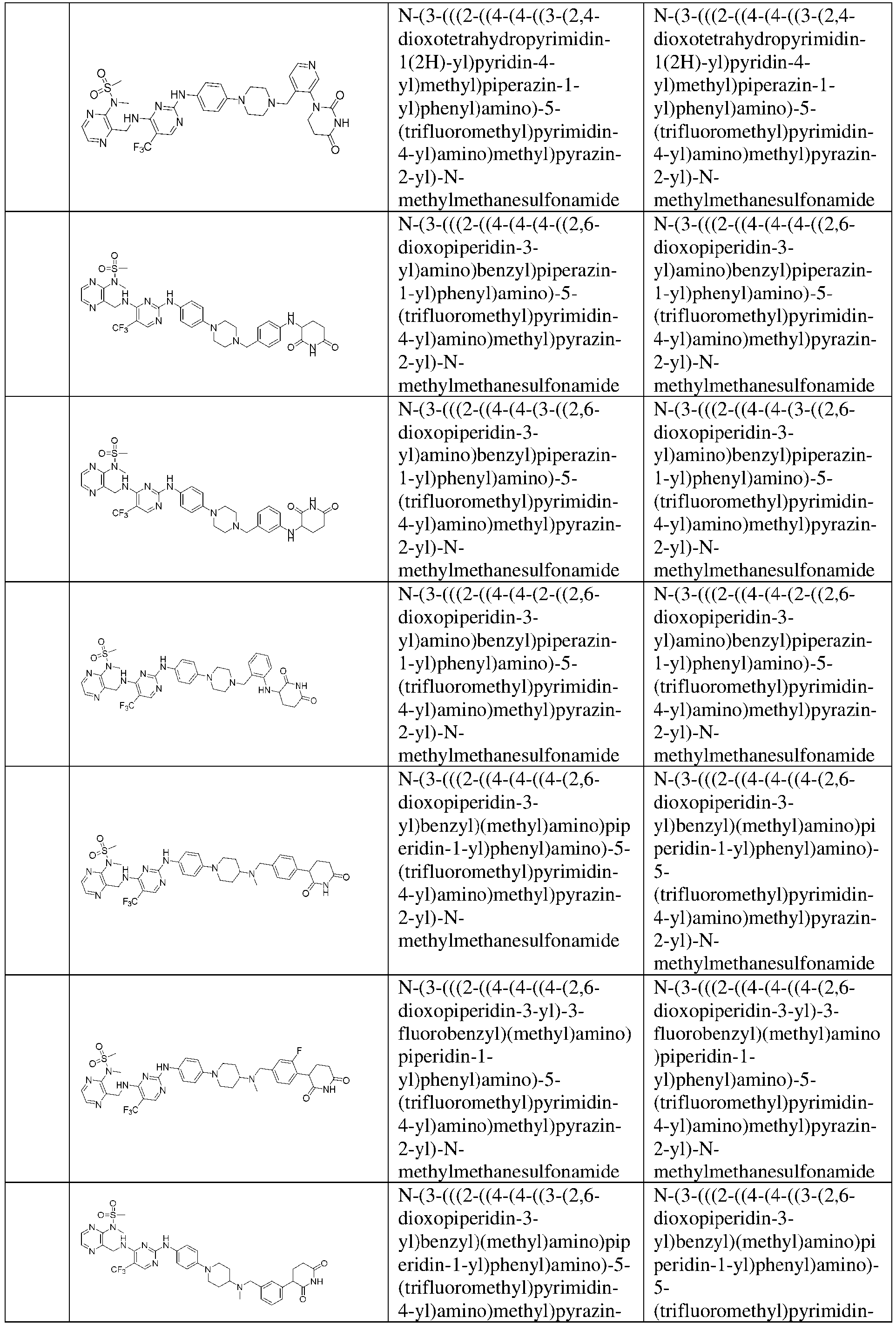

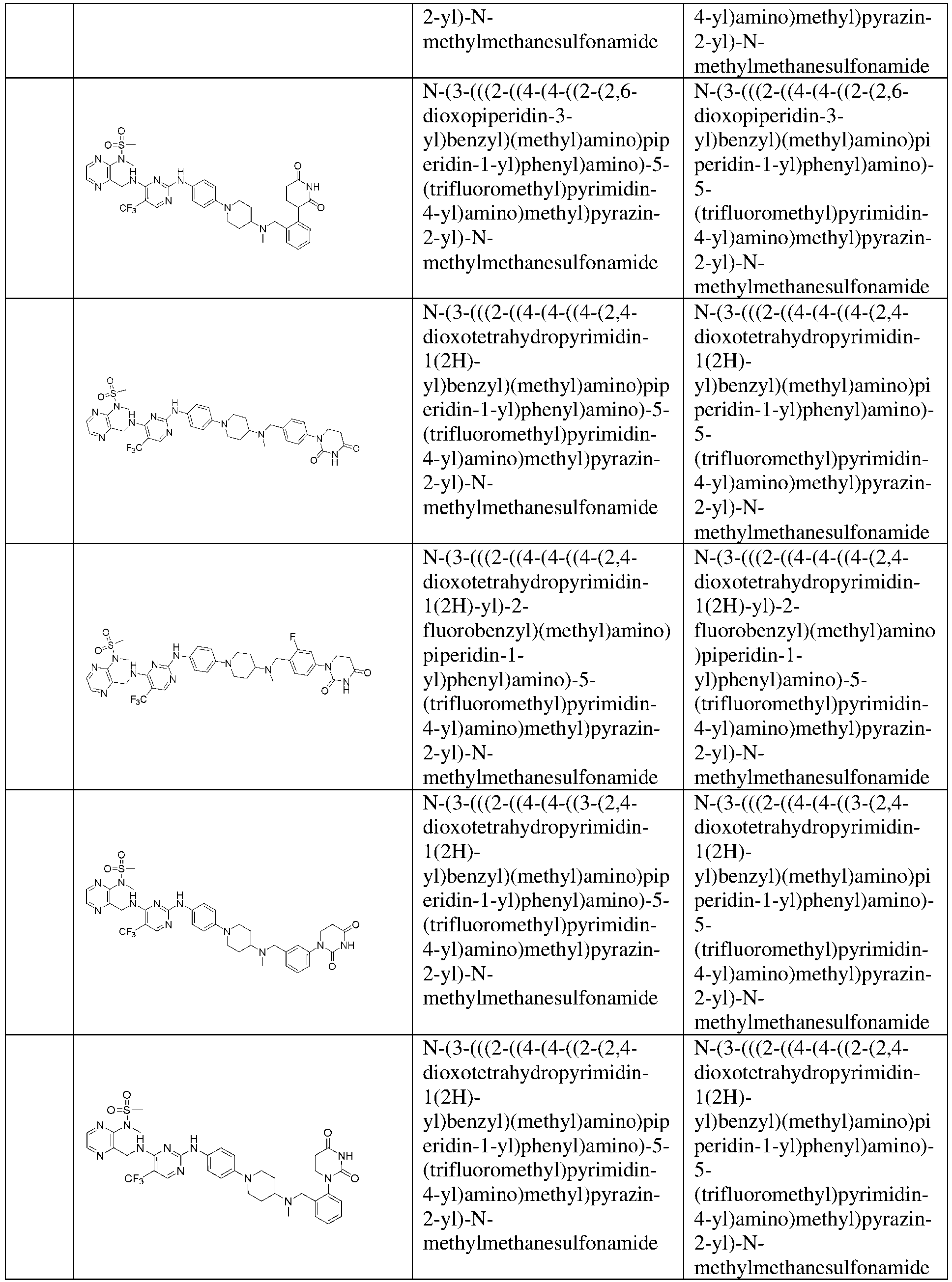

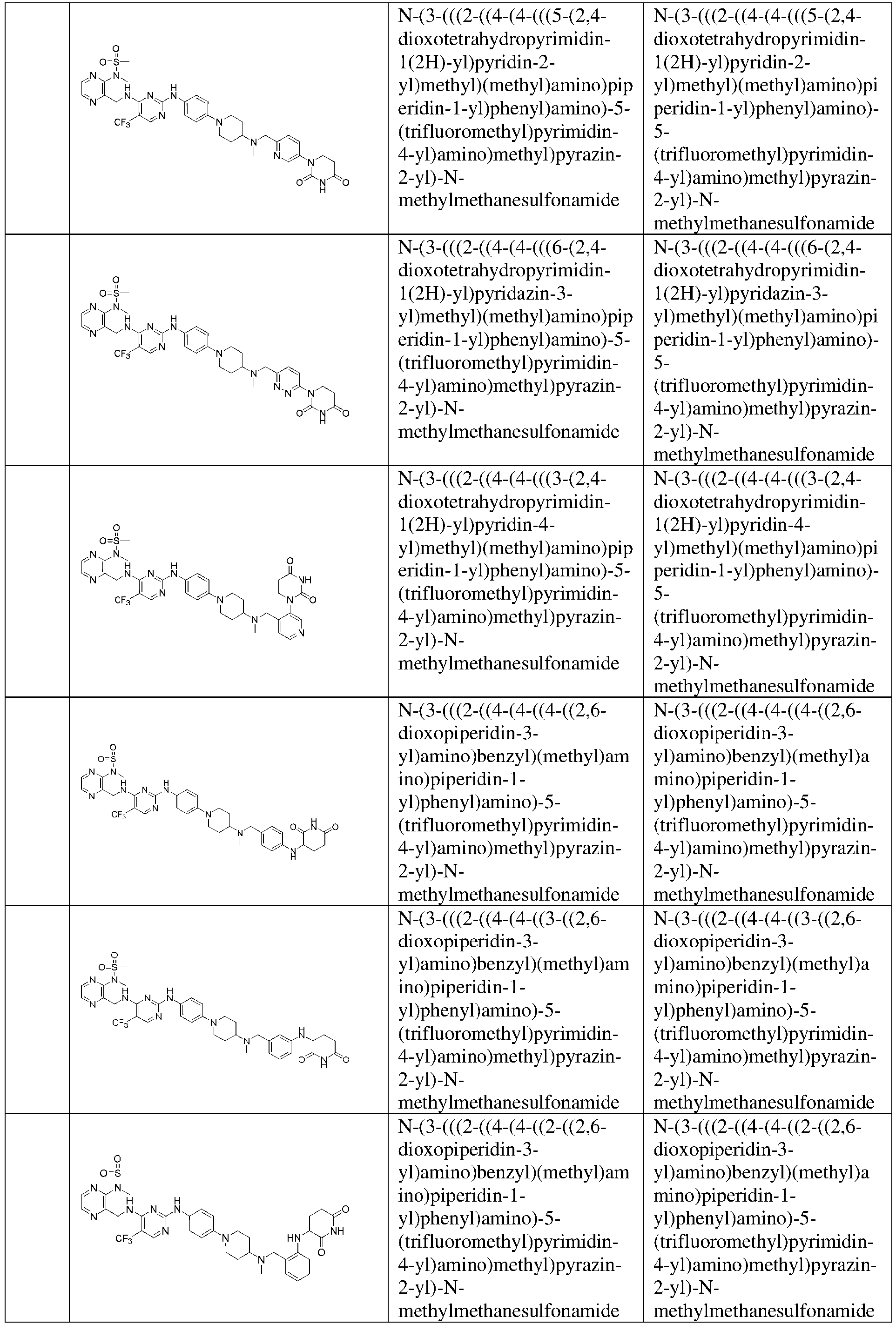

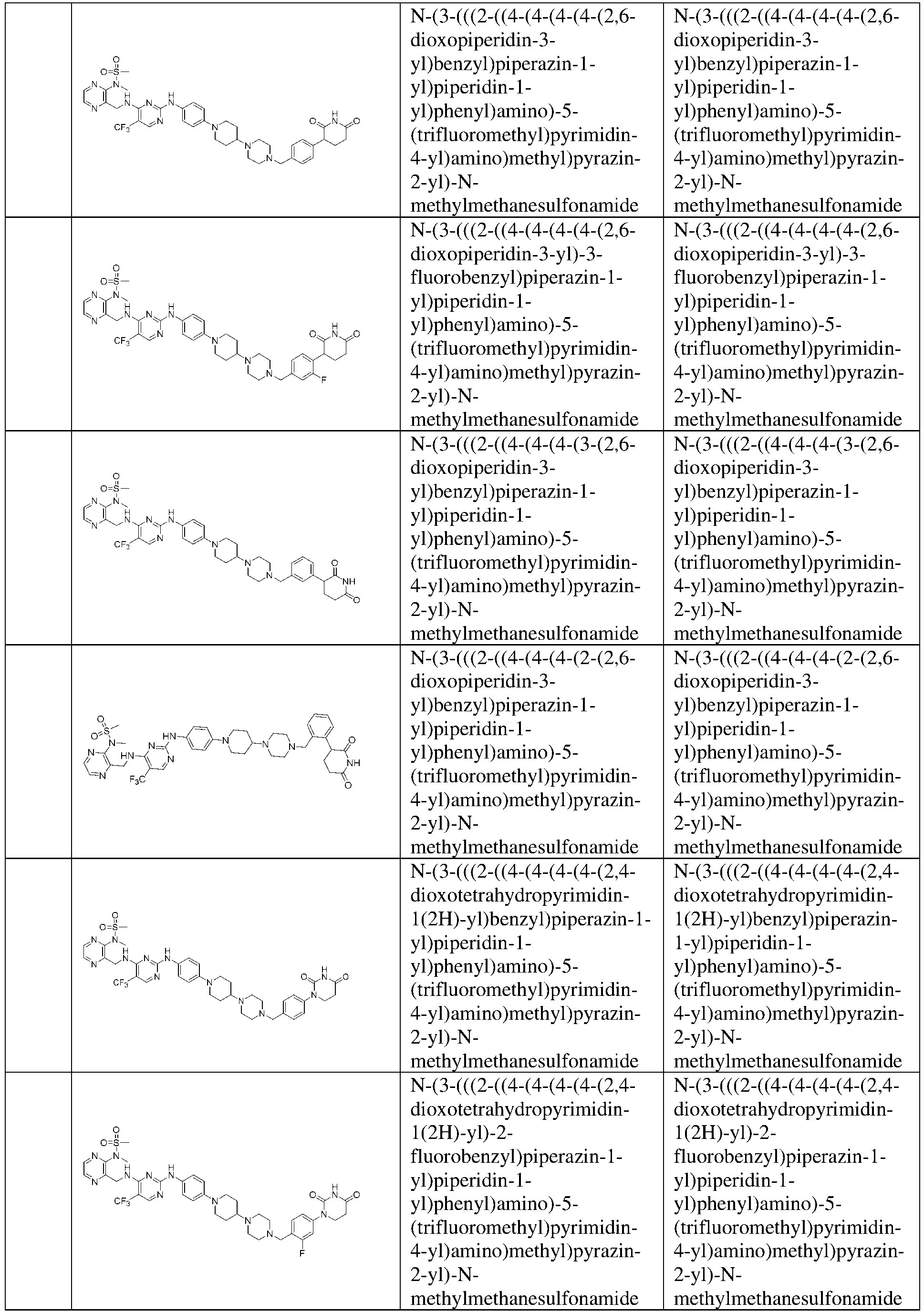

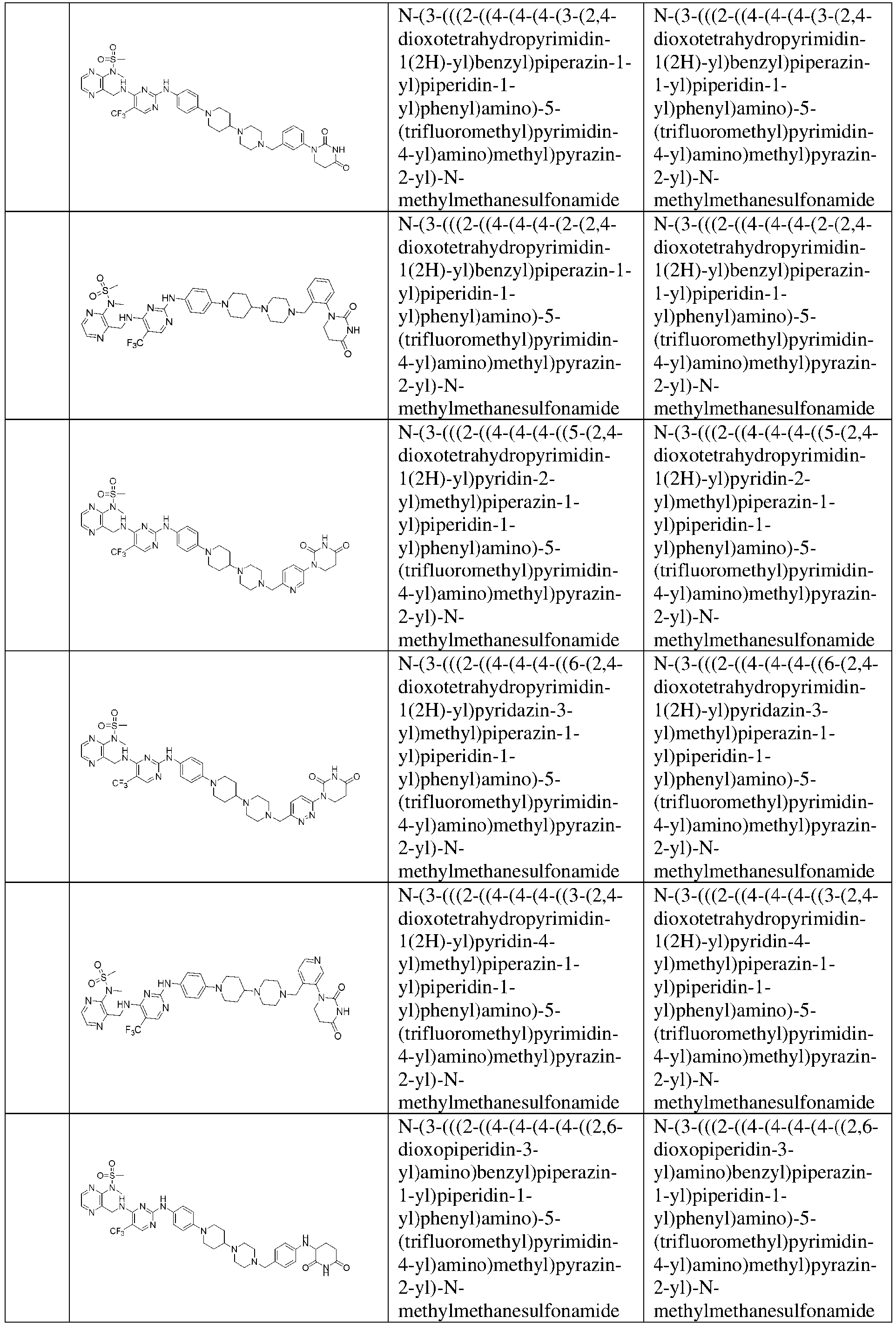

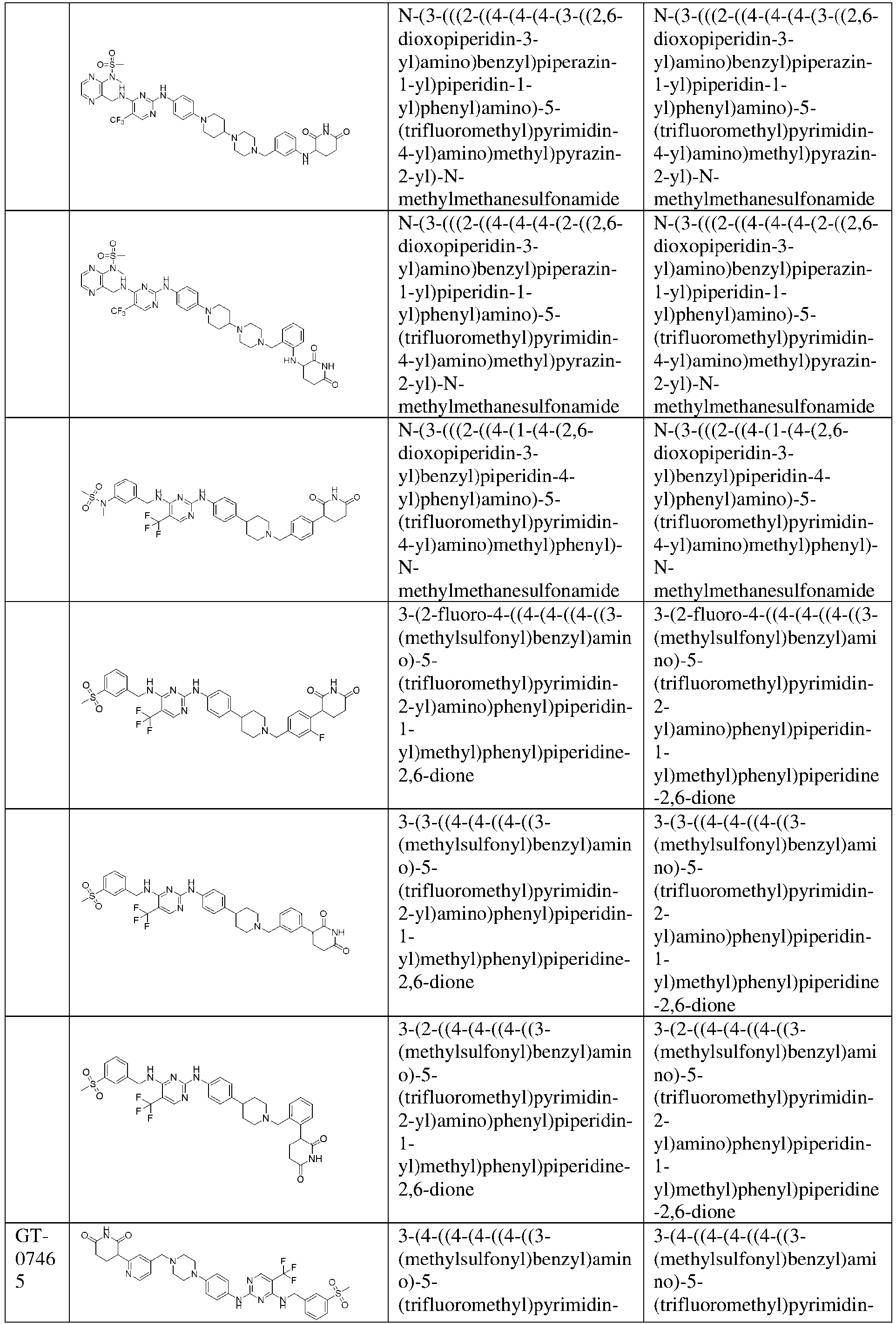

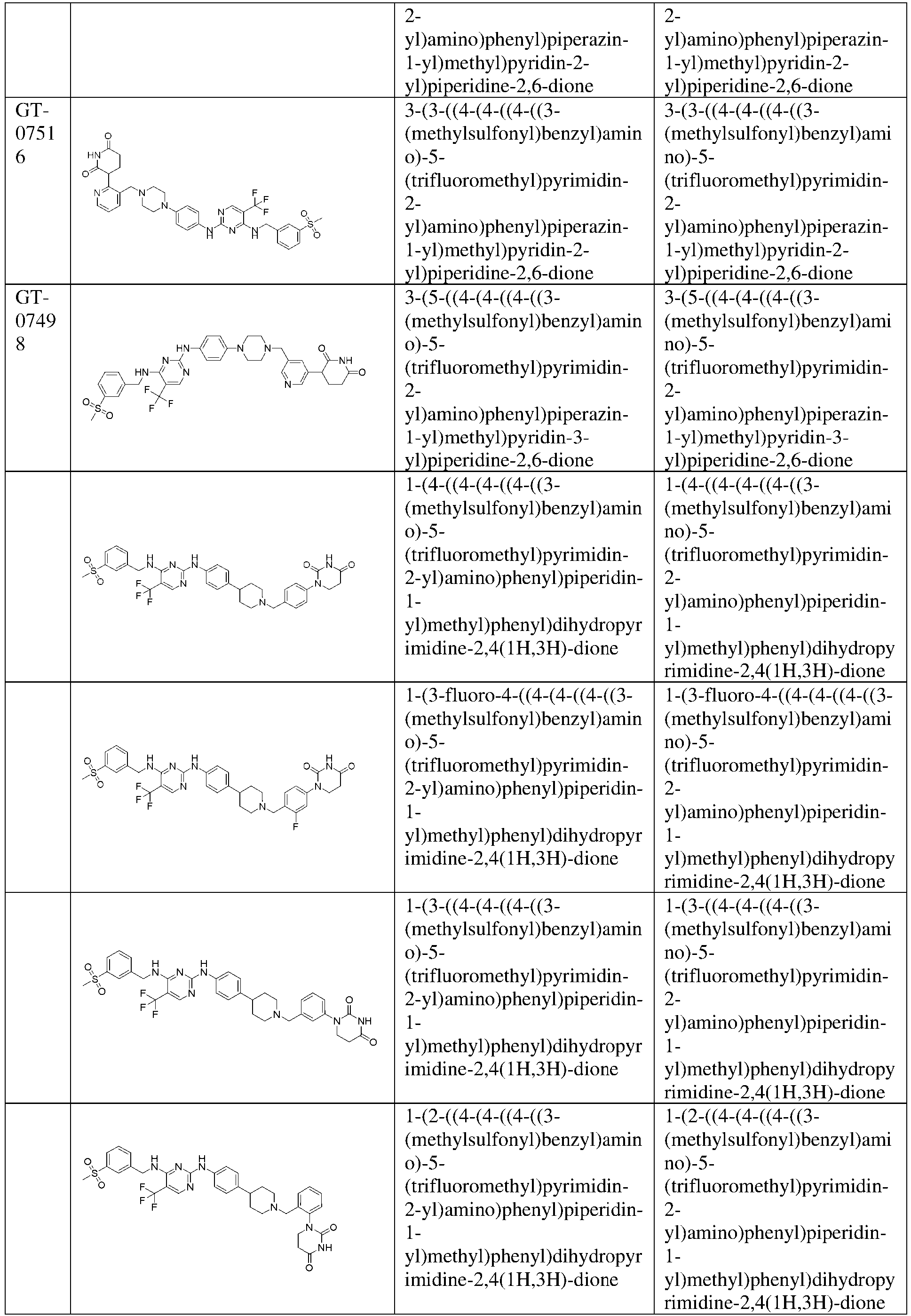

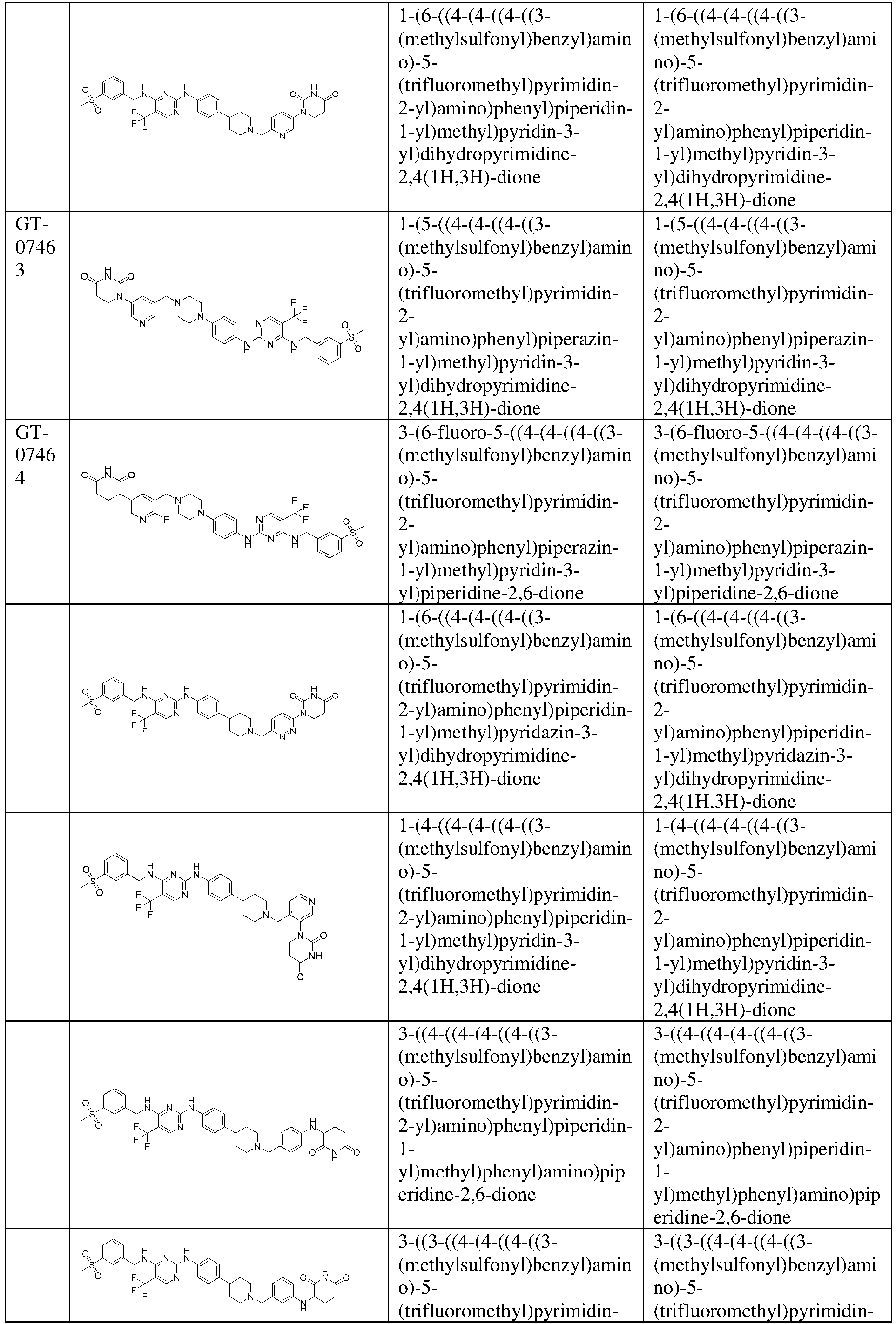

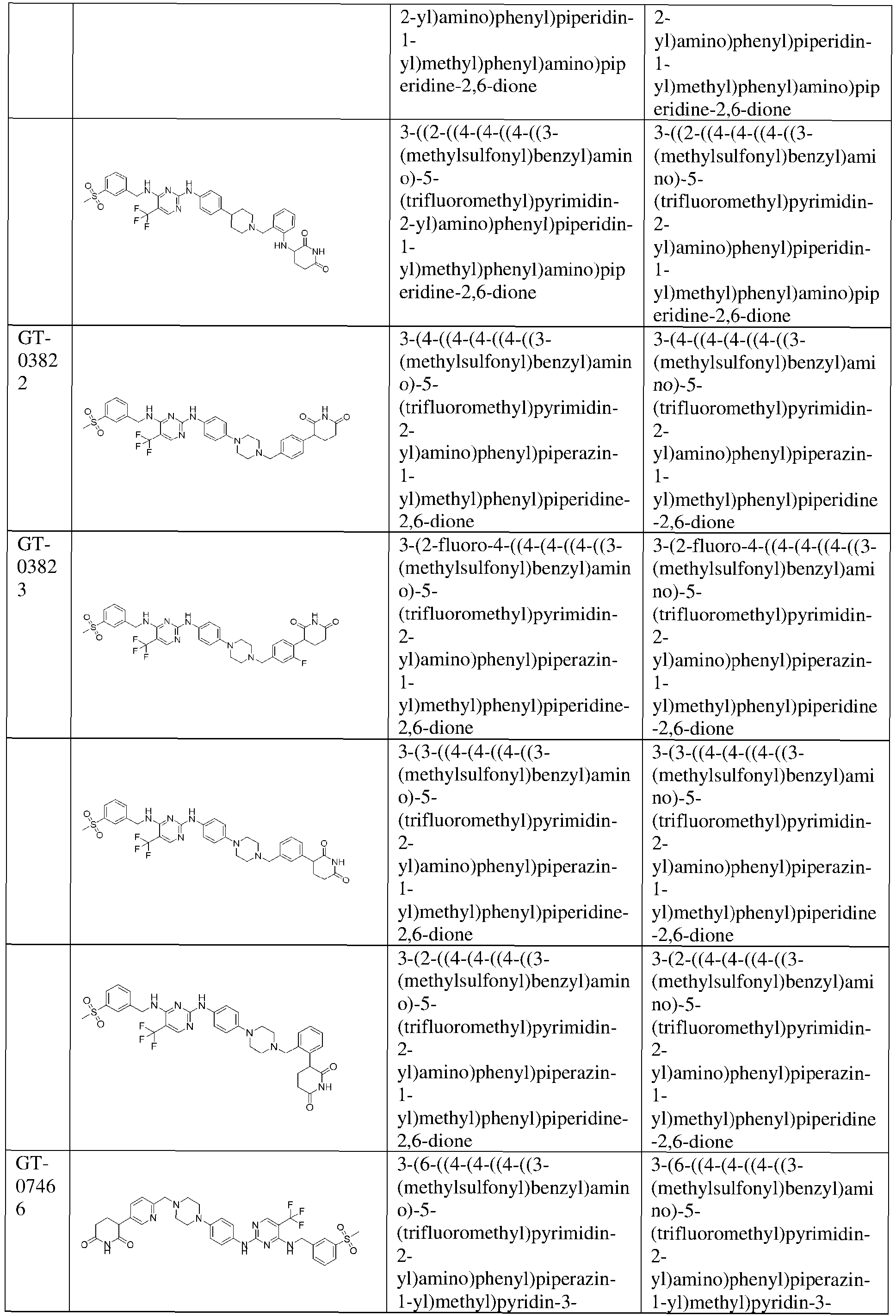

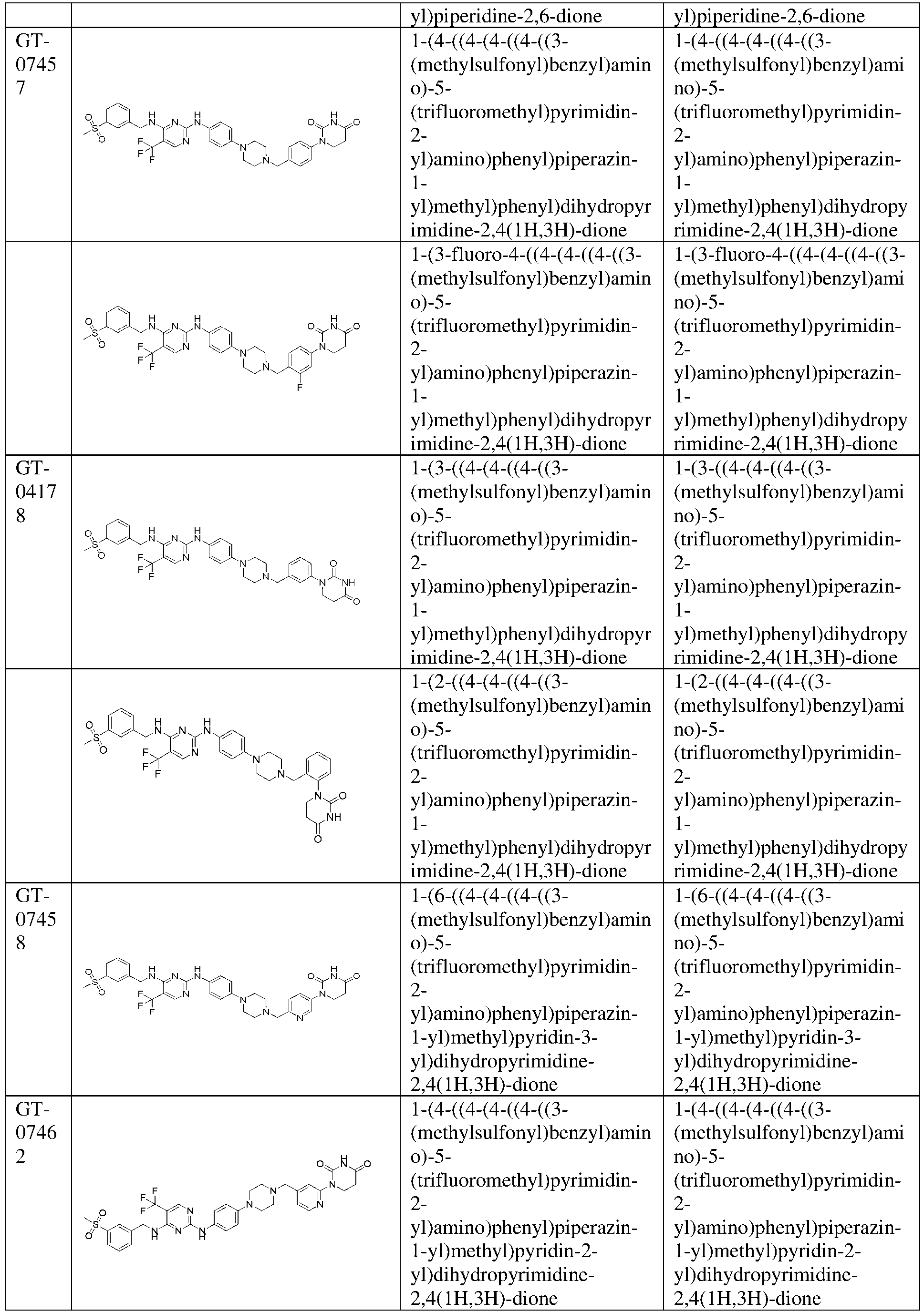

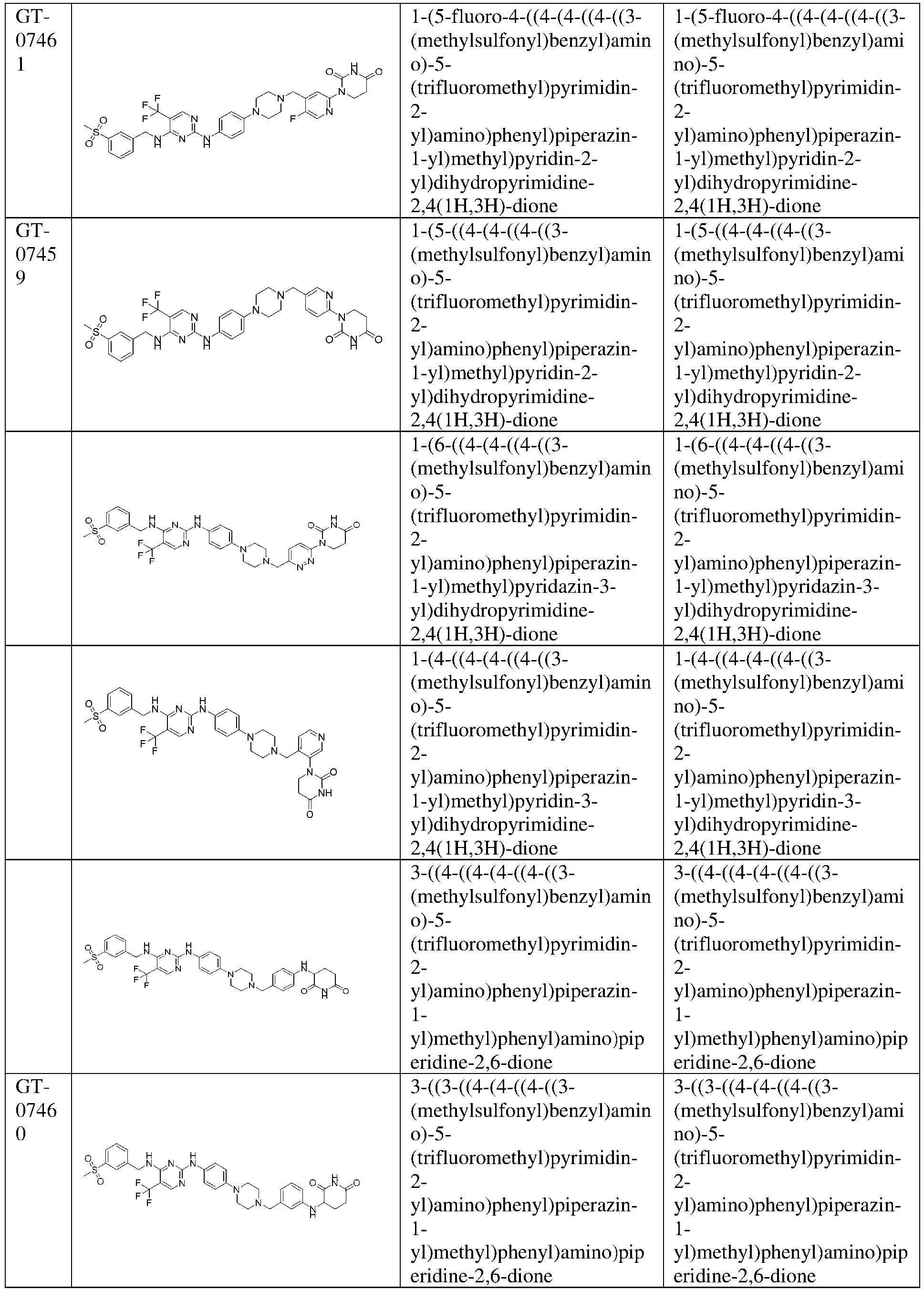

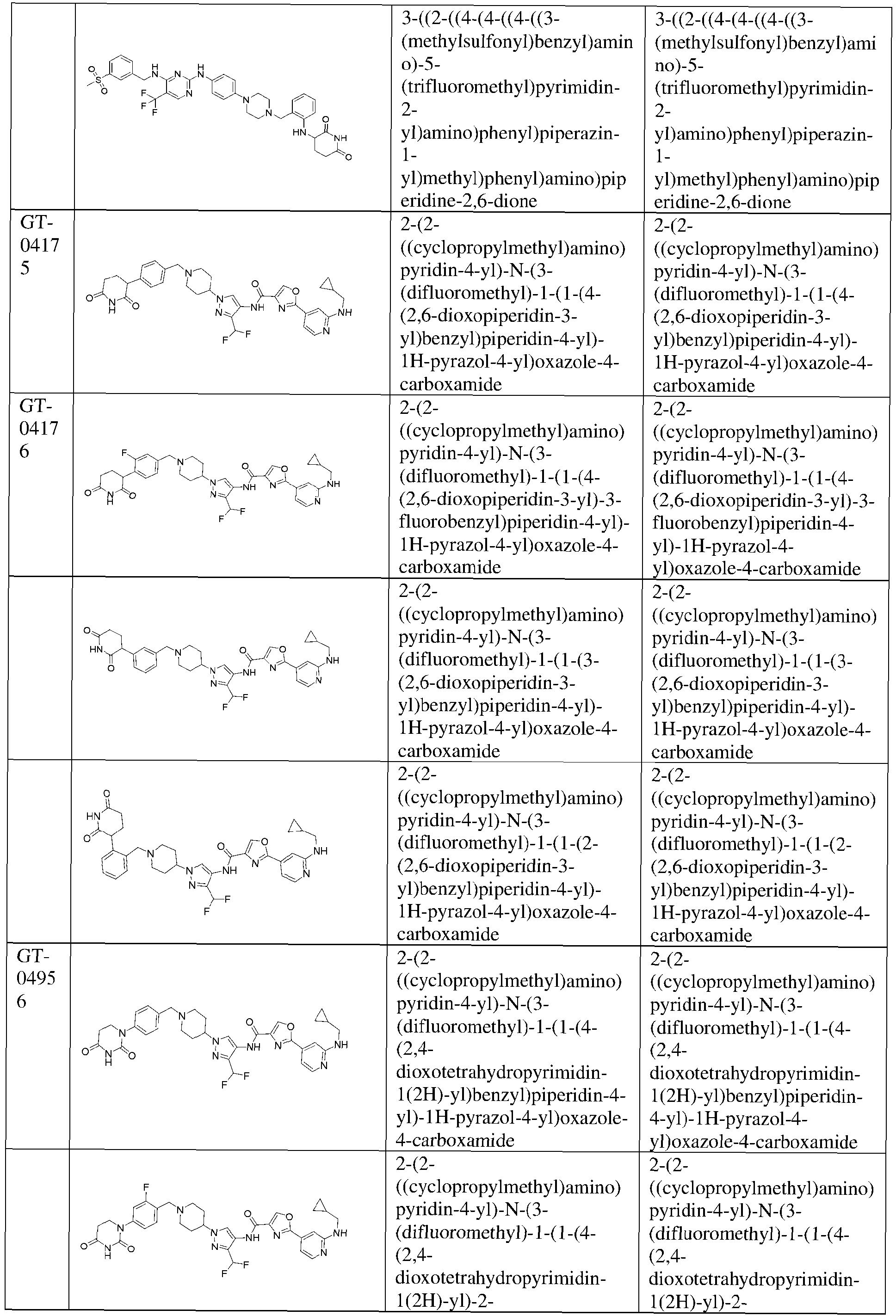

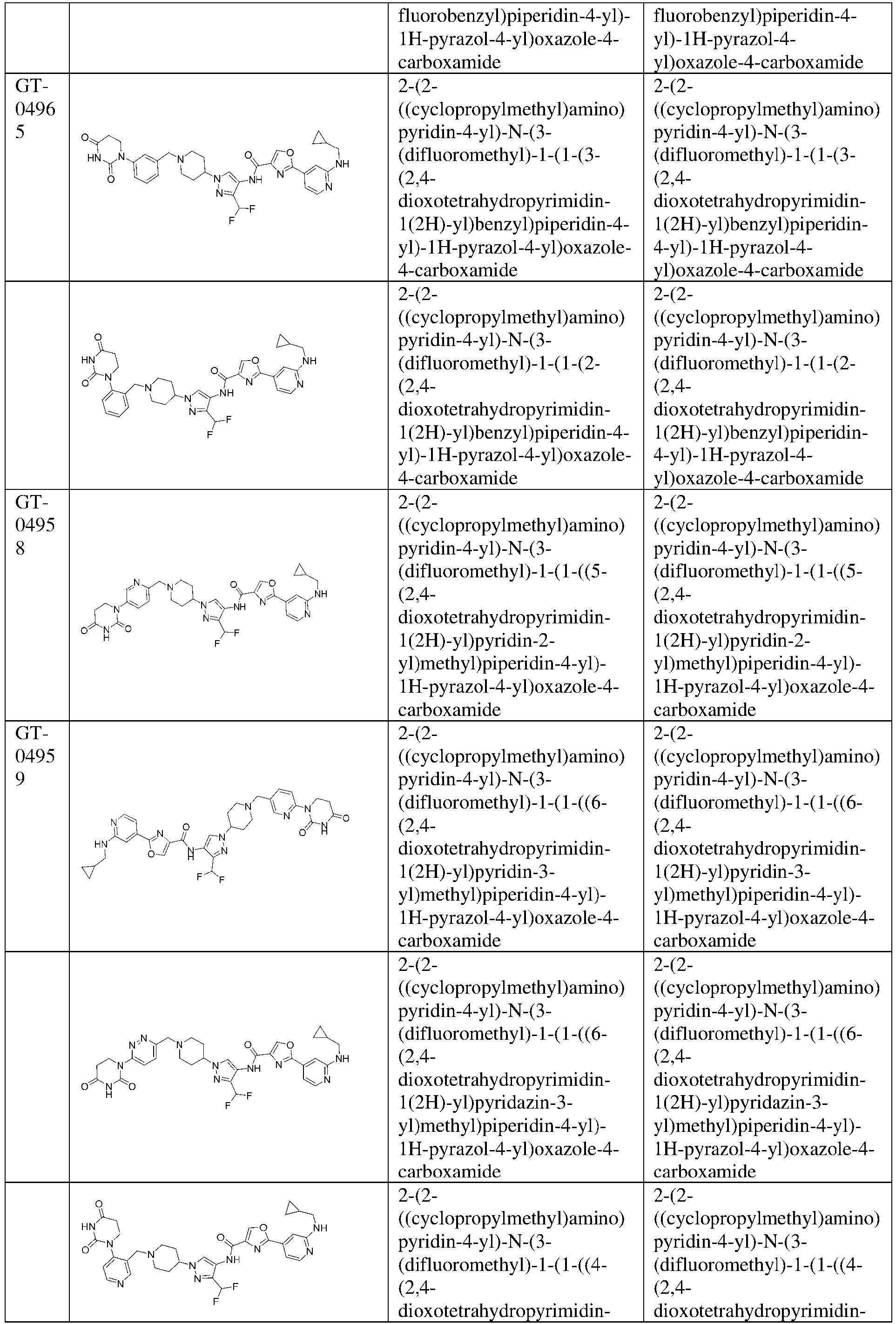

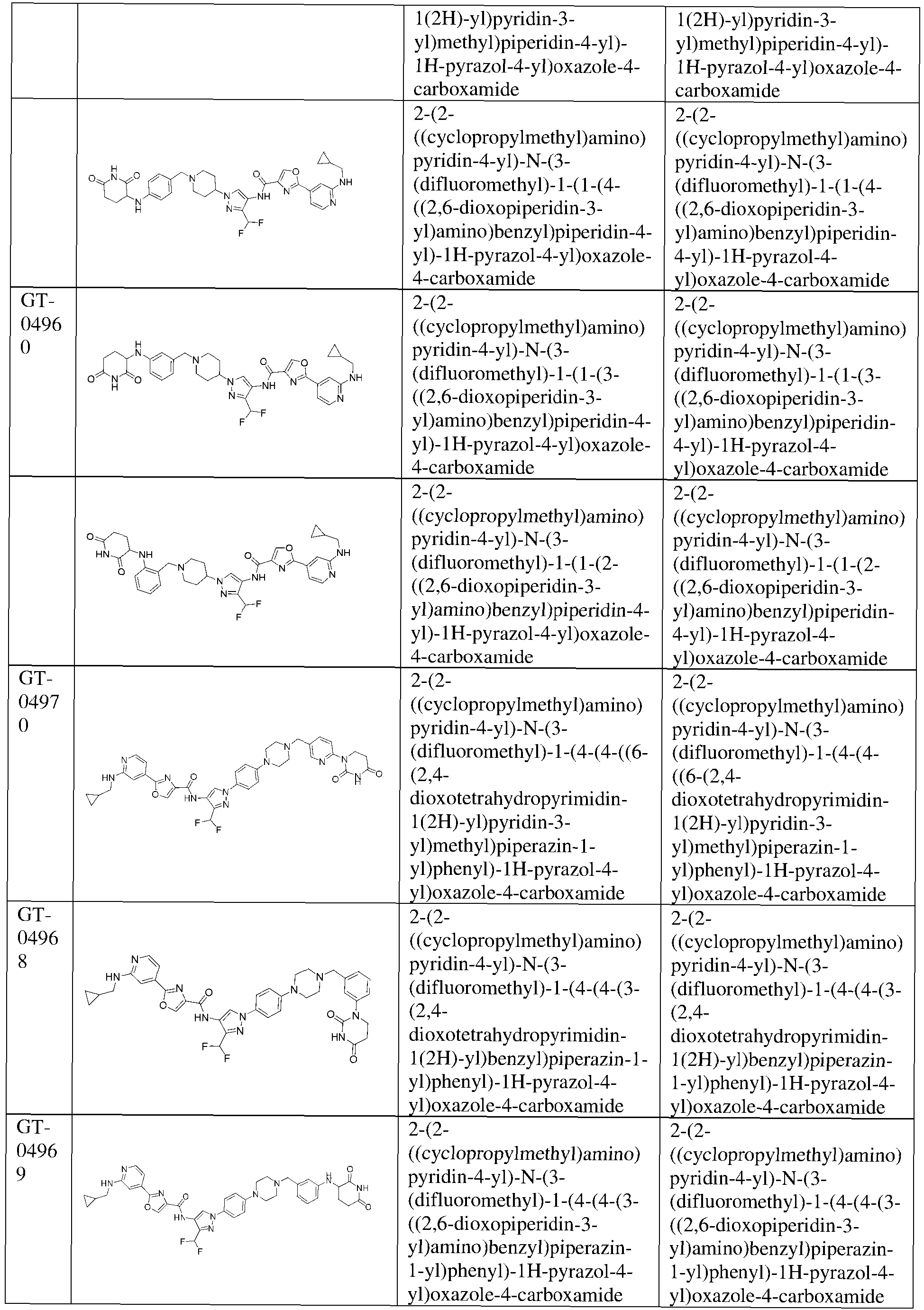

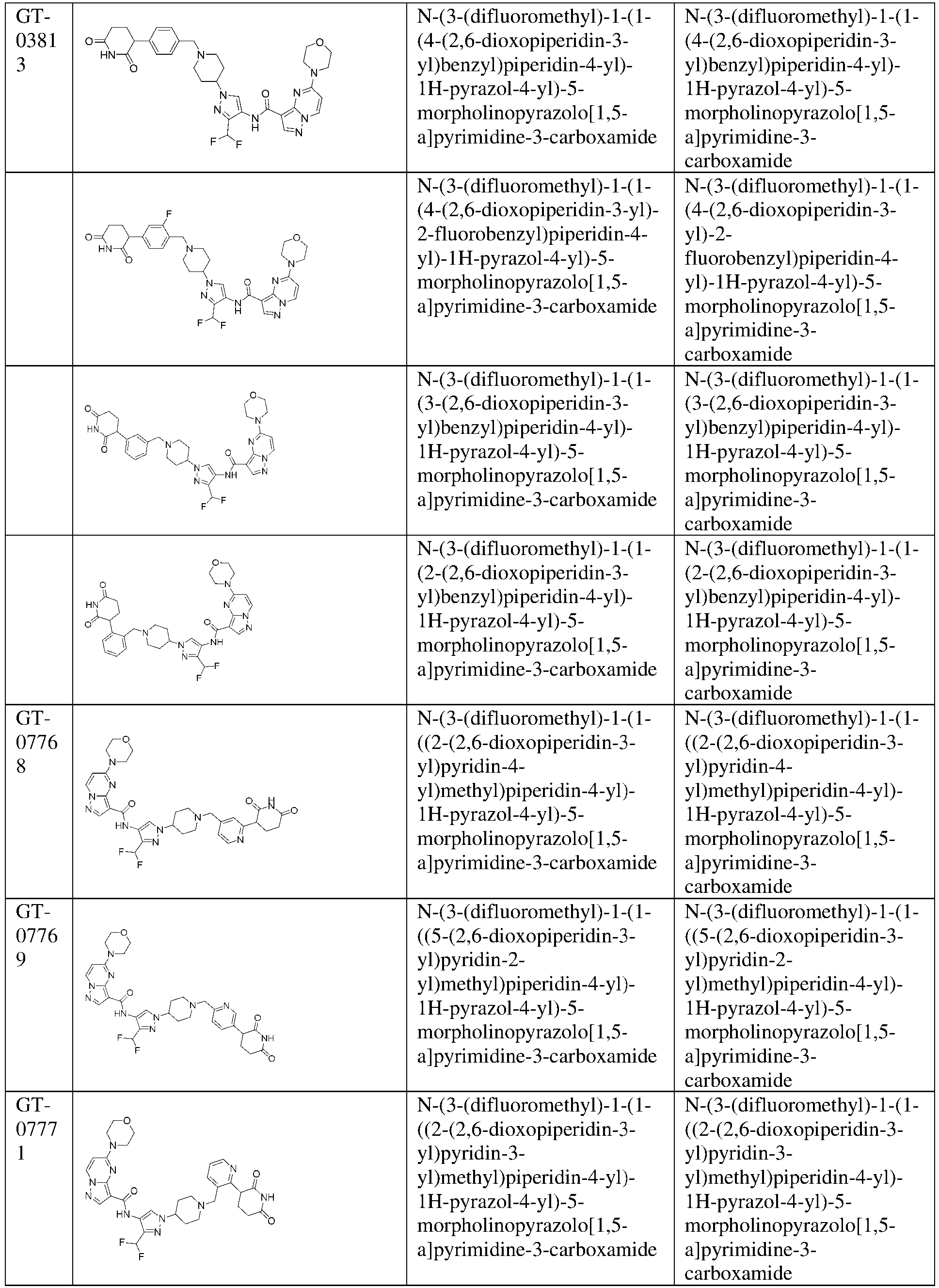

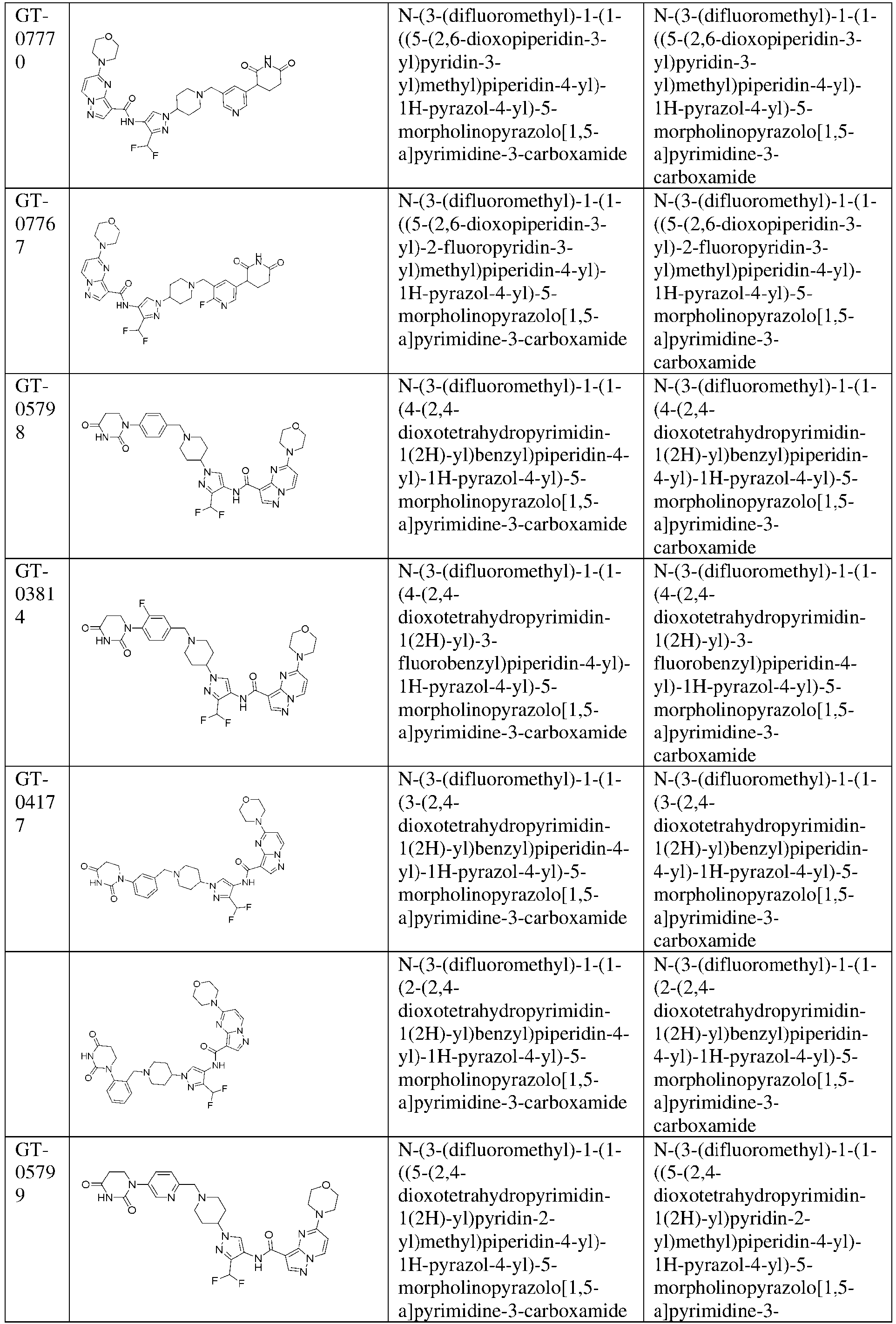

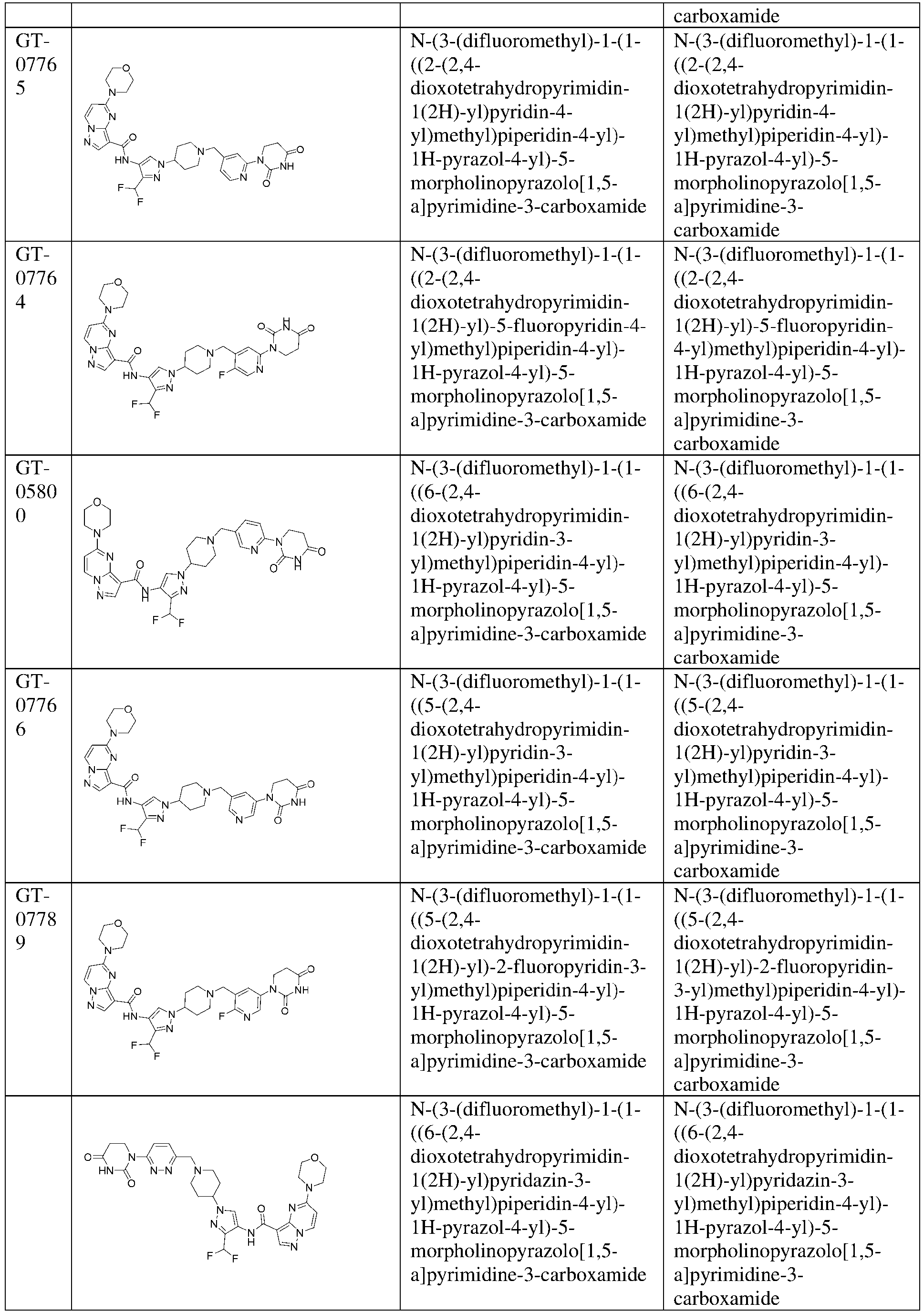

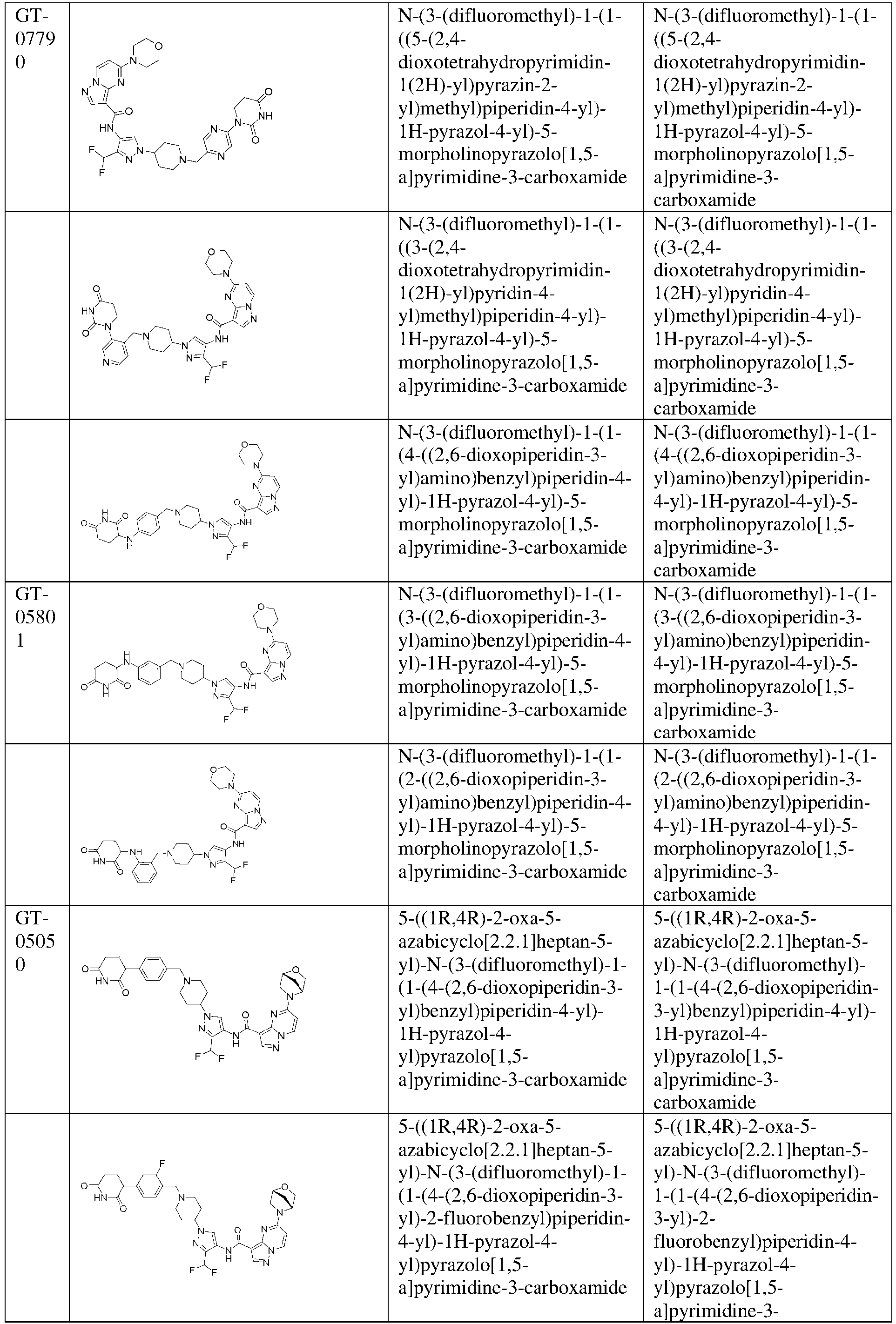

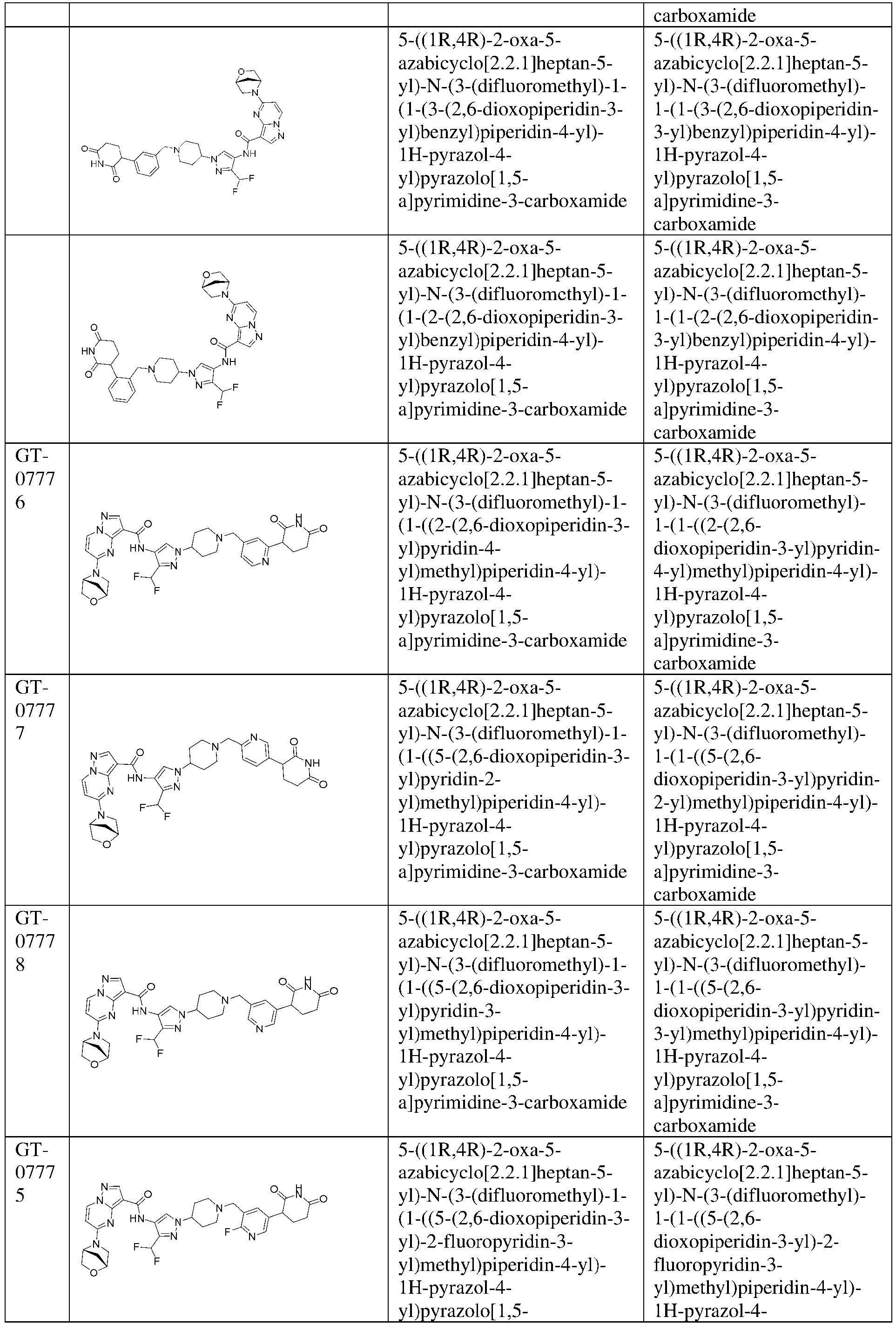

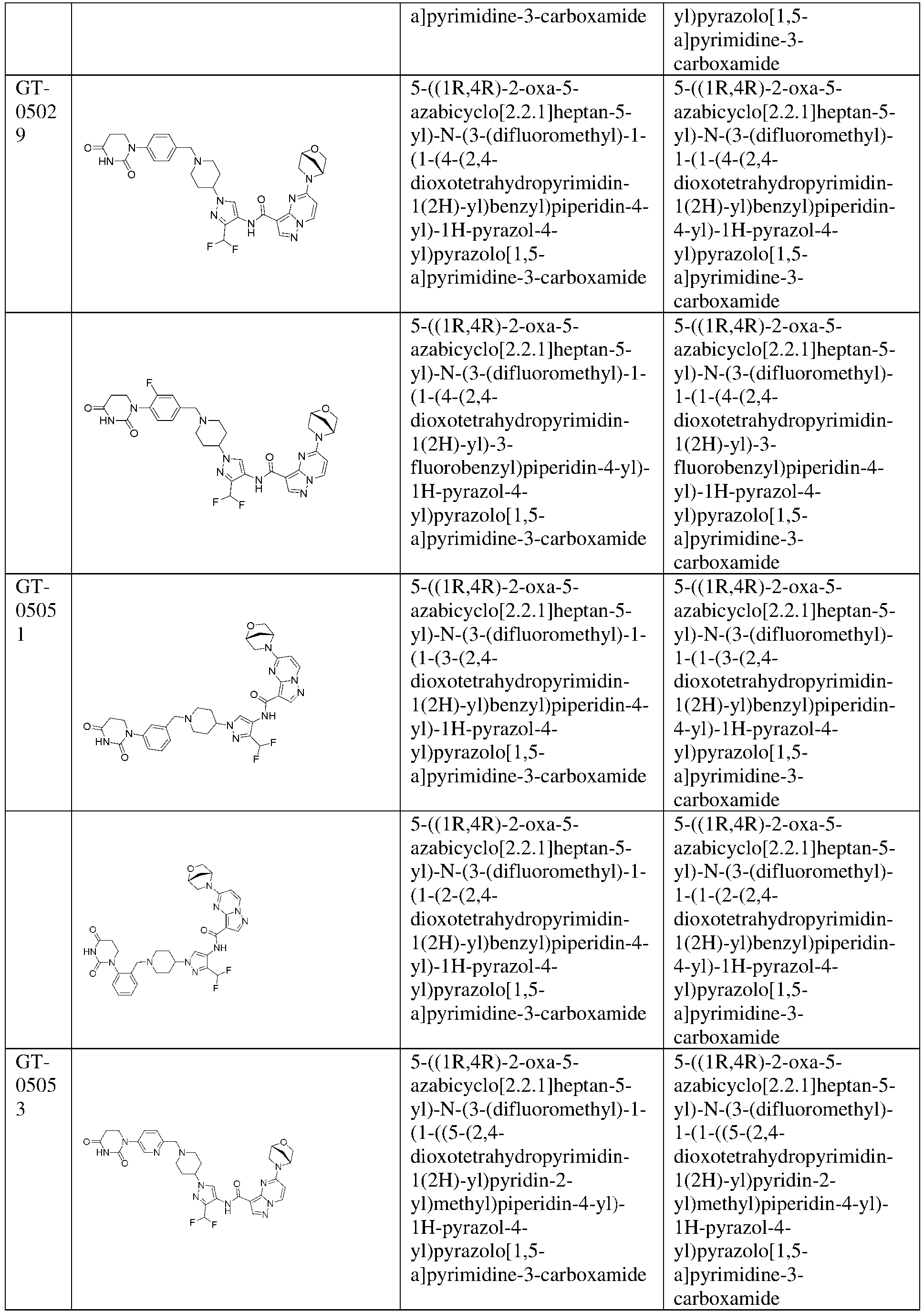

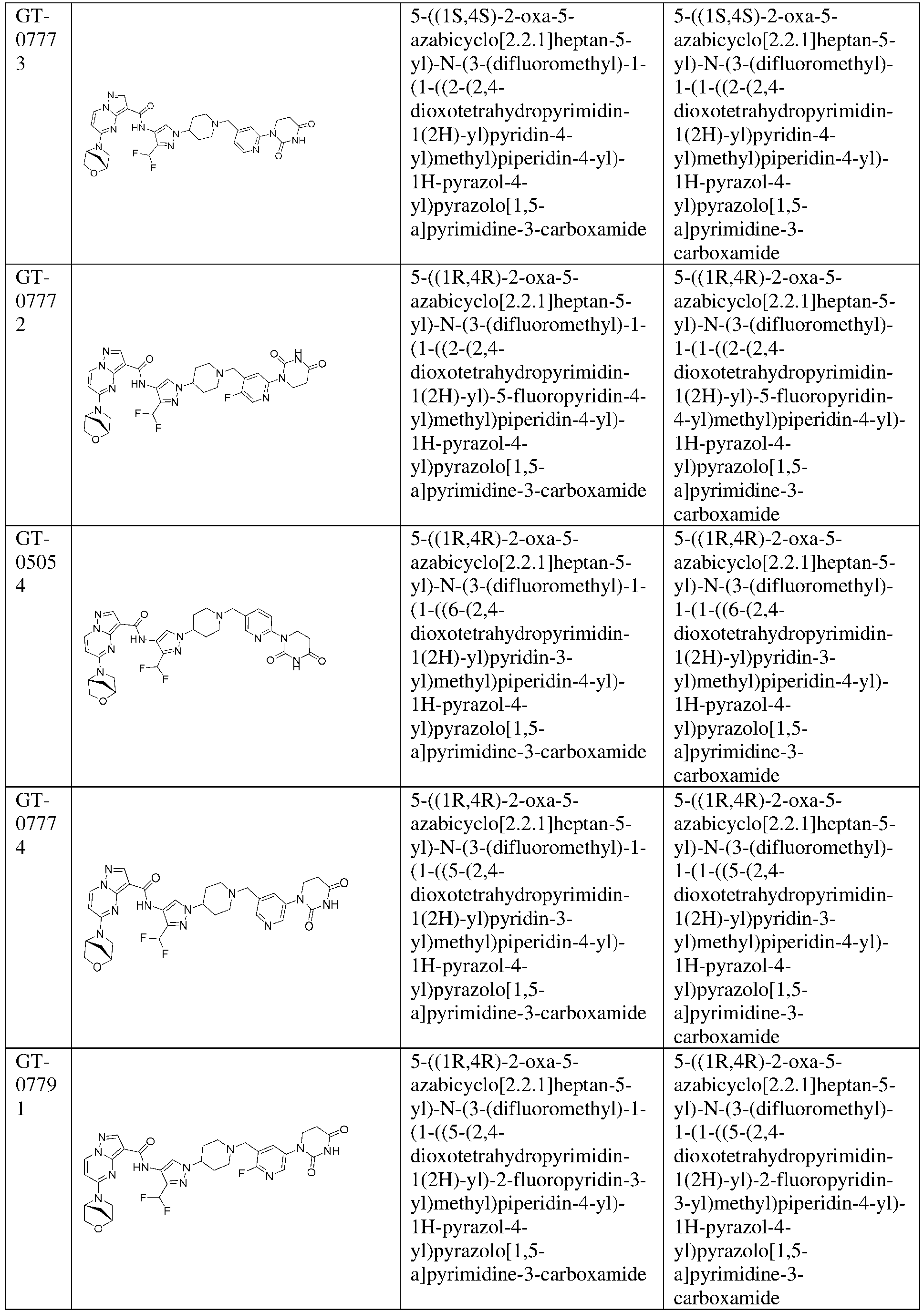

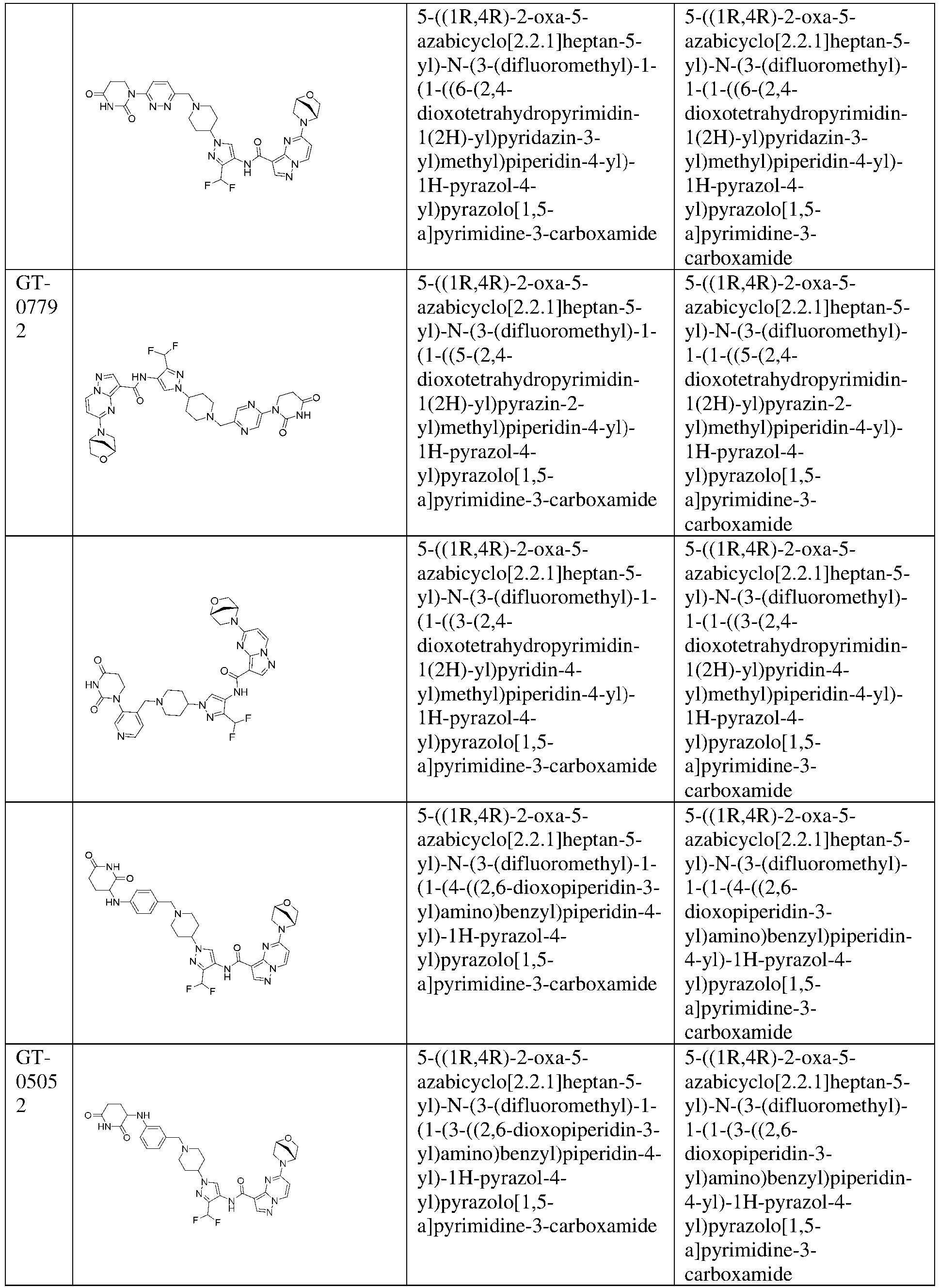

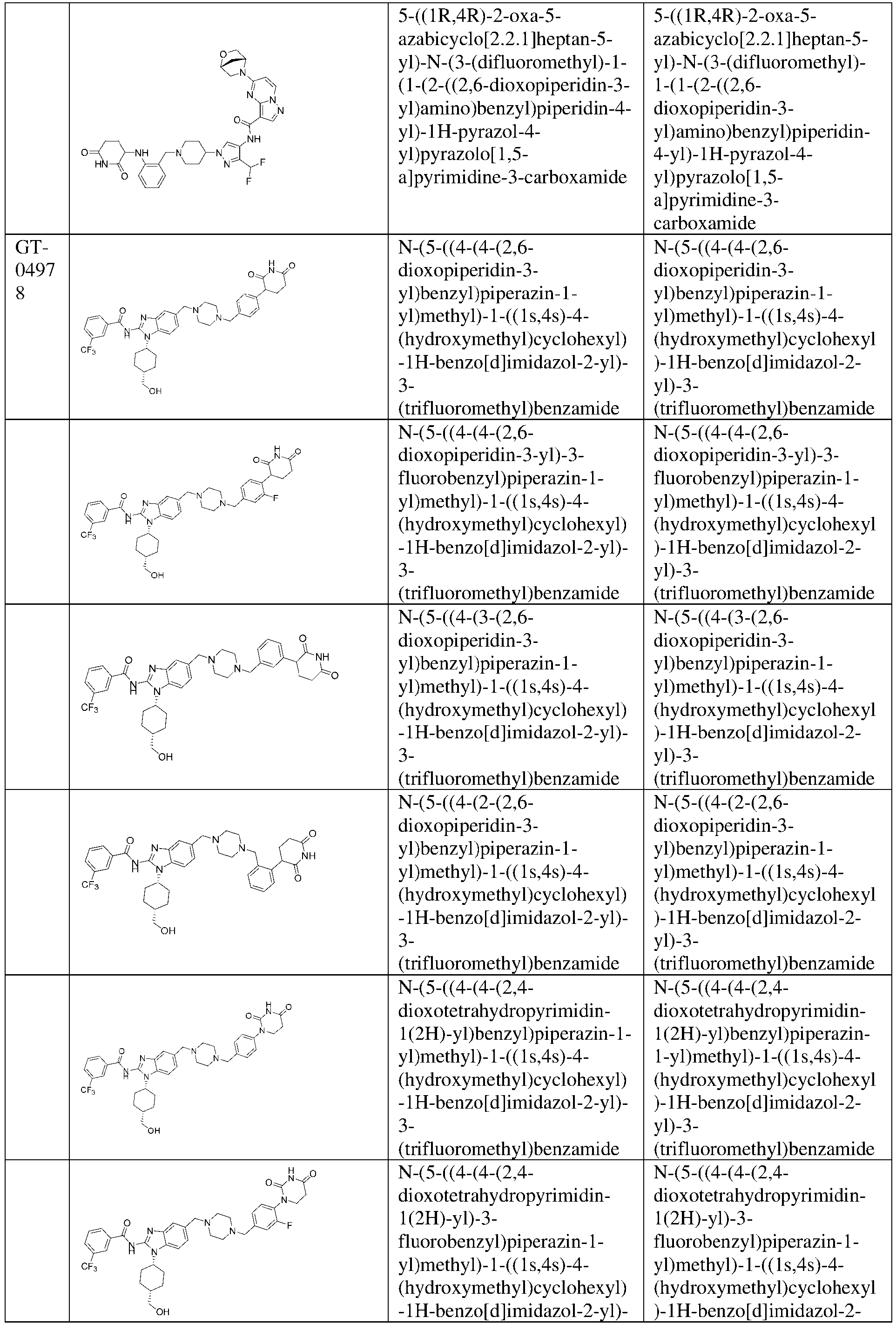

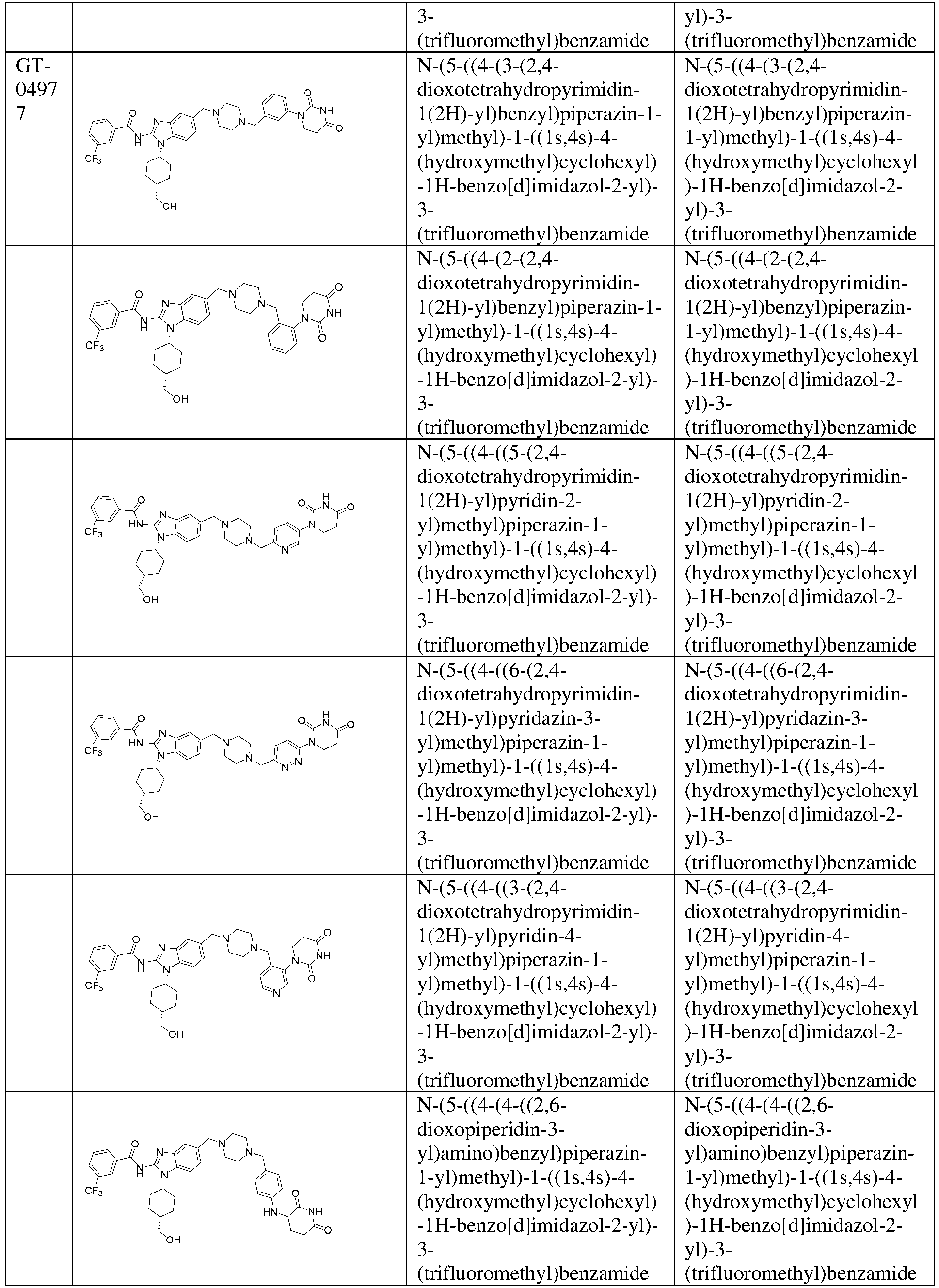

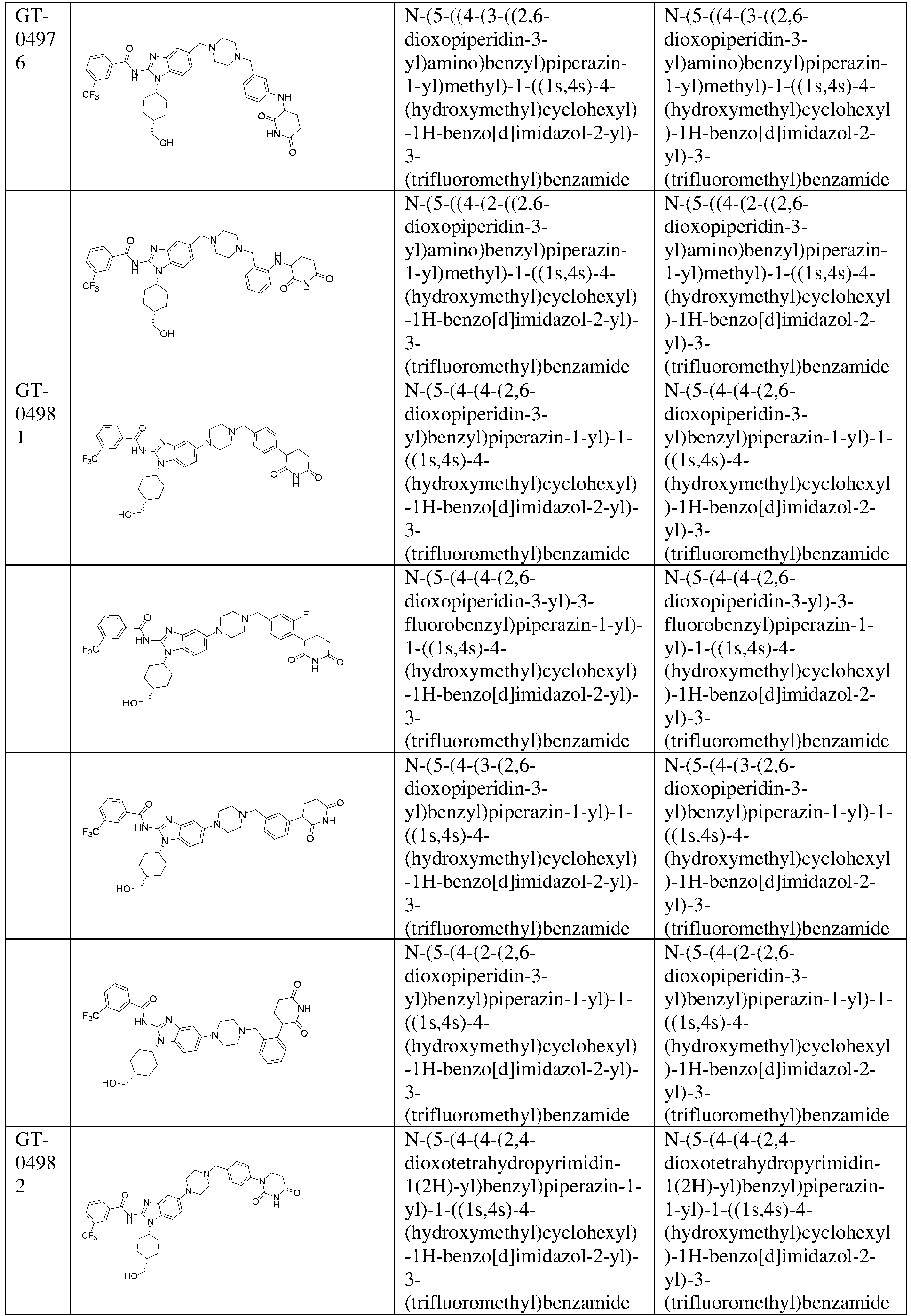

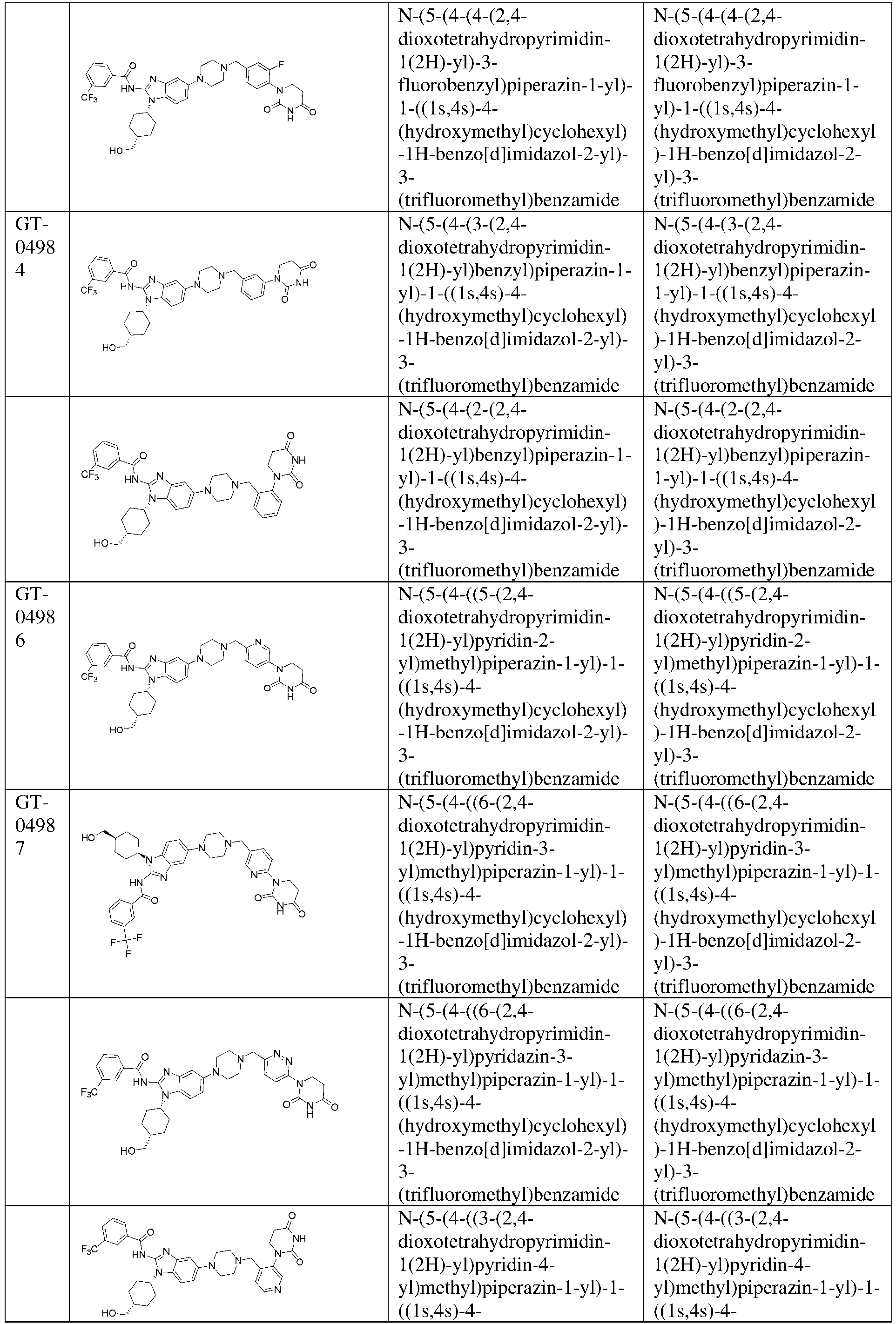

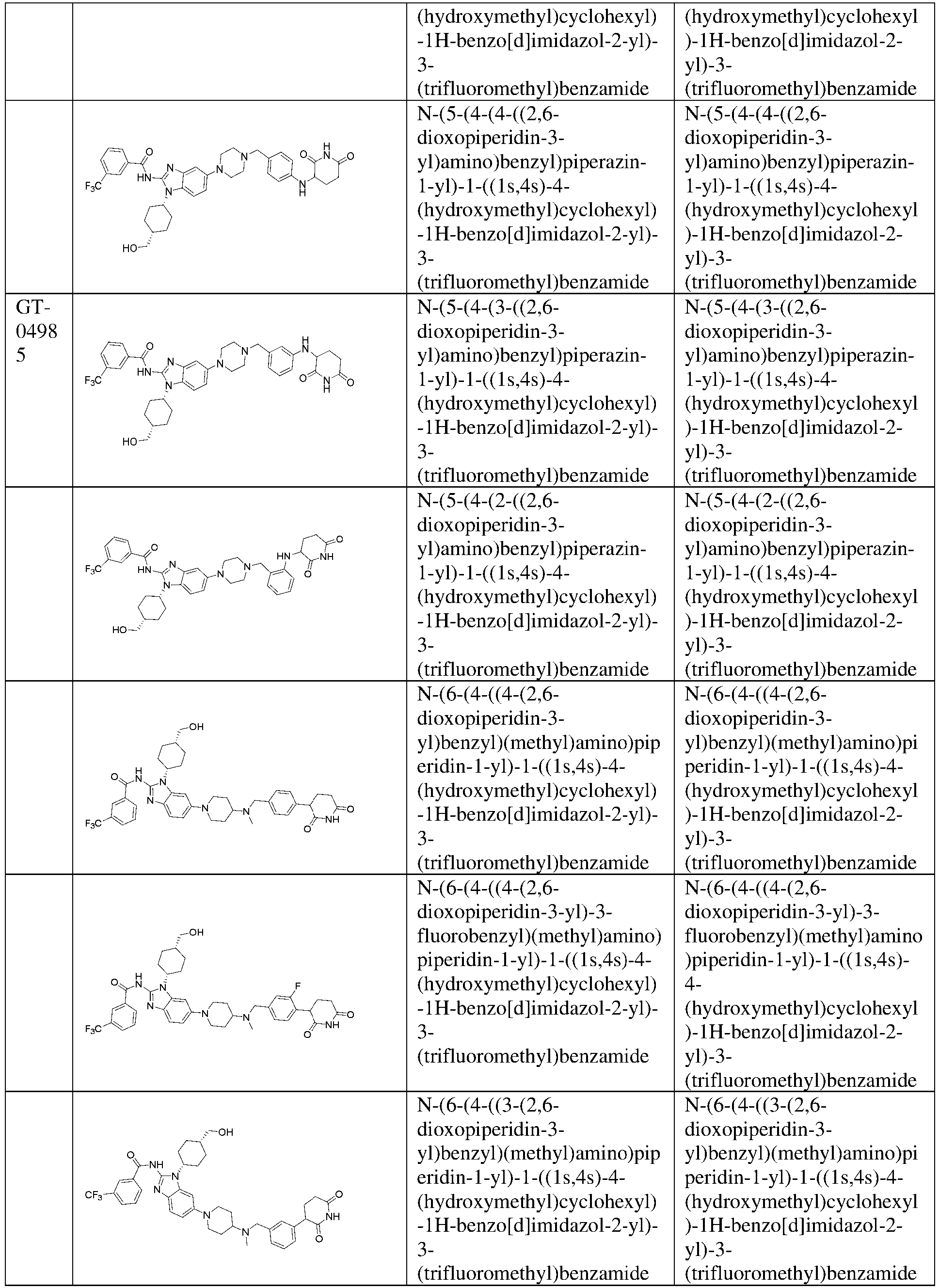

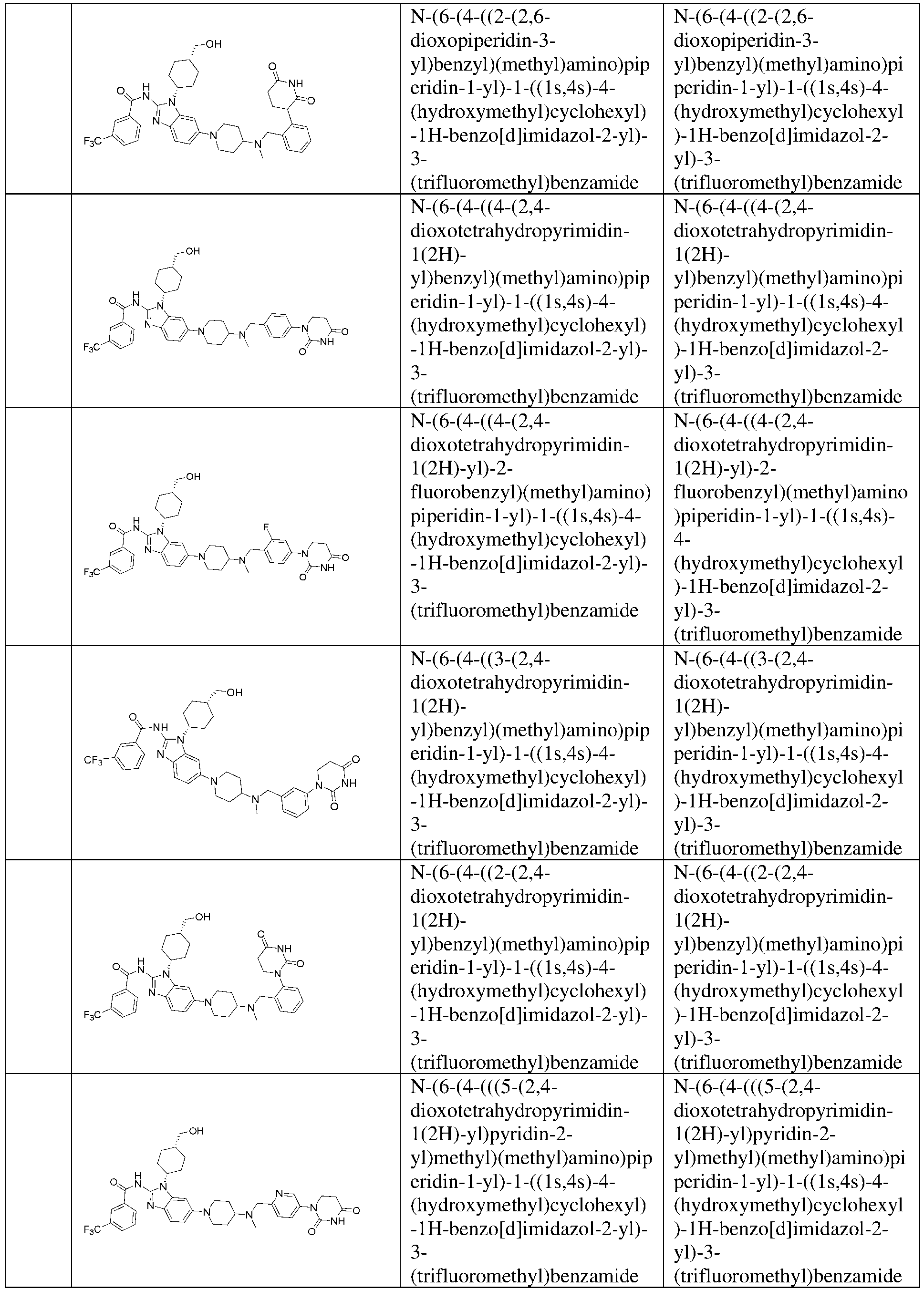

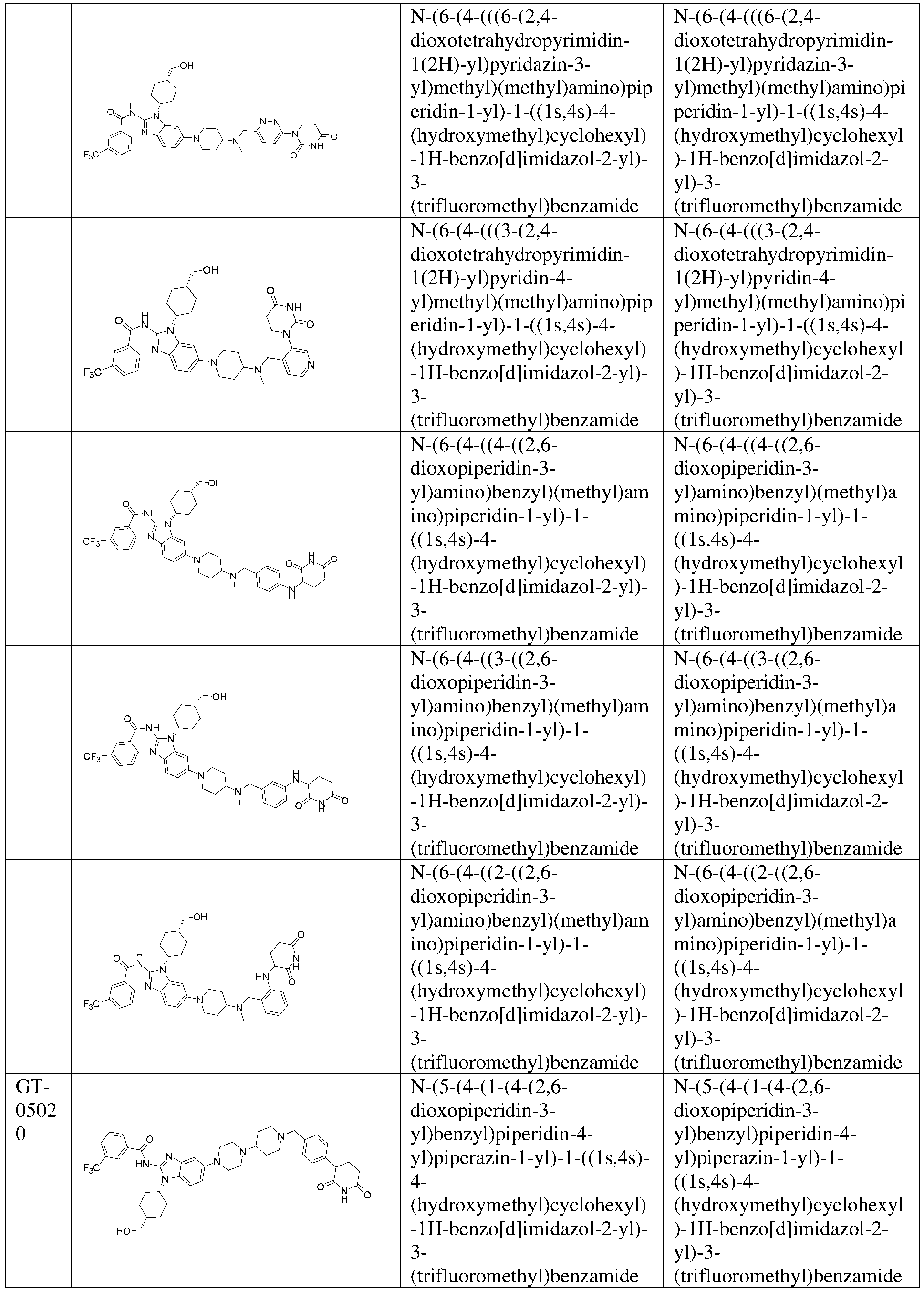

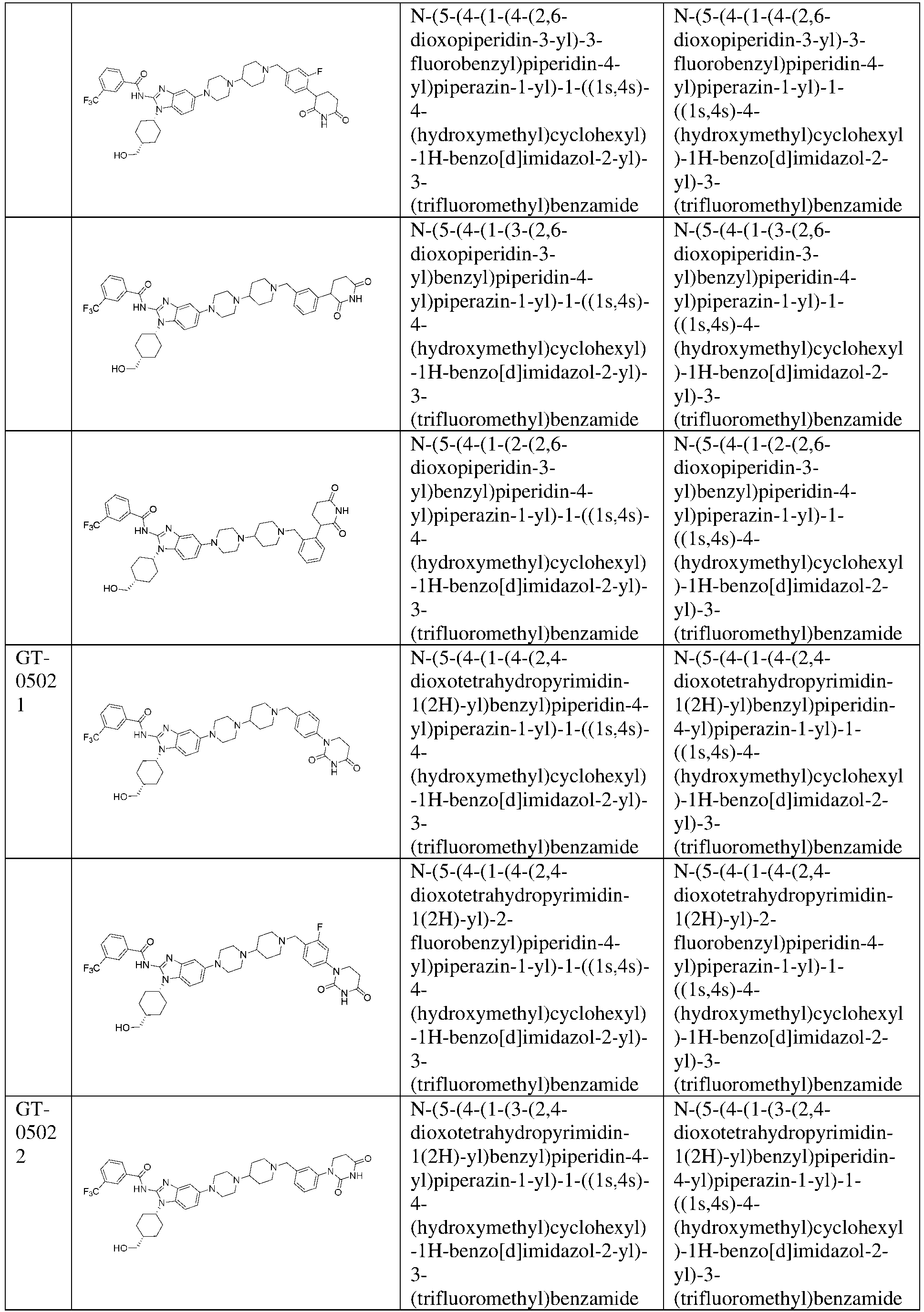

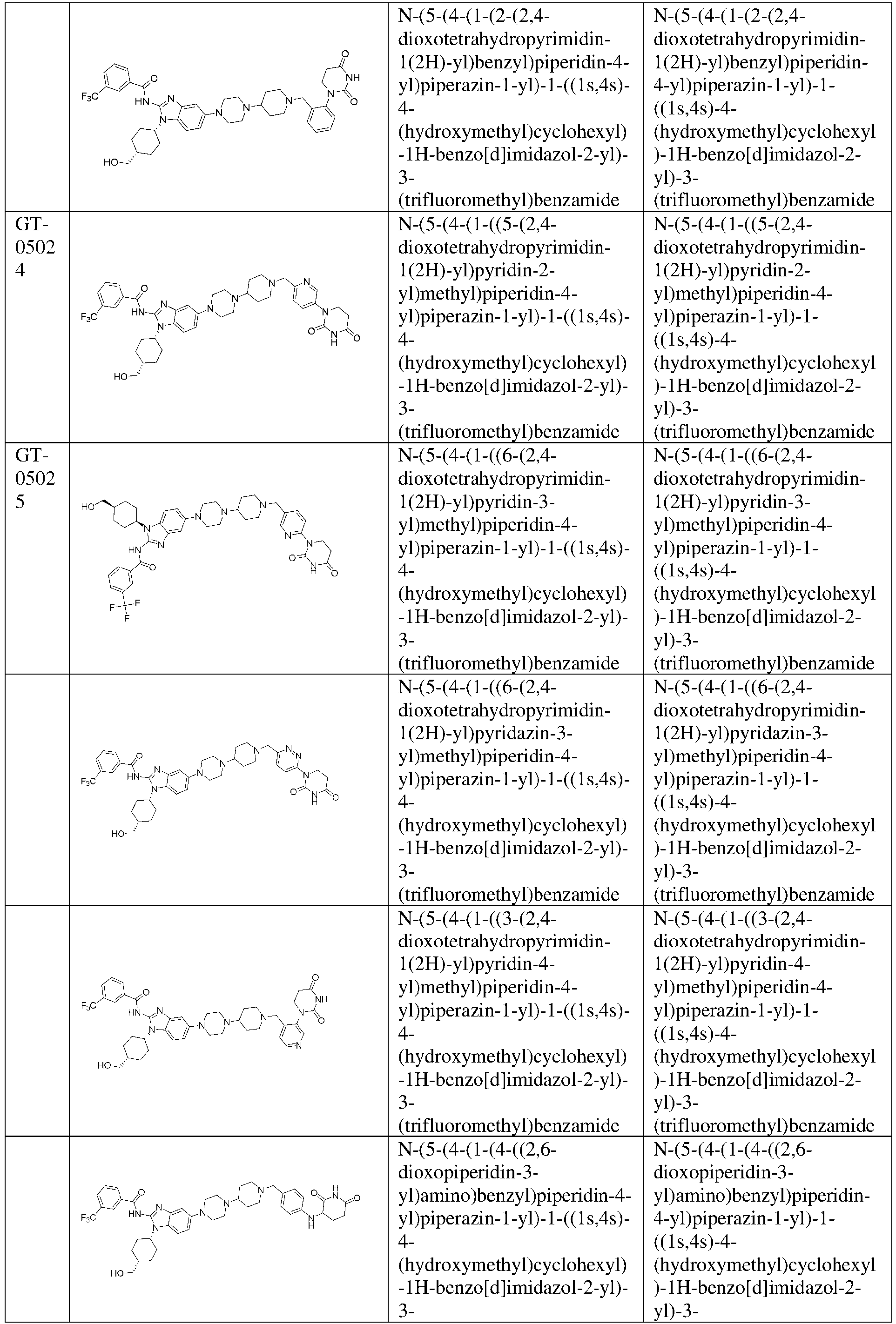

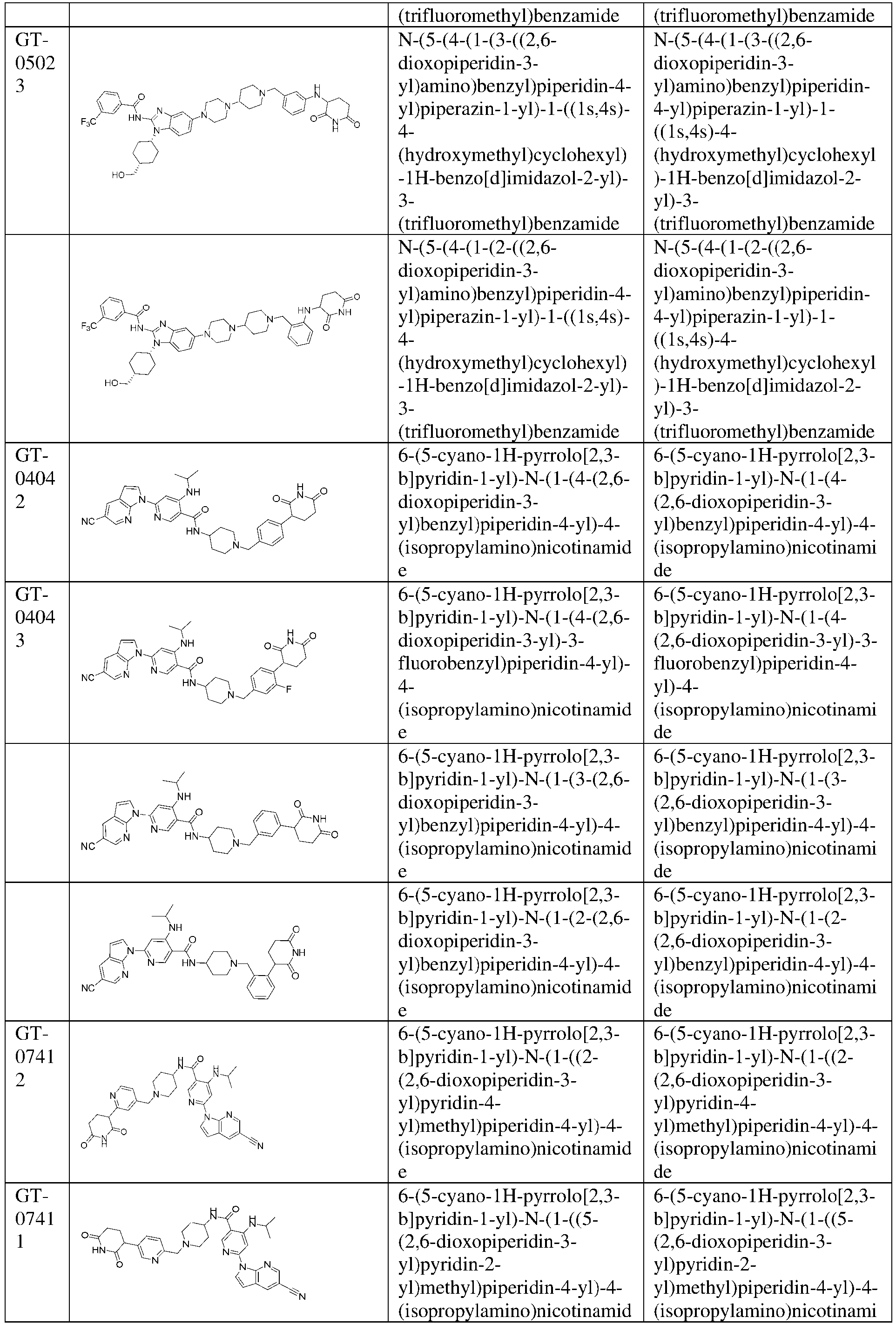

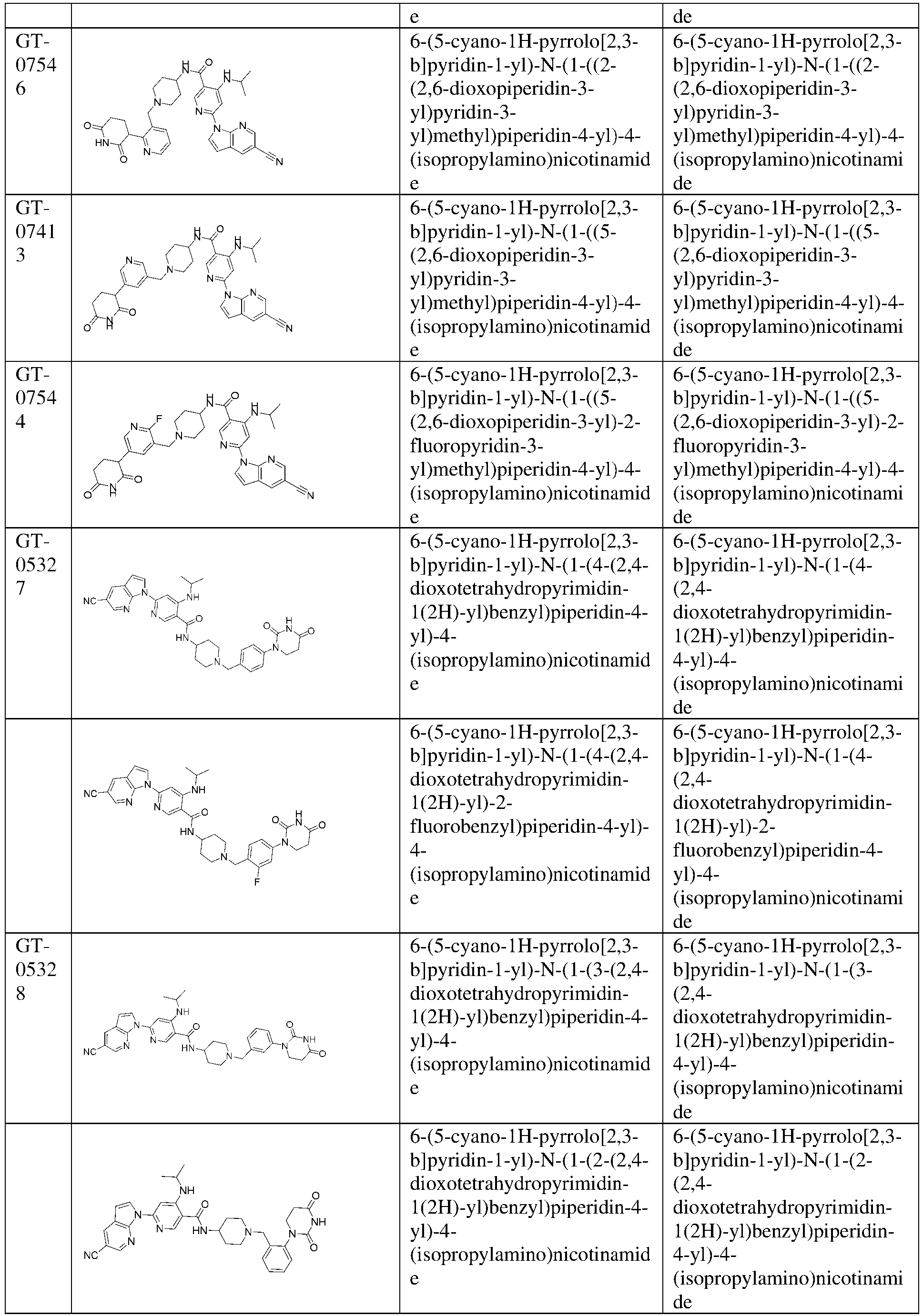

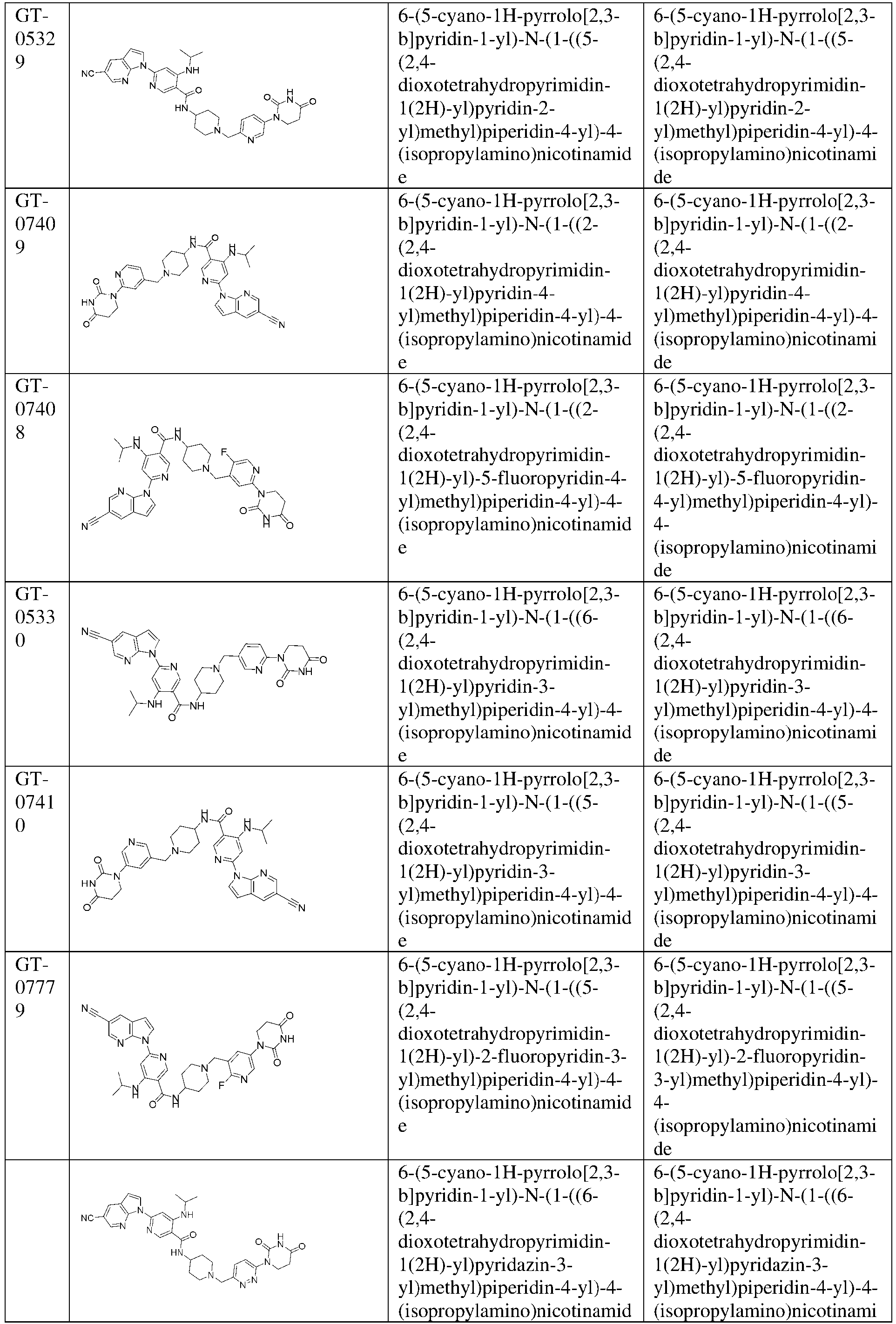

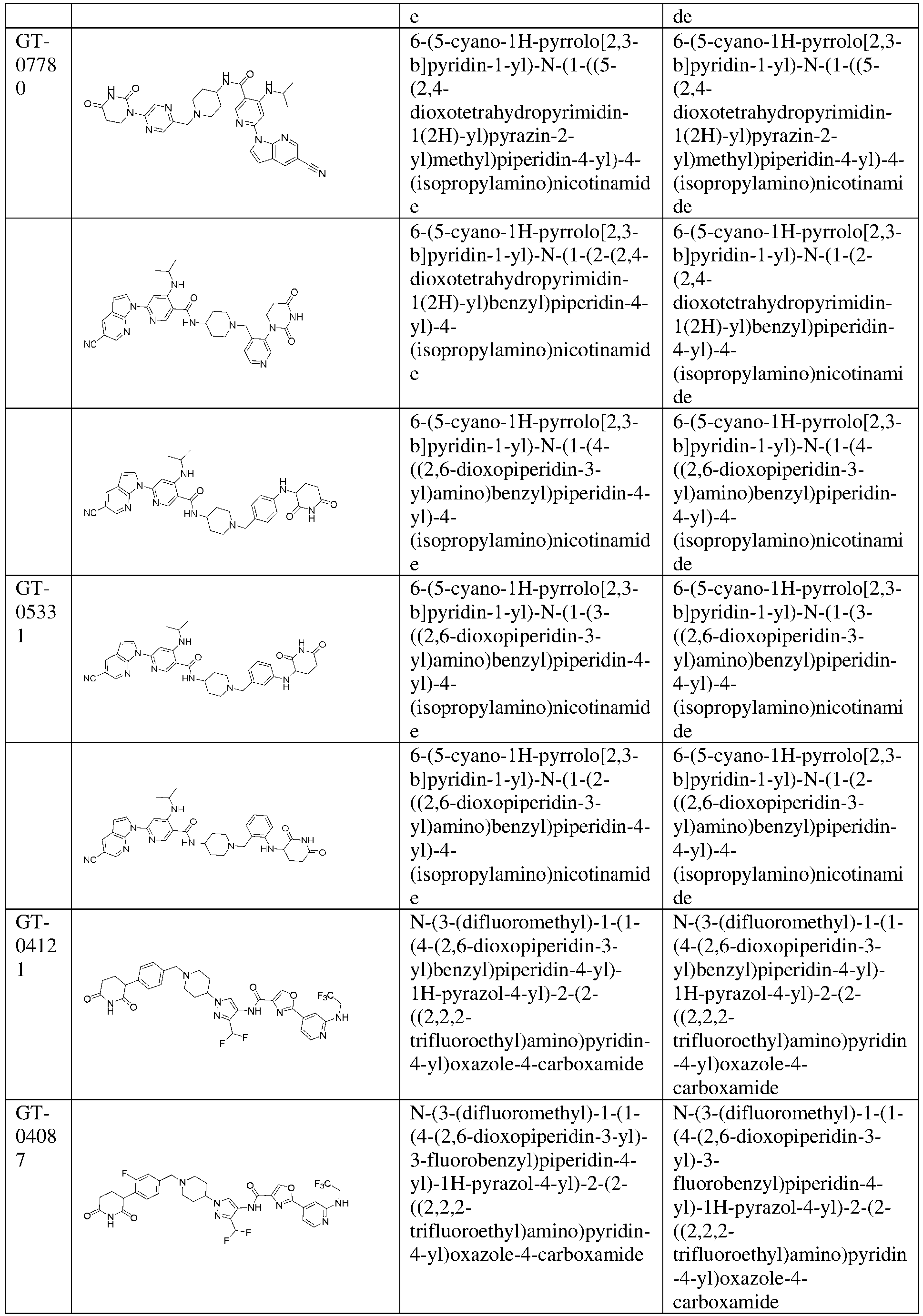

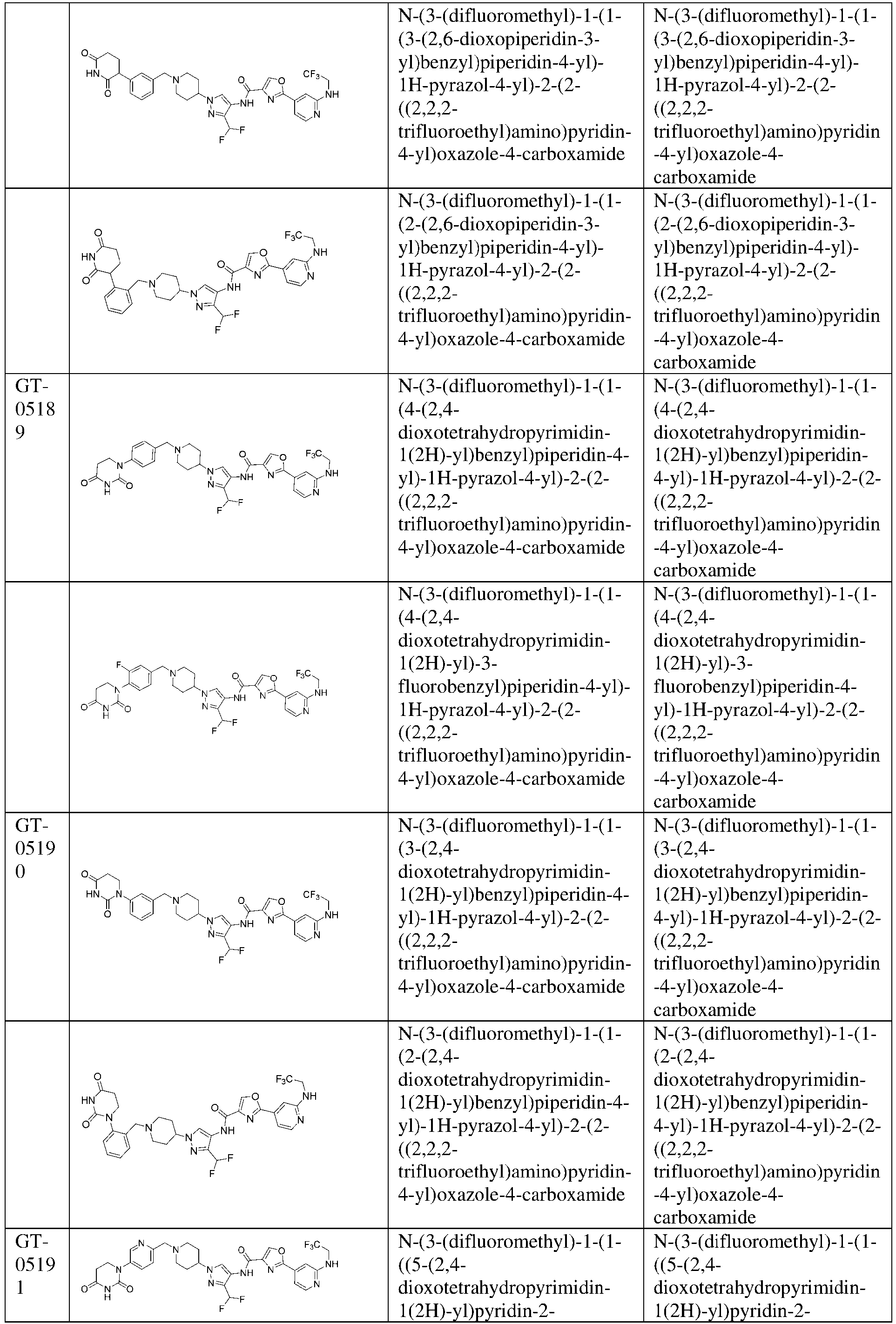

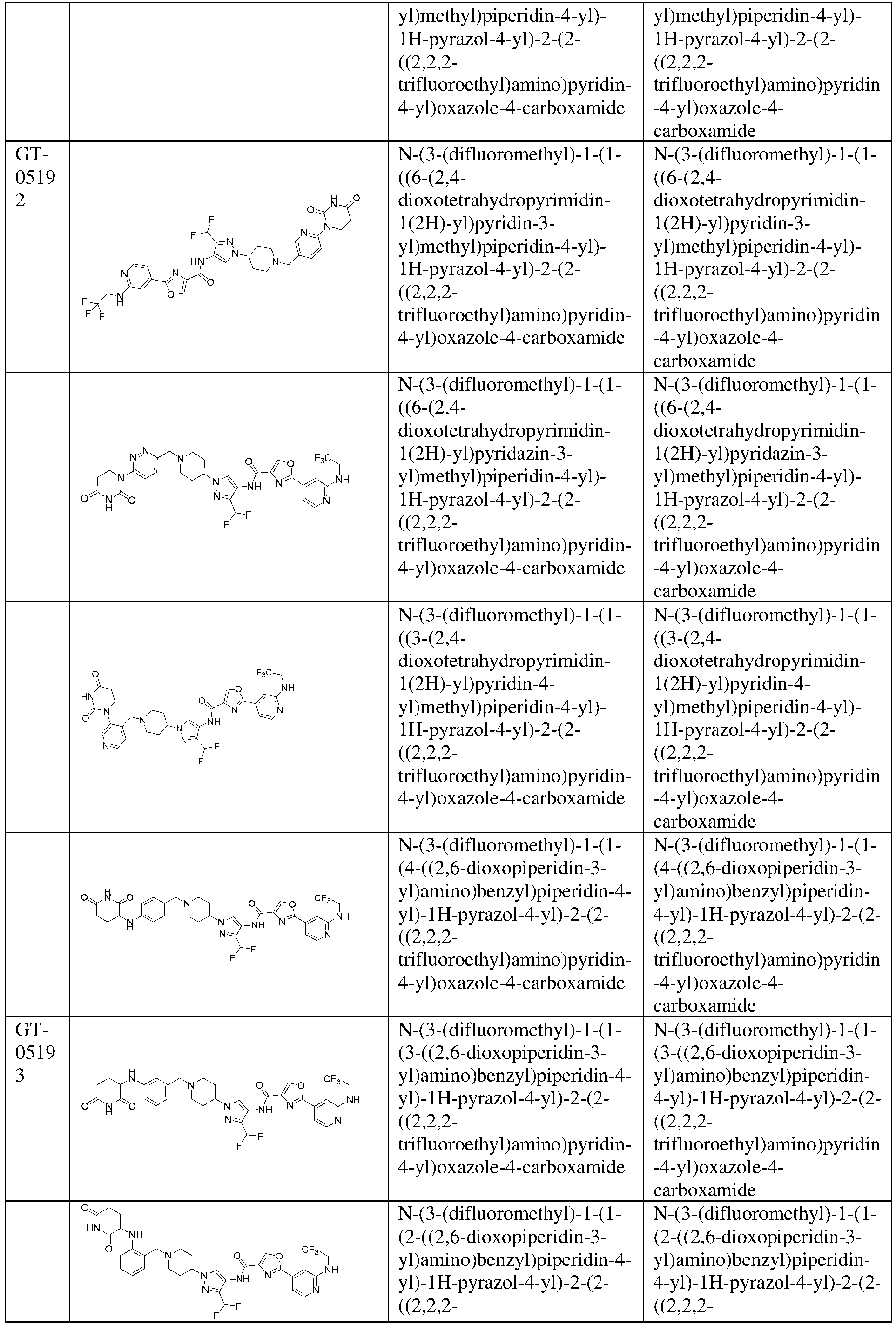

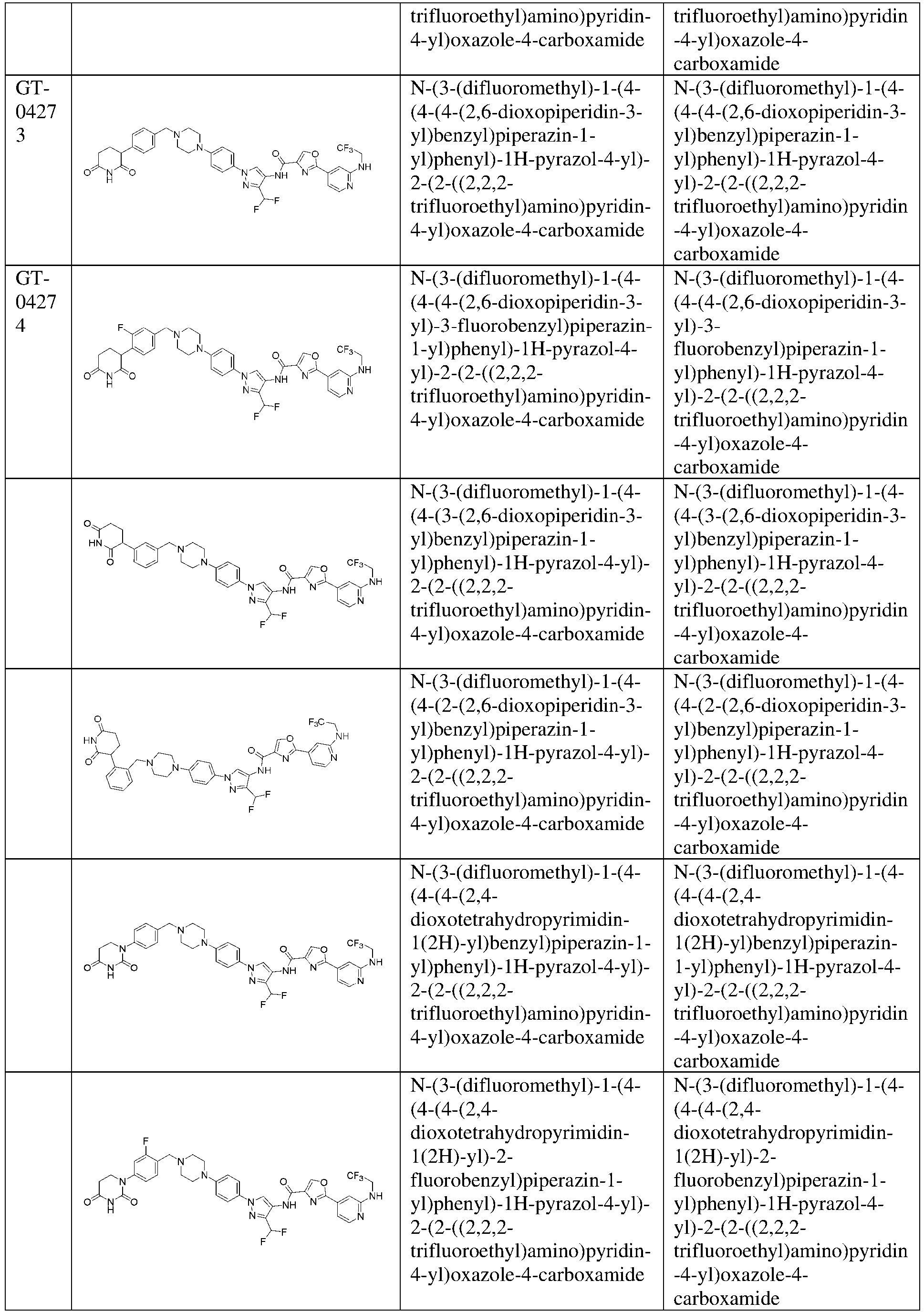

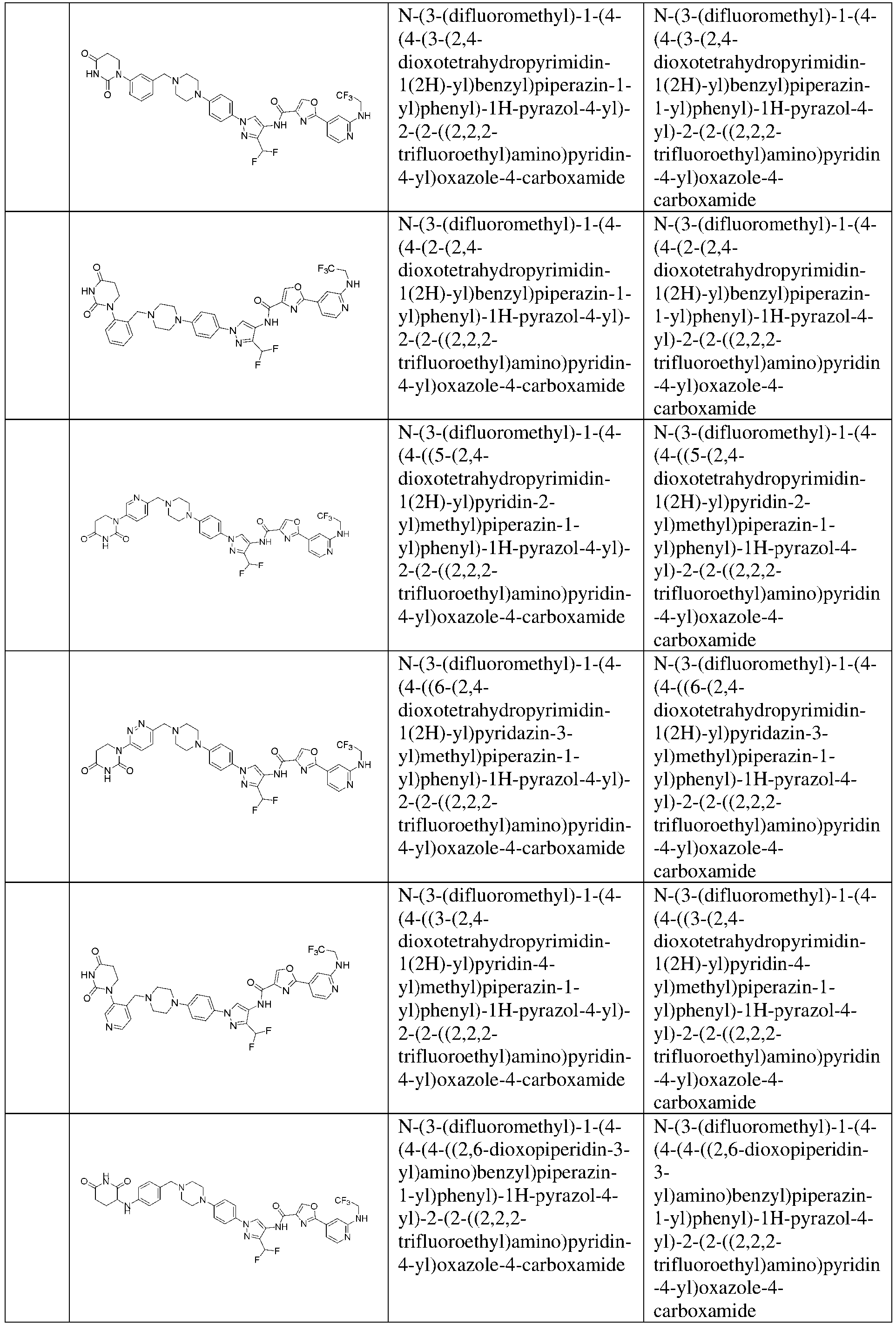

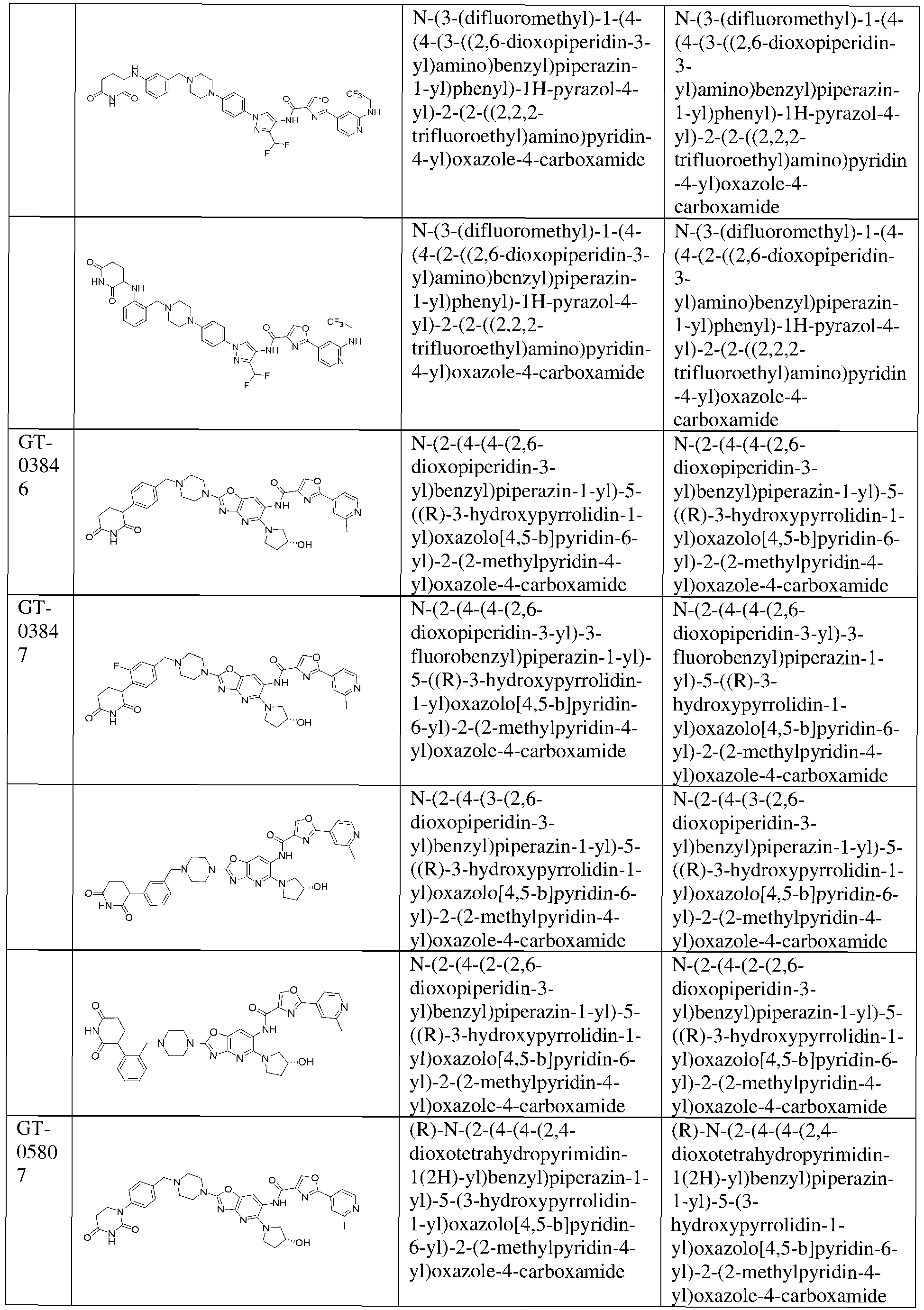

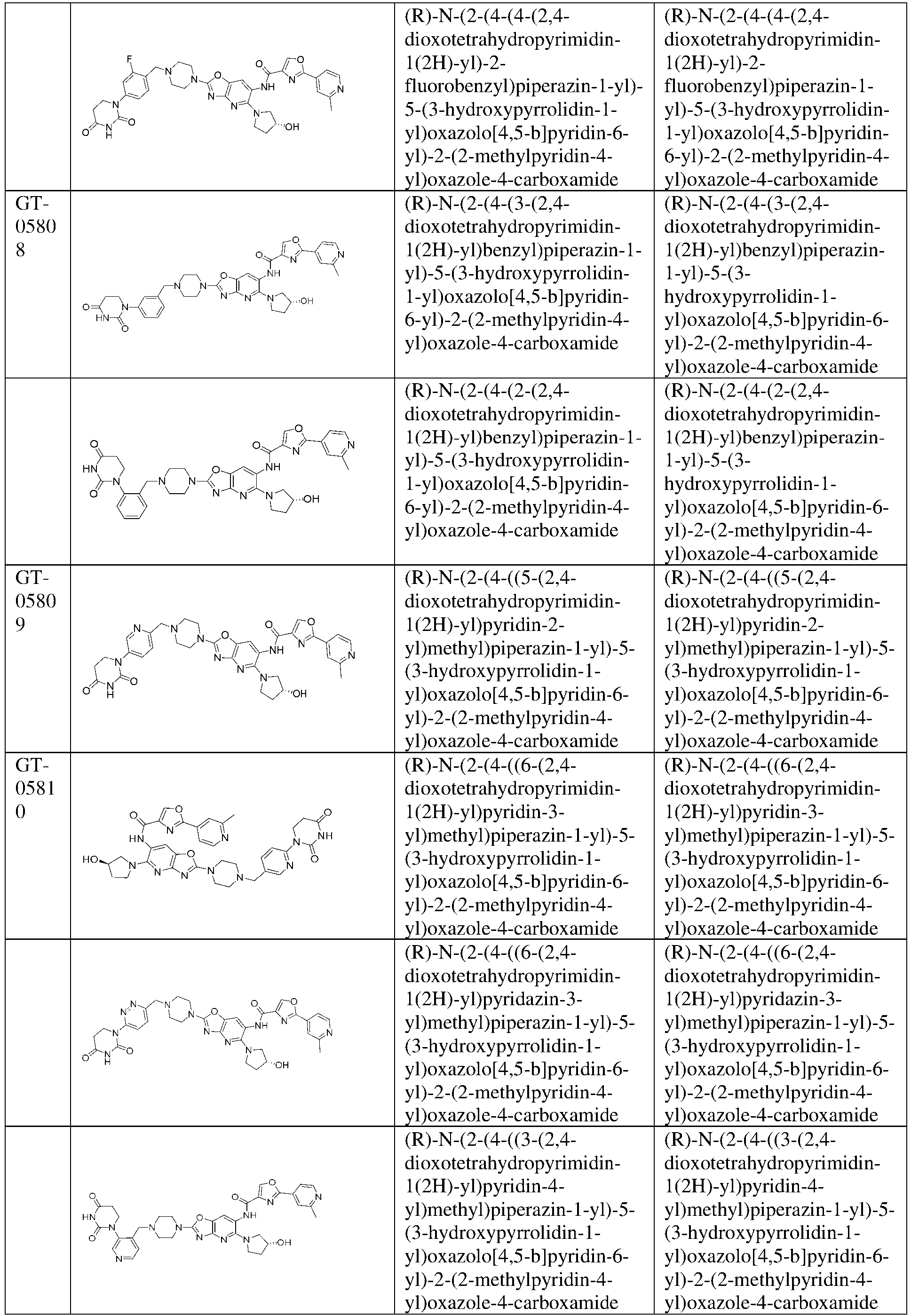

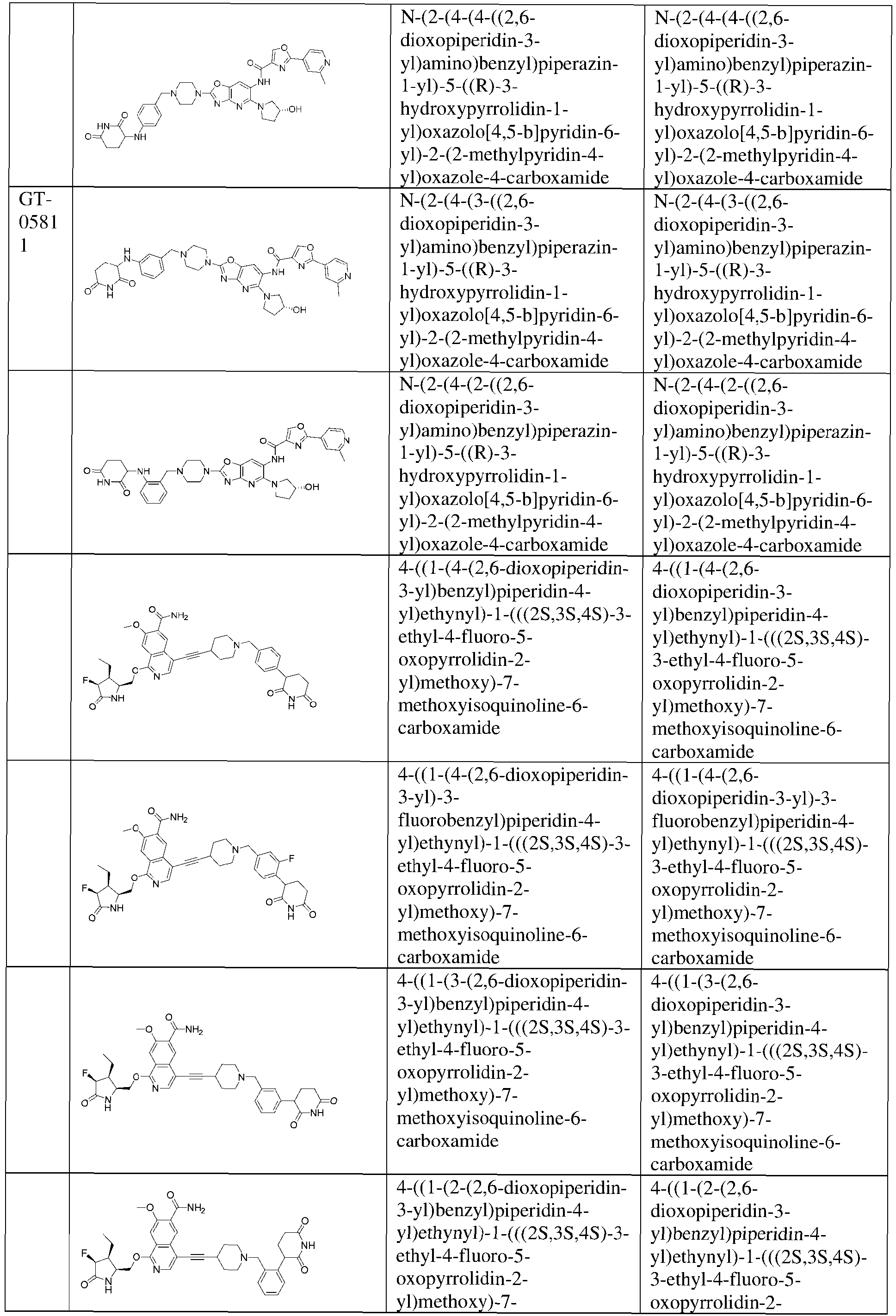

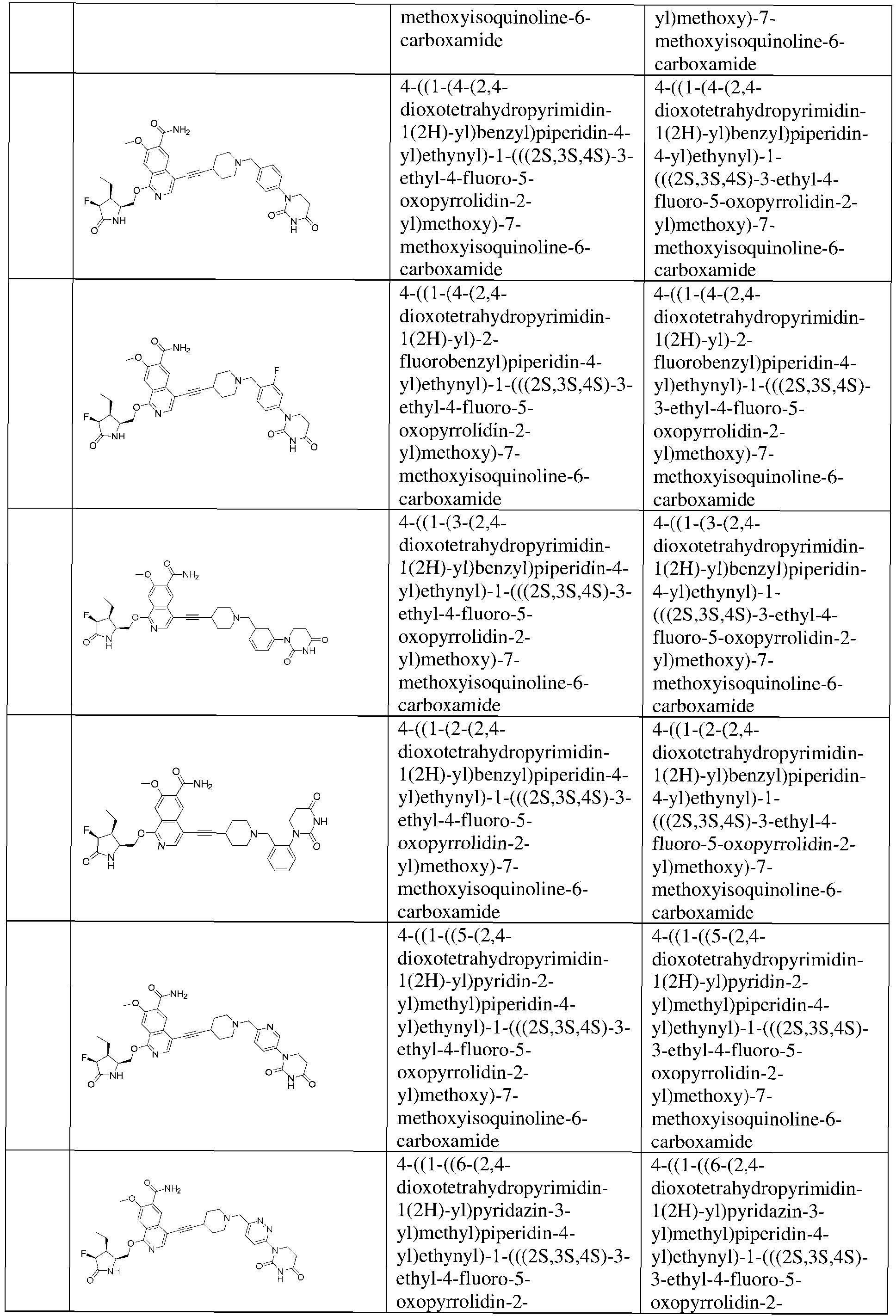

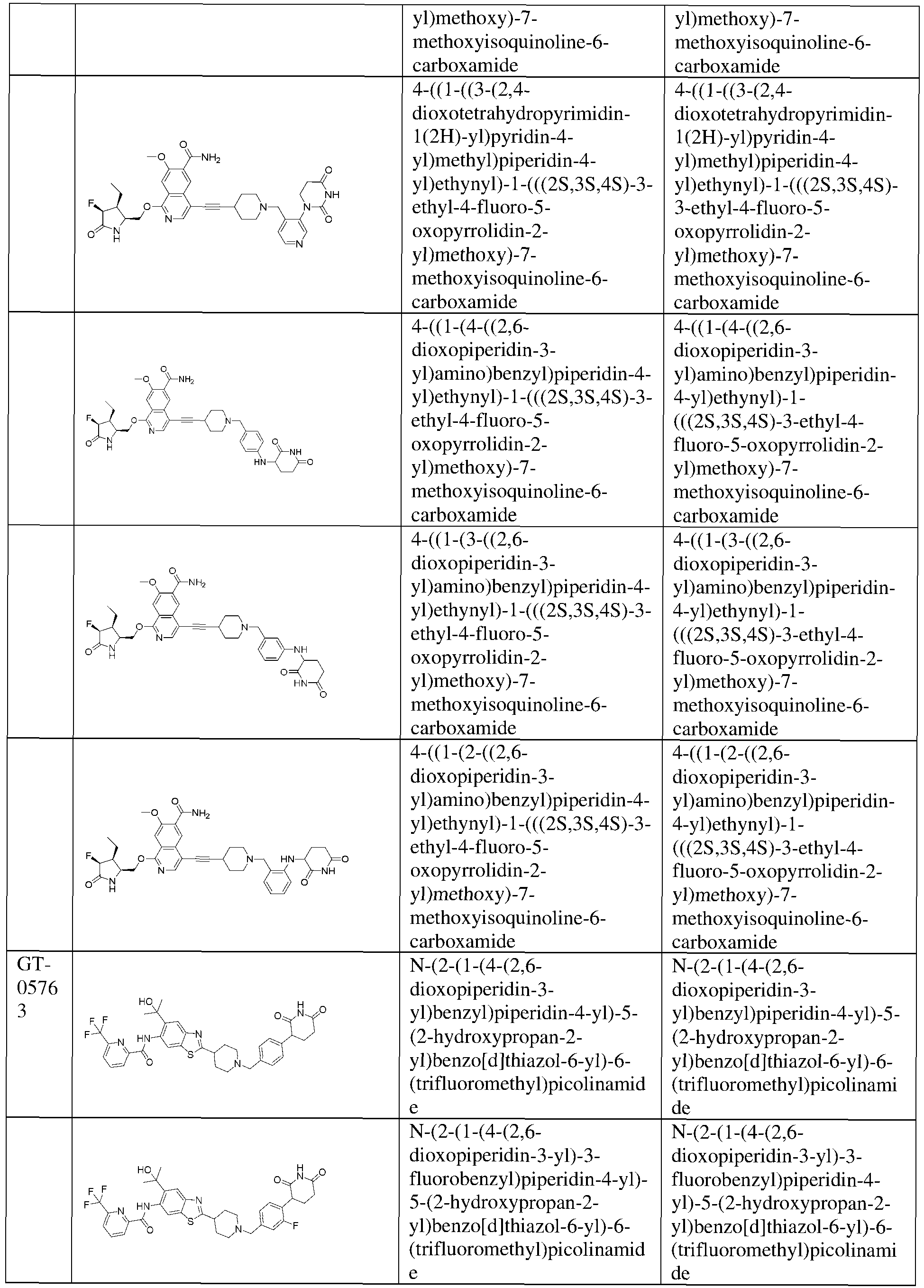

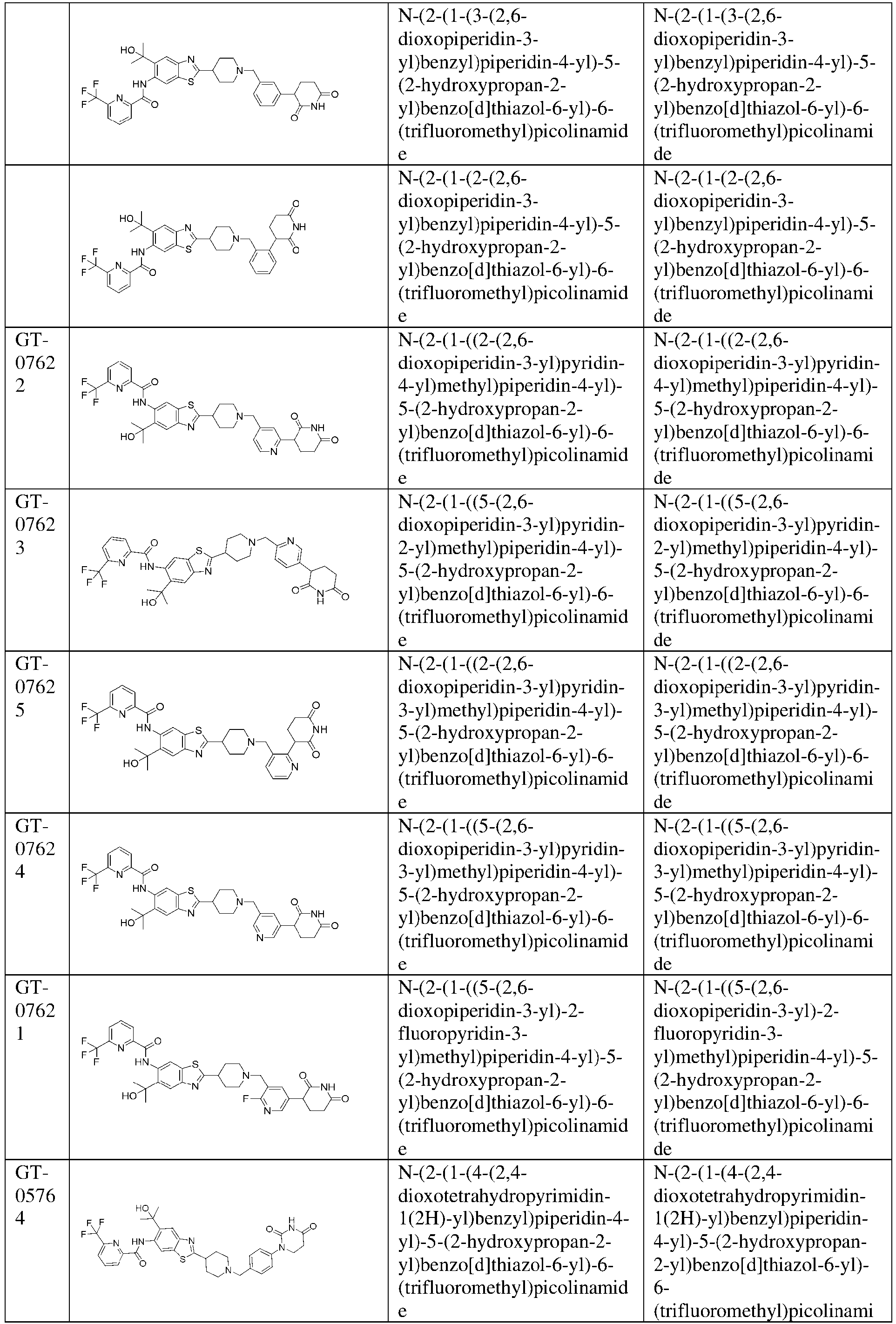

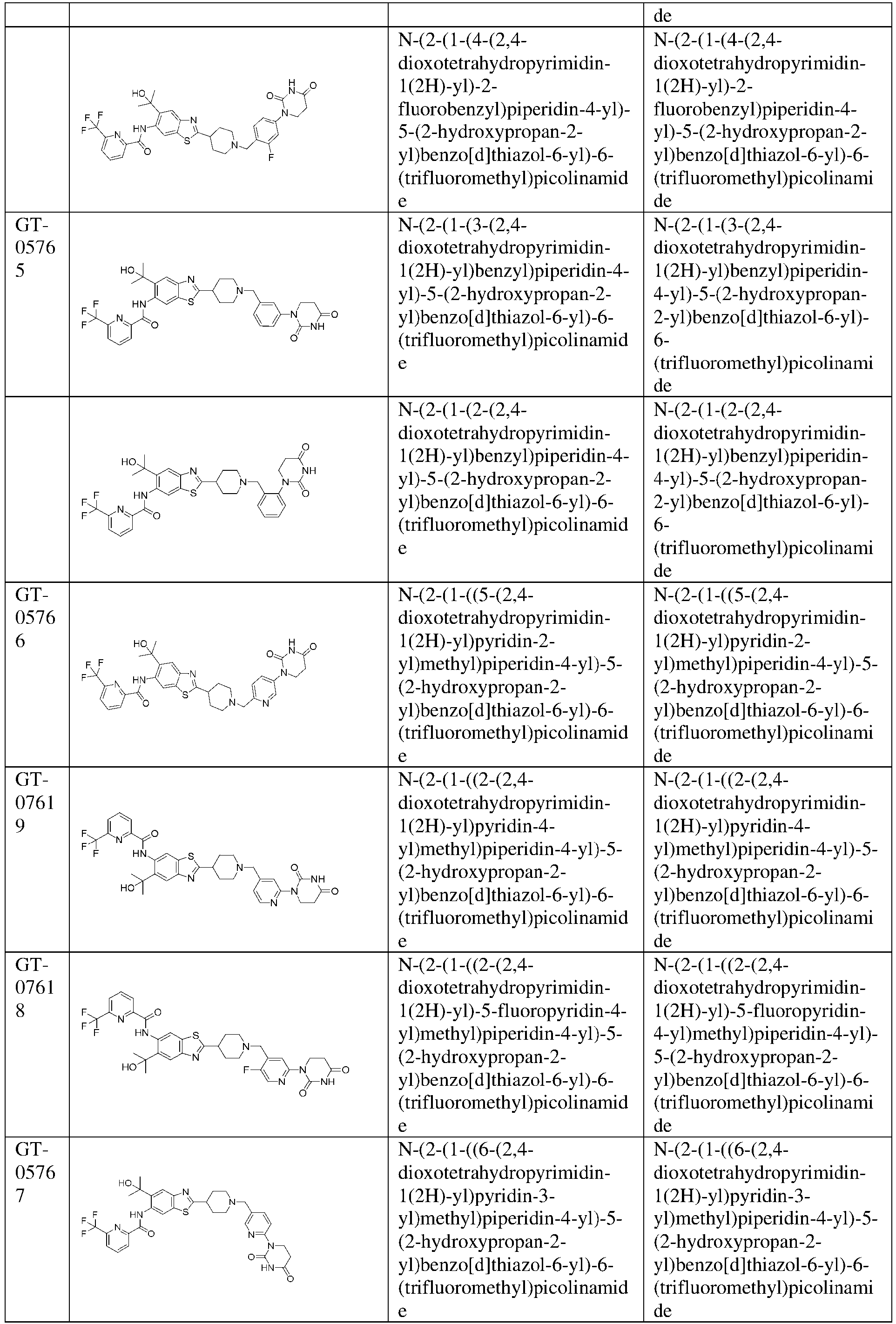

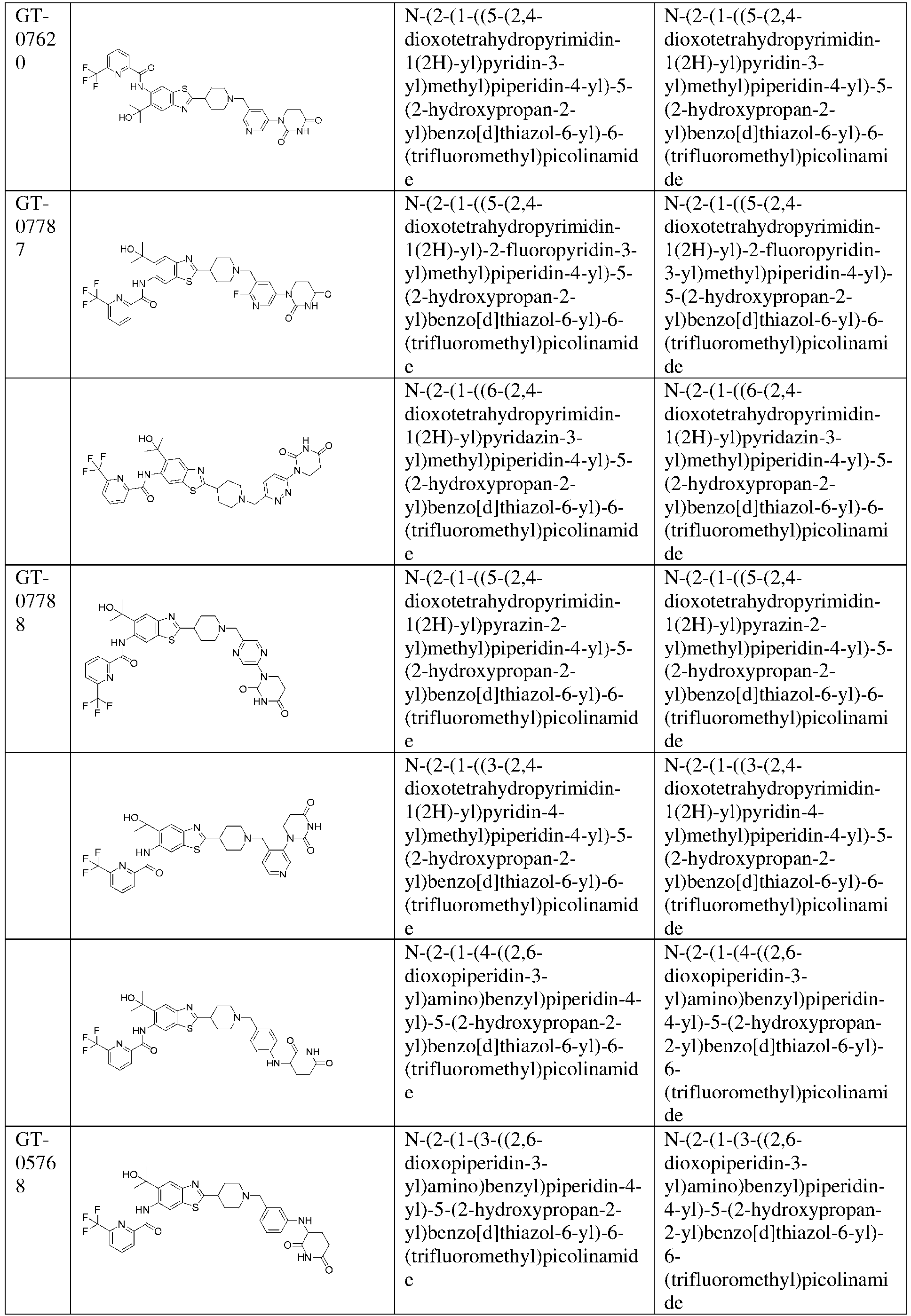

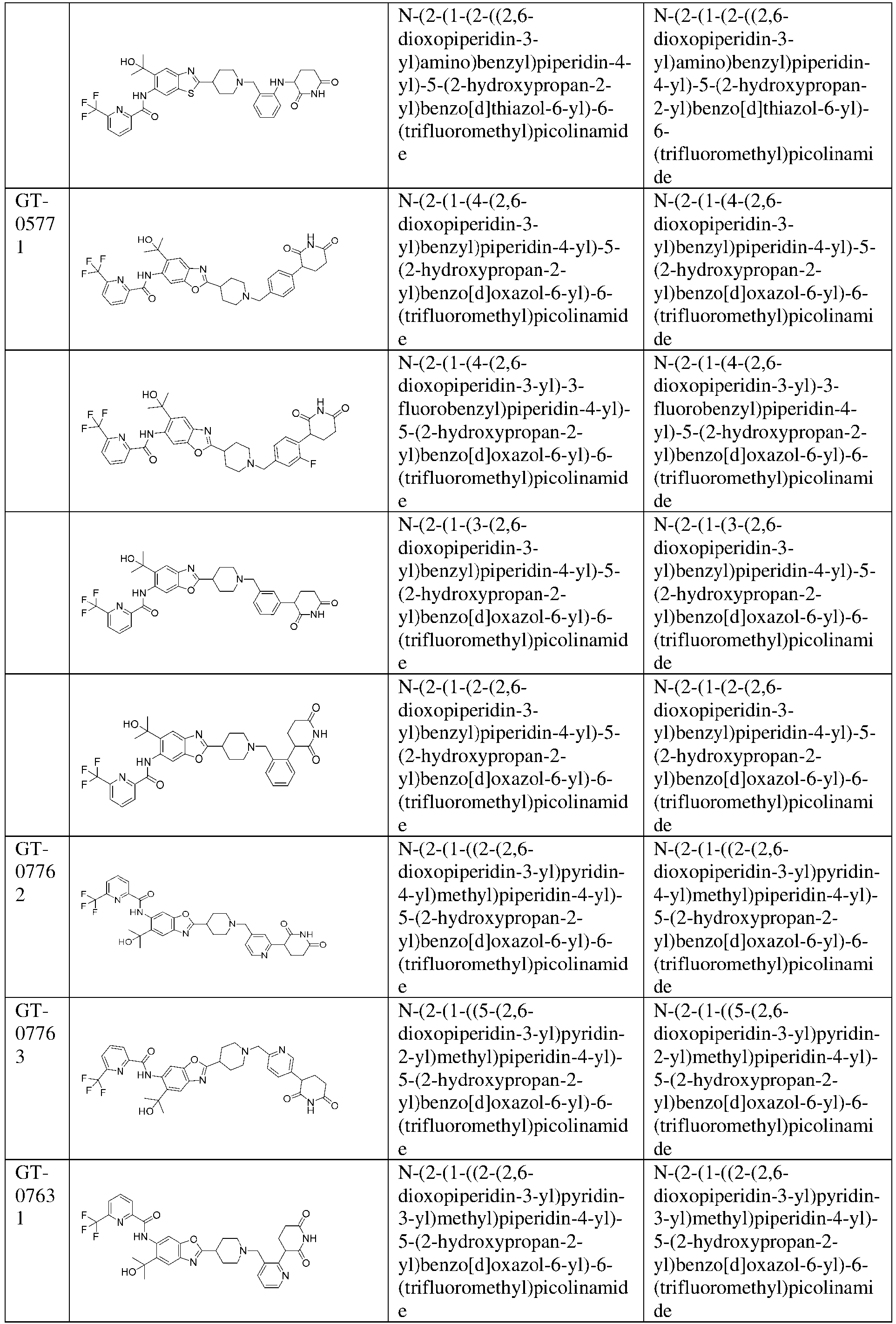

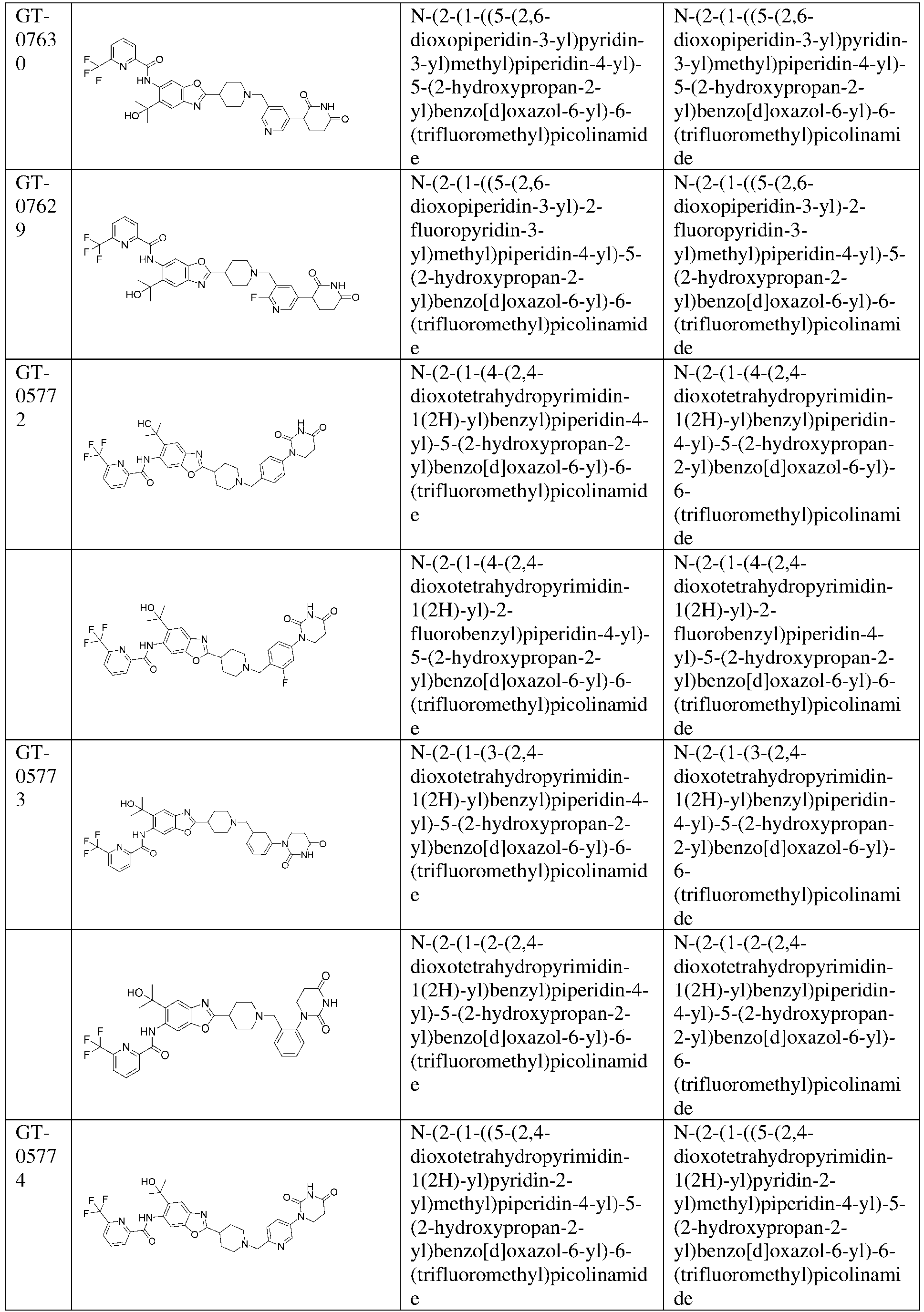

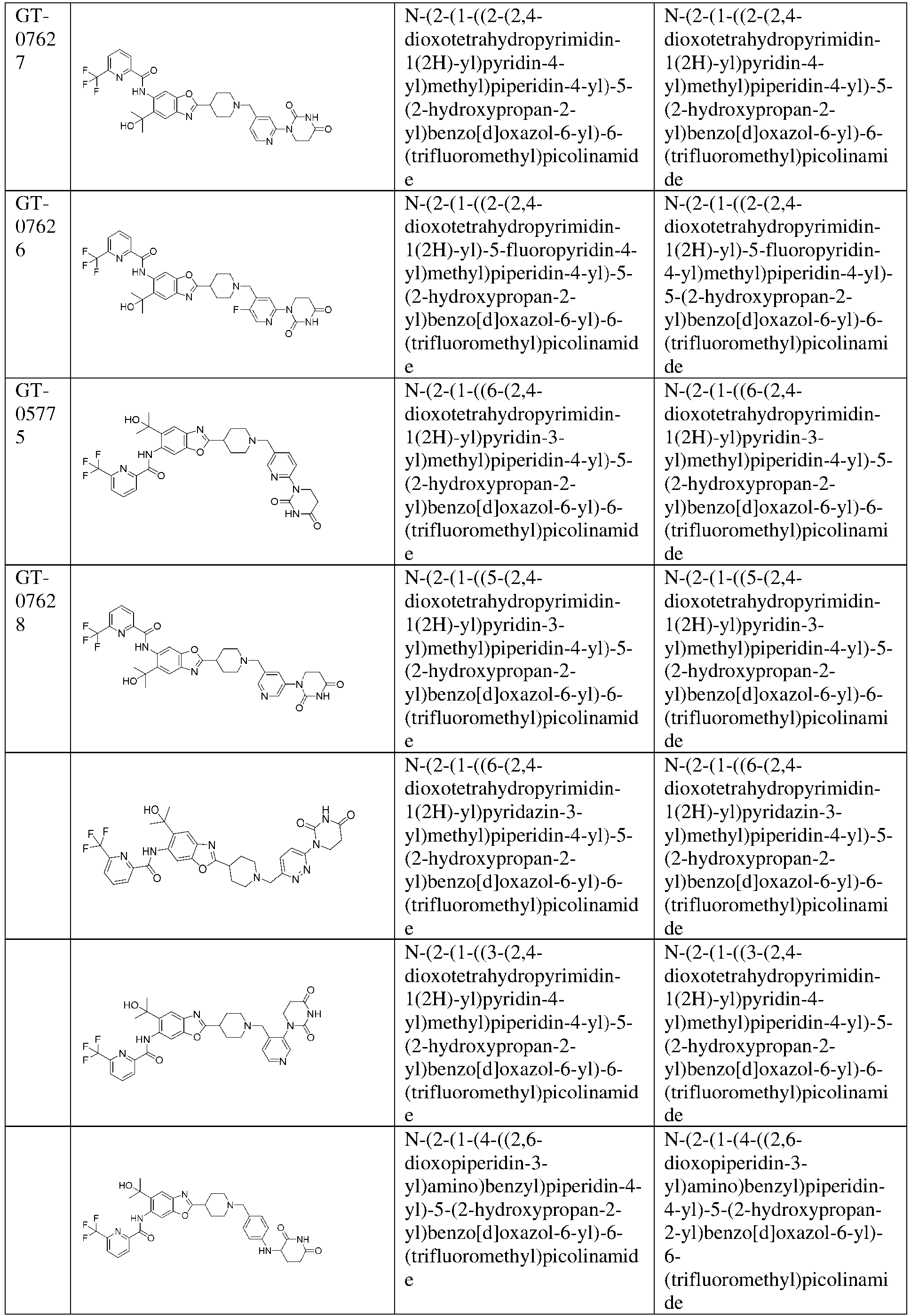

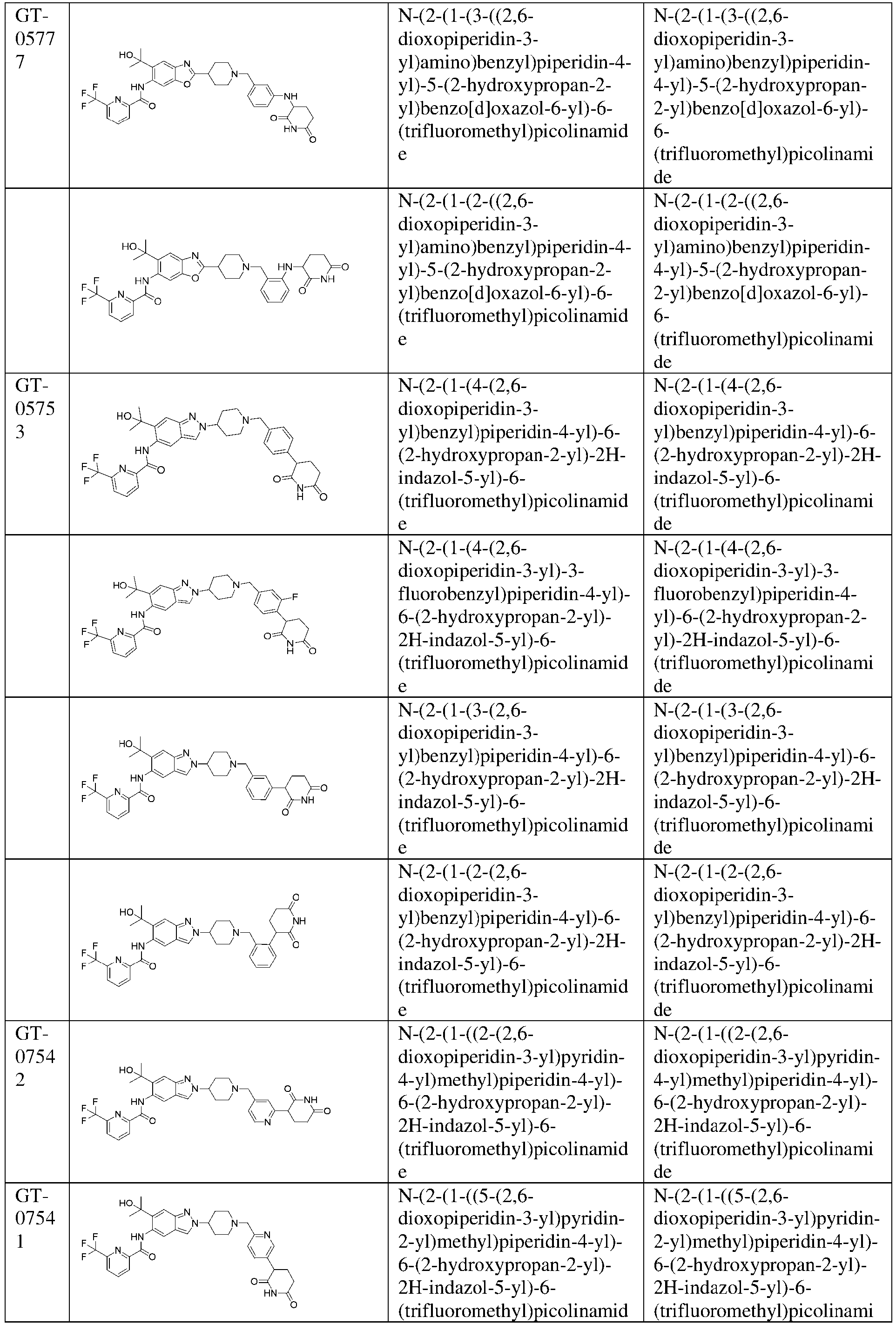

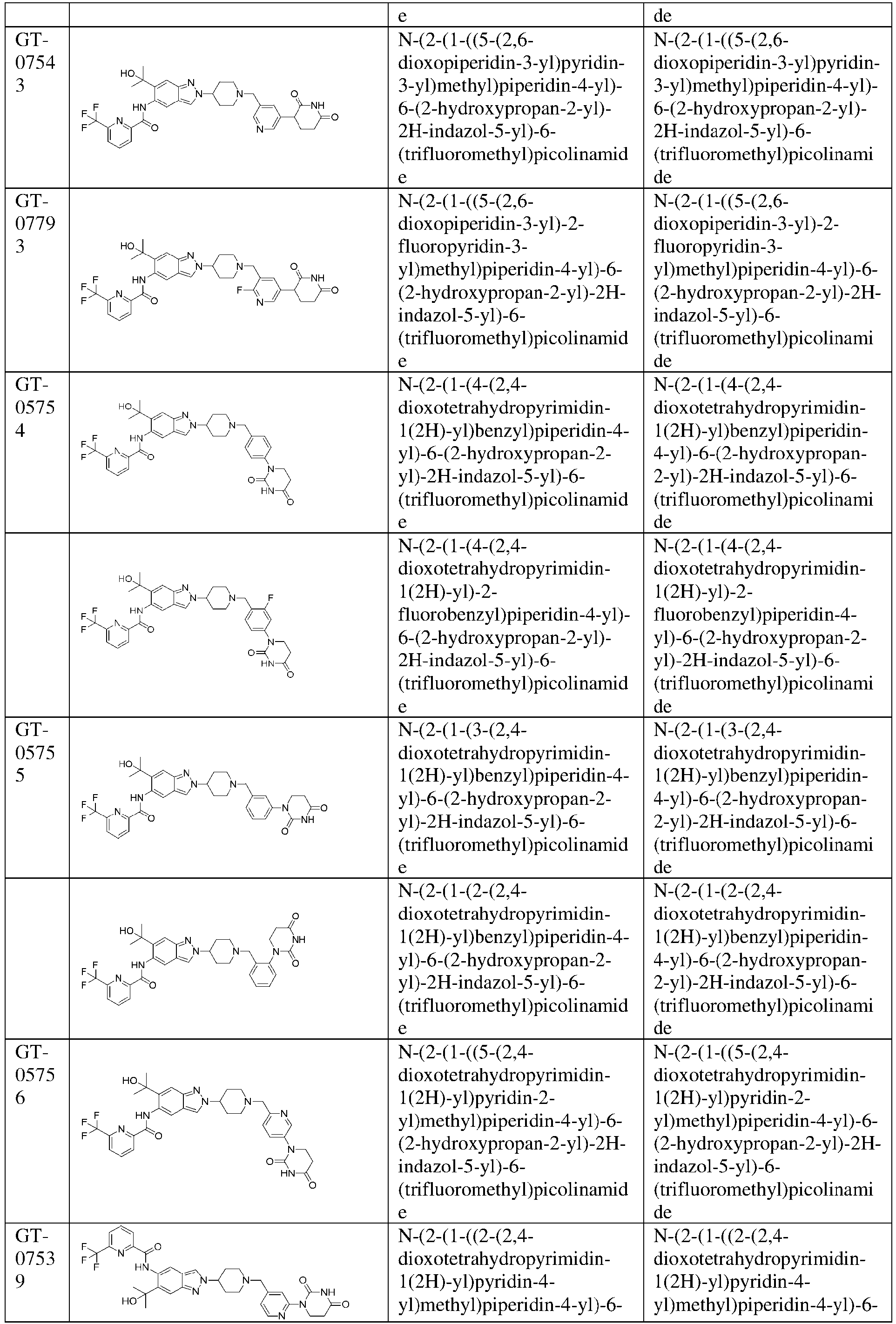

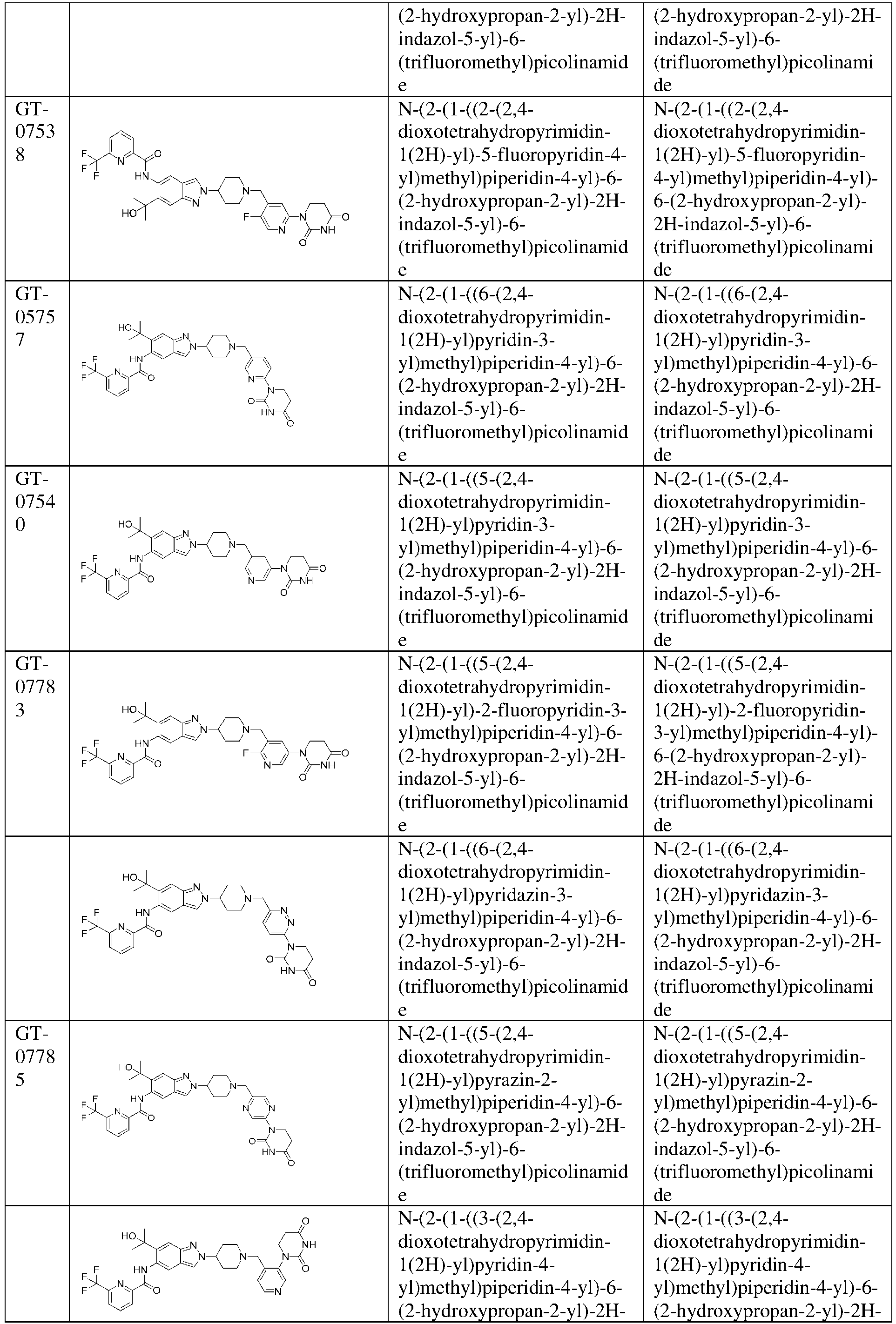

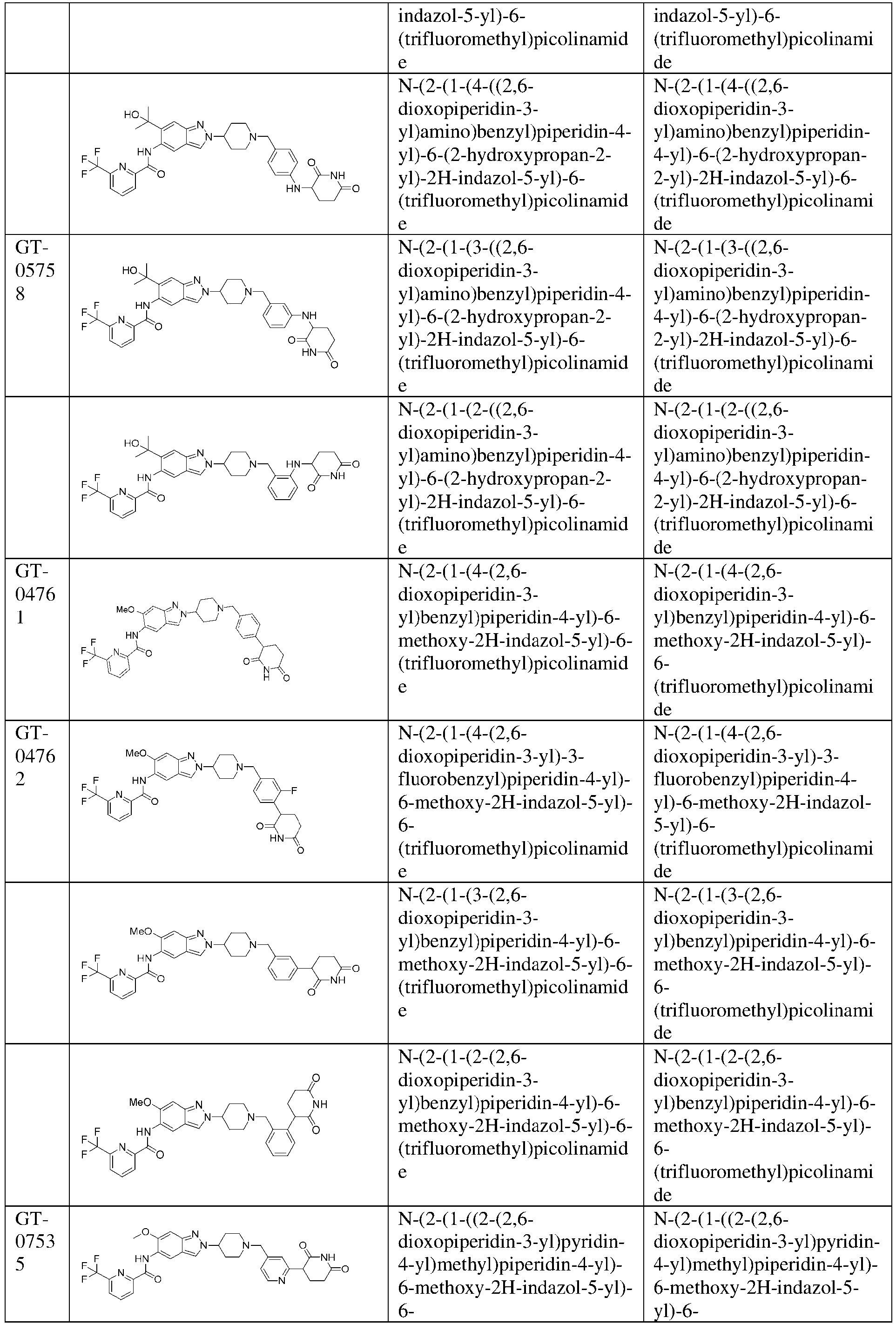

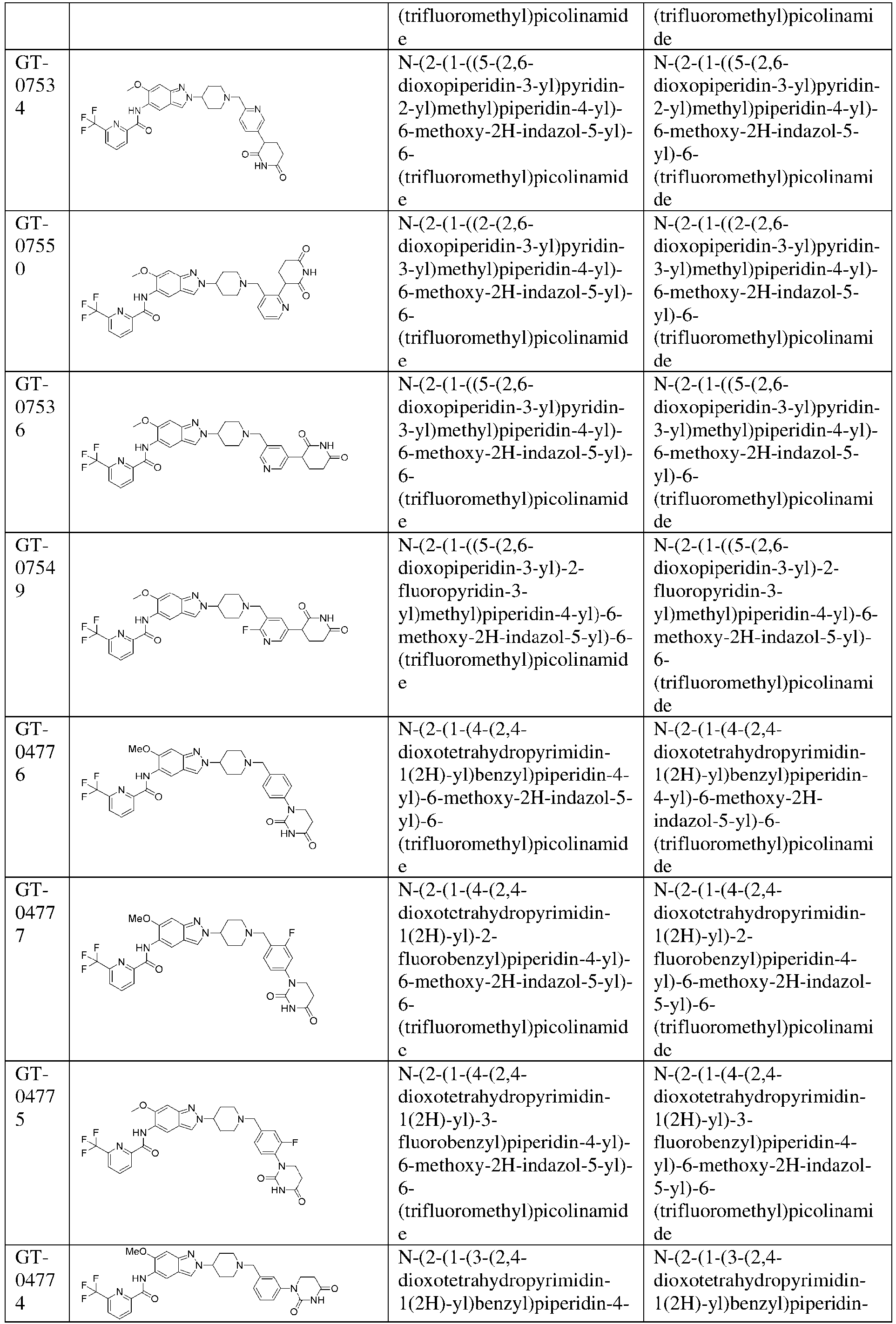

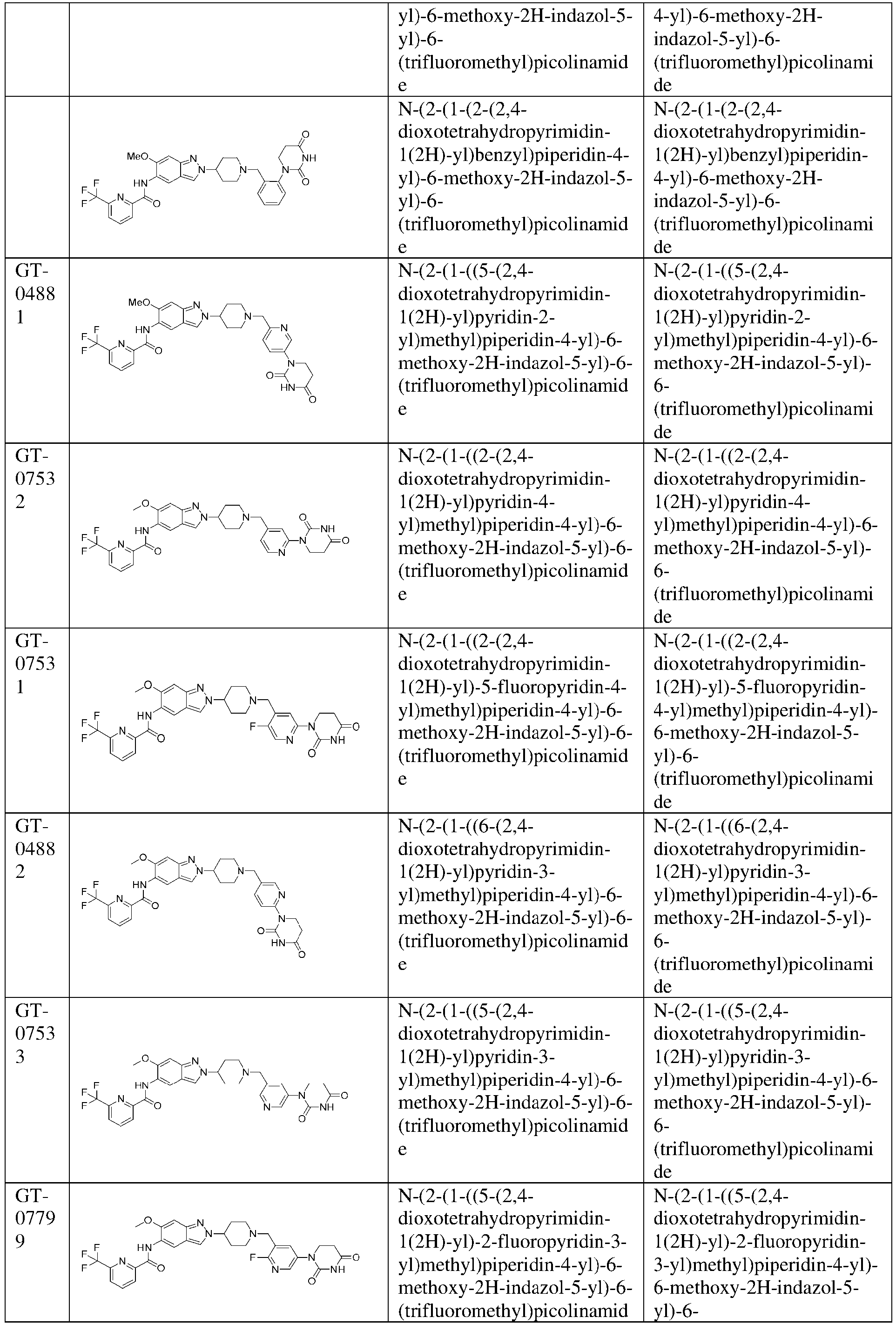

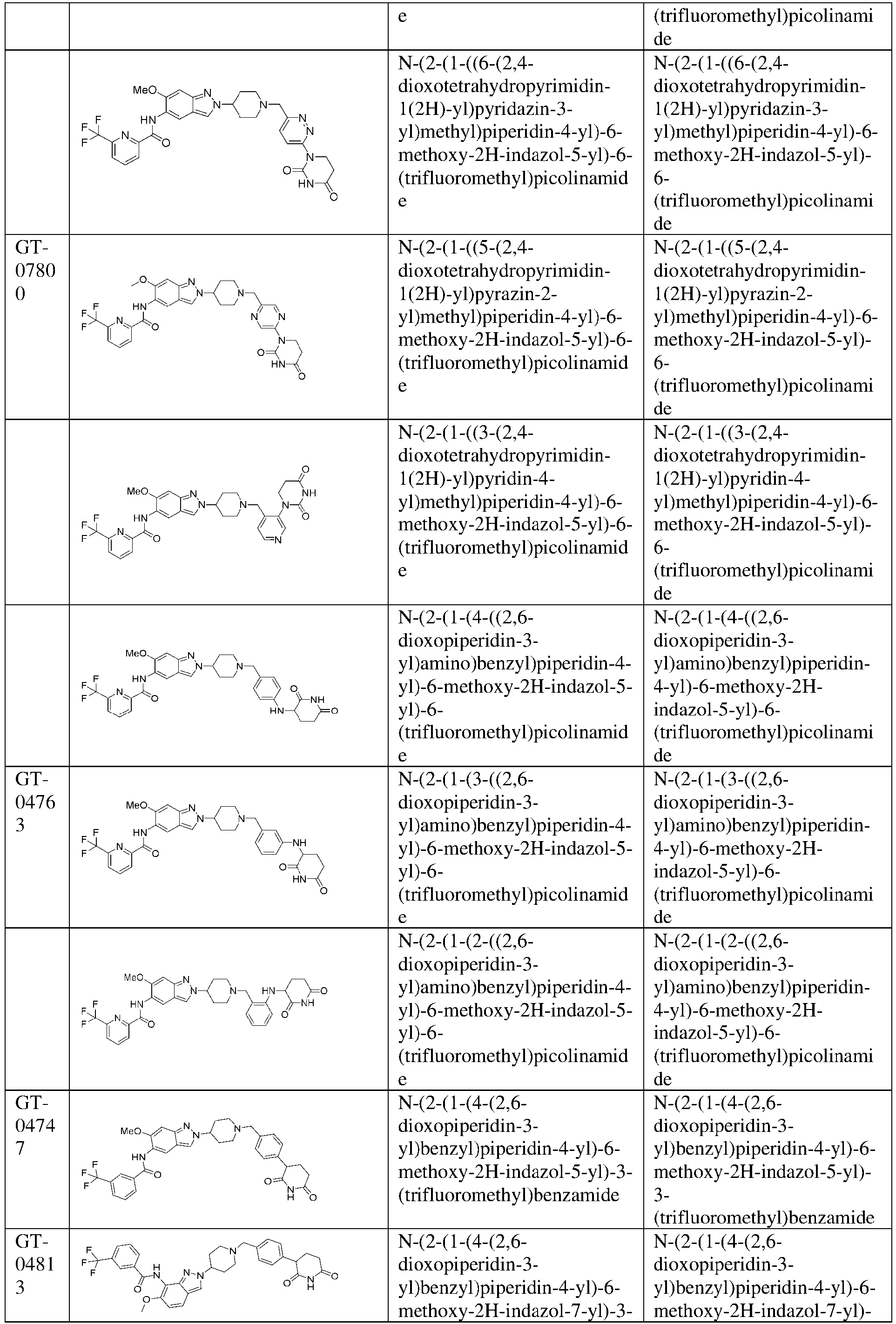

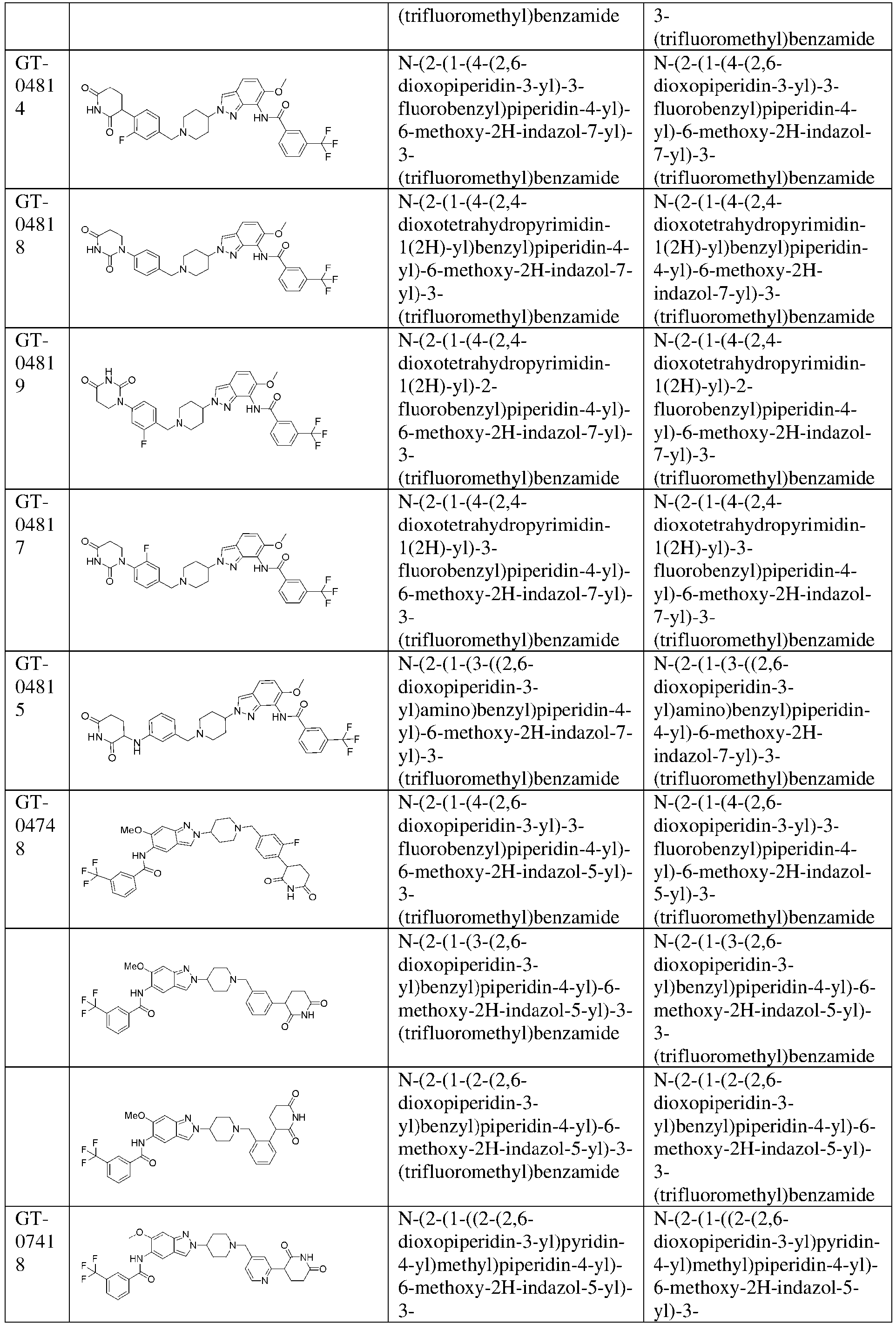

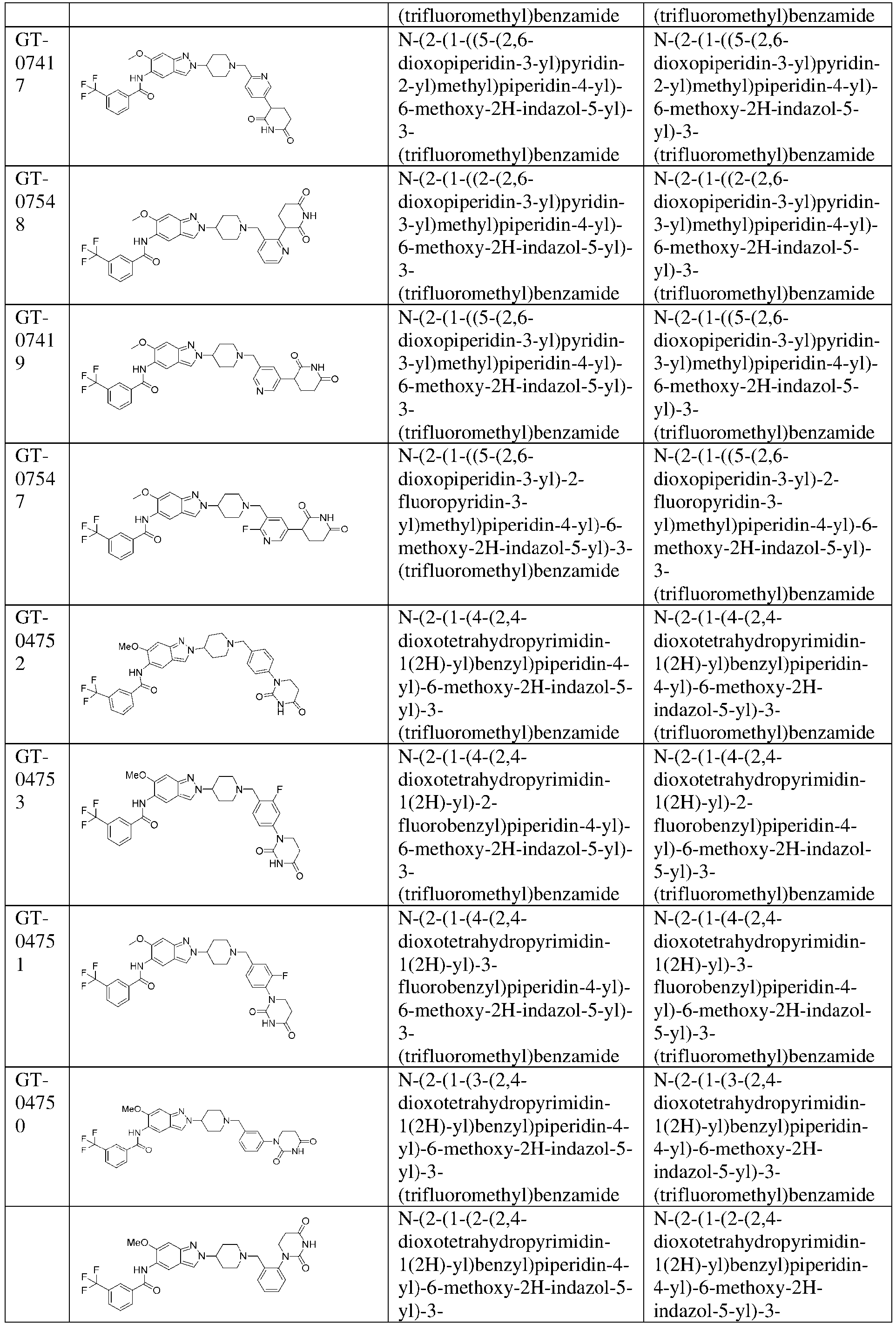

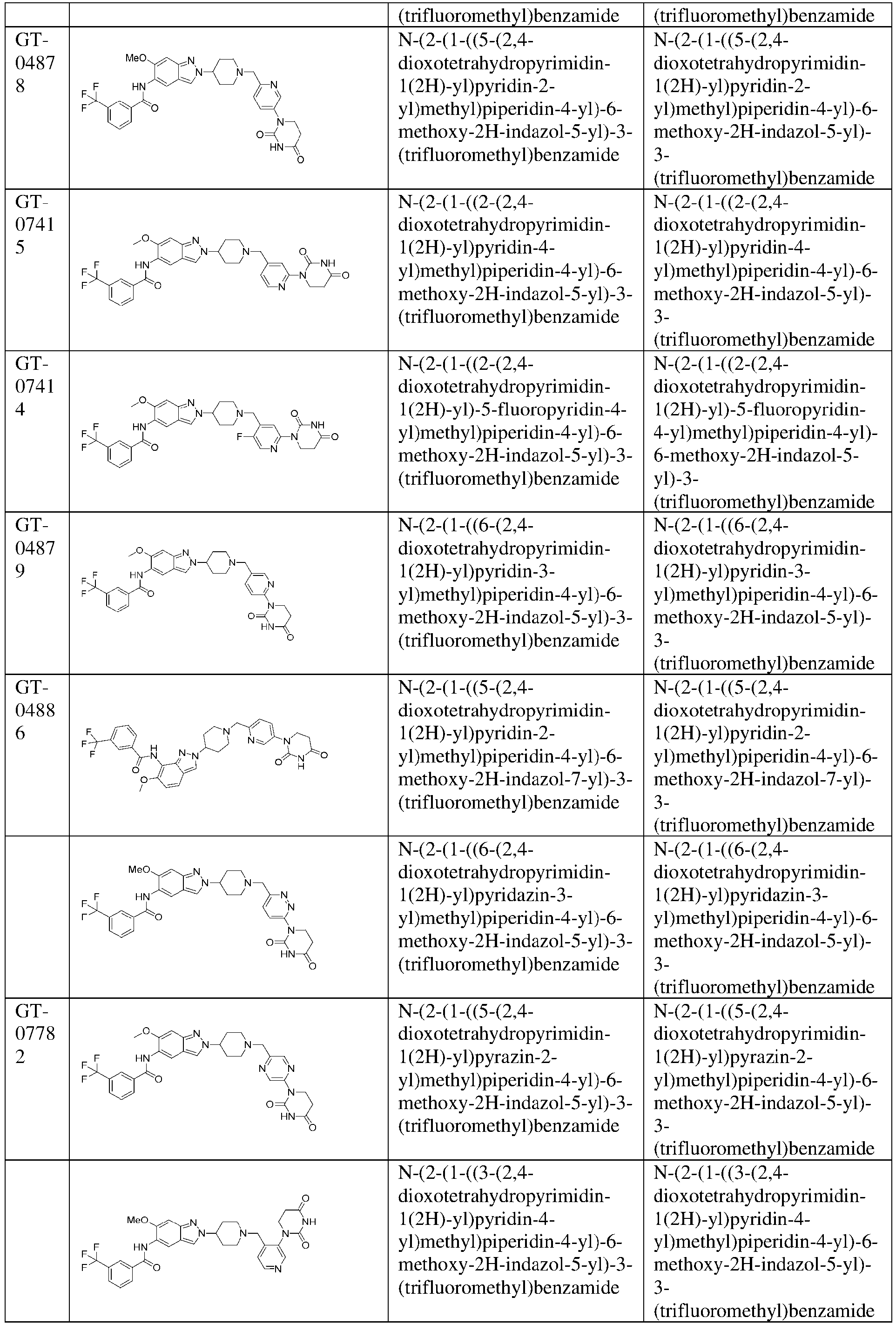

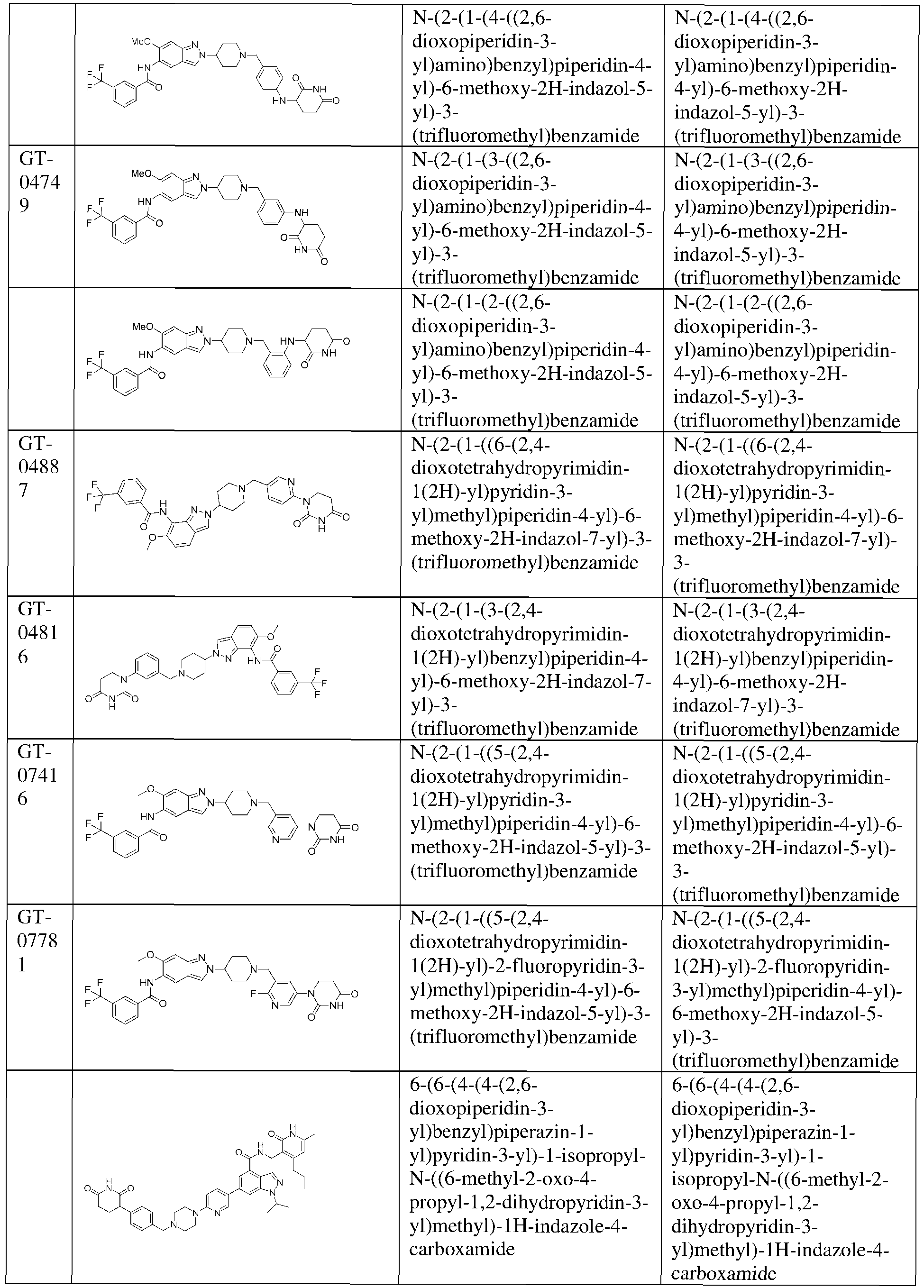

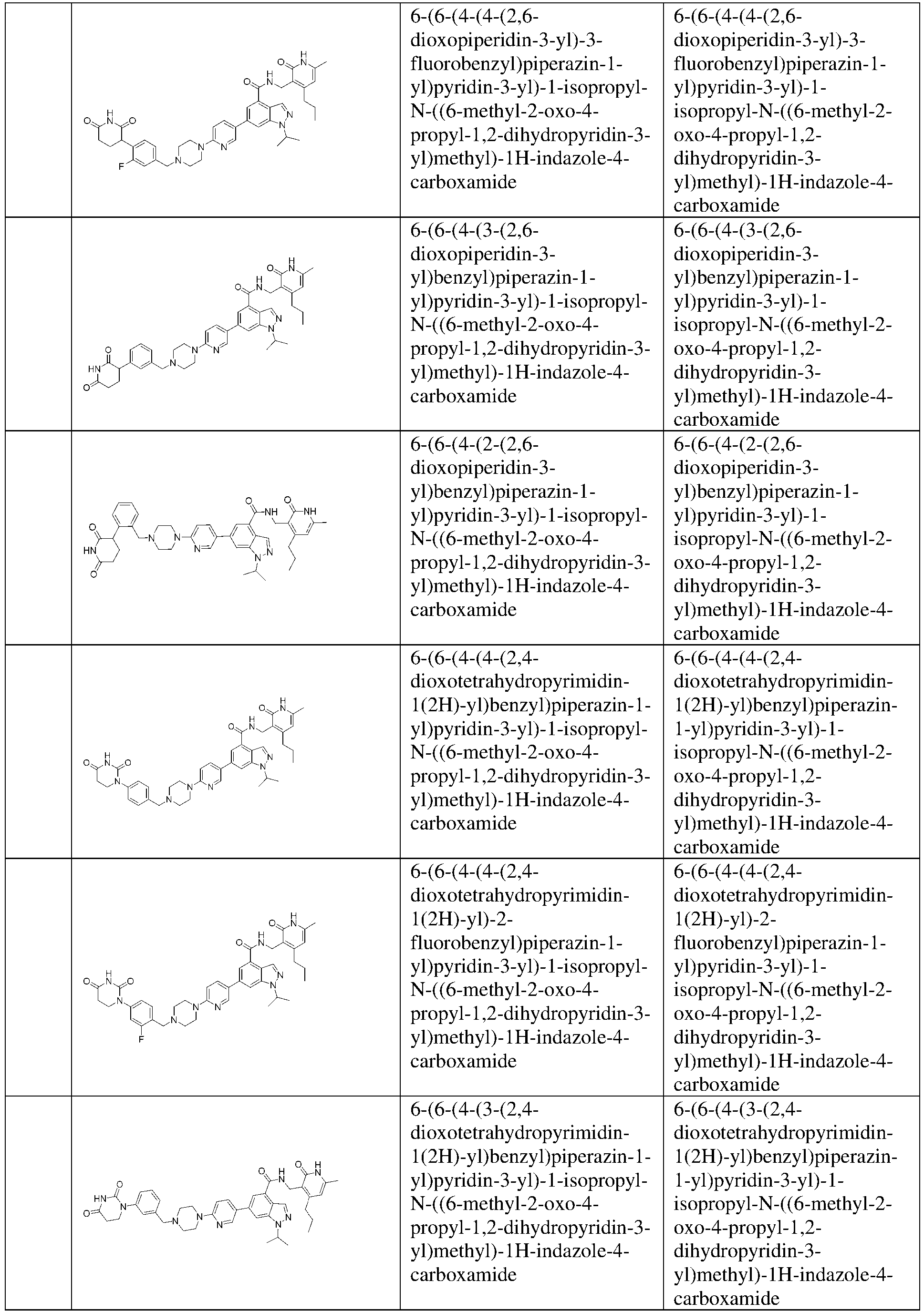

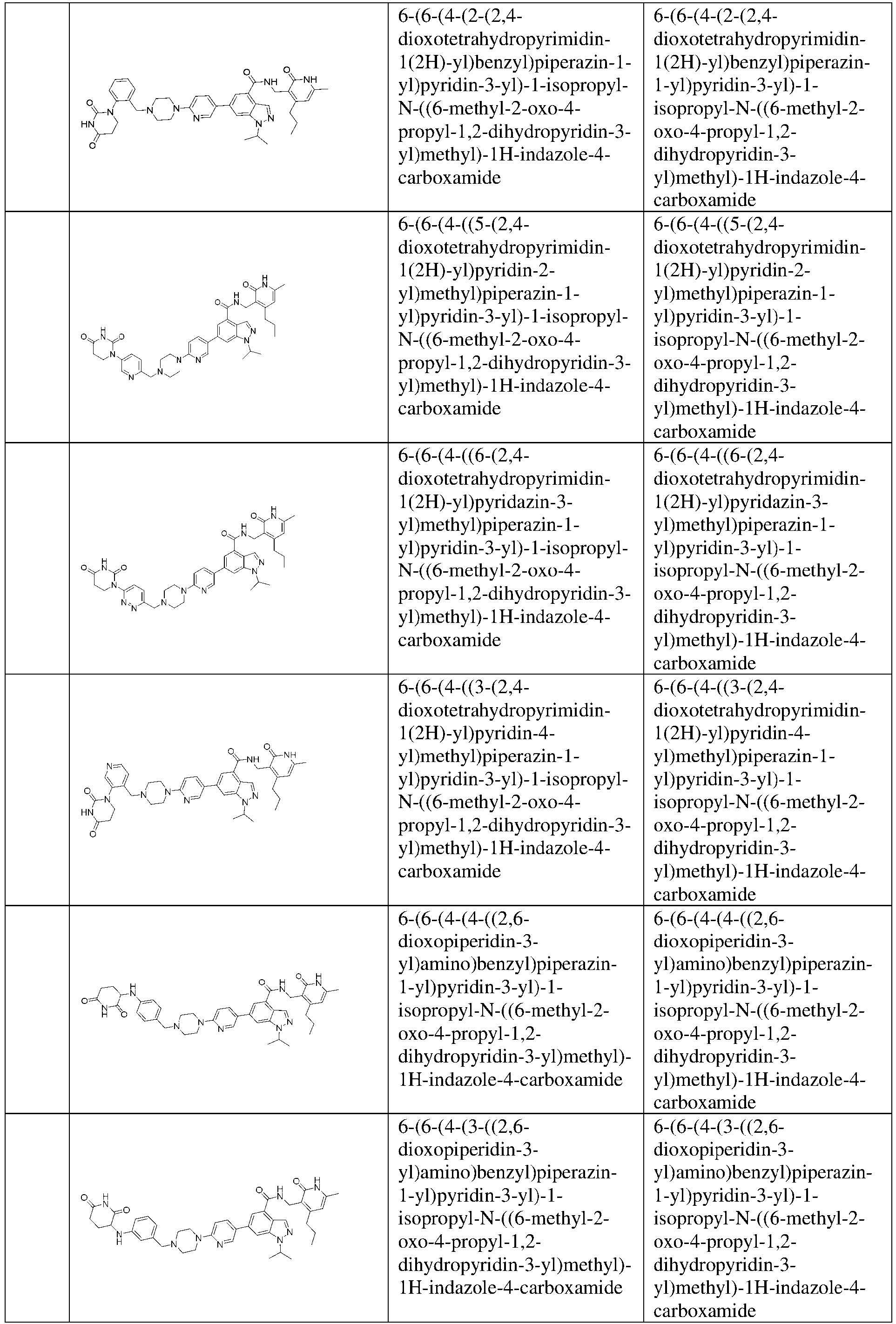

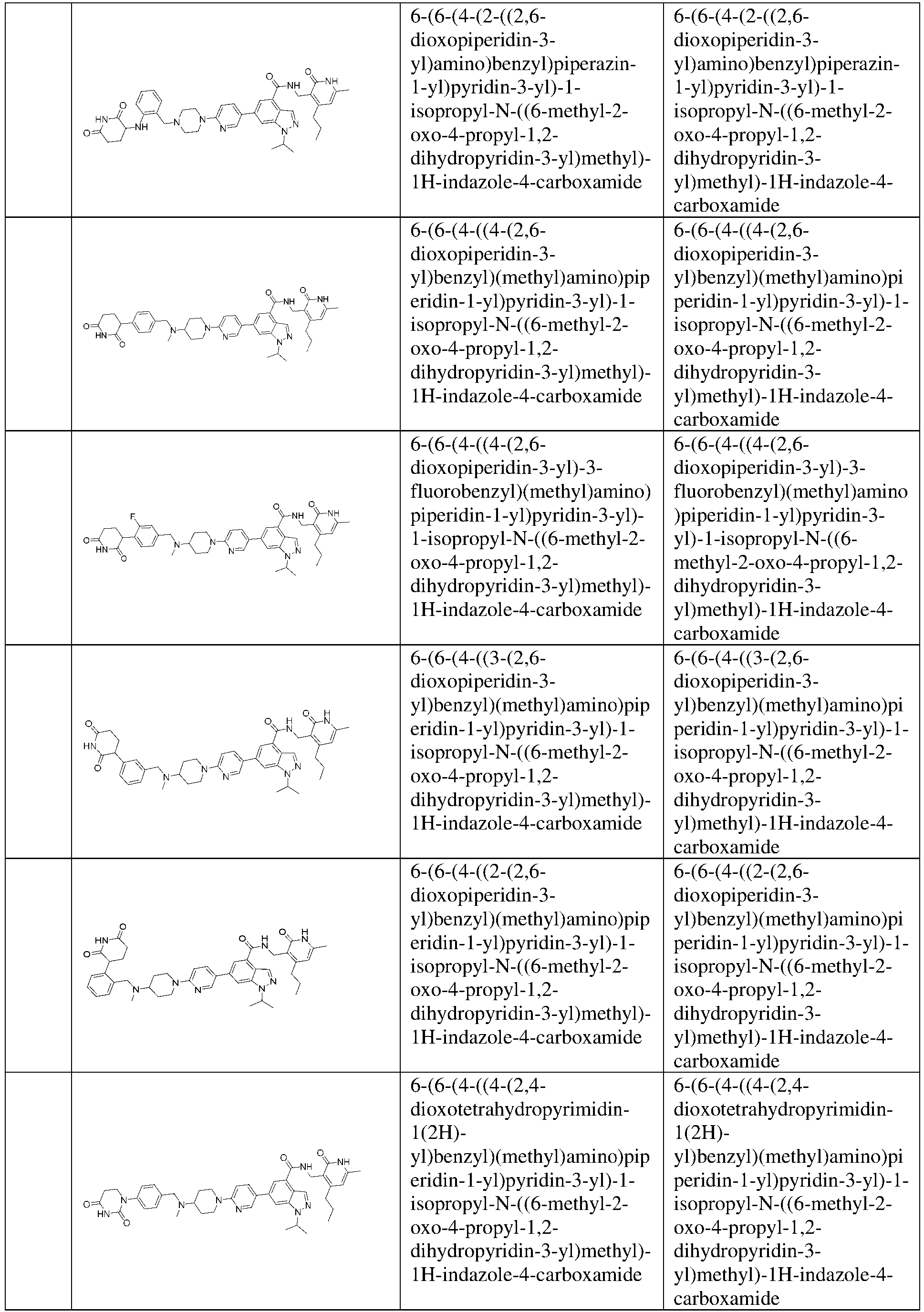

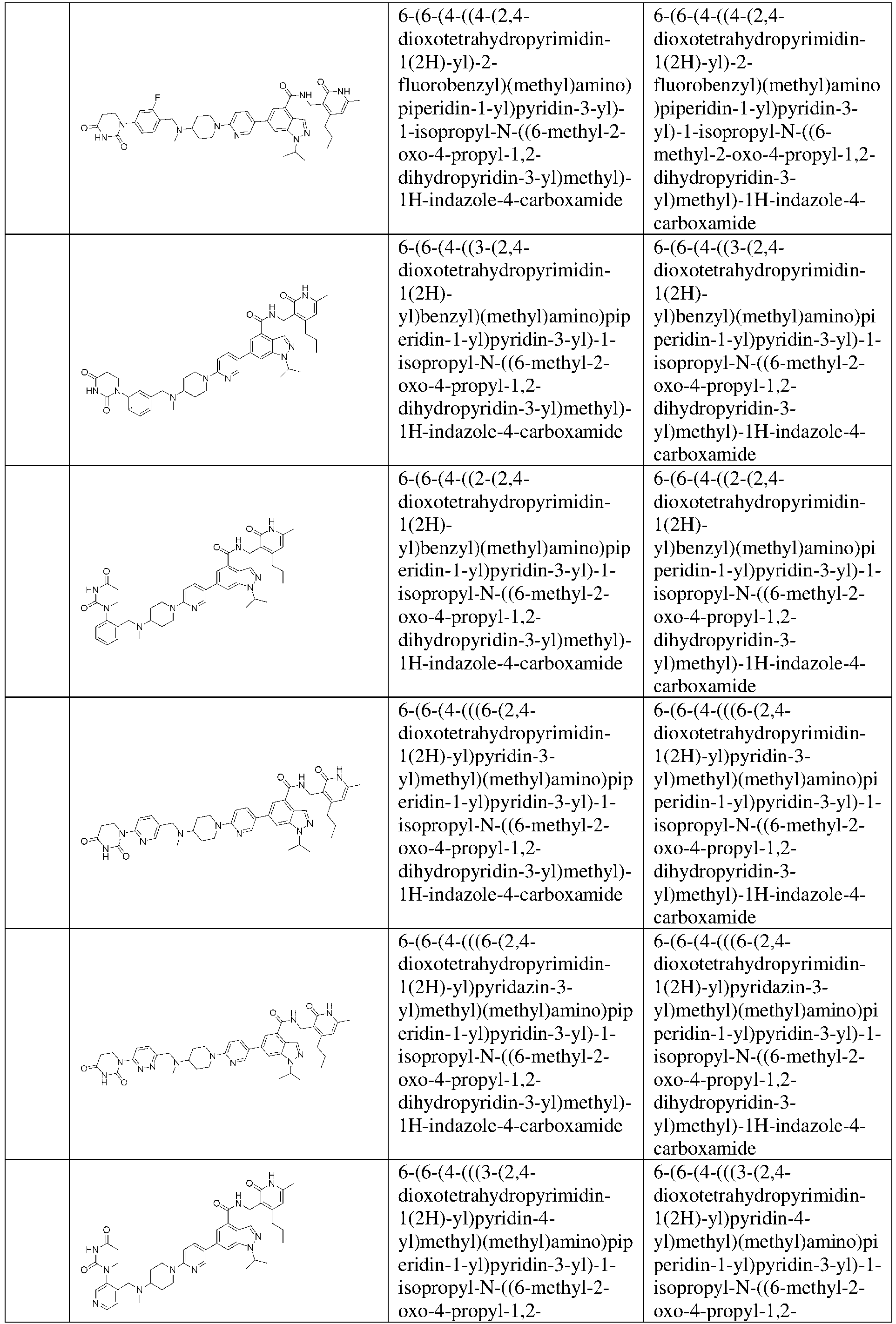

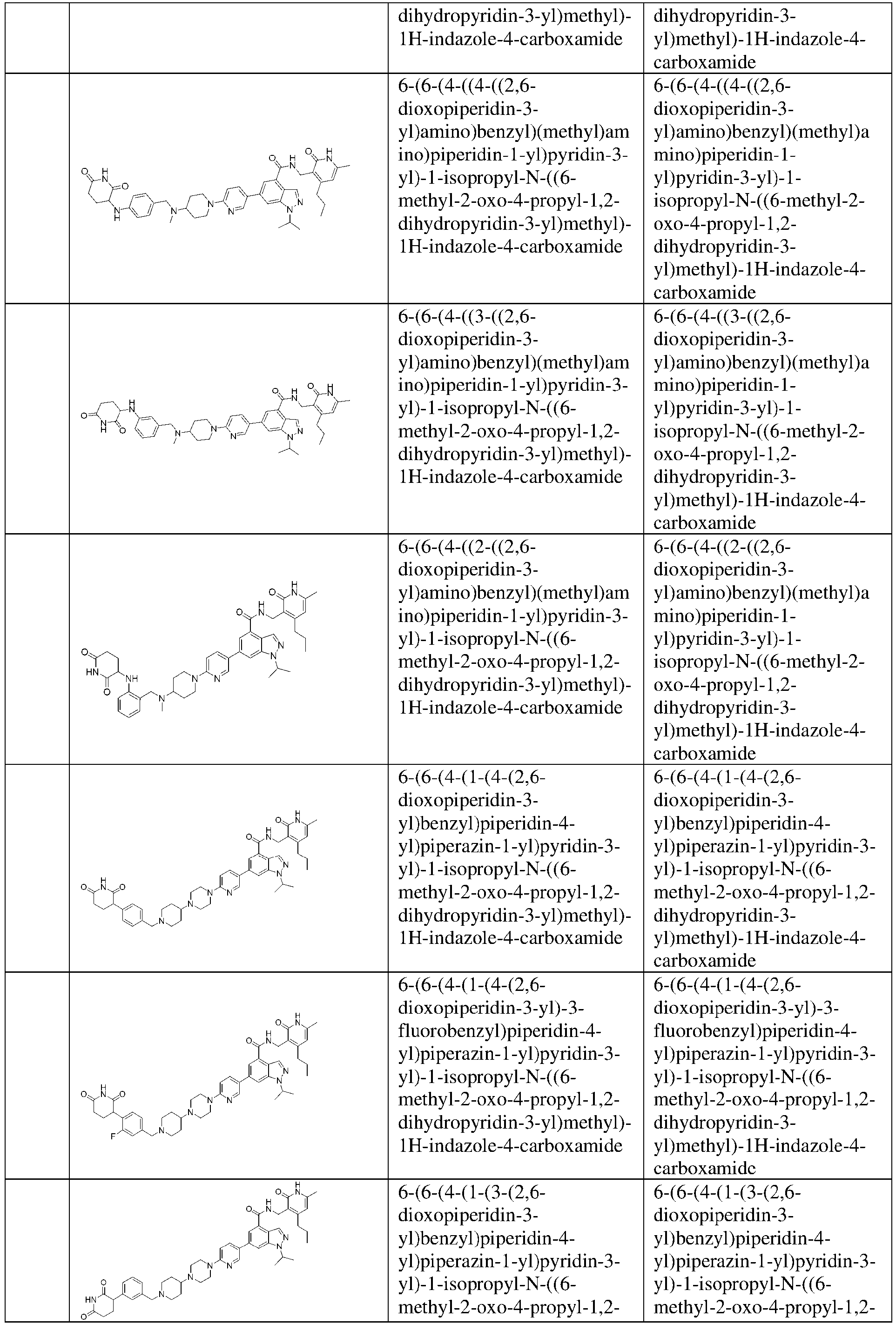

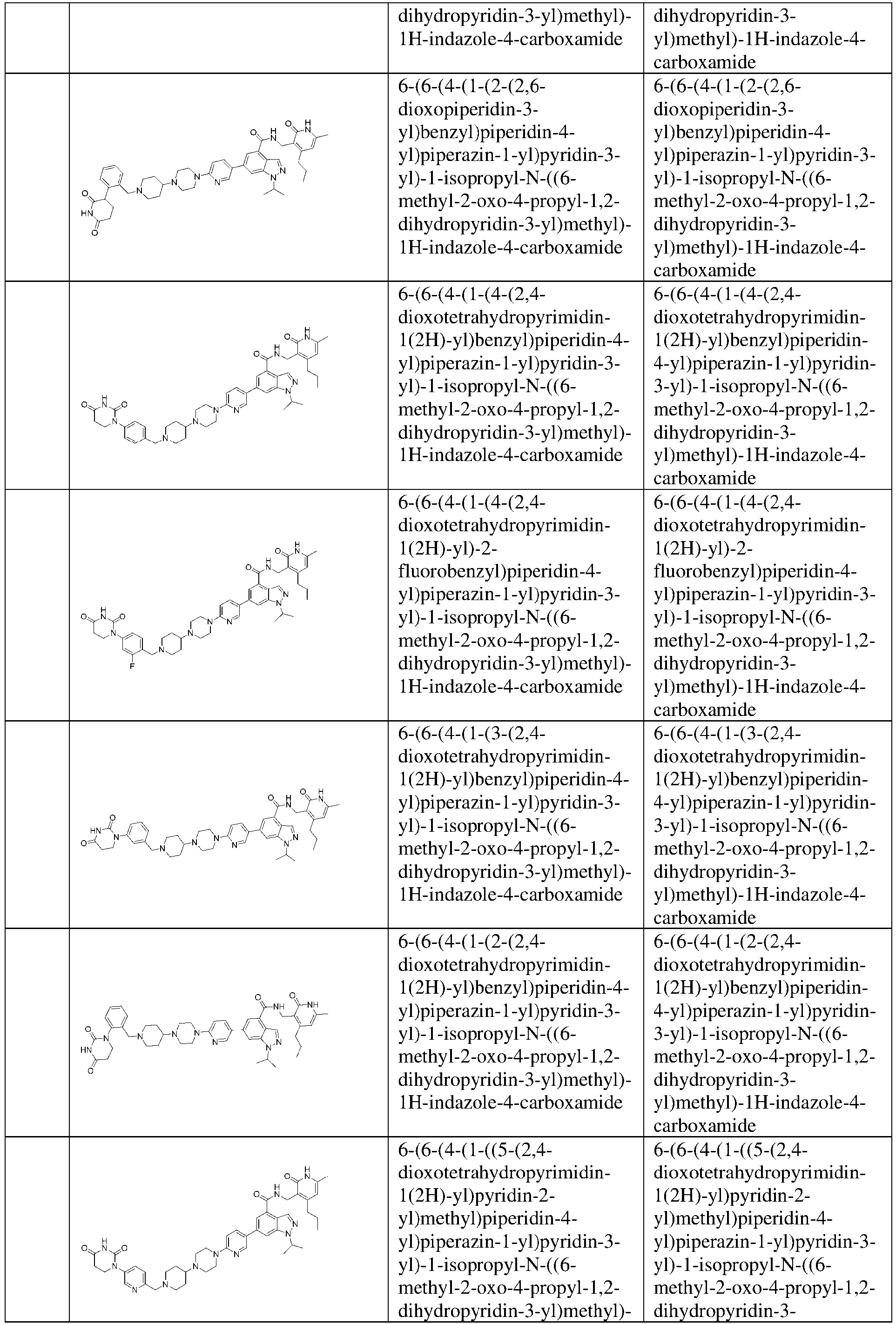

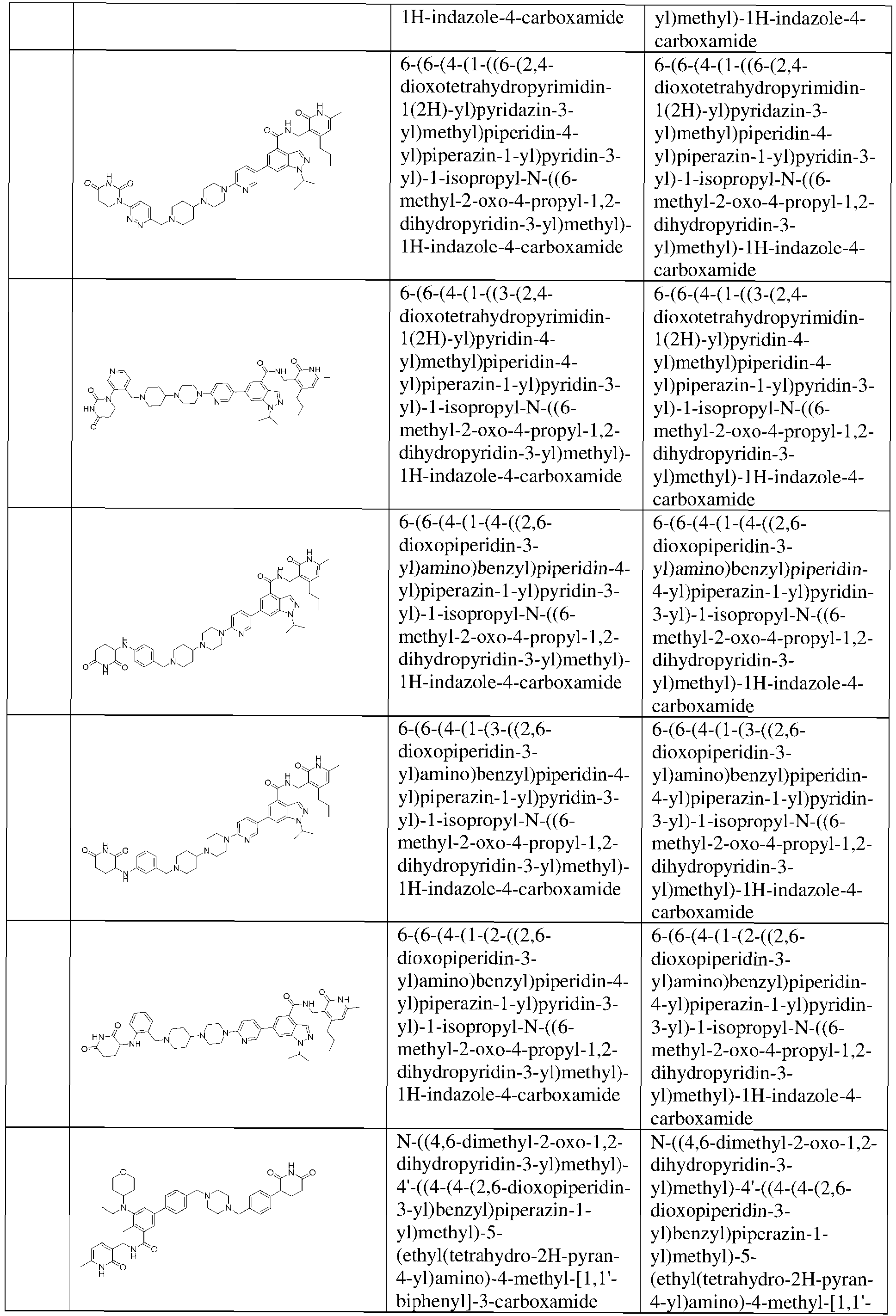

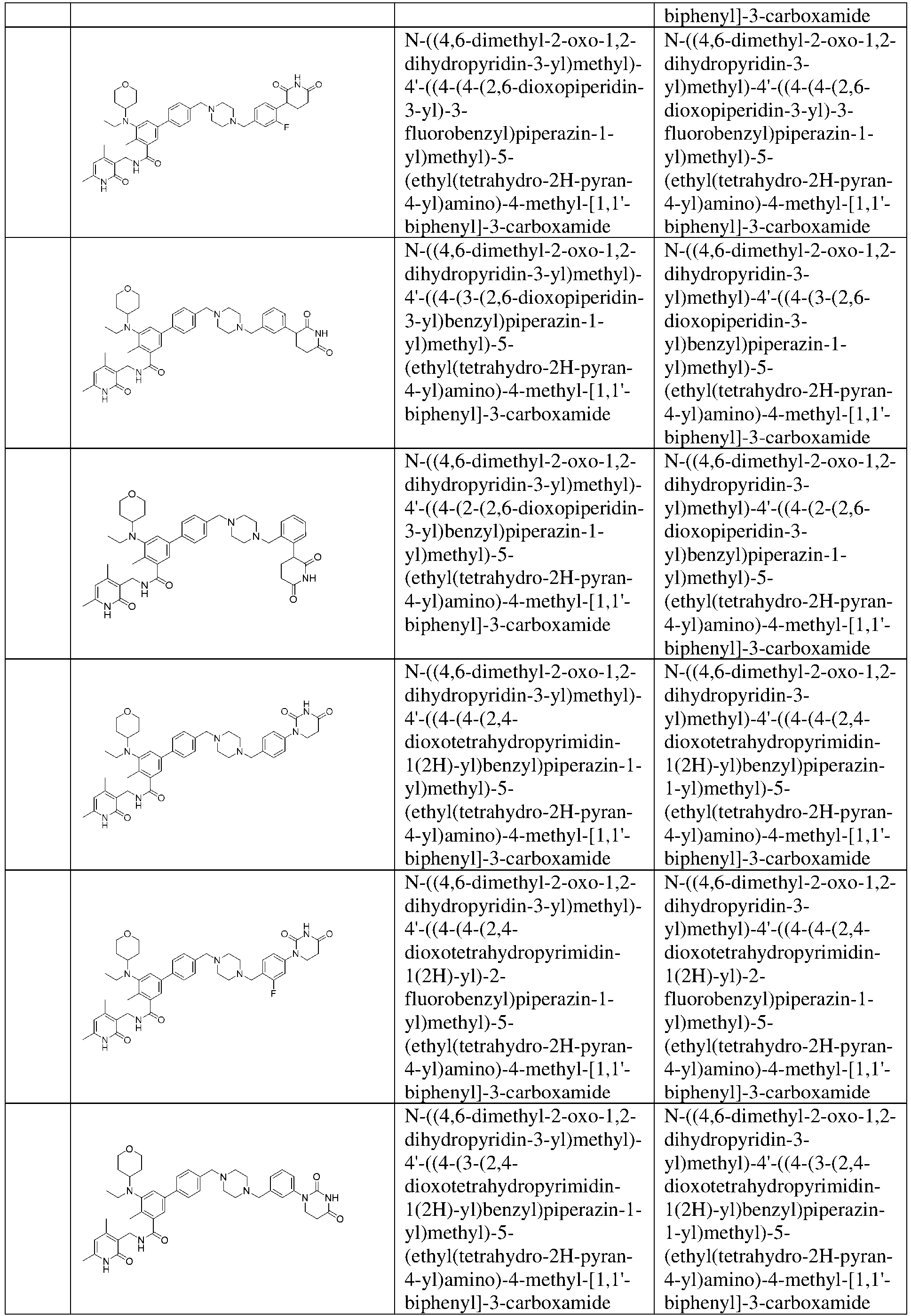

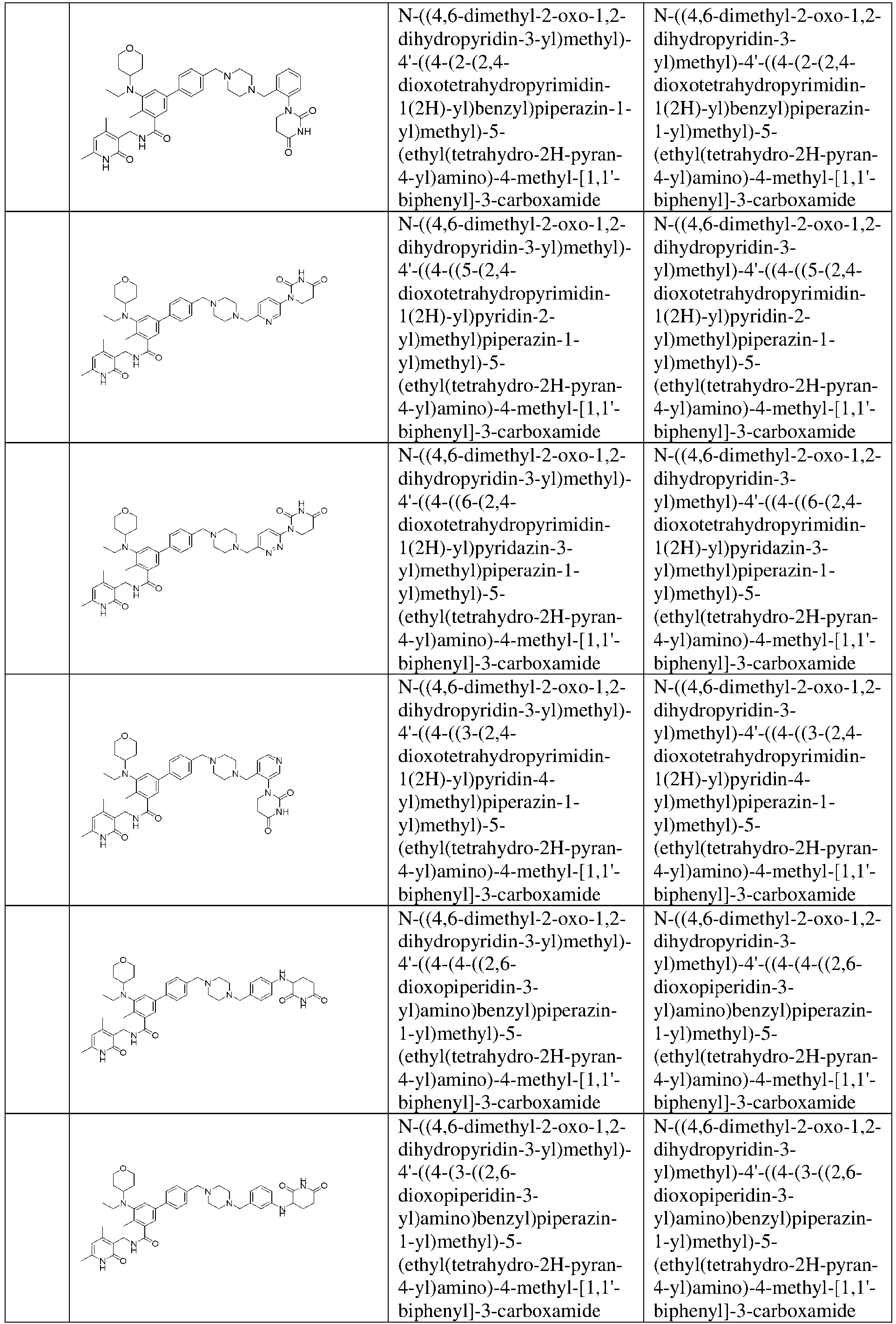

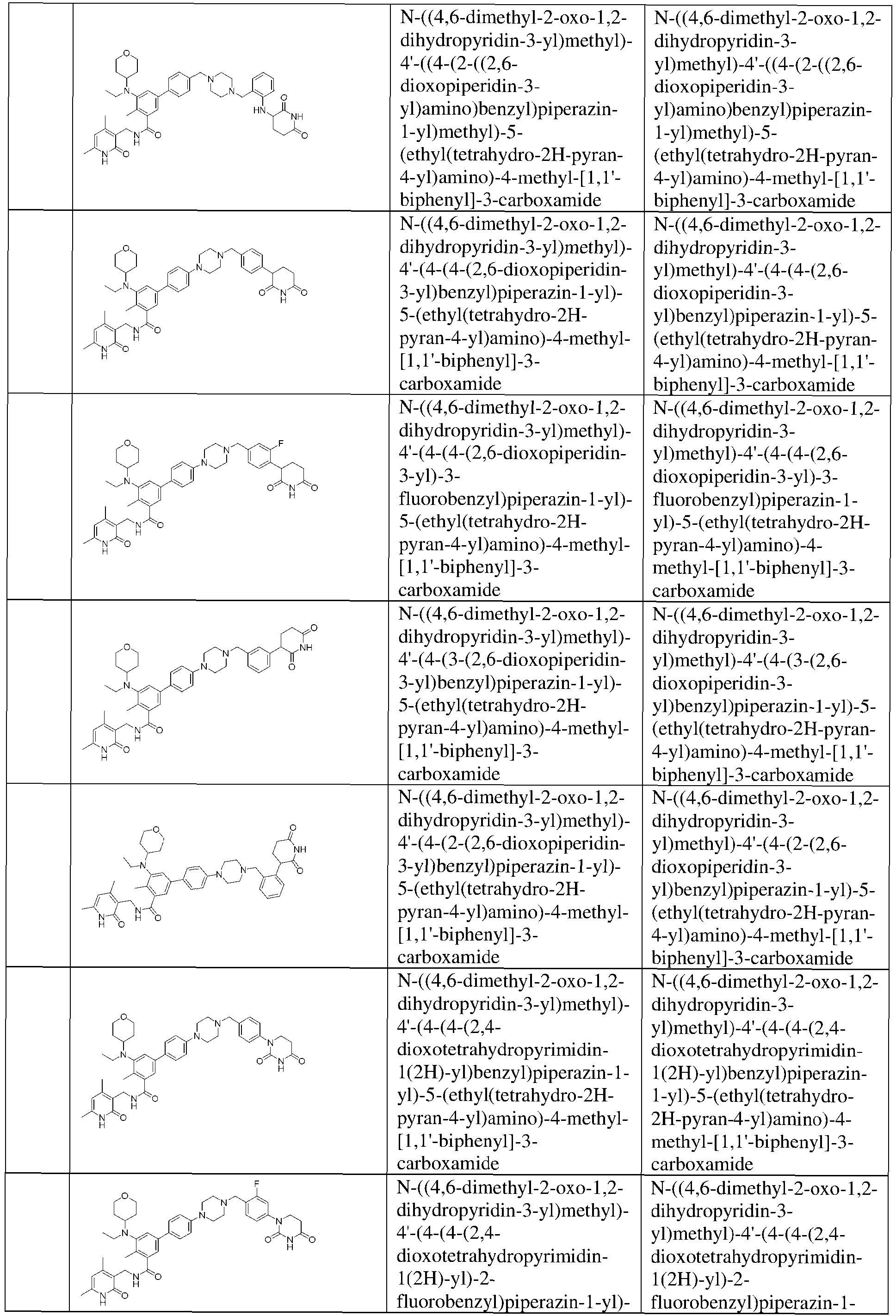

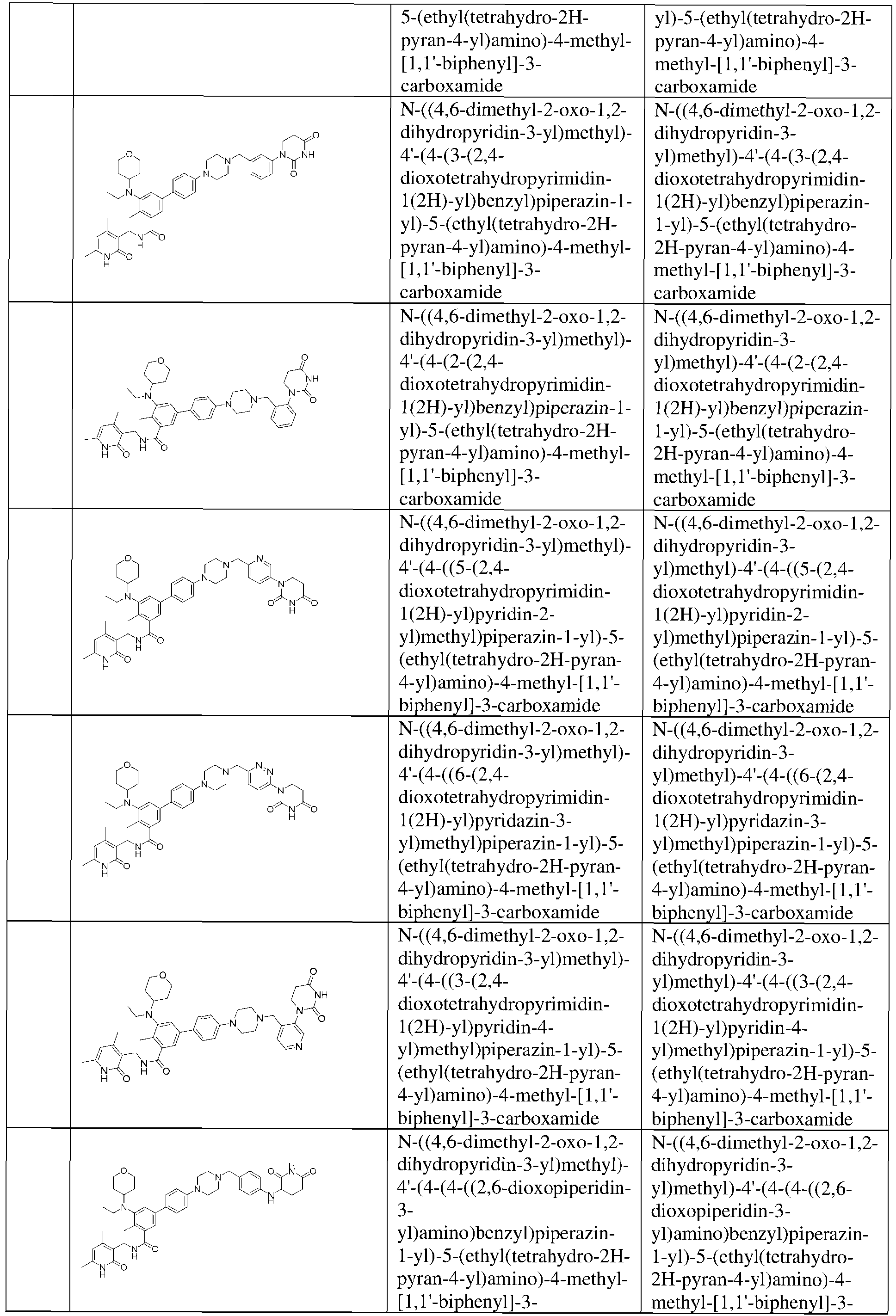

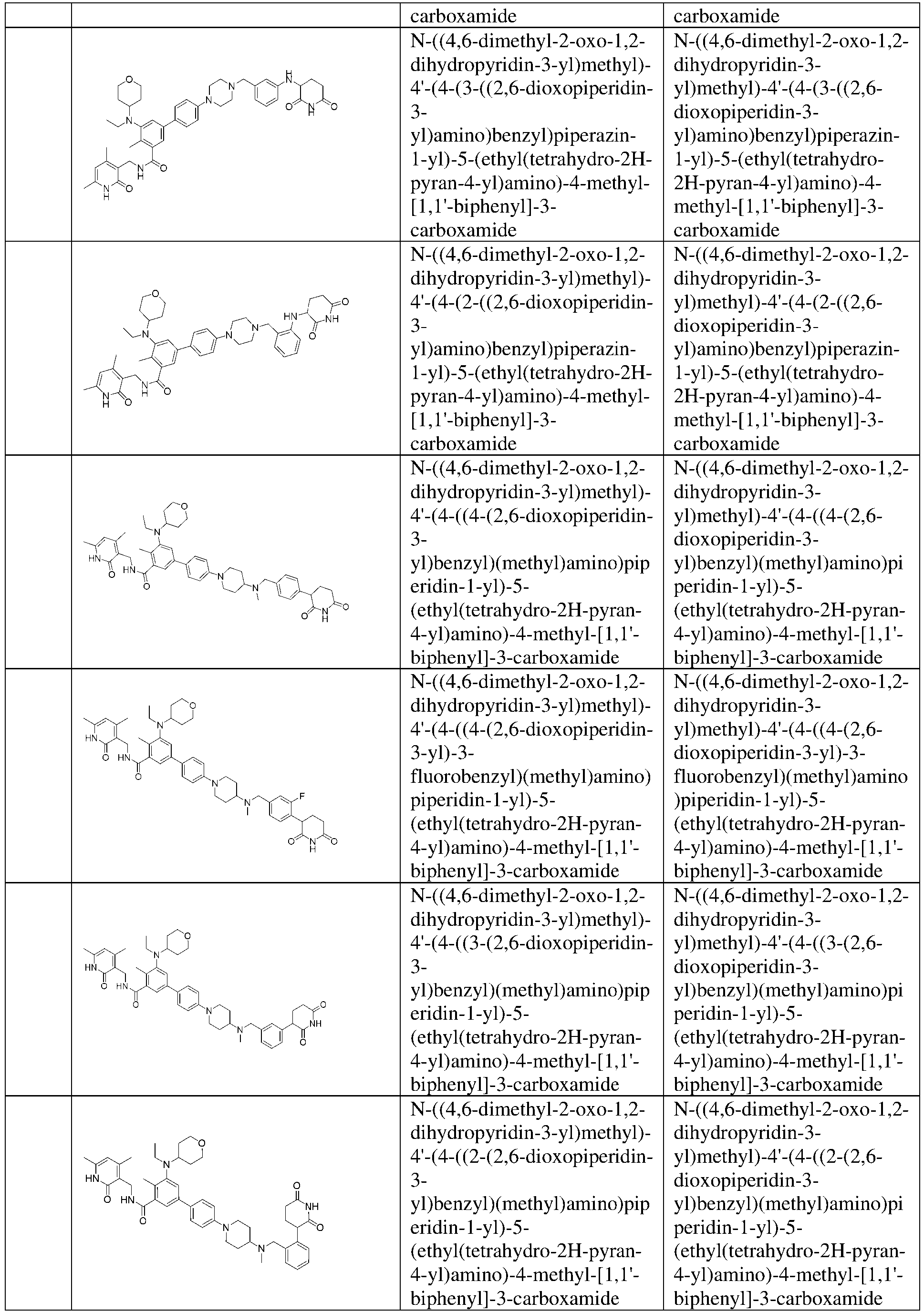

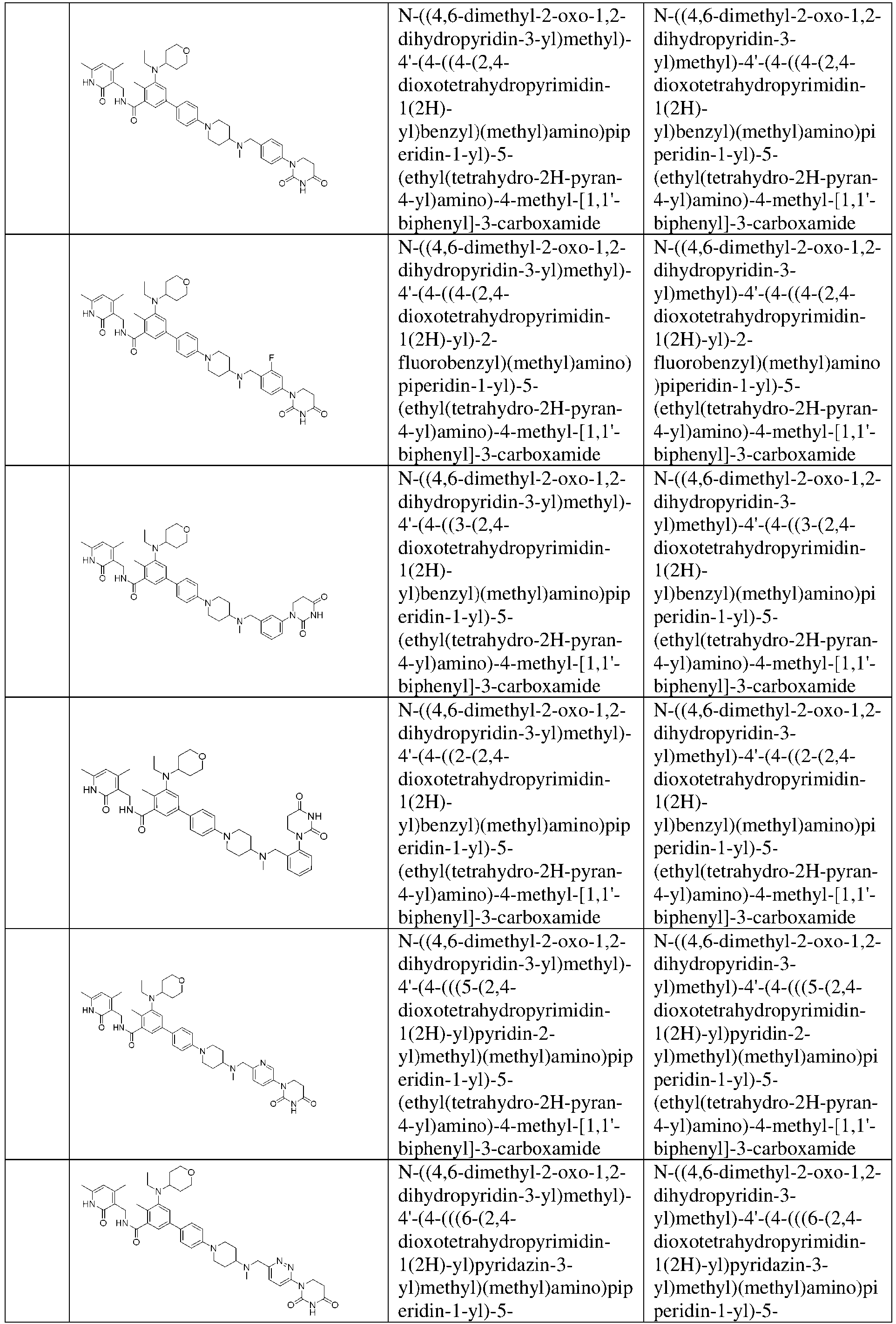

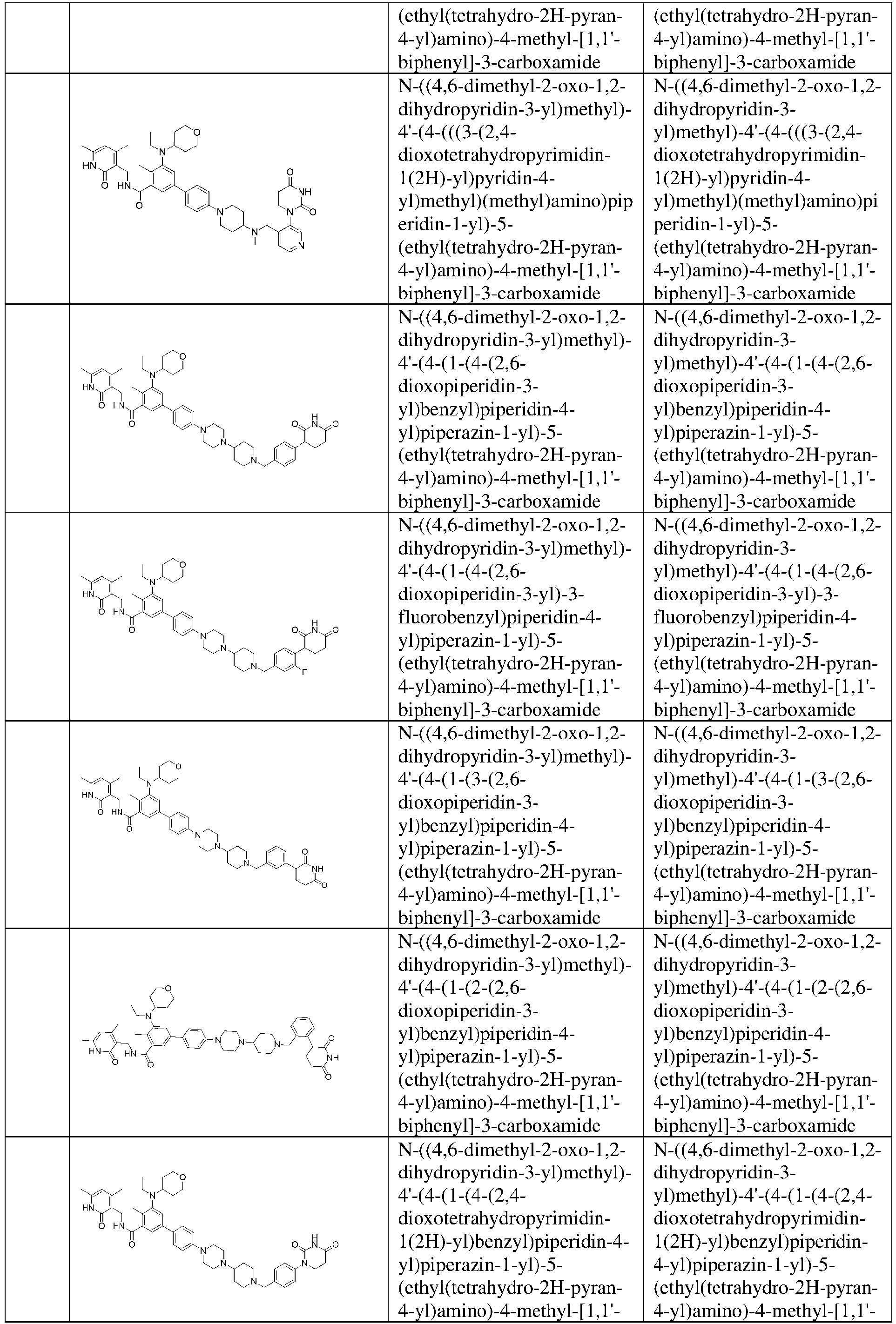

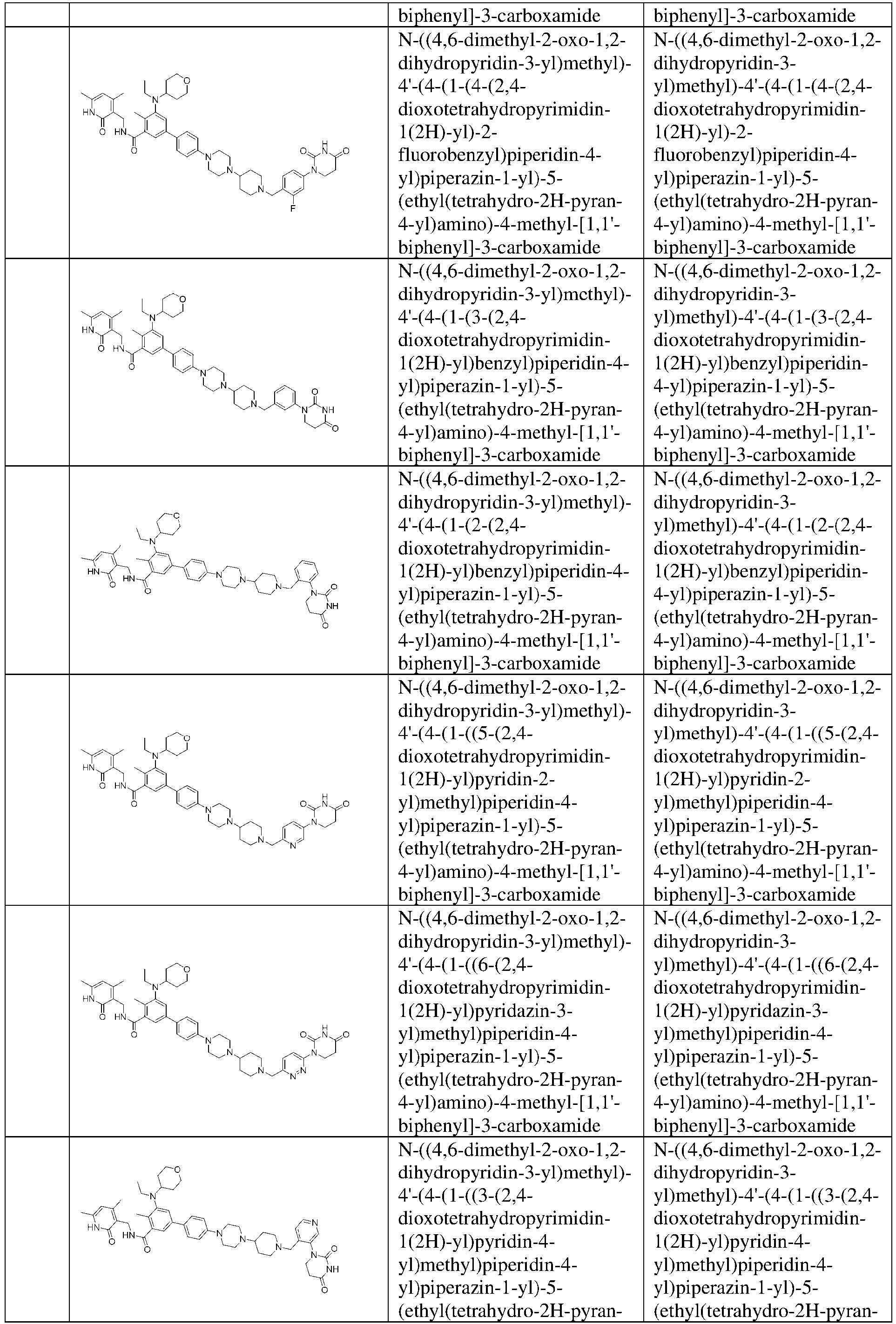

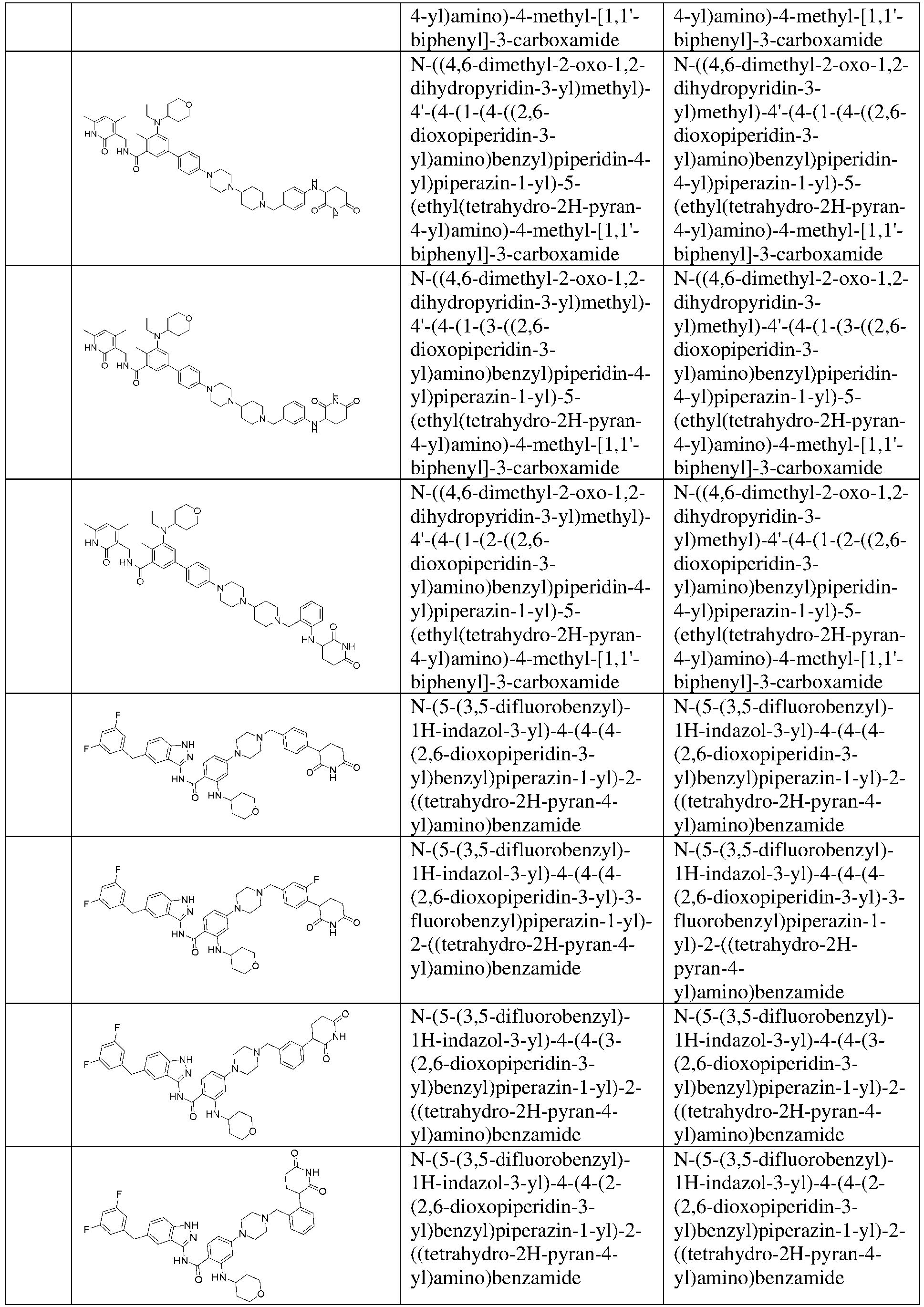

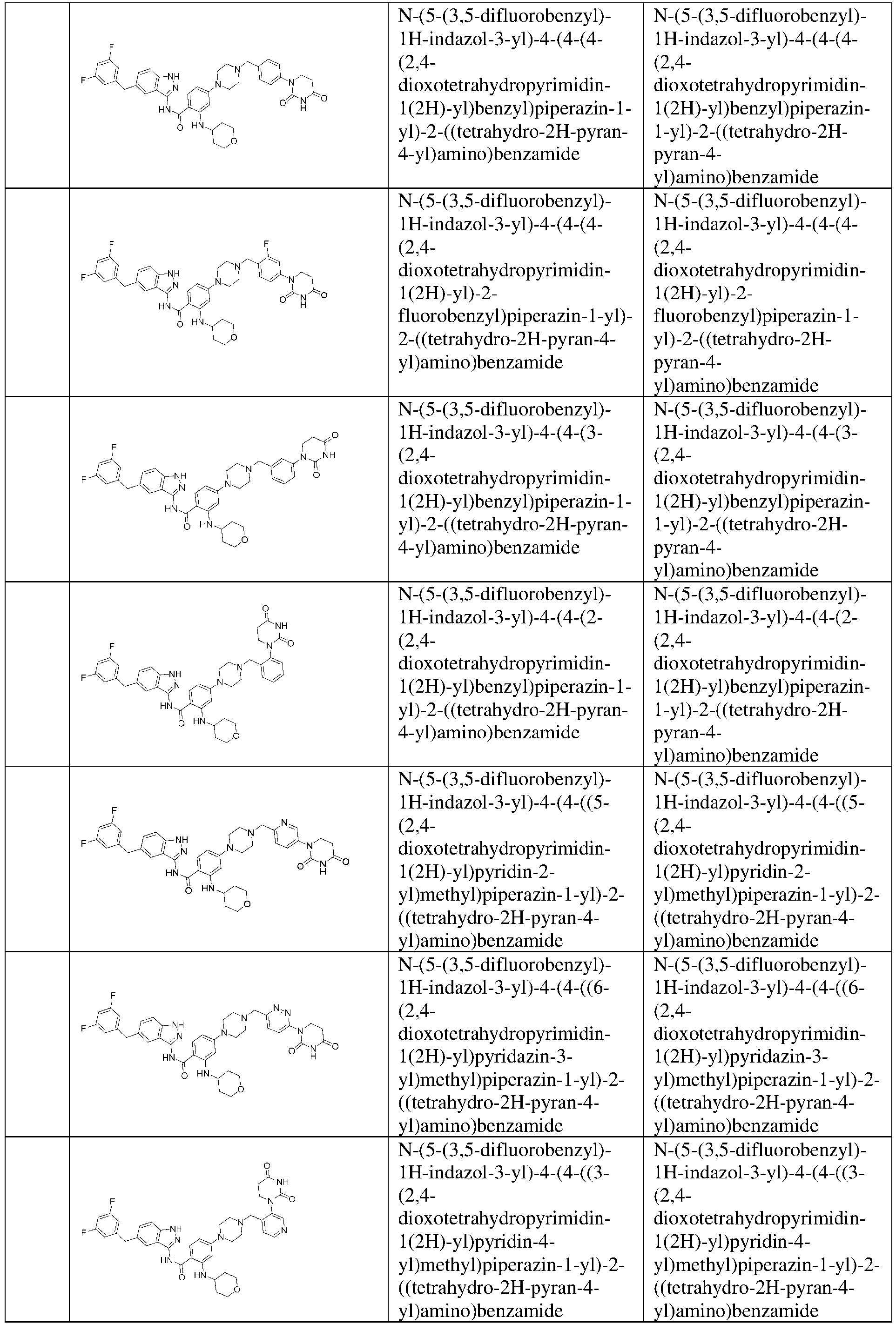

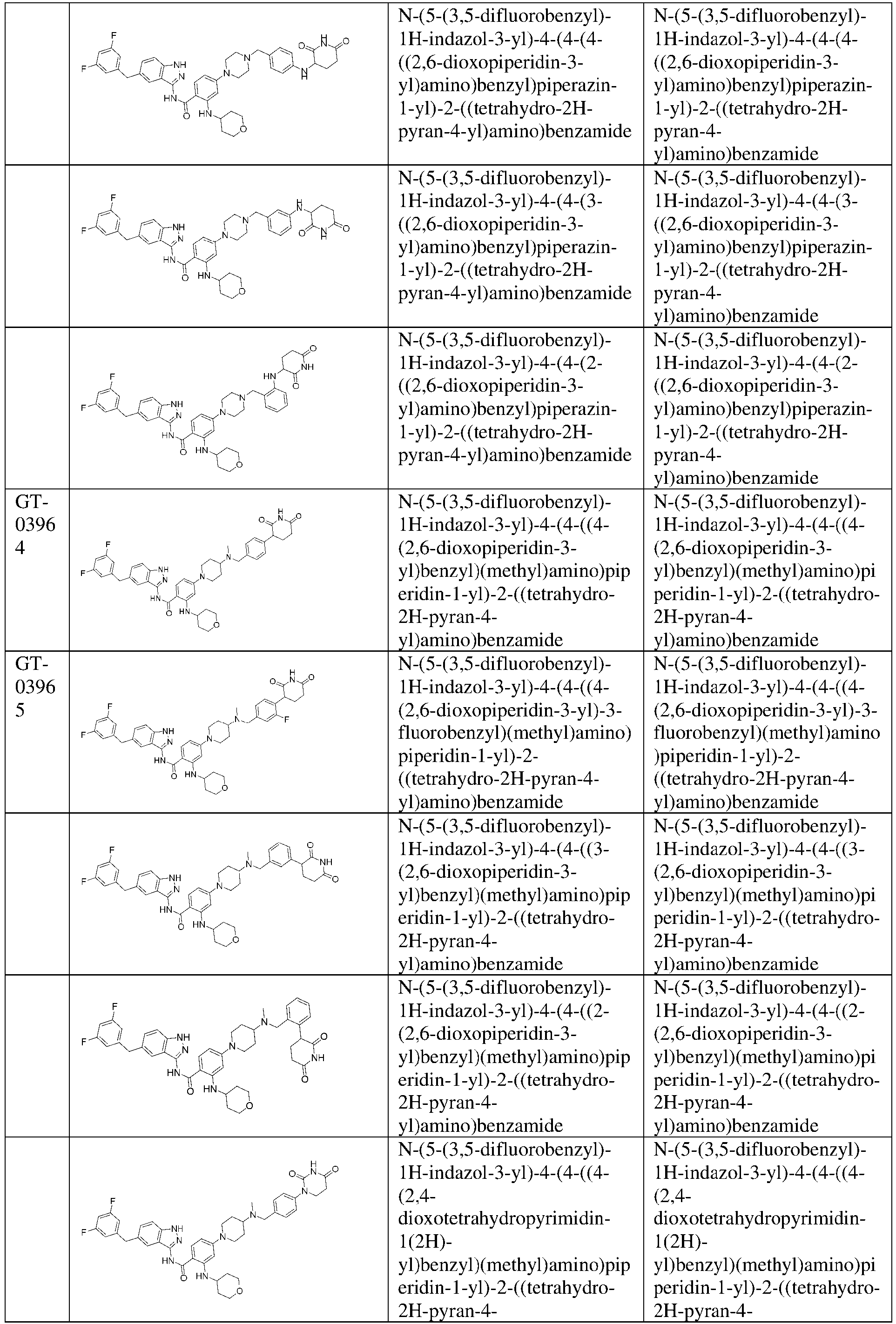

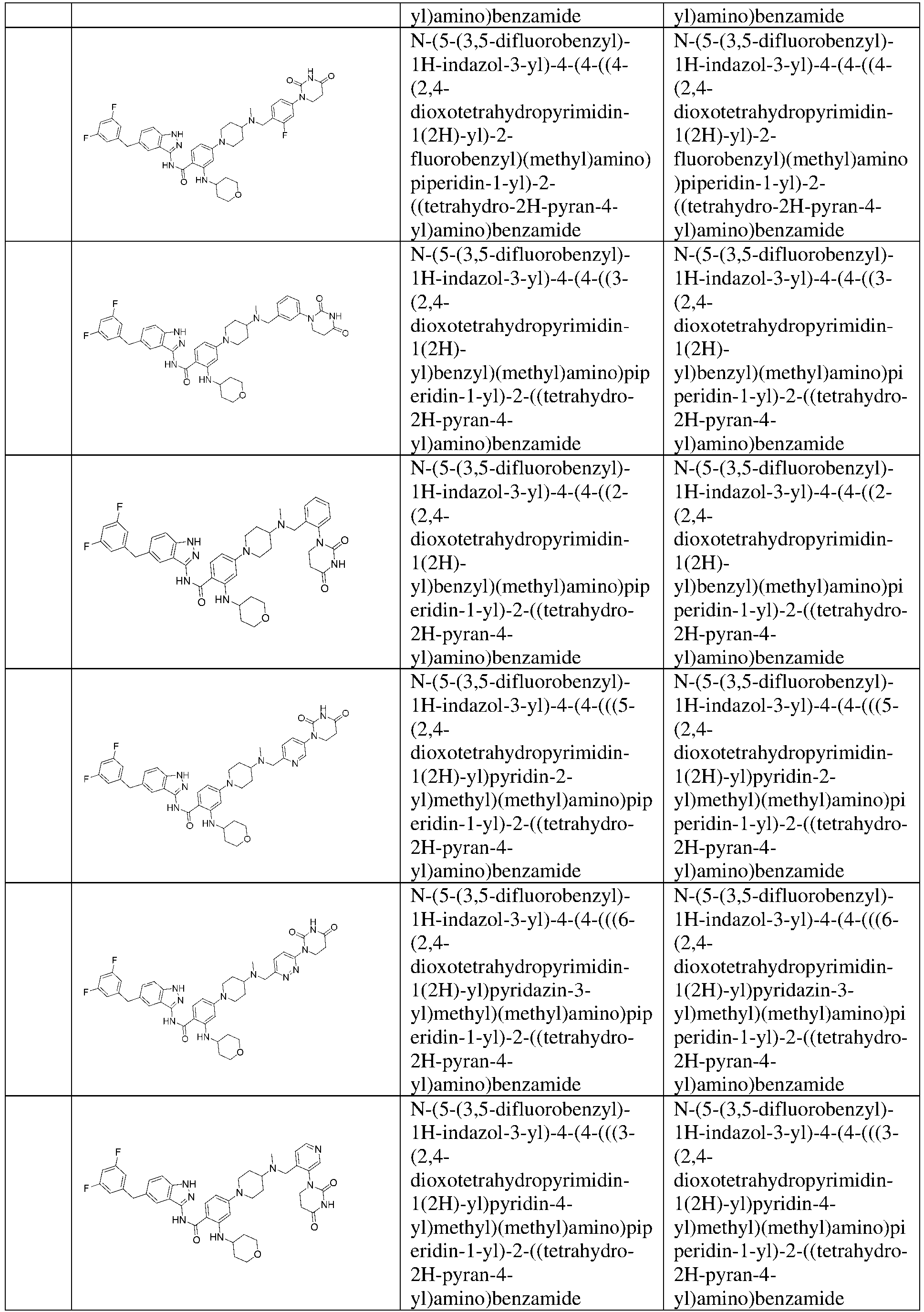

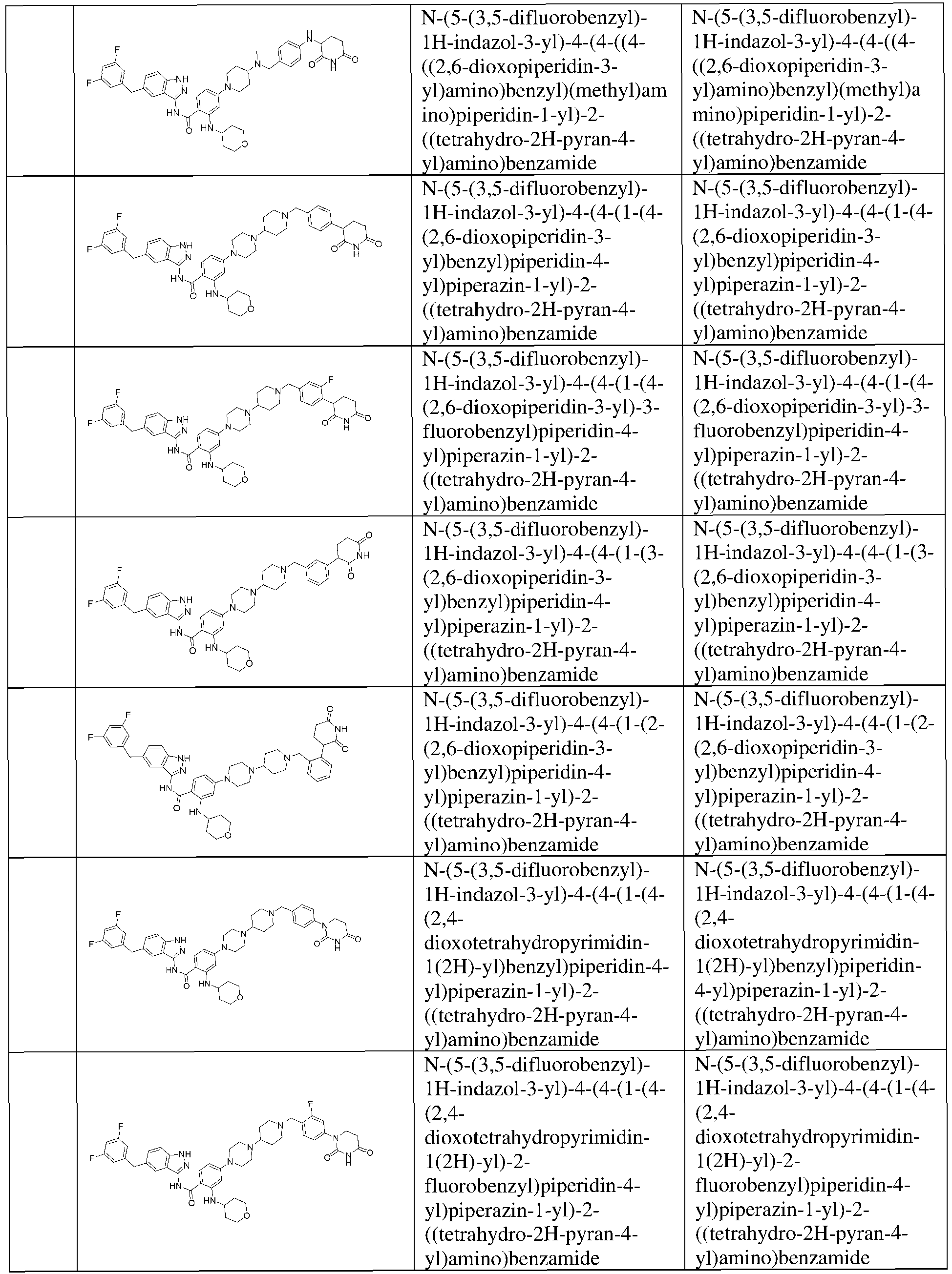

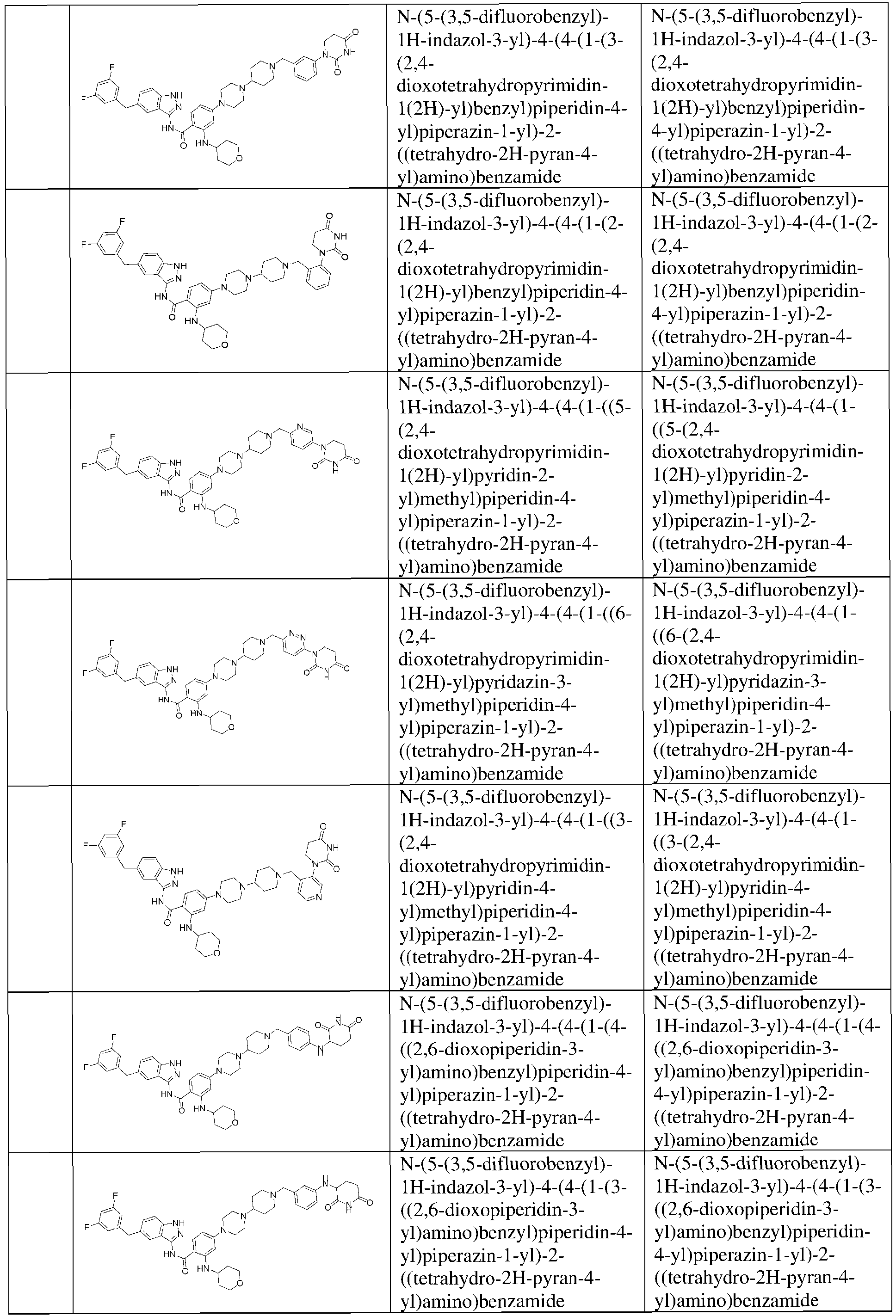

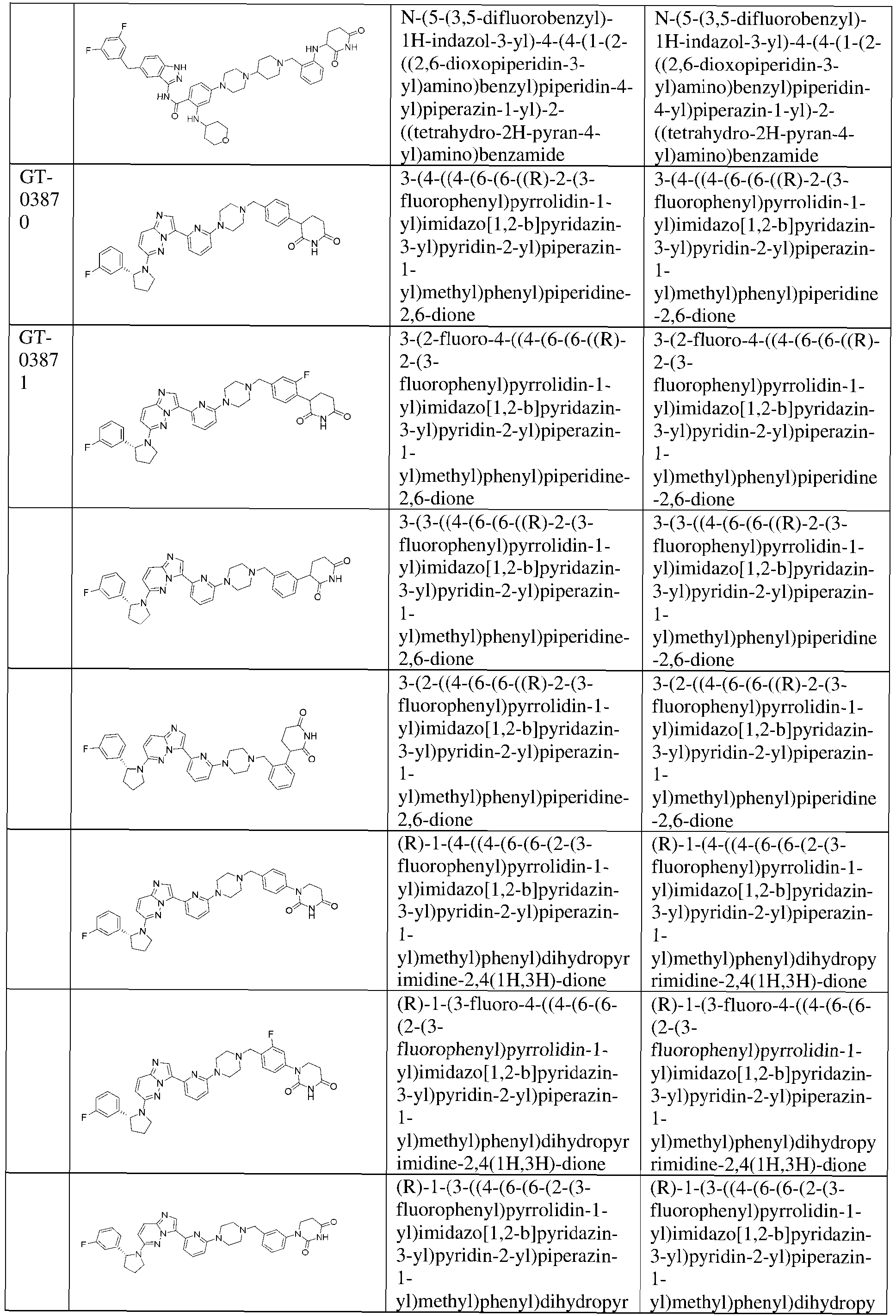

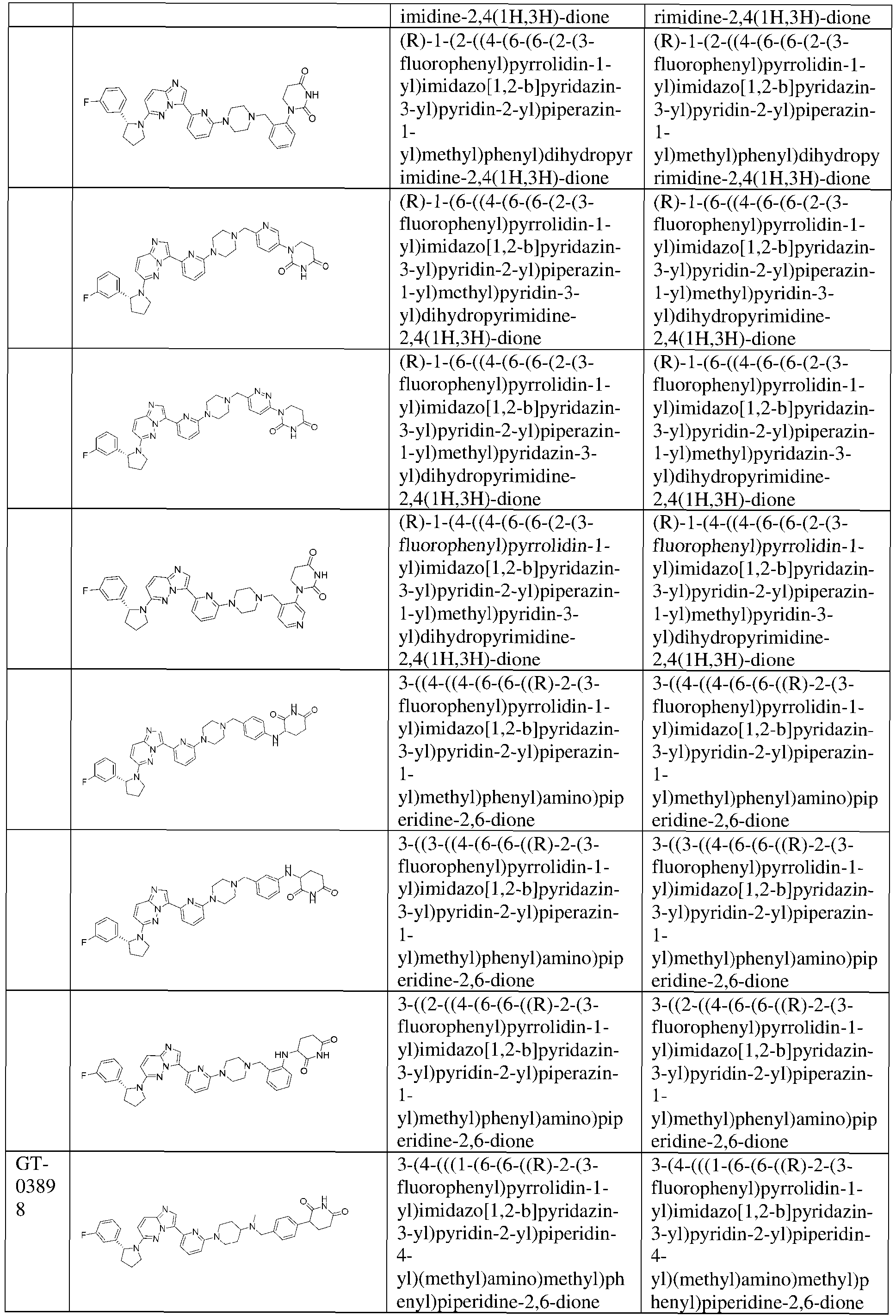

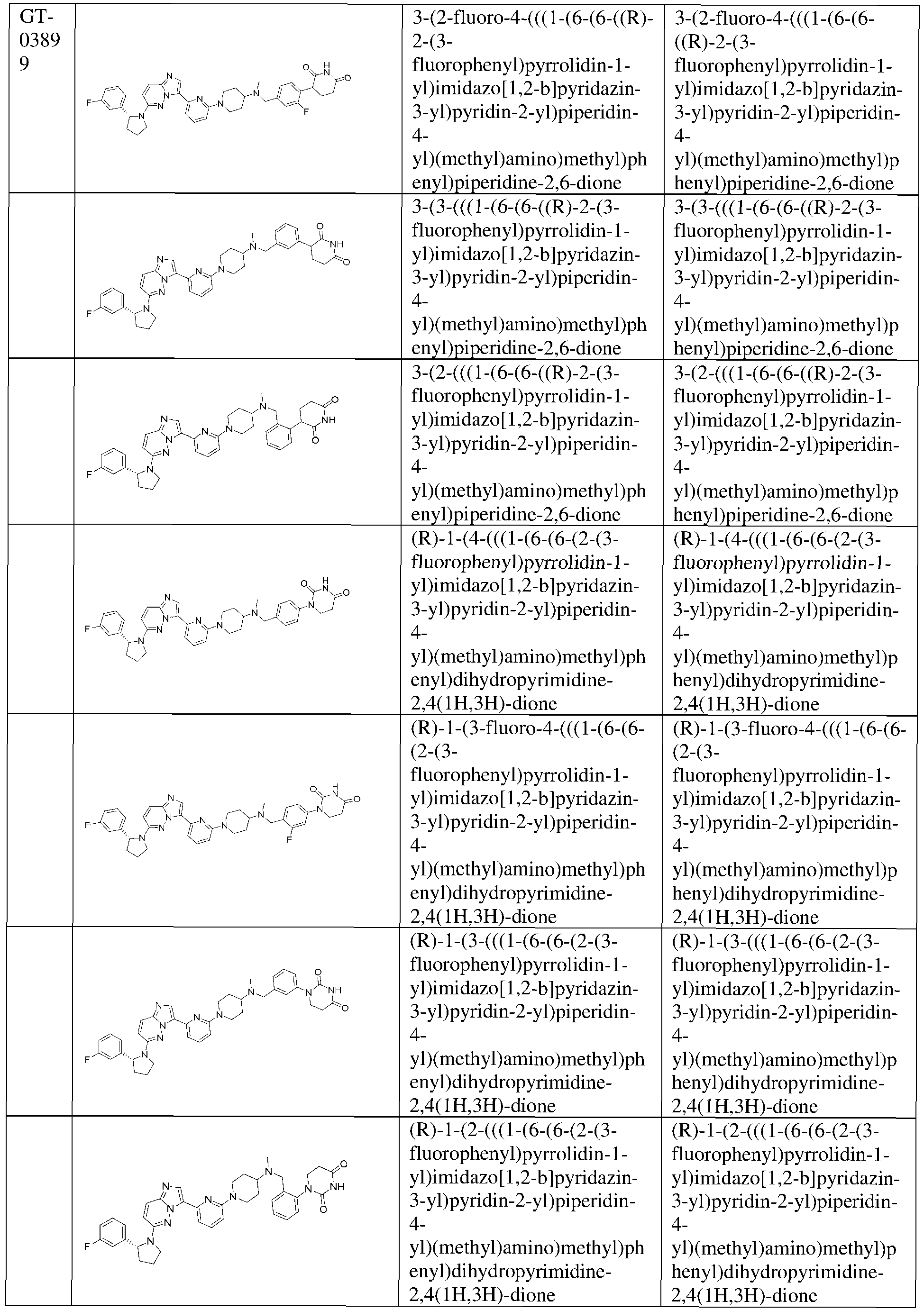

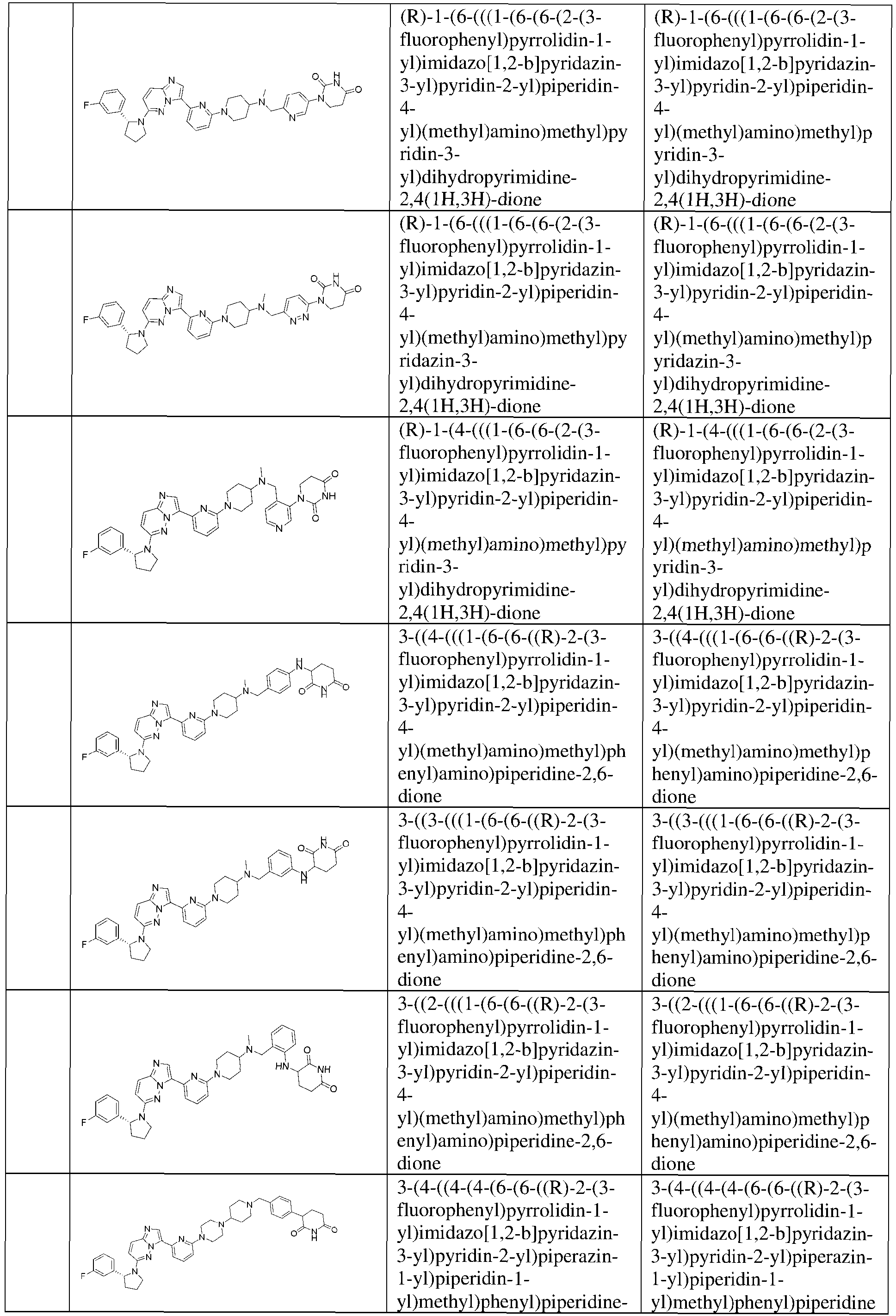

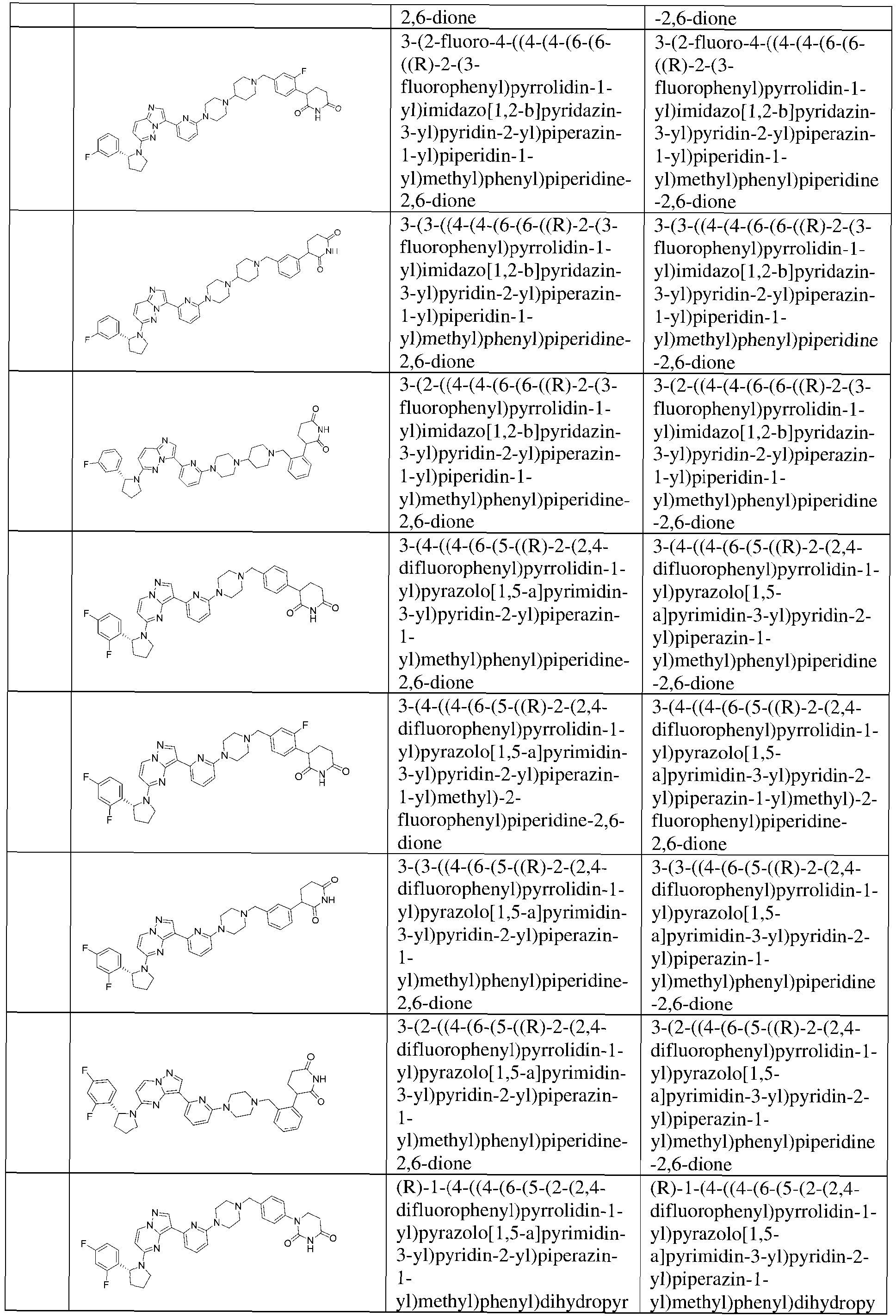

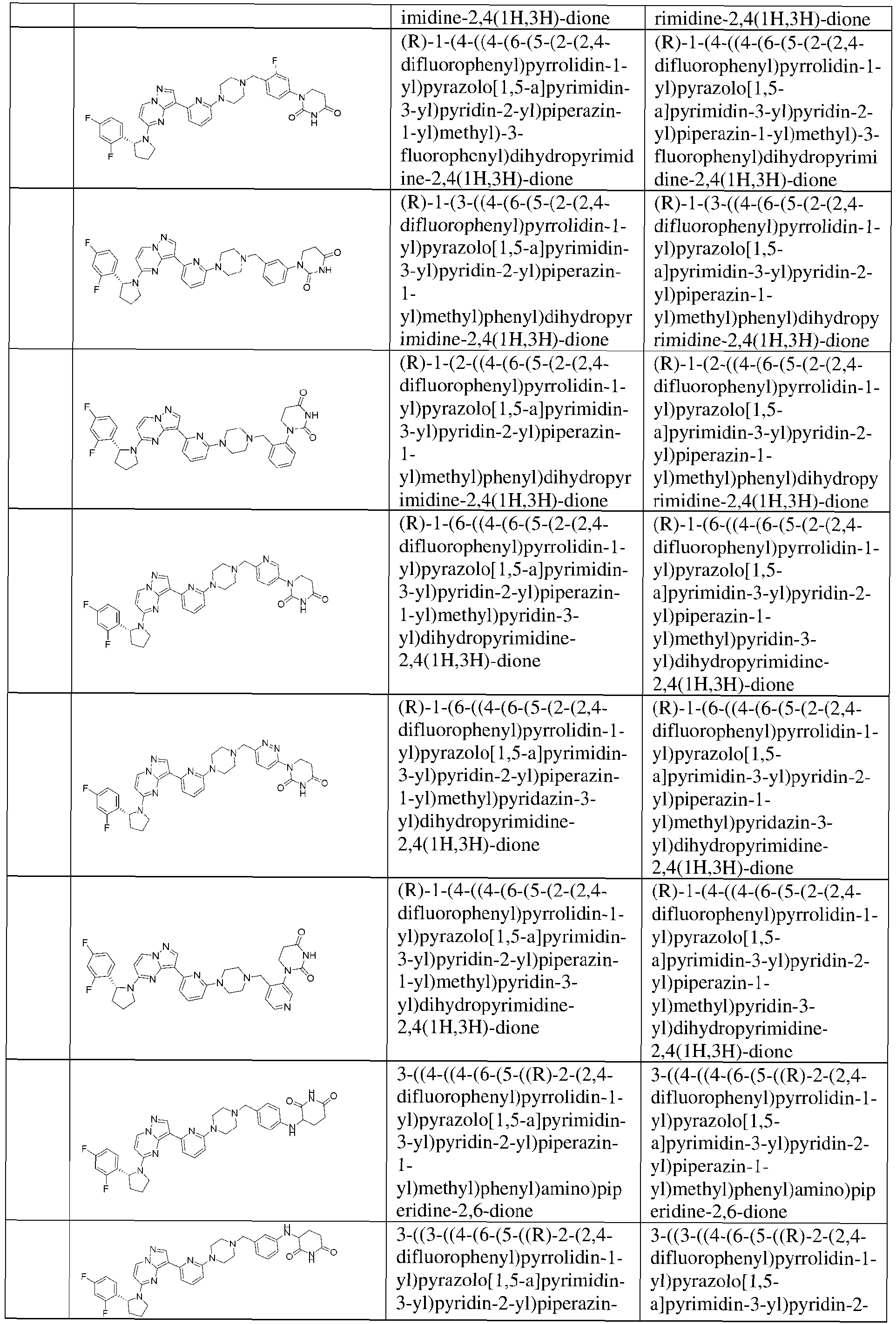

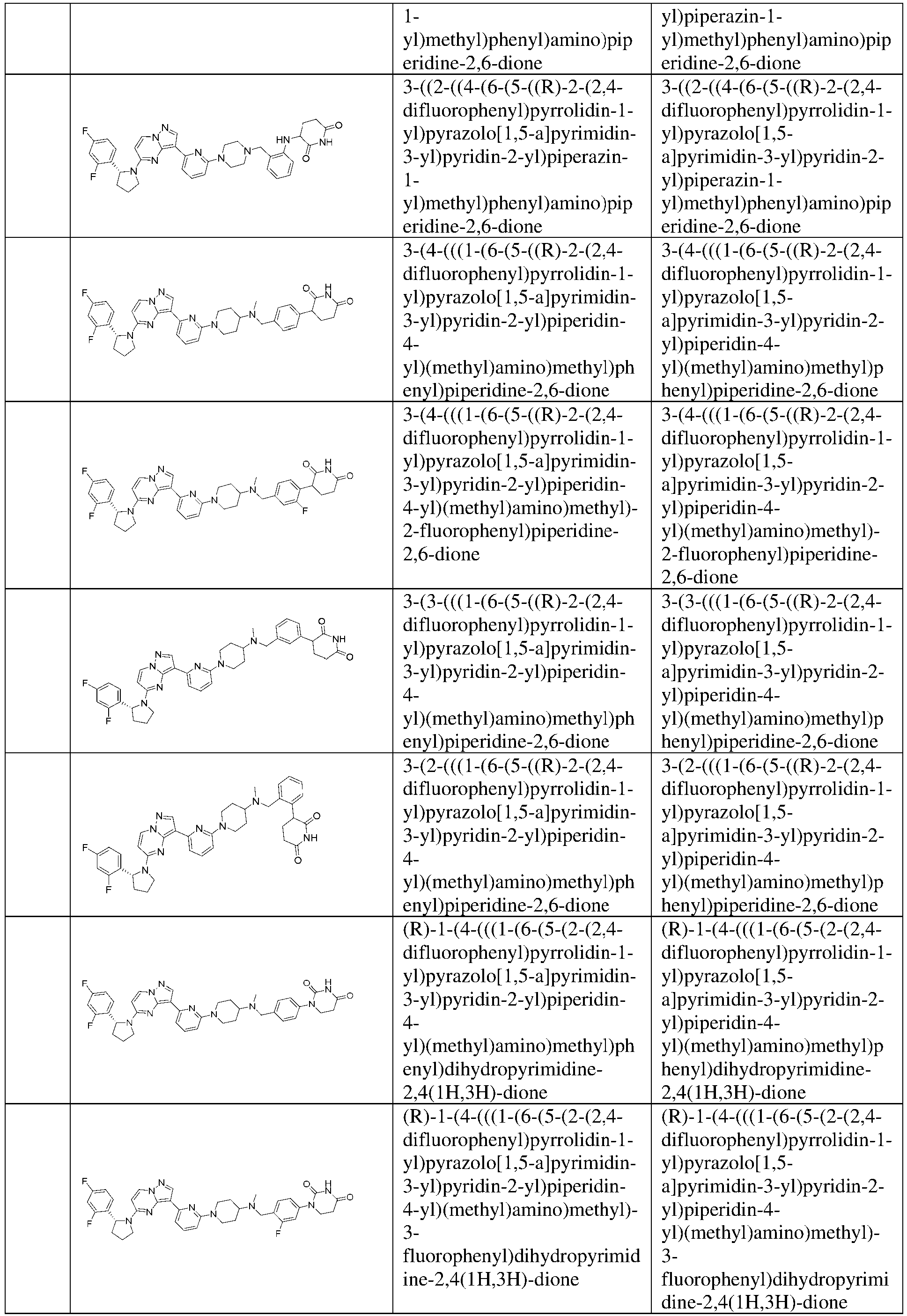

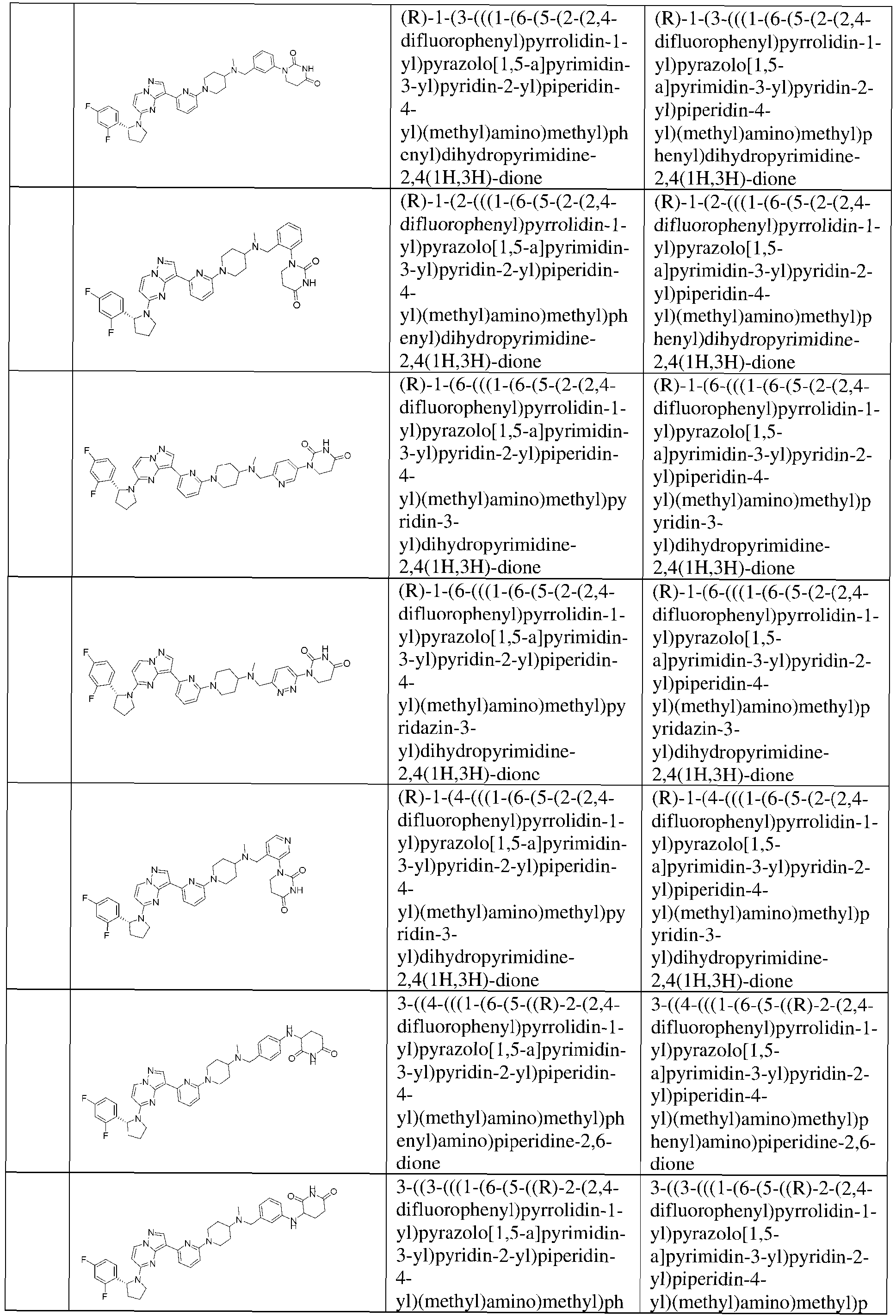

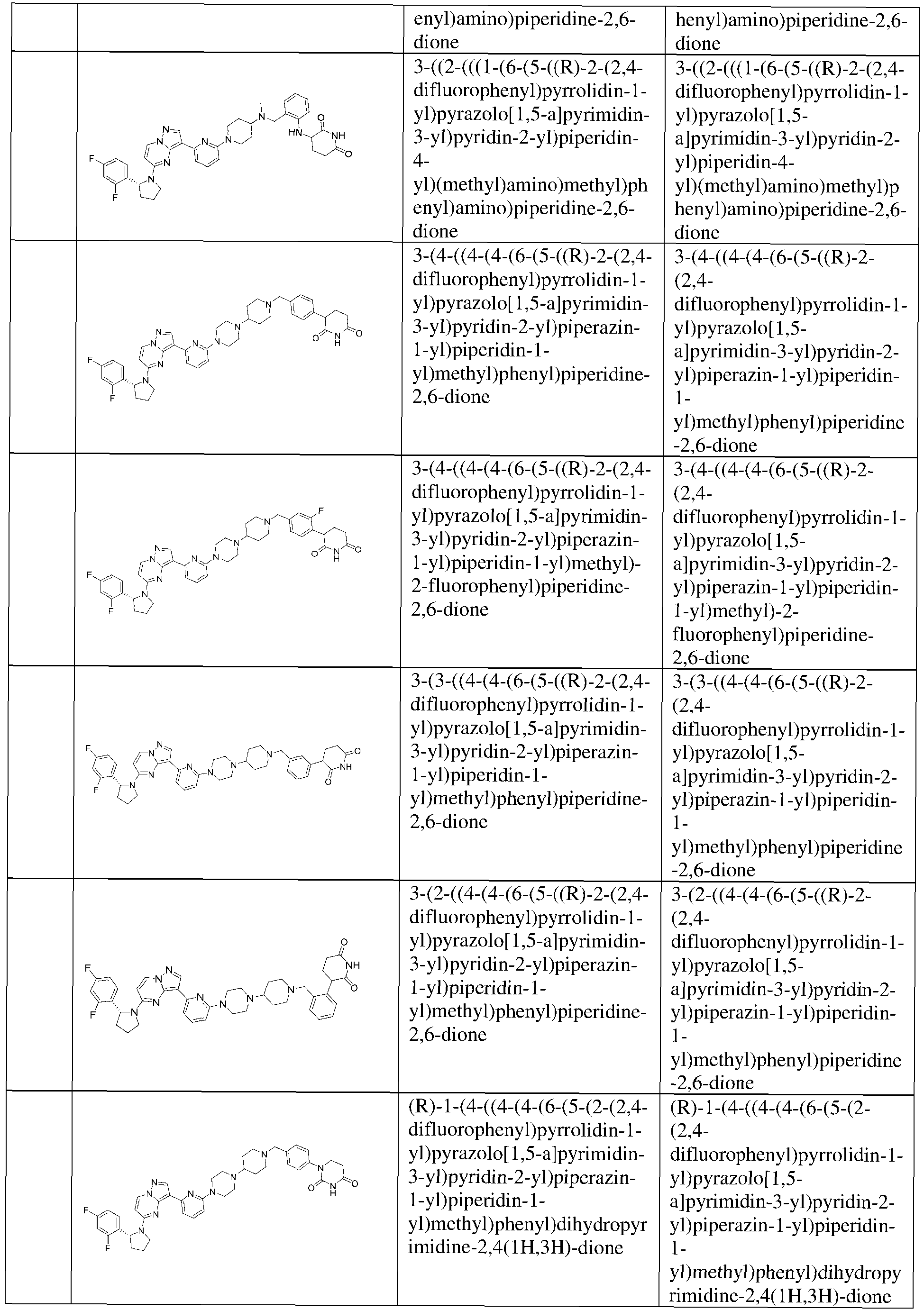

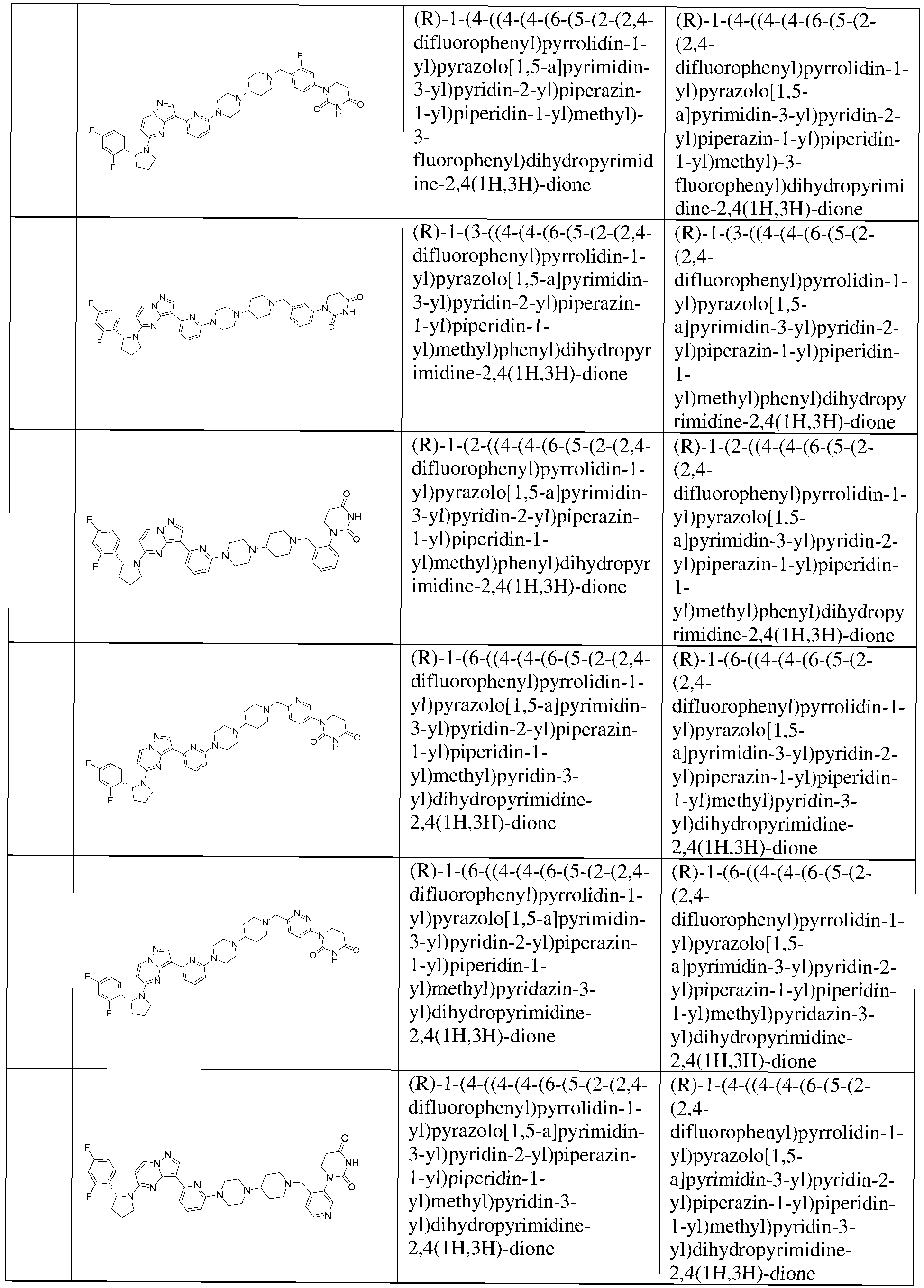

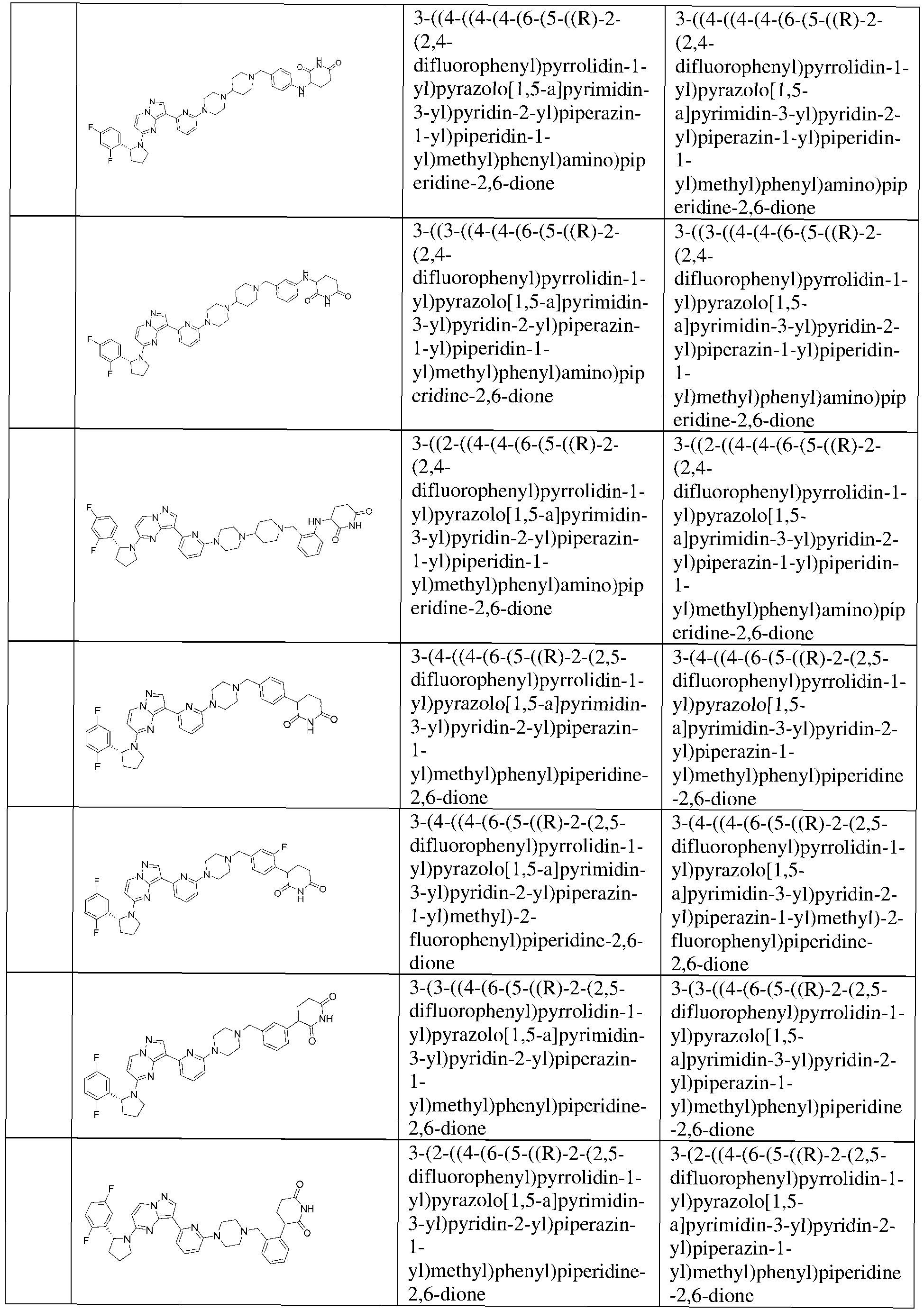

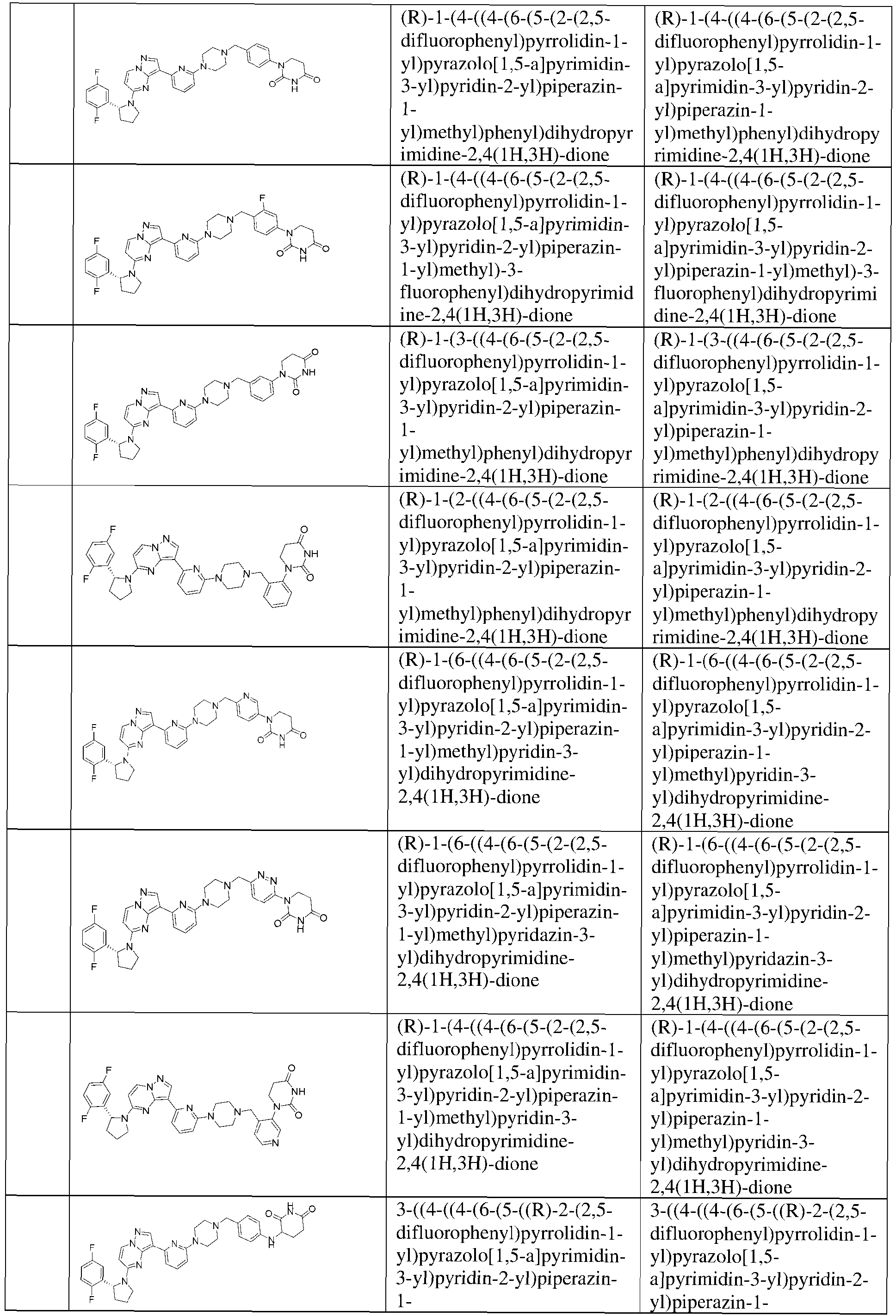

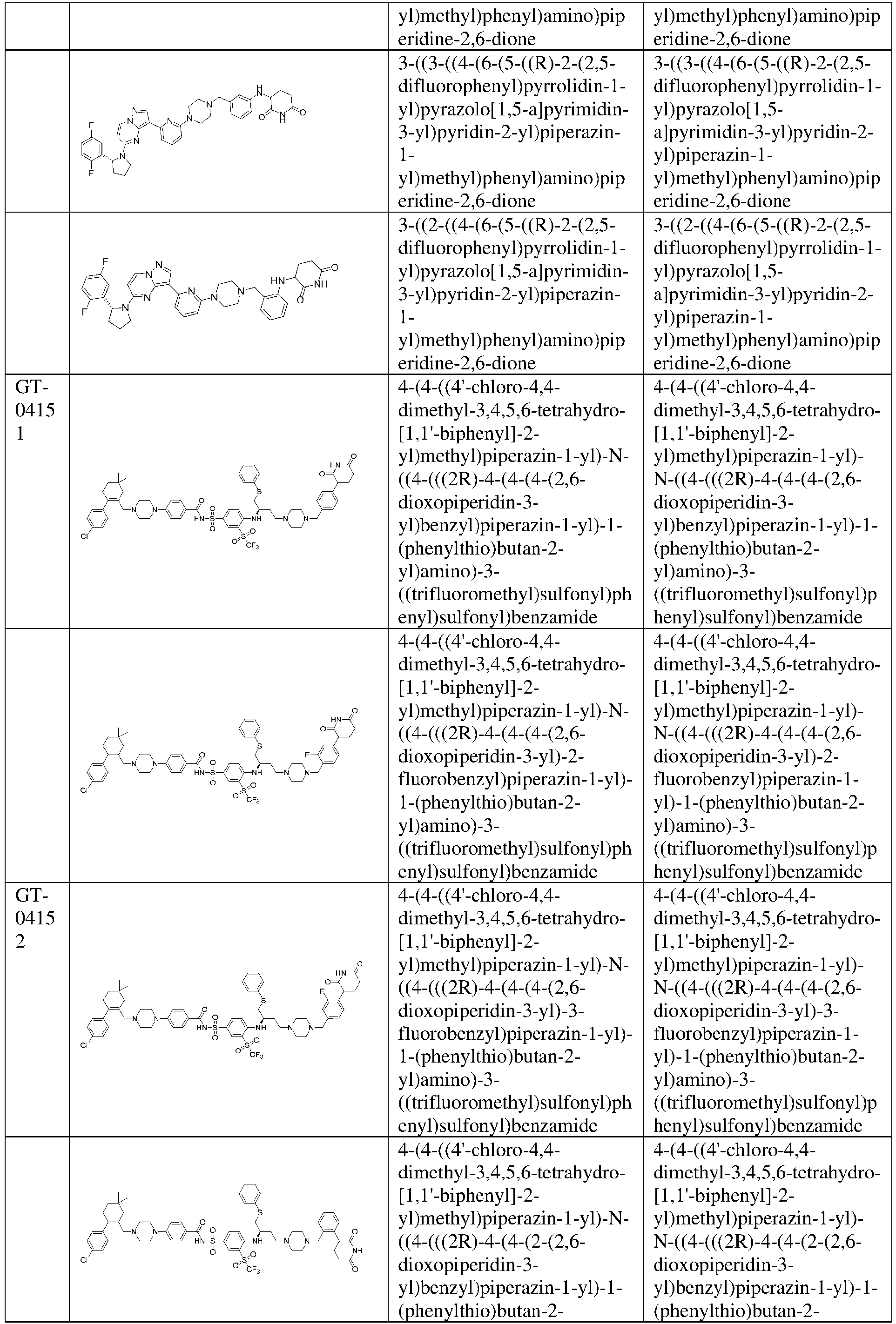

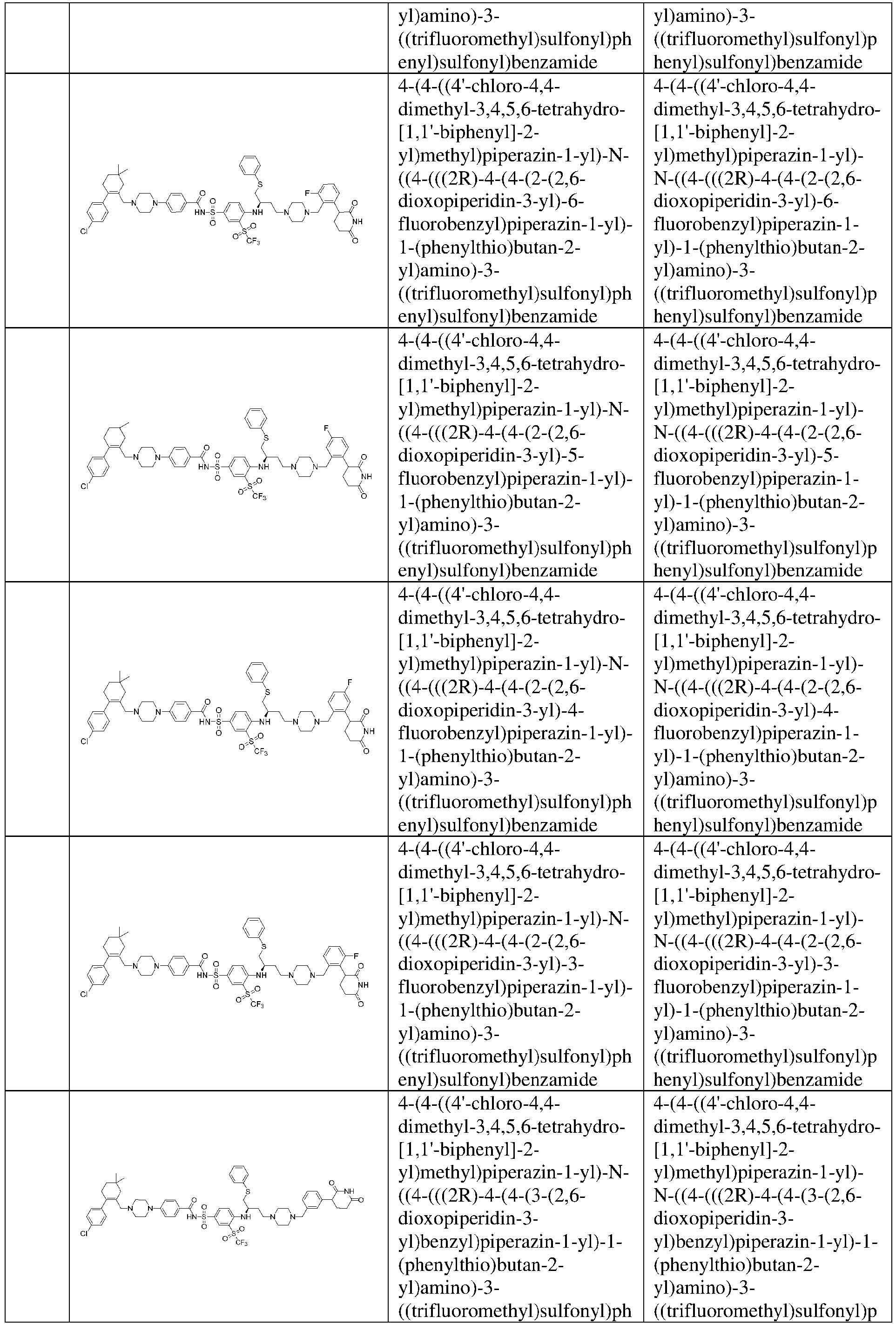

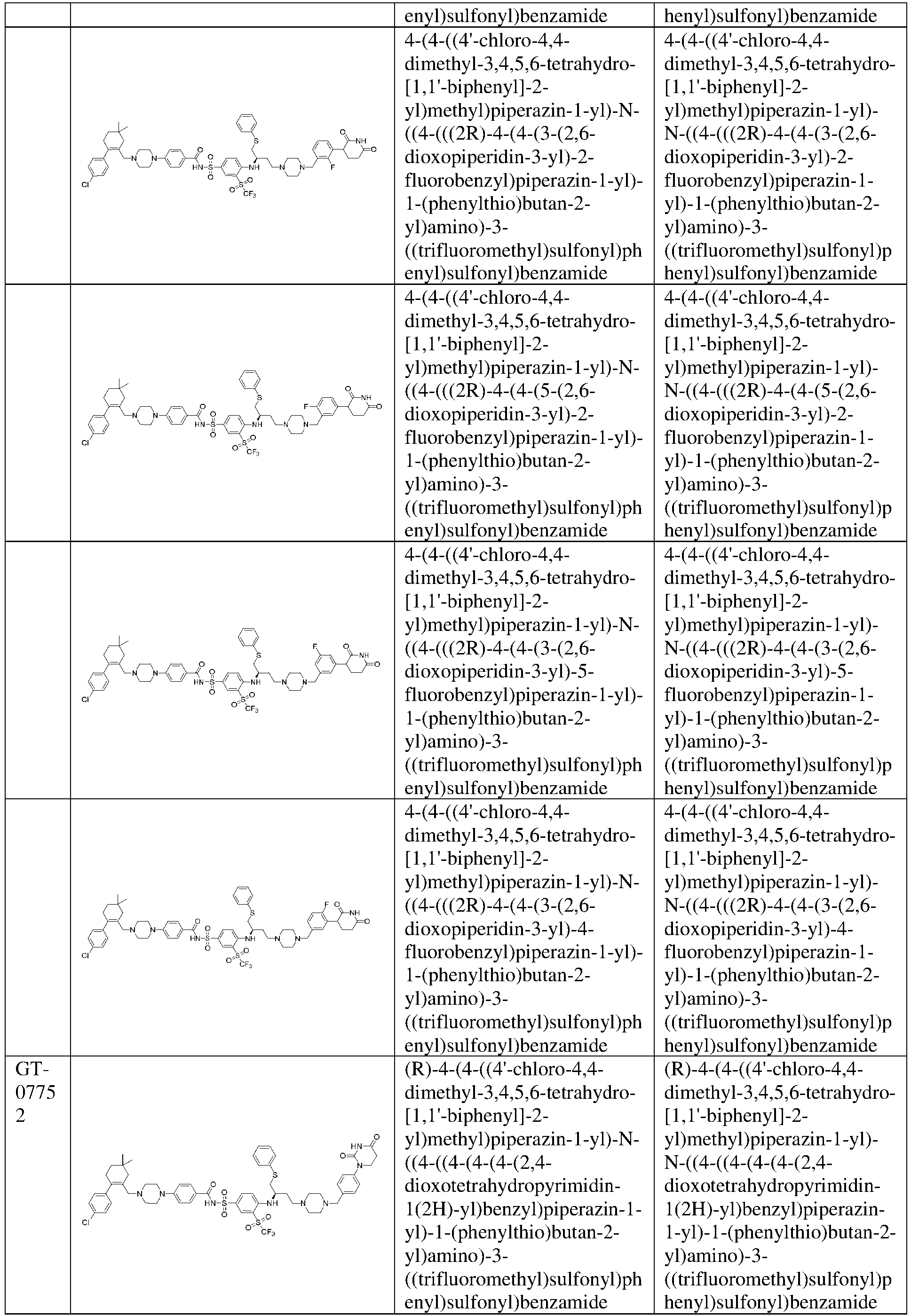

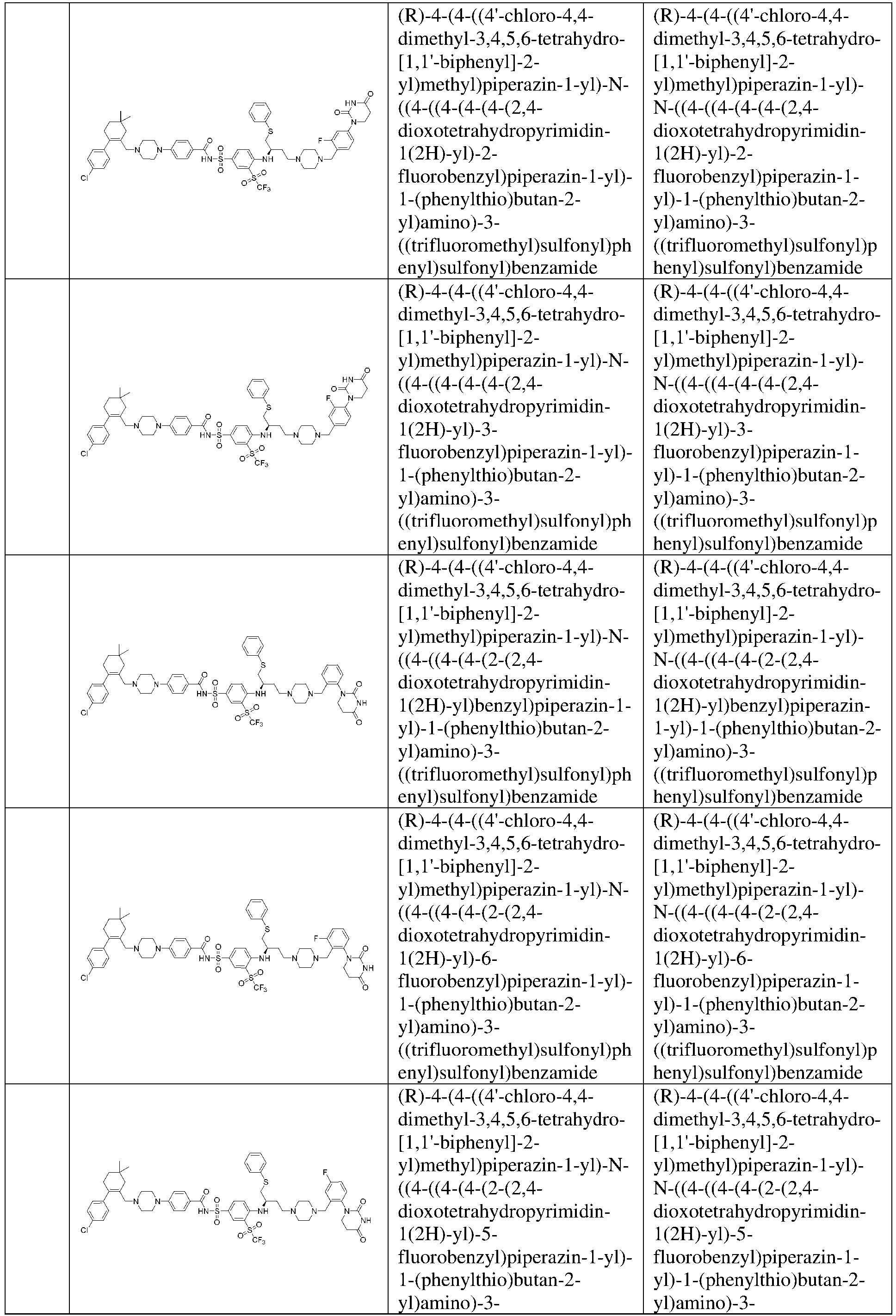

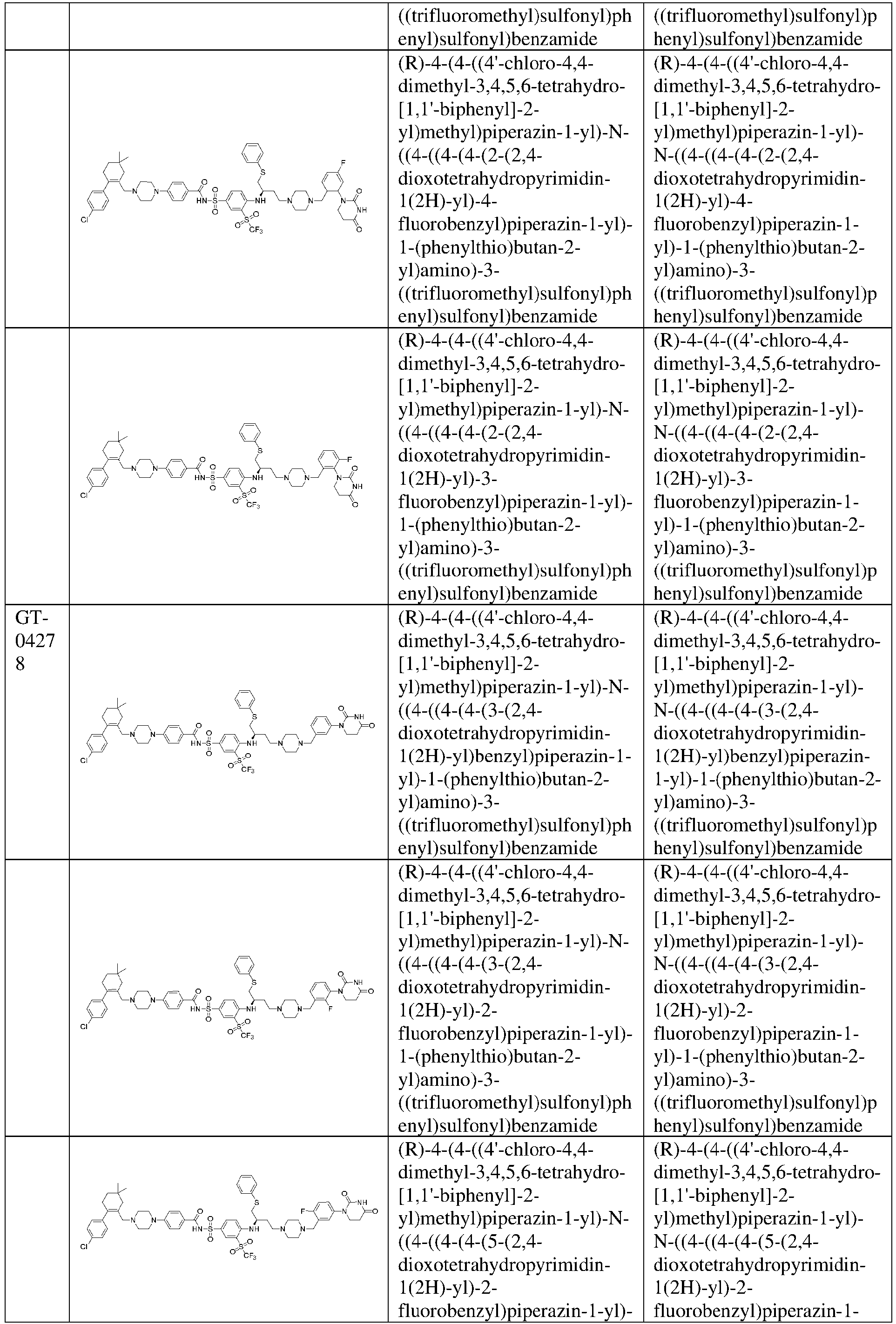

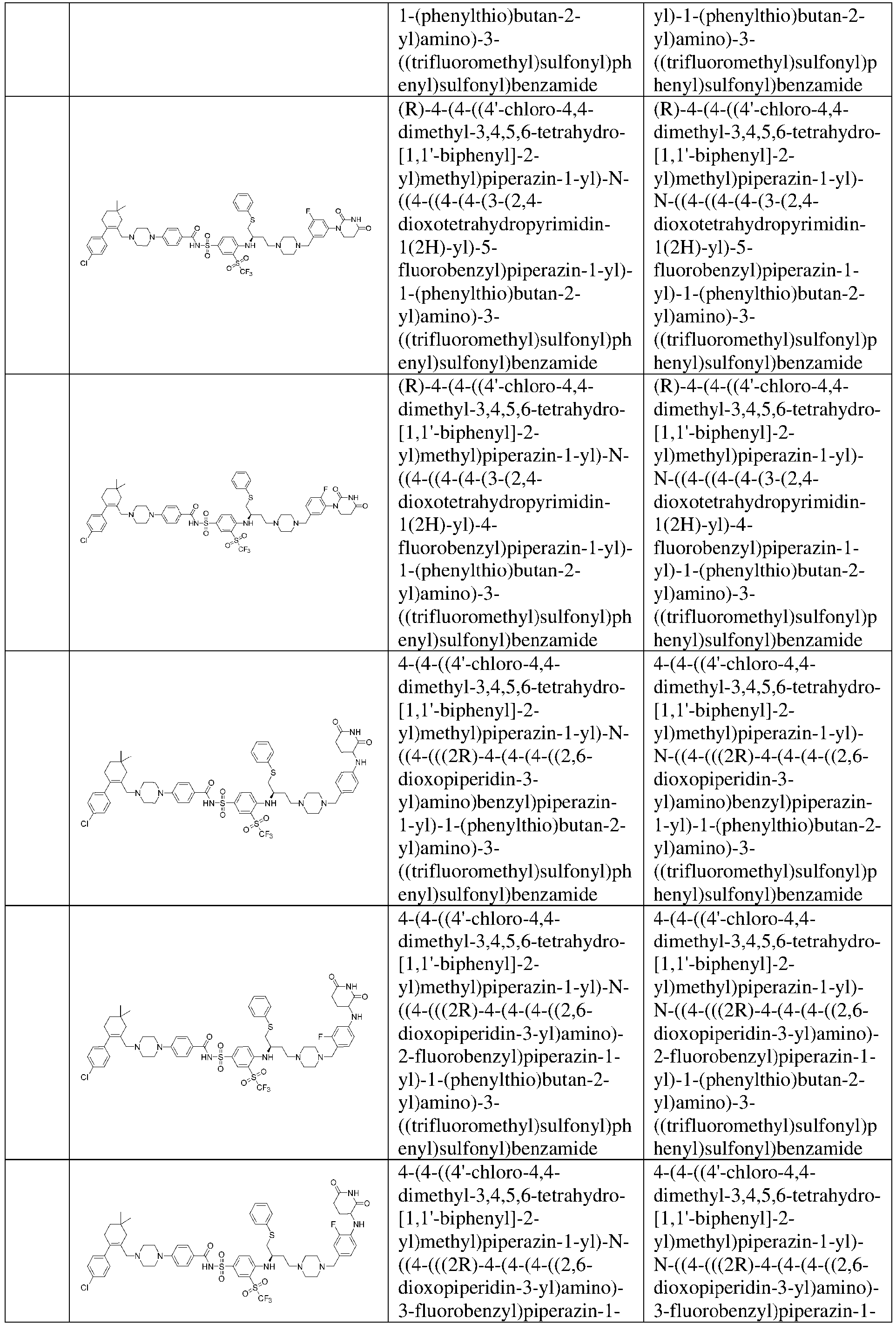

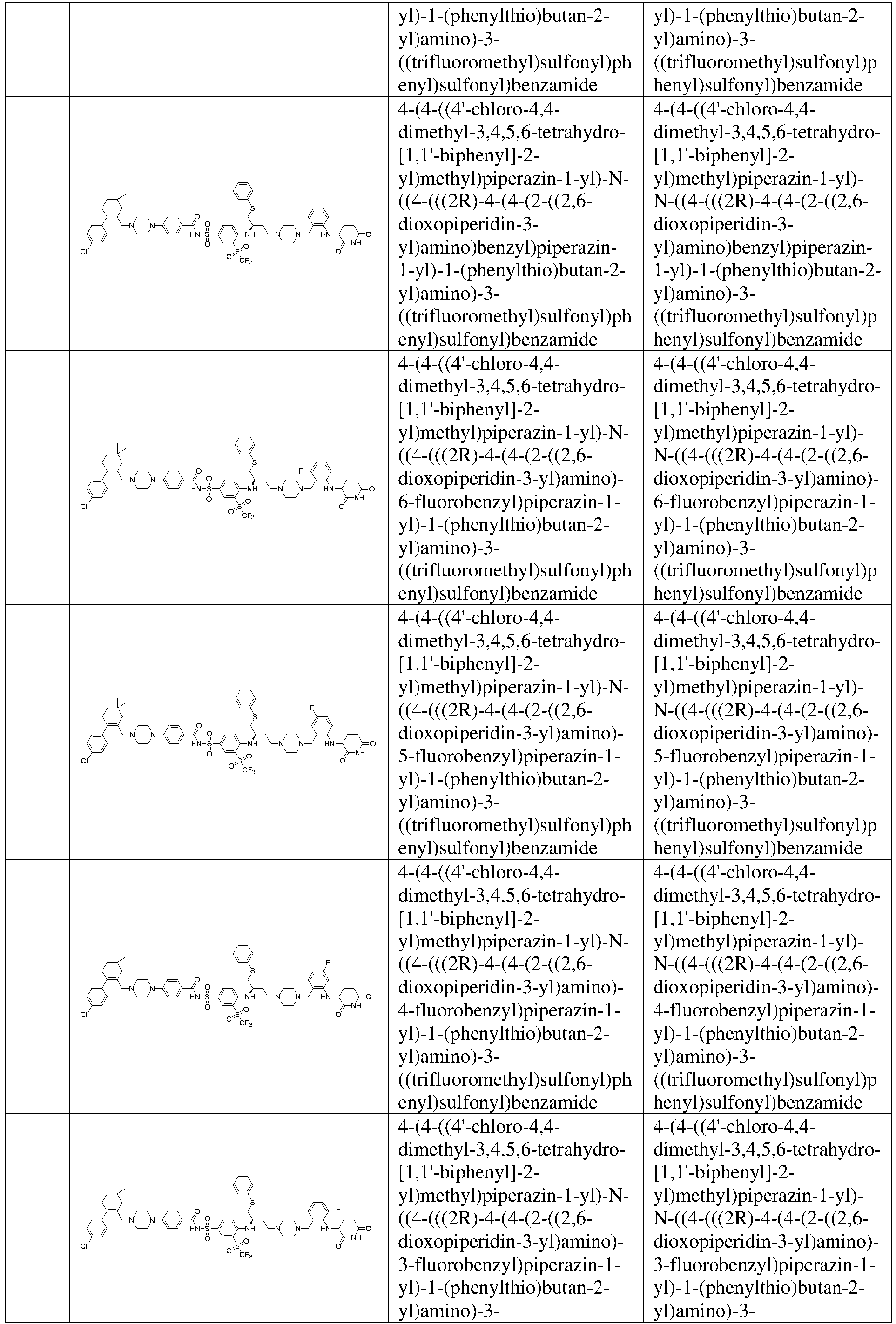

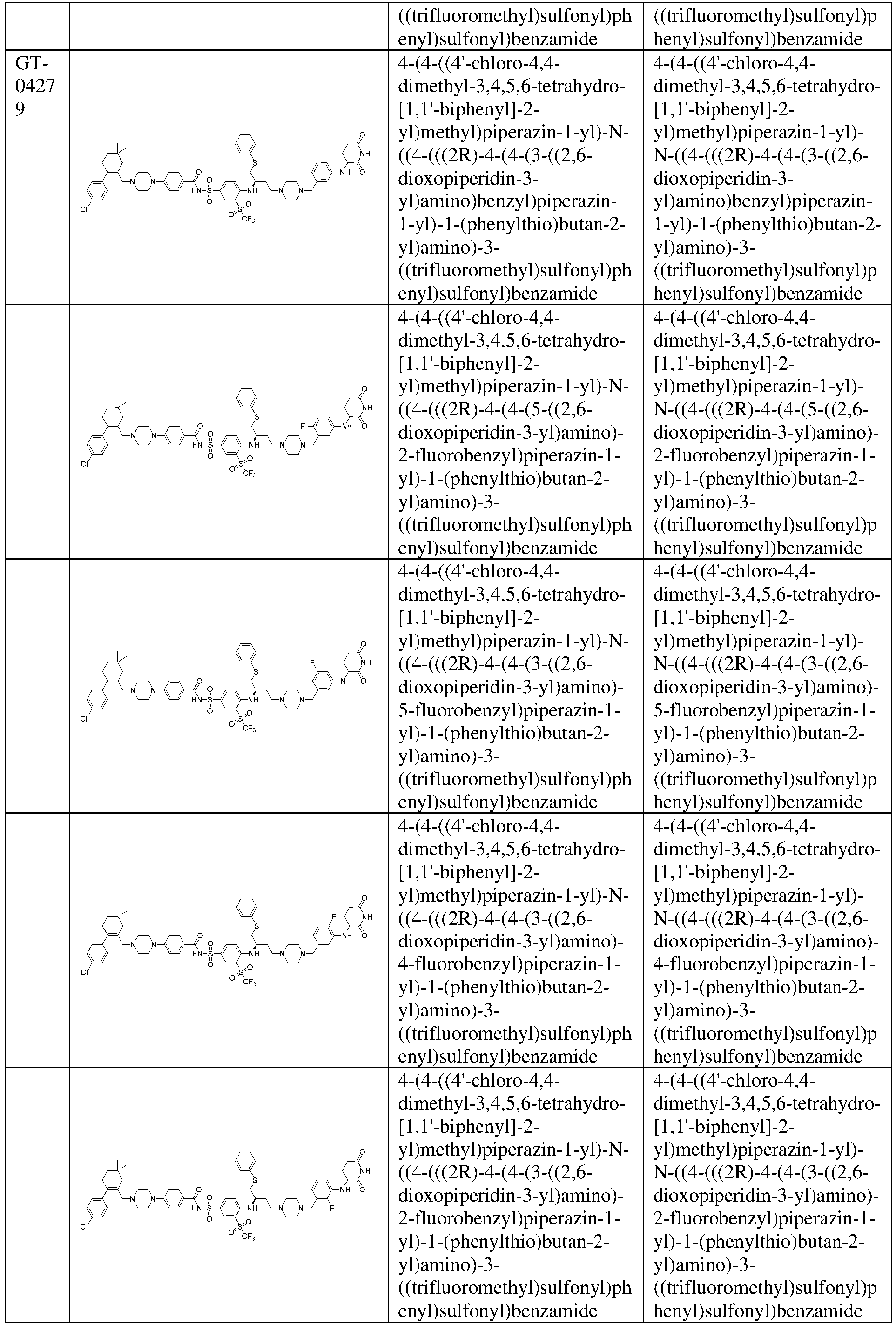

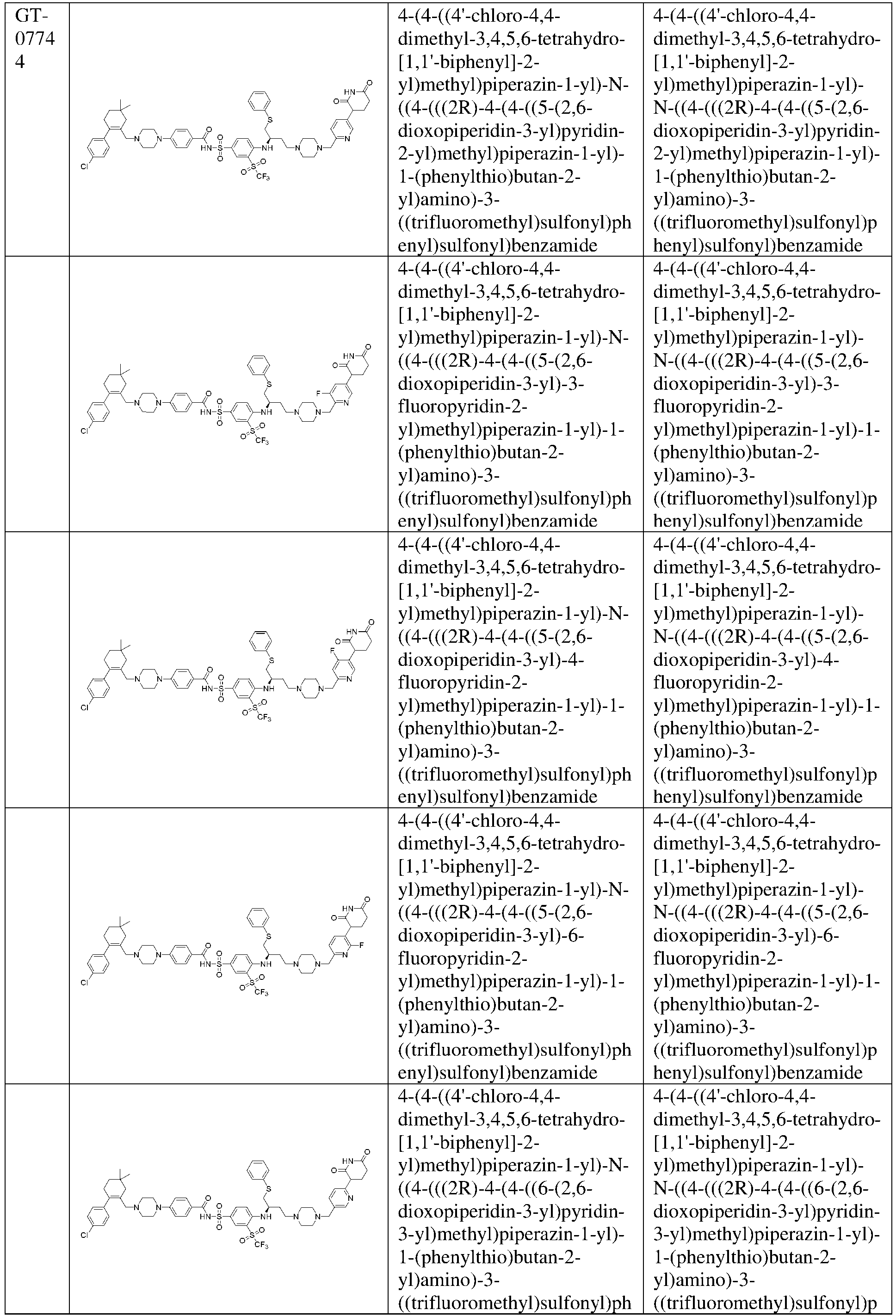

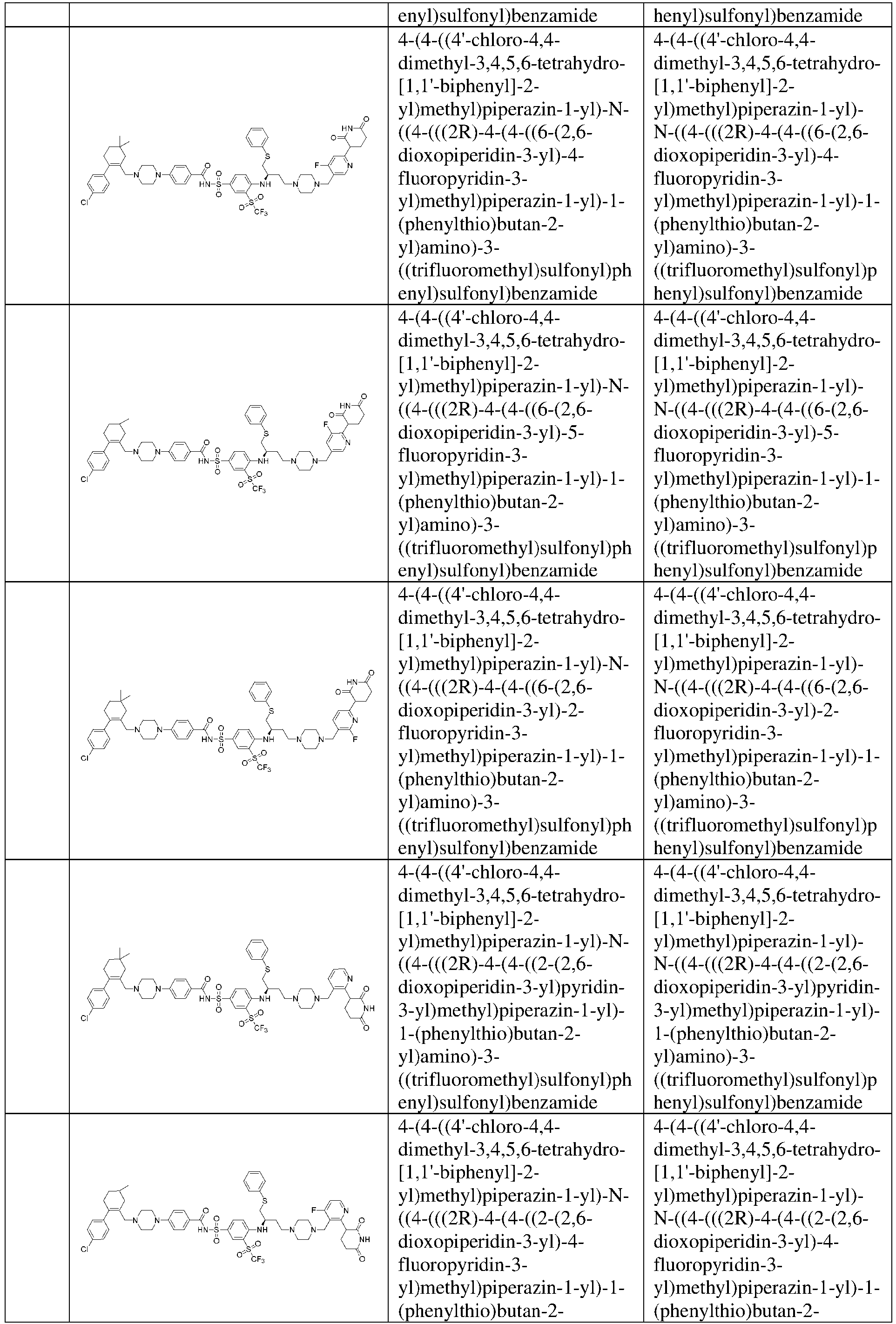

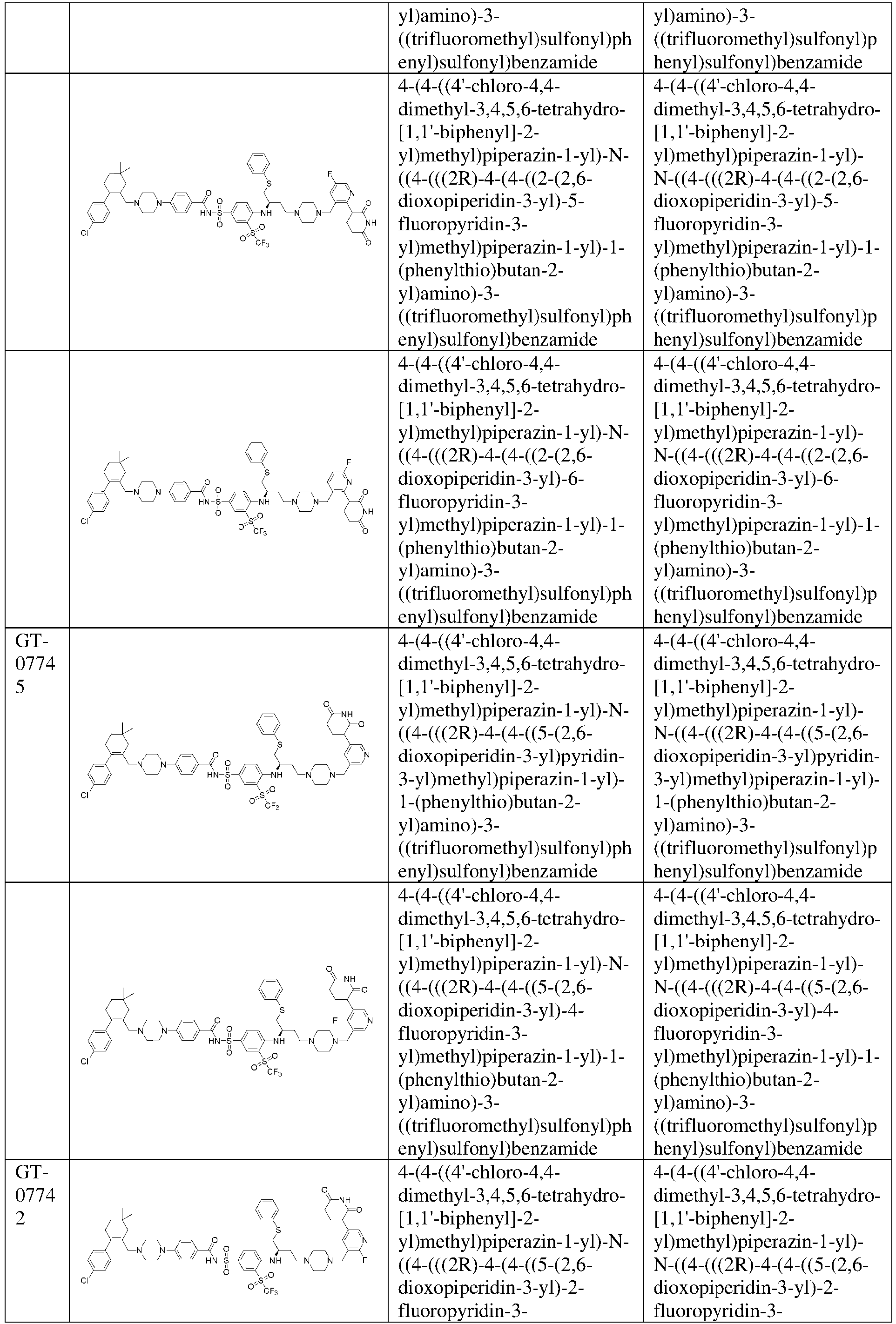

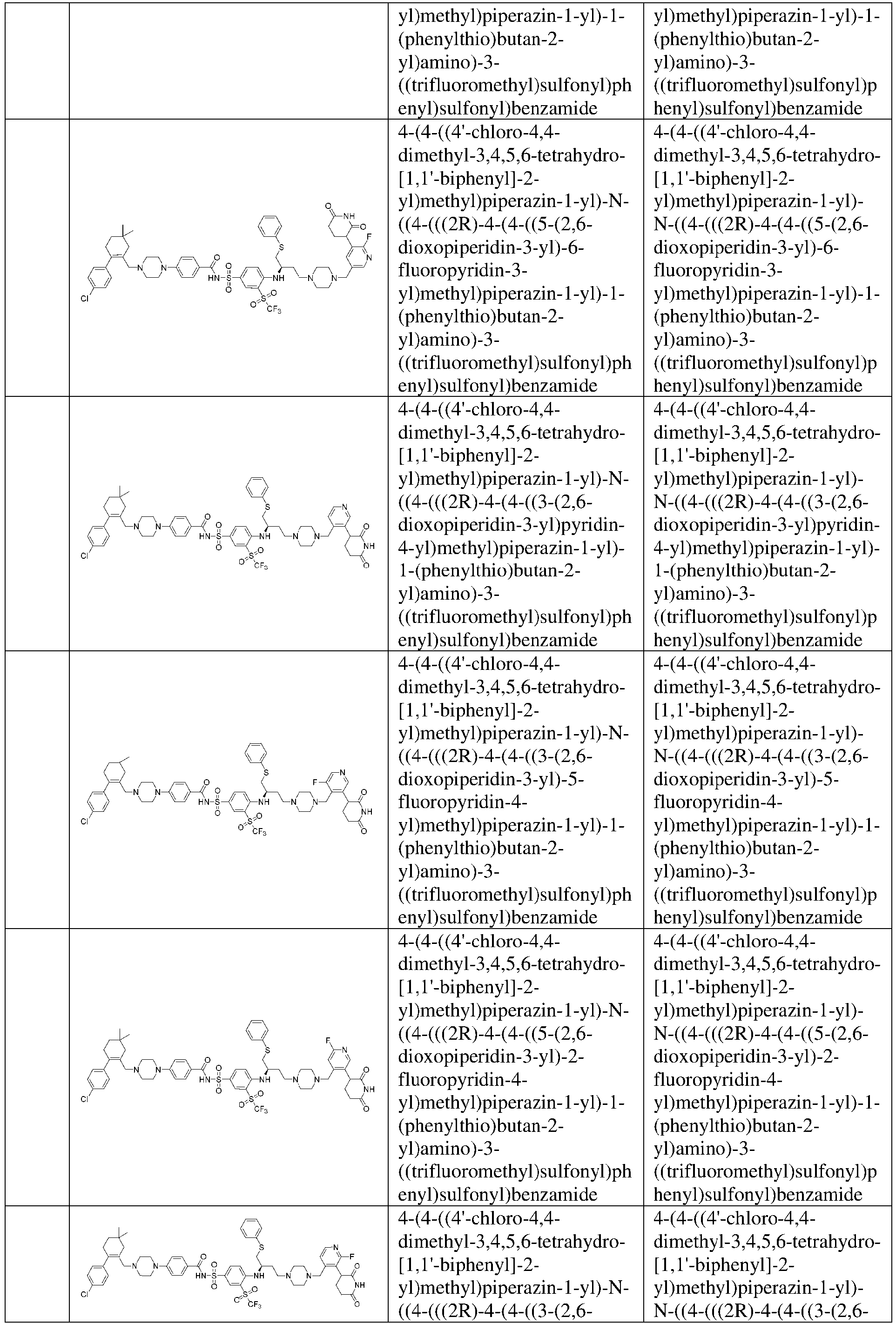

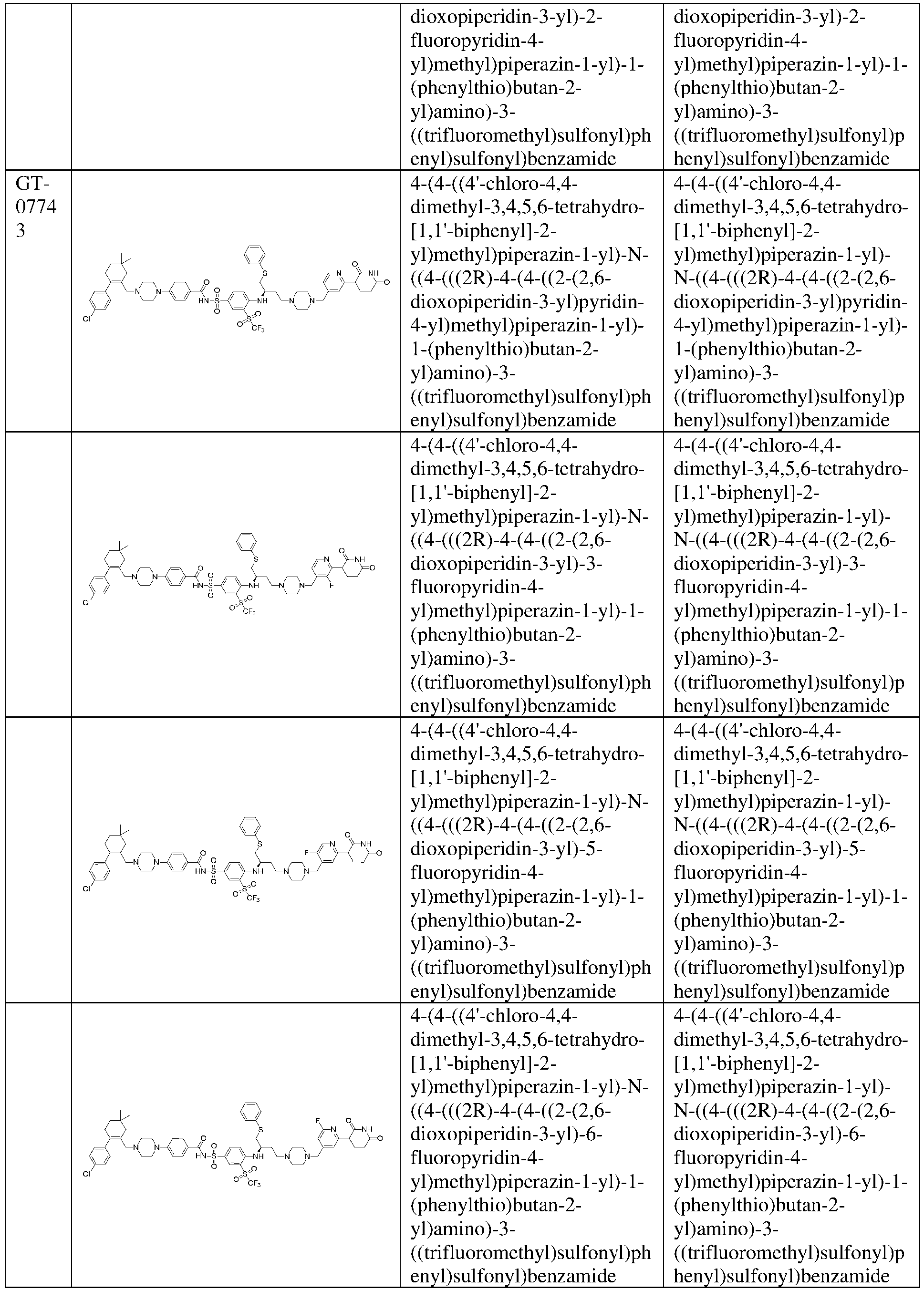

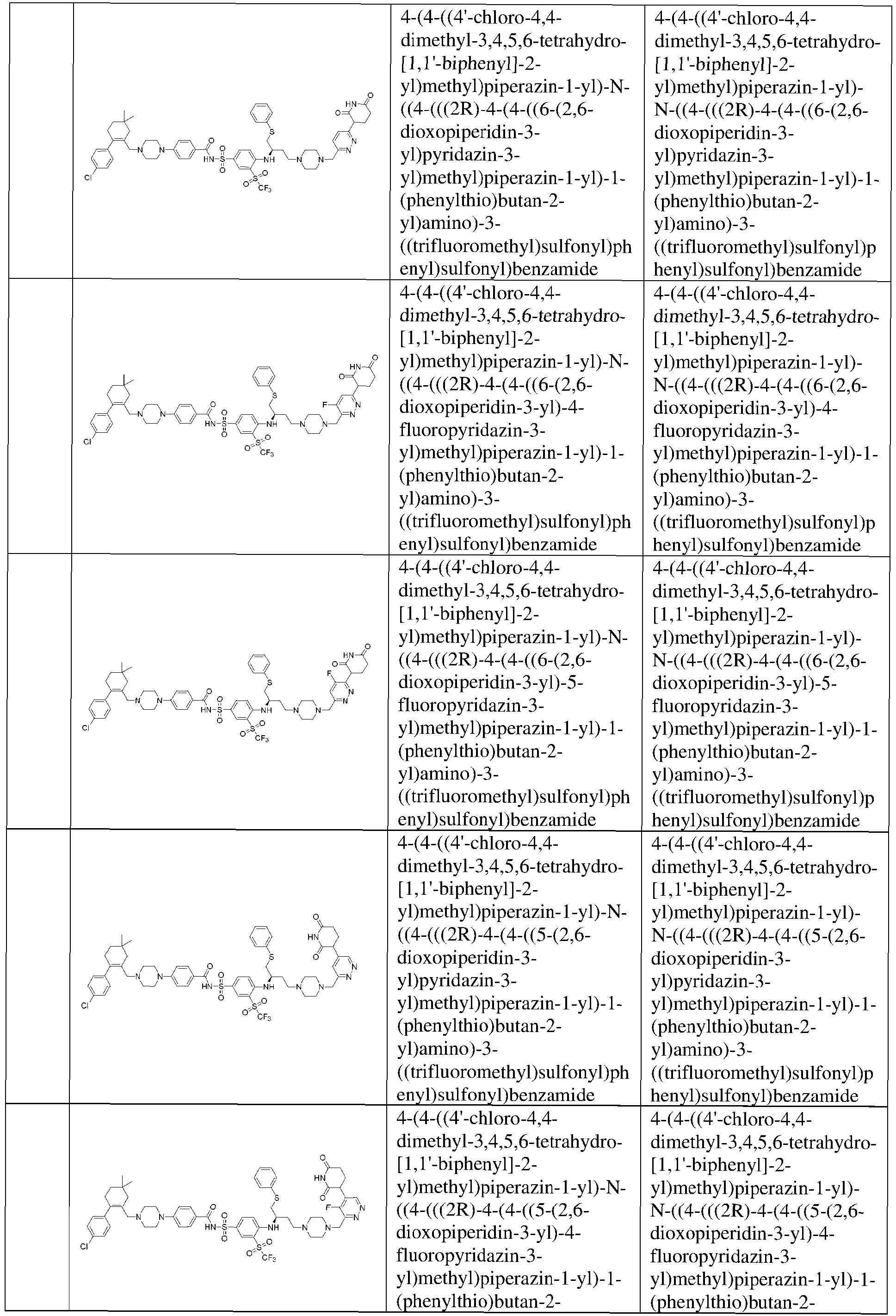

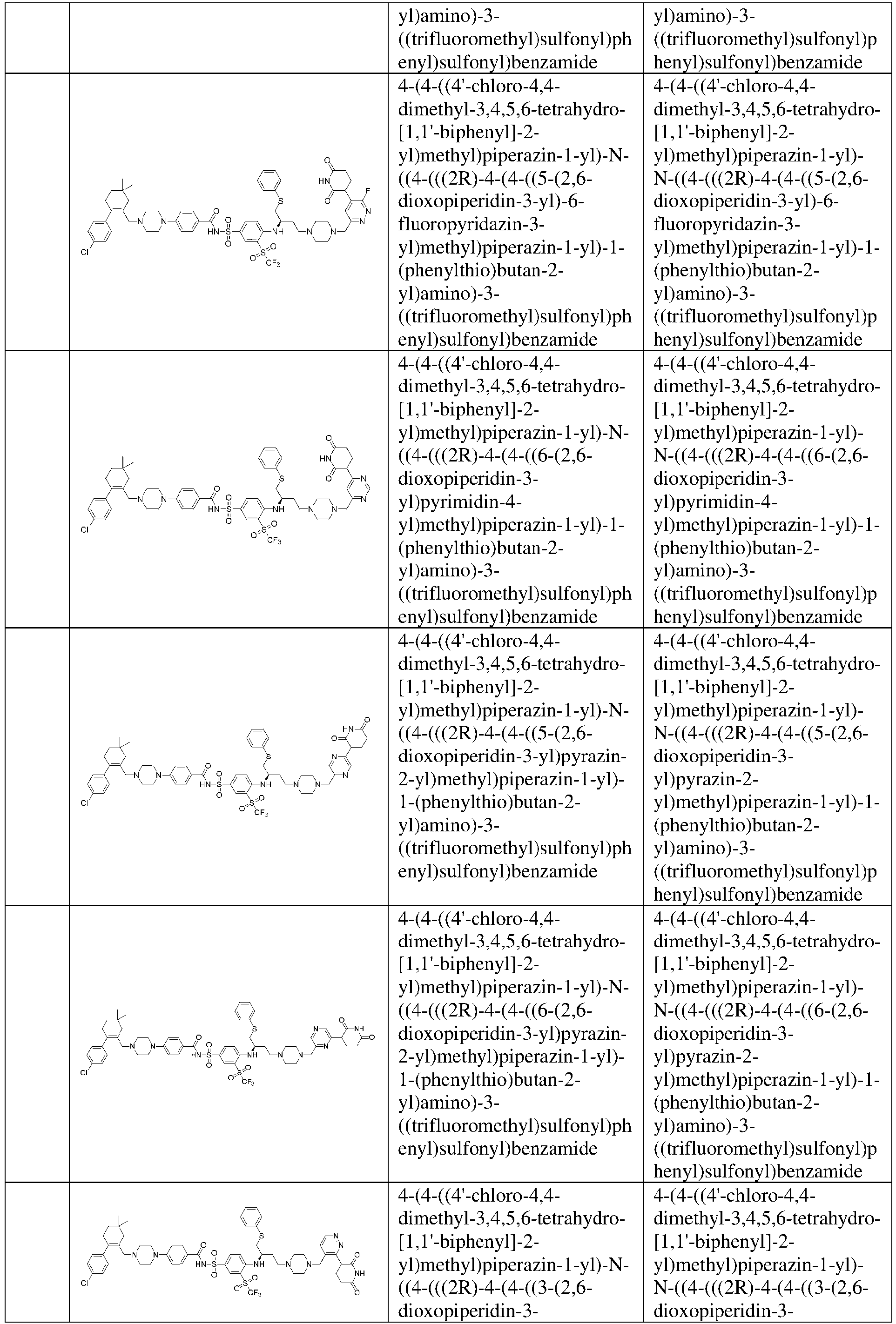

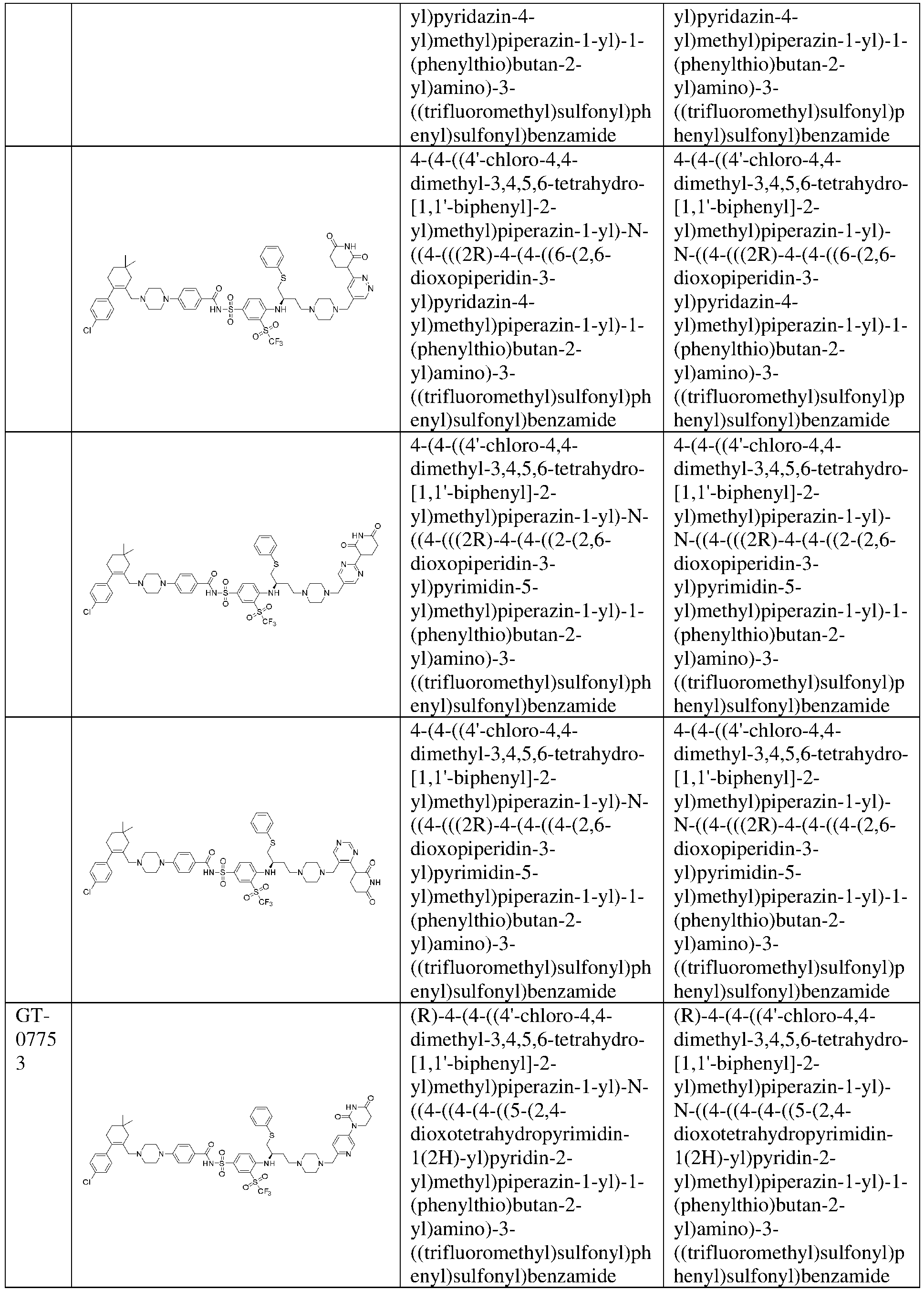

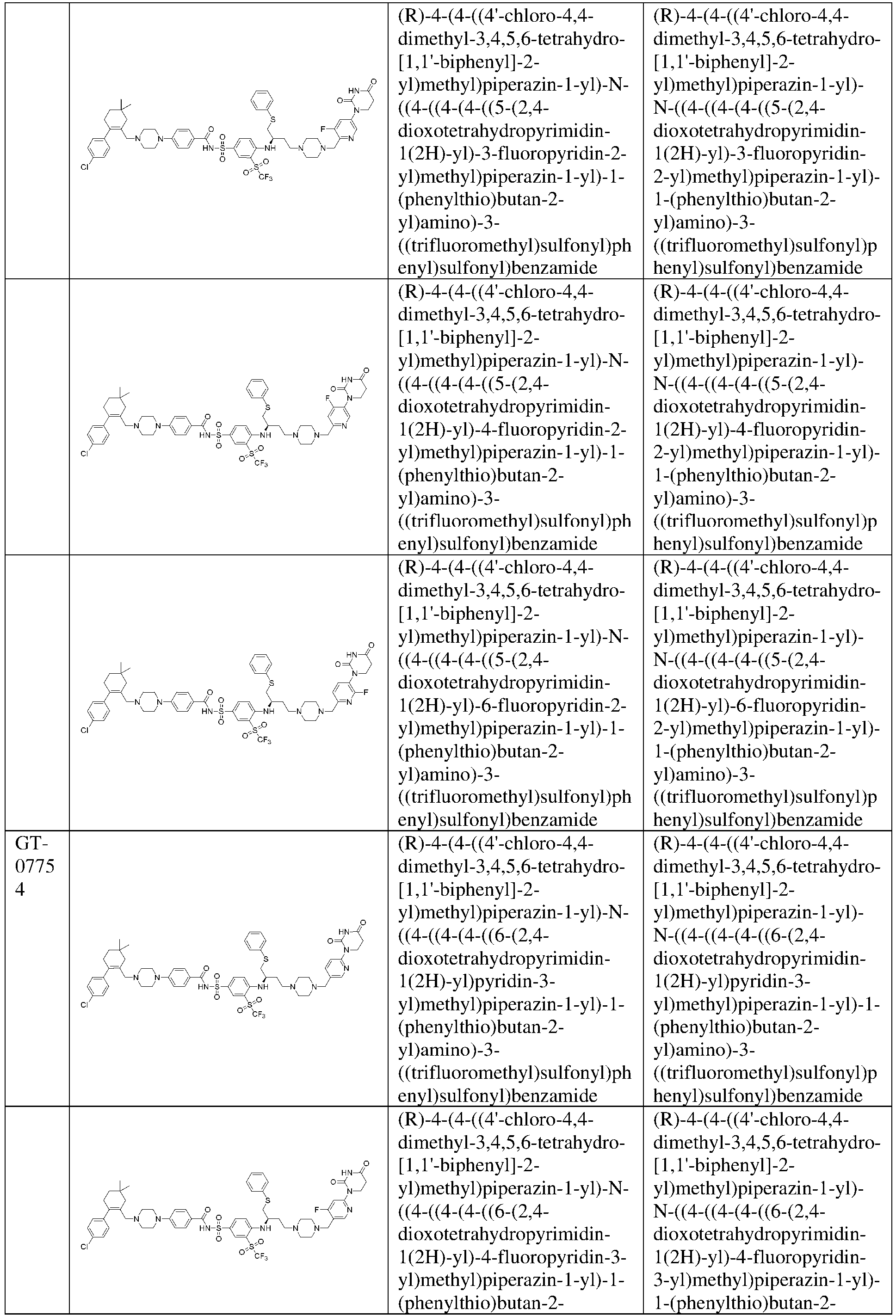

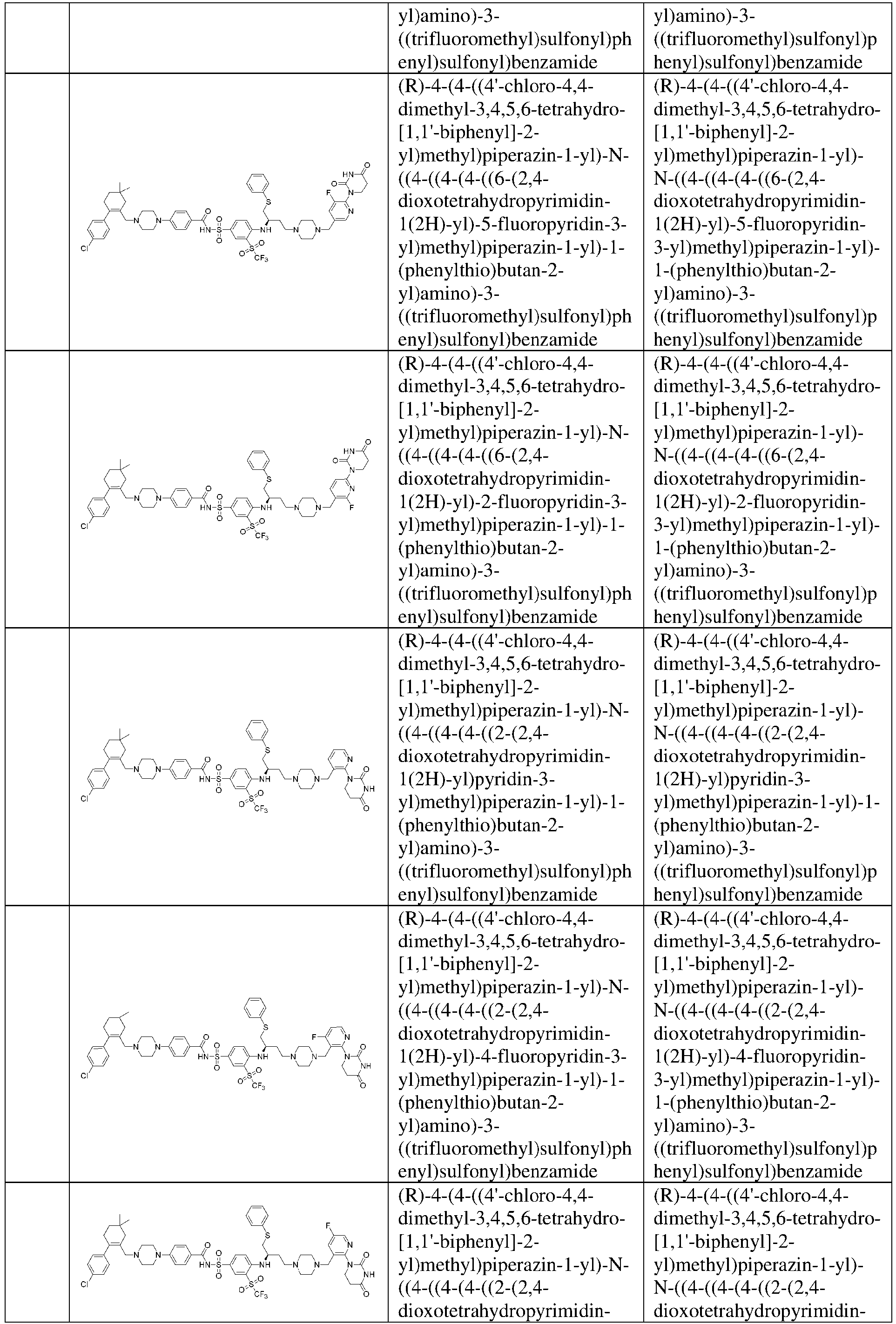

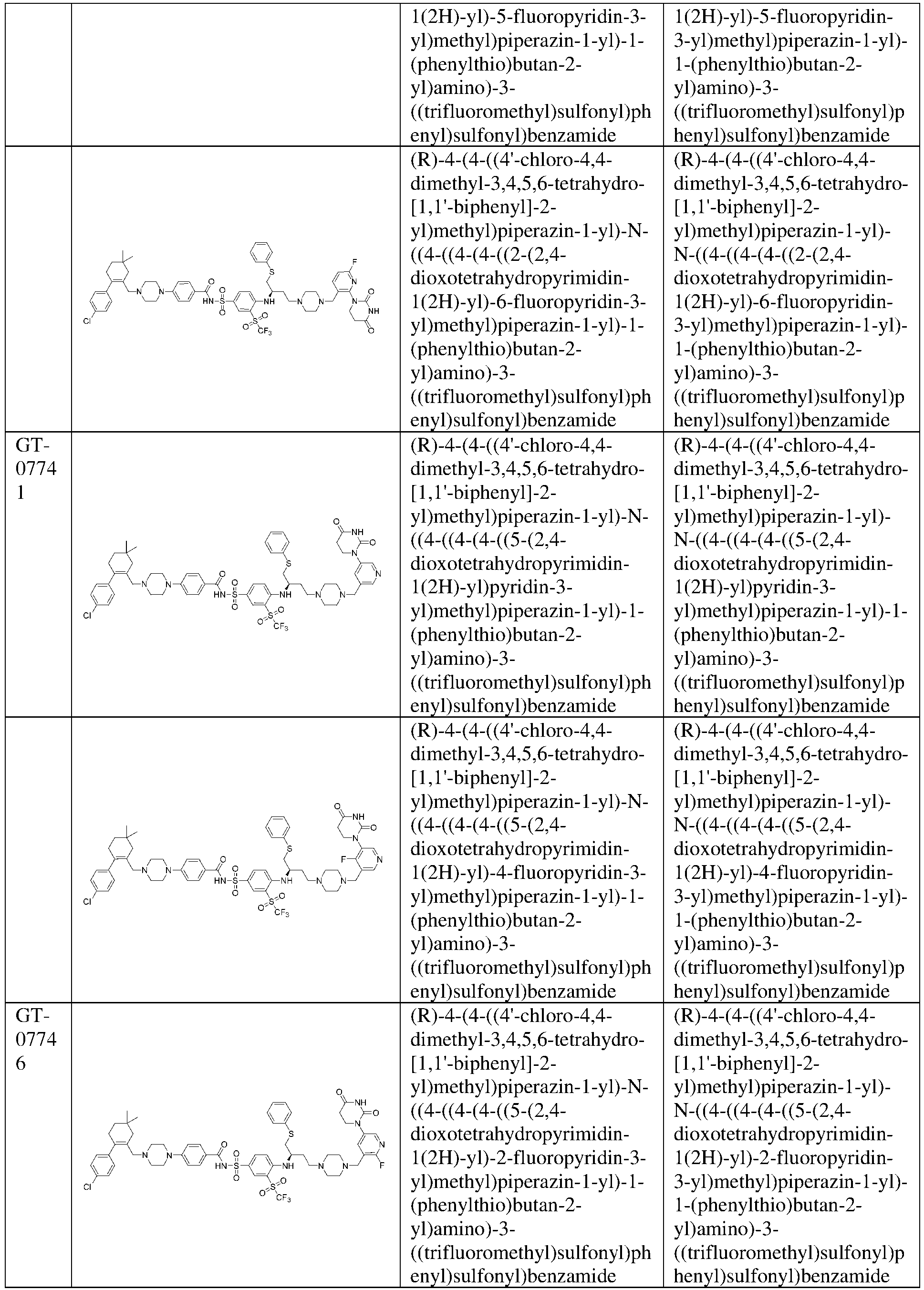

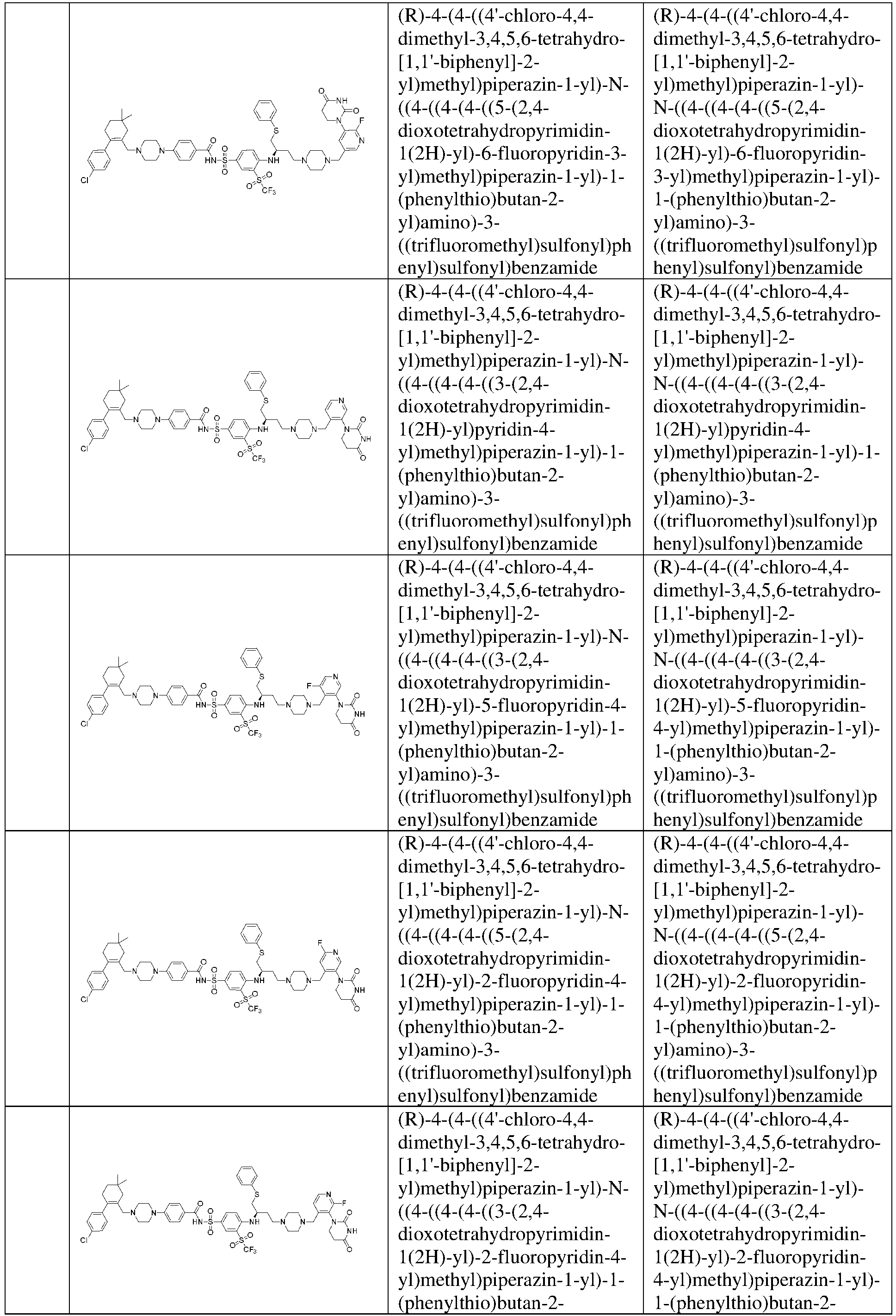

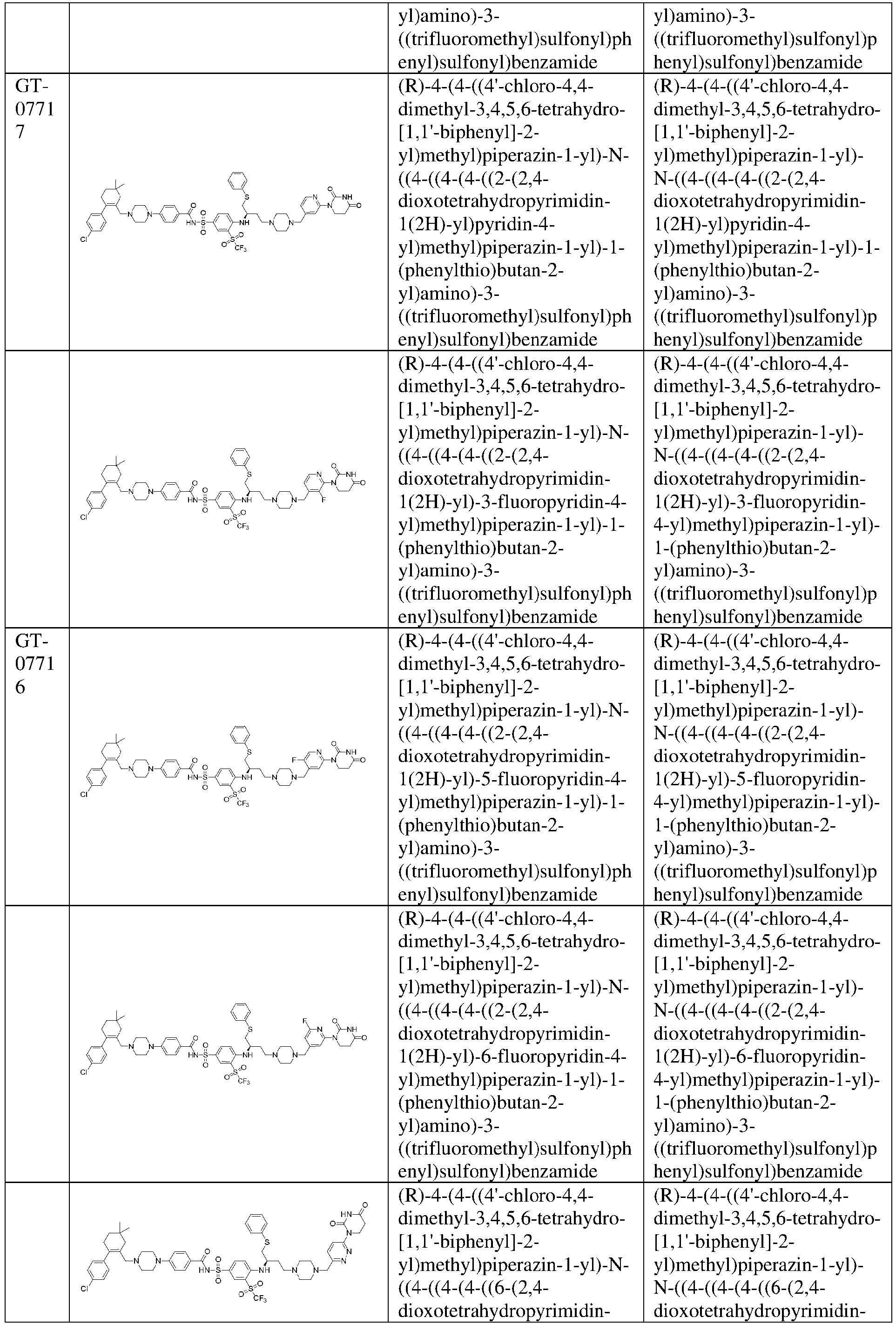

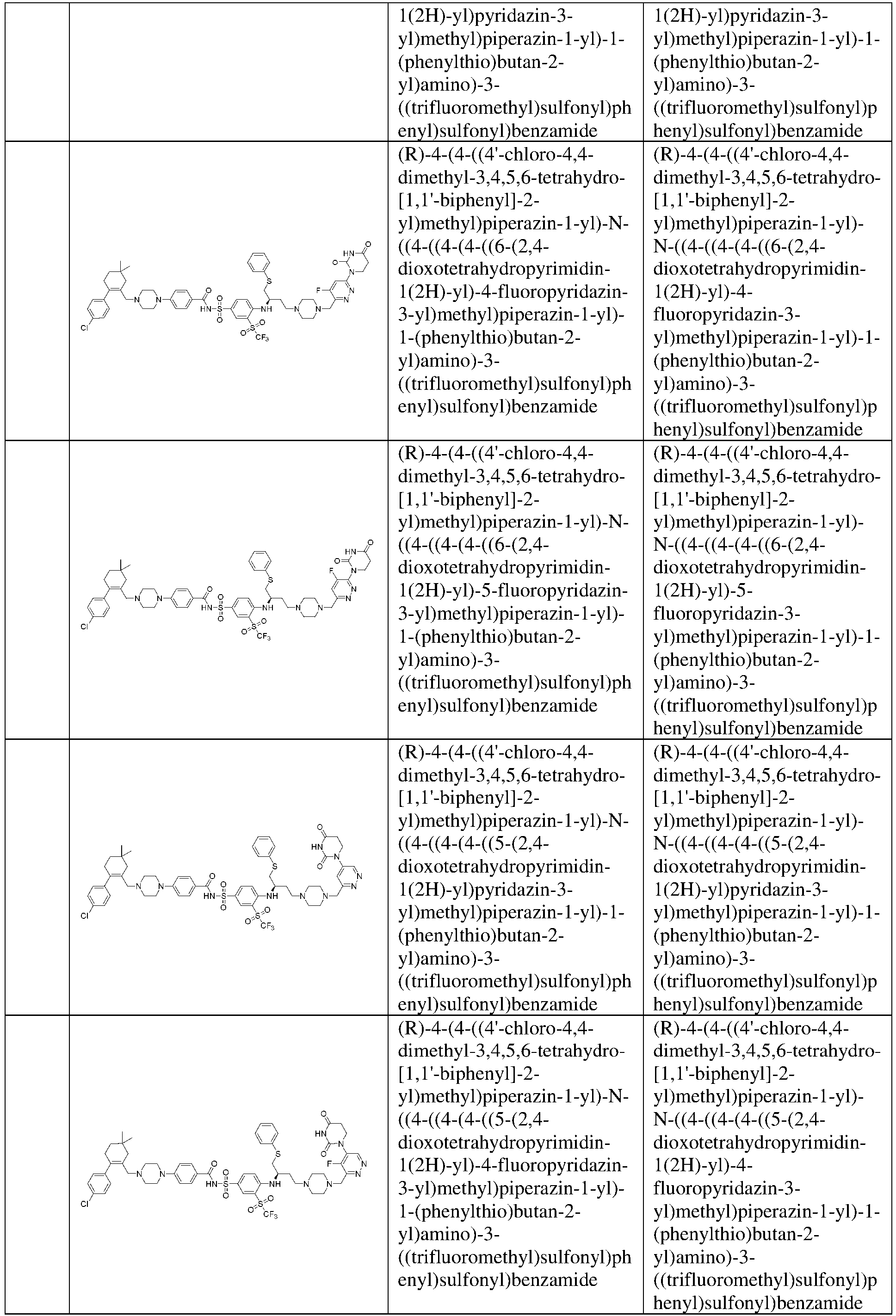

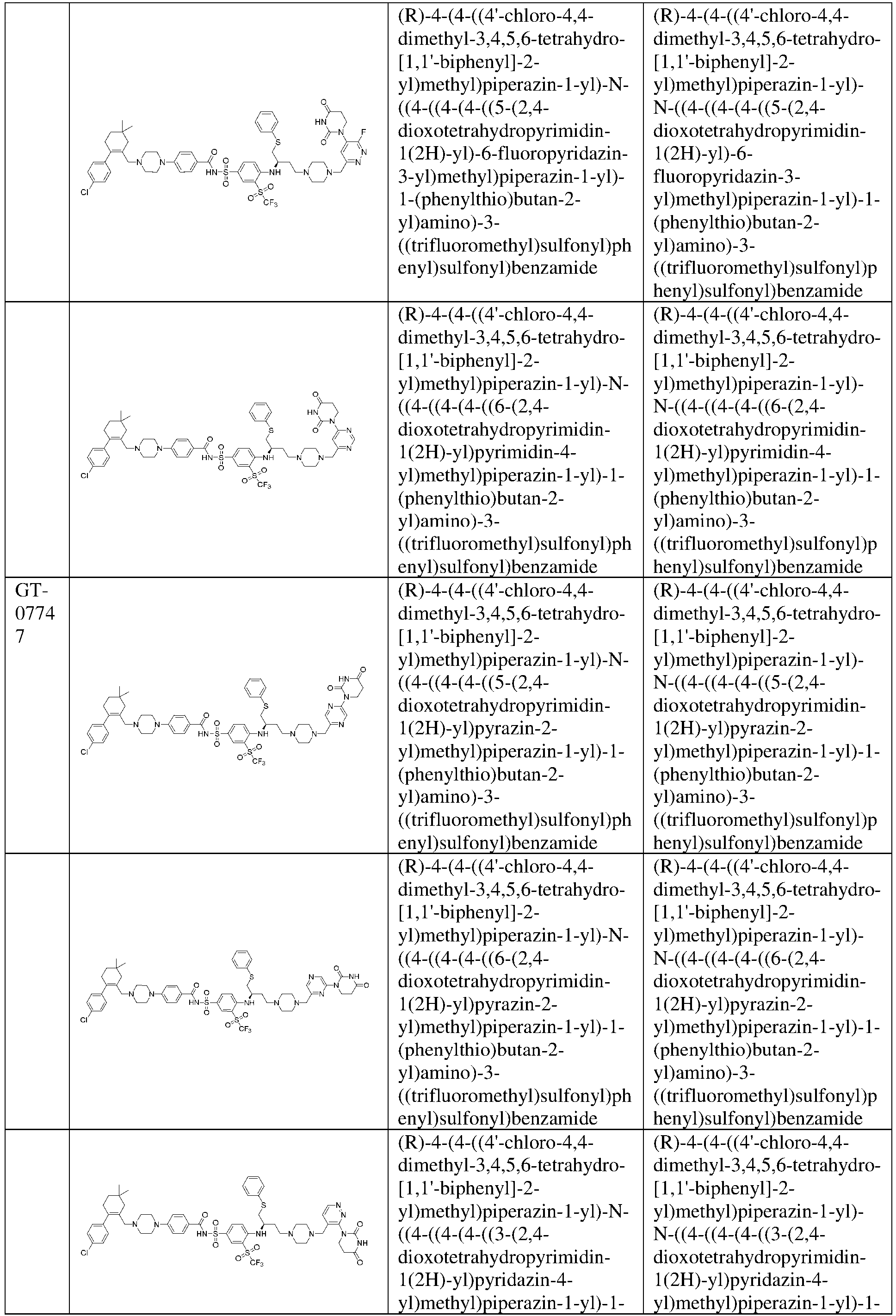

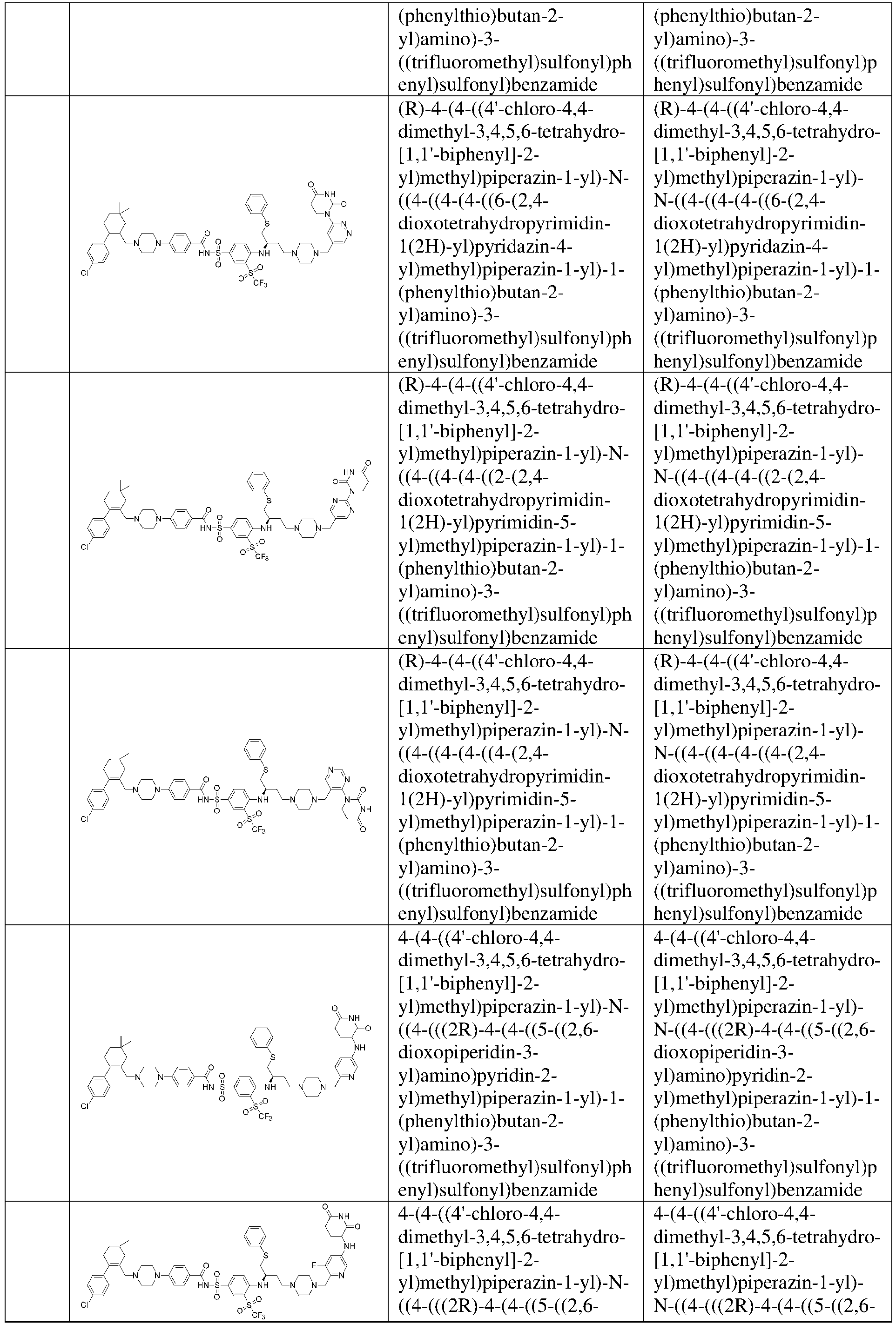

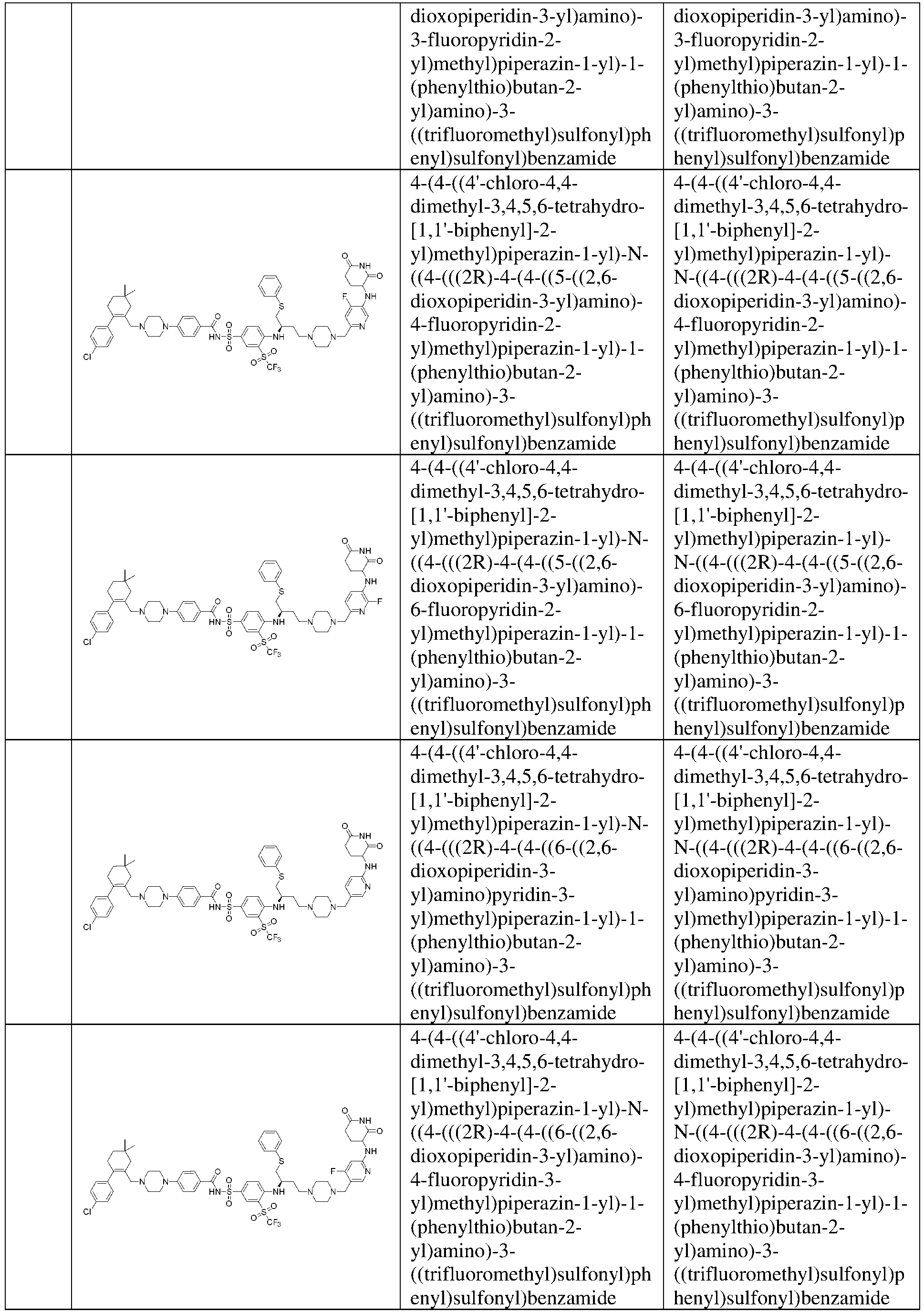

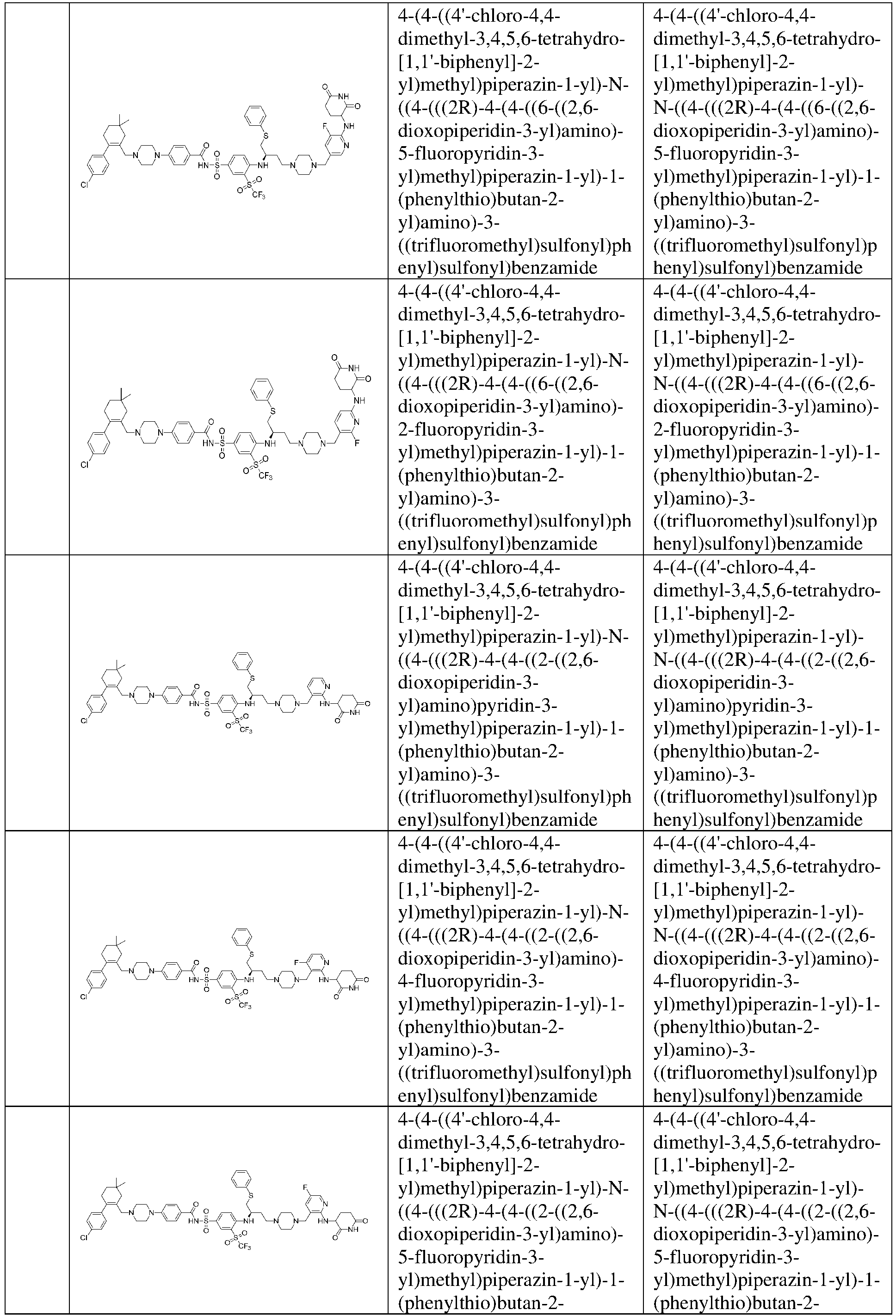

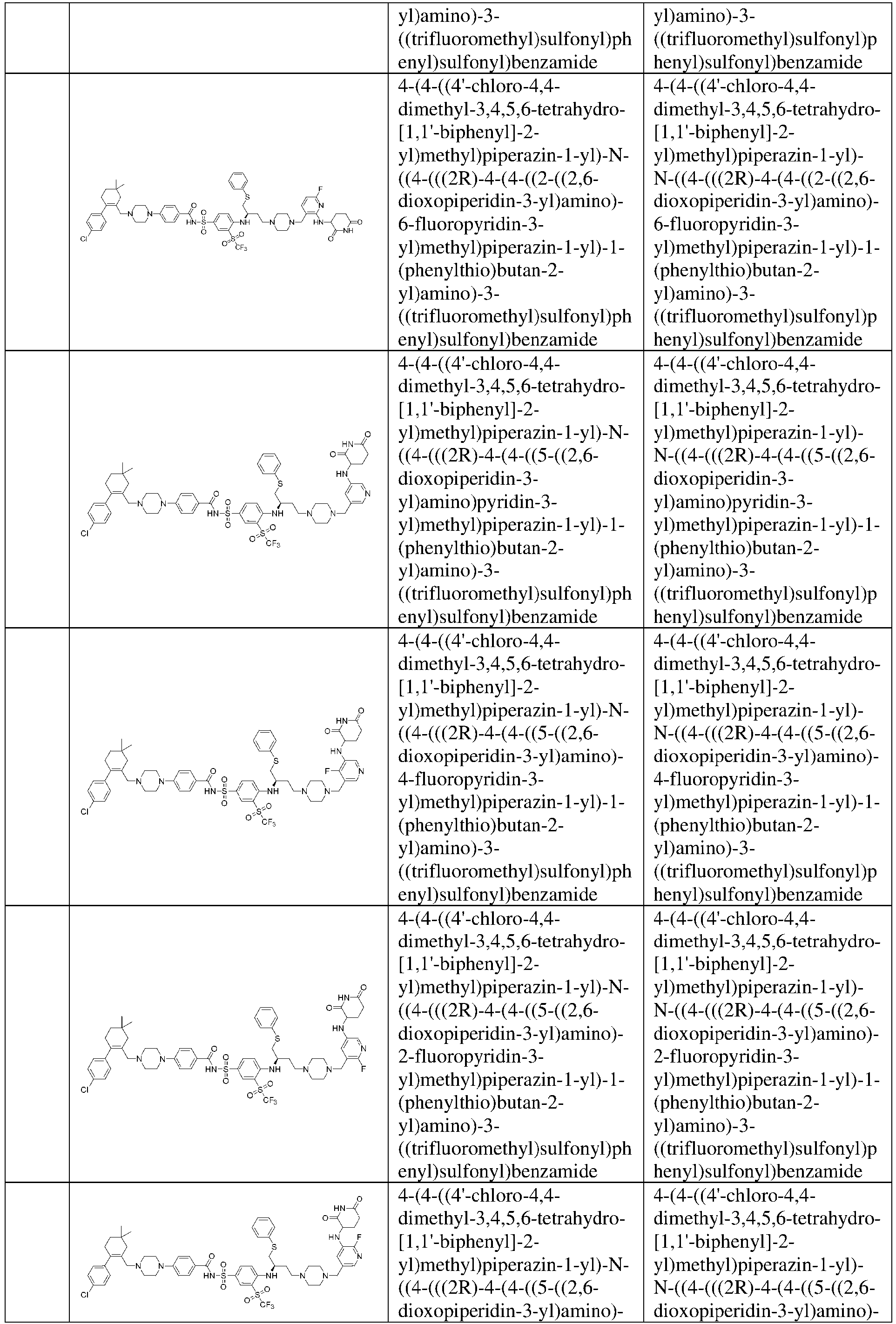

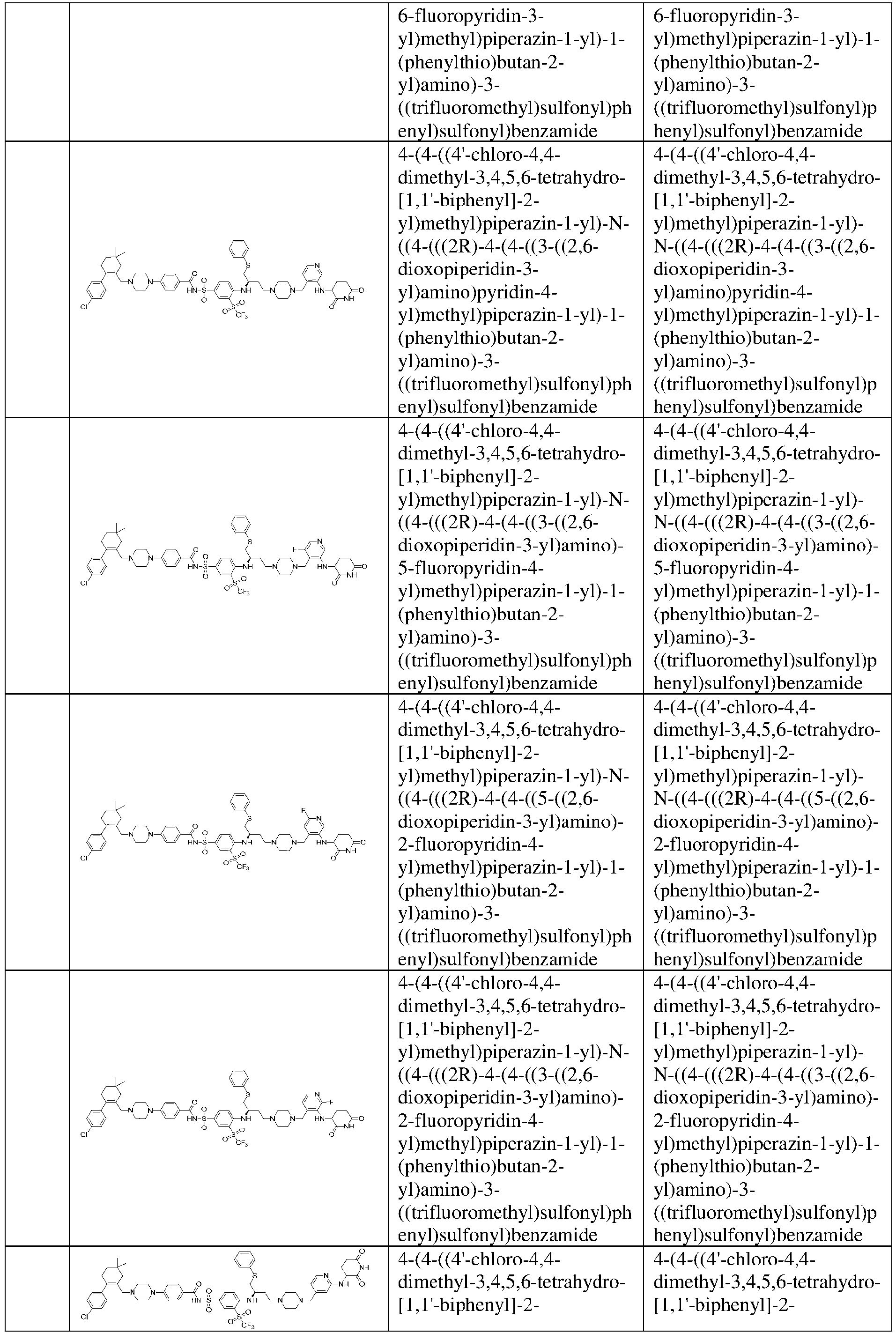

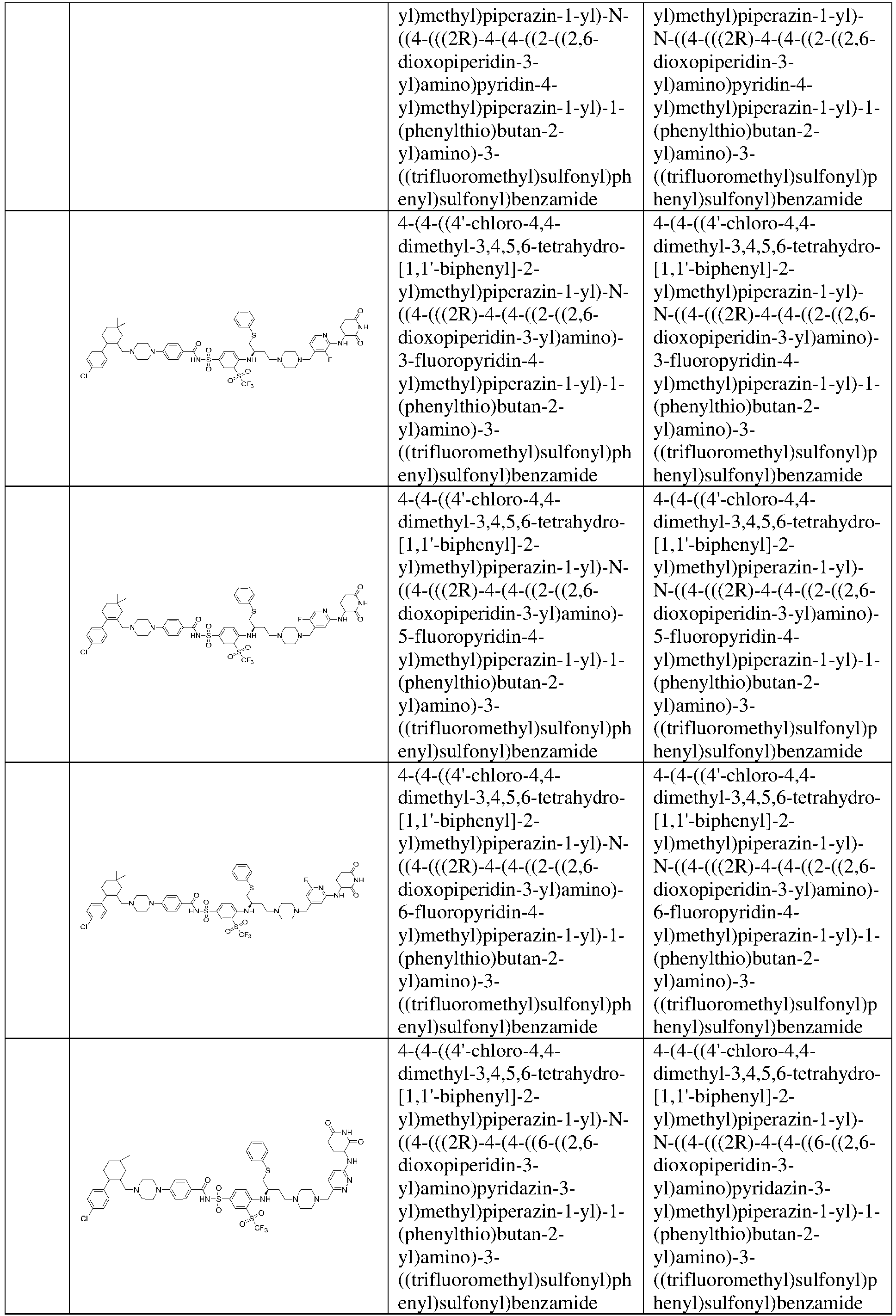

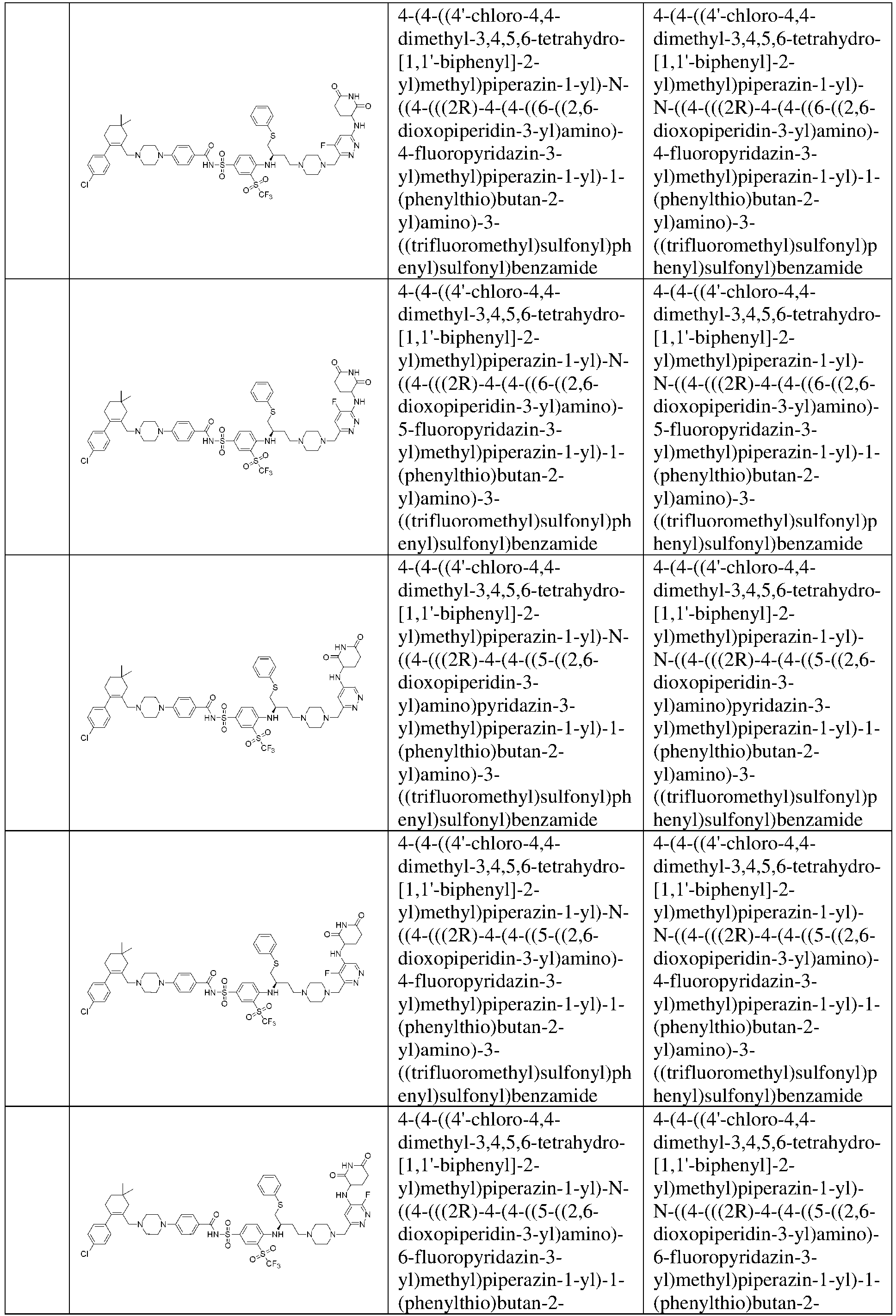

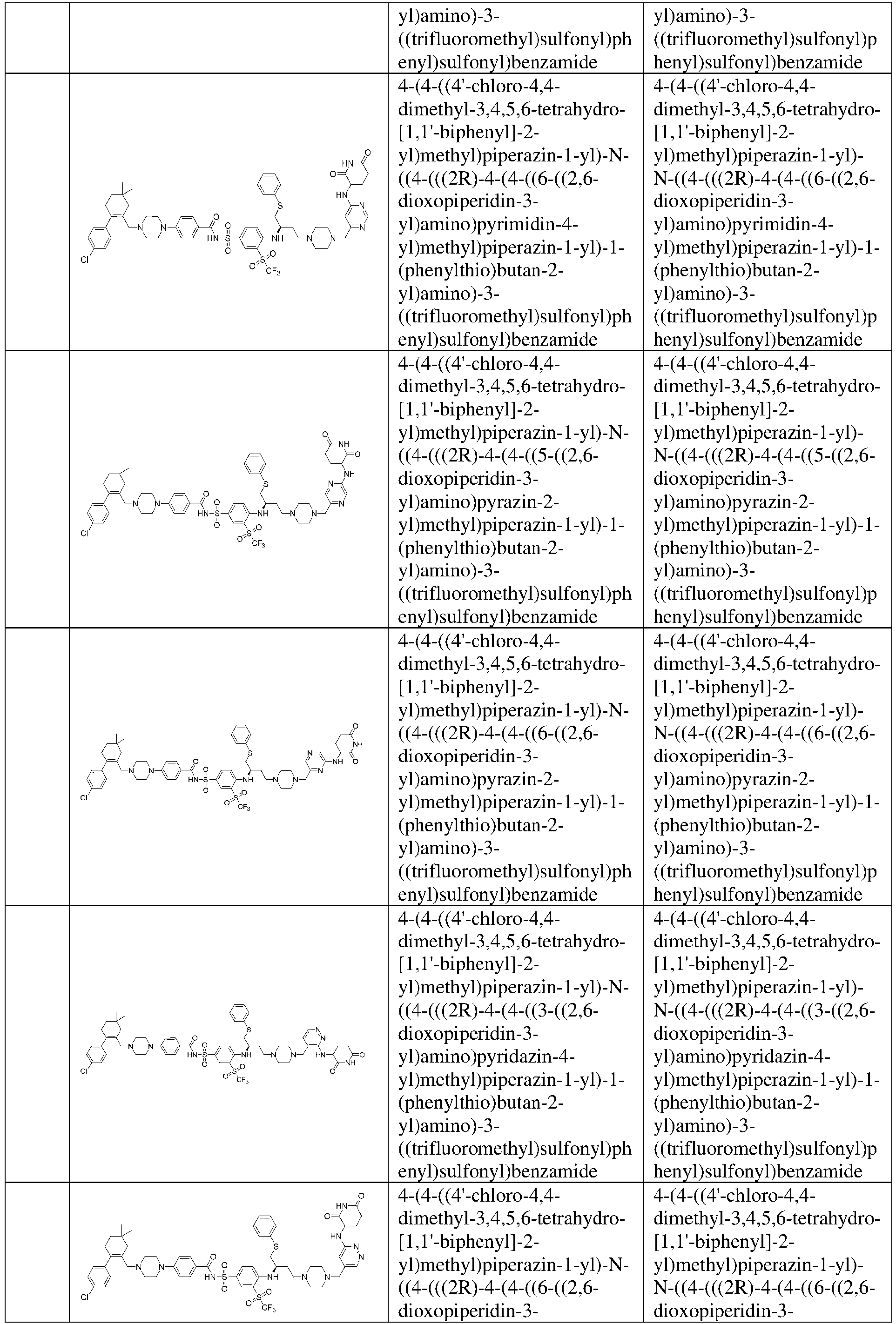

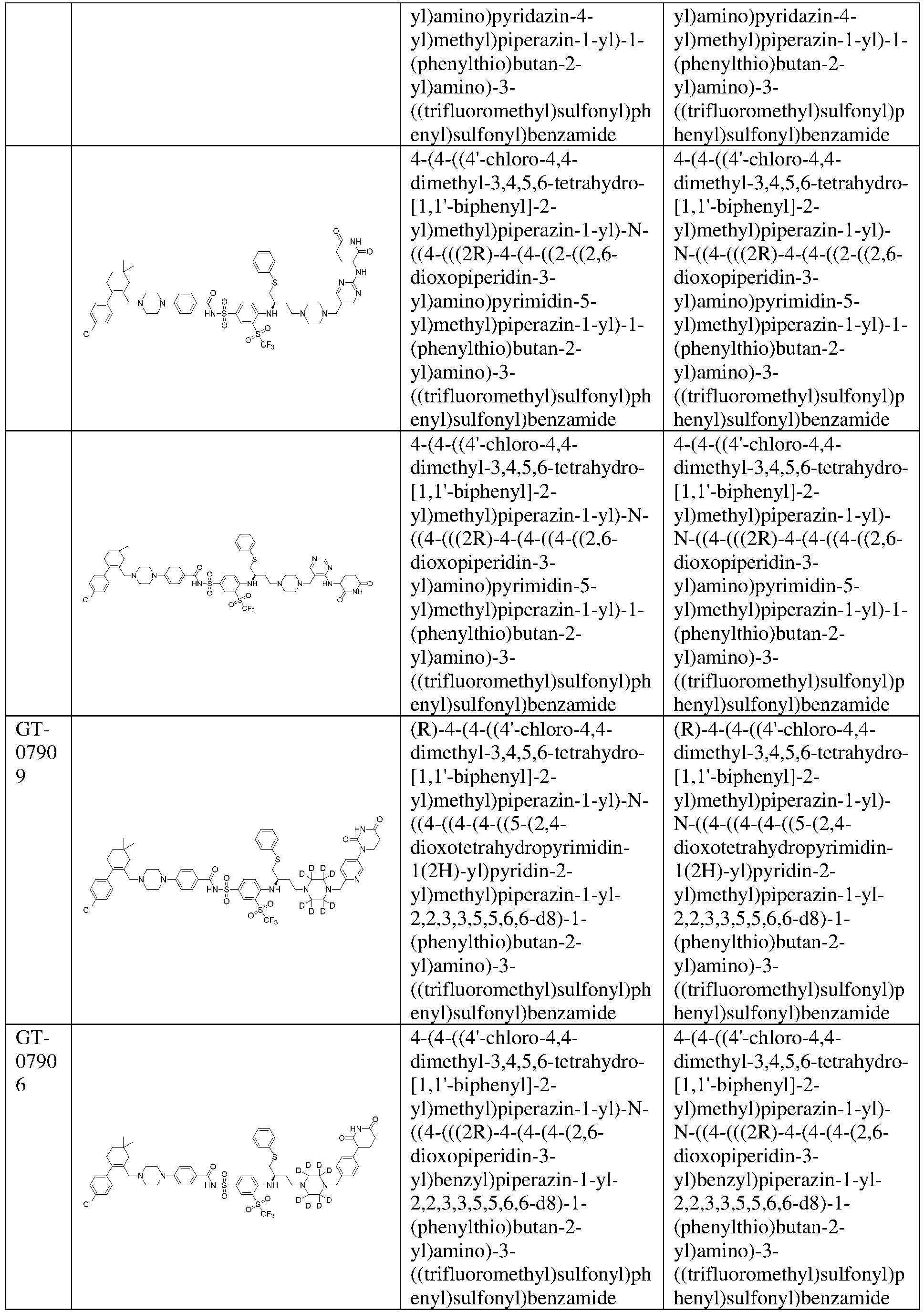

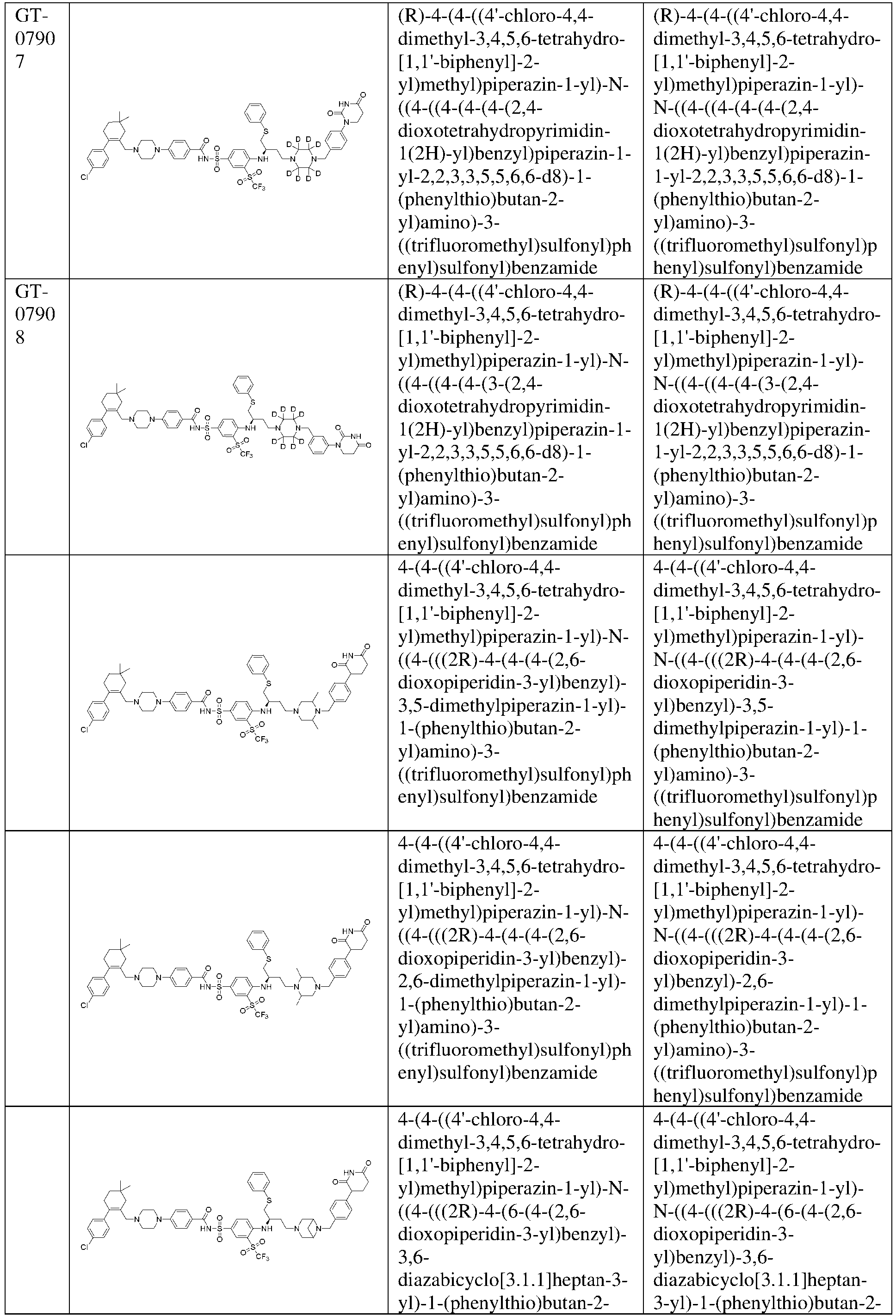

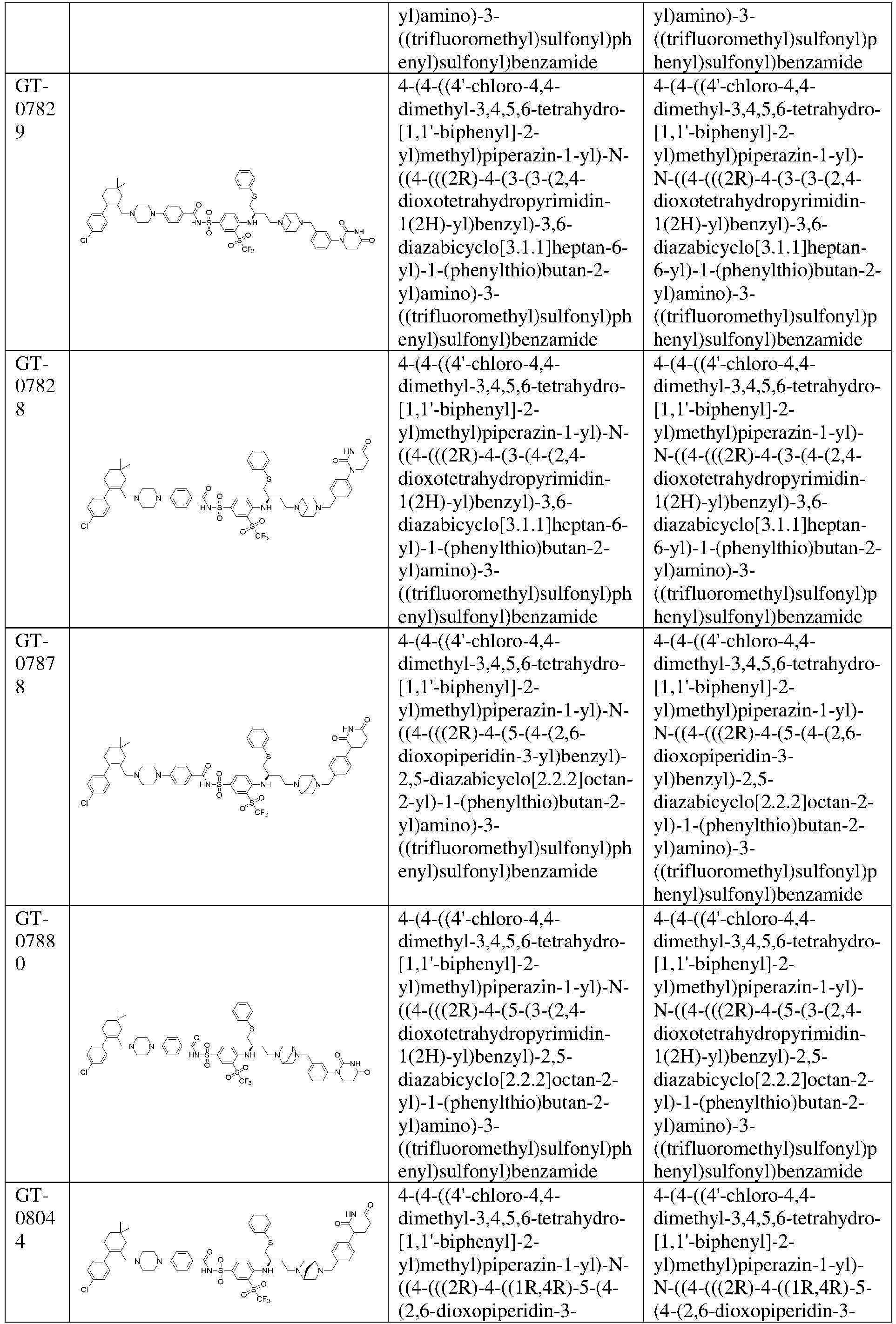

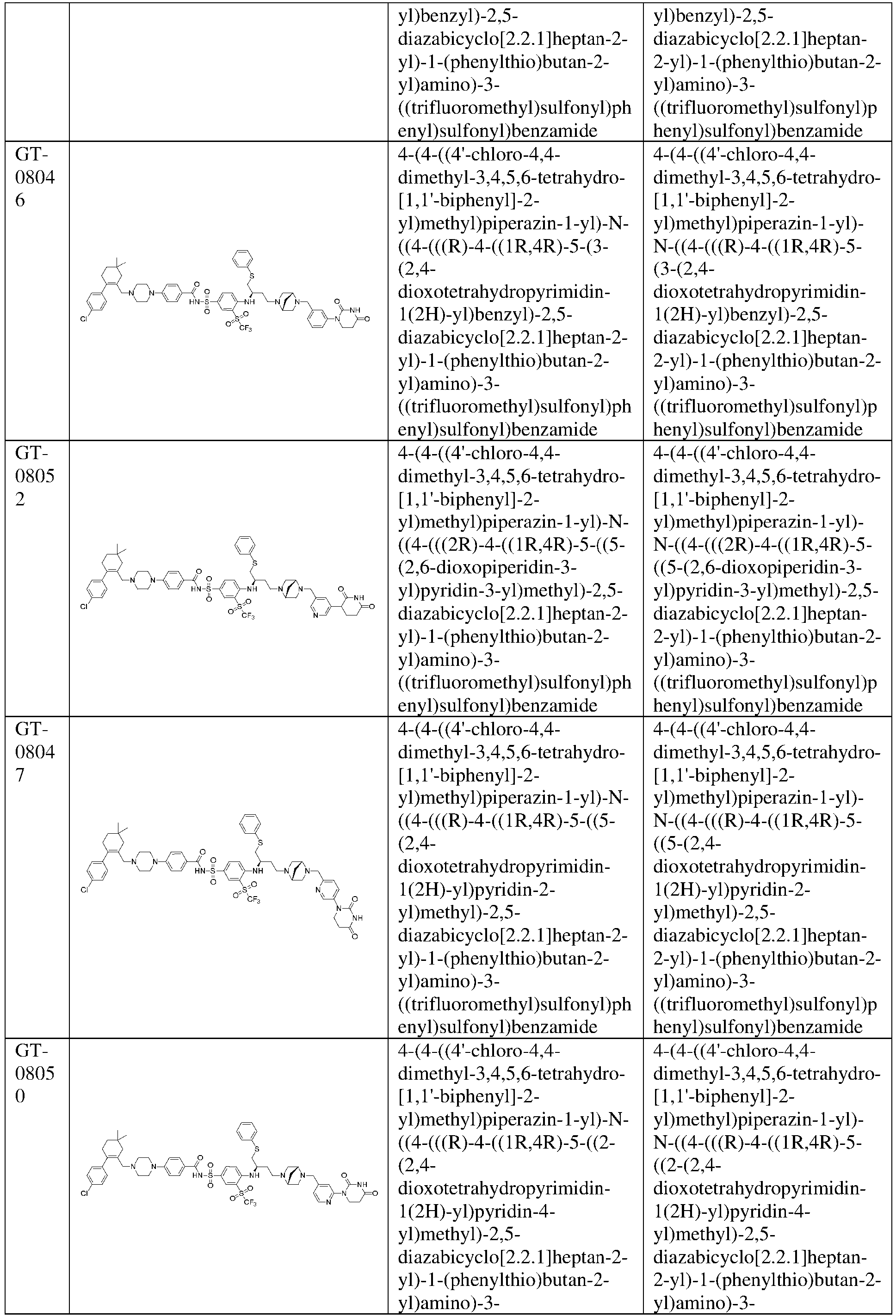

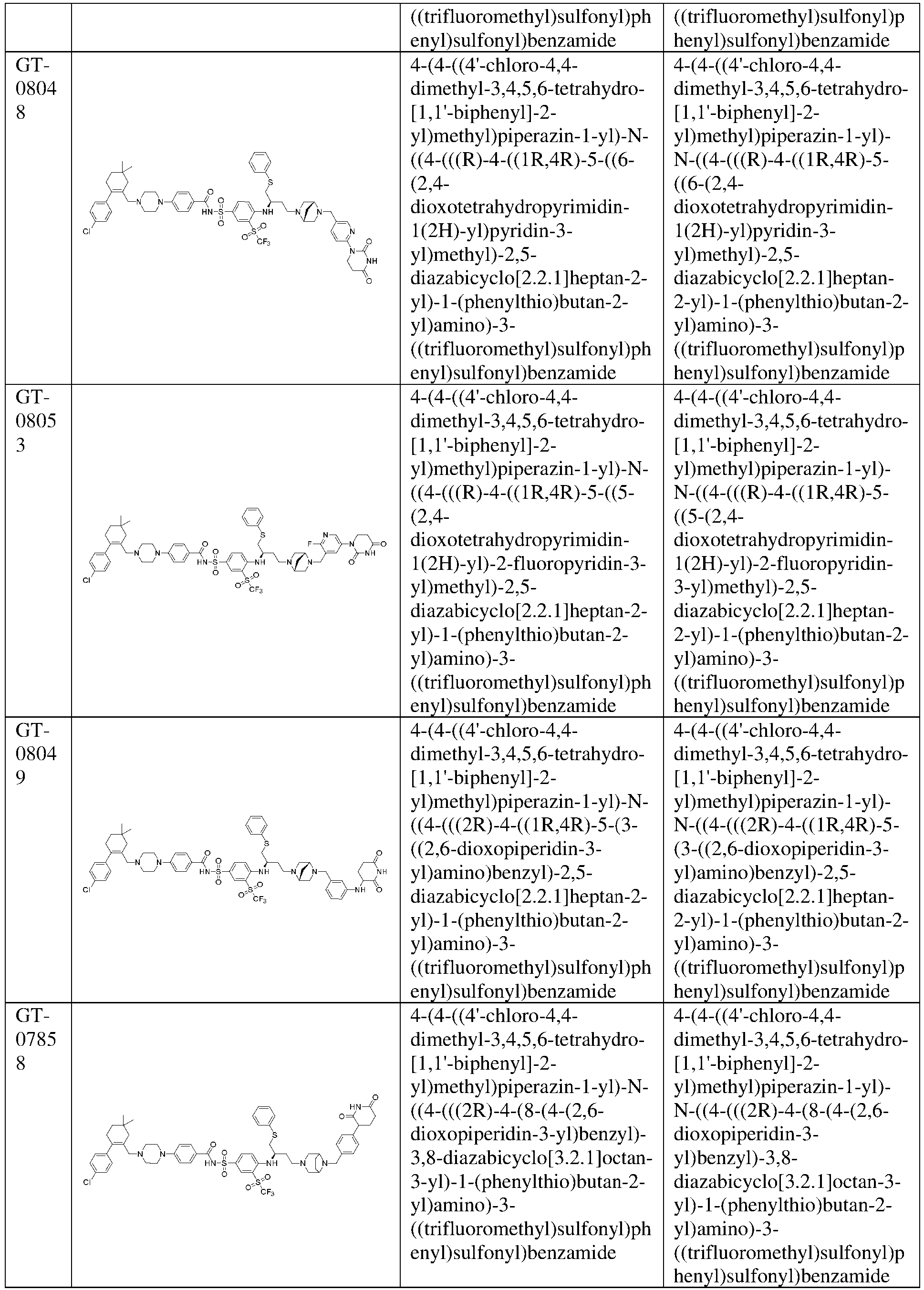

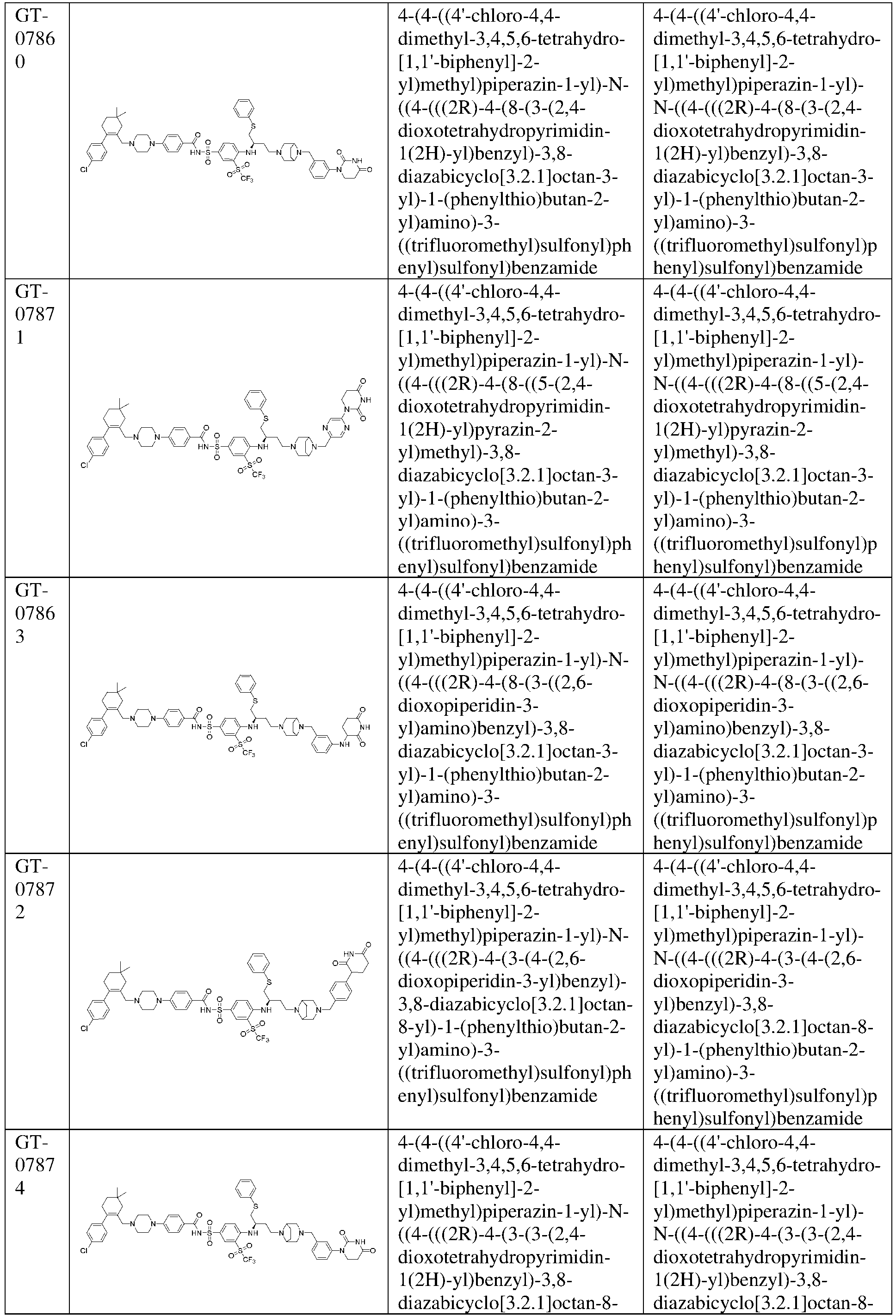

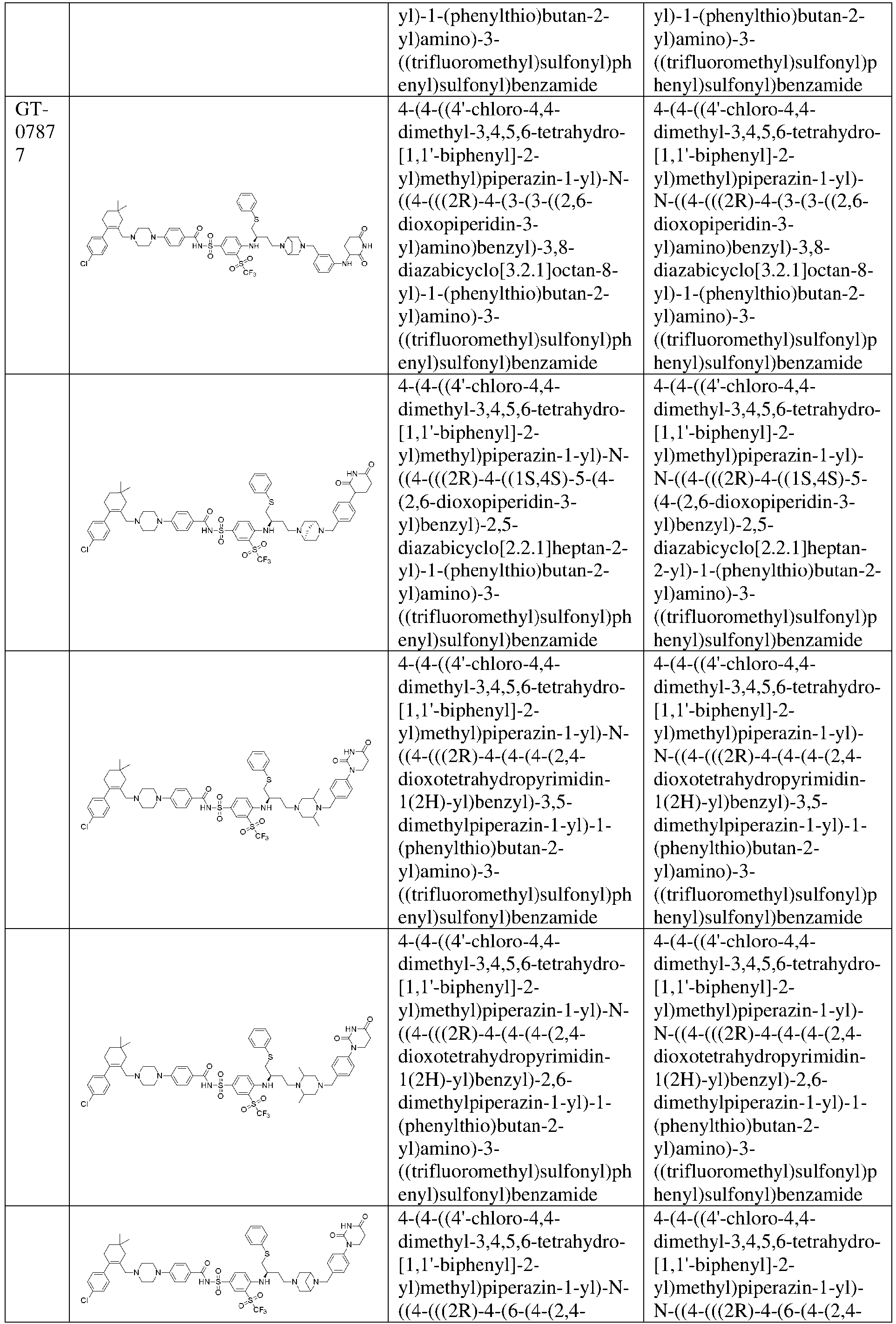

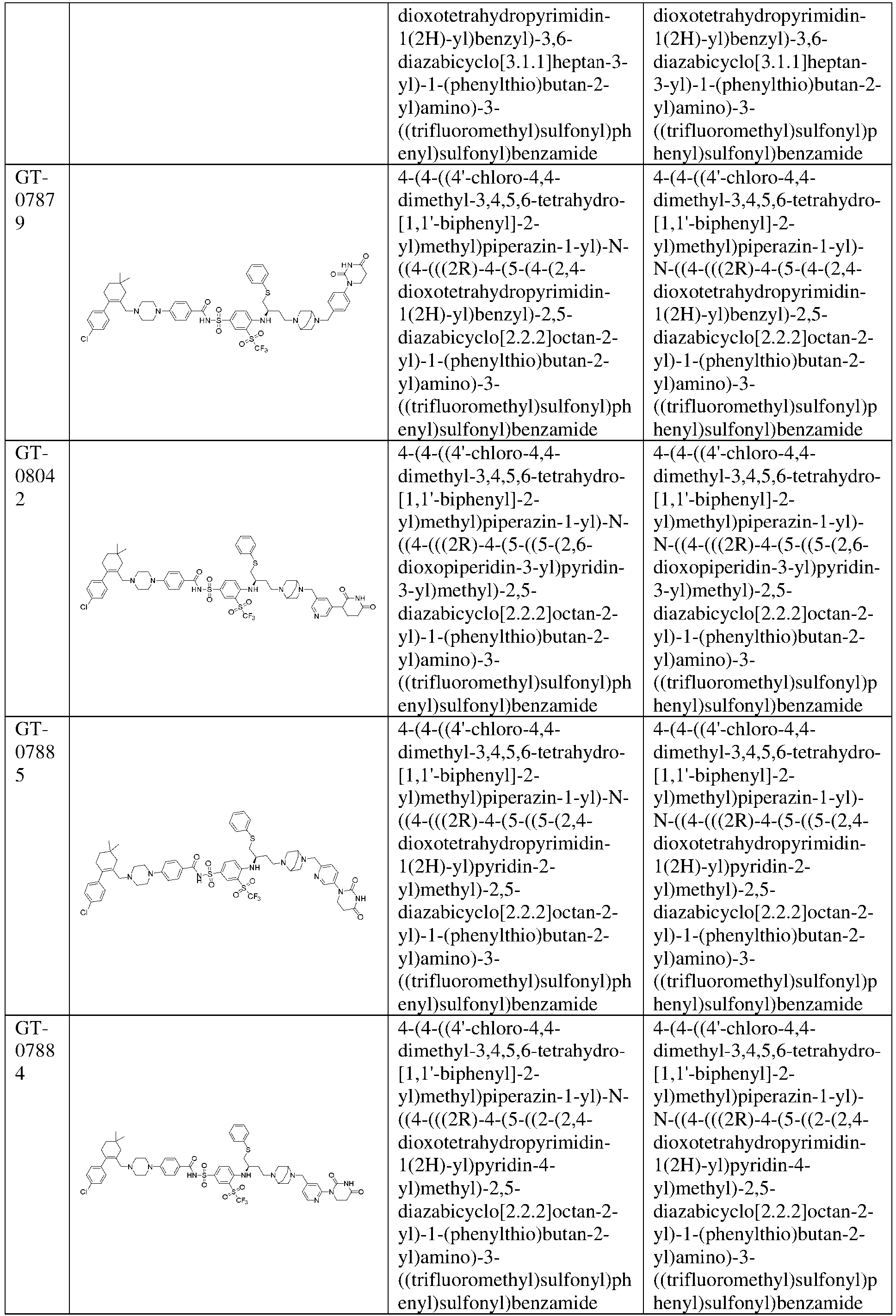

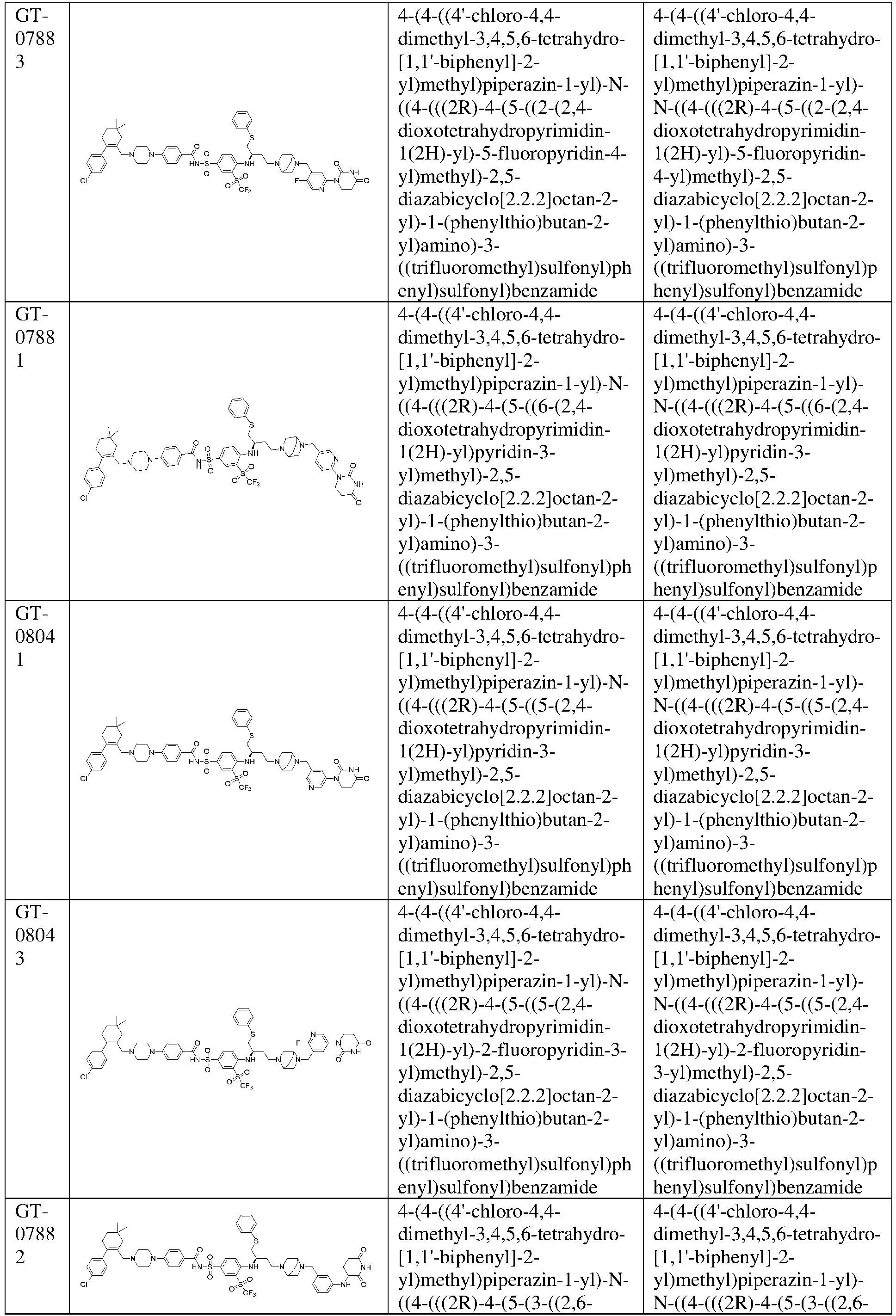

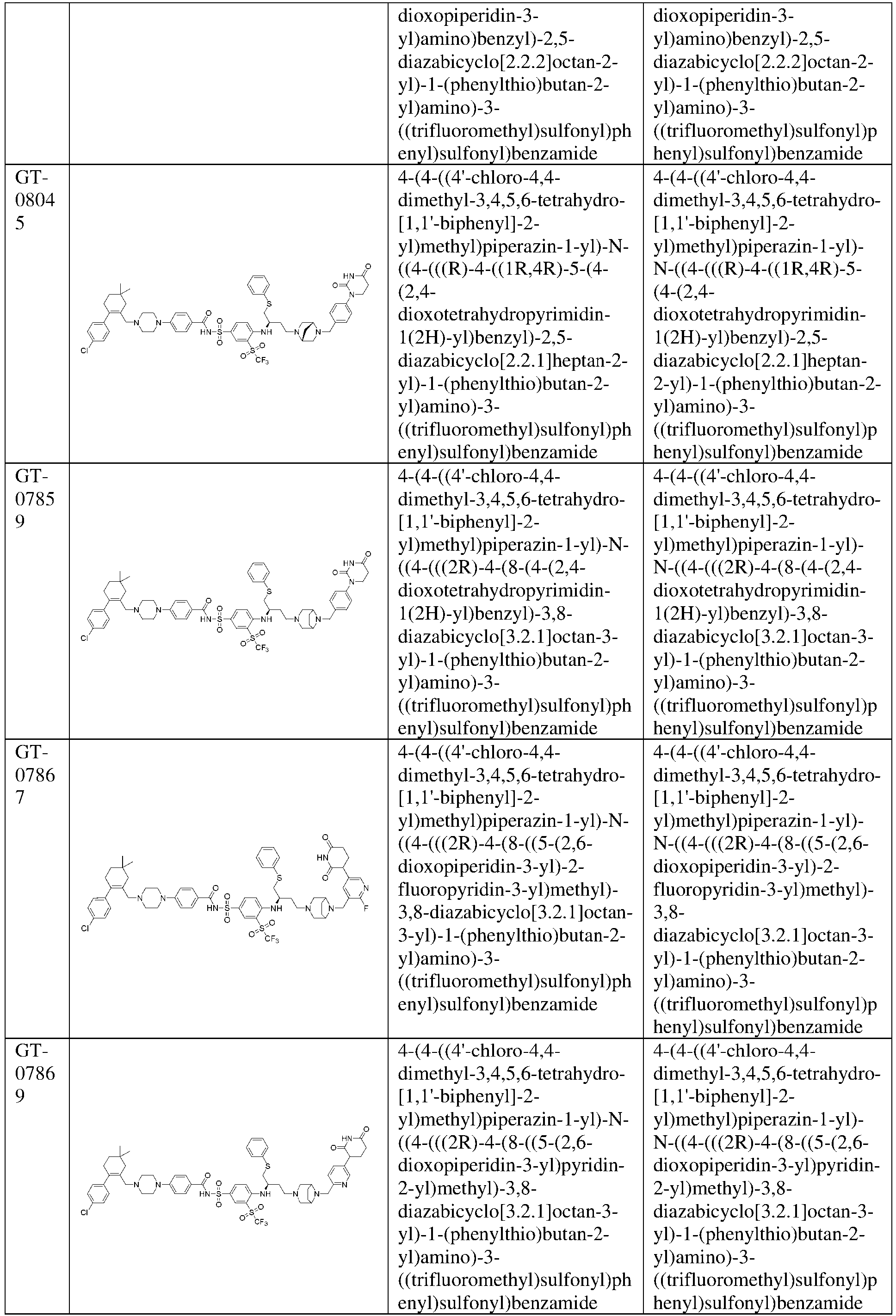

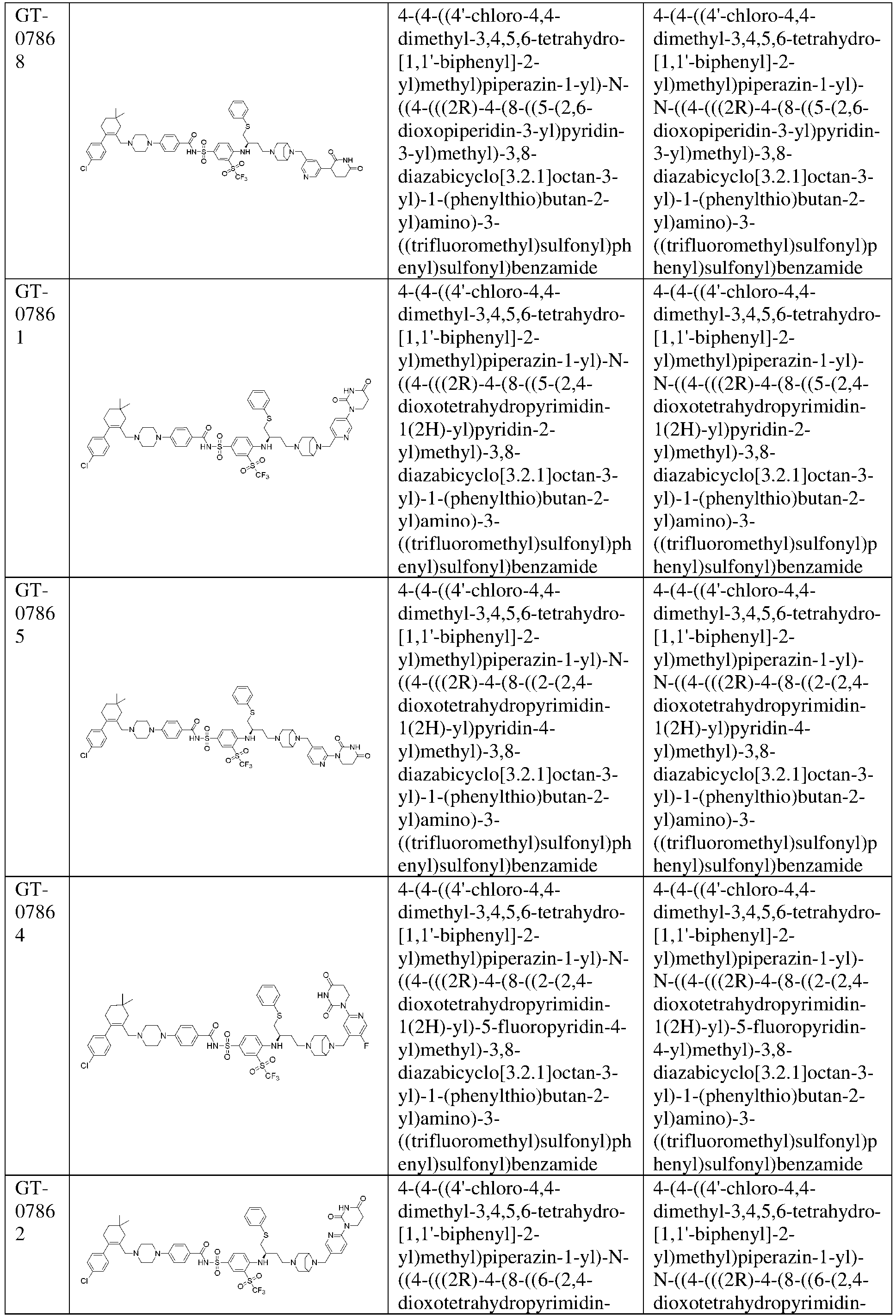

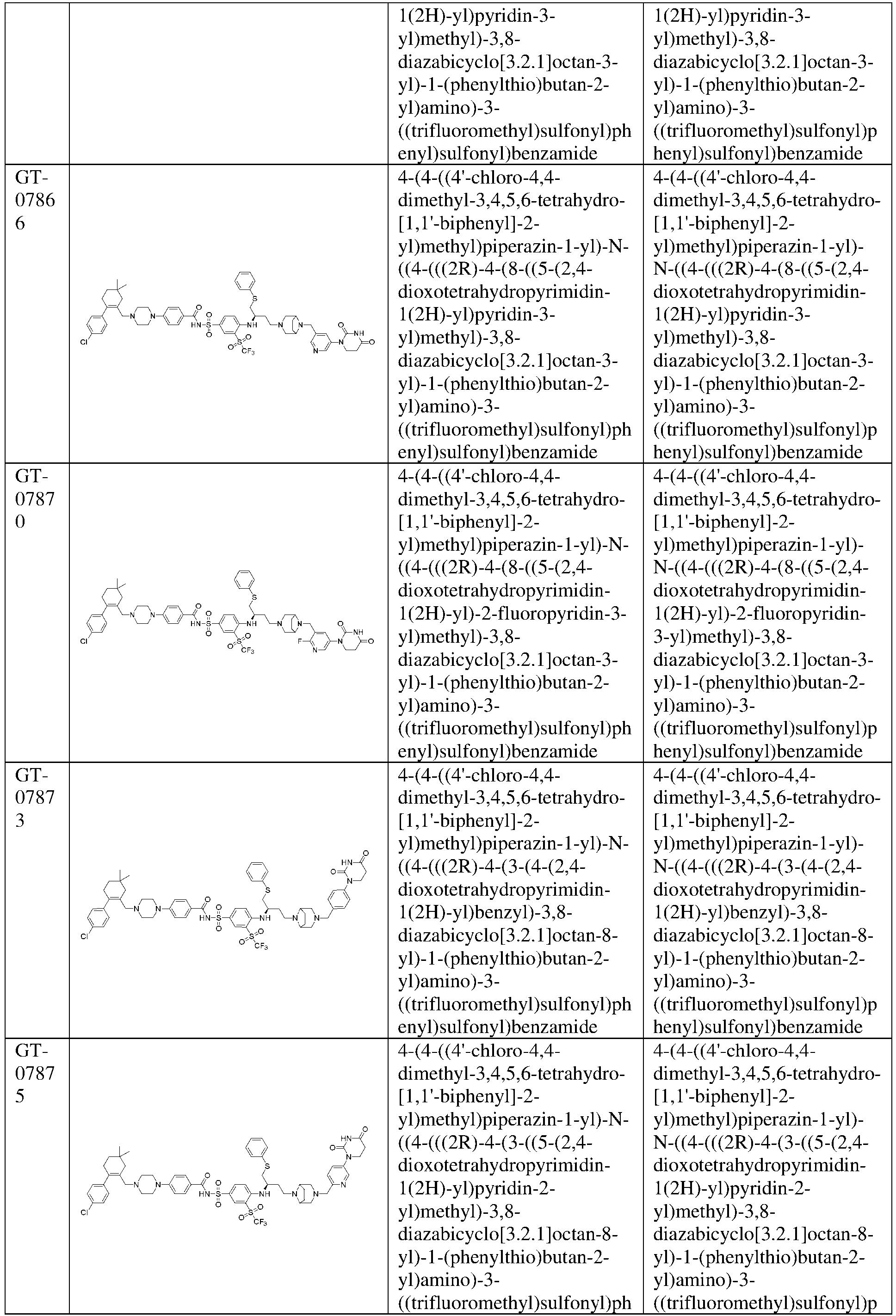

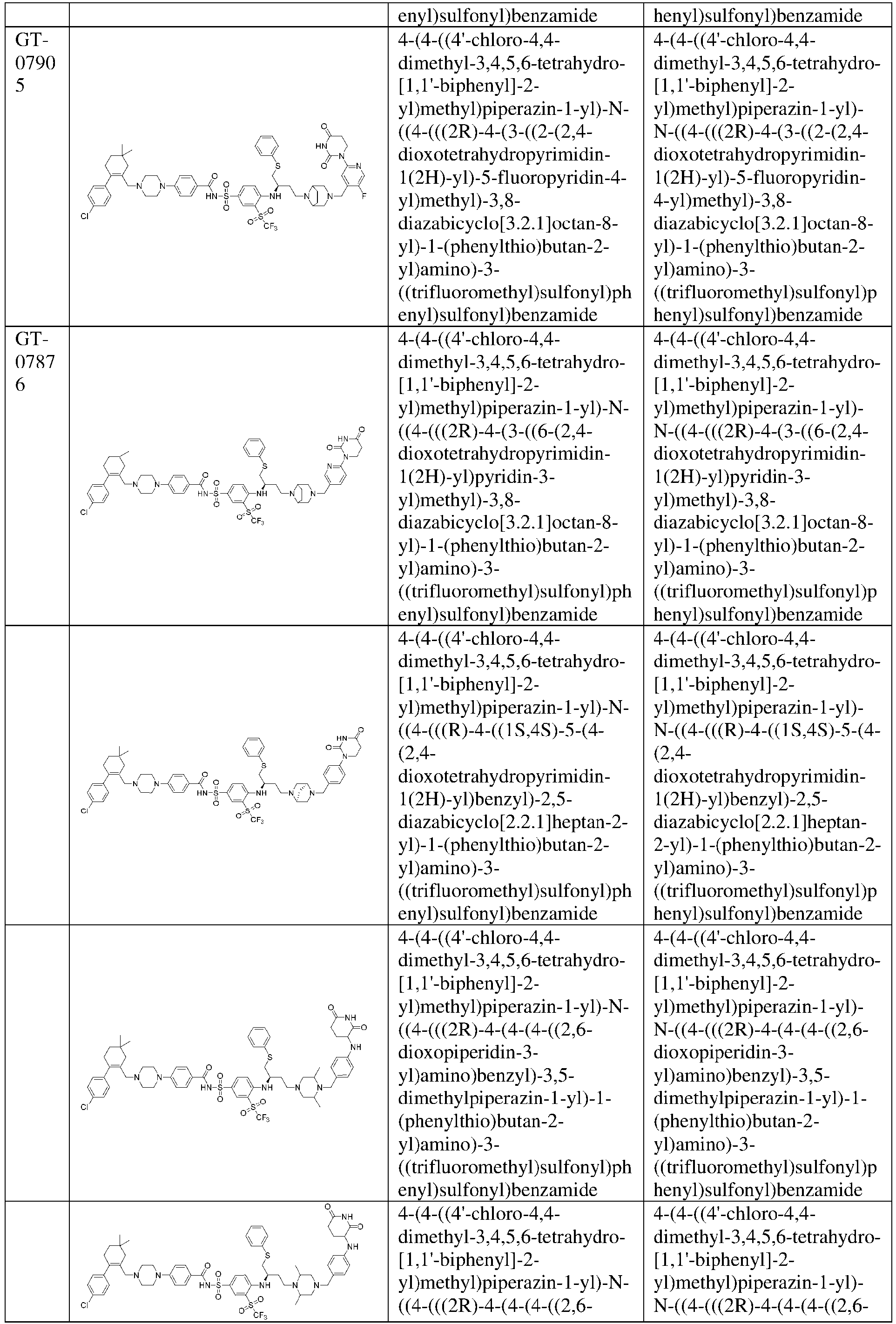

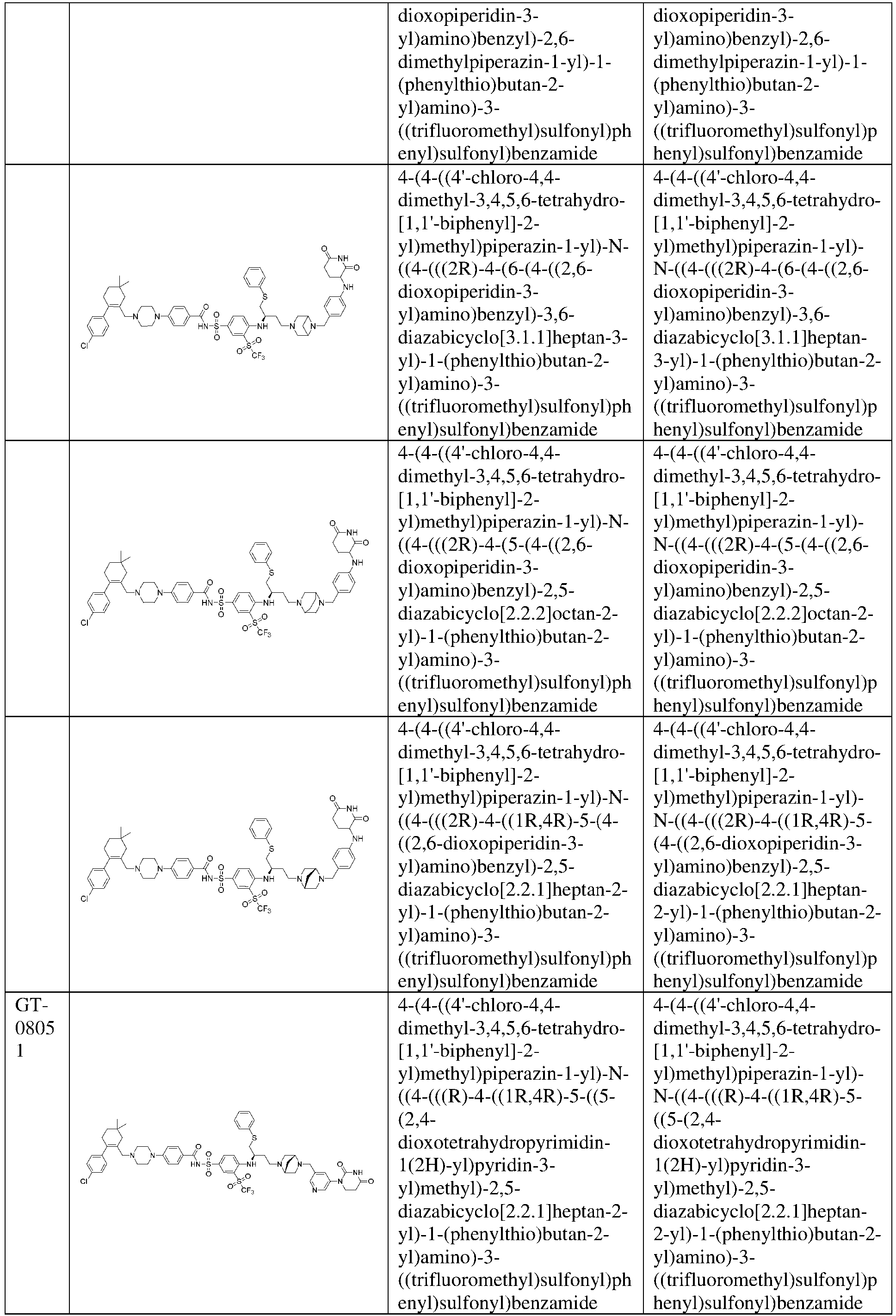

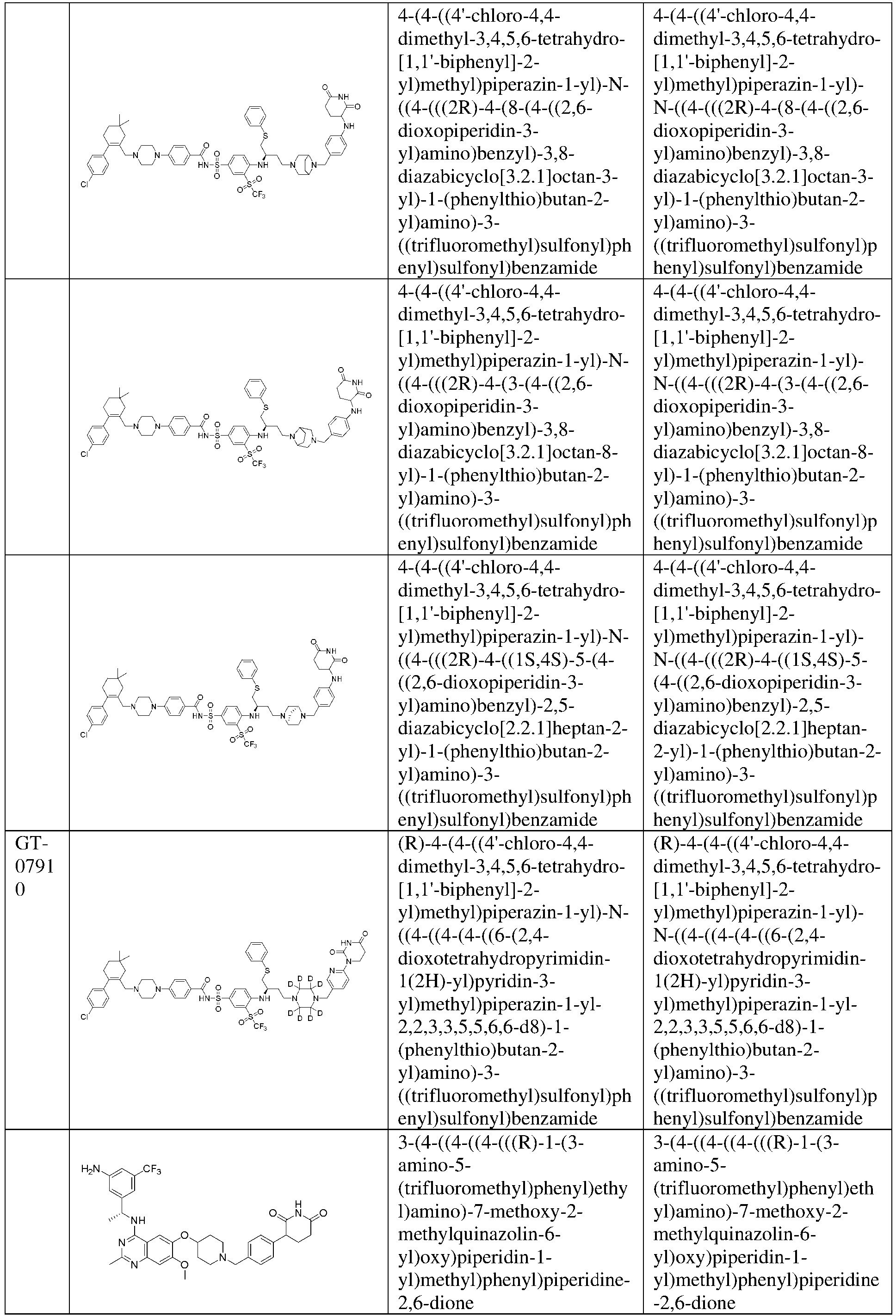

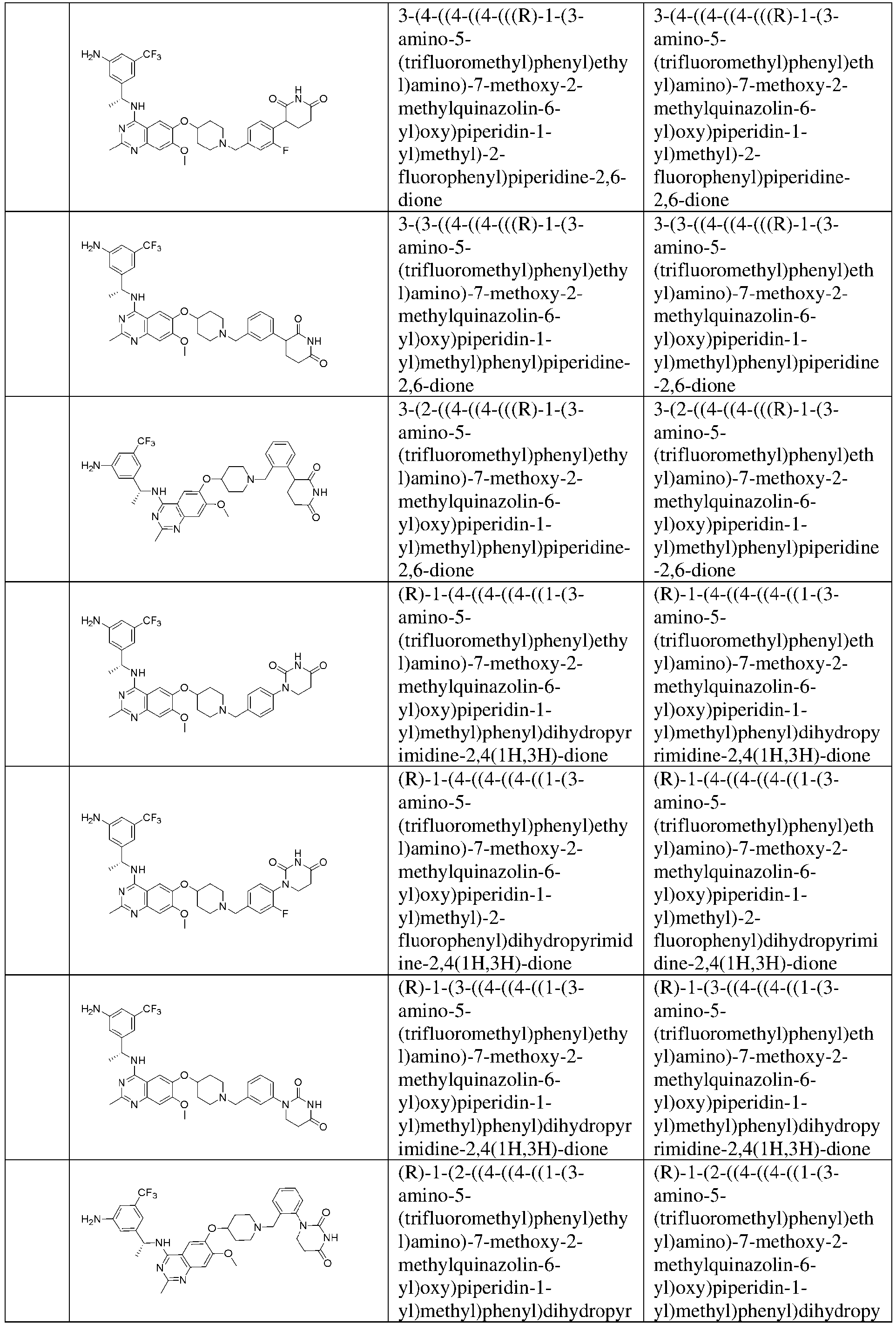

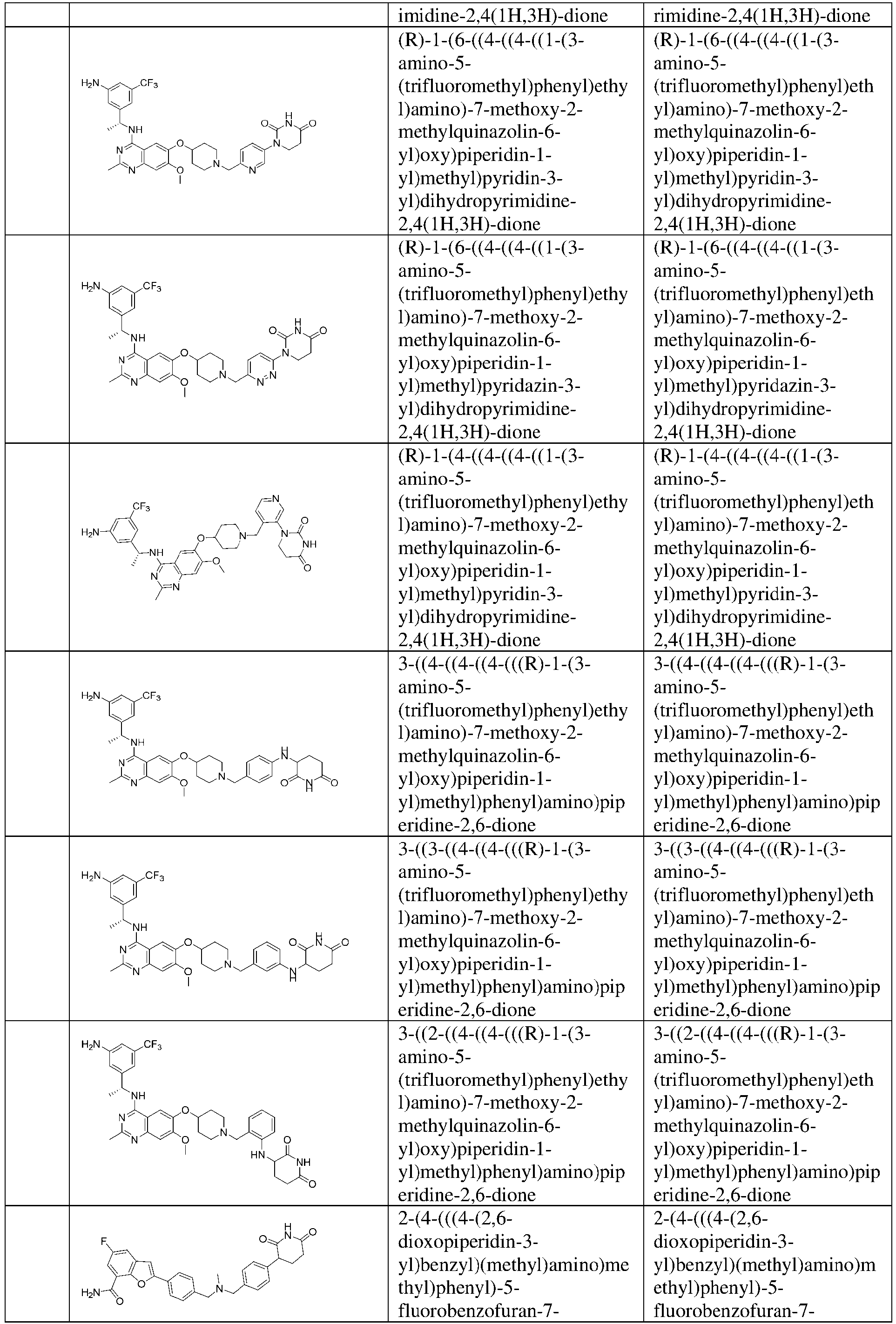

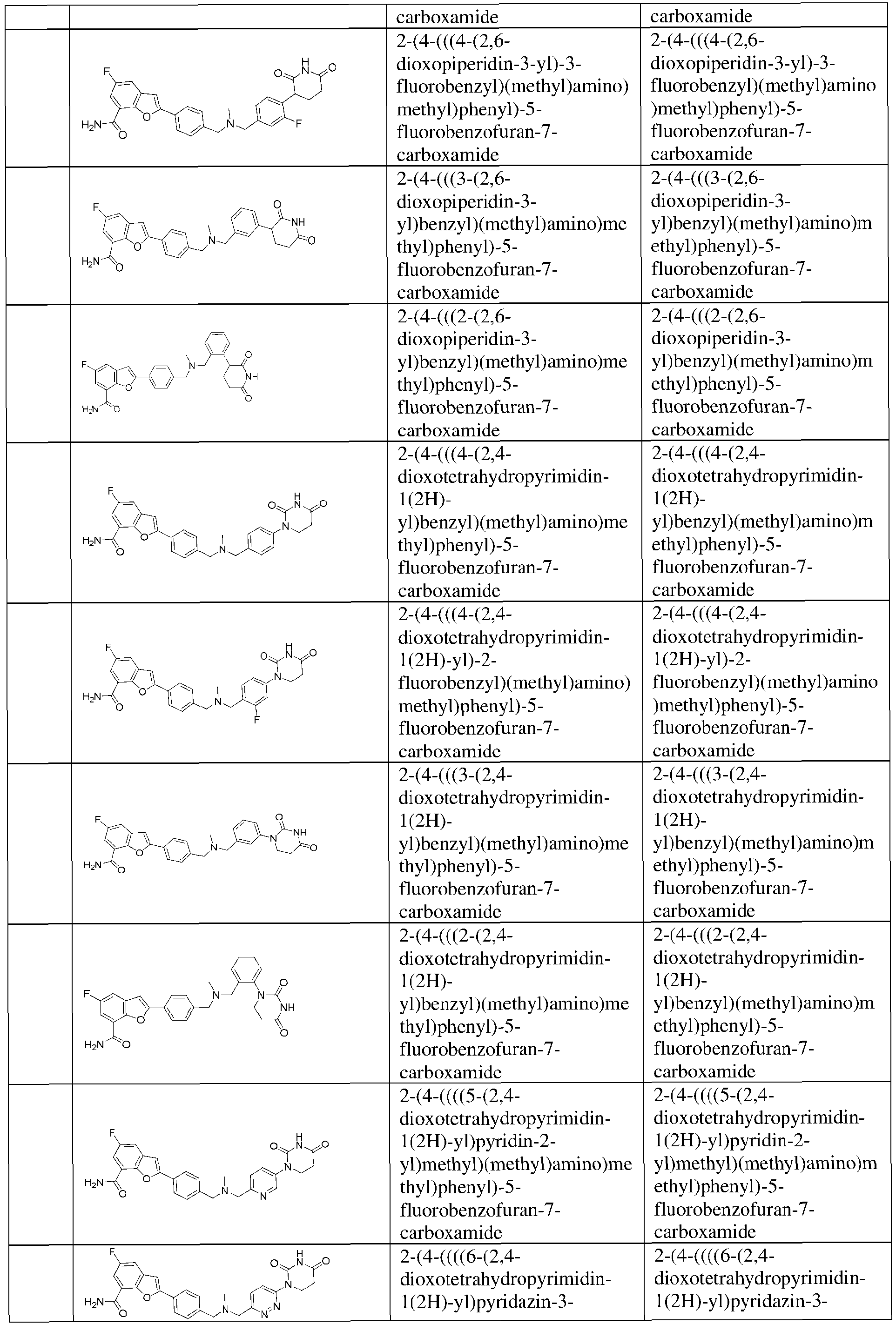

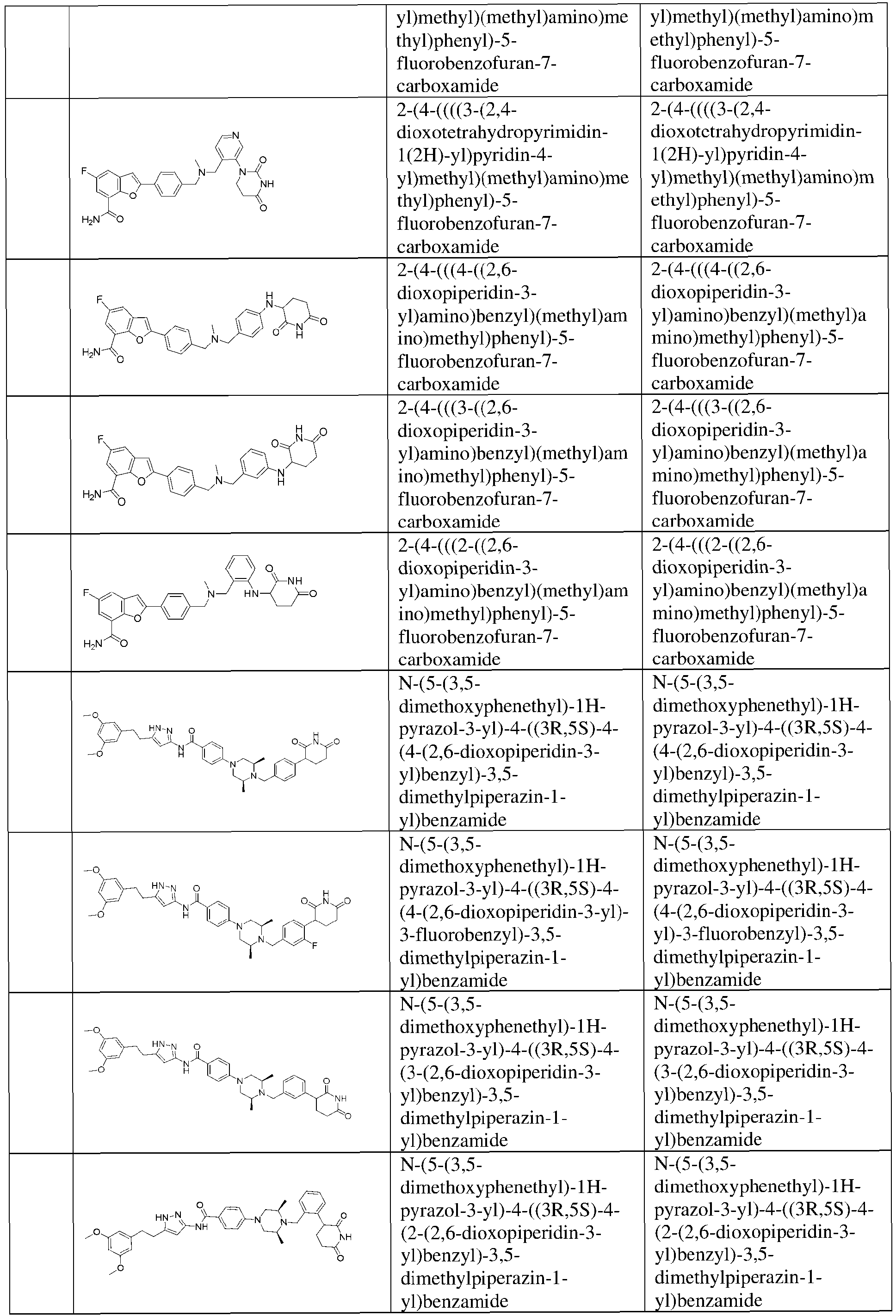

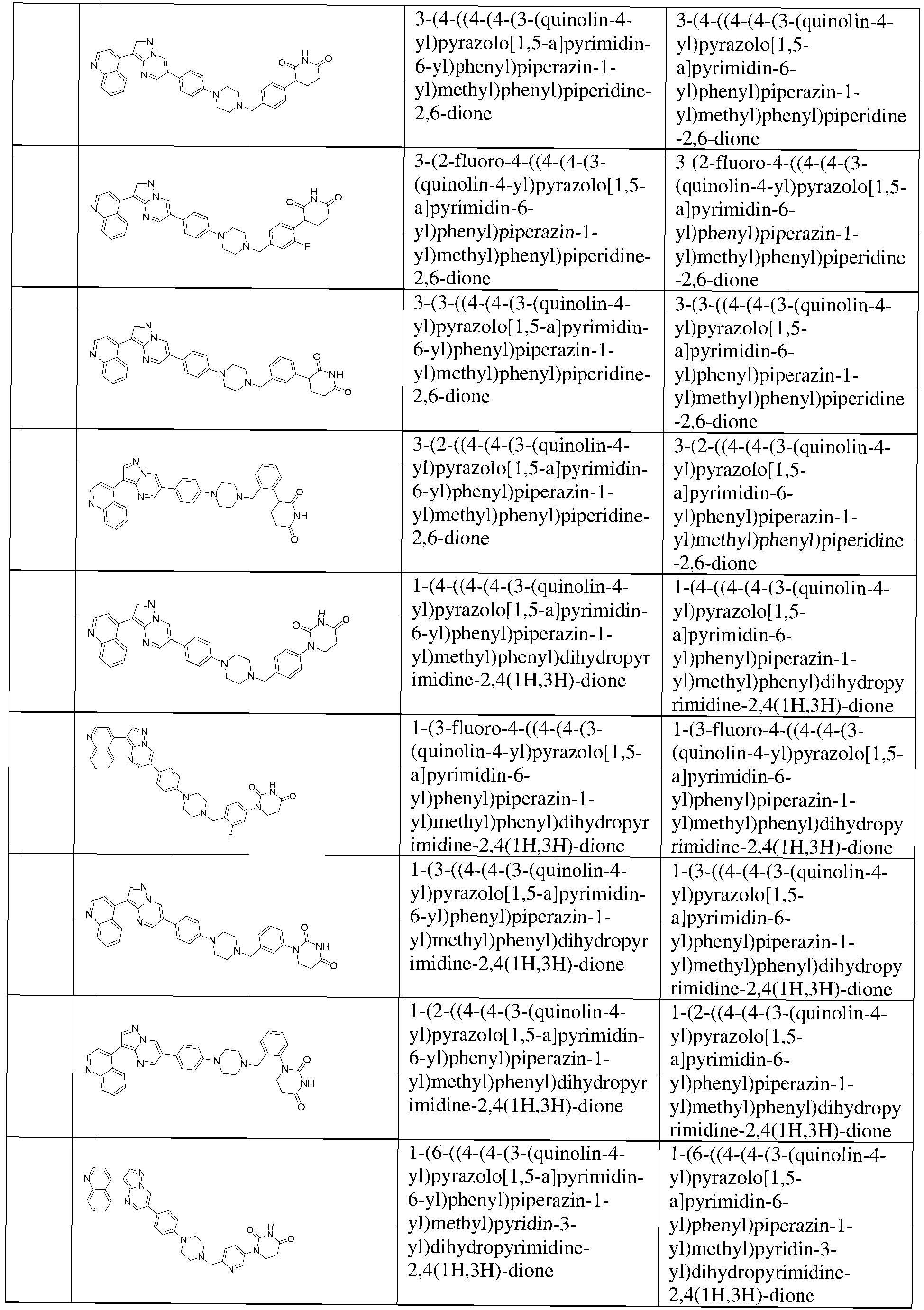

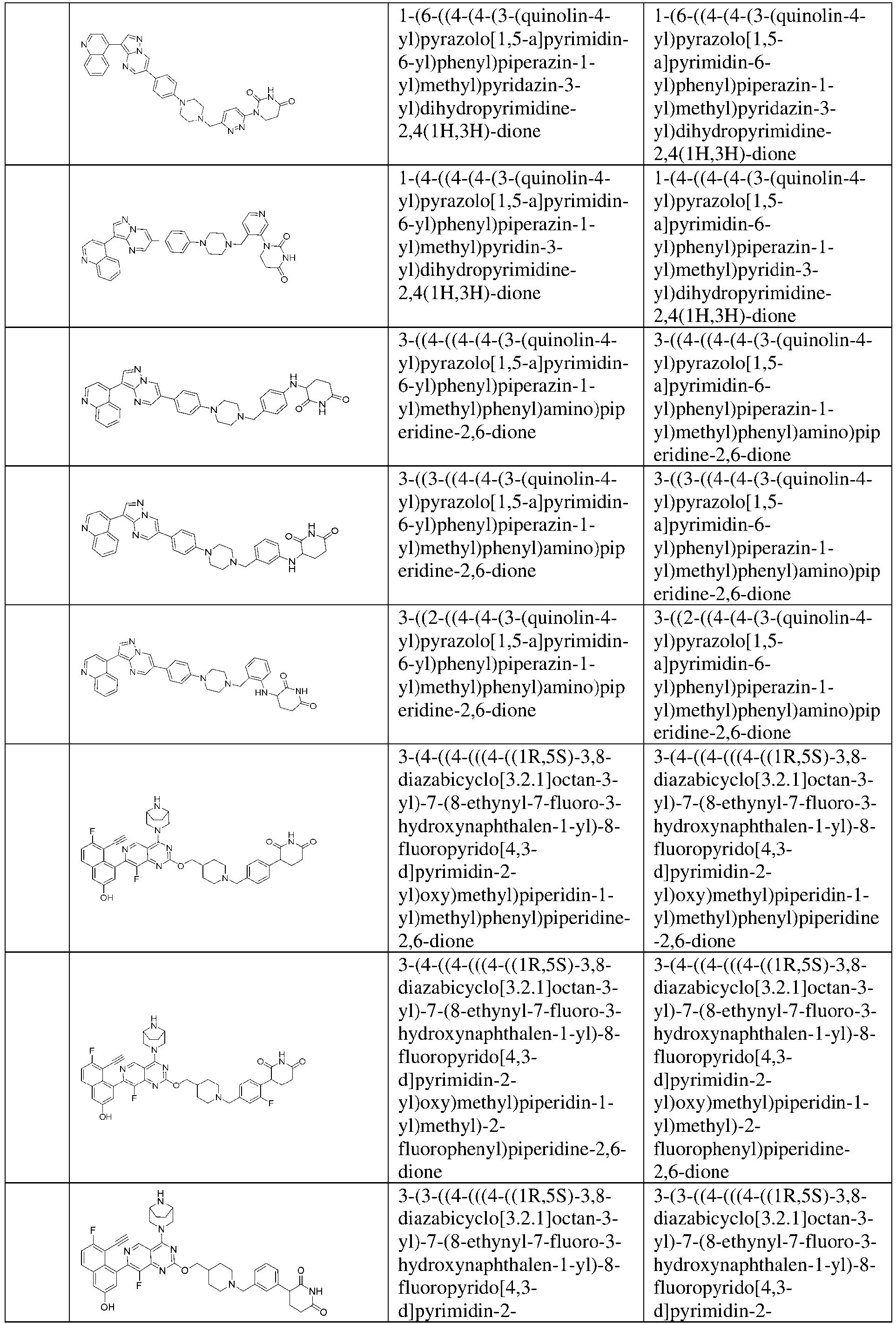

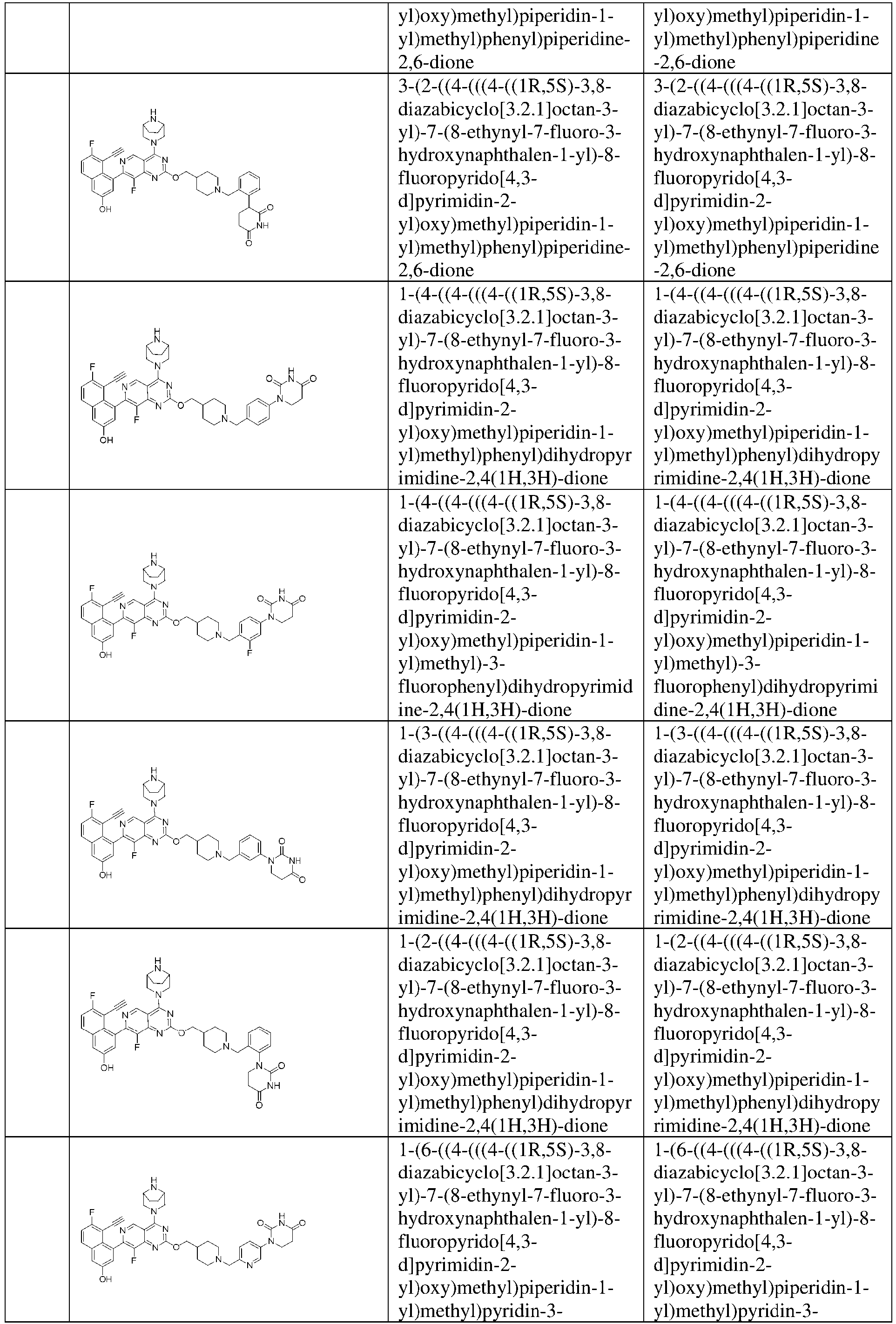

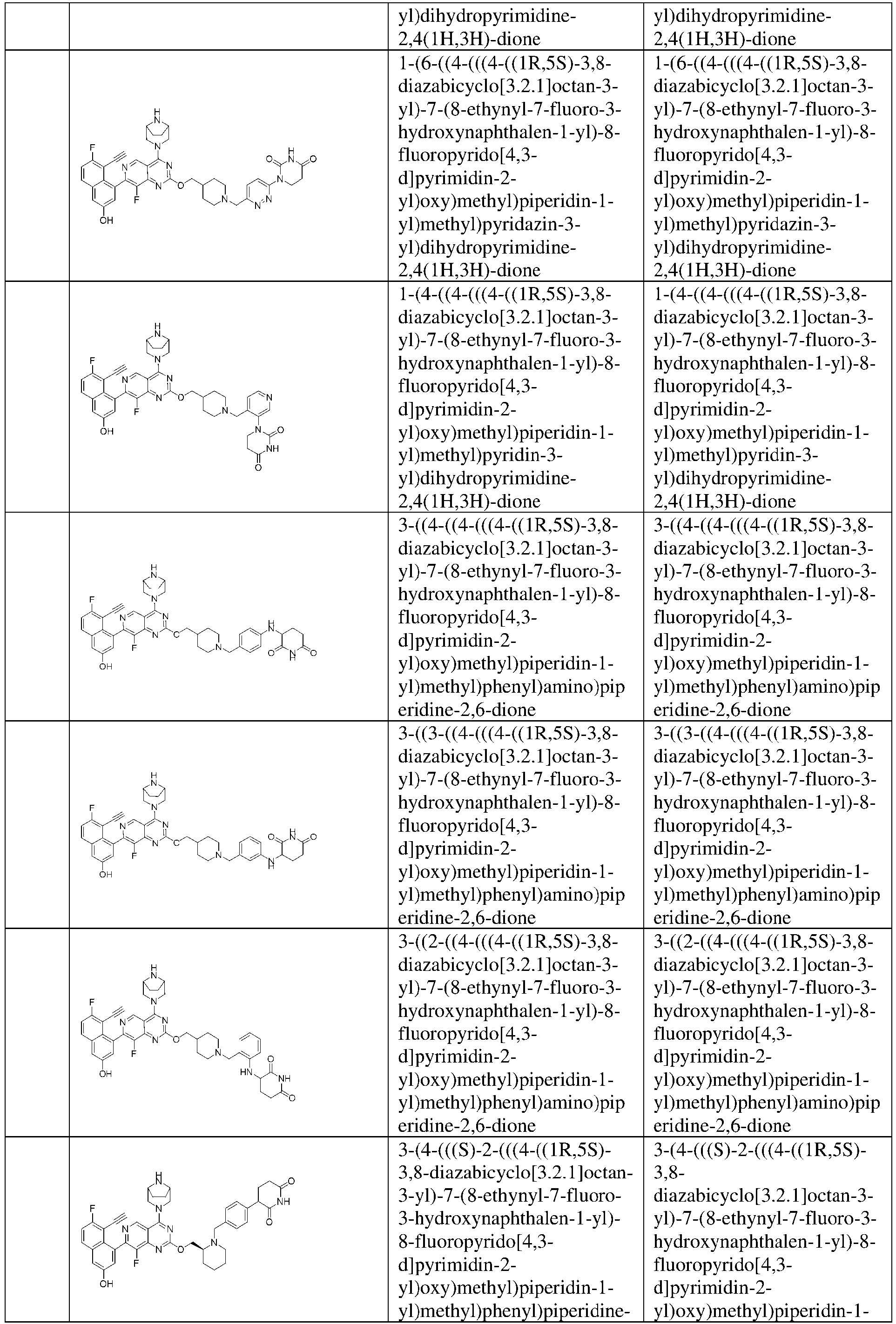

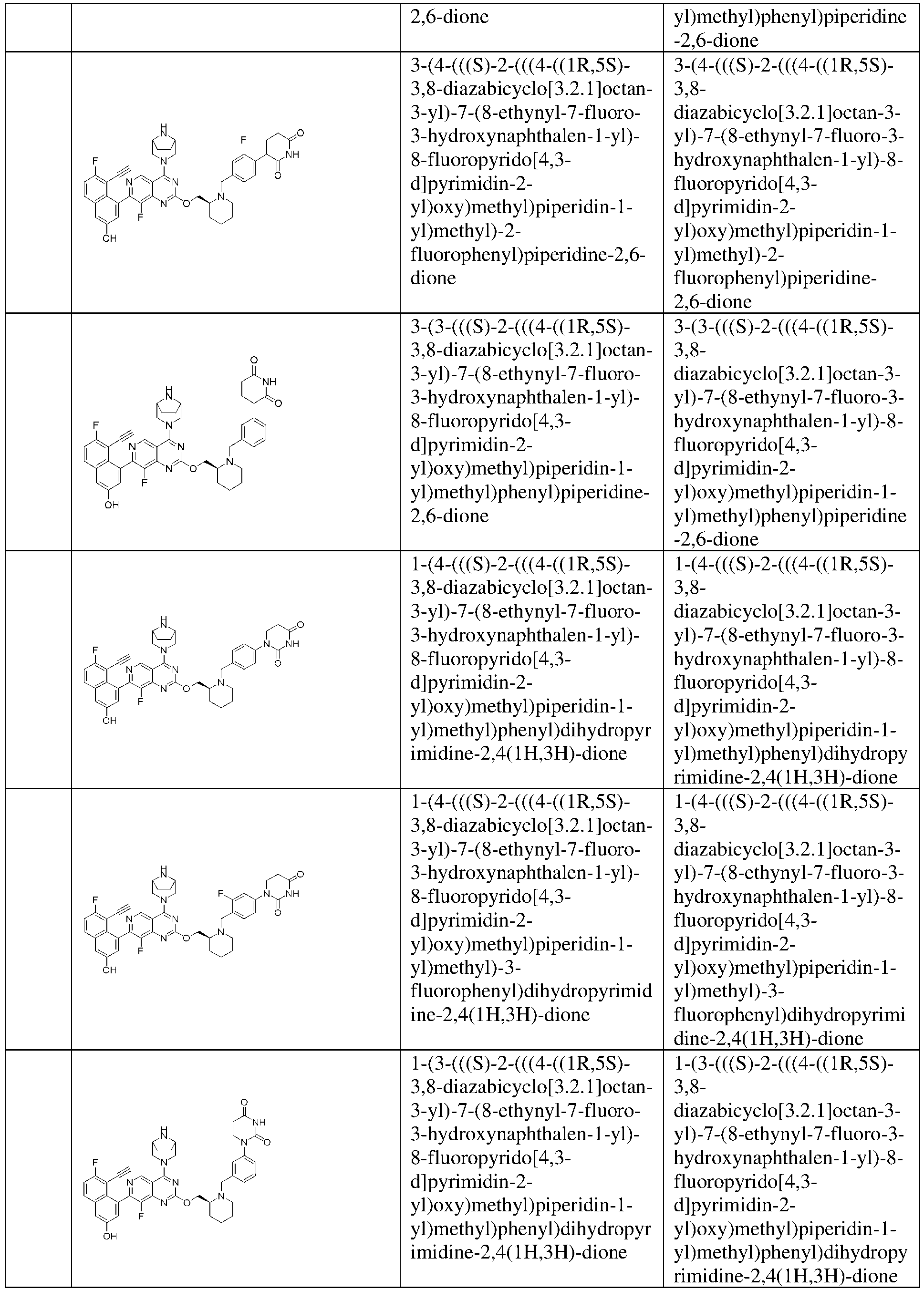

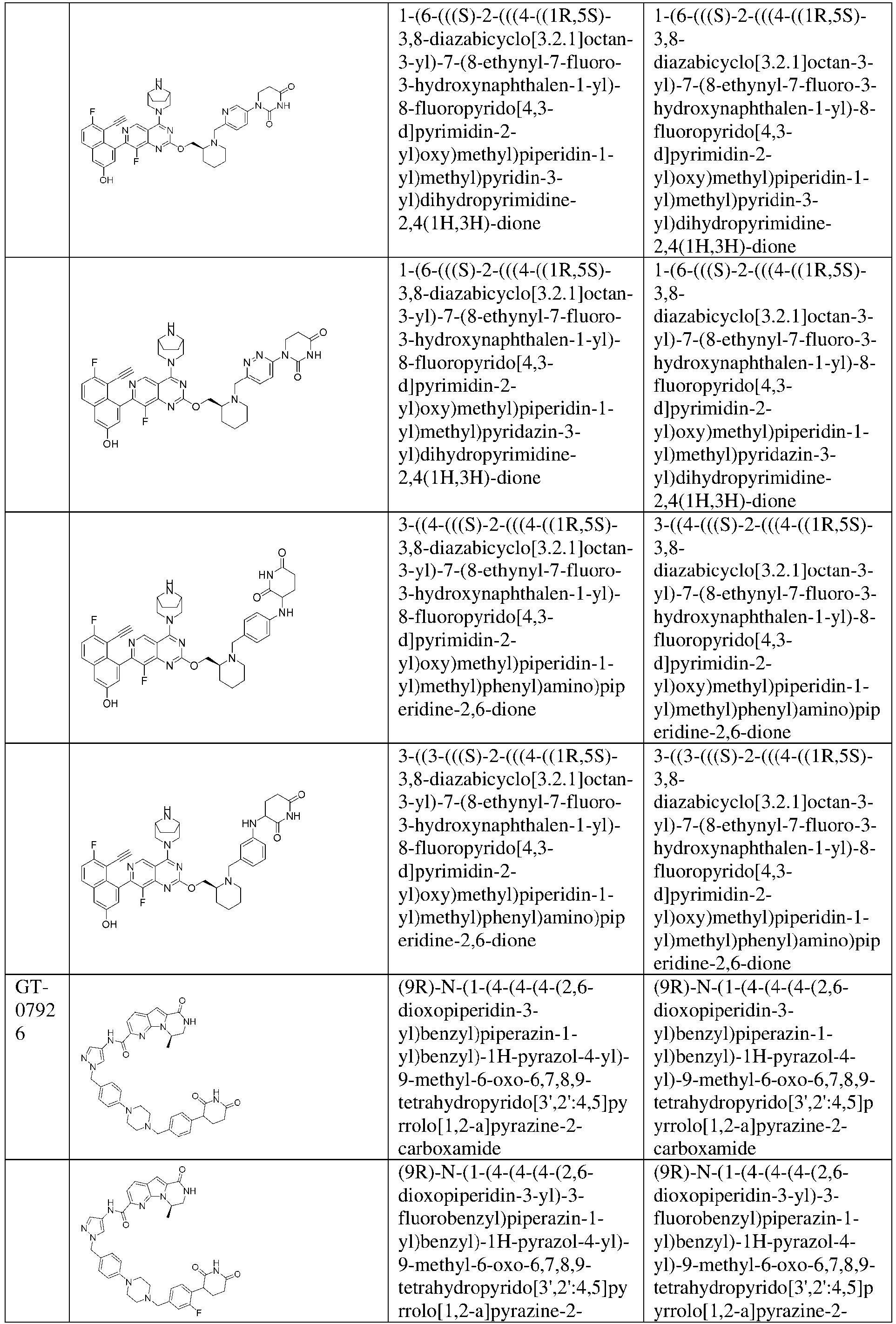

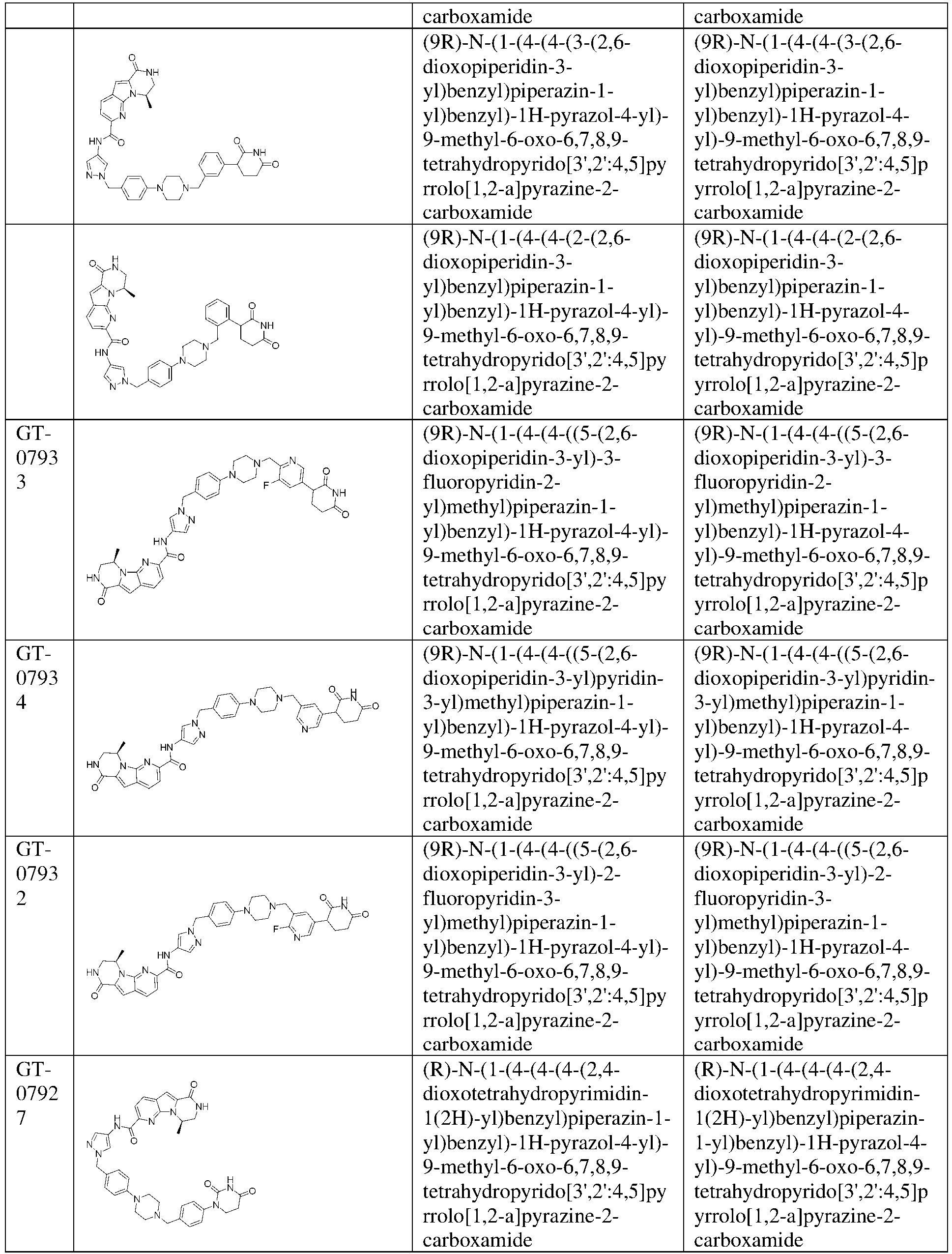

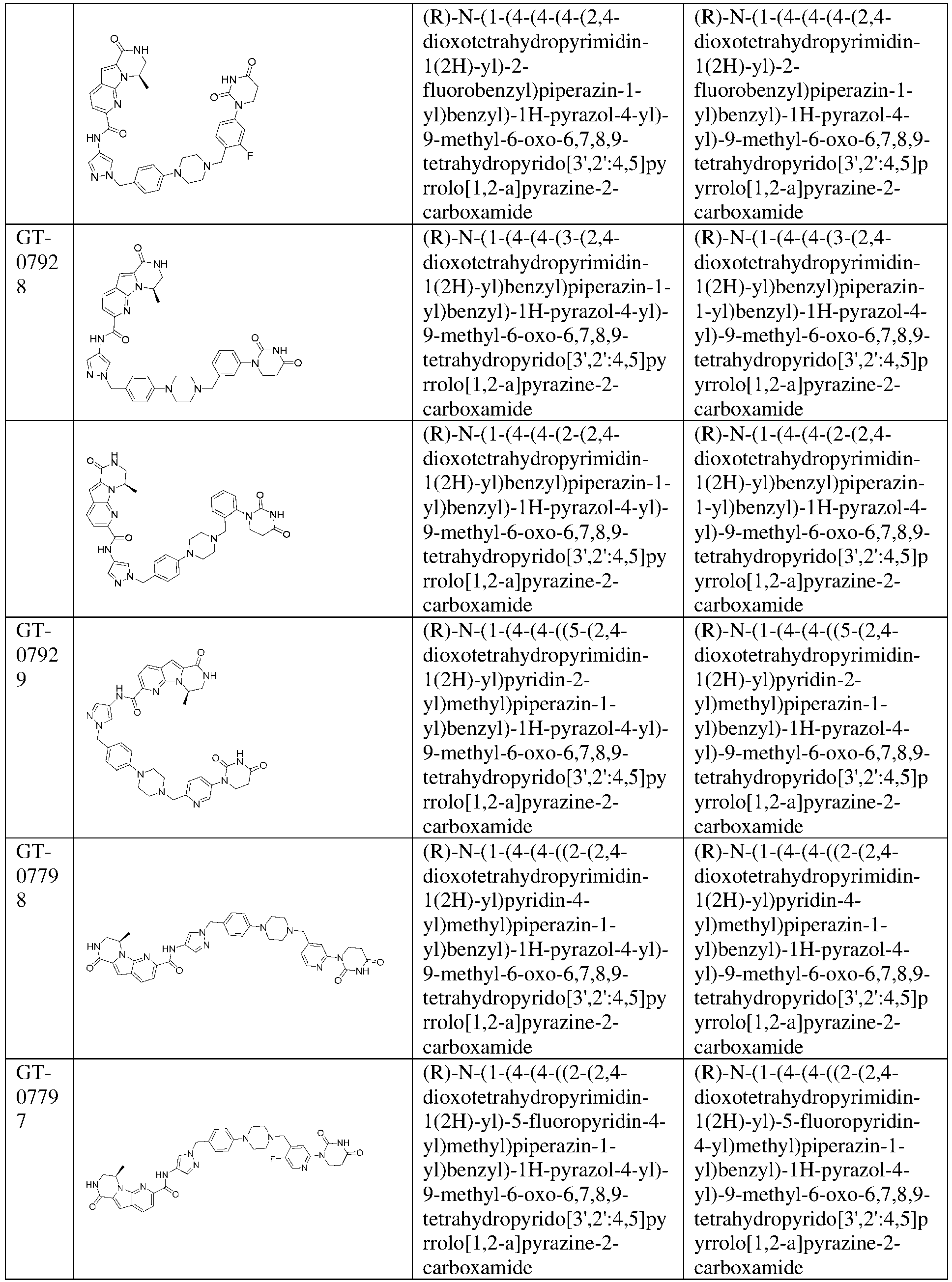

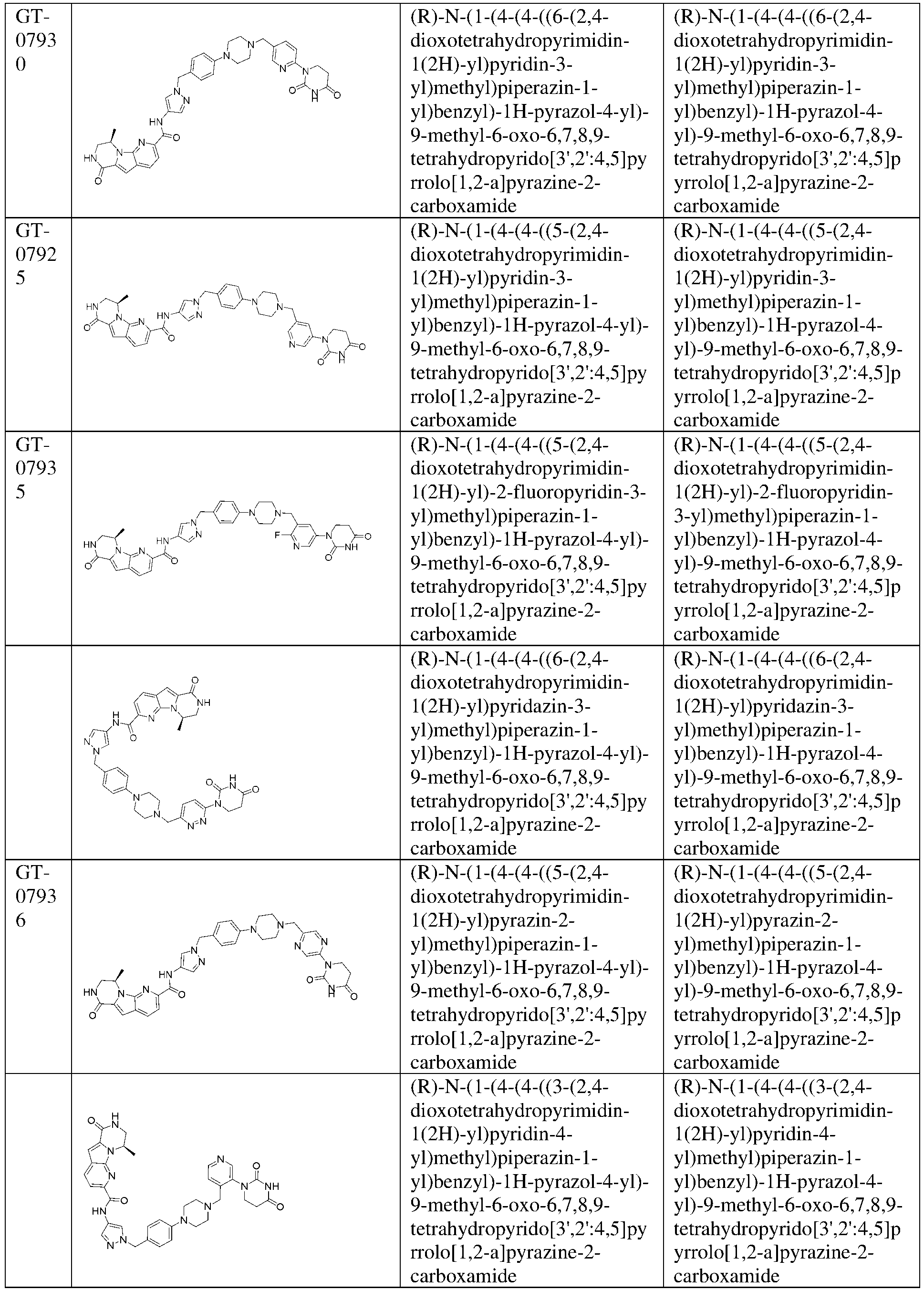

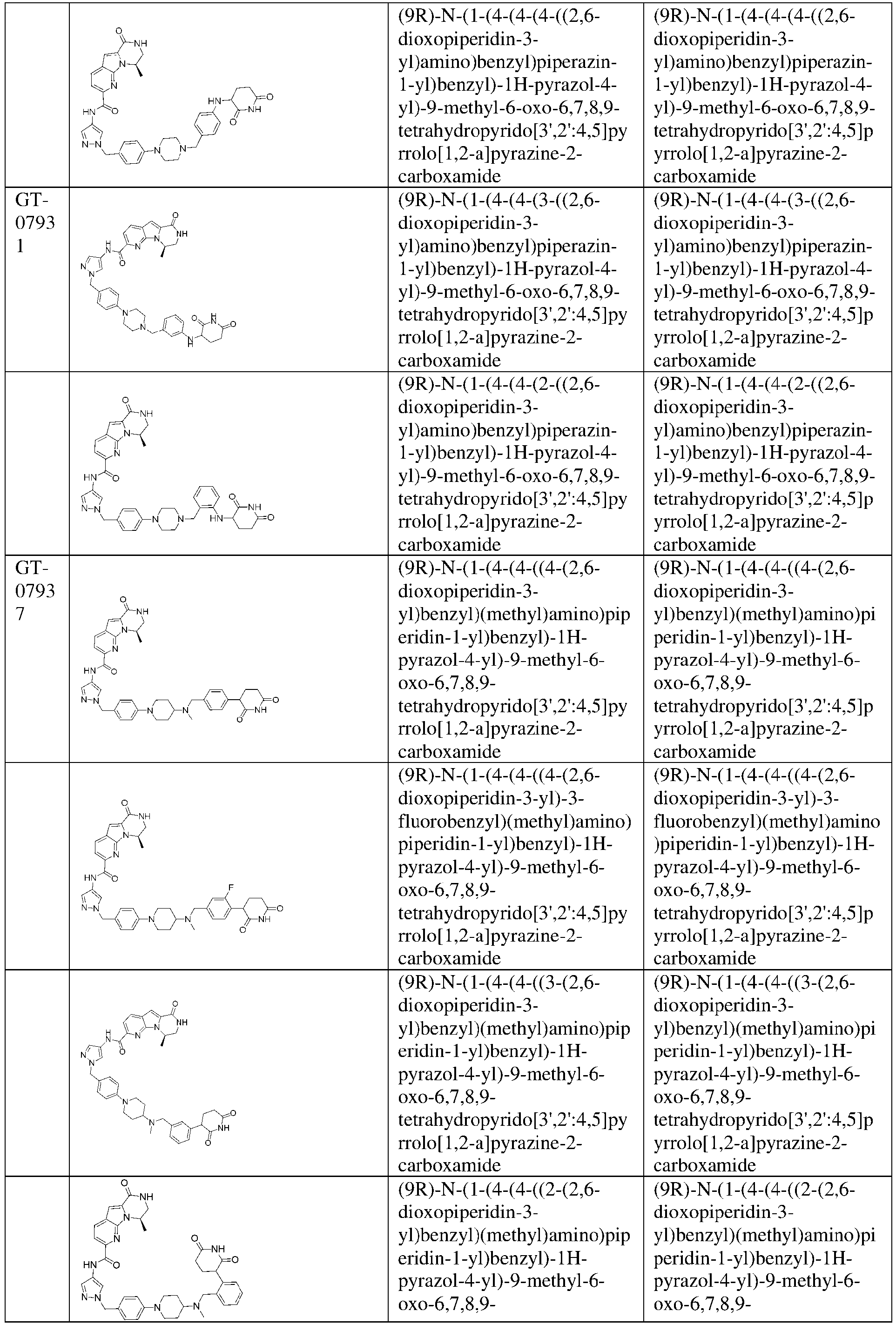

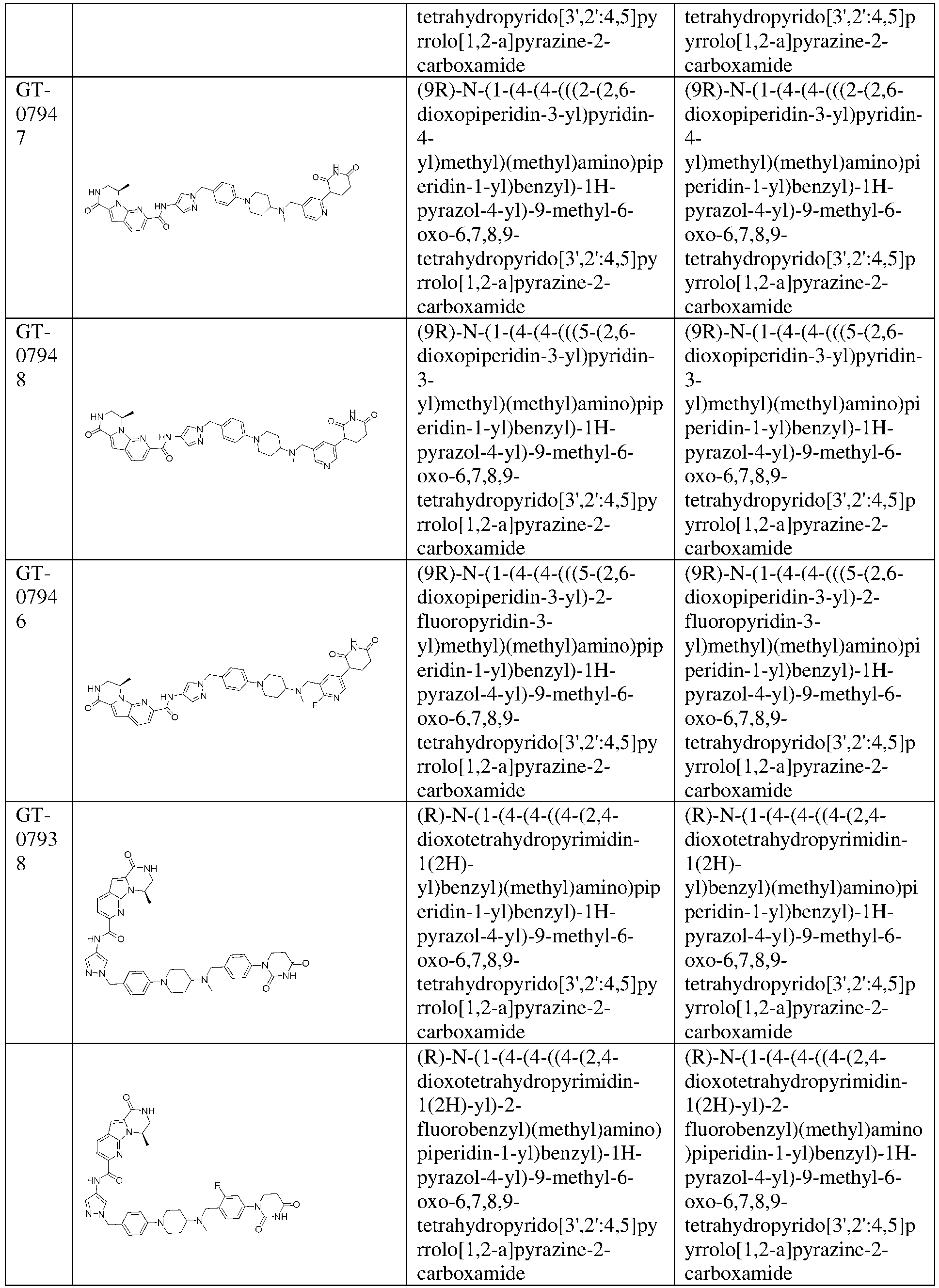

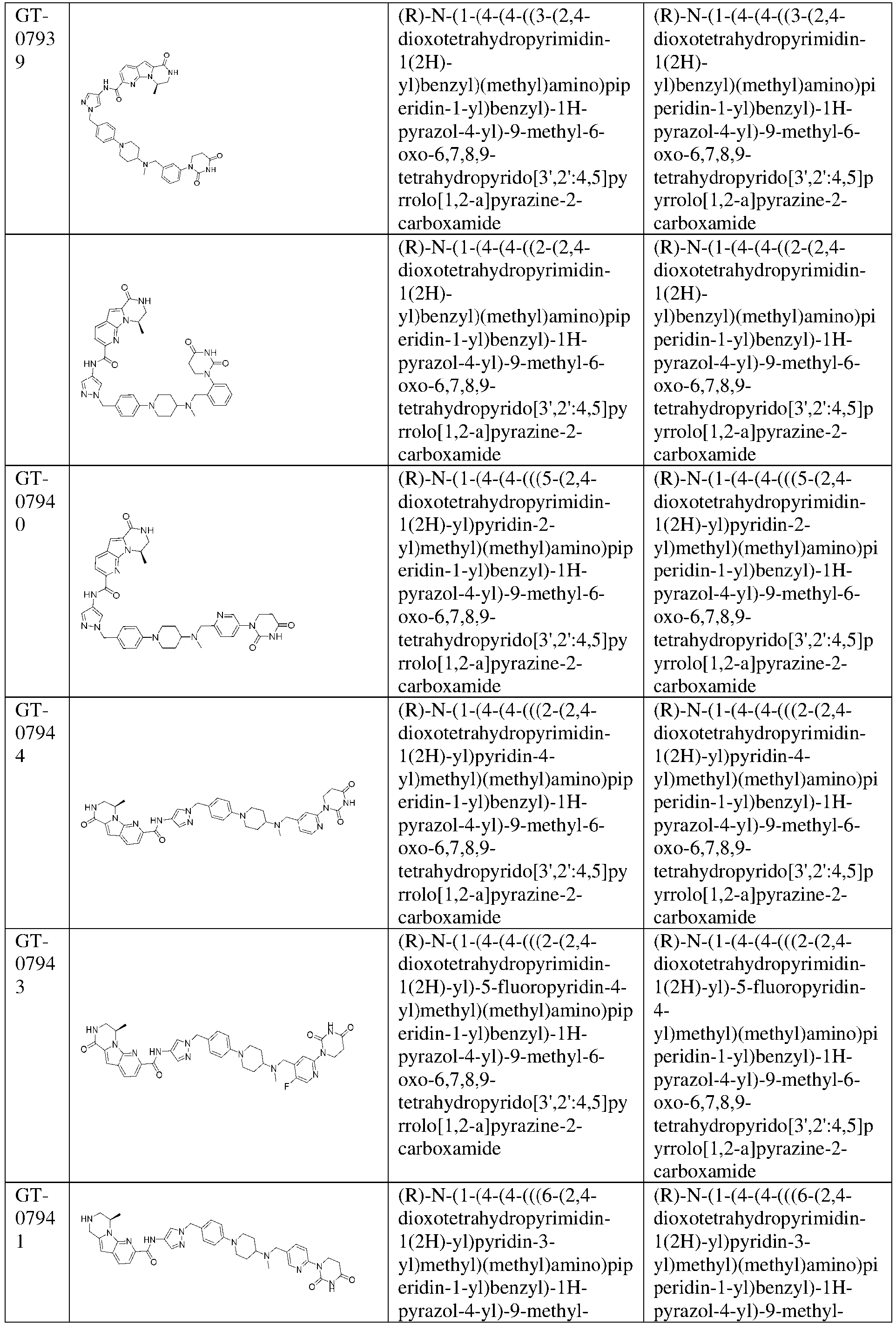

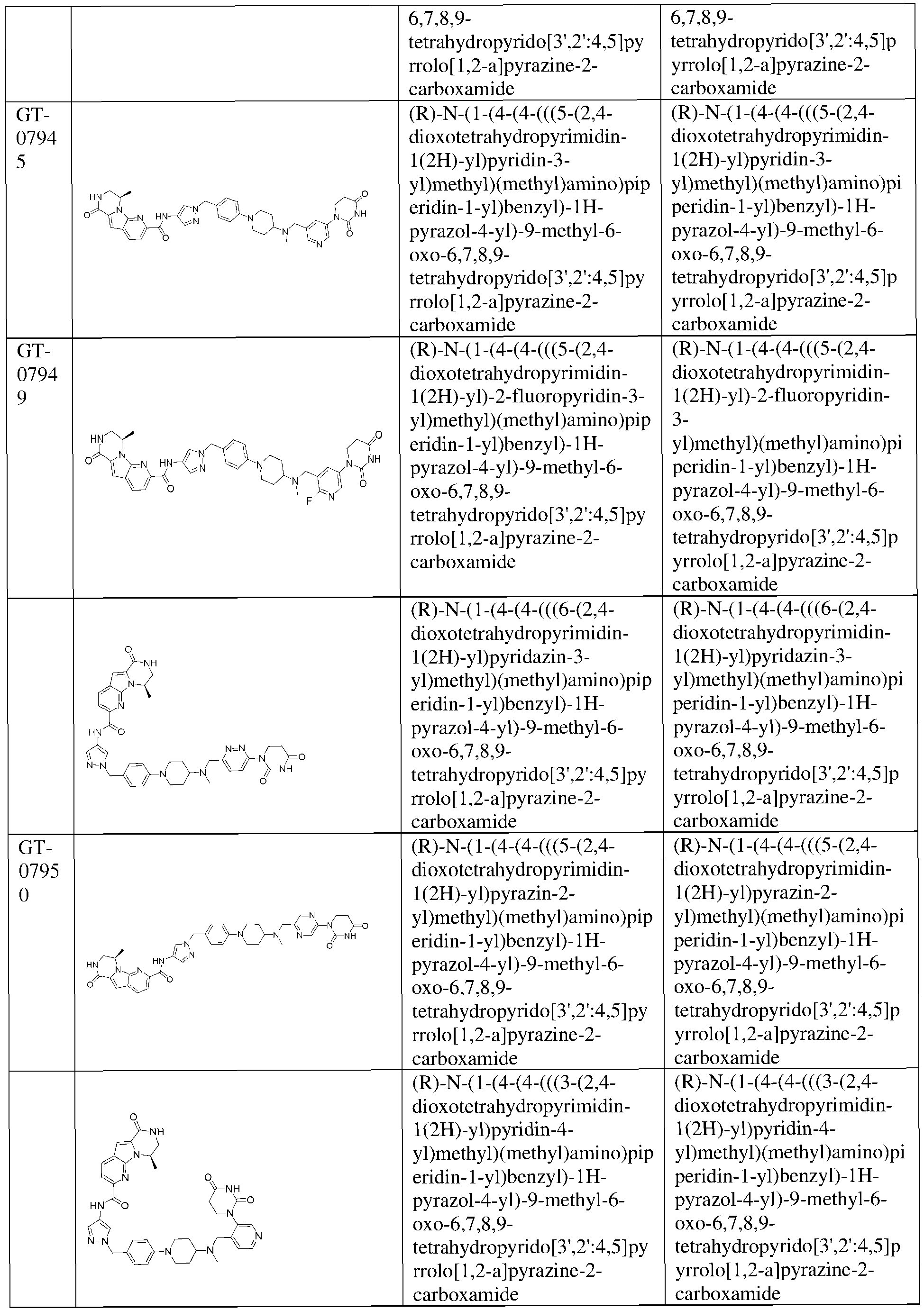

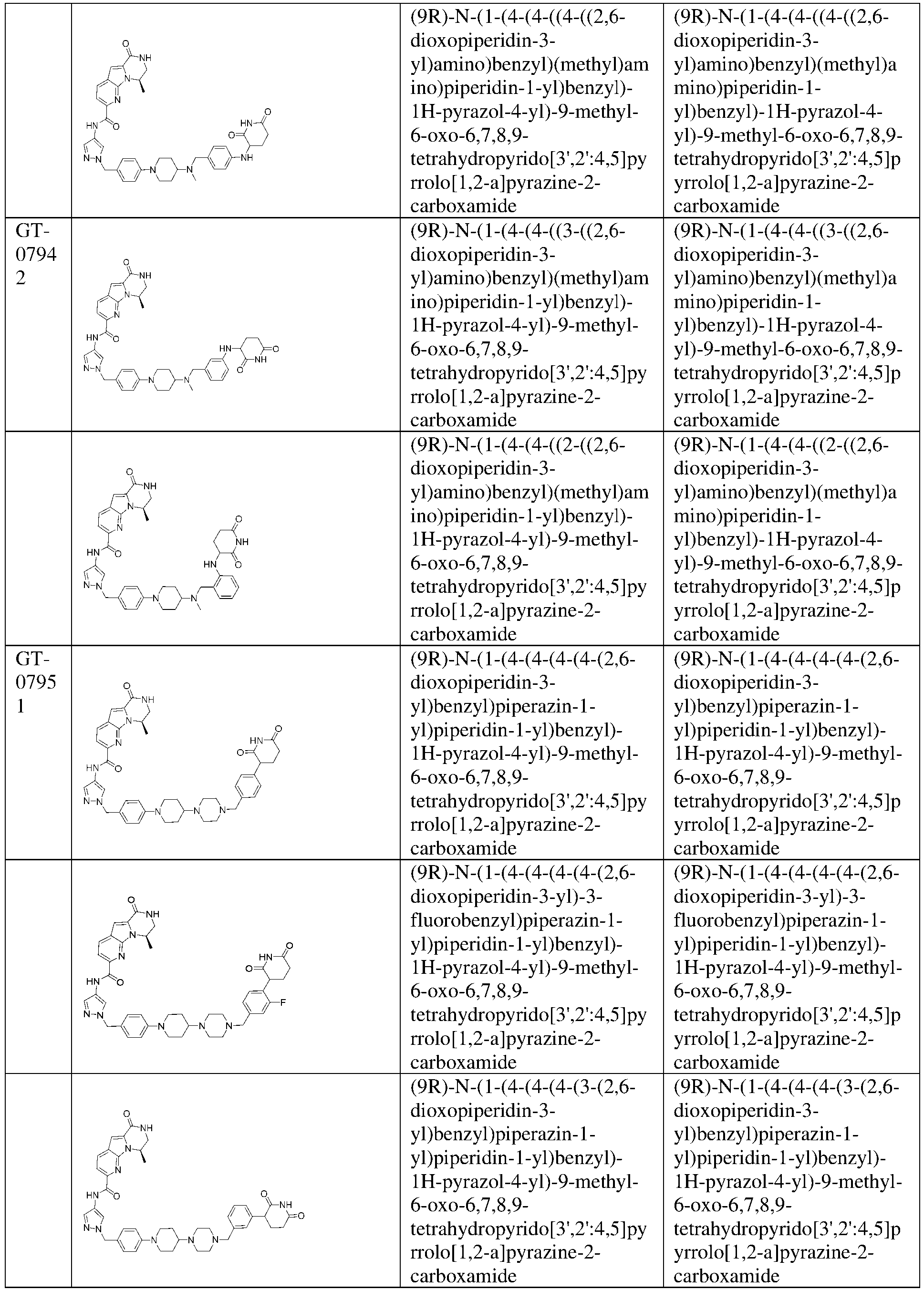

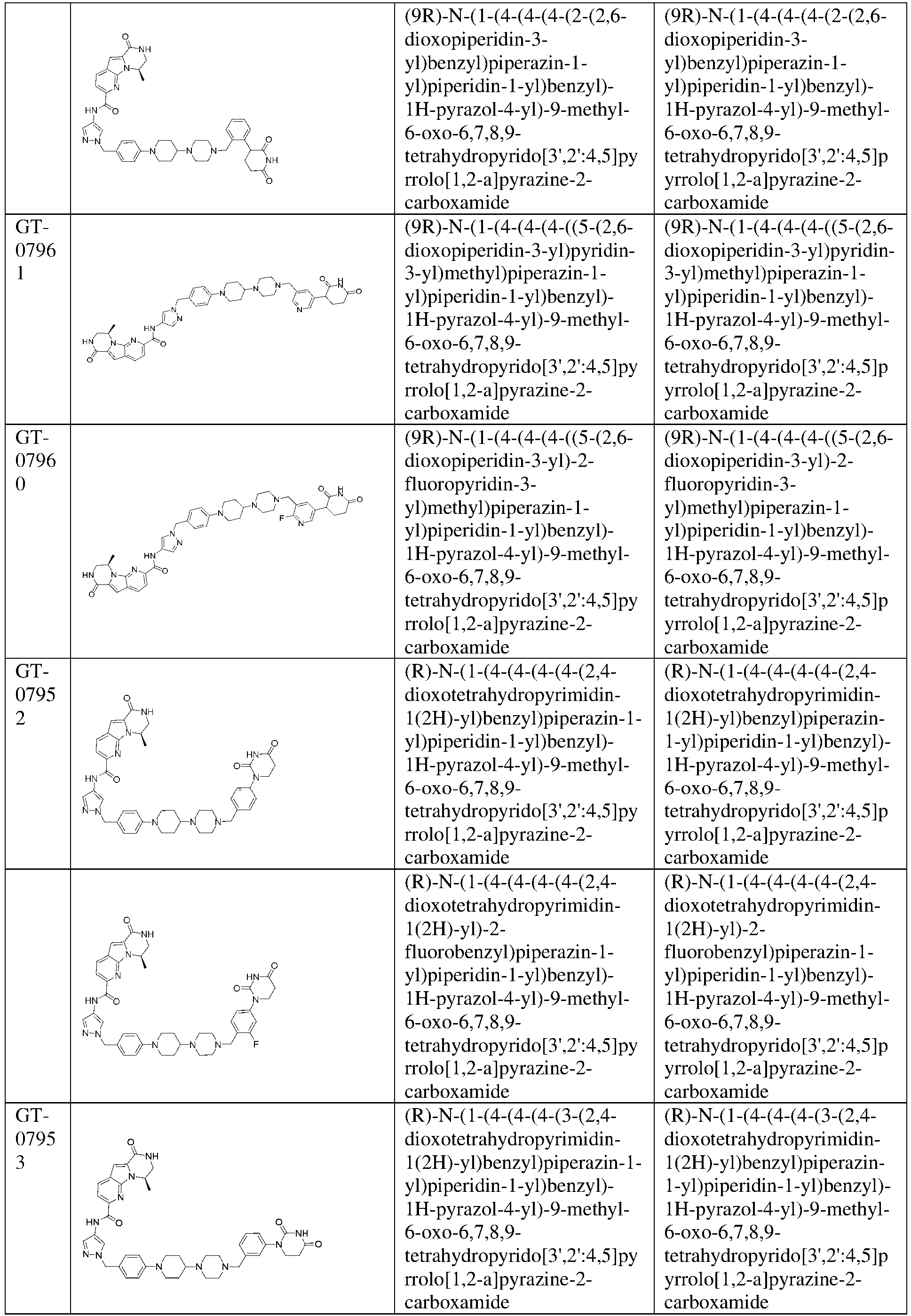

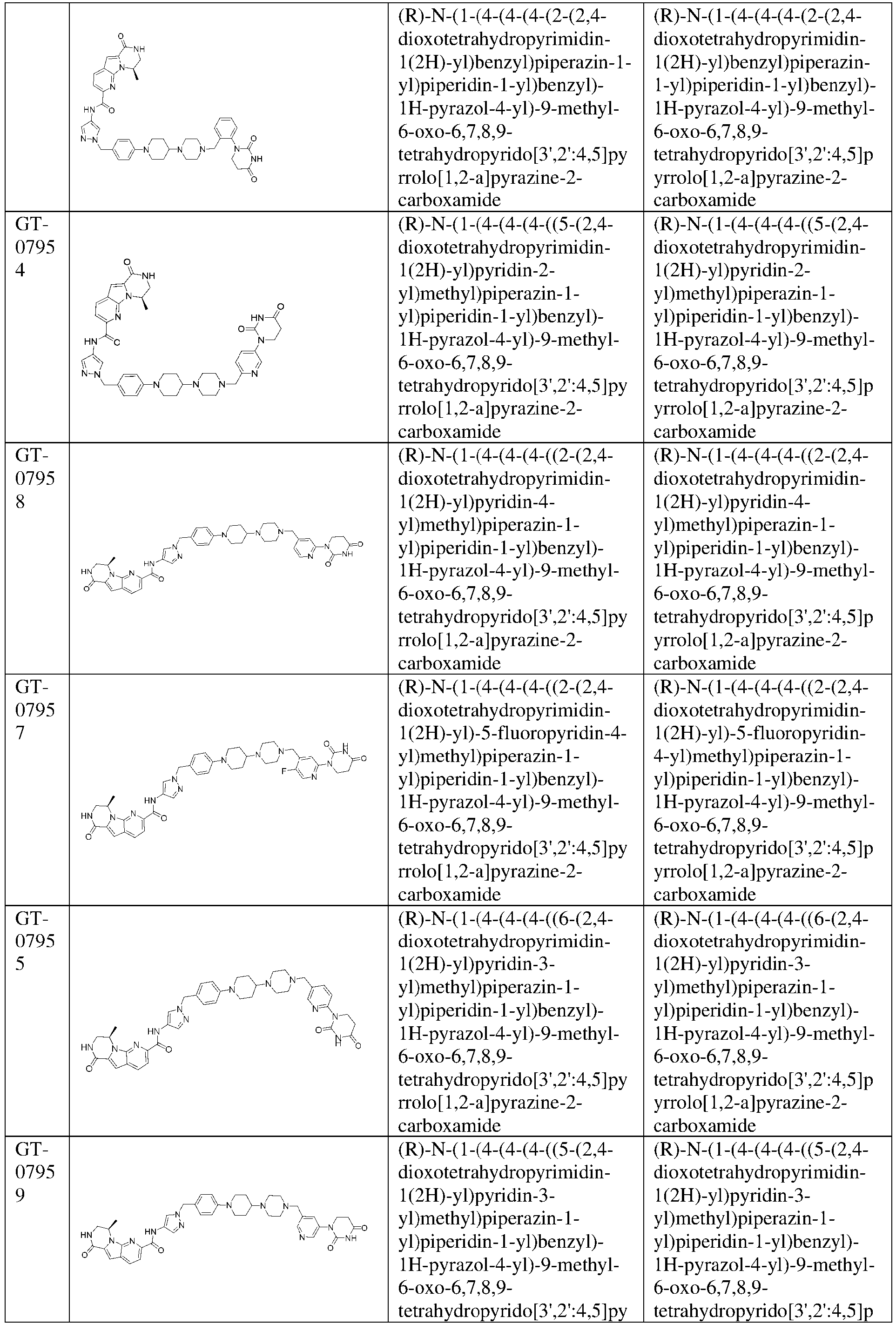

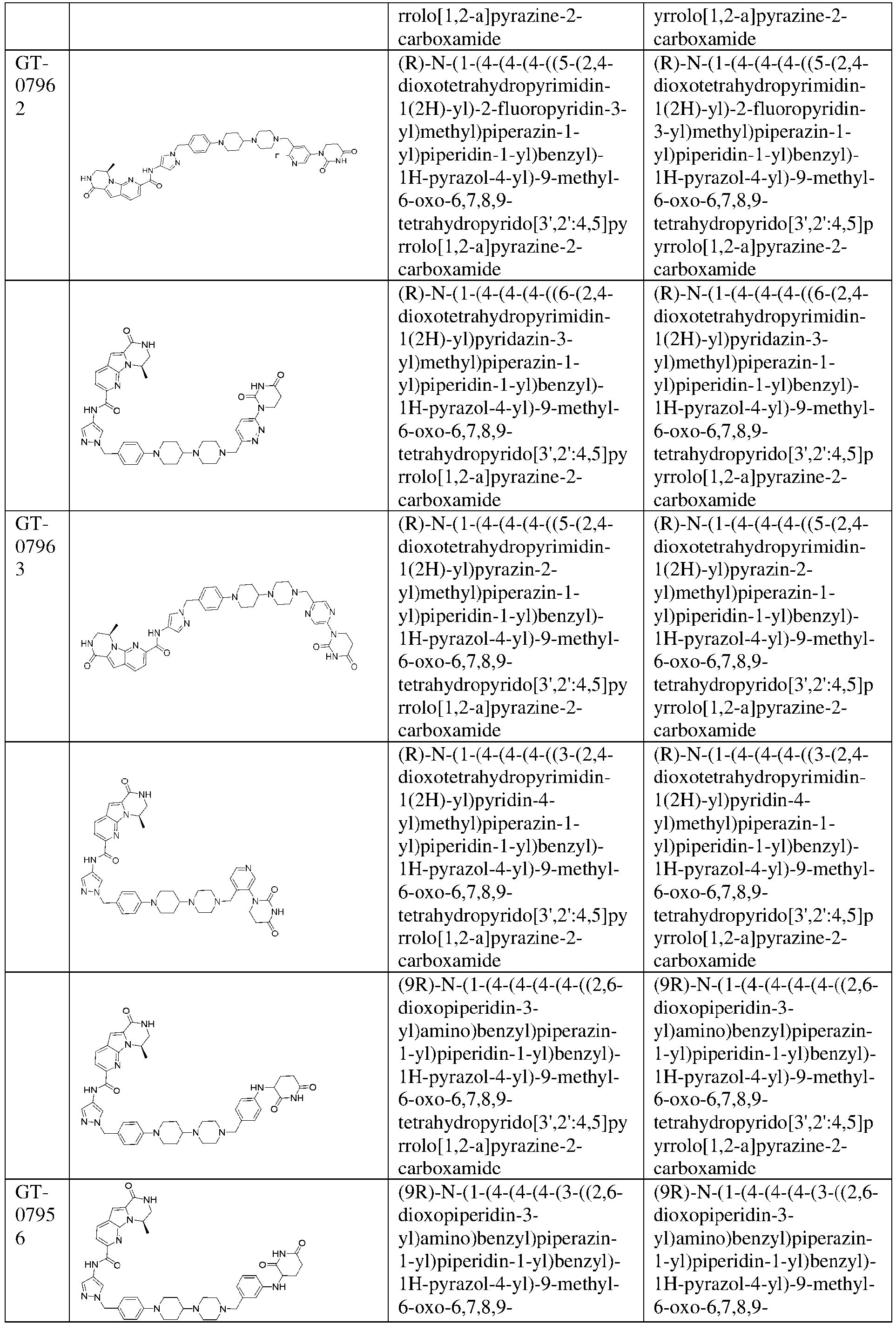

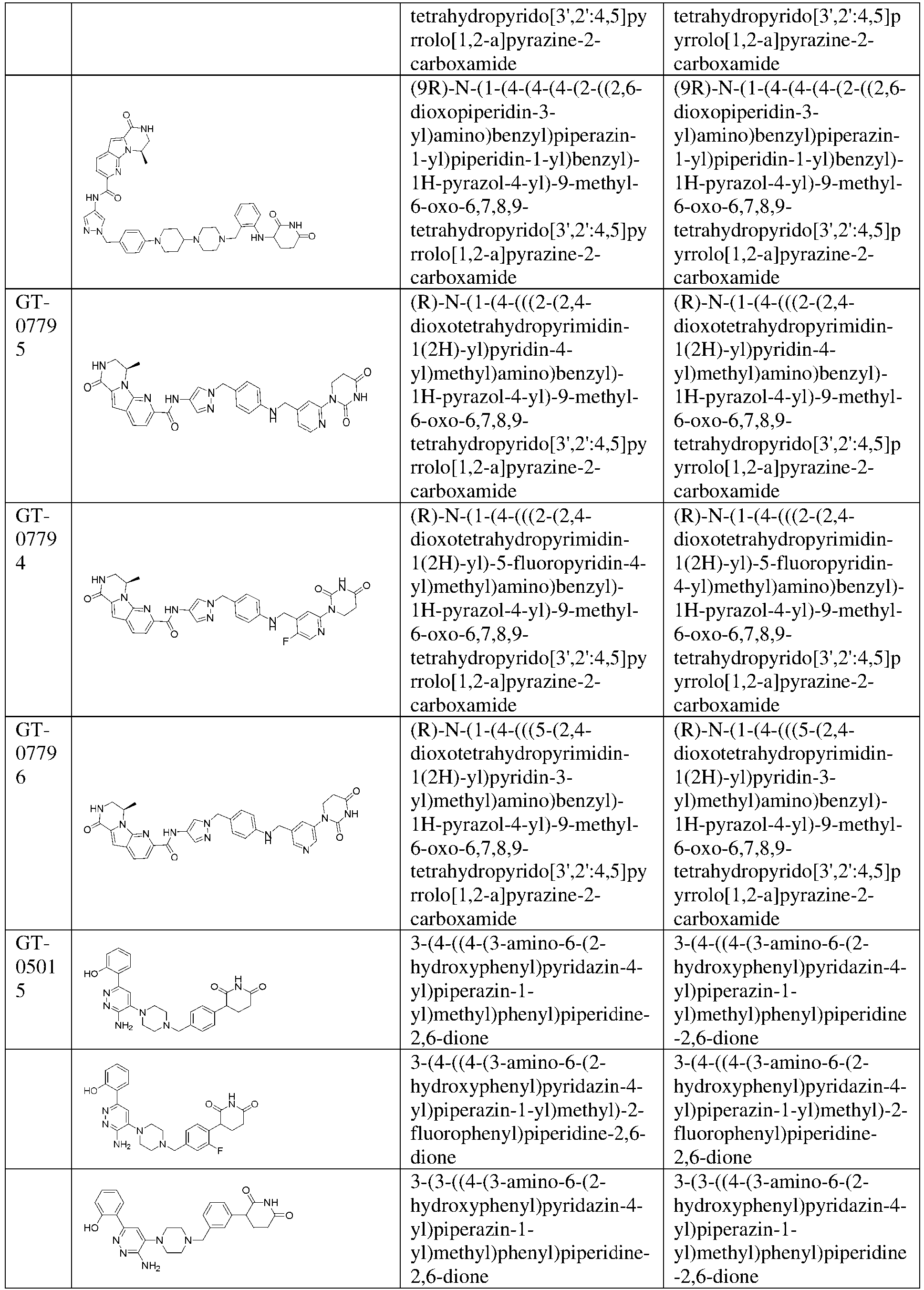

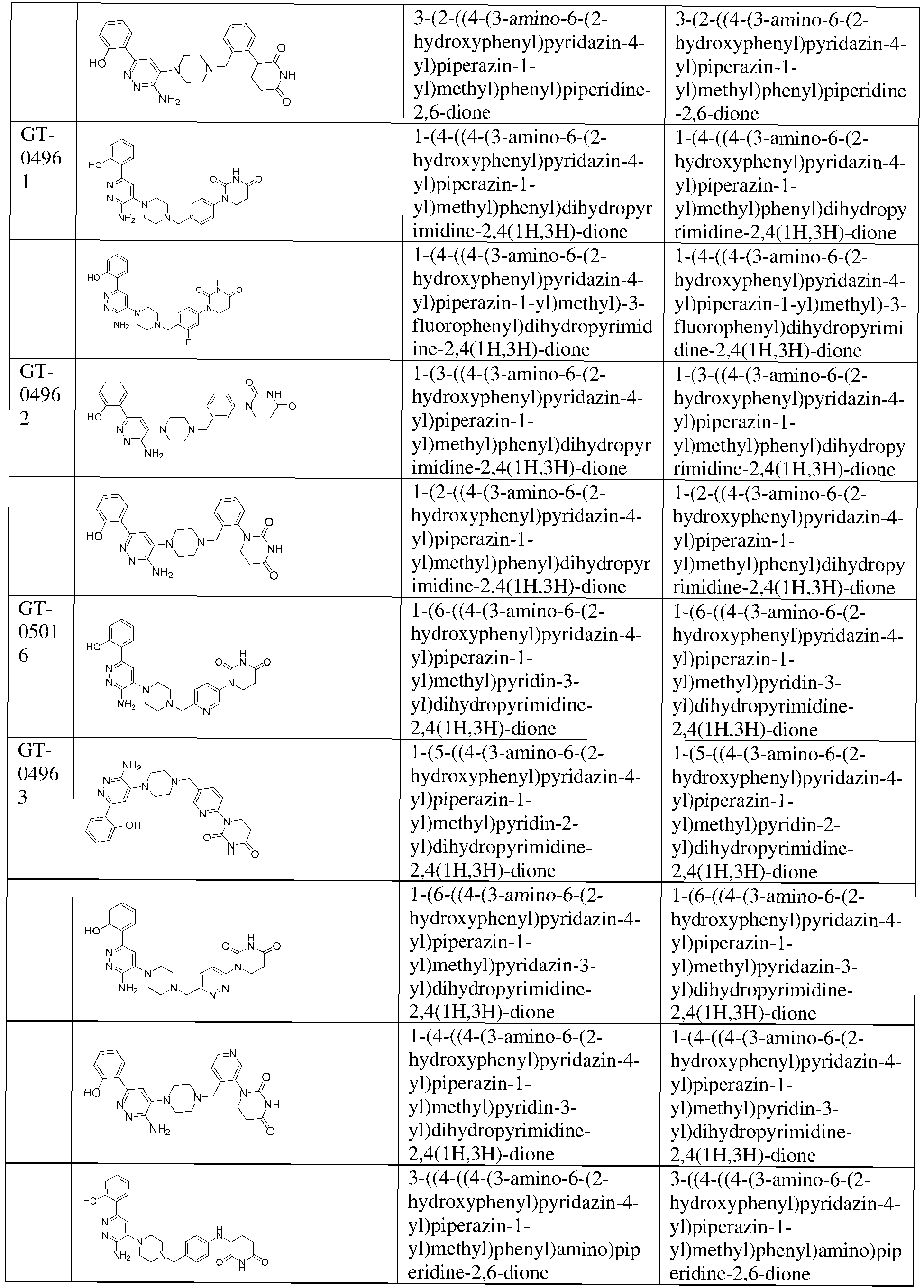

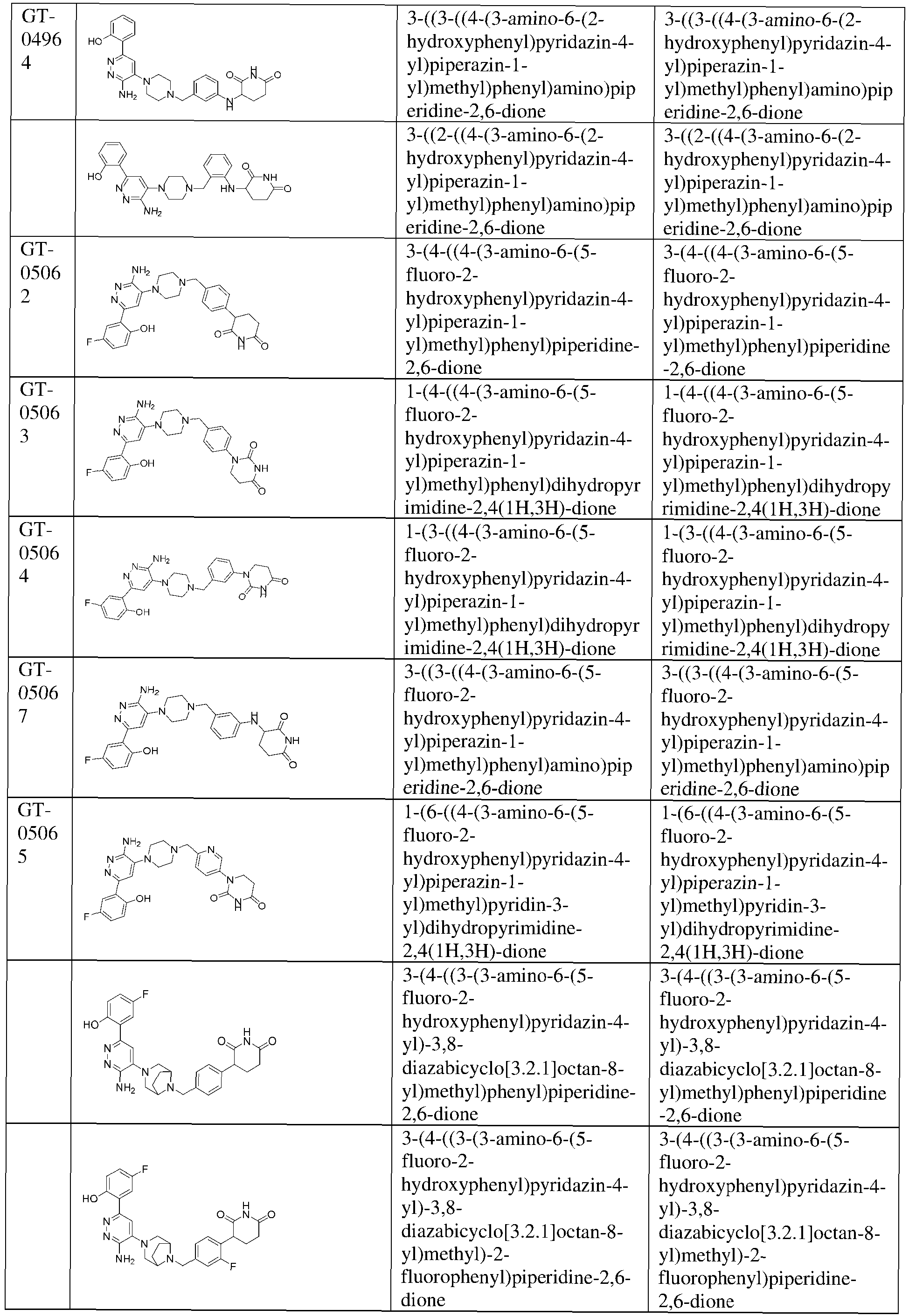

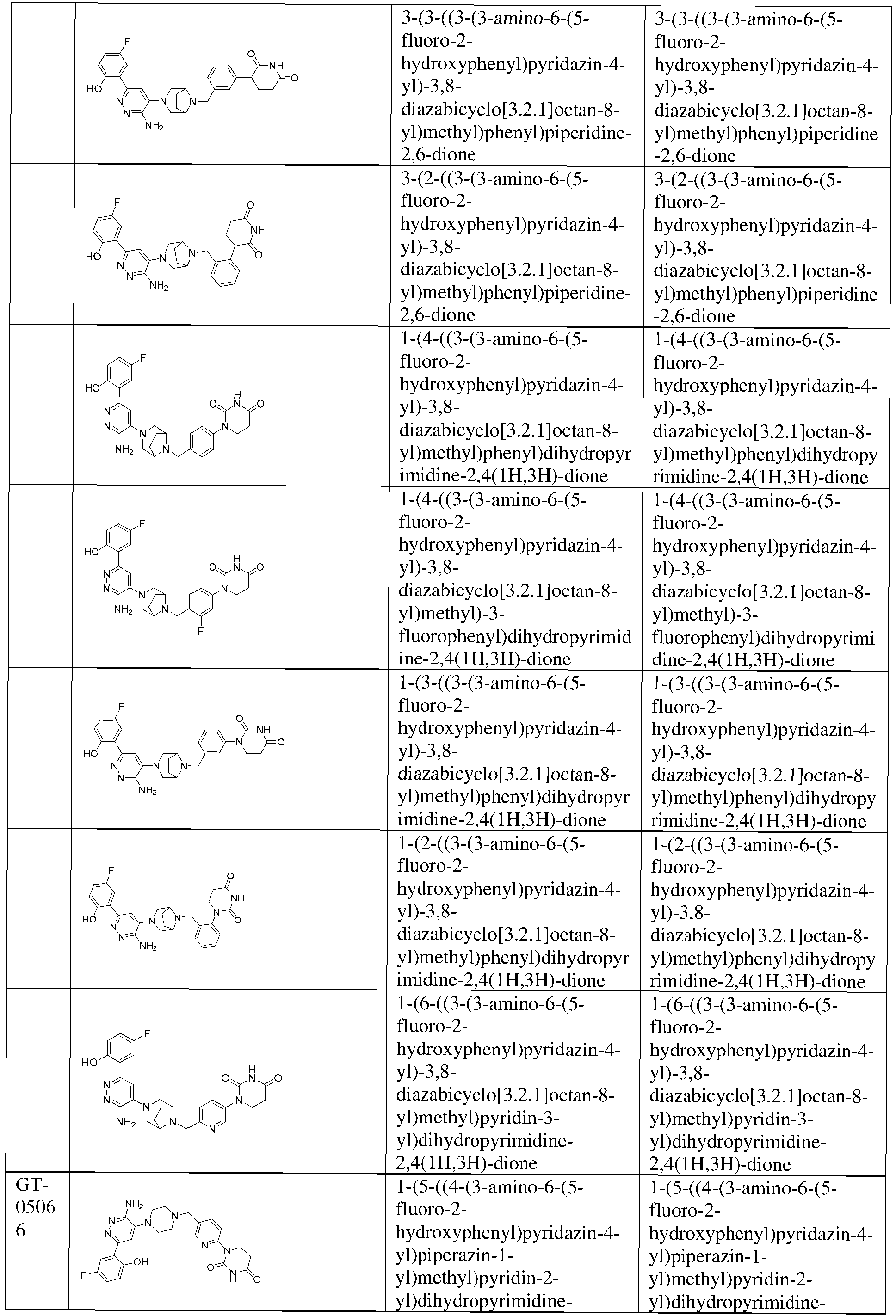

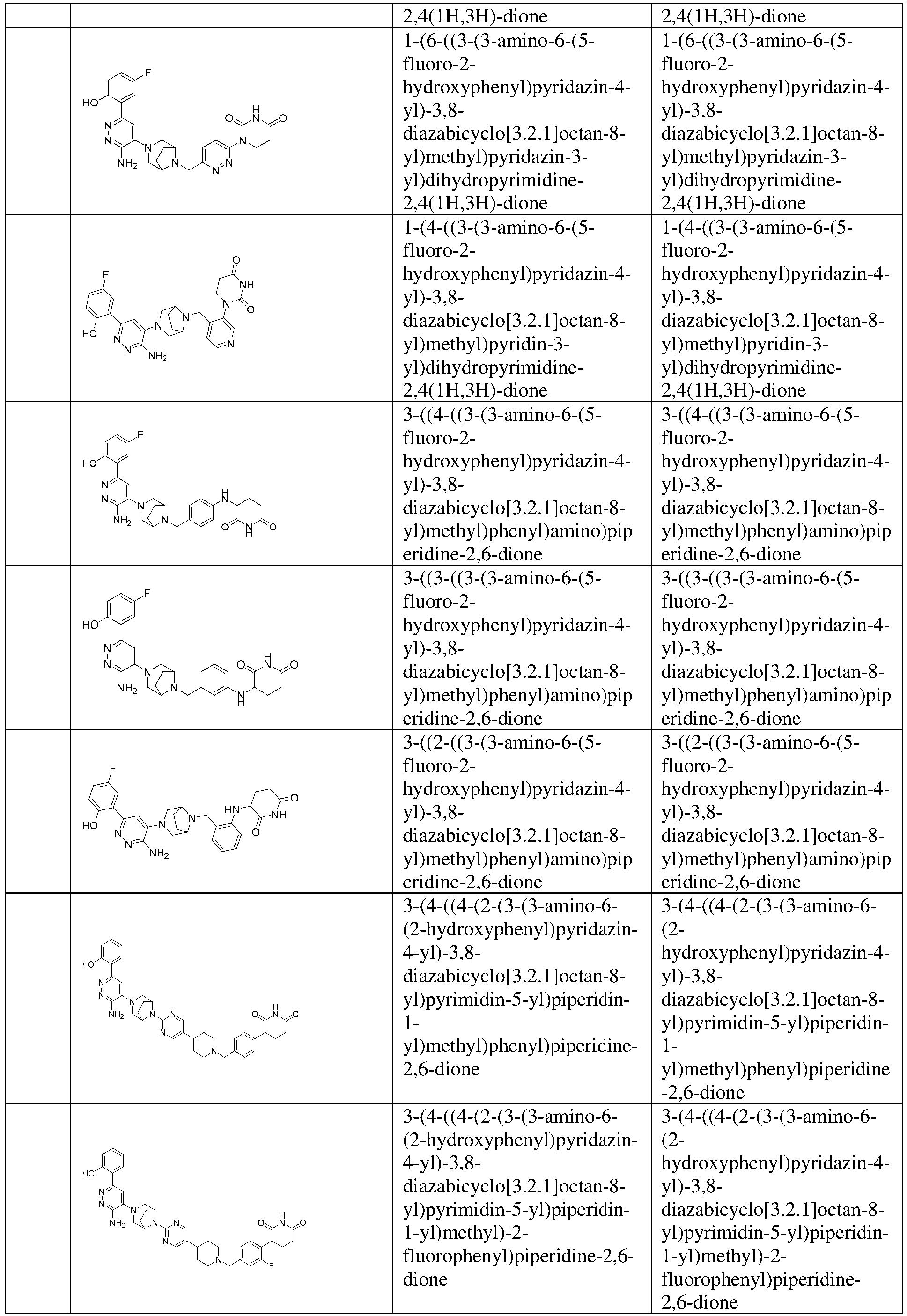

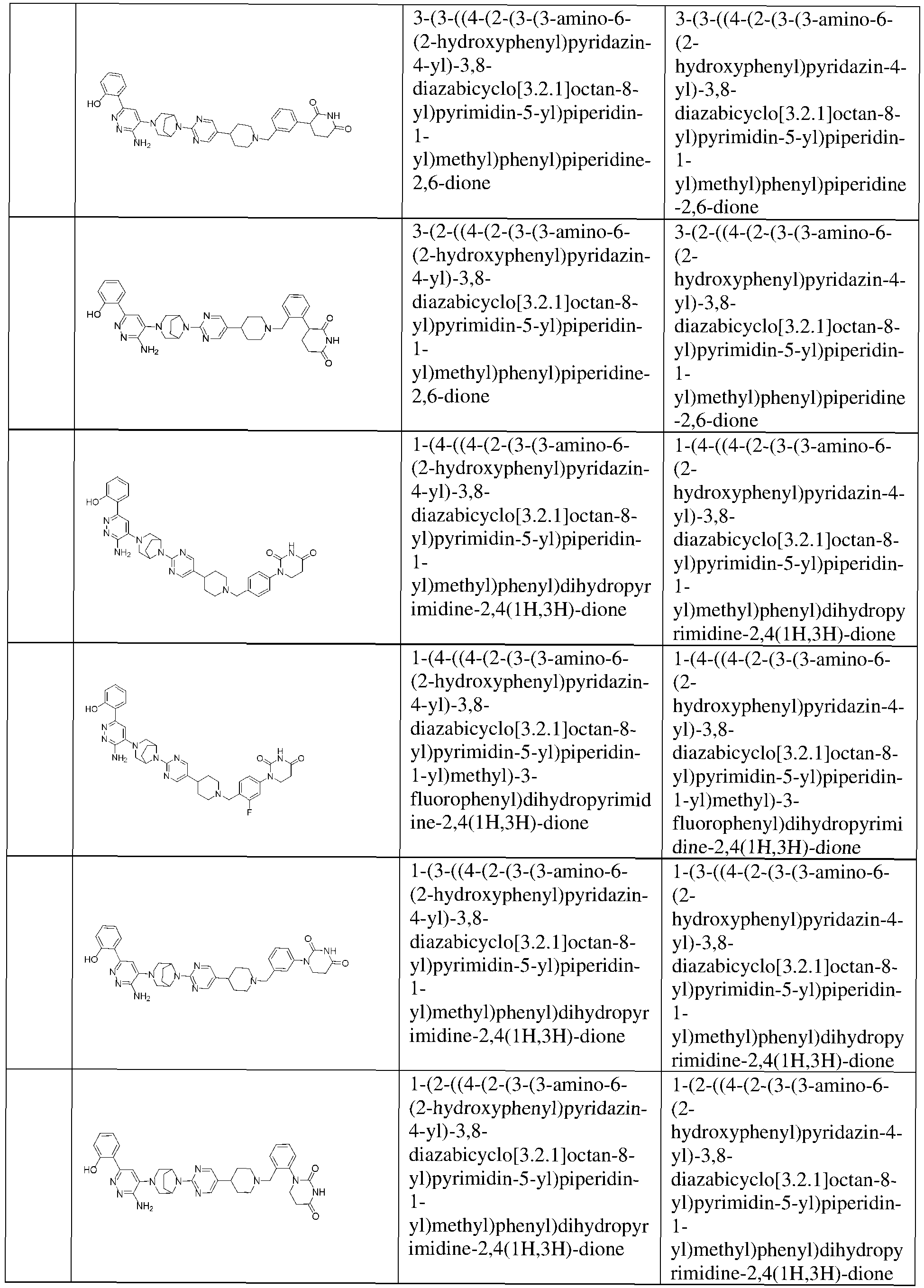

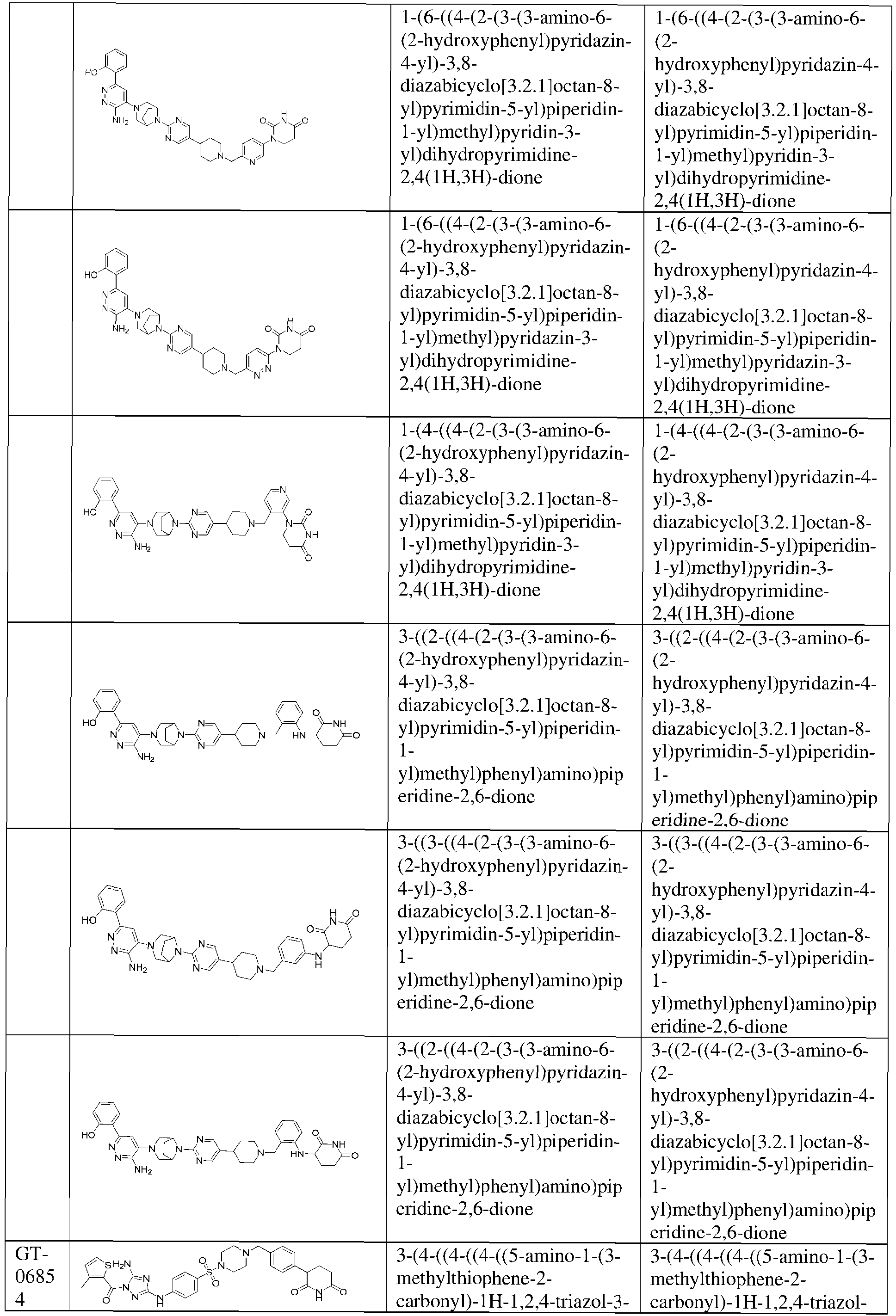

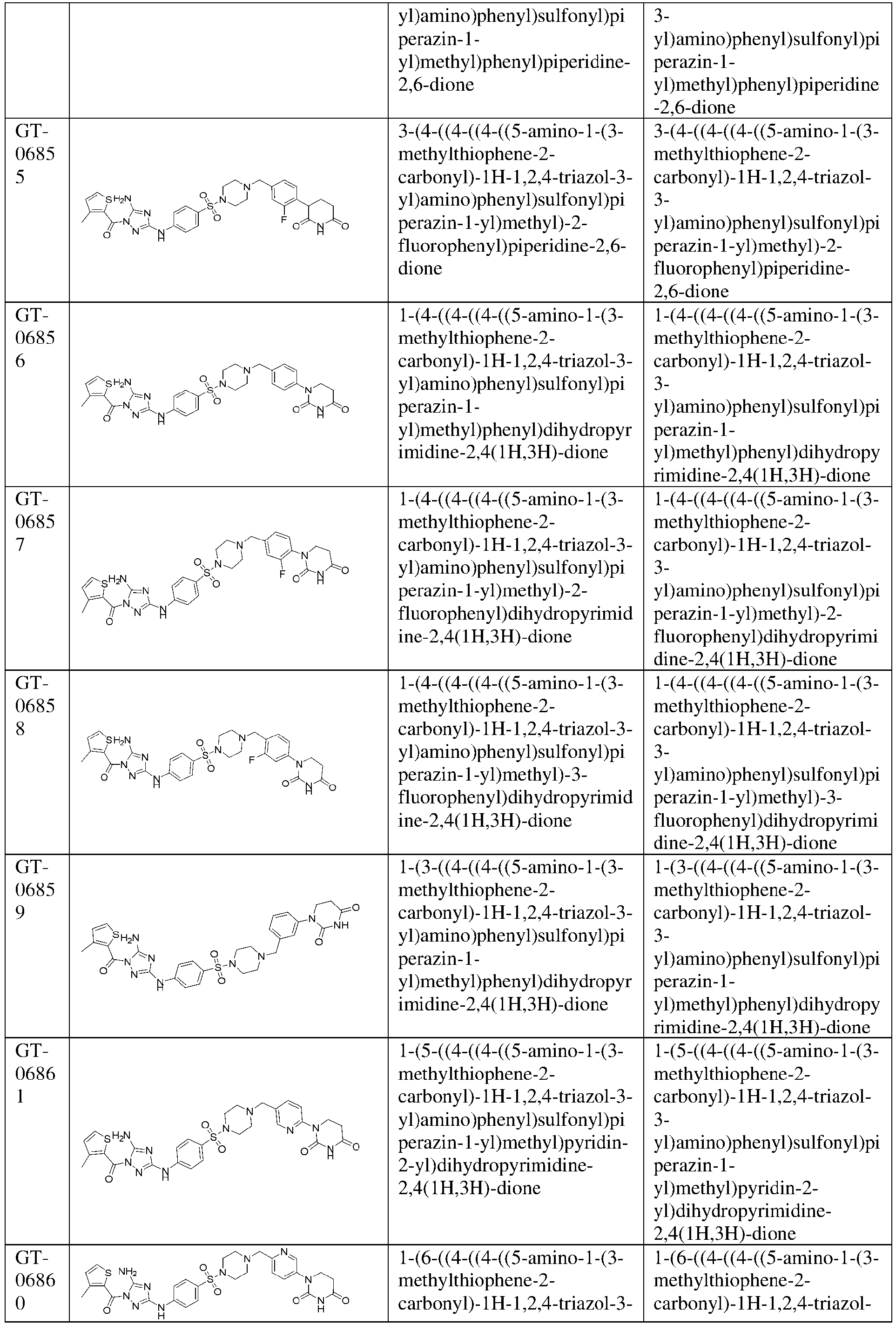

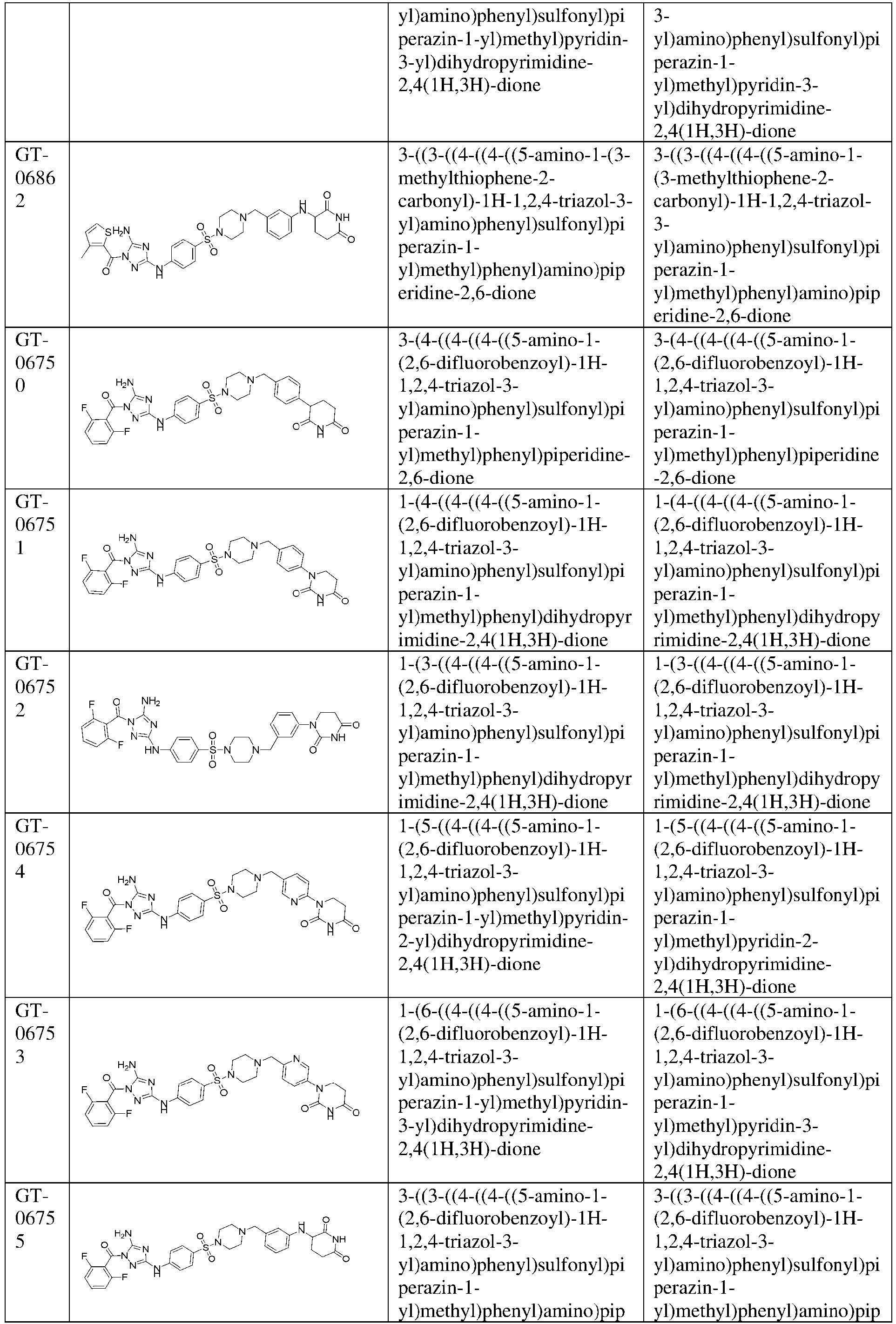

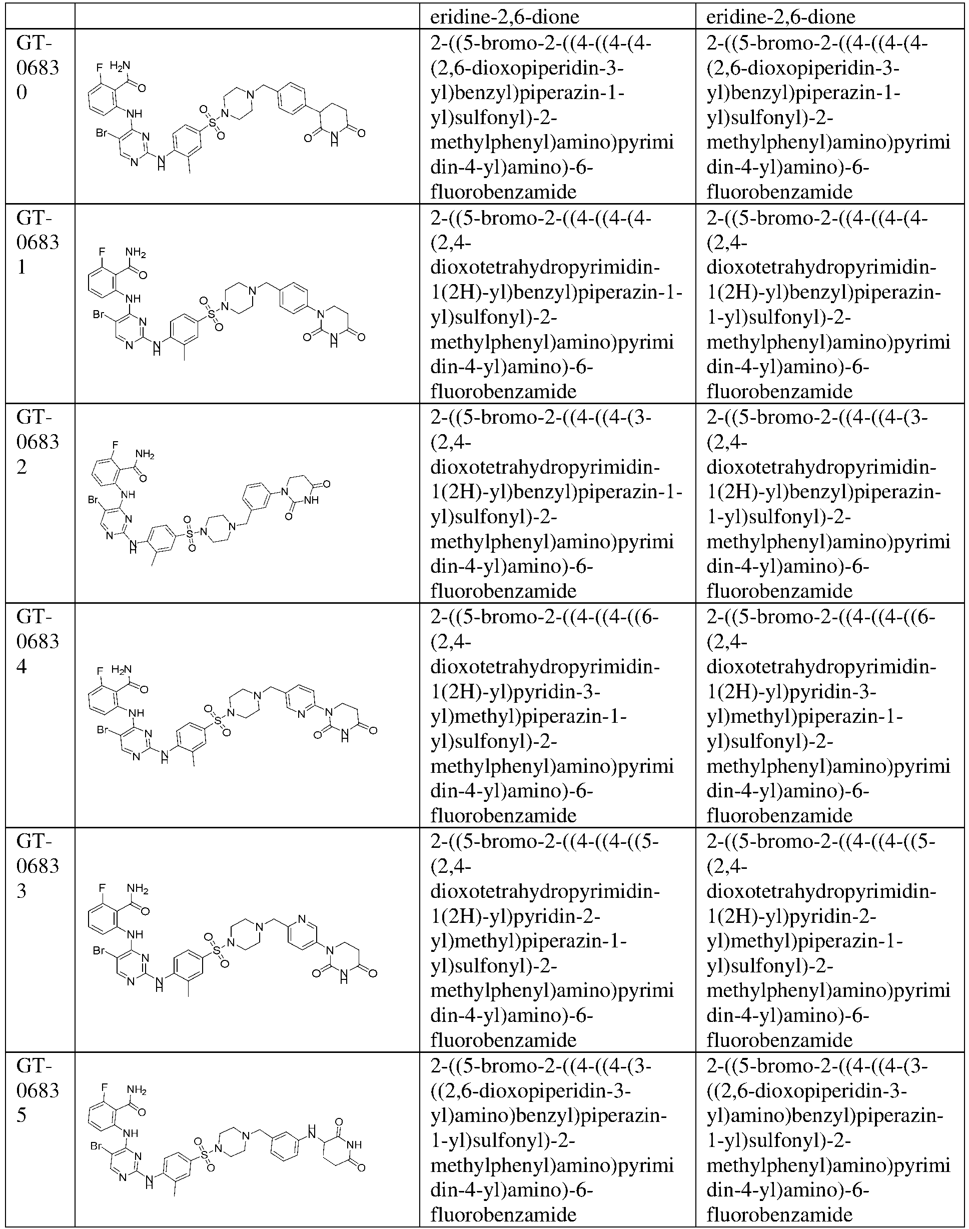

### Compounds of Formula (II)

The present disclosure provides a compound of Formula (II): 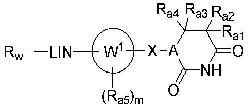 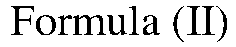 or salts (including pharmaceutically acceptable salts), stereoisomers (including enantiomers), solvates, isotopically enriched analogs, prodrugs, or polymorphs thereof,
wherein A represents N or CH;
Rₐ₁, Rₐ₂, Rₐ₃, and Rₐ₄ are the same or different and independently represent hydrogen, deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or deuterated C₁₋₆ alkoxy;
ring W¹ represents heteroarylene, wherein the ring W¹ is optionally substituted with m Rₐ₅, with each Rₐ₅ being the same or different and independently representing halogen (e.g., fluorine, chlorine, bromine, or iodine), hydroxy, mercapto, nitro, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or deuterated C₁₋₆ alkoxy, C₂₋₆ alkenyl, or C₂₋₆ alkynyl;
m represents an integer of 0, 1, 2, 3 or 4;
X represents a bond or -R_{b1}-N(Rₐ₆)-R_{b2}-, where Rₐ₆ represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), and R_{b1} and R_{b2} each independently represent optionally substituted C₀₋₂ alkylene;
LIN represents optionally substituted methylene, or optionally substituted linear or branched C₂₋₁₅ alkylene, wherein one or more (e.g., 1-20, or 1-15, 1-10, 1-9, 1-8, 1-7, 1-6, 1-5, 1-4, 1-3, or 1-2, or 1) groups selected from Rₐ, R_{b}, and any combination thereof are optionally inserted between any one or more (e.g., 1-15, 1-10, 1-9, 1-8, 1-7, 1-6, 1-5, 1-4, 1-3 or 1-2, or 1) pairs of adjacent carbon atoms in the backbone carbon chain of the linear or branched C₂₋₁₅ alkylene group; wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and in case that two or more groups Rₐ are inserted into the backbone carbon chain of the linear or branched C₂₋₃₀ alkylene group, the two or more groups Rₐ are not directly connected to each other; and wherein each R_{b} is independently selected from the group consisting of optionally substituted cycloalkylene (e.g., optionally substituted C₃₋₂₀ cycloalkylene), optionally substituted arylene (e.g., optionally substituted C₃₋₂₀ arylene), optionally substituted heterocyclylene (e.g., optionally substituted 4- to 20-membered heterocyclylene), optionally substituted heteroarylene (e.g., optionally substituted 5- to 20-membered heteroarylene), alkynylene (e.g., C₂₋₆ alkynylene), alkenylene (e.g., C₂₋₆ alkenylene) and any combination thereof; and
R_{w} represents hydrogen, deuterium, leaving group, halogen, hydroxy, COOH, NH₂, N₃, CHO, methanesulfonyloxy, trifluoromethanesulfonyloxy or p-toluenesulfonyloxy.

In some embodiments, R_{w} of the compound of Formula (II) represents tert-butyldimethylsilyl)oxy (OTBS).

In some embodiments, Rₐ₁, Rₐ₂, Rₐ₃, and Rₐ₄ of the compound of Formula (II) are the same or different and each independently represent hydrogen, deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or deuterated C₁₋₆ alkoxy.

In some embodiments, Rₐ₁, Rₐ₂, Rₐ₃, and Rₐ₄ of the compound of Formula (II) are the same or different and each independently represent hydrogen or deuterium.

In some embodiments, Rₐ₁, Rₐ₂, Rₐ₃, and Rₐ₄ of the compound of Formula (II) represents hydrogen.

In some embodiments, A of the compound of Formula (II) represents N.

In some embodiments, A of the compound of Formula (II) represents CH.

In some embodiments, m of the compound of Formula (II) represents an integer of 0, 1, 2, 3, or 4.

In some embodiments, X of the compound of Formula (II) represents a bond.

In some embodiments, X of the compound of Formula (II) represents -R_{b1}-N(Rₐ₆)-R_{b2}-, wherein Rₐ₆ represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), and R_{b1} and R_{b2} each independently represent optionally substituted C₀₋₂ alkylene. In some embodiments, the substituent of the optionally substituted C₀₋₂ alkylene is optionally selected from the group consisting of deuterium, halogen, hydroxy, mercapto, nitro, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy or any combination thereof.

In some embodiments, X represents -NH-, -N(CH₃)-, -CH₂NH-, -NHCH₂- or -CH₂NHCH₂-.

In some embodiments, R_{w} of the compound of Formula (II) represents hydrogen, deuterium, COOH, NH₂, N₃, CHO, halogen, hydroxy or leaving group (e.g., -N₃, chlorine, bromine, iodine, sulfonate, e.g., methanesulfonyloxy (MsO-), trifluoromethanesulfonyloxy (TfO-) or p-toluenesulfonyloxy (TsO-)).

In some embodiments, ring W¹ of the compound of Formula (II) represents: 5- to 20-membered monocyclic, bicyclic, or polycyclic heteroarylene, and the 5- to 20-membered monocyclic, bicyclic, or polycyclic heteroarylene is optionally substituted with m identical or different Rₐ₅, wherein each Rₐ₅ independently represents halogen, hydroxy, mercapto, nitro, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, C₂₋₆ alkenyl or C₂₋₆ alkynyl; and m represents an integer of 0, 1, 2, 3, or 4.

In some embodiments, ring W¹ of the compound of Formula (II) represents the following groups: furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene. The groups are further optionally substituted with m identical or different Rₐ₅, wherein each Rₐ₅ independently represents halogen, hydroxy, mercapto, nitro, amino, cyano, C₁₋₆ alkyl (e.g., C₁₋₅ alkyl, C₁₋₄ alkyl or C_{I-3} alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, C₂₋₆ alkenyl or C₂₋₆ alkynyl; and m represents an integer of 0, 1, 2, 3, or 4.

In some embodiments, LIN of the compound of Formula (II) represents methylene, which is optionally substituted with 1 to 2 substituents independently selected from the group consisting of deuterium, hydroxy, amino, mercapto, nitro, halogen, cyano, C₁₋₆ alkyl (e.g., C₁₋₅ alkyl, C₁₋₄ alkyl or C_{I-3} alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl or any combination thereof.

In some embodiments, LIN of the compound of Formula (II) represents optionally substituted linear or branched C₂₋₁₅ alkylene, wherein one or more (e.g., 1-20, or 1-15, 1-10, 1-9, 1-8, 1-7, 1-6, 1-5, 1-4, 1-3 or 1-2, or 1) groups selected from Rₐ, R_{b}, and any combination thereof are optionally inserted between any one or more (e.g., 1-15, 1-10, 1-9, 1-8, 1-7, 1-6, 1-5, 1-4, 1-3 or 1-2, or 1) pairs of adjacent carbon atoms in the backbone carbon chain of the linear or branched C₂₋₁₅ alkylene group; wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇) and N(Rₐ₇)C(O)N(Rₐ₇), where each Rₐ₇ independently represents H or C_{I-3} alkyl, and in case that two or more groups Rₐ are inserted into the backbone carbon chain of the linear or branched C₂₋₁₅ alkylene group, the two or more groups Rₐ are not directly connected to each other; and wherein each R_{b} is independently selected from the group consisting of optionally substituted cycloalkylene (e.g., optionally substituted C₃₋₂₀ cycloalkylene), optionally substituted arylene (e.g., optionally substituted C₃₋₂₀ arylene), optionally substituted heterocyclylene (e.g., optionally substituted 4- to 20-membered heterocyclylene), optionally substituted heteroarylene (e.g., optionally substituted 5- to 20-membered heteroarylene), alkynylene (e.g., C₂₋₆ alkynylene), alkenylene (e.g., C₂₋₆ alkenylene) and any combination thereof, wherein the cycloalkylene, arylene, heterocyclylene and heteroarylene are each independently optionally substituted with a substituent selected from the group consisting of deuterium, hydroxy, amino, mercapto, nitro, halogen, cyano, oxo, C₁₋₆ alkyl (e.g., C₁₋₅ alkyl, C₁₋₄ alkyl or C_{I-3} alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl and any combination thereof, and wherein one or more hydrogens of said linear or branched C₂₋₁₅ alkylene are optionally further replaced by a substituent selected from the group consisting of deuterium, hydroxy, amino, mercapto, nitro, halogen, cyano, oxo, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl and any combination thereof.

In some embodiments, LIN of the compound of Formula (II) represents:

-C₁₋₁₅ alkylene-;

-(C(Rₐ₈)(Rₐ₉))ₙ₁-(Rₐ(C(Rₐ₁₀)(Rₐ₁₁))ₙ₂)ₘ₁-;

-(C(Rₐ₈)(Rₐ₉))ₙ₁-(Rₐ(C(Rₐ₁₀₎(Rₐ₁₁))ₙ₂)ₘ₁-(Rₐ(C(Rₐ₁₂)(Rₐ₁₃))ₙ₃)ₘ₂-;

-(C(Rₐ₈)(Rₐ₉))ₙ₁-(Rₐ(C(Rₐ₁₀)(Rₐ₁₁))ₙ₂)ₘ₁-(Rₐ(C(Rₐ₁₂)(Rₐ₁₃))ₙ₃)ₘ₂-(Rₐ(C(Rₐ₁₄)(Rₐ₁₅))ₙ₄)ₘ₃-;

-(C(Rₐ₈)(Rₐ₉))ₙ₁-(R_{b}(C(Rₐ₁₀)(Rₐ₁₁))ₙ₂)ₘ₁-;

-(C(Rₐ₈)(Rₐ₉))ₙ₁-(R_{b}(C(Rₐ₁₀)(Rₐ₁₁))ₙ₂)ₘ₁-(R_{b}(C(Rₐ₁₂)(Rₐ₁₃))ₙ₃)ₘ₂-;

-(C(Rₐ₈)(Rₐ₉))ₙ₁-(R_{b}(C(Rₐ₁₀)(Rₐ₁₁))ₙ₂)ₘ₁-(R_{b}(C(Rₐ₁₂)(Rₐ₁₃))ₙ₃ )ₘ₂-(R_{b}(C(Rₐ₁₄)(Rₐ₁₅))ₙ₄)ₘ₃-;

-(C(Rₐ₈)(Rₐ₉))ₙ₁-(Rₐ- R_{b}-(C(Rₐ₁₀)(Rₐ₁₁))ₙ₂)ₘ₁-;

-(C(Rₐ₈)(Rₐ₉) )ₙ₁-(Rₐ(C(Rₐ₁₀)(Rₐ₁₁))ₙ₂)ₘ₁-(R_{b}(C(Rₐ₁₂)(Rₐ₁₃))ₙ₃)ₘ₂-;

-(C(Rₐ₈)(Rₐ₉))ₙ₁-(R_{b}-Rₐ-(C(Rₐ₁₀)(Rₐ₁₁))ₙ₂)ₘ₁-;

or

-(C(Rₐ₈)(Rₐ₉))ₙ₁-(R₃(C(Rₐ₁₀)(Rₐ₁₁))ₙ₂)ₘ₁-(Rₐ(C(Rₐ₁₂)(Rₐ₁₃))ₙ₃)ₘ₂-;

wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇) and N(Rₐ₇)C(O)N(Rₐ₇), wherein each Rₐ₇ independently represents H or C₁₋₃ alkyl; and each R_{b} is independently selected from the group consisting of optionally substituted cycloalkylene (e.g., optionally substituted C₃₋₂₀ cycloalkylene), optionally substituted arylene (e.g., optionally substituted C₃₋₂₀ arylene), optionally substituted heterocyclylene (e.g., optionally substituted 4- to 20-membered heterocyclylene), optionally substituted heteroarylene (e.g., 5- to 20-membered heteroarylene), alkynylene (e.g., C₂₋₆ alkynylene), alkenylene (e.g., C₂₋₆ alkenylene) and any combination thereof, wherein the cycloalkylene, arylene, heterocyclylene, and heteroarylene are independently optionally substituted with a substituent selected from the group consisting of deuterium, hydroxy, amino, mercapto, nitro, halogen, cyano, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl and any combination thereof;
Rₐ₈, Rₐ₉, Rₐ₁₀, Rₐ₁₁, Rₐ₁₂, Rₐ₁₃, Rₐ₁₄ and Rₐ₁₅ each independently represent H, deuterium, hydroxy, amino, mercapto, nitro, halogen, cyano, oxo, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl and any combination thereof; and
n1, n2, n3, n4, m1, m2, and m3 each independently represent an integer of 1, 2, 3, 4, 5, 6, 7, 8, 9 or 10; and
any one or more hydrogens of said C₁₋₁₅ alkylene are optionally further replaced by a substituent selected from: deuterium, hydroxy, amino, mercapto, nitro, halogen, cyano, oxo, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl and any combination thereof.

In some embodiments, LIN of the compound of Formula (II) represents -C₁₋₁₅ alkylene-, any one or more hydrogens of said C₁₋₁₅ alkylene are optionally further replaced by a substituent selected from: deuterium, hydroxy, amino, mercapto, nitro, halogen, cyano, oxo, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl and any combination thereof.

In some embodiments, LIN of the compound of Formula (II) represents the following groups:

-CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, -(CH₂)₁₃-, -(CH₂)₁₄- or -(CH₂)₁₅-;

wherein any one or more hydrogens of the groups are optionally further replaced by a substituent selected from: deuterium, hydroxy, amino, mercapto, nitro, halogen, cyano, oxo, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl and any combination thereof.

In some embodiments, LIN of the compound of Formula (II) represents the following groups:

-(C(Rₐ₈)(Rₐ₉))ₙ₁-(O(C(Rₐ₁₀)(Rₐ₁₁))ₙ₂)ₘ₁-;

-(C(Rₐ₈)(Rₐ₉))ₙ₁-(O(C(Rₐ₁₀)(Rₐ₁₁))ₙ₂)ₘ₁-(O(C(Rₐ₁₂)(Rₐ₁₃))ₙ₃)ₘ₂-;

-(C(Rₐ₈)(Rₐ₉))ₙ₁-(O(C(Rₐ₁₀)(Rₐ₁₁))ₙ₂)ₘ₁-(O(C(Rₐ₁₂)(Rₐ₁₃))ₙ₃)ₘ₂-(O(C(Rₐ₁₄)(Rₐ₁₅))ₙ₄)ₘ₃-;

-(C(Rₐ₈)(Rₐ₉))ₙ₁-(N(Rₐ₇)(C(Rₐ₁₀)(Rₐ₁₁))ₙ₂)ₘ₁-;

-(C(Rₐ₈)(Rₐ₉))ₙ₁-(N(Rₐ₇)(C(Rₐ₁₀)(Rₐ₁₁))ₙ₂)ₘ₁-(N(Rₐ₇)(C(Rₐ₁₂)(Rₐ₁₃) )ₙ₃)ₘ₂-;

-(C(Rₐ₈)(Rₐ₉))ₙ₁-(N(Rₐ₇)(C(Rₐ₁₀)(Rₐ₁₁))ₙ₂)ₘ₁-(N(Rₐ₇)(C(Rₐ₁₂)(Rₐ₁₃))ₙ₃)ₘ₂-(N(Rₐ₇)(C(Rₐ₁₄)(Rₐ₁₅))ₙ₄)ₘ₃-;

-(C(Rₐ₈)(Rₐ₉))ₙ₁-(C(O)N(Rₐ₇)-(C(Rₐ₁₀)(Rₐ₁₁))ₙ₂)ₘ₁-;

-(C(Rₐ₈)(Rₐ₉))ₙ₁-(C(O)N(Rₐ₇ )-(C(Rₐ₁₀)(Rₐ₁₁))ₙ₂)ₘ₁-(C(O)N(Rₐ₇)-(C(Rₐ₁₂)(Rₐ₁₃))ₙ₃)ₘ₂-;

-(C(Rₐ₈)(Rₐ₉))ₙ₁-(C(O)N(Rₐ₇)-(C(Rₐ₁₀)(Rₐ₁₁))ₙ₂)ₘ₁-(C(O)N(Rₐ₇)-(C(Rₐ₁₂)(Rₐ₁₃))ₙ₃)ₘ₂-(C(O)N(Rₐ₇)-(C(Rₐ₁₄)(Rₐ₁₅))ₙ₄)ₘ₃-;

-(C(Rₐ₈)(Rₐ₉))ₙ₁-(C(O)N(Rₐ₇)-(C(Rₐ₁₀)(Rₐ₁₁))ₙ₂)ₘ₁-(O-(C(Rₐ₁₂)(Rₐ₁₃))ₙ₃)ₘ₂-;

-(C(Rₐ₈)(Rₐ₉))ₙ₁-C(O)N(Rₐ₇)-(C(Rₐ₁₀())(Rₐ₁₁))ₙ₂-(O(C(Rₐ₁₂)(Rₐ₁₃))ₙ₃)ₘ₁-;

-(C(Rₐ₈)(Rₐ₉))ₙ₁-(N(Rₐ₇)C(O)-(C(Rₐ₁₀)(Rₐ₁₁))ₙ₂)ₘ₁-;

-(C(Rₐ₈)(Rₐ₉))ₙ₁-(N(Rₐ₇)C(O)-(C(Rₐ₁₀)(Rₐ₁₁))ₙ₂)ₘ₁-(O-(C(Rₐ₁₂)(Rₐ₁₃)))ₙ₃)ₘ₂-;

-(C(Rₐ₈)(Rₐ₉))ₙ₁-(N(Rₐ₇)C(O)-(C(Rₐ₁₀)(Rₐ₁₁))ₙ₂)ₘ₁-(O-(C(Rₐ₁₂)(Rₐ₁₃))ₙ₃)ₘ₂-(O-(C(Rₐ₁₄)(Rₐ₁₅))ₙ₄)ₘ₃-;

-(C(Rₐ₈)(Rₐ₉))ₙ₁-N(Rₐ₇)C(O)-(C(Rₐ₁₀)(Rₐ₁₁))ₙ₂-(O(C(Rₐ₁₂)(Rₐ₁₃))ₙ₃)ₘ₁-;

-(C(Rₐ₈)(Rₐ₉))ₙ₁-N(Rₐ₇)C(O)N(Rₐ₇)-(C(Rₐ₁₀)(Rₐ₁₁))ₙ₂-;

-(C(Rₐ₈)(Rₐ₉))ₙ₁-C(O)-(C(Rₐ₁₀)(Rₐ₁₁))ₙ₂-;

-(C(Rₐ₈)(Rₐ₉))ₙ₁-(arylene-(C(Rₐ₁₀)(Rₐ₁₁))ₙ₂)ₘ₁-;

-(C(Rₐ₈)(Rₐ₉))ₙ₁-(arylene-(C(Rₐ₁₀)(Rₐ₁₁))ₙ₂)ₘ₁-arylene-(C(Rₐ₁₂)(Rₐ₁₃))ₙ₃-;

-(C(Rₐ₈)(Rₐ₉))ₙ₁-(heterocyclylene-(C(Rₐ₁₀)(Rₐ₁₁))ₙ₂)ₘ₁-;

-(C(Rₐ₈)(Rₐ₉))ₙ₁-(heterocyclylene-(C(Rₐ₁₀)(Rₐ₁₁))ₙ₂)ₘ₁-(heterocyclylene-(C(Rₐ₁₂)(Rₐ₁₃))ₙ₃)ₘ₂-;

-(C(Rₐ₈)(Rₐ₉))ₙ₁-(heteroarylene-(C(Rₐ₁₀)(Rₐ₁₁))ₙ₂)ₘ₁-;

-(C(Rₐ₈)(Rₐ₉))ₙ₁-(heteroarylene-(C(Rₐ₁₀)(Rₐ₁₁))ₙ₂)ₘ₁-(heteroarylene-(C(Rₐ₁₂)(Rₐ₁₃))ₙ₃ )ₘ₂-;

-(C(Rₐ₈)(Rₐ₉))ₙ₁-(cycloalkylene-(C(Rₐ₁₀)(Rₐ₁₁))ₙ₂)ₘ₁-;

or

-(C(Rₐ₈)(Rₐ₉))ₙ₁-(cycloalkylene-(C(Rₐ₁₀)(Rₐ₁₁))ₙ₂)ₘ₁-(cycloalkylene-(C(Rₐ₁₂)(Rₐ₁₃))ₙ₃)ₘ₂-;

wherein each Rₐ₇ independently represents H or C₁₋₃ alkyl;
the cycloalkylene (e.g., C₃₋₂₀ cycloalkylene), arylene (e.g., C₃₋₂₀ arylene), heterocyclylene (e.g., 4- to 20-membered heterocyclylene), and the heteroarylene (e.g., 5- to 20-membered heteroarylene) are each independently optionally substituted with a substituent selected from the group consisting of deuterium, hydroxy, amino, mercapto, nitro, halogen, cyano, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl and any combination thereof;
Rₐ₈, Rₐ₉, Rₐ₁₀, Rₐ₁₁, Rₐ₁₂, Rₐ₁₃, Rₐ₁₄, and Rₐ₁₅ each independently represent H, deuterium, hydroxy, amino, mercapto, nitro, halogen, cyano, oxo, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl and any combination thereof; and
n1, n2, n3, n4, m1, m2, and m3 each independently represent an integer of 1, 2, 3, 4, 5, 6, 7, 8, 9 or 10.

In some embodiments, LIN of the compound of Formula (II) represents the following groups:
-CH₂-O-CH₂-, -CH₂-O-(CH₂)₂-, -(CH₂)₁-O-(CH₂)₃-, -(CH₂)₁-O-(CH₂)₄-, -(CH₂)₁-O-(CH₂)₅-, -(CH₂)₁-O-(CH₂)₆-, -(CH₂)₁-O-(CH₂)₇-, -(CH₂)₁-O-(CH₂)₈-, -(CH₂)₁-O-(CH₂)₉-, -(CH₂)₁-O-(CH₂)₁₀-, -(CH₂)₂-O-(CH₂)₁-, -(CH₂)₂-O-(CH₂)₂-, -(CH₂)₂-O-(CH₂)₃-, -(CH₂)₂-O-(CH₂)₄-, -(CH₂)₂-O-(CH₂)₅-, -(CH₂)₂-O-(CH₂)₆-, -(CH₂)₂-O-(CH₂)₇-, -(CH₂)₂-O-(CH₂)₈-, -(CH₂)₂-O-(CH₂)₉-, -(CH₂)₂-O-(CH₂)₁₀-, -(CH₂)₃-O-(CH₂)₁-, -(CH₂)₃-O-(CH₂)₂-, -(CH₂)₃-O-(CH₂)₃-, -(CH₂)₃-O-(CH₂)₄-, -(CH₂)₃-O-(CH₂)₅-, -(CH₂)₃-O-(CH₂)₆-, -(CH₂)₃-O-(CH₂)₇-, - (CH₂)₄-O-(CH₂)₁-, -(CH₂)₄-O-(CH₂)₂-, -(CH₂)₄-O-(CH₂)₃-, -(CH₂)₄-O-(CH₂)₄-, -(CH₂)₄-O-(CH₂)₅-, -(CH₂)₄-O-(CH₂)₆-, -(CH₂)₅-O-(CH₂)₁-, -(CH₂)₅-O-(CH₂)₂-, -(CH₂)₅-O-(CH₂)₃-, -(CHz)₅-O-(CH₂)₄-, -(CH₂)₅-O-(CH₂)₅-, - (CH₂)₆-o-(CH₂)₁-, -(CH₂)₆-O-(CH₂)₂-, -(CH₂)₆-O-(CH₂)₃-, -(CH₂)₆-O-(CH₂)₄-, -(CH₂)₇-O-(CH₂)₁-, -(CH₂)₇-O-(CH₂)₂-, -(CH₂)₇-O-(CH₂)₃-, -(CH₂)₈-O-(CH₂)₁-, - (CH₂)₈-O-(CH₂)₂-, -CH(CH₃)-O-(CH₂)₁-, -CH(CH₃)-O-(CH₂)₂-, -CH(CH₃)-O-(CH₂)₃-, -CH(CH₃)-O-(CH₂)₄-, -CH(CH₃)-O-(CH₂)₅-, -CH(CH₃)-O-(CH₂)₆-, - CH(CH₃)-O-(CH₂)₇-, -CH(CH₃)-O-(CH₂)₈-, -CH(CH₃)-O-(CH₂)₉-, -CH(CH₃)-O-(CH₂)₁₀-, -CH₂-(O(CH₂)₂)₂-, -CH₂-(O(CH₂)₂)₃-, -CH₂-(O(CH₂)₂)₄-, -CH₂-(O(CH₂)₂)₅-, -CH₂-(O(CH₂)₂)₆-, -CH₂-(O(CH₂)₂)₇-, -CH₂-(O(CH₂)₂)₁-OCH₂-, -CH₂-(O(CH₂)₂)₂-OCH₂-, -CH₂-(O(CH₂)₂)₃-OCH₂-, -CH₂-(O(CH₂)₂)₄-OCH₂-, -CH₂-(O(CH₂)₂)₅-OCH₂-, -CH₂-(O(CH₂)₂)₆-OCH₂-, -CH₂-(O(CH₂)₂)₇-OCH₂-, -(CH₂)₂-(O(CH₂)₂)₂-, -(CH₂)₂-(O(CH₂)₂)₃-, -(CH₂)₂-(O(CH₂)₂)₄-, -(CH₂)₂-(O(CH₂)₂)₅-, -(CH₂)₂-(O(CH₂)₂)₆-, -(CH₂)₂-(O(CH₂)₂)₇-, -(CH₂)₃-(O(CH₂)₂)₂-, -(CH₂)₃-(O(CH₂)₂)₃-, -(CH₂)₃-(O(CH₂)₂)₄-, -(CH₂)₃-(O(CH₂)₂)₅-, -(CH₂)₃-(O(CH₂)₂)₆-, -(CH₂)₃-(O(CH₂)₂)₇-, -(CH₂)₄-(O(CH₂)₂)₂-, -(CH₂)₄-(O(CH₂)₂)₃-, -(CH₂)₄-(O(CH₂)₂)₄-, -(CH₂)₄-(O(CH₂)₂)₅-, -(CH₂)₄-(O(CH₂)₂)₆-, -(CH₂)₄-(O(CH₂)₂)₇-, -CH₂-(O(CH₂)₃)₂-, -CH₂-(O(CH₂)₃)₃-, -CH₂-(O(CH₂)₃)₄-, -(CH₂)₂-(O(CH₂)₃)₂-, -(CH₂)₂-(O(CH₂)₃)₃-, -(CH₂)₂-(O(CH₂)₃)₄-, -(CH₂)₃-(O(CH₂)₃)₂-, -(CH₂)₃-(O(CH₂)₃)₃-, -(CH₂)₃-(O(CH₂)₃)₄-, -CH₂-O-(CH₂)₂-O-(CH₂)₃-, -CH₂-(O(CH₂)₂)₂-(O(CH₂)₃)₂-, -CH₂-(O(CH₂)₂)₃-(O(CH₂)₃)₃-, -CH₂-(O(CH₂)₂)₄-(O(CH₂)₃)₄-, (CH₂)₂-O-(CH₂)₂-O-(CH₂)₃-, -(CH₂)₂-(O(CH₂)₂)₂-(O(CH₂)₃)₂-, -(CH₂)₃-O-(CH₂)₂-O-(CH₂)₃-, -(CH₂)₃-(O(CH₂)₂)₂-(O(CH₂)₃)₂-, -CH₂-O-(CH₂)₃-O-(CH₂)₂-, -CH₂-(O(CH₂)₃)₂-(O(CH₂)₂)₂-, -(CH₂)₂-O-(CH₂)₃-O-(CH₂)₂-, -(CH₂)₂-(O(CH₂)₃)₂-(O(CH₂)₂)₂-, -(CH₂)₃-O-(CH₂)₃-O-(CH₂)₂-, -(CH₂)₃-(O(CH₂)₃)₂-(O(CH₂)₂)₂-, -CH₂-O-(CH₂)₂-O-CH₂-, -(CH₂)₂-O-(CH₂)₂-O-CH₂-, -(CH₂)₂-(O(CH₂)₂)₂-O-(CH₂)₃-, -(CH₂)₂-(O(CH₂)₂)₃-O-(CH₂)₃-, -(CH₂)₂-(O(CH₂)₂)₄-O-(CH₂)₃-, -(CH₂)₅-(O(CH₂)₂)₂-O-(CH₂)₅-, -(CH₂)₅-(O(CH₂)₂)₂-O-(CH₂)₆-, -(CH₂)₁-N(Rₐ₇)-(CH₂)₁-, -(CH₂)]-N(Rₐ₇)-(CH₂)₂-, -(CH₂),-N(Rₐ₇)-(CH₂)₃-, -(CH₂)₁-N(Rₐ₇)-(CH₂)₄-, - (CH₂)₁-N(Rₐ₇)-(CH₂)₅-, -(CH₂)₁-N(Rₐ₇)-(CH₂)₆-, -(CH₂)₁-N(Rₐ₇)-(CH₂)₇-, -(CH₂)₁-N(Rₐ₇)-(CH₂)₈-, -(CH₂)₁-N(Rₐ₇)-(CH₂)₉-, -(CH₂)₁-N(Rₐ₇)-(CH₂)₁₀-, -(CH₂)₂-N(Rₐ₇)-(CH₂)₁-, -(CH₂)₂-N(Rₐ₇)-(CH₂)₂-, -(CH₂)₂-N(Rₐ₇)-(CH₂)₃-, -(CH₂)₂-N(Rₐ₇)-(CH₂)₄-, -(CH₂)₂-N(Rₐ₇)-(CH₂)₅-, -(CH₂)₂-N(Rₐ₇)-(CH₂)₆-, -(CH₂)₂-N(Rₐ₇)-(CH₂)₇-, - (CH₂)₂-N(Rₐ₇)-(CH₂)₈-, -(CH₂)₂-N(Rₐ₇)-(CH₂)₉-, -(CH₂)₂-N(Rₐ₇)-(CH₂)₁₀-, -(CH₂)₃-N(Rₐ₇)-(CH₂)₁-, -(CH₂)₃-N(Rₐ₇)-(CH₂)₂-, -(CH₂)₃-N(Rₐ₇)-(CH₂)₃-, -(CH₂)₄-N(Rₐ₇)-(CH₂)₁-, -(CH₂)₄-N(Rₐ₇)-(CH₂)₂-, -(CH₂)₄-N(Rₐ₇)-(CH₂)₃-, -(CH₂)₄-N(Rₐ₇)-(CH₂)₄-, -(CH₂)s-N(Rₐ₇)-(CH₂)₁-, -(CH₂)₅-N(Rₐ₇)-(CH₂)₂-, -(CH₂)₅-N(Rₐ₇)-(CH₂)₃-, - (CH₂)₅-N(Rₐ₇)-(CH₂)₄-, -(CH₂)₅-N(Rₐ₇)-(CH₂)₅-, -(CH₂)₆-N(Rₐ₇)-(CH₂)₁-, -(CH₂)₆-N(Rₐ₇)-(CH₂)₂-, -(CH₂)₆-N(Rₐ₇)-(CH₂)₃-, -(CH₂)₇-N(Rₐ₇)-(CH₂)₁-, -(CH₂)₇-N(Rₐ₇)-(CH₂)₂-, -(CH₂)₇-N(Rₐ₇)-(CH₂)₃-, -(CH₂)₈-N(Rₐ₇)-(CH₂)₁-, -(CH₂)₈-N(Rₐ₇)-(CH₂)₂-, -(CH₂)₈-N(Rₐ₇)-(CH₂)₃-, -CH(CH₃)-N(Rₐ₇)-(CH₂)₁-, -CH(CH₃)-N(Rₐ₇)-(CH₂)₂-, -CH(CH₃)-N(Rₐ₇)-(CH₂)₃-, -CH(CH₃)-N(Rₐ₇)-(CH₂)₄-, -CH(CH₃)-N(Rₐ₇)-(CH₂)₅-, -CH(CH₃)-N(Rₐ₇)-(CH₂)₆-, -CH(CH₃)-N(Rₐ₇)-(CH₂)₇-, -CH(CH₃)-N(Rₐ₇)-(CH₂)₈-, -CH(CH₃)-N(Rₐ₇)-(CH₂)₉-, -CH(CH₃)-N(Rₐ₇)-(CH₂)₁₀-, -CH₂C(O)NHCH₂-, -(CH₂)₂C(O)NH(CH₂)₂-, -(CH₂)₂C(O)NH(CH₂)₃-, -(CH₂)₂C(O)NH(CH₂)₄-, - (CH₂)₂C(O)NH(CH₂)₅-, -(CH₂)₃C(O)NH(CH₂)₃-, -(CH₂)₃C(O)NH(CH₂)₄-, -(CH₂)₄C(O)NH(CH₂)₄-, - (CH₂)₅C(O)NH(CH₂)₅-, -(CH₂)₆C(O)NH(CH₂)₇-, -(CH₂)₆C(O)NH(CH₂)₆-, -(CH₂)₇C(O)NH(CH₂)₇-, - (CH₂)₂C(O)NH(CH₂)₂-O-(CH₂)₂-, -CH₂NHC(O)CH₂-, -(CH₂)₂NHC(O)(CH₂)₂-, -(CH₂)₂NHC(O)(CH₂)₃-, - (CH₂)₂NHC(O)(CH₂)₄-, -(CH₂)₂NHC(O)(CH₂)₅-, -(CH₂)₃NHC(O)(CH₂)₃-, -(CH₂)₃NHC(O)(CH₂)₄-, - (CH₂)₄NHC(O)(CH₂)₄-, -(CH₂)₅NHC(O)(CH₂)₅-, -(CH₂)₆NHC(O)(CH₂)₇-, -(CH₂)₆NHC(O)(CH₂)₆-, - (CH₂)₇NHC(O)(CH₂)₇-, -(CH₂),NHC(O)(CH₂)₈-, -(CH₂)₂NHC(O)(CH₂)₂-O-(CH₂)₂-, -(CH₂)₄NHC(O)CH₂-, - CH₂-phenylene-CH₂-, -CH₂-phenylene-(CH₂)₂-, -CH₂-phenylene-(CH₂)₃-, -CH₂-phenylene-(CH₂)₄-, -CH₂-phenylene-(CH₂)₅-, -CH₂-phenylene-(CH₂)₆-, -CH₂-phenylene-(CH₂)₇-, -CH₂-phenylene-(CH₂)₈-, -(CH₂)₂-phenylene-(CH₂)₁-, -(CH₂)₂-phenylene-(CH₂)₂-, -(CH₂)₂-phenylene-(CH₂)₃-, -(CH₂)₂-phenylene-(CH₂)₄-, - (CH₂)₂-phenylene-(CH₂)₅-, -(CH₂)₂-phenylene-(CH₂)₆-, -(CH₂)₂-phenylene-(CH₂)₇-, -(CH₂)₂-phenylene-(CH₂)₈-, -(CH₂)₃-phenylene-CH₂-, -(CH₂)₃-phenylene-(CH₂)₂-, -(CH₂)₃-phenylene-(CH₂)₃-, -(CH₂)₃-phenylene-(CH₂)₄-, -(CH₂)₃-phenylene-(CH₂)₅-, -(CH₂)₃-phenylene-(CH₂)₆-, -(CH₂)₃-phenylene-(CH₂)₇-, - (CH₂)₃-phenylene-(CH₂)₈-, -(CH₂)₄-phenylene-CH₂-, -(CH₂)₄-phenylene-(CH₂)₂-, -(CH₂)₄-phenylene-(CH₂)₃-, -(CH₂)₄-phenylene-(CH₂)₄-, -(CH₂)₄-phenylene-(CH₂)₅-, -(CH₂)₄-phenylene-(CH₂)₆-, -(CH₂)₄-phenylene-(CH₂)₇-, -(CH₂)₄-phenylene-(CH₂)₈-, -(CH₂)₅-phenylene-(CH₂)₁-, -(CH₂)₅-phenylene-(CH₂)₂-, -(CH₂)₅-phenylene-(CH₂)₃-, -(CH₂)₅-phenylene-(CH₂)₄-, -(CH₂)₅-phenylene-(CH₂)₅-, -(CH₂)₅-phenylene-(CH₂)₆-, - (CH₂)₅-phenylene-(CH₂)₇-, -(CH₂)₅-phenylene-(CH₂)₈-, -(CH₂)₆-phenylene-(CH₂)₁-, -(CH₂)₆-phenylene-(CH₂)₂-, -(CH₂)₆-phenylene-(CH₂)₃-, -(CH₂)₆-phenylene-(CH₂)₄-, -(CH₂)₆-phenylene-(CH₂)₅-, -(CH₂)₆-phenylene-(CH₂)₆-, -(CH₂)₆-phenylene-(CH₂)₇-, -(CH₂)₆-phenylene-(CH₂)₈-, -(CH₂)₇-phenylene-(CH₂)₁-, - (CH₂)₇-phenylene-(CH₂)₂-, -(CH₂)₇-phenylene-(CH₂)₃-, -(CH₂)₇-phenylene-(CH₂)₄-, -(CH₂)₇-phenylene-(CH₂)₈-, -(CH₂)₈-phenylene-CH₂-, -(CH₂)₈-phenylene-(CH₂)₂-, -(CH₂)₈-phenylene-(CH₂)₃-, -(CH₂)₈-phenylene-(CH₂)₄-, -(CH₂)₈-phenylene-(CH₂)₅-, -(CH₂)₈-phenylene-(CH₂)₆-, -(CH₂)₈-phenylene-(CH₂)₇-, - CH₂-N(Rₐ₇)-CH₂-phenylene-CH₂-, -CH₂-N(Rₐ₇)-CH₂-phenylene-(CH₂)₂-, -CH₂-N(Rₐ₇)-CH₂-phenylene-(CH₂)₃-, -CH₂-N(Rₐ₇)-CH₂-phenylene-(CH₂)₄-, -CH₂-N(Rₐ₇)-CH₂-phenylene-(CH₂)₅-, -CH₂-N(Rₐ₇)-CH₂-phenylene-(CH₂)₆-, -CH₂-N(Rₐ₇)-CH₂-phenylene-(CH₂)₇-, -CH₂-N(Rₐ₇)-CH₂-phenylene-(CH₂)₈-, -CH₂-N(Rₐ₇)-(CH₂)₂-phenylene-CH₂-, -CH₂-N(Rₐ₇)-(CH₂)₂-phenylene-(CH₂)₂-, -CH₂-N(Rₐ₇)-(CH₂)₂-phenylene-(CH₂)₃-, -CH₂-N(Rₐ₇)-(CH₂)₂-phenylene-(CH₂)₄-, -CH₂-N(Rₐ₇)-(CH₂)₂-phenylene-(CH₂)₅-, -CH₂-N(Rₐ₇)-(CH₂)₂-phenylene-(CH₂)₆-, -CH₂-N(Rₐ₇)-(CH₂)₂-phenylene-(CH₂)₇-, -CH₂-N(R.₇)-(CH₂)₂-phenylene-(CH₂)₈-, -(CH₂)₂-N(Rₐ₇)-CH₂-phenylene-CH₂-, -(CH₂)₂-N(Rₐ₇)-CH₂-phenylene-(CH₂)₂-, -(CH₂)₂-N(Rₐ₇)-CH₂-phenylene-(CH₂)₃-, -(CH₂)₂-N(Rₐ₇)-CH₂-phenylene-(CH₂)₄-, -(CH₂)₂-N(Rₐ₇)-CH₂-phenylene-(CH₂)₅-, - (CH₂)₂-N(Rₐ₇)-CH₂-phenylene-(CH₂)₆-, -(CH₂)₂-N(Rₐ₇)-CH₂-phenylene-(CH₂)₇-, -(CH₃)₂-N(Rₐ₇)-CH₂-phenylene-(CH₂)₈-, -(CH₂)₃-N(Rₐ₇)-CH₂-phenylene-CH₂-, -(CH₂)₃-N(Rₐ₇)-CH₂-phenylene-(CH₂)₂-, -(CH₂)₃-N(Rₐ₇)-CH₂-phenylene-(CH₂)₃-, -(CH₂)₃-N(Rₐ₇)-CH₂-phenylene-(CH₂)₈-, -(CH₂)₄-N(Rₐ₇)-CH₂-phenylene-CH₂-, -(CH₂)₄-N(Rₐ₇)-CH₂-phenylene-(CH₂)₂-, -(CH₂)₄-N(Rₐ₇)-CH₂-phenylene-(CH₂)₃-, -(CH₂)₄-N(Rₐ₇)-CH₂-phenylene-(CH₂)₈-, -(CH₂)₅-N(Rₐ₇)-CH₂-phenylene-(CH₂)₃-, -(CH₂)₅-N(Rₐ₇)-CH₂-phenylene-(CH₂)₈-, - (CH₂)₆-N(Rₐ₇)-CH₂-phenylene-(CH₂)₃-, -(CH₂)₆-N(Rₐ₇)-CH₂-phenylene-(CH₂)₈-, -(CH₂)₇-N(Rₐ₇)-CH₂-phenylene-(CH₂)₃-, -(CH₂)₈-N(Rₐ₇)-CH₂-phenylene-CH₂-, -(CH₂)₈-N(Rₐ₇)-CH₂-phenylene-(CH₂)₂-, -(CH₂)₈-N(Rₐ₇)-CH₂-phenylene-(CH₂)₃-, -(CH₂)₈-N(Rₐ₇)-CH₂-phenylene-(CH₂)₄-, -(CH₂)₈-N(Rₐ₇)-CH₂-phenylene-(CH₂)₅-, -(CH₂)₈-N(Rₐ₇)-CH₂-phenylene-(CH₂)₆-, -CH₂-piperidinylene-CH₂-, -CH₂-piperidinylene-(CH₂)₂-, - CH₂-piperidinylene-(CH₂)₃-, -CH₂-piperidinylene-(CH₂)₄-, -CH₂-piperidinylene-(CH₂)₅-, -CH₂-piperidinylene-(CH₂)₆-, -CH₂-piperidinylene-(CH₂)₇-, -CH₂-piperidinylene-(CH₂)₈-, -(CH₂)₂-piperidinylene-(CH₂)₁-, -(CH₂)₂-piperidinylene-(CH₂)₂-, -(CH₂)₂-piperidinylene-(CH₂)₃-, -(CH₂)₂-piperidinylene-(CH₂)₄-, - (CH₂)₂-piperidinylene-(CH₂)₅-, -(CH₂)₂-piperidinylene-(CH₂)₆-, -(CH₂)₂-piperidinylene-(CH₂)₇-, -(CH₂)₂-piperidinylene-(CH₂)₈-, -(CH₂)₃-piperidinylene-CH₂-, -(CH₂)₃-piperidinylene-(CH₂)₂-, -(CH₂)₃-piperidinylene-(CH₂)₃-, -(CH₂)₃-piperidinylene-(CH₂)₄-, -(CH₂)₃-piperidinylene-(CH₂)₅-, -(CH₂)₃-piperidinylene-(CH₂)₆-, -(CH₂)₃-piperidinylene-(CH₂)₇-, -(CH₂)₃-piperidinylene-(CH₂)₈-, -(CH₂)₄-piperidinylene-CH₂-, -(CH₂)₄-piperidinylene-(CH₂)₂-, -(CH₂)₄-piperidinylene-(CH₂)₃-, -(CH₂)₄-piperidinylene-(CH₂)₄-, -(CH₂)₄-piperidinylene-(CH₂)₅-, -(CH₂)₄-piperidinylene-(CH₂)₆-, -(CH₂)₄-piperidinylene-(CH₂)₇-, -(CH₂)₄-piperidinylene-(CH₂)₈-, -(CH₂)₅-piperidinylene-(CH₂)₁-, -(CH₂)₅-piperidinylene-(CH₂)₂-, -(CH₂)₅-piperidinylene-(CH₂)₃-, -(CH₂)₅-piperidinylene-(CH₂)₄-, -(CH₂)₅-piperidinylene-(CH₂)₅-, -(CH₂)₅-piperidinylene-(CH₂)₆-, -(CH₂)₅-piperidinylene-(CH₂)₇-, -(CH₂)₅-piperidinylene-(CH₂)₈-, -(CH₂)₆-piperidinylene-(CH₂)₁-, -(CH₂)₆-piperidinylene-(CH₂)₂-, -(CH₂)₆-piperidinylene-(CH₂)₃-, -(CH₂)₆-piperidinylene-(CH₂)₄-, -(CH₂)₆-piperidinylene-(CH₂)₅-, -(CH₂)₆-piperidinylene-(CH₂)₆-, -(CH₂)₆-piperidinylene-(CH₂)₇-, -(CH₂)₆-piperidinylene-(CH₂)₈-, -(CH₂)₇-piperidinylene-(CH₂)₁-, -(CH₂)₇-piperidinylene-(CH₂)₂-, -(CH₂)₇-piperidinylene-(CH₂)₃-, -(CH₂)₇-piperidinylene-(CH₂)₄-, -(CH₂)₇-piperidinylene-(CH₂)₈-, -(CH₂)₈-piperidinylene-CH₂-, -(CH₂)₈-piperidinylene-(CH₂)₂-, -(CH₂)₈-piperidinylene-(CH₂)₃-, -(CH₂)₈-piperidinylene-(CH₂)₄-, -(CH₂)₈-piperidinylene-(CH₂)₅-, -(CH₂)₈-piperidinylene-(CH₂)₆-, -(CH₂)₈-piperidinylene-(CH₂)₇-, -CH₂-N(Rₐ₇)-CH₂-piperidinylene-CH₂-, -CH₂-N(Rₐ₇)-CH₂-piperidinylene-(CH₂)₂-, -CH₂-N(Rₐ₇)-CH₂-piperidinylene-(CH₂)₃-, - CH₂-N(Rₐ₇)-CH₂-piperidinylene-(CH₂)₄-, -CH₂-N(Rₐ₇)-CH₂-piperidinylene-(CH₂)₅-, -CH₂-N(Rₐ₇ )-CH₂-piperidinylene-(CH₂)₆-, -CH₂-N(Rₐ₇)-CH₂-piperidinylene-(CH₂)₇-, -CH₂-N(Rₐ₇)-CH₂-piperidinylene-(CH₂)₈-, -CH₂-N(Rₐ₇)-(CH₂)₂-piperidinylene-CH₂-, -CH₂-N(Rₐ₇)-(CH₂)₂-piperidinylene-(CH₂)₂-, -CH₂-N(Rₐ₇)-(CH₂)₂-piperidinylene-(CH₂)₃-, -CH₂-N(Rₐ₇ )-(CH₂)₂-piperidinylene-(CH₂)₄-, -CH₂-N(Rₐ₇)-(CH₂)₂-piperidinylene-(CH₂)₅-, -CH₂-N(Rₐ₇)-(CH₂)₂-piperidinylene-(CH₂)₆-, -CH₂-N(Rₐ₇)-(CH₂)₂-piperidinylene-(CH₂)₇-, -CH₂-N(Rₐ₇)-(CH₂-piperidinylene-(CH₂)_{8\}-, -(CH₂)₂-N(Rₐ₇ )-CH₂-piperidinylene-CH₂-, -(CH₂)₂-N(Rₐ₇)-CH₂-piperidinylene-(CH₂)₂-, -(CH₂)₂-N(Rₐ₇)-CH₂-piperidinylene-(CH₂)₃-, -(CH₂)₂-N(Rₐ₇)-CH₂-piperidinylene-(CH₂)₄-, -(CH₂)₂-N(Rₐ₇)-CH₂-piperidinylene-(CH₂)₅-, -(CH₂)₂-N(Rₐ₇ )-CH₂-piperidinylene-(CH₂)₆-, -(CH₂)₂-N(Rₐ₇)-CH₂-piperidinylene-(CH₂)₇-, -(CH₂)₂-N(Rₐ₇)-CH₂-piperidinylene-(CH₂)₈-, -(CH₂)₃-N(Rₐ₇)-CH₂-piperidinylene-CH₂-, -(CH₂)₃-N(Rₐ₇)-CH₂-piperidinylene-(CH₂)₂-, -(CH₂)₃-N(Rₐ₇)-CH₂-piperidinylene-(CH₂)₃-, -(CH₂)₃-N(Rₐ₇)-CH₂-piperidinylene-(CH₂)₈-, -(CH₂)₄-N(Rₐ₇)-CH₂-piperidinylene-CH₂-, -(CH₂)₄-N(Rₐ₇)-CH₂-piperidinylene-(CH₂)₂-, -(CH₂)₄-N(Rₐ₇)-CH₂-piperidinylene-(CH₂)₃-, -(CH₂)₄-N(Rₐ₇)-CH₂-piperidinylene-(CH₂)₈-, -(CH₂)₅-N(Rₐ₇)-CH₂-piperidinylene-(CH₂)₃-, -(CH₂)₅-N(Rₐ₇ )-CH₂-piperidinylene-(CH₂)₈-, -(CH₂)₆-N(Rₐ₇)-CH₂-piperidinylene-(CH₂)₃-, -(CH₂)₆-N(Rₐ₇)-CH₂-piperidinylene-(CH₂)₈-, -(CH₂)₇-N(Rₐ₇)-CH₂-piperidinylene-(CH₂)₃-, -(CH₂)₈-N(Rₐ₇)-CH₂-piperidinylene-CH₂-, -(CH₂)₈-N(Rₐ₇)-CH₂-piperidinylene-(CH₂)₂-, -(CH₂)₈-N(Rₐ₇)-CH₂-piperidinylene-(CH₂)₃-, -(CH₂)₈-N(Rₐ₇)-CH₂-piperidinylene-(CH₂)₄-, -(CH₂)₈-N(Rₐ₇)-CH₂-piperidinylene-(CH₂)₅-, -(CH₂)₈-N(Rₐ₇)-CH₂-piperidinylene-(CH₂)₆-, -CH₂-piperazinylene-CH₂-, -CH₂-piperazinylene-(CH₂)₂-, -CH₂-piperazinylene-(CH₂)₃-, -CH₂-piperazinylene-(CH₂)₄-, -CH₂-piperazinylene-(CH₂)₅-, -CH₂-piperazinylene-(CH₂)₆-, -CH₂-piperazinylene-(CH₂)₇-, -CH₂-piperazinylene-(CH₂)₈-, -(CH₂)₂-piperazinylene-(CH₂)₁-, -(CH₂)₂-piperazinylene-(CH₂)₂-, -(CH₂)₂-piperazinylene-(CH₂)₃-, -(CH₂)₂-piperazinylene-(CH₂)₄-, -(CH₂)₂-piperazinylene-(CH₂)₅-, -(CH₂)₂-piperazinylene-(CH₂)₆-, -(CH₂)₂-piperazinylene-(CH₂)₇-, -(CH₂)₂-piperazinylene-(CH₂)₈-, -(CH₂)₃-piperazinylene-CH₂-, -(CH₂)₃-piperazinylene-(CH₂)₂-, -(CH₂)₃-piperazinylene-(CH₂)₃-, -(CH₂)₃-piperazinylene-(CH₂)₄-, -(CH₂)₃-piperazinylene-(CH₂)₅-, -(CH₂)₃-piperazinylene-(CH₂)₆-, -(CH₂)₃-piperazinylene-(CH₂)₇-, -(CH₂)₃-piperazinylene-(CH₂)₈-, -(CH₂)₄-piperazinylene-CH₂-, -(CH₂)₄-piperazinylene-(CH₂)₂-, -(CH₂)₄-piperazinylene-(CH₂)₃-, -(CH₂)₄-piperazinylene-(CH₂)₄-, -(CH₂)₄-piperazinylene-(CH₂)₅-, -(CH₂)₄-piperazinylene-(CH₂)₆-, -(CH₂)₄-piperazinylene-(CH₂)₇-, -(CH₂)₄-piperazinylene-(CH₂)₈-, -(CH₂)₅-piperazinylene-(CH₂)₁-, -(CH₂)₅-piperazinylene-(CH₂)₂-, -(CH₂)₅-piperazinylene-(CH₂)₃-, -(CH₂)₅-piperazinylene-(CH₂)₄-, -(CH₂)₅-piperazinylene-(CH₂)₅-, -(CH₂)₅-piperazinylene-(CH₂)₆-, -(CH₂)₅-piperazinylene-(CH₂)₇-, -(CH₂)₅-piperazinylene-(CH₂)₈-, -(CH₂)₆-piperazinylene-(CH₂)₁-, -(CH₂)₆-piperazinylene-(CH₂)₂-, -(CH₂)₆-piperazinylene-(CH₂)₃-, -(CH₂)₆-piperazinylene-(CH₂)₄-, -(CH₂)₆-piperazinylene-(CH₂)₅-, -(CH₂)₆-piperazinylene-(CH₂)₆-, -(CH₂)₆-piperazinylene-(CH₂)₇-, -(CH₂)₆-piperazinylene-(CH₂)₈-, -(CH₂)₇-piperazinylene-(CH₂)₁-, -(CH₂)₇-piperazinylene-(CH₂)₂-, -(CH₂)₇-piperazinylene-(CH₂)₃-, -(CH₂)₇-piperazinylene-(CH₂)₄-, -(CH₂)₈-piperazinylene-CH₂-, -(CH₂)₈-piperazinylene-(CH₂)₂-, -(CH₂)₈-piperazinylene-(CH₂)₃-, -(CH₂)₈-piperazinylene-(CH₂)₄-, -(CH₂)₈-piperazinylene-(CH₂)₅-, or -(CH₂)₈-piperazinylene-(CH₂)₆-;
wherein each Rₐ₇ independently represents H or C₁₋₃ alkyl;
the phenylene, piperazinylene and piperidinylene are each independently optionally substituted with a substituent selected from the group consisting of deuterium, hydroxy, amino, mercapto, nitro, halogen, cyano, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl and any combination thereof.

In some embodiments, the compound of Formula (II) is also represented by Formula (II-1) or Formula (II-2): 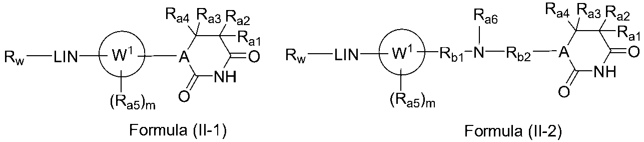 wherein A, Rₐ₁, Rₐ₂, Rₐ₃, Rₐ₄, (Rₐ₅)ₘ, Rₐ₆, R_{b1}, R_{b2}, and ring W¹ are as defined in the compound of Formula (II) and its various embodiments above.

In some embodiments, in the compound of Formula (II-1) or Formula (II-2),
A represents N or CH;
Rₐ₁, Rₐ₂, Rₐ₃, and Rₐ₄ are the same or different and independently represent hydrogen, deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or deuterated C₁₋₆ alkoxy;
ring W¹ represents heteroarylene, and the ring W¹ is optionally substituted with m Rₐ₅, with each Rₐ₅ being the same or different and independently representing halogen (e.g., fluorine, chlorine, bromine, or iodine), hydroxy, mercapto, nitro, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, C₂₋₆ alkenyl or C₂₋₆ alkynyl;
m represents an integer of 0, 1, 2, 3, or 4;
Rₐ₆ represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), and R_{b1} and R_{b2} each independently represent optionally substituted C₀₋₂ alkylene;
LIN represents optionally substituted methylene, or optionally substituted linear or branched C₂₋₁₅ alkylene, wherein one or more (e.g., 1-20, or 1-15, 1-10, 1-9, 1-8, 1-7, 1-6, 1-5, 1-4, 1-3 or 1-2, or 1) groups selected from Rₐ, R_{b}, and any combination thereof are optionally inserted between any one or more (e.g., 1-15, 1-10, 1-9, 1-8, 1-7, 1-6, 1-5, 1-4, 1-3 or 1-2, or 1) pairs of adjacent carbon atoms in the backbone carbon chain of the linear or branched C₂₋₁₅ alkylene group; wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and in case that two or more groups Rₐ are inserted into the backbone carbon chain of the linear or branched C₂₋₃₀ alkylene group, the two or more groups Rₐ are not directly connected to each other; and wherein each R_{b} is independently selected from the group consisting of optionally substituted cycloalkylene (e.g., optionally substituted C₃₋₂₀ cycloalkylene), optionally substituted arylene (e.g., optionally substituted C₃₋₂₀ arylene), optionally substituted heterocyclylene (e.g., optionally substituted 4- to 20-membered heterocyclylene), optionally substituted heteroarylene (e.g., optionally substituted 5- to 20-membered heteroarylene), alkynylene (e.g., C₂₋₆ alkynylene), alkenylene (e.g., C₂₋₆ alkenylene) and any combination thereof; and
R_{w} represents hydrogen, deuterium, leaving group, halogen, hydroxy, COOH, NH₂, N₃, CHO, methanesulfonyloxy, trifluoromethanesulfonyloxy or p-toluenesulfonyloxy. In some sub-embodiments, preferably, the methylene group represented by LIN is optionally substituted with 1 to 2 substituents independently selected from the group consisting of deuterium, hydroxy, amino, mercapto, nitro, halogen, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl or any combination thereof; and/or, the cycloalkylene, arylene, heterocyclylene, and heteroarylene represented by R_{b} are independently optionally substituted with a substituent selected from the group consisting of deuterium, hydroxy, amino, mercapto, nitro, halogen, cyano, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl and any combination thereof; and/or, one or more hydrogens of said linear or branched C₂₋₁₅ alkylene are optionally further replaced by a substituent selected from deuterium, hydroxy, amino, mercapto, nitro, halogen, cyano, oxo, deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, isobutoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl and any combination thereof.

In some embodiments, R_{w} of the compound of Formula (II-1) or Formula (II-2) represents tert-butyldimethylsilyloxy (OTBS).

In some embodiments, the compounds of Formula (II) and their salts (including pharmaceutically acceptable salts, such as hydrochloride, etc.), prodrugs, solvates, isotopically enriched analogs, polymorphs, stereoisomers (including enantiomers and diastereomers), or mixture of stereoisomers thereof in Table 2 below are provided:

**Table 2. The compounds of the present disclosure**

| Compound No. | Structure of compound | Compound's name | Chinese translation of Compound's name |
|---|---|---|---|
| | 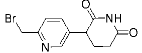 | 3-(6-(bromomethyl)pyridin-3-yl)piperidine-2,6-dione | 3-(6-(bromomethyl)pyridin-3-yl)piperidine-2,6-dione |
| | 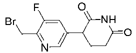 | 3-(6-(bromomethyl)-5-fluoropyridin-3-yl)piperidine-2,6-dione | 3-(6-(bromomethyl)-5-fluoropyridin-3-yl)piperidine-2,6-dione |
| | 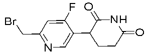 | 3-(6-(bromomethyl)-4-fluoropyridin-3-yl)piperidine-2,6-dione | 3-(6-(bromomethyl)-4-fluoropyridin-3-yl)piperidine-2,6-dione |
| | 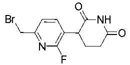 | 3-(6-(bromomethyl)-2-fluoropyridin-3-yl)piperidine-2,6-dione | 3-(6-(bromomethyl)-2-fluoropyridin-3-yl)piperidine-2,6-dione |
| | 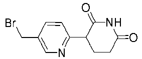 | 3-(5-(bromomethyl)pyridin-2-yl)piperidine-2,6-dione | 3-(5-(bromomethyl)pyridin-2-yl)piperidine-2,6-dione |
| | 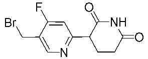 | 3-(5-(bromomethyl)-4-fluoropyridin-2-yl)piperidine-2,6-dione | 3-(5-(bromomethyl)-4-fluoropyridin-2-yl)piperidine-2,6-dione |
| | 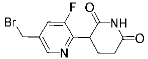 | 3-(5-(bromomethyl)-3-fluoropyridin-2-yl)piperidine-2,6-dione | 3-(5-(bromomethyl)-3-fluoropyridin-2-yl)piperidine-2,6-dione |
| | 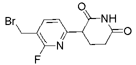 | 3-(5-(bromomethyl)-6-fluoropyridin-2-yl)piperidine-2,6-dione | 3-(5-(bromomethyl)-6-fluoropyridin-2-yl)piperidine-2,6-dione |
| | 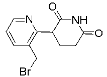 | 3-(3-(bromomethyl)pyridin-2-yl)piperidine-2,6-dione | 3-(3-(bromomethyl)pyridin-2-yl)piperidine-2,6-dione |
| | 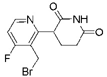 | 3-(3-(bromomethyl)-4-fluoropyridin-2-yl)piperidine-2,6-dione | 3-(3-(bromomethyl)-4-fluoropyridin-2-yl)piperidine-2,6-dione |
| | 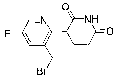 | 3-(3-(bromomethyl)-5-fluoropyridin-2-yl)piperidine-2,6-dione | 3-(3-(bromomethyl)-5-fluoropyridin-2-yl)piperidine-2,6-dione |
| | 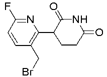 | 3-(3-(bromomethyl)-6-fluoropyridin-2-yl)piperidine-2,6-dione | 3-(3-(bromomethyl)-6-fluoropyridin-2-yl)piperidine-2,6-dione |
| | 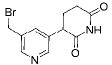 | 3-(5-(bromomethyl)pyridin-3-yl)piperidine-2,6-dione | 3-(5-(bromomethyl)pyridin-3-yl)piperidine-2,6-dione |
| | 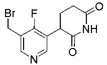 | 3-(5-(bromomethyl)-4-fluoropyridin-3-yl)piperidine-2,6-dione | 3-(5-(bromomethyl)-4-fluoropyridin-3-yl)piperidine-2,6-dione |
| | 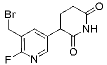 | 3-(5-(bromomethyl)-6-fluoropyridin-3-yl)piperidine-2,6-dione | 3-(5-(bromomethyl)-6-fluoropyridin-3-yl)piperidine-2,6-dione |
| | 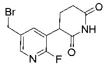 | 3-(5-(bromomethyl)-2-fluoropyridin-3-yl)piperidine-2,6-dione | 3-(5-(bromomethyl)-2-fluoropyridin-3-yl)piperidine-2,6-dione |
| | 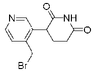 | 3-(4-(bromomethyl)pyridin-3-yl)piperidine-2,6-dione | 3-(4-(bromomethyl)pyridin-3-yl)piperidine-2,6-dione |
| | 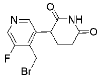 | 3-(4-(bromomethyl)-5-fluoropyridin-3-yl)piperidine-2,6-dione | 3-(4-(bromomethyl)-5-fluoropyridin-3-yl)piperidine-2,6-dione |
| | 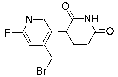 | 3-(4-(bromomethyl)-6-fluoropyridin-3-yl)piperidine-2,6-dione | 3-(4-(bromomethyl)-6-fluoropyridin-3-yl)piperidine-2,6-dione |
| | 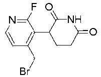 | 3-(4-(bromomethyl)-2-fluoropyridin-3-yl)piperidine-2,6-dione | 3-(4-(bromomethyl)-2-fluoropyridin-3-yl)piperidine-2,6-dione |
| | 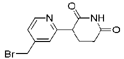 | 3-(4-(bromomethyl)pyridin-2-yl)piperidine-2,6-dione | 3-(4-(bromomethyl)pyridin-2-yl)piperidine-2,6-dione |
| | 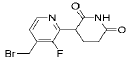 | 3-(4-(bromomethyl)-3-fluoropyridin-2-yl)piperidine-2,6-dione | 3-(4-(bromomethyl)-3-fluoropyridin-2-yl)piperidine-2,6-dione |
| | 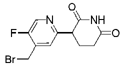 | 3-(4-(bromomethyl)-5-fluoropyridin-2-yl)piperidine-2,6-dione | 3-(4-(bromomethyl)-5-fluoropyridin-2-yl)piperidine-2,6-dione |
| | 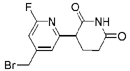 | 3-(4-(bromomethyl)-6-fluoropyridin-2-yl)piperidine-2,6-dione | 3-(4-(bromomethyl)-6-fluoropyridin-2-yl)piperidine-2,6-dione |
| | 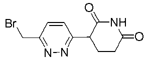 | 3-(6-(bromomethyl)pyridazin-3-yl)piperidine-2,6-dione | 3-(6-(bromomethyl)pyridazin-3-yl)piperidine-2,6-dione |
| | 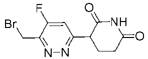 | 3-(6-(bromomethyl)-5-fluoropyridazin-3-yl)piperidine-2,6-dione | 3-(6-(bromomethyl)-5-fluoropyridazin-3-yl)piperidine-2,6-dione |
| | 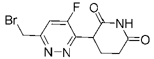 | 3-(6-(bromomethyl)-4-fluoropyridazin-3-yl)piperidine-2,6-dione | 3-(6-(bromomethyl)-4-fluoropyridazin-3-yl)piperidine-2,6-dione |
| | 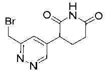 | 3-(6-(bromomethyl)pyridazin-4-yl)piperidine-2,6-dione | 3-(6-(bromomethyl)pyridazin-4-yl)piperidine-2,6-dione |
| | 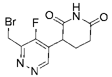 | 3-(6-(bromomethyl)-5-fluoropyridazin-4-yl)piperidine-2,6-dione | 3-(6-(bromomethyl)-5-fluoropyridazin-4-yl)piperidine-2,6-dione |
| | 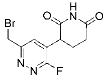 | 3-(6-(bromomethyl)-3-fluoropyridazin-4-yl)piperidine-2,6-dione | 3-(6-(bromomethyl)-3-fluoropyridazin-4-yl)piperidine-2,6-dione |
| | 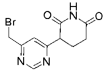 | 3-(6-(bromomethyl)pyrimidin-4-yl)piperidine-2,6-dione | 3-(6-(bromomethyl)pyrimidin-4-yl)piperidine-2,6-dione |
| | 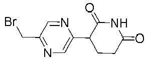 | 3-(5-(bromomethyl)pyrazin-2-yl)piperidine-2,6-dione | 3-(5-(bromomethyl)pyrazin-2-yl)piperidine-2,6-dione |
| | 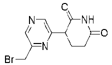 | 3-(6-(bromomethyl)pyrazin-2-yl)piperidine-2,6-dione | 3-(6-(bromomethyl)pyrazin-2-yl)piperidine-2,6-dione |
| | 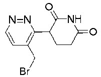 | 3-(4-(bromomethyl)pyridazin-3-yl)piperidine-2,6-dione | 3-(4-(bromomethyl)pyridazin-3-yl)piperidine-2,6-dione |
| | 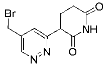 | 3-(5-(bromomethyl)pyridazin-3-yl)piperidine-2,6-dione | 3-(5-(bromomethyl)pyridazin-3-yl)piperidine-2,6-dione |
| | 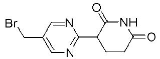 | 3-(5-(bromomethyl)pyrimidin-2-yl)piperidine-2,6-dione | 3-(5-(bromomethyl)pyrimidin-2-yl)piperidine-2,6-dione |
| | 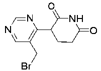 | 3-(5-(bromomethyl)pyrimidin-4-yl)piperidine-2,6-dione | 3-(5-(bromomethyl)pyrimidin-4-yl)piperidine-2,6-dione |
| GT-04517 | 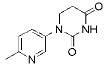 | 1-(6-methylpyridin-3-yl)dihydropyrimidine-2,4(1H,3H)-dione | 1-(6-methylpyridin-3-yl)dihydropyrimidine-2,4(1H,3H)-dione |
| GT-08123 | 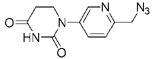 | 1-(6-(azidomethyl)pyridin-3-yl)dihydropyrimidine-2,4(1H,3H)-dione | 1-(6-(azidomethyl)pyridin-3-yl)dihydropyrimidine-2,4(1H,3H)-dione |
| | 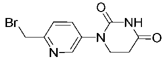 | 1-(6-(bromomethyl)pyridin-3-yl)dihydropyrimidine-2,4(1H,3H)-dione | 1-(6-(bromomethyl)pyridin-3-yl)dihydropyrimidine-2,4(1H,3H)-dione |
| | 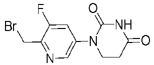 | 1-(6-(bromomethyl)-5-fluoropyridin-3-yl)dihydropyrimidine-2,4(1H,3H)-dione | 1-(6-(bromomethyl)-5-fluoropyridin-3-yl)dihydropyrimidine-2,4(1H,3H)-dione |
| | 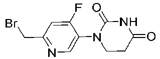 | 1-(6-(bromomethyl)-4-fluoropyridin-3-yl)dihydropyrimidine-2,4(1H,3H)-dione | 1-(6-(bromomethyl)-4-fluoropyridin-3-yl)dihydropyrimidine-2,4(1H,3H)-dione |
| | 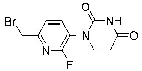 | 1-(6-(bromomethyl)-2-fluoropyridin-3-yl)dihydropyrimidine-2,4(1H,3H)-dione | 1-(6-(bromomethyl)-2-fluoropyridin-3-yl)dihydropyrimidine-2,4(1H,3H)-dione |
| GT-08122 | 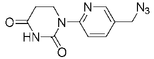 | 1-(5-(azidomethyl)pyridin-2-yl)dihydropyrimidine-2,4(1H,3H)-dione | 1-(5-(azidomethyl)pyridin-2-yl)dihydropyrimidine-2,4(1H,3H)-dione |
| | 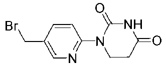 | 1-(5-(bromomethyl)pyridin-2-yl)dihydropyrimidine-2,4(1H,3H)-dione | 1-(5-(bromomethyl)pyridin-2-yl)dihydropyrimidine-2,4(1H,3H)-dione |
| | 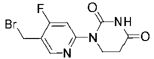 | 1-(5-(bromomethyl)-4-fluoropyridin-2-yl)dihydropyrimidine-2,4(1H,3H)-dione | 1-(5-(bromomethyl)-4-fluoropyridin-2-yl)dihydropyrimidine-2,4(1H,3H)-dione |
| | 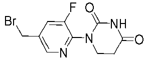 | 1-(5-(bromomethyl)-3-fluoropyridin-2-yl)dihydropyrimidine-2,4(1H,3H)-dione | 1-(5-(bromomethyl)-3-fluoropyridin-2-yl)dihydropyrimidine-2,4(1H,3H)-dione |
| | 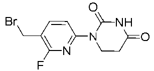 | 1-(5-(bromomethyl)-6-fluoropyridin-2-yl)dihydropyrimidine-2,4(1H,3H)-dione | 1-(5-(bromomethyl)-6-fluoropyridin-2-yl)dihydropyrimidine-2,4(1H,3H)-dione |
| | 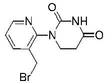 | 1-(3-(bromomethyl)pyridin-2-yl)dihydropyrimidine-2,4(1H,3H)-dione | 1-(3-(bromomethyl)pyridin-2-yl)dihydropyrimidine-2,4(1H,3H)-dione |
| | 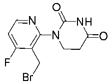 | 1-(3-(bromomethyl)-4-fluoropyridin-2-yl)dihydropyrimidine-2,4(1H,3H)-dione | 1-(3-(bromomethyl)-4-fluoropyridin-2-yl)dihydropyrimidine-2,4(1H,3H)-dione |
| | 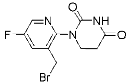 | 1-(3-(bromomethyl)-5-fluoropyridin-2-yl)dihydropyrimidine-2,4(1H,3H)-dione | 1-(3-(bromomethyl)-5-fluoropyridin-2-yl)dihydropyrimidine-2,4(1H,3H)-dione |
| | 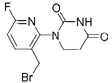 | 1-(3-(bromomethyl)-6-fluoropyridin-2-yl)dihydropyrimidine-2,4(1H,3H)-dione | 1-(3-(bromomethyl)-6-fluoropyridin-2-yl)dihydropyrimidine-2,4(1H,3H)-dione |
| GT-08125 | 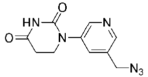 | 1-(5-(azidomethyl)pyridin-3-yl)dihydropyrimidine-2,4(1H,3H)-dione | 1-(5-(azidomethyl)pyridin-3-yl)dihydropyrimidine-2,4(1H,3H)-dione |
| | 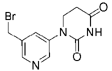 | 1-(5-(bromomethyl)pyridin-3-yl)dihydropyrimidine-2,4(1H,3H)-dione | 1-(5-(bromomethyl)pyridin-3-yl)dihydropyrimidine-2,4(1H,3H)-dione |
| | 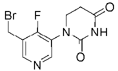 | 1-(5-(bromomethyl)-4-fluoropyridin-3-yl)dihydropyrimidine-2,4(1H,3H)-dione | 1-(5-(bromomethyl)-4-fluoropyridin-3-yl)dihydropyrimidine-2,4(1H,3H)-dione |
| GT-07556 | 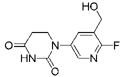 | 1-(6-fluoro-5-(hydroxymethyl)pyridin-3-yl)dihydropyrimidine-2,4(1H,3H)-dione | 1-(6-fluoro-5-(hydroxymethyl)pyridin-3-yl)dihydropyrimidine-2,4(1H,3H)-dione |
| | 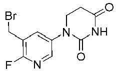 | 1-(5-(bromomethyl)-6-fluoropyridin-3-yl)dihydropyrimidine-2,4(1H,3H)-dione | 1-(5-(bromomethyl)-6-fluoropyridin-3-yl)dihydropyrimidine-2,4(1H,3H)-dione |
| | 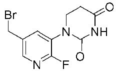 | 1-(5-(bromomethyl)-2-fluoropyridin-3-yl)dihydropyrimidine-2,4(1H,3H)-dione | 1-(5-(bromomethyl)-2-fluoropyridin-3-yl)dihydropyrimidine-2,4(1H,3H)-dione |
| | 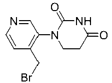 | 1-(4-(bromomethyl)pyridin-3-yl)dihydropyrimidine-2,4(1H,3H)-dione | 1-(4-(bromomethyl)pyridin-3-yl)dihydropyrimidine-2,4(1H,3H)-dione |
| | 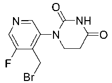 | 1-(4-(bromomethyl)-5-fluoropyridin-3-yl)dihydropyrimidine-2,4(1H,3H)-dione | 1-(4-(bromomethyl)-5-fluoropyridin-3-yl)dihydropyrimidine-2,4(1H,3H)-dione |
| | 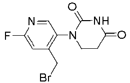 | 1-(4-(bromomethyl)-6-fluoropyridin-3-yl)dihydropyrimidine-2,4(1H,3H)-dione | 1-(4-(bromomethyl)-6-fluoropyridin-3-yl)dihydropyrimidine-2,4(1H,3H)-dione |
| | 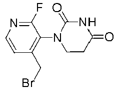 | 1-(4-(bromomethyl)-2-fluoropyridin-3-yl)dihydropyrimidine-2,4(1H,3H)-dione | 1-(4-(bromomethyl)-2-fluoropyridin-3-yl)dihydropyrimidine-2,4(1H,3H)-dione |
| | 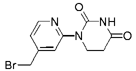 | 1-(4-(bromomethyl)pyridin-2-yl)dihydropyrimidine-2,4(1H,3H)-dione | 1-(4-(bromomethyl)pyridin-2-yl)dihydropyrimidine-2,4(1H,3H)-dione |
| | 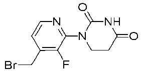 | 1-(4-(bromomethyl)-3-fluoropyridin-2-yl)dihydropyrimidine-2,4(1H,3H)-dione | 1-(4-(bromomethyl)-3-fluoropyridin-2-yl)dihydropyrimidine-2,4(1H,3H)-dione |
| | 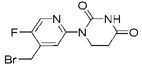 | 1-(4-(bromomethyl)-5-fluoropyridin-2-yl)dihydropyrimidine-2,4(1H,3H)-dione | 1-(4-(bromomethyl)-5-fluoropyridin-2-yl)dihydropyrimidine-2,4(1H,3H)-dione |
| | 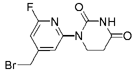 | 1-(4-(bromomethyl)-6-fluoropyridin-2-yl)dihydropyrimidine-2,4(1H,3H)-dione | 1-(4-(bromomethyl)-6-fluoropyridin-2-yl)dihydropyrimidine-2,4(1H,3H)-dione |
| | 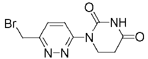 | 1-(6-(bromomethyl)pyridazin-3-yl)dihydropyrimidine-2,4(1H,3H)-dione | 1-(6-(bromomethyl)pyridazin-3-yl)dihydropyrimidine-2,4(1H,3H)-dione |
| | 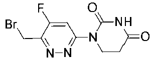 | 1-(6-(bromomethyl)-5-fluoropyridazin-3-yl)dihydropyrimidine-2,4(1H,3H)-dione | 1-(6-(bromomethyl)-5-fluoropyridazin-3-yl)dihydropyrimidine-2,4(1H,3H)-dione |
| | 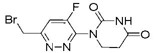 | 1-(6-(bromomethyl)-4-fluoropyridazin-3-yl)dihydropyrimidine-2,4(1H,3H)-dione | 1-(6-(bromomethyl)-4-fluoropyridazin-3-yl)dihydropyrimidine-2,4(1H,3H)-dione |
| | 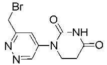 | 1-(6-(bromomethyl)pyridazin-4-yl)dihydropyrimidine-2,4(1H,3H)-dione | 1-(6-(bromomethyl)pyridazin-4-yl)dihydropyrimidine-2,4(1H,3H)-dione |
| | 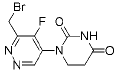 | 1-(6-(bromomethyl)-5-fluoropyridazin-4-yl)dihydropyrimidine-2,4(1H,3H)-dione | 1-(6-(bromomethyl)-5-fluoropyridazin-4-yl)dihydropyrimidine-2,4(1H,3H)-dione |
| | 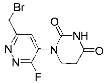 | 1-(6-(bromomethyl)-3-fluoropyridazin-4-yl)dihydropyrimidine-2,4(1H,3H)-dione | 1-(6-(bromomethyl)-3-fluoropyridazin-4-yl)dihydropyrimidine-2,4(1H,3H)-dione |
| | 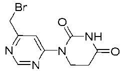 | 1-(6-(bromomethyl)pyrimidin-4-yl)dihydropyrimidine-2,4(1H,3H)-dione | 1-(6-(bromomethyl)pyrimidin-4-yl)dihydropyrimidine-2,4(1H,3H)-dione |
| GT-07634 | 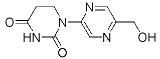 | 1-(5-(hydroxymethyl)pyrazin-2-yl)dihydropyrimidine-2,4(1H,3H)-dione | 1-(5-(hydroxymethyl)pyrazin-2-yl)dihydropyrimidine-2,4(1H,3H)-dione |
| | 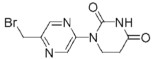 | 1-(5-(bromomethyl)pyrazin-2-yl)dihydropyrimidine-2,4(1H,3H)-dione | 1-(5-(bromomethyl)pyrazin-2-yl)dihydropyrimidine-2,4(1H,3H)-dione |
| GT-07632 | 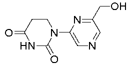 | 1-(6-(hydroxymethyl)pyrazin-2-yl)dihydropyrimidine-2,4(1H,3H)-dione | 1-(6-(hydroxymethyl)pyrazin-2-yl)dihydropyrimidine-2,4(1H,3H)-dione |
| GT-08129 | 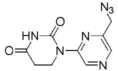 | 1-(6-(azidomethyl)pyrazin-2-yl)dihydropyrimidine-2,4(1H,3H)-dione | 1-(6-(azidomethyl)pyrazin-2-yl)dihydropyrimidine-2,4(1H,3H)-dione |
| | 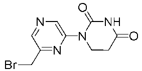 | 1-(6-(bromomethyl)pyrazin-2-yl)dihydropyrimidine-2,4(1H,3H)-dione | 1-(6-(bromomethyl)pyrazin-2-yl)dihydropyrimidine-2,4(1H,3H)-dione |
| | 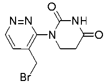 | 1-(4-(bromomethyl)pyridazin-3-yl)dihydropyrimidine-2,4(1H,3H)-dione | 1-(4-(bromomethyl)pyridazin-3-yl)dihydropyrimidine-2,4(1H,3H)-dione |
| | 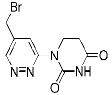 | 1-(5-(bromomethyl)pyridazin-3-yl)dihydropyrimidine-2,4(1H,3H)-dione | 1-(5-(bromomethyl)pyridazin-3-yl)dihydropyrimidine-2,4(1H,3H)-dione |
| | 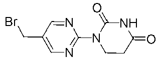 | 1-(5-(bromomethyl)pyrimidin-2-yl)dihydropyrimidine-2,4(1H,3H)-dione | 1-(5-(bromomethyl)pyrimidin-2-yl)dihydropyrimidine-2,4(1H,3H)-dione |
| | 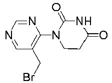 | 1-(5-(bromomethyl)pyrimidin-4-yl)dihydropyrimidine-2,4(1H,3H)-dione | 1-(5-(bromomethyl)pyrimidin-4-yl)dihydropyrimidine-2,4(1H,3H)-dione |
| GT-07446 | 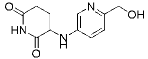 | 3-((6-(hydroxymethyl)pyridin-3-yl)amino)piperidine-2,6-dione | 3-((6-(hydroxymethyl)pyridin-3-yl)amino)piperidine-2,6-dione |
| GT-07640 | 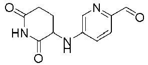 | 5-((2,6-dioxopiperidin-3-yl)amino)picolinaldehyde | 5-((2,6-dioxopiperidin-3-yl)amino)picolinaldehyde |
| GT-07445 | 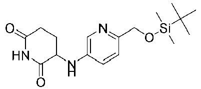 | 3-((6-(((tertbutyldimethylsilyl)oxy)meth yl)pyridin-3-yl)amino)piperidine-2,6-dione | 3-((6-(((tertbutyldimethylsilyl)oxy)methy l)pyridin-3-yl)amino)piperidine-2,6-dione |
| | 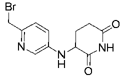 | 3-((6-(bromomethyl)pyridin-3-yl)amino)piperidine-2,6-dione | 3-((6-(bromomethyl)pyridin-3-yl)amino)piperidine-2,6-dione |
| | 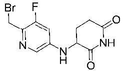 | 3-((6-(bromomethyl)-5-fluoropyridin-3-yl)amino)piperidine-2,6-dione | 3-((6-(bromomethyl)-5-fluoropyridin-3-yl)amino)piperidine-2,6-dione |
| | 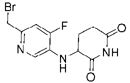 | 3-((6-(bromomethyl)-4-fluoropyridin-3-yl)amino)piperidine-2,6-dione | 3-((6-(bromomethyl)-4-fluoropyridin-3-yl)amino)piperidine-2,6-dione |
| | 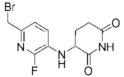 | 3-((6-(bromomethyl)-2-fluoropyridin-3-yl)amino)piperidine-2,6-dione | 3-((6-(bromomethyl)-2-fluoropyridin-3-yl)amino)piperidine-2,6-dione |
| GT-07551 | 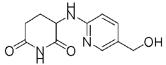 | 3-((5-(hydroxymethyl)pyridin-2-yl)amino)piperidine-2,6-dione | 3-((5-(hydroxymethyl)pyridin-2-yl)amino)piperidine-2,6-dione |
| GT-07552 | 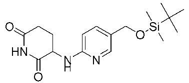 | 3-((5-(((tertbutyldimethylsilyl)oxy)meth yl)pyridin-2-yl)amino)piperidine-2,6-dione | 3-((5-(((tertbutyldimethylsilyl)oxy)methy l)pyridin-2-yl)amino)piperidine-2,6-dione |
| | 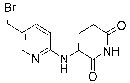 | 3-((5-(bromomethyl)pyridin-2-yl)amino)piperidine-2,6-dione | 3-((5-(bromomethyl)pyridin-2-yl)amino)piperidine-2,6-dione |
| | 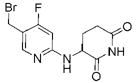 | 3-((5-(bromomethyl)-4-fluoropyridin-2-yl)amino)piperidine-2,6-dione | 3-((5-(bromomethyl)-4-fluoropyridin-2-yl)amino)piperidine-2,6-dione |
| | 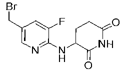 | 3-((5-(bromomethyl)-3-fluoropyridin-2-yl)amino)piperidine-2,6-dione | 3-((5-(bromomethyl)-3-fluoropyridin-2-yl)amino)piperidine-2,6-dione |
| | 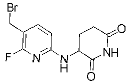 | 3-((5-(bromomethyl)-6-fluoropyridin-2-yl)amino)piperidine-2,6-dione | 3-((5-(bromomethyl)-6-fluoropyridin-2-yl)amino)piperidine-2,6-dione |
| GT-07197 | 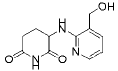 | 3-((3-(hydroxymethyl)pyridin-2-yl)amino)piperidine-2,6-dione | 3-((3-(hydroxymethyl)pyridin-2-yl)amino)piperidine-2,6-dione |
| GT-07447 | 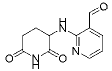 | 2-((2,6-dioxopiperidin-3-yl)amino)nicotinaldehyde | 2-((2,6-dioxopiperidin-3-yl)amino)nicotinaldehyde |
| GT-08131 | 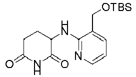 | 3-((3-(((tertbutyldimethylsilyl)oxy)meth yl)pyridin-2-yl)amino)piperidine-2,6-dione | 3-((3-(((tertbutyldimethylsilyl)oxy)methy l)pyridin-2-yl)amino)piperidine-2,6-dione |
| | 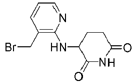 | 3-((3-(bromomethyl)pyridin-2-yl)amino)piperidine-2,6-dione | 3-((3-(bromomethyl)pyridin-2-yl)amino)piperidine-2,6-dione |
| | 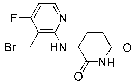 | 3-((3-(bromomethyl)-4-fluoropyridin-2-yl)amino)piperidine-2,6-dione | 3-((3-(bromomethyl)-4-fluoropyridin-2-yl)amino)piperidine-2,6-dione |
| | 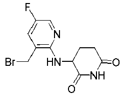 | 3-((3-(bromomethyl)-5-fluoropyridin-2-yl)amino)piperidine-2,6-dione | 3-((3-(bromomethyl)-5-fluoropyridin-2-yl)amino)piperidine-2,6-dione |
| | 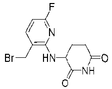 | 3-((3-(bromomethyl)-6-fluoropyridin-2-yl)amino)piperidine-2,6-dione | 3-((3-(bromomethyl)-6-fluoropyridin-2-yl)amino)piperidine-2,6-dione |
| GT-07554 | 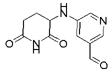 | 5-((2,6-dioxopiperidin-3-yl)amino)nicotinaldehyde | 5-((2,6-dioxopiperidin-3-yl)amino)nicotinaldehyde |
| GT-07448 | 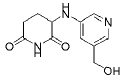 | 3-((5-(hydroxymethyl)pyridin-3-yl)amino)piperidine-2,6-dione | 3-((5-(hydroxymethyl)pyridin-3-yl)amino)piperidine-2,6-dione |
| GT-07449 | 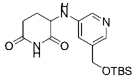 | 3-((5-(((tertbutyldimethylsilyl)oxy)meth yl)pyridin-3-yl)amino)piperidine-2,6-dione | 3-((5-(((tertbutyldimethylsilyl)oxy)methy 1)pyridin-3-yl)amino)piperidine-2,6-dione |
| | 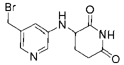 | 3-((5-(bromomethyl)pyridin-3-yl)amino)piperidine-2,6-dione | 3-((5-(bromomethyl)pyridin-3-yl)amino)piperidine-2,6-dione |
| | 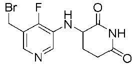 | 3-((5-(bromomethyl)-4-fluoropyridin-3-yl)amino)piperidine-2,6-dione | 3-((5-(bromomethyl)-4-fluoropyridin-3-yl)amino)piperidine-2,6-dione |
| | 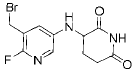 | 3-((5-(bromomethyl)-6-fluoropyridin-3-yl)amino)piperidine-2,6-dione | 3-((5-(bromomethyl)-6-fluoropyridin-3-yl)amino)piperidine-2,6-dione |
| | 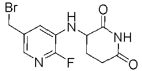 | 3-((5-(bromomethyl)-2-fluoropyridin-3-yl)amino)piperidine-2,6-dione | 3-((5-(bromomethyl)-2-fluoropyridin-3-yl)amino)piperidine-2,6-dione |
| GT-07559 | 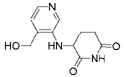 | 3-((4-(hydroxymethyl)pyridin-3-yl)amino)piperidine-2,6-dione | 3-((4-(hydroxymethyl)pyridin-3-yl)amino)piperidine-2,6-dione |
| GT-07804 | 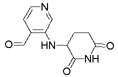 | 3-((2,6-dioxopiperidin-3-yl)amino)isonicotinaldehyde | 3-((2,6-dioxopiperidin-3-yl)amino)isonicotinaldehyde |
| GT-07638 | 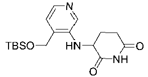 | 3-((4-(((tertbutyldimethylsilyl)oxy)meth yl)pyridin-3-yl)amino)piperidine-2,6-dione | 3-((4-(((tertbutyldimethylsilyl)oxy)methy l)pyridin-3-yl)amino)piperidine-2,6-dione |
| | 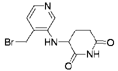 | 3-((4-(bromomethyl)pyridin-3-yl)amino)piperidine-2,6-dione | 3-((4-(bromomethyl)pyridin-3-yl)amino)piperidine-2,6-dione |
| | 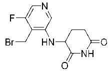 | 3-((4-(bromomethyl)-5-fluoropyridin-3-yl)amino)piperidine-2,6-dione | 3-((4-(bromomethyl)-5-fluoropyridin-3-yl)amino)piperidine-2,6-dione |
| | 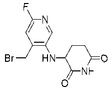 | 3-((4-(bromomethyl)-6-fluoropyridin-3-yl)amino)piperidine-2,6-dione | 3-((4-(bromomethyl)-6-fluoropyridin-3-yl)amino)piperidine-2,6-dione |
| | 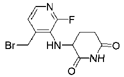 | 3-((4-(bromomethyl)-2-fluoropyridin-3-yl)amino)piperidine-2,6-dione | 3-((4-(bromomethyl)-2-fluoropyridin-3-yl)amino)piperidine-2,6-dione |
| GT-07366 | 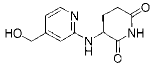 | 3-((4-(hydroxymethyl)pyridin-2-yl)amino)piperidine-2,6-dione | 3-((4-(hydroxymethyl)pyridin-2-yl)amino)piperidine-2,6-dione |
| GT-07553 | 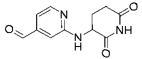 | 2-((2,6-dioxopiperidin-3-yl)amino)isonicotinaldehyde | 2-((2,6-dioxopiperidin-3-yl)amino)isonicotinaldehyde |
| GT-08132 | 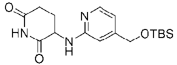 | 3-((4-(((tertbutyldimethylsilyl)oxy)meth yl)pyridin-2-yl)amino)piperidine-2,6-dione | 3-((4-(((tertbutyldimethylsilyl)oxy)methy l)pyridin-2-yl)amino)piperidine-2,6-dione |
| | 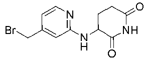 | 3-((4-(bromomethyl)pyridin-2-yl)amino)piperidine-2,6-dione | 3-((4-(bromomethyl)pyridin-2-yl)amino)piperidine-2,6-dione |
| | 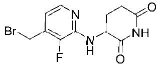 | 3-((4-(bromomethyl)-3-fluoropyridin-2-yl)amino)piperidine-2,6-dione | 3-((4-(bromomethyl)-3-fluoropyridin-2-yl)amino)piperidine-2,6-dione |
| GT-07725 | 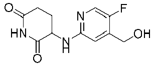 | 3-((5-fluoro-4-(hydroxymethyl)pyridin-2-yl)amino)piperidine-2,6-dione | 3-((5-fluoro-4-(hydroxymethyl)pyridin-2-yl)amino)piperidine-2,6-dione |
| GT-07724 | 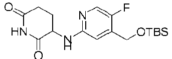 | 3-((4-(((tertbutyldimethylsilyl)oxy)methyl)-5-fluoropyridin-2-yl)amino)piperidine-2,6-dione | 3-((4-(((tertbutyldimethylsilyl)oxy)methyl)-5-fluoropyridin-2-yl)amino)piperidine-2,6-dione |
| | 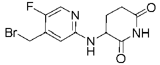 | 3-((4-(bromomethyl)-5-fluoropyridin-2-yl)amino)piperidine-2,6-dione | 3-((4-(bromomethyl)-5-fluoropyridin-2-yl)amino)piperidine-2,6-dione |
| | 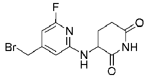 | 3-((4-(bromomethyl)-6-fluoropyridin-2-yl)amino)piperidine-2,6-dione | 3-((4-(bromomethyl)-6-fluoropyridin-2-yl)amino)piperidine-2,6-dione |
| GT-07803 | 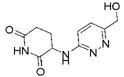 | 3-((6-(hydroxymethyl)pyridazin-3-yl)amino)piperidine-2,6-dione | 3-((6-(hydroxymethyl)pyridazin-3-yl)amino)piperidine-2,6-dione |
| GT-07726 | 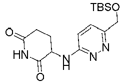 | 3-((6-(((tertbutyldimethylsilyl)oxy)meth yl)pyridazin-3-yl)amino)piperidine-2,6-dione | 3-((6-(((tertbutyldimethylsilyl)oxy)methy l)pyridazin-3-yl)amino)piperidine-2,6-dione |
| | 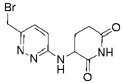 | 3-((6-(bromomethyl)pyridazin-3-yl)amino)piperidine-2,6-dione | 3-((6-(bromomethyl)pyridazin-3-yl)amino)piperidine-2,6-dione |
| | 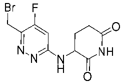 | 3-((6-(bromomethyl)-5-fluoropyridazin-3-yl)amino)piperidine-2,6-dione | 3-((6-(bromomethyl)-5-fluoropyridazin-3-yl)amino)piperidine-2,6-dione |
| | 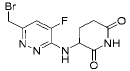 | 3-((6-(bromomethyl)-4-fluoropyridazin-3-yl)amino)piperidine-2,6-dione | 3-((6-(bromomethyl)-4-fluoropyridazin-3-yl)amino)piperidine-2,6-dione |
| | 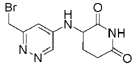 | 3-((6-(bromomethyl)pyridazin-4-yl)amino)piperidine-2,6-dione | 3-((6-(bromomethyl)pyridazin-4-yl)amino)piperidine-2,6-dione |
| | 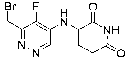 | 3-((6-(bromomethyl)-5-fluoropyridazin-4-yl)amino)piperidine-2,6-dione | 3-((6-(bromomethyl)-5-fluoropyridazin-4-yl)amino)piperidine-2,6-dione |
| | 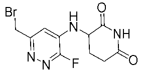 | 3-((6-(bromomethyl)-3-fluoropyridazin-4-yl)amino)piperidine-2,6-dione | 3-((6-(bromomethyl)-3-fluoropyridazin-4-yl)amino)piperidine-2,6-dione |
| GT-07805 | 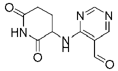 | 4-((2,6-dioxopiperidin-3-yl)amino)pyrimidine-5-carbaldehyde | 4-((2,6-dioxopiperidin-3-yl)amino)pyrimidine-5-carbaldehyde |
| GT-07727 | 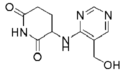 | 3-((5-(hydroxymethyl)pyrimidin-4-yl)amino)piperidine-2,6-dione | 3-((5-(hydroxymethyl)pyrimidin-4-yl)amino)piperidine-2,6-dione |
| GT-07639 | 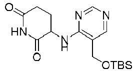 | 3-((5-(((tertbutyldimethylsilyl)oxy)meth yl)pyrimidin-4-yl)amino)piperidine-2,6-dione | 3-((5-(((tertbutyldimethylsilyl)oxy)methy l)pyrimidin-4-yl)amino)piperidine-2,6-dione |
| | 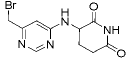 | 3-((6-(bromomethyl)pyrimidin-4-yl)amino)piperidine-2,6-dione | 3-((6-(bromomethyl)pyrimidin-4-yl)amino)piperidine-2,6-dione |
| | 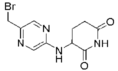 | 3-((5-(bromomethyl)pyrazin-2-yl)amino)piperidine-2,6-dione | 3-((5-(bromomethyl)pyrazin-2-yl)amino)piperidine-2,6-dione |
| | 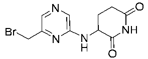 | 3-((6-(bromomethyl)pyrazin-2-yl)amino)piperidine-2,6-dione | 3-((6-(bromomethyl)pyrazin-2-yl)amino)piperidine-2,6-dione |
| | 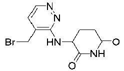 | 3-((4-(bromomethyl)pyridazin-3-yl)amino)piperidine-2,6-dione | 3-((4-(bromomethyl)pyridazin-3-yl)amino)piperidine-2,6-dione |
| | 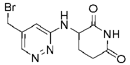 | 3-((5-(bromomethyl)pyridazin-3-yl)amino)piperidine-2,6-dione | 3-((5-(bromomethyl)pyridazin-3-yl)amino)piperidine-2,6-dione |
| GT-07367 | 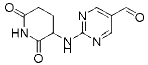 | 2-((2,6-dioxopiperidin-3-yl)amino)pyrimidine-5-carbaldehyde | 2-((2,6-dioxopiperidin-3-yl)amino)pyrimidine-5-carbaldehyde |
| GT-07809 | 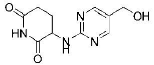 | 3-((5-(hydroxymethyl)pyrimidin-2-yl)amino)piperidine-2,6-dione | 3-((5-(hydroxymethyl)pyrimidin-2-yl)amino)piperidine-2,6-dione |
| GT-07808 | 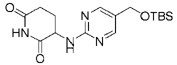 | 3-((5-(((tertbutyldimethylsilyl)oxy)meth yl)pyrimidin-2-yl)amino)piperidine-2,6-dione | 3-((5-(((tertbutyldimethylsilyl)oxy)methy l)pyrimidin-2-yl)amino)piperidine-2,6-dione |
| | 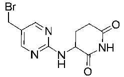 | 3-((5-(bromomethyl)pyrimidin-2-yl)amino)piperidine-2,6-dione | 3-((5-(bromomethyl)pyrimidin-2-yl)amino)piperidine-2,6-dione |
| | 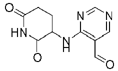 | 4-((2,6-dioxopiperidin-3-yl)amino)pyrimidine-5-carbaldehyde | 4-((2,6-dioxopiperidin-3-yl)amino)pyrimidine-5-carbaldehyde |
| | 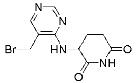 | 3-((5-(bromomethyl)pyrimidin-4-yl)amino)piperidine-2,6-dione | 3-((5-(bromomethyl)pyrimidin-4-yl)amino)piperidine-2,6-dione |
| GT-06986 | 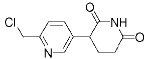 | 3-(6-(chloromethyl)pyridin-3-yl)piperidine-2,6-dione | 3-(6-(chloromethyl)pyridin-3-yl)piperidine-2,6-dione |
| | 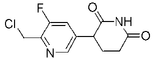 | 3-(6-(chloromethyl)-5-fluoropyridin-3-yl)piperidine-2,6-dione | 3-(6-(chloromethyl)-5-fluoropyridin-3-yl)piperidine-2,6-dione |
| | 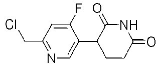 | 3-(6-(chloromethyl)-4-fluoropyridin-3-yl)piperidine-2,6-dione | 3-(6-(chloromethyl)-4-fluoropyridin-3-yl)piperidine-2,6-dione |
| | 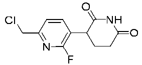 | 3-(6-(chloromethyl)-2-fluoropyridin-3-yl)piperidine-2,6-dione | 3-(6-(chloromethyl)-2-fluoropyridin-3-yl)piperidine-2,6-dione |
| | 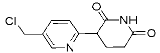 | 3-(5-(chloromethyl)pyridin-2-yl)piperidine-2,6-dione | 3-(5-(chloromethyl)pyridin-2-yl)piperidine-2,6-dione |
| | 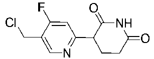 | 3-(5-(chloromethyl)-4-fluoropyridin-2-yl)piperidine-2,6-dione | 3-(5-(chloromethyl)-4-fluoropyridin-2-yl)piperidine-2,6-dione |
| | 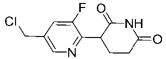 | 3-(5-(chloromethyl)-3-fluoropyridin-2-yl)piperidine-2,6-dione | 3-(5-(chloromethyl)-3-fluoropyridin-2-yl)piperidine-2,6-dione |
| | 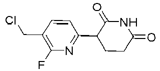 | 3-(5-(chloromethyl)-6-fluoropyridin-2-yl)piperidine-2,6-dione | 3-(5-(chloromethyl)-6-fluoropyridin-2-yl)piperidine-2,6-dione |
| | 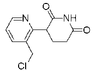 | 3-(3-(chloromethyl)pyridin-2-yl)piperidine-2,6-dione | 3-(3-(chloromethyl)pyridin-2-yl)piperidine-2,6-dione |
| | 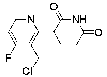 | 3-(3-(chloromethyl)-4-fluoropyridin-2-yl)piperidine-2,6-dione | 3-(3-(chloromethyl)-4-fluoropyridin-2-yl)piperidine-2,6-dione |
| | 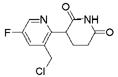 | 3-(3-(chloromethyl)-5-fluoropyridin-2-yl)piperidine-2,6-dione | 3-(3-(chloromethyl)-5-fluoropyridin-2-yl)piperidine-2,6-dione |
| | 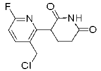 | 3-(3-(chloromethyl)-6-fluoropyridin-2-yl)piperidine-2,6-dione | 3-(3-(chloromethyl)-6-fluoropyridin-2-yl)piperidine-2,6-dione |
| GT-06991 | 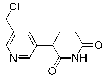 | 3-(5-(chloromethyl)pyridin-3-yl)piperidine-2,6-dione | 3-(5-(chloromethyl)pyridin-3-yl)piperidine-2,6-dione |
| | 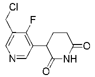 | 3-(5-(chloromethyl)-4-fluoropyridin-3-yl)piperidine-2,6-dione | 3-(5-(chloromethyl)-4-fluoropyridin-3-yl)piperidine-2,6-dione |
| | 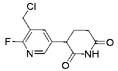 | 3-(5-(chloromethyl)-6-fluoropyridin-3-yl)piperidine-2,6-dione | 3-(5-(chloromethyl)-6-fluoropyridin-3-yl)piperidine-2,6-dione |
| | 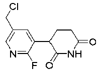 | 3-(5-(chloromethyl)-2-fluoropyridin-3-yl)piperidine-2,6-dione | 3-(5-(chloromethyl)-2-fluoropyridin-3-yl)piperidine-2,6-dione |
| | 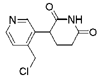 | 3-(4-(chloromethyl)pyridin-3-yl)piperidine-2,6-dione | 3-(4-(chloromethyl)pyridin-3-yl)piperidine-2,6-dione |
| | 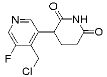 | 3-(4-(chloromethyl)-5-fluoropyridin-3-yl)piperidine-2,6-dione | 3-(4-(chloromethyl)-5-fluoropyridin-3-yl)piperidine-2,6-dione |
| | 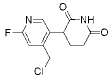 | 3-(4-(chloromethyl)-6-fluoropyridin-3-yl)piperidine-2,6-dione | 3-(4-(chloromethyl)-6-fluoropyridin-3-yl)piperidine-2,6-dione |
| | 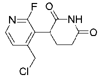 | 3-(4-(chloromethyl)-2-fluoropyridin-3-yl)piperidine-2,6-dione | 3-(4-(chloromethyl)-2-fluoropyridin-3-yl)piperidine-2,6-dione |
| GT-07013 | 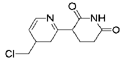 | 3-(4-(chloromethyl)pyridin-2-yl)piperidine-2,6-dione | 3-(4-(chloromethyl)pyridin-2-yl)piperidine-2,6-dione |
| | 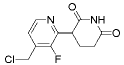 | 3-(4-(chloromethyl)-3-fluoropyridin-2-yl)piperidine-2,6-dione | 3-(4-(chloromethyl)-3-fluoropyridin-2-yl)piperidine-2,6-dione |
| | 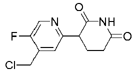 | 3-(4-(chloromethyl)-5-fluoropyridin-2-yl)piperidine-2,6-dione | 3-(4-(chloromethyl)-5-fluoropyridin-2-yl)piperidine-2,6-dione |
| | 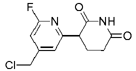 | 3-(4-(chloromethyl)-6-fluoropyridin-2-yl)piperidine-2,6-dione | 3-(4-(chloromethyl)-6-fluoropyridin-2-yl)piperidine-2,6-dione |
| | 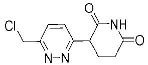 | 3-(6-(chloromethyl)pyridazin-3-yl)piperidine-2,6-dione | 3-(6-(chloromethyl)pyridazin-3-yl)piperidine-2,6-dione |
| | 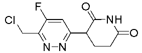 | 3-(6-(chloromethyl)-5-fluoropyridazin-3-yl)piperidine-2,6-dione | 3-(6-(chloromethyl)-5-fluoropyridazin-3-yl)piperidine-2,6-dione |
| | 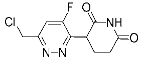 | 3-(6-(chloromethyl)-4-fluoropyridazin-3-yl)piperidine-2,6-dione | 3-(6-(chloromethyl)-4-fluoropyridazin-3-yl)piperidine-2,6-dione |
| | 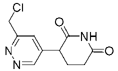 | 3-(6-(chloromethyl)pyridazin-4-yl)piperidine-2,6-dione | 3-(6-(chloromethyl)pyridazin-4-yl)piperidine-2,6-dione |
| | 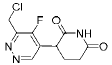 | 3-(6-(chloromethyl)-5-fluoropyridazin-4-yl)piperidine-2,6-dione | 3-(6-(chloromethyl)-5-fluoropyridazin-4-yl)piperidine-2,6-dione |
| | 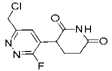 | 3-(6-(chloromethyl)-3-fluoropyridazin-4-yl)piperidine-2,6-dione | 3-(6-(chloromethyl)-3-fluoropyridazin-4-yl)piperidine-2,6-dione |
| | 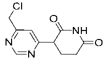 | 3-(6-(chloromethyl)pyrimidin-4-yl)piperidine-2,6-dione | 3-(6-(chloromethyl)pyrimidin-4-yl)piperidine-2,6-dione |
| | 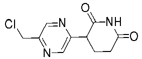 | 3-(5-(chloromethyl)pyrazin-2-yl)piperidine-2,6-dione | 3-(5-(chloromethyl)pyrazin-2-yl)piperidine-2,6-dione |
| | 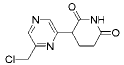 | 3-(6-(chloromethyl)pyrazin-2-yl)piperidine-2,6-dione | 3-(6-(chloromethyl)pyrazin-2-yl)piperidine-2,6-dione |
| | 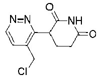 | 3-(4-(chloromethyl)pyridazin-3-yl)piperidine-2,6-dione | 3-(4-(chloromethyl)pyridazin-3-yl)piperidine-2,6-dione |
| | 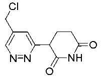 | 3-(5-(chloromethyl)pyridazin-3-yl)piperidine-2,6-dione | 3-(5-(chloromethyl)pyridazin-3-yl)piperidine-2,6-dione |
| | 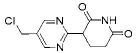 | 3-(5-(chloromethyl)pyrimidin-2-yl)piperidine-2,6-dione | 3-(5-(chloromethyl)pyrimidin-2-yl)piperidine-2,6-dione |
| | 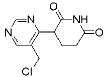 | 3-(5-(chloromethyl)pyrimidin-4-yl)piperidine-2,6-dione | 3-(5-(chloromethyl)pyrimidin-4-yl)piperidine-2,6-dione |
| GT-06984 | 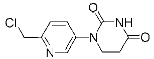 | 1-(6-(chloromethyl)pyridin-3-yl)dihydropyrimidine-2,4(1H,3H)-dione | 1-(6-(chloromethyl)pyridin-3-yl)dihydropyrimidine-2,4(1H,3H)-dione |
| GT-07557 | 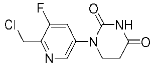 | 1-(6-(chloromethyl)-5-fluoropyridin-3-yl)dihydropyrimidine-2,4(1H,3H)-dione | 1-(6-(chloromethyl)-5-fluoropyridin-3-yl)dihydropyrimidine-2,4(1H,3H)-dione |
| | 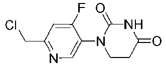 | 1-(6-(chloromethyl)-4-fluoropyridin-3-yl)dihydropyrimidine-2,4(1H,3H)-dione | 1-(6-(chloromethyl)-4-fluoropyridin-3-yl)dihydropyrimidine-2,4(1H,3H)-dione |
| | 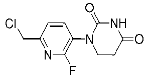 | 1-(6-(chloromethyl)-2-fluoropyridin-3-yl)dihydropyrimidine-2,4(1H,3H)-dione | 1-(6-(chloromethyl)-2-fluoropyridin-3-yl)dihydropyrimidine-2,4(1H,3H)-dione |
| GT-07009 | 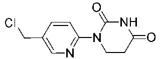 | 1-(5-(chloromethyl)pyridin-2-yl)dihydropyrimidine-2,4(1H,3H)-dione | 1-(5-(chloromethyl)pyridin-2-yl)dihydropyrimidine-2,4(1H,3H)-dione |
| | 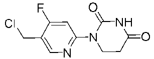 | 1-(5-(chloromethyl)-4-fluoropyridin-2-yl)dihydropyrimidine-2,4(1H,3H)-dione | 1-(5-(chloromethyl)-4-fluoropyridin-2-yl)dihydropyrimidine-2,4(1H,3H)-dione |
| | 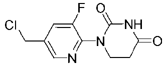 | 1-(5-(chloromethyl)-3-fluoropyridin-2-yl)dihydropyrimidine-2,4(1H,3H)-dione | 1-(5-(chloromethyl)-3-fluoropyridin-2-yl)dihydropyrimidine-2,4(1H,3H)-dione |
| | 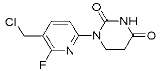 | 1-(5-(chloromethyl)-6-fluoropyridin-2-yl)dihydropyrimidine-2,4(1H,3H)-dione | 1-(5-(chloromethyl)-6-fluoropyridin-2-yl)dihydropyrimidine-2,4(1H,3H)-dione |
| | 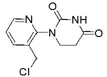 | 1-(3-(chloromethyl)pyridin-2-yl)dihydropyrimidine-2,4(lH,3H)-dione | 1-(3-(chloromethyl)pyridin-2-yl)dihydropyrimidine-2,4(1H,3H)-dione |
| | 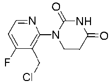 | 1-(3-(chloromethyl)-4-fluoropyridin-2-yl)dihydropyrimidine-2,4(1H,3H)-dione | 1-(3-(chloromethyl)-4-fluoropyridin-2-yl)dihydropyrimidine-2,4(1H,3H)-dione |
| | 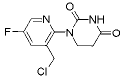 | 1-(3-(chloromethyl)-5-fluoropyridin-2-yl)dihydropyrimidine-2,4(1H,3H)-dione | 1-(3-(chloromethyl)-5-fluoropyridin-2-yl)dihydropyrimidine-2,4(1H,3H)-dione |
| | 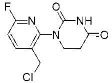 | 1-(3-(chloromethyl)-6-fluoropyridin-2-yl)dihydropyrimidine-2,4(1H,3H)-dione | 1-(3-(chloromethyl)-6-fluoropyridin-2-yl)dihydropyrimidine-2,4(1H,3H)-dione |
| GT-07047 | 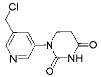 | 1-(5-(chloromethyl)pyridin-3-yl)dihydropyrimidine-2,4(1H,3H)-dione | 1-(5-(chloromethyl)pyridin-3-yl)dihydropyrimidine-2,4(1H,3H)-dione |
| | 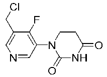 | 1-(5-(chloromethyl)-4-fluoropyridin-3-yl)dihydropyrimidine-2,4(1H,3H)-dione | 1-(5-(chloromethyl)-4-fluoropyridin-3-yl)dihydropyrimidine-2,4(1H,3H)-dione |
| GT-08128 | 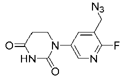 | 1-(5-(azidomethyl)-6-fluoropyridin-3-yl)dihydropyrimidine-2,4(1H,3H)-dione | 1-(5-(azidomethyl)-6-fluoropyridin-3-yl)dihydropyrimidine-2,4(1H,3H)-dione |
| GT-07633 | 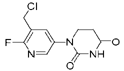 | 1-(5-(chloromethyl)-6-fluoropyridin-3-yl)dihydropyrimidine-2,4(1H,3H)-dione | 1-(5-(chloromethyl)-6-fluoropyridin-3-yl)dihydropyrimidine-2,4(1H,3H)-dione |
| | 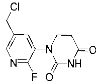 | 1-(5-(chloromethyl)-2-fluoropyridin-3-yl)dihydropyrimidine-2,4(1H,3H)-dione | 1-(5-(chloromethyl)-2-fluoropyridin-3-yl)dihydropyrimidine-2,4(1H,3H)-dione |
| | 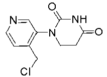 | 1-(4-(chloromethyl)pyridin-3-yl)dihydropyrimidine-2,4(lH,3H)-dione | 1-(4-(chloromethyl)pyridin-3-yl)dihydropyrimidine-2,4(1H,3H)-dione |
| | 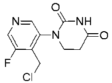 | 1-(4-(chloromethyl)-5-fluoropyridin-3-yl)dihydropyrimidine-2,4(1H,3H)-dione | 1-(4-(chloromethyl)-5-fluoropyridin-3-yl)dihydropyrimidine-2,4(1H,3H)-dione |
| | 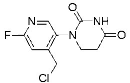 | 1-(4-(chloromethyl)-6-fluoropyridin-3-yl)dihydropyrimidine-2,4(1H,3H)-dione | 1-(4-(chloromethyl)-6-fluoropyridin-3-yl)dihydropyrimidine-2,4(1H,3H)-dione |
| | 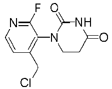 | 1-(4-(chloromethyl)-2-fluoropyridin-3-yl)dihydropyrimidine-2,4(1H,3H)-dione | 1-(4-(chloromethyl)-2-fluoropyridin-3-yl)dihydropyrimidine-2,4(1H,3H)-dione |
| GT-08121 | 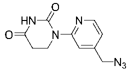 | 1-(4-(azidomethyl)pyridin-2-yl)dihydropyrimidine-2,4(1H,3H)-dione | 1-(4-(azidomethyl)pyridin-2-yl)dihydropyrimidine-2,4(1H,3H)-dione |
| GT-07078 | 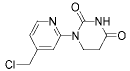 | 1-(4-(chloromethyl)pyridin-2-yl)dihydropyrimidine-2,4(1H,3H)-dione | 1-(4-(chloromethyl)pyridin-2-yl)dihydropyrimidine-2,4(1H,3H)-dione |
| | 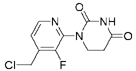 | 1-(4-(chloromethyl)-3-fluoropyridin-2-yl)dihydropyrimidine-2,4(1H,3H)-dione | 1-(4-(chloromethyl)-3-fluoropyridin-2-yl)dihydropyrimidine-2,4(1H,3H)-dione |
| GT-08127 | 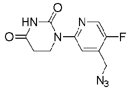 | 1-(4-(azidomethyl)-5-fluoropyridin-2-yl)dihydropyrimidine-2,4(1H,3H)-dione | 1-(4-(azidomethyl)-5-fluoropyridin-2-yl)dihydropyrimidine-2,4(1H,3H)-dione |
| GT-07199 | 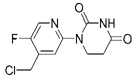 | 1-(4-(chloromethyl)-5-fluoropyridin-2-yl)dihydropyrimidine-2,4(1H,3H)-dione | 1-(4-(chloromethyl)-5-fluoropyridin-2-yl)dihydropyrimidine-2,4(1H,3H)-dione |
| | 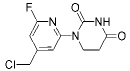 | 1-(4-(chloromethyl)-6-fluoropyridin-2-yl)dihydropyrimidine-2,4(1H,3H)-dione | 1-(4-(chloromethyl)-6-fluoropyridin-2-yl)dihydropyrimidine-2,4(1H,3H)-dione |
| | 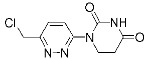 | 1-(6-(chloromethyl)pyridazin-3-yl)dihydropyrimidine-2,4(1H,3H)-dione | 1-(6-(chloromethyl)pyridazin-3-yl)dihydropyrimidine-2,4(1H,3H)-dione |
| | 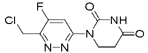 | 1-(6-(chloromethyl)-5-fluoropyridazin-3-yl)dihydropyrimidine-2,4(1H,3H)-dione | 1-(6-(chloromethyl)-5-fluoropyridazin-3-yl)dihydropyrimidine-2,4(1H,3H)-dione |
| | 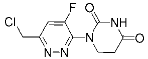 | 1-(6-(chloromethyl)-4-fluoropyridazin-3-yl)dihydropyrimidine-2,4(1H,3H)-dione | 1-(6-(chloromethyl)-4-fluoropyridazin-3-yl)dihydropyrimidine-2,4(1H,3H)-dione |
| | 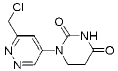 | 1-(6-(chloromethyl)pyridazin-4-yl)dihydropyrimidine-2,4(1H,3H)-dione | 1-(6-(chloromethyl)pyridazin-4-yl)dihydropyrimidine-2,4(1H,3H)-dione |
| | 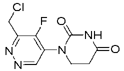 | 1-(6-(chloromethyl)-5-fluoropyridazin-4-yl)dihydropyrimidine-2,4(1H,3H)-dione | 1-(6-(chloromethyl)-5-fluoropyridazin-4-yl)dihydropyrimidine-2,4(1H,3H)-dione |
| | 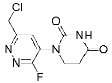 | 1-(6-(chloromethyl)-3-fluoropyridazin-4-yl)dihydropyrimidine-2,4(1H,3H)-dione | 1-(6-(chloromethyl)-3-fluoropyridazin-4-yl)dihydropyrimidine-2,4(1H,3H)-dione |
| | 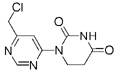 | 1-(6-(chloromethyl)pyrimidin-4-yl)dihydropyrimidine-2,4(1H,3H)-dione | 1-(6-(chloromethyl)pyrimidin-4-yl)dihydropyrimidine-2,4(1H,3H)-dione |
| GT-07635 | 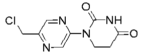 | 1-(5-(chloromethyl)pyrazin-2-yl)dihydropyrimidine-2,4(1H,3H)-dione | 1-(5-(chloromethyl)pyrazin-2-yl)dihydropyrimidine-2,4(1H,3H)-dione |
| GT-07728 | 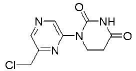 | 1-(6-(chloromethyl)pyrazin-2-yl)dihydropyrimidine-2,4(1H,3H)-dione | 1-(6-(chloromethyl)pyrazin-2-yl)dihydropyrimidine-2,4(1H,3H)-dione |
| | 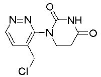 | 1-(4-(chloromethyl)pyridazin-3-yl)dihydropyrimidine-2,4(1H,3H)-dione | 1-(4-(chloromethyl)pyridazin-3-yl)dihydropyrimidine-2,4(1H,3H)-dione |
| | 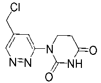 | 1-(5-(chloromethyl)pyridazin-3-yl)dihydropyrimidine-2,4(1H,3H)-dione | 1-(5-(chloromethyl)pyridazin-3-yl)dihydropyrimidine-2,4(1H,3H)-dione |
| | 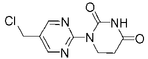 | 1-(5-(chloromethyl)pyrimidin-2-yl)dihydropyrimidine-2,4(1H,3H)-dione | 1-(5-(chloromethyl)pyrimidin-2-yl)dihydropyrimidine-2,4(1H,3H)-dione |
| | 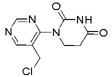 | 1-(5-(chloromethyl)pyrimidin-4-yl)dihydropyrimidine-2,4(1H,3H)-dione | 1-(5-(chloromethyl)pyrimidin-4-yl)dihydropyrimidine-2,4(1H,3H)-dione |
| | 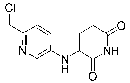 | 3-((6-(chloromethyl)pyridin-3-yl)amino)piperidine-2,6-dione | 3-((6-(chloromethyl)pyridin-3-yl)amino)piperidine-2,6-dione |
| | 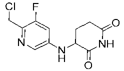 | 3-((6-(chloromethyl)-5-fluoropyridin-3-yl)amino)piperidine-2,6-dione | 3-((6-(chloromethyl)-5-fluoropyridin-3-yl)amino)piperidine-2,6-dione |
| | 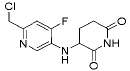 | 3-((6-(chloromethyl)-4-fluoropyridin-3-yl)amino)piperidine-2,6-dione | 3-((6-(chloromethyl)-4-fluoropyridin-3-yl)amino)piperidine-2,6-dione |
| | 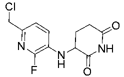 | 3-((6-(chloromethyl)-2-fluoropyridin-3-yl)amino)piperidine-2,6-dione | 3-((6-(chloromethyl)-2-fluoropyridin-3-yl)amino)piperidine-2,6-dione |
| | 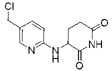 | 3-((5-(chloromethyl)pyridin-2-yl)amino)piperidine-2,6-dione | 3-((5-(chloromethyl)pyridin-2-yl)amino)piperidine-2,6-dione |
| | 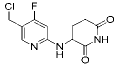 | 3-((5-(chloromethyl)-4-fluoropyridin-2-yl)amino)piperidine-2,6-dione | 3-((5-(chloromethyl)-4-fluoropyridin-2-yl)amino)piperidine-2,6-dione |
| | 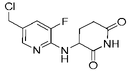 | 3-((5-(chloromethyl)-3-fluoropyridin-2-yl)amino)piperidine-2,6-dione | 3-((5-(chloromethyl)-3-fluoropyridin-2-yl)amino)piperidine-2,6-dione |
| | 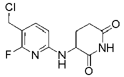 | 3-((5-(chloromethyl)-6-fluoropyridin-2-yl)amino)piperidine-2,6-dione | 3-((5-(chloromethyl)-6-fluoropyridin-2-yl)amino)piperidine-2,6-dione |
| | 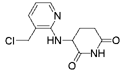 | 3-((3-(chloromethyl)pyridin-2-yl)amino)piperidine-2,6-dione | 3-((3-(chloromethyl)pyridin-2-yl)amino)piperidine-2,6-dione |
| | 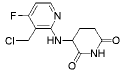 | 3-((3-(chloromethyl)-4-fluoropyridin-2-yl)amino)piperidine-2,6-dione | 3-((3-(chloromethyl)-4-fluoropyridin-2-yl)amino)piperidine-2,6-dione |
| | 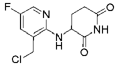 | 3-((3-(chloromethyl)-5-fluoropyridin-2-yl)amino)piperidine-2,6-dione | 3-((3-(chloromethyl)-5-fluoropyridin-2-yl)amino)piperidine-2,6-dione |
| | 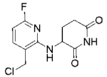 | 3-((3-(chloromethyl)-6-fluoropyridin-2-yl)amino)piperidine-2,6-dione | 3-((3-(chloromethyl)-6-fluoropyridin-2-yl)amino)piperidine-2,6-dione |
| | 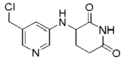 | 3-((5-(chloromethyl)pyridin-3-yl)amino)piperidine-2,6-dione | 3-((5-(chloromethyl)pyridin-3-yl)amino)piperidine-2,6-dione |
| | 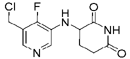 | 3-((5-(chloromethyl)-4-fluoropyridin-3-yl)amino)piperidine-2,6-dione | 3-((5-(chloromethyl)-4-fluoropyridin-3-yl)amino)piperidine-2,6-dione |
| | 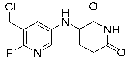 | 3-((5-(chloromethyl)-6-fluoropyridin-3-yl)amino)piperidine-2,6-dione | 3-((5-(chloromethyl)-6-fluoropyridin-3-yl)amino)piperidine-2,6-dione |
| | 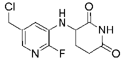 | 3-((5-(chloromethyl)-2-fluoropyridin-3-yl)amino)piperidine-2,6-dione | 3-((5-(chloromethyl)-2-fluoropyridin-3-yl)amino)piperidine-2,6-dione |
| | 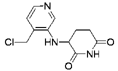 | 3-((4-(chloromethyl)pyridin-3-yl)amino)piperidine-2,6-dione | 3-((4-(chloromethyl)pyridin-3-yl)amino)piperidine-2,6-dione |
| | 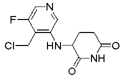 | 3-((4-(chloromethyl)-5-fluoropyridin-3-yl)amino)piperidine-2,6-dione | 3-((4-(chloromethyl)-5-fluoropyridin-3-yl)amino)piperidine-2,6-dione |
| | 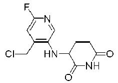 | 3-((4-(chloromethyl)-6-fluoropyridin-3-yl)amino)piperidine-2,6-dione | 3-((4-(chloromethyl)-6-fluoropyridin-3-yl)amino)piperidine-2,6-dione |
| | 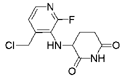 | 3-((4-(chloromethyl)-2-fluoropyridin-3-yl)amino)piperidine-2,6-dione | 3-((4-(chloromethyl)-2-fluoropyridin-3-yl)amino)piperidine-2,6-dione |
| | 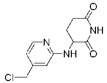 | 3-((4-(chloromethyl)pyridin-2-yl)amino)piperidine-2,6-dione | 3-((4-(chloromethyl)pyridin-2-yl)amino)piperidine-2,6-dione |
| | 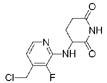 | 3-((4-(chloromethyl)-3-fluoropyridin-2-yl)amino)piperidine-2,6-dione | 3-((4-(chloromethyl)-3-fluoropyridin-2-yl)amino)piperidine-2,6-dione |
| | 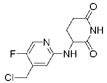 | 3-((4-(chloromethyl)-5-fluoropyridin-2-yl)amino)piperidine-2,6-dione | 3-((4-(chloromethyl)-5-fluoropyridin-2-yl)amino)piperidine-2,6-dione |
| | 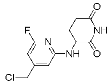 | 3-((4-(chloromethyl)-6-fluoropyridin-2-yl)amino)piperidine-2,6-dione | 3-((4-(chloromethyl)-6-fluoropyridin-2-yl)amino)piperidine-2,6-dione |
| | 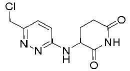 | 3-((6-(chloromethyl)pyridazin-3-yl)amino)piperidine-2,6-dione | 3-((6-(chloromethyl)pyridazin-3-yl)amino)piperidine-2,6-dione |
| | 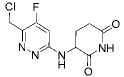 | 3-((6-(chloromethyl)-5-fluoropyridazin-3-yl)amino)piperidine-2,6-dione | 3-((6-(chloromethyl)-5-fluoropyridazin-3-yl)amino)piperidine-2,6-dione |
| | 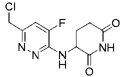 | 3-((6-(chloromethyl)-4-fluoropyridazin-3-yl)amino)piperidine-2,6-dione | 3-((6-(chloromethyl)-4-fluoropyridazin-3-yl)amino)piperidine-2,6-dione |
| | 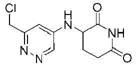 | 3-((6-(chloromethyl)pyridazin-4-yl)amino)piperidine-2,6-dione | 3-((6-(chloromethyl)pyridazin-4-yl)amino)piperidine-2,6-dione |
| | 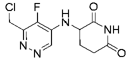 | 3-((6-(chloromethyl)-5-fluoropyridazin-4-yl)amino)piperidine-2,6-dione | 3-((6-(chloromethyl)-5-fluoropyridazin-4-yl)amino)piperidine-2,6-dione |
| | 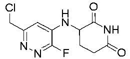 | 3-((6-(chloromethyl)-3-fluoropyridazin-4-yl)amino)piperidine-2,6-dione | 3-((6-(chloromethyl)-3-fluoropyridazin-4-yl)amino)piperidine-2,6-dione |
| | 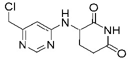 | 3-((6-(chloromethyl)pyrimidin-4-yl)amino)piperidine-2,6-dione | 3-((6-(chloromethyl)pyrimidin-4-yl)amino)piperidine-2,6-dione |
| | 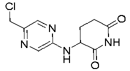 | 3-((5-(chloromethyl)pyrazin-2-yl)amino)piperidine-2,6-dione | 3-((5-(chloromethyl)pyrazin-2-yl)amino)piperidine-2,6-dione |
| | 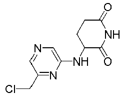 | 3-((6-(chloromethyl)pyrazin-2-yl)amino)piperidine-2,6-dione | 3-((6-(chloromethyl)pyrazin-2-yl)amino)piperidine-2,6-dione |
| | 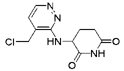 | 3-((4-(chloromethyl)pyridazin-3-yl)amino)piperidine-2,6-dione | 3-((4-(chloromethyl)pyridazin-3-yl)amino)piperidine-2,6-dione |
| | 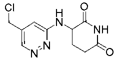 | 3-((5-(chloromethyl)pyridazin-3-yl)amino)piperidine-2,6-dione | 3-((5-(chloromethyl)pyridazin-3-yl)amino)piperidine-2,6-dione |
| | 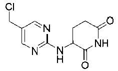 | 3-((5-(chloromethyl)pyrimidin-2-yl)amino)piperidine-2,6-dione | 3-((5-(chloromethyl)pyrimidin-2-yl)amino)piperidine-2,6-dione |
| | 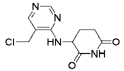 | 3-((5-(chloromethyl)pyrimidin-4-yl)amino)piperidine-2,6-dione | 3-((5-(chloromethyl)pyrimidin-4-yl)amino)piperidine-2,6-dione |
| GT-07010 | 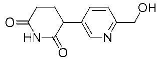 | 3-(6-(hydroxymethyl)pyridin-3-yl)piperidine-2,6-dione | 3-(6-(hydroxymethyl)pyridin-3-yl)piperidine-2,6-dione |
| GT-06987 | 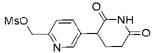 | (5-(2,6-dioxopiperidin-3-yl)pyridin-2-yl)methyl methanesulfonate | (5-(2,6-dioxopiperidin-3-yl)pyridin-2-yl)methyl methanesulfonate |
| GT-07049 | 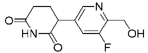 | 3-(5-fluoro-6-(hydroxymethyl)pyridin-3-yl)piperidine-2,6-dione | 3-(5-fluoro-6-(hydroxymethyl)pyridin-3-yl)piperidine-2,6-dione |
| | 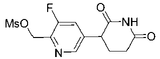 | (5-(2,6-dioxopiperidin-3-yl)-3-fluoropyridin-2-yl)methylmethanesulfonate | (5-(2,6-dioxopiperidin-3-yl)-3-fluoropyridin-2-yl)methylmethanesulfonate |
| GT-07558 | 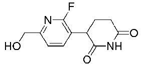 | 3-(2-fluoro-6-(hydroxymethyl)pyridin-3-yl)piperidine-2,6-dione | 3-(2-fluoro-6-(hydroxymethyl)pyridin-3-yl)piperidine-2,6-dione |
| | 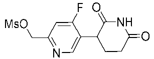 | (5-(2,6-dioxopiperidin-3-yl)-4-fluoropyridin-2-yl)methylmethanesulfonate | (5-(2,6-dioxopiperidin-3-yl)-4-fluoropyridin-2-yl)methylmethanesulfonate |
| | 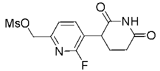 | (5-(2,6-dioxopiperidin-3-yl)-6-fluoropyridin-2-yl)methylmethanesulfonate | (5-(2,6-dioxopiperidin-3-yl)-6-fluoropyridin-2-yl)methylmethanesulfonate |
| GT-07075 | 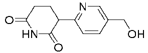 | 3-(5-(hydroxymethyl)pyridin-2-yl)piperidine-2,6-dione | 3-(5-(hydroxymethyl)pyridin-2-yl)piperidine-2,6-dione |
| | 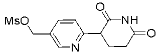 | (6-(2,6-dioxopiperidin-3-yl)pyridin-3-yl)methyl methanesulfonate | (6-(2,6-dioxopiperidin-3-yl)pyridin-3-yl)methyl methanesulfonate |
| | 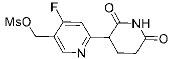 | (6-(2,6-dioxopiperidin-3-yl)-4-fluoropyridin-3-yl)methylmethanesulfonate | (6-(2,6-dioxopiperidin-3-yl)-4-fluoropyridin-3-yl)methylmethanesulfonate |
| GT-07443 | 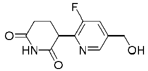 | 3-(3-fluoro-5-(hydroxymethyl)pyridin-2-yl)piperidine-2,6-dione | 3-(3-fluoro-5-(hydroxymethyl)pyridin-2-yl)piperidine-2,6-dione |
| | 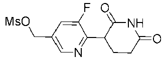 | (6-(2,6-dioxopiperidin-3-yl)-5-fluoropyridin-3-yl)methylmethanesulfonate | (6-(2,6-dioxopiperidin-3-yl)-5-fluoropyridin-3-yl)methylmethanesulfonate |
| | 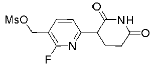 | (6-(2,6-dioxopiperidin-3-yl)-2-fluoropyridin-3-yl)methylmethanesulfonate | (6-(2,6-dioxopiperidin-3-yl)-2-fluoropyridin-3-yl)methylmethanesulfonate |
| GT-07370 | 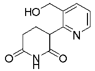 | 3-(3-(hydroxymethyl)pyridin-2-yl)piperidine-2,6-dione | 3-(3-(hydroxymethyl)pyridin-2-yl)piperidine-2,6-dione |
| | 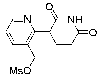 | (2-(2,6-dioxopiperidin-3-yl)pyridin-3-yl)methyl methanesulfonate | (2-(2,6-dioxopiperidin-3-yl)pyridin-3-yl)methyl methanesulfonate |
| | 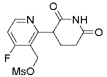 | (2-(2,6-dioxopiperidin-3-yl)-4-fluoropyridin-3-yl)methylmethanesulfonate | (2-(2,6-dioxopiperidin-3-yl)-4-fluoropyridin-3-yl)methylmethanesulfonate |
| | 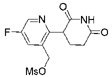 | (2-(2,6-dioxopiperidin-3-yl)-5-fluoropyridin-3-yl)methylmethanesulfonate | (2-(2,6-dioxopiperidin-3-yl)-5-fluoropyridin-3-yl)methylmethanesulfonate |
| | 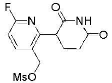 | (2-(2,6-dioxopiperidin-3-yl)-6-fluoropyridin-3-yl)methylmethanesulfonate | (2-(2,6-dioxopiperidin-3-yl)-6-fluoropyridin-3-yl)methylmethanesulfonate |
| GT-08130 | 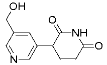 | 3-(5-(hydroxymethyl)pyridin-3-yl)piperidine-2,6-dione | 3-(5-(hydroxymethyl)pyridin-3-yl)piperidine-2,6-dione |
| | 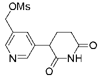 | (5-(2,6-dioxopiperidin-3-yl)pyridin-3-yl)methyl methanesulfonate | (5-(2,6-dioxopiperidin-3-yl)pyridin-3-yl)methyl methanesulfonate |
| | 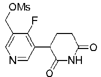 | (5-(2,6-dioxopiperidin-3-yl)-4-fluoropyridin-3-yl)methylmethanesulfonate | (5-(2,6-dioxopiperidin-3-yl)-4-fluoropyridin-3-yl)methylmethanesulfonate |
| GT-07074 | 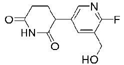 | 3-(6-fluoro-5-(hydroxymethyl)pyridin-3-yl)piperidine-2,6-dione | 3-(6-fluoro-5-(hydroxymethyl)pyridin-3-yl)piperidine-2,6-dione |
| | 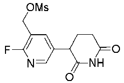 | (5-(2,6-dioxopiperidin-3-yl)-2-fluoropyridin-3-yl)methylmethanesulfonate | (5-(2,6-dioxopiperidin-3-yl)-2-fluoropyridin-3-yl)methylmethanesulfonate |
| | 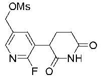 | (5-(2,6-dioxopiperidin-3-yl)-6-fluoropyridin-3-yl)methylmethanesulfonate | (5-(2,6-dioxopiperidin-3-yl)-6-fluoropyridin-3-yl)methylmethanesulfonate |
| | 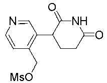 | (3-(2,6-dioxopiperidin-3-yl)pyridin-4-yl)methyl methanesulfonate | (3-(2,6-dioxopiperidin-3-yl)pyridin-4-yl)methyl methanesulfonate |
| | 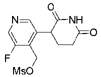 | (3-(2,6-dioxopiperidin-3-yl)-5-fluoropyridin-4-yl)methylmethanesulfonate | (3-(2,6-dioxopiperidin-3-yl)-5-fluoropyridin-4-yl)methylmethanesulfonate |
| | 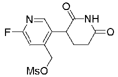 | (5-(2,6-dioxopiperidin-3-yl)-2-fluoropyridin-4-yl)methylmethanesulfonate | (5-(2,6-dioxopiperidin-3-yl)-2-fluoropyridin-4-yl)methylmethanesulfonate |
| | 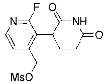 | (3-(2,6-dioxopiperidin-3-yl)-2-fluoropyridin-4-yl)methylmethanesulfonate | (3-(2,6-dioxopiperidin-3-yl)-2-fluoropyridin-4-yl)methylmethanesulfonate |
| GT-07050 | 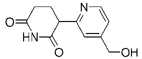 | 3-(4-(hydroxymethyl)pyridin-2-yl)piperidine-2,6-dione | 3-(4-(hydroxymethyl)pyridin-2-yl)piperidine-2,6-dione |
| | 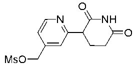 | (2-(2,6-dioxopiperidin-3-yl)pyridin-4-yl)methyl methanesulfonate | (2-(2,6-dioxopiperidin-3-yl)pyridin-4-yl)methyl methanesulfonate |
| | 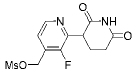 | (2-(2,6-dioxopiperidin-3-yl)-3-fluoropyridin-4-yl)methylmethanesulfonate | (2-(2,6-dioxopiperidin-3-yl)-3-fluoropyridin-4-yl)methylmethanesulfonate |
| GT-07444 | 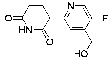 | 3-(5-fluoro-4-(hydroxymethyl)pyridin-2-yl)piperidine-2,6-dione | 3-(5-fluoro-4-(hydroxymethyl)pyridin-2-yl)piperidine-2,6-dione |
| | 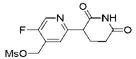 | (2-(2,6-dioxopiperidin-3-yl)-5-fluoropyridin-4-yl)methylmethanesulfonate | (2-(2,6-dioxopiperidin-3-yl)-5-fluoropyridin-4-yl)methylmethanesulfonate |
| | 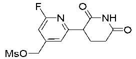 | (2-(2,6-dioxopiperidin-3-yl)-6-fluoropyridin-4-yl)methylmethanesulfonate | (2-(2,6-dioxopiperidin-3-yl)-6-fluoropyridin-4-yl)methylmethanesulfonate |
| GT-07636 | 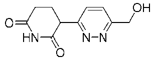 | 3-(6-(hydroxymethyl)pyridazin-3-yl)piperidine-2,6-dione | 3-(6-(hydroxymethyl)pyridazin-3-yl)piperidine-2,6-dione |
| | 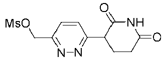 | (6-(2,6-dioxopiperidin-3-yl)pyridazin-3-yl)methyl methanesulfonate | (6-(2,6-dioxopiperidin-3-yl)pyridazin-3-yl)methyl methanesulfonate |
| | 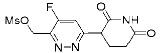 | (6-(2,6-dioxopiperidin-3-yl)-4-fluoropyridazin-3-yl)methylmethanesulfonate | (6-(2,6-dioxopiperidin-3-yl)-4-fluoropyridazin-3-yl)methylmethanesulfonate |
| | 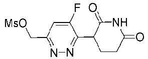 | (6-(2,6-dioxopiperidin-3-yl)-5-fluoropyridazin-3-yl)methylmethanesulfonate | (6-(2,6-dioxopiperidin-3-yl)-5-fluoropyridazin-3-yl)methylmethanesulfonate |
| | 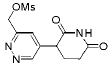 | (5-(2,6-dioxopiperidin-3-yl)pyridazin-3-yl)methyl methanesulfonate | (5-(2,6-dioxopiperidin-3-yl)pyridazin-3-yl)methyl methanesulfonate |
| | 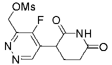 | (5-(2,6-dioxopiperidin-3-yl)-4-fluoropyridazin-3-yl)methylmethanesulfonate | (5-(2,6-dioxopiperidin-3-yl)-4-fluoropyridazin-3-yl)methylmethanesulfonate |
| | 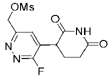 | (5-(2,6-dioxopiperidin-3-yl)-6-fluoropyridazin-3-yl)methylmethanesulfonate | (5-(2,6-dioxopiperidin-3-yl)-6-fluoropyridazin-3-yl)methylmethanesulfonate |
| | 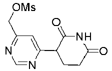 | (6-(2,6-dioxopiperidin-3-yl)pyrimidin-4-yl)methyl methanesulfonate | (6-(2,6-dioxopiperidin-3-yl)pyrimidin-4-yl)methyl methanesulfonate |
| | 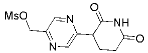 | (5-(2,6-dioxopiperidin-3-yl)pyrazin-2-yl)methyl methanesulfonate | (5-(2,6-dioxopiperidin-3-yl)pyrazin-2-yl)methyl methanesulfonate |
| | 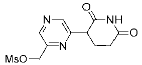 | (6-(2,6-dioxopiperidin-3-yl)pyrazin-2-yl)methyl methanesulfonate | (6-(2,6-dioxopiperidin-3-yl)pyrazin-2-yl)methyl methanesulfonate |
| | 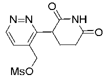 | (3-(2,6-dioxopiperidin-3-yl)pyridazin-4-yl)methyl methanesulfonate | (3-(2,6-dioxopiperidin-3-yl)pyridazin-4-yl)methyl methanesulfonate |
| | 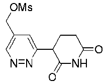 | (6-(2,6-dioxopiperidin-3-yl)pyridazin-4-yl)methyl methanesulfonate | (6-(2,6-dioxopiperidin-3-yl)pyridazin-4-yl)methyl methanesulfonate |
| | 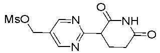 | (2-(2,6-dioxopiperidin-3-yl)pyrimidin-5-yl)methyl methanesulfonate | (2-(2,6-dioxopiperidin-3-yl)pyrimidin-5-yl)methyl methanesulfonate |
| | 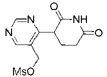 | (4-(2,6-dioxopiperidin-3-yl)pyrimidin-5-yl)methyl methanesulfonate | (4-(2,6-dioxopiperidin-3-yl)pyrimidin-5-yl)methyl methanesulfonate |
| GT-08010 | 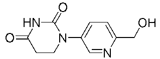 | 1-(6-(hydroxymethyl)pyridin-3-yl)dihydropyrimidine-2,4(1H,3H)-dione | 1-(6-(hydroxymethyl)pyridin-3-yl)dihydropyrimidine-2,4(1H,3H)-dione |
| | 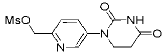 | (5-(2,4-dioxotetrahydropyrimidin-1 (2H)-yl)pyridin-2-yl)methyl methanesulfonate | (5-(2,4-dioxotetrahydropyrimidin-1(2H)-yl)pyridin-2-yl)methyl methanesulfonate |
| GT-07555 | 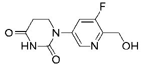 | 1-(5-fluoro-6-(hydroxymethyl)pyridin-3-yl)dihydropyrimidine-2,4(1H,3H)-dione | 1-(5-fluoro-6-(hydroxymethyl)pyridin-3-yl)dihydropyrimidine-2,4(1H,3H)-dione |
| | 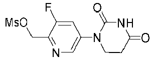 | (5-(2,4-dioxotetrahydropyrimidin-1 (2H)-yl)-3-fluoropyridin-2-yl)methylmethanesulfonate | (5-(2,4-dioxotetrahydropyrimidin-1(2H)-yl)-3-fluoropyridin-2-yl)methylmethanesulfonate |
| | 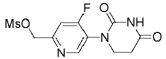 | (5-(2,4-dioxotetrahydropyrimidin-1 (2H)-yl)-4-fluoropyridin-2-yl)methylmethanesulfonate | (5-(2,4-dioxotetrahydropyrimidin-1(2H)-yl)-4-fluoropyridin-2-yl)methylmethanesulfonate |
| | 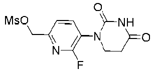 | (5-(2,4-dioxotetrahydropyrimidin-1 (2H)-yl)-6-fluoropyridin-2-yl)methylmethanesulfonate | (5-(2,4-dioxotetrahydropyrimidin-1(2H)-yl)-6-fluoropyridin-2-yl)methylmethanesulfonate |
| | 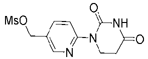 | (6-(2,4-dioxotetrahydropyrimidin-1(2H)-yl)pyridin-3-yl)methyl methanesulfonate | (6-(2,4-dioxotetrahydropyrimidin-1(2H)-yl)pyridin-3-yl)methyl methanesulfonate |
| | 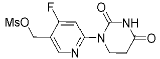 | (6-(2,4-dioxotetrahydropyrimidin-1(2H)-yl)-4-fluoropyridin-3-yl)methylmethanesulfonate | (6-(2,4-dioxotetrahydropyrimidin-1(2H)-yl)-4-fluoropyridin-3-yl)methylmethanesulfonate |
| | 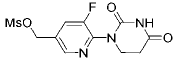 | (6-(2,4-dioxotetrahydropyrimidin-1(2H)-yl)-5-fluoropyridin-3-yl)methylmethanesulfonate | (6-(2,4-dioxotetrahydropyrimidin-1(2H)-yl)-5-fluoropyridin-3-yl)methylmethanesulfonate |
| | 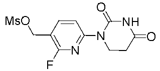 | (6-(2,4-dioxotetrahydropyrimidin-1(2H)-yl)-2-fluoropyridin-3-yl)methylmethanesulfonate | (6-(2,4-dioxotetrahydropyrimidin-1(2H)-yl)-2-fluoropyridin-3-yl)methylmethanesulfonate |
| | 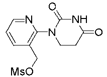 | (2-(2,4-dioxotetrahydropyrimidin-1(2H)-yl)pyridin-3-yl)methyl methanesulfonate | (2-(2,4-dioxotetrahydropyrimidin-1(2H)-yl)pyridin-3-yl)methyl methanesulfonate |
| | 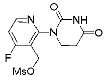 | (2-(2,4-dioxotetrahydropyrimidin-1(2H)-yl)-4-fluoropyridin-3-yl)methylmethanesulfonate | (2-(2,4-dioxotetrahydropyrimidin-1(2H)-yl)-4-fluoropyridin-3-yl)methylmethanesulfonate |
| | 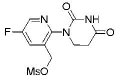 | (2-(2,4-dioxotetrahydropyrimidin-1(2H)-yl)-5-fluoropyridin-3-yl)methylmethanesulfonate | (2-(2,4-dioxotetrahydropyrimidin-1(2H)-yl)-5-fluoropyridin-3-yl)methylmethanesulfonate |
| | 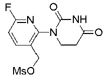 | (2-(2,4-dioxotetrahydropyrimidin-1(2H)-yl)-6-fluoropyridin-3-yl)methylmethanesulfonate | (2-(2,4-dioxotetrahydropyrimidin-1(2H)-yl)-6-fluoropyridin-3-yl)methylmethanesulfonate |
| GT-08124 | 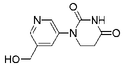 | 1-(5-(hydroxymethyl)pyridin-3-yl)dihydropyrimidine-2,4(1H,3H)-dione | 1-(5-(hydroxymethyl)pyridin-3-yl)dihydropyrimidine-2,4(1H,3H)-dione |
| | 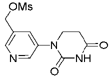 | (5-(2,4-dioxotetrahydropyrimidin-1(2H)-yl)pyridin-3-yl)methyl methanesulfonate | (5-(2,4-dioxotetrahydropyrimidin-1(2H)-yl)pyridin-3-yl)methyl methanesulfonate |
| | 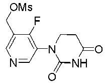 | (5-(2,4-dioxotetrahydropyrimidin-1(2H)-yl)-4-fluoropyridin-3-yl)methylmethanesulfonate | (5-(2,4-dioxotetrahydropyrimidin-1(2H)-yl)-4-fluoropyridin-3-yl)methylmethanesulfonate |
| | 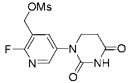 | (5-(2,4-dioxotetrahydropyrimidin-1(2H)-yl)-2-fluoropyridin-3-yl)methylmethanesulfonate | (5-(2,4-dioxotetrahydropyrimidin-1(2H)-yl)-2-fluoropyridin-3-yl)methylmethanesulfonate |
| | 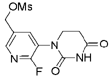 | (5-(2,4-dioxotetrahydropyrimidin-1(2H)-yl)-6-fluoropyridin-3-yl)methylmethanesulfonate | (5-(2,4-dioxotetrahydropyrimidin-1(2H)-yl)-6-fluoropyridin-3-yl)methylmethanesulfonate |
| | 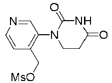 | (3-(2,4-dioxotetrahydropyrimidin-1(2H)-yl)pyridin-4-yl)methyl methanesulfonate | (3-(2,4-dioxotetrahydropyrimidin-1(2H)-yl)pyridin-4-yl)methyl methanesulfonate |
| | 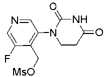 | (3-(2,4-dioxotetrahydropyrimidin-1(2H)-yl)-5-fluoropyridin-4-yl)methylmethanesulfonate | (3-(2,4-dioxotetrahydropyrimidin-1(2H)-yl)-5-fluoropyridin-4-yl)methylmethanesulfonate |
| | 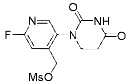 | (5-(2,4-dioxotetrahydropyrimidin-1(2H)-yl)-2-fluoropyridin-4-yl)methylmethanesulfonate | (5-(2,4-dioxotetrahydropyrimidin-1(2H)-yl)-2-fluoropyridin-4-yl)methylmethanesulfonate |
| | 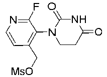 | (3-(2,4-dioxotetrahydropyrimidin-1(2H)-yl)-2-fluoropyridin-4-yl)methylmethanesulfonate | (3-(2,4-dioxotetrahydropyrimidin-1(2H)-yl)-2-fluoropyridin-4-yl)methylmethanesulfonate |
| GT-08120 | 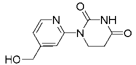 | 1-(4-(hydroxymethyl)pyridin-2-yl)dihydropyrimidine-2,4(1H,3H)-dione | 1-(4-(hydroxymethyl)pyridin-2-yl)dihydropyrimidine-2,4(1H,3H)-dione |
| | 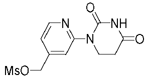 | (2-(2,4-dioxotetrahydropyrimidin-1(2H)-yl)pyridin-4-yl)methyl methanesulfonate | (2-(2,4-dioxotetrahydropyrimidin-1(2H)-yl)pyridin-4-yl)methyl methanesulfonate |
| | 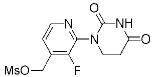 | (2-(2,4-dioxotetrahydropyrimidin-1(2H)-yl)-3-fluoropyridin-4-yl)methylmethanesulfonate | (2-(2,4-dioxotetrahydropyrimidin-1(2H)-yl)-3-fluoropyridin-4-yl)methylmethanesulfonate |
| | 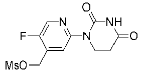 | (2-(2,4-dioxotetrahydropyrimidin-1(2H)-yl)-5-fluoropyridin-4-yl)methylmethanesulfonate | (2-(2,4-dioxotetrahydropyrimidin-1(2H)-yl)-5-fluoropyridin-4-yl)methylmethanesulfonate |
| | 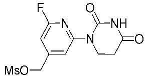 | (2-(2,4-dioxotetrahydropyrimidin-1(2H)-yl)-6-fluoropyridin-4-yl)methylmethanesulfonate | (2-(2,4-dioxotetrahydropyrimidin-1(2H)-yl)-6-fluoropyridin-4-yl)methylmethanesulfonate |
| | 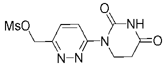 | (6-(2,4-dioxotetrahydropyrimidin-1(2H)-yl)pyridazin-3-yl)methyl methanesulfonate | (6-(2,4-dioxotetrahydropyrimidin-1(2H)-yl)pyridazin-3-yl)methyl methanesulfonate |
| | 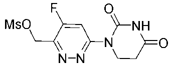 | (6-(2,4-dioxotetrahydropyrimidin-1(2H)-yl)-4-fluoropyridazin-3-yl)methylmethanesulfonate | (6-(2,4-dioxotetrahydropyrimidin-1(2H)-yl)-4-fluoropyridazin-3-yl)methylmethanesulfonate |
| | 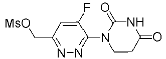 | (6-(2,4-dioxotetrahydropyrimidin-1(2H)-yl)-5-fluoropyridazin-3-yl)methylmethanesulfonate | (6-(2,4-dioxotetrahydropyrimidin-1(2H)-yl)-5-fluoropyridazin-3-yl)methylmethanesulfonate |
| | 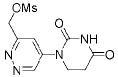 | (5-(2,4-dioxotetrahydropyrimidin-1(2H)-yl)pyridazin-3-yl)methyl methanesulfonate | (5-(2,4-dioxotetrahydropyrimidin-1(2H)-yl)pyridazin-3-yl)methyl methanesulfonate |
| | 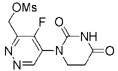 | (5-(2,4-dioxotetrahydropyrimidin-1(2H)-yl)-4-fluoropyridazin-3-yl)methylmethanesulfonate | (5-(2,4-dioxotetrahydropyrimidin-1(2H)-yl)-4-fluoropyridazin-3-yl)methylmethanesulfonate |
| | 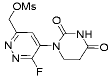 | (5-(2,4-dioxotetrahydropyrimidin-1(2H)-yl)-6-fluoropyridazin-3-yl)methylmethanesulfonate | (5-(2,4-dioxotetrahydropyrimidin-1(2H)-yl)-6-fluoropyridazin-3-yl)methylmethanesulfonate |
| | 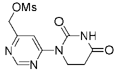 | (6-(2,4-dioxotetrahydropyrimidin-1(2H)-yl)pyrimidin-4-yl)methyl methanesulfonate | (6-(2,4-dioxotetrahydropyrimidin-1(2H)-yl)pyrimidin-4-yl)methyl methanesulfonate |
| | 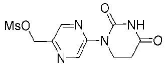 | (5-(2,4-dioxotetrahydropyrimidin-1(2H)-yl)pyrazin-2-yl)methyl methanesulfonate | (5-(2,4-dioxotetrahydropyrimidin-1(2H)-yl)pyrazin-2-yl)methyl methanesulfonate |
| | 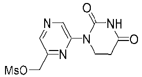 | (6-(2,4-dioxotetrahydropyrimidin-1(2H)-yl)pyrazin-2-yl)methyl methanesulfonate | (6-(2,4-dioxotetrahydropyrimidin-1(2H)-yl)pyrazin-2-yl)methyl methanesulfonate |
| | 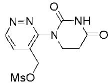 | (3-(2,4-dioxotetrahydropyrimidin-1(2H)-yl)pyridazin-4-yl)methyl methanesulfonate | (3-(2,4-dioxotetrahydropyrimidin-1(2H)-yl)pyridazin-4-yl)methyl methanesulfonate |
| | 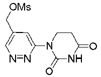 | (6-(2,4-dioxotetrahydropyrimidin-1(2H)-yl)pyridazin-4-yl)methyl methanesulfonate | (6-(2,4-dioxotetrahydropyrimidin-1(2H)-yl)pyridazin-4-yl)methyl methanesulfonate |
| | 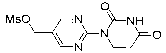 | (2-(2,4-dioxotetrahydropyrimidin-1(2H)-yl)pyrimidin-5-yl)methyl methanesulfonate | (2-(2,4-dioxotetrahydropyrimidin-1(2H)-yl)pyrimidin-5-yl)methyl methanesulfonate |
| | 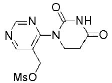 | (4-(2,4-dioxotetrahydropyrimidin-1(2H)-yl)pyrimidin-5-yl)methyl methanesulfonate | (4-(2,4-dioxotetrahydropyrimidin-1(2H)-yl)pyrimidin-5-yl)methyl methanesulfonate |
| | 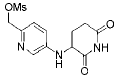 | (5-((2,6-dioxopiperidin-3-yl)amino)pyridin-2-yl)methyl methanesulfonate | (5-((2,6-dioxopiperidin-3-yl)amino)pyridin-2-yl)methyl methanesulfonate |
| | 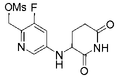 | (5-((2,6-dioxopiperidin-3-yl)amino)-3-fluoropyridin-2-yl)methyl methanesulfonate | (5-((2,6-dioxopiperidin-3-yl)amino)-3-fluoropyridin-2-yl)methyl methanesulfonate |
| | 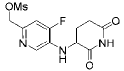 | (5-((2,6-dioxopiperidin-3-yl)amino)-4-fluoropyridin-2-yl)methyl methanesulfonate | (5-((2,6-dioxopiperidin-3-yl)amino)-4-fluoropyridin-2-yl)methyl methanesulfonate |
| | 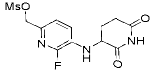 | (5-((2,6-dioxopiperidin-3-yl)amino)-6-fluoropyridin-2-yl)methyl methanesulfonate | (5-((2,6-dioxopiperidin-3-yl)amino)-6-fluoropyridin-2-yl)methyl methanesulfonate |
| | 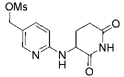 | (6-((2,6-dioxopiperidin-3-yl)amino)pyridin-3-yl)methyl methanesulfonate | (6-((2,6-dioxopiperidin-3-yl)amino)pyridin-3-yl)methyl methanesulfonate |
| | 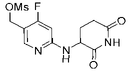 | (6-((2,6-dioxopiperidin-3-yl)amino)-4-fluoropyridin-3-yl)methyl methanesulfonate | (6-((2,6-dioxopiperidin-3-yl)amino)-4-fluoropyridin-3-yl)methyl methanesulfonate |
| | 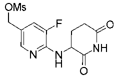 | (6-((2,6-dioxopiperidin-3-yl)amino)-5-fluoropyridin-3-yl)methyl methanesulfonate | (6-((2,6-dioxopiperidin-3-yl)amino)-5-fluoropyridin-3-yl)methyl methanesulfonate |
| | 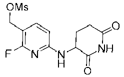 | (6-((2,6-dioxopiperidin-3-yl)amino)-2-fluoropyridin-3-yl)methyl methanesulfonate | (6-((2,6-dioxopiperidin-3-yl)amino)-2-fluoropyridin-3-yl)methyl methanesulfonate |
| | 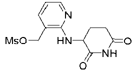 | (2-((2,6-dioxopiperidin-3-yl)amino)pyridin-3-yl)methyl methanesulfonate | (2-((2,6-dioxopiperidin-3-yl)amino)pyridin-3-yl)methyl methanesulfonate |
| | 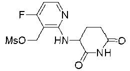 | (2-((2,6-dioxopiperidin-3-yl)amino)-4-fluoropyridin-3-yl)methyl methanesulfonate | (2-((2,6-dioxopiperidin-3-yl)amino)-4-fluoropyridin-3-yl)methyl methanesulfonate |
| | 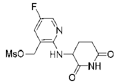 | (2-((2,6-dioxopiperidin-3-yl)amino)-5-fluoropyridin-3-yl)methyl methanesulfonate | (2-((2,6-dioxopiperidin-3-yl)amino)-5-fluoropyridin-3-yl)methyl methanesulfonate |
| | 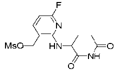 | (2-((2,6-dioxopiperidin-3-yl)amino)-6-fluoropyridin-3-yl)methyl methanesulfonate | (2-((2,6-dioxopiperidin-3-yl)amino)-6-fluoropyridin-3-yl)methyl methanesulfonate |
| | 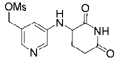 | (5-((2,6-dioxopiperidin-3-yl)amino)pyridin-3-yl)methyl methanesulfonate | (5-((2,6-dioxopiperidin-3-yl)amino)pyridin-3-yl)methyl methanesulfonate |
| | 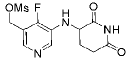 | (5-((2,6-dioxopiperidin-3-yl)amino)-4-fluoropyridin-3-yl)methyl methanesulfonate | (5-((2,6-dioxopiperidin-3-yl)amino)-4-fluoropyridin-3-yl)methyl methanesulfonate |
| | 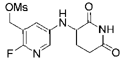 | (5-((2,6-dioxopiperidin-3-yl)amino)-2-fluoropyridin-3-yl)methyl methanesulfonate | (5-((2,6-dioxopiperidin-3-yl)amino)-2-fluoropyridin-3-yl)methyl methanesulfonate |
| | 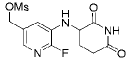 | (5-((2,6-dioxopiperidin-3-yl)amino)-6-fluoropyridin-3-yl)methyl methanesulfonate | (5-((2,6-dioxopiperidin-3-yl)amino)-6-fluoropyridin-3-yl)methyl methanesulfonate |
| | 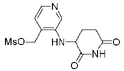 | (3-((2,6-dioxopiperidin-3-yl)amino)pyridin-4-yl)methyl methanesulfonate | (3-((2,6-dioxopiperidin-3-yl)amino)pyridin-4-yl)methyl methanesulfonate |
| | 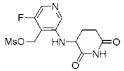 | (3-((2,6-dioxopiperidin-3-yl)amino)-5-fluoropyridin-4-yl)methyl methanesulfonate | (3-((2,6-dioxopiperidin-3-yl)amino)-5-fluoropyridin-4-yl)methyl methanesulfonate |
| | 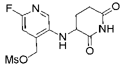 | (5-((2,6-dioxopiperidin-3-yl)amino)-2-fluoropyridin-4-yl)methyl methanesulfonate | (5-((2,6-dioxopiperidin-3-yl)amino)-2-fluoropyridin-4-yl)methyl methanesulfonate |
| | 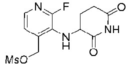 | (3-((2,6-dioxopiperidin-3-yl)amino)-2-fluoropyridin-4-yl)methyl methanesulfonate | (3-((2,6-dioxopiperidin-3-yl)amino)-2-fluoropyridin-4-yl)methyl methanesulfonate |
| | 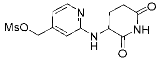 | (2-((2,6-dioxopiperidin-3-yl)amino)pyridin-4-yl)methyl methanesulfonate | (2-((2,6-dioxopiperidin-3-yl)amino)pyridin-4-yl)methyl methanesulfonate |
| | 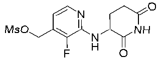 | (2-((2,6-dioxopiperidin-3-yl)amino)-3-fluoropyridin-4-yl)methyl methanesulfonate | (2-((2,6-dioxopiperidin-3-yl)amino)-3-fluoropyridin-4-yl)methyl methanesulfonate |
| | 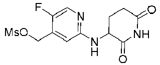 | (2-((2,6-dioxopiperidin-3-yl)amino)-5-fluoropyridin-4-yl)methyl methanesulfonate | (2-((2,6-dioxopiperidin-3-yl)amino)-5-fluoropyridin-4-yl)methyl methanesulfonate |
| | 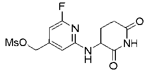 | (2-((2,6-dioxopiperidin-3-yl)amino)-6-fluoropyridin-4-yl)methyl methanesulfonate | (2-((2,6-dioxopiperidin-3-yl)amino)-6-fluoropyridin-4-yl)methyl methanesulfonate |
| | 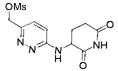 | (6-((2,6-dioxopiperidin-3-yl)amino)pyridazin-3-yl)methyl methanesulfonate | (6-((2,6-dioxopiperidin-3-yl)amino)pyridazin-3-yl)methyl methanesulfonate |
| | 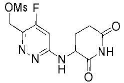 | (6-((2,6-dioxopiperidin-3-yl)amino)-4-fluoropyridazin-3-yl)methyl methanesulfonate | (6-((2,6-dioxopiperidin-3-yl)amino)-4-fluoropyridazin-3-yl)methyl methanesulfonate |
| | 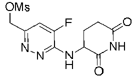 | (6-((2,6-dioxopiperidin-3-yl)amino)-5-fluoropyridazin-3-yl)methyl methanesulfonate | (6-((2,6-dioxopiperidin-3-yl)amino)-5-fluoropyridazin-3-yl)methyl methanesulfonate |
| | 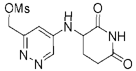 | (5-((2,6-dioxopiperidin-3-yl)amino)pyridazin-3-yl)methyl methanesulfonate | (5-((2,6-dioxopiperidin-3-yl)amino)pyridazin-3-yl)methyl methanesulfonate |
| | 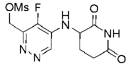 | (5-((2,6-dioxopiperidin-3-yl)amino)-4-fluoropyridazin-3-yl)methyl methanesulfonate | (5-((2,6-dioxopiperidin-3-yl)amino)-4-fluoropyridazin-3-yl)methyl methanesulfonate |
| | 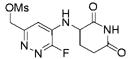 | (5-((2,6-dioxopiperidin-3-yl)amino)-6-fluoropyridazin-3-yl)methyl methanesulfonate | (5-((2,6-dioxopiperidin-3-yl)amino)-6-fluoropyridazin-3-yl)methyl methanesulfonate |
| | 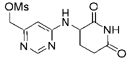 | (6-((2,6-dioxopiperidin-3-yl)amino)pyrimidin-4-yl)methyl methanesulfonate | (6-((2,6-dioxopiperidin-3-yl)amino)pyrimidin-4-yl)methyl methanesulfonate |
| | 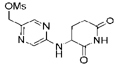 | (5-((2,6-dioxopiperidin-3-yl)amino)pyrazin-2-yl)methyl methanesulfonate | (5-((2,6-dioxopiperidin-3-yl)amino)pyrazin-2-yl)methyl methanesulfonate |
| | 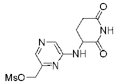 | (6-((2,6-dioxopiperidin-3-yl)amino)pyrazin-2-yl)methyl methanesulfonate | (6-((2,6-dioxopiperidin-3-yl)amino)pyrazin-2-yl)methyl methanesulfonate |
| | 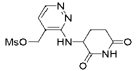 | (3-((2,6-dioxopiperidin-3-yl)amino)pyridazin-4-yl)methyl methanesulfonate | (3-((2,6-dioxopiperidin-3-yl)amino)pyridazin-4-yl)methyl methanesulfonate |
| | 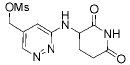 | (6-((2,6-dioxopiperidin-3-yl)amino)pyridazin-4-yl)methyl methanesulfonate | (6-((2,6-dioxopiperidin-3-yl)amino)pyridazin-4-yl)methyl methanesulfonate |
| | 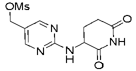 | (2-((2,6-dioxopiperidin-3-yl)amino)pyrimidin-5-yl)methyl methanesulfonate | (2-((2,6-dioxopiperidin-3-yl)amino)pyrimidin-5-yl)methyl methanesulfonate |
| | 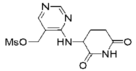 | (4-((2,6-dioxopiperidin-3-yl)amino)pyrimidin-5-yl)methyl methanesulfonate | (4-((2,6-dioxopiperidin-3-yl)amino)pyrimidin-5-yl)methyl methanesulfonate |
| | 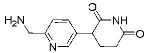 | 3-(6-(aminomethyl)pyridin-3-yl)piperidine-2,6-dione | 3-(6-(aminomethyl)pyridin-3-yl)piperidine-2,6-dione |
| | 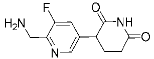 | 3-(6-(aminomethyl)-5-fluoropyridin-3-yl)piperidine-2,6-dione | 3-(6-(aminomethyl)-5-fluoropyridin-3-yl)piperidine-2,6-dione |
| | 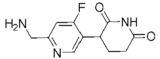 | 3-(6-(aminomethyl)-4-fluoropyridin-3-yl)piperidine-2,6-dione | 3-(6-(aminomethyl)-4-fluoropyridin-3-yl)piperidine-2,6-dione |
| | 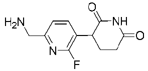 | 3-(6-(aminomethyl)-2-fluoropyridin-3-yl)piperidine-2,6-dione | 3-(6-(aminomethyl)-2-fluoropyridin-3-yl)piperidine-2,6-dione |
| | 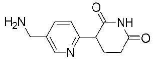 | 3-(5-(aminomethyl)pyridin-2-yl)piperidine-2,6-dione | 3-(5-(aminomethyl)pyridin-2-yl)piperidine-2,6-dione |
| | 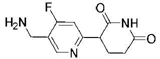 | 3-(5-(aminomethyl)-4-fluoropyridin-2-yl)piperidine-2,6-dione | 3-(5-(aminomethyl)-4-fluoropyridin-2-yl)piperidine-2,6-dione |
| | 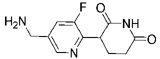 | 3-(5-(aminomethyl)-3-fluoropyridin-2-yl)piperidine-2,6-dione | 3-(5-(aminomethyl)-3-fluoropyridin-2-yl)piperidine-2,6-dione |
| | 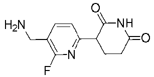 | 3-(5-(aminomethyl)-6-fluoropyridin-2-yl)piperidine-2,6-dione | 3-(5-(aminomethyl)-6-fluoropyridin-2-yl)piperidine-2,6-dione |
| | 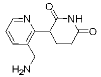 | 3-(3-(aminomethyl)pyridin-2-yl)piperidine-2,6-dione | 3-(3-(aminomethyl)pyridin-2-yl)piperidine-2,6-dione |
| | 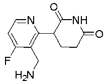 | 3-(3-(aminomethyl)-4-fluoropyridin-2-yl)piperidine-2,6-dione | 3-(3-(aminomethyl)-4-fluoropyridin-2-yl)piperidine-2,6-dione |
| | 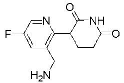 | 3-(3-(aminomethyl)-5-fluoropyridin-2-yl)piperidine-2,6-dione | 3-(3-(aminomethyl)-5-fluoropyridin-2-yl)piperidine-2,6-dione |
| | 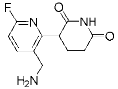 | 3-(3-(aminomethyl)-6-fluoropyridin-2-yl)piperidine-2,6-dione | 3-(3-(aminomethyl)-6-fluoropyridin-2-yl)piperidine-2,6-dione |
| GT-06990 | 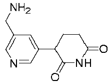 | 3-(5-(aminomethyl)pyridin-3-yl)piperidine-2,6-dione | 3-(5-(aminomethyl)pyridin-3-yl)piperidine-2,6-dione |
| | 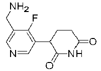 | 3-(5-(aminomethyl)-4-fluoropyridin-3-yl)piperidine-2,6-dione | 3-(5-(aminomethyl)-4-fluoropyridin-3-yl)piperidine-2,6-dione |
| | 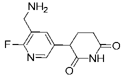 | 3-(5-(aminomethyl)-6-fluoropyridin-3-yl)piperidine-2,6-dione | 3-(5-(aminomethyl)-6-fluoropyridin-3-yl)piperidine-2,6-dione |
| | 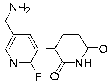 | 3-(5-(aminomethyl)-2-fluoropyridin-3-yl)piperidine-2,6-dione | 3-(5-(aminomethyl)-2-fluoropyridin-3-yl)piperidine-2,6-dione |
| | 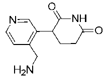 | 3-(4-(aminomethyl)pyridin-3-yl)piperidine-2,6-dione | 3-(4-(aminomethyl)pyridin-3-yl)piperidine-2,6-dione |
| | 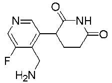 | 3-(4-(aminomethyl)-5-fluoropyridin-3-yl)piperidine-2,6-dione | 3-(4-(aminomethyl)-5-fluoropyridin-3-yl)piperidine-2,6-dione |
| | 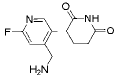 | 3-(4-(aminomethyl)-6-fluoropyridin-3-yl)piperidine-2,6-dione | 3-(4-(aminomethyl)-6-fluoropyridin-3-yl)piperidine-2,6-dione |
| | 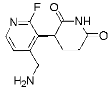 | 3-(4-(aminomethyl)-2-fluoropyridin-3-yl)piperidine-2,6-dione | 3-(4-(aminomethyl)-2-fluoropyridin-3-yl)piperidine-2,6-dione |
| | 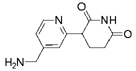 | 3-(4-(aminomethyl)pyridin-2-yl)piperidine-2,6-dione | 3-(4-(aminomethyl)pyridin-2-yl)piperidine-2,6-dione |
| | 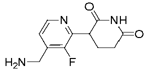 | 3-(4-(aminomethyl)-3-fluoropyridin-2-yl)piperidine-2,6-dione | 3-(4-(aminomethyl)-3-fluoropyridin-2-yl)piperidine-2,6-dione |
| | 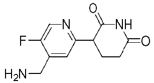 | 3-(4-(aminomethyl)-5-fluoropyridin-2-yl)piperidine-2,6-dione | 3-(4-(aminomethyl)-5-fluoropyridin-2-yl)piperidine-2,6-dione |
| | 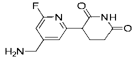 | 3-(4-(aminomethyl)-6-fluoropyridin-2-yl)piperidine-2,6-dione | 3-(4-(aminomethyl)-6-fluoropyridin-2-yl)piperidine-2,6-dione |
| | 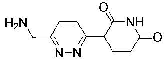 | 3-(6-(aminomethyl)pyridazin-3-yl)piperidine-2,6-dione | 3-(6-(aminomethyl)pyridazin-3-yl)piperidine-2,6-dione |
| | 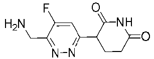 | 3-(6-(aminomethyl)-5-fluoropyridazin-3-yl)piperidine-2,6-dione | 3-(6-(aminomethyl)-5-fluoropyridazin-3-yl)piperidine-2,6-dione |
| | 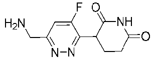 | 3-(6-(aminomethyl)-4-fluoropyridazin-3-yl)piperidine-2,6-dione | 3-(6-(aminomethyl)-4-fluoropyridazin-3-yl)piperidine-2,6-dione |
| | 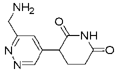 | 3-(6-(aminomethyl)pyridazin-4-yl)piperidine-2,6-dione | 3-(6-(aminomethyl)pyridazin-4-yl)piperidine-2,6-dione |
| | 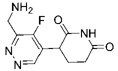 | 3-(6-(aminomethyl)-5-fluoropyridazin-4-yl)piperidine-2,6-dione | 3-(6-(aminomethyl)-5-fluoropyridazin-4-yl)piperidine-2,6-dione |
| | 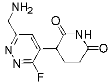 | 3-(6-(aminomethyl)-3-fluoropyridazin-4-yl)piperidine-2,6-dione | 3-(6-(aminomethyl)-3-fluoropyridazin-4-yl)piperidine-2,6-dione |
| | 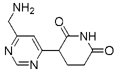 | 3-(6-(aminomethyl)pyrimidin-4-yl)piperidine-2,6-dione | 3-(6-(aminomethyl)pyrimidin-4-yl)piperidine-2,6-dione |
| | 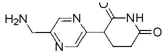 | 3-(5-(aminomethyl)pyrazin-2-yl)piperidine-2,6-dione | 3-(5-(aminomethyl)pyrazin-2-yl)piperidine-2,6-dione |
| | 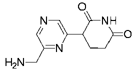 | 3-(6-(aminomethyl)pyrazin-2-yl)piperidine-2,6-dione | 3-(6-(aminomethyl)pyrazin-2-yl)piperidine-2,6-dione |
| | 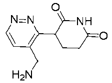 | 3-(4-(aminomethyl)pyridazin-3-yl)piperidine-2,6-dione | 3-(4-(aminomethyl)pyridazin-3-yl)piperidine-2,6-dione |
| | 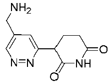 | 3-(5-(aminomethyl)pyridazin-3-yl)piperidine-2,6-dione | 3-(5-(aminomethyl)pyridazin-3-yl)piperidine-2,6-dione |
| | 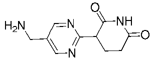 | 3-(5-(aminomethyl)pyrimidin-2-yl)piperidine-2,6-dione | 3-(5-(aminomethyl)pyrimidin-2-yl)piperidine-2,6-dione |
| | 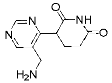 | 3-(5-(aminomethyl)pyrimidin-4-yl)piperidine-2,6-dione | 3-(5-(aminomethyl)pyrimidin-4-yl)piperidine-2,6-dione |
| GT-07048 | 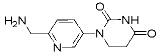 | 1-(6-(aminomethyl)pyridin-3-yl)dihydropyrimidine-2,4(1H,3H)-dione | 1-(6-(aminomethyl)pyridin-3-yl)dihydropyrimidine-2,4(1H,3H)-dione |
| | 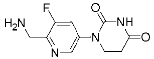 | 1-(6-(aminomethyl)-5-fluoropyridin-3-yl)dihydropyrimidine-2,4(1H,3H)-dione | 1-(6-(aminomethyl)-5-fluoropyridin-3-yl)dihydropyrimidine-2,4(1H,3H)-dione |
| | 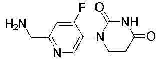 | 1-(6-(aminomethyl)-4-fluoropyridin-3-yl)dihydropyrimidine-2,4(1H,3H)-dione | 1-(6-(aminomethyl)-4-fluoropyridin-3-yl)dihydropyrimidine-2,4(1H,3H)-dione |
| | 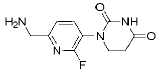 | 1-(6-(aminomethyl)-2-fluoropyridin-3-yl)dihydropyrimidine-2,4(1H,3H)-dione | 1-(6-(aminomethyl)-2-fluoropyridin-3-yl)dihydropyrimidine-2,4(1H,3H)-dione |
| GT-07012 | 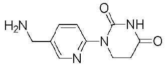 | 1-(5-(aminomethyl)pyridin-2-yl)dihydropyrimidine-2,4(1H,3H)-dione | 1-(5-(aminomethyl)pyridin-2-yl)dihydropyrimidine-2,4(1H,3H)-dione |
| | 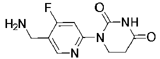 | 1-(5-(aminomethyl)-4-fluoropyridin-2-yl)dihydropyrimidine-2,4(1H,3H)-dione | 1-(5-(aminomethyl)-4-fluoropyridin-2-yl)dihydropyrimidine-2,4(1H,3H)-dione |
| | 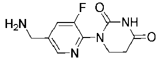 | 1-(5-(aminomethyl)-3-fluoropyridin-2-yl)dihydropyrimidine-2,4(1H,3H)-dione | 1-(5-(aminomethyl)-3-fluoropyridin-2-yl)dihydropyrimidine-2,4(1H,3H)-dione |
| | 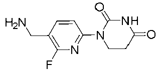 | 1-(5-(aminomethyl)-6-fluoropyridin-2-yl)dihydropyrimidine-2,4(1H,3H)-dione | 1-(5-(aminomethyl)-6-fluoropyridin-2-yl)dihydropyrimidine-2,4(1H,3H)-dione |
| | 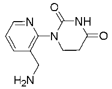 | 1-(3-(aminomethyl)pyridin-2-yl)dihydropyrimidine-2,4(lH,3H)-dione | 1-(3-(aminomethyl)pyridin-2-yl)dihydropyrimidine-2,4(1H,3H)-dione |
| | 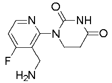 | 1-(3-(aminomethyl)-4-fluoropyridin-2-yl)dihydropyrimidine-2,4(1H,3H)-dione | 1-(3-(aminomethyl)-4-fluoropyridin-2-yl)dihydropyrimidine-2,4(1H,3H)-dione |
| | 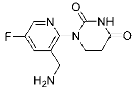 | 1-(3-(aminomethyl)-5-fluoropyridin-2-yl)dihydropyrimidine-2,4(1H,3H)-dione | 1-(3-(aminomethyl)-5-fluoropyridin-2-yl)dihydropyrimidine-2,4(1H,3H)-dione |
| | 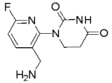 | 1-(3-(aminomethyl)-6-fluoropyridin-2-yl)dihydropyrimidine-2,4(1H,3H)-dione | 1-(3-(aminomethyl)-6-fluoropyridin-2-yl)dihydropyrimidine-2,4(1H,3H)-dione |
| GT-07077 | 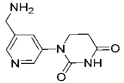 | 1-(5-(aminomethyl)pyridin-3-yl)dihydropyrimidine-2,4(lH,3H)-dione | 1-(5-(aminomethyl)pyridin-3-yl)dihydropyrimidine-2,4(1H,3H)-dione |
| | 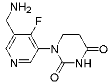 | 1-(5-(aminomethyl)-4-fluoropyridin-3-yl)dihydropyrimidine-2,4(1H,3H)-dione | 1-(5-(aminomethyl)-4-fluoropyridin-3-yl)dihydropyrimidine-2,4(1H,3H)-dione |
| GT-07729 | 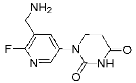 | 1-(5-(aminomethyl)-6-fluoropyridin-3-yl)dihydropyrimidine-2,4(1H,3H)-dione | 1-(5-(aminomethyl)-6-fluoropyridin-3-yl)dihydropyrimidine-2,4(1H,3H)-dione |
| | 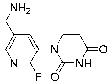 | 1-(5-(aminomethyl)-2-fluoropyridin-3-yl)dihydropyrimidine-2,4(1H,3H)-dione | 1-(5-(aminomethyl)-2-fluoropyridin-3-yl)dihydropyrimidine-2,4(1H,3H)-dione |
| | 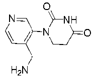 | 1-(4-(aminomethyl)pyridin-3-yl)dihydropyrimidine-2,4(1H,3H)-dione | 1-(4-(aminomethyl)pyridin-3-yl)dihydropyrimidine-2,4(1H,3H)-dione |
| | 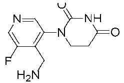 | 1-(4-(aminomethyl)-5-fluoropyridin-3-yl)dihydropyrimidine-2,4(1H,3H)-dione | 1-(4-(aminomethyl)-5-fluoropyridin-3-yl)dihydropyrimidine-2,4(1H,3H)-dione |
| | 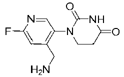 | 1-(4-(aminomethyl)-6-fluoropyridin-3-yl)dihydropyrimidine-2,4(1H,3H)-dione | 1-(4-(aminomethyl)-6-fluoropyridin-3-yl)dihydropyrimidine-2,4(1H,3H)-dione |
| | 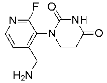 | 1-(4-(aminomethyl)-2-fluoropyridin-3-yl)dihydropyrimidine-2,4(1H,3H)-dione | 1-(4-(aminomethyl)-2-fluoropyridin-3-yl)dihydropyrimidine-2,4(1H,3H)-dione |
| GT-07198 | 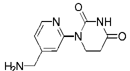 | 1-(4-(aminomethyl)pyridin-2-yl)dihydropyrimidine-2,4(1H,3H)-dione | 1-(4-(aminomethyl)pyridin-2-yl)dihydropyrimidine-2,4(1H,3H)-dione |
| | 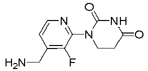 | 1-(4-(aminomethyl)-3-fluoropyridin-2-yl)dihydropyrimidine-2,4(1H,3H)-dione | 1-(4-(aminomethyl)-3-fluoropyridin-2-yl)dihydropyrimidine-2,4(1H,3H)-dione |
| GT-08126 | 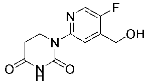 | 1-(5-fluoro-4-(hydroxymethyl)pyridin-2-yl)dihydropyrimidine-2,4(1H,3H)-dione | 1-(5-fluoro-4-(hydroxymethyl)pyridin-2-yl)dihydropyrimidine-2,4(1H,3H)-dione |
| GT-07369 | 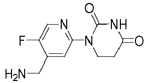 | 1-(4-(aminomethyl)-5-fluoropyridin-2-yl)dihydropyrimidine-2,4(1H,3H)-dione | 1-(4-(aminomethyl)-5-fluoropyridin-2-yl)dihydropyrimidine-2,4(1H,3H)-dione |
| | 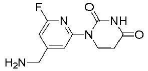 | 1-(4-(aminomethyl)-6-fluoropyridin-2-yl)dihydropyrimidine-2,4(1H,3H)-dione | 1-(4-(aminomethyl)-6-fluoropyridin-2-yl)dihydropyrimidine-2,4(1H,3H)-dione |
| | 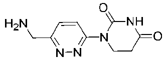 | 1-(6-(aminomethyl)pyridazin-3-yl)dihydropyrimidine-2,4(1H,3H)-dione | 1-(6-(aminomethyl)pyridazin-3-yl)dihydropyrimidine-2,4(1H,3H)-dione |
| | 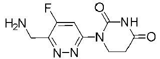 | 1-(6-(aminomethyl)-5-fluoropyridazin-3-yl)dihydropyrimidine-2,4(1H,3H)-dione | 1-(6-(aminomethyl)-5-fluoropyridazin-3-yl)dihydropyrimidine-2,4(1H,3H)-dione |
| | 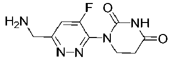 | 1-(6-(aminomethyl)-4-fluoropyridazin-3-yl)dihydropyrimidine-2,4(1H,3H)-dione | 1-(6-(aminomethyl)-4-fluoropyridazin-3-yl)dihydropyrimidine-2,4(1H,3H)-dione |
| | 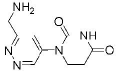 | 1-(6-(aminomethyl)pyridazin-4-yl)dihydropyrimidine-2,4(1H,3H)-dione | 1-(6-(aminomethyl)pyridazin-4-yl)dihydropyrimidine-2,4(1H,3H)-dione |
| | 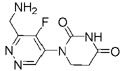 | 1-(6-(aminomethyl)-5-fluoropyridazin-4-yl)dihydropyrimidine-2,4(1H,3H)-dione | 1-(6-(aminomethyl)-5-fluoropyridazin-4-yl)dihydropyrimidine-2,4(1H,3H)-dione |
| | 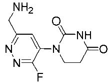 | 1-(6-(aminomethyl)-3-fluoropyridazin-4-yl)dihydropyrimidine-2,4(1H,3H)-dione | 1-(6-(aminomethyl)-3-fluoropyridazin-4-yl)dihydropyrimidine-2,4(1H,3H)-dione |
| | 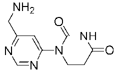 | 1-(6-(aminomethyl)pyrimidin-4-yl)dihydropyrimidine-2,4(1H,3H)-dione | 1-(6-(aminomethyl)pyrimidin-4-yl)dihydropyrimidine-2,4(1H,3H)-dione |
| | 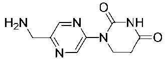 | 1-(5-(aminomethyl)pyrazin-2-yl)dihydropyrimidine-2,4(1H,3H)-dione | 1-(5-(aminomethyl)pyrazin-2-yl)dihydropyrimidine-2,4(1H,3H)-dione |
| GT-07801 | 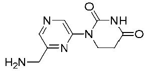 | 1-(6-(aminomethyl)pyrazin-2-yl)dihydropyrimidine-2,4(1H,3H)-dione | 1-(6-(aminomethyl)pyrazin-2-yl)dihydropyrimidine-2,4(1H,3H)-dione |
| | 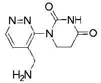 | 1-(4-(aminomethyl)pyridazin-3-yl)dihydropyrimidine-2,4(1H,3H)-dione | 1-(4-(aminomethyl)pyridazin-3-yl)dihydropyrimidine-2,4(1H,3H)-dione |
| | 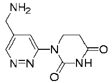 | 1-(5-(aminomethyl)pyridazin-3-yl)dihydropyrimidine-2,4(1H,3H)-dione | 1-(5-(aminomethyl)pyridazin-3-yl)dihydropyrimidine-2,4(1H,3H)-dione |
| | 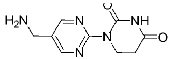 | 1-(5-(aminomethyl)pyrimidin-2-yl)dihydropyrimidine-2,4(1H,3H)-dione | 1-(5-(aminomethyl)pyrimidin-2-yl)dihydropyrimidine-2,4(1H,3H)-dione |
| | 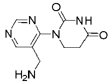 | 1-(5-(aminomethyl)pyrimidin-4-yl)dihydropyrimidine-2,4(1H,3H)-dione | 1-(5-(aminomethyl)pyrimidin-4-yl)dihydropyrimidine-2,4(1H,3H)-dione |
| | 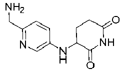 | 3-((6-(aminomethyl)pyridin-3-yl)amino)piperidine-2,6-dione | 3-((6-(aminomethyl)pyridin-3-yl)amino)piperidine-2,6-dione |
| | 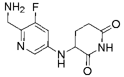 | 3-((6-(aminomethyl)-5-fluoropyridin-3-yl)amino)piperidine-2,6-dione | 3-((6-(aminomethyl)-5-fluoropyridin-3-yl)amino)piperidine-2,6-dione |
| | 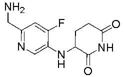 | 3-((6-(aminomethyl)-4-fluoropyridin-3-yl)amino)piperidine-2,6-dione | 3-((6-(aminomethyl)-4-fluoropyridin-3-yl)amino)piperidine-2,6-dione |
| | 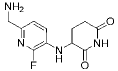 | 3-((6-(aminomethyl)-2-fluoropyridin-3-yl)amino)piperidine-2,6-dione | 3-((6-(aminomethyl)-2-fluoropyridin-3-yl)amino)piperidine-2,6-dione |
| | 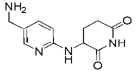 | 3-((5-(aminomethyl)pyridin-2-yl)amino)piperidine-2,6-dione | 3-((5-(aminomethyl)pyridin-2-yl)amino)piperidine-2,6-dione |
| | 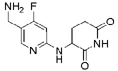 | 3-((5-(aminomethyl)-4-fluoropyridin-2-yl)amino)piperidine-2,6-dione | 3-((5-(aminomethyl)-4-fluoropyridin-2-yl)amino)piperidine-2,6-dione |
| | 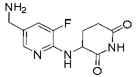 | 3-((5-(aminomethyl)-3-fluoropyridin-2-yl)amino)piperidine-2,6-dione | 3-((5-(aminomethyl)-3-fluoropyridin-2-yl)amino)piperidine-2,6-dione |
| | 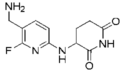 | 3-((5-(aminomethyl)-6-fluoropyridin-2-yl)amino)piperidine-2,6-dione | 3-((5-(aminomethyl)-6-fluoropyridin-2-yl)amino)piperidine-2,6-dione |
| | 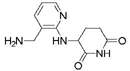 | 3-((3-(aminomethyl)pyridin-2-yl)amino)piperidine-2,6-dione | 3-((3-(aminomethyl)pyridin-2-yl)amino)piperidine-2,6-dione |
| | 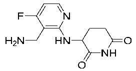 | 3-((3-(aminomethyl)-4-fluoropyridin-2-yl)amino)piperidine-2,6-dione | 3-((3-(aminomethyl)-4-fluoropyridin-2-yl)amino)piperidine-2,6-dione |
| | 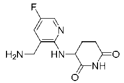 | 3-((3-(aminomethyl)-5-fluoropyridin-2-yl)amino)piperidine-2,6-dione | 3-((3-(aminomethyl)-5-fluoropyridin-2-yl)amino)piperidine-2,6-dione |
| | 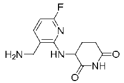 | 3-((3-(aminomethyl)-6-fluoropyridin-2-yl)amino)piperidine-2,6-dione | 3-((3-(aminomethyl)-6-fluoropyridin-2-yl)amino)piperidine-2,6-dione |
| | 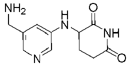 | 3-((5-(aminomethyl)pyridin-3-yl)amino)piperidine-2,6-dione | 3-((5-(aminomethyl)pyridin-3-yl)amino)piperidine-2,6-dione |
| | 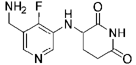 | 3-((5-(aminomethyl)-4-fluoropyridin-3-yl)amino)piperidine-2,6-dione | 3-((5-(aminomethyl)-4-fluoropyridin-3-yl)amino)piperidine-2,6-dione |
| | 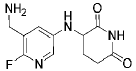 | 3-((5-(aminomethyl)-6-fluoropyridin-3-yl)amino)piperidine-2,6-dione | 3-((5-(aminomethyl)-6-fluoropyridin-3-yl)amino)piperidine-2,6-dione |
| | 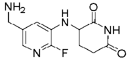 | 3-((5-(aminomethyl)-2-fluoropyridin-3-yl)amino)piperidine-2,6-dione | 3-((5-(aminomethyl)-2-fluoropyridin-3-yl)amino)piperidine-2,6-dione |
| | 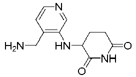 | 3-((4-(aminomethyl)pyridin-3-yl)amino)piperidine-2,6-dione | 3-((4-(aminomethyl)pyridin-3-yl)amino)piperidine-2,6-dione |
| | 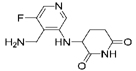 | 3-((4-(aminomethyl)-5-fluoropyridin-3-yl)amino)piperidine-2,6-dione | 3-((4-(aminomethyl)-5-fluoropyridin-3-yl)amino)piperidine-2,6-dione |
| | 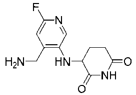 | 3-((4-(aminomethyl)-6-fluoropyridin-3-yl)amino)piperidine-2,6-dione | 3-((4-(aminomethyl)-6-fluoropyridin-3-yl)amino)piperidine-2,6-dione |
| | 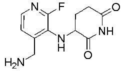 | 3-((4-(aminomethyl)-2-fluoropyridin-3-yl)amino)piperidine-2,6-dione | 3-((4-(aminomethyl)-2-fluoropyridin-3-yl)amino)piperidine-2,6-dione |
| | 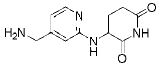 | 3-((4-(aminomethyl)pyridin-2-yl)amino)piperidine-2,6-dione | 3-((4-(aminomethyl)pyridin-2-yl)amino)piperidine-2,6-dione |
| | 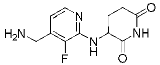 | 3-((4-(aminomethyl)-3-fluoropyridin-2-yl)amino)piperidine-2,6-dione | 3-((4-(aminomethyl)-3-fluoropyridin-2-yl)amino)piperidine-2,6-dione |
| | 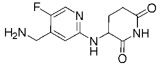 | 3-((4-(aminomethyl)-5-fluoropyridin-2-yl)amino)piperidine-2,6-dione | 3-((4-(aminomethyl)-5-fluoropyridin-2-yl)amino)piperidine-2,6-dione |
| | 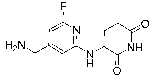 | 3-((4-(aminomethyl)-6-fluoropyridin-2-yl)amino)piperidine-2,6-dione | 3-((4-(aminomethyl)-6-fluoropyridin-2-yl)amino)piperidine-2,6-dione |
| | 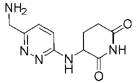 | 3-((6-(aminomethyl)pyridazin-3-yl)amino)piperidine-2,6-dione | 3-((6-(aminomethyl)pyridazin-3-yl)amino)piperidine-2,6-dione |
| | 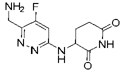 | 3-((6-(aminomethyl)-5-fluoropyridazin-3-yl)amino)piperidine-2,6-dione | 3-((6-(aminomethyl)-5-fluoropyridazin-3-yl)amino)piperidine-2,6-dione |
| | 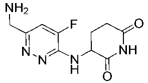 | 3-((6-(aminomethyl)-4-fluoropyridazin-3-yl)amino)piperidine-2,6-dione | 3-((6-(aminomethyl)-4-fluoropyridazin-3-yl)amino)piperidine-2,6-dione |
| | 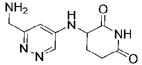 | 3-((6-(aminomethyl)pyridazin-4-yl)amino)piperidine-2,6-dione | 3-((6-(aminomethyl)pyridazin-4-yl)amino)piperidine-2,6-dione |
| | 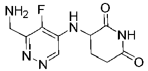 | 3-((6-(aminomethyl)-5-fluoropyridazin-4-yl)amino)piperidine-2,6-dione | 3-((6-(aminomethyl)-5-fluoropyridazin-4-yl)amino)piperidine-2,6-dione |
| | 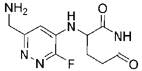 | 3-((6-(aminomethyl)-3-fluoropyridazin-4-yl)amino)piperidine-2,6-dione | 3-((6-(aminomethyl)-3-fluoropyridazin-4-yl)amino)piperidine-2,6-dione |
| | 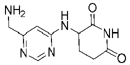 | 3-((6-(aminomethyl)pyrimidin-4-yl)amino)piperidine-2,6-dione | 3-((6-(aminomethyl)pyrimidin-4-yl)amino)piperidine-2,6-dione |
| | 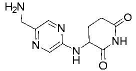 | 3-((5-(aminomethyl)pyrazin-2-yl)amino)piperidine-2,6-dione | 3-((5-(aminomethyl)pyrazin-2-yl)amino)piperidine-2,6-dione |
| | 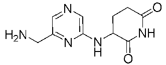 | 3-((6-(aminomethyl)pyrazin-2-yl)amino)piperidine-2,6-dione | 3-((6-(aminomethyl)pyrazin-2-yl)amino)piperidine-2,6-dione |
| | 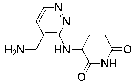 | 3-((4-(aminomethyl)pyridazin-3-yl)amino)piperidine-2,6-dione | 3-((4-(aminomethyl)pyridazin-3-yl)amino)piperidine-2,6-dione |
| | 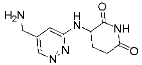 | 3-((5-(aminomethyl)pyridazin-3-yl)amino)piperidine-2,6-dione | 3-((5-(aminomethyl)pyridazin-3-yl)amino)piperidine-2,6-dione |
| | 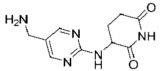 | 3-((5-(aminomethyl)pyrimidin-2-yl)amino)piperidine-2,6-dione | 3-((5-(aminomethyl)pyrimidin-2-yl)amino)piperidine-2,6-dione |
| | 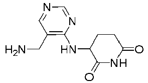 | 3-((5-(aminomethyl)pyrimidin-4-yl)amino)piperidine-2,6-dione | 3-((5-(aminomethyl)pyrimidin-4-yl)amino)piperidine-2,6-dione |
| 835253 | 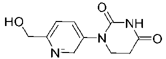 | 1-(6-(hydroxymethyl)pyridin-3-yl)dihydropyrimidine-2,4(1H,3H)-dione | 1-(6-(hydroxymethyl)pyridin-3-yl)dihydropyrimidine-2,4(1H,3H)-dione |
| GT-08011 | 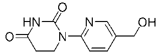 | 1-(5-(hydroxymethyl)pyridin-2-yl)dihydropyrimidine-2,4(1H,3H)-dione | 1-(5-(hydroxymethyl)pyridin-2-yl)dihydropyrimidine-2,4(1H,3H)-dione |
| 835336 | 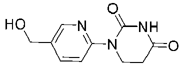 | 1-(5-(hydroxymethyl)pyridin-2-yl)dihydropyrimidine-2,4(1H,3H)-dione | 1-(5-(hydroxymethyl)pyridin-2-yl)dihydropyrimidine-2,4(1H,3H)-dione |

### II. Other Forms of Compounds (including salts, enantiomers, stereoisomers, solvates, isotopically enriched analogs, prodrugs, or polymorphs of compounds)

The compounds of the present disclosure have the structures of any one of Formula (I), Formula (II), Formula (I-1), Formula (I-2), Formula (I-3), Formula (I-4), Formula (I-5), Formula (I-6), Formula (I-7), Formula (I-8-1), Formula (I-8-2), Formula (I-8-3), Formula (I-8-4), Formula (I-9), Formula (I-10), Formula (I-11), Formula (I-12), Formula (I-13), Formula (I-14), Formula (I-15), Formula (I-16), Formula (I-17), Formula (I-18), Formula (I-19), Formula (I-20-1), Formula (I-20-2), Formula (I-20-3), Formula (I-20-4), Formula (I-21-1), Formula (I-21-2), Formula (I-22), Formula (I-23), Formula (I-24), Formula (I-25-1), Formula (I-25-2), Formula (I-26), Formula (I-27), Formula (I-28), Formula (I-29), Formula (I-30), Formula (I-31), Formula (I-32), Formula (I-33), Formula (I-34-1), Formula (I-34-2), Formula (I-35), Formula (I-36), Formula (I-37), Formula (I-38), Formula (I-39-1), Formula (I-39-2), Formula (I-40), Formula (I-41), Formula (I-42), Formula (I-43), Formula (I-44), Formula (I-45), Formula (I-46), Formula (I-47), Formula (I-48), Formula (I-49), Formula (I-50), Formula (I-51), Formula (I-52), Formula (I-53), Formula (I-54), or Formula (I-55). Unless otherwise specified, all references to the compounds of the present disclosure also include compounds of any one of Formula (I), Formula (II), Formula (I-1), Formula (I-2), Formula (I-3), Formula (I-4), Formula (I-5), Formula (I-6), Formula (I-7), Formula (I-8-1), Formula (I-8-2), Formula (I-8-3), Formula (I-8-4), Formula (I-9), Formula (I-10), Formula (I-11), Formula (I-12), Formula (I-13), Formula (I-14), Formula (I-15), Formula (I-16), Formula (I-17), Formula (I-18), Formula (I-19), Formula (I-20-1), Formula (I-20-2), Formula (I-20-3), Formula (I-20-4), Formula (I-21-1), Formula (I-21-2), Formula (I-22), Formula (I-23), Formula (I-24), Formula (I-25-1), Formula (I-25-2), Formula (I-26), Formula (I-27), Formula (I-28), Formula (I-29), Formula (I-30), Formula (I-31), Formula (I-32), Formula (I-33), Formula (I-34-1), Formula (I-34-2), Formula (I-35), Formula (I-36), Formula (I-37), Formula (I-38), Formula (I-39-1), Formula (I-39-2), Formula (I-40), Formula (I-41), Formula (I-42), Formula (I-43), Formula (I-44), Formula (I-45), Formula (I-46), Formula (I-47), Formula (I-48), Formula (I-49), Formula (I-50), Formula (I-51), Formula (I-52), Formula (I-53), Formula (I-54), or Formula (I-55) and specific compounds within the scope of these general formulae.

It should be recognized that compounds of the present disclosure (including compounds of Formula (I), Formula (II), Formula (I-1), Formula (I-2), Formula (I-3), Formula (I-4), Formula (I-5), Formula (I-6), Formula (I-7), Formula (I-8-1), Formula (I-8-2), Formula (I-8-3), Formula (I-8-4), Formula (I-9), Formula (I-10), Formula (I-11), Formula (I-12), Formula (I-13), Formula (I-14), Formula (I-15), Formula (I-16), Formula (I-17), Formula (I-18), Formula (I-19), Formula (I-20-1), Formula (I-20-2), Formula (I-20-3), Formula (I-20-4), Formula (I-21-1), Formula (I-21-2), Formula (I-22), Formula (I-23), Formula (I-24), Formula (I-25-1), Formula (I-25-2), Formula (I-26), Formula (I-27), Formula (I-28), Formula (I-29), Formula (I-30), Formula (I-31), Formula (I-32), Formula (I-33), Formula (I-34-1), Formula (I-34-2), Formula (I-35), Formula (I-36), Formula (I-37), Formula (I-38), Formula (I-39-1), Formula (I-39-2), Formula (I-40), Formula (I-41), Formula (I-42), Formula (I-43), Formula (I-44), Formula (I-45), Formula (I-46), Formula (I-47), Formula (I-48), Formula (I-49), Formula (I-50), Formula (I-51), Formula (I-52), Formula (I-53), Formula (I-54), or Formula (I-55)) may have a stereo-configuration and thus can exist in more than one stereoisomeric form. The present disclosure also relates to optically enriched compounds having a stereo-configuration, e.g., greater than about 90% enantiomeric/diastereomeric excess ("ee"), such as about 95% ee or 97% ee, or greater than 99% ee, and mixtures thereof, including racemic mixtures. As used herein, "optically enriched" means that a mixture of enantiomers consists of a significantly greater proportion of one enantiomer, and can be described by enantiomeric excess (ee%). Purification of isomers and separation of mixtures of isomers may be accomplished by standard techniques known in the art (e.g., column chromatography, preparative TLC, preparative HPLC, asymmetric synthesis (e.g., by using chiral intermediates) and/or or chiral resolution, etc.).

In some embodiments, polymorph forms or salts of the compounds of the present disclosure are also provided. Salts of the compounds of the present disclosure may be pharmaceutically acceptable salts including, but not limited to, hydrohalides (including hydrochlorides and hydrobromates), sulfates, citrates, maleates, methanesulfonates, citrates, lactates, L-tartrates, fumarates, L-malates, phosphates, dihydrophosphates, pyrophosphates, metaphosphates, oxalates, malonates, benzoates, mandelates, succinates, trifluoroacetates, hydroxyacetates, or p-toluenesulfonates, etc. The compounds of the present disclosure may exist as non-solvated or solvated forms in pharmaceutically acceptable solvents such as water, ethanol, and the like. In some embodiments, the compounds of the present disclosure may be prepared as prodrugs or precursor drugs. Prodrugs may be converted into parent drugs in the body to play their role. In some embodiments, isotopically-labeled compounds of the present disclosure are also provided, examples of which include deuterium (D or ²H).

### III. Compositions/Formulations

In some embodiments, the present disclosure provides a pharmaceutical composition comprising as an active ingredient the compounds of the present disclosure or a pharmaceutically acceptable salt, solvate, isotopically enriched analog, polymorph, prodrug, stereoisomer (including enantiomer), or mixture of stereoisomers thereof, and at least one pharmaceutically acceptable carrier.

In some embodiments, pharmaceutically acceptable carriers include, but are not limited to, fillers, stabilizers, dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that may be used as pharmaceutically acceptable carriers include: sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common non-toxic compatible substances used in pharmaceutical formulations.

The pharmaceutical composition of the present disclosure may further comprise at least one second therapeutic agent, e.g., an anticancer agent. The second therapeutic agent may be used in combination with the compounds of Formula (I) of the present disclosure to treat the diseases or disorders as disclosed herein. The second therapeutic agent includes, but is not limited to, chemotherapeutic agents, immunotherapeutic agents, gene therapy agents, and the like.

The pharmaceutical composition of the present disclosure comprising, as an active ingredient, the compounds of Formula (I) of the present disclosure or a pharmaceutically acceptable salt thereof may be formulated into any suitable formulations such as sprays, patches, tablets (such as conventional tablets, dispersible tablets, orally disintegrating tablets), capsules (such as soft capsules, hard capsules, enteric-coated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of Formula (I) of the present disclosure into conventional, dispersible, chewable, orally disintegrating or rapidly dissolving formulations, or sustained-release capsules or controlled-release capsules as needed.

The compounds of Formula (I) of the present disclosure, as an active ingredient, is contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25mg per kilogram body weight of the subject per day.

The compounds of Formula (I) of the present disclosure or pharmaceutically acceptable salts thereof may be conveniently administered in any suitable unit dosage form. Suitable unit dosage form specifications include, but are not limited to, less than 1mg, 1mg to 3000mg, 5mg to 1000mg, for example, 5 to 500mg, 25 to 250mg of active ingredient per unit dosage form.

### IV. Kits/Packaged Products

The compounds of Formula (I) or a pharmaceutically acceptable salt, solvate, isotopically enriched analog, polymorph, prodrug, stereoisomer (including enantiomer), or mixture of stereoisomers thereof is used as a medicament. The medicament of the present disclosure or the pharmaceutical composition of the present disclosure may be presented in a kit/packaged product. The kit/packaged product may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/packaged product may contain instructions for use of the product.

### V. Methods and Uses

The compounds of Formula (I) or a pharmaceutically acceptable salt, solvate, isotopically enriched analog, polymorph, prodrug, stereoisomer (including enantiomer), or mixture of stereoisomers thereof can be used as a medicament. Especially the compounds of Formula (I) or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of diseases or disorders selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular diseases, dysfunctional uterine bleeding, anemia, pediatric aplastic anemia, endometriosis, transplant rejection, polycystic ovary syndrome, and thyroid disease. In some embodiments, the diseases or disorders include, but are not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML), acute B-lymphocytic leukemia, acute T-lymphocytic leukemia, chronic lymphocytic leukemia, lymphoblastic leukemia, T-lymphocytic leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), marginal zone lymphoma (MZL), primary lymphoma, Burkitt's lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer and small cell lung cancer; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

The present disclosure provides a method for preventing and/or treating diseases or disorders in a subject, comprising administering to the subject a therapeutically effective amount of the compounds of Formula (I) of the present disclosure or a pharmaceutically acceptable salt thereof, or the pharmaceutical composition of the present disclosure comprising as an active ingredient the compounds of Formula (I) of the present disclosure or a pharmaceutically acceptable salt thereof, wherein the diseases or disorders include, but are not limited to, tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular diseases, dysfunctional uterine bleeding, anemia, pediatric aplastic anemia, endometriosis, transplant rejection, polycystic ovary syndrome, and thyroid disease. In some embodiments, the diseases or disorders include, but are not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML), acute B-lymphocytic leukemia, acute T-lymphocytic leukemia, chronic lymphocytic leukemia, lymphoblastic leukemia, T-lymphocytic leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), marginal zone lymphoma (MZL), primary lymphoma, Burkitt's lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer and small cell lung cancer; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

The compounds of Formula (II) or a pharmaceutically acceptable salt, solvate, isotopically enriched analog, polymorph, prodrug, stereoisomer (including enantiomer), or mixture of stereoisomers thereof, is a novel CRBN E3 ubiquitin ligase ligand that can function as a molecular glue to bind CRBN E3 ligase.

The compounds of Formula (II) or a pharmaceutically acceptable salt, solvate, isotopically enriched analog, polymorph, prodrug, stereoisomer (including enantiomer), or mixture of stereoisomers thereof, may be used in the manufacture of Cereblon protein (CRBN) binder.

The compounds of Formula (II) or a pharmaceutically acceptable salt, solvate, isotopically enriched analog, polymorph, prodrug, stereoisomer (including enantiomer), or mixture of stereoisomers thereof, may be used in the preparation of the compounds of Formula (I) of the present disclosure. The compounds of Formula (I) of the present disclosure can be used to treat and/or prevent diseases or disorders selected from: tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular diseases, dysfunctional uterine bleeding, anemia, pediatric aplastic anemia, endometriosis, transplant rejection, polycystic ovary syndrome, and thyroid disease.

The compounds of Formula (II) or a pharmaceutically acceptable salt, solvate, isotopically enriched analog, polymorph, prodrug, stereoisomer (including enantiomer), or mixture of stereoisomers thereof, may also be used as a medicament. In particular, the compounds of Formula (II) or a pharmaceutically acceptable salt, solvate, isotopically enriched analog, polymorph, prodrug, stereoisomer (including enantiomer), or mixture of stereoisomers thereof, can be used in the manufacture of a medicament for the prevention and/or treatment of a disease or disorder associated with a cereblon protein.

The disease or disorder associated with a cereblon protein includes tumor, cancer, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes. In some embodiments, the disease or disorder associated with a cereblon protein includes, but is not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including chronic myelogenous leukemia, B-cell chronic lymphocytic leukemia, leukemia-associated anemia, acute myeloid leukemia (AML), chronic myeloid leukemia (CML), monocytic leukemia, myelomonocytic leukemia, acute lymphoblastic leukemia (ALL), acute T-lymphoblastic leukemia, T-lymphocytic leukemia, chronic lymphocytic leukemia (CLL), lymphoma cell leukemia; lymphoma, including diffuse large B-cell lymphoma, non-Hodgkin's lymphoma, Hodgkin's lymphoma, anaplastic lymphoma, anaplastic large cell lymphoma, immunoblastic T-cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal large B-cell lymphoma, primary mediastinal large B-cell lymphoma, recurrent transformed non-Hodgkin's lymphoma, refractory B-cell non-Hodgkin's lymphoma, refractory diffuse large B-cell lymphoma, refractory primary mediastinal large B-cell lymphoma, refractory transformed non-Hodgkin's lymphoma; thyroid cancer; melanoma; lung cancer, including lung adenocarcinoma, lung squamous cell carcinoma, non-small cell lung cancer and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, breast ductal carcinoma, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular disease (including congestive heart failure, myocardial infarction, coronary heart disease, atherosclerosis); multiple organ dysfunction caused by cachexia and septic shock; and acute liver failure.

The present disclosure provides a method for preventing and/or treating a disease or disorder associated with a cereblon protein in a subject, comprising administering to the subject a therapeutically effective amount of the compounds of Formula (II) of the present disclosure or a pharmaceutically acceptable salt thereof, or the pharmaceutical composition of the present disclosure comprising as an active ingredient the compounds of Formula (II) of the present disclosure or a pharmaceutically acceptable salt thereof. In some embodiments, the disease or disorder associated with a cereblon protein includes tumor, cancer, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes. In some embodiments, the disease or disorder associated with a cereblon protein includes, but is not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including chronic myelogenous leukemia, B-cell chronic lymphocytic leukemia, leukemia-associated anemia, acute myeloid leukemia (AML), chronic myeloid leukemia (CML), monocytic leukemia, myelomonocytic leukemia, acute lymphoblastic leukemia (ALL), acute T-lymphoblastic leukemia, T-lymphocytic leukemia, chronic lymphocytic leukemia (CLL); lymphoma, including diffuse large B-cell lymphoma, non-Hodgkin's lymphoma, Hodgkin's lymphoma, anaplastic lymphoma, anaplastic large cell lymphoma, immunoblastic T-cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal large B-cell lymphoma, primary mediastinal large B-cell lymphoma, recurrent transformed non-Hodgkin's lymphoma, refractory B-cell non-Hodgkin's lymphoma, refractory diffuse large B-cell lymphoma, refractory primary mediastinal large B-cell lymphoma, refractory transformed non-Hodgkin's lymphoma; thyroid cancer; melanoma; lung cancer, including lung adenocarcinoma, lung squamous cell carcinoma, non-small cell lung cancer and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, breast ductal carcinoma, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular disease (including congestive heart failure, myocardial infarction, coronary heart disease, atherosclerosis); multiple organ dysfunction caused by cachexia and septic shock; and acute liver failure.

In the method for preventing and/or treating diseases or disorders associated with cereblon protein, the compound of Formula (II) of the present disclosure or the pharmaceutical composition comprising as an active ingredient the compound of Formula (II) of the present disclosure is administered to the subject through at least one mode of administration selected from the group consisting of nasal administration, inhalation administration, topical administration, oral administration, oral mucosal administration, rectal administration, pleural cavity administration, peritoneal administration, intramuscular administration, subcutaneous, transdermal, epidural, intrathecal, and intravenous administration.

The term "treatment" or "treating" refers to administering to a subject the compound of Formula (I) or the compound of Formula (II) of the present disclosure, or a pharmaceutically acceptable salt thereof, or a pharmaceutical composition comprising as an active ingredient the compound of Formula (I) or the compound of Formula (II) or a pharmaceutically acceptable salt thereof, to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

It is to be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see e.g., Jun Li (chief editor), "Clinical Pharmacology", 4th edition, People's Public Health Press, 2008).

As used herein, the term "patient" or "subject" to be treated refers to animal, for example mammal, including but not limited to primate (such as human being), cow, sheep, goat, horse, dog, cat, rabbit, guinea pig, rat, mice, etc.

### VI. Definitions

Unless otherwise specified, the following words, phrases and symbols used herein generally have the meanings as described below.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of" are also provided.

As used herein, the term "about" used alone or in combination refers to approximately, roughly, nearly, or around. When the term "about" is used in conjunction with a numerical range, it modifies that range by extending the boundaries above and below the stated numerical value. In general, the term "about" can modify a numerical value above and below the stated value by an upward or downward (increasing or decreasing) variation, e.g., 10%, 5%, 2%, or 1%.

As used herein, the wording "...represents a bond" used alone or in combination means that the referenced group is a bond linker (that is, the referenced group is absent). For example, the wording "X represents a bond" means that X is a bond linker. In other words, when X represents a bond, the ring atom A of the structure of Formula (ULM) is directly connected to the ring W¹. For example, the wording "LIN represents a bond" means that LIN is a bond linker. In other words, when LIN represents a bond, the PBM in the structure of Formula (I) is directly connected to the ULM.

In this document, the term "optionally substituted" used alone or in combination means that the referenced group may be unsubstituted or substituted with one or more substituents as defined here. Herein, the wording "optionally substituted with..." and "unsubstituted or substituted" can be used interchangeably. The term "substituted" generally represents the replacement of one or more hydrogen atoms in the referenced structure by identical or different specific substituents. The number of substituents is not theoretically limited in any way, or is automatically limited by the size of the building units (i.e., the total number of replaceable hydrogen atoms in the building units), or as clearly defined herein.

As used herein, the term "inserted" of the expression "one or more groups selected from Rₐ, R_{b}, and any combination thereof are optionally inserted between any one or more pairs of adjacent carbon atoms in the backbone carbon chain of the linear or branched C₂₋₁₅ alkylene group", used alone or in combination, has a known definition in the art, which can mean that carbon-carbon bond between one or more pairs of adjacent carbon atoms in the referenced backbone carbon chain is interrupted by the groups Rₐ, R_{b}, or any combination of Rₐ and R_{b}. Herein, examples of the above-mentioned expression "one or more groups...... are inserted into" may include, but are not limited to, that one or more (1-30, 1-20, or 1-15, 1-10, 1-9, 1-8, 1-7, 1-6, 1-5, 1-4, 1-3, or 1-2, or 1) groups Rₐ as defined herein and/or one or more (1-30, 1-20, or 1-15, 1-10, 1-9, 1-8, 1-7, 1-6, 1-5, 1-4, 1-3, or 1-2, or 1) groups R_{b} as defined herein and/or any combination of one or more (1-30, 1-20, or 1-15, 1-10, 1-9, 1-8, 1-7, 1-6, 1-5, 1-4, 1-3, or 1-2, or 1) Rₐ with R_{b} are inserted between one or more pairs of adjacent carbon atoms in the backbone carbon chain, and the resulting backbone chain group conforms to the covalent bond theory. For example, the expression "one or more groups selected from Rₐ, R_{b}, and any combination thereof are inserted between one or more pairs of adjacent carbon atoms in the backbone carbon chain of the linear or branched C₂₋₁₅ alkylene group" can refer to that one or more (e.g., 1-30, 1-20, 1-15, 1-10, 1-8, 1-7, 1-6, 1-5, 1 -4, 1-3, 1-2, or 1) groups Rₐ and/or one or more (e.g., 1-30, 1-20, 1-15, 1-10, 1-8, 1-7, 1-6, 1-5, 1 -4, 1-3, 1-2, or 1) groups R_{b} and/or any combination of one or more (1-30, 1-20, or 1-15, 1-10, 1-9, 1-8, 1-7, 1-6, 1-5, 1-4, 1-3, or 1-2, or 1) Rₐ with R_{b} are inserted between one or more pairs of any two adjacent carbon atoms of the backbone carbon chain of the linear or branched C₂₋₁₅ alkylene group, thereby forming a backbone carbon chain group containing fragments including but not limited to one or more (e.g., 1-30, 1-20, 1-15, 1-10, 1-8, 1-7, 1-6, 1-5, 1 -4, 1-3, 1-2, or 1) fragments "-CH₂-Rₐ-CH₂-" and/or one or more (e.g., 1-30, 1-20, 1-15, 1-10, 1-8, 1-7, 1-6, 1-5, 1-4, 1-3, 1-2, or 1) fragments "-CH₂-R_{b}-CH₂-" and/or one or more (e.g., 1-30, 1-20, 1-15, 1-10, 1-8, 1-7, 1-6, 1-5, 1-4, 1-3, 1-2, or 1) fragments "-CH₂-Rₐ-R_{b}-CH₂-" and/or one or more (e.g., 1-30, 1-20, 1-15, 1-10, 1-8, 1-7, 1-6, 1-5, 1-4, 1-3, 1-2, or 1) fragments "-CH₂-R_{b}-R_{b}-CH₂-", where each Rₐ are the same or different, each R_{b} are the same or different, and are as defined herein.

Herein, it should be understood that the expression "one or more groups selected from Rₐ, R_{b} and any combination thereof are optionally inserted between one or more pairs of adjacent carbon atoms in the backbone carbon chain of the linear or branched C₂₋₁₅ alkylene group" includes embodiments where "one or more groups Rₐ and/or one or more groups R_{b} or any combination of one or more groups Rₐ and R_{b} may or may not be inserted between one or more pairs of adjacent carbon atoms in the backbone carbon chain of the linear or branched C₂₋₁₅ alkylene group".

Herein, a bond interrupted by a wavy line shows the point of attachment of the depicted group to the rest of the molecule. For example, the group of Formula (ULM) depicted below 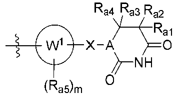 shows the point of attachment of ring W¹ in said group to LIN of the compound of Formula (I). Herein, when a depicted group has two bonds interrupted by a wavy line, for example, when the moiety 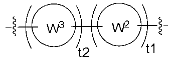 in each general formula of R_{c} represents the following group: 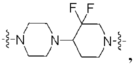 unless otherwise specified, it indicates that either end (e.g., the piperazinyl group) of said group may be connected to LIN. Herein, when R_{c} represents the following group: 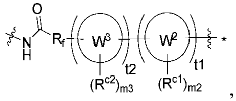 the symbol * indicates the point of attachment to LIN.

As used herein, the expression "one or more hydrogens of the C_{x-y} alkylene are replaced by...", used alone or in combination, means that any one or more hydrogens of the linear or branched C_{x-y} alkylene is replaced by a substituent(s) as defined herein. Herein, the term "one or more" may refer to part or all hydrogen atoms of each referenced alkylene group, including but not limited to 1-60 hydrogens, e.g., 1-30, such as 1-25, 1-20, 1-15, 1-10, 1-5, 1-4, 1-3, 1-2 or 1 hydrogen atoms. The number of hydrogens to be replaced is in principle not limited in any way, or is automatically limited by the size of the building unit.

As used herein, the term "deuterated," used alone or in combination, indicates that one or more hydrogen atoms in the referenced group are replaced by deuterium atoms.

As used herein, the term "oxo" or "oxo group" refers to =O.

As used herein, the term "halogen atom" or "halogen", used alone or in combination, refers to fluorine, chlorine, bromine, or iodine.

As used herein, the term "alkyl", used alone or in combination, refers to a linear or branched alkyl group. The term "Cₓ-C_{y} alkyl" or "C_{x-y} alkyl" (x and y each being an integer) refers to a linear or branched alkyl group containing from x to y carbon atoms. The term "C₁₋₁₀ alkyl" used alone or in combination in the present disclosure refers to a linear or branched alkyl group containing from 1 to 10 carbon atoms. Examples of the C₁₋₁₀ alkyl of the present disclosure may include a C₁₋₉ alkyl, C₁₋₈ alkyl, C₂₋₈ alkyl, C₁₋₇ alkyl, C₁₋₆ alkyl, C₁₋₅ alkyl, C₁₋₄ alkyl, and C₁₋₃ alkyl. Representative examples include methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, and decyl. The term "C₁₋₃ alkyl" or "C₁-C₃ alkyl" in the present disclosure refers to an alkyl group containing from 1 to 3 carbon atoms, and representative examples thereof include methyl, ethyl, n-propyl, and isopropyl. In the present disclosure, the "alkyl" is optionally substituted with one or more substituents optionally selected from the group consisting of halogen, hydroxyl, cyano, C₁₋₃ alkyl, C₁₋₃ alkoxy, trifluoromethyl, heterocyclyl, or a combination thereof.

As used herein, the term "halogenated alkyl" or "haloalkyl", used alone or in combination, refers to a linear or branched alkyl group substituted with one or more halogens, wherein one or more hydrogen atom(s) of the alkyl group are replaced with one or more halogens. The term "halogenated C_{x-Cy} alkyl" or "halogenated C_{x-y} alkyl" (x and y are each an integer) refers to a linear or branched alkyl containing from x to y carbon atoms substituted with one or more halogens. The term "halogenated C₁₋₁₀ alkyl" used alone or in combination in the present disclosure refers to a linear or branched alkyl group containing from 1 to 10 carbon atoms substituted with one or more halogens. Examples of the halogenated C₁₋₁₀ alkyl group of the present disclosure include halogenated C₁₋₉ alkyl group, e.g., halogenated C₁₋₈ alkyl group, halogenated C₂₋₈ alkyl group, halogenated C₁₋₇ alkyl group, halogenated C₁₋₆ alkyl, halogenated C₁₋₅ alkyl, or halogenated C₁₋₄ alkyl. Representative examples include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, halohexyl, haloheptyl, halooctyl, halononyl, and halodecyl. The term "halo-C₁₋₃ alkyl" or "halo-C₁₋C₃ alkyl" of the present disclosure refers to an alkyl group containing from 1 to 3 carbon atoms substituted with one or more halogens, and its representative examples include halomethyl, haloethyl, halo-n-propyl and haloisopropyl.

As used herein, the term "deuterated alkyl", used alone or in combination, refers to a linear or branched alkyl group substituted with one or more deuterium atoms, wherein one or more hydrogen atoms of the alkyl group are replaced with one or more deuterium atoms. The term "deuterated C_{x-Cy} alkyl" or "deuterated C_{x-y} alkyl" (x and y are each an integer) refers to a linear or branched alkyl containing from x to y carbon atoms substituted with one or more deuterium atoms. The term "deuterated C₁₋₁₀ alkyl" used alone or in combination in the present disclosure refers to a linear or branched alkyl group containing from 1 to 10 carbon atoms substituted with one or more deuterium atoms. Examples of the deuterated C₁₋₁₀ alkyl group of the present disclosure include deuterated C₁₋₉ alkyl group, e.g., deuterated C₁₋₈ alkyl group, deuterated C₂₋₈ alkyl group, deuterated C₁₋₇ alkyl group, deuterated C₁₋₆ alkyl, deuterated C₁₋₅ alkyl, or deuterated C₁₋₄ alkyl. Representative examples include perdeuterated methyl (CD₃), perdeuterated ethyl (CD₃CD₂), perdeuterated n-propyl, perdeuterated isopropyl, perdeuterated n-butyl, perdeuterated isobutyl, perdeuterated sec-butyl, perdeuterated tert-butyl, perdeuterated pentyl, perdeuterated isopentyl, perdeuterated neopentyl, perdeuterated tert-pentyl, perdeuterated hexyl, perdeuterated heptyl, perdeuterated octyl, perdeuterated nonyl, and perdeuterated decyl. The term "deuterated C₁₋₃ alkyl" or "deuterated C₁₋C₃ alkyl" of the present disclosure refers to an alkyl group containing from 1 to 3 carbon atoms substituted with one or more deuterium atoms, and its representative examples include perdeuterated methyl (CD₃) and perdeuterated ethyl (CD₃CD₂).

As used herein, the term "alkylene" (which is used interchangeably with "alkylene chain"), used alone or in combination, refers to a linear or branched divalent saturated hydrocarbon group composed of carbon and hydrogen atoms. The term "Cₓ-C_{y} alkylene" or "C_{x-y} alkylene" (x and y each being an integer) refers to a linear or branched alkylene group containing from x to y carbon atoms. Examples of the C₁-C₁₅ alkylene in the present disclosure may include C₁-C₁₅ alkylene, C₁-C₁₄ alkylene, C₁-C₁₃ alkylene, C₁-C₁₂ alkylene, C₁-C₁₁ alkylene, C₁-C₁₀ alkylene, C₁-C₉ alkylene, C₁-C₈ alkylene, C₁-C₇ alkylene, C₁-C₆ alkylene, C₁-C₅ alkylene, C₁-C₄ alkylene, C₁₋C₃ alkylene, or C₁-C₂ alkylene. Representative examples include, but are not limited to, methylene, ethylene, propylene, isopropylene, butylene, isobutylene, sec-butylene, tert-butylene, n-pentylene, isopentylene, neopentylidene, tert-pentylene, hexylene, heptylene, octylene, nonylene, decylene, undecylene, dodecylene, tridecylene, tetradecylene, and pentadecylene. In the present disclosure, the "alkylene" is optionally substituted with one or more substituents optionally selected from C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "alkoxy", used alone or in combination, refers to a linear or branched alkoxy group having structural formula of -O-alkyl. Optionally, the alkyl portion of the alkoxy group may contain 1-10 carbon atoms. Representative examples of "alkoxy" include, but are not limited to, methoxy, ethoxy, propoxy, isopropoxy, n-butoxy, isobutoxy, tert-butoxy, pentyloxy, 2-pentyloxy, isopentyloxy, neopentyloxy, hexyloxy, 2-hexyloxy, 3-hexyloxy, 3-methylpentyloxy, etc. The term "C₁-C₃ alkoxy" or "C₁₋₃ alkoxy" refers to a linear or branched alkoxy group containing from 1 to 3 carbon atoms. Representative examples of C₁₋₃ alkoxy include, but are not limited to, methoxy, ethoxy, n-propoxy, and isopropoxy.

As used herein, the term "halogenated alkoxy", used alone or in combination, refers to a alkoxy group substituted with one or more halogen atoms. Optionally, the alkyl portion of the alkoxy group may contain 1-10 carbon atoms. Examples of "halogenated alkoxy" include, but are not limited to, halogenated C₁₋₆ alkoxy or halogenated C₁₋₄ alkoxy. Representative examples include, but are not limited to, F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-.

As used herein, the term "alkylamino", used alone or in combination, refers to a linear or branched alkylamino group having structural formula of alkyl-NH-. Optionally, the alkyl portion of the alkylamino group may contain 1-10 carbon atoms. Representative examples of "alkylamino" include, but are not limited to, methyl-NH-, ethyl-NH-, n-propyl-NH-, isopropyl-NH-, n-butyl-NH-, isobutyl-NH-, tert-butyl-NH-, pentyl-NH-, and hexyl-NH-, etc. The term "C₁-C₃ alkyl-NH-" or "C₁₋₃ alkyl-NH-" refers to a linear or branched alkyl-NH- group containing from 1 to 3 carbon atoms. Representative examples of C₁₋₃ alkyl-NH-include, but are not limited to, methyl-NH-, ethyl-NH-, n-propyl-NH-, and isopropyl-NH-.

As used herein, the term "amino-substituted alkylene", used alone or in combination, refers to a linear or branched alkylene group substituted with amino having structural formula of NH₂-alkylene. Optionally, the alkylene portion of the amino-substituted alkylene group may contain 1-10 carbon atoms. The term "amino-substituted C₁₋₃ alkylene" or " NH₂-C₁₋₃ alkyl" refers to a linear or branched alkylene group containing from 1 to 3 carbon atoms which is substituted with amino. Representative examples of amino-substituted C₁₋₃ alkylene include, but are not limited to, NH₂-CH₂-, NH₂-CH₂CH₂-, and NH₂-CH₂CH₂CH₂-.

As used herein, the term "alkyl-NHC(O)-", used alone or in combination, refers to a linear or branched alkyl-NHC(O)- group having structural formula of alkyl-NHC(O)-. Optionally, the alkyl portion of the alkyl-NHC(O)- group may contain 1-10 carbon atoms. The term "C₁₋₃ alkyl-NHC(O)-" or "C₁-C₃ alkyl-NHC(O)-" refers to a linear or branched alkyl-NHC(O)- group containing from 1 to 3 carbon atoms. Representative examples of C₁₋₃ alkyl-NHC(O)- include, but are not limited to, CH₃-NHC(O)-, CH₃CH₂-NHC(O)-, and CH₃CH₂CH₂-NHC(O)-.

As used herein, the term "alkyl-C(O)NH-", used alone or in combination, refers to a linear or branched alkyl-C(O)NH- group having structural formula of alkyl-C(O)NH-. Optionally, the alkyl portion of the alkyl-C(O)NH- group may contain 1-10 carbon atoms. The term "C₁₋₃ alkyl-C(O)NH-" or "C₁-C₃ alkyl-C(O)NH-" refers to a linear or branched alkyl-C(O)NH- group containing from 1 to 3 carbon atoms. Representative examples of C₁₋₃ alkyl-C(O)NH- include, but are not limited to, CH₃-C(O)NH-, CH₃CH₂-C(O)NH-, and CH₃CH₂CH₂-C(O)NH-.

As used herein, the term "heteroaryl", used alone or in combination, refers to a 5- to 30-membered (e.g., 5- to 20-membered, 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic or tetracyclic heteroaryl groups, which contain one aromatic ring having one or more heteroatoms independently selected from O, S and N, and the remaining rings which may be a saturated, partially unsaturated or aromatic ring and can be carbocyclic ring or contain one or more heteroatoms independently selected from O, S and N. Examples of monocyclic heteroaryl groups include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, tetrazolyl, and triazinyl. Examples of bicyclic heteroaryl groups include, but are not limited to, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, oxazolopyridyl, furopyridyl, pteridyl, purinyl, pyridopyridyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl and imidazo[2,1-b]thiazolyl. Examples of tricyclic or polycyclic heteroaryl groups include, but are not limited to, acridinyl, benzindolyl, carbazolyl, dibenzofuranyl, and xanthyl. The heteroaryl group may be unsubstituted or substituted. A substituted heteroaryl refers to heteroaryl substituted one or more times (e.g., 1-4, 1-3, or 1-2 times) by a substituent(s) optionally selected from the group consisting of C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "heteroarylene", used alone or in combination, refers to a 5- to 30-membered (e.g., 5- to 20-membered, 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) bivalent monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic or tetracyclic heteroarylene groups, which contain one aromatic ring having one or more heteroatoms independently selected from O, S and N, and the remaining ring(s) which may be a saturated, partially unsaturated or aromatic ring and can be carbocyclic ring or contain one or more heteroatoms independently selected from O, S and N. Examples of monocyclic heteroarylene groups include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, and triazinylene. Examples of bicyclic heteroarylene groups include, but are not limited to, indolylene, isoindolylene, isoindolinylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, oxazolopyridylene, furopyridylene, pteridylene, purinylene, pyridopyridylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene. Examples of tricyclic or polycyclic heteroarylene groups include, but are not limited to, acridinylene, benzindolylene, carbazolylene, dibenzofuranylene, and xanthylene. The heteroarylene group may be unsubstituted or substituted. A substituted heteroarylene refers to heteroarylene substituted one or more times (e.g., 1-4, 1-3, or 1-2 times) by a substituent(s) optionally selected from the group consisting of C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "aryl", used alone or in combination, refers to a monovalent aromatic hydrocarbon group containing from 5 to 30 (e.g., from 5 to 25, from 5 to 20, from 5 to 14, or from 6 to 20) carbon atoms and optionally one or more fused rings, such as phenyl group, naphthyl group, or fluorenyl group. As used herein, the "aryl" is optionally substituted. A substituted aryl group refers to an aryl group substituted one or more times (e.g., 1-4, 1-3, or 1-2 times) with a substituent(s). For example, aryl is mono-, di-, or tri-substituted with a substituent(s) optionally selected from e.g., C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "arylene", used alone or in combination, refers to a divalent aromatic hydrocarbon group containing from 5 to 30 (e.g., from 5 to 25, from 5 to 20, from 5 to 14, or from 6 to 20) carbon atoms and optionally one or more fused rings, such as phenylene, naphthylene, or fluorenylene. As used herein, the "arylene" is optionally substituted. A substituted arylene refers to an arylene group substituted one or more times (e.g., 1-4, 1-3, or 1-2 times) with a substituent(s). For example, arylene is mono-, di-, or tri-substituted with a substituent(s) optionally selected from e.g., C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "cycloalkyl", used alone or in combination, refers to a saturated or partially unsaturated (i.e., containing one or more double bonds, but not having a fully conjugated π-electron system) monocyclic or bicyclic or tricyclic or polycyclic cyclic hydrocarbon radical, which in some embodiments contains from 3 to 30 carbon atoms (i.e., C₃₋₃₀ cycloalkyl), from 3 to 20 carbon atoms (i.e., C₃₋₂₀ cycloalkyl), or from 3 to 15 carbon atoms (i.e., C₃₋₁₅ cycloalkyl), or from 3 to 12 carbon atoms (i.e., C₃₋₁₂ cycloalkyl), or from 3 to 11 carbon atoms (i.e., C₃₋₁₁ cycloalkyl), or from 3 to 10 carbon atoms (i.e., C₃₋₁₀ cycloalkyl), or from 3 to 8 carbon atoms ( i.e., C₃₋₈ cycloalkyl), or from 3 to 7 carbon atoms (i.e., C₃₋₇ cycloalkyl), or from 3 to 6 carbon atoms (i.e., C₃₋₆ cycloalkyl). The term "cycloalkyl" includes monocyclic, bicyclic, tricyclic, and polycyclic cyclic hydrocarbon radical having from 3 to 20 carbon atoms. Examples of the term "cycloalkyl" include, but are not limited to, monocyclic cycloalkyl, bridged cycloalkyl (e.g., C₅₋₁₅ bridged cycloalkyl or C₇₋₁₅ bridged cycloalkyl), fused cycloalkyl, and spiro-cycloalkyl (e.g., C₅₋₁₅ spiro-cycloalkyl). Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Examples of bridged cycloalkyl, fused cycloalkyl and spiro-cycloalkyl groups include, but are not limited to, decalinyl, octahydropentalenyl, octahydro-1H-indenyl, C₅₋₁₅ spiro-cycloalkyl, adamantanyl, noradamantanyl, bornyl, norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" can be optionally one or more (e.g., 1-5, 1-4, 1-3, 1-2, or 1) substituents selected from C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof. Examples of "C₃₋₆ cycloalkyl" include, but are not limited to, cyclopropyl, cyclobutyl, cyclopentyl, cyclopentenyl, and cyclohexyl.

As used herein, the term "C_{x-y} spiro-cycloalkyl" (x and y each being an integer), used alone or in combination, refers to a spiro-cycloalkyl group containing from x to y carbon atoms. As used herein, the term "C₅₋₁₅ spiro-cycloalkyl", used alone or in combination, refers to a spiro-cycloalkyl group containing from 5 to 15 carbon atoms (e.g., 7-15, 5-11, 5-10, or 7-9 carbon atoms). The term "C₅₋₁₅ spirocycloalkyl" includes "C₇₋₁₅ spirocycloalkyl", and representative examples thereof include, but are not limited to, spiro[3.3]heptanyl, spiro[2.5]octanyl, spiro[3.5]nonanyl, spiro[4.4]nonanyl, spiro[4.5]decanyl, and spiro[5.5]undecanyl. The "C₅₋₁₅ spiro-cycloalkyl" is optionally further substituted with one or more substituents selected from the group consisting of C₁₋₃ alkyl, halogenated C₁₋₃ alkyl, C₁₋₃ alkoxy, deuterated C₁₋₃ alkoxy, C₁₋₃ alkylamino, amino, oxo, halogen, hydroxy, cyano, or any combination thereof.

As used herein, the term "C_{x-y} bridged cycloalkyl" (where x and y are integers) used alone or in combination, refers to a bridged cycloalkyl group containing x to y carbon atoms. In the present invention, the term "C₅₋₁₅ bridged cycloalkyl" used alone or in combination, refers to a bridged cycloalkyl group containing 5 to 15 (e.g., 7-15, 7-11, 5-10, or 7-9) carbon atoms. Representative examples of the term "C₅₋₁₅ bridged cycloalkyl" include, but are not limited to, adamantanyl, noradamantanyl, norbornanyl (systematically named bicyclo[2.2.1]heptanyl), and cubanyl. Said "bridged cycloalkyl" may be optionally substituted with 1 to 10 substituents selected from: C₁₋₃ alkyl, deuterated C₁₋₃ alkyl, C₁₋₃ alkoxy, halogen, halogenated C₁₋₃ alkyl, C₁₋₃ alkylamino, amino, hydroxy, cyano, oxo, or any combination thereof.

As used herein, the term "cycloalkylene", used alone or in combination, refers to a saturated or partially unsaturated (i.e., containing one or more double bonds, but not having a fully conjugated π-electron system) monocyclic, bicyclic, tricyclic or polycyclic divalent cyclic hydrocarbon radical, which in some embodiments contains from 3 to 30 carbon atoms (i.e., C₃₋₃₀ cycloalkylene), from 3 to 20 carbon atoms (i.e., C₃₋₂₀ cycloalkylene), or from 3 to 15 carbon atoms (i.e., C₃₋₁₅ cycloalkylene), or from 3 to 12 carbon atoms (i.e., C₃₋₁₂ cycloalkylene), or from 3 to 11 carbon atoms (i.e., C₃₋₁₁ cycloalkylene), or from 3 to 10 carbon atoms (i.e., C₃₋₁₀ cycloalkylene), or from 3 to 8 carbon atoms ( i.e., C₃₋₈ cycloalkylene), or from 3 to 7 carbon atoms (i.e., C₃₋₇ cycloalkylene), or from 3 to 6 carbon atoms (i.e., C₃₋₆ cycloalkylene). The term "cycloalkylene" includes monocyclic, bicyclic, tricyclic and polycyclic divalent cyclic hydrocarbon radical having from 3 to 30 (e.g., 3-20) carbon atoms. Examples of the term "cycloalkylene" include, but are not limited to, monocyclic cycloalkylene, bridged cycloalkylene (e.g., C₅₋₁₅ bridged cycloalkylene or C₇₋₁₅ bridged cycloalkylene), fused cycloalkylene, and spiro-cycloalkylene (e.g., C₅₋₁₅ spiro-cycloalkylene). Representative examples of monocyclic cycloalkylene groups include, but are not limited to, cyclopropylene, cyclobutylene, cyclopentylene, cyclopentenylene, cyclohexylene, cyclohexenylene, cycloheptylene, and cyclooctylene. Examples of bridged cycloalkylene, fused cycloalkylene and spiro-cycloalkylene groups include, but are not limited to, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbornylene (also named as bicyclo[2.2.1]heptylene by the IUPAC system). As used herein, the "cycloalkylene" is optionally mono- or poly-substituted, such as, but not limited to, 2,2-, 2,3-, 2,4-, 2,5-, or 2,6-disubstituted cyclohexylene. The substituents of the substituted "cycloalkylene" can be optionally one or more (e.g., 1-5, 1-4, 1-3, 1-2, or 1) substituents selected from the group consisting of C₁-C₃ alkyl, C₃₋₆ cycloalkyl, hydroxy, amino, mercapto, halogen, C₁₋C₃ alkoxy, C₁-C₃ alkylamino, halogenated C₁₋C₃ alkyl, amino-substituted C₁₋₃ alkylene, C₁₋₃ alkyl-NHC(O)-, C₁₋₃ alkyl-C(O)NH-, cyano or any combination thereof.

As used herein, the term "C_{x-y} spiro-cycloalkylene" (x and y each being an integer), used alone or in combination, refers to a spiro-cycloalkylene group containing from x to y carbon atoms. As used herein, the term "C₅₋₁₅ spiro-cycloalkylene", used alone or in combination, refers to a spiro-cycloalkylene group containing from 5 to 15 carbon atoms (e.g., 7-15, 5-11, 5-10, or 7-9 carbon atoms). Representative examples of "C₅₋₁₅ spiro-cycloalkylene" include, but are not limited to, spiro[3.3]heptylene, spiro[2.5]octylene, spiro[3.5]nonylene, spiro[4.4]nonylene, spiro[4.5]decylene, or spiro[5.5]undecylene. The "C₅₋₁₅ spiro-cycloalkylene" is optionally further substituted with one or more substituents selected from the group consisting of C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "C_{x-y} bridged cycloalkylene" (where x and y are integers) used alone or in combination, refers to a bridged cycloalkylene group containing x to y carbon atoms. In the present invention, the term "C₅₋₁₅ bridged cycloalkylene" used alone or in combination, refers to a bridged cycloalkylene group containing 5 to 15 (e.g., 7-15, 7-11, 5-10, or 7-9) carbon atoms. Representative examples of the term "C₅₋₁₅ bridged cycloalkylene" include, but are not limited to, adamantanylene, noradamantanylene, norbornanylene, bicyclo[2.2.1]heptanylene, 2-oxobicyclo[2.2.1]heptanylene and bicyclo[2.2.1]heptenylene. Said "C₅₋₁₅ bridged cycloalkylene" may be optionally substituted with one or more substituents selected from: C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "heterocyclyl" or "heterocyclic group", used alone or in combination, refers to a 4- to 30-membered (e.g., 4- to 20-membered) saturated or partially unsaturated (i.e., containing one or more double bonds, but not having a fully conjugated π-electron system) monocyclic, bicyclic, tricyclic or polycyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclyl" may refer to a 4- to 20-membered (e.g., 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, or 4- to 9-membered) saturated or partially unsaturated (i.e., containing one or more double bonds, but not having a fully conjugated π-electron system) monocyclic, bicyclic, tricyclic or polycyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclyl groups include, but not limited to, monocyclic heterocyclyl (e.g., 4- to 20-membered monocyclic heterocyclyl), bridged heterocyclyl (e.g., 5- to 20-membered bridged heterocyclyl, and 7- to 15-membered bridged heterocyclyl), fused heterocyclyl and spiro-heterocyclyl groups (e.g., 5- to 20-membered spiro-heterocyclyl). Representative examples of the monocyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl) and diazacyclooctyl. Examples of bridged heterocyclyl, fused heterocyclyl and spiro-heterocyclyl groups include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, and azaspirocycloalkyl (e.g., 5- to 20-membered azaspirocycloalkyl, such as 3-azaspiro[5.5]undecan-3-yl). The heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- and any combination thereof.

As used herein, the term "nitrogen-containing monocyclic heterocyclyl", used alone or in combination, refers to 4- to 30-membered (e.g., 4- to 20-membered, 4- to 15-membered, 4- to 14-membered, 4- to 12-membered, 4- to 11-membered, 4- to 10-membered, 4- to 9-membered, 4- to 8-membered, 4- to 7-membered, 4- to 6-membered, 4- to 5-membered, or 5- to 9-membered) saturated or partially unsaturated (i.e., containing one or more double bonds, but not having a fully conjugated π-electron system) monocyclic monovalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Representative examples of the nitrogen-containing monocyclic heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl (e.g., 1,4-diazacycloheptan-1-yl), and diazacyclooctyl. The nitrogen-containing monocyclic heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "nitrogen-containing bridged heterocyclyl", used alone or in combination, refers to 5- to 30-membered (e.g., 7- to 20-membered, 5- to 15-membered, 5- to 14-membered, 6- to 12-membered, 6- to 11-membered, 6- to 10-membered, 5- to 9-membered, 5- to 8-membered, 5- to 7-membered, 5- to 6-membered, or 6- to 9-membered) saturated or partially unsaturated (i.e., containing one or more double bonds, but not having a fully conjugated π-electron system) bicyclic, tricyclic or polycyclic monovalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of nitrogen-containing bridged heterocyclyl include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, and 2,5-diazabicyclo[2.2.2]octan-2-yl. The nitrogen-containing bridged heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "-O-optionally substituted heterocyclyl", used alone or in combination, refers to a group formed by optionally substituted heterocyclyl connected to oxygen. The definition of "heterocyclyl" may be as defined above. Examples of the term "heterocyclyl" include, but are not limited to, monocyclic heterocyclyl (e.g., 4- to 20-membered monocyclic heterocyclyl), bridged heterocyclyl (e.g., 5- to 20-membered bridged heterocyclyl or 7- to 15-membered bridged heterocyclyl), fused heterocyclyl, and spiro heterocyclyl (e.g., 5- to 20-membered spiro heterocyclyl). Optionally, the heterocyclyl in the term "-O-optionally substituted heterocyclyl" refers to a 4- to 20-membered mono-, bi-, tri-, or polycyclic heterocyclyl containing one or more (e.g., 1-4, or 1, 2, 3, or 4) heteroatoms selected from nitrogen, oxygen, and sulfur. Examples of the "heterocyclyl" include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). As used herein, the heterocyclyl is optionally substituted with a substituent selected from deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "heterocyclylene", used alone or in combination, refers to a 4- to 30-membered (e.g., 4- to 20-membered) saturated or partially unsaturated (i.e., containing one or more double bonds, but not having a fully conjugated π-electron system) monocyclic, bicyclic, tricyclic or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclylene" may refer to e.g., a 4- to 20-membered (optionally 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, or 4- to 9-membered) saturated or partially unsaturated (i.e., containing one or more double bonds, but not having a fully conjugated π-electron system) monocyclic, bicyclic, tricyclic or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclylene groups include, but are not limited to, monocyclic heterocyclylene (e.g., 4- to 20-membered monocyclic heterocyclylene), bridged heterocyclylene (e.g., 5- to 20-membered bridged heterocyclylene, and 7- to 15-membered bridged heterocyclylene), fused heterocyclylene and spiro-heterocyclylene groups (e.g., 5- to 20-membered spiro-heterocyclylene). Representative examples of the monocyclic heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, and diazacycloheptanylene (e.g., 1,4-diazacycloheptanylene, 4,5-diazacycloheptanylene, 1,3-diazacycloheptanylene). Examples of the bridged heterocyclylene, fused heterocyclylene and spiro-heterocyclylene groups include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 5- to 20-membered azaspirocycloalkylene, such as 3-azaspiro[5.5]undecanylene). The heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "nitrogen-containing monocyclic heterocyclylene", used alone or in combination, refers to 4- to 30-membered (e.g., 4- to 20-membered, 4- to 15-membered, 4- to 14-membered, 4- to 12-membered, 4- to 11-membered, 4- to 10-membered, 4- to 9-membered, 4- to 8-membered, 4- to 7-membered, 4- to 6-membered, 4- to 5-membered, or 5- to 9-membered) saturated or partially unsaturated (i.e., containing one or more double bonds, but not having a fully conjugated π-electron system) monocyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Representative examples of the nitrogen-containing monocyclic heterocyclylene include, but are not limited to, piperidinylene, piperazinylene, morpholinylene, azetidinylene, pyrrolidinylene, imidazolidinylene, pyrazolidylene, oxazolidinylene, thiazolidinylene, thiomorpholinylene, azacycloheptylene, diazacycloheptanylene, azacyclooctylene, and diazacyclooctylene. The nitrogen-containing monocyclic heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "nitrogen-containing bridged heterocyclylene", used alone or in combination, refers to 5- to 30-membered (e.g., 5- to 20-membered, 7- to 20-membered, 7- to 15-membered, 5- to 15-membered, 5- to 14-membered, 6- to 12-membered, 6- to 11-membered, 6- to 10-membered, 5- to 9-membered, 5- to 8-membered, 5- to 7-membered, 5- to 6-membered, or 6- to 9-membered) saturated or partially unsaturated (i.e., containing one or more double bonds, but not having a fully conjugated π-electron system) bicyclic, tricyclic or polycyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the nitrogen-containing bridged heterocyclylene include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, and 2,5-diazabicyclo[2.2.2]octanylene. The nitrogen-containing heterocyclylene may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH- or any combination thereof.

As used herein, the term "alkynylene", used alone or in combination, refers to a linear or branched bivalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynylene include, but are not limited to, ethynylene, 1-propynylene, 1-butynylene, and 1,3-diynylene.

As used herein, the term "alkynyl", used alone or in combination, refers to a linear or branched monovalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of C₂₋₆ alkynyl include, but are not limited to, ethynyl, 1-propynyl, 1-butynyl, and 1,3-diynyl.

As used herein, the term "alkenylene", used alone or in combination, refers to a linear or branched divalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of alkenylene groups include, but are not limited to, vinylene (e.g., -CH=CH-), 1-propenylene, allylidene, 1-butenylene, 2-butenylene, 3-butenylene, isobutenylene, pentenylene, n-pent-2,4-dienylene, 1-methyl-but-1-enylene, 2-methyl-but-1-enylene, 3-methyl-but-1-enylene, 1-methyl-but-2-enylene, 2-methyl-but-2-enylene, 3-methyl-but-2-enylene, 1-methyl-but-3-enylene, 2-methyl-but-3-enylene, 3-methyl-but-3-enylene, and hexenylene.

As used herein, the term "alkenyl", used alone or in combination, refers to a linear or branched monovalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of C₂₋₆ alkenyl group include, but are not limited to, vinyl (e.g., CH₂=CH-), 1-propenyl, allyl, 1-butenyl, 2-butenyl, 3-butenyl, isobutenyl, pentenyl, n-pent-2,4-dienyl, 1-methyl-but-1-enyl, 2-methyl-but-1-enyl, 3-methyl-but-1-enyl, 1-methyl-but-2-enyl, 2-methyl-but-2-enyl, 3-methyl-but-2-enyl, 1-methyl-but-3-enyl, 2-methyl-but-3-enyl, 3-methyl-but-3-enyl, and hexenyl.

As used herein, "bornylane" or "bornane" (also known as 1,7,7-trimethylbicyclo[2.2.1]heptane; camphane; bornylane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

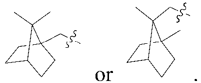

As used herein, "bicyclo[2.2.1]heptane" also known as "norbornane", has a definition known to those skilled in the art. As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en- 3-yl, or bicyclo[2.2.1]hept-5-en-7-yl.

As used herein, "adamantane" (also known as tricyclo[3.3.1.1^{3,7}]decane) has a definition known to those skilled in the art, and its structural formula is e.g., as follows: 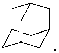 As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" has a definition known to those skilled in the art, and its structural formula is e.g., as follows: 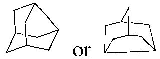 . As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

As used herein, "adamantanamine" has the definitions known to those skilled in the art, namely referring to an adamantane having an amino substituent, wherein the amino substituent can replace a hydrogen on a carbon at any position in the adamantane. An example of "adamantanamine" can be adamantan-1-amine (corresponding English chemical name is adamantan-1-amine or Tricyclo[3.3.1.1^{3,7}]decan-1-amine; CAS No.: 768-94-5), with the following structural Formula:

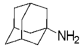

In the present disclosure, the term "leaving group" used alone or in combination is well known to those skilled in the art, which is a leaving molecular fragment (ion or neutral molecule) that carries a pair of electrons from a reactant in chemical reactions, as is a term used in nucleophilic substitution and elimination reactions. Common ionic leaving groups include Cl⁻, Br⁻, I⁻ and sulfonate (such as p-toluenesulfonate, TsO⁻), and neutral molecular leaving groups include water, ammonia and alcohol. In the present disclosure, those skilled in the art can select an appropriate leaving group as needed, such as but not limited to -N₃, halogen, methanesulfonyloxy (MsO-), trifluoromethanesulfonyloxy (TfO-) or p-toluenesulfonyloxy (TsO-), etc.

Salts or pharmaceutically acceptable salts, enantiomers, stereoisomers, solvates, polymorphs of the compounds of Formula (I) or Formula (II) of the present disclosure are also encompassed within the scope of the present invention.

**In** all embodiments of the present disclosure, the salts or pharmaceutically acceptable salts of the compounds of Formula (I) or Formula (II) refer to non-toxic inorganic or organic acid and/or base addition salts. Examples include: sulfates, hydrohalides (including hydrochlorides and hydrobromates), maleates, sulfonates, citrates, lactates, lactobionates, L-tartrates, fumarates, L-malates, L-lactates, α-Ketoglutarates, hippurates, D-glucuronates, D-gluconates, α-D-glucoheptonates, glycolates, mucates, L-ascorbates, orotates, picrates, glycinates, alaninates, argininates, cinnamates, laurates, pamoates, sebacates, benzenesulfonates, methanesulfonates, ethanesulfonates, edisylates, formates, acetates, 2,2-dichloroacetates, trimethylacetates, propionates, valerates, palmitates, triphenylacetates, 2-ethylsuccinates, iodates, niacinates, L-pyroglutamates, L-prolinates, ferulates, 2-hydroxyethanesulfonates, nitrates, gentisates, cholates, salicylates, terephthalates, glutarates, adipates, stearates, oleates, undecenoates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, para-chlorobenzenesulfonates, 1,5-naphthalenedisulfonates, 3-hydroxy-2-naphthoates, 1-hydroxy-2-naphthoates, 2-naphthalenesulfonates, hydroxy acetates, or p-toluenesulfonates, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

As used herein, "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "solvate" refers to an association or complex formed by the interaction between one or more solvent molecules and the compounds of the present invention. Examples of solvents include water, isopropanol, ethanol, methanol, DMSO, ethyl acetate, acetic acid and ethanolamine. The term "hydrate" means that a complex formed with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are nonsuperimposable mirror images.

As used herein, the term "diastereomers" refers to stereoisomers that have two or more chiral centers but are not mirror images of each other. The diastereomers have different physical properties, such as melting point, boiling point, spectral properties and reactivity. The diastereomeric mixture can be separated by high-resolution analytical operations such as electrophoresis and chromatography, such as HPLC.

### Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:
- ACN: acetonitrile
- AcOH: acetic acid
- AcOK: potassium acetate
- AIBN: 2,2'-Azobis(2-methylpropionitrile)
- Bn: Benzyl
- Boc: t-Butyloxy carbonyl
- Brettphos Pd G3: Methanesulfonato(2-dicyclohexylphosphino-3,6-dimethoxy-2',4',6'-tri-i-propyl-1,1'-biphenyl)(2'-amino-1,1'-biphenyl-2-yl)palladium(II)
- Brettphos: Dicyclohexyl[3,6-dimethoxy-2',4',6'-tris( 1-methylethyl)[1,1'-biphenyl]-2-yl]phosphine
- BINAP: 2,2'-Bis(diphenylphosphino)-1,1'-binaphthalene
- BPO: Dibenzoyl peroxide
- CataCXium A Pd G₃: Methanesulfonatobutyl-bis(1-adamantyl)phosphinepalladium(II)
- Cbz: Carbobenzoxy
- Con.: Concentration
- DCE: 1,2-Dichloroethane
- DCM: dichloromethane
- DEA: Diethylamine
- DIAD: Diisopropyl azodicarboxylate
- DIEA: N, N-diisopropylethylamine
- DMA: N,N-Dimethylacetamide
- DMAP: 4-Dimethylaminopyridine
- DME: Dimethylether
- DMEDA: N,N'-dimethylethylenediamine
- DMF: N,N-dimethylformamide
- DMSO: dimethyl sulfoxide
- DIPEA: N, N-diisopropylethylamine
- EDCI: 1-(3-dimethylaminopropyl)-3-ethylcarbodiimide hydrochloride
- ESI: electrospray ionization
- equiv: equivalent
- EtOH: ethanol
- EtOAc or EA: Ethyl acetate
- HATU: 2-(7-Azabenzotriazol-1-yl)-N,N,N',N'-tetramethyluronium hexafluorophosphate
- HPLC: high performance liquid chromatography
- HRMS: high resolution mass spectrometry
- IPA: Isopropyl alcohol
- LC-MS: liquid chromatography-mass spectrometry
- LiHMDS: Lithium hexamethyldisilazide
- LRMS: low resolution mass spectrometry
- LC: liquid chromatography
- Me: methyl
- MeCN: acetonitrile
- MeOH: Methanol
- MOMO-: Methoxymethoxy-
- MS: mass spectrometry
- MsO-: Methanesulfonyloxy
- NaHMDS: Sodium bis(trimethylsilyl)amide
- NBS: N-Bromosuccinimide
- NMI: N-Methylimidazole
- ¹H NMR: Proton nuclear magnetic resonance
- HFIP: Hexafluoroisopropanol
- MeO-: Methoxy
- o/n: Overnight
- OTBS: tert-butyldimethylsilyloxy
- PCC: Pyridinium chlorochromate
- Pin₂B₂: Pinacolborane
- PMB: p-methoxybenzyl
- PyBOP: Benzotriazol-1-yl-oxy-tris(pyrrolidino)phosphonium hexafluorophosphate
- Ruphos Pd G3: Methanesulfonato(2-dicyclohexylphosphino-2',6'-diisopropoxy-1,1'-biphenyl)(2'-amino-1,1'-biphenyl-2-yl)palladium(II) (CAS No.: 1445085-77-7)
- rt: room temperature
- tBu: tert-butyl
- tBuONa: Sodium tert-butoxide
- TBSCl: tert-Butyldimethylchlorosilane
- TCFH: N,N,N',N'-Tetramethylchloroformamidinium hexafluorophosphate
- TEA: triethylamine
- TFA: trifluoroacetic acid
- T_{f}O-: Trifluoromethanesulfonate
- T_{f}OH: trifluoromethanesulfonic acid
- Tf₂O: Triflic anhydride
- THF: Tetrahydrofuran
- THP: Tetrahydropyranyl
- TLC: thin layer chromatography
- TMS: tetramethylsilane
- TsO-: Tosyloxy
- TsOH: p-Toluenesulfonic acid
- Xantphos or Xphos: 4,5-Bis(diphenylphosphino)-9,9-dimethylxanthene

In the present disclosure, the ¹H NMR spectrum was recorded on a Bruker-500MHz nuclear magnetic resonance instrument, by using, as a solvent, CD₃OD (δ = 3.31 ppm) containing 0.1% TMS (as an internal standard); or using, as a solvent, CDCl₃ (δ = 7.26 ppm) containing 0.1% TMS (as an internal standard); or using, as a solvent, DMSO-*d*₆ (δ = 2.50 ppm) containing 0.03% TMS (as an internal standard). LC-MS spectra were recorded on a Sciex API 2000 mass spectrometer equipped with an Agilent 1100 binary pump, DAD and ELSD detectors, or on an Agilent 1260-6125B single quadrupole LC-MS system equipped with an Agilent 1260 quaternary pump, DAD and ELSD detectors. HPLC preparative purification was performed using a SHIMADZU LC-20AP system. HPLC purity was determined using either a SHIMADZU LC-30AP or Waters 1525 system. All reactions were carried out under ambient atmosphere unless otherwise specified. Reaction progress was monitored by TLC or LC-MS.

Solvents and reagents are processed as follows:
the solvents used in the reactions such as DCM, DMF, NMP, anhydrous EtOH, and anhydrous MeOH were commercially available;
preparative HPLC was performed using HPLC-grade CH₃CN and deionized water.
all other reactants, reagents and chemicals were commercially available, unless otherwise specified, or were synthesized using methods known in the art.
Unless otherwise specified, the materials and reagents used in the following examples are commercially available and used directly, or can be synthesized by using methods known in the art.

### General synthesis methods

Compounds and/or pharmaceutically acceptable salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, racemates, enantiomers, phosphates, sulfates, hydrochlorides and prodrug forms of the compounds of Formula (I) and Formula (II) of the present disclosure according to routine techniques in the art.

### General synthesis method 1 for intermediate compounds:

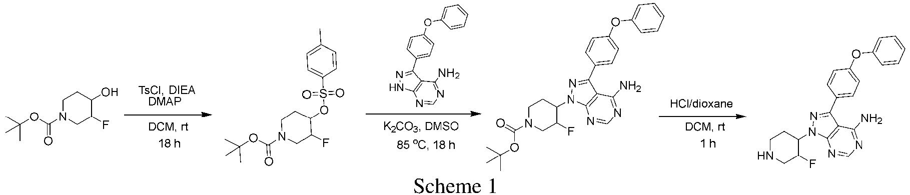

### General synthesis method 2 for intermediate compounds:

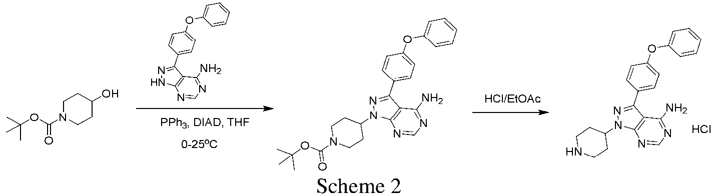

### General synthesis method 3 for intermediate compounds:

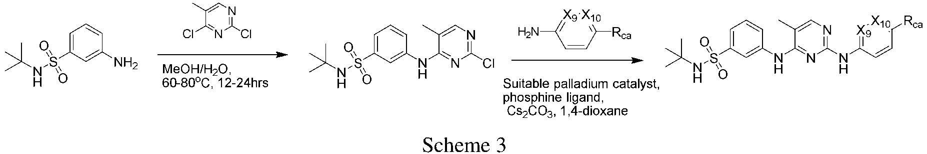

In Scheme 3, X₉ and X₁₀ of the amine substrate used in Step 2 are the same or different and each independently represent N or CH. R_{ca} is a monovalent group corresponding to the divalent group R_{c} of the compound of Formula (I). For example, R_{ca} represents -OH, -NH(R_{d}), -C(O)NH₂, -N(R_{d})-R_{f}-NH(Rₑ), -R_{f}-C(O)NH₂, -R_{f}-C(O)OH, -C(O)OH, -R_{f}-H, -R_{f}-NH(R_{d}), -O-R_{f}-NH(R_{d}), 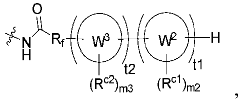 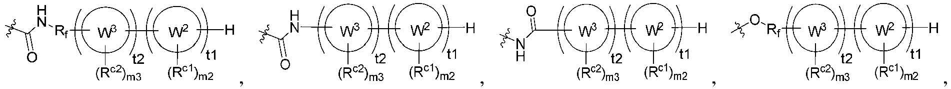 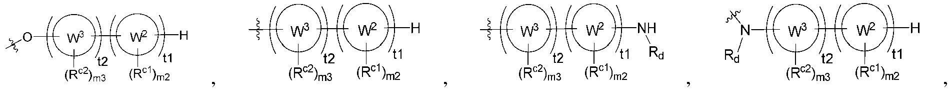 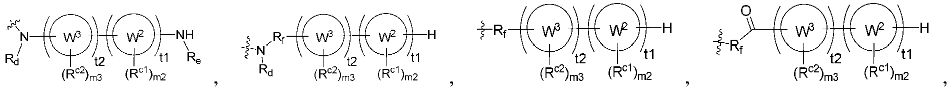 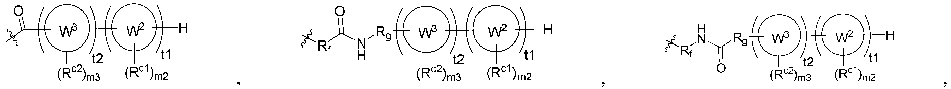 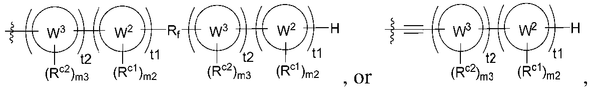 wherein Rₐ, R_{c}, R_{f}, R_{g}, R^{c1}, R^{c2}, ring W², ring W³, t1, t2, m2, and m3 are as defined in the compound of Formula (I) and its various embodiments in the present disclosure. It should be understood that when the group R_{ca} contains reactive hydrogen that can participate in the reaction of Step 2, the reactive hydrogen can be protected by techniques and methods known to those skilled in the art, such as using protecting groups. For example, when R_{ca} represents a piperidinyl or piperazinyl group, conventional protecting groups such as Boc can be used to protect the hydrogen on the N. The removal of the protecting group can be achieved by techniques and methods known to those skilled in the art. For instance, the removal of the Boc protecting group can be accomplished under acidic conditions such as hydrochloric acid or trifluoroacetic acid.

The palladium catalyst and phosphine ligand used in Step 2 of Scheme 3 can be conventional ones suitable for coupling reactions. There are many types of conventional palladium catalysts used for coupling reactions, such as palladium acetate (Pd(OAc)₂), tris(dibenzylideneacetone)dipalladium (Pd₂(dba)₃), diphenylphosphinoferrocene palladium dichloride, tetraphenylphosphine palladium, dichlorobis(triphenylphosphine) palladium, palladium on carbon, and so on. The phosphine ligand can be a conventional one like Xantphos or BINAP.

In Step 1 of Scheme 3, to a solution of 3-amino-N-(tert-butyl)benzenesulfonamide (9.50 g, 41.608 mmol) in MeOH/H₂O (400 mL) was added 2,4-dichloro-5-methylpyrimidine (6.78 g, 41.608 mmol), and the reaction was stirred at 80°C for 18 hours. After monitoring the reaction via LCMS and confirming its completion, the reaction mixture was cooled to room temperature, resulting in the precipitation of a large amount of white solid. The reaction mixture was filtered, and the filter cake was washed with methanol/water (50 mL, 1:1), dried under high vacuum at 50°C, yielding a white solid compound N-(tert-butyl)-3-((2-chloro-5-methylpyrimidin-4-yl)amino)benzenesulfonamide (8.00 g, 22.544 mmol, 54.18%). LCMS (ESI): calcd for (M) 354.09, found, (M+H) ⁺ 355.10.

In Step 2 of Scheme 3, to a solution of N-(tert-butyl)-3-((2-chloro-5-methylpyrimidin-4-yl)amino)benzenesulfonamide (1 equiv) and the corresponding amine substrate (1.3 equiv) in dioxane (50 mL) were added Cs₂CO₃ and a suitable palladium catalyst/phosphine ligand. The reaction was stirred at 100°C for 4 hours. The reaction mixture was diluted with ethyl acetate and saturated NaCl solution, dried over Na₂SO₄, and concentrated under vacuum to obtain the crude product. The crude product was purified by silica gel flash column chromatography (eluent (v/v): EtOAc/PE = 0/1-1/4) to give the target compound.

### General synthesis method 4 of intermediate compounds:

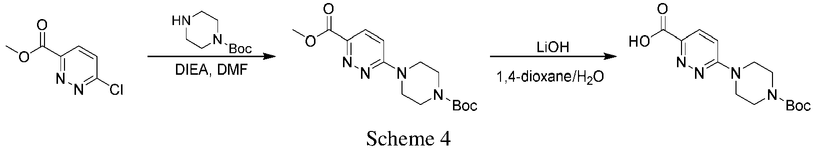

### General synthesis method 5 of intermediate compounds:

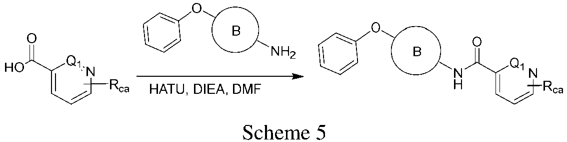

In Scheme 5, ring B of the amine substrate and Q₁ of the acid substrate are defined in Formula (PBM-6-1A) and its various embodiments in the present disclosure. The R_{ca} of the acid substrate used is a monovalent group corresponding to the divalent group R_{c} of the compound of Formula (I). For example, R_{ca} represents -OH, -NH(R_{d}), -C(O)NH₂, -N(R_{d})-R_{f}-NH(R_{c}), -R_{f}-C(O)NH₂, -R_{f}-C(O)OH, -C(O)OH, -R_{f}-H, -R_{f}-NH(R_{d}), -O-R_{f}-NH(R_{d}), 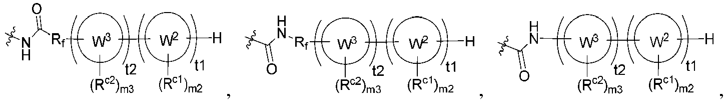 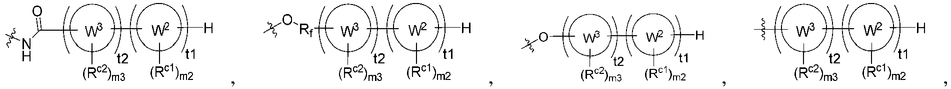 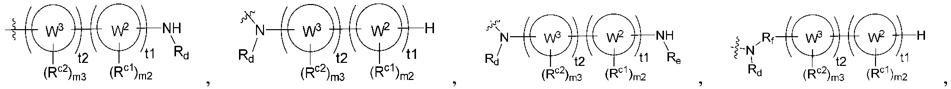 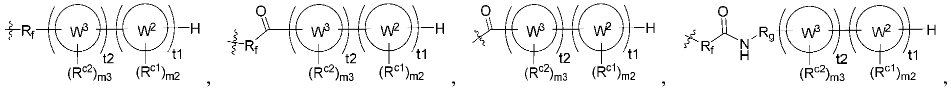 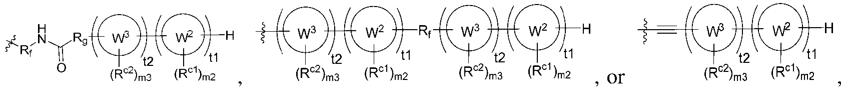 wherein R_{d}, R_{c}, R_{f}, R_{g}, R^{c1}, R^{c2}, ring W², ring W³, t1, t2, m2, and m3 are as defined in the compound of Formula (I) and its various embodiments in the present disclosure. It should be understood that when the group R_{ca} contains an active hydrogen or reactive group that can participate in the reaction of Scheme 5, the active hydrogen or reactive group can be protected by techniques and methods well known to those skilled in the art, such as protective groups. For example, when R_{ca} represents a piperidinyl or piperazinyl group, conventional protective groups such as Boc can be used to protect the hydrogen on N. The removal of the protective group can be achieved by techniques and methods well known to those skilled in the art, such as removing the protective group Boc under acidic conditions such as hydrochloric acid or trifluoroacetic acid.

In Scheme 5, to a solution of the acid substrate (1 equiv) and amine substrate (1 equiv) in DMF were added HATU (1.5 equiv) and DIEA (5 equiv). The reaction mixture was then allowed to react at room temperature for 30 minutes. After monitoring the reaction via LCMS and confirming its completion, water was added to the reaction mixture, and the resulting mixture was extracted with ethyl acetate. The organic phase was washed with saturated saline and concentrated to obtain the crude product. The crude product was purified using a chromatography column (eluent (v/v): DCM/MeOH=100/1) to give the target compound.

### General synthesis method of intermediates (JQ-1 derivatives):

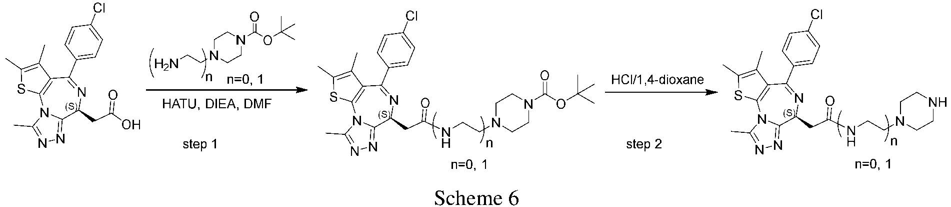

### Step 1:

To a solution of (S)-2-(4-(4-chlorophenyl)-2,3,9-trimethyl-6H-thieno[3,2-f][1,2,4]triazolo[4,3-a] [1,4]diazepin-6-yl)acetic acid (1 equiv), the corresponding amine substrate (1.2 equiv) and HATU (2 equiv) in 10 mL of DCM was added DIEA (5 equiv), and the resulting reaction mixture was allowed to react at room temperature for 1 hour. The reaction solution was washed twice with 10 mL of water and concentrated under reduced pressure to remove the solvent. The resulting residue was purified by silica gel column chromatography (eluent (v/v): DCM to DCM/MeOH (10/1)) to give the Boc intermediate compound.

### Step 2:

The Boc intermediate compound obtained in Step 1 was dissolve in HCl/1,4-dioxane, and the resulting reaction mixture was allowed to react at room temperature for 1 hour. The reaction solution was concentrated under reduced pressure to remove the solvent to give the target compound.

### General synthesis method 6 of intermediate compounds:

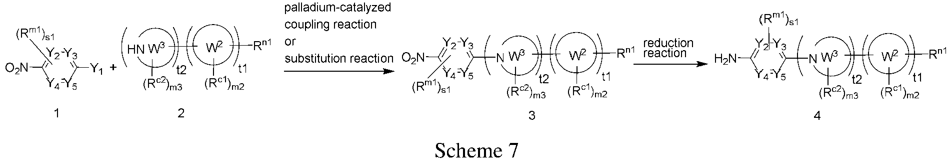

**In** Scheme 7, (R^{m1})ₛ₁ of compound 1 indicates that the six-membered ring to which it is attached is optionally substituted with s1 R^{m1} groups, with each R^{m1} being the same or different and independently representing halogen, hydroxy, C₁₋₆ alkyl (such as methyl or ethyl), halo C₁₋₆ alkyl, deuterated C₁₋₆ alkyl, C₁₋₆ alkoxy (such as methoxy or isopropoxy), halo C₁₋₆ alkoxy, deuterated C₁₋₆ alkoxy, NO₂, NH₂, or cyano. s1 represents an integer of 0, 1, 2, 3, or 4. Y₁ represents halogen (such as fluorine, chlorine, or bromine), and Y₂, Y₃, Y₄, and Y₅ each independently represent N or CH, with the provision that Y₂, Y₃, Y₄, and Y₅ are not all N simultaneously. Ring W², ring W³, t1, t2, (R^{c1})ₘ₂, and (R^{c2})ₘ₃ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W²-Rⁿ¹ contains additional reactive groups (such as -NH- or -NH₂) that can participate in the reaction of Scheme 7, the reactive groups can be protected by techniques and methods known to those skilled in the art, such as using protecting groups.

Depending upon the target compound, the reaction substrate, reaction conditions (including reaction dosage, temperature, time, etc.), and work up, etc. in Scheme 7 can be appropriately modified and adjusted using techniques and methods well known to those skilled in the art to obtain the desired target compound. In Scheme 7, the substitution reaction or palladium-catalyzed coupling reaction of step 1 can be conventional techniques and methods known to those skilled in the art. When step 1 is a substitution reaction, Y₁ represents fluorine, and compounds 1 and 2 react under basic conditions (such as K₂CO₃). When step 1 is a palladium-catalyzed coupling reaction, Y₁ represents bromine, and the palladium catalyst and phosphine ligand used can be conventional ones suitable for coupling reactions. There are many types of conventional palladium catalysts for coupling reactions, such as palladium acetate (Pd(OAc)₂), tris(dibenzylideneacetone)dipalladium (Pd₂(dba)₃), diphenylphosphinoferrocene dichloropalladium, tetraphenylphosphine palladium, dichlorobis(triphenylphosphine)palladium, palladium on carbon, etc. The phosphine ligand can be a conventional ligand such as Xantphos or BINAP. The reduction reaction of step 2 in Scheme 7 can be conventional techniques and methods known to those skilled in the art, such as H₂/Pd/C or Fe/NH₄Cl/MeOH.

### General synthesis method 7 of intermediate compounds:

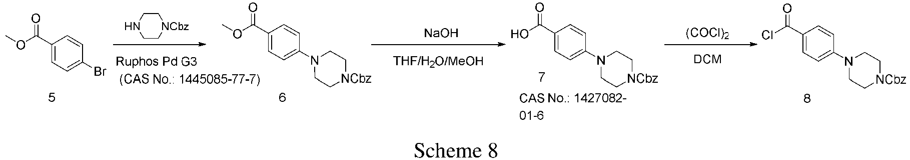

### General synthesis method 8 of intermediate compounds:

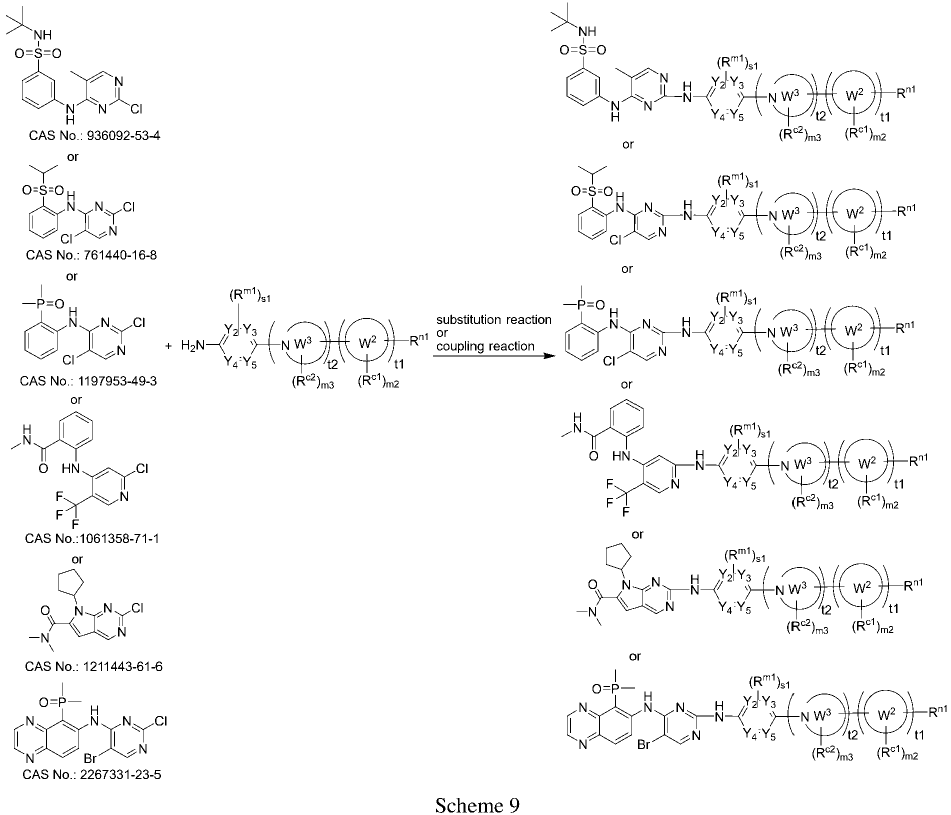

**In** Scheme 9, (R^{m1})ₛ₁ of the compounds indicate that the six-membered ring to which it is attached is optionally substituted with s1 R^{m1} groups, with each R^{m1} being the same or different and independently representing halogen, hydroxy, C₁₋₆ alkyl (such as methyl or ethyl), halo C₁₋₆ alkyl, deuterated C₁₋₆ alkyl, C₁₋₆ alkoxy (such as methoxy or isopropoxy), halo C₁₋₆ alkoxy, deuterated C₁₋₆ alkoxy, NO₂, NH₂, or cyano. Y₁ represents halogen (such as fluorine, chlorine, or bromine), and Y₂, Y₃, Y₄, and Y₅ each independently represent N or CH, with the provision that Y₂, Y₃, Y₄, and Y₅ are not all N simultaneously. Ring W², ring W³, t1, t2, (R^{c1})ₘ₂, and (R^{c2})ₘ₃ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W²-Rⁿ¹ contains additional reactive groups (such as -NH- or -NH₂) that can participate in the reaction of Scheme 9, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

Depending upon the target compound, the reaction substrate, reaction conditions (including reaction dosage, temperature, time, etc.), and work up, etc. in Scheme 9 can be appropriately modified and adjusted using techniques and methods well known to those skilled in the art to obtain the desired target compound. In Scheme 9, the substitution reaction or coupling reaction can be conventional techniques and methods known to those skilled in the art. For example, substitution reactions can be carried out under heating conditions in the presence of triethylamine/isopropanol, p-toluenesulfonic acid/n-butanol, DIEA/ethanol, NaHMDS, or NaI/DIEA/DMF; coupling reactions can be performed under heating conditions in the presence of Brettphos Pd G3/Brettphos ligand/Cs₂CO₃.

### General synthesis method 9 of intermediate compounds:

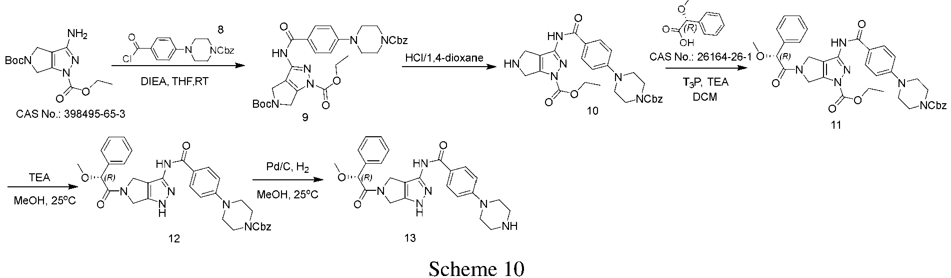

The compound 8 in Step 1 of Scheme 10 can be prepared by referring to Scheme 8.

### General synthesis method 10 of intermediate compounds:

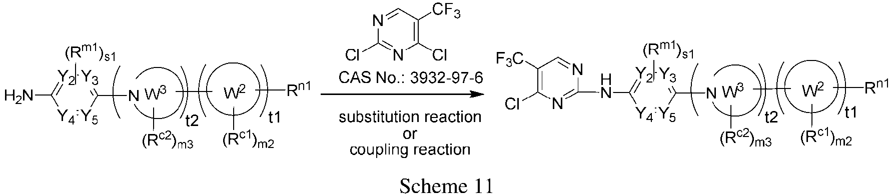

**In** Scheme 11, (R^{m1})ₛ₁ of the compounds indicates that the benzene ring is optionally substituted with s1 R^{m1} groups, with each R^{m1} being the same or different and independently representing halogen, hydroxy, C₁₋₆ alkyl (such as methyl or ethyl), halo C₁₋₆ alkyl, deuterated C₁₋₆ alkyl, C₁₋₆ alkoxy (such as methoxy or isopropoxy), halo C₁₋₆ alkoxy, deuterated C₁₋₆ alkoxy, NO₂, NH₂, or cyano. s1 represents an integer of 0, 1, 2, 3, or 4. Y₁ represents halogen (such as fluorine, chlorine, or bromine), and Y₂, Y₃, Y₄, and Y₅ each independently represent N or CH, with the proviso that Y₂, Y₃, Y₄, and Y₅ are not all N at the same time. Rings W², W³, t1, t2, (R^{c1})ₘ₂, (R^{c2})ₘ₃ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W²-Rⁿ¹ contains additional reactive groups (such as -NH- or -NH₂) that can participate in the reaction of Scheme 11, the reactive groups can be protected by techniques and methods known to those skilled in the art, such as using protecting groups.

Depending upon the target compound, the reaction substrate, reaction conditions (including reaction dosage, temperature, time, etc.), and work up, etc. in Scheme 11 can be appropriately modified and adjusted using techniques and methods well known to those skilled in the art to obtain the desired target compound. In Scheme 11, the substitution reaction or coupling reaction can be conventional techniques and methods known to those skilled in the art. For example, substitution reactions can be carried out under heating conditions in the presence of triethylamine/isopropanol, DIEA/ethanol, NaHMDS, or NaI/DIEA/DMF; coupling reactions can be performed under heating conditions in the presence of Brettphos Pd G3/Brettphos ligand/Cs₂CO₃.

### General synthesis method 11 of intermediate compounds:

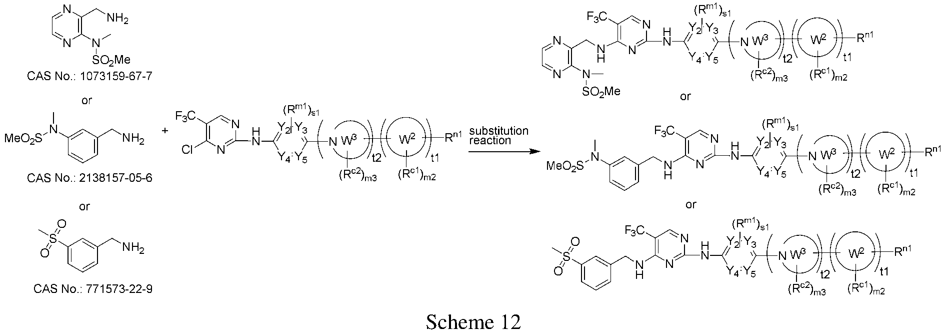

In Scheme 12, (R^{m1})ₛ₁ of the compounds indicates that the benzene ring is optionally substituted with s1 R^{m1} groups, with each R^{m1} being the same or different and independently representing halogen, hydroxy, C₁₋₆ alkyl (such as methyl or ethyl), halo C₁₋₆ alkyl, deuterated C₁₋₆ alkyl, C₁₋₆ alkoxy (such as methoxy or isopropoxy), halo C₁₋₆ alkoxy, deuterated C₁₋₆ alkoxy, NO₂, NH₂, or cyano. Y₁ represents halogen (such as fluorine, chlorine, or bromine), and Y₂, Y₃, Y₄, and Y₅ each independently represent N or CH, with the proviso that Y₂, Y₃, Y₄, and Y₅ are not all N at the same time. Rings W², W³, t1, t2, (R^{c1})ₘ₂, (R^{c2})ₘ₃ are as defined in the compound of Formula (II) of the present disclosure. Rⁿ¹ represents Boc, H, or -NHRₕ, where Rₕ represents hydrogen or C₁₋₆ alkyl (such as methyl or ethyl). It should be understood that when ring W²-Rⁿ¹ contains additional reactive groups (such as -NH- or -NH₂) that can participate in the reaction of Scheme 12, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

Depending upon the target compound, the reaction substrate, reaction conditions (including reaction dosage, temperature, time, etc.), and work up, etc. in Scheme 12 can be appropriately modified and adjusted using techniques and methods well known to those skilled in the art to obtain the desired target compound. In Scheme 12, the substitution reaction can be conventional techniques and methods known to those skilled in the art. For example, substitution reactions can be carried out under heating conditions in the presence of triethylamine/isopropanol, DIEA/ethanol, or NaI/DIEA/DMF.

### General synthesis method 12 of intermediate compounds:

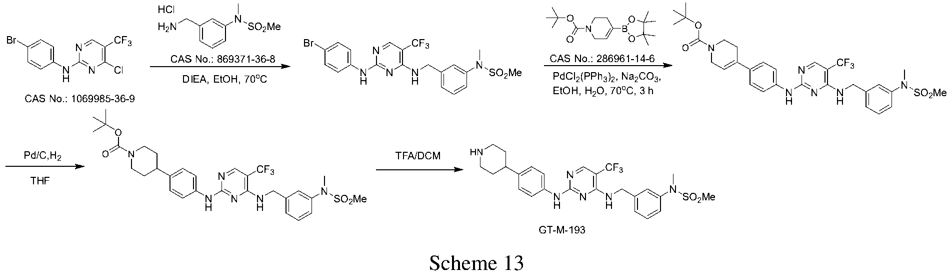

### General synthesis method 13 of intermediate compounds:

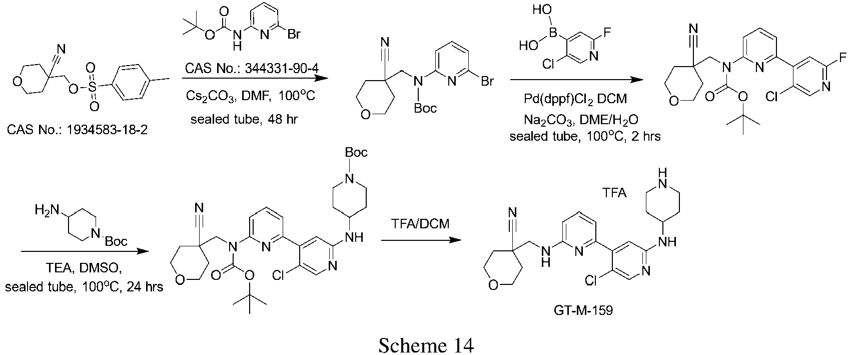

### General synthesis method 14 of intermediate compounds:

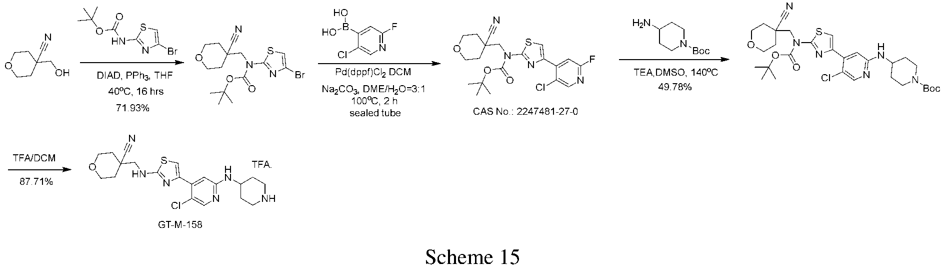

### General synthesis method 15 of intermediate compounds:

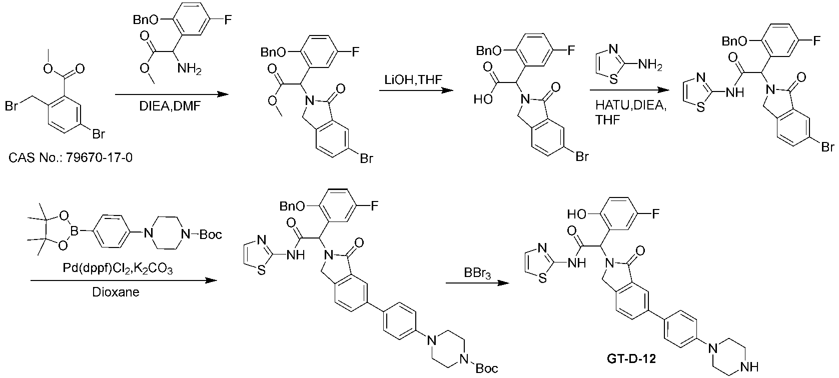

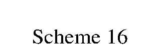

### General synthesis method 16 of intermediate compounds:

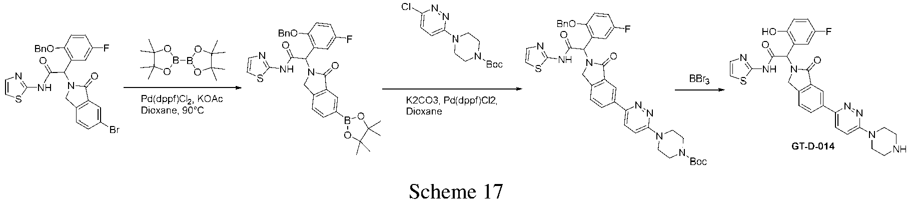

### General synthesis method 17 of intermediate compounds:

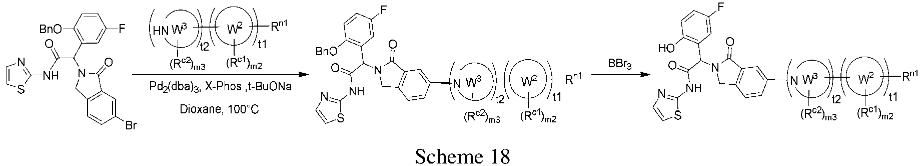

In Scheme 18, the rings W², rings W³, t1, t2, (R^{c1})ₘ₂, (R^{c2})ₘ₃ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W²-Rⁿ¹ contains additional reactive groups (such as -NH- or -NH₂) that can participate in the reaction of Scheme 18, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

Depending upon the target compound, the reaction substrate, reaction conditions (including reaction dosage, temperature, time, etc.), and work up, etc. in Scheme 18 can be appropriately modified and adjusted using techniques and methods well known to those skilled in the art to obtain the desired target compound.

### General synthesis method 18 of intermediate compounds:

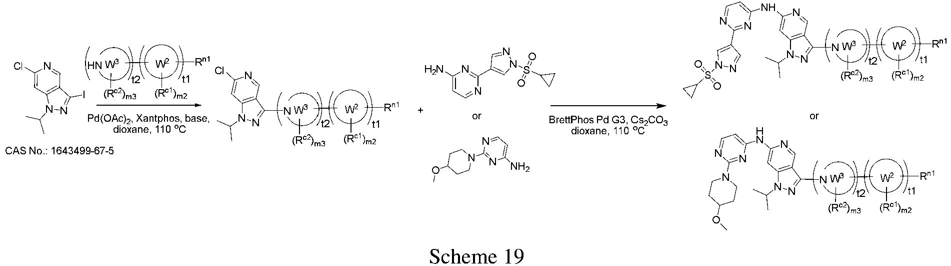

In Scheme 19, the rings W², rings W³, t1, t2, (R^{c1})ₘ₂, (R^{c2})ₘ₃ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W²-Rⁿ¹ contains additional reactive groups (such as -NH- or -NH₂) that can participate in the reaction of Scheme 19, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

### General synthesis method 19 of intermediate compounds:

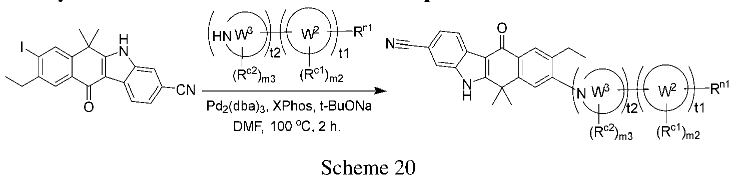

In Scheme 20, the rings W², rings W³, t1, t2, (R^{c1})ₘ₂, (R^{c2})ₘ₃ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W²-Rⁿ¹ contains additional reactive groups (such as -NH- or -NH₂) that can participate in the reaction of Scheme 20, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

### General synthesis method 20 of intermediate compounds:

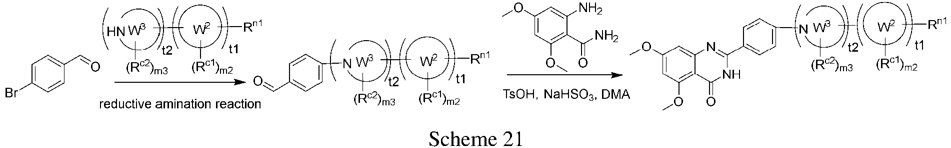

**In** Scheme 21, the rings W², rings W³, t1, t2, (R^{c1})ₘ₂, (R^{c2})ₘ₃ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W⁶-Rⁿ¹ contains additional reactive groups (such as -NH- or -NH₂) that can participate in the reaction of Scheme 21, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

Depending upon the target compound, the reaction substrate, reaction conditions (including reaction dosage, temperature, time, etc.), and work up, etc. in Scheme 21 can be appropriately modified and adjusted using techniques and methods well known to those skilled in the art to obtain the desired target compound. In Scheme 21, the reductive amination reaction can follow conventional techniques and methods known to the skilled in the art. For example, the reductive amination reaction can be carried out at room temperature in the presence of sodium triacetoxyborohydride/1,2-dichloroethane, or sodium cyanoborohydride/1,2-dichloroethane.

### General synthesis method 21 of intermediate compounds:

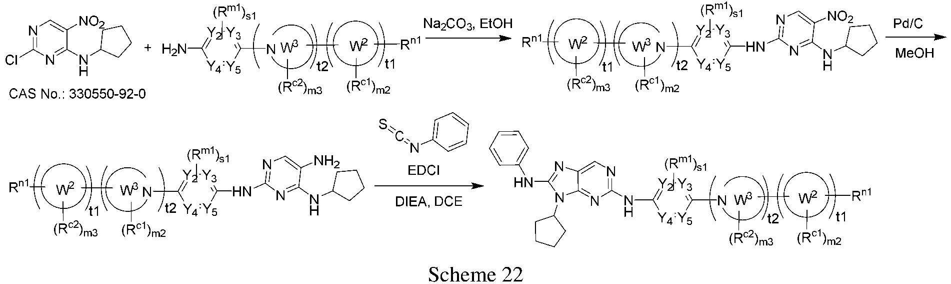

In Scheme 22, (R^{m1})ₛ₁ of compounds indicates that the benzene ring is optionally substituted with s1 R^{m1} groups, with each R^{m1} being the same or different and independently representing halogen, hydroxy, C₁₋₆ alkyl (such as methyl or ethyl), halo C₁₋₆ alkyl, deuterated C₁₋₆ alkyl, C₁₋₆ alkoxy (such as methoxy or isopropoxy), halo C₁₋₆ alkoxy, deuterated C₁₋₆ alkoxy, NO₂, NH₂, or cyano. Y₁ represents halogen (such as fluorine, chlorine, or bromine), and Y₂, Y₃, Y₄, and Y₅ each independently represent N or CH, with the provision that Y₂, Y₃, Y₄, and Y₅ are not all N simultaneously. Rings W², ring W³, t1, t2, (R^{c1})ₘ₂, (R^{c2})ₘ₃ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W²-Rⁿ¹ contains additional reactive groups (such as -NH- or -NH₂) that can participate in the reaction of Scheme 22, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

### General synthesis method 22 of intermediate compounds:

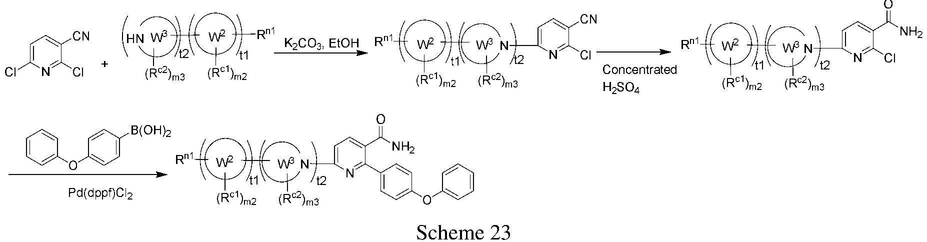

**In** Scheme 23, the rings W², W³, t1, t2, (R^{c1})ₘ₂, (R^{c2})ₘ₃ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W²-Rⁿ¹ of the strating material of step 1 of Scheme 23 contains additional reactive groups (such as -NH- or -NH₂) that can participate in the reaction of step 1, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions (such as concentrated sulfuric acid, etc.) in Step 2.

### General synthesis method 23 of intermediate compounds:

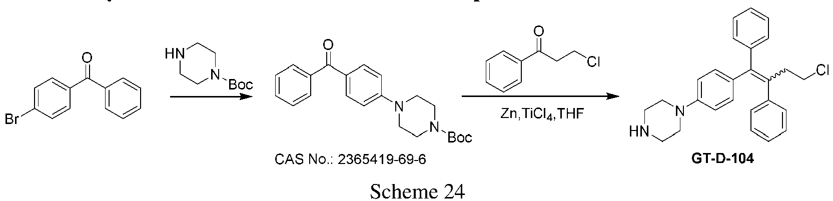

### General synthesis method 24 of intermediate compounds:

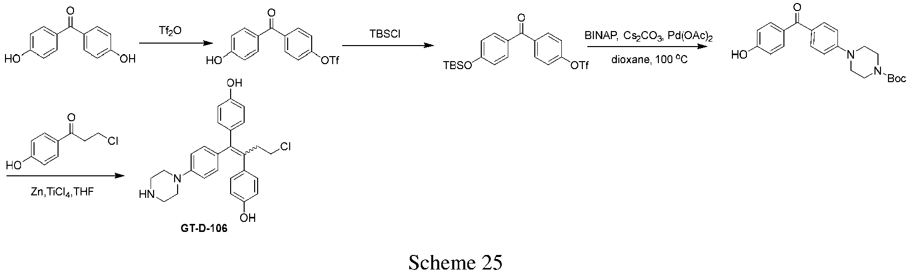

### General synthesis method 25 of intermediate compounds:

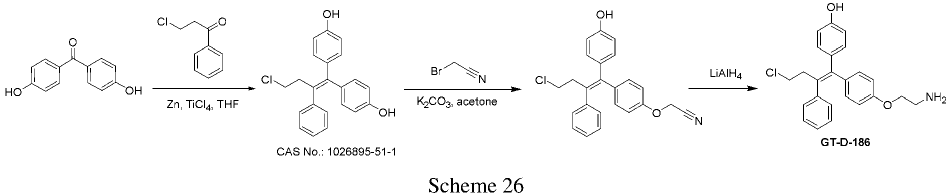

### General synthesis method 26 of intermediate compounds:

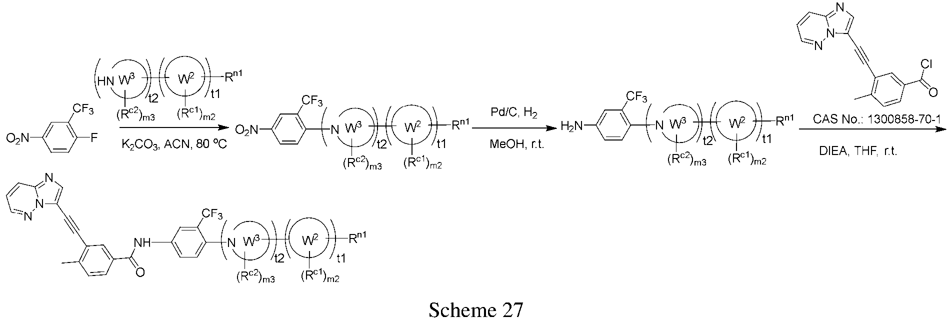

In Scheme 27, the rings W², W³, t1, t2, (R^{c1})ₘ₂, (R^{c2})ₘ₃ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W²-Rⁿ¹ contains additional reactive groups (such as -NH- or -NH₂) that can participate in the reaction, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions (such as concentrated sulfuric acid, etc.).

### General synthesis method 27 of intermediate compounds:

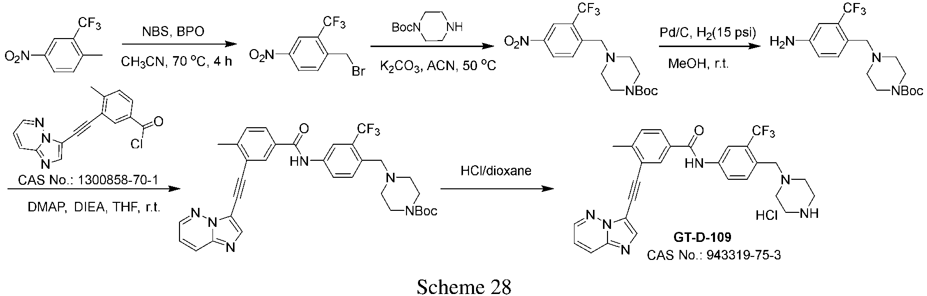

### General synthesis method 28 of intermediate compounds:

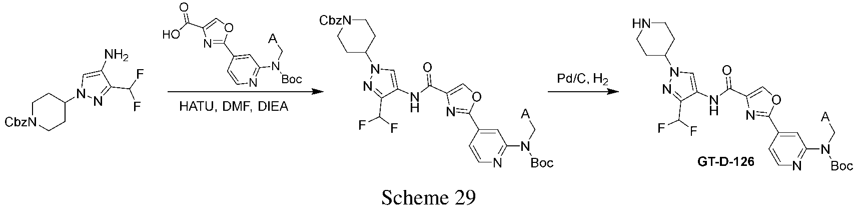

**In** Scheme 29, A represents cyclopropyl or trifluoromethyl (CF₃).

### General synthesis method 29 of intermediate compounds:

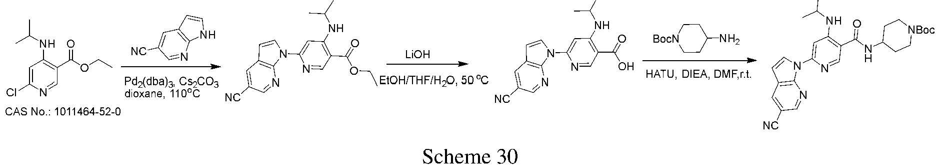

The removal of protecting groups can be achieved through techniques and methods known to those skilled in the art. For example, the removal of the Boc protecting group can be achieved under acidic conditions such as concentrated sulfuric acid.

### General synthesis method 30 of intermediate compounds:

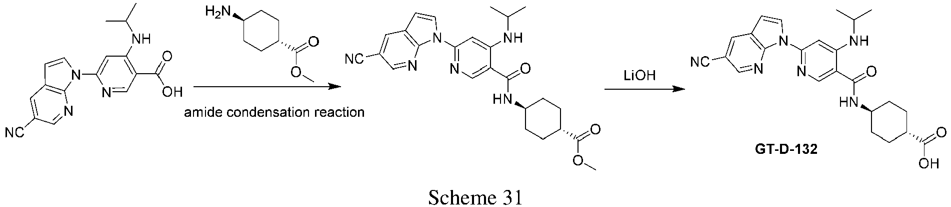

Depending upon the target compound, the reaction substrate, reaction conditions (including reaction dosage, temperature, time, etc.), and work up, etc. in Scheme 31 can be appropriately modified and adjusted using techniques and methods well known to those skilled in the art to obtain the desired target compound. In Scheme 31, the amide condensation reaction can be conventional techniques and methods known to those skilled in the art. For example, the amide condensation reaction can be carried out at room temperature in the presence of HATU/DIEA/DMF or HOAt/EDCI/TEA/DCM.

### General synthesis method 31 of intermediate compounds:

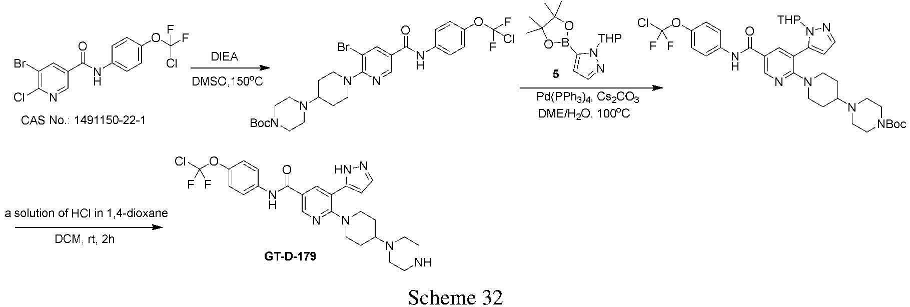

### General synthesis method 32 of intermediate compounds:

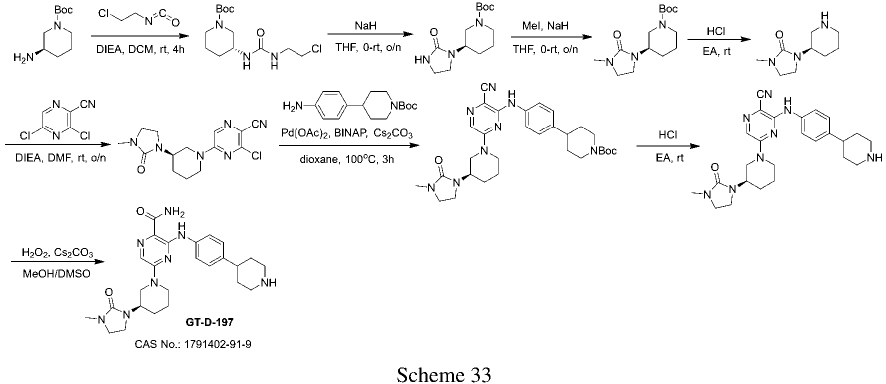

### General synthesis method 33 of intermediate compounds:

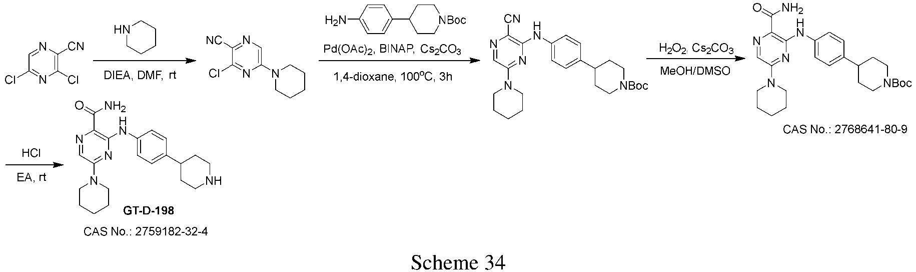

### General synthesis method 34 of intermediate compounds:

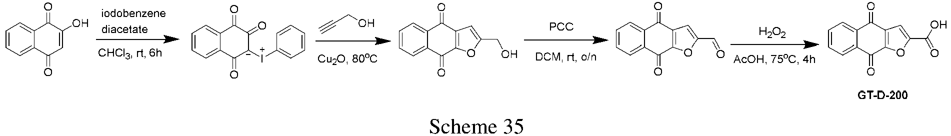

### General synthesis method 35 of intermediate compounds:

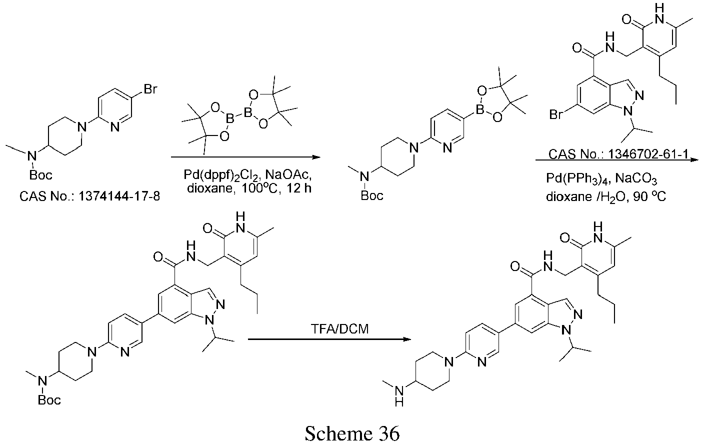

### General synthesis method 36 of intermediate compounds:

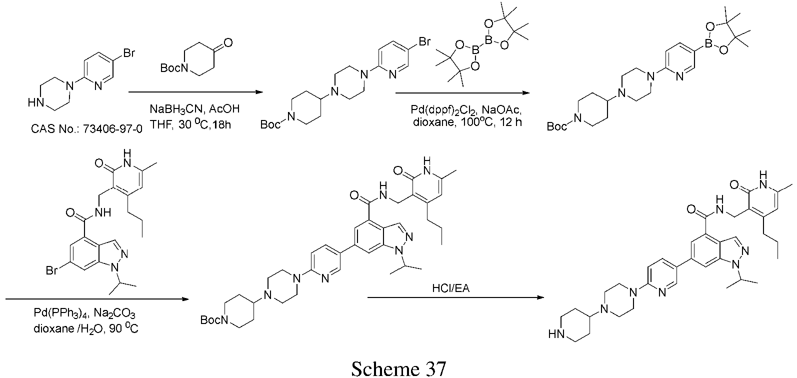

### General synthesis method 37 of intermediate compounds:

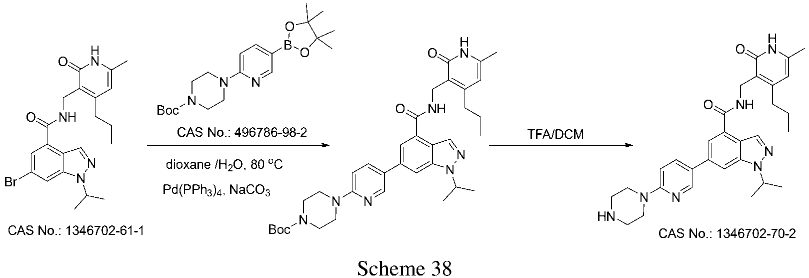

### General synthesis method 38 of intermediate compounds:

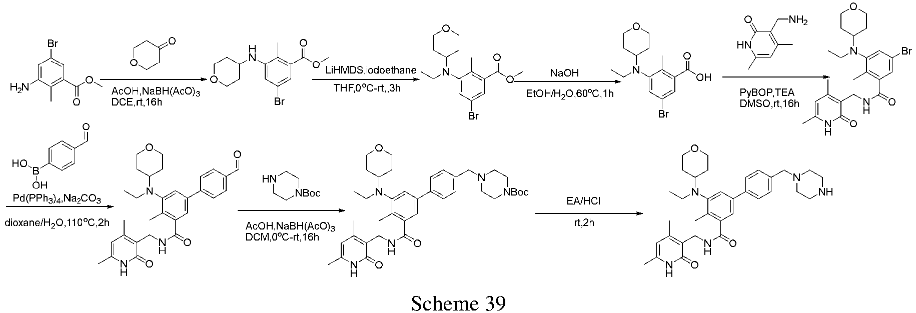

### General synthesis method 39 of intermediate compounds:

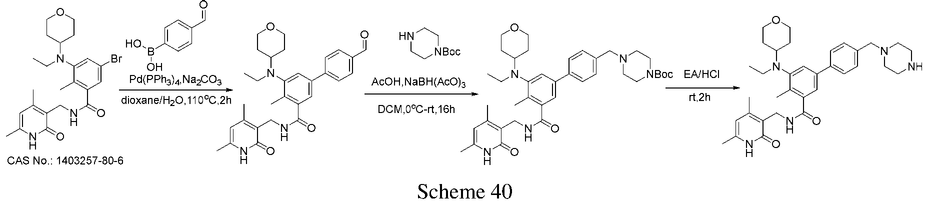

### General synthesis method 40 of intermediate compounds:

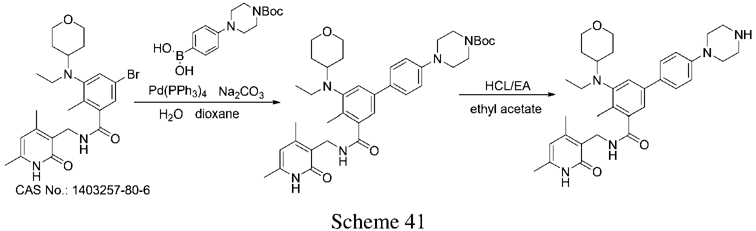

### General synthesis method 41 of intermediate compounds:

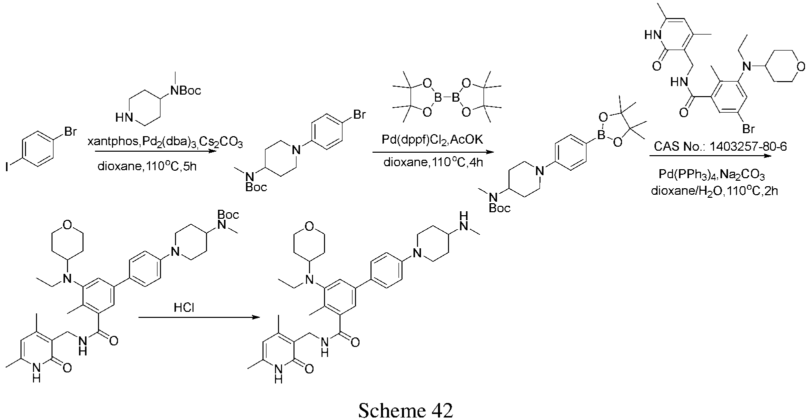

### General synthesis method 42 of intermediate compounds:

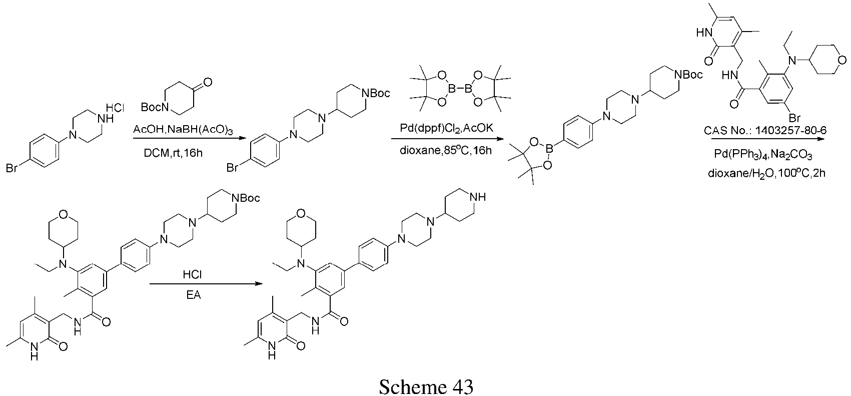

### General synthesis method 43 of intermediate compounds:

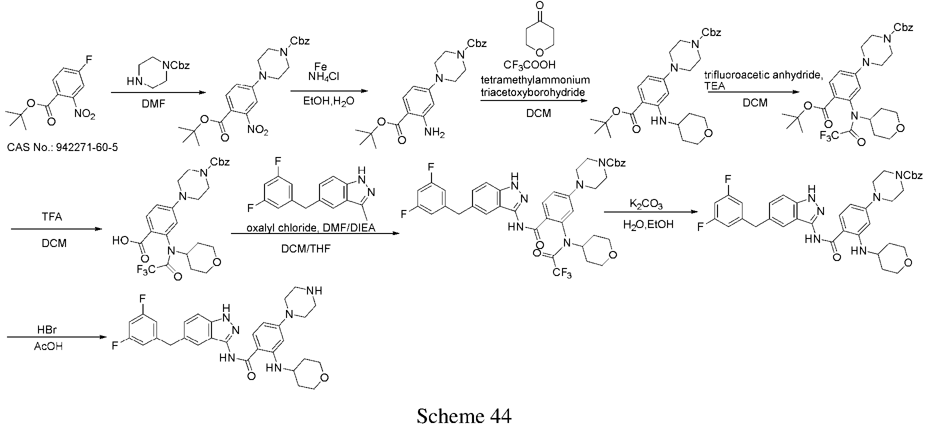

### General synthesis method 44 of intermediate compounds:

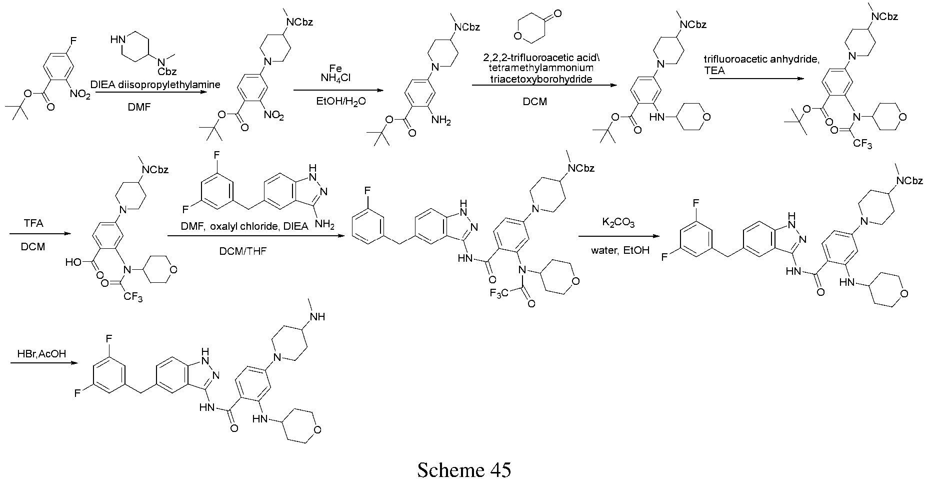

### General synthesis method 45 of intermediate compounds:

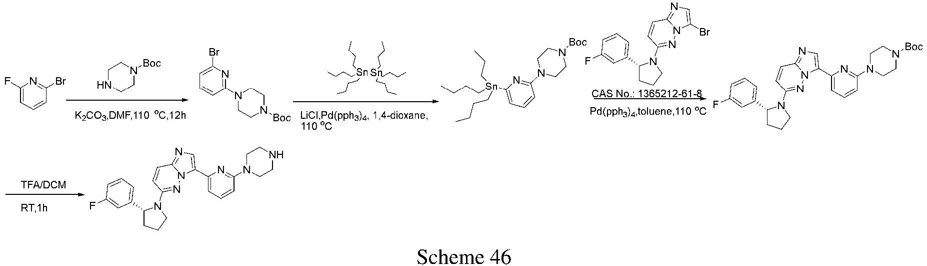

### General synthesis method 46 of intermediate compounds:

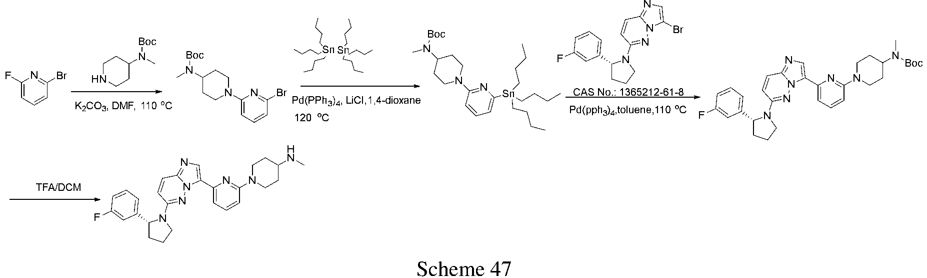

### General synthesis method 47 of intermediate compounds:

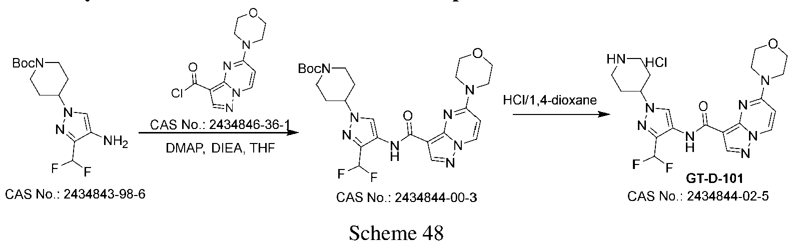

### General synthesis method 48 of intermediate compounds:

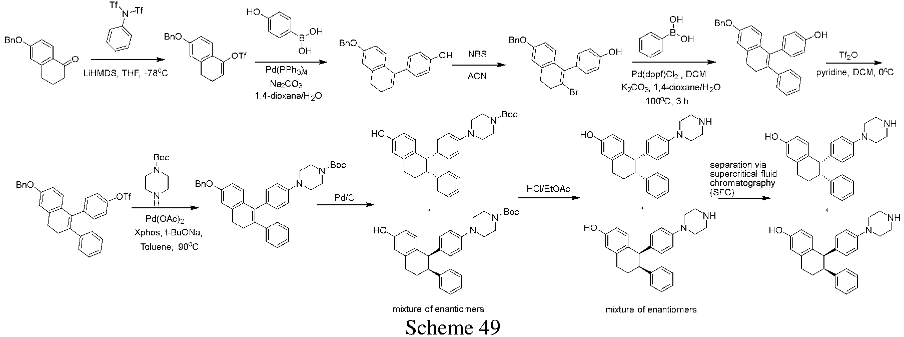

Pure enantiomeric products can be obtained through resolution/separation via supercritical fluid chromatography (SFC) and according to Scheme 49. The conditions of supercritical fluid chromatography (SFC) can be appropriately modified and adjusted using techniques and methods well-known to those skilled in the art to obtain the desired target compound.

### General synthesis method 49 of intermediate compounds:

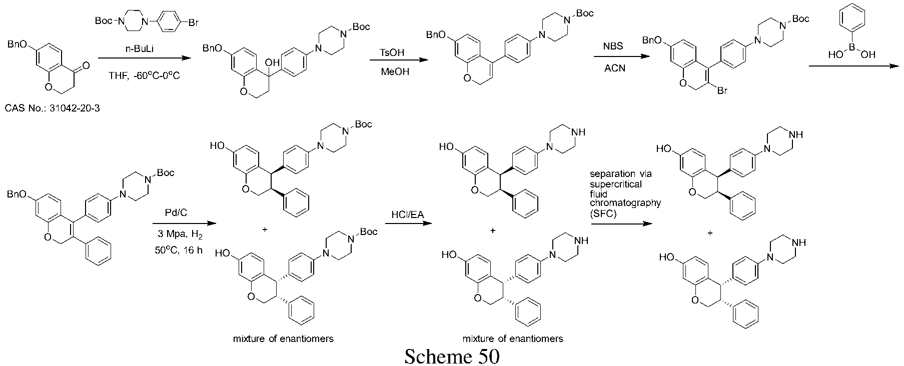

Pure enantiomeric products can be obtained through resolution/separation via supercritical fluid chromatography (SFC) and according to Scheme 50. The conditions of supercritical fluid chromatography (SFC) can be appropriately modified and adjusted using techniques and methods well-known to those skilled in the art to obtain the desired target compound.

### General synthesis method 50 of intermediate compounds:

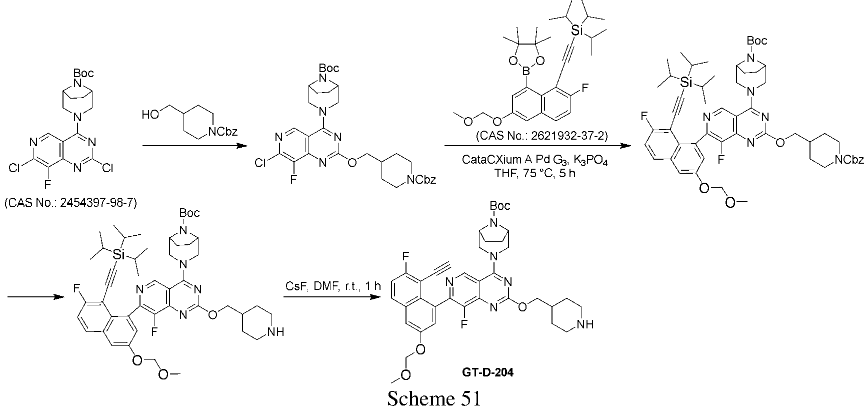

### General synthesis method 51 of intermediate compounds:

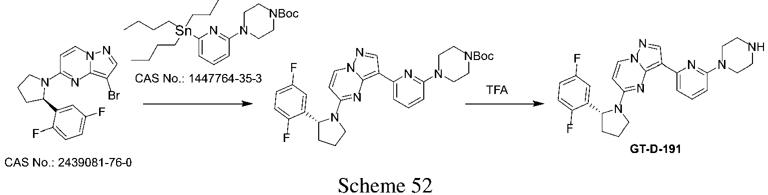

### General synthesis method 52 of intermediate compounds:

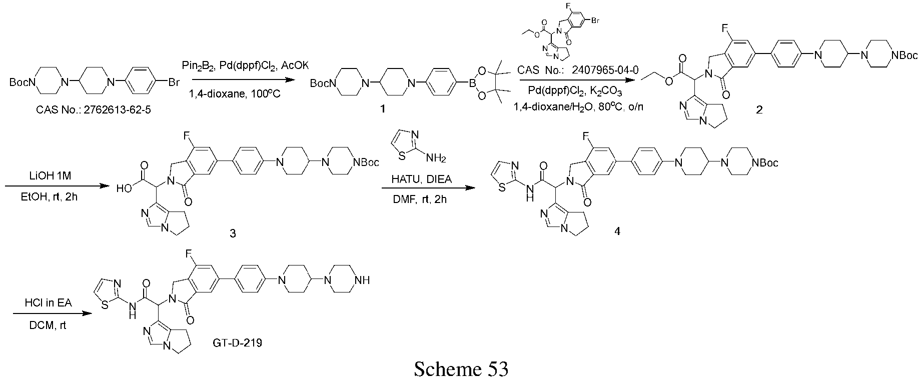

### General synthesis method 53 of intermediate compounds:

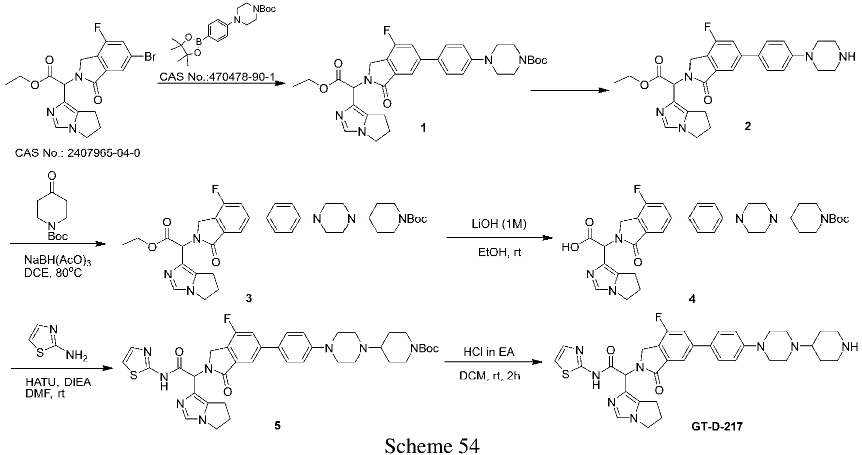

### General synthesis method 54 of intermediate compounds:

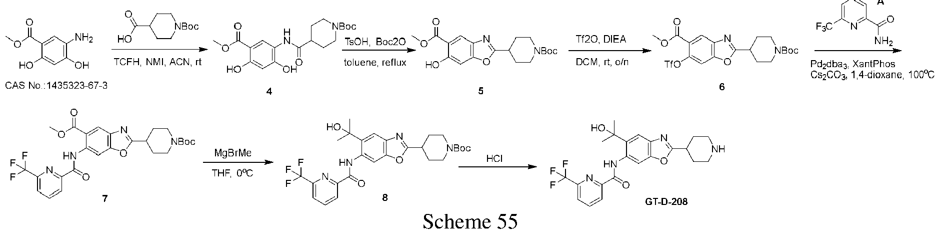

### General synthesis method 55 of intermediate compounds:

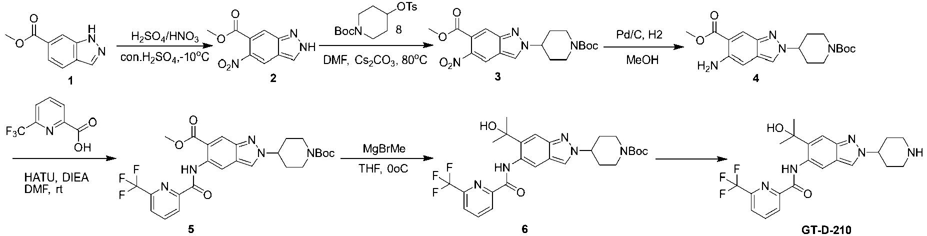

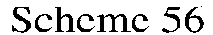

### General synthesis method 56 of intermediate compounds:

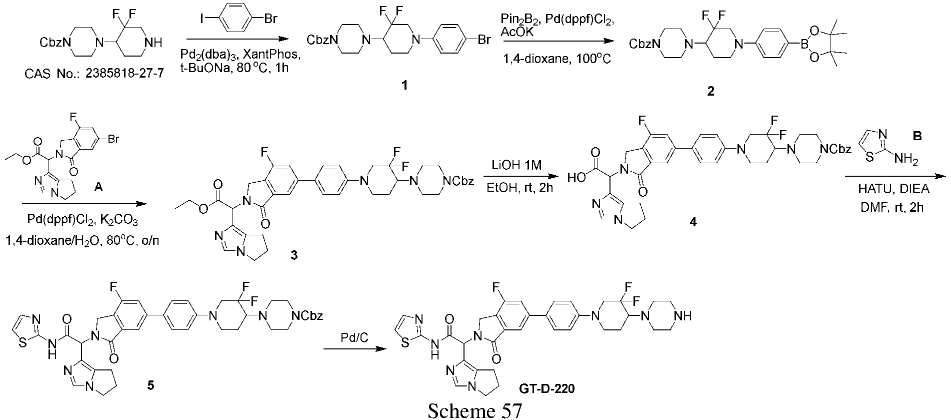

### General synthesis method 1 of compounds of Formula (I):

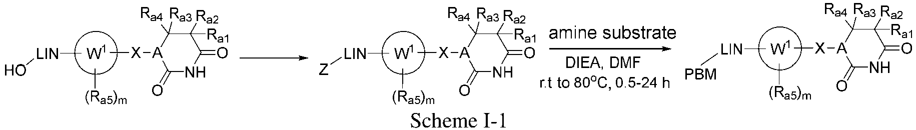

In Step 2 of Scheme I-1, the amine substrate is a compound corresponding to the PBM moiety of the compound of Formula (I) of the present disclosure, containing aliphatic amine or heterocyclic amine (such as nitrogen-containing monocyclic heterocycle, nitrogen-containing bridged heterocycle, nitrogen-containing spiro heterocycle, or nitrogen-containing fused heterocycle) groups or amino compounds. The other substrate has A, Rₐ₁, Rₐ₂, Rₐ₃, Rₐ₄, X, ring W', (Rₐ₅)ₘ, and LIN as defined in the Formula (I) compound herein. The group Z may be Br, Cl, I, OMs, OTs, OTf, or the like. (Rₐ₅)ₘ indicates that the benzene ring to which it is attached is substituted with m Rₐ₅ groups, with each Rₐ₅ being the same or different and independently representing deuterium, fluorine, hydroxyl with a protecting group, mercapto with a protecting group, nitro, amino with a protecting group, cyano, optionally deuterated C₁₋₆ alkyl, optionally deuterated C₁₋₆ alkoxy, halo C₁₋₆ alkyl, optionally substituted C₃₋₆ cycloalkyl, C₂₋₆ alkenyl, or C₂₋₆ alkynyl; m represents an integer of 0, 1, 2, 3, or 4.

Step 1: (1) When the group Z is OMs, OTs, or OTf, the alcohol substrate can undergo esterification with, for example, MsCl (methanesulfonyl chloride), TsCl (p-toluenesulfonyl chloride), or TfCl (trifluoromethanesulfonyl chloride) under basic conditions (such as TEA, DIEA) to obtain the corresponding target intermediate. Alternatively, (2) when the group Z is Br, Cl, or I, the alcohol substrate can undergo halogenation with, for example, CBr₄/PPh₃/DCM to obtain the corresponding target intermediate.

Step 2: to a solution of the amine substrate (1 equiv) and target intermediate (1.2 equiv) from step 1 in DMF were added sodium iodide (1 equiv) and DIEA (5 equiv). The reaction mixture was stirred at room temperature to 80°C for 0.5-24 hours. After monitoring the reaction via LCMS and confirming its completion, the reaction solution was filtered, and the filtrate was separated and purified by preparative HPLC to give the target compound.

### General synthesis method 2 of compounds of Formula (I):

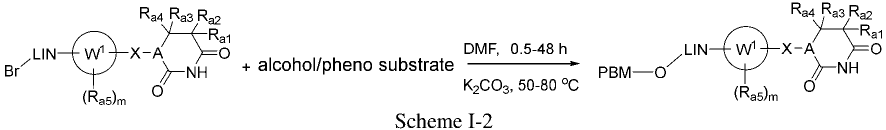

In Scheme I-2, the alcohol/pheno substrate has the formula of PBM-OH. PBM, along with A, Rₐ₁, Rₐ₂, Rₐ₃, Rₐ₄, X, ring W¹, (Rₐ₅)ₘ, and LIN of the other substrate, are as defined in the compound of Formula (I) of the present disclosure.

To a solution of the alcohol substrate (1 equiv) and 3-(5-(bromomethyl)-1-oxoisoindolin-2-yl)piperidine-2,6-dione (1.2 equiv) in DMF was added K₂CO₃ (5 equiv). The reaction mixture was stirred at 50-80°C for 0.5-48 hours. After monitoring the reaction via LCMS and confirming its completion, the reaction solution was filtered, and the filtrate was separated and purified by preparative HPLC to give the target compound.

### General synthesis method 3 of compounds of Formula (I):

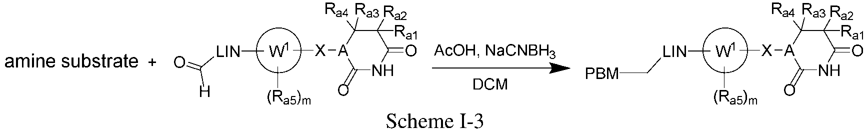

In Scheme I-3, the amine substrate is a compound corresponding to the PBM moiety of the compound of Formula (I) of the present disclosure, where R_{c} contains aliphatic amine or heterocyclic amine (such as nitrogen-containing monocyclic heterocycle, nitrogen-containing bridged heterocycle, nitrogen-containing spiro heterocycle, or nitrogen-containing fused heterocycle) groups or amino groups. PBM, Rₐ₁, Rₐ₂, Rₐ₃, Rₐ₄, X, ring W¹, (Rₐ₅)ₘ, and LIN are as defined in the compound of Formula (I) of the present disclosure. It should be understood that when the LIN group contains additional reactive groups (such as - NH or -NH₂) that can participate in the reaction of Scheme I-3, the reactive groups can be protected by techniques and methods known to those skilled in the art, such as using protecting groups.

To a solution of the corresponding aldehyde or carbonyl compound (1.0 equiv) and amine substrate (1.0 equiv) in dichloromethane was added an appropriate amount of acetic acid. The resulting reaction mixture was reacted at room temperature for 30 minutes, followed by addition of sodium cyanoborohydride (3.0 equiv), and the reaction was continued at room temperature. After monitoring the reaction via LCMS and confirming its completion, the reaction mixture was separated by using C₁₈ reverse phase column chromatography to give the target compound.

### General synthesis method 4 of compounds of Formula (I):

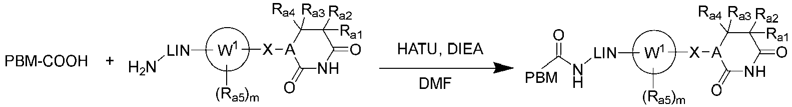

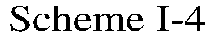

In Scheme I-4, the PBM, A, Rₐ₁, Rₐ₂, Rₐ₃, Rₐ₄, X, ring W¹, (Rₐ₅)ₘ, and LIN of the target product are as defined herein for the Formula (I) compound. The condensation reaction in Scheme I-4 can follow conventional techniques and methods that are well-known to those skilled in the art. For example, it can be carried out using conventional condensing agents such as HOAt/EDCI, T₃P, etc., under alkaline conditions (e.g., in the presence of DMAP) at room temperature or under heating.

### General synthesis method 5 of compounds of Formula (I):

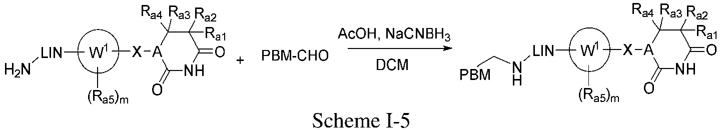

In Scheme I-5, the PBM, A, Rₐ₁, Rₐ₂, Rₐ₃, Rₐ₄, X, ring W¹, (Rₐ₅)ₘ, and LIN of the target product are as defined herein for the Formula (I) compound.

The reductive amination reaction in Scheme I-5 can follow conventional techniques and methods that are known to those skilled in the art. For example, the reductive amination reaction can be carried out at temperatures ranging from room temperature to 80°C in the presence of sodium borohydride acetate and 1,2-dichloroethane.

For example, to a solution of the corresponding aldehyde (1.0 equivalent) and amine (1.0 equivalent) in dichloromethane was added an appropriate amount of acetic acid. The resulting reaction mixture was allowed to react at room temperature for 30 minutes, followed by the addition of sodium cyanoborohydride (3.0 equivalents). The reaction was then continued at room temperature until completion, as monitored by LCMS. After the reaction was complete, the product was separated via C₁₈ reverse-phase column chromatography. The collected fractions were rotary evaporated to remove acetonitrile, and the resulting residue was lyophilized to give the target compound.

### General synthesis method 1 of compounds of Formula (II):

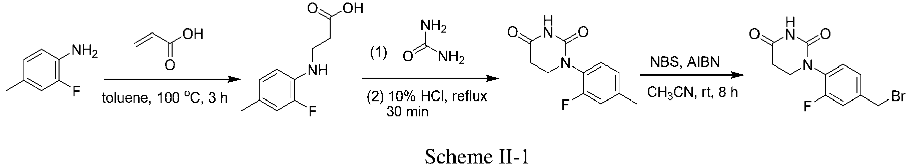

Step 1: 2-Fluoro-4-methylaniline (1.0 eq.) was dissolved in toluene at room temperature, followed by the addition of acrylic acid (3.0 eq.) to the reaction mixture. The air in the reaction vessel was replaced with nitrogen three times. The reaction solution was heated to 100 °C and stirred for 2 h. After monitoring the reaction via LC/MS and confirming its completion, the reaction mixture was cooled to room temperature and concentrated under reduced pressure. The residue was triturated with petroleum ether, filtered, and the collected filter cake was dried under vacuum to afford 3-((2-fluoro-4-methylphenyl)amino)propanoic acid.

Step 2: to a solution of 3-((2-fluoro-4-methylphenyl)amino)propanoic acid (1.0 eq.) and urea (3.0 eq.) in acetic acid was added dilute hydrochloric acid (0.2 eq.) at room temperature. The air in the reaction vessel was replaced with nitrogen three times. The reaction solution was heated to 120 °C and stirred for 3 h. After monitoring the reaction via LC/MS and confirming its completion, the reaction mixture was cooled to room temperature, and hydrochloric acid solution (2.0 eq., 4 M) was added. The solution was heated to 100 °C and stirred for an additional 1 h. After monitoring the reaction via LC/MS and confirming its completion, the reaction mixture was cooled to room temperature and concentrated under reduced pressure. The residue was dissolved in ethyl acetate and washed with water. After phase separation, the aqueous phase was extracted with ethyl acetate. The combined organic phases were washed with saturated brine, dried over anhydrous sodium sulfate, filtered, and concentrated under reduced pressure. The residue was triturated with ethyl acetate/petroleum ether (1/2) and filtered to afford 1-(2-fluoro-4-methylphenyl)dihydropyrimidine-2,4(1H,3H)-dione.

Step 3: to a solution of 1-(2-fluoro-4-methylphenyl)dihydropyrimidine-2,4(1H,3H)-dione (1.0 eq.) in acetonitrile were successively added N-bromosuccinimide (0.9 eq.) and azobisisobutyronitrile (0.09 eq.) at room temperature. The air in the reaction vessel was replaced with nitrogen three times. The reaction solution was stirred at room temperature for 8 h. After monitoring the reaction via LC/MS and confirming its completion, the reaction mixture was concentrated under reduced pressure. The residue was dissolved in ethyl acetate and washed with water. After phase separation, the aqueous phase was extracted with ethyl acetate. The combined organic phases were washed with saturated brine, dried over anhydrous sodium sulfate, filtered, and concentrated under reduced pressure. The residue was triturated with ethyl acetate/petroleum ether (1/2) and filtered to afford the target compound 1-(4-(bromomethyl)-2-fluorophenyl)dihydropyrimidine-2,4(1H,3H)-dione.

### General synthesis method 2 of compounds of Formula (II):

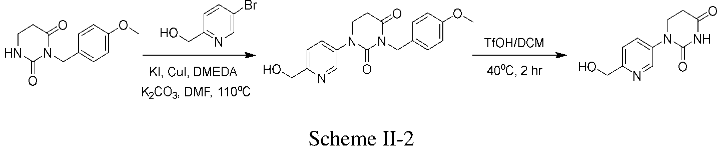

Step 1: 3-(4-Methoxybenzyl)dihydropyrimidine-2,4(1H,3H)-dione (1.0 eq.) and (5-bromopyridin-2-yl)methanol (1.2 eq.) were dissolved in N,N-dimethylformamide at room temperature, followed by the subsequent addition of potassium iodide (2.0 eq.), copper(I) iodide (0.2 eq.), and N,N'-dimethylethylenediamine (2.0 eq.) to the reaction mixture. The air in the reaction vessel was replaced with nitrogen three times. The reaction solution was heated to 110 °C and stirred for 8 h. After monitoring the reaction via LC/MS and confirming its completion, the reaction mixture was cooled to room temperature and concentrated under reduced pressure. The residue was dissolved in ethyl acetate and washed with water. After phase separation, the aqueous phase was extracted with ethyl acetate. The combined organic phases were washed with saturated brine, dried over anhydrous sodium sulfate, filtered, and concentrated under reduced pressure. The residue was triturated with ethyl acetate/petroleum ether (1/2) and filtered to afford 1-(6-(hydroxymethyl)pyridin-3-yl)-3-(4-methoxybenzyl)dihydropyrimidine-2,4(1H,3H)-dione.

Step 2: 1-(6-(Hydroxymethyl)pyridin-3-yl)-3-(4-methoxybenzyl)dihydropyrimidine-2,4(1H,3H)-dione (1.0 eq.) was dissolved in dichloromethane at room temperature, followed by the addition of trifluoromethanesulfonic acid (2.0 eq.) to the reaction mixture. The reaction solution was heated to 40 °C and stirred for 2 h. After monitoring the reaction via LC/MS and confirming its completion, the reaction mixture was cooled to room temperature and concentrated under reduced pressure. The residue was dissolved in ethyl acetate and washed with water. After phase separation, the aqueous phase was extracted with ethyl acetate. The combined organic phases were washed with saturated brine, dried over anhydrous sodium sulfate, filtered, and concentrated under reduced pressure. The residue was triturated with ethyl acetate/petroleum ether (1/2) and filtered to afford 1-(6-(hydroxymethyl)pyridin-3-yl)dihydropyrimidine-2,4(1H,3H)-dione.

### General synthesis method 3 of compounds of Formula (II):

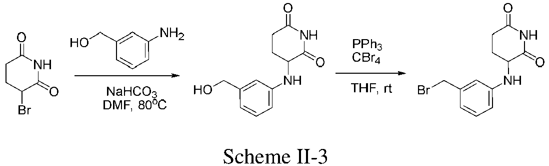

Step 1: 3-Bromopiperidine-2,6-dione (1.2 eq.) and (3-aminophenyl)methanol (1.0 eq.) were dissolved in N,N-dimethylformamide at room temperature, followed by the addition of sodium bicarbonate (1.5 eq.) to the reaction mixture. The air in the reaction vessel was replaced with nitrogen three times. The reaction solution was heated to 80 °C and stirred for 8 h. After monitoring the reaction via LC/MS and confirming its completion, the reaction mixture was cooled to room temperature and concentrated under reduced pressure. The residue was dissolved in ethyl acetate and washed with water. After phase separation, the aqueous phase was extracted with ethyl acetate. The combined organic phases were washed with saturated brine, dried over anhydrous sodium sulfate, filtered, and concentrated under reduced pressure. The residue was purified by column chromatography (methanol/dichloromethane = 0-10%) to afford 3-((3-(hydroxymethyl)phenyl)amino)piperidine-2,6-dione.

Step 2: 3-((3-(Hydroxymethyl)phenyl)amino)piperidine-2,6-dione (1.0 eq.) was dissolved in tetrahydrofuran at room temperature, followed by the addition of triphenylphosphine (2.0 eq.) and carbon tetrachloride (2.0 eq.) to the reaction mixture. The air in the reaction vessel was replaced with nitrogen three times. The reaction solution was stirred at room temperature for 8 h. After monitoring the reaction via LC/MS and confirming its completion, the reaction mixture was concentrated under reduced pressure. The residue was dissolved in ethyl acetate and washed with water. After phase separation, the aqueous phase was extracted with ethyl acetate. The combined organic phases were washed with saturated brine, dried over anhydrous sodium sulfate, filtered, and concentrated under reduced pressure. The residue was purified by column chromatography (ethyl acetate/petroleum ether = 0-100%) to afford 3-((3-(bromomethyl)phenyl)amino)piperidine-2,6-dione.

### General synthesis method 4 of compounds of Formula (II):

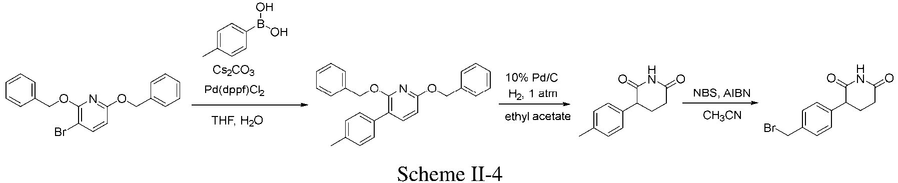

Step 1: 2,6-Bis(benzyloxy)-3-bromopyridine (1.0 eq.) and p-tolylboronic acid (1.3 eq.) were dissolved in a mixture of tetrahydrofuran and water, followed by the addition of cesium carbonate (3.0 eq.) and a palladium catalyst (0.1 eq.) to the reaction mixture. The air in the reaction vessel was replaced with nitrogen three times. The reaction solution was heated to 80 °C and stirred for 8 h. After monitoring the reaction via TLC and confirming its completion, the reaction mixture was cooled to room temperature and concentrated under reduced pressure. The residue was dissolved in ethyl acetate and washed with water. After phase separation, the aqueous phase was extracted with ethyl acetate. The combined organic phases were washed with saturated brine, dried over anhydrous sodium sulfate, filtered, and concentrated under reduced pressure. The residue was purified by column chromatography (ethyl acetate/petroleum ether = 0-10%) to afford 2,6-bis(benzyloxy)-3-(p-tolyl)pyridine.

Step 2: 2,6-Bis(benzyloxy)-3-(p-tolyl)pyridine (1.0 eq.) was dissolved in ethyl acetate at room temperature, followed by the addition of palladium on carbon (0.1 eq., 10%) to the reaction mixture. The air in the reaction vessel was replaced with hydrogen three times. The reaction solution was stirred under a hydrogen atmosphere (1 atm) at room temperature for 18 h. After monitoring the reaction via TLC and confirming its completion, the reaction mixture was filtered, and the filtrate was concentrated under reduced pressure to afford 3-(p-tolyl)piperidine-2,6-dione.

Step 3: N-Bromosuccinimide (1.1 eq.) and azobisisobutyronitrile (0.1 eq.) were successively added to a solution of 3-(p-tolyl)piperidine-2,6-dione (1.0 eq.) in acetonitrile at room temperature. The air in the reaction vessel was replaced with nitrogen three times. The reaction solution was stirred at room temperature for 18 h. After monitoring the reaction via TLC and confirming its completion, the reaction mixture was concentrated under reduced pressure. The residue was dissolved in ethyl acetate and washed with water. After phase separation, the aqueous phase was extracted with ethyl acetate. The combined organic phases were washed with saturated brine, dried over anhydrous sodium sulfate, filtered, and concentrated under reduced pressure. The residue was purified by column chromatography (ethyl acetate/petroleum ether = 0-10%) to afford 3-(4-(bromomethyl)phenyl)piperidine-2,6-dione.

### General synthesis method 5 of compounds of Formula (II):

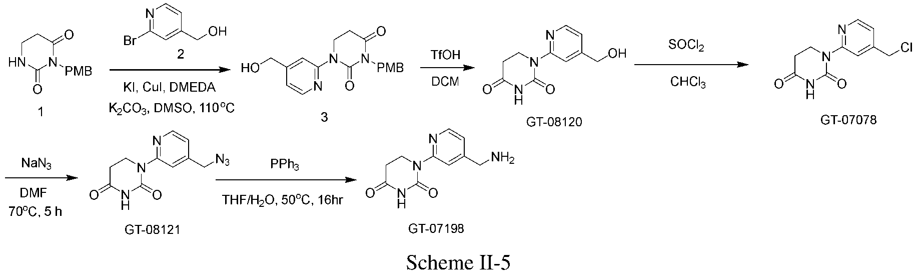

### General synthesis method 6 of compounds of Formula (II):

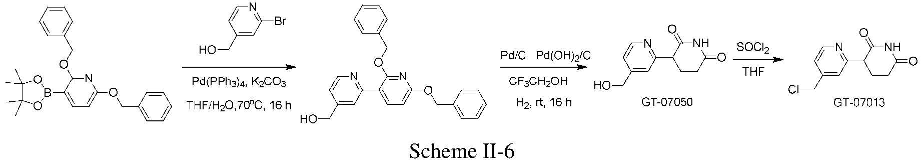

### General synthesis method 7 of compounds of Formula (II):

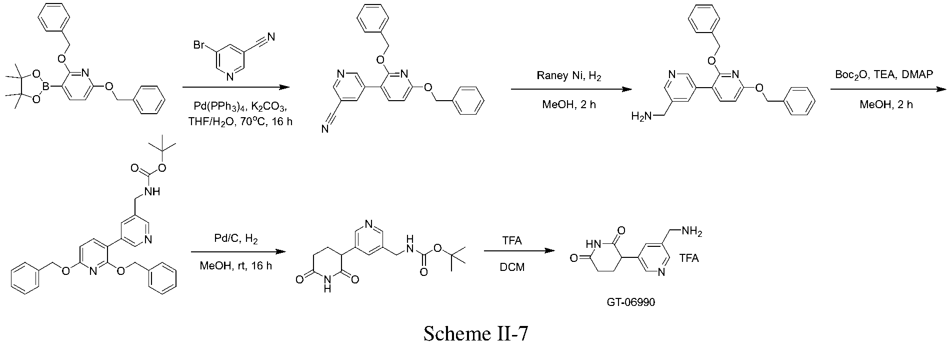

### General synthesis method 8 of compounds of Formula (II):

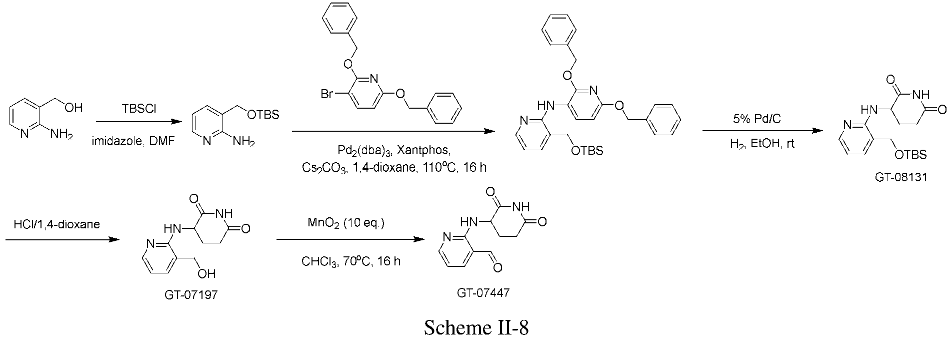

### General synthesis method 57 of intermediate compounds:

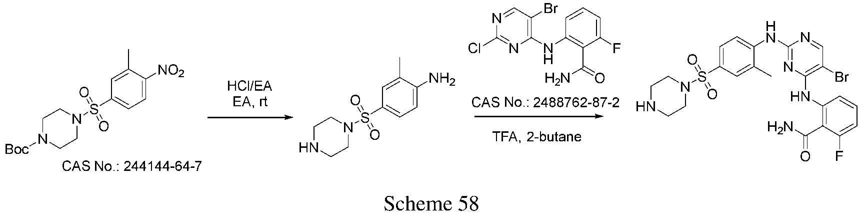

### General synthesis method 58 of intermediate compounds:

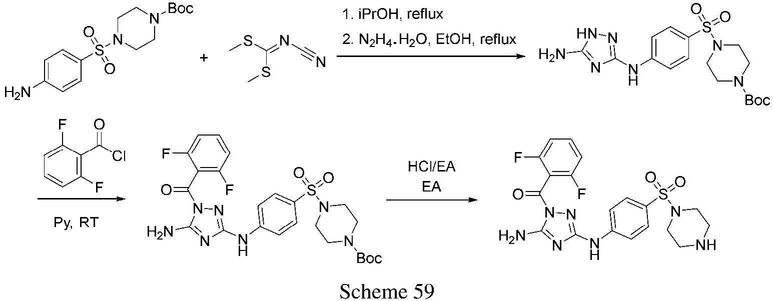

### General synthesis method 59 of intermediate compounds:

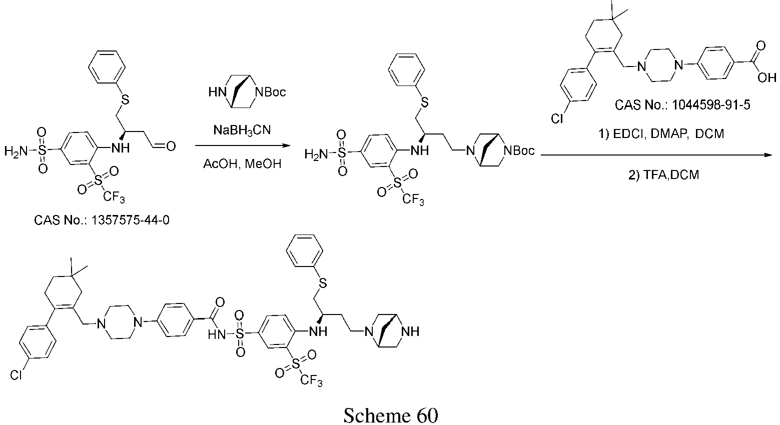

### General synthesis method 60 of intermediate compounds:

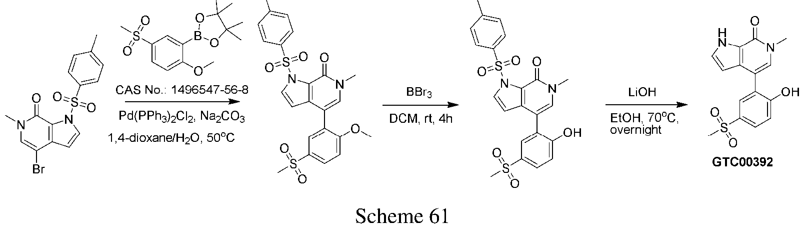

### General synthesis method 61 of intermediate compounds:

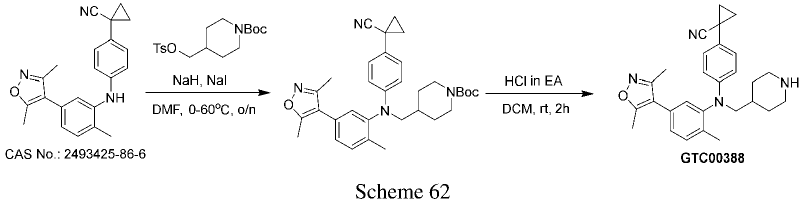

### General synthesis method 62 of intermediate compounds:

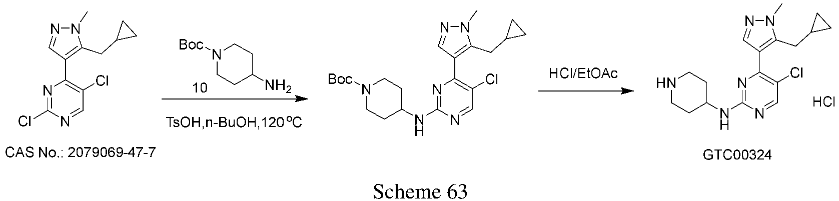

### General synthesis method 63 of intermediate compounds:

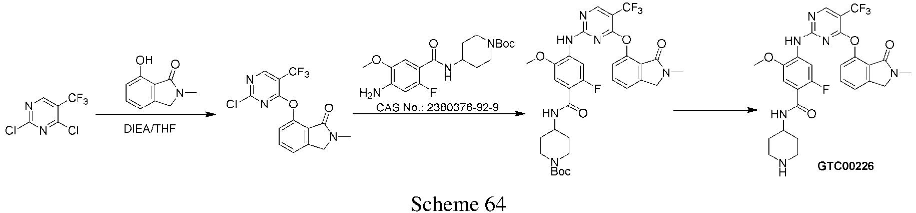

### General synthesis method 64 of intermediate compounds:

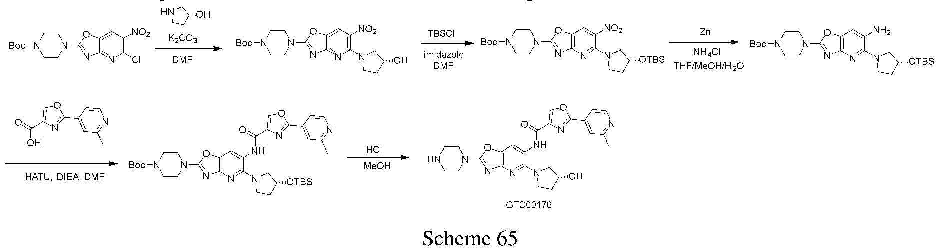

### General synthesis method 65 of intermediate compounds:

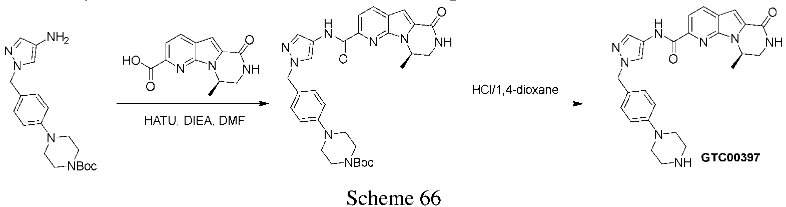

### General synthesis method 66 of intermediate compounds:

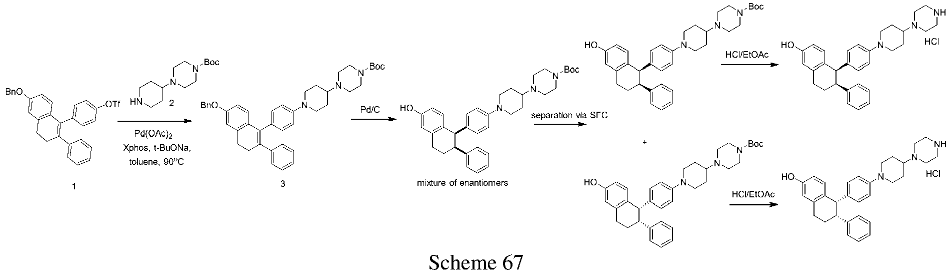

Pure enantiomeric products can be obtained through resolution/separation via supercritical fluid chromatography (SFC) and according to Scheme 67. The conditions of supercritical fluid chromatography (SFC) can be appropriately modified and adjusted using techniques and methods well-known to those skilled in the art to obtain the desired target compound.

Depending upon the target compounds, the above schemes and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

### Examples

### Intermediate Example 1: preparation of 1-((3S,4R)-3-fluoropiperidin-4-yl)-3-(4-phenoxyphenyl)-1H-pyrazolo[3,4-d]pyrimidin-4-amine

1-((3S,4R)-3-fluoropiperidin-4-yl)-3-(4-phenoxyphenyl)-1H-pyrazolo[3,4-d]pyrimidin-4-amine was prepared by referring to the method of Scheme 1.

**Step 1:** to a solution of tert-butyl (3S,4S)- 3-fluoro-4-hydroxypiperidine-1-carboxylate (370 mg, 1.69 mmol) in anhydrous dichloromethane were added DIEA (0.5 ml, 3.38 mmol), p-toluenesulfonyl chloride (386 mg, 2.03 mmol), and DMAP (206.16 mg, 1.69 mmol). The reaction mixture was stirred at room temperature for 18 hours, and the reaction was completed as detected by TLC. The reaction mixture was washed with H₂O (50 mL) and extracted with DCM (50 mL x 3). The combined organic phases were dried over anhydrous sodium sulfate, filtered, and the filtrate was concentrated under reduced pressure. The residue was purified by column chromatography (eluent (v/v): PE/EtOAc = 1/1) to give the white solid compound tert-butyl (3S,4S)-3-fluoro-4-(tosyloxy)piperidine-1-carboxylate (350 mg, yield 55%).

**Step 2:** to a solution of the compound tert-butyl (3S,4S)-3-fluoro-4-(tosyloxy)piperidine-1-carboxylate (350 mg, 0.94 mmol) in anhydrous DMSO (20 mL) were added 3-(4-phenoxyphenyl)-1H-pyrazolo[3,4-d]pyrimidin-4-amine (284 mg, 0.94 mmol) and K₂CO₃ (388 mg, 2.81 mmol). The reaction mixture was heated to 85°C and stirred for 18 hours, and the reaction was completed as detected by TLC. The reaction mixture was cooled to room temperature, washed with H₂O (100 mL), and extracted with EtOAc (100 mL x 3). The combined organic phases were dried over anhydrous sodium sulfate, filtered, and the filtrate was concentrated under reduced pressure. The residue was purified by column chromatography with eluent (DCM/MeOH = 20/1) to give the white solid compound tert-butyl (3S,4R)-4-(4-amino-3-(4-phenoxyphenyl)-1H-pyrazolo[3,4-d]pyrimidin-1-yl)-3-fluoropiperidine-1-carboxylate (350 mg, yield 74%). LC/MS (ESI, *m*/*z*) 505.3 [M + H]⁺.

**Step 3:** to a solution of the compound tert-butyl (3S,4R)-4-(4-amino-3-(4-phenoxyphenyl)-1H-pyrazolo[3,4-d]pyrimidin-1-yl)-3-fluoropiperidine-1-carboxylate (350 mg, 0.69 mmol) in anhydrous DCM (15 mL) was added HCl/dioxane (1.7 mL, 4M). The reaction mixture was stirred at room temperature for 1 hour, and the reaction was completed as detected by TLC. The reaction mixture was concentrated under reduced pressure to give the white solid target compound 1-((3S,4R)-3-fluoropiperidin-4-yl)-3-(4-phenoxyphenyl)-1H-pyrazolo[3,4-d]pyrimidin-4-amine (205 mg, yield 67%). ¹H NMR (400 MHz, DMSO) δ 8.24 (d, J = 5.6 Hz, 1H), 7.69 (d, J = 8.6 Hz, 2H), 7.44 (dd, J = 8.4, 7.6 Hz, 2H), 7.28 - 7.04 (m, 5H), 5.15 - 4.76 (m, 2H), 3.34 (d, J = 7.3 Hz, 1H), 2.97 (d, J = 12.3 Hz, 1H), 2.63 (ddd, J = 15.0, 12.2, 3.5 Hz, 2H), 2.12 (dd, J = 12.3, 4.1 Hz, 1H), 1.94 (d, J = 12.7 Hz, 1H). ¹⁹F NMR (377 MHz, DMSO) δ -185.23 (s). LC/MS (ESI, m/z) calcd for [M + H]⁺ ,405.2 found, 405.1.

### Intermediate Example 2: preparation of 3-(4-phenoxyphenyl)-1-(piperidin-4-yl)-1H-pyrazolo[3,4-d]pyrimidin-4-amine hydrochloride

3-(4-phenoxyphenyl)-1-(piperidin-4-yl)-1H-pyrazolo[3,4-d]pyrimidin-4-amine hydrochloride was prepared by referring to the method of Scheme 2.

**Step 1:** to a solution of triphenylphosphine (154.59 g, 574.1 mmol) in tetrahydrofuran (3 L) was added DIAD (117.54 g, 581.3 mmol) at 0°C. After addition, the mixture was stirred for 0.5 hours. Then, to the mixture was added 2-methylpropan-2-yl 4-hydroxypiperidine-1-carboxylate (118.6 g, 589.3 mmol) at 0°C, and the mixture was stirred for 0.5 hours. Subsequently, to the reaction solution was added 3-[4-(phenoxy)phenyl]-1H-pyrazolo[3,4-d]pyrimidin-4-amine (90 g, 296.7 mmol), and the temperature was slowly raised to 25°C and stirred for 16 hours. TLC analysis showed the completion of the reaction. The reaction mixture was concentrated under vacuum to obtain the crude product. The crude product was purified by silica gel flash column chromatography (eluent (v/v): PE/EtOAc = 1/0-1/1) to obtain the white solid compound tert-butyl 4-(4-amino-3-(4-phenoxyphenyl)-1H-pyrazolo[3,4-d]pyrimidin-1-yl)piperidine-1-carboxylate (92.0 g, 189.3 mmol, yield 63.8%). LCMS (ESI): calcd. 487.24; found, [M+H]+=487.30.

**Step 2:** tert-butyl 4-(4-amino-3-(4-phenoxyphenyl)-1H-pyrazolo[3,4-d]pyrimidin-1-yl)piperidine-1-carboxylate (92.0 g, 189.3 mmol) was dissolved in HCl/EtOAc (340 mL, 3 M) at 10°C. The mixture was stirred at room temperature for 3 hours. TLC analysis showed the completion of the reaction. A large amount of white solid precipitated, which was filtered and ground with ethyl acetate/petroleum ether (1: 8, 300 ml) and filtered again. The solid was dried under high vacuum to obtain the white solid compound 4-(4-amino-3-(4-phenoxyphenyl)-1H-pyrazolo[3,4-d]pyrimidin-1-yl)piperidine hydrochloride (60 g, 154.957 mmol, yield 52%). ¹H NMR (400 MHz, DMSO) δ 8.25 (s, 1H), 7.69 - 7.61 (m, 2H), 7.47 - 7.39 (m, 2H), 7.23 - 7.09 (m, 6H), 4.98 - 4.89 (m, 1H), 3.35 - 3.26 (m, 3H), 2.99 (t, *J =* 12.1 Hz, 2H), 2.35 - 2.19 (m, 2H), 2.07 - 1.98 (m, 2H). LCMS (ESI): calcd.: 387.19; found, [M+H]⁺=387.20.

### Intermediate Example 3: preparation of (S)-2-(4-(4-chlorophenyl)-2,3,9-trimethyl-6H-thieno[3,2-f][1,2,4]triazolo[4,3-a][1,4]diazepin-6-yl)-N-(2-(piperazin-1-yl)ethyl)acetamide

Referring to Scheme 6, in Step 1, to a solution of (S)-2-(4-(4-chlorophenyl)-2,3,9-trimethyl-6H-thieno[3,2-f][1,2,4]triazolo[4,3-a][1,4]diazepin-6-yl)acetic acid (1 equiv), tert-butyl 4-(2-aminoethyl)piperazine-1-carboxylate (1.2 equiv), and HATU (2 equiv) in 10 mL of DCM was added DIEA (5 equiv). The reaction was carried out at room temperature for 1 hour. The reaction mixture was washed with 10 mL of water twice and concentrated under reduced pressure to remove the solvent. The resulting residue was purified by silica gel column chromatography (eluent (v/v): DCM/MeOH = 100/0-10/1) to obtain the corresponding Boc intermediate compound.

**Step 2,** the compound obtained from Step 1 was dissolved in HCl/1,4-dioxane and reacted at room temperature for 1 hour. The reaction mixture was concentrated under reduced pressure to remove the solvent, and the target compound (yellow solid, 72 mg, yield 53%) was obtained. LCMS (ESI) m/z: calcd for C₂₅H₃₁ClN₇OS⁺ [M+H]⁺, 512.20; found, 512.2.

### Intermediate Example 4: preparation of (S)-2-(4-(4-chlorophenyl)-2,3,9-trimethyl-6H-thieno[3,2-f][1,2,4]triazolo[4,3-a][1,4]diazepin-6-yl)-1-(piperazin-1-yl)ethan-1-one

Referring to Scheme 6, in Step 1, to a solution of (S)-2-(4-(4-chlorophenyl)-2,3,9-trimethyl-6H-thieno[3,2-f][1,2,4]triazolo[4,3-a][1,4]diazepin-6-yl)acetic acid (1 equiv), tert-butyl piperazine-1-carboxylate (1.2 equiv), and HATU (2 equiv) in 10 mL of DCM was added DIEA (5 equiv). The reaction was carried out at room temperature for 1 hour. The reaction mixture was washed with 10 mL of water twice and concentrated under reduced pressure to remove the solvent. The resulting residue was purified by silica gel column chromatography (eluent (v/v): DCM to DCM/MeOH (10/1)) to obtain the corresponding Boc intermediate compound.

**Step 2,** the compound obtained from Step 1 was dissolved in HCl/1,4-dioxane and reacted at room temperature for 1 hour. The reaction mixture was concentrated under reduced pressure to remove the solvent, and the target compound (yellow solid, 260 mg, yield 92%) was obtained. LCMS (ESI) m/z: calcd for C₂₃H₂₆ClN₆OS⁺[M+H]⁺, 469.16; found, 469.1.

### Intermediate Example 5: preparation of tert-butyl 4-(2-(4-aminophenoxy)ethyl)piperazine-1-carboxylate

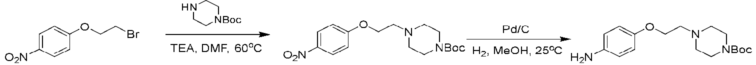

**Step 1:** To a solution of 1-(2-bromoethoxy)-4-nitrobenzene (4.50 g, 18.288 mmol) in N,N-dimethylformamide (100 mL) was added TEA (3.66 g, 36.576 mmol), and the reaction mixture was stirred at 60°C for 30 minutes. TLC analysis showed the completion of the reaction. The reaction mixture was diluted with EtOAc (100 mL) and ice water (100 mL). The organic layer was separated and further washed with saturated brine (100 mL), then concentrated under vacuum. The residue was purified by silica gel column chromatography (eluent (v/v): PE/EtOAc = 1/0-0/1) to obtain the colorless oily compound tert-butyl 4-(2-(4-nitrophenoxy)ethyl)piperazine-1-carboxylate (4 g, 11.383 mmol, yield 62.24%). ¹H NMR (300 MHz, CDCl₃) δ 8.19 (2H, m), 6.97 (2H, m), 4.19 (t, J = 5.6; LCMS (ESI): calcd for (M) 351.18, found, (M+H)⁺ 352.30.

**Step 2:** To a solution of tert-butyl 4-(2-(4-nitrophenoxy)ethyl)piperazine-1-carboxylate (4.00 g, 11.383 mmol) in MeOH (50 mL) was added Pd/C (0.12 g, 1.138 mmol, 10%), and the reaction was stirred under a hydrogen balloon at room temperature for 16 hours. LCMS analysis showed the completion of the reaction. The mixture was filtered through a celite bed and washed with methanol (20 mL). The filtrate was concentrated to obtain the off-white solid tert-butyl 4-(2-(4-aminophenoxy)ethyl)piperazine-1-carboxylate (3.1 g, yield 84.73%). LCMS (ESI): calcd for (M) 321.42, found, (M+H)⁺ 322.20.

### Intermediate Example 6: preparation of tert-butyl 4-(6-aminopyridazin-3-yl)piperazine-1-carboxylate

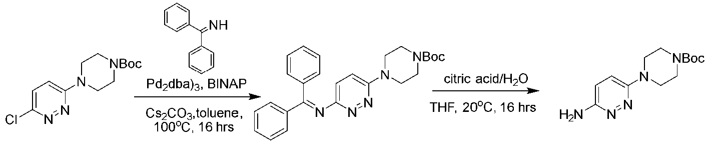

**Step 1:** Under argon protection, to a solution of tert-butyl 4-(6-chloropyridazin-3-yl)piperazine-1-carboxylate (3.00 g, 10.041 mmol) in toluene (60 mL) were added diphenylmethanimine (2.022 mL, 12.049 mmol), BINAP (0.06 g, 0.100 mmol), Cs₂CO₃ (6.54 g, 20.082 mmol) and Pd₂(dba)₃ (0.46 g, 0.502 mmol). The resulting suspension was stirred at 100°C for 16 hours. LCMS analysis showed the completion of the reaction. The reaction mixture was cooled to room temperature, then filtered through celite and washed with ethyl acetate (30 mL). The filtrate was washed with saturated brine (20 mL), dried over anhydrous magnesium sulfate, and filtered to separate the solid. The filtrate was concentrated under reduced pressure, and the resulting residue was purified by silica gel column chromatography to obtain the white solid compound tert-butyl 4-(6-((diphenylmethylene)amino)pyridazin-3-yl)piperazine-1-carboxylate (2.60 g, 5.862 mmol, yield 58.38%). LCMS (ESI): calcd., 443.20; found, [M+H]⁺=444.20.

**Step 2:** To a solution of tert-butyl 4-(6-((diphenylmethylene)amino)pyridazin-3-yl)piperazine-1-carboxylate (2.60 g, 5.862 mmol) in THF (50 mL) was added citric acid/H₂O (20 mL), and the mixture was stirred at 20°C for 16 hours. LCMS analysis showed the completion of the reaction. The reaction mixture was concentrated under vacuum to remove THF. The resulting aqueous phase was adjusted to pH = 8 with saturated NaHCO₃ and extracted with EtOAc (100 mL × 3). The combined organic phases were dried over Na₂SO₄ and concentrated under reduced pressure to obtain the white solid tert-butyl 4-(6-aminopyridazin-3-yl)piperazine-1-carboxylate (1.6 g, yield 97.71%). LCMS (ESI): calcd for M: 279.20; found, [M+H]⁺ =280.20.

### Intermediate Example 7: preparation of N-(tert-butyl)-3-((5-methyl-2-((4-(2-(piperazin-1-yl)ethoxy)phenyl)amino)pyrimidin-4-yl)amino)benzenesulfonamide (GT-M-002)

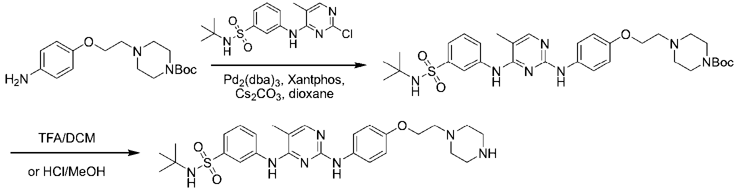

**Step 1:** tert-butyl 4-(2-(4-((4-((3-(N-(tert-butyl)sulfamoyl)phenyl)amino)-5-methylpyrimidin-2-yl)amino)phenoxy)ethyl)piperazine-1-carboxylate was prepared by referring to the method of Step 2 in Scheme 3.

Under argon protection, to a solution of tert-butyl 4-(2-(4-aminophenoxy)ethyl)piperazine-1-carboxylate (2.0 g, 6.222 mmol) and N-(tert-butyl)-3-((2-chloro-5-methylpyrimidin-4-yl)amino)benzenesulfonamide (prepared in Scheme 3, 2.87 g, 8.089 mmol) in dioxane (50 mL) were added Pd₂(dba)₃ (0.36g,0.622mmol), Cs₂CO₃ (4.06g,12.445mmol), and 4,5-bis(diphenylphosphino)-9,9-dimethylxanthene (0.72 g, 1.244 mmol). The reaction mixture was stirred at 100°C for 4 hours. The reaction mixture was diluted with EtOAc (50 mL) and saturated NaCl solution (50 mL). The resulting reaction mixture was dried over Na₂SO₄, concentrated under vacuum to obtain the crude product. The crude product was purified by silica gel flash column chromatography (eluent (v/v): EtOAc/PE = 0/1-1/4) to obtain the white solid compound tert-butyl 4-(2-(4-((4-((3-(N-(tert-butyl)sulfamoyl)phenyl)amino)-5-methylpyrimidin-2-yl)amino)phenoxy)ethyl)piperazine-1-carboxylate (2 g, 3.126 mmol, 50.24%). LCMS (ESI): calcd for (M) 639.20, found, (M+H)⁺ 640.20.

**Step 2:** To a solution of tert-butyl 4-(2-(4-((4-((3-(N-(tert-butyl)sulfamoyl)phenyl)amino)-5-methylpyrimidin-2-yl)amino)phenoxy)ethyl)piperazine-1-carboxylate (2 g, 3.126 mmol) in DCM (50 mL) was added TFA (10 mL), and the reaction mixture was stirred at room temperature for 20 minutes. LCMS analysis showed the completion of the reaction. The reaction mixture was concentrated under vacuum to obtain the white solid compound N-(tert-butyl)-3-((5-methyl-2-((4-(2-(piperazin-1-yl)ethoxy)phenyl)amino)pyrimidin-4-yl)amino)benzenesulfonamide (1.20 g, yield 71.13%). ¹H NMR (400 MHz, DMSO) δ 8.78 (s, 1H), 8.54 (s, 1H), 8.11 (s, 2H), 7.90 (s, 1H), 7.62 - 7.40 (m, 5H), 6.79 (d, 1H) J = 9.0 Hz, 2H), 3.99 (t, J = 5.9 Hz, 2H), 2.65 (dt, J = 11.8, 5.3 Hz, 6H), 2.38 (s, 4H), 2.12 (s, 3H), 1.12 ( s, 9H). LCMS (ESI): (M+H)⁺ calcd for (M) 540.3, found, 540.7.

### Intermediate Example 8: preparation of N-(tert-butyl)-3-((5-methyl-2-((6-(piperazin-1-yl)pyridazin-3-yl)amino)pyrimidin-4-yl)amino)benzenesulfonamide (GT-M-17)

Following the method of Step 2 in Scheme 3 and the method of Intermediate Example 7, under appropriate conditions understood in the field, tert-butyl 4-(6-aminopyridazin-3-yl)piperazine-1-carboxylate prepared from Intermediate Example 6 and N-(tert-butyl)-3-((2-chloro-5-methylpyrimidin-4-yl)amino)benzenesulfonamide were used to prepare the target compound N-(tert-butyl)-3-((5-methyl-2-((6-(piperazin-1-yl)pyridazin-3-yl)amino)pyrimidin-4-yl)amino)benzenesulfonamide (552 mg, yield 66.31%) as a white solid. ¹H NMR (400 MHz, DMSO) δ 9.35 (s, 1H), 8.60 (s, 1H), 8.25 (s, 1H), 8.14 (dd, J = 6.9, 2.4 Hz, 1H), 8.07 (d, J = 9.8 Hz, 1H), 7.94 (d, J = 0.7 Hz, 1H), 7.65 (s, 1H), 7.53 - 7.43 (m, 2H), 7.21 (d, J = 9.9 Hz, 1H), 3.45 - 3.35 (m, 4H), 2.89 - 2.75 (m, 4H), 2.15 (s, 3H), 1.12 (s, 9H). LCMS (ESI): calcd for M: 497.30; found, [M+H]⁺=498.30.

### Intermediate Example 9: preparation of N-(tert-butyl)-3-((5-methyl-2-((4-(piperidin-4-yl)phenyl)amino)pyrimidin-4-yl)amino)benzenesulfonamide (GT-M-003)

Following the method of Step 2 in Scheme 3 and the method of Intermediate Example 7, under appropriate conditions understood in the field, N-(tert-butyl)-3-((2-chloro-5-methylpyrimidin-4-yl)amino)benzenesulfonamide and tert-butyl 4-(4-aminophenyl)piperidine-1-carboxylate were used to prepare the target compound N-(tert-butyl)-3-((5-methyl-2-((4-(piperidin-4-yl)phenyl)amino)pyrimidin-4-yl)amino)benzenesulfonamide (1.2 g, yield 72.14%) as a white solid. ¹H NMR (400 MHz, DMSO) δ 8.87 (s, 1H), 8.56 (s, 1H), 8.11 (s, 2H), 7.92 (s, 1H), 7.65 - 7.40 (m, 5H), 7.04 (d, J = 8.5 Hz, 2H), 3.17 (s, 3H), 3.00 (d, J = 12.0 Hz, 2H), 2.55 (dd, J = 12.1, 10.3 Hz, 2H), 2.48 (d, J = 15.5 Hz, 3H), 2.13 (s, 3H), 1.64 (d, J = 11.5 Hz, 2H), 1.46 (dd, J = 12.2, 3.6 Hz, 2H), 1.12 (s, 9H). LCMS (ESI): (M+H)⁺ calcd. (M) 495.3, found, 495.2.

### Intermediate Example 10: preparation of N-(tert-butyl)-3-((5-methyl-2-((4-(piperazin-1-yl)phenyl)amino)pyrimidin-4-yl)amino)benzenesulfonamide (GT-M-004)

Following the method of Step 2 in Scheme 3 and the method of Intermediate Example 7, under appropriate conditions understood in the field, N-(tert-butyl)-3-((2-chloro-5-methylpyrimidin-4-yl)amino)benzenesulfonamide and tert-butyl 4-(4-aminophenyl)piperazine-1-carboxylate were used to prepare the target compound N-(tert-butyl)-3-((5-methyl-2-((4-(piperazin-1-yl)phenyl)amino)pyrimidin-4-yl)amino)benzenesulfonamide (1.5 g, yield 90.15%) as a white solid. ¹H NMR (400 MHz, DMSO) δ 8.70 (s, 1H), 8.51 (s, 1H), 8.14 (s, 2H), 7.89 (s, 1H), 7.57 (s, 1H), 7.48 (d, J = 6.3 Hz, 4H), 6.80 (d, J = 8.9 Hz, 2H), 3.57 (s, 2H), 3.17 (s, 1H), 3.00 - 2.89 (m, 4H), 2.81 (dd, J = 10.1, 5.8 Hz, 5H), 2.12 (s, 3H), 1.12 (s, 9H). LCMS (ESI): (M+H)⁺ calcd. (M) 496.3, found, 496.2.

### Intermediate Example 11: preparation of 6-(4-(tert-butoxycarbonyl)piperazin-1-yl)pyridazine-3-carboxylic acid

6-(4-(tert-butoxycarbonyl)piperazin-1-yl)pyridazine-3-carboxylic acid was prepared by referring to the method of Scheme 4.

**Step 1:** to a solution of methyl 6-Chloropyridazine-3-carboxylate (500.00 mg, 2.897 mmol) and tert-butyl piperazine-1-carboxylate (809.45 mg, 4.346 mmol) in DMF (10.00 mL) was added DIEA (1.437 mL, 8.692 mmol), and the reaction mixture was stirred at 60°C for 12 h. LC-MS analysis showed completion of the reaction. Water was added to the reaction mixture, which was then extracted with ethyl acetate. The organic phase was separated, washed with saturated brine, and concentrated to obtain a crude product. The crude product was purified by chromatography (eluent (v/v): PE/EA = 5/1) to yield methyl 6-(4-(tert-butoxycarbonyl)piperazin-1-yl)pyridazine-3-carboxylate (900 mg, yield 75.16%) as a white solid. LCMS (ESI): calcd for C₁₅H₂₃N₄O₄⁺ [M+H]⁺: 323.16, found, 323.20.

**Step 2:** to a solution of methyl 6-(4-(tert-butoxycarbonyl)piperazin-1-yl)pyridazine-3-carboxylate (400 mg, 1.241 mmol) in a mixed solvent of 1,4-dioxane and water (1/1; 10 mL) was added Lithium hydroxide (260.32 mg, 6.204 mmol), and the reaction mixture was stirred at 60°C for 12 h. LC-MS analysis showed completion of the reaction. The reaction mixture was concentrated under reduced pressure to remove the organic phase. The remaining aqueous phase was adjusted to pH = 6 with concentrated hydrochloric acid and then extracted with ethyl acetate. The organic phase was separated, concentrated under reduced pressure to obtain 6-(4-(tert-butoxycarbonyl)piperazin-1-yl)pyridazine-3-carboxylic acid (300 mg, yield 76.06%) as a white solid, which can be used for the next step without further purification. LCMS (ESI): calcd for C₁₄H₂₁N₄O₄⁺ [M+H]⁺: 309.15, found, 309.20.

### Intermediate Example 12: preparation of N-((1r,4r)-4-(3-chloro-4-cyanophenoxy)cyclohexyl)-6-(piperazin-1-yl)pyridazine-3-carboxamide

N-((1r,4r)-4-(3-chloro-4-cyanophenoxy)cyclohexyl)-6-(piperazin-1-yl)pyridazine-3-carboxamide was prepared by referring to the method of Scheme 5.

To a solution of 6-(4-(tert-butoxycarbonyl)piperazin-1-yl)pyridazine-3-carboxylic acid (300 mg, 1.135 mmol) prepared from Intermediate Example 11 and 4-(((1r,4r)-4-aminocyclohexyl)oxy)-2-chlorobenzonitrile (326.61 mg, 1.135 mmol) in DMF (5.00 mL) were added HATU (647.40 mg, 1.703 mmol) and DIEA (0.938 mL, 5.676 mmol), and the resulting reaction mixture was stirred at room temperature for 30 minutes. LC-MS analysis showed completion of the reaction. Water was added to the reaction mixture, which was then extracted with ethyl acetate. The organic phase was separated, washed with saturated brine, and concentrated to obtain a crude product. The crude product was purified by chromatography (eluent (v/v): DCM/MeOH = 100/1) to yield the white solid compound tert-butyl 4-(6-(((1r,4r)-4-(3-chloro-4-cyanophenoxy)cyclohexyl)carbamoyl)pyridazin-3-yl)piperazine-1-carboxylate (250 mg, yield 39.49%). LCMS (ESI): calcd for C₂₇H₃₄ClN₆O₄⁺ [M+H]⁺: 541.23, found, 541.30.

Tert-butyl 4-(6-(((1r,4r)-4-(3-chloro-4-cyanophenoxy)cyclohexyl)carbamoyl)pyridazin-3-yl)piperazine-1-carboxylate (250 mg, 0.462 mmol) was dissolved in a mixed solvent of DMF (5.00 mL) and TFA (1.00 mL). The reaction mixture was stirred at room temperature for 3 h. LC-MS analysis showed completion of the reaction. The reaction mixture was concentrated under reduced pressure to remove the organic phase. The remaining aqueous phase was adjusted to pH = 8 with saturated NaHCO₃ solution and then extracted with ethyl acetate. The organic phase was separated, concentrated under reduced pressure to obtain N-((1r,4r)-4-(3-chloro-4-cyanophenoxy)cyclohexyl)-6-(piperazin-1-yl)pyridazine-3-carboxamide (170 mg, yield 75.10%) as a white solid, which can be used for the next step without further purification. LCMS (ESI): calcd for C₂₂H₂₆ClN₆O₂⁺ [M+H]⁺: 441.17, found, 441.20.

### Intermediate Example 13: preparation of N-(tert-butyl)-3-((5-methyl-2-((4-(4-(methylamino)piperidin-1-yl)phenyl)amino)pyrimidin-4-yl)amino)benzenesulfonamide (GT-D-89)

N-(tert-butyl)-3-((5-methyl-2-((4-(4-(methylamino)piperidin-1-yl)phenyl)amino)pyrimidin-4-yl)amino)benzenesulfonamide (GT-D-89) was prepared by referring to Scheme 9 (white solid, 1.1 g).¹H NMR (400 MHz, MeOD) δ 8.07 (s, 1H), 7.80 (d, *J =* 7.9 Hz, 1H), 7.78 - 7.69 (m, 2H), 7.58 (t, *J* = 8.0 Hz, 1H), 7.34 (d, *J =* 9.0 Hz, 2H), 7.16 (d, *J =* 9.0 Hz, 2H), 3.83 (d, *J =* 12.9 Hz, 2H), 3.33 (d, *J =* 11.0 Hz, 1H), 3.10 (t, *J =* 12.1 Hz, 2H), 2.76 (s, 3H), 2.33 - 2.17 (m, 5H), 1.88 (qd, *J =* 12.5, 3.8 Hz, 2H), 1.16 (d, *J =* 5.5 Hz, 9H). LCMS (ESI): calcd., 524.3; found, 524.4.

### Intermediate Example 14: preparation of N-(tert-butyl)-3-((5-methyl-2-((4-(4-(piperazin-1-yl)piperidin-1-yl)phenyl)amino)pyrimidin-4-yl)amino)benzenesulfonamide (GT-D-90)

N-(tert-butyl)-3-((5-methyl-2-((4-(4-(piperazin-1-yl)piperidin-1-yl)phenyl)amino)pyrimidin-4-yl)amino)benzenesulfonamide (GT-D-90) was prepared by referring to Scheme 9 (white solid, 0.90 g). ¹H NMR (400 MHz, MeOD) δ 8.07 (s, 1H), 7.82 (d, *J =* 7.9 Hz, 1H), 7.75 (s, 1H), 7.70 (d, *J =* 8.3 Hz, 1H), 7.60 (t, *J =* 7.9 Hz, 1H), 7.43 (d, *J =* 8.9 Hz, 2H), 7.28 (d, *J =* 9.0 Hz, 2H), 3.98 (s, 1H), 3.84 (d, *J =* 12.7 Hz, 2H), 3.59 - 3.47 (m, 4H), 3.40 (s, 4H), 3.25 (d, *J =* 11.6 Hz, 2H), 2.34 - 2.20 (m, 5H), 2.15 - 1.98 (m, 2H), 1.18 (d, *J* = 15.0 Hz, 9H). LCMS (ESI): calcd., 579.3; found, 579.4.

### Intermediate Example 15: preparation of 5-chloro-N²-(2-isopropoxy-5-methyl-4-(piperazin-1-yl)phenyl)-N⁴-(2-(isopropylsulfonyl)phenyl)pyrimidine-2,4-diamine (GT-D-86)

5-chloro-N²-(2-isopropoxy-5-methyl-4-(piperazin-1-yl)phenyl)-N⁴-(2-(isopropylsulfonyl)phenyl)pyrimidine-2,4-diamine (GT-D-86) was prepared by referring to Scheme 9 (brown solid, 2.46 g). ¹H NMR (400 MHz, DMSO) δ 9.51 (s, 1H), 9.14 (s, 2H), 8.43 (d, *J* = 8.3 Hz, 1H), 8.26 (s, 1H), 8.20 (s, 1H), 7.85 (dd, *J =* 7.9, 1.2 Hz, 1H), 7.63 (t, *J =* 7.7 Hz, 1H), 7.51 (s, 1H), 7.37 (t, *J =* 7.5 Hz, 1H), 6.70 (s, 1H), 4.56 (dd, *J =* 12.0, 6.0 Hz, 1H), 3.23 (s, 4H), 3.04 (d, *J =* 4.3 Hz, 4H), 2.09 (s, 3H), 1.19 (dd, *J =* 24.2, 6.4 Hz, 12H). LCMS (ESI): calcd., 559.2; found, 559.1.

### Intermediate Example 16: preparation of 5-chloro-N²-(2-isopropoxy-5-methyl-4-(4-(methylamino)piperidin-1-yl)phenyl)-N⁴-(2-(isopropylsulfonyl)phenyl)pyrimidine-2,4-diamine (GT-D-87)

5-chloro-N²-(2-isopropoxy-5-methyl-4-(4-(methylamino)piperidin-1-yl)phenyl)-N⁴-(2-(isopropylsulfonyl)phenyl)pyrimidine-2,4-diamine (GT-D-87) was prepared by referring to Scheme 9 (white solid, 1.2 g). ¹H NMR (400 MHz, DMSO) δ 9.93 (s, 1H), 9.19 (s, 2H), 8.93 (s, 1H), 8.42 (s, 1H), 8.20 (s, 1H), 7.90 (s, 1H), 7.73 (t, *J* = 7.7 Hz, 1H), 7.51 (t, *J =* 7.7 Hz, 1H), 7.39 (s, 1H), 6.81 (s, 1H), 4.71 - 4.36 (m, 1H), 3.48 (dt, *J =* 13.4, 6.7 Hz, 1H), 2.57 (t, *J =* 5.3 Hz, 3H), 2.13 (s, 2H), 2.04 (s, 3H), 1.82 (s, 2H), 1.24 (d, *J* = 6.0 Hz, 6H), 1.14 (d, *J =* 6.8 Hz, 6H). LCMS (ESI): calcd., 587.3; found, 587.3.

### Intermediate Example 17: preparation of 5-chloro-N²-(2-isopropoxy-5-methyl-4-(4-(piperazin-1-yl)piperidin-1-yl)phenyl)-N⁴-(2-(isopropylsulfonyl)phenyl)pyrimidine-2,4-diamine (GT-D-88)

5-chloro-N²-(2-isopropoxy-5-methyl-4-(4-(piperazin-1-yl)piperidin-1-yl)phenyl)-N⁴-(2-(isopropylsulfonyl)phenyl)pyrimidine-2,4-diamine (GT-D-88) was prepared by referring to Scheme 9 (brown solid, 1.2 g). ¹H NMR (400 MHz, DMSO) δ 9.47 (s, 1H), 8.46 (d, *J =* 7.9 Hz, 1H), 8.24 (s, 1H), 8.09 (s, 1H), 7.83 (d, *J =* 7.9 Hz, 1H), 7.61 (t, *J =* 7.8 Hz, 1H), 7.49 (s, 1H), 7.35 (t, *J =* 7.7 Hz, 1H), 6.70 (s, 1H), 4.60 - 4.42 (m, 1H), 3.54 - 3.36 (m, 8H), 3.26 - 3.06 (m, 4H), 2.66 (t, *J =* 11.1 Hz, 2H), 2.15 (d, *J =* 11.0 Hz, 1H), 2.09 (s, 3H), 1.79 (s, 2H), 1.26 (dd, *J* = 13.1, 7.2 Hz, 3H), 1.22 - 1.09 (m, 9H). LCMS (ESI): calcd., 642.3; found, 642.3.

### Intermediate Example 18: preparation of (2-((5-chloro-2-((2-methoxy-4-(piperazin-1-yl)phenyl)amino)pyrimidin-4-yl)amino)phenyl)dimethylphosphine oxide (GT-D-39)

(2-((5-chloro-2-((2-methoxy-4-(piperazin-1-yl)phenyl)amino)pyrimidin-4-yl)amino)phenyl)dimethylphosphine oxide (GT-D-39) was prepared by referring to Scheme 9 (yellow solid, 7.9 g). ¹H NMR (400 MHz, DMSO) δ 11.57 (s, 1H), 9.55 (s, 1H), 9.17 (s, 2H), 8.76 (s, 1H), 8.44 (s, 1H), 8.17 (s, 1H), 7.59 (dd, *J* = 13.3, 7.7 Hz, 1H), 7.50 - 7.30 (m, 2H), 7.17 (t, *J* = 7.3 Hz, 1H), 6.73 (d, *J* = 2.1 Hz, 1H), 6.56 (dd, *J =* 8.6, 2.2 Hz, 1H), 3.79 (s, 3H), 3.49 - 3.36 (m, 4H), 3.26 (m, 4H), 1.80 (s, 3H), 1.77 (s, 3H). LCMS (ESI): calcd., 487.2; found, 487.2.

### Intermediate Example 19: preparation of (R)-N-(5-(2-methoxy-2-phenylacetyl)-1,4,5,6-tetrahydropyrrolo[3,4-c]pyrazol-3-yl)-4-(piperazin-1-yl)benzamide (GT-M-08)

(R)-N-(5-(2-methoxy-2-phenylacetyl)-1,4,5,6-tetrahydropyrrolo[3,4-c]pyrazol-3-yl)-4-(piperazin-1-yl)benzamide (GT-M-08) was prepared by referring to Scheme 10 (white solid,0.165 g). ¹H NMR (400 MHz, DMSO) δ 10.71 (s, 1H), 7.89 (dd, J = 9.0, 2.7 Hz, 2H), 7.59 - 7.20 (m, 5H), 6.97 (dd, J = 9.0, 1.9 Hz, 2H), 5.11 (d, J = 12.1 Hz, 1H), 4.79 (dd, J = 23.9, 12.6 Hz, 1H), 4.62 - 4.26 (m, 4H), 3.34 (d, J = 2.4 Hz, 4H), 3.23 - 3.09 (m, 4H), 2.93 - 2.67 (m, 4H). LCMS (ESI): calcd., 461.2; found, 461.6.

### Intermediate Example 21: preparation of 2-((2-((2-methoxy-4-(piperazin-1-yl)phenyl)amino)-5-(trifluoromethyl)pyridin-4-yl)amino)-N-methylbenzamide (GT-M-10)

2-((2-((2-methoxy-4-(piperazin-1-yl)phenyl)amino)-5-(trifluoromethyl)pyridin-4-yl)amino)-N-methylbenzamide (GT-M-10) was prepared by referring to Scheme 9 (white solid, 1.87 g). ¹H NMR (400 MHz, MeOD) δ 8.01 (s, 1H), 7.73 (d, J = 7.9 Hz, 1H), 7.59 (d, J = 3.8 Hz, 2H), 7.36 (dt, J = 8.4, 4.1 Hz, 1H), 7.18 (d, J = 8.6 Hz, 1H), 6.79 (d, J = 2.1 Hz, 1H), 6.68 (dd, J = 8.7, 2.2 Hz, 1H), 6.48 (s, 1H), 3.87 (s, 3H), 3.66 (s, 4H), 3.56 - 3.48 (m, 4H), 3.43 - 3.37 (m, 4H), 2.91 (d, J = 13.7 Hz, 3H). LCMS (ESI): calcd., 501.2; found, 501.2.

### Intermediate Example 22: preparation of 2-((2-((4-(4-aminopiperidin-1-yl)-2-methoxyphenyl)amino)-5-(trifluoromethyl)pyridin-4-yl)amino)-N-methylbenzamide (GT-M-11)

2-((2-((4-(4-aminopiperidin-1-yl)-2-methoxyphenyl)amino)-5-(trifluoromethyl)pyridin-4-yl)amino)-N-methylbenzamide (GT-M-11) was prepared by referring to Scheme 9 (brown solid,0.4 g). ¹H NMR (400 MHz, DMSO) δ 10.13 (s, 1H), 8.63 (d, J = 4.2 Hz, 1H), 8.27 (s, 1H), 8.14 (s, 1H), 7.74 - 7.60 (m, 1H), 7.44 (dd, J = 6.3, 1.3 Hz, 2H), 7.36 (d, J = 8.7 Hz, 1H), 7.08 - 7.00 (m, 1H), 6.57 (d, J = 2.5 Hz, 1H), 6.51 (s, 1H), 6.43 (dd, J = 8.8, 2.5 Hz, 1H), 3.77 (s, 3H), 3.57 (d, J = 12.6 Hz, 3H), 2.75 (d, J = 3.7 Hz, 4H). LCMS (ESI): calcd., 515.2; found, 515.2.

### Intermediate Example 23: preparation of 2-((2-((2-methoxy-4-(4-(piperazin-1-yl)piperidin-1-yl)phenyl)amino)-5-(trifluoromethyl)pyridin-4-yl)amino)-N-methylbenzamide (GT-M-12)

2-((2-((2-methoxy-4-(4-(piperazin-1-yl)piperidin-1-yl)phenyl)amino)-5-(trifluoromethyl)pyridin-4-yl)amino)-N-methylbenzamide (GT-M-12) was prepared by referring to Scheme 9 (purple solid, 0.34 g).¹H NMR (400 MHz, DMSO) δ 10.60 (s, 1H), 10.10 (s, 1H), 9.99 (s, 1H), 9.75 (s, 1H), 8.82 (d, J = 4.6 Hz, 1H), 8.14 (s, 1H), 7.77 (d, J = 7.7 Hz, 1H), 7.57 (d, J = 3.2 Hz, 2H), 7.33 - 7.26 (m, 1H), 7.19 (s, 1H), 6.81 (s, 1H), 6.68 (s, 1H), 6.54 (s, 1H), 3.78 (d, J = 26.5 Hz, 15H), 3.53 (s, 9H), 2.84 (s, 2H), 2.76 (d, J = 4.5 Hz, 3H), 2.22 (d, J = 10.6 Hz, 2H), 1.87 (d, J = 9.5 Hz, 2H), 1.23 (s, 2H). LCMS (ESI): calcd., 584.3; found, 584.0.

### Intermediate Example 24: preparation of 2-((2-((2-methoxy-4-(piperazin-1-yl)phenyl)amino)-5-(trifluoromethyl)pyridin-4-yl)amino)-N-methylbenzamide (GT-M-171)

2-((2-((2-methoxy-4-(piperazin-1-yl)phenyl)amino)-5-(trifluoromethyl)pyridin-4-yl)amino)-N-methylbenzamide (GT-M-171) was prepared by referring to Scheme 9 (yellow solid, 20.5 g). ¹H NMR (400 MHz, DMSO) δ 10.39 (s, 1H), 9.20 (s, 1H), 8.80 (s, 2H), 8.69 (d, *J =* 4.6 Hz, 1H), 8.13 (s, 1H), 7.73 (d, *J =* 7.7 Hz, 1H), 7.53 (s, 2H), 7.35 (d, *J =* 8.6 Hz, 1H), 7.20 (s, 1H), 6.70 (d, *J =* 2.2 Hz, 1H), 6.59 - 6.49 (m, 2H), 3.80 (s, 3H), 3.36 (d, *J =* 5.2 Hz, 4H), 3.25 (s, 4H), 2.76 (d, *J =* 4.5 Hz, 3H). LCMS (ESI): calcd., 501.2; found, 501.3.

### Intermediate Example 25: preparation of 2-((2-((2-methoxy-4-(4-(methylamino)piperidin-1-yl)phenyl)amino)-5-(trifluoromethyl)pyridin-4-yl)amino)-N-methylbenzamide (GT-M-172)

2-((2-((2-methoxy-4-(4-(methylamino)piperidin-1-yl)phenyl)amino)-5-(trifluoromethyl)pyridin-4-yl)amino)-N-methylbenzamide (GT-M-172) was prepared by referring to Scheme 9 (white solid, 1.8 g).¹H NMR (400 MHz, DMSO) δ 10.60 (s, 1H), 9.98 (s, 1H), 9.38 (s, 2H), 8.83 (d, *J =* 4.5 Hz, 1H), 8.17 (s, 1H), 7.78 (d, *J* = 7.8 Hz, 1H), 7.57 (d, *J* = 3.9 Hz, 2H), 7.34 - 7.15 (m, 2H), 6.87 (d, *J* = 54.9 Hz, 2H), 6.59 (s, 1H), 3.82 (s, 5H), 3.21 (s, 1H), 2.98 (s, 2H), 2.69 (t, *J =* 41.5 Hz, 4H), 2.54 (t, *J =* 5.3 Hz, 3H), 2.17 (s, 2H), 1.84 (s, 2H). LCMS (ESI): calcd., 529.3; found, 529.3.

### Intermediate Example 26: preparation of N-methyl-N-(3-(((2-((4-(piperazin-1-yl)phenyl)amino)-5-(trifluoromethyl)pyrimidin-4-yl)amino)methyl)pyrazin-2-yl)methanesulfonamide (GT-M-151)

N-methyl-N-(3-(((2-((4-(piperazin-1-yl)phenyl)amino)-5-(trifluoromethyl)pyrimidin-4-yl)amino)methyl)pyrazin-2-yl)methanesulfonamide (GT-M-151) was prepared by referring to Scheme 12 (white solid, 1.7 g). ¹H NMR(400 MHz, MeOD) δ 8.60 (s, 1H), 8.56 (s, 1H), 8.30 (s, 1H), 7.41 (d, *J =* 8.3 Hz, 2H), 7.20 (d, *J =* 8.6 Hz, 2H), 5.13 (s, 2H), 3.59 (d, *J =* 4.7 Hz, 4H), 3.49 (s, 4H), 3.20 (d, *J =* 16.1 Hz, 3H), 3.13 (s, 3H). LCMS (ESI): calcd., 538.20; found, 538.3.

### Intermediate Example 27: preparation of N-methyl-N-(3-(((2-((4-(piperazin-1-yl)phenyl)amino)-5-(trifluoromethyl)pyrimidin-4-yl)amino)methyl)phenyl)methanesulfonamide (GT-M-152)

N-methyl-N-(3-(((2-((4-(piperazin-1-yl)phenyl)amino)-5-(trifluoromethyl)pyrimidin-4-yl)amino)methyl)phenyl)methanesulfonamide (GT-M-152) was prepared by referring to Scheme 12 (brown solid, 1.2 g). ¹H NMR (400 MHz, DMSO) δ 10.92 (s, 1H), 9.52 (s, 2H), 9.06 (s, 1H), 8.48 (s, 1H), 7.43 - 7.25 (m, 6H), 7.18 (s, 1H), 6.97 (d, J = 8.3 Hz, 2H), 4.64 (d, J = 5.2 Hz, 2H), 3.37 (s, 4H), 3.20 (s, 4H), 3.15 (s, 3H), 2.87 (s, 3H). LCMS (ESI): calcd., 536.2; found, 536.7.

### Intermediate Example 28: preparation of N-methyl-N-(3-(((2-((4-(4-(methylamino)piperidin-1-yl)phenyl)amino)-5-(trifluoromethyl)pyrimidin-4-yl)amino)methyl)phenyl)methanesulfonamide (GT-M-153)

N-methyl-N-(3-(((2-((4-(4-(methylamino)piperidin-1-yl)phenyl)amino)-5-(trifluoromethyl)pyrimidin-4-yl)amino)methyl)phenyl)methanesulfonamide (GT-M-153) was prepared by referring to Scheme 12 (white solid, 1.069 g). ¹H NMR (400 MHz, MeOD) δ 8.36 (d, *J* = 22.7 Hz, 1H), 7.77 (t, *J* = 8.4 Hz, 2H), 7.55 (d, *J* = 8.5 Hz, 2H), 7.47 (t, *J* = 7.9 Hz, 1H), 7.35 (d, *J* = 7.8 Hz, 1H), 7.26 (s, 2H), 7.14 (s, 1H), 4.75 (s, 2H), 3.82 (dd, *J* = 24.4, 12.5 Hz, 4H), 3.63 (d, *J* = 11.4 Hz, 1H), 3.27 (d, *J* = 8.0 Hz, 3H), 2.89 (d, *J* = 18.3 Hz, 3H), 2.81 (s, 3H), 2.50 (d, *J* = 13.0 Hz, 2H), 2.37 (d, *J* = 10.7 Hz, 2H). LCMS (ESI): calcd., 564.2; found, 564.1.

### Intermediate Example 29: preparation of N-methyl-N-(3-(((2-((4-(4-(methylamino)piperidin-1-yl)phenyl)amino)-5-(trifluoromethyl)pyrimidin-4-yl)amino)methyl)pyrazin-2-yl)methanesulfonamide (GT-M-174)

N-methyl-N-(3-(((2-((4-(4-(methylamino)piperidin-1-yl)phenyl)amino)-5-(trifluoromethyl)pyrimidin-4-yl)amino)methyl)pyrazin-2-yl)methanesulfonamide (GT-M-174) was prepared by referring to Scheme 12 (white solid, 707 mg). ¹H NMR (400 MHz, MeOD) δ 8.58 (dd, *J* = 17.7, 2.3 Hz, 2H), 8.39 (s, 1H), 7.76 (dd, *J* = 44.2, 9.0 Hz, 4H), 5.16 (s, 2H), 3.83 (d, *J* = 13.7 Hz, 4H), 3.27 (s, 3H), 3.16 (s, 3H), 2.80 (s, 3H), 2.55 - 2.33 (m, 4H). LCMS (ESI): calcd., 566.2; found, 566.3.

### Intermediate Example 30: preparation of N-methyl-N-(3-(((2-((4-(4-(piperazin-1-yl)piperidin-1-yl)phenyl)amino)-5-(trifluoromethyl)pyrimidin-4-yl)amino)methyl)pyrazin-2-yl)methanesulfonamide (GT-M-175)

N-methyl-N-(3-(((2-((4-(4-(piperazin-1-yl)piperidin-1-yl)phenyl)amino)-5-(trifluoromethyl)pyrimidin-4-yl)amino)methyl)pyrazin-2-yl)methanesulfonamide (GT-M-175) was prepared by referring to Scheme 12 (brown solid, 560 mg). ¹H NMR (400 MHz, MeOD) δ 8.58 (dd, *J =* 18.6, 2.4 Hz, 1H), 8.37 (s, 1H), 7.66 (s, 2H), 5.15 (s, 1H), 4.00 - 3.60 (m, 6H), 3.26 (s, 2H), 3.16 (s, 2H), 2.54 (dd, *J* = 43.4, 11.6 Hz, 2H). LCMS (ESI): calcd., 621.3; found, 621.4.

### Intermediate Example 31: preparation of N⁴-(3-(methylsulfonyl)benzyl)-N²-(4-(piperazin-1-yl)phenyl)-5-(trifluoromethyl)pyrimidine-2,4-diamine (GT-M-191)

N⁴-(3-(methylsulfonyl)benzyl)-N²-(4-(piperazin-1-yl)phenyl)-5-(trifluoromethyl)pyrimidine-2,4-diamine (GT-M-191) was prepared by referring to Scheme 12 (purple solid, 500 mg). ¹H NMR (400 MHz, DMSO) δ 9.78 (s, 1H), 8.62 (s, 3H), 8.26 (s, 1H), 7.90 (s, 1H), 7.81 (d, *J* = 7.1 Hz, 1H), 7.61 (t, *J* = 7.6 Hz, 2H), 7.31 (d, *J* = 7.9 Hz, 2H), 6.91 (d, *J =* 8.7 Hz, 2H), 4.71 (d, *J* = 5.5 Hz, 2H), 3.35 - 3.20 (m, 8H), 3.16 (d, *J* = 11.3 Hz, 3H). LCMS (ESI): calcd., 507.2; found, 507.4.

### Intermediate Example 32: preparation of N-methyl-N-(3-(((2-((4-(piperidin-4-yl)phenyl)amino)-5-(trifluoromethyl)pyrimidin-4-yl)amino)methyl)phenyl)methanesulfonamide (GT-M-193)

N-methyl-N-(3-(((2-((4-(piperidin-4-yl)phenyl)amino)-5-(trifluoromethyl)pyrimidin-4-yl)amino)methyl)phenyl)methanesulfonamide (GT-M-193) was prepared by referring to Scheme 13 (white solid, 0.87 g). ¹H NMR (400 MHz, DMSO) δ 9.75 (s, 1H), 8.63 (s, 1H), 8.38 (s, 1H), 8.25 (s, 1H), 7.98 (s, 1H), 7.51 (d, *J =* 8.0 Hz, 2H), 7.41 - 7.31 (m, 2H), 7.24 (dd, *J =* 16.3, 7.7 Hz, 2H), 7.09 (d, *J =* 8.4 Hz, 2H), 4.66 (d, *J =* 5.6 Hz, 2H), 3.37 (d, *J =* 12.2 Hz, 2H), 3.15 (s, 3H), 3.00 (q, *J =* 11.9 Hz, 2H), 2.86 (s, 3H), 2.77 (t, *J* = 12.0 Hz, 1H), 1.92 (d, *J =* 13.5 Hz, 2H), 1.73 (dd, *J =* 22.9, 12.5 Hz, 2H). LCMS (ESI): calcd., 535.2; found, 535.3.

### Intermediate Example 33: preparation of 4-(((4-(5-chloro-2-(piperidin-4-ylamino)pyridin-4-yl)thiazol-2-yl)amino)methyl)tetrahydro-2H-pyran-4-carbonitrile (GT-M-158)

4-(((4-(5-chloro-2-(piperidin-4-ylamino)pyridin-4-yl)thiazol-2-yl)amino)methyl)tetrahydro-2H-pyran-4-carbonitrile (GT-M-158) was prepared by referring to Scheme 15 (yellow solid, 900 mg). ¹H NMR (400 MHz, DMSO) δ 8.52 (s, 1H), 8.34 (s, 1H), 8.12 (t, J = 6.2 Hz, 1H), 8.02 (s, 1H), 7.39 (s, 1H), 7.11 (s, 1H), 6.99 (s, 1H), 3.92 (s, 2H), 3.68 (d, J = 6.1 Hz, 2H), 3.49 (t, J = 11.3 Hz, 3H), 3.31 (d, J = 12.7 Hz, 2H), 3.03 (d, J = 10.3 Hz, 2H), 2.07 (d, J = 10.8 Hz, 2H), 1.88 (d, J = 13.4 Hz, 2H), 1.77 - 1.59 (m, 4H). LCMS (ESI): calcd for C₂₀H₂₅ClN₆OS⁺ [M+H]⁺: 433.16; found, 433.1.

### Intermediate Example 34: preparation of 4-(((5'-chloro-2'-(piperidin-4-ylamino)-[2,4'-bipyridin]-6-yl)amino)methyl)tetrahydro-2H-pyran-4-carbonitrile (GT-M-159)

4-(((5'-chloro-2'-(piperidin-4-ylamino)-[2,4'-bipyridin]-6-yl)amino)methyl)tetrahydro-2H-pyran-4-carbonitrile (GT-M-159) was prepared by referring to Scheme 14 (yellow solid, 1.5 g, yield 86.9%). ¹H NMR (400 MHz, DMSO) δ 8.55 (s, 1H), 8.39 (s, 1H), 8.04 (s, 1H), 7.57 - 7.43 (m, 1H), 7.10 (t, *J =* 6.4 Hz, 1H), 6.95 (d, *J* = 7.3 Hz, 1H), 6.76 (d, *J =* 7.2 Hz, 1H), 6.67 (d, *J =* 10.0 Hz, 2H), 4.08 - 3.80 (m, 3H), 3.66 (d, *J =* 6.4 Hz, 2H), 3.46 (dd, *J =* 11.9, 10.5 Hz, 2H), 3.29 (s, 2H), 3.04 (dd, *J =* 18.8, 7.9 Hz, 2H), 2.07 (d, *J =* 10.8 Hz, 2H), 1.84 (d, *J =* 13.2 Hz, 2H), 1.73 - 1.46 (m, 4H). LCMS (ESI): calcd., 427.2; found, 427.2.

### Intermediate Example 35: preparation of 2-(5-fluoro-2-hydroxyphenyl)-2-(1-oxo-6-(4-(piperazin-1-yl)phenyl)isoindolin-2-yl)-N-(thiazol-2-yl)acetamide (GT-D-012)

2-(5-fluoro-2-hydroxyphenyl)-2-(1-oxo-6-(4-(piperazin-1-yl)phenyl)isoindolin-2-yl)-N-(thiazol-2-yl)acetamide (GT-D-012) was prepared by referring to Scheme 16 (yellow solid, 110 mg). ¹H NMR (400 MHz, DMSO) δ 10.20 - 8.61 (m, 1H), 7.87 (d, *J =* 8.5 Hz, 2H), 7.63 (dd, *J =* 11.3, 8.4 Hz, 4H), 7.49 (d, *J =* 3.5 Hz, 1H), 7.27 (d, *J =* 3.6 Hz, 1H), 7.12 (td, *J =* 8.6, 3.1 Hz, 1H), 7.07 (d, *J =* 8.8 Hz, 2H), 6.92 (dd, *J =* 8.9, 4.8 Hz, 1H), 6.87 (dd, *J* = 9.2, 3.0 Hz, 1H), 6.33 (s, 1H), 4.64 (d, *J =* 17.5 Hz, 1H), 4.02 (d, *J =* 17.5 Hz, 1H), 3.29 (d, *J =* 4.4 Hz, 5H), 3.12 - 3.00 (m, 6H), 1.24 (s, 1H). LCMS (ESI): calcd., 544.2; found, 544.2.

### Intermediate Example 36: preparation of 2-(5-fluoro-2-hydroxyphenyl)-2-(1-oxo-6-(6-(piperazin-1-yl)pyridazin-3-yl)isoindolin-2-yl)-N-(thiazol-2-yl)acetamide (GT-D-014)

2-(5-fluoro-2-hydroxyphenyl)-2-(1-oxo-6-(6-(piperazin-1-yl)pyridazin-3-yl)isoindolin-2-yl)-N-(thiazol-2-yl)acetamide (GT-D-014) was prepared by referring to Scheme 17 (brown solid, 130 mg). ¹H NMR (400 MHz, DMSO) δ 8.31 (d, *J =* 8.3 Hz, 2H), 8.06 (d, *J =* 9.6 Hz, 1H), 7.68 (d, *J =* 7.9 Hz, 1H), 7.48 (d, *J* = 3.6 Hz, 1H), 7.33 (d, *J =* 9.7 Hz, 1H), 7.25 (d, *J =* 3.5 Hz, 1H), 7.11 (td, *J =* 8.6, 3.1 Hz, 1H), 6.94 - 6.84 (m, 2H), 6.33 (s, 1H), 4.69 (d, *J =* 17.8 Hz, 1H), 4.07 (d, *J =* 17.8 Hz, 1H), 3.66 - 3.54 (m, 4H), 3.17 (s, 1H), 2.91 - 2.78 (m, 4H), 1.23 (s, 1H). LCMS (ESI): calcd., 546.2; found, 546.2.

### Intermediate Example 37: preparation of 2-(5-fluoro-2-hydroxyphenyl)-2-(1-oxo-6-(piperazin-1-yl)isoindolin-2-yl)-N-(thiazol-2-yl)acetamide (GT-D-69)

2-(5-fluoro-2-hydroxyphenyl)-2-(1-oxo-6-(piperazin-1-yl)isoindolin-2-yl)-N-(thiazol-2-yl)acetamide (GT-D-69) was prepared by referring to Scheme 18 (yellow solid, 515 mg). ¹H NMR (400 MHz, DMSO) δ 7.48 (d, *J* = 3.5 Hz, 1H), 7.39 (d, *J =* 8.4 Hz, 1H), 7.24 (dd, *J* = 11.9, 2.8 Hz, 2H), 7.15 (d, *J* = 1.8 Hz, 1H), 7.13 - 7.05 (m, 1H), 6.90 (dd, *J =* 8.9, 4.8 Hz, 1H), 6.82 (dd, *J* = 9.2, 2.9 Hz, 1H), 6.29 (s, 1H), 4.50 (d, *J =* 17.0 Hz, 1H), 3.89 (d, *J =* 17.0 Hz, 2H), 3.13 (s, 5H), 2.91 (s, 5H), 1.24 (s, 1H). LCMS (ESI): calcd., 468.2; found, 468.2.

### Intermediate Example 38: preparation of 2-(5-fluoro-2-hydroxyphenyl)-2-(6-(4-(methylamino)piperidin-1-yl)-1-oxoisoindolin-2-yl)-N-(thiazol-2-yl)acetamide (GT-D-70)

2-(5-fluoro-2-hydroxyphenyl)-2-(6-(4-(methylamino)piperidin-1-yl)-1-oxoisoindolin-2-yl)-N-(thiazol-2-yl)acetamide (GT-D-70) was prepared by referring to Scheme 18 (brown solid, 186 mg). ¹H NMR (400 MHz, DMSO) δ 7.40 (dd, *J =* 29.5, 5.8 Hz, 2H), 7.22 (d, *J* = 7.5 Hz, 1H), 7.16 (d, *J =* 19.7 Hz, 3H), 7.05 (d, *J =* 6.2 Hz, 1H), 6.91 - 6.76 (m, 2H), 6.24 (s, 1H), 4.54 (d, *J =* 17.0 Hz, 1H), 3.94 (d, *J =* 17.0 Hz, 1H), 3.67 (d, *J =* 12.1 Hz, 2H), 3.17 (s, 1H), 2.78 (t, *J =* 11.3 Hz, 2H), 2.31 (d, *J =* 5.8 Hz, 3H), 1.89 (d, *J=* 11.2 Hz, 2H), 1.35 (d, *J =* 11.1 Hz, 2H). LCMS (ESI): calcd., 496.2; found, 496.2.

### Intermediate Example 39: preparation of 2-(5-fluoro-2-hydroxyphenyl)-2-(1-oxo-6-(4-(piperazin-1-yl)piperidin-1-yl)isoindolin-2-yl)-N-(thiazol-2-yl)acetamide (GT-D-71)

2-(5-fluoro-2-hydroxyphenyl)-2-(1-oxo-6-(4-(piperazin-1-yl)piperidin-1-yl)isoindolin-2-yl)-N-(thiazol-2-yl)acetamide (GT-D-71) was prepared by referring to Scheme 18 (brown solid, 110 mg). ¹H NMR (400 MHz, DMSO) δ 7.45 (s, 1H), 7.36 (d, *J =* 8.4 Hz, 1H), 7.22 (d, *J =* 9.5 Hz, 4H), 7.07 (s, 1H), 6.91 - 6.77 (m, 3H), 6.25 (s, 1H), 4.51 (d, *J =* 17.1 Hz, 1H), 3.91 (d, *J =* 16.9 Hz, 1H), 3.77 (d, *J =* 12.1 Hz, 3H), 3.17 (s, 2H), 2.72 (s, 5H), 2.44 (s, 5H), 1.82 (d, *J =* 10.7 Hz, 2H), 1.51 (d, *J =* 11.4 Hz, 3H), 1.25 (s, 1H). LCMS (ESI): calcd., 551.2; found, 551.2.

### Intermediate Example 40: preparation of 9-ethyl-6,6-dimethyl-11-oxo-8-(piperazin-1-yl)-6,11-dihydro-5H-benzo[b]carbazole-3-carbonitrile (GT-D-42)

9-ethyl-6,6-dimethyl-11-oxo-8-(piperazin-1-yl)-6,11-dihydro-5H-benzo[b]carbazole-3-carbonitrile (GT-D-42) was prepared by referring to Scheme 20 (white solid, 240 mg). ¹H NMR (400 MHz, DMSO) δ 13.03 (s, 1H), 9.39 (s, 2H), 8.32 (d, *J =* 8.1 Hz, 1H), 8.05 (d, *J =* 29.2 Hz, 2H), 7.61 (d, *J =* 9.0 Hz, 1H), 7.38 (s, 1H), 3.24 (d, *J* = 14.9 Hz, 8H), 2.73 (d, *J =* 7.5 Hz, 2H), 1.79 (s, 6H), 1.29 (t, *J =* 7.5 Hz, 3H). LCMS (ESI): calcd., 399.2; found, 399.2.

### Intermediate Example 41: preparation of 9-ethyl-6,6-dimethyl-11-oxo-8-(4-(piperazin-1-yl)piperidin-1-yl)-6,11-dihydro-5H-benzo[b]carbazole-3-carbonitrile (GT-D-113)

9-ethyl-6,6-dimethyl-11-oxo-8-(4-(piperazin-1-yl)piperidin-1-yl)-6,11-dihydro-5H-benzo[b]carbazole-3-carbonitrile (GT-D-113) was prepared by referring to Scheme 20 (off-white solid, 1.4 g). ¹H NMR (400 MHz, MeOD) δ 8.39 (d, *J* = 8.2 Hz, 1H), 8.23 (s, 1H), 7.86 (s, 1H), 7.62 - 7.48 (m, 2H), 3.75 (s, 9H), 3.53 (d, *J =* 12.0 Hz, 2H), 3.18 (t, *J =* 11.7 Hz, 2H), 2.86 (q, *J =* 7.4 Hz, 2H), 2.43 (d, *J =* 11.4 Hz, 2H), 2.22 (dd, *J* = 20.7, 11.9 Hz, 2H), 1.82 (s, 6H), 1.37 (t, *J* = 7.5 Hz, 3H). LCMS (ESI): calcd., 482.3; found, 482.2.

### Intermediate Example 42: preparation of 9-ethyl-6,6-dimethyl-8-(4-(methylamino)piperidin-1-yl)-11-oxo-6,11-dihydro-5H-benzo[b]carbazole-3-carbonitrile (GT-D-85)

9-ethyl-6,6-dimethyl-8-(4-(methylamino)piperidin-1-yl)-11-oxo-6,11-dihydro-5H-benzo[b]carbazole-3-carbonitrile (GT-D-85) was prepared by referring to Scheme 20 (white solid, 130 mg). ¹H NMR (400 MHz, MeOD) δ 8.31 (d, *J =* 8.2 Hz, 1H), 8.11 (s, 1H), 7.77 (s, 1H), 7.45 (d, *J =* 8.2 Hz, 1H), 7.36 (s, 1H), 3.29 (d, *J =* 11.9 Hz, 2H), 2.91 (t, *J =* 11.7 Hz, 2H), 2.78 - 2.64 (m, 5H), 2.18 (d, *J =* 11.7 Hz, 2H), 1.86 - 1.75 (m, 2H), 1.71 (s, 6H), 1.25 (t, *J* = 7.5 Hz, 3H), 1.22 - 1.18 (m, 1H). LCMS (ESI): calcd., 427.3; found, 427.2.

### Intermediate Example 43: preparation of 9-cyclopentyl-N⁸-phenyl-N²-(4-(piperazin-1-yl)phenyl)-9H-purine-2,8-diamine (GT-D-62)

9-cyclopentyl-N⁸-phenyl-N²-(4-(piperazin-1-yl)phenyl)-9H-purine-2,8-diamine (GT-D-62) was prepared by referring to Scheme 22 (white solid, 1.18 g). ¹H NMR (400 MHz, MeOD) δ 8.15 (s, 1H), 7.67 - 7.52 (m, 4H), 7.46 (dd, *J* = 15.9, 8.0 Hz, 3H), 7.25 (d, *J* = 9.0 Hz, 2H), 3.66 (s, 1H), 3.61 - 3.53 (m, 4H), 3.47 (dd, *J* = 6.5, 3.6 Hz, 4H), 2.40 (dd, *J* = 12.8, 7.3 Hz, 2H), 2.20 (d, *J* = 7.9 Hz, 2H), 1.92 (s, 2H), 1.77 - 1.62 (m, 2H). LCMS (ESI): calcd., 455.3; found, 455.0.

### Intermediate Example 44: preparation of 9-cyclopentyl-N²-(4-(4-(methylamino)piperidin-1-yl)phenyl)-N⁸-phenyl-9H-purine-2,8-diamine (GT-D-67)

9-cyclopentyl-N²-(4-(4-(methylamino)piperidin-1-yl)phenyl)-N³-phenyl-9H-purine-2,8-diamine (GT-D-67) was prepared by referring to Scheme 22 (yellow solid, 4.6 g). ¹H NMR (400 MHz, MeOD) δ 8.34 (s, 1H), 7.94 - 7.82 (m, 4H), 7.57 (dd, *J* = 7.4, 6.3 Hz, 4H), 7.45 (dd, *J* = 7.7, 4.2 Hz, 1H), 3.95 - 3.81 (m, 4H), 3.71 - 3.60 (m, 2H), 2.89 (s, 1H), 2.81 (s, 3H), 2.51 (t, *J =* 15.4 Hz, 4H), 2.43 - 2.37 (m, 2H), 2.25 (d, *J* = 8.3 Hz, 2H), 2.09 - 2.02 (m, 2H), 1.83 - 1.71 (m, 2H). LCMS (ESI): calcd., 483.3; found, 483.3.

### Intermediate Example 45: preparation of 9-cyclopentyl-N⁸-phenyl-N²-(4-(4-(piperazin-1-yl)piperidin-1-yl)phenyl)-9H-purine-2,8-diamine (GT-D-68)

9-cyclopentyl-N³-phenyl-N²-(4-(4-(piperazin-1-yl)piperidin-1-yl)phenyl)-9H-purine-2,8-diamine (GT-D-68) was prepared by referring to Scheme 22 (yellow solid, 4.1 g). ¹H NMR(400 MHz, DMSO) δ 7.62 (d, *J = 8.9* Hz, 2H), 7.56 (t, *J =* 3.7 Hz, 3H), 7.01 - 6.94 (m, 2H), 6.80 (d, *J* = 9.0 Hz, 2H), 6.42 (t, *J* = 7.1 Hz, 1H), 3.53 (d, *J* = 11.9 Hz, 2H), 2.67 (s, 4H), 2.54 (d, *J* = 10.1 Hz, 2H), 2.42 (s, 2H), 2.38 - 2.31 (m, 6H), 2.20 (t, *J* = 11.2 Hz, 1H), 1.99 (s, 2H), 1.92 - 1.87 (m, 1H), 1.81 (d, *J* = 10.0 Hz, 4H), 1.69 - 1.61 (m, 2H), 1.52 (dt, *J =* 11.7, 8.7 Hz, 2H). LCMS (ESI): calcd., 538.3; found, 538.4.

### Intermediate Example 46: preparation of 2-(4-phenoxyphenyl)-6-(piperidin-4-yl)nicotinamide (GT-D-66)

2-(4-phenoxyphenyl)-6-(piperidin-4-yl)nicotinamide (GT-D-66) was prepared by referring to Scheme 23 (white solid, 1.0 g). ¹H NMR (400 MHz, MeOD) δ 8.53 (d, *J =* 8.2 Hz, 1H), 7.93 (d, *J =* 8.2 Hz, 1H), 7.75 (d, *J =* 8.8 Hz, 2H), 7.44 (t, *J =* 8.0 Hz, 2H), 7.23 (t, *J =* 7.4 Hz, 1H), 7.18 - 7.04 (m, 4H), 3.60 (d, *J* = 12.7 Hz, 2H), 3.52 (t, *J =* 12.0 Hz, 1H), 3.24 (t, *J =* 11.8 Hz, 2H), 2.31 (d, *J =* 13.5 Hz, 2H), 2.26 - 2.09 (m, 2H). LCMS (ESI): calcd., 374.2; found, 374.2.

### Intermediate Example 47: preparation of 2-(4-phenoxyphenyl)-6-(piperazin-1-yl)nicotinamide (GT-D-76)

2-(4-phenoxyphenyl)-6-(piperazin-1-yl)nicotinamide (GT-D-76) was prepared by referring to Scheme 23 (yellow solid, 436 mg). ¹H NMR (400 MHz, MeOD) δ 8.14 (d, *J =* 9.2 Hz, 1H), 7.69 (d, *J =* 8.7 Hz, 2H), 7.47 - 7.33 (m, 3H), 7.20 (t, *J =* 7.4 Hz, 1H), 7.09 (t, *J =* 7.8 Hz, 4H), 4.12 - 4.03 (m, 4H), 3.49 - 3.43 (m, 5H). LCMS (ESI): calcd., 375.2; found, 375.2.

### Intermediate Example 48: preparation of 6-(4-(methylamino)piperidin-1-yl)-2-(4-phenoxyphenyl)nicotinamide (GT-D-77)

6-(4-(methylamino)piperidin-1-yl)-2-(4-phenoxyphenyl)nicotinamide (GT-D-77) was prepared by referring to Scheme 23 (white solid, 690 m). ¹H NMR (400 MHz, CDCl₃) δ 7.99 (d, *J* = 8.9 Hz, 1H), 7.65 (d, *J =* 8.6 Hz, 2H), 7.39 (t, *J =* 7.9 Hz, 2H), 7.17 (t, *J =* 7.4 Hz, 1H), 7.08 (dd, *J =* 14.0, 8.2 Hz, 4H), 6.65 (d, *J =* 8.9 Hz, 1H), 5.50 - 5.19 (m, 2H), 4.42 (d, *J =* 13.4 Hz, 2H), 3.18 - 2.90 (m, 2H), 2.78 - 2.58 (m, 1H), 2.00 (d, *J =* 10.2 Hz, 2H), 1.52 - 1.25 (m, 4H). LCMS (ESI): calcd., 403.2; found, 403.2.

### Intermediate Example 49: preparation of 2-(4-phenoxyphenyl)-6-(4-(piperazin-1-yl)piperidin-1-yl)nicotinamide (GT-D-78)

2-(4-phenoxyphenyl)-6-(4-(piperazin-1-yl)piperidin-1-yl)nicotinamide (GT-D-78) was prepared by referring to Scheme 23 (white solid, 355 mg). ¹H NMR (400 MHz, CDCl₃) δ 7.89 (d, *J =* 8.8 Hz, 1H), 7.55 (d, *J =* 8.7 Hz, 2H), 7.30 (t, *J =* 8.0 Hz, 2H), 7.09 (d, *J =* 7.4 Hz, 1H), 7.04 - 6.91 (m, 4H), 6.56 (d, *J =* 8.9 Hz, 1H), 5.41 (s, 1H), 5.21 (d, *J =* 12.3 Hz, 1H), 4.45 (d, *J =* 13.2 Hz, 2H), 2.95 - 2.76 (m, 7H), 2.57 (d, *J =* 4.4 Hz, 4H), 2.44 (dd, *J* = 15.1, 7.5 Hz, 1H), 1.85 (d, *J =* 11.8 Hz, 2H), 1.46 (qd, *J =* 12.4, 3.9 Hz, 2H). LCMS (ESI): calcd., 458.3; found, 458.2.

### Intermediate Example 50: preparation of N-(2-(1-(cyclopropylsulfonyl)-1H-pyrazol-4-yl)pyrimidin-4-yl)-1-isopropyl-3-(piperazin-1-yl)-1H-pyrazolo[4,3-c]pyridin-6-amine (GT-D-18)

N-(2-(1-(cyclopropylsulfonyl)-1H-pyrazol-4-yl)pyrimidin-4-yl)-1-isopropyl-3-(piperazin-1-yl)-1H-pyrazolo[4,3-c]pyridin-6-amine (GT-D-18) was prepared by referring to Scheme 19 (yellow solid, 0.75 g). ¹H NMR (400 MHz, CDCl₃) δ 8.73 (s, 1H), 8.69 (s, 1H), 8.47 (s, 1H), 8.39 (d, *J =* 5.9 Hz, 1H), 8.12 (s, 1H), 7.97 (s, 1H), 6.81 (d, *J =* 5.8 Hz, 1H), 4.65 (dt, *J =* 13.3, 6.6 Hz, 1H), 3.56 - 3.43 (m, 4H), 3.16 - 3.00 (m, 4H), 2.90 - 2.80 (m, 1H), 1.58 (d, *J =* 6.7 Hz, 6H), 1.55 - 1.49 (m, 2H), 1.26 - 1.19 (m, 2H). LCMS (ESI): calcd., 509.2; found, 509.2.

### Intermediate Example 51: preparation of N-(2-(1-(cyclopropylsulfonyl)-1H-pyrazol-4-yl)pyrimidin-4-yl)-1-isopropyl-3-(4-(methylamino)piperidin-1-yl)-1H-pyrazolo[4,3-c]pyridin-6-amine (GT-D-72)

N-(2-(1-(cyclopropylsulfonyl)-1H-pyrazol-4-yl)pyrimidin-4-yl)-1-isopropyl-3-(4-(methylamino)piperidin-1-yl)-1H-pyrazolo[4,3-c]pyridin-6-amine (GT-D-72) was prepared by referring to Scheme 19 (yellow solid, 0.4 g). ¹H NMR (400 MHz, CDCl3) δ 8.73 (s, 1H), 8.69 (s, 1H), 8.47 (s, 1H), 8.40 (d, *J* = 5.9 Hz, 1H), 8.11 (s, 1H), 7.80 (s, 1H), 6.82 (d, *J =* 5.8 Hz, 1H), 4.65 (dt, *J =* 13.3, 6.6 Hz, 1H), 4.09 (t, *J* = 12.3 Hz, 2H), 3.05 (t, *J =* 11.3 Hz, 2H), 2.85 (ddd, *J =* 12.5, 7.8, 4.7 Hz, 2H), 2.61 (s, 3H), 2.16 (d, *J =* 10.7 Hz, 2H), 1.81 (dd, *J =* 20.3, 11.2 Hz, 2H), 1.58 (d, *J =* 6.7 Hz, 6H), 1.54 (dd, *J =* 5.8, 3.7 Hz, 2H), 1.23 (dt, *J =* 7.0, 3.6 Hz, 2H). LCMS (ESI): calcd., 537.3; found, 537.4.

### Intermediate Example 52: preparation of N-(2-(1-(cyclopropylsulfonyl)-1H-pyrazol-4-yl)pyrimidin-4-yl)-1-isopropyl-3-(4-(piperazin-1-yl)piperidin-1-yl)-1H-pyrazolo[4,3-c]pyridin-6-amine (GT-D-73)

N-(2-(1-(cyclopropylsulfonyl)-1H-pyrazol-4-yl)pyrimidin-4-yl)-1-isopropyl-3-(4-(piperazin-1-yl)piperidin-1-yl)-1H-pyrazolo[4,3-c]pyridin-6-amine (GT-D-73) was prepared by referring to Scheme 19 (yellow solid, 1.19 g). ¹H **NMR** (400 MHz, DMSO) δ 10.37 (s, 1H), 8.86 (s, 1H), 8.70 (s, 1H), 8.48 (s, 1H), 8.41 (d, *J =* 5.9 Hz, 1H), 8.32 (s, 1H), 7.13 (d, *J =* 5.5 Hz, 1H), 4.72 (s, 1H), 4.02 (d, *J =* 12.0 Hz, 2H), 3.53 (d, *J* = 57.7 Hz, 1H), 3.33 - 3.27 (m, 2H), 2.88 (t, *J =* 11.6 Hz, 2H), 2.76 (s, 4H), 2.35 (d, *J =* 12.0 Hz, 1H), 1.87 (d, *J =* 10.4 Hz, 2H), 1.60 (d, *J =* 10.0 Hz, 2H), 1.47 (d, *J =* 6.6 Hz, 6H), 1.36 (s, 2H), 1.28 (dd, *J* = 7.5, 5.1 Hz, 2H). LCMS (ESI): calcd., 592.3; found, 592.4.

### Intermediate Example 53: preparation of 1-isopropyl-N-(2-(4-methoxypiperidin-1-yl)pyrimidin-4-yl)-3-(4-(methylamino)piperidin-1-yl)-1H-pyrazolo[4,3-c]pyridin-6-amine (GT-D-74)

1-isopropyl-N-(2-(4-methoxypiperidin-1-yl)pyrimidin-4-yl)-3-(4-(methylamino)piperidin-1-yl)-1H-pyrazolo[4,3-c]pyridin-6-amine (GT-D-74) was prepared by referring to Scheme 19 (yellow solid, 0.97 g). ¹H **NMR** (400 MHz, MeOD) δ 9.38 (s, 1H), 8.03 (d, *J =* 7.1 Hz, 1H), 7.80 (s, 1H), 6.59 (d, *J =* 7.1 Hz, 1H), 4.89 (s, 1H), 4.30 (d, *J =* 13.0 Hz, 2H), 3.89 (d, *J =* 8.7 Hz, 2H), 3.67 (d, *J =* 15.3 Hz, 2H), 3.59 (s, 1H), 3.39 (s, 3H), 3.37 - 3.32 (m, 1H), 3.20 (s, 2H), 2.77 (s, 3H), 2.27 (d, *J =* 11.6 Hz, 2H), 2.07 - 1.94 (m, 2H), 1.83 (ddd, *J =* 33.0, 16.1, 8.8 Hz, 4H), 1.54 (d, *J =* 6.5 Hz, 6H). LCMS (ESI): calcd., 480.3, found, 480.4.

### Intermediate Example 54: preparation of 1-isopropyl-N-(2-(4-methoxypiperidin-1-yl)pyrimidin-4-yl)-3-(4-(piperazin-1-yl)piperidin-1-yl)-1H-pyrazolo[4,3-c]pyridin-6-amine (GT-D-75)

1-isopropyl-N-(2-(4-methoxypiperidin-1-yl)pyrimidin-4-yl)-3-(4-(piperazin-1-yl)piperidin-1-yl)-1H-pyrazolo[4,3-c]pyridin-6-amine (GT-D-75) was prepared by referring to Scheme 19 (yellow solid, 1.1 g). ¹H NMR(400 MHz, MeOD) δ 9.40 (s, 1H), 8.05 (d, *J =* 7.0 Hz, 1H), 7.83 (s, 1H), 6.61 (d, *J =* 7.0 Hz, 1H), 4.39 (d, *J =* 12.7 Hz, 2H), 3.93 (s, 3H), 3.67 (dd, *J =* 33.6, 15.5 Hz, 12H), 3.41 (s, 3H), 3.23 (t, *J =* 12.4 Hz, 2H), 2.41 (d, *J =* 10.4 Hz, 2H), 2.07 (dd, *J =* 23.8, 8.9 Hz, 4H), 1.79 (s, 2H), 1.57 (d, *J =* 6.4 Hz, 6H). LCMS (ESI): calcd., 535.4; found, 535.5.

### Intermediate Example 55: preparation of 7-cyclopentyl-N,N-dimethyl-2-((5-(4-(methylamino)piperidin-1-yl)pyridin-2-yl)amino)-7H-pyrrolo[2,3-d]pyrimidine-6-carboxamide (GT-D-79)

7-cyclopentyl-N,N-dimethyl-2-((5-(4-(methylamino)piperidin-1-yl)pyridin-2-yl)amino)-7H-pyrrolo[2,3-d]pyrimidine-6-carboxamide (GT-D-79) was prepared by referring to Scheme 9 (yellow solid, 800 mg). ¹H NMR (400 MHz, DMSO) δ 9.38 (s, 1H), 8.77 (s, 1H), 8.15 (d, *J* = 9.1 Hz, 1H), 8.03 (d, *J* = 2.9 Hz, 1H), 7.45 (dd, *J =* 9.1, 3.0 Hz, 1H), 6.60 (s, 1H), 4.74 (p, *J =* 8.8 Hz, 1H), 3.67 (d, *J =* 12.5 Hz, 2H), 3.51 (s, 1H), 3.06 (s, 6H), 2.90 - 2.80 (m, 1H), 2.72 (t, *J* = 11.1 Hz, 2H), 2.47 (s, 3H), 2.43 (d, *J* = 11.5 Hz, 2H), 2.05 - 1.94 (m, 6H), 1.67 - 1.51 (m, 4H). LCMS (ESI): calcd., 463.3; found, 463.2.

### Intermediate Example 56: preparation of 7-cyclopentyl-N,N-dimethyl-2-((5-(4-(piperazin-1-yl)piperidin-1-yl)pyridin-2-yl)amino)-7H-pyrrolo[2,3-d]pyrimidine-6-carboxamide (GT-D-80)

7-cyclopentyl-N,N-dimethyl-2-((5-(4-(piperazin-1-yl)piperidin-1-yl)pyridin-2-yl)amino)-7H-pyrrolo[2,3-d]pyrimidine-6-carboxamide (GT-D-80) was prepared by referring to Scheme 9 (off-white solid, 305 mg). ¹H NMR (400 MHz, DMSO) δ 9.22 (s, 1H), 8.74 (s, 1H), 8.12 (d, *J* = 9.2 Hz, 1H), 7.98 (d, *J* = 2.8 Hz, 1H), 7.42 (dd, *J* = 9.1, 2.9 Hz, 1H), 6.59 (s, 1H), 4.80 - 4.66 (m, 1H), 3.05 (s, 6H), 2.70 - 2.66 (m, 4H), 2.63 (d, *J* = 11.8 Hz, 2H), 2.42 (s, 6H), 2.24 (d, *J* = 11.4 Hz, 1H), 1.97 (s, 6H), 1.84 (d, *J* = 11.2 Hz, 2H), 1.64 (s, 2H), 1.58 - 1.50 (m, 2H), 1.23 (s, 1H). LCMS (ESI): calcd., 518.3; found, 518.4.

### Intermediate Example 57: preparation of 5,7-dimethoxy-2-(4-(piperazin-1-yl)phenyl)quinazolin-4(3H)-one (GT-D-51)

5,7-dimethoxy-2-(4-(piperazin-1-yl)phenyl)quinazolin-4(3H)-one (GT-D-51) was prepared by referring to Scheme 21 (light yellow solid, 1.0 g). ¹H NMR (400 MHz, DMSO) δ 9.40 (s, 2H), 8.14 (d, *J =* 8.8 Hz, 2H), 7.15 (d, *J =* 8.9 Hz, 2H), 7.02 (s, 1H), 6.61 (s, 1H), 3.90 (d, *J =* 7.5 Hz, 6H), 3.87 (s, 1H), 3.65 - 3.25 (m, 8H). LCMS (ESI): calcd., 367.2; found, 367.4.

### Intermediate Example 58: preparation of 5,7-dimethoxy-2-(4-(4-(methylamino)piperidin-1-yl)phenyl)quinazolin-4(3H)-one (GT-D-93)

5,7-dimethoxy-2-(4-(4-(methylamino)piperidin-1-yl)phenyl)quinazolin-4(3H)-one (GT-D-93) was prepared by referring to Scheme 21 (yellow solid, 0.64 g). ¹H NMR (400 MHz, MeOD) δ 7.95 (d, *J* = 8.9 Hz, 2H), 7.10 (d, *J* = 9.0 Hz, 2H), 6.77 (d, *J* = 2.1 Hz, 1H), 6.54 (d, *J* = 2.1 Hz, 1H), 4.08 (d, *J* = 13.1 Hz, 2H), 3.92 *(d, J* = 3.2 Hz, 6H), 2.97 (t, *J* = 11.9 Hz, 2H), 2.74 (s, 3H), 2.20 (d, *J* = 10.4 Hz, 2H), 1.70 (tt, *J* = 12.2, 6.2 Hz, 2H). LCMS (ESI): calcd., 395.2; found, 395.3.

### Intermediate Example 59: preparation of 5,7-dimethoxy-2-(4-(4-(piperazin-1-yl)piperidin-1-yl)phenyl)quinazolin-4(3H)-one (GT-D-94)

5,7-dimethoxy-2-(4-(4-(piperazin-1-yl)piperidin-1-yl)phenyl)quinazolin-4(3H)-one (GT-D-94)was prepared by referring to Scheme 21 (yellow solid, 0.26 g). ¹H NMR (400 MHz, DMSO) δ 11.74 (s, 1H), 8.90 (s, 1H), 8.09 (d, *J* = 8.8 Hz,2H), 7.03 (t, *J* = 10.9 Hz, 2H), 6.68 (d, *J* = 2.0 Hz, 1H), 6.47 (d, *J* = 1.9 Hz, 1H), 3.96 (d, *J =* 12.5 Hz, 2H), 3.88 (s, 3H), 3.84 (s, 3H), 3.05 (s, 4H), 2.83 (t, *J =* 11.6 Hz, 2H), 2.71 (s, 4H), 1.83 (d, *J* = 11.1 Hz, 2H), 1.47 (d, *J* = 9.3 Hz, 2H). LCMS (ESI): calcd., 450.3; found, 450.3.

### Intermediate Example 60: preparation of N-(3-(difluoromethyl)-1-(piperidin-4-yl)-1H-pyrazol-4-yl)-5-morpholinopyrazolo[1,5-a]pyrimidine-3-carboxamide hydrochloride (GT-D-101)

N-(3-(difluoromethyl)-1-(piperidin-4-yl)-1H-pyrazol-4-yl)-5-morpholinopyrazolo[1,5-a]pyrimidine-3-carboxamide hydrochloride (GT-D-101) was prepared by referring to Scheme 48 (yellow solid, 1.3 g).¹H **NMR** (400 MHz, MeOD) δ 8.55 (d, *J* = 8.0 Hz, 1H), 8.42 (s, 1H), 8.32 (s, 1H), 7.03 - 6.75 (m, 2H), 4.60 (s, 1H), 3.86 - 3.80 (m, 8H), 3.58 (dd, *J* = 10.0, 6.5 Hz, 2H), 3.27 - 3.17 (m, 2H), 2.39 - 2.24 (m, 4H). LCMS (ESI): calcd., 447.2; found, 447.2.

### Intermediate Example 61: preparation of 1-(4-(4-chloro-1,2-diphenylbut-1-en-1-yl)phenyl)piperazine (GT-D-104)

1-(4-(4-chloro-1,2-diphenylbut-1-en-1-yl)phenyl)piperazine (GT-D-104) was prepared by referring to Scheme 24 (yellow solid, 1.6 g). ¹H **NMR** (400 MHz, DMSO) δ 7.39 (d, *J =* 7.5 Hz, 2H), 7.29 (dd, *J =* 8.4, 7.0 Hz, 3H), 7.26 - 7.21 (m, 2H), 7.20 - 7.16 (m, 3H), 6.70 (d, *J* = 8.9 Hz, 2H), 6.63 (d, *J* = 9.0 Hz, 2H), 3.42 *(t, J* = 7.3 Hz, 2H), 3.21 - 3.14 (m, 4H), 3.08 - 2.99 (m, 4H), 2.84 (s, 2H). LCMS (ESI): calcd., 403.2; found, 403.2.

### Intermediate Example 62: preparation of 4,4'-(4-chloro-1-(4-(piperazin-1-yl)phenyl)but-1-ene-1,2-diyl)diphenol (GT-D-106)

4,4'-(4-chloro-1-(4-(piperazin-1-yl)phenyl)but-1-ene-1,2-diyl)diphenol (GT-D-106) was prepared by referring to Scheme 25 (brown solid, 0.98 g). ¹H NMR (400 MHz, DMSO) δ 9.37 (d, *J* = 50.2 Hz, 2H), 8.70 (s, 1H), 6.97 (dd, *J* = 34.1, 8.4 Hz, 4H), 6.77 - 6.58 (m, 6H), 6.52 - 6.40 (m, 1H), 4.10 (d, *J* = 4.9 Hz, 1H), 3.43 (t, *J* = 6.5 Hz, 2H), 3.36 (s, 1H), 3.22 (d, *J* = 4.3 Hz, 4H), 3.16 (dd, *J* = 10.2, 4.0 Hz, 4H), 2.81 (t, *J* = 7.3 Hz, 2H). LCMS (ESI): calcd., 435.2; found, 435.2.

### Intermediate Example 63: preparation of (Z)-4-(1-(4-(2-aminoethoxy)phenyl)-4-chloro-2-phenylbut-1-en-1-yl)phenol (GT-D-186)

(Z)-4-(1-(4-(2-aminoethoxy)phenyl)-4-chloro-2-phenylbut-1-en-1-yl)phenol (GT-D-186) was prepared by referring to Scheme 26 (light yellow solid, 100 mg). ¹H NMR (400 MHz, DMSO) δ 7.19 (dt, *J =* 14.2, 7.6 Hz, 6H), 7.07 (d, *J =* 8.5 Hz, 1H), 6.96 (d, *J =* 8.7 Hz, 1H), 6.75 (dd, *J =* 17.5, 8.6 Hz, 2H), 6.61 (dd, *J* = 8.7, 6.6 Hz, 2H), 6.42 (d, *J* = 8.6 Hz, 1H), 3.97 (t, *J* = 5.6 Hz, 1H), 3.79 (t, *J =* 5.6 Hz, 1H), 3.44 (t, *J* = 7.4 Hz, 2H), 2.97 - 2.78 (m, 4H). LCMS (ESI): calcd., 394.2; found, 394.2.

### Intermediate Example 64: preparation of 3-(imidazo[1,2-b]pyridazin-3-ylethynyl)-4-methyl-N-(4-(piperazin-1-ylmethyl)-3-(trifluoromethyl)phenyl)benzamide hydrochloride (GT-D-109)

3-(imidazo[1,2-b]pyridazin-3-ylethynyl)-4-methyl-N-(4-(piperazin-1-ylmethyl)-3-(trifluoromethyl)phenyl)benzamide hydrochloride (GT-D-109) was prepared by referring to Scheme 28 (off-white solid, 670 mg). ¹H NMR (400 MHz, MeOD) δ 9.10 (dd, *J* = 4.5, 1.3 Hz, 1H), 8.62 (s, 1H), 8.52 (dd, *J* = 9.3, 1.3 Hz, 1H), 8.39 (d, *J* = 1.8 Hz, 1H), 8.24 (d, *J* = 1.8 Hz, 1H), 8.14 (d, *J* = 1.8 Hz, 1H), 8.09 (d, *J =* 8.6 Hz, 1H), 7.98 (ddd, *J* = 13.9, 8.7, 3.2 Hz, 2H), 7.55 (d, *J* = 8.1 Hz, 1H), 4.53 (s, 2H), 3.60 (dd, *J* = 26.0, 12.5 Hz, 8H), 2.69 (s, 3H). LCMS (ESI): calcd., 519.2; found, 519.4.

### Intermediate Example 65: preparation of 3-(imidazo[1,2-b]pyridazin-3-ylethynyl)-4-methyl-N-(4-(piperazin-1-yl)-3-(trifluoromethyl)phenyl)benzamide (GT-D-110)

3-(imidazo[1,2-b]pyridazin-3-ylethynyl)-4-methyl-N-(4-(piperazin-1-yl)-3-(trifluoromethyl)phenyl)benzamide (GT-D-110) was prepared by referring to Scheme 27 (off-white solid, 930 mg). ¹H NMR (400 MHz, MeOD) δ 9.10 - 9.00 (m, 1H), 8.55 (s, 1H), 8.47 (dd, J = 9.3, 1.3 Hz, 1H), 8.22 (d, J = 1.8 Hz, 1H), 8.15 (d, J = 2.4 Hz, 1H), 8.05 (dd, J = 8.7, 2.4 Hz, 1H), 7.97 (dd, J = 8.0, 1.9 Hz, 1H), 7.89 (dd, J = 9.3, 4.5 Hz, 1H), 7.65 (t, J = 6.5 Hz, 1H), 7.59 - 7.56 (m, 1H), 3.38 - 3.34 (m, 4H), 3.23 - 3.14 (m, 4H), 2.68 (s, 3H). LCMS (ESI): calcd., 505.2; found, 505.3.

### Intermediate Example 66: preparation of 3-(imidazo[1,2-b]pyridazin-3-ylethynyl)-4-methyl-N-(4-(4-(methylamino)piperidin-1-yl)-3-(trifluoromethyl)phenyl)benzamide (GT-D-111)

3-(imidazo[1,2-b]pyridazin-3-ylethynyl)-4-methyl-N-(4-(4-(methylamino)piperidin-1-yl)-3-(trifluoromethyl)phenyl)benzamide (GT-D-111) was prepared by referring to Scheme 27 (white solid,1.8 g). ¹H NMR (400 MHz, DMSO) δ 10.63 (s, 1H), 9.21 (s, 2H), 8.86 (dd, *J =* 4.4, 1.1 Hz, 1H), 8.43 (s, 1H), 8.21 (dd, *J* = 14.6, 1.9 Hz, 2H), 8.08 (s, 1H), 8.00 (dd, *J* = 8.0, 1.6 Hz, 1H), 7.59 - 7.53 (m, 3H), 3.00 (d, *J* = 11.7 Hz, 3H), 2.78 (s, 2H), 2.61 (s, 3H), 2.54 (t, *J* = 5.4 Hz, 3H), 2.11 (d, *J* = 10.0 Hz, 2H), 1.70 (dt, *J* = 11.3, 8.1 Hz, 2H). LCMS (ESI): calcd., 533.2; found, 533.2.

### Intermediate Example 67: preparation of 3-(imidazo[1,2-b]pyridazin-3-ylethynyl)-4-methyl-N-(4-(4-(piperidin-4-yl)piperazin-1-yl)-3-(trifluoromethyl)phenyl)benzamide (GT-D-112)

3-(imidazo[1,2-b]pyridazin-3-ylethynyl)-4-methyl-N-(4-(4-(piperidin-4-yl)piperazin-1-yl)-3-(trifluoromethyl)phenyl)benzamide (GT-D-112) was prepared by referring to Scheme 27 (brown solid, 2.1 g). ¹H NMR (400 MHz, DMSO) δ 10.60 (s, 1H), 8.74 (dd, *J* = 4.4, 1.5 Hz, 1H), 8.28 (dd, *J* = 9.2, 1.5 Hz, 1H), 8.25 (s, 1H), 8.22 (d, *J =* 2.1 Hz, 2H), 8.19 - 8.13 (m, 1H), 7.96 (dd, *J =* 8.0, 1.8 Hz, 1H), 7.58 (t, *J =* 9.0 Hz, 2H), 7.42 (dd, *J =* 9.2, 4.5 Hz, 1H), 3.49 (d, *J =* 13.0 Hz, 4H), 3.15 (s, 6H), 2.98 - 2.82 (m, 2H), 2.62 (s, 3H), 2.33 (d, *J* = 10.9 Hz, 2H), 1.80 (d, *J* = 9.7 Hz, 2H). LCMS (ESI): calcd., 588.3; found, 588.2.

### Intermediate Example 68: preparation of tert-butyl (4-(4-((3-(difluoromethyl)-1-(piperidin-4-yl)-1H-pyrazol-4-yl)carbamoyl)oxazol-2-yl)pyridin-2-yl)(2,2,2-trifluoroethyl)carbamate (GT-D-126)

tert-butyl (4-(4-((3-(difluoromethyl)-1-(piperidin-4-yl)-1H-pyrazol-4-yl)carbamoyl)oxazol-2-yl)pyridin-2-yl)(2,2,2-trifluoroethyl)carbamate (GT-D-126) was prepared by referring to Scheme 29 (white solid, 2.2 g). ¹H NMR (400 MHz, CDCl₃) δ 9.06 (s, 1H), 8.53 (d, *J =* 5.1 Hz, 1H), 8.39 (s, 1H), 8.34 (d, *J =* 5.3 Hz, 2H), 7.72 (dd, *J =* 5.1, 1.2 Hz, 1H), 6.84 (t, *J =* 54.7 Hz, 1H), 4.88 (q, *J =* 8.7 Hz, 2H), 4.21 (s, 1H), 3.26 (d, *J* = 12.6 Hz, 2H), 2.77 (td, *J* = 12.5, 2.1 Hz, 2H), 2.16 (d, *J* = 12.2 Hz, 2H), 1.92 (dd, *J* = 12.1, 3.8 Hz, 2H), 1.58 (s, 9H). LCMS (ESI): calcd., 586.2; found, 586.3.

### Intermediate Example 69: preparation of (1r,4r)-4-(6-(5-cyano-1H-pyrrolo[2,3-b]pyridin-1-yl)-4-(isopropylamino)nicotinamido)cyclohexane-1-carboxylic acid (GT-D-132)

(1r,4r)-4-(6-(5-cyano-1H-pyrrolo[2,3-b]pyridin-1-yl)-4-(isopropylamino)nicotinamido)cyclohexane-1-carboxylic acid (GT-D-132) was prepared by referring to Scheme 31 (white solid, 3.1 g). ¹H NMR (400 MHz, DMSO) δ 12.11 (s, 1H), 8.81 (d, *J =* 1.9 Hz, 1H), 8.67 (d, *J =* 2.0 Hz, 1H), 8.63 - 8.55 (m, 2H), 8.52 (d, *J =* 3.9 Hz, 1H), 8.37 (d, *J =* 7.6 Hz,1H), 8.07 (s, 1H), 6.89 (d, *J =* 3.9 Hz, 1H), 3.77 (dt, *J =* 19.1, 6.3 Hz, 2H), 2.17 (dd, *J =* 11.2, 7.9 Hz, 1H), 2.04 - 1.85 (m, 4H), 1.39 (dd, *J =* 16.9, 8.0 Hz, 4H), 1.30 (s, 3H), 1.29 (s, 3H). LCMS (ESI): calcd., 447.2; found, 447.2.

### Intermediate Example 70: preparation of 6-(5-cyano-1H-pyrrolo[2,3-b]pyridin-1-yl)-4-(isopropylamino)-N-(piperidin-4-yl)nicotinamide (GT-D-133)

6-(5-cyano-1H-pyrrolo[2,3-b]pyridin-1-yl)-4-(isopropylamino)-N-(piperidin-4-yl)nicotinamide (GT-D-133) was prepared by referring to Scheme 30 (white solid, 2.8 g). ¹H NMR (400 MHz, MeOD) δ 8.80 (d, *J* = 1.3 Hz, 1H), 8.74 (s, 1H), 8.62 (d, *J =* 1.5 Hz, 1H), 8.39 (d, *J* = 4.0 Hz, 1H), 7.44 (s, 1H), 7.09 (d, *J =* 4.0 Hz, 1H), 4.34 - 4.04 (m, 2H), 3.52 (d, *J =* 13.1 Hz, 2H), 3.17 (dd, *J* = 12.4, 10.3 Hz, 2H), 2.23 (d, *J =* 11.3 Hz, 2H), 1.97 (td, *J =* 14.5, 4.2 Hz, 2H), 1.39 (d, *J =* 6.4 Hz, 6H). LCMS (ESI): calcd., 404.2; found, 404.3.

### Intermediate Example 71: preparation of N-(4-(chlorodifluoromethoxy)phenyl)-6-(4-(piperazin-1-yl)piperidin-1-yl)-5-(1H-pyrazol-5-yl)nicotinamide (GT-D-179)

N-(4-(chlorodifluoromethoxy)phenyl)-6-(4-(piperazin-1-yl)piperidin-1-yl)-5-(1H-pyrazol-5-yl)nicotinamide (GT-D-179) was prepared by referring to Scheme 32 (brown solid, 1.03 g). ¹H NMR (400 MHz, MeOD) δ 8.79 (d, *J =* 2.3 Hz, 1H), 8.68 (d, *J =* 2.3 Hz, 1H), 8.00 (d, *J =* 2.3 Hz, 1H), 7.88 - 7.80 (m, 2H), 7.30 (d, *J =* 9.1 Hz, 2H), 6.88 (d, *J =* 2.2 Hz, 1H), 3.90 (dd, *J =* 11.4, 5.8 Hz, 3H), 3.72 (s, 8H), 3.24 (d, *J* = 12.2 Hz, 2H), 2.31 (d, *J* = 10.7 Hz, 2H), 2.09 (dd, *J* = 16.3, 7.7 Hz, 2H). LCMS (ESI): calcd., 532.2; found, 532.2.

### Intermediate Example 72: preparation of (R)-5-(3-(3-methyl-2-oxoimidazolidin-1-yl)piperidin-1-yl)-3-((4-(piperidin-4-yl)phenyl)amino)pyrazine-2-carboxamide (GT-D-197)

(R)-5-(3-(3-methyl-2-oxoimidazolidin-1-yl)piperidin-1-yl)-3-((4-(piperidin-4-yl)phenyl)amino)pyrazine-2-carboxamide (GT-D-197) was prepared by referring to Scheme 33 (yellow solid, 3.0 g). ¹H NMR (400 MHz, DMSO) δ 11.19 (s, 1H), 7.75 (s, 1H), 7.66 (d, *J =* 7.9 Hz, 1H), 7.48 (t, *J =* 10.8 Hz, 2H), 7.37 - 7.29 (m, 1H), 7.14 (d, *J =* 8.4 Hz, 2H), 4.32 (dd, *J =* 28.6, 12.2 Hz, 2H), 3.60 (d, *J =* 10.7 Hz, 1H), 3.26 (dd, *J* = 13.7, 7.6 Hz, 2H), 3.10 - 2.90 (m, 4H), 2.73 (d, *J* = 14.9 Hz, 3H), 2.63 - 2.54 (m, 2H), 1.76 (ddd, *J =* 33.3, 25.5, 11.2 Hz, 6H), 1.49 (ddd, *J =* 20.7, 19.1, 10.2 Hz, 4H). LCMS (ESI): calcd., 479.3; found, 479.5.

### Intermediate Example 73: preparation of 5-(piperidin-1-yl)-3-((4-(piperidin-4-yl)phenyl)amino)pyrazine-2-carboxamide (GT-D-198)

5-(piperidin-1-yl)-3-((4-(piperidin-4-yl)phenyl)amino)pyrazine-2-carboxamide (GT-D-198) was prepared by referring to Scheme 34 (yellow solid, 1.7 g). ¹H NMR (400 MHz, DMSO) δ 11.23 (s, 1H), 7.71 (s, 1H), 7.63 (s, 1H), 7.49 (d, *J =* 8.5 Hz, 2H), 7.28 (s, 1H), 7.14 (d, *J =* 8.5 Hz, 2H), 3.66 (d, *J =* 5.3 Hz, 4H), 3.15 (s, 2H), 3.03 - 2.93 (m, 2H), 2.54 (d, *J* = 14.1 Hz, 2H), 1.64 (d, *J* = 11.7 Hz, 4H), 1.57 (d, *J* = 3.8 Hz, 4H), 1.47 (td, *J* = 12.3, 3.6 Hz, 2H). LCMS (ESI): calcd., 381.2; found, 381.2.

### Intermediate Example 74: preparation of 4,9-dioxo-4,9-dihydronaphtho[2,3-b]furan-2-carboxylic acid (GT-D-200)

4,9-dioxo-4,9-dihydronaphtho[2,3-b]furan-2-carboxylic acid (GT-D-200) was prepared by referring to Scheme 35 (brown solid, 130 mg). ¹H NMR (400 MHz, DMSO) δ 8.14 (ddd, *J* = 7.1, 4.3, 3.3 Hz, 2H), 8.03 - 7.85 (m, 2H), 7.69 (s, 1H). LCMS (ESI): calcd., 243.0; found, 243.0.

### Intermediate Example 75: preparation of (6-((5-bromo-2-((2-methoxy-5-methyl-4-(piperazin-1-yl)phenyl)amino)pyrimidin-4-yl)amino)quinoxalin-5-yl)dimethylphosphine oxide (GT-S-50)

(6-((5-bromo-2-((2-methoxy-5-methyl-4-(piperazin-1-yl)phenyl)amino)pyrimidin-4-yl)amino)quinoxalin-5-yl)dimethylphosphine oxide (GT-S-50) was prepared by referring to Scheme 9 (yellow solid, 950 mg). ¹H NMR (400 MHz, DMSO-d6): δ ppm 12.76 (s, 1H), 9.23 (br, 2H), 8.91(d, 3H), 8.38 - 8.32 (m, 2H), 8.00 (d, 1H), 7.49(s, 1H), 6.77 (s, 1H), 3.83 (s, 3H), 3.32 - 3.24 (m, 4H), 3.12 - 2.97 (m, 4H), 2.13 (s, 3H), 2.10 (s, 3H), 2.03 (s, 3H). LCMS (ESI): calcd., 597.2; found, 597.1.

### Intermediate Example 76: preparation of (6-((5-bromo-2-((2-methoxy-5-methyl-4-(4-(methylamino)piperidin-1-yl)phenyl)amino)pyrimidin-4-yl)amino)quinoxalin-5-yl)dimethylphosphine oxide (GT-S-51)

(6-((5-bromo-2-((2-methoxy-5-methyl-4-(4-(methylamino)piperidin-1-yl)phenyl)amino)pyrimidin-4-yl)amino)quinoxalin-5-yl)dimethylphosphine oxide (GT-S-51) was prepared by referring to Scheme 9 (yellow solid, 1.1 g). ¹H NMR (400 MHz, CD₃OD): δ ppm 8.94 - 8.90 (br, 3H), 8.30 (s, 1H), 8.10 - 8.07 (br, 1H) , 7.35 (s, 1H), 6.89 (s, 1H), 3.91 (s, 3H), 3.39 - 3.24 (m, 3H), 2.91 (t, *J =* 15.6 Hz, 2H), 2.82 (s, 3H), 2.30 - 2.19 (m, 11H), 1.98 - 1.84 (m, 11H), 1.98 - 1.84 (m, 2H). LCMS (ESI): calcd., 625.2; found, 625.1.

### Intermediate Example 77: preparation of (6-((5-bromo-2-((2-methoxy-5-methyl-4-(4-(piperazin-1-yl)piperidin-1-yl)phenyl)amino)pyrimidin-4-yl)amino)quinoxalin-5-yl)dimethylphosphine oxide (GT-S-52)

(6-((5-bromo-2-((2-methoxy-5-methyl-4-(4-(piperazin-1-yl)piperidin-1-yl)phenyl)amino)pyrimidin-4-yl)amino)quinoxalin-5-yl)dimethylphosphine oxide (GT-S-52) was prepared by referring to Scheme 9 (yellow solid, 150 mg, yield 108.88%). ¹H NMR (400 MHz, DMSO-d₆) δ ppm 9.10 - 8.80 (m, 2H), 8.41 (s, 1H), 8.05 - 7.95 (m, 1H), 7.60 - 7.50 (m, 2H), 7.48 - 7.36 (m, 3H), 7.20 - 7.10 (m, 2H), 6.85 - 6.80 (m, 1H), 3.80 - 3.62 (m, 6H), 3.60 - 3.40 (m, 6H), 3.35 - 3.30 (m, 2H), 2.85 - 2.60 (m, 2H), 2.38 - 2.25 (m, 2H), 2.20 - 1.90 (m, 11H). LCMS (ESI): calcd., 680.2; found, 680.1.

### Intermediate Example 78: preparation of 1-isopropyl-N-((6-methyl-2-oxo-4-propyl-1,2-dihydropyridin-3-yl)methyl)-6-(6-(piperazin-1-yl)pyridin-3-yl)-1H-indazole-4-carboxamide (GT-S-7)

1-isopropyl-N-((6-methyl-2-oxo-4-propyl-1,2-dihydropyridin-3-yl)methyl)-6-(6-(piperazin-1-yl)pyridin-3-yl)-1H-indazole-4-carboxamide (GT-S-7) was prepared by referring to Scheme 38 (yellow solid, 300 mg, yield 21%). ¹H NMR (400 MHz, DMSO-d₆) δ ppm 8.67 - 8.61 (m, 2H), 8.38 (s, 1H), 8.08 - 8.05 (br, 2H), 7.85 (s, 1H), 6.96 (d, *J =* 11.6 Hz, 1H), 5.94 (s, 1H), 5.20 - 5.11 (m, 1H), 4.44 (d, *J =* 6.4 Hz, 2H), 3.52 - 3.49 (m, 4H), 2.86 - 2.78 (m, 4H), 2.57 - 2.53 (m, 2H), 2.11 (s, 3H), 1.58 - 1.51 (m, 8H), 0.90 (t, *J* = 9.6 Hz, 3 H). LCMS (ESI): calcd., 528.3; found, 528.5.

### Intermediate Example 79: preparation of 1-isopropyl-N-((6-methyl-2-oxo-4-propyl-1,2-dihydropyridin-3-yl)methyl)-6-(6-(4-(methylamino)piperidin-1-yl)pyridin-3-yl)-1H-indazole-4-carboxamide (GT-S-8)

1-isopropyl-N-((6-methyl-2-oxo-4-propyl-1,2-dihydropyridin-3-yl)methyl)-6-(6-(4-(methylamino)piperidin-1-yl)pyridin-3-yl)-1H-indazole-4-carboxamide (GT-S-8) was prepared by referring to Scheme 36 (light yellow solid, 1.8 g). ¹H NMR (400 MHz, DMSO-d₆): δ ppm 8.69 - 8.57 (m, 4H), 8.63 (s, 1H), 8.15 - 8.08 (m, 4H), 7.82 (br, 1H), 7.11 (d, *J =* 8.8 Hz, 1H), 5.91 (s, 1H), 5.17 - 5.10 (m, 1H), 4.48 - 4.41 (m, 4H), 3.30 - 3.28 (br, 1H), 2.96 (t, *J* = 12.6 Hz, 2H), 2.59 (t, *J* = 5.2 Hz, 3H), 2.54 - 2.49 (m, 4H), 2.14 (s, 3H), 2.09 - 2.07 (m, 2H), 1.57 - 1.49 (m, 8H), 0.88 (t, *J* = 7.4 Hz, 3H). LCMS (ESI): calcd., 556.3; found, 556.5.

### Intermediate Example 80: preparation of 1-isopropyl-N-((6-methyl-2-oxo-4-propyl-1,2-dihydropyridin-3-yl)methyl)-6-(6-(4-(piperidin-4-yl)piperazin-1-yl)pyridin-3-yl)-1H-indazole-4-carboxamide (GT-S-9)

1-isopropyl-N-((6-methyl-2-oxo-4-propyl-1,2-dihydropyridin-3-yl)methyl)-6-(6-(4-(piperidin-4-yl)piperazin-1-yl)pyridin-3-yl)-1H-indazole-4-carboxamide (GT-S-9) was prepared by referring to Scheme 37 (light yellow solid, 2.0 g). ¹H NMR (400 MHz, DMSO-d₆): δ ppm 11.99 (br, 1H), 9.40 - 9.37 (br, 1H), 8.78 (br, 1H), 8.67 (s, 1H), 8.45 - 8.40 (m, 2H), 8.24 (s, 1H), 7.88 (s, 1H), 7.40 (d, *J* = 12 Hz, 1H), 5.99 (s, 1H), 5.24 - 5.15 (m, 1H), 4.66 - 4.62 (m, 2H), 4.45 - 4.44 (br, 2H), 3.77 - 3.18 (m, 7H), 3.25 - 3.18 (m, 2H), 2.97 - 2.90 (m, 2H), 2.58 - 2.52 (m, 4H), 2.37 - 2.33 (br, 2H), 2.17 (s, 3H), 2.10 - 2.02 (m, 2H), 1.57 - 1.51 (m, 8H), 0.89 (t, *J* = 9.6 Hz, 3H). LCMS (ESI): calcd., 611.4; found, 611.7.

### Intermediate Example 81: preparation of N-((4,6-dimethyl-2-oxo-1,2-dihydropyridin-3-yl)methyl)-5-(ethyl(tetrahydro-2H-pyran-4-yl)amino)-4-methyl-4'-(piperazin-1-ylmethyl)-[1,1'-biphenyl]-3-carboxamide (GT-S-10)

N-((4,6-dimethyl-2-oxo-1,2-dihydropyridin-3-yl)methyl)-5-(ethyl(tetrahydro-2H-pyran-4-yl)amino)-4-methyl-4'-(piperazin-1-ylmethyl)-[1,1'-biphenyl]-3-carboxamide (GT-S-10) was prepared by referring to Scheme 40 (yellow solid, 1.4 g). ¹H NMR (400 MHz, DMSO-d₆): δ ppm 11.99 (br, 1H), 9.40 - 9.37 (br, 1H), 8.78 (br, 1H), 8.67 (s, 1H), 8.45 - 8.40 (m, 2H), 8.24 (s, 1H), 7.88 (s, 1H), 7.40 (d, *J =* 12 Hz, 1H), 5.99 (s, 1H), 5.24 - 5.15 (m, 1H), 4.66 - 4.62 (m, 2H), 4.45 - 4.44 (br, 2H), 3.77 - 3.18 (m, 7H), 3.25 - 3.18 (m, 2H), 2.97 - 2.90 (m, 2H), 2.58 - 2.52 (m, 4H), 2.37 - 2.33 (br, 2H), 2.17 (s, 3H), 2.10 - 2.02 (m, 2H), 1.57 - 1.51 (m, 8H), 0.89 (t, *J* = 9.6 Hz, 3H). LCMS (ESI): calcd., 611.4; found, 611.7.

### Intermediate Example 82: preparation of N-((4,6-dimethyl-2-oxo-1,2-dihydropyridin-3-yl)methyl)-5-(ethyl(tetrahydro-2H-pyran-4-yl)amino)-4-methyl-4'-(piperazin-1-yl)-[1,1'-biphenyl]-3-carboxamide (GT-S-11)

N-((4,6-dimethyl-2-oxo-1,2-dihydropyridin-3-yl)methyl)-5-(ethyl(tetrahydro-2H-pyran-4-yl)amino)-4-methyl-4'-(piperazin-1-yl)-[1,1'-biphenyl]-3-carboxamide (GT-S-11) was prepared by referring to Scheme 41 (white solid, 2.6 g). ¹H NMR (400 MHz, DMSO-d₆) δ 8.21 (t, J = 6.0 Hz, 1H), 7.54 (d, J = 11.6 Hz, 2H), 7.3 (s, 1H), 7.20 (s, 1H), 7.07 (d, J = 11.2 Hz, 2H), 4.31 (d, J =6 Hz, 2H), 3.86 (d, J = 13.6 Hz, 2H), 3.35 - 3.12 (m, 15 H), 2.24 (d, J = 6.4 Hz, 6H), 2.14 (s, 2H), 1.71 - 1.48 (m, 4H), 0.86 (t, J = 9.2 Hz, 3H). LCMS (ESI): calcd., 558.3; found, 558.5.

### Intermediate Example 83: preparation of N-((4,6-dimethyl-2-oxo-1,2-dihydropyridin-3-yl)methyl)-5-(ethyl(tetrahydro-2H-pyran-4-yl)amino)-4-methyl-4'-(4-(methylamino)piperidin-1-yl)-[1,1'-biphenyl]-3-carboxamide (GT-S-12)

N-((4,6-dimethyl-2-oxo-1,2-dihydropyridin-3-yl)methyl)-5-(ethyl(tetrahydro-2H-pyran-4-yl)amino)-4-methyl-4'-(4-(methylamino)piperidin-1-yl)-[1,1'-biphenyl]-3-carboxamide (GT-S-12) was prepared by referring to Scheme 42 (white solid, 1.8 g). ¹H NMR (400 MHz, DMSO-d₆) δ 8.16 (t, J = 4.8 Hz, 1H), 7.45 (d, J = 8.8 Hz, 2H), 7.33 (s, 1H), 7.16 (s, 1H), 6.98 (d, J = 8.8 Hz, 2H), 5.86 (s, 1H), 4.29 (d, J = 4.8 Hz, 2H), 3.82 (d, J = 10.4 Hz, 2H), 3.65 (d, J = 12.4 Hz, 2H), 3.25 (t, J = 11.2 Hz, 3H),3.10-3.01 (m, 4H), 2.76 (t, J = 11.6 Hz, 2H), 2.45-2.38 (m, 1H), 2.29 (s, 3H),2.21( d, J = 6.8 Hz, 6H), 2.11 (s, 3H), 1.89 - 1.85 (m, 2H), 1.66 - 1.64 (m, 2H), 1.55 - 1.49 (m, 2H), 1.32 - 1.28 (m, 2H), 0.83 (t, J = 6.8 Hz, 3H). LCMS (ESI): calcd., 586.4; found, 586.2.

### Intermediate Example 84: preparation of N-((4,6-dimethyl-2-oxo-1,2-dihydropyridin-3-yl)methyl)-5-(ethyl(tetrahydro-2H-pyran-4-yl)amino)-4-methyl-4'-(4-(piperidin-4-yl)piperazin-1-yl)-[1,1'-biphenyl]-3-carboxamide (GT-S-13)

N-((4,6-dimethyl-2-oxo-1,2-dihydropyridin-3-yl)methyl)-5-(ethyl(tetrahydro-2H-pyran-4-yl)amino)-4-methyl-4'-(4-(piperidin-4-yl)piperazin-1-yl)-[1,1'-biphenyl]-3-carboxamide (GT-S-13) was prepared by referring to Scheme 43 (white solid, 1.6 g). ¹H NMR (400 MHz, DMSO-d₆) δ 8.18 (t, J = 2.0 Hz, 1H), 7.47 (d, J = 8.4 Hz, 2H), 7.33 (s, 1H), 7.16 (s, 1H), 6.98 (d, J = 8.0 Hz, 2H), 5.86 (s, 1H), 4.29 (d, J = 4.0 Hz, 2H), 4.14 - 4.06 (m, 1H), 3.82 (d, J = 9.2 Hz, 2H), 3.41 - 3.36 (m, 2H), 3.29 (d, J = 12.0 Hz, 3H), 3.14 - 2.98 (m, 9H), 3.62 (s, 4H), 2.28 - 2.21 (m, 7H), 2.11 ( s, 3H), 1.68 - 1.64 (m, 4H), 1.53 - 1.50 (m, 2H), 1.26 - 1.24 (m, 2H), 0.83 (t, J = 6.8 Hz, 3H). LCMS (ESI): calcd., 641.4; found, 642.0.

### Intermediate Example 85: preparation of N-(5-(3,5-difluorobenzyl)-1H-indazol-3-yl)-4-(piperazin-1-yl)-2-((tetrahydro-2H-pyran-4-yl)amino)benzamide (GT-S-14)

N-(5-(3,5-difluorobenzyl)-1H-indazol-3-yl)-4-(piperazin-1-yl)-2-((tetrahydro-2H-pyran-4-yl)amino)benzamide (GT-S-14) was prepared by referring to Scheme 44 (yellow solid, 341 mg). ¹H NMR (400 MHz, DMSO-d₆) δ 10.22 (s, 1H), 9.13 (s, 2H), 7.88 (d, J = 12 Hz, 1H),7.48 (d, J = 19.2 Hz,, 1H), 7.43 (s, 1H), 7.28 (d, J = 11.6 Hz, 1H), 7.03 (t, J = 16 Hz, 3H), 6.30 (t, J = 11.6 Hz, 2H), 4.32 - 4.07 (m, 2H), 3.86 (t, J = 5.2 Hz, 2H), 3.73 (s, 1H), 3.50 (d, J = 14.4 Hz, 6H), 3.26 (s, 4H), 1.97 (d, J =15.6 Hz, 2H), 1.42 - 1.36 (m, 2H). LCMS (ESI): calcd., 547.3; found, 547.0.

### Intermediate Example 86: preparation of N-(5-(3,5-difluorobenzyl)-1H-indazol-3-yl)-4-(4-(methylamino)piperidin-1-yl)-2-((tetrahydro-2H-pyran-4-yl)amino)benzamide (GT-S-15)

N-(5-(3,5-difluorobenzyl)-1H-indazol-3-yl)-4-(4-(methylamino)piperidin-1-yl)-2-((tetrahydro-2H-pyran-4-yl)amino)benzamide (GT-S-15) was prepared by referring to Scheme 45 (yellow solid, 601 mg).¹H NMR (400 MHz, DMSO-d₆) δ 9.40 (s, 3H), 7.49 (t, J = 11.6 Hz, 2H), 7.11 (d, J = 10.4 Hz, 1H), 6.81 (d, J = 8.4 Hz,, 1H), 6.45 (d, J = 9.2 Hz, 2H), 6.34 (t, J = 11.6 Hz, 1H), 5.94 (d, J = 11.6 Hz, 1H), 5.83 (s, 1H), 3.75 (s, 2H), 3.60 (d, J = 18.4 Hz, 4H), 3.26 (d, J =13.2 Hz, 3H), 2.86 (s, 1H), 2.57 (t, J = 16.4 Hz, 2H), 2.38 (s, 3H), 1.91 (d, J = 13.6 Hz, 1H), 1.67 (t, J = 12.4 Hz, 2H), 1.56 (t, J = 13.6 Hz, 2H), 1.27 (s, 2H). LCMS (ESI): calcd., 575.3; found, 575.0.

### Intermediate Example 87: preparation of (R)-6-(2-(3-fluorophenyl)pyrrolidin-1-yl)-3-(6-(piperazin-1-yl)pyridin-2-yl)imidazo[1,2-b]pyridazine (GT-S-17)

(R)-6-(2-(3-fluorophenyl)pyrrolidin-1-yl)-3-(6-(piperazin-1-yl)pyridin-2-yl)imidazo[1,2-b]pyridazine (GT-S-17) was prepared by referring to Scheme 46 (yellow solid, 800 mg). ¹H NMR (400 MHz, DMSO-d6) δ 8.17 (s, 1H), 7.89 (d, *J* = 6.4 Hz, 1H), 5.57 (s, 2H), 7.54 (d, J = 10.2 Hz, 1H), 7.42 - 7.36 (m, 2H), 7.05 (t, *J* = 8.0 Hz, 1H), 6.82 - 6.74 (m, 2H), 5.15(d, J = 4.0 Hz, 1H), 3.98, 3.95 (dd, J = 6.4, 6.0 Hz, 1H), 3.71 - 3.60 (m, 6H), 2.99 (s, 4H), 2.53 - 2.44 (m, 1H), 2.03 (d, J = 6.8 Hz, 2H), 1.89 - 1.85 (m, 1H), 1.23 (s, 1H). LCMS (ESI): calcd., 444.2; found, 444.3.

### Intermediate Example 88: preparation of (R)-1-(6-(6-(2-(3-fluorophenyl)pyrrolidin-1-yl)imidazo[1,2-b]pyridazin-3-yl)pyridin-2-yl)-N-methylpiperidin-4-amine (GT-S-18)

(R)-1-(6-(6-(2-(3-fluorophenyl)pyrrolidin-1-yl)imidazo[1,2-b]pyridazin-3-yl)pyridin-2-yl)-N-methylpiperidin-4-amine (GT-S-18) was prepared by referring to Scheme 47 (yellow solid, 790 mg). ¹H NMR (400 MHz, CD₃OD) δ 8.61 (s, 1H), 8.20 (s, 1H), 7.40 (t, J = 7.6 Hz, 2H), 7.18 - 7.02 (m, 6H), 5.25 (s, 1H), 4.58 (s, 2H), 4.15 (t, J = 9.2 Hz, 1H), 3.68(s, 1H), 3.39 (s, 1H), 3.35 (s, 1H), 2.86 (s, 3H), 2.36 (s, 1H), 2.33 (s, 2H), 2.21 (s, 3H), 2.06 (d, J = 3.6 Hz, 3H), 1.85 - 1.82 (m, 2H). LCMS (ESI): calcd., 472.3; found, 472.3.

### Intermediate Example 89: preparation of the following intermediate compounds

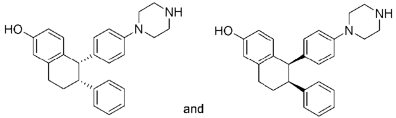

Following the method of Scheme 49, the enantiomeric mixture product obtained according to Step 8 of Scheme 49 was separated by supercritical fluid chromatography (SFC) to obtain the two intermediate compounds as described above (their retention times were 8.232 min and 9.400 min, respectively, in terms of analytical methods). The analytical method, preparation method, and conditions for SFC were shown below.

The analysis method and conditions for SFC are as follows:

| | |
|---|---|
| System: | Shimadzu LC-20AP |
| Column name: | Daicel CHIRALPAK^{®}IE |
| Column size: | 250*4.6mm 5mm |
| Mobile Phase A: | n-Hexane |
| Mobile Phase B: | EtOH(0.2%DEA) |
| Mobile Phase A: Mobile Phase B | 70: 30 |
| Wavelength: | 254 nm |
| Flow | 1mL/min |
| Column temp: | 25°C |
| Injection: | 5mL |
| Solvent: | EtOH: HPLC grade |
| | n-Hexane: HPLC grade |

Preparation method for SFC is as follows:

| | |
|---|---|
| System: | Shimadzu |
| Column name: | DAICELCHIRALPAK^{®}IE |
| Column size: | 250*25 mm 10 mm |
| Mobile Phase A: | n-Hexane |
| Mobile Phase B: | ETOH (+0.1% 7.0mol/l Ammonia in MeOH) |
| A:B: | 70: 30 |
| Wavelength: | 214 nm |
| Flow: | 30ml/min |
| Column temp: | rt |
| Injection: | 1mL |
| Cycle time: | 12min |
| Solvent: | n-Hexane: redistilled grade |
| | ETOH: redistilled grade |
| Preparation of sample solution: | The sample was dissolved in approximately 30 mL of ethanol. |

One of the obtained intermediates (GT-M-160_P1) (white solid, 565 mg): ¹H NMR (400 MHz, DMSO) δ 9.11 (s, 1H), 7.23 - 7.06 (m, 3H), 6.84 (d, *J =* 6.7 Hz, 2H), 6.62 (dd, *J =* 15.5, 5.3 Hz, 2H), 6.55 - 6.43 (m, 3H), 6.21 (d, *J =* 8.6 Hz, 2H), 4.13 (d, *J* = 4.9 Hz, 1H), 3.28 (s, 2H), 3.04 - 2.91 (m, 2H), 2.91 - 2.83 (m, 4H), 2.80 - 2.70 (m, 4H), 2.11 (dd, *J =* 12.3, 6.4 Hz, 1H), 1.71 (d, *J =* 7.5 Hz, 1H). LCMS (ESI): calcd., 385.23; found, 385.30. (retention time was 8.232 min in terms of analytical method).

Another intermediate (GT-M-160_P2) (white solid, 595 mg): ¹H NMR (400 MHz, DMSO) δ 9.10 (s, 1H), 7.14 (dd, *J =* 15.2, 7.8 Hz, 3H), 6.84 (d, *J =* 6.9 Hz, 2H), 6.66 - 6.59 (m, 2H), 6.50 (dd, *J =* 14.1, 8.3 Hz, 3H), 6.22 (d, *J =* 8.4 Hz, 2H), 4.14 (d, *J =* 4.6 Hz, 1H), 3.30 - 3.23 (m, 2H), 2.97 (dd, *J =* 15.6, 9.6 Hz, 2H), 2.90 (d, *J =* 5.0 Hz, 4H), 2.80 (d, *J =* 4.5 Hz, 4H), 2.11 (dd, *J =* 12.2, 6.8 Hz, 1H), 1.72 (s, 1H). LCMS (ESI): calcd., 385.23; found, 385.30. (retention time was 9.400 min in terms of analytical method).

### Intermediate Example 90: preparation of the following intermediate compounds

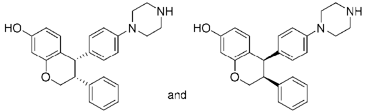

Following the method of Scheme 50, the enantiomeric mixture product obtained according to Step 6 of Scheme 50 was separated by supercritical fluid chromatography (SFC) to obtain the two intermediate compounds as described above (their retention times were 1.763 min and 3.506 min, respectively, in terms of analytical methods). The analytical method, preparation method, and conditions for SFC were shown below.

The analysis method and conditions for SFC are as follows:

| | | | |
|---|---|---|---|
| System: | Waters Ultra Performance Convergence Chromatography (UPCC) (CA-185) | | |
| Column name: | DAICELCHIRALPAK^{®}OD | | |
| Column size: | 100*3.0 mm *3.0 mm | | |
| Mobile Phase A: | Supercritical CO₂ | | |
| Mobile Phase B: | IPA (0.1%DEA) | | |
| Wavelength: | 214nm | | |
| Flow | 1.5 mL/min | | |
| Column temp: | 35°C | | |
| Back Pressure(psi): | 1800 psi | | |
| Injection: | 0.3 mL | | |
| Run time: | 8 min | | |
| Gradient | Time (min) | A(%V/V) | B(%V/V) |
| | 0.00 | 60 | 40 |
| | 8.00 | 60 | 40 |
| Solvent: | IPA: HPLC grade | | |
| | Supercritical CO₂: Food grade | | |
| Preparation of mobile phases: | Mobile Phase B: Add 1 mL DEA in 1000 mL IPA, then ultrasonic degassing for 15min. | | |

Preparation method for SFC is as follows:

| | |
|---|---|
| System: | Waters SFC 150 |
| Column name: | DAICELCHIRALCEL^{®}OD |
| Column size: | 250*25 mm 10 mm |
| Mobile Phase A: | Supercritical CO₂, |
| Mobile Phase B: | IPA (+0.1% 7.0mol/l Ammonia in MEOH) |
| A:B: | 45: 55 |
| Wavelength: | 214 nm |
| Flow: | 80ml/min |
| Column temp: | RT |
| Back Pressure: | 100 bar |
| Injection: | 1.5mL |
| Cycle time: | 6min |
| Solvent: | IPA: redistilled grade |
| | Supercritical CO₂: Food grade |

One of the obtained intermediates (GT-M-173_P1) (white solid, 555 mg): ¹H NMR (400 MHz, DMSO) δ 9.33 (s, 1H), 7.22 (dd, *J* = 20.1, 17.2 Hz, 3H), 6.88 (d, *J* = 45.0 Hz, 2H), 6.71 (d, *J* = 8.3 Hz, 1H), 6.64 (d, *J* = 8.6 Hz, 2H), 6.44 (d, *J* = 8.5 Hz, 2H), 6.39 - 6.22 (m, 2H), 4.39 (t, *J* = 11.1 Hz, 1H), 4.30 - 4.06 (m, 2H), 3.55 (dt, *J* = 39.1, 19.2 Hz, 1H), 2.95 (d, *J* = 4.8 Hz, 4H), 2.83 (d, *J* = 4.5 Hz, 4H). LCMS (ESI): calcd., 387.21; found, 387.40. (retention time was 1.763 min in terms of analytical method).

Another intermediate (GT-M-173_P1) (white solid, 495 mg): ¹H NMR (400 MHz, DMSO) δ 9.33 (s, 1H), 7.22 (dd, *J* = 20.1, 17.2 Hz, 3H), 6.88 (d, *J* = 45.0 Hz, 2H), 6.71 (d, *J* = 8.3 Hz, 1H), 6.64 (d, *J* = 8.6 Hz, 2H), 6.44 (d, *J* = 8.5 Hz, 2H), 6.39 - 6.22 (m, 2H), 4.39 (t, *J* = 11.1 Hz, 1H), 4.30 - 4.06 (m, 2H), 3.55 (dt, *J* = 39.1, 19.2 Hz, 1H), 2.95 (d, *J* = 4.8 Hz, 4H), 2.83 (d, *J* = 4.5 Hz, 4H). LCMS (ESI): calcd., 387.21 found, 387.40. (retention time was 3.506 min in terms of analytical method).

### Intermediate Example 91: preparation of N-(3-(difluoromethyl)-1-(1-methylpiperidin-4-yl)-1H-pyrazol-4-yl)-5-morpholinopyrazolo[1,5-a]pyrimidine-3-carboxamide (GT-04349)

The target compound (GT-04349) was prepared by referring to Scheme 48 (white solid, 15 mg, yield 67.08%). ¹H NMR (400 MHz, MeOD) δ 8.45 (d, *J* = 8.0 Hz, 1H), 8.33 (s, 1H), 8.22 (s, 1H), 6.97 - 6.59 (m, 2H), 4.50 (s, 2H), 3.69 (dd, *J =* 35.3, 32.6 Hz, 10H), 3.16 (s, 1H), 2.85 (s, 3H), 2.29 (s, 4H). LCMS (ESI) calcd for C₂₁H₂₇F₂N₈O₂⁺ [M+H]⁺: 461.22, found, 461.2.

### Intermediate Example 92: preparation of tert-butyl (1R,5S)-3-(7-(8-ethynyl-7-fluoro-3-(methoxymethoxy)naphthalen-1-yl)-8-fluoro-2-(piperidin-4-ylmethoxy)pyrido[4,3-d]pyrimidin-4-yl)-3,8-diazabicyclo[3.2.1]octane-8-carboxylate (GT-D-204)

The target compound (GT-D-204) was prepared by referring to Scheme 51 (yellow solid, 0.92 g, yield 86.79%). ¹H NMR (400 MHz, CDCl₃) δ 8.99 (s, 1H), 7.84 (dd, J = 9.2, 5.7 Hz, 1H), 7.55 (d, J = 2.5 Hz, 1H), 7.39 (d, J = 2.4 Hz, 1H), 7.29 (dd, J = 14.0, 5.2 Hz, 1H), 5.32 (q, J = 6.9 Hz, 2H), 4.55 (dd, J = 29.6, 12.4 Hz, 2H), 4.42 (s, 2H), 4.33 (d, J = 6.2 Hz, 2H), 3.70 (s, 2H), 3.53 (s, 3H), 3.22 (d, J = 12.2 Hz, 2H), 2.80 (s, 1H), 2.71 (t, J = 11.2 Hz, 2H), 2.04 - 1.91 (m, 9H), 1.53 (s, 9H). LCMS (ESI): calcd., 701.33, found, 701.4.

### Intermediate Example 93: preparation of (R)-5-(2-(2,5-difluorophenyl)pyrrolidin-1-yl)-3-(6-(piperazin-1-yl)pyridin-2-yl)pyrazolo[1,5-a]pyrimidine (GT-D-191)

The target compound GT-D-191 was prepared by referring to Scheme 52 (red solid, 262 mg, yield 81.75%). ¹H NMR (400 MHz, DMSO) δ 9.48 (s, 2H), 8.66 (d, J = 8.0 Hz, 1H), 8.49 (s, 1H), 7.79 - 6.24 (m, 7H), 5.44 (d, J = 4.0 Hz, 1H), 4.20 - 3.95 (m, 2H), 3.84-3.77 (m, 5H), 3.20 (s, 4H), 2.10 (s, 2H), 1.93 (d, J = 8.0 Hz, 1H). LCMS (ESI): calcd., 462.22, found, 462.2.

### Intermediate Example 94: preparation of 2-(6,7-dihydro-5H-pyrrolo[1,2-c]imidazol-1-yl)-2-(4-fluoro-1-oxo-6-(4-(4-(piperazin-1-yl)piperidin-1-yl)phenyl)isoindolin-2-yl)-N-(thiazol-2-yl)acetamide (GT-D-219)

The target compound GT-D-219 was prepared by referring to Scheme 53 (yellow solid, 1.1 g, yield 113.53%). ¹H NMR (400 MHz, MeOD) δ 8.92 (s, 1H), 8.03 - 7.82 (m, 5H), 7.77 (dd, J = 10.0, 1.2 Hz, 1H), 7.58 (d, J = 4.0 Hz, 1H), 7.34 (d, J = 4.0 Hz, 1H), 4.95 (d, J = 17.2 Hz, 2H), 4.65 (d, J = 17.2 Hz, 1H), 4.44 - 4.29 (m, 2H), 4.00 (d, J = 12.0 Hz, 3H), 3.92 - 3.65 (m, 10H), 3.10 - 2.97 (m, 1H), 2.94 - 2.82 (m, 1H), 2.78 - 2.48 (m, 6H). LCMS (ESI): calcd., 641.28, found, 641.4.

### Intermediate Example 95: preparation of 2-(6,7-dihydro-5H-pyrrolo[1,2-c]imidazol-1-yl)-2-(4-fluoro-1-oxo-6-(4-(4-(piperidin-4-yl)piperazin-1-yl)phenyl)isoindolin-2-yl)-N-(thiazol-2-yl)acetamide (GT-D-217)

The target compound GT-D-217 was prepared by referring to Scheme 54 (yellow solid, 800 mg, yield 99.45%). ¹H NMR (400 MHz, MeOD) δ 8.90 (s, 1H), 7.88 (s, 1H), 7.66 (d, J = 8.6 Hz, 3H), 7.57 - 7.51 (m, 1H), 7.32 - 7.26 (m, 1H), 7.18-6.89 (m, 3H), 4.91 (d, J = 16.0 Hz, 1H), 4.82-4.75 (m, 1H), 4.58 (d, J = 17.0 Hz, 1H), 4.38-4.33 (m, 2H), 4.01 (s, 2H), 3.78 (s, 2H), 3.64 (d, J = 12.0 Hz, 2H), 3.38 (s, 2H), 3.26 (s, 2H), 3.18-3.12 (m, 2H), 3.07-2.99 (m, 1H), 2.91 - 2.84 (m, 1H), 2.76 - 2.63 (m, 2H), 2.52 (d, J = 12.0 Hz, 2H), 2.18 - 2.05 (m, 2H). LCMS (ESI): calcd., 641.28, found, 641.4.

### Intermediate Example 96: preparation of 2-(6-(4-((1S,4S)-2,5-diazabicyclo[2.2.1]heptan-2-yl)phenyl)-4-fluoro-1-oxoisoindolin-2-yl)-2-(6,7-dihydro-5H-pyrrolo[1,2-c]imidazol-1-yl)-N-(thiazol-2-yl)acetamide (GT-D-222)

Following steps 2-5 in Scheme 53, the starting material (CAS NO.: 2407965-04-0) and the compound (CAS NO.: 942189-80-2) were used to prepare the target compound GT-D-222 (off-white solid, 0.56 g, yield 109.7%). ¹H NMR (400 MHz, MeOD) δ 8.90 (s, 1H), 7.85 (d,J = 1.2 Hz, 1H), 7.69 - 7.59 (m, 3H), 7.54 (d, J =4.0 Hz, 1H), 7.29 (d, J = 4.0 Hz, 1H), 6.81 (d,J = 8.0 Hz, 2H), 4.91 (d, J = 10.0 Hz, 1H), 4.87 - 4.81 (m,1H), 4.74 (s, 1H), 4.62 - 4.50 (m, 2H), 4.35 (dd, J = 12.0, 7.2 Hz, 2H), 3.79 (dd, J = 10.0, 2.4 Hz, 1H),3.40 (s, 2H), 3.06-2.81 (m, 2H), 2.77 - 2.61 (m, 2H), 2.33 (d, J = 10.0 Hz, 1H), 2.09 (d,J = 12.0 Hz, 1H), 2.00 (d, J = 8.8 Hz, 1H), 1.60 (s, 1H). LCMS (ESI): calcd., 570.21, found, 570.2.

### Intermediate Example 97: preparation of 2-(6,7-dihydro-5H-pyrrolo[1,2-c]imidazol-1-yl)-2-(4-fluoro-6-(4-(4-(methylamino)piperidin-1-yl)phenyl)-1-oxoisoindolin-2-yl)-N-(thiazol-2-yl)acetamide (GT-D-221)

Following steps 2-5 in Scheme 53, the starting material (CAS NO.: 2407965-04-0) and the compound (CAS NO.: 2235416-80-3) were used to prepare the target compound GT-D-221 (yellow solid, 0.72 g, yield 105.4%). ¹H NMR (400 MHz, MeOD) δ 8.91 (s, 1H), 7.96 (d, *J =* 1.2 Hz, 1H), 7.92 (d, *J* = 3.2 Hz, 3H), 7.77 (dd, *J =* 10.0, 1.2 Hz, 1H), 7.55 (d, *J =* 3.6 Hz, 1H), 7.30 (d, *J =* 3.6 Hz, 1H), 6.51 (s, 1H), 4.96 (d, *J =* 17.2 Hz, 1H), 4.88 (s, 1H), 4.83 (s, 1H), 4.63 (d, *J =* 17.2 Hz, 1H), 4.34 (dt, *J =* 12.0, 7.2 Hz, 2H), 3.93 (d, *J =* 12.0 Hz, 2H), 3.85 (t, *J =* 11.2 Hz, 2H), 3.63 (dd, *J =* 20.0, 9.6 Hz, 1H), 3.08 - 2.98 (m, 1H), 2.91 - 2.83 (m, 1H), 2.81 (s, 3H), 2.74 - 2.63 (m, 2H), 2.51 (d, *J =* 12.0 Hz, 2H), 2.44 - 2.31 (m, 2H). LCMS (ESI): calcd., 586.24, found, 586.3.

### Intermediate Example 98: preparation of 2-(6-(4-(3,8-diazabicyclo[3.2.1]octan-3-yl)phenyl)-4-fluoro-1-oxoisoindolin-2-yl)-2-(6,7-dihydro-5H-pyrrolo[1,2-c]imidazol-1-yl)-N-(thiazol-2-yl)acetamide (GT-D-224)

Following Scheme 53, the starting compound (CAS NO.: 1146427-86-2) was used in place of the compound (CAS NO.: 2762613-62-5) to afford the target compound GT-D-224 (off-white solid, 619 mg, yield 105.0%). ¹H NMR (400 MHz, MeOD) δ 8.90 (s, 1H), 7.88 (d, J = 1.0 Hz, 1H), 7.65 (dd, J = 10.0, 7.2 Hz, 3H), 7.50 (d, J = 4.0 Hz, 1H), 7.24 (d, J = 4.0 Hz, 1H), 7.07 (d, J = 8.0 Hz, 2H), 4.91 (d, J = 16.0 Hz, 1H), 4.55 (d, J = 16.0 Hz, 1H), 4.35 (dd, J = 12.0, 8.0 Hz, 2H), 4.22 (s, 2H), 4.10 (q, J = 7.2 Hz, 1H), 3.80 (d, J = 10.0 Hz, 2H), 3.16 (t, J = 12.0 Hz, 2H), 3.09 - 2.94 (m, 1H), 2.92 - 2.77 (m, 1H), 2.76 - 2.57 (m, 2H), 2.16 (s, 4H). LCMS (ESI): calcd., 584.22, found, 584.2.

### Intermediate Example 99: preparation of 2-(6-(4-((1R,4R)-2,5-diazabicyclo[2.2.1]heptan-2-yl)phenyl)-4-fluoro-1-oxoisoindolin-2-yl)-2-(6,7-dihydro-5H-pyrrolo[1,2-c]imidazol-1-yl)-N-(thiazol-2-yl)acetamide (GT-D-223)

Following steps 2-5 in Scheme 53, the starting material (CAS NO.: 2407965-04-0) and the compound (CAS NO.: 2654825-27-9) were used to prepare the target compound GT-D-223 (off-white solid, 1.3 g, yield 101.7%). ¹H NMR (400 MHz, MeOD) δ 8.90 (s, 1H), 7.84 (d, J = 4.0 Hz, 1H), 7.64-7.58 (m, 4H), 7.35(d, J = 4.0 Hz, 1H), 6.81 (d, J = 8.7 Hz, 2H), 4.92 (s, 1H), 4.87 - 4.84 (m, 1H), 4.74 (s, 1H), 4.67 - 4.48(m, 2H), 4.39-4.32 (m, 2H), 3.80-3.77 (m, 1H), 3.40 (s, 3H), 3.11 - 2.96 (m,1H), 2.95 - 2.82 (m, 1H), 2.72-2.66 (m, 2H), 2.32 (d, J = 8.0 Hz, 1H), 2.10 (d, J = 12.0 Hz,1H). LCMS (ESI): calcd., 570.21, found, 570.2.

### Intermediate Example 100: preparation of 2-(6-(4-(4-(3,3-difluoropiperidin-4-yl)piperazin-1-yl)phenyl)-4-fluoro-1-oxoisoindolin-2-yl)-2-(6,7-dihydro-5H-pyrrolo[1,2-c]imidazol-1-yl)-N-(thiazol-2-yl)acetamide (GT-D-218)

Following steps 3-6 in Scheme 54, the starting material 2 and the compound tert-butyl 3,3-difluoro-4-oxopiperidine-1-carboxylate were used to prepare the target compound GT-D-218 (off-white solid, 645 mg, yield 103.32%). ¹H NMR (400 MHz, MeOD) δ 8.92 (s, 1H), 7.93 (d,J = 1.2 Hz, 1H), 7.84 (d,J = 8.8 Hz, 2H), 7.74 (dd,J = 10.0, 1.2 Hz, 1H), 7.69 - 7.55 (m, 3H), 7.42 (d, J = 4.0 Hz, 1H), 4.95 (s, 2H), 4.68 (d, J = 16.0 Hz,1H), 4.45 - 4.24 (m, 2H), 3.88 (t, J = 8.0 Hz, 2H), 3.76 (s, 4H), 3.67 - 3.49 (m, 6H), 3.33 (d, J = 12.0 Hz,1H), 3.10 - 2.96 (m, 1H), 2.96 - 2.81 (m, 1H), 2.77 - 2.59 (m, 2H), 2.49 (d, J = 12.0 Hz, 1H), 2.37 - 2.18(m, 1H). LCMS (ESI): calcd., 677.26, found, 677.3.

### Intermediate Example 101: preparation of N-(5-(2-hydroxypropan-2-yl)-2-(piperidin-4-yl)benzo[d]oxazol-6-yl)-6-(trifluoromethyl)picolinamide (GT-D-208)

The target compound GT-D-208 was prepared by referring to Scheme 55 (yellow solid, 1.7 g, yield 109%). ¹H NMR (400 MHz, MeOD) δ 8.79 (s, 1H), 8.48 (d, *J =* 8.0 Hz, 1H), 8.30 (t, *J =* 8.0 Hz, 1H), 8.04 (d, *J =* 7.6 Hz, 1H), 7.71 (s, 1H), 3.56 - 3.48 (m, 3H), 3.47 - 3.41 (m, 1H), 3.28 - 3.20 (m, 2H), 2.45 (dd, *J* = 14.4, 3.6 Hz, 2H), 2.23 - 2.11 (m, 2H), 1.71 (s, 6H). LCMS (ESI): calcd., 449.18, found, 449.2.

### Intermediate Example 102: preparation of N-(6-(2-hydroxypropan-2-yl)-2-(piperidin-4-yl)-2H-indazol-5-yl)-6-(trifluoromethyl)picolinamide (GT-D-210)

The target compound GT-D-210 was prepared by referring to Scheme 56 (yellow solid, 1.52 g, yield 109.4%). ¹H NMR (400 MHz, D₂O) δ 8.20 (s, 1H), 8.10 (s, 1H), 8.04 (d, *J =* 4.0 Hz, 2H), 7.85 - 7.79 (m, 1H), 7.49 (s, 1H), 4.67 - 4.62 (m, 1H), 3.57 (d, *J =* 13.2 Hz, 2H), 3.20 (dd, *J =* 12.8, 10.0 Hz, 2H), 2.34 - 2.14 (m, 4H), 1.54 (s, 6H). LCMS (ESI): calcd., 448.20, found, 448.2.

### Intermediate Example 103: preparation of 2-(6-(4-(3,3-difluoro-4-(piperazin-1-yl)piperidin-1-yl)phenyl)-4-fluoro-1-oxoisoindolin-2-yl)-2-(6,7-dihydro-5H-pyrrolo[1,2-c]imidazol-1-yl)-N-(thiazol-2-yl)acetamide (GT-D-220)

The target compound GT-D-220 was prepared by referring to Scheme 57 (brown solid, 0.5 g, yield 66.6%). ¹H NMR (400 MHz, MeOD) δ 7.83 (s, 1H), 7.65 (s, 1H), 7.60-7.58 (m, 3H), 7.44 (d, J = 3.6 Hz, 1H), 7.15 (d, J = 4.0 Hz, 1H), 7.06 (d, J = 8.0 Hz, 2H), 4.85 (s, 1H), 4.81 (s, 1H), 4.25 (d, J = 16.0 Hz, 1H), 4.12-4.04 (m, 2H), 3.98-3.91 (m, 2H), 3.19 (d, J = 4.0 Hz, 4H), 3.16 - 2.76 (m, 8H), 2.65-2.56 (m, 3H), 2.11-2.06 (m, 1H), 2.00 - 1.86 (m, 1H). LCMS (ESI): calcd., 677.26, found, 677.4.

### Intermediate Example 104: preparation of 2-((5-Bromo-2-((2-methyl-4-(piperazin-1-ylsulfonyl)phenyl)amino)pyrimidin-4-yl)amino)-6-fluorobenzamide

The target compound was prepared by referring to Scheme 58 (722 mg, yield 33%). **¹H NMR (300 MHz, DMSO-*d*₆)** δ 10.03 (s, 1H), 8.14 (dd, *J* = 19.3, 6.7 Hz, 4H), 7.51 (q, *J* = 7.8 Hz, 1H), 7.26 (d, *J* = 6.5 Hz, 2H), 7.01 (t, *J* = 9.3 Hz, 1H), 6.68 (d, *J =* 8.3 Hz, 1H), 6.10 - 5.52 (m, 2H), 3.74 (t, *J* = 4.8 Hz, 4H), 2.85 (d, *J* = 5.4 Hz, 4H), 2.08 (s, 3H). **LCMS (ESI):** [M+H]⁺ calcd., 564.08; found, 564.1.

### Intermediate Example 105: preparation of (5-Amino-3-((4-(piperazin-1-ylsulfonyl)phenyl)amino)-1H-1,2,4-triazol-1-yl)(2,6-difluorophenyl)methanone

According to Scheme 59, the target compound was prepared (252 mg, yield 88%). **¹H NMR (400 MHz, DMSO-*d*₆)** δ 10.08 (s, 1H), 9.18 (s, 2H), 8.05 (s, 1H), 7.73 (ddd, *J =* 15.2, 8.6, 6.6 Hz, 1H), 7.56 (q, *J* = 9.0 Hz, 4H), 7.35 (t, *J* = 8.3 Hz, 2H), 3.13 (q, *J =* 4.7 Hz, 4H), 3.10 - 3.04 (m, 4H). **LCMS (ESI):** [M+H]⁺ calcd., 464.13; found, 464.8.

### Intermediate Example 106: preparation of N-((4-(((R)-4-((1R,4R)-2,5-Diazabicyclo[2.2.1]heptan-2-yl)-1-(phenylthio)butan-2-yl)amino)-3-((trifluoromethyl)sulfonyl)phenyl)sulfonyl)-4-(4-((4'-chloro-4,4-dimethyl-3,4,5,6-tetrahydro-[1,1'-biphenyl]-2-yl)methyl)piperazin-1-yl)benzamide

According to Scheme 60, the target compound was prepared (460 mg, yield 50.67%). **¹H NMR (400 MHz, DMSO-*d*₆)** δ 10.08 (s, 1H), 9.18 (s, 2H), 8.05 (s, 1H), 7.73 (ddd, *J =* 15.2, 8.6, 6.6 Hz, 1H), 7.56 (q, *J* = 9.0 Hz, 4H), 7.35 (t, *J =* 8.3 Hz, 2H), 3.13 (q, *J =* 4.7 Hz, 4H), 3.10 - 3.04 (m, 4H). **LCMS (ESI):** [M+H]⁺ calcd., 985.6; found, 985.1.

### Intermediate Example 107: preparation of 4-(2-Hydroxy-5-(methylsulfonyl)phenyl)-6-methyl-1,6-dihydro-7H-pyrrolo[2,3-c]pyridin-7-one (GTC00392)

According to Scheme 61, the target compound **GTC00392** was prepared (white solid, 1.2 g). ¹H NMR (400 MHz, MeOD) δ 7.91 (d, *J* = 2.4 Hz, 1H), 7.79 (dd, *J =* 8.4, 2.4 Hz, 1H), 7.35 - 7.31 (m, 2H), 7.12 (d, *J =* 8.4 Hz, 1H), 6.27 (d, *J =* 2.8 Hz, 1H), 3.70 (s, 3H), 3.11 (d, *J =* 4.4 Hz, 3H). LCMS (ESI): calcd., 319.07; found, 319.0.

### Intermediate Example 108: preparation of 1-(4-((5-(3,5-Dimethylisoxazol-4-yl)-2-methylphenyl)(piperidin-4-ylmethyl)amino)phenyl)cyclopropane-1-carbonitrile (GTC00388)

According to Scheme 62, the target compound **GTC00388** was prepared (yellow solid, 2.5 g, yield 80.74%). ¹H NMR (400 MHz, MeOD) δ 7.43 (d, *J* = 7.6 Hz, 1H), 7.22 (d, *J =* 9.6 Hz, 2H), 7.15 (d, *J =* 8.8 Hz, 2H), 6.57 (d, *J =* 8.8 Hz, 2H), 3.63 (d, *J =* 6.4 Hz, 2H), 3.49 - 3.33 (m, 2H), 2.96 (dt, *J =* 13.2, 6.4 Hz, 2H), 2.42 (s, 3H), 2.26 (s, 3H), 2.19 - 2.02 (m, 6H), 1.65 - 1.54 (m, 2H), 1.55 - 1.42 (m, 2H), 1.38 - 1.29 (m, 2H). LCMS (ESI): calcd., 441.26; found, 441.2.

### Intermediate Example 109: preparation of 5-Chloro-4-(5-(cyclopropylmethyl)-1-methyl-1H-pyrazol-4-yl)-N-(piperidin-4-yl)pyrimidin-2-amine hydrochloride (GTC00324)

According to Scheme 63, the target compound **GTC00324** was prepared (off-white solid, 2.49 g, yield 96.89%). ¹H NMR (400 MHz, DMSO) δ 9.18 (s, 1H), 8.97 (s, 1H), 8.21 (d, *J =* 10.4 Hz, 1H), 7.88 (s, 1H), 7.64 (s, 1H), 3.87 (d, *J =* 22.7 Hz, 1H), 3.78 - 3.66 (m, 3H), 3.16 (d, *J =* 12.4 Hz, 2H), 2.92 (d, *J =* 6.4 Hz, 2H), 2.79 (d, *J =* 10.2 Hz, 2H), 1.88 (t, *J =* 11.4 Hz, 2H), 1.63 (dd, *J =* 21.1, 10.3 Hz, 2H), 0.82 (s, 1H), 0.25 (d, *J =* 6.7 Hz, 2H), -0.00 (s, 2H). LCMS (ESI): calcd., 347.17; found, 347.2.

### Intermediate Example 110: preparation of 2-Fluoro-5-methoxy-4-((4-((2-methyl-3-oxoisoindolin-4-yl)oxy)-5-(trifluoromethyl)pyrimidin-2-yl)amino)-N-(piperidin-4-yl)benzamide (GTC00226)

According to Scheme 64, the target compound **GTC00226** was prepared (pale yellow solid, 595 mg, yield 87.33%). ¹H NMR (400 MHz, MeOD) δ 8.59 (d, *J =* 0.7 Hz, 1H), 7.71 (t, *J* = 7.8 Hz, 1H), 7.54 (d, *J =* 7.5 Hz, 1H), 7.47 (d, *J* = 12.8 Hz, 1H), 7.26 (dd, *J* = 18.3, 7.2 Hz, 2H), 4.52 (s, 2H), 4.19 - 4.11 (m, 1H), 3.93 (s, 3H), 3.49 - 3.44 (m, 2H), 3.17 (dt, *J* = 11.7, 5.8 Hz, 2H), 3.03 (s, 3H), 2.21 (dd, *J* = 13.7, 2.6 Hz, 2H), 1.85 (dt, *J =* 14.3, 7.4 Hz, 2H). LCMS (ESI): calcd., 575.20; found, 575.2.

### Intermediate Example 111: preparation of (R)-N-(5-(3-Hydroxypyrrolidin-1-yl)-2-(piperazin-1-yl)oxazolo[4,5-b]pyridin-6-yl)-2-(2-methylpyridin-4-yl)oxazole-4-carboxamide (GTC00176)

According to Scheme 65, the target compound **GTC00176** was prepared (white solid, 1.6 g). ¹H NMR (400 MHz, DMSO) δ 9.85 (s, 1H), 8.98 (s, 1H), 8.69 (d, *J =* 5.2 Hz, 1H), 7.87 (s, 1H), 7.78 (d, *J =* 5.1 Hz, 1H), 7.70 (s, 1H), 4.89 (d, *J =* 3.3 Hz, 1H), 4.29 (s, 1H), 3.89 - 3.82 (m, 4H), 3.69 - 3.53 (m, 2H), 3.48 - 3.40 (m, 1H), 3.29 - 3.15 (m, 6H), 2.62 (d, *J* = 17.3 Hz, 3H), 1.87 (ddd, *J* = 49.9, 9.2, 4.4 Hz, 2H). LCMS (ESI): calcd., 491.22; found, 491.2.

### Intermediate Example 112: preparation of (R)-9-Methyl-6-oxo-N-(1-(4-(piperazin-1-yl)benzyl)-1H-pyrazol-4-yl)-6,7,8,9-tetrahydropyrido[3',2':4,5]pyrrolo[1,2-a]pyrazine-2-carboxamide (GTC00397)

According to Scheme 66, the target compound **GTC00397** was prepared (white solid, 2.0 g). ¹H NMR (400 MHz, MeOD) δ 8.45 (s, 1H), 8.29 - 8.20 (m, 2H), 8.00 (d, J = 8.0 Hz, 1H), 7.35 (d, J =8.0 Hz, 2H), 7.23 (s, 1H), 7.15 (d, J = 8.0 Hz, 2H), 5.44 (s, 2H), 5.41 - 5.34 (m, 1H), 3.99 (dd, J = 12.0,4.0 Hz, 1H), 3.58 (d, J = 12.8 Hz, 1H), 3.54 - 3.49 (m, 4H), 3.42 (dd, J = 6.0, 3.6 Hz, 4H), 1.50 (d, J = 6.0Hz, 3H). LCMS (ESI): calcd., 485.24; found, 485.2.

### Intermediate Example 113: preparation of the following intermediate compounds

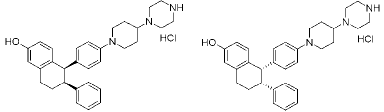

Following the methods of Scheme 67 and Intermediate Example 89, the enantiomeric mixture product obtained according to Step 2 of Scheme 67 was separated by supercritical fluid chromatography (SFC) to obtain the two intermediate compounds as described above.

One of the obtained intermediates (GT-M-459-P1) (white solid, 8.55 g): ¹H NMR (400 MHz, MeOD) δ 7.35 (d, *J =* 8.8 Hz, 2H), 7.19 - 7.07 (m, 3H), 6.88 - 6.80 (m, 2H), 6.71 - 6.62 (m, 4H), 6.53 (dd, *J =* 8.4, 2.4 Hz, 1H), 4.39 (d, *J =* 5.2 Hz, 1H), 3.79 (d, *J =* 12.4 Hz, 4H), 3.68 (s, 8H), 3.60 (q, *J =* 7.2 Hz, 1H), 3.50 - 3.41 (m, 1H), 3.13 - 2.96 (m, 2H), 2.55 (d, *J =* 12.4 Hz, 2H), 2.44 (dd, *J =* 22.8, 11.2 Hz, 2H), 2.22 - 2.09 (m, 1H), 1.84 (dd, *J =* 11.2, 3.6 Hz, 1H). LCMS (ESI): calcd., 468.3; found, 468.4.

Another intermediate (GT-M-459-P2) (white solid, 9.9 g): ¹H NMR (400 MHz, MeOD) δ 7.34 (d, *J* = 8.8 Hz, 2H), 7.18 - 7.10 (m, 3H), 6.84 (dd, *J =* 7.6, 1.6 Hz, 2H), 6.67 (dd, *J =* 12.4, 5.6 Hz, 4H), 6.53 (dd, *J =* 8.4, 2.4 Hz, 1H), 4.39 (d, *J =* 5.6 Hz, 1H), 3.76 (dd, *J =* 21.6, 10.0 Hz, 4H), 3.68 (s, 9H), 3.49 - 3.42 (m, 1H), 3.05 (dd, *J =* 14.8, 4.8 Hz, 1H), 2.55 (d, *J =* 11.2 Hz, 2H), 2.43 (dd, *J =* 22.4, 10.8 Hz, 2H), 2.18 - 2.10 (m, 1H), 1.88 - 1.79 (m, 1H). LCMS (ESI): calcd., 468.3; found, 468.4.

### Example II-1: Preparation of 1-(4-(Bromomethyl)-2-fluorophenyl)dihydropyrimidine-2,4(1H,3H)-dione (791442)

According to the method of Scheme II-1, the target compound (791442) was prepared (white solid, 4.9 g, yield 42.1%). ¹H NMR (400 MHz, DMSO) δ 10.51 (s, 1H), 7.42 (dd, J = 13.7, 5.5 Hz, 2H), 7.34 (d, *J* = 8.2 Hz, 1H), 4.72 (s, 2H), 3.72 (t, *J* = 6.6 Hz, 2H), 2.72 (t, *J* = 6.6 Hz, 2H). LC/MS (ESI) calcd for C₁₁H₁₁BrFN₂O₂⁺ [M+H]⁺, 301.00; found, 301.0.

### Example II-2: Preparation of 1-(4-(Bromomethyl)phenyl)dihydropyrimidine-2,4(1H,3H)-dione (828029)

According to the method of Scheme II-1, the target compound (828029) was prepared. LC/MS (ESI) calcd for C₁₁H₁₂BrN₂O₂⁺ [M+H]⁺, 283.01; found, 283.0.

### Example II-3: Preparation of 1-(Bromomethyl)-3-fluorophenyl)dihydropyrimidine-2,4(1H,3H)-dione (791497)

According to the method of Scheme II-1, the target compound (791497) was prepared (white solid, 3.6 g, yield 31.9%). ¹H NMR (400 MHz, DMSO) δ 10.49 (s, 1H), 7.54 (t, *J =* 8.5 Hz, 1H), 7.29 (dd, *J =* 11.8, 2.0 Hz, 1H), 7.19 (dd, *J* = 8.3, 2.0 Hz, 1H), 4.75 (d, *J* = 33.9 Hz, 2H), 3.82 (t, *J =* 6.6 Hz, 2H), 2.71 (t, *J* = 6.6 Hz, 2H). LC/MS (ESI) calcd for C₁₁H₁₁BrFN₂O₂⁺ [M+H]⁺, 301.00; found, 301.0.

### Example II-4: Preparation of 1-(3-(Chloromethyl)phenyl)dihydropyrimidine-2,4(1H,3H)-dione (791464)

According to the method of Scheme II-1, the target compound (791464) was prepared (white solid, 4.0 g, yield 71.0%). ¹H NMR (400 MHz, DMSO) δ 10.40 (s, 1H), 7.46 - 7.37 (m, 2H), 7.31 (dd, *J* = 8.3, 1.7 Hz, 2H), 4.77 (s, 2H), 3.80 (t, *J* = 6.7 Hz, 2H), 2.71 (t, *J* = 6.7 Hz, 2H). LC/MS (ESI) calcd for C₁₃H₁₅ClN₃O₂⁺ [M+H+CH₃CN]⁺, 280.08; found, 280.1.

### Example II-5: Preparation of 1-(6-(Hydroxymethyl)pyridin-3-yl)dihydropyrimidine-2,4(1H,3H)-dione (835253)

According to the methods of Scheme II-2, the target compound (835253) was prepared (white solid, 1.65 g, yield 68.5%). ¹H NMR (400 MHz, DMSO) δ 10.50 (s, 1H), 8.33 (d, J = 1.5 Hz, 1H), 7.74 (dd, J = 8.5, 2.2 Hz, 1H), 7.69 (d, J = 8.3 Hz, 1H), 5.28 (t, J = 5.6 Hz, 1H), 4.50 (d, J = 5.6 Hz, 2H), 4.03 (t, J = 6.6 Hz, 2H), 2.68 (t, J = 6.6 Hz, 2H). LC/MS (ESI) calcd for C₁₀H₁₂N₃O₃⁺ [M+H]⁺, 222.09; found, 222.1.

### Example II-6: Preparation of 1-(5-(Hydroxymethyl)pyridin-2-yl)dihydropyrimidine-2,4(1H,3H)-dione (GT-08011)

According to the methods of Scheme II-2, the target compound (GT-08011) was prepared (white solid, 1.65 g, yield 68.5%). ¹H NMR (400 MHz, DMSO) δ 10.50 (s, 1H), 8.33 (d, J = 1.5 Hz, 1H), 7.74 (dd, J = 8.5, 2.2 Hz, 1H), 7.69 (d, J = 8.3 Hz, 1H), 5.28 (t, J = 5.6 Hz, 1H), 4.50 (d, J = 5.6 Hz, 2H), 4.03 (t, J = 6.6 Hz, 2H), 2.68 (t, J = 6.6 Hz, 2H). LC/MS (ESI) calcd for C₁₀H₁₂N₃O₃⁺ [M+H]⁺, 222.09; found, 222.1.

### Example II-7: Preparation of 3-((2-(Hydroxymethyl)phenyl)amino)piperidine-2,6-dione (791245)

According to the methods of Scheme II-3, the target compound (791245) was prepared (white solid, 9.7 g, yield 51.0%). ¹H NMR (400 MHz, DMSO) δ 10.91 (s, 1H), 7.11 (t, *J* = 7.6 Hz, 2H), 6.70 (d, *J* = 8.0 Hz, 1H), 6.63 (t, *J* = 7.3 Hz, 1H), 5.75 (d, *J* = 5.3 Hz, 1H), 5.18 (dd, *J* = 5.6, 4.7 Hz, 1H), 4.51 - 4.40 (m, 2H), 4.37 (dt, *J* = 11.9, 5.0 Hz, 1H), 2.85 (ddd, *J* = 18.0, 10.2, 3.6 Hz, 1H), 2.62 - 2.53 (m, 1H), 2.29 - 2.18 (m, 1H), 1.86 (tt, *J* = 12.8, 6.5 Hz, 1H). LCMS (ESI) calcd for C₁₄H₁₇N₃NaO₃⁺ [M+Na+CH₃CN]⁺, 298.12; found, 298.3.

### Example II-8: Preparation of 3-((3-(Bromomethyl)phenyl)amino)piperidine-2,6-dione (791333)

According to the methods of Scheme II-3, the target compound (791333) was prepared (white solid, 130 g, yield 20.6%). ¹H NMR (400 MHz, DMSO) *δ* 10.78 (s, 1H), 7.06 (t, J = 7.8 Hz, 1H), 6.73 (d, J = 1.8 Hz, 1H), 6.66-6.59 (m, 2H), 5.97 (d, J = 7.2 Hz, 1H), 4.56 (s, 2H), 4.38-4.30 (m, 1H), 2.80-2.70 (m, 1H), 2.59 (dd, J = 12.8, 8.7 Hz, 1H), 2.15-2.06 (m, 1H), 1.88 (dd, J = 12.4, 4.4 Hz, 1H). LCMS (ESI) calcd for C₁₄H₁₇BrN₃O₂⁺ [M+H+CH₃CN]⁺, 340.05; found, 340.05.

### Example II-9: Preparation of 3-((4-(Hydroxymethyl)phenyl)amino)piperidine-2,6-dione (791239)

According to the method of step 1 of Scheme II-3, the target compound (791239) was prepared (white solid, 1.2 g, yield 45.1%). LCMS (ESI) calcd for C₁₂H₁₅N₂O₃⁺ [M+H]⁺, 235.11; found, 235.1.

### Example II-10: Preparation of 3-(4-(Bromomethyl)phenyl)piperidine-2,6-dione (795061)

According to the method of Scheme II-4, the target compound (795061) was prepared (white solid, 3.7 g, yield 88.8%). LCMS (ESI) calcd for C₁₂H₁₂BrNO₂⁺ [M+H]⁺, 282.01; found, 282.0.

### Example II-11: Preparation of 3-(4-(Bromomethyl)-2-fluorophenyl)piperidine-2,6-dione (730487)

According to the method of Scheme II-4, the target compound (730487) was prepared (white solid, 2.6 g, yield 80.2%). LCMS (ESI) calcd for C₁₂H₁₂BrFNO₂⁺ [M+H]⁺, 300.00; found, 300.0.

### Example II-12: Preparation of 1-(4-(Hydroxymethyl)pyridin-2-yl)dihydropyrimidine-2,4(1H,3H)-dione (GT-08120)

According to the method of Scheme II-5, the target compound (GT-08120) was prepared (off-white solid, 9.24 g, yield 81.44%). LCMS (ESI): calcd for C₁₀H₁₂N₃O₃⁺ [M+H]⁺, 222.09; found, 222.1.

### Example II-13: Preparation of 1-(4-(Chloromethyl)pyridin-2-yl)dihydropyrimidine-2,4(1H,3H)-dione (GT-07078)

According to the method of Scheme II-5, the target compound (GT-07078) was prepared (white solid, 9.66 g, yield 99.10%). ¹H NMR (400 MHz, DMSO) δ 10.57 (s, 1H), 8.49 - 8.37 (m, 1H), 7.84 (d, *J =* 0.8 Hz, 1H), 7.25 (dd, *J =* 5.2, 1.6 Hz, 1H), 4.82 (s, 2H), 4.06 (t, *J =* 6.4 Hz, 2H), 2.70 (t, *J =* 6.4 Hz, 2H). LCMS (ESI): calcd for C₁₀H₁₁ClN₃O₂⁺ [M+H]⁺, 240.05; found, 240.0.

### Example II-14: Preparation of 1-(4-(Azidomethyl)pyridin-2-yl)dihydropyrimidine-2,4(1H,3H)-dione (GT-08121)

According to the method of Scheme II-5, the target compound (GT-08121) was prepared (off-white solid, 3.65 g, yield 71.12%). LCMS (ESI): calcd for C₁₀H₁₁N₆O₂⁺ [M+H]⁺, 247.09; found, 247.0.

### Example II-15: Preparation of 1-(4-(Aminomethyl)pyridin-2-yl)dihydropyrimidine-2,4(1H,3H)-dione (GT-07198)

According to the method of Scheme II-5, the target compound (GT-07198) was prepared (white solid, 2.68 g, yield 80.89%). ¹H NMR (400 MHz, DMSO-d6) δ 8.31 (d, *J =* 5.2 Hz, 1H), 7.68 (s, 1H), 7.23 - 7.11 (m, 1H), 4.02 (t, *J =* 6.4 Hz, 2H), 3.75 (s, 2H), 2.67 (t, *J =* 6.4 Hz, 2H). LCMS (ESI): calcd for C₁₀H₁₃N₄O₂⁺ [M+H]⁺, 221.10; found, 220.9.

### Example II-16: Preparation of 1-(5-(Chloromethyl)pyridin-2-yl)dihydropyrimidine-2,4(1H,3H)-dione (GT-07009)

According to the method of Scheme II-5, the target compound (GT-07009) was prepared (off-white solid, 6.46 g). ¹H NMR (400 MHz, DMSO-d6) δ 10.57 (s, 1H), 8.47 (d, *J =* 2.4 Hz, 1H), 7.89 (dd, *J =* 8.4, 2.4 Hz, 1H), 7.78 (d, *J =* 8.4 Hz, 1H), 4.82 (s, 2H), 4.06 (t, *J =* 6.4 Hz, 2H), 2.69 (t, *J =* 6.8 Hz, 2H). LCMS (ESI) calcd for C₁₀H₁₁ClN₃O₂⁺ [M+H]⁺: 240.05; found, 240.1.

### Example II-17: Preparation of 1-(5-(Azidomethyl)pyridin-2-yl)dihydropyrimidine-2,4(1H,3H)-dione (GT-08122)

According to the method of Scheme II-5, the target compound (GT-08122) was prepared. LCMS (ESI): 247.0.

### Example II-18: Preparation of 1-(5-(Aminomethyl)pyridin-2-yl)dihydropyrimidine-2,4(1H,3H)-dione (GT-07012)

According to the method of Scheme II-5, the target compound (GT-07012) was prepared (light yellow solid, 1.40 g). ¹H NMR (400 MHz, DMSO-d6) δ 8.39 (d, *J =* 2.0 Hz, 1H), 7.82 (dd, *J =* 8.4, 2.4 Hz, 1H), 7.72 (d, *J =* 8.4 Hz, 1H), 4.08 (t, *J =* 6.4 Hz, 2H), 3.77 (s, 2H), 2.74 (t, *J =* 6.4 Hz, 2H). LCMS (ESI) calcd for C₁₀H₁₃N₄O₂⁺ [M+H]⁺: 221.10; found, 221.1.

### Example II-19: Preparation of 1-(6-(Hydroxymethyl)pyridin-3-yl)dihydropyrimidine-2,4(1H,3H)-dione (GT-08010)

According to the method of Scheme II-5, the target compound (GT-08010) was prepared (white solid, 1.45 g). ¹H NMR (400 MHz, DMSO) δ 10.46 (s, 1H), 8.47 (d, *J =* 2.4 Hz, 1H), 7.75 (dd, *J =* 8.4, 2.4 Hz, 1H), 7.48 (d, *J =* 8.4 Hz, 1H), 5.42 (t, *J =* 5.6 Hz, 1H), 4.55 (d, *J =* 5.6 Hz, 2H), 3.82 (t, *J =* 6.8 Hz, 2H), 2.73 (t, *J =* 6.8 Hz, 2H). LCMS (ESI) calcd for C₁₀H₁₂N₃O₃⁺ [M+H]⁺: 222.08; found, 222.2.

### Example II-20: Preparation of 1-(6-(Chloromethyl)pyridin-3-yl)dihydropyrimidine-2,4(1H,3H)-dione (GT-06984)

According to the method of Scheme **II-5,** the target compound (GT-06984) was prepared (brown solid, 0.88 g). ¹H NMR (400 MHz, DMSO-d6) δ 10.66 (s, 1H), 8.71 (d, *J =* 2.4 Hz, 1H), 8.02 (dd, *J =* 8.4, 2.4 Hz, 1H), 7.77 (d, *J =* 8.4 Hz, 1H), 4.93 (d, *J =* 5.6 Hz, 2H), 3.95 (t, *J =* 6.4 Hz, 2H), 2.81 (t, *J =* 6.4 Hz, 2H). LCMS (ESI) calcd for C₁₀H₁₁ClN₃O₂⁺ [M+H]⁺: 240.05; found, 240.0.

### Example II-21: Preparation of 1-(6-(Azidomethyl)pyridin-3-yl)dihydropyrimidine-2,4(1H,3H)-dione (GT-08123)

According to the method of Scheme II-5, the target compound (GT-08123) was prepared. LCMS (ESI): 247.0.

### Example II-22: Preparation of 1-(6-(Aminomethyl)pyridin-3-yl)dihydropyrimidine-2,4(1H,3H)-dione (GT-07048)

According to the method of Scheme II-5, the target compound (GT-07048) was prepared (brown solid, 820 mg). ¹H NMR (400 MHz, DMSO-d6) δ 8.52 (d, *J =* 2.4 Hz, 1H), 7.77 (dd, *J =* 8.4, 2.4 Hz, 1H), 7.52 (d, *J =* 8.4 Hz, 1H), 3.91 - 3.78 (m, 4H), 2.78 (t, *J =* 6.8 Hz, 2H). LCMS (ESI) calcd for C₁₀H₁₃N₄O₂⁺ [M+H]⁺: 221.10; found, 221.1.

### Example II-23: Preparation of 1-(5-(Hydroxymethyl)pyridin-3-yl)dihydropyrimidine-2,4(1H,3H)-dione (GT-08124)

According to the method of Scheme II-5, the target compound (GT-08124) was prepared. LCMS (ESI): 222.0.

### Example II-24: Preparation of 1-(5-(Chloromethyl)pyridin-3-yl)dihydropyrimidine-2,4(1H,3H)-dione (GT-07047)

According to the method of Scheme II-5, the target compound (GT-07047) was prepared (off-white solid, 6.18 g). ¹H NMR (400 MHz, DMSO-d6) δ 10.70 (s, 1H), 8.79 (s, 1H), 8.69 (s, 1H), 8.29 (t, *J =* 2.0 Hz, 1H), 4.93 (s, 2H), 3.93 (t, *J =* 6.4 Hz, 2H), 2.77 (t, *J =* 6.4 Hz, 2H). LCMS (ESI) calcd for C₁₀H₁₁ClN₃O₂⁺ [M+H]⁺: 240.05; found, 240.1.

### Example II-25: Preparation of 1-(5-(Azidomethyl)pyridin-3-yl)dihydropyrimidine-2,4(1H,3H)-dione (GT-08125)

According to the method of Scheme II-5, the target compound (GT-08125) was prepared. LCMS (ESI): 247.0.

### Example II-26: Preparation of 1-(5-(Aminomethyl)pyridin-3-yl)dihydropyrimidine-2,4(1H,3H)-dione (GT-07077)

According to the method of Scheme II-5, the target compound (GT-07077) was prepared (white solid, 1.0 g). ¹H NMR (400 MHz, DMSO-d6) δ 8.44 (d, *J =* 2.4 Hz, 1H), 8.38 (d, *J =* 1.2 Hz, 1H), 7.74 (s, 1H), 3.83 (t, *J =* 6.8 Hz, 2H), 3.78 (s, 2H), 2.74 (t, *J =* 6.4 Hz, 2H). LCMS (ESI) calcd for C₁₀H₁₃N₄O₂⁺ [M+H]⁺: 221.10; found, 221.1.

### Example II-27: Preparation of 1-(5-Fluoro-4-(hydroxymethyl)pyridin-2-yl)dihydropyrimidine-2,4(1H,3H)-dione (GT-08126)

According to the method of Scheme II-5, the target compound (GT-08126) was prepared. LCMS (ESI): 240.0.

### Example II-28: Preparation of 1-(4-(Chloromethyl)-5-fluoropyridin-2-yl)dihydropyrimidine-2,4(1H,3H)-dione (GT-07199)

According to the method of Scheme II-5, the target compound (GT-07199) was prepared (off-white solid, 9.68 g). ¹H NMR (400 MHz, DMSO) δ 10.59 (s, 1H), 8.45 (s, 1H), 7.93 (d, *J =* 5.6 Hz, 1H), 4.86 (s, 2H), 4.02 (dd, *J=* 11.2, 4.8 Hz, 2H), 2.70 (t, *J =* 6.4 Hz, 2H). LCMS (ESI) calcd for C₁₀H₁₀ClFN₃O₂⁺ [M+H]⁺: 258.04; found, 257.9.

### Example II-29: Preparation of 1-(4-(Azidomethyl)-5-fluoropyridin-2-yl)dihydropyrimidine-2,4(1H,3H)-dione (GT-08127)

According to the method of Scheme II-5, the target compound (GT-08127) was prepared. LCMS (ESI): 265.0.

### Example II-30: Preparation of 1-(4-(Aminomethyl)-5-fluoropyridin-2-yl)dihydropyrimidine-2,4(1H,3H)-dione (GT-07369)

According to the method of Scheme II-5, the target compound (GT-07369) was prepared (off-white solid, 3.52 g). ¹H NMR (400 MHz, DMSO-d6) δ 10.51 (s, 1H), 8.28 (d, *J =* 1.2 Hz, 1H), 7.87 (d, *J =* 5.6 Hz, 1H), 3.98 (t, *J =* 6.4 Hz, 2H), 3.81 (s, 2H), 2.68 (t, *J =* 6.4 Hz, 2H), 2.00 (d, *J =* 9.2 Hz, 2H). LCMS (ESI) calcd for C₁₀H₁₂FN₄O₂⁺ [M+H]⁺: 239.09; found, 238.8.

### Example II-31: Preparation of 1-(5-Fluoro-6-(hydroxymethyl)pyridin-3-yl)dihydropyrimidine-2,4(1H,3H)-dione (GT-07555)

According to the method of Scheme II-5, the target compound (GT-07555) was prepared (off-white solid, 1.26 g). ¹H NMR (400 MHz, DMSO-d6) δ 10.58 (s, 1H), 8.43 (s, 1H), 7.76 (dd, *J =* 11.2, 2.0 Hz, 1H), 5.32 (t, *J =* 6.0 Hz, 1H), 4.58 (dd, *J =* 6.0, 2.0 Hz, 2H), 3.87 (t, *J =* 6.8 Hz, 2H), 2.75 (t, *J =* 6.4 Hz, 2H). LCMS (ESI) calcd for C₁₀H₁₁FN₃O₃⁺ [M+H]⁺: 240.07; found, 240.0.

### Example II-32: Preparation of 1-(6-(Chloromethyl)-5-fluoropyridin-3-yl)dihydropyrimidine-2,4(1H,3H)-dione (GT-07557)

According to the method of Scheme II-5, the target compound (GT-07557) was prepared (off-white solid, 1.1 g). ¹H NMR (400 MHz, DMSO-d6) δ 10.63 (s, 1H), 8.49 (s, 1H), 7.86 (dd, *J =* 11.2, 2.0 Hz, 1H), 4.83 (d, *J =* 1.6 Hz, 2H), 3.90 (t, *J =* 6.4 Hz, 2H), 2.74 (t, *J =* 6.4 Hz, 2H). LCMS (ESI) calcd for C₁₀H₁₀ClFN₃O₂⁺ [M+H]⁺: 258.04; found, 258.0.

### Example II-33: Preparation of 1-(6-Fluoro-5-(hydroxymethyl)pyridin-3-yl)dihydropyrimidine-2,4(1H,3H)-dione (GT-07556)

According to the method of Scheme II-5, the target compound (GT-07556) was prepared (off-white solid, 5.04 g). ¹H NMR (400 MHz, DMSO) δ 10.51 (s, 1H), 8.09 (s, 1H), 7.97 (dd, *J =* 8.4, 2.4 Hz, 1H), 5.52 (t, *J* = 5.6 Hz, 1H), 4.54 (d, *J =* 5.6 Hz, 2H), 3.82 (t, *J =* 6.8 Hz, 2H), 2.74 (t, *J =* 6.8 Hz, 2H). LCMS (ESI) calcd for C₁₀H₁₁FN₃O₃⁺ [M+H]⁺: 240.07; found, 240.1.

### Example II-34: Preparation of 1-(5-(Chloromethyl)-6-fluoropyridin-3-yl)dihydropyrimidine-2,4(1H,3H)-dione (GT-07633)

According to the method of Scheme II-5, the target compound (GT-07633) was prepared (off-white solid, 3.85 g). ¹H NMR (400 MHz, DMSO-d6) δ 10.56 (s, 1H), 8.23 (dd, *J =* 2.4, 1.6 Hz, 1H), 8.13 (dd, *J =* 8.8, 2.8 Hz, 1H), 4.82 (s, 2H), 3.84 (t, *J =* 6.8 Hz, 2H), 2.74 (t, *J =* 6.8 Hz, 2H). LCMS (ESI) calcd for C₁₀H₁₀ClFN₃O₂⁺ [M+H]⁺: 258.04; found, 257.9.

### Example II-35: Preparation of 1-(5-(Azidomethyl)-6-fluoropyridin-3-yl)dihydropyrimidine-2,4(1H,3H)-dione (GT-08128)

According to the method of Scheme II-5, the target compound (GT-08128) was prepared. LCMS (ESI): 265.0.

### Example II-36: Preparation of 1-(5-(Aminomethyl)-6-fluoropyridin-3-yl)dihydropyrimidine-2,4(1H,3H)-dione (GT-07729)

According to the method of Scheme II-5, the target compound (GT-07729) was prepared (white solid, 616 mg). ¹H NMR (400 MHz, DMSO-d6) δ 8.06 (s, 1H), 8.01 (dd, *J =* 8.8, 2.8 Hz, 1H), 3.81 (t, *J* = 6.8 Hz, 2H), 3.74 (s, 2H), 2.74 (t, *J =* 6.8 Hz, 2H). LCMS (ESI) calcd for C₁₀H₁₂FN₄O₂⁺ [M+H]⁺: 239.09; found, 239.3.

### Example II-37: Preparation of 1-(5-(Hydroxymethyl)pyrazin-2-yl)dihydropyrimidine-2,4(1H,3H)-dione (GT-07634)

According to the method of Scheme II-5, the target compound (GT-07634) was prepared (yellow solid, 1.66 g). ¹H NMR (400 MHz, DMSO) δ 10.69 (s, 1H), 8.94 (d, *J =* 1.6 Hz, 1H), 8.57 - 8.41 (m, 1H), 5.55 (t, *J =* 5.6 Hz, 1H), 4.61 (d, *J =* 5.6 Hz, 2H), 4.03 (t, *J =* 6.4 Hz, 2H), 2.72 (t, *J =* 6.4 Hz, 2H). LCMS (ESI) calcd for C₉H₁₁N₄O₃⁺ [M+H]⁺: 223.08; found, 223.1.

### Example 11-38: Preparation of 1-(5-(Chloromethyl)pyrazin-2-yl)dihydropyrimidine-2,4(1H,3H)-dione (GT-07635)

According to the method of Scheme II-5, the target compound (GT-07635) was prepared (brown solid, 1.19 g). ¹H NMR (400 MHz, DMSO) δ 10.76 (s, 1H), 9.05 (d, *J =* 1.2 Hz, 1H), 8.62 (d, *J =* 1.2 Hz, 1H), 4.86 (s, 2H), 4.06 (t, *J =* 6.4 Hz, 2H), 2.73 (t, *J =* 6.4 Hz, 2H). LCMS (ESI) calcd for C₉H₁₀ClN₄O₂⁺ [M+H]⁺: 241.05; found, 240.9.

### Example II-39: Preparation of 1-(6-(Hydroxymethyl)pyrazin-2-yl)dihydropyrimidine-2,4(1H,3H)-dione (GT-07632)

According to the method of Scheme II-5, the target compound (GT-07632) was prepared (off-white solid, 2.02 g). ¹H NMR (400 MHz, DMSO) δ 10.70 (s, 1H), 8.94 (s, 1H), 8.41 (s, 1H), 5.60 (s, 1H), 4.59 (d, *J* = 4.0 Hz, 2H), 4.04 (t, *J =* 6.8 Hz, 2H), 2.71 (t, *J =* 6.4 Hz, 2H). LCMS (ESI) calcd for C₉H₁₁N₄O₃⁺ [M+H]⁺: 223.08; found, 223.1.

### Example II-40: Preparation of 1-(6-(Chloromethyl)pyrazin-2-yl)dihydropyrimidine-2,4(1H,3H)-dione (GT-07728)

According to the method of Scheme II-5, the target compound (GT-07728) was prepared (brown solid, 1.84 g). ¹H NMR (400 MHz, DMSO-d6) δ 10.77 (s, 1H), 9.04 (s, 1H), 8.51 (s, 1H), 4.84 (s, 2H), 4.06 (t, *J =* 6.4 Hz, 2H), 2.74 (t, *J =* 6.4 Hz, 2H). LCMS (ESI) calcd for C₉H₁₀ClN₄O₂⁺ [M+H]⁺: 241.04; found, 241.2.

### Example II-41: Preparation of 1-(6-(Azidomethyl)pyrazin-2-yl)dihydropyrimidine-2,4(1H,3H)-dione (GT-08129)

According to the method of Scheme II-5, the target compound (GT-08129) was prepared. LCMS (ESI): 248.0.

### Example II-42: Preparation of 1-(6-(Aminomethyl)pyrazin-2-yl)dihydropyrimidine-2,4(1H,3H)-dione (GT-07801)

According to the method of Scheme II-5, the target compound (GT-07801) was prepared (off-white solid, 340 mg). ¹H NMR (400 MHz, DMSO-d6) δ 8.90 (s, 1H), 8.41 (s, 1H), 4.07 (t, *J =* 6.8 Hz, 2H), 3.82 (s, 2H), 2.71 (t, *J =* 6.8 Hz, 2H). LCMS (ESI) calcd for C₉H₁₁NaN₅O₂⁺ [M+Na]⁺: 244.09; found, 244.0.

### Example II-43: Preparation of 3-(4-(Hydroxymethyl)pyridin-2-yl)piperidine-2,6-dione (GT-07050)

According to the method of Scheme II-6, the target compound (GT-07050) was prepared (yellow solid, 720 mg, yield 65.13%). ¹HNMR (400 MHz, DMSO-d6) δ 10.84 (s, 1H), 8.42 (d,J = 5.2 Hz, 1H), 7.29 (s, 1H), 7.25 - 7.20 (m, 1H), 5.45 (t,J = 8.4 Hz, 1H), 4.54 (d,J = 4.0 Hz, 2H), 4.01 (dd,J = 9.2, 5.2 Hz, 1H), 3.29 - 3.22 (m, 1H), 2.60 (ddd,J = 17.2, 9.6, 5.6 Hz, 1H), 2.29 - 2.08 (m, 2H). LCMS (ESI) calcd for C₁₁H₁₂N₂O₃⁺ [M+H]⁺: 221.08; found, 221.0.

### Example II-44: Preparation of 3-(4-(Chloromethyl)pyridin-2-yl)piperidine-2,6-dione (GT-07013)

According to the method of Scheme II-6, the target compound (GT-07013) was prepared (brown solid, 825 mg, yield 94.34%). ¹H NMR (400 MHz, DMSO-d6) δ 11.04 (s, 1H), 8.72 (d,J = 5.2 Hz, 1H), 7.75 (s, 1H), 7.69 (d,J = 4.8Hz, 1H), 4.93 (s, 2H), 4.34 (dd,J = 10.8, 4.8 Hz, 1H), 2.68 (dd,J = 11.6, 5.2 Hz, 1H), 2.60 (d,J = 6.8 Hz,1H), 2.41 (d,J = 8.4 Hz, 1H), 2.18 (t,J = 6.4 Hz, 1H). LCMS (ESI) calcd for C₁₁H₁₁ClN₂O₂⁺ [M+H]⁺: 239.05; found, 239.1.

### Example II-45: Preparation of 3-(6-(Hydroxymethyl)pyridin-3-yl)piperidine-2,6-dione (GT-07010)

According to the method of Scheme II-6, the target compound (GT-07010) was prepared (gray solid, 1460 mg, yield 82.55%). ¹H NMR (400 MHz, MeOD) δ 8.38 (s, 1H), 7.76 (d, J = 6.4 Hz, 1H), 7.55 (d, J = 8.0 Hz, 1H), 4.70 (s, 2H), 3.96 (dd, J = 12.0, 4.8 Hz, 1H), 2.86 - 2.63 (m, 2H), 2.29 (td, J = 12.4, 4.8 Hz, 1H), 2.24 - 2.12 (m, 1H). LCMS (ESI) calcd for C₁₁H₁₂N₂O₃⁺ [M+H]⁺: 221.08; found, 221.1.

### Example II-46: Preparation of 3-(6-(Chloromethyl)pyridin-3-yl)piperidine-2,6-dione (GT-06986)

According to the method of Scheme II-6, the target compound (GT-06986) was prepared (white solid, 85 mg, yield 7.84%). ¹H NMR (400 MHz, DMSO-d6) δ 10.92 (s, 1H), 8.44 (d, J = 2.1 Hz, 1H), 7.72 (dd, J = 8.0, 2.3 Hz, 1H), 7.52 (d, J = 8.0 Hz, 1H), 4.77 (s, 2H), 3.98 (dd, J = 12.6, 4.9 Hz, 1H), 2.69 (dd, J = 12.6, 5.2 Hz, 1H), 2.60 - 2.52 (m, 1H), 2.29 (dd, J = 12.8, 4.4 Hz, 1H), 2.08 - 2.00 (m, 1H). LCMS (ESI): [M+H]⁺, calcd., 239.05; found, 239.0.

### Example II-47: Preparation of (5-(2,6-Dioxopiperidin-3-yl)pyridin-2-yl)methyl methanesulfonate (GT-06987)

According to the method of Scheme II-6, the target compound (GT-06987) was prepared (white solid, 152 mg, yield 11.22%). ¹H NMR (400 MHz, DMSO-d6) δ 10.93 (s, 1H), 8.49 (d, J = 2.0 Hz, 1H), 7.77 (dd, J = 8.0, 2.2 Hz, 1H), 7.51 (d, J = 8.0 Hz, 1H), 5.30 (s, 2H), 4.00 (dd, J = 12.6, 4.9 Hz, 1H), 3.29 (s, 3H), 2.78 - 2.67 (m, 1H), 2.56 (dt, J = 17.3, 3.6 Hz, 1H), 2.30 (qd, J = 12.9, 4.4 Hz, 1H), 2.04 (ddd, J = 10.0, 8.0, 5.0 Hz, 1H). LCMS (ESI): [M+H]⁺, calcd., 299.06; found, 299.0.

### Example II-48: Preparation of 3-(3-(Hydroxymethyl)pyridin-2-yl)piperidine-2,6-dione (GT-07370)

According to the method of Scheme II-6, the target compound (GT-07370) was prepared (yellow solid, 218 mg). ¹H NMR (400 MHz, DMSO-d6) δ 10.82 (s, 1H), 8.39 (dd, J = 4.8, 1.6 Hz, 1H), 7.79 (dt, J = 5.2, 2.4 Hz, 1H), 7.29 (dd, J = 7.6, 4.8 Hz, 1H), 5.35 (t, J = 5.6 Hz, 1H), 4.59 (qd, J = 13.6, 5.6 Hz, 2H), 4.22 (dd, J = 8.8, 5.2 Hz, 1H), 2.65 - 2.51 (m, 2H), 2.33 - 2.22 (m, 1H), 2.14 - 2.05 (m, 1H). LCMS (ESI) calcd for C₁₁H₁₂N₂O₃⁺ [M+H]⁺: 221.08; found, 221.0.

### Example II-49: Preparation of 3-(6-Fluoro-5-(hydroxymethyl)pyridin-3-yl)piperidine-2,6-dione (GT-07074)

According to the method of Scheme II-6, the target compound (GT-07074) was prepared (pale yellow solid, 1810 mg, yield 83.27%). ¹HNMR (400 MHz, DMSO-d6) δ 10.91 (s, 1H), 7.98 (s, 1H), 7.85 (dd, J = 9.2, 2.4 Hz, 1H), 5.46 (s, 1H), 4.53 (d, J = 3.6 Hz, 2H), 4.01 (dd, J = 12.4, 4.8 Hz, 1H), 2.71 (ddd, J = 17.6, 12.8, 5.2 Hz, 1H), 2.55 (dt, J = 17.2, 3.6 Hz, 1H), 2.27 (qd, J = 12.8, 4.4 Hz, 1H), 2.07 - 1.98 (m, 1H). LCMS (ESI) calcd for C₁₁H₁₁FN₂O₃⁺ [M+H]⁺: 239.08; found, 239.0.

### Example II-50: Preparation of 3-(5-Fluoro-6-(hydroxymethyl)pyridin-3-yl)piperidine-2,6-dione (GT-07049)

According to the method of Scheme II-6, the target compound (GT-07049) was prepared (green solid, 180 mg). ¹H NMR (400 MHz, DMSO-d6) δ 10.93 (s, 1H), 8.28 (s, 1H), 7.64 (dd, J = 11.2, 1.2 Hz, 1H), 5.29 (t, J = 6.0 Hz, 1H), 4.58 (dd, J = 6.0, 2.0 Hz, 2H), 4.02 (dd, J = 12.8, 4.8 Hz, 1H), 2.77 - 2.64 (m, 1H), 2.58 (d, J = 3.2 Hz, 1H), 2.32 (dd, J = 12.8, 4.4 Hz, 1H), 2.09 - 1.98 (m, 1H). LCMS (ESI) calcd for C₁₁H₁₁FN₂O₃⁺ [M+H]⁺: 239.08; found, 239.0.

### Example II-51: Preparation of 3-(2-Fluoro-6-(hydroxymethyl)pyridin-3-yl)piperidine-2,6-dione (GT-07558)

According to the method of Scheme II-6, the target compound (GT-07558) was prepared (white solid, 405 mg). ¹H NMR (400 MHz, DMSO-d6) δ 10.93 (s, 1H), 7.87 (dd, J = 9.6, 7.4 Hz, 1H), 7.41 - 7.35 (m, 1H), 5.52(t, J = 6.0 Hz, 1H), 4.48 (d, J = 6.0 Hz, 2H), 4.06 (dd, J = 12.8, 4.8 Hz, 1H), 2.75 (ddd, J = 18.4, 13.2, 5.2Hz, 1H), 2.56 (dt, J = 17.2, 3.6 Hz, 1H), 2.23 (dd, J = 13.2, 4.0 Hz, 1H), 2.06 - 1.97 (m, 1H). LCMS (ESI) calcd for C₁₁H₁₁FN₂O₃⁺ [M+H]⁺: 239.08; found, 239.0.

### Example II-52: Preparation of 3-(6-(Hydroxymethyl)pyridazin-3-yl)piperidine-2,6-dione (GT-07636)

According to the method of Scheme II-6, the target compound (GT-07636) was prepared (yellow solid, 296 mg). ¹H NMR (400 MHz, DMSO-d6) δ 11.03 (s, 1H), 7.96 (d, J = 9.2 Hz, 2H), 4.83 (s, 2H), 4.36 (dd,J = 11.2, 5.2 Hz, 1H), 2.79 - 2.67 (m, 1H), 2.58 (dt, J = 17.2, 4.4 Hz, 1H), 2.45 - 2.32 (m, 1H), 2.23 - 2.12 (m, 1H). LCMS (ESI) calcd for C₁₀H₁₁N₃O₃⁺ [M+H]⁺: 222.08; found, 222.0.

### Example II-53: Preparation of 3-(5-Fluoro-4-(hydroxymethyl)pyridin-2-yl)piperidine-2,6-dione (GT-07444)

According to the method of Scheme II-6, the target compound (GT-07444) was prepared (pale yellow solid, 635 mg). ¹H NMR (400 MHz, DMSO-d6) δ 10.91 (s, 1H), 8.51 (s, 1H), 7.60 (d, J = 5.6 Hz, 1H), 4.65 (s, 2H), 4.20(dd, J = 10.4, 5.2 Hz, 1H), 2.64 (ddd, J = 16.0, 10.4, 5.2 Hz, 1H), 2.55 (t, J = 4.8 Hz, 1H), 2.30 (qd, J =10.8, 4.4 Hz, 1H), 2.17 - 2.07 (m, 1H). LCMS (ESI) calcd for C₁₁H₁₁FN₂O₃⁺ [M+H]⁺: 239.09; found, 239.0.

### Example II-54: Preparation of 3-(5-(Hydroxymethyl)pyridin-3-yl)piperidine-2,6-dione (GT-08130)

According to the method of Scheme II-6, the target compound (GT-08130) was prepared. LCMS (ESI): 221.0.

### Example II-55: Preparation of 3-(5-(Chloromethyl)pyridin-3-yl)piperidine-2,6-dione (GT-06991)

According to the method of Scheme II-6, the target compound (GT-06991) was prepared (brown solid, 3090 mg). ¹H NMR (400 MHz, D2O) δ 8.83 (s, 1H), 8.72 (d, J = 1.2 Hz, 1H), 8.60 (s, 1H), 4.82 (s, 2H), 4.30 (dd, J = 12.8, 5.2 Hz, 1H), 2.88 - 2.78 (m, 2H), 2.46 - 2.25 (m, 2H). LCMS (ESI) calcd for C₂₁H₃₀ClN₂⁺ [M+H]⁺: 239.05; found, 239.0.

### Example II-56: Preparation of 3-(5-(Hydroxymethyl)pyridin-2-yl)piperidine-2,6-dione (GT-07075)

According to the method of Scheme II-6, the target compound (GT-07075) was prepared (green solid, 175 mg). ¹H NMR (400 MHz, DMSO-d6) δ 10.87 (s, 1H), 8.47 (d,J = 1.6 Hz, 1H), 7.78 (dd,J = 8.0, 2.0 Hz, 1H), 7.39 (d,J = 8.0 Hz, 1H), 4.53 (s, 2H), 4.05 (dd,J = 9.6, 5.2 Hz, 1H), 2.71 - 2.52 (m, 2H), 2.29 - 2.21 (m, 1H), 2.13 (dt,J = 7.6, 5.2 Hz, 1H). LCMS (ESI) calcd for C₁₁H₁₂N₂O₃⁺ [M+H]⁺: 221.08; found, 221.0.

### Example II-57: Preparation of 3-(3-Fluoro-5-(hydroxymethyl)pyridin-2-yl)piperidine-2,6-dione (GT-07443)

According to the method of Scheme II-6, the target compound (GT-07443) was prepared (white solid, 142 mg). ¹H NMR (400 MHz, DMSO-d6) δ 10.92 (s, 1H), 8.33 (s, 1H), 7.61 (d,J = 10.8 Hz, 1H), 5.44 (t, J = 5.6 Hz, 1H), 4.56 (d, J = 5.6 Hz, 2H), 4.27 (dd, J = 11.2, 5.2 Hz, 1H), 2.73 (ddd, J = 17.2, 11.6, 5.2 Hz, 1H), 2.58 - 2.52 (m, 1H), 2.30 (ddd, J = 16.0, 12.0, 4.4 Hz, 1H), 2.12 - 2.04 (m, 1H). LCMS (ESI) calcd for C₁₁H₁₁FN₂O₃⁺ [M+H]⁺: 239.08; found, 239.0.

### Example II-58: Preparation of 3-(5-(Aminomethyl)pyridin-3-yl)piperidine-2,6-dione (GT-06990)

According to the method of Scheme II-7, the target compound (GT-06990) was prepared (white solid, 45 mg, yield 65.55%). ¹H NMR (400 MHz, MeOD-d4) δ 8.75 (dd, J = 12.5, 1.3 Hz, 2H), 8.27 (s, 1H), 4.32 (s, 2H), 4.17 (dd, J = 12.7, 5.0 Hz, 1H), 2.89 - 2.72 (m, 2H), 2.38 (dd, J = 12.7, 5.0 Hz, 1H), 2.27 (ddd, J = 8.0, 6.7, 4.0 Hz, 1H). LCMS (ESI): calcd for C₁₁H₁₃N₃O₂⁺[M+H]⁺, 220.08; found, 220.1.

### Example II-59: Preparation of (GT-08131)

According to the method of Scheme II-8, the target compound (GT-08131) was prepared. LCMS (ESI): 350.1.

### Example II-60: Preparation of 3-((3-(Hydroxymethyl)pyridin-2-yl)amino)piperidine-2,6-dione (GT-07197)

According to the method of Scheme II-8, the target compound (GT-07197) was prepared (blue solid, 4.2 g, yield 80.23%). ¹H NMR (400 MHz, DMSO-d6) δ 11.12 (s, 1H), 8.04 (s, 1H), 7.98 (d, J = 7.1 Hz, 1H), 7.91 (d, J = 6.1 Hz, 1H), 7.01 (t, J = 6.7 Hz, 1H), 5.31 - 5.14 (m, 1H), 4.52 (s, 2H), 2.85 - 2.72 (m, 1H), 2.66 (d, J = 17.0 Hz, 1H), 2.28 (dd, J = 12.6, 4.5 Hz, 1H), 2.22 - 2.12 (m, 1H).

### Example II-61: Preparation of 2-((2,6-Dioxopiperidin-3-yl)amino)nicotinaldehyde (GT-07447)

According to the method of Scheme II-8, the target compound (GT-07447) was prepared (pale green solid, 1.77 g, yield 51.01 %). ¹H NMR (400 MHz, DMSO-d6) δ 10.93 (s, 1H), 9.89 (s, 1H), 8.74 (d, J = 6.0 Hz, 1H), 8.33 (d, J = 3.0 Hz, 1H), 8.09 (d, J = 6.9 Hz, 1H), 6.95 - 6.69 (m, 1H), 5.14 - 4.90 (m, 1H), 2.90 - 2.73 (m, 1H), 2.56 (s, 1H), 2.35 - 2.24 (m, 1H), 2.07 (dd, J = 22.1, 12.5 Hz, 1H). LCMS (ESI): calcd for C₁₁H₁₁N₃O₃ [M+H]⁺, 234.08; found, 233.9.

### Example II-62: Preparation of 3-((5-(((tert-Butyldimethylsilyl)oxy)methyl)pyrimidin-2-yl)amino)piperidine-2,6-dione (GT-07808)

According to the method of Scheme II-8, the target compound (GT-07808) was prepared (purple solid, 100 mg), ¹H NMR (400 MHz, DMSO-d6) δ 10.69 (s, 1H), 8.17 (s, 2H), 7.28 (d, J = 8.6 Hz, 1H), 4.68 (ddd, J = 12.3, 8.4, 5.0 Hz, 1H), 4.46 (s, 2H), 2.71 (ddd, J = 18.0, 13.5, 5.7 Hz, 1H), 2.54 - 2.46 (m, 1H), 2.05 (qd, J = 13.0, 4.4 Hz, 1H), 1.97 - 1.88 (m, 1H), 0.80 (d, J = 3.2 Hz, 9H), -0.01 (d, J = 4.9 Hz, 6H). LCMS (ESI): calcd for C₁₆H₂₆N₄O₃Si, [M+H]⁺, 351.0; found, 351.0.

### Example II-63: Preparation of 3-((5-(Hydroxymethyl)pyrimidin-2-yl)amino)piperidine-2,6-dione (GT-07809)

According to the method of Scheme II-8, the target compound (GT-07809) was prepared(purple solid, 95 mg). ¹H NMR (400 MHz, DMSO-d6) δ 10.91 (s, 1H), 8.69 - 8.50 (m, 1H), 8.47 (s, 2H), 4.92 (dd, J = 12.2, 5.0 Hz, 1H), 4.39 (s, 2H), 2.80 (ddd, J = 18.3, 13.3, 5.6 Hz, 1H), 2.57 (d, J = 17.3 Hz, 1H), 2.25 - 2.02 (m, 2H). LCMS (ESI): calcd for C₁₀H₁₂N₄O₃, [M+H]⁺, 237.0; found, 237.0.

### Example II-64: Preparation of 2-((2,6-Dioxopiperidin-3-yl)amino)pyrimidine-5-carbaldehyde (GT-07367)

According to the method of Scheme II-8, the target compound (GT-07367) was prepared (pale yellow solid, 60 mg). ¹H NMR (400 MHz, DMSO-d6) δ 10.88 (s, 1H), 9.77 (s, 1H), 8.77 (d, J = 2.2 Hz, 2H), 8.54 (d, J = 8.6 Hz, 1H), 5.07 - 4.91 (m, 1H), 2.89 - 2.77 (m, 1H), 2.58 (s, 1H), 2.17 (dd, J = 13.0, 4.3 Hz, 1H), 2.06 - 2.00 (m, 1H). LCMS (ESI): calcd for C₁₀H₁₀N₄O₃, [M+H]⁺: 235.08; found, 235.2

### Example II-65: Preparation of 3-((5-(((tert-Butyldimethylsilyl)oxy)methyl)pyridin-3-yl)amino)piperidine-2,6-dione (GT-07449)

According to the method of Scheme II-8, the target compound (GT-07449) was prepared (blue solid, 3.5 g). ¹H NMR (400 MHz, DMSO-d6) δ 10.73 (s, 1H), 7.86 (d, J = 2.6 Hz, 1H), 7.67 (s, 1H), 6.87 (s, 1H), 6.07 (d, J = 7.9 Hz, 1H), 4.55 (s, 2H), 4.37 - 4.22 (m, 1H), 2.66 (td, J = 12.3, 6.1 Hz, 1H), 2.51 (dd, J = 13.8, 3.7 Hz, 1H), 2.07 - 1.96 (m, 1H), 1.84 (dd, J = 12.4, 4.4 Hz, 1H), 0.82 (s, 9H), 0.01 (d, J = 9.4 Hz, 6H). LCMS (ESI): calcd for C₁₇H₂₇N₃O₃Si, [M+H]⁺, 350.18; found, 350.1.

### Example II-66: Preparation of 3-((5-(Hydroxymethyl)pyridin-3-yl)amino)piperidine-2,6-dione (GT-07448)

According to the method of Scheme II-8, the target compound (GT-07448) was prepared (blue solid, 2.98 g). ¹H NMR (400 MHz, DMSO-d6) δ 10.93 (s, 1H), 8.10 (d, J = 2.4 Hz, 1H), 7.98 (s, 1H), 7.73 (s, 1H), 7.44 (d, J = 7.8 Hz, 1H), 4.63 (dd, J = 12.2, 7.0 Hz, 1H), 4.58 (s, 2H), 2.74 (ddd, J = 17.8, 12.5, 5.4 Hz, 1H), 2.62 (dd, J = 13.7, 3.3 Hz, 1H), 2.17 - 2.08 (m, 1H), 2.03 (td, J = 12.5, 4.8 Hz, 1H). LCMS (ESI): calcd for C₁₁H₁₃N₃O₃ [M+H]⁺, 236.10; found, 235.9.

### Example II-67: Preparation of 5-((2,6-Dioxopiperidin-3-yl)amino)nicotinaldehyde (GT-07554)

According to the method of Scheme II-8, the target compound (GT-07554) was prepared (brown solid, 440 mg). ¹H NMR (400 MHz, DMSO-d6) δ 10.85 (s, 1H), 9.99 (s, 1H), 8.35 (s, 1H), 7.40 (s, 1H), 6.65 (s, 1H), 4.56 (s, 1H), 3.60 (s, 1H), 2.75 (d, J = 12.0 Hz, 1H), 2.60 (d, J = 18.0 Hz, 1H), 2.10 (s, 1H), 1.97 (d, J = 12.1 Hz, 1H). LCMS (ESI): calcd for C₁₁H₁₁N₃O₃, [M+H]⁺, 234.08; found, 234.0.

### Example II-68: Preparation of 3-((5-(((tert-Butyldimethylsilyl)oxy)methyl)pyridin-2-yl)amino)piperidine-2,6-dione (GT-07552)

According to the method of Scheme II-8, the target compound (GT-07552) was prepared (blue solid, 60 mg). ¹H NMR (400 MHz, DMSO-d6) δ 10.76 (s, 1H), 7.88 (d, J = 2.0 Hz, 1H), 7.35 (dd, J = 8.5, 2.3 Hz, 1H), 6.81 (d, J = 7.8 Hz, 1H), 6.58 (d, J = 8.5 Hz, 1H), 4.77 (ddd, J = 12.8, 7.8, 5.3 Hz, 1H), 4.52 (s, 2H), 2.77 (ddd, J = 18.2, 13.0, 5.6 Hz, 1H), 2.55 (d, J = 3.5 Hz, 1H), 2.13 - 2.05 (m, 1H), 1.99 (td, J = 12.6, 4.5 Hz, 1H), 0.91 - 0.85 (m, 9H), 0.10 - 0.03 (m, 6H). LCMS (ESI): calcd for C₁₇H₂₇N₃O₃Si, [M+H]⁺, 350.18; found, 350.1.

### Example II-69: Preparation of 3-((5-(Hydroxymethyl)pyridin-2-yl)amino)piperidine-2,6-dione (GT-07551)

According to the method of Scheme II-8, the target compound (GT-07551) was prepared (gray solid, 1.0 g). ¹H NMR (400 MHz, DMSO-d6) δ 11.11 (s, 1H), 8.99 (s, 1H), 7.94 (dd, J = 9.2, 1.8 Hz, 1H), 7.85 (s, 1H), 7.19 (d, J = 9.2 Hz, 1H), 5.04 - 4.92 (m, 1H), 4.42 (s, 2H), 2.76 (ddd, J = 18.0, 12.5, 5.7 Hz, 1H), 2.70 - 2.61 (m, 1H), 2.23 - 2.06 (m, 2H). LCMS (ESI): calcd for C₁₁H₁₃N₃O₃, [M+H]⁺, 236.10; found, 235.9.

### Example II-70: Preparation of 3-((4-(((tert-Butyldimethylsilyl)oxy)methyl)pyridin-2-yl)amino)piperidine-2,6-dione (GT-08132)

According to the method of Scheme II-8, the target compound (GT-08132) was prepared. LCMS (ESI): 350.2.

### Example II-71: Preparation of 3-((4-(Hydroxymethyl)pyridin-2-yl)amino)piperidine-2,6-dione (GT-07366)

According to the method of Scheme II-8, the target compound (GT-07366) was prepared (purple solid, 4.2 g). ¹H NMR (400 MHz, MeOD-d4) δ 7.81 (d, J = 6.7 Hz, 1H), 7.20 (s, 1H), 6.92 (d, J = 6.7 Hz, 1H), 4.69 (q, J = 5.4 Hz, 3H), 2.96 - 2.70 (m, 2H), 2.39 - 2.29 (m, 1H), 2.19 (dd, J = 12.2, 6.3 Hz, 1H). LCMS (ESI): calcd for C₁₁H₁₃N₃O₃, [M+H]⁺, 236.10; found, 236.1.

### Example II-72: Preparation of 2-((2,6-Dioxopiperidin-3-yl)amino)isonicotinaldehyde (GT-07553)

According to the method of Scheme II-8, the target compound (GT-07553) was prepared (brown solid, 55 mg). ¹H NMR (400 MHz, DMSO-d6) δ 10.80 (s, 1H), 9.92 (s, 1H), 8.18 (d, J = 5.1 Hz, 1H), 7.37 (d, J = 8.0 Hz, 1H), 7.04 (s, 1H), 6.92 - 6.88 (m, 1H), 4.84 (ddd, J = 11.8, 7.9, 6.0 Hz, 1H), 2.84 - 2.72 (m, 1H), 2.61 - 2.53 (m, 1H), 2.06 (ddd, J = 16.6, 9.6, 3.6 Hz, 2H). LCMS (ESI): calcd for C₁₁H₁₁N₃O₃, [M+H]⁺, 234.08; found, 234.0.

### Example II-73: Preparation of 3-((6-(((tert-Butyldimethylsilyl)oxy)methyl)pyridin-3-yl)amino)piperidine-2,6-dione (GT-07445)

According to the method of Scheme II-8, the target compound (GT-07445) was prepared(purple solid, 1.3 g). ¹H NMR (400 MHz, DMSO-d6) δ 10.80 (s, 1H), 7.96 (d, J = 2.6 Hz, 1H), 7.16 - 7.01 (m, 2H), 6.05 (d, J = 7.9 Hz, 1H), 4.58 (s, 2H), 4.37 (ddd, J = 12.4, 7.9, 4.9 Hz, 1H), 2.81 - 2.53 (m, 2H), 2.17 - 1.81 (m, 2H), 0.89 (s, 9H), 0.06 (s, 6H). LCMS (ESI): calcd for C₁₇H₂₇N₃O₃Si, [M+H]⁺, 350.18; found, 350.3.

### Example II-74: Preparation of 3-((6-(Hydroxymethyl)pyridin-3-yl)amino)piperidine-2,6-dione (GT-07446)

According to the method of Scheme II-8, the target compound (GT-07446) was prepared (purple solid, 850 mg). ¹H NMR (400 MHz, DMSO-d6) δ 10.92 (s, 1H), 8.04 (d, J = 2.6 Hz, 1H), 7.78 (dd, J = 8.9, 2.6 Hz, 1H), 7.68 (d, J = 8.9 Hz, 1H), 7.29 (d, J = 7.1 Hz, 1H), 4.69 (s, 2H), 4.65 - 4.56 (m, 1H), 2.79 - 2.68 (m, 1H), 2.67 - 2.59 (m, 1H), 2.18 - 2.09 (m, 1H), 2.02 (dt, J = 12.5, 6.2 Hz, 1H). LCMS (ESI): calcd for C₁₁H₁₃N₃O₃, [M+H]⁺, 236.0; found, 236.0.

### Example II-75: Preparation of 5-((2,6-Dioxopiperidin-3-yl)amino)picolinaldehyde (GT-07640)

According to the method of Scheme II-8, the target compound (GT-07640) was prepared (brown solid, 100 mg). ¹H NMR (400 MHz, DMSO-d6) δ 10.91 (s, 1H), 9.71 (s, 1H), 8.20 (d, J = 2.6 Hz, 1H), 7.72 (d, J = 8.7 Hz, 1H), 7.26 (d, J = 8.1 Hz, 1H), 7.12 (dd, J = 8.7, 2.6 Hz, 1H), 4.63 (ddd, J = 12.8, 8.1, 5.0 Hz, 1H), 2.76 (s, 1H), 2.63 (s, 1H), 2.18 - 2.09 (m, 1H), 1.99 (s, 1H). LCMS (ESI): calcd for C₁₁H₁₁N₃O₃, [M+H]⁺, 234.0; found, 234.0.

### Example II-76: Preparation of 3-((4-(((tert-Butyldimethylsilyl)oxy)methyl)pyridin-3-yl)amino)piperidine-2,6-dione (GT-07638)

According to the method of Scheme II-8, the target compound (GT-07638) was prepared (purple solid, 1.4 g). ¹H NMR (400 MHz, DMSO-d6) δ 10.79 (s, 1H), 7.95 (s, 1H), 7.80 (d, J = 4.6 Hz, 1H), 7.06 (d, J = 4.6 Hz, 1H), 5.32 (d, J = 6.8 Hz, 1H), 4.59 - 4.40 (m, 3H), 2.72 (ddd, J = 18.0, 13.3, 5.4 Hz, 1H), 2.54 - 2.42 (m, 1H), 2.14 - 2.01 (m, 1H), 1.81 (qd, J = 12.9, 4.4 Hz, 1H), 0.80 (s, 9H), -0.01 (d, J = 9.2 Hz, 6H). LCMS (ESI): calcd for C₁₇H₂₇N₃O₃Si, [M+H]⁺, 350.0; found, 350.1.

### Example II-77: Preparation of 3-((4-(Hydroxymethyl)pyridin-3-yl)amino)piperidine-2,6-dione (GT-07559)

According to the method of Scheme II-8, the target compound (GT-07559) was prepared (purple solid, 422 mg). ¹H NMR (400 MHz, Methanol-d4) δ 8.22 (s, 1H), 8.14 (d, J = 5.7 Hz, 1H), 7.96 (d, J = 5.7 Hz, 1H), 4.78 (d, J = 9.3 Hz, 2H), 4.65 (dd, J = 12.5, 5.2 Hz, 1H), 2.90 - 2.77 (m, 2H), 2.37 - 2.31 (m, 1H), 2.21 (dd, J = 12.8, 5.0 Hz, 1H). LCMS (ESI): calcd for C₁₁H₁₃N₃O₃, [M+H]⁺, 236.0; found, 236.0.

### Example II-78: Preparation of 3-((2,6-Dioxopiperidin-3-yl)amino)isonicotinaldehyde (GT-07804)

According to the method of Scheme II-8, the target compound (GT-07804) was prepared (brown solid, 18 mg). ¹H NMR (400 MHz, DMSO-d6) δ 11.00 (s, 1H), 9.98 (s, 1H), 8.45 (s, 1H), 8.18 (d, J = 7.2 Hz, 1H), 8.06 (d, J = 4.9 Hz, 1H), 7.60 (d, J = 4.9 Hz, 1H), 4.82 (ddd, J = 12.3, 7.0, 5.2 Hz, 1H), 2.86 (ddd, J = 18.3, 13.4, 5.4 Hz, 1H), 2.58 (d, J = 17.9 Hz, 1H), 2.26 (ddd, J = 10.2, 5.0, 3.0 Hz, 1H), 2.12 - 1.98 (m, 1H). LCMS (ESI): calcd for C₁₁H₁₁N₃O₃, [M+H]⁺, 234.0; found, 234.0.

### Example II-79: Preparation of 3-((6-(((tert-Butyldimethylsilyl)oxy)methyl)pyridazin-3-yl)amino)piperidine-2,6-dione (GT-07726)

According to the method of Scheme II-8, the target compound (GT-07726) was prepared (purple solid, 178 mg). ¹H NMR (400 MHz, DMSO-d6) δ 10.82 (s, 1H), 7.31 (d, J = 9.1 Hz, 1H), 7.16 (d, J = 7.7 Hz, 1H), 6.98 (d, J = 9.1 Hz, 1H), 4.88 (td, J = 7.4, 3.8 Hz, 1H), 4.71 (s, 2H), 2.77 (dd, J = 12.9, 5.4 Hz, 1H), 2.61 - 2.53 (m, 1H), 2.21 - 2.02 (m, 2H), 0.88 (s, 9H), 0.07 (s, 6H). LCMS (ESI): calcd for C₁₆H₂₆N₄O₃Si, [M+H]⁺, 351.0; found, 351.0.

### Example II-80: Preparation of 3-((6-(Hydroxymethyl)pyridazin-3-yl)amino)piperidine-2,6-dione (GT-07803)

According to the method of Scheme II-8, the target compound (GT-07803) was prepared (purple solid, 100 mg). ¹H NMR (400 MHz, DMSO-d6) δ 11.10 (s, 1H), 9.42 (s, 1H), 7.94 (d, J = 9.5 Hz, 1H), 7.77 (d, J = 9.4 Hz, 1H), 4.96 (dd, J = 10.6, 6.7 Hz, 1H), 4.60 (s, 2H), 2.77 (ddd, J = 18.1, 11.5, 6.7 Hz, 1H), 2.65 (d, J = 17.5 Hz, 1H), 2.16 (tt, J = 12.4, 5.5 Hz, 2H). LCMS (ESI): calcd for C₁₀H₁₂N₄O₃, [M+H]⁺, 237.0; found, 237.0

### Example II-81: Preparation of 3-((5-(((tert-Butyldimethylsilyl)oxy)methyl)pyrimidin-4-yl)amino)piperidine-2,6-dione (GT-07639)

According to the method of Scheme II-8, the target compound (GT-07639) was prepared (purple solid, 134 mg). ¹H NMR (400 MHz, DMSO-d6) δ 10.90 (s, 1H), 8.42 (s, 1H), 8.13 (s, 1H), 6.93 (d, J = 7.1 Hz, 1H), 4.96 (t, J = 6.8 Hz, 1H), 4.61 (s, 2H), 2.79 (d, J = 13.2 Hz, 1H), 2.54 (dd, J = 16.7, 2.3 Hz, 1H), 2.13 (dd, J = 8.0, 5.6 Hz, 1H), 2.09 - 1.97 (m, 1H), 0.89 (d, J = 1.6 Hz, 9H), 0.10 - 0.07 (m, 6H). LCMS (ESI): calcd for C₁₆H₂₆N₄O₅Si, [M+H]⁺, 351.0; found, 351.0.

### Example II-82: Preparation of 3-((5-(Hydroxymethyl)pyrimidin-4-yl)amino)piperidine-2,6-dione (GT-07727)

According to the method of Scheme II-8, the target compound (GT-07727) was prepared (purple solid, 470 mg). ¹H NMR (400 MHz, DMSO-d6) δ 11.02 (s, 1H), 9.03 (d, J = 7.9 Hz, 1H), 8.85 (s, 1H), 8.25 (s, 1H), 5.21 (ddd, J = 12.9, 7.8, 5.4 Hz, 1H), 4.48 (s, 2H), 2.84 (ddd, J = 18.8, 13.8, 5.4 Hz, 1H), 2.58 (d, J = 17.1 Hz, 1H), 2.33 (qd, J = 13.0, 4.4 Hz, 1H), 2.12 - 1.94 (m, 1H). LCMS (ESI): calcd for C₁₀H₁₂N₄O₃, [M+H]⁺, 237.0; found, 237.0.

### Example II-83: Preparation of 4-((2,6-Dioxopiperidin-3-yl)amino)pyrimidine-5-carbaldehyde (GT-07805)

According to the method of Scheme II-8, the target compound (GT-07805) was prepared (brown solid, 209 mg). ¹H NMR (400 MHz, DMSO-d6) δ 10.98 (s, 1H), 9.92 (s, 1H), 8.95 (d, J = 7.1 Hz, 1H), 8.82 (s, 1H), 8.69 (s, 1H), 5.09 (d, J = 9.5 Hz, 1H), 2.80 (d, J = 8.9 Hz, 1H), 2.56 (s, 1H), 2.21 (d, J = 6.5 Hz, 2H). LCMS (ESI): calcd for C₁₀H₁₀N₄O₃, [M+H]⁺, 235.0; found, 235.0.

### Example II-84: Preparation of 3-((4-(((tert-Butyldimethylsilyl)oxy)methyl)-5-fluoropyridin-2-yl)amino)piperidine-2,6-dione (GT-07724)

According to the method of Scheme II-8, the target compound (GT-07724) was prepared (white solid, 500 mg). ¹H NMR (400 MHz, DMSO-d6) δ 10.63 (s, 1H), 7.71 (d, J = 1.8 Hz, 1H), 6.87 (d, J = 7.9 Hz, 1H), 6.56 (d, J = 5.0 Hz, 1H), 4.61 (dd, J = 7.3, 4.8 Hz, 1H), 4.57 (s, 2H), 2.64 (ddd, J = 18.2, 12.8, 5.7 Hz, 1H), 2.47 - 2.40 (m, 1H), 1.95 (dtd, J = 13.0, 7.6, 6.6, 3.9 Hz, 1H), 1.85 (td, J = 12.6, 4.5 Hz, 1H), 0.81 (s, 9H), 0.00 (s, 6H). LCMS (ESI): calcd for C₁₇H₂₆FN₃O₃Si, [M+H]⁺, 368.5; found, 368.1.

### Example II-85: Preparation of 3-((5-Fluoro-4-(hydroxymethyl)pyridin-2-yl)amino)piperidine-2,6-dione (GT-07725)

According to the method of Scheme II-8, the target compound (GT-07725) was prepared (white solid, 340 mg). ¹H NMR (400 MHz, DMSO-d6) δ 11.02 (s, 1H), 8.66 (s, 1H), 8.05 (d, J = 3.4 Hz, 1H), 7.15 (d, J = 5.1 Hz, 1H), 4.94 (dd, J = 12.0, 5.4 Hz, 1H), 4.61 (s, 2H), 2.76 (ddd, J = 18.1, 12.6, 5.8 Hz, 1H), 2.63 (d, J = 17.4 Hz, 1H), 2.13 (td, J = 12.3, 11.2, 5.7 Hz, 2H). LCMS (ESI): calcd for C₁₁H₁₂FN₃O₃, [M+H]⁺, 254.1; found, 254.1.

### Example 1: Preparation of 3-(4-((4-(4-amino-3-(4-phenoxyphenyl)-1H-pyrazolo[3,4-d]pyrimidin-1-yl)piperidin-1-yl)methyl)phenyl)piperidine-2,6-dione (GT-03507)

According to the method of step 2 of Scheme I-1, the target compound (GT-03507) was prepared (white solid, 110 mg, yield 62%). ¹H NMR (400 MHz, MeOD) δ 8.43 (s, 1H), 7.67 (d, *J =* 8.6 Hz, 2H), 7.58 (d, *J* = 8.1 Hz, 2H), 7.47 - 7.39 (m, 4H), 7.25 - 7.07 (m, 5H), 5.29 - 5.16 (m, 1H), 4.43 (s, 2H), 3.97 (dd, *J =* 11.6, 5.0 Hz, 1H), 3.75 - 3.71 (m, 2H), 3.43 - 3.34 (m, 2H), 2.82 - 2.54 (m, 4H), 2.41 - 2.11 (m, 4H). LCMS (ESI) calcd for C₃₄H₃₄N₇O₃⁺ [M+H]⁺: 588.27; found, 588.3.

### Example 2: Preparation of 3-(4-((4-(4-amino-3-(4-phenoxyphenyl)-1H-pyrazolo[3,4-d]pyrimidin-1-yl)piperidin-1-yl)methyl)-2-fluorophenyl)piperidine-2,6-dione (GT-03508)

According to the method of step 2 of Scheme I-1, the target compound (GT-03508) was prepared (white solid, 80 mg, yield 46%). ¹H NMR (400 MHz, MeOD) δ 8.45 (s, 1H), 7.68 (d, *J =* 8.5 Hz, 2H), 7.48 - 7.39 (m, 5H), 7.21 - 7.05 (m, 5H), 5.31 - 5.20 (m, 1H), 4.45 (s, 2H), 4.14 (dd, *J =* 12.5, 4.8 Hz, 1H), 3.78 - 3.68 (m, 2H), 3.45 - 3.34 (m, 2H), 2.80 - 2.58 (m, 4H), 2.43 - 2.27 (m, 3H), 2.19 - 2.10 (m, 1H). LCMS (ESI) calcd for C₃₈H₃₈N₃O₆⁺ [M+H]⁺: 632.28; found, 632.3.

### Example 3: Preparation of 6-(4-(4-(4-(2,6-dioxopiperidin-3-yl)benzyl)piperazin-1-yl)piperidin-1-yl)-2-(4-phenoxyphenyl)nicotinamide (GT-03753)

According to the method of step 2 of Scheme I-1, the target compound (GT-03753) was prepared (white solid, 36 mg, yield 58%). ¹H NMR (400 MHz, MeOD) δ 7.86 (d, *J =* 8.9 Hz, 1H), 7.68 - 7.62 (m, 2H), 7.56 (d, *J =* 8.1 Hz, 2H), 7.43 - 7.35 (m, 4H), 7.17 (t, *J =* 7.4 Hz, 1H), 7.08 - 6.98 (m, 5H), 4.70 - 4.57 (m, 2H), 4.34 (s, 2H), 3.94 (dd, *J =* 11.4, 5.1 Hz, 1H), 3.88 - 3.34 (m, 9H), 3.19 - 3.04 (m, 2H), 2.83 - 2.60 (m, 2H), 2.37 - 2.13 (m, 4H), 1.87 - 1.73 (m, 2H). LCMS (ESI) m/z: calcd for C₃₉H₄₃N₆O₄⁺[M+H]⁺, 659.33; found, 659.3.

### Example 4: Preparation of 6-(4-(4-(4-(2,6-dioxopiperidin-3-yl)-3-fluorobenzyl)piperazin-1-yl)piperidin-1-yl)-2-(4-phenoxyphenyl)nicotinamide (GT-03754)

According to the method of step 2 of Scheme I-1, the target compound (GT-03754) was prepared (white solid, 36 mg, yield 55%). ¹H NMR (400 MHz, DMSO) δ 10.91 (s, 1H), 7.67 - 7.64 (m, 2H), 7.64 - 7.61 (m, 2H), 7.54 (s, 1H), 7.48 - 7.36 (m, 4H), 7.23 (s, 1H), 7.18 (t, *J =* 7.4 Hz, 1H), 7.10 - 7.04 (m, 2H), 7.01 (d, *J* = 8.7 Hz, 2H), 6.88 (d, *J =* 8.7 Hz, 1H), 4.59 (d, *J =* 13.0 Hz, 2H), 4.09 (dd, *J =* 12.5, 4.5 Hz, 1H), 3.74 - 3.51 (m, 9H), 2.93 - 2.84 (m, 2H), 2.80 - 2.69 (m, 1H), 2.58 - 2.55 (m, 1H), 2.25 - 2.10 (m, 3H), 2.04 - 1.92 (m, 1H), 1.70 - 1.53 (m, 2H). LCMS (ESI) m/z: calcd for C₃₉H₄₂FN₆O₄⁺[M+H]⁺, 677.32; found, 677.3.

### Example 5: Preparation of 6-(4-((4-(2,6-dioxopiperidin-3-yl)benzyl)(methyl)amino)piperidin-1-yl)-2-(4-phenoxyphenyl)nicotinamide (GT-03828)

According to the method of step 2 of Scheme I-1, the target compound (GT-03828) was prepared (white solid, 40 mg, yield 49%). ¹H NMR (400 MHz, DMSO) δ 10.87 (s, 1H), 10.40 (s, 1H), 7.68 - 7.62 (m, 3H), 7.61 - 7.50 (m, 3H), 7.47 - 7.40 (m, 2H), 7.32 (d, *J =* 8.1 Hz, 2H), 7.23 (s, 1H), 7.18 (t, *J =* 7.4 Hz, 1H), 7.07 (d, *J* = 7.7 Hz, 2H), 7.02 (d, *J =* 8.7 Hz, 2H), 6.89 (d, *J =* 8.8 Hz, 1H), 4.70 - 4.57 (m, 2H), 4.47 (d, *J =* 10.9 Hz, 1H), 4.21 - 4.12 (m, 1H), 3.92 (dd, *J =* 11.7, 4.8 Hz, 1H), 3.61 - 3.47 (m, 1H), 2.96 - 2.80 (m, 2H), 2.74 - 2.63 (m, 1H), 2.57 (d, *J =* 4.8 Hz, 3H), 2.55 - 2.52 (m, 1H), 2.30 - 2.14 (m, 3H), 2.09 - 1.97 (m, 1H), 1.81 - 1.67 (m, 2H). LCMS (ESI) m/z: calcd for C₃₆H₃₈N₅O₄⁺[M+H]⁺, 604.29; found, 604.3.

### Example 6: Preparation of 6-(4-((4-(2,6-dioxopiperidin-3-yl)-3-fluorobenzyl)(methyl)amino)piperidin-1-yl)-2-(4-phenoxyphenyl)nicotinamide (GT-03829)

According to the method of step 2 of Scheme I-1, the target compound (GT-03829) was prepared (white solid, 45 mg, yield 54%), ¹H NMR (400 MHz, DMSO) δ 10.96 - 10.68 (m, 2H), 7.65 (dd, *J* = 8.7, 1.9 Hz, 3H), 7.62 - 7.53 (m, 2H), 7.48 - 7.38 (m, 4H), 7.22 (s, 1H), 7.18 (t, *J* = 7.4 Hz, 1H), 7.07 (d, *J =* 7.7 Hz, 2H), 7.02 (d, *J =* 8.7 Hz, 2H), 6.89 (d, *J =* 8.8 Hz, 1H), 4.62 (d, *J =* 12.2 Hz, 2H), 4.49 (d, *J =* 13.3 Hz, 1H), 4.13 - 4.07 (m, 2H), 3.60 - 3.48 (m, 1H), 2.96 - 2.84 (m, 2H), 2.81 - 2.69 (m, 1H), 2.57 (s, 3H), 2.37 - 2.16 (m, 3H), 2.07 - 1.95 (m, 1H), 1.85 - 1.66 (m, 2H). LCMS (ESI) m/z: calcd for C₃₆H₃₇FN₅O₄⁺ [M+H]⁺, 622.28; found, 622.3.

### Example 7: Preparation of 3-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)phenyl)piperidine-2,6-dione (GT-03603)

According to the method of step 2 of Scheme I-1, the target compound (GT-03603) was prepared (white solid, 27 mg, yield 36%). ¹H NMR (400 MHz, MeOD) δ 8.32 (s, 1H), 8.12 (s, 1H), 7.71 (dd, *J =* 14.9, 7.3 Hz, 1H), 7.66 - 7.54 (m, 3H), 7.50 - 7.38 (m, 4H), 7.04 (s, 1H), 6.84 (d, *J =* 8.8 Hz, 1H), 4.63 (d, *J =* 13.1 Hz, 1H), 4.29 (d, *J =* 12.7 Hz, 1H), 3.97 (dd, *J =* 11.4, 5.0 Hz, 3H), 3.91 (s, 3H), 3.76 - 3.67 (m, 1H), 3.27 - 3.16 (m, 2H), 2.83 (s, 3H), 2.78 - 2.63 (m, 2H), 2.43 - 2.35 (m, 2H), 2.33 - 2.17 (m, 4H), 1.90 (s, 3H), 1.87 (s, 3H). LCMS (ESI) m/z: calcd for C₃₇H₄₄ClN₇O₄P⁺[M+H]⁺, 716.29; found, 716.3.

### Example 8: Preparation of 3-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-2-fluorophenyl)piperidine-2,6-dione (GT-03604)

According to the method of step 2 of Scheme I-1, the target compound (GT-03604) was prepared (white solid, 41 mg, yield 54%). ¹H NMR (400 MHz, MeOD) δ8.28 (s, 1H), 8.13 (s, 1H), 7.71 (dd, *J =* 14.0, 7.9 Hz, 1H), 7.62 (t, *J* = 7.5 Hz, 1H), 7.52 - 7.41 (m, 5H), 7.08 (s, 1H), 6.87 (d, *J =* 7.5 Hz, 1H), 4.69 - 4.59 (m, 1H), 4.32 (d, *J =* 14.2 Hz, 1H), 4.15 (dd, *J =* 12.5, 5.0 Hz, 1H), 4.02 - 3.94 (m, 2H), 3.92 (s, 3H), 3.81 - 3.69 (m, 1H), 3.29 - 3.18 (m, 2H), 2.84 (s, 3H), 2.82 - 2.68 (m, 2H), 2.44 - 2.25 (m, 5H), 2.22 - 2.13 (m, 1H), 1.90 (s, 3H), 1.87 (s, 3H). LCMS (ESI) m/z: calcd for C₃₇H₄₃ClFN₇O₄P⁺[M+H]⁺, 734.28; found, 734.3.

### Example 9: Preparation of 8-(4-(4-(4-(2,6-dioxopiperidin-3-yl)benzyl)piperazin-1-yl)piperidin-1-yl)-9-ethyl-6,6-dimethyl-11-oxo-6,11-dihydro-5H-benzo[b]carbazole-3-carbonitrile (GT-03762)

According to the method of step 2 of Scheme I-1, the target compound (GT-03762) was prepared (white solid, 25 mg, yield 33%). ¹H NMR (400 MHz, DMSO) δ 12.84 (s, 1H), 10.87 (s, 1H), 8.32 (d, *J =* 8.1 Hz, 1H), 8.06 (s, 1H), 8.00 (s, 1H), 7.66 - 7.51 (m, 3H), 7.41 - 7.28 (m, 3H), 4.45 - 4.24 (m, 1H), 3.93 (dd, *J =* 11.4, 4.9 Hz, 1H), 3.81 - 3.70 (m, 2H), 3.45 - 3.27 (m, 8H), 2.88 - 2.78 (m, 2H), 2.76 - 2.64 (m, 3H), 2.56 - 2.53 (m, 1H), 2.27 - 2.15 (m, 3H), 2.09 - 2.01 (m, 1H), 1.96 - 1.84 (m, 2H), 1.76 (s, 6H), 1.28 (t, *J =* 7.5 Hz, 3H). LCMS (ESI) m/z: calcd for C₄₂H₄₇N₆O₃⁺[M+H]⁺, 683.37; found, 683.4.

### Example 10: Preparation of 8-(4-(4-(4-(2,6-dioxopiperidin-3-yl)-3-fluorobenzyl)piperazin-1-yl)piperidin-1-yl)-9-ethyl-6,6-dimethyl-11-oxo-6,11-dihydro-5H-benzo[b]carbazole-3-carbonitrile (GT-03763)

According to the method of step 2 of Scheme I-1, the target compound (GT-03763) was prepared (white solid, 36 mg, yield 45%), ¹H NMR (400 MHz, MeOD) δ 8.41 (d, *J =* 8.2 Hz, 1H), 8.20 (s, 1H), 7.87 (s, 1H), 7.55 (dd, *J =* 8.2, 1.4 Hz, 1H), 7.49 - 7.36 (m, 4H), 4.32 (s, 2H), 4.12 (dd, *J =* 12.5, 5.1 Hz, 1H), 3.96 - 3.36 (m, 11H), 2.95 (t, *J =* 11.9 Hz, 2H), 2.86 - 2.66 (m, 4H), 2.39 - 2.28 (m, 3H), 2.22 - 2.12 (m, 1H), 2.10 - 2.00 (m, 2H), 1.80 (s, 6H), 1.34 (t, *J =* 7.5 Hz, 3H). LCMS (ESI) m/z: calcd for C₄₂H₄₆FN₆O₃⁺[M+H]⁺, 701.36; found, 701.4.

### Example 11: Preparation of compounds (GT-03789) and (GT-03799)

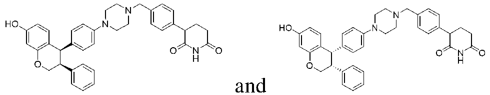

According to the method described in Step 2 of Scheme I-1, the target compounds were prepared using either the compounds (GT-M-173_P1) or (GT-M-173_P2) obtained from Intermediate Example 90 and 3-(4-(bromomethyl)phenyl)piperidine-2,6-dione as starting materials.

The compound (GT-M-173_P1) obtained from Intermediate Example 90 was used to prepare the target compound (GT-03789) as a white solid (25 mg, yield 32.88%). ¹H NMR (400 MHz, DMSO) δ 11.23 (s, 1H), 10.87 (s, 1H), 7.60 (d, J = 7.8 Hz, 2H), 7.31 (d, J = 7.8 Hz, 2H), 7.14 (s, 3H), 6.77 (s, 2H), 6.64 (dd, J = 8.0, 4.3 Hz, 3H), 6.42 (d, J = 8.2 Hz, 2H), 6.33 - 6.25 (m, 2H), 4.30 (d, J = 11.8 Hz, 3H), 4.20 (t, J = 5.8 Hz, 2H), 3.92 (dd, J = 11.7, 4.7 Hz, 1H), 3.65 (d, J = 8.9 Hz, 3H), 3.26 (d, J = 27.2 Hz, 3H), 3.13 - 3.02 (m, 4H), 2.72 - 2.63 (m, 1H), 2.24 (dd, J = 15.6, 12.2 Hz, 1H), 2.03 (dd, J = 12.9, 4.5 Hz, 1H). LCMS (ESI) m/z: calcd for C₃₇H₃₈N₃O₄⁺[M+H]⁺, 588.28; found, 588.40.

The compound (GT-M-173_P2) obtained from Intermediate Example 90 was used to prepare the target compound (GT-03799) as a white solid (37 mg, yield 41%). ¹H NMR (400 MHz, MeOD) δ 7.55 (d, *J =* 8.0 Hz, 2H), 7.42 (d, *J=* 8.0 Hz, 2H), 7.13 - 7.09 (m, 3H), 6.73 - 6.63 (m, 5H), 6.54 (d, *J =* 8.4 Hz, 2H), 6.35 (s, 1H), 6.30 (dd, *J =* 8.3, 2.3 Hz, 1H), 4.95 - 4.88 (m, 1H), 4.44 - 4.37 (m, 3H), 4.23 (d, *J=* 5.1 Hz, 1H), 4.21 - 4.13 (m, 1H), 3.95 (dd, *J =* 11.5, 5.1 Hz, 1H), 3.80 - 3.65 (m, 2H), 3.62 - 3.47 (m, 3H), 3.29 - 3.20 (m, 2H), 3.06 - 2.91 (m, 2H), 2.82 - 2.61 (m, 2H), 2.37 - 2.15 (m, 2H). LCMS (ESI) m/z: calcd for C₃₇H₃₈N₃O₄⁺[M+H]⁺, 588.29; found, 588.3.

### Example 12: Preparation of compounds (GT-03790) and (GT-03800)

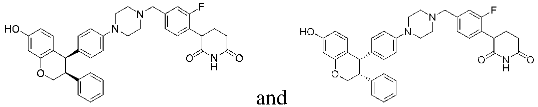

According to the method described in Step 2 of Scheme I-1, the target compounds were prepared using either the compounds (GT-M-173_P1) or (GT-M-173_P2) obtained from Intermediate Example 90 and 3-(4-(bromomethyl)-2-fluorophenyl)piperidine-2,6-dione as starting materials.

The compound (GT-M-173_P1) obtained from Intermediate Example 90 was used to prepare the target compound (GT-03790) as a white solid (27 mg, yield 34.46%). ¹H NMR (400 MHz, DMSO) δ 11.39 (s, 1H), 10.92 (s, 1H), 7.58 (d, J = 11.1 Hz, 1H), 7.49 - 7.36 (m, 2H), 7.18 - 7.10 (m, 3H), 6.83 - 6.73 (m, 2H), 6.65 (dd, J = 8.3, 5.2 Hz, 3H), 6.43 (d, J = 8.5 Hz, 2H), 6.34 - 6.21 (m, 2H), 4.40 - 4.27 (m, 3H), 4.19 (dd, J = 10.6, 3.4 Hz, 2H), 4.11 (dd, J = 12.6, 4.8 Hz, 1H), 3.66 (d, J = 8.2 Hz, 2H), 3.54 - 3.50 (m, 2H), 3.31 (s, 2H), 3.07 (d, J = 8.0 Hz, 4H), 2.82 - 2.69 (m, 1H), 2.58 - 2.52 (m, 1H), 2.31 - 2.16 (m, 1H), 2.08 - 1.93 (m, 1H). LCMS (ESI) m/z: calcd for C₃₇H₃₇FN₃O₄⁺[M+H]⁺, 606.27; found, 606.30.

The compound (GT-M-173_P1) obtained from Intermediate Example 90 was used to prepare the target compound (GT-03800) as a white solid (40 mg, yield 47%). ¹H NMR (400 MHz, MeOD) δ 7.48 (t, *J =* 7.7 Hz, 1H), 7.43 - 7.36 (m, 2H), 7.16 - 7.07 (m, 3H), 6.76 - 6.66 (m, 5H), 6.54 (d, *J =* 8.6 Hz, 2H), 6.35 (d, *J* = 2.3 Hz, 1H), 6.30 (dd, *J =* 8.3, 2.4 Hz, 1H), 4.93 - 4.89 (m, 1H), 4.43 - 4.37 (m, 3H), 4.23 (d, *J* = 5.2 Hz, 1H), 4.19 (dd, *J =* 10.6, 2.8 Hz, 1H), 4.13 (dd, *J =* 12.5, 5.0 Hz, 1H), 3.83 - 3.67 (m, 2H), 3.61 - 3.51 (m, 3H), 3.29 - 3.21 (m, 1H), 3.07 - 2.93 (m, 2H), 2.86 - 2.67 (m, 2H), 2.34 (qd, *J =* 13.0, 4.7 Hz, 1H), 2.20 - 2.11 (m, 1H). LCMS (ESI) m/z: calcd for C₃₇H₃₇FN₃O₄⁺[M+H]⁺, 606.28; found, 606.3.

### Example 13: Preparation of 2-((2-((4-(4-(4-(4-(2,6-dioxopiperidin-3-yl)benzyl)piperazin-1-yl)piperidin-1-yl)-2-methoxyphenyl)amino)-5-(trifluoromethyl)pyridin-4-yl)amino)-N-methylbenzamide (GT-03599)

According to the method of step 2 of Scheme I-1, the target compound (GT-03599) was prepared (white solid, 19 mg, yield 32%). ¹H NMR (400 MHz, MeOD) δ8.04 (s, 1H), 7.73 (d, *J =* 7.5 Hz, 1H), 7.66 - 7.57 (m, 4H), 7.47 - 7.41 (m, 2H), 7.39 - 7.34 (m, 1H), 7.29 - 7.23 (m, 1H), 7.09 (s, 1H), 6.95 (d, *J =* 7.4 Hz, 1H), 6.49 (s, 1H), 4.52 (s, 2H), 4.00 - 3.93 (m, 3H), 3.90 (s, 3H), 3.85 - 3.62 (m, 9H), 3.30 - 3.23 (m, 2H), 2.89 (s, 3H), 2.82 - 2.61 (m, 2H), 2.45 - 2.37 (m, 2H), 2.36 - 2.14 (m, 4H). LCMS (ESI) calcd for C₄₂H₄₈F₃N₈O₄⁺ [M+H]⁺: 785.37; found, 785.4.

### Example 14: Preparation of 2-((2-((4-(4-(4-(4-(2,6-dioxopiperidin-3-yl)-3-fluorobenzyl)piperazin-1-yl)piperidin-1-yl)-2-methoxyphenyl)amino)-5-(trifluoromethyl)pyridin-4-yl)amino)-N-methylbenzamide (GT-03600)

According to the method of step 2 of Scheme I-1, the target compound (GT-03600) was prepared (white solid, 28 mg, yield 47%). ¹H NMR (400 MHz, MeOD) δ8.02 (s, 1H), 7.72 (d, *J =* 7.7 Hz, 1H), 7.64 - 7.55 (m, 2H), 7.51 - 7.41 (m, 3H), 7.40 - 7.32 (m, 1H), 7.27 - 7.18 (m, 1H), 6.98 (s, 1H), 6.86 (d, *J =* 7.4 Hz, 1H), 6.48 (s, 1H), 4.45 (s, 2H), 4.12 (dd, *J =* 12.5, 5.1 Hz, 1H), 4.00 - 3.93 (m, 2H), 3.88 (s, 3H), 3.80 - 3.52 (m, 9H), 3.20 - 3.10 (m, 2H), 2.89 (s, 3H), 2.82 - 2.68 (m, 2H), 2.44 - 2.31 (m, 3H), 2.20 - 2.04 (m, 3H). LCMS (ESI) calcd for C₄₂H₄₇F₄N₈O₄⁺ [M+H]⁺: 803.37; found, 803.4.

### Example 15: Preparation of N-(4-((4-(4-(2,6-dioxopiperidin-3-yl)benzyl)piperazin-1-yl)methyl)-3-(trifluoromethyl)phenyl)-3-(imidazo[1,2-b]pyridazin-3-ylethynyl)-4-methylbenzamide (GT-03936)

According to the method of step 2 of Scheme I-1, the target compound (GT-03936) was prepared (white solid, 35 mg, yield 47%). ¹H NMR (400 MHz, DMSO) δ 10.86 (s, 1H), 10.69 (s, 1H), 8.75 (dd, *J =* 4.4, 1.5 Hz, 1H), 8.35 - 8.27 (m, 3H), 8.23 (d, *J =* 1.6 Hz, 1H), 8.16 (d, *J =* 8.1 Hz, 1H), 8.01 - 7.95 (m, 1H), 7.64 - 7.53 (m, 3H), 7.43 (dd, *J* = 9.2, 4.5 Hz, 1H), 7.32 (d, *J =* 8.1 Hz, 2H), 4.33 (s, 2H), 3.94 - 3.87 (m, 3H), 3.49 - 3.04 (m, 6H), 2.74 - 2.65 (m, 2H), 2.62 (s, 3H), 2.55 - 2.52 (m, 2H), 2.26 - 2.17 (m, 1H), 2.08 - 1.99 (m, 1H). LCMS (ESI) m/z: calcd for C₄₀H₃₇F₃N₇O₃⁺ [M+H]⁺, 720.29; found, 720.3.

### Example 16: Preparation of N-(4-((4-(4-(2,6-dioxopiperidin-3-yl)-3-fluorobenzyl)piperazin-1-yl)methyl)-3-(trifluoromethyl)phenyl)-3-(imidazo[1,2-b]pyridazin-3-ylethynyl)-4-methylbenzamide (GT-03937)

According to the method of step 2 of Scheme I-1, the target compound (GT-03937) was prepared (white solid, 30 mg, yield 40%). ¹H NMR (400 MHz, DMSO) δ 10.92 (s, 1H), 10.65 (s, 1H), 8.28 (dd, *J =* 9.2, 1.5 Hz, 1H), 8.26 (s, 2H), 8.23 (d, *J =* 1.6 Hz, 1H), 8.15 (d, *J =* 8.1 Hz, 1H), 7.97 (dd, *J =* 7.9, 1.8 Hz, 1H), 7.57 (d, J= 8.2 Hz, 1H), 7.50 (d, *J =* 10.1 Hz, 1H), 7.45 - 7.37 (m, 3H), 4.38 - 4.30 (m, 3H), 4.11 (dd, *J =* 12.5, 4.9 Hz, 2H), 3.93 - 3.65 (m, 2H), 3.40 - 2.86 (m, 6H), 2.81 - 2.71 (m, 1H), 2.62 (s, 3H), 2.59 - 2.55 (m, 1H), 2.28 - 2.16 (m, 1H), 2.05 - 1.96 (m, 1H). LCMS (ESI) m/z: calcd for C₄₀H₃₆F₄N₇O₃⁺ [M+H]⁺, 738.28; found, 738.3.

### Example 17: Preparation of N-(3-(difluoromethyl)-1-(1-(4-(2,6-dioxopiperidin-3-yl)benzyl)piperidin-4-yl)-1H-pyrazol-4-yl)-5-morpholinopyrazolo[1,5-a]pyrimidine-3-carboxamide (GT-03813)

According to the method of step 2 of Scheme I-1, the target compound (GT-03813) was prepared (white solid, 30 mg, yield 38%). ¹H NMR (400 MHz, MeOD) δ 8.56 (d, *J =* 7.9 Hz, 1H), 8.41 (s, 1H), 8.32 (s, 1H), 7.54 (d, *J =* 8.0 Hz, 2H), 7.44 (d, *J =* 8.1 Hz, 2H), 7.01 - 6.74 (m, 1H), 6.82 (d, *J* = 7.8 Hz, 1H), 4.45 - 4.38 (m, 2H), 4.01 - 3.92 (m, 1H), 3.92 - 3.78 (m, 9H), 3.74 - 3.63 (m, 2H), 3.52 - 3.45 (m, 1H), 3.26 - 3.21 (m, 1H), 2.81 - 2.63 (m, 2H), 2.46 - 2.30 (m, 6H). LCMS (ESI) m/z: calcd for C₃₂H₃₆F₂N₉O₄⁺ [M+H]⁺, 648.29; found, 648.3.

### Example 18: Preparation of N-(3-(difluoromethyl)-1-(1-(4-(2,4-dioxotetrahydropyrimidin-1(2H)-yl)-3-fluorobenzyl)piperidin-4-yl)-1H-pyrazol-4-yl)-5-morpholinopyrazolo[1,5-a]pyrimidine-3-carboxamide (GT-03814)

According to the method of step 2 of Scheme I-1, the target compound (GT-03814) was prepared (white solid, 41 mg, yield 51%). ¹H NMR (400 MHz, DMSO) δ 10.93 (s, 1H), 10.67 (s, 1H), 9.42 (s, 1H), 8.83 (d, *J =* 7.9 Hz, 1H), 8.42 (s, 1H), 8.29 (s, 1H), 7.54 (d, *J =* 10.6 Hz, 1H), 7.48 - 7.39 (m, 2H), 7.13 (t, *J* = 53.6 Hz, 1H), 6.91 (d, *J =* 7.9 Hz, 1H), 4.62 - 4.52 (m, 1H), 4.39 - 4.30 (m, 2H), 4.12 (dd, *J =* 12.6, 4.7 Hz, 1H), 3.84 - 3.64 (m, 10H), 3.57 - 3.44 (m, 2H), 3.21 - 3.08 (m, 2H), 2.82 - 2.71 (m, 1H), 2.60 - 2.53 (m, 1H), 2.37 - 2.23 (m, 5H), 2.07 - 1.96 (m, 1H). LCMS (ESI) m/z: calcd for C₃₂H₃₅F₃N₉O₄⁺[M+H]⁺, 666.28; found, 666.3.

### Example 19: Preparation of N-(5-(3,5-difluorobenzyl)-1H-indazol-3-yl)-4-(4-((4-(2,6-dioxopiperidin-3-yl)benzyl)(methyl)amino)piperidin-1-yl)-2-((tetrahydro-2H-pyran-4-yl)amino)benzamide (GT-03964)

According to the method of step 2 of Scheme I-1, the target compound (GT-03964) was prepared (white solid, 21 mg, yield 29%). ¹H NMR (400 MHz, DMSO) δ 12.67 (s, 1H), 10.84 (d, *J =* 32.3 Hz, 1H), 10.13 (s, 1H), 10.00 (s, 1H), 7.83 (d, *J =* 9.1 Hz, 1H), 7.56 (d, *J =* 8.1 Hz, 2H), 7.47 (s, 1H), 7.41 (d, *J =* 8.6 Hz, 1H), 7.35 (d, *J =* 8.1 Hz, 2H), 7.26 (d, *J =* 8.4 Hz, 1H), 7.05 - 6.91 (m, 3H), 6.31 (d, *J =* 9.2 Hz, 1H), 6.20 (s, 1H), 4.51 (d, *J =* 13.8 Hz, 1H), 4.21 - 4.08 (m, 2H), 4.04 (s, 3H), 3.93 (dd, *J =* 11.6, 4.7 Hz, 1H), 3.86 - 3.79 (m, 2H), 3.75 - 3.64 (m, 2H), 3.51 - 3.47 (m, 3H), 2.85 - 2.77 (m, 2H), 2.75 - 2.66 (m, 1H), 2.62 (d, *J* = 4.7 Hz, 3H), 2.38 - 2.14 (m, 4H), 2.10 - 1.98 (m, 1H), 1.98 - 1.91 (m, 2H), 1.91 - 1.75 (m, 2H), 1.44 - 1.29 (m, 2H). LCMS (ESI) m/z: calcd for C₄₄H₄₈F₂N₇O₄⁺[M+H]⁺, 776.37; found, 776.4.

### Example 20: Preparation of N-(5-(3,5-difluorobenzyl)-1H-indazol-3-yl)-4-(4-((4-(2,6-dioxopiperidin-3-yl)-3-fluorobenzyl)(methyl)amino)piperidin-1-yl)-2-((tetrahydro-2H-pyran-4-yl)amino)benzamide (GT-03965)

According to the method of step 2 of Scheme I-1, the target compound (GT-03965) was prepared (white solid, 27 mg, yield 37%). ¹H NMR (400 MHz, DMSO) δ 12.67 (s, 1H), 10.92 (s, 1H), 10.49 (s, 1H), 10.14 (s, 1H), 7.83 (d, *J =* 9.1 Hz, 1H), 7.57 (d, *J* = 11.0 Hz, 1H), 7.53 - 7.37 (m, 4H), 7.26 (dd, *J* = 8.6, 1.5 Hz, 1H), 7.05 - 6.94 (m, 3H), 6.33 (d, *J* = 8.7 Hz, 1H), 6.22 (s, 1H), 4.52 (d, *J* = 10.7 Hz, 1H), 4.27 - 4.02 (m, 6H), 3.88 - 3.80 (m, 3H), 3.55 - 3.45 (m, 4H), 2.87 - 2.71 (m, 3H), 2.68 - 2.58 (m, 4H), 2.35 - 2.16 (m, 4H), 2.06 - 1.91 (m, 3H), 1.89 - 1.76 (m, 2H), 1.45 - 1.30 (m, 2H). LCMS (ESI) m/z: calcd for C₄₄H₄₇F₃N₇O₄⁺[M+H]⁺, 794.37; found, 794.4.

### Example 21: Preparation of 3-(4-((4-(4-((9-cyclopentyl-8-(phenylamino)-9H-purin-2-yl)amino)phenyl)piperazin-1-yl)methyl)phenyl)piperidine-2,6-dione (GT-03740)

According to the method of step 2 of Scheme I-1, the target compound (GT-03740) was prepared (white solid, 29 mg, yield 43.43%). ¹H NMR (400 MHz, DMSO) δ 11.27 (s, 1H), 10.87 (s, 1H), 10.10 (s, 1H), 9.93 (s, 1H), 8.41 (s, 1H), 7.82 (d, *J* = 8.0 Hz, 2H), 7.63 (d, *J* = 8.1 Hz, 2H), 7.48 (d, *J* = 8.9 Hz, 2H), 7.41 - 7.33 (m, 4H), 7.11 (t, *J* = 7.3 Hz, 1H), 7.01 (d, *J* = 9.0 Hz, 2H), 5.15 (p, *J* = 8.6 Hz, 1H), 4.37 (d, *J =* 1.8 Hz, 2H), 3.94 (d, *J* = 6.9 Hz, 1H), 3.77 (d, *J* = 7.9 Hz, 2H), 3.38 (d, *J* = 4.9 Hz, 2H), 3.17 (d, *J* = 7.5 Hz, 4H), 2.74 - 2.66 (m, 1H), 2.55 (dd, *J* = 8.8, 4.4 Hz, 1H), 2.34 - 2.20 (m, 3H), 2.10 - 2.01 (m, 3H), 1.95 - 1.87 (m, 2H), 1.63 (dd, *J* = 12.8, 6.9 Hz, 2H). LCMS (ESI) calcd for C₃₈H₄₂N₉O₂+ [M+H]⁺: 656.34; found, 656.40.

### Example 22: Preparation of 3-(4-((4-(4-((9-cyclopentyl-8-(phenylamino)-9H-purin-2-yl)amino)phenyl)piperazin-1-yl)methyl)-2-fluorophenyl)piperidine-2,6-dione (GT-03741)

According to the method of step 2 of Scheme I-1, the target compound (GT-03741) was prepared (white solid, 30 mg, yield 43.73%). ¹H NMR (400 MHz, DMSO) δ 11.61 (s, 1H), 10.92 (s, 1H), 10.21 (s, 1H), 9.99 (s, 1H), 8.42 (s, 1H), 7.83 (d, *J =* 7.9 Hz, 2H), 7.63 (d, *J =* 11.4 Hz, 1H), 7.44 (ddd, *J =* 25.6, 11.1, 5.6 Hz, 6H), 7.12 (t, *J* = 7.3 Hz, 1H), 7.02 (d, *J* = 9.0 Hz, 2H), 5.18 (p, *J* = 8.3 Hz, 1H), 4.40 (s, 2H), 4.13 (d, *J* = 7.8 Hz, 1H), 3.78 (d, *J* = 9.1 Hz, 2H), 3.39 (d, *J* = 7.7 Hz, 2H), 3.24 - 3.15 (m, 4H), 2.81 - 2.71 (m, 1H), 2.59 - 2.53 (m, 1H), 2.34 - 2.21 (m, 3H), 2.10 - 1.99 (m, 3H), 1.94 - 1.83 (m, 2H), 1.65 - 1.55 (m, 2H). LCMS (ESI) calcd for C₃₈H₄₁FN₉O₂⁺ [M+H]⁺: 674.33; found, 674.30.

### Example 23: Preparation of 3-(4-(((1-(4-((9-cyclopentyl-8-(phenylamino)-9H-purin-2-yl)amino)phenyl)piperidin-4-yl)(methyl)amino)methyl)phenyl)piperidine-2,6-dione (GT-03863)

According to the method of step 2 of Scheme I-1, the target compound (GT-03863) was prepared (white solid, 27 mg, yield 40.99%). ¹H NMR (400 MHz, DMSO) δ 11.45 (s, 1H), 10.88 (s, 1H), 10.54 (s, 1H), 8.50 (s, 1H), 7.81 (d, *J* = 7.9 Hz, 2H), 7.79 - 7.59 (m, 6H), 7.42 (t, *J* = 7.8 Hz, 2H), 7.33 (d, *J* = 7.9 Hz, 2H), 7.17 (t, *J* = 7.3 Hz, 1H), 5.34 (dd, *J* = 16.7, 8.2 Hz, 1H), 4.45 (d, *J* = 11.5 Hz, 3H), 4.28 (dd, *J* = 12.3, 6.1 Hz, 2H), 3.95 (dd, *J =* 11.6, 4.8 Hz, 1H), 3.71 (d, *J =* 25.5 Hz, 3H), 3.55 (d, *J =* 7.6 Hz, 2H), 2.74 - 2.61 (m, 4H), 2.53 (d, *J =* 4.2 Hz, 1H), 2.44 (d, *J =* 11.6 Hz, 1H), 2.37 - 2.20 (m, 3H), 2.07 (ddd, *J =* 13.8, 12.5, 4.9 Hz, 3H), 1.94 (s, 2H), 1.71 - 1.60 (m, 2H). LCMS (ESI) calcd for C₄₀H₄₆N₉O₂⁺ [M+H]⁺: 684.37; found, 684.40.

### Example 24: Preparation of 3-(4-(((1-(4-((9-cyclopentyl-8-(phenylamino)-9H-purin-2-yl)amino)phenyl)piperidin-4-yl)(methyl)amino)methyl)-2-fluorophenyl)piperidine-2,6-dione (GT-03864)

According to the method of step 2 of Scheme I-1, the target compound (GT-03864) was prepared (white solid, 13 mg, yield 17.88%). ¹H NMR (400 MHz, DMSO) δ 11.47 (s, 1H), 10.93 (s, 1H), 10.37 (s, 1H), 8.47 (s, 1H), 7.81 (d, *J =* 8.0 Hz, 2H), 7.69 (d, *J =* 9.8 Hz, 3H), 7.63 - 7.50 (m, 3H), 7.42 (dd, *J =* 14.0, 7.4 Hz, 3H), 7.15 (t, *J =* 7.3 Hz, 1H), 5.34 - 5.22 (m, 1H), 4.49 (d, *J =* 13.0 Hz, 1H), 4.30 (dd, *J =* 11.4, 4.6 Hz, 1H), 4.13 (dd, *J =* 12.5, 4.8 Hz, 2H), 3.77 (s, 3H), 3.62 (s, 2H), 3.39 (s, 2H), 2.81 - 2.73 (m, 1H), 2.63 (s, 3H), 2.57 (d, *J =* 3.0 Hz, 1H), 2.36 (dd, *J =* 14.2, 6.4 Hz, 3H), 2.24 (dd, *J =* 12.8, 3.9 Hz, 1H), 2.14 - 2.07 (m, 2H), 2.02 - 1.90 (m, 3H), 1.69 - 1.60 (m, 2H). LCMS (ESI) calcd for C₄₀H₄₅FN₉O₂⁺ [M+H]⁺: 702.36; found, 702.40.

### Example 25: Preparation of 3-(4-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)phenyl)piperidine-2,6-dione (GT-03913)

According to the method of step 2 of Scheme I-1, the target compound (GT-03913) was prepared (white solid, 40.00 mg, yield 71.26%). ¹H NMR (400 MHz, DMSO) δ 12.87 (s, 1H), 11.11 (s, 1H), 10.83 (s, 1H), 8.81 (dt, *J =* 20.6, 10.3 Hz, 3H), 8.66 (s, 1H), 8.28 (s, 1H), 7.91 (d, *J* = 9.4 Hz, 1H), 7.59 (d, *J =* 8.1 Hz, 2H), 7.38 (s, 1H), 7.28 (d, *J =* 8.1 Hz, 2H), 6.66 (s, 1H), 4.34 (d, *J =* 4.3 Hz, 2H), 3.87 (dd, *J =* 11.7, 4.9 Hz, 1H), 3.74 (s, 3H), 3.30 (d, *J =* 9.4 Hz, 3H), 3.20 - 3.06 (m, 6H), 2.63 (ddd, *J =* 16.9, 11.8, 5.1 Hz, 1H), 2.48 (t, *J* = 4.0 Hz, 1H), 2.24 - 2.15 (m, 1H), 2.04 (s, 3H), 1.99 (s, 3H), 1.95 (s, 3H). LCMS (ESI) calcd for C₃₈H₄₂BrN₉O₄P⁺ [M+H]⁺: 798.22; found, 798.20.

### Example 26: Preparation of 3-(4-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-2-fluorophenyl)piperidine-2,6-dione (GT-03914)

According to the method of step 2 of Scheme I-1, the target compound (GT-03914) was prepared (white solid, 40.00 mg, yield 69.70%). ¹H NMR (400 MHz, DMSO) δ 12.98 (s, 1H), 11.34 (s, 1H), 10.94 (s, 1H), 8.88 (dt, *J =* 26.5, 13.2 Hz, 4H), 8.35 (s, 1H), 7.98 (d, *J =* 9.3 Hz, 1H), 7.64 (d, *J =* 11.2 Hz, 1H), 7.48 (dd, *J* = 18.7, 9.0 Hz, 3H), 6.73 (s, 1H), 4.43 (d, *J* = 4.4 Hz, 2H), 4.13 (dd, *J =* 12.5, 4.9 Hz, 1H), 3.81 (s, 3H), 3.37 (d, *J* = 10.1 Hz, 3H), 3.27 - 3.14 (m, 6H), 2.81 - 2.72 (m, 1H), 2.59 - 2.53 (m, 1H), 2.31 - 2.20 (m, 1H), 2.11 (s, 3H), 2.06 (s, 3H), 2.02 (s, 3H). LCMS (ESI) calcd for C₃₈H₄₁BrFN₉O₄P⁺ [M+H]⁺: 816.21; found, 816.20.

### Example 27: Preparation of 3-(4-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)phenyl)piperidine-2,6-dione (GT-03930)

According to the method of step 2 of Scheme I-1, the target compound was prepared (white solid, 23.00 mg, yield 41.14%). ¹H NMR (400 MHz, DMSO) δ 13.30 (s, 1H), 11.15 (s, 1H), 10.87 (s, 1H), 9.50 (s, 1H), 8.94 (s, 2H), 8.75 (s, 1H), 8.47 (s, 1H), 8.00 (d, *J* = 9.4 Hz, 1H), 7.69 (d, *J* = 8.1 Hz, 2H), 7.43 - 7.32 (m, 3H), 6.90 (s, 1H), 4.50 (dd, *J* = 12.7, 3.3 Hz, 1H), 4.25 (dd, *J =* 13.0, 6.6 Hz, 2H), 3.93 (d, *J* = 4.8 Hz, 2H), 3.81 (s, 3H), 3.42 - 3.35 (m, 1H), 3.33 - 3.23 (m, 2H), 2.97 - 2.80 (m, 2H), 2.70 (dd, *J* = 11.4, 6.1 Hz, 1H), 2.65 (d, *J* = 4.6 Hz, 3H), 2.53 (d, *J =* 4.6 Hz, 1H), 2.27 (ddd, *J =* 20.7, 19.6, 13.8 Hz, 4H), 2.16 (s, 3H), 2.07 (s, 3H), 2.03 (s, 3H). LCMS (ESI) calcd for C₄₀H₄₆BrN₉O₄P⁺ [M+H]⁺: 825.25; found, 825.20.

### Example 28: Preparation of 3-(4-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-2-fluorophenyl)piperidine-2,6-dione (GT-03931)

According to the method of step 2 of Scheme I-1, the target compound (GT-03931) was prepared (white solid, 23.00 mg, yield 40.27%). ¹H NMR (400 MHz, DMSO) δ 13.01 (s, 1H), 10.94 (d, *J* = 12.7 Hz, 2H), 8.98 - 8.70 (m, 4H), 8.36 (s, 1H), 7.95 (dd, *J* = 13.7, 5.6 Hz, 1H), 7.67 - 7.61 (m, 1H), 7.52 - 7.38 (m, 3H), 6.83 (s, 1H), 4.60 - 4.49 (m, 1H), 4.31 - 4.22 (m, 1H), 4.13 (dd, *J* = 12.5, 4.7 Hz, 1H), 3.80 (s, 3H), 3.32 - 3.18 (m, 3H), 2.77 (ddd, *J* = 16.9, 11.8, 4.8 Hz, 3H), 2.67 (d, *J* = 4.4 Hz, 3H), 2.62 - 2.53 (m, 3H), 2.27 (dddd, *J* = 14.0, 9.1, 4.3, 2.1 Hz, 4H), 2.13 (s, 3H), 2.06 (s, 3H), 2.02 (s, 3H). LCMS (ESI) calcd for C₄₀H₄₅BrFN₉O₄P⁺ [M+H]⁺: 844.24; found, 844.20.

### Example 29: Preparation of 1-(3-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)phenyl)dihydropyrimidine-2,4(1H,3H)-dione (GT-04206)

According to the method of step 2 of Scheme I-1, the target compound (GT-04206) was prepared (white solid, 30 mg, yield 52.31%). ¹H NMR (400 MHz, DMSO) δ 12.92 (s, 1H), 10.92 (s, 1H), 10.48 (s, 1H), 8.89 (dt, *J =* 20.8, 10.4 Hz, 3H), 8.67 (s, 1H), 8.34 (s, 1H), 7.98 (d, *J =* 9.1 Hz, 1H), 7.66 (s, 1H), 7.53 - 7.42 (m, 4H), 6.74 (s, 1H), 4.47 (t, *J =* 12.9 Hz, 2H), 3.89 (t, *J =* 6.6 Hz, 2H), 3.81 (s, 3H), 3.41 (d, *J* = 11.1 Hz, 2H), 3.31 - 3.14 (m, 6H), 2.75 (t, *J =* 6.6 Hz, 2H), 2.11 (s, 3H), 2.04 (d, *J* = 14.4 Hz, 6H). LCMS (ESI) calcd for C₃₇H₄₁BrN₁₀O₄P⁺ [M+H]⁺: 799.22; found, 799.30.

### Example 30: Preparation of 1-(3-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)phenyl)dihydropyrimidine-2,4(1H,3H)-dione (GT-04214)

According to the method of step 2 of Scheme I-1, the target compound (GT-04214) was prepared (white solid, 19 mg, yield 33.27%). ¹H NMR (400 MHz, DMSO) δ 13.03 (s, 1H), 10.73 (s, 1H), 10.46 (s, 1H), 8.90 (dt, *J =* 31.7, 15.8 Hz, 4H), 8.37 (s, 1H), 7.98 (d, *J =* 9.1 Hz, 1H), 7.68 (s, 1H), 7.58 - 7.41 (m, 3H), 7.36 (d, *J* = 19.4 Hz, 1H), 6.82 (s, 1H), 4.54 (d, *J =* 10.1 Hz, 1H), 4.28 (dd, *J =* 13.3, 7.1 Hz, 1H), 3.88 (t, *J =* 6.6 Hz, 2H), 3.80 (s, 3H), 3.38 (s, 1H), 3.24 (s, 2H), 2.74 (t, *J =* 6.7 Hz, 4H), 2.68 (d, *J =* 4.7 Hz, 3H), 2.26 (d, *J =* 14.3 Hz, 2H), 2.14 (s, 3H), 2.05 (d, *J =* 14.4 Hz, 8H). LCMS (ESI) calcd for C₃₉H₄₅BrN₁₀O₄P⁺ [M+H]⁺: 827.25; found, 827.30.

### Example 31: Preparation of 1-(3-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)phenyl)dihydropyrimidine-2,4(1H,3H)-dione (GT-04260)

According to the method of step 2 of Scheme I-1, the target compound (GT-04260) was prepared (white solid, 25 mg, yield 33.17%). ¹H NMR (400 MHz, DMSO) δ 12.88 (s, 1H), 10.46 (s, 1H), 8.88 (dt, *J =* 20.0, 10.0 Hz, 3H), 8.60 (s, 1H), 8.32 (s, 1H), 7.95 (d, *J =* 9.3 Hz, 1H), 7.58 (s, 1H), 7.46 (s, 3H), 7.39 (s, 1H), 6.79 (s, 1H), 4.31 (s, 2H), 3.87 (t, *J =* 6.5 Hz, 2H), 3.80 (s, 3H), 3.63 (d, *J =* 69.2 Hz, 8H), 3.15 (dd, *J =* 19.2, 14.8 Hz, 3H), 2.74 (t, *J =* 6.6 Hz, 4H), 2.18 (s, 2H), 2.11 (s, 3H), 2.06 (s, 3H), 2.02 (s, 3H), 1.91 (s, 2H). LCMS (ESI) calcd for C₃₈H₃₇F₄N₈O₆⁺ [M+H]⁺: 882.29; found, 882.30.

### Example 32: Preparation of 3-(4-((4-(4-((5-chloro-4-((2-(dimethylphosphoryl)phenyl)amino)pyrimidin-2-yl)amino)-3-methoxyphenyl)piperazin-1-yl)methyl)phenyl)piperidine-2,6-dione (GT-03781)

According to the method of step 2 of Scheme I-1, the target compound (GT-03781) was prepared (white solid, 19 mg, yield 33.26%). ¹H NMR (400 MHz, DMSO) δ 11.99 (s, 1H), 11.65 (s, 1H), 10.87 (s, 1H), 9.43 (s, 1H), 8.36 (s, 1H), 8.27 (s, 1H), 7.69 - 7.57 (m, 3H), 7.44 - 7.31 (m, 4H), 7.24 (t, *J* = 7.5 Hz, 1H), 6.73 (d, *J =* 2.1 Hz, 1H), 6.55 (dd, *J =* 8.7, 2.2 Hz, 1H), 4.38 (d, *J =* 2.7 Hz, 2H), 3.92 (dd, *J =* 12.5, 7.6 Hz, 3H), 3.78 (s, 3H), 3.38 (d, *J* = 10.9 Hz, 2H), 3.28 (t, *J =* 12.2 Hz, 2H), 3.16 (dd, *J =* 19.9, 9.2 Hz, 2H), 2.74 - 2.65 (m, 1H), 2.58 - 2.52 (m, 1H), 2.30 - 2.18 (m, 1H), 2.05 (ddd, *J =* 13.5, 9.1, 4.8 Hz, 1H), 1.81 (s, 3H), 1.77 (s, 3H). LCMS (ESI) calcd for C₃₅H₄₀ClN₇O₄P⁺ [M+H]⁺: 688.25; found, 688.30.

### Example 33: Preparation of 3-(4-((4-(4-((5-chloro-4-((2-(dimethylphosphoryl)phenyl)amino)pyrimidin-2-yl)amino)-3-methoxyphenyl)piperazin-1-yl)methyl)-2-fluorophenyl)piperidine-2,6-dione (GT-03782)

According to the method of step 2 of Scheme I-1, the target compound (GT-03782) was prepared (white solid, 22 mg, yield 37.54%). ¹H NMR (400 MHz, DMSO) δ 12.23 (s, 1H), 11.99 (s, 1H), 10.92 (s, 1H), 9.83 (s, 1H), 8.34 (s, 2H), 7.65 (dd, *J =* 15.4, 9.3 Hz, 2H), 7.39 (ddd, *J =* 29.5, 23.1, 12.5 Hz, 5H), 6.75 (d, *J = 1.9* Hz, 1H), 6.58 (d, *J =* 8.6 Hz, 1H), 4.41 (s, 2H), 4.16 - 4.11 (m, 1H), 3.90 (d, *J =* 11.9 Hz, 2H), 3.79 (s, 3H), 3.35 (dd, *J =* 27.6, 12.3 Hz, 4H), 3.18 (dd, *J =* 19.7, 10.0 Hz, 2H), 2.82 - 2.73 (m, 1H), 2.58 (d, *J =* 2.9 Hz, 1H), 2.24 (dt, *J =* 12.4, 9.0 Hz, 1H), 2.06 - 1.98 (m, 1H), 1.82 (s, 3H), 1.78 (s, 3H). LCMS (ESI) calcd for C₃₅H₃₉ClFN₇O₄P⁺ [M+H]⁺: 706.24; found, 706.30.

### Example 34: Preparation of compounds (GT-03856) and (GT-03953)

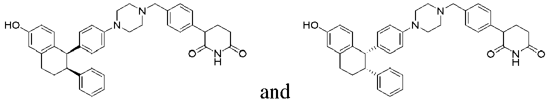

According to the method described in Step 2 of Scheme I-1, the target compounds were prepared using either the compounds (GT-M-160_P1) or (GT-M-160_P2) obtained from Intermediate Example 89 and 3-(4-(bromomethyl)phenyl)piperidine-2,6-dione as starting materials.

The compound (GT-M-160_P1) obtained from Intermediate Example 89 was used to prepare the target compound (GT-03856) as a white solid (20.00 mg, yield 26.26%). ¹H NMR (400 MHz, DMSO) δ 11.23 (s, 1H), 10.86 (s, 1H), 7.60 (d, *J =* 8.1 Hz, 2H), 7.31 (d, *J =* 8.1 Hz, 2H), 7.16 - 7.10 (m, 3H), 6.83 (d, *J =* 6.9 Hz, 2H), 6.60 (dd, *J =* 21.5, 8.6 Hz, 4H), 6.48 (dd, *J =* 8.2, 2.3 Hz, 1H), 6.24 (d, *J =* 8.6 Hz, 2H), 4.32 (d, *J =* 3.4 Hz, 2H), 4.16 (d, J = 4.8 Hz, 1H), 3.91 (dd, *J =* 11.7, 4.9 Hz, 1H), 3.29 (d, *J =* 7.0 Hz, 3H), 3.17 - 2.98 (m, 5H), 2.97 - 2.82 (m, 2H), 2.70 - 2.63 (m, 1H), 2.53 (s, 1H), 2.30 - 2.12 (m, 2H), 2.10 - 1.97 (m, 2H), 1.70 (dd, *J =* 11.3, 4.3 Hz, 1H). LCMS (ESI) calcd for C₃₈H₄₀N₃O₃⁺ [M+H]⁺: 586.30; found, 586.30.

The compound (GT-M-160_P2) obtained from Intermediate Example 89 was used to prepare the target compound (GT-03953) as a white solid (35 mg, yield 42%). ¹H NMR (400 MHz, DMSO) δ 10.87 (s, 1H), 10.80 (s, 1H), 9.12 (s, 1H), 7.56 (d, *J =* 8.1 Hz, 2H), 7.32 (d, *J =* 8.1 Hz, 2H), 7.18 - 7.10 (m, 3H), 6.84 (d, *J* = 6.7 Hz, 2H), 6.65 - 6.54 (m, 4H), 6.48 (dd, *J =* 8.3, 2.4 Hz, 1H), 6.25 (d, *J =* 8.6 Hz, 2H), 4.33 (d, *J =* 4.7 Hz, 2H), 4.16 (d, *J =* 4.9 Hz, 1H), 3.92 (dd, *J =* 11.7, 4.8 Hz, 1H), 3.66 - 3.64 (m, 2H), 3.38 - 3.26 (m, 3H), 3.17 - 2.84 (m, 6H), 2.74 - 2.63 (m, 1H), 2.55 - 2.52 (m, 1H), 2.21 (td, *J=* 12.0, 3.9 Hz, 1H), 2.14 - 1.98 (m, 2H), 1.75 - 1.67 (m, 1H). LCMS (ESI) m/z: calcd for C₃₈H₄₀N₃O₃⁺[M+H]⁺, 586.31; found, 586.3.

### Example 35: Preparation of compounds (GT-03857) and (GT-03954)

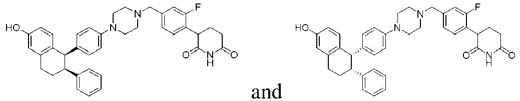

According to the method described in Step 2 of Scheme I-1, the target compounds were prepared using either the compounds (GT-M-160_P1) or (GT-M-160_P2) obtained from Intermediate Example 89 and 3-(4-(bromomethyl)-2-fluorophenyl)piperidine-2,6-dione as starting materials.

The compound (GT-M-160_P1) obtained from Intermediate Example 89 was used to prepare the target compound (GT-03857) as a white solid (43.00 mg, yield 54.77%). ¹H NMR (400 MHz, DMSO) δ 11.63 (s, 1H), 10.91 (s, 1H), 7.61 (d, *J =* 10.7 Hz, 1H), 7.43 (dt, *J =* 15.3, 7.8 Hz, 2H), 7.18 - 7.08 (m, 3H), 6.83 (d, *J =* 6.8 Hz, 2H), 6.67 - 6.53 (m, 4H), 6.49 (dd, *J =* 8.4, 2.3 Hz, 1H), 6.25 (d, *J =* 8.6 Hz, 2H), 4.35 (s, 2H), 4.16 - 4.08 (m, 3H), 3.62 (d, *J =* 7.2 Hz, 2H), 3.29 (d, *J =* 6.4 Hz, 2H), 3.08 (d, *J =* 7.8 Hz, 3H), 2.99 - 2.84 (m, 2H), 2.79 - 2.70 (m, 1H), 2.56 (d, *J =* 3.3 Hz, 1H), 2.33 - 2.15 (m, 2H), 2.04 (ddd, *J =* 13.2, 11.8, 5.6 Hz, 2H), 1.70 (d, *J =* 6.7 Hz, 1H). LCMS (ESI) calcd for C₃₈H₃₉FN₃O₃⁺ [M+H]⁺: 604.29; found, 604.30.

The compound (GT-M-160_P2) obtained from Intermediate Example 89 was used to prepare the target compound (GT-03954) as a white solid (37 mg, yield 44%). ¹H NMR (400 MHz, DMSO) δ 10.92 (s, 1H), 10.88 - 10.63 (m, 1H), 9.11 (s, 1H), 7.56 - 7.48 (m, 1H), 7.46 - 7.37 (m, 2H), 7.18 - 7.07 (m, 3H), 6.84 (d, *J* = 6.7 Hz, 2H), 6.65 - 6.54 (m, 4H), 6.48 (dd, *J =* 8.3, 2.4 Hz, 1H), 6.25 (d, *J =* 8.6 Hz, 2H), 4.40 - 4.30 (m, 2H), 4.16 (d, *J =* 4.9 Hz, 1H), 4.11 (dd, *J =* 12.7, 4.9 Hz, 1H), 3.70 - 3.63 (m, 2H), 3.40 - 3.24 (m, 3H), 3.17 - 2.84 (m, 6H), 2.82 - 2.71 (m, 1H), 2.59 - 2.51 (m, 1H), 2.22 (dt, *J =* 12.9, 9.0 Hz, 1H), 2.15 - 1.96 (m, 2H), 1.76 - 1.68 (m, 1H). LCMS (ESI) m/z: calcd for C₃₈H₃₉FN₃O₃⁺[M+H]⁺, 604.30; found, 604.3.

### Example 36: Preparation of 3-(4-((4-(4-(1,2-bis(4-hydroxyphenyl)but-1-en-1-yl)phenyl)piperazin-1-yl)methyl)phenyl)piperidine-2,6-dione (GT-04149)

According to the method of step 2 of Scheme I-1, the target compound (GT-04149) was prepared (white solid, 50 mg, yield 65.23%). ¹H NMR (400 MHz, DMSO) δ 11.35 (d, *J =* 39.7 Hz, 1H), 10.88 (t, *J =* 6.3 Hz, 1H), 7.61 (dt, *J =* 18.3, 9.2 Hz, 2H), 7.42 - 7.27 (m, 2H), 7.10 - 6.99 (m, 1H), 6.90 (ddd, *J =* 26.0, 8.7, 3.8 Hz, 4H), 6.74 (d, *J =* 8.4 Hz, 1H), 6.70 - 6.53 (m, 5H), 6.42 (d, *J =* 8.6 Hz, 1H), 4.42 - 4.28 (m, 2H), 3.93 (dt, *J =* 10.0, 4.8 Hz, 1H), 3.78 (dd, *J =* 24.2, 17.3 Hz, 3H), 3.33 (dd, *J =* 29.3, 8.5 Hz, 2H), 3.21 - 3.04 (m, 3H), 2.75 - 2.63 (m, 1H), 2.57 - 2.51 (m, 1H), 2.34 (d, *J =* 7.3 Hz, 2H), 2.21 (ddd, *J =* 20.5, 12.6, 7.1 Hz, 1H), 2.04 (dt, *J =* 13.2, 4.7 Hz, 1H), 0.83 (tt, *J =* 24.8, 12.4 Hz, 3H). LCMS (ESI) calcd for C₃₈H₃₀N₃O₄⁺ [M+H]⁺: 602.29; found, 602.30.

### Example 37: Preparation of 3-(4-((4-(4-(1,2-bis(4-hydroxyphenyl)but-1-en-1-yl)phenyl)piperazin-1-yl)methyl)-2-fluorophenyl)piperidine-2,6-dione (GT-04150)

According to the method of step 2 of Scheme I-1, the target compound (GT-04150) was prepared (white solid, 41 mg, yield 51.93%). ¹H NMR (400 MHz, DMSO) δ 11.51 (d, *J=* 39.7 Hz, 1H), 10.92 (d, *J =* 4.0 Hz, 1H), 7.61 (dd, *J =* 14.0, 11.6 Hz, 1H), 7.53 - 7.36 (m, 2H), 7.03 (t, *J =* 9.9 Hz, 1H), 6.91 (ddd, *J =* 26.2, 8.5, 4.7 Hz, 4H), 6.74 (d, *J =* 8.4 Hz, 1H), 6.72 - 6.56 (m, 5H), 6.43 (d, *J =* 8.5 Hz, 1H), 4.38 (d, *J =* 18.0 Hz, 2H), 4.17 - 4.07 (m, 1H), 3.69 (d, *J =* 9.0 Hz, 2H), 3.35 (dd, *J =* 29.2, 8.4 Hz, 2H), 3.23 - 3.03 (m, 4H), 2.83 - 2.70 (m, 1H), 2.61 - 2.53 (m, 1H), 2.33 (t, *J =* 9.6 Hz, 2H), 2.23 (td, *J =* 11.8, 6.2 Hz, 1H), 2.02 (d, *J* = 5.3 Hz, 1H), 0.84 (dd, *J =* 11.6, 7.2 Hz, 3H). LCMS (ESI) calcd for C₃₈H₂₉FN₃O₄⁺ [M+H]⁺: 620.28; found, 620.30.

### Example 38: Preparation of 3-(4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)piperidine-2,6-dione (GT-03905)

According to the method of step 2 of Scheme I-1, the target compound (GT-03905) was prepared (white solid, 50.00 mg, yield 78.00%). ¹H NMR (400 MHz, DMSO) δ 11.75 (s, 1H), 10.86 (s, 1H), 8.85 (d, *J* = 3.3 Hz, 1H), 8.30 (s, 1H), 8.20 (d, *J =* 10.4 Hz, 1H), 7.95 (s, 1H), 7.79 (t, *J =* 10.6 Hz, 2H), 7.65 (d, *J =* 8.0 Hz, 2H), 7.32 (d, *J =* 8.1 Hz, 2H), 4.90 (dd, *J =* 13.8, 6.9 Hz, 1H), 4.40 (s, 2H), 3.96 - 3.84 (m, 9H), 3.60 - 3.57 (m, 2H), 3.54 - 3.50 (m, 2H), 2.83 (t, *J =* 11.8 Hz, 2H), 2.75 (d, *J =* 6.4 Hz, 3H), 2.66 (dd, *J =* 11.8, 5.2 Hz, 1H), 2.26 - 2.17 (m, 3H), 2.07 - 2.00 (m, 1H), 1.84 (ddd, *J =* 12.2, 11.2, 6.9 Hz, 2H), 1.64 (d, *J =* 6.9 Hz, 6H). LCMS (ESI) calcd for C₄₁H₄₇F₂N₁₀O₂⁺ [M+H]⁺: 749.38; found, 749.40.

### Example 39: Preparation of 3-(2-fluoro-4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)piperidine-2,6-dione (GT-03906)

According to the method of step 2 of Scheme I-1, the target compound (GT-03906) was prepared (white solid, 40.00 mg, yield 60.94%). ¹H NMR (400 MHz, DMSO) δ 11.65 (s, 1H), 10.91 (s, 1H), 8.84 (d, *J* = 3.4 Hz, 1H), 8.28 (s, 1H), 8.17 (d, *J =* 8.8 Hz, 1H), 7.95 (s, 1H), 7.78 (dd, *J =* 18.0, 10.7 Hz, 2H), 7.61 (d, *J* = 11.2 Hz, 1H), 7.50 - 7.39 (m, 2H), 4.91 (dd, *J =* 13.9, 7.0 Hz, 1H), 4.45 - 4.34 (m, 2H), 4.11 (dd, *J =* 12.7, 4.9 Hz, 2H), 3.86 (s, 2H), 3.61 - 3.54 (m, 4H), 3.52 - 3.43 (m, 4H), 2.86 - 2.76 (m, 3H), 2.72 (s, 3H), 2.57 (d, *J* = 3.2 Hz, 1H), 2.27 - 2.18 (m, 3H), 2.03 - 1.97 (m, 1H), 1.86 (ddd, *J =* 14.7, 11.6, 5.3 Hz, 2H), 1.64 (d, *J* = 6.9 Hz, 6H). LCMS (ESI) calcd for C₄₁H₄₆F₃N₁₀O₂⁺ [M+H]⁺: 767.37; found, 767.40.

### Example 40: Preparation of 2-((2-((4-(4-(4-(2,6-dioxopiperidin-3-yl)benzyl)piperazin-1-yl)-2-methoxyphenyl)amino)-5-(trifluoromethyl)pyridin-4-yl)amino)-N-methylbenzamide (GT-03715)

According to the method of step 2 of Scheme I-1, the target compound (GT-03715) was prepared (white solid, 28 mg, yield 48.06%). ¹H NMR (400 MHz, DMSO) δ 11.69 (s, 1H), 10.88 (s, 1H), 10.56 (s, 1H), 8.81 (q, *J* = 4.0 Hz, 1H), 8.14 (s, 1H), 7.76 (d, *J =* 7.8 Hz, 1H), 7.65 (d, *J =* 8.1 Hz, 2H), 7.55 (d, *J* = 3.9 Hz, 2H), 7.33 (d, *J =* 8.1 Hz, 2H), 7.27 (dt, *J =* 8.2, 4.2 Hz, 1H), 7.20 (d, *J =* 8.5 Hz, 1H), 6.71 (s, 1H), 6.55 (dd, *J =* 13.1, 11.2 Hz, 2H), 4.36 (d, *J =* 3.4 Hz, 2H), 4.00 - 3.82 (m, 4H), 3.80 (s, 3H), 3.37 - 3.24 (m, 4H), 3.14 (d, *J=* 3.9 Hz, 1H), 2.75 (d, *J* = 4.5 Hz, 3H), 2.72 - 2.63 (m, 1H), 2.53 (d, *J* = 4.7 Hz, 1H), 2.24 (dt, *J =* 11.9, 8.5 Hz, 1H), 2.08 - 2.00 (m, 1H). LCMS (ESI) calcd for C₃₇H₃₉F₃N₇O₄⁺ [M+H]⁺: 702.29; found, 702.30.

### Example 41: Preparation of 2-((2-((4-(4-(4-(2,6-dioxopiperidin-3-yl)-3-fluorobenzyl)piperazin-1-yl)-2-methoxyphenyl)amino)-5-(trifluoromethyl)pyridin-4-yl)amino)-N-methylbenzamide (GT-03717)

According to the method of step 2 of Scheme I-1, the target compound (GT-03717) was prepared (white solid, 30 mg, yield 50.20%). ¹H NMR (400 MHz, DMSO) δ 11.86 (s, 1H), 10.93 (s, 1H), 10.58 (s, 1H), 9.98 (s, 1H), 8.80 (q, *J* = 4.2 Hz, 1H), 8.13 (s, 1H), 7.77 (d, *J =* 7.8 Hz, 1H), 7.64 (d, *J =* 11.3 Hz, 1H), 7.56 (d, *J* = 3.9 Hz, 2H), 7.46 (dt, *J =* 15.3, 7.8 Hz, 2H), 7.29 (dt, *J =* 8.2, 4.2 Hz, 1H), 7.18 (d, *J =* 8.7 Hz, 1H), 6.72 (s, 1H), 6.56 (dd, *J =* 20.6, 13.8 Hz, 2H), 4.40 (d, *J =* 3.0 Hz, 2H), 4.13 (dd, *J =* 12.6, 4.9 Hz, 2H), 3.89 (d, *J =* 11.9 Hz, 2H), 3.80 (s, 3H), 3.37 - 3.27 (m, 3H), 3.14 (dd, *J =* 20.0, 9.9 Hz, 2H), 2.83 - 2.74 (m, 4H), 2.59 - 2.53 (m, 1H), 2.25 (tt, *J* = 12.6, 6.5 Hz, 1H), 2.05 - 1.98 (m, 1H). LCMS (ESI) calcd for C₃₇H₃₈F₄N₇O₄⁺ [M+H]⁺: 720.28; found, 720.30.

### Example 42: Preparation of 2-((2-((4-(4-((4-(2,6-dioxopiperidin-3-yl)benzyl)(methyl)amino)piperidin-1-yl)-2-methoxyphenyl)amino)-5-(trifluoromethyl)pyridin-4-yl)amino)-N-methylbenzamide (GT-03733)

According to the method of step 2 of Scheme I-1, the target compound (GT-03733) was prepared (white solid, 33 mg, yield 51.10%). ¹H NMR (400 MHz, DMSO) δ 11.27 (s, 1H), 10.86 (s, 1H), 10.62 (s, 1H), 10.07 (s, 1H), 8.85 (d, *J =* 4.6 Hz, 1H), 8.19 (s, 1H), 7.78 (d, *J =* 7.8 Hz, 1H), 7.67 (d, *J =* 8.1 Hz, 2H), 7.56 (d, *J =* 3.9 Hz, 2H), 7.37 - 7.20 (m, 4H), 6.94 - 6.51 (m, 3H), 4.50 - 4.44 (m, 1H), 4.23 - 4.18 (m, 1H), 3.92 (dd, *J =* 12.9, 8.4 Hz, 4H), 3.82 (s, 3H), 3.50 - 3.43 (m, 1H), 2.94 (dd, *J =* 11.5, 4.6 Hz, 1H), 2.75 (d, *J* = 4.5 Hz, 3H), 2.71 - 2.65 (m, 1H), 2.58 (d, *J =* 4.6 Hz, 3H), 2.53 (d, *J =* 4.8 Hz, 1H), 2.39 (d, *J =* 11.2 Hz, 1H), 2.25 (ddd, *J* = 20.6, 10.2, 6.1 Hz, 2H), 2.14 - 1.90 (m, 3H). LCMS (ESI) calcd for C₃₉H₄₃F₃N₇O₄⁺ [M+H]⁺: 730.33; found, 730.30.

### Example 43: Preparation of 2-((2-((4-(4-((4-(2,6-dioxopiperidin-3-yl)-3-fluorobenzyl)(methyl)amino)piperidin-1-yl)-2-methoxyphenyl)amino)-5-(trifluoromethyl)pyridin-4-yl)amino)-N-methylbenzamide (GT-03734)

According to the method of step 2 of Scheme I-1, the target compound (GT-03734) was prepared (white solid, 33 mg, yield 46.65%). ¹H NMR (400 MHz, DMSO) δ 11.53 (s, 1H), 10.91 (s, 1H), 10.63 (s, 1H), 10.15 (s, 1H), 8.87 (d, *J =* 4.6 Hz, 1H), 8.20 (s, 1H), 7.79 (d, *J =* 7.8 Hz, 1H), 7.70 (d, *J =* 11.3 Hz, 1H), 7.55 (dd, *J* = 12.3, 6.0 Hz, 3H), 7.41 (t, *J* = 7.8 Hz, 1H), 7.31 - 7.23 (m, 2H), 7.00 - 6.56 (m, 3H), 4.50 (d, *J* = 10.4 Hz, 1H), 4.25 (dd, *J* = 12.9, 6.9 Hz, 2H), 4.13 (d, *J* = 7.8 Hz, 2H), 3.89 (d, *J* = 10.6 Hz, 2H), 3.83 (s, 3H), 3.53 - 3.47 (m, 1H), 3.04 - 2.98 (m, 1H), 2.75 (d, *J* = 4.5 Hz, 3H), 2.59 (d, *J =* 4.0 Hz, 3H), 2.53 (d, *J =* 2.1 Hz, 1H), 2.41 (d, *J* = 11.1 Hz, 1H), 2.33 - 2.22 (m, 2H), 2.14 - 1.97 (m, 3H). LCMS (ESI) calcd for C₃₉H₄₂F₄N₇O₄⁺ [M+H]⁺: 748.32; found, 748.30.

### Example 44: Preparation of 3-(4-((4-(4-((4-((3-(methylsulfonyl)benzyl)amino)-5-(trifluoromethyl)pyrimidin-2-yl)amino)phenyl)piperazin-1-yl)methyl)phenyl)piperidine-2,6-dione (GT-03822)

According to the method of step 2 of Scheme I-1, the target compound (GT-03822) was prepared (white solid, 20.00 mg, yield 28.63%). ¹H NMR (400 MHz, DMSO) δ 11.38 (s, 1H), 10.87 (s, 1H), 8.35 (s, 1H), 7.88 (d, *J =* 12.0 Hz, 1H), 7.80 (d, *J* = 7.3 Hz, 1H), 7.68 - 7.52 (m, 4H), 7.31 (dd, *J =* 19.1, 7.9 Hz, 4H), 6.88 (d, *J* = 8.7 Hz, 2H), 4.72 (d, *J* = 5.4 Hz, 2H), 4.36 (s, 2H), 3.93 (dd, *J* = 11.7, 4.8 Hz, 2H), 3.74 (s, 2H), 3.36 (d, *J* = 8.6 Hz, 2H), 3.16 (d, *J* = 15.2 Hz, 7H), 2.73 - 2.65 (m, 1H), 2.55 - 2.52 (m, 1H), 2.29 - 2.18 (m, 1H), 2.06 (dt, *J =* 8.5, 4.5 Hz, 1H). LCMS (ESI) calcd for C₃₇H₃₇F₃N₈O₆S⁺ [M+H]⁺: 708.25; found, 708.30.

### Example 45: Preparation of 3-(2-fluoro-4-((4-(4-((4-((3-(methylsulfonyl)benzyl)amino)-5-(trifluoromethyl)pyrimidin-2-yl)amino)phenyl)piperazin-1-yl)methyl)phenyl)piperidine-2,6-dione (GT-03823)

According to the method of step 2 of Scheme I-1, the target compound (GT-03823) was prepared (white solid, 20.00 mg, yield 27.92%). ¹H NMR (400 MHz, DMSO) δ 11.61 (s, 1H), 10.92 (s, 1H), 8.36 (s, 1H), 7.90 (s, 1H), 7.81 (d, *J* = 7.3 Hz, 1H), 7.62 (dd, *J* = 13.6, 6.1 Hz, 2H), 7.54 (dd, *J* = 10.5, 6.9 Hz, 1H), 7.46 (dt, *J* = 15.2, 7.8 Hz, 2H), 7.29 (d, *J* = 7.6 Hz, 2H), 6.89 (d, *J* = 8.8 Hz, 2H), 4.72 (d, *J* = 5.5 Hz, 2H), 4.39 (s, 2H), 4.11 (dt, *J* = 23.9, 11.9 Hz, 2H), 3.75 (s, 2H), 3.37 (d, *J* = 9.1 Hz, 2H), 3.23 - 3.11 (m, 7H), 2.82 - 2.73 (m, 1H), 2.59 - 2.54 (m, 1H), 2.23 (tt, J = 12.6, 6.4 Hz, 1H), 2.06 - 1.94 (m, 1H). LCMS (ESI) calcd for C₃₅H₃₆F₄N₇O₄S⁺ [M+H]⁺: 726.24; found, 726.30.

### Example 46: Preparation of 2-((2-((4-(4-(4-(2,6-dioxopiperidin-3-yl)benzyl)piperazin-1-yl)-2-isopropoxy-5-methylphenyl)amino)-5-(trifluoromethyl)pyridin-4-yl)amino)-N-methylbenzamide (GT-03946)

According to the method of step 2 of Scheme I-1, the target compound (GT-03946) was prepared (white solid, 25.00 mg, yield 44.14%). ¹H NMR (400 MHz, DMSO) δ 11.49 (s, 1H), 10.87 (s, 1H), 10.58 (s, 1H), 9.70 (s, 1H), 8.78 (q, *J* = 4.0 Hz, 1H), 8.23 (s, 1H), 7.76 (d, *J* = 6.9 Hz, 1H), 7.67 (d, *J* = 8.1 Hz, 2H), 7.60 (d, *J* = 8.0 Hz, 1H), 7.53 (t, *J* = 7.4 Hz, 1H), 7.33 (d, *J* = 8.1 Hz, 2H), 7.25 (dd, *J* = 15.6, 8.4 Hz, 2H), 6.70 (s, 1H), 6.60 (s, 1H), 4.55 (dt, *J =* 12.0, 6.0 Hz, 1H), 4.38 (d, *J =* 3.5 Hz, 2H), 3.93 (dd, *J =* 11.7, 4.9 Hz, 1H), 3.34 (d, *J =* 6.5 Hz, 2H), 3.19 (s, 6H), 2.74 (t, *J =* 6.1 Hz, 3H), 2.72 - 2.64 (m, 1H), 2.56 - 2.52 (m, 1H), 2.29 - 2.21 (m, 1H), 2.18 (s, 3H), 2.04 (ddd, *J* = 13.4, 9.0, 4.6 Hz, 1H), 1.19 (s, 3H), 1.17 (s, 3H). LCMS (ESI) calcd for C₄₀H₄₅F₃N₇O₄⁺ [M+H]⁺: 744.34; found, 744.30.

### Example 47: Preparation of 2-((2-((4-(4-(4-(2,6-dioxopiperidin-3-yl)-3-fluorobenzyl)piperazin-1-yl)-2-isopropoxy-5-methylphenyl)amino)-5-(trifluoromethyl)pyridin-4-yl)amino)-N-methylbenzamide (GT-03947)

According to the method of step 2 of Scheme I-1, the target compound (GT-03947) was prepared (white solid, 25.00 mg, yield 43.10%). ¹H NMR (400 MHz, DMSO) δ 11.79 (s, 1H), 10.92 (s, 1H), 10.62 (s, 1H), 9.90 (s, 1H), 8.80 (q, *J =* 4.4 Hz, 1H), 8.24 (s, 1H), 7.77 (dd, *J =* 7.8, 0.9 Hz, 1H), 7.68 (d, *J =* 11.0 Hz, 1H), 7.61 (d, *J* = 7.9 Hz, 1H), 7.56 - 7.50 (m, 2H), 7.43 (t, *J =* 7.8 Hz, 1H), 7.28 (t, *J =* 7.4 Hz, 1H), 7.19 (s, 1H), 6.71 (s, 1H), 6.59 (s, 1H), 4.57 (dt, *J* = 12.1, 6.0 Hz, 1H), 4.41 (d, *J =* 2.5 Hz, 2H), 4.13 (dd, *J* = 12.5, 4.9 Hz, 1H), 3.34 (d, *J =* 5.3 Hz, 2H), 3.21 (s, 6H), 2.79 (dd, *J* = 13.2, 5.4 Hz, 1H), 2.76 (t, *J* = 4.9 Hz, 3H), 2.58 - 2.53 (m, 1H), 2.28 - 2.21 (m, 1H), 2.18 (s, 3H), 2.05 - 1.94 (m, 1H), 1.18 (s, 3H), 1.17 (s, 3H). LCMS (ESI) calcd for C₄₀H₄₄F₄N₇O₄⁺ [M+H]⁺: 762.33; found, 762.30.

### Example 48: Preparation of N-(1-(4-(2,6-dioxopiperidin-3-yl)benzyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide (GT-04034)

According to the method of step 2 of Scheme I-1, the target compound (GT-04034) was prepared (white solid, 34 mg, yield 49.36%). ¹H NMR (400 MHz, DMSO) δ 10.88 (s, 1H), 10.69 (d, *J =* 54.3 Hz, 1H), 8.38 (ddd, *J* = 27.4, 19.1, 6.4 Hz, 2H), 7.71 - 7.52 (m, 3H), 7.45 (dd, *J* = 18.1, 7.0 Hz, 1H), 7.38 - 7.30 (m, 3H), 7.23 (dd, *J =* 10.9, 7.0 Hz, 1H), 6.88 (dd, *J* = 63.6, 7.8 Hz, 1H), 4.45 (d, *J* = 4.8 Hz, 1H), 4.37 (s, 1H), 4.26 (dd, *J* = 11.2, 5.0 Hz, 2H), 4.03 - 3.80 (m, 6H), 3.18 (s, 1H), 3.08 (dd, *J* = 19.5, 8.9 Hz, 2H), 3.00 (s, 2H), 2.92 (s, 1H), 2.73 - 2.65 (m, 1H), 2.55 (dd, *J* = 16.1, 4.4 Hz, 1H), 2.23 (dd, *J* = 12.4, 3.6 Hz, 1H), 2.04 (dd, *J* = 10.4, 6.2 Hz, 4H), 1.95 - 1.90 (m, 1H). LCMS (ESI) calcd for C₃₉H₃₈F₄N₇O₆⁺ [M+H]⁺: 776.27; found, 776.30.

### Example 49: Preparation of N-(1-(4-(2,6-dioxopiperidin-3-yl)-3-fluorobenzyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide (GT-04035)

According to the method of step 2 of Scheme I-1, the target compound (GT-04035) was prepared (white solid, 14 mg, yield 19.86%). ¹H NMR (400 MHz, DMSO) δ 10.93 (s, 1H), 8.62 (d, *J* = 6.8 Hz, 1H), 8.51 - 8.42 (m, 1H), 7.65 (t, *J =* 7.8 Hz, 1H), 7.57 (d, *J =* 10.7 Hz, 1H), 7.49 (d, *J =* 7.6 Hz, 1H), 7.38 (dt, *J =* 15.7, 5.4 Hz, 4H), 7.33 - 7.17 (m, 2H), 7.13 (d, *J* = 6.4 Hz, 1H), 4.45 (s, 2H), 4.38 - 4.27 (m, 3H), 4.14 - 4.10 (m, 1H), 3.91 (s, 1H), 3.86 (d, *J =* 9.7 Hz, 3H), 3.08 (d, *J =* 9.4 Hz, 2H), 2.92 (s, 3H), 2.76 (dd, *J =* 10.7, 6.3 Hz, 1H), 2.55 (dd, *J* = 18.2, 2.4 Hz, 2H), 2.23 (dd, *J* = 12.9, 3.6 Hz, 1H), 2.03 - 1.90 (m, 5H). LCMS (ESI) calcd for C₃₉H₃₇F₅N₇O₆⁺ [M+H]⁺: 794.26; found, 794.30.

### Example 50: Preparation of 2-((2-((4-(1-(4-(2,6-dioxopiperidin-3-yl)benzyl)piperidin-4-yl)-2-isopropoxy-5-methylphenyl)amino)-5-(trifluoromethyl)pyridin-4-yl)amino)-N-methylbenzamide (GT-04084)

According to the method of step 2 of Scheme I-1, the target compound (GT-04084) was prepared (white solid, 27 mg, yield 46.71%). ¹H NMR (400 MHz, DMSO) δ 11.24 (d, *J =* 41.1 Hz, 1H), 10.88 (s, 1H), 10.61 (d, *J =* 11.6 Hz, 1H), 9.76 (s, 1H), 8.81 (q, *J =* 4.4 Hz, 1H), 8.29 (d, *J =* 19.8 Hz, 1H), 7.82 - 7.70 (m, 1H), 7.68 - 7.56 (m, 3H), 7.52 (t, *J =* 8.2 Hz, 1H), 7.34 (t, *J =* 7.8 Hz, 2H), 7.27 - 7.16 (m, 2H), 6.93 (s, 1H), 6.65 (d, *J =* 14.8 Hz, 1H), 4.30 (d, *J =* 4.6 Hz, 2H), 3.94 (dd, *J =* 11.7, 4.9 Hz, 1H), 3.40 (d, *J =* 10.8 Hz, 2H), 3.15 - 2.91 (m, 4H), 2.75 (d, *J =* 4.5 Hz, 3H), 2.73 - 2.64 (m, 1H), 2.54 (dd, *J =* 12.0, 7.8 Hz, 1H), 2.30 - 2.20 (m, 6H), 2.08 - 2.01 (m, 1H), 1.83 (d, *J =* 12.6 Hz, 2H), 1.19 (d, *J =* 6.0 Hz, 6H). LCMS (ESI) calcd for C₄₁H₄₆F₃N₆O₄⁺ [M+H]⁺: 743.35; found, 743.40.

### Example 51: Preparation of 2-((2-((4-(1-(4-(2,6-dioxopiperidin-3-yl)-3-fluorobenzyl)piperidin-4-yl)-2-isopropoxy-5-methylphenyl)amino)-5-(trifluoromethyl)pyridin-4-yl)amino)-N-methylbenzamide (GT-04085)

According to the method of step 2 of Scheme I-1, the target compound (GT-04085) was prepared (white solid, 36 mg, yield 60.81%). LCMS (ESI) calcd for C₄₁H₄₅F₄N₆O₄⁺ [M+H]⁺: 761.34; found, 761.30.

### Example 52: Preparation of 1-(3-((4-(4-((4-((3-(methylsulfonyl)benzyl)amino)-5-(trifluoromethyl)pyrimidin-2-yl)amino)phenyl)piperazin-1-yl)methyl)phenyl)dihydropyrimidine-2,4(1H,3H)-dione (GT-04178)

According to the method of step 2 of Scheme I-1, the target compound (GT-04178) was prepared (white solid, 21 mg, yield 36.77%). ¹H NMR (400 MHz, DMSO) δ 11.49 (s, 1H), 10.62 (s, 1H), 10.45 (s, 1H), 8.93 (s, 1H), 8.43 (s, 1H), 7.90 (s, 1H), 7.82 (d, *J =* 7.4 Hz, 1H), 7.66 (s, 1H), 7.62 (t, *J =* 7.7 Hz, 1H), 7.55 - 7.46 (m, 4H), 7.27 (d, *J =* 8.2 Hz, 2H), 6.92 (d, *J =* 8.7 Hz, 2H), 4.73 (d, *J =* 5.4 Hz, 2H), 4.40 (s, 2H), 3.88 (d, *J =* 6.6 Hz, 2H), 3.81 - 3.75 (m, 2H), 3.39 (d, *J =* 10.2 Hz, 2H), 3.24 - 3.13 (m, 7H), 2.73 (dd, *J* = 12.1, 5.5 Hz, 2H). LCMS (ESI) calcd for C₄₀H₄₄F₄N₇O₄⁺ [M+H]⁺: 709.25; found, 709.30.

### Example 53: Preparation of N-(1-(3-(2,4-dioxotetrahydropyrimidin-1(2H)-yl)benzyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide (GT-04257)

According to the method of step 2 of Scheme I-1, the target compound (GT-04257) was prepared (white solid, 26 mg, yield 31.41%). ¹H NMR (400 MHz, DMSO) δ 10.46 (s, 2H), 7.64 - 7.55 (m, 1H), 7.49 - 7.39 (m, 8H), 6.97 (dd, *J =* 7.4, 0.7 Hz, 1H), 6.80 (dd, *J =* 8.2, 0.6 Hz, 1H), 4.78 (s, 4H), 4.30 (t, *J =* 8.7 Hz, 2H), 3.87 (dd, *J =* 8.4, 4.9 Hz, 5H), 3.80 (s, 2H), 3.09 (d, *J =* 10.3 Hz, 1H), 2.99 (d, *J =* 14.8 Hz, 3H), 2.72 (s, 2H), 2.05 (d, *J =* 11.4 Hz, 2H), 2.00 - 1.83 (m, 2H). LCMS (ESI) calcd for C₃₈H₃₇F₄N₈O₆⁺ [M+H]⁺: 777.27; found, 777.30.

### Example 54: Preparation of N-(4-(chlorodifluoromethoxy)phenyl)-6-(4-((4-(2,6-dioxopiperidin-3-yl)benzyl)(methyl)amino)piperidin-1-yl)-5-(1H-pyrazol-3-yl)nicotinamide (GT-04026)

According to the method of step 2 of Scheme I-1, the target compound (GT-04026) was prepared (white solid, 21 mg, yield 35.87%). ¹H NMR (400 MHz, DMSO) δ 10.87 (s, 1H), 10.67 (s, 1H), 10.54 (s, 1H), 8.80 (d, *J* = 2.4 Hz, 1H), 8.40 (d, *J =* 2.4 Hz, 1H), 7.95 - 7.89 (m, 2H), 7.83 (d, *J =* 2.1 Hz, 1H), 7.60 (d, *J* = 8.1 Hz, 2H), 7.33 (dd, *J =* 13.7, 8.7 Hz, 4H), 6.74 (d, *J =* 2.0 Hz, 1H), 4.47 - 4.43 (m, 1H), 4.18 - 4.14 (m, 1H), 3.94 - 3.90 (m, 1H), 3.82 - 3.75 (m, 2H), 3.41 (t, *J =* 11.8 Hz, 1H), 2.84 - 2.73 (m, 2H), 2.68 (td, *J* = 12.0, 5.9 Hz, 1H), 2.61 - 2.51 (m, 4H), 2.27 - 2.17 (m, 2H), 2.12 (d, *J =* 11.4 Hz, 1H), 2.07 - 2.01 (m, 1H), 1.93 - 1.81 (m, 2H). LCMS (ESI) calcd for C₃₄H₃₅ClF₂N₇O₄⁺ [M+H]⁺: 678.23; found, 678.30.

### Example 55: Preparation of N-(4-(chlorodifluoromethoxy)phenyl)-6-(4-((4-(2,6-dioxopiperidin-3-yl)-3-fluorobenzyl)(methyl)amino)piperidin-1-yl)-5-(1H-pyrazol-3-yl)nicotinamide (GT-04027)

According to the method of step 2 of Scheme I-1, the target compound (GT-04027) was prepared (white solid, 16 mg, yield 26.62%). ¹H NMR (400 MHz, DMSO) δ 10.89 (d, *J =* 20.3 Hz, 2H), 10.53 (s, 1H), 8.80 (d, *J* = 2.4 Hz, 1H), 8.40 (d, *J =* 2.4 Hz, 1H), 7.97 - 7.88 (m, 2H), 7.83 (d, *J =* 2.1 Hz, 1H), 7.60 (d, *J =* 11.2 Hz, 1H), 7.44 (dt, *J =* 25.2, 7.8 Hz, 2H), 7.35 (d, *J =* 9.1 Hz, 2H), 6.74 (d, *J =* 1.9 Hz, 1H), 4.48 (dd, *J =* 12.8, 2.7 Hz, 1H), 4.18 (d, *J =* 5.5 Hz, 1H), 4.11 (d, *J =* 7.8 Hz, 1H), 3.83 - 3.76 (m, 2H), 3.42 (t, J = 12.2 Hz, 1H), 2.85 - 2.71 (m, 3H), 2.56 (t, *J =* 11.1 Hz, 4H), 2.28 - 2.17 (m, 2H), 2.13 (d, *J* = 11.2 Hz, 1H), 2.05 - 1.97 (m, 1H), 1.93 - 1.79 (m, 2H). LCMS (ESI) calcd for C₃₄H₃₄ClF₃N₇O₄⁺ [M+H]⁺: 696.22; found, 696.30.

### Example 56: Preparation of N-(4-(chlorodifluoromethoxy)phenyl)-6-(4-(1-(4-(2,6-dioxopiperidin-3-yl)benzyl)piperidin-4-yl)piperazin-1-yl)-5-(1H-pyrazol-3-yl)nicotinamide (GT-04058)

According to the method of step 2 of Scheme I-1, the target compound (GT-04058) was prepared (white solid, 40 mg, yield 60.78%). ¹H NMR (400 MHz, DMSO) δ 11.11 (s, 1H), 10.87 (s, 1H), 10.59 (s, 1H), 8.83 (d, *J* = 2.3 Hz, 1H), 8.46 (d, *J =* 2.3 Hz, 1H), 7.92 (d, *J* = 9.1 Hz, 2H), 7.82 (d, *J =* 2.1 Hz, 1H), 7.57 (d, *J* = 8.1 Hz, 2H), 7.34 (dd, *J =* 11.5, 8.6 Hz, 4H), 6.82 (d, *J =* 2.1 Hz, 1H), 4.28 (s, 2H), 3.92 (s, 1H), 3.69 (s, 2H), 3.48 (d, *J =* 9.8 Hz, 5H), 3.35 (t, *J =* 12.6 Hz, 2H), 3.14 (d, *J =* 8.8 Hz, 2H), 2.97 (dd, *J =* 21.4, 11.4 Hz, 2H), 2.73 - 2.65 (m, 1H), 2.53 (t, *J =* 4.1 Hz, 1H), 2.39 - 2.30 (m, 2H), 2.22 (ddd, *J =* 24.5, 12.2, 3.2 Hz, 3H), 2.07 - 1.98 (m, 1H). LCMS (ESI) calcd for C₃₇H₄₀ClF₂N₈O₄⁺ [M+H]⁺: 733.28; found, 733.30.

### Example 57: Preparation of N-(4-(chlorodifluoromethoxy)phenyl)-6-(4-(1-(4-(2,6-dioxopiperidin-3-yl)-3-fluorobenzyl)piperidin-4-yl)piperazin-1-yl)-5-(1H-pyrazol-3-yl)nicotinamide (GT-04059)

According to the method of step 2 of Scheme I-1, the target compound (GT-04059) was prepared (white solid, 29 mg, yield 43.01%). ¹H NMR (400 MHz, DMSO) δ 11.29 (s, 1H), 10.92 (s, 1H), 10.60 (s, 1H), 8.83 (d, *J* = 2.3 Hz, 1H), 8.47 (d, *J =* 2.3 Hz, 1H), 7.95 - 7.91 (m, 2H), 7.83 (d, *J =* 2.1 Hz, 1H), 7.56 (d, *J* = 11.3 Hz, 1H), 7.42 (d, *J =* 4.0 Hz, 2H), 7.35 (d, *J =* 8.9 Hz, 2H), 6.82 (d, *J =* 2.1 Hz, 1H), 4.32 (s, 2H), 4.12 (d, *J* = 7.7 Hz, 1H), 3.73 (s, 2H), 3.48 (s, 5H), 3.33 (d, *J =* 12.4 Hz, 2H), 3.15 (d, *J =* 8.6 Hz, 2H), 2.99 (d, *J* = 9.1 Hz, 2H), 2.80 - 2.72 (m, 1H), 2.55 (dd, *J =* 13.7, 3.4 Hz, 1H), 2.35 (d, *J =* 11.9 Hz, 2H), 2.23 (dt, *J* = 12.4, 8.8 Hz, 3H), 2.04 - 1.98 (m, 1H). LCMS (ESI) calcd for C₃₇H₃₉ClF₃N₈O₄⁺ [M+H]⁺: 751.27; found, 751.30.

### Example 58: Preparation of N-(4-(4-((4-(2,6-dioxopiperidin-3-yl)benzyl)(methyl)amino)piperidin-1-yl)-3-(trifluoromethyl)phenyl)-3-(imidazo[1,2-b]pyridazin-3-ylethynyl)-4-methylbenzamide (GT-04106)

According to the method of step 2 of Scheme I-1, the target compound (GT-04106) was prepared (white solid, 33 mg, yield 50.16%). LCMS (ESI) calcd for C₄₀H₄₄F₄N₇O₄⁺ [M+H]⁺: 734.30; found, 734.30.

### Example 59: Preparation of N-(4-(4-((4-(2,6-dioxopiperidin-3-yl)-3-fluorobenzyl)(methyl)amino)piperidin-1-yl)-3-(trifluoromethyl)phenyl)-3-(imidazo[1,2-b]pyridazin-3-ylethynyl)-4-methylbenzamide (GT-04107)

According to the method of step 2 of Scheme I-1, the target compound (GT-04107) was prepared (white solid, 37 mg, yield 54.89%). LCMS (ESI) calcd for C₄₁H₃₈F₄N₇O₃⁺ [M+H]⁺: 752.29; found, 752.30.

### Example 60: Preparation of N-(2-(4-(4-(2,6-dioxopiperidin-3-yl)benzyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide (GT-03846)

According to the method of step 2 of Scheme I-1, the target compound (GT-03846) was prepared (white solid, 41 mg, yield 58.15%). ¹H NMR (400 MHz, DMSO) δ 10.87 (s, 1H), 10.11 (s, 1H), 9.20 (s, 1H), 8.92 (d, *J =* 6.1 Hz, 1H), 8.40 (s, 1H), 8.28 (d, *J =* 6.0 Hz, 1H), 7.73 (s, 1H), 7.63 (d, *J =* 8.1 Hz, 2H), 7.32 (d, *J* = 8.1 Hz, 2H), 4.36 (s, 3H), 4.30 - 4.23 (m, 3H), 3.94 (dd, *J =* 11.7, 4.8 Hz, 1H), 3.75 (t, *J =* 12.0 Hz, 2H), 3.61 (dd, *J =* 13.0, 6.6 Hz, 2H), 3.50 - 3.37 (m, 3H), 3.31 - 3.17 (m, 3H), 2.84 (s, 3H), 2.69 (td, *J =* 11.9, 6.0 Hz, 1H), 2.53 (d, *J =* 4.2 Hz, 1H), 2.22 (tt, *J =* 12.4, 6.4 Hz, 1H), 2.07 - 1.99 (m, 1H), 1.97 - 1.87 (m, 1H), 1.78 (dd, *J =* 9.4, 5.7 Hz, 1H). LCMS (ESI) calcd for C₃₆H₃₈N₉O₆⁺ [M+H]⁺: 692.29; found, 692.30.

### Example 61: Preparation of N-(2-(4-(4-(2,6-dioxopiperidin-3-yl)-3-fluorobenzyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide (GT-03847)

According to the method of step 2 of Scheme I-1, the target compound (GT-03847) was prepared (white solid, 39 mg, yield 53.90%). ¹H NMR (400 MHz, DMSO) δ 10.92 (s, 1H), 10.08 (s, 1H), 9.19 (s, 1H), 8.92 (d, *J* = 6.1 Hz, 1H), 8.38 (s, 1H), 8.27 (d, *J =* 5.9 Hz, 1H), 7.72 (s, 1H), 7.62 (d, *J =* 11.2 Hz, 1H), 7.48 - 7.37 (m, 2H), 4.39 (s, 3H), 4.28 (d, *J =* 2.2 Hz, 3H), 4.13 (dd, *J =* 12.6, 4.8 Hz, 2H), 3.75 (s, 2H), 3.65 - 3.58 (m, 2H), 3.48 - 3.36 (m, 3H), 3.27 (d, *J* = 10.2 Hz, 2H), 2.83 (s, 3H), 2.80 - 2.71 (m, 1H), 2.59 - 2.52 (m, 1H), 2.23 (qd, *J =* 12.7, 3.7 Hz, 1H), 2.05 - 1.97 (m, 1H), 1.96 - 1.86 (m, 1H), 1.82 - 1.70 (m, 1H). LCMS (ESI) calcd for C₃₆H₃₇FN₉O₆⁺ [M+H]⁺: 710.28; found, 710.30.

### Example 62: Preparation of 6-(5-cyano-1H-pyrrolo[2,3-b]pyridin-1-yl)-N-(1-(4-(2,6-dioxopiperidin-3-yl)benzyl)piperidin-4-yl)-4-(isopropylamino)nicotinamide (GT-04042)

According to the method of step 2 of Scheme I-1, the target compound (GT-04042) was prepared (white solid, 25 mg, yield 35.65%). ¹H NMR (400 MHz, DMSO) δ 11.04 (s, 1H), 10.87 (s, 1H), 8.86 - 8.80 (m, 2H), 8.69 (dd, *J* = 4.5, 2.1 Hz, 2H), 8.53 - 8.43 (m, 1H), 7.99 (d, *J* = 8.4 Hz, 1H), 7.69 - 7.58 (m, 2H), 7.32 (d, *J* = 8.2 Hz, 2H), 6.93 (dd, *J* = 7.8, 3.9 Hz, 1H), 4.29 (dd, *J* = 30.6, 5.1 Hz, 2H), 3.96 (ddd, *J* = 16.6, 13.5, 6.3 Hz, 3H), 3.36 (d, *J =* 11.0 Hz, 2H), 3.22 - 3.02 (m, 2H), 2.69 (ddd, *J =* 17.1, 11.9, 5.3 Hz, 1H), 2.53 (t, *J* = 4.0 Hz, 1H), 2.23 (qd, *J* = 12.6, 4.5 Hz, 1H), 2.12 - 1.91 (m, 5H), 1.28 (d, *J* = 6.3 Hz, 6H). LCMS (ESI) calcd for C₃₄H₃₇N₈O₃⁺ [M+H]⁺: 605.29; found, 605.30.

### Example 63: Preparation of 6-(5-cyano-1H-pyrrolo[2,3-b]pyridin-1-yl)-N-(1-(4-(2,6-dioxopiperidin-3-yl)-3-fluorobenzyl)piperidin-4-yl)-4-(isopropylamino)nicotinamide (GT-04043)

According to the method of step 2 of Scheme I-1, the target compound (GT-04043) was prepared (white solid, 33 mg, yield 45.70%). ¹H NMR (400 MHz, DMSO) δ 11.30 (s, 1H), 10.92 (s, 1H), 8.88 - 8.80 (m, 2H), 8.69 (dd, *J* = 3.8, 1.9 Hz, 2H), 8.51 (d, *J* = 3.9 Hz, 1H), 7.99 (d, *J* = 7.1 Hz, 1H), 7.64 (d, *J* = 11.1 Hz, 1H), 7.45 (dq, *J =* 22.7, 7.5 Hz, 2H), 6.93 (dd, *J =* 7.0, 3.9 Hz, 1H), 4.33 (dd, *J =* 32.2, 5.0 Hz, 2H), 4.22 - 4.07 (m, 2H), 3.85 (d, *J =* 5.6 Hz, 1H), 3.36 (d, *J =* 10.9 Hz, 2H), 3.25 - 3.01 (m, 2H), 2.85 - 2.68 (m, 1H), 2.61 - 2.51 (m, 1H), 2.23 (qd, *J* = 12.7, 3.7 Hz, 1H), 2.18 - 1.93 (m, 5H), 1.29 (d, *J* = 6.3 Hz, 6H). LCMS (ESI) calcd for C₃₄H₃₆FN₈O₃⁺ [M+H]⁺: 623.28; found, 623.30.

### Example 64: Preparation of 2-(2-((cyclopropylmethyl)amino)pyridin-4-yl)-N-(3-(difluoromethyl)-1-(1-(4-(2,6-dioxopiperidin-3-yl)benzyl)piperidin-4-yl)-1H-pyrazol-4-yl)oxazole-4-carboxamide (GT-04175)

According to the method of step 2 of Scheme I-1, the target compound (GT-04175) was prepared (white solid, 52 mg, yield 71.90%). ¹H NMR (400 MHz, DMSO) δ 11.29 (s, 1H), 10.82 (d, *J =* 10.9 Hz, 1H), 9.95 (d, *J =* 19.6 Hz, 1H), 9.10 - 9.01 (m, 1H), 8.13 (s, 1H), 8.01 (d, *J =* 6.3 Hz, 1H), 7.64 - 7.45 (m, 3H), 7.34 - 7.01 (m, 4H), 4.51 (t, *J* = 11.4 Hz, 1H), 4.25 (s, 2H), 3.87 (dd, *J =* 11.6, 4.8 Hz, 1H), 3.17 (d, *J =* 89.0 Hz, 6H), 2.63 (ddd, *J =* 17.0, 11.9, 5.1 Hz, 1H), 2.47 (dd, *J =* 12.1, 7.9 Hz, 1H), 2.40 - 2.28 (m, 2H), 2.27 - 2.09 (m, 3H), 2.05 - 1.92 (m, 1H), 1.14 - 0.95 (m, 1H), 0.52 - 0.41 (m, 2H), 0.25 (q, *J =* 4.9 Hz, 2H). LCMS (ESI) calcd for C₃₄H₃₇F₂N₈O₄⁺ [M+H]⁺: 659.28; found, 659.30.

### Example 65: Preparation of 2-(2-((cyclopropylmethyl)amino)pyridin-4-yl)-N-(3-(difluoromethyl)-1-(1-(4-(2,6-dioxopiperidin-3-yl)-3-fluorobenzyl)piperidin-4-yl)-1H-pyrazol-4-yl)oxazole-4-carboxamide (GT-04176)

According to the method of step 2 of Scheme I-1, the target compound (GT-04176) was prepared (white solid, 43 mg, yield 57.87%). ¹H NMR (400 MHz, DMSO) δ 11.50 (s, 1H), 10.93 (s, 1H), 10.01 (d, *J =* 11.9 Hz, 1H), 9.43 (s, 1H), 9.16 (d, *J =* 10.4 Hz, 1H), 8.18 (d, *J =* 21.7 Hz, 1H), 8.08 (d, *J =* 6.4 Hz, 1H), 7.72 - 7.60 (m, 2H), 7.51 - 7.41 (m, 2H), 7.39 - 7.07 (m, 2H), 4.59 (t, *J =* 11.5 Hz, 1H), 4.36 (s, 2H), 4.13 (dd, *J =* 12.6, 4.8 Hz, 1H), 3.26 (dd, *J =* 96.6, 9.2 Hz, 6H), 2.77 (ddt, *J =* 22.8, 14.9, 7.6 Hz, 1H), 2.63 - 2.53 (m, 1H), 2.48 - 2.36 (m, 2H), 2.31 - 2.16 (m, 3H), 2.08 - 1.97 (m, 1H), 1.22 - 1.07 (m, 1H), 0.60 - 0.52 (m, 2H), 0.39 - 0.31 (m, 2H). LCMS (ESI) calcd for C₃₄H₃₆F₃N₈O₄⁺ [M+H]⁺: 677.27; found, 677.30.

### Example 66: Preparation of N-(3-(difluoromethyl)-1-(4-(4-(4-(2,6-dioxopiperidin-3-yl)benzyl)piperazin-1-yl)phenyl)-1H-pyrazol-4-yl)-2-(2-((2,2,2-trifluoroethyl)amino)pyridin-4-yl)oxazole-4-carboxamide (GT-04273)

According to the method of step 2 of Scheme I-1, the target compound (GT-04273) was prepared (white solid, 35 mg, yield 59.52%). ¹H NMR (400 MHz, DMSO) δ 10.91 (d, *J =* 18.4 Hz, 2H), 10.01 (s, 1H), 9.02 (s, 1H), 8.71 (s, 1H), 8.25 (d, *J =* 5.4 Hz, 1H), 7.75 (t, *J =* 9.6 Hz, 3H), 7.61 (d, *J =* 8.2 Hz, 2H), 7.43 - 7.11 (m, 8H), 4.39 (d, *J =* 4.2 Hz, 2H), 4.27 (dt, *J =* 16.5, 8.1 Hz, 2H), 4.00 - 3.86 (m, 3H), 3.39 - 3.11 (m, 6H), 2.71 (ddd, *J =* 17.1, 11.9, 5.5 Hz, 1H), 2.55 (t, *J=* 6.5 Hz, 1H), 2.28 - 2.17 (m, 1H), 2.09 - 2.00 (m, 1H). LCMS (ESI) calcd for C₃₇H₃₅F₅N₉O₄⁺ [M+H]⁺: 764.27; found, 764.30.

### Example 67: Preparation of N-(3-(difluoromethyl)-1-(4-(4-(4-(2,6-dioxopiperidin-3-yl)-3-fluorobenzyl)piperazin-1-yl)phenyl)-1H-pyrazol-4-yl)-2-(2-((2,2,2-trifluoroethyl)amino)pyridin-4-yl)oxazole-4-carboxamide (GT-04274)

According to the method of step 2 of Scheme I-1, the target compound (GT-04274) was prepared (white solid, 36 mg, yield 59.81%). ¹H NMR (400 MHz, DMSO) δ 11.17 (s, 1H), 10.94 (s, 1H), 10.00 (s, 1H), 9.02 (s, 1H), 8.70 (s, 1H), 8.25 (d, *J =* 5.3 Hz, 1H), 7.76 (t, *J =* 11.6 Hz, 3H), 7.58 (d, *J =* 11.3 Hz, 1H), 7.30 (ddt, *J =* 42.4, 41.0, 8.8 Hz, 7H), 4.41 (s, 2H), 4.34 - 4.22 (m, 2H), 4.13 (dd, *J =* 12.7, 4.9 Hz, 1H), 3.91 (d, *J =* 10.6 Hz, 2H), 3.40 - 3.08 (m, 6H), 2.84 - 2.72 (m, 1H), 2.62 - 2.54 (m, 1H), 2.24 (td, *J =* 12.6, 8.7 Hz, 1H), 2.09 - 1.98 (m, 1H). LCMS (ESI) calcd for C₃₇H₃₄F₆N₉O₄⁺ [M+H]⁺: 782.26; found, 782.30.

### Example 68: Preparation of 3-(4-((4-((5-chloro-4-(5-(cyclopropylmethyl)-1-methyl-1H-pyrazol-4-yl)pyrimidin-2-yl)amino)piperidin-1-yl)methyl)phenyl)piperidine-2,6-dione (GT-04050)

According to the method of step 2 of Scheme I-1, the target compound (GT-04050) was prepared (white solid, 40 mg, yield 54.83%). ¹H NMR (400 MHz, DMSO) δ 11.08 (d, *J =* 31.3 Hz, 1H), 10.86 (s, 1H), 8.34 (d, *J* = 18.5 Hz, 1H), 7.97 (s, 1H), 7.63 - 7.58 (m, 2H), 7.31 (d, *J* = 8.1 Hz, 2H), 4.26 (d, *J* = 4.3 Hz, 2H), 3.92 (dd, *J =* 11.8, 4.9 Hz, 2H), 3.85 (s, 3H), 3.49 - 3.07 (m, 3H), 3.01 (d, *J =* 6.0 Hz, 3H), 2.68 (ddd, *J* = 11.9, 8.6, 4.1 Hz, 1H), 2.53 (t, *J =* 3.7 Hz, 1H), 2.33 - 2.09 (m, 2H), 2.06 (d, *J =* 10.1 Hz, 2H), 1.93 (dd, *J* = 25.8, 12.4 Hz, 2H), 0.91 (qd, *J =* 12.8, 6.9 Hz, 1H), 0.44 - 0.27 (m, 2H), 0.17 - 0.01 (m, 2H). LCMS (ESI) calcd for C₂₉H₃₅ClN₇O₂⁺ [M+H]⁺: 548.25; found, 548.30.

### Example 69: Preparation of 3-(4-((4-((5-chloro-4-(5-(cyclopropylmethyl)-1-methyl-1H-pyrazol-4-yl)pyrimidin-2-yl)amino)piperidin-1-yl)methyl)-2-fluorophenyl)piperidine-2,6-dione (GT-04051)

According to the method of step 2 of Scheme I-1, the target compound (GT-04051) was prepared (white solid, 38 mg, yield 50.43%). ¹H NMR (400 MHz, DMSO) δ 11.23 (d, *J =* 33.6 Hz, 1H), 10.92 (s, 1H), 8.34 (d, *J =* 18.1 Hz, 1H), 8.04 - 7.89 (m, 1H), 7.65 - 7.49 (m, 2H), 7.47 - 7.38 (m, 2H), 4.29 (d, *J =* 4.0 Hz, 2H), 4.12 (dd, *J =* 12.7, 4.8 Hz, 2H), 3.85 (s, 3H), 3.38 (d, *J =* 11.1 Hz, 1H), 3.28 - 3.16 (m, 1H), 2.99 (dd, *J* = 15.4, 6.3 Hz, 3H), 2.82 - 2.71 (m, 1H), 2.55 (dd, *J =* 17.3, 3.1 Hz, 2H), 2.36 - 2.09 (m, 2H), 2.07 - 1.88 (m, 4H), 1.01 - 0.85 (m, 1H), 0.43 - 0.26 (m, 2H), 0.19 - 0.02 (m, 2H). LCMS (ESI) calcd for C₂₉H₃₄FClN₇O₂⁺ [M+H]⁺: 566.24; found, 566.30.

### Example 70: Preparation of 4-(((5'-chloro-2'-((1-(4-(2,6-dioxopiperidin-3-yl)benzyl)piperidin-4-yl)amino)-[2,4'-bipyridin]-6-yl)amino)methyl)tetrahydro-2H-pyran-4-carbonitrile (GT-04066)

According to the method of step 2 of Scheme I-1, the target compound (GT-04066) was prepared (white solid, 30 mg, yield 50.63%). ¹H NMR (400 MHz, DMSO) δ 11.32 - 11.01 (m, 1H), 10.88 (s, 1H), 8.14 (d, *J =* 16.6 Hz, 1H), 7.69 (s, 1H), 7.62 (t, *J =* 7.4 Hz, 2H), 7.30 (dd, *J =* 19.6, 6.7 Hz, 2H), 6.93 (dd, *J =* 36.3, 9.7 Hz, 3H), 4.28 (d, *J =* 4.7 Hz, 2H), 3.98 - 3.87 (m, 4H), 3.73 (d, *J =* 7.7 Hz, 2H), 3.50 - 3.36 (m, 4H), 3.20 - 2.96 (m, 2H), 2.70 (ddd, *J =* 17.1, 11.9, 5.3 Hz, 1H), 2.52 (dd, *J =* 6.5, 2.7 Hz, 1H), 2.28 - 2.10 (m, 3H), 2.02 (ddd, *J =* 34.5, 15.9, 7.6 Hz, 3H), 1.87 (d, *J =* 13.1 Hz, 2H), 1.76 - 1.62 (m, 2H). LCMS (ESI) calcd for C₃₄H₃₉ClN₇O₃⁺ [M+H]⁺: 628.27; found, 628.30.

### Example 71: Preparation of 4-(((5'-chloro-2'-((1-(4-(2,6-dioxopiperidin-3-yl)-3-fluorobenzyl)piperidin-4-yl)amino)-[2,4'-bipyridin]-6-yl)amino)methyl)tetrahydro-2H-pyran-4-carbonitrile (GT-04067)

According to the method of step 2 of Scheme I-1, the target compound (GT-04067) was prepared (white solid, 25 mg, yield 41.02%). ¹H NMR (400 MHz, DMSO) δ 11.33 (d, *J =* 68.4 Hz, 1H), 10.93 (s, 1H), 8.18 - 8.06 (m, 1H), 7.77 - 7.67 (m, 1H), 7.62 (dd, *J =* 10.1, 8.9 Hz, 1H), 7.50 - 7.38 (m, 2H), 7.26 - 6.85 (m, 3H), 4.32 (d, *J =* 4.7 Hz, 2H), 4.15 - 4.11 (m, 1H), 4.00 (s, 1H), 3.95 - 3.89 (m, 2H), 3.73 (d, *J =* 6.9 Hz, 2H), 3.52 - 3.34 (m, 4H), 3.24 - 2.95 (m, 2H), 2.76 (qd, *J =* 13.3, 6.7 Hz, 1H), 2.60 - 2.53 (m, 1H), 2.23 (ddd, *J =* 31.8, 20.5, 9.0 Hz, 3H), 2.06 - 1.93 (m, 3H), 1.87 (d, *J =* 13.3 Hz, 2H), 1.69 (td, *J =* 13.6, 4.4 Hz, 2H). LCMS (ESI) calcd for C₃₄H₃₈FClN₇O₃⁺ [M+H]⁺: 646.26; found, 646.30.

### Example 72: Preparation of 4-(((4-(5-chloro-2-((1-(4-(2,6-dioxopiperidin-3-yl)benzyl)piperidin-4-yl)amino)pyridin-4-yl)thiazol-2-yl)amino)methyl)tetrahydro-2H-pyran-4-carbonitrile (GT-04074)

According to the method of step 2 of Scheme I-1, the target compound (GT-04074) was prepared (white solid, 33 mg, yield 55.79%). ¹H NMR (400 MHz, DMSO) δ 11.25 (d, *J =* 96.0 Hz, 1H), 10.87 (s, 1H), 8.42 (s, 1H), 8.05 (d, *J =* 20.1 Hz, 1H), 7.86 - 7.50 (m, 4H), 7.39 - 7.24 (m, 2H), 4.30 (d, *J =* 4.5 Hz, 3H), 4.02 (d, *J =* 10.7 Hz, 1H), 3.93 (d, *J =* 11.5 Hz, 2H), 3.72 (dd, *J =* 16.5, 5.4 Hz, 2H), 3.51 - 3.37 (m, 4H), 3.03 (dd, *J =* 22.0, 10.5 Hz, 2H), 2.69 (ddd, *J =* 17.0, 11.9, 5.2 Hz, 1H), 2.57 - 2.52 (m, 1H), 2.23 (ddd, *J =* 26.2, 17.6, 9.1 Hz, 3H), 2.09 - 1.96 (m, 3H), 1.94 - 1.83 (m, 2H), 1.79 - 1.64 (m, 2H). LCMS (ESI) calcd for C₃₂H₃₇ClN₇O₃S⁺ [M+H]⁺: 634.23; found, 634.30.

### Example 73: Preparation of 4-(((4-(5-chloro-2-((1-(4-(2,6-dioxopiperidin-3-yl)-3-fluorobenzyl)piperidin-4-yl)amino)pyridin-4-yl)thiazol-2-yl)amino)methyl)tetrahydro-2H-pyran-4-carbonitrile (GT-04075)

According to the method of step 2 of Scheme I-1, the target compound (GT-04075) was prepared (white solid, 31 mg, yield 50.96%). ¹H NMR (400 MHz, DMSO) δ 11.45 (d, *J =* 89.7 Hz, 1H), 10.98 - 10.86 (m, 1H), 8.37 (d, *J =* 38.0 Hz, 1H), 8.05 (d, *J =* 22.0 Hz, 1H), 7.75 - 7.55 (m, 3H), 7.53 - 7.37 (m, 2H), 4.33 (d, *J* = 4.2 Hz, 2H), 4.13 - 4.04 (m, 2H), 3.93 (d, *J=* 11.6 Hz, 2H), 3.72 (dd, *J =* 15.6, 5.0 Hz, 2H), 3.51 - 3.37 (m, 4H), 3.21 - 2.95 (m, 2H), 2.86 - 2.67 (m, 1H), 2.55 (d, *J =* 21.2 Hz, 1H), 2.30 - 2.13 (m, 3H), 2.05 (dd, J= 24.3, 11.5 Hz, 3H), 1.88 (d, *J=* 13.2 Hz, 2H), 1.79 - 1.64 (m, 2H). LCMS (ESI) calcd for C₃₂H₃₆FCIN_{703S}⁺ [M+H]⁺: 652.22; found, 652.30.

### Example 74: Preparation of 3-(4-((4-(6-(6-((R)-2-(3-fluorophenyl)pyrrolidin-1-yl)imidazo[1,2-b]pyridazin-3-yl)pyridin-2-yl)piperazin-1-yl)methyl)phenyl)piperidine-2,6-dione (GT-03870)

According to the method of step 2 of Scheme I-1, the target compound (GT-03870) was prepared (white solid, 67 mg, yield 92.17%). ¹H NMR (400 MHz, DMSO) δ 11.68 (s, 1H), 10.87 (s, 1H), 8.40 (s, 1H), 8.05 (d, *J* = 9.9 Hz, 1H), 7.80 - 7.46 (m, 4H), 7.38 (ddd, *J* = 26.1, 14.1, 7.4 Hz, 3H), 7.28 - 7.09 (m, 3H), 7.05 (t, *J* = 8.5 Hz, 1H), 6.96 - 6.84 (m, 1H), 5.20 (dd, *J* = 8.0, 2.0 Hz, 1H), 4.60 - 4.43 (m, 2H), 4.35 (s, 2H), 4.06 - 4.00 (m, 1H), 3.94 (dd, *J* = 11.7, 4.9 Hz, 1H), 3.73 - 3.67 (m, 2H), 3.46 - 3.42 (m, 2H), 3.35 (dd, *J =* 8.8, 5.3 Hz, 3H), 3.12 - 3.01 (m, 2H), 2.70 (ddd, *J* = 17.3, 11.9, 5.3 Hz, 1H), 2.30 - 2.18 (m, 1H), 2.14 - 1.98 (m, 3H), 1.90 (ddd, *J* = 12.2, 6.2, 3.9 Hz, 1H). LCMS (ESI) calcd for C₃₇H₃₈FN₈O₂⁺ [M+H]⁺: 645.30; found, 645.30.

### Example 75: Preparation of 3-(2-fluoro-4-((4-(6-(6-((R)-2-(3-fluorophenyl)pyrrolidin-1-yl)imidazo[1,2-b]pyridazin-3-yl)pyridin-2-yl)piperazin-1-yl)methyl)phenyl)piperidine-2,6-dione (GT-03871)

According to the method of step 2 of Scheme I-1, the target compound (GT-03871) was prepared (white solid, 54.00 mg, yield 72.28%). ¹H NMR (400 MHz, DMSO) δ 11.84 (s, 1H), 10.92 (s, 1H), 8.54 (s, 1H), 8.14 (d, *J* = 9.7 Hz, 1H), 7.62 (d, *J* = 11.0 Hz, 2H), 7.53 - 7.34 (m, 4H), 7.18 (d, *J* = 8.0 Hz, 2H), 7.05 (t, *J* = 8.0 Hz, 1H), 6.95 (d, *J* = 8.1 Hz, 1H), 5.23 (d, *J* = 7.7 Hz, 1H), 4.52 (t, *J* = 14.0 Hz, 2H), 4.38 (s, 2H), 4.09 (ddd, *J* = 14.7, 10.7, 4.8 Hz, 2H), 3.75 - 3.67 (m, 2H), 3.35 (d, *J* = 13.0 Hz, 3H), 3.18 - 2.99 (m, 3H), 2.81 - 2.73 (m, 1H), 2.58 (s, 1H), 2.23 (dt, *J* = 13.4, 7.9 Hz, 1H), 2.12 - 1.98 (m, 3H), 1.91 (dd, *J* = 12.3, 5.0 Hz, 1H). LCMS (ESI) calcd for C₃₇H₃₇F₂N₈O₂⁺ [M+H]⁺: 663.29; found, 663.30.

### Example 76: Preparation of 3-(4-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)phenyl)piperidine-2,6-dione (GT-03898)

According to the method of step 2 of Scheme I-1, the target compound (GT-03898) was prepared (white solid, 30.00 mg, yield 42.05%). ¹H NMR (400 MHz, DMSO) δ 10.86 (s, 1H), 8.53 (s, 1H), 8.16 (d, *J* = 10.5 Hz, 1H), 7.63 (d, *J* = 8.1 Hz, 2H), 7.43 - 7.35 (m, 2H), 7.31 (d, *J* = 8.1 Hz, 2H), 7.25 (d, *J* = 8.3 Hz, 1H), 7.18 (d, *J* = 7.7 Hz, 2H), 7.13 - 7.02 (m, 2H), 6.97 (d, *J* = 8.5 Hz, 1H), 5.24 (dd, *J* = 6.8, 5.5 Hz, 1H), 4.61 (d, *J* = 14.7 Hz, 2H), 4.46 (dd, *J* = 12.1, 2.5 Hz, 1H), 4.18 - 4.12 (m, 1H), 4.08 - 4.03 (m, 1H), 3.92 (dd, *J* = 11.7, 4.8 Hz, 2H), 3.72 (d, *J* = 9.3 Hz, 2H), 2.88 - 2.79 (m, 2H), 2.70 - 2.65 (m, 1H), 2.56 (s, 3H), 2.32 (dd, *J* = 12.9, 5.6 Hz, 2H), 2.22 (dd, *J* = 12.1, 3.1 Hz, 2H), 2.08 - 2.01 (m, 3H), 1.92 (dd, *J* = 8.8, 2.4 Hz, 1H), 1.81 - 1.68 (m, 2H). LCMS (ESI) calcd for C₃₉H₄₂FN₈O₂⁺ [M+H]⁺: 673.33; found, 673.30.

### Example 77: Preparation of 3-(2-fluoro-4-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)phenyl)piperidine-2,6-dione (GT-03899)

According to the method of step 2 of Scheme I-1, the target compound (GT-03899) was prepared (white solid, 30.00 mg, yield 40.96%). ¹H NMR (400 MHz, DMSO) δ 11.38 (s, 1H), 10.91 (s, 1H), 8.56 (s, 1H), 8.18 (d, *J =* 9.9 Hz, 1H), 7.66 (d, *J =* 11.3 Hz, 1H), 7.50 (d, *J =* 7.8 Hz, 3H), 7.45 - 7.34 (m, 3H), 7.18 (d, *J* = 8.1 Hz, 2H), 7.09 - 7.04 (m, 1H), 6.98 (d, *J =* 8.5 Hz, 1H), 5.23 (d, *J =* 6.3 Hz, 1H), 4.61 (d, *J* = 12.2 Hz, 2H), 4.51 - 4.46 (m, 1H), 4.22 - 4.17 (m, 1H), 4.14 - 4.07 (m, 2H), 3.75 - 3.69 (m, 2H), 3.56 - 3.47 (m, 2H), 2.90 - 2.75 (m, 3H), 2.55 (s, 3H), 2.34 (d, *J =* 10.5 Hz, 1H), 2.29 - 2.19 (m, 2H), 2.10 - 1.98 (m, 3H), 1.91 (dd, *J =* 9.5, 4.7 Hz, 1H), 1.86 - 1.69 (m, 2H). LCMS (ESI) calcd for C₃₉H₄₁F₂N₈O₂⁺ [M+H]⁺: 691.32; found, 691.30.

### Example 78: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-(2,6-dioxopiperidin-3-yl)benzyl)piperazin-1-yl)-1-(phenylthio)butan-2-yl)amino)-3-((trifluoromethyl)sulfonyl)phenyl)sulfonyl)benzamide (GT-04151)

According to the method of step 2 of Scheme I-1, the target compound (GT-04151) was prepared (white solid, 51 mg, yield 82.84%). ¹H NMR (400 MHz, DMSO) δ 12.16 (s, 1H), 10.86 (s, 1H), 10.64 (s, 1H), 8.18 (d, *J* = 1.9 Hz, 1H), 7.99 (d, *J* = 9.1 Hz, 1H), 7.77 (d, *J =* 8.8 Hz, 2H), 7.55 (s, 2H), 7.41 (d, *J =* 8.4 Hz, 2H), 7.29 (d, *J =* 7.4 Hz, 4H), 7.25 - 7.12 (m, 6H), 6.97 (dd, *J =* 16.0, 9.0 Hz, 3H), 4.20 (s, 2H), 3.97 - 3.82 (m, 3H), 3.50 (d, *J =* 48.7 Hz, 6H), 2.89 (s, 13H), 2.69 (ddd, *J =* 16.9, 13.8, 7.7 Hz, 4H), 2.33 - 1.98 (m, 8H), 1.46 (t, *J =* 6.0 Hz, 2H), 1.00 (s, 6H). LCMS (ESI) calcd for C₅₉H₆₈ClF₃N₇O₇S₃⁺ [M+H]⁺: 1174.39; found, 1174.40.

### Example 79: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-(2,6-dioxopiperidin-3-yl)-3-fluorobenzyl)piperazin-1-yl)-1-(phenylthio)butan-2-yl)amino)-3-((trifluoromethyl)sulfonyl)phenyl)sulfonyl)benzamide (GT-04152)

According to the method of step 2 of Scheme I-1, the target compound (GT-04152) was prepared (white solid, 44 mg, yield 70.39%). ¹H NMR (400 MHz, DMSO) δ 12.17 (s, 1H), 10.90 (s, 1H), 10.57 (s, 1H), 8.18 (d, *J =* 2.0 Hz, 1H), 7.99 (d, *J =* 9.2 Hz, 1H), 7.78 (d, *J =* 8.8 Hz, 2H), 7.40 (t, *J =* 9.2 Hz, 4H), 7.29 (d, *J =* 7.4 Hz, 2H), 7.25 - 7.13 (m, 6H), 7.03 - 6.92 (m, 3H), 4.20 (s, 1H), 4.09 (dd, *J =* 12.3, 4.7 Hz, 1H), 3.88 (d, *J =* 12.1 Hz, 2H), 3.50 (d, *J =* 49.5 Hz, 6H), 3.37 - 3.04 (m, 13H), 2.76 (dd, *J =* 14.2, 10.0 Hz, 3H), 2.55 (d, *J =* 15.2 Hz, 1H), 2.34 - 2.04 (m, 8H), 2.04 - 1.96 (m, 1H), 1.47 (t, *J =* 6.1 Hz, 2H), 1.00 (s, 6H). LCMS (ESI) calcd for C₅₉H₆₇ClF₄N₇O₇S₃⁺ [M+H]⁺: 1192.38; found, 1192.40.

### Example 80: Preparation of 3-(4-(((3R,4S)-4-(4-amino-3-(4-phenoxyphenyl)-1H-pyrazolo[3,4-d]pyrimidin-1-yl)-3-fluoropiperidin-1-yl)methyl)phenyl)piperidine-2,6-dione (GT-03542)

According to the method of step 2 of Scheme I-1, the target compound (GT-03542) was prepared (white solid, 39 mg, yield 61.4%). ¹H NMR (400 MHz, MeOD) δ 8.34 (s, 1H), 7.58 (d, *J =* 8.4 Hz, 2H), 7.49 (d, *J* = 7.9 Hz, 2H), 7.41 - 7.30 (m, 4H), 7.15 - 6.97 (m, 5H), 5.29 (t, *J =* 33.5 Hz, 2H), 4.53 - 4.32 (m, 2H), 3.95 - 3.68 (m, 3H), 3.57 (dd, *J =* 38.0, 14.4 Hz, 1H), 3.38 (s, 1H), 3.03 (s, 1H), 2.76 - 2.52 (m, 2H), 2.39 (d, *J* = 12.9 Hz, 1H), 2.27 - 2.07 (m, 2H). LCMS (ESI) calcd for C₃₄H₃₃FN₇O₃⁺ [M+H]⁺: 606.26; found, 606.3.

### Example 81: Preparation of 3-(4-(((3R,4S)-4-(4-amino-3-(4-phenoxyphenyl)-1H-pyrazolo[3,4-d]pyrimidin-1-yl)-3-fluoropiperidin-1-yl)methyl)-2-fluorophenyl)piperidine-2,6-dione (GT-03513)

According to the method of step 2 of Scheme I-1, the target compound was prepared (GT-03513) (white solid, 42 mg, yield 51.3%). ¹H NMR (400 MHz, MeOD) δ 8.33 (s, 1H), 7.98 - 7.87 (m, 1H), 7.58 (d, *J =* 8.6 Hz, 2H), 7.41 - 7.30 (m, 5H), 7.07 (d, *J =* 8.6 Hz, 2H), 7.03 - 6.97 (m, 2H), 5.34 (d, *J =* 45.8 Hz, 2H), 4.42 (dd, *J =* 26.5, 13.1 Hz, 2H), 4.05 (dd, *J =* 12.7, 4.9 Hz, 1H), 3.86 - 3.70 (m, 2H), 3.59 (dd, *J =* 36.5, 13.8 Hz, 1H), 3.47 - 3.34 (m, 1H), 3.20 - 3.15 (m, 1H), 2.79 - 2.54 (m, 2H), 2.39 (d, *J =* 12.3 Hz, 1H), 2.25 (dt, *J =* 13.0, 8.1 Hz, 1H), 2.08 (dd, *J =* 9.0, 3.9 Hz, 1H). LCMS (ESI) calcd for C₃₄H₃₂F₂N₇O₃⁺ [M+H]⁺: 624.25; found, 624.3.

### Example 82: Preparation of N-(3-(difluoromethyl)-1-(1-(3-(2,4-dioxotetrahydropyrimidin-1(2H)-yl)benzyl)piperidin-4-yl)-1H-pyrazol-4-yl)-5-morpholinopyrazolo[1,5-a]pyrimidine-3-carboxamide (GT-04177)

According to the method of step 2 of Scheme I-1, the target compound (GT-04177) was prepared (white solid, 30 mg, yield 50.58%). ¹H NMR (400 MHz, DMSO) δ 11.38 (s, 1H), 10.41 (d, *J =* 16.2 Hz, 1H), 9.41 (s, 1H), 8.82 (d, *J =* 7.9 Hz, 1H), 8.41 (s, 1H), 8.28 (s, 1H), 7.60 (d, *J =* 30.4 Hz, 1H), 7.43 (d, *J =* 20.7 Hz, 3H), 7.30 - 6.97 (m, 1H), 6.91 (d, *J =* 7.9 Hz, 1H), 4.41 (d, *J =* 73.4 Hz, 2H), 3.87 (t, *J =* 6.4 Hz, 2H), 3.75 (d, *J =* 28.4 Hz, 8H), 3.60 - 3.37 (m, 3H), 3.11 (dd, *J =* 7.4, 4.1 Hz, 2H), 2.73 (t, *J =* 6.4 Hz, 2H), 2.31 (d, *J* = 67.3 Hz, 4H). LCMS (ESI) calcd for C₃₁H₃₅F₂N₁₀O₄⁺ [M+H]⁺: 649.27; found, 649.30.

### Example 83: Preparation of 2-((2-((4-(4-((4-(2,6-dioxopiperidin-3-yl)benzyl)(methyl)amino)piperidin-1-yl)-2-isopropoxy-5-methylphenyl)amino)-5-(trifluoromethyl)pyridin-4-yl)amino)-N-methylbenzamide (GT-04094)

According to the method of step 2 of Scheme I-1, the target compound (GT-04094) was prepared (yellow solid, 30 mg, yield 41.33%). ¹H NMR (400 MHz, MeOD) δ 7.94 (s, 1H), 7.68 - 7.58 (m, 1H), 7.53 - 7.43 (m, 4H), 7.33 (d, *J =* 8.2 Hz, 2H), 7.30 - 7.20 (m, 1H), 7.00 (s, 1H), 6.71 (s, 1H), 6.42 (s, 1H), 4.50 (td, *J =* 12.0, 6.0 Hz, 2H), 4.17 (d, *J=* 13.1 Hz, 1H), 3.87 (dd, *J =* 11.5, 5.1 Hz, 1H), 3.49 - 3.33 (m, 1H), 3.20 (s, 2H), 2.79 (s, 3H), 2.75 - 2.53 (m, 7H), 2.26 - 2.07 (m, 7H), 2.00 (t, *J =* 11.6 Hz, 2H), 1.14 (d, *J =* 6.0 Hz, 6H). LCMS (ESI) calcd for C₄₂H₄₉F₃N₇O₄⁺[M+H]⁺: 772.38; found, 772.4.

### Example 84: Preparation of 2-((2-((4-(4-((4-(2,6-dioxopiperidin-3-yl)-3-fluorobenzyl)(methyl)amino)piperidin-1-yl)-2-isopropoxy-5-methylphenyl)amino)-5-(trifluoromethyl)pyridin-4-yl)amino)-N-methylbenzamide (GT-04095)

According to the method of step 2 of Scheme I-1, the target compound (GT-04095) was prepared (yellow solid, 30 mg, yield 40.43%). 1H NMR (400 MHz, MeOD) δ 7.94 (s, 1H), 7.67 - 7.59 (m, 1H), 7.54 - 7.44 (m, 2H), 7.36 (ddd, *J =* 10.8, 7.5, 4.9 Hz, 3H), 7.25 (ddd, *J =* 15.3, 8.7, 5.7 Hz, 1H), 7.02 (d, *J =* 10.3 Hz, 1H), 6.71 (s, 1H), 6.42 (s, 1H), 4.59 - 4.46 (m, 2H), 4.20 (d, *J =* 11.2 Hz, 1H), 4.04 (dd, *J =* 12.6, 5.1 Hz, 1H), 3.48 - 3.36 (m, 1H), 3.20 (s, 2H), 2.79 - 2.60 (m, 10H), 2.32 - 2.15 (m, 6H), 2.11 - 1.94 (m, 3H), 1.14 (d, *J =* 6.0 Hz, 6H). LCMS (ESI) calcd for C₄₂H₄₈F₄N₇O₄⁺ [M+H]⁺: 790.37; found, 790.4.

### Example 85: Preparation of N-(3-(difluoromethyl)-1-(1-(4-(2,6-dioxopiperidin-3-yl)-3-fluorobenzyl)piperidin-4-yl)-1H-pyrazol-4-yl)-2-(2-((2,2,2-trifluoroethyl)amino)pyridin-4-yl)oxazole-4-carboxamide (GT-04087)

According to the method of step 2 of Scheme I-1, the target compound (GT-04087) was prepared (white solid, 25 mg, yield 38.89%). ¹H NMR (400 MHz, MeOD) δ 8.64 (s, 1H), 8.24 (d, *J =* 26.9 Hz, 1H), 8.08 (d, *J* = 6.0 Hz, 1H), 7.46 (s, 1H), 7.39 (dd, *J =* 12.2, 6.1 Hz, 2H), 7.34 - 7.27 (m, 2H), 6.85 (dd, *J =* 63.5, 45.4 Hz, 1H), 4.58 - 4.45 (m, 1H), 4.35 (d, *J =* 16.5 Hz, 2H), 4.18 (q, *J =* 9.1 Hz, 2H), 4.04 (dd, *J =* 12.6, 4.9 Hz, 1H), 3.60 (d, *J =* 12.3 Hz, 2H), 3.40 (d, *J =* 8.4 Hz, 1H), 3.04 (s, 1H), 2.68 (tdd, *J =* 16.2, 12.8, 4.2 Hz, 2H), 2.25 (ddd, *J =* 17.5, 14.5, 5.7 Hz, 5H), 2.13 - 1.99 (m, 1H). LCMS (ESI) calcd for C₃₂H₃₁F₆N₈O₄⁺ [M+H]⁺: 705.24; found, 705.3.

### Example 86: Preparation of N-(3-(difluoromethyl)-1-(1-(4-(2,6-dioxopiperidin-3-yl)benzyl)piperidin-4-yl)-1H-pyrazol-4-yl)-2-(2-((2,2,2-trifluoroethyl)amino)pyridin-4-yl)oxazole-4-carboxamide (GT-04121)

According to the method of step 2 of Scheme I-1, the target compound (GT-04121) was prepared (white solid, 25 mg, yield 39.86%). ¹H NMR (400 MHz, MeOD) δ 8.70 (d, *J =* 6.8 Hz, 1H), 8.24 (d, *J =* 27.1 Hz, 1H), 8.07 (d, *J =* 6.5 Hz, 1H), 7.65 (s, 1H), 7.49 (dd, *J =* 11.2, 8.0 Hz, 3H), 7.33 (d, *J =* 8.0 Hz, 2H), 6.86 (td, *J =* 54.3, 16.6 Hz, 1H), 4.59 - 4.45 (m, 1H), 4.36 - 4.19 (m, 4H), 3.87 (dd, *J =* 11.6, 5.1 Hz, 1H), 3.59 (d, *J=* 12.8 Hz, 2H), 3.40 (s, 1H), 3.16 (d, *J =* 12.8 Hz, 1H), 2.67 (ddd, *J =* 17.1, 11.5, 5.5 Hz, 1H), 2.55 (dd, *J =* 26.5, 22.2 Hz, 1H), 2.35 - 2.05 (m, 6H). LCMS (ESI) calcd for C₃₂H₃₂F₅N₈O₄⁺ [M+H]⁺: 687.25; found, 687.3.

### Example 87: Preparation of (R)-4-(4-((4'-chloro-4,4-dimethyl-3,4,5,6-tetrahydro-[1,1'-biphenyl]-2-yl)methyl)piperazin-1-yl)-N-((4-((4-(4-(3-(2,4-dioxotetrahydropyrimidin-1(2H)-yl)benzyl)piperazin-1-yl)-1-(phenylthio)butan-2-yl)amino)-3-((trifluoromethyl)sulfonyl)phenyl)sulfonyl)benzamide (GT-04278)

According to the method of step 2 of Scheme I-1, the target compound (GT-04278) was prepared (white solid, 187 mg, yield 75.24%). ¹H NMR (400 MHz, MeOD) δ 8.29 (d, *J =* 2.1 Hz, 1H), 8.07 (dd, *J =* 9.3, 2.2 Hz, 1H), 7.76 (d, *J =* 9.0 Hz, 2H), 7.58 (s, 1H), 7.56 - 7.43 (m, 3H), 7.40 (d, *J =* 8.4 Hz, 2H), 7.32 (d, *J* = 7.3 Hz, 2H), 7.16 (dt, *J =* 14.6, 6.7 Hz, 5H), 6.98 (dd, *J =* 9.1, 6.9 Hz, 3H), 4.35 (s, 2H), 4.13 (d, *J* = 5.3 Hz, 1H), 3.91 (dd, *J =* 15.7, 9.0 Hz, 4H), 3.70 (s, 3H), 3.62 - 3.30 (m, 20H), 3.22 (dd, *J =* 14.4, 6.6 Hz, 7H), 2.83 (dd, *J =* 18.4, 11.7 Hz, 5H), 2.40 (s, 2H), 2.25 (s, 1H), 2.09 (s, 3H), 1.57 (t, *J =* 6.4 Hz, 2H), 1.26 (d, *J* = 24.1 Hz, 1H), 1.08 (s, 6H). LCMS (ESI) calcd for C₅sH₆₆ClF₃NsO₇S₃⁺ [M+H]⁺: 1175.39; found, 1175.4.

### Example 88: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(4-(3-((2,6-dioxopiperidin-3-yl)amino)benzyl)piperazin-1-yl)-1-(phenylthio)butan-2-yl)amino)-3-((trifluoromethyl)sulfonyl)phenyl)sulfonyl)benzamide (GT-04279)

According to the method of step 2 of Scheme I-1, the target compound (GT-04279) was prepared (white solid, 176 mg, yield 70.01%). ¹H NMR (400 MHz, MeOD) δ 8.31 (d, *J* = 1.9 Hz, 1H), 8.07 (dd, *J =* 9.3, 2.2 Hz, 1H), 7.76 (d, *J* = 9.0 Hz, 2H), 7.40 (d, *J* = 8.4 Hz, 2H), 7.32 (d, *J* = 7.4 Hz, 2H), 7.28 - 7.09 (m, 7H), 7.04 (s, 1H), 6.99 (d, *J* = 9.1 Hz, 3H), 6.87 (dd, *J* = 18.1, 7.8 Hz, 2H), 4.44 (dd, *J* = 12.1, 4.9 Hz, 1H), 4.34 (s, 2H), 4.16 (s, 1H), 3.90 (d, *J* = 14.0 Hz, 2H), 3.71 (s, 3H), 3.53 (d, *J* = 12.5 Hz, 9H), 3.38 (dd, *J =* 14.4, 4.8 Hz, 2H), 3.27 - 3.13 (m, 5H), 2.89 (td, *J* = 12.5, 6.6 Hz, 3H), 2.76 - 2.66 (m, 1H), 2.50 - 2.25 (m, 4H), 2.23 - 2.07 (m, 3H), 1.97 (dd, *J* = 12.7, 4.5 Hz, 1H), 1.58 (t, *J* = 6.4 Hz, 2H), 1.30 (s, 1H), 1.08 (s, 6H). LCMS (ESI) calcd for C₅₉H₆₈ClF₃N₈O₇S₃⁺ [M+H]⁺: 1190.40; found, 1190.4.

### Example 89: Preparation of 3-(2-fluoro-4-((2-(6-methyl-7-oxo-6,7-dihydro-1H-pyrrolo[2,3-c]pyridin-4-yl)-4-(methylsulfonyl)phenoxy)methyl)phenyl)piperidine-2,6-dione (GT-04640)

According to the method of step 2 of Scheme I-1, the target compound (GT-04640) was prepared (white solid, 18 mg, yield 21.33%). LCMS (ESI) calcd for C₂₇H₂₅FN₃O₆S⁺ [M+H]⁺: 538.14; found, 538.2.

### Example 90: Preparation of 3-((3-((2-(6-methyl-7-oxo-6,7-dihydro-1H-pyrrolo[2,3-c]pyridin-4-yl)-4-(methylsulfonyl)phenoxy)methyl)phenyl)amino)piperidine-2,6-dione (GT-04641)

According to the method of step 2 of Scheme I-1, the target compound (GT-04641) was prepared (gray solid, 32 mg, yield 38.11%). ¹H NMR (400 MHz, DMSO) δ 12.05 (s, 1H), 10.78 (s, 1H), 7.88 (d, J = 7.2 Hz, 2H), 7.45 - 7.33 (m, 2H), 7.28 (t, J = 2.7 Hz, 1H), 7.03 (t, J = 7.7 Hz, 1H), 6.64 (s, 1H), 6.57 (dd, J = 17.9, 7.7 Hz, 2H), 6.17 (s, 1H), 5.12 (s, 2H), 4.22 (dd, J = 11.3, 4.8 Hz, 1H), 3.53 (s, 3H), 3.21 (s, 3H), 2.74 - 2.66 (m, 1H), 2.58 (d, J = 4.1 Hz, 1H), 2.05 (dd, J = 12.9, 4.3 Hz, 1H), 1.88 - 1.80 (m, 1H). LCMS (ESI) calcd for C₂₇H₂₇N₄O₆S⁺ [M+H]⁺: 535.17; found, 535.2.

### Example 91: Preparation of 1-(2-fluoro-4-((2-(6-methyl-7-oxo-6,7-dihydro-1H-pyrrolo[2,3-c]pyridin-4-yl)-4-(methylsulfonyl)phenoxy)methyl)phenyl)dihydropyrimidine-2,4(1H,3H)-dione (GT-04643)

According to the method of step 2 of Scheme I-1, the target compound (GT-04643) was prepared (white solid, 50 mg, yield 73.89%). ¹H NMR (400 MHz, DMSO) δ 12.07 (s, 1H), 10.47 (s, 1H), 7.92 (s, 1H), 7.88 (d, J = 2.4 Hz, 1H), 7.45 (d, J = 8.8 Hz, 1H), 7.38 (d, J = 9.4 Hz, 2H), 7.30 (s, 1H), 7.18 (d, J = 7.3 Hz, 2H), 6.18 (s, 1H), 5.27 (s, 2H), 3.68 (t, J = 6.7 Hz, 2H), 3.55 (s, 3H), 3.23 (s, 3H), 2.69 (t, J = 6.7 Hz, 2H). LCMS (ESI) calcd for C₂₆H₂₄FN₄O₆S⁺ [M+H]⁺: 539.14; found, 539.2.

### Example 92: Preparation of 1-(3-fluoro-4-((2-(6-methyl-7-oxo-6,7-dihydro-1H-pyrrolo[2,3-c]pyridin-4-yl)-4-(methylsulfonyl)phenoxy)methyl)phenyl)dihydropyrimidine-2,4(1H,3H)-dione (GT-04644)

According to the method of step 2 of Scheme I-1, the target compound (GT-04644) was prepared (white solid, 41 mg, yield 60.59%). ¹H NMR (400 MHz, DMSO) δ 12.03 (s, 1H), 10.45 (s, 1H), 7.92 (dd, J = 8.7, 2.4 Hz, 1H), 7.88 (d, J = 2.4 Hz, 1H), 7.53 (d, J = 8.8 Hz, 1H), 7.35 (d, J = 8.9 Hz, 2H), 7.26 (ddd, J = 6.3, 4.1, 2.1 Hz, 2H), 7.11 (dd, J = 8.3, 1.9 Hz, 1H), 6.22 - 6.08 (m, 1H), 5.28 (s, 2H), 3.78 (t, J = 6.6 Hz, 2H), 3.53 (s, 3H), 3.23 (s, 3H), 2.69 (t, J = 6.6 Hz, 2H). LCMS (ESI) calcd for C₂₆H₂₄FN₄O₆S⁺ [M+H]⁺: 539.15; found, 539.2.

### Example 93: Preparation of 1-(4-((2-(6-methyl-7-oxo-6,7-dihydro-1H-pyrrolo[2,3-c]pyridin-4-yl)-4-(methylsulfonyl)phenoxy)methyl)phenyl)dihydropyrimidine-2,4(1H,3H)-dione (GT-04645)

According to the method of step 2 of Scheme I-1, the target compound (GT-04645) was prepared (white solid, 30 mg, yield 45.86%). ¹H NMR (400 MHz, DMSO) δ 12.06 (s, 1H), 10.36 (s, 1H), 7.92 (dd, J = 8.7, 2.4 Hz, 1H), 7.88 (t, J = 3.6 Hz, 1H), 7.47 (d, J = 8.8 Hz, 1H), 7.38 (s, 1H), 7.34 (d, J = 8.6 Hz, 2H), 7.32 - 7.24 (m, 3H), 6.23 - 6.15 (m, 1H), 5.25 (s, 2H), 3.76 (t, J = 6.6 Hz, 2H), 3.55 (s, 3H), 3.23 (s, 3H), 2.69 (t, J = 6.6 Hz, 2H). LCMS (ESI) calcd for C₂₆H₂₅N₄O₆S⁺ [M+H]⁺: 521.14; found, 521.2.

### Example 94: Preparation of 3-(4-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)phenyl)piperidine-2,6-dione (GT-06735)

According to the method of step 2 of Scheme I-1, the target compound (GT-06735) was prepared (white solid, 20 mg, yield 31.56%). ¹H NMR (400 MHz, DMSO-d6) δ 11.69 (s, 1H), 11.07 (s, 1H), 10.87 (s, 1H), 8.86 (d, J = 3.4 Hz, 1H), 8.29 (s, 1H), 8.20 (d, J = 10.3 Hz, 1H), 7.96 (d, J = 2.3 Hz, 1H), 7.79 (dd, J = 15.3, 10.7 Hz, 2H), 7.64 (d, J = 8.2 Hz, 2H), 4.91 (dt, J = 13.8, 6.8 Hz, 1H), 4.49 (d, J = 10.2 Hz, 1H), 4.20 - 4.13 (m, 1H), 3.91 (d, J = 4.9 Hz, 2H), 3.44 (d, J = 7.0 Hz, 2H), 2.83 (dd, J = 22.3, 10.8 Hz, 2H), 2.73 (s, 3H), 2.70 - 2.65 (m, 1H), 2.59 (d, J = 4.8 Hz, 3H), 2.53 (s, 1H), 2.30 (ddd, J = 25.2, 19.8, 9.9 Hz, 3H), 2.01 (ddd, J = 39.4, 18.1, 9.3 Hz, 3H), 1.64 (d, J = 6.9 Hz, 6H). LCMS (ESI) calcd for C₃₈H₄₂F₂N₉O₂⁺ [M+H]⁺: 694.34; found, 694.4.

### Example 95: Preparation of 1-(4-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)phenyl)dihydropyrimidine-2,4(1H,3H)-dione (GT-06838)

According to the method of step 2 of Scheme I-1, the target compound (GT-06838) was prepared (white solid, 18 mg, yield 36.46%). ¹H NMR (500 MHz, DMSO) δ 11.84 (s, 1H), 11.31 (s, 1H), 10.46 (s, 1H), 8.87 (d, J = 3.2 Hz, 1H), 8.32 (s, 1H), 8.25 (d, J = 7.7 Hz, 1H), 7.97 (s, 1H), 7.82 (dd, J = 16.9, 8.2 Hz, 2H), 7.71 (d, J = 8.4 Hz, 2H), 7.42 (d, J = 8.5 Hz, 2H), 4.92 (dd, J = 13.8, 6.9 Hz, 1H), 4.50 (d, J = 3.1 Hz, 1H), 4.21 (dd, J = 13.0, 7.0 Hz, 1H), 3.89 (s, 3H), 3.44 (d, J = 12.0 Hz, 2H), 2.83 (dd, J = 25.1, 12.4 Hz, 2H), 2.76 (s, 3H), 2.72 (t, J = 6.6 Hz, 2H), 2.59 (d, J = 4.9 Hz, 3H), 2.39 (d, J = 10.7 Hz, 1H), 2.29 (d, J = 10.6 Hz, 1H), 1.96 (dd, J = 23.6, 11.9 Hz, 2H), 1.64 (d, J = 6.9 Hz, 6H). LCMS (ESI) calcd for C₃₇H₄₁F₂N₁₀O₂⁺ [M+H]⁺: 695.34; found, 695.3.

### Example 96: Preparation of 8-(4-(4-(4-(2,4-dioxotetrahydropyrimidin-1(2H)-yl)benzyl)piperazin-1-yl)piperidin-1-yl)-9-ethyl-6,6-dimethyl-11-oxo-6,11-dihydro-5H-benzo[b]carbazole-3-carbonitrile (GT-07307)

According to the method of step 2 of Scheme I-1, the target compound (GT-07307) was prepared (white solid, 28 mg, yield 51 %). ¹H NMR (400 MHz, DMSO-d6) δ 12.82 (s, 1H), 11.88 (s, 1H), 10.35 (s, 1H), 8.27 (d, J = 17.4, 8.3 Hz, 1H), 7.99 (d, J = 17.2 Hz, 2H), 7.67 - 7.52 (m, 3H), 7.42 - 7.34 (m, 2H), 7.29 (s, 1H), 4.27 (s, 1H), 3.76 (t, J = 6.6 Hz, 2H), 3.72 - 3.61 (m, 2H), 3.54 - 3.40 (m, 5H), 3.25 (d, J = 11.3 Hz, 3H), 2.76 (t, J = 11.6 Hz, 2H), 2.69 - 2.61 (m, 4H), 2.14 (s, 2H), 1.91 - 1.78 (m, 2H), 1.70 (s, 6H), 1.22 (t, J = 7.5 Hz, 3H). LCMS (ESI) calcd for C₄₁H₄₆N₇O₃ ⁺ [M+H]⁺: 684.37; found, 684.4.

### Example 97: Preparation of 8-(4-(4-(3-(2,4-dioxotetrahydropyrimidin-1(2H)-yl)benzyl)piperazin-1-yl)piperidin-1-yl)-9-ethyl-6,6-dimethyl-11-oxo-6,11-dihydro-5H-benzo[b]carbazole-3-carbonitrile (GT-07310)

According to the method of step 2 of Scheme I-1, the target compound (GT-07310) was prepared (white solid, 22 mg, yield 40 %). ¹H NMR (400 MHz, DMSO-d6) δ 12.96 (s, 1H), 12.05 (s, 1H), 10.49 (d, *J* = 23.5 Hz, 1H), 8.39 (d, *J* = 8.7 Hz, 1H), 8.12 (s, 1H), 8.07 (s, 1H), 7.74 - 7.63 (m, 2H), 7.54 (s, 3H), 7.42 (s, 1H), 4.50 (d, *J* = 47.2 Hz, 2H), 3.94 (t, *J* = 6.5 Hz, 2H), 3.85 (d, *J* = 6.7 Hz, 2H), 3.57 (s, 8H), 3.38 (d, *J =* 11.1 Hz, 2H), 2.89 (t, *J* = 11.7 Hz, 2H), 2.83 - 2.75 (m, 4H), 2.27 (s, 2H), 2.04 - 1.91 (m, 2H), 1.82 (s, 6H), 1.34 (t, *J =* 7.5 Hz, 3H). LCMS (ESI) calcd for C₄₁H₄₆N₇O₃ ⁺ [M+H]⁺: 684.37; found, 684.4.

### Example 98: Preparation of 8-(4-(4-((5-(2,4-dioxotetrahydropyrimidin-1(2H)-yl)pyridin-2-yl)methyl)piperazin-1-yl)piperidin-1-yl)-9-ethyl-6,6-dimethyl-11-oxo-6,11-dihydro-5H-benzo[b]carbazole-3-carbonitrile (GT-07311)

According to the method of step 2 of Scheme I-1, the target compound (GT-07311) was prepared (white solid, 16 mg, yield 29 %). ¹H NMR (400 MHz, DMSO-d6) δ 13.09 (s, 1H), 12.29 (s, 1H), 10.61 (s, 1H), 8.72 (d, *J* = 2.4 Hz, 1H), 8.32 (d, *J* = 8.2 Hz, 1H), 8.07 (s, 1H), 8.01 (s, 1H), 7.96 (dd, *J* = 8.4, 2.5 Hz, 1H), 7.80 (d, *J* = 8.5 Hz, 1H), 7.60 (d, *J* = 8.3 Hz, 1H), 7.38 (s, 1H), 4.58 (s, 2H), 3.90 (t, *J* = 6.6 Hz, 2H), 3.81 (s, 2H), 3.76 - 3.60 (m, 6H), 3.51 (s, 1H), 3.34 (d, *J* = 10.8 Hz, 2H), 2.86 (t, *J* = 11.5 Hz, 2H), 2.80 - 2.70 (m, 4H), 2.26 (d, *J* = 9.8 Hz, 2H), 2.02 - 1.90 (m, 2H), 1.78 (s, 6H), 1.29 (t, *J* = 7.5 Hz, 3H). LCMS (ESI) calcd for C₄₀H₄₅N₈O₃⁺ [M+H]⁺: 685.36; found, 685.5.

### Example 99: Preparation of 8-(4-(4-((6-(2,4-dioxotetrahydropyrimidin-1(2H)-yl)pyridin-3-yl)methyl)piperazin-1-yl)piperidin-1-yl)-9-ethyl-6,6-dimethyl-11-oxo-6,11-dihydro-5H-benzo[b]carbazole-3-carbonitrile (GT-07312)

According to the method of step 2 of Scheme I-1, the target compound (GT-07312) was prepared (white solid, 16 mg, yield 29 %). ¹H NMR (400 MHz, DMSO-d6) δ 12.98 (s, 1H), 12.31 (s, 1H), 10.63 (s, 1H), 8.68 (d, *J* = 2.0 Hz, 1H), 8.32 (d, *J* = 8.2 Hz, 1H), 8.18 - 8.11 (m, 1H), 8.06 (s, 1H), 8.01 (s, 1H), 7.88 (d, *J* = 8.6 Hz, 1H), 7.61 (d, *J* = 8.2, 1.3 Hz, 1H), 7.37 (s, 1H), 4.49 (s, 1H), 4.11 (t, *J* = 6.6 Hz, 2H), 3.83 - 3.76 (m, 3H), 3.74 - 3.54 (m, 6H), 3.48 (s, 1H), 3.33 (d, *J* = 11.0 Hz, 2H), 2.85 (t, *J* = 11.5 Hz, 2H), 2.79 - 2.66 (m, 4H), 2.24 (d, *J* = 8.5 Hz, 2H), 2.02 - 1.89 (m, 2H), 1.80 (s, 6H), 1.29 (t, *J* = 7.5 Hz, 3H). LCMS (ESI) calcd for C₄₀H₄₅N₈O₃⁺ [M+H]⁺: 685.36; found, 685.4.

### Example 100: Preparation of 8-(4-(4-(3-((2,6-dioxopiperidin-3-yl)amino)benzyl)piperazin-1-yl)piperidin-1-yl)-9-ethyl-6,6-dimethyl-11-oxo-6,11-dihydro-5H-benzo[b]carbazole-3-carbonitrile (GT-07313)

According to the method of step 2 of Scheme I-1, the target compound (GT-07313) was prepared (white solid, 28 mg, yield 51 %). ¹H NMR (400 MHz, DMSO-d6) δ 12.93 (s, 1H), 12.03 (s, 1H), 10.92 (s, 1H), 8.38 (d, *J =* 8.2 Hz, 1H), 8.12 (s, 1H), 8.07 (s, 1H), 7.67 (dd, *J =* 8.2, 1.3 Hz, 1H), 7.42 (s, 1H), 7.23 (t, J = 7.8 Hz, 1H), 7.06 (s, 1H), 6.87 (t, *J* = 7.5 Hz, 2H), 4.47 (dd, *J* = 11.7, 4.6 Hz, 1H), 4.34 (s, 1H), 3.84 (s, 2H), 3.79 - 3.63 (m, 7H), 3.38 (d, *J* = 11.3 Hz, 3H), 2.95 - 2.82 (m, 3H), 2.77 (q, *J* = 7.5 Hz, 2H), 2.71 - 2.61 (m, 1H), 2.36 - 2.18 (m, 3H), 2.04 - 1.89 (m, 3H), 1.82 (s, 6H), 1.35 (t, *J* = 7.5 Hz, 3H). LCMS (ESI) calcd for C₄₂H₄₈N₇O₃⁺ [M+H]⁺: 698.38; found, 698.5 .

### Example 101: Preparation of 8-(4-(4-(2,6-dioxopiperidin-3-yl)benzyl)piperazin-1-yl)-9-ethyl-6,6-dimethyl-11-oxo-6,11-dihydro-5H-benzo[b]carbazole-3-carbonitrile (GT-07323)

According to the method of step 2 of Scheme I-1, the target compound (GT-07323) was prepared (white solid, 7 mg, yield 25 %). ¹H NMR (400 MHz, DMSO-d6) δ 12.81 (s, 1H), 10.82 (s, 2H), 8.25 (d, *J =* 8.2 Hz, 1H), 8.02 (s, 1H), 7.94 (s, 1H), 7.62 - 7.49 (m, 3H), 7.29 (d, *J =* 5.2 Hz, 3H), 7.05 (q, *J =* 8.1 Hz, 2H), 4.37 (d, *J* = 4.3 Hz, 2H), 3.87 (dd, *J* = 11.6, 4.9 Hz, 1H), 3.33 (s, 2H), 3.24 - 3.11 (m, 5H), 2.71 - 2.60 (m, 3H), 2.50 - 2.45 (m, 1H), 2.23 - 2.16 (m, 2H), 2.02 - 1.97 (m, 1H), 1.70 (s, 6H), 1.22 (t, *J* = 7.5 Hz, 3H). LCMS (ESI) calcd for C₃₇H₃₈N₅O₃ ⁺ [M+H]⁺: 600.30; found, 600.3.

### Example 102: Preparation of 8-(4-(4-(2,4-dioxotetrahydropyrimidin-1(2H)-yl)benzyl)piperazin-1-yl)-9-ethyl-6,6-dimethyl-11-oxo-6,11-dihydro-5H-benzo[b]carbazole-3-carbonitrile (GT-07324)

According to the method of step 2 of Scheme I-1, the target compound (GT-07324) was prepared (white solid, 10 mg, yield 34 %). ¹H NMR (400 MHz, DMSO-d6) δ 12.81 (s, 1H), 10.93 (s, 1H), 10.37 (s, 1H), 8.23 (d, *J* = 8.2 Hz, 1H), 8.00 (s, 1H), 7.93 (s, 1H), 7.61 (d, *J* = 8.4 Hz, 1H), 7.53 (dd, *J* = 8.2, 1.3 Hz, 1H), 7.37 (d, 2H), 7.28 (s, 1H), 4.43 - 4.30 (m, 2H), 3.81 - 3.71 (m, 2H), 3.31 (s, 4H), 3.23 - 3.16 (m, 4H), 2.68 - 2.60 (m, 4H), 1.69 (s, 6H), 1.20 (t, *J* = 7.5 Hz, 3H). LCMS (ESI) calcd for C₃₆H₃₇N₆O₃⁺ [M+H]⁺: 601.29; found, 601.4.

### Example 103: Preparation of 8-(4-(3-(2,4-dioxotetrahydropyrimidin-1(2H)-yl)benzyl)piperazin-1-yl)-9-ethyl-6,6-dimethyl-11-oxo-6,11-dihydro-5H-benzo[b]carbazole-3-carbonitrile (GT-07325)

According to the method of step 2 of Scheme I-1, the target compound (GT-07325) was prepared (white solid, 9 mg, yield 30.7 %). ¹H NMR (400 MHz, DMSO-d6) δ 12.81 (s, 1H), 10.91 (s, 1H), 10.40 (s, 1H), 8.25 (d, *J* = 8.2 Hz, 1H), 8.02 (s, 1H), 7.94 (s, 1H), 7.60 (s, 1H), 7.54 (d, *J* = 8.2 Hz, 1H), 7.43 (d, *J =* 5.9 Hz, 2H), 7.30 (s, 1H), 4.39 (d, *J =* 3.8 Hz, 1H), 3.82 (t, *J =* 6.5 Hz, 2H), 3.43 - 3.32 (m, 2H), 3.27 - 3.17 (m, 7H), 2.72 - 2.60 (m, 4H), 1.70 (s, 6H), 1.22 (t, *J* = 7.5 Hz, 3H). LCMS (ESI) calcd for C₃₆H₃₇N₆O₃⁺ [M+H]⁺: 601.29; found, 601.3.

### Example 104: Preparation of 8-(4-((5-(2,4-dioxotetrahydropyrimidin-1(2H)-yl)pyridin-2-yl)methyl)piperazin-1-yl)-9-ethyl-6,6-dimethyl-11-oxo-6,11-dihydro-5H-benzo[b]carbazole-3-carbonitrile (GT-07326)

According to the method of step 2 of Scheme I-1, the target compound (GT-07326) was prepared (white solid, 18 mg, yield 44 %). ¹H NMR (400 MHz, DMSO-d6) δ 12.86 (s, 1H), 10.83 (s, 1H), 10.51 (s, 1H), 9.15 (s, 1H), 8.65 (d, *J* = 2.5 Hz, 1H), 8.23 (d, *J* = 8.2 Hz, 1H), 8.00 (s, 1H), 7.93 (s, 1H), 7.87 - 7.82 (m, 1H), 7.67 (d, *J* = 8.4 Hz, 1H), 7.53 (dd, *J* = 8.1, 1.4 Hz, 1H), 7.30 (s, 1H), 4.53 (s, 1H), 3.81 (t, *J* = 6.6 Hz, 1H), 3.38 (s, 4H), 3.23 - 3.10 (m, 6H), 2.70 - 2.63 (m, 4H), 1.70 (s, 6H), 1.20 (t, *J* = 7.5 Hz, 3H). LCMS (ESI) calcd for C₃₅H₃₆N₇O₃⁺ [M+H]⁺: 602.29; found, 602.3.

### Example 105: Preparation of 8-(4-((6-(2,4-dioxotetrahydropyrimidin-1(2H)-yl)pyridin-3-yl)methyl)piperazin-1-yl)-9-ethyl-6,6-dimethyl-11-oxo-6,11-dihydro-5H-benzo[b]carbazole-3-carbonitrile (GT-07327)

According to the method of step 2 of Scheme I-1, the target compound (GT-07327) was prepared (white solid, 8 mg, yield 27.2 %). ¹H NMR (400 MHz, DMSO-d6) δ 12.80 (s, 1H), 11.18 (s, 1H), 10.54 (s, 1H), 9.06 (s, 1H), 8.57 (s, 1H), 8.23 (d, *J =* 8.2 Hz, 1H), 8.05 (d, *J =* 8.8 Hz, 1H), 8.00 (s, 1H), 7.93 (s, 1H), 7.81 (d, *J* = 8.6 Hz, 1H), 7.53 (d, *J* = 8.2, 1.2 Hz, 1H), 7.29 (d, *J* = 5.0 Hz, 1H), 4.39 (s, 2H), 4.03 (t, *J* = 6.6 Hz, 2H), 3.36 - 3.32 (m, 2H), 3.23 - 3.18 (m, 4H), 3.13 - 3.10 (m, 1H), 2.70 - 2.59 (m, 5H), 1.69 (s, 6H), 1.21 (t, *J =* 7.5 Hz, 3H). LCMS (ESI) calcd for C₃₅H₃₆N₇O₃⁺ [M+H]⁺: 602.29; found, 602.4.

### Example 106: Preparation of 8-(4-(3-((2,6-dioxopiperidin-3-yl)amino)benzyl)piperazin-1-yl)-9-ethyl-6,6-dimethyl-11-oxo-6,11-dihydro-5H-benzo[b]carbazole-3-carbonitrile (GT-07328)

According to the method of step 2 of Scheme I-1, the target compound (GT-07328) was prepared (white solid, 8 mg, yield 26 %). ¹H NMR (500 MHz, DMSO) δ 12.92 (s, 1H), 10.99 (s, 1H), 10.86 (s, 1H), 8.31 (d, *J* = 8.1 Hz, 1H), 8.08 (s, 1H), 8.01 (s, 1H), 7.61 (dd, *J* = 8.2, 1.2 Hz, 1H), 7.37 (d, *J* = 5.2 Hz, 1H), 7.18 (t, *J* = 7.8 Hz, 1H), 7.03 (t, 1H), 6.91 - 6.73 (m, 2H), 4.40 (dd, 1H), 4.35 - 4.27 (m, 2H), 3.40 (d, *J =* 10.4 Hz, 1H), 3.36 - 3.22 (m, 6H), 2.88 - 2.77 (m, 1H), 2.75 - 2.69 (m, 2H), 2.64 - 2.58 (m, 1H), 2.22 - 2.14 (m, 1H), 1.96 - 1.86 (m, 1H), 1.77 (s, 6H), 1.28 (t, *J* = 7.5 Hz, 3H). LCMS (ESI) calcd for C₃₇H₃₉N₆O₃ ⁺ [M+H]⁺: 615.31; found, 615.4.

### Example 107: Preparation of 1-(4-((4-(4-((5-chloro-4-((2-(dimethylphosphoryl)phenyl)amino)pyrimidin-2-yl)amino)-3-methoxyphenyl)piperazin-1-yl)methyl)phenyl)dihydropyrimidine-2,4(1H,3H)-dione (GT-07345)

According to the method of step 2 of Scheme I-1, the target compound (GT-07345) was prepared (white solid, 22 mg, yield 45 %). ¹H NMR (500 MHz, DMSO) δ 11.85 (s, 1H), 11.53 - 11.35 (m, 1H), 10.45 (s, 1H), 9.16 (s, 1H), 8.39 (s, 1H), 8.20 (s, 1H), 7.69 (d, *J =* 8.5 Hz, 2H), 7.64 - 7.58 (m, 1H), 7.45 (d, *J =* 8.6 Hz, 2H), 7.38 (d, *J* = 8.5 Hz, 2H), 7.22 (t, *J* = 7.2 Hz, 1H), 6.73 (d, *J =* 2.3 Hz, 1H), 6.55 (dd, *J* = 8.7, 2.4 Hz, 1H), 4.40 (s, 2H), 3.88 (d, *J* = 11.3 Hz, 2H), 3.84 (t, *J* = 6.7 Hz, 2H), 3.79 (s, 3H), 3.40 (d, *J* = 10.7 Hz, 2H), 3.27 - 3.17 (m, 4H), 2.73 (t, *J* = 6.7 Hz, 2H), 1.80 (s, 3H), 1.78 (s, 3H). LCMS (ESI) calcd for C₃₄H₃₉ClN₈O₄P⁺ [M+H]⁺: 689.25 ; found, 689.3.

### Example 108: Preparation of 1-(3-((4-(4-((5-chloro-4-((2-(dimethylphosphoryl)phenyl)amino)pyrimidin-2-yl)amino)-3-methoxyphenyl)piperazin-1-yl)methyl)phenyl)dihydropyrimidine-2,4(1H,3H)-dione (GT-07348)

According to the method of step 2 of Scheme I-1, the target compound (GT-07348) was prepared (white solid, 22 mg, yield 45 %). ¹H NMR (500 MHz, DMSO) δ 12.09 (s, 1H), 11.72 (s, 1H), 10.46 (s, 1H), 9.59 (s, 1H), 8.24 (s, 2H), 7.68 (s, 1H), 7.64 (dd, *J* = 13.6, 7.8 Hz, 1H), 7.54 (d, *J* = 4.2 Hz, 1H), 7.50 - 7.45 (m, 2H), 7.44 - 7.30 (m, 2H), 7.26 (t, *J* = 7.2 Hz, 1H), 6.74 (d, *J* = 2.1 Hz, 1H), 6.57 (dd, *J* = 8.7, 1.9 Hz, 1H), 4.41 (s, 2H), 3.89 (t, *J* = 6.6 Hz, 4H), 3.79 (s, 3H), 3.41 (d, *J* = 11.1 Hz, 2H), 3.29 (t, *J* = 12.1 Hz, 2H), 3.18 (d, *J* = 9.9 Hz, 2H), 2.74 (t, *J* = 6.6 Hz, 2H), 1.80 (d, 6H). LCMS (ESI) calcd for C₃₄H₃₉ClN₈O₄P ⁺ [M+H]⁺: 689.25; found, 689.3.

### Example 109: Preparation of 1-(6-((4-(4-((5-chloro-4-((2-(dimethylphosphoryl)phenyl)amino)pyrimidin-2-yl)amino)-3-methoxyphenyl)piperazin-1-yl)methyl)pyridin-3-yl)dihydropyrimidine-2,4(1H,3H)-dione (GT-07349)

According to the method of step 2 of Scheme I-1, the target compound (GT-07349) was prepared (white solid, 17 mg, yield 34 %). ¹H NMR (500 MHz, DMSO) δ 12.16 (s, 1H), 11.31 (s, 1H), 10.57 (s, 1H), 9.93 - 9.40 (m, 1H), 8.72 (d, *J* = 2.5 Hz, 1H), 7.93 (dd, *J* = 8.4, 2.6 Hz, 1H), 7.82 - 7.78 (m, 1H), 7.65 (dd, *J* = 13.5, 7.8 Hz, 1H), 7.39 (s, 1H), 7.36 (d, *J =* 8.3 Hz, 1H), 7.26 (t, *J =* 7.1 Hz, 1H), 6.75 (d, *J =* 2.3 Hz, 1H), 6.58 (dd, *J* = 8.7, 2.2 Hz, 1H), 4.56 (s, 2H), 3.90 (t, *J* = 6.6 Hz, 3H), 3.79 (s, 3H), 3.65 (s, 2H), 3.47 - 3.27 (m, 5H), 2.76 (t, *J* = 6.3 Hz, 2H), 1.80 (d, *J* = 13.7 Hz, 6H). LCMS (ESI) calcd for C₃₃H₃₈ClN₉O₄P⁺ [M+H]⁺: 690.25; found, 690.3.

### Example 110: Preparation of 1-(5-((4-(4-((5-chloro-4-((2-(dimethylphosphoryl)phenyl)amino)pyrimidin-2-yl)amino)-3-methoxyphenyl)piperazin-1-yl)methyl)pyridin-2-yl)dihydropyrimidine-2,4(1H,3H)-dione (GT-07350)

According to the method of step 2 of Scheme I-1, the target compound (GT-07350) was prepared (yellow solid, 22 mg, yield 45 %). ¹H NMR (500 MHz, DMSO) δ 11.98 (s, 1H), 11.79 (s, 1H), 10.63 (s, 1H), 9.38 (s, 1H), 8.66 (d, *J* = 1.5 Hz, 1H), 8.35 (s, 1H), 8.26 (s, 1H), 8.15 (dd, *J* = 8.7, 2.0 Hz, 1H), 7.88 (d, *J =* 8.6 Hz, 1H), 7.63 (dd, *J* = 13.4, 7.6 Hz, 1H), 7.37 (d, *J* = 8.5 Hz, 2H), 7.24 (t, *J* = 7.1 Hz, 1H), 6.74 (s, 1H), 6.57 (d, *J* = 8.6 Hz, 1H), 4.44 (s, 2H), 4.11 (t, *J* = 6.5 Hz, 2H), 3.89 (d, *J* = 11.7 Hz, 2H), 3.79 (s, 3H), 3.42 (d, *J* = 10.7 Hz, 2H), 3.30 - 3.17 (m, 4H), 2.71 (t, *J* = 6.5 Hz, 2H), 1.80 (d, *J* = 13.6 Hz, 6H). LCMS (ESI) calcd for C₃₃H₃₈ClN₉O₄P⁺ [M+H]⁺: 690.25; found, 690.3.

### Example 111: Preparation of 3-((3-((4-(4-((5-chloro-4-((2-(dimethylphosphoryl)phenyl)amino)pyrimidin-2-yl)amino)-3-methoxyphenyl)piperazin-1-yl)methyl)phenyl)amino)piperidine-2,6-dione (GT-07351)

According to the method of step 2 of Scheme I-1, the target compound (GT-07351) was prepared (white solid, 19 mg, yield 38 %). ¹H NMR (500 MHz, DMSO) δ 12.22 (s, 1H), 11.56 (s, 1H), 10.86 (s, 1H), 9.79 (s, 1H), 8.26 (s, 2H), 7.65 (dd, *J =* 13.6, 7.7 Hz, 1H), 7.49 - 7.37 (m, 1H), 7.34 (d, *J =* 8.2 Hz, 1H), 7.28 (t, *J =* 7.4 Hz, 1H), 7.17 (t, *J =* 7.8 Hz, 1H), 7.10 (s, 1H), 6.85 - 6.79 (m, 2H), 6.75 (d, *J =* 2.2 Hz, 1H), 6.58 (dd, *J* = 8.8, 2.0 Hz, 1H), 4.43 (dd, *J* = 11.7, 4.8 Hz, 1H), 4.28 - 4.24 (m, 2H), 3.89 (d, *J* = 12.1 Hz, 2H), 3.79 (s, 3H), 3.41 - 3.36 (m, 2H), 3.32 (d, *J* = 12.3 Hz, 1H), 3.14 (dd, *J* = 20.4, 9.3 Hz, 2H), 2.89 - 2.80 (m, 1H), 2.63 - 2.56 (m, 1H), 2.23 - 2.16 (m, 1H), 1.96 - 1.85 (m, 1H), 1.80 (d, *J* = 13.7 Hz, 6H). LCMS (ESI) calcd for C₃₅H₄₁ClN₈O₄P⁺ [M+H]⁺: 703.27; found, 703.3.

### Example 112: Preparation of 3-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)piperidine-2,6-dione (GT-07364)

According to the method of step 2 of Scheme I-1, the target compound (GT-07364) was prepared (white solid, 44 mg, yield 77 %). ¹H NMR (500 MHz, DMSO) δ 10.89 (s, 1H), 10.88 (s, 1H), 9.91 (s, 1H), 8.92 (s, 1H), 8.42 (s, 1H), 8.20 (s, 1H), 7.90 (d, *J* = 7.6 Hz, 1H), 7.75 - 7.66 (m, 1H), 7.62 (d, *J* = 7.5 Hz, 2H), 7.54 - 7.42 (m, 1H), 7.36 - 7.33 (m, 2H), 7.12 (dd, *J* = 20.0, 7.6 Hz, 2H), 6.84 (s, 1H), 4.54 - 4.46 (m, 1H), 4.29 (s, 2H), 3.95 - 3.91 (m, 2H), 3.80 (dd, *J =* 11.1, 4.7 Hz, 1H), 3.50 - 3.44 (m, 1H), 3.40 (d, *J =* 10.7 Hz, 2H), 3.12 - 3.03 (m, 2H), 2.97 - 2.90 (m, 1H), 2.74 - 2.60 (m, 2H), 2.28 (s, 1H), 2.19 - 2.13 (m, 2H), 2.06 (s, 3H), 1.84 (d, *J* = 12.5 Hz, 2H), 1.28 (dd, *J* = 15.7, 6.6 Hz, 3H), 1.23 (d, *J* = 5.7 Hz, 3H), 1.13 (d, *J* = 6.4 Hz, 3H). LCMS (ESI) calcd for C₄₀H₄₈ClN₆O₅S⁺ [M+H]⁺: 759.31; found, 759.4.

### Example 113: Preparation of 1-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)dihydropyrimidine-2,4(1H,3H)-dione (GT-07592)

According to the method of step 2 of Scheme I-1, the target compound (GT-07592) was prepared (white solid, 20 mg, yield 35 %). ¹H NMR (400 MHz, DMSO-d6) δ 10.83 (s, 1H), 10.51 (s, 1H), 9.90 (s, 1H), 8.82 (s, 1H), 8.45 (s, 1H), 8.31 (d, *J* = 8.0 Hz, 1H), 7.95 (d, *J* = 7.9 Hz, 1H), 7.77 - 7.68 (m, 3H), 7.55 - 7.48 (m, 3H), 7.45 (s, 1H), 6.89 (s, 1H), 4.59 - 4.51 (m, 1H), 4.38 - 4.36 (m, 1H), 3.89 (t, *J* = 6.7 Hz, 3H), 3.53 - 3.46 (m, 3H), 3.18 - 3.09 (m, 2H), 3.04 - 2.95 (m, 1H), 2.79 (t, *J* = 6.5 Hz, 2H), 2.21 - 2.10 (m, 5H), 1.92 (d, *J* = 12.1 Hz, 2H), 1.29 (d, *J* = 5.9 Hz, 6H), 1.20 (d, *J* = 6.7 Hz, 6H). LCMS (ESI) calcd for C₃₉H₄₇ClN₇O₅S⁺ [M+H]⁺: 760.30; found, 760.4.

### Example 114: Preparation of 1-(3-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)dihydropyrimidine-2,4(1H,3H)-dione (GT-07593)

According to the method of step 2 of Scheme I-1, the target compound (GT-07593) was prepared (white solid, 25 mg, yield 44 %). ¹H NMR (400 MHz, DMSO-d6) δ 10.84 (s, 1H), 10.39 (s, 1H), 9.73 (s, 1H), 8.63 (s, 1H), 8.26 (d, *J* = 37.4 Hz, 2H), 7.81 (d, *J* = 8.0 Hz, 1H), 7.59 (d, *J* = 14.2 Hz, 2H), 7.43 - 7.32 (m, 5H), 6.77 (s, 1H), 4.46 - 4.36 (m, 1H), 4.26 (s, 2H), 3.82 (t, *J* = 6.6 Hz, 3H), 3.42 - 3.35 (m, 3H), 3.01 (d, *J* = 11.4 Hz, 2H), 2.92 - 2.82 (m, 1H), 2.67 (t, *J* = 6.4 Hz, 2H), 2.11 - 2.06 (m, 1H), 2.01 (s, 3H), 1.78 (d, *J =* 12.9 Hz, 2H), 1.16 (d, *J* = 6.0 Hz, 6H), 1.07 (d, *J* = 6.5 Hz, 6H). LCMS (ESI) calcd for C₃₉H₄₇ClN₇O₅S⁺ [M+H]⁺: 760.30; found, 760.4.

### Example 115: Preparation of 3-((3-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)amino)piperidine-2,6-dione (GT-07594)

According to the method of step 2 of Scheme I-1, the target compound (GT-07594) was prepared (gray solid, 26 mg, yield 45 %). ¹H NMR (400 MHz, DMSO-d6) δ 10.86 (s, 2H), 9.96 (s, 1H), 9.00 (s, 1H), 8.46 (s, 1H), 8.18 (s, 1H), 7.92 (t, *J =* 12.1 Hz, 1H), 7.71 (t, *J* = 7.6 Hz, 1H), 7.50 (t, *J =* 7.5 Hz, 1H), 7.35 (s, 1H), 7.16 (t, *J* = 7.8 Hz, 1H), 7.05 (s, 1H), 6.87 (s, 1H), 6.80 (d, *J* = 7.8 Hz, 2H), 4.54 - 4.47 (m, 1H), 4.47 - 4.41 (m, 1H), 4.19 - 4.15 (m, 2H), 3.48 (dd, *J* = 13.5, 6.7 Hz, 1H), 3.44 - 3.36 (m, 2H), 3.05 (dd, *J* = 22.5, 10.2 Hz, 2H), 2.98 - 2.80 (m, 2H), 2.60 (d, *J* = 17.7 Hz, 1H), 2.20 (d, *J* = 12.7 Hz, 3H), 2.05 (s, 3H), 1.97 - 1.87 (m, 1H), 1.83 (d, *J =* 13.7 Hz, 2H), 1.25 (d, *J* = 9.4, 4.8 Hz, 6H), 1.13 (d, *J* = 6.7 Hz, 6H). LCMS (ESI) calcd for C₄₀H₄₉ClN₇O₅S ⁺ [M+H]⁺: 774.32; found, 774.4 .

### Example 116: Preparation of 1-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)-5-fluoropyridin-2-yl)dihydropyrimidine-2,4(1H,3H)-dione (GT-07595)

According to the method of step 2 of Scheme I-1, the target compound (GT-07595) was prepared (white solid, 34 mg, yield 58 %). ¹H NMR (400 MHz, DMSO-d6) δ 11.13 (s, 1H), 10.59 (s, 1H), 9.80 (s, 1H), 8.77 (s, 1H), 8.51 (s, 1H), 8.33 (s, 1H), 8.17 (d, *J =* 8.1 Hz, 1H), 8.08 (d, *J =* 5.3 Hz, 1H), 7.82 (d, *J =* 7.7 Hz, 1H), 7.62 (t, *J =* 7.5 Hz, 1H), 7.40 (t, *J =* 7.7 Hz, 1H), 7.31 (s, 1H), 6.75 (s, 1H), 4.45 - 4.35 (m, 3H), 3.98 (t, *J* = 6.6 Hz, 2H), 3.46 (d, *J =* 10.9 Hz, 2H), 3.42 - 3.36 (m, 1H), 3.13 (d, *J =* 9.9 Hz, 2H), 2.89 (t, *J =* 11.9 Hz, 1H), 2.66 (t, *J* = 6.5 Hz, 2H), 2.08 (dd, *J* = 23.9, 12.4 Hz, 2H), 1.99 (s, 3H), 1.78 (d, *J* = 12.8 Hz, 2H), 1.17 (d, *J* = 6.0 Hz, 6H), 1.07 (d, *J* = 6.8 Hz, 6H). LCMS (ESI) calcd for C₃₈H₄₅ClFN₈O₅S ⁺ [M+H]⁺: 779.29; found, 779.3.

### Example 117: Preparation of 1-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)pyridin-2-yl)dihydropyrimidine-2,4(1H,3H)-dione (GT-07596)

According to the method of step 2 of Scheme I-1, the target compound (GT-07596) was prepared (white solid, 34 mg, yield 59 %). ¹H NMR (400 MHz, DMSO-d6) δ 11.27 (s, 1H), 10.64 (s, 1H), 9.89 (s, 1H), 8.88 (s, 1H), 8.54 (d, *J* = 5.7 Hz, 1H), 8.41 (s, 1H), 8.23 (d, *J =* 7.7 Hz, 1H), 7.98 (s, 1H), 7.90 (d, *J =* 7.9 Hz, 1H), 7.70 (t, *J =* 7.7 Hz, 1H), 7.63 (d, *J* = 5.0 Hz, 1H), 7.47 (t, *J =* 7.6 Hz, 1H), 7.39 (d, *J =* 8.4 Hz, 1H), 6.85 (s, 1H), 4.54 - 4.45 (m, 1H), 4.40 (d, *J* = 4.6 Hz, 2H), 4.10 (t, *J* = 6.5 Hz, 2H), 3.50 - 3.40 (m, 3H), 3.17 - 3.06 (m, 2H), 2.94 (t, *J* = 12.1 Hz, 1H), 2.72 (t, *J* = 6.5 Hz, 2H), 2.19 (dd, *J* = 24.0, 11.8 Hz, 2H), 2.06 (s, 3H), 1.84 (d, *J* = 14.2 Hz, 2H), 1.25 (d, *J* = 4.9 Hz, 6H), 1.14 (d, *J* = 6.8 Hz, 6H). LCMS (ESI) calcd for C₃₈H₄₆ClN₈O₅S ⁺ [M+H]⁺: 761.30; found, 761.3.

### Example 118: Preparation of 1-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)pyridin-3-yl)dihydropyrimidine-2,4(1H,3H)-dione (GT-07597)

According to the method of step 2 of Scheme I-1, the target compound (GT-07597) was prepared (white solid, 42 mg, yield 73 %). ¹H NMR (400 MHz, DMSO-d6) δ 11.49 (s, 1H), 10.66 (s, 1H), 9.88 (s, 1H), 8.86 (s, 1H), 8.80 (d, *J =* 2.0 Hz, 1H), 8.68 (s, 1H), 8.43 (s, 1H), 8.28 (d, *J* = 12.1 Hz, 1H), 8.24 (d, *J* = 7.5 Hz, 1H), 7.89 (d, *J =* 7.9 Hz, 1H), 7.70 (t, *J =* 7.7 Hz, 1H), 7.51 - 7.44 (m, 1H), 7.42 - 7.36 (m, 1H), 6.86 (s, 1H), 4.53 - 4.42 (m, 3H), 3.98 - 3.96 (m, 2H), 3.50 - 3.42 (m, 3H), 3.12 (dd, *J* = 21.5, 10.2 Hz, 2H), 3.01 - 2.94 (m, 1H), 2.78 (t, *J* = 6.5 Hz, 2H), 2.21 (dd, *J* = 23.9, 11.9 Hz, 2H), 2.07 (s, 3H), 1.84 (d, *J* = 13.8 Hz, 2H), 1.23 (d, *J* = 6.1 Hz, 6H), 1.14 (d, *J* = 6.7 Hz, 6H). LCMS (ESI) calcd for C₃₈H₄₆ClN₈O₅S ⁺ [M+H]⁺: 761.30; found, 761.4.

### Example 119: Preparation of 3-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)-6-fluoropyridin-3-yl)piperidine-2,6-dione (GT-07598)

According to the method of step 2 of Scheme I-1, the target compound (GT-07598) was prepared (white solid, 18 mg, yield 31 %). ¹H NMR (400 MHz, DMSO-d6) δ 11.14 (s, 1H), 10.93 (s, 1H), 9.75 (s, 1H), 8.67 (s, 1H), 8.32 (s, 1H), 8.24 (dd, *J* = 9.2, 2.2 Hz, 1H), 8.21 - 8.13 (m, 2H), 7.83 - 7.78 (m, 1H), 7.61 (t, *J* = 7.1 Hz, 1H), 7.41 - 7.32 (m, 2H), 6.74 (d, *J* = 8.7 Hz, 1H), 4.45 - 4.38 (m, 1H), 4.33 - 4.29 (m, 1H), 4.00 (dd, *J* = 12.5, 4.9 Hz, 1H), 3.46 - 3.35 (m, 4H), 3.28 (d, *J* = 11.2 Hz, 1H), 3.19 - 3.07 (m, 2H), 2.94 - 2.86 (m, 1H), 2.74 - 2.64 (m, 1H), 2.59 - 2.51 (m, 1H), 2.31 - 2.20 (m, 1H), 2.08 (dd, *J* = 13.9, 9.2 Hz, 2H), 2.01 (s, 3H), 1.82 - 1.74 (m, 2H), 1.16 (d, *J* = 6.1 Hz, 6H), 1.07 (d, *J* = 6.8 Hz, 6H). LCMS (ESI) calcd for C₃₉H₄₆ClFN₇O₅S ⁺ [M+H]⁺: 778.29; found, 778.

### Example 120: Preparation of 3-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)pyridin-2-yl)piperidine-2,6-dione (GT-07599)

According to the method of step 2 of Scheme I-1, the target compound (GT-07599) was prepared (white solid, 17 mg, yield 30 %). ¹H NMR (400 MHz, DMSO-d6) δ 11.33 (s, 1H), 10.90 (s, 1H), 9.72 (s, 1H), 8.59 (d, *J* = 5.0 Hz, 2H), 8.32 (s, 1H), 8.21 (d, *J* = 7.9 Hz, 1H), 7.81 (d, *J* = 7.3 Hz, 1H), 7.66 - 7.58 (m, 3H), 7.36 (dd, *J =* 15.4, 7.9 Hz, 2H), 6.79 (s, 1H), 4.47 - 4.39 (m, 1H), 4.33 (s, 2H), 4.03 (dd, *J =* 9.6, 5.3 Hz, 1H), 3.41 - 3.34 (m, 3H), 3.28 (d, *J =* 12.3 Hz, 1H), 3.09 - 3.00 (m, 2H), 2.92 - 2.85 (m, 1H), 2.67 - 2.56 (m, 1H), 2.52 - 2.46 (m, 1H), 2.27 - 2.21 (m, 1H), 2.13 (t, *J* = 6.5 Hz, 2H), 2.01 (s, 3H), 1.77 (d, *J* = 13.5 Hz, 2H), 1.17 (d, *J* = 6.0 Hz, 6H), 1.07 (d, *J* = 6.7 Hz, 6H). LCMS (ESI) calcd for C₃₉H₄₇ClN₇O₅S ⁺ [M+H]⁺: 760.30; found, 760.3.

### Example 121: Preparation of 3-(6-((4-(4-((5-chloro-4-((2-(isopropylsulfonyl)phenyl)amino)pyrimidin-2-yl)amino)-5-isopropoxy-2-methylphenyl)piperidin-1-yl)methyl)pyridin-3-yl)piperidine-2,6-dione (GT-07600)

According to the method of step 2 of Scheme I-1, the target compound (GT-07600) was prepared (white solid, 18 mg, yield 32 %). ¹H NMR (400 MHz, DMSO-d6) δ 10.89 (s, 1H), 10.53 (s, 1H), 9.72 (s, 1H), 8.62 (s, 1H), 8.52 (d, *J =* 1.9 Hz, 1H), 8.31 (s, 1H), 8.21 (d, *J =* 7.9 Hz, 1H), 7.84 - 7.74 (m, 2H), 7.62 (dd, *J* = 18.2, 7.8 Hz, 2H), 7.41 - 7.33 (m, 2H), 6.78 (s, 1H), 4.44 (s, 3H), 3.98 (dd, *J* = 12.6, 4.8 Hz, 1H), 3.45 - 3.37 (m, 3H), 3.17 (s, 2H), 2.90 (t, *J* = 11.7 Hz, 1H), 2.73 - 2.62 (m, 1H), 2.56 - 2.47 (m, 1H), 2.32 - 2.20 (m, 1H), 2.13 - 2.03 (m, 2H), 2.02 (s, 3H), 2.00 - 1.94 (m, 1H), 1.78 (d, *J* = 13.0 Hz, 2H), 1.18 (d, *J* = 6.0 Hz, 6H), 1.07 (d, *J* = 6.8 Hz, 6H). LCMS (ESI) calcd for C₃₉H₄₇ClN₇O₅S ⁺ [M+H]⁺: 760.30; found, 760.3.

### Example 122: Preparation of 3-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)pyridin-3-yl)piperidine-2,6-dione (GT-07601)

According to the method of step 2 of Scheme I-1, the target compound (GT-07601) was prepared (white solid, 33 mg, yield 58 %). ¹H NMR (400 MHz, DMSO-d6) δ 11.56 (s, 1H), 11.05 (s, 1H), 9.86 (s, 1H), 8.92 (s, 1H), 8.84 (s, 1H), 8.75 (s, 1H), 8.42 (s, 2H), 8.24 (d, *J* = 7.7 Hz, 1H), 7.89 (d, *J* = 7.1 Hz, 1H), 7.70 (t, *J* = 7.8 Hz, 1H), 7.48 (t, 1H), 7.40 (s, 1H), 6.85 (s, 1H), 4.54 - 4.44 (m, 3H), 4.15 (dd, *J* = 12.5, 4.8 Hz, 2H), 3.51 - 3.41 (m, 3H), 3.13 (s, 2H), 2.96 (t, *J* = 11.9 Hz, 1H), 2.83 - 2.72 (m, 1H), 2.67 - 2.58 (m, 1H), 2.42 - 2.30 (m, 1H), 2.24 - 2.13 (m, 3H), 2.07 (s, 3H), 1.84 (d, *J* = 13.7 Hz, 2H), 1.23 (d, *J* = 6.0 Hz, 6H), 1.14 (d, *J =* 6.7 Hz, 6H). LCMS (ESI) calcd for C₃₉H₄₇ClN₇O₅S ⁺ [M+H]⁺: 760.30; found, 760.3.

### Example 123: Preparation of 3-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)phenyl)piperidine-2,6-dione (GT-07602)

According to the method of step 2 of Scheme I-1, the target compound (GT-07602) was prepared (white solid, 32 mg, yield 57 %). ¹H NMR (400 MHz, DMSO-d6) δ 12.40 (s, 1H), 12.09 (s, 1H), 10.88 (s, 1H), 9.61 (s, 1H), 8.32 (s, 1H), 7.67 - 7.65 (m, 2H), 7.45 (s, 1H), 7.34 (d, *J =* 8.0 Hz, 2H), 7.28 (t, *J =* 7.3 Hz, 1H), 6.96 (s, 1H), 6.78 (s, 1H), 4.43 (s, 2H), 3.97 - 3.90 (m, 6H), 3.82 (s, 3H), 3.76 - 3.72 (m, 3H), 3.71 - 3.63 (m, 4H), 3.62 - 3.60 (m, 1H), 3.55 - 3.50 (m, 2H), 3.00 (s, 2H), 2.74 - 2.64 (m, 1H), 2.58 - 2.52 (m, 1H), 2.33 - 2.20 (m, 3H), 2.08 - 1.91 (m, 3H). LCMS (ESI) calcd for C₄₀H₄₉ClN₈O₄P ⁺ [M+H]⁺: 771.33; found, 771.4.

### Example 124: Preparation of 1-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)phenyl)dihydropyrimidine-2,4(1H,3H)-dione (GT-07603)

According to the method of step 2 of Scheme I-1, the target compound (GT-07603) was prepared (white solid, 31 mg, yield 56 %). ¹H NMR (400 MHz, DMSO-d6) δ 12.18 (s, 1H), 10.51 (s, 1H), 9.75 (s, 1H), 8.40 (s, 1H), 7.77 (d, *J* = 8.4 Hz, 1H), 7.72 (dd, *J* = 14.0, 7.7 Hz, 1H), 7.50 (d, *J* = 8.5 Hz, 3H), 7.35 (t, *J =* 7.3 Hz, 1H), 7.05 (s, 1H), 6.88 (s, 1H), 4.50 (s, 2H), 3.91 - 3.87 (m, 7H), 3.83 - 3.78 (m, 3H), 3.65 (d, *J =* 21.8 Hz, 3H), 3.10 (s, 2H), 2.83 - 2.72 (m, 2H), 2.34 (d, *J* = 9.5 Hz, 2H), 2.09 (s, 2H), 1.88 (s, 3H), 1.85 (s, 3H). LCMS (ESI) calcd for C₃₉H₄₈ClN₉O₄P⁺ [M+H]⁺: 772.32; found, 772.3.

### Example 125: Preparation of 1-(3-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)phenyl)dihydropyrimidine-2,4(1H,3H)-dione (GT-07604)

According to the method of step 2 of Scheme I-1, the target compound (GT-07604) was prepared (white solid, 42 mg, yield 75 %). ¹H NMR (400 MHz, DMSO-d6) δ 12.50 (s, 1H), 12.17 (s, 1H), 10.52 (s, 1H), 9.76 (s, 1H), 8.41 (s, 1H), 7.80 - 7.67 (m, 2H), 7.61 (d, *J* = 6.0 Hz, 1H), 7.58 - 7.45 (m, 4H), 7.35 (t, *J* = 7.3 Hz, 1H), 7.08 (s, 1H), 6.89 (s, 1H), 4.52 (s, 2H), 4.00 - 3.91 (m, 5H), 3.88 (s, 3H), 3.81 (s, 4H), 3.70 - 3.60 (m, 4H), 3.12 (s, 2H), 2.79 (t, *J =* 6.6 Hz, 2H), 2.34 (d, *J =* 9.0 Hz, 2H), 2.11 (s, 2H), 1.86 (d, *J =* 13.7 Hz, 6H). LCMS (ESI) calcd for C₃₉H₄₈ClN₉O₄P ⁺ [M+H]⁺: 772.32; found, 772.3.

### Example 126: Preparation of 3-((3-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)phenyl)amino)piperidine-2,6-dione (GT-07605)

According to the method of step 2 of Scheme I-1, the target compound (GT-07605) was prepared (white solid, 57 mg, yield 81 %). ¹H NMR (400 MHz, DMSO-d6) δ 12.22 (s, 1H), 12.14 (s, 1H), 10.86 (s, 1H), 9.73 (s, 1H), 8.31 (s, 2H), 7.66 (dd, *J =* 13.5, 7.8 Hz, 1H), 7.53 - 7.41 (m, 2H), 7.30 (t, *J* = 7.3 Hz, 1H), 7.16 (t, *J =* 7.8 Hz, 1H), 7.04 (d, *J =* 16.7 Hz, 2H), 6.83 (dd, *J =* 16.3, 7.8 Hz, 3H), 4.43 (dd, *J =* 11.7, 4.7 Hz, 2H), 4.31 (s, 2H), 3.92 (d, *J =* 10.9 Hz, 2H), 3.82 (s, 3H), 3.76 (s, 3H), 3.61 (s, 2H), 3.54 (s, 3H), 3.06 (s, 2H), 2.91 - 2.77 (m, 1H), 2.64 - 2.56 (m, 1H), 2.29 (d, *J =* 8.3 Hz, 2H), 2.23 - 2.16 (m, 1H), 2.04 (s, 2H), 1.95 - 1.86 (m, 1H), 1.82 (s, 3H), 1.79 (s, 3H). LCMS (ESI) calcd for C₄₀H₅₀ClN₉O₄P ⁺ [M+H]⁺: 786.34; found, 786.4.

### Example 127: Preparation of 1-(6-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-3-yl)dihydropyrimidine-2,4(1H,3H)-dione (GT-07606)

According to the method of step 2 of Scheme I-1, the target compound (GT-07606) was prepared (white solid, 45 mg, yield 64.8 %). ¹H NMR (400 MHz, DMSO-d6) δ 12.18 (s, 1H), 10.66 (s, 1H), 9.75 (s, 1H), 8.80 (dd, *J =* 24.0, 2.4 Hz, 1H), 8.40 (s, 2H), 8.01 (dd, *J =* 8.4, 2.6 Hz, 1H), 7.87 (t, *J =* 15.5 Hz, 1H), 7.72 (dd, *J =* 13.3, 7.2 Hz, 1H), 7.52 (s, 2H), 7.35 (t, *J =* 7.2 Hz, 1H), 7.08 (s, 1H), 6.89 (s, 1H), 4.61 (s, 2H), 4.00 - 3.92 (m, 6H), 3.88 (s, 3H), 3.75 - 3.68 (m, 4H), 3.11 (s, 1H), 2.82 (t, *J =* 6.6 Hz, 2H), 2.58 - 2.55 (m, 5H), 2.35 (d, *J* = 10.0 Hz, 2H), 2.11 (s, 1H), 1.87 (d, *J* = 13.7 Hz, 6H). LCMS (ESI) calcd for C₃₈H₄₇ClN₁₀O₄P⁺ [M+H]⁺: 773.32; found, 773.4.

### Example 128: Preparation of 1-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-2-yl)dihydropyrimidine-2,4(1H,3H)-dione (GT-07607)

According to the method of step 2 of Scheme I-1, the target compound (GT-07607) was prepared (white solid, 55 mg, yield 79 %). ¹H NMR (400 MHz, DMSO-d6) δ 12.22 (s, 1H), 12.15 (s, 1H), 10.63 (s, 1H), 9.77 (s, 1H), 8.68 (s, 1H), 8.36 (s, 2H), 8.15 (dd, *J =* 8.7, 2.1 Hz, 1H), 7.87 (d, *J =* 8.6 Hz, 1H), 7.66 (dd, *J=* 13.4, 7.5 Hz, 1H), 7.55 - 7.41 (m, 2H), 7.30 (t, *J =* 7.2 Hz, 1H), 7.05 (s, 1H), 6.85 (s, 1H), 4.50 (s, 3H), 4.11 *(t, J* = 6.4 Hz, 3H), 3.91 (d, *J =* 11.0 Hz, 3H), 3.82 (s, 3H), 3.76 - 3.64 (m, 6H), 3.09 (s, 2H), 2.71 (t, *J =* 6.5 Hz, 2H), 2.30 (d, *J =* 9.5 Hz, 2H), 2.14 - 1.97 (m, 2H), 1.81 (d, *J =* 13.7 Hz, 6H). LCMS (ESI) calcd for C₃₈H₄₇ClN₁₀O₄P⁺ [M+H]⁺: 773.32; found, 773.4.

### Example 129: Preparation of 1-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-5-fluoropyridin-2-yl)dihydropyrimidine-2,4(1H,3H)-dione (GT-07608)

According to the method of step 2 of Scheme I-1, the target compound (GT-07608) was prepared (white solid, 46 mg, yield 81 %). ¹H NMR (400 MHz, DMSO-d6) δ 12.13 (s, 1H), 10.62 (s, 1H), 9.72 (s, 1H), 8.50 (s, 1H), 8.35 (s, 2H), 8.02 (d, *J =* 4.3 Hz, 1H), 7.66 (dd, *J =* 13.1, 7.6 Hz, 1H), 7.47 (s, 2H), 7.35 - 7.23 (m, 1H), 7.06 (s, 1H), 6.87 (s, 1H), 4.22 (s, 2H), 4.05 - 4.01 (m, 3H), 3.91 (d, *J* = 10.3 Hz, 2H), 3.83 (s, 3H), 3.67 (s, 1H), 3.52 - 3.34 (m, 5H), 3.07 (s, 2H), 2.72 (s, 2H), 2.29 (s, 2H), 2.07 (s, 2H), 1.81 (d, *J* = 13.6 Hz, 6H). LCMS (ESI) calcd for C₃₈H₄₆ClFN₁₀O₄P⁺ [M+H]⁺: 791.31; found, 791.4 .

### Example 130: Preparation of 1-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-2-yl)dihydropyrimidine-2,4(1H,3H)-dione (GT-07609)

According to the method of step 2 of Scheme I-1, the target compound (GT-07609) was prepared (white solid, 40 mg, yield 72 %). ¹H NMR (400 MHz, DMSO-d6) δ 12.18 (s, 1H), 10.68 (s, 1H), 9.72 (s, 1H), 8.56 (d, *J* = 5.1 Hz, 1H), 8.39 (s, 2H), 8.04 (s, 1H), 7.72 (dd, *J* = 13.3, 8.2 Hz, 1H), 7.63 (d, *J* = 4.3 Hz, 1H), 7.56 - 7.46 (m, 2H), 7.35 (t, *J* = 7.2 Hz, 1H), 7.04 (s, 1H), 6.86 (s, 1H), 4.48 (s, 2H), 4.16 - 4.13 (m, 3H), 3.98 (d, *J* = 11.1 Hz, 4H), 3.88 (s, 3H), 3.78 (s, 2H), 3.61 - 3.55 (m, 3H), 3.07 (s, 1H), 2.77 (t, *J* = 6.6 Hz, 2H), 2.33 (d, *J* = 9.7 Hz, 2H), 2.14 - 1.98 (m, 2H), 1.86 (d, *J* = 13.7 Hz, 6H). LCMS (ESI) calcd for C₃₈H₄₇ClN₁₀O₄P ⁺ [M+H]⁺: 773.32; found, 773.4.

### Example 131: Preparation of 1-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-3-yl)dihydropyrimidine-2,4(1H,3H)-dione (GT-07610)

According to the method of step 2 of Scheme I-1, the target compound (GT-07610) was prepared (white solid, 43 mg, yield 77 %). ¹H NMR (400 MHz, DMSO-d6) δ 12.21 (s, 1H), 10.76 (s, 1H), 9.83 (s, 1H), 8.92 (d, *J* = 2.3 Hz, 1H), 8.83 (d, *J* = 1.3 Hz, 1H), 8.44 (d, *J* = 18.0 Hz, 3H), 7.72 (dd, *J* = 13.7, 7.6 Hz, 1H), 7.64 - 7.46 (m, 2H), 7.36 (t, *J* = 7.2 Hz, 1H), 7.12 (s, 1H), 6.93 (s, 1H), 4.61 (s, 2H), 4.04 (t, *J* = 6.6 Hz, 2H), 3.97 (d, *J* = 11.2 Hz, 2H), 3.88 (s, 3H), 3.79 (s, 2H), 3.70 (s, 7H), 3.16 (s, 2H), 2.83 (t, *J* = 6.6 Hz, 2H), 2.37 (d, *J* = 9.6 Hz, 2H), 2.23 - 2.03 (m, 2H), 1.87 (d, *J* = 13.7 Hz, 6H). LCMS (ESI) calcd for C₃₈H₄₇ClN₁₀O₄P ⁺ [M+H]⁺: 773.32; found, 773.4.

### Example 132: Preparation of 3-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-6-fluoropyridin-3-yl)piperidine-2,6-dione (GT-07611)

According to the method of step 2 of Scheme I-1, the target compound (GT-07611) was prepared (white solid, 26 mg, yield 46 %). ¹H NMR (400 MHz, DMSO-d6) δ 12.15 (s, 1H), 11.02 (s, 1H), 9.68 (s, 1H), 8.52 - 8.32 (m, 2H), 8.26 (d, *J* = 11.8 Hz, 1H), 7.71 (dd, *J* = 13.8, 7.7 Hz, 1H), 7.51 (s, 2H), 7.34 (t, *J* = 7.2 Hz, 1H), 7.03 (s, 1H), 6.86 (s, 1H), 4.37 (s, 2H), 4.12 (dd, *J* = 12.5, 4.8 Hz, 2H), 3.97 (d, *J* = 10.4 Hz, 3H), 3.88 (d, *J* = 2.8 Hz, 3H), 3.78 (s, 3H), 3.58 (s, 6H), 3.10 - 3.02 (m, 2H), 2.86 - 2.76 (m, 1H), 2.71 - 2.62 (m, 1H), 2.42 - 2.27 (m, 3H), 2.19 - 2.00 (m, 3H), 1.86 (d, *J* = 13.7 Hz, 6H). LCMS (ESI) calcd for C₃₉H₄₇ClFN₉O₄P⁺ [M+H]⁺: 790.32; found, 790.3.

### Example 133: Preparation of 3-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-2-yl)piperidine-2,6-dione (GT-07612)

According to the method of step 2 of Scheme I-1, the target compound (GT-07612) was prepared (white solid, 27 mg, yield 48 %). ¹H NMR (400 MHz, DMSO-d6) δ 12.20 (s, 1H), 11.08 (s, 1H), 9.82 (s, 1H), 8.79 (d, *J =* 5.3 Hz, 1H), 8.42 (s, 2H), 7.98 - 7.85 (m, 2H), 7.72 (dd, *J =* 13.7, 7.7 Hz, 1H), 7.60 - 7.47 (m, 2H), 7.35 (t, *J =* 7.2 Hz, 1H), 7.13 (s, 1H), 6.94 (s, 1H), 4.48 (s, 2H), 4.30 (dd, *J =* 10.0, 4.8 Hz, 2H), 3.97 (d, *J* = 11.3 Hz, 3H), 3.88 (s, 3H), 3.75 - 3.53 (m, 7H), 3.16 (s, 2H), 2.81 - 2.70 (m, 1H), 2.69 - 2.60 (m, 1H), 2.47 - 2.34 (m, 3H), 2.31 - 2.23 (m, 1H), 2.21 - 2.03 (m, 2H), 1.87 (d, *J* = 13.7 Hz, 6H). LCMS (ESI) calcd for C₃₉H₄₈ClN₉O₄P⁺ [M+H]⁺: 772.32; found, 772.3.

### Example 134: Preparation of 3-(6-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-3-yl)piperidine-2,6-dione (GT-07613)

According to the method of step 2 of Scheme I-1, the target compound (GT-07613) was prepared (white solid, 34 mg, yield 60.95 %). ¹H NMR (400 MHz, DMSO-d6) δ 12.12 (s, 1H), 11.05 (s, 1H), 9.64 (s, 1H), 8.69 (d, *J =* 1.9 Hz, 1H), 8.38 (s, 2H), 8.04 (dd, *J =* 8.1, 2.1 Hz, 1H), 7.83 (d, *J =* 8.1 Hz, 1H), 7.71 (dd, *J=* 13.2, 7.1 Hz, 1H), 7.51 (s, 2H), 7.34 (t, *J =* 7.2 Hz, 1H), 7.03 (s, 1H), 6.85 (s, 1H), 4.54 (s, 2H), 4.17 (dd, *J =* 12.6, 4.8 Hz, 2H), 3.98 (d, *J =* 11.7 Hz, 3H), 3.88 (s, 3H), 3.76 (s, 2H), 3.61 (s, 5H), 3.06 (s, 2H), 2.86 - 2.75 (m, 1H), 2.69 - 2.61 (m, 1H), 2.46 - 2.32 (m, 3H), 2.15 - 2.01 (m, 3H), 1.86 (d, *J* = 13.7 Hz, 6H). LCMS (ESI) calcd for C₃₉H₄₈ClN₉O₄P⁺ [M+H]⁺: 772.32; found, 772.4.

### Example 135: Preparation of 3-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-3-yl)piperidine-2,6-dione (GT-07614)

According to the method of step 2 of Scheme I-1, the target compound (GT-07614) was prepared (white solid, 39 mg, yield 70 %). ¹H NMR (400 MHz, DMSO-d6) δ 12.17 (s, 1H), 11.12 (s, 1H), 9.71 (s, 1H), 9.01 (s, 1H), 8.86 (d, *J =* 1.6 Hz, 1H), 8.53 (s, 1H), 8.39 (s, 2H), 7.72 (dd, *J =* 13.3, 7.3 Hz, 1H), 7.61 - 7.46 (m, 2H), 7.34 (t, *J =* 7.4 Hz, 1H), 7.05 (s, 1H), 6.87 (s, 1H), 4.51 (s, 2H), 4.25 (dd, *J* = 12.5, 4.8 Hz, 3H), 3.96 (d, 2H), 3.88 (s, 3H), 3.77 (s, 2H), 3.59 (s, 5H), 3.08 (s, 2H), 2.88 - 2.79 (m, 1H), 2.73 - 2.65 (m, 1H), 2.48 - 2.39 (m, 1H), 2.35 (d, *J =* 12.4 Hz, 2H), 2.26 - 2.18 (m, 1H), 2.10 (s, 2H), 1.86 (d, *J =* 13.7 Hz, 6H). LCMS (ESI) calcd for C₃₉H₄₈ClN₉O₄P ⁺ [M+H]⁺: 772.32; found, 772.3.

### Example 136: Preparation of 1-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)phenyl)dihydropyrimidine-2,4(1H,3H)-dione (GT-07615)

According to the method of step 2 of Scheme I-1, the target compound (GT-07615) was prepared (white solid, 29 mg, yield 49 %). ¹H NMR (400 MHz, DMSO-d6) δ 12.03 (s, 1H), 11.09 (s, 1H), 10.46 (s, 1H), 9.49 (s, 1H), 8.35 (d, *J =* 35.3 Hz, 2H), 7.72 (d, *J =* 8.5 Hz, 2H), 7.65 (dd, *J =* 14.0, 7.6 Hz, 1H), 7.44 (d, *J =* 8.5 Hz, 3H), 7.27 (t, *J* = 7.3 Hz, 1H), 6.89 (d, *J =* 62.1 Hz, 2H), 4.50 (d, *J =* 10.1 Hz, 1H), 4.28 - 4.22 (m, 1H), 3.83 (s, 4H), 3.50 (s, 2H), 3.01 (s, 1H), 2.73 (t, *J =* 6.6 Hz, 2H), 2.63 (d, *J =* 4.7 Hz, 3H), 2.43 - 2.28 (m, 2H), 2.11 (s, 2H), 1.81 (d, *J =* 13.7 Hz, 7H). LCMS (ESI) calcd for C₃₆H₄₃ClN₈O₄P⁺ [M+H]⁺: 717.28; found, 717.4.

### Example 137: Preparation of 1-(3-(((1-(4-((5-chloro-4-((2-(dimethylphosphoryl)phenyl)amino)pyrimidin-2-yl)amino)-3-methoxyphenyl)piperidin-4-yl)(methyl)amino)methyl)phenyl)dihydropyrimidine-2,4(1H,3H)-dione (GT-07616)

According to the method of step 2 of Scheme I-1, the target compound (GT-07616) was prepared (white solid, 39 mg, yield 66 %). ¹H NMR (400 MHz, DMSO-d6) δ 12.03 (s, 1H), 11.14 (s, 1H), 10.46 (s, 1H), 9.51 (s, 1H), 8.35 (d, *J* = 34.5 Hz, 2H), 7.71 - 7.62 (m, 2H), 7.56 - 7.43 (m, 5H), 7.27 (t, *J* = 7.2 Hz, 1H), 6.89 (d, *J =* 66.6 Hz, 2H), 4.51 (d, *J =* 9.3 Hz, 1H), 4.27 (dd, *J =* 12.8, 6.9 Hz, 1H), 3.91 - 3.86 (m, 6H), 3.83 (s, 3H), 3.50 (s, 1H), 3.01 (s, 1H), 2.74 (t, *J =* 6.7 Hz, 2H), 2.63 (d, *J =* 4.7 Hz, 3H), 2.35 (dd, *J =* 35.9, 11.8 Hz, 2H), 2.13 - 2.06 (m, 1H), 1.81 (d, *J* = 13.7 Hz, 6H). LCMS (ESI) calcd for C₃₆H₄₃ClN₈O₄P ⁺ [M+H]⁺: 717.28; found, 717.4.

### Example 138: Preparation of 1-(6-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-3-yl)dihydropyrimidine-2,4(1H,3H)-dione (GT-07617)

According to the method of step 2 of Scheme I-1, the target compound (GT-07617) was prepared (white solid, 37 mg, yield 63 %). ¹H NMR (400 MHz, DMSO-d6) δ 12.10 (s, 1H), 10.91 (s, 1H), 10.66 (s, 1H), 9.59 (s, 1H), 8.77 (d, *J* = 2.5 Hz, 1H), 8.38 (d, 2H), 7.98 (dd, *J* = 8.4, 2.6 Hz, 1H), 7.84 (d, *J* = 8.4 Hz, 1H), 7.71 (dd, *J =* 13.9, 7.8 Hz, 1H), 7.52 (s, 2H), 7.32 (t, *J =* 7.4 Hz, 1H), 6.97 (d, *J =* 65.7 Hz, 2H), 4.59 (s, 2H), 3.96 (t, *J =* 6.6 Hz, 5H), 3.88 (s, 4H), 3.62 (s, 1H), 3.07 (s, 1H), 2.85 - 2.80 (m, 5H), 2.38 (s, 2H), 2.17 (s, 1H), 1.86 (d, *J =* 13.7 Hz, 6H). LCMS (ESI) calcd for C₃₅H₄₂ClN₉O₄P ⁺ [M+H]⁺: 718.28; found, 718.3.

### Example 139: Preparation of 1-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-2-yl)dihydropyrimidine-2,4(1H,3H)-dione (GT-07691)

According to the method of step 2 of Scheme I-1, the target compound (GT-07691) was prepared (white solid, 34 mg, yield 58 %). ¹H NMR (400 MHz, DMSO-d6) δ 12.03 (s, 1H), 11.47 (s, 1H), 10.62 (s, 1H), 9.51 (s, 1H), 8.68 (d, J = 2.1 Hz, 1H), 8.53 - 8.34 (m, 1H), 8.31 (s, 1H), 8.22 - 8.16 (m, 1H), 7.87 (d, J = 8.7 Hz, 1H), 7.69 - 7.60 (m, 1H), 7.57 - 7.39 (m, 2H), 7.26 (t, J = 7.2 Hz, 1H), 7.00 (s, 1H), 6.85 (s, 1H), 4.52 (d, J = 10.5 Hz, 1H), 4.34 - 4.29 (m, 1H), 4.11 (t, J = 6.6 Hz, 3H), 3.91 (d, J = 9.4 Hz, 3H), 3.83 (s, 3H), 3.51 (s, 1H), 3.13 - 3.02 (m, 1H), 2.71 (t, J = 6.6 Hz, 2H), 2.63 (d, J = 4.5 Hz, 3H), 2.42 (d, J = 11.9 Hz, 1H), 2.36 - 2.29 (m, 1H), 2.13 (s, 1H), 1.80 (d, J = 13.7 Hz, 6H). LCMS (ESI) calcd for C₃₅H₄₂ClN₉O₄P ⁺ [M+H]⁺: 718.28; found, 718.3 .

### Example 140: Preparation of 3-((3-(((1-(4-((5-chloro-4-((2-(dimethylphosphoryl)phenyl)amino)pyrimidin-2-yl)amino)-3-methoxyphenyl)piperidin-4-yl)(methyl)amino)methyl)phenyl)amino)piperidine-2,6-dione (GT-07692)

According to the method of step 2 of Scheme I-1, the target compound (GT-07692) was prepared (white solid, 27 mg, yield 45 %). ¹H NMR (400 MHz, DMSO-d6) δ 12.10 (s, 1H), 11.01 (s, 1H), 10.91 (d, *J* = 3.4 Hz, 1H), 9.59 (s, 1H), 8.40 (d, *J* = 25.0 Hz, 2H), 7.71 (dd, *J* = 13.3, 7.8 Hz, 1H), 7.59 - 7.46 (m, 2H), 7.32 (t, *J* = 7.5 Hz, 1H), 7.23 (t, *J* = 7.8 Hz, 1H), 7.14 (s, 1H), 7.06 (s, 1H), 6.88 (dd, *J* = 14.5, 7.6 Hz, 3H), 4.49 (dd, *J* = 11.7, 3.0 Hz, 1H), 4.45 - 4.39 (m, 1H), 4.20 - 4.16 (m, 1H), 3.93 (d, 2H), 3.88 (s, 4H), 3.54 - 3.51 (m, 1H), 3.14 (s, 2H), 2.95 - 2.88 (m, 1H), 2.69 (d, *J* = 4.7 Hz, 3H), 2.49 - 2.43 (m, 1H), 2.37 - 2.31 (m, 1H), 2.28 - 2.10 (m, 3H), 2.01 - 1.94 (m, 1H), 1.88 (s, 3H), 1.85 (s, 3H). LCMS (ESI) calcd for C₃₇H₄₅ClN₈O₄P ⁺ [M+H]⁺: 731.30; found, 731.3.

### Example 141: Preparation of 1-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-5-fluoropyridin-2-yl)dihydropyrimidine-2,4(1H,3H)-dione (GT-07693)

According to the method of step 2 of Scheme I-1, the target compound (GT-07693) was prepared (white solid, 46 mg, yield 76 %). ¹H NMR (400 MHz, DMSO-d6) δ 12.05 (s, 1H), 11.21 (s, 1H), 10.66 (s, 1H), 9.52 (s, 1H), 8.57 (s, 1H), 8.39 (s, 1H), 8.31 (s, 1H), 8.12 (d, *J =* 5.3 Hz, 1H), 7.65 (dd, *J =* 13.6, 8.4 Hz, 1H), 7.51 - 7.38 (m, 2H), 7.27 (t, *J* = 7.3 Hz, 1H), 6.94 (s, 1H), 6.78 (s, 1H), 4.61 (s, 1H), 4.39 (s, 1H), 4.04 (t, *J* = 6.7 Hz, 2H), 3.94 (d, *J* = 11.5 Hz, 2H), 3.82 (s, 3H), 2.99 (s, 1H), 2.76 - 2.71 (m, 5H), 2.37 (s, 1H), 2.27 (s, 1H), 2.09 (s, 2H), 1.81 (d, *J* = 13.7 Hz, 6H). LCMS (ESI) calcd for C₃₅H₄₁ClFN₉O₄P ⁺ [M+H]⁺: 736.27; found, 736.2.

### Example 142: Preparation of 1-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-2-yl)dihydropyrimidine-2,4(1H,3H)-dione (GT-07694)

According to the method of step 2 of Scheme I-1, the target compound (GT-07694) was prepared (white solid, 37 mg, yield 63 %). ¹H NMR (400 MHz, DMSO-d6) δ 12.06 (s, 1H), 11.37 (s, 1H), 10.63 (s, 1H), 9.54 (s, 1H), 8.53 (d, *J =* 5.1 Hz, 1H), 8.35 (d, *J =* 30.4 Hz, 2H), 7.99 (s, 1H), 7.68 - 7.61 (m, 2H), 7.46 (s, 2H), 7.27 (t, *J* = 7.0 Hz, 1H), 6.96 (s, 1H), 6.80 (s, 1H), 4.56 (d, *J* = 12.4 Hz, 1H), 4.40 - 4.34 (m, 1H), 4.10 (t, *J* = 6.6 Hz, 2H), 3.92 - 3.89 (m, 2H), 3.82 (s, 3H), 3.51 - 3.49 (m, 1H), 3.07 - 2.94 (m, 2H), 2.72 (t, *J* = 6.6 Hz, 2H), 2.67 (d, *J =* 3.0 Hz, 3H), 2.41 - 2.36 (m, 1H), 2.33 - 2.27 (m, 1H), 2.09 (s, 2H), 1.81 (d, *J* = 13.7 Hz, 6H). LCMS (ESI) calcd for C₃₅H₄₂ClN₉O₄P⁺ [M+H]⁺: 718.28; found, 718.3.

### Example 143: Preparation of 1-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-3-yl)dihydropyrimidine-2,4(1H,3H)-dione (GT-07695)

According to the method of step 2 of Scheme I-1, the target compound (GT-07695) was prepared (white solid, 22 mg, yield 37 %). ¹H NMR (400 MHz, DMSO-d6) δ 12.14 (s, 1H), 11.77 (s, 1H), 10.74 (s, 1H), 9.70 (s, 1H), 8.87 (dd, *J =* 20.2, 1.8 Hz, 2H), 8.44 (d, *J =* 36.6 Hz, 3H), 7.72 (dd, *J =* 13.9, 7.7 Hz, 1H), 7.61 - 7.49 (m, 2H), 7.34 (t, *J* = 7.2 Hz, 1H), 7.12 (s, 1H), 6.95 (s, 1H), 4.70 (d, *J* = 12.3 Hz, 1H), 4.49 (s, 1H), 4.04 (t, *J* = 4.9 Hz, 4H), 3.89 (s, 3H), 3.66 (s, 1H), 3.19 (s, 2H), 2.84 (t, *J =* 6.7 Hz, 2H), 2.71 (s, 3H), 2.52 - 2.41 (m, 2H), 2.23 (s, 2H), 1.88 (s, 3H), 1.85 (s, 3H). LCMS (ESI) calcd for C₃₅H₄₂ClN₉O₄P ⁺ [M+H]⁺: 718.28; found, 718.3.

### Example 145: Preparation of 3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-6-fluoropyridin-3-yl)piperidine-2,6-dione (GT-07696)

According to the method of step 2 of Scheme I-1, the target compound (GT-07696) was prepared (white solid, 32 mg, yield 53 %). ¹H NMR (400 MHz, DMSO-d6) δ 11.98 (s, 1H), 11.22 (s, 1H), 10.93 (s, 1H), 9.48 (s, 1H), 8.29 - 8.23 (m, 2H), 8.18 (s, 1H), 7.58 (dd, *J* = 13.6, 7.9 Hz, 1H), 7.44 - 7.34 (m, 2H), 7.20 (t, *J* = 7.4 Hz, 1H), 6.89 (s, 1H), 6.73 (s, 1H), 4.51 (d, *J =* 12.8 Hz, 1H), 4.22 (s, 1H), 4.00 (dd, *J =* 12.5, 4.7 Hz, 2H), 3.85 (s, 4H), 3.75 (s, 3H), 3.01 - 2.89 (m, 2H), 2.67 - 2.58 (m, 4H), 2.35 - 2.29 (m, 1H), 2.27 - 2.17 (m, 2H), 2.06 - 2.00 (m, 2H), 1.75 (s, 3H), 1.72 (s, 3H). LCMS (ESI) calcd for C₃₆H₄₂ClFN₈O₄P ⁺ [M+H]⁺: 735.27; found, 735.3.

### Example 146: Preparation of 3-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-2-yl)piperidine-2,6-dione (GT-07697)

According to the method of step 2 of Scheme I-1, the target compound (GT-07697) was prepared (white solid, 23 mg, yield 39 %). ¹H NMR (400 MHz, DMSO-d6) δ 12.13 (s, 1H), 11.57 (s, 1H), 11.04 (s, 1H), 9.64 (s, 1H), 8.64 (s, 1H), 8.50 - 8.32 (m, 2H), 8.10 - 8.03 (m, 1H), 7.90 - 7.82 (m, 1H), 7.74 - 7.67 (m, 1H), 7.59 - 7.44 (m, 2H), 7.32 (t, *J =* 17.0, 10.2 Hz, 1H), 7.05 (s, 1H), 6.95 - 6.81 (m, 1H), 4.65 (d, *J =* 12.8 Hz, 1H), 4.42 (s, 1H), 3.96 (s, 4H), 3.89 (s, 3H), 3.62 - 3.56 (m, 2H), 3.20 - 3.04 (m, 2H), 2.74 - 2.65 (m, 4H), 2.47 - 2.38 (m, 3H), 2.31 - 2.22 (m, 2H), 1.88 (s, 3H), 1.85 (s, 3H). LCMS (ESI) calcd for C₃₆H₄₃ClN₈O₄P ⁺ [M+H]⁺: 717.28; found, 717.3.

### Example 147: Preparation of 3-(6-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-3-yl)piperidine-2,6-dione (GT-07698)

According to the method of step 2 of Scheme I-1, the target compound (GT-07698) was prepared (white solid, 39 mg, yield 66.3 %). ¹H NMR (400 MHz, DMSO-d6) δ 12.15 (s, 1H), 11.03 (s, 1H), 9.75 (s, 1H), 8.66 (d, *J =* 2.0 Hz, 1H), 8.42 (s, 2H), 7.93 (dd, *J =* 8.1, 2.1 Hz, 1H), 7.81 (d, *J =* 8.1 Hz, 1H), 7.72 (dd, *J* = 12.9, 7.8 Hz, 1H), 7.61 - 7.49 (m, 2H), 7.34 (t, *J =* 7.3 Hz, 1H), 7.16 (s, 1H), 6.99 (s, 1H), 4.61 (s, 2H), 4.15 (d, 1H), 4.11 (d, 1H), 3.98 - 3.93 (m, 6H), 3.89 (s, 3H), 3.72 - 3.64 (m, 2H), 3.28 - 3.14 (m, 2H), 2.84 (s, 3H), 2.82 - 2.75 (m, 1H), 2.68 - 2.62 (m, 1H), 2.46 - 2.36 (m, 3H), 2.32 - 2.18 (m, 2H), 2.16 - 2.10 (m, 1H), 1.86 (d, *J =* 13.7 Hz, 6H). LCMS (ESI) calcd for C₃₆H₄₃ClN₈O₄P ⁺ [M+H]⁺: 717.28; found, 717.3.

### Example 148: Preparation of 3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-3-yl)piperidine-2,6-dione (GT-07699)

According to the method of step 2 of Scheme I-1, the target compound (GT-07699) was prepared (white solid, 36 mg, yield 61 %). ¹H NMR (400 MHz, DMSO-d6) δ 12.12 (s, 1H), 11.79 (s, 1H), 11.12 (s, 1H), 9.54 (d, *J =* 81.8 Hz, 1H), 9.08 (s, 1H), 8.85 (d, *J =* 1.6 Hz, 1H), 8.61 (s, 1H), 8.38 (d, 2H), 7.71 (dd, *J =* 13.9, 7.6 Hz, 1H), 7.58 - 7.47 (m, 2H), 7.33 (t, *J =* 7.2 Hz, 1H), 7.08 (s, 1H), 6.91 (s, 1H), 4.73 (d, *J =* 12.5 Hz, 1H), 4.51 (s, 1H), 4.24 (dd, 2H), 3.89 (s, 3H), 3.65 (s, 2H), 3.14 (s, 2H), 2.88 - 2.79 (m, 1H), 2.72 - 2.68 (m, 3H), 2.63 (t, *J =* 5.2 Hz, 1H), 2.47 - 2.31 (m, 3H), 2.26 - 2.08 (m, 3H), 1.88 (s, 3H), 1.85 (s, 3H). LCMS (ESI) calcd for C₃₆H₄₃ClN₈O₄P⁺ [M+H]⁺: 717.28; found, 717.3.

### Example 149: Preparation of 8-(4-(4-((2-(2,4-dioxotetrahydropyrimidin-1(2H)-yl)-5-fluoropyridin-4-yl)methyl)piperazin-1-yl)piperidin-1-yl)-9-ethyl-6,6-dimethyl-11-oxo-6,11-dihydro-5H-benzo[b]carbazole-3-carbonitrile (GT-07700)

According to the method of step 2 of Scheme I-1, the target compound (GT-07700) was prepared (white solid, 28 mg, yield 50 %). ¹H NMR (400 MHz, DMSO-d6) δ 12.98 (s, 1H), 11.19 (s, 1H), 10.66 (s, 1H), 8.52 (s, 1H), 8.38 (d, *J =* 8.2 Hz, 1H), 8.12 (s, 1H), 8.07 (s, 1H), 8.00 (d, *J =* 5.2 Hz, 1H), 7.66 (dd, *J =* 8.2, 1.3 Hz, 1H), 7.43 (s, 1H), 4.13 - 4.02 (m, 4H), 3.57 - 3.42 (m, 3H), 3.40 - 3.23 (m, 6H), 3.04 (s, 2H), 2.88 (t, *J =* 11.5 Hz, 2H), 2.80 - 2.73 (m, 4H), 2.29 (d, *J =* 9.9 Hz, 2H), 2.04 - 1.92 (m, 2H), 1.83 (s, 6H), 1.34 (t, *J=* 7.5 Hz, 3H). LCMS (ESI) calcd for C₄₀H₄₄FN₈O₃⁺ [M+H]⁺: 703.35; found, 703.4.

### Example 150: Preparation of 8-(4-(4-((2-(2,4-dioxotetrahydropyrimidin-1(2H)-yl)pyridin-4-yl)methyl)piperazin-1-yl)piperidin-1-yl)-9-ethyl-6,6-dimethyl-11-oxo-6,11-dihydro-5H-benzo[b]carbazole-3-carbonitrile (GT-07701)

According to the method of step 2 of Scheme I-1, the target compound (GT-07701) was prepared (yellow solid, 27 mg, yield 49 %). ¹H NMR (400 MHz, DMSO-d6) δ 13.03 (s, 1H), 12.12 (s, 1H), 10.69 (s, 1H), 8.57 (d, *J =* 5.1 Hz, 1H), 8.38 (d, *J =* 8.2 Hz, 1H), 8.12 (s, 1H), 8.06 (d, *J =* 6.2 Hz, 2H), 7.66 (dd, *J =* 8.2, 1.3 Hz, 1H), 7.63 (d, *J =* 4.8 Hz, 1H), 7.43 (s, 1H), 4.49 (s, 2H), 4.15 (t, *J =* 6.6 Hz, 2H), 3.74 - 3.59 (m, 6H), 3.58 - 3.47 (m, 3H), 3.38 (d, *J =* 11.2 Hz, 2H), 2.90 (t, *J =* 11.4 Hz, 2H), 2.82 - 2.73 (m, 4H), 2.30 (d, *J* = 9.3 Hz, 2H), 2.07 - 1.94 (m, 2H), 1.83 (s, 6H), 1.35 (t, *J* = 7.5 Hz, 3H). LCMS (ESI) calcd for C₄₀H₄₅N₈O₃⁺ [M+H]⁺: 685.36; found, 685.5 .

### Example 151: Preparation of 8-(4-(4-((5-(2,4-dioxotetrahydropyrimidin-1(2H)-yl)pyridin-3-yl)methyl)piperazin-1-yl)piperidin-1-yl)-9-ethyl-6,6-dimethyl-11-oxo-6,11-dihydro-5H-benzo[b]carbazole-3-carbonitrile (GT-07702)

According to the method of step 2 of Scheme I-1, the target compound (GT-07702) was prepared (yellow solid, 22 mg, yield 40 %). ¹H NMR (400 MHz, DMSO-d6) δ 12.99 (s, 1H), 12.23 (s, 1H), 10.68 (s, 1H), 8.86 (d, *J =* 2.3 Hz, 1H), 8.76 (s, 1H), 8.39 (s, 1H), 8.32 (d, *J =* 8.2 Hz, 1H), 8.06 (s, 1H), 8.01 (s, 1H), 7.61 (dd, *J =* 8.2, 1.3 Hz, 1H), 7.37 (s, 1H), 4.52 (s, 2H), 3.96 (s, 4H), 3.84 - 3.71 (m, 6H), 3.62 (s, 5H), 3.55 - 3.47 (m, 2H), 3.33 (d, *J =* 11.0 Hz, 2H), 2.88 - 2.76 (m, 4H), 2.72 (q, *J =* 7.4 Hz, 2H), 2.25 (d, *J =* 9.3 Hz, 2H), 2.02 - 1.89 (m, 2H), 1.29 (t, *J* = 7.5 Hz, 3H). LCMS (ESI) calcd for C₄₀H₄₅N₈O₃⁺ [M+H]⁺: 685.36; found, 685.4.

### Example 152: Preparation of 8-(4-(4-((5-(2,6-dioxopiperidin-3-yl)-2-fluoropyridin-3-yl)methyl)piperazin-1-yl)piperidin-1-yl)-9-ethyl-6,6-dimethyl-11-oxo-6,11-dihydro-5H-benzo[b]carbazole-3-carbonitrile (GT-07703)

According to the method of step 2 of Scheme I-1, the target compound (GT-07703) was prepared (white solid, 14 mg, yield 25 %). ¹H NMR (400 MHz, DMSO-d6) δ 12.98 (s, 1H), 11.63 (s, 1H), 11.04 (s, 1H), 8.38 (d, *J =* 8.2 Hz, 1H), 8.26 (s, 1H), 8.18 (d, 1H), 8.12 (s, 1H), 8.07 (s, 1H), 7.66 (dd, *J =* 8.2, 1.3 Hz, 1H), 7.42 (s, 1H), 4.27 (s, 2H), 4.11 (dd, *J* = 12.5, 4.8 Hz, 1H), 3.75 (s, 3H), 3.54 - 3.46 (m, 5H), 3.38 (d, *J =* 11.1 Hz, 3H), 2.88 (t, *J* = 11.3 Hz, 2H), 2.83 - 2.72 (m, 3H), 2.72 - 2.63 (m, 1H), 2.42 - 2.24 (m, 3H), 2.19 - 2.11 (m, 1H), 2.05 - 1.94 (m, 2H), 1.82 (s, 6H), 1.34 (t, *J =* 7.5 Hz, 3H). LCMS (ESI) calcd for C₄₁H₄₅FN₇O₃ ⁺ [M+H]⁺: 702.36; found, 702.4.

### Example 153: Preparation of 8-(4-(4-((2-(2,6-dioxopiperidin-3-yl)pyridin-4-yl)methyl)piperazin-1-yl)piperidin-1-yl)-9-ethyl-6,6-dimethyl-11-oxo-6,11-dihydro-5H-benzo[b]carbazole-3-carbonitrile (GT-07704)

According to the method of step 2 of Scheme I-1, the target compound (GT-07704) was prepared (white solid, 4 mg, yield 7 %). ¹H NMR (400 MHz, DMSO-d6) δ 13.02 (s, 1H), 11.96 (s, 1H), 11.08 (s, 1H), 8.79 (d, *J* = 5.3 Hz, 1H), 8.38 (d, *J =* 8.2 Hz, 1H), 8.12 (s, 1H), 8.07 (s, 1H), 7.91 - 7.80 (m, 2H), 7.66 (dd, *J* = 8.2, 1.3 Hz, 1H), 7.43 (s, 1H), 4.39 (s, 1H), 4.32 - 4.27 (m, 1H), 3.65 - 3.58 (m, 4H), 3.55 - 3.46 (m, 4H), 3.38 (d, *J* = 11.2 Hz, 4H), 2.90 (t, *J* = 11.5 Hz, 2H), 2.81 - 2.73 (m, 3H), 2.68 - 2.62 (m, 1H), 2.45 - 2.38 (m, 1H), 2.34 - 2.25 (m, 3H), 2.05 - 1.95 (m, 2H), 1.83 (s, 6H), 1.35 (t, *J* = 7.5 Hz, 3H). LCMS (ESI) calcd for C₄₁H₄₆N₇O₃⁺ [M+H]⁺: 684.37; found, 684.4.

### Example 154: Preparation of 8-(4-(4-((5-(2,6-dioxopiperidin-3-yl)pyridin-2-yl)methyl)piperazin-1-yl)piperidin-1-yl)-9-ethyl-6,6-dimethyl-11-oxo-6,11-dihydro-5H-benzo[b]carbazole-3-carbonitrile (GT-07705)

According to the method of step 2 of Scheme I-1, the target compound (GT-07705) was prepared (white solid, 27 mg, yield 50 %). ¹H NMR (400 MHz, DMSO-d6) δ 12.99 (s, 1H), 12.03 (s, 1H), 11.05 (s, 1H), 8.94 (s, 1H), 8.80 (s, 1H), 8.46 (s, 1H), 8.32 (d, *J* = 8.2 Hz, 1H), 8.06 (s, 1H), 8.01 (s, 1H), 7.61 (d, *J =* 8.2, 1.2 Hz, 1H), 7.37 (s, 1H), 4.41 (s, 1H), 4.20 (dd, *J* = 12.6, 4.8 Hz, 1H), 3.66 - 3.61 (m, 2H), 3.58 - 3.38 (m, 8H), 3.32 (d, *J* = 10.8 Hz, 3H), 2.84 (t, *J* = 11.2 Hz, 2H), 2.75 - 2.70 (m, 2H), 2.68 - 2.59 (m, 1H), 2.40 - 2.32 (m, 1H), 2.25 (d, *J* = 9.3 Hz, 2H), 2.19 - 2.11 (m, 1H), 2.01 - 1.89 (m, 2H), 1.77 (s, 6H), 1.29 (t, *J* = 7.5 Hz, 3H). LCMS (ESI) calcd for C₄₁H₄₆N₇O₃ ⁺ [M+H]⁺: 684.37; found, 684.4.

### Example 155: Preparation of 8-(4-(4-((5-(2,6-dioxopiperidin-3-yl)pyridin-3-yl)methyl)piperazin-1-yl)piperidin-1-yl)-9-ethyl-6,6-dimethyl-11-oxo-6,11-dihydro-5H-benzo[b]carbazole-3-carbonitrile (GT-07706)

According to the method of step 2 of Scheme I-1, the target compound (GT-07706) was prepared (white solid, 29 mg, yield 53 %). ¹H NMR (400 MHz, DMSO-d6) δ 13.06 (s, 1H), 12.05 (s, 1H), 11.05 (s, 1H), 8.70 (d, *J =* 1.8 Hz, 1H), 8.38 (d, *J =* 8.2 Hz, 1H), 8.12 (s, 1H), 8.10 - 8.01 (m, 2H), 7.83 (d, *J =* 8.1 Hz, 1H), 7.66 (d, *J =* 8.2, 1.2 Hz, 1H), 7.43 (s, 1H), 4.56 (s, 2H), 4.18 (dd, *J =* 12.6, 4.7 Hz, 1H), 3.68 - 3.53 (m, 10H), 3.39 (d, *J =* 11.1 Hz, 2H), 2.90 (t, *J =* 11.6 Hz, 2H), 2.80 - 2.76 (m, 2H), 2.69 - 2.60 (m, 1H), 2.47 - 2.36 (m, 1H), 2.31 (d, *J* = 9.4 Hz, 2H), 2.17 - 2.07 (m, 1H), 2.06 - 1.96 (m, 2H), 1.83 (s, 6H), 1.35 (t, *J =* 7.5 Hz, 3H). LCMS (ESI) calcd for C₄₁H₄₆N₇O₃⁺ [M+H]⁺: 684.37; found, 684.4.

### Example 156: Preparation of 1-(4-((4-(4-((5-chloro-4-((2-(dimethylphosphoryl)phenyl)amino)pyrimidin-2-yl)amino)-3-methoxyphenyl)piperazin-1-yl)methyl)-5-fluoropyridin-2-yl)dihydropyrimidine-2,4(1H,3H)-dione (GT-07707)

According to the method of step 2 of Scheme I-1, the target compound (GT-07707) was prepared (white solid, 22 mg, yield 36 %). ¹H NMR (400 MHz, DMSO-d6) δ 11.88 (s, 1H), 11.36 (s, 1H), 10.67 (s, 1H), 9.24 (s, 1H), 8.58 (s, 1H), 8.39 (s, 1H), 8.24 (s, 1H), 8.14 (d, *J* = 5.2 Hz, 1H), 7.62 (dd, *J =* 13.5, 7.9 Hz, 1H), 7.39 (d, *J* = 8.5 Hz, 2H), 7.22 (t, *J =* 7.0 Hz, 1H), 6.75 (s, 1H), 6.57 (d, *J =* 8.7 Hz, 1H), 4.53 (s, 2H), 4.06 (t, *J =* 6.6 Hz, 2H), 3.89 (s, 2H), 3.79 (s, 3H), 3.33 - 3.15 (m, 6H), 2.73 (t, *J =* 6.6 Hz, 2H), 1.80 (d, *J* = 13.6 Hz, 6H). LCMS (ESI) calcd for C₃₃H₃₇ClFN₉O₄P ⁺ [M+H]⁺: 708.24; found, 708.1.

### Example 157: Preparation of 1-(4-((4-(4-((5-chloro-4-((2-(dimethylphosphoryl)phenyl)amino)pyrimidin-2-yl)amino)-3-methoxyphenyl)piperazin-1-yl)methyl)pyridin-2-yl)dihydropyrimidine-2,4(1H,3H)-dione (GT-07708)

According to the method of step 2 of Scheme I-1, the target compound (GT-07708) was prepared (white solid, 20 mg, yield 40 %). ¹H NMR (400 MHz, DMSO-d6) δ 12.24 (s, 1H), 11.98 (s, 1H), 10.64 (s, 1H), 9.84 (s, 1H), 8.53 (t, *J =* 6.2 Hz, 1H), 8.34 (s, 1H), 7.97 (s, 1H), 7.74 - 7.61 (m, 2H), 7.41 (s, 1H), 7.35 (d, *J = 8.4* Hz, 1H), 7.29 (t, *J* = 7.3 Hz, 1H), 6.76 (d, *J =* 2.3 Hz, 1H), 6.58 (dd, *J =* 8.8, 2.2 Hz, 1H), 4.49 (s, 2H), 4.10 (t, *J* = 6.6 Hz, 3H), 3.93 (s, 1H), 3.80 (s, 3H), 3.48 - 3.41 (m, 2H), 3.36 - 3.27 (m, 2H), 3.23 (s, 2H), 2.72 (t, *J* = 6.6 Hz, 2H), 1.82 (d, *J* = 7.1 Hz, 3H), 1.78 (d, *J* = 7.3 Hz, 3H). LCMS (ESI) calcd for C₃₃H₃₈ClN₉O₄P⁺ [M+H]⁺: 690.25; found, 690.1.

### Example 158: Preparation of 1-(5-((4-(4-((5-chloro-4-((2-(dimethylphosphoryl)phenyl)amino)pyrimidin-2-yl)amino)-3-methoxyphenyl)piperazin-1-yl)methyl)pyridin-3-yl)dihydropyrimidine-2,4(1H,3H)-dione (GT-07709)

According to the method of step 2 of Scheme I-1, the target compound (GT-07709) was prepared (yellow solid, 16 mg, yield 32 %). ¹H NMR (400 MHz, DMSO-d6) δ 12.18 (s, 1H), 12.01 (s, 1H), 10.66 (s, 1H), 9.72 (s, 1H), 8.81 (s, 1H), 8.71 (s, 1H), 8.32 (s, 2H), 7.65 (dd, *J =* 13.2, 7.6 Hz, 1H), 7.41 (s, 1H), 7.35 (d, *J = 8.6* Hz, 1H), 7.28 (t, *J* = 7.2 Hz, 1H), 6.76 (d, *J =* 2.3 Hz, 1H), 6.59 (dd, *J =* 8.8, 2.3 Hz, 1H), 4.53 (s, 2H), 3.97 (t, *J =* 6.6 Hz, 3H), 3.90 - 3.89 (m, 1H), 3.80 (s, 3H), 3.46 (s, 2H), 3.35 - 3.20 (m, 4H), 2.78 (t, *J* = 6.6 Hz, 2H), 1.82 (d, *J* = 6.3 Hz, 3H), 1.78 (d, *J* = 6.1 Hz, 3H). LCMS (ESI) calcd for C₃₃H₃₈ClN₉O₄P ⁺ [M+H]⁺: 690.25; found, 690.1.

### Example 159: Preparation of 3-(5-((4-(4-((5-chloro-4-((2-(dimethylphosphoryl)phenyl)amino)pyrimidin-2-yl)amino)-3-methoxyphenyl)piperazin-1-yl)methyl)-6-fluoropyridin-3-yl)piperidine-2,6-dione (GT-07710)

According to the method of step 2 of Scheme I-1, the target compound (GT-07710) was prepared (white solid, 13 mg, yield 26 %). ¹H NMR (400 MHz, DMSO-d6) δ 12.10 (s, 1H), 11.72 (s, 1H), 11.06 (s, 1H), 9.54 (s, 1H), 8.48 - 8.26 (m, 4H), 7.69 (dd, *J* = 13.5, 7.2 Hz, 1H), 7.55 - 7.39 (m, 2H), 7.31 (t, *J* = 7.5 Hz, 1H), 6.81 (d, *J* = 2.3 Hz, 1H), 6.63 (d, 1H), 4.54 (s, 2H), 4.13 (dd, *J* = 12.5, 4.9 Hz, 1H), 3.95 (s, 3H), 3.85 (s, 3H), 3.33 (s, 5H), 2.88 - 2.79 (m, 1H), 2.72 - 2.68 (m, 1H), 2.43 - 2.33 (m, 1H), 2.23 - 2.15 (m, 1H), 1.86 (d, *J* = 13.7 Hz, 6H). LCMS (ESI) calcd for C₃₄H₃₈ClFN₈O₄P ⁺ [M+H]⁺: 707.24; found, 707.2.

### Example 160: Preparation of 3-(4-((4-(4-((5-chloro-4-((2-(dimethylphosphoryl)phenyl)amino)pyrimidin-2-yl)amino)-3-methoxyphenyl)piperazin-1-yl)methyl)pyridin-2-yl)piperidine-2,6-dione (GT-07712)

According to the method of step 2 of Scheme I-1, the target compound (GT-07712) was prepared (white solid, 13 mg, yield 26 %). ¹H NMR (400 MHz, DMSO-d6) δ 12.28 (s, 1H), 12.17 (s, 1H), 11.03 (s, 1H), 9.86 (s, 1H), 8.76 (d, *J =* 5.7 Hz, 1H), 8.32 (s, 2H), 7.83 (s, 2H), 7.76 - 7.63 (m, 1H), 7.47 (s, 1H), 7.41 (d, *J* = 8.7 Hz, 1H), 7.34 (t, *J* = 7.4 Hz, 1H), 6.82 (s, 1H), 6.65 (d, 1H), 4.56 (s, 2H), 4.22 - 4.18 (m, 1H), 3.85 (s, 3H), 3.55 - 3.20 (m, 8H), 2.80 - 2.69 (m, 1H), 2.67 - 2.59 (m, 1H), 2.45 - 2.36 (m, 1H), 2.34 - 2.22 (m, 1H), 1.87 (d, *J* = 6.5 Hz, 3H), 1.84 (d, *J* = 6.9 Hz, 3H). LCMS (ESI) calcd for C₃₄H₃₉ClN₈O₄P⁺ [M+H]⁺: 689.25; found, 689.2.

### Example 161: Preparation of 3-(6-((4-(4-((5-chloro-4-((2-(dimethylphosphoryl)phenyl)amino)pyrimidin-2-yl)amino)-3-methoxyphenyl)piperazin-1-yl)methyl)pyridin-3-yl)piperidine-2,6-dione (GT-07713)

According to the method of step 2 of Scheme I-1, the target compound (GT-07713) was prepared (white solid, 19 mg, yield 39 %). ¹H NMR (400 MHz, DMSO-d6) δ 12.21 (s, 1H), 11.03 (s, 1H), 9.74 (s, 1H), 8.66 (s, 1H), 8.31 (s, 2H), 7.92 (dd, *J =* 8.0, 2.2 Hz, 1H), 7.79 (d, *J =* 8.0 Hz, 1H), 7.71 (dd, *J =* 13.0, 7.8 Hz, 1H), 7.53 - 7.38 (m, 2H), 7.33 (t, *J* = 7.0 Hz, 1H), 6.82 (d, *J* = 2.3 Hz, 1H), 6.64 (dd, *J* = 8.7, 2.3 Hz, 1H), 4.63 (s, 2H), 4.12 (dd, *J* = 12.7, 4.8 Hz, 2H), 3.86 (s, 3H), 3.65 - 3.64 (m, 2H), 3.50 (s, 5H), 2.85 - 2.75 (m, 1H), 2.70 - 2.62 (m, 1H), 2.43 - 2.32 (m, 1H), 2.17 - 2.07 (m, 1H), 1.88 (s, 3H), 1.85 (s, 3H). LCMS (ESI) calcd for C₃₄H₃₉ClN₈O₄P ⁺ [M+H]⁺: 689.25; found, 689.2.

### Example 162: Preparation of 3-(5-((4-(4-((5-chloro-4-((2-(dimethylphosphoryl)phenyl)amino)pyrimidin-2-yl)amino)-3-methoxyphenyl)piperazin-1-yl)methyl)pyridin-3-yl)piperidine-2,6-dione (GT-07714)

According to the method of step 2 of Scheme I-1, the target compound (GT-07714) was prepared (white solid, 14 mg, yield 28 %). ¹H NMR (400 MHz, DMSO-d6) δ 12.24 (s, 2H), 11.07 (s, 1H), 9.85 (s, 1H), 9.01 (s, 1H), 8.83 (s, 1H), 8.55 (s, 1H), 8.34 (s, 2H), 7.66 (dd, *J* = 12.8, 7.7 Hz, 1H), 7.41 (s, 1H), 7.35 - 7.26 (m, 2H), 6.76 (s, 1H), 6.60 (d, *J* = 8.7, 2.0 Hz, 1H), 4.61 (d, *J* = 7.4 Hz, 2H), 4.20 (dd, *J* = 12.7, 4.8 Hz, 2H), 3.80 (s, 3H), 3.51 - 3.44 (m, 3H), 3.33 - 3.18 (m, 5H), 2.85 - 2.76 (m, 1H), 2.69 - 2.60 (m, 1H), 2.42 - 2.31 (m, 1H), 2.22 - 2.15 (m, 1H), 1.82 (s, 3H), 1.79 (s, 3H). LCMS (ESI) calcd for C₃₄H₃₉ClN₈O₄P ⁺ [M+H]⁺: 689.25; found, 689.2.

### Example 163: Preparation of 1-(5-((4-(4-((5-chloro-4-((2-(dimethylphosphoryl)phenyl)amino)pyrimidin-2-yl)amino)-3-methoxyphenyl)piperazin-1-yl)methyl)-6-fluoropyridin-3-yl)dihydropyrimidine-2,4(1H,3H)-dione (GT-07748)

According to the method of step 2 of Scheme I-1, the target compound (GT-07748) was prepared (white solid, 21 mg, yield 34 %). ¹H NMR (400 MHz, DMSO-d6) δ 11.88 (s, 1H), 11.60 (s, 1H), 10.56 (s, 1H), 9.21 (s, 1H), 8.34 (d, *J* = 4.7 Hz, 3H), 8.17 (s, 1H), 7.55 (dd, *J =* 13.7, 7.8 Hz, 1H), 7.31 (d, *J =* 8.4 Hz, 2H), 7.16 (t, *J =* 7.1 Hz, 1H), 6.68 (d, *J =* 2.0 Hz, 1H), 6.50 (d, *J =* 8.7 Hz, 1H), 4.42 (s, 2H), 3.86 (t, *J =* 6.6 Hz, 3H), 3.82 (s, 1H), 3.72 (s, 4H), 3.45 (s, 2H), 3.20 (s, 4H), 2.70 (t, *J =* 6.6 Hz, 2H), 1.72 (d, *J =* 13.6 Hz, 6H). LCMS (ESI) calcd for C₃₃H₃₇ClFN₉O₄P ⁺ [M+H]⁺: 708.24; found, 708.1.

### Example 164: Preparation of 1-(5-((4-(4-((5-chloro-4-((2-(dimethylphosphoryl)phenyl)amino)pyrimidin-2-yl)amino)-3-methoxyphenyl)piperazin-1-yl)methyl)pyrazin-2-yl)dihydropyrimidine-2,4(1H,3H)-dione (GT-07749)

According to the method of step 2 of Scheme I-1, the target compound (GT-07749) was prepared (white solid, 20 mg, yield 34 %). ¹H NMR (400 MHz, DMSO-d6) δ 12.12 (s, 1H), 11.68 (s, 1H), 10.83 (s, 1H), 9.61 (s, 1H), 9.17 (s, 1H), 8.82 (s, 1H), 8.31 (s, 2H), 7.67 - 7.61 (m, 1H), 7.42 - 7.33 (m, 2H), 7.26 (t, *J* = 7.2 Hz, 1H), 6.76 (d, *J =* 2.2 Hz, 1H), 6.58 (dd, *J =* 8.7, 2.2 Hz, 1H), 4.59 (s, 2H), 4.10 (t, *J =* 6.6 Hz, 4H), 3.80 (s, 3H), 3.56 (s, 2H), 3.26 (s, 4H), 2.75 (t, *J =* 6.6 Hz, 2H), 1.83 - 1.79 (m, 6H). LCMS (ESI) calcd for C₃₂H₃₇ClN₁₀O₄P⁺ [M+H]⁺: 691.24; found, 691.1.

### Example 165: Preparation of 1-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-6-fluoropyridin-3-yl)dihydropyrimidine-2,4(1H,3H)-dione (GT-07750)

According to the method of step 2 of Scheme I-1, the target compound (GT-07750) was prepared (white solid, 17 mg, yield 28 %). ¹H NMR (400 MHz, DMSO-d6) δ 11.96 (s, 1H), 11.24 (s, 1H), 10.56 (s, 1H), 9.41 (s, 1H), 8.38 - 8.30 (m, 3H), 8.22 (s, 1H), 7.58 (dd, *J =* 14.3, 7.3 Hz, 1H), 7.38 (s, 2H), 7.19 (t, *J =* 7.3 Hz, 1H), 6.85 (s, 1H), 6.69 (s, 1H), 4.52 (d, *J =* 12.8 Hz, 1H), 4.24 (s, 1H), 3.86 (t, *J =* 6.6 Hz, 4H), 3.75 (s, 4H), 3.49 (s, 1H), 2.90 (s, 1H), 2.70 (t, *J =* 6.7 Hz, 2H), 2.64 (s, 3H), 2.35 - 2.30 (m, 1H), 2.24 - 2.18 (m, 1H), 2.00 (s, 2H), 1.73 (d, *J =* 13.7 Hz, 6H). LCMS (ESI) calcd for C₃₅H₄₁ClFN₉O₄P⁺ [M+H]⁺: 736.27; found, 736.1.

### Example 166: Preparation of 1-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyrazin-2-yl)dihydropyrimidine-2,4(1H,3H)-dione (GT-07751)

According to the method of step 2 of Scheme I-1, the target compound (GT-07751) was prepared (white solid, 33 mg, yield 56 %). ¹H NMR (400 MHz, DMSO-d6) δ 12.05 (s, 1H), 11.31 (s, 1H), 10.83 (s, 1H), 9.53 (s, 1H), 9.18 (d, *J =* 1.1 Hz, 1H), 8.82 (d, *J =* 1.1 Hz, 1H), 8.32 (d, 2H), 7.65 (dd, *J =* 13.5, 7.6 Hz, 1H), 7.46 (s, 2H), 7.27 (t, *J =* 7.1 Hz, 1H), 6.99 (s, 1H), 6.83 (s, 1H), 4.64 (d, *J =* 13.5 Hz, 1H), 4.50 (d, *J =* 9.9 Hz, 1H), 4.11 (t, *J =* 6.6 Hz, 3H), 3.92 (d, *J =* 9.3 Hz, 3H), 3.83 (s, 3H), 3.56 (s, 1H), 3.03 (s, 1H), 2.79 - 2.73 (m, 5H), 2.40 (s, 1H), 2.29 (s, 1H), 2.12 (s, 1H), 1.81 (d, *J* = 13.7 Hz, 6H). LCMS (ESI) calcd for C₃₄H₄₁ClN₁₀O₄P ⁺ [M+H]⁺: 719.27; found, 719.1.

### Example 167: Preparation of 8-(4-(4-((5-(2,4-dioxotetrahydropyrimidin-1(2H)-yl)-2-fluoropyridin-3-yl)methyl)piperazin-1-yl)piperidin-1-yl)-9-ethyl-6,6-dimethyl-11-oxo-6,11-dihydro-5H-benzo[b]carbazole-3-carbonitrile (GT-07755)

According to the method of step 2 of Scheme I-1, the target compound (GT-07755) was prepared (white solid, 20 mg, yield 33 %). ¹H NMR (400 MHz, DMSO-d6) δ 12.90 (s, 1H), 11.38 (s, 1H), 10.60 (s, 1H), 8.32 (d, *J =* 8.2 Hz, 2H), 8.19 (s, 1H), 8.07 (s, 1H), 8.01 (s, 1H), 7.61 (dd, *J =* 8.2, 1.3 Hz, 1H), 7.37 (s, 1H), 4.12 (s, 2H), 3.93 - 3.87 (m, 6H), 3.70 (d, *J* = 8.5 Hz, 4H), 3.46 - 3.30 (m, 6H), 3.24 - 3.13 (m, 1H), 2.86 - 2.68 (m, 6H), 2.23 (d, *J* = 10.2 Hz, 2H), 1.97 - 1.86 (m, 2H), 1.29 (t, *J* = 7.5 Hz, 3H). LCMS (ESI) calcd for C₄₀H₄₄FN₈O₃ ⁺ [M+H]⁺: 703.35; found, 703.3.

### Example 168: Preparation of 8-(4-(4-((5-(2,4-dioxotetrahydropyrimidin-1(2H)-yl)pyrazin-2-yl)methyl)piperazin-1-yl)piperidin-1-yl)-9-ethyl-6,6-dimethyl-11-oxo-6,11-dihydro-5H-benzo[b]carbazole-3-carbonitrile (GT-07756)

According to the method of step 2 of Scheme I-1, the target compound (GT-07756) was prepared (white solid, 25 mg, yield 43 %). ¹H NMR (400 MHz, DMSO-d6) δ 12.95 (s, 1H), 12.00 (s, 1H), 10.82 (s, 1H), 9.16 (d, *J* = 1.3 Hz, 1H), 8.74 (s, 1H), 8.32 (d, *J* = 8.2 Hz, 1H), 8.07 (s, 1H), 8.01 (s, 1H), 7.61 (d, *J =* 8.2, 1.3 Hz, 1H), 7.37 (s, 1H), 4.53 (s, 2H), 4.09 (d, *J* = 6.7 Hz, 2H), 3.79 - 3.64 (m, 9H), 3.62 - 3.43 (m, 6H), 3.33 (d, *J =* 11.4 Hz, 2H), 2.84 (t, *J =* 11.4 Hz, 2H), 2.78 - 2.70 (m, 4H), 2.24 (d, *J =* 10.6 Hz, 2H), 2.00 - 1.87 (m, 2H), 1.29 (t, *J =* 7.5 Hz, 3H). LCMS (ESI) calcd for C₃₉H₄₄N₉O₃⁺ [M+H]⁺: 686.36; found, 686.2.

### Example 169: Preparation of 1-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)-6-fluoropyridin-3-yl)dihydropyrimidine-2,4(1H,3H)-dione (GT-07757)

According to the method of step 2 of Scheme I-1, the target compound (GT-07757) was prepared (white solid, 29 mg, yield 49 %). ¹H NMR (400 MHz, DMSO-d6) δ 11.01 (s, 1H), 10.64 (s, 1H), 9.69 (s, 1H), 8.50 (s, 1H), 8.43 - 8.29 (m, 4H), 7.87 (d, *J =* 7.9, 1.4 Hz, 1H), 7.66 (t, *J =* 7.4 Hz, 1H), 7.46 - 7.38 (m, 2H), 6.81 (s, 1H), 4.51 - 4.44 (m, 1H), 4.41 (d, *J* = 4.2 Hz, 1H), 3.94 (t, *J =* 6.6 Hz, 2H), 3.55 - 3.40 (m, 4H), 3.27 - 3.17 (m, 2H), 2.99 (t, *J =* 12.0 Hz, 1H), 2.78 (t, *J =* 6.6 Hz, 2H), 2.19 - 2.06 (m, 5H), 1.87 (d, *J =* 12.8 Hz, 2H), 1.23 (d, *J* = 6.0 Hz, 6H), 1.15 (d, *J* = 6.8 Hz, 6H). LCMS (ESI) calcd for C₃₈H₄₅ClFN₈O₅S⁺ [M+H]⁺: 779.29; found, 779.1.

### Example 170: Preparation of 1-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)pyrazin-2-yl)dihydropyrimidine-2,4(1H,3H)-dione (GT-07758)

According to the method of step 2 of Scheme I-1, the target compound (GT-07758) was prepared (white solid, 19 mg, yield 33 %). ¹H NMR (400 MHz, DMSO-d6) δ 11.04 (s, 1H), 10.83 (s, 1H), 9.81 (s, 1H), 9.17 (s, 1H), 8.80 (s, 1H), 8.73 (s, 1H), 8.39 (s, 1H), 8.27 (d, *J =* 7.6 Hz, 1H), 7.89 (dd, *J =* 8.0, 1.4 Hz, 1H), 7.68 (t, *J* = 7.1 Hz, 1H), 7.48 - 7.38 (m, 2H), 6.81 (s, 1H), 4.53 - 4.47 (m, 3H), 4.10 (t, *J* = 6.6 Hz, 2H), 3.53 (t, *J* = 11.2 Hz, 2H), 3.49 - 3.44 (m, 1H), 3.29 - 3.11 (m, 2H), 3.01 - 2.92 (m, 1H), 2.75 (t, *J =* 6.5 Hz, 2H), 2.16 - 2.11 (m, 1H), 2.08 (s, 3H), 1.90 - 1.82 (m, 2H), 1.25 (d, *J =* 6.0, 3.3 Hz, 6H), 1.14 (d, *J =* 6.8 Hz, 6H). LCMS (ESI) calcd for C₃₇H₄₅ClN₉O₅S ⁺ [M+H]⁺: 762.29; found, 762.2.

### Example 171: Preparation of 1-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-6-fluoropyridin-3-yl)dihydropyrimidine-2,4(1H,3H)-dione (GT-07759)

According to the method of step 2 of Scheme I-1, the target compound (GT-07759) was prepared (white solid, 35 mg, yield 61 %). ¹H NMR (400 MHz, DMSO-d6) δ 12.12 (s, 1H), 10.61 (s, 1H), 9.64 (s, 1H), 8.47 - 8.19 (m, 4H), 7.66 (dd, *J =* 13.5, 7.5 Hz, 1H), 7.46 (s, 2H), 7.28 (t, *J =* 7.3 Hz, 1H), 6.98 (s, 1H), 6.80 (s, 1H), 4.31 (s, 2H), 3.90 (t, *J =* 6.6 Hz, 5H), 3.82 (s, 3H), 3.71 (s, 2H), 3.53 (s, 6H), 3.00 (s, 2H), 2.76 (t, *J* = 6.6 Hz, 2H), 2.27 (d, *J* = 9.7 Hz, 2H), 2.01 (s, 2H), 1.83 (s, 3H), 1.79 (s, 3H). LCMS (ESI) calcd for C₃₈H₄₆ClFN₁₀O₄P ⁺ [M+H]⁺: 791.31; found, 791.2.

### Example 172: Preparation of 1-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyrazin-2-yl)dihydropyrimidine-2,4(1H,3H)-dione (GT-07822)

According to the method of step 2 of Scheme I-1, the target compound (GT-07822) was prepared (white solid, 41 mg, yield 73 %). ¹H NMR (400 MHz, DMSO-d6) δ 12.12 (s, 1H), 10.82 (s, 1H), 9.69 (s, 1H), 9.16 (d, *J =* 1.4 Hz, 1H), 8.77 (d, *J =* 1.3 Hz, 1H), 8.35 (s, 2H), 7.66 (dd, *J =* 13.3, 7.2 Hz, 1H), 7.46 (s, 2H), 7.29 (t, *J* = 7.2 Hz, 1H), 7.01 (s, 1H), 6.84 (s, 1H), 4.58 (s, 2H), 4.10 (t, *J =* 6.6 Hz, 3H), 3.92 (d, *J =* 10.9 Hz, 3H), 3.82 (s, 3H), 3.75 (s, 3H), 3.64 (s, 4H), 3.04 (s, 2H), 2.75 (t, *J =* 6.6 Hz, 2H), 2.32 - 2.22 (m, 2H), 2.12 - 1.95 (m, 2H), 1.81 (d, *J =* 13.7 Hz, 6H). LCMS (ESI) calcd for C₃₇H₄₆ClN₁₁O₄P⁺ [M+H]⁺: 774.32; found, 774.2.

### Example 173: Preparation of 8-(4-((2-(2,4-dioxotetrahydropyrimidin-1(2H)-yl)-5-fluoropyridin-4-yl)methyl)piperazin-1-yl)-9-ethyl-6,6-dimethyl-11-oxo-6,11-dihydro-5H-benzo[b]carbazole-3-carbonitrile (GT-07833)

According to the method of step 2 of Scheme I-1, the target compound (GT-07833) was prepared (white solid, 17 mg, yield 38 %). ¹H NMR (400 MHz, DMSO-d6) δ 12.97 (s, 1H), 11.24 (s, 1H), 10.68 (s, 1H), 8.59 (s, 1H), 8.32 (d, *J =* 8.2 Hz, 1H), 8.18 (d, *J =* 5.2 Hz, 1H), 8.09 (s, 1H), 8.02 (s, 1H), 7.61 (dd, *J =* 8.2, 1.4 Hz, 1H), 7.37 (s, 1H), 4.58 (s, 2H), 4.06 (t, *J =* 6.6 Hz, 2H), 3.51 - 3.38 (m, 4H), 3.37 - 3.26 (m, 4H), 2.78 - 2.69 (m, 4H), 1.78 (s, 6H), 1.28 (t, 3H). LCMS (ESI) calcd for C₃₅H₃₅FN₇O₃⁺ [M+H]⁺: 620.28; found, 620.2.

### Example 174: Preparation of 8-(4-((2-(2,4-dioxotetrahydropyrimidin-1(2H)-yl)pyridin-4-yl)methyl)piperazin-1-yl)-9-ethyl-6,6-dimethyl-11-oxo-6,11-dihydro-5H-benzo[b]carbazole-3-carbonitrile (GT-07834)

According to the method of step 2 of Scheme I-1, the target compound (GT-07834) was prepared (white solid, 20 mg, yield 45 %). ¹H NMR (400 MHz, DMSO-d6) δ 12.95 (s, 1H), 11.47 (s, 1H), 10.65 (s, 1H), 8.55 (d, *J* = 5.1 Hz, 1H), 8.32 (d, *J* = 8.2 Hz, 1H), 8.09 (s, 1H), 8.02 (d, *J* = 8.1 Hz, 2H), 7.66 - 7.59 (m, 2H), 7.38 (s, 1H), 4.53 (s, 2H), 4.11 (t, *J* = 6.6 Hz, 2H), 3.44 (s, 2H), 3.33 (s, 6H), 2.77 - 2.68 (m, 4H), 1.78 (s, 6H), 1.29 (t, *J* = 7.5 Hz, 3H). LCMS (ESI) calcd for C₃₅H₃₆N₇O₃ ⁺ [M+H]⁺: 602.29; found, 602.2.

### Example 175: Preparation of 8-(4-((5-(2,4-dioxotetrahydropyrimidin-1(2H)-yl)pyridin-3-yl)methyl)piperazin-1-yl)-9-ethyl-6,6-dimethyl-11-oxo-6,11-dihydro-5H-benzo[b]carbazole-3-carbonitrile (GT-07835)

According to the method of step 2 of Scheme I-1, the target compound (GT-07835) was prepared (white solid, 20 mg, yield 45 %). ¹H NMR (400 MHz, DMSO-d6) δ 12.96 (s, 1H), 11.76 (s, 1H), 10.68 (s, 1H), 8.82 (d, *J* = 2.4 Hz, 1H), 8.74 (d, *J* = 1.6 Hz, 1H), 8.39 - 8.28 (m, 2H), 8.09 (s, 1H), 8.01 (s, 1H), 7.61 (d, *J* = 8.2, 1.4 Hz, 1H), 7.38 (s, 1H), 4.57 (s, 2H), 3.98 (t, *J =* 6.6 Hz, 2H), 3.46 (s, 2H), 3.34 (s, 6H), 2.81 - 2.69 (m, 4H), 1.78 (s, 6H), 1.30 (t, *J* = 7.5 Hz, 3H). LCMS (ESI) calcd for C₃₅H₃₆N₇O₃⁺ [M+H]⁺: 602.29; found, 602.2.

### Example 176: Preparation of 8-(4-((5-(2,6-dioxopiperidin-3-yl)-2-fluoropyridin-3-yl)methyl)piperazin-1-yl)-9-ethyl-6,6-dimethyl-11-oxo-6,11-dihydro-5H-benzo[b]carbazole-3-carbonitrile (GT-07836)

According to the method of step 2 of Scheme I-1, the target compound (GT-07836) was prepared (yellow solid, 10 mg, yield 22 %). ¹H NMR (400 MHz, DMSO-d6) δ 12.92 (s, 1H), 11.31 (s, 1H), 11.01 (s, 1H), 8.32 (d, *J* = 8.1 Hz, 2H), 8.27 (s, 1H), 8.09 (s, 1H), 8.01 (s, 1H), 7.61 (d, *J* = 8.2, 1.3 Hz, 1H), 7.38 (s, 1H), 4.52 (s, 2H), 4.08 (dd, *J* = 12.5, 4.8 Hz, 1H), 3.55 - 3.52 (m, 2H), 3.43 - 3.27 (m, 6H), 2.80 - 2.68 (m, 3H), 2.68 - 2.58 (m, 1H), 2.40 - 2.24 (m, 1H), 2.19 - 2.08 (m, 1H), 1.78 (s, 6H), 1.30 (t, *J* = 7.5 Hz, 3H). LCMS (ESI) calcd for C₃₆H₃₆FN₆O₃⁺ [M+H]⁺: 619.28; found, 619.2.

### Example 177: Preparation of 8-(4-(4-((6-(2,4-dioxotetrahydropyrimidin-1(2H)-yl)pyrazin-2-yl)methyl)piperazin-1-yl)piperidin-1-yl)-9-ethyl-6,6-dimethyl-11-oxo-6,11-dihydro-5H-benzo[b]carbazole-3-carbonitrile (GT-07923)

According to the method of step 2 of Scheme I-1, the target compound (GT-07923) was prepared (yellow solid, 23 mg, yield 39 %). ¹H NMR (400 MHz, DMSO-d6) δ 13.01 (s, 1H), 12.32 (s, 1H), 10.81 (s, 1H), 9.13 (s, 1H), 8.61 (s, 1H), 8.33 (d, J = 8.5 Hz, 1H), 8.06 (s, 1H), 8.01 (s, 1H), 7.60 (d, J = 8.2, 1.3 Hz, 1H), 7.38 (s, 1H), 4.60 (s, 2H), 4.25 - 4.21 (m, 3H), 3.77 (d, J = 31.3 Hz, 7H), 3.49 (s, 1H), 3.33 (d, J = 10.5 Hz, 2H), 2.85 (t, J = 11.5 Hz, 2H), 2.77 - 2.70 (m, 4H), 2.26 (d, J = 9.9 Hz, 2H), 1.98 - 1.90 (m, 2H), 1.77 (s, 6H), 1.29 (t, J = 7.5 Hz, 3H). LCMS (ESI) calcd for C₃₉H₄₄N₉O₃ ⁺ [M+H]⁺: 686.36; found, 686.3.

### Example 178: Preparation of 8-(4-(4-((5-(2,4-dioxotetrahydropyrimidin-1(2H)-yl)-3-fluoropyridin-2-yl)methyl)piperazin-1-yl)piperidin-1-yl)-9-ethyl-6,6-dimethyl-11-oxo-6,11-dihydro-5H-benzo[b]carbazole-3-carbonitrile (GT-07924)

According to the method of step 2 of Scheme I-1, the target compound (GT-07924) was prepared (yellow solid, 28 mg, yield 47 %). ¹H NMR (400 MHz, DMSO-d6) δ 13.06 (s, 1H), 12.23 (s, 1H), 10.69 (s, 1H), 8.62 (d, J = 0.9 Hz, 1H), 8.32 (d, J = 8.2 Hz, 1H), 8.07 (s, 1H), 8.01 (s, 1H), 7.96 (dd, J = 11.1, 2.0 Hz, 1H), 7.60 (dd, J = 8.2, 1.3 Hz, 1H), 7.38 (s, 1H), 4.59 (s, 2H), 3.92 (t, J = 6.6 Hz, 2H), 3.81 (s, 4H), 3.73 - 3.58 (m, 4H), 3.50 (s, 1H), 3.34 (d, J = 11.3 Hz, 2H), 2.85 (t, J = 11.6 Hz, 2H), 2.80 - 2.69 (m, 4H), 2.25 (d, J = 9.5 Hz, 2H), 1.95 (d, J = 9.8 Hz, 2H), 1.78 (s, 6H), 1.29 (t, J = 7.5 Hz, 3H). LCMS (ESI) calcd for C₄₀H₄₄FN₈O₃ ⁺ [M+H]⁺: 703.35; found, 703.3.

### Example 179: Preparation of 8-(4-(4-(4-(2,4-dioxotetrahydropyrimidin-1(2H)-yl)-2-fluorobenzyl)piperazin-1-yl)piperidin-1-yl)-9-ethyl-6,6-dimethyl-11-oxo-6,11-dihydro-5H-benzo[b]carbazole-3-carbonitrile (GT-07309)

According to the method of step 2 of Scheme I-1, the target compound (GT-07309) was prepared (white solid, 27 mg, yield 48 %). ¹H NMR (400 MHz, DMSO-d6) δ 12.90 (s, 1H), 12.53 - 11.23 (m, 1H), 10.92 (s, 1H), 8.32 (d, J = 8.2 Hz, 1H), 8.06 (s, 1H), 8.01 (s, 1H), 7.61 (dd, J = 8.2, 1.2 Hz, 1H), 7.42 (s, 1H), 7.40 (s, 1H), 7.36 (s, 1H), 4.15 (s, 1H), 4.11 (dd, J = 12.6, 4.7 Hz, 1H), 3.68 (s, 2H), 3.48 - 3.44 (m, 2H), 3.32 (d, J = 11.3 Hz, 4H), 3.24 - 2.95 (m, 2H), 2.83 (t, J = 11.4 Hz, 2H), 2.79 - 2.74 (m, 1H), 2.74 - 2.68 (m, 2H), 2.62 - 2.55 (m, 1H), 2.52 (s, 1H), 2.30 - 2.13 (m, 3H), 2.04 - 1.97 (m, 1H), 1.91 (d, J = 11.2 Hz, 2H), 1.83 - 1.74 (m, 6H), 1.29 (t, J = 7.5 Hz, 3H). LCMS (ESI) calcd for C₄₁H₄₅FN₇O₃ ⁺ [M+H]⁺: 702.36; found, 702.4.

### Example 180: Preparation of 8-(4-(4-(4-(2,4-dioxotetrahydropyrimidin-1(2H)-yl)-3-fluorobenzyl)piperazin-1-yl)piperidin-1-yl)-9-ethyl-6,6-dimethyl-11-oxo-6,11-dihydro-5H-benzo[b]carbazole-3-carbonitrile (GT-07308)

According to the method of step 2 of Scheme I-1, the target compound (GT-07308) was prepared (white solid, 19 mg, yield 34 %). ¹H NMR (400 MHz, DMSO-d6) δ 12.87 (s, 1H), 12.44 - 11.20 (m, 1H), 10.53 (s, 1H), 8.44 - 8.24 (m, 1H), 8.06 (s, 1H), 8.01 (s, 1H), 7.61 (d, J = 8.2, 1.3 Hz, 1H), 7.56 - 7.41 (m, 2H), 7.39 - 7.33 (m, 1H), 4.27 (s, 2H), 3.82 (s, 1H), 3.74 (t, J = 6.6 Hz, 4H), 3.52 - 3.47 (m, 4H), 3.32 (d, J = 10.8 Hz, 3H), 3.27 - 2.92 (m, 2H), 2.83 (t, J = 11.5 Hz, 2H), 2.77 - 2.67 (m, 4H), 2.46 - 2.04 (m, 2H), 2.00 - 1.83 (m, 2H), 1.84 - 1.68 (m, 6H), 1.29 (t, J = 7.5 Hz, 2H). LCMS (ESI) calcd for C₄₁H₄₅FN₇O₃ ⁺ [M+H]⁺: 702.36; found, 702.4.

### Example 181: Preparation of 1-(4-((4-(4-((5-chloro-4-((2-(dimethylphosphoryl)phenyl)amino)pyrimidin-2-yl)amino)-3-methoxyphenyl)piperazin-1-yl)methyl)-2-fluorophenyl)dihydropyrimidine-2,4(1H,3H)-dione (GT-07346)

According to the method of step 2 of Scheme I-1, the target compound (GT-07346) was prepared (white solid, 21 mg, yield 42 %). ¹H NMR (400 MHz, DMSO-d6) δ 12.18 (s, 1H), 11.94 (s, 1H), 10.56 (s, 1H), 9.70 (s, 1H), 8.31 (s, 1H), 7.78 (d, J = 11.2 Hz, 1H), 7.65 (dd, J = 13.5, 7.8 Hz, 1H), 7.59 - 7.51 (m, 2H), 7.41 (s, 1H), 7.35 (d, J = 8.5 Hz, 1H), 7.27 (t, J = 7.4 Hz, 1H), 6.75 (s, 1H), 6.58 (d, J = 8.7 Hz, 1H), 4.43 (s, 2H), 3.89 (d, 2H), 3.79 (s, 2H), 3.76 (t, J = 6.6 Hz, 3H), 3.40 (d, J = 10.9 Hz, 2H), 3.31 (t, J = 11.9 Hz, 2H), 3.24 - 3.12 (m, 2H), 2.74 (t, J = 6.6 Hz, 2H), 1.80 (d, J = 13.7 Hz, 6H). LCMS (ESI) calcd for C₃₄H₃₈ClFN₈O₄P⁺ [M+H]⁺: 707.24; found, 707.3.

### Example 182: Preparation of 1-(4-((4-(4-((5-chloro-4-((2-(dimethylphosphoryl)phenyl)amino)pyrimidin-2-yl)amino)-3-methoxyphenyl)piperazin-1-yl)methyl)-3-fluorophenyl)dihydropyrimidine-2,4(1H,3H)-dione (GT-07347)

According to the method of step 2 of Scheme I-1, the target compound (GT-07347) was prepared (white solid, 24 mg, yield 47 %). ¹H NMR (400 MHz, DMSO-d6) δ 11.85 (s, 1H), 11.42 (s, 1H), 10.54 (s, 1H), 9.15 (s, 1H), 8.39 (s, 1H), 8.20 (d, J = 17.3 Hz, 1H), 7.84 (t, J = 8.5 Hz, 1H), 7.61 (dd, J = 13.9, 7.5 Hz, 1H), 7.46 - 7.36 (m, 3H), 7.33 (d, J = 8.4, 1.9 Hz, 1H), 7.21 (t, J = 7.4 Hz, 1H), 6.74 (d, J = 2.2 Hz, 1H), 6.55 (d, J = 8.7, 2.2 Hz, 1H), 4.45 (s, 2H), 3.89 (s, 1H), 3.87 (t, J = 6.6 Hz, 3H), 3.79 (s, 3H), 3.48 (s, 2H), 3.28 - 3.18 (m, 4H), 2.73 (t, J = 6.6 Hz, 2H), 1.79 (d, J = 13.6 Hz, 6H). LCMS (ESI) calcd for C₃₄H₃₈ClFN₈O₄P⁺ [M+H]⁺: 707.24 ; found, 707.3.

### Example 183: Preparation of 3-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)-2-fluorophenyl)piperidine-2,6-dione (GT-07365)

According to the method of step 2 of Scheme I-1, the target compound (GT-07365) was prepared (white solid, 26 mg, yield 44 %). ¹H NMR (500 MHz, DMSO) δ 10.88 (s, 2H), 10.80 (s, 1H), 9.91 (s, 1H), 8.92 (s, 1H), 8.42 (s, 1H), 8.20 (s, 1H), 7.92 (d, J = 16.3 Hz, 1H), 7.70 (s, 1H), 7.62 (d, J = 7.5 Hz, 2H), 7.48 (s, 1H), 7.34 (t, J = 7.8 Hz, 2H), 7.14 (d, J = 7.5 Hz, 1H), 7.10 (d, J = 7.7 Hz, 1H), 6.84 (s, 1H), 4.54 - 4.47 (m, 1H), 4.29 (s, 2H), 3.95 - 3.92 (m, 2H), 3.82 - 3.78 (m, 1H), 3.47 (t, 1H), 3.40 (d, J = 10.7 Hz, 2H), 3.07 (d, J = 11.0 Hz, 2H), 2.97 - 2.90 (m, 1H), 2.75 - 2.61 (m, 2H), 2.28 (s, 2H), 2.20 - 2.11 (m, 3H), 2.06 (s, 4H), 1.84 (d, J = 12.5 Hz, 2H), 1.31 - 1.27 (m, 2H), 1.23 (d, J = 5.7 Hz, 3H), 1.13 (d, J = 6.4 Hz, 3H). LCMS (ESI) calcd for C₄₀H₄₇ClFN₆O₅S⁺ [M+H]⁺: 777.30; found, 777.3.

### Example 184: Preparation of 1-(6-((4-(4-((5-chloro-4-((2-(dimethylphosphoryl)phenyl)amino)pyrimidin-2-yl)amino)-3-methoxyphenyl)piperazin-1-yl)methyl)pyrazin-2-yl)dihydropyrimidine-2,4(1H,3H)-dione (GT-08012)

According to the method of step 2 of Scheme I-1, the target compound (GT-08012) was prepared (gray solid, 17 mg, yield 28 %). ¹H NMR (400 MHz, DMSO-d6) δ 12.19 (s, 1H), 11.88 (s, 1H), 10.81 (s, 1H), 9.74 (s, 1H), 9.13 (s, 1H), 8.64 (s, 1H), 8.26 (s, 2H), 7.65 (dd, J = 13.7, 7.7 Hz, 1H), 7.47 - 7.32 (m, 2H), 7.26 (t, J = 7.5 Hz, 1H), 6.76 (s, 1H), 6.59 (d, J = 8.6 Hz, 1H), 4.58 (s, 2H), 4.16 (t, J = 6.5 Hz, 2H), 3.90 (s, 2H), 3.80 (s, 3H), 3.63 (s, 2H), 3.34 (s, 4H), 2.73 (t, J = 6.5 Hz, 2H), 1.80 (d, J = 13.7 Hz, 6H). LCMS (ESI) calcd for C₃₂H₃₇ClN₁₀O₄P ⁺ [M+H]⁺: 691.24; found, 691.2.

### Example 185: Preparation of 1-(6-((4-(4-((5-chloro-4-((2-(dimethylphosphoryl)phenyl)amino)pyrimidin-2-yl)amino)-3-methoxyphenyl)piperazin-1-yl)methyl)-5-fluoropyridin-3-yl)dihydropyrimidine-2,4(1H,3H)-dione (GT-08013)

According to the method of step 2 of Scheme I-1, the target compound (GT-08013) was prepared (gray solid, 25 mg, yield 41 %). ¹H NMR (400 MHz, DMSO-d6) δ 12.18 (s, 1H), 11.34 (s, 1H), 10.69 (s, 1H), 9.75 (s, 1H), 8.64 (s, 1H), 8.25 (s, 2H), 7.97 (d, 1H), 7.73 - 7.55 (m, 1H), 7.48 - 7.32 (m, 2H), 7.27 (t, J = 7.2 Hz, 1H), 6.77 (s, 1H), 6.59 (d, J = 8.6 Hz, 1H), 4.62 (s, 2H), 3.95 - 3.91 (m, 4H), 3.80 (s, 3H), 3.67 - 3.60 (m, 2H), 3.34 (d, J = 41.8 Hz, 4H), 2.77 (t, J = 6.5 Hz, 2H), 1.81 (d, J = 13.7 Hz, 6H). LCMS (ESI) calcd for C₃₃H₃₇ClFN₉O₄P⁺ [M+H]⁺: 708.24; found, 708.1.

### Example 186: Preparation of 1-(6-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyrazin-2-yl)dihydropyrimidine-2,4(1H,3H)-dione (GT-08014)

According to the method of step 2 of Scheme I-1, the target compound (GT-08014) was prepared (white solid, 34 mg, yield 61 %). ¹H NMR (400 MHz, DMSO-d6) δ 12.37 (s, 1H), 12.13 (s, 1H), 10.81 (s, 1H), 9.72 (s, 1H), 9.13 (s, 1H), 8.60 (s, 1H), 8.35 (s, 2H), 7.69 - 7.58 (m, 1H), 7.47 (s, 2H), 7.29 (t, J = 7.2 Hz, 1H), 6.94 (d, J = 71.1 Hz, 2H), 4.58 (s, 2H), 4.23 (t, J = 6.5 Hz, 3H), 3.92 (d, J = 10.6 Hz, 3H), 3.82 (s, 3H), 3.75 (s, 4H), 3.70 - 3.65 (m, 2H), 3.59 - 3.53 (m, 1H), 3.05 (s, 2H), 2.73 (t, J = 6.5 Hz, 2H), 2.30 (d, J = 9.8 Hz, 2H), 2.06 (s, 2H), 1.81 (d, J = 13.7 Hz, 6H). LCMS (ESI) calcd for C₃₇H₄₆ClN₁₁O₄P ⁺ [M+H]⁺: 774.32; found, 774.2.

### Example 187: Preparation of 1-(6-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-5-fluoropyridin-3-yl)dihydropyrimidine-2,4(1H,3H)-dione (GT-08015)

According to the method of step 2 of Scheme I-1, the target compound (GT-08015) was prepared (white solid, 39 mg, yield 68 %). ¹H NMR (400 MHz, DMSO-d6) δ 12.10 (s, 1H), 10.68 (s, 1H), 9.65 (s, 1H), 8.60 (s, 1H), 8.33 (s, 2H), 7.95 (dd, J = 11.1, 2.0 Hz, 1H), 7.66 (dd, J = 13.6, 7.5 Hz, 1H), 7.46 (s, 2H), 7.28 (t, J = 7.2 Hz, 1H), 6.99 (s, 1H), 6.82 (s, 1H), 4.54 (s, 3H), 3.91 (t, 6H), 3.82 (s, 3H), 3.76 (s, 3H), 3.59 (s, 3H), 3.02 (s, 2H), 2.76 (t, J = 6.6 Hz, 2H), 2.34 - 2.25 (m, 2H), 2.03 (s, 2H), 1.80 (d, J = 13.7 Hz, 6H). LCMS (ESI) calcd for C₃₈H₄₆ClFN₁₀O₄P ⁺ [M+H]⁺: 791.31; found, 791.2.

### Example 188: Preparation of 1-(6-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyrazin-2-yl)dihydropyrimidine-2,4(1H,3H)-dione (GT-08016)

According to the method of step 2 of Scheme I-1, the target compound (GT-08016) was prepared (white solid, 38 mg, yield 64 %). ¹H NMR (400 MHz, DMSO-d6) δ 12.08 (s, 1H), 11.56 (s, 1H), 10.82 (s, 1H), 9.62 (s, 1H), 9.15 (s, 1H), 8.65 (s, 1H), 8.34 (s, 2H), 7.65 (dd, J = 13.5, 7.7 Hz, 1H), 7.52 - 7.42 (m, 2H), 7.27 (t, J = 7.2 Hz, 1H), 7.05 (s, 1H), 6.87 (s, 1H), 4.62 - 4.51 (m, 2H), 4.24 - 4.21 (m, 1H), 4.16 - 4.14 (m, 1H), 3.89 (d, 3H), 3.83 (s, 3H), 3.63 (s, 2H), 3.09 (s, 2H), 2.82 (s, 3H), 2.75 (t, J = 6.7 Hz, 2H), 2.45 (s, 1H), 2.32 (d, J = 9.6 Hz, 1H), 2.16 (s, 2H), 1.81 (d, J = 13.7 Hz, 6H). LCMS (ESI) calcd for C₃₄H₄₁ClN₁₀O₄P ⁺ [M+H]⁺: 719.27; found, 719.1.

### Example 189: Preparation of 1-(6-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-5-fluoropyridin-3-yl)dihydropyrimidine-2,4(1H,3H)-dione (GT-08017)

According to the method of step 2 of Scheme I-1, the target compound (GT-08017) was prepared (white solid, 49 mg, yield 81 %). ¹H NMR (400 MHz, DMSO-d6) δ 12.07 (s, 1H), 10.98 (s, 1H), 10.69 (s, 1H), 9.63 (s, 1H), 8.63 (s, 1H), 8.35 (s, 2H), 7.97 (dd, J = 11.2, 2.0 Hz, 1H), 7.66 (dd, J = 13.3, 7.2 Hz, 1H), 7.57 - 7.39 (m, 2H), 7.27 (t, J = 7.3 Hz, 1H), 7.07 (s, 1H), 6.89 (s, 1H), 4.67 (s, 1H), 4.48 (s, 1H), 3.92 (t, J = 6.3 Hz, 4H), 3.90 (s, 2H), 3.83 (s, 3H), 3.68 - 3.64 (m, 1H), 3.15 - 3.07 (m, 1H), 2.87 (d, J = 11.7 Hz, 3H), 2.77 (t, J = 6.6 Hz, 2H), 2.42 - 2.27 (m, 2H), 2.18 (s, 1H), 1.80 (d, J = 13.7 Hz, 6H). LCMS (ESI) calcd for C₃₅H₄₁ClFN₉O₄P ⁺ [M+H]⁺: 736.27; found, 736.1.

### Example 190: Preparation of 1-(6-((4-(4-((5-chloro-4-((2-(isopropylsulfonyl)phenyl)amino)pyrimidin-2-yl)amino)-5-isopropoxy-2-methylphenyl)piperidin-1-yl)methyl)pyrazin-2-yl)dihydropyrimidine-2,4(1H,3H)-dione (GT-08018)

According to the method of step 2 of Scheme I-1, the target compound (GT-08018) was prepared (white solid, 35 mg, yield 61 %). ¹H NMR (400 MHz, DMSO-d6) δ 11.24 (s, 1H), 10.81 (s, 1H), 9.87 (s, 1H), 9.14 (s, 1H), 8.83 (s, 1H), 8.65 (s, 1H), 8.42 (s, 1H), 8.24 (d, J = 7.8 Hz, 1H), 7.89 (d, J = 7.9, 1.3 Hz, 1H), 7.69 (t, J = 7.7 Hz, 1H), 7.47 (t, J = 7.6 Hz, 1H), 7.41 - 7.37 (m, 1H), 6.85 (s, 1H), 4.56 - 4.42 (m, 3H), 4.17 (t, J = 6.5 Hz, 2H), 3.58 (d, J = 11.0 Hz, 2H), 3.49 - 3.44 (m, 1H), 3.29 - 3.17 (m, 2H), 2.98 (d, J = 12.6 Hz, 1H), 2.75 (t, J = 6.6 Hz, 2H), 2.27 - 2.14 (m, 2H), 2.06 (s, 3H), 1.88 - 1.80 (m, 2H), 1.24 (d, J = 6.0 Hz, 6H), 1.14 (d, J = 6.8 Hz, 6H). LCMS (ESI) calcd for C₃₇H₄₅ClN₉O₅S ⁺ [M+H]⁺: 762.29; found, 762.2.

### Example 191: Preparation of 1-(6-((4-(4-((5-chloro-4-((2-(isopropylsulfonyl)phenyl)amino)pyrimidin-2-yl)amino)-5-isopropoxy-2-methylphenyl)piperidin-1-yl)methyl)-5-fluoropyridin-3-yl)dihydropyrimidine-2,4(1H,3H)-dione (GT-08019)

According to the method of step 2 of Scheme I-1, the target compound (GT-08019) was prepared (white solid, 42 mg, yield 72 %). ¹H NMR (400 MHz, DMSO-d6) δ 11.01 (s, 1H), 10.64 (s, 1H), 9.69 (s, 1H), 8.50 (s, 1H), 8.41 - 8.25 (m, 4H), 7.87 (d, J = 7.9, 1.4 Hz, 1H), 7.66 (t, J = 7.4 Hz, 1H), 7.50 - 7.34 (m, 2H), 6.81 (s, 1H), 4.53 - 4.44 (m, 1H), 4.41 (d, J = 4.2 Hz, 2H), 3.94 (t, J = 6.6 Hz, 2H), 3.51 (d, J = 12.8 Hz, 2H), 3.48 - 3.44 (m, 1H), 3.27 - 3.17 (m, 2H), 2.99 (t, J = 12.0 Hz, 1H), 2.78 (t, J = 6.6 Hz, 2H), 2.20 - 2.14 (m, 1H), 2.11 (s, 4H), 1.87 (d, J = 12.8 Hz, 2H), 1.23 (d, J = 6.0 Hz, 6H), 1.15 (d, J = 6.8 Hz, 6H). LCMS (ESI) calcd for C₃₈H₄₅ClFN₈O₅S ⁺ [M+H]⁺: 779.29; found, 779.2.

### Example 192: Preparation of 8-(4-(4-((2-(2,6-dioxopiperidin-3-yl)-5-fluoropyridin-4-yl)methyl)piperazin-1-yl)piperidin-1-yl)-9-ethyl-6,6-dimethyl-11-oxo-6,11-dihydro-5H-benzo[b]carbazole-3-carbonitrile (GT-08073)

According to the method of step 2 of Scheme I-1, the target compound (GT-08073) was prepared (brown solid, 31 mg, yield 52 %). ¹H NMR (400 MHz, DMSO-d6) δ 12.96 (s, 1H), 11.62 (s, 1H), 10.92 (s, 1H), 8.60 (s, 1H), 8.32 (d, *J =* 8.2 Hz, 1H), 8.06 (s, 1H), 8.01 (s, 1H), 7.72 (d, *J =* 5.4 Hz, 1H), 7.60 (dd, *J =* 8.2, 1.2 Hz, 1H), 7.37 (s, 1H), 4.21 (s, 3H), 4.11 - 4.07 (m, 2H), 3.86 - 3.81 (m, 3H), 3.71 (d, *J =* 10.9 Hz, 3H), 3.64 - 3.53 (m, 1H), 3.44 (s, 5H), 3.32 (d, *J =* 11.5 Hz, 2H), 3.27 - 3.21 (m, 1H), 2.83 (t, *J =* 11.5 Hz, 2H), 2.74 - 2.68 (m, 2H), 2.67 - 2.60 (m, 1H), 2.58 - 2.53 (m, 1H), 2.35 - 2.15 (m, 4H), 2.00 - 1.87 (m, 2H), 1.29 (t, *J =* 7.5 Hz, 3H). LCMS (ESI) calcd for C₄₁H₄₅FN₇O₃⁺ [M+H]⁺: 702.36; found, 702.3.

### Example 193: Preparation of 3-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)-5-fluoropyridin-2-yl)piperidine-2,6-dione (GT-08074)

According to the method of step 2 of Scheme I-1, the target compound (GT-08074) was prepared (white solid, 40 mg, yield 68 %). ¹H NMR (400 MHz, DMSO-d6) δ 11.34 (s, 1H), 10.93 (s, 1H), 9.81 (s, 1H), 8.69 (s, 1H), 8.66 (s, 1H), 8.39 (s, 1H), 8.27 (d, J = 7.6 Hz, 1H), 7.95 - 7.86 (m, 2H), 7.68 (t, J = 7.8 Hz, 1H), 7.45 (t, J = 7.6 Hz, 1H), 7.40 (s, 1H), 6.83 (s, 1H), 4.52 - 4.46 (m, 1H), 4.44 (s, 1H), 4.09 (dd, J = 9.8, 5.2 Hz, 1H), 3.74 (s, 2H), 3.61 - 3.54 (m, 3H), 3.54 - 3.43 (m, 4H), 3.42 - 3.29 (m, 1H), 3.27 - 3.16 (m, 2H), 2.97 (t, J = 11.9 Hz, 1H), 2.72 - 2.64 (m, 1H), 2.59 - 2.53 (m, 1H), 2.35 - 2.27 (m, 1H), 2.23 - 2.12 (m, 3H), 2.08 (s, 3H), 1.85 (d, J = 13.0 Hz, 2H), 1.24 (t, J = 6.5 Hz, 6H), 1.14 (d, J = 6.8 Hz, 6H). LCMS (ESI) calcd for C₃₉H₄₆ClFN₇O₅S⁺ [M+H]⁺: 778.29; found, 778.2.

### Example 194: Preparation of 3-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-5-fluoropyridin-2-yl)piperidine-2,6-dione (GT-08075)

According to the method of step 2 of Scheme I-1, the target compound (GT-08075) was prepared (white solid, 46 mg, yield 81 %). ¹H NMR (400 MHz, DMSO-d6) δ 12.15 (s, 1H), 10.92 (s, 1H), 9.76 (s, 1H), 8.61 (s, 1H), 8.25 (s, 2H), 7.77 (d, J = 5.0 Hz, 1H), 7.66 (dd, J = 13.8, 7.6 Hz, 1H), 7.58 - 7.40 (m, 2H), 7.29 (t, J = 7.3 Hz, 1H), 7.07 (s, 1H), 6.88 (s, 1H), 4.31 (s, 3H), 4.09 (dd, J = 9.6, 5.3 Hz, 3H), 3.91 (d, J = 11.2 Hz, 3H), 3.82 (s, 3H), 3.69 (s, 3H), 3.52 (s, 4H), 3.42 - 3.37 (m, 1H), 3.09 (s, 1H), 2.70 - 2.60 (m, 1H), 2.57 - 2.53 (m, 1H), 2.33 - 2.27 (m, 2H), 2.21 - 2.12 (m, 1H), 2.08 (s, 1H), 1.81 (d, J = 13.7 Hz, 6H). LCMS (ESI) calcd for C₃₉H₄₇ClFN₉O₄P⁺ [M+H]⁺: 790.32; found, 790.2.

### Example 195: Preparation of 3-(4-((4-(4-((5-chloro-4-((2-(dimethylphosphoryl)phenyl)amino)pyrimidin-2-yl)amino)-3-methoxyphenyl)piperazin-1-yl)methyl)-5-fluoropyridin-2-yl)piperidine-2,6-dione (GT-08076)

According to the method of step 2 of Scheme I-1, the target compound (GT-08076) was prepared (brown solid, 20 mg, yield 33 %). ¹H NMR (400 MHz, DMSO-d6) δ 12.19 (s, 1H), 12.00 (s, 1H), 10.93 (s, 1H), 9.74 (s, 1H), 8.66 (s, 1H), 8.51 - 8.11 (m, 2H), 7.95 (d, J = 5.7 Hz, 1H), 7.65 (dd, J = 13.2, 8.0 Hz, 1H), 7.41 (s, 1H), 7.35 (d, J = 8.6 Hz, 1H), 7.27 (t, J = 7.2 Hz, 1H), 6.76 (d, J = 2.2 Hz, 1H), 6.59 (dd, J = 8.8, 2.1 Hz, 1H), 4.53 (s, 2H), 4.09 (dd, J = 9.8, 5.3 Hz, 2H), 3.79 (s, 3H), 3.52 (s, 3H), 3.31 (s, 4H), 2.72 - 2.63 (m, 1H), 2.58 - 2.54 (m, 1H), 2.34 - 2.23 (m, 1H), 2.23 - 2.12 (m, 1H), 1.80 (dd, J = 13.6, 6.5 Hz, 6H). LCMS (ESI) calcd for C₃₄H₃₈ClFN₈O₄P⁺ [M+H]⁺: 707.24; found, 707.1.

### Example 196: Preparation of 3-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-5-fluoropyridin-2-yl)piperidine-2,6-dione (GT-08078)

According to the method of step 2 of Scheme I-1, the target compound (GT-08078) was prepared (white solid, 45 mg, yield 75 %). ¹H NMR (400 MHz, DMSO-d6) δ 12.12 (s, 1H), 11.61 (s, 1H), 10.93 (s, 1H), 9.70 (s, 1H), 8.65 (s, 1H), 8.35 (s, 2H), 7.96 (s, 1H), 7.66 (dd, J = 13.6, 7.6 Hz, 1H), 7.58 - 7.40 (m, 2H), 7.28 (t, J = 7.4 Hz, 1H), 7.05 (s, 1H), 6.88 (s, 1H), 4.65 - 4.57 (m, 1H), 4.40 - 4.35 (m, 1H), 4.12 - 4.09 (m, 2H), 3.94 - 3.91 (m, 3H), 3.83 (s, 3H), 3.62 - 3.57 (m, 1H), 3.09 (s, 1H), 2.72 (s, 3H), 2.67 (s, 1H), 2.55 (s, 1H), 2.42 (s, 1H), 2.34 - 2.26 (m, 2H), 2.23 - 2.15 (m, 2H), 1.81 (d, J = 13.7 Hz, 6H). LCMS (ESI) calcd for C₃₆H₄₂ClFN₈O₄P⁺ [M+H]⁺: 735.27; found, 735.2.

### Example 197: Preparation of 1-(4-((4-chloro-2-(7-chloro-5-(trifluoromethyl)-1H-benzo[d]imidazol-2-yl)phenoxy)methyl)phenyl)dihydropyrimidine-2,4(1H,3H)-dione (GT-06479)

According to the method of Scheme I-2, the target compound (GT-06479) was prepared (white solid, 16 mg, yield 25.28%). ¹H NMR (400 MHz, DMSO-d6) δ 10.36 (s, 1H), 8.25 (d, J = 2.7 Hz, 1H), 8.00 (s, 1H), 7.67 (d, J = 1.0 Hz, 1H), 7.56 - 7.49 (m, 3H), 7.32 (dd, J = 8.7, 5.1 Hz, 3H), 5.54 (s, 2H), 3.76 (d, J = 6.6 Hz, 2H), 2.67 (t, J = 6.7 Hz, 2H). LCMS (ESI) calcd for C₂₅H₁₈Cl₂F₃N₄O₃⁺ [M+H]⁺: 549.07; found, 549.1.

### Example 198: Preparation of 1-(3-((4-chloro-2-(7-chloro-5-(trifluoromethyl)-1H-benzo[d]imidazol-2-yl)phenoxy)methyl)phenyl)dihydropyrimidine-2,4(1H,3H)-dione (GT-06480)

According to the method of Scheme I-2, the target compound (GT-06480) was prepared (white solid, 8 mg, yield 12.64%). ¹H NMR (400 MHz, DMSO-d6) δ 10.36 (s, 1H), 8.23 (d, J = 2.7 Hz, 1H), 7.98 (s, 1H), 7.67 (d, J = 1.1 Hz, 1H), 7.56 (dd, J = 9.0, 2.8 Hz, 1H), 7.49 (s, 1H), 7.40 - 7.32 (m, 3H), 7.28 (dd, J = 7.3, 1.9 Hz, 1H), 5.52 (s, 2H), 3.72 (d, J = 6.6 Hz, 2H), 2.64 (t, J = 6.6 Hz, 2H). LCMS (ESI) calcd for C₂₅H₁₈Cl₂F₃N₄O₃⁺ [M+H]⁺: 549.07; found, 549.2.

### Example 199: Preparation of 1-(5-((4-chloro-2-(7-chloro-5-(trifluoromethyl)-1H-benzo[d]imidazol-2-yl)phenoxy)methyl)pyridin-2-yl)dihydropyrimidine-2,4(1H,3H)-dione (GT-06481)

According to the method of Scheme I-2, the target compound (GT-06481) was prepared (white solid, 10 mg, yield 15.77%). ¹H NMR (400 MHz, DMSO-d6) δ 10.53 (s, 1H), 8.57 (d, J = 2.1 Hz, 1H), 8.24 (d, J = 2.7 Hz, 1H), 7.99 (s, 1H), 7.96 (dd, J = 8.7, 2.3 Hz, 1H), 7.74 (d, J = 8.6 Hz, 1H), 7.68 (d, J = 1.2 Hz, 1H), 7.57 (dd, J = 9.0, 2.8 Hz, 1H), 7.41 (d, J = 9.0 Hz, 1H), 5.55 (s, 2H), 4.02 (t, J = 6.6 Hz, 2H), 2.66 (t, J = 6.6 Hz, 2H). LCMS (ESI) calcd for C₂₄H₁₇Cl₂F₃N₅O₃⁺ [M+H]⁺: 550.07; found, 550.1.

### Example 200: Preparation of 1-(4-((5-(3,5-dimethylisoxazol-4-yl)-2-methylphenyl)((1-((5-(2,4-dioxotetrahydropyrimidin-1(2H)-yl)pyridin-2-yl)methyl)piperidin-4-yl)methyl)amino)phenyl)cyclopropane-1-carbonitrile (GT-06411)

According to the method of Scheme I-2, the target compound (GT-06411) was prepared (white solid, 12 mg, yield 20.53%). ¹H NMR (400 MHz, DMSO-d6) δ 10.58 (s, 2H), 8.67 (d, J = 2.4 Hz, 1H), 7.88 (dd, J = 8.3, 2.5 Hz, 1H), 7.71 (d, J = 8.3 Hz, 1H), 7.43 (t, J = 11.7 Hz, 1H), 7.32 - 7.21 (m, 2H), 7.12 (d, J = 8.8 Hz, 2H), 6.49 (d, J = 8.0 Hz, 2H), 3.87 (t, J = 6.6 Hz, 2H), 3.40 - 3.31 (m, 4H), 3.00 (s, 2H), 2.75 (t, J = 6.6 Hz, 2H), 2.41 (s, 3H), 2.24 (s, 3H), 2.04 - 1.93 (m, 6H), 1.60 (dd, J = 7.3, 4.7 Hz, 4H), 1.35 - 1.25 (m, 2H). LCMS (ESI) calcd for C₃₈H₄₂N₇O₃⁺ [M+H]⁺: 644.33; found, 644.4.

### Example 201: Preparation of 1-(4-((5-(3,5-dimethylisoxazol-4-yl)-2-methylphenyl)((1-((6-(2,4-dioxotetrahydropyrimidin-1(2H)-yl)pyridin-3-yl)methyl)piperidin-4-yl)methyl)amino)phenyl)cyclopropane-1-carbonitrile (GT-06412)

According to the method of Scheme I-2, the target compound (GT-06412) was prepared (white solid, 22 mg, yield 37.64%). ¹H NMR (400 MHz, DMSO-d6) δ 10.70 (d, J = 23.7 Hz, 1H), 10.60 (s, 1H), 8.55 (t, J = 8.7 Hz, 1H), 8.11 - 7.99 (m, 1H), 7.85 (dd, J = 20.1, 11.4 Hz, 1H), 7.44 (t, J = 8.4 Hz, 1H), 7.23 (dd, J = 15.7, 4.6 Hz, 2H), 7.09 (t, J = 9.4 Hz, 2H), 6.51 (dd, J = 30.8, 8.8 Hz, 2H), 4.31 (t, J = 38.4 Hz, 2H), 4.07 (t, J = 6.5 Hz, 2H), 3.29 - 3.17 (m, 4H), 2.86 (d, J = 11.4 Hz, 2H), 2.68 (t, J = 6.4 Hz, 2H), 2.41 (d, J = 5.0 Hz, 3H), 2.23 (d, J = 5.2 Hz, 3H), 1.99 (q, J = 11.4 Hz, 6H), 1.69 - 1.44 (m, 4H), 1.36 - 1.27 (m, 2H). LCMS (ESI) calcd for C₃₈H₄₂N₇O₃⁺ [M+H]⁺: 644.33; found, 644.3.

### Example 202: Preparation of 3-(4-((2-(6-methyl-7-oxo-6,7-dihydro-1H-pyrrolo[2,3-c]pyridin-4-yl)-4-(methylsulfonyl)phenoxy)methyl)phenyl)piperidine-2,6-dione (GT-06415)

According to the method of Scheme I-2, the target compound (GT-06415) was prepared (white solid, 6 mg, yield 12.25%). ¹H NMR (400 MHz, DMSO-d6) δ 12.03 (s, 1H), 7.88 (d, J = 8.6 Hz, 1H), 7.46 (d, J = 8.8 Hz, 1H), 7.28 (s, 1H), 7.10 (t, J = 8.7 Hz, 10H), 6.17 (s, 1H), 5.23 (s, 2H), 3.80 - 3.76 (m, 2H), 3.53 (s, 3H), 3.21 (s, 3H), 2.63 (s, 1H), 2.14 (d, J = 4.4 Hz, 1H), 2.02 (d, J = 3.8 Hz, 1H). LCMS (ESI) calcd for C₂₇H₂₆N₃O₆S⁺ [M+H]⁺: 520.15; found, 520.2.

### Example 203: Preparation of 3-(2-fluoro-4-((2-(6-methyl-7-oxo-6,7-dihydro-1H-pyrrolo[2,3-c]pyridin-4-yl)-4-(methylsulfonyl)phenoxy)methyl)phenyl)piperidine-2,6-dione (GT-04640)

According to the method of Scheme I-2, the target compound (GT-04640) was prepared (white solid, 18 mg, yield 21.33%). ¹H NMR (400 MHz, DMSO) δ 12.07 (s, 1H), 10.86 (s, 1H), 7.98 - 7.83 (m, 2H), 7.45 (d, J = 8.8 Hz, 1H), 7.40 (s, 1H), 7.28 (dt, J = 15.5, 5.3 Hz, 2H), 7.07 (dt, J = 7.5, 5.3 Hz, 2H), 6.18 (t, J = 2.3 Hz, 1H), 5.26 (s, 1H), 4.01 (dd, J = 12.4, 4.8 Hz, 1H), 3.55 (s, 3H), 3.44 (d, J = 7.0 Hz, 2H), 3.24 (d, J = 6.2 Hz, 3H), 2.72 (dd, J = 14.8, 10.2 Hz, 1H), 2.54 (s, 1H), 2.18 (d, J = 13.0 Hz, 1H), 1.97 (d, J = 4.9 Hz, 1H). LCMS (ESI) calcd for C₂₇H₂₅FN₃O₆S⁺ [M+H]⁺: 538.14; found, 538.2.

### Example 204: Preparation of 1-(4-((2-(6-methyl-7-oxo-6,7-dihydro-1H-pyrrolo[2,3-c]pyridin-4-yl)-4-(methylsulfonyl)phenoxy)methyl)phenyl)dihydropyrimidine-2,4(1H,3H)-dione (GT-04645)

According to the method of Scheme I-2, the target compound (GT-04645) was prepared (white solid, 30 mg, yield 45.86%). ¹H NMR (400 MHz, DMSO) δ 12.06 (s, 1H), 10.36 (s, 1H), 7.92 (dd,J = 8.7, 2.4 Hz, 1H), 7.88 (t,J = 3.6 Hz, 1H), 7.47 (d,J = 8.8 Hz, 1H), 7.38 (s, 1H), 7.34 (d,J = 8.6 Hz, 2H), 7.32 - 7.24 (m, 3H), 6.23 - 6.15 (m, 1H), 5.25 (s, 2H), 3.76 (t,J = 6.6 Hz, 2H), 3.55 (s, 3H), 3.23 (s, 3H), 2.69 (t,J = 6.6 Hz, 2H). LCMS (ESI) calcd for C₂₆H₂₅N₄O₆S⁺ [M+H]⁺: 521.15; found, 521.2.

### Example 205: Preparation of 1-(2-fluoro-4-((2-(6-methyl-7-oxo-6,7-dihydro-1H-pyrrolo[2,3-c]pyridin-4-yl)-4-(methylsulfonyl)phenoxy)methyl)phenyl)dihydropyrimidine-2,4(1H,3H)-dione (GT-04643)

According to the method of Scheme I-2, the target compound (GT-04643) was prepared (white solid, 50 mg, yield 73.89%). ¹H NMR (400 MHz, DMSO) δ 12.07 (s, 1H), 10.47 (s, 1H), 7.92 (s, 1H), 7.88 (d,J = 2.4 Hz, 1H), 7.45 (d,J = 8.8 Hz, 1H), 7.38 (d,J = 9.4 Hz, 2H), 7.30 (s, 1H), 7.18 (d,J = 7.3 Hz, 2H), 6.18 (s, 1H), 5.27 (s, 2H), 3.68 (t,J = 6.7 Hz, 2H), 3.55 (s, 3H), 3.23 (s, 3H), 2.69 (t,J = 6.7 Hz, 2H). LCMS (ESI) calcd for C₂₆H₂₄FN₄O₆S⁺ [M+H]⁺: 539.14; found, 539.2.

### Example 206: Preparation of 1-(3-fluoro-4-((2-(6-methyl-7-oxo-6,7-dihydro-1H-pyrrolo[2,3-c]pyridin-4-yl)-4-(methylsulfonyl)phenoxy)methyl)phenyl)dihydropyrimidine-2,4(1H,3H)-dione (GT-04644)

According to the method of Scheme I-2, the target compound (GT-04644) was prepared (white solid, 41 mg, yield 60.59%). ¹H NMR (400 MHz, DMSO) δ 12.03 (s, 1H), 10.45 (s, 1H), 7.96 - 7.90 (m, 1H), 7.88 (d, J = 2.4 Hz, 1H), 7.53 (d, J = 8.8 Hz, 1H), 7.40 - 7.32 (m, 2H), 7.26 (ddd, J = 6.3, 4.1, 2.1 Hz, 2H), 7.11 (dd, J = 8.3, 1.9 Hz, 1H), 6.25 - 6.08 (m, 1H), 5.28 (s, 2H), 3.78 (t, J = 6.6 Hz, 2H), 3.53 (s, 3H), 3.23 (s, 3H), 2.69 (t, J = 6.6 Hz, 2H). LCMS (ESI) calcd for C₂₆H₂₄FN₄O₆S⁺ [M+H]⁺: 539.14; found, 539.2.

### Example 207: Preparation of 1-(3-((2-(6-methyl-7-oxo-6,7-dihydro-1H-pyrrolo[2,3-c]pyridin-4-yl)-4-(methylsulfonyl)phenoxy)methyl)phenyl)dihydropyrimidine-2,4(1H,3H)-dione (GT-04642)

According to the method of Scheme I-2, the target compound (GT-04642) was prepared (white solid, 58 mg, yield 70.94%). ¹H NMR (400 MHz, DMSO) δ 12.09 (s, 1H), 10.38 (s, 1H), 7.93 (dd,J = 8.7, 2.4 Hz, 1H), 7.87 (d,J = 2.4 Hz, 1H), 7.48 (d,J = 8.8 Hz, 1H), 7.37 (s, 1H), 7.35 - 7.24 (m, 3H), 7.19 (d,J = 7.2 Hz, 2H), 6.24 - 6.12 (m, 1H), 5.27 (s, 2H), 3.60 - 3.54 (m, 5H), 3.23 (s, 3H), 2.70 (t,J = 6.6 Hz, 2H). LCMS (ESI) calcd for C₂₆H₂₅N₄O₆S⁺ [M+H]⁺: 521.15; found, 521.15.

### Example 208: Preparation of 1-(6-((2-(6-methyl-7-oxo-6,7-dihydro-1H-pyrrolo[2,3-c]pyridin-4-yl)-4-(methylsulfonyl)phenoxy)methyl)pyridin-3-yl)dihydropyrimidine-2,4(1H,3H)-dione (GT-06416)

According to the method of Scheme I-2, the target compound (GT-06416) was prepared (white solid, 17 mg, yield 34.59%). ¹H NMR (400 MHz, DMSO-d6) δ 12.05 (s, 1H), 10.51 (s, 1H), 8.58 (d, J = 2.3 Hz, 1H), 7.98 - 7.85 (m, 2H), 7.74 (dd, J = 8.4, 2.5 Hz, 1H), 7.52 - 7.41 (m, 2H), 7.29 (dd, J = 9.4, 5.8 Hz, 2H), 6.20 (t, J = 2.2 Hz, 1H), 5.34 (s, 2H), 3.82 (t, J = 6.6 Hz, 2H), 3.57 (s, 3H), 3.22 (s, 3H), 2.71 (t, J = 6.6 Hz, 2H). LCMS (ESI) calcd for C₂₅H₂₄N₅O₆S⁺ [M+H]⁺: 522.14; found, 522.2.

### Example 209: Preparation of 1-(5-((2-(6-methyl-7-oxo-6,7-dihydro-1H-pyrrolo[2,3-c]pyridin-4-yl)-4-(methylsulfonyl)phenoxy)methyl)pyridin-2-yl)dihydropyrimidine-2,4(1H,3H)-dione (GT-06417)

According to the method of Scheme I-2, the target compound (GT-06417) was prepared (white solid, 22 mg, yield 44.76%). ¹H NMR (400 MHz, DMSO-d6) δ 12.03 (s, 1H), 10.53 (s, 1H), 8.34 (d, J = 1.8 Hz, 1H), 7.94 (dd, J = 8.7, 2.4 Hz, 1H), 7.87 (d, J = 2.4 Hz, 1H), 7.77 - 7.72 (m, 1H), 7.69 (d, J = 8.5 Hz, 1H), 7.50 (d, J = 8.8 Hz, 1H), 7.36 (s, 1H), 7.29 (t, J = 2.8 Hz, 1H), 6.22 - 6.03 (m, 1H), 5.27 (s, 2H), 4.01 (t, J = 6.6 Hz, 2H), 3.57 (s, 3H), 3.22 (d, J = 4.0 Hz, 3H), 2.67 (t, J = 6.6 Hz, 2H). LCMS (ESI) calcd for C₂₅H₂₄N₅O₆S⁺ [M+H]⁺: 522.14; found, 522.2.

### Example 210: Preparation of 3-((3-((2-(6-methyl-7-oxo-6,7-dihydro-1H-pyrrolo[2,3-c]pyridin-4-yl)-4-(methylsulfonyl)phenoxy)methyl)phenyl)amino)piperidine-2,6-dione (GT-04641)

According to the method of Scheme I-2, the target compound (GT-04641) was prepared (white solid, 32 mg, yield 38.11%). ¹H NMR (400 MHz, DMSO) δ 12.05 (s, 1H), 10.78 (s, 1H), 7.88 (d,J = 7.2 Hz, 2H), 7.45 - 7.33 (m, 2H), 7.28 (t,J = 2.7 Hz, 1H), 7.03 (t,J = 7.7 Hz, 1H), 6.64 (s, 1H), 6.57 (dd,J = 17.9, 7.7 Hz, 2H), 6.17 (s, 1H), 5.12 (s, 2H), 4.22 (dd,J = 11.3, 4.8 Hz, 1H), 3.53 (s, 3H), 3.21 (s, 3H), 2.74 - 2.66 (m, 1H), 2.58 (d,J = 4.1 Hz, 1H), 2.05 (dd,J = 12.9, 4.3 Hz, 1H), 1.88 - 1.80 (m, 1H). LCMS (ESI) calcd for C₂₇H₂₇N₄O₆S⁺ [M+H]⁺: 535.17; found, 535.2.

### Example 211: Preparation of 1-(4-((5-(3,5-dimethylisoxazol-4-yl)-2-methylphenyl)((1-(4-(2,6-dioxopiperidin-3-yl)benzyl)piperidin-4-yl)methyl)amino)phenyl)cyclopropane-1-carbonitrile (GT-04839)

According to the method of step 2 of Scheme I-1, the target compound (GT-04839) was prepared (white solid, 43 mg, yield 56 %). ¹H NMR (400 MHz, DMSO) δ 10.87 (s, 1H), 9.98 (s, 1H), 7.49 (d, *J* = 8.2 Hz, 2H), 7.44 (d, *J* = 8.1 Hz, 1H), 7.32 (t, *J* = 8.6 Hz, 2H), 7.28 - 7.21 (m, 2H), 7.15 - 7.08 (m, 2H), 6.47 (d, *J* = 8.9 Hz, 2H), 4.21 (d, *J* = 4.7 Hz, 2H), 3.91 (dd, *J* = 11.6, 4.9 Hz, 1H), 3.51 (d, *J* = 5.6 Hz, 2H), 3.11 (s, 1H), 2.87 (d, *J* = 15.1 Hz, 2H), 2.74 - 2.61 (m, 2H), 2.41 (d, *J* = 6.1 Hz, 3H), 2.24 (d, *J* = 6.4 Hz, 4H), 2.04 (s, 2H), 2.01 (s, 3H), 1.98 - 1.76 (m, 3H), 1.62 - 1.58 (m, 2H), 1.49 (d, *J* = 12.4 Hz, 2H), 1.33 - 1.29 (m, 2H). LCMS (ESI) calcd for C₄₀H₄₄N₅O₃⁺ [M+H]⁺: 642.34; found, 642.4.

### Example 212: Preparation of 1-(4-((5-(3,5-dimethylisoxazol-4-yl)-2-methylphenyl)((1-(4-(2,6-dioxopiperidin-3-yl)-3-fluorobenzyl)piperidin-4-yl)methyl)amino)phenyl)cyclopropane-1-carbonitrile (GT-04840)

According to the method of step 2 of Scheme I-1, the target compound (GT-04840) was prepared (white solid, 41 mg, yield 52 %). ¹H NMR (400 MHz, DMSO) δ 10.92 (s, 1H), 10.22 (s, 1H), 7.46 - 7.38 (m, 3H), 7.34 (d, *J =* 7.8 Hz, 1H), 7.28 - 7.22 (m, 2H), 7.11 (d, *J =* 8.8 Hz, 2H), 6.48 (d, *J* = 8.9 Hz, 2H), 4.23 (d, *J* = 5.0 Hz, 2H), 4.10 (dd, *J* = 12.5, 4.9 Hz, 1H), 3.51 (d, *J* = 6.1 Hz, 2H), 3.10 (s, 1H), 2.92 - 2.83 (m, 2H), 2.81 - 2.64 (m, 2H), 2.57 (s, 1H), 2.41 (d, *J* = 4.4 Hz, 3H), 2.24 (d, *J* = 4.7 Hz, 4H), 2.04 - 1.95 (m, 7H), 1.61 - 1.58 (m, 2H), 1.52 (d, *J* = 12.8 Hz, 2H), 1.32 - 1.29 (m, 2H). LCMS (ESI) calcd for C₄₀H₄₃FN₅O₃ ⁺ [M+H]⁺: 660.33; found, 660.3.

### Example 213: Preparation of 1-(4-((5-(3,5-dimethylisoxazol-4-yl)-2-methylphenyl)((1-(3-((2,6-dioxopiperidin-3-yl)amino)benzyl)piperidin-4-yl)methyl)amino)phenyl)cyclopropane-1-carbonitrile (GT-04841)

According to the method of step 2 of Scheme I-1, the target compound (GT-04841) was prepared (white solid, 39 mg, yield 50 %). ¹H NMR (400 MHz, DMSO) δ 10.83 (s, 1H), 10.14 (s, 1H), 7.44 (d, *J* = 8.0 Hz, 1H), 7.29 - 7.21 (m, 2H), 7.15 - 7.09 (m, 3H), 6.89 (d, *J* = 11.5 Hz, 1H), 6.72 (dd, *J* = 20.2, 8.7 Hz, 2H), 6.47 (d, *J* = 8.7 Hz, 2H), 4.35 (dd, *J* = 11.5, 4.6 Hz, 1H), 4.07 (d, *J* = 5.0 Hz, 1H), 3.50 (d, *J* = 5.3 Hz, 2H), 3.31 (s, 2H), 3.09 (s, 1H), 2.91 - 2.72 (m, 3H), 2.59 (d, *J* = 17.6 Hz, 1H), 2.41 (d, *J* = 3.7 Hz, 3H), 2.23 (d, *J* = 3.4 Hz, 3H), 2.18 - 2.11 (m, 1H), 2.02 (d, *J* = 10.9 Hz, 3H), 1.99 - 1.85 (m, 4H), 1.63 - 1.47 (m, 4H), 1.33 - 1.28 (m, 2H). LCMS (ESI) calcd for C₄₀H₄₅N₆O₃⁺ [M+H]⁺: 657.35; found, 657.4.

### Example 214: Preparation of 1-(4-((5-(3,5-dimethylisoxazol-4-yl)-2-methylphenyl)((1-(3-(2,4-dioxotetrahydropyrimidin-1(2H)-yl)benzyl)piperidin-4-yl)methyl)amino)phenyl)cyclopropane-1-carbonitrile (GT-04842)

According to the method of step 2 of Scheme I-1, the target compound (GT-04842) was prepared (white solid, 44 mg, yield 57 %). ¹H NMR (400 MHz, DMSO) δ 10.44 (s, 1H), 10.18 (s, 1H), 7.57 - 7.51 (m, 1H), 7.44 (t, *J =* 6.8 Hz, 3H), 7.38 (d, *J =* 6.2 Hz, 1H), 7.28 - 7.21 (m, 2H), 7.11 (d, *J =* 8.8 Hz, 2H), 6.47 (d, *J =* 8.8 Hz, 2H), 4.24 (d, *J =* 4.9 Hz, 2H), 3.83 (t, *J =* 6.6 Hz, 2H), 3.51 (d, *J =* 5.9 Hz, 2H), 3.11 (s, 1H), 2.86 (dd, *J =* 22.7, 10.9 Hz, 2H), 2.71 (t, *J =* 6.6 Hz, 2H), 2.41 (d, *J =* 6.0 Hz, 3H), 2.23 (d, *J =* 5.8 Hz, 3H), 2.04 - 1.97 (m, 4H), 1.95 (s, 2H), 1.84 (s, 1H), 1.61 - 1.57 (m, 2H), 1.52 (d, *J =* 12.1 Hz, 2H), 1.33 - 1.29 (m, 2H). LCMS (ESI) calcd for C₃₉H₄₃N₆O₃ ⁺ [M+H]⁺: 643.34; found, 643.4.

### Example 215: Preparation of 1-(4-((5-(3,5-dimethylisoxazol-4-yl)-2-methylphenyl)((1-(4-(2,4-dioxotetrahydropyrimidin-1(2H)-yl)-3-fluorobenzyl)piperidin-4-yl)methyl)amino)phenyl)cyclopropane-1-carbonitrile (GT-04843)

According to the method of step 2 of Scheme I-1, the target compound (GT-04843) was prepared (white solid, 39 mg, yield 50 %). ¹H NMR (400 MHz, DMSO) δ 10.52 (s, 1H), 10.36 (s, 1H), 7.58 (d, *J =* 11.4 Hz, 1H), 7.55 - 7.48 (m, 1H), 7.46 - 7.40 (m, 2H), 7.28 - 7.20 (m, 2H), 7.11 (d, *J =* 8.7 Hz, 2H), 6.48 (d, *J =* 8.8 Hz, 2H), 4.25 (d, *J =* 4.4 Hz, 2H), 3.73 (t, *J =* 6.5 Hz, 3H), 3.52 (d, *J =* 5.5 Hz, 2H), 3.12 (s, 1H), 2.95 - 2.78 (m, 2H), 2.71 (t, *J =* 17.3, 10.7 Hz, 3H), 2.41 (d, *J =* 4.2 Hz, 3H), 2.24 (d, *J =* 4.6 Hz, 3H), 2.05 - 1.98 (m, 4H), 1.96 (s, 2H), 1.60 (q, *J =* 4.6 Hz, 2H), 1.54 (d, *J =* 13.2 Hz, 1H), 1.31 (dd, *J =* 7.4, 4.7 Hz, 2H). LCMS (ESI) calcd for C₃₉H₄₂FN₆O₃⁺ [M+H]⁺: 661.33; found, 661.4.

### Example 216: Preparation of 1-(4-((5-(3,5-dimethylisoxazol-4-yl)-2-methylphenyl)((1-(4-(2,4-dioxotetrahydropyrimidin-1(2H)-yl)benzyl)piperidin-4-yl)methyl)amino)phenyl)cyclopropane-1-carbonitrile (GT-04844)

According to the method of step 2 of Scheme I-1, the target compound (GT-04844) was prepared (white solid, 33 mg, yield 43 %). ¹H NMR (400 MHz, DMSO) δ 10.42 (s, 1H), 10.21 (s, 1H), 7.55 (d, *J =* 8.3 Hz, 2H), 7.46 - 7.39 (m, 3H), 7.27 - 7.19 (m, 2H), 7.11 (d, *J =* 8.7 Hz, 2H), 6.48 (d, *J =* 8.8 Hz, 2H), 4.22 (d, *J =* 4.7 Hz, 1H), 3.81 (t, *J =* 6.6 Hz, 2H), 3.51 (d, *J =* 5.6 Hz, 2H), 3.34 (s, 2H), 3.11 (s, 1H), 2.86 (d, *J =* 11.9 Hz, 2H), 2.71 (t, *J =* 6.6 Hz, 2H), 2.40 (s, 3H), 2.23 (s, 3H), 2.05 - 2.01 (m, 3H), 1.96 (s, 3H), 1.85 (s, 1H), 1.60 (q, *J* = 4.6 Hz, 2H), 1.52 (d, *J* = 12.7 Hz, 1H), 1.31 (dd, *J* = 7.6, 4.9 Hz, 2H). LCMS (ESI) calcd for C₃₉H₄₃N₆O₃ ⁺ [M+H]⁺: 643.34; found, 643.3.

### Example 217: Preparation of 1-(4-((5-(3,5-dimethylisoxazol-4-yl)-2-methylphenyl)((1-(4-(2,4-dioxotetrahydropyrimidin-1(2H)-yl)-2-fluorobenzyl)piperidin-4-yl)methyl)amino)phenyl)cyclopropane-1-carbonitrile (GT-04973)

According to the method of step 2 of Scheme I-1, the target compound (GT-04973) was prepared (white solid, 17 mg, yield 22 %). ¹H NMR (400 MHz, DMSO) δ 10.51 (s, 1H), 10.22 (d, *J =* 44.7 Hz, 1H), 7.69 (t, *J* = 8.4 Hz, 1H), 7.43 (d, *J =* 7.9 Hz, 1H), 7.36 (d, *J =* 11.6 Hz, 1H), 7.27 (t, *J =* 9.3 Hz, 2H), 7.23 (s, 1H), 7.11 (d, *J =* 8.7 Hz, 2H), 6.47 (d, *J =* 8.8 Hz, 2H), 4.26 (s, 1H), 3.83 (t, *J =* 6.6 Hz, 2H), 3.50 (d, *J =* 5.3 Hz, 2H), 3.37 (d, *J* = 12.4 Hz, 2H), 2.94 (d, *J =* 11.8 Hz, 2H), 2.71 (t, *J =* 6.6 Hz, 2H), 2.41 (d, *J =* 5.5 Hz, 3H), 2.24 (d, *J* = 5.6 Hz, 3H), 2.03 (d, *J* = 11.7 Hz, 4H), 1.93 (d, *J* = 21.7 Hz, 2H), 1.60 (q, *J* = 4.6 Hz, 2H), 1.53 (d, *J* = 13.3 Hz, 2H), 1.30 (q, *J* = 4.9 Hz, 2H), 1.25 (d, *J* = 9.2 Hz, 1H). LCMS (ESI) calcd for C₃₉H₄₂FN₆O₃ ⁺ [M+H]⁺: 661.33; found, 661.4.

### Example 218: Preparation of compound (GT-04684)

According to the method described in Step 2 of Scheme I-1, the target compound (GT-04684) was prepared using the compound (GT-M-459-P1) obtained from Intermediate Example 113 and 3-(4-(bromomethyl)phenyl)piperidine-2,6-dione as starting materials. The target compound (GT-04684) was obtained as a white solid (15 mg, yield 20.98%). ¹H NMR (400 MHz, DMSO) δ 12.11 (s, 1H), 10.88 (s, 1H), 7.61 (d, J = 7.9 Hz, 2H), 7.32 (d, J = 8.0 Hz, 2H), 7.21 - 7.08 (m, 3H), 6.83 (d, J = 7.0 Hz, 3H), 6.64 (d, J = 8.7 Hz, 2H), 6.50 (dd, J = 8.3, 2.3 Hz, 1H), 6.31 (d, J = 7.7 Hz, 2H), 4.37 (s, 2H), 4.19 (d, J = 4.4 Hz, 1H), 3.93 (dd, J = 11.6, 4.9 Hz, 1H), 3.66 (s, 7H), 3.38 (dd, J = 39.9, 9.9 Hz, 6H), 3.04 - 2.90 (m, 2H), 2.80 (s, 1H), 2.68 (td, J = 12.2, 6.1 Hz, 1H), 2.53 (s, 1H), 2.28 - 2.00 (m, 5H), 1.89 (s, 2H), 1.72 (d, J = 7.4 Hz, 1H). LCMS (ESI) calcd for C₄₃H₄₉N₄O₃⁺ [M+H]⁺: 669.38; found, 669.4.

### Example 219: Preparation of compound (GT-04670)

According to the method described in Step 2 of Scheme I-1, the target compound (GT-04670) was prepared using the compound (GT-M-459-P1) obtained from Intermediate Example 113 and 3-(4-(bromomethyl)-2-fluorophenyl)piperidine-2,6-dione as starting materials. The target compound (GT-04670) was obtained as a white solid (35 mg, yield 47.66%). ¹H NMR (400 MHz, MeOD) δ 7.58 - 7.39 (m, 3H), 7.29 (dd, J = 17.8, 8.9 Hz, 2H), 7.12 (q, J = 5.8 Hz, 3H), 6.86 - 6.80 (m, 2H), 6.69 - 6.61 (m, 4H), 6.52 (dd, J = 8.3, 2.5 Hz, 1H), 4.82 - 4.72 (m, 2H), 4.38 (dd, J = 23.6, 18.2 Hz, 3H), 3.81 (dd, J = 14.6, 7.9 Hz, 2H), 3.71 (d, J = 10.7 Hz, 2H), 3.64 - 3.49 (m, 3H), 3.43 (dd, J = 20.5, 6.8 Hz, 7H), 3.25 - 3.18 (m, 1H), 3.09 - 2.99 (m, 2H), 2.83 (t, J = 6.6 Hz, 2H), 2.34 (d, J = 12.7 Hz, 2H), 2.28 - 2.06 (m, 3H), 1.83 (d, J = 10.1 Hz, 1H). LCMS (ESI) calcd for C₄₃H₄₈FN₄O₃⁺ [M+H]⁺: 687.37; found, 687.4.

### Example 220: Preparation of compound (GT-04686)

According to the method described in Step 2 of Scheme I-1, the target compound (GT-04686) was prepared using the compound (GT-M-459-P1) obtained from Intermediate Example 113 and 1-(4-(bromomethyl)phenyl)dihydropyrimidine-2,4(1H,3H)-dione as starting materials. The target compound (GT-04686) was obtained as a white solid (45 mg, yield 48.33%). ¹H NMR (400 MHz, DMSO) δ 12.28 (s, 1H), 10.44 (s, 1H), 7.69 (d, J = 8.5 Hz, 2H), 7.43 (d, J = 8.5 Hz, 2H), 7.14 (t, J = 8.0 Hz, 4H), 7.01 (s, 2H), 6.83 (d, J = 6.6 Hz, 2H), 6.68 - 6.62 (m, 2H), 6.54 - 6.48 (m, 1H), 6.38 (d, J = 8.1 Hz, 2H), 4.42 (s, 2H), 4.23 (d, J = 4.8 Hz, 1H), 3.83 (d, J = 6.6 Hz, 2H), 3.76 (d, J = 9.1 Hz, 4H), 3.50 (s, 6H), 3.39 - 3.30 (m, 2H), 2.98 (d, J = 6.2 Hz, 4H), 2.72 (t, J = 6.6 Hz, 2H), 2.22 (s, 2H), 2.07 (s, 3H), 1.72 (d, J = 6.1 Hz, 1H). LCMS (ESI) calcd for C₄₂H₄₈N₅O₃⁺ [M+H]⁺: 670.38; found, 670.4.

### Example 221: Preparation of compound (GT-04687)

According to the method described in Step 2 of Scheme I-1, the target compound (GT-04687) was prepared using the compound (GT-M-459-P1) obtained from Intermediate Example 113 and 1-(4-(bromomethyl)-3-fluorophenyl)dihydropyrimidine-2,4(1H,3H)-dione as starting materials. The target compound (GT-04687) was obtained as a white solid (55 mg, yield 41.55%). ¹H NMR (400 MHz, DMSO) δ 10.71 - 10.36 (m, 1H), 7.72 (t,J = 8.1 Hz, 1H), 7.39 (dd,J = 11.6, 1.7 Hz, 1H), 7.30 (dd,J = 8.4, 1.7 Hz, 1H), 7.24 - 7.08 (m, 3H), 6.85 (t,J = 13.8 Hz, 4H), 6.64 (d,J = 8.3 Hz, 2H), 6.50 (dd,J = 8.3, 2.4 Hz, 1H), 6.34 (d,J = 7.8 Hz, 2H), 4.35 (s, 2H), 4.21 (d,J = 4.6 Hz, 1H), 3.85 (t, J = 6.6 Hz, 2H), 3.68 (s, 4H), 3.34 (d,J = 14.0 Hz, 2H), 3.06 - 2.83 (m, 4H), 2.73 (t,J = 6.6 Hz, 2H), 2.27- 2.05 (m, 3H), 1.99 (d,J = 23.3 Hz, 2H), 1.72 (d,J = 6.4 Hz, 1H). LCMS (ESI) calcd for C₄₂H₄₇FN₅O₃⁺ [M+H]⁺: 688.37; found, 688.4.

### Example 222: Preparation of compound (GT-04738)

According to the method described in Step 2 of Scheme I-1, the target compound (GT-04738) was prepared using the compound (GT-M-459-P1) obtained from Intermediate Example 113 and 1-(4-(bromomethyl)-2-fluorophenyl)dihydropyrimidine-2,4(1H,3H)-dione as starting materials. The target compound (GT-04738) was obtained as a white solid (44 mg, yield 59.83%). ¹H NMR (400 MHz, DMSO) δ 10.53 (s, 1H), 7.64 (s, 1H), 7.55 - 7.40 (m, 2H), 7.18 - 7.08 (m, 3H), 6.83 (d, J = 6.8 Hz, 2H), 6.78 (s, 1H), 6.66 - 6.59 (m, 2H), 6.49 (dd, J = 8.3, 2.4 Hz, 1H), 6.30 (d, J = 7.6 Hz, 2H), 4.23 (t, J = 26.5 Hz, 3H), 3.73 (t, J = 6.6 Hz, 9H), 3.32 (d, J = 13.4 Hz, 6H), 3.00 - 2.88 (m, 2H), 2.73 (t, J = 6.6 Hz, 3H), 2.09 (dd, J = 18.6, 12.3 Hz, 3H), 1.86 (s, 2H), 1.72 (s, 1H). LCMS (ESI) calcd for C₄₂H₄₇FN₅O₃⁺ [M+H]⁺: 688.37; found, 688.4.

### Example 223: Preparation of compound (GT-04685)

According to the method described in Step 2 of Scheme I-1, the target compound (GT-04685) was prepared using the compound (GT-M-459-P1) obtained from Intermediate Example 113 and 1-(3-(chloromethyl)phenyl)dihydropyrimidine-2,4(1H,3H)-dione as starting materials. The target compound (GT-04685) was obtained as a white solid (45 mg, yield 44.88%). ¹H NMR (400 MHz, DMSO) δ 10.45 (s, 1H), 7.61 (s, 1H), 7.47 (s, 3H), 7.43 - 7.28 (m, 1H), 7.21 - 7.07 (m, 3H), 6.83 (d,J = 6.7 Hz, 4H), 6.63 (t,J = 6.2 Hz, 2H), 6.49 (dd,J = 8.3, 2.3 Hz, 1H), 6.31 (d,J = 7.4 Hz, 2H), 4.37 (s, 2H), 4.19 (d,J = 4.6 Hz, 1H), 3.87 (d,J = 6.6 Hz, 2H), 3.65 (s, 4H), 3.33 (d,J = 12.9 Hz, 2H), 2.97 (dd,J = 20.3, 14.6 Hz, 3H), 2.72 (t,J = 6.6 Hz, 3H), 2.19 - 2.04 (m, 3H), 1.87 (s, 2H), 1.73 (s, 1H). LCMS (ESI) calcd for C₄₂H₄₈N₅O₃⁺ [M+H]⁺: 670.38; found, 670.4.

### Example 224: Preparation of compound (GT-04674)

According to the method described in Step 2 of Scheme I-1, the target compound (GT-04674) was prepared using the compound (GT-M-459-P1) obtained from Intermediate Example 113 and 3-((3-(chloromethyl)phenyl)amino)piperidine-2,6-dione as starting materials. The target compound (GT-04674) was obtained as a white solid (58 mg, yield 79.32%). ¹H NMR (400 MHz, MeOD) δ 7.34 - 7.21 (m, 3H), 7.17 - 7.09 (m, 3H), 7.05 (s, 1H), 6.93 - 6.81 (m, 4H), 6.72 - 6.61 (m, 4H), 6.52 (dd, J = 8.3, 2.5 Hz, 1H), 4.44 (dd, J = 12.1, 4.9 Hz, 1H), 4.39 - 4.31 (m, 3H), 3.78 - 3.40 (m, 14H), 3.03 (dd, J = 14.3, 5.0 Hz, 2H), 2.89 (td, J = 12.8, 6.4 Hz, 1H), 2.72 (dt, J = 7.6, 4.4 Hz, 1H), 2.43 (d, J = 12.5 Hz, 2H), 2.34 (ddd, J = 19.4, 11.2, 8.3 Hz, 3H), 2.14 (dd, J = 11.8, 6.5 Hz, 1H), 1.97 (dd, J = 12.6, 4.5 Hz, 1H), 1.82 (d, J = 7.8 Hz, 1H). LCMS (ESI) calcd for C₄₃H₅₀N₅O₃⁺ [M+H]⁺: 684.39; found, 684.4 .

### Example 225: Preparation of 3-((4-(1-(4-(2,6-dioxopiperidin-3-yl)benzyl)piperidin-4-yl)phenyl)amino)-5-(piperidin-1-yl)pyrazine-2-carboxamide (GT-04785)

According to the method of step 2 of Scheme I-1, the target compound (GT-04785) was prepared (white solid, 49 mg, yield 64.10%). ¹H NMR (400 MHz, DMSO) δ 11.32 (s, 1H), 10.89 (s, 1H), 10.04 (s, 1H), 7.75 (s, 1H), 7.68 (s, 1H), 7.56 (d, J = 8.0 Hz, 4H), 7.39 - 7.32 (m, 3H), 7.17 (d, J = 8.5 Hz, 2H), 4.32 (d, J = 4.7 Hz, 1H), 3.94 (dd, J = 11.7, 4.8 Hz, 1H), 3.68 (s, 4H), 3.44 (d, J = 12.1 Hz, 2H), 3.14 - 3.03 (m, 5H), 2.80 - 2.66 (m, 2H), 2.23 (s, 1H), 1.97 (s, 5H), 1.62 (d, J = 26.2 Hz, 5H). LCMS (ESI) calcd for C₃₃H₄₀N₇O₃⁺ [M+H]⁺: 582.32; found, 582.3.

### Example 226: Preparation of 3-((4-(1-(4-(2,6-dioxopiperidin-3-yl)-3-fluorobenzyl)piperidin-4-yl)phenyl)amino)-5-(piperidin-1-yl)pyrazine-2-carboxamide (GT-04786)

According to the method of step 2 of Scheme I-1, the target compound (GT-04786) was prepared (white solid, 28 mg, yield 35.53%). ¹H NMR (400 MHz, DMSO-d6) δ 11.31 (s, 1H), 11.13 (s, 1H), 10.93 (s, 1H), 7.74 (s, 1H), 7.65 (d, J = 14.9 Hz, 2H), 7.55 (d, J = 8.6 Hz, 2H), 7.48 - 7.40 (m, 2H), 7.32 (s, 1H), 7.17 (d, J = 8.6 Hz, 2H), 4.32 (d, J = 3.9 Hz, 2H), 4.12 (dd, J = 12.6, 4.8 Hz, 1H), 3.67 (d, J = 5.0 Hz, 4H), 3.41 (s, 2H), 3.03 (d, J = 10.0 Hz, 2H), 2.74 (dd, J = 11.5, 3.8 Hz, 2H), 2.22 (ddd, J = 20.3, 11.9, 8.0 Hz, 2H), 2.11 (d, J = 12.7 Hz, 2H), 1.94 (d, J = 13.3 Hz, 2H), 1.67 - 1.55 (m, 7H). LCMS (ESI) calcd for C₃₃H₃₉FN₇O₃⁺ [M+H]⁺: 600.31; found, 600.4.

### Example 227: Preparation of 3-((4-(1-(4-(2,4-dioxotetrahydropyrimidin-1(2H)-yl)benzyl)piperidin-4-yl)phenyl)amino)-5-(piperidin-1-yl)pyrazine-2-carboxamide (GT-04789)

According to the method of step 2 of Scheme I-1, the target compound (GT-04789) was prepared (white solid, 63 mg, yield 63.29%). ¹H NMR (400 MHz, DMSO) δ 11.32 (s, 1H), 10.46 (s, 1H), 10.05 (s, 1H), 7.75 (s, 1H), 7.68 (s, 1H), 7.59 (dd, J = 16.2, 8.4 Hz, 4H), 7.46 (d, J = 8.4 Hz, 2H), 7.32 (s, 1H), 7.18 (d, J = 8.3 Hz, 2H), 4.33 (s, 1H), 3.84 (t, J = 6.4 Hz, 2H), 3.68 (s, 4H), 3.43 (s, 2H), 3.06 - 3.03 (m, 2H), 2.74 (d, J = 6.5 Hz, 2H), 1.99 (s, 4H), 1.62 (d, J = 25.9 Hz, 5H), 1.26 (d, J = 12.6 Hz, 5H). LCMS (ESI) calcd for C₃₂H₃₉N₈O₃⁺ [M+H]⁺: 583.31; found, 583.3.

### Example 228: Preparation of 3-((4-(1-(4-(2,4-dioxotetrahydropyrimidin-1(2H)-yl)-3-fluorobenzyl)piperidin-4-yl)phenyl)amino)-5-(piperidin-1-yl)pyrazine-2-carboxamide (GT-04788)

According to the method of step 2 of Scheme I-1, the target compound (GT-04788) was prepared (white solid, 66 mg, yield 83.61%). ¹H NMR (400 MHz, DMSO-d6) δ 11.32 (s, 1H), 11.01 (s, 1H), 10.55 (s, 1H), 7.71 (dd, J = 17.9, 6.1 Hz, 2H), 7.67 (s, 1H), 7.57 - 7.51 (m, 4H), 7.32 (s, 1H), 7.17 (d, J = 8.6 Hz, 2H), 4.34 (d, J = 4.9 Hz, 2H), 3.75 (t, J = 6.6 Hz, 2H), 3.68 (d, J = 5.3 Hz, 4H), 3.45 (s, 1H), 3.04 (d, J = 12.1 Hz, 2H), 2.73 (t, J = 6.7 Hz, 3H), 2.08 (d, J = 11.6 Hz, 2H), 1.96 (d, J = 13.2 Hz, 2H), 1.62 (dd, J = 25.4, 4.2 Hz, 7H). LCMS (ESI) calcd for C₃₂H₃₈FN₈O₃⁺ [M+H]⁺: 601.31; found, 601.3.

### Example 229: Preparation of 3-((4-(1-(4-(2,4-dioxotetrahydropyrimidin-1(2H)-yl)-2-fluorobenzyl)piperidin-4-yl)phenyl)amino)-5-(piperidin-1-yl)pyrazine-2-carboxamide (GT-04790)

According to the method of step 2 of Scheme I-1, the target compound (GT-04790) was prepared (white solid, 45 mg, yield 57.01%). ¹H NMR (400 MHz, DMSO-d6) δ 11.32 (s, 1H), 10.55 (s, 1H), 7.81 - 7.11 (m, 8H), 4.35 (s, 2H), 3.85 (s, 1H), 3.67 (s, 4H), 3.12 (s, 4H), 2.72 (s, 3H), 1.98 (s, 3H), 1.58 (s, 5H), 1.15 (d, J = 86.7 Hz, 3H). LCMS (ESI) calcd for C₃₂H₃₈FN₈O₃⁺ [M+H]⁺: 601.31; found, 601.3.

### Example 230: Preparation of 3-((4-(1-(3-(2,4-dioxotetrahydropyrimidin-1(2H)-yl)benzyl)piperidin-4-yl)phenyl)amino)-5-(piperidin-1-yl)pyrazine-2-carboxamide (GT-04787)

According to the method of step 2 of Scheme I-1, the target compound (GT-04787) was prepared (white solid, 51 mg, yield 66.61%). ¹H NMR (400 MHz, MeOD) δ 7.60 (tt, J = 16.0, 8.1 Hz, 4H), 7.51 (d, J = 8.2 Hz, 1H), 7.47 (s, 1H), 7.34 (s, 1H), 7.19 (d, J = 8.6 Hz, 1H), 4.38 (d, J = 3.9 Hz, 1H), 3.94 (t, J = 6.7 Hz, 3H), 3.73 (dd, J = 13.2, 6.6 Hz, 3H), 3.63 (d, J = 10.8 Hz, 2H), 3.22 (dd, J = 14.9, 7.4 Hz, 2H), 2.84 (t, J = 6.7 Hz, 2H), 2.11 (s, 4H), 1.69 (d, J = 29.2 Hz, 5H), 1.29 (d, J = 3.1 Hz, 3H). LCMS (ESI) calcd for C₃₂H₃₉N₈O₃⁺ [M+H]⁺: 583.31; found, 583.3.

### Example 231: Preparation of 3-((4-(1-(3-((2,6-dioxopiperidin-3-yl)amino)benzyl)piperidin-4-yl)phenyl)amino)-5-(piperidin-1-yl)pyrazine-2-carboxamide (GT-04791)

According to the method of step 2 of Scheme I-1, the target compound (GT-04791) was prepared (white solid, 51 mg, yield 65.03%). ¹H NMR (400 MHz, DMSO) δ 11.34 (d, J = 17.9 Hz, 1H), 10.85 (s, 1H), 10.50 (s, 1H), 7.78 - 7.64 (m, 2H), 7.61 - 7.52 (m, 3H), 7.17 (t, J = 7.7 Hz, 3H), 7.01 (d, J = 13.4 Hz, 1H), 6.78 (d, J = 7.8 Hz, 2H), 4.41 (dd, J = 11.6, 4.7 Hz, 1H), 4.17 (s, 2H), 3.68 (s, 4H), 3.43 (s, 2H), 3.01 (d, J = 12.0 Hz, 2H), 2.85 - 2.71 (m, 2H), 2.61 (d, J = 17.5 Hz, 1H), 2.23 - 2.14 (m, 1H), 2.07 (dd, J = 24.6, 12.2 Hz, 2H), 1.98 - 1.89 (m, 3H), 1.67 - 1.57 (m, 6H). LCMS (ESI) calcd for C₃₃H₄₁N₈O₃⁺ [M+H]⁺: 597.33; found, 597.4.

### Example 232: Preparation of 3-((4-(1-(4-(2,6-dioxopiperidin-3-yl)benzyl)piperidin-4-yl)phenyl)amino)-5-((R)-3-(3-methyl-2-oxoimidazolidin-1-yl)piperidin-1-yl)pyrazine-2-carboxamide (GT-04798)

According to the method of step 2 of Scheme I-1, the target compound (GT-04798) was prepared (white solid, 19 mg, yield 26.75%). ¹H NMR (400 MHz, DMSO) δ 11.27 (s, 1H), 10.88 (s, 1H), 10.56 (s, 1H), 7.78 (s, 1H), 7.67 (s, 1H), 7.57 (dd, J = 19.9, 7.9 Hz, 4H), 7.34 (d, J = 7.7 Hz, 3H), 7.15 (d, J = 8.3 Hz, 2H), 4.30 (s, 4H), 3.93 (dd, J = 11.6, 4.8 Hz, 1H), 3.60 (s, 1H), 3.41 (s, 2H), 3.32 - 3.23 (m, 4H), 3.12 - 2.96 (m, 4H), 2.72 (d, J = 12.7 Hz, 4H), 2.66 (d, J = 5.5 Hz, 1H), 2.54 (s, 1H), 2.31 - 2.18 (m, 1H), 2.09 - 1.94 (m, 5H), 1.82 (d, J = 9.3 Hz, 3H), 1.57 (s, 1H). LCMS (ESI) calcd for C₃₇H₄₆N₉O₄⁺ [M+H]⁺: 680.37; found, 680.4.

### Example 233: Preparation of 3-((4-(1-(4-(2,6-dioxopiperidin-3-yl)-3-fluorobenzyl)piperidin-4-yl)phenyl)amino)-5-((R)-3-(3-methyl-2-oxoimidazolidin-1-yl)piperidin-1-yl)pyrazine-2-carboxamide (GT-04799)

According to the method of step 2 of Scheme I-1, the target compound (GT-04799) was prepared (white solid, 22 mg, yield 30.18%). ¹H NMR (400 MHz, DMSO) δ 11.27 (s, 1H), 10.94 (s, 1H), 10.22 (s, 1H), 7.78 (s, 1H), 7.67 (s, 1H), 7.55 (d, J = 8.2 Hz, 3H), 7.48 - 7.41 (m, 2H), 7.35 (s, 1H), 7.15 (d, J = 8.4 Hz, 2H), 4.31 (d, J = 27.7 Hz, 4H), 4.13 (dd, J = 12.6, 4.6 Hz, 1H), 3.61 (d, J = 3.6 Hz, 2H), 3.44 (s, 2H), 3.27 (d, J = 6.4 Hz, 3H), 3.16 - 2.93 (m, 6H), 2.76 (d, J = 12.5 Hz, 2H), 2.58 (s, 1H), 2.24 (d, J = 13.0 Hz, 1H), 1.98 (s, 6H), 1.81 (s, 3H), 1.58 (s, 1H). LCMS (ESI) calcd for C₃₇H₄₅FN₉O₄⁺ [M+H]⁺: 698.36; found, 698.4.

### Example 234: Preparation of (R)-3-((4-(1-(4-(2,4-dioxotetrahydropyrimidin-1(2H)-yl)benzyl)piperidin-4-yl)phenyl)amino)-5-(3-(3-methyl-2-oxoimidazolidin-1-yl)piperidin-1-yl)pyrazine-2-carboxamide (GT-04802)

According to the method of step 2 of Scheme I-1, the target compound (GT-04802) was prepared (white solid, mg, yield ). ¹H NMR (400 MHz, DMSO-d6) δ 11.27 (s, 1H), 10.72 (s, 1H), 10.46 (s, 1H), 7.82 - 7.73 (m, 1H), 7.66 (d, J = 11.5 Hz, 3H), 7.59 - 7.50 (m, 2H), 7.44 (d, J = 8.0 Hz, 2H), 7.35 (s, 1H), 7.15 (d, J = 8.3 Hz, 2H), 4.31 (s, 4H), 3.84 (t, J = 6.5 Hz, 2H), 3.61 (s, 1H), 3.44 (s, 2H), 3.26 (s, 2H), 3.12 - 2.89 (m, 5H), 2.73 (d, J = 11.3 Hz, 6H), 2.48 - 2.42 (m, 1H), 2.00 (d, J = 18.6 Hz, 4H), 1.81 (s, 3H), 1.58 (s, 1H). LCMS (ESI) calcd for C₃₆H₄₅N₁₀O₄⁺ [M+H]⁺: 681.36; found, 681.4.

### Example 235: Preparation of (R)-3-((4-(1-(4-(2,4-dioxotetrahydropyrimidin-1(2H)-yl)-3-fluorobenzyl)piperidin-4-yl)phenyl)amino)-5-(3-(3-methyl-2-oxoimidazolidin-1-yl)piperidin-1-yl)pyrazine-2-carboxamide (GT-04801)

According to the method of step 2 of Scheme I-1, the target compound (GT-04801) was prepared (white solid, 49 mg, yield 67.12%). ¹H NMR (400 MHz, DMSO) δ 11.27 (s, 1H), 11.03 (s, 1H), 10.87 (s, 1H), 7.77 (s, 1H), 7.67 (t, J = 7.9 Hz, 3H), 7.54 (d, J = 8.0 Hz, 2H), 7.34 (s, 1H), 7.15 (d, J = 8.2 Hz, 2H), 5.11 (dd, J = 13.2, 4.7 Hz, 1H), 4.35 (d, J = 31.8 Hz, 2H), 3.60 (s, 1H), 3.45 (s, 2H), 3.31 - 3.22 (m, 4H), 3.07 (dd, J = 21.4, 10.4 Hz, 4H), 2.99 - 2.88 (m, 2H), 2.71 (s, 4H), 2.61 (d, J = 16.6 Hz, 1H), 2.42 (d, J = 13.1 Hz, 1H), 1.99 (d, J = 17.5 Hz, 5H), 1.81 (s, 3H), 1.58 (s, 1H). LCMS (ESI) calcd for C₃₆H₄₄FN₁₀O₄⁺ [M+H]⁺: 699.35; found, 699.4.

### Example 236: Preparation of (R)-3-((4-(1-(4-(2,4-dioxotetrahydropyrimidin-1(2H)-yl)-2-fluorobenzyl)piperidin-4-yl)phenyl)amino)-5-(3-(3-methyl-2-oxoimidazolidin-1-yl)piperidin-1-yl)pyrazine-2-carboxamide (GT-04803)

According to the method of step 2 of Scheme I-1, the target compound (GT-04803) was prepared (white solid, 45 mg, yield 61.64%). ¹H NMR (400 MHz, DMSO) δ 11.27 (s, 1H), 11.01 (s, 1H), 10.54 (s, 1H), 7.83 (t, J = 8.5 Hz, 2H), 7.67 (s, 1H), 7.54 (d, J = 8.6 Hz, 2H), 7.42 - 7.31 (m, 3H), 7.14 (d, J = 8.6 Hz, 2H), 4.35 (d, J = 3.6 Hz, 4H), 3.87 (t, J = 6.6 Hz, 2H), 3.61 (s, 1H), 3.46 (d, J = 11.2 Hz, 2H), 3.34 - 3.25 (m, 4H), 3.17 - 2.97 (m, 4H), 2.78 - 2.71 (m, 6H), 2.08 (d, J = 12.1 Hz, 2H), 1.96 (d, J = 12.3 Hz, 2H), 1.82 (d, J = 9.5 Hz, 3H), 1.56 (d, J = 11.7 Hz, 1H). LCMS (ESI) calcd for C₃₆H₄₄FN₁₀O₄⁺ [M+H]⁺: 699.35; found, 699.4.

### Example 237: Preparation of (R)-3-((4-(1-(3-(2,4-dioxotetrahydropyrimidin-1(2H)-yl)benzyl)piperidin-4-yl)phenyl)amino)-5-(3-(3-methyl-2-oxoimidazolidin-1-yl)piperidin-1-yl)pyrazine-2-carboxamide (GT-04800)

According to the method of step 2 of Scheme I-1, the target compound (GT-04800) was prepared (white solid, 50 mg, yield 70.29%). ¹H NMR (400 MHz, DMSO-d6) δ 11.28 (d, J = 15.4 Hz, 1H), 11.01 (s, 1H), 10.46 (s, 1H), 7.77 (s, 1H), 7.66 (d, J = 5.0 Hz, 2H), 7.54 (d, J = 8.4 Hz, 2H), 7.48 (t, J = 4.8 Hz, 3H), 7.33 (d, J = 11.6 Hz, 1H), 7.15 (d, J = 8.2 Hz, 2H), 4.30 (d, J = 20.3 Hz, 4H), 3.88 (t, J = 6.6 Hz, 2H), 3.60 (s, 2H), 3.29 (ddd, J = 20.4, 15.6, 8.3 Hz, 6H), 3.12 - 2.97 (m, 4H), 2.76 - 2.69 (m, 6H), 2.09 (dd, J = 24.1, 12.0 Hz, 2H), 1.95 (d, J = 12.7 Hz, 2H), 1.78 (d, J = 21.6 Hz, 3H), 1.56 (d, J = 11.4 Hz, 1H). LCMS (ESI) calcd for C₃₆H₄₅N₁₀O₄⁺ [M+H]⁺: 681.36; found, 681.4.

### Example 238: Preparation of 3-((4-(1-(3-((2,6-dioxopiperidin-3-yl)amino)benzyl)piperidin-4-yl)phenyl)amino)-5-((R)-3-(3-methyl-2-oxoimidazolidin-1-yl)piperidin-1-yl)pyrazine-2-carboxamide (GT-04804)

According to the method of step 2 of Scheme I-1, the target compound (GT-04804) was prepared (white solid, 37 mg, yield 50.97%). ¹H NMR (400 MHz, DMSO) δ 11.27 (s, 1H), 10.85 (s, 1H), 10.44 (s, 1H), 7.77 (s, 1H), 7.67 (s, 1H), 7.54 (d, J = 8.3 Hz, 2H), 7.34 (s, 1H), 7.15 (d, J = 8.6 Hz, 3H), 6.98 (s, 1H), 6.78 (t, J = 6.7 Hz, 2H), 4.44 - 4.26 (m, 3H), 4.17 (s, 1H), 3.60 (s, 1H), 3.35 - 3.22 (m, 7H), 3.13 - 2.95 (m, 4H), 2.71 (s, 5H), 2.63 (s, 1H), 2.17 (d, J = 8.6 Hz, 1H), 2.07 - 1.73 (m, 8H), 1.57 (s, 1H). LCMS (ESI) calcd for C₃₇H₄₇N₁₀O₄⁺ [M+H]⁺: 695.38; found, 695.4.

### Example 239: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(5-(4-(2,6-dioxopiperidin-3-yl)benzyl)-2,5-diazabicyclo[2.2.2]octan-2-yl)-1-(phenylthio)butan-2-yl)amino)-3-((trifluoromethyl)sulfonyl)phenyl)sulfonyl)benzamide (GT-07878)

According to the method of step 2 of Scheme I-1, the target compound (GT-07878) was prepared (white solid, 16 mg, yield 44.40%). ¹H NMR (400 MHz, DMSO-d6) δ 12.17 (s, 1H), 11.58 (s, 1H), 10.87 (s, 1H), 10.37 (s, 1H), 8.19 (s, 1H), 8.00 (d, J = 8.8 Hz, 1H), 7.77 (d, J = 8.9 Hz, 4H), 7.41 (d, J = 8.4 Hz, 2H), 7.30 (d, J = 7.4 Hz, 4H), 7.23 (t, J = 7.6 Hz, 2H), 7.16 (dd, J = 14.6, 7.8 Hz, 4H), 6.98 (dd, J = 20.1, 9.2 Hz, 3H), 4.37 (d, J = 90.2 Hz, 3H), 3.97 - 3.71 (m, 6H), 3.57 (s, 4H), 3.36 (s, 16H), 3.32 - 3.10 (m, 3H), 2.82 - 2.63 (m, 4H), 2.35 - 1.96 (m, 12H), 1.91 - 1.73 (m, 1H), 1.47 (t, J = 6.2 Hz, 2H), 1.00 (s, 6H). LCMS (ESI) calcd for C₆₁H₇₀ClF₃N₇O₇S₃⁺ [M+H]⁺: 1200.41; found, 1200.1.

### Example 240: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(5-(4-(2,4-dioxotetrahydropyrimidin-1(2H)-yl)benzyl)-2,5-diazabicyclo[2.2.2]octan-2-yl)-1-(phenylthio)butan-2-yl)amino)-3-((trifluoromethyl)sulfonyl)phenyl)sulfonyl)benzamide (GT-07879)

According to the method of step 2 of Scheme I-1, the target compound (GT-07879) was prepared (white solid, 24 mg, yield 57.03%). ¹H NMR (400 MHz, DMSO-d6) δ 12.16 (s, 1H), 11.48 (s, 1H), 10.43 (s, 1H), 10.24 (s, 1H), 8.18 (s, 1H), 8.00 (d, J = 8.4 Hz, 1H), 7.77 (d, J = 8.8 Hz, 3H), 7.36 (dd, J = 44.1, 8.1 Hz, 8H), 7.26 - 7.12 (m, 6H), 6.98 (t, J = 14.6 Hz, 3H), 4.37 (d, J = 95.6 Hz, 4H), 3.97 - 3.72 (m, 7H), 3.57 (s, 9H), 3.31 - 3.15 (m, 3H), 2.88 - 2.62 (m, 6H), 2.37 - 2.04 (m, 8H), 1.92 (s, 2H), 1.47 (t, J = 6.2 Hz, 2H), 1.00 (s, 6H). LCMS (ESI) calcd for C₆₀H₆₉ClF₃N₈O₇S₃⁺ [M+H]⁺: 1201.41; found, 1201.1.

### Example 241: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(5-(3-(2,4-dioxotetrahydropyrimidin-1(2H)-yl)benzyl)-2,5-diazabicyclo[2.2.2]octan-2-yl)-1-(phenylthio)butan-2-yl)amino)-3-((trifluoromethyl)sulfonyl)phenyl)sulfonyl)benzamide (GT-07880)

According to the method of step 2 of Scheme I-1, the target compound (GT-07880) was prepared (white solid, 6 mg, yield 16.63%). ¹H NMR (400 MHz, DMSO-d6) δ 12.17 (s, 1H), 11.73 - 11.33 (m, 1H), 10.43 (s, 1H), 10.26 (s, 1H), 8.18 (s, 1H), 8.00 (d, J = 8.4 Hz, 1H), 7.83 - 7.74 (m, 3H), 7.41 (d, J = 8.4 Hz, 4H), 7.30 (d, J = 7.3 Hz, 3H), 7.19 (dt, J = 15.2, 7.2 Hz, 6H), 6.97 (t, J = 13.5 Hz, 3H), 4.38 (d, J = 105.2 Hz, 3H), 3.87 (s, 6H), 3.58 (s, 10H), 3.34 - 3.26 (m, 6H), 2.73 (dd, J = 16.3, 9.7 Hz, 5H), 2.22 (d, J = 48.6 Hz, 7H), 1.97 - 1.65 (m, 2H), 1.48 (d, J = 6.1 Hz, 2H), 1.00 (s, 6H). LCMS (ESI) calcd for C₆₀H₆₉ClF₃N₈O₇S₃⁺ [M+H]⁺: 1201.41; found, 1201.1.

### Example 242: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(5-((5-(2,4-dioxotetrahydropyrimidin-1(2H)-yl)pyridin-2-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide (GT-07885)

According to the method of step 2 of Scheme I-1, the target compound (GT-07885) was prepared (white solid, 20 mg, yield 47.49%). ¹H NMR (400 MHz, DMSO-d6) δ 12.17 (s, 1H), 10.60 (d, J = 22.9 Hz, 1H), 10.36 (s, 1H), 8.63 (s, 1H), 8.18 (s, 1H), 8.00 (d, J = 9.1 Hz, 1H), 7.87 (s, 1H), 7.75 (t, J = 17.0 Hz, 3H), 7.41 (d, J = 8.4 Hz, 2H), 7.30 (d, J = 7.3 Hz, 3H), 7.23 (t, J = 7.6 Hz, 2H), 7.16 (dd, J = 14.6, 7.8 Hz, 3H), 6.98 (dd, J = 21.0, 9.0 Hz, 3H), 4.39 (d, J = 108.3 Hz, 4H), 3.86 (t, J = 6.3 Hz, 5H), 3.57 (s, 11H), 3.32 - 3.06 (m, 5H), 2.75 (t, J = 6.6 Hz, 5H), 2.22 (d, J = 42.8 Hz, 7H), 1.86 (s, 2H), 1.47 (t, J = 6.1 Hz, 2H), 1.00 (s, 6H). LCMS (ESI) calcd for C₅₉H₆₈ClF₃N₉O₇S₃⁺ [M+H]⁺: 1202.40; found, 1202.1.

### Example 243: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(5-((6-(2,4-dioxotetrahydropyrimidin-1(2H)-yl)pyridin-3-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide (GT-07881)

According to the method of step 2 of Scheme I-1, the target compound (GT-07881) was prepared (white solid, 22 mg, yield 60.94%). ¹H NMR (400 MHz, DMSO-d6) δ 12.17 (s, 1H), 10.60 (s, 1H), 10.26 (s, 1H), 8.75 (s, 1H), 8.19 (s, 1H), 8.00 (d, J = 8.3 Hz, 1H), 7.77 (d, J = 8.9 Hz, 3H), 7.41 (d, J = 8.4 Hz, 2H), 7.34 - 7.20 (m, 5H), 7.15 (dd, J = 13.9, 7.7 Hz, 3H), 6.98 (t, J = 14.5 Hz, 3H), 4.39 (d, J = 107.1 Hz, 3H), 4.08 (s, 2H), 3.89 (d, J = 12.4 Hz, 5H), 3.57 (s, 10H), 3.33 - 2.98 (m, 5H), 2.72 (dd, J = 24.9, 18.6 Hz, 5H), 2.22 (d, J = 47.4 Hz, 7H), 1.92 (s, 2H), 1.47 (t, J = 6.2 Hz, 2H), 1.00 (s, 6H). LCMS (ESI) calcd for C₅₉H₆₈ClF₃N₉O₇S₃⁺ [M+H]⁺: 1202.40; found, 1202.1.

### Example 244: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(5-(3-((2,6-dioxopiperidin-3-yl)amino)benzyl)-2,5-diazabicyclo[2.2.2]octan-2-yl)-1-(phenylthio)butan-2-yl)amino)-3-((trifluoromethyl)sulfonyl)phenyl)sulfonyl)benzamide (GT-07882)

According to the method of step 2 of Scheme I-1, the target compound (GT-07882) was prepared (white solid, 20 mg, yield 46.98%). ¹H NMR (400 MHz, DMSO-d6) δ 12.17 (s, 1H), 11.48 (s, 1H), 10.85 (s, 1H), 10.24 (s, 1H), 8.18 (s, 1H), 8.00 (d, J = 8.6 Hz, 1H), 7.77 (d, J = 8.9 Hz, 2H), 7.41 (d, J = 8.4 Hz, 2H), 7.33 - 7.21 (m, 5H), 7.19 - 7.08 (m, 5H), 7.05 - 6.83 (m, 5H), 6.79 (s, 1H), 4.55 - 4.11 (m, 5H), 3.72 (d, J = 119.1 Hz, 9H), 3.35 (s, 8H), 2.77 (d, J = 12.0 Hz, 4H), 2.59 (d, J = 17.7 Hz, 2H), 2.22 (d, J = 48.6 Hz, 9H), 1.88 (d, J = 12.3 Hz, 3H), 1.47 (t, J = 6.1 Hz, 2H), 1.00 (s, 6H). LCMS (ESI) calcd for C₆₁H₇₁ClF₃N₈O₇S₃⁺ [M+H]⁺: 1215.42; found, 1215.1.

### Example 245: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(5-((2-(2,4-dioxotetrahydropyrimidin-1(2H)-yl)-5-fluoropyridin-4-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide (GT-07883)

According to the method of step 2 of Scheme I-1, the target compound (GT-07883) was prepared (white solid, 17 mg, yield 39.77%). ¹H NMR (400 MHz, DMSO-d6) δ 12.16 (s, 1H), 10.54 (s, 2H), 10.08 (s, 1H), 8.35 (d, J = 8.1 Hz, 1H), 8.18 (d, J = 2.5 Hz, 1H), 8.00 (d, J = 9.4 Hz, 1H), 7.87 (s, 1H), 7.77 (d, J = 8.9 Hz, 2H), 7.41 (d, J = 8.4 Hz, 2H), 7.33 - 7.20 (m, 5H), 7.20 - 7.14 (m, 3H), 6.96 (d, J = 9.0 Hz, 3H), 4.22 (s, 1H), 4.06 - 3.83 (m, 6H), 3.54 (d, J = 30.6 Hz, 7H), 3.35 - 3.14 (m, 8H), 2.96 - 2.66 (m, 7H), 2.16 (t, J = 45.8 Hz, 8H), 1.91 - 1.63 (m, 2H), 1.48 (d, J = 6.0 Hz, 2H), 1.01 (s, 6H). LCMS (ESI) calcd for C₅₉H₆₇ClF₄N₉O₇S₃⁺ [M+H]⁺: 1220.40; found, 1220.1.

### Example 246: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,4-dioxotetrahydropyrimidin-1(2H)-yl)pyridin-4-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide (GT-07884)

According to the method of step 2 of Scheme I-1, the target compound (GT-07884) was prepared (white solid, 21 mg, yield 49.86%). ¹H NMR (400 MHz, DMSO-d6) δ 12.17 (s, 1H), 10.57 (s, 1H), 10.28 (s, 1H), 8.41 (s, 1H), 8.18 (s, 1H), 7.99 (s, 1H), 7.77 (d, J = 8.9 Hz, 2H), 7.41 (d, J = 8.4 Hz, 2H), 7.34 - 7.12 (m, 9H), 6.98 (dd, J = 19.6, 9.0 Hz, 3H), 4.25 (s, 2H), 4.12 - 3.83 (m, 6H), 3.57 (s, 9H), 3.37 - 3.11 (m, 7H), 2.84 - 2.61 (m, 6H), 2.22 (d, J = 46.8 Hz, 7H), 1.79 (s, 2H), 1.47 (t, J = 6.4 Hz, 2H), 1.00 (s, 6H). LCMS (ESI) calcd for C₅₉H₆₈ClF₃N₉O₇S₃⁺ [M+H]⁺: 1202.40; found, 1202.1.

### Example 247: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(5-((5-(2,4-dioxotetrahydropyrimidin-1(2H)-yl)pyridin-3-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide (GT-08041)

According to the method of step 2 of Scheme I-1, the target compound (GT-08041) was prepared (white solid, 14 mg, yield 33.25%). ¹H NMR (400 MHz, DMSO-d6) δ 12.17 (s, 1H), 10.60 (d, J = 10.3 Hz, 1H), 10.42 (s, 1H), 8.72 (s, 1H), 8.19 (s, 1H), 7.99 (s, 1H), 7.78 (d, J = 8.9 Hz, 2H), 7.41 (d, J = 8.4 Hz, 2H), 7.30 (d, J = 7.4 Hz, 3H), 7.26 - 7.13 (m, 6H), 6.99 (dd, J = 22.0, 8.9 Hz, 3H), 4.42 (d, J = 130.0 Hz, 3H), 4.02 - 3.58 (m, 12H), 3.33 (dd, J = 27.8, 9.4 Hz, 11H), 2.75 (t, J = 6.5 Hz, 4H), 2.23 (d, J = 39.9 Hz, 7H), 1.86 (d, J = 29.9 Hz, 2H), 1.47 (t, J = 6.1 Hz, 2H), 1.00 (s, 6H). LCMS (ESI) calcd for C₅₉H₆₈ClF₃N₉O₇S₃⁺ [M+H]⁺: 1202.40; found, 1202.1.

### Example 248: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(5-((5-(2,6-dioxopiperidin-3-yl)pyridin-3-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide (GT-08042)

According to the method of step 2 of Scheme I-1, the target compound (GT-08042) was prepared (white solid, 18 mg, yield 42.78%). ¹H NMR (400 MHz, DMSO-d6) δ 12.17 (s, 1H), 10.98 (d, J = 13.7 Hz, 1H), 10.43 (s, 1H), 8.89 (s, 1H), 8.63 (s, 1H), 8.33 (s, 1H), 8.19 (s, 1H), 7.98 (s, 1H), 7.78 (d, J = 8.9 Hz, 2H), 7.41 (d, J = 8.4 Hz, 2H), 7.34 - 7.20 (m, 5H), 7.16 (t, J = 8.5 Hz, 3H), 6.99 (dd, J = 21.5, 8.7 Hz, 3H), 4.28 (s, 3H), 4.11 - 3.70 (m, 10H), 3.36 (d, J = 12.1 Hz, 12H), 2.87 - 2.71 (m, 3H), 2.61 (d, J = 17.3 Hz, 1H), 2.34 - 2.06 (m, 9H), 1.83 (s, 2H), 1.47 (t, J = 6.2 Hz, 2H), 1.00 (s, 6H). LCMS (ESI) calcd for C₆₀H₆₉ClF₃N₈O₇S₃⁺ [M+H]⁺: 1201.41; found, 1201.1.

### Example 249: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(5-((5-(2,4-dioxotetrahydropyrimidin-1(2H)-yl)-2-fluoropyridin-3-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide (GT-08043)

According to the method of step 2 of Scheme I-1, the target compound (GT-08043) was prepared (white solid, 10 mg, yield 23.40%). ¹H NMR (400 MHz, DMSO-d6) δ 12.17 (s, 1H), 10.56 (s, 1H), 10.32 (s, 1H), 8.18 (d, J = 2.0 Hz, 2H), 7.99 (d, J = 8.8 Hz, 1H), 7.77 (d, J = 8.9 Hz, 2H), 7.41 (d, J = 8.4 Hz, 2H), 7.22 (ddd, J = 25.2, 20.3, 7.4 Hz, 9H), 6.98 (dd, J = 19.4, 8.8 Hz, 3H), 4.24 (s, 1H), 3.89 (d, J = 13.7 Hz, 6H), 3.57 (s, 6H), 3.40 - 3.08 (m, 12H), 2.75 (s, 4H), 2.22 (d, J = 45.1 Hz, 8H), 1.83 (d, J = 53.4 Hz, 2H), 1.47 (t, J = 6.1 Hz, 2H), 1.00 (s, 6H). LCMS (ESI) calcd for C₅₉H₆₇ClF₄N₉O₇S₃⁺ [M+H]⁺: 1220.40; found, 1220.1.

### Example 250: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-(4-(2,6-dioxopiperidin-3-yl)benzyl)-2,5-diazabicyclo[2.2.1]heptan-2-yl)-1-(phenylthio)butan-2-yl)amino)-3-((trifluoromethyl)sulfonyl)phenyl)sulfonyl)benzamide (GT-08044)

According to the method of step 2 of Scheme I-1, the target compound (GT-08044) was prepared (white solid, 20 mg, yield 47.45%). ¹H NMR (400 MHz, DMSO-d6) δ 12.17 (s, 2H), 10.86 (d, J = 7.5 Hz, 1H), 10.44 (s, 1H), 8.18 (s, 1H), 7.97 (s, 1H), 7.74 (t, J = 20.2 Hz, 4H), 7.41 (t, J = 7.1 Hz, 3H), 7.21 (ddd, J = 23.2, 17.8, 7.5 Hz, 12H), 6.97 (t, J = 12.5 Hz, 3H), 4.38 (d, J = 50.3 Hz, 5H), 3.86 (ddd, J = 27.5, 11.6, 6.9 Hz, 5H), 3.57 (s, 8H), 3.32 - 3.07 (m, 4H), 2.82 - 2.65 (m, 4H), 2.32 - 2.15 (m, 8H), 2.08 - 1.99 (m, 2H), 1.47 (t, J = 6.1 Hz, 2H), 1.00 (s, 6H). LCMS (ESI) calcd for C₆₀H₆₈ClF₃N₇O₇S₃⁺ [M+H]⁺: 1186.40; found, 1186.1.

### Example 251: Preparation of 4-(4-((4'-chloro-4,4-dimethyl-3.4,5,6-tetrahydro-[1,1'-biphenyl]-2-yl)methyl)piperazin-1-yl)-N-((4-(((R)-4-((1R,4R)-5-(4-(2,4-dioxotetrahydropyrimidin-1(2H)-yl)benzyl)-2,5-diazabicyclo[2.2.1]heptan-2-yl)-1-(phenylthio)butan-2-yl)amino)-3-((trifluoromethyl)sulfonyl)phenyl)sulfonyl)benzamide (GT-08045)

According to the method of step 2 of Scheme I-1, the target compound (GT-08045) was prepared (white solid, 25 mg, yield 59.27%). ¹H NMR (400 MHz, DMSO-d6) δ 12.17 (s, 2H), 10.43 (s, 2H), 8.18 (s, 1H), 7.98 (s, 1H), 7.77 (d, J = 8.7 Hz, 4H), 7.41 (d, J = 8.3 Hz, 4H), 7.22 (dt, J = 16.2, 7.7 Hz, 9H), 6.98 (dd, J = 16.9, 8.8 Hz, 3H), 4.38 (d, J = 46.0 Hz, 5H), 3.96 - 3.77 (m, 5H), 3.57 (s, 9H), 3.32 - 3.06 (m, 4H), 2.73 (dd, J = 15.0, 8.4 Hz, 5H), 2.23 (d, J = 41.3 Hz, 7H), 1.48 (d, J = 5.7 Hz, 2H), 1.00 (s, 6H). LCMS (ESI) calcd for C₅₉H₆₇ClF₃N₈O₇S₃⁺ [M+H]⁺: 1187.39; found, 1187.1.

### Example 252: Preparation of 4-(4-((4'-chloro-4,4-dimethyl-3,4,5,6-tetrahydro-[1,1'-biphenyl]-2-yl)methyl)piperazin-1-yl)-N-((4-(((R)-4-((1R,4R)-5-(3-(2,4-dioxotetrahydropyrimidin-1(2H)-yl)benzyl)-2,5-diazabicyclo[2.2.1]heptan-2-yl)-1-(phenylthio)butan-2-yl)amino)-3-((trifluoromethyl)sulfonyl)phenyl)sulfonyl)benzamide (GT-08046)

According to the method of step 2 of Scheme I-1, the target compound (GT-08046) was prepared (white solid, 30 mg, yield 71.12%). ¹H NMR (400 MHz, DMSO-d6) δ 12.16 (s, 2H), 10.42 (d, J = 18.4 Hz, 2H), 8.18 (s, 1H), 7.98 (s, 1H), 7.77 (d, J = 8.8 Hz, 2H), 7.64 (d, J = 54.6 Hz, 2H), 7.41 (t, J = 7.7 Hz, 5H), 7.34 - 7.13 (m, 10H), 6.96 (d, J = 9.0 Hz, 3H), 4.38 (dd, J = 81.4, 30.7 Hz, 5H), 3.81 (dd, J = 19.2, 12.5 Hz, 7H), 3.57 (s, 7H), 3.31 - 3.06 (m, 3H), 2.86 - 2.67 (m, 6H), 2.34 - 2.02 (m, 7H), 1.47 (t, J = 6.1 Hz, 2H), 1.00 (s, 6H). LCMS (ESI) calcd for C₅₉H₆₇ClF₃N₈O₇S₃⁺ [M+H]⁺: 1187.39; found, 1187.1.

### Example 253: Preparation of 4-(4-((4'-chloro-4,4-dimethyl-3,4,5,6-tetrahydro-[1,1'-biphenyl]-2-yl)methyl)piperazin-1-yl)-N-((4-(((R)-4-((1R,4R)-5-((5-(2,4-dioxotetrahydropyrimidin-1(2H)-yl)pyridin-2-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide (GT-08047)

According to the method of step 2 of Scheme I-1, the target compound (GT-08047) was prepared (white solid, 27 mg, yield 63.95%). ¹H NMR (400 MHz, DMSO-d6) δ 12.02 (d, J = 128.4 Hz, 2H), 10.57 (s, 2H), 8.64 (d, J = 2.0 Hz, 1H), 8.18 (s, 1H), 7.97 (s, 1H), 7.91 - 7.85 (m, 1H), 7.77 (d, J = 8.8 Hz, 2H), 7.72 - 7.58 (m, 1H), 7.41 (d, J = 8.3 Hz, 2H), 7.22 (ddd, J = 24.7, 19.9, 7.7 Hz, 9H), 6.98 (dd, J = 17.8, 8.8 Hz, 3H), 4.40 (d, J = 68.3 Hz, 7H), 3.97 - 3.81 (m, 5H), 3.56 (s, 8H), 3.30 - 3.08 (m, 4H), 2.75 (t, J = 6.6 Hz, 4H), 2.23 (d, J = 32.9 Hz, 7H), 1.46 (t, J = 6.0 Hz, 2H), 1.00 (s, 6H). LCMS (ESI) calcd for C₅₈H₆₆ClF₃N₉O₇S₃⁺ [M+H]⁺: 1188.39; found, 1188.1.

### Example 254: Preparation of 4-(4-((4'-chloro-4,4-dimethyl-3.4,5,6-tetrahydro-[1,1'-biphenyl]-2-yl)methyl)piperazin-1-yl)-N-((4-(((R)-4-((1R,4R)-5-((6-(2,4-dioxotetrahydropyrimidin-1(2H)-yl)pyridin-3-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide (GT-08048)

According to the method of step 2 of Scheme I-1, the target compound (GT-08048) was prepared (white solid, 27 mg, yield 63.95%). ¹H NMR (400 MHz, DMSO-d6) δ 12.17 (s, 2H), 10.61 (s, 1H), 10.37 (s, 1H), 8.69 (s, 1H), 8.18 (s, 2H), 7.98 (s, 1H), 7.80 (dd, J = 23.1, 8.7 Hz, 3H), 7.41 (d, J = 8.3 Hz, 2H), 7.34 - 7.13 (m, 9H), 6.96 (d, J = 9.0 Hz, 3H), 4.47 (s, 6H), 4.08 (t, J = 6.5 Hz, 2H), 3.89 (d, J = 12.6 Hz, 3H), 3.57 (s, 6H), 3.34 - 3.08 (m, 6H), 2.82 - 2.66 (m, 5H), 2.23 (d, J = 41.4 Hz, 7H), 1.47 (t, J = 6.0 Hz, 2H), 1.00 (s, 6H). LCMS (ESI) calcd for C₅₈H₆₆ClF₃N₉O₇S₃⁺ [M+H]⁺: 1188.39; found, 1188.1.

### Example 255: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-((1R,4R)-5-(3-((2,6-dioxopiperidin-3-yl)amino)benzyl)-2,5-diazabicyclo[2.2.1]heptan-2-yl)-1-(phenylthio)butan-2-yl)amino)-3-((trifluoromethyl)sulfonyl)phenyl)sulfonyl)benzamide (GT-08049)

According to the method of step 2 of Scheme I-1, the target compound (GT-08049) was prepared (white solid, 27 mg, yield 63.26%). ¹H NMR (400 MHz, DMSO-d6) δ 12.16 (s, 2H), 10.82 (d, J = 25.1 Hz, 1H), 10.33 (s, 1H), 8.18 (s, 1H), 7.97 (s, 1H), 7.77 (d, J = 8.7 Hz, 2H), 7.41 (d, J = 8.2 Hz, 2H), 7.32 - 7.06 (m, 11H), 6.97 (t, J = 12.1 Hz, 3H), 6.88 - 6.74 (m, 2H), 4.31 (d, J = 76.7 Hz, 7H), 3.88 (d, J = 12.0 Hz, 4H), 3.57 (s, 6H), 3.29 (d, J = 7.3 Hz, 5H), 2.78 (s, 4H), 2.61 (s, 2H), 2.22 (d, J = 43.1 Hz, 7H), 1.95 - 1.83 (m, 1H), 1.47 (d, J = 5.6 Hz, 2H), 1.00 (s, 6H). LCMS (ESI) calcd for C₆₀H₆₉ClF₃N₈O₇S₃⁺ [M+H]⁺: 1201.41; found, 1201.1.

### Example 256: Preparation of 4-(4-((4'-chloro-4,4-dimethyl-3,4,5,6-tetrahydro-[1,1'-biphenyl]-2-yl)methyl)piperazin-1-yl)-N-((4-(((R)-4-((1R,4R)-5-((2-(2,4-dioxotetrahydropyrimidin-1(2H)-yl)pyridin-4-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide (GT-08050)

According to the method of step 2 of Scheme I-1, the target compound (GT-08050) was prepared (white solid, 27 mg, yield 63.96%). ¹H NMR (400 MHz, DMSO-d6) δ 12.16 (s, 1H), 10.59 (s, 1H), 10.38 (s, 1H), 8.45 (s, 1H), 8.17 (s, 1H), 7.97 (s, 2H), 7.77 (d, J = 8.8 Hz, 2H), 7.41 (d, J = 8.3 Hz, 2H), 7.32 - 7.11 (m, 9H), 6.95 (d, J = 8.9 Hz, 3H), 4.32 (d, J = 49.0 Hz, 5H), 4.06 (t, J = 6.5 Hz, 2H), 3.88 (d, J = 11.7 Hz, 3H), 3.51 (d, J = 43.4 Hz, 7H), 3.35 - 3.07 (m, 7H), 2.87 - 2.65 (m, 5H), 2.22 (d, J = 41.7 Hz, 7H), 1.47 (d, J = 6.2 Hz, 2H), 1.00 (s, 6H). LCMS (ESI) calcd for C₅₈H₆₆ClF₃N₉O₇S₃⁺ [M+H]⁺: 1188.39; found, 1188.1.

### Example 257: Preparation of 4-(4-((4'-chloro-4,4-dimethyl-3,4,5,6-tetrahydro-[1,1'-biphenyl]-2-yl)methyl)piperazin-1-yl)-N-((4-(((R)-4-((1R,4R)-5-((5-(2,4-dioxotetrahydropyrimidin-1(2H)-yl)pyridin-3-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide (GT-08051)

According to the method of step 2 of Scheme I-1, the target compound (GT-08051) was prepared (white solid, 28 mg, yield 66.32%). ¹H NMR (400 MHz, DMSO-d6) δ 12.17 (s, 2H), 10.60 (s, 1H), 10.36 (s, 1H), 8.70 (d, J = 7.4 Hz, 1H), 8.18 (s, 2H), 7.97 (s, 1H), 7.77 (d, J = 8.7 Hz, 2H), 7.41 (d, J = 8.3 Hz, 2H), 7.32 - 7.12 (m, 9H), 6.96 (d, J = 9.0 Hz, 3H), 4.34 (d, J = 92.6 Hz, 5H), 4.03 - 3.81 (m, 5H), 3.57 (s, 7H), 3.36 (d, J = 11.5 Hz, 8H), 2.75 (t, J = 6.5 Hz, 4H), 2.33 - 2.07 (m, 7H), 1.47 (t, J = 6.1 Hz, 2H), 1.00 (s, 6H). LCMS (ESI) calcd for C₅₈H₆₆ClF₃N₉O₇S₃⁺ [M+H]⁺: 1188.39; found, 1188.1.

### Example 258: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-((1R,4R)-5-((5-(2,6-dioxopiperidin-3-yl)pyridin-3-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide (GT-08052)

According to the method of step 2 of Scheme I-1, the target compound (GT-08052) was prepared (white solid, 28 mg, yield 66.38%). ¹H NMR (400 MHz, DMSO-d6) δ 12.17 (s, 1H), 10.99 (s, 1H), 10.12 (s, 1H), 8.82 (s, 1H), 8.61 (s, 1H), 8.19 (d, J = 19.8 Hz, 2H), 7.96 (s, 1H), 7.76 (d, J = 8.7 Hz, 2H), 7.41 (d, J = 8.3 Hz, 2H), 7.31 - 7.11 (m, 9H), 6.97 (t, J = 8.9 Hz, 3H), 4.40 (dd, J = 75.9, 38.3 Hz, 7H), 4.07 (dd, J = 12.3, 4.5 Hz, 2H), 3.86 (s, 6H), 3.16 (s, 5H), 2.73 (d, J = 12.0 Hz, 4H), 2.60 (d, J = 17.3 Hz, 2H), 2.30 (d, J = 21.9 Hz, 5H), 2.13 (s, 5H), 1.47 (d, J = 5.8 Hz, 2H), 1.00 (s, 6H). LCMS (ESI) calcd for C₅₉H₆₇ClF₃N₈O₇S₃⁺ [M+H]⁺: 1187.39; found, 1187.1.

### Example 259: Preparation of 4-(4-((4'-chloro-4,4-dimethyl-3,4,5,6-tetrahydro-[1,1'-biphenyl]-2-yl)methyl)piperazin-1-yl)-N-((4-(((R)-4-((1R,4R)-5-((5-(2,4-dioxotetrahydropyrimidin-1(2H)-yl)-2-fluoropyridin-3-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide (GT-08053)

According to the method of step 2 of Scheme I-1, the target compound (GT-08053) was prepared (white solid, 23 mg, yield 72.26%). ¹H NMR (400 MHz, DMSO-d6) δ 12.17 (s, 1H), 10.57 (s, 1H), 10.37 (s, 1H), 8.21 (d, J = 25.5 Hz, 3H), 7.99 (d, J = 8.4 Hz, 1H), 7.77 (d, J = 8.8 Hz, 2H), 7.41 (d, J = 8.3 Hz, 2H), 7.33 = 7.10 (m, 9H), 6.95 (d, J = 8.9 Hz, 3H), 4.26 (s, 4H), 3.86 (s, 7H), 3.57 (s, 6H), 3.32 - 3.00 (m, 5H), 2.74 (t, J = 6.5 Hz, 5H), 2.33 - 2.07 (m, 8H), 1.47 (d, J = 5.9 Hz, 2H), 1.00 (s, 6H). LCMS (ESI) calcd for C₅₈H₆₅ClF₄N₉O₇S₃⁺ [M+H]⁺: 1206.38; found, 1206.1.

### Example 260: Preparation of (R)-4-(4-((4'-chloro-4,4-dimethyl-3,4,5,6-tetrahydro-[1,1'-biphenyl]-2-yl)methyl)piperazin-1-yl)-N-((4-((4-(4-((2-(2,4-dioxotetrahydropyrimidin-1(2H)-yl)-5-fluoropyridin-4-yl)methyl)piperazin-1-yl)-1-(phenylthio)butan-2-yl)amino)-3-((trifluoromethyl)sulfonyl)phenyl)sulfonyl)benzamide (GT-07716)

According to the method of step 2 of Scheme I-1, the target compound (GT-07716) was prepared (white solid, 27 mg, yield 50.5 %). ¹H NMR (400 MHz, DMSO-d6) δ 12.11 (s, 1H), 10.85 (s, 1H), 10.49 (s, 1H), 10.38 (s, 1H), 8.33 (s, 1H), 8.11 (d, *J =* 2.2 Hz, 1H), 7.91 (dd, *J* = 9.2, 2.1 Hz, 1H), 7.77 (d, *J =* 4.9 Hz, 1H), 7.71 (d, *J* = 9.0 Hz, 2H), 7.34 (d, *J* = 8.4 Hz, 2H), 7.28 - 7.20 (m, 2H), 7.12 (ddd, *J* = 16.2, 9.3, 4.7 Hz, 6H), 6.90 (t, *J* = 9.0 Hz, 3H), 4.14 (s, 1H), 3.94 (t, *J* = 6.6 Hz, 2H), 3.81 (d, *J* = 13.0 Hz, 2H), 3.73 (s, 2H), 3.50 (s, 3H), 3.31 - 3.28 (m, 5H), 3.09 (s, 1H), 2.94 (s, 5H), 2.70 (d, *J* = 9.8 Hz, 2H), 2.62 (t, *J* = 6.6 Hz, 3H), 2.21 (s, 2H), 2.06 (d, *J* = 43.1 Hz, 4H), 1.40 (t, *J* = 6.1 Hz, 2H), 0.96 (s, 6H). LCMS (ESI) calcd for C₅₇H₆₅ClF₄N₉O₇S₃⁺ [M+H]⁺: 1194.38; found, 1194.1.

### Example 261: Preparation of (R)-4-(4-((4'-chloro-4,4-dimethyl-3,4,5,6-tetrahydro-[1,1'-biphenyl]-2-yl)methyl)piperazin-1-yl)-N-((4-((4-(4-((2-(2,4-dioxotetrahydropyrimidin-1(2H)-yl)pyridin-4-yl)methyl)piperazin-1-yl)-1-(phenylthio)butan-2-yl)amino)-3-((trifluoromethyl)sulfonyl)phenyl)sulfonyl)benzamide (GT-07717)

According to the method of step 2 of Scheme I-1, the target compound (GT-07717) was prepared (white solid, 26 mg, yield 49.3 %). ¹H NMR (400 MHz, DMSO-d6) δ 12.10 (s, 1H), 10.50 (s, 1H), 10.32 (s, 1H), 8.35 (d, *J* = 4.8 Hz, 1H), 8.11 (d, *J* = 2.2 Hz, 1H), 7.91 (dd, *J* = 9.3, 2.0 Hz, 1H), 7.76 (s, 1H), 7.70 (d, *J* = 8.9 Hz, 2H), 7.34 (d, *J* = 8.4 Hz, 2H), 7.24 - 7.21 (m, 2H), 7.16 (dd, *J* = 10.1, 4.9 Hz, 3H), 7.10 (t, *J* = 8.0 Hz, 3H), 6.90 (t, *J* = 10.6 Hz, 3H), 4.15 (s, 1H), 3.99 (t, *J* = 6.6 Hz, 2H), 3.82 (d, *J* = 12.8 Hz, 2H), 3.50 (s, 3H), 3.39 (s, 2H), 3.32 - 3.28 (m, 8H), 3.09 (s, 6H), 2.75 - 2.68 (m, 2H), 2.62 (t, *J* = 6.6 Hz, 2H), 2.21 (s, 2H), 2.11 (s, 4H), 1.40 (t, *J* = 6.1 Hz, 2H), 0.93 (s, 6H). LCMS (ESI) calcd for C₅₇H₆₆ClF₃N₉O₇S₃⁺ [M+H]⁺: 1176.39; found, 1176.1.

### Example 262: Preparation of (R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)pyridin-3-yl)methyl)piperazin-1-yl)-1-(phenylthio)butan-2-yl)amino)-3-((trifluoromethyl)sulfonyl)phenyl)sulfonyl)benzamide (GT-07741)

According to the method of step 2 of Scheme I-1, the target compound (GT-07741) was prepared (white solid, 31 mg, yield 58.8 %). ¹H NMR (400 MHz, DMSO-d6) δ 12.10 (s, 1H), 10.54 (s, 1H), 10.28 (s, 1H), 8.63 (d, *J* = 2.1 Hz, 1H), 8.50 (s, 1H), 8.11 (d, *J* = 2.2 Hz, 1H), 8.00 (s, 1H), 7.91 (dd, *J* = 9.3, 2.1 Hz, 1H), 7.70 (d, *J* = 9.0 Hz, 2H), 7.35 (d, *J* = 8.4 Hz, 2H), 7.24 - 7.20 (m, 2H), 7.15 (t, *J* = 7.5 Hz, 3H), 7.13 - 7.07 (m, 3H), 6.94 - 6.86 (m, 3H), 4.14 (s, 2H), 3.87 - 3.75 (m, 5H), 3.50 (s, 5H), 3.30 (s, 6H), 3.17 - 2.93 (m, 6H), 2.68 (t, *J* = 6.6 Hz, 5H), 2.21 (s, 2H), 2.10 (s, 4H), 1.40 (t, *J* = 6.2 Hz, 2H), 0.93 (s, 6H). LCMS (ESI) calcd for C₅₇H₆₆ClF₃N₉O₇S₃ ⁺ [M+H]⁺: 1176.39; found, 1176.1.

### Example 263: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)-2-fluoropyridin-3-yl)methyl)piperazin-1-yl)-1-(phenylthio)butan-2-yl)amino)-3-((trifluoromethyl)sulfonyl)phenyl)sulfonyl)benzamide (GT-07742)

According to the method of step 2 of Scheme I-1, the target compound (GT-07742) was prepared (white solid, 24 mg, yield 44.9 %). ¹H NMR (400 MHz, DMSO-d6) δ 12.10 (s, 1H), 10.87 (s, 1H), 10.17 (s, 1H), 8.11 (d, *J* = 2.2 Hz, 1H), 8.02 (s, 1H), 7.94 - 7.89 (m, 1H), 7.84 (s, 1H), 7.70 (d, *J* = 8.9 Hz, 2H), 7.35 (d, *J* = 8.5 Hz, 2H), 7.23 - 7.20 (m, 2H), 7.18 - 7.05 (m, 7H), 6.90 (t, *J* = 7.8 Hz, 3H), 4.13 (s, 1H), 3.94 (dd, *J* = 12.6, 4.8 Hz, 1H), 3.82 (d, *J* = 12.5 Hz, 3H), 3.73 - 3.60 (m, 2H), 3.50 (s, 3H), 3.40 - 3.36 (m, 6H), 3.13 - 3.05 (m, 2H), 2.98 (s, 4H), 2.65 (dd, *J* = 21.5, 8.7 Hz, 5H), 2.51 (d, *J* = 17.4 Hz, 2H), 2.20 (d, *J* = 7.9 Hz, 3H), 2.09 (s, 4H), 1.41 (d, *J* = 6.3 Hz, 2H), 0.94 (s, 6H). LCMS (ESI) calcd for C₅₈H₆₆ClF₄N₈O₇S₃⁺ [M+H]⁺: 1193.38; found, 1193.1.

### Example 264: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)pyridin-4-yl)methyl)piperazin-1-yl)-1-(phenylthio)butan-2-yl)amino)-3-((trifluoromethyl)sulfonyl)phenyl)sulfonyl)benzamide (GT-07743)

According to the method of step 2 of Scheme I-1, the target compound (GT-07743) was prepared (white solid, 25 mg, yield 47.4 %). ¹H NMR (400 MHz, DMSO-d6) δ 12.18 (s, 1H), 10.98 (s, 1H), 10.33 (s, 1H), 8.18 (d, *J* = 2.0 Hz, 1H), 7.99 (d, *J =* 9.2 Hz, 1H), 7.78 (d, *J =* 8.9 Hz, 2H), 7.59 - 7.50 (m, 1H), 7.42 (d, *J* = 8.4 Hz, 2H), 7.30 (d, *J* = 7.6 Hz, 2H), 7.23 (t, *J* = 7.5 Hz, 3H), 7.17 (t, *J* = 7.8 Hz, 3H), 6.98 (t, J = 10.5 Hz, 3H), 4.27 - 4.08 (m, 2H), 3.89 (d, *J* = 13.2 Hz, 4H), 3.58 (s, 3H), 3.37 - 3.33 (m, 6H), 3.14 (s, 8H), 2.85 - 2.72 (m, 3H), 2.68 - 2.55 (m, 2H), 2.28 (s, 3H), 2.17 (s, 5H), 1.47 (t, *J* = 6.2 Hz, 2H), 1.01 (s, 6H). LCMS (ESI) calcd for C₅₈H₆₇ClF₃N₈O₇S₃⁺ [M+H]⁺: 1175.39; found, 1175.1.

### Example 265: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)pyridin-2-yl)methyl)piperazin-1-yl)-1-(phenylthio)butan-2-yl)amino)-3-((trifluoromethyl)sulfonyl)phenyl)sulfonyl)benzamide (GT-07744)

According to the method of step 2 of Scheme I-1, the target compound (GT-07744) was prepared (white solid, 30 mg, yield 56.9 %). ¹H NMR (400 MHz, DMSO-d6) δ 12.11 (s, 1H), 10.89 (s, 1H), 10.34 (s, 1H), 8.49 (s, 1H), 8.11 (d, *J =* 2.0 Hz, 1H), 7.92 (dd, *J =* 9.3, 2.0 Hz, 1H), 7.81 (d, *J =* 7.8 Hz, 1H), 7.70 (d, *J* = 8.9 Hz, 2H), 7.57 (d, *J* = 7.7 Hz, 1H), 7.34 (d, *J* = 8.4 Hz, 2H), 7.22 (d, *J* = 7.4 Hz, 2H), 7.15 (t, *J* = 7.5 Hz, 3H), 7.10 (t, *J* = 8.2 Hz, 3H), 6.94 - 6.87 (m, 3H), 4.15 (s, 3H), 3.99 (dd, *J* = 12.6, 4.9 Hz, 1H), 3.81 (d, *J =* 13.8 Hz, 2H), 3.50 (s, 3H), 3.27 - 3.22 (m, 6H), 3.16 - 3.03 (m, 6H), 2.76 - 2.59 (m, 4H), 2.55 - 2.46 (m, 2H), 2.21 (s, 3H), 2.11 (s, 4H), 2.00 - 1.95 (m, 1H), 1.40 (t, *J* = 6.2 Hz, 2H), 0.93 (s, 6H). LCMS (ESI) calcd for C₅₈H₆₇ClF₃N₈O₇S₃ ⁺ [M+H]⁺: 1175.39; found, 1175.1.

### Example 266: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)pyridin-3-yl)methyl)piperazin-1-yl)-1-(phenylthio)butan-2-yl)amino)-3-((trifluoromethyl)sulfonyl)phenyl)sulfonyl)benzamide (GT-07745)

According to the method of step 2 of Scheme I-1, the target compound (GT-07745) was prepared (white solid, 26 mg, yield 49.3 %). ¹H NMR (400 MHz, DMSO-d6) δ 12.18 (s, 1H), 11.00 (s, 1H), 10.35 (s, 1H), 8.75 (s, 1H), 8.65 (s, 1H), 8.17 (d, 2H), 7.99 (d, *J* = 9.4 Hz, 1H), 7.78 (d, *J* = 8.9 Hz, 2H), 7.42 (d, *J =* 8.4 Hz, 2H), 7.29 (d, *J* = 7.2 Hz, 2H), 7.26 - 7.12 (m, 7H), 6.98 (t, *J* = 9.9 Hz, 3H), 4.29 - 4.02 (m, 4H), 3.89 (d, *J* = 12.6 Hz, 3H), 3.57 (s, 4H), 3.39 (s, 5H), 3.23 - 3.06 (m, 6H), 2.83 - 2.71 (m, 4H), 2.64 - 2.54 (m, 2H), 2.33 - 2.26 (m, 3H), 2.21 - 2.08 (m, 5H), 1.47 (t, *J* = 6.1 Hz, 2H), 1.01 (s, 6H). LCMS (ESI) calcd for C₅₈H₆₇ClF₃N₈O₇S₃ ⁺ [M+H]⁺: 1175.39; found, 1175.1.

### Example 267: Preparation of (R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)-2-fluoropyridin-3-yl)methyl)piperazin-1-yl)-1-(phenylthio)butan-2-yl)amino)-3-((trifluoromethyl)sulfonyl)phenyl)sulfonyl)benzamide (GT-07746)

According to the method of step 2 of Scheme I-1, the target compound (GT-07746) was prepared (white solid, 26 mg, yield 48.6 %). ¹H NMR (400 MHz, DMSO-d6) δ 12.10 (s, 1H), 10.48 (s, 2H), 10.14 (s, 1H), 8.11 (t, *J* = 5.5 Hz, 2H), 7.99 - 7.86 (m, 2H), 7.70 (d, *J* = 8.9 Hz, 2H), 7.34 (d, *J* = 8.4 Hz, 2H), 7.22 (d, *J* = 7.3 Hz, 2H), 7.18 - 7.07 (m, 6H), 6.90 (t, *J* = 8.5 Hz, 3H), 4.11 (s, 1H), 3.84 - 3.75 (m, 4H), 3.66 (s, 1H), 3.50 (s, 3H), 3.31 (dd, *J* = 15.9, 11.4 Hz, 8H), 3.09 (s, 1H), 2.96 (s, 5H), 2.75 - 2.52 (m, 6H), 2.21 (s, 2H), 2.09 (s, 4H), 1.40 (t, *J* = 6.2 Hz, 2H), 0.94 (s, 6H). LCMS (ESI) calcd for C₅₇H₆₅ClF₄N₉O₇S₃⁺ [M+H]⁺: 1194.38; found, 1194.1.

### Example 268: Preparation of (R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)pyrazin-2-yl)methyl)piperazin-1-yl)-1-(phenylthio)butan-2-yl)amino)-3-((trifluoromethyl)sulfonyl)phenyl)sulfonyl)benzamide (GT-07747)

According to the method of step 2 of Scheme I-1, the target compound (GT-07747) was prepared (white solid, 23 mg, yield 43.6 %). ¹H NMR (400 MHz, DMSO-d6) δ 12.10 (s, 1H), 10.70 (s, 1H), 10.40 (s, 1H), 9.00 (s, 1H), 8.54 (s, 1H), 8.11 (d, *J* = 2.1 Hz, 1H), 7.91 (dd, *J* = 9.2, 1.9 Hz, 1H), 7.70 (d, *J* = 8.9 Hz, 2H), 7.34 (d, *J* = 8.4 Hz, 2H), 7.22 (d, *J* = 7.4 Hz, 2H), 7.15 (t, *J* = 7.5 Hz, 3H), 7.10 (t, *J* = 8.1 Hz, 3H), 6.90 (t, *J =* 11.1 Hz, 3H), 4.13 (s, 1H), 3.99 (t, *J* = 6.5 Hz, 2H), 3.81 (d, *J* = 13.0 Hz, 2H), 3.50 (s, 2H), 3.34 - 3.24 (m, 10H), 3.22 - 2.82 (m, 8H), 2.72 - 2.63 (m, 4H), 2.21 (s, 2H), 2.11 (s, 4H), 1.40 (t, *J* = 6.2 Hz, 2H), 0.93 (s, 6H). LCMS (ESI) calcd for C₅₆H₆₅ClF₃N₁₀O₇S₃ ⁺ [M+H]⁺: 1177.38; found, 1177.1.

### Example 269: Preparation of (R)-4-(4-((4'-chloro-4,4-dimethyl-3,4,5,6-tetrahydro-[1,1'-biphenyl]-2-yl)methyl)piperazin-1-yl)-N-((4-((4-(4-(4-(2,4-dioxotetrahydropyrimidin-1(2H)-yl)benzyl)piperazin-1-yl)-1-(phenylthio)butan-2-yl)amino)-3-((trifluoromethyl)sulfonyl)phenyl)sulfonyl)benzamide (GT-07752)

According to the method of step 2 of Scheme I-1, the target compound (GT-07752) was prepared (white solid, 25 mg, yield 47.4 %). ¹H NMR (400 MHz, DMSO-d6) δ 12.17 (s, 1H), 10.44 (s, 1H), 10.29 (s, 1H), 8.18 (d, *J* = 2.0 Hz, 1H), 7.99 (d, *J =* 9.3 Hz, 1H), 7.78 (d, *J =* 8.9 Hz, 2H), 7.60 (s, 1H), 7.42 (d, *J =* 8.4 Hz, 4H), 7.29 (d, *J* = 7.3 Hz, 2H), 7.26 - 7.14 (m, 6H), 7.01 - 6.93 (m, 3H), 4.21 (s, 2H), 3.89 (d, *J* = 12.5 Hz, 2H), 3.82 (t, *J* = 6.6 Hz, 2H), 3.58 (s, 2H), 3.42 - 3.34 (m, 10H), 3.22 (s, 6H), 2.78 (d, *J* = 9.3 Hz, 2H), 2.72 (t, *J* = 6.6 Hz, 2H), 2.29 (s, 2H), 2.17 (s, 3H), 1.48 (t, *J* = 6.2 Hz, 2H), 1.01 (s, 6H). LCMS (ESI) calcd for C₅₈H₆₇ClF₃N₈O₇S₃⁺ [M+H]⁺: 1175.39; found, 1175.1.

### Example 270: Preparation of (R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)pyridin-2-yl)methyl)piperazin-1-yl)-1-(phenylthio)butan-2-yl)amino)-3-((trifluoromethyl)sulfonyl)phenyl)sulfonyl)benzamide (GT-07753)

According to the method of step 2 of Scheme I-1, the target compound (GT-07753) was prepared (white solid, 23 mg, yield 43.6 %). ¹H NMR (400 MHz, DMSO-d6) δ 12.18 (s, 1H), 10.58 (s, 1H), 10.44 (s, 1H), 8.66 (d, *J* = 2.3 Hz, 1H), 8.18 (d, *J* = 2.1 Hz, 1H), 7.99 (dd, *J* = 9.3, 2.1 Hz, 1H), 7.90 (dd, *J* = 8.4, 2.5 Hz, 1H), 7.78 (d, *J* = 8.9 Hz, 2H), 7.68 (d, *J* = 8.5 Hz, 1H), 7.42 (d, *J* = 8.4 Hz, 2H), 7.32 - 7.27 (m, 2H), 7.23 (t, *J* = 7.5 Hz, 3H), 7.17 (t, *J* = 8.1 Hz, 3H), 7.02 - 6.94 (m, 3H), 4.29 (d, *J* = 41.6 Hz, 2H), 3.87 (t, *J =* 6.6 Hz, 4H), 3.57 (s, 3H), 3.43 - 3.41 (m, 2H), 3.40 - 3.33 (m, 8H), 3.31 - 3.19 (m, 4H), 2.75 (t, *J* = 6.7 Hz, 4H), 2.28 (s, 2H), 2.19 (s, 4H), 1.47 (t, *J* = 6.0 Hz, 2H), 1.01 (s, 6H). LCMS (ESI) calcd for C₅₇H₆₆ClF₃N₉O₇S₃⁺ [M+H]⁺: 1176.39; found, 1176.1.

### Example 271: Preparation of (R)-4-(4-((4'-chloro-4,4-dimethyl-3,4,5,6-tetrahydro-[1,1'-biphenyl]-2-yl)methyl)piperazin-1-yl)-N-((4-((4-(4-((6-(2,4-dioxotetrahydropyrimidin-1(2H)-yl)pyridin-3-yl)methyl)piperazin-1-yl)-1-(phenylthio)butan-2-yl)amino)-3-((trifluoromethyl)sulfonyl)phenyl)sulfonyl)benzamide (GT-07754)

According to the method of step 2 of Scheme I-1, the target compound (GT-07754) was prepared (white solid, 23 mg, yield 43.6 %). ¹H NMR (400 MHz, DMSO-d6) δ 12.17 (s, 1H), 10.60 (s, 1H), 10.27 (s, 1H), 8.56 (s, 1H), 8.18 (d, *J* = 2.2 Hz, 1H), 7.99 (d, *J* = 9.2 Hz, 2H), 7.86 - 7.74 (m, 3H), 7.42 (d, *J* = 8.4 Hz, 2H), 7.31 - 7.27 (m, 2H), 7.25 - 7.14 (m, 6H), 7.02 - 6.94 (m, 3H), 4.21 (s, 2H), 4.08 (t, *J* = 6.5 Hz, 2H), 3.89 (d, *J* = 13.0 Hz, 2H), 3.58 (s, 3H), 3.43 - 3.30 (m, 10H), 3.30 - 2.88 (m, 6H), 2.83 - 2.74 (m, 2H), 2.70 (t, *J* = 6.6 Hz, 2H), 2.29 (s, 2H), 2.17 (s, 3H), 1.48 (t, *J* = 6.1 Hz, 2H), 1.01 (s, 6H). LCMS (ESI) calcd for C₅₇H₆₆ClF₃N₉O₇S₃ ⁺ [M+H]⁺: 1176.39; found, 1176.1.

### Example 272: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(3-(4-(2,4-dioxotetrahydropyrimidin-1(2H)-yl)benzyl)-3,6-diazabicyclo[3.1.1]heptan-6-yl)-1-(phenylthio)butan-2-yl)amino)-3-((trifluoromethyl)sulfonyl)phenyl)sulfonyl)benzamide (GT-07828)

According to the method of step 2 of Scheme I-1, the target compound (GT-07828) was prepared (white solid, 24 mg, yield 50.0 %). ¹H NMR (400 MHz, DMSO-d6) δ 12.18 (s, 1H), 10.59 - 10.24 (m, 2H), 9.19 (s, 1H), 8.18 (d, *J* = 2.3 Hz, 1H), 8.05 - 7.97 (m, 1H), 7.78 (d, *J* = 8.7 Hz, 2H), 7.42 (d, *J* = 8.4 Hz, 3H), 7.37 - 7.27 (m, 4H), 7.25 - 7.14 (m, 5H), 6.96 (d, *J* = 8.3 Hz, 2H), 4.63 (s, 1H), 4.31 (d, *J* = 46.9 Hz, 2H), 3.89 (d, *J* = 12.1 Hz, 2H), 3.83 - 3.75 (m, 2H), 3.63 (s, 1H), 3.57 (s, 2H), 3.42 - 3.32 (m, 8H), 3.00 (s, 2H), 2.85 - 2.66 (m, 5H), 2.28 (s, 2H), 2.19 - 2.06 (m, 4H), 1.48 (t, *J* = 6.1 Hz, 2H), 1.01 (s, 6H). LCMS (ESI) calcd for C₅₉H₆₇ClF₃N₈O₇S₃ ⁺ [M+H]⁺: 1187.39; found, 1187.1.

### Example 273: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(3-(3-(2,4-dioxotetrahydropyrimidin-1(2H)-yl)benzyl)-3,6-diazabicyclo[3.1.1]heptan-6-yl)-1-(phenylthio)butan-2-yl)amino)-3-((trifluoromethyl)sulfonyl)phenyl)sulfonyl)benzamide (GT-07829)

According to the method of step 2 of Scheme I-1, the target compound (GT-07829) was prepared (white solid, 31 mg, yield 64.6 %). ¹H NMR (400 MHz, DMSO-d6) δ 12.18 (s, 1H), 10.41 (s, 2H), 9.20 (s, 1H), 8.18 (s, 1H), 8.08 - 7.94 (m, 1H), 7.78 (d, *J* = 8.8 Hz, 2H), 7.42 (d, *J* = 8.4 Hz, 3H), 7.35 (d, *J* = 8.8 Hz, 1H), 7.32 - 7.27 (m, 3H), 7.25 - 7.21 (m, 2H), 7.21 - 7.17 (m, 2H), 7.17 (s, 1H), 7.16 - 7.10 (m, 1H), 6.96 (d, *J* = 8.3 Hz, 3H), 4.64 (s, 1H), 4.29 (d, *J* = 46.8 Hz, 3H), 3.92 - 3.77 (m, 5H), 3.57 (s, 3H), 3.41 - 3.34 (m, 8H), 3.09 - 2.88 (m, 2H), 2.82 - 2.69 (m, 5H), 2.28 (s, 2H), 2.20 - 2.09 (m, 4H), 1.47 (t, *J* = 6.2 Hz, 2H), 1.01 (s, 6H). LCMS (ESI) calcd for C₅₉H₆₇ClF₃N₈O₇S₃ ⁺ [M+H]⁺: 1187.39; found, 1187.2.

### Example 274: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(8-(4-(2,6-dioxopiperidin-3-yl)benzyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-07858)

According to the method of step 2 of Scheme I-1, the target compound (GT-07858) was prepared (white solid, 18 mg, yield 34.3 %). ¹H NMR (400 MHz, DMSO-d6) δ 12.08 (s, 1H), 10.80 (s, 1H), 10.29 (s, 1H), 8.10 (d, *J* = 2.1 Hz, 1H), 7.90 (dd, *J* = 9.3, 2.1 Hz, 1H), 7.70 (d, *J* = 8.9 Hz, 2H), 7.59 (s, 2H), 7.36 - 7.33 (m, 2H), 7.25 - 7.21 (m, 3H), 7.16 (dd, *J* = 11.8, 4.8 Hz, 2H), 7.12 - 7.03 (m, 5H), 6.89 (d, *J* = 9.0 Hz, 3H), 4.06 (s, 3H), 3.89 - 3.77 (m, 4H), 3.73 (dd, *J* = 11.3, 5.0 Hz, 1H), 3.67 - 3.57 (m, 1H), 3.50 (s, 2H), 3.34 - 3.32 (m, 3H), 3.32 - 3.22 (m, 6H), 2.75 - 2.63 (m, 3H), 2.63 - 2.55 (m, 2H), 2.21 (s, 4H), 2.15 - 2.06 (m, 4H), 2.01 - 1.92 (m, 2H), 1.71 (s, 1H), 1.40 (t, *J* = 6.2 Hz, 2H), 0.93 (s, 6H). LCMS (ESI) calcd for C₆₁H₇₀ClF₃N₇O₇S₃ ⁺ [M+H]⁺: 1200.41; found, 1200.1.

### Example 275: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(8-(4-(2,4-dioxotetrahydropyrimidin-1(2H)-yl)benzyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-07859)

According to the method of step 2 of Scheme I-1, the target compound (GT-07859) was prepared (white solid, 14 mg, yield 26.7 %). ¹H NMR (400 MHz, DMSO-d6) δ 12.08 (s, 1H), 10.37 (s, 1H), 10.25 (s, 1H), 8.10 (d, *J* = 2.1 Hz, 1H), 7.94 - 7.87 (m, 1H), 7.70 (d, *J* = 9.0 Hz, 2H), 7.66 - 7.60 (m, 1H), 7.36 (d, *J =* 14.0 Hz, 4H), 7.23 (d, *J* = 7.4 Hz, 2H), 7.16 (t, *J* = 7.5 Hz, 2H), 7.12 - 7.05 (m, 3H), 6.89 (d, *J* = 9.0 Hz, 3H), 4.06 (s, 3H), 3.81 (d, *J* = 12.4 Hz, 2H), 3.75 (t, *J* = 6.6 Hz, 2H), 3.53 (s, 1H), 3.50 (s, 2H), 3.38 - 3.34 (m, 6H), 3.32 - 3.21 (m, 8H), 2.70 (d, *J* = 9.0 Hz, 2H), 2.64 (t, *J* = 6.6 Hz, 2H), 2.37 - 2.16 (m, 4H), 2.10 (s, 3H), 1.90 - 1.60 (m, 2H), 1.40 (t, *J* = 6.2 Hz, 2H), 0.93 (s, 6H). LCMS (ESI) calcd for C₆₀H₆₉ClF₃N₈O₇S₃ ⁺ [M+H]⁺: 1201.41; found, 1201.1.

### Example 276: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(8-(3-(2,4-dioxotetrahydropyrimidin-1(2H)-yl)benzyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-07860)

According to the method of step 2 of Scheme I-1, the target compound (GT-07860) was prepared (white solid, 14 mg, yield 26.7 %). ¹H NMR (400 MHz, DMSO-d6) δ 12.09 (s, 1H), 10.38 (s, 1H), 10.26 (s, 1H), 8.10 (d, *J* = 2.1 Hz, 1H), 7.90 (dd, *J* = 9.3, 2.1 Hz, 1H), 7.70 (d, *J* = 9.0 Hz, 2H), 7.63 (s, 1H), 7.48 (s, 1H), 7.43 - 7.32 (m, 4H), 7.23 (d, *J* = 7.5 Hz, 2H), 7.16 (t, *J* = 7.5 Hz, 2H), 7.10 (t, *J* = 8.0 Hz, 3H), 6.89 (d, *J* = 9.0 Hz, 3H), 4.07 (d, *J* = 25.4 Hz, 3H), 3.89 - 3.76 (m, 5H), 3.50 (s, 2H), 3.48 - 3.39 (m, 4H), 3.36 - 3.35 (m, 2H), 3.35 - 3.20 (m, 8H), 2.70 (d, *J* = 10.0 Hz, 2H), 2.68 - 2.59 (m, 3H), 2.34 - 2.26 (m, 1H), 2.21 (s, 3H), 2.10 (s, 3H), 1.71 (s, 1H), 1.40 (t, *J* = 6.2 Hz, 2H), 0.94 (s, 6H). LCMS (ESI) calcd for C₆₀H₆₉ClF₃N₈O₇S₃ ⁺ [M+H]⁺: 1201.41; found, 1201.1.

### Example 277: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(8-((5-(2,4-dioxotetrahydropyrimidin-1(2H)-yl)pyridin-2-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-07861)

According to the method of step 2 of Scheme I-1, the target compound (GT-07861) was prepared (white solid, 18 mg, yield 34.3 %). ¹H NMR (500 MHz, DMSO) δ 12.16 (s, 1H), 10.58 (s, 1H), 10.51 (s, 1H), 8.67 (s, 1H), 8.17 (s, 1H), 7.99 - 7.96 (m, 1H), 7.92 - 7.88 (m, 1H), 7.77 (d, *J* = 8.9 Hz, 3H), 7.41 (d, *J* = 8.3 Hz, 3H), 7.30 (d, *J* = 7.4 Hz, 3H), 7.23 (t, *J* = 7.6 Hz, 3H), 7.18 (d, *J* = 8.4 Hz, 3H), 6.96 (d, *J* = 8.8 Hz, 3H), 4.31 (s, 1H), 4.12 (s, 2H), 3.87 (t, *J* = 6.6 Hz, 5H), 3.56 (s, 4H), 3.40 - 3.33 (m, 10H), 2.75 (t, *J* = 6.6 Hz, 5H), 2.28 (s, 3H), 2.19 (s, 3H), 1.47 (t, *J* = 6.2 Hz, 3H), 1.00 (s, 9H). LCMS (ESI) calcd for C₃₉H₆₈ClF₃N₉O₇S₃⁺ [M+H]⁺: 1202.40; found, 1202.1.

### Example 278: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(8-((6-(2,4-dioxotetrahydropyrimidin-1(2H)-yl)pyridin-3-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-07862)

According to the method of step 2 of Scheme I-1, the target compound (GT-07862) was prepared (white solid, 16 mg, yield 30.5 %). ¹H NMR (500 MHz, DMSO) δ 12.15 (s, 1H), 10.61 (s, 1H), 10.36 (s, 1H), 8.66 (s, 1H), 8.17 (s, 2H), 7.98 - 7.95 (m, 1H), 7.84 (d, *J* = 8.3 Hz, 1H), 7.77 (d, *J* = 8.9 Hz, 2H), 7.41 (d, *J =* 8.4 Hz, 2H), 7.30 (d, *J* = 7.5 Hz, 2H), 7.22 (t, *J* = 7.7 Hz, 2H), 7.17 (d, *J* = 8.4 Hz, 3H), 6.96 (d, *J* = 8.9 Hz, 3H), 4.18 (s, 2H), 4.08 (t, *J* = 6.5 Hz, 3H), 3.89 (d, *J* = 12.4 Hz, 3H), 3.57 (s, 3H), 3.39 - 3.33 (m, 7H), 2.89 (s, 1H), 2.77 (d, *J =* 9.6 Hz, 3H), 2.73 (s, 1H), 2.69 (t, *J =* 6.6 Hz, 3H), 2.28 (s, 3H), 2.17 (s, 4H), 1.78 (s, 2H), 1.47 (t, *J* = 6.2 Hz, 3H), 1.24 (s, 1H), 1.00 (s, 6H). LCMS (ESI) calcd for C₅₉H₆₈ClF₃N₉O₇S₃ ⁺ [M+H]⁺: 1202.40; found, 1202.1 .

### Example 279: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(8-(3-((2,6-dioxopiperidin-3-yl)amino)benzyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-07863)

According to the method of step 2 of Scheme I-1, the target compound (GT-07863) was prepared (white solid, 22 mg, yield 41.5 %). ¹H NMR (500 MHz, DMSO) δ 12.15 (s, 1H), 10.85 (s, 1H), 10.36 (s, 1H), 8.17 (d, *J* = 2.0 Hz, 1H), 7.96 (dd, *J* = 9.3, 1.9 Hz, 1H), 7.77 (d, *J* = 8.9 Hz, 2H), 7.41 (d, *J* = 8.5 Hz, 2H), 7.29 (d, *J* = 7.5 Hz, 2H), 7.22 (t, *J* = 7.6 Hz, 2H), 7.20 - 7.10 (m, 5H), 6.96 (d, *J* = 9.1 Hz, 3H), 6.92 - 6.70 (m, 2H), 4.40 (d, *J* = 7.8 Hz, 1H), 4.15 - 3.94 (m, 4H), 3.89 (d, *J* = 12.5 Hz, 3H), 3.56 (d, *J* = 12.7 Hz, 4H), 3.40 - 3.29 (m, 7H), 3.11 (s, 1H), 2.87 - 2.67 (m, 4H), 2.61 - 2.55 (m, 1H), 2.41 - 2.21 (m, 5H), 2.17 (s, 4H), 1.92 - 1.84 (m, 1H), 1.76 (s, 1H), 1.47 (t, *J* = 6.3 Hz, 2H), 1.00 (s, 6H). LCMS (ESI) calcd for C₆₁H₇₁ClF₃N₈O₇S₃ ⁺ [M+H]⁺: 1215.42; found, 1215.1.

### Example 280: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,4-dioxotetrahydropyrimidin-1(2H)-yl)-5-fluoropyridin-4-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-07864)

According to the method of step 2 of Scheme I-1, the target compound (GT-07864) was prepared (white solid, 20 mg, yield 37.6 %). ¹H NMR (500 MHz, DMSO) δ 12.17 (s, 1H), 10.57 (s, 1H), 10.36 (s, 1H), 8.38 (s, 1H), 8.18 (d, *J* = 1.9 Hz, 1H), 7.98 (dd, *J* = 9.3, 2.0 Hz, 1H), 7.92 (s, 1H), 7.77 (d, *J* = 9.0 Hz, 2H), 7.43 (dd, *J* = 16.4, 8.4 Hz, 2H), 7.32 - 7.28 (m, 2H), 7.23 (t, *J* = 7.6 Hz, 3H), 7.17 (t, *J* = 8.1 Hz, 3H), 7.01 - 6.93 (m, 3H), 4.09 (s, 1H), 4.01 (t, *J* = 6.4 Hz, 2H), 3.89 (d, *J* = 13.3 Hz, 2H), 3.72 (s, 2H), 3.57 (s, 3H), 3.44 - 3.27 (m, 10H), 3.24 - 2.88 (m, 4H), 2.77 (d, *J* = 10.5 Hz, 2H), 2.70 (t, *J* = 6.6 Hz, 2H), 2.28 (s, 2H), 2.22 - 2.03 (m, 7H), 1.47 (t, *J* = 6.2 Hz, 2H), 1.00 (s, 6H). LCMS (ESI) calcd for C₅₉H₆₇ClF₄N₉O₇S₃ ⁺ [M+H]⁺: 1220.39 ; found, 1220.1.

### Example 281: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,4-dioxotetrahydropyrimidin-1(2H)-yl)pyridin-4-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-07865)

According to the method of step 2 of Scheme I-1, the target compound (GT-07865) was prepared (white solid, 22 mg, yield 41.9 %). ¹H NMR (500 MHz, DMSO) δ 12.16 (s, 1H), 10.60 (s, 1H), 10.40 (s, 1H), 8.47 (s, 1H), 8.17 (d, *J* = 1.7 Hz, 1H), 7.97 (dd, *J* = 9.3, 1.8 Hz, 2H), 7.77 (d, *J* = 8.9 Hz, 2H), 7.64 (s, 1H), 7.41 (d, *J* = 8.4 Hz, 2H), 7.30 (d, *J* = 7.4 Hz, 2H), 7.23 (t, *J* = 7.6 Hz, 2H), 7.20 - 7.14 (m, 3H), 6.97 (t, *J =* 9.0 Hz, 3H), 4.23 (s, 1H), 4.20 - 4.04 (m, 4H), 3.89 (d, *J* = 13.1 Hz, 3H), 3.57 (s, 3H), 3.55 - 3.52 (m, 4H), 3.46 - 3.31 (m, 8H), 3.28 - 2.95 (m, 2H), 2.84 - 2.72 (m, 3H), 2.70 (t, *J* = 6.6 Hz, 2H), 2.28 (s, 3H), 2.18 (s, 4H), 1.47 (t, *J* = 6.3 Hz, 2H), 1.00 (s, 6H). LCMS (ESI) calcd for C₅₉H₆₈ClF₃N₉O₇S₃ ⁺ [M+H]⁺: 1202.40; found, 1202.1.

### Example 282: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(8-((5-(2,4-dioxotetrahydropyrimidin-1(2H)-yl)pyridin-3-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-07866)

According to the method of step 2 of Scheme I-1, the target compound (GT-07866) was prepared (white solid, 26 mg, yield 49.5 %). ¹H NMR (500 MHz, DMSO) δ 12.16 (s, 1H), 10.70 - 10.54 (m, 1H), 10.40 (s, 1H), 8.73 (dd, *J =* 21.3, 4.6 Hz, 2H), 8.28 (s, 1H), 8.17 (d, *J =* 2.1 Hz, 1H), 7.97 (dd, *J =* 9.2, 1.9 Hz, 1H), 7.77 (d, *J* = 8.9 Hz, 2H), 7.41 (d, *J* = 8.4 Hz, 2H), 7.29 (d, *J* = 7.6 Hz, 2H), 7.22 (t, *J* = 7.6 Hz, 2H), 7.20 - 7.12 (m, 4H), 6.97 (t, *J* = 8.3 Hz, 3H), 4.48 - 4.04 (m, 4H), 3.99 - 3.84 (m, 6H), 3.57 (s, 3H), 3.42 - 3.23 (m, 8H), 3.20 - 2.93 (m, 2H), 2.85 - 2.70 (m, 5H), 2.41 - 2.11 (m, 9H), 1.47 (t, *J* = 6.3 Hz, 2H), 1.00 (s, 6H). LCMS (ESI) calcd for C₅₉H₆₈ClF₃N₉O₇S₃ ⁺ [M+H]⁺: 1202.40; found, 1202.1.

### Example 283: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(8-((5-(2,6-dioxopiperidin-3-yl)-2-fluoropyridin-3-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-07867)

According to the method of step 2 of Scheme I-1, the target compound (GT-07867) was prepared (gray solid, 18 mg, yield 33.9 %). ¹H NMR (500 MHz, DMSO) δ 12.16 (s, 1H), 10.93 (s, 1H), 10.32 (s, 1H), 8.18 (s, 1H), 7.98 (dd, *J* = 9.3, 2.0 Hz, 1H), 7.77 (d, *J =* 8.9 Hz, 2H), 7.41 (d, *J =* 8.4 Hz, 2H), 7.30 (d, *J =* 7.5 Hz, 2H), 7.23 (t, *J* = 7.6 Hz, 2H), 7.16 (dd, *J* = 17.6, 7.8 Hz, 4H), 6.96 (d, *J* = 9.0 Hz, 3H), 4.15 - 3.99 (m, 2H), 3.89 (d, *J* = 12.9 Hz, 2H), 3.57 (s, 2H), 3.52 - 3.49 (m, 2H), 3.43 - 3.28 (m, 10H), 3.13 - 2.92 (m, 2H), 2.81 - 2.70 (m, 3H), 2.62 (t, 1H), 2.31 - 2.25 (m, 3H), 2.17 (s, 5H), 2.09 - 1.99 (m, 2H), 1.97 - 1.74 (m, 2H), 1.47 (t, *J* = 6.3 Hz, 2H), 1.00 (s, 6H). LCMS (ESI) calcd forC₆₀H₆₈ClF₄N₈O₇S₃ ⁺ [M+H]⁺: 1219.40; found, 1219.1.

### Example 284: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(8-((5-(2,6-dioxopiperidin-3-yl)pyridin-3-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-07868)

According to the method of step 2 of Scheme I-1, the target compound (GT-07868) was prepared (brown solid, 15 mg, yield 28.6 %). ¹H NMR (500 MHz, DMSO) δ 12.16 (s, 1H), 11.01 (s, 1H), 10.39 (s, 1H), 8.87 (s, 1H), 8.63 (s, 1H), 8.31 (s, 1H), 8.17 (d, *J =* 2.1 Hz, 1H), 7.97 (dd, *J =* 9.3, 2.0 Hz, 1H), 7.77 (d, *J = 8.9* Hz, 2H), 7.41 (d, *J* = 8.4 Hz, 2H), 7.29 (d, *J* = 7.6 Hz, 2H), 7.22 (t, *J* = 7.6 Hz, 2H), 7.19 - 7.14 (m, 3H), 6.96 (d, *J* = 9.0 Hz, 3H), 4.27 (s, 2H), 4.11 (s, 3H), 3.88 (d, *J* = 12.6 Hz, 4H), 3.82 - 3.75 (m, 4H), 3.57 (s, 4H), 3.43 - 3.31 (m, 8H), 3.18 - 2.96 (m, 2H), 2.83 - 2.70 (m, 4H), 2.63 - 2.58 (m, 1H), 2.37 - 2.23 (m, 6H), 2.23 - 2.05 (m, 6H), 1.47 (t, *J* = 6.2 Hz, 2H). LCMS (ESI) calcd for C₆₀H₆₉ClF₃N₈O₇S₃⁺ [M+H]⁺: 1201.41; found, 1201.1 .

### Example 285: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(8-((5-(2,6-dioxopiperidin-3-yl)pyridin-2-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-07869)

According to the method of step 2 of Scheme I-1, the target compound (GT-07869) was prepared (white solid, 22 mg, yield 41.9 %). ¹H NMR (500 MHz, DMSO) δ 12.17 (s, 1H), 11.43 (s, 1H), 10.97 (s, 1H), 10.55 (s, 1H), 8.57 (s, 1H), 8.18 (d, *J =* 2.2 Hz, 1H), 7.98 (dd, *J =* 9.3, 2.1 Hz, 1H), 7.88 (s, 1H), 7.78 (d, *J* = 9.0 Hz, 2H), 7.71 (s, 1H), 7.41 (d, *J* = 8.5 Hz, 2H), 7.32 - 7.28 (m, 2H), 7.23 (t, *J* = 7.6 Hz, 2H), 7.19 - 7.14 (m, 3H), 6.97 (t, *J* = 9.7 Hz, 3H), 4.32 (s, 2H), 4.17 - 3.97 (m, 4H), 3.88 (d, *J* = 12.7 Hz, 2H), 3.56 (s, 4H), 3.54 - 3.52 (m, 2H), 3.43 - 3.30 (m, 10H), 2.84 - 2.66 (m, 4H), 2.60 - 2.55 (m, 1H), 2.35 - 2.23 (m, 5H), 2.19 (s, 3H), 2.06 - 2.02 (m, 1H), 1.47 (t, *J* = 6.3 Hz, 2H), 1.00 (s, 6H). LCMS (ESI) calcd for C₆₀H₆₉ClF₃N₈O₇S₃⁺ [M+H]⁺: 1201.41; found, 1201.1.

### Example 286: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(8-((5-(2,4-dioxotetrahydropyrimidin-1(2H)-yl)-2-fluoropyridin-3-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-07870)

According to the method of step 2 of Scheme I-1, the target compound (GT-07870) was prepared (white solid, 23 mg, yield 43.2 %). ¹H NMR (500 MHz, DMSO) δ 12.18 (s, 1H), 10.54 (s, 2H), 8.16 (t, *J =* 11.1 Hz, 2H), 8.06 - 7.95 (m, 2H), 7.77 (d, *J =* 9.0 Hz, 2H), 7.41 (d, *J =* 8.4 Hz, 2H), 7.30 (dd, *J =* 7.5, 6.1 Hz, 2H), 7.26 - 7.21 (m, 3H), 7.20 -7.14 (m, 3H), 6.97 (t, *J =* 8.5 Hz, 3H), 4.19 (s, 1H), 3.97 (d, *J =* 18.1 Hz, 2H), 3.92 - 3.79 (m, 5H), 3.57 (s, 3H), 3.47 - 3.31 (m, 7H), 3.02 (s, 1H), 2.90 (d, *J =* 4.6 Hz, 2H), 2.84 - 2.71 (m, 6H), 2.28 (s, 2H), 2.19 (s, 4H), 2.00 (s, 4H), 1.47 (t, *J* = 6.2 Hz, 2H), 1.00 (s, 6H). LCMS (ESI) calcd for C₅₉H₆₇ClF₄N₉O₇S₃ ⁺ [M+H]⁺: 1220.39; found, 1220.1.

### Example 287: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(8-((5-(2,4-dioxotetrahydropyrimidin-1(2H)-yl)pyrazin-2-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-07871)

According to the method of step 2 of Scheme I-1, the target compound (GT-07871) was prepared (white solid, 24 mg, yield 45.7 %). ¹H NMR (500 MHz, DMSO) δ 12.18 (s, 1H), 10.74 (s, 1H), 10.51 (s, 2H), 9.01 (s, 1H), 8.56 (s, 1H), 8.18 (d, *J* = 2.1 Hz, 1H), 8.04 - 7.94 (m, 1H), 7.77 (d, *J =* 8.9 Hz, 2H), 7.41 (d, *J =* 8.4 Hz, 2H), 7.29 (d, *J =* 7.4 Hz, 2H), 7.23 (t, *J =* 7.7 Hz, 3H), 7.19 - 7.14 (m, 3H), 6.97 (t, *J =* 9.0 Hz, 3H), 4.19 (d, *J* = 4.8 Hz, 1H), 4.05 (t, *J =* 6.6 Hz, 2H), 3.99 (d, *J =* 16.9 Hz, 2H), 3.88 (d, *J =* 12.9 Hz, 4H), 3.56 (s, 3H), 3.44 - 3.30 (m, 8H), 3.03 (s, 2H), 2.90 (d, *J =* 8.1 Hz, 3H), 2.78 - 2.70 (m, 4H), 2.28 (s, 2H), 2.19 (s, 4H), 2.04 (s, 3H), 1.47 (t, *J =* 6.3 Hz, 2H), 1.00 (s, 6H). LCMS (ESI) calcd for C₅₈H₆₇ClF₃N₁₀O₇S₃ ⁺ [M+H]⁺: 1203.40; found, 1203.1.

### Example 288: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(3-(4-(2,6-dioxopiperidin-3-yl)benzyl)-3,8-diazabicyclo[3.2.1]octan-8-yl)-1-(phenylthio)butan-2-yl)amino)-3-((trifluoromethyl)sulfonyl)phenyl)sulfonyl)benzamide (GT-07872)

According to the method of step 2 of Scheme I-1, the target compound (GT-07872) was prepared (white solid, 20 mg, yield 38.2 %). ¹H NMR (500 MHz, DMSO) δ 12.18 (s, 1H), 10.85 (s, 1H), 10.41 (s, 1H), 10.22 (s, 1H), 8.18 (d, *J* = 2.0 Hz, 1H), 7.99 (d, *J =* 9.1 Hz, 1H), 7.77 (d, *J =* 8.9 Hz, 2H), 7.41 (d, *J =* 8.4 Hz, 2H), 7.29 (d, *J =* 7.5 Hz, 3H), 7.23 (t, *J =* 7.6 Hz, 5H), 7.19 - 7.15 (m, 3H), 6.97 (t, *J =* 9.8 Hz, 3H), 4.19 (s, 1H), 3.99 - 3.81 (m, 5H), 3.57 (s, 4H), 3.38 (t, *J =* 12.8 Hz, 8H), 3.04 (s, 1H), 2.91 (s, 1H), 2.84 - 2.71 (m, 4H), 2.71 - 2.62 (m, 2H), 2.28 (s, 2H), 2.18 (s, 6H), 2.10 - 1.95 (m, 4H), 1.47 (t, *J =* 6.2 Hz, 2H), 1.00 (s, 6H). LCMS (ESI) calcd for C₆₁H₇₀ClF₃N₇O₇S₃⁺ [M+H]⁺: 1200.41; found, 1200.1

### Example 289: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(3-(4-(2,4-dioxotetrahydropyrimidin-1(2H)-yl)benzyl)-3,8-diazabicyclo[3.2.1]octan-8-yl)-1-(phenylthio)butan-2-yl)amino)-3-((trifluoromethyl)sulfonyl)phenyl)sulfonyl)benzamide (GT-07873)

According to the method of step 2 of Scheme I-1, the target compound (GT-07873) was prepared (white solid, 21 mg, yield 40.0 %). ¹H NMR (500 MHz, DMSO) δ 12.18 (s, 1H), 10.50 (d, *J* = 22.3 Hz, 1H), 10.39 (s, 1H), 8.19 (t, *J* = 9.4 Hz, 1H), 7.99 (d, *J* = 9.1 Hz, 1H), 7.77 (d, *J* = 8.9 Hz, 2H), 7.41 (d, *J* = 8.4 Hz, 2H), 7.37 - 7.32 (m, 2H), 7.29 (d, *J* = 7.5 Hz, 2H), 7.26 - 7.21 (m, 3H), 7.19 - 7.14 (m, 3H), 6.97 (t, *J* = 10.5 Hz, 3H), 4.21 (s, 1H), 3.99 (s, 1H), 3.88 (d, *J* = 12.8 Hz, 3H), 3.79 (t, *J* = 6.6 Hz, 2H), 3.56 (s, 4H), 3.46 - 3.29 (m, 8H), 3.05 (s, 1H), 2.91 (s, 1H), 2.76 (d, *J* = 10.6 Hz, 3H), 2.73 - 2.65 (m, 3H), 2.28 (s, 2H), 2.19 (s, 4H), 2.05 (s, 3H), 1.47 (t, *J* = 6.2 Hz, 2H), 1.28 - 1.18 (m, 1H), 1.00 (s, 6H). LCMS (ESI) calcd for C₆₀H₆₉ClF₃N₈O₇S₃ ⁺ [M+H]⁺: 1201.41; found, 1201.1.

### Example 290: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(3-(3-(2,4-dioxotetrahydropyrimidin-1(2H)-yl)benzyl)-3,8-diazabicyclo[3.2.1]octan-8-yl)-1-(phenylthio)butan-2-yl)amino)-3-((trifluoromethyl)sulfonyl)phenyl)sulfonyl)benzamide (GT-07874)

According to the method of step 2 of Scheme I-1, the target compound (GT-07874) was prepared (white solid, 21 mg, yield 40.0 %). ¹H NMR (500 MHz, DMSO) δ 12.18 (s, 1H), 10.49 (s, 1H), 10.39 (s, 1H), 8.17 (d, *J* = 1.9 Hz, 1H), 7.99 (d, *J* = 9.2 Hz, 1H), 7.77 (d, *J* = 8.8 Hz, 2H), 7.41 (d, *J* = 8.4 Hz, 2H), 7.38 (s, 1H), 7.29 (d, *J* = 7.5 Hz, 3H), 7.23 (t, *J* = 7.6 Hz, 3H), 7.19 - 7.14 (m, 3H), 6.97 (t, *J* = 9.5 Hz, 3H), 4.20 (s, 1H), 3.97 (s, 1H), 3.88 (d, *J* = 12.8 Hz, 2H), 3.83 - 3.79 (m, 2H), 3.57 (s, 4H), 3.46 - 3.26 (m, 8H), 3.04 (s, 1H), 2.91 (s, 1H), 2.76 (d, *J =* 9.6 Hz, 3H), 2.69 (t, *J =* 24.7, 18.1 Hz, 4H), 2.28 (s, 2H), 2.19 (s, 4H), 2.03 (s, 4H), 1.47 (t, *J* = 6.2 Hz, 2H), 1.00 (s, 6H). LCMS (ESI) calcd for C₆₀H₆₉ClF₃N₈O₇S₃ ⁺ [M+H]⁺: 1201.41; found, 1201.1

### Example 291: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(3-((5-(2,4-dioxotetrahydropyrimidin-1(2H)-yl)pyridin-2-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide (GT-07875)

According to the method of step 2 of Scheme I-1, the target compound (GT-07875) was prepared (white solid, 24 mg, yield 45.7 %). ¹H NMR (500 MHz, DMSO) δ 12.19 (s, 1H), 11.00 (s, 1H), 10.68 - 10.52 (m, 2H), 8.65 (s, 1H), 8.18 (d, *J* = 2.0 Hz, 1H), 7.99 (d, 2H), 7.78 (d, *J* = 8.9 Hz, 3H), 7.41 (d, *J* = 8.5 Hz, 2H), 7.29 (d, *J* = 7.5 Hz, 2H), 7.27 - 7.20 (m, 3H), 7.19 - 7.13 (m, 3H), 6.97 (t, *J* = 10.5 Hz, 3H), 4.22 (s, 1H), 4.03 (d, *J* = 17.3 Hz, 2H), 3.95 (s, 2H), 3.90 - 3.85 (m, 5H), 3.56 (s, 3H), 3.45 - 3.33 (m, 7H), 3.25 (s, 2H), 3.01 (d, *J* = 38.5 Hz, 4H), 2.75 (t, *J* = 6.6 Hz, 4H), 2.28 (s, 2H), 2.22 - 2.16 (m, 4H), 2.07 (s, 2H), 1.47 (t, *J* = 6.2 Hz, 2H), 1.00 (s, 6H). LCMS (ESI) calcd for C₅₉H₆₈ClF₃N₉O₇S₃ ⁺ [M+H]⁺: 1202.40; found, 1202.1.

### Example 292: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(3-((6-(2,4-dioxotetrahydropyrimidin-1(2H)-yl)pyridin-3-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide (GT-07876)

According to the method of step 2 of Scheme I-1, the target compound (GT-07876) was prepared (white solid, 22 mg, yield 41.9 %). ¹H NMR (500 MHz, DMSO) δ 12.18 (s, 1H), 10.57 (s, 2H), 8.43 (s, 1H), 8.18 (d, *J* = 2.0 Hz, 1H), 7.99 (d, 1H), 7.78 (d, *J* = 8.9 Hz, 3H), 7.41 (d, *J* = 8.5 Hz, 2H), 7.29 (d, *J* = 7.5 Hz, 2H), 7.27 - 7.21 (m, 3H), 7.19 - 7.14 (m, 3H), 6.97 (t, *J* = 10.1 Hz, 3H), 4.21 (s, 1H), 4.09 - 3.96 (m, 4H), 3.88 (d, *J* = 12.8 Hz, 3H), 3.61 (s, 4H), 3.48 - 3.30 (m, 8H), 3.05 (s, 1H), 2.91 (s, 2H), 2.79 - 2.73 (m, 2H), 2.66 (t, 3H), 2.28 (s, 2H), 2.20 (s, 4H), 2.07 (s, 3H), 1.47 (t, *J* = 6.2 Hz, 2H), 1.00 (s, 6H). LCMS (ESI) calcd for C₅₉H₆₈ClF₃N₉O₇S₃ ⁺ [M+H]⁺: 1202.40; found, 1202.1.

### Example 293: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(3-(3-((2,6-dioxopiperidin-3-yl)amino)benzyl)-3,8-diazabicyclo[3.2.1]octan-8-yl)-1-(phenylthio)butan-2-yl)amino)-3-((trifluoromethyl)sulfonyl)phenyl)sulfonyl)benzamide (GT-07877)

According to the method of step 2 of Scheme I-1, the target compound (GT-07877) was prepared (white solid, 9 mg, yield 17.0 %). ¹H NMR (500 MHz, DMSO) δ 12.17 (s, 1H), 10.80 (s, 1H), 10.24 (s, 1H), 8.17 (d, *J* = 2.1 Hz, 1H), 7.99 (d, *J* = 9.0 Hz, 1H), 7.77 (d, *J* = 8.9 Hz, 2H), 7.41 (d, *J* = 8.4 Hz, 2H), 7.29 (d, *J* = 7.4 Hz, 2H), 7.22 (t, *J* = 7.6 Hz, 3H), 7.19 - 7.14 (m, 3H), 7.10 - 7.04 (m, 1H), 6.97 (t, *J* = 8.9 Hz, 3H), 6.68 - 6.47 (m, 2H), 4.35 - 4.29 (m, 1H), 4.19 (s, 1H), 4.02 - 3.73 (m, 5H), 3.58 (s, 2H), 3.43 - 3.29 (m, 8H), 3.05 (s, 1H), 2.91 (s, 1H), 2.83 - 2.67 (m, 5H), 2.65 - 2.55 (m, 2H), 2.28 (s, 2H), 2.09 (d, *J* = 66.5 Hz, 9H), 1.91 - 1.84 (m, 1H), 1.47 (t, *J* = 6.2 Hz, 2H), 1.00 (s, 6H). LCMS (ESI) calcd for C₆₁H₇₁ClF₃N₈O₇S₃ ⁺ [M+H]⁺: 1215.42 ; found, 1215.1.

### Example 294: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(3-((2-(2,4-dioxotetrahydropyrimidin-1(2H)-yl)-5-fluoropyridin-4-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide (GT-07905)

According to the method of step 2 of Scheme I-1, the target compound (GT-07905) was prepared (white solid, 32 mg, yield 60.1 %). ¹H NMR (500 MHz, DMSO) δ 12.18 (s, 1H), 10.56 (s, 1H), 10.48 (s, 1H), 8.36 (d, *J = 7.3* Hz, 1H), 8.18 (d, *J* = 2.2 Hz, 1H), 8.00 (dd, *J* = 9.2, 2.1 Hz, 1H), 7.93 (d, *J* = 5.3 Hz, 1H), 7.77 (d, *J* = 8.9 Hz, 2H), 7.41 (d, *J* = 8.4 Hz, 2H), 7.32 - 7.28 (m, 2H), 7.24 (dt, *J* = 10.7, 6.6 Hz, 3H), 7.16 (dd, *J* = 17.0, 7.8 Hz, 3H), 6.98 (dd, *J* = 18.4, 8.4 Hz, 3H), 4.19 (s, 1H), 4.04 (t, *J* = 6.6 Hz, 2H), 3.95 (d, *J =* 18.4 Hz, 2H), 3.88 (d, *J* = 13.0 Hz, 2H), 3.72 (s, 2H), 3.57 (s, 2H), 3.42 - 3.27 (m, 8H), 3.08 - 2.99 (m, 1H), 2.90 (d, *J* = 11.6 Hz, 2H), 2.76 (d, *J* = 12.0 Hz, 3H), 2.69 (t, *J* = 6.6 Hz, 2H), 2.28 (s, 2H), 2.24 - 2.13 (m, 4H), 2.09 - 2.00 (m, 3H), 1.47 (t, *J* = 6.3 Hz, 2H), 1.00 (s, 6H). LCMS (ESI) calcd for C₅₉H₆₇ClF₄N₉O₇S₃⁺ [M+H]⁺: 1220.39; found, 1220.1.

### Example 295: Preparation of 4-(4-((4'-chloro-4,4-dimethyl-3.4,5,6-tetrahydro-[1,1'-biphenyl]-2-yl)methyl)piperazin-1-yl)-N-((4-(((2R)-4-(4-(4-(2,6-dioxopiperidin-3-yl)benzyl)piperazin-1-yl-2,2,3,3,5,5,6,6-d8)-1-(phenylthio)butan-2-yl)amino)-3-((trifluoromethyl)sulfonyl)phenyl)sulfonyl)benzamide (GT-07906)

According to the method of step 2 of Scheme I-1, the target compound (GT-07906) was prepared (white solid, 15 mg, yield 28.3 %). ¹H NMR (500 MHz, DMSO) δ 12.16 (s, 1H), 10.87 (s, 1H), 10.32 (s, 1H), 8.17 (d, *J* = 1.8 Hz, 1H), 7.99 (d, *J* = 8.6 Hz, 1H), 7.77 (d, *J* = 8.9 Hz, 2H), 7.51 (s, 2H), 7.41 (d, *J* = 8.4 Hz, 2H), 7.29 (d, *J* = 7.4 Hz, 4H), 7.22 (t, *J* = 7.6 Hz, 2H), 7.19 - 7.14 (m, 3H), 7.02 - 6.91 (m, 3H), 4.25 (d, *J* = 56.4 Hz, 3H), 3.88 (d, *J* = 12.1 Hz, 4H), 3.66 - 3.44 (m, 4H), 3.36 - 3.31 (m, 4H), 3.31 - 2.95 (m, 2H), 2.77 (d, *J* = 8.9 Hz, 2H), 2.71 - 2.54 (m, 2H), 2.28 (s, 2H), 2.23 - 2.09 (m, 4H), 2.07 - 2.01 (m, 1H), 1.47 (t, *J* = 6.1 Hz, 2H), 1.00 (s, 6H). LCMS (ESI) calcd for C₅₉H₆₀D₈ClF₃N₇O₇S₃ ⁺ [M+H]⁺: 1182.45; found, 1182.2.

### Example 296: Preparation of (R)-4-(4-((4'-chloro-4,4-dimethyl-3,4,5,6-tetrahydro-[1,1'-biphenyl]-2-yl)methyl)piperazin-1-yl)-N-((4-((4-(4-(4-(2,4-dioxotetrahydropyrimidin-1(2H)-yl)benzyl)piperazin-1-yl-2,2,3,3,5,5,6,6-d8)-1-(phenylthio)butan-2-yl)amino)-3-((trifluoromethyl)sulfonyl)phenyl)sulfonyl)benzamide (GT-07907)

According to the method of step 2 of Scheme I-1, the target compound (GT-07907) was prepared (white solid, 17 mg, yield 32.1 %). ¹H NMR (500 MHz, DMSO) δ 12.17 (s, 1H), 10.45 (s, 2H), 8.18 (d, *J =* 1.8 Hz, 1H), 7.99 (d, *J* = 8.3 Hz, 1H), 7.77 (d, *J* = 8.9 Hz, 2H), 7.65 - 7.56 (m, 1H), 7.46 - 7.35 (m, 4H), 7.29 (d, *J* = 7.4 Hz, 2H), 7.23 (t, *J* = 7.6 Hz, 2H), 7.19 - 7.14 (m, 3H), 7.00 (d, *J* = 8.9 Hz, 1H), 6.96 (d, *J =* 9.0 Hz, 2H), 4.27 (s, 1H), 4.20 (s, 1H), 3.88 (d, *J* = 12.8 Hz, 2H), 3.81 (t, *J* = 6.6 Hz, 2H), 3.68 - 3.45 (m, 3H), 3.38 - 3.33 (m, 6H), 3.29 - 3.04 (m, 2H), 2.84 - 2.73 (m, 2H), 2.73 - 2.64 (m, 2H), 2.28 (s, 2H), 2.13 (s, 4H), 1.47 (t, *J* = 6.2 Hz, 2H), 1.00 (s, 6H). LCMS (ESI) calcd for C₅₈H₅₉D₈ClF₃N₈O₇S₃ ⁺ [M+H]⁺: 1183.44; found, 1183.2.

### Example 297: Preparation of (R)-4-(4-((4'-chloro-4,4-dimethyl-3,4,5,6-tetrahydro-[1,1'-biphenyl]-2-yl)methyl)piperazin-1-yl)-N-((4-((4-(4-(3-(2,4-dioxotetrahydropyrimidin-1(2H)-yl)benzyl)piperazin-1-yl-2,2,3,3,5,5,6,6-d8)-1-(phenylthio)butan-2-yl)amino)-3-((trifluoromethyl)sulfonyl)phenyl)sulfonyl)benzamide (GT-07908)

According to the method of step 2 of Scheme I-1, the target compound (GT-07908) was prepared (white solid, 21 mg, yield 39.6 %). ¹H NMR (500 MHz, DMSO) δ 12.16 (s, 1H), 10.43 (s, 1H), 10.37 (s, 1H), 8.17 (d, *J* = 1.9 Hz, 1H), 7.99 (d, *J* = 8.8 Hz, 1H), 7.77 (d, *J* = 8.9 Hz, 2H), 7.55 (s, 1H), 7.41 (d, *J* = 8.4 Hz, 5H), 7.29 (d, *J =* 7.5 Hz, 2H), 7.22 (t, *J =* 7.6 Hz, 3H), 7.19 - 7.14 (m, 3H), 7.01 - 6.93 (m, 3H), 4.34 (s, 1H), 4.20 (s, 1H), 4.00 - 3.75 (m, 5H), 3.57 (s, 3H), 3.39 - 3.33 (m, 6H), 3.28 - 2.91 (m, 2H), 2.77 (d, *J* = 10.1 Hz, 2H), 2.71 (t, *J* = 6.6 Hz, 2H), 2.28 (s, 2H), 2.15 (s, 3H), 1.47 (t, *J* = 6.2 Hz, 2H), 1.00 (s, 6H). LCMS (ESI) calcd for C₅₈H₅₉D₈ClF₃N₈O₇S₃ ⁺ [M+H]⁺: 1183.44 ; found, 1183.2.

### Example 298: Preparation of (R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)pyridin-2-yl)methyl)piperazin-1-yl-2,2,3,3,5,5,6,6-d8)-1-(phenylthio)butan-2-yl)amino)-3-((trifluoromethyl)sulfonyl)phenyl)sulfonyl)benzamide (GT-07909)

According to the method of step 2 of Scheme I-1, the target compound (GT-07909) was prepared (white solid, 25 mg, yield 47.1 %). ¹H NMR (500 MHz, DMSO) δ 12.17 (s, 1H), 10.66 - 10.40 (m, 2H), 8.66 (s, 1H), 8.18 (d, *J =* 2.1 Hz, 1H), 7.99 (dd, *J =* 9.2, 2.1 Hz, 1H), 7.89 (d, 1H), 7.78 (d, *J =* 8.9 Hz, 2H), 7.66 (t, *J* = 17.4 Hz, 1H), 7.41 (d, *J* = 8.4 Hz, 2H), 7.31 - 7.27 (m, 2H), 7.26 - 7.20 (m, 3H), 7.19 - 7.14 (m, 3H), 7.00 (d, *J =* 8.9 Hz, 1H), 6.96 (d, *J =* 9.1 Hz, 2H), 4.29 (d, *J =* 58.6 Hz, 3H), 3.87 (t, *J =* 6.6 Hz, 4H), 3.56 (s, 3H), 3.40 - 3.35 (m, 5H), 3.21 - 2.99 (m, 2H), 2.75 (t, *J* = 6.6 Hz, 4H), 2.28 (s, 2H), 2.19 (s, 4H), 1.47 (t, *J* = 6.3 Hz, 2H), 1.00 (s, 6H). LCMS (ESI) calcd for C₅₇H₅₈D₈ClF₃N₉O₇S₃ ⁺ [M+H]⁺: 1184.44; found, 1184.2.

### Example 299: Preparation of (R)-4-(4-((4'-chloro-4,4-dimethyl-3,4,5,6-tetrahydro-[1,1'-biphenyl]-2-yl)methyl)piperazin-1-yl)-N-((4-((4-(4-((6-(2,4-dioxotetrahydropyrimidin-1(2H)-yl)pyridin-3-yl)methyl)piperazin-1-yl-2,2,3,3,5,5,6,6-d8)-1-(phenylthio)butan-2-yl)amino)-3-((trifluoromethyl)sulfonyl)phenyl)sulfonyl)benzamide (GT-07910)

According to the method of step 2 of Scheme I-1, the target compound (GT-07910) was prepared (white solid, 25 mg, yield 47.1 %). ¹H NMR (400 MHz, DMSO-d6) δ 12.17 (s, 1H), 10.60 (s, 1H), 10.39 (s, 1H), 8.55 (s, 1H), 8.18 (s, 1H), 7.98 (d, J = 8.6 Hz, 2H), 7.83 (d, J = 8.3 Hz, 1H), 7.77 (d, J = 8.7 Hz, 2H), 7.41 (d, J = 8.2 Hz, 2H), 7.29 (d, J = 7.5 Hz, 2H), 7.22 (t, J = 7.5 Hz, 3H), 7.17 (t, J = 7.8 Hz, 3H), 7.02 - 6.94 (m, 3H), 4.21 (s, 2H), 4.08 (t, J = 6.5 Hz, 2H), 3.89 (d, J = 12.4 Hz, 2H), 3.57 (s, 2H), 3.39 - 3.29 (m, 8H), 3.20 - 3.02 (m, 2H), 2.82 - 2.74 (m, 2H), 2.69 (t, J = 6.5 Hz, 2H), 2.28 (s, 2H), 2.18 (s, 3H), 1.47 (s, 2H), 1.00 (s, 6H). LCMS (ESI) calcd for C₅₇H₅₈D₈ClF₃N₉O₇S₃⁺ [M+H]⁺: 1184.44; found, 1184.2.

### Example 300: Preparation of 7-cyclopentyl-2-((5-(4-(4-(2,6-dioxopiperidin-3-yl)benzyl)piperazin-1-yl)pyridin-2-yl)amino)-N,N-dimethyl-7H-pyrrolo[2,3-d]pyrimidine-6-carboxamide (GT-06339)

According to the method of step 2 of Scheme I-1, the target compound (GT-06339) was prepared (white solid, 29 mg, yield 49.56%). ¹H NMR (400 MHz, DMSO-d6) δ 11.49 (s, 2H), 10.88 (s, 1H), 9.01 (s, 1H), 8.05 - 7.87 (m, 2H), 7.64 (t, J = 7.7 Hz, 3H), 7.35 (d, J = 8.1 Hz, 2H), 6.84 (s, 1H), 4.86 - 4.72 (m, 1H), 4.39 (s, 2H), 3.96 (d, J = 4.8 Hz, 1H), 3.81 (s, 2H), 3.26 - 3.16 (m, 6H), 3.06 (s, 6H), 2.75 - 2.68 (m, 1H), 2.55 (s, 1H), 2.34 - 2.21 (m, 3H), 2.07 - 1.93 (m, 5H), 1.65 (d, J = 5.7 Hz, 2H). LCMS (ESI) calcd for C₃₅H₄₂N₉O₃⁺ [M+H]⁺: 636.34; found, 636.4.

### Example 301: Preparation of 7-cyclopentyl-2-((5-(4-(4-(2,4-dioxotetrahydropyrimidin-1(2H)-yl)benzyl)piperazin-1-yl)pyridin-2-yl)amino)-N,N-dimethyl-7H-pyrrolo[2,3-d]pyrimidine-6-carboxamide (GT-06340)

According to the method of step 2 of Scheme I-1, the target compound (GT-06340) was prepared (white solid, 18 mg, yield 30.71%). ¹H NMR (400 MHz, DMSO-d6) δ 11.45 (d, J = 41.0 Hz, 2H), 10.46 (d, J = 15.7 Hz, 1H), 8.99 (s, 1H), 8.07 - 7.92 (m, 2H), 7.72 - 7.61 (m, 3H), 7.46 (t, J = 15.7 Hz, 2H), 6.82 (d, J = 3.9 Hz, 1H), 4.88 - 4.73 (m, 1H), 4.39 (s, 2H), 3.83 (t, J = 6.6 Hz, 4H), 3.28 - 3.18 (m, 6H), 3.05 (s, 6H), 2.72 (t, J = 6.6 Hz, 2H), 2.30 (s, 2H), 2.06 - 1.87 (m, 4H), 1.64 (d, J = 5.8 Hz, 2H). LCMS (ESI) calcd for C₃₄H₄₁N₁₀O₃⁺ [M+H]⁺: 637.34; found, 637.3.

### Example 302: Preparation of 7-cyclopentyl-2-((5-(4-(3-(2,4-dioxotetrahydropyrimidin-1(2H)-yl)benzyl)piperazin-1-yl)pyridin-2-yl)amino)-N,N-dimethyl-7H-pyrrolo[2,3-d]pyrimidine-6-carboxamide (GT-06341)

According to the method of step 2 of Scheme I-1, the target compound (GT-06341) was prepared (white solid, 30 mg, yield 51.19%). ¹H NMR (400 MHz, DMSO-d6) δ 11.67 (s, 2H), 10.45 (s, 1H), 9.03 (s, 1H), 8.08 (dd, J = 9.5, 2.6 Hz, 1H), 7.98 (d, J = 2.5 Hz, 1H), 7.67 (s, 1H), 7.60 (d, J = 9.5 Hz, 1H), 7.56 - 7.44 (m, 3H), 6.85 (s, 1H), 4.79 (d, J = 8.7 Hz, 1H), 4.43 (s, 2H), 3.87 (d, J = 6.7 Hz, 6H), 3.33 (s, 4H), 3.06 (s, 6H), 2.74 (t, J = 6.6 Hz, 2H), 2.29 (s, 2H), 2.09 - 1.93 (m, 4H), 1.64 (d, J = 5.8 Hz, 2H). LCMS (ESI) calcd for C₃₄H₄₂₁N₁₀O₃⁺ [M+H]⁺: 637.34; found, 637.3.

### Example 303: Preparation of 7-cyclopentyl-2-((5-(4-((5-(2,4-dioxotetrahydropyrimidin-1(2H)-yl)pyridin-2-yl)methyl)piperazin-1-yl)pyridin-2-yl)amino)-N,N-dimethyl-7H-pyrrolo[2,3-d]pyrimidine-6-carboxamide (GT-06342)

According to the method of step 2 of Scheme I-1, the target compound (GT-06342) was prepared (white solid, 28 mg, yield 47.70%). ¹H NMR (400 MHz, DMSO-d6) δ 11.58 (s, 1H), 10.60 (s, 1H), 9.02 (s, 1H), 8.72 (d, J = 2.4 Hz, 1H), 8.07 (dd, J = 9.4, 2.6 Hz, 1H), 7.98 (d, J = 2.5 Hz, 1H), 7.93 (dd, J = 8.4, 2.5 Hz, 1H), 7.77 (d, J = 8.4 Hz, 1H), 7.61 (d, J = 9.4 Hz, 1H), 6.85 (s, 1H), 4.87 - 4.76 (m, 1H), 4.57 (s, 2H), 3.90 (d, J = 6.6 Hz, 2H), 3.57 (s, 8H), 3.06 (s, 6H), 2.77 (t, J = 6.6 Hz, 2H), 2.32 (d, J = 11.8 Hz, 2H), 2.09 - 1.92 (m, 4H), 1.74 - 1.57 (m, 2H). LCMS (ESI) calcd for C₃₃H₄₀N₁₁O₃⁺ [M+H]⁺: 638.33; found, 638.3.

### Example 304: Preparation of 7-cyclopentyl-2-((5-(4-((6-(2,4-dioxotetrahydropyrimidin-1(2H)-yl)pyridin-3-yl)methyl)piperazin-1-yl)pyridin-2-yl)amino)-N,N-dimethyl-7H-pyrrolo[2,3-d]pyrimidine-6-carboxamide (GT-06343)

According to the method of step 2 of Scheme I-1, the target compound (GT-06343) was prepared (white solid, 40 mg, yield 68.14%). ¹H NMR (400 MHz, DMSO-d6) δ 11.81 (s, 1H), 11.55 (s, 1H), 10.62 (s, 1H), 9.01 (s, 1H), 8.64 (d, J = 1.8 Hz, 1H), 8.14 (dd, J = 8.7, 2.2 Hz, 1H), 8.05 (dd, J = 9.5, 2.5 Hz, 1H), 7.97 (d, J = 2.5 Hz, 1H), 7.86 (d, J = 8.6 Hz, 1H), 7.61 (d, J = 9.4 Hz, 1H), 6.84 (s, 1H), 4.78 (dd, J = 17.5, 8.7 Hz, 1H), 4.44 (s, 2H), 4.09 (t, J = 6.5 Hz, 2H), 3.81 (s, 4H), 3.29 - 3.19 (m, 4H), 3.05 (s, 6H), 2.70 (t, J = 6.5 Hz, 2H), 2.31 (d, J = 11.7 Hz, 2H), 2.08 - 1.93 (m, 4H), 1.68 - 1.58 (m, 2H). LCMS (ESI) calcd for C₃₃H₄₀N₁₁O₃⁺ [M+H]⁺: 638.33; found, 638.4.

### Example 305: Preparation of 7-cyclopentyl-2-((5-(4-(3-((2,6-dioxopiperidin-3-yl)amino)benzyl)piperazin-1-yl)pyridin-2-yl)amino)-N,N-dimethyl-7H-pyrrolo[2,3-d]pyrimidine-6-carboxamide (GT-06344)

According to the method of step 2 of Scheme I-1, the target compound (GT-06344) was prepared (white solid, 25 mg, yield 41.74 %). ¹H NMR (400 MHz, DMSO-d6) δ 11.54 (s, 2H), 10.84 (s, 1H), 9.03 (s, 1H), 8.20 - 7.79 (m, 3H), 7.72 - 7.44 (m, 3H), 7.38 - 7.11 (m, 2H), 6.92 - 6.71 (m, 2H), 4.83 - 4.74 (m, 1H), 4.42 (s, 2H), 4.27 (s, 2H), 3.35 (dd, J = 50.3, 31.1 Hz, 7H), 3.05 (s, 6H), 2.89 - 2.78 (m, 1H), 2.61 (s, 1H), 2.23 (d, J = 39.7 Hz, 3H), 2.07 - 1.89 (m, 5H), 1.64 (s, 2H). LCMS (ESI) calcd for C₃₅H₄₃N₁₀O₃⁺ [M+H]⁺: 651.35; found, 651.4.

### Example 306: Preparation of 7-cyclopentyl-2-((5-(4-((4-(2,6-dioxopiperidin-3-yl)benzyl)(methyl)amino)piperidin-1-yl)pyridin-2-yl)amino)-N,N-dimethyl-7H-pyrrolo[2,3-d]pyrimidine-6-carboxamide (GT-06490)

According to the method of step 2 of Scheme I-1, the target compound (GT-06490) was prepared (white solid, 10 mg, yield 13.94%). ¹H NMR (400 MHz, DMSO-d6) δ 11.58 (s, 1H), 10.97 (s, 1H), 10.87 (s, 1H), 9.01 (s, 1H), 8.26 - 8.05 (m, 1H), 7.95 (d, J = 2.5 Hz, 1H), 7.67 - 7.57 (m, 3H), 7.32 (d, J = 8.2 Hz, 2H), 6.83 (s, 1H), 4.88 - 4.72 (m, 1H), 4.49 (d, J = 10.7 Hz, 1H), 4.18 (dd, J = 12.8, 7.1 Hz, 1H), 3.96 - 3.82 (m, 3H), 3.61 (d, J = 6.8 Hz, 4H), 3.06 (s, 6H), 2.80 (d, J = 11.0 Hz, 2H), 2.59 (d, J = 4.7 Hz, 3H), 2.40 - 2.19 (m, 5H), 2.06 - 1.93 (m, 6H), 1.72 - 1.59 (m, 2H). LCMS (ESI) calcd for C₃₇H₄₆N₉O₃' [M+H]⁺: 664.37; found, 664.4.

### Example 307: Preparation of 7-cyclopentyl-2-((5-(4-((4-(2,4-dioxotetrahydropyrimidin-1(2H)-yl)benzyl)(methyl)amino)piperidin-1-yl)pyridin-2-yl)amino)-N,N-dimethyl-7H-pyrrolo[2,3-d]pyrimidine-6-carboxamide (GT-06491)

According to the method of step 2 of Scheme I-1, the target compound (GT-06491) was prepared (white solid, 8 mg, yield 11.13 %). ¹H NMR (400 MHz, DMSO-d6) δ 11.69 - 11.14 (m, 2H), 10.46 (d, J = 14.4 Hz, 1H), 9.02 (s, 1H), 8.16 (dd, J = 9.6, 2.5 Hz, 1H), 7.95 (d, J = 2.4 Hz, 1H), 7.73 - 7.58 (m, 3H), 7.43 (d, J = 8.4 Hz, 2H), 7.24 (d, J = 36.0 Hz, 2H), 6.84 (s, 1H), 4.82 (p, J = 8.6 Hz, 1H), 4.50 (d, J = 11.4 Hz, 1H), 4.21 (dd, J = 12.8, 7.2 Hz, 1H), 3.83 (t, J = 6.7 Hz, 4H), 3.06 (s, 6H), 2.86 - 2.78 (m, 2H), 2.73 (t, J = 6.6 Hz, 2H), 2.61 (d, J = 4.6 Hz, 3H), 2.34 (dd, J = 20.4, 14.5 Hz, 5H), 2.09 - 1.92 (m, 6H), 1.75 - 1.60 (m, 2H). LCMS (ESI) calcd for C₃₆H₄₅N₁₀O₃⁺ [M+H]⁺: 665.37; found, 665.4.

### Example 308: Preparation of 7-cyclopentyl-2-((5-(4-((3-(2,4-dioxotetrahydropyrimidin-1(2H)-yl)benzyl)(methyl)amino)piperidin-1-yl)pyridin-2-yl)amino)-N,N-dimethyl-7H-pyrrolo[2,3-d]pyrimidine-6-carboxamide (GT-06492)

According to the method of step 2 of Scheme I-1, the target compound (GT-06492) was prepared (white solid, 30 mg, yield 41.75 %). ¹H NMR (400 MHz, DMSO-d6) δ 11.63 (s, 1H), 11.27 (s, 1H), 10.44 (s, 1H), 9.01 (s, 1H), 8.16 (dd, J = 9.6, 2.7 Hz, 1H), 7.96 (d, J = 2.6 Hz, 1H), 7.68 (s, 1H), 7.62 (d, J = 9.5 Hz, 1H), 7.54 (d, J = 4.3 Hz, 1H), 7.46 (dd, J = 7.6, 5.7 Hz, 2H), 6.83 (s, 1H), 4.91 - 4.75 (m, 1H), 4.51 (d, J = 10.3 Hz, 1H), 4.22 (dd, J = 12.9, 7.0 Hz, 1H), 3.86 (d, J = 6.6 Hz, 3H), 3.39 - 3.34 (m, 2H), 3.05 (s, 6H), 2.80 (dd, J = 16.6, 11.5 Hz, 2H), 2.72 (t, J = 6.7 Hz, 2H), 2.60 (d, J = 4.7 Hz, 3H), 2.42 - 2.26 (m, 4H), 2.06 - 1.91 (m, 6H), 1.69 - 1.60 (m, 2H). LCMS (ESI) calcd for C₃₆H₄₅N₁₀O₃⁺ [M+H]⁺: 665.37; found, 665.4.

### Example 309: Preparation of 7-cyclopentyl-2-((5-(4-(((5-(2,4-dioxotetrahydropyrimidin-1(2H)-yl)pyridin-2-yl)methyl)(methyl)amino)piperidin-1-yl)pyridin-2-yl)amino)-N,N-dimethyl-7H-pyrrolo[2,3-d]pyrimidine-6-carboxamide (GT-06493)

According to the method of step 2 of Scheme I-1, the target compound (GT-06493) was prepared (white solid, 10 mg, yield 13.90%). ¹H NMR (400 MHz, DMSO-d6) δ 11.62 (s, 1H), 10.59 (s, 1H), 9.02 (s, 1H), 8.70 (d, J = 2.5 Hz, 1H), 8.17 (dd, J = 9.6, 2.8 Hz, 1H), 7.96 (d, J = 2.6 Hz, 1H), 7.92 (dd, J = 8.4, 2.6 Hz, 1H), 7.77 (d, J = 8.4 Hz, 1H), 7.60 (d, J = 9.5 Hz, 1H), 6.85 (s, 1H), 4.87 - 4.77 (m, 1H), 4.52 (s, 2H), 3.88 (d, J = 6.6 Hz, 2H), 3.44 (d, J = 7.0 Hz, 4H), 3.06 (s, 6H), 2.76 (t, J = 6.6 Hz, 6H), 2.30 (s, 4H), 2.08 - 1.92 (m, 6H), 1.70 - 1.61 (m, 2H). LCMS (ESI) calcd for C₃₅H₄₄N₁₁O₃⁺ [M+H]⁺: 666.36; found, 666.4.

### Example 310: Preparation of 7-cyclopentyl-2-((5-(4-(((6-(2,4-dioxotetrahydropyrimidin-1(2H)-yl)pyridin-3-yl)methyl)(methyl)amino)piperidin-1-yl)pyridin-2-yl)amino)-N,N-dimethyl-7H-pyrrolo[2,3-d]pyrimidine-6-carboxamide (GT-06494)

According to the method of step 2 of Scheme I-1, the target compound (GT-06494) was prepared (white solid, 10 mg, yield 13.90%). ¹H NMR (400 MHz, DMSO-d6) δ 11.59 (d, J = 40.8 Hz, 2H), 10.70 - 10.54 (m, 2H), 9.02 (s, 1H), 8.68 (d, J = 2.1 Hz, 1H), 8.47 (d, J = 2.0 Hz, 1H), 8.25 - 8.16 (m, 2H), 7.98 (d, J = 2.7 Hz, 1H), 7.91 - 7.84 (m, 2H), 7.81 - 7.76 (m, 1H), 7.62 (d, J = 9.5 Hz, 1H), 6.84 (s, 1H), 4.51 (s, 2H), 4.31 - 4.26 (m, 1H), 4.09 - 4.06 (m, 2H), 3.44 (dd, J = 14.0, 7.0 Hz, 2H), 3.06 (s, 6H), 2.84 (d, J = 11.6 Hz, 2H), 2.69 (s, 3H), 2.61 (d, J = 4.7 Hz, 3H), 2.41 (d, J = 11.9 Hz, 1H), 2.31 (d, J = 4.5 Hz, 3H), 2.05 - 1.92 (m, 6H), 1.71 - 1.58 (m, 2H). LCMS (ESI) calcd for C₃₅H₄₄N₁₁O₃⁺ [M+H]⁺: 666.36; found, 666.4.

### Example 311: Preparation of 7-cyclopentyl-2-((5-(4-((3-((2,6-dioxopiperidin-3-yl)amino)benzyl)(methyl)amino)piperidin-1-yl)pyridin-2-yl)amino)-N,N-dimethyl-7H-pyrrolo[2,3-d]pyrimidine-6-carboxamide (GT-06495)

According to the method of step 2 of Scheme I-1, the target compound (GT-06495) was prepared (white solid, 20 mg, yield 27.26 %). ¹H NMR (400 MHz, DMSO-d6) δ 11.63 (s, 1H), 11.02 (d, J = 29.1 Hz, 1H), 9.02 (s, 1H), 8.17 (d, J = 9.6 Hz, 1H), 7.96 (s, 1H), 7.61 (d, J = 9.6 Hz, 2H), 7.51 - 7.43 (m, 2H), 7.35 - 7.15 (m, 2H), 6.88 - 6.76 (m, 2H), 5.01 - 4.79 (m, 2H), 4.52 - 4.29 (m, 3H), 3.89 (s, 2H), 3.41 - 3.32 (m, 4H), 3.06 (s, 6H), 2.84 - 2.75 (m, 2H), 2.64 - 2.59 (m, 3H), 2.39 - 2.24 (m, 4H), 2.00 (dt, J = 33.5, 17.5 Hz, 6H), 1.71 - 1.63 (m, 2H). LCMS (ESI) calcd for C₃₇H₄₇N₁₀O₃⁺ [M+H]⁺: 679.38; found, 679.5.

### Example 312: Preparation of 3-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)phenyl)piperidine-2,6-dione (GT-06575)

According to the method of step 2 of Scheme I-1, the target compound (GT-06575) was prepared (white solid, 30 mg, yield 51.73%). ¹H NMR (400 MHz, DMSO-d6) δ 11.63 (s, 1H), 11.05 - 10.75 (m, 2H), 9.12 - 8.90 (m, 1H), 8.08 (dd, J = 6.6, 2.6 Hz, 1H), 7.93 - 7.76 (m, 2H), 7.64 (d, J = 8.1 Hz, 2H), 7.34 (d, J = 8.2 Hz, 2H), 5.83 (p, J = 8.8 Hz, 1H), 4.79 (s, 1H), 4.38 (s, 2H), 3.98 - 3.84 (m, 4H), 3.44 - 3.36 (m, 2H), 3.31 (t, J = 12.2 Hz, 2H), 3.18 (d, J = 9.0 Hz, 2H), 2.69 (td, J = 12.0, 6.0 Hz, 1H), 2.54 (t, J = 4.0 Hz, 1H), 2.43 (s, 2H), 2.35 (d, J = 11.0 Hz, 3H), 2.29 - 2.15 (m, 3H), 2.05 (dt, J = 13.0, 4.4 Hz, 1H), 1.92 (s, 2H), 1.84 - 1.73 (m, 2H), 1.59 (d, J = 4.9 Hz, 2H). LCMS (ESI) calcd for C₃₆H₄₁N₈O₄⁺ [M+H]⁺: 649.33; found, 649.3.

### Example 313: Preparation of 1-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)phenyl)dihydropyrimidine-2,4(1H,3H)-dione (GT-06576

According to the method of step 2 of Scheme I-1, the target compound (GT-06576) was prepared (white solid, 8 mg, yield 13.78%). ¹H NMR (400 MHz, DMSO-d6) δ 11.70 (s, 1H), 11.13 (s, 1H), 10.44 (s, 1H), 9.02 (d, J = 3.6 Hz, 1H), 8.07 (d, J = 2.7 Hz, 1H), 7.89 (dd, J = 28.2, 9.4 Hz, 2H), 7.71 (dd, J = 14.4, 8.2 Hz, 2H), 7.49 (dd, J = 42.7, 8.5 Hz, 2H), 5.93 - 5.73 (m, 1H), 4.42 (d, J = 25.8 Hz, 2H), 3.90 - 3.83 (m, 4H), 3.42 - 3.19 (m, 6H), 2.72 (t, J = 6.6 Hz, 2H), 2.44 (s, 3H), 2.35 (d, J = 10.5 Hz, 3H), 2.28 - 2.18 (m, 2H), 1.94 (s, 2H), 1.85 - 1.71 (m, 2H), 1.66 - 1.53 (m, 2H). LCMS (ESI) calcd for C₃₅H₄₀N₉O₄⁺ [M+H]⁺: 650.32; found, 650.4.

### Example 314: Preparation of 1-(3-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)phenyl)dihydropyrimidine-2,4(1H,3H)-dione (GT-06577)

According to the method of step 2 of Scheme I-1, the target compound (GT-06577) was prepared (white solid, 36 mg, yield 61.99%). ¹H NMR (400 MHz, DMSO-d6) δ 11.79 (s, 1H), 11.23 (s, 1H), 10.45 (s, 1H), 9.02 (s, 1H), 8.07 (d, J = 2.8 Hz, 1H), 7.97 (d, J = 9.5 Hz, 1H), 7.86 (d, J = 9.4 Hz, 1H), 7.67 (s, 1H), 7.57 - 7.50 (m, 1H), 7.48 (d, J = 5.2 Hz, 2H), 5.84 (p, J = 8.8 Hz, 1H), 4.41 (s, 2H), 3.87 (d, J = 6.7 Hz, 4H), 3.43 - 3.15 (m, 6H), 2.73 (t, J = 6.6 Hz, 2H), 2.43 (s, 3H), 2.34 (s, 3H), 2.21 (dt, J = 16.1, 8.1 Hz, 2H), 1.94 (s, 2H), 1.85 - 1.75 (m, 2H), 1.65 - 1.51 (m, 2H). LCMS (ESI) calcd for C₃₅H₄₀N₉O₄⁺ [M+H]⁺: 650.32; found, 650.3.

### Example 315: Preparation of 1-(6-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-3-yl)dihydropyrimidine-2,4(1H,3H)-dione (GT-06578)

According to the method of step 2 of Scheme I-1, the target compound (GT-06578) was prepared (white solid, 20 mg, yield 34.39%). ¹H NMR (400 MHz, DMSO-d6) δ 11.55 (d, J = 54.0 Hz, 1H), 10.60 (s, 1H), 9.05 (s, 1H), 8.71 (d, J = 2.5 Hz, 1H), 8.08 (d, J = 8.7 Hz, 2H), 7.93 (dd, J = 8.4, 2.6 Hz, 1H), 7.88 (d, J = 9.7 Hz, 1H), 7.82 (d, J = 8.4 Hz, 1H), 5.95 - 5.71 (m, 1H), 4.57 (s, 2H), 3.90 (s, 2H), 3.63 (s, 4H), 3.44 (d, J = 6.9 Hz, 4H), 2.76 (t, J = 6.6 Hz, 2H), 2.45 (s, 3H), 2.36 (s, 3H), 2.30 - 2.18 (m, 2H), 1.96 (s, 2H), 1.88 - 1.76 (m, 2H), 1.65 - 1.56 (m, 2H). LCMS (ESI) calcd for C₃₄H₃₉N₁₀O₄⁺ [M+H]⁺: 651.32; found, 651.3.

### Example 316: Preparation of 1-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-2-yl)dihydropyrimidine-2,4(1H,3H)-dione (GT-06579)

According to the method of step 2 of Scheme I-1, the target compound (GT-06579) was prepared (white solid, 26 mg, yield 44.70%). ¹H NMR (400 MHz, DMSO-d6) δ 12.02 (s, 1H), 11.34 (s, 1H), 10.63 (s, 1H), 9.05 (d, J = 16.3 Hz, 1H), 8.66 (d, J = 2.0 Hz, 1H), 8.16 (dd, J = 8.7, 2.3 Hz, 1H), 8.08 (d, J = 2.7 Hz, 1H), 8.02 (d, J = 9.4 Hz, 1H), 7.91 - 7.84 (m, 2H), 5.85 (p, J = 8.8 Hz, 1H), 4.45 (s, 2H), 4.11 (t, J = 6.6 Hz, 2H), 3.90 (s, 2H), 3.47 - 3.21 (m, 6H), 2.70 (dd, J = 13.8, 7.1 Hz, 2H), 2.45 (s, 3H), 2.37 (d, J = 10.7 Hz, 3H), 2.29 - 2.16 (m, 2H), 1.96 (s, 2H), 1.87 - 1.76 (m, 2H), 1.65 - 1.56 (m, 2H). LCMS (ESI) calcd for C₃₄H₃₉N_{10O4}⁺ [M+H]⁺: 651.32; found, 651.3.

### Example 317: Preparation of 3-((3-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)phenyl)amino)piperidine-2,6-dione (GT-06580)

According to the method of step 2 of Scheme I-1, the target compound (GT-06580) was prepared (white solid, 33 mg, yield 55.62%). ¹H NMR (400 MHz, DMSO-d6) δ 11.56 (s, 1H), 11.29 (s, 1H), 10.85 (s, 1H), 9.02 (s, 1H), 8.06 (d, J = 2.8 Hz, 1H), 7.99 (d, J = 9.4 Hz, 1H), 7.85 (d, J = 9.4 Hz, 1H), 7.16 (t, J = 7.8 Hz, 1H), 7.07 (s, 1H), 6.80 (dd, J = 11.1, 4.6 Hz, 2H), 5.91 - 5.72 (m, 1H), 4.41 (dd, J = 11.7, 4.8 Hz, 1H), 4.26 (s, 2H), 3.88 (s, 2H), 3.45 - 3.34 (m, 4H), 3.17 (d, J = 10.1 Hz, 2H), 2.83 (ddd, J = 17.6, 12.4, 5.3 Hz, 1H), 2.59 (dd, J = 13.9, 3.7 Hz, 1H), 2.43 (d, J = 6.3 Hz, 3H), 2.34 (s, 3H), 2.28 - 2.15 (m, 3H), 1.99 - 1.90 (m, 2H), 1.85 - 1.75 (m, 2H), 1.65 - 1.53 (m, 2H). LCMS (ESI) calcd for C₃₆H₄₂N₉O₄⁺ [M+H]⁺: 664.34; found, 664.3.

### Example 318: Preparation of 3-(4-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)phenyl)piperidine-2,6-dione (GT-06659)

According to the method of step 2 of Scheme I-1, the target compound (GT-06659) was prepared (white solid, 18 mg, yield 32.63%). ¹H NMR (400 MHz, DMSO-d6) δ 11.86 (s, 1H), 10.81 (s, 2H), 8.93 (s, 1H), 7.99 (s, 1H), 7.74 (d, J = 9.1 Hz, 2H), 7.52 (s, 2H), 7.25 (d, J = 7.6 Hz, 2H), 5.86 - 5.68 (m, 1H), 4.26 (s, 2H), 3.88 - 3.79 (m, 3H), 3.53 (s, 6H), 2.80 - 2.62 (m, 3H), 2.47 (s, 2H), 2.37 (s, 3H), 2.27 (s, 3H), 2.14 (s, 6H), 2.00 - 1.69 (m, 8H), 1.53 (s, 2H). LCMS (ESI) calcd for C₄₁H₅₀N₉O₄⁺ [M+H]⁺: 732.40; found, 732.4.

### Example 319: Preparation of 3-(4-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)phenyl)piperidine-2,6-dione (GT-06664)

According to the method of step 2 of Scheme I-1, the target compound (GT-06664) was prepared (white solid, 10 mg, yield 18.69%). ¹H NMR (400 MHz, DMSO-d6) δ 12.38 - 11.74 (m, 1H), 11.07 (s, 1H), 10.88 (s, 1H), 8.84 (d, J = 3.4 Hz, 1H), 8.54 (s, 1H), 8.37 (s, 1H), 8.20 (s, 2H), 7.85 - 7.29 (m, 5H), 4.93 (s, 1H), 4.37 (s, 2H), 3.98 - 3.90 (m, 5H), 3.53 (s, 6H), 2.76 (s, 3H), 2.69 (s, 1H), 2.52 (s, 1H), 2.29 - 2.17 (m, 1H), 2.09 - 1.98 (m, 1H), 1.67 (d, J = 6.9 Hz, 6H). LCMS (ESI) calcd for C₃₇H₄₀F₂N₉O₂⁺ [M+H]⁺: 680.33; found, 680.3.

### Example 320: Preparation of 1-(4-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)phenyl)dihydropyrimidine-2,4(1H,3H)-dione (GT-06665)

According to the method of step 2 of Scheme I-1, the target compound (GT-06665) was prepared (white solid, 15 mg, yield 26.36%). ¹H NMR (400 MHz, DMSO-d6) δ 12.50 - 11.83 (m, 1H), 11.41 (d, J = 64.0 Hz, 1H), 10.44 (s, 1H), 8.86 (d, J = 3.3 Hz, 1H), 8.60 (s, 1H), 8.40 (s, 1H), 8.30 (d, J = 8.8 Hz, 1H), 8.16 (d, J = 8.9 Hz, 1H), 7.88 (d, J = 11.6 Hz, 1H), 7.73 (t, J = 23.3 Hz, 2H), 7.47 (dd, J = 42.4, 8.3 Hz, 2H), 4.96 (dt, J = 13.8, 6.9 Hz, 1H), 4.42 (s, 3H), 3.82 (t, J = 6.6 Hz, 5H), 3.54 (s, 6H), 2.81 (s, 3H), 2.72 (t, J = 6.6 Hz, 2H), 1.68 (d, J = 6.9 Hz, 6H). LCMS (ESI) calcd for C₃₆H₃₉F₂N₁₀O₂⁺ [M+H]⁺: 681.32; found, 681.3.

### Example 321: Preparation of 1-(3-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)phenyl)dihydropyrimidine-2,4(1H,3H)-dione (GT-06666)

According to the method of step 2 of Scheme I-1, the target compound (GT-06666) was prepared (white solid, 26 mg, yield 45.69%). ¹H NMR (400 MHz, DMSO-d6) δ 12.38 (s, 1H), 11.47 (s, 1H), 10.44 (s, 1H), 8.87 (d, J = 3.3 Hz, 1H), 8.61 (s, 1H), 8.41 (s, 1H), 8.34 (d, J = 8.6 Hz, 1H), 8.13 (d, J = 8.9 Hz, 1H), 7.89 (d, J = 11.5 Hz, 1H), 7.65 (s, 1H), 7.57 - 7.41 (m, 3H), 4.96 (dt, J = 13.9, 6.8 Hz, 1H), 4.44 (s, 6H), 3.86 (t, J = 6.6 Hz, 2H), 3.55 (s, 6H), 2.82 (s, 3H), 2.71 (t, J = 6.6 Hz, 2H), 1.68 (d, J = 6.9 Hz, 6H). LCMS (ESI) calcd for C₃₆H₃₉F₂N₁₀O₂⁺ [M+H]⁺: 681.32; found, 681.3.

### Example 322: Preparation of 1-(6-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)pyridin-3-yl)dihydropyrimidine-2,4(1H,3H)-dione (GT-06667)

According to the method of step 2 of Scheme I-1, the target compound (GT-06667) was prepared (white solid, 12 mg, yield 21.06%). ¹H NMR (400 MHz, DMSO-d6) δ 11.49 (s, 1H), 10.60 (s, 1H), 8.88 (d, J = 3.2 Hz, 1H), 8.69 (d, J = 2.4 Hz, 1H), 8.63 (s, 1H), 8.42 (s, 1H), 8.35 (d, J = 8.8 Hz, 1H), 8.16 (d, J = 8.9 Hz, 1H), 7.97 - 7.88 (m, 2H), 7.78 (d, J = 8.5 Hz, 1H), 4.99 (dd, J = 13.7, 6.9 Hz, 1H), 4.53 (d, J = 11.7 Hz, 4H), 3.89 (d, J = 6.6 Hz, 2H), 3.56 (s, 8H), 2.83 (s, 3H), 2.76 (t, J = 6.6 Hz, 2H), 1.69 (d, J = 6.9 Hz, 6H). LCMS (ESI) calcd for C₃₅H₃₈F₂N₁₁O₂⁺ [M+H]⁺: 682.32; found, 682.3.

### Example 323: Preparation of 1-(5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)pyridin-2-yl)dihydropyrimidine-2,4(1H,3H)-dione (GT-06668)

According to the method of step 2 of Scheme I-1, the target compound (GT-06668) was prepared (white solid, 23 mg, yield 40.37%). ¹H NMR (400 MHz, DMSO-d6) δ 12.68 - 11.85 (m, 1H), 11.47 (s, 1H), 10.62 (s, 1H), 8.88 (d, J = 2.9 Hz, 1H), 8.63 (d, J = 16.8 Hz, 2H), 8.42 (s, 1H), 8.34 (d, J = 7.6 Hz, 1H), 8.13 (d, J = 8.8 Hz, 2H), 7.88 (dd, J = 24.4, 10.0 Hz, 2H), 5.02 - 4.89 (m, 1H), 4.49 (d, J = 16.4 Hz, 4H), 4.10 (d, J = 6.2 Hz, 4H), 3.51 (d, J = 34.8 Hz, 8H), 2.83 (s, 3H), 2.70 (t, J = 6.4 Hz, 2H), 1.68 (d, J = 6.8 Hz, 6H). LCMS (ESI) calcd for C₃₅H₃₈F₂N₁₁O₂⁺ [M+H]⁺: 682.32; found, 682.3.

### Example 324: Preparation of 1-(3-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)phenyl)dihydropyrimidine-2,4(1H,3H)-dione (GT-06839)

According to the method of step 2 of Scheme I-1, the target compound (GT-06839) was prepared (white solid, 18 mg, yield 36.46%). ¹H NMR (500 MHz, DMSO) δ 11.91 (s, 1H), 11.41 (s, 1H), 10.53 (s, 1H), 8.94 (d, J = 3.3 Hz, 1H), 8.39 (s, 1H), 8.35 - 8.27 (m, 1H), 8.03 (s, 1H), 7.88 (t, J = 9.9 Hz, 2H), 7.75 (s, 1H), 7.63 - 7.59 (m, 1H), 7.52 (dd, J = 7.6, 4.8 Hz, 2H), 4.99 (dt, J = 13.9, 6.9 Hz, 1H), 4.57 (d, J = 10.0 Hz, 1H), 4.29 (dd, J = 13.0, 7.1 Hz, 1H), 3.95 (d, J = 6.6 Hz, 3H), 3.51 (d, J = 12.0 Hz, 2H), 2.90 (dd, J = 20.4, 10.8 Hz, 2H), 2.83 (s, 3H), 2.79 (dd, J = 7.7, 6.3 Hz, 2H), 2.66 (d, J = 4.8 Hz, 3H), 2.46 (d, J = 11.6 Hz, 1H), 2.35 (d, J = 11.2 Hz, 1H), 2.08 - 1.95 (m, 2H), 1.71 (d, J = 6.9 Hz, 6H). LCMS (ESI) calcd for C₃₇H₄₁F₂N₁₀O₂⁺ [M+H]⁺: 695.34; found, 695.3.

### Example 325: Preparation of 1-(6-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)pyridin-3-yl)dihydropyrimidine-2,4(1H,3H)-dione (GT-06840)

According to the method of step 2 of Scheme I-1, the target compound (GT-06840) was prepared (white solid, 15 mg, yield 30.34%). ¹H NMR (500 MHz, DMSO) δ 11.87 (s, 1H), 11.02 (s, 1H), 10.61 (s, 1H), 8.89 (d, J = 3.2 Hz, 1H), 8.70 (d, J = 2.5 Hz, 1H), 8.34 (s, 1H), 8.26 (d, J = 7.0 Hz, 1H), 7.98 (s, 1H), 7.92 (dd, J = 8.4, 2.6 Hz, 1H), 7.86 - 7.78 (m, 3H), 4.94 (dt, J = 13.9, 6.9 Hz, 1H), 4.52 (s, 2H), 3.90 (d, J = 6.7 Hz, 3H), 3.50 (d, J = 11.5 Hz, 2H), 2.83 (dd, J = 22.5, 10.6 Hz, 2H), 2.79 - 2.73 (m, 8H), 2.34 (d, J = 29.3 Hz, 2H), 1.95 (dd, J = 20.8, 11.8 Hz, 2H), 1.66 (d, J = 6.9 Hz, 6H). LCMS (ESI) calcd for C₃₆H₄₀F₂N₁₁O₂⁺ [M+H]⁺: 696.33; found, 696.3.

### Example 326: Preparation of 1-(5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)pyridin-2-yl)dihydropyrimidine-2,4(1H,3H)-dione (GT-06841)

According to the method of step 2 of Scheme I-1, the target compound (GT-06841) was prepared (white solid, 20 mg, yield 40.45%). ¹H NMR (500 MHz, DMSO) δ 11.89 (s, 1H), 11.58 (s, 1H), 10.63 (s, 1H), 8.89 (d, J = 3.2 Hz, 1H), 8.67 (d, J = 2.0 Hz, 1H), 8.34 (s, 1H), 8.29 - 8.24 (m, 1H), 8.18 (dd, J = 8.7, 2.3 Hz, 1H), 7.97 (s, 1H), 7.86 - 7.81 (m, 3H), 4.95 (dd, J = 13.8, 6.9 Hz, 1H), 4.55 - 4.49 (m, 1H), 4.29 - 4.25 (m, 1H), 4.10 (d, J = 6.5 Hz, 2H), 3.61 (s, 2H), 3.45 (s, 1H), 2.86 (dd, J = 23.6, 11.9 Hz, 2H), 2.78 (s, 3H), 2.70 (t, J = 6.6 Hz, 2H), 2.59 (t, J = 7.1 Hz, 3H), 2.40 (d, J = 11.8 Hz, 1H), 2.30 (d, J = 11.4 Hz, 1H), 2.03 - 1.89 (m, 2H), 1.65 (d, J = 6.9 Hz, 6H). LCMS (ESI) calcd for C₃₆H₄₀F₂N₁₁O₂⁺ [M+H]⁺: 696.33; found, 696.4.

### Example 327: Preparation of 1-(4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)dihydropyrimidine-2,4(1H,3H)-dione (GT-06739)

According to the method of step 2 of Scheme I-1, the target compound (GT-06739) was prepared (white solid, 22 mg, yield 35.70%). ¹H NMR (400 MHz, DMSO-d6) δ 12.40 (s, 1H), 11.73 (s, 1H), 10.44 (s, 1H), 8.85 (d, J = 3.3 Hz, 1H), 8.29 (s, 1H), 8.20 (d, J = 8.4 Hz, 1H), 7.95 (s, 1H), 7.79 (t, J = 10.4 Hz, 2H), 7.70 (d, J = 8.4 Hz, 2H), 7.42 (d, J = 8.4 Hz, 2H), 4.91 (dt, J = 13.9, 6.8 Hz, 1H), 4.45 (d, J = 27.6 Hz, 2H), 3.92 - 3.80 (m, 8H), 3.43 (dd, J = 14.0, 7.0 Hz, 6H), 2.83 (t, J = 11.6 Hz, 2H), 2.76 - 2.72 (m, 4H), 2.23 (d, J = 8.8 Hz, 2H), 1.86 (d, J = 8.9 Hz, 2H), 1.64 (d, J = 6.9 Hz, 6H). LCMS (ESI) calcd for C₄₀H₄₆F₂N₁₁O₂⁺ [M+H]⁺: 750.38; found, 750.4.

### Example 328: Preparation of 1-(3-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)dihydropyrimidine-2,4(1H,3H)-dione (GT-06740)

According to the method of step 2 of Scheme I-1, the target compound (GT-06740) was prepared (white solid, 24 mg, yield 38.95%). ¹H NMR (400 MHz, DMSO-d6) δ 12.42 (s, 1H), 11.75 (s, 1H), 10.46 (s, 1H), 8.87 (d, J = 3.3 Hz, 1H), 8.30 (s, 1H), 8.21 (d, J = 9.3 Hz, 1H), 7.95 (s, 1H), 7.79 (dd, J = 10.6, 4.0 Hz, 2H), 7.67 (s, 1H), 7.52 (s, 1H), 7.47 (d, J = 5.3 Hz, 2H), 4.95 - 4.89 (m, 1H), 4.43 (s, 2H), 3.91 - 3.85 (m, 6H), 3.55 - 3.43 (m, 7H), 2.83 (t, J = 12.0 Hz, 2H), 2.76 - 2.71 (m, 5H), 2.24 (d, J = 8.9 Hz, 2H), 1.87 (d, J = 9.7 Hz, 2H), 1.65 (d, J = 6.9 Hz, 6H). LCMS (ESI) calcd for C₄₀H₄₆F₂N₁₁O₂⁺ [M+H]⁺: 750.38; found, 750.4.

### Example 329: Preparation of 1-(6-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)pyridin-3-yl)dihydropyrimidine-2,4(1H,3H)-dione (GT-06741)

According to the method of step 2 of Scheme I-1, the target compound (GT-06741) was prepared (white solid, 26 mg, yield 42.14%). ¹H NMR (400 MHz, DMSO-d6) δ 12.45 (s, 1H), 11.93 (s, 1H), 10.66 (s, 1H), 8.94 (d, J = 3.2 Hz, 1H), 8.76 (d, J = 2.3 Hz, 1H), 8.40 (s, 1H), 8.31 (d, J = 9.3 Hz, 1H), 8.05 - 7.98 (m, 2H), 7.87 (dd, J = 18.8, 9.3 Hz, 3H), 5.07 - 4.95 (m, 1H), 4.60 (s, 2H), 4.00 - 3.89 (m, 12H), 2.96 - 2.79 (m, 8H), 2.31 (d, J = 9.7 Hz, 2H), 1.94 (d, J = 9.4 Hz, 2H), 1.71 (s, 4H). LCMS (ESI) calcd for C₃₉H₄₅F₂N₁₂O₂⁺ [M+H]⁺: 751.38; found, 751.4.

### Example 330: Preparation of 1-(5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)pyridin-2-yl)dihydropyrimidine-2,4(1H,3H)-dione (GT-06742)

According to the method of step 2 of Scheme I-1, the target compound (GT-06742) was prepared (white solid, 23 mg, yield 37.28%). ¹H NMR (400 MHz, DMSO-d6) δ 12.55 (s, 1H), 11.91 (s, 1H), 10.69 - 10.41 (m, 2H), 8.89 (d, J = 3.2 Hz, 1H), 8.68 (s, 1H), 8.47 (d, J = 2.1 Hz, 1H), 8.36 (s, 1H), 8.27 (d, J = 9.9 Hz, 1H), 8.14 (dd, J = 8.7, 2.1 Hz, 1H), 7.96 (s, 1H), 7.89 - 7.77 (m, 4H), 4.96 (dt, J = 13.8, 6.8 Hz, 1H), 4.82 (s, 1H), 4.48 (s, 2H), 4.11 - 4.01 (m, 10H), 2.90 - 2.78 (m, 5H), 2.70 (dd, J = 12.6, 6.2 Hz, 4H), 2.24 (d, J = 9.1 Hz, 2H), 1.88 (d, J = 10.0 Hz, 2H), 1.66 (d, J = 6.9 Hz, 6H). LCMS (ESI) calcd for C₃₉H₄₅F₂N₁₂O₂⁺ [M+H]⁺: 751.38; found, 751.4.

### Example 331: Preparation of 3-((3-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)amino)piperidine-2,6-dione (GT-06743)

According to the method of step 2 of Scheme I-1, the target compound (GT-06743) was prepared (white solid, 17 mg, yield 27.08%). ¹H NMR (400 MHz, DMSO-d6) δ 12.23 (s, 1H), 11.82 (s, 1H), 10.85 (s, 1H), 8.87 (d, J = 3.2 Hz, 1H), 8.31 (s, 1H), 8.23 (d, J = 8.6 Hz, 1H), 7.94 (s, 1H), 7.85 - 7.72 (m, 2H), 7.15 (t, J = 7.8 Hz, 1H), 7.05 (s, 1H), 6.82 (dd, J = 16.6, 7.8 Hz, 2H), 4.91 (dd, J = 13.7, 6.9 Hz, 1H), 4.45 - 4.40 (m, 1H), 4.28 (s, 2H), 3.88 (d, J = 11.3 Hz, 6H), 3.52 (s, 5H), 2.87 - 2.75 (m, 6H), 2.59 (d, J = 17.5 Hz, 1H), 2.20 (d, J = 13.2 Hz, 3H), 1.94 - 1.79 (m, 3H), 1.64 (d, J = 6.9 Hz, 6H). LCMS (ESI) calcd for C₄₁H₄₈F₂N₁₁O₂⁺ [M+H]⁺: 764.40; found, 764.5.

### Example 332: Preparation of 7-cyclopentyl-2-((5-(4-((6-(2,4-dioxotetrahydropyrimidin-1(2H)-yl)pyrazin-2-yl)methyl)piperazin-1-yl)pyridin-2-yl)amino)-N,N-dimethyl-7H-pyrrolo[2,3-d]pyrimidine-6-carboxamide (GT-08020)

According to the method of step 2 of Scheme I-1, the target compound (GT-08020) was prepared (yellow solid, 45 mg, yield 72 %). ¹H NMR (400 MHz, DMSO-d6) δ 12.08 (s, 1H), 11.57 (s, 1H), 10.81 (s, 1H), 9.13 (s, 1H), 9.02 (s, 1H), 8.67 (s, 1H), 8.08 (dd, J = 9.5, 2.8 Hz, 1H), 8.00 (d, J = 2.7 Hz, 1H), 7.67 (d, J = 9.4 Hz, 1H), 6.84 (s, 1H), 4.86 - 4.74 (m, 1H), 4.58 (s, 2H), 4.18 (t, J = 6.6 Hz, 2H), 3.82 (s, 2H), 3.64 (s, 2H), 3.38 (s, 3H), 3.25 (d, J = 11.0 Hz, 1H), 3.06 (s, 6H), 2.74 (t, J = 6.6 Hz, 2H), 2.34 - 2.26 (m, 2H), 2.06 - 1.94 (m, 4H), 1.68 - 1.60 (m, 2H). LCMS (ESI) calcd for C₃₂H₃₉N₁₂O₃ ⁺ [M+H]⁺: 639.33; found, 639.2.

### Example 333: Preparation of 7-cyclopentyl-2-((5-(4-((5-(2,4-dioxotetrahydropyrimidin-1(2H)-yl)-3-fluoropyridin-2-yl)methyl)piperazin-1-yl)pyridin-2-yl)amino)-N,N-dimethyl-7H-pyrrolo[2,3-d]pyrimidine-6-carboxamide (GT-08021)

According to the method of step 2 of Scheme I-1, the target compound (GT-08021) was prepared (yellow solid, 48 mg, yield 75 %). ¹H NMR (400 MHz, DMSO-d6) δ 11.41 (s, 2H), 10.69 (s, 1H), 9.00 (s, 1H), 8.64 (s, 1H), 8.03 (d, J = 9.1 Hz, 1H), 8.01 - 7.94 (m, 2H), 7.68 (d, J = 9.3 Hz, 1H), 6.83 (s, 1H), 4.86 - 4.75 (m, 1H), 4.61 (s, 2H), 3.93 (t, J = 6.6 Hz, 2H), 3.75 (s, 2H), 3.70 - 3.57 (m, 4H), 3.23 (s, 2H), 3.06 (s, 6H), 2.77 (t, J = 6.6 Hz, 2H), 2.37 - 2.27 (m, 2H), 2.07 - 1.95 (m, 4H), 1.71 - 1.60 (m, 2H). LCMS (ESI) calcd for C₃₃H₃₉FN₁₁O₃ ⁺ [M+H]⁺: 656.32; found, 656.2.

### Example 334: Preparation of 1-(6-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyrazin-2-yl)dihydropyrimidine-2,4(1H,3H)-dione (GT-08022)

According to the method of step 2 of Scheme I-1, the target compound (GT-08022) was prepared (yellow solid, 46 mg, yield 82 %). ¹H NMR (400 MHz, DMSO-d6) δ 11.81 (s, 1H), 11.08 (s, 1H), 10.81 (s, 1H), 9.13 (s, 1H), 9.02 (s, 1H), 8.64 (s, 1H), 8.09 (d, J = 2.5 Hz, 1H), 7.92 (d, J = 8.1 Hz, 1H), 7.87 (d, J = 9.3 Hz, 1H), 5.90 - 5.79 (m, 1H), 4.58 (s, 2H), 4.16 (t, J = 6.6 Hz, 2H), 3.64 (s, 2H), 3.46 (t, J = 5.2 Hz, 1H), 3.36 (s, 4H), 3.25 (s, 1H), 2.74 (t, J = 6.6 Hz, 2H), 2.44 (s, 3H), 2.35 (s, 3H), 2.27 - 2.20 (m, 2H), 1.94 (s, 2H), 1.84 - 1.77 (m, 2H), 1.63 - 1.55 (m, 2H). LCMS (ESI) calcd for C₃₃H₃₈N₁₁O₄ ⁺ [M+H]⁺: 652.31; found, 652.2.

### Example 335: Preparation of 1-(6-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-5-fluoropyridin-3-yl)dihydropyrimidine-2,4(1H,3H)-dione (GT-08023)

According to the method of step 2 of Scheme I-1, the target compound (GT-08023) was prepared (yellow solid, 47 mg, yield 81 %). ¹H NMR (400 MHz, DMSO-d6) δ 11.57 (s, 1H), 11.23 (s, 1H), 10.69 (s, 1H), 9.03 (s, 1H), 8.64 (s, 1H), 8.11 (d, J = 2.8 Hz, 1H), 8.04 - 7.94 (m, 2H), 7.92 - 7.87 (m, 1H), 5.89 - 5.80 (m, 1H), 4.62 (s, 2H), 3.93 (t, J = 6.6 Hz, 3H), 3.63 (s, 2H), 3.52 - 3.44 (m, 2H), 3.43 - 3.29 (m, 2H), 3.25 (s, 1H), 2.77 (t, J = 6.6 Hz, 2H), 2.44 (s, 3H), 2.35 (s, 3H), 2.28 - 2.18 (m, 2H), 2.00 - 1.91 (m, 2H), 1.86 - 1.76 (m, 2H), 1.65 - 1.55 (m, 2H). LCMS (ESI) calcd for C₃₄H₃₈FN₁₀O₄ ⁺ [M+H]⁺: 669.31; found, 669.2.

### Example 336: Preparation of 7-cyclopentyl-2-((5-(4-((2-(2,6-dioxopiperidin-3-yl)-5-fluoropyridin-4-yl)methyl)piperazin-1-yl)pyridin-2-yl)amino)-N,N-dimethyl-7H-pyrrolo[2,3-d]pyrimidine-6-carboxamide (GT-08071)

According to the method of step 2 of Scheme I-1, the target compound (GT-08071) was prepared (yellow solid, 45 mg, yield 71 %). ¹H NMR (400 MHz, DMSO-d6) δ 12.26 (s, 1H), 11.68 (s, 1H), 10.93 (s, 1H), 9.03 (s, 1H), 8.71 - 8.60 (m, 1H), 8.10 (dd, J = 9.5, 2.8 Hz, 1H), 8.03 - 7.87 (m, 2H), 7.65 (d, J = 9.5 Hz, 1H), 6.85 (s, 1H), 4.86 - 4.75 (m, 1H), 4.56 (s, 2H), 4.09 (dd, J = 9.8, 5.3 Hz, 1H), 3.55 - 3.45 (m, 4H), 3.40 (s, 4H), 3.06 (s, 6H), 2.72 - 2.64 (m, 1H), 2.59 - 2.53 (m, 1H), 2.37 - 2.25 (m, 3H), 2.22 - 2.15 (m, 1H), 2.07 - 1.94 (m, 4H), 1.71 - 1.59 (m, 2H). LCMS (ESI) calcd for C₃₄H₄₀FN₁₀O₃⁺ [M+H]⁺: 655.33; found, 655.2.

### Example 337: Preparation of 3-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-5-fluoropyridin-2-yl)piperidine-2,6-dione (GT-08072)

According to the method of step 2 of Scheme I-1, the target compound (GT-08072) was prepared (white solid, 25 mg, yield 43 %). ¹H NMR (400 MHz, DMSO-d6) δ 11.77 (s, 1H), 10.98 (s, 2H), 9.03 (s, 1H), 8.64 (s, 1H), 8.08 (s, 1H), 7.99 - 7.76 (m, 3H), 5.89 - 5.75 (m, 1H), 4.51 (s, 2H), 4.09 (dd, J = 9.8, 5.3 Hz, 1H), 3.82 (s, 2H), 3.32 (s, 4H), 2.72 - 2.63 (m, 1H), 2.59 - 2.54 (m, 1H), 2.44 (s, 3H), 2.37 - 2.32 (m, 3H), 2.27 - 2.17 (m, 3H), 1.98 - 1.89 (m, 2H), 1.85 - 1.75 (m, 2H), 1.67 - 1.52 (m, 2H). LCMS (ESI) calcd for C₃₅H₃₉FN₉O₄⁺ [M+H]⁺: 668.31; found, 668.2.

### Example 338: Preparation of 2-((5-bromo-2-((4-((4-(4-(2,6-dioxopiperidin-3-yl)benzyl)piperazin-1-yl)sulfonyl)-2-methylphenyl)amino)pyrimidin-4-yl)amino)-6-fluorobenzamide (GT-06830)

According to the method of step 2 of Scheme I-1, the target compound (GT-06830) was prepared (white solid, 23 mg, yield 42.39%). ¹H NMR (400 MHz, DMSO-d6) δ 11.07 (s, 1H), 10.86 (s, 1H), 10.34 (s, 1H), 9.36 (s, 1H), 8.38 (s, 1H), 8.13 (d, J = 26.7 Hz, 2H), 8.05 (d, J = 8.4 Hz, 1H), 7.91 (d, J = 8.5 Hz, 1H), 7.59 (s, 1H), 7.51 (t, J = 6.6 Hz, 3H), 7.32 (dd, J = 17.9, 7.4 Hz, 3H), 7.07 - 6.96 (m, 1H), 4.31 (s, 2H), 3.91 (dd, J = 11.7, 4.8 Hz, 1H), 3.79 (s, 2H), 3.36 (d, J = 10.6 Hz, 2H), 3.18 (s, 2H), 2.83 - 2.63 (m, 3H), 2.48 (s, 1H), 2.34 (s, 3H), 2.21 (dd, J = 12.5, 3.7 Hz, 1H), 2.05 - 1.96 (m, 1H). LCMS (ESI) calcd for C₃₄H₃₅BrFN₈O₅S⁺ [M+H]⁺: 765.16; found, 765.2.

### Example 339: Preparation of 2-((5-bromo-2-((4-((4-(4-(2,4-dioxotetrahydropyrimidin-1(2H)-yl)benzyl)piperazin-1-yl)sulfonyl)-2-methylphenyl)amino)pyrimidin-4-yl)amino)-6-fluorobenzamide (GT-06831)

According to the method of step 2 of Scheme I-1, the target compound (GT-06831) was prepared (white solid, 22 mg, yield 40.49%). ¹H NMR (400 MHz, DMSO-d6) δ 11.18 (s, 1H), 10.43 (s, 1H), 10.36 (s, 1H), 9.40 (s, 1H), 8.39 (s, 1H), 8.13 (d, J = 25.9 Hz, 2H), 7.97 (dd, J = 54.3, 8.5 Hz, 2H), 7.70 - 7.48 (m, 5H), 7.43 - 7.37 (m, 2H), 7.37 - 7.31 (m, 1H), 7.10 - 6.95 (m, 1H), 4.35 (d, J = 26.1 Hz, 2H), 3.83 (s, 2H), 3.77 - 3.74 (m, 2H), 3.36 (d, J = 10.6 Hz, 2H), 3.18 (s, 2H), 2.79 (t, J = 11.0 Hz, 2H), 2.71 (t, J = 6.6 Hz, 2H), 2.35 (s, 3H). LCMS (ESI) calcd for C₃₃H₃₄BrFN₉O₅S⁺ [M+H]⁺: 766.16; found, 766.2.

### Example 340: Preparation of 2-((5-bromo-2-((4-((4-(3-(2,4-dioxotetrahydropyrimidin-1(2H)-yl)benzyl)piperazin-1-yl)sulfonyl)-2-methylphenyl)amino)pyrimidin-4-yl)amino)-6-fluorobenzamide (GT-06832)

According to the method of step 2 of Scheme I-1, the target compound (GT-06832) was prepared (white solid, 28 mg, yield 51.54%). ¹H NMR (400 MHz, DMSO-d6) δ 11.02 (s, 1H), 10.45 (s, 1H), 10.33 (s, 1H), 9.34 (s, 1H), 8.38 (s, 1H), 8.17 (s, 1H), 8.12 - 8.04 (m, 2H), 7.91 (d, J = 8.5 Hz, 1H), 7.60 (s, 1H), 7.53 (dd, J = 12.8, 4.3 Hz, 2H), 7.47 - 7.29 (m, 6H), 7.07 - 6.99 (m, 1H), 4.78 (s, 1H), 4.35 (s, 2H), 3.87 - 3.78 (m, 4H), 3.37 (s, 1H), 3.19 (s, 2H), 2.77 - 2.68 (m, 4H), 2.35 (s, 3H). LCMS (ESI) calcd for C₃₃H₃₄BrFN₉O₅S⁺ [M+H]⁺: 766.16; found, 766.2.

### Example 341: Preparation of 2-((5-bromo-2-((4-((4-((5-(2,4-dioxotetrahydropyrimidin-1(2H)-yl)pyridin-2-yl)methyl)piperazin-1-yl)sulfonyl)-2-methylphenyl)amino)pyrimidin-4-yl)amino)-6-fluorobenzamide (GT-06833)

According to the method of step 2 of Scheme I-1, the target compound (GT-06833) was prepared (white solid, 28 mg, yield 51.47%). ¹H NMR (400 MHz, DMSO-d6) δ 10.57 (s, 1H), 10.44 (d, J = 9.5 Hz, 1H), 9.60 (d, J = 23.3 Hz, 1H), 8.64 (d, J = 2.4 Hz, 1H), 8.43 (d, J = 3.9 Hz, 1H), 8.14 (d, J = 24.9 Hz, 2H), 8.01 (d, J = 8.0 Hz, 1H), 7.97 - 7.85 (m, 2H), 7.68 (d, J = 8.4 Hz, 1H), 7.61 (d, J = 1.7 Hz, 1H), 7.53 (d, J = 8.5 Hz, 1H), 7.36 (dd, J = 14.9, 8.3 Hz, 1H), 7.05 (t, J = 9.2 Hz, 1H), 4.49 (s, 2H), 3.86 (d, J = 6.6 Hz, 2H), 3.49 - 3.10 (m, 8H), 2.74 (t, J = 6.6 Hz, 2H), 2.37 (s, 3H). LCMS (ESI) calcd for C₃₂H₃₃BrFN₁₀O₅S⁺ [M+H]⁺: 767.15; found, 767.3.

### Example 342: Preparation of 2-((5-bromo-2-((4-((4-((6-(2,4-dioxotetrahydropyrimidin-1(2H)-yl)pyridin-3-yl)methyl)piperazin-1-yl)sulfonyl)-2-methylphenyl)amino)pyrimidin-4-yl)amino)-6-fluorobenzamide (GT-06834)

According to the method of step 2 of Scheme I-1, the target compound (GT-06834) was prepared (white solid, 29 mg, yield 53.31%). ¹H NMR (400 MHz, DMSO-d6) δ 11.54 (d, J = 56.1 Hz, 1H), 10.61 (s, 1H), 10.45 (s, 1H), 9.56 (s, 1H), 8.55 (d, J = 1.8 Hz, 1H), 8.43 (s, 1H), 8.14 (d, J = 27.3 Hz, 2H), 8.05 - 7.98 (m, 2H), 7.91 (d, J = 8.5 Hz, 1H), 7.83 (d, J = 8.7 Hz, 1H), 7.62 (d, J = 1.6 Hz, 1H), 7.57 - 7.49 (m, 1H), 7.37 (dd, J = 14.8, 8.3 Hz, 1H), 7.13 - 7.03 (m, 1H), 4.38 (s, 2H), 4.08 (t, J = 6.5 Hz, 2H), 3.79 - 3.73 (m, 2H), 3.38 (s, 2H), 3.20 (s, 2H), 2.80 (s, 2H), 2.69 (t, J = 6.5 Hz, 2H), 2.36 (s, 3H). LCMS (ESI) calcd for C₃₂H₃₃BrFN₁₀O₅S⁺ [M+H]⁺: 767.15; found, 767.3.

### Example 343: Preparation of 2-((5-bromo-2-((4-((4-(3-((2,6-dioxopiperidin-3-yl)amino)benzyl)piperazin-1-yl)sulfonyl)-2-methylphenyl)amino)pyrimidin-4-yl)amino)-6-fluorobenzamide (GT-06835)

According to the method of step 2 of Scheme I-1, the target compound (GT-06835) was prepared (white solid, 28 mg, yield 50.61%). ¹H NMR (400 MHz, DMSO-d6) δ 10.82 (s, 1H), 10.66 (s, 1H), 10.32 (s, 1H), 9.29 (s, 1H), 8.36 (s, 1H), 8.16 (s, 1H), 8.12 - 8.03 (m, 2H), 7.90 (d, J = 8.5 Hz, 1H), 7.59 (s, 1H), 7.51 (d, J = 8.4 Hz, 1H), 7.34 (dd, J = 14.9, 8.3 Hz, 1H), 7.13 (t, J = 7.8 Hz, 1H), 7.06 - 6.98 (m, 1H), 6.86 (s, 1H), 6.76 (d, J = 8.2 Hz, 1H), 6.69 (d, J = 7.4 Hz, 1H), 4.33 (dd, J = 11.7, 4.7 Hz, 1H), 4.18 (s, 1H), 3.77 (d, J = 11.2 Hz, 2H), 3.16 (s, 6H), 2.76 (t, J = 12.2 Hz, 3H), 2.34 (s, 3H), 2.17 - 2.08 (m, 1H), 1.93 - 1.82 (m, 1H). LCMS (ESI) calcd for C₃₄H₃₆BrFN₉O₅S⁺ [M+H]⁺: 780.17; found, 780.2.

### Example 344: Preparation of 3-(4-((4-((4-((5-amino-1-(2,6-difluorobenzoyl)-1H-1,2,4-triazol-3-yl)amino)phenyl)sulfonyl)piperazin-1-yl)methyl)phenyl)piperidine-2,6-dione (GT-06750)

According to the method of step 2 of Scheme I-1, the target compound (GT-06750) was prepared (white solid, 18 mg, yield 31.38%). ¹H NMR (400 MHz, DMSO-d6) δ 10.83 (d, J = 23.6 Hz, 2H), 10.07 (s, 1H), 8.04 (s, 2H), 7.75 - 7.66 (m, 1H), 7.56 (d, J = 9.0 Hz, 2H), 7.49 (dd, J = 8.4, 3.5 Hz, 3H), 7.38 - 7.26 (m, 4H), 7.11 (q, J = 8.1 Hz, 1H), 4.27 (s, 2H), 3.85 (ddd, J = 41.1, 11.5, 4.9 Hz, 1H), 3.66 (d, J = 10.9 Hz, 2H), 3.32 (d, J = 10.8 Hz, 2H), 3.13 (s, 2H), 2.73 - 2.60 (m, 3H), 2.49 - 2.41 (m, 1H), 2.26 - 2.12 (m, 1H), 2.06 - 1.93 (m, 1H). LCMS (ESI) calcd for C₃₁H₃₁F₂N₈O₅S⁺ [M+H]⁺: 665.21; found, 665.3.

### Example 345: Preparation of 1-(4-((4-((4-((5-amino-1-(2,6-difluorobenzoyl)-1H-1,2,4-triazol-3-yl)amino)phenyl)sulfonyl)piperazin-1-yl)methyl)phenyl)dihydropyrimidine-2,4(1H,3H)-dione (GT-06751)

According to the method of step 2 of Scheme I-1, the target compound (GT-06751) was prepared (white solid, 22 mg, yield 38.29%). ¹H NMR (400 MHz, DMSO-d6) δ 10.82 (s, 1H), 10.43 (s, 1H), 10.07 (s, 1H), 8.04 (s, 2H), 7.77 - 7.67 (m, 1H), 7.59 - 7.48 (m, 6H), 7.41 - 7.31 (m, 4H), 4.31 (d, J = 24.1 Hz, 2H), 3.79 (t, J = 6.6 Hz, 2H), 3.67 (d, J = 10.9 Hz, 2H), 3.33 (d, J = 12.1 Hz, 2H), 3.14 (s, 2H), 2.69 (dd, J = 17.3, 10.6 Hz, 4H). LCMS (ESI) calcd for C₃₀H₃₀F₂N₉O₅S⁺ [M+H]⁺: 666.21; found, 666.4.

### Example 346: Preparation of 1-(3-((4-((4-((5-amino-1-(2,6-difluorobenzoyl)-1H-1,2,4-triazol-3-yl)amino)phenyl)sulfonyl)piperazin-1-yl)methyl)phenyl)dihydropyrimidine-2,4(1H,3H)-dione (GT-06752)

According to the method of step 2 of Scheme I-1, the target compound (GT-06752) was prepared (white solid, 17 mg, yield 29.59%). ¹H NMR (400 MHz, DMSO-d6) δ 10.85 (s, 1H), 10.42 (d, J = 15.1 Hz, 1H), 10.07 (s, 1H), 8.05 (s, 1H), 7.75 - 7.67 (m, 1H), 7.53 (q, J = 9.0 Hz, 4H), 7.42 (t, J = 6.5 Hz, 2H), 7.34 (t, J = 8.3 Hz, 2H), 4.30 (s, 2H), 3.81 (dd, J = 14.2, 7.5 Hz, 2H), 3.66 (d, J = 10.9 Hz, 2H), 3.33 (d, J = 12.0 Hz, 2H), 3.11 (s, 2H), 2.70 (dd, J = 12.8, 6.4 Hz, 4H). LCMS (ESI) calcd for C₃₀H₃₀F₂N₉O₅S⁺ [M+H]⁺: 666.21; found, 666.4.

### Example 347: Preparation of 1-(6-((4-((4-((5-amino-1-(2,6-difluorobenzoyl)-1H-1,2,4-triazol-3-yl)amino)phenyl)sulfonyl)piperazin-1-yl)methyl)pyridin-3-yl)dihydropyrimidine-2,4(1H,3H)-dione (GT-06753)

According to the method of step 2 of Scheme I-1, the target compound (GT-06753) was prepared (white solid, 30 mg, yield 52.14%). ¹H NMR (400 MHz, DMSO-d6) δ 10.57 (s, 1H), 10.08 (s, 1H), 8.63 (d, J = 2.3 Hz, 1H), 8.05 (s, 2H), 7.85 (d, J = 2.6 Hz, 1H), 7.76 - 7.67 (m, 1H), 7.64 - 7.50 (m, 5H), 7.35 (t, J = 8.3 Hz, 2H), 4.45 (s, 2H), 3.85 (t, J = 6.6 Hz, 2H), 3.41 (s, 2H), 3.34 (s, 4H), 3.10 (d, J = 36.3 Hz, 3H), 2.73 (t, J = 6.6 Hz, 2H). LCMS (ESI) calcd for C₂₉H₂₉F₂N₁₀O₅S⁺ [M+H]⁺: 667.20; found, 667.4.

### Example 348: Preparation of 1-(5-((4-((4-((5-amino-1-(2,6-difluorobenzoyl)-1H-1,2,4-triazol-3-yl)amino)phenyl)sulfonyl)piperazin-1-yl)methyl)pyridin-2-yl)dihydropyrimidine-2,4(1H,3H)-dione (GT-06754)

According to the method of step 2 of Scheme I-1, the target compound (GT-06754) was prepared (white solid, 21 mg, yield 36.50%). ¹H NMR (400 MHz, DMSO-d6) δ 11.19 (s, 1H), 10.60 (s, 1H), 10.07 (s, 1H), 8.51 (d, J = 1.8 Hz, 1H), 8.11 - 7.97 (m, 2H), 7.81 (d, J = 8.6 Hz, 1H), 7.74 - 7.65 (m, 1H), 7.58 - 7.49 (m, 3H), 7.34 (t, J = 8.3 Hz, 2H), 4.33 (s, 2H), 4.06 (t, J = 6.5 Hz, 2H), 3.65 (s, 2H), 3.43 - 3.27 (m, 4H), 3.15 (s, 2H), 2.69 (d, J = 6.5 Hz, 2H). LCMS (ESI) calcd for C₂₉H₂₉F₂N₁₀O₅S⁺ [M+H]⁺: 667.20; found, 667.3.

### Example 349: Preparation of 3-((3-((4-((4-((5-amino-1-(2,6-difluorobenzoyl)-1H-1,2,4-triazol-3-yl)amino)phenyl)sulfonyl)piperazin-1-yl)methyl)phenyl)amino)piperidine-2,6-dione (GT-06755)

According to the method of step 2 of Scheme I-1, the target compound (GT-06755) was prepared (white solid, 23 mg, yield 39.21%). ¹H NMR (400 MHz, DMSO-d6) δ 10.79 (d, J = 24.4 Hz, 2H), 10.07 (s, 1H), 8.04 (s, 2H), 7.76 - 7.64 (m, 2H), 7.60 - 7.48 (m, 4H), 7.34 (t, J = 8.4 Hz, 2H), 7.13 (dd, J = 22.3, 14.5 Hz, 1H), 6.88 (d, J = 13.7 Hz, 1H), 6.72 (dd, J = 21.3, 7.1 Hz, 2H), 4.32 (dd, J = 11.7, 4.6 Hz, 1H), 4.15 (s, 2H), 3.68 (s, 2H), 3.31 (d, J = 11.2 Hz, 2H), 3.11 (s, 2H), 2.74 (ddd, J = 25.7, 19.3, 8.0 Hz, 3H), 2.57 (dd, J = 17.3, 3.7 Hz, 1H), 2.14 (dd, J = 9.0, 3.9 Hz, 1H), 1.95 - 1.77 (m, 1H). LCMS (ESI) calcd for C₃₁H₃₂F₂N₉O₅S⁺ [M+H]⁺: 680.22; found, 680.3.

### Example 350: Preparation of 3-(4-((4-((4-((5-amino-1-(3-methylthiophene-2-carbonyl)-1H-1,2,4-triazol-3-yl)amino)phenyl)sulfonyl)piperazin-1-yl)methyl)phenyl)piperidine-2,6-dione (GT-06854)

According to the method of step 2 of Scheme I-1, the target compound (GT-06854) was prepared (white solid, 28 mg, yield 48.28%). ¹H NMR (500 MHz, DMSO) δ 10.87 (s, 1H), 10.82 (s, 1H), 10.13 (s, 1H), 8.04 (t, J = 4.9 Hz, 1H), 7.90 (d, J = 8.8 Hz, 3H), 7.68 (t, J = 11.2 Hz, 2H), 7.49 (d, J = 8.1 Hz, 2H), 7.27 (t, J = 10.9 Hz, 2H), 7.17 (t, J = 4.8 Hz, 1H), 7.11 (dd, J = 20.4, 8.0 Hz, 1H), 4.29 (s, 2H), 3.89 (dd, J = 11.8, 4.9 Hz, 1H), 3.71 (d, J = 11.7 Hz, 2H), 3.35 (d, J = 11.3 Hz, 2H), 3.16 (d, J = 6.7 Hz, 2H), 2.75 - 2.60 (m, 6H), 2.49 - 2.43 (m, 1H), 2.23 - 2.14 (m, 1H), 2.05 - 1.96 (m, 1H). LCMS (ESI) calcd for C₃₀H₃₃N₅O_{S}S₂⁺ [M+H]⁺: 649.20; found, 649.3.

### Example 351: Preparation of 3-(4-((4-((4-((5-amino-1-(3-methylthiophene-2-carbonyl)-1H-1,2,4-triazol-3-yl)amino)phenyl)sulfonyl)piperazin-1-yl)methyl)-2-fluorophenyl)piperidine-2,6-dione (GT-06855)

According to the method of step 2 of Scheme I-1, the target compound (GT-06855) was prepared (white solid, 28 mg, yield 46.99 %). ¹H NMR (500 MHz, DMSO) δ 10.93 (s, 1H), 10.12 (s, 1H), 8.03 (d, J = 5.0 Hz, 1H), 7.91 (d, J = 8.8 Hz, 3H), 7.71 (d, J = 9.0 Hz, 2H), 7.47 - 7.31 (m, 3H), 7.16 (d, J = 5.0 Hz, 1H), 4.32 (s, 2H), 4.08 (dd, J = 12.7, 4.9 Hz, 1H), 3.72 (s, 2H), 3.36 (s, 2H), 3.19 (s, 2H), 2.79 - 2.64 (m, 3H), 2.62 (s, 3H), 2.55 (d, J = 3.6 Hz, 1H), 2.19 (dd, J = 13.0, 3.9 Hz, 1H), 2.01 - 1.93 (m, 1H). LCMS (ESI) calcd for C₃₀H₃₂FN₈O₅S₂⁺ [M+H]⁺: 667.19; found, 667.3.

### Example 352: Preparation of 1-(4-((4-((4-((5-amino-1-(3-methylthiophene-2-carbonyl)-1H-1,2,4-triazol-3-yl)amino)phenyl)sulfonyl)piperazin-1-yl)methyl)phenyl)dihydropyrimidine-2,4(1H,3H)-dione (GT-06856)

According to the method of step 2 of Scheme I-1, the target compound (GT-06856) was prepared (white solid, 34 mg, yield 58.55%). ¹H NMR (500 MHz, DMSO) δ 11.09 (d, J = 93.3 Hz, 1H), 10.43 (s, 1H), 10.12 (s, 1H), 8.04 (d, J = 5.0 Hz, 1H), 7.91 (d, J = 8.8 Hz, 3H), 7.71 (d, J = 9.0 Hz, 2H), 7.56 (d, J = 8.5 Hz, 2H), 7.39 (d, J = 8.5 Hz, 2H), 7.17 (d, J = 5.0 Hz, 1H), 4.31 (s, 2H), 3.79 (t, J = 6.6 Hz, 2H), 3.72 (d, J = 11.6 Hz, 2H), 3.36 (d, J = 10.7 Hz, 2H), 3.17 (s, 2H), 2.70 (t, J = 6.6 Hz, 4H), 2.63 (s, 3H). LCMS (ESI) calcd for C₂₉H₃₂N₉O₅S₂⁺ [M+H]⁺: 650.20; found, 650.2.

### Example 353: Preparation of 1-(4-((4-((4-((5-amino-1-(3-methylthiophene-2-carbonyl)-1H-1,2,4-triazol-3-yl)amino)phenyl)sulfonyl)piperazin-1-yl)methyl)-2-fluorophenyl)dihydropyrimidine-2,4(1H,3H)-dione (GT-06857)

According to the method of step 2 of Scheme I-1, the target compound (GT-06857) was prepared (white solid, 39 mg, yield 65.35%). ¹H NMR (500 MHz, DMSO) δ 11.12 (s, 1H), 10.53 (s, 1H), 10.11 (s, 1H), 8.03 (d, J = 5.0 Hz, 1H), 7.90 (d, J = 8.8 Hz, 3H), 7.70 (d, J = 9.0 Hz, 2H), 7.62 - 7.39 (m, 3H), 7.16 (d, J = 5.0 Hz, 1H), 4.34 (s, 2H), 3.71 (t, J = 6.6 Hz, 4H), 3.36 (s, 2H), 3.17 (s, 2H), 2.71 (t, J = 6.7 Hz, 4H), 2.63 (s, 3H). LCMS (ESI) calcd for C₂₉H₃₁FN₉O₅S₂⁺ [M+H]⁺: 668.19; found, 668.2.

### Example 354: Preparation of 1-(4-((4-((4-((5-amino-1-(3-methylthiophene-2-carbonyl)-1H-1,2,4-triazol-3-yl)amino)phenyl)sulfonyl)piperazin-1-yl)methyl)-3-fluorophenyl)dihydropyrimidine-2,4(1H,3H)-dione (GT-06858)

According to the method of step 2 of Scheme I-1, the target compound (GT-06858) was prepared (white solid, 28 mg, yield 46.92%). ¹H NMR (500 MHz, DMSO) δ 10.63 (s, 1H), 10.50 (s, 1H), 10.10 (s, 1H), 8.03 (d, J = 5.0 Hz, 1H), 7.90 (d, J = 8.8 Hz, 3H), 7.70 (d, J = 9.0 Hz, 2H), 7.63 (s, 1H), 7.34 (d, J = 11.4 Hz, 1H), 7.25 (d, J = 7.9 Hz, 1H), 7.16 (d, J = 5.1 Hz, 1H), 4.33 (s, 2H), 3.78 (dt, J = 44.9, 20.6 Hz, 4H), 3.39 (s, 3H), 3.23 (s, 2H), 2.70 (t, J = 6.6 Hz, 3H), 2.63 (s, 3H). LCMS (ESI) calcd for C₂₉H₃₁FN₉O₅S₂⁺ [M+H]⁺: 668.19; found, 668.3.

### Example 355: Preparation of 1-(3-((4-((4-((5-amino-1-(3-methylthiophene-2-carbonyl)-1H-1,2,4-triazol-3-yl)amino)phenyl)sulfonyl)piperazin-1-yl)methyl)phenyl)dihydropyrimidine-2,4(1H,3H)-dione (GT-06859)

According to the method of step 2 of Scheme I-1, the target compound (GT-06859) was prepared (white solid, 38 mg, yield 65.44 %). ¹H NMR (500 MHz, DMSO) δ 10.99 (s, 1H), 10.43 (s, 1H), 10.12 (s, 1H), 8.04 (d, J = 5.0 Hz, 1H), 7.90 (d, J = 8.8 Hz, 3H), 7.70 (d, J = 9.0 Hz, 2H), 7.53 (s, 1H), 7.43 (d, J = 5.1 Hz, 2H), 7.37 (dd, J = 6.4, 2.2 Hz, 1H), 7.17 (d, J = 5.0 Hz, 1H), 4.33 (s, 2H), 3.82 (t, J = 6.6 Hz, 2H), 3.72 (d, J = 11.1 Hz, 2H), 3.36 (d, J = 10.9 Hz, 2H), 3.14 (s, 2H), 2.76 (t, J = 11.3 Hz, 2H), 2.69 (t, J = 6.6 Hz, 2H), 2.63 (s, 3H). LCMS (ESI) calcd for C₂₉H₃₂N₉O₅S₂⁺ [M+H]⁺: 650.20; found, 650.3.

### Example 356: Preparation of 1-(6-((4-((4-((5-amino-1-(3-methylthiophene-2-carbonyl)-1H-1,2,4-triazol-3-yl)amino)phenyl)sulfonyl)piperazin-1-yl)methyl)pyridin-3-yl)dihydropyrimidine-2,4(1H,3H)-dione (GT-06860)

According to the method of step 2 of Scheme I-1, the target compound (GT-06860) was prepared (white solid, 28 mg, yield 48.14%). ¹H NMR (500 MHz, DMSO) δ 10.57 (s, 1H), 10.14 (s, 1H), 8.63 (d, J = 2.5 Hz, 1H), 8.05 (d, J = 5.0 Hz, 1H), 7.95 - 7.86 (m, 4H), 7.72 (d, J = 9.0 Hz, 2H), 7.65 (d, J = 8.4 Hz, 1H), 7.17 (d, J = 5.0 Hz, 1H), 4.47 (s, 2H), 3.85 (t, J = 6.6 Hz, 2H), 3.31 (d, J = 69.1 Hz, 8H), 2.73 (t, J = 6.6 Hz, 2H), 2.64 (s, 3H). LCMS (ESI) calcd for C₂₈H₃₁N₁₀O₅S₂⁺ [M+H]⁺: 651.19; found, 651.2.

### Example 357: Preparation of 1-(5-((4-((4-((5-amino-1-(3-methylthiophene-2-carbonyl)-1H-1,2,4-triazol-3-yl)amino)phenyl)sulfonyl)piperazin-1-yl)methyl)pyridin-2-yl)dihydropyrimidine-2,4(1H,3H)-dione (GT-06861)

According to the method of step 2 of Scheme I-1, the target compound (GT-06861) was prepared (white solid, 20 mg, yield 34.39%). ¹H NMR (500 MHz, DMSO) δ 11.02 (s, 1H), 10.60 (s, 1H), 10.10 (s, 1H), 8.50 (s, 1H), 8.03 (d, J = 5.0 Hz, 1H), 7.96 (d, J = 6.9 Hz, 1H), 7.90 (d, J = 8.8 Hz, 3H), 7.81 (d, J = 8.6 Hz, 1H), 7.70 (d, J = 8.9 Hz, 2H), 7.16 (d, J = 5.0 Hz, 1H), 4.34 (s, 2H), 4.06 (t, J = 6.5 Hz, 2H), 3.71 (s, 2H), 3.18 (s, 5H), 2.74 - 2.66 (m, 3H), 2.62 (s, 3H). LCMS (ESI) calcd for C₂₈H₃₁N₁₀O₅S₂⁺ [M+H]⁺: 651.19; found, 651.2.

### Example 358: Preparation of 3-((3-((4-((4-((5-amino-1-(3-methylthiophene-2-carbonyl)-1H-1,2,4-triazol-3-yl)amino)phenyl)sulfonyl)piperazin-1-yl)methyl)phenyl)amino)piperidine-2,6-dione (GT-06862)

According to the method of step 2 of Scheme I-1, the target compound (GT-06862) was prepared (white solid, 34 mg, yield 57.31%). ¹H NMR (500 MHz, DMSO) δ 10.97 (s, 1H), 10.82 (s, 1H), 10.12 (s, 1H), 8.04 (d, J = 5.0 Hz, 1H), 7.90 (d, J = 8.9 Hz, 4H), 7.69 (dd, J = 10.9, 7.4 Hz, 2H), 7.16 (d, J = 5.0 Hz, 1H), 7.14 - 7.08 (m, 1H), 6.91 (s, 1H), 6.76 (dd, J = 8.2, 1.7 Hz, 1H), 6.71 (d, J = 7.5 Hz, 1H), 4.35 (dd, J = 14.2, 9.4 Hz, 1H), 4.17 (s, 2H), 3.70 (s, 4H), 3.34 (d, J = 11.5 Hz, 2H), 3.13 (d, J = 3.5 Hz, 2H), 2.84 - 2.71 (m, 3H), 2.63 (s, 3H), 2.60 - 2.54 (m, 1H), 2.19 - 2.10 (m, 1H), 1.86 (qd, J = 12.6, 4.6 Hz, 1H). LCMS (ESI) calcd for C₃₀H₃₄N₉O₅S₂⁺ [M+H]⁺: 664.21; found, 664.3.

### Example 359: Preparation of 7-cyclopentyl-2-((5-(4-((2-(2,4-dioxotetrahydropyrimidin-1(2H)-yl)-5-fluoropyridin-4-yl)methyl)piperazin-1-yl)pyridin-2-yl)amino)-N,N-dimethyl-7H-pyrrolo[2,3-d]pyrimidine-6-carboxamide (GT-07474)

According to the method of step 2 of Scheme I-1, the target compound (GT-07474) was prepared (white solid, 47 mg, yield 73.8 %). ¹H NMR (400 MHz, DMSO-d6) δ 11.36 (s, 1H), 10.57 (s, 1H), 8.93 (s, 1H), 8.48 (s, 1H), 8.04 (s, 1H), 7.97 (d, *J =* 9.6 Hz, 1H), 7.89 (s, 1H), 7.58 (d, *J =* 9.4 Hz, 1H), 6.76 (s, 1H), 4.82 - 4.69 (m, 1H), 4.39 (s, 2H), 3.98 (t, *J* = 6.6 Hz, 2H), 3.42 - 3.37 (m, 8H), 2.99 (s, 6H), 2.65 (t, *J* = 6.6 Hz, 2H), 2.31 - 2.19 (m, 2H), 2.03 - 1.87 (m, 4H), 1.64 - 1.51 (m, 2H). LCMS (ESI) calcd for C₃₃H₃₉FN₁₁O₃ ⁺ [M+H]⁺: 656.32; found, 656.4.

### Example 360: Preparation of 7-cyclopentyl-2-((5-(4-((2-(2,4-dioxotetrahydropyrimidin-1(2H)-yl)pyridin-4-yl)methyl)piperazin-1-yl)pyridin-2-yl)amino)-N,N-dimethyl-7H-pyrrolo[2,3-d]pyrimidine-6-carboxamide (GT-07475)

According to the method of step 2 of Scheme I-1, the target compound (GT-07475) was prepared (yellow solid, 43 mg, yield 69.3 %). ¹H NMR (400 MHz, DMSO-d6) δ 12.04 (s, 1H), 11.49 (s, 1H), 10.56 (s, 1H), 8.95 (s, 1H), 8.46 (d, *J* = 5.1 Hz, 1H), 8.07 - 7.96 (m, 1H), 7.91 (s, 2H), 7.59 (t, *J* = 7.1 Hz, 2H), 6.77 (s, 1H), 4.84 - 4.64 (m, 1H), 4.41 (s, 2H), 4.03 (t, *J* = 6.6 Hz, 2H), 3.74 (s, 2H), 3.23 - 3.11 (m, 4H), 2.99 (s, 6H), 2.65 (t, *J* = 6.6 Hz, 2H), 2.28 - 2.17 (m, 2H), 2.00 - 1.85 (m, 4H), 1.64 - 1.54 (m, 2H). LCMS (ESI) calcd for C₃₃H₄₀N₁₁O₃⁺ [M+H]⁺: 638.33; found, 638.4.

### Example 361: Preparation of 7-cyclopentyl-2-((5-(4-((5-(2,4-dioxotetrahydropyrimidin-1(2H)-yl)pyridin-3-yl)methyl)piperazin-1-yl)pyridin-2-yl)amino)-N,N-dimethyl-7H-pyrrolo[2,3-d]pyrimidine-6-carboxamide (GT-07476)

According to the method of step 2 of Scheme I-1, the target compound (GT-07476) was prepared (yellow solid, 44 mg, yield 70.9 %). ¹H NMR (400 MHz, DMSO-d6) δ 12.06 (s, 1H), 11.56 (s, 1H), 10.57 (s, 1H), 8.96 (s, 1H), 8.71 (s, 1H), 8.61 (s, 1H), 8.20 (s, 1H), 8.02 (d, *J* = 9.3 Hz, 1H), 7.91 (s, 1H), 7.55 (d, *J =* 9.3 Hz, 1H), 6.78 (s, 1H), 4.80 - 4.71 (m, 1H), 4.46 (s, 2H), 3.88 (t, *J* = 16.0, 9.4 Hz, 3H), 3.75 (s, 2H), 3.22 (s, 4H), 2.99 (s, 6H), 2.70 (t, *J* = 6.6 Hz, 2H), 2.28 - 2.19 (m, 2H), 2.00 - 1.87 (m, 4H), 1.64 - 1.54 (m, 2H). LCMS (ESI) calcd for C₃₃H₄ₒN₁₁O₃⁺ [M+H]⁺: 638.33; found, 638.4.

### Example 362: Preparation of 7-cyclopentyl-2-((5-(4-((5-(2,6-dioxopiperidin-3-yl)pyridin-2-yl)methyl)piperazin-1-yl)pyridin-2-yl)amino)-N,N-dimethyl-7H-pyrrolo[2,3-d]pyrimidine-6-carboxamide (GT-07477)

According to the method of step 2 of Scheme I-1, the target compound (GT-07477) was prepared (yellow solid, 46 mg, yield 74.2 %). ¹H NMR (400 MHz, DMSO-d6) δ 11.34 (s, 1H), 10.90 (s, 1H), 8.93 (s, 1H), 8.52 (d, *J* = 2.1 Hz, 1H), 8.00 - 7.88 (m, 2H), 7.78 (dd, *J* = 8.1, 2.2 Hz, 1H), 7.62 (dd, *J* = 16.3, 8.6 Hz, 2H), 6.76 (s, 1H), 4.77 - 4.68 (m, 1H), 4.49 (s, 2H), 3.99 (dd, *J* = 12.6, 4.8 Hz, 1H), 3.48 (s, 6H), 3.18 - 3.10 (m, 2H), 2.99 (s, 6H), 2.73 - 2.63 (m, 1H), 2.54 - 2.47 (m, 1H), 2.30 - 2.20 (m, 3H), 2.01 - 1.85 (m, 5H), 1.65 - 1.51 (m, 2H). LCMS (ESI) calcd for C₃₄H₄₁N₁₀O₃⁺ [M+H]⁺: 637.34; found, 637.4.

### Example 363: Preparation of 7-cyclopentyl-2-((5-(4-((2-(2,6-dioxopiperidin-3-yl)pyridin-4-yl)methyl)piperazin-1-yl)pyridin-2-yl)amino)-N,N-dimethyl-7H-pyrrolo[2,3-d]pyrimidine-6-carboxamide (GT-07478)

According to the method of step 2 of Scheme I-1, the target compound (GT-07478) was prepared (yellow solid, 19 mg, yield 30.7 %). ¹H NMR (400 MHz, DMSO-d6) δ 12.03 (s, 1H), 11.54 (s, 1H), 10.87 (s, 1H), 8.95 (s, 1H), 8.59 (d, *J* = 5.0 Hz, 1H), 8.00 (d, *J* = 9.4 Hz, 1H), 7.90 (d, *J* = 2.6 Hz, 1H), 7.65 (s, 2H), 7.53 (d, *J* = 9.5 Hz, 1H), 6.78 (s, 1H), 4.74 (p, *J* = 8.6 Hz, 1H), 4.40 (s, 2H), 4.03 (dd, *J* = 9.3, 5.2 Hz, 1H), 3.73 (s, 3H), 3.30 - 3.27 (m, 2H), 3.20 (s, 3H), 2.99 (s, 6H), 2.65 - 2.56 (m, 1H), 2.52 - 2.47 (m, 1H), 2.28 - 2.19 (m, 3H), 2.17 - 2.10 (m, 1H), 2.00 - 1.88 (m, 4H), 1.62 - 1.53 (m, 2H). LCMS (ESI) calcd for C₃₄H₄₁N₁₀O₃⁺ [M+H]⁺: 637.34; found, 637.4.

### Example 364: Preparation of 7-cyclopentyl-2-((5-(4-((5-(2,6-dioxopiperidin-3-yl)pyridin-3-yl)methyl)piperazin-1-yl)pyridin-2-yl)amino)-N,N-dimethyl-7H-pyrrolo[2,3-d]pyrimidine-6-carboxamide (GT-07479)

According to the method of step 2 of Scheme I-1, the target compound (GT-07479) was prepared (yellow solid, 35 mg, yield 56.5 %). ¹H NMR (400 MHz, DMSO-d6) δ 12.29 (s, 1H), 11.72 (s, 1H), 11.09 (s, 1H), 9.09 (s, 1H), 9.00 (s, 1H), 8.83 (s, 1H), 8.48 (s, 1H), 8.16 (dd, *J* = 9.5, 2.7 Hz, 1H), 8.08 - 8.02 (m, 1H), 7.69 (d, *J* = 9.5 Hz, 1H), 6.91 (s, 1H), 4.91 - 4.82 (m, 1H), 4.62 (s, 2H), 4.22 (dd, *J =* 12.5, 4.9 Hz, 2H), 3.65 - 3.57 (m, 3H), 3.37 (s, 4H), 3.12 (s, 6H), 2.89 - 2.80 (m, 1H), 2.73 - 2.66 (m, 1H), 2.45 - 2.33 (m, 3H), 2.26 - 2.19 (m, 1H), 2.13 - 1.99 (m, 4H), 1.76 - 1.67 (m, 2H). LCMS (ESI) calcd for C₃₄H₄₁N₁₀O₃ ⁺ [M+H]⁺: 637.34; found, 637.4.

### Example 365: Preparation of 1-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-5-fluoropyridin-2-yl)dihydropyrimidine-2,4(1H,3H)-dione (GT-07480)

According to the method of step 2 of Scheme I-1, the target compound (GT-07480) was prepared (yellow solid, 45 mg, yield 71.7 %). ¹H NMR (400 MHz, DMSO-d6) δ 11.19 (s, 1H), 10.70 (s, 1H), 10.59 (s, 1H), 8.93 (s, 1H), 8.50 (s, 1H), 8.05 (d, *J =* 5.1 Hz, 1H), 8.01 (d, *J =* 2.8 Hz, 1H), 7.79 (d, *J =* 9.2 Hz, 1H), 7.72 (d, *J* = 8.8 Hz, 1H), 5.77 (p, *J* = 8.7 Hz, 1H), 4.44 (s, 2H), 3.98 (t, *J* = 6.6 Hz, 2H), 3.76 (s, 2H), 3.53 - 3.51 (m, 2H), 3.24 - 3.01 (m, 4H), 2.65 (t, *J* = 6.6 Hz, 2H), 2.37 (s, 3H), 2.27 (s, 3H), 2.22 - 2.12 (m, 2H), 1.89 - 1.80 (m, 2H), 1.77 - 1.68 (m, 2H), 1.56 - 1.48 (m, 2H). LCMS (ESI) calcd for C₃₄H₃₈FN₁₀O₄ ⁺ [M+H]⁺: 669.31; found, 669.4.

### Example 366: Preparation of 1-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-2-yl)dihydropyrimidine-2,4(1H,3H)-dione (GT-07481)

According to the method of step 2 of Scheme I-1, the target compound (GT-07481) was prepared (yellow solid, 40 mg, yield 65.4 %). ¹H NMR (400 MHz, DMSO-d6) δ 11.60 (s, 1H), 10.86 (s, 1H), 10.56 (s, 1H), 8.94 (s, 1H), 8.46 (d, *J =* 5.1 Hz, 1H), 8.01 (s, 1H), 7.92 (s, 1H), 7.85 - 7.74 (m, 2H), 7.53 (s, 1H), 5.77 (p, *J* = 8.9 Hz, 1H), 4.41 (s, 2H), 4.03 (t, *J* = 6.6 Hz, 2H), 3.79 (s, 2H), 3.19 (s, 6H), 2.65 (t, *J* = 6.6 Hz, 2H), 2.37 (s, 3H), 2.27 (s, 3H), 2.21 - 2.12 (m, 2H), 1.90 - 1.81 (m, 2H), 1.77 - 1.68 (m, 2H), 1.56 - 1.46 (m, 2H). LCMS (ESI) calcd for C₃₄H₃₉N₁₀O₄ ⁺ [M+H]⁺: 651.32; found, 651.4.

### Example 367: Preparation of 1-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-3-yl)dihydropyrimidine-2,4(1H,3H)-dione (GT-07482)

According to the method of step 2 of Scheme I-1, the target compound (GT-07482) was prepared (yellow solid, 40 mg, yield 65.4 %). ¹H NMR (400 MHz, DMSO-d6) δ 11.68 (s, 1H), 10.91 (s, 1H), 10.55 (d, *J* = 7.9 Hz, 1H), 8.94 (s, 1H), 8.70 (d, *J* = 2.4 Hz, 1H), 8.58 (s, 1H), 8.12 (s, 1H), 8.01 (s, 1H), 7.79 (s, 2H), 5.81 - 5.75 (m, 1H), 4.44 (s, 2H), 3.88 (t, *J* = 6.6 Hz, 2H), 3.84 - 3.79 (m, 2H), 3.19 (s, 6H), 2.70 (t, *J* = 6.7 Hz, 2H), 2.37 (s, 3H), 2.27 (s, 3H), 2.19 - 2.14 (m, 2H), 1.89 - 1.84 (m, 2H), 1.76 - 1.71 (m, 2H), 1.56 - 1.50 (m, 2H). LCMS (ESI) calcd for C₃₄H₃₉N₁₀O₄ ⁺ [M+H]⁺: 651.32; found, 651.4.

### Example 368: Preparation of 3-(6-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-3-yl)piperidine-2,6-dione (GT-07483)

According to the method of step 2 of Scheme I-1, the target compound (GT-07483) was prepared (yellow solid, 46 mg, yield 75.3 %). ¹H NMR (400 MHz, DMSO-d6) δ 11.00 (s, 1H), 10.90 (s, 1H), 8.97 (d, *J* = 16.1 Hz, 1H), 8.53 (d, *J* = 2.0 Hz, 1H), 8.05 - 7.96 (m, 1H), 7.86 - 7.76 (m, 3H), 7.63 (d, J = 8.0 Hz, 1H), 5.78 (p, *J* = 8.9 Hz, 1H), 4.50 (s, 2H), 3.51 - 3.46 (m, 5H), 3.41 - 3.32 (m, 6H), 2.72 - 2.61 (m, 1H), 2.56 - 2.48 (m, 1H), 2.37 (s, 2H), 2.29 (d, *J =* 10.5 Hz, 3H), 2.21 - 2.12 (m, 2H), 2.03 - 1.94 (m, 1H), 1.91 - 1.81 (m, 2H), 1.78 - 1.70 (m, 2H), 1.58 - 1.48 (m, 2H). LCMS (ESI) calcd for C₃₅H₄₀N₉O₄ + [M+H]+: 650.32; found, 650.4.

### Example 369: Preparation of 3-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-2-yl)piperidine-2,6-dione (GT-07484)

According to the method of step 2 of Scheme I-1, the target compound (GT-07484) was prepared (yellow solid, 21 mg, yield 34.4 %). ¹H NMR (400 MHz, DMSO-d6) δ 11.58 (s, 1H), 10.86 (s, 1H), 10.77 (s, 1H), 8.93 (s, 1H), 8.59 (d, *J* = 5.8 Hz, 1H), 8.01 (s, 1H), 7.80 - 7.73 (m, 2H), 7.60 (d, *J* = 4.4 Hz, 2H), 5.77 (p, *J* = 8.8 Hz, 1H), 4.39 (s, 2H), 3.78 (s, 4H), 3.19 (s, 6H), 2.60 - 2.49 (m, 2H), 2.37 (s, 3H), 2.27 (s, 3H), 2.19 - 2.12 (m, 3H), 1.88 - 1.84 (m, 2H), 1.76 - 1.70 (m, 2H), 1.55 - 1.50 (m, 2H). LCMS (ESI) calcd for C₃₅H₄₀N₉O₄ ⁺ [M+H]⁺: 650.32; found, 650.4.

### Example 370: Preparation of 3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-3-yl)piperidine-2,6-dione (GT-07485)

According to the method of step 2 of Scheme I-1, the target compound (GT-07485) was prepared (yellow solid, 13 mg, yield 21.3 %). ¹H NMR (400 MHz, DMSO-d6) δ 11.86 (s, 1H), 10.96 (s, 2H), 8.94 (s, 1H), 8.80 (s, 1H), 8.64 (s, 1H), 8.22 (s, 1H), 8.02 (s, 1H), 7.82 - 7.75 (m, 2H), 5.81 - 5.72 (m, 1H), 4.46 (s, 2H), 4.08 - 4.01 (m, 2H), 3.39 (s, 3H), 3.20 (s, 4H), 2.74 - 2.66 (m, 1H), 2.56 (d, *J* = 17.1 Hz, 1H), 2.37 (s, 3H), 2.27 (s, 4H), 2.20 - 2.06 (m, 4H), 1.87 (s, 2H), 1.76 - 1.70 (m, 2H), 1.55 - 1.50 (m, 2H). LCMS (ESI) calcd for C₃₅H₄₀N₉O₄⁺ [M+H]⁺: 650.32; found, 650.4.

### Example 371: Preparation of 1-(5-fluoro-4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)pyridin-2-yl)dihydropyrimidine-2,4(1H,3H)-dione (GT-07486)

According to the method of step 2 of Scheme I-1, the target compound (GT-07486) was prepared (yellow solid, 19 mg, yield 35.2 %). ¹H NMR (400 MHz, DMSO-d6) δ 11.85 (s, 1H), 10.66 (s, 1H), 8.93 (d, *J* = 3.2 Hz, 1H), 8.53 (s, 1H), 8.37 (s, 1H), 8.29 (d, J = 10.5 Hz, 1H), 8.02 (s, 2H), 7.86 (d, *J* = 10.3 Hz, 2H), 5.04 - 4.90 (m, 1H), 4.09 (t, 4H), 3.92 (d, 3H), 3.67 (d, 2H), 3.53 - 3.48 (m, 1H), 3.34 (s, 3H), 3.14 (s, 2H), 2.90 - 2.74 (m, 7H), 2.31 (d, *J* = 10.1 Hz, 2H), 2.00 - 1.87 (m, 2H), 1.71 (d, *J* = 6.9 Hz, 6H). LCMS (ESI) calcd for C₃₉H₄₄F₃N₁₂O₂⁺ [M+H]⁺: 769.37; found, 769.4.

### Example 372: Preparation of 1-(4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)pyridin-2-yl)dihydropyrimidine-2,4(1H,3H)-dione (GT-07487)

According to the method of step 2 of Scheme I-1, the target compound (GT-07487) was prepared (yellow solid, 18 mg, yield 34.1 %). ¹H NMR (400 MHz, DMSO-d6) δ 12.17 (s, 1H), 11.73 (s, 1H), 10.54 (s, 1H), 8.80 (d, *J* = 3.2 Hz, 1H), 8.42 (d, *J* = 5.0 Hz, 1H), 8.24 (s, 1H), 8.16 (d, *J* = 9.6 Hz, 1H), 7.89 (s, 2H), 7.79 - 7.65 (m, 2H), 7.47 (s, 1H), 4.94 - 4.80 (m, 1H), 4.30 (s, 3H), 4.01 (t, *J* = 6.5 Hz, 3H), 3.54 - 3.27 (m, 11H), 2.79 - 2.59 (m, 7H), 2.17 (d, *J* = 9.3 Hz, 2H), 1.80 (d, *J* = 9.5 Hz, 2H), 1.58 (d, *J* = 6.9 Hz, 6H). LCMS (ESI) calcd for C₃₉H₄₅F₂N₁₂O₂⁺ [M+H]⁺: 751.38; found, 751.4.

### Example 373: Preparation of 1-(5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)pyridin-3-yl)dihydropyrimidine-2,4(1H,3H)-dione (GT-07488)

According to the method of step 2 of Scheme I-1, the target compound (GT-07488) was prepared (yellow solid, 15 mg, yield 28.5 %). ¹H NMR (400 MHz, DMSO-d6) δ 12.39 (s, 1H), 11.82 (s, 1H), 10.31 - 9.98 (m, 1H), 9.79 (t, *J* = 35.5 Hz, 1H), 8.88 (d, *J* = 3.1 Hz, 1H), 8.79 - 8.70 (m, 1H), 8.32 (s, 1H), 8.24 (d, *J* = 8.5 Hz, 1H), 7.96 (s, 1H), 7.81 (d, *J* = 10.7 Hz, 2H), 4.98 - 4.89 (m, 1H), 4.48 (s, 1H), 4.06 (d, *J* = 6.7 Hz, 4H), 3.72 (s, 4H), 3.55 - 3.45 (m, 6H), 2.87 - 2.80 (m, 3H), 2.77 (s, 4H), 2.26 (d, *J* = 9.6 Hz, 2H), 1.93 - 1.83 (m, 2H), 1.65 (d, *J* = 6.9 Hz, 6H). LCMS (ESI) calcd for C₃₉H₄₅F₂N₁₂O₂⁺ [M+H]⁺: 751.38; found, 751.4.

### Example 374: Preparation of 7-cyclopentyl-2-((5-(4-((5-(2,6-dioxopiperidin-3-yl)-2-fluoropyridin-3-yl)methyl)piperazin-1-yl)pyridin-2-yl)amino)-N,N-dimethyl-7H-pyrrolo[2,3-d]pyrimidine-6-carboxamide (GT-07563)

According to the method of step 2 of Scheme I-1, the target compound (GT-07563) was prepared (yellow solid, 6 mg, yield 9.4 %). ¹H NMR (400 MHz, DMSO-d6) δ 12.24 (s, 1H), 11.74 (s, 1H), 10.95 (s, 1H), 9.04 (s, 1H), 8.39 (d, *J* = 7.8 Hz, 1H), 8.25 (s, 1H), 8.11 (d, *J* = 9.3 Hz, 1H), 8.01 (s, 1H), 7.69 (d, *J =* 9.4 Hz, 1H), 6.85 (s, 1H), 4.84 - 4.76 (m, 1H), 4.47 (t, *J =* 16.7 Hz, 2H), 4.08 (dd, *J =* 12.4, 4.7 Hz, 2H), 3.85 (s, 2H), 3.54 - 3.27 (m, 6H), 3.06 (s, 6H), 2.83 - 2.71 (m, 1H), 2.62 (d, *J* = 17.2 Hz, 1H), 2.37 - 2.23 (m, 3H), 2.16 - 2.09 (m, 1H), 2.08 - 1.94 (m, 4H), 1.71 - 1.57 (m, 2H). LCMS (ESI) calcd for C₃₄H₄₀FN₁₀O₃⁺ [M+H]⁺: 655.33; found, 655.4.

### Example 375: Preparation of 3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-6-fluoropyridin-3-yl)piperidine-2,6-dione (GT-07591)

According to the method of step 2 of Scheme I-1, the target compound (GT-07591) was prepared (white solid, 24 mg, yield 41.1 %). ¹H NMR (400 MHz, DMSO-d6) δ 11.75 (s, 1H), 11.04 (s, 1H), 10.93 (s, 1H), 8.96 - 8.94 (m, 1H), 8.25 (d, *J =* 9.1 Hz, 1H), 8.19 (s, 1H), 8.01 (d, *J =* 2.8 Hz, 1H), 7.87 - 7.77 (m, 3H), 4.40 (s, 2H), 4.00 (dd, *J =* 12.5, 4.8 Hz, 2H), 3.39 (s, 2H), 3.24 - 3.17 (m, 4H), 2.74 - 2.64 (m, 1H), 2.55 (d, *J* = 17.1 Hz, 1H), 2.37 (s, 3H), 2.28 (s, 3H), 2.18 - 2.15 (m, 2H), 2.07 - 2.03 (m, 1H), 1.87 (s, 2H), 1.75 - 1.71 (m, 2H), 1.55 - 1.51 (m, 2H). LCMS (ESI) calcd for C₃₅H₃₉FN₉O₄ ⁺ [M+H]⁺: 668.31; found, 688.4.

### Example 376: Preparation of 1-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyrazin-2-yl)dihydropyrimidine-2,4(1H,3H)-dione (GT-07827)

According to the method of step 2 of Scheme I-1, the target compound (GT-07827) was prepared (white solid, 28 mg, yield 49.6 %). ¹H NMR (400 MHz, DMSO-d6) δ 11.60 (s, 1H), 10.97 (s, 1H), 10.83 (s, 1H), 9.17 (s, 1H), 9.03 (s, 1H), 8.79 (s, 1H), 8.09 (s, 1H), 7.85 (s, 2H), 5.89 - 5.81 (m, 1H), 4.59 (s, 2H), 4.10 (t, *J =* 6.6 Hz, 2H), 3.87 (s, 3H), 3.61 - 3.52 (m, 4H), 3.30 (s, 4H), 2.75 (t, *J =* 6.6 Hz, 2H), 2.44 (s, 2H), 2.35 (s, 2H), 2.26 - 2.22 (m, 1H), 1.94 (s, 2H), 1.86 - 1.76 (m, 2H), 1.65 - 1.56 (m, 2H). LCMS (ESI) calcd for C₃₃H₃₈N₁₁O₄⁺ [M+H]⁺: 652.31; found, 652.2.

### Example 377: Preparation of 7-cyclopentyl-2-((5-(4-((5-(2,4-dioxotetrahydropyrimidin-1(2H)-yl)-2-fluoropyridin-3-yl)methyl)piperazin-1-yl)pyridin-2-yl)amino)-N,N-dimethyl-7H-pyrrolo[2,3-d]pyrimidine-6-carboxamide (GT-07830)

According to the method of step 2 of Scheme I-1, the target compound (GT-07830) was prepared (white solid, 27 mg, yield 42.4 %). ¹H NMR (400 MHz, DMSO-d6) δ 12.01 (s, 1H), 11.48 (s, 1H), 10.63 (s, 1H), 9.01 (s, 1H), 8.41 (d, *J* = 10.7 Hz, 2H), 8.05 (d, *J* = 9.5 Hz, 1H), 7.98 (d, *J* = 2.6 Hz, 1H), 7.66 (d, *J =* 9.4 Hz, 1H), 6.84 (s, 1H), 4.86 - 4.74 (m, 1H), 4.47 (s, 2H), 3.94 (t, *J =* 6.6 Hz, 2H), 3.83 (s, 1H), 3.36 - 3.28 (m, 5H), 3.06 (s, 6H), 2.77 (t, *J =* 6.6 Hz, 2H), 2.36 - 2.26 (m, 2H), 2.09 - 1.94 (m, 4H), 1.71 - 1.61 (m, 2H). LCMS (ESI) calcd for C₃₃H₃₉FN₁₁O₃ ⁺ [M+H]⁺: 656.32; found, 656.3.

### Example 378: Preparation of 7-cyclopentyl-2-((5-(4-((5-(2,4-dioxotetrahydropyrimidin-1(2H)-yl)pyrazin-2-yl)methyl)piperazin-1-yl)pyridin-2-yl)amino)-N,N-dimethyl-7H-pyrrolo[2,3-d]pyrimidine-6-carboxamide (GT-07831)

According to the method of step 2 of Scheme I-1, the target compound (GT-07831) was prepared (white solid, 21 mg, yield 33.8 %). ¹H NMR (400 MHz, DMSO-d6) δ 11.91 (s, 1H), 11.51 (s, 1H), 10.83 (s, 1H), 9.16 (d, *J =* 1.4 Hz, 1H), 9.02 (d, *J =* 2.6 Hz, 1H), 8.83 (d, *J =* 1.3 Hz, 1H), 8.08 - 8.03 (m, 1H), 7.99 (d, *J* = 2.7 Hz, 1H), 7.67 (d, *J =* 9.4 Hz, 1H), 6.84 (s, 1H), 4.87 - 4.76 (m, 1H), 4.58 (s, 2H), 4.10 (t, *J =* 6.6 Hz, 2H), 3.83 (s, 1H), 3.62 - 3.57 (m, 2H), 3.47 - 3.43 (m, 3H), 3.29 - 3.25 (m, 2H), 3.06 (s, 6H), 2.75 (t, *J =* 6.6 Hz, 2H), 2.36 - 2.29 (m, 2H), 2.05 - 1.96 (m, 4H), 1.68 - 1.61 (m, 2H). LCMS (ESI) calcd for C₃₂H₃₉N₁₂O₃⁺ [M+H]⁺: 639.33; found, 639.2.

### Example 379: Preparation of 1-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-6-fluoropyridin-3-yl)dihydropyrimidine-2,4(1H,3H)-dione (GT-07832)

According to the method of step 2 of Scheme I-1, the target compound (GT-07832) was prepared (white solid, 40 mg, yield 69.2 %). ¹H NMR (400 MHz, DMSO-d6) δ 11.67 (s, 1H), 10.97 (s, 1H), 10.63 (s, 1H), 9.01 (s, 1H), 8.44 - 8.33 (m, 2H), 8.08 (s, 1H), 7.92 - 7.80 (m, 2H), 5.93 - 5.78 (m, 1H), 4.48 (s, 2H), 3.96 - 3.84 (m, 4H), 3.33 (s, 5H), 2.77 (t, *J* = 6.6 Hz, 2H), 2.44 (s, 3H), 2.35 (s, 3H), 2.30 - 2.19 (m, 2H), 1.99 - 1.89 (m, 2H), 1.87 - 1.76 (m, 2H), 1.67 - 1.53 (m, 2H). LCMS (ESI) calcd for C₃₄H₃₈FN₁₀O₄⁺ [M+H]⁺: 669.31; found, 669.2.

### Example 380: Preparation of 1-(4-((4-(4-((9-cyclopentyl-8-(phenylamino)-9H-purin-2-yl)amino)phenyl)piperazin-1-yl)methyl)phenyl)dihydropyrimidine-2,4(1H,3H)-dione (GT-05837)

According to the method of step 2 of Scheme I-1, the target compound (GT-05837) was prepared (white solid, 10 mg, yield 17.30%). ¹H NMR (400 MHz, DMSO-d6) δ 11.13 (s, 1H), 10.45 (s, 1H), 10.01 (d, J = 58.6 Hz, 2H), 8.40 (s, 1H), 7.81 (d, J = 7.6 Hz, 2H), 7.67 (d, J = 7.8 Hz, 2H), 7.43 (dt, J = 15.0, 8.3 Hz, 6H), 7.13 (t, J = 7.2 Hz, 1H), 7.02 (d, J = 8.5 Hz, 2H), 5.19 - 5.03 (m, 1H), 4.51 - 4.36 (m, 2H), 3.81 (dd, J = 15.9, 7.1 Hz, 6H), 3.17 (s, 4H), 2.73 (t, J = 6.2 Hz, 2H), 2.30 (s, 2H), 2.08 (s, 2H), 1.90 (s, 2H), 1.62 (s, 2H). LCMS (ESI) calcd for C₃₇H₄₁N₁₀O₂⁺ [M+H]⁺: 657.34; found, 657.3.

### Example 381: Preparation of 1-(3-((4-(4-((9-cyclopentyl-8-(phenylamino)-9H-purin-2-yl)amino)phenyl)piperazin-1-yl)methyl)phenyl)dihydropyrimidine-2,4(1H,3H)-dione (GT-05838)

According to the method of step 2 of Scheme I-1, the target compound (GT-05838) was prepared (white solid, 15 mg, yield 25.96%). ¹H NMR (400 MHz, DMSO-d6) δ 11.38 (s, 1H), 10.46 (s, 1H), 10.27 (s, 2H), 8.42 (s, 1H), 7.83 (d, J = 8.0 Hz, 2H), 7.66 (s, 1H), 7.54 - 7.37 (m, 8H), 7.14 (t, J = 7.1 Hz, 1H), 7.03 (d, J = 8.3 Hz, 2H), 5.25 - 5.07 (m, 1H), 4.41 (s, 2H), 3.92 - 3.82 (m, 6H), 3.42 (s, 2H), 3.18 (s, 2H), 2.74 (t, J = 6.4 Hz, 2H), 2.28 (s, 2H), 2.08 (s, 2H), 1.89 (s, 2H), 1.61 (s, 2H). LCMS (ESI) calcd for C₃₇H₄₁N₁₀O₂⁺ [M+H]⁺: 657.34; found, 657.4.

### Example 382: Preparation of 1-(6-((4-(4-((9-cyclopentyl-8-(phenylamino)-9H-purin-2-yl)amino)phenyl)piperazin-1-yl)methyl)pyridin-3-yl)dihydropyrimidine-2,4(1H,3H)-dione (GT-05839)

According to the method of step 2 of Scheme I-1, the target compound (GT-05839) was prepared (white solid, 10 mg, yield 17.30%). ¹H NMR (400 MHz, DMSO-d6) δ 10.60 (s, 1H), 10.29 (s, 2H), 8.72 (d, J = 2.4 Hz, 1H), 8.42 (s, 1H), 7.93 (dd, J = 8.4, 2.5 Hz, 1H), 7.83 (d, J = 7.8 Hz, 2H), 7.76 (d, J = 8.4 Hz, 1H), 7.51 - 7.39 (m, 4H), 7.14 (t, J = 7.4 Hz, 1H), 7.04 (d, J = 9.0 Hz, 2H), 5.33 - 5.11 (m, 1H), 4.56 (s, 2H), 3.91 (d, J = 6.6 Hz, 2H), 3.42 - 3.34 (m, 8H), 2.76 (t, J = 6.6 Hz, 2H), 2.27 (d, J = 7.6 Hz, 2H), 2.07 (s, 2H), 1.88 (s, 2H), 1.61 (d, J = 5.3 Hz, 2H). LCMS (ESI) calcd for C₃₆H₄₀N₁₁O₂⁺ [M+H]⁺: 658.34; found, 658.4.

### Example 383: Preparation of 1-(5-((4-(4-((9-cyclopentyl-8-(phenylamino)-9H-purin-2-yl)amino)phenyl)piperazin-1-yl)methyl)pyridin-2-yl)dihydropyrimidine-2,4(1H,3H)-dione (GT-05840)

According to the method of step 2 of Scheme I-1, the target compound (GT-05840) was prepared (white solid, 14 mg, yield 24.19%). ¹H NMR (400 MHz, DMSO-d6) δ 11.04 (s, 1H), 10.63 (s, 1H), 9.71 (d, J = 40.3 Hz, 2H), 8.61 (s, 1H), 8.38 (s, 1H), 8.08 (d, J = 8.9 Hz, 1H), 7.89 (d, J = 8.5 Hz, 1H), 7.80 (d, J = 7.3 Hz, 2H), 7.53 (d, J = 7.9 Hz, 2H), 7.39 (t, J = 7.4 Hz, 2H), 7.10 (d, J = 7.0 Hz, 1H), 7.00 (d, J = 8.0 Hz, 2H), 5.05 (d, J = 8.0 Hz, 1H), 4.43 (s, 2H), 4.10 (d, J = 5.8 Hz, 2H), 3.77 (d, J = 10.3 Hz, 2H), 3.11 (d, J = 12.6 Hz, 6H), 2.70 (d, J = 5.7 Hz, 2H), 2.33 (s, 2H), 2.06 (s, 2H), 1.94 (s, 2H), 1.64 (s, 2H). LCMS (ESI) calcd for C₃₆H₄₀N₁₁O₂⁺ [M+H]⁺: 658.34; found, 658.4.

### Example 384: Preparation of 3-((3-((4-(4-((9-cyclopentyl-8-(phenylamino)-9H-purin-2-yl)amino)phenyl)piperazin-1-yl)methyl)phenyl)amino)piperidine-2,6-dione (GT-05841)

According to the method of step 2 of Scheme I-1, the target compound (GT-05841) was prepared (white solid, 30 mg, yield 50.82%). ¹H NMR (400 MHz, DMSO-d6) δ 10.85 (s, 1H), 10.56 (s, 1H), 9.65 (s, 2H), 8.37 (s, 1H), 7.80 (d, J = 7.6 Hz, 2H), 7.54 (d, J = 7.7 Hz, 2H), 7.38 (t, J = 7.5 Hz, 2H), 7.18 (t, J = 7.8 Hz, 1H), 7.08 (s, 1H), 6.98 (d, J = 7.9 Hz, 2H), 6.93 (s, 1H), 6.79 (s, 2H), 5.03 (d, J = 7.8 Hz, 1H), 4.38 (d, J = 7.8 Hz, 1H), 4.26 (s, 2H), 3.75 (d, J = 12.2 Hz, 2H), 3.11 (d, J = 12.7 Hz, 4H), 2.78 (d, J = 13.1 Hz, 1H), 2.61 (d, J = 17.0 Hz, 2H), 2.36 (s, 2H), 2.17 - 1.93 (m, 6H), 1.65 (s, 2H). LCMS (ESI) calcd for C₃₈H₄₃N₁₀O₂⁺ [M+H]⁺: 671.36; found, 671.4.

### Example 385: Preparation of 1-(4-(((1-(4-((9-cyclopentyl-8-(phenylamino)-9H-purin-2-yl)amino)phenyl)piperidin-4-yl)(methyl)amino)methyl)phenyl)dihydropyrimidine-2,4(1H,3H)-dione (GT-07092)

According to the method of step 2 of Scheme I-1, the target compound (GT-07092) was prepared (white solid, 19 mg, yield 33.47%). ¹H NMR (500 MHz, DMSO) δ 10.46 (s, 1H), 10.20 (s, 2H), 8.45 (s, 1H), 7.83 (t, J = 9.7 Hz, 2H), 7.73 (dd, J = 12.4, 8.2 Hz, 2H), 7.64 (dd, J = 25.1, 19.0 Hz, 2H), 7.49 - 7.37 (m, 5H), 7.14 (t, J = 7.3 Hz, 1H), 5.19 (p, J = 8.4 Hz, 1H), 4.48 (d, J = 8.3 Hz, 1H), 4.27 (dd, J = 12.8, 6.7 Hz, 1H), 3.83 (dd, J = 19.2, 12.5 Hz, 5H), 3.23 (s, 2H), 2.73 (dd, J = 13.0, 6.5 Hz, 2H), 2.64 (d, J = 4.6 Hz, 3H), 2.36 (t, J = 30.2 Hz, 6H), 2.11 (s, 2H), 1.93 (d, J = 19.3 Hz, 2H), 1.74 - 1.62 (m, 2H). LCMS (ESI) calcd for C₃₉H₄₅N₁₀O₂⁺ [M+H]⁺: 685.37; found, 685.4.

### Example 386: Preparation of 1-(3-(((1-(4-((9-cyclopentyl-8-(phenylamino)-9H-purin-2-yl)amino)phenyl)piperidin-4-yl)(methyl)amino)methyl)phenyl)dihydropyrimidine-2,4(1H,3H)-dione (GT-05870)

According to the method of step 2 of Scheme I-1, the target compound (GT-05870) was prepared (white solid, 6 mg, yield 10.57%). ¹H NMR (400 MHz, DMSO-d6) δ 11.09 (s, 1H), 10.46 (s, 1H), 10.20 (s, 2H), 8.45 (s, 1H), 7.81 (d, J = 8.1 Hz, 2H), 7.66 (d, J = 20.0 Hz, 3H), 7.45 (ddd, J = 22.0, 15.7, 7.6 Hz, 7H), 7.14 (t, J = 7.5 Hz, 1H), 5.29 - 5.10 (m, 1H), 4.50 (d, J = 11.0 Hz, 1H), 4.29 (s, 1H), 3.88 (t, J = 6.6 Hz, 2H), 3.78 (s, 2H), 3.26 - 3.10 (m, 4H), 2.74 (t, J = 6.5 Hz, 2H), 2.64 (d, J = 3.6 Hz, 3H), 2.34 (s, 5H), 2.11 (s, 2H), 1.95 (s, 2H), 1.66 (s, 2H). LCMS (ESI) calcd for C₃₉H₄₅N₁₀O₂⁺ [M+H]⁺: 685.37; found, 685.4.

### Example 387: Preparation of 1-(6-(((1-(4-((9-cyclopentyl-8-(phenylamino)-9H-purin-2-yl)amino)phenyl)piperidin-4-yl)(methyl)amino)methyl)pyridin-3-yl)dihydropyrimidine-2,4(1H,3H)-dione (GT-07093)

According to the method of step 2 of Scheme I-1, the target compound (GT-07093) was prepared (white solid, 19 mg, yield 33.43%). ¹H NMR (500 MHz, DMSO) δ 11.08 (s, 1H), 10.60 (s, 2H), 10.45 (s, 2H), 8.72 (d, J = 2.5 Hz, 1H), 8.49 (s, 1H), 7.94 (dd, J = 8.4, 2.5 Hz, 1H), 7.81 (d, J = 8.2 Hz, 3H), 7.71 (s, 2H), 7.64 (s, 2H), 7.43 (t, J = 7.9 Hz, 2H), 7.18 (dd, J = 13.9, 6.6 Hz, 1H), 5.38 - 5.22 (m, 1H), 4.55 (s, 2H), 3.91 (s, 2H), 3.77 (d, J = 11.0 Hz, 2H), 3.69 (s, 2H), 3.43 (s, 2H), 2.82 - 2.75 (m, 5H), 2.43 (s, 3H), 2.36 - 2.29 (m, 2H), 2.13 (s, 2H), 1.95 (s, 2H), 1.73 - 1.65 (m, 2H). LCMS (ESI) calcd for C₃₈H₄₄N₁₁O₂⁺ [M+H]⁺: 686.37; found, 686.4.

### Example 388: Preparation of 1-(5-(((1-(4-((9-cyclopentyl-8-(phenylamino)-9H-purin-2-yl)amino)phenyl)piperidin-4-yl)(methyl)amino)methyl)pyridin-2-yl)dihydropyrimidine-2,4(1H,3H)-dione (GT-05871)

According to the method of step 2 of Scheme I-1, the target compound (GT-05871) was prepared (white solid, 6 mg, yield 10.56 %). ¹H NMR (400 MHz, DMSO-d6) δ 11.35 (s, 1H), 10.62 (s, 1H), 10.26 (s, 1H), 8.67 (s, 1H), 8.45 (s, 1H), 8.17 (d, J = 8.2 Hz, 1H), 7.87 (d, J = 8.1 Hz, 1H), 7.77 (d, J = 7.7 Hz, 2H), 7.68 (d, J = 7.9 Hz, 2H), 7.55 (s, 2H), 7.41 (dd, J = 15.8, 8.3 Hz, 2H), 7.16 (dd, J = 16.2, 9.1 Hz, 1H), 5.28 - 5.08 (m, 1H), 4.42 (d, J = 53.0 Hz, 2H), 4.11 (s, 2H), 3.77 (s, 6H), 2.68 (d, J = 21.5 Hz, 5H), 2.37 (s, 5H), 2.11 (s, 2H), 1.94 (s, 2H), 1.66 (s, 2H). LCMS (ESI) calcd for C₃₈H₄₄N₁₁O₂⁺ [M+H]⁺: 686.37; found, 686.4.

### Example 389: Preparation of 3-((3-(((1-(4-((9-cyclopentyl-8-(phenylamino)-9H-purin-2-yl)amino)phenyl)piperidin-4-yl)(methyl)amino)methyl)phenyl)amino)piperidine-2,6-dione (GT-07094)

According to the method of step 2 of Scheme I-1, the target compound (GT-07094) was prepared (white solid, 10 mg, yield 17.30%). ¹H NMR (400 MHz, DMSO-d6) δ 11.07 (s, 1H), 10.84 (s, 1H), 10.43 (s, 1H), 8.47 (s, 1H), 7.80 - 7.67 (m, 5H), 7.43 (t, J = 7.7 Hz, 2H), 7.18 (dd, J = 18.1, 7.7 Hz, 2H), 7.09 (s, 1H), 6.94 - 6.74 (m, 2H), 5.37 - 5.17 (m, 1H), 4.43 (dd, J = 11.3, 4.4 Hz, 1H), 4.31 (s, 1H), 4.17 (s, 2H), 3.60 - 3.43 (m, 6H), 2.90 - 2.77 (m, 1H), 2.64 (d, J = 14.5 Hz, 3H), 2.56 (d, J = 13.3 Hz, 2H), 2.33 (s, 3H), 2.12 (s, 3H), 1.99 - 1.84 (m, 3H), 1.66 (s, 2H). LCMS (ESI) calcd for C₄₀H₄₇N₁₀O₂⁺ [M+H]⁺: 699.39; found, 699.4.

### Example 390: Preparation of 3-(4-((4-(1-(4-((9-cyclopentyl-8-(phenylamino)-9H-purin-2-yl)amino)phenyl)piperidin-4-yl)piperazin-1-yl)methyl)phenyl)piperidine-2,6-dione (GT-05915)

According to the method of step 2 of Scheme I-1, the target compound (GT-05915) was prepared (white solid, 20 mg, yield 36.38%). ¹H NMR (400 MHz, DMSO-d6) δ 10.88 (s, 1H), 10.30 (s, 1H), 8.44 (s, 1H), 7.78 (d, J = 7.8 Hz, 2H), 7.69 - 7.61 (m, 4H), 7.50 (d, J = 8.2 Hz, 2H), 7.43 (t, J = 7.9 Hz, 2H), 7.34 (d, J = 8.1 Hz, 2H), 7.18 (t, J = 7.4 Hz, 1H), 5.25 - 5.15 (m, 1H), 4.45 (s, 2H), 3.96 - 3.86 (m, 8H), 3.30 (s, 4H), 2.76 - 2.64 (m, 2H), 2.54 (d, J = 2.8 Hz, 1H), 2.33 (s, 4H), 2.27 - 2.17 (m, 3H), 2.07 (dd, J = 18.9, 14.3 Hz, 4H), 1.94 (s, 2H), 1.65 (d, J = 5.2 Hz, 2H). LCMS (ESI) calcd for C₄₃H₅₁N₁₀O₂⁺ [M+H]⁺: 739.42; found, 739.5.

### Example 391: Preparation of 1-(4-((4-(I-(4-((9-cyclopentyl-8-(phenylamino)-9H-purin-2-yl)amino)phenyl)piperidin-4-yl)piperazin-1-yl)methyl)phenyl)dihydropyrimidine-2,4(1H,3H)-dione (GT-05917)

According to the method of step 2 of Scheme I-1, the target compound (GT-05917) was prepared (white solid, 10 mg, yield 18.17%). ¹H NMR (400 MHz, DMSO-d6) δ 10.44 (s, 1H), 10.09 (s, 2H), 8.42 (s, 1H), 7.80 (d, J = 7.9 Hz, 2H), 7.67 (d, J = 8.0 Hz, 2H), 7.59 (s, 2H), 7.42 (dd, J = 14.6, 7.2 Hz, 4H), 7.32 (s, 2H), 7.14 (d, J = 6.3 Hz, 1H), 5.21 - 5.04 (m, 1H), 4.40 (s, 2H), 3.82 (d, J = 5.7 Hz, 8H), 3.24 - 2.94 (m, 6H), 2.73 (t, J = 6.1 Hz, 2H), 2.54 (s, 1H), 2.29 (d, J = 34.9 Hz, 4H), 2.01 (d, J = 54.5 Hz, 6H), 1.65 (s, 2H). LCMS (ESI) calcd for C₄₂H₅₀N₁₁O₂⁺ [M+H]⁺: 740.41; found, 740.4.

### Example 392: Preparation of 1-(3-((4-(I-(4-((9-cyclopentyl-8-(phenylamino)-9H-purin-2-yl)amino)phenyl)piperidin-4-yl)piperazin-1-yl)methyl)phenyl)dihydropyrimidine-2,4(1H,3H)-dione (GT-05940)

According to the method of step 2 of Scheme I-1, the target compound (GT-05940) was prepared (white solid, 10 mg, yield 18.17%). ¹H NMR (400 MHz, DMSO-d6) δ 10.46 (s, 1H), 10.11 (s, 2H), 8.42 (s, 1H), 7.80 (d, J = 7.7 Hz, 2H), 7.63 (s, 3H), 7.48 (s, 3H), 7.41 (s, 2H), 7.32 (s, 2H), 7.14 (s, 1H), 5.13 (s, 1H), 4.41 (s, 2H), 3.83 (d, J = 33.3 Hz, 8H), 3.19 - 3.05 (m, 4H), 2.73 (s, 2H), 2.54 (s, 1H), 2.30 (d, J = 31.2 Hz, 4H), 2.01 (d, J = 54.2 Hz, 6H), 1.65 (s, 2H), 1.26 (d, J = 13.7 Hz, 2H). LCMS (ESI) calcd for C₄₂H₅₀N₁₁O₂⁺ [M+H]⁺: 740.41; found, 740.5.

### Example 393: Preparation of 1-(6-((4-(I-(4-((9-cyclopentyl-8-(phenylamino)-9H-purin-2-yl)amino)phenyl)piperidin-4-yl)piperazin-1-yl)methyl)pyridin-3-yl)dihydropyrimidine-2,4(1H,3H)-dione (GT-05941)

According to the method of step 2 of Scheme I-1, the target compound (GT-05941) was prepared (white solid, 20 mg, yield 36.28%). ¹H NMR (400 MHz, DMSO-d6) δ 10.60 (s, 1H), 10.10 (s, 2H), 8.70 (s, 1H), 8.43 (s, 1H), 7.94 (d, J = 8.0 Hz, 1H), 7.80 (d, J = 7.6 Hz, 2H), 7.73 (d, J = 8.3 Hz, 1H), 7.60 (s, 2H), 7.41 (t, J = 7.4 Hz, 2H), 7.32 (s, 1H), 7.15 (d, J = 7.5 Hz, 1H), 5.13 (s, 1H), 4.49 (s, 2H), 3.90 - 3.78 (m, 8H), 3.24 - 2.97 (m, 6H), 2.76 (s, 2H), 2.53 (s, 1H), 2.30 (d, J = 29.8 Hz, 4H), 2.09 (s, 3H), 1.94 (s, 2H), 1.66 (s, 2H), 1.26 (d, J = 7.5 Hz, 1H). LCMS (ESI) calcd for C₄₁H₄₉Ni₂O₂⁺ [M+H]⁺: 741.41; found, 741.5.

### Example 394: Preparation of 1-(5-((4-(1-(4-((9-cyclopentyl-8-(phenylamino)-4,9-dihydro-3H-purin-2-yl)amino)phenyl)piperidin-4-yl)piperazin-1-yl)methyl)pyridin-2-yl)dihydropyrimidine-2,4(1H,3H)-dione (GT-05942)

According to the method of step 2 of Scheme I-1, the target compound (GT-05942) was prepared (white solid, 15 mg, yield 27.21%). ¹H NMR (400 MHz, DMSO-d6) δ 10.62 (s, 1H), 10.34 (s, 2H), 8.67 (s, 1H), 8.46 (s, 1H), 8.13 (d, J = 8.8 Hz, 1H), 7.87 (d, J = 8.4 Hz, 1H), 7.68 (d, J = 7.2 Hz, 2H), 7.52 (s, 2H), 7.43 (t, J = 7.5 Hz, 2H), 7.18 (t, J = 7.7 Hz, 1H), 5.31 - 5.16 (m, 1H), 4.49 (s, 2H), 4.14 - 3.97 (m, 8H), 3.31 (s, 6H), 2.70 (d, J = 6.3 Hz, 2H), 2.55 (s, 1H), 2.29 (d, J = 38.1 Hz, 6H), 2.12 (s, 2H), 1.94 (s, 2H), 1.66 (s, 2H). LCMS (ESI) calcd for C₄₁H₄₉N₁₂O₂⁺ [M+H]⁺: 741.41; found, 741.4.

### Example 395: Preparation of 3-((3-((4-(I-(4-((9-cyclopentyl-8-(phenylamino)-9H-purin-2-yl)amino)phenyl)piperidin-4-yl)piperazin-1-yl)methyl)phenyl)amino)piperidine-2,6-dione (GT-05943)

According to the method of step 2 of Scheme I-1, the target compound (GT-05943) was prepared (white solid, 10 mg, yield 17.30%). ¹H NMR (400 MHz, DMSO-d6) δ 10.86 (s, 1H), 10.32 (s, 1H), 8.46 (s, 1H), 7.79 (d, J = 7.8 Hz, 2H), 7.65 (s, 2H), 7.44 (dd, J = 17.4, 9.9 Hz, 4H), 7.17 (t, J = 7.5 Hz, 2H), 7.04 (s, 1H), 6.89 - 6.78 (m, 2H), 5.29 - 5.17 (m, 1H), 4.46 - 4.29 (m, 4H), 4.00 (s, 2H), 3.28 (s, 8H), 2.81 (d, J = 12.2 Hz, 1H), 2.64 - 2.53 (m, 2H), 2.33 (s, 4H), 2.15 (d, J = 29.9 Hz, 5H), 1.93 (d, J = 10.6 Hz, 3H), 1.66 (s, 2H). LCMS (ESI) calcd for C₄₃H₅₂N₁₁O₂⁺ [M+H]⁺: 754.43; found, 754.5.

### Example 396: Preparation of 1-(5-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)pyridin-2-yl)dihydropyrimidine-2,4(1H,3H)-dione (GT-05947)

According to the method of step 2 of Scheme I-1, the target compound (GT-05947) was prepared (white solid, 20 mg, yield 37.31%). ¹H NMR (400 MHz, DMSO-d6) δ 13.13 (s, 1H), 11.43 (s, 1H), 10.63 (s, 1H), 9.10 (s, 1H), 8.92 (d, J = 2.5 Hz, 2H), 8.82 (s, 1H), 8.67 (s, 1H), 8.40 (s, 1H), 8.15 (d, J = 8.4 Hz, 1H), 7.99 (d, J = 8.9 Hz, 1H), 7.88 (d, J = 8.6 Hz, 1H), 7.43 (s, 1H), 6.75 (s, 1H), 4.46 (s, 2H), 4.11 (t, J = 6.2 Hz, 2H), 3.82 (s, 3H), 3.41 (d, J = 10.2 Hz, 2H), 3.22 (t, J = 12.3 Hz, 6H), 2.72 (t, J = 5.9 Hz, 2H), 2.15 - 2.00 (m, 9H). LCMS (ESI) calcd for C₃₆H₄₀BrN₁₁O₄P⁺ [M+H]⁺: 800.22; found, 800.2.

### Example 397: Preparation of 1-(4-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)phenyl)dihydropyrimidine-2,4(1H,3H)-dione (GT-05951)

According to the method of step 2 of Scheme I-1, the target compound (GT-05951) was prepared (white solid, 28 mg, yield 42.32%). ¹H NMR (400 MHz, DMSO-d6) δ 10.71 (s, 1H), 10.45 (s, 1H), 9.93 (s, 1H), 9.32 (s, 1H), 9.06 (s, 1H), 8.46 (s, 1H), 8.35 (s, 1H), 8.23 (d, J = 9.5 Hz, 1H), 8.12 (d, J = 9.0 Hz, 1H), 7.69 (d, J = 8.2 Hz, 2H), 7.45 (d, J = 8.2 Hz, 2H), 7.38 (s, 1H), 6.75 (s, 1H), 4.49 (d, J = 10.2 Hz, 1H), 4.24 (dd, J = 13.3, 6.9 Hz, 1H), 3.88 - 3.80 (m, 5H), 3.37 - 3.28 (m, 3H), 3.11 (s, 2H), 2.73 (t, J = 6.6 Hz, 3H), 2.65 (d, J = 4.4 Hz, 3H), 2.22 (d, J = 15.6 Hz, 2H), 1.98 (s, 2H), 1.84 (d, J = 13.7 Hz, 6H), 1.72 (s, 2H). LCMS (ESI) calcd for C₃₉H₄₅BrN₁₀O₄P⁺ [M+H]⁺: 827.25; found, 827.3.

### Example 398: Preparation of 1-(6-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)pyridin-3-yl)dihydropyrimidine-2,4(1H,3H)-dione (GT-05952)

According to the method of step 2 of Scheme I-1, the target compound (GT-05952) was prepared (white solid, 25 mg, yield 37.74%). ¹H NMR (400 MHz, DMSO-d6) δ 10.81 (s, 1H), 10.60 (s, 1H), 10.24 (s, 1H), 9.52 (s, 1H), 9.35 (s, 1H), 8.72 (s, 1H), 8.55 (s, 1H), 8.34 (s, 1H), 8.17 (dd, J = 22.3, 8.8 Hz, 2H), 7.93 (d, J = 8.2 Hz, 1H), 7.79 (d, J = 8.4 Hz, 1H), 7.35 (s, 1H), 6.80 (s, 1H), 4.53 (s, 2H), 3.89 (d, J = 6.8 Hz, 7H), 3.42 (s, 1H), 3.14 (s, 2H), 2.84 - 2.73 (m, 6H), 2.24 (s, 2H), 2.05 (s, 2H), 1.85 (d, J = 13.6 Hz, 6H), 1.68 (s, 2H). LCMS (ESI) calcd for C₃₈H₄₄BrN₁₁O₄P⁺ [M+H]⁺: 828.25; found, 828.3.

### Example 399: Preparation of 1-(5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)pyridin-2-yl)dihydropyrimidine-2,4(1H,3H)-dione (GT-05953)

According to the method of step 2 of Scheme I-1, the target compound (GT-05953) was prepared (white solid, 10 mg, yield 15.10%). ¹H NMR (400 MHz, DMSO-d6) δ 11.11 (s, 1H), 10.62 (s, 1H), 10.06 (s, 1H), 9.33 (s, 1H), 8.66 (s, 1H), 8.49 (s, 1H), 8.35 (s, 1H), 8.22 (d, J = 8.1 Hz, 1H), 8.14 (t, J = 9.6 Hz, 2H), 7.88 (d, J = 8.3 Hz, 1H), 7.36 (s, 1H), 6.77 (s, 1H), 4.52 (d, J = 11.9 Hz, 1H), 4.32 (s, 1H), 4.11 (t, J = 5.7 Hz, 2H), 3.82 (s, 3H), 3.35 (s, 3H), 3.13 (s, 2H), 2.80 - 2.65 (m, 6H), 2.27 (s, 2H), 1.99 (s, 2H), 1.85 (d, J = 13.7 Hz, 6H), 1.71 (s, 2H). LCMS (ESI) calcd for C₃₈H₄₄BrN₁₁O₄P⁺ [M+H]⁺: 828.25; found, 828.3.

### Example 400: Preparation of 3-((3-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)phenyl)amino)piperidine-2,6-dione (GT-05954)

According to the method of step 2 of Scheme I-1, the target compound (GT-05954) was prepared (white solid, 22 mg, yield 32.69%). ¹H NMR (400 MHz, DMSO-d6) δ 10.83 (d, J = 19.9 Hz, 2H), 10.20 (s, 1H), 9.45 (s, 1H), 9.34 (s, 1H), 8.53 (s, 1H), 8.35 (s, 1H), 8.17 (dd, J = 23.7, 8.8 Hz, 2H), 7.31 (d, J = 26.4 Hz, 1H), 7.21 - 7.12 (m, 1H), 7.07 (s, 1H), 6.80 (d, J = 6.9 Hz, 2H), 4.51 - 4.31 (m, 2H), 4.14 (s, 1H), 3.83 (s, 3H), 3.30 (s, 2H), 3.13 (s, 3H), 2.77 (s, 2H), 2.66 (s, 3H), 2.58 (s, 1H), 2.23 (d, J = 37.5 Hz, 3H), 2.00 (s, 3H), 1.85 (d, J = 13.6 Hz, 6H), 1.68 (s, 2H). LCMS (ESI) calcd for C₄₀H₄₇BrN₁₀O₄P⁺ [M+H]⁺: 841.27; found, 841.3.

### Example 401: Preparation of 3-(4-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)phenyl)piperidine-2,6-dione (GT-05999)

According to the method of step 2 of Scheme I-1, the target compound (GT-05999) was prepared (white solid, 35 mg, yield 54.03%). ¹H NMR (400 MHz, DMSO-d6) δ 12.10 (s, 1H), 10.88 (s, 1H), 10.04 (s, 1H), 9.32 (s, 1H), 9.26 (s, 1H), 8.48 (s, 1H), 8.35 (s, 1H), 8.21 (d, J = 9.1 Hz, 1H), 8.12 (d, J = 8.8 Hz, 1H), 7.64 (d, J = 7.5 Hz, 2H), 7.34 (d, J = 8.5 Hz, 3H), 6.75 (s, 1H), 4.42 (s, 2H), 3.93 (d, J = 7.0 Hz, 1H), 3.78 (d, J = 26.1 Hz, 10H), 3.42 - 3.32 (m, 3H), 3.13 (s, 2H), 2.83 - 2.65 (m, 3H), 2.53 (s, 1H), 2.28 - 2.11 (m, 3H), 2.05 (s, 1H), 1.84 (d, J = 13.6 Hz, 8H), 1.70 (s, 2H). LCMS (ESI) calcd for C₄₃H₅₁BrN₁₀O₄P⁺ [M+H]⁺: 881.30; found, 881.3.

### Example 402: Preparation of 1-(4-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)phenyl)dihydropyrimidine-2,4(1H,3H)-dione (GT-06000)

According to the method of step 2 of Scheme I-1, the target compound (GT-06000) was prepared (white solid, 35 mg, yield 53.96%). ¹H NMR (400 MHz, DMSO-d6) δ 12.13 (s, 1H), 10.45 (s, 1H), 9.98 (s, 1H), 9.32 (s, 1H), 9.17 (s, 1H), 8.47 (s, 1H), 8.36 (s, 1H), 8.22 (d, J = 8.9 Hz, 1H), 8.13 (s, 1H), 7.70 (d, J = 7.5 Hz, 2H), 7.44 (d, J = 7.5 Hz, 2H), 7.37 (s, 1H), 6.75 (s, 1H), 4.43 (s, 2H), 3.82 (s, 9H), 3.46 - 3.40 (m, 4H), 3.13 (s, 2H), 2.83 - 2.65 (m, 4H), 2.17 (s, 2H), 1.85 (d, J = 13.7 Hz, 8H), 1.71 (s, 2H). LCMS (ESI) calcd for C₄₂H₅₀BrN₁₁O₄P⁺ [M+H]⁺: 882.30; found, 882.3.

### Example 404: Preparation of 1-(6-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)pyridin-3-yl)dihydropyrimidine-2,4(1H,3H)-dione (GT-06003)

According to the method of step 2 of Scheme I-1, the target compound (GT-06003) was prepared (white solid, 35 mg, yield 53.90%). ¹H NMR (400 MHz, DMSO-d6) δ 12.10 (s, 1H), 10.61 (s, 1H), 10.04 (s, 1H), 9.33 (s, 1H), 9.26 (s, 1H), 8.71 (s, 1H), 8.49 (s, 1H), 8.36 (s, 1H), 8.22 (d, J = 9.1 Hz, 1H), 8.12 (d, J = 9.0 Hz, 1H), 7.95 (d, J = 8.1 Hz, 1H), 7.77 (d, J = 8.3 Hz, 1H), 7.37 (s, 1H), 4.54 (s, 2H), 3.88 (dd, J = 21.0, 14.5 Hz, 13H), 3.42 - 3.37 (m, 2H), 3.13 (s, 2H), 2.77 (t, J = 6.1 Hz, 4H), 2.18 (s, 2H), 1.85 (d, J = 13.7 Hz, 8H), 1.70 (s, 2H). LCMS (ESI) calcd for C₄₁H₄₉BrN₁₂O₄P⁺ [M+H]⁺: 883.29; found, 883.3.

### Example 405: Preparation of 1-(5-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)pyridin-2-yl)dihydropyrimidine-2,4(1H,3H)-dione (GT-06004)

According to the method of step 2 of Scheme I-1, the target compound (GT-06004) was prepared (white solid, 45 mg, yield 69.31%). ¹H NMR (400 MHz, DMSO-d6) δ 12.25 - 11.49 (m, 1H), 10.62 (s, 1H), 9.81 (s, 1H), 9.30 (s, 1H), 8.93 (s, 1H), 8.63 (s, 1H), 8.42 (s, 1H), 8.37 (s, 1H), 8.24 (d, J = 9.4 Hz, 1H), 8.14 - 8.05 (m, 2H), 7.86 (d, J = 8.3 Hz, 1H), 7.39 (s, 1H), 6.73 (s, 1H), 4.39 (s, 2H), 4.10 (t, J = 6.4 Hz, 2H), 3.77 (d, J = 38.1 Hz, 8H), 3.57 (s, 5H), 3.12 (s, 2H), 2.72 (d, J = 6.2 Hz, 4H), 2.16 (s, 2H), 1.84 (d, J = 13.7 Hz, 8H), 1.73 (s, 2H). LCMS (ESI) calcd for C₄₁H₄₉BrN₁₂O₄P⁺ [M+H]⁺: 883.29; found, 883.3.

### Example 406: Preparation of 3-((3-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)phenyl)amino)piperidine-2,6-dione (GT-06005)

According to the method of step 2 of Scheme I-1, the target compound (GT-06005) was prepared (white solid, 35 mg, yield 53.12%). ¹H NMR (400 MHz, DMSO-d6) δ 12.15 (s, 1H), 10.86 (s, 1H), 10.11 (s, 1H), 9.34 (s, 2H), 8.50 (s, 1H), 8.35 (s, 1H), 8.21 (d, J = 9.2 Hz, 1H), 8.13 (d, J = 8.9 Hz, 1H), 7.35 (s, 1H), 7.17 (t, J = 7.8 Hz, 1H), 7.05 (s, 1H), 6.90 - 6.73 (m, 3H), 4.48 - 4.29 (m, 3H), 3.82 (s, 6H), 3.50 - 3.32 (m, 6H), 3.14 (s, 2H), 2.88 - 2.70 (m, 3H), 2.60 (d, J = 16.7 Hz, 1H), 2.18 (s, 3H), 1.87 (t, J = 14.6 Hz, 10H), 1.70 (s, 2H). LCMS (ESI) calcd for C₄₃H₅₂BrN₁₁O₄P⁺ [M+H]⁺: 896.31; found, 896.4.

### Example 407: Preparation of 3-(4-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)phenyl)piperidine-2,6-dione (GT-06011)

According to the method of step 2 of Scheme I-1, the target compound (GT-06011) was prepared (white solid, 30 mg, yield 53.50%). ¹H NMR (400 MHz, DMSO-d6) δ 11.56 (d, J = 151.1 Hz, 2H), 10.88 (s, 1H), 9.13 (d, J = 11.1 Hz, 1H), 9.01 (s, 1H), 8.62 (d, J = 10.6 Hz, 1H), 8.48 (dd, J = 36.6, 6.0 Hz, 1H), 8.18 (s, 1H), 7.64 (d, J = 7.3 Hz, 2H), 7.35 (d, J = 7.5 Hz, 2H), 7.27 (s, 1H), 4.83 (d, J = 6.7 Hz, 1H), 4.42 (s, 2H), 4.12 (d, J = 12.0 Hz, 2H), 3.94 (d, J = 6.5 Hz, 2H), 3.29 (s, 7H), 2.76 - 2.64 (m, 1H), 2.54 (s, 1H), 2.25 (d, J = 11.4 Hz, 1H), 2.06 (s, 1H), 1.48 (d, J = 6.0 Hz, 6H), 1.37 (s, 1H), 1.29 (d, J = 6.8 Hz, 1H), 0.60 - 0.51 (m, 1H). LCMS (ESI) calcd for C₃₅H₄₀N₁₁O₄S⁺ [M+H]⁺: 710.30; found, 710.3.

### Example 408: Preparation of 1-(4-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)phenyl)dihydropyrimidine-2,4(1H,3H)-dione (GT-06012)

According to the method of step 2 of Scheme I-1, the target compound (GT-06012) was prepared (white solid, 23 mg, yield 41.14%). ¹H NMR (400 MHz, DMSO-d6) δ 11.51 (s, 2H), 10.45 (s, 1H), 9.19 - 9.01 (m, 2H), 8.70 - 8.41 (m, 3H), 8.18 (s, 1H), 7.72 - 7.67 (m, 2H), 7.45 (d, J = 8.5 Hz, 2H), 7.29 (s, 1H), 4.85 (dd, J = 13.2, 6.6 Hz, 1H), 4.46 (d, J = 25.2 Hz, 2H), 4.13 (d, J = 12.7 Hz, 2H), 3.86 - 3.82 (m, 4H), 3.31 (ddd, J = 12.5, 7.8, 4.7 Hz, 6H), 2.73 (t, J = 6.6 Hz, 2H), 1.48 (d, J = 6.5 Hz, 6H), 1.41 - 1.35 (m, 1H), 1.30 (dd, J = 7.6, 5.0 Hz, 1H), 0.57 (ddt, J = 12.9, 10.2, 3.5 Hz, 2H). LCMS (ESI) calcd for C₃₄H₃₉N₁₂O₄S⁺ [M+H]⁺: 711.29; found, 711.3.

### Example 409: Preparation of 1-(3-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)phenyl)dihydropyrimidine-2,4(1H,3H)-dione (GT-06013)

According to the method of step 2 of Scheme I-1, the target compound (GT-06013) was prepared (white solid, 30 mg, yield 53.67%). ¹H NMR (400 MHz, DMSO-d6) δ 11.52 (s, 2H), 10.47 (s, 1H), 9.16 (s, 1H), 8.63 (s, 2H), 8.45 (d, J = 7.0 Hz, 1H), 8.14 (s, 1H), 7.67 (s, 1H), 7.51 (dd, J = 12.8, 4.8 Hz, 3H), 4.83 (d, J = 6.2 Hz, 1H), 4.45 (s, 2H), 4.15 (s, 2H), 3.88 (d, J = 6.6 Hz, 2H), 3.50 (dd, J = 26.5, 12.8 Hz, 4H), 3.28 (d, J = 10.1 Hz, 2H), 2.74 (t, J = 6.6 Hz, 2H), 1.97 (ddd, J = 9.8, 8.1, 5.1 Hz, 1H), 1.47 (t, J = 10.9 Hz, 6H), 0.64 - 0.53 (m, 4H). LCMS (ESI) calcd for C₃₄H₃₉N₁₂O₄S⁺ [M+H]⁺: 711.29; found, 711.3.

### Example 410: Preparation of 1-(6-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)pyridin-3-yl)dihydropyrimidine-2,4(1H,3H)-dione (GT-06014)

According to the method of step 2 of Scheme I-1, the target compound (GT-06014) was prepared (white solid, 28 mg, yield 50.02%). ¹H NMR (400 MHz, DMSO-d6) δ 9.65 (d, J = 8.5 Hz, 2H), 9.14 (s, 1H), 8.87 (t, J = 38.0 Hz, 3H), 8.14 (s, 2H), 4.86 - 4.80 (m, 1H), 4.49 (d, J = 13.0 Hz, 3H), 3.86 - 3.80 (m, 2H), 3.53 - 3.42 (m, 4H), 3.29 (s, 3H), 2.70 (t, J = 6.6 Hz, 2H), 2.05 - 1.88 (m, 1H), 1.47 (d, J = 6.6 Hz, 6H), 0.66 - 0.50 (m, 4H). LCMS (ESI) calcd for C₃₃H₃₈N₁₃O₄S⁺ [M+H]⁺: 712.29; found, 712.3.

### Example 411: Preparation of 1-(5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)pyridin-2-yl)dihydropyrimidine-2,4(1H,3H)-dione (GT-06015)

According to the method of step 2 of Scheme I-1, the target compound (GT-06015) was prepared (white solid, 30 mg, yield 53.59%). ¹H NMR (400 MHz, DMSO-d6) δ 12.23 - 11.49 (m, 2H), 10.62 (s, 1H), 9.19 (s, 1H), 8.69 - 8.60 (m, 3H), 8.44 (d, J = 7.0 Hz, 1H), 8.14 (dd, J = 8.7, 2.2 Hz, 2H), 7.93 - 7.83 (m, 1H), 7.53 - 7.18 (m, 1H), 4.86 - 4.80 (m, 1H), 4.49 (d, J = 13.0 Hz, 3H), 3.86 - 3.80 (m, 2H), 3.53 - 3.42 (m, 4H), 3.29 (s, 3H), 2.70 (t, J = 6.6 Hz, 2H), 2.05 - 1.88 (m, 1H), 1.47 (d, J = 6.6 Hz, 6H), 0.66 - 0.50 (m, 4H). LCMS (ESI) calcd for C₃₃H₃₈N₁₃O₄S⁺ [M+H]⁺: 712.29; found, 712.3.

### Example 412: Preparation of 3-((3-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)phenyl)amino)piperidine-2,6-dione (GT-06016)

According to the method of step 2 of Scheme I-1, the target compound (GT-06016) was prepared (white solid, 22 mg, yield 38.59%). ¹H NMR (400 MHz, DMSO-d6) δ 11.60 (d, J = 200.4 Hz, 2H), 10.85 (s, 1H), 9.15 (s, 1H), 8.63 (s, 2H), 8.45 (d, J = 7.0 Hz, 1H), 7.99 - 7.84 (m, 1H), 7.71 - 7.37 (m, 2H), 7.27 - 7.05 (m, 2H), 6.82 (t, J = 7.8 Hz, 1H), 4.93 - 4.79 (m, 2H), 4.44 (d, J = 4.8 Hz, 2H), 4.30 (s, 3H), 3.48 (dd, J = 29.3, 12.7 Hz, 4H), 3.24 (d, J = 10.1 Hz, 2H), 2.83 (ddd, J = 17.8, 12.5, 5.4 Hz, 1H), 2.65 - 2.55 (m, 1H), 2.25 - 2.07 (m, 1H), 1.96 (tt, J = 8.0, 5.1 Hz, 1H), 1.48 (d, J = 6.6 Hz, 6H), 0.65 - 0.50 (m, 4H). LCMS (ESI) calcd for C₃₅H₄₁Ni₂O₄S⁺ [M+H]⁺: 725.31; found, 725.3.

### Example 413: Preparation of 3-(4-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)phenyl)piperidine-2,6-dione (GT-06020)

According to the method of step 2 of Scheme I-1, the target compound (GT-06020) was prepared (white solid, 25 mg, yield 45.45%). ¹H NMR (400 MHz, DMSO-d6) δ 11.41 (s, 1H), 10.88 (s, 1H), 10.59 (s, 1H), 9.18 (s, 1H), 8.96 (s, 1H), 8.62 (d, J = 8.7 Hz, 1H), 8.54 (d, J = 6.2 Hz, 1H), 8.12 (s, 1H), 7.62 (d, J = 8.1 Hz, 2H), 7.34 (d, J = 8.1 Hz, 2H), 7.23 (s, 1H), 4.92 - 4.77 (m, 1H), 4.51 (d, J = 11.2 Hz, 1H), 4.21 (d, J = 12.3 Hz, 3H), 3.98 - 3.92 (m, 1H), 3.32 (dd, J = 7.7, 4.5 Hz, 2H), 3.08 - 2.99 (m, 2H), 2.62 (d, J = 4.6 Hz, 3H), 2.40 - 2.18 (m, 4H), 2.11 - 1.90 (m, 4H), 1.48 (d, J = 6.5 Hz, 6H), 1.41 - 1.34 (m, 2H), 1.30 (dd, J = 7.6, 5.1 Hz, 2H). LCMS (ESI) calcd for C₃₇H₄₄N₁₁O₄S⁺ [M+H]⁺: 738.33; found, 738.5.

### Example 414: Preparation of 1-(4-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)phenyl)dihydropyrimidine-2,4(1H,3H)-dione (GT-06021)

According to the method of step 2 of Scheme I-1, the target compound was prepared (GT-06021) (white solid, 26 mg, yield 47.21%). ¹H NMR (400 MHz, DMSO-d6) δ 11.36 (s, 1H), 10.60 (s, 1H), 10.10 (s, 1H), 9.30 (s, 1H), 8.95 (s, 1H), 8.62 (d, J = 8.7 Hz, 1H), 8.32 (d, J = 6.2 Hz, 1H), 8.22 (s, 1H), 7.52 (d, J = 8.1 Hz, 2H), 7.14 (d, J = 8.1 Hz, 2H), 7.23 (s, 1H), 4.92 - 4.77 (m, 1H), 4.51 (d, J = 11.2 Hz, 1H), 4.21 (d, J = 12.3 Hz, 3H), 3.98 - 3.92 (m, 1H), 3.32 (dd, J = 7.7, 4.5 Hz, 2H), 3.08 - 2.99 (m, 2H), 2.62 (d, J = 4.6 Hz, 3H), 2.30 - 2.20 (m, 4H), 2.11 - 1.90 (m, 4H), 1.46 (d, J = 6.5 Hz, 6H), 1.31 - 1.26 (m, 2H), 1.28 (dd, J = 7.6, 5.1 Hz, 2H). LCMS (ESI) calcd for C₃₆H₄₃N₁₂O₄S⁺ [M+H]⁺: 739.33; found, 739.3.

### Example 415: Preparation of 1-(5-(((1-(6-((2-(1-(cyclopropylsulfonyl)-1H-pyrazol-4-yl)pyrimidin-4-yl)amino)-1-ethyl-1H-pyrazolo[4,3-c]pyridin-3-yl)piperidin-4-yl)(methyl)amino)methyl)pyridin-2-yl)dihydropyrimidine-2,4(1H,3H)-dione (GT-06022)

According to the method of step 2 of Scheme I-1, the target compound (GT-06022) was prepared (white solid, 15 mg, yield 27.20%). ¹H NMR (400 MHz, DMSO-d6) δ 12.03 (s, 1H), 11.22 (s, 1H), 10.62 (s, 1H), 9.22 (s, 1H), 8.70 - 8.61 (m, 3H), 8.46 (d, J = 7.0 Hz, 1H), 8.16 (dd, J = 8.7, 2.3 Hz, 1H), 7.87 (d, J = 8.7 Hz, 1H), 4.85 - 4.78 (m, 1H), 4.53 (d, J = 13.0 Hz, 2H), 4.09 - 4.03 (m, 4H), 3.53 (s, 1H), 3.03 (dd, J = 22.1, 11.0 Hz, 2H), 2.71 (t, J = 6.6 Hz, 2H), 2.62 (d, J = 4.8 Hz, 3H), 2.34 (dd, J = 31.8, 11.9 Hz, 2H), 2.08 - 1.93 (m, 3H), 1.49 (d, J = 6.6 Hz, 6H), 0.64 - 0.52 (m, 4H). LCMS (ESI) calcd for C₃₅H₄₂N₁₃O₄S⁺ [M+H]⁺: 740.32; found, 740.4.

### Example 416: Preparation of 3-((3-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)phenyl)amino)piperidine-2,6-dione (GT-06023)

According to the method of step 2 of Scheme I-1, the target compound (GT-06023) was prepared (white solid, 20 mg, yield 35.31%). ¹H NMR (400 MHz, DMSO-d6) δ 11.99 (s, 1H), 10.84 (d, J = 5.1 Hz, 1H), 10.65 (s, 1H), 9.20 (s, 1H), 8.65 (d, J = 16.7 Hz, 2H), 8.45 (d, J = 7.0 Hz, 1H), 8.00 (d, J = 46.5 Hz, 1H), 7.68 - 7.47 (m, 1H), 7.32 (d, J = 50.9 Hz, 2H), 7.20 - 7.00 (m, 2H), 6.80 (t, J = 9.9 Hz, 1H), 5.01 - 4.79 (m, 2H), 4.40 - 4.22 (m, 5H), 3.43 (dd, J = 14.0, 7.0 Hz, 3H), 2.90 (dd, J = 65.2, 8.2 Hz, 3H), 2.62 (d, J = 4.6 Hz, 3H), 2.30 (dd, J = 41.7, 10.8 Hz, 2H), 2.03 - 1.90 (m, 3H), 1.47 (d, J = 6.5 Hz, 6H), 0.64 - 0.51 (m, 4H). LCMS (ESI) calcd for C₃₇H₄₅N₁₂O₄S⁺ [M+H]⁺: 753.34; found, 753.4.

### Example 417: Preparation of 3-(4-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)phenyl)piperidine-2,6-dione (GT-06078)

According to the method of step 2 of Scheme I-1, the target compound (GT-06078) was prepared (white solid, 19 mg, yield 35.45%). ¹H NMR (400 MHz, DMSO-d6) δ 11.83 (s, 1H), 10.87 (s, 1H), 9.16 (s, 1H), 8.58 (s, 2H), 8.44 (d, J = 7.0 Hz, 1H), 7.99 (s, 1H), 7.61 (dd, J = 12.9, 8.2 Hz, 2H), 7.36 (t, J = 6.3 Hz, 2H), 7.16 (d, J = 51.0 Hz, 1H), 4.86 - 4.76 (m, 1H), 4.42 (s, 2H), 4.20 (s, 2H), 3.93 (dd, J = 11.7, 4.9 Hz, 10H), 2.98 (d, J = 12.1 Hz, 2H), 2.72 - 2.60 (m, 1H), 2.26 - 2.12 (m, 4H), 1.97 - 1.85 (m, 3H), 1.46 (d, J = 6.6 Hz, 6H), 1.29 - 1.20 (m, 1H), 0.66 - 0.44 (m, 4H). LCMS (ESI) calcd for C₄₀H₄₉N₁₂O₄S⁺ [M+H]⁺: 793.37; found, 793.4.

### Example 418: Preparation of 1-(4-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)phenyl)dihydropyrimidine-2,4(1H,3H)-dione (GT-06079)

According to the method of step 2 of Scheme I-1, the target compound (GT-06079) was prepared (white solid, 16 mg, yield 29.81%). ¹H NMR (400 MHz, DMSO-d6) δ 11.84 (s, 1H), 10.44 (s, 1H), 9.17 (s, 1H), 8.59 (s, 2H), 8.44 (d, J = 6.9 Hz, 1H), 8.01 (s, 1H), 7.68 (t, J = 7.6 Hz, 2H), 7.43 (d, J = 8.4 Hz, 2H), 4.80 (dd, J = 13.0, 6.5 Hz, 1H), 4.44 (s, 2H), 4.19 (d, J = 12.0 Hz, 2H), 3.84 - 3.77 (m, 10H), 3.00 (t, J = 11.7 Hz, 3H), 2.72 (t, J = 6.6 Hz, 2H), 2.22 (d, J = 9.9 Hz, 2H), 1.97 - 1.80 (m, 3H), 1.46 (d, J = 6.6 Hz, 6H), 0.64 - 0.49 (m, 4H). LCMS (ESI) calcd for C₃₉H₄₈N₁₃O₄S⁺ [M+H]⁺: 794.37; found, 794.4.

### Example 419: Preparation of 1-(3-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)phenyl)dihydropyrimidine-2,4(1H,3H)-dione (GT-06080)

According to the method of step 2 of Scheme I-1, the target compound (GT-06080) was prepared (white solid, 16 mg, yield 29.81%). ¹H NMR (400 MHz, DMSO-d6) δ 11.98 (s, 2H), 10.46 (s, 1H), 9.22 (s, 1H), 8.63 (s, 2H), 8.46 (d, J = 7.0 Hz, 1H), 8.03 (s, 1H), 7.66 (s, 1H), 7.58 - 7.46 (m, 3H), 4.80 (dd, J = 13.0, 6.5 Hz, 2H), 4.46 (s, 2H), 4.22 (s, 3H), 3.88 (s, 3H), 3.66 (s, 4H), 3.03 (d, J = 12.3 Hz, 2H), 2.73 (t, J = 6.6 Hz, 2H), 2.24 (d, J = 9.5 Hz, 2H), 1.96 (ddd, J = 20.9, 13.0, 8.9 Hz, 3H), 1.46 (t, J = 10.0 Hz, 6H), 1.29 (dd, J = 12.7, 6.7 Hz, 2H), 0.64 - 0.53 (m, 4H). LCMS (ESI) calcd for C₃₉H₄₈N₁₃O₄S⁺ [M+H]⁺: 794.37; found, 794.3.

### Example 420: Preparation of 1-(6-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)pyridin-3-yl)dihydropyrimidine-2,4(1H,3H)-dione (GT-06081)

According to the method of step 2 of Scheme I-1, the target compound (GT-06081) was prepared (white solid, 16 mg, yield 29.77%). ¹H NMR (400 MHz, DMSO-d6) δ 11.84 (s, 1H), 10.59 (s, 1H), 9.17 (s, 1H), 8.71 (d, J = 2.5 Hz, 1H), 8.59 (s, 2H), 8.44 (d, J = 7.0 Hz, 1H), 8.00 (s, 1H), 7.94 (dd, J = 8.4, 2.6 Hz, 1H), 7.74 (d, J = 8.4 Hz, 1H), 7.29 (d, J = 50.8 Hz, 1H), 4.84 - 4.72 (m, 1H), 4.54 (s, 2H), 4.19 (d, J = 12.4 Hz, 2H), 3.88 (s, 2H), 3.77 (s, 3H), 3.46 - 3.40 (m, 6H), 3.00 (t, J = 12.0 Hz, 3H), 2.75 (t, J = 6.6 Hz, 2H), 2.24 (d, J = 10.9 Hz, 2H), 1.99 - 1.85 (m, 3H), 1.47 (d, J = 6.6 Hz, 6H), 0.62 - 0.53 (m, 3H). LCMS (ESI) calcd for C₃₈H₄₇N₁₄O₄S⁺ [M+H]⁺: 795.36; found, 795.4.

### Example 421: Preparation of 1-(5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)pyridin-2-yl)dihydropyrimidine-2,4(1H,3H)-dione (GT-06082)

According to the method of step 2 of Scheme I-1, the target compound (GT-06082) was prepared (white solid, 10 mg, yield 18.61%). ¹H NMR (400 MHz, DMSO-d6) δ 10.59 (d, J = 20.8 Hz, 2H), 9.16 (d, J = 12.7 Hz, 1H), 8.62 (d, J = 7.3 Hz, 1H), 8.58 (s, 1H), 8.55 - 8.42 (m, 2H), 8.09 (d, J = 8.3 Hz, 1H), 7.91 - 7.84 (m, 2H), 7.78 (d, J = 8.6 Hz, 1H), 4.42 (s, 2H), 4.19 (d, J = 11.9 Hz, 2H), 4.11 - 4.05 (m, 4H), 3.73 (s, 4H), 3.00 (d, J = 7.7 Hz, 2H), 2.70 (dd, J = 12.7, 6.3 Hz, 5H), 2.23 (d, J = 9.3 Hz, 2H), 1.92 (t, J = 8.1 Hz, 3H), 1.47 (d, J = 6.5 Hz, 6H), 1.29 (dd, J = 33.5, 17.7 Hz, 4H), 0.62 - 0.48 (m, 2H). LCMS (ESI) calcd for C₃₈H₄₇N₁₄O₄S⁺ [M+H]⁺: 795.36; found, 795.4.

### Example 422: Preparation of 3-((3-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)phenyl)amino)piperidine-2,6-dione (GT-06083)

According to the method of step 2 of Scheme I-1, the target compound (GT-06083) was prepared (white solid, 20 mg, yield 36.62%). ¹H NMR (400 MHz, DMSO-d6) δ 11.88 (s, 1H), 10.84 (s, 1H), 9.18 (s, 1H), 8.59 (s, 2H), 8.44 (d, J = 6.9 Hz, 1H), 7.95 (d, J = 34.7 Hz, 1H), 7.83 (s, 1H), 7.47 (d, J = 5.1 Hz, 1H), 7.36 (s, 1H), 7.26 - 7.14 (m, 1H), 7.05 (d, J = 42.9 Hz, 1H), 6.82 (d, J = 7.4 Hz, 1H), 4.95 - 4.76 (m, 2H), 4.53 - 4.20 (m, 6H), 3.79 (s, 5H), 3.00 (t, J = 12.0 Hz, 3H), 2.85 - 2.76 (m, 1H), 2.63 - 2.55 (m, 1H), 2.22 (d, J = 10.2 Hz, 3H), 1.93 (ddd, J = 22.5, 12.6, 7.6 Hz, 4H), 1.46 (d, J = 6.5 Hz, 6H), 1.27 (dd, J = 11.4, 6.5 Hz, 1H), 0.64 - 0.49 (m, 4H). LCMS (ESI) calcd for C₄₀H₅₀N₁₃O₄S⁺ [M+H]⁺: 808.38; found, 808.4.

### Example 423: Preparation of 3-(4-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)phenyl)piperidine-2,6-dione (GT-06036)

According to the method of step 2 of Scheme I-1, the target compound (GT-06036) was prepared (white solid, 20 mg, yield 34.59%). ¹H NMR (400 MHz, DMSO-d6) δ 11.77 (s, 1H), 11.41 (s, 1H), 10.88 (s, 1H), 9.06 (d, J = 3.0 Hz, 1H), 8.11 (s, 1H), 7.98 (d, J = 7.1 Hz, 1H), 7.65 (dd, J = 13.6, 8.2 Hz, 2H), 7.41 - 7.32 (m, 2H), 6.65 (s, 1H), 4.67 (dt, J = 13.3, 6.6 Hz, 1H), 4.40 (s, 2H), 4.08 (t, J = 12.0 Hz, 4H), 3.94 (dd, J = 11.7, 4.9 Hz, 2H), 3.55 - 3.36 (m, 7H), 3.33 - 3.22 (m, 5H), 2.70 (ddd, J = 17.0, 11.8, 5.1 Hz, 1H), 2.54 (d, J = 4.4 Hz, 1H), 2.22 - 2.13 (m, 1H), 1.97 (dd, J = 11.7, 4.7 Hz, 2H), 1.61 (dd, J = 8.2, 4.2 Hz, 2H), 1.44 (d, J = 6.6 Hz, 6H). LCMS (ESI) calcd for C₃₅H₄₅N₁₀O₃⁺ [M+H]⁺: 653.37; found, 653.4.

### Example 424: Preparation of 1-(4-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)phenyl)dihydropyrimidine-2,4(1H,3H)-dione (GT-06037)

According to the method of step 2 of Scheme I-1, the target compound (GT-06037) was prepared (white solid, 20 mg, yield 34.54%). ¹H NMR (400 MHz, DMSO-d6) δ 11.70 (s, 1H), 11.36 (s, 1H), 10.45 (s, 1H), 9.05 (s, 1H), 8.12 (s, 1H), 7.97 (d, J = 7.1 Hz, 1H), 7.71 (d, J = 8.6 Hz, 2H), 7.45 (d, J = 8.5 Hz, 2H), 6.62 (s, 1H), 4.73 - 4.61 (m, 1H), 4.42 (s, 2H), 4.09 (d, J = 13.6 Hz, 4H), 3.84 (d, J = 6.6 Hz, 2H), 3.69 (s, 2H), 3.45 (dd, J = 24.8, 11.6 Hz, 4H), 3.32 - 3.22 (m, 6H), 2.73 (t, J = 6.6 Hz, 2H), 1.96 (s, 2H), 1.61 (d, J = 8.0 Hz, 2H), 1.44 (d, J = 6.6 Hz, 6H). LCMS (ESI) calcd for C₃₄H₄₄N₁₁O₃⁺ [M+H]⁺: 654.36; found, 654.4.

### Example 425: Preparation of 1-(3-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)phenyl)dihydropyrimidine-2,4(1H,3H)-dione (GT-06038)

According to the method of step 2 of Scheme I-1, the target compound (GT-06038) was prepared (white solid, 14 mg, yield 24.18%). ¹H NMR (400 MHz, DMSO-d6) δ 11.39 (d, J = 46.9 Hz, 2H), 10.45 (s, 1H), 9.04 (s, 1H), 8.09 (s, 1H), 7.96 (d, J = 7.1 Hz, 1H), 7.65 (s, 1H), 7.49 (dd, J = 12.3, 4.6 Hz, 3H), 6.64 (s, 1H), 4.66 (s, 1H), 4.43 (s, 2H), 4.17 - 3.99 (m, 4H), 3.87 (t, J = 6.6 Hz, 2H), 3.65 (s, 4H), 3.43 - 3.41 (m, 2H), 3.29 (s, 6H), 2.73 (t, J = 6.6 Hz, 2H), 1.95 (s, 2H), 1.60 (d, J = 7.8 Hz, 2H), 1.43 (d, J = 6.6 Hz, 6H). LCMS (ESI) calcd for C₃₄H₄₄N₁₁O₃⁺ [M+H]⁺: 654.36; found, 654.4.

### Example 426: Preparation of 1-(6-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)pyridin-3-yl)dihydropyrimidine-2,4(1H,3H)-dione (GT-06039)

According to the method of step 2 of Scheme I-1, the target compound (GT-06039) was prepared (white solid, 10 mg, yield 17.24%). ¹H NMR (400 MHz, DMSO-d6) δ 11.62 (s, 1H), 10.58 (s, 1H), 9.16 (s, 1H), 8.71 (d, J = 2.1 Hz, 1H), 8.08 (s, 1H), 7.98 (d, J = 7.0 Hz, 1H), 7.92 (dd, J = 8.4, 2.4 Hz, 1H), 7.82 (d, J = 8.4 Hz, 1H), 6.67 (s, 1H), 4.77 - 4.65 (m, 1H), 4.58 (s, 2H), 4.00 (s, 9H), 3.65 (s, 2H), 3.53 - 3.45 (m, 4H), 3.28 (s, 3H), 2.75 (t, J = 6.5 Hz, 2H), 1.93 (s, 2H), 1.59 (d, J = 7.9 Hz, 2H), 1.43 (d, J = 6.5 Hz, 6H). LCMS (ESI) calcd for C₃₃H₄₃N₁₂O₃⁺ [M+H]⁺: 655.36; found, 655.4.

### Example 427: Preparation of 1-(5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)pyridin-2-yl)dihydropyrimidine-2,4(1H,3H)-dione (GT-06040)

According to the method of step 2 of Scheme I-1, the target compound (GT-06040) was prepared (white solid, 11 mg, yield 18.97%). ¹H NMR (400 MHz, DMSO-d6) δ 11.59 (s, 1H), 10.78 (s, 1H), 9.16 (s, 1H), 8.71 (d, J = 2.1 Hz, 1H), 8.08 (s, 1H), 7.98 (d, J = 7.0 Hz, 1H), 7.92 (dd, J = 8.4, 2.4 Hz, 1H), 7.82 (d, J = 8.4 Hz, 1H), 6.67 (s, 1H), 4.77 - 4.65 (m, 1H), 4.58 (s, 2H), 4.00 (s, 9H), 3.8 (s, 2H), 3.53 - 3.45 (m, 4H), 3.28 (s, 3H), 2.66 (t, J = 6.5 Hz, 2H), 1.93 (s, 2H), 1.69 (d, J = 7.9 Hz, 2H), 1.33 (d, J = 6.5 Hz, 6H). LCMS (ESI) calcd for C₃₃H₄₃N₁₂O₃⁺ [M+H]⁺: 655.36; found, 655.4.

### Example 428: Preparation of 3-((3-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)phenyl)amino)piperidine-2,6-dione (GT-06041)

According to the method of step 2 of Scheme I-1, the target compound (GT-06041) was prepared (white solid, 15 mg, yield 25.36%). ¹H NMR (400 MHz, DMSO-d6) δ 11.37 (s, 2H), 9.06 (s, 1H), 8.12 (s, 1H), 7.97 (d, J = 7.0 Hz, 1H), 7.67 - 7.39 (m, 2H), 7.17 (dd, J = 43.6, 35.7 Hz, 2H), 6.82 (t, J = 8.3 Hz, 1H), 6.64 (s, 1H), 4.76 - 4.58 (m, 1H), 4.51 - 4.29 (m, 2H), 4.07 (s, 4H), 3.56 - 3.38 (m, 10H), 3.27 (d, J = 26.2 Hz, 6H), 2.64 - 2.54 (m, 1H), 1.97 (d, J = 9.6 Hz, 2H), 1.61 (d, J = 8.2 Hz, 2H), 1.44 (d, J = 6.6 Hz, 6H). LCMS (ESI) calcd for C₃₅H₄₆N₁₁O₃⁺ [M+H]⁺: 668.38; found, 668.4.

### Example 429: Preparation of 3-(4-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)piperidine-2,6-dione (GT-06120)

According to the method of step 2 of Scheme I-1, the target compound (GT-06120) was prepared (white solid, 15 mg, yield 26.42%). ¹H NMR (400 MHz, DMSO-d6) δ 11.44 (s, 1H), 10.89 (d, J = 15.9 Hz, 2H), 9.07 (s, 1H), 8.07 (s, 1H), 7.98 (d, J = 7.0 Hz, 1H), 7.90 - 7.56 (m, 3H), 7.40 - 7.28 (m, 2H), 6.65 (s, 1H), 4.71 - 4.64 (m, 1H), 4.48 (d, J = 10.6 Hz, 1H), 4.18 (d, J = 12.5 Hz, 3H), 4.04 (d, J = 5.1 Hz, 2H), 3.95 - 3.91 (m, 1H), 3.68 (s, 3H), 3.29 (s, 3H), 3.00 - 2.92 (m, 2H), 2.74 - 2.66 (m, 1H), 2.60 (d, J = 4.7 Hz, 3H), 2.54 (s, 1H), 2.35 (d, J = 10.0 Hz, 1H), 2.21 (dd, J = 36.6, 20.7 Hz, 3H), 2.06 - 1.91 (m, 5H), 1.60 (d, J = 8.1 Hz, 2H), 1.44 (d, J = 6.5 Hz, 6H). LCMS (ESI) calcd for C₃₇H₄₉N₁₀O₃⁺ [M+H]⁺: 681.40; found, 681.5.

### Example 430: Preparation of 1-(4-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)dihydropyrimidine-2,4(1H,3H)-dione (GT-06121)

According to the method of step 2 of Scheme I-1, the target compound (GT-06121) was prepared (white solid, 16 mg, yield 28.14%). ¹H NMR (400 MHz, DMSO-d6) δ 11.33 (s, 1H), 10.86 (s, 1H), 10.25 (s, 1H), 9.07 (s, 1H), 8.08 (s, 1H), 7.98 (d, J = 7.0 Hz, 1H), 7.67 (s, 1H), 7.57 - 7.44 (m, 3H), 6.66 (s, 1H), 4.67 (dt, J = 13.0, 6.5 Hz, 1H), 4.51 (d, J = 10.0 Hz, 1H), 4.29 - 4.15 (m, 3H), 4.04 (d, J = 5.2 Hz, 2H), 3.88 (t, J = 6.6 Hz, 2H), 3.65 (s, 5H), 3.20 (s, 3H), 2.86 (d, J = 6.5 Hz, 2H), 2.63 (t, J = 6.5 Hz, 2H), 2.58 (d, J = 4.5 Hz, 3H), 2.21 (dd, J = 33.9, 10.8 Hz, 2H), 2.00 - 1.83 (m, 3H), 1.51 (d, J = 8.1 Hz, 2H), 1.34 (d, J = 6.5 Hz, 6H). LCMS (ESI) calcd for C₃₆H₄₈N₁₁O₃⁺ [M+H]⁺: 682.39; found, 682.4.

### Example 431: Preparation of 1-(3-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)dihydropyrimidine-2,4(1H,3H)-dione (GT-06122)

According to the method of step 2 of Scheme I-1, the target compound (GT-06122) was prepared (white solid, 19 mg, yield 33.42%). ¹H NMR (400 MHz, DMSO-d6) δ 11.43 (s, 1H), 10.97 (s, 1H), 10.45 (s, 1H), 9.07 (s, 1H), 8.08 (s, 1H), 7.98 (d, J = 7.0 Hz, 1H), 7.67 (s, 1H), 7.57 - 7.44 (m, 3H), 6.66 (s, 1H), 4.67 (dt, J = 13.0, 6.5 Hz, 1H), 4.51 (d, J = 10.0 Hz, 1H), 4.29 - 4.15 (m, 3H), 4.04 (d, J = 5.2 Hz, 2H), 3.88 (t, J = 6.6 Hz, 2H), 3.65 (s, 5H), 3.30 (s, 3H), 2.96 (d, J = 6.5 Hz, 2H), 2.73 (t, J = 6.5 Hz, 2H), 2.62 (d, J = 4.5 Hz, 3H), 2.31 (dd, J = 33.9, 10.8 Hz, 2H), 2.02 - 1.93 (m, 3H), 1.61 (d, J = 8.1 Hz, 2H), 1.44 (d, J = 6.5 Hz, 6H). LCMS (ESI) calcd for C₃₆H₄₈N₁₁O₃⁺ [M+H]⁺: 682.39; found, 682.39.

### Example 432: Preparation of 1-(6-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)pyridin-3-yl)dihydropyrimidine-2,4(1H,3H)-dione (GT-06186)

According to the method of step 2 of Scheme I-1, the target compound (GT-06186) was prepared (white solid, 5 mg, yield 8.78%). ¹H NMR (400 MHz, DMSO-d6) δ 11.42 (s, 1H), 10.59 (s, 1H), 9.07 (s, 1H), 8.70 (d, J = 2.4 Hz, 1H), 8.07 (s, 1H), 7.98 (d, J = 7.1 Hz, 1H), 7.91 (dd, J = 8.4, 2.6 Hz, 1H), 7.75 (d, J = 8.4 Hz, 1H), 6.66 (s, 1H), 4.67 (dt, J = 13.3, 6.6 Hz, 1H), 4.52 (s, 1H), 4.19 (d, J = 12.0 Hz, 2H), 4.04 (s, 2H), 3.89 (t, J = 6.6 Hz, 2H), 3.65 (s, 5H), 3.30 (s, 3H), 2.98 (d, J = 12.1 Hz, 2H), 2.78 - 2.71 (m, 5H), 2.27 (s, 2H), 1.97 (d, J = 9.1 Hz, 4H), 1.61 (d, J = 8.5 Hz, 2H), 1.44 (d, J = 6.6 Hz, 6H). LCMS (ESI) calcd for C₃₅H₄₇N₁₂O₃⁺ [M+H]⁺: 683.39; found, 683.4.

### Example 433: Preparation of 1-(5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)pyridin-2-yl)dihydropyrimidine-2,4(1H,3H)-dione (GT-06187)

According to the method of step 2 of Scheme I-1, the target compound (GT-06187) was prepared (white solid, 14 mg, yield 24.58%). ¹H NMR (400 MHz, DMSO-d6) δ 11.53 (s, 1H), 11.19 (s, 1H), 10.61 (s, 1H), 9.11 (s, 1H), 8.66 (d, J = 2.0 Hz, 1H), 8.15 (dd, J = 8.8, 2.3 Hz, 1H), 8.06 (s, 1H), 7.99 (d, J = 7.0 Hz, 1H), 7.87 - 7.66 (m, 2H), 6.67 (s, 1H), 4.72 - 4.65 (m, 1H), 4.53 (d, J = 12.1 Hz, 2H), 4.28 (dd, J = 13.0, 6.7 Hz, 1H), 4.19 (d, J = 12.6 Hz, 2H), 4.12 - 4.00 (m, 6H), 3.30 (s, 3H), 3.04 - 2.93 (m, 2H), 2.77 - 2.68 (m, 3H), 2.62 (d, J = 4.3 Hz, 2H), 2.55 (d, J = 4.5 Hz, 1H), 2.32 (dd, J = 30.6, 12.4 Hz, 2H), 1.96 (d, J = 8.7 Hz, 4H), 1.61 (d, J = 8.1 Hz, 2H), 1.44 (d, J = 6.6 Hz, 6H). LCMS (ESI) calcd for C₃₅H₄₇N₁₂O₃⁺ [M+H]⁺: 683.39; found, 683.4.

### Example 434: Preparation of 3-((3-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)amino)piperidine-2,6-dione (GT-06123)

According to the method of step 2 of Scheme I-1, the target compound (GT-06123) was prepared (white solid, 10 mg, yield 17.23%). ¹H NMR (400 MHz, DMSO-d6) δ 11.48 (s, 1H), 11.00 - 10.73 (m, 2H), 9.09 (s, 1H), 8.08 (s, 1H), 7.99 (d, J = 7.0 Hz, 1H), 7.65 - 7.04 (m, 5H), 6.80 (t, J = 7.7 Hz, 1H), 6.66 (s, 1H), 4.68 (dd, J = 13.2, 6.6 Hz, 1H), 4.49 - 4.34 (m, 2H), 4.18 (d, J = 12.0 Hz, 2H), 4.05 (d, J = 6.2 Hz, 3H), 3.44 (d, J = 6.9 Hz, 5H), 3.30 (s, 3H), 2.95 (d, J = 13.3 Hz, 2H), 2.65 - 2.58 (m, 4H), 2.27 (dd, J = 63.4, 17.3 Hz, 3H), 1.96 (s, 5H), 1.61 (d, J = 8.2 Hz, 2H), 1.44 (d, J = 6.5 Hz, 6H). LCMS (ESI) calcd for C₃₇H₅₀N₁₁O₃⁺ [M+H]⁺: 696.41; found, 696.5.

### Example 435: Preparation of 2-(6,7-dihydro-5H-pyrrolo[1,2-c]imidazol-1-yl)-2-(6-(4-(4-(4-(2,6-dioxopiperidin-3-yl)benzyl)piperazin-1-yl)phenyl)-4-fluoro-1-oxoisoindolin-2-yl)-N-(thiazol-2-yl)acetamide (GT-06163)

According to the method of step 2 of Scheme I-1, the target compound (GT-06163) was prepared (white solid, 20 mg, yield 37.31%). ¹H NMR (400 MHz, DMSO-d6) δ 11.37 (s, 1H), 10.88 (s, 1H), 9.35 (s, 1H), 9.05 (d, J = 16.8 Hz, 1H), 7.81 (t, J = 7.5 Hz, 2H), 7.75 - 7.70 (m, 2H), 7.66 - 7.49 (m, 3H), 7.36 - 7.27 (m, 3H), 7.00 (dd, J = 71.7, 6.6 Hz, 3H), 6.28 (d, J = 90.1 Hz, 1H), 4.84 - 4.71 (m, 1H), 4.52 (d, J = 16.0 Hz, 1H), 4.38 (s, 2H), 4.29 - 4.25 (m, 2H), 3.94 (dd, J = 11.6, 4.8 Hz, 3H), 3.27 - 3.09 (m, 8H), 3.01 (dd, J = 15.5, 7.6 Hz, 1H), 2.88 - 2.80 (m, 1H), 2.74 - 2.67 (m, 1H), 2.62 - 2.55 (m, 2H), 2.24 (dd, J = 12.4, 3.7 Hz, 1H), 2.09 - 2.02 (m, 1H). LCMS (ESI) calcd for C₄₁H₄₀FN₈O₄S⁺ [M+H]⁺: 759.29; found, 759.3.

### Example 436: Preparation of 2-(6,7-dihydro-5H-pyrrolo[1,2-c]imidazol-1-yl)-2-(6-(4-(4-(4-(2,4-dioxotetrahydropyrimidin-1(2H)-yl)benzyl)piperazin-1-yl)phenyl)-4-fluoro-1-oxoisoindolin-2-yl)-N-(thiazol-2-yl)acetamide (GT-06164)

According to the method of step 2 of Scheme I-1, the target compound (GT-06164) was prepared (white solid, 24 mg, yield 44.04%). ¹H NMR (400 MHz, DMSO-d6) δ 11.46 (d, J = 38.3 Hz, 1H), 10.45 (s, 1H), 9.34 (s, 1H), 9.05 (d, J = 17.4 Hz, 1H), 7.81 (t, J = 7.5 Hz, 2H), 7.75 - 7.67 (m, 4H), 7.39 (ddd, J = 25.3, 22.2, 4.0 Hz, 4H), 7.00 (dd, J = 72.5, 6.6 Hz, 3H), 6.28 (d, J = 89.2 Hz, 1H), 4.60 (d, J = 82.8 Hz, 3H), 4.39 (s, 2H), 4.27 (dd, J = 9.1, 5.3 Hz, 2H), 3.93 (s, 2H), 3.84 (t, J = 6.6 Hz, 2H), 3.17 (s, 6H), 2.99 (dd, J = 15.3, 7.9 Hz, 1H), 2.89 - 2.78 (m, 1H), 2.73 (t, J = 6.6 Hz, 2H), 2.64 - 2.57 (m, 2H). LCMS (ESI) calcd for C₄₀H₃₉FN₉O₄S⁺ [M+H]⁺: 760.28; found, 760.3.

### Example 437: Preparation of 2-(6,7-dihydro-5H-pyrrolo[1,2-c]imidazol-1-yl)-2-(6-(4-(4-(3-(2,4-dioxotetrahydropyrimidin-1(2H)-yl)benzyl)piperazin-1-yl)phenyl)-4-fluoro-1-oxoisoindolin-2-yl)-N-(thiazol-2-yl)acetamide (GT-06165)

According to the method of step 2 of Scheme I-1, the target compound (GT-06165) was prepared (white solid, 19 mg, yield 34.86%). ¹H NMR (400 MHz, DMSO-d6) δ 11.40 (s, 1H), 10.45 (s, 1H), 9.33 (s, 1H), 9.04 (d, J = 16.6 Hz, 1H), 7.80 (t, J = 7.3 Hz, 2H), 7.76 - 7.69 (m, 2H), 7.64 (s, 1H), 7.55 - 7.45 (m, 3H), 7.29 (dd, J = 18.5, 4.0 Hz, 1H), 6.99 (dd, J = 73.4, 6.6 Hz, 2H), 6.27 (d, J = 89.2 Hz, 1H), 4.80 - 4.47 (m, 2H), 4.40 (s, 2H), 4.31 - 4.23 (m, 2H), 3.88 (dd, J = 15.5, 8.9 Hz, 5H), 3.27 - 3.08 (m, 6H), 3.04 - 2.93 (m, 1H), 2.84 (dd, J = 16.6, 8.0 Hz, 1H), 2.73 (t, J = 6.6 Hz, 2H), 2.58 (dd, J = 14.9, 6.6 Hz, 2H). LCMS (ESI) calcd for C₄₀H₃₉FN₉O₄S⁺ [M+H]⁺: 760.28; found, 760.3.

### Example 438: Preparation of 2-(6,7-dihydro-5H-pyrrolo[1,2-c]imidazol-1-yl)-2-(6-(4-(4-((5-(2,4-dioxotetrahydropyrimidin-1(2H)-yl)pyridin-2-yl)methyl)piperazin-1-yl)phenyl)-4-fluoro-1-oxoisoindolin-2-yl)-N-(thiazol-2-yl)acetamide (GT-06229)

According to the method of step 2 of Scheme I-1, the target compound (GT-06229) was prepared (white solid, 22 mg, yield 32.25%). ¹H NMR (400 MHz, DMSO) δ 10.59 (s, 1H), 9.47 (s, 1H), 9.09 (d, J = 21.6 Hz, 1H), 8.71 (d, J = 2.5 Hz, 1H), 7.93 (dd, J = 8.4, 2.5 Hz, 1H), 7.81 (dd, J = 8.1, 4.7 Hz, 3H), 7.71 (t, J = 8.6 Hz, 2H), 7.42 (dd, J = 80.7, 3.6 Hz, 1H), 7.30 - 6.91 (m, 3H), 6.29 (d, J = 94.8 Hz, 1H), 4.58 (dd, J = 46.5, 40.2 Hz, 4H), 4.27 (t, J = 7.2 Hz, 2H), 3.90 (d, J = 6.6 Hz, 6H), 3.41 (s, 4H), 3.19 (d, J = 13.0 Hz, 1H), 3.04 - 2.96 (m, 1H), 2.85 (dd, J = 16.0, 7.7 Hz, 1H), 2.76 (t, J = 6.6 Hz, 2H), 2.63 - 2.55 (m, 2H). LCMS (ESI) calcd for C₃₉H₃₈FN₁₀O₄S⁺ [M+H]⁺: 761.28; found, 761.3.

### Example 439: Preparation of 2-(6,7-dihydro-5H-pyrrolo[1,2-c]imidazol-1-yl)-2-(6-(4-(4-((6-(2,4-dioxotetrahydropyrimidin-1(2H)-yl)pyridin-3-yl)methyl)piperazin-1-yl)phenyl)-4-fluoro-1-oxoisoindolin-2-yl)-N-(thiazol-2-yl)acetamide (GT-06166)

According to the method of step 2 of Scheme I-1, the target compound (GT-06166) was prepared (white solid, 23 mg, yield 42.14%). ¹H NMR (400 MHz, DMSO-d6) δ 11.79 (s, 1H), 10.62 (s, 1H), 9.43 (s, 1H), 9.09 - 8.14 (m, 2H), 7.88 - 7.68 (m, 4H), 7.57 - 6.87 (m, 3H), 6.17 (s, 1H), 4.77 (d, J = 40.2 Hz, 1H), 4.52 - 4.42 (m, 2H), 4.27 (t, J = 7.2 Hz, 1H), 4.12 - 3.91 (m, 6H), 3.34 (d, J = 36.1 Hz, 8H), 2.91 (dtd, J = 23.4, 15.7, 7.2 Hz, 2H), 2.70 (dd, J = 13.6, 6.9 Hz, 2H), 2.58 (s, 1H). LCMS (ESI) calcd for C₃₉H₃₈FN₁₀O₄S⁺ [M+H]⁺: 761.28 ; found, 761.3.

### Example 440: Preparation of 2-(6,7-dihydro-5H-pyrrolo[1,2-c]imidazol-1-yl)-2-(6-(4-(4-(3-((2,6-dioxopiperidin-3-yl)amino)benzyl)piperazin-1-yl)phenyl)-4-fluoro-1-oxoisoindolin-2-yl)-N-(thiazol-2-yl)acetamide (GT-06188)

According to the method of step 2 of Scheme I-1, the target compound (GT-06188) was prepared (white solid, 16 mg, yield 28.82%). ¹H NMR (400 MHz, DMSO-d6) δ 11.23 (s, 1H), 10.84 (s, 1H), 9.39 (s, 1H), 9.07 (d, J = 19.9 Hz, 1H), 7.81 (t, J = 7.7 Hz, 2H), 7.71 (d, J = 8.7 Hz, 2H), 7.53 - 7.26 (m, 2H), 7.20 - 7.14 (m, 1H), 7.13 - 7.02 (m, 3H), 6.93 - 6.76 (m, 2H), 6.28 (d, J = 90.9 Hz, 1H), 4.78 - 4.49 (m, 2H), 4.41 (dd, J = 11.4, 4.9 Hz, 1H), 4.26 (d, J = 7.0 Hz, 3H), 3.92 (d, J = 11.3 Hz, 3H), 3.22 (dd, J = 52.6, 11.0 Hz, 8H), 2.99 (dd, J = 15.6, 7.9 Hz, 1H), 2.88 - 2.78 (m, 2H), 2.60 (dd, J = 12.3, 4.1 Hz, 3H), 2.20 (dd, J = 8.7, 4.0 Hz, 1H). LCMS (ESI) calcd for C₄₁H₄₁FN₉O₄S⁺ [M+H]⁺: 774.30; found, 774.3.

### Example 441: Preparation of 2-(6,7-dihydro-5H-pyrrolo[1,2-c]imidazol-1-yl)-2-(6-(4-(4-((4-(2,6-dioxopiperidin-3-yl)benzyl)(methyl)amino)piperidin-1-yl)phenyl)-4-fluoro-1-oxoisoindolin-2-yl)-N-(thiazol-2-yl)acetamide (GT-05218)

According to the method of step 2 of Scheme I-1, the target compound (GT-05218) was prepared (white solid, 22 mg, yield 32.75%). ¹H NMR (400 MHz, DMSO-d6) δ 10.59 (s, 1H), 9.44 (s, 1H), 9.08 (d, J = 21.0 Hz, 1H), 8.69 (d, J = 2.3 Hz, 1H), 7.90 (dd, J = 8.4, 2.4 Hz, 1H), 7.78 (q, J = 8.3 Hz, 3H), 7.66 (d, J = 8.6 Hz, 2H), 7.30 - 6.87 (m, 4H), 6.29 (d, J = 93.5 Hz, 1H), 4.69 (dd, J = 55.7, 38.5 Hz, 2H), 4.48 (d, J = 10.6 Hz, 2H), 4.27 (t, J = 6.9 Hz, 2H), 4.11 - 3.95 (m, 6H), 3.23 (d, J = 44.7 Hz, 8H), 2.95 (dd, J = 15.7, 7.6 Hz, 1H), 2.83 (dd, J = 16.0, 8.2 Hz, 1H), 2.75 (t, J = 6.6 Hz, 2H), 2.59 (d, J = 6.6 Hz, 2H), 1.93 (s, 2H). LCMS (ESI) calcd for C₄₃H₄₄FN₈O₄S⁺ [M+H]⁺: 787.32; found, 787.4.

### Example 442: Preparation of 2-(6,7-dihydro-5H-pyrrolo[1,2-c]imidazol-1-yl)-2-(6-(4-(4-((4-(2,4-dioxotetrahydropyrimidin-1(2H)-yl)benzyl)(methyl)amino)piperidin-1-yl)phenyl)-4-fluoro-1-oxoisoindolin-2-yl)-N-(thiazol-2-yl)acetamide (GT-05219)

According to the method of step 2 of Scheme I-1, the target compound (GT-05219) was prepared (white solid, 15 mg, yield 22.30%). ¹H NMR (400 MHz, DMSO) δ 11.07 (s, 1H), 10.44 (s, 1H), 9.10 (s, 1H), 7.83 (dd, J = 10.7, 3.6 Hz, 2H), 7.76 (d, J = 8.7 Hz, 2H), 7.73 - 7.68 (m, 2H), 7.53 (t, J = 3.6 Hz, 1H), 7.38 (dd, J = 38.7, 6.1 Hz, 3H), 7.30 - 7.25 (m, 2H), 6.40 (s, 1H), 4.86 - 4.47 (m, 3H), 4.25 (dt, J = 13.4, 6.6 Hz, 3H), 3.95 (s, 2H), 3.83 (d, J = 6.7 Hz, 4H), 3.49 (s, 2H), 3.02 - 2.86 (m, 3H), 2.72 (t, J = 6.6 Hz, 2H), 2.60 (d, J = 4.7 Hz, 4H), 2.35 (s, 1H), 2.29 (d, J = 10.0 Hz, 1H), 2.06 (d, J = 11.4 Hz, 2H). LCMS (ESI) calcd for C₄₂H₄₃FN₉O₄S⁺ [M+H]⁺: 788.31; found, 788.3.

### Example 443: Preparation of 2-(6,7-dihydro-5H-pyrrolo[1,2-c]imidazol-1-yl)-2-(6-(4-(4-((3-(2,4-dioxotetrahydropyrimidin-1(2H)-yl)benzyl)(methyl)amino)piperidin-1-yl)phenyl)-4-fluoro-1-oxoisoindolin-2-yl)-N-(thiazol-2-yl)acetamide (GT-05281)

According to the method of step 2 of Scheme I-1, the target compound (GT-05281) was prepared (white solid, 12 mg, yield 14.87%). ¹H NMR (400 MHz, DMSO) δ 11.05 (s, 1H), 10.45 (s, 1H), 9.10 (s, 1H), 7.82 (d, J = 7.4 Hz, 2H), 7.74 (d, J = 8.7 Hz, 2H), 7.67 (s, 1H), 7.53 (d, J = 3.6 Hz, 2H), 7.47 (d, J = 5.7 Hz, 2H), 7.33 (d, J = 3.6 Hz, 1H), 7.21 (d, J = 7.6 Hz, 2H), 6.40 (s, 1H), 4.66 (dd, J = 112.4, 17.2 Hz, 3H), 4.26 (t, J = 7.1 Hz, 2H), 3.95 (s, 2H), 3.88 (d, J = 6.6 Hz, 2H), 3.51 - 3.39 (m, 5H), 2.88 (d, J = 8.9 Hz, 3H), 2.73 (t, J = 6.6 Hz, 2H), 2.59 (t, J = 9.4 Hz, 4H), 2.35 (d, J = 10.9 Hz, 1H), 2.26 (d, J = 10.1 Hz, 1H), 1.99 (s, 2H). LCMS (ESI) calcd for C₄₂H₄₃FN₉O₄S⁺ [M+H]⁺: 788.31; found, 788.3.

### Example 444: Preparation of 2-(6,7-dihydro-5H-pyrrolo[1,2-c]imidazol-1-yl)-2-(6-(4-(4-(((5-(2,4-dioxotetrahydropyrimidin-1(2H)-yl)pyridin-2-yl)methyl)(methyl)amino)piperidin-1-yl)phenyl)-4-fluoro-1-oxoisoindolin-2-yl)-N-(thiazol-2-yl)acetamide (GT-05282)

According to the method of step 2 of Scheme I-1, the target compound (GT-05282) was prepared (white solid, 20 mg, yield 24.75%). ¹H NMR (400 MHz, DMSO) δ 11.21 (s, 1H), 10.59 (s, 1H), 9.13 (s, 1H), 8.71 (d, J = 2.1 Hz, 1H), 7.89 (ddd, J = 18.1, 8.3, 5.4 Hz, 6H), 7.54 (dd, J = 17.1, 5.6 Hz, 3H), 7.32 (d, J = 3.5 Hz, 1H), 6.42 (s, 1H), 4.68 (d, J = 93.1 Hz, 6H), 3.89 (d, J = 6.4 Hz, 4H), 3.66 - 3.54 (m, 2H), 3.28 (s, 2H), 3.10 (d, J = 3.1 Hz, 1H), 2.87 (dd, J = 14.4, 6.1 Hz, 1H), 2.75 (d, J = 10.1 Hz, 5H), 2.64 - 2.54 (m, 2H), 2.39 - 2.23 (m, 4H). LCMS (ESI) calcd for C₄₁H₄₂FN₁₀O₄S⁺ [M+H]⁺: 789.31; found, 789.3.

### Example 445: Preparation of 2-(6,7-dihydro-5H-pyrrolo[1,2-c]imidazol-1-yl)-2-(6-(4-(4-(((6-(2,4-dioxotetrahydropyrimidin-1(2H)-yl)pyridin-3-yl)methyl)(methyl)amino)piperidin-1-yl)phenyl)-4-fluoro-1-oxoisoindolin-2-yl)-N-(thiazol-2-yl)acetamide (GT-05283)

According to the method of step 2 of Scheme I-1, the target compound (GT-05283) was prepared (white solid, 20 mg, yield 24.75%). ¹H NMR (400 MHz, DMSO) δ 11.64 (s, 1H), 10.61 (s, 1H), 9.12 (s, 1H), 8.69 (d, J = 1.7 Hz, 1H), 8.20 (dd, J = 8.7, 2.1 Hz, 1H), 7.86 (dt, J = 8.0, 3.2 Hz, 5H), 7.51 (t, J = 4.2 Hz, 3H), 7.32 (d, J = 3.6 Hz, 1H), 6.41 (s, 1H), 4.80 - 4.51 (m, 3H), 4.27 (d, J = 6.9 Hz, 4H), 3.89 (s, 4H), 3.63 - 3.56 (m, 2H), 3.25 (s, 2H), 3.10 (dd, J = 7.4, 4.2 Hz, 1H), 2.87 (d, J = 7.9 Hz, 1H), 2.70 (t, J = 6.5 Hz, 2H), 2.62 (d, J = 3.7 Hz, 3H), 2.56 (d, J = 6.9 Hz, 1H), 2.45 (d, J = 13.1 Hz, 1H), 2.31 (d, J = 41.5 Hz, 3H). LCMS (ESI) calcd for C₄₁H₄₂FN₁₀O₄S⁺ [M+H]⁺: 789.31; found, 789.3.

### Example 446: Preparation of 2-(6,7-dihydro-5H-pyrrolo[1,2-c]imidazol-1-yl)-2-(6-(4-(4-((3-((2,6-dioxopiperidin-3-yl)amino)benzyl)(methyl)amino)piperidin-1-yl)phenyl)-4-fluoro-1-oxoisoindolin-2-yl)-N-(thiazol-2-yl)acetamide (GT-05284)

According to the method of step 2 of Scheme I-1, the target compound (GT-05284) was prepared (white solid, 15 mg, yield 18.26%). ¹H NMR (400 MHz, DMSO) δ 10.95 (s, 1H), 10.85 (d, J = 2.6 Hz, 1H), 9.11 (s, 1H), 7.85 (d, J = 7.4 Hz, 2H), 7.79 (d, J = 8.4 Hz, 2H), 7.53 (d, J = 3.5 Hz, 1H), 7.34 (t, J = 6.5 Hz, 3H), 7.16 (t, J = 7.7 Hz, 1H), 7.08 (s, 1H), 6.82 (dd, J = 16.0, 7.7 Hz, 2H), 6.41 (s, 1H), 4.84 - 4.53 (m, 2H), 4.46 - 4.33 (m, 2H), 4.26 (t, J = 6.9 Hz, 2H), 4.16 - 4.07 (m, 2H), 3.95 (s, 2H), 3.48 (s, 4H), 3.15 - 3.00 (m, 2H), 2.93 - 2.80 (m, 2H), 2.59 (dd, J = 17.9, 4.8 Hz, 6H), 2.37 (s, 1H), 2.25 (s, 1H), 2.22 - 2.05 (m, 3H), 1.90 (d, J = 8.5 Hz, 1H). LCMS (ESI) calcd for C₄₃H₄₅FN₉O₄S⁺ [M+H]⁺: 802.33; found, 802.4.

### Example 447: Preparation of 2-(6,7-dihydro-5H-pyrrolo[1,2-c]imidazol-1-yl)-2-(6-(4-(4-(4-(4-(2,6-dioxopiperidin-3-yl)benzyl)piperazin-1-yl)piperidin-1-yl)phenyl)-4-fluoro-1-oxoisoindolin-2-yl)-N-(thiazol-2-yl)acetamide (GT-06243)

According to the method of step 2 of Scheme I-1, the target compound (GT-06243) was prepared (white solid, 16 mg, yield 27.06%). ¹H NMR (400 MHz, DMSO-d6) δ 12.16 (s, 1H), 10.90 (d, J = 15.7 Hz, 1H), 9.39 (s, 1H), 9.08 (d, J = 19.4 Hz, 1H), 7.81 (t, J = 6.8 Hz, 2H), 7.72 (d, J = 6.8 Hz, 2H), 7.65 (d, J = 8.1 Hz, 2H), 7.54 - 7.28 (m, 4H), 7.18 (d, J = 8.5 Hz, 2H), 6.29 (d, J = 90.9 Hz, 1H), 4.70 (dd, J = 56.0, 38.8 Hz, 2H), 4.44 (d, J = 13.3 Hz, 2H), 4.29 - 4.24 (m, 2H), 4.05 - 3.81 (m, 10H), 3.37 - 3.30 (m, 2H), 3.01 (dd, J = 15.8, 7.6 Hz, 1H), 2.85 (dt, J = 15.7, 9.0 Hz, 4H), 2.73 - 2.57 (m, 3H), 2.32 - 2.12 (m, 4H), 2.08 - 1.99 (m, 1H), 1.87 (d, J = 9.3 Hz, 2H). LCMS (ESI) calcd for C₄₆H₄₉FN₉O₄S⁺ [M+H]⁺: 842.36; found, 842.3.

### Example 448: Preparation of 2-(6,7-dihydro-5H-pyrrolo[1,2-c]imidazol-1-yl)-2-(6-(4-(4-(4-(4-(2,4-dioxotetrahydropyrimidin-1(2H)-yl)benzyl)piperazin-1-yl)piperidin-1-yl)phenyl)-4-fluoro-1-oxoisoindolin-2-yl)-N-(thiazol-2-yl)acetamide (GT-06244)

According to the method of step 2 of Scheme I-1, the target compound (GT-06244) was prepared (white solid, 10 mg, yield 16.89%). ¹H NMR (400 MHz, DMSO-d6) δ 10.44 (s, 1H), 9.36 (s, 1H), 9.07 (d, J = 18.9 Hz, 1H), 7.82 (d, J = 3.7 Hz, 2H), 7.70 (t, J = 7.2 Hz, 4H), 7.55 - 7.31 (m, 3H), 7.31 - 6.90 (m, 4H), 6.29 (d, J = 89.0 Hz, 1H), 4.73 - 4.49 (m, 2H), 4.44 (d, J = 7.7 Hz, 2H), 4.27 (t, J = 7.2 Hz, 2H), 3.98 (d, J = 11.4 Hz, 2H), 3.83 (t, J = 6.6 Hz, 10H), 3.35 - 3.30 (m, 2H), 3.04 - 2.97 (m, 1H), 2.86 (dd, J = 19.5, 9.6 Hz, 3H), 2.72 (t, J = 6.7 Hz, 2H), 2.60 (dd, J = 15.8, 6.9 Hz, 2H), 2.21 (d, J = 9.3 Hz, 2H), 1.85 (d, J = 11.2 Hz, 2H). LCMS (ESI) calcd for C₄₅H₄₈FN₁₀O₄S⁺ [M+H]⁺: 843.36; found, 843.3.

### Example 449: Preparation of 2-(6,7-dihydro-5H-pyrrolo[1,2-c]imidazol-1-yl)-2-(6-(4-(4-(4-(3-(2,4-dioxotetrahydropyrimidin-1(2H)-yl)benzyl)piperazin-1-yl)piperidin-1-yl)phenyl)-4-fluoro-1-oxoisoindolin-2-yl)-N-(thiazol-2-yl)acetamide (GT-06245)

According to the method of step 2 of Scheme I-1, the target compound (GT-06245) was prepared (white solid, 10 mg, yield 16.89%). ¹H NMR (400 MHz, DMSO-d6) δ 12.24 (s, 1H), 10.45 (s, 1H), 9.39 (s, 1H), 9.08 (d, J = 19.6 Hz, 1H), 7.82 (d, J = 5.2 Hz, 2H), 7.76 - 7.70 (m, 2H), 7.66 (s, 1H), 7.56 - 7.47 (m, 3H), 7.31 (dd, J = 17.1, 4.0 Hz, 1H), 7.19 (d, J = 8.5 Hz, 2H), 6.91 (d, J = 4.4 Hz, 1H), 6.29 (d, J = 90.8 Hz, 1H), 4.70 (dd, J = 55.9, 38.8 Hz, 2H), 4.46 (s, 2H), 4.27 (dd, J = 8.8, 5.4 Hz, 2H), 3.97 (d, J = 12.0 Hz, 2H), 3.87 (d, J = 6.6 Hz, 10H), 3.43 - 3.36 (m, 2H), 3.05 - 2.97 (m, 1H), 2.86 (dt, J = 16.5, 9.7 Hz, 3H), 2.73 (t, J = 6.6 Hz, 2H), 2.65 - 2.56 (m, 2H), 2.22 (d, J = 10.3 Hz, 2H), 1.95 - 1.82 (m, 2H). LCMS (ESI) calcd for C₄₅H₄₈FN₁₀O₄S⁺ [M+H]⁺: 843.36; found, 843.3.

### Example 450: Preparation of 2-(6,7-dihydro-5H-pyrrolo[1,2-c]imidazol-1-yl)-2-(6-(4-(4-(4-((5-(2,4-dioxotetrahydropyrimidin-1(2H)-yl)pyridin-2-yl)methyl)piperazin-1-yl)piperidin-1-yl)phenyl)-4-fluoro-1-oxoisoindolin-2-yl)-N-(thiazol-2-yl)acetamide (GT-06246)

According to the method of step 2 of Scheme I-1, the target compound (GT-06246) was prepared (white solid, 10 mg, yield 16.87%). ¹H NMR (400 MHz, DMSO) δ 10.60 (s, 1H), 9.38 (s, 1H), 9.08 (d, J = 19.9 Hz, 1H), 8.71 (d, J = 2.5 Hz, 1H), 7.95 (dd, J = 8.4, 2.5 Hz, 1H), 7.84 - 7.70 (m, 5H), 7.53 - 6.90 (m, 4H), 6.29 (d, J = 90.6 Hz, 1H), 4.59 (t, J = 42.2 Hz, 4H), 4.28 (d, J = 7.1 Hz, 2H), 3.93 (dd, J = 30.3, 9.3 Hz, 14H), 2.91 (td, J = 21.8, 8.0 Hz, 4H), 2.76 (t, J = 6.6 Hz, 2H), 2.60 (dd, J = 15.6, 6.8 Hz, 2H), 2.24 (d, J = 9.3 Hz, 2H), 1.91 (d, J = 9.3 Hz, 2H). LCMS (ESI) calcd for C₄₄H₄₇FN₁₁O₄S⁺ [M+H]⁺: 844.35; found, 844.4.

### Example 451: Preparation of 2-(6,7-dihydro-5H-pyrrolo[1,2-c]imidazol-1-yl)-2-(6-(4-(4-(4-(3-((2,6-dioxopiperidin-3-yl)amino)benzyl)piperazin-1-yl)piperidin-1-yl)phenyl)-4-fluoro-1-oxoisoindolin-2-yl)-N-(thiazol-2-yl)acetamide (GT-06247)

According to the method of step 2 of Scheme I-1, the target compound (GT-06247) was prepared (white solid, 10 mg, yield 18.69%). ¹H NMR (400 MHz, DMSO-d6) δ 12.25 (s, 1H), 10.85 (s, 1H), 9.40 (s, 1H), 9.08 (d, J = 19.6 Hz, 1H), 7.81 (t, J = 5.7 Hz, 2H), 7.73 (d, J = 8.6 Hz, 2H), 7.55 - 7.29 (m, 2H), 7.23 - 7.03 (m, 3H), 6.93 - 6.76 (m, 2H), 6.29 (d, J = 90.9 Hz, 1H), 4.74 (d, J = 17.0 Hz, 2H), 4.47 - 4.41 (m, 1H), 4.28 (d, J = 7.2 Hz, 2H), 3.97 (d, J = 11.4 Hz, 9H), 3.53 (s, 8H), 3.04 - 2.80 (m, 4H), 2.66 - 2.55 (m, 3H), 2.21 (s, 2H), 1.89 (d, J = 8.2 Hz, 2H). LCMS (ESI) calcd for C₄₆H₅₀FN₁₀O₄S⁺ [M+H]⁺: 857.37; found, 857.3.

### Example 452: Preparation of 2-(6,7-dihydro-5H-pyrrolo[1,2-c]imidazol-1-yl)-2-(6-(4-(4-(1-(4-(2,6-dioxopiperidin-3-yl)benzyl)piperidin-4-yl)piperazin-1-yl)phenyl)-4-fluoro-1-oxoisoindolin-2-yl)-N-(thiazol-2-yl)acetamide (GT-06326)

According to the method of step 2 of Scheme I-1, the target compound (GT-06326) was prepared (white solid, 13 mg, yield 19.79%). ¹H NMR (400 MHz, DMSO-d6) δ 11.20 (d, J = 161.2 Hz, 1H), 10.90 (d, J = 13.5 Hz, 1H), 9.30 (s, 1H), 9.04 (d, J = 15.0 Hz, 1H), 7.87 - 7.79 (m, 2H), 7.73 (d, J = 8.5 Hz, 2H), 7.58 (d, J = 7.9 Hz, 2H), 7.49 - 7.34 (m, 2H), 7.28 (d, J = 4.5 Hz, 1H), 7.14 - 6.89 (m, 3H), 6.28 (d, J = 87.9 Hz, 1H), 4.66 (t, J = 41.8 Hz, 2H), 4.31 - 4.25 (m, 3H), 4.04 - 3.93 (m, 3H), 3.61 (s, 6H), 3.26 - 3.12 (m, 6H), 3.05 - 2.95 (m, 3H), 2.88 - 2.69 (m, 2H), 2.62 - 2.56 (m, 2H), 2.38 (s, 2H), 2.17 (ddd, J = 40.4, 21.2, 11.0 Hz, 4H). LCMS(ESI) C₄₆H₄₉FN₉O₄S⁺ [M+H]⁺: 842.36; found, 842.3.

### Example 453: Preparation of 2-(6,7-dihydro-5H-pyrrolo[1,2-c]imidazol-1-yl)-2-(6-(4-(4-(1-(4-(2,4-dioxotetrahydropyrimidin-1(2H)-yl)benzyl)piperidin-4-yl)piperazin-1-yl)phenyl)-4-fluoro-1-oxoisoindolin-2-yl)-N-(thiazol-2-yl)acetamide (GT-06327)

According to the method of step 2 of Scheme I-1, the target compound (GT-06327) was prepared (white solid, 16 mg, yield 24.32%). ¹H NMR (400 MHz, DMSO-d6) δ 11.03 (s, 1H), 10.45 (s, 1H), 9.26 (s, 1H), 9.02 (d, J = 12.2 Hz, 1H), 7.81 (t, J = 7.2 Hz, 2H), 7.68 (dd, J = 40.6, 7.4 Hz, 4H), 7.55 - 7.32 (m, 3H), 7.27 - 6.91 (m, 3H), 6.27 (d, J = 88.5 Hz, 1H), 4.76 - 4.49 (m, 2H), 4.28 (dd, J = 14.8, 7.9 Hz, 4H), 3.83 (t, J = 6.5 Hz, 4H), 3.29 (s, 10H), 3.00 (dd, J = 14.9, 8.0 Hz, 3H), 2.88 - 2.78 (m, 1H), 2.72 (t, J = 6.5 Hz, 2H), 2.60 (d, J = 8.0 Hz, 2H), 2.38 (s, 2H), 2.24 (s, 2H). LCMS (ESI) calcd for C₄₅H₄₈FN₁₀O₄S⁺ [M+H]⁺: 843.36; found, 843.4.

### Example 454: Preparation of 2-(6,7-dihydro-5H-pyrrolo[1,2-c]imidazol-1-yl)-2-(6-(4-(4-(1-(3-(2,4-dioxotetrahydropyrimidin-1(2H)-yl)benzyl)piperidin-4-yl)piperazin-1-yl)phenyl)-4-fluoro-1-oxoisoindolin-2-yl)-N-(thiazol-2-yl)acetamide (GT-06328)

According to the method of step 2 of Scheme I-1, the target compound (GT-06328) was prepared (white solid, 15 mg, yield 22.80%). ¹H NMR (400 MHz, DMSO-d6) δ 11.21 (d, J = 156.6 Hz, 2H), 10.46 (s, 1H), 9.18 (s, 1H), 9.00 (d, J = 6.7 Hz, 1H), 7.82 (t, J = 7.1 Hz, 2H), 7.77 - 7.59 (m, 4H), 7.53 - 7.30 (m, 5H), 7.26 - 6.88 (m, 4H), 6.27 (d, J = 86.7 Hz, 1H), 4.81 - 4.48 (m, 2H), 4.28 (dd, J = 18.9, 11.9 Hz, 4H), 3.87 (t, J = 6.6 Hz, 9H), 3.22 - 3.11 (m, 6H), 2.90 (dd, J = 26.3, 17.7 Hz, 3H), 2.74 (d, J = 6.4 Hz, 2H), 2.60 (d, J = 8.0 Hz, 2H), 2.38 (s, 2H), 2.23 (s, 2H). LCMS (ESI) calcd for C₄₅H₄₈FN₁₀O₄S⁺ [M+H]⁺: 843.36; found, 843.4.

### Example 455: Preparation of 2-(6,7-dihydro-5H-pyrrolo[1,2-c]imidazol-1-yl)-2-(6-(4-(4-(1-((5-(2,4-dioxotetrahydropyrimidin-1(2H)-yl)pyridin-2-yl)methyl)piperidin-4-yl)piperazin-1-yl)phenyl)-4-fluoro-1-oxoisoindolin-2-yl)-N-(thiazol-2-yl)acetamide (GT-06329)

According to the method of step 2 of Scheme I-1, the target compound (GT-06329) was prepared (white solid, 11 mg, yield 16.70%). ¹H NMR (400 MHz, DMSO-d6) δ 11.61 (s, 1H), 10.59 (s, 1H), 9.37 (d, J = 14.5 Hz, 2H), 9.07 (d, J = 20.5 Hz, 1H), 7.94 - 7.79 (m, 3H), 7.72 (t, J = 8.6 Hz, 3H), 7.54 - 7.31 (m, 1H), 7.30 - 6.89 (m, 4H), 6.28 (d, J = 91.6 Hz, 1H), 4.62 (dd, J = 84.0, 16.8 Hz, 3H), 4.27 (s, 2H), 3.90 (dd, J = 18.9, 12.5 Hz, 6H), 3.28 (dd, J = 77.4, 27.6 Hz, 12H), 2.99 - 2.84 (m, 2H), 2.75 (d, J = 6.5 Hz, 1H), 2.59 (d, J = 5.7 Hz, 2H), 2.31 (dd, J = 41.0, 15.9 Hz, 3H), 2.03 (d, J = 11.3 Hz, 1H). LCMS (ESI) calcd for C₄₄H₄₇FN₁₁O₄S⁺ [M+H]⁺: 844.35; found, 844.4.

### Example 456: Preparation of 2-(6,7-dihydro-5H-pyrrolo[1,2-c]imidazol-1-yl)-2-(6-(4-(4-(1-((6-(2,4-dioxotetrahydropyrimidin-1(2H)-yl)pyridin-3-yl)methyl)piperidin-4-yl)piperazin-1-yl)phenyl)-4-fluoro-1-oxoisoindolin-2-yl)-N-(thiazol-2-yl)acetamide (GT-06330)

According to the method of step 2 of Scheme I-1, the target compound (GT-06330) was prepared (white solid, 13 mg, yield 19.74%). ¹H NMR (400 MHz, DMSO-d6) δ 11.42 (d, J = 90.8 Hz, 2H), 10.62 (s, 1H), 9.44 (s, 2H), 9.05 (s, 1H), 8.60 (s, 1H), 8.08 (d, J = 8.5 Hz, 1H), 7.83 (d, J = 13.1 Hz, 2H), 7.72 (d, J = 8.1 Hz, 2H), 7.28 - 6.88 (m, 4H), 6.29 (d, J = 92.6 Hz, 1H), 4.63 (dd, J = 85.1, 16.5 Hz, 2H), 4.36 (s, 2H), 4.27 (t, J = 7.0 Hz, 3H), 4.10 (t, J = 6.4 Hz, 2H), 3.95 (s, 4H), 3.34 - 3.15 (m, 8H), 3.05 - 2.92 (m, 3H), 2.81 (dd, J = 16.1, 7.6 Hz, 1H), 2.70 (t, J = 6.4 Hz, 2H), 2.59 (d, J = 5.8 Hz, 2H), 2.39 (s, 2H), 2.23 (d, J = 8.8 Hz, 2H). LCMS (ESI) calcd for C₄₄H₄₇FN₁₁O₄S⁺ [M+H]⁺: 844.35; found, 844.4.

### Example 457: Preparation of 2-(6,7-dihydro-5H-pyrrolo[1,2-c]imidazol-1-yl)-2-(6-(4-(4-(1-(3-((2,6-dioxopiperidin-3-yl)amino)benzyl)piperidin-4-yl)piperazin-1-yl)phenyl)-4-fluoro-1-oxoisoindolin-2-yl)-N-(thiazol-2-yl)acetamide (GT-06331)

According to the method of step 2 of Scheme I-1, the target compound (GT-06331) was prepared (white solid, 11 mg, yield 16.45%). ¹H NMR (400 MHz, DMSO-d6) δ 11.46 (s, 1H), 11.02 - 10.75 (m, 2H), 9.39 (s, 1H), 9.06 (d, J = 19.9 Hz, 1H), 7.87 - 7.78 (m, 2H), 7.72 (d, J = 7.9 Hz, 2H), 7.53 - 7.27 (m, 2H), 7.20 - 7.04 (m, 3H), 6.98 - 6.77 (m, 2H), 6.28 (d, J = 91.0 Hz, 1H), 4.81 - 4.45 (m, 3H), 4.29 (d, J = 19.8 Hz, 2H), 3.94 (s, 8H), 3.38 - 3.15 (m, 8H), 3.03 - 2.95 (m, 2H), 2.89 - 2.76 (m, 2H), 2.65 - 2.54 (m, 3H), 2.37 (s, 2H), 2.28 - 2.11 (m, 2H), 1.93 (dd, J = 22.6, 10.7 Hz, 1H). LCMS (ESI) calcd for C₄₆H₅₀FN₁₀O₄S⁺ [M+H]⁺: 857.37; found, 857.4.

### Example 458: Preparation of 2-(6,7-dihydro-5H-pyrrolo[1,2-c]imidazol-1-yl)-2-(6-(4-(4-(4-(4-(2,6-dioxopiperidin-3-yl)benzyl)piperazin-1-yl)-3,3-difluoropiperidin-1-yl)phenyl)-4-fluoro-1-oxoisoindolin-2-yl)-N-(thiazol-2-yl)acetamide (GT-06317)

According to the method of step 2 of Scheme I-1, the target compound (GT-06317) was prepared (white solid, 31 mg, yield 53.10%). ¹H NMR (400 MHz, DMSO-d6) δ 10.86 (s, 1H), 9.05 (d, J = 18.8 Hz, 1H), 7.83 - 7.63 (m, 3H), 7.61 - 7.30 (m, 4H), 7.26 - 6.91 (m, 2H), 6.27 (d, J = 89.4 Hz, 1H), 4.61 (dd, J = 83.2, 17.1 Hz, 2H), 4.27 (d, J = 10.2 Hz, 2H), 4.05 (s, 2H), 3.38 - 3.07 (m, 17H), 2.99 - 2.85 (m, 3H), 2.67 - 2.55 (m, 2H), 2.27 - 2.00 (m, 2H), 1.88 (s, 1H). LCMS (ESI) calcd for C₄₆H₄₇F₃N₉O₄S⁺ [M+H]⁺: 878.34; found, 878.4.

### Example 459: Preparation of 2-(6,7-dihydro-5H-pyrrolo[1,2-c]imidazol-1-yl)-2-(6-(4-(4-(4-(4-(2,4-dioxotetrahydropyrimidin-1(2H)-yl)benzyl)piperazin-1-yl)-3,3-difluoropiperidin-1-yl)phenyl)-4-fluoro-1-oxoisoindolin-2-yl)-N-(thiazol-2-yl)acetamide (GT-06318)

According to the method of step 2 of Scheme I-1, the target compound (GT-06318) was prepared (white solid, 30 mg, yield 57.75%). ¹H NMR (400 MHz, DMSO-d6) δ 11.15 (s, 1H), 10.44 (s, 1H), 9.39 (s, 1H), 9.08 (d, J = 19.1 Hz, 1H), 7.87 - 7.63 (m, 5H), 7.56 - 7.32 (m, 3H), 7.14 (dd, J = 39.2, 8.6 Hz, 2H), 6.29 (d, J = 90.7 Hz, 1H), 4.76 - 4.44 (m, 2H), 4.31 - 4.25 (m, 2H), 4.06 (s, 2H), 3.82 (d, J = 6.6 Hz, 6H), 3.07 (ddd, J = 47.8, 35.8, 31.3 Hz, 14H), 2.72 (t, J = 6.6 Hz, 1H), 2.58 (dd, J = 18.6, 11.0 Hz, 2H), 1.90 (s, 1H). LCMS (ESI) calcd for C₄₅H₄₆F₃N₁₀O₄S⁺ [M+H]⁺: 879.34; found, 879.3.

### Example 460: Preparation of 2-(6,7-dihydro-5H-pyrrolo[1,2-c]imidazol-1-yl)-2-(6-(4-(4-(4-((5-(2,4-dioxotetrahydropyrimidin-1(2H)-yl)pyridin-2-yl)methyl)piperazin-1-yl)-3,3-difluoropiperidin-1-yl)phenyl)-4-fluoro-1-oxoisoindolin-2-yl)-N-(thiazol-2-yl)acetamide (GT-06319)

According to the method of step 2 of Scheme I-1, the target compound (GT-06319) was prepared (white solid, 28 mg, yield 53.84%). ¹H NMR (400 MHz, DMSO-d6) δ 10.59 (s, 1H), 9.44 (s, 1H), 9.08 (d, J = 21.0 Hz, 1H), 8.69 (d, J = 2.3 Hz, 1H), 7.90 (dd, J = 8.4, 2.4 Hz, 1H), 7.79 (t, J = 8.3 Hz, 2H), 7.72 - 7.64 (m, 2H), 7.56 - 6.89 (m, 4H), 6.29 (d, J = 93.5 Hz, 1H), 4.76 - 4.46 (m, 4H), 4.27 (t, J = 6.9 Hz, 2H), 4.07 - 3.90 (m, 7H), 3.33 - 2.89 (m, 12H), 2.75 (t, J = 6.6 Hz, 2H), 2.65 - 2.55 (m, 2H), 1.93 (s, 1H). LCMS (ESI) calcd for C₄₄H₄₅F₃N₁₁O₄S⁺ [M+H]⁺: 880.33; found, 880.4.

### Example 461: Preparation of 2-(6,7-dihydro-5H-pyrrolo[1,2-c]imidazol-1-yl)-2-(6-(4-(4-(4-((6-(2,4-dioxotetrahydropyrimidin-1(2H)-yl)pyridin-3-yl)methyl)piperazin-1-yl)-3,3-difluoropiperidin-1-yl)phenyl)-4-fluoro-1-oxoisoindolin-2-yl)-N-(thiazol-2-yl)acetamide (GT-06320)

According to the method of step 2 of Scheme I-1, the target compound (GT-06320) was prepared (white solid, 35 mg, yield 67.29%). ¹H NMR (400 MHz, DMSO-d6) δ 11.59 - 10.96 (m, 1H), 10.61 (s, 1H), 9.47 (d, J = 27.1 Hz, 1H), 9.08 (d, J = 20.8 Hz, 1H), 8.62 (d, J = 1.5 Hz, 1H), 8.11 (dd, J = 8.7, 1.9 Hz, 1H), 7.87 - 7.74 (m, 3H), 7.66 (d, J = 8.6 Hz, 2H), 7.55 - 6.90 (m, 4H), 6.29 (d, J = 93.1 Hz, 1H), 4.62 (d, J = 68.7 Hz, 1H), 4.35 (s, 2H), 4.26 (s, 2H), 4.08 (t, J = 6.5 Hz, 3H), 3.95 (d, J = 12.0 Hz, 5H), 3.29 - 2.79 (m, 12H), 2.70 (t, J = 6.5 Hz, 2H), 2.62 - 2.54 (m, 2H), 1.92 (s, 1H). LCMS (ESI) calcd for C₄₄H₄₅F₃N₁₁O₄S⁺ [M+H]⁺: 880.33; found, 880.4.

### Example 462: Preparation of 2-(6,7-dihydro-5H-pyrrolo[1,2-c]imidazol-1-yl)-2-(6-(4-(4-(4-(3-((2,6-dioxopiperidin-3-yl)amino)benzyl)piperazin-1-yl)-3,3-difluoropiperidin-1-yl) phenyl)-4-fluoro-1-oxoisoindolin-2-yl)-N-(thiazol-2-yl)acetamide (GT-06321)

According to the method of step 2 of Scheme I-1, the target compound (GT-06321) was prepared (white solid, 21 mg, yield 39.79%). ¹H NMR (400 MHz, DMSO-d6) δ 10.83 (s, 1H), 9.40 (s, 1H), 9.05 (d, J = 20.7 Hz, 1H), 7.78 (dd, J = 10.2, 6.2 Hz, 2H), 7.65 (d, J = 6.7 Hz, 2H), 7.54 - 6.76 (m, 8H), 6.27 (d, J = 91.0 Hz, 1H), 4.78 - 4.42 (m, 3H), 4.39 - 4.16 (m, 6H), 4.06 (s, 5H), 3.15 - 2.81 (m, 12H), 2.59 (d, J = 16.4 Hz, 3H), 1.90 (d, J = 11.1 Hz, 2H). LCMS (ESI) calcd for C₄₆H₄₈F₃N₁₀O₄S⁺ [M+H]⁺: 893.35; found, 893.4.

### Example 463: Preparation of 2-(6,7-dihydro-5H-pyrrolo[1,2-c]imidazol-1-yl)-2-(6-(4-((1R,4R)-5-(4-(2,6-dioxopiperidin-3-yl)benzyl)-2,5-diazabicyclo[2.2.1]heptan-2-yl)phenyl)-4-fluoro-1-oxoisoindolin-2-yl)-N-(thiazol-2-yl)acetamide (GT-06176)

According to the method of step 2 of Scheme I-1, the target compound (GT-06176) was prepared (white solid, 15 mg, yield 27.71%). ¹H NMR (400 MHz, DMSO-d6) δ 10.86 (s, 1H), 10.44 (s, 1H), 9.01 (d, J = 9.6 Hz, 1H), 7.82 - 7.76 (m, 1H), 7.67 - 7.52 (m, 3H), 7.32 (t, J = 5.8 Hz, 2H), 6.75 (d, J = 8.7 Hz, 1H), 6.37 (s, 1H), 4.80 - 4.47 (m, 3H), 4.31 (dd, J = 42.1, 19.1 Hz, 3H), 3.91 (dd, J = 11.8, 4.9 Hz, 1H), 3.68 (dd, J = 22.0, 9.9 Hz, 4H), 3.27 - 3.08 (m, 4H), 2.87 (d, J = 7.1 Hz, 1H), 2.64 (ddd, J = 37.7, 22.1, 11.9 Hz, 5H), 2.19 (d, J = 11.1 Hz, 2H), 2.04 (s, 1H). LCMS (ESI) calcd for C₄₂H₄₀FN₈O₄S⁺ [M+H]⁺: 771.29; found, 771.3.

### Example 464: Preparation of 2-(6,7-dihydro-5H-pyrrolo[1,2-c]imidazol-1-yl)-2-(6-(4-((1R,4R)-5-(4-(2,4-dioxotetrahydropyrimidin-1(2H)-yl)benzyl)-2,5-diazabicyclo[2.2.1]heptan-2-yl)phenyl)-4-fluoro-1-oxoisoindolin-2-yl)-N-(thiazol-2-yl)acetamide (GT-06177)

According to the method of step 2 of Scheme I-1, the target compound (GT-06177) was prepared (white solid, 12 mg, yield 22.14%). ¹H NMR (400 MHz, DMSO-d6) δ 10.69 (s, 1H), 10.42 (d, J = 7.5 Hz, 1H), 9.04 (d, J = 14.8 Hz, 1H), 7.83 - 7.70 (m, 4H), 7.65 (t, J = 7.4 Hz, 2H), 7.53 (d, J = 3.6 Hz, 1H), 7.45 - 7.37 (m, 2H), 7.33 (d, J = 3.6 Hz, 1H), 7.28 (d, J = 5.4 Hz, 1H), 6.80 (dd, J = 43.2, 8.3 Hz, 2H), 6.38 (s, 1H), 4.58 (ddd, J = 34.9, 26.5, 13.1 Hz, 4H), 4.33 - 4.23 (m, 3H), 3.81 (t, J = 6.6 Hz, 2H), 3.65 (d, J = 8.7 Hz, 5H), 3.11 (dd, J = 82.8, 19.1 Hz, 6H), 2.72 (t, J = 6.7 Hz, 2H), 2.19 (d, J = 10.6 Hz, 1H). LCMS (ESI) calcd for C₄₁H₃₉FN₉O₄S⁺ [M+H]⁺: 772.28; found, 772.3.

### Example 465: Preparation of 2-(6,7-dihydro-5H-pyrrolo[1,2-c]imidazol-1-yl)-2-(6-(4-((1R,4R)-5-(3-(2,4-dioxotetrahydropyrimidin-1(2H)-yl)benzyl)-2,5-diazabicyclo[2.2.1]heptan-2-yl)phenyl)-4-fluoro-1-oxoisoindolin-2-yl)-N-(thiazol-2-yl)acetamide (GT-06178)

According to the method of step 2 of Scheme I-1, the target compound (GT-06178) was prepared (white solid, 17 mg, yield 31.37%). ¹H NMR (400 MHz, DMSO-d6) δ 10.82 (s, 1H), 10.43 (d, J = 14.5 Hz, 2H), 9.31 (s, 1H), 9.05 (d, J = 15.1 Hz, 1H), 7.83 - 7.63 (m, 4H), 7.55 (ddd, J = 11.1, 8.0, 4.3 Hz, 2H), 7.47 - 7.38 (m, 2H), 7.31 (dd, J = 18.4, 4.0 Hz, 1H), 6.74 (d, J = 8.6 Hz, 2H), 4.85 - 4.44 (m, 4H), 4.39 - 4.19 (m, 3H), 3.81 (ddd, J = 18.4, 13.6, 6.8 Hz, 4H), 3.64 (d, J = 8.7 Hz, 3H), 3.28 - 2.96 (m, 4H), 2.92 - 2.78 (m, 1H), 2.72 (td, J = 6.6, 3.2 Hz, 2H), 2.64 - 2.56 (m, 2H). LCMS (ESI) calcd for C₄₁H₃₉FN₉O₄S⁺ [M+H]⁺: 772.28; found, 772.3.

### Example 466: Preparation of 2-(6,7-dihydro-5H-pyrrolo[1,2-c]imidazol-1-yl)-2-(6-(4-((1R,4R)-5-((5-(2,4-dioxotetrahydropyrimidin-1(2H)-yl)pyridin-2-yl)methyl)-2,5-diazabicyclo[2.2.1]heptan-2-yl)phenyl)-4-fluoro-1-oxoisoindolin-2-yl)-N-(thiazol-2-yl)acetamide (GT-06190)

According to the method of step 2 of Scheme I-1, the target compound (GT-06190) was prepared (white solid, 19 mg, yield 35.01%). ¹H NMR (400 MHz, DMSO-d6) δ 10.90 - 10.64 (m, 1H), 10.57 (s, 1H), 9.35 (s, 1H), 9.06 (d, J = 17.4 Hz, 1H), 8.65 (d, J = 2.4 Hz, 1H), 7.88 (dd, J = 8.4, 2.5 Hz, 1H), 7.79 (t, J = 7.1 Hz, 2H), 7.74 - 7.66 (m, 3H), 7.26 (ddd, J = 152.4, 114.2, 4.0 Hz, 2H), 6.77 (t, J = 9.2 Hz, 2H), 6.28 (d, J = 89.3 Hz, 1H), 4.75 - 4.57 (m, 3H), 4.48 (d, J = 7.6 Hz, 1H), 4.27 (dd, J = 9.1, 5.4 Hz, 2H), 3.87 (t, J = 6.7 Hz, 2H), 3.74 (s, 2H), 3.13 (d, J = 32.2 Hz, 5H), 3.04 - 2.81 (m, 3H), 2.75 (t, J = 6.6 Hz, 2H), 2.64 - 2.56 (m, 2H), 2.22 (d, J = 10.8 Hz, 1H). LCMS (ESI) calcd for C₄₀H₃₈FN₁₀O₄S⁺ [M+H]⁺: 773.28; found, 773.3.

### Example 467: Preparation of 2-(6,7-dihydro-5H-pyrrolo[1,2-c]imidazol-1-yl)-2-(6-(4-((1R,4R)-5-((6-(2,4-dioxotetrahydropyrimidin-1(2H)-yl)pyridin-3-yl)methyl)-2,5-diazabicyclo[2.2.1]heptan-2-yl)phenyl)-4-fluoro-1-oxoisoindolin-2-yl)-N-(thiazol-2-yl)acetamide (GT-06191)

According to the method of step 2 of Scheme I-1, the target compound (GT-06191) was prepared (white solid, 26 mg, yield 47.91%). ¹H NMR (400 MHz, DMSO-d6) δ 10.70 - 10.47 (m, 1H), 9.35 (s, 1H), 8.57 - 8.19 (m, 1H), 7.86 - 7.64 (m, 3H), 7.31 - 6.72 (m, 2H), 5.93 (d, J = 185.6 Hz, 1H), 4.78 - 4.43 (m, 3H), 4.40 - 4.25 (m, 2H), 4.09 - 4.02 (m, 2H), 3.79 (d, J = 10.4 Hz, 3H), 3.22 (d, J = 36.7 Hz, 9H), 3.02 - 2.83 (m, 1H), 2.69 (t, J = 6.5 Hz, 2H), 2.62 - 2.54 (m, 1H). LCMS (ESI) calcd for C₄₀H₃₈FN₁₀O₄S⁺ [M+H]⁺: 773.28; found, 773.3.

### Example 468: Preparation of 2-(6,7-dihydro-5H-pyrrolo[1,2-c]imidazol-1-yl)-2-(6-(4-((1R,4R)-5-(3-((2,6-dioxopiperidin-3-yl)amino)benzyl)-2,5-diazabicyclo[2.2.1]heptan-2-yl)phenyl)-4-fluoro-1-oxoisoindolin-2-yl)-N-(thiazol-2-yl)acetamide (GT-06179)

According to the method of step 2 of Scheme I-1, the target compound (GT-06179) was prepared (white solid, 10 mg, yield 18.69%). ¹H NMR (400 MHz, DMSO-d6) δ 10.86 (dd, J = 20.0, 13.4 Hz, 1H), 9.39 (s, 1H), 9.17 - 8.98 (m, 1H), 7.80 (dd, J = 12.9, 5.3 Hz, 1H), 7.65 (t, J = 8.2 Hz, 1H), 7.56 - 7.27 (m, 2H), 7.19 - 7.07 (m, 1H), 6.91 - 6.73 (m, 2H), 6.45 - 6.15 (m, 1H), 4.80 - 4.37 (m, 4H), 4.31 - 4.20 (m, 2H), 3.82 (s, 6H), 3.20 (s, 6H), 2.90 (ddd, J = 30.5, 15.6, 7.4 Hz, 2H), 2.63 - 2.55 (m, 2H), 2.16 (s, 1H). LCMS (ESI) calcd for C₄₂H₄₁FN₉O₄S⁺ [M+H]⁺: 786.30; found, 786.3.

### Example 469: Preparation of 2-(6,7-dihydro-5H-pyrrolo[1,2-c]imidazol-1-yl)-2-(6-(4-(8-(4-(2,6-dioxopiperidin-3-yl)benzyl)-3,8-diazabicyclo[3.2.1]octan-3-yl)phenyl)-4-fluoro-1-oxoisoindolin-2-yl)-N-(thiazol-2-yl)acetamide (GT-06252)

According to the method of step 2 of Scheme I-1, the target compound (GT-06252) was prepared (white solid, 20 mg, yield 33.05%). ¹H NMR (400 MHz, DMSO-d6) δ 11.52 (s, 1H), 10.89 (s, 1H), 9.05 (d, J = 14.8 Hz, 1H), 7.86 - 7.67 (m, 6H), 7.55 - 6.93 (m, 6H), 6.28 (d, J = 88.4 Hz, 1H), 4.86 - 4.48 (m, 2H), 4.26 (s, 3H), 3.95 (d, J = 5.3 Hz, 2H), 3.75 (d, J = 11.6 Hz, 2H), 3.54 (s, 5H), 3.02 - 2.80 (m, 2H), 2.74 - 2.54 (m, 4H), 2.40 - 2.20 (m, 3H), 2.10 - 1.96 (m, 3H). LCMS (ESI) calcd for C₄₃H₄₂FN₈O₄S⁺ [M+H]⁺: 785.30; found, 785.3.

### Example 470: Preparation of 2-(6,7-dihydro-5H-pyrrolo[1,2-c]imidazol-1-yl)-2-(6-(4-(8-(4-(2,4-dioxotetrahydropyrimidin-1(2H)-yl)benzyl)-3,8-diazabicyclo[3.2.1]octan-3-yl)phenyl)-4-fluoro-1-oxoisoindolin-2-yl)-N-(thiazol-2-yl)acetamide (GT-06253)

According to the method of step 2 of Scheme I-1, the target compound (GT-06253) was prepared (white solid, 18 mg, yield 29.71%). ¹H NMR (400 MHz, DMSO-d6) δ 11.54 (d, J = 30.1 Hz, 1H), 10.46 (s, 1H), 9.05 (d, J = 14.9 Hz, 1H), 7.90 - 7.67 (m, 6H), 7.57 - 7.29 (m, 4H), 7.02 - 6.91 (m, 2H), 6.28 (d, J = 88.1 Hz, 1H), 4.83 - 4.49 (m, 2H), 4.27 (d, J = 6.5 Hz, 3H), 3.95 (s, 2H), 3.85 (t, J = 6.6 Hz, 2H), 3.76 (d, J = 11.2 Hz, 2H), 3.54 (d, J = 11.9 Hz, 5H), 2.96 - 2.80 (m, 1H), 2.73 (t, J = 6.6 Hz, 2H), 2.66 - 2.55 (m, 2H), 2.35 (d, J = 5.3 Hz, 2H), 2.02 (d, J = 8.3 Hz, 2H). LCMS (ESI) calcd for C₄₂H₄₁FN₉O₄S⁺ [M+H]⁺: 786.30; found, 786.3.

### Example 471: Preparation of 2-(6,7-dihydro-5H-pyrrolo[1,2-c]imidazol-1-yl)-2-(6-(4-(8-(3-(2,4-dioxotetrahydropyrimidin-1(2H)-yl)benzyl)-3,8-diazabicyclo[3.2.1]octan-3-yl)phenyl)-4-fluoro-1-oxoisoindolin-2-yl)-N-(thiazol-2-yl)acetamide (GT-06254)

According to the method of step 2 of Scheme I-1, the target compound (GT-06254) was prepared (white solid, 18 mg, yield 29.71%). ¹H NMR (400 MHz, DMSO-d6) δ 11.55 (s, 1H), 10.45 (s, 1H), 9.05 (d, J = 16.7 Hz, 1H), 7.85 - 7.76 (m, 3H), 7.68 (t, J = 9.3 Hz, 3H), 7.50 (dd, J = 13.6, 4.3 Hz, 2H), 7.35 - 7.28 (m, 1H), 6.94 (dd, J = 31.9, 6.6 Hz, 2H), 6.28 (d, J = 89.1 Hz, 1H), 4.70 (t, J = 56.5 Hz, 2H), 4.26 (d, J = 7.7 Hz, 3H), 3.96 - 3.77 (m, 5H), 3.54 (s, 8H), 2.96 - 2.84 (m, 1H), 2.73 (t, J = 6.6 Hz, 2H), 2.57 (dd, J = 18.5, 11.1 Hz, 2H), 2.33 (s, 1H), 2.01 (d, J = 8.3 Hz, 1H). LCMS (ESI) calcd for C₄₂H₄₁FN₉O₄S⁺ [M+H]⁺: 786.30; found, 786.3.

### Example 472: Preparation of 2-(6,7-dihydro-5H-pyrrolo[1,2-c]imidazol-1-yl)-2-(6-(4-(8-((5-(2,4-dioxotetrahydropyrimidin-1(2H)-yl)pyridin-2-yl)methyl)-3,8-diazabicyclo[3.2.1]octan-3-yl)phenyl)-4-fluoro-1-oxoisoindolin-2-yl)-N-(thiazol-2-yl)acetamide (GT-06255)

According to the method of step 2 of Scheme I-1, the target compound (GT-06255) was prepared (white solid, 10 mg, yield 16.48%). ¹H NMR (400 MHz, DMSO-d6) δ 10.60 (s, 1H), 9.41 (d, J = 33.1 Hz, 1H), 9.06 (d, J = 18.2 Hz, 1H), 8.72 (d, J = 2.5 Hz, 1H), 7.93 (dd, J = 8.4, 2.5 Hz, 1H), 7.88 - 7.76 (m, 3H), 7.74 - 7.65 (m, 2H), 7.53 - 6.90 (m, 4H), 6.28 (d, J = 89.4 Hz, 1H), 4.62 (dd, J = 83.9, 17.0 Hz, 2H), 4.27 (dd, J = 8.9, 5.4 Hz, 2H), 4.17 (s, 2H), 3.90 (t, J = 6.6 Hz, 2H), 3.80 (d, J = 11.7 Hz, 2H), 3.59 (s, 6H), 2.98 (s, 1H), 2.77 (t, J = 6.6 Hz, 2H), 2.59 (dd, J = 15.3, 6.4 Hz, 2H), 2.34 (s, 2H), 2.05 (d, J = 8.3 Hz, 2H). LCMS (ESI) calcd for C₄₁H₄₀FN₁₀O₄S⁺ [M+H]⁺: 787.29; found, 787.3.

### Example 473: Preparation of 2-(6,7-dihydro-5H-pyrrolo[1,2-c]imidazol-1-yl)-2-(6-(4-(8-((6-(2,4-dioxotetrahydropyrimidin-1(2H)-yl)pyridin-3-yl)methyl)-3,8-diazabicyclo[3.2.1]octan-3-yl)phenyl)-4-fluoro-1-oxoisoindolin-2-yl)-N-(thiazol-2-yl)acetamide (GT-06256)

According to the method of step 2 of Scheme I-1, the target compound (GT-06256) was prepared (white solid, 15 mg, yield 24.72%). ¹H NMR (400 MHz, DMSO-d6) δ 11.97 (s, 1H), 10.63 (s, 1H), 9.41 (s, 1H), 9.08 (d, J = 19.6 Hz, 1H), 8.79 (d, J = 1.9 Hz, 1H), 8.34 (dd, J = 8.7, 2.2 Hz, 1H), 7.87 (d, J = 8.7 Hz, 1H), 7.79 (dd, J = 9.8, 6.3 Hz, 2H), 7.70 (d, J = 8.7 Hz, 2H), 7.54 - 6.89 (m, 4H), 6.29 (d, J = 92.0 Hz, 1H), 4.71 (dd, J = 68.8, 42.9 Hz, 2H), 4.34 - 4.23 (m, 4H), 4.11 (t, J = 6.5 Hz, 2H), 3.99 (s, 2H), 3.58 (d, J = 11.7 Hz, 6H), 3.03 - 2.83 (m, 1H), 2.71 (t, J = 6.5 Hz, 2H), 2.63 - 2.56 (m, 2H), 2.35 (s, 2H), 2.02 (d, J = 8.2 Hz, 2H). LCMS (ESI) calcd for C₄₁H₄₀FN₁₀O₄S⁺ [M+H]⁺: 787.29; found, 787.3.

### Example 474: Preparation of 2-(6,7-dihydro-5H-pyrrolo[1,2-c]imidazol-1-yl)-2-(6-(4-(8-(3-((2,6-dioxopiperidin-3-yl)amino)benzyl)-3,8-diazabicyclo[3.2.1]octan-3-yl)phenyl)-4-fluoro-1-oxoisoindolin-2-yl)-N-(thiazol-2-yl)acetamide (GT-06257)

According to the method of step 2 of Scheme I-1, the target compound (GT-06257) was prepared (white solid, 17 mg, yield 27.57%). ¹H NMR (400 MHz, DMSO-d6) δ 11.35 (s, 1H), 10.85 (s, 1H), 9.06 (d, J = 18.6 Hz, 1H), 7.82 - 7.68 (m, 3H), 7.54 - 7.32 (m, 1H), 7.25 (dd, J = 30.6, 7.7 Hz, 1H), 7.02 - 6.80 (m, 3H), 6.28 (d, J = 89.2 Hz, 1H), 4.80 - 4.55 (m, 1H), 4.26 (d, J = 3.4 Hz, 2H), 4.14 (s, 1H), 3.94 (s, 2H), 3.75 (d, J = 11.5 Hz, 3H), 3.61 (s, 9H), 3.02 - 2.81 (m, 2H), 2.62 - 2.55 (m, 2H), 2.36 - 2.19 (m, 2H), 2.04 - 1.91 (m, 2H). LCMS (ESI) calcd for C₄₃H₄₃FN₉O₄S⁺ [M+H]⁺: 800.31; found, 800.3.

### Example 475: Preparation of 2-(6,7-dihydro-5H-pyrrolo[1,2-c]imidazol-1-yl)-2-(6-(4-((1S,4S)-5-(4-(2,6-dioxopiperidin-3-yl)benzyl)-2,5-diazabicyclo[2.2.1]heptan-2-yl)phenyl)-4-fluoro-1-oxoisoindolin-2-yl)-N-(thiazol-2-yl)acetamide (GT-05115)

According to the method of step 2 of Scheme I-1, the target compound (GT-05115) was prepared (white solid, 19 mg, yield 38.0 %). ¹H NMR (400 MHz, DMSO-d6) δ 12.82 (s, 1H), 10.86 (s, 1H), 10.50 (s, 1H), 8.94 (s, 1H), 7.79 - 7.75 (m, 2H), 7.65 (t, J = 8.1 Hz, 4H), 7.52 (d, J = 3.6 Hz, 1H), 7.31 (t, J = 5.6 Hz, 3H), 6.74 (d, J = 8.7 Hz, 2H), 6.35 (s, 1H), 4.80 - 4.72 (m, 2H), 4.56 - 4.37 (m, 4H), 4.33 - 4.20 (m, 4H), 3.91 (dd, J = 11.7, 4.8 Hz, 1H), 3.74 - 3.59 (m, 3H), 3.25 (s, 3H), 2.86 (d, J = 7.1 Hz, 1H), 2.57 (s, 2H), 2.24 - 2.16 (m, 2H), 2.03 (s, 1H). LCMS (ESI) calcd for C₄₂H₄₀FN₈O₄S⁺ [M+H]⁺: 771.29; found, 771.3.

### Example 476: Preparation of 2-(6,7-dihydro-5H-pyrrolo[1,2-c]imidazol-1-yl)-2-(6-(4-((1S,4S)-5-(4-(2,4-dioxotetrahydropyrimidin-1(2H)-yl)benzyl)-2,5-diazabicyclo[2.2.1]heptan-2-yl)phenyl)-4-fluoro-1-oxoisoindolin-2-yl)-N-(thiazol-2-yl)acetamide (GT-05116)

According to the method of step 2 of Scheme I-1, the target compound (GT-05116) was prepared (white solid, 25 mg, yield 49.9 %). ¹H NMR (400 MHz, DMSO-d6) δ 12.83 (s, 1H), 10.43 (s, 2H), 8.92 (s, 1H), 7.82 - 7.76 (m, 2H), 7.72 - 7.64 (m, 4H), 7.52 (d, J = 3.6 Hz, 1H), 7.41 (d, J = 8.5 Hz, 2H), 7.32 (d, J = 3.6 Hz, 1H), 6.74 (d, J = 8.7 Hz, 2H), 6.35 (s, 1H), 4.81 - 4.70 (m, 2H), 4.55 - 4.38 (m, 3H), 4.32 - 4.19 (m, 3H), 3.81 (t, J = 6.7 Hz, 2H), 3.73 - 3.63 (m, 2H), 3.24 (d, J = 10.6 Hz, 2H), 2.90 - 2.81 (m, 1H), 2.71 (t, J = 6.6 Hz, 2H), 2.59 - 2.52 (m, 5H), 2.19 (d, J = 10.2 Hz, 1H). LCMS (ESI) calcd for C₄₁H₃₉FN₉O₄S ⁺ [M+H]⁺: 772.28; found, 772.3.

### Example 477: Preparation of 2-(6,7-dihydro-5H-pyrrolo[1,2-c]imidazol-1-yl)-2-(6-(4-((1S,4S)-5-(3-(2,4-dioxotetrahydropyrimidin-1(2H)-yl)benzyl)-2,5-diazabicyclo[2.2.1]heptan-2-yl)phenyl)-4-fluoro-1-oxoisoindolin-2-yl)-N-(thiazol-2-yl)acetamide (GT-05117)

According to the method of step 2 of Scheme I-1, the target compound (GT-05117) was prepared (white solid, 29 mg, yield 57.9 %). ¹H NMR (400 MHz, DMSO-d6) δ 10.78 (s, 1H), 10.44 (s, 1H), 9.10 (s, 1H), 8.97 (s, 1H), 7.82 - 7.74 (m, 2H), 7.72 - 7.63 (m, 3H), 7.57 - 7.51 (m, 1H), 7.47 - 7.39 (m, 2H), 6.86 (d, J = 11.4 Hz, 1H), 6.74 (d, J = 8.5 Hz, 1H), 4.80 - 4.65 (m, 2H), 4.57 - 4.39 (m, 3H), 4.36 - 4.30 (m, 1H), 4.29 - 4.22 (m, 2H), 3.84 (t, J = 6.4 Hz, 2H), 3.79 (d, J = 10.6 Hz, 1H), 3.64 (d, J = 9.2 Hz, 1H), 3.23 (s, 2H), 3.03 - 2.93 (m, 1H), 2.88 - 2.77 (m, 1H), 2.71 (t, J = 6.6 Hz, 2H), 2.62 - 2.52 (m, 4H), 2.18 (d, J = 10.5 Hz, 1H). LCMS (ESI) calcd for C₄₁H₃₉FN₉O₄S ⁺ [M+H]⁺: 772.28; found, 772.3.

### Example 478: Preparation of 2-(6,7-dihydro-5H-pyrrolo[1,2-c]imidazol-1-yl)-2-(6-(4-((1S,4S)-5-(3-((2,6-dioxopiperidin-3-yl)amino)benzyl)-2,5-diazabicyclo[2.2.1]heptan-2-yl)phenyl)-4-fluoro-1-oxoisoindolin-2-yl)-N-(thiazol-2-yl)acetamide (GT-05118)

According to the method of step 2 of Scheme I-1, the target compound (GT-05118) was prepared (white solid, 20 mg, yield 39.2 %). ¹H NMR (400 MHz, DMSO-d6) δ 10.84 (s, 1H), 9.20 (s, 2H), 9.00 (s, 1H), 7.81 - 7.75 (m, 2H), 7.65 (d, J = 8.5 Hz, 2H), 7.47 - 7.38 (m, 1H), 7.26 (d, J = 4.3 Hz, 1H), 7.14 (t, J = 7.8 Hz, 1H), 6.89 (d, J = 4.4 Hz, 1H), 6.84 (d, J = 7.1 Hz, 1H), 6.75 (t, J = 8.4 Hz, 2H), 6.16 (s, 1H), 4.71 (d, J = 17.5 Hz, 2H), 4.49 - 4.31 (m, 4H), 4.26 (t, J = 7.2 Hz, 2H), 3.84 - 3.72 (m, 2H), 3.66 - 3.58 (m, 3H), 3.24 - 3.18 (m, 2H), 3.00 - 2.95 (m, 1H), 2.85 - 2.75 (m, 2H), 2.63 - 2.55 (m, 4H), 2.16 (s, 2H). LCMS (ESI) calcd for C₄₂H₄₁FN₉O₄S ⁺ [M+H]⁺: 786.30; found, 786.3.

### Example 479: Preparation of 2-(6,7-dihydro-5H-pyrrolo[1,2-c]imidazol-1-yl)-2-(6-(4-((1S,4S)-5-((5-(2,4-dioxotetrahydropyrimidin-1(2H)-yl)pyridin-2-yl)methyl)-2,5-diazabicyclo[2.2.1]heptan-2-yl)phenyl)-4-fluoro-1-oxoisoindolin-2-yl)-N-(thiazol-2-yl)acetamide (GT-05119)

According to the method of step 2 of Scheme I-1, the target compound (GT-05119) was prepared (white solid, 23 mg, yield 45.8 %). ¹H NMR (400 MHz, DMSO-d6) δ 10.56 (s, 1H), 9.13 (s, 1H), 8.98 (s, 1H), 8.65 (d, J = 2.5 Hz, 1H), 7.88 (dd, J = 8.4, 2.5 Hz, 1H), 7.78 (d, J = 9.6 Hz, 2H), 7.69 (d, J = 8.4 Hz, 3H), 7.26 (d, J = 4.4 Hz, 1H), 6.89 (d, J = 4.4 Hz, 1H), 6.77 (t, J = 9.2 Hz, 2H), 6.15 (s, 1H), 4.80 - 4.65 (m, 3H), 4.54 (s, 2H), 4.47 (d, J = 3.4 Hz, 1H), 4.26 (t, J = 7.1 Hz, 2H), 3.86 (t, J = 6.6 Hz, 2H), 3.75 - 3.67 (m, 2H), 3.28 - 3.17 (m, 3H), 2.98 (dd, J = 15.4, 7.8 Hz, 1H), 2.85 - 2.78 (m, 1H), 2.74 (t, J = 6.6 Hz, 2H), 2.60 - 2.52 (m, 3H), 2.22 (d, J = 10.5 Hz, 1H). LCMS (ESI) calcd for C₄₀H₃₈FN₁₀O₄S ⁺ [M+H]⁺: 773.28; found, 773.3.

### Example 480: Preparation of 2-(6,7-dihydro-5H-pyrrolo[1,2-c]imidazol-1-yl)-2-(6-(4-((1S,4S)-5-((6-(2,4-dioxotetrahydropyrimidin-1(2H)-yl)pyridin-3-yl)methyl)-2,5-diazabicyclo[2.2.1]heptan-2-yl)phenyl)-4-fluoro-1-oxoisoindolin-2-yl)-N-(thiazol-2-yl)acetamide (GT-05120)

According to the method of step 2 of Scheme I-1, the target compound (GT-05120) was prepared (white solid, 21 mg, yield 41.9 %). ¹H NMR (400 MHz, DMSO-d6) δ 10.60 (s, 2H), 8.94 (s, 2H), 8.66 (s, 1H), 8.14 (d, J = 8.3 Hz, 1H), 7.83 (d, J = 8.6 Hz, 1H), 7.77 (d, J = 3.8 Hz, 2H), 7.66 (d, J = 7.6 Hz, 2H), 7.52 (d, J = 3.6 Hz, 1H), 7.32 (d, J = 3.5 Hz, 1H), 6.88 (d, J = 4.3 Hz, 1H), 6.78 - 6.71 (m, 2H), 4.79 - 4.68 (m, 2H), 4.49 - 4.44 (m, 2H), 4.38 - 4.33 (m, 1H), 4.26 - 4.21 (m, 2H), 4.08 (t, J = 6.6 Hz, 2H), 3.75 - 3.61 (m, 3H), 3.28 - 3.23 (m, 3H), 2.88 - 2.80 (m, 1H), 2.74 - 2.64 (m, 3H), 2.57 (d, J = 9.9 Hz, 3H), 2.20 (d, J = 11.9 Hz, 1H). LCMS (ESI) calcd for C₄₀H₃₈FN₁₀O₄S⁺ [M+H]⁺: 773.28; found, 773.3.

### Example 481: Preparation of 2-(6,7-dihydro-5H-pyrrolo[1,2-c]imidazol-1-yl)-2-(6-(4-(4-((2-(2,4-dioxotetrahydropyrimidin-1(2H)-yl)-5-fluoropyridin-4-yl)methyl)piperazin-1-yl)phenyl)-4-fluoro-1-oxoisoindolin-2-yl)-N-(thiazol-2-yl)acetamide (GT-07378)

According to the method of step 2 of Scheme I-1, the target compound (GT-07378) was prepared (white solid, 27 mg, yield 49.3 %). ¹H NMR (400 MHz, DMSO-d6) δ 11.38 (s, 1H), 10.66 (s, 1H), 9.08 (s, 1H), 8.57 (s, 1H), 8.14 (d, *J* = 5.3 Hz, 1H), 7.87 - 7.80 (m, 2H), 7.73 (d, *J =* 8.7 Hz, 2H), 7.53 (d, *J =* 3.6 Hz, 1H), 7.33 (d, *J* = 3.6 Hz, 1H), 7.10 (d, *J* = 8.8 Hz, 2H), 6.43 (s, 1H), 4.80 (d, *J* = 17.3 Hz, 1H), 4.59 - 4.47 (m, 3H), 4.26 (t, *J* = 7.1 Hz, 2H), 4.05 (t, *J* = 6.6 Hz, 2H), 3.97 (s, 2H), 3.45 (d, *J* = 7.1 Hz, 2H), 3.27 (s, 4H), 2.94 - 2.84 (m, 1H), 2.73 (t, *J* = 6.6 Hz, 2H), 2.68 - 2.53 (m, 4H). LCMS (ESI) calcd for C₃₉H₃₇F₂N₁₀O₄S ⁺ [M+H]⁺: 779.27; found, 779.2.

### Example 482: Preparation of 2-(6,7-dihydro-5H-pyrrolo[1,2-c]imidazol-1-yl)-2-(6-(4-(4-((2-(2,4-dioxotetrahydropyrimidin-1(2H)-yl)pyridin-4-yl)methyl)piperazin-1-yl)phenyl)-4-fluoro-1-oxoisoindolin-2-yl)-N-(thiazol-2-yl)acetamide (GT-07379)

According to the method of step 2 of Scheme I-1, the target compound (GT-07379) was prepared (white solid, 16 mg, yield 30.0 %). ¹H NMR (400 MHz, DMSO-d6) δ 11.74 (s, 1H), 10.63 (s, 1H), 9.10 (s, 1H), 8.53 (d, *J* = 5.0 Hz, 1H), 7.99 (s, 1H), 7.84 - 7.81 (m, 2H), 7.73 (d, *J* = 8.9 Hz, 2H), 7.53 (d, *J =* 3.6 Hz, 1H), 7.33 (d, *J* = 3.6 Hz, 1H), 7.10 (d, *J* = 8.9 Hz, 2H), 6.40 (s, 1H), 4.83 - 4.78 (m, 3H), 4.57 - 4.52 (m, 1H), 4.49 (d, *J* = 8.7 Hz, 2H), 4.27 - 4.25 (m, 2H), 4.08 - 4.06 (m, 2H), 3.49 - 3.40 (m, 2H), 3.36 - 3.14 (m, 4H), 2.93 - 2.81 (m, 1H), 2.74 - 2.67 (m, 4H), 2.63 - 2.56 (m, 2H). LCMS (ESI) calcd for C₃₉H₃₈FN₁₀O₄S ⁺ [M+H]⁺: 761.28; found, 761.4 .

### Example 483: Preparation of 2-(6,7-dihydro-5H-pyrrolo[1,2-c]imidazol-1-yl)-2-(6-(4-(4-((5-(2,4-dioxotetrahydropyrimidin-1(2H)-yl)pyridin-3-yl)methyl)piperazin-1-yl)phenyl)-4-fluoro-1-oxoisoindolin-2-yl)-N-(thiazol-2-yl)acetamide (GT-07380)

According to the method of step 2 of Scheme I-1, the target compound (GT-07380) was prepared (white solid, 24 mg, yield 44.9 %). ¹H NMR (400 MHz, DMSO-d6) δ 10.65 (s, 1H), 9.31 (s, 1H), 9.09 (s, 1H), 8.82 - 8.64 (m, 1H), 8.27 (s, 1H), 7.83 (t, *J* = 4.9 Hz, 2H), 7.74 (d, *J* = 8.6 Hz, 2H), 7.53 (d, *J* = 3.6 Hz, 1H), 7.33 (d, *J* = 3.6 Hz, 1H), 7.11 (d, *J* = 8.8 Hz, 2H), 6.40 (s, 1H), 4.80 (d, *J* = 17.3 Hz, 1H), 4.61 - 4.42 (m, 2H), 4.26 (t, *J* = 7.1 Hz, 2H), 3.99 - 3.90 (m, 2H), 3.81 (s, 2H), 3.51 - 3.37 (m, 4H), 3.26 (s, 4H), 2.93 - 2.83 (m, 1H), 2.77 (t, *J* = 6.6 Hz, 1H), 2.70 - 2.53 (m, 4H). LCMS (ESI) calcd for C₃₉H₃₈FN₁₀O₄S⁺ [M+H]⁺: 761.28; found, 761.2.

### Example 484: Preparation of 2-(6,7-dihydro-5H-pyrrolo[1,2-c]imidazol-1-yl)-2-(6-(4-(4-((5-(2,6-dioxopiperidin-3-yl)pyridin-2-yl)methyl)piperazin-1-yl)phenyl)-4-fluoro-1-oxoisoindolin-2-yl)-N-(thiazol-2-yl)acetamide (GT-07381)

According to the method of step 2 of Scheme I-1, the target compound (GT-07381) was prepared (white solid, 17 mg, yield 31.9 %). ¹H NMR (400 MHz, DMSO-d6) δ 10.97 (s, 1H), 9.10 (s, 1H), 8.60 (s, 1H), 7.85 (dt, *J* = 10.8, 4.3 Hz, 3H), 7.74 (d, *J* = 8.8 Hz, 2H), 7.70 (d, *J* = 8.0 Hz, 1H), 7.53 (d, *J* = 3.6 Hz, 1H), 7.33 (d, *J* = 3.6 Hz, 1H), 7.10 (d, *J* = 8.8 Hz, 2H), 6.40 (s, 1H), 4.80 (d, *J* = 17.2 Hz, 1H), 4.60 - 4.48 (m, 3H), 4.26 (t, *J* = 7.2 Hz, 2H), 4.06 (dd, *J* = 12.6, 4.8 Hz, 2H), 3.61 (s, 4H), 3.42 (s, 4H), 2.93 - 2.85 (m, 1H), 2.79 - 2.70 (m, 1H), 2.65 - 2.55 (m, 4H), 2.40 - 2.27 (m, 1H), 2.10 - 2.01 (m, 1H). LCMS (ESI) calcd for C₄₀H₃₉FN₉O₄S⁺ [M+H]⁺: 760.28; found, 760.4.

### Example 485: Preparation of 2-(6,7-dihydro-5H-pyrrolo[1,2-c]imidazol-1-yl)-2-(6-(4-(4-(((2-(2,4-dioxotetrahydropyrimidin-1(2H)-yl)-5-fluoropyridin-4-yl)methyl)(methyl)amino)piperidin-1-yl)phenyl)-4-fluoro-1-oxoisoindolin-2-yl)-N-(thiazol-2-yl)acetamide (GT-07382)

According to the method of step 2 of Scheme I-1, the target compound (GT-07382) was prepared (white solid, 19 mg, yield 35.2 %). ¹H NMR (400 MHz, DMSO-d6) δ 10.98 (s, 1H), 10.66 (s, 1H), 9.09 (s, 1H), 8.56 (s, 1H), 8.10 (d, *J* = 5.3 Hz, 1H), 7.88 - 7.79 (m, 2H), 7.72 (d, *J =* 8.7 Hz, 2H), 7.53 (d, *J* = 3.6 Hz, 1H), 7.33 (d, *J* = 3.6 Hz, 1H), 7.16 (d, *J* = 8.4 Hz, 2H), 6.40 (s, 1H), 4.80 (d, *J* = 17.2 Hz, 1H), 4.68 - 4.58 (m, 1H), 4.57 - 4.46 (m, 1H), 4.36 (s, 1H), 4.27 (t, *J =* 7.2 Hz, 2H), 4.06 - 3.99 (m, 4H), 2.94 - 2.83 (m, 3H), 2.77 = 2.70 (m, 5H), 2.65 - 2.54 (m, 4H), 2.33 (d, 1H), 2.19 (d, 1H), 2.01 - 1.86 (m, 2H). LCMS (ESI) calcd for C₄₁H₄₁F₂N₁₀O₄S ⁺ [M+H]⁺: 807.30; found, 807.4.

### Example 486: Preparation of 2-(6,7-dihydro-5H-pyrrolo[1,2-c]imidazol-1-yl)-2-(6-(4-(4-(((2-(2,4-dioxotetrahydropyrimidin-1(2H)-yl)pyridin-4-yl)methyl)(methyl)amino)piperidin-1-yl)phenyl)-4-fluoro-1-oxoisoindolin-2-yl)-N-(thiazol-2-yl)acetamide (GT-07383)

According to the method of step 2 of Scheme I-1, the target compound (GT-07383) was prepared (white solid, 7 mg, yield 13.3 %). ¹H NMR (400 MHz, DMSO-d6) δ 11.38 (s, 1H), 10.63 (s, 1H), 10.57 (s, 1H), 9.10 (s, 1H), 8.52 (d, *J* = 5.1 Hz, 1H), 8.42 (d, *J* = 5.1 Hz, 1H), 7.98 (s, 1H), 7.84 (s, 2H), 7.76 (d, *J =* 8.7 Hz, 2H), 7.65 (d, *J* = 5.1 Hz, 1H), 7.53 (d, *J* = 3.6 Hz, 1H), 7.33 (d, *J* = 3.6 Hz, 1H), 7.27 - 7.23 (m, 2H), 6.41 (s, 1H), 4.85 - 4.77 (m, 3H), 4.57 - 4.51 (m, 2H), 4.11 - 4.06 (m, 5H), 3.96 (d, *J* = 10.9 Hz, 2H), 3.48 (s, 1H), 2.99 - 2.89 (m, 2H), 2.73 - 2.67 (m, 4H), 2.66 - 2.64 (m, 2H), 2.59 - 2.54 (m, 2H), 2.41 - 2.32 (m, 1H), 2.32 - 2.24 (m, 1H), 2.07 - 1.97 (m, 1H). LCMS (ESI) C₄₁H₄₂FN₁₀O₄S ⁺ [M+H]⁺: 789.31; found, 789.2.

### Example 487: Preparation of 2-(6,7-dihydro-5H-pyrrolo[1,2-c]imidazol-1-yl)-2-(6-(4-(4-(((5-(2,4-dioxotetrahydropyrimidin-1(2H)-yl)pyridin-3-yl)methyl)(methyl)amino)piperidin-1-yl)phenyl)-4-fluoro-1-oxoisoindolin-2-yl)-N-(thiazol-2-yl)acetamide (GT-07384)

According to the method of step 2 of Scheme I-1, the target compound (GT-07384) was prepared (white solid, 32 mg, yield 60.6 %). ¹H NMR (400 MHz, DMSO-d6) δ 11.60 (s, 1H), 10.65 (s, 1H), 9.27 (s, 1H), 9.10 (s, 1H), 8.81 (d, *J* = 19.5, 5.0 Hz, 1H), 7.83 (d, *J* = 6.9 Hz, 2H), 7.79 - 7.73 (m, 2H), 7.53 (d, *J =* 3.6 Hz, 1H), 7.33 (d, *J* = 3.6 Hz, 1H), 7.31 - 7.20 (m, 2H), 6.41 (s, 1H), 4.81 (d, *J* = 17.1 Hz, 1H), 4.51 (d, 1H), 4.27 (t, *J =* 7.1 Hz, 2H), 4.12 - 4.03 (m, 4H), 3.81 - 3.73 (m, 2H), 3.55 (s, 1H), 3.22 (s, 1H), 3.03 - 2.88 (m, 3H), 2.77 (t, *J* = 6.3 Hz, 1H), 2.66 - 2.61 (m, 2H), 2.59 - 2.53 (m, 4H), 2.40 - 2.29 (m, 1H), 2.17 (d, *J =* 10.3 Hz, 1H), 2.07 - 1.96 (m, 1H), 1.87 - 1.77 (m, 1H). LCMS (ESI) calcd for C₄₁H₄₂FN₁₀O₄S ⁺ [M+H]⁺: 789.31; found, 789.3.

### Example 488: Preparation of 2-(6,7-dihydro-5H-pyrrolo[1,2-c]imidazol-1-yl)-2-(6-(4-(4-(((5-(2,6-dioxopiperidin-3-yl)pyridin-2-yl)methyl)(methyl)amino)piperidin-1-yl)phenyl)-4-fluoro-1-oxoisoindolin-2-yl)-N-(thiazol-2-yl)acetamide (GT-07385)

According to the method of step 2 of Scheme I-1, the target compound (GT-07385) was prepared (white solid, 21 mg, yield 39.8 %). ¹H NMR (400 MHz, DMSO-d6) δ 10.97 (s, 1H), 10.77 (s, 1H), 9.11 (s, 1H), 8.59 (s, 1H), 7.88 - 7.83 (m, 3H), 7.75 (q, *J* = 15.9, 8.3 Hz, 3H), 7.53 (d, *J* = 3.6 Hz, 1H), 7.33 (d, *J =* 3.6 Hz, 1H), 7.30 (s, 1H), 6.41 (s, 1H), 4.81 (d, *J* = 17.1 Hz, 1H), 4.57 - 4.48 (m, 4H), 4.22 - 4.21 (m, 2H), 4.06 (dd, *J* = 12.6, 4.8 Hz, 4H), 3.96 (d, *J* = 12.3 Hz, 2H), 3.62 - 3.53 (m, 1H), 3.02 - 2.96 (m, 1H), 2.93 - 2.86 (m, 1H), 2.75 (s, 3H), 2.62 - 2.54 (m, 4H), 2.35 - 2.29 (m, 2H), 2.09 - 2.03 (m, 2H). LCMS (ESI) calcd for C₄₂H₄₃FN₉O₄S ⁺ [M+H]⁺: 788.31; found, 788.4.

### Example 489: Preparation of 2-(6,7-dihydro-5H-pyrrolo[1,2-c]imidazol-1-yl)-2-(6-(4-((1R,4R)-5-((2-(2,4-dioxotetrahydropyrimidin-1(2H)-yl)-5-fluoropyridin-4-yl)methyl)-2,5-diazabicyclo[2.2.1]heptan-2-yl)phenyl)-4-fluoro-1-oxoisoindolin-2-yl)-N-(thiazol-2-yl)acetamide (GT-07386)

According to the method of step 2 of Scheme I-1, the target compound (GT-07386) was prepared (white solid, 15 mg, yield 21.8 %). ¹H NMR (400 MHz, DMSO-d6) δ 11.09 (s, 1H), 10.64 (s, 1H), 9.11 (s, 1H), 8.53 (s, 1H), 8.16 (d, *J* = 5.1 Hz, 1H), 7.79 (t, *J* = 4.7 Hz, 2H), 7.68 (d, *J* = 8.5 Hz, 2H), 7.53 (d, *J* = 3.6 Hz, 1H), 7.33 (d, *J* = 3.6 Hz, 1H), 6.76 (d, *J* = 8.4 Hz, 2H), 6.40 (s, 1H), 4.82 - 4.75 (m, 2H), 4.70 (d, *J* = 9.2 Hz, 1H), 4.57 - 4.51 (m, 4H), 4.26 (t, 2H), 4.02 (t, *J* = 6.6 Hz, 2H), 3.72 (dd, *J* = 44.8, 10.1 Hz, 2H), 3.59 (s, 1H), 3.35 (d, *J* = 10.5 Hz, 1H), 2.94 - 2.83 (m, 1H), 2.70 (t, *J* = 6.6 Hz, 2H), 2.62 - 2.54 (m, 3H), 2.23 (d, *J* = 11.0 Hz, 1H). LCMS (ESI) calcd for C₄₀H₃₇F₂N₁₀O₄S ⁺ [M+H]⁺: 791.27; found, 791.3.

### Example 490: Preparation of 2-(6,7-dihydro-5H-pyrrolo[1,2-c]imidazol-1-yl)-2-(6-(4-((1R,4R)-5-((2-(2,4-dioxotetrahydropyrimidin-1(2H)-yl)pyridin-4-yl)methyl)-2,5-diazabicyclo[2.2.1]heptan-2-yl)phenyl)-4-fluoro-1-oxoisoindolin-2-yl)-N-(thiazol-2-yl)acetamide (GT-07387)

According to the method of step 2 of Scheme I-1, the target compound (GT-07387) was prepared (white solid, 9 mg, yield 13.4 %). ¹H NMR (400 MHz, DMSO-d6) δ 11.07 (s, 1H), 10.62 (s, 1H), 9.10 (s, 1H), 8.48 (d, *J* = 5.0 Hz, 1H), 8.02 (s, 1H), 7.82 - 7.76 (m, 2H), 7.71 - 7.62 (m, 3H), 7.52 (t, *J* = 6.0 Hz, 1H), 7.33 (d, *J* = 3.6 Hz, 1H), 6.75 (d, *J* = 8.7 Hz, 1H), 6.44 - 6.32 (m, 1H), 5.76 (s, 1H), 4.85 - 4.73 (m, 3H), 4.54 - 4.50 (m, 2H), 4.30 - 4.25 (m, 4H), 4.06 (t, *J* = 6.6 Hz, 3H), 3.48 - 3.42 (m, 1H), 3.17 (d, *J* = 12.2 Hz, 1H), 2.93 - 2.81 (m, 1H), 2.70 (t, *J* = 6.5 Hz, 3H), 2.62 - 2.54 (m, 3H), 2.19 (d, *J* = 10.1 Hz, 1H). LCMS (ESI) calcd for C₄₀H₃₈FN₁₀O₄S⁺ [M+H]⁺: 773.28; found, 773.3.

### Example 491: Preparation of 2-(6,7-dihydro-5H-pyrrolo[1,2-c]imidazol-1-yl)-2-(6-(4-((1R,4R)-5-((5-(2,4-dioxotetrahydropyrimidin-1(2H)-yl)pyridin-3-yl)methyl)-2,5-diazabicyclo[2.2.1]heptan-2-yl)phenyl)-4-fluoro-1-oxoisoindolin-2-yl)-N-(thiazol-2-yl)acetamide (GT-07388)

According to the method of step 2 of Scheme I-1, the target compound (GT-07388) was prepared (white solid, 16 mg, yield 23.8 %). ¹H NMR (400 MHz, DMSO-d6) δ 11.48 (s, 1H), 10.71 (s, 1H), 9.11 (s, 1H), 8.88 (d, *J* = 5.5 Hz, 1H), 8.63 (s, 1H), 7.82 - 7.76 (m, 2H), 7.67 (q, *J* = 12.1, 8.8 Hz, 2H), 7.53 (d, *J =* 3.6 Hz, 1H), 7.33 (d, *J* = 3.6 Hz, 1H), 6.75 (t, *J* = 9.3 Hz, 2H), 6.41 (s, 1H), 4.76 (t, *J* = 21.3 Hz, 4H), 4.30 - 4.23 (m, 4H), 4.01 - 3.90 (m, 2H), 3.70 - 3.59 (m, 2H), 3.32 - 3.22 (m, 1H), 3.15 - 3.09 (m, 1H), 2.92 - 2.84 (m, 1H), 2.82 - 2.69 (m, 2H), 2.66 - 2.53 (m, 4H), 2.17 (q, *J* = 32.1, 10.7 Hz, 1H). LCMS (ESI) calcd for C₄₀H₃₈FN₁₀O₄S ⁺ [M+H]⁺: 773.28; found, 773.2.

### Example 492: Preparation of 2-(6,7-dihydro-5H-pyrrolo[1,2-c]imidazol-1-yl)-2-(6-(4-((1R,4R)-5-((5-(2,6-dioxopiperidin-3-yl)pyridin-2-yl)methyl)-2,5-diazabicyclo[2.2.1]heptan-2-yl)phenyl)-4-fluoro-1-oxoisoindolin-2-yl)-N-(thiazol-2-yl)acetamide (GT-07389)

According to the method of step 2 of Scheme I-1, the target compound (GT-07389) was prepared (white solid, 16 mg, yield 23.9 %). ¹H NMR (400 MHz, DMSO-d6) δ 10.95 (s, 1H), 9.10 (s, 1H), 8.53 (d, *J* = 1.8 Hz, 1H), 7.85 - 7.78 (m, 3H), 7.70 (d, *J* = 8.7 Hz, 2H), 7.64 (d, *J* = 8.1 Hz, 1H), 7.53 (d, *J* = 3.6 Hz, 1H), 7.33 (d, *J* = 3.6 Hz, 1H), 6.79 (d, *J* = 8.7 Hz, 2H), 6.40 (s, 1H), 4.82 - 4.73 (m, 2H), 4.67 (d, *J* = 13.7 Hz, 1H), 4.58 - 4.47 (m, 3H), 4.26 (t, *J* = 7.1 Hz, 2H), 4.06 - 4.02 (m, 2H), 3.72 (dd, *J* = 29.4, 10.2 Hz, 3H), 3.53 - 3.40 (m, 2H), 2.92 - 2.84 (m, 1H), 2.76 - 2.68 (m, 1H), 2.64 - 2.54 (m, 4H), 2.35 - 2.18 (m, 2H), 2.07 - 1.98 (m, 1H). LCMS (ESI) calcd for C₄₁H₃₉FN₉O₄S⁺ [M+H]⁺: 772.28; found, 772.2.

### Example 493: Preparation of 2-(6,7-dihydro-5H-pyrrolo[1,2-c]imidazol-1-yl)-2-(6-(4-((1R,4R)-5-((2-(2,6-dioxopiperidin-3-yl)pyridin-4-yl)methyl)-2,5-diazabicyclo[2.2.1]heptan-2-yl)phenyl)-4-fluoro-1-oxoisoindolin-2-yl)-N-(thiazol-2-yl)acetamide (GT-07390)

According to the method of step 2 of Scheme I-1, the target compound (GT-07390) was prepared (white solid, 3 mg, yield 4.5 %). ¹H NMR (400 MHz, DMSO-d6) δ 11.25 (s, 1H), 10.97 (s, 1H), 9.09 (s, 1H), 8.64 (d, *J* = 5.1 Hz, 1H), 7.82 - 7.77 (m, 3H), 7.67 (d, *J* = 8.5 Hz, 2H), 7.53 (d, *J* = 3.6 Hz, 1H), 7.33 (d, *J =* 3.6 Hz, 1H), 6.78 (d, *J* = 16.1 Hz, 2H), 6.39 (s, 1H), 4.79 (d, *J* = 17.6 Hz, 2H), 4.72 - 4.61 (m, 1H), 4.55 - 4.40 (m, 3H), 4.26 (t, *J* = 7.1 Hz, 2H), 4.10 (dd, *J* = 9.6, 4.9 Hz, 1H), 3.89 (d, *J* = 10.8 Hz, 2H), 3.52 - 3.38 (m, 3H), 3.22 - 3.11 (m, 1H), 2.94 - 2.83 (m, 1H), 2.66 - 2.56 (m, 4H), 2.36 - 2.25 (m, 1H), 2.24 - 2.06 (m, 2H). LCMS (ESI) C₄₁H₃₉FN₉O₄S ⁺ [M+H]⁺: 772.28; found, 772.3.

### Example 494: Preparation of 2-(6,7-dihydro-5H-pyrrolo[1,2-c]imidazol-1-yl)-2-(6-(4-((1R,4R)-5-((5-(2,6-dioxopiperidin-3-yl)pyridin-3-yl)methyl)-2,5-diazabicyclo[2.2.1]heptan-2-yl)phenyl)-4-fluoro-1-oxoisoindolin-2-yl)-N-(thiazol-2-yl)acetamide (GT-07391)

According to the method of step 2 of Scheme I-1, the target compound (GT-07391) was prepared (white solid, 6 mg, yield 8.9 %). ¹H NMR (400 MHz, DMSO-d6) δ 11.31 (s, 1H), 11.05 (s, 1H), 9.06 (d, *J* = 25.4 Hz, 2H), 8.76 (s, 1H), 8.56 (s, 1H), 7.83 - 7.77 (m, 2H), 7.71 - 7.63 (m, 2H), 7.53 (d, *J* = 3.6 Hz, 1H), 7.33 (d, *J* = 3.5 Hz, 1H), 6.75 (t, *J* = 8.7 Hz, 2H), 6.39 (s, 1H), 4.76 (t, *J* = 19.2 Hz, 3H), 4.53 - 4.48 (m, 2H), 4.26 (t, *J* = 7.0 Hz, 3H), 4.16 - 4.10 (m, 2H), 3.66 (d, *J* = 8.2 Hz, 2H), 3.43 - 3.26 (m, 2H), 2.95 - 2.86 (m, 1H), 2.83 - 2.70 (m, 1H), 2.65 - 2.56 (m, 4H), 2.43 - 2.03 (m, 4H). LCMS (ESI) calcd for C₄₁H₃₉FN₉O₄S ⁺ [M+H]⁺: 772.28; found, 772.4.

### Example 495: Preparation of 2-(6,7-dihydro-5H-pyrrolo[1,2-c]imidazol-1-yl)-2-(6-(4-(4-((2-(2,6-dioxopiperidin-3-yl)pyridin-4-yl)methyl)piperazin-1-yl)phenyl)-4-fluoro-1-oxoisoindolin-2-yl)-N-(thiazol-2-yl)acetamide (GT-07392)

According to the method of step 2 of Scheme I-1, the target compound (GT-07392) was prepared (white solid, 8 mg, yield 12.0 %). ¹H NMR (400 MHz, DMSO-d6) δ 11.93 (s, 1H), 10.96 (s, 1H), 9.10 (s, 1H), 8.69 (d, *J* = 5.8 Hz, 1H), 7.86 - 7.81 (m, 2H), 7.79 - 7.71 (m, 4H), 7.53 (d, *J* = 3.6 Hz, 1H), 7.33 (d, *J =* 3.6 Hz, 1H), 7.10 (d, *J* = 8.9 Hz, 2H), 6.40 (s, 1H), 4.80 (d, *J* = 17.2 Hz, 1H), 4.57 - 4.46 (m, 3H), 4.26 (t, *J* = 7.1 Hz, 2H), 4.16 - 4.11 (m, 1H), 3.92 (s, 2H), 3.48 - 3.41 (m, 3H), 3.36 - 3.27 (m, 2H), 3.23 (s, 2H), 2.95 - 2.83 (m, 1H), 2.65 - 2.54 (m, 4H), 2.36 - 2.27 (m, 1H), 2.25 - 2.15 (m, 1H). LCMS (ESI) calcd for C₄₀H₃₉FN₉O₄S ⁺ [M+H]⁺: 760.28; found, 760.4.

### Example 496: Preparation of 2-(6,7-dihydro-5H-pyrrolo[1,2-c]imidazol-1-yl)-2-(6-(4-(4-((5-(2,6-dioxopiperidin-3-yl)pyridin-3-yl)methyl)piperazin-1-yl)phenyl)-4-fluoro-1-oxoisoindolin-2-yl)-N-(thiazol-2-yl)acetamide (GT-07472)

According to the method of step 2 of Scheme I-1, the target compound (GT-07472) was prepared (white solid, 21 mg, yield 31.4 %). ¹H NMR (400 MHz, DMSO-d6) δ 12.16 (s, 1H), 11.06 (s, 1H), 9.12 (s, 1H), 9.03 (s, 1H), 8.84 (d, *J =* 16.1 Hz, 1H), 8.60 (s, 1H), 7.83 (t, *J =* 4.9 Hz, 2H), 7.74 (d, *J =* 8.7 Hz, 2H), 7.53 (d, *J =* 3.6 Hz, 1H), 7.33 (d, *J* = 3.6 Hz, 1H), 7.11 (d, *J =* 8.8 Hz, 2H), 6.41 (s, 1H), 4.81 (d, *J =* 17.2 Hz, 1H), 4.53 (q, *J =* 32.7, 15.5 Hz, 4H), 4.00 - 3.91 (m, 4H), 3.51 - 3.41 (m, 2H), 3.35 - 3.18 (m, 4H), 2.93 - 2.85 (m, 1H), 2.83 - 2.73 (m, 1H), 2.67 - 2.53 (m, 4H), 2.42 - 2.30 (m, 1H), 2.22 - 2.13 (m, 1H). LCMS (ESI) calcd for C₄₀H₃₉FN₉O₄S ⁺ [M+H]⁺: 760.28; found, 760.3.

### Example 497: Preparation of 2-(6,7-dihydro-5H-pyrrolo[1,2-c]imidazol-1-yl)-2-(6-(4-(4-(((2-(2,6-dioxopiperidin-3-yl)pyridin-4-yl)methyl)(methyl)amino)piperidin-1-yl)phenyl)-4-fluoro-1-oxoisoindolin-2-yl)-N-(thiazol-2-yl)acetamide (GT-07473)

According to the method of step 2 of Scheme I-1, the target compound (GT-07473) was prepared (white solid, 11 mg, yield 16.7 %). ¹H NMR (400 MHz, DMSO-d6) δ 11.66 (s, 1H), 10.99 (s, 1H), 9.11 (s, 1H), 8.72 (d, *J =* 5.0 Hz, 1H), 7.93 - 7.82 (m, 4H), 7.77 (d, *J =* 8.4 Hz, 2H), 7.53 (d, *J =* 3.6 Hz, 1H), 7.33 (d, *J* = 3.6 Hz, 1H), 7.28 (s, 2H), 6.41 (s, 1H), 4.81 (d, *J =* 17.1 Hz, 1H), 4.61 (d, *J =* 12.6 Hz, 1H), 4.57 - 4.47 (m, 2H), 4.38 (s, 2H), 4.27 (t, *J =* 7.1 Hz, 4H), 4.22 - 4.17 (m, 3H), 3.47 (s, 2H), 3.03 - 2.92 (m, 2H), 2.65 - 2.60 (m, 4H), 2.43 - 2.30 (m, 3H), 2.11 - 1.97 (m, 2H). LCMS (ESI) calcd for C₄₂H₄₃FN₉O₄S⁺ [M+H]⁺: 788.31; found, 788.3.

### Example 498: Preparation of 2-(6,7-dihydro-5H-pyrrolo[1,2-c]imidazol-1-yl)-2-(6-(4-(4-(((5-(2,6-dioxopiperidin-3-yl)pyridin-3-yl)methyl)(methyl)amino)piperidin-1-yl)phenyl)-4-fluoro-1-oxoisoindolin-2-yl)-N-(thiazol-2-yl)acetamide (GT-07393)

According to the method of step 2 of Scheme I-1, the target compound (GT-07393) was prepared (white solid, 12 mg, yield 18.2 %). ¹H NMR (400 MHz, DMSO-d6) δ 11.74 (s, 1H), 11.15 - 11.03 (m, 1H), 9.09 (d, *J =* 16.9 Hz, 2H), 8.84 (s, 1H), 8.64 (s, 1H), 7.84 (t, *J =* 4.7 Hz, 2H), 7.76 (d, *J =* 8.6 Hz, 2H), 7.53 (d, *J* = 3.6 Hz, 1H), 7.33 (d, *J =* 3.6 Hz, 1H), 7.25 (d, *J =* 7.5 Hz, 2H), 6.41 (s, 1H), 4.81 (d, *J* = 17.1 Hz, 1H), 4.68 (d, *J =* 12.4 Hz, 1H), 4.55 - 4.45 (m, 2H), 4.29 - 4.19 (m, 6H), 3.57 (s, 2H), 2.99 - 2.84 (m, 3H), 2.67 - 2.61 (m, 4H), 2.59 - 2.55 (m, 2H), 2.41 - 2.28 (m, 3H), 2.22 - 2.13 (m, 1H), 2.03 (s, 2H). LCMS (ESI) calcd for C₄₂H₄₃FN₉O₄S⁺ [M+H]⁺: 788.31; found, 788.4.

### Example 499: Preparation of 2-(6,7-dihydro-5H-pyrrolo[1,2-c]imidazol-1-yl)-2-(6-(4-(4-((2-((2,6-dioxopiperidin-3-yl)amino)pyridin-3-yl)methyl)piperazin-1-yl)phenyl)-4-fluoro-1-oxoisoindolin-2-yl)-N-(thiazol-2-yl)acetamide (GT-07489)

According to the method of step 2 of Scheme I-1, the target compound (GT-07489) was prepared (white solid, 26 mg, yield 38.1 %). ¹H NMR (400 MHz, DMSO-d6) δ 11.65 (s, 1H), 11.10 (s, 1H), 9.16 (s, 1H), 8.68 (s, 1H), 8.34 (s, 1H), 8.16 (d, *J* = 5.5 Hz, 1H), 7.89 (t, *J =* 4.8 Hz, 2H), 7.79 (d, *J =* 8.8 Hz, 2H), 7.59 *(d, J* = 3.6 Hz, 1H), 7.39 (d, *J =* 3.6 Hz, 1H), 7.17 (d, *J =* 8.9 Hz, 2H), 7.05 (d, *J =* 5.3 Hz, 1H), 6.46 (s, 1H), 5.32 (s, 1H), 4.86 (d, *J =* 17.1 Hz, 1H), 4.75 (s, 2H), 4.58 (d, *J =* 17.2 Hz, 1H), 4.32 (t, *J =* 7.1 Hz, 3H), 3.55 (s, 6H), 3.35 (s, 3H), 2.94 (dd, *J =* 14.2, 6.9 Hz, 1H), 2.87 - 2.78 (m, 1H), 2.75 - 2.61 (m, 4H), 2.26 (s, 1H). LCMS (ESI) calcd for C₄₀H₄₀FN₁₀O₄S⁺ [M+H]⁺: 775.29; found, 775.4.

### Example 500: Preparation of 2-(6,7-dihydro-5H-pyrrolo[1,2-c]imidazol-1-yl)-2-(6-(4-(4-((5-(2,6-dioxopiperidin-3-yl)-2-fluoropyridin-3-yl)methyl)piperazin-1-yl)phenyl)-4-fluoro-1-oxoisoindolin-2-yl)-N-(thiazol-2-yl)acetamide (GT-07490)

According to the method of step 2 of Scheme I-1, the target compound (GT-07490) was prepared (yellow solid, 18 mg, yield 33.0 %). ¹H NMR (400 MHz, DMSO-d6) δ 11.82 (s, 1H), 11.00 (s, 1H), 9.11 (s, 1H), 8.35 (d, *J* = 9.0 Hz, 1H), 8.26 (s, 1H), 7.83 (t, *J =* 5.0 Hz, 2H), 7.73 (d, *J* = 8.6 Hz, 2H), 7.53 (d, *J =* 3.5 Hz, 1H), 7.33 (d, *J* = 3.5 Hz, 1H), 7.10 (d, *J* = 8.6 Hz, 2H), 6.41 (s, 1H), 4.80 (d, *J* = 17.1 Hz, 1H), 4.55 - 4.45 (m, 3H), 4.26 (t, *J =* 7.0 Hz, 2H), 4.07 (dd, *J =* 12.5, 4.7 Hz, 2H), 3.46 (d, *J =* 23.3 Hz, 3H), 3.29 (s, 4H), 2.93 - 2.85 (m, 1H), 2.80 - 2.72 (m, 1H), 2.66 - 2.54 (m, 5H), 2.36 - 2.25 (m, 1H), 2.15 - 2.07 (m, 1H). LCMS (ESI) calcd for C₄₀H₃₈F₂N₉O₄S⁺ [M+H]⁺: 778.27; found, 778.3.

### Example 501: Preparation of 2-(6,7-dihydro-5H-pyrrolo[1,2-c]imidazol-1-yl)-2-(6-(4-(4-(((5-(2,6-dioxopiperidin-3-yl)-2-fluoropyridin-3-yl)methyl)(methyl)amino)piperidin-1-yl)phenyl)-4-fluoro-1-oxoisoindolin-2-yl)-N-(thiazol-2-yl)acetamide (GT-07560)

According to the method of step 2 of Scheme I-1, the target compound (GT-07560) was prepared (white solid, 18 mg, yield 33.4 %). ¹H NMR (400 MHz, DMSO-d6) δ 11.29 (d, 1H), 10.99 (s, 1H), 9.11 (s, 1H), 8.32 (s, 1H), 8.25 (s, 1H), 7.83 (s, 2H), 7.75 (s, 2H), 7.53 (d, *J* = 3.6 Hz, 1H), 7.33 (d, *J* = 3.6 Hz, 1H), 7.24 (s, 2H), 6.41 (s, 1H), 4.81 (d, *J =* 17.2 Hz, 1H), 4.60 - 4.48 (m, 3H), 4.29 - 4.24 (m, 4H), 3.95 - 3.92 (m, 2H), 3.57 - 3.51 (m, 1H), 2.98 - 2.87 (m, 3H), 2.70 - 2.63 (m, 4H), 2.60 - 2.54 (m, 3H), 2.38 - 2.24 (m, 3H), 2.14 - 1.93 (m, 4H). LCMS (ESI) calcd for C₄₂H₄₂F₂N₉O₄S⁺ [M+H]⁺: 806.30; found, 806.3.

### Example 502: Preparation of 2-(6,7-dihydro-5H-pyrrolo[1,2-c]imidazol-1-yl)-2-(6-(4-(4-(((2-((2,6-dioxopiperidin-3-yl)amino)pyridin-3-yl)methyl)(methyl)amino)piperidin-1-yl)phenyl)-4-fluoro-1-oxoisoindolin-2-yl)-N-(thiazol-2-yl)acetamide (GT-07561)

According to the method of step 2 of Scheme I-1, the target compound (GT-07561) was prepared (white solid, 8.6 mg, yield 16.0 %). ¹H NMR (500 MHz, DMSO) δ 11.18 (d, J = 27.3 Hz, 1H), 11.05 (s, 1H), 9.11 (s, 1H), 8.89 - 8.49 (m, 1H), 8.28 (s, 1H), 8.10 (d, J = 5.5 Hz, 1H), 7.86 - 7.81 (m, 2H), 7.76 (d, J = 7.7 Hz, 2H), 7.53 (d, J = 3.6 Hz, 1H), 7.33 (d, J = 3.6 Hz, 1H), 7.30 - 7.18 (m, 2H), 6.99 (s, 1H), 6.41 (s, 1H), 5.24 (s, 1H), 4.80 (d, J = 17.1 Hz, 1H), 4.66 (s, 3H), 4.53 (d, J = 17.1 Hz, 2H), 4.26 (t, J = 7.2 Hz, 3H), 3.96 (d, J = 8.2 Hz, 2H), 3.71 - 3.60 (m, 1H), 2.96 (s, 1H), 2.91 - 2.85 (m, 1H), 2.80 - 2.65 (m, 5H), 2.62 - 2.54 (m, 2H), 2.39 - 2.32 (m, 2H), 2.20 - 2.01 (m, 3H). LCMS (ESI) calcd for C₄₂H₄₄FN₁₉O₄S⁺ [M+H]⁺: 803.32; found, 803.2.

### Example 503: Preparation of 2-(6,7-dihydro-5H-pyrrolo[1,2-c]imidazol-1-yl)-2-(6-(4-((1R,4R)-5-((5-(2,6-dioxopiperidin-3-yl)-2-fluoropyridin-3-yl)methyl)-2,5-diazabicyclo[2.2.1]heptan-2-yl)phenyl)-4-fluoro-1-oxoisoindolin-2-yl)-N-(thiazol-2-yl)acetamide (GT-07562)

According to the method of step 2 of Scheme I-1, the target compound (GT-07562) was prepared (white solid, 6 mg, yield 11.0 %). ¹H NMR (400 MHz, DMSO-d6) δ 11.16 - 10.90 (m, 2H), 9.10 (s, 1H), 8.36 (d, *J = 9.2* Hz, 1H), 8.19 (d, *J* = 19.9 Hz, 1H), 7.84 - 7.76 (m, 2H), 7.67 (d, *J* = 8.5 Hz, 2H), 7.53 (d, *J =* 3.3 Hz, 1H), 7.33 (d, *J* = 3.4 Hz, 1H), 6.75 (d, *J =* 8.2 Hz, 1H), 6.40 (s, 1H), 5.77 - 5.62 (m, 1H), 4.85 - 4.74 (m, 2H), 4.61 - 4.39 (m, 4H), 4.26 (t, *J* = 6.7 Hz, 2H), 4.09 - 3.93 (m, 2H), 3.78 - 3.73 (m, 2H), 3.33 (s, 1H), 2.94 - 2.85 (m, 1H), 2.78 - 2.71 (m, 1H), 2.66 - 2.55 (m, 4H), 2.34 - 2.00 (m, 4H). LCMS (ESI) C₄₁H₃₈F₂N₉O₄S ⁺ [M+H]⁺: 790.27; found, 790.3.

### Example 504: Preparation of 1-(5-((4-(4-(2-(1-(6,7-dihydro-5H-pyrrolo[1,2-c]imidazol-1-yl)-2-(thiazol-2-ylamino)ethyl)-7-fluoro-3-oxoisoindolin-5-yl)phenyl)piperazin-1-yl)methyl)-6-fluoropyridin-3-yl)dihydropyrimidine-2,4(1H,3H)-dione (GT-07760)

According to the method of step 2 of Scheme I-1, the target compound (GT-07760) was prepared (white solid, 20 mg, yield 36.6 %). ¹H NMR (400 MHz, DMSO-d6) δ 11.80 (s, 1H), 10.63 (s, 1H), 9.10 (s, 1H), 8.45 - 8.39 (m, 2H), 7.83 (t, *J* = 5.3, 4.3 Hz, 2H), 7.74 (d, *J* = 8.8 Hz, 2H), 7.53 (d, *J* = 3.6 Hz, 1H), 7.33 (d, *J* = 3.6 Hz, 1H), 7.10 (d, *J =* 8.9 Hz, 2H), 6.40 (s, 1H), 4.82 (s, 1H), 4.49 (d, *J* = 7.6 Hz, 3H), 4.26 (t, *J* = 7.1 Hz, 3H), 3.94 (t, *J* = 6.6 Hz, 3H), 3.50 (s, 2H), 3.30 (s, 3H), 2.93 - 2.84 (m, 1H), 2.76 (t, *J* = 6.8 Hz, 3H), 2.67 - 2.55 (m, 3H). LCMS (ESI) calcd for C₃₉H₃₇F₂N₁₀O₄S ⁺ [M+H]⁺: 779.27; found, 779.1.

### Example 505: Preparation of 2-(6,7-dihydro-5H-pyrrolo[1,2-c]imidazol-1-yl)-2-(6-(4-(4-((5-(2,4-dioxotetrahydropyrimidin-1(2H)-yl)pyrazin-2-yl)methyl)piperazin-1-yl)phenyl)-4-fluoro-1-oxoisoindolin-2-yl)-N-(thiazol-2-yl)acetamide (GT-07761)

According to the method of step 2 of Scheme I-1, the target compound (GT-07761) was prepared (white solid, 16 mg, yield 29.9 %). ¹H NMR (400 MHz, DMSO-d6) δ 11.62 (s, 1H), 10.83 (s, 1H), 9.16 (d, *J* = 1.4 Hz, 1H), 9.11 (s, 1H), 8.80 (d, *J* = 2.5 Hz, 1H), 7.89 - 7.80 (m, 2H), 7.74 (d, *J* = 8.9 Hz, 2H), 7.53 (d, *J* = 3.6 Hz, 1H), 7.33 (d, *J* = 3.6 Hz, 1H), 7.10 (d, *J* = 8.9 Hz, 2H), 6.44 (s, 1H), 4.83 (t, 1H), 4.62 - 4.50 (m, 3H), 4.27 (t, *J* = 7.1 Hz, 2H), 4.10 (t, 3H), 3.96 (d, *J* = 9.1 Hz, 2H), 3.61 - 3.50 (m, 2H), 3.38 - 3.20 (m, 4H), 2.94 - 2.83 (m, 1H), 2.75 (t, *J* = 6.6 Hz, 2H), 2.66 - 2.53 (m, 3H). LCMS (ESI) calcd for C₃₈H₃₇FN₁₁O₄S⁺ [M+H]⁺: 762.27; found, 762.2.

### Example 506: Preparation of 2-(6,7-dihydro-5H-pyrrolo[1,2-c]imidazol-1-yl)-2-(6-(4-(4-(((5-(2,4-dioxotetrahydropyrimidin-1(2H)-yl)-2-fluoropyridin-3-yl)methyl)(methyl)amino)piperidin-1-yl)phenyl)-4-fluoro-1-oxoisoindolin-2-yl)-N-(thiazol-2-yl)acetamide (GT-07823)

According to the method of step 2 of Scheme I-1, the target compound (GT-07823) was prepared (white solid, 27 mg, yield 49.2 %). ¹H NMR (400 MHz, DMSO-d6) δ 11.36 (s, 1H), 10.62 (s, 1H), 9.11 (s, 1H), 8.48 - 8.35 (m, 2H), 7.84 (t, *J* = 4.8 Hz, 2H), 7.75 (d, *J* = 8.5 Hz, 2H), 7.53 (d, *J* = 3.6 Hz, 1H), 7.33 (d, *J* = 3.6 Hz, 1H), 7.22 (d, *J* = 7.8 Hz, 2H), 6.41 (s, 1H), 4.81 (d, *J* = 17.1 Hz, 1H), 4.59 - 4.48 (m, 2H), 4.27 (t, *J* = 7.1 Hz, 3H), 3.92 (t, *J* = 5.4 Hz, 5H), 3.56 (s, 1H), 2.98 - 2.86 (m, 3H), 2.76 (t, *J* = 6.8 Hz, 2H), 2.70 (s, 3H), 2.64 - 2.53 (m, 3H), 2.37 (d, J = 11.7 Hz, 1H), 2.26 (d, J = 10.3 Hz, 1H), 2.06 - 1.93 (m, 2H). LCMS (ESI) calcd for C₄₁H₄₁F₂N₁₀O₄S ⁺ [M+H]⁺: 807.30; found, 807.2.

### Example 507: Preparation of 2-(6,7-dihydro-5H-pyrrolo[1,2-c]imidazol-1-yl)-2-(6-(4-(4-(((5-(2,4-dioxotetrahydropyrimidin-1(2H)-yl)pyrazin-2-yl)methyl)(methyl)amino)piperidin-1-yl)phenyl)-4-fluoro-1-oxoisoindolin-2-yl)-N-(thiazol-2-yl)acetamide (GT-07824)

According to the method of step 2 of Scheme I-1, the target compound (GT-07824) was prepared (white solid, 18 mg, yield 33.5 %). ¹H NMR (400 MHz, DMSO-d6) δ 11.30 (s, 1H), 10.82 (s, 1H), 9.17 (d, *J* = 1.4 Hz, 1H), 9.11 (s, 1H), 8.81 (d, *J =* 1.3 Hz, 1H), 7.84 (t, *J =* 4.8 Hz, 2H), 7.75 (d, *J =* 8.6 Hz, 2H), 7.53 (d, *J* = 3.6 Hz, 1H), 7.33 (d, *J =* 3.6 Hz, 1H), 7.24 (d, *J =* 7.0 Hz, 2H), 6.41 (s, 1H), 4.81 (d, *J* = 17.1 Hz, 1H), 4.66 - 4.46 (m, 3H), 4.27 (t, *J =* 7.1 Hz, 2H), 4.10 (t, *J =* 6.6 Hz, 3H), 3.96 - 3.95 (m, 2H), 3.58 - 3.51 (m, 1H), 3.01 - 2.85 (m, 3H), 2.78 - 2.72 (m, 5H), 2.66 - 2.53 (m, 3H), 2.41 - 2.22 (m, 2H), 2.01 (s, 2H). LCMS (ESI) calcd for C₄₀H₄₁FN₁₁O₄S ⁺ [M+H]⁺: 790.30; found, 790.2.

### Example 508: Preparation of 2-(6,7-dihydro-5H-pyrrolo[1,2-c]imidazol-1-yl)-2-(6-(4-((1R,4R)-5-((5-(2,4-dioxotetrahydropyrimidin-1(2H)-yl)-2-fluoropyridin-3-yl)methyl)-2,5-diazabicyclo[2.2.1]heptan-2-yl)phenyl)-4-fluoro-1-oxoisoindolin-2-yl)-N-(thiazol-2-yl)acetamide (GT-07825)

According to the method of step 2 of Scheme I-1, the target compound (GT-07825) was prepared (white solid, 14 mg, yield 25.6 %). ¹H NMR (400 MHz, DMSO-d6) δ 11.14 (s, 1H), 10.66 (s, 1H), 9.13 (s, 1H), 8.50 (d, *J =* 8.2 Hz, 1H), 8.41 (s, 1H), 7.89 - 7.81 (m, 2H), 7.73 (d, *J =* 8.6 Hz, 2H), 7.59 (d, *J =* 3.6 Hz, 1H), 7.39 (d, *J =* 3.6 Hz, 1H), 6.81 (d, *J =* 8.5 Hz, 1H), 6.45 (s, 1H), 4.91 - 4.80 (m, 2H), 4.68 (d, *J =* 8.8 Hz, 1H), 4.59 - 4.48 (m, 3H), 4.32 (t, *J =* 7.0 Hz, 2H), 3.94 (t, *J =* 6.7 Hz, 2H), 3.83 - 3.80 (m, 1H), 3.71 (s, 4H), 3.40 (d, *J* = 9.4 Hz, 1H), 2.93 (dd, *J =* 14.1, 6.3 Hz, 1H), 2.80 (t, *J =* 6.6 Hz, 2H), 2.66 - 2.59 (m, 3H), 2.29 (d, *J* = 10.3 Hz, 1H). LCMS (ESI) calcd for C₄₀H₃₇F₂N₁₀O₄S ⁺ [M+H]⁺: 791.27; found, 791.1.

### Example 509: Preparation of 2-(6,7-dihydro-5H-pyrrolo[1,2-c]imidazol-1-yl)-2-(6-(4-((1R,4R)-5-((5-(2,4-dioxotetrahydropyrimidin-1(2H)-yl)pyrazin-2-yl)methyl)-2,5-diazabicyclo[2.2.1]heptan-2-yl)phenyl)-4-fluoro-1-oxoisoindolin-2-yl)-N-(thiazol-2-yl)acetamide (GT-07826)

According to the method of step 2 of Scheme I-1, the target compound (GT-07826) was prepared (white solid, 15 mg, yield 28.1 %). ¹H NMR (400 MHz, DMSO-d6) δ 11.16 (s, 1H), 10.86 (s, 1H), 9.15 (d, *J* = 9.5 Hz, 3H), 8.80 (s, 1H), 7.85 (t, *J =* 4.8 Hz, 2H), 7.73 (d, *J =* 8.7 Hz, 2H), 7.59 (d, *J =* 3.6 Hz, 1H), 7.39 (d, *J* = 3.6 Hz, 1H), 6.82 (d, *J =* 8.6 Hz, 2H), 6.45 (s, 1H), 4.89 - 4.73 (m, 4H), 4.32 (t, *J =* 7.0 Hz, 3H), 4.13 (t, *J =* 6.6 Hz, 3H), 3.84 - 3.77 (m, 2H), 3.72 (d, *J =* 9.0 Hz, 2H), 3.47 (d, *J =* 10.9 Hz, 2H), 2.97 - 2.91 (m, 1H), 2.79 (t, *J* = 6.6 Hz, 3H), 2.68 - 2.63 (m, 2H), 2.30 - 2.25 (m, 1H). LCMS (ESI) C₃₉H₃₇FN₁₁O₄S ⁺ [M+H]⁺: 774.27; found, 774.2.

### Example 510: Preparation of 2-(6,7-dihydro-5H-pyrrolo[1,2-c]imidazol-1-yl)-2-(6-(4-(4-(((2-(2,6-dioxopiperidin-3-yl)-5-fluoropyridin-4-yl)methyl)(methyl)amino)piperidin-1-yl)phenyl)-4-fluoro-1-oxoisoindolin-2-yl)-N-(thiazol-2-yl)acetamide (GT-08069)

According to the method of step 2 of Scheme I-1, the target compound (GT-08069) was prepared (reddish-brown solid, 11 mg, yield 20 %). ¹H NMR (400 MHz, DMSO-d6) δ 11.54 (s, 1H), 10.93 (s, 1H), 9.11 (s, 1H), 8.64 (s, 1H), 7.94 (s, 1H), 7.88 - 7.81 (m, 2H), 7.80 - 7.74 (m, 2H), 7.53 (d, J = 3.6 Hz, 1H), 7.33 (d, J = 3.6 Hz, 1H), 7.31 - 7.26 (m, 1H), 6.41 (s, 1H), 4.81 (d, J = 17.1 Hz, 1H), 4.61 (d, J = 15.3 Hz, 2H), 4.55 (s, 1H), 4.51 - 4.48 (m, 3H), 4.28 - 4.24 (m, 3H), 4.09 (dd, J = 10.0, 5.3 Hz, 2H), 3.97 (d, J = 11.0 Hz, 2H), 3.57 (s, 1H), 3.01 (s, 1H), 2.93 - 2.85 (m, 1H), 2.70 (s, 3H), 2.59 - 2.54 (m, 3H), 2.38 (s, 1H), 2.33 - 2.25 (m, 2H), 2.18 - 2.05 (m, 3H). LCMS (ESI) calcd for C₄₂H₄₂F₂N₉O₄S ⁺ [M+H]⁺: 806.30; found, 806.2.

### Example 511: Preparation of 2-(6,7-dihydro-5H-pyrrolo[1,2-c]imidazol-1-yl)-2-(6-(4-((1R,4R)-5-((2-(2,6-dioxopiperidin-3-yl)-5-fluoropyridin-4-yl)methyl)-2,5-diazabicyclo[2.2.1]heptan-2-yl)phenyl)-4-fluoro-1-oxoisoindolin-2-yl)-N-(thiazol-2-yl)acetamide (GT-08070)

According to the method of step 2 of Scheme I-1, the target compound (GT-08070) was prepared (reddish-brown solid, 27 mg, yield 49 %). ¹H NMR (400 MHz, DMSO-d6) δ 11.44 (s, 1H), 10.86 (s, 1H), 9.08 (s, 1H), 8.62 (s, 1H), 7.94 (d, J = 8.4 Hz, 1H), 7.84 - 7.75 (m, 2H), 7.68 (d, J = 8.4 Hz, 2H), 7.53 (d, J = 3.6 Hz, 1H), 7.33 (d, J = 3.6 Hz, 1H), 6.89 - 6.66 (m, 2H), 6.41 (s, 1H), 4.83 - 4.74 (m, 2H), 4.74 - 4.61 (m, 1H), 4.56 - 4.46 (m, 3H), 4.26 (t, J = 7.0 Hz, 2H), 4.09 - 4.03 (m, 2H), 3.77 - 3.70 (m, 2H), 3.64 (d, J = 9.9 Hz, 2H), 3.37 - 3.24 (m, 1H), 2.97 - 2.82 (m, 1H), 2.71 - 2.54 (m, 5H), 2.29 - 2.18 (m, 2H), 2.18 - 2.09 (m, 1H). LCMS (ESI) calcd for C₄₁H₃₈F₂N₉O₄S ⁺ [M+H]⁺: 790.27; found, 790.1.

### Example 512: Preparation of 2-(6,7-dihydro-5H-pyrrolo[1,2-c]imidazol-1-yl)-2-(6-(4-(4-(((6-(2,4-dioxotetrahydropyrimidin-1(2H)-yl)pyrazin-2-yl)methyl)(methyl)amino)piperidin-1-yl)phenyl)-4-fluoro-1-oxoisoindolin-2-yl)-N-(thiazol-2-yl)acetamide (GT-07919)

According to the method of step 2 of Scheme I-1, the target compound (GT-07919) was prepared (white solid, 12 mg, yield 22 %). ¹H NMR (400 MHz, DMSO-d6) δ 11.62 (s, 1H), 10.81 (s, 1H), 9.13 (d, *J* = 6.2 Hz, 2H), 8.65 (s, 1H), 7.93 - 7.76 (m, 4H), 7.53 (d, *J* = 3.5 Hz, 1H), 7.33 (d, *J* = 3.6 Hz, 3H), 6.42 (s, 1H), 4.84 (s, 1H), 4.62 - 4.48 (m, 3H), 4.26 (t, *J* = 7.3 Hz, 2H), 4.23 - 4.17 (m, 1H), 4.17 - 4.09 (m, 1H), 3.94 (d, *J* = 10.2 Hz, 2H), 3.62 (s, 1H), 3.07 (s, 2H), 2.93 - 2.84 (m, 1H), 2.80 (d, *J* = 3.6 Hz, 3H), 2.75 (t, *J* = 6.7 Hz, 2H), 2.64 - 2.54 (m, 3H), 2.43 (s, 2H), 2.29 (s, 1H), 2.12 (s, 2H). LCMS (ESI) calcd for C₄₀H₄₁FN₁₁O₄S⁺ [M+H]⁺: 790.30; found, 790.30.

### Example 513: Preparation of 2-(6,7-dihydro-5H-pyrrolo[1,2-c]imidazol-1-yl)-2-(6-(4-(4-(((5-(2,4-dioxotetrahydropyrimidin-1(2H)-yl)-3-fluoropyridin-2-yl)methyl)(methyl)amino)piperidin-1-yl)phenyl)-4-fluoro-1-oxoisoindolin-2-yl)-N-(thiazol-2-yl)acetamide (GT-07920)

According to the method of step 2 of Scheme I-1, the target compound (GT-07920) was prepared (white solid, 8 mg, yield 15 %). ¹H NMR (400 MHz, DMSO-d6) δ 10.82 (s, 1H), 10.68 (s, 1H), 9.11 (s, 1H), 8.62 (s, 1H), 7.96 (dd, *J* = 11.2, 1.9 Hz, 1H), 7.83 (t, *J* = 4.8 Hz, 2H), 7.75 (d, *J* = 8.7 Hz, 2H), 7.53 (d, *J =* 3.6 Hz, 1H), 7.33 (d, *J* = 3.6 Hz, 1H), 7.24 (s, 2H), 6.41 (s, 1H), 4.81 (d, *J* = 17.2 Hz, 1H), 4.66 (s, 1H), 4.55 - 4.48 (m, 2H), 4.29 - 4.24 (m, 4H), 3.98 (d, *J* = 12.0 Hz, 2H), 3.92 (t, *J* = 6.6 Hz, 2H), 3.61 (s, 1H), 3.01 - 2.86 (m, 3H), 2.83 (s, 3H), 2.76 (t, *J* = 6.6 Hz, 2H), 2.64 - 2.56 (m, 2H), 2.29 (d, *J* = 29.1 Hz, 2H), 2.01 (s, 2H). LCMS (ESI) C₄₁H₄₁F₂N₁₀O₄S ⁺ [M+H]⁺: 807.30; found, 807.1.

### Example 514: Preparation of 2-(6,7-dihydro-5H-pyrrolo[1,2-c]imidazol-1-yl)-2-(6-(4-((1R,4R)-5-((6-(2,4-dioxotetrahydropyrimidin-1(2H)-yl)pyrazin-2-yl)methyl)-2,5-diazabicyclo[2.2.1]heptan-2-yl)phenyl)-4-fluoro-1-oxoisoindolin-2-yl)-N-(thiazol-2-yl)acetamide (GT-07921)

According to the method of step 2 of Scheme I-1, the target compound (GT-07921) was prepared (white solid, 8 mg, yield 15 %). ¹H NMR (400 MHz, DMSO-d6) δ 11.15 (s, 1H), 10.80 (s, 1H), 9.11 - 9.04 (m, 2H), 8.58 (d, *J* = 12.9 Hz, 1H), 7.81 - 7.74 (m, 2H), 7.74 - 7.60 (m, 3H), 7.53 (d, *J* = 3.6 Hz, 1H), 7.33 (d, *J* = 3.6 Hz, 1H), 6.85 - 6.74 (m, 2H), 6.39 (s, 1H), 4.85 - 4.71 (m, 4H), 4.26 (t, *J* = 6.8 Hz, 3H), 4.08 - 4.03 (m, 3H), 3.79 - 3.65 (m, 4H), 3.44 (d, *J* = 9.4 Hz, 1H), 2.87 (d, *J* = 7.2 Hz, 1H), 2.74 (q, *J* = 12.1, 6.3 Hz, 3H), 2.65 - 2.58 (m, 3H), 2.19 (d, J = 10.0 Hz, 1H). LCMS (ESI) calcd for C₃₉H₃₇FN₁₁O₄S ⁺ [M+H]⁺: 774.27; found, 774.1.

### Example 515: Preparation of 2-(6,7-dihydro-5H-pyrrolo[1,2-c]imidazol-1-yl)-2-(6-(4-((1R,4R)-5-((5-(2,4-dioxotetrahydropyrimidin-1(2H)-yl)-3-fluoropyridin-2-yl)methyl)-2,5-diazabicyclo[2.2.1]heptan-2-yl)phenyl)-4-fluoro-1-oxoisoindolin-2-yl)-N-(thiazol-2-yl)acetamide (GT-07922)

According to the method of step 2 of Scheme I-1, the target compound (GT-07922) was prepared (white solid, 10 mg, yield 18 %). ¹H NMR (400 MHz, DMSO-d6) δ 10.76 (s, 1H), 10.65 (s, 1H), 9.10 (s, 1H), 8.55 (s, 1H), 7.97 - 7.89 (m, 1H), 7.80 (t, *J =* 4.9 Hz, 2H), 7.70 (d, *J* = 7.7 Hz, 2H), 7.53 (t, *J* = 4.1 Hz, 1H), 7.33 (d, *J* = 3.6 Hz, 1H), 6.80 (d, *J =* 8.3 Hz, 2H), 6.40 (s, 1H), 4.84 - 4.74 (m, 3H), 4.68 (s, 2H), 4.54 - 4.47 (m, 1H), 4.26 (t, *J =* 7.0 Hz, 2H), 3.89 (t, *J =* 6.6 Hz, 4H), 3.72 (d, *J =* 6.9 Hz, 5H), 3.48 (d, 1H), 2.94 - 2.82 (m, 1H), 2.75 (t, *J* = 6.3 Hz, 3H), 2.63 - 2.53 (m, 4H), 2.23 (d, *J* = 10.8 Hz, 1H). LCMS (ESI) calcd for C₄₀H₃₇F₂N₁₀O₄S⁺ [M+H]⁺: 791.27; found, 791.1.

### Example 516: Preparation of 2-((2-((4-(4-((4-(2,4-dioxotetrahydropyrimidin-1(2H)-yl)benzyl)(methyl)amino)piperidin-1-yl)-2-methoxyphenyl)amino)-5-(trifluoromethyl)pyridin-4-yl)amino)-N-methylbenzamide (GT-07649)

According to the method of step 2 of Scheme I-1, the target compound (GT-07649) was prepared (white solid, 22 mg, yield 39.78%). ¹H NMR (500 MHz, DMSO) δ 11.52 - 11.10 (m, 1H), 10.49 (dd, J = 66.8, 42.3 Hz, 2H), 10.08 (s, 1H), 8.86 (d, J = 4.6 Hz, 1H), 8.17 (d, J = 18.2 Hz, 1H), 7.79 - 7.56 (m, 4H), 7.44 (t, J = 10.6 Hz, 2H), 7.35 - 7.22 (m, 2H), 6.98 - 6.56 (m, 2H), 4.56 - 4.44 (m, 1H), 4.23 (dd, J = 12.9, 7.0 Hz, 1H), 3.99 - 3.82 (m, 10H), 3.47 (s, 1H), 2.95 (s, 1H), 2.77 - 2.70 (m, 4H), 2.59 (d, J = 4.8 Hz, 2H), 2.39 (d, J = 10.7 Hz, 1H), 2.29 (d, J = 9.2 Hz, 1H), 2.05 (s, 2H). LCMS (ESI) calcd for C₃₈H₄₂F₃N₈O₄⁺ [M+H]⁺: 731.33; found, 731.2.

### Example 517: Preparation of 2-((2-((4-(4-((3-(2,4-dioxotetrahydropyrimidin-1(2H)-yl)benzyl)(methyl)amino)piperidin-1-yl)-2-methoxyphenyl)amino)-5-(trifluoromethyl)pyridin-4-yl)amino)-N-methylbenzamide (GT-07650)

According to the method of step 2 of Scheme I-1, the target compound (GT-07650) was prepared (white solid, 18 mg, yield 32.55%). ¹H NMR (500 MHz, DMSO) δ 11.18 (s, 1H), 10.60 (s, 1H), 10.45 (s, 1H), 10.01 (s, 1H), 8.83 (q, J = 4.4 Hz, 1H), 8.15 (d, J = 10.9 Hz, 1H), 7.77 (d, J = 7.8 Hz, 1H), 7.68 (s, 1H), 7.60 - 7.52 (m, 3H), 7.49 - 7.41 (m, 2H), 7.30 - 7.19 (m, 2H), 6.86 (s, 1H), 6.74 (s, 1H), 6.56 (s, 1H), 4.49 (dd, J = 11.5, 4.5 Hz, 1H), 4.24 (dd, J = 13.0, 7.0 Hz, 1H), 3.88 (dd, J = 18.1, 11.4 Hz, 4H), 3.82 (s, 3H), 3.46 (s, 2H), 2.89 (s, 2H), 2.74 (dd, J = 12.0, 5.9 Hz, 4H), 2.60 (d, J = 4.7 Hz, 3H), 2.36 (d, J = 11.1 Hz, 1H), 2.26 (d, J = 11.1 Hz, 1H), 2.01 (s, 2H). LCMS (ESI) calcd for C₃₈H₄₂F₃N₈O₄⁺ [M+H]⁺: 731.33; found, 731.2.

### Example 518: Preparation of 2-((2-((4-(4-(((6-(2,4-dioxotetrahydropyrimidin-1(2H)-yl)pyridin-3-yl)methyl)(methyl)amino)piperidin-1-yl)-2-methoxyphenyl)amino)-5-(trifluoromethyl)pyridin-4-yl)amino)-N-methylbenzamide (GT-07651)

According to the method of step 2 of Scheme I-1, the target compound (GT-07651) was prepared (white solid, 10 mg, yield 18.69%). ¹H NMR (500 MHz, DMSO) δ 11.47 (s, 1H), 10.72 - 10.56 (m, 2H), 10.10 (s, 1H), 8.86 (d, J = 4.6 Hz, 1H), 8.68 (d, J = 2.0 Hz, 1H), 7.86 (d, J = 8.6 Hz, 1H), 7.78 (d, J = 7.7 Hz, 1H), 7.58 (d, J = 3.9 Hz, 2H), 7.32 - 7.24 (m, 2H), 6.96 (s, 1H), 6.85 (d, J = 16.0 Hz, 1H), 6.58 (s, 1H), 4.52 (d, J = 7.6 Hz, 1H), 4.30 (dd, J = 13.1, 6.7 Hz, 1H), 4.10 (t, J = 6.6 Hz, 2H), 3.90 (s, 4H), 3.48 (d, J = 22.1 Hz, 2H), 3.02 (s, 2H), 2.76 (d, J = 4.6 Hz, 3H), 2.71 (t, J = 6.6 Hz, 2H), 2.61 (d, J = 4.5 Hz, 3H), 2.39 (d, J = 11.9 Hz, 1H), 2.31 (d, J = 11.0 Hz, 1H), 2.08 (s, 2H). LCMS (ESI) calcd for C₃₇H₄₁F₃N₉O₄⁺ [M+H]⁺: 732.32; found, 732.2.

### Example 519: Preparation of 2-((2-((4-(4-((3-((2,6-dioxopiperidin-3-yl)amino)benzyl)(methyl)amino)piperidin-1-yl)-2-methoxyphenyl)amino)-5-(trifluoromethyl)pyridin-4-yl)amino)-N-methylbenzamide (GT-07652)

According to the method of step 2 of Scheme I-1, the target compound (GT-07652) was prepared (white solid, 12 mg, yield 21.29%). ¹H NMR (500 MHz, DMSO) δ 11.11 (s, 1H), 10.84 (t, J = 6.9 Hz, 1H), 10.63 (s, 1H), 10.13 (s, 1H), 8.94 - 8.82 (m, 1H), 8.17 (d, J = 17.9 Hz, 1H), 7.79 (d, J = 7.9 Hz, 1H), 7.58 (t, J = 6.4 Hz, 2H), 7.30 (td, J = 8.0, 3.9 Hz, 2H), 7.15 (dd, J = 17.7, 9.9 Hz, 2H), 7.04 - 6.94 (m, 1H), 6.86 - 6.78 (m, 2H), 6.58 (s, 1H), 4.45 (dd, J = 11.4, 3.9 Hz, 1H), 4.37 - 4.31 (m, 1H), 4.14 - 3.99 (m, 6H), 3.46 (s, 1H), 3.01 (s, 2H), 2.86 (ddd, J = 24.1, 12.2, 5.9 Hz, 1H), 2.77 (t, J = 5.2 Hz, 3H), 2.60 (t, J = 7.5 Hz, 4H), 2.38 (s, 1H), 2.25 (s, 1H), 2.19 (d, J = 10.2 Hz, 1H), 2.10 (s, 2H), 1.90 (qd, J = 12.5, 4.7 Hz, 1H). LCMS (ESI) calcd for C₃₉H₄₄F₃N₈O₄⁺ [M+H]⁺: 745.34; found, 745.2.

### Example 520: Preparation of 2-((2-((4-(4-(((2-(2,4-dioxotetrahydropyrimidin-1(2H)-yl)-5-fluoropyridin-4-yl)methyl)(methyl)amino)piperidin-1-yl)-2-methoxyphenyl)amino)-5-(trifluoromethyl)pyridin-4-yl)amino)-N-methylbenzamide (GT-07653)

According to the method of step 2 of Scheme I-1, the target compound (GT-07653) was prepared (white solid, 23 mg, yield 40.54%). ¹H NMR (500 MHz, DMSO) δ 11.45 (s, 1H), 10.65 (d, J = 9.8 Hz, 2H), 10.13 (s, 1H), 8.93 - 8.82 (m, 1H), 8.55 (s, 1H), 8.19 (s, 1H), 8.12 (d, J = 5.3 Hz, 1H), 7.79 (d, J = 7.8 Hz, 1H), 7.61 - 7.54 (m, 2H), 7.28 (ddd, J = 22.0, 11.3, 5.6 Hz, 2H), 6.91 (s, 1H), 6.78 (d, J = 6.1 Hz, 1H), 6.57 (s, 1H), 4.60 (d, J = 14.4 Hz, 1H), 4.38 (s, 1H), 4.05 (d, J = 6.6 Hz, 2H), 3.94 (d, J = 11.8 Hz, 2H), 3.83 (s, 3H), 3.57 (s, 2H), 2.98 (s, 2H), 2.77 - 2.71 (m, 7H), 2.37 (s, 1H), 2.24 (s, 1H), 2.05 (s, 2H). LCMS (ESI) calcd for C₃₇H₄₀F₄N₉O₄⁺ [M+H]⁺: 750.31; found, 750.2.

### Example 521: Preparation of 2-((2-((4-(4-(((2-(2,4-dioxotetrahydropyrimidin-1(2H)-yl)pyridin-4-yl)methyl)(methyl)amino)piperidin-1-yl)-2-methoxyphenyl)amino)-5-(trifluoromethyl)pyridin-4-yl)amino)-N-methylbenzamide (GT-07654)

According to the method of step 2 of Scheme I-1, the target compound (GT-07654) was prepared (white solid, 20 mg, yield 36.11%). ¹H NMR (500 MHz, DMSO) δ 11.58 (d, J = 50.8 Hz, 1H), 10.65 (t, J = 9.1 Hz, 1H), 10.59 (s, 1H), 10.29 (s, 1H), 8.90 - 8.38 (m, 2H), 8.09 (d, J = 105.0 Hz, 1H), 7.83 (s, 1H), 7.81 - 7.67 (m, 1H), 7.58 (d, J = 3.8 Hz, 1H), 7.31 (td, J = 8.6, 5.1 Hz, 1H), 7.25 (dd, J = 5.1, 1.2 Hz, 1H), 7.09 (d, J = 81.7 Hz, 1H), 4.55 (d, J = 12.6 Hz, 1H), 4.39 (d, J = 11.3 Hz, 1H), 4.05 (d, J = 6.6 Hz, 9H), 3.90 - 3.79 (m, 4H), 3.59 - 3.33 (m, 1H), 3.21 (s, 1H), 2.71 (ddd, J = 24.0, 15.0, 7.6 Hz, 6H), 2.29 (dd, J = 77.8, 28.1 Hz, 2H). LCMS (ESI) calcd for C₃₇H₄₁F₃N₉O₄⁺ [M+H]⁺: 732.32; found, 732.2.

### Example 522: Preparation of 2-((2-((4-(4-(((5-(2,4-dioxotetrahydropyrimidin-1(2H)-yl)pyridin-3-yl)methyl)(methyl)amino)piperidin-1-yl)-2-methoxyphenyl)amino)-5-(trifluoromethyl)pyridin-4-yl)amino)-N-methylbenzamide (GT-07655)

According to the method of step 2 of Scheme I-1, the target compound (GT-07655) was prepared (white solid, 8 mg, yield 14.45%). ¹H NMR (500 MHz, DMSO) δ 11.53 (s, 1H), 10.60 (d, J = 4.8 Hz, 1H), 9.98 (s, 1H), 9.41 - 9.14 (m, 1H), 8.91 - 8.71 (m, 2H), 8.37 - 8.11 (m, 1H), 7.77 (d, J = 7.8 Hz, 1H), 7.57 (d, J = 3.8 Hz, 2H), 7.32 - 7.20 (m, 2H), 6.99 - 6.70 (m, 2H), 6.60 (d, J = 23.4 Hz, 1H), 4.64 - 4.34 (m, 1H), 4.02 - 3.82 (m, 6H), 3.42 (d, J = 7.0 Hz, 4H), 2.96 (s, 2H), 2.75 (d, J = 4.6 Hz, 4H), 2.62 (s, 1H), 2.55 (t, J = 5.4 Hz, 2H), 2.33 (dd, J = 34.1, 6.1 Hz, 1H), 2.17 (d, J = 10.8 Hz, 1H), 1.95 (d, J = 43.7 Hz, 1H), 1.89 - 1.64 (m, 1H). LCMS (ESI) calcd for C₃₇H₄₁F₃N₉O₄⁺ [M+H]⁺: 732.32; found, 732.2.

### Example 523: Preparation of 2-((2-((4-(4-(((5-(2,6-dioxopiperidin-3-yl)-2-fluoropyridin-3-yl)methyl)(methyl)amino)piperidin-1-yl)-2-methoxyphenyl)amino)-5-(trifluoromethyl)pyridin-4-yl)amino)-N-methylbenzamide (GT-07656)

According to the method of step 2 of Scheme I-1, the target compound (GT-07656) was prepared (white solid, 8 mg, yield 14.12%). ¹H NMR (500 MHz, DMSO) δ 11.00 (s, 1H), 10.86 (d, J = 48.6 Hz, 1H), 9.92 (s, 1H), 8.80 (d, J = 4.6 Hz, 1H), 8.32 - 8.24 (m, 1H), 8.10 (d, J = 42.1 Hz, 1H), 7.78 (d, J = 7.7 Hz, 1H), 7.61 - 7.52 (m, 2H), 7.29 (dd, J = 9.7, 6.4 Hz, 2H), 6.75 (d, J = 57.0 Hz, 2H), 4.82 (d, J = 16.8 Hz, 1H), 4.62 - 4.48 (m, 1H), 4.26 (dd, J = 14.4, 5.8 Hz, 1H), 4.12 - 3.93 (m, 3H), 3.84 (d, J = 11.5 Hz, 3H), 2.86 (s, 2H), 2.77 (t, J = 5.6 Hz, 3H), 2.66 (dd, J = 18.4, 10.1 Hz, 4H), 2.32 (ddd, J = 16.5, 15.1, 4.5 Hz, 2H), 2.16 (d, J = 41.3 Hz, 2H), 1.94 (d, J = 28.2 Hz, 3H). LCMS (ESI) calcd for C₃₈H₄₁F₄N₈O₄⁺ [M+H]⁺: 749.32; found, 749.2.

### Example 524: Preparation of 2-((2-((4-(4-(((6-(2,6-dioxopiperidin-3-yl)pyridin-3-yl)methyl)(methyl)amino)piperidin-1-yl)-2-methoxyphenyl)amino)-5-(trifluoromethyl)pyridin-4-yl)amino)-N-methylbenzamide (GT-07657)

According to the method of step 2 of Scheme I-1, the target compound (GT-07657) was prepared (white solid, 8 mg, yield 14.47%). ¹H NMR (500 MHz, DMSO) δ 11.64 (s, 1H), 11.00 (d, J = 12.9 Hz, 1H), 10.63 (s, 1H), 10.14 (s, 1H), 8.86 (dd, J = 19.3, 14.7 Hz, 2H), 8.39 (d, J = 7.2 Hz, 1H), 8.18 (s, 1H), 7.78 (d, J = 7.8 Hz, 1H), 7.64 (d, J = 8.1 Hz, 1H), 7.57 (d, J = 3.9 Hz, 2H), 7.31 - 7.25 (m, 2H), 6.99 (s, 1H), 6.85 (s, 1H), 6.57 (s, 1H), 4.58 (d, J = 12.7 Hz, 1H), 4.33 (dd, J = 31.2, 25.1 Hz, 3H), 3.83 (s, 3H), 3.56 (s, 2H), 3.06 (s, 2H), 2.77 - 2.73 (m, 4H), 2.70 - 2.56 (m, 5H), 2.44 - 2.28 (m, 4H), 2.20 - 2.05 (m, 3H). LCMS (ESI) calcd for C₃₈H₄₂F₃N₈O₄⁺ [M+H]⁺: 731.33; found, 731.2.

### Example 525: Preparation of 2-((2-((4-(4-(((5-(2,6-dioxopiperidin-3-yl)pyridin-2-yl)methyl)(methyl)amino)piperidin-1-yl)-2-methoxyphenyl)amino)-5-(trifluoromethyl)pyridin-4-yl)amino)-N-methylbenzamide (GT-07658)

According to the method of step 2 of Scheme I-1, the target compound (GT-07658) was prepared (white solid, 20 mg, yield 36.17%). ¹H NMR (500 MHz, DMSO) δ 10.96 (s, 1H), 10.64 (s, 1H), 10.20 (s, 1H), 8.88 (q, J = 4.4 Hz, 1H), 8.59 (d, J = 2.0 Hz, 1H), 8.19 (s, 1H), 7.88 (dd, J = 8.1, 2.1 Hz, 1H), 7.78 (dd, J = 7.7, 5.8 Hz, 2H), 7.57 (d, J = 4.0 Hz, 2H), 7.33 - 7.22 (m, 2H), 7.01 (s, 1H), 6.86 (s, 1H), 6.58 (s, 1H), 4.52 (d, J = 19.9 Hz, 2H), 4.09 (d, J = 4.8 Hz, 2H), 3.83 (s, 4H), 3.62 - 3.53 (m, 2H), 3.02 (dd, J = 18.3, 14.2 Hz, 2H), 2.76 - 2.71 (m, 6H), 2.58 (dd, J = 13.8, 3.5 Hz, 1H), 2.32 (tt, J = 13.0, 6.4 Hz, 3H), 2.16 - 2.02 (m, 3H). LCMS (ESI) calcd for C₃₈H₄₂F₃N₈O₄⁺ [M+H]⁺: 731.33; found, 731.2.

### Example 526: Preparation of N-(3-(((2-((4-(4-(4-(2,6-dioxopiperidin-3-yl)benzyl)piperazin-1-yl)phenyl)amino)-5-(trifluoromethyl)pyrimidin-4-yl)amino)methyl)pyrazin-2-yl)-N-methylmethanesulfonamide (GT-07399)

According to the method of step 2 of Scheme I-1, the target compound (GT-07399) was prepared (white solid, 23 mg, yield 41.84%). ¹H NMR (400 MHz, DMSO-d6) δ 11.62 (s, 1H), 10.88 (s, 1H), 10.68 (s, 1H), 8.69 (d, J = 2.5 Hz, 1H), 8.61 (d, J = 2.5 Hz, 1H), 8.47 (s, 1H), 7.65 (d, J = 8.1 Hz, 2H), 7.34 (d, J = 8.2 Hz, 2H), 7.17 (s, 2H), 6.82 (d, J = 7.1 Hz, 2H), 4.96 (d, J = 5.1 Hz, 2H), 4.38 (s, 3H), 3.94 (dd, J = 11.7, 4.9 Hz, 1H), 3.72 (d, J = 11.1 Hz, 2H), 3.37 (d, J = 9.6 Hz, 2H), 3.24 - 3.07 (m, 9H), 2.70 (ddd, J = 17.1, 12.0, 5.2 Hz, 1H), 2.54 (d, J = 5.1 Hz, 1H), 2.24 (qd, J = 12.6, 4.4 Hz, 1H), 2.10 - 2.01 (m, 1H). LCMS (ESI) calcd for C₃₄H₃₈F₃N₁₀O₄S⁺ [M+H]⁺: 739.28; found, 739.3.

### Example 527: Preparation of N-(3-(((2-((4-(4-(4-(2,4-dioxotetrahydropyrimidin-1(2H)-yl)benzyl)piperazin-1-yl)phenyl)amino)-5-(trifluoromethyl)pyrimidin-4-yl)amino)methyl)pyrazin-2-yl)-N-methylmethanesulfonamide (GT-07400)

According to the method of step 2 of Scheme I-1, the target compound (GT-07400) was prepared (white solid, 14 mg, yield 25.43%). ¹H NMR (400 MHz, DMSO-d6) δ 11.69 (d, J = 71.2 Hz, 1H), 10.60 (s, 1H), 10.44 (s, 1H), 8.68 (d, J = 2.5 Hz, 1H), 8.60 (d, J = 2.5 Hz, 1H), 8.44 (s, 2H), 7.78 - 7.63 (m, 2H), 7.48 (dd, J = 42.6, 8.5 Hz, 2H), 7.19 (s, 2H), 6.82 (d, J = 6.2 Hz, 2H), 4.95 (d, J = 5.1 Hz, 2H), 4.36 (s, 3H), 3.83 (t, J = 6.6 Hz, 2H), 3.72 (d, J = 10.6 Hz, 2H), 3.37 (d, J = 10.2 Hz, 2H), 3.21 - 3.06 (m, 9H), 2.71 (q, J = 6.5 Hz, 2H). LCMS (ESI) calcd for C₃₃H₃₇F₃N₁₁O₄S⁺ [M+H]⁺: 740.27; found, 740.3.

### Example 528: Preparation of N-(3-(((2-((4-(4-(3-(2,4-dioxotetrahydropyrimidin-1(2H)-yl)benzyl)piperazin-1-yl)phenyl)amino)-5-(trifluoromethyl)pyrimidin-4-yl)amino)methyl)pyrazin-2-yl)-N-methylmethanesulfonamide (GT-07401)

According to the method of step 2 of Scheme I-1, the target compound (GT-07401) was prepared (white solid, 23 mg, yield 41.78%). ¹H NMR (400 MHz, DMSO-d6) δ 11.62 (s, 1H), 10.61 (s, 1H), 10.46 (s, 1H), 8.69 (d, J = 2.5 Hz, 1H), 8.61 (d, J = 2.4 Hz, 1H), 8.45 (s, 2H), 7.67 (s, 1H), 7.51 (dt, J = 13.1, 4.1 Hz, 3H), 7.19 (s, 2H), 6.83 (d, J = 6.7 Hz, 2H), 4.96 (d, J = 5.1 Hz, 2H), 4.39 (s, 3H), 3.88 (t, J = 6.6 Hz, 2H), 3.72 (d, J = 10.9 Hz, 2H), 3.39 (d, J = 10.3 Hz, 2H), 3.22 - 3.07 (m, 9H), 2.73 (t, J = 6.6 Hz, 2H). LCMS (ESI) calcd for C₃₃H₃₇F₃N₁₁O₄S⁺ [M+H]⁺: 740.27; found, 740.3.

### Example 529: Preparation of N-(3-(((2-((4-(4-((5-(2,4-dioxotetrahydropyrimidin-1(2H)-yl)pyridin-2-yl)methyl)piperazin-1-yl)phenyl)amino)-5-(trifluoromethyl)pyrimidin-4-yl)amino)methyl)pyrazin-2-yl)-N-methylmethanesulfonamide (GT-07403)

According to the method of step 2 of Scheme I-1, the target compound (GT-07403) was prepared (white solid, 16 mg, yield 29.03%). ¹H NMR (400 MHz, DMSO-d6) δ 10.86 (s, 1H), 10.61 (s, 1H), 8.71 (d, J = 2.4 Hz, 1H), 8.69 (d, J = 2.5 Hz, 1H), 8.61 (d, J = 2.5 Hz, 1H), 8.51 (s, 1H), 7.93 (dd, J = 8.4, 2.6 Hz, 1H), 7.81 (d, J = 8.4 Hz, 1H), 7.16 (s, 2H), 6.84 (s, 2H), 4.96 (d, J = 5.0 Hz, 2H), 4.54 (s, 3H), 3.90 (t, J = 6.6 Hz, 2H), 3.39 (s, 6H), 3.18 (s, 3H), 3.08 (s, 3H), 2.76 (t, J = 6.6 Hz, 2H). LCMS (ESI) calcd for C₃₂H₃₆F₃N₁₂O₄S⁺ [M+H]⁺: 741.27; found, 741.3.

### Example 530: Preparation of N-(3-(((2-((4-(4-((6-(2,4-dioxotetrahydropyrimidin-1(2H)-yl)pyridin-3-yl)methyl)piperazin-1-yl)phenyl)amino)-5-(trifluoromethyl)pyrimidin-4-yl)amino)methyl)pyrazin-2-yl)-N-methylmethanesulfonamide (GT-07404)

According to the method of step 2 of Scheme I-1, the target compound (GT-07404) was prepared (white solid, 23 mg, yield 41.73%). ¹H NMR (400 MHz, DMSO-d6) δ 12.01 (s, 1H), 11.02 (s, 1H), 10.63 (s, 1H), 8.81 (s, 1H), 8.67 (dd, J = 5.5, 2.3 Hz, 2H), 8.61 (d, J = 2.4 Hz, 1H), 8.47 (d, J = 2.1 Hz, 1H), 8.17 (dd, J = 8.7, 2.3 Hz, 1H), 7.92 - 7.76 (m, 2H), 7.11 (d, J = 6.9 Hz, 2H), 6.83 (s, 2H), 4.82 (s, 1H), 4.42 (s, 2H), 4.08 (dt, J = 15.9, 6.6 Hz, 3H), 3.73 (d, J = 10.1 Hz, 2H), 3.39 (d, J = 9.5 Hz, 2H), 3.21 (d, J = 18.6 Hz, 6H), 3.05 (s, 3H), 2.70 (q, J = 6.6 Hz, 3H). LCMS (ESI) calcd for C₃₂H₃₆F₃N₁₂O₄S⁺ [M+H]⁺: 741.27; found, 741.3.

### Example 531: Preparation of N-(3-(((2-((4-(4-(3-((2,6-dioxopiperidin-3-yl)amino)benzyl)piperazin-1-yl)phenyl)amino)-5-(trifluoromethyl)pyrimidin-4-yl)amino)methyl)pyrazin-2-yl)-N-methylmethanesulfonamide (GT-07402)

According to the method of step 2 of Scheme I-1, the target compound (GT-07402) was prepared (white solid, 23 mg, yield 41.01%). ¹H NMR (400 MHz, DMSO-d6) δ 11.48 (s, 1H), 10.86 (s, 2H), 8.71 - 8.61 (m, 2H), 8.50 (s, 1H), 7.16 (t, J = 7.8 Hz, 3H), 6.82 (t, J = 8.7 Hz, 3H), 4.95 (d, J = 5.1 Hz, 2H), 4.42 (dd, J = 11.7, 4.8 Hz, 2H), 4.24 (s, 2H), 3.72 (d, J = 12.1 Hz, 2H), 3.37 (d, J = 10.9 Hz, 2H), 3.29 - 3.21 (m, 2H), 3.18 (s, 3H), 3.09 (d, J = 12.3 Hz, 3H), 2.84 (ddd, J = 17.5, 12.6, 5.2 Hz, 1H), 2.67 - 2.55 (m, 2H), 2.26 - 2.13 (m, 1H), 1.97 - 1.84 (m, 1H). LCMS (ESI) calcd for C₃₄H₃₉F₃N₁₁O₄S⁺ [M+H]⁺: 754.29; found, 754.29.

### Example 532: Preparation of N-(3-(((2-((4-(4-((2-(2,4-dioxotetrahydropyrimidin-1(2H)-yl)-5-fluoropyridin-4-yl)methyl)piperazin-1-yl)phenyl)amino)-5-(trifluoromethyl)pyrimidin-4-yl)amino)methyl)pyrazin-2-yl)-N-methylmethanesulfonamide (GT-07405)

According to the method of step 2 of Scheme I-1, the target compound (GT-07405) was prepared (white solid, 28 mg, yield 49.59%). ¹H NMR (400 MHz, DMSO-d6) δ 11.73 (s, 1H), 10.77 (s, 1H), 10.66 (s, 1H), 8.69 (d, J = 2.5 Hz, 1H), 8.61 (d, J = 2.5 Hz, 1H), 8.56 (s, 1H), 8.47 (d, J = 11.1 Hz, 1H), 8.15 (d, J = 5.3 Hz, 1H), 7.18 (d, J = 6.0 Hz, 2H), 6.84 (d, J = 6.6 Hz, 2H), 4.96 (d, J = 5.2 Hz, 2H), 4.51 (s, 3H), 4.04 (dd, J = 12.2, 5.6 Hz, 2H), 3.73 (s, 2H), 3.52 (s, 2H), 3.18 (s, 6H), 3.08 (s, 3H), 2.72 (t, J = 6.6 Hz, 2H). LCMS (ESI) calcd for C₃₂H₃₅F₄N₁₂O₄S⁺ [M+H]⁺: 759.26; found, 759.3.

### Example 533: Preparation of N-(3-(((2-((4-(4-((2-(2,4-dioxotetrahydropyrimidin-1(2H)-yl)pyridin-4-yl)methyl)piperazin-1-yl)phenyl)amino)-5-(trifluoromethyl)pyrimidin-4-yl)amino)methyl)pyrazin-2-yl)-N-methylmethanesulfonamide (GT-07406)

According to the method of step 2 of Scheme I-1, the target compound (GT-07406) was prepared (white solid, 26 mg, yield 47.17%). ¹H NMR (400 MHz, DMSO-d6) δ 12.00 (s, 1H), 10.97 (s, 1H), 10.63 (s, 1H), 8.74 (s, 1H), 8.68 (d, J = 2.5 Hz, 1H), 8.61 (d, J = 2.5 Hz, 1H), 8.47 (dd, J = 40.9, 5.1 Hz, 2H), 7.98 (s, 1H), 7.69 (dd, J = 5.1, 1.1 Hz, 1H), 7.13 (d, J = 7.2 Hz, 2H), 6.83 (s, 2H), 4.95 (d, J = 5.1 Hz, 2H), 4.47 (s, 2H), 4.08 (dt, J = 13.2, 6.6 Hz, 3H), 3.72 (s, 2H), 3.40 (s, 2H), 3.27 - 3.17 (m, 6H), 3.06 (s, 3H), 2.71 (dt, J = 9.5, 6.6 Hz, 3H). LCMS (ESI) calcd for C₃₂H₃₆F₃N₁₂O₄S⁺ [M+H]⁺: 741.27; found, 741.3.

### Example 534: Preparation of N-(3-(((2-((4-(4-((5-(2,4-dioxotetrahydropyrimidin-1(2H)-yl)pyridin-3-yl)methyl)piperazin-1-yl)phenyl)amino)-5-(trifluoromethyl)pyrimidin-4-yl)amino)methyl)pyrazin-2-yl)-N-methylmethanesulfonamide (GT-07407)

According to the method of step 2 of Scheme I-1, the target compound (GT-07407) was prepared (white solid, 22 mg, yield 39.91%). ¹H NMR (400 MHz, DMSO-d6) δ 11.87 (s, 1H), 10.68 (s, 1H), 10.47 (s, 1H), 8.83 (d, J = 2.3 Hz, 1H), 8.70 (dd, J = 9.8, 2.0 Hz, 2H), 8.61 (d, J = 2.5 Hz, 1H), 8.42 (s, 1H), 8.34 (s, 1H), 7.21 (s, 2H), 6.85 (s, 2H), 4.96 (d, J = 5.1 Hz, 2H), 4.52 (s, 2H), 4.07 (s, 3H), 3.72 (s, 2H), 3.44 (s, 2H), 3.18 (d, J = 6.2 Hz, 6H), 3.10 (s, 3H), 2.78 (t, J = 6.6 Hz, 2H). LCMS (ESI) calcd for C₃₂H₃₆F₃N₁₂O₄S⁺ [M+H]⁺: 741.27; found, 741.3.

### Example 535: Preparation of N-(3-(((2-((4-(4-((5-(2,6-dioxopiperidin-3-yl)-2-fluoropyridin-3-yl)methyl)piperazin-1-yl)phenyl)amino)-5-(trifluoromethyl)pyrimidin-4-yl)amino)methyl)pyrazin-2-yl)-N-methylmethanesulfonamide (GT-07397)

According to the method of step 2 of Scheme I-1, the target compound (GT-07397) was prepared (white solid, 6 mg, yield 10.64%). ¹H NMR (400 MHz, DMSO-d6) δ 11.81 (s, 1H), 11.00 (s, 1H), 10.71 (s, 1H), 8.69 (d, J = 2.5 Hz, 1H), 8.61 (d, J = 2.4 Hz, 1H), 8.47 (s, 1H), 8.37 - 8.31 (m, 1H), 8.25 (d, J = 1.6 Hz, 1H), 7.19 (s, 2H), 6.83 (s, 2H), 4.95 (d, J = 4.9 Hz, 2H), 4.45 (d, J = 2.6 Hz, 2H), 4.05 (d, J = 4.9 Hz, 2H), 3.78 - 3.70 (m, 2H), 3.47 (d, J = 26.3 Hz, 2H), 3.25 - 3.05 (m, 9H), 2.84 - 2.70 (m, 1H), 2.66 - 2.58 (m, 1H), 2.30 (ddd, J = 27.1, 13.4, 9.3 Hz, 1H), 2.18 - 1.98 (m, 1H). LCMS (ESI) calcd for C₃₃H₃₆F₄N₁₁O₄S⁺ [M+H]⁺: 758.26; found, 758.3.

### Example 536: Preparation of N-(3-(((2-((4-(4-((6-(2,6-dioxopiperidin-3-yl)pyridin-3-yl)methyl)piperazin-1-yl)phenyl)amino)-5-(trifluoromethyl)pyrimidin-4-yl)amino)methyl)pyrazin-2-yl)-N-methylmethanesulfonamide (GT-07398)

According to the method of step 2 of Scheme I-1, the target compound (GT-07398) was prepared (white solid, 18 mg, yield 32.70%). ¹H NMR (400 MHz, DMSO-d6) δ 12.10 (s, 1H), 11.02 (s, 1H), 10.93 (s, 1H), 8.91 (d, J = 1.5 Hz, 1H), 8.68 (d, J = 2.4 Hz, 2H), 8.61 (d, J = 2.4 Hz, 1H), 8.53 (s, 1H), 8.43 (d, J = 8.1 Hz, 1H), 7.71 (d, J = 8.2 Hz, 1H), 7.13 (s, 2H), 6.83 (s, 2H), 4.95 (d, J = 5.1 Hz, 2H), 4.51 (s, 2H), 4.30 (dd, J = 10.6, 5.1 Hz, 2H), 3.73 (s, 2H), 3.41 (s, 2H), 3.19 (s, 7H), 3.06 (s, 2H), 2.68 (dd, J = 11.1, 5.5 Hz, 1H), 2.62 - 2.55 (m, 1H), 2.39 (d, J = 8.6 Hz, 1H), 2.18 (dd, J = 13.2, 5.1 Hz, 1H). LCMS (ESI) calcd for C₃₃H₃₇F₃N₁₁O₄S⁺ [M+H]⁺: 740.27; found, 740.3.

### Example 537: Preparation of N-(3-(((2-((4-(4-((2-(2,6-dioxopiperidin-3-yl)pyridin-4-yl)methyl)piperazin-1-yl)phenyl)amino)-5-(trifluoromethyl)pyrimidin-4-yl)amino)methyl)pyrazin-2-yl)-N-methylmethanesulfonamide (GT-07507)

According to the method of step 2 of Scheme I-1, the target compound (GT-07507) was prepared (white solid, 16 mg, yield 29.23%). ¹H NMR (500 MHz, DMSO) δ 12.17 (s, 1H), 11.01 (s, 1H), 10.82 (s, 1H), 8.77 - 8.73 (m, 1H), 8.68 (d, J = 2.4 Hz, 1H), 8.62 (s, 1H), 8.50 (s, 1H), 7.89 (s, 2H), 7.16 (s, 2H), 6.84 (s, 2H), 4.95 (d, J = 5.2 Hz, 2H), 4.52 (s, 2H), 4.23 (d, J = 5.1 Hz, 2H), 3.72 (s, 2H), 3.45 - 3.24 (m, 5H), 3.19 (s, 3H), 3.07 (s, 3H), 2.77 - 2.66 (m, 1H), 2.59 (dt, J = 17.2, 4.7 Hz, 1H), 2.42 - 2.31 (m, 1H), 2.25 - 2.15 (m, 1H). LCMS (ESI) calcd for C₃₃H₃₇F₃N₁₁O₄S⁺ [M+H]⁺: 740.27; found, 740.3.

### Example 538: Preparation of N-(3-(((2-((4-(4-((5-(2,6-dioxopiperidin-3-yl)pyridin-2-yl)methyl)piperazin-1-yl)phenyl)amino)-5-(trifluoromethyl)pyrimidin-4-yl)amino)methyl)pyrazin-2-yl)-N-methylmethanesulfonamide (GT-07509)

According to the method of step 2 of Scheme I-1, the target compound (GT-07509) was prepared (white solid, 18 mg, yield 32.70%). ¹H NMR (500 MHz, DMSO) δ 10.97 (s, 1H), 10.72 (d, J = 65.4 Hz, 1H), 8.69 (d, J = 2.5 Hz, 1H), 8.61 (dd, J = 5.1, 2.3 Hz, 2H), 8.50 (s, 1H), 7.88 (dd, J = 8.1, 2.2 Hz, 1H), 7.76 (d, J = 8.0 Hz, 1H), 7.12 (d, J = 68.3 Hz, 2H), 6.85 (s, 2H), 4.96 (d, J = 5.2 Hz, 2H), 4.55 (s, 2H), 4.10 (s, 3H), 3.43 (d, J = 24.6 Hz, 7H), 3.19 (s, 3H), 3.09 (s, 2H), 2.79 - 2.68 (m, 1H), 2.61 (s, 1H), 2.33 (qd, J = 12.9, 4.4 Hz, 1H), 2.12 - 2.01 (m, 1H). LCMS (ESI) calcd for C₃₃H₃₇F₃N₁₁O₄S⁺ [M+H]⁺: 740.27; found, 740.3.

### Example 539: Preparation of N-(3-(((2-((4-(4-((5-(2,6-dioxopiperidin-3-yl)pyridin-3-yl)methyl)piperazin-1-yl)phenyl)amino)-5-(trifluoromethyl)pyrimidin-4-yl)amino)methyl)pyrazin-2-yl)-N-methylmethanesulfonamide (GT-07510)

According to the method of step 2 of Scheme I-1, the target compound (GT-07510) was prepared (white solid, 12 mg, yield 21.80%). ¹H NMR (500 MHz, DMSO) δ 12.09 (s, 1H), 11.07 (s, 1H), 10.65 (s, 1H), 9.01 (d, J = 1.3 Hz, 1H), 8.84 (d, J = 1.6 Hz, 1H), 8.69 (d, J = 2.5 Hz, 1H), 8.61 (d, J = 2.4 Hz, 1H), 8.56 (s, 1H), 8.45 (s, 2H), 7.19 (s, 2H), 6.85 (s, 2H), 4.95 (d, J = 5.1 Hz, 2H), 4.59 (d, J = 15.4 Hz, 2H), 4.23 - 4.20 (m, 3H), 3.73 (s, 2H), 3.44 (s, 2H), 3.19 (s, 6H), 3.09 (s, 2H), 2.83 - 2.72 (m, 1H), 2.68 - 2.61 (m, 1H), 2.37 (qd, J = 12.9, 4.4 Hz, 1H), 2.22 - 2.13 (m, 1H). LCMS (ESI) calcd for C₃₃H₃₇F₃N₁₁O₄S⁺ [M+H]⁺: 740.27; found, 740.3.

### Example 540: Preparation of N-(3-(((2-((4-(4-((2-(2,6-dioxopiperidin-3-yl)pyridin-3-yl)methyl)piperazin-1-yl)phenyl)amino)-5-(trifluoromethyl)pyrimidin-4-yl)amino)methyl)pyrazin-2-yl)-N-methylmethanesulfonamide (GT-07512)

According to the method of step 2 of Scheme I-1, the target compound (GT-07512) was prepared (white solid, 16 mg, yield 29.06%). ¹H NMR (500 MHz, DMSO) δ 11.73 (s, 1H), 10.94 (s, 1H), 10.77 (s, 1H), 8.71 - 8.65 (m, 2H), 8.61 (d, J = 2.4 Hz, 1H), 8.49 (s, 1H), 8.42 (d, J = 6.9 Hz, 1H), 7.53 (dd, J = 7.8, 4.9 Hz, 1H), 7.15 (s, 2H), 6.84 (s, 2H), 4.97 - 4.84 (m, 3H), 4.60 - 4.50 (m, 2H), 3.65 (d, J = 85.3 Hz, 3H), 3.35 (d, J = 32.8 Hz, 5H), 3.19 (s, 3H), 3.07 (s, 2H), 2.84 - 2.74 (m, 1H), 2.63 (dd, J = 13.5, 3.7 Hz, 1H), 2.23 - 2.13 (m, 1H). LCMS (ESI) calcd for C₃₃H₃₇F₃N₁₁O₄S⁺ [M+H]⁺: 740.27; found, 740.3.

### Example 541: Preparation of 1-(4-((4-(4-((4-((3-(methylsulfonyl)benzyl)amino)-5-(trifluoromethyl)pyrimidin-2-yl)amino)phenyl)piperazin-1-yl)methyl)phenyl)dihydropyrimidine-2,4(1H,3H)-dione (GT-07457)

According to the method of step 2 of Scheme I-1, the target compound (GT-07457) was prepared (white solid, 15 mg, yield 26.80%). ¹H NMR (400 MHz, DMSO-d6) δ 11.61 - 11.24 (m, 1H), 10.45 (s, 2H), 8.75 (s, 1H), 7.90 (s, 1H), 7.81 (d, J = 7.2 Hz, 2H), 7.71 (dd, J = 21.9, 8.2 Hz, 2H), 7.61 (t, J = 7.7 Hz, 1H), 7.54 (d, J = 8.4 Hz, 1H), 7.44 (d, J = 8.5 Hz, 2H), 7.28 (d, J = 3.7 Hz, 2H), 6.89 (d, J = 8.7 Hz, 2H), 4.71 (d, J = 5.5 Hz, 2H), 4.40 (d, J = 25.4 Hz, 2H), 3.83 (d, J = 6.7 Hz, 3H), 3.36 (d, J = 8.7 Hz, 2H), 3.17 (d, J = 14.0 Hz, 8H), 2.72 (t, J = 6.6 Hz, 2H). LCMS (ESI) calcd for C₃₄H₃₆F₃N₈O₄S⁺ [M+H]⁺: 709.25; found, 709.3.

### Example 542: Preparation of 1-(6-((4-(4-((4-((3-(methylsulfonyl)benzyl)amino)-5-(trifluoromethyl)pyrimidin-2-yl)amino)phenyl)piperazin-1-yl)methyl)pyridin-3-yl)dihydropyrimidine-2,4(1H,3H)-dione (GT-07458)

According to the method of step 2 of Scheme I-1, the target compound (GT-07458) was prepared (white solid, 12 mg, yield 21.41%). ¹H NMR (400 MHz, DMSO-d6) δ 10.99 (s, 1H), 10.59 (s, 1H), 9.19 (s, 1H), 8.71 (d, J = 2.5 Hz, 1H), 8.50 (d, J = 8.2 Hz, 1H), 7.96 - 7.87 (m, 2H), 7.81 (d, J = 8.3 Hz, 2H), 7.61 (t, J = 7.7 Hz, 1H), 7.53 (s, 1H), 7.25 (d, J = 8.5 Hz, 2H), 6.93 (d, J = 8.6 Hz, 2H), 4.72 (d, J = 5.4 Hz, 2H), 4.55 (s, 2H), 4.46 (s, 1H), 3.89 (t, J = 6.6 Hz, 2H), 3.45 (d, J = 43.1 Hz, 7H), 3.17 (d, J = 13.0 Hz, 3H), 2.75 (t, J = 6.6 Hz, 2H). LCMS (ESI) calcd for C₃₃H₃₅F₃N₉O₄S⁺ [M+H]⁺: 710.25; found, 710.3.

### Example 543: Preparation of 1-(5-((4-(4-((4-((3-(methylsulfonyl)benzyl)amino)-5-(trifluoromethyl)pyrimidin-2-yl)amino)phenyl)piperazin-1-yl)methyl)pyridin-2-yl)dihydropyrimidine-2,4(1H,3H)-dione (GT-07459)

According to the method of step 2 of Scheme I-1, the target compound (GT-07459) was prepared (white solid, 6 mg, yield 10.70%). ¹H NMR (400 MHz, DMSO-d6) δ 11.69 (d, J = 61.9 Hz, 1H), 10.77 (s, 1H), 10.62 (s, 1H), 9.13 (d, J = 61.1 Hz, 1H), 8.65 (d, J = 1.9 Hz, 1H), 8.47 (d, J = 2.3 Hz, 1H), 8.14 (dd, J = 8.7, 2.3 Hz, 1H), 7.94 - 7.77 (m, 3H), 7.61 (t, J = 7.7 Hz, 1H), 7.53 (s, 1H), 7.26 (d, J = 8.3 Hz, 2H), 6.92 (d, J = 8.5 Hz, 2H), 4.72 (d, J = 5.4 Hz, 2H), 4.42 (s, 2H), 4.11 (s, 2H), 3.79 (d, J = 10.1 Hz, 2H), 3.38 (d, J = 8.8 Hz, 2H), 3.26 - 3.11 (m, 7H), 2.71 (d, J = 6.7 Hz, 2H). LCMS (ESI) calcd for C₃₃H₃₅F₃N₉O₄S⁺ [M+H]⁺: 710.25; found, 710.3.

### Example 544: Preparation of 3-((3-((4-(4-((4-((3-(methylsulfonyl)benzyl)amino)-5-(trifluoromethyl)pyrimidin-2-yl)amino)phenyl)piperazin-1-yl)methyl)phenyl)amino)piperidine-2,6-dione (GT-07460)

According to the method of step 2 of Scheme I-1, the target compound (GT-07460) was prepared (white solid, 22 mg, yield 38.54%). ¹H NMR (400 MHz, DMSO-d6) δ 11.27 (s, 1H), 10.84 (s, 1H), 10.65 (s, 1H), 8.97 (s, 1H), 8.45 (d, J = 11.1 Hz, 1H), 7.89 (s, 1H), 7.81 (d, J = 7.4 Hz, 1H), 7.61 (t, J = 7.7 Hz, 1H), 7.53 (s, 1H), 7.26 (d, J = 8.0 Hz, 2H), 7.16 (t, J = 7.8 Hz, 1H), 7.05 (s, 1H), 6.91 (d, J = 8.5 Hz, 2H), 6.80 (dd, J = 11.0, 4.6 Hz, 2H), 4.72 (d, J = 5.4 Hz, 2H), 4.44 - 4.39 (m, 1H), 4.24 (s, 2H), 3.78 (s, 2H), 3.36 (d, J = 10.7 Hz, 2H), 3.23 - 3.08 (m, 7H), 2.82 (ddd, J = 17.5, 12.5, 5.3 Hz, 1H), 2.59 (dd, J = 14.0, 3.7 Hz, 1H), 2.24 - 2.12 (m, 1H), 1.90 (qd, J = 12.6, 4.6 Hz, 1H). LCMS (ESI) calcd for C₃₅H₃₈F₃N₈O₄S⁺ [M+H]⁺: 723.27; found, 723.3.

### Example 545: Preparation of 1-(5-fluoro-4-((4-(4-((4-((3-(methylsulfonyl)benzyl)amino)-5-(trifluoromethyl)pyrimidin-2-yl)amino)phenyl)piperazin-1-yl)methyl)pyridin-2-yl)dihydropyrimidine-2,4(1H,3H)-dione (GT-07461)

According to the method of step 2 of Scheme I-1, the target compound (GT-07461) was prepared (white solid, 26 mg, yield 45.24%). ¹H NMR (400 MHz, DMSO-d6) δ 11.46 (s, 1H), 10.65 (s, 1H), 10.54 (d, J = 15.4 Hz, 1H), 8.84 (s, 1H), 8.56 (s, 1H), 8.47 - 8.30 (m, 1H), 8.14 (d, J = 5.3 Hz, 1H), 7.90 (s, 1H), 7.81 (d, J = 7.3 Hz, 1H), 7.60 (t, J = 7.7 Hz, 1H), 7.54 (s, 1H), 7.28 (d, J = 7.8 Hz, 2H), 6.92 (d, J = 8.6 Hz, 2H), 4.72 (d, J = 5.5 Hz, 2H), 4.51 (s, 2H), 4.04 (t, J = 6.6 Hz, 2H), 3.51 (s, 4H), 3.33 - 3.09 (m, 7H), 2.72 (t, J = 6.6 Hz, 2H). LCMS (ESI) calcd for C₃₃H₃₄F₄N₉O₄S⁺ [M+H]⁺: 728.24; found, 728.3.

### Example 546: Preparation of 1-(4-((4-(4-((4-((3-(methylsulfonyl)benzyl)amino)-5-(trifluoromethyl)pyrimidin-2-yl)amino)phenyl)piperazin-1-yl)methyl)pyridin-2-yl)dihydropyrimidine-2,4(1H,3H)-dione (GT-07462)

According to the method of step 2 of Scheme I-1, the target compound (GT-07462) was prepared (white solid, 20 mg, yield 35.68%). ¹H NMR (400 MHz, DMSO) δ 11.75 (s, 1H), 10.60 (d, J = 24.9 Hz, 3H), 9.08 (s, 1H), 8.61 - 8.36 (m, 3H), 7.98 (s, 1H), 7.90 (s, 1H), 7.82 (d, J = 10.7 Hz, 2H), 7.62 (dd, J = 17.8, 5.8 Hz, 2H), 7.53 (s, 1H), 7.26 (d, J = 8.6 Hz, 3H), 6.93 (d, J = 7.4 Hz, 2H), 4.81 (s, 1H), 4.73 (s, 2H), 4.47 (s, 2H), 4.08 (d, J = 7.2 Hz, 2H), 3.79 - 3.73 (m, 2H), 3.41 - 3.15 (m, 8H), 2.70 (d, J = 9.0 Hz, 2H). LCMS (ESI) calcd for C₃₃H₃₅F₃N₉O₄S⁺ [M+H]⁺: 710.25; found, 710.3.

### Example 547: Preparation of 1-(5-((4-(4-((4-((3-(methylsulfonyl)benzyl)amino)-5-(trifluoromethyl)pyrimidin-2-yl)amino)phenyl)piperazin-1-yl)methyl)pyridin-3-yl)dihydropyrimidine-2,4(1H,3H)-dione (GT-07463)

According to the method of step 2 of Scheme I-1, the target compound (GT-07463) was prepared (white solid, 32 mg, yield 57.09%). ¹H NMR (400 MHz, DMSO-d6) δ 11.74 (s, 1H), 10.65 (s, 1H), 10.36 (s, 1H), 8.79 (d, J = 2.4 Hz, 1H), 8.68 (d, J = 1.6 Hz, 1H), 8.37 (s, 1H), 8.27 (s, 1H), 7.90 (s, 1H), 7.81 (d, J = 7.2 Hz, 1H), 7.59 (dd, J = 19.5, 11.8 Hz, 2H), 7.29 (d, J = 7.1 Hz, 2H), 6.90 (d, J = 8.7 Hz, 2H), 4.72 (d, J = 5.5 Hz, 2H), 4.50 (s, 2H), 3.95 (t, J = 6.6 Hz, 4H), 3.42 (s, 2H), 3.22 - 3.10 (m, 7H), 2.77 (t, J = 6.6 Hz, 2H). LCMS (ESI) calcd for C₃₃H₃₅F₃N₉O₄S⁺ [M+H]⁺: 710.25; found, 710.3.

### Example 548: Preparation of 3-(6-fluoro-5-((4-(4-((4-((3-(methylsulfonyl)benzyl)amino)-5-(trifluoromethyl)pyrimidin-2-yl)amino)phenyl)piperazin-1-yl)methyl)pyridin-3-yl)piperidine-2,6-dione (GT-07464)

According to the method of step 2 of Scheme I-1, the target compound (GT-07464) was prepared (white solid, 16 mg, yield 27.88%). ¹H NMR (400 MHz, DMSO-d6) δ 11.67 (s, 1H), 11.00 (s, 1H), 10.60 (s, 1H), 8.91 (s, 1H), 8.45 (d, J = 17.0 Hz, 1H), 8.33 (dd, J = 9.2, 2.2 Hz, 1H), 8.26 (d, J = 1.6 Hz, 1H), 7.91 (s, 1H), 7.82 (d, J = 7.3 Hz, 1H), 7.60 (dd, J = 19.9, 12.2 Hz, 2H), 7.28 (d, J = 7.8 Hz, 2H), 6.94 (t, J = 14.4 Hz, 2H), 4.73 (d, J = 5.5 Hz, 2H), 4.45 (d, J = 15.5 Hz, 2H), 4.07 (dd, J = 12.5, 4.8 Hz, 2H), 3.46 (d, J = 22.3 Hz, 2H), 3.33 - 3.13 (m, 8H), 2.81 - 2.71 (m, 1H), 2.62 (dd, J = 13.8, 3.5 Hz, 1H), 2.32 (qd, J = 12.9, 4.3 Hz, 1H), 2.18 - 2.08 (m, 1H). LCMS (ESI) calcd for C₃₄H₃₅F₄N₈O₄S⁺ [M+H]⁺: 727.24; found, 727.3.

### Example 549: Preparation of 3-(5-((4-(4-((4-((3-(methylsulfonyl)benzyl)amino)-5-(trifluoromethyl)pyrimidin-2-yl)amino)phenyl)piperazin-1-yl)methyl)pyridin-2-yl)piperidine-2,6-dione (GT-07506)

According to the method of step 2 of Scheme I-1, the target compound (GT-07506) was prepared (white solid, 12 mg, yield 21.44%). ¹H NMR (500 MHz, DMSO) δ 11.87 (s, 1H), 11.00 (d, J = 19.8 Hz, 1H), 10.80 (s, 1H), 9.02 (d, J = 60.9 Hz, 1H), 8.83 (t, J = 11.3 Hz, 1H), 8.49 (s, 1H), 8.30 (ddd, J = 17.5, 8.1, 1.9 Hz, 1H), 7.92 (d, J = 19.6 Hz, 1H), 7.81 (t, J = 14.7 Hz, 1H), 7.66 - 7.58 (m, 2H), 7.53 (s, 1H), 7.24 (dd, J = 31.2, 7.6 Hz, 2H), 6.93 (d, J = 8.0 Hz, 2H), 4.73 (d, J = 5.3 Hz, 2H), 4.48 (s, 2H), 4.23 - 4.21 (m, 1H), 3.79 (s, 2H), 3.40 (s, 2H), 3.28 - 3.15 (m, 7H), 2.68 (ddd, J = 16.3, 10.8, 5.3 Hz, 1H), 2.62 - 2.54 (m, 1H), 2.41 - 2.28 (m, 1H), 2.23 - 2.12 (m, 1H). LCMS (ESI) calcd for C₃₄H₃₆F₃N₈O₄S⁺ [M+H]⁺: 709.25; found, 709.3.

### Example 550: Preparation of 3-(4-((4-(4-((4-((3-(methylsulfonyl)benzyl)amino)-5-(trifluoromethyl)pyrimidin-2-yl)amino)phenyl)piperazin-1-yl)methyl)pyridin-2-yl)piperidine-2,6-dione (GT-07465)

According to the method of step 2 of Scheme I-1, the target compound (GT-07465) was prepared (white solid, 6 mg, yield 10.72%). ¹H NMR (400 MHz, DMSO-d6) δ 11.49 (s, 1H), 10.98 (d, J = 22.9 Hz, 1H), 10.33 (s, 1H), 8.63 (dd, J = 33.8, 5.4 Hz, 2H), 8.36 (s, 1H), 7.92 (d, J = 18.3 Hz, 2H), 7.81 (d, J = 7.3 Hz, 1H), 7.72 (d, J = 4.4 Hz, 1H), 7.59 (dd, J = 19.7, 12.0 Hz, 2H), 7.29 (d, J = 6.2 Hz, 2H), 6.90 (d, J = 8.4 Hz, 2H), 4.71 (d, J = 5.4 Hz, 2H), 4.47 (d, J = 8.4 Hz, 2H), 3.78 (s, 2H), 3.41 (s, 3H), 3.17 (d, J = 15.9 Hz, 7H), 2.69 - 2.57 (m, 1H), 2.40 - 2.16 (m, 3H). LCMS (ESI) calcd for C₃₄H₃₆F₃N₈O₄S⁺ [M+H]⁺: 709.25; found, 709.3.

### Example 551: Preparation of N-(3-(((2-((4-(4-((5-(2,4-dioxotetrahydropyrimidin-1(2H)-yl)pyridin-2-yl)methyl)piperazin-1-yl)phenyl)amino)-5-(trifluoromethyl)pyrimidin-4-yl)amino)methyl)pyrazin-2-yl)-N-methylmethanesulfonamide (GT-07403)

According to the method of step 2 of Scheme I-1, the target compound (GT-07403) was prepared (white solid, 16 mg, yield 29.03%). ¹H NMR (400 MHz, DMSO-d6) δ 10.97 (s, 1H), 10.85 (s, 1H), 9.10 (s, 1H), 8.61 (d, J = 2.0 Hz, 1H), 8.49 (s, 1H), 7.95 - 7.86 (m, 2H), 7.82 (d, J = 7.5 Hz, 1H), 7.76 (t, J = 7.2 Hz, 1H), 7.62 (t, J = 7.7 Hz, 1H), 7.54 (s, 1H), 7.24 (dd, J = 23.4, 8.6 Hz, 2H), 6.94 (d, J = 8.5 Hz, 2H), 4.73 (d, J = 5.5 Hz, 2H), 4.56 (s, 2H), 4.09 (d, J = 4.8 Hz, 3H), 3.45 (d, J = 37.5 Hz, 6H), 3.16 (d, J = 5.4 Hz, 3H), 2.82 - 2.71 (m, 1H), 2.64 - 2.55 (m, 1H), 2.33 (qd, J = 12.9, 4.4 Hz, 1H), 2.13 - 2.02 (m, 1H). LCMS (ESI) calcd for C₃₂H₃₆F₃N₁₂O₄S⁺ [M+H]⁺: 741.27; found, 741.3.

### Example 552: Preparation of 3-(5-((4-(4-((4-((3-(methylsulfonyl)benzyl)amino)-5-(trifluoromethyl)pyrimidin-2-yl)amino)phenyl)piperazin-1-yl)methyl)pyridin-3-yl)piperidine-2,6-dione (GT-07498)

According to the method of step 2 of Scheme I-1, the target compound (GT-07498) was prepared (white solid, 10 mg, yield 17.87%). ¹H NMR (400 MHz, DMSO-d6) δ 11.97 (s, 1H), 11.06 (s, 1H), 10.59 (s, 1H), 8.97 (s, 1H), 8.81 (s, 1H), 8.46 (d, J = 25.6 Hz, 2H), 7.91 (s, 1H), 7.82 (d, J = 7.5 Hz, 1H), 7.61 (t, J = 7.7 Hz, 1H), 7.55 (s, 1H), 7.28 (d, J = 8.3 Hz, 2H), 6.93 (d, J = 8.6 Hz, 2H), 4.73 (d, J = 5.4 Hz, 2H), 4.57 (s, 2H), 4.21 - 4.16 (m, 2H), 3.79 (s, 2H), 3.43 (s, 2H), 3.28 - 3.15 (m, 6H), 2.84 - 2.70 (m, 1H), 2.64 (d, J = 17.0 Hz, 1H), 2.37 (dt, J = 12.6, 8.4 Hz, 1H), 2.24 - 2.11 (m, 1H). LCMS (ESI) calcd for C₃₄H₃₆F₃N₈O₄S⁺ [M+H]⁺: 709.25; found, 709.3.

### Example 553: Preparation of 3-(3-((4-(4-((4-((3-(methylsulfonyl)benzyl)amino)-5-(trifluoromethyl)pyrimidin-2-yl)amino)phenyl)piperazin-1-yl)methyl)pyridin-2-yl)piperidine-2,6-dione (GT-07516)

According to the method of step 2 of Scheme I-1, the target compound (GT-07516) was prepared (white solid, 24 mg, yield 42.88%). ¹H NMR (500 MHz, DMSO) δ 11.59 (s, 1H), 10.93 (s, 1H), 10.72 (s, 1H), 9.02 (s, 1H), 8.65 (dd, J = 4.8, 1.5 Hz, 1H), 8.47 (s, 1H), 8.37 (d, J = 7.8 Hz, 1H), 7.90 (s, 1H), 7.82 (d, J = 7.4 Hz, 1H), 7.61 (t, J = 7.7 Hz, 1H), 7.50 (dd, J = 7.8, 4.8 Hz, 1H), 7.26 (d, J = 7.4 Hz, 1H), 6.93 (d, J = 7.6 Hz, 2H), 4.82 (dd, J = 11.2, 4.8 Hz, 1H), 4.72 (d, J = 5.3 Hz, 2H), 4.60 - 4.51 (m, 2H), 3.79 (s, 2H), 3.53 (s, 1H), 3.34 (d, J = 37.1 Hz, 5H), 3.16 (s, 3H), 2.81 - 2.72 (m, 1H), 2.67 - 2.60 (m, 1H), 2.56 - 2.51 (m, 1H), 2.23 - 2.15 (m, 1H). LCMS (ESI) calcd for C₃₄H₃₆F₃N₈O₄S⁺ [M+H]⁺: 709.25; found, 709.3.

### Example 554: Preparation of 2-((2-((4-(1-(4-(2,4-dioxotetrahydropyrimidin-1(2H)-yl)benzyl)piperidin-4-yl)-2-isopropoxy-5-methylphenyl)amino)-5-(trifluoromethyl)pyridin-4-yl)amino)-N-methylbenzamide (GT-07667)

According to the method of step 2 of Scheme I-1, the target compound (GT-07667) was prepared (white solid, 16 mg, yield 29.13%). ¹H NMR (400 MHz, DMSO-d6) δ 11.35 (d, J = 43.7 Hz, 1H), 10.62 (s, 1H), 10.45 (s, 1H), 9.90 (d, J = 57.2 Hz, 1H), 8.81 (d, J = 4.5 Hz, 1H), 8.26 (s, 1H), 7.77 (d, J = 7.8 Hz, 1H), 7.70 (q, J = 4.7 Hz, 2H), 7.61 (d, J = 8.1 Hz, 1H), 7.53 (t, J = 7.6 Hz, 1H), 7.44 (d, J = 8.5 Hz, 2H), 7.26 (d, J = 7.4 Hz, 1H), 7.19 (s, 1H), 6.63 (s, 1H), 4.56 - 4.41 (m, 1H), 4.31 (d, J = 4.5 Hz, 2H), 3.83 (t, J = 6.6 Hz, 2H), 3.41 (d, J = 11.0 Hz, 2H), 3.16 - 2.93 (m, 4H), 2.74 (dd, J = 14.1, 5.6 Hz, 5H), 2.26 (d, J = 8.2 Hz, 4H), 1.84 (d, J = 12.7 Hz, 2H), 1.20 (d, J = 6.0 Hz, 6H). LCMS (ESI) calcd for C₄₀H₄₅F₃N₇O₄⁺ [M+H]⁺: 744.35; found, 744.4.

### Example 555: Preparation of 2-((2-((4-(1-(3-(2,4-dioxotetrahydropyrimidin-1(2H)-yl)benzyl)piperidin-4-yl)-2-isopropoxy-5-methylphenyl)amino)-5-(trifluoromethyl)pyridin-4-yl)amino)-N-methylbenzamide

According to the method of step 2 of Scheme I-1, the target compound (GT-07668) was prepared (white solid, 16 mg, yield 29.13%). ¹H NMR (400 MHz, DMSO-d6) δ 11.33 (s, 1H), 10.62 (s, 1H), 10.46 (s, 1H), 9.82 (s, 1H), 8.82 (d, J = 4.4 Hz, 1H), 8.27 (s, 1H), 7.77 (d, J = 7.0 Hz, 1H), 7.68 (s, 1H), 7.61 (d, J = 8.0 Hz, 1H), 7.51 (ddd, J = 12.5, 8.5, 4.8 Hz, 4H), 7.26 (t, J = 7.5 Hz, 1H), 7.20 (s, 1H), 6.94 (s, 1H), 6.65 (d, J = 14.6 Hz, 1H), 4.48 (dt, J = 12.2, 6.0 Hz, 1H), 4.33 (d, J = 4.4 Hz, 2H), 3.89 (t, J = 6.6 Hz, 2H), 3.41 (s, 2H), 3.15 - 2.96 (m, 3H), 2.84 - 2.67 (m, 5H), 2.33 - 2.18 (m, 5H), 1.83 (d, J = 12.6 Hz, 2H), 1.21 (dd, J = 14.5, 6.0 Hz, 6H). LCMS (ESI) calcd for C₄₀H₄₅F₃N₇O₄⁺ [M+H]⁺: 744.35; found, 744.4.

### Example 556: Preparation of 2-((2-((4-(1-((5-(2,4-dioxotetrahydropyrimidin-1(2H)-yl)pyridin-2-yl)methyl)piperidin-4-yl)-2-isopropoxy-5-methylphenyl)amino)-5-(trifluoromethyl)pyridin-4-yl)amino)-N-methylbenzamide (GT-07669)

According to the method of step 2 of Scheme I-1, the target compound (GT-07669) was prepared (white solid, 22 mg, yield 39.99%). ¹H NMR (400 MHz, DMSO-d6) δ 10.90 (s, 1H), 10.62 (d, J = 18.0 Hz, 2H), 9.88 (s, 1H), 8.81 (d, J = 4.6 Hz, 1H), 8.71 (d, J = 2.5 Hz, 1H), 8.28 (s, 1H), 7.92 (dd, J = 8.4, 2.5 Hz, 1H), 7.80 (dd, J = 17.6, 7.8 Hz, 2H), 7.63 (d, J = 8.1 Hz, 1H), 7.54 (t, J = 7.6 Hz, 1H), 7.27 (t, J = 7.6 Hz, 1H), 7.20 (s, 1H), 6.91 (s, 1H), 6.63 (s, 1H), 4.48 (s, 3H), 3.90 (d, J = 6.6 Hz, 2H), 3.49 (d, J = 10.1 Hz, 2H), 3.22 (s, 2H), 3.02 (s, 1H), 2.76 (t, J = 5.5 Hz, 5H), 2.26 (s, 3H), 2.22 - 2.10 (m, 2H), 1.84 (d, J = 13.0 Hz, 2H), 1.21 (d, J = 6.0 Hz, 6H). LCMS (ESI) calcd for C₃₉H₄₄F₃N₈O₄⁺ [M+H]⁺: 745.34; found, 745.4.

### Example 557: Preparation of 2-((2-((4-(1-((6-(2,4-dioxotetrahydropyrimidin-1(2H)-yl)pyridin-3-yl)methyl)piperidin-4-yl)-2-isopropoxy-5-methylphenyl)amino)-5-(trifluoromethyl)pyridin-4-yl)amino)-N-methylbenzamide (GT-07670)

According to the method of step 2 of Scheme I-1, the target compound (GT-07670) was prepared (white solid, 22 mg, yield 39.99%). ¹H NMR (400 MHz, DMSO-d6) δ 11.57 (d, J = 40.2 Hz, 1H), 10.64 (d, J = 12.2 Hz, 2H), 9.98 (s, 1H), 8.81 (s, 1H), 8.64 (d, J = 1.9 Hz, 1H), 8.27 (s, 1H), 8.15 (d, J = 8.3 Hz, 1H), 7.94 - 7.83 (m, 1H), 7.78 (dd, J = 7.8, 5.4 Hz, 1H), 7.62 (d, J = 7.8 Hz, 1H), 7.54 (t, J = 7.7 Hz, 1H), 7.28 (t, J = 7.6 Hz, 1H), 7.17 (s, 1H), 6.94 (s, 1H), 6.61 (s, 1H), 4.49 (dt, J = 12.2, 6.1 Hz, 1H), 4.36 (d, J = 4.3 Hz, 2H), 4.11 (t, J = 6.6 Hz, 2H), 3.43 (d, J = 10.7 Hz, 2H), 3.15 - 2.95 (m, 3H), 2.80 - 2.66 (m, 6H), 2.31 - 2.16 (m, 5H), 1.84 (d, J = 12.9 Hz, 2H), 1.21 (dd, J = 14.7, 6.0 Hz, 6H). LCMS (ESI) calcd for C₃₉H₄₄F₃N₈O₄⁺ [M+H]⁺: 745.34; found, 745.4.

### Example 558: Preparation of 2-((2-((4-(1-(3-((2,6-dioxopiperidin-3-yl)amino)benzyl)piperidin-4-yl)-2-isopropoxy-5-methylphenyl)amino)-5-(trifluoromethyl)pyridin-4-yl)amino)-N-methylbenzamide (GT-07671)

According to the method of step 2 of Scheme I-1, the target compound (GT-07671) was prepared (white solid, 26 mg, yield 46.45%). ¹H NMR (400 MHz, DMSO-d6) δ 10.91 (d, J = 42.0 Hz, 2H), 10.59 (s, 1H), 9.69 (s, 1H), 8.77 (s, 1H), 8.25 (s, 1H), 7.76 (d, J = 7.7 Hz, 1H), 7.66 - 7.59 (m, 1H), 7.53 (t, J = 7.7 Hz, 1H), 7.29 - 7.13 (m, 3H), 7.05 (s, 1H), 6.93 (s, 1H), 6.79 (d, J = 8.0 Hz, 2H), 6.65 (d, J = 16.5 Hz, 1H), 4.47 (ddd, J = 17.8, 12.0, 5.5 Hz, 2H), 4.16 (d, J = 5.4 Hz, 2H), 3.40 (s, 2H), 3.09 - 2.81 (m, 4H), 2.75 (d, J = 4.5 Hz, 3H), 2.66 - 2.56 (m, 1H), 2.29 - 2.14 (m, 6H), 1.92 - 1.77 (m, 3H), 1.20 (t, J = 5.2 Hz, 6H). LCMS (ESI) calcd for C₄₁H₄₇F₃N₇O₄⁺ [M+H]⁺: 758.36; found, 758.2.

### Example 559: Preparation of 2-((2-((4-(1-((2-(2,4-dioxotetrahydropyrimidin-1(2H)-yl)-5-fluoropyridin-4-yl)methyl)piperidin-4-yl)-2-isopropoxy-5-methylphenyl)amino)-5-(trifluoromethyl)pyridin-4-yl)amino)-N-methylbenzamide (GT-07672)

According to the method of step 2 of Scheme I-1, the target compound (GT-07672) was prepared (white solid, 29 mg, yield 51.47%). ¹H NMR (400 MHz, DMSO-d6) δ 11.46 (s, 1H), 10.64 (d, J = 19.0 Hz, 2H), 9.81 (s, 1H), 8.81 (s, 1H), 8.57 (s, 1H), 8.27 (s, 1H), 8.16 (d, J = 5.3 Hz, 1H), 7.77 (d, J = 7.7 Hz, 1H), 7.62 (d, J = 8.1 Hz, 1H), 7.54 (d, J = 7.5 Hz, 1H), 7.26 (t, J = 7.6 Hz, 1H), 7.21 (s, 1H), 6.89 (s, 1H), 6.64 (s, 1H), 4.51 - 4.40 (m, 3H), 4.05 (t, J = 6.6 Hz, 2H), 3.52 (d, J = 10.7 Hz, 2H), 3.19 (d, J = 8.1 Hz, 2H), 3.00 (s, 1H), 2.79 - 2.69 (m, 5H), 2.22 (d, J = 19.9 Hz, 5H), 1.84 (d, J = 12.4 Hz, 2H), 1.20 (d, J = 6.0 Hz, 6H). LCMS (ESI) calcd for C₃₉H₄₃F₄N₈O₄⁺ [M+H]⁺: 763.33; found, 763.4.

### Example 560: Preparation of 2-((2-((4-(1-((2-(2,4-dioxotetrahydropyrimidin-1(2H)-yl)pyridin-4-yl)methyl)piperidin-4-yl)-2-isopropoxy-5-methylphenyl)amino)-5-(trifluoromethyl)pyridin-4-yl)amino)-N-methylbenzamide (GT-07673)

According to the method of step 2 of Scheme I-1, the target compound (GT-07673) was prepared (white solid, 29 mg, yield 52.72%). ¹H NMR (400 MHz, DMSO-d6) δ 11.63 (s, 1H), 10.79 - 10.59 (m, 2H), 10.12 (s, 1H), 8.84 (d, J = 4.6 Hz, 1H), 8.53 (t, J = 6.0 Hz, 1H), 8.29 (s, 1H), 8.01 (d, J = 26.1 Hz, 1H), 7.78 (d, J = 7.8 Hz, 1H), 7.69 (d, J = 5.1 Hz, 1H), 7.63 (d, J = 8.0 Hz, 1H), 7.55 (t, J = 7.7 Hz, 1H), 7.29 (t, J = 7.6 Hz, 1H), 7.17 (d, J = 9.9 Hz, 1H), 6.96 (s, 1H), 6.63 (d, J = 13.5 Hz, 1H), 4.50 (dt, J = 12.2, 6.1 Hz, 1H), 4.40 (d, J = 4.4 Hz, 2H), 4.12 - 4.08 (m, 2H), 3.44 (d, J = 10.9 Hz, 2H), 3.21 - 2.97 (m, 3H), 2.72 (ddd, J = 13.3, 10.1, 5.6 Hz, 6H), 2.30 - 2.19 (m, 4H), 1.83 (d, J = 12.8 Hz, 2H), 1.21 (dd, J = 12.4, 6.1 Hz, 6H). LCMS (ESI) calcd for C₃₉H₄₄F₃N₈O₄⁺ [M+H]⁺: 745.34; found, 745.2.

### Example 561: Preparation of 2-((2-((4-(1-((5-(2,4-dioxotetrahydropyrimidin-1(2H)-yl)pyridin-3-yl)methyl)piperidin-4-yl)-2-isopropoxy-5-methylphenyl)amino)-5-(trifluoromethyl)pyridin-4-yl)amino)-N-methylbenzamide (GT-07674)

According to the method of step 2 of Scheme I-1, the target compound (GT-07674) was prepared (white solid, 22 mg, yield 39.99%). ¹H NMR (400 MHz, DMSO-d6) δ 11.58 (s, 1H), 10.65 (s, 2H), 9.91 (s, 1H), 8.78 (s, 2H), 8.67 (s, 1H), 8.26 (s, 2H), 7.77 (d, J = 7.7 Hz, 1H), 7.62 (d, J = 8.1 Hz, 1H), 7.54 (t, J = 7.6 Hz, 1H), 7.28 (t, J = 7.5 Hz, 1H), 7.17 (s, 1H), 6.93 (s, 1H), 6.61 (s, 1H), 4.52 - 4.40 (m, 3H), 3.97 (d, J = 6.5 Hz, 2H), 3.46 (d, J = 10.5 Hz, 2H), 3.20 - 2.98 (m, 3H), 2.77 (dd, J = 10.8, 5.5 Hz, 5H), 2.25 (s, 5H), 1.84 (d, J = 12.5 Hz, 2H), 1.20 (t, J = 7.6 Hz, 6H). LCMS (ESI) calcd for C₃₉H₄₄F₃N₈O₄⁺ [M+H]⁺: 745.34; found, 745.2.

### Example 562: Preparation of 2-((2-((4-(1-((5-(2,6-dioxopiperidin-3-yl)-2-fluoropyridin-3-yl)methyl)piperidin-4-yl)-2-isopropoxy-5-methylphenyl)amino)-5-(trifluoromethyl)pyridin-4-yl)amino)-N-methylbenzamide (GT-07675)

According to the method of step 2 of Scheme I-1, the target compound (GT-07675) was prepared (white solid, 16 mg, yield 28.44%). ¹H NMR (400 MHz, DMSO-d6) δ 11.48 (s, 1H), 11.00 (s, 1H), 10.61 (s, 1H), 9.77 (s, 1H), 8.79 (d, J = 4.6 Hz, 1H), 8.42 - 8.31 (m, 1H), 8.26 (s, 2H), 7.76 (d, J = 7.7 Hz, 1H), 7.61 (d, J = 8.1 Hz, 1H), 7.53 (t, J = 7.7 Hz, 1H), 7.27 (t, J = 7.5 Hz, 1H), 7.20 (s, 1H), 6.90 (s, 1H), 6.63 (s, 1H), 4.47 (dt, J = 12.1, 6.1 Hz, 1H), 4.41 - 4.31 (m, 2H), 4.07 (dd, J = 12.5, 4.8 Hz, 1H), 3.27 - 3.14 (m, 3H), 3.00 (d, J = 12.3 Hz, 1H), 2.81 - 2.71 (m, 5H), 2.66 - 2.59 (m, 1H), 2.33 (dd, J = 12.6, 4.3 Hz, 1H), 2.25 (s, 3H), 2.22 - 2.08 (m, 3H), 1.84 (d, J = 12.5 Hz, 2H), 1.19 (d, J = 6.0 Hz, 6H). LCMS (ESI) calcd for C₄₀H₄₄F₄N₇O₄⁺ [M+H]⁺: 762.34; found, 762.2.

### Example 563: Preparation of 2-((2-((4-(1-((6-(2,6-dioxopiperidin-3-yl)pyridin-3-yl)methyl)piperidin-4-yl)-2-isopropoxy-5-methylphenyl)amino)-5-(trifluoromethyl)pyridin-4-yl)amino)-N-methylbenzamide (GT-07676)

According to the method of step 2 of Scheme I-1, the target compound (GT-07676) was prepared (white solid, 31 mg, yield 56.44%). ¹H NMR (400 MHz, DMSO-d6) δ 11.48 (s, 1H), 10.93 (s, 1H), 10.63 (s, 1H), 9.87 (s, 1H), 8.83 - 8.74 (m, 2H), 8.29 - 8.14 (m, 2H), 7.77 (d, J = 7.8 Hz, 1H), 7.62 (d, J = 8.1 Hz, 1H), 7.54 (t, J = 7.0 Hz, 2H), 7.28 (t, J = 7.5 Hz, 1H), 7.18 (s, 1H), 6.92 (s, 1H), 6.62 (s, 1H), 4.54 - 4.45 (m, 1H), 4.38 (d, J = 4.1 Hz, 2H), 4.14 (dd, J = 9.7, 5.2 Hz, 1H), 3.43 (s, 1H), 3.16 - 2.97 (m, 3H), 2.75 (d, J = 4.4 Hz, 3H), 2.68 - 2.52 (m, 2H), 2.36 - 2.09 (m, 8H), 1.84 (d, J = 12.0 Hz, 2H), 1.20 (t, J = 7.0 Hz, 6H). LCMS (ESI) calcd for C₄₀H₄₅F₃N₇O₄⁺ [M+H]⁺: 744.35; found, 744.4.

### Example 564: Preparation of 2-((2-((4-(1-((2-(2,6-dioxopiperidin-3-yl)pyridin-4-yl)methyl)piperidin-4-yl)-2-isopropoxy-5-methylphenyl)amino)-5-(trifluoromethyl)pyridin-4-yl)amino)-N-methylbenzamide (GT-07677)

According to the method of step 2 of Scheme I-1, the target compound (GT-07677) was prepared (white solid, 16 mg, yield 29.07%). ¹H NMR (400 MHz, DMSO-d6) δ 11.69 (s, 1H), 10.95 (s, 1H), 10.66 (s, 1H), 9.97 (s, 1H), 8.81 (d, J = 4.6 Hz, 1H), 8.67 (d, J = 5.7 Hz, 1H), 8.30 (d, J = 19.4 Hz, 1H), 7.78 (d, J = 4.9 Hz, 3H), 7.62 (d, J = 8.1 Hz, 1H), 7.55 (t, J = 7.5 Hz, 1H), 7.29 (t, J = 7.5 Hz, 1H), 7.19 (d, J = 14.6 Hz, 1H), 6.96 (s, 1H), 6.63 (d, J = 14.4 Hz, 1H), 4.50 (dt, J = 12.1, 6.1 Hz, 1H), 4.41 (d, J = 3.8 Hz, 2H), 4.17 - 4.10 (m, 1H), 3.43 (d, J = 10.2 Hz, 2H), 3.16 - 2.97 (m, 3H), 2.76 (d, J = 4.5 Hz, 3H), 2.70 - 2.63 (m, 1H), 2.60 - 2.54 (m, 1H), 2.34 - 2.17 (m, 7H), 1.83 (d, J = 12.8 Hz, 2H), 1.21 (t, J = 8.8 Hz, 6H). LCMS (ESI) calcd for C₄₀H₄₅F₃N₇O₄⁺ [M+H]⁺: 744.35; found, 744.4.

### Example 565: Preparation of 2-((2-((4-(1-((5-(2,6-dioxopiperidin-3-yl)pyridin-3-yl)methyl)piperidin-4-yl)-2-isopropoxy-5-methylphenyl)amino)-5-(trifluoromethyl)pyridin-4-yl)amino)-N-methylbenzamide (GT-07678)

According to the method of step 2 of Scheme I-1, the target compound (GT-07678) was prepared (white solid, 18 mg, yield 32.77%). ¹H NMR (400 MHz, DMSO-d6) δ 11.65 (s, 1H), 11.04 (s, 1H), 10.64 (s, 1H), 9.92 (s, 1H), 8.90 (s, 1H), 8.80 (d, J = 4.5 Hz, 1H), 8.72 (s, 1H), 8.35 (s, 1H), 8.27 (s, 1H), 7.77 (d, J = 7.7 Hz, 1H), 7.62 (d, J = 8.1 Hz, 1H), 7.54 (t, J = 7.7 Hz, 1H), 7.28 (t, J = 7.5 Hz, 1H), 7.18 (s, 1H), 6.93 (s, 1H), 6.64 (d, J = 16.0 Hz, 1H), 4.49 (dd, J = 12.0, 5.9 Hz, 3H), 4.13 (d, J = 7.7 Hz, 1H), 3.53 - 3.41 (m, 2H), 3.18 = 2.98 (m, 3H), 2.75 (d, J = 4.4 Hz, 4H), 2.63 (d, J = 17.4 Hz, 1H), 2.42 - 2.32 (m, 1H), 2.25 (s, 5H), 2.19 - 2.12 (m, 1H), 1.84 (d, J = 13.1 Hz, 2H), 1.20 (dd, J = 9.7, 4.8 Hz, 6H). LCMS (ESI) calcd for C₄₀H₄₅F₃N₇O₄⁺ [M+H]⁺: 744.35; found, 744.2.

### Example 566: Preparation of 2-((2-((4-(1-((5-(2,4-dioxotetrahydropyrimidin-1(2H)-yl)-2-fluoropyridin-3-yl)methyl)piperidin-4-yl)-2-isopropoxy-5-methylphenyl)amino)-5-(trifluoromethyl)pyridin-4-yl)amino)-N-methylbenzamide (GT-07679)

According to the method of step 2 of Scheme I-1, the target compound (GT-07679) was prepared (white solid, 30 mg, yield 53.25%). ¹H NMR (400 MHz, DMSO-d6) δ 11.64 (s, 1H), 10.62 (t, J = 16.5 Hz, 2H), 9.96 (s, 1H), 8.82 (d, J = 4.6 Hz, 1H), 8.51 - 8.37 (m, 2H), 8.32 - 7.96 (m, 2H), 7.78 (d, J = 7.6 Hz, 1H), 7.62 (d, J = 8.2 Hz, 1H), 7.54 (t, J = 7.8 Hz, 1H), 7.28 (t, J = 7.6 Hz, 1H), 7.19 (d, J = 11.2 Hz, 1H), 6.92 (s, 1H), 6.62 (s, 1H), 4.54 - 4.35 (m, 3H), 3.94 (t, J = 6.5 Hz, 2H), 3.49 (d, J = 10.2 Hz, 2H), 3.22 (d, J = 11.1 Hz, 2H), 3.02 (t, J = 11.8 Hz, 1H), 2.79 - 2.71 (m, 5H), 2.34 - 2.17 (m, 5H), 1.84 (d, J = 12.6 Hz, 2H), 1.20 (dd, J = 10.4, 9.5 Hz, 6H). LCMS (ESI) calcd for C₃₉H₄₃F₄N₈O₄⁺ [M+H]⁺: 763.33; found, 763.3.

### Example 567: Preparation of 2-((2-((4-(1-((5-(2,4-dioxotetrahydropyrimidin-1(2H)-yl)pyrazin-2-yl)methyl)piperidin-4-yl)-2-isopropoxy-5-methylphenyl)amino)-5-(trifluoromethyl)pyridin-4-yl)amino)-N-methylbenzamide (GT-07680)

According to the method of step 2 of Scheme I-1, the target compound (GT-07680) was prepared (white solid, 24 mg, yield 43.57%). ¹H NMR (400 MHz, DMSO-d6) δ 11.40 (s, 1H), 10.83 (s, 1H), 10.62 (s, 1H), 9.82 (s, 1H), 9.16 (d, J = 1.2 Hz, 1H), 8.90 - 8.79 (m, 2H), 8.28 (s, 1H), 7.77 (d, J = 7.4 Hz, 1H), 7.62 (d, J = 8.1 Hz, 1H), 7.53 (t, J = 7.5 Hz, 1H), 7.26 (t, J = 7.5 Hz, 1H), 7.21 (s, 1H), 6.90 (s, 1H), 6.64 (s, 1H), 4.57 - 4.45 (m, 3H), 4.10 (t, J = 6.6 Hz, 2H), 3.55 (s, 2H), 3.21 (d, J = 11.0 Hz, 2H), 3.00 (s, 1H), 2.75 (t, J = 5.7 Hz, 5H), 2.30 - 2.16 (m, 5H), 1.85 (d, J = 12.5 Hz, 2H), 1.20 (d, J = 6.0 Hz, 6H). LCMS (ESI) calcd for C₃₈H₄₃F₃N₉O₄⁺ [M+H]⁺: 746.34; found, 746.3.

### Example 568: Preparation of 2-(2-((cyclopropylmethyl)amino)pyridin-4-yl)-N-(3-(difluoromethyl)-1-(4-(4-(3-(2,4-dioxotetrahydropyrimidin-1(2H)-yl)benzyl)piperazin-1-yl)phenyl)-1H-pyrazol-4-yl)oxazole-4-carboxamide (GT-04968)

According to the method of step 2 of Scheme I-1, the target compound (GT-04968) was prepared (white solid, 35 mg, yield 59.84%). ¹H NMR (400 MHz, DMSO) δ 11.31 (s, 1H), 10.46 (s, 1H), 10.13 (s, 1H), 9.14 (s, 1H), 8.70 (s, 1H), 8.09 (d, J = 6.5 Hz, 1H), 7.73 (d, J = 9.0 Hz, 2H), 7.64 (d, J = 8.5 Hz, 2H), 7.53 - 7.46 (m, 3H), 7.29 (dd, J = 56.2, 52.2 Hz, 2H), 7.12 (s, 1H), 4.41 (s, 2H), 3.89 (dd, J = 13.9, 7.4 Hz, 4H), 3.32 - 3.13 (m, 8H), 2.74 (t, J = 6.6 Hz, 2H), 1.20 - 1.09 (m, 1H), 0.65 - 0.50 (m, 2H), 0.33 (q, J = 4.8 Hz, 2H). LCMS (ESI) calcd for C₃₈H₃₈F₂N₁₀O₄⁺ [M+H]⁺: 737.31; found, 737.3.

### Example 569: Preparation of 2-(2-((cyclopropylmethyl)amino)pyridin-4-yl)-N-(3-(difluoromethyl)-1-(4-(4-((6-(2,4-dioxotetrahydropyrimidin-1(2H)-yl)pyridin-3-yl)methyl)piperazin-1-yl)phenyl)-1H-pyrazol-4-yl)oxazole-4-carboxamide (GT-04970)

According to the method of step 2 of Scheme I-1, the target compound (GT-04970) was prepared (white solid, 33 mg, yield 53.25%). ¹H NMR (400 MHz, DMSO) δ 11.58 (s, 1H), 10.62 (s, 1H), 10.14 (s, 1H), 9.16 (s, 1H), 8.70 (s, 1H), 8.64 (d, J = 1.9 Hz, 1H), 8.17 - 8.05 (m, 2H), 7.88 (d, J = 8.6 Hz, 1H), 7.74 (d, J = 9.0 Hz, 2H), 7.68 (s, 1H), 7.34 (dd, J = 31.0, 23.2 Hz, 2H), 7.14 (d, J = 9.1 Hz, 2H), 4.44 (s, 2H), 4.11 (t, J = 6.5 Hz, 2H), 3.91 (d, J = 12.2 Hz, 2H), 3.36 - 3.19 (m, 8H), 2.71 (t, J = 6.6 Hz, 2H), 1.21 - 1.10 (m, 1H), 0.61 - 0.54 (m, 2H), 0.34 (q, J = 4.9 Hz, 2H). LCMS (ESI) calcd for C₃₇H₃₈F₂N₁₁O₄⁺ [M+H]⁺: 738.31; found, 738.4.

### Example 570: Preparation of 2-(2-((cyclopropylmethyl)amino)pyridin-4-yl)-N-(3-(difluoromethyl)-1-(4-(4-(3-((2,6-dioxopiperidin-3-yl)amino)benzyl)piperazin-1-yl)phenyl)-1H-pyrazol-4-yl)oxazole-4-carboxamide (GT-04969)

According to the method of step 2 of Scheme I-1, the target compound (GT-04969) was prepared (white solid, 20 mg, yield 33.28%). ¹H NMR (400 MHz, DMSO) δ 11.01 (s, 1H), 10.85 (s, 1H), 10.11 (s, 1H), 9.14 (s, 1H), 8.70 (s, 1H), 8.10 (d, J = 6.5 Hz, 1H), 7.73 (d, J = 9.0 Hz, 2H), 7.61 (s, 1H), 7.30 (dd, J = 47.7, 44.2 Hz, 2H), 7.15 (dd, J = 13.2, 8.7 Hz, 3H), 7.00 (s, 1H), 6.80 (d, J = 8.0 Hz, 2H), 4.46 - 4.37 (m, 1H), 4.26 (s, 2H), 3.89 (d, J = 12.2 Hz, 2H), 3.28 - 3.11 (m, 7H), 2.82 (s, 1H), 2.63 (s, 1H), 2.25 - 2.14 (m, 1H), 2.00 - 1.85 (m, 1H), 1.15 (s, 1H), 0.61 - 0.53 (m, 2H), 0.34 (t, J = 5.0 Hz, 2H). LCMS (ESI) calcd for C₃₉H₄₁F₂N₁₀O₄⁺ [M+H]⁺: 751.33; found, 751.4.

### Example 571: Preparation of 2-(2-((cyclopropylmethyl)amino)pyridin-4-yl)-N-(3-(difluoromethyl)-1-(1-(4-(2,4-dioxotetrahydropyrimidin-1(2H)-yl)benzyl)piperidin-4-yl)-1H-pyrazol-4-yl)oxazole-4-carboxamide (GT-04956)

According to the method of step 2 of Scheme I-1, the target compound (GT-04956) was prepared (white solid, 27 mg, yield 44.46%). ¹H NMR (400 MHz, DMSO) δ 10.93 (s, 1H), 10.45 (s, 1H), 9.95 (s, 1H), 9.11 (s, 1H), 8.31 (d, J = 77.3 Hz, 1H), 8.09 (d, J = 6.4 Hz, 1H), 7.68 - 7.63 (m, 2H), 7.57 (d, J = 6.4 Hz, 1H), 7.44 (t, J = 8.3 Hz, 2H), 7.21 (dd, J = 39.4, 30.2 Hz, 2H), 4.59 (s, 1H), 4.34 (d, J = 3.8 Hz, 2H), 3.84 (t, J = 6.6 Hz, 2H), 3.49 (s, 2H), 3.32 (s, 2H), 3.12 (d, J = 11.5 Hz, 2H), 2.73 (t, J = 6.6 Hz, 2H), 2.42 - 2.23 (m, 4H), 1.22 - 1.09 (m, 1H), 0.56 (q, J = 5.6 Hz, 2H), 0.32 (q, J = 4.8 Hz, 2H). LCMS (ESI) calcd for C₃₃H₃₆F₂N₉O₄⁺ [M+H]⁺: 660.29; found, 660.3.

### Example 572: Preparation of 2-(2-((cyclopropylmethyl)amino)pyridin-4-yl)-N-(3-(difluoromethyl)-1-(1-(3-(2,4-dioxotetrahydropyrimidin-1(2H)-yl)benzyl)piperidin-4-yl)-1H-pyrazol-4-yl)oxazole-4-carboxamide (GT-04965)

According to the method of step 2 of Scheme I-1, the target compound (GT-04965) was prepared (white solid, 31 mg, yield 51.62%). ¹H NMR (400 MHz, DMSO) δ 10.95 (s, 1H), 10.45 (d, J = 7.0 Hz, 1H), 9.96 (s, 1H), 9.12 (d, J = 8.2 Hz, 1H), 8.31 (d, J = 76.7 Hz, 1H), 8.08 (d, J = 6.4 Hz, 1H), 7.63 (t, J = 11.1 Hz, 2H), 7.55 - 7.45 (m, 3H), 7.28 (dd, J = 30.4, 24.0 Hz, 2H), 4.55 (s, 1H), 4.40 (d, J = 37.7 Hz, 2H), 3.88 (t, J = 6.5 Hz, 2H), 3.51 (s, 2H), 3.32 (s, 2H), 3.12 (d, J = 11.1 Hz, 2H), 2.74 (t, J = 6.5 Hz, 2H), 2.42 - 2.20 (m, 4H), 1.19 - 1.09 (m, 1H), 0.56 (q, J = 5.5 Hz, 2H), 0.32 (q, J = 4.8 Hz, 2H). LCMS (ESI) calcd for C₃₃H₃₆F₂N₉O₄⁺ [M+H]⁺: 660.29; found, 660.3.

### Example 573: Preparation of 2-(2-((cyclopropylmethyl)amino)pyridin-4-yl)-N-(3-(difluoromethyl)-1-(1-((5-(2,4-dioxotetrahydropyrimidin-1(2H)-yl)pyridin-2-yl)methyl)piperidin-4-yl)-1H-pyrazol-4-yl)oxazole-4-carboxamide (GT-04958)

According to the method of step 2 of Scheme I-1, the target compound (GT-04958) was prepared (white solid, 25 mg, yield 41.76%). ¹H NMR (400 MHz, DMSO) δ 10.93 (s, 1H), 10.59 (s, 1H), 10.02 (s, 1H), 9.50 (s, 1H), 9.15 (s, 1H), 8.71 (d, J = 2.5 Hz, 1H), 8.24 (s, 1H), 8.08 (d, J = 6.7 Hz, 1H), 7.92 (dd, J = 8.4, 2.5 Hz, 1H), 7.77 (d, J = 8.4 Hz, 1H), 7.69 (s, 1H), 7.39 - 7.09 (m, 2H), 4.57 (d, J = 39.4 Hz, 3H), 3.89 (t, J = 6.6 Hz, 2H), 3.55 - 3.52 (m, 2H), 3.37 (d, J = 7.0 Hz, 2H), 3.28 (t, J = 10.9 Hz, 2H), 2.76 (t, J = 6.6 Hz, 2H), 2.35 (dd, J = 38.6, 11.0 Hz, 4H), 1.22 - 1.11 (m, 1H), 0.62 - 0.51 (m, 2H), 0.35 (q, J = 4.8 Hz, 2H). LCMS (ESI) calcd for C₃₂H₃₅F₂N₁₀O₄⁺ [M+H]⁺: 661.28; found, 661.3.

### Example 574: Preparation of 2-(2-((cyclopropylmethyl)amino)pyridin-4-yl)-N-(3-(difluoromethyl)-1-(1-((6-(2,4-dioxotetrahydropyrimidin-1(2H)-yl)pyridin-3-yl)methyl)piperidin-4-yl)-1H-pyrazol-4-yl)oxazole-4-carboxamide (GT-04959)

According to the method of step 2 of Scheme I-1, the target compound (GT-04959) was prepared (white solid, 22 mg, yield 36.54%). ¹H NMR (400 MHz, DMSO) δ 11.53 (d, J = 67.0 Hz, 1H), 10.62 (s, 1H), 10.02 (d, J = 13.9 Hz, 1H), 9.16 (d, J = 10.3 Hz, 1H), 8.65 (t, J = 6.4 Hz, 1H), 8.21 (s, 1H), 8.15 - 8.00 (m, 2H), 7.87 (d, J = 8.7 Hz, 1H), 7.69 (s, 1H), 7.38 - 7.08 (m, 2H), 4.61 (dd, J = 32.1, 20.6 Hz, 1H), 4.42 (d, J = 35.0 Hz, 2H), 4.11 (t, J = 6.5 Hz, 2H), 3.50 (d, J = 11.9 Hz, 2H), 3.37 (d, J = 6.9 Hz, 2H), 3.13 (d, J = 11.3 Hz, 2H), 2.71 (t, J = 6.5 Hz, 2H), 2.36 (dt, J = 41.8, 11.4 Hz, 4H), 1.25 - 1.11 (m, 1H), 0.57 (q, J = 5.7 Hz, 2H), 0.35 (q, J = 4.7 Hz, 2H). LCMS (ESI) calcd for C₃₂H₃₅F₂N₁₀O₄⁺ [M+H]⁺: 661.28 ; found, 661.3.

### Example 575: Preparation of 2-(2-((cyclopropylmethyl)amino)pyridin-4-yl)-N-(3-(difluoromethyl)-1-(1-(3-((2,6-dioxopiperidin-3-yl)amino)benzyl)piperidin-4-yl)-1H-pyrazol-4-yl)oxazole-4-carboxamide (GT-04960)

According to the method of step 2 of Scheme I-1, the target compound (GT-04960) was prepared (white solid, 21 mg, yield 33.50%). ¹H NMR (400 MHz, DMSO) δ 10.92 (s, 1H), 10.85 (s, 1H), 9.98 (s, 1H), 9.15 (d, J = 8.3 Hz, 1H), 8.30 (d, J = 78.0 Hz, 1H), 8.01 (dd, J = 56.5, 7.0 Hz, 1H), 7.67 (s, 1H), 7.38 - 7.09 (m, 3H), 7.03 (d, J = 15.0 Hz, 1H), 6.81 - 6.77 (m, 1H), 4.57 (t, J = 11.7 Hz, 1H), 4.40 (dd, J = 11.6, 4.8 Hz, 1H), 4.27 (d, J = 65.5 Hz, 2H), 3.47 (s, 2H), 3.36 (d, J = 6.9 Hz, 2H), 3.16 - 3.06 (m, 2H), 2.82 (td, J = 12.5, 6.2 Hz, 1H), 2.61 (dd, J = 13.8, 3.7 Hz, 1H), 2.44 - 2.15 (m, 5H), 1.91 (dd, J = 12.0, 4.4 Hz, 1H), 1.15 (dd, J = 9.9, 5.0 Hz, 1H), 0.57 (q, J = 5.7 Hz, 2H), 0.34 (q, J = 4.8 Hz, 2H). LCMS (ESI) calcd for C₃₄H₃₈F₂N₉O₄⁺ [M+H]⁺: 674.30; found, 674.3.

### Example 576: Preparation of N-(3-(difluoromethyl)-1-(1-(4-(2,4-dioxotetrahydropyrimidin-1(2H)-yl)benzyl)piperidin-4-yl)-1H-pyrazol-4-yl)-2-(2-((2,2,2-trifluoroethyl)amino)pyridin-4-yl)oxazole-4-carboxamide (GT-05189)

According to the method of step 2 of Scheme I-1, the target compound (GT-05189) was prepared (white solid, 25 mg, yield 34.79%). ¹H NMR (400 MHz, DMSO) δ 11.21 (d, J = 68.6 Hz, 1H), 10.45 (s, 1H), 9.91 (s, 1H), 9.13 - 9.01 (m, 1H), 8.34 (s, 1H), 8.26 - 8.18 (m, 2H), 7.67 (d, J = 8.5 Hz, 1H), 7.44 (d, J = 8.3 Hz, 2H), 7.38 - 7.08 (m, 2H), 4.59 (dd, J = 13.5, 9.7 Hz, 1H), 4.36 (dd, J = 18.0, 6.8 Hz, 4H), 3.83 (d, J = 6.7 Hz, 3H), 3.48 (d, J = 11.3 Hz, 2H), 3.12 (dd, J = 22.3, 10.1 Hz, 2H), 2.74 (d, J = 6.6 Hz, 1H), 2.47 - 2.21 (m, 4H). LCMS (ESI) calcd for C₃₁H₃₁F₅N₉O₄⁺ [M+H]⁺: 688.24; found, 688.3.

### Example 577: Preparation of N-(3-(difluoromethyl)-1-(1-(3-(2,4-dioxotetrahydropyrimidin-1(2H)-yl)benzyl)piperidin-4-yl)-1H-pyrazol-4-yl)-2-(2-((2,2,2-trifluoroethyl)amino)pyridin-4-yl)oxazole-4-carboxamide (GT-05190)

According to the method of step 2 of Scheme I-1, the target compound (GT-05190) was prepared (white solid, 23 mg, yield 32.25%). ¹H NMR (400 MHz, DMSO) δ 10.76 (s, 1H), 10.46 (d, J = 7.2 Hz, 1H), 9.86 (s, 1H), 9.01 (d, J = 7.8 Hz, 1H), 8.27 - 8.14 (m, 2H), 7.88 (s, 1H), 7.63 (d, J = 10.7 Hz, 1H), 7.48 (s, 2H), 7.37 - 7.07 (m, 3H), 4.58 (t, J = 11.5 Hz, 1H), 4.47 - 4.23 (m, 4H), 3.87 (t, J = 6.5 Hz, 2H), 3.43 - 3.30 (m, 2H), 3.12 (d, J = 11.7 Hz, 2H), 2.74 (t, J = 6.5 Hz, 2H), 2.43 - 2.21 (m, 4H). LCMS (ESI) calcd for C₃₁H₃₁F₅N₉O₄⁺ [M+H]⁺: 688.24; found, 688.3.

### Example 578: Preparation of N-(3-(difluoromethyl)-1-(1-((5-(2,4-dioxotetrahydropyrimidin-1(2H)-yl)pyridin-2-yl)methyl)piperidin-4-yl)-1H-pyrazol-4-yl)-2-(2-((2,2,2-trifluoroethyl)amino)pyridin-4-yl)oxazole-4-carboxamide (GT-05191)

According to the method of step 2 of Scheme I-1, the target compound (GT-05191) was prepared (white solid, 13 mg, yield 18.30%). ¹H NMR (400 MHz, DMSO) δ 10.59 (s, 1H), 9.89 (s, 1H), 9.02 (s, 1H), 8.71 (d, J = 2.4 Hz, 1H), 8.24 (d, J = 5.3 Hz, 2H), 8.06 (s, 1H), 7.92 (dd, J = 8.4, 2.6 Hz, 1H), 7.73 (d, J = 8.4 Hz, 1H), 7.37 - 7.08 (m, 3H), 4.57 (d, J = 36.8 Hz, 3H), 4.39 - 4.26 (m, 2H), 3.89 (t, J = 6.6 Hz, 2H), 3.35 (dd, J = 44.9, 11.6 Hz, 4H), 2.76 (t, J = 6.6 Hz, 2H), 2.35 (dd, J = 20.9, 9.0 Hz, 4H). LCMS (ESI) calcd for C₃₀H₃₀F₅N₁₀O₄⁺ [M+H]⁺: 689.24; found, 689.3.

### Example 579: Preparation of N-(3-(difluoromethyl)-1-(1-((6-(2,4-dioxotetrahydropyrimidin-1(2H)-yl)pyridin-3-yl)methyl)piperidin-4-yl)-1H-pyrazol-4-yl)-2-(2-((2,2,2-trifluoroethyl)amino)pyridin-4-yl)oxazole-4-carboxamide (GT-05192)

According to the method of step 2 of Scheme I-1, the target compound (GT-05192) was prepared (white solid, 35 mg, yield 48.95%). ¹H NMR (400 MHz, DMSO) δ 11.68 - 11.25 (m, 1H), 10.62 (s, 1H), 9.93 (s, 1H), 9.08 (d, J = 9.5 Hz, 1H), 8.63 (d, J = 1.9 Hz, 2H), 8.28 - 8.18 (m, 2H), 8.11 (dd, J = 8.7, 2.3 Hz, 1H), 7.86 (d, J = 8.7 Hz, 1H), 7.53 (s, 1H), 7.39 - 7.06 (m, 2H), 4.58 (t, J = 11.5 Hz, 1H), 4.51 - 4.35 (m, 4H), 4.11 (d, J = 6.4 Hz, 2H), 3.49 (d, J = 11.4 Hz, 2H), 3.13 (d, J = 11.2 Hz, 2H), 2.70 (t, J = 6.6 Hz, 2H), 2.35 (dt, J = 39.7, 10.8 Hz, 4H). LCMS (ESI) calcd for C₃₀H₃₀F₅N₁₀O₄⁺ [M+H]⁺: 689.24; found, 689.3.

### Example 580: Preparation of N-(3-(difluoromethyl)-1-(1-(3-((2,6-dioxopiperidin-3-yl)amino)benzyl)piperidin-4-yl)-1H-pyrazol-4-yl)-2-(2-((2,2,2-trifluoroethyl)amino)pyridin-4-yl)oxazole-4-carboxamide (GT-05193)

According to the method of step 2 of Scheme I-1, the target compound (GT-05193) was prepared (white solid, 28 mg, yield 38.40%). ¹H NMR (400 MHz, DMSO) δ 10.85 (s, 2H), 9.89 (s, 1H), 9.04 (d, J = 7.4 Hz, 1H), 8.23 (dd, J = 8.8, 5.9 Hz, 2H), 7.41 (s, 1H), 7.28 (dd, J = 5.7, 1.4 Hz, 1H), 7.17 (dt, J = 33.7, 14.7 Hz, 2H), 6.99 (s, 1H), 6.79 (d, J = 8.2 Hz, 2H), 4.57 (s, 1H), 4.43 - 4.19 (m, 5H), 3.48 (d, J = 11.6 Hz, 2H), 3.16 - 3.03 (m, 2H), 2.82 (s, 1H), 2.65 - 2.57 (m, 1H), 2.44 - 2.32 (m, 2H), 2.26 (d, J = 11.6 Hz, 2H), 2.17 (s, 1H), 1.91 (qd, J = 12.4, 4.5 Hz, 1H). LCMS (ESI) calcd for C₃₂H₃₃F₅N₉O₄⁺ [M+H]⁺: 702.26; found, 702.3.

### Example 581: Preparation of 6-(5-cyano-1H-pyrrolo[2,3-b]pyridin-1-yl)-N-(1-(4-(2,4-dioxotetrahydropyrimidin-1(2H)-yl)benzyl)piperidin-4-yl)-4-(isopropylamino)nicotinamide (GT-05327)

According to the method of step 2 of Scheme I-1, the target compound (GT-05327) was prepared (white solid, 21 mg, yield 34.97%). ¹H NMR (400 MHz, DMSO) δ 10.92 (s, 1H), 10.44 (s, 1H), 8.86 - 8.73 (m, 3H), 8.71 - 8.64 (m, 2H), 8.52 (t, J = 4.0 Hz, 1H), 8.03 (d, J = 10.1 Hz, 1H), 7.43 (d, J = 8.4 Hz, 2H), 6.92 (d, J = 3.9 Hz, 1H), 4.33 (dd, J = 40.7, 17.8 Hz, 2H), 4.09 (d, J = 63.1 Hz, 1H), 3.83 (t, J = 6.6 Hz, 3H), 3.37 (d, J = 11.0 Hz, 2H), 3.08 (s, 2H), 2.73 (t, J = 6.6 Hz, 2H), 2.06 (d, J = 28.8 Hz, 4H), 1.29 (d, J = 6.3 Hz, 6H). LCMS (ESI) C₃₃H₃₆N₉O₃⁺ [M+H]⁺: 606.29; found, 606.3.

### Example 582: Preparation of 6-(5-cyano-1H-pyrrolo[2,3-b]pyridin-1-yl)-N-(1-(3-(2,4-dioxotetrahydropyrimidin-1(2H)-yl)benzyl)piperidin-4-yl)-4-(isopropylamino)nicotinamide (GT-05328)

According to the method of step 2 of Scheme I-1, the target compound (GT-05328) was prepared (white solid, 25 mg, yield 41.63%). ¹H NMR (400 MHz, DMSO) δ 10.82 (s, 1H), 10.45 (d, J = 3.3 Hz, 1H), 8.82 (dd, J = 5.4, 1.9 Hz, 1H), 8.78 - 8.68 (m, 2H), 8.66 (s, 1H), 8.52 (dd, J = 9.3, 3.7 Hz, 1H), 8.05 (d, J = 11.5 Hz, 1H), 7.62 (s, 1H), 7.48 (s, 3H), 6.93 (dd, J = 9.0, 3.9 Hz, 1H), 4.33 (dd, J = 25.9, 4.9 Hz, 2H), 3.99 (d, J = 5.3 Hz, 1H), 3.86 (dd, J = 17.5, 10.9 Hz, 3H), 3.40 (d, J = 11.6 Hz, 2H), 3.06 (d, J = 11.7 Hz, 2H), 2.74 (t, J = 6.5 Hz, 2H), 2.03 (t, J = 21.4 Hz, 4H), 1.28 (t, J = 7.7 Hz, 6H). LCMS (ESI) calcd for C₃₃H₃₆N₉O₃⁺ [M+H]⁺: 606.29; found, 606.3.

### Example 583: Preparation of 6-(5-cyano-1H-pyrrolo[2,3-b]pyridin-1-yl)-N-(1-((5-(2,4-dioxotetrahydropyrimidin-1(2H)-yl)pyridin-2-yl)methyl)piperidin-4-yl)-4-(isopropylamino)nicotinamide (GT-05329)

According to the method of step 2 of Scheme I-1, the target compound (GT-05329) was prepared (white solid, 18 mg, yield 29.93%). ¹H NMR (400 MHz, DMSO) δ 10.58 (s, 2H), 8.82 (d, J = 1.9 Hz, 1H), 8.70 (dd, J = 4.4, 2.2 Hz, 4H), 8.52 (d, J = 3.9 Hz, 1H), 8.03 (s, 1H), 7.91 (dd, J = 8.4, 2.5 Hz, 1H), 7.76 (d, J = 8.4 Hz, 1H), 6.93 (d, J = 3.9 Hz, 1H), 4.46 (s, 2H), 4.07 (s, 1H), 3.89 (t, J = 6.6 Hz, 2H), 3.82 (s, 1H), 3.48 (d, J = 12.2 Hz, 2H), 3.22 (s, 2H), 2.76 (s, 2H), 2.05 (s, 4H), 1.29 (d, J = 6.3 Hz, 6H). LCMS (ESI) calcd for C₃₂H₃₅N₁₀O₃⁺ [M+H]⁺: 607.29; found, 607.3.

### Example 584: Preparation of 6-(5-cyano-1H-pyrrolo[2,3-b]pyridin-1-yl)-N-(1-((6-(2,4-dioxotetrahydropyrimidin-1(2H)-yl)pyridin-3-yl)methyl)piperidin-4-yl)-4-(isopropylamino)nicotinamide (GT-05330)

According to the method of step 2 of Scheme I-1, the target compound (GT-05330) was prepared (white solid, 28 mg, yield 46.56%). ¹H NMR (400 MHz, DMSO) δ 11.22 (s, 1H), 10.61 (s, 1H), 8.87 - 8.81 (m, 2H), 8.70 - 8.68 (m, 1H), 8.62 (d, J = 2.0 Hz, 1H), 8.50 (t, J = 9.1 Hz, 1H), 8.12 (dd, J = 8.6, 2.2 Hz, 1H), 8.01 (d, J = 10.5 Hz, 1H), 7.86 (d, J = 8.6 Hz, 1H), 6.94 (dd, J = 6.8, 3.9 Hz, 1H), 4.32 (d, J = 4.5 Hz, 2H), 4.11 (t, J = 6.5 Hz, 2H), 4.01 (d, J = 7.1 Hz, 1H), 3.84 (s, 1H), 3.39 (d, J = 11.7 Hz, 2H), 3.08 (s, 2H), 2.71 (t, J = 6.5 Hz, 2H), 2.03 (s, 4H), 1.29 (d, J = 6.3 Hz, 6H). LCMS (ESI) calcd for C₃₂H₃₅N₁₀O₃⁺ [M+H]⁺: 607.29; found, 607.3.

### Example 585: Preparation of 6-(5-cyano-1H-pyrrolo[2,3-b]pyridin-1-yl)-N-(1-(3-((2,6-dioxopiperidin-3-yl)amino)benzyl)piperidin-4-yl)-4-(isopropylamino)nicotinamide (GT-05331)

According to the method of step 2 of Scheme I-1, the target compound (GT-05331) was prepared (white solid, 27 mg, yield 43.95%). ¹H NMR (400 MHz, DMSO) δ 10.85 (s, 1H), 10.70 (s, 1H), 8.82 (dd, J = 4.8, 2.0 Hz, 1H), 8.70 (dd, J = 4.1, 2.0 Hz, 1H), 8.66 (s, 1H), 8.51 (d, J = 4.0 Hz, 1H), 8.04 (d, J = 10.1 Hz, 1H), 7.16 (t, J = 7.8 Hz, 1H), 7.02 (d, J = 10.1 Hz, 1H), 6.93 (dd, J = 7.8, 3.9 Hz, 1H), 6.79 (d, J = 8.0 Hz, 2H), 4.39 (dd, J = 11.6, 4.6 Hz, 1H), 4.16 (t, J = 13.9 Hz, 2H), 4.00 - 3.84 (m, 2H), 3.32 (t, J = 32.4 Hz, 3H), 3.03 (s, 2H), 2.82 (dd, J = 11.1, 6.1 Hz, 1H), 2.59 (dd, J = 23.5, 9.9 Hz, 1H), 2.26 - 2.15 (m, 1H), 2.08 - 1.91 (m, 4H), 1.29 (d, J = 6.3 Hz, 6H). LCMS (ESI) calcd for C₃₄H₃₈N₉O₃⁺ [M+H]⁺: 620.31; found, 620.3.

### Example 586: Preparation of N-(2-(1-(4-(2,6-dioxopiperidin-3-yl)benzyl)piperidin-4-yl)-6-methoxy-2H-indazol-5-yl)-3-(trifluoromethyl)benzamide (GT-04747)

According to the method of step 2 of Scheme I-1, the target compound (GT-04747) was prepared (white solid, 21 mg, yield 28.36%). ¹H NMR (400 MHz, DMSO) δ 11.13 (s, 1H), 10.88 (d, J = 4.2 Hz, 1H), 9.81 (d, J = 15.5 Hz, 1H), 8.29 (dd, J = 13.1, 7.0 Hz, 2H), 8.05 (d, J = 5.1 Hz, 1H), 7.97 (d, J = 8.0 Hz, 1H), 7.79 (dd, J = 9.9, 5.6 Hz, 1H), 7.64 (d, J = 8.0 Hz, 2H), 7.34 (t, J = 9.5 Hz, 2H), 7.11 (d, J = 6.5 Hz, 1H), 4.74 (t, J = 11.7 Hz, 1H), 4.36 (dd, J = 15.9, 4.7 Hz, 2H), 3.95 (dd, J = 11.6, 5.0 Hz, 1H), 3.88 (d, J = 8.1 Hz, 3H), 3.54 (dd, J = 22.2, 8.9 Hz, 4H), 3.40 - 3.04 (m, 3H), 2.74 - 2.66 (m, 1H), 2.55 (s, 1H), 2.40 - 2.17 (m, 3H), 2.11 - 2.01 (m, 1H). LCMS (ESI) calcd for C₃₃H₃₃F₃N₅O₄⁺ [M+H]⁺: 620.25; found, 620.3.

### Example 587: Preparation of N-(2-(1-(4-(2,6-dioxopiperidin-3-yl)-3-fluorobenzyl)piperidin-4-yl)-6-methoxy-2H-indazol-5-yl)-3-(trifluoromethyl)benzamide (GT-04748)

According to the method of step 2 of Scheme I-1, the target compound (GT-04748) was prepared (white solid, 35 mg, yield 43.33%). ¹H NMR (400 MHz, DMSO) δ 11.07 (s, 1H), 10.94 (s, 1H), 9.81 (d, J = 15.0 Hz, 1H), 8.34 - 8.22 (m, 3H), 8.04 (s, 1H), 7.97 (d, J = 8.8 Hz, 1H), 7.78 (t, J = 8.0 Hz, 1H), 7.59 (s, 1H), 7.45 (s, 2H), 7.11 (s, 1H), 4.81 (d, J = 52.7 Hz, 1H), 4.37 (s, 2H), 4.13 (d, J = 11.9 Hz, 1H), 3.87 (s, 3H), 3.36 (s, 4H), 3.19 (d, J = 10.7 Hz, 2H), 2.79 (d, J = 13.1 Hz, 1H), 2.57 (d, J = 3.4 Hz, 2H), 2.41 - 2.24 (m, 2H), 2.03 (s, 1H). LCMS (ESI) calcd for C₃₃H₃₂F₄N₅O₄⁺ [M+H]⁺: 638.24; found, 638.3.

### Example 588: Preparation of N-(2-(1-(4-(2,4-dioxotetrahydropyrimidin-1(2H)-yl)benzyl)piperidin-4-yl)-6-methoxy-2H-indazol-5-yl)-3-(trifluoromethyl)benzamide (GT-04752)

According to the method of step 2 of Scheme I-1, the target compound (GT-04752) was prepared (white solid, 46 mg, yield 55.38%). ¹H NMR (400 MHz, DMSO) δ 10.76 (s, 1H), 10.46 (d, J = 3.8 Hz, 1H), 9.81 (d, J = 15.1 Hz, 1H), 8.38 - 8.21 (m, 3H), 8.09 - 7.93 (m, 2H), 7.78 (d, J = 7.5 Hz, 1H), 7.65 (s, 2H), 7.51 (d, J = 41.8 Hz, 2H), 7.11 (s, 1H), 4.81 (d, J = 54.9 Hz, 1H), 4.36 (s, 2H), 3.91 - 3.83 (m, 4H), 3.57 (d, J = 35.6 Hz, 2H), 3.41 - 3.34 (m, 2H), 3.17 (d, J = 24.2 Hz, 3H), 2.72 (dd, J = 14.5, 11.2 Hz, 2H), 2.37 (s, 2H). LCMS (ESI) calcd for C₃₂H₃₂F₃N₆O₄⁺ [M+H]⁺: 621.24; found, 621.3.

### Example 589: Preparation of N-(2-(1-(4-(2,4-dioxotetrahydropyrimidin-1(2H)-yl)-3-fluorobenzyl)piperidin-4-yl)-6-methoxy-2H-indazol-5-yl)-3-(trifluoromethyl)benzamide (GT-04751)

According to the method of step 2 of Scheme I-1, the target compound (GT-04751) was prepared (white solid, 50 mg, yield 65.52%). ¹H NMR (400 MHz, DMSO) δ 10.99 (s, 1H), 10.56 (s, 1H), 9.80 (d, J = 14.7 Hz, 1H), 8.51 - 8.24 (m, 3H), 8.05 (d, J = 4.3 Hz, 1H), 7.97 (d, J = 7.7 Hz, 1H), 7.78 (t, J = 7.8 Hz, 1H), 7.70 (d, J = 11.1 Hz, 1H), 7.61 - 7.48 (m, 2H), 7.11 (s, 1H), 4.76 (t, J = 11.5 Hz, 1H), 4.47 - 4.35 (m, 2H), 3.88 (d, J = 6.8 Hz, 3H), 3.76 (t, J = 6.6 Hz, 2H), 3.67 - 3.48 (m, 3H), 3.21 (d, J = 11.8 Hz, 1H), 2.75 (t, J = 5.7 Hz, 2H), 2.56 (s, 1H), 2.35 (d, J = 14.1 Hz, 2H). LCMS (ESI) calcd for C₃₂H₃₁F₄N₆O₄⁺ [M+H]⁺: 639.23; found, 639.3.

### Example 590: Preparation of N-(2-(1-(4-(2,4-dioxotetrahydropyrimidin-1(2H)-yl)-2-fluorobenzyl)piperidin-4-yl)-6-methoxy-2H-indazol-5-yl)-3-(trifluoromethyl)benzamide (GT-04753)

According to the method of step 2 of Scheme I-1, the target compound (GT-04753) was prepared (white solid, 49 mg, yield 58.38%). ¹H NMR (400 MHz, DMSO) δ 10.86 (s, 1H), 10.55 (s, 1H), 9.80 (d, J = 13.1 Hz, 1H), 8.49 - 8.23 (m, 3H), 8.05 (d, J = 5.8 Hz, 1H), 7.97 (d, J = 7.8 Hz, 1H), 7.79 (dd, J = 12.3, 4.9 Hz, 2H), 7.38 (ddt, J = 13.9, 7.5, 3.7 Hz, 2H), 7.10 (s, 1H), 4.75 (s, 1H), 4.40 (s, 2H), 3.92 - 3.82 (m, 5H), 3.59 (dd, J = 8.5, 4.7 Hz, 2H), 3.27 (d, J = 11.6 Hz, 2H), 3.13 (dd, J = 7.4, 4.3 Hz, 1H), 2.74 (t, J = 6.6 Hz, 2H), 2.57 (d, J = 33.1 Hz, 1H), 2.45 (s, 1H), 2.35 (d, J = 14.4 Hz, 2H). LCMS (ESI) calcd for C₃₂H₃₁F₄N₆O₄⁺ [M+H]⁺: 639.23; found, 639.3.

### Example 591: Preparation of N-(2-(1-(3-(2,4-dioxotetrahydropyrimidin-1(2H)-yl)benzyl)piperidin-4-yl)-6-methoxy-2H-indazol-5-yl)-3-(trifluoromethyl)benzamide (GT-04750)

According to the method of step 2 of Scheme I-1, the target compound (GT-04750) was prepared (white solid, 44 mg, yield 59.33%). ¹H NMR (400 MHz, DMSO) δ 10.96 (s, 1H), 10.45 (d, J = 8.5 Hz, 1H), 9.80 (d, J = 15.0 Hz, 1H), 8.53 - 8.24 (m, 3H), 8.05 (d, J = 5.1 Hz, 1H), 7.97 (d, J = 7.3 Hz, 1H), 7.78 (t, J = 7.8 Hz, 1H), 7.64 (s, 1H), 7.49 (s, 3H), 7.10 (s, 1H), 4.77 (t, J = 33.5 Hz, 1H), 4.38 (d, J = 3.7 Hz, 2H), 3.95 - 3.83 (m, 5H), 3.62 - 3.51 (m, 2H), 3.25 - 3.15 (m, 2H), 2.74 (t, J = 6.6 Hz, 2H), 2.62 (s, 1H), 2.49 - 2.41 (m, 1H), 2.34 (d, J = 11.2 Hz, 2H). LCMS (ESI) calcd for C₃₂H₃₂F₃N₆O₄⁺ [M+H]⁺: 621.24; found, 621.24.

### Example 592: Preparation of N-(2-(1-((5-(2,4-dioxotetrahydropyrimidin-1(2H)-yl)pyridin-2-yl)methyl)piperidin-4-yl)-6-methoxy-2H-indazol-5-yl)-3-(trifluoromethyl)benzamide (GT-04878)

According to the method of step 2 of Scheme I-1, the target compound (GT-04878) was prepared (white solid, 30 mg, yield 38.10%). ¹H NMR (400 MHz, DMSO) δ 10.87 (s, 1H), 10.59 (s, 1H), 9.79 (s, 1H), 8.72 (d, J = 2.5 Hz, 1H), 8.34 (d, J = 8.8 Hz, 1H), 8.31 - 8.23 (m, 2H), 8.05 (s, 1H), 8.00 - 7.90 (m, 2H), 7.78 (dd, J = 14.6, 8.0 Hz, 2H), 7.11 (s, 1H), 4.78 (s, 1H), 4.57 (d, J = 30.7 Hz, 2H), 3.95 - 3.84 (m, 5H), 3.67 - 3.57 (m, 2H), 3.35 (t, J = 11.4 Hz, 3H), 2.76 (t, J = 6.6 Hz, 2H), 2.54 (s, 1H), 2.44 - 2.33 (m, 2H). LCMS (ESI) calcd for C₃₁H₃₁F₃N₇O₄⁺ [M+H]⁺: 622.24; found, 622.3.

### Example 593: Preparation of N-(2-(1-((6-(2,4-dioxotetrahydropyrimidin-1(2H)-yl)pyridin-3-yl)methyl)piperidin-4-yl)-6-methoxy-2H-indazol-5-yl)-3-(trifluoromethyl)benzamide (GT-04879)

According to the method of step 2 of Scheme I-1, the target compound (GT-04879) was prepared (white solid, 32 mg, yield 53.84%). ¹H NMR (400 MHz, DMSO) δ 11.33 (s, 1H), 10.62 (s, 1H), 9.80 (d, J = 16.1 Hz, 1H), 8.65 (d, J = 1.6 Hz, 1H), 8.55 - 8.25 (m, 3H), 8.13 (dd, J = 8.7, 2.0 Hz, 1H), 8.05 (d, J = 6.2 Hz, 1H), 7.97 (d, J = 7.7 Hz, 1H), 7.89 (d, J = 8.6 Hz, 1H), 7.78 (t, J = 7.8 Hz, 1H), 7.11 (d, J = 8.5 Hz, 1H), 4.75 (t, J = 11.5 Hz, 1H), 4.40 (s, 2H), 4.11 (q, J = 6.5 Hz, 2H), 3.88 (d, J = 7.8 Hz, 3H), 3.59 (ddd, J = 25.3, 13.9, 7.8 Hz, 2H), 3.28 - 3.07 (m, 3H), 2.71 (t, J = 6.5 Hz, 2H), 2.63 - 2.54 (m, 1H), 2.35 (d, J = 11.7 Hz, 2H). LCMS (ESI) calcd for C₃₁H₃₁F₃N₇O₄⁺ [M+H]⁺: 622.24; found, 622.3.

### Example 594: Preparation of N-(2-(1-(3-((2,6-dioxopiperidin-3-yl)amino)benzyl)piperidin-4-yl)-6-methoxy-2H-indazol-5-yl)-3-(trifluoromethyl)benzamide (GT-04749)

According to the method of step 2 of Scheme I-1, the target compound (GT-04749) was prepared (white solid, 48 mg, yield 52.74%). ¹H NMR (400 MHz, DMSO) δ 10.82 (d, J = 23.2 Hz, 2H), 9.80 (d, J = 14.7 Hz, 1H), 8.29 (dd, J = 13.7, 7.2 Hz, 3H), 8.04 (d, J = 4.3 Hz, 1H), 7.97 (d, J = 7.5 Hz, 1H), 7.78 (t, J = 7.7 Hz, 1H), 7.18 (t, J = 7.9 Hz, 1H), 7.11 (s, 1H), 6.99 (s, 1H), 6.80 (d, J = 7.8 Hz, 2H), 4.76 (dd, J = 40.2, 28.8 Hz, 1H), 4.40 (dd, J = 11.6, 4.6 Hz, 1H), 4.23 (t, J = 8.9 Hz, 2H), 3.88 (d, J = 7.5 Hz, 3H), 3.46 (t, J = 37.8 Hz, 3H), 3.20 - 3.05 (m, 3H), 2.89 - 2.75 (m, 1H), 2.61 (d, J = 17.8 Hz, 2H), 2.34 (d, J = 11.2 Hz, 2H), 2.18 (dd, J = 8.2, 4.1 Hz, 1H), 2.00 - 1.84 (m, 1H). LCMS (ESI) calcd for C₃₃H₃₄F₃N₆O₄⁺ [M+H]+: 635.26; found, 635.3.

### Example 595: Preparation of N-(2-(1-(4-(2,6-dioxopiperidin-3-yl)benzyl)piperidin-4-yl)-6-methoxy-2H-indazol-5-yl)-6-(trifluoromethyl)picolinamide (GT-04761)

According to the method of step 2 of Scheme I-1, the target compound (GT-04761) was prepared (white solid, 30 mg, yield 40.55%). ¹H NMR (400 MHz, DMSO) δ 10.89 (s, 2H), 10.51 (s, 1H), 8.71 (s, 1H), 8.55 - 8.38 (m, 2H), 8.35 (s, 1H), 8.22 (d, J = 7.7 Hz, 1H), 7.61 (d, J = 8.1 Hz, 2H), 7.36 (d, J = 8.1 Hz, 2H), 7.19 (s, 1H), 4.74 (s, 1H), 4.36 (dd, J = 13.6, 5.0 Hz, 2H), 4.04 - 3.90 (m, 4H), 3.51 (s, 2H), 3.19 (dd, J = 23.8, 12.0 Hz, 3H), 2.68 (dd, J = 11.9, 5.1 Hz, 1H), 2.54 (d, J = 4.5 Hz, 1H), 2.45 (s, 1H), 2.34 (d, J = 11.9 Hz, 2H), 2.24 (dd, J = 12.4, 3.8 Hz, 1H), 2.11 - 2.02 (m, 1H). LCMS (ESI) calcd for C₃₂H₃₂F₃N₆O₄⁺ [M+H]⁺: 621.24; found, 621.3.

### Example 596: Preparation of N-(2-(1-(4-(2,6-dioxopiperidin-3-yl)-3-fluorobenzyl)piperidin-4-yl)-6-methoxy-2H-indazol-5-yl)-6-(trifluoromethyl)picolinamide (GT-04762)

According to the method of step 2 of Scheme I-1, the target compound (GT-04762) was prepared (white solid, 38 mg, yield 55.46%). ¹H NMR (400 MHz, DMSO) δ 11.22 (s, 1H), 10.93 (d, J = 4.4 Hz, 1H), 10.52 (d, J = 8.2 Hz, 1H), 8.72 (d, J = 9.5 Hz, 1H), 8.55 - 8.40 (m, 2H), 8.35 (s, 1H), 8.23 (t, J = 5.9 Hz, 1H), 7.62 (d, J = 10.8 Hz, 1H), 7.51 - 7.39 (m, 2H), 7.19 (d, J = 9.9 Hz, 1H), 4.78 (dd, J = 38.1, 26.3 Hz, 1H), 4.38 (t, J = 8.4 Hz, 2H), 4.14 (dd, J = 12.6, 5.1 Hz, 1H), 3.99 (d, J = 8.1 Hz, 3H), 3.80 (s, 2H), 3.65 - 3.47 (m, 2H), 3.27 (d, J = 54.1 Hz, 2H), 2.83 - 2.74 (m, 1H), 2.57 (dd, J = 8.4, 5.3 Hz, 2H), 2.27 (ddd, J = 21.9, 20.1, 10.6 Hz, 3H), 2.08 - 1.98 (m, 1H). LCMS (ESI) calcd for C₃₂H₃₁F₄N₆O₄⁺ [M+H]⁺: 639.23; found, 639.3.

### Example 597: Preparation of N-(2-(1-(4-(2,4-dioxotetrahydropyrimidin-1(2H)-yl)benzyl)piperidin-4-yl)-6-methoxy-2H-indazol-5-yl)-6-(trifluoromethyl)picolinamide (GT-04776)

According to the method of step 2 of Scheme I-1, the target compound (GT-04776) was prepared (white solid, 55 mg, yield 74.21%). ¹H NMR (400 MHz, DMSO) δ 11.03 (s, 1H), 10.55 - 10.39 (m, 2H), 8.72 (d, J = 10.3 Hz, 1H), 8.56 - 8.38 (m, 2H), 8.23 (t, J = 6.1 Hz, 1H), 7.68 (d, J = 8.4 Hz, 1H), 7.46 (d, J = 8.5 Hz, 1H), 7.19 (d, J = 5.8 Hz, 1H), 4.74 (t, J = 11.7 Hz, 1H), 4.45 - 4.32 (m, 2H), 4.00 (d, J = 8.8 Hz, 3H), 3.84 (q, J = 6.2 Hz, 2H), 3.52 (d, J = 11.7 Hz, 4H), 3.40 - 3.16 (m, 2H), 2.74 (d, J = 5.9 Hz, 2H), 2.55 (d, J = 8.9 Hz, 1H), 2.35 (d, J = 11.0 Hz, 1H). LCMS (ESI) calcd for C₃₁H₃₁F₃N₇O₄⁺ [M+H]⁺: 622.24; found, 622.3.

### Example 598: Preparation of N-(2-(1-(4-(2,4-dioxotetrahydropyrimidin-1(2H)-yl)-3-fluorobenzyl)piperidin-4-yl)-6-methoxy-2H-indazol-5-yl)-6-(trifluoromethyl)picolinamide (GT-04775)

According to the method of step 2 of Scheme I-1, the target compound (GT-04775) was prepared (white solid, 34 mg, yield 49.54%). ¹H NMR (400 MHz, DMSO) δ 11.02 (s, 1H), 10.52 - 10.42 (m, 2H), 8.65 (d, J = 9.7 Hz, 1H), 8.48 - 8.33 (m, 2H), 8.30 (d, J = 13.2 Hz, 1H), 8.16 (t, J = 5.4 Hz, 1H), 7.64 (d, J = 11.2 Hz, 1H), 7.56 - 7.42 (m, 2H), 7.12 (d, J = 7.1 Hz, 1H), 4.68 (t, J = 11.7 Hz, 1H), 4.33 (d, J = 4.0 Hz, 2H), 3.93 (d, J = 8.2 Hz, 3H), 3.69 (t, J = 6.6 Hz, 2H), 3.54 (s, 2H), 3.20 - 3.04 (m, 2H), 2.67 (dd, J = 9.0, 4.3 Hz, 2H), 2.55 (s, 2H), 2.28 (d, J = 13.4 Hz, 2H). LCMS (ESI) calcd for C₃₁H₃₀F₄N₇O₄⁺ [M+H]+: 640.23; found, 640.3.

### Example 599: Preparation of N-(2-(1-(4-(2,4-dioxotetrahydropyrimidin-1(2H)-yl)-2-fluorobenzyl)piperidin-4-yl)-6-methoxy-2H-indazol-5-yl)-6-(trifluoromethyl)picolinamide (GT-04777)

According to the method of step 2 of Scheme I-1, the target compound (GT-04777) was prepared (white solid, 48 mg, yield 62.95%). ¹H NMR (400 MHz, DMSO) δ 11.10 (s, 1H), 10.62 - 10.39 (m, 2H), 8.72 (d, J = 9.0 Hz, 1H), 8.55 - 8.33 (m, 3H), 8.22 (d, J = 7.6 Hz, 1H), 7.82 (t, J = 8.4 Hz, 1H), 7.46 - 7.31 (m, 2H), 7.18 (d, J = 7.8 Hz, 1H), 4.75 (t, J = 11.6 Hz, 1H), 4.42 (d, J = 12.3 Hz, 2H), 4.00 (d, J = 7.7 Hz, 3H), 3.87 (t, J = 6.4 Hz, 2H), 3.56 (s, 3H), 3.45 - 3.20 (m, 2H), 2.74 (t, J = 6.5 Hz, 2H), 2.57 (s, 1H), 2.35 (d, J = 12.2 Hz, 2H). LCMS (ESI) calcd for C₃₁H₃₀F₄N₇O₄⁺ [M+H]⁺: 640.23; found, 640.3.

### Example 600: Preparation of N-(2-(1-(3-(2,4-dioxotetrahydropyrimidin-1(2H)-yl)benzyl)piperidin-4-yl)-6-methoxy-2H-indazol-5-yl)-6-(trifluoromethyl)picolinamide (GT-04774)

According to the method of step 2 of Scheme I-1, the target compound (GT-04774) was prepared (white solid, 30 mg, yield 40.48%). ¹H NMR (400 MHz, DMSO) δ 10.94 (s, 1H), 10.49 (d, J = 16.5 Hz, 2H), 8.72 (d, J = 11.0 Hz, 1H), 8.44 (dd, J = 17.6, 7.6 Hz, 2H), 8.37 (d, J = 14.0 Hz, 1H), 8.22 (d, J = 7.6 Hz, 1H), 7.64 (s, 1H), 7.48 (d, J = 8.5 Hz, 2H), 7.19 (s, 1H), 4.73 (s, 1H), 4.39 (t, J = 7.9 Hz, 2H), 4.00 (d, J = 9.3 Hz, 3H), 3.93 - 3.82 (m, 2H), 3.62 - 3.51 (m, 2H), 3.42 - 3.15 (m, 3H), 2.78 - 2.73 (m, 2H), 2.64 - 2.53 (m, 1H), 2.38 (t, J = 28.4 Hz, 2H). LCMS (ESI) calcd for C₃₁H₃₁F₃N₇O₄⁺ [M+H]⁺: 622.24; found, 622.3.

### Example 601: Preparation of N-(2-(1-((5-(2,4-dioxotetrahydropyrimidin-1(2H)-yl)pyridin-2-yl)methyl)piperidin-4-yl)-6-methoxy-2H-indazol-5-yl)-6-(trifluoromethyl)picolinamide (GT-04881)

According to the method of step 2 of Scheme I-1, the target compound (GT-04881) was prepared (white solid, 35 mg, yield 58.94%). ¹H NMR (400 MHz, DMSO) δ 10.85 (s, 1H), 10.59 (s, 1H), 10.52 (s, 1H), 8.72 (d, J = 4.4 Hz, 2H), 8.50 - 8.35 (m, 3H), 8.22 (d, J = 7.6 Hz, 1H), 7.92 (dd, J = 8.4, 2.5 Hz, 1H), 7.76 (dd, J = 8.4, 2.2 Hz, 1H), 7.19 (s, 1H), 4.78 (s, 1H), 4.53 (s, 2H), 3.99 (s, 3H), 3.90 (t, J = 6.6 Hz, 2H), 3.68 - 3.56 (m, 2H), 3.40 - 3.29 (m, 3H), 2.76 (t, J = 6.6 Hz, 2H), 2.54 (s, 1H), 2.38 (d, J = 11.1 Hz, 2H). LCMS (ESI) calcd for C₃₀H₃₀F₃N₈O₄⁺ [M+H]⁺: 623.23; found, 623.3.

### Example 602: Preparation of N-(2-(1-((6-(2,4-dioxotetrahydropyrimidin-1(2H)-yl)pyridin-3-yl)methyl)piperidin-4-yl)-6-methoxy-2H-indazol-5-yl)-6-(trifluoromethyl)picolinamide (GT-04882)

According to the method of step 2 of Scheme I-1, the target compound (GT-04882) was prepared (white solid, 40 mg, yield 67.36%). ¹H NMR (400 MHz, DMSO) δ 10.91 (s, 1H), 10.67 - 10.48 (m, 2H), 8.72 (d, J = 11.2 Hz, 1H), 8.62 (d, J = 1.8 Hz, 1H), 8.56 - 8.40 (m, 2H), 8.37 (d, J = 14.4 Hz, 1H), 8.22 (d, J = 7.7 Hz, 1H), 8.14 - 8.02 (m, 1H), 7.88 (dd, J = 12.8, 8.7 Hz, 1H), 7.18 (d, J = 5.2 Hz, 1H), 4.76 (d, J = 11.5 Hz, 1H), 4.40 (s, 2H), 4.10 (q, J = 6.7 Hz, 2H), 3.99 (d, J = 8.3 Hz, 3H), 3.61 - 3.52 (m, 2H), 3.15 (ddd, J = 14.1, 11.7, 7.5 Hz, 3H), 2.71 (t, J = 6.5 Hz, 2H), 2.40 (dd, J = 41.1, 11.6 Hz, 3H). LCMS (ESI) calcd for C₃₀H₃₀F₃N₈O₄⁺ [M+H]⁺: 623.23; found, 623.3.

### Example 603: Preparation of N-(2-(1-(3-((2,6-dioxopiperidin-3-yl)amino)benzyl)piperidin-4-yl)-6-methoxy-2H-indazol-5-yl)-6-(trifluoromethyl)picolinamide (GT-04763)

According to the method of step 2 of Scheme I-1, the target compound (GT-04763) was prepared (white solid, 45 mg, yield 65.98%). ¹H NMR (400 MHz, DMSO) δ 11.02 (s, 1H), 10.86 (d, J = 4.7 Hz, 1H), 10.52 (d, J = 7.6 Hz, 1H), 8.72 (d, J = 10.2 Hz, 1H), 8.57 - 8.39 (m, 2H), 8.37 (d, J = 15.4 Hz, 1H), 8.26 - 8.16 (m, 1H), 7.17 (dd, J = 12.7, 4.9 Hz, 2H), 7.04 (s, 1H), 6.82 (t, J = 6.6 Hz, 2H), 4.76 (dd, J = 40.2, 28.5 Hz, 1H), 4.41 (dd, J = 11.6, 4.7 Hz, 2H), 4.00 (d, J = 8.9 Hz, 3H), 3.65 - 3.46 (m, 2H), 3.26 - 3.07 (m, 3H), 2.84 (ddd, J = 17.6, 12.5, 5.4 Hz, 1H), 2.66 - 2.53 (m, 3H), 2.34 (d, J = 11.5 Hz, 1H), 2.25 - 2.02 (m, 1H), 2.00 - 1.79 (m, 1H). LCMS (ESI) calcd for C₃₂H₃₃F₃N₇O₄⁺ [M+H]⁺: 636.25; found, 636.3.

### Example 604: Preparation of N-(2-(1-(4-(2,6-dioxopiperidin-3-yl)benzyl)piperidin-4-yl)-6-(2-hydroxypropan-2-yl)-2H-indazol-5-yl)-6-(trifluoromethyl)picolinamide (GT-05753)

According to the method of step 2 of Scheme I-1, the target compound (GT-05753) was prepared (white solid, 17 mg, yield 29.32%). ¹H NMR (400 MHz, DMSO-d6) δ 12.44 (s, 1H), 10.93 (d, J = 4.5 Hz, 2H), 8.81 (d, J = 9.8 Hz, 1H), 8.51 (d, J = 7.8 Hz, 1H), 8.46 - 8.39 (m, 2H), 8.22 (d, J = 7.8 Hz, 1H), 7.72 - 7.60 (m, 3H), 7.45 - 7.35 (m, 2H), 4.89 (dd, J = 38.0, 26.4 Hz, 1H), 4.41 (s, 2H), 4.00 (dd, J = 11.7, 4.8 Hz, 1H), 3.59 (d, J = 12.1 Hz, 4H), 3.43 - 3.34 (m, 2H), 3.26 (d, J = 10.9 Hz, 2H), 2.76 (ddd, J = 16.8, 12.0, 5.2 Hz, 1H), 2.60 (s, 1H), 2.41 (d, J = 13.2 Hz, 2H), 2.34 - 2.21 (m, 1H), 2.16 - 2.08 (m, 1H), 1.68 (d, J = 7.8 Hz, 6H). LCMS (ESI) calcd for C₃₄H₃₆F₃N₆O₄⁺ [M+H]⁺: 649.28; found, 649.3.

### Example 605: Preparation of N-(2-(1-(4-(2,4-dioxotetrahydropyrimidin-1(2H)-yl)benzyl)piperidin-4-yl)-6-(2-hydroxypropan-2-yl)-2H-indazol-5-yl)-6-(trifluoromethyl)picolinamide (GT-05754)

According to the method of step 2 of Scheme I-1, the target compound (GT-05754) was prepared (white solid, 23 mg, yield 39.60%). ¹H NMR (400 MHz, DMSO-d6) δ 12.39 (d, J = 5.8 Hz, 1H), 10.79 (s, 1H), 10.45 (d, J = 4.6 Hz, 1H), 8.75 (d, J = 10.2 Hz, 1H), 8.45 (d, J = 7.7 Hz, 1H), 8.42 - 8.29 (m, 2H), 8.23 - 8.10 (m, 1H), 7.66 (d, J = 8.5 Hz, 2H), 7.46 (dd, J = 8.6, 2.4 Hz, 2H), 7.33 (dd, J = 8.0, 6.0 Hz, 1H), 4.81 (d, J = 7.4 Hz, 1H), 4.37 (s, 2H), 3.90 - 3.80 (m, 2H), 3.54 (d, J = 11.2 Hz, 2H), 3.20 (d, J = 10.8 Hz, 2H), 2.73 (dd, J = 11.1, 4.4 Hz, 2H), 2.55 (s, 1H), 2.49 - 2.44 (m, 1H), 2.36 (d, J = 14.3 Hz, 2H), 1.63 (d, J = 7.8 Hz, 6H). LCMS (ESI) calcd for C₃₃H₃₅F₃N₇O₄⁺ [M+H]⁺: 650.27; found, 650.3.

### Example 606: Preparation of N-(2-(1-(3-(2,4-dioxotetrahydropyrimidin-1(2H)-yl)benzyl)piperidin-4-yl)-6-(2-hydroxypropan-2-yl)-2H-indazol-5-yl)-6-(trifluoromethyl)picolinamide (GT-05755)

According to the method of step 2 of Scheme I-1, the target compound (GT-05755) was prepared (white solid, 20 mg, yield 34.44%). ¹H NMR (400 MHz, DMSO-d6) δ 12.39 (d, J = 6.0 Hz, 1H), 10.87 (s, 1H), 10.45 (d, J = 9.6 Hz, 1H), 8.75 (d, J = 10.3 Hz, 1H), 8.45 (d, J = 7.8 Hz, 1H), 8.38 (s, 1H), 8.20 - 8.09 (m, 1H), 7.66 - 7.56 (m, 2H), 7.50 (s, 2H), 7.44 - 7.39 (m, 1H), 7.32 (t, J = 8.3 Hz, 1H), 5.03 - 4.77 (m, 1H), 4.38 (s, 2H), 3.88 (t, J = 6.6 Hz, 2H), 3.79 (d, J = 6.7 Hz, 1H), 3.56 (s, 2H), 3.21 (d, J = 8.7 Hz, 1H), 2.72 (dd, J = 12.3, 6.0 Hz, 3H), 2.55 (s, 1H), 2.37 (s, 2H), 1.63 (d, J = 8.2 Hz, 6H). LCMS (ESI) calcd for C₃₃H₃₅F₃N₇O₄⁺ [M+H]⁺: 650.27; found, 650.3.

### Example 607: Preparation of N-(2-(1-((5-(2,4-dioxotetrahydropyrimidin-1(2H)-yl)pyridin-2-yl)methyl)piperidin-4-yl)-6-(2-hydroxypropan-2-yl)-2H-indazol-5-yl)-6-(trifluoromethyl)picolinamide (GT-05756)

According to the method of step 2 of Scheme I-1, the target compound (GT-05756) was prepared (white solid, 18 mg, yield 30.95%). ¹H NMR (400 MHz, DMSO) δ 12.38 (s, 1H), 10.59 (s, 1H), 8.76 - 8.66 (m, 2H), 8.49 - 8.33 (m, 3H), 8.16 (dd, J = 7.7, 0.8 Hz, 1H), 7.92 (dd, J = 8.4, 2.6 Hz, 1H), 7.79 (t, J = 8.8 Hz, 1H), 7.57 (d, J = 23.1 Hz, 1H), 4.83 (s, 1H), 4.52 (s, 2H), 3.89 (t, J = 6.6 Hz, 2H), 3.56 - 3.49 (m, 4H), 3.35 (t, J = 11.3 Hz, 2H), 2.76 (t, J = 6.6 Hz, 2H), 2.56 (s, 1H), 2.35 (dd, J = 19.6, 6.4 Hz, 2H), 1.62 (s, 6H). LCMS (ESI) calcd for C₃₂H₃₄F₃N₈O₄⁺ [M+H]⁺: 651.27; found, 651.3.

### Example 608: Preparation of N-(2-(1-((6-(2,4-dioxotetrahydropyrimidin-1(2H)-yl)pyridin-3-yl)methyl)piperidin-4-yl)-6-(2-hydroxypropan-2-yl)-2H-indazol-5-yl)-6-(trifluoromethyl)picolinamide (GT-05757)

According to the method of step 2 of Scheme I-1, the target compound (GT-05757) was prepared (white solid, 23 mg, yield 39.55%). ¹H NMR (400 MHz, DMSO) δ 12.44 (s, 1H), 11.32 (s, 1H), 10.69 (s, 1H), 8.81 (d, J = 10.3 Hz, 1H), 8.70 (d, J = 1.9 Hz, 1H), 8.62 - 8.40 (m, 3H), 8.25 - 8.17 (m, 2H), 7.94 (d, J = 8.6 Hz, 1H), 7.66 (s, 1H), 4.86 (t, J = 11.5 Hz, 1H), 4.47 (s, 2H), 4.17 (t, J = 6.6 Hz, 2H), 3.58 - 3.53 (m, 3H), 3.26 (d, J = 11.0 Hz, 2H), 2.77 (t, J = 6.5 Hz, 2H), 2.62 (d, J = 12.5 Hz, 1H), 2.41 (d, J = 12.5 Hz, 2H), 1.68 (d, J = 7.2 Hz, 6H). LCMS (ESI) calcd for C₃₂H₃₄F₃N₈O₄⁺ [M+H]⁺: 651.27; found, 651.3.

### Example 609: Preparation of N-(2-(1-(3-((2,6-dioxopiperidin-3-yl)amino)benzyl)piperidin-4-yl)-6-(2-hydroxypropan-2-yl)-2H-indazol-5-yl)-6-(trifluoromethyl)picolinamide (GT-05758)

According to the method of step 2 of Scheme I-1, the target compound (GT-05758) was prepared (white solid, 19 mg, yield 32.02%). ¹H NMR (400 MHz, DMSO) δ 12.38 (d, J = 4.6 Hz, 1H), 10.84 (d, J = 5.4 Hz, 2H), 8.74 (d, J = 8.4 Hz, 1H), 8.61 - 8.34 (m, 3H), 8.16 (dd, J = 7.7, 2.9 Hz, 1H), 7.64 - 7.53 (m, 1H), 7.16 (dt, J = 12.5, 7.8 Hz, 1H), 7.00 (s, 1H), 6.85 - 6.67 (m, 2H), 4.81 (dd, J = 39.4, 27.6 Hz, 1H), 4.40 (dd, J = 11.6, 4.7 Hz, 1H), 4.21 (d, J = 3.9 Hz, 2H), 3.37 (d, J = 30.0 Hz, 2H), 3.17 (d, J = 6.8 Hz, 2H), 2.87 - 2.74 (m, 1H), 2.62 - 2.53 (m, 2H), 2.34 (d, J = 11.9 Hz, 2H), 2.24 - 2.11 (m, 1H), 1.96 - 1.83 (m, 1H), 1.62 (d, J = 7.3 Hz, 6H). LCMS (ESI) calcd for C₃₄H₃₇F₃N₇O₄⁺ [M+H]⁺: 664.29; found, 664.3.

### Example 610: Preparation of N-(2-(1-(4-(2,6-dioxopiperidin-3-yl)benzyl)piperidin-4-yl)-5-(2-hydroxypropan-2-yl)benzo[d]thiazol-6-yl)-6-(trifluoromethyl)picolinamide (GT-05763)

According to the method of step 2 of Scheme I-1, the target compound (GT-05763) was prepared (white solid, 16 mg, yield 27.91%). ¹H NMR (400 MHz, DMSO-d6) δ 12.58 (d, J = 5.3 Hz, 1H), 10.88 (d, J = 5.8 Hz, 2H), 9.11 (d, J = 6.8 Hz, 1H), 8.52 - 8.36 (m, 2H), 8.24 - 8.16 (m, 1H), 7.94 (d, J = 10.2 Hz, 1H), 7.62 (d, J = 8.2 Hz, 2H), 7.36 (s, 1H), 7.31 (d, J = 8.2 Hz, 1H), 4.32 (d, J = 4.8 Hz, 2H), 3.94 (dd, J = 11.7, 4.8 Hz, 1H), 3.40 (dd, J = 37.7, 26.1 Hz, 4H), 3.20 - 3.07 (m, 2H), 2.69 (td, J = 12.0, 6.0 Hz, 1H), 2.42 (s, 1H), 2.33 - 2.21 (m, 4H), 2.10 - 2.01 (m, 1H), 1.65 (d, J = 8.9 Hz, 6H). LCMS (ESI) calcd for C₃₄H₃₅F₃N₅O₄S⁺ [M+H]⁺: 666.24; found, 666.3.

### Example 611: Preparation of N-(2-(1-(4-(2,4-dioxotetrahydropyrimidin-1(2H)-yl)benzyl)piperidin-4-yl)-5-(2-hydroxypropan-2-yl)benzo[d]thiazol-6-yl)-6-(trifluoromethyl)picolinamide (GT-05764)

According to the method of step 2 of Scheme I-1, the target compound (GT-05764) was prepared (white solid, 25 mg, yield 43.55%). ¹H NMR (400 MHz, DMSO-d6) δ 12.58 (d, J = 5.1 Hz, 1H), 11.15 (s, 1H), 10.54 - 10.40 (m, 1H), 9.14 - 9.08 (m, 1H), 8.48 (dd, J = 7.6, 4.4 Hz, 1H), 8.43 - 8.35 (m, 1H), 8.20 (dd, J = 7.4, 4.5 Hz, 1H), 7.95 (d, J = 9.1 Hz, 1H), 7.68 (s, 1H), 7.44 (dt, J = 10.5, 5.2 Hz, 2H), 7.41 - 7.28 (m, 1H), 4.36 (dd, J = 25.2, 4.7 Hz, 2H), 3.84 (t, J = 6.7 Hz, 2H), 3.48 - 3.39 (m, 2H), 3.17 (dd, J = 44.1, 35.2 Hz, 3H), 2.72 (d, J = 7.5 Hz, 2H), 2.44 - 2.21 (m, 4H), 1.65 (d, J = 8.5 Hz, 6H). LCMS (ESI) calcd for C₃₃H₃₄F₃N₆O₄S⁺ [M+H]⁺: 667.23; found, 667.2.

### Example 612: Preparation of N-(2-(1-(3-(2,4-dioxotetrahydropyrimidin-1(2H)-yl)benzyl)piperidin-4-yl)-5-(2-hydroxypropan-2-yl)benzo[d]thiazol-6-yl)-6-(trifluoromethyl)picolinamide (GT-05765)

According to the method of step 2 of Scheme I-1, the target compound (GT-05765) was prepared (white solid, 26 mg, yield 45.29%). ¹H NMR (400 MHz, DMSO) δ 12.58 (d, J = 5.3 Hz, 1H), 11.11 (s, 1H), 10.45 (d, J = 10.0 Hz, 1H), 9.11 (d, J = 6.4 Hz, 1H), 8.52 - 8.44 (m, 1H), 8.39 (dd, J = 9.8, 5.8 Hz, 1H), 8.20 (t, J = 5.7 Hz, 1H), 7.94 (d, J = 7.9 Hz, 1H), 7.65 (s, 1H), 7.50 - 7.44 (m, 2H), 7.42 - 7.32 (m, 1H), 4.35 (d, J = 4.4 Hz, 2H), 3.95 - 3.82 (m, 3H), 3.47 (t, J = 13.3 Hz, 2H), 3.12 (d, J = 12.0 Hz, 2H), 2.73 (dt, J = 9.3, 6.7 Hz, 3H), 2.32 (t, J = 35.4 Hz, 4H), 1.65 (d, J = 8.7 Hz, 6H). LCMS (ESI) calcd for C₃₃H₃₄F₃N₆O₄S⁺ [M+H]⁺: 667.23; found, 667.2.

### Example 613: Preparation of N-(2-(1-((5-(2,4-dioxotetrahydropyrimidin-1(2H)-yl)pyridin-2-yl)methyl)piperidin-4-yl)-5-(2-hydroxypropan-2-yl)benzo[d]thiazol-6-yl)-6-(trifluoromethyl)picolinamide (GT-05766)

According to the method of step 2 of Scheme I-1, the target compound (GT-05766) was prepared (white solid, 23 mg, yield 40.00%). ¹H NMR (400 MHz, DMSO-_{d6}) δ 12.58 (s, 1H), 10.89 (s, 1H), 10.60 (s, 1H), 9.11 (s, 1H), 8.72 (d, J = 2.5 Hz, 1H), 8.47 (d, J = 7.8 Hz, 1H), 8.40 (t, J = 7.8 Hz, 1H), 8.20 (d, J = 7.8 Hz, 1H), 7.95 - 7.87 (m, 2H), 7.78 (d, J = 8.4 Hz, 1H), 4.49 (s, 2H), 3.90 (t, J = 6.6 Hz, 2H), 3.46 - 3.02 (m, 5H), 2.76 (t, J = 6.6 Hz, 2H), 2.31 (dd, J = 35.2, 11.3 Hz, 4H), 1.64 (s, 6H). LCMS (ESI) calcd for C₃₂H₃₃F₃N₇O₄S⁺ [M+H]⁺: 668.23; found, 668.2.

### Example 614: Preparation of N-(2-(1-((6-(2,4-dioxotetrahydropyrimidin-1(2H)-yl)pyridin-3-yl)methyl)piperidin-4-yl)-5-(2-hydroxypropan-2-yl)benzo[d]thiazol-6-yl)-6-(trifluoromethyl)picolinamide (GT-05767)

According to the method of step 2 of Scheme I-1, the target compound (GT-05767) was prepared (white solid, 25 mg, yield 43.48%). ¹H NMR (400 MHz, DMSO-d6) δ 12.58 (d, J = 5.2 Hz, 1H), 11.14 (s, 1H), 10.62 (d, J = 5.4 Hz, 1H), 9.11 (d, J = 7.8 Hz, 1H), 8.61 (dd, J = 13.4, 1.9 Hz, 1H), 8.51 - 8.45 (m, 1H), 8.39 (dd, J = 9.8, 5.9 Hz, 1H), 8.20 (t, J = 5.8 Hz, 1H), 8.10 (td, J = 8.3, 2.3 Hz, 1H), 7.97 - 7.83 (m, 2H), 4.38 (d, J = 4.2 Hz, 2H), 4.10 (dd, J = 14.4, 7.8 Hz, 2H), 3.50 (s, 2H), 3.37 - 3.09 (m, 3H), 2.71 (t, J = 6.5 Hz, 2H), 2.45 - 2.18 (m, 4H), 1.64 (d, J = 8.1 Hz, 6H). LCMS (ESI) calcd for C₃₂H₃₃F₃N₇O₄S⁺ [M+H]⁺: 668.23; found, 668.2.

### Example 615: Preparation of N-(2-(1-(3-((2,6-dioxopiperidin-3-yl)amino)benzyl)piperidin-4-yl)-5-(2-hydroxypropan-2-yl)benzo[d]thiazol-6-yl)-6-(trifluoromethyl)picolinamide (GT-05768)

According to the method of step 2 of Scheme I-1, the target compound (GT-05768) was prepared (white solid, 19 mg, yield 32.41%). ¹H NMR (400 MHz, DMSO-d6) δ 12.58 (d, J = 4.1 Hz, 1H), 11.03 - 10.69 (m, 2H), 9.11 (d, J = 4.7 Hz, 1H), 8.53 - 8.45 (m, 1H), 8.39 (dd, J = 9.5, 6.1 Hz, 1H), 8.20 (dd, J = 7.7, 3.8 Hz, 1H), 7.94 (d, J = 7.5 Hz, 1H), 7.16 (dd, J = 15.1, 7.3 Hz, 1H), 7.02 (d, J = 8.5 Hz, 1H), 6.85 - 6.72 (m, 2H), 4.40 (d, J = 4.8 Hz, 1H), 4.18 (s, 2H), 3.50 - 3.36 (m, 4H), 3.09 (d, J = 8.8 Hz, 2H), 2.83 (s, 1H), 2.61 (dd, J = 13.8, 3.8 Hz, 1H), 2.27 (d, J = 16.5 Hz, 4H), 1.65 (d, J = 7.9 Hz, 6H). LCMS (ESI) calcd for C₃₄H₃₆F₃N₆O₄S⁺ [M+H]⁺: 681.25; found, 681.3.

### Example 616: Preparation of N-(2-(1-(4-(2,6-dioxopiperidin-3-yl)benzyl)piperidin-4-yl)-5-(2-hydroxypropan-2-yl)benzo[d]oxazol-6-yl)-6-(trifluoromethyl)picolinamide (GT-05771)

According to the method of step 2 of Scheme I-1, the target compound (GT-05771) was prepared (white solid, 15 mg, yield 25.88%). ¹H NMR (400 MHz, DMSO-d6) δ 12.64 (d, J = 7.5 Hz, 1H), 10.89 (d, J = 5.1 Hz, 1H), 10.66 (s, 1H), 8.76 (d, J = 9.5 Hz, 1H), 8.47 - 8.37 (m, 2H), 8.25 - 8.18 (m, 1H), 7.71 (d, J = 9.5 Hz, 1H), 7.59 (t, J = 6.9 Hz, 2H), 7.36 - 7.31 (m, 2H), 4.31 (d, J = 10.8 Hz, 2H), 4.00 - 3.89 (m, 1H), 3.48 (d, J = 11.5 Hz, 2H), 3.12 (d, J = 8.0 Hz, 2H), 2.69 (d, J = 11.9 Hz, 1H), 2.55 (t, J = 5.9 Hz, 1H), 2.35 (d, J = 13.4 Hz, 2H), 2.20 (dd, J = 22.4, 8.5 Hz, 3H), 2.08 - 2.01 (m, 2H), 1.62 (d, J = 10.1 Hz, 6H). LCMS (ESI) calcd for C₃₄H₃₅F₃N₅O₅⁺ [M+H]⁺: 650.26; found, 650.3.

### Example 617: Preparation of N-(2-(1-(4-(2,4-dioxotetrahydropyrimidin-1(2H)-yl)benzyl)piperidin-4-yl)-5-(2-hydroxypropan-2-yl)benzo[d]oxazol-6-yl)-6-(trifluoromethyl)picolinamide (GT-05772)

According to the method of step 2 of Scheme I-1, the target compound (GT-05772) was prepared (white solid, 15 mg, yield 25.84%). ¹H NMR (400 MHz, DMSO-d6) δ 12.64 (d, J = 7.7 Hz, 1H), 10.98 (s, 1H), 10.45 (d, J = 5.9 Hz, 1H), 8.76 (d, J = 9.5 Hz, 1H), 8.47 - 8.37 (m, 2H), 8.19 (dd, J = 7.6, 3.8 Hz, 1H), 7.73 - 7.64 (m, 3H), 7.48 - 7.43 (m, 2H), 4.33 (d, J = 4.2 Hz, 2H), 3.85 - 3.80 (m, 2H), 3.49 (s, 2H), 3.12 (d, J = 7.6 Hz, 3H), 2.72 (d, J = 7.0 Hz, 2H), 2.44 - 2.34 (m, 2H), 2.26 - 2.20 (m, 1H), 2.03 (d, J = 13.2 Hz, 2H), 1.62 (d, J = 10.2 Hz, 6H). LCMS (ESI) calcd for C₃₃H₃₄F₃N₆O₅⁺ [M+H]⁺: 651.25; found, 651.3.

### Example 618: Preparation of N-(2-(1-(3-(2,4-dioxotetrahydropyrimidin-1(2H)-yl)benzyl)piperidin-4-yl)-5-(2-hydroxypropan-2-yl)benzo[d]oxazol-6-yl)-6-(trifluoromethyl)picolinamide (GT-05773)

According to the method of step 2 of Scheme I-1, the target compound (GT-05773) was prepared (white solid, 20 mg, yield 34.46%). ¹H NMR (400 MHz, DMSO-d6) δ 12.64 (d, J = 7.2 Hz, 1H), 10.42 (dd, J = 22.1, 7.2 Hz, 3H), 8.48 - 8.38 (m, 2H), 8.23 - 8.17 (m, 1H), 7.62 (d, J = 12.5 Hz, 1H), 7.49 - 7.45 (m, 3H), 7.42 - 7.39 (m, 1H), 7.31 (t, J = 4.4 Hz, 1H), 4.38 - 4.31 (m, 2H), 3.90 - 3.85 (m, 2H), 3.79 (d, J = 6.7 Hz, 1H), 3.15 (dd, J = 27.2, 22.9 Hz, 2H), 2.93 (d, J = 5.2 Hz, 1H), 2.73 (dd, J = 6.7, 2.6 Hz, 3H), 2.34 (dd, J = 43.4, 31.8 Hz, 2H), 2.00 (s, 2H), 1.70 - 1.48 (m, 6H). LCMS (ESI) calcd for C₃₃H₃₄F₃N₆O₅⁺ [M+H]⁺: 651.25; found, 651.3.

### Example 619: Preparation of N-(2-(1-((5-(2,4-dioxotetrahydropyrimidin-1(2H)-yl)pyridin-2-yl)methyl)piperidin-4-yl)-5-(2-hydroxypropan-2-yl)benzo[d]oxazol-6-yl)-6-(trifluoromethyl)picolinamide (GT-05774)

According to the method of step 2 of Scheme I-1, the target compound (GT-05774) was prepared (white solid, 21 mg, yield 36.13%). ¹H NMR (400 MHz, DMSO-d6) δ 10.59 (d, J = 14.3 Hz, 1H), 10.23 (s, 1H), 8.76 - 8.69 (m, 1H), 8.41 (tt, J = 10.6, 5.4 Hz, 2H), 8.23 - 8.18 (m, 1H), 8.16 - 8.12 (m, 1H), 7.91 (dt, J = 8.5, 4.3 Hz, 1H), 7.82 - 7.69 (m, 2H), 4.45 (d, J = 21.8 Hz, 2H), 3.90 (t, J = 5.8 Hz, 2H), 3.39 (d, J = 67.3 Hz, 6H), 3.17 - 2.82 (m, 3H), 2.76 (t, J = 6.6 Hz, 2H), 2.10 - 1.92 (m, 6H). LCMS (ESI) calcd for C₃₂H₃₃F₃N₇O₅⁺ [M+H]⁺: 652.25; found, 652.3.

### Example 620: Preparation of N-(2-(1-((6-(2,4-dioxotetrahydropyrimidin-1(2H)-yl)pyridin-3-yl)methyl)piperidin-4-yl)-5-(2-hydroxypropan-2-yl)benzo[d]oxazol-6-yl)-6-(trifluoromethyl)picolinamide (GT-05775)

According to the method of step 2 of Scheme I-1, the target compound (GT-05775) was prepared (white solid, 17 mg, yield 29.25%). ¹H NMR (400 MHz, DMSO) δ 10.75 (s, 1H), 10.62 (s, 1H), 9.38 (d, J = 10.2 Hz, 1H), 8.61 (t, J = 11.6 Hz, 1H), 8.49 - 8.35 (m, 2H), 8.24 - 8.18 (m, 1H), 8.17 - 8.13 (m, 1H), 8.08 (dd, J = 8.5, 2.4 Hz, 1H), 7.85 (dd, J = 13.4, 5.5 Hz, 1H), 5.44 (s, 1H), 5.06 (s, 1H), 4.32 (d, J = 22.2 Hz, 2H), 4.10 (t, J = 5.3 Hz, 2H), 3.42 - 3.20 (m, 3H), 2.87 (d, J = 54.1 Hz, 3H), 2.71 (t, J = 6.5 Hz, 2H), 2.07 (dd, J = 31.2, 16.0 Hz, 7H). LCMS (ESI) calcd for C₃₂H₃₃F₃N₇O₅⁺ [M+H]⁺: 652.25; found, 652.3.

### Example 621: Preparation of N-(2-(1-(3-((2,6-dioxopiperidin-3-yl)amino)benzyl)piperidin-4-yl)-5-(2-hydroxypropan-2-yl)benzo[d]oxazol-6-yl)-6-(trifluoromethyl)picolinamide (GT-05777)

According to the method of step 2 of Scheme I-1, the target compound (GT-05777) was prepared (white solid, 17 mg, yield 28.67%). ¹H NMR (400 MHz, DMSO-d6) δ 10.85 (s, 2H), 10.48 (s, 1H), 9.38 (s, 1H), 8.41 (td, J = 15.5, 7.8 Hz, 3H), 8.23 - 8.12 (m, 3H), 7.16 (dd, J = 7.8, 2.5 Hz, 1H), 6.97 (s, 1H), 6.79 (t, J = 8.7 Hz, 3H), 4.40 (s, 1H), 4.16 (s, 2H), 3.35 (d, J = 24.2 Hz, 2H), 3.10 (s, 2H), 2.83 - 2.74 (m, 2H), 2.63 (s, 1H), 2.32 (s, 1H), 2.18 (d, J = 10.3 Hz, 2H), 2.01 (s, 3H), 1.67 - 1.47 (m, 6H). LCMS (ESI) calcd for C₃₄H₃₆F₃N₆O₅⁺ [M+H]⁺: 665.27; found, 665.3.

### Example 622: Preparation of N-(3-(difluoromethyl)-1-(1-(4-(2,4-dioxotetrahydropyrimidin-1(2H)-yl)benzyl)piperidin-4-yl)-1H-pyrazol-4-yl)-5-morpholinopyrazolo[1,5-a]pyrimidine-3-carboxamide (GT-05798)

According to the method of step 2 of Scheme I-1, the target compound (GT-05798) was prepared (white solid, 25 mg, yield 43.01%). ¹H NMR (400 MHz, DMSO-d6) δ 10.88 (s, 2H), 9.43 (d, J = 9.5 Hz, 1H), 8.87 - 8.77 (m, 1H), 8.42 (d, J = 3.8 Hz, 1H), 8.32 - 8.28 (m, 1H), 7.65 - 7.52 (m, 2H), 7.34 (t, J = 8.0 Hz, 2H), 7.28 - 7.00 (m, 1H), 6.92 (t, J = 6.7 Hz, 1H), 4.59 (dd, J = 31.7, 20.4 Hz, 1H), 4.36 (dd, J = 35.1, 5.0 Hz, 2H), 3.94 (dd, J = 11.7, 4.8 Hz, 1H), 3.85 - 3.67 (m, 8H), 3.50 (s, 2H), 3.15 - 3.05 (m, 2H), 2.70 (ddd, J = 17.2, 11.9, 5.2 Hz, 1H), 2.35 (dd, J = 22.6, 11.7 Hz, 2H), 2.29 - 2.17 (m, 3H), 2.09 - 2.00 (m, 1H). LCMS (ESI) calcd for C₃₁H₃₅F₂N₁₀O₄⁺ [M+H]⁺: 649.28; found, 649.3.

### Example 623: Preparation of N-(3-(difluoromethyl)-1-(1-((5-(2,4-dioxotetrahydropyrimidin-1(2H)-yl)pyridin-2-yl)methyl)piperidin-4-yl)-1H-pyrazol-4-yl)-5-morpholinopyrazolo[1,5-a]pyrimidine-3-carboxamide (GT-05799)

According to the method of step 2 of Scheme I-1, the target compound (GT-05799) was prepared (white solid, 26 mg, yield 44.67%). ¹H NMR (400 MHz, DMSO-d6) δ 10.92 (s, 1H), 10.59 (s, 1H), 9.43 (s, 1H), 8.83 (d, J = 7.9 Hz, 1H), 8.71 (d, J = 2.5 Hz, 1H), 8.44 (d, J = 12.3 Hz, 1H), 8.30 (s, 1H), 7.92 (dd, J = 8.4, 2.6 Hz, 1H), 7.76 (d, J = 8.4 Hz, 1H), 7.14 (t, J = 53.5 Hz, 1H), 6.92 (d, J = 8.0 Hz, 1H), 4.60 (s, 1H), 4.51 (s, 2H), 3.89 (t, J = 6.6 Hz, 2H), 3.81 - 3.71 (m, 9H), 3.29 (d, J = 10.9 Hz, 2H), 2.76 (t, J = 6.6 Hz, 2H), 2.35 (dt, J = 27.8, 10.9 Hz, 5H). LCMS (ESI) calcd for C₃₀H₃₄F₂N₁₁O₄⁺ [M+H]⁺: 650.28; found, 650.3.

### Example 624: Preparation of N-(3-(difluoromethyl)-1-(1-((6-(2,4-dioxotetrahydropyrimidin-1(2H)-yl)pyridin-3-yl)methyl)piperidin-4-yl)-1H-pyrazol-4-yl)-5-morpholinopyrazolo[1,5-a]pyrimidine-3-carboxamide (GT-05800)

According to the method of step 2 of Scheme I-1, the target compound (GT-05800) was prepared (white solid, 40 mg, yield 68.72%). ¹H NMR (400 MHz, DMSO-d6) δ 11.41 (s, 1H), 10.60 (d, J = 20.5 Hz, 1H), 9.43 (d, J = 11.2 Hz, 1H), 8.83 (t, J = 6.7 Hz, 1H), 8.64 (t, J = 3.5 Hz, 1H), 8.42 (s, 1H), 8.30 (d, J = 5.9 Hz, 1H), 8.15 - 8.09 (m, 1H), 7.91 - 7.85 (m, 1H), 7.32 - 7.00 (m, 1H), 6.92 (t, J = 6.7 Hz, 1H), 4.59 (dd, J = 31.9, 20.8 Hz, 1H), 4.40 (t, J = 17.3 Hz, 2H), 4.11 (d, J = 6.4 Hz, 2H), 3.72 (d, J = 4.4 Hz, 7H), 3.49 (d, J = 11.5 Hz, 2H), 3.19 (t, J = 42.9 Hz, 3H), 2.71 (t, J = 6.5 Hz, 2H), 2.38 (dd, J = 22.6, 12.3 Hz, 2H), 2.25 (d, J = 11.3 Hz, 2H). LCMS (ESI) calcd for C₃₀H₃₄F₂N₁₁O₄⁺ [M+H]⁺: 650.28; found, 650.3.

### Example 625: Preparation of N-(3-(difluoromethyl)-1-(1-(3-((2,6-dioxopiperidin-3-yl)amino)benzyl)piperidin-4-yl)-1H-pyrazol-4-yl)-5-morpholinopyrazolo[1,5-a]pyrimidine-3-carboxamide (GT-05801)

According to the method of step 2 of Scheme I-1, the target compound (GT-05801) was prepared (white solid, 26 mg, yield 43.79%). ¹H NMR (400 MHz, DMSO-d6) δ 11.00 (s, 1H), 10.86 (s, 1H), 9.43 (d, J = 10.1 Hz, 1H), 8.83 (dd, J = 7.8, 5.7 Hz, 1H), 8.42 (s, 1H), 8.30 (d, J = 6.6 Hz, 1H), 7.45 - 7.10 (m, 2H), 7.03 (t, J = 8.7 Hz, 1H), 6.92 (t, J = 6.8 Hz, 1H), 6.80 (d, J = 6.4 Hz, 1H), 4.58 (dd, J = 33.8, 21.9 Hz, 1H), 4.40 (dd, J = 11.7, 4.8 Hz, 1H), 4.25 (dd, J = 48.4, 12.7 Hz, 2H), 3.79 (s, 8H), 3.47 (d, J = 11.7 Hz, 2H), 3.09 (dd, J = 23.8, 11.4 Hz, 2H), 2.83 (ddd, J = 17.5, 12.4, 5.3 Hz, 1H), 2.66 - 2.57 (m, 1H), 2.47 - 2.13 (m, 5H), 2.09 - 1.84 (m, 1H). LCMS (ESI) calcd for C₃₂H₃₇F₂N₁₀O₄⁺ [M+H]⁺: 663.30; found, 663.3.

### Example 626: Preparation of (R)-N-(2-(4-(4-(2,4-dioxotetrahydropyrimidin-1(2H)-yl)benzyl)piperazin-1-yl)-5-(3-hydroxypyrrolidin-1-yl)oxazolo[4,5-b]pyridin-6-yl)-2-(2-methylpyridin-4-yl)oxazole-4-carboxamide (GT-05807)

According to the method of step 2 of Scheme I-1, the target compound (GT-05807) was prepared (white solid, 17 mg, yield 30.09%). ¹H NMR (400 MHz, DMSO-d6) δ 11.90 (s, 1H), 10.45 (s, 1H), 10.07 (d, J = 4.4 Hz, 1H), 9.87 (s, 1H), 9.18 (d, J = 3.2 Hz, 1H), 8.92 (d, J = 6.0 Hz, 1H), 8.37 (s, 1H), 8.26 (d, J = 5.9 Hz, 1H), 7.81 - 7.63 (m, 3H), 7.43 (dd, J = 8.4, 4.4 Hz, 2H), 4.39 (d, J = 13.4 Hz, 4H), 3.68 - 3.27 (m, 13H), 2.83 (s, 3H), 2.73 (t, J = 6.6 Hz, 2H), 1.94 - 1.85 (m, 1H), 1.78 (d, J = 8.9 Hz, 1H). LCMS (ESI) calcd for C₃₅H₃₇N₁₀O₆⁺ [M+H]⁺: 693.29; found, 693.3.

### Example 627: Preparation of (R)-N-(2-(4-(3-(2,4-dioxotetrahydropyrimidin-1(2H)-yl)benzyl)piperazin-1-yl)-5-(3-hydroxypyrrolidin-1-yl)oxazolo[4,5-b]pyridin-6-yl)-2-(2-methylpyridin-4-yl)oxazole-4-carboxamide (GT-05808)

According to the method of step 2 of Scheme I-1, the target compound (GT-05808) was prepared (white solid, 16 mg, yield 28.32%). ¹H NMR (400 MHz, DMSO-d6) δ 10.45 (d, J = 2.1 Hz, 1H), 10.05 (d, J = 7.9 Hz, 1H), 9.83 (s, 1H), 9.31 - 9.08 (m, 1H), 8.91 (d, J = 6.0 Hz, 1H), 8.35 (s, 1H), 8.23 (d, J = 5.8 Hz, 1H), 7.64 (d, J = 2.8 Hz, 1H), 7.56 - 7.44 (m, 4H), 4.40 (d, J = 11.5 Hz, 4H), 3.63 - 3.25 (m, 13H), 2.81 (s, 3H), 2.73 (td, J = 6.6, 3.4 Hz, 2H), 2.03 - 1.85 (m, 1H), 1.78 (d, J = 3.7 Hz, 1H). LCMS (ESI) calcd for C₃₅H₃₇N₁₀O₆⁺ [M+H]⁺: 693.29; found, 693.3.

### Example 628: Preparation of (R)-N-(2-(4-((5-(2,4-dioxotetrahydropyrimidin-1(2H)-yl)pyridin-2-yl)methyl)piperazin-1-yl)-5-(3-hydroxypyrrolidin-1-yl)oxazolo[4,5-b]pyridin-6-yl)-2-(2-methylpyridin-4-yl)oxazole-4-carboxamide (GT-05809)

According to the method of step 2 of Scheme I-1, the target compound (GT-05809) was prepared (white solid, 15 mg, yield 26.52%). ¹H NMR (400 MHz, DMSO-d6) δ 10.49 (d, J = 82.9 Hz, 1H), 10.09 (s, 1H), 9.93 (s, 1H), 9.32 - 9.13 (m, 1H), 8.93 (d, J = 6.1 Hz, 1H), 8.71 (t, J = 2.3 Hz, 1H), 8.40 (s, 1H), 8.28 (d, J = 6.0 Hz, 1H), 7.93 (dt, J = 8.4, 2.2 Hz, 1H), 7.82 - 7.48 (m, 2H), 3.90 - 3.86 (m, 3H), 3.66 - 3.24 (m, 14H), 2.84 (s, 3H), 2.76 (t, J = 6.6 Hz, 2H), 2.00 - 1.86 (m, 1H), 1.78 (d, J = 4.8 Hz, 1H). LCMS (ESI) calcd for C₃₄H₃₆N₁₁O₆⁺ [M+H]⁺: 694.29; found, 694.3.

### Example 629: Preparation of (R)-N-(2-(4-((6-(2,4-dioxotetrahydropyrimidin-1(2H)-yl)pyridin-3-yl)methyl)piperazin-1-yl)-5-(3-hydroxypyrrolidin-1-yl)oxazolo[4,5-b]pyridin-6-yl)-2-(2-methylpyridin-4-yl)oxazole-4-carboxamide (GT-05810)

According to the method of step 2 of Scheme I-1, the target compound (GT-05810) was prepared (white solid, 10 mg, yield 17.68%). ¹H NMR (400 MHz, DMSO-d6) δ 10.61 (s, 1H), 10.50 - 10.05 (m, 1H), 9.93 (s, 2H), 9.32 - 9.12 (m, 1H), 8.93 (d, J = 6.1 Hz, 1H), 8.71 - 8.60 (m, 1H), 8.41 (s, 1H), 8.29 (d, J = 6.0 Hz, 1H), 8.12 (dd, J = 8.7, 2.3 Hz, 1H), 7.85 (dd, J = 8.6, 5.7 Hz, 1H), 7.75 - 7.45 (m, 1H), 4.45 (s, 2H), 4.09 (dd, J = 8.6, 4.4 Hz, 5H), 3.60 - 3.28 (m, 11H), 2.84 (s, 3H), 2.69 (t, J = 6.5 Hz, 2H), 1.88 (d, J = 8.4 Hz, 1H), 1.77 (d, J = 3.8 Hz, 1H). LCMS (ESI) calcd for C₃₄H₃₆N₁₁O₆⁺ [M+H]⁺: 694.29; found, 694.3.

### Example 630: Preparation of N-(2-(4-(3-((2,6-dioxopiperidin-3-yl)amino)benzyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide (GT-05811)

According to the method of step 2 of Scheme I-1, the target compound (GT-05811) was prepared (white solid, 15 mg, yield 26.03%). ¹H NMR (400 MHz, DMSO-d6) δ 10.86 (s, 1H), 10.21 (d, J = 109.4 Hz, 1H), 9.84 (s, 1H), 9.29 - 9.15 (m, 1H), 8.92 (d, J = 6.1 Hz, 1H), 8.39 (s, 1H), 8.27 (d, J = 6.0 Hz, 1H), 7.75 - 7.47 (m, 1H), 7.20 - 7.12 (m, 1H), 6.86 (dd, J = 49.6, 42.6 Hz, 2H), 4.40 (dd, J = 7.8, 3.6 Hz, 4H), 3.45 (dddd, J = 54.0, 43.0, 15.9, 7.8 Hz, 12H), 2.89 - 2.77 (m, 4H), 2.60 (dd, J = 17.3, 3.5 Hz, 1H), 2.19 (dd, J = 8.3, 3.7 Hz, 1H), 2.03 - 1.83 (m, 2H), 1.79 (s, 1H). LCMS (ESI) calcd for C₃₆H₃₉N₁₀O₆⁺ [M+H]⁺: 707.31; found, 707.3.

### Example 631: Preparation of N-(2-(1-((5-(2,4-dioxotetrahydropyrimidin-1(2H)-yl)pyridin-2-yl)methyl)piperidin-4-yl)-6-methoxy-2H-indazol-7-yl)-3-(trifluoromethyl)benzamide (GT-04886)

According to the method of step 2 of Scheme I-1, the target compound (GT-04886) was prepared (white solid, 26 mg, yield 44.06%). ¹H NMR (400 MHz, DMSO) δ 10.69 (s, 1H), 10.57 (s, 1H), 10.11 (s, 1H), 8.69 (d, J = 2.4 Hz, 1H), 8.38 (dd, J = 30.7, 10.8 Hz, 3H), 7.98 (d, J = 7.6 Hz, 1H), 7.89 (dd, J = 8.4, 2.5 Hz, 1H), 7.79 (t, J = 7.8 Hz, 1H), 7.70 (dd, J = 8.6, 3.7 Hz, 2H), 7.12 (d, J = 9.2 Hz, 1H), 4.79 (s, 1H), 4.49 (s, 2H), 3.87 (t, J = 6.6 Hz, 2H), 3.83 (s, 3H), 3.59 (dd, J = 21.8, 9.1 Hz, 2H), 3.29 (d, J = 11.7 Hz, 2H), 2.75 (t, J = 6.6 Hz, 2H), 2.45 (d, J = 11.0 Hz, 1H), 2.34 (d, J = 11.0 Hz, 2H). LCMS (ESI) calcd for C₃₁H₃₁F₃N₇O₄⁺ [M+H]⁺: 622.24; found, 622.3.

### Example 632: Preparation of N-(2-(1-((6-(2,4-dioxotetrahydropyrimidin-1(2H)-yl)pyridin-3-yl)methyl)piperidin-4-yl)-6-methoxy-2H-indazol-7-yl)-3-(trifluoromethyl)benzamide (GT-04887)

According to the method of step 2 of Scheme I-1, the target compound (GT-04887) was prepared (white solid, 35 mg, yield 58.89%). ¹H NMR (400 MHz, DMSO) δ 10.89 (s, 1H), 10.61 (s, 1H), 10.14 (d, J = 29.2 Hz, 1H), 8.64 - 8.55 (m, 1H), 8.48 - 8.26 (m, 3H), 8.05 (dd, J = 8.7, 2.2 Hz, 1H), 7.98 (t, J = 8.2 Hz, 1H), 7.87 (d, J = 8.6 Hz, 1H), 7.79 (dd, J = 17.4, 8.5 Hz, 1H), 7.71 (t, J = 9.2 Hz, 1H), 7.12 (t, J = 10.4 Hz, 1H), 4.79 (dd, J = 36.6, 24.9 Hz, 1H), 4.37 (d, J = 3.5 Hz, 2H), 4.09 (t, J = 6.5 Hz, 2H), 3.84 (d, J = 13.8 Hz, 3H), 3.65 - 3.50 (m, 2H), 3.32 - 3.09 (m, 3H), 2.70 (t, J = 6.5 Hz, 2H), 2.37 (dd, J = 40.5, 11.4 Hz, 3H). LCMS (ESI) calcd for C₃₁H₃₁F₃N₇O₄⁺ [M+H]⁺: 622.24; found, 622.3.

### Example 633: Preparation of N-(2-(1-(4-(2,6-dioxopiperidin-3-yl)benzyl)piperidin-4-yl)-6-methoxy-2H-indazol-7-yl)-3-(trifluoromethyl)benzamide (GT-04813)

According to the method of step 2 of Scheme I-1, the target compound (GT-04813) was prepared (white solid, 25 mg, yield 32.0 %). ¹H NMR (400 MHz, DMSO) δ 10.88 (s, 1H), 10.37 (s, 1H), 10.10 (s, 1H), 8.41 (s, 2H), 8.33 (s, 1H), 7.97 (s, 1H), 7.80 (d, *J =* 7.8 Hz, 1H), 7.71 (d, *J =* 9.1 Hz, 1H), 7.55 (d, *J =* 8.0 Hz, 2H), 7.34 (d, *J* = 8.1 Hz, 1H), 7.12 (d, *J* = 9.2 Hz, 1H), 4.76 (s, 1H), 4.33 (d, *J* = 4.8 Hz, 2H), 3.93 (dd, *J =* 11.7, 4.9 Hz, 1H), 3.85 (d, *J =* 14.8 Hz, 3H), 3.52 (s, 2H), 3.16 (d, *J =* 7.9 Hz, 2H), 2.69 (ddd, *J =* 17.1, 12.2, 5.3 Hz, 2H), 2.48 - 2.37 (m, 2H), 2.32 (d, *J* = 13.4 Hz, 2H), 2.22 (dd, *J* = 12.4, 4.2 Hz, 1H), 2.07 - 1.97 (m, 1H). LCMS (ESI) calcd for C₃₃H₃₃F₃N₅O₄ ⁺ [M+H]⁺: 620.25; found, 620.3.

### Example 634: Preparation of N-(2-(1-(4-(2,6-dioxopiperidin-3-yl)-3-fluorobenzyl)piperidin-4-yl)-6-methoxy-2H-indazol-7-yl)-3-(trifluoromethyl)benzamide (GT-04814)

According to the method of step 2 of Scheme I-1, the target compound (GT-04814) was prepared (white solid, 32 mg, yield 39.9 %). ¹H NMR (400 MHz, DMSO) δ 10.93 (s, 1H), 10.45 (s, 1H), 10.10 (s, 1H), 8.41 (s, 1H), 8.35 (s, 1H), 7.97 (s, 1H), 7.80 (d, *J* = 7.3 Hz, 1H), 7.71 (d, *J* = 9.0 Hz, 1H), 7.50 (d, *J* = 11.2 Hz, 1H), 7.42 (dt, *J =* 15.1, 7.7 Hz, 2H), 7.12 (d, *J =* 9.3 Hz, 1H), 4.77 (s, 1H), 4.36 (d, *J =* 4.2 Hz, 2H), 4.12 (dd, *J* = 12.4, 4.8 Hz, 1H), 3.85 (d, *J* = 13.6 Hz, 3H), 3.31 (s, 2H), 3.18 (d, *J* = 10.6 Hz, 2H), 2.76 (dd, *J =* 21.4, 9.2 Hz, 2H), 2.47 - 2.36 (m, 2H), 2.32 (d, *J* = 11.6 Hz, 2H), 2.25 - 2.12 (m, 1H), 2.05 - 1.91 (m, 1H). LCMS (ESI) calcd for C₃₃H₃₂F₄N₅O₄ ⁺ [M+H]⁺: 638.24; found, 638.3.

### Example 635: Preparation of N-(2-(1-(3-((2,6-dioxopiperidin-3-yl)amino)benzyl)piperidin-4-yl)-6-methoxy-2H-indazol-7-yl)-3-(trifluoromethyl)benzamide (GT-04815)

According to the method of step 2 of Scheme I-1, the target compound (GT-04815) was prepared(white solid, 31 mg, yield 38.8 %). ¹H NMR (400 MHz, DMSO) δ 10.83 (s, 1H), 10.38 (s, 1H), 10.10 (s, 1H), 8.40 (s, 2H), 8.34 (s, 1H), 7.98 (d, *J* = 7.2 Hz, 1H), 7.79 (d, *J =* 7.0 Hz, 1H), 7.71 (t, *J =* 8.3 Hz, 1H), 7.21 - 7.06 (m, 2H), 6.89 (s, 1H), 6.76 (t, *J =* 9.3 Hz, 2H), 4.74 (t, *J =* 11.8 Hz, 1H), 4.36 (dd, *J =* 11.5, 4.7 Hz, 1H), 4.19 (d, *J* = 4.8 Hz, 2H), 3.82 (s, 3H), 3.28 (s, 1H), 3.17 - 3.08 (m, 2H), 2.78 (ddd, *J* = 17.6, 12.2, 5.5 Hz, 1H), 2.67 - 2.55 (m, 2H), 2.43 (d, *J =* 12.3 Hz, 2H), 2.32 (s, 1H), 2.29 (s, 1H), 2.15 (dd, *J =* 8.5, 4.1 Hz, 1H), 1.90 (dd, *J =* 12.4, 4.3 Hz, 1H). LCMS (ESI) calcd for C₃₃H₃₄F₃N₆O⁴⁺ [M+H]⁺: 635.26; found, 635.3.

### Example 636: Preparation of N-(2-(1-(3-(2,4-dioxotetrahydropyrimidin-1(2H)-yl)benzyl)piperidin-4-yl)-6-methoxy-2H-indazol-7-yl)-3-(trifluoromethyl)benzamide (GT-04816)

According to the method of step 2 of Scheme I-1, the target compound (GT-04816) was prepared (white solid, 35 mg, yield 44.8 %). ¹H NMR (400 MHz, DMSO) δ 10.46 (s, 1H), 10.31 (s, 1H), 10.10 (s, 1H), 8.41 (s, 1H), 8.34 (s, 1H), 7.98 (d, *J =* 6.8 Hz, 1H), 7.79 (s, 1H), 7.71 (d, *J =* 9.1 Hz, 1H), 7.56 (s, 1H), 7.52 - 7.44 (m, 2H), 7.41 (d, *J =* 6.6 Hz, 1H), 7.12 (d, *J =* 9.3 Hz, 1H), 4.76 (s, 1H), 4.36 (d, *J =* 4.4 Hz, 2H), 3.90 - 3.84 (m, 2H), 3.83 (s, 2H), 3.52 (d, *J =* 11.4 Hz, 2H), 3.32 (s, 1H), 3.22 - 3.11 (m, 2H), 2.73 (t, *J =* 6.5 Hz, 2H), 2.47 - 2.36 (m, 2H), 2.31 (d, *J =* 14.1 Hz, 2H). LCMS (ESI) calcd for C₃₂H₃₂F₃N₆O₄ ⁺ [M+H]⁺: 621.24; found, 621.3.

### Example 637: Preparation of N-(2-(1-(4-(2,4-dioxotetrahydropyrimidin-1(2H)-yl)-3-fluorobenzyl)piperidin-4-yl)-6-methoxy-2H-indazol-7-yl)-3-(trifluoromethyl)benzamide (GT-04817)

According to the method of step 2 of Scheme I-1, the target compound (GT-04817) was prepared (white solid, 37 mg, yield 46.1 %). ¹H NMR (400 MHz, DMSO) δ 10.56 (s, 1H), 10.45 (s, 1H), 10.10 (s, 1H), 8.41 (s, 1H), 8.34 (s, 1H), 7.98 (s, 1H), 7.80 (d, *J =* 7.6 Hz, 1H), 7.72 (t, *J =* 8.8 Hz, 1H), 7.61 (d, *J =* 11.1 Hz, 1H), 7.54 (d, *J =* 8.0 Hz, 1H), 7.46 (d, *J =* 7.8 Hz, 1H), 7.13 (t, *J =* 10.1 Hz, 1H), 4.78 (s, 1H), 4.37 (s, 2H), 3.84 (d, *J =* 13.3 Hz, 3H), 3.74 (t, *J =* 6.6 Hz, 2H), 3.52 (d, *J =* 10.6 Hz, 2H), 3.18 (d, *J =* 12.0 Hz, 2H), 2.73 (t, *J* = 6.6 Hz, 2H), 2.47 - 2.38 (m, 2H), 2.32 (d, *J* = 10.0 Hz, 2H). LCMS (ESI) calcd for C₃₂H₃₁F₄N₆O₄ ⁺ [M+H]⁺: 639.23; found, 639.3.

### Example 638: Preparation of N-(2-(1-(4-(2,4-dioxotetrahydropyrimidin-1(2H)-yl)benzyl)piperidin-4-yl)-6-methoxy-2H-indazol-7-yl)-3-(trifluoromethyl)benzamide (GT-04818)

According to the method of step 2 of Scheme I-1, the target compound (GT-04818) was prepared (white solid, 36 mg, yield 46.0 %). ¹H NMR (400 MHz, DMSO) δ 10.45 (s, 1H), 10.28 (s, 1H), 10.10 (s, 1H), 8.41 (s, 2H), 8.35 (s, 1H), 7.97 (s, 1H), 7.80 (d, *J =* 7.2 Hz, 1H), 7.71 (d, *J =* 9.1 Hz, 1H), 7.58 (t, *J =* 7.6 Hz, 2H), 7.44 (d, *J =* 8.4 Hz, 2H), 7.12 (d, *J* = 9.2 Hz, 1H), 4.77 (s, 1H), 4.34 (d, *J =* 4.3 Hz, 2H), 3.85 (d, *J =* 9.3 Hz, 1H), 3.83 (s, 3H), 3.51 (d, *J =* 11.2 Hz, 2H), 3.32 - 3.26 (m, 1H), 3.17 (d, *J =* 12.5 Hz, 2H), 2.72 (t, *J =* 6.7 Hz, 2H), 2.42 (d, *J =* 11.4 Hz, 2H), 2.36 - 2.30 (m, 2H). LCMS (ESI) calcd for C₃₂H₃₂F₃N₆O₄ ⁺ [M+H]⁺: 621.24; found, 621.3.

### Example 639: Preparation of N-(2-(1-(4-(2,4-dioxotetrahydropyrimidin-1(2H)-yl)-2-fluorobenzyl)piperidin-4-yl)-6-methoxy-2H-indazol-7-yl)-3-(trifluoromethyl)benzamide (GT-04819)

According to the method of step 2 of Scheme I-1, the target compound (GT-04819) was prepared (white solid, 38 mg, yield 47.3 %). ¹H NMR (400 MHz, DMSO) δ 10.54 (s, 1H), 10.37 (s, 1H), 10.10 (s, 1H), 8.41 (s, 2H), 8.35 (s, 1H), 7.98 (d, *J* = 6.9 Hz, 1H), 7.83 - 7.76 (m, 1H), 7.70 (t, *J* = 8.3 Hz, 2H), 7.41 (d, *J =* 11.3 Hz, 1H), 7.33 (d, *J* = 8.7 Hz, 1H), 7.12 (d, *J* = 9.2 Hz, 1H), 4.77 (s, 1H), 4.38 (s, 2H), 3.86 (d, *J* = 5.5 Hz, 2H), 3.83 (s, 3H), 3.54 (s, 2H), 3.26 (d, *J* = 9.1 Hz, 2H), 2.72 (t, *J* = 6.3 Hz, 2H), 2.48 - 2.38 (m, 2H), 2.38 - 2.26 (m, 2H). LCMS (ESI) calcd for C₃₂H₃₁F₄N₆O₄ ⁺ [M+H]⁺: 639.23; found, 639.3.

### Example 640: Preparation of 5-((1R,4R)-2-oxa-5-azabicyclo[2.2.1]heptan-5-yl)-N-(3-(difluoromethyl)-1-(1-(4-(2,4-dioxotetrahydropyrimidin-1(2H)-yl)benzyl)piperidin-4-yl)-1H-pyrazol-4-yl)pyrazolo[1,5-a]pyrimidine-3-carboxamide (GT-05029)

According to the method of step 2 of Scheme I-1, the target compound (GT-05029) was prepared (white solid, 29 mg, yield 41.2 %). ¹H NMR (400 MHz, DMSO-d6) δ 10.77 (s, 1H), 10.45 (s, 1H), 9.52 (d, J = 5.9 Hz, 1H), 8.79 (d, J = 7.7 Hz, 1H), 8.43 (d, J = 3.0 Hz, 1H), 8.32 - 8.18 (m, 1H), 7.65 (d, J = 8.8 Hz, 2H), 7.44 (d, J = 8.2 Hz, 2H), 4.77 (d, J = 19.5 Hz, 1H), 4.52 (s, 1H), 4.43 - 4.22 (m, 2H), 3.85 - 3.79 (m, 3H), 3.76 - 3.57 (m, 2H), 3.53 - 3.45 (m, 2H), 3.29 - 2.96 (m, 3H), 2.73 (t, J = 6.6 Hz, 2H), 2.44 - 2.09 (m, 5H), 2.07 - 1.90 (m, 2H). LCMS (ESI) calcd for C₃₂H₃₅F₂N₁₀O₄⁺ [M+H]⁺: 661.28; found, 661.3.

### Example 641: Preparation of N-(5-((4-(3-((2,6-dioxopiperidin-3-yl)amino)benzyl)piperazin-1-yl)methyl)-1-((1s,4s)-4-(hydroxymethyl)cyclohexyl)-1H-benzo[d]imidazol-2-yl)-3-(trifluoromethyl)benzamide (GT-04976)

According to the method of step 2 of Scheme I-1, the target compound (GT-04976) was prepared (white solid, 26 mg, yield 50.5 %). ¹H NMR (400 MHz, DMSO) δ 11.65 (s, 1H), 10.84 (s, 1H), 8.50 (d, *J =* 7.9 Hz, 1H), 8.45 (s, 1H), 7.92 (d, *J* = 7.5 Hz, 1H), 7.77 (d, *J* = 7.7 Hz, 1H), 7.72 (t, *J* = 9.6 Hz, 2H), 7.52 (s, 1H), 7.15 (t, *J* = 7.8 Hz, 1H), 6.92 (s, 1H), 6.77 (d, *J* = 6.4 Hz, 2H), 4.75 (s, 1H), 4.43 (s, 2H), 4.36 (dd, *J =* 11.4, 4.8 Hz, 2H), 4.20 (s, 2H), 3.69 (d, *J* = 7.5 Hz, 4H), 3.13 (dd, *J* = 7.4, 4.2 Hz, 1H), 2.84 - 2.72 (m, 2H), 2.61 (s, 2H), 2.15 (s, 1H), 1.95 (d, *J =* 13.1 Hz, 2H), 1.88 (d, *J =* 11.7 Hz, 2H), 1.74 - 1.60 (m, 4H), 1.28 (dd, *J =* 10.6, 6.6 Hz, 2H). LCMS (ESI) calcd for C₃₉H₄₅F₃N₇O₄ ⁺ [M+H]⁺: 732.35; found, 732.2.

### Example 642: Preparation of N-(5-((4-(3-(2,4-dioxotetrahydropyrimidin-1(2H)-yl)benzyl)piperazin-1-yl)methyl)-1-((1s,4s)-4-(hydroxymethyl)cyclohexyl)-1H-benzo[d]imidazol-2-yl)-3-(trifluoromethyl)benzamide (GT-04977)

According to the method of step 2 of Scheme I-1, the target compound (GT-04977) was prepared (white solid, 24 mg, yield 47.5 %). ¹H NMR (400 MHz, DMSO) δ 13.08 (s, 1H), 10.42 (d, *J =* 14.2 Hz, 1H), 8.50 (d, *J =* 7.7 Hz, 1H), 8.45 (s, 1H), 7.92 (d, *J* = 7.7 Hz, 1H), 7.77 (d, *J =* 7.6 Hz, 1H), 7.71 (t, *J =* 11.0 Hz, 2H), 7.53 (s, 2H), 7.46 - 7.38 (m, 3H), 7.31 (d, *J* = 6.7 Hz, 1H), 4.77 (s, 1H), 4.76 - 4.67 (m, 1H), 4.45 (d, *J* = 41.0 Hz, 4H), 3.83 (t, *J* = 7.3 Hz, 3H), 3.79 (d, *J* = 6.7 Hz, 1H), 3.69 (d, *J* = 7.5 Hz, 3H), 3.56 (s, 3H), 2.70 (dd, *J* = 14.8, 8.2 Hz, 3H), 1.95 (d, *J* = 12.4 Hz, 2H), 1.86 (s, 1H), 1.76 - 1.52 (m, 4H). LCMS (ESI) calcd for C₃₈H₄₃F₃N₇O₄⁺ [M+H]⁺: 718.33; found, 718.3.

### Example 643: Preparation of N-(5-((4-(4-(2,6-dioxopiperidin-3-yl)benzyl)piperazin-1-yl)methyl)-1-((1s,4s)-4-(hydroxymethyl)cyclohexyl)-1H-benzo[d]imidazol-2-yl)-3-(trifluoromethyl)benzamide (GT-04978)

According to the method of step 2 of Scheme I-1, the target compound (GT-04978) was prepared (white solid, 17 mg, yield 33.7 %). ¹H NMR (400 MHz, DMSO) δ 13.03 (s, 1H), 10.86 (s, 1H), 8.50 (d, *J =* 8.0 Hz, 1H), 8.45 (s, 1H), 7.92 (d, *J =* 7.6 Hz, 1H), 7.76 (t, *J =* 7.8 Hz, 1H), 7.68 (s, 2H), 7.50 (s, 3H), 7.30 (d, *J* = 8.0 Hz, 2H), 4.75 (s, 1H), 4.29 (s, 4H), 3.91 (dd, *J* = 11.4, 4.8 Hz, 2H), 3.69 (d, *J =* 7.5 Hz, 3H), 3.49 (s, 6H), 2.72 - 2.61 (m, 2H), 2.21 (d, *J* = 9.0 Hz, 1H), 2.07 - 1.99 (m, 1H), 1.95 (d, *J* = 12.5 Hz, 2H), 1.86 (s, 1H), 1.73 - 1.59 (m, 4H). LCMS (ESI) calcd for C₃₉H₄₄F₃N₆O₄ ⁺ [M+H]⁺: 717.34; found, 717.2.

### Example 644: Preparation of N-(5-(4-(4-(2,6-dioxopiperidin-3-yl)benzyl)piperazin-1-yl)-1-((1s,4s)-4-(hydroxymethyl)cyclohexyl)-1H-benzo[d]imidazol-2-yl)-3-(trifluoromethyl)benzamide (GT-04981)

According to the method of step 2 of Scheme I-1, the target compound (GT-04981) was prepared (white solid, 30 mg, yield 46.7 %). ¹H NMR (400 MHz, DMSO-d6) δ 12.78 (s, 1H), 11.09 (s, 1H), 10.89 (s, 1H), 8.49 (d, J = 7.8 Hz, 1H), 8.44 (s, 1H), 7.90 (d, J = 7.7 Hz, 1H), 7.74 (t, J = 7.8 Hz, 1H), 7.62 (d, J = 8.2 Hz, 2H), 7.52 (d, J = 8.9 Hz, 1H), 7.35 (d, J = 8.2 Hz, 2H), 7.20 (d, J = 2.2 Hz, 1H), 7.00 (dd, J = 9.0, 2.2 Hz, 1H), 4.70 (t, J = 12.4 Hz, 1H), 4.38 (d, J = 4.5 Hz, 2H), 3.94 (dd, J = 11.8, 4.9 Hz, 2H), 3.69 (t, J = 10.6 Hz, 4H), 3.43 (d, J = 9.9 Hz, 2H), 3.31 - 3.12 (m, 4H), 2.75 - 2.65 (m, 1H), 2.56 - 2.53 (m, 1H), 2.48 - 2.42 (m, 1H), 2.31 - 2.18 (m, 1H), 2.09 - 2.01 (m, 1H), 1.94 (d, J = 12.9 Hz, 2H), 1.85 (s, 1H), 1.73 - 1.58 (m, 4H). LCMS (ESI) calcd for C₃₈H₄₂F₃N₆O₄⁺ [M+H]⁺: 703.32; found, 703.4.

### Example 645: Preparation of N-(5-(4-(4-(2,4-dioxotetrahydropyrimidin-1(2H)-yl)benzyl)piperazin-1-yl)-1-((1s,4s)-4-(hydroxymethyl)cyclohexyl)-1H-benzo[d]imidazol-2-yl)-3-(trifluoromethyl)benzamide (GT-04982)

According to the method of step 2 of Scheme I-1, the target compound (GT-04982) was prepared (white solid, 44 mg, yield 68.3 %). ¹H NMR (400 MHz, DMSO-d6) δ 12.78 (s, 1H), 10.89 (s, 1H), 10.46 (s, 1H), 8.49 (d, J = 7.7 Hz, 1H), 8.44 (s, 1H), 7.90 (d, J = 7.4 Hz, 1H), 7.73 (d, J = 7.7 Hz, 1H), 7.65 (d, J = 8.4 Hz, 2H), 7.51 (d, J = 8.8 Hz, 1H), 7.46 (d, J = 8.4 Hz, 2H), 7.20 (s, 1H), 7.01 (d, J = 8.8 Hz, 1H), 4.70 (s, 1H), 4.39 (s, 2H), 3.83 (d, J = 6.8 Hz, 2H), 3.69 - 3.67 (m, 3H), 3.44 (d, J = 10.1 Hz, 2H), 3.28 - 3.12 (m, 5H), 2.73 (t, J = 6.5 Hz, 2H), 2.45 - 2.42 (m, 1H), 1.92 (s, 2H), 1.85 (s, 1H), 1.71 - 1.59 (m, 5H). LCMS (ESI) calcd for C₃₇H₄₁F₃N₇O₄⁺ [M+H]⁺: 704.32; found, 704.3.

### Example 646: Preparation of N-(5-(4-(3-(2,4-dioxotetrahydropyrimidin-1(2H)-yl)benzyl)piperazin-1-yl)-1-((1s,4s)-4-(hydroxymethyl)cyclohexyl)-1H-benzo[d]imidazol-2-yl)-3-(trifluoromethyl)benzamide (GT-04984)

According to the method of step 2 of Scheme I-1, the target compound (GT-04984) was prepared (white solid, 34 mg, yield 52.8 %). ¹H NMR (400 MHz, DMSO-d6) δ 12.79 (s, 1H), 11.04 (s, 1H), 10.46 (s, 1H), 8.49 (d, J = 7.8 Hz, 1H), 8.44 (s, 1H), 7.90 (d, J = 7.8 Hz, 1H), 7.74 (t, J = 7.7 Hz, 1H), 7.63 (s, 1H), 7.56 - 7.44 (m, 4H), 7.19 (s, 1H), 7.01 (d, J = 8.9 Hz, 1H), 4.70 (t, 1H), 4.41 (d, J = 4.1 Hz, 2H), 3.85 - 3.81 (m, 2H), 3.69 (t, J = 11.0 Hz, 4H), 3.45 (d, J = 10.1 Hz, 2H), 3.28 - 3.10 (m, 4H), 2.74 (t, J = 6.6 Hz, 2H), 2.49 - 2.39 (m, 2H), 1.94 (d, J = 12.3 Hz, 2H), 1.85 (s, 1H), 1.72 - 1.56 (m, 4H). LCMS (ESI) calcd for C₃₇H₄₁F₃N₇O₄⁺ [M+H]⁺: 704.32; found, 704.4.

### Example 647: Preparation of N-(5-(4-(3-((2,6-dioxopiperidin-3-yl)amino)benzyl)piperazin-1-yl)-1-((1s,4s)-4-(hydroxymethyl)cyclohexyl)-1H-benzo[d]imidazol-2-yl)-3-(trifluoromethyl)benzamide (GT-04985)

According to the method of step 2 of Scheme I-1, the target compound (GT-04985) was prepared (white solid, 29 mg, yield 44.2 %). ¹H NMR (400 MHz, DMSO-d6) δ 12.79 (s, 1H), 10.85 (s, 2H), 8.49 (d, J = 7.9 Hz, 1H), 8.44 (s, 1H), 7.90 (d, J = 8.0 Hz, 1H), 7.74 (t, J = 7.7 Hz, 1H), 7.51 (d, J = 8.9 Hz, 1H), 7.22 - 7.15 (m, 2H), 7.04 - 6.92 (m, 2H), 6.79 (d, J = 7.9 Hz, 2H), 4.75 - 4.63 (m, 1H), 4.39 (dd, J = 11.6, 4.6 Hz, 1H), 4.25 (s, 2H), 3.67 (s, 2H), 3.42 (s, 3H), 3.18 (d, J = 8.5 Hz, 4H), 2.89 - 2.71 (m, 1H), 2.63 (t, J = 17.3 Hz, 1H), 2.47 - 2.39 (m, 2H), 2.23 - 2.14 (m, 1H), 1.94 (d, J = 12.6 Hz, 3H), 1.85 (s, 1H), 1.71 - 1.58 (m, 4H), 1.31 - 1.20 (m, 1H). LCMS (ESI) calcd for C₃₈H₄₃F₃N₇O₄⁺ [M+H]⁺: 718.33; found, 718.3.

### Example 648: Preparation of N-(5-(4-((5-(2,4-dioxotetrahydropyrimidin-1(2H)-yl)pyridin-2-yl)methyl)piperazin-1-yl)-1-((1s,4s)-4-(hydroxymethyl)cyclohexyl)-1H-benzo[d]imidazol-2-yl)-3-(trifluoromethyl)benzamide (GT-04986)

According to the method of step 2 of Scheme I-1, the target compound (GT-04986) was prepared (white solid, 40 mg, yield 62.0 %). ¹H NMR (400 MHz, DMSO-d6) δ 12.84 (s, 1H), 10.92 (s, 1H), 10.60 (s, 1H), 9.18 (s, 1H), 8.49 (d, J = 7.9 Hz, 1H), 8.44 (s, 1H), 7.95 - 7.87 (m, 2H), 7.75 (t, J = 7.9 Hz, 2H), 7.52 (d, J = 8.9 Hz, 1H), 7.22 - 7.19 (m, 1H), 7.02 (d, J = 8.9 Hz, 1H), 4.70 (t, 1H), 4.57 (s, 1H), 3.89 (t, J = 6.8 Hz, 3H), 3.68 (d, J = 7.4 Hz, 2H), 3.45 (s, 4H), 3.34 - 3.25 (m, 4H), 2.77 (t, J = 6.6 Hz, 1H), 2.48 - 2.42 (m, 2H), 1.94 (d, J = 13.1 Hz, 2H), 1.85 (s, 1H), 1.73 - 1.59 (m, 5H). LCMS (ESI) calcd for C₃₆H₄₀F₃N₈O₄ ⁺ [M+H]⁺: 705.31; found, 705.3.

### Example 649: Preparation of N-(5-(4-((6-(2,4-dioxotetrahydropyrimidin-1(2H)-yl)pyridin-3-yl)methyl)piperazin-1-yl)-1-((1s,4s)-4-(hydroxymethyl)cyclohexyl)-1H-benzo[d]imidazol-2-yl)-3-(trifluoromethyl)benzamide (GT-04987)

According to the method of step 2 of Scheme I-1, the target compound (GT-04987) was prepared (white solid, 39 mg, yield 60.5 %). ¹H NMR (400 MHz, DMSO-d6) δ 12.81 (s, 1H), 11.32 (s, 1H), 10.63 (s, 1H), 8.63 (d, J = 2.0 Hz, 1H), 8.49 (d, J = 7.8 Hz, 1H), 8.44 (s, 1H), 8.14 - 8.07 (m, 1H), 7.89 (t, J = 7.3 Hz, 2H), 7.74 (t, J = 7.8 Hz, 1H), 7.52 (d, J = 8.9 Hz, 1H), 7.22 - 7.19 (m, 1H), 7.01 (dd, J = 8.9, 2.2 Hz, 1H), 4.69 (d, J = 11.9 Hz, 1H), 4.43 (s, 2H), 4.11 (t, J = 6.6 Hz, 2H), 3.71 (d, J = 11.1 Hz, 2H), 3.68 (d, J = 7.5 Hz, 2H), 3.45 (d, J = 10.3 Hz, 2H), 3.31 - 3.17 (m, 5H), 2.71 (t, J = 6.6 Hz, 2H), 2.43 (d, J = 12.7 Hz, 1H), 1.94 (d, J = 12.6 Hz, 2H), 1.85 (s, 1H), 1.68 - 1.60 (m, 4H). LCMS (ESI) calcd for C₃₆H₄₀F₃N₈O₄ ⁺ [M+H]⁺: 705.31; found, 705.3.

### Example 650: Preparation of N-(5-(4-(1-(4-(2,6-dioxopiperidin-3-yl)benzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide (GT-05020)

According to the method of step 2 of Scheme I-1, the target compound (GT-05020) was prepared (white solid, 24 mg, yield 50.3 %). ¹H NMR (400 MHz, DMSO-d6) δ 12.78 (s, 1H), 11.22 (s, 1H), 10.88 (s, 2H), 8.49 (d, J = 7.7 Hz, 1H), 8.44 (s, 1H), 7.90 (d, J = 7.9 Hz, 1H), 7.74 (t, J = 7.8 Hz, 1H), 7.56 (d, J = 8.1 Hz, 1H), 7.52 (d, J = 8.8 Hz, 1H), 7.34 (d, J = 8.2 Hz, 1H), 7.21 (d, J = 2.1 Hz, 1H), 7.05 - 7.00 (m, 1H), 4.70 (t, 1H), 4.29 (s, 1H), 3.94 (dd, J = 11.7, 4.8 Hz, 1H), 3.73 (d, J = 8.7 Hz, 2H), 3.68 (d, J = 7.4 Hz, 3H), 3.59 (d, J = 13.1 Hz, 4H), 3.42 - 3.30 (m, 2H), 3.21 (s, 4H), 3.02 - 2.89 (m, 2H), 2.71 - 2.65 (m, 1H), 2.58 - 2.53 (m, 2H), 2.47 - 2.33 (m, 3H), 2.27 - 2.17 (m, 2H), 2.09 - 2.00 (m, 1H), 1.94 (d, J = 12.4 Hz, 2H), 1.85 (s, 1H), 1.71 - 1.57 (m, 4H). LCMS (ESI) calcd for C₄₃H₅₁F₃N₇O₄⁺ [M+H]⁺: 786.39; found, 786.4.

### Example 651: Preparation of N-(5-(4-(1-(4-(2,4-dioxotetrahydropyrimidin-1(2H)-yl)benzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide (GT-05021)

According to the method of step 2 of Scheme I-1, the target compound (GT-05021) was prepared (white solid, 19 mg, yield 39.8 %). ¹H NMR (400 MHz, DMSO-d6) δ 12.78 (s, 1H), 11.25 (s, 1H), 10.93 (s, 1H), 10.45 (s, 1H), 8.49 (d, J = 7.7 Hz, 1H), 8.44 (s, 1H), 7.90 (d, J = 7.9 Hz, 1H), 7.74 (t, J = 7.6 Hz, 1H), 7.61 (d, J = 8.5 Hz, 1H), 7.52 (d, J = 9.0 Hz, 1H), 7.44 (d, J = 8.5 Hz, 1H), 7.23 - 7.19 (m, 1H), 7.03 (d, J = 9.0 Hz, 1H), 4.70 (s, 1H), 4.30 (s, 1H), 3.83 (t, J = 6.6 Hz, 2H), 3.73 (d, J = 7.7 Hz, 2H), 3.68 (d, J = 7.4 Hz, 2H), 3.61 (s, 2H), 3.55 - 3.49 (m, 3H), 3.21 (s, 5H), 3.04 - 2.92 (m, 2H), 2.73 (t, J = 6.6 Hz, 2H), 2.47 - 2.32 (m, 4H), 2.27 - 2.13 (m, 2H), 1.94 (d, J = 12.6 Hz, 2H), 1.85 (s, 1H), 1.72 - 1.59 (m, 4H). LCMS (ESI) calcd for C₄₂H₅₀F₃N₈O₄⁺ [M+H]⁺: 787.39; found, 787.4.

### Example 652: Preparation of N-(5-(4-(1-(3-(2,4-dioxotetrahydropyrimidin-1(2H)-yl)benzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide (GT-05022)

According to the method of step 2 of Scheme I-1, the target compound (GT-05022) was prepared (white solid, 19 mg, yield 39.8 %). ¹H NMR (400 MHz, DMSO-d6) δ 12.78 (s, 1H), 11.24 (s, 1H), 10.87 (s, 1H), 10.47 (s, 1H), 8.49 (d, J = 7.7 Hz, 1H), 8.44 (s, 1H), 7.90 (d, J = 7.7 Hz, 1H), 7.74 (t, J = 7.9 Hz, 1H), 7.58 (s, 1H), 7.55 - 7.46 (m, 3H), 7.46 - 7.41 (m, 1H), 7.21 (s, 1H), 7.03 (d, J = 8.7 Hz, 1H), 4.70 (s, 1H), 4.32 (s, 1H), 3.87 (t, J = 6.6 Hz, 2H), 3.73 (d, J = 8.6 Hz, 2H), 3.68 (d, J = 7.4 Hz, 2H), 3.65 - 3.60 (m, 2H), 3.56 - 3.52 (m, 3H), 3.25 - 3.15 (m, 4H), 3.03 - 2.90 (m, 2H), 2.74 (t, J = 6.6 Hz, 2H), 2.48 - 2.31 (m, 5H), 2.25 - 2.10 (m, 2H), 1.94 (d, J = 12.6 Hz, 2H), 1.85 (s, 1H), 1.72 - 1.59 (m, 4H). LCMS (ESI) calcd for C₄₂H₅₀F₃N₈O₄ ⁺ [M+H]⁺: 787.39; found, 787.4.

### Example 653: Preparation of N-(5-(4-(1-(3-((2,6-dioxopiperidin-3-yl)amino)benzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide (GT-05023)

According to the method of step 2 of Scheme I-1, the target compound (GT-05023) was prepared (white solid, 12 mg, yield 24.7 %). ¹H NMR (400 MHz, DMSO-d6) δ 12.72 (s, 1H), 10.84 (s, 1H), 10.54 (s, 1H), 8.49 (d, J = 7.5 Hz, 1H), 8.44 (s, 1H), 7.90 (d, J = 7.8 Hz, 1H), 7.74 (t, J = 7.8 Hz, 1H), 7.52 (d, J = 8.7 Hz, 1H), 7.23 - 7.15 (m, 2H), 7.03 (d, J = 7.5 Hz, 1H), 6.89 (s, 1H), 6.77 (dd, J = 17.7, 8.0 Hz, 2H), 4.70 (t, 1H), 4.41 - 4.33 (m, 1H), 4.17 (s, 1H), 3.72 (s, 2H), 3.68 (d, J = 7.4 Hz, 2H), 3.61 (s, 2H), 3.20 - 3.12 (m, 4H), 2.97 (s, 3H), 2.82 - 2.75 (m, 1H), 2.70 - 2.58 (m, 2H), 2.46 - 2.34 (m, 4H), 2.17 (d, J = 13.0 Hz, 3H), 1.94 (d, J = 11.9 Hz, 3H), 1.88 - 1.83 (m, 1H), 1.72 - 1.57 (m, 5H). LCMS (ESI) calcd for C₄₃H₅₂F₃N₈O₄ ⁺ [M+H]⁺: 801.41; found, 801.5.

### Example 654: Preparation of N-(5-(4-(1-((5-(2,4-dioxotetrahydropyrimidin-1(2H)-yl)pyridin-2-yl)methyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide (GT-05024)

According to the method of step 2 of Scheme I-1, the target compound (GT-05024) was prepared (white solid, 22 mg, yield 46.0 %). ¹H NMR (400 MHz, DMSO-d6) δ 12.79 (s, 1H), 11.42 (d, J = 38.5 Hz, 1H), 10.76 (s, 1H), 10.59 (s, 1H), 9.08 - 8.66 (m, 1H), 8.49 (d, J = 7.7 Hz, 1H), 8.44 (s, 1H), 7.91 - 7.89 (m, 1H), 7.83 - 7.60 (m, 2H), 7.52 (d, J = 9.0 Hz, 1H), 7.22 (d, J = 2.0 Hz, 1H), 7.03 (dd, J = 9.6, 2.7 Hz, 1H), 4.79 - 4.62 (m, 1H), 4.47 (s, 1H), 3.88 (d, J = 6.6 Hz, 1H), 3.73 (d, J = 7.6 Hz, 2H), 3.70 - 3.65 (m, 3H), 3.64 (s, 3H), 3.58 - 3.53 (m, 2H), 3.42 - 3.30 (m, 4H), 3.23 (d, J = 7.9 Hz, 4H), 3.13 (s, 1H), 2.92 (d, J = 10.2 Hz, 1H), 2.75 (t, J = 6.7 Hz, 1H), 2.48 - 2.29 (m, 3H), 2.23 (d, J = 11.3 Hz, 1H), 1.98 - 1.80 (m, 3H), 1.74 - 1.56 (m, 4H). LCMS (ESI) calcd for C₄₁H₄₉F₃N₉O₄ ⁺ [M+H]⁺: 788.39; found, 788.4.

### Example 655: Preparation of N-(5-(4-(1-((6-(2,4-dioxotetrahydropyrimidin-1(2H)-yl)pyridin-3-yl)methyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide (GT-05025)

According to the method of step 2 of Scheme I-1, the target compound (GT-05025) was prepared (white solid, 24 mg, yield 50.2 %). ¹H NMR (400 MHz, DMSO-d6) δ 12.79 (s, 1H), 11.16 (d, J = 56.3 Hz, 1H), 10.62 (s, 1H), 8.58 (s, 1H), 8.49 (d, J = 7.7 Hz, 1H), 8.44 (s, 1H), 8.05 (d, J = 8.7 Hz, 1H), 7.89 (t, J = 8.0 Hz, 2H), 7.74 (t, J = 7.8 Hz, 1H), 7.52 (d, J = 8.7 Hz, 1H), 7.21 (s, 1H), 7.03 (d, J = 8.9 Hz, 1H), 4.70 (t, 1H), 4.35 (s, 1H), 4.10 (t, J = 6.6 Hz, 2H), 3.73 (d, J = 8.4 Hz, 2H), 3.68 (d, J = 7.5 Hz, 2H), 3.65 - 3.58 (m, 4H), 3.47 - 3.31 (m, 5H), 3.21 (s, 4H), 2.97 (s, 1H), 2.71 (t, J = 6.5 Hz, 2H), 2.47 - 2.35 (m, 3H), 2.18 (d, J = 11.3 Hz, 1H), 1.94 (d, J = 11.7 Hz, 2H), 1.85 (s, 1H), 1.72 - 1.59 (m, 4H). LCMS (ESI) calcd for C₄₁H₄₉F₃N₉O₄ ⁺ [M+H]⁺: 788.39; found, 788.4.

### Example 656: Preparation of 5-((1R,4R)-2-oxa-5-azabicyclo[2.2.1]heptan-5-yl)-N-(3-(difluoromethyl)-1-(1-(4-(2,6-dioxopiperidin-3-yl)benzyl)piperidin-4-yl)-1H-pyrazol-4-yl)pyrazolo[1,5-a]pyrimidine-3-carboxamide (GT-05050)

According to the method of step 2 of Scheme I-1, the target compound (GT-05050) was prepared (white solid, 21 mg, yield 29.9 %). ¹H NMR (400 MHz, DMSO-d6) δ 10.88 (s, 1H), 10.43 (s, 1H), 9.53 (d, J = 6.4 Hz, 1H), 8.79 (d, J = 7.7 Hz, 1H), 8.43 (d, J = 3.1 Hz, 1H), 8.26 (d, J = 5.5 Hz, 1H), 7.57 (d, J = 7.8 Hz, 2H), 7.36 (d, J = 7.9 Hz, 2H), 4.77 (d, J = 21.4 Hz, 1H), 4.57 (s, 1H), 4.34 (d, J = 4.7 Hz, 2H), 3.94 (dd, J = 11.6, 4.7 Hz, 1H), 3.81 (s, 2H), 3.70 - 3.54 (m, 2H), 3.53 - 3.46 (m, 2H), 3.18 - 3.08 (m, 2H), 2.72 - 2.66 (m, 1H), 2.57 - 2.52 (m, 1H), 2.35 - 2.19 (m, 6H), 2.06 - 1.94 (m, 3H). LCMS (ESI) calcd for C₃₃H₃₆F₂N₉O₄ ⁺ [M+H]⁺: 660.29; found, 660.3

### Example 657: Preparation of 5-((1R,4R)-2-oxa-5-azabicyclo[2.2.1]heptan-5-yl)-N-(3-(difluoromethyl)-1-(1-(3-(2,4-dioxotetrahydropyrimidin-1(2H)-yl)benzyl)piperidin-4-yl)-1H-pyrazol-4-yl)pyrazolo[1,5-a]pyrimidine-3-carboxamide (GT-05051)

According to the method of step 2 of Scheme I-1, the target compound (GT-05051) was prepared (white solid, 23 mg, yield 32.7 %). ¹H NMR (400 MHz, DMSO-d6) δ 10.45 (d, J = 6.6 Hz, 2H), 9.52 (s, 1H), 8.79 (d, J = 7.6 Hz, 1H), 8.43 (d, J = 3.3 Hz, 1H), 8.26 (d, J = 5.5 Hz, 1H), 7.59 (s, 1H), 7.50 - 7.42 (m, 3H), 4.77 (d, J = 21.5 Hz, 1H), 4.57 (s, 1H), 4.36 (s, 2H), 3.89 - 3.79 (m, 4H), 3.70 - 3.55 (m, 2H), 3.54 - 3.45 (m, 2H), 3.12 (d, J = 11.4 Hz, 2H), 2.74 (t, J = 6.5 Hz, 2H), 2.30 (d, J = 22.0 Hz, 4H), 1.98 (t, J = 17.1 Hz, 2H). LCMS (ESI) calcd for C₃₂H₃₅F₂N₁₀O₄ ⁺ [M+H]⁺: 661.28; found, 661.3.

### Example 658: Preparation of 5-((1R,4R)-2-oxa-5-azabicyclo[2.2.1]heptan-5-yl)-N-(3-(difluoromethyl)-1-(1-(3-((2,6-dioxopiperidin-3-yl)amino)benzyl)piperidin-4-yl)-1H-pyrazol-4-yl)pyrazolo[1,5-a]pyrimidine-3-carboxamide (GT-05052)

According to the method of step 2 of Scheme I-1, the target compound (GT-05052) was prepared (gray solid, 25 mg, yield 35.8 %). ¹H NMR (400 MHz, DMSO-d6) δ 10.46 (d, 1H), 9.52 (d, J = 6.3 Hz, 1H), 8.79 (d, J = 7.7 Hz, 1H), 8.43 (s, 1H), 8.26 (d, J = 5.5 Hz, 1H), 7.59 - 7.47 (m, 1H), 7.29 (d, J = 11.1 Hz, 1H), 7.18 - 7.13 (m, 1H), 6.96 - 6.84 (m, 1H), 6.77 (t, J = 7.5 Hz, 1H), 4.77 (d, J = 21.7 Hz, 1H), 4.55 (s, 1H), 4.35 (s, 2H), 4.20 (s, 1H), 3.90 - 3.75 (m, 2H), 3.66 - 3.56 (m, 2H), 3.46 (s, 3H), 3.11 (s, 2H), 2.67 - 2.54 (m, 2H), 2.39 - 2.23 (m, 5H), 2.17 - 1.87 (m, 4H). LCMS (ESI) calcd for C₃₃H₃₇F₂N₁₀O₄⁺ [M+H]⁺: 675.30; found, 675.3.

### Example 659: Preparation of 5-((1R,4R)-2-oxa-5-azabicyclo[2.2.1]heptan-5-yl)-N-(3-(difluoromethyl)-1-(1-((5-(2,4-dioxotetrahydropyrimidin-1(2H)-yl)pyridin-2-yl)methyl)piperidin-4-yl)-1H-pyrazol-4-yl)pyrazolo[1,5-a]pyrimidine-3-carboxamide (GT-05053)

According to the method of step 2 of Scheme I-1, the target compound (GT-05053) was prepared (white solid, 12 mg, yield 17.0 %). ¹H NMR (400 MHz, DMSO-d6) δ 10.85 - 10.48 (m, 2H), 9.53 (d, J = 6.1 Hz, 1H), 8.79 (d, J = 7.7 Hz, 1H), 8.72 (d, J = 2.3 Hz, 1H), 8.45 (d, J = 12.7 Hz, 1H), 8.27 (t, J = 7.6 Hz, 1H), 7.91 (dd, J = 8.3, 2.5 Hz, 1H), 7.71 (d, J = 8.4 Hz, 1H), 5.18 (d, J = 76.4 Hz, 1H), 4.59 (s, 1H), 4.52 (s, 2H), 3.89 (t, J = 6.6 Hz, 2H), 3.82 (s, 2H), 3.66 - 3.58 (m, 3H), 3.44 (s, 2H), 3.27 (s, 2H), 2.76 (t, J = 6.6 Hz, 2H), 2.38 - 2.27 (m, 4H), 2.05 - 1.92 (m, 2H). LCMS (ESI) calcd for C₃₁H₃₄F₂N₁₁O₄⁺ [M+H]⁺: 662.28; found, 662.3.

### Example 660: Preparation of 5-((1R,4R)-2-oxa-5-azabicyclo[2.2.1]heptan-5-yl)-N-(3-(difluoromethyl)-1-(1-((6-(2,4-dioxotetrahydropyrimidin-1(2H)-yl)pyridin-3-yl)methyl)piperidin-4-yl)-1H-pyrazol-4-yl)pyrazolo[1,5-a]pyrimidine-3-carboxamide (GT-05054)

According to the method of step 2 of Scheme I-1, the target compound (GT-05054) was prepared (white solid, 20 mg, yield 28.4 %). ¹H NMR (400 MHz, DMSO-d6) δ 10.98 (s, 1H), 10.62 (s, 1H), 9.52 (d, J = 5.9 Hz, 1H), 8.79 (d, J = 7.7 Hz, 1H), 8.63 - 8.58 (m, 1H), 8.43 (d, J = 3.2 Hz, 1H), 8.30 - 8.25 (m, 1H), 8.11 - 8.04 (m, 1H), 7.88 (d, J = 8.7 Hz, 1H), 5.31 - 5.04 (m, 1H), 4.77 (d, J = 19.6 Hz, 1H), 4.57 (s, 1H), 4.38 (d, J = 3.6 Hz, 2H), 4.10 (t, J = 6.5 Hz, 2H), 3.80 (s, 2H), 3.53 - 3.45 (m, 3H), 3.22 - 2.98 (m, 3H), 2.70 (t, J = 13.4, 6.8 Hz, 2H), 2.39 - 2.30 (m, 2H), 2.30 - 2.24 (m, 2H), 2.05 - 1.92 (m, 2H). LCMS (ESI) calcd for C₃₁H₃₄F₂N₁₁O₄⁺ [M+H]⁺: 662.28; found, 662.3.

### Example 661: Preparation of 6-(5-cyano-1H-pyrrolo[2,3-b]pyridin-1-yl)-N-(1-((2-(2,4-dioxotetrahydropyrimidin-1(2H)-yl)-5-fluoropyridin-4-yl)methyl)piperidin-4-yl)-4-(isopropylamino)nicotinamide (GT-07408)

According to the method of step 2 of Scheme I-1, the target compound (GT-07408) was prepared (white solid, 41 mg, yield 67 %). ¹H NMR (400 MHz, DMSO-d6) δ 11.31 (s, 1H), 10.72 (s, 1H), 8.97 (d, *J =* 7.2 Hz, 1H), 8.88 (d, *J =* 1.9 Hz, 1H), 8.80 - 8.73 (m, 2H), 8.61 (s, 1H), 8.57 (d, *J =* 3.9 Hz, 1H), 8.21 (t, *J =* 5.8 Hz, 1H), 8.04 (s, 1H), 7.00 (t, *J =* 4.6 Hz, 1H), 4.50 (d, *J =* 25.7 Hz, 2H), 4.20 (s, 2H), 3.91 (s, 2H), 3.56 (d, *J* = 11.3 Hz, 2H), 3.33 (d, *J =* 64.0 Hz, 2H), 2.79 (t, *J =* 6.2 Hz, 2H), 2.25 - 1.99 (m, 4H), 1.35 (d, *J =* 6.3 Hz, 6H). LCMS (ESI) calcd for C₃₂H₃₄FN₁₀O₃⁺ [M+H]⁺: 625.28; found, 625.3.

### Example 662: Preparation of 6-(5-cyano-1H-pyrrolo[2,3-b]pyridin-1-yl)-N-(1-((2-(2,4-dioxotetrahydropyrimidin-1(2H)-yl)pyridin-4-yl)methyl)piperidin-4-yl)-4-(isopropylamino)nicotinamide (GT-07409)

According to the method of step 2 of Scheme I-1, the target compound (GT-07409) was prepared (white solid, 36 mg, yield 61 %). ¹H NMR (400 MHz, DMSO-d6) δ 10.68 (d, *J =* 5.9 Hz, 1H), 8.88 (d, *J =* 2.0 Hz, 1H), 8.76 (d, *J =* 1.8 Hz, 2H), 8.57 (d, *J =* 3.8 Hz, 2H), 8.03 (d, *J =* 3.4 Hz, 2H), 7.69 (d, *J =* 3.9 Hz, 1H), 7.00 (d, *J* = 3.9 Hz, 1H), 4.49 (d, *J* = 5.1 Hz, 1H), 4.42 (d, *J* = 4.5 Hz, 2H), 4.16 (t, *J* = 6.6 Hz, 3H), 4.07 (s, 1H), 3.92 (s, 1H), 3.46 (d, *J =* 11.4 Hz, 2H), 2.78 (t, *J =* 6.5 Hz, 2H), 2.10 (s, 4H), 1.36 (s, 3H), 1.34 (s, 3H). LCMS (ESI) calcd for C₃₂H₃₅N₁₀O₃⁺ [M+H]⁺: 607.23; found, 607.4.

### Example 663: Preparation of 6-(5-cyano-1H-pyrrolo[2,3-b]pyridin-1-yl)-N-(1-((5-(2,4-dioxotetrahydropyrimidin-1(2H)-yl)pyridin-3-yl)methyl)piperidin-4-yl)-4-(isopropylamino)nicotinamide (GT-07410)

According to the method of step 2 of Scheme I-1, the target compound (GT-07410) was prepared (white solid, 41 mg, yield 69 %). ¹H NMR (400 MHz, DMSO-d6) δ 10.69 (s, 1H), 8.83 (d, *J =* 2.1 Hz, 3H), 8.71 (s, 2H), 8.68 (s, 1H), 8.51 (d, *J =* 3.8 Hz, 1H), 8.35 (s, 1H), 8.00 (s, 1H), 6.93 (d, *J =* 3.9 Hz, 1H), 4.43 (d, *J* = 3.8 Hz, 3H), 3.98 (t, *J =* 6.6 Hz, 3H), 3.85 (s, 1H), 3.45 (d, *J =* 11.7 Hz, 2H), 2.78 (t, *J =* 6.5 Hz, 2H), 2.04 (s, 4H), 1.30 (d, *J =* 6.3 Hz, 7H). LCMS (ESI) calcd for C₃₂H₃₅N₁₀O₃⁺ [M+H]⁺: 607.29; found, 607.3.

### Example 664: Preparation of 6-(5-cyano-1H-pyrrolo[2,3-b]pyridin-1-yl)-N-(1-((5-(2,6-dioxopiperidin-3-yl)pyridin-2-yl)methyl)piperidin-4-yl)-4-(isopropylamino)nicotinamide (GT-07411)

According to the method of step 2 of Scheme I-1, the target compound (GT-07411) was prepared (white solid, 32 mg, yield 55 %). ¹H NMR (400 MHz, DMSO-d6) δ 11.02 (s, 1H), 8.89 (d, *J =* 2.0 Hz, 2H), 8.77 (d, *J =* 2.0 Hz, 1H), 8.65 (d, *J =* 2.0 Hz, 1H), 8.58 (d, *J =* 3.9 Hz, 1H), 8.07 (s, 1H), 7.91 (d, *J =* 2.2 Hz, 1H), 7.78 (d, *J* = 8.0 Hz, 1H), 7.00 (d, *J* = 3.9 Hz, 1H), 4.53 (s, 2H), 4.13 (d, *J* = 4.8 Hz, 3H), 3.91 (s, 1H), 3.54 (s, 2H), 3.33 (d, *J* = 30.9 Hz, 2H), 2.87 - 2.74 (m, 1H), 2.69 - 2.61 (m, 1H), 2.39 (m, *J* = 12.9, 4.4 Hz, 1H), 2.11 (d, *J =* 4.7 Hz, 4H), 1.36 (s, 3H), 1.35 (s, 3H). LCMS (ESI) calcd for C₃₃H₃₆N₉O₃⁺ [M+H]⁺: 606.29; found, 606.4.

### Example 665: Preparation of 6-(5-cyano-1H-pyrrolo[2,3-b]pyridin-1-yl)-N-(1-((2-(2,6-dioxopiperidin-3-yl)pyridin-4-yl)methyl)piperidin-4-yl)-4-(isopropylamino)nicotinamide (GT-07412)

According to the method of step 2 of Scheme I-1, the target compound (GT-07412) was prepared (white solid, 31 mg, yield 55 %). ¹H NMR (400 MHz, DMSO-d6) δ 9.10 (d, *J* = 28.3 Hz, 1H), 8.89 (d, *J =* 2.0 Hz, 3H), 8.79 - 8.73 (m, 2H), 8.58 (d, *J =* 3.9 Hz, 1H), 8.06 (s, 1H), 7.00 (d, *J =* 3.9 Hz, 1H), 4.44 (s, 1H), 4.28 (s, 3H), 4.12 (m, *J* = 10.5, 3.9 Hz, 2H), 3.90 (s, 1H), 3.42 (m, *J* = 34.4, 11.8 Hz, 2H), 3.18 (m, *J* = 14.7, 7.3, 4.2 Hz, 1H), 3.05 (d, *J* = 10.1 Hz, 1H), 2.11 - 2.00 (m, 2H), 1.92 - 1.85 (m, 1H), 1.42 - 1.30 (m, 9H). LCMS (ESI) calcd for C₃₃H₃₆N₉O₃⁺ [M+H]⁺: 606.29; found, 606.3.

### Example 666: Preparation of 6-(5-cyano-1H-pyrrolo[2,3-b]pyridin-1-yl)-N-(1-((5-(2,6-dioxopiperidin-3-yl)pyridin-3-yl)methyl)piperidin-4-yl)-4-(isopropylamino)nicotinamide (GT-07413)

According to the method of step 2 of Scheme I-1, the target compound (GT-07413) was prepared (white solid, 31 mg, yield 55 %). ¹H NMR (400 MHz, DMSO-d6) δ 11.13 (s, 1H), 9.05 (s, 1H), 8.91 - 8.85 (m, 3H), 8.75 (d, *J* = 8.8 Hz, 2H), 8.58 (s, 2H), 8.06 (s, 1H), 6.99 (d, *J* = 3.8 Hz, 1H), 4.53 (s, 2H), 4.12 - 4.02 (m, 2H), 3.90 (s, 2H), 3.57 - 3.44 (m, 2H), 2.91 - 2.78 (m, 1H), 2.71 (dd, *J* = 12.2, 8.7 Hz, 1H), 2.43 (dt, *J* = 11.6, 8.0 Hz, 1H), 2.28 - 2.20 (m, 1H), 2.09 (s, 4H), 1.35 (d, *J* = 6.3 Hz, 7H). LCMS (ESI) calcd for C₃₃H₃₆N₉O₃⁺ [M+H]⁺: 606.29; found, 606.3.

### Example 667: Preparation of N-(2-(1-((2-(2,4-dioxotetrahydropyrimidin-1(2H)-yl)-5-fluoropyridin-4-yl)methyl)piperidin-4-yl)-6-methoxy-2H-indazol-5-yl)-3-(trifluoromethyl)benzamide (GT-07414)

According to the method of step 2 of Scheme I-1, the target compound (GT-07414) was prepared (white solid, 35 mg, yield 59 %). ¹H NMR (400 MHz, DMSO-d6) δ 10.67 (s, 1H), 9.80 (s, 1H), 8.58 (s, 1H), 8.31 (d, *J* = 7.6 Hz, 3H), 8.17 (d, *J* = 5.3 Hz, 1H), 8.04 (s, 1H), 7.97 (d, *J* = 7.8 Hz, 1H), 7.79 (t, *J* = 7.8 Hz, 1H), 7.11 (s, 1H), 4.87 (s, 1H), 4.50 (s, 2H), 4.06 (d, *J =* 6.6 Hz, 2H), 3.87 (s, 3H), 3.64 (d, *J =* 11.7 Hz, 2H), 2.72 (d, *J* = 6.4 Hz, 2H), 2.35 (d, *J* = 11.9 Hz, 2H). LCMS (ESI) calcd for C₃₁H₃₀F₄N₇O₄⁺ [M+H]⁺: 640.23; found, 640.3.

### Example 668: Preparation of N-(2-(1-((2-(2,4-dioxotetrahydropyrimidin-1(2H)-yl)pyridin-4-yl)methyl)piperidin-4-yl)-6-methoxy-2H-indazol-5-yl)-3-(trifluoromethyl)benzamide (GT-07415)

According to the method of step 2 of Scheme I-1, the target compound (GT-07415) was prepared (white solid, 32 mg, yield 55 %). ¹H NMR (400 MHz, DMSO-d6) δ 10.64 (s, 1H), 9.80 (s, 1H), 8.53 (d, *J =* 5.1 Hz, 1H), 8.31 (d, *J* = 11.4 Hz, 3H), 8.04 (s, 1H), 7.99 (d, *J* = 9.2 Hz, 2H), 7.78 (s, 1H), 7.65 (d, *J* = 5.1 Hz, 1H), 7.12 (s, 1H), 4.74 (t, *J* = 11.6 Hz, 1H), 4.44 (s, 2H), 4.11 (s, 2H), 3.87 (s, 3H), 3.23 (d, *J* = 9.8 Hz, 2H), 2.72 (t, *J =* 6.5 Hz, 2H), 2.57 (s, 1H), 2.34 (d, *J =* 12.5 Hz, 2H). LCMS (ESI) calcd for C₃₁H₃₁F₃N₇O₄⁺ [M+H]⁺: 622.24; found, 622.3.

### Example 669: Preparation of N-(2-(1-((5-(2,4-dioxotetrahydropyrimidin-1(2H)-yl)pyridin-3-yl)methyl)piperidin-4-yl)-6-methoxy-2H-indazol-5-yl)-3-(trifluoromethyl)benzamide (GT-07416)

According to the method of step 2 of Scheme I-1, the target compound (GT-07416) was prepared (white solid, 36 mg, yield 61 %). ¹H NMR (400 MHz, DMSO-d6) δ 10.74 (s, 1H), 9.80 (s, 1H), 8.92 (s, 1H), 8.82 (s, 1H), 8.53 (s, 1H), 8.33 (s, 1H), 8.29 (d, *J* = 5.7 Hz, 2H), 8.04 (s, 1H), 7.97 (d, *J* = 7.6 Hz, 1H), 7.78 (s, 1H), 7.13 (s, 1H), 4.54 (s, 2H), 4.01 (t, *J* = 6.5 Hz, 2H), 3.87 (s, 3H), 3.60 (d, *J* = 11.6 Hz, 2H), 3.26 (s, 2H), 2.79 (t, *J* = 6.5 Hz, 2H), 2.35 (d, *J* = 11.4 Hz, 2H). LCMS (ESI) calcd for C₃₁H₃₁F₃N₇O₄⁺ [M+H]⁺: 622.24; found, 622.3.

### Example 670: Preparation of N-(2-(1-((5-(2,6-dioxopiperidin-3-yl)pyridin-2-yl)methyl)piperidin-4-yl)-6-methoxy-2H-indazol-5-yl)-3-(trifluoromethyl)benzamide (GT-07417)

According to the method of step 2 of Scheme I-1, the target compound (GT-07417) was prepared (white solid, 29 mg, yield 50 %). ¹H NMR (400 MHz, DMSO-d6) δ 10.98 (s, 1H), 9.82 (s, 1H), 8.62 (d, *J =* 1.8 Hz, 1H), 8.36 (s, 1H), 8.33 - 8.23 (m, 2H), 8.04 (s, 1H), 7.96 (s, 1H), 7.89 (d, *J* = 2.1 Hz, 1H), 7.84 - 7.75 (m, 2H), 7.12 (s, 1H), 4.55 (s, 3H), 4.08 (m, *J* = 12.6, 4.8 Hz, 1H), 3.58 (d, *J* = 11.8 Hz, 2H), 3.36 (s, 2H), 2.75 (s, 1H), 2.64 - 2.59 (m, 1H), 2.55 (s, 2H), 2.38 (d, *J* = 13.5 Hz, 3H), 2.14 - 2.02 (m, 1H). LCMS (ESI) calcd for C₃₂H₃₂F₃N₆O₄⁺ [M+H]⁺: 621.24; found, 621.3.

### Example 671: Preparation of N-(2-(1-((2-(2,6-dioxopiperidin-3-yl)pyridin-4-yl)methyl)piperidin-4-yl)-6-methoxy-2H-indazol-5-yl)-3-(trifluoromethyl)benzamide (GT-07418)

According to the method of step 2 of Scheme I-1, the target compound (GT-07418) was prepared (white solid, 29 mg, yield 50 %). ¹H NMR (400 MHz, DMSO-d6) δ 9.81 (s, 1H), 9.33 (dd, *J* = 68.6, 9.6 Hz, 1H), 8.34 (s, 1H), 8.30 (s, 2H), 8.04 (s, 1H), 7.95 (d, *J =* 10.3 Hz, 2H), 7.79 (s, 1H), 7.12 (s, 1H), 4.82 - 4.73 (m, 1H), 3.87 (d, *J* = 2.5 Hz, 3H), 3.64 - 3.51 (m, 1H), 3.43 (d, *J* = 12.7 Hz, 1H), 3.25 (s, 1H), 3.11 (m, *J =* 7.4, 4.2 Hz, 1H), 2.59 (d, *J =* 17.3 Hz, 1H), 2.31 (s, 3H), 1.31 (d, *J =* 6.6 Hz, 2H), 1.28 (s, 1H), 1.27 (s, 1H). LCMS (ESI) calcd for C₃₂H₃₂F₃N₆O₄⁺ [M+H]⁺: 621.24; found, 621.3.

### Example 672: Preparation of N-(2-(1-((5-(2,6-dioxopiperidin-3-yl)pyridin-3-yl)methyl)piperidin-4-yl)-6-methoxy-2H-indazol-5-yl)-3-(trifluoromethyl)benzamide (GT-07419)

According to the method of step 2 of Scheme I-1, the target compound (GT-07419) was prepared (white solid, 36 mg, yield 62 %). ¹H NMR (400 MHz, DMSO-d6) δ 11.10 (s, 1H), 9.81 (s, 1H), 9.12 (s, 1H), 8.93 (s, 1H), 8.73 (s, 1H), 8.32 (d, *J* = 13.6 Hz, 3H), 8.00 (d, *J* = 30.4 Hz, 2H), 7.78 (s, 1H), 7.13 (s, 1H), 4.61 (s, 2H), 4.27 (m, *J* = 12.5, 4.6 Hz, 1H), 3.87 (s, 3H), 3.70 - 3.43 (m, 2H), 3.27 (s, 2H), 2.89 - 2.73 (m, 1H), 2.60 (m, *J =* 23.3, 10.6 Hz, 2H), 2.37 (m, *J =* 16.1, 7.9 Hz, 2H), 2.26 - 2.10 (m, 1H). LCMS (ESI) calcd for C₃₂H₃₂F₃N₆O₄⁺ [M+H]⁺: 621.24; found, 621.3.

### Example 673: Preparation of N-(2-(1-((2-(2,4-dioxotetrahydropyrimidin-1(2H)-yl)-5-fluoropyridin-4-yl)methyl)piperidin-4-yl)-6-methoxy-2H-indazol-5-yl)-6-(trifluoromethyl)picolinamide (GT-07531)

According to the method of step 2 of Scheme I-1, the target compound (GT-07531) was prepared (white solid, 41 mg, yield 69 %). ¹H NMR (400 MHz, DMSO-d6) δ 10.60 (s, 1H), 10.44 (s, 1H), 8.64 (s, 1H), 8.51 (s, 1H), 8.38 (s, 1H), 8.34 (t, *J =* 7.8 Hz, 1H), 8.29 (s, 1H), 8.16 (d, *J =* 7.1 Hz, 1H), 8.08 (d, *J =* 5.1 Hz, 1H), 7.11 (s, 1H), 4.43 (s, 2H), 4.00 (s, 2H), 3.92 (s, 3H), 3.55 (s, 2H), 3.24 (s, 2H), 2.65 (d, *J =* 6.2 Hz, 2H), 2.40 (s, 1H), 2.29 (s, 1H). LCMS (ESI) calcd for C₃₂H₃₂F₃N₆O₄⁺ [M+H]⁺: 621.24; found, 621.3.

### Example 674: Preparation of N-(2-(1-((2-(2,4-dioxotetrahydropyrimidin-1(2H)-yl)pyridin-4-yl)methyl)piperidin-4-yl)-6-methoxy-2H-indazol-5-yl)-6-(trifluoromethyl)picolinamide (GT-07532)

According to the method of step 2 of Scheme I-1, the target compound (GT-07532) was prepared (white solid, 32 mg, yield 54 %). ¹H NMR (400 MHz, DMSO-d6) δ 10.57 (s, 1H), 10.44 (s, 1H), 8.64 (s, 1H), 8.47 (d, *J* = 5.2 Hz, 1H), 8.38 (s, 1H), 8.34 (s, 1H), 8.29 (s, 1H), 8.16 (d, *J* = 7.5 Hz, 1H), 7.94 (s, 1H), 7.12 (s, 1H), 4.66 (t, *J =* 11.5 Hz, 1H), 4.37 (s, 2H), 4.04 (t, *J =* 6.6 Hz, 2H), 3.92 (s, 3H), 3.16 (d, *J =* 11.0 Hz, 2H), 2.65 (t, *J* = 6.6 Hz, 2H), 2.46 (s, 1H), 2.27 (d, *J* = 11.7 Hz, 2H). LCMS (ESI) calcd for C₃₀H₃₀F₃N₈O₄⁺ [M+H]⁺: 623.23; found, 623.3.

### Example 675: Preparation of N-(2-(1-((5-(2,4-dioxotetrahydropyrimidin-1(2H)-yl)pyridin-3-yl)methyl)piperidin-4-yl)-6-methoxy-2H-indazol-5-yl)-6-(trifluoromethyl)picolinamide (GT-07533)

According to the method of step 2 of Scheme I-1, the target compound (GT-07533) was prepared (white solid, 36 mg, yield 62 %). ¹H NMR (400 MHz, DMSO-d6) δ 10.72 (s, 1H), 10.57 (s, 1H), 8.86 (s, 1H), 8.76 (d, *J =* 6.5 Hz, 2H), 8.51 (s, 1H), 8.47 (s, 1H), 8.42 (s, 1H), 8.33 (s, 1H), 8.29 (s, 1H), 7.26 (s, 1H), 4.81 *(t, J =* 11.7 Hz, 1H), 4.54 (s, 2H), 4.04 (s, 3H), 3.64 (d, *J =* 11.9 Hz, 2H), 3.29 (d, *J =* 9.6 Hz, 2H), 2.83 (d, *J* = 6.7 Hz, 2H), 2.62 (d, *J* = 11.3 Hz, 1H), 2.40 (d, *J* = 11.1 Hz, 2H). LCMS (ESI) calcd for C₃₀H₃₀F₃N₈O₄⁺ [M+H]⁺: 623.23; found, 623.3.

### Example 676: Preparation of N-(2-(1-((5-(2,6-dioxopiperidin-3-yl)pyridin-2-yl)methyl)piperidin-4-yl)-6-methoxy-2H-indazol-5-yl)-6-(trifluoromethyl)picolinamide (GT-07534)

According to the method of step 2 of Scheme I-1, the target compound (GT-07534) was prepared (white solid, 31 mg, yield 65 %). ¹H NMR (400 MHz, DMSO-d6) δ 10.97 (s, 1H), 10.52 (s, 1H), 8.72 (s, 1H), 8.60 (d, *J =* 2.1 Hz, 1H), 8.46 (s, 1H), 8.42 (s, 1H), 8.40 (s, 1H), 8.23 (m, *J =* 7.6, 1.0 Hz, 1H), 7.84 (s, 1H), 7.68 (d, *J* = 8.0 Hz, 1H), 7.19 (s, 1H), 4.78 (m, *J =* 12.5, 8.5 Hz, 1H), 4.54 (s, 2H), 4.06 (m, *J =* 12.6, 4.9 Hz, 1H), 4.00 (s, 3H), 3.36 (t, *J* = 11.6 Hz, 2H), 2.80 - 2.69 (m, 1H), 2.63 - 2.53 (m, 2H), 2.39 (s, 1H), 2.38 - 2.27 (m, 2H), 2.11 - 2.02 (m, 1H). LCMS (ESI) calcd for C₃₁H₃₁F₃N₇O₄⁺ [M+H]⁺: 622.24; found, 622.3.

### Example 677: Preparation of N-(2-(1-((2-(2,6-dioxopiperidin-3-yl)pyridin-4-yl)methyl)piperidin-4-yl)-6-methoxy-2H-indazol-5-yl)-6-(trifluoromethyl)picolinamide (GT-07535)

According to the method of step 2 of Scheme I-1, the target compound (GT-07535) was prepared (white solid, 22 mg, yield 38 %). ¹H NMR (400 MHz, DMSO-d6) δ 10.45 (s, 1H), 9.01 (s, 1H), 8.83 (s, 1H), 8.65 (d, *J =* 2.7 Hz, 1H), 8.39 (s, 1H), 8.34 (s, 1H), 8.30 (d, *J* = 5.8 Hz, 1H), 8.18 - 8.14 (m, 1H), 7.61 (s, 1H), 7.12 (s, 1H), 4.71 (t, *J =* 10.6 Hz, 1H), 4.35 (s, 1H), 3.92 (d, *J =* 2.6 Hz, 3H), 3.52 - 3.44 (m, 1H), 3.37 (d, *J =* 12.9 Hz, 1H), 3.16 (s, 1H), 3.06 (m, *J* = 7.4, 4.2 Hz, 1H), 2.67 - 2.46 (m, 2H), 2.27 - 2.19 (m, 3H), 1.21 (m, *J =* 11.7, 6.5 Hz, 2H). LCMS (ESI) calcd for C₃₁H₃₁F₃N₇O₄⁺ [M+H]⁺: 622.24; found, 622.3.

### Example 678: Preparation of N-(2-(1-((5-(2,6-dioxopiperidin-3-yl)pyridin-3-yl)methyl)piperidin-4-yl)-6-methoxy-2H-indazol-5-yl)-6-(trifluoromethyl)picolinamide (GT-07536)

According to the method of step 2 of Scheme I-1, the target compound (GT-07536) was prepared (white solid, 34 mg, yield 59 %). ¹H NMR (400 MHz, DMSO-d6) δ 11.05 (d, *J =* 17.4 Hz, 1H), 10.51 (s, 1H), 9.01 (s, 1H), 8.82 (s, 1H), 8.72 (d, *J =* 12.0 Hz, 1H), 8.53 (d, *J =* 10.3 Hz, 1H), 8.46 (d, *J =* 7.8 Hz, 1H), 8.41 (t, *J =* 7.7 Hz, 1H), 8.36 (s, 1H), 8.22 (d, *J =* 7.6 Hz, 1H), 7.21 (s, 1H), 4.54 (s, 3H), 3.98 (s, 3H), 3.67 - 3.49 (m, 2H), 3.25 (s, 2H), 2.79 (m, *J* = 17.7, 12.8, 5.3 Hz, 1H), 2.67 (s, 1H), 2.56 (s, 1H), 2.34 (d, *J =* 10.2 Hz, 2H), 2.25 - 2.10 (m, 1H). LCMS (ESI) calcd for C₃₁H₃₁F₃N₇O₄⁺ [M+H]⁺: 622.24; found, 622.3.

### Example 679: Preparation of N-(2-(1-((2-(2,4-dioxotetrahydropyrimidin-1(2H)-yl)-5-fluoropyridin-4-yl)methyl)piperidin-4-yl)-6-(2-hydroxypropan-2-yl)-2H-indazol-5-yl)-6-(trifluoromethyl)picolinamide (GT-07538)

According to the method of step 2 of Scheme I-1, the target compound (GT-07538) was prepared (white solid, 26 mg, yield 44 %). ¹H NMR (400 MHz, DMSO-d6) δ 12.32 (s, 1H), 10.60 (s, 1H), 8.67 (s, 1H), 8.51 (s, 1H), 8.38 (d, *J* = 4.1 Hz, 1H), 8.31 (s, 2H), 8.10 (d, *J =* 8.0 Hz, 2H), 7.52 (s, 1H), 4.72 (t, *J =* 11.7 Hz, 1H), 4.43 (s, 2H), 3.99 (s, 2H), 3.39 (s, 1H), 3.25 (s, 2H), 2.65 (d, *J =* 6.3 Hz, 2H), 2.46 (s, 1H), 2.28 (d, *J* = 13.1 Hz, 2H), 1.55 (s, 6H). LCMS (ESI) calcd for C₃₂H₃₃F₄N₈O₄⁺ [M+H]⁺: 669.26; found, 669.3.

### Example 680: Preparation of N-(2-(1-((2-(2,4-dioxotetrahydropyrimidin-1(2H)-yl)pyridin-4-yl)methyl)piperidin-4-yl)-6-(2-hydroxypropan-2-yl)-2H-indazol-5-yl)-6-(trifluoromethyl)picolinamide (GT-07539)

According to the method of step 2 of Scheme I-1, the target compound (GT-07539) was prepared (white solid, 25 mg, yield 43 %). ¹H NMR (400 MHz, DMSO-d6) δ 12.45 (s, 1H), 10.70 (s, 1H), 8.80 (s, 1H), 8.59 (d, *J =* 5.1 Hz, 1H), 8.53 - 8.49 (m, 1H), 8.45 (s, 2H), 8.22 (d, *J* = 7.7 Hz, 1H), 8.06 (s, 1H), 7.67 (s, 2H), 4.89 - 4.79 (m, 1H), 4.50 (s, 3H), 4.16 (s, 3H), 3.61 (d, *J =* 11.3 Hz, 2H), 3.29 (d, *J =* 9.4 Hz, 2H), 2.78 (s, 2H), 2.41 (d, *J* = 11.6 Hz, 2H), 1.69 (d, *J* = 6.9 Hz, 6H). LCMS (ESI) calcd for C₃₂H₃₄F₃N₈O₄⁺ [M+H]⁺: 651.26; found, 651.3.

### Example 681: Preparation of N-(2-(1-((5-(2,4-dioxotetrahydropyrimidin-1(2H)-yl)pyridin-3-yl)methyl)piperidin-4-yl)-6-(2-hydroxypropan-2-yl)-2H-indazol-5-yl)-6-(trifluoromethyl)picolinamide (GT-07540)

According to the method of step 2 of Scheme I-1, the target compound (GT-07540) was prepared (white solid, 24 mg, yield 42 %). ¹H NMR (400 MHz, DMSO-d6) δ 12.45 (s, 1H), 10.74 (d, *J =* 21.9 Hz, 1H), 8.93 (d, *J =* 2.2 Hz, 1H), 8.86 - 8.79 (m, 2H), 8.51 (d, *J =* 8.2 Hz, 2H), 8.47 - 8.39 (m, 2H), 8.22 (d, *J =* 7.7 Hz, 1H), 7.67 (s, 1H), 4.87 (t, *J =* 11.5 Hz, 1H), 4.58 (s, 3H), 4.05 (t, *J* = 6.6 Hz, 2H), 3.65 (d, *J =* 11.4 Hz, 2H), 3.31 (d, *J =* 6.8 Hz, 2H), 2.85 (t, *J =* 6.6 Hz, 2H), 2.65 (d, *J =* 12.7 Hz, 1H), 2.41 (d, *J =* 12.4 Hz, 2H), 1.69 (d, *J =* 9.1 Hz, 6H). LCMS (ESI) calcd for C₃₂H₃₄F₃N₈O₄⁺ [M+H]⁺: 651.26; found, 651.3.

### Example 682: Preparation of N-(2-(1-((5-(2,6-dioxopiperidin-3-yl)pyridin-2-yl)methyl)piperidin-4-yl)-6-(2-hydroxypropan-2-yl)-2H-indazol-5-yl)-6-(trifluoromethyl)picolinamide (GT-07541)

According to the method of step 2 of Scheme I-1, the target compound (GT-07541) was prepared (white solid, 19 mg, yield 33 %). ¹H NMR (400 MHz, DMSO-d6) δ 12.45 (s, 1H), 11.03 (s, 2H), 8.81 (s, 1H), 8.66 (d, *J =* 2.0 Hz, 1H), 8.54 - 8.47 (m, 2H), 8.43 (t, *J =* 7.8 Hz, 1H), 8.23 (d, *J =* 7.8 Hz, 1H), 7.93 (dd, *J =* 8.1, 2.2 Hz, 1H), 7.80 (d, *J =* 8.1 Hz, 1H), 7.67 (s, 1H), 4.90 (t, *J =* 10.5 Hz, 1H), 4.60 (s, 2H), 3.64 (d, *J =* 11.7 Hz, 2H), 3.42 (t, *J =* 11.2 Hz, 2H), 2.86 - 2.75 (m, 1H), 2.69 - 2.59 (m, 3H), 2.51 - 2.33 (m, 3H), 2.18 - 2.07 (m, 1H), 1.68 (s, 6H). LCMS (ESI) calcd for C₃₃H₃₅F₃N₇O₄⁺ [M+H]⁺: 650.27; found, 650.4.

### Example 683: Preparation of N-(2-(1-((2-(2,6-dioxopiperidin-3-yl)pyridin-4-yl)methyl)piperidin-4-yl)-6-(2-hydroxypropan-2-yl)-2H-indazol-5-yl)-6-(trifluoromethyl)picolinamide (GT-07542)

According to the method of step 2 of Scheme I-1, the target compound (GT-07542) was prepared (white solid, 7 mg, yield 12 %). ¹H NMR (400 MHz, DMSO-d6) δ 12.39 (s, 1H), 10.95 (s, 1H), 8.75 (s, 1H), 8.67 (*t*, *J* = 7.5 Hz, 1H), 8.45 (s, 1H), 8.38 (d, *J =* 6.6 Hz, 2H), 8.18 (s, 1H), 7.72 (s, 2H), 7.61 (s, 1H), 4.82 (d, *J* = 10.9 Hz, 1H), 4.44 (s, 2H), 4.13 (m, *J* = 9.6, 5.1 Hz, 1H), 3.56 (d, *J =* 11.0 Hz, 1H), 3.50 - 3.34 (m, 1H), 3.24 (s, 2H), 2.69 - 2.63 (m, 1H), 2.61 - 2.54 (m, 2H), 2.34 (m, *J =* 8.5, 6.6 Hz, 3H), 2.21 (m, *J* = 13.4, 5.7 Hz, 1H), 1.62 (s, 6H). LCMS (ESI) calcd for C₃₃H₃₅F₃N₇O₄⁺ [M+H]⁺: 650.27; found, 650.3.

### Example 684: Preparation of N-(2-(1-((5-(2,6-dioxopiperidin-3-yl)pyridin-3-yl)methyl)piperidin-4-yl)-6-(2-hydroxypropan-2-yl)-2H-indazol-5-yl)-6-(trifluoromethyl)picolinamide (GT-07543)

According to the method of step 2 of Scheme I-1, the target compound (GT-07543) was prepared (white solid, 24 mg, yield 42 %). ¹H NMR (400 MHz, DMSO-d6) δ 12.39 (s, 1H), 11.08 (s, 1H), 9.02 (d, *J =* 1.4 Hz, 1H), 8.84 (s, 1H), 8.74 (s, 1H), 8.55 (s, 1H), 8.45 (d, *J =* 7.8 Hz, 1H), 8.38 (d, *J =* 7.8 Hz, 2H), 8.17 (d, *J* = 7.7 Hz, 1H), 7.61 (s, 1H), 4.86 - 4.77 (m, 1H), 4.56 (s, 3H), 4.21 (m, *J* = 12.6, 4.8 Hz, 2H), 3.70 - 3.48 (m, 2H), 3.26 (s, 2H), 2.86 - 2.72 (m, 1H), 2.67 (s, 1H), 2.37 (s, 2H), 2.26 - 2.11 (m, 1H), 1.62 (s, 6H). LCMS (ESI) calcd for C₃₃H₃₅F₃N₇O₄⁺ [M+H]⁺: 650.27; found, 650.3.

### Example 685: Preparation of 6-(5-cyano-1H-pyrrolo[2,3-b]pyridin-1-yl)-N-(1-((5-(2,6-dioxopiperidin-3-yl)-2-fluoropyridin-3-yl)methyl)piperidin-4-yl)-4-(isopropylamino)nicotinamide (GT-07544)

According to the method of step 2 of Scheme I-1, the target compound (GT-07544) was prepared (white solid, 28 mg, yield 47 %). ¹H NMR (400 MHz, DMSO-d6) δ 11.00 (s, 1H), 8.82 (d, *J =* 2.0 Hz, 1H), 8.69 (d, *J =* 2.0 Hz, 1H), 8.64 (s, 1H), 8.52 (d, *J =* 3.9 Hz, 1H), 8.26 (s, 2H), 8.08 (s, 1H), 6.91 (d, *J =* 3.9 Hz, 1H), 4.38 (d, *J* = 20.9 Hz, 2H), 4.06 (, *J =* 18.4, 9.2 Hz, 2H), 3.78 (m, *J =* 16.6, 9.9 Hz, 2H), 3.46 (d, *J =* 10.0 Hz, 1H), 3.27 - 3.12 (m, 2H), 2.84 - 2.70 (m, 1H), 2.69 - 2.62 (m, 1H), 2.31 (m, *J* = 12.7, 4.2 Hz, 1H), 2.05 (d, *J* = 13.9 Hz, 3H), 1.30 (d, *J =* 6.3 Hz, 7H). LCMS (ESI) calcd for C₃₃H₃₅FN₉O₃⁺ [M+H]⁺: 624.28; found, 624.3.

### Example 686: Preparation of 6-(5-cyano-1H-pyrrolo[2,3-b]pyridin-1-yl)-N-(1-((2-(2,6-dioxopiperidin-3-yl)pyridin-3-yl)methyl)piperidin-4-yl)-4-(isopropylamino)nicotinamide (GT-07546)

According to the method of step 2 of Scheme I-1, the target compound (GT-07546) was prepared (white solid, 38 mg, yield 65 %). ¹H NMR (400 MHz, DMSO-d6) δ 10.95 (s, 2H), 8.82 (d, *J* = 1.9 Hz, 1H), 8.70 (s, 1H), 8.67 (s, 1H), 8.65 - 8.62 (m, 1H), 8.51 (d, *J =* 3.8 Hz, 1H), 8.31 (d, *J =* 7.7 Hz, 1H), 8.02 (d, *J =* 4.9 Hz, 1H), 7.48 (m, *J* = 7.8, 4.8 Hz, 1H), 6.92 (d, *J* = 3.9 Hz, 1H), 4.76 (m, *J* = 10.8, 4.8 Hz, 1H), 4.47 - 4.43 (m, 1H), 3.86 - 3.79 (m, 2H), 3.46 (m, *J* = 23.9, 11.8 Hz, 2H), 3.24 (d, *J* = 7.3 Hz, 2H), 2.84 - 2.72 (m, 1H), 2.64 (m, *J* = 13.0, 3.9 Hz, 1H), 2.24 - 2.10 (m, 2H), 2.05 (s, 4H), 1.30 (d, *J* = 6.3 Hz, 7H). LCMS (ESI) calcd for C₃₃H₃₆N₉O₃⁺ [M+H]⁺: 606.29; found, 606.3.

### Example 687: Preparation of N-(2-(1-((5-(2,6-dioxopiperidin-3-yl)-2-fluoropyridin-3-yl)methyl)piperidin-4-yl)-6-methoxy-2H-indazol-5-yl)-3-(trifluoromethyl)benzamide (GT-07547)

According to the method of step 2 of Scheme I-1, the target compound (GT-07547) was prepared (white solid, 28 mg, yield 47 %). ¹H NMR (400 MHz, DMSO-d6) δ 10.94 (s, 1H), 9.74 (d, *J =* 13.9 Hz, 1H), 8.24 (s, 1H), 8.22 (d, *J* = 6.2 Hz, 2H), 8.20 (d, *J* = 1.9 Hz, 1H), 7.97 (d, *J* = 6.8 Hz, 1H), 7.90 (d, *J* = 7.8 Hz, 1H), 7.71 (t, *J* = 7.8 Hz, 1H), 7.04 (s, 1H), 4.69 (m, *J* = 22.7, 10.8 Hz, 1H), 4.35 (s, 1H), 4.01 (m, *J* = 12.5, 4.9 Hz, 1H), 3.80 (s, 3H), 3.52 (m, *J* = 24.7, 11.7 Hz, 2H), 3.23 (s, 2H), 2.75 - 2.64 (m, 1H), 2.58 (t, *J* = 6.9 Hz, 1H), 2.33 - 2.18 (m, 3H), 2.10 - 2.01 (m, 1H). LCMS (ESI) calcd for C₃₂H₃₁F₄N₆O₄⁺ [M+H]⁺: 639.23; found, 639.3.

### Example 688: Preparation of N-(2-(1-((2-(2,6-dioxopiperidin-3-yl)pyridin-3-yl)methyl)piperidin-4-yl)-6-methoxy-2H-indazol-5-yl)-3-(trifluoromethyl)benzamide (GT-07548)

According to the method of step 2 of Scheme I-1, the target compound (GT-07548) was prepared (white solid, 21 mg, yield 36 %). ¹H NMR (400 MHz, DMSO-d6) δ 10.88 (s, 1H), 9.72 (s, 1H), 8.56 (dd, *J =* 4.7, 1.5 Hz, 1H), 8.27 (s, 1H), 8.22 (d, *J =* 5.2 Hz, 2H), 8.19 (s, 1H), 7.97 (s, 1H), 7.90 (d, *J =* 7.7 Hz, 1H), 7.71 (t, *J* = 7.8 Hz, 1H), 7.41 (m, *J* = 7.8, 4.8 Hz, 1H), 7.04 (s, 1H), 4.70 - 4.65 (m, 1H), 4.50 - 4.40 (m, 2H), 3.80 (s, 3H), 3.57 (s, 1H), 3.33 - 3.22 (m, 2H), 2.72 - 2.65 (m, 1H), 2.60 (d, *J* = 4.0 Hz, 1H), 2.56 - 2.49 (m, 2H), 2.28 (d, *J* = 12.4 Hz, 2H), 2.16 - 2.07 (m, 1H). LCMS (ESI) calcd for C₃₂H₃₂F₃N₆O₄⁺ [M+H]⁺: 621.24; found, 621.3.

### Example 689: Preparation of N-(2-(1-((5-(2,6-dioxopiperidin-3-yl)-2-fluoropyridin-3-yl)methyl)piperidin-4-yl)-6-methoxy-2H-indazol-5-yl)-6-(trifluoromethyl)picolinamide (GT-07549)

According to the method of step 2 of Scheme I-1, the target compound (GT-07549) was prepared (white solid, 17 mg, yield 29 %). ¹H NMR (400 MHz, DMSO-d6) δ 11.01 (s, 1H), 10.52 (s, 1H), 8.71 (s, 1H), 8.46 (s, 1H), 8.41 (t, *J =* 7.8 Hz, 1H), 8.36 (s, 1H), 8.28 (s, 1H), 8.23 (d, *J =* 7.6 Hz, 2H), 7.18 (s, 1H), 4.75 (s, 1H), 4.44 (s, 2H), 4.08 (m, *J* = 12.5, 4.7 Hz, 1H), 3.99 (s, 3H), 3.61 (s, 2H), 3.35 - 3.24 (m, 2H), 2.84 - 2.71 (m, 1H), 2.70 - 2.58 (m, 2H), 2.33 (s, 1H), 2.13 (d, *J* = 5.3 Hz, 1H). LCMS (ESI) calcd for C₃₁H₃₀F₄N₇O₄⁺ [M+H]⁺: 640.23; found, 640.1.

### Example 690: Preparation of N-(2-(1-((2-(2,6-dioxopiperidin-3-yl)pyridin-3-yl)methyl)piperidin-4-yl)-6-methoxy-2H-indazol-5-yl)-6-(trifluoromethyl)picolinamide (GT-07550)

According to the method of step 2 of Scheme I-1, the target compound (GT-07550) was prepared (white solid, 7 mg, yield 12 %). ¹H NMR (400 MHz, DMSO-d6) δ 10.89 (s, 1H), 10.44 (s, 1H), 8.64 (s, 1H), 8.57-8.54 (m, *J* = 4.7, 1.6 Hz, 1H), 8.39 (d, *J =* 7.3 Hz, 1H), 8.36 - 8.33 (m, 1H), 8.31 (s, 1H), 8.18 - 8.14 (m, 2H), 7.41 (m, *J* = 7.9, 4.8 Hz, 1H), 7.12 (s, 1H), 4.66 (m, *J* = 10.9, 4.7 Hz, 2H), 4.44 (d, *J =* 13.7 Hz, 2H), 3.92 (s, 3H), 3.27 (d, *J =* 11.3 Hz, 2H), 2.71 - 2.64 (m, 1H), 2.60 (d, *J =* 3.9 Hz, 1H), 2.50 (d, *J =* 12.4 Hz, 2H), 2.28 (d, *J =* 11.3 Hz, 2H), 2.14 - 2.07 (m, 1H). LCMS (ESI) calcd for C₃₁H₃₁F₃N₇O₄⁺ [M+H]⁺: 622.24; found, 622.3.

### Example 691: Preparation of N-(2-(1-((2-(2,4-dioxotetrahydropyrimidin-1(2H)-yl)-5-fluoropyridin-4-yl)methyl)piperidin-4-yl)-5-(2-hydroxypropan-2-yl)benzo[d]thiazol-6-yl)-6-(trifluoromethyl)picolinamide (GT-07618)

According to the method of step 2 of Scheme I-1, the target compound (GT-07618) was prepared (white solid, 37 mg, yield 60 %). ¹H NMR (400 MHz, DMSO-d6) δ 12.63 (s, 1H), 10.72 (d, *J =* 8.8 Hz, 1H), 9.17 (d, *J* = 6.5 Hz, 1H), 8.62 (d, *J =* 14.6 Hz, 1H), 8.53 (d, *J =* 7.8 Hz, 1H), 8.45 (t, *J =* 7.8 Hz, 1H), 8.25 (d, *J =* 7.7 Hz, 1H), 8.21 (d, *J* = 5.3 Hz, 1H), 7.99 (d, *J =* 4.7 Hz, 1H), 4.53 (s, 2H), 4.12 (t, *J =* 6.6 Hz, 2H), 3.87 (s, 1H), 3.65 (d, *J =* 11.6 Hz, 2H), 3.48 (d, *J =* 11.3 Hz, 1H), 3.31 (s, 2H), 2.79 (t, *J =* 6.1 Hz, 2H), 2.37 (t, *J* = 14.3 Hz, 2H), 2.29 (d, *J* = 11.6 Hz, 1H), 1.69 (s, 6H). LCMS (ESI) calcd for C₃₂H₃₂F₄N₇O₄S⁺ [M+H]⁺: 686.22; found, 686.3.

### Example 692: Preparation of N-(2-(1-((2-(2,4-dioxotetrahydropyrimidin-1(2H)-yl)pyridin-4-yl)methyl)piperidin-4-yl)-5-(2-hydroxypropan-2-yl)benzo[d]thiazol-6-yl)-6-(trifluoromethyl)picolinamide (GT-07619)

According to the method of step 2 of Scheme I-1, the target compound (GT-07619) was prepared (white solid, 33 mg, yield 54 %). ¹H NMR (400 MHz, DMSO-d6) δ 12.58 (d, *J =* 6.2 Hz, 1H), 10.65 (s, 1H), 9.11 (s, 1H), 8.54 (d, *J =* 5.1 Hz, 1H), 8.47 (d, *J =* 7.8 Hz, 1H), 8.39 (t, *J =* 7.8 Hz, 1H), 8.20 (d, *J =* 7.7 Hz, 1H), 8.00 (s, 1H), 7.93 (s, 1H), 7.55 (d, *J =* 5.1 Hz, 1H), 4.43 (d, *J =* 4.5 Hz, 2H), 4.11 (t, *J =* 6.6 Hz, 2H), 3.53 (s, 2H), 3.17 (d, *J =* 11.1 Hz, 2H), 2.72 (d, *J =* 6.6 Hz, 2H), 2.42 (s, 1H), 2.33 (d, *J =* 12.0 Hz, 2H), 2.23 (t, *J =* 11.8 Hz, 2H), 1.64 (s, 6H). LCMS (ESI) calcd for C₃₂H₃₃F₃N₇O₄S⁺ [M+H]⁺: 668.23; found, 668.3.

### Example 693: Preparation of N-(2-(1-((5-(2,4-dioxotetrahydropyrimidin-1(2H)-yl)pyridin-3-yl)methyl)piperidin-4-yl)-5-(2-hydroxypropan-2-yl)benzo[d]thiazol-6-yl)-6-(trifluoromethyl)picolinamide (GT-07620)

According to the method of step 2 of Scheme I-1, the target compound (GT-07620) was prepared (white solid, 18 mg, yield 29 %). 1H NMR (400 MHz, DMSO-d6) δ 12.51 (s, 1H), 10.58 (s, 1H), 9.03 (s, 1H), 8.72 (d, J = 2.3 Hz, 1H), 8.60 (d, J = 1.5 Hz, 1H), 8.39 (s, 1H), 8.32 (d, J = 9.8, 5.8 Hz, 1H), 8.17 (s, 1H), 8.14 (s, 1H), 7.86 (s, 1H), 4.38 (d, J = 3.4 Hz, 2H), 3.89 (s, 2H), 3.46 (d, J = 11.5 Hz, 2H), 3.40 - 3.34 (m, 1H), 3.09 (d, J = 11.4 Hz, 2H), 2.70 (d, J = 6.7 Hz, 2H), 2.37 (s, 1H), 2.24 (s, 2H), 2.20 (s, 1H), 1.56 (s, 6H). LCMS (ESI) calcd for C₃₂H₃₃F₃N₇O₄S⁺ [M+H]⁺: 668.23; found, 668.3.

### Example 694: Preparation of N-(2-(1-((5-(2,6-dioxopiperidin-3-yl)-2-fluoropyridin-3-yl)methyl)piperidin-4-yl)-5-(2-hydroxypropan-2-yl)benzo[d]thiazol-6-yl)-6-(trifluoromethyl)picolinamide (GT-07621)

According to the method of step 2 of Scheme I-1, the target compound (GT-07621) was prepared (white solid, 23 mg, yield 37 %). ¹H NMR (400 MHz, DMSO-d6) δ 12.58 (s, 1H), 11.01 (s, 1H), 9.11 (s, 1H), 8.47 (d, *J =* 7.7 Hz, 1H), 8.40 (d, *J =* 7.8 Hz, 1H), 8.27 (s, 2H), 8.20 (d, *J =* 7.8 Hz, 1H), 7.93 (s, 1H), 4.41 (s, 2H), 4.07 (d, *J =* 7.7 Hz, 1H), 3.55 (s, 2H), 3.43 (d, *J =* 11.7 Hz, 2H), 3.23 (s, 2H), 2.83 - 2.71 (m, 1H), 2.65 (s, 1H), 2.33 (s, 2H), 2.26 (d, *J =* 10.8 Hz, 1H), 2.21 (s, 1H), 2.17 - 2.09 (m, 1H), 1.64 (s, 6H). LCMS (ESI) calcd for C₃₃H₃₃F₄N₆O₄S⁺ [M+H]⁺: 685.22; found, 685.3.

### Example 695: Preparation of N-(2-(1-((2-(2,6-dioxopiperidin-3-yl)pyridin-4-yl)methyl)piperidin-4-yl)-5-(2-hydroxypropan-2-yl)benzo[d]thiazol-6-yl)-6-(trifluoromethyl)picolinamide (GT-07622)

According to the method of step 2 of Scheme I-1, the target compound (GT-07622) was prepared (white solid, 23 mg, yield 38 %). ¹H NMR (400 MHz, DMSO-d6) δ 12.58 (s, 1H), 9.11 (s, 1H), 8.98 (s, 1H), 8.66 (d, *J* = 5.6 Hz, 1H), 8.46 (s, 1H), 8.40 (d, *J =* 7.8 Hz, 1H), 8.20 (d, *J =* 7.7 Hz, 1H), 7.94 (s, 1H), 7.71 (s, 1H), 4.41 (s, 1H), 3.63 - 3.55 (m, 2H), 3.46 - 3.43 (m, 1H), 3.38 (d, *J* = 12.4 Hz, 2H), 3.12 (d, *J =* 3.1 Hz, 2H), 2.30 (s, 3H), 2.07 (s, 1H), 1.64 (s, 6H), 1.29 (d, *J* = 13.2, 6.6 Hz, 3H). LCMS (ESI) calcd for C₃₃H₃₄F₃N₆O₄S⁺ [M+H]⁺: 667.23; found, 667.3.

### Example 696: Preparation of N-(2-(1-((5-(2,6-dioxopiperidin-3-yl)pyridin-2-yl)methyl)piperidin-4-yl)-5-(2-hydroxypropan-2-yl)benzo[d]thiazol-6-yl)-6-(trifluoromethyl)picolinamide (GT-07623)

According to the method of step 2 of Scheme I-1, the target compound (GT-07623) was prepared (white solid, 34 mg, yield 56 %). ¹H NMR (400 MHz, DMSO-d6) δ 12.51 (s, 1H), 10.90 (s, 1H), 9.04 (s, 1H), 8.52 (d, *J* = 2.0 Hz, 1H), 8.40 (d, *J =* 7.7 Hz, 1H), 8.33 (d, *J =* 7.8 Hz, 1H), 8.13 (d, *J =* 7.8 Hz, 1H), 7.86 (s, 1H), 7.77 (d, *J =* 2.2 Hz, 1H), 7.64 (d, *J =* 8.0 Hz, 1H), 4.43 (s, 2H), 3.99 (m, *J* = 12.6, 4.8 Hz, 1H), 3.49 - 3.46 (m, 2H), 3.46 - 3.43 (m, 2H), 3.22 (t, *J =* 10.6 Hz, 2H), 2.73 - 2.62 (m, 1H), 2.51 (m, *J* = 13.8, 3.5 Hz, 1H), 2.28 (d, *J* = 4.0 Hz, 2H), 2.19 (d, *J* = 10.8 Hz, 2H), 2.04 - 1.95 (m, 1H), 1.57 (s, 6H). LCMS (ESI) calcd for C₃₃H₃₄F₃N₆O₄S⁺ [M+H]⁺: 667.23; found, 667.3.

### Example 697: Preparation of N-(2-(1-((5-(2,6-dioxopiperidin-3-yl)pyridin-3-yl)methyl)piperidin-4-yl)-5-(2-hydroxypropan-2-yl)benzo[d]thiazol-6-yl)-6-(trifluoromethyl)picolinamide (GT-07624)

According to the method of step 2 of Scheme I-1, the target compound (GT-07624) was prepared (white solid, 31 mg, yield 51 %). ¹H NMR (400 MHz, DMSO-d6) δ 12.51 (s, 1H), 10.98 (s, 1H), 9.03 (s, 1H), 8.86 (s, 1H), 8.69 (d, *J =* 1.5 Hz, 1H), 8.39 (s, 1H), 8.32 (s, 2H), 8.14 (s, 1H), 7.86 (s, 1H), 4.42 (s, 2H), 4.08 (d, *J =* 7.6 Hz, 2H), 3.67 - 3.61 (m, 1H), 3.44 (d, *J =* 17.8 Hz, 2H), 3.10 (s, 2H), 2.71 (s, 1H), 2.59 (s, 1H), 2.36 (s, 1H), 2.23 (s, 3H), 2.10 (m, *J =* 9.2, 4.2 Hz, 1H), 1.56 (s, 6H). LCMS (ESI) calcd for C₃₃H₃₄F₃N₆O₄S⁺ [M+H]⁺: 667.23; found, 667.3.

### Example 698: Preparation of N-(2-(1-((2-(2,6-dioxopiperidin-3-yl)pyridin-3-yl)methyl)piperidin-4-yl)-5-(2-hydroxypropan-2-yl)benzo[d]thiazol-6-yl)-6-(trifluoromethyl)picolinamide (GT-07625)

According to the method of step 2 of Scheme I-1, the target compound (GT-07625) was prepared (white solid, 31 mg, yield 51 %). ¹H NMR (400 MHz, DMSO-d6) δ 12.51 (s, 1H), 10.88 (s, 1H), 9.04 (s, 1H), 8.56 (d, *J* = 3.2 Hz, 1H), 8.39 (s, 1H), 8.32 (s, 1H), 8.21 (d, *J =* 6.4 Hz, 1H), 8.13 (s, 1H), 7.86 (s, 1H), 7.41 (m, *J* = 7.8, 4.8 Hz, 1H), 4.68 (m, *J* = 11.0, 4.9 Hz, 1H), 4.44 (s, 1H), 3.45 - 3.40 (m, 2H), 3.36 (d, *J* = 7.1 Hz, 1H), 3.21 (s, 2H), 2.70 (m, *J* = 17.3, 12.4, 5.2 Hz, 1H), 2.60 - 2.55 (m, 1H), 2.39 (s, 1H), 2.24 (s, 4H), 2.14 - 2.09 (m, 1H), 1.57 (s, 6H), 1.21 (s, 1H). LCMS (ESI) calcd for C₃₃H₃₄F₃N₆O₄S⁺ [M+H]⁺: 667.23; found, 667.3.

### Example 699: Preparation of N-(2-(1-((2-(2,4-dioxotetrahydropyrimidin-1(2H)-yl)-5-fluoropyridin-4-yl)methyl)piperidin-4-yl)-5-(2-hydroxypropan-2-yl)benzo[d]oxazol-6-yl)-6-(trifluoromethyl)picolinamide (GT-07626)

According to the method of step 2 of Scheme I-1, the target compound (GT-07626) was prepared (white solid, 12 mg, yield 20 %). ¹H NMR (400 MHz, DMSO-d6) δ 10.60 (s, 1H), 10.32 (s, 1H), 10.12 (s, 1H), 9.29 (s, 1H), 8.50 (s, 1H), 8.35 (s, 1H), 8.33 (d, *J* = 7.5 Hz, 1H), 8.13 (d, *J* = 7.3 Hz, 1H), 8.07 (s, 1H), 8.04 (d, *J* = 5.1 Hz, 1H), 7.69 (s, 1H), 5.37 (s, 1H), 4.99 (s, 1H), 4.39 (s, 2H), 3.99 (t, *J* = 6.6 Hz, 2H), 3.45 (d, *J* = 10.0 Hz, 2H), 3.33 (s, 1H), 3.26 - 3.22 (m, 1H), 2.99 (s, 2H), 2.72 (s, 1H), 2.65 (t, *J* = 6.6 Hz, 2H), 1.98 (s, 3H), 1.94 (s, 3H). LCMS (ESI) calcd for C₃₂H₃₂F₄N₇O₅⁺ [M+H]⁺: 670.24; found, 670.3.

### Example 700: Preparation of N-(2-(1-((2-(2,4-dioxotetrahydropyrimidin-1(2H)-yl)pyridin-4-yl)methyl)piperidin-4-yl)-5-(2-hydroxypropan-2-yl)benzo[d]oxazol-6-yl)-6-(trifluoromethyl)picolinamide (GT-07627)

According to the method of step 2 of Scheme I-1, the target compound (GT-07627) was prepared (white solid, 9 mg, yield 15 %). ¹H NMR (400 MHz, DMSO-d6) δ 10.64 (s, 1H), 10.39 (s, 1H), 10.19 (s, 1H), 9.37 (s, 1H), 8.53 (d, *J* = 5.1 Hz, 1H), 8.42 (s, 1H), 8.40 (d, *J =* 7.5 Hz, 1H), 8.20 (d, *J =* 7.4 Hz, 1H), 8.14 (s, 1H), 7.98 (s, 1H), 7.77 (s, 1H), 5.44 (s, 1H), 5.06 (s, 1H), 4.40 (d, *J =* 4.4 Hz, 2H), 4.10 (t, *J =* 6.7 Hz, 2H), 3.42 (s, 3H), 3.25 (s, 1H), 2.98 (d, *J* = 5.9 Hz, 2H), 2.85 - 2.76 (m, 1H), 2.72 (t, *J* = 6.5 Hz, 2H), 2.06 (d, *J* = 5.6 Hz, 3H), 2.00 (s, 3H). LCMS (ESI) calcd for C₃₂H₃₃F₃N₇O₅⁺ [M+H]⁺: 652.25; found, 652.2.

### Example 701: Preparation of N-(2-(1-((5-(2,4-dioxotetrahydropyrimidin-1(2H)-yl)pyridin-3-yl)methyl)piperidin-4-yl)-5-(2-hydroxypropan-2-yl)benzo[d]oxazol-6-yl)-6-(trifluoromethyl)picolinamide (GT-07628)

According to the method of step 2 of Scheme I-1, the target compound (GT-07628) was prepared (white solid, 10 mg, yield 16 %). ¹H NMR (400 MHz, DMSO-d6) δ 10.57 (s, 1H), 10.32 (s, 1H), 9.29 (s, 1H), 8.69 (d, *J =* 2.2 Hz, 1H), 8.55 (d, *J =* 1.4 Hz, 1H), 8.35 (s, 1H), 8.33 (d, *J =* 7.5 Hz, 1H), 8.14 (s, 1H), 8.11 (d, *J =* 7.5 Hz, 1H), 8.07 (s, 1H), 7.70 (s, 1H), 5.37 (s, 1H), 4.99 (s, 1H), 4.35 (d, *J =* 4.4 Hz, 2H), 3.87 (t, *J =* 6.6 Hz, 2H), 3.79 (s, 1H), 3.70 - 3.68 (m, 1H), 3.38 (d, *J* = 11.1 Hz, 2H), 3.30 - 3.16 (m, 1H), 2.90 (d, *J* = 8.9 Hz, 1H), 2.83 - 2.74 (m, 1H), 2.70 (t, *J* = 6.5 Hz, 2H), 1.99 (d, *J* = 7.7 Hz, 3H), 1.93 (s, 3H). LCMS (ESI) calcd for C₃₂H₃₃F₃N₇O₅⁺ [M+H]⁺: 652.25; found, 652.2.

### Example 702: Preparation of N-(2-(1-((5-(2,6-dioxopiperidin-3-yl)-2-fluoropyridin-3-yl)methyl)piperidin-4-yl)-5-(2-hydroxypropan-2-yl)benzo[d]oxazol-6-yl)-6-(trifluoromethyl)picolinamide (GT-07629)

According to the method of step 2 of Scheme I-1, the target compound (GT-07629) was prepared (white solid, 5 mg, yield 7 %). ¹H NMR (400 MHz, DMSO-d6) δ 10.93 (s, 1H), 10.32 (s, 1H), 10.11 (s, 1H), 9.26 (s, 1H), 8.35 (s, 1H), 8.33 (d, *J* = 7.5 Hz, 1H), 8.19 (s, 1H), 8.13 (d, *J* = 7.6 Hz, 1H), 8.07 (s, 1H), 7.69 (s, 1H), 5.37 (s, 1H), 4.99 (s, 1H), 4.31 (s, 2H), 4.00 (m, *J* = 12.7, 4.3 Hz, 1H), 3.40 (d, *J* = 13.5 Hz, 2H), 2.98 (s, 3H), 2.68 (m, *J* = 21.7, 8.7 Hz, 2H), 2.57 (t, *J =* 15.5 Hz, 2H), 2.25 - 2.18 (m, 1H), 2.04 (s, 2H), 1.98 (s, 3H), 1.87 (s, 3H). LCMS (ESI) calcd for C₃₃H₃₃F₄N₆O₅⁺ [M+H]⁺: 669.24; found, 669.2.

### Example 703: Preparation of N-(2-(1-((5-(2,6-dioxopiperidin-3-yl)pyridin-3-yl)methyl)piperidin-4-yl)-5-(2-hydroxypropan-2-yl)benzo[d]oxazol-6-yl)-6-(trifluoromethyl)picolinamide (GT-07630)

According to the method of step 2 of Scheme I-1, the target compound (GT-07630) was prepared (white solid, 12 mg, yield 19 %). ¹H NMR (400 MHz, DMSO-d6) δ 10.96 (s, 1H), 10.32 (s, 1H), 9.30 (s, 1H), 8.75 (s, 1H), 8.62 (s, 1H), 8.35 (s, 1H), 8.33 (d, *J =* 7.5 Hz, 1H), 8.14 (s, 1H), 8.07 (s, 1H), 7.70 (s, 1H), 5.37 (s, 1H), 4.99 (s, 1H), 4.36 (s, 2H), 4.06 - 4.00 (m, 1H), 3.44 - 3.30 (m, 2H), 3.20 (s, 1H), 2.90 (s, 2H), 2.80 - 2.64 (m, 2H), 2.55 (d, *J* = 17.3 Hz, 1H), 2.26 (m, *J* = 12.7, 4.1 Hz, 1H), 2.12 - 2.01 (m, 2H), 1.98 (s, 3H), 1.93 (s, 3H). LCMS (ESI) calcd for C₃₃H₃₄F₃N₆O₅⁺ [M+H]⁺: 651.25; found, 651.2.

### Example 704: Preparation of N-(2-(1-((2-(2,6-dioxopiperidin-3-yl)pyridin-3-yl)methyl)piperidin-4-yl)-5-(2-hydroxypropan-2-yl)benzo[d]oxazol-6-yl)-6-(trifluoromethyl)picolinamide (GT-07631)

According to the method of step 2 of Scheme I-1, the target compound (GT-07631) was prepared (white solid, 9 mg, yield 15 %). ¹H NMR (400 MHz, DMSO-d6) δ 10.89 (s, 1H), 10.32 (s, 2H), 9.35 (s, 1H), 8.55 (m, *J* = 4.7, 1.5 Hz, 1H), 8.35 (s, 1H), 8.31 (d, *J* = 7.6 Hz, 1H), 8.17 (m, *J* = 7.9, 1.4 Hz, 1H), 8.12 (m, *J* = 2.6, 1.2 Hz, 1H), 8.07 (s, 1H), 7.70 (s, 1H), 7.41 - 7.38 (m, 1H), 5.46 (s, 1H), 5.37 (s, 1H), 4.99 (s, 1H), 4.66 (m, *J* = 10.9, 5.0 Hz, 1H), 4.41 (d, *J* = 5.1 Hz, 2H), 3.47 - 3.38 (m, 2H), 3.04 (s, 2H), 2.78 - 2.70 (m, 1H), 2.65 (m, *J* = 12.2, 4.8 Hz, 1H), 2.60 (s, 1H), 2.54 (m, *J* = 11.9, 4.7 Hz, 1H), 2.38 (m, *J* = 11.8, 4.7 Hz, 1H), 2.08 (m, *J =* 8.9, 4.3 Hz, 1H), 1.98 (s, 3H), 1.95 (s, 3H). LCMS (ESI) calcd for C₃₃H₃₄F₃N₆O₅⁺ [M+H]⁺: 651.25; found, 651.2.

### Example 705: Preparation of N-(2-(1-((2-(2,6-dioxopiperidin-3-yl)pyridin-4-yl)methyl)piperidin-4-yl)-5-(2-hydroxypropan-2-yl)benzo[d]oxazol-6-yl)-6-(trifluoromethyl)picolinamide (GT-07762)

According to the method of step 2 of Scheme I-1, the target compound (GT-07762) was prepared (white solid, 9 mg, yield 15 %). ¹H NMR (400 MHz, DMSO-d6) δ 10.32 (s, 1H), 9.26 (d, *J* = 19.9 Hz, 1H), 8.57 (d, *J* = 5.0 Hz, 1H), 8.35 (s, 1H), 8.32 (s, 1H), 8.14 (s, 1H), 8.07 (s, 1H), 7.70 (s, 1H), 7.55 (s, 1H), 5.37 (s, 1H), 4.99 (s, 1H), 4.30 (s, 1H), 3.52 (s, 2H), 3.46 - 3.41 (m, 1H), 3.38 (s, 1H), 3.24 (s, 1H), 2.88 (d, *J =* 22.6 Hz, 1H), 2.81 (d, *J* = 11.5 Hz, 1H), 2.73 (s, 1H), 2.51 (s, 1H), 2.44 (s, 1H), 2.23 (m, *J* = 16.6, 7.3 Hz, 1H), 2.12 (m, *J* = 13.2, 5.7 Hz, 1H), 1.99 (s, 3H), 1.93 (s, 3H), 1.76 (s, 1H). LCMS (ESI) calcd for C₃₃H₃₄F₃N₆O₅⁺ [M+H]⁺: 651.25; found, 651.2.

### Example 706: Preparation of N-(2-(1-((5-(2,6-dioxopiperidin-3-yl)pyridin-2-yl)methyl)piperidin-4-yl)-5-(2-hydroxypropan-2-yl)benzo[d]oxazol-6-yl)-6-(trifluoromethyl)picolinamide (GT-07763)

According to the method of step 2 of Scheme I-1, the target compound (GT-07763) was prepared (white solid, 10 mg, yield 16 %). ¹H NMR (400 MHz, DMSO-d6) δ 10.89 (s, 1H), 10.32 (s, 1H), 9.29 (s, 1H), 8.52 (d, *J* = 1.7 Hz, 1H), 8.36 (d, *J* = 7.4 Hz, 1H), 8.33 (d, *J* = 7.6 Hz, 1H), 8.13 (d, *J* = 7.5 Hz, 1H), 8.07 (s, 1H), 7.77 (d, *J =* 6.0 Hz, 1H), 7.71 (s, 1H), 7.55 (d, *J =* 8.0 Hz, 1H), 5.37 (s, 1H), 4.99 (s, 1H), 4.41 (s, 2H), 3.97 (m, *J* = 12.6, 4.7 Hz, 1H), 3.60 (s, 1H), 3.50 - 3.48 (m, 1H), 3.42 (d, *J* = 11.9 Hz, 2H), 3.03 (s, 2H), 2.77 (s, 1H), 2.70 - 2.62 (m, 1H), 2.53 (s, 2H), 2.25 (m, *J* = 12.8, 4.3 Hz, 1H), 1.99 (s, 3H), 1.95 (s, 3H). LCMS (ESI) calcd for C₃₃H₃₄F₃N₆O₅⁺ [M+H]⁺: 651.25; found, 651.2.

### Example 707: Preparation of N-(3-(difluoromethyl)-1-(1-((2-(2,4-dioxotetrahydropyrimidin-1(2H)-yl)-5-fluoropyridin-4-yl)methyl)piperidin-4-yl)-1H-pyrazol-4-yl)-5-morpholinopyrazolo[1,5-a]pyrimidine-3-carboxamide (GT-07764)

According to the method of step 2 of Scheme I-1, the target compound (GT-07764) was prepared (white solid, 38 mg, yield 64 %). ¹H NMR (400 MHz, DMSO-d6) δ 10.60 (s, 1H), 9.35 (s, 1H), 8.76 (d, *J =* 7.9 Hz, 1H), 8.50 (s, 1H), 8.34 (s, 1H), 8.22 (s, 1H), 8.08 (d, *J* = 5.1 Hz, 1H), 6.84 (d, *J* = 7.9 Hz, 1H), 4.49 (s, 1H), 4.40 (s, 2H), 3.98 (t, *J* = 6.4 Hz, 2H), 3.77 - 3.62 (m, 8H), 3.53 (d, *J* = 11.3 Hz, 2H), 3.45 (s, 1H), 3.17 (s, 2H), 2.66 (t, *J* = 6.5 Hz, 2H), 2.37 - 2.24 (m, 2H), 2.20 (s, 2H). LCMS (ESI) calcd for C₃₀H₃₃F₃N₁₁O₄⁺ [M+H]⁺: 668.27; found, 668.2.

### Example 708: Preparation of N-(3-(difluoromethyl)-1-(1-((2-(2,4-dioxotetrahydropyrimidin-1(2H)-yl)pyridin-4-yl)methyl)piperidin-4-yl)-1H-pyrazol-4-yl)-5-morpholinopyrazolo[1,5-a]pyrimidine-3-carboxamide (GT-07765)

According to the method of step 2 of Scheme I-1, the target compound (GT-07765) was prepared (white solid, 38 mg, yield 64 %). ¹H NMR (400 MHz, DMSO-d6) δ 10.57 (s, 1H), 9.34 (s, 1H), 8.76 (s, 1H), 8.45 (s, 1H), 8.36 (s, 1H), 8.22 (s, 1H), 7.92 (s, 1H), 7.55 (s, 1H), 6.85 (s, 1H), 4.49 (s, 1H), 4.35 (s, 2H), 4.03 (s, 2H), 3.72 (s, 4H), 3.65 (s, 4H), 3.42 (s, 2H), 3.10 (s, 2H), 2.44 (s, 3H), 2.33 (s, 2H), 2.20 (s, 2H). LCMS (ESI) calcd for C₃₀H₃₄F₂N₁₁O₄⁺ [M+H]⁺: 650.28; found, 650.2.

### Example 709: Preparation of N-(3-(difluoromethyl)-1-(1-((5-(2,4-dioxotetrahydropyrimidin-1(2H)-yl)pyridin-3-yl)methyl)piperidin-4-yl)-1H-pyrazol-4-yl)-5-morpholinopyrazolo[1,5-a]pyrimidine-3-carboxamide (GT-07766)

According to the method of step 2 of Scheme I-1, the target compound (GT-07766) was prepared (white solid, 41 mg, yield 72 %). ¹H NMR (400 MHz, DMSO-d6) δ 10.61 (s, 1H), 9.35 (s, 1H), 8.75 (s, 2H), 8.65 (s, 1H), 8.36 (s, 1H), 8.27 (s, 1H), 8.22 (s, 1H), 6.84 (d, *J =* 7.9 Hz, 1H), 4.40 (s, 2H), 3.72 (s, 4H), 3.65 (d, *J* = 3.2 Hz, 4H), 3.47 (d, *J* = 11.6 Hz, 2H), 3.08 (s, 2H), 2.71 (t, *J* = 6.2 Hz, 2H), 2.68 - 2.67 (m, 1H), 2.33 (d, *J* = 10.8 Hz, 2H), 2.20 (s, 2H), 1.22 (dd, *J* = 13.9, 6.5 Hz, 4H). LCMS (ESI) calcd for C₃₀H₃₄F₂N₁₁O₄⁺ [M+H]⁺: 650.28; found, 650.2.

### Example 710: Preparation of N-(3-(difluoromethyl)-1-(1-((5-(2,6-dioxopiperidin-3-yl)-2-fluoropyridin-3-yl)methyl)piperidin-4-yl)-1H-pyrazol-4-yl)-5-morpholinopyrazolo[1,5-a]pyrimidine-3-carboxamide (GT-07767)

According to the method of step 2 of Scheme I-1, the target compound (GT-07767) was prepared (white solid, 20 mg, yield 34 %). ¹H NMR (400 MHz, DMSO-d6) δ 10.93 (s, 1H), 9.35 (s, 1H), 8.76 (d, *J =* 7.9 Hz, 1H), 8.34 (s, 1H), 8.22 (s, 1H), 8.19 (s, 1H), 6.84 (d, *J* = 7.9 Hz, 1H), 4.49 (s, 1H), 4.33 (s, 1H), 4.00 (d, *J* = 7.7 Hz, 1H), 3.72 (s, 4H), 3.65 (s, 4H), 3.49 (s, 2H), 3.33 - 3.29 (m, 1H), 3.14 (s, 2H), 2.68 (d, *J =* 12.4 Hz, 1H), 2.55 (d, *J* = 17.4 Hz, 1H), 2.27 (d, *J* = 12.2 Hz, 2H), 2.21 (s, 2H), 2.06 (s, 2H). LCMS (ESI) calcd for C₃₁H₃₄F₃N₁₀O₄⁺ [M+H]⁺: 667.27; found, 667.2.

### Example 711: Preparation of N-(3-(difluoromethyl)-1-(1-((2-(2,6-dioxopiperidin-3-yl)pyridin-4-yl)methyl)piperidin-4-yl)-1H-pyrazol-4-yl)-5-morpholinopyrazolo[1,5-a]pyrimidine-3-carboxamide (GT-07768)

According to the method of step 2 of Scheme I-1, the target compound (GT-07768) was prepared (white solid, 18 mg, yield 31 %). ¹H NMR (400 MHz, DMSO-d6) δ 10.88 (s, 1H), 9.35 (s, 1H), 8.76 (d, *J =* 7.9 Hz, 1H), 8.60 (d, *J =* 5.0 Hz, 1H), 8.36 (s, 1H), 8.22 (s, 1H), 7.64 (s, 2H), 6.85 (d, *J =* 8.0 Hz, 1H), 4.50 (s, 1H), 4.35 (s, 2H), 4.05 (dd, *J* = 9.5, 5.3 Hz, 1H), 3.74 - 3.73 (m, 4H), 3.66 (d, *J* = 3.5 Hz, 4H), 3.44 (d, *J* = 11.0 Hz, 2H), 3.08 (s, 2H), 2.59 (dd, *J* = 10.2, 5.4 Hz, 1H), 2.50 (d, *J* = 5.2 Hz, 1H), 2.37 - 2.28 (m, 2H), 2.20 (s, 2H), 2.18 - 2.08 (m, 2H). LCMS (ESI) calcd for C₃₁H₃₅F₂N₁₀O₄⁺ [M+H]⁺: 649.28; found, 649.2.

### Example 712: Preparation of N-(3-(difluoromethyl)-1-(1-((5-(2,6-dioxopiperidin-3-yl)pyridin-2-yl)methyl)piperidin-4-yl)-1H-pyrazol-4-yl)-5-morpholinopyrazolo[1,5-a]pyrimidine-3-carboxamide (GT-07769)

According to the method of step 2 of Scheme I-1, the target compound (GT-07769) was prepared (white solid, 34 mg, yield 60 %). ¹H NMR (400 MHz, DMSO-d6) δ 10.90 (s, 1H), 9.36 (s, 1H), 8.76 (d, *J =* 7.9 Hz, 1H), 8.52 (s, 1H), 8.38 (s, 1H), 8.23 (s, 1H), 7.78 (dd, *J =* 8.0, 1.6 Hz, 1H), 7.66 (s, 1H), 6.85 (d, *J =* 8.0 Hz, 1H), 4.53 (t, *J* = 10.6 Hz, 1H), 4.44 (s, 2H), 3.99 (d, *J* = 7.8 Hz, 1H), 3.73 (s, 4H), 3.66 (s, 4H), 3.63 - 3.61 (m, 2H), 3.47 (d, *J* = 11.7 Hz, 2H), 3.22 (s, 2H), 2.77 - 2.61 (m, 1H), 2.53 (s, 1H), 2.29 (d, *J* = 12.0 Hz, 2H), 2.24 (s, 2H), 2.05 - 1.94 (m, 1H). LCMS (ESI) calcd for C₃₁H₃₅F₂N₁₀O₄⁺ [M+H]⁺: 649.28; found, 649.2.

### Example 713: Preparation of N-(3-(difluoromethyl)-1-(1-((5-(2,6-dioxopiperidin-3-yl)pyridin-3-yl)methyl)piperidin-4-yl)-1H-pyrazol-4-yl)-5-morpholinopyrazolo[1,5-a]pyrimidine-3-carboxamide (GT-07770)

According to the method of step 2 of Scheme I-1, the target compound (GT-07770) was prepared (white solid, 37 mg, yield 65 %). ¹H NMR (400 MHz, DMSO-d6) δ 11.05 (s, 1H), 9.42 (s, 1H), 8.95 (s, 1H), 8.83 (d, *J* = 7.9 Hz, 1H), 8.78 (s, 1H), 8.43 (s, 2H), 8.29 (s, 1H), 6.92 (d, *J =* 7.9 Hz, 1H), 4.58 (s, 1H), 4.50 (s, 2H), 3.79 (s, 4H), 3.72 (s, 4H), 3.54 (s, 2H), 3.15 (s, 2H), 2.76 (dd, *J =* 12.5, 4.8 Hz, 1H), 2.66 (s, 1H), 2.36 (d, *J* = 8.8 Hz, 2H), 2.27 (s, 2H), 2.18 (s, 2H), 1.34 - 1.25 (m, 3H). LCMS (ESI) calcd for C₃₁H₃₅F₂N₁₀O₄⁺ [M+H]⁺: 649.28; found, 649.2.

### Example 714: Preparation of N-(3-(difluoromethyl)-1-(1-((2-(2,6-dioxopiperidin-3-yl)pyridin-3-yl)methyl)piperidin-4-yl)-1H-pyrazol-4-yl)-5-morpholinopyrazolo[1,5-a]pyrimidine-3-carboxamide (GT-07771)

According to the method of step 2 of Scheme I-1, the target compound (GT-07771) was prepared (white solid, 34 mg, yield 60 %). ¹H NMR (400 MHz, DMSO-d6) δ 10.89 (s, 1H), 9.34 (s, 1H), 8.75 (d, *J =* 7.9 Hz, 1H), 8.58 (d, *J =* 3.3 Hz, 1H), 8.37 (s, 1H), 8.30 (d, *J =* 7.8 Hz, 1H), 8.22 (s, 1H), 7.44 (d, *J =* 3.0 Hz, 1H), 6.84 (d, *J =* 7.9 Hz, 1H), 4.76 (d, *J =* 6.2 Hz, 1H), 4.47 (s, 3H), 3.93 (s, 1H), 3.72 (s, 4H), 3.65 (d, *J =* 4.4 Hz, 4H), 3.52 (s, 1H), 3.41 (s, 1H), 3.25 (d, *J =* 10.2 Hz, 2H), 2.73 (s, 1H), 2.59 (s, 1H), 2.37 (d, *J =* 13.0 Hz, 1H), 2.18 (s, 2H), 2.13 (d, *J* = 4.6 Hz, 1H). LCMS (ESI) calcd for C₃₁H₃₅F₂N₁₀O₄⁺ [M+H]⁺: 649.28; found, 649.2.

### Example 715: Preparation of 5-((1R,4R)-2-oxa-5-azabicyclo[2.2.1]heptan-5-yl)-N-(3-(difluoromethyl)-1-(1-((2-(2,4-dioxotetrahydropyrimidin-1(2H)-yl)-5-fluoropyridin-4-yl)methyl)piperidin-4-yl)-1H-pyrazol-4-yl)pyrazolo[1,5-a]pyrimidine-3-carboxamide (GT-07772)

According to the method of step 2 of Scheme I-1, the target compound (GT-07772) was prepared (white solid, 27 mg, yield 46 %). ¹H NMR (400 MHz, DMSO-d6) δ 10.67 (s, 1H), 9.52 (d, *J =* 5.8 Hz, 1H), 8.79 (d, *J* = 7.7 Hz, 1H), 8.57 (s, 1H), 8.42 (d, *J =* 2.7 Hz, 1H), 8.26 (d, *J =* 5.4 Hz, 1H), 8.15 (d, *J =* 5.2 Hz, 1H), 6.88 (d, *J =* 7.8 Hz, 1H), 6.46 (d, *J =* 7.8 Hz, 1H), 5.24 (d, *J =* 27.1 Hz, 1H), 5.08 (s, 1H), 4.77 (d, *J =* 18.6 Hz, 1H), 4.56 (s, 1H), 4.47 (s, 2H), 4.06 (t, *J =* 6.5 Hz, 2H), 3.81 (s, 2H), 3.74 (d, *J =* 7.6 Hz, 1H), 3.65 (s, 1H), 3.45 (d, *J =* 10.0 Hz, 1H), 3.24 (s, 2H), 2.73 (t, *J =* 6.6 Hz, 2H), 2.44 - 2.32 (m, 2H), 2.28 (s, 2H), 2.03 (t, *J* = 9.9 Hz, 1H), 2.00 - 1.91 (m, 1H). LCMS (ESI) calcd for C₃₁H₃₃F₃N₁₁O₄⁺ [M+H]⁺: 680.27; found, 680.2.

### Example 716: Preparation of 5-((1S,4S)-2-oxa-5-azabicyclo[2.2.1]heptan-5-yl)-N-(3-(difluoromethyl)-1-(1-((2-(2,4-dioxotetrahydropyrimidin-1(2H)-yl)pyridin-4-yl)methyl)piperidin-4-yl)-1H-pyrazol-4-yl)pyrazolo[1,5-a]pyrimidine-3-carboxamide (GT-07773)

According to the method of step 2 of Scheme I-1, the target compound (GT-07773) was prepared (white solid, 27 mg, yield 48 %). ¹H NMR (400 MHz, DMSO-d6) δ 10.63 (s, 1H), 9.53 (s, 1H), 8.79 (s, 1H), 8.52 (s, 1H), 8.43 (d, *J =* 2.8 Hz, 1H), 8.26 (s, 1H), 7.99 (s, 1H), 7.63 (d, *J =* 4.9 Hz, 1H), 4.79 (s, 1H), 4.54 (d, *J =* 11.0 Hz, 1H), 4.41 (s, 2H), 4.28 (s, 1H), 4.10 (t, *J =* 6.6 Hz, 3H), 3.80 (s, 2H), 3.59 (s, 1H), 3.48 (s, 2H), 3.17 (s, 2H), 2.72 (t, *J =* 6.4 Hz, 3H), 2.42 (d, *J =* 12.4 Hz, 2H), 2.26 (s, 2H), 2.03 (t, *J =* 10.1 Hz, 1H), 1.98 (s, 1H). LCMS (ESI) calcd for C₃₁H₃₄F₂N₁₁O₄⁺ [M+H]⁺: 662.28; found, 662.2.

### Example 717: Preparation of 5-((1R,4R)-2-oxa-5-azabicyclo[2.2.1]heptan-5-yl)-N-(3-(difluoromethyl)-1-(1-((5-(2,4-dioxotetrahydropyrimidin-1(2H)-yl)pyridin-3-yl)methyl)piperidin-4-yl)-1H-pyrazol-4-yl)pyrazolo[1,5-a]pyrimidine-3-carboxamide (GT-07774)

According to the method of step 2 of Scheme I-1, the target compound (GT-07774) was prepared (white solid, 30 mg, yield 53 %). ¹H NMR (400 MHz, DMSO-d6) δ 10.71 (s, 1H), 9.52 (d, *J =* 5.5 Hz, 1H), 8.88 (s, 1H), 8.78 (d, *J =* 7.5 Hz, 2H), 8.44 (s, 2H), 8.26 (d, *J =* 5.2 Hz, 1H), 4.77 (d, *J =* 17.1 Hz, 1H), 4.58 (s, 3H), 4.50 (s, 2H), 4.27 - 4.23 (m, 1H), 3.99 (t, *J =* 6.3 Hz, 2H), 3.80 (s, 1H), 3.64 (d, *J =* 10.1 Hz, 1H), 3.58 (d, *J =* 10.1 Hz, 2H), 3.18 (s, 2H), 2.78 (t, *J =* 6.3 Hz, 2H), 2.42 (d, *J =* 11.9 Hz, 2H), 2.27 (s, 2H), 2.03 (t, *J* = 9.7 Hz, 1H), 2.00 - 1.90 (m, 1H), 1.28 (m, *J* = 16.0, 6.7 Hz, 1H). LCMS (ESI) calcd for C₃₁H₃₄F₂N₁₁O₄⁺ [M+H]⁺: 662.28; found, 662.2.

### Example 718: Preparation of 5-((1R,4R)-2-oxa-5-azabicyclo[2.2.1]heptan-5-yl)-N-(3-(difluoromethyl)-1-(1-((5-(2,6-dioxopiperidin-3-yl)-2-fluoropyridin-3-yl)methyl)piperidin-4-yl)-1H-pyrazol-4-yl)pyrazolo[1,5-a]pyrimidine-3-carboxamide (GT-07775)

According to the method of step 2 of Scheme I-1, the target compound (GT-07775) was prepared (white solid, 23 mg, yield 40 %). ¹H NMR (400 MHz, DMSO-d6) δ 10.93 (s, 1H), 9.46 (s, 1H), 8.71 (d, *J =* 7.7 Hz, 1H), 8.35 (d, *J =* 2.5 Hz, 1H), 8.22 (s, 1H), 8.18 (s, 2H), 4.70 (d, *J =* 18.4 Hz, 1H), 4.49 (s, 1H), 4.33 (s, 2H), 4.00 (m, *J =* 12.4, 4.7 Hz, 1H), 3.73 (s, 2H), 3.67 (d, *J =* 7.7 Hz, 1H), 3.56 (d, *J =* 10.6 Hz, 2H), 3.16 (s, 3H), 2.67 (m, *J* = 12.5, 4.9 Hz, 1H), 2.57 (s, 1H), 2.38 - 2.24 (m, 3H), 2.18 (d, *J =* 13.5 Hz, 3H), 2.08 - 2.02 (m, 1H), 1.97 (d, *J =* 9.1 Hz, 1H), 1.91 (s, 2H). LCMS (ESI) calcd for C₃₂H₃₄F₃N₁₀O₄⁺ [M+H]⁺: 679.27; found, 679.2.

### Example 719: Preparation of 5-((1R,4R)-2-oxa-5-azabicyclo[2.2.1]heptan-5-yl)-N-(3-(difluoromethyl)-1-(1-((2-(2,6-dioxopiperidin-3-yl)pyridin-4-yl)methyl)piperidin-4-yl)-1H-pyrazol-4-yl)pyrazolo[1,5-a]pyrimidine-3-carboxamide (GT-07776)

According to the method of step 2 of Scheme I-1, the target compound (GT-07776) was prepared (white solid, 21 mg, yield 37 %). ¹H NMR (400 MHz, DMSO-d6) δ 10.96 (s, 1H), 9.53 (s, 1H), 9.08 (d, *J =* 82.8 Hz, 1H), 8.79 (d, *J =* 7.7 Hz, 1H), 8.68 (s, 1H), 8.43 (s, 1H), 8.27 (d, *J =* 3.8 Hz, 1H), 7.75 (s, 1H), 4.77 (d, *J =* 18.2 Hz, 1H), 4.57 (s, 1H), 4.42 (s, 1H), 4.13 (m , *J =* 9.7, 5.3 Hz, 1H), 4.01 (s, 1H), 3.95 (s, 2H), 3.85 - 3.83 (m, 1H), 3.81 (s, 2H), 3.75 (s, 1H), 3.60 (s, 2H), 3.51 (d, *J =* 12.3 Hz, 1H), 3.45 - 3.36 (m, 1H), 3.15 (s, 1H), 3.05 (d, *J =* 11.4 Hz, 1H), 2.80 - 2.63 (m, 1H), 2.59 - 2.54 (m, 1H), 2.43 (d, *J =* 11.5 Hz, 1H), 2.27 (s, 1H), 2.20 (s, 2H), 2.04 (t, *J =* 9.2 Hz, 1H), 1.99 (s, 1H). LCMS (ESI) calcd for C₃₂H₃₅F₂N₁₀O₄⁺ [M+H]⁺: 661.28; found, 661.2.

### Example 720: Preparation of 5-((1R,4R)-2-oxa-5-azabicyclo[2.2.1]heptan-5-yl)-N-(3-(difluoromethyl)-1-(1-((5-(2,6-dioxopiperidin-3-yl)pyridin-2-yl)methyl)piperidin-4-yl)-1H-pyrazol-4-yl)pyrazolo[1,5-a]pyrimidine-3-carboxamide (GT-07777)

According to the method of step 2 of Scheme I-1, the target compound (GT-07777) was prepared (white solid, 32 mg, yield 57 %). ¹H NMR (400 MHz, DMSO-d6) δ 10.97 (s, 1H), 9.53 (d, *J =* 6.1 Hz, 1H), 8.79 (d, *J* = 7.7 Hz, 1H), 8.60 (d, *J =* 2.0 Hz, 1H), 8.46 (d, *J =* 3.0 Hz, 1H), 8.26 (d, *J =* 5.5 Hz, 1H), 7.86 (d, *J =* 2.2 Hz, 1H), 7.75 (d, *J =* 8.1 Hz, 1H), 4.77 (d, *J =* 17.6 Hz, 1H), 4.60 (s, 1H), 4.52 (s, 2H), 4.09 (s, 3H), 4.05 (s, 3H), 3.81 (s, 1H), 3.60 (s, 1H), 3.54 (d, *J =* 11.9 Hz, 2H), 3.35 - 3.22 (m, 2H), 2.80 - 2.69 (m, 1H), 2.63 - 2.55 (m, 1H), 2.47 - 2.37 (m, 2H), 2.35 (d, *J =* 4.3 Hz, 1H), 2.31 (s, 2H), 2.05 (dd, *J =* 9.2, 4.2 Hz, 1H), 2.01 - 1.91 (m, 1H). LCMS (ESI) calcd for C₃₂H₃₅F₂N₁₀O₄⁺ [M+H]⁺: 661.28; found, 661.2.

### Example 721: Preparation of 5-((1R,4R)-2-oxa-5-azabicyclo[2.2.1]heptan-5-yl)-N-(3-(difluoromethyl)-1-(1-((5-(2,6-dioxopiperidin-3-yl)pyridin-3-yl)methyl)piperidin-4-yl)-1H-pyrazol-4-yl)pyrazolo[1,5-a]pyrimidine-3-carboxamide (GT-07778)

According to the method of step 2 of Scheme I-1, the target compound (GT-07778) was prepared (white solid, 28 mg, yield 50 %). ¹H NMR (400 MHz, DMSO-d6) δ 11.06 (s, 1H), 9.52 (d, *J =* 5.4 Hz, 1H), 8.97 (s, 1H), 8.79 (d, *J =* 8.6 Hz, 2H), 8.44 (d, *J =* 2.5 Hz, 2H), 8.26 (d, *J =* 5.3 Hz, 1H), 4.77 (d, *J =* 17.7 Hz, 1H), 4.58 (s, 2H), 4.51 (s, 3H), 3.97 (s, 1H), 3.80 (s, 2H), 3.59 (s, 2H), 3.56 - 3.45 (m, 2H), 3.17 (s, 2H), 2.83 - 2.72 (m, 1H), 2.64 (d, *J =* 17.0 Hz, 1H), 2.38 (dd, *J =* 12.1, 8.0 Hz, 3H), 2.27 (s, 2H), 2.17 (d, *J =* 4.7 Hz, 1H), 2.03 (t, *J* = 10.0 Hz, 1H), 1.98 (s, 1H). LCMS (ESI) calcd for C₃₂H₃₅F₂N₁₀O₄⁺ [M+H]⁺: 661.28; found, 661.2.

### Example 722: Preparation of 6-(5-cyano-1H-pyrrolo[2,3-b]pyridin-1-yl)-N-(1-((5-(2,4-dioxotetrahydropyrimidin-1(2H)-yl)-2-fluoropyridin-3-yl)methyl)piperidin-4-yl)-4-(isopropylamino)nicotinamide (GT-07779)

According to the method of step 2 of Scheme I-1, the target compound (GT-07779) was prepared (white solid, 46 mg, yield 76 %). ¹H NMR (400 MHz, DMSO-d6) δ 10.55 (s, 1H), 8.74 (s, 1H), 8.70 (d, *J =* 7.1 Hz, 1H), 8.62 (d, *J =* 2.0 Hz, 1H), 8.59 (s, 1H), 8.32 (s, 2H), 7.97 (s, 1H), 6.84 (d, *J =* 3.9 Hz, 1H), 4.75 (s, 1H), 4.29 (d, *J =* 3.5 Hz, 2H), 4.00 - 3.92 (m, 2H), 3.77 (s, 3H), 3.13 (s, 2H), 2.70 (s, 2H), 2.68 (d, *J =* 3.5 Hz, 1H), 1.96 (s, 2H), 1.23 (s, 3H), 1.22 (s, 4H). LCMS (ESI) calcd for C₃₂H₃₄FN₁₀O₃⁺ [M+H]⁺: 625.28; found, 625.2.

### Example 723: Preparation of 6-(5-cyano-1H-pyrrolo[2,3-b]pyridin-1-yl)-N-(1-((5-(2,4-dioxotetrahydropyrimidin-1(2H)-yl)pyrazin-2-yl)methyl)piperidin-4-yl)-4-(isopropylamino)nicotinamide (GT-07780)

According to the method of step 2 of Scheme I-1, the target compound (GT-07780) was prepared (white solid, 30 mg, yield 51 %). ¹H NMR (400 MHz, DMSO-d6) δ 10.75 (s, 1H), 9.09 (s, 1H), 8.75 (s, 1H), 8.69 (s, 1H), 8.62 (s, 2H), 8.58 (s, 1H), 8.45 (d, *J =* 4.0 Hz, 1H), 7.99 (s, 1H), 6.84 (d, *J =* 3.9 Hz, 1H), 4.39 (d, *J* = 3.4 Hz*,* 2H), 4.03 (t, *J =* 6.5 Hz, 2H), 3.73 (s, 2H), 3.45 (d, *J =* 11.5 Hz, 2H), 3.13 (d, *J =* 11.1 Hz, 1H), 2.68 (t, *J =* 6.4 Hz, 2H), 1.96 (s, 3H), 1.89 (d, *J =* 12.4 Hz, 1H), 1.22 (d, *J =* 6.3 Hz, 7H). LCMS (ESI) calcd for C₃₁H₃₄N₁₁O₃⁺ [M+H]⁺: 608.28; found, 608.2.

### Example 724: Preparation of N-(2-(1-((5-(2,4-dioxotetrahydropyrimidin-1(2H)-yl)-2-fluoropyridin-3-yl)methyl)piperidin-4-yl)-6-methoxy-2H-indazol-5-yl)-3-(trifluoromethyl)benzamide (GT-07781)

According to the method of step 2 of Scheme I-1, the target compound (GT-07781) was prepared (white solid, 42 mg, yield 71 %). ¹H NMR (400 MHz, DMSO-d6) δ 10.56 (s, 1H), 9.71 (s, 1H), 8.33 (s, 1H), 8.24 (d, *J* = 9.9 Hz, 2H), 8.20 (d, *J =* 8.1 Hz, 1H), 7.97 (s, 1H), 7.90 (d, *J =* 7.5 Hz, 1H), 7.71 (s, 1H), 7.03 (s, 1H), 4.67 (s, 1H), 4.37 (s, 2H), 3.85 (t, *J* = 6.5 Hz, 2H), 3.80 (s, 2H), 3.55 (d, *J* = 8.9 Hz, 2H), 3.27 (s, 2H), 2.71 (t, *J =* 6.5 Hz, 2H), 2.30 (s, 2H). LCMS (ESI) calcd for C₃₁H₂₉F₄N₇O₄⁺ [M+H]⁺: 640.22; found, 640.2.

### Example 725: Preparation of N-(2-(1-((5-(2,4-dioxotetrahydropyrimidin-1(2H)-yl)pyrazin-2-yl)methyl)piperidin-4-yl)-6-methoxy-2H-indazol-5-yl)-3-(trifluoromethyl)benzamide (GT-07782)

According to the method of step 2 of Scheme I-1, the target compound (GT-07782) was prepared (white solid, 39 mg, yield 67 %). ¹H NMR (400 MHz, DMSO-d6) δ 10.76 (s, 1H), 9.72 (s, 1H), 9.11 (s, 1H), 8.73 (s, 1H), 8.24 (d, *J =* 8.6 Hz, 2H), 7.97 (s, 1H), 7.90 (d, *J* = 7.7 Hz, 1H), 7.71 (s, 1H), 7.05 (s, 1H), 4.67 (t, *J* = 11.6 Hz, 1H), 4.47 (s, 2H), 4.03 (t, *J* = 6.5 Hz, 2H), 3.81 (d, *J* = 6.7 Hz, 3H), 3.25 (d, *J* = 9.6 Hz, 2H), 2.68 (t, *J* = 6.5 Hz, 2H), 2.50 (d, *J* = 22.4 Hz, 1H), 2.31 (s, 2H). LCMS (ESI) calcd for C₃₀H₃₀F₃N₈O₄⁺ [M+H]⁺: 623.23; found, 623.2.

### Example 726: Preparation of N-(2-(1-((5-(2,4-dioxotetrahydropyrimidin-1(2H)-yl)-2-fluoropyridin-3-yl)methyl)piperidin-4-yl)-6-methoxy-2H-indazol-5-yl)-6-(trifluoromethyl)picolinamide (GT-07799)

According to the method of step 2 of Scheme I-1, the target compound (GT-07799) was prepared (white solid, 38 mg, yield 64 %). ¹H NMR (400 MHz, DMSO-d6) δ 10.56 (s, 1H), 10.44 (s, 1H), 8.64 (s, 1H), 8.39 (d, *J =* 7.7 Hz, 1H), 8.34 (t, *J* = 7.7 Hz, 2H), 8.29 (s, 2H), 8.15 (d, *J* = 7.6 Hz, 1H), 7.12 (s, 1H), 4.37 (s, 2H), 3.93 (d, *J* = 9.7 Hz, 3H), 3.86 (t, *J* = 6.6 Hz, 2H), 3.54 (s, 2H), 3.27 (s, 2H), 2.71 (t, *J* = 6.5 Hz, 2H), 2.30 (s, 2H). LCMS (ESI) calcd for C₃₀H₂₉F₄N₈O₄⁺ [M+H]⁺: 641.22; found, 641.2.

### Example 727: Preparation of N-(2-(1-((5-(2,4-dioxotetrahydropyrimidin-1(2H)-yl)pyrazin-2-yl)methyl)piperidin-4-yl)-6-methoxy-2H-indazol-5-yl)-6-(trifluoromethyl)picolinamide (GT-07800)

According to the method of step 2 of Scheme I-1, the target compound (GT-07800) was prepared (white solid, 20 mg, yield 35 %). LCMS (ESI) calcd for C₂₉H₂₉F₃N₉O₄⁺ [M+H]⁺: 624.23; found, 624.1.

### Example 728: Preparation of N-(2-(1-((5-(2,4-dioxotetrahydropyrimidin-1(2H)-yl)-2-fluoropyridin-3-yl)methyl)piperidin-4-yl)-6-(2-hydroxypropan-2-yl)-2H-indazol-5-yl)-6-(trifluoromethyl)picolinamide (GT-07783)

According to the method of step 2 of Scheme I-1, the target compound (GT-07783) was prepared (white solid, 27 mg, yield 46 %). ¹H NMR (400 MHz, DMSO-d6) δ 12.32 (s, 1H), 10.56 (s, 1H), 8.67 (s, 1H), 8.37 (s, 1H), 8.34 (s, 1H), 8.32 (s, 1H), 8.30 (s, 1H), 8.10 (s, 1H), 7.54 (s, 1H), 4.79 - 4.68 (m, 1H), 4.37 (s, 2H), 3.86 (d, *J* = 6.5 Hz, 2H), 3.55 (d, *J* = 11.3 Hz, 2H), 3.27 (s, 2H), 2.71 (s, 2H), 2.58 - 2.48 (m, 2H), 2.29 (d, *J* = 12.1 Hz, 2H), 1.56 (d, *J* = 8.9 Hz, 6H). LCMS (ESI) calcd for C₃₂H₃₃F₄N₈O₄⁺ [M+H]⁺: 669.26; found, 669.1.

### Example 729: Preparation of N-(2-(1-((5-(2,4-dioxotetrahydropyrimidin-1(2H)-yl)pyrazin-2-yl)methyl)piperidin-4-yl)-6-(2-hydroxypropan-2-yl)-2H-indazol-5-yl)-6-(trifluoromethyl)picolinamide (GT-07785)

According to the method of step 2 of Scheme I-1, the target compound (GT-07785) was prepared (white solid, 27 mg, yield 46 %). ¹H NMR (400 MHz, DMSO-d6) δ 12.32 (s, 1H), 10.76 (s, 1H), 9.11 (s, 1H), 8.73 (s, 1H), 8.67 (s, 1H), 8.38 (d, *J =* 7.7 Hz, 1H), 8.31 (d, *J =* 5.6 Hz, 2H), 8.10 (d, *J =* 7.8 Hz, 1H), 7.54 (s, 1H), 4.73 (t, *J* = 11.6 Hz, 1H), 4.47 (s, 2H), 4.04 (t, *J =* 6.4 Hz, 2H), 3.59 (d, *J* = 11.8 Hz, 2H), 3.27 (s, 2H), 2.68 (t, *J =* 6.5 Hz, 2H), 2.53 (d, *J =* 20.1 Hz, 1H), 2.46 (s, 1H), 2.31 (s, 2H), 1.55 (s, 6H). LCMS (ESI) calcd for C₃₂H₃₃F₄N₈O₄⁺ [M+H]⁺: 669.26; found, 669.1.

### Example 730: Preparation of N-(2-(1-((5-(2,4-dioxotetrahydropyrimidin-1(2H)-yl)-2-fluoropyridin-3-yl)methyl)piperidin-4-yl)-5-(2-hydroxypropan-2-yl)benzo[d]thiazol-6-yl)-6-(trifluoromethyl)picolinamide (GT-07787)

According to the method of step 2 of Scheme I-1, the target compound (GT-07787) was prepared (white solid, 36 mg, yield 58 %). ¹H NMR (500 MHz, DMSO) δ 12.58 (s, 1H), 10.63 (s, 1H), 9.10 (s, 1H), 8.47 (d, *J =* 7.7 Hz, 1H), 8.40 (d, *J =* 5.7 Hz, 2H), 8.37 (s, 1H), 8.19 (d, *J =* 7.8 Hz, 1H), 7.93 (s, 1H), 4.41 (s, 2H), 3.93 (t, *J* = 6.7 Hz, 2H), 3.57 (d, *J* = 11.3 Hz, 2H), 3.44 (s, 1H), 3.25 (d, *J* = 11.3 Hz, 2H), 2.77 (t, *J =* 6.6 Hz, 2H), 2.32 (s, 2H), 2.24 (m, *J* = 23.4, 12.1 Hz, 2H), 1.65 (d, *J* = 11.9 Hz, 6H). LCMS (ESI) calcd for C₃₂H₃₂F₄N₇O₄S⁺ [M+H]⁺: 686.22; found, 686.1.

### Example 731: Preparation of N-(2-(1-((5-(2,4-dioxotetrahydropyrimidin-1(2H)-yl)pyrazin-2-yl)methyl)piperidin-4-yl)-5-(2-hydroxypropan-2-yl)benzo[d]thiazol-6-yl)-6-(trifluoromethyl)picolinamide (GT-07788)

According to the method of step 2 of Scheme I-1, the target compound (GT-07788) was prepared (white solid, 31 mg, yield 51 %). ¹H NMR (500 MHz, DMSO) δ 12.58 (s, 1H), 10.83 (s, 1H), 9.17 (d*, J =* 1.2 Hz, 1H), 9.11 (s, 1H), 8.82 (d, *J* = 1.2 Hz, 1H), 8.47 (d, *J* = 7.8 Hz, 1H), 8.39 (s, 1H), 8.20 (d, *J* = 7.8 Hz, 1H), 7.93 (s, 1H), 4.51 (d, *J* = 3.5 Hz, 2H), 4.11 (t, *J* = 6.6 Hz, 2H), 3.59 (s, 2H), 3.48 - 3.40 (m, 1H), 3.25 (dd, *J* = 21.7, 9.9 Hz, 2H), 2.75 (t, *J =* 6.5 Hz, 2H), 2.32 (s, 2H), 2.24 (dd, *J =* 23.2, 12.2 Hz, 2H), 1.65 (d, *J* = 9.5 Hz, 6H). LCMS (ESI) calcd for C₃₁H₃₂F₃N₈O₄S⁺ [M+H]⁺: 669.22; found, 669.1.

### Example 732: Preparation of N-(3-(difluoromethyl)-1-(1-((5-(2,4-dioxotetrahydropyrimidin-1(2H)-yl)-2-fluoropyridin-3-yl)methyl)piperidin-4-yl)-1H-pyrazol-4-yl)-5-morpholinopyrazolo[1,5-a]pyrimidine-3-carboxamide (GT-07789)

According to the method of step 2 of Scheme I-1, the target compound (GT-07789) was prepared (white solid, 41 mg, yield 70 %). ¹H NMR (500 MHz, DMSO) δ 10.63 (s, 1H), 9.42 (s, 1H), 8.83 (d, *J* = 7.9 Hz, 1H), 8.42 (s, 1H), 8.39 (s, 1H), 8.34 (d, *J* = 6.9 Hz, 1H), 8.29 (s, 1H), 6.91 (d, *J* = 7.9 Hz, 1H), 4.56 (s, 1H), 4.42 (s, 2H), 3.91 (t, *J* = 6.6 Hz, 2H), 3.79 (s, 4H), 3.72 (d, *J* = 4.7 Hz, 4H), 3.58 (d, *J* = 8.6 Hz, 2H), 3.36 - 3.33 (m, 1H), 3.26 (s, 2H), 2.77 (t, *J* = 6.6 Hz, 2H), 2.35 (dd, *J* = 14.0, 6.8 Hz, 2H), 2.28 (s, 2H). LCMS (ESI) calcd for C₃₀H₃₃F₃N₁₁O₄⁺ [M+H]⁺: 668.27; found, 668.3.

### Example 733: Preparation of N-(3-(difluoromethyl)-1-(1-((5-(2,4-dioxotetrahydropyrimidin-1(2H)-yl)pyrazin-2-yl)methyl)piperidin-4-yl)-1H-pyrazol-4-yl)-5-morpholinopyrazolo[1,5-a]pyrimidine-3-carboxamide (GT-07790)

According to the method of step 2 of Scheme I-1, the target compound (GT-07790) was prepared (white solid, 30 mg, yield 53 %). ¹H NMR (500 MHz, DMSO) δ 10.83 (s, 1H), 9.41 (s, 1H), 9.16 (s, 1H), 8.90 - 8.75 (m, 2H), 8.42 (s, 1H), 8.29 (s, 1H), 4.52 (d, *J* = 12.1 Hz, 2H), 4.10 (d, *J* = 6.3 Hz, 2H), 3.79 (s, 4H), 3.72 (s, 4H), 3.61 (d, *J* = 11.0 Hz, 3H), 3.26 (s, 2H), 2.75 (d, *J* = 6.2 Hz, 2H), 2.53 (s, 1H), 2.37 (d, *J* = 10.6 Hz, 2H), 2.28 (s, 2H). LCMS (ESI) calcd for C₂₉H₃₃F₂N₁₂O₄⁺ [M+H]⁺: 651.27; found, 651.2.

### Example 734: Preparation of 5-((1R,4R)-2-oxa-5-azabicyclo[2.2.1]heptan-5-yl)-N-(3-(difluoromethyl)-1-(1-((5-(2,4-dioxotetrahydropyrimidin-1(2H)-yl)-2-fluoropyridin-3-yl)methyl)piperidin-4-yl)-1H-pyrazol-4-yl)pyrazolo[1,5-a]pyrimidine-3-carboxamide (GT-07791)

According to the method of step 2 of Scheme I-1, the target compound (GT-07791) was prepared (white solid, 33 mg, yield 57 %). ¹H NMR (500 MHz, DMSO) δ 10.63 (s, 1H), 9.53 (t, *J* = 10.4 Hz, 1H), 8.79 (dd, *J* = 7.7, 1.4 Hz, 1H), 8.42 (d, *J* = 3.9 Hz, 1H), 8.40 (s, 2H), 8.26 (d, *J* = 6.8 Hz, 1H), 4.79 (t, *J =* 17.9 Hz, 1H), 4.56 (d, *J* = 3.1 Hz, 1H), 4.41 (s, 2H), 3.92 (t, *J* = 6.6 Hz, 2H), 3.80 (s, 1H), 3.59 (s, 3H), 3.46 (s, 2H), 3.43 - 3.36 (m, 2H), 3.25 (d, *J* = 8.4 Hz, 2H), 2.77 (t, *J* = 6.6 Hz, 2H), 2.43 - 2.33 (m, 2H), 2.27 (d, *J* = 11.7 Hz, 2H), 2.07 - 1.91 (m, 2H). LCMS (ESI) calcd for C₃₁H₃₃F₃N₁₁O₄⁺ [M+H]⁺: 680.27; found, 680.1.

### Example 735: Preparation of 5-((1R,4R)-2-oxa-5-azabicyclo[2.2.1]heptan-5-yl)-N-(3-(difluoromethyl)-1-(1-((5-(2,4-dioxotetrahydropyrimidin-1(2H)-yl)pyrazin-2-yl)methyl)piperidin-4-yl)-1H-pyrazol-4-yl)pyrazolo[1,5-a]pyrimidine-3-carboxamide (GT-07792)

According to the method of step 2 of Scheme I-1, the target compound (GT-07792) was prepared (white solid, 30 mg, yield 53 %). ¹H NMR (500 MHz, DMSO) δ 10.83 (s, 1H), 9.49 (t, *J* = 26.7 Hz, 1H), 9.17 (d, *J* = 1.1 Hz, 1H), 8.80 (d, *J* = 9.4 Hz, 2H), 8.43 (d, *J* = 3.7 Hz, 1H), 8.26 (d, *J* = 6.7 Hz, 1H), 4.77 (d, *J =* 21.3 Hz, 1H), 4.58 (dd, *J =* 19.7, 11.5 Hz, 1H), 4.51 (s, 2H), 4.10 (t, *J =* 6.5 Hz, 2H), 3.81 (s, 1H), 3.61 (d, *J* = 16.3 Hz, 4H), 3.57 (s, 1H), 3.52 - 3.48 (m, 1H), 3.45 (d, *J* = 9.9 Hz, 1H), 3.26 (d, *J* = 8.8 Hz, 2H), 2.75 (t, *J* = 6.6 Hz, 2H), 2.38 (dd, *J* = 22.7, 12.0 Hz, 2H), 2.28 (d, *J* = 11.9 Hz, 2H), 2.08 - 1.90 (m, 2H). LCMS (ESI) calcd for C₃₀H₃₃F₂N₁₂O₄⁺ [M+H]⁺: 663.27; found, 663.2.

### Example 736: Preparation of N-(2-(1-((5-(2,6-dioxopiperidin-3-yl)-2-fluoropyridin-3-yl)methyl)piperidin-4-yl)-6-(2-hydroxypropan-2-yl)-2H-indazol-5-yl)-6-(trifluoromethyl)picolinamide (GT-07793)

According to the method of step 2 of Scheme I-1, the target compound (GT-07793) was prepared (white solid, 16 mg, yield 27 %). ¹H NMR (500 MHz, DMSO) δ 12.39 (s, 1H), 11.01 (s, 1H), 8.74 (s, 1H), 8.45 (d, *J* = 7.7 Hz, 1H), 8.38 (t, *J* = 3.8 Hz, 2H), 8.30 (d, *J* = 9.2 Hz, 1H), 8.27 (s, 1H), 8.17 (d, *J* = 7.8 Hz, 1H), 7.60 (s, 1H), 4.79 (t, *J =* 11.7 Hz, 1H), 4.43 (s, 2H), 4.07 (dt, *J =* 14.4, 7.2 Hz, 1H), 3.87 (s, 1H), 3.62 (d, *J* = 11.2 Hz, 1H), 3.56 (d, *J =* 11.6 Hz, 1H), 3.30 (d, *J* = 9.4 Hz, 2H), 2.81 - 2.72 (m, 1H), 2.64 (d, *J* = 3.2 Hz, 1H), 2.62 - 2.58 (m, 1H), 2.58 - 2.52 (m, 1H), 2.35 (d, *J* = 12.9 Hz, 2H), 2.32 - 2.27 (m, 1H), 2.16 - 2.11 (m, 1H), 1.62 (s, 6H). LCMS (ESI) calcd for C₃₃H₃₄F₄N₇O₄⁺ [M+H]⁺: 668.26; found, 668.2.

### Example 737: Preparation of (R)-N-(1-(4-(((2-(2,4-dioxotetrahydropyrimidin-1(2H)-yl)-5-fluoropyridin-4-yl)methyl)amino)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07794)

According to the method of step 2 of Scheme I-1, the target compound (GT-07794) was prepared (white solid, 40 mg, yield 62 %). ¹H NMR (500 MHz, DMSO) δ 8.35 (s, 2H), 8.29 (d, J = 8.2 Hz, 1H), 8.10 (s, 1H), 7.91 (d, J = 8.2 Hz, 1H), 7.75 (d, J = 0.4 Hz, 1H), 7.13 (s, 1H), 7.06 (d, J = 8.6 Hz, 2H), 6.55 (d, J = 8.6 Hz, 2H), 5.30 - 5.26 (m, 1H), 5.12 (s, 2H), 4.37 (s, 3H), 3.96 (s, 2H), 2.65 (s, 2H), 1.42 (d, J = 6.5 Hz, 4H). LCMS (ESI) calcd for C₃₂H₃₀FN₁₀O₄⁺ [M+H]⁺: 637.24; found, 637.3.

### Example 738: Preparation of (R)-N-(1-(4-(((2-(2,4-dioxotetrahydropyrimidin-1(2H)-yl)pyridin-4-yl)methyl)amino)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07795)

According to the method of step 2 of Scheme I-1, the target compound (GT-07795) was prepared (white solid, 31 mg, yield 49 %). ¹H NMR (500 MHz, DMSO) δ 8.35 (s, 1H), 8.11 (s, 1H), 7.92 (s, 1H), 7.78 (s, 1H), 7.76 (s, 1H), 7.39 (s, 1H), 7.22 (d, *J =* 5.2 Hz, 1H), 7.13 (s, 1H), 7.08 (d, *J* = 8.5 Hz, 2H), 6.64 (d, *J* = 8.3 Hz, 2H), 5.14 (s, 3H), 4.37 (s, 2H), 4.03 (s, 2H), 2.74 - 2.70 (m, 1H), 2.67 (s, 2H), 1.43 - 1.41 (m, 4H). LCMS (ESI) calcd for C₃₂H₃₁N₁₀O₄⁺ [M+H]⁺: 619.25; found, 619.3.

### Example 739: Preparation of (R)-N-(1-(4-(((5-(2,4-dioxotetrahydropyrimidin-1(2H)-yl)pyridin-3-yl)methyl)amino)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07796)

According to the method of step 2 of Scheme I-1, the target compound (GT-07796) was prepared (white solid, 24 mg, yield 38 %). ¹H NMR (500 MHz, DMSO) δ 10.76 (s, 1H), 10.64 (s, 1H), 8.84 (d, *J* = 2.1 Hz, 1H), 8.65 (s, 1H), 8.50 (s, 1H), 8.37 (d, *J* = 4.8 Hz, 1H), 8.12 (s, 1H), 7.76 (s, 1H), 7.39 (d, *J* = 1.1 Hz, 1H), 7.13 (s, 1H), 7.11 (d, *J* = 8.5 Hz, 2H), 6.70 (d, *J* = 8.4 Hz, 2H), 5.39 (s, 1H), 5.33 - 5.26 (m, 2H), 5.15 (s, 2H), 4.51 (s, 2H), 3.95 (s, 1H), 3.89 (d, *J* = 4.2 Hz, 1H), 2.76 (s, 1H), 1.43 (d, *J* = 3.1 Hz, 2H), 1.42 (d, *J* = 3.4 Hz, 2H). LCMS (ESI) calcd for C₃₂H₃₁N₁₀O₄⁺ [M+H]⁺: 619.25; found, 619.2.

### Example 740: Preparation of (R)-N-(1-(4-(4-((2-(2,4-dioxotetrahydropyrimidin-1(2H)-yl)-5-fluoropyridin-4-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07797)

According to the method of step 2 of Scheme I-1, the target compound (GT-07797) was prepared (white solid, 28 mg, yield 49 %). ¹H NMR (500 MHz, DMSO) δ 10.64 (d, *J* = 10.1 Hz, 2H), 8.56 (s, 1H), 8.35 (d, *J* = 4.9 Hz, 1H), 8.30 (d, *J =* 8.2 Hz, 1H), 8.17 (s, 1H), 8.11 (d, *J =* 5.2 Hz, 1H), 7.90 (d, *J =* 8.2 Hz, 1H), 7.76 (s, 1H), 7.23 (d, *J* = 8.7 Hz, 2H), 7.13 (s, 1H), 6.98 (d, *J* = 8.7 Hz, 2H), 5.28 (dd, *J* = 11.3, 6.0 Hz, 1H), 5.23 (s, 2H), 4.51 (s, 2H), 4.03 (d, *J* = 6.6 Hz, 2H), 3.90 (dd, *J* = 12.9, 4.4 Hz, 2H), 3.48 (dd, *J* = 12.0, 5.2 Hz, 2H), 3.26 (s, 2H), 3.11 (s, 2H), 2.70 (d, *J* = 6.6 Hz, 2H), 1.42 (d, *J* = 6.6 Hz, 3H). LCMS (ESI) calcd for C₃₆H₃₇FN₁₁O₄⁺ [M+H]⁺: 706.30; found, 706.3.

### Example 741: Preparation of (R)-N-(1-(4-(4-((2-(2,4-dioxotetrahydropyrimidin-1(2H)-yl)pyridin-4-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07798)

According to the method of step 2 of Scheme I-1, the target compound (GT-07798) was prepared (white solid, 37 mg, yield 66 %). ¹H NMR (500 MHz, DMSO) δ 10.63 (d, *J =* 9.1 Hz, 2H), 8.51 (d, *J =* 5.1 Hz, 1H), 8.36 (d, *J* = 4.9 Hz, 1H), 8.17 (s, 1H), 7.97 (s, 1H), 7.77 (s, 1H), 7.59 (d, *J =* 5.0 Hz, 1H), 7.24 (s, 1H), 7.22 (s, 1H), 7.13 (s, 1H), 6.97 (d, *J =* 8.7 Hz, 2H), 5.32 - 5.27 (m, 1H), 5.23 (s, 2H), 4.81 (s, 1H), 4.45 (s, 2H), 4.08 (d, *J =* 6.3 Hz, 2H), 3.81 (d, *J =* 9.9 Hz, 2H), 3.39 (d, *J =* 9.2 Hz, 2H), 3.18 (s, 2H), 2.71 (t, *J* = 6.6 Hz, 3H), 1.42 (d, *J =* 6.6 Hz, 3H). LCMS (ESI) calcd for C₃₆H₃₈N₁₁O₄⁺ [M+H]⁺: 688.31; found, 688.3.

### Example 742: Preparation of (R)-N-(1-(4-(4-((5-(2,4-dioxotetrahydropyrimidin-1(2H)-yl)pyridin-3-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07925)

According to the method of step 2 of Scheme I-1, the target compound (GT-07925) was prepared (white solid, 35 mg, yield 63 %). ¹H NMR (500 MHz, DMSO) δ 10.66 (d, *J =* 21.3 Hz, 2H), 8.84 (d, *J =* 2.2 Hz, 1H), 8.71 (s, 1H), 8.36 (s, 2H), 8.30 (d, *J =* 8.2 Hz, 1H), 8.17 (s, 1H), 7.91 (d, *J =* 8.2 Hz, 1H), 7.78 (s, 1H), 7.23 (d, *J =* 8.6 Hz, 2H), 7.13 (s, 1H), 6.98 (d, *J =* 8.7 Hz, 2H), 5.32 - 5.26 (m, 1H), 5.23 (s, 2H), 4.51 (s, 3H), 3.97 (s, 2H), 3.90 (dd, *J =* 12.9, 4.3 Hz, 1H), 3.82 (d, *J =* 8.8 Hz, 2H), 3.48 (dd, *J =* 12.4, 5.1 Hz, 1H), 3.41 (s, 1H), 3.20 (s, 3H), 2.77 (s, 2H), 1.42 (d, *J* = 6.5 Hz, 3H). LCMS (ESI) calcd for C₃₆H₃₈N₁₁O₄⁺ [M+H]⁺: 688.31; found, 688.3.

### Example 743: Preparation of (9R)-N-(1-(4-(4-(4-(2,6-dioxopiperidin-3-yl)benzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07926)

According to the method of step 2 of Scheme I-1, the target compound (GT-07926) was prepared (white solid, 35 mg, yield 63 %). ¹H NMR (500 MHz, DMSO) δ 10.88 (s, 1H), 10.65 (s, 1H), 8.36 (d, *J =* 4.8 Hz, 1H), 8.30 (d, *J =* 8.2 Hz, 1H), 8.17 (s, 1H), 7.91 (d, *J =* 8.2 Hz, 1H), 7.78 (s, 1H), 7.62 (d, *J =* 8.1 Hz, 2H), 7.33 (d, *J =* 8.1 Hz, 2H), 7.23 (d, *J =* 8.7 Hz, 2H), 7.13 (s, 1H), 6.97 (d, *J =* 8.7 Hz, 2H), 5.32 - 5.27 (m, 1H), 5.23 (s, 2H), 4.35 (d, *J =* 4.3 Hz, 2H), 3.92 (d, *J =* 4.6 Hz, 2H), 3.80 (s, 2H), 3.48 (dd, *J =* 12.1, 5.2 Hz, 1H), 3.34 (d, *J =* 11.0 Hz, 2H), 3.20 (s, 2H), 3.13 (dd, *J =* 20.6, 9.9 Hz, 2H), 2.74 - 2.64 (m, 1H), 2.53 (m, *J* = 7.5, 3.0 Hz, 1H), 2.23 (m, *J* = 12.5, 4.4 Hz, 1H), 2.04 (m, *J* = 8.4, 4.4 Hz, 1H), 1.42 (d, *J* = 6.6 Hz, 3H). LCMS (ESI) calcd for C₃₈H₄₀N₉O₄⁺ [M+H]⁺: 686.32; found, 686.2.

### Example 744: Preparation of (R)-N-(1-(4-(4-(4-(2,4-dioxotetrahydropyrimidin-1(2H)-yl)benzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07927)

According to the method of step 2 of Scheme I-1, the target compound (GT-07927) was prepared (white solid, 32 mg, yield 57 %). ¹H NMR (500 MHz, DMSO) δ 10.65 (s, 1H), 10.45 (s, 1H), 8.36 (d, *J =* 4.8 Hz, 1H), 8.30 (d, *J =* 8.2 Hz, 1H), 8.17 (s, 1H), 7.91 (d, *J =* 8.2 Hz, 1H), 7.79 (s, 1H), 7.67 (d, *J =* 8.5 Hz, 2H), 7.43 (d, *J =* 8.5 Hz, 2H), 7.23 (d, *J =* 8.7 Hz, 2H), 7.14 (s, 1H), 6.97 (d, *J =* 8.7 Hz, 2H), 5.32 - 5.28 (m, 1H), 5.23 (s, 2H), 4.36 (d, *J =* 3.9 Hz, 2H), 3.90 (dd, *J =* 12.9, 4.4 Hz, 1H), 3.83 (t, *J =* 6.6 Hz, 3H), 3.80 (s, 1H), 3.48 (dd, *J =* 12.2, 5.2 Hz, 1H), 3.34 (s, 2H), 3.21 (s, 2H), 3.17 - 3.08 (m, 2H), 2.72 (t, *J =* 6.6 Hz, 2H), 1.42 (d, *J =* 6.6 Hz, 3H). LCMS (ESI) calcd for C₃₇H₃₉N₁₀O₄⁺ [M+H]⁺: 687.32; found, 687.2.

### Example 745: Preparation of (R)-N-(1-(4-(4-(3-(2,4-dioxotetrahydropyrimidin-1(2H)-yl)benzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07928)

According to the method of step 2 of Scheme I-1, the target compound (GT-07928) was prepared (white solid, 34 mg, yield 61 %). ¹H NMR (500 MHz, DMSO) δ 10.63 (s, 1H), 8.35 (d, *J =* 4.9 Hz, 1H), 8.30 (d, *J* = 8.2 Hz, 1H), 8.17 (s, 1H), 7.91 (d, *J =* 8.2 Hz, 1H), 7.77 (s, 1H), 7.62 (s, 1H), 7.47 (s, 3H), 7.23 (d, *J* = 8.7 Hz, 2H), 7.13 (s, 1H), 6.97 (d, *J =* 8.8 Hz, 2H), 5.31 - 5.27 (m, 1H), 5.23 (s, 2H), 4.38 (d, *J =* 4.5 Hz, 2H), 3.92 (d, *J =* 4.5 Hz, 1H), 3.89 (d, *J =* 4.5 Hz, 1H), 3.88 (s, 1H), 3.80 (s, 2H), 3.48 (dd, *J =* 12.0, 5.2 Hz, 1H), 3.37 (d, *J =* 10.4 Hz, 2H), 3.19 (d, *J =* 12.5 Hz, 1H), 3.13 (dd, *J =* 19.3, 8.0 Hz, 3H), 2.72 (s, 2H), 1.42 (d, *J =* 6.6 Hz, 3H). LCMS (ESI) calcd for C₃₇H₃₉N₁₀O₄⁺ [M+H]⁺: 687.32; found, 687.2.

### Example 746: Preparation of (R)-N-(1-(4-(4-((5-(2,4-dioxotetrahydropyrimidin-1(2H)-yl)pyridin-2-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07929)

According to the method of step 2 of Scheme I-1, the target compound (GT-07929) was prepared (white solid, 34 mg, yield 61 %). ¹H NMR (500 MHz, DMSO) δ 10.65 (s, 1H), 10.59 (s, 1H), 8.70 (d, *J =* 2.5 Hz, 1H), 8.36 (d, *J =* 4.8 Hz, 1H), 8.30 (d, *J =* 8.2 Hz, 1H), 8.18 (s, 1H), 7.91 (d, *J =* 8.2 Hz, 2H), 7.78 (s, 1H), 7.76 (s, 1H), 7.23 (d, *J =* 8.7 Hz, 2H), 7.13 (s, 1H), 6.98 (d, *J =* 8.7 Hz, 2H), 5.31 - 5.27 (m, 1H), 5.23 (s, 2H), 4.53 (s, 2H), 4.09 (s, 1H), 3.92 (d, *J =* 4.4 Hz, 1H), 3.89 (t, *J =* 6.6 Hz, 3H), 3.53 (s, 1H), 3.48 (dd, *J* = 12.0, 5.2 Hz, 2H), 3.37 (s, 4H), 2.75 (s, 2H), 1.42 (d, *J =* 6.6 Hz, 3H). LCMS (ESI) calcd for C₃₆H₃₈N₁₁O₄⁺ [M+H]⁺: 688.31; found, 688.3.

### Example 747: Preparation of (R)-N-(1-(4-(4-((6-(2,4-dioxotetrahydropyrimidin-1(2H)-yl)pyridin-3-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07930)

According to the method of step 2 of Scheme I-1, the target compound (GT-07930) was prepared (white solid, 33 mg, yield 59 %). ¹H NMR (500 MHz, DMSO) δ 10.64 (s, 1H), 10.62 (s, 1H), 8.61 (d, *J =* 2.0 Hz, 1H), 8.35 (d, *J =* 4.9 Hz, 1H), 8.30 (d, *J =* 8.2 Hz, 1H), 8.17 (s, 1H), 8.09 (s, 1H), 7.91 (d, *J =* 8.2 Hz, 1H), 7.86 (d, *J =* 8.6 Hz, 1H), 7.77 (s, 1H), 7.23 (d, *J =* 8.7 Hz, 2H), 7.13 (s, 1H), 6.98 (d, *J =* 8.7 Hz, 2H), 5.29 (dd, *J =* 11.0, 5.6 Hz, 1H), 5.23 (s, 2H), 4.40 (d, *J =* 3.0 Hz, 2H), 4.09 (t, *J =* 6.6 Hz, 3H), 3.81 (s, 2H), 3.48 (dd, *J =* 12.1, 5.3 Hz, 1H), 3.37 (d, *J =* 9.9 Hz, 2H), 3.17 (d, *J =* 11.3 Hz, 4H), 2.70 (t, *J =* 6.6 Hz, 2H), 1.42 (d, *J =* 6.6 Hz, 3H). LCMS (ESI) calcd for C₃₆H₃₈N₁₁O₄⁺ [M+H]⁺: 688.31; found, 688.3.

### Example 748: Preparation of (9R)-N-(1-(4-(4-(3-((2,6-dioxopiperidin-3-yl)amino)benzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07931)

According to the method of step 2 of Scheme I-1, the target compound (GT-07931) was prepared (white solid, 37 mg, yield 65 %). ¹H NMR (500 MHz, DMSO) δ 10.85 (s, 1H), 10.65 (s, 1H), 8.36 (d, *J =* 4.6 Hz, 1H), 8.30 (d, *J =* 8.2 Hz, 1H), 8.17 (s, 1H), 7.91 (d, *J =* 8.2 Hz, 1H), 7.78 (s, 1H), 7.23 (d, *J =* 8.5 Hz, 2H), 7.14 (d, *J* = 9.2 Hz, 2H), 7.03 (s, 1H), 6.97 (d, *J =* 8.6 Hz, 2H), 6.80 (d, *J =* 6.7 Hz, 2H), 5.37 - 5.26 (m, 1H), 5.23 (s, 2H), 4.40 (dd, *J =* 11.7, 4.7 Hz, 2H), 4.22 (s, 2H), 3.90 (dd, *J =* 12.8, 4.2 Hz, 1H), 3.80 (d, *J =* 12.5 Hz, 2H), 3.48 (dd, *J =* 12.6, 4.8 Hz, 1H), 3.33 (s, 2H), 3.23 (s, 2H), 3.15 - 3.04 (m, 2H), 2.63 - 2.55 (m, 1H), 2.21 - 2.14 (m, 1H), 1.90 (m, *J* = 12.4, 4.3 Hz, 1H), 1.42 (d, *J* = 6.5 Hz, 3H). LCMS (ESI) calcd for C₃₈H₄₁N₁₀O₄⁺ [M+H]⁺: 701.33; found, 701.3.

### Example 749: Preparation of (9R)-N-(1-(4-(4-((5-(2,6-dioxopiperidin-3-yl)-2-fluoropyridin-3-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07932)

According to the method of step 2 of Scheme I-1, the target compound (GT-07932) was prepared (white solid, 19 mg, yield 33 %). ¹H NMR (500 MHz, DMSO) δ 10.99 (s, 1H), 10.63 (s, 1H), 8.35 (d, *J =* 4.8 Hz, 1H), 8.30 (d, *J =* 8.2 Hz, 1H), 8.25 (s, 1H), 8.17 (s, 1H), 7.91 (d, *J =* 8.2 Hz, 1H), 7.77 (s, 1H), 7.23 (d, *J* = 8.6 Hz, 2H), 7.13 (s, 1H), 6.98 (d, *J =* 8.7 Hz, 2H), 5.31 - 5.27 (m, 1H), 5.23 (s, 2H), 4.44 (s, 2H), 4.04 (s, 2H), 3.90 (dd, *J =* 12.9, 4.4 Hz, 2H), 3.81 (s, 2H), 3.48 (dd, *J =* 12.2, 5.1 Hz, 2H), 3.24 (s, 1H), 3.21 - 3.12 (m, 2H), 2.81 - 2.70 (m, 1H), 2.66 - 2.56 (m, 1H), 2.30 (m, *J* = 12.8, 4.3 Hz, 1H), 2.14 - 2.08 (m, 1H), 1.42 (d, *J =* 6.6 Hz, 3H). LCMS (ESI) calcd for C₃₇H₃₈FN₁₀O₄⁺ [M+H]⁺: 705.31; found, 705.2.

### Example 750: Preparation of (9R)-N-(1-(4-(4-((5-(2,6-dioxopiperidin-3-yl)-3-fluoropyridin-2-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07933)

According to the method of step 2 of Scheme I-1, the target compound (GT-07933) was prepared (white solid, 12 mg, yield 21 %). ¹H NMR (500 MHz, DMSO) δ 10.97 (s, 1H), 10.64 (s, 1H), 8.70 (d, *J =* 5.3 Hz, 1H), 8.36 (d, *J =* 4.9 Hz, 1H), 8.30 (d, *J =* 8.2 Hz, 1H), 8.17 (s, 1H), 7.91 (d, *J =* 8.2 Hz, 1H), 7.77 (s, 2H), 7.23 (d, *J =* 8.7 Hz, 2H), 7.13 (s, 1H), 6.98 (d, *J =* 8.7 Hz, 2H), 5.31 - 5.27 (m, 1H), 5.23 (s, 2H), 4.47 (s, 3H), 4.15 (dd, *J =* 10.1, 5.2 Hz, 1H), 3.90 (dd, *J =* 12.9, 4.4 Hz, 1H), 3.80 (s, 2H), 3.48 (dd, *J =* 12.2, 5.2 Hz, 1H), 3.36 (d, *J =* 10.5 Hz, 2H), 3.22 (d, *J =* 20.6 Hz, 4H), 2.68 (m, *J =* 20.0, 9.8, 4.5 Hz, 1H), 2.39 - 2.27 (m, 1H), 2.19-2.12 (m, *J =* 13.2, 5.2 Hz, 1H), 1.42 (d, *J =* 6.6 Hz, 3H). LCMS (ESI) calcd for C₃₇H₃₈FN₁₀O₄⁺ [M+H]⁺: 705.31; found, 705.2.

### Example 751: Preparation of (9R)-N-(1-(4-(4-((5-(2,6-dioxopiperidin-3-yl)pyridin-3-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07934)

According to the method of step 2 of Scheme I-1, the target compound (GT-07934) was prepared (white solid, 28 mg, yield 50 %). ¹H NMR (500 MHz, DMSO) δ 11.08 (s, 1H), 10.66 (s, 1H), 9.04 (d, *J =* 1.1 Hz, 1H), 8.88 (d, *J =* 1.5 Hz, 1H), 8.63 (s, 1H), 8.37 (d, *J =* 4.8 Hz, 1H), 8.30 (d, *J* = 8.2 Hz, 1H), 8.18 (s, 1H), 7.91 (d, *J =* 8.2 Hz, 1H), 7.79 (s, 1H), 7.22 (s, 2H), 7.14 (s, 1H), 7.00 (s, 2H), 5.30 (s, 1H), 5.24 (s, 2H), 4.59 (s, 3H), 4.23 (dd, *J* = 12.7, 4.8 Hz, 1H), 3.94 - 3.88 (m, 1H), 3.82 (s, 2H), 3.42 (s, 2H), 3.22 (s, 4H), 2.82 - 2.73 (m, 1H), 2.66 (s, 1H), 2.37-2.31 (m, *J =* 12.9, 4.3 Hz, 1H), 2.23 - 2.13 (m, 1H), 1.42 (d, *J =* 6.6 Hz, 3H). LCMS (ESI) calcd for C₃₇H₃₉N₁₀O₄⁺ [M+H]⁺: 687.32; found, 687.2.

### Example 752: Preparation of (R)-N-(1-(4-(4-((5-(2,4-dioxotetrahydropyrimidin-1(2H)-yl)-2-fluoropyridin-3-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07935)

According to the method of step 2 of Scheme I-1, the target compound (GT-07935) was prepared (white solid, 20 mg, yield 35 %). ¹H NMR (500 MHz, DMSO) δ 10.62 (d, *J =* 7.7 Hz, 2H), 8.38 (s, 1H), 8.35 (d, *J* = 5.3 Hz, 2H), 8.30 (d, *J =* 8.2 Hz, 1H), 8.17 (s, 1H), 7.91 (d, *J =* 8.2 Hz, 1H), 7.77 (s, 1H), 7.23 (d, *J =* 8.7 Hz, 2H), 7.13 (s, 1H), 6.98 (d, *J =* 8.7 Hz, 2H), 5.31 - 5.26 (m, 1H), 5.23 (s, 2H), 4.45 (s, 2H), 3.92 (d, *J* = 6.5 Hz, 2H), 3.90 (s, 1H), 3.84 (s, 1H), 3.82 (s, 1H), 3.48 (dd, *J =* 11.8, 5.2 Hz, 3H), 3.26 (s, 2H), 3.16 (d, *J* = 12.0 Hz, 2H), 2.75 (d, *J* = 6.4 Hz, 2H), 1.42 (d, *J* = 6.6 Hz, 3H). LCMS (ESI) calcd for C₃₇H₃₉N₁₀O₄⁺ [M+H]⁺: 706.30; found, 706.3.

### Example 753: Preparation of (R)-N-(1-(4-(4-((5-(2,4-dioxotetrahydropyrimidin-1(2H)-yl)pyrazin-2-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07936)

According to the method of step 2 of Scheme I-1, the target compound (GT-07936) was prepared (white solid, 20 mg, yield 35 %). ¹H NMR (500 MHz, DMSO) δ 10.82 (s, 1H), 10.63 (s, 1H), 9.15 (d, *J =* 1.4 Hz, 1H), 8.72 (d, *J =* 1.3 Hz, 1H), 8.35 (d, *J =* 4.8 Hz, 1H), 8.30 (d, *J =* 8.2 Hz, 1H), 8.17 (s, 1H), 7.91 (d, *J =* 8.2 Hz, 1H), 7.76 (s, 1H), 7.23 (d, *J =* 8.7 Hz, 2H), 7.13 (s, 1H), 6.98 (d, *J =* 8.8 Hz, 2H), 5.32 - 5.27 (m, 1H), 5.23 (s, 2H), 4.56 (s, 2H), 4.08 (t, *J =* 6.6 Hz, 2H), 3.91 (dd, *J =* 12.9, 4.4 Hz, 1H), 3.82 (d, *J =* 12.7 Hz, 2H), 3.56 - 3.49 (m, 2H), 3.48 (dd, *J* = 9.8, 3.2 Hz, 1H), 3.27 (s, 2H), 3.12 (t, *J =* 11.4 Hz, 2H), 2.74 (t, *J =* 6.6 Hz, 2H), 1.42 (d, *J =* 6.6 Hz, 3H). LCMS (ESI) calcd for C₃₆H₃₇FN₁₁O₄⁺ [M+H]⁺: 689.31; found, 689.3.

### Example 754: Preparation of (9R)-N-(1-(4-(4-((4-(2,6-dioxopiperidin-3-yl)benzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07937)

According to the method of step 2 of Scheme I-1, the target compound (GT-07937) was prepared (white solid, 28 mg, yield 51 %). ¹H NMR (500 MHz, DMSO) δ 10.88 (s, 1H), 10.67 (s, 1H), 8.37 (d, *J =* 4.8 Hz, 1H), 8.30 (d, *J =* 8.2 Hz, 1H), 8.22 (s, 1H), 7.91 (d, *J =* 8.2 Hz, 1H), 7.80 (s, 1H), 7.64 (d, *J =* 8.1 Hz, 2H), 7.33 (s, 1H), 7.31 (d, *J =* 3.8 Hz, 4H), 7.14 (s, 1H), 5.29 (s, 3H), 4.46 (dd, *J =* 13.0, 3.1 Hz, 1H), 4.20 (dd, *J =* 12.9, 7.0 Hz, 2H), 3.92 (td, *J =* 11.9, 4.6 Hz, 4H), 3.81 (s, 3H), 3.50 (d, *J =* 5.3 Hz, 2H), 3.06 (s, 2H), 2.59 (d, *J* = 4.7 Hz, 3H), 2.38 (d, *J =* 12.6 Hz, 1H), 2.33 - 2.27 (m, 1H), 2.16 (s, 1H), 2.08 - 2.00 (m, 1H), 1.43 (d, *J =* 6.5 Hz, 3H). LCMS (ESI) calcd for C₄₀H₄₄N₉O₄⁺ [M+H]⁺: 714.35; found, 714.3.

### Example 755: Preparation of (R)-N-(1-(4-(4-((4-(2,4-dioxotetrahydropyrimidin-1(2H)-yl)benzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07938)

According to the method of step 2 of Scheme I-1, the target compound (GT-07938) was prepared (white solid, 30 mg, yield 55 %). ¹H NMR (500 MHz, DMSO) δ 10.67 (s, 1H), 10.44 (s, 1H), 8.36 (t, *J =* 8.9 Hz, 1H), 8.30 (d, *J* = 8.2 Hz, 1H), 8.23 (s, 1H), 7.91 (d, *J* = 8.2 Hz, 1H), 7.80 (s, 1H), 7.70 (d, *J* = 8.5 Hz, 2H), 7.42 (d, *J =* 8.5 Hz, 2H), 7.30 (d, *J =* 10.9 Hz, 4H), 7.14 (s, 1H), 5.29 (s, 3H), 4.46 (dd, *J =* 13.0, 3.3 Hz, 1H), 4.27 - 4.17 (m, 2H), 3.91 (dd, *J =* 12.9, 4.3 Hz, 3H), 3.83 (t, *J =* 6.6 Hz, 5H), 3.48 (dd, *J =* 12.6, 4.8 Hz, 2H), 2.72 (t, *J =* 6.6 Hz, 2H), 2.60 (d, *J* = 4.7 Hz, 3H), 2.39 (d, *J* = 12.3 Hz, 1H), 2.31 (d, *J* = 7.8 Hz, 1H), 2.07 (s, 1H), 1.43 (d, *J =* 6.5 Hz, 3H). LCMS (ESI) calcd for C₃₉H₄₃N₁₀O₄⁺ [M+H]⁺: 715.35; found, 715.3.

### Example 756: Preparation of (R)-N-(1-(4-(4-((3-(2,4-dioxotetrahydropyrimidin-1(2H)-yl)benzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07939)

According to the method of step 2 of Scheme I-1, the target compound (GT-07939) was prepared (white solid, 29 mg, yield 53 %). ¹H NMR (500 MHz, DMSO) δ 10.66 (s, 1H), 10.45 (s, 1H), 8.37 (s, 1H), 8.30 (d, *J* = 8.2 Hz, 1H), 8.22 (s, 1H), 7.91 (d, *J* = 8.2 Hz, 1H), 7.79 (s, 1H), 7.67 (s, 1H), 7.55 - 7.50 (m, 1H), 7.48 - 7.43 (m, 2H), 7.30 (s, 4H), 7.14 (s, 1H), 5.29 (s, 3H), 4.47 (dd, *J =* 13.0, 3.5 Hz, 1H), 4.25 (dd, *J* = 13.0, 6.9 Hz, 2H), 3.93 - 3.89 (m, 1H), 3.87 (t, *J =* 6.7 Hz, 2H), 3.84 - 3.78 (m, 2H), 3.48 (dd, *J =* 12.0, 5.2 Hz, 2H), 3.05 (s, 2H), 2.72 (t, *J* = 6.8 Hz, 2H), 2.61 (d, *J* = 4.8 Hz, 3H), 2.40 - 2.25 (m, 2H), 2.15 (s, 1H), 1.43 (d, *J =* 6.6 Hz, 3H). LCMS (ESI) calcd for C₃₉H₄₃N₁₀O₄⁺ [M+H]⁺: 715.35; found, 715.3.

### Example 757: Preparation of (R)-N-(1-(4-(4-(((5-(2,4-dioxotetrahydropyrimidin-1(2H)-yl)pyridin-2-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07940)

According to the method of step 2 of Scheme I-1, the target compound (GT-07940) was prepared (white solid, 31 mg, yield 56 %). ¹H NMR (500 MHz, DMSO) δ 10.68 (s, 1H), 10.59 (s, 1H), 8.70 (d, *J =* 2.6 Hz, 1H), 8.38 (t, *J* = 7.0 Hz, 1H), 8.30 (d, *J* = 8.2 Hz, 1H), 8.25 (s, 1H), 7.95 - 7.89 (m, 2H), 7.81 (s, 2H), 7.46 (s, 2H), 7.35 (s, 2H), 7.14 (s, 1H), 5.33 (s, 3H), 4.52 (s, 3H), 3.89 (t, *J =* 6.6 Hz, 3H), 3.79 (d, *J =* 11.7 Hz, 2H), 3.61 (dd, *J* = 8.5, 4.5 Hz, 1H), 3.49 (dd, *J* = 12.0, 5.2 Hz, 1H), 3.24 (s, 1H), 2.78 - 2.74 (m, 4H), 2.36 (d, *J* = 10.5 Hz, 2H), 2.31 (s, 1H), 1.43 (d, *J* = 6.6 Hz, 3H), 1.31 (s, 1H), 1.28 (d, *J* = 6.5 Hz, 1H). LCMS (ESI) calcd for C₃₈H₄₂N₁₁O₄⁺ [M+H]⁺: 716.34; found, 716.3.

### Example 758: Preparation of (R)-N-(1-(4-(4-(((6-(2,4-dioxotetrahydropyrimidin-1(2H)-yl)pyridin-3-yl)methyl)(methyl)amino)piperidin-1-yl)benzyl)-1H-pyrazol-4-yl)-9-methyl-6,7,8,9-tetrahydropyrido[3',2':4,5]pyrrolo[1,2-a]pyrazine-2-carboxamide (GT-07941)

According to the method of step 2 of Scheme I-1, the target compound (GT-07941) was prepared (white solid, 33 mg, yield 60 %). ¹H NMR (500 MHz, DMSO) δ 10.69 (s, 1H), 10.62 (s, 1H), 8.68 (d, *J =* 2.1 Hz, 1H), 8.38 (t, *J =* 8.1 Hz, 1H), 8.30 (d, *J =* 8.2 Hz, 1H), 8.26 (s, 1H), 8.19 (dd, *J =* 8.7, 2.3 Hz, 1H), 7.92 (d, *J =* 8.2 Hz, 1H), 7.86 (d, *J =* 8.7 Hz, 1H), 7.81 (s, 1H), 7.51 (s, 2H), 7.36 (d, *J =* 8.4 Hz, 2H), 7.14 (s, 1H), 5.34 (s, 3H), 4.48 (dd, *J =* 13.1, 3.0 Hz, 2H), 4.33 (dd, *J =* 13.1, 6.4 Hz, 2H), 4.11 (t, *J* = 6.6 Hz, 2H), 3.91 (dd, *J =* 12.9, 4.3 Hz, 1H), 3.77 (d, *J =* 8.8 Hz, 1H), 3.58 (s, 1H), 3.49 (dd, *J =* 12.2, 5.2 Hz, 1H), 3.32 (s, 2H), 2.70 (d, *J =* 6.6 Hz, 2H), 2.63 (d, *J =* 4.4 Hz, 3H), 2.45 (d, *J =* 10.7 Hz, 1H), 2.37 (s, 2H), 1.43 (d, *J =* 6.6 Hz, 3H). LCMS (ESI) calcd for C₃₈H₄₂N₁₁O₄⁺ [M+H]⁺: 716.34; found, 716.3.

### Example 759: Preparation of (9R)-N-(1-(4-(4-((3-((2,6-dioxopiperidin-3-yl)amino)benzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07942)

According to the method of step 2 of Scheme I-1, the target compound (GT-07942) was prepared (white solid, 26 mg, yield 47 %). ¹H NMR (500 MHz, DMSO) δ 10.85 (d, *J* = 1.9 Hz, 1H), 10.67 (s, 1H), 8.37 (d, *J* = 4.8 Hz, 1H), 8.30 (d, *J* = 8.2 Hz, 1H), 8.24 (s, 1H), 7.91 (d, *J =* 8.2 Hz, 1H), 7.80 (s, 1H), 7.58 - 7.37 (m, 2H), 7.33 (d, *J =* 8.3 Hz, 2H), 7.14 (s, 1H), 7.08 (s, 1H), 6.83 (s, 1H), 6.80 (dd, *J =* 8.2, 1.6 Hz, 1H), 5.31 (s, 3H), 4.42 (dd, *J =* 11.5, 4.5 Hz, 2H), 4.35 - 4.28 (m, 2H), 4.13 (dd, *J =* 13.2, 6.6 Hz, 1H), 3.91 (dd, *J* = 12.8, 4.3 Hz, 1H), 3.77 (s, 2H), 3.49 (d, *J* = 1.1 Hz, 2H), 3.33 - 2.97 (m, 2H), 2.89 - 2.77 (m, 1H), 2.62 (d, *J* = 4.7 Hz, 3H), 2.38 (d, *J* = 14.9 Hz, 1H), 2.27 (s, 2H), 2.18 (dd, *J* = 9.0, 3.9 Hz, 1H), 1.89-1.82 (m, *J =* 12.6, 4.5 Hz, 1H), 1.43 (d, *J* = 6.6 Hz, 3H). LCMS (ESI) calcd for C₄₀H₄₅N₁₀O₄⁺ [M+H]⁺: 729.36; found, 729.3.

### Example 760: Preparation of (R)-N-(1-(4-(4-(((2-(2,4-dioxotetrahydropyrimidin-1(2H)-yl)-5-fluoropyridin-4-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07943)

According to the method of step 2 of Scheme I-1, the target compound (GT-07943) was prepared (white solid, 36 mg, yield 64 %). ¹H NMR (500 MHz, DMSO) δ 10.65 (s, 2H), 8.55 (s, 1H), 8.36 (d, *J* = 4.8 Hz, 1H), 8.30 (d, *J* = 8.2 Hz, 1H), 8.21 (s, 1H), 8.09 (d, *J* = 5.2 Hz, 1H), 7.91 (d, *J* = 8.2 Hz, 1H), 7.78 (s, 1H), 7.27 (s, 2H), 7.14 (s, 1H), 5.33 - 5.29 (m, 1H), 5.27 (s, 2H), 4.62 - 4.57 (m, 1H), 4.36 (d, *J* = 11.1 Hz, 2H), 4.03 (d, *J* = 6.8 Hz, 3H), 3.91 (dd, *J* = 12.9, 4.4 Hz, 1H), 3.85 (d, *J* = 11.6 Hz, 2H), 3.55 (s, 1H), 3.51 - 3.45 (m, 1H), 2.72 (s, 3H), 2.71 (s, 1H), 2.70 (d, *J* = 4.4 Hz, 1H), 2.34 (d, *J* = 18.7 Hz, 1H), 2.22 (s, 1H), 2.11 (s, 1H), 2.08 (s, 1H), 1.43 (d, *J* = 6.6 Hz, 3H). LCMS (ESI) calcd for C₃₈H₄₁FN₁₁O₄⁺ [M+H]⁺: 734.33; found, 734.3.

### Example 761: Preparation of (R)-N-(1-(4-(4-(((2-(2,4-dioxotetrahydropyrimidin-1(2H)-yl)pyridin-4-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07944)

According to the method of step 2 of Scheme I-1, the target compound (GT-07944) was prepared (white solid, 39 mg, yield 71 %). ¹H NMR (500 MHz, DMSO) δ 10.70 (s, 1H), 10.64 (s, 1H), 8.52 (d, *J =* 5.1 Hz, 1H), 8.38 (d, *J* = 4.7 Hz, 1H), 8.30 (d, *J* = 8.2 Hz, 1H), 8.27 (s, 1H), 7.99 (s, 1H), 7.92 (d, *J* = 8.2 Hz, 1H), 7.82 (s, 1H), 7.70 (d, *J* = 4.4 Hz, 1H), 7.58 (s, 2H), 7.39 (s, 2H), 7.14 (s, 1H), 5.35 (s, 2H), 5.33 - 5.29 (m, 1H), 4.82 (s, 1H), 4.51 (s, 1H), 4.43 (s, 1H), 4.08 (s, 2H), 3.91 (dd, *J* = 12.8, 4.3 Hz, 1H), 3.76 (s, 2H), 3.49 (dd, *J* = 12.3, 5.0 Hz, 1H), 3.37 (s, 2H), 2.71 (d, *J =* 8.4 Hz, 2H), 2.67 (s, 3H), 2.44 (s, 2H), 2.37 (s, 2H), 1.43 (d, *J =* 6.5 Hz, 3H). LCMS (ESI) calcd for C₃₈H₄₂N₁₁O₄⁺ [M+H]⁺: 716.34; found, 716.3.

### Example 762: Preparation of (R)-N-(1-(4-(4-(((5-(2,4-dioxotetrahydropyrimidin-1(2H)-yl)pyridin-3-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07945)

According to the method of step 2 of Scheme I-1, the target compound (GT-07945) was prepared (white solid, 24 mg, yield 44 %). ¹H NMR (500 MHz, DMSO) δ 10.71 (s, 1H), 10.67 (s, 1H), 8.87 (d, *J* = 2.3 Hz, 1H), 8.80 (d, *J* = 1.3 Hz, 1H), 8.48 (s, 1H), 8.36 (d, *J* = 4.9 Hz, 1H), 8.30 (d, *J* = 8.2 Hz, 1H), 8.23 (s, 1H), 7.91 (d, *J* = 8.2 Hz, 1H), 7.80 (s, 1H), 7.34 (s, 1H), 7.31 (d, *J* = 7.8 Hz, 2H), 7.14 (s, 1H), 5.29 (d, *J =* 7.0 Hz, 3H), 4.43 (s, 2H), 3.99 (t, *J =* 6.6 Hz, 2H), 3.90 (s, 1H), 3.82 (s, 2H), 3.59 (s, 1H), 3.48 (dd, *J =* 12.2, 5.2 Hz, 1H), 3.14 (s, 2H), 2.77 (dd, *J* = 7.5, 6.1 Hz, 2H), 2.64 (s, 3H), 2.36 (dd, *J* = 13.1, 11.3 Hz, 2H), 2.21 (s, 2H), 1.43 (d, *J =* 6.6 Hz, 3H). LCMS (ESI) calcd for C₃₈H₄₂N₁₁O₄⁺ [M+H]⁺: 716.34; found, 716.3.

### Example 763: Preparation of (9R)-N-(1-(4-(4-(((5-(2,6-dioxopiperidin-3-yl)-2-fluoropyridin-3-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07946)

According to the method of step 2 of Scheme I-1, the target compound (GT-07946) was prepared (white solid, 24 mg, yield 43 %). ¹H NMR (500 MHz, DMSO) δ 10.99 (s, 1H), 10.66 (s, 1H), 8.36 (d, *J* = 4.8 Hz, 1H), 8.30 (d, *J* = 8.2 Hz, 2H), 8.24 (d, *J* = 1.7 Hz, 1H), 8.21 (s, 1H), 7.91 (d, *J* = 8.2 Hz, 1H), 7.79 (s, 1H), 7.28 (s, 2H), 7.25 (s, 1H), 7.14 (s, 1H), 5.28 (s, 3H), 4.59 - 4.49 (m, 1H), 4.33 - 4.22 (m, 2H), 4.08 - 4.04 (m, 2H), 4.01 - 3.98 (m, 1H), 3.91 (dd, *J* = 12.9, 4.3 Hz, 2H), 3.85 (d, *J* = 9.9 Hz, 2H), 3.48 (dd, *J =* 12.6, 4.7 Hz, 1H), 2.99 (d, *J* = 21.2 Hz, 2H), 2.69 (s, 1H), 2.68 - 2.61 (m, 2H), 2.35 (dd, *J* = 11.9, 3.2 Hz, 1H), 2.30 (dd, *J* = 12.9, 4.3 Hz, 1H), 2.27 - 2.21 (m, 1H), 2.09 (d, *J* = 6.4 Hz, 2H), 1.43 (d, *J* = 6.6 Hz, 3H). LCMS (ESI) calcd for C₃₉H₄₂FN₁₀O₄⁺ [M+H]⁺: 733.34; found, 733.3.

### Example 764: Preparation of (9R)-N-(1-(4-(4-(((2-(2,6-dioxopiperidin-3-yl)pyridin-4-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07947)

According to the method of step 2 of Scheme I-1, the target compound (GT-07947) was prepared (white solid, 10 mg, yield 18 %). ¹H NMR (500 MHz, DMSO) δ 10.66 (s, 1H), 8.70 (d, *J* = 5.2 Hz, 1H), 8.36 (d, *J* = 4.9 Hz, 1H), 8.30 (d, *J* = 8.2 Hz, 1H), 8.22 (s, 1H), 7.91 (d, *J* = 8.2 Hz, 1H), 7.86 (s, 1H), 7.83 (d, *J =* 4.5 Hz, 1H), 7.79 (s, 1H), 7.29 (s, 3H), 7.13 (s, 1H), 5.28 (s, 3H), 4.57 (d, *J* = 12.9 Hz, 3H), 4.36 (s, 1H), 4.16 (dd, *J* = 10.1, 5.1 Hz, 1H), 3.91 (dd, *J* = 12.9, 4.3 Hz, 1H), 3.82 (d, *J* = 10.5 Hz, 2H), 3.48 (dd, *J* = 12.0, 5.3 Hz, 2H), 3.03 (s, 2H), 2.63 (s, 3H), 2.60 - 2.53 (m, 1H), 2.36 (dd, *J* = 5.5, 3.6 Hz, 2H), 2.30 (s, 1H), 2.22 - 2.18 (m, 1H), 2.14 (s, 1H), 1.42 (d, *J* = 6.5 Hz, 3H). LCMS (ESI) calcd for C₃₉H₄₃N₁₀O₄⁺ [M+H]⁺: 715.35; found, 715.3.

### Example 765: Preparation of (9R)-N-(1-(4-(4-(((5-(2,6-dioxopiperidin-3-yl)pyridin-3-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07948)

According to the method of step 2 of Scheme I-1, the target compound (GT-07948) was prepared (white solid, 18 mg, yield 33 %). ¹H NMR (500 MHz, DMSO) δ 10.67 (s, 1H), 9.10 (s, 1H), 8.87 (d, *J =* 1.4 Hz, 1H), 8.70 (s, 1H), 8.38 (t, *J =* 8.5 Hz, 1H), 8.30 (d, *J =* 8.2 Hz, 1H), 8.23 (s, 1H), 7.91 (d, *J =* 8.2 Hz, 1H), 7.80 (s, 1H), 7.35 (s, 1H), 7.32 (s, 2H), 7.14 (s, 1H), 5.30 (s, 3H), 4.67 (d, *J* = 13.0 Hz, 2H), 4.49 (s, 2H), 4.23 (dd, *J* = 12.7, 4.8 Hz, 1H), 3.91 (dd, *J =* 12.9, 4.4 Hz, 1H), 3.82 (s, 2H), 3.60 (s, 1H), 3.48 (dd, *J =* 12.1, 5.2 Hz, 1H), 2.81 - 2.73 (m, 1H), 2.68 - 2.57 (m, 4H), 2.37 (s, 3H), 2.19 (s, 3H), 1.43 (d, *J* = 6.6 Hz, 3H). LCMS (ESI) calcd for C₃₉H₄₃N₁₀O₄⁺ [M+H]⁺: 715.35; found, 715.3.

### Example 766: Preparation of (R)-N-(1-(4-(4-(((5-(2,4-dioxotetrahydropyrimidin-1(2H)-yl)-2-fluoropyridin-3-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07949)

According to the method of step 2 of Scheme I-1, the target compound (GT-07949) was prepared (white solid, 31 mg, yield 55 %). ¹H NMR (500 MHz, DMSO) δ 10.82 (s, 1H), 10.66 (s, 1H), 9.16 (d, *J =* 1.4 Hz, 1H), 8.78 (d, *J =* 1.4 Hz, 1H), 8.36 (d, *J* = 4.9 Hz, 1H), 8.30 (d, *J =* 8.2 Hz, 1H), 8.21 (s, 1H), 7.91 (d, *J =* 8.2 Hz, 1H), 7.80 - 7.77 (m, 1H), 7.26 (s, 2H), 7.22 (s, 1H), 7.14 (s, 1H), 5.27 (s, 3H), 4.61 (d, *J =* 13.3 Hz, 1H), 4.46 (d, *J =* 9.0 Hz, 1H), 4.09 (t, *J =* 6.7 Hz, 3H), 3.90 (s, 3H), 3.84 (d, *J* = 7.0 Hz, 3H), 3.51 - 3.46 (m, 1H), 2.75 (s, 2H), 2.73 (d, *J =* 6.6 Hz, 2H), 2.38 - 2.31 (m, 1H), 2.24 (d, *J =* 10.2 Hz, 1H), 2.08 (d, *J =* 14.3 Hz, 2H), 1.43 (d, *J =* 6.6 Hz, 3H). LCMS (ESI) calcd for C₃₈H₄₁FN₁₁O₄⁺ [M+H]⁺: 734.33; found, 734.3.

### Example 767: Preparation of (R)-N-(1-(4-(4-(((5-(2,4-dioxotetrahydropyrimidin-1(2H)-yl)pyrazin-2-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07950)

According to the method of step 2 of Scheme I-1, the target compound (GT-07950) was prepared (white solid, 38 mg, yield 69 %). ¹H NMR (500 MHz, DMSO) δ 10.66 (s, 1H), 10.62 (s, 1H), 8.42 - 8.39 (m, 1H), 8.38 (s, 1H), 8.36 (d, *J =* 4.9 Hz, 1H), 8.30 (d, *J =* 8.2 Hz, 1H), 8.22 (s, 1H), 7.91 (d, *J =* 8.2 Hz, 1H), 7.79 (s, 1H), 7.30 (s, 3H), 7.14 (s, 1H), 5.29 (s, 3H), 4.56 (d, *J =* 14.8 Hz, 1H), 4.32 - 4.28 (m, 2H), 3.91 (d, *J* = 6.9 Hz, 2H), 3.83 (s, 2H), 3.58 (s, 1H), 3.48 (dd, *J =* 12.2, 5.2 Hz, 1H), 3.08 (s, 2H), 2.75 (d, *J =* 6.6 Hz, 2H), 2.70 (s, 3H), 2.38 (d, *J* = 12.0 Hz, 1H), 2.27 (s, 1H), 2.13 (d, *J* = 30.8 Hz, 2H), 1.43 (d, *J* = 6.6 Hz, 3H). LCMS (ESI) calcd for C₃₇H₄₁N₁₂O₄⁺ [M+H]⁺: 717.34; found, 717.3.

### Example 768: Preparation of (9R)-N-(1-(4-(4-(4-(4-(2,6-dioxopiperidin-3-yl)benzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07951)

According to the method of step 2 of Scheme I-1, the target compound (GT-07951) was prepared (white solid, 34 mg, yield 64 %). ¹H NMR (400 MHz, DMSO-d6) δ 10.88 (s, 1H), 10.65 (s, 1H), 8.36 (d, *J =* 4.8 Hz, 1H), 8.30 (d, *J =* 8.2 Hz, 1H), 8.19 (s, 1H), 7.91 (d, *J =* 8.2 Hz, 1H), 7.78 (s, 1H), 7.63 (d, *J =* 8.0 Hz, 2H), 7.33 (d, *J =* 8.1 Hz, 2H), 7.25 (d, *J =* 8.1 Hz, 2H), 7.14 (s, 1H), 5.31 - 5.27 (m, 1H), 5.26 (s, 2H), 4.41 (s, 2H), 3.94 - 3.88 (m, 4H), 3.72 (s, 4H), 3.61 (d, *J =* 11.3 Hz, 4H), 3.48 (dd, *J =* 12.0, 5.1 Hz, 4H), 2.91 (s, 2H), 2.71 - 2.63 (m, 1H), 2.21 (d, *J =* 3.8 Hz, 2H), 2.07 - 2.00 (m, 1H), 1.93 (s, 2H), 1.42 (d, *J =* 6.5 Hz, 3H). LCMS (ESI) calcd for C₄₃H₄₉N₁₀O₄⁺ [M+H]⁺: 769.39; found, 769.3.

### Example 769: Preparation of (R)-N-(1-(4-(4-(4-(4-(2,4-dioxotetrahydropyrimidin-1(2H)-yl)benzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07952)

According to the method of step 2 of Scheme I-1, the target compound (GT-07952) was prepared (white solid, 33 mg, yield 62 %). ¹H NMR (400 MHz, DMSO-d6) δ 10.65 (s, 1H), 10.44 (s, 1H), 8.36 (d, *J =* 4.8 Hz, 1H), 8.30 (d, *J =* 8.2 Hz, 1H), 8.19 (s, 1H), 7.91 (d, *J =* 8.2 Hz, 1H), 7.79 (s, 1H), 7.70 (s, 1H), 7.68 (s, 1H), 7.44 (s, 1H), 7.42 (s, 1H), 7.26 (d, *J =* 8.6 Hz, 2H), 7.19 (s, 1H), 7.14 (s, 1H), 5.31 - 5.27 (m, 1H), 5.26 (s, 2H), 4.42 (s, 2H), 3.94 - 3.88 (m, 4H), 3.84 (s, 2H), 3.82 (s, 3H), 3.80 (s, 1H), 3.60 (s, 2H), 3.50 (d, *J* = 5.4 Hz, 3H), 2.93 (s, 2H), 2.73 (d, *J =* 6.6 Hz, 2H), 2.21 (d, *J =* 10.2 Hz, 2H), 1.94 (d, *J =* 10.1 Hz, 2H), 1.42 (d, *J =* 6.5 Hz, 3H). LCMS (ESI) calcd for C₄₂H₄₈N₁₁O₄⁺ [M+H]⁺: 770.39; found, 770.3.

### Example 770: Preparation of (R)-N-(1-(4-(4-(4-(3-(2,4-dioxotetrahydropyrimidin-1(2H)-yl)benzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07953)

According to the method of step 2 of Scheme I-1, the target compound (GT-07953) was prepared (white solid, 36 mg, yield 68 %). ¹H NMR (400 MHz, DMSO-d6) δ 10.66 (s, 1H), 10.45 (s, 1H), 8.36 (d, *J =* 4.7 Hz, 1H), 8.30 (d, *J =* 8.2 Hz, 1H), 8.20 (s, 1H), 7.91 (d, *J =* 8.2 Hz, 1H), 7.79 (s, 1H), 7.66 (s, 1H), 7.54 - 7.50 (m, 1H), 7.47 (s, 1H), 7.26 (s, 2H), 7.22 (s, 2H), 7.14 (s, 1H), 5.30 (d, *J* = 5.7 Hz, 1H), 5.27 (s, 2H), 4.47 (d, *J =* 25.3 Hz, 2H), 3.92 (d, *J =* 4.4 Hz, 2H), 3.87 (s, 2H), 3.86 (s, 2H), 3.82 (s, 3H), 3.72 (s, 2H), 3.61 (s, 2H), 3.48 (dd, *J =* 12.8, 4.9 Hz, 2H), 2.97 (s, 2H), 2.72 (s, 2H), 2.23 (d, *J =* 10.9 Hz, 2H), 1.98 (d, *J =* 10.0 Hz, 2H), 1.42 (d, *J =* 6.5 Hz, 3H). LCMS (ESI) calcd for C₄₂H₄₈N₁₁O₄⁺ [M+H]⁺: 770.39; found, 770.3.

### Example 771: Preparation of (R)-N-(1-(4-(4-(4-((5-(2,4-dioxotetrahydropyrimidin-1(2H)-yl)pyridin-2-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07954)

According to the method of step 2 of Scheme I-1, the target compound (GT-07954) was prepared (white solid, 38 mg, yield 71 %). ¹H NMR (400 MHz, DMSO-d6) δ 10.70 (s, 1H), 10.61 (s, 1H), 8.71 (d, *J =* 2.5 Hz, 1H), 8.38 (d, *J =* 4.8 Hz, 1H), 8.31 (d, *J =* 8.2 Hz, 1H), 8.25 (s, 1H), 7.92 (d, *J =* 8.2 Hz, 1H), 7.82 (s, 1H), 7.82 - 7.78 (m, 1H), 7.46 (d, *J =* 6.9 Hz, 2H), 7.35 (s, 2H), 7.14 (s, 1H), 5.33 (s, 2H), 4.57 (s, 3H), 4.47 - 4.27 (m, 4H), 3.91 (d, *J =* 3.4 Hz, 1H), 3.89 (s, 2H), 3.82 (d, *J =* 11.8 Hz, 3H), 3.49 (dd, *J =* 12.2, 5.1 Hz, 1H), 3.25 (s, 2H), 2.76 (s, 2H), 2.32 (d, *J =* 10.6 Hz, 2H), 2.19 (d, *J =* 9.8 Hz, 2H), 1.43 (d, *J =* 6.5 Hz, 3H), 1.32 (d, *J* = 6.6 Hz, 2H), 1.28 (d, *J* = 6.6 Hz, 2H). LCMS (ESI) calcd for C₄₁H₄₇N₁₂O₄⁺ [M+H]⁺: 771.38; found, 771.3.

### Example 772: Preparation of (R)-N-(1-(4-(4-(4-((6-(2,4-dioxotetrahydropyrimidin-1(2H)-yl)pyridin-3-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07955)

According to the method of step 2 of Scheme I-1, the target compound (GT-07955) was prepared (white solid, 36 mg, yield 68 %). ¹H NMR (400 MHz, DMSO-d6) δ 10.66 (s, 1H), 10.62 (s, 1H), 8.66 (d, *J =* 1.9 Hz, 1H), 8.36 (d, *J =* 4.8 Hz, 1H), 8.30 (d, *J =* 8.2 Hz, 1H), 8.21 (s, 1H), 8.12 (dd, *J =* 8.7, 2.3 Hz, 1H), 7.91 (d, *J* = 8.2 Hz, 1H), 7.87 (s, 1H), 7.79 (s, 1H), 7.27 (s, 2H), 7.26 - 7.23 (m, 1H), 7.14 (s, 1H), 5.28 (s, 2H), 4.49 (d, *J =* 14.9 Hz, 2H), 4.12 - 4.07 (m, 4H), 3.92 (d, *J =* 4.4 Hz, 1H), 3.89 (d, *J =* 4.3 Hz, 1H), 3.83 (d, *J* = 11.7 Hz, 3H), 3.72 (s, 2H), 3.63 (s, 3H), 3.48 (dd, *J =* 12.6, 4.9 Hz, 2H), 3.01 (s, 2H), 2.70 (s, 2H), 2.24 (d, *J =* 10.2 Hz, 2H), 2.09 - 1.93 (m, 2H), 1.42 (d, *J =* 6.5 Hz, 3H). LCMS (ESI) calcd for C₄₁H₄₇N₁₂O₄⁺ [M+H]⁺: 771.38; found, 771.3.

### Example 773: Preparation of (9R)-N-(1-(4-(4-(4-(3-((2,6-dioxopiperidin-3-yl)amino)benzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07956)

According to the method of step 2 of Scheme I-1, the target compound (GT-07956) was prepared (white solid, 35 mg, yield 65 %). ¹H NMR (400 MHz, DMSO-d6) δ 10.86 (s, 1H), 10.67 (s, 1H), 8.37 (s, 1H), 8.30 (d, *J =* 8.2 Hz, 1H), 8.22 (s, 1H), 7.91 (d, *J =* 8.2 Hz, 1H), 7.80 (s, 1H), 7.31 (s, 4H), 7.14 (s, 1H), 7.04 (s, 1H), 6.85 (s, 2H), 5.30 (s, 3H), 4.40 (d, *J =* 4.7 Hz, 2H), 4.30 (s, 4H), 3.91 (dd, *J =* 12.9, 4.3 Hz, 2H), 3.82 (d, *J =* 11.4 Hz, 2H), 3.73 (s, 3H), 3.60 (s, 1H), 3.50 (d, *J* = 4.5 Hz, 1H), 3.47 (s, 1H), 3.10 (s, 2H), 2.85 - 2.78 (m, 1H), 2.59 (dd, *J =* 13.9, 3.6 Hz, 1H), 2.26 (d, *J =* 9.7 Hz, 2H), 2.08 (s, 2H), 1.89 (dd, *J =* 12.3, 4.2 Hz, 1H), 1.42 (d, *J =* 6.5 Hz, 3H). LCMS (ESI) calcd for C₄₃H₅₀N₁₁O₄⁺ [M+H]⁺: 784.40; found, 784.3.

### Example 774: Preparation of (R)-N-(1-(4-(4-(4-((2-(2,4-dioxotetrahydropyrimidin-1(2H)-yl)-5-fluoropyridin-4-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07957)

According to the method of step 2 of Scheme I-1, the target compound (GT-07957) was prepared (white solid, 36 mg, yield 66 %). ¹H NMR (400 MHz, DMSO-d6) δ 10.66 (s, 1H), 10.62 (s, 1H), 8.49 (s, 1H), 8.36 (d, *J* = 4.8 Hz, 1H), 8.30 (d, *J =* 8.2 Hz, 1H), 8.21 (s, 1H), 8.00 (d, *J =* 5.2 Hz, 1H), 7.91 (d, *J =* 8.2 Hz, 1H), 7.79 (s, 1H), 7.27 (s, 3H), 7.14 (s, 1H), 5.28 (s, 2H), 4.20 (s, 4H), 4.02 (t, *J =* 6.6 Hz, 4H), 3.91 (dd, *J =* 12.9, 4.4 Hz, 2H), 3.82 (d, *J =* 12.1 Hz, 3H), 3.63 (s, 2H), 3.48 (dd, *J =* 12.0, 5.2 Hz, 3H), 3.00 (s, 2H), 2.71 (t, *J* = 6.6 Hz, 2H), 2.25 (d, *J* = 10.5 Hz, 2H), 2.01 (d, *J* = 10.6 Hz, 2H), 1.43 (d, *J* = 6.5 Hz, 3H). LCMS (ESI) calcd for C₄₁H₄₆FN₁₂O₄⁺ [M+H]⁺: 789.37; found, 789.3.

### Example 775: Preparation of (R)-N-(1-(4-(4-(4-((2-(2,4-dioxotetrahydropyrimidin-1(2H)-yl)pyridin-4-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07958)

According to the method of step 2 of Scheme I-1, the target compound (GT-07958) was prepared (white solid, 36 mg, yield 68 %). ¹H NMR (400 MHz, DMSO-d6) δ 10.68 (s, 1H), 10.63 (s, 1H), 8.51 (d, *J =* 5.1 Hz, 1H), 8.37 (d, *J* = 4.8 Hz, 1H), 8.30 (d, *J =* 8.2 Hz, 1H), 8.23 (s, 1H), 8.00 (s, 1H), 7.92 (d, *J =* 8.2 Hz, 1H), 7.81 (s, 1H), 7.60 (d, *J =* 4.9 Hz, 1H), 7.34 (d, *J =* 12.9 Hz, 3H), 7.14 (s, 1H), 5.30 (s, 3H), 4.49 (s, 3H), 4.08 (t, *J =* 6.6 Hz, 3H), 3.91 (dd, *J =* 12.9, 4.3 Hz, 2H), 3.82 (d, *J =* 11.7 Hz, 3H), 3.70 (s, 4H), 3.52 - 3.45 (m, 2H), 3.13 (s, 2H), 2.71 (dd, *J =* 8.7, 4.4 Hz, 2H), 2.27 (s, 2H), 2.11 (d, *J =* 9.4 Hz, 2H), 1.43 (d, *J =* 6.5 Hz, 3H). LCMS (ESI) calcd for C₄₁H₄₇N₁₂O₄⁺ [M+H]⁺: 771.38; found, 771.3.

### Example 776: Preparation of (R)-N-(1-(4-(4-(4-((5-(2,4-dioxotetrahydropyrimidin-1(2H)-yl)pyridin-3-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07959)

According to the method of step 2 of Scheme I-1, the target compound (GT-07959) was prepared (white solid, 34 mg, yield 64 %). ¹H NMR (400 MHz, DMSO-d6) δ 10.74 (s, 1H), 10.68 (s, 1H), 8.91 (d, *J =* 2.2 Hz, 1H), 8.81 (s, 1H), 8.38 (s, 1H), 8.30 (d, *J =* 8.2 Hz, 1H), 8.24 (s, 1H), 7.92 (d, *J =* 8.2 Hz, 1H), 7.81 (s, 1H), 7.39 (s, 2H), 7.32 (d, *J =* 8.3 Hz, 2H), 7.14 (s, 1H), 5.32 (s, 3H), 4.58 (s, 3H), 4.00 (t, *J =* 6.6 Hz, 2H), 3.95 - 3.87 (m, 2H), 3.80 (s, 2H), 3.66 (s, 3H), 3.49 (dd, *J =* 12.1, 5.2 Hz, 1H), 3.23 - 3.13 (m, 2H), 2.78 (t, *J* = 6.6 Hz, 2H), 2.30 (d, *J =* 9.0 Hz, 2H), 2.16 (s, 2H), 1.43 (d, *J =* 6.5 Hz, 3H), 1.32 (d, *J =* 6.6 Hz, 2H), 1.28 (dd, *J =* 7.0, 3.3 Hz, 2H). LCMS (ESI) calcd for C₄₁H₄₇N₁₂O₄⁺ [M+H]⁺: 771.38; found, 771.3.

### Example 777: Preparation of (9R)-N-(1-(4-(4-(4-((5-(2,6-dioxopiperidin-3-yl)-2-fluoropyridin-3-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07960)

According to the method of step 2 of Scheme I-1, the target compound (GT-07960) was prepared (white solid, 28 mg, yield 51 %). ¹H NMR (400 MHz, DMSO-d6) δ 10.99 (s, 1H), 10.67 (s, 1H), 8.37 (d, *J =* 4.8 Hz, 1H), 8.30 (d, *J =* 8.2 Hz, 1H), 8.23 (d, *J =* 4.7 Hz, 2H), 7.92 (d, *J =* 8.2 Hz, 1H), 7.80 (s, 1H), 7.31 (s, 4H), 7.14 (s, 1H), 5.30 (s, 3H), 4.40 (s, 2H), 4.06 (dd, *J =* 12.5, 4.9 Hz, 2H), 3.91 (dd, *J =* 12.8, 4.3 Hz, 2H), 3.82 (d, *J =* 11.6 Hz, 3H), 3.71 (s, 2H), 3.64 - 3.55 (m, 4H), 3.48 (dd, *J =* 12.4, 5.2 Hz, 2H), 3.12 - 3.03 (m, 2H), 2.81 - 2.70 (m, 1H), 2.65 - 2.56 (m, 1H), 2.31 (dd, *J =* 12.5, 8.5 Hz, 2H), 2.09 (dd, *J =* 9.6, 3.5 Hz, 2H), 1.43 (d, *J* = 6.5 Hz, 3H), 1.31 (d, *J* = 6.6 Hz, 1H), 1.27 (d, *J* = 6.6 Hz, 1H). LCMS (ESI) calcd for C₄₂H₄₇FN₁₁O₄⁺ [M+H]⁺: 788.38; found, 788.3.

### Example 778: Preparation of (9R)-N-(1-(4-(4-(4-((5-(2,6-dioxopiperidin-3-yl)pyridin-3-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07961)

According to the method of step 2 of Scheme I-1, the target compound (GT-07961) was prepared (white solid, 34 mg, yield 64 %). ¹H NMR (400 MHz, DMSO-d6) δ 11.08 (s, 1H), 10.67 (s, 1H), 9.02 (s, 1H), 8.87 (d, *J* = 1.5 Hz, 1H), 8.62 (s, 1H), 8.37 (d, *J =* 4.8 Hz, 1H), 8.30 (d, *J =* 8.2 Hz, 1H), 8.22 (s, 1H), 7.91 (d, *J* = 8.2 Hz, 1H), 7.80 (s, 1H), 7.31 (s, 3H), 7.14 (s, 1H), 5.30 (s, 3H), 4.54 (s, 3H), 4.45 - 4.36 (m, 2H), 4.23 (dd, *J =* 12.6, 4.8 Hz, 2H), 3.91 (dd, *J =* 12.9, 4.4 Hz, 1H), 3.82 (d, *J =* 11.8 Hz, 2H), 3.68 (s, 2H), 3.50 (d, *J* = 4.7 Hz, 2H), 3.12 - 3.04 (m, 2H), 2.84 - 2.72 (m, 1H), 2.68 - 2.59 (m, 1H), 2.28 (d, *J =* 9.9 Hz, 2H), 2.11 (s, 2H), 1.42 (d, *J* = 6.5 Hz, 3H), 1.31 (d, *J* = 6.6 Hz, 2H), 1.29 - 1.26 (m, 2H). LCMS (ESI) calcd for C₄₂H₄₈N₁₁O₄⁺ [M+H]⁺: 770.39; found, 770.3.

### Example 779: Preparation of (R)-N-(1-(4-(4-(4-((5-(2,4-dioxotetrahydropyrimidin-1(2H)-yl)-2-fluoropyridin-3-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07962)

According to the method of step 2 of Scheme I-1, the target compound (GT-07962) was prepared (white solid, 38 mg, yield 70 %). ¹H NMR (400 MHz, DMSO-d6) δ 10.66 (s, 1H), 10.61 (s, 1H), 8.36 (s, 2H), 8.30 (d, *J =* 8.2 Hz, 2H), 8.21 (s, 1H), 7.91 (d, *J =* 8.2 Hz, 1H), 7.79 (s, 1H), 7.27 (s, 3H), 7.14 (s, 1H), 5.28 (s, 3H), 4.35 (s, 3H), 4.12 (s, 1H), 3.90 (t, *J =* 6.6 Hz, 4H), 3.85 - 3.79 (m, 3H), 3.69 (s, 3H), 3.51 - 3.45 (m, 3H), 2.99 (s, 2H), 2.76 (d, *J* = 6.6 Hz, 2H), 2.24 (d, *J =* 10.5 Hz, 2H), 2.00 (d, *J* = 9.6 Hz, 2H), 1.42 (d, *J =* 6.5 Hz, 3H). LCMS (ESI) calcd for C₄₁H₄₆FN₁₂O₄⁺ [M+H]⁺: 789.37; found, 789.3.

### Example 780: Preparation of (R)-N-(1-(4-(4-(4-((5-(2,4-dioxotetrahydropyrimidin-1(2H)-yl)pyrazin-2-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-07963)

According to the method of step 2 of Scheme I-1, the target compound (GT-07963) was prepared (white solid, 37 mg, yield 69 %). ¹H NMR (400 MHz, DMSO-d6) δ 10.81 (s, 1H), 10.65 (s, 1H), 9.14 (d, *J =* 1.4 Hz, 1H), 8.74 (d, *J =* 1.2 Hz, 1H), 8.36 (d, *J =* 4.9 Hz, 1H), 8.30 (d, *J =* 8.2 Hz, 1H), 8.20 (s, 1H), 7.91 (d, *J* = 8.2 Hz, 1H), 7.79 (s, 1H), 7.25 (s, 2H), 7.19 (s, 1H), 7.14 (s, 1H), 5.26 (s, 2H), 4.54 (s, 2H), 4.08 (d, *J =* 6.6 Hz, 3H), 3.90 (s, 3H), 3.84 (d, *J* = 12.1 Hz, 4H), 3.72 (s, 4H), 3.48 (dd, *J* = 12.6, 4.8 Hz, 2H), 2.92 (s, 2H), 2.74 (s, 2H), 2.22 (d, *J =* 10.1 Hz, 2H), 1.95 (d, *J =* 10.4 Hz, 2H), 1.42 (d, *J =* 6.5 Hz, 3H). LCMS (ESI) calcd for C₄₀H₄₆N₁₃O₄⁺ [M+H]⁺: 772.38; found, 772.3.

### Example 781: Preparation of 3-(4-((4-(3-amino-6-(2-hydroxyphenyl)pyridazin-4-yl)piperazin-1-yl)methyl)phenyl)piperidine-2,6-dione (GT-05015)

According to the method of step 2 of Scheme I-1, the target compound (GT-05015) was prepared (white solid, 22 mg, yield 31.58%). ¹H NMR (400 MHz, DMSO) δ 11.82 (s, 1H), 10.88 (s, 1H), 7.66 - 7.58 (m, 4H), 7.52 (s, 1H), 7.36 (dd, J = 17.3, 7.7 Hz, 3H), 7.07 (d, J = 8.2 Hz, 1H), 6.97 (t, J = 7.6 Hz, 1H), 4.36 (s, 2H), 3.94 (dd, J = 11.7, 4.9 Hz, 1H), 3.76 (s, 2H), 3.64 - 3.59 (m, 1H), 3.45 (s, 4H), 3.15 - 3.06 (m, 1H), 2.69 (ddd, J = 17.0, 11.9, 5.3 Hz, 1H), 2.53 (d, J = 4.4 Hz, 1H), 2.23 (qd, J = 12.6, 4.3 Hz, 1H), 2.09 - 1.97 (m, 1H). LCMS (ESI) calcd for C₂₆H₂₉N₆O₃⁺ [M+H]⁺: 473.23; found, 473.3.

### Example 782: Preparation of 1-(4-((4-(3-amino-6-(2-hydroxyphenyl)pyridazin-4-yl)piperazin-1-yl)methyl)phenyl)dihydropyrimidine-2,4(1H,3H)-dione (GT-04961)

According to the method of step 2 of Scheme I-1, the target compound (GT-04961) was prepared (white solid, 24 mg, yield 34.38%). ¹H NMR (400 MHz, DMSO) δ 11.72 (s, 1H), 10.45 (s, 1H), 9.03 (s, 1H), 7.66 (t, J = 8.6 Hz, 2H), 7.61 (d, J = 5.5 Hz, 2H), 7.45 (d, J = 8.4 Hz, 2H), 7.39 (s, 1H), 7.08 (d, J = 8.2 Hz, 1H), 7.00 (d, J = 7.5 Hz, 1H), 4.38 (s, 2H), 3.83 (t, J = 6.6 Hz, 4H), 3.67 - 3.58 (m, 2H), 3.46 (s, 2H), 3.13 (dd, J = 7.4, 4.3 Hz, 2H), 2.73 (t, J = 6.6 Hz, 2H). LCMS (ESI) calcd for C₂₅H₂₈N₇O₃⁺ [M+H]⁺: 474.23; found, 474.3.

### Example 783: Preparation of 1-(3-((4-(3-amino-6-(2-hydroxyphenyl)pyridazin-4-yl)piperazin-1-yl)methyl)phenyl)dihydropyrimidine-2,4(1H,3H)-dione (GT-04962)

According to the method of step 2 of Scheme I-1, the target compound (GT-04962) was prepared (white solid, 17 mg, yield 24.35%). ¹H NMR (400 MHz, DMSO) δ 11.88 (s, 1H), 10.46 (s, 1H), 9.25 (s, 1H), 7.69 - 7.59 (m, 3H), 7.54 - 7.46 (m, 3H), 7.39 (t, J = 7.7 Hz, 1H), 7.09 (d, J = 8.2 Hz, 1H), 6.99 (t, J = 7.5 Hz, 1H), 4.39 (s, 2H), 3.88 (t, J = 6.6 Hz, 2H), 3.78 (s, 2H), 3.66 - 3.58 (m, 2H), 3.48 (s, 2H), 3.12 (qd, J = 7.3, 4.2 Hz, 2H), 2.73 (t, J = 6.6 Hz, 2H). LCMS (ESI) calcd for C₂₅H₂₈N₇O₃⁺ [M+H]⁺: 474.23; found, 474.2.

### Example 784: Preparation of 1-(6-((4-(3-amino-6-(2-hydroxyphenyl)pyridazin-4-yl)piperazin-1-yl)methyl)pyridin-3-yl)dihydropyrimidine-2,4(1H,3H)-dione (GT-05016)

According to the method of step 2 of Scheme I-1, the target compound (GT-05016) was prepared (white solid, 20 mg, yield 28.59%). ¹H NMR (400 MHz, DMSO) δ 11.62 (s, 1H), 10.60 (s, 1H), 8.71 (d, J = 2.5 Hz, 1H), 7.93 (dd, J = 8.4, 2.5 Hz, 1H), 7.81 (d, J = 8.4 Hz, 1H), 7.68 - 7.54 (m, 2H), 7.40 (t, J = 7.2 Hz, 1H), 7.13 (d, J = 8.2 Hz, 1H), 6.99 (t, J = 7.5 Hz, 1H), 4.53 (s, 2H), 3.89 (t, J = 6.6 Hz, 2H), 3.69 - 3.59 (m, 8H), 2.76 (t, J = 6.6 Hz, 2H). LCMS (ESI) calcd for C₂₄H₂₇N₈O₃⁺ [M+H]⁺: 475.22; found, 475.2.

### Example 785: Preparation of 1-(5-((4-(3-amino-6-(2-hydroxyphenyl)pyridazin-4-yl)piperazin-1-yl)methyl)pyridin-2-yl)dihydropyrimidine-2,4(1H,3H)-dione (GT-04963)

According to the method of step 2 of Scheme I-1, the target compound (GT-04963) was prepared (white solid, 27 mg, yield 38.59%). ¹H NMR (400 MHz, DMSO) δ 12.19 (s, 1H), 10.63 (s, 1H), 8.64 (d, J = 2.0 Hz, 1H), 8.13 (dd, J = 8.7, 2.2 Hz, 1H), 7.88 (d, J = 8.6 Hz, 1H), 7.66 - 7.52 (m, 2H), 7.40 (t, J = 7.7 Hz, 1H), 7.12 (d, J = 8.2 Hz, 1H), 6.99 (t, J = 7.5 Hz, 1H), 4.43 (s, 2H), 4.10 (t, J = 6.6 Hz, 2H), 3.79 (s, 2H), 3.47 (d, J = 11.2 Hz, 4H), 3.33 (s, 2H), 2.71 (t, J = 6.6 Hz, 2H). LCMS (ESI) calcd for C₂₄H₂₇N₈O₃⁺ [M+H]⁺: 475.22; found, 475.2.

### Example 786: Preparation of 3-((3-((4-(3-amino-6-(2-hydroxyphenyl)pyridazin-4-yl)piperazin-1-yl)methyl)phenyl)amino)piperidine-2,6-dione (GT-04964)

According to the method of step 2 of Scheme I-1, the target compound (GT-04964) was prepared (white solid, 19 mg, yield 26.43%). ¹H NMR (400 MHz, DMSO) δ 11.74 (s, 1H), 10.85 (s, 1H), 7.64 - 7.52 (m, 2H), 7.41 (t, J = 7.3 Hz, 1H), 7.23 - 7.09 (m, 2H), 7.05 (s, 1H), 6.99 (t, J = 7.5 Hz, 1H), 6.81 (d, J = 8.0 Hz, 2H), 4.41 (dd, J = 11.6, 4.8 Hz, 1H), 4.24 (s, 2H), 3.78 (s, 2H), 3.43 - 3.27 (m, 5H), 3.12 (ddd, J = 14.7, 7.4, 4.3 Hz, 1H), 2.84 (ddd, J = 17.6, 12.2, 6.0 Hz, 1H), 2.64 - 2.55 (m, 1H), 2.26 - 2.12 (m, 1H), 1.90 (tt, J = 12.3, 6.1 Hz, 1H). LCMS (ESI) calcd for C₂₆H₃₀N₇O₃⁺ [M+H]⁺: 488.24; found, 488.3.

### Example 787: Preparation of 3-(4-((4-(3-amino-6-(5-fluoro-2-hydroxyphenyl)pyridazin-4-yl)piperazin-1-yl)methyl)phenyl)piperidine-2,6-dione (GT-05062)

According to the method of step 2 of Scheme I-1, the target compound (GT-05062) was prepared (white solid, 32 mg, yield 47.18%). ¹H NMR (400 MHz, DMSO) δ 11.75 (s, 1H), 10.88 (s, 1H), 7.70 (d, J = 25.4 Hz, 1H), 7.67 - 7.59 (m, 3H), 7.53 (dd, J = 9.4, 3.1 Hz, 1H), 7.35 (d, J = 8.2 Hz, 2H), 7.25 (td, J = 8.6, 3.2 Hz, 1H), 7.08 (dd, J = 9.0, 4.8 Hz, 1H), 4.37 (s, 2H), 3.94 (dd, J = 11.7, 4.9 Hz, 1H), 3.75 (s, 2H), 3.35 - 3.26 (m, 6H), 2.69 (td, J = 11.9, 6.0 Hz, 1H), 2.54 (d, J = 4.5 Hz, 1H), 2.24 (dd, J = 12.3, 3.7 Hz, 1H), 2.10 - 2.01 (m, 1H). LCMS (ESI) calcd for C₂₆H₂₈FN₆O₃⁺ [M+H]⁺: 491.22; found, 491.2.

### Example 788: Preparation of 1-(4-((4-(3-amino-6-(5-fluoro-2-hydroxyphenyl)pyridazin-4-yl)piperazin-1-yl)methyl)phenyl)dihydropyrimidine-2,4(1H,3H)-dione (GT-05063)

According to the method of step 2 of Scheme I-1, the target compound (GT-05063) was prepared (white solid, 30 mg, yield 44.14%). ¹H NMR (400 MHz, DMSO) δ 11.89 (s, 1H), 10.45 (s, 1H), 7.79 - 7.64 (m, 4H), 7.55 (dd, J = 9.0, 2.2 Hz, 1H), 7.45 (d, J = 8.5 Hz, 2H), 7.24 (td, J = 8.6, 3.2 Hz, 1H), 7.08 (dd, J = 9.0, 4.8 Hz, 1H), 4.41 (d, J = 25.4 Hz, 2H), 3.84 (t, J = 6.6 Hz, 2H), 3.74 (s, 2H), 3.36 - 3.24 (m, 6H), 2.73 (t, J = 6.6 Hz, 2H). LCMS (ESI) calcd for C₂₅H₂₇FN₇O₃⁺ [M+H]⁺: 492.22; found, 492.2.

### Example 789: Preparation of 1-(3-((4-(3-amino-6-(5-fluoro-2-hydroxyphenyl)pyridazin-4-yl)piperazin-1-yl)methyl)phenyl)dihydropyrimidine-2,4(1H,3H)-dione (GT-05064)

According to the method of step 2 of Scheme I-1, the target compound (GT-05064) was prepared (white solid, 29 mg, yield 42.67%). ¹H NMR (400 MHz, DMSO) δ 11.91 (s, 1H), 10.46 (s, 1H), 7.77 (s, 1H), 7.66 (s, 2H), 7.51 (ddd, J = 9.1, 7.4, 2.4 Hz, 4H), 7.25 (td, J = 8.6, 3.2 Hz, 1H), 7.09 (dd, J = 9.0, 4.8 Hz, 1H), 4.40 (s, 2H), 3.88 (t, J = 6.6 Hz, 2H), 3.74 (s, 2H), 3.38 - 3.27 (m, 6H), 2.73 (t, J = 6.6 Hz, 2H). LCMS (ESI) calcd for C₂₅H₂₇FN₇O₃⁺ [M+H]⁺: 492.22; found, 492.2.

### Example 790: Preparation of 1-(6-((4-(3-amino-6-(5-fluoro-2-hydroxyphenyl)pyridazin-4-yl)piperazin-1-yl)methyl)pyridin-3-yl)dihydropyrimidine-2,4(1H,3H)-dione (GT-05065)

According to the method of step 2 of Scheme I-1, the target compound (GT-05065) was prepared (white solid, 14 mg, yield 20.56%). ¹H NMR (400 MHz, DMSO) δ 11.61 (s, 1H), 10.60 (s, 1H), 8.71 (d, J = 2.6 Hz, 1H), 7.93 (dd, J = 8.4, 2.6 Hz, 1H), 7.80 (d, J = 8.4 Hz, 1H), 7.67 (s, 1H), 7.50 (dd, J = 9.3, 3.1 Hz, 1H), 7.26 (td, J = 8.6, 3.2 Hz, 1H), 7.12 (dd, J = 9.0, 4.8 Hz, 1H), 4.53 (s, 2H), 3.89 (t, J = 6.6 Hz, 2H), 3.61 (s, 8H), 2.76 (t, J = 6.6 Hz, 2H). LCMS (ESI) calcd for C₂₄H₂₆FN₈O₃⁺ [M+H]⁺: 493.21; found, 493.3.

### Example 791: Preparation of 1-(5-((4-(3-amino-6-(5-fluoro-2-hydroxyphenyl)pyridazin-4-yl)piperazin-1-yl)methyl)pyridin-2-yl)dihydropyrimidine-2,4(1H,3H)-dione (GT-05066)

According to the method of step 2 of Scheme I-1, the target compound (GT-05066) was prepared (white solid, 37 mg, yield 54.33%). ¹H NMR (400 MHz, DMSO) δ 12.16 (s, 1H), 10.63 (s, 1H), 8.64 (d, J = 2.0 Hz, 1H), 8.13 (dd, J = 8.7, 2.4 Hz, 1H), 7.88 (d, J = 8.7 Hz, 1H), 7.67 (s, 1H), 7.49 (dd, J = 9.3, 3.2 Hz, 1H), 7.26 (td, J = 8.6, 3.2 Hz, 1H), 7.12 (d, J = 4.8 Hz, 1H), 4.43 (s, 2H), 4.11 (t, J = 6.6 Hz, 2H), 3.75 (s, 4H), 3.33 (s, 4H), 2.71 (t, J = 6.6 Hz, 2H). LCMS (ESI) calcd for C₂₄H₂₆FN₈O₃⁺ [M+H]⁺: 493.21; found, 493.3.

### Example 792: Preparation of 3-((3-((4-(3-amino-6-(5-fluoro-2-hydroxyphenyl)pyridazin-4-yl)piperazin-1-yl)methyl)phenyl)amino)piperidine-2,6-dione (GT-05067)

According to the method of step 2 of Scheme I-1, the target compound (GT-05067) was prepared (white solid, 34 mg, yield 48.64%). ¹H NMR (400 MHz, DMSO) δ 11.78 (s, 1H), 10.85 (s, 1H), 7.94 (s, 1H), 7.66 (s, 1H), 7.48 (dd, J = 9.3, 3.2 Hz, 1H), 7.26 (td, J = 8.6, 3.2 Hz, 1H), 7.21 - 7.10 (m, 2H), 7.06 (s, 1H), 6.86 - 6.76 (m, 2H), 4.42 (dd, J = 11.7, 4.8 Hz, 1H), 4.24 (s, 2H), 3.58 - 3.50 (m, 4H), 3.43 (d, J = 11.7 Hz, 2H), 3.29 (d, J = 10.1 Hz, 2H), 2.84 (ddd, J = 17.6, 12.4, 5.2 Hz, 1H), 2.60 (dd, J = 13.8, 3.7 Hz, 1H), 2.26 - 2.13 (m, 1H), 1.90 (qd, J = 12.7, 4.6 Hz, 1H). LCMS (ESI) calcd for C₂₆H₂₉FN₇O₃⁺ [M+H]⁺: 506.23; found, 506.2.

### Biological activity assay

### Reagents and Materials

| | |
|---|---|
| Reagents and materials | Suppliers or Source |
| Human B lymphoma cell line: SU-DHL-4 | ATCC (American Type Culture Collection) |
| Human diffuse large B-cell lymphoma cell line: SU-DHL-6 | ATCC |
| Human acute lymphocytic leukemia T lymphocytes cell line: CCRF-CEM | Dalian Meilun Biotech Co., Ltd. |
| Human Burkitt's lymphoma cell line: Daudi | Dalian Meilun Biotech Co., Ltd. |
| Human non-small cell lung cancer cell line: HCC827 | ATCC |
| Human liver cancer cell line: HEP3B2.7-1 | Dalian Meilun Biotech Co., Ltd. |
| Human gastric cancer cell line: HGC-27 | Dalian Meilun Biotech Co., Ltd. |
| Human acute lymphoblastic leukemia cell line: Kasumi-1 | ATCC |
| Human breast cancer cell line: MDA-MB-231 | Dalian Meilun Biotech Co., Ltd. |
| Multiple myeloma cell line: MM.1S | ATCC |
| Human B-lymphoma cell line: Ramos | Dalian Meilun Biotech Co., Ltd. |
| Human hepatocellular carcinoma cell line: SNU-182 | Dalian Meilun Biotech Co., Ltd. |
| Human immunoblastic lymphoblastic lymphoma cell line: SR | Dalian Meilun Biotech Co., Ltd. |
| Human breast ductal carcinoma cell line: T47D | ATCC |
| Human diffuse large B-cell lymphoma cell line: TMD8 | Kyinno Biotechnology Co., Ltd |
| Human prostate cancer cell line: 22RV1 | Dalian Meilun Biotech Co., Ltd. |
| Human non-small cell lung cancer cell line: NCI-H1975 | ATCC |
| Human Malignant Meningioma Cell Line: JEKO-1 | ATCC |
| Human M-cell lymphoma cell line: Mino | ATCC |
| Human monocytic leukemia cell line: THP-1 | ATCC |
| Human myeloid monocytic leukemia cell line: MV-4-11 | ATCC |
| dexamethasone-resistant multiple myeloma cell line: MM.1R | ATCC |
| Human prostate cancer cell line: Vcap | ATCC |
| Human colon cancer cell line: SW620 | ATCC |
| Human prostate cancer cell line: DU-145 | ATCC |
| Human ovarian cancer cell line: PC-3 | ATCC |
| Human lung adenocarcinoma cell line: PC-9 | Dalian Meilun Biotech Co., Ltd. |
| Human prostate cancer cell line: NCI-H358 | ATCC |
| Human non-small cell lung cancer cell line: NCI-H2228 | ATCC |
| Human bronchioloalveolar adenocarcinoma cell line: NCI- | ATCC |
| H1666 | |
| Human prostate cancer cell line: LNcap | ATCC |
| Human chronic myelogenous leukemia cell line: K562 | ATCC |
| Human ovarian cancer cell line: NIH: OVCAR3 | Dalian Meilun Biotech Co., Ltd. |
| Human T-lymphocyte leukemia cell line: Jurkat | Dalian Meilun Biotech Co., Ltd. |
| Human thyroid cancer cell line (undifferentiated): CAL-62 | Dalian Meilun Biotech Co., Ltd. |
| Human gastric cancer cell line: MGC-803 | Dalian Meilun Biotech Co., Ltd. |
| Human pancreatic cancer cell line: CFPAC-1 | Dalian Meilun Biotech Co., Ltd. |
| Human non-small cells lung cancer cell line: A549 | ATCC |
| Human primary pancreatic adenocarcinoma cell line: BXPC-3 | Dalian Meilun Biotech Co., Ltd. |
| Human non-small cells lung cancer cell line: H1299 | Dalian Meilun Biotech Co., Ltd. |
| Human lung squamous cell carcinoma cell line: H520 | ATCC |
| Human breast cancer cell line: MCF7 | ATCC |
| RPMI-1640 Medium | Dalian Meilun Biotech Co., Ltd. |
| MEM Medium | Dalian Meilun Biotech Co., Ltd. |
| IMDM Medium | Gibco Company |
| DMEM Medium | Dalian Meilun Biotech Co., Ltd. |
| DMEM medium, high glucose | ATCC |
| L-15 Medium | ATCC |
| F-12K Medium | ATCC |
| EMEM Medium | Dalian Meilun Biotech Co., Ltd. |
| Meilun Cell Counting Kit-8 | Dalian Meilun Biotech Co., Ltd. |
| Fetal bovine serum (FBS) | Sunrise Science Products Company |
| Penicillin-Streptomycin | Beijing TransGen Biotech Co., Ltd. |
| horse serum (HS) | Dalian Meilun Biotech Co., Ltd. |
| Mycoplasma detection kit | Beijing TransGen Biotech Co., Ltd. |
| STR genotype detection | Shanghai Biowing Applied Biotechnology Co. Ltd. |

### Methods:

### Cell cultures

The tumor cells used herein were cultured in a cell culture medium listed in table A1 at 37°C in an incubator with 5% CO₂. Prior to experimentation, all cell lines used were correctly identified by STR profiling, and tested negative for mycoplasma contamination using a mycoplasma detection kit through routine screenings.

**Table A1. Tumor cells and cell culture medium used in the present disclosure**

| Cell line | Cell culture medium used |
|---|---|
| SU-DHL-4, SU-DHL-6, CCRF-CEM, Daudi, HCC827, Hep3B2.7-1, HGC-27, Kasumi-1, MM.1S, Ramos, SNU-182, SR, T47D, TMD8, 22RV1, NCI-H1975, JEKO-1, Mino, THP-1, MM.1R, PC-9, NCI-H358, NCI-H2228, NCI-H1666, NIH: OVCAR3, Jurkat, MGC-803, BXPC-3, H1299 | RPMI 1640 culture medium supplemented with 10% FBS, as well as penicillin at a final concentration of 100 U/mL and streptomycin at a final concentration of 100 µg/mL |
| MDA-MB-231, CAL-62 | DMEM culture medium supplemented with 10% FBS, as well as penicillin at a final concentration of 100 U/mL and streptomycin at a final concentration of 100 µg/mL |
| MV-4-11, K562, CFPAC-1 | IMDM culture medium supplemented with 10% FBS, as well as penicillin at a final concentration of 100 U/mL and streptomycin at a final concentration of 100 µg/mL |
| Vcap | DMEM high-glucose medium, supplemented with 10% FBS, as well as penicillin at a final concentration of 100 U/mL and streptomycin at a final concentration of 100 µg/mL |
| SW620 | L-15 culture medium supplemented with 10% FBS, as well as penicillin at a final concentration of 100 U/mL and streptomycin at a final concentration of 100 µg/mL |
| DU-145 | EMEM culture medium supplemented with 10% FBS, as well as penicillin at a final concentration of 100 U/mL and streptomycin at a final concentration of 100 µg/mL |
| PC-3, LNcap, A549 | F-12K culture medium supplemented with 10% FBS, as well as penicillin at a final concentration of 100 U/mL and streptomycin at a final concentration of 100 µg/mL |
| H520 | RPMI-1640 medium supplemented with 10% FBS, 2.5% horse serum, as well as penicillin at a final concentration of 100 U/mL and streptomycin at a final concentration of 100 µg/mL |

### I. Determination of half inhibitory concentration (IC₅₀) of the compounds against tumor cells:

IC₅₀ values of the compounds of the present disclosure (including the compounds in Table 1, and compounds of examples) were measured using the Meilun Cell Counting Kit-8 kit from Dalian Meilun Biotech Co., Ltd. Assay details are as follows: tumor cells were seeded in a 96-well plate at a density listed in Table A2. After 24h, 100 µL of the inoculated cells were treated with 0.5µL of the compounds of the present disclosure (including the compounds in Tables 1 and 2, and compounds of examples) at 10 different successively decreasing concentrations (starting at the highest concentration of 10µM; 5-fold serial dilutions). After the cells were treated with the compounds of the present disclosure for a period of time, the cell viability detection reagent was added to the culture medium for cell viability assessment according to the instructions provided with the CCK-8 kit. DMSO served as the negative control, and corresponding commercial inhibitors (including Orelabrutinib (CAS NO.: 1655504-04-3); Ibrutinib (CAS NO.: 936563-96-1); compound GT-03308 (CAS NO.: 1679327-21-9); compound GT-03335 (CAS NO.: 1801765-04-7); compound GT-03336 (CAS NO.: 2160546-07-4); compound GT-03334 (CAS NO.: 1801747-42-1); compound TQB3804 (CAS NO.: 2267329-76-8); erlotinib (CAS NO.: 183321-74-6); brigatinib (CAS NO.: 1197953-54-0); alectinib (CAS NO.: 1256580-46-7); ARV-471 (CAS NO.: 2229711-08-2); abemaciclib (CAS NO.: 1231929-97-7); Ribociclib (CAS NO.: 1211443-58-1); Palbociclib (CAS NO.: 571190-30-2); Defactinib (CAS NO.: 1073154-85-4); PND-1186 (CAS NO.: 1061353-68-1); Dasatinib (CAS NO.: 302962-49-8); emavusertib (CAS NO.: 1801344-14-8); AT7519 (CAS NO.: 844442-38-2); Venetoclax (ABT-199, CAS NO.: 1257044-40-8); PF06650833 (CAS NO.: 1817626-54-2); HJM-561 (CAS NO.: 2570251-68-0); osimertinib (CAS NO.: 1421373-65-0); FN-1501 (CAS NO.: 1429515-59-2); and ABT-263 (Navitoclax, CAS NO.: 923564-51-6)) were used as the positive control, both of which treated the cells using the same protocol as that of the compounds of the present disclosure. The growth inhibition of the compounds of the present disclosure on cells was plotted by Prism Graphpad software, and the IC₅₀ values of the compounds of the present disclosure were calculated therefrom. Results were shown in Tables B1-B10 below.

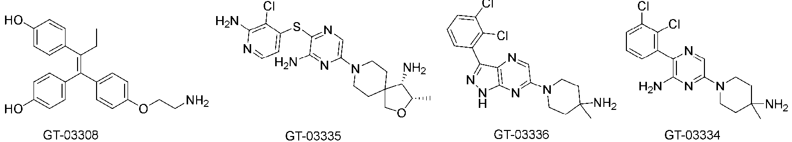

**Table A2. Seeding protocol and treatment time for tumor cells**

| Cells | Cells seeding protocol | The times for treating cells with the compounds of the present disclosure (hours) |
|---|---|---|
| SU-DHL-4, SU-DHL-6, Kasumi-1, MM.1S, Ramos, SR, TMD8, Mino, THP-1, MM.1R, MGC-803, CAL-62 | cells were seeded at a density of 10,000 cells per well in 100 µL of serum-containing RPMI-1640 medium per well | 72 hours |
| CCRF-CEM, Daudi, HCC827, Hep3B2.7-1, HGC-27, SNU-182, T47D, 22RV1, NCI-H1975, JEKO-1, PC-9, NCI-H358, NCI-H2228, NCI-H1666, NIH: OVCAR3, Jurkat, BXPC-3 | cells were seeded at a density of 4,000 cells per well in 100 µL of serum-containing RPMI-1640 medium per well | 96 hours |
| H1299 | cells were seeded at a density of 2,000 cells per well in 100 µL of serum-containing RPMI-1640 medium per well | 120 hours |
| H520 | cells were seeded at a density of 3,000 cells per well in 100 µL of serum-containing RPMI-1640 medium per well | 96 hours |
| MDA-MB-231 | cells were seeded at a density of 4,000 cells per well in 100 µL of serum-containing DMEM medium per well | 96 hours |
| MV-4-11, K562, CFPAC-1 | cells were seeded at a density of 10,000 cells per well in 100 µL of serum-containing IMDM medium per well | 72 or 96 hours |
| Vcap | cells were seeded at a density of 4,000 cells per well in 100 µL of serum-containing DMEM high-glucose medium per well | 96 hours |
| SW620 | cells were seeded at a density of 4,000 cells per well in 100 µL of serum-containing L-15 medium per well | 96 hours |
| DU-145 | cells were seeded at a density of 4,000 cells per well in 100 µL of serum-containing EMEM medium per well | 96 hours |
| PC-3, LNcap, A549 | cells were seeded at a density of 4,000 cells per well in 100 µL of serum-containing F-12K medium per well | 96 hours |

**Table B1. Inhibitory activity (IC₅₀, nM) of the compounds of examples of the present disclosure, designed based on the BTK inhibitors, on tumor cell proliferation**

| Comp. No. /names | H2228 | PC-9 | H1975 | MM.1R | SW620 | SU-DHL-6 | K562 | TMD8 |
|---|---|---|---|---|---|---|---|---|
| ibrutinib | F | B | C | F | F | B | - | F |
| Orelabrutinib | F | F | F | F | F | - | F | E |
| GT-04785 | B | B | C | B | C | - | - | - |
| GT-04786 | B | C | C | - | - | - | - | - |
| GT-04787 | B | B | C | C | - | - | - | - |
| GT-04788 | B | B | C | - | - | - | - | - |
| GT-04789 | B | B | C | - | - | - | - | - |
| GT-04790 | B | B | C | - | C | - | - | - |
| GT-04791 | B | C | - | A+++ | C | - | - | - |
| GT-04798 | C | B | C | - | - | - | - | - |
| GT-04799 | C | C | - | - | - | - | - | - |
| GT-04800 | C | B | - | C | - | - | - | - |
| GT-04801 | B | B | C | - | - | - | - | - |
| GT-04802 | B | B | C | B | - | - | - | - |
| GT-04803 | B | B | C | B | B | - | - | - |
| GT-03507 | - | - | - | - | - | C | - | B |
| GT-03508 | - | - | - | - | - | C | - | B |
| GT-03542 | - | - | - | - | - | B | - | B |
| GT-03513 | - | - | - | - | - | B | - | B |
| GT-03753 | - | - | - | C | C | - | B | - |
| GT-03754 | - | - | - | B | B | - | B | - |
| GT-03828 | - | - | - | C | - | - | - | - |

**Table B2. Inhibitory activity (IC₅₀, nM) of the compounds of examples of the present disclosure, designed based on the AR inhibitors, on tumor cell proliferation**

| Comp. No. /names | BXPC-3 | MGC80-3 | THP-1 | DU-145 | H520 |
|---|---|---|---|---|---|
| Enzalutamide | F | F | F | D | F |
| abiraterone | F | F | F | F | F |
| GT-04842 | C | - | C | - | - |
| GT-04843 | B | B | C | - | C |
| GT-04844 | B | B | C | - | C |
| GT-04973 | B | C | C | - | C |
| GT-06411 | - | - | - | C | - |
| GT-06479 | - | - | - | B | - |
| GT-06480 | - | - | - | B | - |
| GT-06481 | - | - | - | A | - |
| GT-04641 | C | - | C | - | C |
| GT-04841 | B | - | C | - | - |

**Table B3. Inhibitory activity (IC₅₀, nM) of the compounds of examples of the present disclosure, designed based on the EGFR inhibitors, on tumor cell proliferation**

| Comp. No. /names | H1975 | H1975-1µm Osimertinib | PC-9 | HCC827 |
|---|---|---|---|---|
| osimertinib | B | F | A+ | A+ |
| HJM-561 | A | D | B | C |
| GT-03740 | - | - | A+ | A+ |
| GT-03741 | - | - | A+ | A+ |
| GT-04260 | A | B | A | A |
| GT-03863 | B | - | A++ | A++ |
| GT-03864 | B | - | A++ | A++ |
| GT-04206 | B | B | B | B |
| GT-03930 | A | C | B | B |
| GT-04214 | A+ | B | A | B |
| GT-05115 | A+ | - | - | - |
| GT-05116 | A+ | - | - | - |
| GT-05117 | A+ | - | - | - |
| GT-05118 | A | - | - | - |
| GT-05119 | A+ | - | - | - |
| GT-05218 | A++ | - | - | - |
| GT-05219 | A++ | - | - | - |
| GT-05281 | A++ | - | - | - |
| GT-05282 | A++ | - | - | - |
| GT-05283 | A++ | - | - | - |
| GT-05837 | B | - | A++ | A++ |
| GT-05838 | B | - | A++ | A++ |
| GT-05839 | B | - | A++ | A+ |
| GT-05840 | B | - | A++ | A+ |
| GT-05841 | - | - | A+ | A+ |
| GT-05870 | B | - | A++ | A+ |
| GT-05871 | B | - | A++ | A+ |
| GT-05915 | A | C | A++ | A++ |
| GT-05917 | B | C | A++ | A++ |
| GT-05940 | B | - | A++ | A++ |
| GT-05941 | B | - | A++ | A+ |
| GT-05942 | - | C | A++ | A+ |
| GT-05943 | - | - | A+ | A+ |
| GT-05947 | B | B | A | B |
| GT-05951 | - | C | B | - |
| GT-05952 | - | C | B | C |
| GT-06020 | - | B | A | B |
| GT-06021 | - | C | B | B |
| GT-06037 | - | A++ | - | - |
| GT-06078 | - | B | - | - |
| GT-06080 | - | C | C | - |
| GT-06082 | B | A+ | B | - |
| GT-06163 | A++ | - | - | - |
| GT-06164 | A++ | - | - | - |
| GT-06165 | A++ | - | - | - |
| GT-06166 | A++ | - | - | - |
| GT-06188 | A++ | A+++ | - | - |
| GT-06176 | A++ | - | - | - |
| GT-06177 | A++ | - | - | - |
| GT-06178 | A++ | - | - | - |
| GT-06190 | A++ | B | - | - |
| GT-06191 | A++ | - | - | - |
| GT-06179 | A+ | - | - | - |
| GT-06229 | A++ | - | - | - |
| GT-06243 | A++ | - | - | - |
| GT-06244 | A++ | - | - | - |
| GT-06245 | A++ | - | - | - |
| GT-06246 | A++ | - | - | - |
| GT-06247 | A+ | A | - | - |
| GT-06252 | A++ | - | - | - |
| GT-06255 | A++ | - | - | - |
| GT-06256 | A++ | - | - | - |
| GT-06257 | A++ | - | - | - |
| GT-06317 | A+ | - | - | - |
| GT-06318 | A+ | - | - | - |
| GT-06319 | A++ | - | - | - |
| GT-06320 | A++ | C | - | - |
| GT-06321 | A+ | - | - | - |
| GT-06326 | A+ | - | - | - |
| GT-06327 | A++ | - | - | - |
| GT-06328 | A++ | - | - | - |
| GT-06329 | A++ | - | - | - |
| GT-06330 | A++ | - | - | - |
| GT-06331 | A+ | - | - | - |
| GT-05284 | A++ | C | - | - |
| GT-07378 | A++ | - | - | - |
| GT-07379 | A++ | - | - | - |
| GT-07380 | A++ | - | - | - |
| GT-07381 | A++ | - | - | - |
| GT-07382 | A+ | - | - | - |
| GT-07383 | A++ | - | - | - |
| GT-07384 | A++ | - | - | - |
| GT-07385 | A++ | - | - | - |
| GT-07386 | A++ | - | - | - |
| GT-07387 | A++ | - | - | - |
| GT-07388 | A++ | - | - | - |
| GT-07389 | A++ | - | - | - |
| GT-07390 | A++ | - | - | - |
| GT-07391 | A++ | - | - | - |
| GT-07392 | A++ | - | - | - |
| GT-07393 | A++ | - | - | - |
| GT-07472 | A++ | - | - | - |
| GT-07489 | A+ | - | - | - |
| GT-07490 | A++ | - | - | - |
| GT-07560 | A++ | - | - | - |
| GT-07561 | A++ | - | - | - |
| GT-07562 | A+ | - | - | - |

| | | | | |
|---|---|---|---|---|
| Note: H1975-1µM Osimertinib cells refer to osimertinib-resistant H1975 cells obtained by treating H1975 cells with 1 µM Osimertinib for 3 months during cell culture. | | | | |

**Table B4. Inhibitory activity (IC₅₀, nM) of the compounds of examples of the present disclosure, designed based on the CDK4/6 inhibitors, on tumor cell proliferation**

| Comp. No. /names | MCF7 | MV-4-11 | SR | 22RV1 | CCRF-CEM | Kasumi-1 | H1975 | MM1S | MM.1R |
|---|---|---|---|---|---|---|---|---|---|
| abemaciclib | D | A | C | A+ | D | C | D | D | D |
| Ribociclib | E | E | E | D | F | D | F | E | F |
| Palbociclib | D | B | D | B | D | D | E | F | F |
| GT-03905 | - | - | - | - | B | A | B | A | A |
| GT-03906 | B | - | A | - | B | A | B | A+ | A |
| GT-06659 | B | B | - | A+ | B | B | - | - | - |
| GT-06664 | - | A+ | B | A | C | B | C | - | - |
| GT-06665 | B | A+ | B | A+ | C | B | B | - | - |
| GT-06666 | B | A+ | B | A | B | B | C | C | |
| GT-06667 | B | A+ | B | A | B | B | - | - | - |
| GT-06668 | A | A | - | - | - | B | - | - | - |
| GT-06735 | - | A+ | A | A+ | B | B | B | B | - |
| GT-06739 | A | A+ | A | A+ | B | A | B | B | - |
| GT-06740 | A | A+ | A+ | A+ | B | A | B | B | - |
| GT-06741 | A | A+ | A+ | A+ | A | B | B | B | - |
| GT-06742 | A+ | A+ | A+ | A+ | A | B | B | B | - |
| GT-06743 | B | A | A | A+ | B | B | B | B | - |
| GT-06838 | A | A+ | A+ | A+ | B | A | B | B | - |
| GT-06839 | A | A+ | A | A+ | B | A+ | B | B | - |
| GT-06840 | A++ | A | A | A | B | B | B | B | - |
| GT-06841 | A | A+ | A+ | A+ | B | A | B | B | - |
| GT-07475 | - | - | - | - | C | - | - | - | - |
| GT-07486 | A | - | - | A+ | B | - | A | A | - |
| GT-07487 | A+ | - | - | A+ | A | - | A | A+ | - |
| GT-07488 | A | - | - | - | B | - | B | A | - |
| GT-06575 | B | - | - | - | - | - | - | - | - |
| GT-06577 | B | - | - | - | - | - | - | - | - |
| GT-06578 | C | - | - | - | - | - | - | - | - |
| GT-06580 | C | - | - | - | - | - | - | - | - |

**Table B5. Inhibitory activity (IC₅₀, nM) of the compounds of examples of the present disclosure, designed based on the CDK2 inhibitors, on tumor cell proliferation**

| Comp. No. /names | MV-4-11 | Kasumi-1 | CCRF-CEM | Comp. No. /names | MV-4-11 | Kasumi-1 | CCRF-CEM |
|---|---|---|---|---|---|---|---|
| FN-1501 | A++ | B | B | GT-06854 | B | - | - |
| GT-06751 | C | - | C | GT-06855 | C | - | - |
| GT-06752 | C | - | - | GT-06856 | B | C | C |
| GT-06753 | B | - | - | GT-06858 | B | - | - |
| GT-06754 | C | - | C | GT-06859 | B | - | - |
| GT-06830 | B | - | - | GT-06860 | B | - | - |
| GT-06831 | B | C | - | GT-06861 | B | C | C |
| GT-06832 | B | C | - | GT-06862 | B | B | - |
| GT-06833 | A | - | - | GT-06834 | B | C | C |

**Table B6. Inhibitory activity (IC₅₀, nM) of the compounds of examples of the present disclosure, designed based on the ALK inhibitors, on tumor cell proliferation**

| Comp. No. /names | SR | H2228 | H1975 | MCF-7 | MDA-MB-231 | MM.1S |
|---|---|---|---|---|---|---|
| brigatinib | B | F | F | D | D | D |
| alectinib | A+ | E | D | E | D | D |
| GT-03603 | A++ | A | C | - | C | C |
| GT-03604 | A++ | A+ | B | - | B | C |
| GT-03781 | A++ | - | C | - | - | - |
| GT-03782 | A+ | - | - | - | - | - |
| GT-03762 | A++ | - | - | - | - | C |
| GT-03763 | A++ | - | - | - | - | B |
| GT-07307 | A+ | B | - | B | - | - |
| GT-07308 | A+ | A+ | - | C | C | - |
| GT-07309 | A+ | B | - | - | C | - |
| GT-07310 | A++ | B | - | C | C | - |
| GT-07311 | A++ | A | C | C | - | C |
| GT-07312 | A++ | A | - | C | - | - |
| GT-07313 | A++ | B | - | - | - | - |
| GT-07323 | A++ | B | - | - | - | C |
| GT-07324 | A+ | B | - | C | - | - |
| GT-07325 | A+ | B | - | - | - | - |
| GT-07326 | A+ | B | - | - | - | - |
| GT-07327 | A++ | B | - | C | - | - |
| GT-07328 | A+ | B | - | - | - | - |
| GT-07345 | A+ | B | C | C | - | - |
| GT-07346 | A+ | B | B | C | - | - |
| GT-07347 | A+ | B | B | - | - | - |
| GT-07348 | A+ | B | C | - | - | - |
| GT-07349 | A | C | C | - | - | - |
| GT-07350 | A | B | C | - | - | - |
| GT-07351 | A+ | B | C | C | - | - |
| GT-07364 | A+ | B | - | B | C | C |
| GT-07365 | B | B | - | - | - | - |
| GT-07592 | A+ | B | - | C | - | - |
| GT-07593 | A+ | A | - | B | - | C |
| GT-07594 | A | B | - | - | - | - |
| GT-07595 | B | C | - | - | - | - |
| GT-07596 | A+ | B | - | B | - | - |
| GT-07597 | A+ | A | - | C | - | C |
| GT-07598 | B | B | - | - | - | - |
| GT-07599 | A+ | B | - | B | - | C |
| GT-07600 | A+ | A | - | B | - | C |
| GT-07601 | A+ | A+ | - | B | - | C |
| GT-07602 | A++ | A+ | B | B | B | B |
| GT-07603 | A+ | A+ | B | B | - | C |
| GT-07604 | A++ | A+ | B | B | C | C |
| GT-07605 | A++ | A | B | B | B | - |
| GT-07606 | A++ | A | B | B | B | C |
| GT-07607 | A++ | A+ | B | B | B | - |
| GT-07608 | A++ | A+ | B | B | B | C |
| GT-07609 | A++ | A+ | B | B | C | C |
| GT-07610 | A++ | A | C | C | - | - |
| GT-07611 | A++ | A+ | C | - | - | - |
| GT-07612 | A++ | A | B | - | C | - |
| GT-07613 | A++ | A | C | C | C | - |
| GT-07614 | A++ | B | C | C | - | - |
| GT-07615 | A++ | A+ | B | B | B | C |
| GT-07616 | A++ | A+ | B | B | C | C |
| GT-07617 | A+ | A | C | B | - | - |
| GT-07691 | A++ | A | B | B | - | - |
| GT-07692 | A+ | B | C | B | C | - |
| GT-07693 | A+ | B | B | B | - | - |
| GT-07694 | A++ | A+ | B | B | - | C |
| GT-07695 | A+ | B | C | B | C | B |
| GT-07696 | A+ | B | C | C | - | - |
| GT-07697 | A+ | B | - | C | - | - |
| GT-07698 | A+ | B | C | B | - | - |
| GT-07699 | A+ | B | C | C | C | - |
| GT-07700 | A++ | A | - | C | - | - |
| GT-07701 | A++ | A | - | C | - | - |
| GT-07702 | A++ | B | C | C | C | C |
| GT-07703 | A++ | A | - | C | C | - |
| GT-07704 | A++ | A | C | - | - | - |
| GT-07705 | A++ | A | C | B | - | - |
| GT-07706 | A++ | A | C | C | - | C |
| GT-07707 | A+ | B | C | C | - | - |
| GT-07708 | A+ | B | B | C | - | - |
| GT-07709 | A | C | - | - | - | - |
| GT-07710 | A+ | C | C | B | - | - |
| GT-07712 | A+ | B | C | - | - | - |
| GT-07713 | A+ | B | - | - | C | - |
| GT-07714 | A+ | B | - | A | B | - |

**Table B7. Inhibitory activity (IC₅₀, nM) of the compounds of examples of the present disclosure, designed based on the BCL-2 inhibitors, on tumor cell proliferation**

| Comp. No. /names | CCRF-CEM | Jurkat | Lncap | TMD8 | LS174T | SW620 | THP-1 |
|---|---|---|---|---|---|---|---|
| ABT-199 | D | D | F | D | F | F | C |
| ABT-263 | B | B | D | D | C | C | B |
| GT-04151 | A+++ | A++ | A+ | A++ | A | A+ | A++ |
| GT-04152 | A++ | A++ | A | A++ | A | A++ | A+ |
| GT-04278 | A++ | A+ | B | A+ | C | B | A+ |
| GT-04279 | A++ | A+ | A | A+ | B | A | A+ |
| GT-07716 | A+ | A | - | - | - | - | - |
| GT-07717 | A+ | A+ | - | - | - | - | - |

**Table B8. Inhibitory activity (IC₅₀, nM) of the compounds of examples of the present disclosure, designed based on the ER inhibitors, on tumor cell proliferation**

| Comp. No. /names | CAL-62 | Vcap | CCRF-CEM | Jurkat | SW620 | BXPC-3 | MGC80-3 |
|---|---|---|---|---|---|---|---|
| ARV-471 | F | F | F | F | D | - | F |
| GT-03856 | B | C | B | B | - | - | - |
| GT-04150 | - | - | - | - | A+++ | - | F |
| GT-04839 | - | - | C | - | C | A | C |
| GT-04840 | - | - | - | - | B | A+ | B |

**Table B9. Inhibitory activity (IC₅₀, nM) of the compounds of examples of the present disclosure, designed based on the FAK inhibitors, on tumor cell proliferation**

| Comp. No. /names | MV-4-11 | SR | CAL-62 | H1975 | SW620 | CCRF-CEM | Jurkat | MM.1R |
|---|---|---|---|---|---|---|---|---|
| Defactinib | A | C | E | D | D | D | D | D |
| PND-1186 | B | - | D | - | - | D | D | C |
| GT-04257 | - | - | - | - | C | B | B | B |
| GT-03599 | - | - | - | - | - | B | - | - |
| GT-03600 | - | A | B | C | A | C | - | C |
| GT-03715 | B | B | - | - | C | C | - | - |
| GT-03717 | B | B | - | C | C | - | - | - |
| GT-03733 | B | A | A | B | B | B | - | C |
| GT-03734 | B | A | A | B | B | B | - | - |
| GT-04094 | - | - | C | - | B | C | - | - |
| GT-04035 | - | - | - | - | - | - | A | - |

**Table B10. Inhibitory activity (IC₅₀, nM) of the compounds of examples of the present disclosure, designed based on the IRAK4 inhibitors, on tumor cell proliferation**

| Comp. No. /names | Jurkat | MV-4-11 | SR | MGC803 | H1299 | MM.1S | Lncap | CCRF-CEM |
|---|---|---|---|---|---|---|---|---|
| PF06650833 | F | D | D | E | E | F | F | E |
| emavusertib | F | A | F | F | F | F | F | F |
| GT-04042 | C | C | C | - | - | - | C | C |
| GT-04043 | B | C | C | - | - | - | C | C |
| GT-04175 | B | C | - | - | - | - | B | C |
| GT-03813 | - | B | B | - | - | - | - | - |
| GT-03814 | - | B | - | - | - | - | - | - |
| GT-03846 | - | A | - | - | - | - | - | - |
| GT-03847 | - | A | - | - | - | - | - | - |
| GT-04273 | A++ | - | A | A+ | A | A++ | B | - |
| GT-04274 | C | - | - | - | - | B | B | C |
| GT-04762 | - | - | - | C | - | - | - | - |
| GT-04956 | - | - | - | - | - | B | - | - |
| GT-04958 | - | - | - | - | - | B | - | - |
| GT-04959 | - | - | - | - | C | C | - | - |
| GT-04968 | B | - | B | B | B | A+ | - | - |
| GT-04969 | B | - | B | B | B | B | - | - |
| GT-04970 | B | - | A | A | B | A | - | - |
| GT-04976 | - | - | B | - | - | B | - | - |
| GT-04977 | - | - | B | - | - | B | - | - |
| GT-04978 | - | - | B | - | - | C | - | - |
| GT-04981 | C | - | B | C | C | B | - | - |
| GT-04982 | B | - | B | B | B | A | - | - |
| GT-04986 | B | - | B | B | B | B | - | - |
| GT-04987 | B | - | B | B | B | B | - | - |
| GT-05020 | C | - | C | - | - | C | - | - |
| GT-05021 | C | - | - | C | - | C | - | - |
| GT-05022 | C | - | C | C | - | C | - | - |
| GT-05023 | C | - | C | - | - | C | - | - |
| GT-05024 | C | - | C | C | - | C | - | - |
| GT-05025 | C | - | C | - | - | C | - | - |
| GT-05053 | - | - | - | - | - | C | - | - |

**Table B11. Inhibitory activity (IC₅₀, nM) of the compounds of examples of the present disclosure, designed based on the CDK9 inhibitors. on tumor cell proliferation**

| Comp. No. /names | DU-145 | SU-DHL-4 | Jurkat | CCRF-CEM | TMD8 |
|---|---|---|---|---|---|
| AT7519 | C | B | B | C | C |
| GT-04050 | A+ | - | B | - | - |
| GT-04051 | B | C | - | C | C |
| GT-04121 | B | C | B | A+ | B |
| GT-04087 | C | C | A+ | A++ | A |

**Table B12. Inhibitory activity (IC₅₀, nM) of the compounds of examples of the present disclosure, designed based on the BCR-ABL inhibitors, on tumor cell proliferation**

| Comp. No. /names | Lncap | CCRF-CEM | CFPAC-1 | SW620 | MGC803 | Jurkat |
|---|---|---|---|---|---|---|
| Dasatinib | E | F | B | D | E | D |
| GT-04027 | B | - | - | B | - | - |
| GT-04059 | - | - | - | B | C | - |
| GT-03936 | B | B | B | B | B | A |
| GT-03937 | A | A | B | A | B | A |

**Table C1. Inhibitory activity (IC₅₀, nM) of the compounds of Formula (II) of the present disclosure on tumor cell proliferation**

| Comp. No. /names | MCF-7 | MDA-MB-231 | MM1S | Comp. No. /names | MCF-7 | MDA-MB-231 | MM1S |
|---|---|---|---|---|---|---|---|
| lenalidomide | F | F | E | GT-07445 | | | C |
| GT-07047 | D | | D | GT-07446 | D | | |
| GT-07050 | D | | D | GT-07447 | D | | |
| GT-07049 | D | | | GT-07553 | | | D |
| GT-07727 | D | | | GT-07552 | | | D |
| GT-07079 | D | | D | GT-07551 | D | | |
| GT-07077 | D | | | GT-07554 | D | | |
| GT-07078 | D | D | | GT-07555 | D | | |
| GT-07199 | D | D | | GT-07557 | | | D |
| GT-07198 | | | D | GT-07633 | D | D | |
| GT-07448 | | | D | GT-07635 | | D | |
| GT-07369 | | | D | GT-07636 | | | D |
| GT-07370 | D | | | GT-07637 | D | | |
| GT-07366 | D | | | GT-07725 | D | | |
| GT-07367 | | | D | GT-07726 | D | | |

| | | | | | | | |
|---|---|---|---|---|---|---|---|
| Note: in Tables B1-B12 and C1, the symbols A+++, A++, A+, A, B, C, D, E, and F are defined as follows: 0 < A+++ ≤ 1 nM; 1 nM < A++ ≤ 10 nM; 10 nM < A+ ≤ 50 nM; 50 nM < A ≤ 100 nM; 100 nM < B ≤ 500 nM; 500 nM < C ≤ 1000 nM; 1000 nM < D ≤ 5000 nM; 5000 nM < E ≤ 10000 nM; 10000 nM < F. Symbol "-" indicates "not detected". | | | | | | | |

The results demonstrated that the compounds of Formula (I) and Formula (II) of the present invention (including the compounds listed in Tables 1 and 2, as well as the Example compounds) effectively inhibited the proliferation of tumor cells (as shown in Tables B1-B12 and C1), with a significantly superior inhibitory effect compared to the corresponding positive control drugs.

### II. Determination of CRBN-binding affinity of compounds:

The CEREBLON BINDING kits (specification: 10,000 tests; product number: Catalog # 64BDCRBNPEH; CISBIO company) was used to determine the CRBN binding ability of the compounds to be tested through a HTRF method (homogeneous time-resolved fluorescence). The specific methods are as follows:
**1.** According to the instructions of the CEREBLON BINDING kits, the compounds to be tested of the present disclosure and lenalidomide were serially diluted using diluent #9 (1X) solution to obtain a final concentration of 2 µM for both the tested compounds and lenalidomide solution.
**2.** 2.5 µL of the above 2 µM of the tested compounds and lenalidomide solution, as well as the same volume of diluent #9 (1X) solution (solvent control group, Std0) were added to each well of a 96-well plate, respectively. Then, 2.5 µL of human Cereblon WT GST-tagged protein solution was added to each well. Finally, 5 µL of the thoroughly mixed Thalidomide-Re reagent and GST Eu antibody working solution were added to each of the aforementioned wells. The final concentration of the tested compounds and lenalidomide in each well was 0.5 µM.
**3.** The blank control wells were sequentially added with 2.5 µL of diluent #9 (1X) solution, 2.5 µL of binding buffer, and 5 µL of thoroughly mixed Thalidomide-Re reagent and GST Eu antibody working solution.
**4.** After sealing and incubating the solutions in the aforementioned wells at room temperature for 3 hours, the absorbance values at emission wavelengths of 620 nm and 665 nm were detected by the HTRF method using a Spark microplate reader (V3.1 SP1).
The corresponding CRBN binding inhibition rate was calculated using the following method: ${R}_{compound} = OD 665 {nm}_{compound} / OD 620 {nm}_{compound} - OD 665 {nm}_{control} / OD 620 {nm}_{control}$ $\begin{matrix}{R}_{Std 0} = OD 665 {nm}_{Std 0} / OD 620 {nm}_{Std 0} - OD 665 {nm}_{control} / OD 620 {nm}_{control} \\ inhibition rate \left(%\right) = \left(1-{R}_{compound}/{R}_{Std 0}\right) * 100\end{matrix}$
Note: OD 665 nm _{compound} is the absorbance value of each well of the compounds to be tested of the present disclosure at an emission wavelength of 665 nm.
OD 620 nm _{compound} is the absorbance value of each well of the compounds to be tested of the present disclosure at an emission wavelength of 620 nm.
OD 665 nm _{control} is the absorbance value of the blank control well at an emission wavelength of 665 nm.
OD 620 nm _{control} is the absorbance value of the blank control well at an emission wavelength of 620 nm.
OD 665 nm _{Std0} is the absorbance value of the solvent control well at an emission wavelength of 665 nm.
OD 620 nm _{Std0} is the absorbance value of the solvent control well at an emission wavelength of 620 nm.

The test results were shown in Tables II-1 and II-2 below.

**Table II-1. HTRF inhibitory activity (IC₅₀, µM) of the compounds of the present disclosure (including the compounds in Tables 1, 2, and the Examples compounds)**

| Comp. No. | HTRF IC₅₀ (µM) | Comp. No. | HTRF IC₅₀ (µM) | Comp. No. | HTRF IC₅₀ (µM) | Comp. No. | HTRF IC₅₀ (µM) |
|---|---|---|---|---|---|---|---|
| Lenalidomide | d | GT-05052 | c | GT-06179 | b | GT-07383 | b |
| GT-07710 | a | GT-05053 | b | GT-06229 | b | GT-07384 | a |
| GT-04684 | c | GT-05015 | a | GT-06243 | a | GT-07385 | a |
| GT-04670 | b | GT-04961 | b | GT-06244 | b | GT-07386 | c |
| GT-04685 | b | GT-04962 | c | GT-06245 | a | GT-07387 | c |
| GT-04738 | c | GT-05016 | b | GT-06246 | a | GT-07388 | a |
| GT-04686 | c | GT-05062 | b | GT-06247 | b | GT-07389 | a |
| GT-04687 | c | afatinib | e | GT-06252 | a | GT-07390 | b |
| PF06650833 | e | osimertinib | e | GT-06253 | b | GT-07391 | a |
| emavusertib | e | erlotinib | e | GT-06254 | a | GT-07392 | c |
| GT-04747 | b | GT-05115 | b | GT-06255 | a | GT-07393 | a |
| GT-04748 | c | GT-05116 | b | GT-06317 | c | GT-07472 | a |
| GT-04749 | c | GT-05117 | b | GT-06318 | c | GT-07473 | b |
| GT-04750 | c | GT-05118 | b | GT-06319 | a | GT-07490 | a |
| GT-04751 | c | GT-05119 | b | GT-06326 | b | GT-07560 | a |
| GT-04752 | c | GT-05753 | b | GT-06327 | b | GT-07562 | a |
| GT-04753 | c | GT-05754 | b | GT-06328 | b | GT-07307 | b |
| GT-04761 | b | GT-05755 | b | GT-06329 | c | GT-07308 | b |
| GT-04762 | c | GT-05756 | b | GT-06331 | c | GT-07309 | b |
| GT-04763 | b | GT-05765 | b | GT-05284 | a | GT-07310 | b |
| GT-04774 | c | GT-05772 | c | GT-05807 | b | GT-07311 | b |
| GT-04775 | c | GT-05773 | b | GT-05808 | b | GT-07313 | c |
| GT-04777 | c | GT-05774 | c | GT-06339 | a | GT-07323 | b |
| GT-04813 | c | GT-05798 | a | GT-06340 | b | GT-07324 | c |
| GT-04814 | b | GT-05799 | a | GT-06341 | b | GT-07325 | b |
| ibrutinib | e | GT-07382 | b | GT-06342 | c | GT-07326 | c |
| Orelabrutinib | e | GT-05801 | b | GT-06490 | b | GT-07328 | a |
| GT-04785 | b | GT-05218 | b | GT-06491 | b | GT-07346 | c |
| GT-04786 | a | GT-05219 | a | GT-06492 | c | GT-07348 | c |
| GT-04787 | b | GT-05281 | a | GT-06493 | b | GT-07364 | b |
| GT-04791 | b | GT-05282 | b | GT-06575 | b | GT-07593 | b |
| GT-04798 | a | GT-05283 | c | GT-06577 | c | GT-05051 | b |
| GT-04799 | a | GT-05838 | b | GT-06578 | c | GT-07598 | b |
| GT-04800 | b | GT-05839 | b | GT-06411 | b | GT-07601 | c |
| GT-04801 | a | GT-05841 | a | GT-06415 | a | GT-07602 | a |
| GT-04802 | b | GT-05870 | b | GT-06416 | c | GT-07604 | c |
| GT-04803 | b | GT-05915 | a | GT-06659 | b | GT-07605 | c |
| GT-04804 | c | GT-05917 | b | GT-06664 | b | GT-07611 | b |
| Enzalutamide | e | GT-05940 | b | GT-06665 | c | GT-07613 | b |
| GT-07713 | b | GT-05941 | c | GT-06666 | a | GT-07614 | c |
| GT-07714 | a | GT-05943 | b | GT-06667 | c | GT-07616 | c |
| GT-04842 | c | GT-05952 | b | GT-06735 | b | GT-07617 | c |
| GT-04843 | c | GT-05999 | c | GT-06739 | b | GT-07695 | c |
| GT-04844 | b | GT-04260 | c | GT-06740 | b | GT-07696 | a |
| GT-04973 | c | GT-06003 | c | GT-06741 | b | GT-07698 | c |
| GT-04958 | b | GT-06036 | c | GT-06743 | c | GT-07699 | b |
| GT-04965 | b | GT-06080 | b | GT-06838 | b | GT-07702 | a |
| GT-04977 | b | GT-06122 | c | GT-06839 | c | GT-07703 | a |
| GT-04978 | c | GT-06123 | b | GT-06750 | b | GT-07704 | c |
| GT-04985 | b | GT-06163 | b | GT-06751 | c | GT-07705 | a |
| GT-05020 | c | GT-06164 | b | GT-06832 | a | GT-07706 | a |
| GT-05021 | c | GT-06165 | a | GT-07093 | b | GT-07709 | b |
| GT-05022 | c | GT-06176 | b | GT-07094 | c | GT-05050 | a |
| GT-05024 | c | GT-06177 | a | GT-07379 | a | GT-06190 | b |
| GT-05029 | b | GT-06178 | a | GT-07380 | a | GT-07381 | a |

In Table II-1, the symbols a, b, c, d, and e are defined as follows: 0<a≤0.5µM, 0.5µM<b≤1µM, 1µM<c<1.9µM, 1.9µM ≤ d≤10µM, 10µM < e.

**Table II-2: Screening of CRBN Binding Affinity of the Compounds of the Present Invention (Including but Not Limited to Compounds Listed in Tables 1 and 2, and Example Compounds) at Concentrations of 2 µM and 0.5 µM**

| Comp. No. | Inhibition rate (%) at 2 µM | Inhibitio n rate (%) at 0.5µM | Comp. No. | Inhibitio n rate (%) at 2 µM | Inhibitio n rate (%) at 0.5µM | Comp. No. | Inhibitio n rate (%) at 2 µM | Inhibitio n rate (%) at 0.5µM |
|---|---|---|---|---|---|---|---|---|
| Lenalidomi de | a4 | a2 | GT-07668 | a5 | a2 | GT-07878 | a5 | a3 |
| GT-07417 | a6 | a3 | GT-07669 | a5 | a1 | GT-07882 | a3 | a3 |
| GT-07419 | a5 | a2 | GT-07675 | a6 | a4 | GT-07759 | a5 | a3 |
| GT-07533 | a5 | a1 | GT-07763 | a6 | a4 | GT-07830 | a6 | a3 |
| GT-07534 | a5 | a1 | GT-07766 | a6 | a3 | GT-03507 | a7 | a3 |
| GT-07536 | a5 | a1 | GT-07767 | a7 | a4 | GT-03508 | a6 | a3 |
| GT-07540 | a5 | a3 | GT-07769 | a7 | a4 | GT-03542 | a7 | a2 |
| GT-07541 | a6 | a4 | GT-07770 | a7 | a3 | GT-04106 | a6 | a4 |
| GT-07542 | a6 | a3 | GT-07774 | a7 | a4 | GT-04107 | a7 | a3 |
| GT-07543 | a6 | a3 | GT-07775 | a7 | a6 | GT-03946 | a5 | a3 |
| GT-07544 | a7 | a5 | GT-07776 | a6 | a2 | GT-04094 | a5 | a1 |
| GT-07547 | a7 | a4 | GT-07777 | a7 | a5 | GT-04095 | a6 | a2 |
| GT-07549 | a7 | a4 | GT-07778 | a7 | a4 | GT-04034 | a7 | a5 |
| GT-07621 | a6 | a5 | GT-07779 | a6 | a2 | GT-04035 | a7 | a4 |
| GT-07623 | a6 | a2 | GT-07781 | a5 | a1 | GT-04121 | a6 | a2 |
| GT-07624 | a6 | a2 | GT-07799 | a6 | a2 | GT-03898 | a7 | a6 |
| GT-07626 | a6 | a2 | GT-07783 | a6 | a3 | GT-03905 | a5 | a4 |
| GT-07628 | a6 | a2 | GT-07789 | a6 | a2 | GT-03781 | a6 | a4 |
| GT-07629 | a7 | a6 | GT-07791 | a6 | a3 | GT-03782 | a7 | a4 |
| GT-07630 | a6 | a2 | GT-07793 | a7 | a6 | GT-03762 | a6 | a4 |
| GT-07476 | a6 | a3 | GT-07795 | a4 | a3 | GT-03763 | a5 | a4 |
| GT-07477 | a6 | a2 | GT-07796 | a7 | a7 | GT-03715 | a6 | a1 |
| GT-07479 | a7 | a5 | GT-07926 | a7 | a6 | GT-03717 | a4 | a3 |
| GT-07482 | a7 | a2 | GT-07946 | a6 | a3 | GT-03733 | a4 | a3 |
| GT-07483 | a7 | a5 | GT-07953 | a5 | a2 | GT-04640 | a7 | a6 |
| GT-07485 | a7 | a5 | GT-07954 | a7 | a6 | GT-04645 | a5 | a2 |
| GT-07487 | a5 | a3 | GT-07960 | a5 | a3 | GT-04643 | a6 | a3 |
| GT-07488 | a7 | a5 | GT-07961 | a6 | a2 | GT-04641 | a6 | a2 |
| GT-07563 | a6 | a2 | GT-07962 | a5 | a2 | GT-04257 | a7 | a6 |
| GT-07591 | a7 | a2 | GT-07760 | a6 | a4 | GT-03603 | a5 | a3 |
| GT-07397 | a6 | a3 | GT-07761 | a5 | a2 | GT-03604 | a5 | a2 |
| GT-07399 | a6 | a1 | GT-07823 | a7 | a5 | GT-07906 | a5 | a2 |
| GT-07403 | a6 | a3 | GT-07825 | a7 | a6 | GT-06984 | a6 | a3 |
| GT-07656 | a6 | a2 | GT-07826 | a5 | a1 | GT-06991 | a5 | a3 |
| GT-07667 | a5 | a2 | GT-07858 | a6 | a3 | | | |

In Table II-2, the symbols a1, a2, a3, a4, a5, a6, and a7 are defined as follows: 10% < a1 ≤ 20%, 20% < a2 ≤ 30%, 30% < a3 ≤ 40%, 40% < a4 ≤ 53%, 53% < a5 ≤ 60%, 60% < a6 ≤ 80%, 80% < a7 ≤ 100%.

The results presented in Tables II-1 and II-2 demonstrate that the compounds of the present invention (including but not limited to those listed in Tables 1 and 2, as well as the Example compounds) exhibit strong binding affinity to CRBN.

### III. Western-blot assay

Conventional Western blot assay was performed to evaluate the effects of the compounds of the present disclosure on the expression of target proteins in cells. Cell lines selected for WB detection based on the target of interest were summarized in Table III-1.

**Table III-1**

| Cell Type | Cell Line | Corresponding Target(s) |
|---|---|---|
| hPBMC Cell | hPBMC Cell | IKZF1/2/3, Wee1, CK1a, GSPT1, IRAK4, CDK2, RSK1, RSK2 |
| Adherent Cells | MDA-MB-231 Cell | CDK4/6, CDK9 |
| | 22RV1 Cell | AR-V7 |
| | LnCap Cell | AR |
| | H1975 Cell | EGFR |
| | H2228 Cell | ALK |
| | MCF-7 Cell | ER |
| | SK-N-AS Cell | NTRK |
| Suspension Cells | Molt-4 Cell | BCL-2, BRM, BRG1 |
| | K562 Cell | BCR-ABL |
| | Daudi Cell | BTK |

(1) Cell Seeding and Protein Harvesting: The selected cells were processed according to the cell seeding procedure and protein harvesting procedure in Table III-2.

**Table III-2 Cell Seeding and Protein Harvesting Procedures**

| Cell Type | Cell Line | Cell Plating Procedures for Various Cell Types |
|---|---|---|
| hPBMC | hPBMC Cell | Human peripheral blood mononuclear cells (hPBMCs) at a density of 1×10⁶ cells/mL were thawed according to the PBMC Recovery Protocol (Milecell Biotechnologies Inc, Shanghai). The cells were then treated with the compounds of the present disclosure (including the compounds listed in Tables 1 and 2, and the Examples compounds) at concentrations of 50 nM and 500 nM. Controls included a negative control (DMSO) and a positive control (the commercial immunomodulatory drug lenalidomide), both processed following the identical experimental protocol as the compounds of the present disclosure. After 24 hours of compounds treatment, cells were harvested and lysed in RIPA protein lysate containing protease inhibitors on ice for 30 min, followed by centrifugation. The supernatant was aspirated as the extracted total cell protein. The protein concentration of the supernatant was determined using the conventional BCA (Bicinchoninic Acid) assay. |
| Suspension Cells | Molt-4, K562, Daudi Cell | Suspension cells (e.g., Molt-4 cells) were added to 12-well plates at a seeding density of 5×10⁵/mL, with a total volume of 1 mL of cell suspension per well, cultured for 12 hours, and then treated with the compounds of the present disclosure (including the compounds listed in Tables 1 and 2, and the Examples compounds) at concentrations of 500 nM and 50 nM. Controls included a negative control (DMSO) and a positive control (corresponding commercial inhibitors), both processed following the identical experimental protocol as the compounds of the present disclosure. After 24 hours of compounds treatment, cells were harvested and lysed in RIPA protein lysate containing protease inhibitors on ice for 30 min, followed by centrifugation. The supernatant was aspirated as the extracted total cell protein. The protein concentration of the supernatant was determined using the conventional BCA assay. |
| Adherent Cells | MDA-MB-231 Cell, 22RV1 Cell, LnCap Cell, H1975 Cell, H2228 Cell, MCF-7 Cell, SK-N-AS Cell | Adherent cells (e.g., MDA-MB-231 cells) were added to 6-well plates at a seeding density of 2.5x10⁵/mL, with a total volume of 2 mL of cell suspension per well, cultured for 12 hours, and then treated with the compounds of the present disclosure (including the compounds listed in Tables 1 and 2, and the Examples compounds) at concentrations of 500 nM and 50 nM. Controls included a negative control (DMSO) and a positive control (corresponding commercial inhibitors), both processed following the identical experimental protocol as the compounds of the present disclosure. After 24 hours of compounds treatment, the culture medium was removed. The cells were washed once with PBS and then lysed with RIPA protein lysate containing protease inhibitors. The resulting lysed proteins were subsequently scraped with a cell scraper, placed on ice for 30 minutes, and centrifuged. The supernatant was aspirated as the extracted total cell protein. The protein concentration of the supernatant was determined using the conventional BCA assay. |

(2) To the collected supernatant, 5X SDS sample loading buffer was added, followed by denaturation at 95°C for 5 minutes. The denatured samples were then separated by SDS-PAGE electrophoresis on a polyacrylamide gel. Proteins were transferred to a nitrocellulose membrane (0.45 µm NC membrane), blocked with blocking buffer at room temperature for 1 hour, and subjected to antibody incubation and development procedures according to the antibody manufacturer's instructions (Cell Signaling Technology).

Half-Maximal Degradation Concentration value (the drug concentration required for degrading proteins by 50%, abbreviated as DC₅₀) reads method: comparing the grayscale values of Western blot bands from drug-treated samples with those from blank DMSO-treated controls, and identifying the drug concentration range at which the grayscale value is half that of the DMSO-treated control bands.

DC₅₀ values can be calculated using ImageJ software to measure grayscale values of Western blot bands from drug-treated samples. The concentration-response curve is fitted to estimate the drug concentration corresponding to half the grayscale value.

Target protein remaining (%) refers to the percentage of target protein remaining in hPBMC cells after treatment with protein degrader compounds.

Calculation method for target protein remaining (%): Use ImageJ software to measure grayscale values of Western blot bands from drug (protein degrader)-treated samples. The grayscale value of drug-treated bands is divided by that of internal control protein bands, and the resulting quotient is normalized to obtain the relative percentage of remaining target protein after protein degrader treatment.

Target protein degradation rate (%) = 100% - target protein remaining (%).

The effects of the compounds of the present disclosure (including the compounds listed in Tables 1 and 2, and the Example compounds) on substrate proteins expression in cells were shown in Tables D1-D16 below.

**Table D1 Degradation of substrate protein IRAK4 by the Example compounds of the present disclosure designed based on IRAK4 inhibitors**

| Comp. No./na mes | IRAK4 | Comp. No./na mes | IRAK4 | Comp. No./na mes | IRAK4 | Comp. No./na mes | IRAK4 | Comp. No./na mes | IRAK4 |
|---|---|---|---|---|---|---|---|---|---|
| PF0665 0833 | B | GT-04887 | A | GT-05022 | A | GT-07414 | A | GT-07549 | A |
| emavus ertib | B | GT-04956 | A | GT-05023 | A | GT-07415 | A | GT-07550 | A |
| GT-04749 | A | GT-04958 | A | GT-05054 | A | GT-07416 | A | GT-07619 | A |
| GT-04750 | A | GT-04968 | A | GT-05190 | A | GT-07417 | A | GT-07622 | A |
| GT-04752 | A | GT-04969 | A | GT-05771 | A | GT-07418 | A | GT-07623 | A |
| GT-04753 | A | GT-04970 | A | GT-05807 | A | GT-07419 | A | GT-07624 | A |
| GT-04761 | A | GT-04976 | A | GT-07408 | A | GT-07532 | A | GT-07625 | A |
| GT-04762 | A | GT-04977 | A | GT-07409 | A | GT-07533 | A | GT-07626 | A |
| GT-04763 | A | GT-04981 | A | GT-07410 | A | GT-07542 | A | GT-07627 | A |
| GT-04774 | A | GT-04982 | A | GT-07411 | A | GT-07543 | A | GT-07628 | A |
| GT-04813 | A | GT-04985 | A | GT-07412 | A | GT-07544 | A | GT-07631 | A |
| GT-04879 | A | GT-04986 | A | GT-07413 | A | GT-07546 | A | GT-07763 | A |
| GT-04175 | A | GT-04176 | A | GT-04177 | A | GT-04274 | A | | |

**Table D2 Degradation of substrate protein EGFR by the Example compounds of the present disclosure designed based on EGFR inhibitors**

| Comp. No./name s | EGFR | Comp. No./name s | EGFR | Comp. No./name s | EGFR | Comp. No./name s | EGFR | Comp. No./name s | EGFR |
|---|---|---|---|---|---|---|---|---|---|
| GT-05115 | A | GT-05943 | A | GT-06121 | A | GT-06256 | A | GT-07388 | A |
| GT-05116 | A | GT-05947 | A | GT-06122 | A | GT-06317 | A | GT-07389 | A |
| GT-05117 | A | GT-05951 | A | GT-06123 | A | GT-06318 | A | GT-07390 | A |
| GT-05118 | A | GT-05952 | A | GT-06163 | A | GT-06319 | A | GT-07391 | A |
| GT-05119 | A | GT-04260 | A | GT-06164 | A | GT-06320 | A | GT-07393 | A |
| GT-05120 | A | GT-06014 | A | GT-06165 | A | GT-06321 | A | GT-03863 | A |
| GT-05838 | A | GT-06015 | A | GT-06166 | A | GT-06326 | A | GT-03864 | A |
| GT-05839 | A | GT-06016 | A | GT-06177 | A | GT-06329 | A | GT-03913 | A |
| GT-05840 | A | GT-06021 | A | GT-06178 | A | GT-06330 | A | GT-03914 | A |
| GT-05841 | A | GT-06022 | A | GT-06190 | A | GT-06331 | A | GT-04206 | A |
| GT-05870 | A | GT-06038 | A | GT-06229 | A | GT-05284 | A | GT-03931 | A |
| GT-05915 | A | GT-06039 | A | GT-06243 | A | GT-07092 | A | GT-04214 | A |
| GT-05917 | A | GT-06078 | A | GT-06244 | A | GT-07093 | A | GT-03740 | A |
| GT-05940 | A | GT-06079 | A | GT-06245 | A | GT-07094 | A | | |
| GT-05941 | A | GT-06083 | A | GT-06246 | A | GT-07383 | A | | |
| GT-05942 | A | GT-06120 | A | GT-06247 | A | GT-07386 | A | | |

**Table D3 Degradation of substrate proteins CDK4 and CDK6 by the Example compounds of the present disclosure designed based on CDK4/6 inhibitors**

| Comp. No./names | CDK4 | CDK6 | Comp. No./names | CDK4 | CDK6 | Comp. No./names | CDK4 | CDK6 |
|---|---|---|---|---|---|---|---|---|
| GT-06339 | A | A | GT-06580 | B | A | GT-06841 | A | A |
| GT-06340 | A | A | GT-06659 | A | A | GT-07474 | A | A |
| GT-06341 | A | A | GT-06664 | A | B | GT-07475 | A | A |
| GT-06342 | A | A | GT-06665 | A | A | GT-07476 | A | A |
| GT-06343 | A | A | GT-06666 | A | A | GT-07477 | A | A |
| GT-06344 | B | A | GT-06667 | A | A | GT-07478 | A | A |
| GT-06490 | A | B | GT-06668 | A | A | GT-07479 | A | A |
| GT-06491 | B | A | GT-06735 | A | A | GT-07480 | A | A |
| GT-06492 | B | A | GT-06739 | A | B | GT-07481 | A | A |
| GT-06493 | A | B | GT-06740 | A | B | GT-07827 | A | A |
| GT-06495 | A | A | GT-06741 | A | B | GT-07830 | A | A |
| GT-06575 | A | A | GT-06743 | A | B | GT-07831 | A | A |
| GT-06576 | A | A | GT-06838 | A | B | GT-07832 | A | A |
| GT-06578 | B | A | GT-06839 | A | A | GT-03905 | A | A |
| GT-06579 | B | A | GT-06840 | A | B | GT-03906 | A | A |

**Table D4 Degradation of substrate protein CDK2 by the Example compounds of the present disclosure designed based on CDK2 inhibitors**

| Comp. No./names | CDK2 | Comp. No./names | CDK2 | Comp. No./names | CDK2 | Comp. No./names | CDK2 |
|---|---|---|---|---|---|---|---|
| GT-06750 | A | GT-06755 | A | GT-06834 | A | GT-06859 | A |
| GT-06751 | A | GT-06830 | A | GT-06854 | A | GT-06860 | A |
| GT-06752 | A | GT-06831 | A | GT-06855 | A | GT-06861 | A |
| GT-06753 | A | GT-06832 | A | GT-06856 | A | GT-06862 | A |
| GT-06754 | A | GT-06833 | A | GT-06858 | A | | |

**Table D5 Degradation of substrate protein ALK by the Example compounds of the present disclosure designed based on ALK inhibitors**

| Comp. No./name s | ALK | Comp. No./name s | ALK | Comp. No./name s | ALK | Comp. No./name s | ALK | Comp. No./name s | ALK |
|---|---|---|---|---|---|---|---|---|---|
| GT-07309 | A | GT-07348 | A | GT-07609 | A | GT-07697 | A | GT-07834 | A |
| GT-07310 | A | GT-07349 | A | GT-07610 | A | GT-07698 | A | GT-07835 | A |
| GT-07312 | A | GT-07351 | A | GT-07611 | A | GT-07699 | A | GT-07836 | A |
| GT-07323 | A | GT-07364 | A | GT-07612 | A | GT-07714 | A | GT-03781 | A |
| GT-07324 | A | GT-07592 | A | GT-07613 | A | GT-07748 | A | GT-03782 | A |
| GT-07325 | A | GT-07593 | A | GT-07614 | A | GT-07749 | A | GT-03762 | A |
| GT-07326 | A | GT-07594 | A | GT-07615 | A | GT-07750 | A | GT-03763 | A |
| GT-07327 | A | GT-07595 | A | GT-07617 | A | GT-07751 | A | GT-03603 | A |
| GT-07328 | A | GT-07596 | A | GT-07691 | A | GT-07755 | A | GT-03604 | A |
| GT-07345 | A | GT-07600 | A | GT-07692 | A | GT-07756 | A | GT-07924 | A |
| GT-07346 | A | GT-07605 | A | GT-07694 | A | GT-07758 | A | | |
| GT-07347 | A | GT-07608 | A | GT-07696 | A | GT-07833 | A | | |

**Table D6 Degradation of substrate protein BCL-2 by the Example compounds of the present disclosure designed based on BCL-2 inhibitors**

| Comp. No./names | BCL-2 |
|---|---|
| GT-07828 | A |
| GT-07910 | A |

**Table D7 Degradation of substrate protein FAK by the Example compounds of the present disclosure designed based on FAK inhibitors**

| Comp. No./name s | FAK | Comp. No./name s | FAK | Comp. No./name s | FAK | Comp. No./name s | FAK | Comp. No./name s | FAK |
|---|---|---|---|---|---|---|---|---|---|
| GT-07400 | A | GT-07510 | A | GT-07670 | A | GT-04085 | A | GT-04178 | A |
| GT-07401 | A | GT-07649 | A | GT-07674 | A | GT-03946 | A | GT-03715 | A |
| GT-07405 | A | GT-07650 | A | GT-07678 | A | GT-03947 | A | GT-03717 | A |
| GT-07406 | A | GT-07652 | A | GT-07680 | A | GT-04094 | A | GT-03733 | A |
| GT-07462 | A | GT-07654 | A | GT-03599 | A | GT-04095 | A | GT-03734 | A |
| GT-07466 | A | GT-07657 | A | GT-03600 | A | GT-04034 | A | GT-03822 | A |
| GT-07507 | A | GT-07658 | A | GT-04084 | A | GT-04035 | A | GT-03823 | A |

**Table D8 Degradation of substrate proteins RSK1 and RSK2 by the Example compounds of the present disclosure designed based on RSK inhibitors**

| Comp. No./names | RSK1 | RSK2 | Comp. No./names | RSK1 | RSK2 | Comp. No./names | RSK1 | RSK2 |
|---|---|---|---|---|---|---|---|---|
| GT-07794 | A | B | GT-07935 | A | B | GT-07950 | A | A |
| GT-07795 | A | B | GT-07936 | A | B | GT-07951 | A | A |
| GT-07796 | A | B | GT-07937 | A | B | GT-07952 | A | A |
| GT-07797 | A | A | GT-07938 | A | B | GT-07953 | A | A |
| GT-07798 | A | A | GT-07939 | A | B | GT-07954 | A | A |
| GT-07925 | A | B | GT-07940 | A | A | GT-07955 | A | A |
| GT-07926 | A | B | GT-07941 | A | A | GT-07956 | A | A |
| GT-07927 | A | A | GT-07942 | A | A | GT-07957 | A | A |
| GT-07928 | A | A | GT-07943 | A | A | GT-07958 | A | A |
| GT-07929 | A | B | GT-07944 | A | A | GT-07959 | A | A |
| GT-07930 | A | B | GT-07945 | A | A | GT-07960 | A | B |
| GT-07931 | A | A | GT-07946 | A | A | GT-07961 | A | A |
| GT-07932 | A | B | GT-07947 | A | A | GT-07962 | A | A |
| GT-07933 | A | B | GT-07948 | A | A | GT-07963 | A | A |
| GT-07934 | A | B | GT-07949 | A | A | | | |

**Table D9 Degradation of substrate protein BTK by the Example compounds of the present disclosure designed based on BTK inhibitors**

| Comp. No./names | BTK | Comp. No./names | BTK |
|---|---|---|---|
| GT-04785 | A | GT-04800 | A |
| GT-04786 | A | GT-04801 | A |
| GT-04788 | A | GT-04802 | A |
| GT-04789 | A | GT-04803 | A |
| GT-04790 | A | GT-04804 | A |
| GT-04791 | A | GT-03507 | A |
| GT-04798 | A | GT-03542 | A |
| GT-04799 | A | | |

**Table D10 Degradation of substrate protein ER by the Example compounds of the present disclosure designed based on ER inhibitors**

| Comp. No./names | ER |
|---|---|
| GT-04684 | A |
| GT-04670 | A |
| GT-04674 | A |
| GT-04149 | A |

**Table D11 Degradation of substrate protein AR by the Example compounds of the present disclosure designed based on AR inhibitors**

| Comp. No./names | AR |
|---|---|
| GT-06411 | A |
| GT-04641 | A |

**Table D12 Degradation of substrate protein ER by the Example compounds of the present disclosure designed based on ER inhibitors**

| Comp. No./names | ER |
|---|---|
| GT-04684 | A |
| GT-04670 | A |
| GT-04674 | A |
| GT-04149 | A |

**Table D13 Degradation of substrate protein CDK9 by the Example compounds of the present disclosure designed based on CDK9 inhibitors**

| Comp. No./names | CDK9 |
|---|---|
| GT-04050 | A |
| GT-04051 | A |
| GT-04074 | A |
| GT-04121 | A |
| GT-04087 | A |

**Table D14 Degradation of substrate protein TRK by the Example compounds of the present disclosure designed based on NTRK inhibitors**

| Comp. No./names | TRK |
|---|---|
| GT-03964 | A |
| GT-03965 | A |
| GT-03898 | A |

**Table D15 Degradation of substrate protein BCR-ABL by the Example compounds of the present disclosure designed based on BCR-ABL inhibitors**

| Comp. No./names | BCR-ABL |
|---|---|
| GT-04058 | A |
| GT-04059 | A |
| GT-03936 | A |
| GT-03937 | A |

**Table D16 Degradation of substrate proteins WEE1, IKZF1, IKZF2, IKZF3, CK1a, and GSPT1 by the Example compounds of the present disclosure designed based on CRBN**

| Comp. No./names | WEE1 | IKZF1 | IKZF2 | IKZF3 | CKla | GSPT1 |
|---|---|---|---|---|---|---|
| lenalidomide | B | A | B | A | B | B |
| GT-07048 | B | A | A | A | A | A |
| GT-07047 | B | A | A | A | A | B |
| GT-07050 | B | A | B | A | A | A |
| GT-07049 | B | B | A | A | A | A |
| GT-07074 | B | B | A | A | B | A |
| GT-07075 | B | B | A | A | B | A |
| GT-07079 | A | A | A | A | B | A |
| GT-07077 | B | B | B | B | B | A |
| GT-07078 | B | B | B | B | B | A |
| GT-07199 | B | B | A | A | A | A |
| GT-07198 | B | B | B | A | B | B |
| GT-07197 | B | B | B | B | A | A |
| GT-07369 | B | B | B | B | A | A |
| GT-07370 | B | B | B | B | A | A |
| GT-07366 | B | B | B | B | A | A |
| GT-07367 | B | B | B | B | A | A |
| GT-07443 | A | A | A | B | A | A |
| GT-07448 | A | A | A | A | A | A |
| GT-07445 | A | A | B | B | A | A |
| GT-07446 | A | A | A | A | A | A |
| GT-07447 | A | A | A | A | A | A |
| GT-07444 | B | B | A | A | B | B |
| GT-07449 | B | B | A | B | B | B |
| GT-07553 | A | A | A | B | B | B |
| GT-07552 | B | B | A | B | B | B |
| GT-07551 | A | B | A | B | B | B |
| GT-07554 | A | A | A | A | A | B |
| GT-07555 | A | A | A | A | A | A |
| GT-07556 | A | A | B | A | A | B |
| GT-07557 | A | A | B | B | A | B |
| GT-07558 | A | A | B | B | A | B |
| GT-07632 | A | A | A | A | A | B |
| GT-07633 | A | B | A | B | A | B |
| GT-07634 | A | A | A | A | A | B |
| GT-07635 | A | A | A | A | A | B |
| GT-07636 | B | A | A | A | B | A |
| GT-07637 | B | A | A | A | A | A |
| GT-07638 | A | B | A | A | B | A |
| GT-07639 | A | A | A | A | A | A |
| GT-07640 | A | A | A | A | B | A |
| GT-07724 | A | A | A | A | B | A |
| GT-07725 | A | A | A | A | B | A |
| GT-07726 | A | A | A | A | B | B |
| GT-07727 | A | A | A | A | B | B |
| GT-07728 | A | A | A | A | B | B |
| GT-07729 | A | A | A | A | B | A |
| GT-07805 | B | A | A | A | B | B |
| GT-07804 | B | B | A | A | B | B |
| GT-07803 | B | A | A | A | B | B |
| GT-07802 | A | B | A | B | B | B |
| GT-07801 | B | B | B | B | A | B |
| GT-07807 | A | B | A | B | B | B |
| GT-07808 | A | A | A | A | B | B |
| GT-07809 | A | A | A | A | B | B |
| GT-07851 | A | B | A | B | B | B |
| GT-07852 | A | B | A | B | A | B |
| GT-07009 | B | B | B | B | A | B |
| GT-07012 | A | B | B | A | B | A |
| GT-07010 | B | B | B | A | A | A |
| GT-06991 | B | B | A | B | B | B |
| GT-07559 | A | A | A | A | A | A |

| | | | | | | |
|---|---|---|---|---|---|---|
| Note: in Tables D1-D16, A is defined as 0% ≤ substrate protein remaining % ≤ 80%, and B is defined as substrate protein remaining % > 80%. | | | | | | |

The degradation effects of the designed and synthesized compounds of Formula (I) and Formula (II) of the present invention on the target substrate proteins were shown in Tables D1-D16 and Figure 1. From the experimental results in Tables D1-D16 and Figure 1, it can be concluded that the compounds of Formula (I) and Formula (II) of the present invention significantly degrade the target substrate proteins. While commercial inhibitors failed to degrade the target proteins, the compounds of Formula (I) of the present invention demonstrated notable degradation efficacy. Compared to lenalidomide, the compounds of Formula (II) of the present invention exhibited significantly enhanced efficiency in inducing substrate protein degradation and also showed selective induction of degradation. In summary, the structural diversity of the compounds of the present invention enables effective degradation of diverse substrate proteins, thereby potentially enabling their use in treating indications associated with these substrates.

### IV. Pharmacokinetic Study of the compounds of the present disclosure

The pharmacokinetic properties of the compounds of the present disclosure were evaluated using conventional pharmacokinetic experimental methods.

The test compounds of the present disclosure (including the compounds in Tables 1 and 2, and the Examples compounds) were orally administered to experimental animals to evaluate their pharmacokinetic properties. The experimental animals used may be those commonly used in pharmacokinetic experiments, e.g., adult and healthy rodents (e.g., mice, rats (such as Sprague-Dawley (SD) rats), guinea pigs) or non-rodents (rabbits, dogs, and monkeys).

The experimental animals were divided into two groups: a control group (for blank plasma collection) and an oral administration group (dosed at 10 mg/kg). Blood samples were collected at predetermined time points after administration, including: 0.25h, 0.5h, 1h, 2h, 4h, 8h, and 24h post-dose. Blank plasma was collected from the control group.

Prior to analysis, plasma samples were processed as follows: To 10 µL of plasma sample were added 10 µL of 50% acetonitrile and 200 µL of internal standard solution (containing 50 ng/mL dexamethasone in acetonitrile). For blank control plasma samples, no internal standard was added, instead an equivalent volume of acetonitrile was supplemented. The processed samples were vortex-mixed, centrifuged, and the supernatant was subjected to LC-MS/MS analysis.

After preparation of calibration curves and QC samples, the concentrations of compounds in plasma samples were determined by LC-MS/MS analysis. The pharmacokinetic parameters of the test compounds were calculated using the pharmacokinetic calculation software WinNonlin v8.1 with non-compartmental analysis based on the plasma concentration data obtained from LC-MS/MS.

The results demonstrated that the compounds of the present disclosure administered via oral route exhibited favorable drug metabolism and pharmacokinetic (DMPK) properties, indicating their potential as therapeutic agents for cancer patients.

### IV. TNF-α Activity Inhibition Assay

PBMCs cells were cultured at 37°C with 5% CO₂ and seeded in 96-well plates at 1x10^7 cells/well. The compounds to be tested (including those listed in Tables 1 and 2, and Examples compounds) were dissolved in DMSO and diluted to appropriate concentrations, ensuring that the final DMSO concentration in cell culture did not exceed 0.5%. After 1-hour incubation in medium with or without the compounds, the cells were stimulated with LPS (1 ng/ml) and continued to be cultured for 18-20 hours. Culture supernatants were then collected, diluted with serum-free medium, and analyzed for TNF-α levels using an ELISA kit. IC₅₀ values were calculated using GraphPad Prism 7.0.

TNF-α downregulation is a key mechanism for the anti-tumor action of immunomodulators. The compounds of the present disclosure demonstrated dose-dependent inhibition of TNF-α levels.

### VI. Effects of the compounds of the present disclosure on TNF-α, IL-10, IL-6, IL-1β, IFN-y, GM-CSF, IL-2, and IL-12p40 release from PBMCs induced by Lipopolysaccharide (LPS) or Phytohemagglutinin (PHA)

The following materials were used in this study:

### VI.1 Reagents and Consumable materials

| **Name** | **Supplier or Source** |
|---|---|
| Human PBMC cell | Shanghai Sai Li |
| Lipopolysaccharide (LPS) | Sigma |
| Phytohemagglutinin (PHA) | MCE |
| RPMI 1640 Medium | Gibco |
| Human TNF-alpha DuoSet ELISA | R&D Systems |
| Human IL-6 DuoSet ELISA | R&D Systems |
| Human IL-10 DuoSet ELISA | R&D Systems |
| Human IFN-gamma DuoSet ELISA | R&D Systems |
| Human IL-2 DuoSet ELISA | R&D Systems |
| Human IL-1 beta ELISA Kit | Abclonal |
| Human GM-CSF ELISA Kit | Abclonal |
| Human IL-12/IL-23 p40 ELISA Kit | Abclonal |

### VI.2 Methods

### VI.2.1 Cell Thawing and Plating

A complete medium was prepared and pre-warmed to 37°C. Cryovials were retrieved from liquid nitrogen, and the area immediately below the cap-notch interface was firmly gripped with forceps. The cryovials were immersed in a 37°C water bath, and shook occasionally to facilitate thawing. The cell suspension was aspirated and transferred to a centrifuge tube containing 10 mL complete medium, followed by thorough mixing and centrifugation at 400 × g for 10 min at room temperature. The supernatant was discarded, and the pellet was resuspended in fresh complete medium. After cell counting and the cell density adjustment, the cell suspension was seeded in 96-well plates at a density of 1×10⁵ cells/well (75 µL/well) and incubated in a incubator for 2 h at 37°C with 5% CO₂ under high humidity conditions.

### VI.2.2 Compound Preparation and Addition to Cell Plates

1) The test compounds were prepared as 10 mM stock solutions in DMSO.
2) The test compounds were serially diluted 5-fold in DMSO starting from 1 mM (highest concentration) to generate 9 concentration gradients.
3) The test compounds were further diluted in culture medium to five times their final working concentration intended for use.
4) The diluted compounds were added to designated wells according to the plate layout at 25 µL/well, achieving final concentrations starting from 1 µM with 5-fold serial dilution (9 concentration points in total).
5) After 1 h incubation, LPS or PHA (25 µL/well) was added to the cell wells to reach final concentrations of: 1 µg/mL LPS (for TNF-α, IL-10, IL-6, GM-CSF and IL-12p40), 5 µg/mL LPS (IL-1β), 50 µg/mL LPS (IFN-γ), or 1 µg/mL PHA (IL-2).
6) The plates were incubated in a incubator for 24 h at 37°C with 5% CO₂ under high humidity. Cell plate layout:

| | 1 | 2 | 3 | 4 | 5 | 6 | 7 | 8 | 9 | 10 | 11 | 12 |
|---|---|---|---|---|---|---|---|---|---|---|---|---|
| A | H₂O | H₂O | H₂O | H₂O | H₂O | H₂O | H₂O | H₂O | H₂O | H₂O | H₂O | H₂O |
| B | H₂O | Cmpd 1# (1 µM Starting, 5-fold dilution, 9 points) | | | | | | | | | LPS/PHA | H₂O |
| C | H₂O | | | | | | | | | | | H₂O |
| D | H₂O | Cmpd 2# (1 µM Starting, 5-fold dilution, 9 points) | | | | | | | | | | H₂O |
| E | H₂O | | | | | | | | | | | H₂O |
| F | H₂O | Cmpd 3# (1 µM Starting, 5-fold dilution, 9 points) | | | | | | | | | Blank | H₂O |
| G | H₂O | | | | | | | | | | | H₂O |
| H | H₂O | H₂O | H₂O | H₂O | H₂O | H₂O | H₂O | H₂O | H₂O | H₂O | H₂O | H₂O |

### VI.2.3 Detection

(1) The cell plate was centrifuged and the culture supernatant was aspirated. TNF-α, IL-10, IL-6, IL-1β, IFN-y, GM-CSF, IL-2, and IL-12p40 cytokines in the culture supernatant were detected according to the ELISA kit instructions.
   The concentrations of TNF-α, IL-10, IL-6, IL-1β, IFN-y, GM-CSF, IL-2, and IL-12p40 in the samples were calculated based on the standard curve. Inhibition rate% = [(Ac-As)/(Ac-Ab)]×100%
   As: OA of the samples (cells + LPS/PHA + test compound)
   Ac: OA of positive cell control (cells + LPS/PHA + DMSO)
   Ab: OA of blank control (cells + culture medium + DMSO)
(2) IC₅₀ value was calculated as follows: using GraphPad Prism 6 software and applying the calculation formula XY-analysis/Nonlinear regression (curve fit)/Dose response-Inhibition/log (inhibitor) vs. response-Variable slope (four parameters) to perform IC₅₀ curve fitting and determine the IC₅₀ values.

The experimental results demonstrated that the compounds of the present disclosure can modulate the production levels of cytokines associated with autoimmune diseases and may be used for the treatment of autoimmune diseases.

### VII. Efficacy experiment of the compounds of the present disclosure on Psoriasis-like skin lesions induced by Imiquimod in Mice

The compounds of the present disclosure (test compounds) were administered orally via gavage once daily for 7 consecutive days to mice with psoriasis-like skin lesions induced by imiquimod, to investigate their efficacy on such lesions.

A total of 60 female C57BL/6J mice (purchased from Shanghai SLAC Laboratory Animal Co., Ltd.) were randomly divided into 6 groups (n=10 per group) based on body weight:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 7 days |
| 2 | Model: Vehicle | 10 | NA | 10 | NA | ig. qd. 7 days |
| 3 | Positive Control | 10 | 3 | 10 | 0.3 | sc. qd. 7 days |
| 4 | Low-dose test compounds group | 10 | 3 | 10 | 0.3 | ig. qd. 7 days |
| 5 | Medium-dose test compounds group | 10 | 10 | 10 | 1 | ig. qd. 7 days |
| 6 | High-dose test compounds group | 10 | 30 | 10 | 3 | ig. qd. 7 days |

| | | | | | | |
|---|---|---|---|---|---|---|
| Note: ig., oral gavage; sc., subcutaneous injection; qd., once daily. | | | | | | |

After grouping the experimental animals, the backs of all mice were shaved. Except for the Control group, the remaining groups were topically treated with imiquimod cream once daily for 7 consecutive days. Simultaneously, each group was administered with a vehicle control, a positive control drug, or the test compounds, according to the above table, once daily for 7 consecutive days.

All clinical symptoms of each animal were observed at the beginning and throughout the experiment. The animals' weights were measured and recorded every 2 days.

### Scoring

Gross Observation Scoring of Skin Lesions (PASI Method): during the last 3 days of modeling and drug administration, the changes in skin lesions of mice in each group were observed once daily. PASI Method: the erythema, scaling, and thickening/infiltration of the lesions were scored separately, and the scores were summed to obtain the total score. PASI Scoring Criteria: 0 = none; 1 = mild; 2 = moderate; 3 = severe; 4 = very severe.

The skin lesions were photographed once daily during the last 3 days of modeling and drug administration.

After the experiment, the mice were euthanized by CO₂ inhalation. Three different areas of skin from the lesion site on the back were collected using a 6 mm punch, weighed, and recorded, and the average weight was calculated.

The skin tissue from the lesion site was homogenized and the levels of IL-23p19, IL-12p40, IL-17, and TNF-α were determined.

The local lesion tissue was fixed, stained with HE, and subjected to pathological scoring (based on epidermal thickness, degree of epidermal keratinization, thickness of the basal layer, and degree of dermal inflammatory cell infiltration). Scoring Criteria: 0 = none; 1 = mild; 2 = moderate; 3 = severe; 4 = very severe.

Experimental data were presented as Mean ± SD. Statistical analysis between two groups was performed using SPSS, with p < 0.05 considered statistically significant.

The experimental results demonstrated that the compounds of the present disclosure improve psoriasis-like skin lesions on the back of mice induced by imiquimod and may be used for the treatment of psoriasis.

### VIII. Efficacy Experiment of the compounds of the present disclosure on the CIA Model in Rats Induced by Type II Collagen

The compounds of the present disclosure (test compounds) were administered orally via gavage once daily for 21 consecutive days to Wistar rats with collagen-induced arthritis (CIA) that was induced using bovine type II collagen (Chondrex, Inc.), in order to investigate their efficacy in the rat arthritis model.

### Preparation of the Modeling Agent:

10 mg of CII (Immunization Grade Bovine Type II Collagen, purchased from Chondrex, Inc.) was dissolved in 5 mL of 0.05M acetic acid solution, allowing it to be fully dissolved, thereby preparing a solution with a concentration of 2 mg/mL, which was then stored at 4°C in the dark overnight. On the day of the experiment, the 2 mg/mL CII solution was mixed with an equal volume of a 4 mg/mL solution of Complete Freund's Adjuvant (CFA, also purchased from Chondrex, Inc.) on ice, and was thoroughly emulsified into an emulsion.

On day 0, 10 Wistar rats (purchased from Beijing Vital River Laboratory Animal Technology Co., Ltd.) were randomly selected as the blank control group and were not immunized. The remaining 100 Wistar rats were intradermally injected at the base of the tail with an emulsion prepared by mixing equal volumes of CII (2 mg/mL) and CFA (4 mg/mL) for the first immunization. A second booster immunization was administered to them on day 7. Between days 10 and 14, i.e., 3 to 7 days after the second booster immunization, 50 rats with an arthritis index (AI) score ranging from 1 to 2 were selected from the modeled animals and were divided into 5 groups based on their body weight, foot volume, and AI score, with 10 rats per group:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 21 days |
| 2 | Model: Vehicle | 10 | NA | 10 | NA | ig. qd. 21 days |
| 3 | Positive Control | 10 | 0.1 | 10 | 0.01 | sc. qd. 21 days |
| 4 | Low-dose test compounds group | 10 | 3 | 10 | 0.3 | ig. qd. 21 days |
| 5 | Medium-dose test compounds group | 10 | 10 | 10 | 1 | ig. qd. 21 days |
| 6 | High-dose test compounds group | 10 | 30 | 10 | 3 | ig. qd. 21 days |

| | | | | | | |
|---|---|---|---|---|---|---|
| Note: ig. = oral gavage; sc. = subcutaneous injection | | | | | | |

Administration commenced between 3 and 7 days after the second booster immunization, once the AI score of the affected paws reached 1-2. According to the table above, animals in each group received daily oral gavage (or subcutaneous injection) of either the vehicle control, positive control drug, or the test compounds for 21 consecutive days.

Beginning on day 3 after the second booster immunization, the volume of both hind paws (or toes) was measured and recorded twice weekly.

Foot Volume Measurement: Before the measurement, a line was marked on the rat's ankle joint with a marker pen to indicate the position. After clean water was added to the instrument, the instrument's reading was reset to zero in preparation for the measurement. The hind limb of the rat was placed in the water so that the marked line at the ankle joint was at the surface of the liquid. The reading obtained by pressing the foot pedal at this time was the foot volume of the rat. After the measurement was completed, the foot pedal was pressed again to reset the instrument to zero, preparing it for the measurement of the next rat.

### Scoring

Arthritis Index (AI): The foot volume was measured twice a week, and the swelling of the four toes was scored simultaneously. Each foot was scored on a scale from 0 to 4, with a maximum score of 16 for each rat.

### Arthritis Index (AI) Scoring Criteria:

| Score | Degree |
|---|---|
| 0 | No swelling, normal appearance |
| 1 | Mild swelling or redness of the ankle, wrist, or finger joints |
| 2 | Moderate swelling or redness of the ankle, wrist, or finger joints |
| 3 | Severe swelling or redness of the ankle, wrist, or finger joints |
| 4 | Very severe swelling or redness of the ankle, wrist, or finger joints |

The day of starting drug administration is recorded as Day 0. Five days after starting drug administration, i.e., 2 hours after Day 5 administration, blood was collected from the jugular vein of the experimental animals, allowed to stand for more than 30 minutes, centrifuged to separate the serum, which was stored at -80°C. The levels of TNF-α, IL-1β, and IL-6 in serum were measured by ELISA.

After the experiment, the animals were euthanized by CO₂ inhalation. The spleen and thymus of the animals were collected and weighed, and the organ coefficients were calculated.

Pathological Examination: One side of the foot joint tissue was fixed in 10% neutral formalin solution, followed by decalcification and paraffin embedding to prepare pathological sections. The sections were then stained with hematoxylin and eosin (HE) for pathological observation.

### Observation Indices:

1. Infiltration of synovial inflammatory cells (lymphocytes, plasma cells);
2. Synovial tissue hyperplasia;
3. Synovial pannus;
4. Fibrinoid necrosis on the synovial surface.
Scoring and Counting Method: 0, 1, 2, 3, and 4 levels, where "0" indicates no observation; "1" is mild; "2" is moderate; "3" is severe; and "4" is extremely severe.

Experimental data were presented as Mean ± SEM. Data between two groups were analyzed using Excel-T test, with p<0.05 considered statistically significant. Scoring data were analyzed using the non-parametric Mann-Whitney U test in SPSS, with p<0.05 considered statistically significant.

The experimental results demonstrated that the compounds of the present disclosure can regulate the serum levels of inflammatory cytokines in rats with collagen-induced arthritis (CIA) model that was induced using bovine type II collagen, and significantly improve the swelling of rat limbs, and thus may be used for the treatment of arthritis.

The above descriptions represent only preferred embodiments of the present invention, but the scope of protection is not limited thereto. Any person skilled in the art, within the technical scope disclosed by the invention, may make equivalent replacements or modifications based on the technical solutions and inventive concepts of the present invention, and such changes shall fall within the protection scope of the invention.

## Claims

1. A compound of Formula (I)
PBM-LIN-ULM Formula (I)
or salts, enantiomers, stereoisomers, solvates, isotopically enriched analogs, prodrugs, or polymorphs thereof,
wherein PBM represents a protein-binding moiety capable of binding to a target protein, and is covalently linked to ULM through LIN;
ULM represents a ligand moiety capable of binding to an E3 ubiquitin ligase, and represents the structure of the following Formula (ULM): 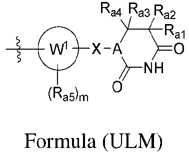
wherein A represents N or CH;
Rₐ₁, Rₐ₂, Rₐ₃, and Rₐ₄ are the same or different and independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
ring W¹ represents arylene or heteroarylene, wherein said ring W¹ is optionally substituted with m Rₐ₅, with each Rₐ₅ being the same or different and independently representing halogen, hydroxy, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl or C₂₋₆ alkynyl;
m represents an integer of 0, 1, 2, 3, or 4;
X represents a bond or -R_{b1}-N(Rₐ₆)-R_{b2}-, where Rₐ₆ represents hydrogen or C₁₋₆ alkyl, and R_{b1} and R_{b2} each independently represent optionally substituted C₀₋₂ alkylene;
LIN is a linker moiety, representing a bond, optionally substituted methylene, or optionally substituted linear or branched C₂₋₁₅ alkylene, wherein one or more groups selected from Rₐ, R_{b}, and any combination thereof are optionally inserted between one or more pairs of adjacent carbon atoms in the backbone carbon chain of the linear or branched C₂₋₁₅ alkylene group; wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and in case that two or more groups Rₐ are inserted into the backbone carbon chain of the linear or branched C₂₋₃₀ alkylene group, the two or more groups Rₐ are not directly connected to each other; and wherein each R_{b} is independently selected from the group consisting of optionally substituted cycloalkylene, optionally substituted arylene, optionally substituted heterocyclylene, optionally substituted heteroarylene, alkynylene, alkenylene, or any combination thereof.

2. The compound of Formula (I) or salts, enantiomers, stereoisomers, solvates, isotopically enriched analogs, prodrugs, or polymorphs thereof as claimed in claim 1, wherein the PBM represents a small molecule ligand that binds to the following protein: epidermal growth factor receptor (EGFR); Cyclin-dependent kinase 4/6 (CDK4/6); Cyclin-dependent kinase 2 (CDK2); anaplastic lymphoma kinase (ALK); activin receptor-like kinase 2 (ALK2); fibroblast growth factor receptors (FGFRs); proto-oncogene tyrosine-protein kinase receptor RET (RET); Focal adhesion kinase (FAK); breakpoint cluster region (BCR)-Abelson leukemia virus (ABL) (BCR-ABL) tyrosine kinase; Bruton tyrosine kinase (BTK); Androgen receptor (AR); Estrogen receptor (ER); Bromodomain and extra-terminal domain protein (BET); Interleukin-1 receptor-associated kinase 4 (IRAK4); Cyclin-dependent kinase 9 (CDK9); Histone-lysine N-methyltransferase EZH2 (EZH2); neurotrophic receptor tyrosine kinase (NTRK); Src homology 2 domain containing protein tyrosine phosphatase (SHP2); Poly (ADP-ribose) polymerase (PARP); signal transducers and activators of transcription 3 (STAT3); FMS-like tyrosine kinase 3 (FLT3); B cell lymphoma-2 (BCL-2) family proteins; SOS Ras/Rac guanine nucleotide exchange factor 1 (SOS1); GTPase Kras (KRAS); SWI/SNF related, matrix associated, actin dependent regulator of chromatin, subfamily a, member 2/4 (SMARCA2/4); or ribosomal protein S6 kinase (RSK).

3. The compound of Formula (I) or salts, enantiomers, stereoisomers, solvates, isotopically enriched analogs, prodrugs, or polymorphs thereof as claimed in claim 1, wherein the PBM comprises the structures of the following formulae: 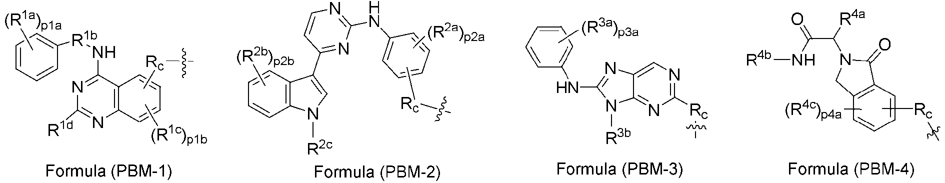 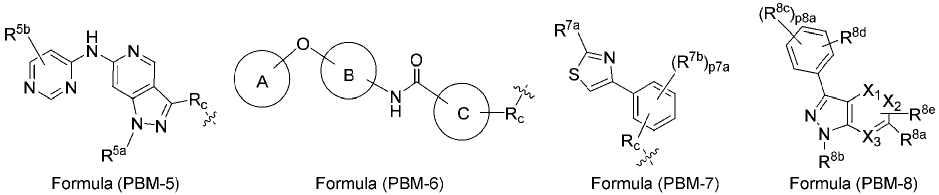 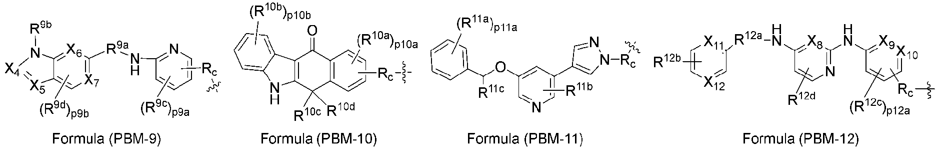 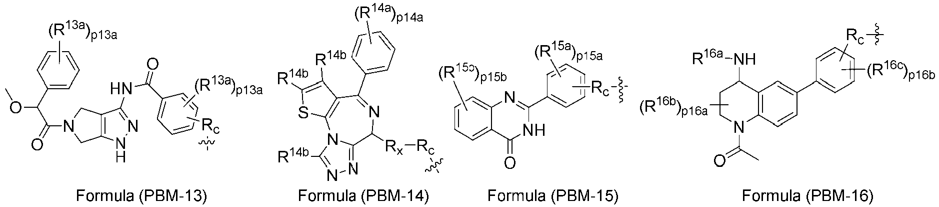 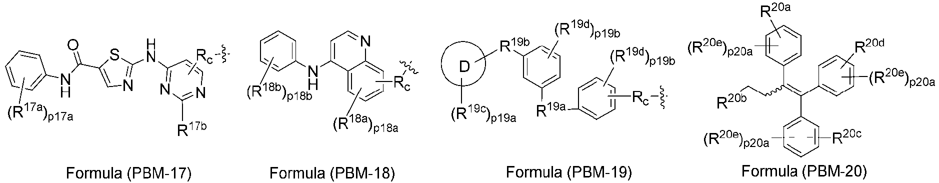 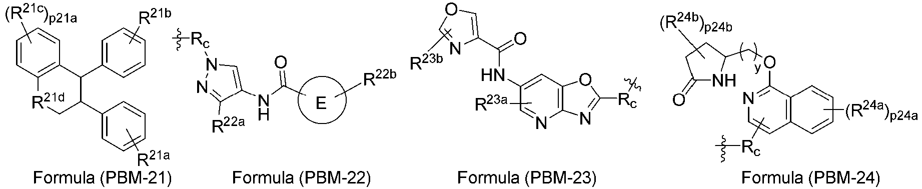 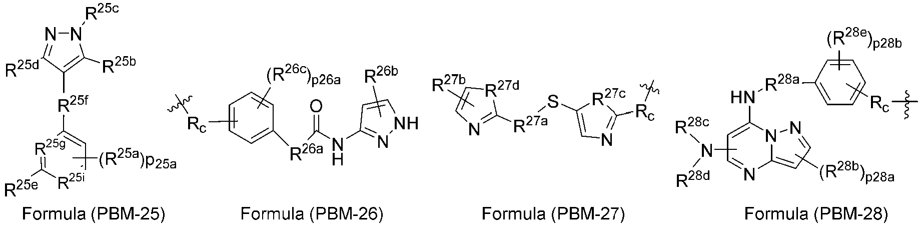 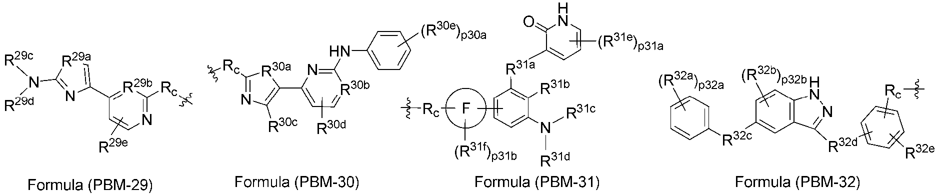 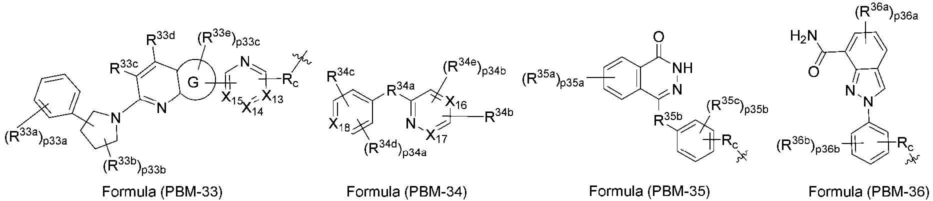 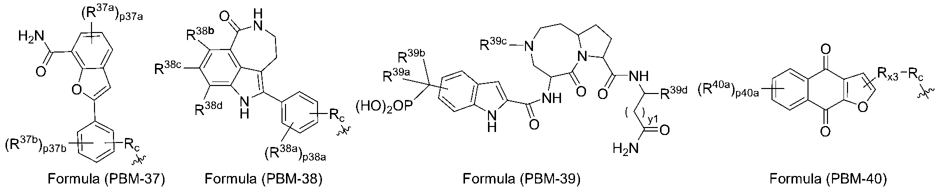 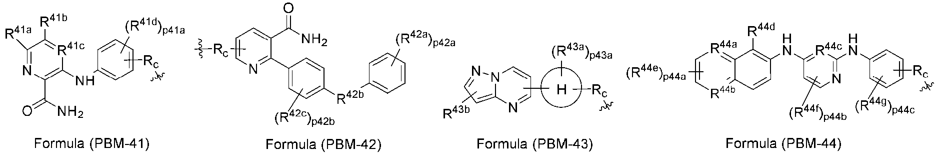 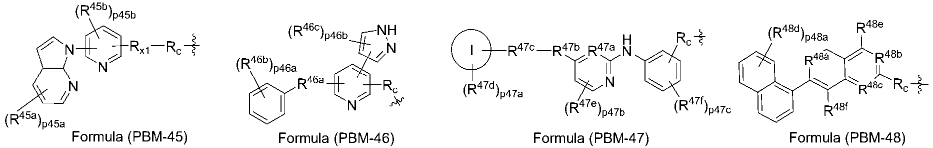 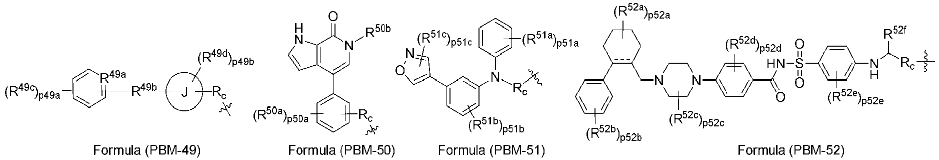 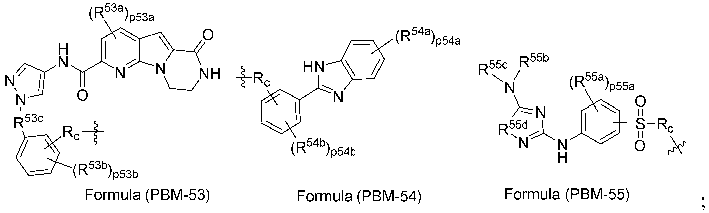 wherein
(R^{1a})ₚ₁ₐ in Formula (PBM-1) indicates that the benzene ring to which it is attached is substituted with p1a R^{1a} groups, with each R^{1a} being the same or different and each independently representing deuterium, halogen, hydroxy, NH₂, NO₂, cyano, C₁₋₆ alkyl, halogenated C₁₋₆ alkyl, deuterated C₁₋₆ alkyl, C₁₋₆ alkoxy, or halogenated C₁₋₆ alkoxy;
p1a represents an integer of 1, 2, 3, 4 or 5;
R^{1b} represents a bond, or C₁₋₂ alkylene optionally substituted with a substituent selected from the group consisting of C₁₋₃ alkyl, deuterated C₁₋₃ alkyl, halogenated C₁₋₃ alkyl, and halogen;
(R^{1c})_{p1b} indicates that the quinazoline ring in Formula (PBM-1) is substituted with p1b R^{1c}, with each R^{1c} being the same or different and each independently representing deuterium, halogen, NO₂, cyano, halogenated C₁₋₆ alkyl, C₁₋₁₀ alkyl, deuterated C₁₋₁₀ alkyl, C₁₋₁₀ alkoxy, halogenated C₁₋₁₀ alkoxy, -O-optionally substituted heterocyclyl, or -NHC(O)R^{1c}, where R^{1c} represents C₁₋₁₀ alkyl, deuterated C₁₋₁₀ alkyl or C₂₋₁₀ alkenyl, e.g., R^{1c} represents methyl, methoxy, 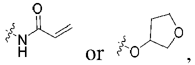 such as 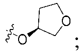
p1b represents an integer of 1, 2, or 3;
R^{1d} represents hydrogen, deuterium or C₁₋₃ alkyl;
(R^{2a})ₚ₂ₐ in Formula (PBM-2) indicates that the benzene ring to which it is attached is substituted with p2a R^{2a}, with each R^{2a} being the same or different and each independently representing deuterium, halogen, nitro, cyano, halogenated C₁₋₆ alkyl, C₁₋₁₀ alkyl, deuterated C₁₋₁₀ alkyl, C₁₋₁₀ alkoxy or -NHC(O)R^{2d}, where R^{2d} represents C₁₋₁₀ alkyl, deuterated C₁₋₁₀ alkyl or C₂₋₁₀ alkenyl, e.g., R^{2a} represents methyl, methoxy, nitro or 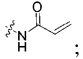
p2a represents an integer of 1, 2, 3 or 4;
(R^{2b})_{p2b} indicates that the 1H-indole ring in Formula (PBM-2) is substituted with p2b R^{2b}, where p2b represents an integer of 0, 1, 2 or 3, and each R^{2b} is identical or different and independently represents deuterium, deuterated C₁₋₁₀ alkyl, C₁₋₁₀ alkyl, halogen or halogenated C₁₋₁₀ alkyl;
R^{2c} represents hydrogen, C₁₋₁₀ alkyl, deuterated C₁₋₁₀ alkyl, halogen or halogenated C₁₋₁₀ alkyl;
(R^{3a})ₚ₃ₐ in Formula (PBM-3) indicates that the benzene ring to which it is attached is substituted with p3a R^{3a}, with each R^{3a} being the same or different and each independently representing deuterium, halogen, hydroxy, C₁₋₆ alkyl, deuterated C₁₋₆ alkyl, halogenated C₁₋₆ alkyl, C₁₋₆ alkoxy, halogenated C₁₋₆ alkoxy, NO₂, NH₂ or cyano;
p3a represents an integer of 0, 1, 2, 3, 4 or 5;
R^{3b} represents substituted or unsubstituted C₃₋₁₅ cycloalkyl, e.g., cyclopentyl;
R^{4a} in Formula (PBM-4) represents C₆₋₁₀ aryl or 5- to 20-membered heteroaryl, where the C₆₋₁₀ aryl and 5- to 20-membered heteroaryl are each independently optionally substituted with one or more substituents, wherein each substituent is independently selected from the group consisting of deuterium, halogen, hydroxy, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy or any combination thereof;
R^{4b} represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), wherein the C₆₋₁₀ aryl and the 5- to 20-membered heteroaryl are each independently optionally substituted with one or more R^{4d}, with each R^{4d} being the same or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
(R^{4c})ₚ₄ₐ indicates that the isoindoline ring in Formula (PBM-4) is optionally substituted with p4a R^{4c}, with each R^{4c} being the same or different and each independently representing deuterium, halogen, hydroxy, C₁₋₆ alkyl, halogenated C₁₋₆ alkyl, deuterated C₁₋₆ alkyl, NO₂, NH₂, C₁₋₆ alkoxy, halogenated C₁₋₆ alkoxy, or cyano;
p4a represents an integer of 0, 1, 2 or 3;
R^{5a} in Formula (PBM-5) represents hydrogen, C₁₋₁₀ alkyl (e.g., 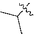
), deuterated C₁₋₁₀ alkyl or halogenated C₁₋₁₀ alkyl;
R^{5b} represents 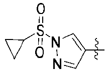 or 4- to 12-membered nitrogen-containing heterocyclyl, wherein the 4- to 12-membered nitrogen-containing heterocyclyl is optionally substituted with one or more substituents, wherein each substituent is independently selected from the group consisting of deuterium, halogen, hydroxy, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, NO₂, NH₂, cyano or any combination thereof;
ring A in Formula (PBM-6) represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), and the ring A is optionally substituted with one or more R^{6a}, with each R^{6a} being the same or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkenyl or C₂₋₆ alkynyl;
ring B in Formula (PBM-6) represents C₃₋₁₅ cycloalkyl or 4- to 20-membered heterocyclyl (e.g., 4- to 20-membered heterocyclyl containing 1 to 4 heteroatoms selected from N, O and S), wherein the ring B is optionally substituted with one or more R^{6b}, with each R^{6b} being the same or different and each independently representing deuterium, halogen, C₁₋₆ alkyl, halogenated C₁₋₆ alkyl, deuterated C₁₋₆ alkyl, C₁₋₆ alkoxy, or halogenated C₁₋₆ alkoxy;
ring C in Formula (PBM-6) represents 5- to 20-membered heteroaryl (e.g., 5- to 20-membered monocyclic or bicyclic heteroaryl containing 1 to 4 heteroatoms selected from N, O and S), wherein the ring C is optionally substituted with one or more R^{6c}, with each R^{6c} being the same or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
R^{7a} in Formula (PBM-7) represents 4- to 20-membered heterocyclyl (e.g., 4- to 20-membered heterocyclyl containing 1 to 4 heteroatoms selected from N, O and S), wherein the 4- to 20-membered heterocyclyl is optionally substituted with one or more R^{7c}, with each R^{7c} being the same or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
(R^{7b})ₚ₇ₐ in Formula (PBM-7) indicates that the benzene ring to which it is attached is substituted with p7a R^{7b}, with each R^{7b} being the same or different and independently representing deuterium, halogen, C₁₋₆ alkyl, halogenated C₁₋₆ alkyl, or deuterated C₁₋₆ alkyl, and p7a represents an integer of 1, 2, 3 or 4;
X₁ in Formula (PBM-8) represents N or CR^{8f}, where R^{8f} represents hydrogen, deuterium or amino, and X₂ represents N or CR^{8g}, where R^{8g} represents hydrogen or a bond, and X₃ represents CH or N;
R^{8a} and R^{8b} each independently represent hydrogen or a bond;
(R^{8c})ₚ₈ₐ in Formula (PBM-8) indicates that the benzene ring to which it is attached is substituted with p8a R^{8c}, with each R^{8c} being the same or different and independently representing -O-aryl, -O-heteroaryl, 4- to 20-membered heterocyclyl, -C₁₋₃ alkylene-NHC(O)-heteroaryl, -C₁₋₃ alkylene-C(O)NH-heteroaryl, C₁₋₆ alkyl, deuterium, deuterated C₁₋₆ alkyl, halogen, or halogenated C₁₋₆ alkyl, wherein the 4- to 20-membered heterocyclyl, aryl and heteroaryl are each independently optionally substituted with one or more R^{8h}, with each R^{8h} being the same or different and each independently representing deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p8a represents an integer of 1, 2, 3 or 4;
R^{8d} represents a bond, hydrogen, halogen, C₁₋₆ alkyl, halogenated C₁₋₆ alkyl, deuterated C₁₋₆ alkyl, halogenated C₁₋₆ alkoxy, or C₁₋₆ alkoxy;
R^{8c} represents hydrogen, halogen, cyano, C₁₋₆ alkyl, halogenated C₁₋₆ alkyl, or amino;
wherein R^{8g}, R^{8a}, R^{8b} and R^{8d} are not simultaneously hydrogen, and only one of R^{8g}, R^{8a}, R^{8b} and R^{8d} represents a bond, wherein
when R^{8a} represents a bond, the ring carbon atom connected to R^{8a} is directly bonded to R_{c}, X₂ represents CH or N, and R^{8b} is hydrogen, and R^{8d} is not a bond;
when X₂ represents CR^{8g}, where R^{8g} represents a bond, the ring carbon atom connected to R^{8g} is directly bonded to R_{c}, and R^{8a} and R^{8b} are hydrogen, and R^{8d} is not a bond;
when R^{8b} represents a bond, the ring nitrogen atom connected to R^{8b} is directly bonded to R_{c}, X₂ represents N or CH, and R^{8a} is hydrogen, R^{8d} is not a bond; and
when R^{8d} represents a bond, the ring carbon atom connected to R^{8d} is directly bonded to R_{c}, and X₂ represents N or CH, and R^{8a} and R^{8b} are hydrogen;
X₄ in Formula (PBM-9) represents CR^{9c} or a fragment 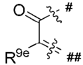
, wherein symbol # indicates the point of attachment to N atom adjacent to X₄, symbol ## indicates the point of attachment to X₅, and each R^{9c} independently represents deuterium, hydrogen, halogen, C₁₋₆ alkyl, halogenated C₁₋₆ alkyl, deuterated C₁₋₆ alkyl, -C(O)-C₁₋₆ alkyl, -C(O)-deuterated C₁₋₆ alkyl or -C(O)NR^{9f}R^{9g}, where R^{9f} and R^{9g} are the same or different and each independently represent hydrogen, C₁₋₆ alkyl, or deuterated C₁₋₆ alkyl;
X₅ in Formula (PBM-9) represents N or CR^{9h}, where R^{9h} represents hydrogen, deuterium, halogen, C₁₋₆ alkyl, deuterated C₁₋₆ alkyl or halogenated C₁₋₆ alkyl;
X₆ and X₇ each independently represent CH or N;
R^{9a} represents a bond or optionally substituted heteroarylene (e.g., heteroarylene substituted with halogen or deuterium);
R^{9b} represents hydrogen, C₁₋₆ alkyl, halogenated C₁₋₆ alkyl, deuterated C₁₋₆ alkyl, or optionally substituted C₃₋₁₂ cycloalkyl;
(R^{9c})ₚ₉ₐ in Formula (PBM-9) indicates that the pyridine ring to which it is attached is optionally substituted with p9a R^{9c}, with each R^{9c} being the same or different and each independently representing deuterium, halogen, C₁₋₆ alkyl, C₁₋₆ alkoxy, halogenated C₁₋₆ alkoxy, deuterated C₁₋₆ alkyl or halogenated C₁₋₆ alkyl, p9a represents an integer of 0, 1, 2 or 3;
(R^{9d})_{p9b} in Formula (PBM-9) indicates that the nitrogen-containing bicyclic heteroaryl ring to which it is attached is optionally substituted with p9b R^{9d}, with each R^{9d} being the same or different and each independently representing deuterium, halogen, C₁₋₆ alkyl, C₁₋₆ alkoxy, halogenated C₁₋₆ alkoxy, deuterated C₁₋₆ alkyl or halogenated C₁₋₆ alkyl, and p9b represents an integer of 0 or 1;
(R^{10a})ₚ₁₀ₐ in Formula (PBM-10) indicates that the benzene ring to which it is attached is optionally substituted with p10a R^{10a}, with each R^{10a} being the same or different and each independently representing deuterium, cyano, halogen, C₁₋₆ alkyl, C₁₋₆ alkoxy, halogenated C₁₋₆ alkoxy, deuterated C₁₋₆ alkyl or halogenated C₁₋₆ alkyl, and p10a represents an integer of 0, 1, 2 or 3;
(R^{10b})_{p10b} indicates that the benzene ring to which it is attached is optionally substituted with p10b R^{10b}, with each R^{10b} being the same or different and each independently representing deuterium, cyano, halogen, C₁₋₆ alkyl, C₁₋₆ alkoxy, halogenated C₁₋₆ alkoxy, deuterated C₁₋₆ alkyl or halogenated C₁₋₆ alkyl, and p10b represents an integer of 0, 1, 2, 3, or 4;
R^{10c} and R^{10d} are the same or different and each independently represent deuterium, C₁₋₆ alkyl, halogen, halogenated C₁₋₆ alkyl, deuterated C₁₋₆ alkyl, C₁₋₆ alkoxy, or halogenated C₁₋₆ alkoxy;
(R^{11a})ₚ₁₁ₐ in Formula (PBM-11) indicates that the benzene ring to which it is attached is substituted with p11a R^{11a}, with each R^{11a} being the same or different and each independently representing deuterium, halogen, hydroxy, amino, cyano, C₁₋₆ alkyl, C₁₋₆ alkoxy, halogenated C₁₋₆ alkoxy, halogenated C₁₋₆ alkyl, or deuterated C₁₋₆ alkyl;
p11a represents an integer of 1, 2, 3, 4 or 5;
R^{11b} represents NR^{11d}R^{11c}, where R^{11d} and R^{11c} are the same or different and each independently represent hydrogen, C₁₋₆ alkyl, halogenated C₁₋₆ alkyl, or deuterated C₁₋₆ alkyl;
R^{11c} represents deuterium, hydrogen, halogen, hydroxy, amino, cyano, C₁₋₆ alkyl, C₁₋₆ alkoxy, halogenated C₁₋₆ alkoxy, halogenated C₁₋₆ alkyl, or deuterated C₁₋₆ alkyl;
X₈, X₉, X₁₀, X₁₁ and X₁₂ in Formula (PBM-12) are the same or different and each independently represent N or CH;
R^{12a} represents a bond or optionally substituted methylene (e.g., halogenated methylene);
R^{12b} represents -C(O)N(R^{12f})R^{12c}, -N(R^{12f})C(O)R^{12c}, -S(O)₂N(R^{12f})R^{12c}, -N(R^{12f})S(O)₂R^{12c}, -S(O)₂R^{12c} or - P(O)(R^{12c})₂, wherein each R^{12c} is the same or different and each independently represents C₁₋₆ alkyl or deuterated C₁₋₆ alkyl, and R^{12f} represents hydrogen or C₁₋₆ alkyl;
(R^{12c})ₚ₁₂ₐ in Formula (PBM-12) indicates that the 6-membered ring containing X₉ and X₁₀ to which it is attached is optionally substituted with p12a R^{12c}, with each R^{12c} being the same or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p12a represents an integer of 0, 1, 2, 3, or 4;
R^{12d} represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
each (R^{13a})ₚ₁₃ₐ in Formula (PBM-13) independently indicates that the benzene ring to which it is attached is optionally substituted with p13a R^{13a}, with each R^{13a} being the same or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
each p13a is the same or different and each independently represents an integer of 0, 1, 2, 3, or 4 ;
(R^{14a})ₚ₁₄ₐ in Formula (PBM-14) indicates that the benzene ring to which it is attached is substituted with p14a R^{14a}, wherein p14a represents an integer of 0, 1, 2, 3, 4 or 5, and each R^{14a} is the same or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
each R^{14b} is the same or different and each independently represents halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
Rₓ in Formula (PBM-14) represents a bond or -C₁₋₃ alkylene-C(O)-;
(R^{15a})ₚ₁₅ₐ in Formula (PBM-15) indicates that the benzene ring to which it is attached is optionally substituted with p15a R^{15a}, with each R^{15a} being the same or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p15a represents an integer of 0, 1, 2, 3, or 4 ;
(R^{15b})_{p15b} indicates that the 4-oxo-3,4-dihydroquinazoline ring to which it is attached is substituted with p15b R^{15b}, with each R^{15b} being the same or different and each independently representing halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p15b represents an integer of 1, 2, 3 or 4 ;
R^{16a} in Formula (PBM-16) represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), wherein the C₆₋₁₀ aryl and 5- to 20-membered heteroaryl are each independently optionally substituted with one or more R^{16d}, wherein each R^{16d} is identical or different and independently represents deuterium, halogen, hydroxy, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkenyl or C₂₋₆ alkynyl;
(R^{16b})ₚ₁₆ₐ in Formula (PBM-16) indicates that 1,2,3,4-tetrahydronaphthalene ring to which it is attached is substituted with p16a R^{16b}, with each R^{16b} being the same or different and each independently representing deuterium, halogen, C₁₋₆ alkoxy, halogenated C₁₋₆ alkoxy, C₁₋₆ alkyl, deuterated C₁₋₆ alkyl, or halogenated C₁₋₆ alkyl;
p16a represents an integer of 0, 1, 2, 3, or 4 ;
(R^{16c})_{p16b} in Formula (PBM-16) indicates that the benzene ring to which it is attached is substituted with p16b R^{16c}, with each R^{16c} being the same or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p16b represents an integer of 0, 1, 2, 3, or 4 ;
(R^{17a})ₚ₁₇ₐ in Formula (PBM-17) indicates that the benzene ring to which it is attached is substituted with p17a R^{17a}, with each R^{17a} being the same or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p17a represents an integer of 1, 2, 3, 4 or 5 ;
R^{17b} represents halogen, C₁₋₆ alkyl, deuterated C₁₋₆ alkyl, or halogenated C₁₋₆ alkyl;
(R^{18a})ₚ₁₈ₐ in Formula (PBM-18) indicates that the quinazoline ring to which it is attached is substituted with p18a R^{18a}, with each R^{18a} being the same or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p18a represents an integer of 1, 2, 3 or 4 ;
(R^{18b})_{p18b} in Formula (PBM-18) indicates that the benzene ring to which it is attached is substituted with p18b R^{18b}, with each R^{18b} being the same or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p18b represents an integer of 1, 2, 3, 4 or 5 ;
R^{19a} in Formula (PBM-19) represents -NHC(O)- or -C(O)NH-;
R^{19b} represents -NH-, -N(C₁₋₆ alkyl)- or ethynylene;
ring D in Formula (PBM-19) represents a 5- to 20-membered monocyclic, bicyclic, or polycyclic heteroaryl group containing 1 to 4 heteroatoms selected from N, O, and S, and (R^{19c})ₚ₁₉ₐ indicates that the ring D is optionally substituted with p19a R^{19c}, with each R^{19c} being the same or different and each independently representing optionally substituted 5- to 15-membered heteroaryl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p19a represents an integer of 0, 1, 2, or 3;
each (R^{19d})_{p19b} in Formula (PBM-19) independently indicates that the benzene ring to which it is attached is independently optionally substituted with p19b R^{19d}, with each R^{19d} being the same or different and each independently representing deuterium, halogen, hydroxy, cyano, amino, C₁₋₆ alkoxy, halogenated C₁₋₆ alkoxy, C₁₋₆ alkyl, deuterated C₁₋₆ alkyl, or halogenated C₁₋₆ alkyl;
each p19b is the same or different and each independently represents an integer of 0, 1, 2, 3, or 4 ;
R^{20a}, R^{20b}, R^{20c} and R^{20d} in Formula (PBM-20) each independently represent a bond, hydrogen, deuterium, halogen, hydroxy, cyano, amino, C₁₋₆ alkoxy, halogenated C₁₋₆ alkoxy, C₁₋₆ alkyl, deuterated C₁₋₆ alkyl, or halogenated C₁₋₆ alkyl, wherein only one of R^{20a}, R^{20b}, R^{20c} and R^{20d} represents a bond, and the remaining three are the same or different and each independently represents hydrogen, halogen, or hydroxy, wherein when one of R^{20a}, R^{20b}, R^{20c} and R^{20d} represents a bond, the benzene ring or methylene group in Formula (PBM-20) to which it is attached is directly bonded to R_{c};
symbol " 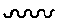 " attached to the double bond in Formula (PBM-20) represents a bond that may be in stereo-configuration (cis or trans configuration, or E- or Z-configuration);
each (R^{20c})ₚ₂₀ₐ in Formula (PBM-20) independently indicates that the benzene ring to which it is attached is independently optionally substituted with p20a R^{20c}, with each R^{20c} being the same or different and each independently representing deuterium, halogen, hydroxy, cyano, amino, C₁₋₆ alkoxy, halogenated C₁₋₆ alkoxy, C₁₋₆ alkyl, deuterated C₁₋₆ alkyl, or halogenated C₁₋₆ alkyl;
each p20a is the same or different and each independently represents an integer of 0, 1, 2, 3, or 4 ;
R^{21d} in Formula (PBM-21) represents O, S or CH₂;
(R^{21c})ₚ₂₁ₐ represents p21a R^{21c} groups, with each R^{21c} being the same or different and each independently representing deuterium, halogen, hydroxy, cyano, amino, C₁₋₆ alkoxy, halogenated C₁₋₆ alkoxy, C₁₋₆ alkyl, deuterated C₁₋₆ alkyl, or halogenated C₁₋₆ alkyl;
p21a represents an integer of 0, 1, 2, 3, or 4 ;
only one of R^{21a} and R^{21b} represents a bond, and the other represents hydrogen, halogen, hydroxy, cyano, amino, C₁₋₆ alkoxy, halogenated C₁₋₆ alkoxy, C₁₋₆ alkyl, deuterated C₁₋₆ alkyl, or halogenated C₁₋₆ alkyl, wherein when one of R^{21a} and R^{21b} represents a bond, the benzene ring carbon atom in Formula (PBM-21) to which it is attached is directly bonded to R_{c} ;
ring E in Formula (PBM-22) represents a 5- to 20-membered monocyclic, bicyclic, or polycyclic heteroarylene group containing 1 to 4 heteroatoms selected from N, O, and S;
R^{22b} represents optionally substituted 5- to 15-membered heteroaryl, optionally substituted 5- to 15-membered heterocyclyl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R^{22a} represents hydrogen, halogen, C₁₋₆ alkyl, halogenated C₁₋₆ alkyl, deuterated C₁₋₆ alkyl, C₁₋₆ alkoxy, or halogenated C₁₋₆ alkoxy;
R^{23a} in Formula (PBM-23) represents optionally substituted 5- to 15-membered heterocyclyl, optionally substituted 5- to 15-membered heteroaryl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R^{23b} represents a 5- to 20-membered monocyclic, bicyclic, or polycyclic heteroaryl group containing 1 to 4 heteroatoms selected from N, O, and S, and the heteroaryl is optionally substituted with one or more substituents selected from the group consisting of deuterium, halogen, hydroxy, cyano, amino, C₁₋₆ alkyl, halogenated C₁₋₆ alkyl, deuterated C₁₋₆ alkyl, C₁₋₆ alkoxy, halogenated C₁₋₆ alkoxy or any combination thereof;
(R^{24a})ₚ₂₄ₐ indicates that the isoquinoline ring in Formula (PBM-24) is optionally substituted with p24a R^{24a}, with each R^{24a} being the same or different and each independently representing deuterium, halogen, hydroxy, cyano, amino, C₁₋₆ alkyl, C₁₋₆ alkoxy, halogenated C₁₋₆ alkoxy, -C(O)NH₂, deuterated C₁₋₆ alkyl or halogenated C₁₋₆ alkyl;
p24a represents an integer of 0, 1, 2, 3, or 4 ;
(R^{24b})_{p24b} indicates that the pyrrolidinone ring in Formula (PBM-24) is optionally substituted with p24b R^{24b}, with each R^{24b} being the same or different and each independently representing deuterium, halogen, hydroxy, cyano, amino, C₁₋₆ alkyl, C₁₋₆ alkoxy, halogenated C₁₋₆ alkoxy, -C(O)NH₂, deuterated C₁₋₆ alkyl or halogenated C₁₋₆ alkyl;
p24b represents an integer of 0, 1, 2, 3, or 4 ;
y in Formula (PBM-24) represents an integer of 1, 2 or 3;
R^{25g} in Formula (PBM-25) represents N or CR^{25h}, where R^{25h} represents hydrogen or halogen, R²⁵ⁱ represents N or CR^{25j}, where R^{25j} represents hydrogen or halogen;
R^{25f} represents a bond, -C(O)NH-, -NHC(O)- , -S(O)₂NH- or -NHS(O)₂-;
(R^{25a})ₚ₂₅ₐ indicates that the 6-membered ring containing R^{25g} and R²⁵ⁱ in Formula (PBM-25) to which it is attached is optionally substituted with p25a R^{25a}, with each R^{25a} being the same or different and each independently representing deuterium, halogen, hydroxy, cyano, amino, C₁₋₆ alkyl, C₁₋₆ alkoxy, halogenated C₁₋₆ alkoxy, deuterated C₁₋₆ alkyl, or halogenated C₁₋₆ alkyl;
p25a represents an integer of 0, 1 or 2 ;
R^{25b} represents hydrogen, halogen, C₁₋₆ alkyl, deuterated C₁₋₆ alkyl, halogenated C₁₋₆ alkyl or -C₁₋₃ alkylene-C₃₋₆ cycloalkyl;
R^{25c} represents hydrogen, C₁₋₆ alkyl, deuterated C₁₋₆ alkyl or halogenated C₁₋₆ alkyl; or
R^{25b} and R^{25c} together with their respective attached nitrogen and carbon atoms form an optionally substituted 5- to 7-membered nitrogen-containing heterocycle;
R^{25d} represents hydrogen or a bond, and R^{25e} represents hydrogen or a bond, wherein R^{25d} and R^{25e} are not simultaneously hydrogen, and only one of R^{25d} and R^{25e} represents a bond, and the other represents hydrogen, wherein when R^{25d} represents a bond, the ring carbon atom connected to R^{25d} is directly connected to R_{c} , and when R^{25e} represents a bond, the ring carbon atom connected to R^{25e} is directly connected to R_{c} ;
R^{26a} in Formula (PBM-26) represents a bond, C₁₋₃ alkylene or halogenated C₁₋₃ alkylene;
R^{26b} represents halogen, optionally substituted C₁₋₆ alkyl, C₁₋₆ alkoxy, halogenated C₁₋₆ alkoxy, or optionally substituted C₃₋₁₅ cycloalkyl;
(R^{26c})ₚ₂₆ₐ indicates that the benzene ring in Formula (PBM-26) is optionally substituted with p26a R^{26c}, with each R^{26c} being the same or different and each independently representing deuterium, halogen, hydroxy, cyano, amino, C₁₋₆ alkyl, C₁₋₆ alkoxy, halogenated C₁₋₆ alkoxy, deuterated C₁₋₆ alkyl, or halogenated C₁₋₆ alkyl;
p26a represents an integer of 0, 1, 2 , 3 or 4;
R^{27a} in Formula (PBM-27) represents C₁₋₃ alkylene or halogenated C₁₋₃ alkylene;
R^{27b} represents deuterium, C₁₋₆ alkyl, deuterated C₁₋₆ alkyl, halogenated C₁₋₆ alkyl, or optionally substituted C₃₋₆ cycloalkyl;
R^{27c} and R^{27d} each independently represent S or O;
R^{28a} in Formula (PBM-28) represents C₁₋₃ alkylene or halogenated C₁₋₃ alkylene;
(R^{28b})ₚ₂₈ₐ indicates that the pyrazolo[1,5-a]pyrimidinyl ring to which it is attached is optionally substituted with p28a R^{28b}, with each R^{28b} being the same or different and each independently representing hydroxy, halogen, C₁₋₆ alkyl, C₁₋₆ alkoxy, halogenated C₁₋₆ alkoxy, deuterated C₁₋₆ alkyl, or halogenated C₁₋₆ alkyl;
p28a represents an integer of 0, 1, 2 or 3;
R^{28c} and R^{28d} are the same or different and each independently represent hydrogen, optionally substituted C₁₋₁₀ alkyl or optionally substituted C₃₋₆ cycloalkyl;
(R^{28e})_{p28b} indicates that the benzene ring to which it is attached is optionally substituted with p28b R^{28e} , with each R^{28e} being the same or different and each independently representing halogen, C₁₋₆ alkyl, C₁₋₆ alkoxy, halogenated C₁₋₆ alkoxy, deuterated C₁₋₆ alkyl, or halogenated C₁₋₆ alkyl;
p28b represents an integer of 0, 1, 2 , 3 or 4;
R^{29a} in Formula (PBM-29) represents -S- or a fragment 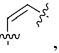 wherein when R^{29a} represents -S-, heteroaryl containing R^{29a} and nitrogen atom in Formula (PBM-29) is thiazolyl, and when R^{29a} represents the fragment 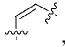 the heteroaryl containing R^{29a} and nitrogen atom in Formula (PBM-29) is pyridyl;
R^{29b} represents N or CH;
R^{29c} represents hydrogen oroptionally substituted C₁₋₁₀ alkyl;
R^{29d} represents hydrogen, -C₁₋₃ alkylene-optionally substituted 4- to 10-membered heterocyclyl, or optionally substituted 4- to 10-membered heterocyclyl;
R^{29e} represents hydroxy, halogen, C₁₋₆ alkyl, C₁₋₆ alkoxy, halogenated C₁₋₆ alkoxy, deuterated C₁₋₆ alkyl, or halogenated C₁₋₆ alkyl;
R^{30a} in Formula (PBM-30) represents O or S;
R^{30b} represents N or CH;
R^{30c} and R^{30d} are the same or different and each independently represent hydroxy, halogen, C₁₋₆ alkyl, C₁₋₆ alkoxy, halogenated C₁₋₆ alkoxy, deuterated C₁₋₆ alkyl, or halogenated C₁₋₆ alkyl;
(R^{30e})ₚ₃₀ₐ indicates that the benzene ring to which it is attached is optionally substituted with p30a R^{30e}, with each R^{30e} being the same or different and each independently representing deuterium, hydroxy, halogen, amino, cyano, C₁₋₆ alkoxy, halogenated C₁₋₆ alkoxy, C₁₋₆ alkyl, deuterated C₁₋₆ alkyl, halogenated C₁₋₆ alkyl, - S(O)₂NH₂ or -C(O)NH₂;
p30a represents an integer of 0, 1, 2 , 3, 4 or 5;
R^{31a} in Formula (PBM-31) represents C(O)NH, NHC(O), (CH₂)₁₋₂C(O)NH, (CH₂)₁₋₂NHC(O), C(O)NH(CH₂)₁₋₂, NHC(O)(CH₂)₁₋₂, S(O)₂NH or NHS(O)₂;
R^{31b} represents hydrogen, hydroxy, halogen, cyano, C₁₋₆ alkoxy, halogenated C₁₋₆ alkoxy, C₁₋₆ alkyl, deuterated C₁₋₆ alkyl, or halogenated C₁₋₆ alkyl;
R^{31c} represents hydrogen, C₁₋₆ alkyl, deuterated C₁₋₆ alkyl, or halogenated C₁₋₆ alkyl; or
R^{31b} and R^{31c}, together with their respective attached carbon and nitrogen atoms and the carbon atom on the benzene ring attached to the nitrogen atom, form an optionally substituted 4- to 6-membered heteroaromatic ring or an optionally substituted 4- to 6-membered heterocycle ring;
R^{31d} represents optionally substituted C₃₋₆ cycloalkyl, C₁₋₆ alkyl, deuterated C₁₋₆ alkyl, halogenated C₁₋₆ alkyl or optionally substituted 4- to 20-membered heterocyclyl;
(R^{31e})ₚ₃₁ₐ indicates that pyridin-2(1H)-one ring to which it is attached is substituted with p31a R^{31e}, with each R^{31e} being the same or different and each independently representing hydroxy, halogen, cyano, C₁₋₆ alkyl, C₁₋₆ alkoxy, halogenated C₁₋₆ alkoxy, deuterated C₁₋₆ alkyl, or halogenated C₁₋₆ alkyl;
p31a represents an integer of 0, 1, 2 or 3;
ring F in Formula (PBM-31) represents arylene or 5- to 20-membered monocyclic or bicyclic heteroarylene containing from 1 to 4 heteroatoms selected from the group consisting of oxygen, nitrogen, and sulfur; and (R^{31f})_{p31b} indicates that the ring F is optionally substituted with p31b R^{31f}, with each R^{31f}being the same or different and each independently representing deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p31b represents an integer of 0, 1, 2, 3, or 4;
R^{32c} in Formula (PBM-32) represents optionally substituted C₁₋₃ alkylene;
(R^{32a})ₚ₃₂ₐ indicates that the benzene ring to which it is attached is substituted with p32a R^{32a}, with each R^{32a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p32a represents an integer of 0, 1, 2, 3, 4 or 5;
(R^{32b})_{p32b} indicates that the 1H-indazole ring to which it is attached is substituted with p32b R^{32b}, with each R^{32b} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p32b represents an integer of 0, 1, 2, 3, 4 or 5;
R^{32d} represents C(O)NH, NHC(O), S(O)₂NH or NHS(O)₂;
R^{32e} represents NR^{32f}R^{32g}, where R^{32f} and R^{32g} are the same or different and each independently represent hydrogen, C₁₋₆ alkyl, deuterated C₁₋₆ alkyl, halogenated C₁₋₆ alkyl, optionally substituted C₃₋₆ cycloalkyl or optionally substituted 4- to 8-membered heterocyclyl;
X₁₃, X₁₄ and X₁₅ in Formula (PBM-33) are the same or different and each independently represent CH or N, wherein X₁₃, X₁₄ and X₁₅ are not simultaneously N, and the 6-membered ring containing X₁₃, X₁₄ and X₁₅ is optionally substituted with 1 or 2 substituents selected from the group consisting of halogen, hydroxy, cyano, amino, C₁₋₆ alkyl, C₁₋₆ alkoxy, halogenated C₁₋₆ alkoxy, deuterated C₁₋₆ alkyl, halogenated C₁₋₆ alkyl or any combination thereof;
ring G in Formula (PBM-33) represents pyrrole ring, imidazole ring, pyrazole ring, benzene ring, pyridine ring, pyrimidine ring, or pyrazine ring;
(R^{33a})ₚ₃₃ₐ indicates that the benzene ring to which it is attached is optionally substituted with p33a R^{33a}, with each R^{33a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p33a represents an integer of 0, 1, 2, 3, 4 or 5;
(R^{33b})_{p33b} indicates that the pyrrolidine ring to which it is attached is optionally substituted with p33b R^{33b}, with each R^{33b} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p33b represents an integer of 0, 1, 2, 3, 4, 5 or 6;
R^{33c} and R^{33d} are the same or different and each independently represent hydrogen, halogen, hydroxy, amino, cyano, C₁₋₆ alkyl, C₁₋₆ alkoxy, halogenated C₁₋₆ alkoxy, deuterated C₁₋₆ alkyl, or halogenated C₁₋₆ alkyl;
(R^{33c})_{p33c} indicates that the ring G to which it is attached is optionally substituted with p33c R^{33e}, with each R^{33e} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p33c represents an integer of 0 or 1;
X₁₆, X₁₇ and X₁₈ in Formula (PBM-34) each independently represent CH or N;
R^{34a} represents a bond, S or NH;
R^{34b} represents a bond, optionally substituted cycloalkyl or optionally substituted 4- to 15-membered nitrogen-containing heterocyclyl;
R^{34c} represents a bond, halogen, amino, hydroxy, cyano, C₁₋₆ alkyl, C₁₋₆ alkoxy, halogenated C₁₋₆ alkoxy, deuterated C₁₋₆ alkyl, halogenated C₁₋₆ alkyl, optionally substituted cycloalkyl or optionally substituted 4- to 15-membered nitrogen-containing heterocyclyl;
wherein R^{34b} and R^{34c} do not simultaneously represent a bond, and only one of R^{34b} and R^{34c} represents a bond, wherein when R^{34b} represents a bond, the ring carbon atom connected to R^{34b} is directly bonded to R_{c}, and when R^{34c} represents a bond, the ring carbon atom connected to R^{34c} is directly bonded to R_{c} ;
(R^{34d})ₚ₃₄ₐ indicates that the 6-membered ring to which it is attached is optionally substituted with p34a R^{34d}, with each R^{34d} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p34a represents an integer of 0, 1, 2, 3, or 4;
(R^{34e})_{p34b} indicates that the 6-membered nitrogen-containing heteroaryl ring to which it is attached is optionally substituted with p34b R^{34e}, with each R^{34c} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p34b represents an integer of 0, 1 or 2;
(R^{35a})ₚ₃₅ₐ in Formula (PBM-35) indicates that the phthalazinone ring to which it is attached is optionally substituted with p35a R^{35a}, with each R^{35a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p35a represents an integer of 0, 1, 2, 3, or 4;
R^{35b} represents optionally substituted C₁₋₃ alkylene;
(R^{35c})_{p35b} indicates that the benzene ring to which it is attached is optionally substituted with p35b R^{35c}, with each R^{35c} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p35b represents an integer of 0, 1, 2, 3, or 4;
(R^{36a})ₚ₃₆ₐ in Formula (PBM-36) indicates that the 2H-indazole ring to which it is attached is optionally substituted with p36a R^{36a}, with each R^{36a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p36a represents an integer of 0, 1, 2 or 3;
(R^{36b})_{p36b} indicates that the benzene ring to which it is attached is optionally substituted with p36b R^{36b}, with each R^{36b} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p36b represents an integer of 0, 1, 2, 3, or 4;
(R^{37a})ₚ₃₇ₐ in Formula (PBM-37) indicates that the benzofuran ring to which it is attached is optionally substituted with p37a R^{37a}, with each R^{37a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p37a represents an integer of 0, 1, 2 or 3;
(R^{37b})_{p37b} indicates that the benzene ring to which it is attached is optionally substituted with p37b R^{37b}, with each R^{37b} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p37b represents an integer of 0, 1, 2 or 3;
(R^{38a})ₚ₃₈ₐ in Formula (PBM-38) indicates that the benzene ring to which it is attached is optionally substituted with p38a R^{38a}, with each R^{38a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p38a represents an integer of 0, 1, 2, 3, or 4;
R^{38b}, R^{38c}, and R^{38d} are the same or different and each independently represent hydrogen, deuterium, hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
R^{39a} and R^{39b} in Formula (PBM-39) are the same or different and each independently represent hydrogen, halogen, C₁₋₃ alkyl or halogenated C₁₋₃ alkyl;
R^{39c} represents a bond, hydrogen, C₁₋₆ alkyl, deuterated C₁₋₆ alkyl, or halogenated C₁₋₆ alkyl;
y1 represents an integer of 1, 2 or 3;
R^{39d} represents the structure of the following formula: 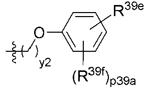
wherein R^{39e} represents a bond or hydrogen; (R^{39f})ₚ₃₉ₐ indicates that the benzene ring to which it is attached is optionally substituted with p39a R^{39f}, with each R^{39f} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl, and p39a represents an integer of 0, 1, 2, 3, or 4; and y2 represents an integer of 1, 2 or 3; or
R^{39d} represents the structure of the following formula: 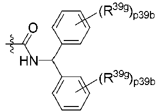
wherein each (R^{39g})_{p39b} independently indicates that the benzene ring to which it is attached is optionally substituted with p39b R^{39g}, with each R^{39g} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl, and p39b represents an integer of 0, 1, 2, 3, 4 or 5;
wherein R^{39c} and R^{39e} are not simultaneously a bond, and only one of R^{39c} and R^{39e} represents a bond, wherein when R^{39c} represents a bond, the ring nitrogen atom connected to R^{39c} is directly connected to R_{c}, and when R^{39e} represents a bond, the ring carbon atom connected to R^{39e} is directly connected to R_{c} ;
(R^{40a})ₚ₄₀ₐ in Formula (PBM-40) indicates that the benzene ring to which it is attached is optionally substituted with p40a R^{40a}, with each R^{40a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p40a represents an integer of 0, 1, 2, 3, or 4;
Rₓ₃ in Formula (PBM-40) represents a bond or C(O);
R^{41a} in Formula (PBM-41) represents hydrogen, C₁₋₆ alkyl, deuterated C₁₋₆ alkyl, halogenated C₁₋₆ alkyl, optionally substituted C₃₋₁₅ cycloalkyl, or optionally substituted 4- to 15-membered nitrogen-containing heterocyclyl;
R^{41b} represents hydrogen, C₁₋₆ alkyl, deuterated C₁₋₆ alkyl, halogenated C₁₋₆ alkyl, optionally substituted C₃₋₁₅ cycloalkyl, optionally substituted 4- to 15-membered nitrogen-containing heterocyclyl or NR^{41e}R^{41f}, where R^{41e} and R^{41f} are the same or different and each independently represent hydrogen, C₁₋₆ alkyl, deuterated C₁₋₆ alkyl, halogenated C₁₋₆ alkyl or optionally substituted 4- to 15-membered heterocyclyl;
R^{41c} represents N or CH;
(R^{41d})ₚ₄₁ₐ indicates that the benzene ring to which it is attached is optionally substituted with p41a R^{41d}, with each R^{41d} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p41a represents an integer of 0, 1, 2, 3, or 4;
(R^{42a})ₚ₄₂ₐ in Formula (PBM-42) indicates that the benzene ring to which it is attached is optionally substituted with p42a R^{42a}, with each R^{42a} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p42a represents an integer of 0, 1, 2, 3, 4 or 5;
R^{42b} represents O, S, C₁₋₃ alkylene or halogenated C₁₋₃ alkylene;
(R^{42c})_{p42b} indicates that the benzene ring to which it is attached is optionally substituted with p42b R^{42c}, with each R^{42c} being the same or different and each independently representing hydroxy, amino, cyano, halogen, C₁₋₆ alkyl, C₁₋₆ alkoxy, halogenated C₁₋₆ alkoxy, deuterated C₁₋₆ alkyl, or halogenated C₁₋₆ alkyl;
p42b represents an integer of 0, 1, 2, 3, or 4;
ring H in Formula (PBM-43) represents C₆₋₁₅ arylene or 5- to 15-membered monocyclic, bicyclic, or polycyclic heteroarylene, and (R^{43a})ₚ₄₃ₐ indicates that the ring H is optionally substituted with p43a R^{43a}, with each R^{43a} being the same or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p43a represents an integer of 0, 1, 2, 3, or 4;
R^{43b} represents an optionally substituted 5- to 20-membered monocyclic, bicyclic, or polycyclic heteroaryl group containing 1 to 4 heteroatoms selected from N, O, and S, which is optionally substituted with a substituent selected from the group consisting of hydroxy, amino, cyano, halogen, nitro, C₁₋₆ alkyl, C₁₋₆ alkoxy, halogenated C₁₋₆ alkoxy, deuterated C₁₋₆ alkyl, or halogenated C₁₋₆ alkyl;
R^{44a}, R^{44b}, and R^{44c} in Formula (PBM-44) each independently represent CH or N;
R^{44d} represents -C(O)NHR^{44h}, -NHC(O)R^{44h}, -S(O)₂NHR^{44h}, -NHS(O)₂R^{44h}, -S(O)₂R^{44h} or -P(O)(R^{44h})₂, wherein each R^{44h} is the same or different and each independently represents C₁₋₆ alkyl, deuterated C₁₋₆ alkyl, or halogenated C₁₋₆ alkyl;
(R^{44e})ₚ₄₄ₐ represents p44a R^{44e} groups, wherein each R^{44e} is the same or different and each independently represents halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p44a represents an integer of 0, 1, 2, 3, or 4;
(R^{44f})_{p44b} represents p44b R^{44f} groups, wherein each R^{44f} is the same or different and each independently represents halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p44b represents an integer of 0, 1 or 2;
(R^{44g})_{p44c} indicates that the benzene ring to which it is attached is optionally substituted with p44c R^{44g}, with each R^{44g} independently representing halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p44c represents an integer of 0, 1, 2, 3, or 4;
Rₓ₁ in Formula (PBM-45) represents a bond or C(O), or -C(O)NH-Rₓ₂-C(O)-***, wherein symbol *** indicates the point of attachment to R_{c}, and Rₓ₂ represents optionally substituted C₃₋₁₅ cycloalkyl;
(R^{45a})ₚ₄₅ₐ indicates that the 1H-pyrrolo[2,3-b]pyridine ring to which it is attached is optionally substituted with p45a R^{45a}, wherein each R^{45a} is independently cyano, halogen, hydroxy, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p45a represents an integer of 0, 1, 2, 3, 4 or 5;
(R^{45b})_{p45b} indicates that the pyridine ring to which it is attached is optionally substituted with p45b R^{45b}, wherein each R^{45b} is independently cyano, halogen, hydroxy, nitro, C₁₋₆ alkyl, halogenated C₁₋₆ alkyl, deuterated C₁₋₆ alkyl, C₁₋₆ alkoxy, halogenated C₁₋₆ alkoxy or NR^{45c}R^{45d}, where R^{45c} and R^{45d} are the same or different and each independently represent hydrogen, C₁₋₆ alkyl, deuterated C₁₋₆ alkyl, halogenated C₁₋₆ alkyl, optionally substituted C₃₋₁₅ cycloalkyl or optionally substituted 4- to 15-membered heterocyclyl, and p45b represents an integer of 0, 1, 2 or 3;
R^{46a} in Formula (PBM-46) represents NHC(O), C(O)NH, NHS(O)₂ or S(O)₂NH;
(R^{46b})ₚ₄₆ₐ indicates that the benzene ring to which it is attached is optionally substituted with p46a R^{46b}, wherein each R^{46b} is independently halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p46a represents an integer of 0, 1, 2, 3, 4 or 5;
(R^{46c})_{p46b} indicates that the 1H-pyrazole ring to which it is attached is optionally substituted with p46b R^{46c}, wherein each R^{46c} is independently halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p46b represents an integer of 0, 1 or 2;
R^{47a} in Formula (PBM-47) represents N or CH, and R^{47b} represents O, S, NH, CH₂, CH(F) or C(F)₂;
R^{47c} represents a bond or optionally substituted methylene (e.g., halogenated methylene);
ring I represents C₆₋₁₅ aryl, 4- to 15-membered heterocyclyl, or 5- to 15-membered heteroaryl, and (R^{47d})ₚ₄₇ₐ indicates that the ring I to which it is attached is optionally substituted with p47a R^{47d}, with each R^{47d} being the same or different and each independently representing deuterium, halogen, hydroxy, cyano, amino, nitro, oxo, C₁₋₆ alkyl, halogenated C₁₋₆ alkyl, deuterated C₁₋₆ alkyl, C₁₋₆ alkoxy, or halogenated C₁₋₆ alkoxy, and p47a represents an integer of 0, 1, 2, 3, or 4;
(R^{47c})_{p47b} indicates that the 6-membered nitrogen-containing heteroaromatic ring containing R^{47a} to which it is attached is optionally substituted with p47b R^{47c}, with each R^{47c} independently representing deuterium, halogen, hydroxy, cyano, amino, nitro, C₁₋₆ alkoxy, halogenated C₁₋₆ alkoxy, C₁₋₆ alkyl, deuterated C₁₋₆ alkyl, or halogenated C₁₋₆ alkyl, and p47b represents an integer of 0, 1 or 2;
(R^{47t})_{p47c} indicates that the benzene ring to which it is attached is optionally substituted with p47c R^{47f}, with each R^{47f} being the same or different and each independently representing deuterium, halogen, hydroxy, cyano, amino, nitro, C₁₋₆ alkoxy, halogenated C₁₋₆ alkoxy, C₁₋₆ alkyl, deuterated C₁₋₆ alkyl, or halogenated C₁₋₆ alkyl, and p47c represents an integer of 0, 1, 2, 3, or 4;
R^{48a}, R^{48b} and R^{48c} in Formula (PBM-48) independently represent N or CH;
(R^{48d})ₚ₄₈ₐ indicates that the naphthyl to which it is attached is optionally substituted with p48a R^{48d}, wherein each R^{48d} is independently halogen, hydroxy, amino, mercapto, nitro, cyano, C₂₋₆ alkynyl, C₂₋₆ alkenyl, C₁₋₆ alkoxy, halogenated C₁₋₆ alkoxy, C₁₋₆ alkyl, deuterated C₁₋₆ alkyl, or halogenated C₁₋₆ alkyl, and p48a represents an integer of 0, 1, 2, 3, 4, 5, 6 or 7;
R^{48e} represents optionally substituted C₃₋₁₅ cycloalkyl or optionally substituted 4- to 15-membered heterocyclyl containing from 1 to 4 heteroatoms independently selected from sulfur, oxygen, and nitrogen;
R^{43f} represents hydrogen, halogen, hydroxy, cyano, amino, mercapto, nitro, C₂₋₆ alkynyl, C₂₋₆ alkenyl, C₁₋₆ alkoxy, halogenated C₁₋₆ alkoxy, C₁₋₆ alkyl, deuterated C₁₋₆ alkyl, or halogenated C₁₋₆ alkyl;
R^{49a} in Formula (PBM-49) represents N or CH;
R^{49b} represents NHC(O), C(O)NH, NHS(O)₂ or S(O)₂NH;
(R^{49c})ₚ₄₉ₐ indicates that the 6-membered ring containing R^{49a} is substituted with p49a R^{49c}, with each R^{49c} being the same or different and each independently representing halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p49a represents an integer of 0, 1, 2, 3, or 4;
ring J represents 5- to 15-membered heteroarylene, and (R^{49d})_{p49b} indicates that the ring J to which it is attached is optionally substituted with p49b R^{49d}, with each R^{49d} being the same or different and each independently representing halogen, hydroxy, mercapto, cyano, amino, nitro, C₁₋₆ alkyl, halogenated C₁₋₆ alkyl, deuterated C₁₋₆ alkyl, hydroxy-substituted C₁₋₆ alkyl, amino-substituted C₁₋₆ alkyl, C₁₋₆ alkoxy, halogenated C₁₋₆ alkoxy or optionally substituted C₃₋₁₅ cycloalkyl, and p49b represents an integer of 0, 1, 2 or 3;
(R^{50a})ₚ₅₀ₐ in Formula (PBM-50) indicates that the benzene ring to which it is attached is substituted with p50a R^{50a}, with each R^{50a} being the same or different and each independently representing halogen, hydroxy, mercapto, cyano, amino, nitro, C₁₋₆ alkyl, halogenated C₁₋₆ alkyl, deuterated C₁₋₆ alkyl, C₁₋₆ alkyl-S(O)₂, halogenated C₁₋₆ alkyl-S(O)₂, halogenated C₁₋₆ alkyl-NHC(O), halogenated C₁₋₆ alkyl-C(O)NH, halogenated C₁₋₆ alkyl-NHS(O)₂, halogenated C₁₋₆ alkyl-S(O)₂NH, C₁₋₆ alkoxy, or halogenated C₁₋₆ alkoxy, and p50a represents an integer of 0, 1, 2, 3, or 4;
R^{50b} represents hydrogen, C₁₋₆ alkyl, halogenated C₁₋₆ alkyl, or deuterated C₁₋₆ alkyl;
(R^{51a})ₚ₅₁ₐ in Formula (PBM-51) indicates that the benzene ring to which it is attached is substituted with p51a R^{51a}, with each R^{51a} being the same or different and each independently representing halogen, hydroxy, mercapto, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy, optionally substituted C₃₋₁₅ cycloalkyl or optionally substituted 4- to 15-membered heterocyclyl containing from 1 to 4 heteroatoms selected from the group consisting of oxygen, nitrogen, and sulfur, and p51a represents an integer of 0, 1, 2, 3, 4 or 5;
(R^{51b})_{p51b} indicates that the benzene ring to which it is attached is substituted with p51b R^{51b}, with each R^{51b} being the same or different and each independently representing halogen, hydroxy, mercapto, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy, optionally substituted C₃₋₁₅ cycloalkyl or optionally substituted 4- to 15-membered heterocyclyl containing from 1 to 4 heteroatoms selected from the group consisting of oxygen, nitrogen, and sulfur, and p51b represents an integer of 0, 1, 2, 3, or 4;
(R^{51c})_{p51c} indicates that the isoxazole ring to which it is attached is optionally substituted with p51c R^{51c}, with each R^{51c} being the same or different and each independently representing halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p51c represents an integer of 0, 1 or 2;
the dashed line --- in Formula (PBM-52) indicates the optional presence of a double bond;
(R^{52a})ₚ₅₂ₐ indicates that the 6-membered cycloalkyl ring to which it is attached is optionally substituted with p52a R^{52a}, with each R^{52a} being the same or different and each independently representing halogen, hydroxy, mercapto, cyano, amino, nitro, oxo, C₁₋₆ alkyl, halogenated C₁₋₆ alkyl, deuterated C₁₋₆ alkyl, C₁₋₆ alkoxy, or halogenated C₁₋₆ alkoxy, and p52a represents an integer of 0, 1, 2, 3, 4, 5, 6, 7 or 8;
(R^{52b})_{p52b} indicates that the benzene ring to which it is attached is optionally substituted with p52b R^{52b}, with each R^{52b} being the same or different and each independently representing deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p52b represents an integer of 0, 1, 2, 3, 4 or 5;
(R^{52c})_{p52c} indicates that the 6-membered cycloalkyl ring to which it is attached is optionally substituted with p52c R^{52c}, with each R^{52c} being the same or different and each independently representing halogen, cyano, oxo, C₁₋₆ alkyl, halogenated C₁₋₆ alkyl, deuterated C₁₋₆ alkyl, C₁₋₆ alkoxy, or halogenated C₁₋₆ alkoxy, and p52c represents an integer of 0, 1, 2, 3, 4, 5, 6, 7 or 8;
(R^{52d})_{p52d} indicates that the benzene ring to which it is attached is optionally substituted with p52d R^{52d}, with each R^{52d} being the same or different and each independently representing halogen, hydroxy, mercapto, C₁₋₆ alkyl, halogenated C₁₋₆ alkyl, deuterated C₁₋₆ alkyl, C₁₋₆ alkoxy, halogenated C₁₋₆ alkoxy, or 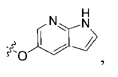 and p52d represents an integer of 0, 1 or 2;
(R^{52e})ₚ₅₂ₑ indicates that the benzene ring to which it is attached is optionally substituted with p52e R^{52c}, with each R^{52e} being the same or different and each independently representing deuterium, halogen, hydroxy, mercapto, cyano, amino, nitro, -SO₂CF₃, C₁₋₆ alkyl, halogenated C₁₋₆ alkyl, deuterated C₁₋₆ alkyl, C₁₋₆ alkoxy, or halogenated C₁₋₆ alkoxy, and p52e represents an integer of 0, 1, 2, 3, or 4;
R^{52f} represents hydrogen, deuterium, halogen, hydroxy, mercapto, cyano, amino, nitro,C₁₋₆ alkyl, halogenated C₁₋₆ alkyl, deuterated C₁₋₆ alkyl, C₁₋₆ alkoxy, halogenated C₁₋₆ alkoxy, or 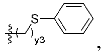 where y3 represents an integer of 1, 2 or 3;
(R^{53a})ₚ₅₃ₐ in Formula (PBM-53) represents p53a R^{53a} groups, with each R^{53a} being the same or different and each independently representing halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p53a represents an integer of 0, 1, 2, 3, 4 or 5;
(R⁵³⁶)_{p53b} indicates that the benzene ring to which it is attached is optionally substituted with p53b R^{53b}, with each R^{53b} being the same or different and each independently representing deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p53b represents an integer of 0, 1, 2, 3, or 4; and
R^{53c} represents C₁₋₆ alkylene or halogenated C₁₋₆ alkylene;
(R^{54a})ₚ₅₄ₐ in Formula (PBM-54) represents p54a R^{54a} groups, wherein each R^{54a} is the same or different and each independently represents halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p54a represents an integer of 0, 1, 2, 3, or 4;
(R^{54b})_{p54b} indicates that the benzene ring to which it is attached is optionally substituted with p54b R^{54b}, with each R^{54b} being the same or different and each independently representing deuterium, halogen, hydroxy, mercapto, cyano, amino, nitro,C₁₋₆ alkyl, halogenated C₁₋₆ alkyl, deuterated C₁₋₆ alkyl, C₁₋₆ alkoxy, or halogenated C₁₋₆ alkoxy, and p54b represents an integer of 0, 1, 2, 3, or 4;
(R^{55a})ₚ₅₅ₐ in Formula (PBM-55) represents p55a R^{55a} groups, wherein each R^{55a} is the same or different and each independently represents halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p55a represents an integer of 0, 1, 2, 3, or 4;
R^{55b} and R^{55c} are the same or different and each independently represent hydrogen, C₁₋₆ alkyl, halogenated C₁₋₆ alkyl, deuterated C₁₋₆ alkyl, or optionally substituted aryl;
R^{55d} in Formula (PBM-55) represents a fragment 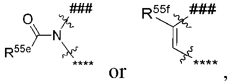
wherein symbol ### indicates the point of attachment to the ring carbon atom, symbol **** indicates the point of attachment to the ring nitrogen atom, R^{55e} represents optionally substituted aryl or optionally substituted heteroaryl, and R^{55f} represents hydrogen, halogen, C₁₋₆ alkyl, halogenated C₁₋₆ alkyl or deuterated C₁₋₆ alkyl; and
R_{c} in each general formula independently represents a bond, -O-, -N(R_{d})-, -NHC(O)-*, -C(O)NH-*, - N(R_{d})-R_{f}-N(Rₑ)-*, -R_{f}-C(O)NH-*, -R_{f}-NHC(O)-*, -R_{f}-C(O)O-*, -R_{f}-OC(O)-*, -C(O)O-*, -OC(O)-*, -R_{f}-, -
R_{f}-N(R_{d})-*, -O-R_{f}-N(R_{d})-*, 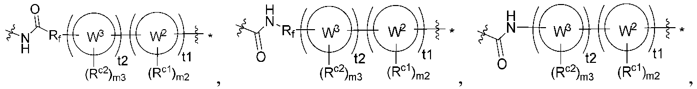 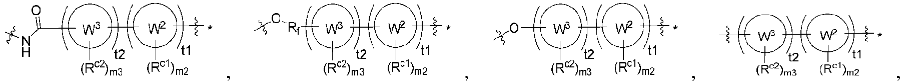 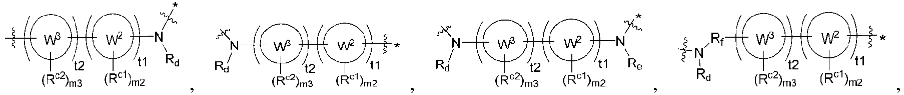 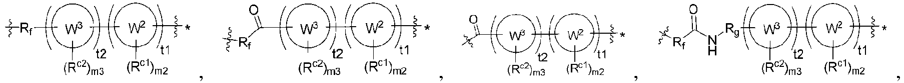 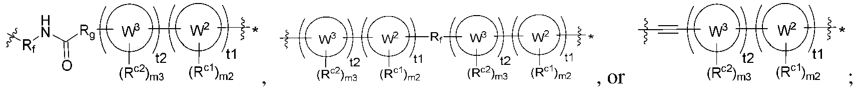
wherein each ring W² is the same or different and each independently represents nitrogen-containing heterocyclylene, t1 represents an integer of 1 or 2, and (R^{c1})ₘ₂ indicates that each ring W² is independently optionally substituted with m2 R^{c1} groups, with each R^{c1} being independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c4}-R^{c3}-, where R^{c3} is optionally substituted C₁₋₆ alkylene or optionally substituted C₂₋₆ alkenylene, and R^{c4} represents halogen, R^{c5}R^{c6}N-, hydroxy, carboxyl, ethynyl, or vinyl, wherein R^{c5} and R^{c6} are the same or different and each independently represent hydrogen or C₁₋₃ alkyl, and m2 represents an integer of 0-20;
each ring W³ is the same or different and each independently represents heterocyclylene, cycloalkylene, arylene, or heteroarylene, t2 represents an integer of 0 or 1, and (R^{c2})ₘ₃ indicates that each ring W³ is independently optionally substituted with m3 R^{c2} groups, with each R^{c2} being independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c8}-R^{c7}-, where R^{c7} is optionally substituted C₁₋₆ alkylene or optionally substituted C₂₋₆ alkenylene, and R^{c8} represents halogen, R^{c9}R^{c10})N-, hydroxy, carboxyl, ethynyl, or vinyl, wherein R^{c9} and R^{c10} are the same or different and each independently represent hydrogen or C₁₋₃ alkyl, and m3 represents an integer of 0-20; and
R_{d} and R_{c} each independently represent hydrogen or C₁₋₆ alkyl, R_{f} and R_{g} each independently represent optionally substituted C₁₋₆ alkylene or optionally substituted C₂₋₆ alkenylene, and symbol * indicates the point of attachment to LIN.

4. The compound of Formula (I) or salts, enantiomers, stereoisomers, solvates, isotopically enriched analogs, prodrugs, or polymorphs thereof as claimed in claim 3, wherein the PBM represents a structure of the following formulae: 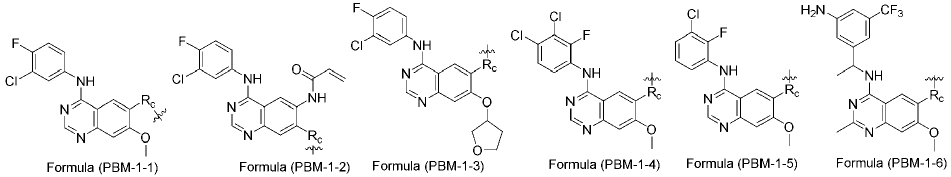 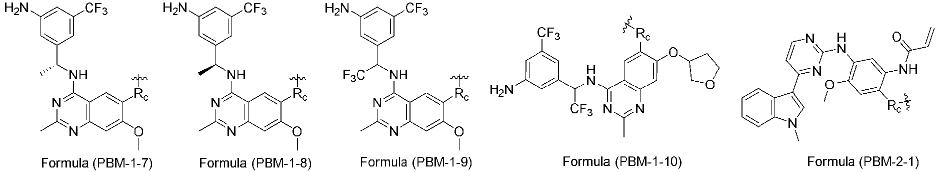 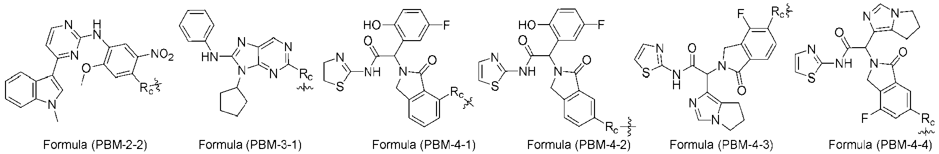 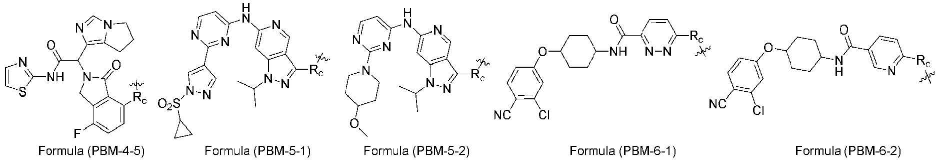 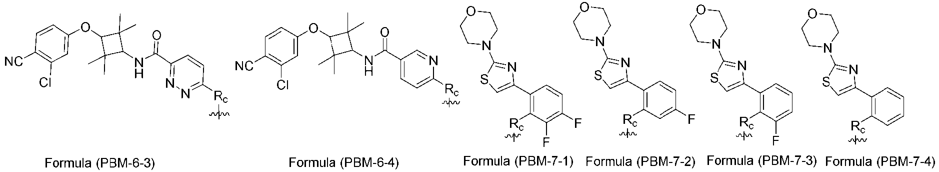 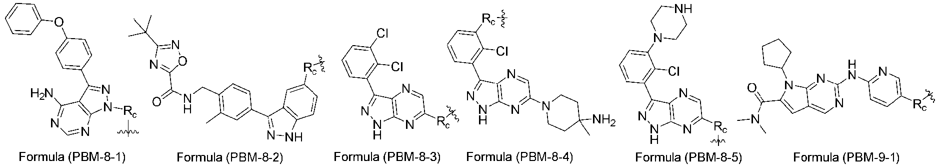 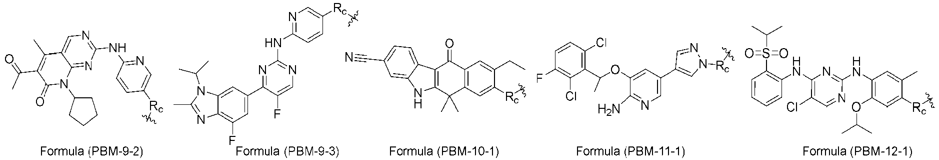 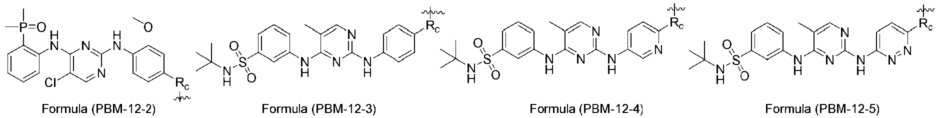 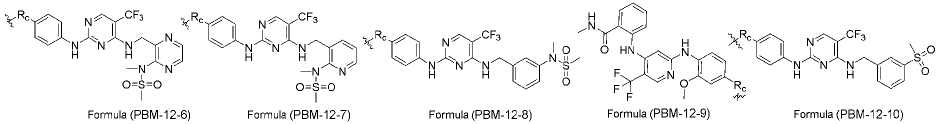 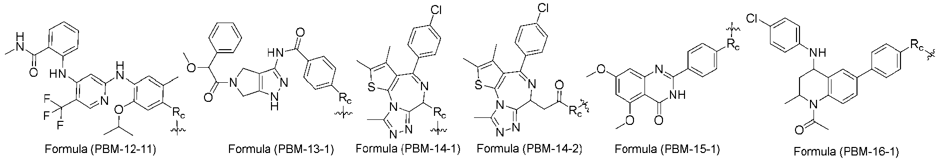 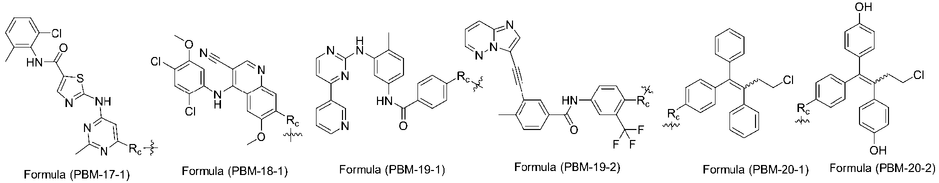 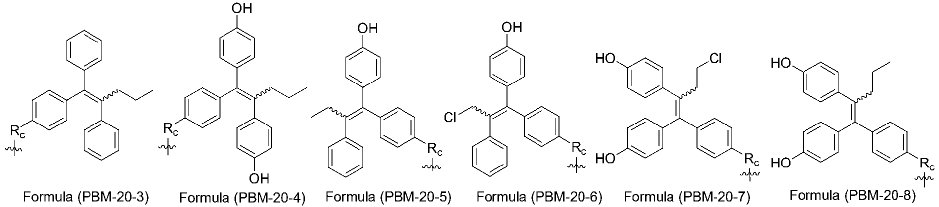 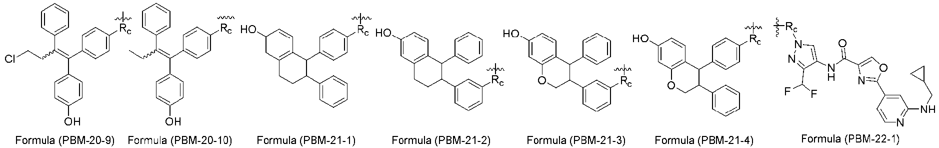 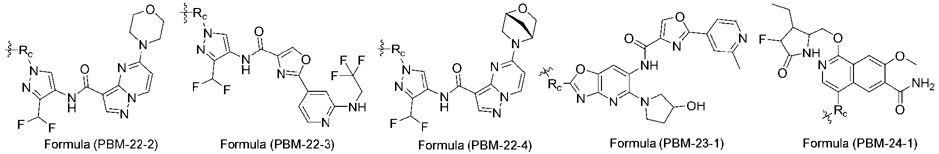 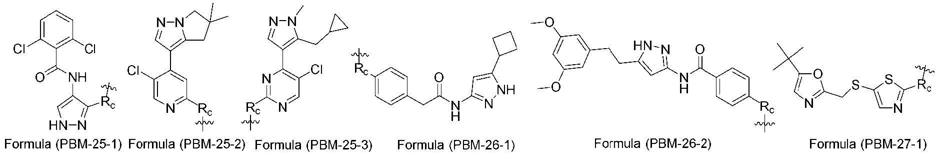 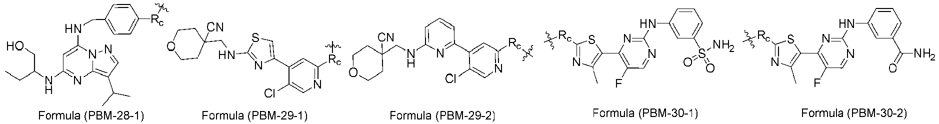 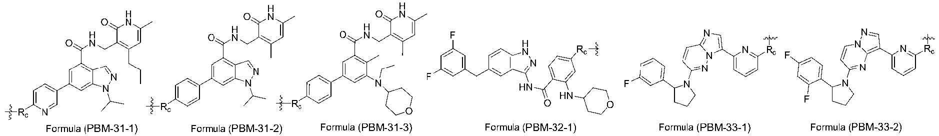 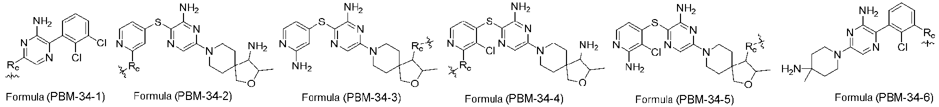 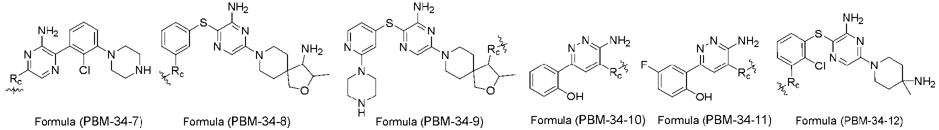 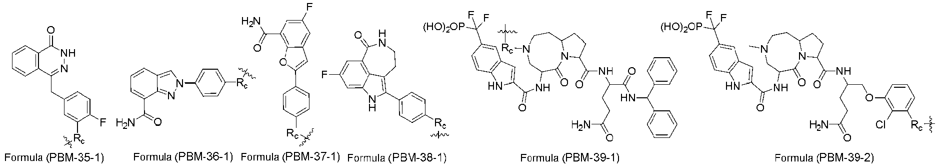 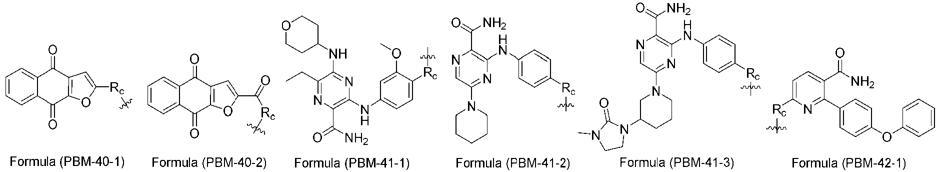 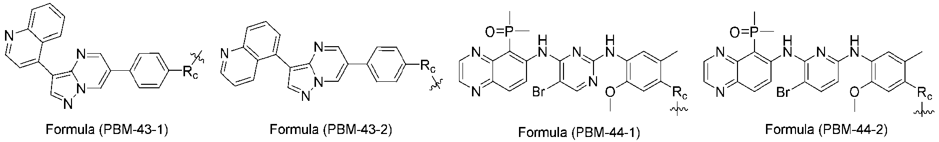 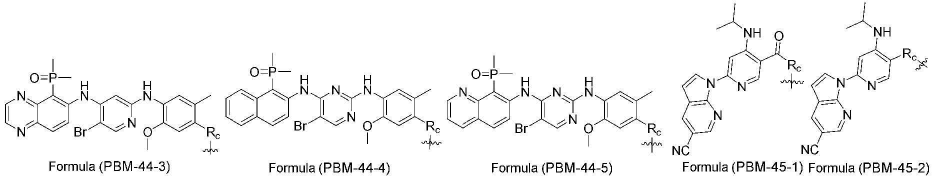 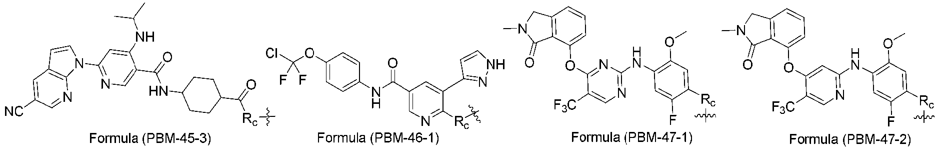 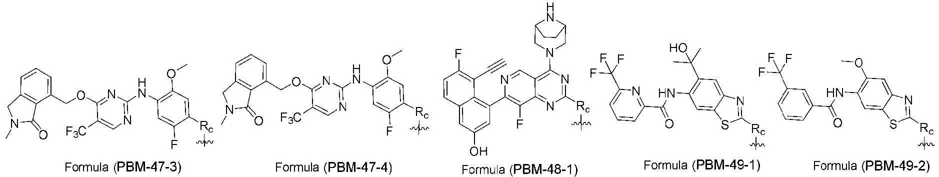 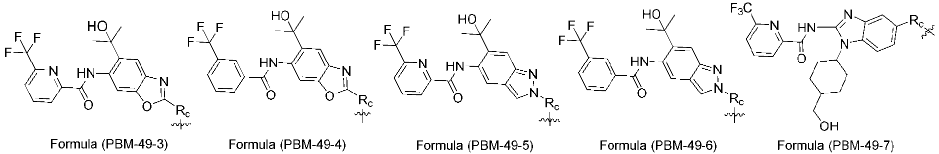 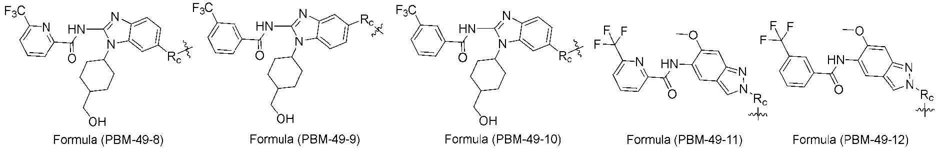 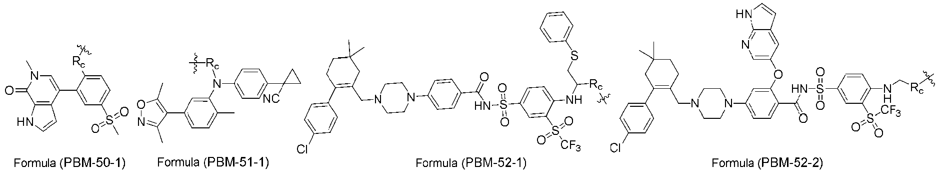 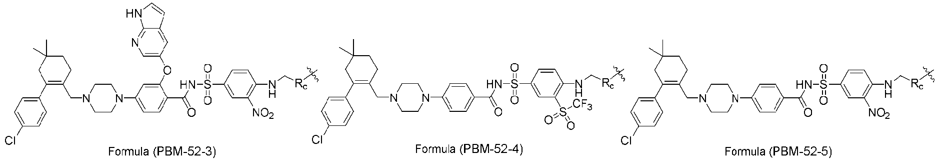 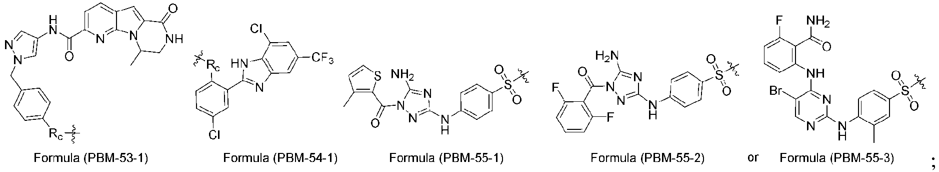 wherein R_{c} is as defined in claim 3.

5. The compound of Formula (I) or salts, enantiomers, stereoisomers, solvates, isotopically enriched analogs, prodrugs, or polymorphs thereof as claimed in claim 3, wherein
the R_{c} represents a bond, -O-, -NHC(O)-*, -C(O)NH-*, -C(O)O-*, -OC(O)-*, or -NH-, or
R_{c} represents a structure of the following formulae:
-N(R_{d})-, -N(R_{d})-R_{f}-N(R_{c})-*, -R_{f}-C(O)NH-*, -R_{f}-NHC(O)-*, -R_{f}-C(O)O-*, -R_{f}-OC(O)-*, -R_{f}-, -R_{f}-N(R_{d})-*, -O-R_{f}-N(R_{d})-*, 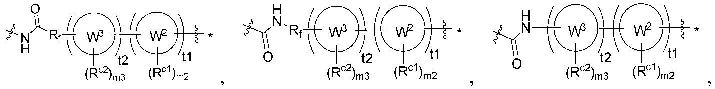 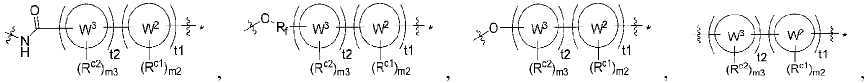 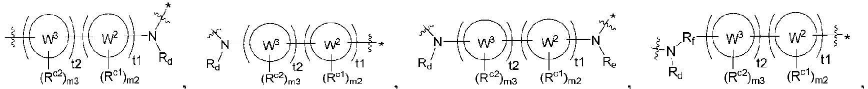 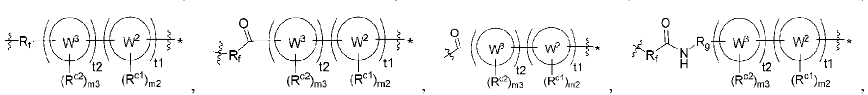 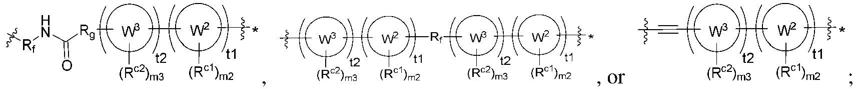
wherein each ring W² is the same or different and each independently represents 4- to 20-membered nitrogen-containing heterocyclylene, t1 represents an integer of 1 or 2, and (R^{c1})ₘ₂ indicates that each ring W² is independently optionally substituted with m2 R^{c1} groups, with each R^{c1} being independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c4}-R^{c3}-, wherein R^{c3} is optionally substituted C₁₋₆alkylene or optionally substituted C₂₋₆ alkenylene, and R^{c4} represents halogen, R^{c5}R^{c6}N-, hydroxy, carboxyl, ethynyl, or vinyl, where R^{c5} and R^{c6} are the same or different and each independently represent hydrogen or C₁₋₃ alkyl, and m2 represents an integer of 0-20 ;
each ring W³ is the same or different and each independently represents 4- to 20-membered nitrogen-containing heterocyclylene, C₃₋₂₀ cycloalkylene, C₆₋₂₀ arylene, or 5- to 20-membered heteroarylene, t2 represents an integer of 0 or 1, and (R^{c2})ₘ₃ indicates that each ring W³ is independently optionally substituted with m3 R^{c2} groups, with each R^{c2} being independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c8}-R^{c7}-, wherein R^{c7} is optionally substituted C₁₋₆alkylene or optionally substituted C₂₋₆ alkenylene, and R^{c8} represents halogen, R^{c9}R^{c10}N-, hydroxy, carboxyl, ethynyl, or vinyl, where R^{c9} and R^{c10} are the same or different and each independently represent hydrogen or C₁₋₃ alkyl, and m3 represents an integer of 0-20; and
R_{d} and Rₑ each independently represent hydrogen or C₁₋₆ alkyl, and R_{f} and R_{g} each independently represent optionally substituted C₁₋₆ alkylene or optionally substituted C₂₋₆ alkenylene;
the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy; and
symbol * indicates the point of attachment to LIN.

6. The compound of Formula (I) or salts, enantiomers, stereoisomers, solvates, isotopically enriched analogs, prodrugs, or polymorphs thereof as claimed in any one of claims 3-5, wherein
each ring W² is the same or different and each independently represents 4- to 15-membered nitrogen-containing heterocyclylene, e.g.,
piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene, or 3-methyl-2-oxa-8-azaspiro[4.5]decanylene, or 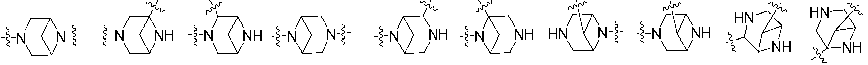 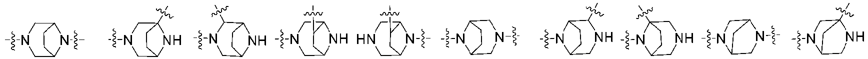 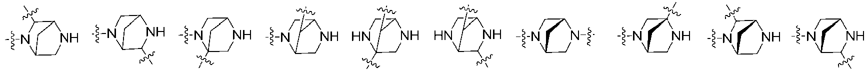 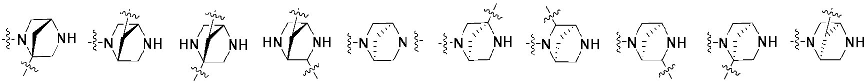 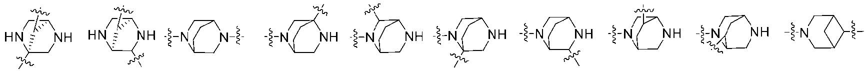 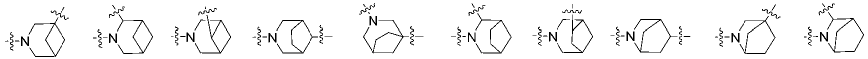 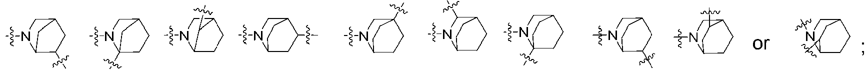
each ring W² is independently optionally substituted with m2 R^{c1} groups, with each R^{c1} being independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c4}-R^{c3}-, wherein R^{c3} is optionally substituted C₁₋₆ alkylene or optionally substituted C₂₋₆ alkenylene, and R^{c4} represents halogen, R^{c5}R^{c6}N-, hydroxy, carboxyl, ethynyl, or vinyl, wherein R^{c5} and R^{c6} are the same or different and each independently represent hydrogen or C₁₋₃ alkyl, and the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and m2 represents an integer of 0-20;
each ring W³ is the same or different and each independently represents 4- to 15-membered nitrogen-containing heterocyclylene, C₃₋₁₅ cycloalkylene, 6- to 15-membered arylene, or 5- to 15-membered heteroarylene, e.g.,
piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene, 3-methyl-2-oxa-8-azaspiro[4.5]decanylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spirocycloalkylene, adamantanylene, noradamantanylene, norbomylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, or 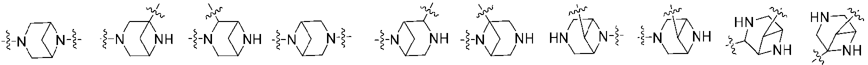 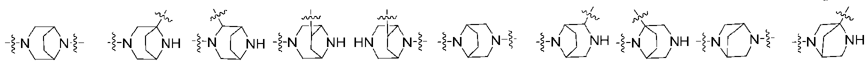 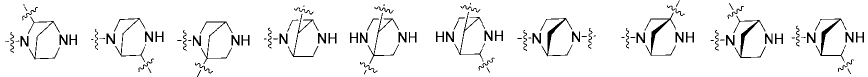 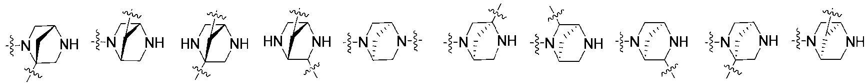 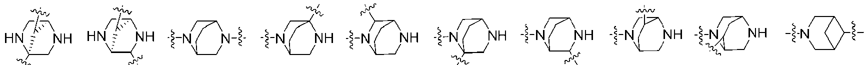 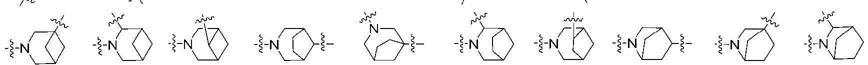 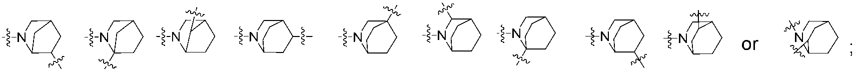
each ring W³ is independently optionally substituted with m3 R^{c2} groups, with each R^{c2} being independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c3}-R^{c7}-, wherein R^{c7} is optionally substituted C₁₋₆ alkylene or optionally substituted C₂₋₆ alkenylene, and R^{c8} represents halogen, R^{c9}R^{c10}N-, hydroxy, carboxyl, ethynyl, or vinyl, where R^{c9} and R^{c10} are the same or different and each independently represent hydrogen or C₁₋₃ alkyl, and the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and m3 represents an integer of 0-20.

7. The compound of Formula (I) or salts, enantiomers, stereoisomers, solvates, isotopically enriched analogs, prodrugs, or polymorphs thereof as claimed in any one of claims 3-6, wherein the 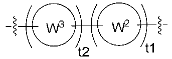 moiety of R_{c} in each general formula represents the following groups: 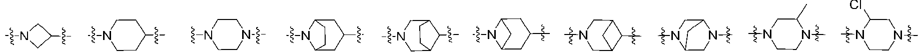 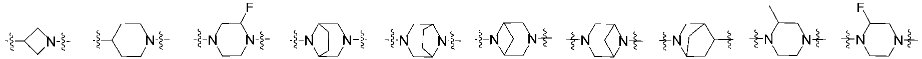 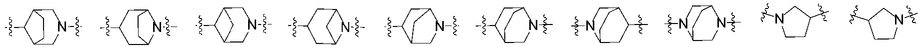 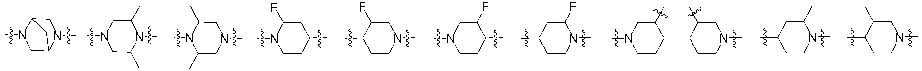 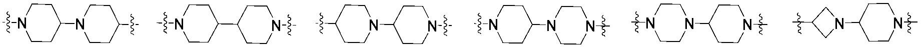 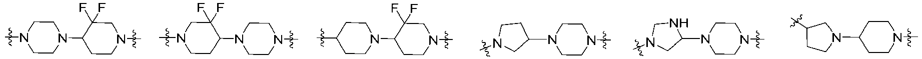 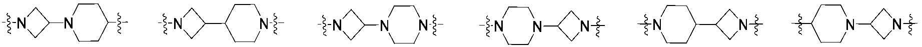 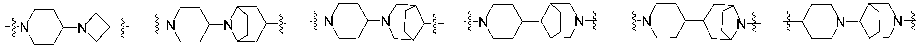 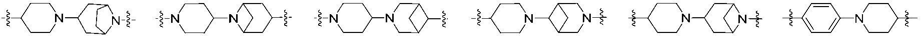 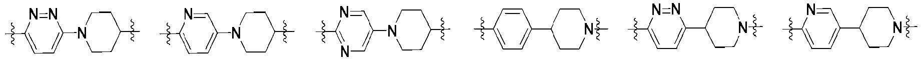 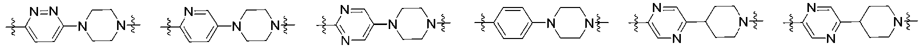 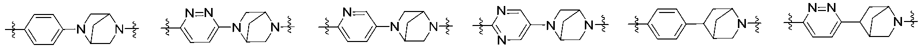 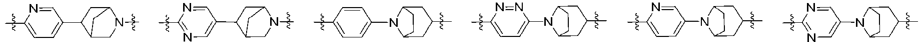 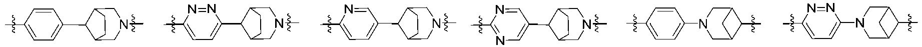 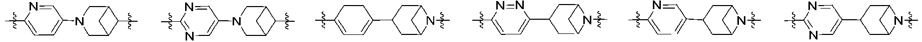 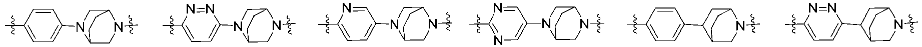 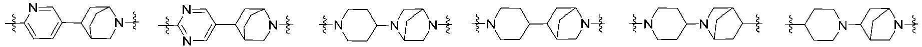 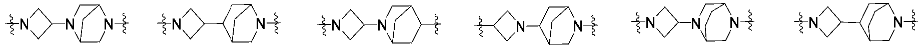 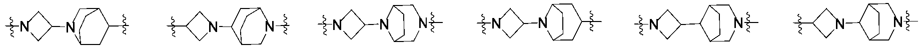 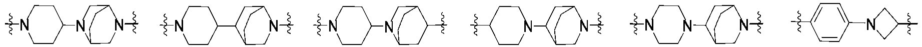 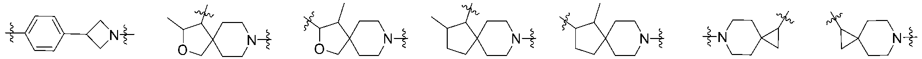 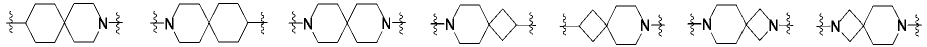 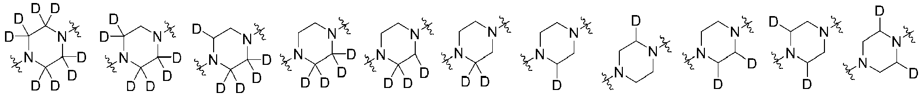 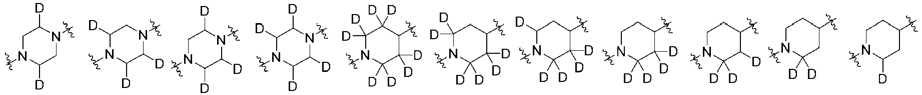 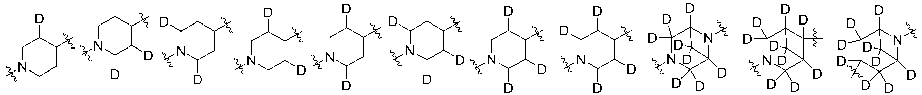 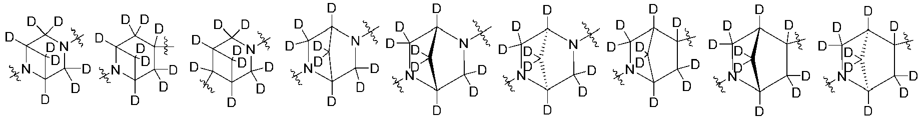 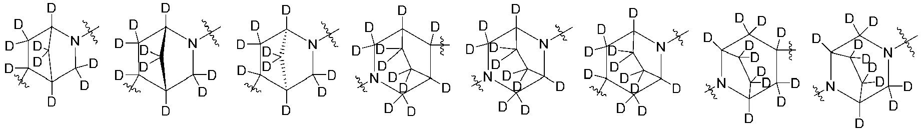 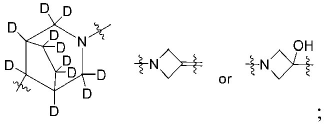 wherein the groups are independently optionally substituted with one or more substituents selected from the group consisting of deuterium, optionally deuterated C₁₋₆ alkyl, optionally halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, R^{c12}-R^{c11}, or any combination thereof, wherein R^{c11} is optionally substituted C₁₋₆ alkylene or optionally substituted C₂₋₆ alkenylene, and R^{c12} represents halogen, R^{c13}R^{c14}N-, hydroxy, carboxyl, ethynyl, or vinyl, where R^{c13} and R^{c14} are the same or different and each independently represent hydrogen or C₁₋₃ alkyl, and the C₁₋₆ alkylene and C₂₋₆ alkenylene are independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy.

8. The compound of Formula (I) or salts, enantiomers, stereoisomers, solvates, isotopically enriched analogs, prodrugs, or polymorphs thereof as claimed in any one of claims 3-6, wherein the R_{c} represents the following groups:
a bond, -O-, -NHC(O)-*, -C(O)NH-*, -C(O)O-*, -OC(O)-*, -CH₂C(O)O-*, -CH₂OC(O)-*, -NH-, - N(CH₃)-, -N(CH₃)-(CH₂)₂-N(CH₃)-*, -N(CH₃)-(CH₂)₃-N(CH₃)-*, -NH-(CH₂)₂-N(CH₃)-*, -N(CH₃)-(CH₂)₃-NH-*, -CH₂-NHC(O)-*, -CH₂-C(O)NH-*, -CH₂-NH-*, -O-(CH₂)₂-N(CH₃)-*, -O-(CH₂)₂-NH-*, or -CH₂-N(CH₃)-*, or 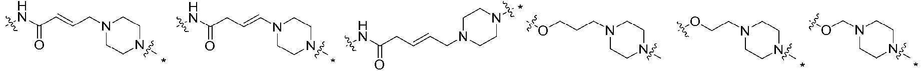 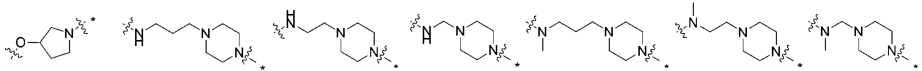 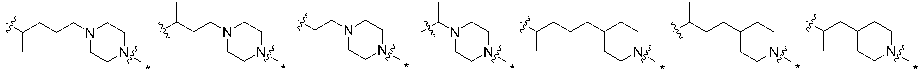 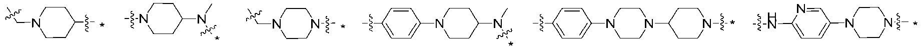 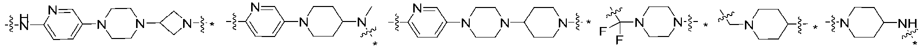 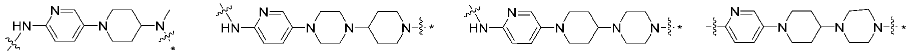 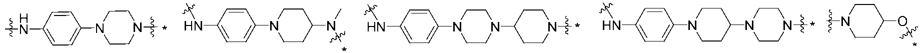 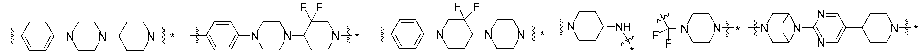 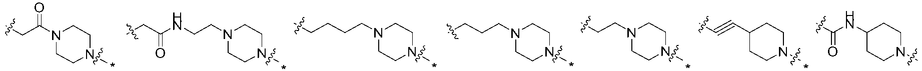 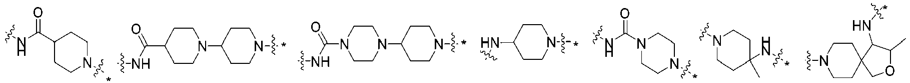 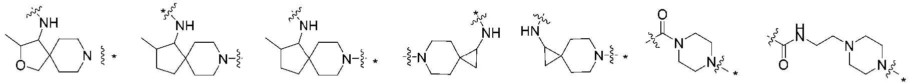 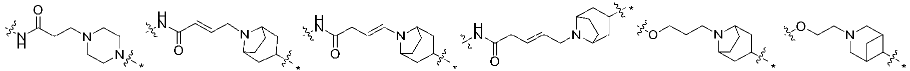 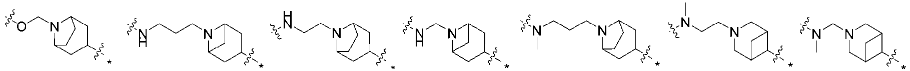 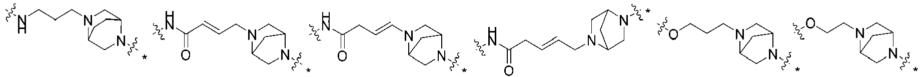 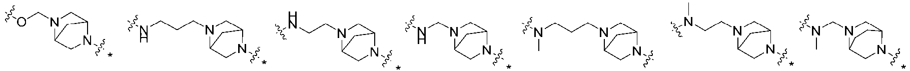 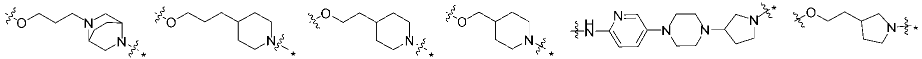 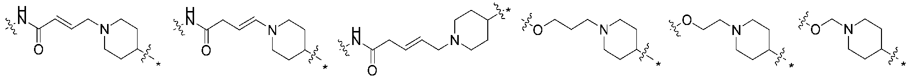 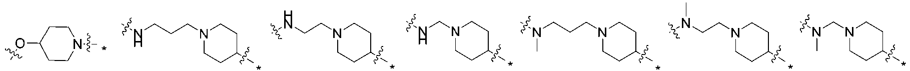 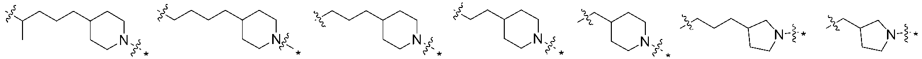 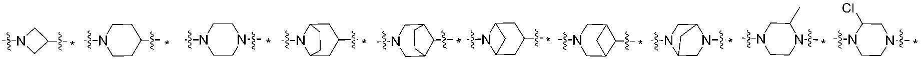 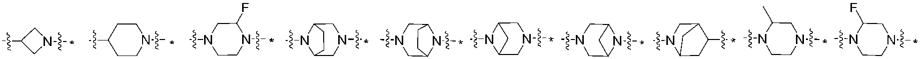 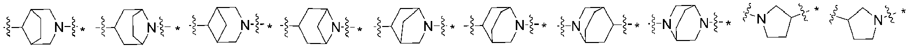 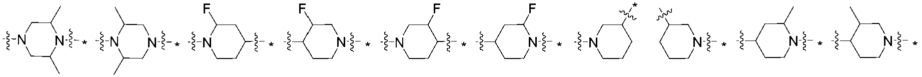 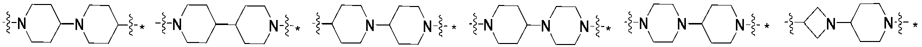 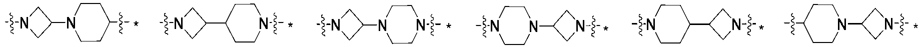 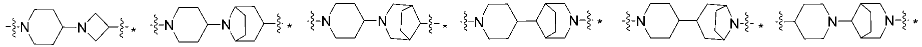 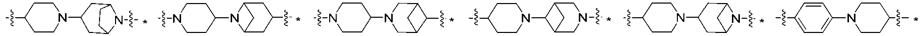 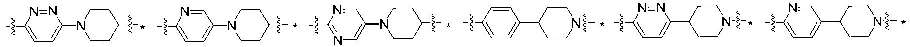 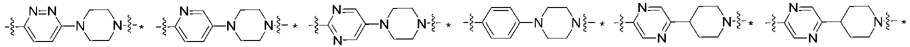 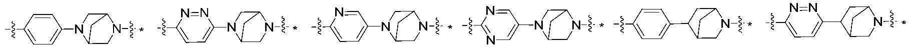 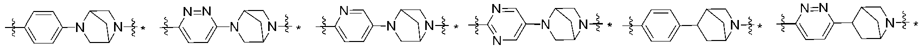 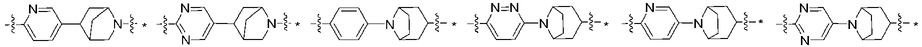 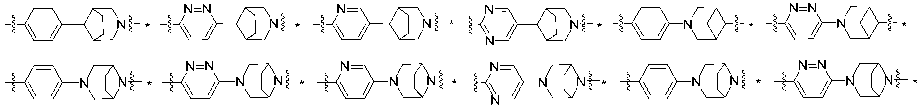 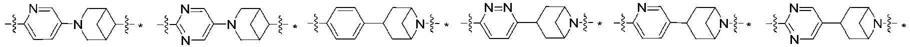 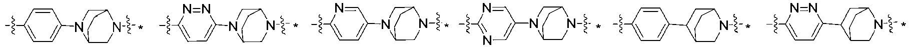 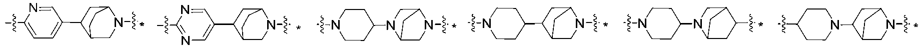 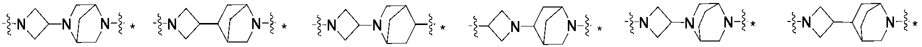 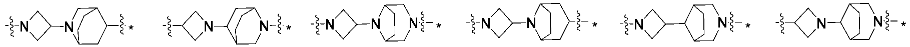 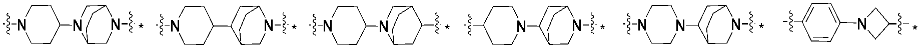 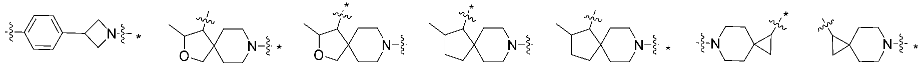 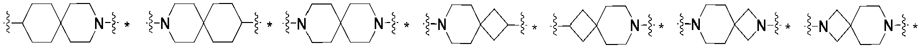 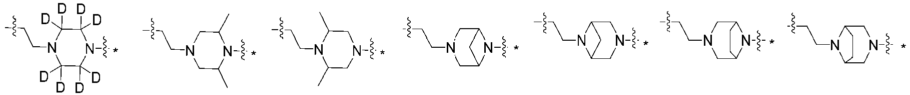 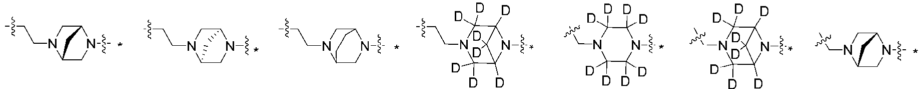 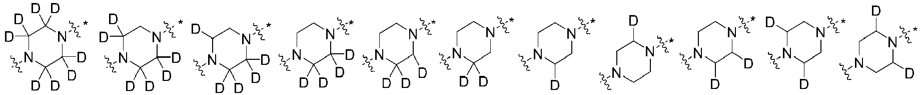 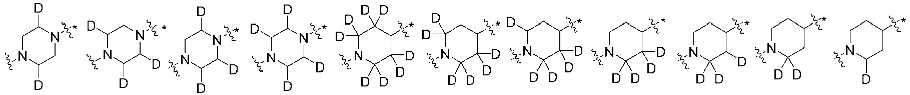 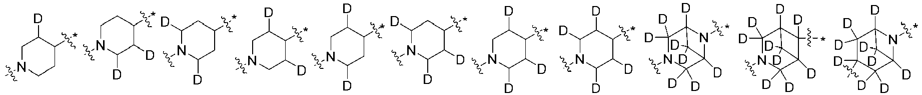 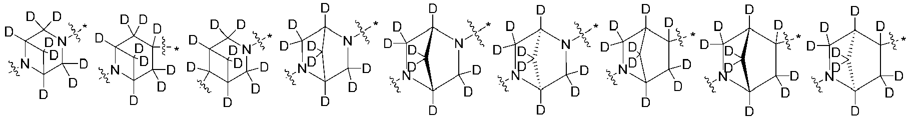 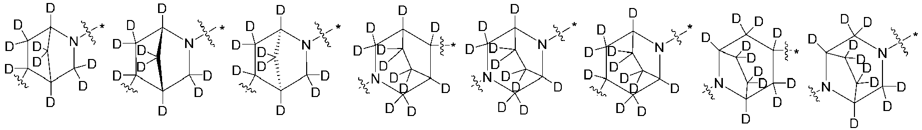 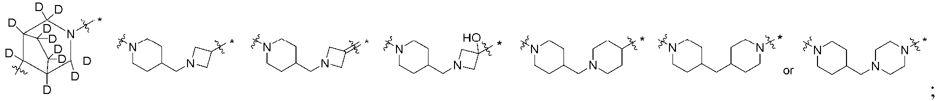 wherein the groups are independently optionally substituted with one or more substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, or any combination thereof; and symbol * indicates the point of attachment to LIN.

9. The compound of Formula (I) or salts, enantiomers, stereoisomers, solvates, isotopically enriched analogs, prodrugs, or polymorphs thereof as claimed in claim 1, wherein
(i) ring W¹ in the structure of Formula (ULM) represents: C₆₋₂₀ arylene or 5- to 20-membered monocyclic, bicyclic, or polycyclic heteroarylene, wherein the C₆₋₂₀ arylene and the 5- to 20-membered monocyclic, bicyclic, or polycyclic heteroarylene are each independently optionally substituted with m identical or different Rₐ₅; preferably wherein the ring W¹ represents the following groups which are optionally substituted with m identical or different Rₐ₅: phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene;
wherein each Rₐ₅ independently represents halogen, hydroxy, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl or C₂₋₆ alkynyl; and m represents an integer of 0, 1, 2, 3, or 4;
and/or
(ii) X in the structure of Formula (ULM) represents a bond or -R_{b1}-N(Rₐ₆)-R_{b2}-, where Rₐ₆ represents hydrogen or C₁₋₆ alkyl, and R_{b1} and R_{b2} each independently represent optionally substituted C₀₋₂ alkylene, preferably wherein the substituents of said optionally substituted C₀₋₂ alkylene are optionally selected from deuterium, halogen, hydroxy, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy or any combination thereof.

10. The compound of Formula (I) or salts, enantiomers, stereoisomers, solvates, isotopically enriched analogs, prodrugs, or polymorphs thereof as claimed in claim 1 or 9, wherein the ULM represents a structure of the following Formulae (ULM-1) or (ULM-2): 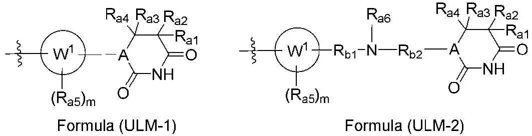 wherein A, Rₐ₁, Rₐ₂, Rₐ₃, Rₐ₄, (Rₐ₅)ₘ, Rₐ₆, R_{b1}, R_{b2}, and ring W¹ are as defined in claim 1 or 9, preferably wherein the ULM represents a structure of the following formulae:

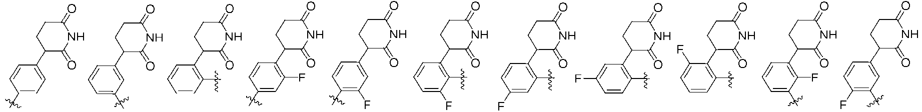

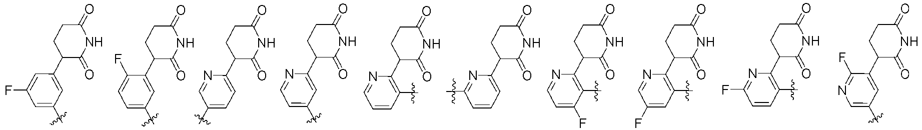

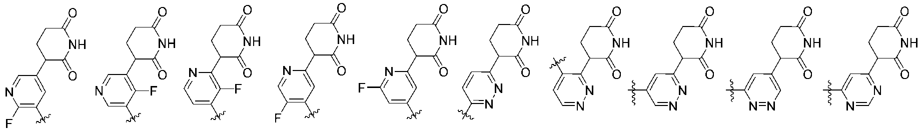

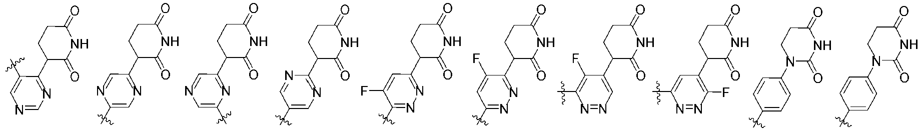

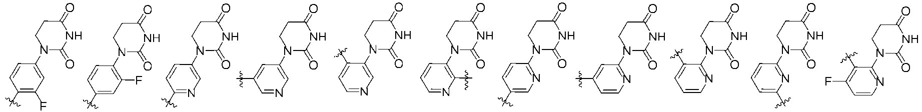

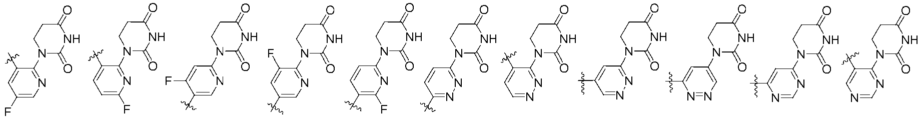

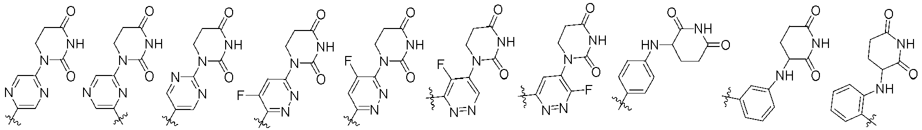

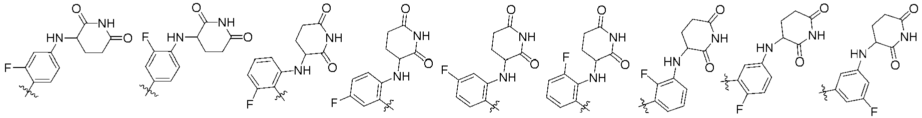

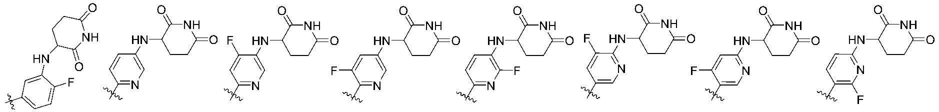

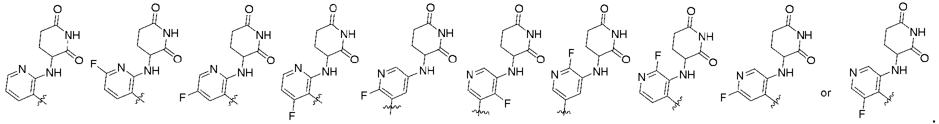

11. The compound of Formula (I) or salts, enantiomers, stereoisomers, solvates, isotopically enriched analogs, prodrugs, or polymorphs thereof as claimed in any one of claims 1-10, wherein the LIN represents:
methylene, which is optionally substituted with 1 to 2 substituents independently selected from deuterium, hydroxy, amino, mercapto, nitro, halogen, cyano, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl or any combination thereof; or
optionally substituted linear or branched C₂₋₁₅ alkylene, wherein one or more groups selected from Rₐ, R_{b}, and any combination thereof are optionally inserted between one or more pairs of adjacent carbon atoms in the backbone carbon chain of the linear or branched C₂₋₁₅ alkylene group; wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and in case that two or more groups Rₐ are inserted into the backbone carbon chain of the linear or branched C₂₋₁₅ alkylene group, the two or more groups Rₐ are not directly connected to each other; and wherein each R_{b} is independently selected from the group consisting of optionally substituted cycloalkylene (e.g., optionally substituted C₃₋₂₀ cycloalkylene), optionally substituted arylene (e.g., optionally substituted C₃₋₂₀ arylene), optionally substituted heterocyclylene (e.g., optionally substituted 4- to 20-membered heterocyclylene), optionally substituted heteroarylene (e.g., optionally substituted 5- to 20-membered heteroarylene), alkynylene (e.g., C₂₋₆ alkynylene), alkenylene (e.g., C₂₋₆ alkenylene), and any combination thereof, wherein the cycloalkylene, arylene, heterocyclylene, and heteroarylene are independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof; and wherein one or more hydrogens of said linear or branched C₂₋₁₅ alkylene are optionally further replaced by a substituent selected from deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof.

12. The compound of Formula (I) or salts, enantiomers, stereoisomers, solvates, isotopically enriched analogs, prodrugs, or polymorphs thereof as claimed in any one of claims 1-10, wherein the LIN represents:
-C₁₋₁₅ alkylene-;
-(C(Rₐ₈)(Rₐ₉))ₙ₁-(Rₐ(C(Rₐ₁₀)(Rₐ₁₁))ₙ₂)ₘ₁-;
-(C(Rₐ₈)(Rₐ₉))ₙ₁-(Rₐ(C(Rₐ₁₀)(Rₐ₁₁))ₙ₂)ₘ₁-(Rₐ(C(Rₐ₁₂)(Rₐ₁₃))ₙ₃)ₘ₂-;
-(C(Rₐ₈)(Rₐ₉))ₙ₁-(Rₐ(C(Rₐ₁₀)(Rₐ₁₁))ₙ₂)ₘ₁-(Rₐ(C(Rₐ₁₂)(Rₐ₁₃))ₙ₃)ₘ₂-(Rₐ(C(Rₐ₁₄)(Rₐ₁₅))ₙ₄)ₘ₃-
-(C(Rₐ₈)(Rₐ₉))ₙ₁-(R_{b}(C(Rₐ₁₀)(Rₐ₁₁))ₙ₂)ₘ₁-;
-(C(Rₐ₈)(Rₐ₉))ₙ₁-(R_{b}(C(Rₐ₁₀)(Rₐ₁₁))ₙ₂)ₘ₁-(R_{b}(C(Rₐ₁₂)(Rₐ₁₃))ₙ₃)ₘ₂-;
-(C(Rₐ₈)(Rₐ₉))ₙ₁-(R_{b}(C(Rₐ₁₀)(Rₐ₁₁))ₙ₂)ₘ₁-(R_{b}(C(Rₐ₁₂)(Rₐ₁₃))ₙ₃)ₘ₂-(R_{b}(C(Rₐ₁₄)(Rₐ₁₅))ₙ₄)ₘ₃-;
-(C(Rₐ₈)(Rₐ₉))ₙ₁-(Rₐ-R_{b}-(C(Rₐ₁₀)(Rₐ₁₁))ₙ₂)ₘ₁-;
-(C(Rₐ₈)(Rₐ₉))ₙ₁-(Rₐ(C(Rₐ₁₀)(Rₐ₁₁))ₙ₂)ₘ₁-(R_{b}(CRₐ₁₂)(Rₐ₁₃))ₙ₃)ₘ₂-;
-(C(Rₐ₈)(Rₐ₉))ₙ₁-(R_{b}-Rₐ-(C(Rₐ₁₀)(Rₐ₁₁))ₙ₂)ₘ₁-;
or
-(C(Rₐ₈)(Rₐ₉))ₙ₁-(R_{b}(C(Rₐ₁₀)(Rₐ₁₁))ₙ₂)ₘ₁-(Rₐ(C(Rₐ₁₂)(Rₐ₁₃))ₙ₃)ₘ₂-;
wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇) and N(Rₐ₇)C(O)N(Rₐ₇), wherein each Rₐ₇ independently represents H or C₁₋₃ alkyl; and each R_{b} is independently selected from the group consisting of optionally substituted cycloalkylene (e.g., optionally substituted C₃₋₂₀ cycloalkylene), optionally substituted arylene (e.g., optionally substituted C₃₋₂₀ arylene), optionally substituted heterocyclylene (e.g., optionally substituted 4- to 20-membered heterocyclylene), optionally substituted heteroarylene (e.g., 5- to 20-membered heteroarylene), alkynylene (e.g., C₂₋₆ alkynylene), alkenylene (e.g., C₂₋₆ alkenylene) and any combination thereof, wherein the cycloalkylene, arylene, heterocyclylene, and heteroarylene are independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof;
Rₐ₈, Rₐ₉, Rₐ₁₀, Rₐ₁₁, Rₐ₁₂, Rₐ₁₃, Rₐ₁₄ and Rₐ₁₅ each independently represent H, deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof; and
n1, n2, n3, n4, m1, m2, and m3 each independently represent an integer of 1, 2, 3, 4, 5, 6, 7, 8, 9 or 10; and
any one or more hydrogens of said C₁₋₁₅ alkylene are optionally further replaced by a substituent selected from: deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof.

13. The compound of Formula (I) or salts, enantiomers, stereoisomers, solvates, isotopically enriched analogs, prodrugs, or polymorphs thereof as claimed in any one of claims 1-10, wherein the LIN represents a structure of the following formulae:
-(C(Rₐ₈)(Rₐ₉))ₙ₁-(O(C(Rₐ₁₀)(Rₐ₁₁))ₙ₂)ₘ₁-;
-(C(Rₐ₈)(Rₐ₉))ₙ₁-(O(C(Rₐ₁₀)(Rₐ₁₁))ₙ₂)ₘ₁-(O(C(Rₐ₁₂)(Rₐ₁₃))ₙ₃)ₘ₂-;
-(C(Rₐ₈)(Rₐ₉))ₙ₁-(O(C(Rₐ₁₀)(Rₐ₁₁))ₙ₂)ₘ₁-(O(C(Rₐ₁₂)(Rₐ₁₃))ₙ₃)ₘ₂- (O(C(Rₐ₁₄)(Rₐ₁₅))ₙ₄)ₘ₃-;
-(C(Rₐ₈)(Rₐ₉))ₙ₁-(N(Rₐ₇)(C(Rₐ₁₀)(Rₐ₁₁))ₙ₂)ₘ₁-;
-(C(Rₐ₈)(Rₐ₉))ₙ₁-(N(Rₐ₇)(C(Rₐ₁₀)(Rₐ₁₁))ₙ₂)ₘ₁-(N(Rₐ₇)(C(Rₐ₁₂)(Rₐ₁₃))ₙ₃)ₘ₂-;
-(C(Rₐ₈)(Rₐ₉)ₙ₁-(N(Rₐ₇)(C(Rₐ₁₀)(Rₐ₁₁))ₙ₂)ₘ₁-(N(Rₐ₇)(C(Rₐ₁₂)(Rₐ₁₃))ₐ₃)ₘ₂-(N(Rₐ₇)(C(Rₐ₁₄)(Rₐ₁₅)ₙ₄)ₘ₃-;
-(C(Rₐ₈)(Rₐ₉))ₙ₁-(C(O)N(Rₐ₇)-(C(Rₐ₁₀)(Rₐ₁₁))ₙ₂)ₘ₁-;
-(C(Rₐ₈)(Rₐ₉))ₙ₁-(C(O)N(Rₐ₇)-(C(Rₐ₁₀)(Rₐ₁₁))ₙ₂)ₘ₁-(C(O)N(Rₐ₇)-(C(Rₐ₁₂)(Rₐ₁₃))ₙ₃)ₘ₂-;
-(C(Rₐ₈)(Rₐ₉))ₙ₁-(C(O)N(Rₐ₇)-(C(Rₐ₁₀)(Rₐ₁₁))ₙ₂)ₘ₁-(C(O)N(Rₐ₇)-(C(Rₐ₁₂)(Rₐ₁₃))ₙ₃)ₘ₂-(C(O)N(Rₐ₇)-(C(Rₐ₁₄)(Rₐ₁₅))ₙ₄)ₘ₃-;
-(C(Rₐ₈)(Rₐ₉))ₙ₁-(C(O)N(Rₐ₇)-(C(Rₐ₁₁)(Rₐ₁₁))ₙ₂)ₘ₁-(O-(C(Rₐ₁₂)(Rₐ₁₃))ₙ₃)ₘ₂-;
-(C(Rₐ₈)(Rₐ₉))ₙ₁-C(O)N(Rₐ₇)-(C(Rₐ₁₀)(Rₐ₁₁))ₙ₂-(O(C(Rₐ₁₂)(Rₐ₁₃))ₙ₃)ₘ₁-;
-(C(Rₐ₈)(Rₐ₉))ₙ₁-(N(Rₐ₇)C(O)-(C(Rₐ₁₀)(Rₐ₁₁))ₙ₂)ₘ₁-;
-(C(Rₐ₈)(Rₐ₉))ₙ₁-(N(Rₐ₇)C(O)-(C(Rₐ₁₀)(Rₐ₁₁))ₙ₂)ₘ₁-(O-(C(Rₐ₁₂)(Rₐ₁₃)))ₙ₃)ₘ₂-;
-(C(Rₐ₈)(Rₐ₉))ₙ₁-(N(Rₐ₇)C(O)-(C(Rₐ₁₀)(Rₐ₁₁))ₙ₂)ₘ₁-(O-(C(Rₐ₁₂)(Rₐ₁₃))ₙ₃)ₘ₂-(O-(C(Rₐ₁₄)(Rₐ₁₅))ₙ₄)ₘ₃-;
-(C(Rₐ₈)(Rₐ₉))ₙ₁-N(Rₐ₇)C(O)-(C(Rₐ₁₀)(Rₐ₁₁))ₙ₂-(O(C(Rₐ₁₂)(Rₐ₁₃))ₙ₃)ₘ₁-;
-(C(Rₐ₈)(Rₐ₉))ₙ₁-N(Rₐ₇)C(O)N(Rₐ₇)-(C(Rₐ₁₀)(Rₐ₁₁))ₙ₂-;
-(C(Rₐ₈)(Rₐ₉))ₙ₁-C(O)-(C(Rₐ₁₀)(Rₐ₁₁))ₙ₂-;
-(C(Rₐ₈)(Rₐ₉))ₙ₁-(arylene-(C(Rₐ₁₀)(Rₐ₁₁))ₙ₂)ₘ₁-;
-(C(Rₐ₈)(Rₐ₉))ₙ₁-(arylene-(C(Rₐ₁₀)(Rₐ₁₁))ₙ₂)ₘ₁-arylene-(C(Rₐ₁₂)(Rₐ₁₃))ₙ₃-;
-(C(Rₐ₈)(Rₐ₉))ₙ₁-(heterocyclylene-(C(Rₐ₁₀)(Rₐ₁₁))ₙ₂)ₘ₁-;
-(C(Rₐ₈)(Rₐ₉))ₙ₁-(heterocyclylene-(C(Rₐ₁₀)(Rₐ₁₁))ₙ₂)ₘ₁-(heterocyclylene-(C(Rₐ₁₂)(Rₐ₁₃))ₙ₃)ₘ₂-;
-(C(Rₐ₈)(Rₐ9))ₙ₁-(heteroarylene-(C(Rₐ₁₀)(Rₐ₁₁))ₙ₂)ₘ₁-;
-(C(Rₐ₈)(Rₐ₉))ₙ₁-(heteroarylene-(C(Rₐ₁₀)(Rₐ₁₁))ₙ₂)ₘ₁-(heteroarylene-(C(Rₐ₁₂)(Rₐ₁₃))ₙ₃)ₘ₂-;
-(C(Rₐ₈)(Rₐ₉))ₙ₁-(cycloalkylene-(C(Rₐ₁₀)(Rₐ₁₁))ₙ₂)ₘ₁-;
or
-(C(Rₐ₈)(Rₐ₉))ₙ₁-(cycloalkylene-(C(Rₐ₁₀)(Rₐ₁₁))ₙ₂)ₘ₁-(cycloalkylene-(C(Rₐ₁₂)(Rₐ₁₃))ₙ₃)ₘ₂-;
wherein each Rₐ₇ independently represents H or C₁₋₃ alkyl;
the cycloalkylene (e.g., C₃₋₂₀ cycloalkylene), arylene (e.g., C₃₋₂₀ arylene), heterocyclylene (e.g., 4- to 20-membered heterocyclylene) and heteroarylene (e.g., 5- to 20-membered heteroarylene) are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof;
Rₐ₈, Rₐ₉, Rₐ₁₀, Rₐ₁₁, Rₐ₁₂, Rₐ₁₃, Rₐ₁₄, and Rₐ₁₅ each independently represent H, deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof; and
n1, n2, n3, n4, m1, m2, and m3 each independently represent an integer of 1, 2, 3, 4, 5, 6, 7, 8, 9 or 10.

14. The compound of Formula (I) or salts, enantiomers, stereoisomers, solvates, isotopically enriched analogs, prodrugs, or polymorphs thereof as claimed in any one of claims 11-13, wherein LIN represents the following groups:
-CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, -(CH₂)₁₃-, -(CH₂)₁₄-, -(CH₂)₁₅-; -CH₂-O-CH₂-, -CH₂-O-(CH₂)₂-, -(CH₂)₁-O-(CH₂)₃-, -(CH₂)₁-O-(CH₂)₄-, -(CH₂)₁-O-(CH₂)₅-, -(CH₂)₁-O-(CH₂)₆-, -(CH₂)₁-O-(CH₂)₇-, -(CH₂)₁-O-(CH₂)₈-, -(CH₂)₁-O-(CH₂)₉-, - (CH₂)₁-O-(CH₂)₁₀-, -(CH₂)₂-O-(CH₂)₁-, -(CH₂)₂-O-(CH₂)₂-, -(CH₂)₂-O-(CH₂)₃-, -(CH₂)₂-O-(CH₂)₄-, -(CH₂)₂-O-(CH₂)₅-, -(CH₂)₂-O-(CH₂)₆-, -(CH₂)₂-O-(CH₂)₇-, -(CH₂)₂-O-(CH₂)₈-, -(CH₂)₂-O-(CH₂)₉-, -(CH₂)₂-O-(CH₂)₁₀-, -(CH₂)₃-O-(CH₂)₁-, -(CH₂)₃-O-(CH₂)₂-, -(CH₂)₃-O-(CH₂)₃-, -(CH₂)₃-O-(CH₂)₄-, -(CH₂)₃-O-(CH₂)₅-, -(CH₂)₃-O-(CH₂)₆-, -(CH₂)₃-O-(CH₂)₇-, -(CH₂)₄-O-(CH₂)₁-, -(CH₂)₄-O-(CH₂)₂-, -(CH₂)₄-O-(CH₂)₃-, -(CH₂)₄-O-(CH₂)₄-, -(CH₂)₄-O-(CH₂)₅-, -(CH₂)₄-O-(CH₂)₆-, -(CH₂)₅-O-(CH₂)₁-, -(CH₂)₅-O-(CH₂)₂-, -(CH₂)₅-O-(CH₂)₃-, -(CH₂)₅-O-(CH₂)₄-, -(CH₂)₅-O-(CH₂)₅-, -(CH₂)₆-O-(CH₂)₁-, -(CH₂)₆-O-(CH₂)₂-, -(CH₂)₆-O-(CH₂)₃-, -(CH₂)₆-O-(CH₂)₄-, -(CH₂)₇-O-(CH₂)₁-, -(CH₂)₇-O-(CH₂)₂-, -(CH₂)₇-O-(CH₂)₃-, -(CH₂)₈-O-(CH₂)₁-, -(CH₂)₈-O-(CH₂)₂-, -CH(CH₃)-O-(CH₂)₁-, -CH(CH₃)-O-(CH₂)₂-, -CH(CH₃)-O-(CH₂)₃-, -CH(CH₃)-O-(CH₂)₄-, -CH(CH₃)-O-(CH₂)₅-, -CH(CH₃)-O-(CH₂)₆-, -CH(CH₃)-O-(CH₂)₇-, -CH(CH₃)-O-(CH₂)₈-, -CH(CH₃)-O-(CH₂)₉-, - CH(CH₃)-O-(CH₂)₁₀-, -CH₂-(O(CH₂)₂)₂-, -CH₂-(O(CH₂)₂)₃-, -CH₂-(O(CH₂)₂)₄-, -CH₂-(O(CH₂)₂)₅-, -CH₂-(O(CH₂)₂)₆-, -CH₂-(O(CH₂)₂)₇-, -CH₂-(O(CH₂)₂)₁-OCH₂-, -CH₂-(O(CH₂)₂)₂-OCH₂-, -CH₂-(O(CH₂)₂)₃-OCH₂-, -CH₂-(O(CH₂)₂)₄-OCH₂-, -CH₂-(O(CH₂)₂)₅-OCH₂-, -CH₂-(O(CH₂)₂)₆-OCH₂-, -CH₂-(O(CH₂)₂)₇-OCH₂-, - (CH₂)₂-(O(CH₂)₂)₂-, -(CH₂)₂-(O(CH₂)₂)₃-, -(CH₂)₂-(O(CH₂)₂)₄-, -(CH₂)₂-(O(CH₂)₂)₅-, -(CH₂)₂-(O(CH₂)₂)₆-, - (CH₂)₂-(O(CH₂)₂)₇-, -(CH₂)₃-(O(CH₂)₂)₂-, -(CH₂)₃-(O(CH₂)₂)₃-, -(CH₂)₃-(O(CH₂)₂)₄-, -(CH₂)₃-(O(CH₂)₂)₅-, - (CH₂)₃-(O(CH₂)₂)₆-, -(CH₂)₃-(O(CH₂)₂)₇-, -(CH₂)₄-(O(CH₂)₂)₂-, -(CH₂)₄-(O(CH₂)₂)₃-, -(CH₂)₄-(O(CH₂)₂)₄-, - (CH₂)₄-(O(CH₂)₂)₅-, -(CH₂)₄-(O(CH₂)₂)₆-, -(CH₂)₄-(O(CH₂)₂)₇-, -CH₂-(O(CH₂)₃)₂-, -CH₂-(O(CH₂)₃)₃-, -CH₂-(O(CH₂)₃)₄-, -(CH₂)₂-(O(CH₂)₃)₂-, -(CH₂)₂-(O(CH₂)₃)₃-, -(CH₂)₂-(O(CH₂)₃)₄-, -(CH₂)₃-(O(CH₂)₃)₂-, -(CH₂)₃-(O(CH₂)₃)₃-, -(CH₂)₃-(O(CH₂)₃)₄-, -CH₂-O-(CH₂)₂-O-(CH₂)₃-, -CH₂-(O(CH₂)₂)₂-(O(CH₂)₃)₂-, -CH₂-(O(CH₂)₂)₃-(O(CH₂)₃)₃-, -CH₂-(O(CH₂)₂)₄-(O(CH₂)₃)₄-, (CH₂)₂-O-(CH₂)₂-O-(CH₂)₃-, -(CH₂)₂-(O(CH₂)₂)₂-(O(CH₂)₃)₂-, -(CH₂)₃-O-(CH₂)₂-O-(CH₂)₃-, -(CH₂)₃-(O(CH₂)₂)₂-(O(CH₂)₃)₂-, -CH₂-O-(CH₂)₃-O-(CH₂)₂-, - CH₂-(O(CH₂)₃)₂-(O(CH₂)₂)₂-, -(CH₂)₂-O-(CH₂)₃-O-(CH₂)₂-, -(CH₂)₂-(O(CH₂)₃)₂-(O(CH₂)₂)₂-, -(CH₂)₃-O-(CH₂)₃-O-(CH₂)₂-, -(CH₂)₃-(O(CH₂)₃)₂-(O(CH₂)₂)₂-, -CH₂-O-(CH₂)₂-O-CH₂-, -(CH₂)₂-O-(CH₂)₂-O-CH₂-, - (CH₂)₂-(O(CH₂)₂)₂-O-(CH₂)₃-, -(CH₂)₂-(O(CH₂)₂)₃-O-(CH₂)₃-, -(CH₂)₂-(O(CH₂)₂)₄-O-(CH₂)₃-, -(CH₂)₅-(O(CH₂)₂)₂-O-(CH₂)₅-, -(CH₂)₅-(O(CH₂)₂)₂-O-(CH₂)₆-, -(CH₂)I-N(Rₐ₇)-(CH₂)1-, -(CH₂)₁-N(Rₐ₇)-(CH₂)₂-, - (CH₂)₁-N(Rₐ₇)-(CH₂)₃-, -(CH₂)₁-N(Rₐ₇)-(CH₂)₄-, -(CH₂)₁-N(Rₐ₇)-(CH₂)₅-, -(CH₂)₁-N(Rₐ₇)-(CH₂)₆-, -(CH₂)₁-N(Rₐ₇)-(CH₂)₇-, -(CH₂)₁-N(Rₐ₇)-(CH₂)₈-, -(CH₂)₁-N(Rₐ₇)-(CH₂)₉-, -(CH₂)₁-N(Rₐ₇)-(CH₂)₁₀-, -(CH₂)₂-N(Rₐ₇)-(CH₂)₁-, -(CH₂)₂-N(Rₐ₇)-(CH₂)₂-, -(CH₂)₂-N(Rₐ₇)-(CH₂)₃-, -(CH₂)₂-N(Rₐ₇)-(CH₂)₄-, -(CH₂)₂-N(Rₐ₇)-(CH₂)₅-, - (CH₂)₂-N(Rₐ₇)-(CH₂)₆-, -(CH₂)₂-N(Rₐ₇)-(CH₂)₇-, -(CH₂)₂-N(Rₐ₇)-(CH₂)₈-, -(CH₂)₂-N(Rₐ₇)-(CH₂)₉-, -(CH₂)₂-N(Rₐ₇)-(CH₂)₁₀-, -(CH₂)₃-N(Rₐ₇)-(CH₂)₁-, -(CH₂)₃-N(Rₐ₇)-(CH₂)₂-, -(CH₂)₃-N(Rₐ₇)-(CH₂)₃-, -(CH₂)₄-N(Rₐ₇)-(CH₂)₁-, -(CH₂)₄-N(Rₐ₇)-(CH₂)₂-, -(CH₂)₄-N(Rₐ₇)-(CH₂)₃-, -(CH₂)₄-N(Rₐ₇)-(CH₂)₄-, -(CH₂)₅-N(Rₐ₇)-(CH₂)₁-, - (CH₂)₅-N(Rₐ₇)-(CH₂)₂-, -(CH₂)₅-N(Rₐ₇)-(CH₂)₃-, -(CH₂)₅-N(Rₐ₇)-(CH₂)₄-, -(CH₂)₅-N(Rₐ₇)-(CH₂)₅-, -(CH₂)₆-N(Rₐ₇)-(CH₂)₁-, -(CH₂)₆-N(Rₐ₇)-(CH₂)₂-, -(CH₂)₆-N(Rₐ₇)-(CH₂)₃-, -(CH₂)₇-N(Rₐ₇)-(CH₂)₁-, -(CH₂)₇-N(Rₐ₇)-(CH₂)₂-, -(CH₂)₇-N(Rₐ₇)-(CH₂)₃-, -(CH₂)₈-N(Rₐ₇)-(CH₂)₁-, -(CH₂)₈-N(Rₐ₇)-(CH₂)₂-, -(CH₂)₈-N(Rₐ₇)-(CH₂)₃-, - CH(CH₃)-N(Rₐ₇)-(CH₂)₁-, -CH(CH₃)-N(Rₐ₇)-(CH₂)₂-, -CH(CH₃)-N(Rₐ₇)-(CH₂)₃-, -CH(CH₃)-N(Rₐ₇)-(CH₂)₄-, - CH(CH₃)-N(Rₐ₇)-(CH₂)₅-, -CH(CH₃)-N(Rₐ₇)-(CH₂)₆-, -CH(CH₃)-N(Rₐ₇)-(CH₂)₇-, -CH(CH₃)-N(Rₐ₇)-(CH₂)₈-, - CH(CH₃)-N(Rₐ₇)-(CH₂)₉-, -CH(CH₃)-N(Rₐ₇)-(CH₂)₁₀-, -CH₂C(O)NHCH₂-, -(CH₂)₂C(O)NH(CH₂)₂-, - (CH₂)₂C(O)NH(CH₂)₃-, -(CH₂)₂C(O)NH(CH₂)₄-, -(CH₂)₂C(O)NH(CH₂)₅-, -(CH₂)₃C(O)NH(CH₂)₃-, - (CH₂)₃C(O)NH(CH₂)₄-, -(CH₂)₄C(O)NH(CH₂)₄-, -(CH₂)₅C(O)NH(CH₂)₅-, -(CH₂)₆C(O)NH(CH₂)₇-, - (CH₂)₆C(O)NH(CH₂)₆-, -(CH₂)₇C(O)NH(CH₂)₇-, -(CH₂)₂C(O)NH(CH₂)₂-O-(CH₂)₂-, -CH₂NHC(O)CH₂-, - (CH₂)₂NHC(O)(CH₂)₂-, -(CH₂)₂NHC(O)(CH₂)₃-, -(CH₂)₂NHC(O)(CH₂)₄-, -(CH₂)₂NHC(O)(CH₂)₅-, - (CH₂)₃NHC(O)(CH₂)₃-, -(CH₂)₃NHC(O)(CH₂)₄-, -(CH₂)₄NHC(O)(CH₂)₄-, -(CH₂)₅NHC(O)(CH₂)₅-, - (CH₂)₆NHC(O)(CH₂)₇-, -(CH₂)₆NHC(O)(CH₂)₆-, -(CH₂)₇NHC(O)(CH₂)₇-, -(CH₂)₄NHC(O)(CH₂)₈-, - (CH₂)₂NHC(O)(CH₂)₂-O-(CH₂)₂-, -(CH₂)₄NHC(O)CH₂-, -CH₂-phenylene-CH₂-, -CH₂-phenylene-(CH₂)₂-, - CH₂-phenylene-(CH₂)₃-, -CH₂-phenylene-(CH₂)₄-, -CH₂-phenylene-(CH₂)₅-, -CH₂-phenylene-(CH₂)₆-, -CH₂-phenylene-(CH₂)₇-, -CH₂-phenylene-(CH₂)₈-, -(CH₂)₂-phenylene-(CH₂)₁-, -(CH₂)₂-phenylene-(CH₂)₂-, - (CH₂)₂-phenylene-(CH₂)₃-, -(CH₂)₂-phenylene-(CH₂)₄-, -(CH₂)₂-phenylene-(CH₂)₅-, -(CH₂)₂-phenylene-(CH₂)₆-, -(CH₂)₂-phenylene-(CH₂)₇-, -(CH₂)₂-phenylene-(CH₂)₈-, -(CH₂)₃-phenylene-CH₂-, -(CH₂)₃-phenylene-(CH₂)₂-, -(CH₂)₃-phenylene-(CH₂)₃-, -(CH₂)₃-phenylene-(CH₂)₄-, -(CH₂)₃-phenylene-(CH₂)₅-, - (CH₂)₃-phenylene-(CH₂)₆-, -(CH₂)₃-phenylene-(CH₂)₇-, -(CH₂)₃-phenylene-(CH₂)₈-, -(CH₂)₄-phenylene-CH₂-, -(CH₂)₄-phenylene-(CH₂)₂-, -(CH₂)₄-phenylene-(CH₂)₃-, -(CH₂)₄-phenylene-(CH₂)₄-, -(CH₂)₄-phenylene-(CH₂)₅-, -(CH₂)₄-phenylene-(CH₂)₆-, -(CH₂)₄-phenylene-(CH₂)₇-, -(CH₂)₄-phenylene-(CH₂)₈-, -(CH₂)₅-phenylene-(CH₂)₁-, -(CH₂)₅-phenylene-(CH₂)₂-, -(CH₂)₅-phenylene-(CH₂)₃-, -(CH₂)₅-phenylene-(CH₂)₄-, - (CH₂)₅-phenylene-(CH₂)₅-, -(CH₂)₅-phenylene-(CH₂)₆-, -(CH₂)₅-phenylene-(CH₂)₇-, -(CH₂)₅-phenylene-(CH₂)₈-, -(CH₂)₆-phenylene-(CH₂)₁-, -(CH₂)₆-phenylene-(CH₂)₂-, -(CH₂)₆-phenylene-(CH₂)₃-, -(CH₂)₆-phenylene-(CH₂)₄-, -(CH₂)₆-phenylene-(CH₂)₅-, -(CH₂)₆-phenylene-(CH₂)₆-, -(CH₂)₆-phenylene-(CH₂)₇-, - (CH₂)₆-phenylene-(CH₂)₈-, -(CH₂)₇-phenylene-(CH₂)₁-, -(CH₂)₇-phenylene-(CH₂)₂-, -(CH₂)₇-phenylene-(CH₂)₃-, -(CH₂)₇-phenylene-(CH₂)₄-, -(CH₂)₇-phenylene-(CH₂)₈-, -(CH₂)₈-phenylene-CH₂-, -(CH₂)₈-phenylene-(CH₂)₂-, -(CH₂)₈-phenylene-(CH₂)₃-, -(CH₂)₈-phenylene-(CH₂)₄-, -(CH₂)₈-phenylene-(CH₂)₅-, - (CH₂)₈-phenylene-(CH₂)₆-, -(CH₂)₈-phenylene-(CH₂)₇-, -CH₂-N(Rₐ₇)-CH₂-phenylene-CH₂-, -CH₂-N(Rₐ₇)-CH₂-phenylene-(CH₂)₂-, -CH₂-N(Rₐ₇)-CH₂-phenylene-(CH₂)₃-, -CH₂-N(Rₐ₇)-CH₂-phenylene-(CH₂)₄-, -CH₂-N(Rₐ₇)-CH₂-phenylene-(CH₂)₅-, -CH₂-N(Rₐ₇)-CH₂-phenylene-(CH₂)₆-, -CH₂-N(Rₐ₇)-CH₂-phenylene-(CH₂)₇-, -CH₂-N(Rₐ₇)-CH₂-phenylene-(CH₂)₈-, -CH₂-N(Rₐ₇)-(CH₂)₂-phenylene-CH₂-, -CH₂-N(Rₐ₇)-(CH₂)₂-phenylene-(CH₂)₂-, -CH₂-N(Rₐ₇)-(CH₂)₂-phenylene-(CH₂)₃-, -CH₂-N(Rₐ₇)-(CH₂)₂-phenylene-(CH₂)₄-, -CH₂-N(Rₐ₇)-(CH₂)₂-phenylene-(CH₂)₅-, -CH₂-N(Rₐ₇)-(CH₂)₂-phenylene-(CH₂)₆-, -CH₂-N(Rₐ₇)-(CH₂)₂-phenylene-(CH₂)₇-, -CH₂-N(Rₐ₇)-(CH₂)₂-phenylene-(CH₂)₈-, -(CH₂)₂-N(Rₐ₇)-CH₂-phenylene-CH₂-, -(CH₂)₂-N(Rₐ₇)-CH₂-phenylene-(CH₂)₂-, -(CH₂)₂-N(Rₐ₇)-CH₂-phenylene-(CH₂)₃-, -(CH₂)₂-N(Rₐ₇)-CH₂-phenylene-(CH₂)₄-, - (CH₂)₂-N(Rₐ₇)-CH₂-phenylene-(CH₂)₅-, -(CH₂)₂-N(Rₐ₇)-CH₂-phenylene-(CH₂)₆-, -(CH₃)₂-N(Rₐ₇)-CH₂-phenylene-(CH₂)₇-, -(CH₂)₂-N(Rₐ₇)-CH₂-phenylene-(CH₂)₈-, -(CH₂)₃-N(Rₐ₇)-CH₂-phenylene-CH₂-, -(CH₂)₃-N(Rₐ₇)-CH₂-phenylene-(CH₂)₂-, -(CH₂)₃-N(Rₐ₇)-CH₂-phenylene-(CH₂)₃-, -(CH₂)₃-N(Rₐ₇)-CH₂-phenylene-(CH₂)₈-, -(CH₂)₄-N(Rₐ₇)-CH₂-phenylene-CH₂-, -(CH₂)₄-N(Rₐ₇)-CH₂-phenylene-(CH₂)₂-, -(CH₂)₄-N(Rₐ₇)-CH₂-phenylene-(CH₂)₃-, -(CH₂)₄-N(Rₐ₇)-CH₂-phenylene-(CH₂)₈-, -(CH₂)₅-N(Rₐ₇)-CH₂-phenylene-(CH₂)₃-, - (CH₂)₅-N(Rₐ₇)-CH₂-phenylene-(CH₂)₈-, -(CH₂)₆-N(Rₐ₇)-CH₂-phenylene-(CH₂)₃-, -(CH₂)₆-N(Rₐ₇)-CH₂-phenylene-(CH₂)₈-, -(CH₂)₇-N(Rₐ₇)-CH₂-phenylene-(CH₂)₃-, -(CH₂)₈-N(Rₐ₇)-CH₂-phenylene-CH₂-, -(CH₂)₈-N(Rₐ₇)-CH₂-phenylene-(CH₂)₂-, -(CH₂)₈-N(Rₐ₇)-CH₂-phenylene-(CH₂)₃-, -(CH₂)₈-N(Rₐ₇)-CH₂-phenylene-(CH₂)₄-, -(CH₂)₈-N(Rₐ₇)-CH₂-phenylene-(CH₂)₅-, -(CH₂)₈-N(Rₐ₇)-CH₂-phenylene-(CH₂)₆-, -CH₂-piperidinylene-CH₂-, -CH₂-piperidinylene-(CH₂)₂-, -CH₂-piperidinylene-(CH₂)₃-, -CH₂-piperidinylene-(CH₂)₄-, -CH₂-piperidinylene-(CH₂)₅-, -CH₂-piperidinylene-(CH₂)₆-, -CH₂-piperidinylene-(CH₂)₇-, -CH₂-piperidinylene-(CH₂)₈-, -(CH₂)₂-piperidinylene-(CH₂)₁-, -(CH₂)₂-piperidinylene-(CH₂)₂-, -(CH₂)₂-piperidinylene-(CH₂)₃-, -(CH₂)₂-piperidinylene-(CH₂)₄-, -(CH₂)₂-piperidinylene-(CH₂)₅-, -(CH₂)₂-piperidinylene-(CH₂)₆-, -(CH₂)₂-piperidinylene-(CH₂)₇-, -(CH₂)₂-piperidinylene-(CH₂)₈-, -(CH₂)₃-piperidinylene-CH₂-, -(CH₂)₃-piperidinylene-(CH₂)₂-, -(CH₂)₃-piperidinylene-(CH₂)₃-, -(CH₂)₃-piperidinylene-(CH₂)₄-, -(CH₂)₃-piperidinylene-(CH₂)₅-, -(CH₂)₃-piperidinylene-(CH₂)₆-, -(CH₂)₃-piperidinylene-(CH₂)₇-, -(CH₂)₃-piperidinylene-(CH₂)₈-, -(CH₂)₄-piperidinylene-CH₂-, -(CH₂)₄-piperidinylene-(CH₂)₂-, -(CH₂)₄-piperidinylene-(CH₂)₃-, -(CH₂)₄-piperidinylene-(CH₂)₄-, -(CH₂)₄-piperidinylene-(CH₂)₅-, -(CH₂)₄-piperidinylene-(CH₂)₆-, -(CH₂)₄-piperidinylene-(CH₂)₇-, -(CH₂)₄-piperidinylene-(CH₂)₈-, -(CH₂)₅-piperidinylene-(CH₂)₁-, -(CH₂)₅-piperidinylene-(CH₂)₂-, -(CH₂)₅-piperidinylene-(CH₂)₃-, -(CH₂)₅-piperidinylene-(CH₂)₄-, -(CH₂)₅-piperidinylene-(CH₂)₅-, -(CH₂)₅-piperidinylene-(CH₂)₆-, -(CH₂)₅-piperidinylene-(CH₂)₇-, -(CH₂)₅-piperidinylene-(CH₂)₈-, -(CH₂)₆-piperidiny lene-( CH₂) ₁-, -(CH₂)₆-piperidinylene-(CH₂)₂-, -(CH₂)₆-piperidinylene-(CH₂)₃-, -(CH₂)₆-piperidinylene-(CH₂)₄-, -(CH₂)₆-piperidinylene-(CH₂)₅-, -(CH₂)₆-piperidinylene-(CH₂)₆-, -(CH₂)₆-piperidinylene-(CH₂)₇-, -(CH₂)₆-piperidinylene-(CH₂)₈-, -(CH₂)₇-piperidinylene-(CH₂)₁-, -(CH₂)₇-piperidinylene-(CH₂)₂-, -(CH₂)₇-piperidinylene-(CH₂)₃-, -(CH₂)₇-piperidinylene-(CH₂)₄-, -(CH₂)₇-piperidinylene-(CH₂)₈-, -(CH₂)₈-piperidinylene-CH₂-, -(CH₂)₈-piperidinylene-(CH₂)₂-, -(CH₂)₈-piperidinylene-(CH₂)₃-, -(CH₂)₈-piperidinylene-(CH₂)₄-, -(CH₂)₈-piperidinylene-(CH₂)₅-, -(CH₂)₈-piperidinylene-(CH₂)₆-, -(CH₂)₈-piperidinylene-(CH₂)₇-, -CH₂-N(Rₐ₇)-CH₂-piperidinylene-CH₂-, -CH₂-N(Rₐ₇)-CH₂-piperidinylene-(CH₂)₂-, -CH₂-N(Rₐ₇)-CH₂-piperidinylene-(CH₂)₃-, -CH₂-N(Rₐ₇)-CH₂-piperidinylene-(CH₂)₄-, -CH₂-N(Rₐ₇)-CH₂-piperidinylene-(CH₂)₅-, -CH₂-N(Rₐ₇)-CH₂-piperidinylene-(CH₂)₆-, -CH₂-N(Rₐ₇)-CH₂-piperidinylene-(CH₂)₇-, -CH₂-N(Rₐ₇)-CH₂-piperidinylene-(CH₂)₈-, -CH₂-N(Rₐ₇)-(CH₂)₂-piperidinylene-CH₂-, -CH₂-N(Rₐ₇)-(CH₂)₂-piperidinylene-(CH₂)₂-, -CH₂-N(Rₐ₇)-(CH₂)₂-piperidinylene-(CH₂)₃-, -CH₂-N(Rₐ₇)-(CH₂)₂-piperidinylene-(CH₂)₄-, -CH₂-N(Rₐ₇)-(CH₂)₂-piperidinylene-(CH₂)₅-, -CH₂-N(Rₐ₇)-(CH₂)₂-piperidinylene-(CH₂)₆-, -CH₂-N(Rₐ₇)-(CH₂)₂-piperidinylene-(CH₂)₇-, -CH₂-N(Rₐ₇)-(CH₂)₂-piperidinylene-(CH₂)₈-, -(CH₂)₂-N(Rₐ₇)-CH₂-piperidinylene-CH₂-, -(CH₂)₂-N(Rₐ₇)-CH₂-piperidinylene-(CH₂)₂-, -(CH₂)₂-N(R_{ay})-CH₂-piperidinylene-(CH₂)₃-, -(CH₂)₂-N(Rₐ₇)-CH₂-piperidinylene-(CH₂)₄-, -(CH₂)₂-N(Rₐ₇)-CH₂-piperidinylene-(CH₂)₅-, -(CH₂)₂-N(R.₇)-CH₂-piperidinylene-(CH₂)₆-, -(CH₂)₂-N(Rₐ₇)-CH₂-piperidinylene-(CH₂)₇-, -(CH₂)₂-N(Rₐ₇)-CH₂-piperidinylene-(CH₂)₈-, -(CH₂)₃-N(Rₐ₇)-CH₂-piperidinylene-CH₂-, -(CH₂)₃-N(R_{ay})-CH₂-piperidinylene-(CH₂)₂-, -(CH₂)₃-N(Rₐ₇)-CH₂-piperidinylene-(CH₂)₃-, -(CH₂)₃-N(Rₐ₇)-CH₂-piperidinylene-(CH₂)₈-, -(CH₂)₄-N(Rₐ₇)-CH₂-piperidinylene-CH₂-, -(CH₂)₄-N(Rₐ₇)-CH₂-piperidinylene-(CH₂)₂-, -(CH₂)₄-N(Rₐ₇)-CH₂-piperidinylene-(CH₂)₃-, -(CH₂)₄-N(Rₐ₇)-CH₂-piperidinylene-(CH₂)₈-, -(CH₂)₅-N(Rₐ₇)-CH₂-piperidinylene-(CH₂)₃-, -(CH₂)₅-N(Rₐ₇)-CH₂-piperidinylene-(CH₂)₈-, -(CH₂)₆-N(Rₐ₇)-CH₂-piperidinylene-(CH₂)₃-, -(CH₂)₆-N(Rₐ₇)-CH₂-piperidinylene-(CH₂)₈-, -(CH₂)₇-N(Rₐ₇)-CH₂-piperidinylene-(CH₂)₃-, -(CH₂)₈-N(Rₐ₇)-CH₂-piperidinylene-CH₂-, -(CH₂)₈-N(Rₐ₇)-CH₂-piperidinylene-(CH₂)₂-, -(CH₂)₈-N(Rₐ₇)-CH₂-piperidinylene-(CH₂)₃-, -(CH₂)₈-N(Rₐ₇)-CH₂-piperidinylene-(CH₂)₄-, -(CH₂)₈-N(Rₐ₇)-CH₂-piperidinylene-(CH₂)₅-, -(CH₂)₈-N(Rₐ₇)-CH₂-piperidinylene-(CH₂)₆-, -CH₂-piperazinylene-CH₂-, -CH₂-piperazinylene-(CH₂)₂-, -CH₂-piperazinylene-(CH₂)₃-, -CH₂-piperazinylene-(CH₂)₄-, -CH₂-piperazinylene-(CH₂)₅-, -CH₂-piperazinylene-(CH₂)₆-, -CH₂-piperazinylene-(CH₂)₇-, -CH₂-piperazinylene-(CH₂)₈-, -(CH₂)₂-piperazinylene-(CH₂)₁-, -(CH₂)₂-piperazinylene-(CH₂)₂-, -(CH₂)₂-piperazinylene-(CH₂)₃-,-(CH₂)₂-piperazinylene-(CH₂)₄-, -(CH₂)₂-piperazinylene-(CH₂)₅-, -(CH₂)₂-piperazinylene-(CH₂)₆-, -(CH₂)₂-piperazinylene-(CH₂)₇-, -(CH₂)₂-piperazinylene-(CH₂)₈-, -(CH₂)₃-piperazinylene-CH₂-, -(CH₂)₃-piperazinylene-(CH₂)₂-, -(CH₂)₃-piperazinylene-(CH₂)₃-, -(CH₂)₃-piperazinylene-(CH₂)₄-, -(CH₂)₃-piperazinylene-(CH₂)₅-, -(CH₂)₃-piperazinylene-(CH₂)₆-, -(CH₂)₃-piperazinylene-(CH₂)₇-, -(CH₂)₃-piperazinylene-(CH₂)₈-, -(CH₂)₄-piperazinylene-CH₂-, -(CH₂)₄-piperazinylene-(CH₂)₂-, -(CH₂)₄-piperazinylene-(CH₂)₃-, -(CH₂)₄-piperazinylene-(CH₂)₄-, -(CH₂)₄-piperazinylene-(CH₂)₅-, -(CH₂)₄-piperazinylene-(CH₂)₆-, -(CH₂)₄-piperazinylene-(CH₂)₇-, -(CH₂)₄-piperazinylene-(CH₂)₈-, -(CH₂)₅-piperazinylene-(CH₂)₁-, -(CH₂)₅-piperazinylene-(CH₂)₂-, -(CH₂)₅-piperazinylene-(CH₂)₃-, -(CH₂)₅-piperazinylene-(CH₂)₈-, -(CH₂)₅-piperazinylene-(CH₂)₅-, -(CH₂)₅-piperazinylene-(CH₂)₆-, -(CH₂)₅-piperazinylene-(CH₂)₇-, -(CH₂)₅-piperazinylene-(CH₂)₈-, -(CH₂)₆-piperazinylene-(CH₂)₁-, -(CH₂)₆-piperazinylene-(CH₂)₂-, -(CH₂)₆-piperazinylene-(CH₂)₃-, -(CH₂)₆-piperazinylene-(CH₂)₄-, -(CH₂)₆-piperazinylene-(CH₂)₅-, -(CH₂)₆-piperazinylene-(CH₂)₆-, -(CH₂)₆-piperazinylene-(CH₂)₇-, -(CH₂)₆-piperazinylene-(CH₂)₈-, -(CH₂)₇-piperazinylene-(CH₂)₁-, -(CH₂)₇-piperazinylene-(CH₂)₂-, -(CH₂)₇-piperazinylene-(CH₂)₃-, -(CH₂)₇-piperazinylene-(CH₂)₄-, -(CH₂)₈-piperazinylene-CH₂-, -(CH₂)₈-piperazinylene-(CH₂)₂-, -(CH₂)₈-piperazinylene-(CH₂)₃-, -(CH₂)₈-piperazinylene-(CH₂)₄-, -(CH₂)₈-piperazinylene-(CH₂)₅-, or -(CH₂)₈-piperazinylene-(CH₂)₆-;
wherein each Rₐ₇ independently represents H or C₁₋₃ alkyl;
the phenylene, piperazinylene and piperidinylene are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof.

15. The compound of Formula (I) or salts, enantiomers, stereoisomers, solvates, isotopically enriched analogs, prodrugs, or polymorphs thereof as claimed in claim 1, which is also represented by Formula (I-1), Formula (I-2), Formula (I-3), Formula (I-4), Formula (I-5), Formula (I-6), Formula (I-7), Formula (I-8-1), Formula (I-8-2), Formula (I-8-3), Formula (I-8-4), Formula (I-9), Formula (I-10), Formula (I-11), Formula (I-12), Formula (I-13), Formula (I-14), Formula (I-15), Formula (I-16), Formula (I-17), Formula (I-18), Formula (I-19), Formula (I-20-1), Formula (I-20-2), Formula (I-20-3), Formula (I-20-4), Formula (I-21-1), Formula (I-21-2), Formula (I-22), Formula (I-23), Formula (I-24), Formula (I-25-1), Formula (I-25-2), Formula (I-26), Formula (I-27), Formula (I-28), Formula (I-29), Formula (I-30), Formula (I-31), Formula (I-32), Formula (I-33), Formula (I-34-1), Formula (I-34-2), Formula (I-35), Formula (I-36), Formula (I-37), Formula (I-38), Formula (I-39-1), Formula (I-39-2), Formula (I-40), Formula (I-41), Formula (I-42), Formula (I-43), Formula (I-44), Formula (I-45), Formula (I-46), Formula (I-47), Formula (I-48), Formula (I-49), Formula (I-50), Formula (I-51), Formula (I-52), Formula (I-53), Formula (I-54), or Formula (I-55): 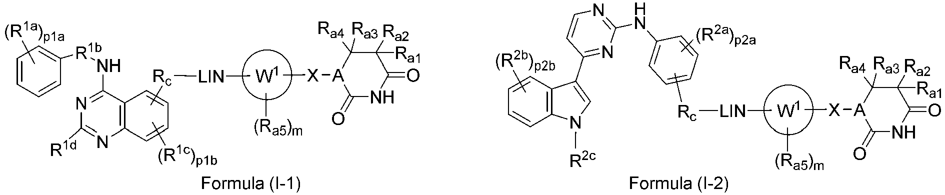 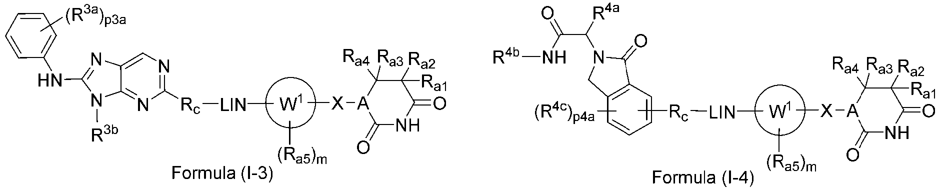 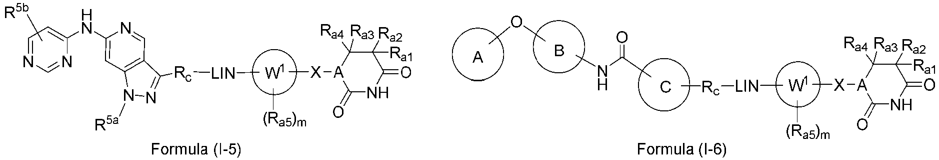 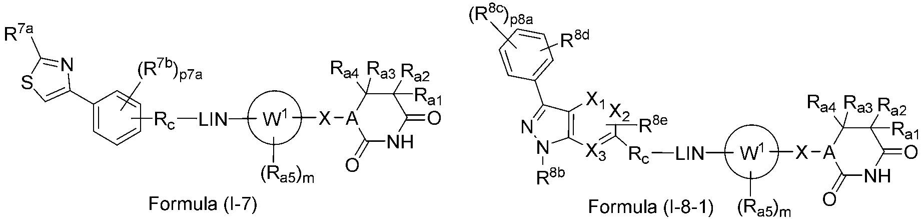 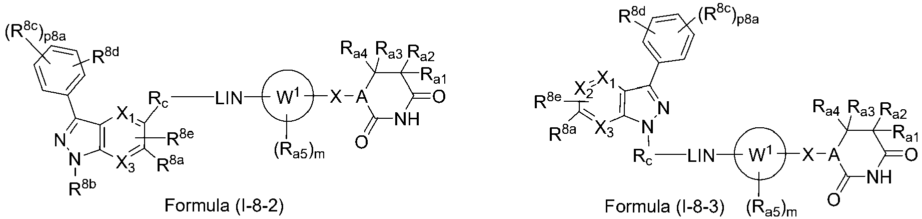 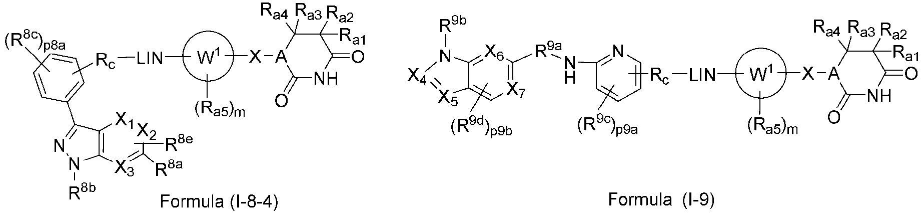 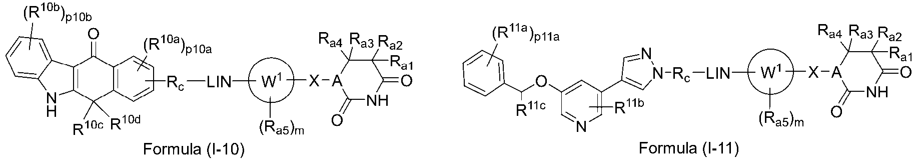 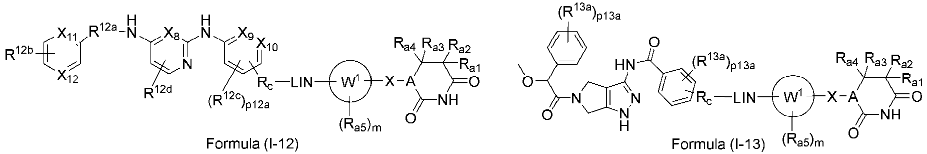 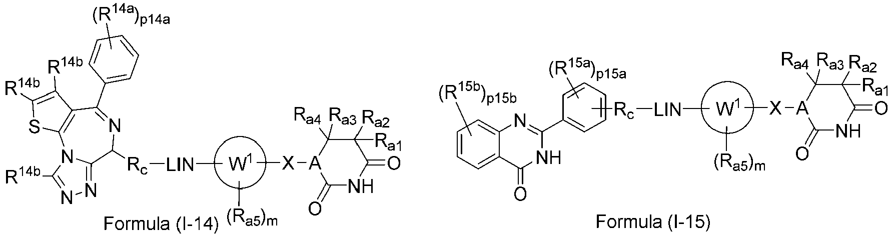 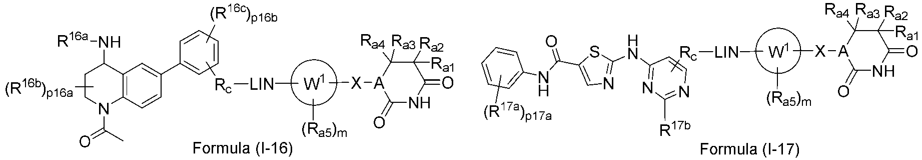 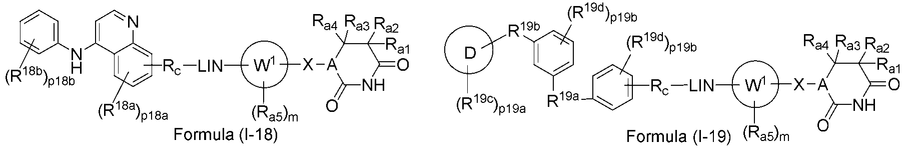 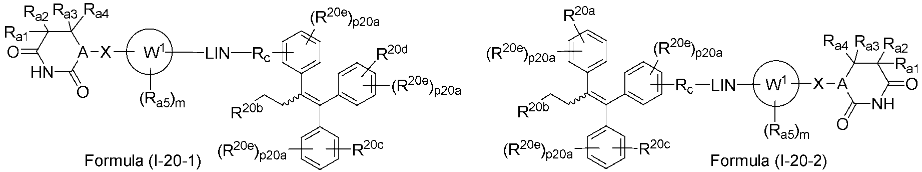 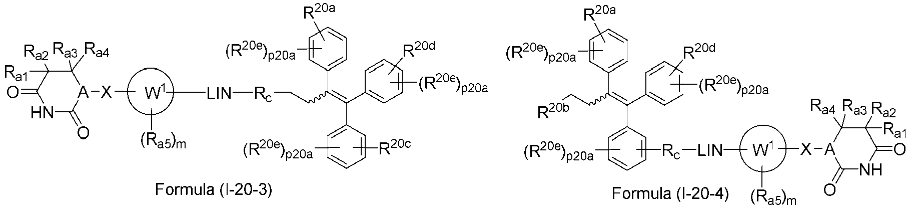 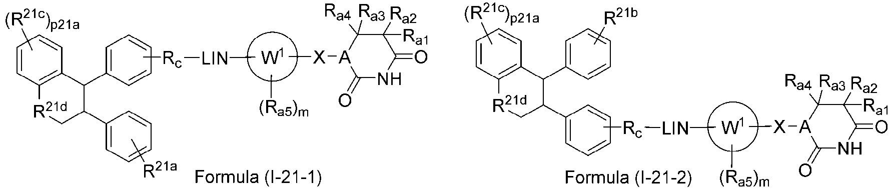 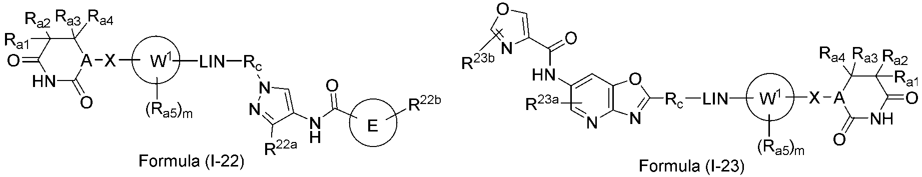 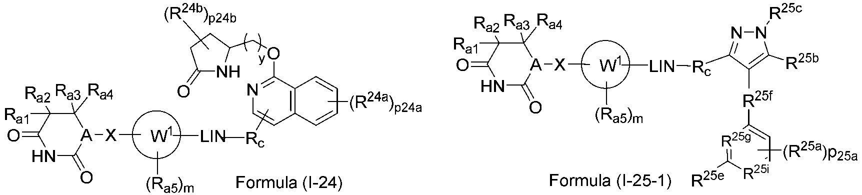 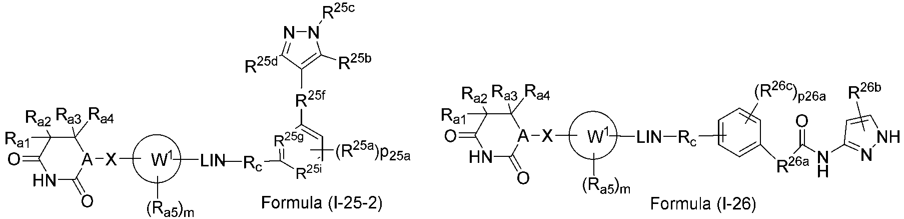 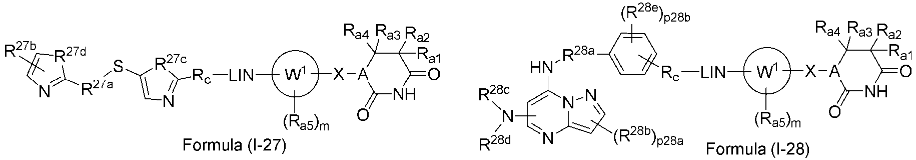 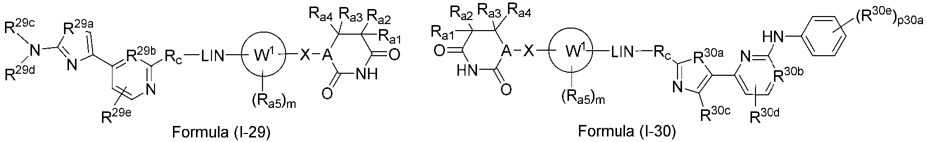 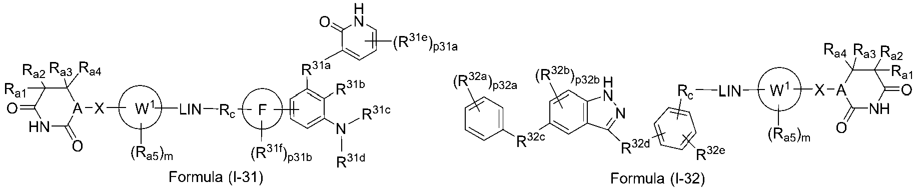 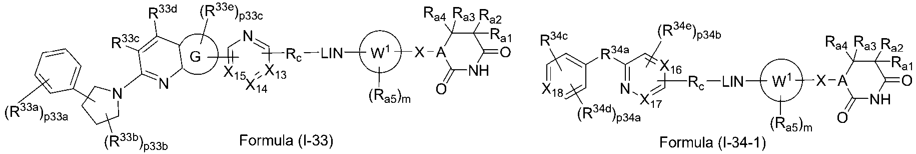 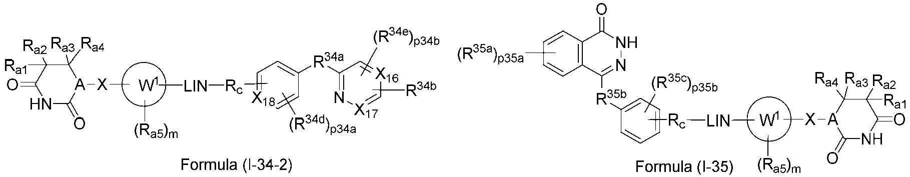 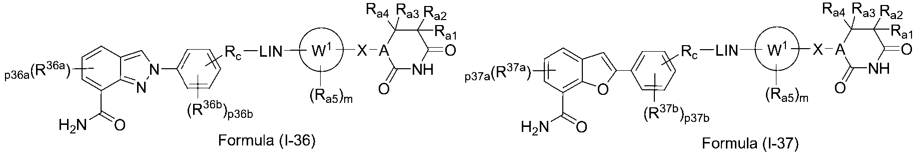 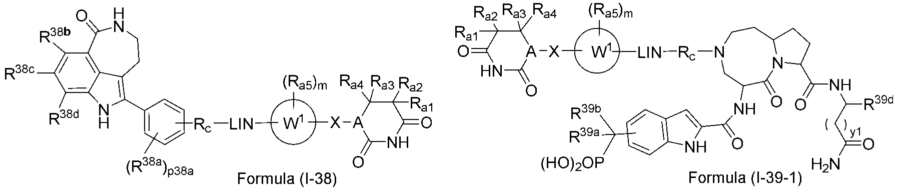 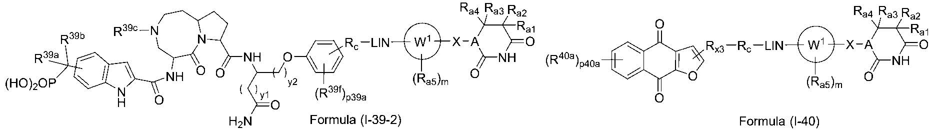 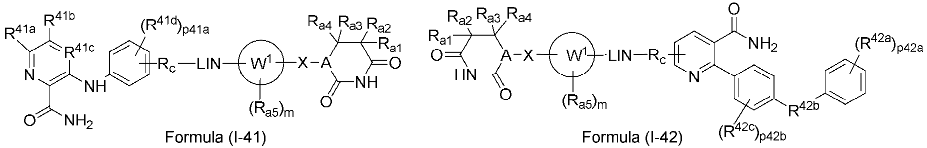 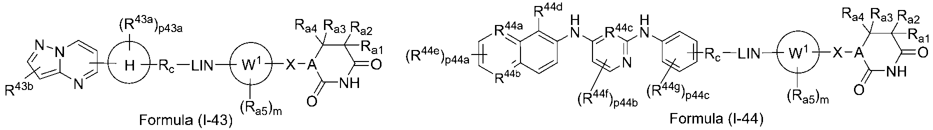 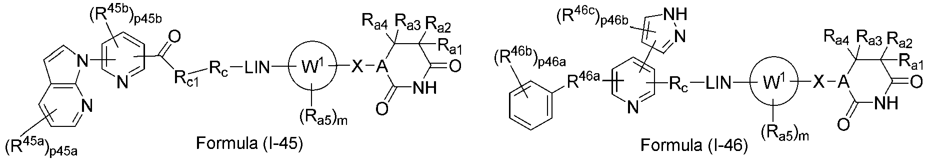 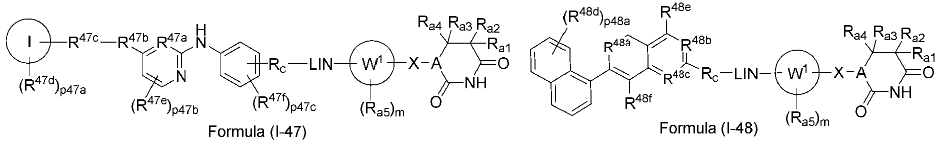 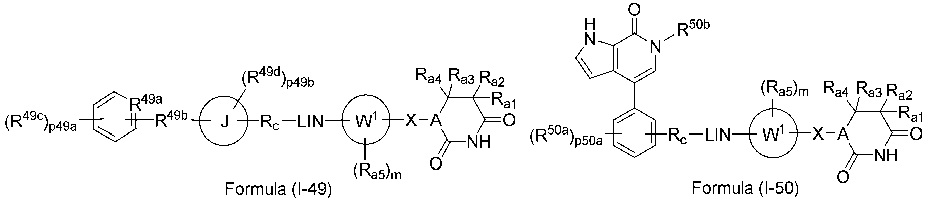 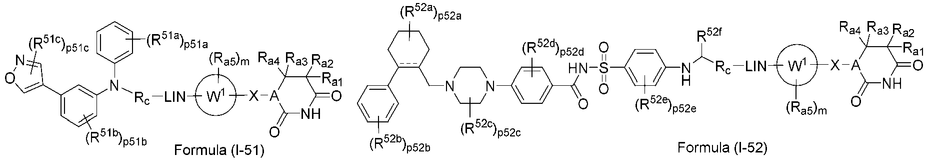 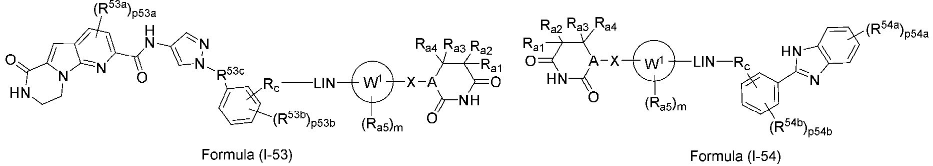 or 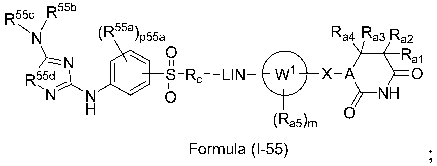 wherein A, Rₐ₁, Rₐ₂, Rₐ₃, Rₐ₄, X, ring W¹, (Rₐ₅)ₘ, LIN, R_{c}, (R^{1a})ₚ₁ₐ, R^{1b}, (R^{1c})_{p1b}, R^{1d}, (R^{2a})ₚ₂ₐ, (R^{2b})_{p2b}, R^{2c}, (R^{3a})ₚ₃ₐ, R^{3b}, R^{4a}, R^{4b}, (R^{4c})ₚ₄ₐ, R^{5a}, R^{5b}, ring A, ring B, ring C, R^{7a}, (R^{7b})ₚ₇ₐ, R^{8a} R^{8b}, (R^{8c})ₚ₈ₐ, R^{8d}, R^{8c}, X₁, X₂, X₃, R^{9a}, R^{9b}, (R^{9c})ₚ₉ₐ, (R^{9d})_{p9b}, X₄, X₅, X₆, X₇, (R^{10a})ₚ₁₀ₐ, (R^{10b})_{p10b}, R^{10c}, R^{10d}, (R^{11a})ₚ₁₁ₐ, R^{11b}, R^{11c}, R^{12a}, R^{12b}, (R^{12c})ₚ₁₂ₐ, R^{12d}, X₈, X₉, X₁₀, X₁₁, X₁₂, (R^{13a})ₚ₁₃ₐ, (R^{14a})ₚ₁₄ₐ, R^{14b} , (R^{15a})ₚ₁₅ₐ, (R^{15b})_{p15b}, R^{16a}, (R^{16b})ₚ₁₆ₐ, (R^{16c})_{p16b}, (R^{17a})ₚ₁₇ₐ, R^{17b}, (R^{18a})ₚ₁₈ₐ, (R^{18b})_{p18b}, R^{19a}, R^{19b}, (R^{19c})ₚ₁₉ₐ, (R^{19d})_{p19b}, R^{20a}, R^{20b}, R^{20c}, R^{20d}, (R^{20c})ₚ₂₀ₐ, R^{21a}, R^{21b}, (R^{21c})_{p21a,} R^{21d}, R^{22a}, R^{22b}, ring E, R^{23a}, R^{23b}, (R^{24a})ₚ₂₄ₐ, (R^{24b})_{p24b}, y, (R^{25a})ₚ₂₅ₐ, R^{25b}, R^{25c}, R^{25d}, R^{25e}, R^{25f}, R^{25g}, R²⁵ⁱ, R^{26a}, R^{26b}, (R^{26c})ₚ₂₆ₐ, R^{27a}, R^{27b}, R^{27c}, R^{27d}, R^{28a}, (R^{28b})ₚ₂₈ₐ, R^{28c}, R^{28d}, (R^{28e})_{p28b}, R^{29a}, R^{29b}, R^{29c}, R^{29d}, R^{29e}, R^{30a}, R^{30b}, R^{30c},R^{30d}, (R^{30e})ₚ₃₀ₐ, R^{31a}, R^{31b}, R^{31c}, R^{31d}, (R^{31e})ₚ₃₁ₐ, (R^{31f})_{p31b}, ring F, (R^{32a})ₚ₃₂ₐ, (R^{32b})_{p32b}, R^{32c}, R^{32d}, R^{32e}, (R^{33a})ₚ₃₃ₐ, (R^{33b})_{p33b}, R^{33c}, R^{33d}, (R^{33e})ₚ₃₃ₑ, X₁₃, X₁₄, X₁₅, ring G, R^{34a}, R^{34b}, R^{34c}, (R^{34d})ₚ₃₄ₐ, (R^{34e})_{p34b}, X₁₆, X₁₇, X₁₈, (R^{35a})ₚ₃₅ₐ, R^{35b}, (R^{35c})_{p35b}, (R^{36a})ₚ₃₆ₐ, (R^{36b})_{p36b}, (R^{37a})ₚ₃₇ₐ, (R^{37b})_{p37b}, (R^{38a})ₚ₃₈ₐ, R^{38b}, R^{38c}, R^{38d} R^{39a}, R^{39b}, R^{39c}, R^{39d}, y1, (R^{40a})ₚ₄₀ₐ, R^{41a}, Rₓ₃, R^{41b}, R^{41c}, (R^{41d})ₚ₄₁ₐ, (R^{42a})_{p42a,} R^{42b}, (R^{42c})_{p42b}, (R^{43a})_{p43a,} R^{43b}, ring H, R^{44a}, R^{44b}, R^{44c}, R^{44d}, (R^{44c})ₚ₄₄ₐ, (R^{44f})_{p44b}, (R^{44g})_{p44c}, (R^{45a})ₚ₄₅ₐ, (R^{45b})_{p45b}, R_{c1}, R^{46a}, (R^{46b})ₚ₄₆ₐ, (R^{46c})_{p46b}, R^{47a}, R^{47b}, R^{47c}, (R^{47d})ₚ₄₇ₐ, (R^{47c})_{p47b}, (R^{47f})_{p47c}, ring I, R^{48a}, R^{48b}, R^{48c}, (R^{48d})ₚ₄₈ₐ, R^{48e}, R^{48f}, R^{49a}, R^{49b}, (R^{49c})ₚ₄₉ₐ, (R^{49d})_{p49b}, ring J , (R^{50a})ₚ₅₀ₐ, R^{50b} (R^{51a})ₚ₅₁ₐ, (R^{51b})_{p51b}, (R^{51c})_{p51c}, (R^{52a})ₚ₅₂ₐ, (R^{52b})_{p52b}, (R^{52c})_{p52c}, (R^{52d})_{p52d}, (R^{52c})_{p52c}, R^{52f}, (R^{53a})ₚ₅₃ₐ, (R^{53b})_{p53b}, R^{53c}, (R^{54a})ₚ₅₄ₐ, (R^{54b})_{p54b}, (R^{55a})ₚ₅₅ₐ, R^{55b}, R^{55c} and R^{55d} are as defined in claim 3.

16. The compound of Formula (I) or salts, enantiomers, stereoisomers, solvates, isotopically enriched analogs, prodrugs, or polymorphs thereof as claimed in claim 1, which is selected from:
N-(2-(4-(4-(2,6-dioxopiperidin-3-yl)benzyl)piperazin-1-yl)ethyl)-4,9-dioxo-4,9-dihydronaphtho[2,3-b]furan-2-carboxamide;
N-(2-(4-(4-(2,6-dioxopiperidin-3-yl)-3-fluorobenzyl)piperazin-1-yl)ethyl)-4,9-dioxo-4,9-dihydronaphtho[2,3-b]furan-2-carboxamide;
N-(2-(4-(3-(2,6-dioxopiperidin-3-yl)benzyl)piperazin-1-yl)ethyl)-4,9-dioxo-4,9-dihydronaphtho[2,3-b]furan-2-carboxamide;
N-(2-(4-(2-(2,6-dioxopiperidin-3-yl)benzyl)piperazin-1-yl)ethyl)-4,9-dioxo-4,9-dihydronaphtho[2,3-b]furan-2-carboxamide;
N-(2-(4-(4-(2,4-dioxotetrahydropyrimidin-1(2H)-yl)benzyl)piperazin-1-yl)ethyl)-4,9-dioxo-4,9-dihydronaphtho[2,3-b]furan-2-carboxamide;
N-(2-(4-(4-(2,4-dioxotetrahydropyrimidin-1(2H)-yl)-2-fluorobenzyl)piperazin-1-yl)ethyl)-4,9-dioxo-4,9-dihydronaphtho[2,3-b]furan-2-carboxamide;
N-(2-(4-(3-(2,4-dioxotetrahydropyrimidin-1(2H)-yl)benzyl)piperazin-1-yl)ethyl)-4,9-dioxo-4,9-dihydronaphtho[2,3-b]furan-2-carboxamide;
N-(2-(4-(2-(2,4-dioxotetrahydropyrimidin-1(2H)-yl)benzyl)piperazin-1-yl)ethyl)-4,9-dioxo-4,9-dihydronaphtho[2,3-b]furan-2-carboxamide;
N-(2-(4-((5-(2,4-dioxotetrahydropyrimidin-1(2H)-yl)pyridin-2-yl)methyl)piperazin-1-yl)ethyl)-4,9-dioxo-4,9-dihydronaphtho[2,3-b]furan-2-carboxamide;
N-(2-(4-((3-(2,4-dioxotetrahydropyrimidin-1(2H)-yl)pyridin-4-yl)methyl)piperazin-1-yl)ethyl)-4,9-dioxo-4,9-dihydronaphtho[2,3-b]furan-2-carboxamide;
N-(2-(4-((6-(2,4-dioxotetrahydropyrimidin-1(2H)-yl)pyridazin-3-yl)methyl)piperazin-1-yl)ethyl)-4,9-dioxo-4,9-dihydronaphtho[2,3-b]furan-2-carboxamide;
N-(2-(4-(4-((2,6-dioxopiperidin-3-yl)amino)benzyl)piperazin-1-yl)ethyl)-4,9-dioxo-4,9-dihydronaphtho[2,3-b]furan-2-carboxamide;
N-(2-(4-(3-((2,6-dioxopiperidin-3-yl)amino)benzyl)piperazin-1-yl)ethyl)-4,9-dioxo-4,9-dihydronaphtho[2,3-b]furan-2-carboxamide;
N-(2-(4-(2-((2,6-dioxopiperidin-3-yl)amino)benzyl)piperazin-1-yl)ethyl)-4,9-dioxo-4,9-dihydronaphtho[2,3-b]furan-2-carboxamide;
N-(4-(2,6-dioxopiperidin-3-yl)benzyl)-4,9-dioxo-4,9-dihydronaphtho[2,3-b]furan-2-carboxamide;
N-(4-(2,6-dioxopiperidin-3-yl)-3-fluorobenzyl)-4,9-dioxo-4,9-dihydronaphtho[2,3-b]furan-2-carboxamide;
N-(3-(2,6-dioxopiperidin-3-yl)benzyl)-4,9-dioxo-4,9-dihydronaphtho[2,3-b]furan-2-carboxamide;
N-(2-(2,6-dioxopiperidin-3-yl)benzyl)-4,9-dioxo-4,9-dihydronaphtho[2,3-b]furan-2-carboxamide;
N-(4-(2,4-dioxotetrahydropyrimidin-1(2H)-yl)benzyl)-4,9-dioxo-4,9-dihydronaphtho[2,3-b]furan-2-carboxamide;
N-(4-(2,4-dioxotetrahydropyrimidin-1(2H)-yl)-2-fluorobenzyl)-4,9-dioxo-4,9-dihydronaphtho[2,3-b]furan-2-carboxamide;
N-(3-(2,4-dioxotetrahydropyrimidin-1(2H)-yl)benzyl)-4,9-dioxo-4,9-dihydronaphtho[2,3-b]furan-2-carboxamide;
N-(2-(2,4-dioxotetrahydropyrimidin-1(2H)-yl)benzyl)-4,9-dioxo-4,9-dihydronaphtho[2,3-b]furan-2-carboxamide;
N-((5-(2,4-dioxotetrahydropyrimidin-1(2H)-yl)pyridin-2-yl)methyl)-4,9-dioxo-4,9-dihydronaphtho[2,3-b]furan-2-carboxamide;
N-((3-(2,4-dioxotetrahydropyrimidin-1(2H)-yl)pyridin-4-yl)methyl)-4,9-dioxo-4,9-dihydronaphtho[2,3-b]furan-2-carboxamide;
N-((6-(2,4-dioxotetrahydropyrimidin-1(2H)-yl)pyridazin-3-yl)methyl)-4,9-dioxo-4,9-dihydronaphtho[2,3-b]furan-2-carboxamide;
N-(4-((2,6-dioxopiperidin-3-yl)amino)benzyl)-4,9-dioxo-4,9-dihydronaphtho[2,3-b]furan-2-carboxamide;
N-(3-((2,6-dioxopiperidin-3-yl)amino)benzyl)-4,9-dioxo-4,9-dihydronaphtho[2,3-b]furan-2-carboxamide;
N-(2-((2,6-dioxopiperidin-3-yl)amino)benzyl)-4,9-dioxo-4,9-dihydronaphtho[2,3-b]furan-2-carboxamide;
((2-(((5S,8S,10aR)-8-(((2S)-5-amino-1-(2-chloro-3-(4-(2,6-dioxopiperidin-3-yl)benzyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(4-(2,6-dioxopiperidin-3-yl)-3-fluorobenzyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(3-(2,6-dioxopiperidin-3-yl)benzyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(2-(2,6-dioxopiperidin-3-yl)benzyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(2-chloro-3-(4-(2,4-dioxotetrahydropyrimidin-1(2H)-yl)benzyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(2-chloro-3-(4-(2,4-dioxotetrahydropyrimidin-1(2H)-yl)-2-fluorobenzyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(2-chloro-3-(3-(2,4-dioxotetrahydropyrimidin-1(2H)-yl)benzyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(2-chloro-3-(2-(2,4-dioxotetrahydropyrimidin-1(2H)-yl)benzyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(2-chloro-3-((5-(2,4-dioxotetrahydropyrimidin-1(2H)-yl)pyridin-2-yl)methyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(2-chloro-3-((3-(2,4-dioxotetrahydropyrimidin-1(2H)-yl)pyridin-4-yl)methyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(2-chloro-3-((6-(2,4-dioxotetrahydropyrimidin-1(2H)-yl)pyridazin-3-yl)methyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(4-((2,6-dioxopiperidin-3-yl)amino)benzyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(3-((2,6-dioxopiperidin-3-yl)amino)benzyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(2-((2,6-dioxopiperidin-3-yl)amino)benzyl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(4-(4-(2,6-dioxopiperidin-3-yl)benzyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(4-(4-(2,6-dioxopiperidin-3-yl)-3-fluorobenzyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(4-(3-(2,6-dioxopiperidin-3-yl)benzyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(4-(2-(2,6-dioxopiperidin-3-yl)benzyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(2-chloro-3-(4-(4-(2,4-dioxotetrahydropyrimidin-1(2H)-yl)benzyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(2-chloro-3-(4-(4-(2,4-dioxotetrahydropyrimidin-1(2H)-yl)-2-fluorobenzyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(2-chloro-3-(4-(3-(2,4-dioxotetrahydropyrimidin-1(2H)-yl)benzyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(2-chloro-3-(4-(2-(2,4-dioxotetrahydropyrimidin-1(2H)-yl)benzyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(2-chloro-3-(4-((5-(2,4-dioxotetrahydropyrimidin-1(2H)-yl)pyridin-2-yl)methyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(2-chloro-3-(4-((3-(2,4-dioxotetrahydropyrimidin-1(2H)-yl)pyridin-4-yl)methyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(2-chloro-3-(4-((6-(2,4-dioxotetrahydropyrimidin-1(2H)-yl)pyridazin-3-yl)methyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(4-(4-((2,6-dioxopiperidin-3-yl)amino)benzyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(4-(3-((2,6-dioxopiperidin-3-yl)amino)benzyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((2S)-5-amino-1-(2-chloro-3-(4-(2-((2,6-dioxopiperidin-3-yl)amino)benzyl)piperazin-1-yl)phenoxy)-5-oxopentan-2-yl)carbamoyl)-3-methyl-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-(4-(2,6-dioxopiperidin-3-yl)benzyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-(4-(2,6-dioxopiperidin-3-yl)-3-fluorobenzyl)piperidin-4-yl)methyl)-6-oxodecahydropyrrolo[1,2-a] [1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-(3-(2,6-dioxopiperidin-3-yl)benzyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-(2-(2,6-dioxopiperidin-3-yl)benzyl)piperidin-4-yl)methyl)-6-oxodecahydropyrrolo[1,2-a] [1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-(4-(2,4-dioxotetrahydropyrimidin-1(2H)-yl)benzyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-(4-(2,4-dioxotetrahydropyrimidin-1(2H)-yl)-2-fluorobenzyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-(3-(2,4-dioxotetrahydropyrimidin-1(2H)-yl)benzyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-((5-(2,4-dioxotetrahydropyrimidin-1(2H)-yl)pyridin-2-yl)methyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-((3-(2,4-dioxotetrahydropyrimidin-1(2H)-yl)pyridin-4-yl)methyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-((6-(2,4-dioxotetrahydropyrimidin-1(2H)-yl)pyridazin-3-yl)methyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-(4-((2,6-dioxopiperidin-3-yl)amino)benzyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-(3-((2,6-dioxopiperidin-3-yl)amino)benzyl)piperidin-4-yl)methyl)-6-oxodecahydropyrrolo[1,2-a][1,5]diazocin-5-yl)carbamoyl)-1H-indol-5-yl)difluoromethyl)phosphonic acid;
((2-(((5S,8S,10aR)-8-(((S)-5-amino-1-(benzhydrylamino)-1,5-dioxopentan-2-yl)carbamoyl)-3-((1-(2-((2,6-dioxopiperidin-3-yl)amino)benzyl)piperidin-4-yl)methyl)-6-oxodecahydropyrrolo[1,2-a][1,5]diazocin-5-yl)carbamoyl)-1H-indol-5-yl)difluoromethyl)phosphonic acid;
6-(1-(4-(2,6-dioxopiperidin-3-yl)benzyl)piperidin-4-yl)-2-(4-phenoxyphenyl)nicotinamide;
6-(1-(4-(2,6-dioxopiperidin-3-yl)-3-fluorobenzyl)piperidin-4-yl)-2-(4-phenoxyphenyl)nicotinamide;
6-(1-(3-(2,6-dioxopiperidin-3-yl)benzyl)piperidin-4-yl)-2-(4-phenoxyphenyl)nicotinamide;
6-(1-(2-(2,6-dioxopiperidin-3-yl)benzyl)piperidin-4-yl)-2-(4-phenoxyphenyl)nicotinamide;
6-(1-(4-(2,4-dioxotetrahydropyrimidin-1(2H)-yl)benzyl)piperidin-4-yl)-2-(4-phenoxyphenyl)nicotinamide;
6-(1-(4-(2,4-dioxotetrahydropyrimidin-1(2H)-yl)-2-fluorobenzyl)piperidin-4-yl)-2-(4-phenoxyphenyl)nicotinamide;
6-(1-(3-(2,4-dioxotetrahydropyrimidin-1(2H)-yl)benzyl)piperidin-4-yl)-2-(4-phenoxyphenyl)nicotinamide;
6-(1-(2-(2,4-dioxotetrahydropyrimidin-1(2H)-yl)benzyl)piperidin-4-yl)-2-(4-phenoxyphenyl)nicotinamide;
6-(1-((5-(2,4-dioxotetrahydropyrimidin-1(2H)-yl)pyridin-2-yl)methyl)piperidin-4-yl)-2-(4-phenoxyphenyl)nicotinamide;
6-(1-((3-(2,4-dioxotetrahydropyrimidin-1(2H)-yl)pyridin-4-yl)methyl)piperidin-4-yl)-2-(4-phenoxyphenyl)nicotinamide;
6-(1-((6-(2,4-dioxotetrahydropyrimidin-1(2H)-yl)pyridazin-3-yl)methyl)piperidin-4-yl)-2-(4-phenoxyphenyl)nicotinamide;
6-(1-(4-((2,6-dioxopiperidin-3-yl)amino)benzyl)piperidin-4-yl)-2-(4-phenoxyphenyl)nicotinamide;
6-(1-(3-((2,6-dioxopiperidin-3-yl)amino)benzyl)piperidin-4-yl)-2-(4-phenoxyphenyl)nicotinamide;
6-(1-(2-((2,6-dioxopiperidin-3-yl)amino)benzyl)piperidin-4-yl)-2-(4-phenoxyphenyl)nicotinamide;
6-(4-(4-(2,6-dioxopiperidin-3-yl)benzyl)piperazin-1-yl)-2-(4-phenoxyphenyl)nicotinamide;
6-(4-(4-(2,6-dioxopiperidin-3-yl)-3-fluorobenzyl)piperazin-1-yl)-2-(4-phenoxyphenyl)nicotinamide;
6-(4-(3-(2,6-dioxopiperidin-3-yl)benzyl)piperazin-1-yl)-2-(4-phenoxyphenyl)nicotinamide;
6-(4-(2-(2,6-dioxopiperidin-3-yl)benzyl)piperazin-1-yl)-2-(4-phenoxyphenyl)nicotinamide;
6-(4-(4-(2,4-dioxotetrahydropyrimidin-1(2H)-yl)benzyl)piperazin-1-yl)-2-(4-phenoxyphenyl)nicotinamide;
6-(4-(4-(2,4-dioxotetrahydropyrimidin-1(2H)-yl)-2-fluorobenzyl)piperazin-1-yl)-2-(4-phenoxyphenyl)nicotinamide;
6-(4-(3-(2,4-dioxotetrahydropyrimidin-1(2H)-yl)benzyl)piperazin-1-yl)-2-(4-phenoxyphenyl)nicotinamide;
6-(4-(2-(2,4-dioxotetrahydropyrimidin-1(2H)-yl)benzyl)piperazin-1-yl)-2-(4-phenoxyphenyl)nicotinamide;
6-(4-((5-(2,4-dioxotetrahydropyrimidin-1(2H)-yl)pyridin-2-yl)methyl)piperazin-1-yl)-2-(4-phenoxyphenyl)nicotinamide;
6-(4-((3-(2,4-dioxotetrahydropyrimidin-1(2H)-yl)pyridin-4-yl)methyl)piperazin-1-yl)-2-(4-phenoxyphenyl)nicotinamide;
6-(4-((6-(2,4-dioxotetrahydropyrimidin-1(2H)-yl)pyridazin-3-yl)methyl)piperazin-1-yl)-2-(4-phenoxyphenyl)nicotinamide;
6-(4-(4-((2,6-dioxopiperidin-3-yl)amino)benzyl)piperazin-1-yl)-2-(4-phenoxyphenyl)nicotinamide;
6-(4-(3-((2,6-dioxopiperidin-3-yl)amino)benzyl)piperazin-1-yl)-2-(4-phenoxyphenyl)nicotinamide;
6-(4-(2-((2,6-dioxopiperidin-3-yl)amino)benzyl)piperazin-1-yl)-2-(4-phenoxyphenyl)nicotinamide;
6-(4-((4-(2,6-dioxopiperidin-3-yl)benzyl)(methyl)amino)piperidin-1-yl)-2-(4-phenoxyphenyl)nicotinamide;
6-(4-((4-(2,6-dioxopiperidin-3-yl)-3-fluorobenzyl)(methyl)amino)piperidin-1-yl)-2-(4-phenoxyphenyl)nicotinamide;
6-(4-((3-(2,6-dioxopiperidin-3-yl)benzyl)(methyl)amino)piperidin-1-yl)-2-(4-phenoxyphenyl)nicotinamide;
6-(4-((2-(2,6-dioxopiperidin-3-yl)benzyl)(methyl)amino)piperidin-1-yl)-2-(4-phenoxyphenyl)nicotinamide;
6-(4-((4-(2,4-dioxotetrahydropyrimidin-1(2H)-yl)benzyl)(methyl)amino)piperidin-1-yl)-2-(4-phenoxyphenyl)nicotinamide;
6-(4-((4-(2,4-dioxotetrahydropyrimidin-1(2H)-yl)-2-fluorobenzyl)(methyl)amino)piperidin-1-yl)-2-(4-phenoxyphenyl)nicotinamide;
6-(4-((3-(2,4-dioxotetrahydropyrimidin-1(2H)-yl)benzyl)(methyl)amino)piperidin-1-yl)-2-(4-phenoxyphenyl)nicotinamide;
6-(4-((2-(2,4-dioxotetrahydropyrimidin-1(2H)-yl)benzyl)(methyl)amino)piperidin-1-yl)-2-(4-phenoxyphenyl)nicotinamide;
6-(4-(((5-(2,4-dioxotetrahydropyrimidin-1(2H)-yl)pyridin-2-yl)methyl)(methyl)amino)piperidin-1-yl)-2-(4-phenoxyphenyl)nicotinamide;
6-(4-(((6-(2,4-dioxotetrahydropyrimidin-1(2H)-yl)pyridazin-3-yl)methyl)(methyl)amino)piperidin-1-yl)-2-(4-phenoxyphenyl)nicotinamide;
6-(4-(((3-(2,4-dioxotetrahydropyrimidin-1(2H)-yl)pyridin-4-yl)methyl)(methyl)amino)piperidin-1-yl)-2-(4-phenoxyphenyl)nicotinamide;
6-(4-((4-((2,6-dioxopiperidin-3-yl)amino)benzyl)(methyl)amino)piperidin-1-yl)-2-(4-phenoxyphenyl)nicotinamide;
6-(4-((3-((2,6-dioxopiperidin-3-yl)amino)benzyl)(methyl)amino)piperidin-1-yl)-2-(4-phenoxyphenyl)nicotinamide;
6-(4-((2-((2,6-dioxopiperidin-3-yl)amino)benzyl)(methyl)amino)piperidin-1-yl)-2-(4-phenoxyphenyl)nicotinamide;
6-(4-(4-(4-(2,6-dioxopiperidin-3-yl)benzyl)piperazin-1-yl)piperidin-1-yl)-2-(4-phenoxyphenyl)nicotinamide;
6-(4-(4-(4-(2,6-dioxopiperidin-3-yl)-3-fluorobenzyl)piperazin-1-yl)piperidin-1-yl)-2-(4-phenoxyphenyl)nicotinamide;
6-(4-(4-(3-(2,6-dioxopiperidin-3-yl)benzyl)piperazin-1-yl)piperidin-1-yl)-2-(4-phenoxyphenyl)nicotinamide;
6-(4-(4-(2-(2,6-dioxopiperidin-3-yl)benzyl)piperazin-1-yl)piperidin-1-yl)-2-(4-phenoxyphenyl)nicotinamide;
6-(4-(4-(4-(2,4-dioxotetrahydropyrimidin-1(2H)-yl)benzyl)piperazin-1-yl)piperidin-1-yl)-2-(4-phenoxyphenyl)nicotinamide;
6-(4-(4-(4-(2,4-dioxotetrahydropyrimidin-1(2H)-yl)-2-fluorobenzyl)piperazin-1-yl)piperidin-1-yl)-2-(4-phenoxyphenyl)nicotinamide;
6-(4-(4-(3-(2,4-dioxotetrahydropyrimidin-1(2H)-yl)benzyl)piperazin-1-yl)piperidin-1-yl)-2-(4-phenoxyphenyl)nicotinamide;
6-(4-(4-(2-(2,4-dioxotetrahydropyrimidin-1(2H)-yl)benzyl)piperazin-1-yl)piperidin-1-yl)-2-(4-phenoxyphenyl)nicotinamide;
6-(4-(4-((S-(2,4-dioxotetrahydropyrimidin-1(2H)-yl)pyridin-2-yl)methyl)piperazin-1-yl)piperidin-1-yl)-2-(4-phenoxyphenyl)nicotinamide;
6-(4-(4-((6-(2,4-dioxotetrahydropyrimidin-1(2H)-yl)pyridazin-3-yl)methyl)piperazin-1-yl)piperidin-1-yl)-2-(4-phenoxyphenyl)nicotinamide;
6-(4-(4-((3-(2,4-dioxotetrahydropyrimidin-1(2H)-yl)pyridin-4-yl)methyl)piperazin-1-yl)piperidin-1-yl)-2-(4-phenoxyphenyl)nicotinamide;
6-(4-(4-(4-((2,6-dioxopiperidin-3-yl)amino)benzyl)piperazin-1-yl)piperidin-1-yl)-2-(4-phenoxyphenyl)nicotinamide;
6-(4-(4-(3-((2,6-dioxopiperidin-3-yl)amino)benzyl)piperazin-1-yl)piperidin-1-yl)-2-(4-phenoxyphenyl)nicotinamide;
6-(4-(4-(2-((2,6-dioxopiperidin-3-yl)amino)benzyl)piperazin-1-yl)piperidin-1-yl)-2-(4-phenoxyphenyl)nicotinamide;
3-(4-((4-(4-amino-3-(4-phenoxyphenyl)-1H-pyrazolo[3,4-d]pyrimidin-1-yl)piperidin-1-yl)methyl)phenyl)piperidine-2,6-dione;
3-(4-((4-(4-amino-3-(4-phenoxyphenyl)-1H-pyrazolo[3,4-d]pyrimidin-1-yl)piperidin-1-yl)methyl)-3-fluorophenyl)piperidine-2,6-dione;
3-(4-((4-(4-amino-3-(4-phenoxyphenyl)-1H-pyrazolo[3,4-d]pyrimidin-1-yl)piperidin-1-yl)methyl)-2-fluorophenyl)piperidine-2,6-dione;
3-(2-((4-(4-amino-3-(4-phenoxyphenyl)-1H-pyrazolo[3,4-d]pyrimidin-1-yl)piperidin-1-yl)methyl)phenyl)piperidine-2,6-dione;
3-(2-((4-(4-amino-3-(4-phenoxyphenyl)-1H-pyrazolo[3,4-d]pyrimidin-1-yl)piperidin-1-yl)methyl)-3-fluorophenyl)piperidine-2,6-dione;
3-(2-((4-(4-amino-3-(4-phenoxyphenyl)-1H-pyrazolo[3,4-d]pyrimidin-1-yl)piperidin-1-yl)methyl)-4-fluorophenyl)piperidine-2,6-dione;
3-(2-((4-(4-amino-3-(4-phenoxyphenyl)-1H-pyrazolo[3,4-d]pyrimidin-1-yl)piperidin-1-yl)methyl)-5-fluorophenyl)piperidine-2,6-dione;
3-(2-((4-(4-amino-3-(4-phenoxyphenyl)-1H-pyrazolo[3,4-d]pyrimidin-1-yl)piperidin-1-yl)methyl)-6-fluorophenyl)piperidine-2,6-dione;
3-(3-((4-(4-amino-3-(4-phenoxyphenyl)-1H-pyrazolo[3,4-d]pyrimidin-1-yl)piperidin-1-yl)methyl)phenyl)piperidine-2,6-dione;
3-(3-((4-(4-amino-3-(4-phenoxyphenyl)-1H-pyrazolo[3,4-d]pyrimidin-1-yl)piperidin-1-yl)methyl)-2-fluorophenyl)piperidine-2,6-dione;
3-(3-((4-(4-amino-3-(4-phenoxyphenyl)-1H-pyrazolo[3,4-d]pyrimidin-1-yl)piperidin-1-yl)methyl)-4-fluorophenyl)piperidine-2,6-dione;
3-(3-((4-(4-amino-3-(4-phenoxyphenyl)-1H-pyrazolo[3,4-d]pyrimidin-1-yl)piperidin-1-yl)methyl)-5-fluorophenyl)piperidine-2,6-dione;
3-(5-((4-(4-amino-3-(4-phenoxyphenyl)-1H-pyrazolo[3,4-d]pyrimidin-1-yl)piperidin-1-yl)methyl)-2-fluorophenyl)piperidine-2,6-dione;
1-(4-((4-(4-amino-3-(4-phenoxyphenyl)-1H-pyrazolo[3,4-d]pyrimidin-1-yl)piperidin-1-yl)methyl)phenyl)dihydropyrimidine-2,4(1H,3H)-dione;
1-(4-((4-(4-amino-3-(4-phenoxyphenyl)-1H-pyrazolo[3,4-d]pyrimidin-1-yl)piperidin-1-yl)methyl)-3-fluorophenyl)dihydropyrimidine-2,4(1H,3H)-dione;
1-(4-((4-(4-amino-3-(4-phenoxyphenyl)-1H-pyrazolo[3,4-d]pyrimidin-1-yl)piperidin-1-yl)methyl)-2-fluorophenyl)dihydropyrimidine-2,4(1H,3H)-dione;
1-(2-((4-(4-amino-3-(4-phenoxyphenyl)-1H-pyrazolo[3,4-d]pyrimidin-1-yl)piperidin-1-yl)methyl)phenyl)dihydropyrimidine-2,4(1H,3H)-dione;
1-(2-((4-(4-amino-3-(4-phenoxyphenyl)-1H-pyrazolo[3,4-d]pyrimidin-1-yl)piperidin-1-yl)methyl)-3-fluorophenyl)dihydropyrimidine-2,4(1H,3H)-dione;
1-(2-((4-(4-amino-3-(4-phenoxyphenyl)-1H-pyrazolo[3,4-d]pyrimidin-1-yl)piperidin-1-yl)methyl)-4-fluorophenyl)dihydropyrimidine-2,4(1H,3H)-dione;
1-(2-((4-(4-amino-3-(4-phenoxyphenyl)-1H-pyrazolo[3,4-d]pyrimidin-1-yl)piperidin-1-yl)methyl)-5-fluorophenyl)dihydropyrimidine-2,4(1H,3H)-dione;
1-(2-((4-(4-amino-3-(4-phenoxyphenyl)-1H-pyrazolo[3,4-d]pyrimidin-1-yl)piperidin-1-yl)methyl)-6-fluorophenyl)dihydropyrimidine-2,4(1H,3H)-dione;
1-(3-((4-(4-amino-3-(4-phenoxyphenyl)-1H-pyrazolo[3,4-d]pyrimidin-1-yl)piperidin-1-yl)methyl)phenyl)dihydropyrimidine-2,4(1H,3H)-dione;
1-(3-((4-(4-amino-3-(4-phenoxyphenyl)-1H-pyrazolo[3,4-d]pyrimidin-1-yl)piperidin-1-yl)methyl)-2-fluorophenyl)dihydropyrimidine-2,4(1H,3H)-dione;
1-(3-((4-(4-amino-3-(4-phenoxyphenyl)-1H-pyrazolo[3,4-d]pyrimidin-1-yl)piperidin-1-yl)methyl)-4-fluorophenyl)dihydropyrimidine-2,4(1H,3H)-dione;
1-(3-((4-(4-amino-3-(4-phenoxyphenyl)-1H-pyrazolo[3,4-d]pyrimidin-1-yl)piperidin-1-yl)methyl)-5-fluorophenyl)dihydropyrimidine-2,4(1H,3H)-dione;
1-(5-((4-(4-amino-3-(4-phenoxyphenyl)-1H-pyrazolo[3,4-d]pyrimidin-1-yl)piperidin-1-yl)methyl)-2-fluorophenyl)dihydropyrimidine-2,4(1H,3H)-dione;
3-((4-((4-(4-amino-3-(4-phenoxyphenyl)-1H-pyrazolo[3,4-d]pyrimidin-1-yl)piperidin-1-yl)methyl)phenyl)amino)piperidine-2,6-dione;
3-((4-((4-(4-amino-3-(4-phenoxyphenyl)-1H-pyrazolo[3,4-d]pyrimidin-1-yl)piperidin-1-yl)methyl)-3-fluorophenyl)amino)piperidine-2,6-dione;
3-((4-((4-(4-amino-3-(4-phenoxyphenyl)-1H-pyrazolo[3,4-d]pyrimidin-1-yl)piperidin-1-yl)methyl)-2-fluorophenyl)amino)piperidine-2,6-dione;
3-((2-((4-(4-amino-3-(4-phenoxyphenyl)-1H-pyrazolo[3,4-d]pyrimidin-1-yl)piperidin-1-yl)methyl)phenyl)amino)piperidine-2,6-dione;
3-((2-((4-(4-amino-3-(4-phenoxyphenyl)-1H-pyrazolo[3,4-d]pyrimidin-1-yl)piperidin-1-yl)methyl)-3-fluorophenyl)amino)piperidine-2,6-dione;
3-((2-((4-(4-amino-3-(4-phenoxyphenyl)-1H-pyrazolo[3,4-d]pyrimidin-1-yl)piperidin-1-yl)methyl)-5-fluorophenyl)amino)piperidine-2,6-dione;
3-((2-((4-(4-amino-3-(4-phenoxyphenyl)-1H-pyrazolo[3,4-d]pyrimidin-1-yl)piperidin-1-yl)methyl)-6-fluorophenyl)amino)piperidine-2,6-dione;
3-((2-((4-(4-amino-3-(4-phenoxyphenyl)-1H-pyrazolo[3,4-d]pyrimidin-1-yl)piperidin-1-yl)methyl)-4-fluorophenyl)amino)piperidine-2,6-dione;
3-((3-((4-(4-amino-3-(4-phenoxyphenyl)-1H-pyrazolo[3,4-d]pyrimidin-1-yl)piperidin-1-yl)methyl)phenyl)amino)piperidine-2,6-dione;
3-((3-((4-(4-amino-3-(4-phenoxyphenyl)-1H-pyrazolo[3,4-d]pyrimidin-1-yl)piperidin-1-yl)methyl)-4-fluorophenyl)amino)piperidine-2,6-dione;
3-((3-((4-(4-amino-3-(4-phenoxyphenyl)-1H-pyrazolo[3,4-d]pyrimidin-1-yl)piperidin-1-yl)methyl)-5-fluorophenyl)amino)piperidine-2,6-dione;
3-((5-((4-(4-amino-3-(4-phenoxyphenyl)-1H-pyrazolo[3,4-d]pyrimidin-1-yl)piperidin-1-yl)methyl)-2-fluorophenyl)amino)piperidine-2,6-dione;
3-((3-((4-(4-amino-3-(4-phenoxyphenyl)-1H-pyrazolo[3,4-d]pyrimidin-1-yl)piperidin-1-yl)methyl)-2-fluorophenyl)amino)piperidine-2,6-dione;
3-(6-((4-(4-amino-3-(4-phenoxyphenyl)-1H-pyrazolo[3,4-d]pyrimidin-1-yl)piperidin-1-yl)methyl)pyridin-3-yl)piperidine-2,6-dione;
3-(6-((4-(4-amino-3-(4-phenoxyphenyl)-1H-pyrazolo[3,4-d]pyrimidin-1-yl)piperidin-1-yl)methyl)-5-fluoropyridin-3-yl)piperidine-2,6-dione;
3-(6-((4-(4-amino-3-(4-phenoxyphenyl)-1H-pyrazolo[3,4-d]pyrimidin-1-yl)piperidin-1-yl)methyl)-4-fluoropyridin-3-yl)piperidine-2,6-dione;
3-(6-((4-(4-amino-3-(4-phenoxyphenyl)-1H-pyrazolo[3,4-d]pyrimidin-1-yl)piperidin-1-yl)methyl)-2-fluoropyridin-3-yl)piperidine-2,6-dione;
3-(5-((4-(4-amino-3-(4-phenoxyphenyl)-1H-pyrazolo[3,4-d]pyrimidin-1-yl)piperidin-1-yl)methyl)pyridin-2-yl)piperidine-2,6-dione;
3-(5-((4-(4-amino-3-(4-phenoxyphenyl)-1H-pyrazolo[3,4-d]pyrimidin-1-yl)piperidin-1-yl)methyl)-4-fluoropyridin-2-yl)piperidine-2,6-dione;
3-(5-((4-(4-amino-3-(4-phenoxyphenyl)-1H-pyrazolo[3,4-d]pyrimidin-1-yl)piperidin-1-yl)methyl)-3-fluoropyridin-2-yl)piperidine-2,6-dione;
3-(5-((4-(4-amino-3-(4-phenoxyphenyl)-1H-pyrazolo[3,4-d]pyrimidin-1-yl)piperidin-1-yl)methyl)-6-fluoropyridin-2-yl)piperidine-2,6-dione;
3-(3-((4-(4-amino-3-(4-phenoxyphenyl)-1H-pyrazolo[3,4-d]pyrimidin-1-yl)piperidin-1-yl)methyl)pyridin-2-yl)piperidine-2,6-dione;
3-(3-((4-(4-amino-3-(4-phenoxyphenyl)-1H-pyrazolo[3,4-d]pyrimidin-1-yl)piperidin-1-yl)methyl)-4-fluoropyridin-2-yl)piperidine-2,6-dione;
3-(3-((4-(4-amino-3-(4-phenoxyphenyl)-1H-pyrazolo[3,4-d]pyrimidin-1-yl)piperidin-1-yl)methyl)-5-fluoropyridin-2-yl)piperidine-2,6-dione;
3-(3-((4-(4-amino-3-(4-phenoxyphenyl)-1H-pyrazolo[3,4-d]pyrimidin-1-yl)piperidin-1-yl)methyl)-6-fluoropyridin-2-yl)piperidine-2,6-dione;
3-(5-((4-(4-amino-3-(4-phenoxyphenyl)-1H-pyrazolo[3,4-d]pyrimidin-1-yl)piperidin-1-yl)methyl)pyridin-3-yl)piperidine-2,6-dione;
3-(5-((4-(4-amino-3-(4-phenoxyphenyl)-1H-pyrazolo[3,4-d]pyrimidin-1-yl)piperidin-1-yl)methyl)-4-fluoropyridin-3-yl)piperidine-2,6-dione;
3-(5-((4-(4-amino-3-(4-phenoxyphenyl)-1H-pyrazolo[3,4-d]pyrimidin-1-yl)piperidin-1-yl)methyl)-6-fluoropyridin-3-yl)piperidine-2,6-dione;
3-(5-((4-(4-amino-3-(4-phenoxyphenyl)-1H-pyrazolo[3,4-d]pyrimidin-1-yl)piperidin-1-yl)methyl)-2-fluoropyridin-3-yl)piperidine-2,6-dione;
3-(4-((4-(4-amino-3-(4-phenoxyphenyl)-1H-pyrazolo[3,4-d]pyrimidin-1-yl)piperidin-1-yl)methyl)pyridin-3-yl)piperidine-2,6-dione;
3-(4-((4-(4-amino-3-(4-phenoxyphenyl)-1H-pyrazolo[3,4-d]pyrimidin-1-yl)piperidin-1-yl)methyl)-5-fluoropyridin-3-yl)piperidine-2,6-dione;
3-(4-((4-(4-amino-3-(4-phenoxyphenyl)-1H-pyrazolo[3,4-d]pyrimidin-1-yl)piperidin-1-yl)methyl)-6-fluoropyridin-3-yl)piperidine-2,6-dione;
3-(4-((4-(4-amino-3-(4-phenoxyphenyl)-1H-pyrazolo[3,4-d]pyrimidin-1-yl)piperidin-1-yl)methyl)-2-fluoropyridin-3-yl)piperidine-2,6-dione;
3-(4-((4-(4-amino-3-(4-phenoxyphenyl)-1H-pyrazolo[3,4-d]pyrimidin-1-yl)piperidin-1-yl)methyl)pyridin-2-yl)piperidine-2,6-dione;
3-(4-((4-(4-amino-3-(4-phenoxyphenyl)-1H-pyrazolo[3,4-d]pyrimidin-1-yl)piperidin-1-yl)methyl)-3-fluoropyridin-2-yl)piperidine-2,6-dione;
3-(4-((4-(4-amino-3-(4-phenoxyphenyl)-1H-pyrazolo[3,4-d]pyrimidin-1-yl)piperidin-1-yl)methyl)-5-fluoropyridin-2-yl)piperidine-2,6-dione;
3-(4-((4-(4-amino-3-(4-phenoxyphenyl)-1H-pyrazolo[3,4-d]pyrimidin-1-yl)piperidin-1-yl)methyl)-6-fluoropyridin-2-yl)piperidine-2,6-dione;
3-(6-((4-(4-amino-3-(4-phenoxyphenyl)-1H-pyrazolo[3,4-d]pyrimidin-1-yl)piperidin-1-yl)methyl)pyridazin-3-yl)piperidine-2,6-dione;
3-(6-((4-(4-amino-3-(4-phenoxyphenyl)-1H-pyrazolo[3,4-d]pyrimidin-1-yl)piperidin-1-yl)methyl)-5-fluoropyridazin-3-yl)piperidine-2,6-dione;
3-(6-((4-(4-amino-3-(4-phenoxyphenyl)-1H-pyrazolo[3,4-d]pyrimidin-1-yl)piperidin-1-yl)methyl)-4-fluoropyridazin-3-yl)piperidine-2,6-dione;
3-(6-((4-(4-amino-3-(4-phenoxyphenyl)-1H-pyrazolo[3,4-d]pyrimidin-1-yl)piperidin-1-yl)methyl)pyridazin-4-yl)piperidine-2,6-dione;
3-(6-((4-(4-amino-3-(4-phenoxyphenyl)-1H-pyrazolo[3,4-d]pyrimidin-1-yl)piperidin-1-yl)methyl)-5-fluoropyridazin-4-yl)piperidine-2,6-dione;
3-(6-((4-(4-amino-3-(4-phenoxyphenyl)-1H-pyrazolo[3,4-d]pyrimidin-1-yl)piperidin-1-yl)methyl)-3-fluoropyridazin-4-yl)piperidine-2,6-dione;
3-(6-((4-(4-amino-3-(4-phenoxyphenyl)-1H-pyrazolo[3,4-d]pyrimidin-1-yl)piperidin-1-yl)methyl)pyrimidin-4-yl)piperidine-2,6-dione;
3-(5-((4-(4-amino-3-(4-phenoxyphenyl)-1H-pyrazolo[3,4-d]pyrimidin-1-yl)piperidin-1-yl)methyl)pyrazin-2-yl)piperidine-2,6-dione;
3-(6-((4-(4-amino-3-(4-phenoxyphenyl)-1H-pyrazolo[3,4-d]pyrimidin-1-yl)piperidin-1-yl)methyl)pyrazin-2-yl)piperidine-2,6-dione;
3-(4-((4-(4-amino-3-(4-phenoxyphenyl)-1H-pyrazolo[3,4-d]pyrimidin-1-yl)piperidin-1-yl)methyl)pyridazin-3-yl)piperidine-2,6-dione;
3-(5-((4-(4-amino-3-(4-phenoxyphenyl)-1H-pyrazolo[3,4-d]pyrimidin-1-yl)piperidin-1-yl)methyl)pyridazin-3-yl)piperidine-2,6-dione;
3-(5-((4-(4-amino-3-(4-phenoxyphenyl)-1H-pyrazolo[3,4-d]pyrimidin-1-yl)piperidin-1-yl)methyl)pyrimidin-2-yl)piperidine-2,6-dione;
3-(5-((4-(4-amino-3-(4-phenoxyphenyl)-1H-pyrazolo[3,4-d]pyrimidin-1-yl)piperidin-1-yl)methyl)pyrimidin-4-yl)piperidine-2,6-dione;
1-(6-((4-(4-amino-3-(4-phenoxyphenyl)-1H-pyrazolo[3,4-d]pyrimidin-1-yl)piperidin-1-yl)methyl)pyridin-3-yl)dihydropyrimidine-2,4(1H,3H)-dione;
1-(6-((4-(4-amino-3-(4-phenoxyphenyl)-1H-pyrazolo[3,4-d]pyrimidin-1-yl)piperidin-1-yl)methyl)-5-fluoropyridin-3-yl)dihydropyrimidine-2,4(1H,3H)-dione;
1-(6-((4-(4-amino-3-(4-phenoxyphenyl)-1H-pyrazolo[3,4-d]pyrimidin-1-yl)piperidin-1-yl)methyl)-4-fluoropyridin-3-yl)dihydropyrimidine-2,4(1H,3H)-dione;
1-(6-((4-(4-amino-3-(4-phenoxyphenyl)-1H-pyrazolo[3,4-d]pyrimidin-1-yl)piperidin-1-yl)methyl)-2-fluoropyridin-3-yl)dihydropyrimidine-2,4(1H,3H)-dione;
1-(5-((4-(4-amino-3-(4-phenoxyphenyl)-1H-pyrazolo[3,4-d]pyrimidin-1-yl)piperidin-1-yl)methyl)pyridin-2-yl)dihydropyrimidine-2,4(1H,3H)-dione;
1-(5-((4-(4-amino-3-(4-phenoxyphenyl)-1H-pyrazolo[3,4-d]pyrimidin-1-yl)piperidin-1-yl)methyl)-4-fluoropyridin-2-yl)dihydropyrimidine-2,4(1H,3H)-dione;
1-(5-((4-(4-amino-3-(4-phenoxyphenyl)-1H-pyrazolo[3,4-d]pyrimidin-1-yl)piperidin-1-yl)methyl)-3-fluoropyridin-2-yl)dihydropyrimidine-2,4(1H,3H)-dione;
1-(5-((4-(4-amino-3-(4-phenoxyphenyl)-1H-pyrazolo[3,4-d]pyrimidin-1-yl)piperidin-1-yl)methyl)-6-fluoropyridin-2-yl)dihydropyrimidine-2,4(1H,3H)-dione;
1-(3-((4-(4-amino-3-(4-phenoxyphenyl)-1H-pyrazolo[3,4-d]pyrimidin-1-yl)piperidin-1-yl)methyl)pyridin-2-yl)dihydropyrimidine-2,4(1H,3H)-dione;
1-(3-((4-(4-amino-3-(4-phenoxyphenyl)-1H-pyrazolo[3,4-d]pyrimidin-1-yl)piperidin-1-yl)methyl)-4-fluoropyridin-2-yl)dihydropyrimidine-2,4(1H,3H)-dione;
1-(3-((4-(4-amino-3-(4-phenoxyphenyl)-1H-pyrazolo[3,4-d]pyrimidin-1-yl)piperidin-1-yl)methyl)-5-fluoropyridin-2-yl)dihydropyrimidine-2,4(1H,3H)-dione;
1-(3-((4-(4-amino-3-(4-phenoxyphenyl)-1H-pyrazolo[3,4-d]pyrimidin-1-yl)piperidin-1-yl)methyl)-6-fluoropyridin-2-yl)dihydropyrimidine-2,4(1H,3H)-dione;
1-(5-((4-(4-amino-3-(4-phenoxyphenyl)-1H-pyrazolo[3,4-d]pyrimidin-1-yl)piperidin-1-yl)methyl)pyridin-3-yl)dihydropyrimidine-2,4(1H,3H)-dione;
1-(5-((4-(4-amino-3-(4-phenoxyphenyl)-1H-pyrazolo[3,4-d]pyrimidin-1-yl)piperidin-1-yl)methyl)-4-fluoropyridin-3-yl)dihydropyrimidine-2,4(1H,3H)-dione;
1-(5-((4-(4-amino-3-(4-phenoxyphenyl)-1H-pyrazolo[3,4-d]pyrimidin-1-yl)piperidin-1-yl)methyl)-6-fluoropyridin-3-yl)dihydropyrimidine-2,4(1H,3H)-dione;
1-(5-((4-(4-amino-3-(4-phenoxyphenyl)-1H-pyrazolo[3,4-d]pyrimidin-1-yl)piperidin-1-yl)methyl)-2-fluoropyridin-3-yl)dihydropyrimidine-2,4(1H,3H)-dione;
1-(4-((4-(4-amino-3-(4-phenoxyphenyl)-1H-pyrazolo[3,4-d]pyrimidin-1-yl)piperidin-1-yl)methyl)pyridin-3-yl)dihydropyrimidine-2,4(1H,3H)-dione;
1-(4-((4-(4-amino-3-(4-phenoxyphenyl)-1H-pyrazolo[3,4-d]pyrimidin-1-yl)piperidin-1-yl)methyl)-5-fluoropyridin-3-yl)dihydropyrimidine-2,4(1H,3H)-dione;
1-(4-((4-(4-amino-3-(4-phenoxyphenyl)-1H-pyrazolo[3,4-d]pyrimidin-1-yl)piperidin-1-yl)methyl)-6-fluoropyridin-3-yl)dihydropyrimidine-2,4(1H,3H)-dione;
1-(4-((4-(4-amino-3-(4-phenoxyphenyl)-1H-pyrazolo[3,4-d]pyrimidin-1-yl)piperidin-1-yl)methyl)-2-fluoropyridin-3-yl)dihydropyrimidine-2,4(1H,3H)-dione;
1-(4-((4-(4-amino-3-(4-phenoxyphenyl)-1H-pyrazolo[3,4-d]pyrimidin-1-yl)piperidin-1-yl)methyl)pyridin-2-yl)dihydropyrimidine-2,4(1H,3H)-dione;
1-(4-((4-(4-amino-3-(4-phenoxyphenyl)-1H-pyrazolo[3,4-d]pyrimidin-1-yl)piperidin-1-yl)methyl)-3-fluoropyridin-2-yl)dihydropyrimidine-2,4(1H,3H)-dione;
1-(4-((4-(4-amino-3-(4-phenoxyphenyl)-1H-pyrazolo[3,4-d]pyrimidin-1-yl)piperidin-1-yl)methyl)-5-fluoropyridin-2-yl)dihydropyrimidine-2,4(1H,3H)-dione;
1-(4-((4-(4-amino-3-(4-phenoxyphenyl)-1H-pyrazolo[3,4-d]pyrimidin-1-yl)piperidin-1-yl)methyl)-6-fluoropyridin-2-yl)dihydropyrimidine-2,4(1H,3H)-dione;
1-(6-((4-(4-amino-3-(4-phenoxyphenyl)-1H-pyrazolo[3,4-d]pyrimidin-1-yl)piperidin-1-yl)methyl)pyridazin-3-yl)dihydropyrimidine-2,4(1H,3H)-dione;
1-(6-((4-(4-amino-3-(4-phenoxyphenyl)-1H-pyrazolo[3,4-d]pyrimidin-1-yl)piperidin-1-yl)methyl)-5-fluoropyridazin-3-yl)dihydropyrimidine-2,4(1H,3H)-dione;
1-(6-((4-(4-amino-3-(4-phenoxyphenyl)-1H-pyrazolo[3,4-d]pyrimidin-1-yl)piperidin-1-yl)methyl)-4-fluoropyridazin-3-yl)dihydropyrimidine-2,4(1H,3H)-dione;
1-(6-((4-(4-amino-3-(4-phenoxyphenyl)-1H-pyrazolo[3,4-d]pyrimidin-1-yl)piperidin-1-yl)methyl)pyridazin-4-yl)dihydropyrimidine-2,4(1H,3H)-dione;
1-(6-((4-(4-amino-3-(4-phenoxyphenyl)-1H-pyrazolo[3,4-d]pyrimidin-1-yl)piperidin-1-yl)methyl)-5-fluoropyridazin-4-yl)dihydropyrimidine-2,4(1H,3H)-dione;
1-(6-((4-(4-amino-3-(4-phenoxyphenyl)-1H-pyrazolo[3,4-d]pyrimidin-1-yl)piperidin-1-yl)methyl)-3-fluoropyridazin-4-yl)dihydropyrimidine-2,4(1H,3H)-dione;
1-(6-((4-(4-amino-3-(4-phenoxyphenyl)-1H-pyrazolo[3,4-d]pyrimidin-1-yl)piperidin-1-yl)methyl)pyrimidin-4-yl)dihydropyrimidine-2,4(1H,3H)-dione;
1-(5-((4-(4-amino-3-(4-phenoxyphenyl)-1H-pyrazolo[3,4-d]pyrimidin-1-yl)piperidin-1-yl)methyl)pyrazin-2-yl)dihydropyrimidine-2,4(1H,3H)-dione;
1-(6-((4-(4-amino-3-(4-phenoxyphenyl)-1H-pyrazolo[3,4-d]pyrimidin-1-yl)piperidin-1-yl)methyl)pyrazin-2-yl)dihydropyrimidine-2,4(1H,3H)-dione;
1-(4-((4-(4-amino-3-(4-phenoxyphenyl)-1H-pyrazolo[3,4-d]pyrimidin-1-yl)piperidin-1-yl)methyl)pyridazin-3-yl)dihydropyrimidine-2,4(1H,3H)-dione;
1-(5-((4-(4-amino-3-(4-phenoxyphenyl)-1H-pyrazolo[3,4-d]pyrimidin-1-yl)piperidin-1-yl)methyl)pyridazin-3-yl)dihydropyrimidine-2,4(1H,3H)-dione;
1-(5-((4-(4-amino-3-(4-phenoxyphenyl)-1H-pyrazolo[3,4-d]pyrimidin-1-yl)piperidin-1-yl)methyl)pyrimidin-2-yl)dihydropyrimidine-2,4(1H,3H)-dione;
1-(5-((4-(4-amino-3-(4-phenoxyphenyl)-1H-pyrazolo[3,4-d]pyrimidin-1-yl)piperidin-1-yl)methyl)pyrimidin-4-yl)dihydropyrimidine-2,4(1H,3H)-dione;
3-((6-((4-(4-amino-3-(4-phenoxyphenyl)-1H-pyrazolo[3,4-d]pyrimidin-1-yl)piperidin-1-yl)methyl)pyridin-3-yl)amino)piperidine-2,6-dione;
3-((6-((4-(4-amino-3-(4-phenoxyphenyl)-1H-pyrazolo[3,4-d]pyrimidin-1-yl)piperidin-1-yl)methyl)-5-fluoropyridin-3-yl)amino)piperidine-2,6-dione;
3-((6-((4-(4-amino-3-(4-phenoxyphenyl)-1H-pyrazolo[3,4-d]pyrimidin-1-yl)piperidin-1-yl)methyl)-4-fluoropyridin-3-yl)amino)piperidine-2,6-dione;
3-((6-((4-(4-amino-3-(4-phenoxyphenyl)-1H-pyrazolo[3,4-d]pyrimidin-1-yl)piperidin-1-yl)methyl)-2-fluoropyridin-3-yl)amino)piperidine-2,6-dione;
3-((5-((4-(4-amino-3-(4-phenoxyphenyl)-1H-pyrazolo[3,4-d]pyrimidin-1-yl)piperidin-1-yl)methyl)pyridin-2-yl)amino)piperidine-2,6-dione;
3-((5-((4-(4-amino-3-(4-phenoxyphenyl)-1H-pyrazolo[3,4-d]pyrimidin-1-yl)piperidin-1-yl)methyl)-4-fluoropyridin-2-yl)amino)piperidine-2,6-dione;
3-((5-((4-(4-amino-3-(4-phenoxyphenyl)-1H-pyrazolo[3,4-d]pyrimidin-1-yl)piperidin-1-yl)methyl)-3-fluoropyridin-2-yl)amino)piperidine-2,6-dione;
3-((5-((4-(4-amino-3-(4-phenoxyphenyl)-1H-pyrazolo[3,4-d]pyrimidin-1-yl)piperidin-1-yl)methyl)-6-fluoropyridin-2-yl)amino)piperidine-2,6-dione;
3-((3-((4-(4-amino-3-(4-phenoxyphenyl)-1H-pyrazolo[3,4-d]pyrimidin-1-yl)piperidin-1-yl)methyl)pyridin-2-yl)amino)piperidine-2,6-dione;
3-((3-((4-(4-amino-3-(4-phenoxyphenyl)-1H-pyrazolo[3,4-d]pyrimidin-1-yl)piperidin-1-yl)methyl)-4-fluoropyridin-2-yl)amino)piperidine-2,6-dione;
3-((3-((4-(4-amino-3-(4-phenoxyphenyl)-1H-pyrazolo[3,4-d]pyrimidin-1-yl)piperidin-1-yl)methyl)-5-fluoropyridin-2-yl)amino)piperidine-2,6-dione;
3-((3-((4-(4-amino-3-(4-phenoxyphenyl)-1H-pyrazolo[3,4-d]pyrimidin-1-yl)piperidin-1-yl)methyl)-6-fluoropyridin-2-yl)amino)piperidine-2,6-dione;
3-((5-((4-(4-amino-3-(4-phenoxyphenyl)-1H-pyrazolo[3,4-d]pyrimidin-1-yl)piperidin-1-yl)methyl)pyridin-3-yl)amino)piperidine-2,6-dione;
3-((5-((4-(4-amino-3-(4-phenoxyphenyl)-1H-pyrazolo[3,4-d]pyrimidin-1-yl)piperidin-1-yl)methyl)-4-fluoropyridin-3-yl)amino)piperidine-2,6-dione;
3-((5-((4-(4-amino-3-(4-phenoxyphenyl)-1H-pyrazolo[3,4-d]pyrimidin-1-yl)piperidin-1-yl)methyl)-6-fluoropyridin-3-yl)amino)piperidine-2,6-dione;
3-((5-((4-(4-amino-3-(4-phenoxyphenyl)-1H-pyrazolo[3,4-d]pyrimidin-1-yl)piperidin-1-yl)methyl)-2-fluoropyridin-3-yl)amino)piperidine-2,6-dione;
3-((4-((4-(4-amino-3-(4-phenoxyphenyl)-1H-pyrazolo[3,4-d]pyrimidin-1-yl)piperidin-1-yl)methyl)pyridin-3-yl)amino)piperidine-2,6-dione;
3-((4-((4-(4-amino-3-(4-phenoxyphenyl)-1H-pyrazolo[3,4-d]pyrimidin-1-yl)piperidin-1-yl)methyl)-5-fluoropyridin-3-yl)amino)piperidine-2,6-dione;
3-((4-((4-(4-amino-3-(4-phenoxyphenyl)-1H-pyrazolo[3,4-d]pyrimidin-1-yl)piperidin-1-yl)methyl)-6-fluoropyridin-3-yl)amino)piperidine-2,6-dione;
3-((4-((4-(4-amino-3-(4-phenoxyphenyl)-1H-pyrazolo[3,4-d]pyrimidin-1-yl)piperidin-1-yl)methyl)-2-fluoropyridin-3-yl)amino)piperidine-2,6-dione;
3-((4-((4-(4-amino-3-(4-phenoxyphenyl)-1H-pyrazolo[3,4-d]pyrimidin-1-yl)piperidin-1-yl)methyl)pyridin-2-yl)amino)piperidine-2,6-dione;
3-((4-((4-(4-amino-3-(4-phenoxyphenyl)-1H-pyrazolo[3,4-d]pyrimidin-1-yl)piperidin-1-yl)methyl)-3-fluoropyridin-2-yl)amino)piperidine-2,6-dione;
3-((4-((4-(4-amino-3-(4-phenoxyphenyl)-1H-pyrazolo[3,4-d]pyrimidin-1-yl)piperidin-1-yl)methyl)-5-fluoropyridin-2-yl)amino)piperidine-2,6-dione;
3-((4-((4-(4-amino-3-(4-phenoxyphenyl)-1H-pyrazolo[3,4-d]pyrimidin-1-yl)piperidin-1-yl)methyl)-6-fluoropyridin-2-yl)amino)piperidine-2,6-dione;
3-((6-((4-(4-amino-3-(4-phenoxyphenyl)-1H-pyrazolo[3,4-d]pyrimidin-1-yl)piperidin-1-yl)methyl)pyridazin-3-yl)amino)piperidine-2,6-dione;
3-((6-((4-(4-amino-3-(4-phenoxyphenyl)-1H-pyrazolo[3,4-d]pyrimidin-1-yl)piperidin-1-yl)methyl)-5-fluoropyridazin-3-yl)amino)piperidine-2,6-dione;
3-((6-((4-(4-amino-3-(4-phenoxyphenyl)-1H-pyrazolo[3,4-d]pyrimidin-1-yl)piperidin-1-yl)methyl)-4-fluoropyridazin-3-yl)amino)piperidine-2,6-dione;
3-((6-((4-(4-amino-3-(4-phenoxyphenyl)-1H-pyrazolo[3,4-d]pyrimidin-1-yl)piperidin-1-yl)methyl)pyridazin-4-yl)amino)piperidine-2,6-dione;
3-((6-((4-(4-amino-3-(4-phenoxyphenyl)-1H-pyrazolo[3,4-d]pyrimidin-1-yl)piperidin-1-yl)methyl)-5-fluoropyridazin-4-yl)amino)piperidine-2,6-dione;
3-((6-((4-(4-amino-3-(4-phenoxyphenyl)-1H-pyrazolo[3,4-d]pyrimidin-1-yl)piperidin-1-yl)methyl)-3-fluoropyridazin-4-yl)amino)piperidine-2,6-dione;
3-((6-((4-(4-amino-3-(4-phenoxyphenyl)-1H-pyrazolo[3,4-d]pyrimidin-1-yl)piperidin-1-yl)methyl)pyrimidin-4-yl)amino)piperidine-2,6-dione;
3-((5-((4-(4-amino-3-(4-phenoxyphenyl)-1H-pyrazolo[3,4-d]pyrimidin-1-yl)piperidin-1-yl)methyl)pyrazin-2-yl)amino)piperidine-2,6-dione;
3-((6-((4-(4-amino-3-(4-phenoxyphenyl)-1H-pyrazolo[3,4-d]pyrimidin-1-yl)piperidin-1-yl)methyl)pyrazin-2-yl)amino)piperidine-2,6-dione;
3-((4-((4-(4-amino-3-(4-phenoxyphenyl)-1H-pyrazolo[3,4-d]pyrimidin-1-yl)piperidin-1-yl)methyl)pyridazin-3-yl)amino)piperidine-2,6-dione;
3-((5-((4-(4-amino-3-(4-phenoxyphenyl)-1H-pyrazolo[3,4-d]pyrimidin-1-yl)piperidin-1-yl)methyl)pyridazin-3-yl)amino)piperidine-2,6-dione;
3-((5-((4-(4-amino-3-(4-phenoxyphenyl)-1H-pyrazolo[3,4-d]pyrimidin-1-yl)piperidin-1-yl)methyl)pyrimidin-2-yl)amino)piperidine-2,6-dione;
3-((5-((4-(4-amino-3-(4-phenoxyphenyl)-1H-pyrazolo[3,4-d]pyrimidin-1-yl)piperidin-1-yl)methyl)pyrimidin-4-yl)amino)piperidine-2,6-dione;
3-(4-(((3R,4S)-4-(4-amino-3-(4-phenoxyphenyl)-1H-pyrazolo[3,4-d]pyrimidin-1-yl)-3-fluoropiperidin-1-yl)methyl)phenyl)piperidine-2,6-dione;
3-(4-(((3R,4S)-4-(4-amino-3-(4-phenoxyphenyl)-1H-pyrazolo[3,4-d]pyrimidin-1-yl)-3-fluoropiperidin-1-yl)methyl)-2-fluorophenyl)piperidine-2,6-dione;
3-((4-(1-(4-(2,6-dioxopiperidin-3-yl)benzyl)piperidin-4-yl)phenyl)amino)-5-(piperidin-1-yl)pyrazine-2-carboxamide;
3-((4-(1-(4-(2,6-dioxopiperidin-3-yl)-3-fluorobenzyl)piperidin-4-yl)phenyl)amino)-5-(piperidin-1-yl)pyrazine-2-carboxamide;
3-((4-(1-(3-(2,6-dioxopiperidin-3-yl)benzyl)piperidin-4-yl)phenyl)amino)-5-(piperidin-1-yl)pyrazine-2-carboxamide;
3-((4-(1-(2-(2,6-dioxopiperidin-3-yl)benzyl)piperidin-4-yl)phenyl)amino)-5-(piperidin-1-yl)pyrazine-2-carboxamide;
3-((4-(1-(4-(2,4-dioxotetrahydropyrimidin-1(2H)-yl)benzyl)piperidin-4-yl)phenyl)amino)-5-(piperidin-1-yl)pyrazine-2-carboxamide;
3-((4-(1-(4-(2,4-dioxotetrahydropyrimidin-1(2H)-yl)-2-fluorobenzyl)piperidin-4-yl)phenyl)amino)-5-(piperidin-1-yl)pyrazine-2-carboxamide;
3-((4-(1-(4-(2,4-dioxotetrahydropyrimidin-1(2H)-yl)-3-fluorobenzyl)piperidin-4-yl)phenyl)amino)-5-(piperidin-1-yl)pyrazine-2-carboxamide;
3-((4-(1-(3-(2,4-dioxotetrahydropyrimidin-1(2H)-yl)benzyl)piperidin-4-yl)phenyl)amino)-5-(piperidin-1-yl)pyrazine-2-carboxamide;
3-((4-(1-(2-(2,4-dioxotetrahydropyrimidin-1(2H)-yl)benzyl)piperidin-4-yl)phenyl)amino)-5-(piperidin-1-yl)pyrazine-2-carboxamide;
3-((4-(1-((5-(2,4-dioxotetrahydropyrimidin-1(2H)-yl)pyridin-2-yl)methyl)piperidin-4-yl)phenyl)amino)-5-(piperidin-1-yl)pyrazine-2-carboxamide;
3-((4-(1-((6-(2,4-dioxotetrahydropyrimidin-1(2H)-yl)pyridazin-3-yl)methyl)piperidin-4-yl)phenyl)amino)-5-(piperidin-1-yl)pyrazine-2-carboxamide;
3-((4-(1-((3-(2,4-dioxotetrahydropyrimidin-1(2H)-yl)pyridin-4-yl)methyl)piperidin-4-yl)phenyl)amino)-5-(piperidin-1-yl)pyrazine-2-carboxamide;
3-((4-(1-(4-((2,6-dioxopiperidin-3-yl)amino)benzyl)piperidin-4-yl)phenyl)amino)-5-(piperidin-1-yl)pyrazine-2-carboxamide;
3-((4-(1-(3-((2,6-dioxopiperidin-3-yl)amino)benzyl)piperidin-4-yl)phenyl)amino)-5-(piperidin-1-yl)pyrazine-2-carboxamide;
3-((4-(1-(2-((2,6-dioxopiperidin-3-yl)amino)benzyl)piperidin-4-yl)phenyl)amino)-5-(piperidin-1-yl)pyrazine-2-carboxamide;
3-((4-(1-(4-(2,6-dioxopiperidin-3-yl)benzyl)piperidin-4-yl)phenyl)amino)-5-((R)-3-(3-methyl-2-oxoimidazolidin-1-yl)piperidin-1-yl)pyrazine-2-carboxamide;
3-((4-(1-(4-(2,6-dioxopiperidin-3-yl)-3-fluorobenzyl)piperidin-4-yl)phenyl)amino)-5-((R)-3-(3-methyl-2-oxoimidazolidin-1-yl)piperidin-1-yl)pyrazine-2-carboxamide;
3-((4-(1-(3-(2,6-dioxopiperidin-3-yl)benzyl)piperidin-4-yl)phenyl)amino)-5-((R)-3-(3-methyl-2-oxoimidazolidin-1-yl)piperidin-1-yl)pyrazine-2-carboxamide;
3-((4-(1-(2-(2,6-dioxopiperidin-3-yl)benzyl)piperidin-4-yl)phenyl)amino)-5-((R)-3-(3-methyl-2-oxoimidazolidin-1-yl)piperidin-1-yl)pyrazine-2-carboxamide;
(R)-3-((4-(1-(4-(2,4-dioxotetrahydropyrimidin-1(2H)-yl)benzyl)piperidin-4-yl)phenyl)amino)-5-(3-(3-methyl-2-oxoimidazolidin-1-yl)piperidin-1-yl)pyrazine-2-carboxamide;
(R)-3-((4-(1-(4-(2,4-dioxotetrahydropyrimidin-1(2H)-yl)-2-fluorobenzyl)piperidin-4-yl)phenyl)amino)-5-(3-(3-methyl-2-oxoimidazolidin-1-yl)piperidin-1-yl)pyrazine-2-carboxamide;
(R)-3-((4-(1-(4-(2,4-dioxotetrahydropyrimidin-1(2H)-yl)-3-fluorobenzyl)piperidin-4-yl)phenyl)amino)-5-(3-(3-methyl-2-oxoimidazolidin-1-yl)piperidin-1-yl)pyrazine-2-carboxamide;
(R)-3-((4-(1-(3-(2,4-dioxotetrahydropyrimidin-1(2H)-yl)benzyl)piperidin-4-yl)phenyl)amino)-5-(3-(3-methyl-2-oxoimidazolidin-1-yl)piperidin-1-yl)pyrazine-2-carboxamide;
(R)-3-((4-(1-(2-(2,4-dioxotetrahydropyrimidin-1(2H)-yl)benzyl)piperidin-4-yl)phenyl)amino)-5-(3-(3-methyl-2-oxoimidazolidin-1-yl)piperidin-1-yl)pyrazine-2-carboxamide;
(R)-3-((4-(1-((5-(2,4-dioxotetrahydropyrimidin-1(2H)-yl)pyridin-2-yl)methyl)piperidin-4-yl)phenyl)amino)-5-(3-(3-methyl-2-oxoimidazolidin-1-yl)piperidin-1-yl)pyrazine-2-carboxamide;
(R)-3-((4-(1-((6-(2,4-dioxotetrahydropyrimidin-1(2H)-yl)pyridazin-3-yl)methyl)piperidin-4-yl)phenyl)amino)-5-(3-(3-methyl-2-oxoimidazolidin-1-yl)piperidin-1-yl)pyrazine-2-carboxamide;
(R)-3-((4-(1-((3-(2,4-dioxotetrahydropyrimidin-1(2H)-yl)pyridin-4-yl)methyl)piperidin-4-yl)phenyl)amino)-5-(3-(3-methyl-2-oxoimidazolidin-1-yl)piperidin-1-yl)pyrazine-2-carboxamide;
*3-((4-(1-(4-((2,6-dioxopiperidin-3-yl)amino)benzyl)piperidin-4-yl)phenyl)amino)-5-((R)-3-(3-methyl-2-*oxoimidazolidin-1-yl)piperidin-1-yl)pyrazine-2-carboxamide;
3-((4-(1-(3-((2,6-dioxopiperidin-3-yl)amino)benzyl)piperidin-4-yl)phenyl)amino)-5-((R)-3-(3-methyl-2-oxoimidazolidin-1-yl)piperidin-1-yl)pyrazine-2-carboxamide;
3-((4-(1-(2-((2,6-dioxopiperidin-3-yl)amino)benzyl)piperidin-4-yl)phenyl)amino)-5-((R)-3-(3-methyl-2-oxoimidazolidin-1-yl)piperidin-1-yl)pyrazine-2-carboxamide;
3-(4-((4-(4-(1,2-bis(4-hydroxyphenyl)but-1-en-1-yl)phenyl)piperazin-1-yl)methyl)phenyl)piperidine-2,6-dione;
3-(4-((4-(4-(1,2-bis(4-hydroxyphenyl)but-1-en-1-yl)phenyl)piperazin-1-yl)methyl)-2-fluorophenyl)piperidine-2,6-dione;
3-(3-((4-(4-(1,2-bis(4-hydroxyphenyl)but-1-en-1-yl)phenyl)piperazin-1-yl)methyl)phenyl)piperidine-2,6-dione;
3-(2-((4-(4-(1,2-bis(4-hydroxyphenyl)but-1-en-1-yl)phenyl)piperazin-1-yl)methyl)phenyl)piperidine-2,6-dione;
1-(4-((4-(4-(1,2-bis(4-hydroxyphenyl)but-1-en-1-yl)phenyl)piperazin-1-yl)methyl)phenyl)dihydropyrimidine-2,4(1H,3H)-dione;
1-(4-((4-(4-(1,2-bis(4-hydroxyphenyl)but-1-en-1-yl)phenyl)piperazin-1-yl)methyl)-3-fluorophenyl)dihydropyrimidine-2,4(1H,3H)-dione;
1-(3-((4-(4-(1,2-bis(4-hydroxyphenyl)but-1-en-1-yl)phenyl)piperazin-1-yl)methyl)phenyl)dihydropyrimidine-2,4(1H,3H)-dione;
1-(2-((4-(4-(1,2-bis(4-hydroxyphenyl)but-1-en-1-yl)phenyl)piperazin-1-yl)methyl)phenyl)dihydropyrimidine-2,4(1H,3H)-dione;
1-(6-((4-(4-(1,2-bis(4-hydroxyphenyl)but-1-en-1-yl)phenyl)piperazin-1-yl)methyl)pyridin-3-yl)dihydropyrimidine-2,4(1H,3H)-dione;
1-(6-((4-(4-(1,2-bis(4-hydroxyphenyl)but-1-en-1-yl)phenyl)piperazin-1-yl)methyl)pyridazin-3-yl)dihydropyrimidine-2,4(1H,3H)-dione;
1-(4-((4-(4-(1,2-bis(4-hydroxyphenyl)but-1-en-1-yl)phenyl)piperazin-1-yl)methyl)pyridin-3-yl)dihydropyrimidine-2,4(1H,3H)-dione;
3-((4-((4-(4-(1,2-bis(4-hydroxyphenyl)but-1-en-1-yl)phenyl)piperazin-1-yl)methyl)phenyl)amino)piperidine-2,6-dione;
3-((3-((4-(4-(1,2-bis(4-hydroxyphenyl)but-1-en-1-yl)phenyl)piperazin-1-yl)methyl)phenyl)amino)piperidine-2,6-dione;
3-((2-((4-(4-(1,2-bis(4-hydroxyphenyl)but-1-en-1-yl)phenyl)piperazin-1-yl)methyl)phenyl)amino)piperidine-2,6-dione;
3-(4-((4-(4-(1-(4-hydroxyphenyl)-2-phenylbut-1-en-1-yl)phenyl)piperazin-1-yl)methyl)phenyl)piperidine-2,6-dione;
3-(2-fluoro-4-((4-(4-(1-(4-hydroxyphenyl)-2-phenylbut-1-en-1-yl)phenyl)piperazin-1-yl)methyl)phenyl)piperidine-2,6-dione;
3-(3-((4-(4-(1-(4-hydroxyphenyl)-2-phenylbut-1-en-1-yl)phenyl)piperazin-1-yl)methyl)phenyl)piperidine-2,6-dione;
3-(2-((4-(4-(1-(4-hydroxyphenyl)-2-phenylbut- 1-en-1-yl)phenyl)piperazin-1-yl)methyl)phenyl)piperidine-2,6-dione;
1-(4-((4-(4-(1-(4-hydroxyphenyl)-2-phenylbut-1-en-1-yl)phenyl)piperazin-1-yl)methyl)phenyl)dihydropyrimidine-2,4(1H,3H)-dione;
1-(3-fluoro-4-((4-(4-(1-(4-hydroxyphenyl)-2-phenylbut-1-en-1-yl)phenyl)piperazin-1-yl)methyl)phenyl)dihydropyrimidine-2,4(1H,3H)-dione;
1-(3-((4-(4-(1-(4-hydroxyphenyl)-2-phenylbut-1-en-1-yl)phenyl)piperazin-1-yl)methyl)phenyl)dihydropyrimidine-2,4(1H,3H)-dione;
1-(2-((4-(4-(1-(4-hydroxyphenyl)-2-phenylbut-1-en-1-yl)phenyl)piperazin-1-yl)methyl)phenyl)dihydropyrimidine-2,4(1H,3H)-dione;
1-(6-((4-(4-(1-(4-hydroxyphenyl)-2-phenylbut-1-en-1-yl)phenyl)piperazin-1-yl)methyl)pyridin-3-yl)dihydropyrimidine-2,4(1H,3H)-dione;
1-(6-((4-(4-(1-(4-hydroxyphenyl)-2-phenylbut-1-en-1-yl)phenyl)piperazin-1-yl)methyl)pyridazin-3-yl)dihydropyrimidine-2,4(1H,3H)-dione;
1-(4-((4-(4-(1-(4-hydroxyphenyl)-2-phenylbut-1-en-1-yl)phenyl)piperazin-1-yl)methyl)pyridin-3-yl)dihydropyrimidine-2,4(1H,3H)-dione;
3-((4-((4-(4-(1-(4-hydroxyphenyl)-2-phenylbut-1-en-1-yl)phenyl)piperazin-1-yl)methyl)phenyl)amino)piperidine-2,6-dione;
3-((3-((4-(4-(1-(4-hydroxyphenyl)-2-phenylbut-1-en-1-yl)phenyl)piperazin-1-yl)methyl)phenyl)amino)piperidine-2,6-dione;
3-((2-((4-(4-(1-(4-hydroxyphenyl)-2-phenylbut-1-en-1-yl)phenyl)piperazin-1-yl)methyl)phenyl)amino)piperidine-2,6-dione;
3-(4-((4-(4-(4-chloro-1,2-bis(4-hydroxyphenyl)but-1-en-1-yl)phenyl)piperazin-1-yl)methyl)phenyl)piperidine-2,6-dione;
3-(4-((4-(4-(4-chloro-1,2-bis(4-hydroxyphenyl)but-1-en-1-yl)phenyl)piperazin-1-yl)methyl)-2-fluorophenyl)piperidine-2,6-dione;
3-(3-((4-(4-(4-chloro-1,2-bis(4-hydroxyphenyl)but-1-en-1-yl)phenyl)piperazin-1-yl)methyl)phenyl)piperidine-2,6-dione;
3-(2-((4-(4-(4-chloro-1,2-bis(4-hydroxyphenyl)but-1-en-1-yl)phenyl)piperazin-1-yl)methyl)phenyl)piperidine-2,6-dione;
1-(4-((4-(4-(4-chloro-1,2-bis(4-hydroxyphenyl)but-1-en-1-yl)phenyl)piperazin-1-yl)methyl)phenyl)dihydropyrimidine-2,4(1H,3H)-dione;
1-(4-((4-(4-(4-chloro-1,2-bis(4-hydroxyphenyl)but-1-en-1-yl)phenyl)piperazin-1-yl)methyl)-3-fluorophenyl)dihydropyrimidine-2,4(1H,3H)-dione;
1-(3-((4-(4-(4-chloro-1,2-bis(4-hydroxyphenyl)but-1-en-1-yl)phenyl)piperazin-1-yl)methyl)phenyl)dihydropyrimidine-2,4(1H,3H)-dione;
1-(2-((4-(4-(4-chloro-1,2-bis(4-hydroxyphenyl)but-1-en-1-yl)phenyl)piperazin-1-yl)methyl)phenyl)dihydropyrimidine-2,4(1H,3H)-dione;
1-(6-((4-(4-(4-chloro-1,2-bis(4-hydroxyphenyl)but-1-en-1-yl)phenyl)piperazin-1-yl)methyl)pyridin-3-yl)dihydropyrimidine-2,4(1H,3H)-dione;
1-(6-((4-(4-(4-chloro-1,2-bis(4-hydroxyphenyl)but-1-en-1-yl)phenyl)piperazin-1-yl)methyl)pyridazin-3-yl)dihydropyrimidine-2,4(1H,3H)-dione;
1-(4-((4-(4-(4-chloro-1,2-bis(4-hydroxyphenyl)but-1-en-1-yl)phenyl)piperazin-1-yl)methyl)pyridin-3-yl)dihydropyrimidine-2,4(1H,3H)-dione;
3-((4-((4-(4-(4-chloro-1,2-bis(4-hydroxyphenyl)but-1-en-1-yl)phenyl)piperazin-1-yl)methyl)phenyl)amino)piperidine-2,6-dione;
3-((3-((4-(4-(4-chloro-1,2-bis(4-hydroxyphenyl)but-1-en-1-yl)phenyl)piperazin-1-yl)methyl)phenyl)amino)piperidine-2,6-dione;
3-((2-((4-(4-(4-chloro-1,2-bis(4-hydroxyphenyl)but-1-en-1-yl)phenyl)piperazin-1-yl)methyl)phenyl)amino)piperidine-2,6-dione;
3-(4-((4-(4-(4-chloro-1-(4-hydroxyphenyl)-2-phenylbut-1-en-1-yl)phenyl)piperazin-1-yl)methyl)phenyl)piperidine-2,6-dione;
3-(4-((4-(4-(4-chloro-1-(4-hydroxyphenyl)-2-phenylbut-1-en-1-yl)phenyl)piperazin-1-yl)methyl)-2-fluorophenyl)piperidine-2,6-dione;
3-(3-((4-(4-(4-chloro-1-(4-hydroxyphenyl)-2-phenylbut-1-en-1-yl)phenyl)piperazin-1-yl)methyl)phenyl)piperidine-2,6-dione;
3-(2-((4-(4-(4-chloro-1-(4-hydroxyphenyl)-2-phenylbut-1-en-1-yl)phenyl)piperazin-1-yl)methyl)phenyl)piperidine-2,6-dione;
1-(4-((4-(4-(4-chloro-1-(4-hydroxyphenyl)-2-phenylbut-1-en-1-yl)phenyl)piperazin-1-yl)methyl)phenyl)dihydropyrimidine-2,4(1H,3H)-dione;
1-(4-((4-(4-(4-chloro-1-(4-hydroxyphenyl)-2-phenylbut-1-en-1-yl)phenyl)piperazin-1-yl)methyl)-3-fluorophenyl)dihydropyrimidine-2,4(1H,3H)-dione;
1-(3-((4-(4-(4-chloro-1-(4-hydroxyphenyl)-2-phenylbut-1-en-1-yl)phenyl)piperazin-1-yl)methyl)phenyl)dihydropyrimidine-2,4(1H,3H)-dione;
1-(2-((4-(4-(4-chloro-1-(4-hydroxyphenyl)-2-phenylbut-1-en-1-yl)phenyl)piperazin-1-yl)methyl)phenyl)dihydropyrimidine-2,4(1H,3H)-dione;
1-(6-((4-(4-(4-chloro-1-(4-hydroxyphenyl)-2-phenylbut-1-en-1-yl)phenyl)piperazin-1-yl)methyl)pyridin-3-yl)dihydropyrimidine-2,4(1H,3H)-dione;
1-(6-((4-(4-(4-chloro-1-(4-hydroxyphenyl)-2-phenylbut-1-en-1-yl)phenyl)piperazin-1-yl)methyl)pyridazin-3-yl)dihydropyrimidine-2,4(1H,3H)-dione;
1-(4-((4-(4-(4-chloro-1-(4-hydroxyphenyl)-2-phenylbut-1-en-1-yl)phenyl)piperazin-1-yl)methyl)pyridin-3-yl)dihydropyrimidine-2,4(1H,3H)-dione;
3-((4-((4-(4-(4-chloro-1-(4-hydroxyphenyl)-2-phenylbut-1-en-1-yl)phenyl)piperazin-1-yl)methyl)phenyl)amino)piperidine-2,6-dione;
3-((3-((4-(4-(4-chloro-1-(4-hydroxyphenyl)-2-phenylbut-1-en-1-yl)phenyl)piperazin-1-yl)methyl)phenyl)amino)piperidine-2,6-dione;
3-((2-((4-(4-(4-chloro-1-(4-hydroxyphenyl)-2-phenylbut-1-en-1-yl)phenyl)piperazin-1-yl)methyl)phenyl)amino)piperidine-2,6-dione;
3-(4-((4-(4-((1R,2S)-6-hydroxy-2-phenyl-1,2,3,4-tetrahydronaphthalen-1-yl)phenyl)piperazin-1-yl)methyl)phenyl)piperidine-2,6-dione;
3-(4-((4-(4-((1S,2R)-6-hydroxy-2-phenyl-1,2,3,4-tetrahydronaphthalen-1-yl)phenyl)piperazin-1-yl)methyl)phenyl)piperidine-2,6-dione;
3-(2-fluoro-4-((4-(4-((1R,2S)-6-hydroxy-2-phenyl-1,2,3,4-tetrahydronaphthalen-1-yl)phenyl)piperazin-1-yl)methyl)phenyl)piperidine-2,6-dione;
3-(2-fluoro-4-((4-(4-((1S,2R)-6-hydroxy-2-phenyl-1,2,3,4-tetrahydronaphthalen-1-yl)phenyl)piperazin-1-yl)methyl)phenyl)piperidine-2,6-dione;
3-(3-((4-(4-(6-hydroxy-2-phenyl-1,2,3,4-tetrahydronaphthalen-1-yl)phenyl)piperazin-1-yl)methyl)phenyl)piperidine-2,6-dione;
3-(3-((4-(4-((1R,2S)-6-hydroxy-2-phenyl-1,2,3,4-tetrahydronaphthalen-1-yl)phenyl)piperazin-1-yl)methyl)phenyl)piperidine-2,6-dione;
3-(3-((4-(4-((1S,2R)-6-hydroxy-2-phenyl-1,2,3,4-tetrahydronaphthalen-1-yl)phenyl)piperazin-1-yl)methyl)phenyl)piperidine-2,6-dione;
3-(2-((4-(4-((1R,2S)-6-hydroxy-2-phenyl-1,2,3,4-tetrahydronaphthalen-1-yl)phenyl)piperazin-1-yl)methyl)phenyl)piperidine-2,6-dione;
3-(2-((4-(4-((1S,2R)-6-hydroxy-2-phenyl-1,2,3,4-tetrahydronaphthalen-1-yl)phenyl)piperazin-1-yl)methyl)phenyl)piperidine-2,6-dione;
1-(4-((4-(4-((1R,2S)-6-hydroxy-2-phenyl-1,2,3,4-tetrahydronaphthalen-1-yl)phenyl)piperazin-1-yl)methyl)phenyl)dihydropyrimidine-2,4(1H,3H)-dione;
1-(4-((4-(4-((1S,2R)-6-hydroxy-2-phenyl-1,2,3,4-tetrahydronaphthalen-1-yl)phenyl)piperazin-1-yl)methyl)phenyl)dihydropyrimidine-2,4(1H,3H)-dione;
1-(3-fluoro-4-((4-(4-((1R,2S)-6-hydroxy-2-phenyl-1,2,3,4-tetrahydronaphthalen-1-yl)phenyl)piperazin-1-yl)methyl)phenyl)dihydropyrimidine-2,4(1H,3H)-dione;
1-(3-fluoro-4-((4-(4-((1S,2R)-6-hydroxy-2-phenyl-1,2,34-tetrahydronaphthalen-1-yl)phenyl)piperazin-1-yl)methyl)phenyl)dihydropyrimidine-2,4(1H,3H)-dione;
1-(3-((4-(4-((1R,2S)-6-hydroxy-2-phenyl-1,2,3,4-tetrahydronaphthalen-1-yl)phenyl)piperazin-1-yl)methyl)phenyl)dihydropyrimidine-2,4(1H,3H)-dione;
1-(3-((4-(4-((1S,2R)-6-hydroxy-2-phenyl-1,2,3,4-tetrahydronaphthalen-1-yl)phenyl)piperazin-1-yl)methyl)phenyl)dihydropyrimidine-2,4(1H,3H)-dione;
1-(2-((4-(4-((1R,2S)-6-hydroxy-2-phenyl-1,2,3,4-tetrahydronaphthalen-1-yl)phenyl)piperazin-1-yl)methyl)phenyl)dihydropyrimidine-2,4(1H,3H)-dione;
1-(2-((4-(4-((1S,2R)-6-hydroxy-2-phenyl-1,2,3,4-tetrahydronaphthalen-1-yl)phenyl)piperazin-1-yl)methyl)phenyl)dihydropyrimidine-2,4(1H,3H)-dione;
1-(6-((4-(4-((1R,2S)-6-hydroxy-2-phenyl-1,2,3,4-tetrahydronaphthalen-1-yl)phenyl)piperazin-1-yl)methyl)pyridin-3-yl)dihydropyrimidine-2,4(1H,3H)-dione;
1-(6-((4-(4-((1S,2R)-6-hydroxy-2-phenyl-1,2,3,4-tetrahydronaphthalen-1-yl)phenyl)piperazin-1-yl)methyl)pyridin-3-yl)dihydropyrimidine-2,4(1H,3H)-dione;
1-(6-((4-(4-((1R,2S)-6-hydroxy-2-phenyl-1,2,3,4-tetrahydronaphthalen-1-yl)phenyl)piperazin-1-yl)methyl)pyridazin-3-yl)dihydropyrimidine-2,4(1H,3H)-dione;
1-(6-((4-(4-((1S,2R)-6-hydroxy-2-phenyl-1,2,3,4-tetrahydronaphthalen-1-yl)phenyl)piperazin-1-yl)methyl)pyridazin-3-yl)dihydropyrimidine-2,4(1H,3H)-dione;
1-(4-((4-(4-((1R,2S)-6-hydroxy-2-phenyl-1,2,3,4-tetrahydronaphthalen-1-yl)phenyl)piperazin-1-yl)methyl)pyridin-3-yl)dihydropyrimidine-2,4(1H,3H)-dione;
1-(4-((4-(4-((1S,2R)-6-hydroxy-2-phenyl-1,2,3,4-tetrahydronaphthalen-1-yl)phenyl)piperazin-1-yl)methyl)pyridin-3-yl)dihydropyrimidine-2,4(1H,3H)-dione;
3-((4-((4-(4-((1R,2S)-6-hydroxy-2-phenyl-1,2,3,4-tetrahydronaphthalen-1-yl)phenyl)piperazin-1-yl)methyl)phenyl)amino)piperidine-2,6-dione;
3-((4-((4-(4-((1S,2R)-6-hydroxy-2-phenyl-1,2,3,4-tetrahydronaphthalen-1-yl)phenyl)piperazin-1-yl)methyl)phenyl)amino)piperidine-2,6-dione;
3-((3-((4-(4-((1R,2S)-6-hydroxy-2-phenyl-1,2,3,4-tetrahydronaphthalen-1-yl)phenyl)piperazin-1-yl)methyl)phenyl)amino)piperidine-2,6-dione;
3-((3-((4-(4-((1S,2R)-6-hydroxy-2-phenyl-1,2,3,4-tetrahydronaphthalen-1-yl)phenyl)piperazin-1-yl)methyl)phenyl)amino)piperidine-2,6-dione;
3-((2-((4-(4-((1R,2S)-6-hydroxy-2-phenyl-1,2,3,4-tetrahydronaphthalen-1-yl)phenyl)piperazin-1-yl)methyl)phenyl)amino)piperidine-2,6-dione;
3-((2-((4-(4-((1S,2R)-6-hydroxy-2-phenyl-1,2,3,4-tetrahydronaphthalen-1-yl)phenyl)piperazin-1-yl)methyl)phenyl)amino)piperidine-2,6-dione;
3-(4-(((1-(4-(6-hydroxy-2-phenyl-1,2,3,4-tetrahydronaphthalen-1-yl)phenyl)piperidin-4-yl)(methyl)amino)methyl)phenyl)piperidine-2,6-dione;
3-(2-fluoro-4-(((1-(4-(6-hydroxy-2-phenyl-1,2,3,4-tetrahydronaphthalen-1-yl)phenyl)piperidin-4-yl)(methyl)amino)methyl)phenyl)piperidine-2,6-dione;
3-(3-(((1-(4-(6-hydroxy-2-phenyl-1,2,3,4-tetrahydronaphthalen-1-yl)phenyl)piperidin-4-yl)(methyl)amino)methyl)phenyl)piperidine-2,6-dione;
3-(2-(((1-(4-(6-hydroxy-2-phenyl-1,2,3,4-tetrahydronaphthalen-1-yl)phenyl)piperidin-4-yl)(methyl)amino)methyl)phenyl)piperidine-2,6-dione;
1-(4-(((1-(4-(6-hydroxy-2-phenyl-1,2,3,4-tetrahydronaphthalen-1-yl)phenyl)piperidin-4-yl)(methyl)amino)methyl)phenyl)dihydropyrimidine-2,4(1H,3H)-dione;
1-(3-fluoro-4-(((1-(4-(6-hydroxy-2-phenyl-1,2,3,4-tetrahydronaphthalen-1-yl)phenyl)piperidin-4-yl)(methyl)amino)methyl)phenyl)dihydropyrimidine-2,4(1H,3H)-dione;
1-(3-(((1-(4-(6-hydroxy-2-phenyl-1,2,3,4-tetrahydronaphthalen-1-yl)phenyl)piperidin-4-yl)(methyl)amino)methyl)phenyl)dihydropyrimidine-2,4(1H,3H)-dione;
1-(2-(((1-(4-(6-hydroxy-2-phenyl-1,2,3,4-tetrahydronaphthalen-1-yl)phenyl)piperidin-4-yl)(methyl)amino)methyl)phenyl)dihydropyrimidine-2,4(1H,3H)-dione;
1-(6-(((1-(4-(6-hydroxy-2-phenyl-1,2,3,4-tetrahydronaphthalen-1-yl)phenyl)piperidin-4-yl)(methyl)amino)methyl)pyridin-3-yl)dihydropyrimidine-2,4(1H,3H)-dione;
1-(6-(((1-(4-(6-hydroxy-2-phenyl-1,2,3,4-tetrahydronaphthalen-1-yl)phenyl)piperidin-4-yl)(methyl)amino)methyl)pyridazin-3-yl)dihydropyrimidine-2,4(1H,3H)-dione;
1-(4-(((1-(4-(6-hydroxy-2-phenyl-1,2,3,4-tetrahydronaphthalen-1-yl)phenyl)piperidin-4-yl)(methyl)amino)methyl)pyridin-3-yl)dihydropyrimidine-2,4(1H,3H)-dione;
3-((4-(((1-(4-(6-hydroxy-2-phenyl-1,2,3,4-tetrahydronaphthalen-1-yl)phenyl)piperidin-4-yl)(methyl)amino)methyl)phenyl)amino)piperidine-2,6-dione;
3-((3-(((1-(4-(6-hydroxy-2-phenyl-1,2,3,4-tetrahydronaphthalen-1-yl)phenyl)piperidin-4-yl)(methyl)amino)methyl)phenyl)amino)piperidine-2,6-dione;
3-((2-(((1-(4-(6-hydroxy-2-phenyl-1,2,3,4-tetrahydronaphthalen-1-yl)phenyl)piperidin-4-yl)(methyl)amino)methyl)phenyl)amino)piperidine-2,6-dione;
3-(4-((4-(1-(4-(6-hydroxy-2-phenyl-1,2,3,4-tetrahydronaphthalen-1-yl)phenyl)piperidin-4-yl)piperazin-1-yl)methyl)phenyl)piperidine-2,6-dione;
3-(4-((4-(1-(4-((1R,2S)-6-hydroxy-2-phenyl-1,2,3,4-tetrahydronaphthalen-1-yl)phenyl)piperidin-4-yl)piperazin-1-yl)methyl)phenyl)piperidine-2,6-dione;
3-(4-((4-(1-(4-((1S,2R)-6-hydroxy-2-phenyl-1,2,3,4-tetrahydronaphthalen-1-yl)phenyl)piperidin-4-yl)piperazin-1-yl)methyl)phenyl)piperidine-2,6-dione;
3-(2-fluoro-4-((4-(1-(4-((1R,2S)-6-hydroxy-2-phenyl-1,2,3,4-tetrahydronaphthalen-1-yl)phenyl)piperidin-4-yl)piperazin-1-yl)methyl)phenyl)piperidine-2,6-dione;
3-(2-fluoro-4-((4-(1-(4-((1S,2R)-6-hydroxy-2-phenyl-1,2,3,4-tetrahydronaphthalen-1-yl)phenyl)piperidin-4-yl)piperazin-1-yl)methyl)phenyl)piperidine-2,6-dione;
1-(4-((4-(1-(4-((1R,2S)-6-hydroxy-2-phenyl-1,2,3,4-tetrahydronaphthalen-1-yl)phenyl)piperidin-4-yl)piperazin-1-yl)methyl)phenyl)dihydropyrimidine-2,4(1H,3H)-dione;
1-(4-((4-(1-(4-((1S,2R)-6-hydroxy-2-phenyl-1,2,3,4-tetrahydronaphthalen-1-yl)phenyl)piperidin-4-yl)piperazin-1-yl)methyl)phenyl)dihydropyrimidine-2,4(1H,3H)-dione;
1-(3-fluoro-4-((4-(1-(4-((1R,2S)-6-hydroxy-2-phenyl-1,2,3,4-tetrahydronaphthalen-1-yl)phenyl)piperidin-4-yl)piperazin-1-yl)methyl)phenyl)dihydropyrimidine-2,4(1H,3H)-dione;
1-(3-fluoro-4-((4-(1-(4-((1S,2R)-6-hydroxy-2-phenyl-1,2,3,4-tetrahydronaphthalen-1-yl)phenyl)piperidin-4-yl)piperazin-1-yl)methyl)phenyl)dihydropyrimidine-2,4(1H,3H)-dione;
1-(2-fluoro-4-((4-(1-(4-((1R,2S)-6-hydroxy-2-phenyl-1,2,3,4-tetrahydronaphthalen-1-yl)phenyl)piperidin-4-yl)piperazin-1-yl)methyl)phenyl)dihydropyrimidine-2,4(1H,3H)-dione;
1-(2-fluoro-4-((4-(1-(4-((1S,2R)-6-hydroxy-2-phenyl-1,2,3,4-tetrahydronaphthalen-1-yl)phenyl)piperidin-4-yl)piperazin-1-yl)methyl)phenyl)dihydropyrimidine-2,4(1H,3H)-dione;
1-(3-((4-(1-(4-((1R,2S)-6-hydroxy-2-phenyl-1,2,3,4-tetrahydronaphthalen-1-yl)phenyl)piperidin-4-yl)piperazin-1-yl)methyl)phenyl)dihydropyrimidine-2,4(1H,3H)-dione;
1-(3-((4-(1-(4-((1S,2R)-6-hydroxy-2-phenyl-1,2,3,4-tetrahydronaphthalen-1-yl)phenyl)piperidin-4-yl)piperazin-1-yl)methyl)phenyl)dihydropyrimidine-2,4(1H,3H)-dione;
3-((3-((4-(1-(4-((1R,2S)-6-hydroxy-2-phenyl-1,2,3,4-tetrahydronaphthalen-1-yl)phenyl)piperidin-4-yl)piperazin-1-yl)methyl)phenyl)amino)piperidine-2,6-dione;
3-((3-((4-(1-(4-((1S,2R)-6-hydroxy-2-phenyl-1,2,3,4-tetrahydronaphthalen-1-yl)phenyl)piperidin-4-yl)piperazin-1-yl)methyl)phenyl)amino)piperidine-2,6-dione;
3-(2-fluoro-4-((4-(1-(4-(6-hydroxy-2-phenyl-1,2,3,4-tetrahydronaphthalen-1-yl)phenyl)piperidin-4-yl)piperazin-1-yl)methyl)phenyl)piperidine-2,6-dione;
3-(3-((4-(1-(4-(6-hydroxy-2-phenyl-1,2,3,4-tetrahydronaphthalen-1-yl)phenyl)piperidin-4-yl)piperazin-1-yl)methyl)phenyl)piperidine-2,6-dione;
3-(2-((4-(1-(4-(6-hydroxy-2-phenyl-1,2,3,4-tetrahydronaphthalen-1-yl)phenyl)piperidin-4-yl)piperazin-1-yl)methyl)phenyl)piperidine-2,6-dione;
1-(4-((4-(1-(4-(6-hydroxy-2-phenyl-1,2,3,4-tetrahydronaphthalen-1-yl)phenyl)piperidin-4-yl)piperazin-1-yl)methyl)phenyl)dihydropyrimidine-2,4(1H,3H)-dione;
1-(3-fluoro-4-((4-(1-(4-(6-hydroxy-2-phenyl-1,2,3,4-tetrahydronaphthalen-1-yl)phenyl)piperidin-4-yl)piperazin-1-yl)methyl)phenyl)dihydropyrimidine-2,4(1H,3H)-dione;
1-(3-((4-(1-(4-(6-hydroxy-2-phenyl-1,2,3,4-tetrahydronaphthalen-1-yl)phenyl)piperidin-4-yl)piperazin-1-yl)methyl)phenyl)dihydropyrimidine-2,4(1H,3H)-dione;
1-(2-((4-(1-(4-(6-hydroxy-2-phenyl-1,2,3,4-tetrahydronaphthalen-1-yl)phenyl)piperidin-4-yl)piperazin-1-yl)methyl)phenyl)dihydropyrimidine-2,4(1H,3H)-dione;
1-(6-((4-(1-(4-(6-hydroxy-2-phenyl-1,2,3,4-tetrahydronaphthalen-1-yl)phenyl)piperidin-4-yl)piperazin-1-yl)methyl)pyridin-3-yl)dihydropyrimidine-2,4(1H,3H)-dione;
1-(6-((4-(1-(4-(6-hydroxy-2-phenyl-1,2,3,4-tetrahydronaphthalen-1-yl)phenyl)piperidin-4-yl)piperazin-1-yl)methyl)pyridazin-3-yl)dihydropyrimidine-2,4(1H,3H)-dione;
1-(4-((4-(1-(4-(6-hydroxy-2-phenyl-1,2,3,4-tetrahydronaphthalen-1-yl)phenyl)piperidin-4-yl)piperazin-1-yl)methyl)pyridin-3-yl)dihydropyrimidine-2,4(1H,3H)-dione;
3-((4-((4-(1-(4-(6-hydroxy-2-phenyl-1,2,3,4-tetrahydronaphthalen-1-yl)phenyl)piperidin-4-yl)piperazin-1-yl)methyl)phenyl)amino)piperidine-2,6-dione;
3-((3-((4-(1-(4-(6-hydroxy-2-phenyl-1,2,3,4-tetrahydronaphthalen-1-yl)phenyl)piperidin-4-yl)piperazin-1-yl)methyl)phenyl)amino)piperidine-2,6-dione;
3-((2-((4-(1-(4-(6-hydroxy-2-phenyl-1,2,3,4-tetrahydronaphthalen-1-yl)phenyl)piperidin-4-yl)piperazin-1-yl)methyl)phenyl)amino)piperidine-2,6-dione;
3-(4-((4-(4-((3S,4R)-7-hydroxy-3-phenylchroman-4-yl)phenyl)piperazin-1-yl)methyl)phenyl)piperidine-2,6-dione;
3-(4-((4-(4-((3R,4S)-7-hydroxy-3-phenylchroman-4-yl)phenyl)piperazin-1-yl)methyl)phenyl)piperidine-2,6-dione;
3-(2-fluoro-4-((4-(4-((3S,4R)-7-hydroxy-3-phenylchroman-4-yl)phenyl)piperazin-1-yl)methyl)phenyl)piperidine-2,6-dione;
3-(2-fluoro-4-((4-(4-((3R,4S)-7-hydroxy-3-phenylchroman-4-yl)phenyl)piperazin-1-yl)methyl)phenyl)piperidine-2,6-dione;
3-(3-((4-(4-((3S,4R)-7-hydroxy-3-phenylchroman-4-yl)phenyl)piperazin-1-yl)methyl)phenyl)piperidine-2,6-dione;
3-(3-((4-(4-((3R,4S)-7-hydroxy-3-phenylchroman-4-yl)phenyl)piperazin-1-yl)methyl)phenyl)piperidine-2,6-dione;
3-(2-((4-(4-((3S,4R)-7-hydroxy-3-phenylchroman-4-yl)phenyl)piperazin-1-yl)methyl)phenyl)piperidine-2,6-dione;
3-(2-((4-(4-((3R,4S)-7-hydroxy-3-phenylchroman-4-yl)phenyl)piperazin-1-yl)methyl)phenyl)piperidine-2,6-dione;
1-(4-((4-(4-((3S,4R)-7-hydroxy-3-phenylchroman-4-yl)phenyl)piperazin-1-yl)methyl)phenyl)dihydropyrimidine-2,4(1H,3H)-dione;
1-(4-((4-(4-((3R,4S)-7-hydroxy-3-phenylchroman-4-yl)phenyl)piperazin-1-yl)methyl)phenyl)dihydropyrimidine-2,4(1H,3H)-dione;
1-(3-fluoro-4-((4-(4-((3S,4R)-7-hydroxy-3-phenylchroman-4-yl)phenyl)piperazin-1-yl)methyl)phenyl)dihydropyrimidine-2,4(1H,3H)-dione;
1-(3-fluoro-4-((4-(4-((3R,4S)-7-hydroxy-3-phenylchroman-4-yl)phenyl)piperazin-1-yl)methyl)phenyl)dihydropyrimidine-2,4(1H,3H)-dione;
1-(3-((4-(4-((3S,4R)-7-hydroxy-3-phenylchroman-4-yl)phenyl)piperazin-1-yl)methyl)phenyl)dihydropyrimidine-2,4(1H,3H)-dione;
1-(3-((4-(4-((3R,4S)-7-hydroxy-3-phenylchroman-4-yl)phenyl)piperazin-1-yl)methyl)phenyl)dihydropyrimidine-2,4(1H,3H)-dione;
1-(2-((4-(4-((3S,4R)-7-hydroxy-3-phenylchroman-4-yl)phenyl)piperazin-1-yl)methyl)phenyl)dihydropyrimidine-2,4(1H,3H)-dione;
1-(2-((4-(4-((3R,4S)-7-hydroxy-3-phenylchroman-4-yl)phenyl)piperazin-1-yl)methyl)phenyl)dihydropyrimidine-2,4(1H,3H)-dione;
1-(6-((4-(4-((3S,4R)-7-hydroxy-3-phenylchroman-4-yl)phenyl)piperazin-1-yl)methyl)pyridin-3-yl)dihydropyrimidine-2,4(1H,3H)-dione;
1-(6-((4-(4-((3R,4S)-7-hydroxy-3-phenylchroman-4-yl)phenyl)piperazin-1-yl)methyl)pyridin-3-yl)dihydropyrimidine-2,4(1H,3H)-dione;
1-(6-((4-(4-((3S,4R)-7-hydroxy-3-phenylchroman-4-yl)phenyl)piperazin-1-yl)methyl)pyridazin-3-yl)dihydropyrimidine-2,4(1H,3H)-dione;
1-(6-((4-(4-((3R,4S)-7-hydroxy-3-phenylchroman-4-yl)phenyl)piperazin-1-yl)methyl)pyridazin-3-yl)dihydropyrimidine-2,4(1H,3H)-dione;
1-(4-((4-(4-((3S,4R)-7-hydroxy-3-phenylchroman-4-yl)phenyl)piperazin-1-yl)methyl)pyridin-3-yl)dihydropyrimidine-2,4(1H,3H)-dione;
1-(4-((4-(4-((3R,4S)-7-hydroxy-3-phenylchroman-4-yl)phenyl)piperazin-1-yl)methyl)pyridin-3-yl)dihydropyrimidine-2,4(1H,3H)-dione;
3-((4-((4-(4-((3S,4R)-7-hydroxy-3-phenylchroman-4-yl)phenyl)piperazin-1-yl)methyl)phenyl)amino)piperidine-2,6-dione;
3-((4-((4-(4-((3R,4S)-7-hydroxy-3-phenylchroman-4-yl)phenyl)piperazin-1-yl)methyl)phenyl)amino)piperidine-2,6-dione;
3-((3-((4-(4-((3S,4R)-7-hydroxy-3-phenylchroman-4-yl)phenyl)piperazin-1-yl)methyl)phenyl)amino)piperidine-2,6-dione;
3-((3-((4-(4-((3R,4S)-7-hydroxy-3-phenylchroman-4-yl)phenyl)piperazin-1-yl)methyl)phenyl)amino)piperidine-2,6-dione;
3-((2-((4-(4-((3S,4R)-7-hydroxy-3-phenylchroman-4-yl)phenyl)piperazin-1-yl)methyl)phenyl)amino)piperidine-2,6-dione;
3-((2-((4-(4-((3R,4S)-7-hydroxy-3-phenylchroman-4-yl)phenyl)piperazin-1-yl)methyl)phenyl)amino)piperidine-2,6-dione;
3-(4-(((1-(4-(7-hydroxy-3-phenylchroman-4-yl)phenyl)piperidin-4-yl)(methyl)amino)methyl)phenyl)piperidine-2,6-dione;
3-(2-fluoro-4-(((1-(4-(7-hydroxy-3-phenylchroman-4-yl)phenyl)piperidin-4-yl)(methyl)amino)methyl)phenyl)piperidine-2,6-dione;
3-(3-(((1-(4-(7-hydroxy-3-phenylchroman-4-yl)phenyl)piperidin-4-yl)(methyl)amino)methyl)phenyl)piperidine-2,6-dione;
3-(2-(((1-(4-(7-hydroxy-3-phenylchroman-4-yl)phenyl)piperidin-4-yl)(methyl)amino)methyl)phenyl)piperidine-2,6-dione;
1-(4-(((1-(4-(7-hydroxy-3-phenylchroman-4-yl)phenyl)piperidin-4-yl)(methyl)amino)methyl)phenyl)dihydropyrimidine-2,4(1H,3H)-dione;
1-(3-fluoro-4-(((1-(4-(7-hydroxy-3-phenylchroman-4-yl)phenyl)piperidin-4-yl)(methyl)amino)methyl)phenyl)dihydropyrimidine-2,4(1H,3H)-dione;
1-(3-(((1-(4-(7-hydroxy-3-phenylchroman-4-yl)phenyl)piperidin-4-yl)(methyl)amino)methyl)phenyl)dihydropyrimidine-2,4(1H,3H)-dione;
1-(2-(((1-(4-(7-hydroxy-3-phenylchroman-4-yl)phenyl)piperidin-4-yl)(methyl)amino)methyl)phenyl)dihydropyrimidine-2,4(1H,3H)-dione;
1-(6-(((1-(4-(7-hydroxy-3-phenylchroman-4-yl)phenyl)piperidin-4-yl)(methyl)amino)methyl)pyridin-3-yl)dihydropyrimidine-2,4(1H,3H)-dione;
1-(6-(((1-(4-(7-hydroxy-3-phenylchroman-4-yl)phenyl)piperidin-4-yl)(methyl)amino)methyl)pyridazin-3-yl)dihydropyrimidine-2,4(1H,3H)-dione;
1-(4-(((1-(4-(7-hydroxy-3-phenylchroman-4-yl)phenyl)piperidin-4-yl)(methyl)amino)methyl)pyridin-3-yl)dihydropyrimidine-2,4(1H,3H)-dione;
3-((4-(((1-(4-(7-hydroxy-3-phenylchroman-4-yl)phenyl)piperidin-4-yl)(methyl)amino)methyl)phenyl)amino)piperidine-2,6-dione;
3-((3-(((1-(4-(7-hydroxy-3-phenylchroman-4-yl)phenyl)piperidin-4-yl)(methyl)amino)methyl)phenyl)amino)piperidine-2,6-dione;
3-((2-(((1-(4-(7-hydroxy-3-phenylchroman-4-yl)phenyl)piperidin-4-yl)(methyl)amino)methyl)phenyl)amino)piperidine-2,6-dione;
3-(4-((4-(1-(4-(7-hydroxy-3-phenylchroman-4-yl)phenyl)piperidin-4-yl)piperazin-1-yl)methyl)phenyl)piperidine-2,6-dione;
3-(2-fluoro-4-((4-(1-(4-(7-hydroxy-3-phenylchroman-4-yl)phenyl)piperidin-4-yl)piperazin-1-yl)methyl)phenyl)piperidine-2,6-dione;
3-(3-((4-(1-(4-(7-hydroxy-3-phenylchroman-4-yl)phenyl)piperidin-4-yl)piperazin-1-yl)methyl)phenyl)piperidine-2,6-dione;
3-(2-((4-(1-(4-(7-hydroxy-3-phenylchroman-4-yl)phenyl)piperidin-4-yl)piperazin-1-yl)methyl)phenyl)piperidine-2,6-dione;
1-(4-((4-(1-(4-(7-hydroxy-3-phenylchroman-4-yl)phenyl)piperidin-4-yl)piperazin-1-yl)methyl)phenyl)dihydropyrimidine-2,4(1H,3H)-dione;
1-(3-fluoro-4-((4-(1-(4-(7-hydroxy-3-phenylchroman-4-yl)phenyl)piperidin-4-yl)piperazin-1-yl)methyl)phenyl)dihydropyrimidine-2,4(1H,3H)-dione;
1-(3-((4-(1-(4-(7-hydroxy-3-phenylchroman-4-yl)phenyl)piperidin-4-yl)piperazin-1-yl)methyl)phenyl)dihydropyrimidine-2,4(1H,3H)-dione;
1-(2-((4-(1-(4-(7-hydroxy-3-phenylchroman-4-yl)phenyl)piperidin-4-yl)piperazin-1-yl)methyl)phenyl)dihydropyrimidine-2,4(1H,3H)-dione;
1-(6-((4-(1-(4-(7-hydroxy-3-phenylchroman-4-yl)phenyl)piperidin-4-yl)piperazin-1-yl)methyl)pyridin-3-yl)dihydropyrimidine-2,4(1H,3H)-dione;
1-(6-((4-(1-(4-(7-hydroxy-3-phenylchroman-4-yl)phenyl)piperidin-4-yl)piperazin-1-yl)methyl)pyridazin-3-yl)dihydropyrimidine-2,4(1H,3H)-dione;
1-(4-((4-(1-(4-(7-hydroxy-3-phenylchroman-4-yl)phenyl)piperidin-4-yl)piperazin-1-yl)methyl)pyridin-3-yl)dihydropyrimidine-2,4(1H,3H)-dione;
3-((4-((4-(1-(4-(7-hydroxy-3-phenylchroman-4-yl)phenyl)piperidin-4-yl)piperazin-1-yl)methyl)phenyl)amino)piperidine-2,6-dione;
3-((3-((4-(1-(4-(7-hydroxy-3-phenylchroman-4-yl)phenyl)piperidin-4-yl)piperazin-1-yl)methyl)phenyl)amino)piperidine-2,6-dione;
3-((2-((4-(1-(4-(7-hydroxy-3-phenylchroman-4-yl)phenyl)piperidin-4-yl)piperazin-1-yl)methyl)phenyl)amino)piperidine-2,6-dione;
3-(4-(((1-(3-(2,3-dichlorophenyl)-1H-pyrazolo[3,4-b]pyrazin-6-yl)-4-methylpiperidin-4-yl)amino)methyl)phenyl)piperidine-2,6-dione;
3-(4-(((1-(3-(2,3-dichlorophenyl)-1H-pyrazolo[3,4-b]pyrazin-6-yl)-4-methylpiperidin-4-yl)amino)methyl)-2-fluorophenyl)piperidine-2,6-dione;
3-(3-(((1-(3-(2,3-dichlorophenyl)-1H-pyrazolo[3,4-b]pyrazin-6-yl)-4-methylpiperidin-4-yl)amino)methyl)phenyl)piperidine-2,6-dione;
3-(2-(((1-(3-(2,3-dichlorophenyl)-1H-pyrazolo[3,4-b]pyrazin-6-yl)-4-methylpiperidin-4-yl)amino)methyl)phenyl)piperidine-2,6-dione;
1-(4-(((1-(3-(2,3-dichlorophenyl)-1H-pyrazolo[3,4-b]pyrazin-6-yl)-4-methylpiperidin-4-yl)amino)methyl)phenyl)dihydropyrimidine-2,4(1H,3H)-dione;
1-(4-(((1-(3-(2,3-dichlorophenyl)-1H-pyrazolo[3,4-b]pyrazin-6-yl)-4-methylpiperidin-4-yl)amino)methyl)-2-fluorophenyl)dihydropyrimidine-2,4(1H,3H)-dione;
1-(3-(((1-(3-(2,3-dichlorophenyl)-1H-pyrazolo[3,4-b]pyrazin-6-yl)-4-methylpiperidin-4-yl)amino)methyl)phenyl)dihydropyrimidine-2,4(1H,3H)-dione;
1-(2-(((1-(3-(2,3-dichlorophenyl)-1H-pyrazolo[3,4-b]pyrazin-6-yl)-4-methylpiperidin-4-yl)amino)methyl)phenyl)dihydropyrimidine-2,4(1H,3H)-dione;
1-(6-(((1-(3-(2,3-dichlorophenyl)-1H-pyrazolo[3,4-b]pyrazin-6-yl)-4-methylpiperidin-4-yl)amino)methyl)pyridin-3-yl)dihydropyrimidine-2,4(1H,3H)-dione;
1-(6-(((1-(3-(2,3-dichlorophenyl)-1H-pyrazolo[3,4-b]pyrazin-6-yl)-4-methylpiperidin-4-yl)amino)methyl)pyridazin-3-yl)dihydropyrimidine-2,4(1H,3H)-dione;
1-(4-(((1-(3-(2,3-dichlorophenyl)-1H-pyrazolo[3,4-b]pyrazin-6-yl)-4-methylpiperidin-4-yl)amino)methyl)pyridin-3-yl)dihydropyrimidine-2,4(1H,3H)-dione;
3-((4-(((1-(3-(2,3-dichlorophenyl)-1H-pyrazolo[3,4-b]pyrazin-6-yl)-4-methylpiperidin-4-yl)amino)methyl)phenyl)amino)piperidine-2,6-dione;
3-((3-(((1-(3-(2,3-dichlorophenyl)-1H-pyrazolo[3,4-b]pyrazin-6-yl)-4-methylpiperidin-4-yl)amino)methyl)phenyl)amino)piperidine-2,6-dione;
3-((2-(((1-(3-(2,3-dichlorophenyl)-1H-pyrazolo[3,4-b]pyrazin-6-yl)-4-methylpiperidin-4-yl)amino)methyl)phenyl)amino)piperidine-2,6-dione;
3-(4-((4-(3-(6-(4-amino-4-methylpiperidin-1-yl)-1H-pyrazolo[3,4-b]pyrazin-3-yl)-2-chlorophenyl)piperazin-1-yl)methyl)phenyl)piperidine-2,6-dione;
3-(4-((4-(3-(6-(4-amino-4-methylpiperidin-1-yl)-1H-pyrazolo[3,4-b]pyrazin-3-yl)-2-chlorophenyl)piperazin-1-yl)methyl)-2-fluorophenyl)piperidine-2,6-dione;
3-(3-((4-(3-(6-(4-amino-4-methylpiperidin-1-yl)-1H-pyrazolo[3,4-b]pyrazin-3-yl)-2-chlorophenyl)piperazin-1-yl)methyl)phenyl)piperidine-2,6-dione;
3-(2-((4-(3-(6-(4-amino-4-methylpiperidin-1-yl)-1H-pyrazolo[3,4-b]pyrazin-3-yl)-2-chlorophenyl)piperazin-1-yl)methyl)phenyl)piperidine-2,6-dione;
1-(4-((4-(3-(6-(4-amino-4-methylpiperidin-1-yl)-1H-pyrazolo[3,4-b]pyrazin-3-yl)-2-chlorophenyl)piperazin-1-yl)methyl)phenyl)dihydropyrimidine-2,4(1H,3H)-dione;
1-(4-((4-(3-(6-(4-amino-4-methylpiperidin-1-yl)-1H-pyrazolo[3,4-b]pyrazin-3-yl)-2-chlorophenyl)piperazin-1-yl)methyl)-3-fluorophenyl)dihydropyrimidine-2,4(1H,3H)-dione;
1-(3-((4-(3-(6-(4-amino-4-methylpiperidin-1-yl)-1H-pyrazolo[3,4-b]pyrazin-3-yl)-2-chlorophenyl)piperazin-1-yl)methyl)phenyl)dihydropyrimidine-2,4(1H,3H)-dione;
1-(2-((4-(3-(6-(4-amino-4-methylpiperidin-1-yl)-1H-pyrazolo[3,4-b]pyrazin-3-yl)-2-chlorophenyl)piperazin-1-yl)methyl)phenyl)dihydropyrimidine-2,4(1H,3H)-dione;
1-(6-((4-(3-(6-(4-amino-4-methylpiperidin-1-yl)-1H-pyrazolo[3,4-b]pyrazin-3-yl)-2-chlorophenyl)piperazin-1-yl)methyl)pyridin-3-yl)dihydropyrimidine-2,4(1H,3H)-dione;
1-(6-((4-(3-(6-(4-amino-4-methylpiperidin-1-yl)-1H-pyrazolo[3,4-b]pyrazin-3-yl)-2-chlorophenyl)piperazin-1-yl)methyl)pyridazin-3-yl)dihydropyrimidine-2,4(1H,3H)-dione;
1-(4-((4-(3-(6-(4-amino-4-methylpiperidin-1-yl)-1H-pyrazolo[3,4-b]pyrazin-3-yl)-2-chlorophenyl)piperazin-1-yl)methyl)pyridin-3-yl)dihydropyrimidine-2,4(1H,3H)-dione;
3-((4-((4-(3-(6-(4-amino-4-methylpiperidin-1-yl)-1H-pyrazolo[3,4-b]pyrazin-3-yl)-2-chlorophenyl)piperazin-1-yl)methyl)phenyl)amino)piperidine-2,6-dione;
3-((3-((4-(3-(6-(4-amino-4-methylpiperidin-1-yl)-1H-pyrazolo[3,4-b]pyrazin-3-yl)-2-chlorophenyl)piperazin-1-yl)methyl)phenyl)amino)piperidine-2,6-dione;
3-((2-((4-(3-(6-(4-amino-4-methylpiperidin-1-yl)-1H-pyrazolo[3,4-b]pyrazin-3-yl)-2-chlorophenyl)piperazin-1-yl)methyl)phenyl)amino)piperidine-2,6-dione;
3-(4-((4-(3-((3-amino-5-(4-amino-4-methylpiperidin-1-yl)pyrazin-2-yl)thio)-2-chlorophenyl)piperazin-1-yl)methyl)phenyl)piperidine-2,6-dione;
3-(4-((4-(3-((3-amino-5-(4-amino-4-methylpiperidin-1-yl)pyrazin-2-yl)thio)-2-chlorophenyl)piperazin-1-yl)methyl)-2-fluorophenyl)piperidine-2,6-dione;
3-(3-((4-(3-((3-amino-5-(4-amino-4-methylpiperidin-1-yl)pyrazin-2-yl)thio)-2-chlorophenyl)piperazin-1-yl)methyl)phenyl)piperidine-2,6-dione;
3-(2-((4-(3-((3-amino-5-(4-amino-4-methylpiperidin-1-yl)pyrazin-2-yl)thio)-2-chlorophenyl)piperazin-1-yl)methyl)phenyl)piperidine-2,6-dione;
1-(4-((4-(3-((3-amino-5-(4-amino-4-methylpiperidin-1-yl)pyrazin-2-yl)thio)-2-chlorophenyl)piperazin-1-yl)methyl)phenyl)dihydropyrimidine-2,4(1H,3H)-dione;
1-(4-((4-(3-((3-amino-5-(4-amino-4-methylpiperidin-1-yl)pyrazin-2-yl)thio)-2-chlorophenyl)piperazin-1-yl)methyl)-3-fluorophenyl)dihydropyrimidine-2,4(1H,3H)-dione;
1-(3-((4-(3-((3-amino-5-(4-amino-4-methylpiperidin-1-yl)pyrazin-2-yl)thio)-2-chlorophenyl)piperazin-1-yl)methyl)phenyl)dihydropyrimidine-2,4(1H,3H)-dione;
1-(2-((4-(3-((3-amino-5-(4-amino-4-methylpiperidin-1-yl)pyrazin-2-yl)thio)-2-chlorophenyl)piperazin-1-yl)methyl)phenyl)dihydropyrimidine-2,4(1H,3H)-dione;
1-(6-((4-(3-((3-amino-5-(4-amino-4-methylpiperidin-1-yl)pyrazin-2-yl)thio)-2-chlorophenyl)piperazin-1-yl)methyl)pyridin-3-yl)dihydropyrimidine-2,4(1H,3H)-dione;
1-(6-((4-(3-((3-amino-5-(4-amino-4-methylpiperidin-1-yl)pyrazin-2-yl)thio)-2-chlorophenyl)piperazin-1-yl)methyl)pyridazin-3-yl)dihydropyrimidine-2,4(1H,3H)-dione;
1-(4-((4-(3-((3-amino-5-(4-amino-4-methylpiperidin-1-yl)pyrazin-2-yl)thio)-2-chlorophenyl)piperazin-1-yl)methyl)pyridin-3-yl)dihydropyrimidine-2,4(1H,3H)-dione;
3-((4-((4-(3-((3-amino-5-(4-amino-4-methylpiperidin-1-yl)pyrazin-2-yl)thio)-2-chlorophenyl)piperazin-1-yl)methyl)phenyl)amino)piperidine-2,6-dione;
3-((3-((4-(3-((3-amino-5-(4-amino-4-methylpiperidin-1-yl)pyrazin-2-yl)thio)-2-chlorophenyl)piperazin-1-yl)methyl)phenyl)amino)piperidine-2,6-dione;
3-((2-((4-(3-((3-amino-5-(4-amino-4-methylpiperidin-1-yl)pyrazin-2-yl)thio)-2-chlorophenyl)piperazin-1-yl)methyl)phenyl)amino)piperidine-2,6-dione;
3-(4-(((3-((3-amino-5-(4-amino-4-methylpiperidin-1-yl)pyrazin-2-yl)thio)-2-chlorophenyl)amino)methyl)phenyl)piperidine-2,6-dione;
3-(4-(((3-((3-amino-5-(4-amino-4-methylpiperidin-1-yl)pyrazin-2-yl)thio)-2-chlorophenyl)amino)methyl)-2-fluorophenyl)piperidine-2,6-dione;
3-(3-(((3-((3-amino-5-(4-amino-4-methylpiperidin-1-yl)pyrazin-2-yl)thio)-2-chlorophenyl)amino)methyl)phenyl)piperidine-2,6-dione;
3-(2-(((3-((3-amino-5-(4-amino-4-methylpiperidin-1-yl)pyrazin-2-yl)thio)-2-chlorophenyl)amino)methyl)phenyl)piperidine-2,6-dione;
1-(4-(((3-((3-amino-5-(4-amino-4-methylpiperidin-1-yl)pyrazin-2-yl)thio)-2-chlorophenyl)amino)methyl)phenyl)dihydropyrimidine-2,4(1H,3H)-dione;
1-(4-(((3-((3-amino-5-(4-amino-4-methylpiperidin-1-yl)pyrazin-2-yl)thio)-2-chlorophenyl)amino)methyl)-3-fluorophenyl)dihydropyrimidine-2,4(1H,3H)-dione;
1-(3-(((3-((3-amino-5-(4-amino-4-methylpiperidin-1-yl)pyrazin-2-yl)thio)-2-chlorophenyl)amino)methyl)phenyl)dihydropyrimidine-2,4(1H,3H)-dione;
1-(2-(((3-((3-amino-5-(4-amino-4-methylpiperidin-1-yl)pyrazin-2-yl)thio)-2-chlorophenyl)amino)methyl)phenyl)dihydropyrimidine-2,4(1H,3H)-dione;
1-(6-(((3-((3-amino-5-(4-amino-4-methylpiperidin-1-yl)pyrazin-2-yl)thio)-2-chlorophenyl)amino)methyl)pyridin-3-yl)dihydropyrimidine-2,4(1H,3H)-dione;
1-(6-(((3-((3-amino-5-(4-amino-4-methylpiperidin-1-yl)pyrazin-2-yl)thio)-2-chlorophenyl)amino)methyl)pyridazin-3-yl)dihydropyrimidine-2,4(1H,3H)-dione;
1-(4-(((3-((3-amino-5-(4-amino-4-methylpiperidin-1-yl)pyrazin-2-yl)thio)-2-chlorophenyl)amino)methyl)pyridin-3-yl)dihydropyrimidine-2,4(1H,3H)-dione;
3-((4-(((3-((3-amino-5-(4-amino-4-methylpiperidin-1-yl)pyrazin-2-yl)thio)-2-chlorophenyl)amino)methyl)phenyl)amino)piperidine-2,6-dione;
3-((3-(((3-((3-amino-5-(4-amino-4-methylpiperidin-1-yl)pyrazin-2-yl)thio)-2-chlorophenyl)amino)methyl)phenyl)amino)piperidine-2,6-dione;
3-((2-(((3-((3-amino-5-(4-amino-4-methylpiperidin-1-yl)pyrazin-2-yl)thio)-2-chlorophenyl)amino)methyl)phenyl)amino)piperidine-2,6-dione;
3-(4-((4-(3-(3-amino-5-(4-amino-4-methylpiperidin-1-yl)pyrazin-2-yl)-2-chlorophenyl)piperazin-1-yl)methyl)phenyl)piperidine-2,6-dione;
3-(4-((4-(3-(3-amino-5-(4-amino-4-methylpiperidin-1-yl)pyrazin-2-yl)-2-chlorophenyl)piperazin-1-yl)methyl)-2-fluorophenyl)piperidine-2,6-dione;
3-(3-((4-(3-(3-amino-5-(4-amino-4-methylpiperidin-1-yl)pyrazin-2-yl)-2-chlorophenyl)piperazin-1-yl)methyl)phenyl)piperidine-2,6-dione;
3-(2-((4-(3-(3-amino-5-(4-amino-4-methylpiperidin-1-yl)pyrazin-2-yl)-2-chlorophenyl)piperazin-1-yl)methyl)phenyl)piperidine-2,6-dione;
1-(4-((4-(3-(3-amino-5-(4-amino-4-methylpiperidin-1-yl)pyrazin-2-yl)-2-chlorophenyl)piperazin-1-yl)methyl)phenyl)dihydropyrimidine-2,4(1H,3H)-dione;
1-(4-((4-(3-(3-amino-5-(4-amino-4-methylpiperidin-1-yl)pyrazin-2-yl)-2-chlorophenyl)piperazin-1-yl)methyl)-3-fluorophenyl)dihydropyrimidine-2,4(1H,3H)-dione;
1-(3-((4-(3-(3-amino-5-(4-amino-4-methylpiperidin-1-yl)pyrazin-2-yl)-2-chlorophenyl)piperazin-1-yl)methyl)phenyl)dihydropyrimidine-2,4(1H,3H)-dione;
1-(2-((4-(3-(3-amino-5-(4-amino-4-methylpiperidin-1-yl)pyrazin-2-yl)-2-chlorophenyl)piperazin-1-yl)methyl)phenyl)dihydropyrimidine-2,4(1H,3H)-dione;
1-(6-((4-(3-(3-amino-5-(4-amino-4-methylpiperidin-1-yl)pyrazin-2-yl)-2-chlorophenyl)piperazin-1-yl)methyl)pyridin-3-yl)dihydropyrimidine-2,4(1H,3H)-dione;
1-(6-((4-(3-(3-amino-5-(4-amino-4-methylpiperidin-1-yl)pyrazin-2-yl)-2-chlorophenyl)piperazin-1-yl)methyl)pyridazin-3-yl)dihydropyrimidine-2,4(1H,3H)-dione;
1-(4-((4-(3-(3-amino-5-(4-amino-4-methylpiperidin-1-yl)pyrazin-2-yl)-2-chlorophenyl)piperazin-1-yl)methyl)pyridin-3-yl)dihydropyrimidine-2,4(1H,3H)-dione;
3-((4-((4-(3-(3-amino-5-(4-amino-4-methylpiperidin-1-yl)pyrazin-2-yl)-2-chlorophenyl)piperazin-1-yl)methyl)phenyl)amino)piperidine-2,6-dione;
3-((3-((4-(3-(3-amino-5-(4-amino-4-methylpiperidin-1-yl)pyrazin-2-yl)-2-chlorophenyl)piperazin-1-yl)methyl)phenyl)amino)piperidine-2,6-dione;
3-((2-((4-(3-(3-amino-5-(4-amino-4-methylpiperidin-1-yl)pyrazin-2-yl)-2-chlorophenyl)piperazin-1-yl)methyl)phenyl)amino)piperidine-2,6-dione;
3-(4-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)phenyl)piperidine-2,6-dione;
3-(4-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)-2-fluorophenyl)piperidine-2,6-dione;
3-(3-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)phenyl)piperidine-2,6-dione;
3-(2-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)phenyl)piperidine-2,6-dione;
1-(4-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)phenyl)dihydropyrimidine-2,4(1H,3H)-dione;
1-(4-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)-2-fluorophenyl)dihydropyrimidine-2,4(1H,3H)-dione;
1-(3-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)phenyl)dihydropyrimidine-2,4(1H,3H)-dione;
1-(2-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)phenyl)dihydropyrimidine-2,4(1H,3H)-dione;
1-(6-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)pyridin-3-yl)dihydropyrimidine-2,4(1H,3H)-dione;
1-(6-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)pyridazin-3-yl)dihydropyrimidine-2,4(1H,3H)-dione;
1-(4-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)pyridin-3-yl)dihydropyrimidine-2,4(1H,3H)-dione;
3-((4-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)phenyl)amino)piperidine-2,6-dione;
3-((3-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)phenyl)amino)piperidine-2,6-dione;
3-((2-((((3S,4S)-8-(6-amino-5-((2-amino-3-chloropyridin-4-yl)thio)pyrazin-2-yl)-3-methyl-2-oxa-8-azaspiro[4.5]decan-4-yl)amino)methyl)phenyl)amino)piperidine-2,6-dione;
3-(4-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)phenyl)piperidine-2,6-dione;
3-(4-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)-2-fluorophenyl)piperidine-2,6-dione;
3-(3-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)phenyl)piperidine-2,6-dione;
3-(2-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)phenyl)piperidine-2,6-dione;
1-(4-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)phenyl)dihydropyrimidine-2,4(1H,3H)-dione;
1-(4-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)-3-fluorophenyl)dihydropyrimidine-2,4(1H,3H)-dione;
1-(3-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)phenyl)dihydropyrimidine-2,4(1H,3H)-dione;
1-(2-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)phenyl)dihydropyrimidine-2,4(1H,3H)-dione;
1-(6-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)pyridin-3-yl)dihydropyrimidine-2,4(1H,3H)-dione;
1-(6-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)pyridazin-3-yl)dihydropyrimidine-2,4(1H,3H)-dione;
1-(4-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)pyridin-3-yl)dihydropyrimidine-2,4(1H,3H)-dione;
3-((4-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)phenyl)amino)piperidine-2,6-dione;
3-((3-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)phenyl)amino)piperidine-2,6-dione;
3-((2-((4-(4-((3-amino-5-((3S,4S)-4-amino-3-methyl-2-oxa-8-azaspiro[4.5]decan-8-yl)pyrazin-2-yl)thio)pyridin-2-yl)piperazin-1-yl)methyl)phenyl)amino)piperidine-2,6-dione;
3-(4-(((1-(6-amino-5-(2,3-dichlorophenyl)pyrazin-2-yl)-4-methylpiperidin-4-yl)amino)methyl)phenyl)piperidine-2,6-dione;
3-(4-(((1-(6-amino-5-(2,3-dichlorophenyl)pyrazin-2-yl)-4-methylpiperidin-4-yl)amino)methyl)-2-fluorophenyl)piperidine-2,6-dione;
3-(3-(((1-(6-amino-5-(2,3-dichlorophenyl)pyrazin-2-yl)-4-methylpiperidin-4-yl)amino)methyl)phenyl)piperidine-2,6-dione;
3-(2-(((1-(6-amino-5-(2,3-dichlorophenyl)pyrazin-2-yl)-4-methylpiperidin-4-yl)amino)methyl)phenyl)piperidine-2,6-dione;
1-(4-(((1-(6-amino-5-(2,3-dichlorophenyl)pyrazin-2-yl)-4-methylpiperidin-4-yl)amino)methyl)phenyl)dihydropyrimidine-2,4(1H,3H)-dione;
1-(4-(((1-(6-amino-5-(2,3-dichlorophenyl)pyrazin-2-yl)-4-methylpiperidin-4-yl)amino)methyl)-2-fluorophenyl)dihydropyrimidine-2,4(1H,3H)-dione;
1-(3-(((1-(6-amino-5-(2,3-dichlorophenyl)pyrazin-2-yl)-4-methylpiperidin-4-yl)amino)methyl)phenyl)dihydropyrimidine-2,4(1H,3H)-dione;
1-(2-(((1-(6-amino-5-(2,3-dichlorophenyl)pyrazin-2-yl)-4-methylpiperidin-4-yl)amino)methyl)phenyl)dihydropyrimidine-2,4(1H,3H)-dione;
1-(6-(((1-(6-amino-5-(2,3-dichlorophenyl)pyrazin-2-yl)-4-methylpiperidin-4-yl)amino)methyl)pyridin-3-yl)dihydropyrimidine-2,4(1H,3H)-dione;
1-(6-(((1-(6-amino-5-(2,3-dichlorophenyl)pyrazin-2-yl)-4-methylpiperidin-4-yl)amino)methyl)pyridazin-3-yl)dihydropyrimidine-2,4(1H,3H)-dione;
1-(4-(((1-(6-amino-5-(2,3-dichlorophenyl)pyrazin-2-yl)-4-methylpiperidin-4-yl)amino)methyl)pyridin-3-yl)dihydropyrimidine-2,4(1H,3H)-dione;
3-((4-(((1-(6-amino-5-(2,3-dichlorophenyl)pyrazin-2-yl)-4-methylpiperidin-4-yl)amino)methyl)phenyl)amino)piperidine-2,6-dione;
3-((3-(((1-(6-amino-5-(2,3-dichlorophenyl)pyrazin-2-yl)-4-methylpiperidin-4-yl)amino)methyl)phenyl)amino)piperidine-2,6-dione;
3-((2-(((1-(6-amino-5-(2,3-dichlorophenyl)pyrazin-2-yl)-4-methylpiperidin-4-yl)amino)methyl)phenyl)amino)piperidine-2,6-dione;
3-(4-((2,3-difluoro-6-(2-morpholinothiazol-4-yl)phenoxy)methyl)phenyl)piperidine-2,6-dione;
3-(4-((2,3-difluoro-6-(2-morpholinothiazol-4-yl)phenoxy)methyl)-2-fluorophenyl)piperidine-2,6-dione;
3-(3-((2,3-difluoro-6-(2-morpholinothiazol-4-yl)phenoxy)methyl)phenyl)piperidine-2,6-dione;
3-(2-((2,3-difluoro-6-(2-morpholinothiazol-4-yl)phenoxy)methyl)phenyl)piperidine-2,6-dione;
1-(4-((2,3-difluoro-6-(2-morpholinothiazol-4-yl)phenoxy)methyl)phenyl)dihydropyrimidine-2,4(1H,3H)-dione;
1-(4-((2,3-difluoro-6-(2-morpholinothiazol-4-yl)phenoxy)methyl)-3-fluorophenyl)dihydropyrimidine-2,4(1H,3H)-dione;
1-(3-((2,3-difluoro-6-(2-morpholinothiazol-4-yl)phenoxy)methyl)phenyl)dihydropyrimidine-2,4(1H,3H)-dione;
1-(2-((2,3-difluoro-6-(2-morpholinothiazol-4-yl)phenoxy)methyl)phenyl)dihydropyrimidine-2,4(1H,3H)-dione;
1-(6-((2,3-difluoro-6-(2-morpholinothiazol-4-yl)phenoxy)methyl)pyridin-3-yl)dihydropyrimidine-2,4(1H,3H)-dione;
1-(6-((2,3-difluoro-6-(2-morpholinothiazol-4-yl)phenoxy)methyl)pyridazin-3-yl)dihydropyrimidine-2,4(1H,3H)-dione;
1-(4-((2,3-difluoro-6-(2-morpholinothiazol-4-yl)phenoxy)methyl)pyridin-3-yl)dihydropyrimidine-2,4(1H,3H)-dione;
3-((4-((2,3-difluoro-6-(2-morpholinothiazol-4-yl)phenoxy)methyl)phenyl)amino)piperidine-2,6-dione;
3-((3-((2,3-difluoro-6-(2-morpholinothiazol-4-yl)phenoxy)methyl)phenyl)amino)piperidine-2,6-dione;
3-((2-((2,3-difluoro-6-(2-morpholinothiazol-4-yl)phenoxy)methyl)phenyl)amino)piperidine-2,6-dione;
3-(4-((2-(6-methyl-7-oxo-6,7-dihydro-1H-pyrrolo[2,3-c]pyridin-4-yl)-4-(methylsulfonyl)phenoxy)methyl)phenyl)piperidine-2,6-dione;
3-(2-fluoro-4-((2-(6-methyl-7-oxo-6,7-dihydro-1H-pyrrolo[2,3-c]pyridin-4-yl)-4-(methylsulfonyl)phenoxy)methyl)phenyl)piperidine-2,6-dione;
3-(3-((2-(6-methyl-7-oxo-6,7-dihydro-1H-pyrrolo[2,3-c]pyridin-4-yl)-4-(methylsulfonyl)phenoxy)methyl)phenyl)piperidine-2,6-dione;
3-(2-((2-(6-methyl-7-oxo-6,7-dihydro-1H-pyrrolo[2,3-c]pyridin-4-yl)-4-(methylsulfonyl)phenoxy)methyl)phenyl)piperidine-2,6-dione;
1-(4-((2-(6-methyl-7-oxo-6,7-dihydro-1H-pyrrolo[2,3-c]pyridin-4-yl)-4-(methylsulfonyl)phenoxy)methyl)phenyl)dihydropyrimidine-2,4(1H,3H)-dione;
1-(2-fluoro-4-((2-(6-methyl-7-oxo-6,7-dihydro-1H-pyrrolo[2,3-c]pyridin-4-yl)-4-(methylsulfonyl)phenoxy)methyl)phenyl)dihydropyrimidine-2,4(1H,3H)-dione;
1-(3-fluoro-4-((2-(6-methyl-7-oxo-6,7-dihydro-1H-pyrrolo[2,3-c]pyridin-4-yl)-4-(methylsulfonyl)phenoxy)methyl)phenyl)dihydropyrimidine-2,4(1H,3H)-dione;
1-(3-((2-(6-methyl-7-oxo-6,7-dihydro-1H-pyrrolo[2,3-c]pyridin-4-yl)-4-(methylsulfonyl)phenoxy)methyl)phenyl)dihydropyrimidine-2,4(1H,3H)-dione;
1-(2-((2-(6-methyl-7-oxo-6,7-dihydro-1H-pyrrolo[2,3-c]pyridin-4-yl)-4-(methylsulfonyl)phenoxy)methyl)phenyl)dihydropyrimidine-2,4(1H,3H)-dione;
1-(6-((2-(6-methyl-7-oxo-6,7-dihydro-1H-pyrrolo[2,3-c]pyridin-4-yl)-4-(methylsulfonyl)phenoxy)methyl)pyridin-3-yl)dihydropyrimidine-2,4(1H,3H)-dione;
1-(5-((2-(6-methyl-7-oxo-6,7-dihydro-1H-pyrrolo[2,3-c]pyridin-4-yl)-4-(methylsulfonyl)phenoxy)methyl)pyridin-2-yl)dihydropyrimidine-2,4(1H,3H)-dione;
1-(6-((2-(6-methyl-7-oxo-6,7-dihydro-1H-pyrrolo[2,3-c]pyridin-4-yl)-4-(methylsulfonyl)phenoxy)methyl)pyridazin-3-yl)dihydropyrimidine-2,4(1H,3H)-dione;
1-(4-((2-(6-methyl-7-oxo-6,7-dihydro-1H-pyrrolo[2,3-c]pyridin-4-yl)-4-(methylsulfonyl)phenoxy)methyl)pyridin-3-yl)dihydropyrimidine-2,4(1H,3H)-dione;
3-((4-((2-(6-methyl-7-oxo-6,7-dihydro-1H-pyrrolo[2,3-c]pyridin-4-yl)-4-(methylsulfonyl)phenoxy)methyl)phenyl)amino)piperidine-2,6-dione;
3-((3-((2-(6-methyl-7-oxo-6,7-dihydro-1H-pyrrolo[2,3-c]pyridin-4-yl)-4-(methylsulfonyl)phenoxy)methyl)phenyl)amino)piperidine-2,6-dione;
3-((2-((2-(6-methyl-7-oxo-6,7-dihydro-1H-pyrrolo[2,3-c]pyridin-4-yl)-4-(methylsulfonyl)phenoxy)methyl)phenyl)amino)piperidine-2,6-dione;
N-((1r,4r)-4-(3-chloro-4-cyanophenoxy)cyclohexyl)-6-(4-(4-(4-(2,6-dioxopiperidin-3-yl)benzyl)piperazin-1-yl)piperidin-1-yl)pyridazine-3-carboxamide;
N-((1r,4r)-4-(3-chloro-4-cyanophenoxy)cyclohexyl)-6-(4-(4-(4-(2,6-dioxopiperidin-3-yl)-3-fluorobenzyl)piperazin-1-yl)piperidin-1-yl)pyridazine-3-carboxamide;
N-((1r,4r)-4-(3-chloro-4-cyanophenoxy)cyclohexyl)-6-(4-(4-(3-(2,6-dioxopiperidin-3-yl)benzyl)piperazin-1-yl)piperidin-1-yl)pyridazine-3-carboxamide;
N-((1r,4r)-4-(3-chloro-4-cyanophenoxy)cyclohexyl)-6-(4-(4-(2-(2,6-dioxopiperidin-3-yl)benzyl)piperazin-1-yl)piperidin-1-yl)pyridazine-3-carboxamide;
N-((1r,4r)-4-(3-chloro-4-cyanophenoxy)cyclohexyl)-6-(4-(4-(4-(2,4-dioxotetrahydropyrimidin-1(2H)-yl)benzyl)piperazin-1-yl)piperidin-1-yl)pyridazine-3-carboxamide;
N-((1r,4r)-4-(3-chloro-4-cyanophenoxy)cyclohexyl)-6-(4-(4-(4-(2,4-dioxotetrahydropyrimidin-1(2H)-yl)-3-fluorobenzyl)piperazin-1-yl)piperidin-1-yl)pyridazine-3-carboxamide;
N-((1r,4r)-4-(3-chloro-4-cyanophenoxy)cyclohexyl)-6-(4-(4-(3-(2,4-dioxotetrahydropyrimidin-1(2H)-yl)benzyl)piperazin-1-yl)piperidin-1-yl)pyridazine-3-carboxamide;
N-((1r,4r)-4-(3-chloro-4-cyanophenoxy)cyclohexyl)-6-(4-(4-(2-(2,4-dioxotetrahydropyrimidin-1(2H)-yl)benzyl)piperazin-1-yl)piperidin-1-yl)pyridazine-3-carboxamide;
N-((1r,4r)-4-(3-chloro-4-cyanophenoxy)cyclohexyl)-6-(4-(4-((5-(2,4-dioxotetrahydropyrimidin-1(2H)-yl)pyridin-2-yl)methyl)piperazin-1-yl)piperidin-1-yl)pyridazine-3-carboxamide;
N-((1r,4r)-4-(3-chloro-4-cyanophenoxy)cyclohexyl)-6-(4-(4-((6-(2,4-dioxotetrahydropyrimidin-1(2H)-yl)pyridazin-3-yl)methyl)piperazin-1-yl)piperidin-1-yl)pyridazine-3-carboxamide;
N-((1r,4r)-4-(3-chloro-4-cyanophenoxy)cyclohexyl)-6-(4-(4-((3-(2,4-dioxotetrahydropyrimidin-1(2H)-yl)pyridin-4-yl)methyl)piperazin-1-yl)piperidin-1-yl)pyridazine-3-carboxamide;
N-((1r,4r)-4-(3-chloro-4-cyanophenoxy)cyclohexyl)-6-(4-(4-(4-((2,6-dioxopiperidin-3-yl)amino)benzyl)piperazin-1-yl)piperidin-1-yl)pyridazine-3-carboxamide;
N-((1r,4r)-4-(3-chloro-4-cyanophenoxy)cyclohexyl)-6-(4-(4-(3-((2,6-dioxopiperidin-3-yl)amino)benzyl)piperazin-1-yl)piperidin-1-yl)pyridazine-3-carboxamide;
N-((1r,4r)-4-(3-chloro-4-cyanophenoxy)cyclohexyl)-6-(4-(4-(2-((2,6-dioxopiperidin-3-yl)amino)benzyl)piperazin-1-yl)piperidin-1-yl)pyridazine-3-carboxamide;
N-((1r,3r)-3-(3-chloro-4-cyanophenoxy)-2,2,4,4-tetramethylcyclobutyl)-6-(4-(4-(2,6-dioxopiperidin-3-yl)benzyl)piperazin-1-yl)pyridazine-3-carboxamide;
N-((1r,3r)-3-(3-chloro-4-cyanophenoxy)-2,2,4,4-tetramethylcyclobutyl)-6-(4-(4-(2,6-dioxopiperidin-3-yl)-3-fluorobenzyl)piperazin-1-yl)pyridazine-3-carboxamide;
N-((1r,3r)-3-(3-chloro-4-cyanophenoxy)-2,2,4,4-tetramethylcyclobutyl)-6-(4-(3-(2,6-dioxopiperidin-3-yl)benzyl)piperazin-1-yl)pyridazine-3-carboxamide;
N-((1r,3r)-3-(3-chloro-4-cyanophenoxy)-2,2,4,4-tetramethylcyclobutyl)-6-(4-(2-(2,6-dioxopiperidin-3-yl)benzyl)piperazin-1-yl)pyridazine-3-carboxamide;
N-((1r,3r)-3-(3-chloro-4-cyanophenoxy)-2,2,4,4-tetramethylcyclobutyl)-6-(4-(4-(2,4-dioxotetrahydropyrimidin-1(2H)-yl)benzyl)piperazin-1-yl)pyridazine-3-carboxamide;
N-((1r,3r)-3-(3-chloro-4-cyanophenoxy)-2,2,4,4-tetramethylcyclobutyl)-6-(4-(4-(2,4-dioxotetrahydropyrimidin-1(2H)-yl)-2-fluorobenzyl)piperazin-1-yl)pyridazine-3-carboxamide;
N-((1r,3r)-3-(3-chloro-4-cyanophenoxy)-2,2,4,4-tetramethylcyclobutyl)-6-(4-(3-(2,4-dioxotetrahydropyrimidin-1(2H)-yl)benzyl)piperazin-1-yl)pyridazine-3-carboxamide;
N-((1r,3r)-3-(3-chloro-4-cyanophenoxy)-2,2,4,4-tetramethylcyclobutyl)-6-(4-(2-(2,4-dioxotetrahydropyrimidin-1(2H)-yl)benzyl)piperazin-1-yl)pyridazine-3-carboxamide;
N-((1r,3r)-3-(3-chloro-4-cyanophenoxy)-2,2,4,4-tetramethylcyclobutyl)-6-(4-((5-(2,4-dioxotetrahydropyrimidin-1(2H)-yl)pyridin-2-yl)methyl)piperazin-1-yl)pyridazine-3-carboxamide;
N-((1r,3r)-3-(3-chloro-4-cyanophenoxy)-2,2,4,4-tetramethylcyclobutyl)-6-(4-((6-(2,4-dioxotetrahydropyrimidin-1(2H)-yl)pyridazin-3-yl)methyl)piperazin-1-yl)pyridazine-3-carboxamide;
N-((1r,3r)-3-(3-chloro-4-cyanophenoxy)-2,2,4,4-tetramethylcyclobutyl)-6-(4-((3-(2,4-dioxotetrahydropyrimidin-1(2H)-yl)pyridin-4-yl)methyl)piperazin-1-yl)pyridazine-3-carboxamide;
N-((1r,3r)-3-(3-chloro-4-cyanophenoxy)-2,2,4,4-tetramethylcyclobutyl)-6-(4-(4-((2,6-dioxopiperidin-3-yl)amino)benzyl)piperazin-1-yl)pyridazine-3-carboxamide;
N-((1r,3r)-3-(3-chloro-4-cyanophenoxy)-2,2,4,4-tetramethylcyclobutyl)-6-(4-(3-((2,6-dioxopiperidin-3-yl)amino)benzyl)piperazin-1-yl)pyridazine-3-carboxamide;
N-((1r,3r)-3-(3-chloro-4-cyanophenoxy)-2,2,4,4-tetramethylcyclobutyl)-6-(4-(2-((2,6-dioxopiperidin-3-yl)amino)benzyl)piperazin-1-yl)pyridazine-3-carboxamide;
N-((1r,3r)-3-(3-chloro-4-cyanophenoxy)-2,2,4,4-tetramethylcyclobutyl)-6-(4-(4-(2,6-dioxopiperidin-3-yl)benzyl)piperazin-1-yl)nicotinamide;
N-((1r,3r)-3-(3-chloro-4-cyanophenoxy)-2,2,4,4-tetramethylcyclobutyl)-6-(4-(4-(2,6-dioxopiperidin-3-yl)-3-fluorobenzyl)piperazin-1-yl)nicotinamide;
N-((1r,3r)-3-(3-chloro-4-cyanophenoxy)-2,2,4,4-tetramethylcyclobutyl)-6-(4-(3-(2,6-dioxopiperidin-3-yl)benzyl)piperazin-1-yl)nicotinamide;
N-((1r,3r)-3-(3-chloro-4-cyanophenoxy)-2,2,4,4-tetramethylcyclobutyl)-6-(4-(2-(2,6-dioxopiperidin-3-yl)benzyl)piperazin-1-yl)nicotinamide;
N-((1r,3r)-3-(3-chloro-4-cyanophenoxy)-2,2,4,4-tetramethylcyclobutyl)-6-(4-(4-(2,4-dioxotetrahydropyrimidin- 1(2H)-yl)benzyl)piperazin-1-yl)nicotinamide;
N-((1r,3r)-3-(3-chloro-4-cyanophenoxy)-2,2,4,4-tetramethylcyclobutyl)-6-(4-(4-(2,4-dioxotetrahydropyrimidin-1(2H)-yl)-2-fluorobenzyl)piperazin-1-yl)nicotinamide;
N-((1r,3r)-3-(3-chloro-4-cyanophenoxy)-2,2,4,4-tetramethylcyclobutyl)-6-(4-(3-(2,4-dioxotetrahydropyrimidin- 1(2H)-yl)benzyl)piperazin-1-yl)nicotinamide;
N-((lr,3r)-3-(3-chloro-4-cyanophenoxy)-2,2,4,4-tetramethylcyclobutyl)-6-(4-(2-(2,4-dioxotetrahydropyrimidin- 1(2H)-yl)benzyl)piperazin-1-yl)nicotinamide;
N-((1r,3r)-3-(3-chloro-4-cyanophenoxy)-2,2,4,4-tetramethylcyclobutyl)-6-(4-((5-(2,4-dioxotetrahydropyrimidin-1(2H)-yl)pyridin-2-yl)methyl)piperazin-1-yl)nicotinamide;
N-((1r,3r)-3-(3-chloro-4-cyanophenoxy)-2,2,4,4-tetramethylcyclobutyl)-6-(4-((6-(2,4-dioxotetrahydropyrimidin-1(2H)-yl)pyridazin-3-yl)methyl)piperazin-1-yl)nicotinamide;
N-((1r,3r)-3-(3-chloro-4-cyanophenoxy)-2,2,4,4-tetramethylcyclobutyl)-6-(4-((3-(2,4-dioxotetrahydropyrimidin-1(2H)-yl)pyridin-4-yl)methyl)piperazin-1-yl)nicotinamide;
N-((1r,3r)-3-(3-chloro-4-cyanophenoxy)-2,2,4,4-tetramethylcyclobutyl)-6-(4-(4-((2,6-dioxopiperidin-3-yl)amino)benzyl)piperazin-1-yl)nicotinamide;
N-((1r,3r)-3-(3-chloro-4-cyanophenoxy)-2,2,4,4-tetramethylcyclobutyl)-6-(4-(3-((2,6-dioxopiperidin-3-yl)amino)benzyl)piperazin-1-yl)nicotinamide;
N-((1r,3r)-3-(3-chloro-4-cyanophenoxy)-2,2,4,4-tetramethylcyclobutyl)-6-(4-(2-((2,6-dioxopiperidin-3-yl)amino)benzyl)piperazin-1-yl)nicotinamide;
N-((1r,4r)-4-(3-chloro-4-cyanophenoxy)cyclohexyl)-6-(4-(4-(2,6-dioxopiperidin-3-yl)benzyl)piperazin-1-yl)pyridazine-3-carboxamide;
N-((1r,4r)-4-(3-chloro-4-cyanophenoxy)cyclohexyl)-6-(4-(4-(2,6-dioxopiperidin-3-yl)-3-fluorobenzyl)piperazin-1-yl)pyridazine-3-carboxamide;
N-((1r,4r)-4-(3-chloro-4-cyanophenoxy)cyclohexyl)-6-(4-(3-(2,6-dioxopiperidin-3-yl)benzyl)piperazin-1-yl)pyridazine-3-carboxamide;
N-((1r,4r)-4-(3-chloro-4-cyanophenoxy)cyclohexyl)-6-(4-(2-(2,6-dioxopiperidin-3-yl)benzyl)piperazin-1-yl)pyridazine-3-carboxamide;
N-((1r,4r)-4-(3-chloro-4-cyanophenoxy)cyclohexyl)-6-(4-(4-(2,4-dioxotetrahydropyrimidin-1(2H)-yl)benzyl)piperazin-1-yl)pyridazine-3-carboxamide;
N-((1r,4r)-4-(3-chloro-4-cyanophenoxy)cyclohexyl)-6-(4-(4-(2,4-dioxotetrahydropyrimidin-1(2H)-yl)-3-fluorobenzyl)piperazin-1-yl)pyridazine-3-carboxamide;
N-((1r,4r)-4-(3-chloro-4-cyanophenoxy)cyclohexyl)-6-(4-(3-(2,4-dioxotetrahydropyrimidin-1(2H)-yl)benzyl)piperazin-1-yl)pyridazine-3-carboxamide;
N-((1r,4r)-4-(3-chloro-4-cyanophenoxy)cyclohexyl)-6-(4-(2-(2,4-dioxotetrahydropyrimidin-1(2H)-yl)benzyl)piperazin-1-yl)pyridazine-3-carboxamide;
N-((1r,4r)-4-(3-chloro-4-cyanophenoxy)cyclohexyl)-6-(4-((5-(2,4-dioxotetrahydropyrimidin-1(2H)-yl)pyridin-2-yl)methyl)piperazin-1-yl)pyridazine-3-carboxamide;
N-((1r,4r)-4-(3-chloro-4-cyanophenoxy)cyclohexyl)-6-(4-((6-(2,4-dioxotetrahydropyrimidin-1(2H)-yl)pyridazin-3-yl)methyl)piperazin-1-yl)pyridazine-3-carboxamide;
N-((1r,4r)-4-(3-chloro-4-cyanophenoxy)cyclohexyl)-6-(4-((3-(2,4-dioxotetrahydropyrimidin-1(2H)-yl)pyridin-4-yl)methyl)piperazin-1-yl)pyridazine-3-carboxamide;
N-((1r,4r)-4-(3-chloro-4-cyanophenoxy)cyclohexyl)-6-(4-(4-((2,6-dioxopiperidin-3-yl)amino)benzyl)piperazin-1-yl)pyridazine-3-carboxamide;
N-((1r,4r)-4-(3-chloro-4-cyanophenoxy)cyclohexyl)-6-(4-(3-((2,6-dioxopiperidin-3-yl)amino)benzyl)piperazin-1-yl)pyridazine-3-carboxamide;
N-((1r,4r)-4-(3-chloro-4-cyanophenoxy)cyclohexyl)-6-(4-(2-((2,6-dioxopiperidin-3-yl)amino)benzyl)piperazin-1-yl)pyridazine-3-carboxamide;
1-(4-((5-(3,5-dimethylisoxazol-4-yl)-2-methylphenyl)((1-(4-(2,6-dioxopiperidin-3-yl)benzyl)piperidin-4-yl)methyl)amino)phenyl)cyclopropane-1-carbonitrile;
1-(4-((5-(3,5-dimethylisoxazol-4-yl)-2-methylphenyl)((1-(4-(2,6-dioxopiperidin-3-yl)-3-fluorobenzyl)piperidin-4-yl)methyl)amino)phenyl)cyclopropane-1-carbonitrile;
1-(4-((5-(3,5-dimethylisoxazol-4-yl)-2-methylphenyl)((1-(3-(2,6-dioxopiperidin-3-yl)benzyl)piperidin-4-yl)methyl)amino)phenyl)cyclopropane-1-carbonitrile;
1-(4-((5-(3,5-dimethylisoxazol-4-yl)-2-methylphenyl)((1-(2-(2,6-dioxopiperidin-3-yl)benzyl)piperidin-4-yl)methyl)amino)phenyl)cyclopropane-1-carbonitrile;
1-(4-((5-(3,5-dimethylisoxazol-4-yl)-2-methylphenyl)((1-(4-(2,4-dioxotetrahydropyrimidin-1(2H)-yl)benzyl)piperidin-4-yl)methyl)amino)phenyl)cyclopropane-1-carbonitrile;
1-(4-((5-(3,5-dimethylisoxazol-4-yl)-2-methylphenyl)((1-(4-(2,4-dioxotetrahydropyrimidin-1(2H)-yl)-2-fluorobenzyl)piperidin-4-yl)methyl)amino)phenyl)cyclopropane-1-carbonitrile;
1-(4-((5-(3,5-dimethylisoxazol-4-yl)-2-methylphenyl)((1-(4-(2,4-dioxotetrahydropyrimidin-1(2H)-yl)-3-fluorobenzyl)piperidin-4-yl)methyl)amino)phenyl)cyclopropane-1-carbonitrile;
1-(4-((5-(3,5-dimethylisoxazol-4-yl)-2-methylphenyl)((1-(3-(2,4-dioxotetrahydropyrimidin-1(2H)-yl)benzyl)piperidin-4-yl)methyl)amino)phenyl)cyclopropane-1-carbonitrile;
1-(4-((5-(3,5-dimethylisoxazol-4-yl)-2-methylphenyl)((1-(2-(2,4-dioxotetrahydropyrimidin-1(2H)-yl)benzyl)piperidin-4-yl)methyl)amino)phenyl)cyclopropane-1-carbonitrile;
1-(4-((5-(3,5-dimethylisoxazol-4-yl)-2-methylphenyl)((1-((5-(2,4-dioxotetrahydropyrimidin-1(2H)-yl)pyridin-2-yl)methyl)piperidin-4-yl)methyl)amino)phenyl)cyclopropane-1-carbonitrile;
1-(4-((5-(3,5-dimethylisoxazol-4-yl)-2-methylphenyl)((1-((6-(2,4-dioxotetrahydropyrimidin-1(2H)-yl)pyridin-3-yl)methyl)piperidin-4-yl)methyl)amino)phenyl)cyclopropane-1-carbonitrile;
1-(4-((5-(3,5-dimethylisoxazol-4-yl)-2-methylphenyl)((1-((6-(2,4-dioxotetrahydropyrimidin-1(2H)-yl)pyridazin-3-yl)methyl)piperidin-4-yl)methyl)amino)phenyl)cyclopropane-1-carbonitrile;
1-(4-((5-(3,5-dimethylisoxazol-4-yl)-2-methylphenyl)((1-((3-(2,4-dioxotetrahydropyrimidin-1(2H)-yl)pyridin-4-yl)methyl)piperidin-4-yl)methyl)amino)phenyl)cyclopropane-1-carbonitrile;
1-(4-((5-(3,5-dimethylisoxazol-4-yl)-2-methylphenyl)((1-(4-((2,6-dioxopiperidin-3-yl)amino)benzyl)piperidin-4-yl)methyl)amino)phenyl)cyclopropane-1-carbonitrile;
1-(4-((5-(3,5-dimethylisoxazol-4-yl)-2-methylphenyl)((1-(3-((2,6-dioxopiperidin-3-yl)amino)benzyl)piperidin-4-yl)methyl)amino)phenyl)cyclopropane-1-carbonitrile;
1-(4-((5-(3,5-dimethylisoxazol-4-yl)-2-methylphenyl)((1-(2-((2,6-dioxopiperidin-3-yl)amino)benzyl)piperidin-4-yl)methyl)amino)phenyl)cyclopropane-1-carbonitrile;
1-(5-((4-chloro-2-(7-chloro-5-(trifluoromethyl)-1H-benzo[d]imidazol-2-yl)phenoxy)methyl)pyridin-2-yl)dihydropyrimidine-2,4(1H,3H)-dione;
1-(3-((4-chloro-2-(7-chloro-5-(trifluoromethyl)- 1 H-benzo [d]imidazol-2-yl)phenoxy)methyl)phenyl)dihydropyrimidine-2,4(1H,3H)-dione;
1-(4-((4-chloro-2-(7-chloro-5-(trifluoromethyl)-1H-benzo[d]imidazol-2-yl)phenoxy)methyl)phenyl)dihydropyrimidine-2,4(1H,3H)-dione;
3-(4-((4-(4-((9-cyclopentyl-8-(phenylamino)-9H-purin-2-yl)amino)phenyl)piperazin-1-yl)methyl)phenyl)piperidine-2,6-dione;
3-(4-((4-(4-((9-cyclopentyl-8-(phenylamino)-9H-purin-2-yl)amino)phenyl)piperazin-1-yl)methyl)-2-fluorophenyl)piperidine-2,6-dione;
3-(3-((4-(4-((9-cyclopentyl-8-(phenylamino)-9H-purin-2-yl)amino)phenyl)piperazin-1-yl)methyl)phenyl)piperidine-2,6-dione;
3-(2-((4-(4-((9-cyclopentyl-8-(phenylamino)-9H-purin-2-yl)amino)phenyl)piperazin-1-yl)methyl)phenyl)piperidine-2,6-dione;
1-(4-((4-(4-((9-cyclopentyl-8-(phenylamino)-9H-purin-2-yl)amino)phenyl)piperazin-1-yl)methyl)phenyl)dihydropyrimidine-2,4(1H,3H)-dione;
1-(4-((4-(4-((9-cyclopentyl-8-(phenylamino)-9H-purin-2-yl)amino)phenyl)piperazin-1-yl)methyl)-3-fluorophenyl)dihydropyrimidine-2,4(1H,3H)-dione;
1-(3-((4-(4-((9-cyclopentyl-8-(phenylamino)-9H-purin-2-yl)amino)phenyl)piperazin-1-yl)methyl)phenyl)dihydropyrimidine-2,4(1H,3H)-dione;
1-(2-((4-(4-((9-cyclopentyl-8-(phenylamino)-9H-purin-2-yl)amino)phenyl)piperazin-1-yl)methyl)phenyl)dihydropyrimidine-2,4(1H,3H)-dione;
1-(4-((4-(6-((9-cyclopentyl-8-(phenylamino)-9H-purin-2-yl)amino)pyridin-3-yl)piperazin-1-yl)methyl)phenyl)dihydropyrimidine-2,4(1H,3H)-dione;
1-(4-((4-(6-((9-cyclopentyl-8-(phenylamino)-9H-purin-2-yl)amino)pyridazin-3-yl)piperazin-1-yl)methyl)phenyl)dihydropyrimidine-2,4(1H,3H)-dione;
1-(6-((4-(4-((9-cyclopentyl-8-(phenylamino)-9H-purin-2-yl)amino)phenyl)piperazin-1-yl)methyl)pyridin-3-yl)dihydropyrimidine-2,4(1H,3H)-dione;
1-(5-((4-(4-((9-cyclopentyl-8-(phenylamino)-9H-purin-2-yl)amino)phenyl)piperazin-1-yl)methyl)pyridin-2-yl)dihydropyrimidine-2,4(1H,3H)-dione;
1-(4-((4-(4-((9-cyclopentyl-8-(phenylamino)-9H-purin-2-yl)amino)phenyl)piperazin-1-yl)methyl)pyridin-3-yl)dihydropyrimidine-2,4(1H,3H)-dione;
3-((4-((4-(4-((9-cyclopentyl-8-(phenylamino)-9H-purin-2-yl)amino)phenyl)piperazin-1-yl)methyl)phenyl)amino)piperidine-2,6-dione;
3-((3-((4-(4-((9-cyclopentyl-8-(phenylamino)-9H-purin-2-yl)amino)phenyl)piperazin-1-yl)methyl)phenyl)amino)piperidine-2,6-dione;
3-((2-((4-(4-((9-cyclopentyl-8-(phenylamino)-9H-purin-2-yl)amino)phenyl)piperazin-1-yl)methyl)phenyl)amino)piperidine-2,6-dione;
3-(4-(((1-(4-((9-cyclopentyl-8-(phenylamino)-9H-purin-2-yl)amino)phenyl)piperidin-4-yl)(methyl)amino)methyl)phenyl)piperidine-2,6-dione;
3-(4-(((1-(4-((9-cyclopentyl-8-(phenylamino)-9H-purin-2-yl)amino)phenyl)piperidin-4-yl)(methyl)amino)methyl)-2-fluorophenyl)piperidine-2,6-dione;
3-(3-(((1-(4-((9-cyclopentyl-8-(phenylamino)-9H-purin-2-yl)amino)phenyl)piperidin-4-yl)(methyl)amino)methyl)phenyl)piperidine-2,6-dione;
3-(2-(((1-(4-((9-cyclopentyl-8-(phenylamino)-9H-purin-2-yl)amino)phenyl)piperidin-4-yl)(methyl)amino)methyl)phenyl)piperidine-2,6-dione;
1-(4-(((1-(4-((9-cyclopentyl-8-(phenylamino)-9H-purin-2-yl)amino)phenyl)piperidin-4-yl)(methyl)amino)methyl)phenyl)dihydropyrimidine-2,4(1H,3H)-dione;
1-(4-(((1-(4-((9-cyclopentyl-8-(phenylamino)-9H-purin-2-yl)amino)phenyl)piperidin-4-yl)(methyl)amino)methyl)-2-fluorophenyl)dihydropyrimidine-2,4(1H,3H)-dione;
1-(3-(((1-(4-((9-cyclopentyl-8-(phenylamino)-9H-purin-2-yl)amino)phenyl)piperidin-4-yl)(methyl)amino)methyl)phenyl)dihydropyrimidine-2,4(1H,3H)-dione;
1-(2-(((1-(4-((9-cyclopentyl-8-(phenylamino)-9H-purin-2-yl)amino)phenyl)piperidin-4-yl)(methyl)amino)methyl)phenyl)dihydropyrimidine-2,4(1H,3H)-dione;
1-(6-(((1-(4-((9-cyclopentyl-8-(phenylamino)-9H-purin-2-yl)amino)phenyl)piperidin-4-yl)(methyl)amino)methyl)pyridin-3-yl)dihydropyrimidine-2,4(1H,3H)-dione;
1-(5-(((1-(4-((9-cyclopentyl-8-(phenylamino)-9H-purin-2-yl)amino)phenyl)piperidin-4-yl)(methyl)amino)methyl)pyridin-2-yl)dihydropyrimidine-2,4(1H,3H)-dione;
1-(6-(((1-(4-((9-cyclopentyl-8-(phenylamino)-9H-purin-2-yl)amino)phenyl)piperidin-4-yl)(methyl)amino)methyl)pyridazin-3-yl)dihydropyrimidine-2,4(1H,3H)-dione;
1-(4-(((1-(4-((9-cyclopentyl-8-(phenylamino)-9H-purin-2-yl)amino)phenyl)piperidin-4-yl)(methyl)amino)methyl)pyridin-3-yl)dihydropyrimidine-2,4(1H,3H)-dione;
3-((4-(((1-(4-((9-cyclopentyl-8-(phenylamino)-9H-purin-2-yl)amino)phenyl)piperidin-4-yl)(methyl)amino)methyl)phenyl)amino)piperidine-2,6-dione;
3-((3-(((1-(4-((9-cyclopentyl-8-(phenylamino)-9H-purin-2-yl)amino)phenyl)piperidin-4-yl)(methyl)amino)methyl)phenyl)amino)piperidine-2,6-dione;
3-((2-(((1-(4-((9-cyclopentyl-8-(phenylamino)-9H-purin-2-yl)amino)phenyl)piperidin-4-yl)(methyl)amino)methyl)phenyl)amino)piperidine-2,6-dione;
3-(4-((4-(1-(4-((9-cyclopentyl-8-(phenylamino)-9H-purin-2-yl)amino)phenyl)piperidin-4-yl)piperazin-1-yl)methyl)phenyl)piperidine-2,6-dione;
3-(4-((4-(1-(4-((9-cyclopentyl-8-(phenylamino)-9H-purin-2-yl)amino)phenyl)piperidin-4-yl)piperazin-1-yl)methyl)-2-fluorophenyl)piperidine-2,6-dione;
3-(3-((4-(1-(4-((9-cyclopentyl-8-(phenylamino)-9H-purin-2-yl)amino)phenyl)piperidin-4-yl)piperazin-1-yl)methyl)phenyl)piperidine-2,6-dione;
3-(2-((4-(1-(4-((9-cyclopentyl-8-(phenylamino)-9H-purin-2-yl)amino)phenyl)piperidin-4-yl)piperazin-1-yl)methyl)phenyl)piperidine-2,6-dione;
1-(4-((4-(1-(4-((9-cyclopentyl-8-(phenylamino)-9H-purin-2-yl)amino)phenyl)piperidin-4-yl)piperazin-1-yl)methyl)phenyl)dihydropyrimidine-2,4(1H,3H)-dione;
1-(4-((4-(1-(4-((9-cyclopentyl-8-(phenylamino)-9H-purin-2-yl)amino)phenyl)piperidin-4-yl)piperazin-1-yl)methyl)-3-fluorophenyl)dihydropyrimidine-2,4(1H,3H)-dione;
1-(3-((4-(1-(4-((9-cyclopentyl-8-(phenylamino)-9H-purin-2-yl)amino)phenyl)piperidin-4-yl)piperazin-1-yl)methyl)phenyl)dihydropyrimidine-2,4(1H,3H)-dione;
1-(2-((4-(1-(4-((9-cyclopentyl-8-(phenylamino)-9H-purin-2-yl)amino)phenyl)piperidin-4-yl)piperazin-1-yl)methyl)phenyl)dihydropyrimidine-2,4(1H,3H)-dione;
1-(6-((4-(1-(4-((9-cyclopentyl-8-(phenylamino)-9H-purin-2-yl)amino)phenyl)piperidin-4-yl)piperazin-1-yl)methyl)pyridin-3-yl)dihydropyrimidine-2,4(1H,3H)-dione;
1-(5-((4-(1-(4-((9-cyclopentyl-8-(phenylamino)-4,9-dihydro-3H-purin-2-yl)amino)phenyl)piperidin-4-yl)piperazin-1-yl)methyl)pyridin-2-yl)dihydropyrimidine-2,4(1H,3H)-dione;
1-(6-((4-(1-(4-((9-cyclopentyl-8-(phenylamino)-9H-purin-2-yl)amino)phenyl)piperidin-4-yl)piperazin-1-yl)methyl)pyridazin-3-yl)dihydropyrimidine-2,4(1H,3H)-dione;
1-(4-((4-(1-(4-((9-cyclopentyl-8-(phenylamino)-9H-purin-2-yl)amino)phenyl)piperidin-4-yl)piperazin-1-yl)methyl)pyridin-3-yl)dihydropyrimidine-2,4(1H,3H)-dione;
3-((4-((4-(1-(4-((9-cyclopentyl-8-(phenylamino)-9H-purin-2-yl)amino)phenyl)piperidin-4-yl)piperazin-1-yl)methyl)phenyl)amino)piperidine-2,6-dione;
3-((3-((4-(1-(4-((9-cyclopentyl-8-(phenylamino)-9H-purin-2-yl)amino)phenyl)piperidin-4-yl)piperazin-1-yl)methyl)phenyl)amino)piperidine-2,6-dione;
3-((2-((4-(1-(4-((9-cyclopentyl-8-(phenylamino)-9H-purin-2-yl)amino)phenyl)piperidin-4-yl)piperazin-1-yl)methyl)phenyl)amino)piperidine-2,6-dione;
3-(4-(((5-methoxy-4-((4-(1-methyl-1H-indol-3-yl)pyrimidin-2-yl)amino)-2-nitrophenyl)amino)methyl)phenyl)piperidine-2,6-dione;
3-(2-fluoro-4-(((5-methoxy-4-((4-(1-methyl-1H-indol-3-yl)pyrimidin-2-yl)amino)-2-nitrophenyl)amino)methyl)phenyl)piperidine-2,6-dione;
3-(3-(((5-methoxy-4-((4-(1-methyl-1H-indol-3-yl)pyrimidin-2-yl)amino)-2-nitrophenyl)amino)methyl)phenyl)piperidine-2,6-dione;
3-(2-(((5-methoxy-4-((4-(1-methyl-1H-indol-3-yl)pyrimidin-2-yl)amino)-2-nitrophenyl)amino)methyl)phenyl)piperidine-2,6-dione;
1-(4-(((5-methoxy-4-((4-(1-methyl-1H-indol-3-yl)pyrimidin-2-yl)amino)-2-nitrophenyl)amino)methyl)phenyl)dihydropyrimidine-2,4(1H,3H)-dione;
1-(3-fluoro-4-(((5-methoxy-4-((4-(1-methyl-1H-indol-3-yl)pyrimidin-2-yl)amino)-2-nitrophenyl)amino)methyl)phenyl)dihydropyrimidine-2,4(1H,3H)-dione;
1-(3-(((5-methoxy-4-((4-(1-methyl-1H-indol-3-yl)pyrimidin-2-yl)amino)-2-nitrophenyl)amino)methyl)phenyl)dihydropyrimidine-2,4(1H,3H)-dione;
1-(2-(((5-methoxy-4-((4-(1-methyl-1H-indol-3-yl)pyrimidin-2-yl)amino)-2-nitrophenyl)amino)methyl)phenyl)dihydropyrimidine-2,4(1H,3H)-dione;
1-(6-(((5-methoxy-4-((4-(1-methyl-1H-indol-3-yl)pyrimidin-2-yl)amino)-2-nitrophenyl)amino)methyl)pyridin-3-yl)dihydropyrimidine-2,4(1H,3H)-dione;
1-(6-(((5-methoxy-4-((4-(1-methyl-1H-indol-3-yl)pyrimidin-2-yl)amino)-2-nitrophenyl)amino)methyl)pyridazin-3-yl)dihydropyrimidine-2,4(1H,3H)-dione;
1-(4-(((5-methoxy-4-((4-(1-methyl-1H-indol-3-yl)pyrimidin-2-yl)amino)-2-nitrophenyl)amino)methyl)pyridin-3-yl)dihydropyrimidine-2,4(1H,3H)-dione;
3-((4-(((5-methoxy-4-((4-(1-methyl-1H-indol-3-yl)pyrimidin-2-yl)amino)-2-nitrophenyl)amino)methyl)phenyl)amino)piperidine-2,6-dione;
3-((3-(((5-methoxy-4-((4-(1-methyl-1H-indol-3-yl)pyrimidin-2-yl)amino)-2-nitrophenyl)amino)methyl)phenyl)amino)piperidine-2,6-dione;
3-((2-(((5-methoxy-4-((4-(1-methyl-1H-indol-3-yl)pyrimidin-2-yl)amino)-2-nitrophenyl)amino)methyl)phenyl)amino)piperidine-2,6-dione;
N-(2-((2-((4-(2,6-dioxopiperidin-3-yl)benzyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-((4-(2,6-dioxopiperidin-3-yl)-3-fluorobenzyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-((3-(2,6-dioxopiperidin-3-yl)benzyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-((2-(2,6-dioxopiperidin-3-yl)benzyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-((4-(2,4-dioxotetrahydropyrimidin-1(2H)-yl)benzyl)(methyl)amino)ethyl)(methyl)amino)-4-methoxy-5-((4-(1-methyl-1H-indol-3-yl)pyrimidin-2-yl)amino)phenylacrylamide;
N-(2-((2-((4-(2,4-dioxotetrahydropyrimidin-1(2H)-yl)-3-fluorobenzyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-((3-(2,4-dioxotetrahydropyrimidin-1(2H)-yl)benzyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-((2-(2,4-dioxotetrahydropyrimidin-1(2H)-yl)benzyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-(((5-(2,4-dioxotetrahydropyrimidin-1(2H)-yl)pyridin-2-yl)methyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-(((6-(2,4-dioxotetrahydropyrimidin-1(2H)-yl)pyridazin-3-yl)methyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-(((3-(2,4-dioxotetrahydropyrimidin-1(2H)-yl)pyridin-4-yl)methyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-((4-((2,6-dioxopiperidin-3-yl)amino)benzyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-((3-((2,6-dioxopiperidin-3-yl)amino)benzyl)(methyl)amino)ethyl)(methyl)amino)-4-methoxy-5-((4-(1-methyl-1H-indol-3-yl)pyrimidin-2-yl)amino)phenyl)acrylamide;
N-(2-((2-((2-((2,6-dioxopiperidin-3-yl)amino)benzyl)(methyl)amino)ethyl)(methyl)amino)-4-methoxy-5-((4-(1-methyl-1H-indol-3-yl)pyrimidin-2-yl)amino)phenyl)acrylamide;
3-(4-((4-(3-((4-((3-chloro-4-fluorophenyl)amino)-7-methoxyquinazolin-6-yl)oxy)propyl)piperazin-1-yl)methyl)phenyl)piperidine-2,6-dione;
3-(4-((4-(3-((4-((3-chloro-4-fluorophenyl)amino)-7-methoxyquinazolin-6-yl)oxy)propyl)piperazin-1-yl)methyl)-2-fluorophenyl)piperidine-2,6-dione;
3-(3-((4-(3-((4-((3-chloro-4-fluorophenyl)amino)-7-methoxyquinazolin-6-yl)oxy)propyl)piperazin-1-yl)methyl)phenyl)piperidine-2,6-dione;
3-(2-((4-(3-((4-((3-chloro-4-fluorophenyl)amino)-7-methoxyquinazolin-6-yl)oxy)propyl)piperazin-1-yl)methyl)phenyl)piperidine-2,6-dione;
1-(4-((4-(3-((4-((3-chloro-4-fluorophenyl)amino)-7-methoxyquinazolin-6-yl)oxy)propyl)piperazin-1-yl)methyl)phenyl)dihydropyrimidine-2,4(1H,3H)-dione;
1-(4-((4-(3-((4-((3-chloro-4-fluorophenyl)amino)-7-methoxyquinazolin-6-yl)oxy)propyl)piperazin-1-yl)methyl)-2-fluorophenyl)dihydropyrimidine-2,4(1H,3H)-dione;
1-(3-((4-(3-((4-((3-chloro-4-fluorophenyl)amino)-7-methoxyquinazolin-6-yl)oxy)propyl)piperazin-1-yl)methyl)phenyl)dihydropyrimidine-2,4(1H,3H)-dione;
1-(2-((4-(3-((4-((3-chloro-4-fluorophenyl)amino)-7-methoxyquinazolin-6-yl)oxy)propyl)piperazin-1-yl)methyl)phenyl)dihydropyrimidine-2,4(1H,3H)-dione;
1-(6-((4-(3-((4-((3-chloro-4-fluorophenyl)amino)-7-methoxyquinazolin-6-yl)oxy)propyl)piperazin-1-yl)methyl)pyridin-3-yl)dihydropyrimidine-2,4(1H,3H)-dione;
1-(6-((4-(3-((4-((3-chloro-4-fluorophenyl)amino)-7-methoxyquinazolin-6-yl)oxy)propyl)piperazin-1-yl)methyl)pyridazin-3-yl)dihydropyrimidine-2,4(1H,3H)-dione;
1-(4-((4-(3-((4-((3-chloro-4-fluorophenyl)amino)-7-methoxyquinazolin-6-yl)oxy)propyl)piperazin-1-yl)methyl)pyridin-3-yl)dihydropyrimidine-2,4(1H,3H)-dione;
3-((4-((4-(3-((4-((3-chloro-4-fluorophenyl)amino)-7-methoxyquinazolin-6-yl)oxy)propyl)piperazin-1-yl)methyl)phenyl)amino)piperidine-2,6-dione;
3-((3-((4-(3-((4-((3-chloro-4-fluorophenyl)amino)-7-methoxyquinazolin-6-yl)oxy)propyl)piperazin-1-yl)methyl)phenyl)amino)piperidine-2,6-dione;
3-((2-((4-(3-((4-((3-chloro-4-fluorophenyl)amino)-7-methoxyquinazolin-6-yl)oxy)propyl)piperazin-1-yl)methyl)phenyl)amino)piperidine-2,6-dione;
N-(4-((3-chloro-4-fluorophenyl)amino)-7-(3-(4-(4-(2,6-dioxopiperidin-3-yl)benzyl)piperazin-1-yl)propoxy)quinazolin-6-yl)acrylamide;
N-(4-((3-chloro-4-fluorophenyl)amino)-7-(3-(4-(4-(2,6-dioxopiperidin-3-yl)-3-fluorobenzyl)piperazin-1-yl)propoxy)quinazolin-6-yl)acrylamide;
N-(4-((3-chloro-4-fluorophenyl)amino)-7-(3-(4-(3-(2,6-dioxopiperidin-3-yl)benzyl)piperazin-1-yl)propoxy)quinazolin-6-yl)acrylamide;
N-(4-((3-chloro-4-fluorophenyl)amino)-7-(3-(4-(2-(2,6-dioxopiperidin-3-yl)benzyl)piperazin-1-yl)propoxy)quinazolin-6-yl)acrylamide;
N-(4-((3-chloro-4-fluorophenyl)amino)-7-(3-(4-(4-(2,4-dioxotetrahydropyrimidin-1(2H)-yl)benzyl)piperazin-1-yl)propoxy)quinazolin-6-yl)acrylamide;
N-(4-((3-chloro-4-fluorophenyl)amino)-7-(3-(4-(4-(2,4-dioxotetrahydropyrimidin-1(2H)-yl)-3-fluorobenzyl)piperazin-1-yl)propoxy)quinazolin-6-yl)acrylamide;
N-(4-((3-chloro-4-fluorophenyl)amino)-7-(3-(4-(3-(2,4-dioxotetrahydropyrimidin-1(2H)-yl)benzyl)piperazin-1-yl)propoxy)quinazolin-6-yl)acrylamide;
N-(4-((3-chloro-4-fluorophenyl)amino)-7-(3-(4-(2-(2,4-dioxotetrahydropyrimidin-1(2H)-yl)benzyl)piperazin-1-yl)propoxy)quinazolin-6-yl)acrylamide;
N-(4-((3-chloro-4-fluorophenyl)amino)-7-(3-(4-((5-(2,4-dioxotetrahydropyrimidin-1(2H)-yl)pyridin-2-yl)methyl)piperazin-1-yl)propoxy)quinazolin-6-yl)acrylamide;
N-(4-((3-chloro-4-fluorophenyl)amino)-7-(3-(4-((6-(2,4-dioxotetrahydropyrimidin-1(2H)-yl)pyridazin-3-yl)methyl)piperazin-1-yl)propoxy)quinazolin-6-yl)acrylamide;
N-(4-((3-chloro-4-fluorophenyl)amino)-7-(3-(4-((3-(2,4-dioxotetrahydropyrimidin-1(2H)-yl)pyridin-4-yl)methyl)piperazin-1-yl)propoxy)quinazolin-6-yl)acrylamide;
N-(4-((3-chloro-4-fluorophenyl)amino)-7-(3-(4-(4-((2,6-dioxopiperidin-3-yl)amino)benzyl)piperazin-1-yl)propoxy)quinazolin-6-yl)acrylamide;
N-(4-((3-chloro-4-fluorophenyl)amino)-7-(3-(4-(3-((2,6-dioxopiperidin-3-yl)amino)benzyl)piperazin-1-yl)propoxy)quinazolin-6-yl)acrylamide;
N-(4-((3-chloro-4-fluorophenyl)amino)-7-(3-(4-(2-((2,6-dioxopiperidin-3-yl)amino)benzyl)piperazin-1-yl)propoxy)quinazolin-6-yl)acrylamide;
3-(4-((4-((4-((3,4-dichloro-2-fluorophenyl)amino)-7-methoxyquinazolin-6-yl)oxy)piperidin-1-yl)methyl)phenyl)piperidine-2,6-dione;
3-(4-((4-((4-((3,4-dichloro-2-fluorophenyl)amino)-7-methoxyquinazolin-6-yl)oxy)piperidin-1-yl)methyl)-2-fluorophenyl)piperidine-2,6-dione;
3-(3-((4-((4-((3,4-dichloro-2-fluorophenyl)amino)-7-methoxyquinazolin-6-yl)oxy)piperidin-1-yl)methyl)phenyl)piperidine-2,6-dione;
3-(2-((4-((4-((3,4-dichloro-2-fluorophenyl)amino)-7-methoxyquinazolin-6-yl)oxy)piperidin-1-yl)methyl)phenyl)piperidine-2,6-dione;
1-(4-((4-((4-((3,4-dichloro-2-fluorophenyl)amino)-7-methoxyquinazolin-6-yl)oxy)piperidin-1-yl)methyl)phenyl)dihydropyrimidine-2,4(1H,3H)-dione;
1-(4-((4-((4-((3,4-dichloro-2-fluorophenyl)amino)-7-methoxyquinazolin-6-yl)oxy)piperidin-1-yl)methyl)-3-fluorophenyl)dihydropyrimidine-2,4(1H,3H)-dione;
1-(3-((4-((4-((3,4-dichloro-2-fluorophenyl)amino)-7-methoxyquinazolin-6-yl)oxy)piperidin-1-yl)methyl)phenyl)dihydropyrimidine-2,4(1H,3H)-dione;
1-(2-((4-((4-((3,4-dichloro-2-fluorophenyl)amino)-7-methoxyquinazolin-6-yl)oxy)piperidin-1-yl)methyl)phenyl)dihydropyrimidine-2,4(1H,3H)-dione;
1-(6-((4-((4-((3,4-dichloro-2-fluorophenyl)amino)-7-methoxyquinazolin-6-yl)oxy)piperidin-1-yl)methyl)pyridin-3-yl)dihydropyrimidine-2,4(1H,3H)-dione;
1-(6-((4-((4-((3,4-dichloro-2-fluorophenyl)amino)-7-methoxyquinazolin-6-yl)oxy)piperidin-1-yl)methyl)pyridazin-3-yl)dihydropyrimidine-2,4(1H,3H)-dione;
1-(4-((4-((4-((3,4-dichloro-2-fluorophenyl)amino)-7-methoxyquinazolin-6-yl)oxy)piperidin-1-yl)methyl)pyridin-3-yl)dihydropyrimidine-2,4(1H,3H)-dione;
3-((4-((4-((4-((3,4-dichloro-2-fluorophenyl)amino)-7-methoxyquinazolin-6-yl)oxy)piperidin-1-yl)methyl)phenyl)amino)piperidine-2,6-dione;
3-((3-((4-((4-((3,4-dichloro-2-fluorophenyl)amino)-7-methoxyquinazolin-6-yl)oxy)piperidin-1-yl)methyl)phenyl)amino)piperidine-2,6-dione;
3-((2-((4-((4-((3,4-dichloro-2-fluorophenyl)amino)-7-methoxyquinazolin-6-yl)oxy)piperidin-1-yl)methyl)phenyl)amino)piperidine-2,6-dione;
3-(4-(((4-((3-chloro-4-fluorophenyl)amino)-7-(((S)-tetrahydrofuran-3-yl)oxy)quinazolin-6-yl)amino)methyl)phenyl)piperidine-2,6-dione;
3-(4-(((4-((3-chloro-4-fluorophenyl)amino)-7-(((S)-tetrahydrofuran-3-yl)oxy)quinazolin-6-yl)amino)methyl)-2-fluorophenyl)piperidine-2,6-dione;
3-(3-(((4-((3-chloro-4-fluorophenyl)amino)-7-(((S)-tetrahydrofuran-3-yl)oxy)quinazolin-6-yl)amino)methyl)phenyl)piperidine-2,6-dione;
3-(2-(((4-((3-chloro-4-fluorophenyl)amino)-7-(((S)-tetrahydrofuran-3-yl)oxy)quinazolin-6-yl)amino)methyl)phenyl)piperidine-2,6-dione;
(S)-1-(4-(((4-((3-chloro-4-fluorophenyl)amino)-7-((tetrahydrofuran-3-yl)oxy)quinazolin-6-yl)amino)methyl)phenyl)dihydropyrimidine-2,4(1H,3H)-dione;
(S)-1-(4-(((4-((3-chloro-4-fluorophenyl)amino)-7-((tetrahydrofuran-3-yl)oxy)quinazolin-6-yl)amino)methyl)-3-fluorophenyl)dihydropyrimidine-2,4(1H,3H)-dione;
(S)-1-(3-(((4-((3-chloro-4-fluorophenyl)amino)-7-((tetrahydrofuran-3-yl)oxy)quinazolin-6-yl)amino)methyl)phenyl)dihydropyrimidine-2,4(1H,3H)-dione;
(S)-1-(2-(((4-((3-chloro-4-fluorophenyl)amino)-7-((tetrahydrofuran-3-yl)oxy)quinazolin-6-yl)amino)methyl)phenyl)dihydropyrimidine-2,4(1H,3H)-dione;
(S)-1-(6-(((4-((3-chloro-4-fluorophenyl)amino)-7-((tetrahydrofuran-3-yl)oxy)quinazolin-6-yl)amino)methyl)pyridin-3-yl)dihydropyrimidine-2,4(1H,3H)-dione;
(S)-1-(6-(((4-((3-chloro-4-fluorophenyl)amino)-7-((tetrahydrofuran-3-yl)oxy)quinazolin-6-yl)amino)methyl)pyridazin-3-yl)dihydropyrimidine-2,4(1H,3H)-dione;
(S)-1-(4-(((4-((3-chloro-4-fluorophenyl)amino)-7-((tetrahydrofuran-3-yl)oxy)quinazolin-6-yl)amino)methyl)pyridin-3-yl)dihydropyrimidine-2,4(1H,3H)-dione;
3-((4-(((4-((3-chloro-4-fluorophenyl)amino)-7-(((S)-tetrahydrofuran-3-yl)oxy)quinazolin-6-yl)amino)methyl)phenyl)amino)piperidine-2,6-dione;
3-((3-(((4-((3-chloro-4-fluorophenyl)amino)-7-(((S)-tetrahydrofuran-3-yl)oxy)quinazolin-6-yl)amino)methyl)phenyl)amino)piperidine-2,6-dione;
3-((2-(((4-((3-chloro-4-fluorophenyl)amino)-7-(((S)-tetrahydrofuran-3-yl)oxy)quinazolin-6-yl)amino)methyl)phenyl)amino)piperidine-2,6-dione;
(E)-N-(4-((3-chloro-4-fluorophenyl)amino)-7-methoxyquinazolin-6-yl)-4-(4-(4-(2,6-dioxopiperidin-3-yl)benzyl)piperazin-1-yl)but-2-enamide;
(E)-N-(4-((3-chloro-4-fluorophenyl)amino)-7-methoxyquinazolin-6-yl)-4-(4-(4-(2,6-dioxopiperidin-3-yl)-3-fluorobenzyl)piperazin-1-yl)but-2-enamide;
(E)-N-(4-((3-chloro-4-fluorophenyl)amino)-7-methoxyquinazolin-6-yl)-4-(4-(3-(2,6-dioxopiperidin-3-yl)benzyl)piperazin-1-yl)but-2-enamide;
(E)-N-(4-((3-chloro-4-fluorophenyl)amino)-7-methoxyquinazolin-6-yl)-4-(4-(2-(2,6-dioxopiperidin-3-yl)benzyl)piperazin-1-yl)but-2-enamide;
(E)-N-(4-((3-chloro-4-fluorophenyl)amino)-7-methoxyquinazolin-6-yl)-4-(4-(4-(2,4-dioxotetrahydropyrimidin-1(2H)-yl)benzyl)piperazin-1-yl)but-2-enamide;
(E)-N-(4-((3-chloro-4-fluorophenyl)amino)-7-methoxyquinazolin-6-yl)-4-(4-(4-(2,4-dioxotetrahydropyrimidin-1(2H)-yl)-2-fluorobenzyl)piperazin-1-yl)but-2-enamide;
(E)-N-(4-((3-chloro-4-fluorophenyl)amino)-7-methoxyquinazolin-6-yl)-4-(4-(3-(2,4-dioxotetrahydropyrimidin-1(2H)-yl)benzyl)piperazin-1-yl)but-2-enamide;
(E)-N-(4-((3-chloro-4-fluorophenyl)amino)-7-methoxyquinazolin-6-yl)-4-(4-(2-(2,4-dioxotetrahydropyrimidin-1(2H)-yl)benzyl)piperazin-1-yl)but-2-enamide;
(E)-N-(4-((3-chloro-4-fluorophenyl)amino)-7-methoxyquinazolin-6-yl)-4-(4-((5-(2,4-dioxotetrahydropyrimidin-1(2H)-yl)pyridin-2-yl)methyl)piperazin-1-yl)but-2-enamide;
(E)-N-(4-((3-chloro-4-fluorophenyl)amino)-7-methoxyquinazolin-6-yl)-4-(4-((6-(2,4-dioxotetrahydropyrimidin-1(2H)-yl)pyridazin-3-yl)methyl)piperazin-1-yl)but-2-enamide;
(E)-N-(4-((3-chloro-4-fluorophenyl)amino)-7-methoxyquinazolin-6-yl)-4-(4-((3-(2,4-dioxotetrahydropyrimidin-1(2H)-yl)pyridin-4-yl)methyl)piperazin-1-yl)but-2-enamide;
(E)-N-(4-((3-chloro-4-fluorophenyl)amino)-7-methoxyquinazolin-6-yl)-4-(4-(4-((2,6-dioxopiperidin-3-yl)amino)benzyl)piperazin-1-yl)but-2-enamide;
(E)-N-(4-((3-chloro-4-fluorophenyl)amino)-7-methoxyquinazolin-6-yl)-4-(4-(3-((2,6-dioxopiperidin-3-yl)amino)benzyl)piperazin-1-yl)but-2-enamide;
(E)-N-(4-((3-chloro-4-fluorophenyl)amino)-7-methoxyquinazolin-6-yl)-4-(4-(2-((2,6-dioxopiperidin-3-yl)amino)benzyl)piperazin-1-yl)but-2-enamide;
3-(4-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)phenyl)piperidine-2,6-dione;
3-(4-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-2-fluorophenyl)piperidine-2,6-dione;
3-(3-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)phenyl)piperidine-2,6-dione;
3-(2-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)phenyl)piperidine-2,6-dione;
1-(4-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)phenyl)dihydropyrimidine-2,4(1H,3H)-dione;
1-(4-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-3-fluorophenyl)dihydropyrimidine-2,4(1H,3H)-dione;
1-(3-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)phenyl)dihydropyrimidine-2,4(1H,3H)-dione;
1-(2-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)phenyl)dihydropyrimidine-2,4(1H,3H)-dione;
1-(6-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)pyridin-3-yl)dihydropyrimidine-2,4(1H,3H)-dione;
1-(5-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)pyridin-2-yl)dihydropyrimidine-2,4(1H,3H)-dione;
1-(6-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)pyridazin-3-yl)dihydropyrimidine-2,4(1H,3H)-dione;
1-(4-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)pyridin-3-yl)dihydropyrimidine-2,4(1H,3H)-dione;
3-((4-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)phenyl)amino)piperidine-2,6-dione;
3-((3-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)phenyl)amino)piperidine-2,6-dione;
3-((2-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)phenyl)amino)piperidine-2,6-dione;
3-(4-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)phenyl)piperidine-2,6-dione;
3-(4-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-2-fluorophenyl)piperidine-2,6-dione;
3-(3-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)phenyl)piperidine-2,6-dione;
3-(2-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)phenyl)piperidine-2,6-dione;
1-(4-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)phenyl)dihydropyrimidine-2,4(1H,3H)-dione;
1-(4-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-3-fluorophenyl)dihydropyrimidine-2,4(1H,3H)-dione;
1-(3-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)phenyl)dihydropyrimidine-2,4(1H,3H)-dione;
1-(2-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)phenyl)dihydropyrimidine-2,4(1H,3H)-dione;
1-(6-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)pyridin-3-yl)dihydropyrimidine-2,4(1H,3H)-dione;
1-(5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)pyridin-2-yl)dihydropyrimidine-2,4(1H,3H)-dione;
1-(6-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)pyridazin-3-yl)dihydropyrimidine-2,4(1H,3H)-dione;
1-(4-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)pyridin-3-yl)dihydropyrimidine-2,4(1H,3H)-dione;
3-((4-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)phenyl)amino)piperidine-2,6-dione;
3-((3-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)phenyl)amino)piperidine-2,6-dione;
3-((2-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)phenyl)amino)piperidine-2,6-dione;
3-(4-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)phenyl)piperidine-2,6-dione;
3-(4-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)-2-fluorophenyl)piperidine-2,6-dione;
3-(3-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)phenyl)piperidine-2,6-dione;
3-(2-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)phenyl)piperidine-2,6-dione;
1-(4-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)phenyl)dihydropyrimidine-2,4(1H,3H)-dione;
1-(4-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)-3-fluorophenyl)dihydropyrimidine-2,4(1H,3H)-dione;
1-(3-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)phenyl)dihydropyrimidine-2,4(1H,3H)-dione;
1-(2-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)phenyl)dihydropyrimidine-2,4(1H,3H)-dione;
1-(6-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)pyridin-3-yl)dihydropyrimidine-2,4(1H,3H)-dione;
1-(5-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)pyridin-2-yl)dihydropyrimidine-2,4(1H,3H)-dione;
1-(6-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)pyridazin-3-yl)dihydropyrimidine-2,4(1H,3H)-dione;
1-(4-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)pyridin-3-yl)dihydropyrimidine-2,4(1H,3H)-dione;
3-((4-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)phenyl)amino)piperidine-2,6-dione;
3-((3-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)phenyl)amino)piperidine-2,6-dione;
3-((2-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)phenyl)amino)piperidine-2,6-dione;
2-(7-((4-(2,6-dioxopiperidin-3-yl)benzyl)amino)-1-oxoisoindolin-2-yl)-2-(5-fluoro-2-hydroxyphenyl)-N-(thiazol-2-yl)acetamide;
2-(7-((4-(2,6-dioxopiperidin-3-yl)-3-fluorobenzyl)amino)-1-oxoisoindolin-2-yl)-2-(5-fluoro-2-hydroxyphenyl)-N-(thiazol-2-yl)acetamide;
2-(7-((3-(2,6-dioxopiperidin-3-yl)benzyl)amino)-1-oxoisoindolin-2-yl)-2-(5-fluoro-2-hydroxyphenyl)-N-(thiazol-2-yl)acetamide;
2-(7-((2-(2,6-dioxopiperidin-3-yl)benzyl)amino)-1-oxoisoindolin-2-yl)-2-(5-fluoro-2-hydroxyphenyl)-N-(thiazol-2-yl)acetamide;
2-(7-((4-(2,4-dioxotetrahydropyrimidin-1(2H)-yl)benzyl)amino)-1-oxoisoindolin-2-yl)-2-(5-fluoro-2-hydroxyphenyl)-N-(thiazol-2-yl)acetamide;
2-(7-((4-(2,4-dioxotetrahydropyrimidin-1(2H)-yl)-2-fluorobenzyl)amino)-1-oxoisoindolin-2-yl)-2-(5-fluoro-2-hydroxyphenyl)-N-(thiazol-2-yl)acetamide;
2-(7-((3-(2,4-dioxotetrahydropyrimidin-1(2H)-yl)benzyl)amino)-1-oxoisoindolin-2-yl)-2-(5-fluoro-2-hydroxyphenyl)-N-(thiazol-2-yl)acetamide;
2-(7-((2-(2,4-dioxotetrahydropyrimidin-1(2H)-yl)benzyl)amino)-1-oxoisoindolin-2-yl)-2-(5-fluoro-2-hydroxyphenyl)-N-(thiazol-2-yl)acetamide;
2-(7-(((5-(2,4-dioxotetrahydropyrimidin-1(2H)-yl)pyridin-2-yl)methyl)amino)-1-oxoisoindolin-2-yl)-2-(5-fluoro-2-hydroxyphenyl)-N-(thiazol-2-yl)acetamide;
2-(7-(((6-(2,4-dioxotetrahydropyrimidin-1(2H)-yl)pyridazin-3-yl)methyl)amino)-1-oxoisoindolin-2-yl)-2-(5-fluoro-2-hydroxyphenyl)-N-(thiazol-2-yl)acetamide;
2-(7-(((3-(2,4-dioxotetrahydropyrimidin-1(2H)-yl)pyridin-4-yl)methyl)amino)-1-oxoisoindolin-2-yl)-2-(5-fluoro-2-hydroxyphenyl)-N-(thiazol-2-yl)acetamide;
2-(7-((4-((2,6-dioxopiperidin-3-yl)amino)benzyl)amino)-1-oxoisoindolin-2-yl)-2-(5-fluoro-2-hydroxyphenyl)-N-(thiazol-2-yl)acetamide;
2-(7-((3-((2,6-dioxopiperidin-3-yl)amino)benzyl)amino)-1-oxoisoindolin-2-yl)-2-(5-fluoro-2-hydroxyphenyl)-N-(thiazol-2-yl)acetamide;
2-(7-((2-((2,6-dioxopiperidin-3-yl)amino)benzyl)amino)-1-oxoisoindolin-2-yl)-2-(5-fluoro-2-hydroxyphenyl)-N-(thiazol-2-yl)acetamide;
2-(6-(4-(4-(2,6-dioxopiperidin-3-yl)benzyl)piperazin-1-yl)-1-oxoisoindolin-2-yl)-2-(5-fluoro-2-hydroxyphenyl)-N-(thiazol-2-yl)acetamide;
2-(6-(4-(4-(2,6-dioxopiperidin-3-yl)-3-fluorobenzyl)piperazin-1-yl)-1-oxoisoindolin-2-yl)-2-(5-fluoro-2-hydroxyphenyl)-N-(thiazol-2-yl)acetamide;
2-(6-(4-(3-(2,6-dioxopiperidin-3-yl)benzyl)piperazin-1-yl)-1-oxoisoindolin-2-yl)-2-(5-fluoro-2-hydroxyphenyl)-N-(thiazol-2-yl)acetamide;
2-(6-(4-(2-(2,6-dioxopiperidin-3-yl)benzyl)piperazin-1-yl)-1-oxoisoindolin-2-yl)-2-(5-fluoro-2-hydroxyphenyl)-N-(thiazol-2-yl)acetamide;
2-(6-(4-(4-(2,4-dioxotetrahydropyrimidin-1(2H)-yl)benzyl)piperazin-1-yl)-1-oxoisoindolin-2-yl)-2-(5-fluoro-2-hydroxyphenyl)-N-(thiazol-2-yl)acetamide;
2-(6-(4-(4-(2,4-dioxotetrahydropyrimidin-1(2H)-yl)-2-fluorobenzyl)piperazin-1-yl)-1-oxoisoindolin-2-yl)-2-(5-fluoro-2-hydroxyphenyl)-N-(thiazol-2-yl)acetamide;
2-(6-(4-(3-(2,4-dioxotetrahydropyrimidin-1(2H)-yl)benzyl)piperazin-1-yl)-1-oxoisoindolin-2-yl)-2-(5-fluoro-2-hydroxyphenyl)-N-(thiazol-2-yl)acetamide;
2-(6-(4-(2-(2,4-dioxotetrahydropyrimidin-1(2H)-yl)benzyl)piperazin-1-yl)-1-oxoisoindolin-2-yl)-2-(5-fluoro-2-hydroxyphenyl)-N-(thiazol-2-yl)acetamide;
2-(6-(4-((4-(2,6-dioxopiperidin-3-yl)benzyl)(methyl)amino)piperidin-1-yl)-1-oxoisoindolin-2-yl)-2-(5-fluoro-2-hydroxyphenyl)-N-(thiazol-2-yl)acetamide;
2-(6-(4-((4-(2,6-dioxopiperidin-3-yl)-3-fluorobenzyl)(methyl)amino)piperidin-1-yl)-1-oxoisoindolin-2-yl)-2-(5-fluoro-2-hydroxyphenyl)-N-(thiazol-2-yl)acetamide;
2-(6-(4-((3-(2,6-dioxopiperidin-3-yl)benzyl)(methyl)amino)piperidin-1-yl)-1-oxoisoindolin-2-yl)-2-(5-fluoro-2-hydroxyphenyl)-N-(thiazol-2-yl)acetamide;
2-(6-(4-((2-(2,6-dioxopiperidin-3-yl)benzyl)(methyl)amino)piperidin-1-yl)-1-oxoisoindolin-2-yl)-2-(5-fluoro-2-hydroxyphenyl)-N-(thiazol-2-yl)acetamide;
2-(6,7-dihydro-5H-pyrrolo[1,2-c]imidazol-1-yl)-2-(6-(4-(4-(((5-(2,6-dioxopiperidin-3-yl)-2-fluoropyridin-3-yl)methyl)(methyl)amino)piperidin-1-yl)phenyl)-4-fluoro-1-oxoisoindolin-2-yl)-N-(thiazol-2-yl)acetamide;
2-(6-(4-((4-(2,4-dioxotetrahydropyrimidin-1(2H)-yl)benzyl)(methyl)amino)piperidin-1-yl)-1-oxoisoindolin-2-yl)-2-(5-fluoro-2-hydroxyphenyl)-N-(thiazol-2-yl)acetamide;
2-(6-(4-((4-(2,4-dioxotetrahydropyrimidin-1(2H)-yl)-2-fluorobenzyl)(methyl)amino)piperidin-1-yl)-1-oxoisoindolin-2-yl)-2-(5-fluoro-2-hydroxyphenyl)-N-(thiazol-2-yl)acetamide;
2-(6-(4-((3-(2,4-dioxotetrahydropyrimidin-1(2H)-yl)benzyl)(methyl)amino)piperidin-1-yl)-1-oxoisoindolin-2-yl)-2-(5-fluoro-2-hydroxyphenyl)-N-(thiazol-2-yl)acetamide;
2-(6-(4-((2-(2,4-dioxotetrahydropyrimidin-1(2H)-yl)benzyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(((5-(2,4-dioxotetrahydropyrimidin-1(2H)-yl)pyridin-2-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(((6-(2,4-dioxotetrahydropyrimidin-1(2H)-yl)pyridazin-3-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(((3-(2,4-dioxotetrahydropyrimidin-1(2H)-yl)pyridin-4-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-((4-((2,6-dioxopiperidin-3-yl)amino)benzyl)(methyl)amino)piperidin-1-yl)-1-oxoisoindolin-2-yl)-2-(5-fluoro-2-hydroxyphenyl)-N-(thiazol-2-yl)acetamide;
2-(6-(4-((3-((2,6-dioxopiperidin-3-yl)amino)benzyl)(methyl)amino)piperidin-1-yl)-1-oxoisoindolin-2-yl)-2-(5-fluoro-2-hydroxyphenyl)-N-(thiazol-2-yl)acetamide;
2-(6-(4-((2-((2,6-dioxopiperidin-3-yl)amino)benzyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(4-(4-(2,6-dioxopiperidin-3-yl)benzyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-(4-(2,6-dioxopiperidin-3-yl)-3-fluorobenzyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-(3-(2,6-dioxopiperidin-3-yl)benzyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-(2-(2,6-dioxopiperidin-3-yl)benzyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-(4-(2,4-dioxotetrahydropyrimidin-1(2H)-yl)benzyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-(4-(2,4-dioxotetrahydropyrimidin-1(2H)-yl)-2-fluorobenzyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-(3-(2,4-dioxotetrahydropyrimidin-1(2H)-yl)benzyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-(2-(2,4-dioxotetrahydropyrimidin-1(2H)-yl)benzyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-((5-(2,4-dioxotetrahydropyrimidin-1(2H)-yl)pyridin-2-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-((6-(2,4-dioxotetrahydropyrimidin-1(2H)-yl)pyridazin-3-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-((3-(2,4-dioxotetrahydropyrimidin-1(2H)-yl)pyridin-4-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-(4-((2,6-dioxopiperidin-3-yl)amino)benzyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-(3-((2,6-dioxopiperidin-3-yl)amino)benzyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-(2-((2,6-dioxopiperidin-3-yl)amino)benzyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-(4-(2,6-dioxopiperidin-3-yl)benzyl)piperazin-1-yl)phenyl)-1-oxoisoindolin-2-yl)-2-(5-fluoro-2-hydroxyphenyl)-N-(thiazol-2-yl)acetamide;
2-(6-(4-(4-(4-(2,6-dioxopiperidin-3-yl)-3-fluorobenzyl)piperazin-1-yl)phenyl)-1-oxoisoindolin-2-yl)-2-(5-fluoro-2-hydroxyphenyl)-N-(thiazol-2-yl)acetamide;
2-(6-(4-(4-(3-(2,6-dioxopiperidin-3-yl)benzyl)piperazin-1-yl)phenyl)-1-oxoisoindolin-2-yl)-2-(5-fluoro-2-hydroxyphenyl)-N-(thiazol-2-yl)acetamide;
2-(6-(4-(4-(2-(2,6-dioxopiperidin-3-yl)benzyl)piperazin-1-yl)phenyl)-1-oxoisoindolin-2-yl)-2-(5-fluoro-2-hydroxyphenyl)-N-(thiazol-2-yl)acetamide;
2-(6-(4-(4-(4-(2,4-dioxotetrahydropyrimidin-1(2H)-yl)benzyl)piperazin-1-yl)phenyl)-1-oxoisoindolin-2-yl)-2-(5-fluoro-2-hydroxyphenyl)-N-(thiazol-2-yl)acetamide;
2-(6-(4-(4-(4-(2,4-dioxotetrahydropyrimidin-1(2H)-yl)-2-fluorobenzyl)piperazin-1-yl)phenyl)-1-oxoisoindolin-2-yl)-2-(5-fluoro-2-hydroxyphenyl)-N-(thiazol-2-yl)acetamide;
2-(6-(4-(4-(3-(2,4-dioxotetrahydropyrimidin-1(2H)-yl)benzyl)piperazin-1-yl)phenyl)-1-oxoisoindolin-2-yl)-2-(5-fluoro-2-hydroxyphenyl)-N-(thiazol-2-yl)acetamide;
2-(6-(4-(4-(2-(2,4-dioxotetrahydropyrimidin-1(2H)-yl)benzyl)piperazin-1-yl)phenyl)-1-oxoisoindolin-2-yl)-2-(5-fluoro-2-hydroxyphenyl)-N-(thiazol-2-yl)acetamide;
2-(6-(4-(4-((5-(2,4-dioxotetrahydropyrimidin-1(2H)-yl)pyridin-2-yl)methyl)piperazin-1-yl)phenyl)-1-oxoisoindolin-2-yl)-2-(5-fluoro-2-hydroxyphenyl)-N-(thiazol-2-yl)acetamide;
2-(6-(4-(4-((6-(2,4-dioxotetrahydropyrimidin-1(2H)-yl)pyridazin-3-yl)methyl)piperazin-1-yl)phenyl)-1-oxoisoindolin-2-yl)-2-(5-fluoro-2-hydroxyphenyl)-N-(thiazol-2-yl)acetamide;
2-(6-(4-(4-((3-(2,4-dioxotetrahydropyrimidin-1(2H)-yl)pyridin-4-yl)methyl)piperazin-1-yl)phenyl)-1-oxoisoindolin-2-yl)-2-(5-fluoro-2-hydroxyphenyl)-N-(thiazol-2-yl)acetamide;
2-(6-(4-(4-(4-((2,6-dioxopiperidin-3-yl)amino)benzyl)piperazin-1-yl)phenyl)-1-oxoisoindolin-2-yl)-2-(5-fluoro-2-hydroxyphenyl)-N-(thiazol-2-yl)acetamide;
2-(6-(4-(4-(3-((2,6-dioxopiperidin-3-yl)amino)benzyl)piperazin-1-yl)phenyl)-1-oxoisoindolin-2-yl)-2-(5-fluoro-2-hydroxyphenyl)-N-(thiazol-2-yl)acetamide;
2-(6-(4-(4-(2-((2,6-dioxopiperidin-3-yl)amino)benzyl)piperazin-1-yl)phenyl)-1-oxoisoindolin-2-yl)-2-(5-fluoro-2-hydroxyphenyl)-N-(thiazol-2-yl)acetamide;
2-(6-(6-(4-(4-(2,6-dioxopiperidin-3-yl)benzyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-(4-(2,6-dioxopiperidin-3-yl)-3-fluorobenzyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-(3-(2,6-dioxopiperidin-3-yl)benzyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-(2-(2,6-dioxopiperidin-3-yl)benzyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-(4-(2,4-dioxotetrahydropyrimidin-1(2H)-yl)benzyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-(4-(2,4-dioxotetrahydropyrimidin-1(2H)-yl)-2-fluorobenzyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-(3-(2,4-dioxotetrahydropyrimidin-1(2H)-yl)benzyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-(2-(2,4-dioxotetrahydropyrimidin-1(2H)-yl)benzyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((5-(2,4-dioxotetrahydropyrimidin-1(2H)-yl)pyridin-2-yl)methyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((6-(2,4-dioxotetrahydropyrimidin-1(2H)-yl)pyridazin-3-yl)methyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((3-(2,4-dioxotetrahydropyrimidin-1(2H)-yl)pyridin-4-yl)methyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-(4-((2,6-dioxopiperidin-3-yl)amino)benzyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-(3-((2,6-dioxopiperidin-3-yl)amino)benzyl)piperazin-1-yl)pyridazin-3-yl)-1-oxoisoindolin-2-yl)-2-(5-fluoro-2-hydroxyphenyl)-N-(thiazol-2-yl)acetamide;
2-(6-(6-(4-(2-((2,6-dioxopiperidin-3-yl)amino)benzyl)piperazin-1-yl)pyridazin-3-yl)-1-oxoisoindolin-2-yl)-2-(5-fluoro-2-hydroxyphenyl)-N-(thiazol-2-yl)acetamide;
2-(6,7-dihydro-5H-pyrrolo[1,2-c]imidazol-1-yl)-2-(6-(4-(4-(4-(2,6-dioxopiperidin-3-yl)benzyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(2,6-dioxopiperidin-3-yl)-3-fluorobenzyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(3-(2,6-dioxopiperidin-3-yl)benzyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(2-(2,6-dioxopiperidin-3-yl)benzyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((2-(2,6-dioxopiperidin-3-yl)pyridin-4-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((5-(2,6-dioxopiperidin-3-yl)pyridin-2-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((5-(2,6-dioxopiperidin-3-yl)pyridin-3-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((5-(2,6-dioxopiperidin-3-yl)-2-fluoropyridin-3-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(2,4-dioxotetrahydropyrimidin-1(2H)-yl)benzyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(2,4-dioxotetrahydropyrimidin-1(2H)-yl)-2-fluorobenzyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(3-(2,4-dioxotetrahydropyrimidin-1(2H)-yl)benzyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(2-(2,4-dioxotetrahydropyrimidin-1(2H)-yl)benzyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((5-(2,4-dioxotetrahydropyrimidin-1(2H)-yl)pyridin-2-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((2-(2,4-dioxotetrahydropyrimidin-1(2H)-yl)pyridin-4-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((2-(2,4-dioxotetrahydropyrimidin-1(2H)-yl)-5-fluoropyridin-4-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((6-(2,4-dioxotetrahydropyrimidin-1(2H)-yl)pyridin-3-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((5-(2,4-dioxotetrahydropyrimidin-1(2H)-yl)pyridin-3-yl)methyl)piperazin-1-yl)phenyl)-4-fluoro-1-oxoisoindolin-2-yl)-N-(thiazol-2-yl)acetamide;
1-(5-((4-(4-(2-(1-(6,7-dihydro-5H-pyrrolo[1,2-c]imidazol-1-yl)-2-(thiazol-2-ylamino)ethyl)-7-fluoro-3-oxoisoindolin-5-yl)phenyl)piperazin-1-yl)methyl)-6-fluoropyridin-3-yl)dihydropyrimidine-2,4(1H,3H)-dione;
2-(6,7-dihydro-5H-pyrrolo[1,2-c]imidazol-1-yl)-2-(6-(4-(4-((6-(2,4-dioxotetrahydropyrimidin-1(2H)-yl)pyridazin-3-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((5-(2,4-dioxotetrahydropyrimidin-1(2H)-yl)pyrazin-2-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((3-(2,4-dioxotetrahydropyrimidin-1(2H)-yl)pyridin-4-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-((2,6-dioxopiperidin-3-yl)amino)benzyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(3-((2,6-dioxopiperidin-3-yl)amino)benzyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(2-((2,6-dioxopiperidin-3-yl)amino)benzyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((2-((2,6-dioxopiperidin-3-yl)amino)pyridin-3-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-(4-(2,6-dioxopiperidin-3-yl)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-(4-(2,6-dioxopiperidin-3-yl)-3-fluorobenzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-(3-(2,6-dioxopiperidin-3-yl)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-(2-(2,6-dioxopiperidin-3-yl)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-(4-(2,4-dioxotetrahydropyrimidin-1(2H)-yl)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-(4-(2,4-dioxotetrahydropyrimidin-1(2H)-yl)-2-fluorobenzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-SH-pyrrolo[1,2-c]imidazol-1-yl)-2-(6-(4-((1S,4S)-5-(3-(2,4-dioxotetrahydropyrimidin-1(2H)-yl)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-(2-(2,4-dioxotetrahydropyrimidin-1(2H)-yl)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-((5-(2,4-dioxotetrahydropyrimidin-1(2H)-yl)pyridin-2-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-((6-(2,4-dioxotetrahydropyrimidin-1(2H)-yl)pyridin-3-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-((6-(2,4-dioxotetrahydropyrimidin-1(2H)-yl)pyridazin-3-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-((3-(2,4-dioxotetrahydropyrimidin-1(2H)-yl)pyridin-4-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-(4-((2,6-dioxopiperidin-3-yl)amino)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-(3-((2,6-dioxopiperidin-3-yl)amino)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-(2-((2,6-dioxopiperidin-3-yl)amino)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-(4-(2,6-dioxopiperidin-3-yl)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-(4-(2,6-dioxopiperidin-3-yl)-3-fluorobenzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-(3-(2,6-dioxopiperidin-3-yl)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-(2-(2,6-dioxopiperidin-3-yl)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((2-(2,6-dioxopiperidin-3-yl)pyridin-4-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((2-(2,6-dioxopiperidin-3-yl)-5-fluoropyridin-4-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((5-(2,6-dioxopiperidin-3-yl)pyridin-2-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((5-(2,6-dioxopiperidin-3-yl)pyridin-3-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((5-(2,6-dioxopiperidin-3-yl)-2-fluoropyridin-3-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-(4-(2,4-dioxotetrahydropyrimidin-1(2H)-yl)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-(4-(2,4-dioxotetrahydropyrimidin-1(2H)-yl)-2-fluorobenzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-(3-(2,4-dioxotetrahydropyrimidin-1(2H)-yl)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-(2-(2,4-dioxotetrahydropyrimidin-1(2H)-yl)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((5-(2,4-dioxotetrahydropyrimidin-1(2H)-yl)pyridin-2-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((5-(2,4-dioxotetrahydropyrimidin-1(2H)-yl)-3-fluoropyridin-2-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((2-(2,4-dioxotetrahydropyrimidin-1(2H)-yl)pyridin-4-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((2-(2,4-dioxotetrahydropyrimidin-1(2H)-yl)-5-fluoropyridin-4-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((6-(2,4-dioxotetrahydropyrimidin-1(2H)-yl)pyridin-3-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((5-(2,4-dioxotetrahydropyrimidin-1(2H)-yl)pyridin-3-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((5-(2,4-dioxotetrahydropyrimidin-1(2H)-yl)-2-fluoropyridin-3-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((6-(2,4-dioxotetrahydropyrimidin-1(2H)-yl)pyridazin-3-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((5-(2,4-dioxotetrahydropyrimidin-1(2H)-yl)pyrazin-2-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((6-(2,4-dioxotetrahydropyrimidin-1(2H)-yl)pyrazin-2-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((3-(2,4-dioxotetrahydropyrimidin-1(2H)-yl)pyridin-4-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-(4-((2,6-dioxopiperidin-3-yl)amino)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-(3-((2,6-dioxopiperidin-3-yl)amino)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-(2-((2,6-dioxopiperidin-3-yl)amino)benzyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-(4-(2,6-dioxopiperidin-3-yl)benzyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-(4-(2,6-dioxopiperidin-3-yl)-3-fluorobenzyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-(3-(2,6-dioxopiperidin-3-yl)benzyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-(2-(2,6-dioxopiperidin-3-yl)benzyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-(4-(2,4-dioxotetrahydropyrimidin-1(2H)-yl)benzyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-(4-(2,4-dioxotetrahydropyrimidin-1(2H)-yl)-2-fluorobenzyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-(3-(2,4-dioxotetrahydropyrimidin-1(2H)-yl)benzyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-(2-(2,4-dioxotetrahydropyrimidin-1(2H)-yl)benzyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-((5-(2,4-dioxotetrahydropyrimidin-1(2H)-yl)pyridin-2-yl)methyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-((6-(2,4-dioxotetrahydropyrimidin-1(2H)-yl)pyridin-3-yl)methyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-((6-(2,4-dioxotetrahydropyrimidin-1(2H)-yl)pyridazin-3-yl)methyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-((3-(2,4-dioxotetrahydropyrimidin-1(2H)-yl)pyridin-4-yl)methyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-(4-((2,6-dioxopiperidin-3-yl)amino)benzyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-(3-((2,6-dioxopiperidin-3-yl)amino)benzyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-(2-((2,6-dioxopiperidin-3-yl)amino)benzyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((4-(2,6-dioxopiperidin-3-yl)benzyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((4-(2,6-dioxopiperidin-3-yl)-3-fluorobenzyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((3-(2,6-dioxopiperidin-3-yl)benzyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((2-(2,6-dioxopiperidin-3-yl)benzyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((2-(2,6-dioxopiperidin-3-yl)pyridin-4-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((2-(2,6-dioxopiperidin-3-yl)-5-fluoropyridin-4-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((5-(2,6-dioxopiperidin-3-yl)pyridin-2-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((5-(2,6-dioxopiperidin-3-yl)pyridin-3-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((4-(2,4-dioxotetrahydropyrimidin-1(2H)-yl)benzyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((4-(2,4-dioxotetrahydropyrimidin-1(2H)-yl)-2-fluorobenzyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((3-(2,4-dioxotetrahydropyrimidin-1(2H)-yl)benzyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((2-(2,4-dioxotetrahydropyrimidin-1(2H)-yl)benzyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((5-(2,4-dioxotetrahydropyrimidin-1(2H)-yl)pyridin-2-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((2-(2,4-dioxotetrahydropyrimidin-1(2H)-yl)pyridin-4-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((5-(2,4-dioxotetrahydropyrimidin-1(2H)-yl)-3-fluoropyridin-2-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((2-(2,4-dioxotetrahydropyrimidin-1(2H)-yl)-5-fluoropyridin-4-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((6-(2,4-dioxotetrahydropyrimidin-1(2H)-yl)pyridin-3-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((5-(2,4-dioxotetrahydropyrimidin-1(2H)-yl)pyridin-3-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((5-(2,4-dioxotetrahydropyrimidin-1(2H)-yl)-2-fluoropyridin-3-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((6-(2,4-dioxotetrahydropyrimidin-1(2H)-yl)pyridazin-3-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((5-(2,4-dioxotetrahydropyrimidin-1(2H)-yl)pyrazin-2-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((6-(2,4-dioxotetrahydropyrimidin-1(2H)-yl)pyrazin-2-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((3-(2,4-dioxotetrahydropyrimidin-1(2H)-yl)pyridin-4-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((4-((2,6-dioxopiperidin-3-yl)amino)benzyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((3-((2,6-dioxopiperidin-3-yl)amino)benzyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((2-((2,6-dioxopiperidin-3-yl)amino)benzyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((2-((2,6-dioxopiperidin-3-yl)amino)pyridin-3-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(4-(2,6-dioxopiperidin-3-yl)benzyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(4-(2,6-dioxopiperidin-3-yl)-3-fluorobenzyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(3-(2,6-dioxopiperidin-3-yl)benzyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(2-(2,6-dioxopiperidin-3-yl)benzyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(4-(2,4-dioxotetrahydropyrimidin-1(2H)-yl)benzyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(4-(2,4-dioxotetrahydropyrimidin-1(2H)-yl)-2-fluorobenzyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(3-(2,4-dioxotetrahydropyrimidin-1(2H)-yl)benzyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(2-(2,4-dioxotetrahydropyrimidin-1(2H)-yl)benzyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-((5-(2,4-dioxotetrahydropyrimidin-1(2H)-yl)pyridin-2-yl)methyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-((6-(2,4-dioxotetrahydropyrimidin-1(2H)-yl)pyridazin-3-yl)methyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-((3-(2,4-dioxotetrahydropyrimidin-1(2H)-yl)pyridin-4-yl)methyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(4-((2,6-dioxopiperidin-3-yl)amino)benzyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(3-((2,6-dioxopiperidin-3-yl)amino)benzyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(2-((2,6-dioxopiperidin-3-yl)amino)benzyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(4-(2,6-dioxopiperidin-3-yl)benzyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(4-(2,6-dioxopiperidin-3-yl)-3-fluorobenzyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(3-(2,6-dioxopiperidin-3-yl)benzyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(2-(2,6-dioxopiperidin-3-yl)benzyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(4-(2,4-dioxotetrahydropyrimidin-1(2H)-yl)benzyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(4-(2,4-dioxotetrahydropyrimidin-1(2H)-yl)-2-fluorobenzyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(3-(2,4-dioxotetrahydropyrimidin-1(2H)-yl)benzyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(2-(2,4-dioxotetrahydropyrimidin-1(2H)-yl)benzyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-((5-(2,4-dioxotetrahydropyrimidin-1(2H)-yl)pyridin-2-yl)methyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-((6-(2,4-dioxotetrahydropyrimidin-1(2H)-yl)pyridin-3-yl)methyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-((6-(2,4-dioxotetrahydropyrimidin-1(2H)-yl)pyridazin-3-yl)methyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-((3-(2,4-dioxotetrahydropyrimidin-1(2H)-yl)pyridin-4-yl)methyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(4-((2,6-dioxopiperidin-3-yl)amino)benzyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(3-((2,6-dioxopiperidin-3-yl)amino)benzyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-(2-((2,6-dioxopiperidin-3-yl)amino)benzyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(4-(2,6-dioxopiperidin-3-yl)benzyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(4-(2,6-dioxopiperidin-3-yl)-3-fluorobenzyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(3-(2,6-dioxopiperidin-3-yl)benzyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(2-(2,6-dioxopiperidin-3-yl)benzyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(4-(2,4-dioxotetrahydropyrimidin-1(2H)-yl)benzyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(4-(2,4-dioxotetrahydropyrimidin-1(2H)-yl)-2-fluorobenzyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(3-(2,4-dioxotetrahydropyrimidin-1(2H)-yl)benzyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(2-(2,4-dioxotetrahydropyrimidin-1(2H)-yl)benzyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-((5-(2,4-dioxotetrahydropyrimidin-1(2H)-yl)pyridin-2-yl)methyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-((6-(2,4-dioxotetrahydropyrimidin-1(2H)-yl)pyridin-3-yl)methyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-((6-(2,4-dioxotetrahydropyrimidin-1(2H)-yl)pyridazin-3-yl)methyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-((3-(2,4-dioxotetrahydropyrimidin-1(2H)-yl)pyridin-4-yl)methyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(4-((2,6-dioxopiperidin-3-yl)amino)benzyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(3-((2,6-dioxopiperidin-3-yl)amino)benzyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(2-((2,6-dioxopiperidin-3-yl)amino)benzyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(4-(2,6-dioxopiperidin-3-yl)benzyl)-3,3-difluoropiperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(4-(2,6-dioxopiperidin-3-yl)-3-fluorobenzyl)-3,3-difluoropiperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(3-(2,6-dioxopiperidin-3-yl)benzyl)-3,3-difluoropiperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(2-(2,6-dioxopiperidin-3-yl)benzyl)-3,3-difluoropiperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(4-(2,4-dioxotetrahydropyrimidin-1(2H)-yl)benzyl)-3,3-difluoropiperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(4-(2,4-dioxotetrahydropyrimidin-1(2H)-yl)-2-fluorobenzyl)-3,3-difluoropiperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(3-(2,4-dioxotetrahydropyrimidin-1(2H)-yl)benzyl)-3,3-difluoropiperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(2-(2,4-dioxotetrahydropyrimidin-1(2H)-yl)benzyl)-3,3-difluoropiperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-((5-(2,4-dioxotetrahydropyrimidin-1(2H)-yl)pyridin-2-yl)methyl)-3,3-difluoropiperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-((6-(2,4-dioxotetrahydropyrimidin-1(2H)-yl)pyridazin-3-yl)methyl)-3,3-difluoropiperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-((3-(2,4-dioxotetrahydropyrimidin-1(2H)-yl)pyridin-4-yl)methyl)-3,3-difluoropiperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(4-((2,6-dioxopiperidin-3-yl)amino)benzyl)-3,3-difluoropiperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(3-((2,6-dioxopiperidin-3-yl)amino)benzyl)-3,3-difluoropiperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-(2-((2,6-dioxopiperidin-3-yl)amino)benzyl)-3,3-difluoropiperidin-4-yl)piperazin-1-yl)phenyl)-4-fluoro-1-oxoisoindolin-2-yl)-N-(thiazol-2-yl)acetamide;
3-(4-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)phenyl)piperidine-2,6-dione;
3-(4-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-2-fluorophenyl)piperidine-2,6-dione;
3-(3-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)phenyl)piperidine-2,6-dione;
3-(2-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)phenyl)piperidine-2,6-dione;
1-(4-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)phenyl)dihydropyrimidine-2,4(1H,3H)-dione;
1-(4-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-3-fluorophenyl)dihydropyrimidine-2,4(1H,3H)-dione;
1-(3-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)phenyl)dihydropyrimidine-2,4(1H,3H)-dione;
1-(2-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)phenyl)dihydropyrimidine-2,4(1H,3H)-dione;
1-(6-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)pyridin-3-yl)dihydropyrimidine-2,4(1H,3H)-dione;
1-(5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)pyridin-2-yl)dihydropyrimidine-2,4(1H,3H)-dione;
1-(6-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)pyridazin-3-yl)dihydropyrimidine-2,4(1H,3H)-dione;
1-(4-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)pyridin-3-yl)dihydropyrimidine-2,4(1H,3H)-dione;
3-((4-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)phenyl)amino)piperidine-2,6-dione;
3-((3-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)phenyl)amino)piperidine-2,6-dione;
3-((2-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)phenyl)amino)piperidine-2,6-dione;
3-(4-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)phenyl)piperidine-2,6-dione;
3-(4-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-2-fluorophenyl)piperidine-2,6-dione;
3-(3-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)phenyl)piperidine-2,6-dione;
3-(2-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)phenyl)piperidine-2,6-dione;
1-(4-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)phenyl)dihydropyrimidine-2,4(1H,3H)-dione;
1-(4-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-3-fluorophenyl)dihydropyrimidine-2,4(1H,3H)-dione;
1-(3-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)phenyl)dihydropyrimidine-2,4(1H,3H)-dione;
1-(2-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)phenyl)dihydropyrimidine-2,4(1H,3H)-dione;
1-(6-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)pyridin-3-yl)dihydropyrimidine-2,4(1H,3H)-dione;
1-(5-(((1-(6-((2-(1-(cyclopropylsulfonyl)-1H-pyrazol-4-yl)pyrimidin-4-yl)amino)-1-ethyl-1H-pyrazolo[4,3-c]pyridin-3-yl)piperidin-4-yl)(methyl)amino)methyl)pyridin-2-yl)dihydropyrimidine-2,4(1H,3H)-dione;
1-(6-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)pyridazin-3-yl)dihydropyrimidine-2,4(1H,3H)-dione;
1-(4-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)pyridin-3-yl)dihydropyrimidine-2,4(1H,3H)-dione;
3-((4-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)phenyl)amino)piperidine-2,6-dione;
3-((3-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)phenyl)amino)piperidine-2,6-dione;
3-((2-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)phenyl)amino)piperidine-2,6-dione;
3-(4-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)phenyl)piperidine-2,6-dione;
3-(4-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-2-fluorophenyl)piperidine-2,6-dione;
3-(3-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)phenyl)piperidine-2,6-dione;
3-(2-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)phenyl)piperidine-2,6-dione;
1-(4-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)phenyl)dihydropyrimidine-2,4(1H,3H)-dione;
1-(4-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-3-fluorophenyl)dihydropyrimidine-2,4(1H,3H)-dione;
1-(3-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)phenyl)dihydropyrimidine-2,4(1H,3H)-dione;
1-(2-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)phenyl)dihydropyrimidine-2,4(1,3H)-dione;
1-(6-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)pyridin-3-yl)dihydropyrimidine-2,4(1H,3H)-dione;
1-(5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)pyridin-2-yl)dihydropyrimidine-2,4(1H,3H)-dione;
1-(6-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)pyridazin-3-yl)dihydropyrimidine-2,4(1H,3H)-dione;
1-(4-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)pyridin-3-yl)dihydropyrimidine-2,4(1H,3H)-dione;
3-((4-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)phenyl)amino)piperidine-2,6-dione;
3-((3-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)phenyl)amino)piperidine-2,6-dione;
3-((2-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)phenyl)amino)piperidine-2,6-dione;
3-(4-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)phenyl)piperidine-2,6-dione;
3-(2-fluoro-4-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)phenyl)piperidine-2,6-dione;
3-(3-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)phenyl)piperidine-2,6-dione;
3-(2-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)phenyl)piperidine-2,6-dione;
1-(4-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)phenyl)dihydropyrimidine-2,4(1H,3H)-dione;
1-(3-fluoro-4-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)phenyl)dihydropyrimidine-2,4(1H,3H)-dione;
1-(3-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)phenyl)dihydropyrimidine-2,4(1H,3H)-dione;
1-(2-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)phenyl)dihydropyrimidine-2,4(1H,3H)-dione;
1-(6-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)pyridin-3-yl)dihydropyrimidine-2,4(1H,3H)-dione;
1-(5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)pyridin-2-yl)dihydropyrimidine-2,4(1H,3H)-dione;
3-((3-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)phenyl)amino)piperidine-2,6-dione;
3-(4-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)piperidine-2,6-dione;
3-(2-fluoro-4-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)piperidine-2,6-dione;
3-(3-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)piperidine-2,6-dione;
3-(2-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)piperidine-2,6-dione;
1-(4-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)dihydropyrimidine-2,4(1H,3H)-dione;
1-(3-fluoro-4-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)dihydropyrimidine-2,4(1H,3H)-dione;
1-(3-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)dihydropyrimidine-2,4(1H,3H)-dione;
1-(2-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)dihydropyrimidine-2,4(1H,3H)-dione;
1-(6-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)pyridin-3-yl)dihydropyrimidine-2,4(1H,3H)-dione;
1-(5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)pyridin-2-yl)dihydropyrimidine-2,4(1H,3H)-dione;
1-(6-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)pyridazin-3-yl)dihydropyrimidine-2,4(1H,3H)-dione;
1-(4-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)pyridin-3-yl)dihydropyrimidine-2,4(1H,3H)-dione;
3-((4-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)amino)piperidine-2,6-dione;
3-((3-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)amino)piperidine-2,6-dione;
3-((2-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)phenyl)amino)piperidine-2,6-dione;
3-(4-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)phenyl)piperidine-2,6-dione;
3-(2-fluoro-4-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)phenyl)piperidine-2,6-dione;
3-(3-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)phenyl)piperidine-2,6-dione;
3-(2-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)phenyl)piperidine-2,6-dione;
3-(6-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)pyridin-3-yl)piperidine-2,6-dione;
3-(4-fluoro-6-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)pyridazin-3-yl)piperidine-2,6-dione;
3-(4-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)pyridin-3-yl)piperidine-2,6-dione;
3-((4-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)phenyl)amino)piperidine-2,6-dione;
3-((3-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)phenyl)amino)piperidine-2,6-dione;
3-((2-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)phenyl)amino)piperidine-2,6-dione;
N-(5-(((5-(tert-butyl)oxazol-2-yl)methyl)thio)thiazol-2-yl)-1-(4-(2,6-dioxopiperidin-3-yl)benzyl)piperidine-4-carboxamide;
N-(5-(((5-(tert-butyl)oxazol-2-yl)methyl)thio)thiazol-2-yl)-1-(4-(2,6-dioxopiperidin-3-yl)-3-fluorobenzyl)piperidine-4-carboxamide;
N-(5-(((5-(tert-butyl)oxazol-2-yl)methyl)thio)thiazol-2-yl)-1-(3-(2,6-dioxopiperidin-3-yl)benzyl)piperidine-4-carboxamide;
N-(5-(((5-(tert-butyl)oxazol-2-yl)methyl)thio)thiazol-2-yl)-1-(2-(2,6-dioxopiperidin-3-yl)benzyl)piperidine-4-carboxamide;
N-(5-(((5-(tert-butyl)oxazol-2-yl)methyl)thio)thiazol-2-yl)-1-(4-(2,4-dioxotetrahydropyrimidin-1(2H)-yl)benzyl)piperidine-4-carboxamide;
N-(5-(((5-(tert-butyl)oxazol-2-yl)methyl)thio)thiazol-2-yl)-1-(4-(2,4-dioxotetrahydropyrimidin-1(2H)-yl)-2-fluorobenzyl)piperidine-4-carboxamide;
N-(5-(((5-(tert-butyl)oxazol-2-yl)methyl)thio)thiazol-2-yl)-1-(3-(2,4-dioxotetrahydropyrimidin-1(2H)-yl)benzyl)piperidine-4-carboxamide;
N-(5-(((5-(tert-butyl)oxazol-2-yl)methyl)thio)thiazol-2-yl)-1-(2-(2,4-dioxotetrahydropyrimidin-1(2H)-yl)benzyl)piperidine-4-carboxamide;
N-(5-(((5-(tert-butyl)oxazol-2-yl)methyl)thio)thiazol-2-yl)-1-((5-(2,4-dioxotetrahydropyrimidin-1(2H)-yl)pyridin-2-yl)methyl)piperidine-4-carboxamide;
N-(5-(((5-(tert-butyl)oxazol-2-yl)methyl)thio)thiazol-2-yl)-1-((6-(2,4-dioxotetrahydropyrimidin-1(2H)-yl)pyridazin-3-yl)methyl)piperidine-4-carboxamide;
N-(5-(((5-(tert-butyl)oxazol-2-yl)methyl)thio)thiazol-2-yl)-1-((3-(2,4-dioxotetrahydropyrimidin-1(2H)-yl)pyridin-4-yl)methyl)piperidine-4-carboxamide;
N-(5-(((5-(tert-butyl)oxazol-2-yl)methyl)thio)thiazol-2-yl)-1-(4-((2,6-dioxopiperidin-3-yl)amino)benzyl)piperidine-4-carboxamide;
N-(5-(((5-(tert-butyl)oxazol-2-yl)methyl)thio)thiazol-2-yl)-1-(3-((2,6-dioxopiperidin-3-yl)amino)benzyl)piperidine-4-carboxamide;
N-(5-(((5-(tert-butyl)oxazol-2-yl)methyl)thio)thiazol-2-yl)-1-(2-((2,6-dioxopiperidin-3-yl)amino)benzyl)piperidine-4-carboxamide;
N-(5-(((5-(tert-butyl)oxazol-2-yl)methyl)thio)thiazol-2-yl)-1'-(4-(2,6-dioxopiperidin-3-yl)benzyl)-[1,4'-bipiperidine]-4-carboxamide;
N-(5-(((5-(tert-butyl)oxazol-2-yl)methyl)thio)thiazol-2-yl)-1-(4-(2,6-dioxopiperidin-3-yl)-3-fluorobenzyl)-[1,4'-bipiperidine]-4-carboxamide;
N-(5-(((5-(tert-butyl)oxazol-2-yl)methyl)thio)thiazol-2-yl)-1'-(3-(2,6-dioxopiperidin-3-yl)benzyl)-[1,4'-bipiperidine]-4-carboxamide;
N-(5-(((5-(tert-butyl)oxazol-2-yl)methyl)thio)thiazol-2-yl)-1'-(2-(2,6-dioxopiperidin-3-yl)benzyl)-[1,4'-bipiperidine]-4-carboxamide;
N-(5-(((5-(tert-butyl)oxazol-2-yl)methyl)thio)thiazol-2-yl)-1-(4-(2,4-dioxotetrahydropyrimidin-1(2H)-yl)benzyl)-[1,4'-bipiperidine]-4-carboxamide;
N-(5-(((5-(tert-butyl)oxazol-2-yl)methyl)thio)thiazol-2-yl)-1-(4-(2,4-dioxotetrahydropyrimidin-1(2H)-yl)-2-fluorobenzyl)-[1,4'-bipiperidine]-4-carboxamide;
N-(5-(((5-(tert-butyl)oxazol-2-yl)methyl)thio)thiazol-2-yl)-1-(3-(2,4-dioxotetrahydropyrimidin-1(2H)-yl)benzyl)-[1,4'-bipiperidine]-4-carboxamide;
N-(5-(((5-(tert-butyl)oxazol-2-yl)methyl)thio)thiazol-2-yl)-1-(2-(2,4-dioxotetrahydropyrimidin-1(2H)-yl)benzyl)-[1,4'-bipiperidine]-4-carboxamide;
N-(5-(((5-(tert-butyl)oxazol-2-yl)methyl)thio)thiazol-2-yl)-1-((5-(2,4-dioxotetrahydropyrimidin-12H)-yl)pyridin-2-yl)methyl)-[1,4'-bipiperidine]-4-carboxamide;
N-(5-(((5-(tert-butyl)oxazol-2-yl)methyl)thio)thiazol-2-yl)-1-((6-(2,4-dioxotetrahydropyrimidin-1(2H)-yl)pyridazin-3-yl)methyl)-[1,4'-bipiperidine]-4-carboxamide;
N-(5-(((5-(tert-butyl)oxazol-2-yl)methyl)thio)thiazol-2-yl)-1'-((3-(2,4-dioxotetrahydropyrimidin-1(2H)-yl)pyridin-4-yl)methyl)-[1,4'-bipiperidine]-4-carboxamide;
N-(5-(((5-(tert-butyl)oxazol-2-yl)methyl)thio)thiazol-2-yl)-1'-(4-((2,6-dioxopiperidin-3-yl)amino)benzyl)-[1,4'-bipiperidine]-4-carboxamide;
N-(5-(((5-(tert-butyl)oxazol-2-yl)methyl)thio)thiazol-2-yl)-1'-(3-((2,6-dioxopiperidin-3-yl)amino)benzyl)-[1,4'-bipiperidine]-4-carboxamide;
N-(5-(((5-(tert-butyl)oxazol-2-yl)methyl)thio)thiazol-2-yl)-1'-(2-((2,6-dioxopiperidin-3-yl)amino)benzyl)-[1,4'-bipiperidine]-4-carboxamide;
4-(2,6-dichlorobenzamido)-N-(1-(4-(2,6-dioxopiperidin-3-yl)benzyl)piperidin-4-yl)-1H-pyrazole-3-carboxamide;
4-(2,6-dichlorobenzamido)-N-(1-(4-(2,6-dioxopiperidin-3-yl)-3-fluorobenzyl)piperidin-4-yl)-1H-pyrazole-3-carboxamide;
4-(2,6-dichlorobenzamido)-N-(1-(3-(2,6-dioxopiperidin-3-yl)benzyl)piperidin-4-yl)-1H-pyrazole-3-carboxamide;
4-(2,6-dichlorobenzamido)-N-(1-(2-(2,6-dioxopiperidin-3-yl)benzyl)piperidin-4-yl)-1H-pyrazole-3-carboxamide;
4-(2,6-dichlorobenzamido)-N-(1-((5-(2,6-dioxopiperidin-3-yl)pyridin-2-yl)methyl)piperidin-4-yl)-1H-pyrazole-3-carboxamide;
4-(2,6-dichlorobenzamido)-N-(1-(4-(2,4-dioxotetrahydropyrimidin-1(2H)-yl)-2-fluorobenzyl)piperidin-4-yl)-1H-pyrazole-3-carboxamide;
4-(2,6-dichlorobenzamido)-N-(1-(3-(2,4-dioxotetrahydropyrimidin-1(2H)-yl)benzyl)piperidin-4-yl)-1H-pyrazole-3-carboxamide;
4-(2,6-dichlorobenzamido)-N-(1-(2-(2,4-dioxotetrahydropyrimidin-1(2H)-yl)benzyl)piperidin-4-yl)-1H-pyrazole-3-carboxamide;
4-(2,6-dichlorobenzamido)-N-(1-((5-(2,6-dioxopiperidin-3-yl)pyridin-2-yl)methyl)piperidin-4-yl)-1H-pyrazole-3-carboxamide;
4-(2,6-dichlorobenzamido)-N-(1-((6-(2,6-dioxopiperidin-3-yl)pyridazin-3-yl)methyl)piperidin-4-yl)-1H-pyrazole-3-carboxamide;
4-(2,6-dichlorobenzamido)-N-(1-((3-(2,4-dioxotetrahydropyrimidin-1(2H)-yl)pyridin-4-yl)methyl)piperidin-4-yl)-1H-pyrazole-3-carboxamide;
4-(2,6-dichlorobenzamido)-N-(1-(4-((2,6-dioxopiperidin-3-yl)amino)benzyl)piperidin-4-yl)-1H-pyrazole-3-carboxamide;
4-(2,6-dichlorobenzamido)-N-(1-(3-((2,6-dioxopiperidin-3-yl)amino)benzyl)piperidin-4-yl)-1H-pyrazole-3-carboxamide;
4-(2,6-dichlorobenzamido)-N-(1-(2-((2,6-dioxopiperidin-3-yl)amino)benzyl)piperidin-4-yl)-1H-pyrazole-3-carboxamide;
N-(5-chloro-4-(5,5-dimethyl-5,6-dihydro-4H-pyrrolo[1,2-b]pyrazol-3-yl)pyridin-2-yl)-1-(4-(2,6-dioxopiperidin-3-yl)benzyl)piperidine-4-carboxamide;
N-(5-chloro-4-(5,5-dimethyl-5,6-dihydro-4H-pyrrolo[1,2-b]pyrazol-3-yl)pyridin-2-yl)-1-(4-(2,6-dioxopiperidin-3-yl)-3-fluorobenzyl)piperidine-4-carboxamide;
N-(5-chloro-4-(5,5-dimethyl-5,6-dihydro-4H-pyrrolo[1,2-b]pyrazol-3-yl)pyridin-2-yl)-1-(3-(2,6-dioxopiperidin-3-yl)benzyl)piperidine-4-carboxamide;
N-(5-chloro-4-(5,5-dimethyl-5,6-dihydro-4H-pyrrolo[1,2-b]pyrazol-3-yl)pyridin-2-yl)-1-(2-(2,6-dioxopiperidin-3-yl)benzyl)piperidine-4-carboxamide;
N-(5-chloro-4-(5,5-dimethyl-5,6-dihydro-4H-pyrrolo[1,2-b]pyrazol-3-yl)pyridin-2-yl)-1-(4-(2,4-dioxotetrahydropyrimidin-1(2H)-yl)benzyl)piperidine-4-carboxamide;
N-(5-chloro-4-(5,5-dimethyl-5,6-dihydro-4H-pyrrolo[1,2-b]pyrazol-3-yl)pyridin-2-yl)-1-(4-(2,4-dioxotetrahydropyrimidin-1(2H)-yl)-2-fluorobenzyl)piperidine-4-carboxamide;
N-(5-chloro-4-(5,5-dimethyl-5,6-dihydro-4H-pyrrolo[1,2-b]pyrazol-3-yl)pyridin-2-yl)-1-(3-(2,4-dioxotetrahydropyrimidin-1(2H)-yl)benzyl)piperidine-4-carboxamide;
N-(5-chloro-4-(5,5-dimethyl-5,6-dihydro-4H-pyrrolo[1,2-b]pyrazol-3-yl)pyridin-2-yl)-1-(2-(2,4-dioxotetrahydropyrimidin-1(2H)-yl)benzyl)piperidine-4-carboxamide;
N-(5-chloro-4-(5,5-dimethyl-5,6-dihydro-4H-pyrrolo[1,2-b]pyrazol-3-yl)pyridin-2-yl)-1-((5-(2,4-dioxotetrahydropyrimidin-1(2H)-yl)pyridin-2-yl)methyl)piperidine-4-carboxamide;
N-(5-chloro-4-(5,5-dimethyl-5,6-dihydro-4H-pyrrolo[1,2-b]pyrazol-3-yl)pyridin-2-yl)-1-((6-(2,4-dioxotetrahydropyrimidin-1(2H)-yl)pyridazin-3-yl)methyl)piperidine-4-carboxamide;
N-(5-chloro-4-(5,5-dimethyl-5,6-dihydro-4H-pyrrolo[1,2-b]pyrazol-3-yl)pyridin-2-yl)-1-((3-(2,4-dioxotetrahydropyrimidin-1(2H)-yl)pyridin-4-yl)methyl)piperidine-4-carboxamide;
N-(5-chloro-4-(5,5-dimethyl-5,6-dihydro-4H-pyrrolo[1,2-b]pyrazol-3-yl)pyridin-2-yl)-1-(4-((2,6-dioxopiperidin-3-yl)amino)benzyl)piperidine-4-carboxamide;
N-(5-chloro-4-(5,5-dimethyl-5,6-dihydro-4H-pyrrolo[1,2-b]pyrazol-3-yl)pyridin-2-yl)-1-(3-((2,6-dioxopiperidin-3-yl)amino)benzyl)piperidine-4-carboxamide;
N-(5-chloro-4-(5,5-dimethyl-5,6-dihydro-4H-pyrrolo[1,2-b]pyrazol-3-yl)pyridin-2-yl)-1-(2-((2,6-dioxopiperidin-3-yl)amino)benzyl)piperidine-4-carboxamide;
N-(5-cyclobutyl-1H-pyrazol-3-yl)-2-(4-((4-(2,6-dioxopiperidin-3-yl)benzyl)oxy)phenyl)acetamide;
N-(5-cyclobutyl-1H-pyrazol-3-yl)-2-(4-((4-(2,6-dioxopiperidin-3-yl)-3-fluorobenzyl)oxy)phenyl)acetamide;
N-(5-cyclobutyl-1H-pyrazol-3-yl)-2-(4-((3-(2,6-dioxopiperidin-3-yl)benzyl)oxy)phenyl)acetamide;
N-(5-cyclobutyl-1H-pyrazol-3-yl)-2-(4-((2-(2,6-dioxopiperidin-3-yl)benzyl)oxy)phenyl)acetamide;
N-(5-cyclobutyl-1H-pyrazol-3-yl)-2-(4-((4-(2,4-dioxotetrahydropyrimidin-1(2H)-yl)benzyl)oxy)phenyl)acetamide;
N-(5-cyclobutyl-1H-pyrazol-3-yl)-2-(4-((4-(2,4-dioxotetrahydropyrimidin-1(2H)-yl)-2-fluorobenzyl)oxy)phenyl)acetamide;
N-(5-cyclobutyl-1H-pyrazol-3-yl)-2-(4-((3-(2,4-dioxotetrahydropyrimidin-1(2H)-yl)benzyl)oxy)phenyl)acetamide;
N-(5-cyclobutyl-1H-pyrazol-3-yl)-2-(4-((2-(2,4-dioxotetrahydropyrimidin-1(2H)-yl)benzyl)oxy)phenyl)acetamide;
N-(5-cyclobutyl-1H-pyrazol-3-yl)-2-(4-((5-(2,4-dioxotetrahydropyrimidin-1(2H)-yl)pyridin-2-yl)methoxy)phenyl)acetamide;
N-(5-cyclobutyl-1H-pyrazol-3-yl)-2-(4-((6-(2,4-dioxotetrahydropyrimidin-1(2H)-yl)pyridazin-3-yl)methoxy)phenyl)acetamide;
N-(5-cyclobutyl-1H-pyrazol-3-yl)-2-(4-((3-(2,4-dioxotetrahydropyrimidin-1(2H)-yl)pyridin-4-yl)methoxy)phenyl)acetamide;
N-(5-cyclobutyl-1H-pyrazol-3-yl)-2-(4-((4-((2,6-dioxopiperidin-3-yl)amino)benzyl)oxy)phenyl)acetamide;
N-(5-cyclobutyl-1H-pyrazol-3-yl)-2-(4-((3-((2,6-dioxopiperidin-3-yl)amino)benzyl)oxy)phenyl)acetamide;
N-(5-cyclobutyl-1H-pyrazol-3-yl)-2-(4-((2-((2,6-dioxopiperidin-3-yl)amino)benzyl)oxy)phenyl)acetamide;
3-(4-((4-((5-chloro-4-(5-(cyclopropylmethyl)-1-methyl-1H-pyrazol-4-yl)pyrimidin-2-yl)amino)piperidin-1-yl)methyl)phenyl)piperidine-2,6-dione;
3-(4-((4-((5-chloro-4-(5-(cyclopropylmethyl)-1-methyl-1H-pyrazol-4-yl)pyrimidin-2-yl)amino)piperidin-1-yl)methyl)-2-fluorophenyl)piperidine-2,6-dione;
3-(3-((4-((5-chloro-4-(5-(cyclopropylmethyl)-1-methyl-1H-pyrazol-4-yl)pyrimidin-2-yl)amino)piperidin-1-yl)methyl)phenyl)piperidine-2,6-dione;
3-(2-((4-((5-chloro-4-(5-(cyclopropylmethyl)-1-methyl-1H-pyrazol-4-yl)pyrimidin-2-yl)amino)piperidin-1-yl)methyl)phenyl)piperidine-2,6-dione;
1-(4-((4-((5-chloro-4-(5-(cyclopropylmethyl)-1-methyl-1H-pyrazol-4-yl)pyrimidin-2-yl)amino)piperidin-1-yl)methyl)phenyl)dihydropyrimidine-2,4(1H,3H)-dione;
1-(4-((4-((5-chloro-4-(5-(cyclopropylmethyl)-1-methyl-1H-pyrazol-4-yl)pyrimidin-2-yl)amino)piperidin-1-yl)methyl)-3-fluorophenyl)dihydropyrimidine-2,4(1H,3H)-dione;
1-(3-((4-((5-chloro-4-(5-(cyclopropylmethyl)-1-methyl-1H-pyrazol-4-yl)pyrimidin-2-yl)amino)piperidin-1-yl)methyl)phenyl)dihydropyrimidine-2,4(1H,3H)-dione;
1-(2-((4-((5-chloro-4-(5-(cyclopropylmethyl)-1-methyl-1H-pyrazol-4-yl)pyrimidin-2-yl)amino)piperidin-1-yl)methyl)phenyl)dihydropyrimidine-2,4(1H,3H)-dione;
1-(5-((4-((5-chloro-4-(5-(cyclopropylmethyl)-1-methyl-1H-pyrazol-4-yl)pyrimidin-2-yl)amino)piperidin-1-yl)methyl)pyridin-2-yl)dihydropyrimidine-2,4(1H,3H)-dione;
1-(6-((4-((5-chloro-4-(5-(cyclopropylmethyl)-1-methyl-1H-pyrazol-4-yl)pyrimidin-2-yl)amino)piperidin-1-yl)methyl)pyridazin-3-yl)dihydropyrimidine-2,4(1H,3H)-dione;
1-(4-((4-((5-chloro-4-(5-(cyclopropylmethyl)-1-methyl-1H-pyrazol-4-yl)pyrimidin-2-yl)amino)piperidin-1-yl)methyl)pyridin-3-yl)dihydropyrimidine-2,4(1H,3H)-dione;
3-((4-((4-((5-chloro-4-(5-(cyclopropylmethyl)-1-methyl-1H-pyrazol-4-yl)pyrimidin-2-yl)amino)piperidin-1-yl)methyl)phenyl)amino)piperidine-2,6-dione;
3-((3-((4-((5-chloro-4-(5-(cyclopropylmethyl)-1-methyl-1H-pyrazol-4-yl)pyrimidin-2-yl)amino)piperidin-1-yl)methyl)phenyl)amino)piperidine-2,6-dione;
3-((2-((4-((5-chloro-4-(5-(cyclopropylmethyl)-1-methyl-1H-pyrazol-4-yl)pyrimidin-2-yl)amino)piperidin-1-yl)methyl)phenyl)amino)piperidine-2,6-dione;
4-(4-(2,6-dioxopiperidin-3-yl)benzyl)-N-(4-(((5-(((S)-1-hydroxybutan-2-yl)amino)-3-isopropylpyrazolo[1,5-a]pyrimidin-7-yl)amino)methyl)phenyl)piperazine-1-carboxamide;
4-(4-(2,6-dioxopiperidin-3-yl)-3-fluorobenzyl)-N-(4-(((5-(((S)-1-hydroxybutan-2-yl)amino)-3-isopropylpyrazolo[1,5-a]pyrimidin-7-yl)amino)methyl)phenyl)piperazine-1-carboxamide;
4-(3-(2,6-dioxopiperidin-3-yl)benzyl)-N-(4-(((5-(((S)-1-hydroxybutan-2-yl)amino)-3-isopropylpyrazolo[1,5-a]pyrimidin-7-yl)amino)methyl)phenyl)piperazine-1-carboxamide;
4-(2-(2,6-dioxopiperidin-3-yl)benzyl)-N-(4-(((5-(((S)-1-hydroxybutan-2-yl)amino)-3-isopropylpyrazolo[1,5-a]pyrimidin-7-yl)amino)methyl)phenyl)piperazine-1-carboxamide;
(S)-4-(4-(2,4-dioxotetrahydropyrimidin-1(2H)-yl)benzyl)-N-(4-(((5-((1-hydroxybutan-2-yl)amino)-3-isopropylpyrazolo[1,5-a]pyrimidin-7-yl)amino)methyl)phenyl)piperazine-1-carboxamide;
(S)-4-(4-(2,4-dioxotetrahydropyrimidin-1(2H)-yl)-2-fluorobenzyl)-N-(4-(((5-((1-hydroxybutan-2-yl)amino)-3-isopropylpyrazolo[1,5-a]pyrimidin-7-yl)amino)methyl)phenyl)piperazine-1-carboxamide;
(S)-4-(3-(2,4-dioxotetrahydropyrimidin-1(2H)-yl)benzyl)-N-(4-(((5-((1-hydroxybutan-2-yl)amino)-3-isopropylpyrazolo[1,5-a]pyrimidin-7-yl)amino)methyl)phenyl)piperazine-1-carboxamide;
(S)-4-(2-(2,4-dioxotetrahydropyrimidin-1(2H)-yl)benzyl)-N-(4-(((5-((1-hydroxybutan-2-yl)amino)-3-isopropylpyrazolo[1,5-a]pyrimidin-7-yl)amino)methyl)phenyl)piperazine-1-carboxamide;
4-(4-((2,6-dioxopiperidin-3-yl)amino)benzyl)-N-(4-(((5-(((S)-1-hydroxybutan-2-yl)amino)-3-isopropylpyrazolo[1,5-a]pyrimidin-7-yl)amino)methyl)phenyl)piperazine-1-carboxamide;
4-(3-((2,6-dioxopiperidin-3-yl)amino)benzyl)-N-(4-(((5-(((S)-1-hydroxybutan-2-yl)amino)-3-isopropylpyrazolo[1,5-a]pyrimidin-7-yl)amino)methyl)phenyl)piperazine-1-carboxamide;
4-(2-((2,6-dioxopiperidin-3-yl)amino)benzyl)-N-(4-(((5-(((S)-1-hydroxybutan-2-yl)amino)-3-isopropylpyrazolo[1,5-a]pyrimidin-7-yl)amino)methyl)phenyl)piperazine-1-carboxamide;
4-(((4-(5-chloro-2-((1-(4-(2,6-dioxopiperidin-3-yl)benzyl)piperidin-4-yl)amino)pyridin-4-yl)thiazol-2-yl)amino)methyl)tetrahydro-2H-pyran-4-carbonitrile;
4-(((4-(5-chloro-2-((1-(4-(2,6-dioxopiperidin-3-yl)-3-fluorobenzyl)piperidin-4-yl)amino)pyridin-4-yl)thiazol-2-yl)amino)methyl)tetrahydro-2H-pyran-4-carbonitrile;
4-(((4-(5-chloro-2-((1-(3-(2,6-dioxopiperidin-3-yl)benzyl)piperidin-4-yl)amino)pyridin-4-yl)thiazol-2-yl)amino)methyl)tetrahydro-2H-pyran-4-carbonitrile;
4-(((4-(5-chloro-2-((1-(2-(2,6-dioxopiperidin-3-yl)benzyl)piperidin-4-yl)amino)pyridin-4-yl)thiazol-2-yl)amino)methyl)tetrahydro-2H-pyran-4-carbonitrile;
4-(((4-(5-chloro-2-((1-(4-(2,4-dioxotetrahydropyrimidin-1(2H)-yl)benzyl)piperidin-4-yl)amino)pyridin-4-yl)thiazol-2-yl)amino)methyl)tetrahydro-2H-pyran-4-carbonitrile;
4-(((4-(5-chloro-2-((1-(4-(2,4-dioxotetrahydropyrimidin-1(2H)-yl)-2-fluorobenzyl)piperidin-4-yl)amino)pyridin-4-yl)thiazol-2-yl)amino)methyl)tetrahydro-2H-pyran-4-carbonitrile;
4-(((4-(5-chloro-2-((1-(3-(2,4-dioxotetrahydropyrimidin-1(2H)-yl)benzyl)piperidin-4-yl)amino)pyridin-4-yl)thiazol-2-yl)amino)methyl)tetrahydro-2H-pyran-4-carbonitrile;
4-(((4-(5-chloro-2-((1-(2-(2,4-dioxotetrahydropyrimidin-1(2H)-yl)benzyl)piperidin-4-yl)amino)pyridin-4-yl)thiazol-2-yl)amino)methyl)tetrahydro-2H-pyran-4-carbonitrile;
4-(((4-(5-chloro-2-((1-((5-(2,4-dioxotetrahydropyrimidin-1(2H)-yl)pyridin-2-yl)methyl)piperidin-4-yl)amino)pyridin-4-yl)thiazol-2-yl)amino)methyl)tetrahydro-2H-pyran-4-carbonitrile;
4-(((4-(5-chloro-2-((1-((6-(2,4-dioxotetrahydropyrimidin-1(2H)-yl)pyridazin-3-yl)methyl)piperidin-4-yl)amino)pyridin-4-yl)thiazol-2-yl)amino)methyl)tetrahydro-2H-pyran-4-carbonitrile;
4-(((4-(5-chloro-2-((1-((3-(2,4-dioxotetrahydropyrimidin-1(2H)-yl)pyridin-4-yl)methyl)piperidin-4-yl)amino)pyridin-4-yl)thiazol-2-yl)amino)methyl)tetrahydro-2H-pyran-4-carbonitrile;
4-(((4-(5-chloro-2-((1-(4-((2,6-dioxopiperidin-3-yl)amino)benzyl)piperidin-4-yl)amino)pyridin-4-yl)thiazol-2-yl)amino)methyl)tetrahydro-2H-pyran-4-carbonitrile;
4-(((4-(5-chloro-2-((1-(3-((2,6-dioxopiperidin-3-yl)amino)benzyl)piperidin-4-yl)amino)pyridin-4-yl)thiazol-2-yl)amino)methyl)tetrahydro-2H-pyran-4-carbonitrile;
4-(((4-(5-chloro-2-((1-(2-((2,6-dioxopiperidin-3-yl)amino)benzyl)piperidin-4-yl)amino)pyridin-4-yl)thiazol-2-yl)amino)methyl)tetrahydro-2H-pyran-4-carbonitrile;
4-(((5'-chloro-2'-((1-(4-(2,6-dioxopiperidin-3-yl)benzyl)piperidin-4-yl)amino)-[2,4'-bipyridin]-6-yl)amino)methyl)tetrahydro-2H-pyran-4-carbonitrile;
4-(((5'-chloro-2'-((1-(4-(2,6-dioxopiperidin-3-yl)-3-fluorobenzyl)piperidin-4-yl)amino)-[2,4'-bipyridin]-6-yl)amino)methyl)tetrahydro-2H-pyran-4-carbonitrile;
4-(((5'-chloro-2'-((1-(3-(2,6-dioxopiperidin-3-yl)benzyl)piperidin-4-yl)amino)-[2,4'-bipyridin]-6-yl)amino)methyl)tetrahydro-2H-pyran-4-carbonitrile;
4-(((5'-chloro-2'-((1-(2-(2,6-dioxopiperidin-3-yl)benzyl)piperidin-4-yl)amino)-[2,4'-bipyridin]-6-yl)amino)methyl)tetrahydro-2H-pyran-4-carbonitrile;
4-(((5'-chloro-2'-((1-(4-(2,4-dioxotetrahydropyrimidin-1(2H)-yl)benzyl)piperidin-4-yl)amino)-[2,4'-bipyridin]-6-yl)amino)methyl)tetrahydro-2H-pyran-4-carbonitrile;
4-(((5'-chloro-2'-((1-(4-(2,4-dioxotetrahydropyrimidin-1(2H)-yl)-2-fluorobenzyl)piperidin-4-yl)amino)-[2,4'-bipyridin]-6-yl)amino)methyl)tetrahydro-2H-pyran-4-carbonitrile;
4-(((5'-chloro-2'-((1-(3-(2,4-dioxotetrahydropyrimidin-1(2H)-yl)benzyl)piperidin-4-yl)amino)-[2,4'-bipyridin]-6-yl)amino)methyl)tetrahydro-2H-pyran-4-carbonitrile;
4-(((5'-chloro-2'-((1-(2-(2,4-dioxotetrahydropyrimidin-1(2H)-yl)benzyl)piperidin-4-yl)amino)-[2,4'-bipyridin]-6-yl)amino)methyl)tetrahydro-2H-pyran-4-carbonitrile;
4-(((5'-chloro-2'-((1-((5-(2,4-dioxotetrahydropyrimidin-1(2H)-yl)pyridin-2-yl)methyl)piperidin-4-yl)amino)-[2,4'-bipyridin]-6-yl)amino)methyl)tetrahydro-2H-pyran-4-carbonitrile;
4-(((5'-chloro-2'-((1-((6-(2,4-dioxotetrahydropyrimidin-1(2H)-yl)pyridazin-3-yl)methyl)piperidin-4-yl)amino)-[2,4'-bipyridin]-6-yl)amino)methyl)tetrahydro-2H-pyran-4-carbonitrile;
4-(((5'-chloro-2'-((1-((3-(2,4-dioxotetrahydropyrimidin-1(2H)-yl)pyridin-4-yl)methyl)piperidin-4-yl)amino)-[2,4'-bipyridin]-6-yl)amino)methyl)tetrahydro-2H-pyran-4-carbonitrile;
4-(((5'-chloro-2'-((1-(4-((2,6-dioxopiperidin-3-yl)amino)benzyl)piperidin-4-yl)amino)-[2,4'-bipyridin]-6-yl)amino)methyl)tetrahydro-2H-pyran-4-carbonitrile;
4-(((5'-chloro-2'-((1-(3-((2,6-dioxopiperidin-3-yl)amino)benzyl)piperidin-4-yl)amino)-[2,4'-bipyridin]-6-yl)amino)methyl)tetrahydro-2H-pyran-4-carbonitrile;
4-(((5'-chloro-2'-((1-(2-((2,6-dioxopiperidin-3-yl)amino)benzyl)piperidin-4-yl)amino)-[2,4'-bipyridin]-6-yl)amino)methyl)tetrahydro-2H-pyran-4-carbonitrile;
3-((4-(2-((4-(2,6-dioxopiperidin-3-yl)benzyl)(methyl)amino)-4-methylthiazol-5-yl)-5-fluoropyrimidin-2-yl)amino)benzenesulfonamide;
3-((4-(2-((4-(2,6-dioxopiperidin-3-yl)-3-fluorobenzyl)(methyl)amino)-4-methylthiazol-5-yl)-5-fluoropyrimidin-2-yl)amino)benzenesulfonamide;
3-((4-(2-((3-(2,6-dioxopiperidin-3-yl)benzyl)(methyl)amino)-4-methylthiazol-5-yl)-5-fluoropyrimidin-2-yl)amino)benzenesulfonamide;
3-((4-(2-((2-(2,6-dioxopiperidin-3-yl)benzyl)(methyl)amino)-4-methylthiazol-5-yl)-5-fluoropyrimidin-2-yl)amino)benzenesulfonamide;
3-((4-(2-((4-(2,4-dioxotetrahydropyrimidin-1(2H)-yl)benzyl)(methyl)amino)-4-methylthiazol-5-yl)-5-fluoropyrimidin-2-yl)amino)benzenesulfonamide;
3-((4-(2-((4-(2,4-dioxotetrahydropyrimidin-1(2H)-yl)-2-fluorobenzyl)(methyl)amino)-4-methylthiazol-5-yl)-5-fluoropyrimidin-2-yl)amino)benzenesulfonamide;
3-((4-(2-((3-(2,4-dioxotetrahydropyrimidin-1(2H)-yl)benzyl)(methyl)amino)-4-methylthiazol-5-yl)-5-fluoropyrimidin-2-yl)amino)benzenesulfonamide;
3-((4-(2-((2-(2,4-dioxotetrahydropyrimidin-1(2H)-yl)benzyl)(methyl)amino)-4-methylthiazol-5-yl)-5-fluoropyrimidin-2-yl)amino)benzenesulfonamide;
3-((4-(2-(((5-(2,4-dioxotetrahydropyrimidin-1(2H)-yl)pyridin-2-yl)methyl)(methyl)amino)-4-methylthiazol-5-yl)-5-fluoropyrimidin-2-yl)amino)benzenesulfonamide;
3-((4-(2-(((6-(2,4-dioxotetrahydropyrimidin-1(2H)-yl)pyridazin-3-yl)methyl)(methyl)amino)-4-methylthiazol-5-yl)-5-fluoropyrimidin-2-yl)amino)benzenesulfonamide;
3-((4-(2-(((3-(2,4-dioxotetrahydropyrimidin-1(2H)-yl)pyridin-4-yl)methyl)(methyl)amino)-4-methylthiazol-5-yl)-5-fluoropyrimidin-2-yl)amino)benzenesulfonamide;
3-((4-(2-((4-((2,6-dioxopiperidin-3-yl)amino)benzyl)(methyl)amino)-4-methylthiazol-5-yl)-5-fluoropyrimidin-2-yl)amino)benzenesulfonamide;
3-((4-(2-((3-((2,6-dioxopiperidin-3-yl)amino)benzyl)(methyl)amino)-4-methylthiazol-5-yl)-5-fluoropyrimidin-2-yl)amino)benzenesulfonamide;
3-((4-(2-((2-((2,6-dioxopiperidin-3-yl)amino)benzyl)(methyl)amino)-4-methylthiazol-5-yl)-5-fluoropyrimidin-2-yl)amino)benzenesulfonamide;
N-(2-chloro-6-methylphenyl)-2-((6-((4-(2,6-dioxopiperidin-3-yl)benzyl)amino)-2-methylpyrimidin-4-yl)amino)thiazole-5-carboxamide;
N-(2-chloro-6-methylphenyl)-2-((6-((4-(2,6-dioxopiperidin-3-yl)-3-fluorobenzyl)amino)-2-methylpyrimidin-4-yl)amino)thiazole-5-carboxamide;
N-(2-chloro-6-methylphenyl)-2-((6-((3-(2,6-dioxopiperidin-3-yl)benzyl)amino)-2-methylpyrimidin-4-yl)amino)thiazole-5-carboxamide;
N-(2-chloro-6-methylphenyl)-2-((6-((2-(2,6-dioxopiperidin-3-yl)benzyl)amino)-2-methylpyrimidin-4-yl)amino)thiazole-5-carboxamide;
N-(2-chloro-6-methylphenyl)-2-((6-((4-(2,4-dioxotetrahydropyrimidin-1(2H)-yl)benzyl)amino)-2-methylpyrimidin-4-yl)amino)thiazole-5-carboxamide;
N-(2-chloro-6-methylphenyl)-2-((6-((4-(2,4-dioxotetrahydropyrimidin-1(2H)-yl)-2-fluorobenzyl)amino)-2-methylpyrimidin-4-yl)amino)thiazole-5-carboxamide;
N-(2-chloro-6-methylphenyl)-2-((6-((3-(2,4-dioxotetrahydropyrimidin-1(2H)-yl)benzyl)amino)-2-methylpyrimidin-4-yl)amino)thiazole-5-carboxamide;
N-(2-chloro-6-methylphenyl)-2-((6-((2-(2,4-dioxotetrahydropyrimidin-1(2H)-yl)benzyl)amino)-2-methylpyrimidin-4-yl)amino)thiazole-5-carboxamide;
N-(2-chloro-6-methylphenyl)-2-((6-(((5-(2,4-dioxotetrahydropyrimidin-1(2H)-yl)pyridin-2-yl)methyl)amino)-2-methylpyrimidin-4-yl)amino)thiazole-5-carboxamide;
N-(2-chloro-6-methylphenyl)-2-((6-(((6-(2,4-dioxotetrahydropyrimidin-1(2H)-yl)pyridazin-3-yl)methyl)amino)-2-methylpyrimidin-4-yl)amino)thiazole-5-carboxamide;
N-(2-chloro-6-methylphenyl)-2-((6-(((3-(2,4-dioxotetrahydropyrimidin-1(2H)-yl)pyridin-4-yl)methyl)amino)-2-methylpyrimidin-4-yl)amino)thiazole-5-carboxamide;
N-(2-chloro-6-methylphenyl)-2-((6-((4-((2,6-dioxopiperidin-3-yl)amino)benzyl)amino)-2-methylpyrimidin-4-yl)amino)thiazole-5-carboxamide;
N-(2-chloro-6-methylphenyl)-2-((6-((3-((2,6-dioxopiperidin-3-yl)amino)benzyl)amino)-2-methylpyrimidin-4-yl)amino)thiazole-5-carboxamide;
N-(2-chloro-6-methylphenyl)-2-((6-((2-((2,6-dioxopiperidin-3-yl)amino)benzyl)amino)-2-methylpyrimidin-4-yl)amino)thiazole-5-carboxamide;
N-(2-chloro-6-methylphenyl)-2-((6-(4-(4-(2,6-dioxopiperidin-3-yl)benzyl)piperazin-1-yl)-2-methylpyrimidin-4-yl)amino)thiazole-5-carboxamide;
N-(2-chloro-6-methylphenyl)-2-((6-(4-(4-(2,6-dioxopiperidin-3-yl)-3-fluorobenzyl)piperazin-1-yl)-2-methylpyrimidin-4-yl)amino)thiazole-5-carboxamide;
N-(2-chloro-6-methylphenyl)-2-((6-(4-(3-(2,6-dioxopiperidin-3-yl)benzyl)piperazin-1-yl)-2-methylpyrimidin-4-yl)amino)thiazole-5-carboxamide;
N-(2-chloro-6-methylphenyl)-2-((6-(4-(2-(2,6-dioxopiperidin-3-yl)benzyl)piperazin-1-yl)-2-methylpyrimidin-4-yl)amino)thiazole-5-carboxamide;
N-(2-chloro-6-methylphenyl)-2-((6-(4-(4-(2,4-dioxotetrahydropyrimidin-1(2H)-yl)benzyl)piperazin-1-yl)-2-methylpyrimidin-4-yl)amino)thiazole-5-carboxamide;
N-(2-chloro-6-methylphenyl)-2-((6-(4-(4-(2,4-dioxotetrahydropyrimidin-1(2H)-yl)-2-fluorobenzyl)piperazin-1-yl)-2-methylpyrimidin-4-yl)amino)thiazole-5-carboxamide;
N-(2-chloro-6-methylphenyl)-2-((6-(4-(3-(2,4-dioxotetrahydropyrimidin-1(2H)-yl)benzyl)piperazin-1-yl)-2-methylpyrimidin-4-yl)amino)thiazole-5-carboxamide;
N-(2-chloro-6-methylphenyl)-2-((6-(4-(2-(2,4-dioxotetrahydropyrimidin-1(2H)-yl)benzyl)piperazin-1-yl)-2-methylpyrimidin-4-yl)amino)thiazole-5-carboxamide;
N-(2-chloro-6-methylphenyl)-2-((6-(4-((5-(2,4-dioxotetrahydropyrimidin-1(2H)-yl)pyridin-2-yl)methyl)piperazin-1-yl)-2-methylpyrimidin-4-yl)amino)thiazole-5-carboxamide;
N-(2-chloro-6-methylphenyl)-2-((6-(4-((6-(2,4-dioxotetrahydropyrimidin-1(2H)-yl)pyridazin-3-yl)methyl)piperazin-1-yl)-2-methylpyrimidin-4-yl)amino)thiazole-5-carboxamide;
N-(2-chloro-6-methylphenyl)-2-((6-(4-((3-(2,4-dioxotetrahydropyrimidin-1(2H)-yl)pyridin-4-yl)methyl)piperazin-1-yl)-2-methylpyrimidin-4-yl)amino)thiazole-5-carboxamide;
N-(2-chloro-6-methylphenyl)-2-((6-(4-(4-((2,6-dioxopiperidin-3-yl)amino)benzyl)piperazin-1-yl)-2-methylpyrimidin-4-yl)amino)thiazole-5-carboxamide;
N-(2-chloro-6-methylphenyl)-2-((6-(4-(3-((2,6-dioxopiperidin-3-yl)amino)benzyl)piperazin-1-yl)-2-methylpyrimidin-4-yl)amino)thiazole-5-carboxamide;
N-(2-chloro-6-methylphenyl)-2-((6-(4-(2-((2,6-dioxopiperidin-3-yl)amino)benzyl)piperazin-1-yl)-2-methylpyrimidin-4-yl)amino)thiazole-5-carboxamide;
N-(2-chloro-6-methylphenyl)-2-((6-(4-((4-(2,6-dioxopiperidin-3-yl)benzyl)(methyl)amino)piperidin-1-yl)-2-methylpyrimidin-4-yl)amino)thiazole-5-carboxamide;
N-(2-chloro-6-methylphenyl)-2-((6-(4-((4-(2,6-dioxopiperidin-3-yl)-3-fluorobenzyl)(methyl)amino)piperidin-1-yl)-2-methylpyrimidin-4-yl)amino)thiazole-5-carboxamide;
N-(2-chloro-6-methylphenyl)-2-((6-(4-((3-(2,6-dioxopiperidin-3-yl)benzyl)(methyl)amino)piperidin-1-yl)-2-methylpyrimidin-4-yl)amino)thiazole-5-carboxamide;
N-(2-chloro-6-methylphenyl)-2-((6-(4-((2-(2,6-dioxopiperidin-3-yl)benzyl)(methyl)amino)piperidin-1-yl)-2-methylpyrimidin-4-yl)amino)thiazole-5-carboxamide;
N-(2-chloro-6-methylphenyl)-2-((6-(4-((4-(2,4-dioxotetrahydropyrimidin-1(2H)-yl)benzyl)(methyl)amino)piperidin-1-yl)-2-methylpyrimidin-4-yl)amino)thiazole-5-carboxamide;
N-(2-chloro-6-methylphenyl)-2-((6-(4-((4-(2,4-dioxotetrahydropyrimidin-1(2H)-yl)-2-fluorobenzyl)(methyl)amino)piperidin-1-yl)-2-methylpyrimidin-4-yl)amino)thiazole-5-carboxamide;
N-(2-chloro-6-methylphenyl)-2-((6-(4-((3-(2,4-dioxotetrahydropyrimidin-1(2H)-yl)benzyl)(methyl)amino)piperidin-1-yl)-2-methylpyrimidin-4-yl)amino)thiazole-5-carboxamide;
N-(2-chloro-6-methylphenyl)-2-((6-(4-((2-(2,4-dioxotetrahydropyrimidin-1(2H)-yl)benzyl)(methyl)amino)piperidin-1-yl)-2-methylpyrimidin-4-yl)amino)thiazole-5-carboxamide;
N-(2-chloro-6-methylphenyl)-2-((6-(4-(((5-(2,4-dioxotetrahydropyrimidin-1(2H)-yl)pyridin-2-yl)methyl)(methyl)amino)piperidin-1-yl)-2-methylpyrimidin-4-yl)amino)thiazole-5-carboxamide;
N-(2-chloro-6-methylphenyl)-2-((6-(4-(((6-(2,4-dioxotetrahydropyrimidin-1(2H)-yl)pyridazin-3-yl)methyl)(methyl)amino)piperidin-1-yl)-2-methylpyrimidin-4-yl)amino)thiazole-5-carboxamide;
N-(2-chloro-6-methylphenyl)-2-((6-(4-(((3-(2,4-dioxotetrahydropyrimidin-1(2H)-yl)pyridin-4-yl)methyl)(methyl)amino)piperidin-1-yl)-2-methylpyrimidin-4-yl)amino)thiazole-5-carboxamide;
N-(2-chloro-6-methylphenyl)-2-((6-(4-((4-((2,6-dioxopiperidin-3-yl)amino)benzyl)(methyl)amino)piperidin-1-yl)-2-methylpyrimidin-4-yl)amino)thiazole-5-carboxamide;
N-(2-chloro-6-methylphenyl)-2-((6-(4-((3-((2,6-dioxopiperidin-3-yl)amino)benzyl)(methyl)amino)piperidin-1-yl)-2-methylpyrimidin-4-yl)amino)thiazole-5-carboxamide;
N-(2-chloro-6-methylphenyl)-2-((6-(4-((2-((2,6-dioxopiperidin-3-yl)amino)benzyl)(methyl)amino)piperidin-1-yl)-2-methylpyrimidin-4-yl)amino)thiazole-5-carboxamide;
N-(2-chloro-6-methylphenyl)-2-((6-(4-(4-(4-(2,6-dioxopiperidin-3-yl)benzyl)piperazin-1-yl)piperidin-1-yl)-2-methylpyrimidin-4-yl)amino)thiazole-5-carboxamide;
N-(2-chloro-6-methylphenyl)-2-((6-(4-(4-(4-(2,6-dioxopiperidin-3-yl)-3-fluorobenzyl)piperazin-1-yl)piperidin-1-yl)-2-methylpyrimidin-4-yl)amino)thiazole-5-carboxamide;
N-(2-chloro-6-methylphenyl)-2-((6-(4-(4-(3-(2,6-dioxopiperidin-3-yl)benzyl)piperazin-1-yl)piperidin-1-yl)-2-methylpyrimidin-4-yl)amino)thiazole-5-carboxamide;
N-(2-chloro-6-methylphenyl)-2-((6-(4-(4-(2-(2,6-dioxopiperidin-3-yl)benzyl)piperazin-1-yl)piperidin-1-yl)-2-methylpyrimidin-4-yl)amino)thiazole-5-carboxamide;
N-(2-chloro-6-methylphenyl)-2-((6-(4-(4-(4-(2,6-dioxopiperidin-3-yl)benzyl)piperazin-1-yl)piperidin-1-yl)-2-methylpyrimidin-4-yl)amino)thiazole-5-carboxamide;
N-(2-chloro-6-methylphenyl)-2-((6-(4-(4-(4-(2,4-dioxotetrahydropyrimidin-1(2H)-yl)-2-fluorobenzyl)piperazin-1-yl)piperidin-1-yl)-2-methylpyrimidin-4-yl)amino)thiazole-5-carboxamide;
N-(2-chloro-6-methylphenyl)-2-((6-(4-(4-(3-(2,4-dioxotetrahydropyrimidin-1(2H)-yl)benzyl)piperazin-1-yl)piperidin-1-yl)-2-methylpyrimidin-4-yl)amino)thiazole-5-carboxamide;
N-(2-chloro-6-methylphenyl)-2-((6-(4-(4-(2-(2,4-dioxotetrahydropyrimidin-1(2H)-yl)benzyl)piperazin-1-yl)piperidin-1-yl)-2-methylpyrimidin-4-yl)amino)thiazole-5-carboxamide;
N-(2-chloro-6-methylphenyl)-2-((6-(4-(4-((5-(2,4-dioxotetrahydropyrimidin-1(2H)-yl)pyridin-2-yl)methyl)piperazin-1-yl)piperidin-1-yl)-2-methylpyrimidin-4-yl)amino)thiazole-5-carboxamide;
N-(2-chloro-6-methylphenyl)-2-((6-(4-(4-((6-(2,4-dioxotetrahydropyrimidin-1(2H)-yl)pyridazin-3-yl)methyl)piperazin-1-yl)piperidin-1-yl)-2-methylpyrimidin-4-yl)amino)thiazole-5-carboxamide;
N-(2-chloro-6-methylphenyl)-2-((6-(4-(4-((3-(2,4-dioxotetrahydropyrimidin-1(2H)-yl)pyridin-4-yl)methyl)piperazin-1-yl)piperidin-1-yl)-2-methylpyrimidin-4-yl)amino)thiazole-5-carboxamide;
N-(2-chloro-6-methylphenyl)-2-((6-(4-(4-(4-((2,6-dioxopiperidin-3-yl)amino)benzyl)piperazin-1-yl)piperidin-1-yl)-2-methylpyrimidin-4-yl)amino)thiazole-5-carboxamide;
N-(2-chloro-6-methylphenyl)-2-((6-(4-(4-(3-((2,6-dioxopiperidin-3-yl)amino)benzyl)piperazin-1-yl)piperidin-1-yl)-2-methylpyrimidin-4-yl)amino)thiazole-5-carboxamide;
N-(2-chloro-6-methylphenyl)-2-((6-(4-(4-(2-((2,6-dioxopiperidin-3-yl)amino)benzyl)piperazin-1-yl)piperidin-1-yl)-2-methylpyrimidin-4-yl)amino)thiazole-5-carboxamide;
4-((4-(4-(2,6-dioxopiperidin-3-yl)benzyl)piperazin-1-yl)methyl)-N-(4-methyl-3-((4-(pyridin-3-yl)pyrimidin-2-yl)amino)phenyl)benzamide;
4-((4-(4-(2,6-dioxopiperidin-3-yl)-3-fluorobenzyl)piperazin-1-yl)methyl)-N-(4-methyl-3-((4-(pyridin-3-yl)pyrimidin-2-yl)amino)phenyl)benzamide;
4-((4-(3-(2,6-dioxopiperidin-3-yl)benzyl)piperazin-1-yl)methyl)-N-(4-methyl-3-((4-(pyridin-3-yl)pyrimidin-2-yl)amino)phenyl)benzamide;
4-((4-(2-(2,6-dioxopiperidin-3-yl)benzyl)piperazin-1-yl)methyl)-N-(4-methyl-3-((4-(pyridin-3-yl)pyrimidin-2-yl)amino)phenyl)benzamide;
4-((4-(4-(2,4-dioxotetrahydropyrimidin-1(2H)-yl)benzyl)piperazin-1-yl)methyl)-N-(4-methyl-3-((4-(pyridin-3-yl)pyrimidin-2-yl)amino)phenyl)benzamide;
4-((4-(4-(2,4-dioxotetrahydropyrimidin-1(2H)-yl)-2-fluorobenzyl)piperazin-1-yl)methyl)-N-(4-methyl-3-((4-(pyridin-3-yl)pyrimidin-2-yl)amino)phenyl)benzamide;
4-((4-(3-(2,4-dioxotetrahydropyrimidin-1(2H)-yl)benzyl)piperazin-1-yl)methyl)-N-(4-methyl-3-((4-(pyridin-3-yl)pyrimidin-2-yl)amino)phenyl)benzamide;
4-((4-(2-(2,4-dioxotetrahydropyrimidin-1(2H)-yl)benzyl)piperazin-1-yl)methyl)-N-(4-methyl-3-((4-(pyridin-3-yl)pyrimidin-2-yl)amino)phenyl)benzamide;
4-((4-((5-(2,4-dioxotetrahydropyrimidin-1(2H)-yl)pyridin-2-yl)methyl)piperazin-1-yl)methyl)-N-(4-methyl-3-((4-(pyridin-3-yl)pyrimidin-2-yl)amino)phenyl)benzamide;
4-((4-((6-(2,4-dioxotetrahydropyrimidin-1(2H)-yl)pyridazin-3-yl)methyl)piperazin-1-yl)methyl)-N-(4-methyl-3-((4-(pyridin-3-yl)pyrimidin-2-yl)amino)phenyl)benzamide;
4-((4-((3-(2,4-dioxotetrahydropyrimidin-1(2H)-yl)pyridin-4-yl)methyl)piperazin-1-yl)methyl)-N-(4-methyl-3-((4-(pyridin-3-yl)pyrimidin-2-yl)amino)phenyl)benzamide;
4-((4-(4-((2,6-dioxopiperidin-3-yl)amino)benzyl)piperazin-1-yl)methyl)-N-(4-methyl-3-((4-(pyridin-3-yl)pyrimidin-2-yl)amino)phenyl)benzamide;
4-((4-(3-((2,6-dioxopiperidin-3-yl)amino)benzyl)piperazin-1-yl)methyl)-N-(4-methyl-3-((4-(pyridin-3-yl)pyrimidin-2-yl)amino)phenyl)benzamide;
4-((4-(2-((2,6-dioxopiperidin-3-yl)amino)benzyl)piperazin-1-yl)methyl)-N-(4-methyl-3-((4-(pyridin-3-yl)pyrimidin-2-yl)amino)phenyl)benzamide;
4-(((4-(2,6-dioxopiperidin-3-yl)benzyl)amino)methyl)-N-(4-methyl-3-((4-(pyridin-3-yl)pyrimidin-2-yl)amino)phenyl)benzamide;
4-(((4-(2,6-dioxopiperidin-3-yl)-3-fluorobenzyl)amino)methyl)-N-(4-methyl-3-((4-(pyridin-3-yl)pyrimidin-2-yl)amino)phenyl)benzamide;
4-(((3-(2,6-dioxopiperidin-3-yl)benzyl)amino)methyl)-N-(4-methyl-3-((4-(pyridin-3-yl)pyrimidin-2-yl)amino)phenyl)benzamide;
4-(((2-(2,6-dioxopiperidin-3-yl)benzyl)amino)methyl)-N-(4-methyl-3-((4-(pyridin-3-yl)pyrimidin-2-yl)amino)phenyl)benzamide;
4-(((4-(2,4-dioxotetrahydropyrimidin-1(2H)-yl)benzyl)amino)methyl)-N-(4-methyl-3-((4-(pyridin-3-yl)pyrimidin-2-yl)amino)phenyl)benzamide;
4-(((4-(2,4-dioxotetrahydropyrimidin-1(2H)-yl)-2-fluorobenzyl)amino)methyl)-N-(4-methyl-3-((4-(pyridin-3-yl)pyrimidin-2-yl)amino)phenyl)benzamide;
4-(((3-(2,4-dioxotetrahydropyrimidin-1(2H)-yl)benzyl)amino)methyl)-N-(4-methyl-3-((4-(pyridin-3-yl)pyrimidin-2-yl)amino)phenyl)benzamide;
4-(((2-(2,4-dioxotetrahydropyrimidin-1(2H)-yl)benzyl)amino)methyl)-N-(4-methyl-3-((4-(pyridin-3-yl)pyrimidin-2-yl)amino)phenyl)benzamide;
4-((((5-(2,4-dioxotetrahydropyrimidin-1(2H)-yl)pyridin-2-yl)methyl)amino)methyl)-N-(4-methyl-3-((4-(pyridin-3-yl)pyrimidin-2-yl)amino)phenyl)benzamide;
4-((((6-(2,4-dioxotetrahydropyrimidin-1(2H)-yl)pyridazin-3-yl)methyl)amino)methyl)-N-(4-methyl-3-((4-(pyridin-3-yl)pyrimidin-2-yl)amino)phenyl)benzamide;
4-((((3-(2,4-dioxotetrahydropyrimidin-1(2H)-yl)pyridin-4-yl)methyl)amino)methyl)-N-(4-methyl-3-((4-(pyridin-3-yl)pyrimidin-2-yl)amino)phenyl)benzamide;
4-(((4-((2,6-dioxopiperidin-3-yl)amino)benzyl)amino)methyl)-N-(4-methyl-3-((4-(pyridin-3-yl)pyrimidin-2-yl)amino)phenyl)benzamide;
4-(((3-((2,6-dioxopiperidin-3-yl)amino)benzyl)amino)methyl)-N-(4-methyl-3-((4-(pyridin-3-yl)pyrimidin-2-yl)amino)phenyl)benzamide;
4-(((2-((2,6-dioxopiperidin-3-yl)amino)benzyl)amino)methyl)-N-(4-methyl-3-((4-(pyridin-3-yl)pyrimidin-2-yl)amino)phenyl)benzamide;
4-(((2-((2,6-dioxopiperidin-3-yl)amino)benzyl)amino)methyl)-N-(4-methyl-3-((4-(pyridin-3-yl)pyrimidin-2-yl)amino)phenyl)benzamide;
4-(((4-(2,6-dioxopiperidin-3-yl)benzyl)(methyl)amino)methyl)-N-(4-methyl-3-((4-(pyridin-3-yl)pyrimidin-2-yl)amino)phenyl)benzamide;
4-(((4-(2,6-dioxopiperidin-3-yl)-3-fluorobenzyl)(methyl)amino)methyl)-N-(4-methyl-3-((4-(pyridin-3-yl)pyrimidin-2-yl)amino)phenyl)benzamide;
4-(((3-(2,6-dioxopiperidin-3-yl)benzyl)(methyl)amino)methyl)-N-(4-methyl-3-((4-(pyridin-3-yl)pyrimidin-2-yl)amino)phenyl)benzamide;
4-(((2-(2,6-dioxopiperidin-3-yl)benzyl)(methyl)amino)methyl)-N-(4-methyl-3-((4-(pyridin-3-yl)pyrimidin-2-yl)amino)phenyl)benzamide;
4-(((4-(2,4-dioxotetrahydropyrimidin-1(2H)-yl)benzyl)(methyl)amino)methyl)-N-(4-methyl-3-((4-(pyridin-3-yl)pyrimidin-2-yl)amino)phenyl)benzamide;
4-(((4-(2,4-dioxotetrahydropyrimidin-1(2H)-yl)-2-fluorobenzyl)(methyl)amino)methyl)-N-(4-methyl-3-((4-(pyridin-3-yl)pyrimidin-2-yl)amino)phenyl)benzamide;
4-(((3-(2,4-dioxotetrahydropyrimidin-1(2H)-yl)benzyl)(methyl)amino)methyl)-N-(4-methyl-3-((4-(pyridin-3-yl)pyrimidin-2-yl)amino)phenyl)benzamide;
4-(((2-(2,4-dioxotetrahydropyrimidin-1(2H)-yl)benzyl)(methyl)amino)methyl)-N-(4-methyl-3-((4-(pyridin-3-yl)pyrimidin-2-yl)amino)phenyl)benzamide;
4-((((5-(2,4-dioxotetrahydropyrimidin-1(2H)-yl)pyridin-2-yl)methyl)(methyl)amino)methyl)-N-(4-methyl-3-((4-(pyridin-3-yl)pyrimidin-2-yl)amino)phenyl)benzamide;
4-((((6-(2,4-dioxotetrahydropyrimidin-1(2H)-yl)pyridazin-3-yl)methyl)(methyl)amino)methyl)-N-(4-methyl-3-((4-(pyridin-3-yl)pyrimidin-2-yl)amino)phenyl)benzamide;
4-((((3-(2,4-dioxotetrahydropyrimidin-1(2H)-yl)pyridin-4-yl)methyl)(methyl)amino)methyl)-N-(4-methyl-3-((4-(pyridin-3-yl)pyrimidin-2-yl)amino)phenyl)benzamide;
4-(((4-((2,6-dioxopiperidin-3-yl)amino)benzyl)(methyl)amino)methyl)-N-(4-methyl-3-((4-(pyridin-3-yl)pyrimidin-2-yl)amino)phenyl)benzamide;
4-(((3-((2,6-dioxopiperidin-3-yl)amino)benzyl)(methyl)amino)methyl)-N-(4-methyl-3-((4-(pyridin-3-yl)pyrimidin-2-yl)amino)phenyl)benzamide;
4-(((2-((2,6-dioxopiperidin-3-yl)amino)benzyl)(methyl)amino)methyl)-N-(4-methyl-3-((4-(pyridin-3-yl)pyrimidin-2-yl)amino)phenyl)benzamide;
4-(4-(4-(2,6-dioxopiperidin-3-yl)benzyl)piperazin-1-yl)-N-(4-methyl-3-((4-(pyridin-3-yl)pyrimidin-2-yl)amino)phenyl)benzamide;
4-(4-(4-(2,6-dioxopiperidin-3-yl)-3-fluorobenzyl)piperazin-1-yl)-N-(4-methyl-3-((4-(pyridin-3-yl)pyrimidin-2-yl)amino)phenyl)benzamide;
4-(4-(3-(2,6-dioxopiperidin-3-yl)benzyl)piperazin-1-yl)-N-(4-methyl-3-((4-(pyridin-3-yl)pyrimidin-2-yl)amino)phenyl)benzamide;
4-(4-(2-(2,6-dioxopiperidin-3-yl)benzyl)piperazin-1-yl)-N-(4-methyl-3-((4-(pyridin-3-yl)pyrimidin-2-yl)amino)phenyl)benzamide;
4-(4-(4-(2,4-dioxotetrahydropyrimidin-1(2H)-yl)benzyl)piperazin-1-yl)-N-(4-methyl-3-((4-(pyridin-3-yl)pyrimidin-2-yl)amino)phenyl)benzamide;
4-(4-(4-(2,4-dioxotetrahydropyrimidin-1(2H)-yl)-2-fluorobenzyl)piperazin-1-yl)-N-(4-methyl-3-((4-(pyridin-3-yl)pyrimidin-2-yl)amino)phenyl)benzamide;
4-(4-(3-(2,4-dioxotetrahydropyrimidin-1(2H)-yl)benzyl)piperazin-1-yl)-N-(4-methyl-3-((4-(pyridin-3-yl)pyrimidin-2-yl)amino)phenyl)benzamide;
4-(4-(2-(2,4-dioxotetrahydropyrimidin-1(2H)-yl)benzyl)piperazin-1-yl)-N-(4-methyl-3-((4-(pyridin-3-yl)pyrimidin-2-yl)amino)phenyl)benzamide;
4-(4-((5-(2,4-dioxotetrahydropyrimidin-1(2H)-yl)pyridin-2-yl)methyl)piperazin-1-yl)-N-(4-methyl-3-((4-(pyridin-3-yl)pyrimidin-2-yl)amino)phenyl)benzamide;
4-(4-((6-(2,4-dioxotetrahydropyrimidin-1(2H)-yl)pyridazin-3-yl)methyl)piperazin-1-yl)-N-(4-methyl-3-((4-(pyridin-3-yl)pyrimidin-2-yl)amino)phenyl)benzamide;
4-(4-((3-(2,4-dioxotetrahydropyrimidin-1(2H)-yl)pyridin-4-yl)methyl)piperazin-1-yl)-N-(4-methyl-3-((4-(pyridin-3-yl)pyrimidin-2-yl)amino)phenyl)benzamide;
4-(4-(4-((2,6-dioxopiperidin-3-yl)amino)benzyl)piperazin-1-yl)-N-(4-methyl-3-((4-(pyridin-3-yl)pyrimidin-2-yl)amino)phenyl)benzamide;
4-(4-(3-((2,6-dioxopiperidin-3-yl)amino)benzyl)piperazin-1-yl)-N-(4-methyl-3-((4-(pyridin-3-yl)pyrimidin-2-yl)amino)phenyl)benzamide;
4-(4-(2-((2,6-dioxopiperidin-3-yl)amino)benzyl)piperazin-1-yl)-N-(4-methyl-3-((4-(pyridin-3-yl)pyrimidin-2-yl)amino)phenyl)benzamide;
4-(4-((4-(2,6-dioxopiperidin-3-yl)benzyl)(methyl)amino)piperidin-1-yl)-N-(4-methyl-3-((4-(pyridin-3-yl)pyrimidin-2-yl)amino)phenyl)benzamide;
4-(4-((4-(2,6-dioxopiperidin-3-yl)-3-fluorobenzyl)(methyl)amino)piperidin-1-yl)-N-(4-methyl-3-((4-(pyridin-3-yl)pyrimidin-2-yl)amino)phenyl)benzamide;
4-(4-((3-(2,6-dioxopiperidin-3-yl)benzyl)(methyl)amino)piperidin-1-yl)-N-(4-methyl-3-((4-(pyridin-3-yl)pyrimidin-2-yl)amino)phenyl)benzamide;
4-(4-((2-(2,6-dioxopiperidin-3-yl)benzyl)(methyl)amino)piperidin-1-yl)-N-(4-methyl-3-((4-(pyridin-3-yl)pyrimidin-2-yl)amino)phenyl)benzamide;
4-(4-((4-(2,4-dioxotetrahydropyrimidin-1(2H)-yl)benzyl)(methyl)amino)piperidin-1-yl)-N-(4-methyl-3-((4-(pyridin-3-yl)pyrimidin-2-yl)amino)phenyl)benzamide;
4-(4-((4-(2,4-dioxotetrahydropyrimidin-1(2H)-yl)-2-fluorobenzyl)(methyl)amino)piperidin-1-yl)-N-(4-methyl-3-((4-(pyridin-3-yl)pyrimidin-2-yl)amino)phenyl)benzamide;
4-(4-((3-(2,4-dioxotetrahydropyrimidin-1(2H)-yl)benzyl)(methyl)amino)piperidin-1-yl)-N-(4-methyl-3-((4-(pyridin-3-yl)pyrimidin-2-yl)amino)phenyl)benzamide;
4-(4-((2-(2,4-dioxotetrahydropyrimidin-1(2H)-yl)benzyl)(methyl)amino)piperidin-1-yl)-N-(4-methyl-3-((4-(pyridin-3-yl)pyrimidin-2-yl)amino)phenyl)benzamide;
4-(4-(((5-(2,4-dioxotetrahydropyrimidin-1(2H)-yl)pyridin-2-yl)methyl)(methyl)amino)piperidin-1-yl)-N-(4-methyl-3-((4-(pyridin-3-yl)pyrimidin-2-yl)amino)phenyl)benzamide;
4-(4-(((6-(2,4-dioxotetrahydropyrimidin-1(2H)-yl)pyridazin-3-yl)methyl)(methyl)amino)piperidin-1-yl)-N-(4-methyl-3-((4-(pyridin-3-yl)pyrimidin-2-yl)amino)phenyl)benzamide;
4-(4-(((3-(2,4-dioxotetrahydropyrimidin-1(2H)-yl)pyridin-4-yl)methyl)(methyl)amino)piperidin-1-yl)-N-(4-methyl-3-((4-(pyridin-3-yl)pyrimidin-2-yl)amino)phenyl)benzamide;
4-(4-((4-((2,6-dioxopiperidin-3-yl)amino)benzyl)(methyl)amino)piperidin-1-yl)-N-(4-methyl-3-((4-(pyridin-3-yl)pyrimidin-2-yl)amino)phenyl)benzamide;
4-(4-((3-((2,6-dioxopiperidin-3-yl)amino)benzyl)(methyl)amino)piperidin-1-yl)-N-(4-methyl-3-((4-(pyridin-3-yl)pyrimidin-2-yl)amino)phenyl)benzamide;
4-(4-((2-((2,6-dioxopiperidin-3-yl)amino)benzyl)(methyl)amino)piperidin-1-yl)-N-(4-methyl-3-((4-(pyridin-3-yl)pyrimidin-2-yl)amino)phenyl)benzamide;
4-(4-(4-(4-(2,6-dioxopiperidin-3-yl)benzyl)piperazin-1-yl)piperidin-1-yl)-N-(4-methyl-3-((4-(pyridin-3-yl)pyrimidin-2-yl)amino)phenyl)benzamide;
4-(4-(4-(4-(2,6-dioxopiperidin-3-yl)-3-fluorobenzyl)piperazin-1-yl)piperidin-1-yl)-N-(4-methyl-3-((4-(pyridin-3-yl)pyrimidin-2-yl)amino)phenyl)benzamide;
4-(4-(4-(3-(2,6-dioxopiperidin-3-yl)benzyl)piperazin-1-yl)piperidin-1-yl)-N-(4-methyl-3-((4-(pyridin-3-yl)pyrimidin-2-yl)amino)phenyl)benzamide;
4-(4-(4-(2-(2,6-dioxopiperidin-3-yl)benzyl)piperazin-1-yl)piperidin-1-yl)-N-(4-methyl-3-((4-(pyridin-3-yl)pyrimidin-2-yl)amino)phenyl)benzamide;
4-(4-(4-(4-(2,4-dioxotetrahydropyrimidin-1(2H)-yl)benzyl)piperazin-1-yl)piperidin-1-yl)-N-(4-methyl-3-((4-(pyridin-3-yl)pyrimidin-2-yl)amino)phenyl)benzamide;
4-(4-(4-(4-(2,4-dioxotetrahydropyrimidin-1(2H)-yl)-2-fluorobenzyl)piperazin-1-yl)piperidin-1-yl)-N-(4-methyl-3-((4-(pyridin-3-yl)pyrimidin-2-yl)amino)phenyl)benzamide;
4-(4-(4-(3-(2,4-dioxotetrahydropyrimidin-1(2H)-yl)benzyl)piperazin-1-yl)piperidin-1-yl)-N-(4-methyl-3-((4-(pyridin-3-yl)pyrimidin-2-yl)amino)phenyl)benzamide;
4-(4-(4-(2-(2,4-dioxotetrahydropyrimidin-1(2H)-yl)benzyl)piperazin-1-yl)piperidin-1-yl)-N-(4-methyl-3-((4-(pyridin-3-yl)pyrimidin-2-yl)amino)phenyl)benzamide;
4-(4-(4-((5-(2,4-dioxotetrahydropyrimidin-1(2H)-yl)pyridin-2-yl)methyl)piperazin-1-yl)piperidin-1-yl)-N-(4-methyl-3-((4-(pyridin-3-yl)pyrimidin-2-yl)amino)phenyl)benzamide;
4-(4-(4-((6-(2,4-dioxotetrahydropyrimidin-1(2H)-yl)pyridazin-3-yl)methyl)piperazin-1-yl)piperidin-1-yl)-N-(4-methyl-3-((4-(pyridin-3-yl)pyrimidin-2-yl)amino)phenyl)benzamide;
4-(4-(4-((3-(2,4-dioxotetrahydropyrimidin-1(2H)-yl)pyridin-4-yl)methyl)piperazin-1-yl)piperidin-1-yl)-N-(4-methyl-3-((4-(pyridin-3-yl)pyrimidin-2-yl)amino)phenyl)benzamide;
4-(4-(4-(4-((2,6-dioxopiperidin-3-yl)amino)benzyl)piperazin-1-yl)piperidin-1-yl)-N-(4-methyl-3-((4-(pyridin-3-yl)pyrimidin-2-yl)amino)phenyl)benzamide;
4-(4-(4-(3-((2,6-dioxopiperidin-3-yl)amino)benzyl)piperazin-1-yl)piperidin-1-yl)-N-(4-methyl-3-((4-(pyridin-3-yl)pyrimidin-2-yl)amino)phenyl)benzamide;
4-(4-(4-(2-((2,6-dioxopiperidin-3-yl)amino)benzyl)piperazin-1-yl)piperidin-1-yl)-N-(4-methyl-3-((4-(pyridin-3-yl)pyrimidin-2-yl)amino)phenyl)benzamide;
N-(4-(chlorodifluoromethoxy)phenyl)-6-(4-((4-(2,6-dioxopiperidin-3-yl)benzyl)(methyl)amino)piperidin-1-yl)-5-(1H-pyrazol-3-yl)nicotinamide;
N-(4-(chlorodifluoromethoxy)phenyl)-6-(4-((4-(2,6-dioxopiperidin-3-yl)-3-fluorobenzyl)(methyl)amino)piperidin-1-yl)-5-(1H-pyrazol-3-yl)nicorinamide;
N-(4-(chlorodifluoromethoxy)phenyl)-6-(4-((3-(2,6-dioxopiperidin-3-yl)benzyl)(methyl)amino)piperidin-1-yl)-5-(1H-pyrazol-3-yl)nicotinamide;
N-(4-(chlorodifluoromethoxy)phenyl)-6-(4-((2-(2,6-dioxopiperidin-3-yl)benzyl)(methyl)amino)piperidin-1-yl)-5-(1H-pyrazol-3-yl)nicotinamide;
N-(4-(chlorodifluoromethoxy)phenyl)-6-(4-((4-(2,4-dioxotetrahydropyrimidin-1(2H)-yl)benzyl)(methyl)amino)piperidin-1-yl)-5-(1H-pyrazol-3-yl)nicotinamide;
N-(4-(chlorodifluoromethoxy)phenyl)-6-(4-((4-(2,4-dioxotetrahydropyrimidin-1(2H)-yl)-2-fluorobenzyl)(methyl)amino)piperidin-1-yl)-5-(1H-pyrazol-3-yl)nicotinamide;
N-(4-(chlorodifluoromethoxy)phenyl)-6-(4-((3-(2,4-dioxotetrahydropyrimidin-1(2H)-yl)benzyl)(methyl)amino)piperidin-1-yl)-5-(1H-pyrazol-3-yl)nicotinamide;
N-(4-(chlorodifluoromethoxy)phenyl)-6-(4-((2-(2,4-dioxotetrahydropyrimidin-1(2H)-yl)benzyl)(methyl)amino)piperidin-1-yl)-5-(1H-pyrazol-3-yl)nicotinamide;
N-(4-(chlorodifluoromethoxy)phenyl)-6-(4-(((5-(2,4-dioxotetrahydropyrimidin-1(2H)-yl)pyridin-2-yl)methyl)(methyl)amino)piperidin-1-yl)-5-(1H-pyrazol-3-yl)nicotinamide;
N-(4-(chlorodifluoromethoxy)phenyl)-6-(4-(((6-(2,4-dioxotetrahydropyrimidin-1(2H)-yl)pyridazin-3-yl)methyl)(methyl)amino)piperidin-1-yl)-5-(1H-pyrazol-3-yl)nicotinamide;
N-(4-(chlorodifluoromethoxy)phenyl)-6-(4-(((3-(2,4-dioxotetrahydropyrimidin-1(2H)-yl)pyridin-4-yl)methyl)(methyl)amino)piperidin-1-yl)-5-(1H-pyrazol-3-yl)nicotinamide;
N-(4-(chlorodifluoromethoxy)phenyl)-6-(4-((4-((2,6-dioxopiperidin-3-yl)amino)benzyl)(methyl)amino)piperidin-1-yl)-5-(1H-pyrazol-3-yl)nicotinamide;
N-(4-(chlorodifluoromethoxy)phenyl)-6-(4-((3-((2,6-dioxopiperidin-3-yl)amino)benzyl)(methyl)amino)piperidin-1-yl)-5-(1H-pyrazol-3-yl)nicotinamide;
N-(4-(chlorodifluoromethoxy)phenyl)-6-(4-((2-((2,6-dioxopiperidin-3-yl)amino)benzyl)(methyl)amino)piperidin-1-yl)-5-(1H-pyrazol-3-yl)nicotinamide;
N-(4-(chlorodifluoromethoxy)phenyl)-6-(4-(1-(4-(2,6-dioxopiperidin-3-yl)benzyl)piperidin-4-yl)piperazin-1-yl)-5-(1H-pyrazol-3-yl)nicotinamide;
N-(4-(chlorodifluoromethoxy)phenyl)-6-(4-(1-(4-(2,6-dioxopiperidin-3-yl)-3-fluorobenzyl)piperidin-4-yl)piperazin-1-yl)-5-(1H-pyrazol-3-yl)nicotinamide;
N-(4-(chlorodifluoromethoxy)phenyl)-6-(4-(1-(3-(2,6-dioxopiperidin-3-yl)benzyl)piperidin-4-yl)piperazin-1-yl)-5-(1H-pyrazol-3-yl)nicotinamide;
N-(4-(chlorodifluoromethoxy)phenyl)-6-(4-(1-(2-(2,6-dioxopiperidin-3-yl)benzyl)piperidin-4-yl)piperazin-1-yl)-5-(1H-pyrazol-3-yl)nicotinamide;
N-(4-(chlorodifluoromethoxy)phenyl)-6-(4-(1-(4-(2,4-dioxotetrahydropyrimidin-1(2H)-yl)benzyl)piperidin-4-yl)piperazin-1-yl)-5-(1H-pyrazol-3-yl)nicotinamide;
N-(4-(chlorodifluoromethoxy)phenyl)-6-(4-(1-(4-(2,4-dioxotetrahydropyrimidin-1(2H)-yl)-2-fluorobenzyl)piperidin-4-yl)piperazin-1-yl)-5-(1H-pyrazol-3-yl)nicotinamide;
N-(4-(chlorodifluoromethoxy)phenyl)-6-(4-(1-(3-(2,4-dioxotetrahydropyrimidin-1(2H)-yl)benzyl)piperidin-4-yl)piperazin-1-yl)-5-(1H-pyrazol-3-yl)nicotinamide;
N-(4-(chlorodifluoromethoxy)phenyl)-6-(4-(1-(2-(2,4-dioxotetrahydropyrimidin-1(2H)-yl)benzyl)piperidin-4-yl)piperazin-1-yl)-5-(1H-pyrazol-3-yl)nicotinamide;
N-(4-(chlorodifluoromethoxy)phenyl)-6-(4-(1-((5-(2,4-dioxotetrahydropyrimidin-1(2H)-yl)pyridin-2-yl)methyl)piperidin-4-yl)piperazin-1-yl)-5-(1H-pyrazol-3-yl)nicotinamide;
N-(4-(chlorodifluoromethoxy)phenyl)-6-(4-(1-((6-(2,4-dioxotetrahydropyrimidin-1(2H)-yl)pyridazin-3-yl)methyl)piperidin-4-yl)piperazin-1-yl)-5-(1H-pyrazol-3-yl)nicotinamide;
N-(4-(chlorodifluoromethoxy)phenyl)-6-(4-(1-((3-(2,4-dioxotetrahydropyrimidin-1(2H)-yl)pyridin-4-yl)methyl)piperidin-4-yl)piperazin-1-yl)-5-(1H-pyrazol-3-yl)nicotinamide;
N-(4-(chlorodifluoromethoxy)phenyl)-6-(4-(1-(4-((2,6-dioxopiperidin-3-yl)amino)benzyl)piperidin-4-yl)piperazin-1-yl)-5-(1H-pyrazol-3-yl)nicotinamide;
N-(4-(chlorodifluoromethoxy)phenyl)-6-(4-(1-(3-((2,6-dioxopiperidin-3-yl)amino)benzyl)piperidin-4-yl)piperazin-1-yl)-5-(1H-pyrazol-3-yl)nicotinamide;
N-(4-(chlorodifluoromethoxy)phenyl)-6-(4-(1-(2-((2,6-dioxopiperidin-3-yl)amino)benzyl)piperidin-4-yl)piperazin-1-yl)-5-(1H-pyrazol-3-yl)nicotinamide;
4-((2,4-dichloro-5-methoxyphenyl)amino)-7-(3-(4-(4-(2,6-dioxopiperidin-3-yl)benzyl)piperazin-1-yl)propoxy)-6-methoxyquinoline-3-carbonitrile;
4-((2,4-dichloro-5-methoxyphenyl)amino)-7-(3-(4-(4-(2,6-dioxopiperidin-3-yl)-3-fluorobenzyl)piperazin-1-yl)propoxy)-6-methoxyquinoline-3-carbonitrile;
4-((2,4-dichloro-5-methoxyphenyl)amino)-7-(3-(4-(3-(2,6-dioxopiperidin-3-yl)benzyl)piperazin-1-yl)propoxy)-6-methoxyquinoline-3-carbonitrile;
4-((2,4-dichloro-5-methoxyphenyl)amino)-7-(3-(4-(2-(2,6-dioxopiperidin-3-yl)benzyl)piperazin-1-yl)propoxy)-6-methoxyquinoline-3-carbonitrile;
4-((2,4-dichloro-5-methoxyphenyl)amino)-7-(3-(4-(4-(2,4-dioxotetrahydropyrimidin-1(2H)-yl)benzyl)piperazin-1-yl)propoxy)-6-methoxyquinoline-3-carbonitrile;
4-((2,4-dichloro-5-methoxyphenyl)amino)-7-(3-(4-(4-(2,4-dioxotetrahydropyrimidin-1(2H)-yl)-2-fluorobenzyl)piperazin-1-yl)propoxy)-6-methoxyquinoline-3-carbonitrile;
4-((2,4-dichloro-5-methoxyphenyl)amino)-7-(3-(4-(3-(2,4-dioxotetrahydropyrimidin-1(2H)-yl)benzyl)piperazin-1-yl)propoxy)-6-methoxyquinoline-3-carbonitrile;
4-((2,4-dichloro-5-methoxyphenyl)amino)-7-(3-(4-(2-(2,4-dioxotetrahydropyrimidin-1(2H)-yl)benzyl)piperazin-1-yl)propoxy)-6-methoxyquinoline-3-carbonitrile;
4-((2,4-dichloro-5-methoxyphenyl)amino)-7-(3-(4-((5-(2,4-dioxotetrahydropyrimidin-1(2H)-yl)pyridin-2-yl)methyl)piperazin-1-yl)propoxy)-6-methoxyquinoline-3-carbonitrile;
4-((2,4-dichloro-5-methoxyphenyl)amino)-7-(3-(4-((6-(2,4-dioxotetrahydropyrimidin-1(2H)-yl)pyridazin-3-yl)methyl)piperazin-1-yl)propoxy)-6-methoxyquinoline-3-carbonitrile;
4-((2,4-dichloro-5-methoxyphenyl)amino)-7-(3-(4-((3-(2,4-dioxotetrahydropyrimidin-1(2H)-yl)pyridin-4-yl)methyl)piperazin-1-yl)propoxy)-6-methoxyquinoline-3-carbonitrile;
4-((2,4-dichloro-5-methoxyphenyl)amino)-7-(3-(4-(4-((2,6-dioxopiperidin-3-yl)amino)benzyl)piperazin-1-yl)propoxy)-6-methoxyquinoline-3-carbonitrile;
4-((2,4-dichloro-5-methoxyphenyl)amino)-7-(3-(4-(3-((2,6-dioxopiperidin-3-yl)amino)benzyl)piperazin-1-yl)propoxy)-6-methoxyquinoline-3-carbonitrile;
4-((2,4-dichloro-5-methoxyphenyl)amino)-7-(3-(4-(2-(2,6-dioxopiperidin-3-yl)benzyl)piperazin-1-yl)propoxy)-6-methoxyquinoline-3-carbonitrile;
N-(4-((4-(4-(2,6-dioxopiperidin-3-yl)benzyl)piperazin-1-yl)methyl)-3-(trifluoromethyl)phenyl)-3-(imidazo[1,2-b]pyridazin-3-ylethynyl)-4-methylbenzamide;
N-(4-((4-(4-(2,6-dioxopiperidin-3-yl)-3-fluorobenzyl)piperazin-1-yl)methyl)-3-(trifluoromethyl)phenyl)-3-(imidazo[1,2-b]pyridazin-3-ylethynyl)-4-methylbenzamide;
N-(4-((4-(3-(2,6-dioxopiperidin-3-yl)benzyl)piperazin-1-yl)methyl)-3-(trifluoromethyl)phenyl)-3-(imidazo[1,2-b]pyridazin-3-ylethynyl)-4-methylbenzamide;
N-(4-((4-(2-(2,6-dioxopiperidin-3-yl)benzyl)piperazin-1-yl)methyl)-3-(trifluoromethyl)phenyl)-3-(imidazo[1,2-b]pyridazin-3-ylethynyl)-4-methylbenzamide;
N-(4-((4-(4-(2,4-dioxotetrahydropyrimidin-1(2H)-yl)benzyl)piperazin-1-yl)methyl)-3-(trifluoromethyl)phenyl)-3-(imidazo[1,2-b]pyridazin-3-ylethynyl)-4-methylbenzamide;
N-(4-((4-(4-(2,4-dioxotetrahydropyrimidin-1(2H)-yl)-2-fluorobenzyl)piperazin-1-yl)methyl)-3-(trifluoromethyl)phenyl)-3-(imidazo[1,2-b]pyridazin-3-ylethynyl)-4-methylbenzamide;
N-(4-((4-(3-(2,4-dioxotetrahydropyrimidin-1(2H)-yl)benzyl)piperazin-1-yl)methyl)-3-(trifluoromethyl)phenyl)-3-(imidazo[1,2-b]pyridazin-3-ylethynyl)-4-methylbenzamide;
N-(4-((4-(2-(2,4-dioxotetrahydropyrimidin-1(2H)-yl)benzyl)piperazin-1-yl)methyl)-3-(trifluoromethyl)phenyl)-3-(imidazo[1,2-b]pyridazin-3-ylethynyl)-4-methylbenzamide;
N-(4-((4-((5-(2,4-dioxotetrahydropyrimidin-1(2H)-yl)pyridin-2-yl)methyl)piperazin-1-yl)methyl)-3-(trifluoromethyl)phenyl)-3-(imidazo[1,2-b]pyridazin-3-ylethynyl)-4-methylbenzamide;
N-(4-((4-((6-(2,4-dioxotetrahydropyrimidin-1(2H)-yl)pyridazin-3-yl)methyl)piperazin-1-yl)methyl)-3-(trifluoromethyl)phenyl)-3-(imidazo[1,2-b]pyridazin-3-ylethynyl)-4-methylbenzamide;
N-(4-((4-((3-(2,4-dioxotetrahydropyrimidin-1(2H)-yl)pyridin-4-yl)methyl)piperazin-1-yl)methyl)-3-(trifluoromethyl)phenyl)-3-(imidazo[1,2-b]pyridazin-3-ylethynyl)-4-methylbenzamide;
N-(4-((4-(4-((2,6-dioxopiperidin-3-yl)amino)benzyl)piperazin-1-yl)methyl)-3-(trifluoromethyl)phenyl)-3-(imidazo[1,2-b]pyridazin-3-ylethynyl)-4-methylbenzamide;
N-(4-((4-(3-((2,6-dioxopiperidin-3-yl)amino)benzyl)piperazin-1-yl)methyl)-3-(trifluoromethyl)phenyl)-3-(imidazo[1,2-b]pyridazin-3-ylethynyl)-4-methylbenzamide;
N-(4-((4-(2-((2,6-dioxopiperidin-3-yl)amino)benzyl)piperazin-1-yl)methyl)-3-(trifluoromethyl)phenyl)-3-(imidazo[1,2-b]pyridazin-3-ylethynyl)-4-methylbenzamide;
N-(4-(4-(4-(2,6-dioxopiperidin-3-yl)benzyl)piperazin-1-yl)-3-(trifluoromethyl)phenyl)-3-(imidazo[1,2-b]pyridazin-3-ylethynyl)-4-methylbenzamide;
N-(4-(4-(4-(2,6-dioxopiperidin-3-yl)-3-fluorobenzyl)piperazin-1-yl)-3-(trifluoromethyl)phenyl)-3-(imidazo[1,2-b]pyridazin-3-ylethynyl)-4-methylbenzamide;
N-(4-(4-(3-(2,6-dioxopiperidin-3-yl)benzyl)piperazin-1-yl)-3-(trifluoromethyl)phenyl)-3-(imidazo[1,2-b]pyridazin-3-ylethynyl)-4-methylbenzamide;
N-(4-(4-(2-(2,6-dioxopiperidin-3-yl)benzyl)piperazin-1-yl)-3-(trifluoromethyl)phenyl)-3-(imidazo[1,2-b]pyridazin-3-ylethynyl)-4-methylbenzamide;
N-(4-(4-(4-(2,4-dioxotetrahydropyrimidin-1(2H)-yl)benzyl)piperazin-1-yl)-3-(trifluoromethyl)phenyl)-3-(imidazo[1,2-b]pyridazin-3-ylethynyl)-4-methylbenzamide;
N-(4-(4-(4-(2,4-dioxotetrahydropyrimidin-1(2H)-yl)-2-fluorobenzyl)piperazin-1-yl)-3-(trifluoromethyl)phenyl)-3-(imidazo[1,2-b]pyridazin-3-ylethynyl)-4-methylbenzamide;
N-(4-(4-(3-(2,4-dioxotetrahydropyrimidin-1(2H)-yl)benzyl)piperazin-1-yl)-3-(trifluoromethyl)phenyl)-3-(imidazo[1,2-b]pyridazin-3-ylethynyl)-4-methylbenzamide;
N-(4-(4-(2-(2,4-dioxotetrahydropyrimidin-1(2H)-yl)benzyl)piperazin-1-yl)-3-(trifluoromethyl)phenyl)-3-(imidazo[1,2-b]pyridazin-3-ylethynyl)-4-methylbenzamide;
N-(4-(4-((5-(2,4-dioxotetrahydropyrimidin-1(2H)-yl)pyridin-2-yl)methyl)piperazin-1-yl)-3-(trifluoromethyl)phenyl)-3-(imidazo[1,2-b]pyridazin-3-ylethynyl)-4-methylbenzamide;
N-(4-(4-((6-(2,4-dioxotetrahydropyrimidin-1(2H)-yl)pyridazin-3-yl)methyl)piperazin-1-yl)-3-(trifluoromethyl)phenyl)-3-(imidazo[1,2-b]pyridazin-3-ylethynyl)-4-methylbenzamide;
N-(4-(4-((3-(2,4-dioxotetrahydropyrimidin-1(2H)-yl)pyridin-4-yl)methyl)piperazin-1-yl)-3-(trifluoromethyl)phenyl)-3-(imidazo[1,2-b]pyridazin-3-ylethynyl)-4-methylbenzamide;
N-(4-(4-(4-((2,6-dioxopiperidin-3-yl)amino)benzyl)piperazin-1-yl)-3-(trifluoromethyl)phenyl)-3-(imidazo[1,2-b]pyridazin-3-ylethynyl)-4-methylbenzamide;
N-(4-(4-(3-((2,6-dioxopiperidin-3-yl)amino)benzyl)piperazin-1-yl)-3-(trifluoromethyl)phenyl)-3-(imidazo[1,2-b]pyridazin-3-ylethynyl)-4-methylbenzamide;
N-(4-(4-(2-((2,6-dioxopiperidin-3-yl)amino)benzyl)piperazin-1-yl)-3-(trifluoromethyl)phenyl)-3-(imidazo[1,2-b]pyridazin-3-ylethynyl)-4-methylbenzamide;
N-(4-(4-(1-(4-(2,6-dioxopiperidin-3-yl)benzyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-(4-(2,6-dioxopiperidin-3-yl)-3-fluorobenzyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-(3-(2,6-dioxopiperidin-3-yl)benzyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-(2-(2,6-dioxopiperidin-3-yl)benzyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(l-(4-(2,4-dioxotetrahydropyrimidin-1(2H)-yl)benzyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-(4-(2,4-dioxotetrahydropyrimidin-1(2H)-yl)-2-fluorobenzyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(l-(3-(2,4-dioxotetrahydropyrimidin-1(2H)-yl)benzyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-(2-(2,4-dioxotetrahydropyrimidin-1(2H)-yl)benzyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(l-((5-(2,4-dioxotetrahydropyrimidin-1(2H)-yl)pyridin-2-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-((6-(2,4-dioxotetrahydropyrimidin-1(2H)-yl)pyridazin-3-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(l-((3-(2,4-dioxotetrahydropyrimidin-1(2H)-yl)pyridin-4-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-(3-((2,6-dioxopiperidin-3-yl)amino)benzyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-(3-((2,6-dioxopiperidin-3-yl)amino)benzyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(1-(2-((2,6-dioxopiperidin-3-yl)amino)benzyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-((4-(2,6-dioxopiperidin-3-yl)benzyl)(methyl)amino)piperidin-1-yl)-3-(trifluoromethyl)phenyl)-3-(imidazo[1,2-b]pyridazin-3-ylethynyl)-4-methylbenzamide;
N-(4-(4-((4-(2,6-dioxopiperidin-3-yl)-3-fluorobenzyl)(methyl)amino)piperidin-1-yl)-3-(trifluoromethyl)phenyl)-3-(imidazo[1,2-b]pyridazin-3-ylethynyl)-4-methylbenzamide;
N-(4-(4-((3-(2,6-dioxopiperidin-3-yl)benzyl)(methyl)amino)piperidin-1-yl)-3-(trifluoromethyl)phenyl)-3-(imidazo[1,2-b]pyridazin-3-ylethynyl)-4-methylbenzamide;
N-(4-(4-((2-(2,6-dioxopiperidin-3-yl)benzyl)(methyl)amino)piperidin-1-yl)-3-(trifluoromethyl)phenyl)-3-(imidazo[1,2-b]pyridazin-3-ylethynyl)-4-methylbenzamide;
N-(4-(4-((4-(2,4-dioxotetrahydropyrimidin-1(2H)-yl)benzyl)(methyl)amino)piperidin-1-yl)-3-(trifluoromethyl)phenyl)-3-(imidazo[1,2-b]pyridazin-3-ylethynyl)-4-methylbenzamide;
N-(4-(4-((4-(2,4-dioxotetrahydropyrimidin-1(2H)-yl)-2-fluorobenzyl)(methyl)amino)piperidin-1-yl)-3-(trifluoromethyl)phenyl)-3-(imidazo[1,2-b]pyridazin-3-ylethynyl)-4-methylbenzamide;
N-(4-(4-((3-(2,4-dioxotetrahydropyrimidin-1(2H)-yl)benzyl)(methyl)amino)piperidin-1-yl)-3-(trifluoromethyl)phenyl)-3-(imidazo[1,2-b]pyridazin-3-ylethynyl)-4-methylbenzamide;
N-(4-(4-((2-(2,4-dioxotetrahydropyrimidin-1(2H)-yl)benzyl)(methyl)amino)piperidin-1-yl)-3-(trifluoromethyl)phenyl)-3-(imidazo[1,2-b]pyridazin-3-ylethynyl)-4-methylbenzamide;
N-(4-(4-(((5-(2,4-dioxotetrahydropyrimidin-1(2H)-yl)pyridin-2-yl)methyl)(methyl)amino)piperidin-1-yl)-3-(trifluoromethyl)phenyl)-3-(imidazo[1,2-b]pyridazin-3-ylethynyl)-4-methylbenzamide;
N-(4-(4-(((6-(2,4-dioxotetrahydropyrimidin-1(2H)-yl)pyridazin-3-yl)methyl)(methyl)amino)piperidin-1-yl)-3-(trifluoromethyl)phenyl)-3-(imidazo[1,2-b]pyridazin-3-ylethynyl)-4-methylbenzamide;
N-(4-(4-(((3-(2,4-dioxotetrahydropyrimidin-1(2H)-yl)pyridin-4-yl)methyl)(methyl)amino)piperidin-1-yl)-3-(trifluoromethyl)phenyl)-3-(imidazo[1,2-b]pyridazin-3-ylethynyl)-4-methylbenzamide;
N-(4-(4-((4-((2,6-dioxopiperidin-3-yl)amino)benzyl)(methyl)amino)piperidin-1-yl)-3-(trifluoromethyl)phenyl)-3-(imidazo[1,2-b]pyridazin-3-ylethynyl)-4-methylbenzamide;
N-(4-(4-((3-((2,6-dioxopiperidin-3-yl)amino)benzyl)(methyl)amino)piperidin-1-yl)-3-(trifluoromethyl)phenyl)-3-(imidazo[1,2-b]pyridazin-3-ylethynyl)-4-methylbenzamide;
N-(4-(4-((2-((2,6-dioxopiperidin-3-yl)amino)benzyl)(methyl)amino)piperidin-1-yl)-3-(trifluoromethyl)phenyl)-3-(imidazo[1,2-b]pyridazin-3-ylethynyl)-4-methylbenzamide;
2-(4-((3S)-1-(4-(2,6-dioxopiperidin-3-yl)benzyl)piperidin-3-yl)phenyl)-2H-indazole-7-carboxamide;
2-(4-((3S)-1-(4-(2,6-dioxopiperidin-3-yl)-3-fluorobenzyl)piperidin-3-yl)phenyl)-2H-indazole-7-carboxamide;
2-(4-((3S)-1-(3-(2,6-dioxopiperidin-3-yl)benzyl)piperidin-3-yl)phenyl)-2H-indazole-7-carboxamide;
2-(4-((3S)-1-(2-(2,6-dioxopiperidin-3-yl)benzyl)piperidin-3-yl)phenyl)-2H-indazole-7-carboxamide;
(S)-2-(4-(1-(4-(2,4-dioxotetrahydropyrimidin-1(2H)-yl)benzyl)piperidin-3-yl)phenyl)-2H-indazole-7-carboxamide;
(S)-2-(4-(1-(4-(2,4-dioxotetrahydropyrimidin-1(2H)-yl)-2-fluorobenzyl)piperidin-3-yl)phenyl)-2H-indazole-7-carboxamide;
(S)-2-(4-(1-(3-(2,4-dioxotetrahydropyrimidin-1(2H)-yl)benzyl)piperidin-3-yl)phenyl)-2H-indazole-7-carboxamide;
(S)-2-(4-(1-(2-(2,4-dioxotetrahydropyrimidin-1(2H)-yl)benzyl)piperidin-3-yl)phenyl)-2H-indazole-7-carboxamide;
(S)-2-(4-(1-((5-(2,4-dioxotetrahydropyrimidin-1(2H)-yl)pyridin-2-yl)methyl)piperidin-3-yl)phenyl)-2H-indazole-7-carboxamide;
(S)-2-(4-(1-((6-(2,4-dioxotetrahydropyrimidin-1(2H)-yl)pyridazin-3-yl)methyl)piperidin-3-yl)phenyl)-2H-indazole-7-carboxamide;
(S)-2-(4-(1-((3-(2,4-dioxotetrahydropyrimidin-1(2H)-yl)pyridin-4-yl)methyl)piperidin-3-yl)phenyl)-2H-indazole-7-carboxamide;
2-(4-((3S)-1-(4-((2,6-dioxopiperidin-3-yl)amino)benzyl)piperidin-3-yl)phenyl)-2H-indazole-7-carboxamide;
2-(4-((3S)-1-(3-((2,6-dioxopiperidin-3-yl)amino)benzyl)piperidin-3-yl)phenyl)-2H-indazole-7-carboxamide;
2-(4-((3S)-1-(2-((2,6-dioxopiperidin-3-yl)amino)benzyl)piperidin-3-yl)phenyl)-2H-indazole-7-carboxamide;
2-(4-(4-(4-(2,6-dioxopiperidin-3-yl)benzyl)piperazin-1-yl)phenyl)-2H-indazole-7-carboxamide;
2-(4-(4-(4-(2,6-dioxopiperidin-3-yl)-3-fluorobenzyl)piperazin-1-yl)phenyl)-2H-indazole-7-carboxamide;
2-(4-(4-(3-(2,6-dioxopiperidin-3-yl)benzyl)piperazin-1-yl)phenyl)-2H-indazole-7-carboxamide;
2-(4-(4-(2-(2,6-dioxopiperidin-3-yl)benzyl)piperazin-1-yl)phenyl)-2H-indazole-7-carboxamide;
2-(4-(4-(4-(2,4-dioxotetrahydropyrimidin-1(2H)-yl)benzyl)piperazin-1-yl)phenyl)-2H-indazole-7-carboxamide;
2-(4-(4-(4-(2,4-dioxotetrahydropyrimidin-1(2H)-yl)-3-fluorobenzyl)piperazin-1-yl)phenyl)-2H-indazole-7-carboxamide;
2-(4-(4-(3-(2,4-dioxotetrahydropyrimidin-1(2H)-yl)benzyl)piperazin-1-yl)phenyl)-2H-indazole-7-carboxamide;
2-(4-(4-(2-(2,4-dioxotetrahydropyrimidin-1(2H)-yl)benzyl)piperazin-1-yl)phenyl)-2H-indazole-7-carboxamide;
2-(4-(4-((5-(2,4-dioxotetrahydropyrimidin-1(2H)-yl)pyridin-2-yl)methyl)piperazin-1-yl)phenyl)-2H-indazole-7-carboxamide;
2-(4-(4-((6-(2,4-dioxotetrahydropyrimidin-1(2H)-yl)pyridazin-3-yl)methyl)piperazin-1-yl)phenyl)-2H-indazole-7-carboxamide;
2-(4-(4-((3-(2,4-dioxotetrahydropyrimidin-1(2H)-yl)pyridin-4-yl)methyl)piperazin-1-yl)phenyl)-2H-indazole-7-carboxamide;
2-(4-(4-(4-((2,6-dioxopiperidin-3-yl)amino)benzyl)piperazin-1-yl)phenyl)-2H-indazole-7-carboxamide;
2-(4-(4-(3-((2,6-dioxopiperidin-3-yl)amino)benzyl)piperazin-1-yl)phenyl)-2H-indazole-7-carboxamide;
2-(4-(4-(2-((2,6-dioxopiperidin-3-yl)amino)benzyl)piperazin-1-yl)phenyl)-2H-indazole-7-carboxamide;
3-(4-((4-(2-fluoro-5-((4-oxo-3,4-dihydrophthalazin-1-yl)methyl)benzoyl)piperazin-1-yl)methyl)phenyl)piperidine-2,6-dione;
3-(2-fluoro-4-((4-(2-fluoro-5-((4-oxo-3,4-dihydrophthalazin-1-yl)methyl)benzoyl)piperazin-1-yl)methyl)phenyl)piperidine-2,6-dione;
3-(3-((4-(2-fluoro-5-((4-oxo-3,4-dihydrophthalazin-1-yl)methyl)benzoyl)piperazin-1-yl)methyl)phenyl)piperidine-2,6-dione;
3-(2-((4-(2-fluoro-5-((4-oxo-3,4-dihydrophthalazin-1-yl)methyl)benzoyl)piperazin-1-yl)methyl)phenyl)piperidine-2,6-dione;
1-(4-((4-(2-fluoro-5-((4-oxo-3,4-dihydrophthalazin-1-yl)methyl)benzoyl)piperazin-1-yl)methyl)phenyl)dihydropyrimidine-2,4(lH,3H)-dione;
1-(3-fluoro-4-((4-(2-fluoro-5-((4-oxo-3,4-dihydrophthalazin-1-yl)methyl)benzoyl)piperazin-1-yl)methyl)phenyl)dihydropyrimidine-2,4(1H,3H)-dione;
1-(3-((4-(2-fluoro-5-((4-oxo-3,4-dihydrophthalazin-1-yl)methyl)benzoyl)piperazin-1-yl)methyl)phenyl)dihydropyrimidine-2,4(1H,3H)-dione;
1-(2-((4-(2-fluoro-5-((4-oxo-3,4-dihydrophthalazin-1-yl)methyl)benzoyl)piperazin-1-yl)methyl)phenyl)dihydropyrimidine-2,4(1H,3H)-dione;
1-(6-((4-(2-fluoro-5-((4-oxo-3,4-dihydrophthalazin-1-yl)methyl)benzoyl)piperazin-1-yl)methyl)pyridin-3-yl)dihydropyrimidine-2,4(1H,3H)-dione;
1-(6-((4-(2-fluoro-5-((4-oxo-3,4-dihydrophthalazin-1-yl)methyl)benzoyl)piperazin-1-yl)methyl)pyridazin-3-yl)dihydropyrimidine-2,4(1H,3H)-dione;
1-(4-((4-(2-fluoro-5-((4-oxo-3,4-dihydrophthalazin-1-yl)methyl)benzoyl)piperazin-1-yl)methyl)pyridin-3-yl)dihydropyrimidine-2,4(1H,3H)-dione;
3-((4-((4-(2-fluoro-5-((4-oxo-3,4-dihydrophthalazin-1-yl)methyl)benzoyl)piperazin-1-yl)methyl)phenyl)amino)piperidine-2,6-dione;
3-((3-((4-(2-fluoro-5-((4-oxo-3,4-dihydrophthalazin-1-yl)methyl)benzoyl)piperazin-1-yl)methyl)phenyl)amino)piperidine-2,6-dione;
3-((2-((4-(2-fluoro-5-((4-oxo-3,4-dihydrophthalazin-1-yl)methyl)benzoyl)piperazin-1-yl)methyl)phenyl)amino)piperidine-2,6-dione;
3-(4-(((4-(8-fluoro-1-oxo-2,3,4,6-tetrahydro-1H-azepino[5,4,3-cd]indol-5-yl)benzyl)(methyl)amino)methyl)phenyl)piperidine-2,6-dione;
3-(2-fluoro-4-(((4-(8-fluoro-1-oxo-2,3,4,6-tetrahydro-1H-azepino[5,4,3-cd]indol-5-yl)benzyl)(methyl)amino)methyl)phenyl)piperidine-2,6-dione;
3-(3-(((4-(8-fluoro-1-oxo-2,3,4,6-tetrahydro-1H-azepino[5,4,3-cd]indol-5-yl)benzyl)(methyl)amino)methyl)phenyl)piperidine-2,6-dione;
3-(2-(((4-(8-fluoro-1-oxo-2,3,4,6-tetrahydro-1H-azepino[5,4,3-cd]indol-5-yl)benzyl)(methyl)amino)methyl)phenyl)piperidine-2,6-dione;
1-(4-(((4-(8-fluoro-1-oxo-2,3,4,6-tetrahydro-1H-azepino[5,4,3-cd]indol-5-yl)benzyl)(methyl)amino)methyl)phenyl)dihydropyrimidine-2,4(1H,3H)-dione;
1-(3-fluoro-4-(((4-(8-fluoro-1-oxo-2,3,4,6-tetrahydro-1H-azepino[5,4,3-cd]indol-5-yl)benzyl)(methyl)amino)methyl)phenyl)dihydropyrimidine-2,4(1H,3H)-dione;
1-(3-(((4-(8-fluoro-1-oxo-2,3,4,6-tetrahydro-1H-azepino[5,4,3-cd]indol-5-yl)benzyl)(methyl)amino)methyl)phenyl)dihydropyrimidine-2,4(1H,3H)-dione;
1-(2-(((4-(8-fluoro-1-oxo-2,3,4,6-tetrahydro-1H-azepino[5,4,3-cd]indol-5-yl)benzyl)(methyl)amino)methyl)phenyl)dihydropyrimidine-2,4(1H,3H)-dione;
3-((4-(((4-(8-fluoro-1-oxo-2,3,4,6-tetrahydro-1H-azepino[5,4,3-cd]indol-5-yl)benzyl)(methyl)amino)methyl)phenyl)amino)piperidine-2,6-dione;
3-((3-(((4-(8-fluoro-1-oxo-2,3,4,6-tetrahydro-1H-azepino[5,4,3-cd]indol-5-yl)benzyl)(methyl)amino)methyl)phenyl)amino)piperidine-2,6-dione;
3-((2-(((4-(8-fluoro-1-oxo-2,3,4,6-tetrahydro-1H-azepino[5,4,3-cd]indol-5-yl)benzyl)(methyl)amino)methyl)phenyl)amino)piperidine-2,6-dione;
3-((4-(4-(4-(4-(2,6-dioxopiperidin-3-yl)benzyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-(4-(2,6-dioxopiperidin-3-yl)-3-fluorobenzyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-(3-(2,6-dioxopiperidin-3-yl)benzyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-(2-(2,6-dioxopiperidin-3-yl)benzyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-(4-(2,4-dioxotetrahydropyrimidin-1(2H)-yl)benzyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-(4-(2,4-dioxotetrahydropyrimidin-1(2H)-yl)-3-fluorobenzyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-(3-(2,4-dioxotetrahydropyrimidin-1(2H)-yl)benzyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-(2-(2,4-dioxotetrahydropyrimidin-1(2H)-yl)benzyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-((5-(2,4-dioxotetrahydropyrimidin-1(2H)-yl)pyridin-2-yl)methyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-((6-(2,4-dioxotetrahydropyrimidin-1(2H)-yl)pyridazin-3-yl)methyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-((3-(2,4-dioxotetrahydropyrimidin-1(2H)-yl)pyridin-4-yl)methyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-(4-((2,6-dioxopiperidin-3-yl)amino)benzyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-(3-((2,6-dioxopiperidin-3-yl)amino)benzyl)piperazin-1-yl)piperidin-1-yl)-3-methoxyphenyl)amino)-6-ethyl-5-((tetrahydro-2H-pyran-4-yl)amino)pyrazine-2-carboxamide;
3-((4-(4-(4-(2-((2,6-dioxopiperidin-3-yl)amino)benzyl)piperazin-1-yl)piperidin-1-yl)-3-methoxyphenyl)amino)-6-ethyl-5-((tetrahydro-2H-pyran-4-yl)amino)pyrazine-2-carboxamide;
7-cyclopentyl-2-((5-(4-(4-(2,6-dioxopiperidin-3-yl)benzyl)piperazin-1-yl)pyridin-2-yl)amino)-N,N-dimethyl-7H-pyrrolo[2,3-d]pyrimidine-6-carboxamide;
7-cyclopentyl-2-((5-(4-(4-(2,6-dioxopiperidin-3-yl)-3-fluorobenzyl)piperazin-1-yl)pyridin-2-yl)amino)-N,N-dimethyl-7H-pyrrolo[2,3-d]pyrimidine-6-carboxamide;
7-cyclopentyl-2-((5-(4-(3-(2,6-dioxopiperidin-3-yl)benzyl)piperazin-1-yl)pyridin-2-yl)amino)-N,N-dimethyl-7H-pyrrolo[2,3-d]pyrimidine-6-carboxamide;
7-cyclopentyl-2-((5-(4-(2-(2,6-dioxopiperidin-3-yl)benzyl)piperazin-1-yl)pyridin-2-yl)amino)-N,N-dimethyl-7H-pyrrolo[2,3-d]pyrimidine-6-carboxamide;
7-cyclopentyl-2-((5-(4-((2-(2,6-dioxopiperidin-3-yl)-5-fluoropyridin-4-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((2-(2,6-dioxopiperidin-3-yl)pyridin-4-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((5-(2,6-dioxopiperidin-3-yl)pyridin-2-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((5-(2,6-dioxopiperidin-3-yl)pyridin-3-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((5-(2,6-dioxopiperidin-3-yl)-2-fluoropyridin-3-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-(4-(2,4-dioxotetrahydropyrimidin-1(2H)-yl)benzyl)piperazin-1-yl)pyridin-2-yl)amino)-N,N-dimethyl-7H-pyrrolo[2,3-d]pyrimidine-6-carboxamide;
7-cyclopentyl-2-((5-(4-(4-(2,4-dioxotetrahydropyrimidin-1(2H)-yl)-2-fluorobenzyl)piperazin-1-yl)pyridin-2-yl)amino)-N,N-dimethyl-7H-pyrrolo[2,3-d]pyrimidine-6-carboxamide;
7-cyclopentyl-2-((5-(4-(3-(2,4-dioxotetrahydropyrimidin-1(2H)-yl)benzyl)piperazin-1-yl)pyridin-2-yl)amino)-N,N-dimethyl-7H-pyrrolo[2,3-d]pyrimidine-6-carboxamide;
7-cyclopentyl-2-((5-(4-(2-(2,4-dioxotetrahydropyrimidin-1(2H)-yl)benzyl)piperazin-1-yl)pyridin-2-yl)amino)-N,N-dimethyl-7H-pyrrolo[2,3-d]pyrimidine-6-carboxamide;
7-cyclopentyl-2-((5-(4-((5-(2,4-dioxotetrahydropyrimidin-1(2H)-yl)pyridin-2-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((5-(2,4-dioxotetrahydropyrimidin-1(2H)-yl)-3-fluoropyridin-2-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((2-(2,4-dioxotetrahydropyrimidin-1(2H)-yl)pyridin-4-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((2-(2,4-dioxotetrahydropyrimidin-1(2H)-yl)-5-fluoropyridin-4-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((6-(2,4-dioxotetrahydropyrimidin-1(2H)-yl)pyridin-3-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((5-(2,4-dioxotetrahydropyrimidin-1(2H)-yl)pyridin-3-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((5-(2,4-dioxotetrahydropyrimidin-1(2H)-yl)-2-fluoropyridin-3-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((6-(2,4-dioxotetrahydropyrimidin-1(2H)-yl)pyridazin-3-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((5-(2,4-dioxotetrahydropyrimidin-1(2H)-yl)pyrazin-2-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((6-(2,4-dioxotetrahydropyrimidin-1(2H)-yl)pyrazin-2-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((3-(2,4-dioxotetrahydropyrimidin-1(2H)-yl)pyridin-4-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-(4-((2,6-dioxopiperidin-3-yl)amino)benzyl)piperazin-1-yl)pyridin-2-yl)amino)-N,N-dimethyl-7H-pyrrolo[2,3-d]pyrimidine-6-carboxamide;
7-cyclopentyl-2-((5-(4-(3-((2,6-dioxopiperidin-3-yl)amino)benzyl)piperazin-1-yl)pyridin-2-yl)amino)-N,N-dimethyl-7H-pyrrolo[2,3-d]pyrimidine-6-carboxamide;
7-cyclopentyl-2-((5-(4-(2-((2,6-dioxopiperidin-3-yl)amino)benzyl)piperazin-1-yl)pyridin-2-yl)amino)-N,N-dimethyl-7H-pyrrolo[2,3-d]pyrimidine-6-carboxamide;
7-cyclopentyl-2-((5-(4-((4-(2,6-dioxopiperidin-3-yl)benzyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-((4-(2,6-dioxopiperidin-3-yl)-3-fluorobenzyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-((3-(2,6-dioxopiperidin-3-yl)benzyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-((2-(2,6-dioxopiperidin-3-yl)benzyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-((4-(2,4-dioxotetrahydropyrimidin-1(2H)-yl)benzyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-((4-(2,4-dioxotetrahydropyrimidin-1(2H)-yl)-2-fluorobenzyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-((3-(2,4-dioxotetrahydropyrimidin-1(2H)-yl)benzyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-((2-(2,4-dioxotetrahydropyrimidin-1(2H)-yl)benzyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(((5-(2,4-dioxotetrahydropyrimidin-1(2H)-yl)pyridin-2-yl)methyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(((6-(2,4-dioxotetrahydropyrimidin-1(2H)-yl)pyridin-3-yl)methyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(((6-(2,4-dioxotetrahydropyrimidin-1(2H)-yl)pyridazin-3-yl)methyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(((3-(2,4-dioxotetrahydropyrimidin-1(2H)-yl)pyridin-4-yl)methyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-((4-((2,6-dioxopiperidin-3-yl)amino)benzyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-((3-((2,6-dioxopiperidin-3-yl)amino)benzyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-((2-((2,6-dioxopiperidin-3-yl)amino)benzyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(4-(4-(2,6-dioxopiperidin-3-yl)benzyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-(4-(2,6-dioxopiperidin-3-yl)-3-fluorobenzyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-(3-(2,6-dioxopiperidin-3-yl)benzyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-(2-(2,6-dioxopiperidin-3-yl)benzyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-(4-(2,4-dioxotetrahydropyrimidin-1(2H)-yl)benzyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-(4-(2,4-dioxotetrahydropyrimidin-1(2H)-yl)-2-fluorobenzyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-(3-(2,4-dioxotetrahydropyrimidin-1(2H)-yl)benzyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-(2-(2,4-dioxotetrahydropyrimidin-1(2H)-yl)benzyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-((5-(2,4-dioxotetrahydropyrimidin-1(2H)-yl)pyridin-2-yl)methyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-((6-(2,4-dioxotetrahydropyrimidin-1(2H)-yl)pyridazin-3-yl)methyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-((3-(2,4-dioxotetrahydropyrimidin-1(2H)-yl)pyridin-4-yl)methyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-(4-((2,6-dioxopiperidin-3-yl)amino)benzyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-(3-((2,6-dioxopiperidin-3-yl)amino)benzyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(4-(2-((2,6-dioxopiperidin-3-yl)amino)benzyl)piperazin-1-yl)piperidin-1-yl)pyridin-2-yl)amino)-N,N-dimethyl-7H-pyrrolo[2,3-d]pyrimidine-6-carboxamide;
3-(4-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)phenyl)piperidine-2,6-dione;
3-(2-fluoro-4-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)phenyl)piperidine-2,6-dione;
3-(3-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)phenyl)piperidine-2,6-dione;
3-(2-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)phenyl)piperidine-2,6-dione;
1-(4-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)phenyl)dihydropyrimidine-2,4(1H,3H)-dione;
1-(3-fluoro-4-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)phenyl)dihydropyrimidine-2,4(1H,3H)-dione;
1-(3-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)phenyl)dihydropyrimidine-2,4(1H,3H)-dione;
1-(2-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)phenyl)dihydropyrimidine-2,4(1H,3H)-dione;
1-(6-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)pyridin-3-yl)dihydropyrimidine-2,4(1H,3H)-dione;
1-(6-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)pyridazin-3-yl)dihydropyrimidine-2,4(1H,3H)-dione;
1-(4-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)pyridin-3-yl)dihydropyrimidine-2,4(1H,3H)-dione;
3-((4-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)phenyl)amino)piperidine-2,6-dione;
3-((3-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)phenyl)amino)piperidine-2,6-dione;
3-((2-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)phenyl)amino)piperidine-2,6-dione;
3-(4-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)phenyl)piperidine-2,6-dione;
3-(2-fluoro-4-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)phenyl)piperidine-2,6-dione;
3-(3-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)phenyl)piperidine-2,6-dione;
3-(2-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)phenyl)piperidine-2,6-dione;
1-(4-((4-(6-((5-fluoro-4-(4-fluoro-1-isopropyl-2-methyl-1 H-benzo [d] imidazol-6-yl)pyrimidin-2-yl)amino)pyridin-3-yl)piperazin-1-yl)methyl)phenyl)dihydropyrimidine-2,4(1H,3H)-dione;
1-(3-fluoro-4-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)phenyl)dihydropyrimidine-2,4(1H,3H)-dione;
1-(3-((4-(6-((5-fluoro-4-(4-fluoro-1-isopropyl-2-methyl-1H-benzo [d] imidazol-6-yl)pyrimidin-2-yl)amino)pyridin-3-yl)piperazin-1-yl)methyl)phenyl)dihydropyrimidine-2,4(1H,3H)-dione;
1-(2-((4-(6-((5-fluoro-4-(4-fluoro-1-isopropyl-2-methyl-1H-benzo [d] imidazol-6-yl)pyrimidin-2-yl)amino)pyridin-3-yl)piperazin-1-yl)methyl)phenyl)dihydropyrimidine-2,4(1H,3H)-dione;
1-(6-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)pyridin-3-yl)dihydropyrimidine-2,4(1H,3H)-dione;
1-(5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)pyridin-2-yl)dihydropyrimidine-2,4(1H,3H)-dione;
1-(6-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)pyridazin-3-yl)dihydropyrimidine-2,4(1H,3H)-dione;
1-(4-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)pyridin-3-yl)dihydropyrimidine-2,4(1H,3H)-dione;
3-((4-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)phenyl)amino)piperidine-2,6-dione;
3-((3-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)phenyl)amino)piperidine-2,6-dione;
3-((2-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)phenyl)amino)piperidine-2,6-dione;
3-(4-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)phenyl)piperidine-2,6-dione;
3-(2-fluoro-4-(((l-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)phenyl)piperidine-2,6-dione;
3-(3-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)phenyl)piperidine-2,6-dione;
3-(2-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)phenyl)piperidine-2,6-dione;
1-(4-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)phenyl)dihydropyrimidine-2,4(1H,3H)-dione;
1-(3-fluoro-4-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)phenyl)dihydropyrimidine-2,4(1H,3H)-dione;
1-(3-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)phenyl)dihydropyrimidine-2,4(1H,3H)-dione;
1-(2-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)phenyl)dihydropyrimidine-2,4(1H,3H)-dione;
1-(6-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)pyridin-3-yl)dihydropyrimidine-2,4(1H,3H)-dione;
1-(5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)pyridin-2-yl)dihydropyrimidine-2,4(1H,3H)-dione;
3-(4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)piperidine-2,6-dione;
3-(2-fluoro-4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)piperidine-2,6-dione;
3-(3-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)piperidine-2,6-dione;
3-(2-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)piperidine-2,6-dione;
1-(4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)dihydropyrimidine-2,4(1H,3H)-dione;
1-(3-fluoro-4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)dihydropyrimidine-2,4(1H,3H)-dione;
1-(3-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)dihydropyrimidine-2,4(1H,3H)-dione;
1-(2-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)dihydropyrimidine-2,4(1H,3H)-dione;
1-(6-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)pyridin-3-yl)dihydropyrimidine-2,4(1H,3H)-dione;
1-(4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)pyridin-2-yl)dihydropyrimidine-2,4(1H,3H)-dione;
1-(5-fluoro-4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)pyridin-2-yl)dihydropyrimidine-2,4(1H,3H)-dione;
1-(5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)pyridin-2-yl)dihydropyrimidine-2,4(1H,3H)-dione;
1-(5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)pyridin-3-yl)dihydropyrimidine-2,4(1H,3H)-dione;
1-(6-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)pyridazin-3-yl)dihydropyrimidine-2,4(1H,3H)-dione;
1-(4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)pyridin-3-yl)dihydropyrimidine-2,4(1H,3H)-dione;
3-((4-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)amino)piperidine-2,6-dione;
3-((3-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)amino)piperidine-2,6-dione;
3-((2-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)phenyl)amino)piperidine-2,6-dione;
3-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)phenyl)piperidine-2,6-dione;
3-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-2-fluorophenyl)piperidine-2,6-dione;
3-(3-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)phenyl)piperidine-2,6-dione;
3-(2-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)phenyl)piperidine-2,6-dione;
3-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-5-fluoropyridin-2-yl)piperidine-2,6-dione;
3-(6-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-3-yl)piperidine-2,6-dione;
3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-6-fluoropyridin-3-yl)piperidine-2,6-dione;
1-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)phenyl)dihydropyrimidine-2,4(1H,3H)-dione;
1-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-3-fluorophenyl)dihydropyrimidine-2,4(1H,3H)-dione;
1-(3-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)phenyl)dihydropyrimidine-2,4(1H,3H)-dione;
1-(2-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)phenyl)dihydropyrimidine-2,4(1H,3H)-dione;
1-(6-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-3-yl)dihydropyrimidine-2,4(1H,3H)-dione;
1-(6-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-5-fluoropyridin-3-yl)dihydropyrimidine-2,4(1H,3H)-dione;
1-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-2-yl)dihydropyrimidine-2,4(1H,3H)-dione;
1-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-5-fluoropyridin-2-yl)dihydropyrimidine-2,4(1H,3H)-dione;
1-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-2-yl)dihydropyrimidine-2,4(1H,3H)-dione;
1-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-3-yl)dihydropyrimidine-2,4(1H,3H)-dione;
1-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-6-fluoropyridin-3-yl)dihydropyrimidine-2,4(1H,3H)-dione;
1-(6-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridazin-3-yl)dihydropyrimidine-2,4(1H,3H)-dione;
1-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyrazin-2-yl)dihydropyrimidine-2,4(1H,3H)-dione;
1-(6-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyrazin-2-yl)dihydropyrimidine-2,4(1H,3H)-dione;
1-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-3-yl)dihydropyrimidine-2,4(1H,3H)-dione;
3-((4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)phenyl)amino)piperidine-2,6-dione;
3-((3-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)phenyl)amino)piperidine-2,6-dione;
3-((2-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)phenyl)amino)piperidine-2,6-dione;
3-(4-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)phenyl)piperidine-2,6-dione;
3-(4-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)-2-fluorophenyl)piperidine-2,6-dione;
3-(3-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)phenyl)piperidine-2,6-dione;
3-(2-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)phenyl)piperidine-2,6-dione;
3-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-2-yl)piperidine-2,6-dione;
3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)pyridin-3-yl)piperidine-2,6-dione;
1-(4-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)phenyl)dihydropyrimidine-2,4(1H,3H)-dione;
1-(4-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)-3-fluorophenyl)dihydropyrimidine-2,4(1H,3H)-dione;
1-(3-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)phenyl)dihydropyrimidine-2,4(1H,3H)-dione;
1-(2-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)phenyl)dihydropyrimidine-2,4(1H,3H)-dione;
1-(6-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)pyridin-3-yl)dihydropyrimidine-2,4(1H,3H)-dione;
1-(6-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)pyridazin-3-yl)dihydropyrimidine-2,4(1H,3H)-dione;
1-(4-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)pyridin-3-yl)dihydropyrimidine-2,4(1H,3H)-dione;
3-((4-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)phenyl)amino)piperidine-2,6-dione;
3-((3-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)phenyl)amino)piperidine-2,6-dione;
3-((2-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)phenyl)amino)piperidine-2,6-dione;
3-(4-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)phenyl)piperidine-2,6-dione;
3-(4-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-2-fluorophenyl)piperidine-2,6-dione;
3-(3-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)phenyl)piperidine-2,6-dione;
3-(2-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)phenyl)piperidine-2,6-dione;
1-(4-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)phenyl)dihydropyrimidine-2,4(1H,3H)-dione;
1-(4-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-3-fluorophenyl)dihydropyrimidine-2,4(1H,3H)-dione;
1-(3-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)phenyl)dihydropyrimidine-2,4(1H,3H)-dione;
1-(2-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)phenyl)dihydropyrimidine-2,4(1H,3H)-dione;
1-(6-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)pyridin-3-yl)dihydropyrimidine-2,4(1H,3H)-dione;
1-(6-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)pyridazin-3-yl)dihydropyrimidine-2,4(1H,3H)-dione;
1-(4-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)pyridin-3-yl)dihydropyrimidine-2,4(1H,3H)-dione;
3-((4-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)phenyl)amino)piperidine-2,6-dione;
3-((3-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)phenyl)amino)piperidine-2,6-dione;
3-((2-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)phenyl)amino)piperidine-2,6-dione;
3-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)phenyl)piperidine-2,6-dione;
3-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-2-fluorophenyl)piperidine-2,6-dione;
3-(3-(((1-(4-((5-chloro-4-((2-(dimethylphosphoryl)phenyl)amino)pyrimidin-2-yl)amino)-3-methoxyphenyl)piperidin-4-yl)(methyl)amino)methyl)phenyl)piperidine-2,6-dione;
3-(2-(((1-(4-((5-chloro-4-((2-(dimethylphosphoryl)phenyl)amino)pyrimidin-2-yl)amino)-3-methoxyphenyl)piperidin-4-yl)(methyl)amino)methyl)phenyl)piperidine-2,6-dione;
3-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-2-yl)piperidine-2,6-dione;
3-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-5-fluoropyridin-2-yl)piperidine-2,6-dione;
3-(6-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-3-yl)piperidine-2,6-dione;
3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-3-yl)piperidine-2,6-dione;
3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-6-fluoropyridin-3-yl)piperidine-2,6-dione;
1-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)phenyl)dihydropyrimidine-2,4(1H,3H)-dione;
1-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-3-fluorophenyl)dihydropyrimidine-2,4(1H,3H)-dione;
1-(3-(((1-(4-((5-chloro-4-((2-(dimethylphosphoryl)phenyl)amino)pyrimidin-2-yl)amino)-3-methoxyphenyl)piperidin-4-yl)(methyl)amino)methyl)phenyl)dihydropyrimidine-2,4(1H,3H)-dione;
1-(2-(((1-(4-((5-chloro-4-((2-(dimethylphosphoryl)phenyl)amino)pyrimidin-2-yl)amino)-3-methoxyphenyl)piperidin-4-yl)(methyl)amino)methyl)phenyl)dihydropyrimidine-2,4(1H,3H)-dione;
1-(6-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-3-yl)dihydropyrimidine-2,4(1H,3H)-dione;
1-(6-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-5-fluoropyridin-3-yl)dihydropyrimidine-2,4(1H,3H)-dione;
1-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-2-yl)dihydropyrimidine-2,4(1H,3H)-dione;
1-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-5-fluoropyridin-2-yl)dihydropyrimidine-2,4(1H,3H)-dione;
1-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-2-yl)dihydropyrimidine-2,4(1H,3H)-dione;
1-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-3-yl)dihydropyrimidine-2,4(1H,3H)-dione;
1-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-6-fluoropyridin-3-yl)dihydropyrimidine-2,4(1H,3H)-dione;
1-(6-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridazin-3-yl)dihydropyrimidine-2,4(1H,3H)-dione;
1-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyrazin-2-yl)dihydropyrimidine-2,4(1H,3H)-dione;
1-(6-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyrazin-2-yl)dihydropyrimidine-2,4(1H,3H)-dione;
1-(4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)pyridin-3-yl)dihydropyrimidine-2,4(1H,3H)-dione;
3-((4-(((1-(4-((5-chloro-4-((2-(dimethylphosphoryl)phenyl)amino)pyrimidin-2-yl)amino)-3-methoxyphenyl)piperidin-4-yl)(methyl)amino)methyl)phenyl)amino)piperidine-2,6-dione;
3-((3-(((1-(4-((5-chloro-4-((2-(dimethylphosphoryl)phenyl)amino)pyrimidin-2-yl)amino)-3-methoxyphenyl)piperidin-4-yl)(methyl)amino)methyl)phenyl)amino)piperidine-2,6-dione;
3-((2-(((1-(4-((5-chloro-4-((2-(dimethylphosphoryl)phenyl)amino)pyrimidin-2-yl)amino)-3-methoxyphenyl)piperidin-4-yl)(methyl)amino)methyl)phenyl)amino)piperidine-2,6-dione;
3-(4-((4-(4-((5-chloro-4-((2-(dimethylphosphoryl)phenyl)amino)pyrimidin-2-yl)amino)-3-methoxyphenyl)piperazin-1-yl)methyl)phenyl)piperidine-2,6-dione;
3-(4-((4-(4-((5-chloro-4-((2-(dimethylphosphoryl)phenyl)amino)pyrimidin-2-yl)amino)-3-methoxyphenyl)piperazin-1-yl)methyl)-2-fluorophenyl)piperidine-2,6-dione;
3-(3-((4-(4-((5-chloro-4-((2-(dimethylphosphoryl)phenyl)amino)pyrimidin-2-yl)amino)-3-methoxyphenyl)piperazin-1-yl)methyl)phenyl)piperidine-2,6-dione;
3-(2-((4-(4-((5-chloro-4-((2-(dimethylphosphoryl)phenyl)amino)pyrimidin-2-yl)amino)-3-methoxyphenyl)piperazin-1-yl)methyl)phenyl)piperidine-2,6-dione;
3-(6-((4-(4-((5-chloro-4-((2-(dimethylphosphoryl)phenyl)amino)pyrimidin-2-yl)amino)-3-methoxyphenyl)piperazin-1-yl)methyl)pyridin-3-yl)piperidine-2,6-dione;
3-(5-((4-(4-((5-chloro-4-((2-(dimethylphosphoryl)phenyl)amino)pyrimidin-2-yl)amino)-3-methoxyphenyl)piperazin-1-yl)methyl)-6-fluoropyridin-3-yl)piperidine-2,6-dione;
1-(4-((4-(4-((5-chloro-4-((2-(dimethylphosphoryl)phenyl)amino)pyrimidin-2-yl)amino)-3-methoxyphenyl)piperazin-1-yl)methyl)phenyl)dihydropyrimidine-2,4(1H,3H)-dione;
1-(4-((4-(4-((5-chloro-4-((2-(dimethylphosphoryl)phenyl)amino)pyrimidin-2-yl)amino)-3-methoxyphenyl)piperazin-1-yl)methyl)-2-fluorophenyl)dihydropyrimidine-2,4(1H,3H)-dione;
1-(3-((4-(4-((5-chloro-4-((2-(dimethylphosphoryl)phenyl)amino)pyrimidin-2-yl)amino)-3-methoxyphenyl)piperazin-1-yl)methyl)phenyl)dihydropyrimidine-2,4(1H,3H)-dione;
1-(2-((4-(4-((5-chloro-4-((2-(dimethylphosphoryl)phenyl)amino)pyrimidin-2-yl)amino)-3-methoxyphenyl)piperazin-1-yl)methyl)phenyl)dihydropyrimidine-2,4(1H,3H)-dione;
1-(6-((4-(4-((5-chloro-4-((2-(dimethylphosphoryl)phenyl)amino)pyrimidin-2-yl)amino)-3-methoxyphenyl)piperazin-1-yl)methyl)pyridin-3-yl)dihydropyrimidine-2,4(1H,3H)-dione;
1-(6-((4-(4-((5-chloro-4-((2-(dimethylphosphoryl)phenyl)amino)pyrimidin-2-yl)amino)-3-methoxyphenyl)piperazin-1-yl)methyl)-5-fluoropyridin-3-yl)dihydropyrimidine-2,4(1H,3H)-dione;
1-(4-((4-(4-((5-chloro-4-((2-(dimethylphosphoryl)phenyl)amino)pyrimidin-2-yl)amino)-3-methoxyphenyl)piperazin-1-yl)methyl)pyridin-2-yl)dihydropyrimidine-2,4(1H,3H)-dione;
1-(4-((4-(4-((5-chloro-4-((2-(dimethylphosphoryl)phenyl)amino)pyrimidin-2-yl)amino)-3-methoxyphenyl)piperazin-1-yl)methyl)-5-fluoropyridin-2-yl)dihydropyrimidine-2,4(1H,3H)-dione;
1-(5-((4-(4-((5-chloro-4-((2-(dimethylphosphoryl)phenyl)amino)pyrimidin-2-yl)amino)-3-methoxyphenyl)piperazin-1-yl)methyl)pyridin-3-yl)dihydropyrimidine-2,4(1H,3H)-dione;
1-(5-((4-(4-((5-chloro-4-((2-(dimethylphosphoryl)phenyl)amino)pyrimidin-2-yl)amino)-3-methoxyphenyl)piperazin-1-yl)methyl)-6-fluoropyridin-3-yl)dihydropyrimidine-2,4(1H,3H)-dione;
1-(6-((4-(4-((5-chloro-4-((2-(dimethylphosphoryl)phenyl)amino)pyrimidin-2-yl)amino)-3-methoxyphenyl)piperazin-1-yl)methyl)pyridazin-3-yl)dihydropyrimidine-2,4(1H,3H)-dione;
1-(6-((4-(4-((5-chloro-4-((2-(dimethylphosphoryl)phenyl)amino)pyrimidin-2-yl)amino)-3-methoxyphenyl)piperazin-1-yl)methyl)pyrazin-2-yl)dihydropyrimidine-2,4(1H,3H)-dione;
1-(4-((4-(4-((5-chloro-4-((2-(dimethylphosphoryl)phenyl)amino)pyrimidin-2-yl)amino)-3-methoxyphenyl)piperazin-1-yl)methyl)pyridin-3-yl)dihydropyrimidine-2,4(1H,3H)-dione;
3-((4-((4-(4-((5-chloro-4-((2-(dimethylphosphoryl)phenyl)amino)pyrimidin-2-yl)amino)-3-methoxyphenyl)piperazin-1-yl)methyl)phenyl)amino)piperidine-2,6-dione;
3-((3-((4-(4-((5-chloro-4-((2-(dimethylphosphoryl)phenyl)amino)pyrimidin-2-yl)amino)-3-methoxyphenyl)piperazin-1-yl)methyl)phenyl)amino)piperidine-2,6-dione;
3-((2-((4-(4-((5-chloro-4-((2-(dimethylphosphoryl)phenyl)amino)pyrimidin-2-yl)amino)-3-methoxyphenyl)piperazin-1-yl)methyl)phenyl)amino)piperidine-2,6-dione;
3-(4-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)phenyl)piperidine-2,6-dione;
3-(4-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)-2-fluorophenyl)piperidine-2,6-dione;
3-(3-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)phenyl)piperidine-2,6-dione;
3-(2-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)phenyl)piperidine-2,6-dione;
1-(4-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)phenyl)dihydropyrimidine-2,4(1H,3H)-dione;
1-(4-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)-3-fluorophenyl)dihydropyrimidine-2,4(1H,3H)-dione;
1-(3-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)phenyl)dihydropyrimidine-2,4(1H,3H)-dione;
1-(2-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)phenyl)dihydropyrimidine-2,4(1H,3H)-dione;
1-(6-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)pyridin-3-yl)dihydropyrimidine-2,4(1H,3H)-dione;
1-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-2-yl)dihydropyrimidine-2,4(1H,3H)-dione;
1-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-5-fluoropyridin-2-yl)dihydropyrimidine-2,4(1H,3H)-dione;
1-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-2-yl)dihydropyrimidine-2,4(1H,3H)-dione;
1-(6-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)pyridazin-3-yl)dihydropyrimidine-2,4(1H,3H)-dione;
1-(4-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)pyridin-3-yl)dihydropyrimidine-2,4(1H,3H)-dione;
3-((4-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)phenyl)amino)piperidine-2,6-dione;
3-((3-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)phenyl)amino)piperidine-2,6-dione;
3-((2-((4-(4-(4-((5-chloro-4-((2-(dimethylphosphoryl)phenyl)amino)pyrimidin-2-yl)amino)-3-methoxyphenyl)piperazin-1-yl)piperidin-1-yl)methyl)phenyl)amino)piperidine-2,6-dione;
3-(4-((4-(4-((5-chloro-4-((2-(dimethylphosphoryl)phenyl)amino)pyrimidin-2-yl)amino)-3-methoxyphenyl)piperazin-1-yl)methyl)phenyl)piperidine-2,6-dione;
3-(4-((4-(4-((5-chloro-4-((2-(dimethylphosphoryl)phenyl)amino)pyrimidin-2-yl)amino)-3-methoxyphenyl)piperazin-1-yl)methyl)-2-fluorophenyl)piperidine-2,6-dione;
3-(3-((4-(4-((5-chloro-4-((2-(dimethylphosphoryl)phenyl)amino)pyrimidin-2-yl)amino)-3-methoxyphenyl)piperazin-1-yl)methyl)phenyl)piperidine-2,6-dione;
3-(2-((4-(4-((5-chloro-4-((2-(dimethylphosphoryl)phenyl)amino)pyrimidin-2-yl)amino)-3-methoxyphenyl)piperazin-1-yl)methyl)phenyl)piperidine-2,6-dione;
3-(4-((4-(4-((5-chloro-4-((2-(dimethylphosphoryl)phenyl)amino)pyrimidin-2-yl)amino)-3-methoxyphenyl)piperazin-1-yl)methyl)pyridin-2-yl)piperidine-2,6-dione;
3-(4-((4-(4-((5-chloro-4-((2-(dimethylphosphoryl)phenyl)amino)pyrimidin-2-yl)amino)-3-methoxyphenyl)piperazin-1-yl)methyl)-5-fluoropyridin-2-yl)piperidine-2,6-dione;
3-(5-((4-(4-((5-chloro-4-((2-(dimethylphosphoryl)phenyl)amino)pyrimidin-2-yl)amino)-3-methoxyphenyl)piperazin-1-yl)methyl)pyridin-3-yl)piperidine-2,6-dione;
1-(4-((4-(4-((5-chloro-4-((2-(dimethylphosphoryl)phenyl)amino)pyrimidin-2-yl)amino)-3-methoxyphenyl)piperazin-1-yl)methyl)phenyl)dihydropyrimidine-2,4(1H,3H)-dione;
1-(4-((4-(4-((5-chloro-4-((2-(dimethylphosphoryl)phenyl)amino)pyrimidin-2-yl)amino)-3-methoxyphenyl)piperazin-1-yl)methyl)-3-fluorophenyl)dihydropyrimidine-2,4(1H,3H)-dione;
1-(3-((4-(4-((5-chloro-4-((2-(dimethylphosphoryl)phenyl)amino)pyrimidin-2-yl)amino)-3-methoxyphenyl)piperazin-1-yl)methyl)phenyl)dihydropyrimidine-2,4(1H,3H)-dione;
1-(2-((4-(4-((5-chloro-4-((2-(dimethylphosphoryl)phenyl)amino)pyrimidin-2-yl)amino)-3-methoxyphenyl)piperazin-1-yl)methyl)phenyl)dihydropyrimidine-2,4(1H,3H)-dione;
1-(6-((4-(4-((5-chloro-4-((2-(dimethylphosphoryl)phenyl)amino)pyrimidin-2-yl)amino)-3-methoxyphenyl)piperazin-1-yl)methyl)pyridin-3-yl)dihydropyrimidine-2,4(1H,3H)-dione;
1-(5-((4-(4-((5-chloro-4-((2-(dimethylphosphoryl)phenyl)amino)pyrimidin-2-yl)amino)-3-methoxyphenyl)piperazin-1-yl)methyl)pyridin-2-yl)dihydropyrimidine-2,4(1H,3H)-dione;
1-(6-((4-(4-((5-chloro-4-((2-(dimethylphosphoryl)phenyl)amino)pyrimidin-2-yl)amino)-3-methoxyphenyl)piperazin-1-yl)methyl)pyridazin-3-yl)dihydropyrimidine-2,4(1H,3H)-dione;
1-(5-((4-(4-((5-chloro-4-((2-(dimethylphosphoryl)phenyl)amino)pyrimidin-2-yl)amino)-3-methoxyphenyl)piperazin-1-yl)methyl)pyrazin-2-yl)dihydropyrimidine-2,4(1H,3H)-dione;
1-(4-((4-(4-((5-chloro-4-((2-(dimethylphosphoryl)phenyl)amino)pyrimidin-2-yl)amino)-3-methoxyphenyl)piperazin-1-yl)methyl)pyridin-3-yl)dihydropyrimidine-2,4(1H,3H)-dione;
3-((4-((4-(4-((5-chloro-4-((2-(dimethylphosphoryl)phenyl)amino)pyrimidin-2-yl)amino)-3-methoxyphenyl)piperazin-1-yl)methyl)phenyl)amino)piperidine-2,6-dione;
3-((3-((4-(4-((5-chloro-4-((2-(dimethylphosphoryl)phenyl)amino)pyrimidin-2-yl)amino)-3-methoxyphenyl)piperazin-1-yl)methyl)phenyl)amino)piperidine-2,6-dione;
3-((2-((4-(4-((5-chloro-4-((2-(dimethylphosphoryl)phenyl)amino)pyrimidin-2-yl)amino)-3-methoxyphenyl)piperazin-1-yl)methyl)phenyl)amino)piperidine-2,6-dione;
3-(4-((4-(4-(6-amino-5-((R)-1-(2,6-dichloro-3-fluorophenyl)ethoxy)pyridin-3-yl)-1H-pyrazol-1-yl)piperidin-1-yl)methyl)phenyl)piperidine-2,6-dione;
3-(4-((4-(4-(6-amino-5-((R)-1-(2,6-dichloro-3-fluorophenyl)ethoxy)pyridin-3-yl)-1H-pyrazol-1-yl)piperidin-1-yl)methyl)-2-fluorophenyl)piperidine-2,6-dione;
3-(3-((4-(4-(6-amino-5-((R)-1-(2,6-dichloro-3-fluorophenyl)ethoxy)pyridin-3-yl)-1H-pyrazol-1-yl)piperidin-1-yl)methyl)phenyl)piperidine-2,6-dione;
3-(2-((4-(4-(6-amino-5-((R)-1-(2,6-dichloro-3-fluorophenyl)ethoxy)pyridin-3-yl)-1H-pyrazol-1-yl)piperidin-1-yl)methyl)phenyl)piperidine-2,6-dione;
(R)-1-(4-((4-(4-(6-amino-5-(1-(2,6-dichloro-3-fluorophenyl)ethoxy)pyridin-3-yl)-1H-pyrazol-1-yl)piperidin-1-yl)methyl)phenyl)dihydropyrimidine-2,4(1H,3H)-dione;
(R)-1-(4-((4-(4-(6-amino-5-(1-(2,6-dichloro-3-fluorophenyl)ethoxy)pyridin-3-yl)-1H-pyrazol-1-yl)piperidin-1-yl)methyl)-3-fluorophenyl)dihydropyrimidine-2,4(1H,3H)-dione;
(R)-1-(3-((4-(4-(6-amino-5-(1-(2,6-dichloro-3-fluorophenyl)ethoxy)pyridin-3-yl)-1H-pyrazol-1-yl)piperidin-1-yl)methyl)phenyl)dihydropyrimidine-2,4(1H,3H)-dione;
(R)-1-(2-((4-(4-(6-amino-5-(1-(2,6-dichloro-3-fluorophenyl)ethoxy)pyridin-3-yl)-1H-pyrazol-1-yl)piperidin-1-yl)methyl)phenyl)dihydropyrimidine-2,4(1H,3H)-dione;
(R)-1-(6-((4-(4-(6-amino-5-(1-(2,6-dichloro-3-fluorophenyl)ethoxy)pyridin-3-yl)-1H-pyrazol-1-yl)piperidin-1-yl)methyl)pyridin-3-yl)dihydropyrimidine-2,4(1H,3H)-dione;
(R)-1-(6-((4-(4-(6-amino-5-(1-(2,6-dichloro-3-fluorophenyl)ethoxy)pyridin-3-yl)-1H-pyrazol-1-yl)piperidin-1-yl)methyl)pyridazin-3-yl)dihydropyrimidine-2,4(1H,3H)-dione;
(R)-1-(4-((4-(4-(6-amino-5-(1-(2,6-dichloro-3-fluorophenyl)ethoxy)pyridin-3-yl)-1H-pyrazol-1-yl)piperidin-1-yl)methyl)pyridin-3-yl)dihydropyrimidine-2,4(1H,3H)-dione;
3-((4-((4-(4-(6-amino-5-((R)-1-(2,6-dichloro-3-fluorophenyl)ethoxy)pyridin-3-yl)-1H-pyrazol-1-yl)piperidin-1-yl)methyl)phenyl)amino)piperidine-2,6-dione;
3-((3-((4-(4-(6-amino-5-((R)-1-(2,6-dichloro-3-fluorophenyl)ethoxy)pyridin-3-yl)-1H-pyrazol-1-yl)piperidin-1-yl)methyl)phenyl)amino)piperidine-2,6-dione;
3-((2-((4-(4-(6-amino-5-((R)-1-(2,6-dichloro-3-fluorophenyl)ethoxy)pyridin-3-yl)-1H-pyrazol-1-yl)piperidin-1-yl)methyl)phenyl)amino)piperidine-2,6-dione;
3-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)piperidine-2,6-dione;
3-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)-2-fluorophenyl)piperidine-2,6-dione;
3-(3-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)piperidine-2,6-dione;
3-(2-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)piperidine-2,6-dione;
3-(6-((4-(4-((5-chloro-4-((2-(isopropylsulfonyl)phenyl)amino)pyrimidin-2-yl)amino)-5-isopropoxy-2-methylphenyl)piperidin-1-yl)methyl)pyridin-3-yl)piperidine-2,6-dione;
3-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)pyridin-3-yl)piperidine-2,6-dione;
1-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)dihydropyrimidine-2,4(1H,3H)-dione;
1-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)-3-fluorophenyl)dihydropyrimidine-2,4(1H,3H)-dione;
1-(3-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)dihydropyrimidine-2,4(1H,3H)-dione;
1-(2-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)dihydropyrimidine-2,4(1H,3H)-dione;
1-(6-((4-(4-((5-chloro-4-((2-(isopropylsulfonyl)phenyl)amino)pyrimidin-2-yl)amino)-5-isopropoxy-2-methylphenyl)piperidin-1-yl)methyl)pyridin-3-yl)dihydropyrimidine-2,4(1H,3H)-dione;
1-(6-((4-(4-((5-chloro-4-((2-(isopropylsulfonyl)phenyl)amino)pyrimidin-2-yl)amino)-5-isopropoxy-2-methylphenyl)piperidin-1-yl)methyl)-5-fluoropyridin-3-yl)dihydropyrimidine-2,4(1H,3H)-dione;
1-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)pyridin-3-yl)dihydropyrimidine-2,4(1H,3H)-dione;
1-(6-((4-(4-((5-chloro-4-((2-(isopropylsulfonyl)phenyl)amino)pyrimidin-2-yl)amino)-5-isopropoxy-2-methylphenyl)piperidin-1-yl)methyl)pyridazin-3-yl)dihydropyrimidine-2,4(1H,3H)-dione;
1-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)pyrazin-2-yl)dihydropyrimidine-2,4(1H,3H)-dione;
1-(6-((4-(4-((5-chloro-4-((2-(isopropylsulfonyl)phenyl)amino)pyrimidin-2-yl)amino)-5-isopropoxy-2-methylphenyl)piperidin-1-yl)methyl)pyrazin-2-yl)dihydropyrimidine-2,4(1H,3H)-dione;
1-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)pyridin-3-yl)dihydropyrimidine-2,4(1H,3H)-dione;
3-((4-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)amino)piperidine-2,6-dione;
3-((3-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)amino)piperidine-2,6-dione;
3-((2-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)amino)piperidine-2,6-dione;
3-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)piperidine-2,6-dione;
3-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)-2-fluorophenyl)piperidine-2,6-dione;
3-(3-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)piperidine-2,6-dione;
3-(2-((4-(4-((5-chloro-4-((2-(isopropylsulfonyl)phenyl)amino)pyrimidin-2-yl)amino)-5-isopropoxy-2-methylphenyl)piperidin-1-yl)methyl)phenyl)piperidine-2,6-dione;
3-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)pyridin-2-yl)piperidine-2,6-dione;
3-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)-5-fluoropyridin-2-yl)piperidine-2,6-dione;
3-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)-6-fluoropyridin-3-yl)piperidine-2,6-dione;
1-(4-((4-(4-((5-chloro-4-((2-(isopropylsulfonyl)phenyl)amino)pyrimidin-2-yl)amino)-5-isopropoxy-2-methylphenyl)piperazin-1-yl)methyl)phenyl)dihydropyrimidine-2,4(1H,3H)-dione;
1-(4-((4-(4-((5-chloro-4-((2-(isopropylsulfonyl)phenyl)amino)pyrimidin-2-yl)amino)-5-isopropoxy-2-methylphenyl)piperazin-1-yl)methyl)-3-fluorophenyl)dihydropyrimidine-2,4(1H,3H)-dione;
1-(3-((4-(4-((5-chloro-4-((2-(isopropylsulfonyl)phenyl)amino)pyrimidin-2-yl)amino)-5-isopropoxy-2-methylphenyl)piperazin-1-yl)methyl)phenyl)dihydropyrimidine-2,4(1H,3H)-dione;
1-(2-((4-(4-((5-chloro-4-((2-(isopropylsulfonyl)phenyl)amino)pyrimidin-2-yl)amino)-5-isopropoxy-2-methylphenyl)piperazin-1-yl)methyl)phenyl)dihydropyrimidine-2,4(1H,3H)-dione;
1-(6-((4-(4-((5-chloro-4-((2-(isopropylsulfonyl)phenyl)amino)pyrimidin-2-yl)amino)-5-isopropoxy-2-methylphenyl)piperazin-1-yl)methyl)pyridin-3-yl)dihydropyrimidine-2,4(1H,3H)-dione;
1-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)pyridin-2-yl)dihydropyrimidine-2,4(1H,3H)-dione;
1-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)-5-fluoropyridin-2-yl)dihydropyrimidine-2,4(1H,3H)-dione;
1-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)-6-fluoropyridin-3-yl)dihydropyrimidine-2,4(1H,3H)-dione;
1-(6-((4-(4-((5-chloro-4-((2-(isopropylsulfonyl)phenyl)amino)pyrimidin-2-yl)amino)-5-isopropoxy-2-methylphenyl)piperazin-1-yl)methyl)pyridazin-3-yl)dihydropyrimidine-2,4(1H,3H)-dione;
1-(4-((4-(4-((5-chloro-4-((2-(isopropylsulfonyl)phenyl)amino)pyrimidin-2-yl)amino)-5-isopropoxy-2-methylphenyl)piperazin-1-yl)methyl)pyridin-3-yl)dihydropyrimidine-2,4(1H,3H)-dione;
3-((4-((4-(4-((5-chloro-4-((2-(isopropylsulfonyl)phenyl)amino)pyrimidin-2-yl)amino)-5-isopropoxy-2-methylphenyl)piperazin-1-yl)methyl)phenyl)amino)piperidine-2,6-dione;
3-((3-((4-(4-((5-chloro-4-((2-(isopropylsulfonyl)phenyl)amino)pyrimidin-2-yl)amino)-5-isopropoxy-2-methylphenyl)piperazin-1-yl)methyl)phenyl)amino)piperidine-2,6-dione;
3-((2-((4-(4-((5-chloro-4-((2-(isopropylsulfonyl)phenyl)amino)pyrimidin-2-yl)amino)-5-isopropoxy-2-methylphenyl)piperazin-1-yl)methyl)phenyl)amino)piperidine-2,6-dione;
3-(4-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)phenyl)piperidine-2,6-dione;
3-(4-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)-2-fluorophenyl)piperidine-2,6-dione;
3-(3-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)phenyl)piperidine-2,6-dione;
3-(2-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)phenyl)piperidine-2,6-dione;
1-(4-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)phenyl)dihydropyrimidine-2,4(1H,3H)-dione;
1-(4-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)-3-fluorophenyl)dihydropyrimidine-2,4(1H,3H)-dione;
1-(3-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)phenyl)dihydropyrimidine-2,4(1H,3H)-dione;
1-(2-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)phenyl)dihydropyrimidine-2,4(1H,3H)-dione;
1-(6-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)pyridin-3-yl)dihydropyrimidine-2,4(1H,3H)-dione;
1-(6-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)pyridazin-3-yl)dihydropyrimidine-2,4(1H,3H)-dione;
1-(4-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)pyridin-3-yl)dihydropyrimidine-2,4(1H,3H)-dione;
3-((4-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)phenyl)amino)piperidine-2,6-dione;
3-((3-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)phenyl)amino)piperidine-2,6-dione;
3-((2-(((1-(4-((5-chloro-4-((2-(isopropylsulfonyl)phenyl)amino)pyrimidin-2-yl)amino)-5-isopropoxy-2-methylphenyl)piperidin-4-yl)(methyl)amino)methyl)phenyl)amino)piperidine-2,6-dione;
3-(4-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)phenyl)piperidine-2,6-dione;
3-(4-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)-2-fluorophenyl)piperidine-2,6-dione;
3-(3-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)phenyl)piperidine-2,6-dione;
3-(2-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)phenyl)piperidine-2,6-dione;
1-(4-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)phenyl)dihydropyrimidine-2,4(1H,3H)-dione;
1-(4-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)-3-fluorophenyl)dihydropyrimidine-2,4(1H,3H)-dione;
1-(3-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)phenyl)dihydropyrimidine-2,4(1H,3H)-dione;
1-(2-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)phenyl)dihydropyrimidine-2,4(1H,3H)-dione;
3-((4-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)phenyl)amino)piperidine-2,6-dione;
3-((3-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)phenyl)amino)piperidine-2,6-dione;
3-((2-((4-(1-(4-((5-chloro-4-((2-(isopropylsulfonyl)phenyl)amino)pyrimidin-2-yl)amino)-5-isopropoxy-2-methylphenyl)piperidin-4-yl)piperazin-1-yl)methyl)phenyl)amino)piperidine-2,6-dione;
8-(4-(4-(4-(2,6-dioxopiperidin-3-yl)benzyl)piperazin-1-yl)piperidin-1-yl)-9-ethyl-6,6-dimethyl-11-oxo-6,11-dihydro-5H-benzo[b]carbazole-3-carbonitrile;
8-(4-(4-(4-(2,6-dioxopiperidin-3-yl)-3-fluorobenzyl)piperazin-1-yl)piperidin-1-yl)-9-ethyl-6,6-dimethyl-11-oxo-6,11-dihydro-5H-benzo[b]carbazole-3-carbonitrile;
8-(4-(4-(3-(2,6-dioxopiperidin-3-yl)benzyl)piperazin-1-yl)piperidin-1-yl)-9-ethyl-6,6-dimethyl-11-oxo-6,11-dihydro-5H-benzo[b]carbazole-3-carbonitrile;
8-(4-(4-(2-(2,6-dioxopiperidin-3-yl)benzyl)piperazin-1-yl)piperidin-1-yl)-9-ethyl-6,6-dimethyl-11-oxo-6,11-dihydro-5H-benzo[b]carbazole-3-carbonitrile;
8-(4-(4-((2-(2,6-dioxopiperidin-3-yl)pyridin-4-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((2-(2,6-dioxopiperidin-3-yl)-5-fluoropyridin-4-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((5-(2,6-dioxopiperidin-3-yl)pyridin-2-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((5-(2,6-dioxopiperidin-3-yl)pyridin-3-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((5-(2,6-dioxopiperidin-3-yl)-2-fluoropyridin-3-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-(4-(2,4-dioxotetrahydropyrimidin-1(2H)-yl)benzyl)piperazin-1-yl)piperidin-1-yl)-9-ethyl-6,6-dimethyl-11-oxo-6,11-dihydro-5H-benzo[b]carbazole-3-carbonitrile;
8-(4-(4-(4-(2,4-dioxotetrahydropyrimidin-1(2H)-yl)-2-fluorobenzyl)piperazin-1-yl)piperidin-1-yl)-9-ethyl-6,6-dimethyl-11-oxo-6,11-dihydro-5H-benzo[b]carbazole-3-carbonitrile;
8-(4-(4-(4-(2,4-dioxotetrahydropyrimidin-1(2H)-yl)-3-fluorobenzyl)piperazin-1-yl)piperidin-1-yl)-9-ethyl-6,6-dimethyl-11-oxo-6,11-dihydro-5H-benzo[b]carbazole-3-carbonitrile;
8-(4-(4-(3-(2,4-dioxotetrahydropyrimidin-1(2H)-yl)benzyl)piperazin-1-yl)piperidin-1-yl)-9-ethyl-6,6-dimethyl-11-oxo-6,11-dihydro-5H-benzo[b]carbazole-3-carbonitrile;
8-(4-(4-(2-(2,4-dioxotetrahydropyrimidin-1(2H)-yl)benzyl)piperazin-1-yl)piperidin-1-yl)-9-ethyl-6,6-dimethyl-11-oxo-6,11-dihydro-5H-benzo[b]carbazole-3-carbonitrile;
8-(4-(4-((5-(2,4-dioxotetrahydropyrimidin-1(2H)-yl)pyridin-2-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((5-(2,4-dioxotetrahydropyrimidin-1(2H)-yl)-3-fluoropyridin-2-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((2-(2,4-dioxotetrahydropyrimidin-1(2H)-yl)pyridin-4-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((2-(2,4-dioxotetrahydropyrimidin-1(2H)-yl)-5-fluoropyridin-4-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((6-(2,4-dioxotetrahydropyrimidin-1(2H)-yl)pyridin-3-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((5-(2,4-dioxotetrahydropyrimidin-1(2H)-yl)pyridin-3-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((5-(2,4-dioxotetrahydropyrimidin-1(2H)-yl)-2-fluoropyridin-3-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((6-(2,4-dioxotetrahydropyrimidin-1(2H)-yl)pyridazin-3-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((5-(2,4-dioxotetrahydropyrimidin-1(2H)-yl)pyrazin-2-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((6-(2,4-dioxotetrahydropyrimidin-1(2H)-yl)pyrazin-2-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((3-(2,4-dioxotetrahydropyrimidin-1(2H)-yl)pyridin-4-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-(4-((2,6-dioxopiperidin-3-yl)amino)benzyl)piperazin-1-yl)piperidin-1-yl)-9-ethyl-6,6-dimethyl-11-oxo-6,11-dihydro-5H-benzo[b]carbazole-3-carbonitrile;
8-(4-(4-(3-((2,6-dioxopiperidin-3-yl)amino)benzyl)piperazin-1-yl)piperidin-1-yl)-9-ethyl-6,6-dimethyl-11-oxo-6,11-dihydro-5H-benzo[b]carbazole-3-carbonitrile;
8-(4-(4-(2-((2,6-dioxopiperidin-3-yl)amino)benzyl)piperazin-1-yl)piperidin-1-yl)-9-ethyl-6,6-dimethyl-11-oxo-6,11-dihydro-5H-benzo[b]carbazole-3-carbonitrile;
8-(4-(4-(2,6-dioxopiperidin-3-yl)benzyl)piperazin-1-yl)-9-ethyl-6,6-dimethyl-11-oxo-6,11-dihydro-5H-benzo[b]carbazole-3-carbonitrile;
8-(4-(4-(2,6-dioxopiperidin-3-yl)-3-fluorobenzyl)piperazin-1-yl)-9-ethyl-6,6-dimethyl-11-oxo-6,11-dihydro-5H-benzo[b]carbazole-3-carbonitrile;
8-(4-(3-(2,6-dioxopiperidin-3-yl)benzyl)piperazin-1-yl)-9-ethyl-6,6-dimethyl-11-oxo-6,11-dihydro-5H-benzo[b]carbazole-3-carbonitrile;
8-(4-(2-(2,6-dioxopiperidin-3-yl)benzyl)piperazin-1-yl)-9-ethyl-6,6-dimethyl-11-oxo-6,11-dihydro-5H-benzo[b]carbazole-3-carbonitrile;
8-(4-((5-(2,6-dioxopiperidin-3-yl)-2-fluoropyridin-3-yl)methyl)piperazin-1-yl)-9-ethyl-6,6-dimethyl-11-oxo-6,11-dihydro-5H-benzo[b]carbazole-3-carbonitrile;
8-(4-(4-(2,4-dioxotetrahydropyrimidin-1(2H)-yl)benzyl)piperazin-1-yl)-9-ethyl-6,6-dimethyl-11-oxo-6,11-dihydro-5H-benzo[b]carbazole-3-carbonitrile;
8-(4-(4-(2,4-dioxotetrahydropyrimidin-1(2H)-yl)-2-fluorobenzyl)piperazin-1-yl)-9-ethyl-6,6-dimethyl-11-oxo-6,11-dihydro-5H-benzo[b]carbazole-3-carbonitrile;
8-(4-(3-(2,4-dioxotetrahydropyrimidin-1(2H)-yl)benzyl)piperazin-1-yl)-9-ethyl-6,6-dimethyl-11-oxo-6,11-dihydro-5H-benzo[b]carbazole-3-carbonitrile;
8-(4-(2-(2,4-dioxotetrahydropyrimidin-1(2H)-yl)benzyl)piperazin-1-yl)-9-ethyl-6,6-dimethyl-11-oxo-6,11-dihydro-5H-benzo[b]carbazole-3-carbonitrile;
8-(4-((5-(2,4-dioxotetrahydropyrimidin-1(2H)-yl)pyridin-2-yl)methyl)piperazin-1-yl)-9-ethyl-6,6-dimethyl-11-oxo-6,11-dihydro-5H-benzo[b]carbazole-3-carbonitrile;
8-(4-((2-(2,4-dioxotetrahydropyrimidin-1(2H)-yl)pyridin-4-yl)methyl)piperazin-1-yl)-9-ethyl-6,6-dimethyl-11-oxo-6,11-dihydro-5H-benzo[b]carbazole-3-carbonitrile;
8-(4-((2-(2,4-dioxotetrahydropyrimidin-1(2H)-yl)-5-fluoropyridin-4-yl)methyl)piperazin-1-yl)-9-ethyl-6,6-dimethyl-11-oxo-6,11-dihydro-5H-benzo[b]carbazole-3-carbonitrile;
8-(4-((6-(2,4-dioxotetrahydropyrimidin-1(2H)-yl)pyridin-3-yl)methyl)piperazin-1-yl)-9-ethyl-6,6-dimethyl-11-oxo-6,11-dihydro-5H-benzo[b]carbazole-3-carbonitrile;
8-(4-((5-(2,4-dioxotetrahydropyrimidin-1(2H)-yl)pyridin-3-yl)methyl)piperazin-1-yl)-9-ethyl-6,6-dimethyl-11-oxo-6,11-dihydro-5H-benzo[b]carbazole-3-carbonitrile;
8-(4-((6-(2,4-dioxotetrahydropyrimidin-1(2H)-yl)pyridazin-3-yl)methyl)piperazin-1-yl)-9-ethyl-6,6-dimethyl-11-oxo-6,11-dihydro-5H-benzo[b]carbazole-3-carbonitrile;
8-(4-((3-(2,4-dioxotetrahydropyrimidin-1(2H)-yl)pyridin-4-yl)methyl)piperazin-1-yl)-9-ethyl-6,6-dimethyl-11-oxo-6,11-dihydro-5H-benzo[b]carbazole-3-carbonitrile;
8-(4-(4-((2,6-dioxopiperidin-3-yl)amino)benzyl)piperazin-1-yl)-9-ethyl-6,6-dimethyl-11-oxo-6,11-dihydro-5H-benzo[b]carbazole-3-carbonitrile;
8-(4-(3-((2,6-dioxopiperidin-3-yl)amino)benzyl)piperazin-1-yl)-9-ethyl-6,6-dimethyl-11-oxo-6,11-dihydro-5H-benzo[b]carbazole-3-carbonitrile;
8-(4-(2-((2,6-dioxopiperidin-3-yl)amino)benzyl)piperazin-1-yl)-9-ethyl-6,6-dimethyl-11-oxo-6,11-dihydro-5H-benzo[b]carbazole-3-carbonitrile;
8-(4-((4-(2,6-dioxopiperidin-3-yl)benzyl)(methyl)amino)piperidin-1-yl)-9-ethyl-6,6-dimethyl-11-oxo-6,11-dihydro-5H-benzo[b]carbazole-3-carbonitrile;
8-(4-((4-(2,6-dioxopiperidin-3-yl)-3-fluorobenzyl)(methyl)amino)piperidin-1-yl)-9-ethyl-6,6-dimethyl-11-oxo-6,11-dihydro-5H-benzo[b]carbazole-3-carbonitrile;
8-(4-((3-(2,6-dioxopiperidin-3-yl)benzyl)(methyl)amino)piperidin-1-yl)-9-ethyl-6,6-dimethyl-11-oxo-6,11-dihydro-5H-benzo[b]carbazole-3-carbonitrile;
8-(4-((2-(2,6-dioxopiperidin-3-yl)benzyl)(methyl)amino)piperidin-1-yl)-9-ethyl-6,6-dimethyl-11-oxo-6,11-dihydro-5H-benzo[b]carbazole-3-carbonitrile;
8-(4-((4-(2,4-dioxotetrahydropyrimidin-1(2H)-yl)benzyl)(methyl)amino)piperidin-1-yl)-9-ethyl-6,6-dimethyl-11-oxo-6,11-dihydro-5H-benzo[b]carbazole-3-carbonitrile;
8-(4-((4-(2,4-dioxotetrahydropyrimidin-1(2H)-yl)-2-fluorobenzyl)(methyl)amino)piperidin-1-yl)-9-ethyl-6,6-dimethyl-11-oxo-6,11-dihydro-5H-benzo[b]carbazole-3-carbonitrile;
8-(4-((3-(2,4-dioxotetrahydropyrimidin-1(2H)-yl)benzyl)(methyl)amino)piperidin-1-yl)-9-ethyl-6,6-dimethyl-11-oxo-6,11-dihydro-5H-benzo[b]carbazole-3-carbonitrile;
8-(4-(((3-(2,6-dioxopiperidin-3-yl)pyridin-4-yl)methyl)(methyl)amino)piperidin-1-yl)-9-ethyl-6,6-dimethyl-11-oxo-6,11-dihydro-5H-benzo[b]carbazole-3-carbonitrile;
3-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)phenyl)piperidine-2,6-dione;
3-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-2-fluorophenyl)piperidine-2,6-dione;
3-(3-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)phenyl)piperidine-2,6-dione;
3-(2-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)phenyl)piperidine-2,6-dione;
3-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-2-yl)piperidine-2,6-dione;
3-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-5-fluoropyridin-2-yl)piperidine-2,6-dione;
3-(6-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-3-yl)piperidine-2,6-dione;
3-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-3-yl)piperidine-2,6-dione;
3-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-6-fluoropyridin-3-yl)piperidine-2,6-dione;
1-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)phenyl)dihydropyrimidine-2,4(1H,3H)-dione;
1-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-3-fluorophenyl)dihydropyrimidine-2,4(1H,3H)-dione;
1-(3-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)phenyl)dihydropyrimidine-2,4(1H,3H)-dione;
1-(2-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)phenyl)dihydropyrimidine-2,4(1H,3H)-dione;
1-(6-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-3-yl)dihydropyrimidine-2,4(1H,3H)-dione;
1-(6-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-5-fluoropyridin-3-yl)dihydropyrimidine-2,4(1H,3H)-dione;
1-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-3-yl)dihydropyrimidine-2,4(1H,3H)-dione;
1-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-6-fluoropyridin-3-yl)dihydropyrimidine-2,4(1H,3H)-dione;
1-(6-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridazin-3-yl)dihydropyrimidine-2,4(1H,3H)-dione;
1-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyrazin-2-yl)dihydropyrimidine-2,4(1H,3H)-dione;
1-(6-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyrazin-2-yl)dihydropyrimidine-2,4(1H,3H)-dione;
1-(4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)pyridin-3-yl)dihydropyrimidine-2,4(1H,3H)-dione;
3-((4-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)phenyl)amino)piperidine-2,6-dione;
3-((3-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)phenyl)amino)piperidine-2,6-dione;
3-((2-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)phenyl)amino)piperidine-2,6-dione;
2-((S)-4-(4-chlorophenyl)-2,3,9-trimethyl-6H-thieno[3,2-f][1,2,4]triazolo[4,3-a][1,4]diazepin-6-yl)-N-(4-(2,6-dioxopiperidin-3-yl)benzyl)acetamide;
2-((S)-4-(4-chlorophenyl)-2,3,9-trimethyl-6H-thieno[3,2-f][1,2,4]triazolo[4,3-a][1,4]diazepin-6-yl)-N-(4-(2,6-dioxopiperidin-3-yl)-3-fluorobenzyl)acetamide;
2-((S)-4-(4-chlorophenyl)-2,3,9-trimethyl-6H-thieno[3,2-f][1,2,4]triazolo[4,3-a][1,4]diazepin-6-yl)-N-(3-(2,6-dioxopiperidin-3-yl)benzyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(2,6-dioxopiperidin-3-yl)benzyl)acetamide;
(S)-2-(4-(4-chlorophenyl)-2,3,9-trimethyl-6H-thieno[3,2-f][1,2,4]triazolo[4,3-a][1,4]diazepin-6-yl)-N-(4-(2,4-dioxotetrahydropyrimidin-1(2H)-yl)benzyl)acetamide;
(S)-2-(4-(4-chlorophenyl)-2,3,9-trimethyl-6H-thieno[3,2-f][1,2,4]triazolo[4,3-a][1,4]diazepin-6-yl)-N-(4-(2,4-dioxotetrahydropyrimidin-1(2H)-yl)-2-fluorobenzyl)acetamide;
(S)-2-(4-(4-chlorophenyl)-2,3,9-trimethyl-6H-thieno[3,2-f][1,2,4]triazolo[4,3-a][1,4]diazepin-6-yl)-N-(3-(2,4-dioxotetrahydropyrimidin-1(2H)-yl)benzyl)acetamide;
(S)-2-(4-(4-chlorophenyl)-2,3,9-trimethyl-6H-thieno[3,2-f][1,2,4]triazolo[4,3-a][1,4]diazepin-6-yl)-N-(2-(2,4-dioxotetrahydropyrimidin-1(2H)-yl)benzyl)acetamide;
(S)-2-(4-(4-chlorophenyl)-2,3,9-trimethyl-6H-thieno[3,2-f][1,2,4]triazolo[4,3-a][1,4]diazepin-6-yl)-N-((5-(2,4-dioxotetrahydropyrimidin-1(2H)-yl)pyridin-2-yl)methyl)acetamide;
(S)-2-(4-(4-chlorophenyl)-2,3,9-trimethyl-6H-thieno[3,2-f][1,2,4]triazolo[4,3-a][1,4]diazepin-6-yl)-N-((6-(2,4-dioxotetrahydropyrimidin-1(2H)-yl)pyridazin-3-yl)methyl)acetamide;
(S)-2-(4-(4-chlorophenyl)-2,3,9-trimethyl-6H-thieno[3,2-f][1,2,4]triazolo[4,3-a][1,4]diazepin-6-yl)-N-((3-(2,4-dioxotetrahydropyrimidin-1(2H)-yl)pyridin-4-yl)methyl)acetamide;
2-((S)-4-(4-chlorophenyl)-2,3,9-trimethyl-6H-thieno[3,2-f][1,2,4]triazolo[4,3-a][1,4]diazepin-6-yl)-N-(3-((2,6-dioxopiperidin-3-yl)amino)benzyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-((2,6-dioxopiperidin-3-yl)amino)benzyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(4-(4-(2,6-dioxopiperidin-3-yl)benzyl)piperazin-1-yl)ethyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(4-(4-(2,6-dioxopiperidin-3-yl)-3-fluorobenzyl)piperazin-1-yl)ethyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(4-(3-(2,6-dioxopiperidin-3-yl)benzyl)piperazin-1-yl)ethyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(4-(2-(2,6-dioxopiperidin-3-yl)benzyl)piperazin-1-yl)ethyl)acetamide;
(S)-2-(4-(4-chlorophenyl)-2,3,9-trimethyl-6H-thieno[3,2-f][1,2,4]triazolo[4,3-a][1,4]diazepin-6-yl)-N-(2-(4-(4-(2,4-dioxotetrahydropyrimidin-1(2H)-yl)benzyl)piperazin-1-yl)ethyl)acetamide;
(S)-2-(4-(4-chlorophenyl)-2,3,9-trimethyl-6H-thieno[3,2-f][1,2,4]triazolo[4,3-a][1,4]diazepin-6-yl)-N-(2-(4-(4-(2,4-dioxotetrahydropyrimidin-1(2H)-yl)-2-fluorobenzyl)piperazin-1-yl)ethyl)acetamide;
(S)-2-(4-(4-chlorophenyl)-2,3,9-trimethyl-6H-thieno[3,2-f][1,2,4]triazolo[4,3-a][1,4]diazepin-6-yl)-N-(2-(4-(3-(2,4-dioxotetrahydropyrimidin-1(2H)-yl)benzyl)piperazin-1-yl)ethyl)acetamide;
(S)-2-(4-(4-chlorophenyl)-2,3,9-trimethyl-6H-thieno[3,2-f][1,2,4]triazolo[4,3-a][1,4]diazepin-6-yl)-N-(2-(4-(2-(2,4-dioxotetrahydropyrimidin-1(2H)-yl)benzyl)piperazin-1-yl)ethyl)acetamide;
(S)-2-(4-(4-chlorophenyl)-2,3,9-trimethyl-6H-thieno[3,2-f][1,2,4]triazolo[4,3-a][1,4]diazepin-6-yl)-N-(2-(4-((5-(2,4-dioxotetrahydropyrimidin-1(2H)-yl)pyridin-2-yl)methyl)piperazin-1-yl)ethyl)acetamide;
(S)-2-(4-(4-chlorophenyl)-2,3,9-trimethyl-6H-thieno[3,2-f][1,2,4]triazolo[4,3-a][1,4]diazepin-6-yl)-N-(2-(4-((6-(2,4-dioxotetrahydropyrimidin-1(2H)-yl)pyridazin-3-yl)methyl)piperazin-1-yl)ethyl)acetamide;
(S)-2-(4-(4-chlorophenyl)-2,3,9-trimethyl-6H-thieno[3,2-f][1,2,4]triazolo[4,3-a][1,4]diazepin-6-yl)-N-(2-(4-((3-(2,4-dioxotetrahydropyrimidin-1(2H)-yl)pyridin-4-yl)methyl)piperazin-1-yl)ethyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(4-(4-((2,6-dioxopiperidin-3-yl)amino)benzyl)piperazin-1-yl)ethyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(4-(3-((2,6-dioxopiperidin-3-yl)amino)benzyl)piperazin-1-yl)ethyl)acetamide;
2-((S)-4-(4-chlorophenyl)-2,3,9-trimethyl-6H-thieno[3,2-f][1,2,4]triazolo[4,3-a][1,4]diazepin-6-yl)-N-(2-(4-(2-((2,6-dioxopiperidin-3-yl)amino)benzyl)piperazin-1-yl)ethyl)acetamide;
3-(4-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)phenyl)piperidine-2,6-dione;
3-(4-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)-2-fluorophenyl)piperidine-2,6-dione;
3-(3-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)phenyl)piperidine-2,6-dione;
3-(2-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)phenyl)piperidine-2,6-dione;
(S)-1-(4-((4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)methyl)phenyl)dihydropyrimidine-2,4(1H,3H)-dione;
(S)-1-(4-((4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)methyl)-3-fluorophenyl)dihydropyrimidine-2,4(1H,3H)-dione;
(S)-1-(3-((4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)methyl)phenyl)dihydropyrimidine-2,4(1H,3H)-dione;
(S)-1-(2-((4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)methyl)phenyl)dihydropyrimidine-2,4(1H,3H)-dione;
(S)-1-(6-((4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)methyl)pyridin-3-yl)dihydropyrimidine-2,4(1H,3H)-dione;
(S)-1-(6-((4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)methyl)pyridazin-3-yl)dihydropyrimidine-2,4(1H,3H)-dione;
(S)-1-(4-((4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)methyl)pyridin-3-yl)dihydropyrimidine-2,4(1H,3H)-dione;
3-((4-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)phenyl)amino)piperidine-2,6-dione;
3-((3-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)phenyl)amino)piperidine-2,6-dione;
3-((2-((4-(2-((S)-4-(4-chlorophenyl)-2,3,9-trimethyl-6H-thieno[3,2-f][1,2,4]triazolo[4,3-a][1,4]diazepin-6-yl)acetyl)piperazin-1-yl)methyl)phenyl)amino)piperidine-2,6-dione;
3-(4-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)phenyl)piperidine-2,6-dione;
3-(4-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)-2-fluorophenyl)piperidine-2,6-dione;
3-(3-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)phenyl)piperidine-2,6-dione;
3-(2-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)phenyl)piperidine-2,6-dione;
(S)-1-(4-((4-(2-(4-(4-chlorophenyl)-2,3,9-trimethyl-6H-thieno[3,2-f][1,2,4]triazolo[4,3-a][1,4]diazepin-6-yl)ethyl)piperazin-1-yl)methyl)phenyl)dihydropyrimidine-2,4(1H,3H)-dione;
(S)-1-(4-((4-(2-(4-(4-chlorophenyl)-2,3,9-trimethyl-6H-thieno[3,2-f][1,2,4]triazolo[4,3-a][1,4]diazepin-6-yl)ethyl)piperazin-1-yl)methyl)-3-fluorophenyl)dihydropyrimidine-2,4(1H,3H)-dione;
(S)-1-(3-((4-(2-(4-(4-chlorophenyl)-2,3,9-trimethyl-6H-thieno[3,2-f][1,2,4]triazolo[4,3-a][1,4]diazepin-6-yl)ethyl)piperazin-1-yl)methyl)phenyl)dihydropyrimidine-2,4(1H,3H)-dione;
(S)-1-(2-((4-(2-(4-(4-chlorophenyl)-2,3,9-trimethyl-6H-thieno[3,2-f][1,2,4]triazolo[4,3-a][1,4]diazepin-6-yl)ethyl)piperazin-1-yl)methyl)phenyl)dihydropyrimidine-2,4(1H,3H)-dione;
(S)-1-(6-((4-(2-(4-(4-chlorophenyl)-2,3,9-trimethyl-6H-thieno[3,2-f] [1,2,4]triazolo[4,3-a] [1,4]diazepin-6-yl)ethyl)piperazin-1-yl)methyl)pyridin-3-yl)dihydropyrimidine-2,4(1H,3H)-dione;
(S)-1-(6-((4-(2-(4-(4-chlorophenyl)-2,3,9-trimethyl-6H-thieno[3,2-f][1,2,4]triazolo[4,3-a][1,4]diazepin-6-yl)ethyl)piperazin-1-yl)methyl)pyridazin-3-yl)dihydropyrimidine-2,4(1H,3H)-dione;
(S)-1-(4-((4-(2-(4-(4-chlorophenyl)-2,3,9-trimethyl-6H-thieno[3,2-f][1,2,4]triazolo[4,3-a][1,4]diazepin-6-yl)ethyl)piperazin-1-yl)methyl)pyridin-3-yl)dihydropyrimidine-2,4(1H,3H)-dione;
3-((4-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)phenyl)amino)piperidine-2,6-dione;
3-((3-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)phenyl)amino)piperidine-2,6-dione;
3-((2-((4-(2-((S)-4-(4-chlorophenyl)-2,3,9-trimethyl-6H-thieno[3,2-f][1,2,4]triazolo[4,3-a][1,4]diazepin-6-yl)ethyl)piperazin-1-yl)methyl)phenyl)amino)piperidine-2,6-dione;
3-(4-((4-(4-(5,7-dimethoxy-4-oxo-3,4-dihydroquinazolin-2-yl)phenyl)piperazin-1-yl)methyl)phenyl)piperidine-2,6-dione;
3-(4-((4-(4-(5,7-dimethoxy-4-oxo-3,4-dihydroquinazolin-2-yl)phenyl)piperazin-1-yl)methyl)-2-fluorophenyl)piperidine-2,6-dione;
3-(3-((4-(4-(5,7-dimethoxy-4-oxo-3,4-dihydroquinazolin-2-yl)phenyl)piperazin-1-yl)methyl)phenyl)piperidine-2,6-dione;
3-(2-((4-(4-(5,7-dimethoxy-4-oxo-3,4-dihydroquinazolin-2-yl)phenyl)piperazin-1-yl)methyl)phenyl)piperidine-2,6-dione;
1-(4-((4-(4-(5,7-dimethoxy-4-oxo-3,4-dihydroquinazolin-2-yl)phenyl)piperazin-1-yl)methyl)phenyl)dihydropyrimidine-2,4(1H,3H)-dione;
1-(4-((4-(4-(5,7-dimethoxy-4-oxo-3,4-dihydroquinazolin-2-yl)phenyl)piperazin-1-yl)methyl)-3-fluorophenyl)dihydropyrimidine-2,4(1H,3H)-dione;
1-(3-((4-(4-(5,7-dimethoxy-4-oxo-3,4-dihydroquinazolin-2-yl)phenyl)piperazin-1-yl)methyl)phenyl)dihydropyrimidine-2,4(1H,3H)-dione;
1-(2-((4-(4-(5,7-dimethoxy-4-oxo-3,4-dihydroquinazolin-2-yl)phenyl)piperazin-1-yl)methyl)phenyl)dihydropyrimidine-2,4(1H,3H)-dione;
1-(6-((4-(4-(5,7-dimethoxy-4-oxo-3,4-dihydroquinazolin-2-yl)phenyl)piperazin-1-yl)methyl)pyridin-3-yl)dihydropyrimidine-2,4(1H,3H)-dione;
1-(6-((4-(4-(5,7-dimethoxy-4-oxo-3,4-dihydroquinazolin-2-yl)phenyl)piperazin-1-yl)methyl)pyridazin-3-yl)dihydropyrimidine-2,4(1H,3H)-dione;
1-(4-((4-(4-(5,7-dimethoxy-4-oxo-3,4-dihydroquinazolin-2-yl)phenyl)piperazin-1-yl)methyl)pyridin-3-yl)dihydropyrimidine-2,4(1H,3H)-dione;
3-((4-((4-(4-(5,7-dimethoxy-4-oxo-3,4-dihydroquinazolin-2-yl)phenyl)piperazin-1-yl)methyl)phenyl)amino)piperidine-2,6-dione;
3-((3-((4-(4-(5,7-dimethoxy-4-oxo-3,4-dihydroquinazolin-2-yl)phenyl)piperazin-1-yl)methyl)phenyl)amino)piperidine-2,6-dione;
3-((2-((4-(4-(5,7-dimethoxy-4-oxo-3,4-dihydroquinazolin-2-yl)phenyl)piperazin-1-yl)methyl)phenyl)amino)piperidine-2,6-dione;
3-(4-(((1-(4-(5,7-dimethoxy-4-oxo-3,4-dihydroquinazolin-2-yl)phenyl)piperidin-4-yl)(methyl)amino)methyl)phenyl)piperidine-2,6-dione;
3-(4-(((1-(4-(5,7-dimethoxy-4-oxo-3,4-dihydroquinazolin-2-yl)phenyl)piperidin-4-yl)(methyl)amino)methyl)-2-fluorophenyl)piperidine-2,6-dione;
3-(3-(((1-(4-(5,7-dimethoxy-4-oxo-3,4-dihydroquinazolin-2-yl)phenyl)piperidin-4-yl)(methyl)amino)methyl)phenyl)piperidine-2,6-dione;
3-(2-(((1-(4-(5,7-dimethoxy-4-oxo-3,4-dihydroquinazolin-2-yl)phenyl)piperidin-4-yl)(methyl)amino)methyl)phenyl)piperidine-2,6-dione;
1-(4-(((1-(4-(5,7-dimethoxy-4-oxo-3,4-dihydroquinazolin-2-yl)phenyl)piperidin-4-yl)(methyl)amino)methyl)phenyl)dihydropyrimidine-2,4(1H,3H)-dione;
1-(4-(((1-(4-(5,7-dimethoxy-4-oxo-3,4-dihydroquinazolin-2-yl)phenyl)piperidin-4-yl)(methyl)amino)methyl)-3-fluorophenyl)dihydropyrimidine-2,4(1H,3H)-dione;
1-(3-(((1-(4-(5,7-dimethoxy-4-oxo-3,4-dihydroquinazolin-2-yl)phenyl)piperidin-4-yl)(methyl)amino)methyl)phenyl)dihydropyrimidine-2,4(1H,3H)-dione;
1-(2-(((1-(4-(5,7-dimethoxy-4-oxo-3,4-dihydroquinazolin-2-yl)phenyl)piperidin-4-yl)(methyl)amino)methyl)phenyl)dihydropyrimidine-2,4(1H,3H)-dione;
1-(6-(((1-(4-(5,7-dimethoxy-4-oxo-3,4-dihydroquinazolin-2-yl)phenyl)piperidin-4-yl)(methyl)amino)methyl)pyridin-3-yl)dihydropyrimidine-2,4(1H,3H)-dione;
1-(6-(((1-(4-(5,7-dimethoxy-4-oxo-3,4-dihydroquinazolin-2-yl)phenyl)piperidin-4-yl)(methyl)amino)methyl)pyridazin-3-yl)dihydropyrimidine-2,4(1H,3H)-dione;
1-(4-(((1-(4-(5,7-dimethoxy-4-oxo-3,4-dihydroquinazolin-2-yl)phenyl)piperidin-4-yl)(methyl)amino)methyl)pyridin-3-yl)dihydropyrimidine-2,4(1H,3H)-dione;
3-((4-(((1-(4-(5,7-dimethoxy-4-oxo-3,4-dihydroquinazolin-2-yl)phenyl)piperidin-4-yl)(methyl)amino)methyl)phenyl)amino)piperidine-2,6-dione;
3-((3-(((1-(4-(5,7-dimethoxy-4-oxo-3,4-dihydroquinazolin-2-yl)phenyl)piperidin-4-yl)(methyl)amino)methyl)phenyl)amino)piperidine-2,6-dione;
3-((2-(((1-(4-(5,7-dimethoxy-4-oxo-3,4-dihydroquinazolin-2-yl)phenyl)piperidin-4-yl)(methyl)amino)methyl)phenyl)amino)piperidine-2,6-dione;
3-(4-((4-(1-(4-(5,7-dimethoxy-4-oxo-3,4-dihydroquinazolin-2-yl)phenyl)piperidin-4-yl)piperazin-1-yl)methyl)phenyl)piperidine-2,6-dione;
3-(4-((4-(1-(4-(5,7-dimethoxy-4-oxo-3,4-dihydroquinazolin-2-yl)phenyl)piperidin-4-yl)piperazin-1-yl)methyl)-2-fluorophenyl)piperidine-2,6-dione;
3-(3-((4-(1-(4-(5,7-dimethoxy-4-oxo-3,4-dihydroquinazolin-2-yl)phenyl)piperidin-4-yl)piperazin-1-yl)methyl)phenyl)piperidine-2,6-dione;
3-(2-((4-(1-(4-(5,7-dimethoxy-4-oxo-3,4-dihydroquinazolin-2-yl)phenyl)piperidin-4-yl)piperazin-1-yl)methyl)phenyl)piperidine-2,6-dione;
1-(4-((4-(1-(4-(5,7-dimethoxy-4-oxo-3,4-dihydroquinazolin-2-yl)phenyl)piperidin-4-yl)piperazin-1-yl)methyl)phenyl)dihydropyrimidine-2,4(1H,3H)-dione;
1-(4-((4-(1-(4-(5,7-dimethoxy-4-oxo-3,4-dihydroquinazolin-2-yl)phenyl)piperidin-4-yl)piperazin-1-yl)methyl)-3-fluorophenyl)dihydropyrimidine-2,4(1H,3H)-dione;
1-(3-((4-(1-(4-(5,7-dimethoxy-4-oxo-3,4-dihydroquinazolin-2-yl)phenyl)piperidin-4-yl)piperazin-1-yl)methyl)phenyl)dihydropyrimidine-2,4(1H,3H)-dione;
1-(2-((4-(1-(4-(5,7-dimethoxy-4-oxo-3,4-dihydroquinazolin-2-yl)phenyl)piperidin-4-yl)piperazin-1-yl)methyl)phenyl)dihydropyrimidine-2,4(1H,3H)-dione;
1-(6-((4-(1-(4-(5,7-dimethoxy-4-oxo-3,4-dihydroquinazolin-2-yl)phenyl)piperidin-4-yl)piperazin-1-yl)methyl)pyridin-3-yl)dihydropyrimidine-2,4(1H,3H)-dione;
1-(6-((4-(1-(4-(5,7-dimethoxy-4-oxo-3,4-dihydroquinazolin-2-yl)phenyl)piperidin-4-yl)piperazin-1-yl)methyl)pyridazin-3-yl)dihydropyrimidine-2,4(1H,3H)-dione;
1-(4-((4-(1-(4-(5,7-dimethoxy-4-oxo-3,4-dihydroquinazolin-2-yl)phenyl)piperidin-4-yl)piperazin-1-yl)methyl)pyridin-3-yl)dihydropyrimidine-2,4(1H,3H)-dione;
3-((4-((4-(1-(4-(5,7-dimethoxy-4-oxo-3,4-dihydroquinazolin-2-yl)phenyl)piperidin-4-yl)piperazin-1-yl)methyl)phenyl)amino)piperidine-2,6-dione;
3-((3-((4-(1-(4-(5,7-dimethoxy-4-oxo-3,4-dihydroquinazolin-2-yl)phenyl)piperidin-4-yl)piperazin-1-yl)methyl)phenyl)amino)piperidine-2,6-dione;
3-((2-((4-(1-(4-(5,7-dimethoxy-4-oxo-3,4-dihydroquinazolin-2-yl)phenyl)piperidin-4-yl)piperazin-1-yl)methyl)phenyl)amino)piperidine-2,6-dione;
3-(4-((4-(4-(1-acetyl-4-((4-chlorophenyl)amino)-2-methyl-1,2,3,4-tetrahydroquinolin-6-yl)phenyl)piperazin-1-yl)methyl)phenyl)piperidine-2,6-dione;
3-(4-((4-(4-(1-acetyl-4-((4-chlorophenyl)amino)-2-methyl-1,2,3,4-tetrahydroquinolin-6-yl)phenyl)piperazin-1-yl)methyl)-2-fluorophenyl)piperidine-2,6-dione;
3-(3-((4-(4-(1-acetyl-4-((4-chlorophenyl)amino)-2-methyl-1,2,3,4-tetrahydroquinolin-6-yl)phenyl)piperazin-1-yl)methyl)phenyl)piperidine-2,6-dione;
3-(2-((4-(4-(1-acetyl-4-((4-chlorophenyl)amino)-2-methyl-1,2,3,4-tetrahydroquinolin-6-yl)phenyl)piperazin-1-yl)methyl)phenyl)piperidine-2,6-dione;
1-(4-((4-(4-(1-acetyl-4-((4-chlorophenyl)amino)-2-methyl-1,2,3,4-tetrahydroquinolin-6-yl)phenyl)piperazin-1-yl)methyl)phenyl)dihydropyrimidine-2,4(1H,3H)-dione;
1-(4-((4-(4-(1-acetyl-4-((4-chlorophenyl)amino)-2-methyl-1,2,3,4-tetrahydroquinolin-6-yl)phenyl)piperazin-1-yl)methyl)-3-fluorophenyl)dihydropyrimidine-2,4(1H,3H)-dione;
1-(3-((4-(4-(1-acetyl-4-((4-chlorophenyl)amino)-2-methyl-1,2,3,4-tetrahydroquinolin-6-yl)phenyl)piperazin-1-yl)methyl)phenyl)dihydropyrimidine-2,4(1H,3H)-dione;
1-(2-((4-(4-(1-acetyl-4-((4-chlorophenyl)amino)-2-methyl-1,2,3,4-tetrahydroquinolin-6-yl)phenyl)piperazin-1-yl)methyl)phenyl)dihydropyrimidine-2,4(1H,3H)-dione;
1-(6-((4-(4-(1-acetyl-4-((4-chlorophenyl)amino)-2-methyl-1,2,3,4-tetrahydroquinolin-6-yl)phenyl)piperazin-1-yl)methyl)pyridin-3-yl)dihydropyrimidine-2,4(1H,3H)-dione;
1-(6-((4-(4-(1-acetyl-4-((4-chlorophenyl)amino)-2-methyl-1,2,3,4-tetrahydroquinolin-6-yl)phenyl)piperazin-1-yl)methyl)pyridazin-3-yl)dihydropyrimidine-2,4(1H,3H)-dione;
1-(4-((4-(4-(1-acetyl-4-((4-chlorophenyl)amino)-2-methyl-1,2,3,4-tetrahydroquinolin-6-yl)phenyl)piperazin-1-yl)methyl)pyridin-3-yl)dihydropyrimidine-2,4(1H,3H)-dione;
3-(4-(((1-(4-(1-acetyl-4-((4-chlorophenyl)amino)-2-methyl-1,2,3,4-tetrahydroquinolin-6-yl)phenyl)piperidin-4-yl)(methyl)amino)methyl)phenyl)piperidine-2,6-dione;
3-(4-(((1-(4-(1-acetyl-4-((4-chlorophenyl)amino)-2-methyl-1,2,3,4-tetrahydroquinolin-6-yl)phenyl)piperidin-4-yl)(methyl)amino)methyl)-2-fluorophenyl)piperidine-2,6-dione;
3-(3-(((1-(4-(1-acetyl-4-((4-chlorophenyl)amino)-2-methyl-1,2,3,4-tetrahydroquinolin-6-yl)phenyl)piperidin-4-yl)(methyl)amino)methyl)phenyl)piperidine-2,6-dione;
3-(2-(((1-(4-(1-acetyl-4-((4-chlorophenyl)amino)-2-methyl-1,2,3,4-tetrahydroquinolin-6-yl)phenyl)piperidin-4-yl)(methyl)amino)methyl)phenyl)piperidine-2,6-dione;
1-(4-(((1-(4-(1-acetyl-4-((4-chlorophenyl)amino)-2-methyl-1,2,3,4-tetrahydroquinolin-6-yl)phenyl)piperidin-4-yl)(methyl)amino)methyl)phenyl)dihydropyrimidine-2,4(1H,3H)-dione;
1-(4-(((1-(4-(1-acetyl-4-((4-chlorophenyl)amino)-2-methyl-1,2,3,4-tetrahydroquinolin-6-yl)phenyl)piperidin-4-yl)(methyl)amino)methyl)-3-fluorophenyl)dihydropyrimidine-2,4(1H,3H)-dione;
1-(3-(((1-(4-(1-acetyl-4-((4-chlorophenyl)amino)-2-methyl-1,2,3,4-tetrahydroquinolin-6-yl)phenyl)piperidin-4-yl)(methyl)amino)methyl)phenyl)dihydropyrimidine-2,4(1H,3H)-dione;
1-(2-(((1-(4-(1-acetyl-4-((4-chlorophenyl)amino)-2-methyl-1,2,3,4-tetrahydroquinolin-6-yl)phenyl)piperidin-4-yl)(methyl)amino)methyl)phenyl)dihydropyrimidine-2,4(1H,3H)-dione;
1-(6-(((1-(4-(1-acetyl-4-((4-chlorophenyl)amino)-2-methyl-1,2,3,4-tetrahydroquinolin-6-yl)phenyl)piperidin-4-yl)(methyl)amino)methyl)pyridin-3-yl)dihydropyrimidine-2,4(1H,3H)-dione;
1-(6-(((1-(4-(1-acetyl-4-((4-chlorophenyl)amino)-2-methyl-1,2,3,4-tetrahydroquinolin-6-yl)phenyl)piperidin-4-yl)(methyl)amino)methyl)pyridazin-3-yl)dihydropyrimidine-2,4(1H,3H)-dione;
1-(4-(((1-(4-(1-acetyl-4-((4-chlorophenyl)amino)-2-methyl-1,2,3,4-tetrahydroquinolin-6-yl)phenyl)piperidin-4-yl)(methyl)amino)methyl)pyridin-3-yl)dihydropyrimidine-2,4(1H,3H)-dione;
3-((4-(((1-(4-(1-acetyl-4-((4-chlorophenyl)amino)-2-methyl-1,2,3,4-tetrahydroquinolin-6-yl)phenyl)piperidin-4-yl)(methyl)amino)methyl)phenyl)amino)piperidine-2,6-dione;
3-((3-(((1-(4-(1-acetyl-4-((4-chlorophenyl)amino)-2-methyl-1,2,3,4-tetrahydroquinolin-6-yl)phenyl)piperidin-4-yl)(methyl)amino)methyl)phenyl)amino)piperidine-2,6-dione;
3-((2-(((1-(4-(1-acetyl-4-((4-chlorophenyl)amino)-2-methyl-1,2,3,4-tetrahydroquinolin-6-yl)phenyl)piperidin-4-yl)(methyl)amino)methyl)phenyl)amino)piperidine-2,6-dione;
3-(4-((4-(1-(4-(1-acetyl-4-((4-chlorophenyl)amino)-2-methyl-1,2,3,4-tetrahydroquinolin-6-yl)phenyl)piperidin-4-yl)piperazin-1-yl)methyl)phenyl)piperidine-2,6-dione;
3-(4-((4-(1-(4-(1-acetyl-4-((4-chlorophenyl)amino)-2-methyl-1,2,3,4-tetrahydroquinolin-6-yl)phenyl)piperidin-4-yl)piperazin-1-yl)methyl)-2-fluorophenyl)piperidine-2,6-dione;
3-(3-((4-(1-(4-(1-acetyl-4-((4-chlorophenyl)amino)-2-methyl-1,2,3,4-tetrahydroquinolin-6-yl)phenyl)piperidin-4-yl)piperazin-1-yl)methyl)phenyl)piperidine-2,6-dione;
3-(2-((4-(1-(4-(1-acetyl-4-((4-chlorophenyl)amino)-2-methyl-1,2,3,4-tetrahydroquinolin-6-yl)phenyl)piperidin-4-yl)piperazin-1-yl)methyl)phenyl)piperidine-2,6-dione;
1-(4-((4-(1-(4-(1-acetyl-4-((4-chlorophenyl)amino)-2-methyl-1,2,3,4-tetrahydroquinolin-6-yl)phenyl)piperidin-4-yl)piperazin-1-yl)methyl)phenyl)dihydropyrimidine-2,4(1H,3H)-dione;
1-(4-((4-(1-(4-(1-acetyl-4-((4-chlorophenyl)amino)-2-methyl-1,2,3,4-tetrahydroquinolin-6-yl)phenyl)piperidin-4-yl)piperazin-1-yl)methyl)-3-fluorophenyl)dihydropyrimidine-2,4(1H,3H)-dione;
1-(3-((4-(1-(4-(1-acetyl-4-((4-chlorophenyl)amino)-2-methyl-1,2,3,4-tetrahydroquinolin-6-yl)phenyl)piperidin-4-yl)piperazin-1-yl)methyl)phenyl)dihydropyrimidine-2,4(1H,3H)-dione;
1-(2-((4-(1-(4-(1-acetyl-4-((4-chlorophenyl)amino)-2-methyl-1,2,3,4-tetrahydroquinolin-6-yl)phenyl)piperidin-4-yl)piperazin-1-yl)methyl)phenyl)dihydropyrimidine-2,4(1H,3H)-dione;
1-(6-((4-(1-(4-(1-acetyl-4-((4-chlorophenyl)amino)-2-methyl-1,2,3,4-tetrahydroquinolin-6-yl)phenyl)piperidin-4-yl)piperazin-1-yl)methyl)pyridin-3-yl)dihydropyrimidine-2,4(1H,3H)-dione;
1-(6-((4-(1-(4-(1-acetyl-4-((4-chlorophenyl)amino)-2-methyl-1,2,3,4-tetrahydroquinolin-6-yl)phenyl)piperidin-4-yl)piperazin-1-yl)methyl)pyridazin-3-yl)dihydropyrimidine-2,4(1H,3H)-dione;
1-(4-((4-(1-(4-(1-acetyl-4-((4-chlorophenyl)amino)-2-methyl-1,2,3,4-tetrahydroquinolin-6-yl)phenyl)piperidin-4-yl)piperazin-1-yl)methyl)pyridin-3-yl)dihydropyrimidine-2,4(1H,3H)-dione;
3-((4-((4-(1-(4-(1-acetyl-4-((4-chlorophenyl)amino)-2-methyl-1,2,3,4-tetrahydroquinolin-6-yl)phenyl)piperidin-4-yl)piperazin-1-yl)methyl)phenylyamino)piperidine-2,6-dione;
3-((3-((4-(1-(4-(1-acetyl-4-((4-chlorophenyl)amino)-2-methyl-1,2,3,4-tetrahydroquinolin-6-yl)phenyl)piperidin-4-yl)piperazin-1-yl)methyl)phenylyamino)piperidine-2,6-dione;
3-((2-((4-(1-(4-(1-acetyl-4-((4-chlorophenyl)amino)-2-methyl-1,2,3,4-tetrahydroquinolin-6-yl)phenyl)piperidin-4-yl)piperazin-1-yl)methyl)phenylyamino)piperidine-2,6-dione;
4-(4-(4-(2,6-dioxopiperidin-3-yl)benzyl)piperazin-1-yl)-N-(5-((R)-2-methoxy-2-phenylacetyl)-1,4,5,6-tetrahydropyrrolo[3,4-c]pyrazol-3-yl)benzamide;
4-(4-(4-(2,6-dioxopiperidin-3-yl)-3-fluorobenzyl)piperazin-1-yl)-N-(5-((R)-2-methoxy-2-phenylacetyl)-1,4,5,6-tetrahydropyrrolo[3,4-c]pyrazol-3-yl)benzamide;
4-(4-(3-(2,6-dioxopiperidin-3-yl)benzyl)piperazin-1-yl)-N-(5-((R)-2-methoxy-2-phenylacetyl)-1,4,5,6-tetrahydropyrrolo[3,4-c]pyrazol-3-yl)benzamide;
4-(4-(2-(2,6-dioxopiperidin-3-yl)benzyl)piperazin-1-yl)-N-(5-((R)-2-methoxy-2-phenylacetyl)-1,4,5,6-tetrahydropyrrolo[3,4-c]pyrazol-3-yl)benzamide;
(R)-4-(4-(4-(2,4-dioxotetrahydropyrimidin-1(2H)-yl)benzyl)piperazin-1-yl)-N-(5-(2-methoxy-2-phenylacetyl)-1,4,5,6-tetrahydropyrrolo[3,4-c]pyrazol-3-yl)benzamide;
(R)-4-(4-(4-(2,4-dioxotetrahydropyrimidin-1(2H)-yl)-2-fluorobenzyl)piperazin-1-yl)-N-(5-(2-methoxy-2-phenylacetyl)-1,4,5,6-tetrahydropyrrolo[3,4-c]pyrazol-3-yl)benzamide;
(R)-4-(4-(3-(2,4-dioxotetrahydropyrimidin-1(2H)-yl)benzyl)piperazin-1-yl)-N-(5-(2-methoxy-2-phenylacetyl)-1,4,5,6-tetrahydropyrrolo[3,4-c]pyrazol-3-yl)benzamide;
(R)-4-(4-(2-(2,4-dioxotetrahydropyrimidin-1(2H)-yl)benzyl)piperazin-1-yl)-N-(5-(2-methoxy-2-phenylacetyl)-1,4,5,6-tetrahydropyrrolo[3,4-c]pyrazol-3-yl)benzamide;
(R)-4-(4-((5-(2,4-dioxotetrahydropyrimidin-1(2H)-yl)pyridin-2-yl)methyl)piperazin-1-yl)-N-(5-(2-methoxy-2-phenylacetyl)-1,4,5,6-tetrahydropyrrolo[3,4-c]pyrazol-3-yl)benzamide;
(R)-4-(4-((6-(2,4-dioxotetrahydropyrimidin-1(2H)-yl)pyridazin-3-yl)methyl)piperazin-1-yl)-N-(5-(2-methoxy-2-phenylacetyl)-1,4,5,6-tetrahydropyrrolo[3,4-c]pyrazol-3-yl)benzamide;
(R)-4-(4-((3-(2,4-dioxotetrahydropyrimidin-1(2H)-yl)pyridin-4-yl)methyl)piperazin-1-yl)-N-(5-(2-methoxy-2-phenylacetyl)-1,4,5,6-tetrahydropyrrolo[3,4-c]pyrazol-3-yl)benzamide;
4-(4-(4-((2,6-dioxopiperidin-3-yl)amino)benzyl)piperazin-1-yl)-N-(5-((R)-2-methoxy-2-phenylacetyl)-1,4,5,6-tetrahydropyrrolo[3,4-c]pyrazol-3-yl)benzamide;
4-(4-(3-((2,6-dioxopiperidin-3-yl)amino)benzyl)piperazin-1-yl)-N-(5-((R)-2-methoxy-2-phenylacetyl)-1,4,5,6-tetrahydropyrrolo[3,4-c]pyrazol-3-yl)benzamide;
4-(4-(2-((2,6-dioxopiperidin-3-yl)amino)benzyl)piperazin-1-yl)-N-(5-((R)-2-methoxy-2-phenylacetyl)-1,4,5,6-tetrahydropyrrolo[3,4-c]pyrazol-3-yl)benzamide;
4-(4-((4-(2,6-dioxopiperidin-3-yl)benzyl)(methyl)amino)piperidin-1-yl)-N-(5-((R)-2-methoxy-2-phenylacetyl)-1,4,5,6-tetrahydropyrrolo[3,4-c]pyrazol-3-yl)benzamide;
4-(4-((4-(2,6-dioxopiperidin-3-yl)-3-fluorobenzyl)(methyl)amino)piperidin-1-yl)-N-(5-((R)-2-methoxy-2-phenylacetyl)-1,4,5,6-tetrahydropyrrolo[3,4-c]pyrazol-3-yl)benzamide;
4-(4-((3-(2,6-dioxopiperidin-3-yl)benzyl)(methyl)amino)piperidin-1-yl)-N-(5-((R)-2-methoxy-2-phenylacetyl)-1,4,5,6-tetrahydropyrrolo[3,4-c]pyrazol-3-yl)benzamide;
4-(4-((2-(2,6-dioxopiperidin-3-yl)benzyl)(methyl)amino)piperidin-1-yl)-N-(5-((R)-2-methoxy-2-phenylacetyl)-1,4,5,6-tetrahydropyrrolo[3,4-c]pyrazol-3-yl)benzamide;
(R)-4-(4-((4-(2,4-dioxotetrahydropyrimidin-1(2H)-yl)benzyl)(methyl)amino)piperidin-1-yl)-N-(5-(2-methoxy-2-phenylacetyl)-1,4,5,6-tetrahydropyrrolo[3,4-c]pyrazol-3-yl)benzamide;
(R)-4-(4-((4-(2,4-dioxotetrahydropyrimidin-1(2H)-yl)-2-fluorobenzyl)(methyl)amino)piperidin-1-yl)-N-(5-(2-methoxy-2-phenylacetyl)-1,4,5,6-tetrahydropyrrolo[3,4-c]pyrazol-3-yl)benzamide;
(R)-4-(4-((3-(2,4-dioxotetrahydropyrimidin-1(2H)-yl)benzyl)(methyl)amino)piperidin-1-yl)-N-(5-(2-methoxy-2-phenylacetyl)-1,4,5,6-tetrahydropyrrolo[3,4-c]pyrazol-3-yl)benzamide;
(R)-4-(4-((2-(2,4-dioxotetrahydropyrimidin-1(2H)-yl)benzyl)(methyl)amino)piperidin-1-yl)-N-(5-(2-methoxy-2-phenylacetyl)-1,4,5,6-tetrahydropyrrolo[3,4-c]pyrazol-3-yl)benzamide;
(R)-4-(4-(((5-(2,4-dioxotetrahydropyrimidin-1(2H)-yl)pyridin-2-yl)methyl)(methyl)amino)piperidin-1-yl)-N-(5-(2-methoxy-2-phenylacetyl)-1,4,5,6-tetrahydropyrrolo[3,4-c]pyrazol-3-yl)benzamide;
(R)-4-(4-(((6-(2,4-dioxotetrahydropyrimidin-1(2H)-yl)pyridazin-3-yl)methyl)(methyl)amino)piperidin-1-yl)-N-(5-(2-methoxy-2-phenylacetyl)-1,4,5,6-tetrahydropyrrolo[3,4-c]pyrazol-3-yl)benzamide;
(R)-4-(4-(((3-(2,4-dioxotetrahydropyrimidin-1(2H)-yl)pyridin-4-yl)methyl)(methyl)amino)piperidin-1-yl)-N-(5-(2-methoxy-2-phenylacetyl)-1,4,5,6-tetrahydropyrrolo[3,4-c]pyrazol-3-yl)benzamide;
4-(4-((4-((2,6-dioxopiperidin-3-yl)amino)benzyl)(methyl)amino)piperidin-1-yl)-N-(5-((R)-2-methoxy-2-phenylacetyl)-1,4,5,6-tetrahydropyrrolo[3,4-c]pyrazol-3-yl)benzamide;
4-(4-((3-((2,6-dioxopiperidin-3-yl)amino)benzyl)(methyl)amino)piperidin-1-yl)-N-(5-((R)-2-methoxy-2-phenylacetyl)-1,4,5,6-tetrahydropyrrolo[3,4-c]pyrazol-3-yl)benzamide;
4-(4-((2-((2,6-dioxopiperidin-3-yl)amino)benzyl)(methyl)amino)piperidin-1-yl)-N-(5-((R)-2-methoxy-2-phenylacetyl)-1,4,5,6-tetrahydropyrrolo[3,4-c]pyrazol-3-yl)benzamide;
4-(4-(4-(4-(2,6-dioxopiperidin-3-yl)benzyl)piperazin-1-yl)piperidin-1-yl)-N-(5-((R)-2-methoxy-2-phenylacetyl)-1,4,5,6-tetrahydropyrrolo[3,4-c]pyrazol-3-yl)benzamide;
4-(4-(4-(4-(2,6-dioxopiperidin-3-yl)-3-fluorobenzyl)piperazin-1-yl)piperidin-1-yl)-N-(5-((R)-2-methoxy-2-phenylacetyl)-1,4,5,6-tetrahydropyrrolo[3,4-c]pyrazol-3-yl)benzamide;
4-(4-(4-(3-(2,6-dioxopiperidin-3-yl)benzyl)piperazin-1-yl)piperidin-1-yl)-N-(5-((R)-2-methoxy-2-phenylacetyl)-1,4,5,6-tetrahydropyrrolo[3,4-c]pyrazol-3-yl)benzamide;
4-(4-(4-(2-(2,6-dioxopiperidin-3-yl)benzyl)piperazin-1-yl)piperidin-1-yl)-N-(5-((R)-2-methoxy-2-phenylacetyl)-1,4,5,6-tetrahydropyrrolo[3,4-c]pyrazol-3-yl)benzamide;
(R)-4-(4-(4-(4-(2,4-dioxotetrahydropyrimidin-1(2H)-yl)benzyl)piperazin-1-yl)piperidin-1-yl)-N-(5-(2-methoxy-2-phenylacetyl)-1,4,5,6-tetrahydropyrrolo[3,4-c]pyrazol-3-yl)benzamide;
(R)-4-(4-(4-(4-(2,4-dioxotetrahydropyrimidin-1(2H)-yl)-2-fluorobenzyl)piperazin-1-yl)piperidin-1-yl)-N-(5-(2-methoxy-2-phenylacetyl)-1,4,5,6-tetrahydropyrrolo[3,4-c]pyrazol-3-yl)benzamide;
(R)-4-(4-(4-(3-(2,4-dioxotetrahydropyrimidin-1(2H)-yl)benzyl)piperazin-1-yl)piperidin-1-yl)-N-(5-(2-methoxy-2-phenylacetyl)-1,4,5,6-tetrahydropyrrolo[3,4-c]pyrazol-3-yl)benzamide;
(R)-4-(4-(4-(2-(2,4-dioxotetrahydropyrimidin-1(2H)-yl)benzyl)piperazin-1-yl)piperidin-1-yl)-N-(5-(2-methoxy-2-phenylacetyl)-1,4,5,6-tetrahydropyrrolo[3,4-c]pyrazol-3-yl)benzamide;
(R)-4-(4-(4-((5-(2,4-dioxotetrahydropyrimidin-1(2H)-yl)pyridin-2-yl)methyl)piperazin-1-yl)piperidin-1-yl)-N-(5-(2-methoxy-2-phenylacetyl)-1,4,5,6-tetrahydropyrrolo[3,4-c]pyrazol-3-yl)benzamide;
(R)-4-(4-(4-((6-(2,4-dioxotetrahydropyrimidin-1(2H)-yl)pyridazin-3-yl)methyl)piperazin-1-yl)piperidin-1-yl)-N-(5-(2-methoxy-2-phenylacetyl)-1,4,5,6-tetrahydropyrrolo[3,4-c]pyrazol-3-yl)benzamide;
(R)-4-(4-(4-((3-(2,4-dioxotetrahydropyrimidin-1(2H)-yl)pyridin-4-yl)methyl)piperazin-1-yl)piperidin-1-yl)-N-(5-(2-methoxy-2-phenylacetyl)-1,4,5,6-tetrahydropyrrolo[3,4-c]pyrazol-3-yl)benzamide;
4-(4-(4-(4-((2,6-dioxopiperidin-3-yl)amino)benzyl)piperazin-1-yl)piperidin-1-yl)-N-(5-((R)-2-methoxy-2-phenylacetyl)-1,4,5,6-tetrahydropyrrolo[3,4-c]pyrazol-3-yl)benzamide;
4-(4-(4-(3-((2,6-dioxopiperidin-3-yl)amino)benzyl)piperazin-1-yl)piperidin-1-yl)-N-(5-((R)-2-methoxy-2-phenylacetyl)-1,4,5,6-tetrahydropyrrolo[3,4-c]pyrazol-3-yl)benzamide;
4-(4-(4-(2-((2,6-dioxopiperidin-3-yl)amino)benzyl)piperazin-1-yl)piperidin-1-yl)-N-(5-((R)-2-methoxy-2-phenylacetyl)-1,4,5,6-tetrahydropyrrolo[3,4-c]pyrazol-3-yl)benzamide;
N-(tert-butyl)-3-((2-((6-(4-(4-(2,6-dioxopiperidin-3-yl)benzyl)piperazin-1-yl)pyridazin-3-yl)amino)-5-methylpyrimidin-4-yl)amino)benzenesulfonamide;
N-(tert-butyl)-3-((2-((6-(4-(4-(2,6-dioxopiperidin-3-yl)-3-fluorobenzyl)piperazin-1-yl)pyridazin-3-yl)amino)-5-methylpyrimidin-4-yl)amino)benzenesulfonamide;
N-(tert-butyl)-3-((2-((6-(4-(3-(2,6-dioxopiperidin-3-yl)benzyl)piperazin-1-yl)pyridazin-3-yl)amino)-5-methylpyrimidin-4-yl)amino)benzenesulfonamide;
N-(tert-butyl)-3-((2-((6-(4-(2-(2,6-dioxopiperidin-3-yl)benzyl)piperazin-1-yl)pyridazin-3-yl)amino)-5-methylpyrimidin-4-yl)amino)benzenesulfonamide;
N-(tert-butyl)-3-((2-((6-(4-(4-(2,4-dioxotetrahydropyrimidin-1(2H)-yl)benzyl)piperazin-1-yl)pyridazin-3-yl)amino)-5-methylpyrimidin-4-yl)amino)benzenesulfonamide;
N-(tert-butyl)-3-((2-((6-(4-(4-(2,4-dioxotetrahydropyrimidin-1(2H)-yl)-2-fluorobenzyl)piperazin-1-yl)pyridazin-3-yl)amino)-5-methylpyrimidin-4-yl)amino)benzenesulfonamide;
N-(tert-butyl)-3-((2-((6-(4-(3-(2,4-dioxotetrahydropyrimidin-1(2H)-yl)benzyl)piperazin-1-yl)pyridazin-3-yl)amino)-5-methylpyrimidin-4-yl)amino)benzenesulfonamide;
N-(tert-butyl)-3-((2-((6-(4-(2-(2,4-dioxotetrahydropyrimidin-1(2H)-yl)benzyl)piperazin-1-yl)pyridazin-3-yl)amino)-5-methylpyrimidin-4-yl)amino)benzenesulfonamide;
N-(tert-butyl)-3-((2-((6-(4-((5-(2,4-dioxotetrahydropyrimidin-1(2H)-yl)pyridin-2-yl)methyl)piperazin-1-yl)pyridazin-3-yl)amino)-5-methylpyrimidin-4-yl)amino)benzenesulfonamide;
N-(tert-butyl)-3-((2-((6-(4-((6-(2,4-dioxotetrahydropyrimidin-1(2H)-yl)pyridazin-3-yl)methyl)piperazin-1-yl)pyridazin-3-yl)amino)-5-methylpyrimidin-4-yl)amino)benzenesulfonamide;
N-(tert-butyl)-3-((2-((6-(4-((3-(2,4-dioxotetrahydropyrimidin-1(2H)-yl)pyridin-4-yl)methyl)piperazin-1-yl)pyridazin-3-yl)amino)-5-methylpyrimidin-4-yl)amino)benzenesulfonamide;
N-(tert-butyl)-3-((2-((6-(4-(4-((2,6-dioxopiperidin-3-yl)amino)benzyl)piperazin-1-yl)pyridazin-3-yl)amino)-5-methylpyrimidin-4-yl)amino)benzenesulfonamide;
N-(tert-butyl)-3-((2-((6-(4-(3-((2,6-dioxopiperidin-3-yl)amino)benzyl)piperazin-1-yl)pyridazin-3-yl)amino)-5-methylpyrimidin-4-yl)amino)benzenesulfonamide;
N-(tert-butyl)-3-((2-((6-(4-(2-((2,6-dioxopiperidin-3-yl)amino)benzyl)piperazin-1-yl)pyridazin-3-yl)amino)-5-methylpyrimidin-4-yl)amino)benzenesulfonamide;
N-(tert-butyl)-3-((2-((4-(4-((4-(2,6-dioxopiperidin-3-yl)benzyl)(methyl)amino)piperidin-1-yl)phenyl)amino)-5-methylpyrimidin-4-yl)amino)benzenesulfonamide;
N-(tert-butyl)-3-((2-((4-(4-((4-(2,6-dioxopiperidin-3-yl)-3-fluorobenzyl)(methyl)amino)piperidin-1-yl)phenyl)amino)-5-methylpyrimidin-4-yl)amino)benzenesulfonamide;
N-(tert-butyl)-3-((2-((4-(4-((3-(2,6-dioxopiperidin-3-yl)benzyl)(methyl)amino)piperidin-1-yl)phenyl)amino)-5-methylpyrimidin-4-yl)amino)benzenesulfonamide;
N-(tert-butyl)-3-((2-((4-(4-((2-(2,6-dioxopiperidin-3-yl)benzyl)(methyl)amino)piperidin-1-yl)phenyl)amino)-5-methylpyrimidin-4-yl)amino)benzenesulfonamide;
N-(tert-butyl)-3-((2-((4-(4-((4-(2,4-dioxotetrahydropyrimidin-1(2H)-yl)benzyl)(methyl)amino)piperidin-1-yl)phenyl)amino)-5-methylpyrimidin-4-yl)amino)benzenesulfonamide;
N-(tert-butyl)-3-((2-((4-(4-((4-(2,4-dioxotetrahydropyrimidin-1(2H)-yl)-2-fluorobenzyl)(methyl)amino)piperidin-1-yl)phenyl)amino)-5-methylpyrimidin-4-yl)amino)benzenesulfonamide;
N-(tert-butyl)-3-((2-((4-(4-((3-(2,4-dioxotetrahydropyrimidin-1(2H)-yl)benzyl)(methyl)amino)piperidin-1-yl)phenyl)amino)-5-methylpyrimidin-4-yl)amino)benzenesulfonamide;
N-(tert-butyl)-3-((2-((4-(4-((2-(2,4-dioxotetrahydropyrimidin-1(2H)-yl)benzyl)(methyl)amino)piperidin-1-yl)phenyl)amino)-5-methylpyrimidin-4-yl)amino)benzenesulfonamide;
N-(tert-butyl)-3-((2-((4-(4-(((5-(2,4-dioxotetrahydropyrimidin-1(2H)-yl)pyridin-2-yl)methyl)(methyl)amino)piperidin-1-yl)phenyl)amino)-5-methylpyrimidin-4-yl)amino)benzenesulfonamide;
N-(tert-butyl)-3-((2-((4-(4-(((6-(2,4-dioxotetrahydropyrimidin-1(2H)-yl)pyridazin-3-yl)methyl)(methyl)amino)piperidin-1-yl)phenyl)amino)-5-methylpyrimidin-4-yl)amino)benzenesulfonamide;
N-(tert-butyl)-3-((2-((4-(4-(((3-(2,4-dioxotetrahydropyrimidin-1(2H)-yl)pyridin-4-yl)methyl)(methyl)amino)piperidin-1-yl)phenyl)amino)-5-methylpyrimidin-4-yl)amino)benzenesulfonamide;
N-(tert-butyl)-3-((2-((4-(4-((4-((2,6-dioxopiperidin-3-yl)amino)benzyl)(methyl)amino)piperidin-1-yl)phenyl)amino)-5-methylpyrimidin-4-yl)amino)benzenesulfonamide;
N-(tert-butyl)-3-((2-((4-(4-((3-((2,6-dioxopiperidin-3-yl)amino)benzyl)(methyl)amino)piperidin-1-yl)phenyl)amino)-5-methylpyrimidin-4-yl)amino)benzenesulfonamide;
N-(tert-butyl)-3-((2-((4-(4-((2-((2,6-dioxopiperidin-3-yl)amino)benzyl)(methyl)amino)piperidin-1-yl)phenyl)amino)-5-methylpyrimidin-4-yl)amino)benzenesulfonamide;
N-(tert-butyl)-3-((2-((4-(4-(4-(4-(2,6-dioxopiperidin-3-yl)benzyl)piperazin-1-yl)piperidin-1-yl)phenyl)amino)-5-methylpyrimidin-4-yl)amino)benzenesulfonamide;
N-(tert-butyl)-3-((2-((4-(4-(4-(4-(2,6-dioxopiperidin-3-yl)-3-fluorobenzyl)piperazin-1-yl)piperidin-1-yl)phenyl)amino)-5-methylpyrimidin-4-yl)amino)benzenesulfonamide;
N-(tert-butyl)-3-((2-((4-(4-(4-(3-(2,6-dioxopiperidin-3-yl)benzyl)piperazin-1-yl)piperidin-1-yl)phenyl)amino)-5-methylpyrimidin-4-yl)amino)benzenesulfonamide;
N-(tert-butyl)-3-((2-((4-(4-(4-(2-(2,6-dioxopiperidin-3-yl)benzyl)piperazin-1-yl)piperidin-1-yl)phenyl)amino)-5-methylpyrimidin-4-yl)amino)benzenesulfonamide;
N-(tert-butyl)-3-((2-((4-(4-(4-(4-(2,4-dioxotetrahydropyrimidin-1(2H)-yl)benzyl)piperazin-1-yl)piperidin-1-yl)phenyl)amino)-5-methylpyrimidin-4-yl)amino)benzenesulfonamide;
N-(tert-butyl)-3-((2-((4-(4-(4-(4-(2,4-dioxotetrahydropyrimidin-1(2H)-yl)-2-fluorobenzyl)piperazin-1-yl)piperidin-1-yl)phenyl)amino)-5-methylpyrimidin-4-yl)amino)benzenesulfonamide;
N-(tert-butyl)-3-((2-((4-(4-(4-(3-(2,4-dioxotetrahydropyrimidin-1(2H)-yl)benzyl)piperazin-1-yl)piperidin-1-yl)phenyl)amino)-5-methylpyrimidin-4-yl)amino)benzenesulfonamide;
N-(tert-butyl)-3-((2-((4-(4-(4-(2-(2,4-dioxotetrahydropyrimidin-1(2H)-yl)benzyl)piperazin-1-yl)piperidin-1-yl)phenyl)amino)-5-methylpyrimidin-4-yl)amino)benzenesulfonamide;
N-(tert-butyl)-3-((2-((4-(4-(4-((5-(2,4-dioxotetrahydropyrimidin-1(2H)-yl)pyridin-2-yl)methyl)piperazin-1-yl)piperidin-1-yl)phenyl)amino)-5-methylpyrimidin-4-yl)amino)benzenesulfonamide;
N-(tert-butyl)-3-((2-((4-(4-(4-((6-(2,4-dioxotetrahydropyrimidin-1(2H)-yl)pyridazin-3-yl)methyl)piperazin-1-yl)piperidin-1-yl)phenyl)amino)-5-methylpyrimidin-4-yl)amino)benzenesulfonamide;
N-(tert-butyl)-3-((2-((4-(4-(4-((3-(2,4-dioxotetrahydropyrimidin-1(2H)-yl)pyridin-4-yl)methyl)piperazin-1-yl)piperidin-1-yl)phenyl)amino)-5-methylpyrimidin-4-yl)amino)benzenesulfonamide;
N-(tert-butyl)-3-((2-((4-(4-(4-(4-((2,6-dioxopiperidin-3-yl)amino)benzyl)piperazin-1-yl)piperidin-1-yl)phenyl)amino)-5-methylpyrimidin-4-yl)amino)benzenesulfonamide;
N-(tert-butyl)-3-((2-((4-(4-(4-(3-((2,6-dioxopiperidin-3-yl)amino)benzyl)piperazin-1-yl)piperidin-1-yl)phenyl)amino)-5-methylpyrimidin-4-yl)amino)benzenesulfonamide;
N-(tert-butyl)-3-((2-((4-(4-(4-(2-((2,6-dioxopiperidin-3-yl)amino)benzyl)piperazin-1-yl)piperidin-1-yl)phenyl)amino)-5-methylpyrimidin-4-yl)amino)benzenesulfonamide;
N-(tert-butyl)-3-((2-((4-(4-(4-(2,6-dioxopiperidin-3-yl)benzyl)piperazin-1-yl)phenyl)amino)-5-methylpyrimidin-4-yl)amino)benzenesulfonamide;
N-(tert-butyl)-3-((2-((4-(4-(4-(2,6-dioxopiperidin-3-yl)-3-fluorobenzyl)piperazin-1-yl)phenyl)amino)-5-methylpyrimidin-4-yl)amino)benzenesulfonamide;
N-(tert-butyl)-3-((2-((4-(4-(3-(2,6-dioxopiperidin-3-yl)benzyl)piperazin-1-yl)phenyl)amino)-5-methylpyrimidin-4-yl)amino)benzenesulfonamide;
N-(tert-butyl)-3-((2-((4-(4-(2-(2,6-dioxopiperidin-3-yl)benzyl)piperazin-1-yl)phenyl)amino)-5-methylpyrimidin-4-yl)amino)benzenesulfonamide;
N-(tert-butyl)-3-((2-((4-(4-(4-(2,4-dioxotetrahydropyrimidin-1(2H)-yl)benzyl)piperazin-1-yl)phenyl)amino)-5-methylpyrimidin-4-yl)amino)benzenesulfonamide;
N-(tert-butyl)-3-((2-((4-(4-(4-(2,4-dioxotetrahydropyrimidin-1(2H)-yl)-2-fluorobenzyl)piperazin-1-yl)phenyl)amino)-5-methylpyrimidin-4-yl)amino)benzenesulfonamide;
N-(tert-butyl)-3-((2-((4-(4-(3-(2,4-dioxotetrahydropyrimidin-1(2H)-yl)benzyl)piperazin-1-yl)phenyl)amino)-5-methylpyrimidin-4-yl)amino)benzenesulfonamide;
N-(tert-butyl)-3-((2-((4-(4-(2-(2,4-dioxotetrahydropyrimidin-1(2H)-yl)benzyl)piperazin-1-yl)phenyl)amino)-5-methylpyrimidin-4-yl)amino)benzenesulfonamide;
N-(tert-butyl)-3-((2-((4-(4-((5-(2,4-dioxotetrahydropyrimidin-1(2H)-yl)pyridin-2-yl)methyl)piperazin-1-yl)phenyl)amino)-5-methylpyrimidin-4-yl)amino)benzenesulfonamide;
N-(tert-butyl)-3-((2-((4-(4-((6-(2,4-dioxotetrahydropyrimidin-1(2H)-yl)pyridazin-3-yl)methyl)piperazin-1-yl)phenyl)amino)-5-methylpyrimidin-4-yl)amino)benzenesulfonamide;
N-(tert-butyl)-3-((2-((4-(4-((3-(2,4-dioxotetrahydropyrimidin-1(2H)-yl)pyridin-4-yl)methyl)piperazin-1-yl)phenyl)amino)-5-methylpyrimidin-4-yl)amino)benzenesulfonamide;
N-(tert-butyl)-3-((2-((4-(4-(4-((2,6-dioxopiperidin-3-yl)amino)benzyl)piperazin-1-yl)phenyl)amino)-5-methylpyrimidin-4-yl)amino)benzenesulfonamide;
N-(tert-butyl)-3-((2-((4-(4-(3-((2,6-dioxopiperidin-3-yl)amino)benzyl)piperazin-1-yl)phenyl)amino)-5-methylpyrimidin-4-yl)amino)benzenesulfonamide;
N-(tert-butyl)-3-((2-((4-(4-(2-((2,6-dioxopiperidin-3-yl)amino)benzyl)piperazin-1-yl)phenyl)amino)-5-methylpyrimidin-4-yl)amino)benzenesulfonamide;
N-(tert-butyl)-3-((2-((4-(2-(4-(4-(2,6-dioxopiperidin-3-yl)benzyl)piperazin-1-yl)ethoxy)phenyl)amino)-5-methylpyrimidin-4-yl)amino)benzenesulfonamide;
N-(tert-butyl)-3-((2-((4-(2-(4-(4-(2,6-dioxopiperidin-3-yl)-3-fluorobenzyl)piperazin-1-yl)ethoxy)phenyl)amino)-5-methylpyrimidin-4-yl)amino)benzenesulfonamide;
N-(tert-butyl)-3-((2-((4-(2-(4-(3-(2,6-dioxopiperidin-3-yl)benzyl)piperazin-1-yl)ethoxy)phenyl)amino)-5-methylpyrimidin-4-yl)amino)benzenesulfonamide;
N-(tert-butyl)-3-((2-((4-(2-(4-(2-(2,6-dioxopiperidin-3-yl)benzyl)piperazin-1-yl)ethoxy)phenyl)amino)-5-methylpyrimidin-4-yl)amino)benzenesulfonamide;
N-(tert-butyl)-3-((2-((4-(2-(4-(4-(2,4-dioxotetrahydropyrimidin-1(2H)-yl)benzyl)piperazin-1-yl)ethoxy)phenyl)amino)-5-methylpyrimidin-4-yl)amino)benzenesulfonamide;
N-(tert-butyl)-3-((2-((4-(2-(4-(4-(2,4-dioxotetrahydropyrimidin-1(2H)-yl)-2-fluorobenzyl)piperazin-1-yl)ethoxy)phenyl)amino)-5-methylpyrimidin-4-yl)amino)benzenesulfonamide;
N-(tert-butyl)-3-((2-((4-(2-(4-(3-(2,4-dioxotetrahydropyrimidin-1(2H)-yl)benzyl)piperazin-1-yl)ethoxy)phenyl)amino)-5-methylpyrimidin-4-yl)amino)benzenesulfonamide;
N-(tert-butyl)-3-((2-((4-(2-(4-(2-(2,4-dioxotetrahydropyrimidin-1(2H)-yl)benzyl)piperazin-1-yl)ethoxy)phenyl)amino)-5-methylpyrimidin-4-yl)amino)benzenesulfonamide;
N-(tert-butyl)-3-((2-((4-(2-(4-((5-(2,4-dioxotetrahydropyrimidin-1(2H)-yl)pyridin-2-yl)methyl)piperazin-1-yl)ethoxy)phenyl)amino)-5-methylpyrimidin-4-yl)amino)benzenesulfonamide;
N-(tert-butyl)-3-((2-((4-(2-(4-((6-(2,4-dioxotetrahydropyrimidin-1(2H)-yl)pyridazin-3-yl)methyl)piperazin-1-yl)ethoxy)phenyl)amino)-5-methylpyrimidin-4-yl)amino)benzenesulfonamide;
N-(tert-butyl)-3-((2-((4-(2-(4-((3-(2,4-dioxotetrahydropyrimidin-1(2H)-yl)pyridin-4-yl)methyl)piperazin-1-yl)ethoxy)phenyl)amino)-5-methylpyrimidin-4-yl)amino)benzenesulfonamide;
N-(tert-butyl)-3-((2-((4-(2-(4-(4-((2,6-dioxopiperidin-3-yl)amino)benzyl)piperazin-1-yl)ethoxy)phenyl)amino)-5-methylpyrimidin-4-yl)amino)benzenesulfonamide;
N-(tert-butyl)-3-((2-((4-(2-(4-(3-((2,6-dioxopiperidin-3-yl)amino)benzyl)piperazin-1-yl)ethoxy)phenyl)amino)-5-methylpyrimidin-4-yl)amino)benzenesulfonamide;
N-(tert-butyl)-3-((2-((4-(2-(4-(2-((2,6-dioxopiperidin-3-yl)amino)benzyl)piperazin-1-yl)ethoxy)phenyl)amino)-5-methylpyrimidin-4-yl)amino)benzenesulfonamide;
2-((2-((4-(4-(4-(4-(2,6-dioxopiperidin-3-yl)benzyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-(4-(2,6-dioxopiperidin-3-yl)-3-fluorobenzyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-(3-(2,6-dioxopiperidin-3-yl)benzyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-(2-(2,6-dioxopiperidin-3-yl)benzyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-(4-(2,4-dioxotetrahydropyrimidin-1(2H)-yl)benzyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-(4-(2,4-dioxotetrahydropyrimidin-1(2H)-yl)-2-fluorobenzyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-(3-(2,4-dioxotetrahydropyrimidin-1(2H)-yl)benzyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-(2-(2,4-dioxotetrahydropyrimidin-1(2H)-yl)benzyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-((5-(2,4-dioxotetrahydropyrimidin-1(2H)-yl)pyridin-2-yl)methyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-((6-(2,4-dioxotetrahydropyrimidin-1(2H)-yl)pyridazin-3-yl)methyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-((3-(2,4-dioxotetrahydropyrimidin-1(2H)-yl)pyridin-4-yl)methyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-(4-((2,6-dioxopiperidin-3-yl)amino)benzyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-(3-((2,6-dioxopiperidin-3-yl)amino)benzyl)piperazin-1-yl)piperidin-1-yl)-2-methoxyphenyl)amino)-5-(trifluoromethyl)pyridin-4-yl)amino)-N-methylbenzamide;
2-((2-((4-(4-(4-(2-((2,6-dioxopiperidin-3-yl)amino)benzyl)piperazin-1-yl)piperidin-1-yl)-2-methoxyphenyl)amino)-5-(trifluoromethyl)pyridin-4-yl)amino)-N-methylbenzamide;
2-((2-((4-(4-((4-(2,6-dioxopiperidin-3-yl)benzyl)(methyl)amino)piperidin-1-yl)-2-methoxyphenyl)amino)-5-(trifluoromethyl)pyridin-4-yl)amino)-N-methylbenzamide;
2-((2-((4-(4-((4-(2,6-dioxopiperidin-3-yl)-3-fluorobenzyl)(methyl)amino)piperidin-1-yl)-2-methoxyphenyl)amino)-5-(trifluoromethyl)pyridin-4-yl)amino)-N-methylbenzamide;
2-((2-((4-(4-((3-(2,6-dioxopiperidin-3-yl)benzyl)(methyl)amino)piperidin-1-yl)-2-methoxyphenyl)amino)-5-(trifluoromethyl)pyridin-4-yl)amino)-N-methylbenzamide;
2-((2-((4-(4-((2-(2,6-dioxopiperidin-3-yl)benzyl)(methyl)amino)piperidin-1-yl)-2-methoxyphenyl)amino)-5-(trifluoromethyl)pyridin-4-yl)amino)-N-methylbenzamide;
2-((2-((4-(4-(((5-(2,6-dioxopiperidin-3-yl)pyridin-2-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-((4-(2,4-dioxotetrahydropyrimidin-1(2H)-yl)benzyl)(methyl)amino)piperidin-1-yl)-2-methoxyphenyl)amino)-5-(trifluoromethyl)pyridin-4-yl)amino)-N-methylbenzamide;
2-((2-((4-(4-((4-(2,4-dioxotetrahydropyrimidin-1(2H)-yl)-2-fluorobenzyl)(methyl)amino)piperidin-1-yl)-2-methoxyphenyl)amino)-5-(trifluoromethyl)pyridin-4-yl)amino)-N-methylbenzamide;
2-((2-((4-(4-((3-(2,4-dioxotetrahydropyrimidin-1(2H)-yl)benzyl)(methyl)amino)piperidin-1-yl)-2-methoxyphenyl)amino)-5-(trifluoromethyl)pyridin-4-yl)amino)-N-methylbenzamide;
2-((2-((4-(4-((2-(2,4-dioxotetrahydropyrimidin-1(2H)-yl)benzyl)(methyl)amino)piperidin-1-yl)-2-methoxyphenyl)amino)-5-(trifluoromethyl)pyridin-4-yl)amino)-N-methylbenzamide;
2-((2-((4-(4-(((5-(2,4-dioxotetrahydropyrimidin-1(2H)-yl)pyridin-2-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-(((2-(2,4-dioxotetrahydropyrimidin-1(2H)-yl)pyridin-4-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-(((2-(2,4-dioxotetrahydropyrimidin-1 (2H)-yl)-5-fluoropyridin-4-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-(((6-(2,4-dioxotetrahydropyrimidin-1(2H)-yl)pyridin-3-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-(((6-(2,4-dioxotetrahydropyrimidin-1(2H)-yl)pyridazin-3-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-(((3-(2,4-dioxotetrahydropyrimidin-1(2H)-yl)pyridin-4-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-((4-((2,6-dioxopiperidin-3-yl)amino)benzyl)(methyl)amino)piperidin-1-yl)-2-methoxyphenyl)amino)-5-(trifluoromethyl)pyridin-4-yl)amino)-N-methylbenzamide;
2-((2-((4-(4-((3-((2,6-dioxopiperidin-3-yl)amino)benzyl)(methyl)amino)piperidin-1-yl)-2-methoxyphenyl)amino)-5-(trifluoromethyl)pyridin-4-yl)amino)-N-methylbenzamide;
2-((2-((4-(4-((2-((2,6-dioxopiperidin-3-yl)amino)benzyl)(methyl)amino)piperidin-1-yl)-2-methoxyphenyl)amino)-5-(trifluoromethyl)pyridin-4-yl)amino)-N-methylbenzamide;
2-((2-((4-(4-(4-(2,6-dioxopiperidin-3-yl)benzyl)piperazin-1-yl)-2-methoxyphenyl)amino)-5-(trifluoromethyl)pyridin-4-yl)amino)-N-methylbenzamide;
2-((2-((4-(4-(4-(2,6-dioxopiperidin-3-yl)-3-fluorobenzyl)piperazin-1-yl)-2-methoxyphenyl)amino)-5-(trifluoromethyl)pyridin-4-yl)amino)-N-methylbenzamide;
2-((2-((4-(4-(3-(2,6-dioxopiperidin-3-yl)benzyl)piperazin-1-yl)-2-methoxyphenyl)amino)-5-(trifluoromethyl)pyridin-4-yl)amino)-N-methylbenzamide;
2-((2-((4-(4-(2-(2,6-dioxopiperidin-3-yl)benzyl)piperazin-1-yl)-2-methoxyphenyl)amino)-5-(trifluoromethyl)pyridin-4-yl)amino)-N-methylbenzamide;
2-((2-((4-(4-(4-(2,4-dioxotetrahydropyrimidin-1(2H)-yl)benzyl)piperazin-1-yl)-2-methoxyphenyl)amino)-5-(trifluoromethyl)pyridin-4-yl)amino)-N-methylbenzamide;
2-((2-((4-(4-(4-(2,4-dioxotetrahydropyrimidin-1(2H)-yl)-2-fluorobenzyl)piperazin-1-yl)-2-methoxyphenyl)amino)-5-(trifluoromethyl)pyridin-4-yl)amino)-N-methylbenzamide;
2-((2-((4-(4-(3-(2,4-dioxotetrahydropyrimidin-1(2H)-yl)benzyl)piperazin-1-yl)-2-methoxyphenyl)amino)-5-(trifluoromethyl)pyridin-4-yl)amino)-N-methylbenzamide;
2-((2-((4-(4-(2-(2,4-dioxotetrahydropyrimidin-1(2H)-yl)benzyl)piperazin-1-yl)-2-methoxyphenyl)amino)-5-(trifluoromethyl)pyridin-4-yl)amino)-N-methylbenzamide;
2-((2-((4-(4-((5-(2,4-dioxotetrahydropyrimidin-1(2H)-yl)pyridin-2-yl)methyl)piperazin-1-yl)-2-methoxyphenyl)amino)-5-(trifluoromethyl)pyridin-4-yl)amino)-N-methylbenzamide;
2-((2-((4-(4-((6-(2,4-dioxotetrahydropyrimidin-1(2H)-yl)pyridazin-3-yl)methyl)piperazin-1-yl)-2-methoxyphenyl)amino)-5-(trifluoromethyl)pyridin-4-yl)amino)-N-methylbenzamide;
2-((2-((4-(4-((3-(2,4-dioxotetrahydropyrimidin-1(2H)-yl)pyridin-4-yl)methyl)piperazin-1-yl)-2-methoxyphenyl)amino)-5-(trifluoromethyl)pyridin-4-yl)amino)-N-methylbenzamide;
2-((2-((4-(4-(((5-(2,6-dioxopiperidin-3-yl)-2-fluoropyridin-3-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-(((6-(2,6-dioxopiperidin-3-yl)pyridin-3-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-(((5-(2,4-dioxotetrahydropyrimidin-1(2H)-yl)pyridin-3-yl)methyl)(methyl)amino)piperidin-1-yl)-2-methoxyphenyl)amino)-5-(trifluoromethyl)pyridin-4-yl)amino)-N-methylbenzamide;
2-((2-((4-(1-(4-(2,6-dioxopiperidin-3-yl)benzyl)piperidin-4-yl)-2-isopropoxy-5-methylphenyl)amino)-5-(trifluoromethyl)pyridin-4-yl)amino)-N-methylbenzamide;
2-((2-((4-(1-(4-(2,6-dioxopiperidin-3-yl)-3-fluorobenzyl)piperidin-4-yl)-2-isopropoxy-5-methylphenyl)amino)-5-(trifluoromethyl)pyridin-4-yl)amino)-N-methylbenzamide;
2-((2-((4-(1-(3-(2,6-dioxopiperidin-3-yl)benzyl)piperidin-4-yl)-2-isopropoxy-5-methylphenyl)amino)-5-(trifluoromethyl)pyridin-4-yl)amino)-N-methylbenzamide;
2-((2-((4-(1-(2-(2,6-dioxopiperidin-3-yl)benzyl)piperidin-4-yl)-2-isopropoxy-5-methylphenyl)amino)-5-(trifluoromethyl)pyridin-4-yl)amino)-N-methylbenzamide;
2-((2-((4-(1-((5-(2,6-dioxopiperidin-3-yl)pyridin-3-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((5-(2,6-dioxopiperidin-3-yl)-2-fluoropyridin-3-yl)methyl)piperidin-4-yl)-2-isopropoxy-5-methylphenyl)amino)-5-(trifluoromethyl)pyridin-4-yl)amino)-N-methylbenzamide;
2-((2-((4-(1-(4-(2,4-dioxotetrahydropyrimidin-1(2H)-yl)benzyl)piperidin-4-yl)-2-isopropoxy-5-methylphenyl)amino)-5-(trifluoromethyl)pyridin-4-yl)amino)-N-methylbenzamide;
2-((2-((4-(1-(4-(2,4-dioxotetrahydropyrimidin-1(2H)-yl)-2-fluorobenzyl)piperidin-4-yl)-2-isopropoxy-5-methylphenyl)amino)-5-(trifluoromethyl)pyridin-4-yl)amino)-N-methylbenzamide;
2-((2-((4-(1-(3-(2,4-dioxotetrahydropyrimidin-1(2H)-yl)benzyl)piperidin-4-yl)-2-isopropoxy-5-methylphenyl)amino)-5-(trifluoromethyl)pyridin-4-yl)amino)-N-methylbenzamide;
2-((2-((4-(1-(2-(2,4-dioxotetrahydropyrimidin-1(2H)-yl)benzyl)piperidin-4-yl)-2-isopropoxy-5-methylphenyl)amino)-5-(trifluoromethyl)pyridin-4-yl)amino)-N-methylbenzamide;
2-((2-((4-(1-((5-(2,4-dioxotetrahydropyrimidin-1(2H)-yl)pyridin-2-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((2-(2,6-dioxopiperidin-3-yl)pyridin-4-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((2-(2,4-dioxotetrahydropyrimidin-1(2H)-yl)pyridin-4-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((2-(2,4-dioxotetrahydropyrimidin-1(2H)-yl)-5-fluoropyridin-4-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((5-(2,4-dioxotetrahydropyrimidin-1(2H)-yl)-2-fluoropyridin-3-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((6-(2,6-dioxopiperidin-3-yl)pyridin-3-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((6-(2,4-dioxotetrahydropyrimidin-1(2H)-yl)pyridin-3-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((5-(2,4-dioxotetrahydropyrimidin-1(2H)-yl)pyridin-3-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((6-(2,4-dioxotetrahydropyrimidin-1(2H)-yl)pyridazin-3-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((3-(2,4-dioxotetrahydropyrimidin-1(2H)-yl)pyridin-4-yl)methyl)piperidin-4-yl)-2-isopropoxy-5-methylphenyl)amino)-5-(trifluoromethyl)pyridin-4-yl)amino)-N-methylbenzamide;
2-((2-((4-(1-(4-((2,6-dioxopiperidin-3-yl)amino)benzyl)piperidin-4-yl)-2-isopropoxy-5-methylphenyl)amino)-5-(trifluoromethyl)pyridin-4-yl)amino)-N-methylbenzamide;
2-((2-((4-(1-(3-((2,6-dioxopiperidin-3-yl)amino)benzyl)piperidin-4-yl)-2-isopropoxy-5-methylphenyl)amino)-5-(trifluoromethyl)pyridin-4-yl)amino)-N-methylbenzamide;
2-((2-((4-(1-(2-((2,6-dioxopiperidin-3-yl)amino)benzyl)piperidin-4-yl)-2-isopropoxy-5-methylphenyl)amino)-5-(trifluoromethyl)pyridin-4-yl)amino)-N-methylbenzamide;
2-((2-((4-(4-(4-(2,6-dioxopiperidin-3-yl)benzyl)piperazin-1-yl)-2-isopropoxy-5-methylphenyl)amino)-5-(trifluoromethyl)pyridin-4-yl)amino)-N-methylbenzamide;
2-((2-((4-(4-(4-(2,6-dioxopiperidin-3-yl)-3-fluorobenzyl)piperazin-1-yl)-2-isopropoxy-5-methylphenyl)amino)-5-(trifluoromethyl)pyridin-4-yl)amino)-N-methylbenzamide;
2-((2-((4-(4-(3-(2,6-dioxopiperidin-3-yl)benzyl)piperazin-1-yl)-2-isopropoxy-5-methylphenyl)amino)-5-(trifluoromethyl)pyridin-4-yl)amino)-N-methylbenzamide;
2-((2-((4-(4-(2-(2,6-dioxopiperidin-3-yl)benzyl)piperazin-1-yl)-2-isopropoxy-5-methylphenyl)amino)-5-(trifluoromethyl)pyridin-4-yl)amino)-N-methylbenzamide;
2-((2-((4-(4-(4-(2,4-dioxotetrahydropyrimidin-1(2H)-yl)benzyl)piperazin-1-yl)-2-isopropoxy-5-methylphenyl)amino)-5-(trifluoromethyl)pyridin-4-yl)amino)-N-methylbenzamide;
2-((2-((4-(4-(4-(2,4-dioxotetrahydropyrimidin-1(2H)-yl)-2-fluorobenzyl)piperazin-1-yl)-2-isopropoxy-5-methylphenyl)amino)-5-(trifluoromethyl)pyridin-4-yl)amino)-N-methylbenzamide;
2-((2-((4-(4-(3-(2,4-dioxotetrahydropyrimidin-1(2H)-yl)benzyl)piperazin-1-yl)-2-isopropoxy-5-methylphenyl)amino)-5-(trifluoromethyl)pyridin-4-yl)amino)-N-methylbenzamide;
2-((2-((4-(4-(2-(2,4-dioxotetrahydropyrimidin-1(2H)-yl)benzyl)piperazin-1-yl)-2-isopropoxy-5-methylphenyl)amino)-5-(trifluoromethyl)pyridin-4-yl)amino)-N-methylbenzamide;
2-((2-((4-(4-((5-(2,4-dioxotetrahydropyrimidin-1(2H)-yl)pyridin-2-yl)methyl)piperazin-1-yl)-2-isopropoxy-5-methylphenyl)amino)-5-(trifluoromethyl)pyridin-4-yl)amino)-N-methylbenzamide;
2-((2-((4-(4-((6-(2,4-dioxotetrahydropyrimidin-1(2H)-yl)pyridazin-3-yl)methyl)piperazin-1-yl)-2-isopropoxy-5-methylphenyl)amino)-5-(trifluoromethyl)pyridin-4-yl)amino)-N-methylbenzamide;
2-((2-((4-(1-((5-(2,4-dioxotetrahydropyrimidin-1(2H)-yl)pyrazin-2-yl)methyl)piperidin-4-yl)-2-isopropoxy-5-methylphenyl)amino)-5-(trifluoromethyl)pyridin-4-yl)amino)-N-methylbenzamide;
2-((2-((4-(4-((3-(2,4-dioxotetrahydropyrimidin-1(2H)-yl)pyridin-4-yl)methyl)piperazin-1-yl)-2-isopropoxy-5-methylphenyl)amino)-5-(trifluoromethyl)pyridin-4-yl)amino)-N-methylbenzamide;
2-((2-((4-(4-(4-((2,6-dioxopiperidin-3-yl)amino)benzyl)piperazin-1-yl)-2-isopropoxy-5-methylphenyl)amino)-5-(trifluoromethyl)pyridin-4-yl)amino)-N-methylbenzamide;
2-((2-((4-(4-(3-((2,6-dioxopiperidin-3-yl)amino)benzyl)piperazin-1-yl)-2-isopropoxy-5-methylphenyl)amino)-5-(trifluoromethyl)pyridin-4-yl)amino)-N-methylbenzamide;
2-((2-((4-(4-(2-((2,6-dioxopiperidin-3-yl)amino)benzyl)piperazin-1-yl)-2-isopropoxy-5-methylphenyl)amino)-5-(trifluoromethyl)pyridin-4-yl)amino)-N-methylbenzamide;
2-((2-((4-(4-((4-(2,6-dioxopiperidin-3-yl)benzyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-((4-(2,6-dioxopiperidin-3-yl)-3-fluorobenzyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-((3-(2,6-dioxopiperidin-3-yl)benzyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-((2-(2,6-dioxopiperidin-3-yl)benzyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-((4-(2,4-dioxotetrahydropyrimidin-1(2H)-yl)benzyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-((4-(2,4-dioxotetrahydropyrimidin-1(2H)-yl)-2-fluorobenzyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-((3-(2,4-dioxotetrahydropyrimidin-1(2H)-yl)benzyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-((2-(2,4-dioxotetrahydropyrimidin-1(2H)-yl)benzyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-(((5-(2,4-dioxotetrahydropyrimidin-1(2H)-yl)pyridin-2-yl)methyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-(((6-(2,4-dioxotetrahydropyrimidin-1(2H)-yl)pyridazin-3-yl)methyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-(((3-(2,4-dioxotetrahydropyrimidin-1(2H)-yl)pyridin-4-yl)methyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-((4-((2,6-dioxopiperidin-3-yl)amino)benzyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-((3-((2,6-dioxopiperidin-3-yl)amino)benzyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-((2-((2,6-dioxopiperidin-3-yl)amino)benzyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-(1-(4-(2,6-dioxopiperidin-3-yl)benzyl)piperidin-4-yl)piperazin-1-yl)-2-isopropoxy-5-methylphenyl)amino)-5-(trifluoromethyl)pyridin-4-yl)amino)-N-methylbenzamide;
2-((2-((4-(4-(1-(4-(2,6-dioxopiperidin-3-yl)-3-fluorobenzyl)piperidin-4-yl)piperazin-1-yl)-2-isopropoxy-5-methylphenyl)amino)-5-(trifluoromethyl)pyridin-4-yl)amino)-N-methylbenzamide;
2-((2-((4-(4-(1-(3-(2,6-dioxopiperidin-3-yl)benzyl)piperidin-4-yl)piperazin-1-yl)-2-isopropoxy-5-methylphenyl)amino)-5-(trifluoromethyl)pyridin-4-yl)amino)-N-methylbenzamide;
2-((2-((4-(4-(1-(2-(2,6-dioxopiperidin-3-yl)benzyl)piperidin-4-yl)piperazin-1-yl)-2-isopropoxy-5-methylphenyl)amino)-5-(trifluoromethyl)pyridin-4-yl)amino)-N-methylbenzamide;
2-((2-((4-(4-(1-(4-(2,4-dioxotetrahydropyrimidin-1(2H)-yl)benzyl)piperidin-4-yl)piperazin-1-yl)-2-isopropoxy-5-methylphenyl)amino)-5-(trifluoromethyl)pyridin-4-yl)amino)-N-methylbenzamide;
2-((2-((4-(4-(1-(4-(2,4-dioxotetrahydropyrimidin-1(2H)-yl)-3-fluorobenzyl)piperidin-4-yl)piperazin-1-yl)-2-isopropoxy-5-methylphenyl)amino)-5-(trifluoromethyl)pyridin-4-yl)amino)-N-methylbenzamide;
2-((2-((4-(4-(1-(3-(2,4-dioxotetrahydropyrimidin-1(2H)-yl)benzyl)piperidin-4-yl)piperazin-1-yl)-2-isopropoxy-5-methylphenyl)amino)-5-(trifluoromethyl)pyridin-4-yl)amino)-N-methylbenzamide;
2-((2-((4-(4-(1-(2-(2,4-dioxotetrahydropyrimidin-1(2H)-yl)benzyl)piperidin-4-yl)piperazin-1-yl)-2-isopropoxy-5-methylphenyl)amino)-5-(trifluoromethyl)pyridin-4-yl)amino)-N-methylbenzamide;
2-((2-((4-(4-(1-(4-((2,6-dioxopiperidin-3-yl)amino)benzyl)piperidin-4-yl)piperazin-1-yl)-2-isopropoxy-5-methylphenyl)amino)-5-(trifluoromethyl)pyridin-4-yl)amino)-N-methylbenzamide;
2-((2-((4-(4-(1-(3-((2,6-dioxopiperidin-3-yl)amino)benzyl)piperidin-4-yl)piperazin-1-yl)-2-isopropoxy-5-methylphenyl)amino)-5-(trifluoromethyl)pyridin-4-yl)amino)-N-methylbenzamide;
2-((2-((4-(4-(1-(2-((2,6-dioxopiperidin-3-yl)amino)benzyl)piperidin-4-yl)piperazin-1-yl)-2-isopropoxy-5-methylphenyl)amino)-5-(trifluoromethyl)pyridin-4-yl)amino)-N-methylbenzamide;
N-(1-(4-(2,6-dioxopiperidin-3-yl)benzyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-(4-(2,6-dioxopiperidin-3-yl)-3-fluorobenzyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-(3-(2,6-dioxopiperidin-3-yl)benzyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-(2-(2,6-dioxopiperidin-3-yl)benzyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-(4-(2,4-dioxotetrahydropyrimidin-1(2H)-yl)benzyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-(4-(2,4-dioxotetrahydropyrimidin-1(2H)-yl)-3-fluorobenzyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-(3-(2,4-dioxotetrahydropyrimidin-1(2H)-yl)benzyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-(2-(2,4-dioxotetrahydropyrimidin-1(2H)-yl)benzyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-((5-(2,4-dioxotetrahydropyrimidin-1(2H)-yl)pyridin-2-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-((6-(2,4-dioxotetrahydropyrimidin-1(2H)-yl)pyridazin-3-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-((3-(2,4-dioxotetrahydropyrimidin-1(2H)-yl)pyridin-4-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-(4-((2,6-dioxopiperidin-3-yl)amino)benzyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-(3-((2,6-dioxopiperidin-3-yl)amino)benzyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-(2-((2,6-dioxopiperidin-3-yl)amino)benzyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(3-(((2-((4-(4-(4-(2,6-dioxopiperidin-3-yl)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(2,6-dioxopiperidin-3-yl)-3-fluorobenzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(3-(2,6-dioxopiperidin-3-yl)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(2-(2,6-dioxopiperidin-3-yl)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((5-(2,6-dioxopiperidin-3-yl)pyridin-2-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)pyridin-3-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((5-(2,6-dioxopiperidin-3-yl)pyridin-3-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((5-(2,6-dioxopiperidin-3-yl)-2-fluoropyridin-3-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((6-(2,6-dioxopiperidin-3-yl)pyridin-3-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(2,4-dioxotetrahydropyrimidin-1(2H)-yl)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(2,4-dioxotetrahydropyrimidin-1(2H)-yl)-2-fluorobenzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(3-(2,4-dioxotetrahydropyrimidin-1(2H)-yl)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(2-(2,4-dioxotetrahydropyrimidin-1(2H)-yl)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((5-(2,4-dioxotetrahydropyrimidin-1(2H)-yl)pyridin-2-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,4-dioxotetrahydropyrimidin-1(2H)-yl)pyridin-4-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,4-dioxotetrahydropyrimidin-1(2H)-yl)-5-fluoropyridin-4-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((6-(2,4-dioxotetrahydropyrimidin-1(2H)-yl)pyridin-3-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((6-(2,4-dioxotetrahydropyrimidin-1(2H)-yl)pyridazin-3-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((3-(2,4-dioxotetrahydropyrimidin-1(2H)-yl)pyridin-4-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((2,6-dioxopiperidin-3-yl)amino)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(3-((2,6-dioxopiperidin-3-yl)amino)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(2-((2,6-dioxopiperidin-3-yl)amino)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((4-(2,6-dioxopiperidin-3-yl)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((4-(2,6-dioxopiperidin-3-yl)-3-fluorobenzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((3-(2,6-dioxopiperidin-3-yl)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((4-(2,4-dioxotetrahydropyrimidin-1(2H)-yl)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((4-(2,4-dioxotetrahydropyrimidin-1(2H)-yl)-2-fluorobenzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((3-(2,4-dioxotetrahydropyrimidin-1(2H)-yl)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,4-dioxotetrahydropyrimidin-1(2H)-yl)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((5-(2,4-dioxotetrahydropyrimidin-1(2H)-yl)pyridin-2-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((6-(2,4-dioxotetrahydropyrimidin-1(2H)-yl)pyridazin-3-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((3-(2,4-dioxotetrahydropyrimidin-1(2H)-yl)pyridin-4-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((4-((2,6-dioxopiperidin-3-yl)amino)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((3-((2,6-dioxopiperidin-3-yl)amino)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-((2,6-dioxopiperidin-3-yl)amino)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(4-(2,6-dioxopiperidin-3-yl)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(4-(2,6-dioxopiperidin-3-yl)-3-fluorobenzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(3-(2,6-dioxopiperidin-3-yl)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(2-(2,6-dioxopiperidin-3-yl)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(4-(2,4-dioxotetrahydropyrimidin-1(2H)-yl)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(4-(2,4-dioxotetrahydropyrimidin-1(2H)-yl)-2-fluorobenzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(3-(2,4-dioxotetrahydropyrimidin-1(2H)-yl)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(2-(2,4-dioxotetrahydropyrimidin-1(2H)-yl)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((5-(2,4-dioxotetrahydropyrimidin-1(2H)-yl)pyridin-2-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((6-(2,4-dioxotetrahydropyrimidin-1(2H)-yl)pyridazin-3-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((3-(2,4-dioxotetrahydropyrimidin-1(2H)-yl)pyridin-4-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(4-((2,6-dioxopiperidin-3-yl)amino)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(3-((2,6-dioxopiperidin-3-yl)amino)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(2-((2,6-dioxopiperidin-3-yl)amino)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(2,6-dioxopiperidin-3-yl)benzyl)piperazin-1-yl)phenyl)amino)-5-(trifluoromethyl)pyrimidin-4-yl)amino)methyl)phenyl)-N-methylmethanesulfonamide;
N-(3-(((2-((4-(4-(4-(2,6-dioxopiperidin-3-yl)-3-fluorobenzyl)piperazin-1-yl)phenyl)amino)-5-(trifluoromethyl)pyrimidin-4-yl)amino)methyl)phenyl)-N-methylmethanesulfonamide;
N-(3-(((2-((4-(4-(3-(2,6-dioxopiperidin-3-yl)benzyl)piperazin-1-yl)phenyl)amino)-5-(trifluoromethyl)pyrimidin-4-yl)amino)methyl)phenyl)-N-methylmethanesulfonamide;
N-(3-(((2-((4-(4-(2-(2,6-dioxopiperidin-3-yl)benzyl)piperazin-1-yl)phenyl)amino)-5-(trifluoromethyl)pyrimidin-4-yl)amino)methyl)phenyl)-N-methylmethanesulfonamide;
3-(5-((4-(4-((4-((3-(methylsulfonyl)benzyl)amino)-5-(trifluoromethyl)pyrimidin-2-yl)amino)phenyl)piperazin-1-yl)methyl)pyridin-2-yl)piperidine-2,6-dione;
N-(3-(((2-((4-(4-(4-(2,4-dioxotetrahydropyrimidin-1(2H)-yl)benzyl)piperazin-1-yl)phenyl)amino)-5-(trifluoromethyl)pyrimidin-4-yl)amino)methyl)phenyl)-N-methylmethanesulfonamide;
N-(3-(((2-((4-(4-(4-(2,4-dioxotetrahydropyrimidin-1(2H)-yl)-2-fluorobenzyl)piperazin-1-yl)phenyl)amino)-5-(trifluoromethyl)pyrimidin-4-yl)amino)methyl)phenyl)-N-methylmethanesulfonamide;
N-(3-(((2-((4-(4-(3-(2,6-dioxopiperidin-3-yl)benzyl)piperazin-1-yl)phenyl)amino)-5-(trifluoromethyl)pyrimidin-4-yl)amino)methyl)phenyl)-N-methylmethanesulfonamide;
N-(3-(((2-((4-(4-(2-(2,6-dioxopiperidin-3-yl)benzyl)piperazin-1-yl)phenyl)amino)-5-(trifluoromethyl)pyrimidin-4-yl)amino)methyl)phenyl)-N-methylmethanesulfonamide;
N-(3-(((2-((4-(4-((5-(2,4-dioxotetrahydropyrimidin-1(2H)-yl)pyridin-2-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-((6-(2,4-dioxotetrahydropyrimidin-1(2H)-yl)pyridazin-3-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-((3-(2,4-dioxotetrahydropyrimidin-1(2H)-yl)pyridin-4-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-(4-((2,6-dioxopiperidin-3-yl)amino)benzyl)piperazin-1-yl)phenyl)amino)-5-(trifluoromethyl)pyrimidin-4-yl)amino)methyl)phenyl)-N-methylmethanesulfonamide;
N-(3-(((2-((4-(4-(3-((2,6-dioxopiperidin-3-yl)amino)benzyl)piperazin-1-yl)phenyl)amino)-5-(trifluoromethyl)pyrimidin-4-yl)amino)methyl)phenyl)-N-methylmethanesulfonamide;
N-(3-(((2-((4-(4-(2-((2,6-dioxopiperidin-3-yl)amino)benzyl)piperazin-1-yl)phenyl)amino)-5-(trifluoromethyl)pyrimidin-4-yl)amino)methyl)phenyl)-N-methylmethanesulfonamide;
N-(3-(((2-((4-(4-((4-(2,6-dioxopiperidin-3-yl)benzyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-((4-(2,6-dioxopiperidin-3-yl)-3-fluorobenzyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-((3-(2,6-dioxopiperidin-3-yl)benzyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-((2-(2,6-dioxopiperidin-3-yl)benzyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-((4-(2,4-dioxotetrahydropyrimidin-1(2H)-yl)benzyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-((4-(2,4-dioxotetrahydropyrimidin-1(2H)-yl)-2-fluorobenzyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-((3-(2,4-dioxotetrahydropyrimidin-1(2H)-yl)benzyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-((2-(2,4-dioxotetrahydropyrimidin-1(2H)-yl)benzyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(((5-(2,4-dioxotetrahydropyrimidin-1(2H)-yl)pyridin-2-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(((6-(2,4-dioxotetrahydropyrimidin-1(2H)-yl)pyridazin-3-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(((3-(2,4-dioxotetrahydropyrimidin-1(2H)-yl)pyridin-4-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-((4-((2,6-dioxopiperidin-3-yl)amino)benzyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-((3-((2,6-dioxopiperidin-3-yl)amino)benzyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-((2-((2,6-dioxopiperidin-3-yl)amino)benzyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-(4-(2,6-dioxopiperidin-3-yl)benzyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-(4-(2,6-dioxopiperidin-3-yl)-3-fluorobenzyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-(3-(2,6-dioxopiperidin-3-yl)benzyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-(2-(2,6-dioxopiperidin-3-yl)benzyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-(4-(2,4-dioxotetrahydropyrimidin-1(2H)-yl)benzyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-(4-(2,4-dioxotetrahydropyrimidin-1(2H)-yl)-2-fluorobenzyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-(3-(2,4-dioxotetrahydropyrimidin-1(2H)-yl)benzyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-(2-(2,4-dioxotetrahydropyrimidin-1(2H)-yl)benzyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((5-(2,4-dioxotetrahydropyrimidin-1(2H)-yl)pyridin-2-yl)methyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((6-(2,4-dioxotetrahydropyrimidin-1(2H)-yl)pyridazin-3-yl)methyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((3-(2,4-dioxotetrahydropyrimidin-1(2H)-yl)pyridin-4-yl)methyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-(4-((2,6-dioxopiperidin-3-yl)amino)benzyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-(3-((2,6-dioxopiperidin-3-yl)amino)benzyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-(2-((2,6-dioxopiperidin-3-yl)amino)benzyl)piperazin-1-yl)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-(2,6-dioxopiperidin-3-yl)benzyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-(2,6-dioxopiperidin-3-yl)-3-fluorobenzyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-(3-(2,6-dioxopiperidin-3-yl)benzyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-(2-(2,6-dioxopiperidin-3-yl)benzyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-((2-(2,6-dioxopiperidin-3-yl)pyridin-4-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(2,4-dioxotetrahydropyrimidin-1(2H)-yl)benzyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-(2,4-dioxotetrahydropyrimidin-1(2H)-yl)-2-fluorobenzyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-(3-(2,4-dioxotetrahydropyrimidin-1(2H)-yl)benzyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-(2-(2,4-dioxotetrahydropyrimidin-1(2H)-yl)benzyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-((5-(2,4-dioxotetrahydropyrimidin-1(2H)-yl)pyridin-2-yl)methyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-((6-(2,4-dioxotetrahydropyrimidin-1(2H)-yl)pyridazin-3-yl)methyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-((3-(2,4-dioxotetrahydropyrimidin-1(2H)-yl)pyridin-4-yl)methyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-((2,6-dioxopiperidin-3-yl)amino)benzyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-(3-((2,6-dioxopiperidin-3-yl)amino)benzyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-(2-((2,6-dioxopiperidin-3-yl)amino)benzyl)piperazin-1-yl)phenyl)amino)-5-(trifluoromethyl)pyrimidin-4-yl)amino)methyl)pyridin-2-yl)-N-methylmethanesulfonamide;
N-(3-(((2-((4-(4-((4-(2,6-dioxopiperidin-3-yl)benzyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-((4-(2,6-dioxopiperidin-3-yl)-3-fluorobenzyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-((3-(2,6-dioxopiperidin-3-yl)benzyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-((2-(2,6-dioxopiperidin-3-yl)benzyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-((4-(2,4-dioxotetrahydropyrimidin-1(2H)-yl)benzyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-((4-(2,4-dioxotetrahydropyrimidin-1(2H)-yl)-2-fluorobenzyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-((3-(2,4-dioxotetrahydropyrimidin-1(2H)-yl)benzyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-((2-(2,4-dioxotetrahydropyrimidin-1(2H)-yl)benzyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(((5-(2,4-dioxotetrahydropyrimidin-1(2H)-yl)pyridin-2-yl)methyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-((5-(2,4-dioxotetrahydropyrimidin-1(2H)-yl)pyridin-3-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((6-(2,4-dioxotetrahydropyrimidin-1(2H)-yl)pyridazin-3-yl)methyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(((3-(2,4-dioxotetrahydropyrimidin-1(2H)-yl)pyridin-4-yl)methyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-((4-((2,6-dioxopiperidin-3-yl)amino)benzyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-((3-((2,6-dioxopiperidin-3-yl)amino)benzyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-((2-((2,6-dioxopiperidin-3-yl)amino)benzyl)(methyl)amino)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-(4-(2,6-dioxopiperidin-3-yl)benzyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-(4-(2,6-dioxopiperidin-3-yl)-3-fluorobenzyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-(3-(2,6-dioxopiperidin-3-yl)benzyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-(2-(2,6-dioxopiperidin-3-yl)benzyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-(4-(2,4-dioxotetrahydropyrimidin-1(2H)-yl)benzyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-(4-(2,4-dioxotetrahydropyrimidin-1(2H)-yl)-2-fluorobenzyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-(3-(2,4-dioxotetrahydropyrimidin-1(2H)-yl)benzyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-(2-(2,4-dioxotetrahydropyrimidin-1(2H)-yl)benzyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-((5-(2,4-dioxotetrahydropyrimidin-1(2H)-yl)pyridin-2-yl)methyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-((6-(2,4-dioxotetrahydropyrimidin-1(2H)-yl)pyridazin-3-yl)methyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-((3-(2,4-dioxotetrahydropyrimidin-1(2H)-yl)pyridin-4-yl)methyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-(4-((2,6-dioxopiperidin-3-yl)amino)benzyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-(3-((2,6-dioxopiperidin-3-yl)amino)benzyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-(2-((2,6-dioxopiperidin-3-yl)amino)benzyl)piperazin-1-yl)piperidin-1-yl)phenyl)amino)-5-(trifluoromethyl)pyrimidin-4-yl)amino)methyl)pyridin-2-yl)-N-methylmethanesulfonamide;
N-(3-(((2-((4-(4-(4-(2,6-dioxopiperidin-3-yl)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(2,6-dioxopiperidin-3-yl)-3-fluorobenzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(3-(2,6-dioxopiperidin-3-yl)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(2-(2,6-dioxopiperidin-3-yl)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(2,4-dioxotetrahydropyrimidin-1(2H)-yl)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(2,4-dioxotetrahydropyrimidin-1(2H)-yl)-2-fluorobenzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(3-(2,4-dioxotetrahydropyrimidin-1(2H)-yl)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(2-(2,4-dioxotetrahydropyrimidin-1(2H)-yl)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((5-(2,4-dioxotetrahydropyrimidin-1(2H)-yl)pyridin-2-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((6-(2,4-dioxotetrahydropyrimidin-1(2H)-yl)pyridazin-3-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((3-(2,4-dioxotetrahydropyrimidin-1(2H)-yl)pyridin-4-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((2,6-dioxopiperidin-3-yl)amino)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(3-((2,6-dioxopiperidin-3-yl)amino)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(2-((2,6-dioxopiperidin-3-yl)amino)benzyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((4-(2,6-dioxopiperidin-3-yl)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((4-(2,6-dioxopiperidin-3-yl)-3-fluorobenzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((3-(2,6-dioxopiperidin-3-yl)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((4-(2,4-dioxotetrahydropyrimidin-1(2H)-yl)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((4-(2,4-dioxotetrahydropyrimidin-1(2H)-yl)-2-fluorobenzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((3-(2,4-dioxotetrahydropyrimidin-1(2H)-yl)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,4-dioxotetrahydropyrimidin-1(2H)-yl)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((5-(2,4-dioxotetrahydropyrimidin-1(2H)-yl)pyridin-2-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((6-(2,4-dioxotetrahydropyrimidin-1(2H)-yl)pyridazin-3-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((3-(2,4-dioxotetrahydropyrimidin-1(2H)-yl)pyridin-4-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((4-((2,6-dioxopiperidin-3-yl)amino)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((3-((2,6-dioxopiperidin-3-yl)amino)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-((2,6-dioxopiperidin-3-yl)amino)benzyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(4-(2,6-dioxopiperidin-3-yl)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(4-(2,6-dioxopiperidin-3-yl)-3-fluorobenzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(3-(2,6-dioxopiperidin-3-yl)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(2-(2,6-dioxopiperidin-3-yl)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(4-(2,4-dioxotetrahydropyrimidin-1(2H)-yl)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(4-(2,4-dioxotetrahydropyrimidin-1(2H)-yl)-2-fluorobenzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(3-(2,4-dioxotetrahydropyrimidin-1(2H)-yl)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(2-(2,4-dioxotetrahydropyrimidin-1(2H)-yl)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((5-(2,4-dioxotetrahydropyrimidin-1(2H)-yl)pyridin-2-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((6-(2,4-dioxotetrahydropyrimidin-1(2H)-yl)pyridazin-3-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((3-(2,4-dioxotetrahydropyrimidin-1(2H)-yl)pyridin-4-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(4-((2,6-dioxopiperidin-3-yl)amino)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(3-((2,6-dioxopiperidin-3-yl)amino)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-(2-((2,6-dioxopiperidin-3-yl)amino)benzyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(1-(4-(2,6-dioxopiperidin-3-yl)benzyl)piperidin-4-yl)phenyl)amino)-5-(trifluoromethyl)pyrimidin-4-yl)amino)methyl)phenyl)-N-methylmethanesulfonamide;
3-(2-fluoro-4-((4-(4-((4-((3-(methylsulfonyl)benzyl)amino)-5-(trifluoromethyl)pyrimidin-2-yl)amino)phenyl)piperidin-1-yl)methyl)phenyl)piperidine-2,6-dione;
3-(3-((4-(4-((4-((3-(methylsulfonyl)benzyl)amino)-5-(trifluoromethyl)pyrimidin-2-yl)amino)phenyl)piperidin-1-yl)methyl)phenyl)piperidine-2,6-dione;
3-(2-((4-(4-((4-((3-(methylsulfonyl)benzyl)amino)-5-(trifluoromethyl)pyrimidin-2-yl)amino)phenyl)piperidin-1-yl)methyl)phenyl)piperidine-2,6-dione;
3-(4-((4-(4-((4-((3-(methylsulfonyl)benzyl)amino)-5-(trifluoromethyl)pyrimidin-2-yl)amino)phenyl)piperazin-1-yl)methyl)pyridin-2-yl)piperidine-2,6-dione;
3-(3-((4-(4-((4-((3-(methylsulfonyl)benzyl)amino)-5-(trifluoromethyl)pyrimidin-2-yl)amino)phenyl)piperazin-1-yl)methyl)pyridin-2-yl)piperidine-2,6-dione;
3-(5-((4-(4-((4-((3-(methylsulfonyl)benzyl)amino)-5-(trifluoromethyl)pyrimidin-2-yl)amino)phenyl)piperazin-1-yl)methyl)pyridin-3-yl)piperidine-2,6-dione;
1-(4-((4-(4-((4-((3-(methylsulfonyl)benzyl)amino)-5-(trifluoromethyl)pyrimidin-2-yl)amino)phenyl)piperidin-1-yl)methyl)phenyl)dihydropyrimidine-2,4(1H,3H)-dione;
1-(3-fluoro-4-((4-(4-((4-((3-(methylsulfonyl)benzyl)amino)-5-(trifluoromethyl)pyrimidin-2-yl)amino)phenyl)piperidin-1-yl)methyl)phenyl)dihydropyrimidine-2,4(1H,3H)-dione;
1-(3-((4-(4-((4-((3-(methylsulfonyl)benzyl)amino)-5-(trifluoromethyl)pyrimidin-2-yl)amino)phenyl)piperidin-1-yl)methyl)phenyl)dihydropyrimidine-2,4(1H,3H)-dione;
1-(2-((4-(4-((4-((3-(methylsulfonyl)benzyl)amino)-5-(trifluoromethyl)pyrimidin-2-yl)amino)phenyl)piperidin-1-yl)methyl)phenyl)dihydropyrimidine-2,4(1H,3H)-dione;
1-(6-((4-(4-((4-((3-(methylsulfonyl)benzyl)amino)-5-(trifluoromethyl)pyrimidin-2-yl)amino)phenyl)piperidin-1-yl)methyl)pyridin-3-yl)dihydropyrimidine-2,4(1H,3H)-dione;
1-(5-((4-(4-((4-((3-(methylsulfonyl)benzyl)amino)-5-(trifluoromethyl)pyrimidin-2-yl)amino)phenyl)piperazin-1-yl)methyl)pyridin-3-yl)dihydropyrimidine-2,4(1H,3H)-dione;
3-(6-fluoro-5-((4-(4-((4-((3-(methylsulfonyl)benzyl)amino)-5-(trifluoromethyl)pyrimidin-2-yl)amino)phenyl)piperazin-1-yl)methyl)pyridin-3-yl)piperidine-2,6-dione;
1-(6-((4-(4-((4-((3-(methylsulfonyl)benzyl)amino)-5-(trifluoromethyl)pyrimidin-2-yl)amino)phenyl)piperidin-1-yl)methyl)pyridazin-3-yl)dihydropyrimidine-2,4(1H,3H)-dione;
1-(4-((4-(4-((4-((3-(methylsulfonyl)benzyl)amino)-5-(trifluoromethyl)pyrimidin-2-yl)amino)phenyl)piperidin-1-yl)methyl)pyridin-3-yl)dihydropyrimidine-2,4(1H,3H)-dione;
3-((4-((4-(4-((4-((3-(methylsulfonyl)benzyl)amino)-5-(trifluoromethyl)pyrimidin-2-yl)amino)phenyl)piperidin-1-yl)methyl)phenyl)amino)piperidine-2,6-dione;
3-((3-((4-(4-((4-((3-(methylsulfonyl)benzyl)amino)-5-(trifluoromethyl)pyrimidin-2-yl)amino)phenyl)piperidin-1-yl)methyl)phenyl)amino)piperidine-2,6-dione;
3-((2-((4-(4-((4-((3-(methylsulfonyl)benzyl)amino)-5-(trifluoromethyl)pyrimidin-2-yl)amino)phenyl)piperidin-1-yl)methyl)phenyl)amino)piperidine-2,6-dione;
3-(4-((4-(4-((4-((3-(methylsulfonyl)benzyl)amino)-5-(trifluoromethyl)pyrimidin-2-yl)amino)phenyl)piperazin-1-yl)methyl)phenyl)piperidine-2,6-dione;
3-(2-fluoro-4-((4-(4-((4-((3-(methylsulfonyl)benzyl)amino)-5-(trifluoromethyl)pyrimidin-2-yl)amino)phenyl)piperazin-1-yl)methyl)phenyl)piperidine-2,6-dione;
3-(3-((4-(4-((4-((3-(methylsulfonyl)benzyl)amino)-5-(trifluoromethyl)pyrimidin-2-yl)amino)phenyl)piperazin-1-yl)methyl)phenyl)piperidine-2,6-dione;
3-(2-((4-(4-((4-((3-(methylsulfonyl)benzyl)amino)-5-(trifluoromethyl)pyrimidin-2-yl)amino)phenyl)piperazin-1-yl)methyl)phenyl)piperidine-2,6-dione;
3-(6-((4-(4-((4-((3-(methylsulfonyl)benzyl)amino)-5-(trifluoromethyl)pyrimidin-2-yl)amino)phenyl)piperazin-1-yl)methyl)pyridin-3-yl)piperidine-2,6-dione;
1-(4-((4-(4-((4-((3-(methylsulfonyl)benzyl)amino)-5-(trifluoromethyl)pyrimidin-2-yl)amino)phenyl)piperazin-1-yl)methyl)phenyl)dihydropyrimidine-2,4(1H,3H)-dione;
1-(3-fluoro-4-((4-(4-((4-((3-(methylsulfonyl)benzyl)amino)-5-(trifluoromethyl)pyrimidin-2-yl)amino)phenyl)piperazin-1-yl)methyl)phenyl)dihydropyrimidine-2,4(1H,3H)-dione;
1-(3-((4-(4-((4-((3-(methylsulfonyl)benzyl)amino)-5-(trifluoromethyl)pyrimidin-2-yl)amino)phenyl)piperazin-1-yl)methyl)phenyl)dihydropyrimidine-2,4(1H,3H)-dione;
1-(2-((4-(4-((4-((3-(methylsulfonyl)benzyl)amino)-5-(trifluoromethyl)pyrimidin-2-yl)amino)phenyl)piperazin-1-yl)methyl)phenyl)dihydropyrimidine-2,4(1H,3H)-dione;
1-(6-((4-(4-((4-((3-(methylsulfonyl)benzyl)amino)-5-(trifluoromethyl)pyrimidin-2-yl)amino)phenyl)piperazin-1-yl)methyl)pyridin-3-yl)dihydropyrimidine-2,4(1H,3H)-dione;
1-(4-((4-(4-((4-((3-(methylsulfonyl)benzyl)amino)-5-(trifluoromethyl)pyrimidin-2-yl)amino)phenyl)piperazin-1-yl)methyl)pyridin-2-yl)dihydropyrimidine-2,4(1H,3H)-dione;
1-(5-fluoro-4-((4-(4-((4-((3-(methylsulfonyl)benzyl)amino)-5-(trifluoromethyl)pyrimidin-2-yl)amino)phenyl)piperazin-1-yl)methyl)pyridin-2-yl)dihydropyrimidine-2,4(1H,3H)-dione;
1-(5-((4-(4-((4-((3-(methylsulfonyl)benzyl)amino)-5-(trifluoromethyl)pyrimidin-2-yl)amino)phenyl)piperazin-1-yl)methyl)pyridin-2-yl)dihydropyrimidine-2,4(1H,3H)-dione;
1-(6-((4-(4-((4-((3-(methylsulfonyl)benzyl)amino)-5-(trifluoromethyl)pyrimidin-2-yl)amino)phenyl)piperazin-1-yl)methyl)pyridazin-3-yl)dihydropyrimidine-2,4(1H,3H)-dione;
1-(4-((4-(4-((4-((3-(methylsulfonyl)benzyl)amino)-5-(trifluoromethyl)pyrimidin-2-yl)amino)phenyl)piperazin-1-yl)methyl)pyridin-3-yl)dihydropyrimidine-2,4(1H,3H)-dione;
3-((4-((4-(4-((4-((3-(methylsulfonyl)benzyl)amino)-5-(trifluoromethyl)pyrimidin-2-yl)amino)phenyl)piperazin-1-yl)methyl)phenyl)amino)piperidine-2,6-dione;
3-((3-((4-(4-((4-((3-(methylsulfonyl)benzyl)amino)-5-(trifluoromethyl)pyrimidin-2-yl)amino)phenyl)piperazin-1-yl)methyl)phenyl)amino)piperidine-2,6-dione;
3-((2-((4-(4-((4-((3-(methylsulfonyl)benzyl)amino)-5-(trifluoromethyl)pyrimidin-2-yl)amino)phenyl)piperazin-1-yl)methyl)phenyl)amino)piperidine-2,6-dione;
2-(2-((cyclopropylmethyl)amino)pyridin-4-yl)-N-(3-(difluoromethyl)-1-(1-(4-(2,6-dioxopiperidin-3-yl)benzyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-(4-(2,6-dioxopiperidin-3-yl)-3-fluorobenzyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-(3-(2,6-dioxopiperidin-3-yl)benzyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-(2-(2,6-dioxopiperidin-3-yl)benzyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-(4-(2,4-dioxotetrahydropyrimidin-1(2H)-yl)benzyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-(4-(2,4-dioxotetrahydropyrimidin-1(2H)-yl)-2-fluorobenzyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-(3-(2,4-dioxotetrahydropyrimidin-1(2H)-yl)benzyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-(2-(2,4-dioxotetrahydropyrimidin-1(2H)-yl)benzyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((5-(2,4-dioxotetrahydropyrimidin-1(2H)-yl)pyridin-2-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((6-(2,4-dioxotetrahydropyrimidin-1(2H)-yl)pyridin-3-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((6-(2,4-dioxotetrahydropyrimidin-1(2H)-yl)pyridazin-3-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((4-(2,4-dioxotetrahydropyrimidin-1(2H)-yl)pyridin-3-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-(4-((2,6-dioxopiperidin-3-yl)amino)benzyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-(3-((2,6-dioxopiperidin-3-yl)amino)benzyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-(2-((2,6-dioxopiperidin-3-yl)amino)benzyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(4-(4-((6-(2,4-dioxotetrahydropyrimidin-1(2H)-yl)pyridin-3-yl)methyl)piperazin-1-yl)phenyl)-1H-pyrazol-4-yl)oxazole-4-carboxamide;
2-(2-((cyclopropylmethyl)amino)pyridin-4-yl)-N-(3-(difluoromethyl)-1-(4-(4-(3-(2,4-dioxotetrahydropyrimidin-1(2H)-yl)benzyl)piperazin-1-yl)phenyl)-1H-pyrazol-4-yl)oxazole-4-carboxamide;
2-(2-((cyclopropylmethyl)amino)pyridin-4-yl)-N-(3-(difluoromethyl)-1-(4-(4-(3-((2,6-dioxopiperidin-3-yl)amino)benzyl)piperazin-1-yl)phenyl)-1H-pyrazol-4-yl)oxazole-4-carboxamide;
N-(3-(difluoromethyl)-1-(1-(4-(2,6-dioxopiperidin-3-yl)benzyl)piperidin-4-yl)-1H-pyrazol-4-yl)-5-morpholinopyrazolo[1,5-a]pyrimidine-3-carboxamide;
N-(3-(difluoromethyl)-1-(1-(4-(2,6-dioxopiperidin-3-yl)-2-fluorobenzyl)piperidin-4-yl)-1H-pyrazol-4-yl)-5-morpholinopyrazolo[1,5-a]pyrimidine-3-carboxamide;
N-(3-(difluoromethyl)-1-(1-(3-(2,6-dioxopiperidin-3-yl)benzyl)piperidin-4-yl)-1H-pyrazol-4-yl)-5-morpholinopyrazolo[1,5-a]pyrimidine-3-carboxamide;
N-(3-(difluoromethyl)-1-(1-(2-(2,6-dioxopiperidin-3-yl)benzyl)piperidin-4-yl)-1H-pyrazol-4-yl)-5-morpholinopyrazolo[1,5-a]pyrimidine-3-carboxamide;
N-(3-(difluoromethyl)-1-(1-((2-(2,6-dioxopiperidin-3-yl)pyridin-4-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((5-(2,6-dioxopiperidin-3-yl)pyridin-2-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((2-(2,6-dioxopiperidin-3-yl)pyridin-3-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((5-(2,6-dioxopiperidin-3-yl)pyridin-3-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((5-(2,6-dioxopiperidin-3-yl)-2-fluoropyridin-3-yl)methyl)piperidin-4-yl)-1H-pyrazol-4-yl)-5-morpholinopyrazolo[1,5-a]pyrimidine-3-carboxamide;
N-(3-(difluoromethyl)-1-(1-(4-(2,4-dioxotetrahydropyrimidin-1(2H)-yl)benzyl)piperidin-4-yl)-1H-pyrazol-4-yl)-5-morpholinopyrazolo[1,5-a]pyrimidine-3-carboxamide;
N-(3-(difluoromethyl)-1-(1-(4-(2,4-dioxotetrahydropyrimidin-1(2H)-yl)-3-fluorobenzyl)piperidin-4-yl)-1H-pyrazol-4-yl)-5-morpholinopyrazolo[1,5-a]pyrimidine-3-carboxamide;
N-(3-(difluoromethyl)-1-(1-(3-(2,4-dioxotetrahydropyrimidin-1(2H)-yl)benzyl)piperidin-4-yl)-1H-pyrazol-4-yl)-5-morpholinopyrazolo[1,5-a]pyrimidine-3-carboxamide;
N-(3-(difluoromethyl)-1-(1-(2-(2,4-dioxotetrahydropyrimidin-1(2H)-yl)benzyl)piperidin-4-yl)-1H-pyrazol-4-yl)-5-morpholinopyrazolo[1,5-a]pyrimidine-3-carboxamide;
N-(3-(difluoromethyl)-1-(1-((5-(2,4-dioxotetrahydropyrimidin-1(2H)-yl)pyridin-2-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((2-(2,4-dioxotetrahydropyrimidin-1(2H)-yl)pyridin-4-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((2-(2,4-dioxotetrahydropyrimidin-1(2H)-yl)-5-fluoropyridin-4-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((6-(2,4-dioxotetrahydropyrimidin-1(2H)-yl)pyridin-3-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((5-(2,4-dioxotetrahydropyrimidin-1(2H)-yl)pyridin-3-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((5-(2,4-dioxotetrahydropyrimidin-1(2H)-yl)-2-fluoropyridin-3-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((6-(2,4-dioxotetrahydropyrimidin-1(2H)-yl)pyridazin-3-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((5-(2,4-dioxotetrahydropyrimidin-1(2H)-yl)pyrazin-2-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((3-(2,4-dioxotetrahydropyrimidin-1(2H)-yl)pyridin-4-yl)methyl)piperidin-4-yl)-1H-pyrazol-4-yl)-5-morpholinopyrazolo[1,5-a]pyrimidine-3-carboxamide;
N-(3-(difluoromethyl)-1-(1-(4-((2,6-dioxopiperidin-3-yl)amino)benzyl)piperidin-4-yl)-1H-pyrazol-4-yl)-5-morpholinopyrazolo[1,5-a]pyrimidine-3-carboxamide;
N-(3-(difluoromethyl)-1-(1-(3-((2,6-dioxopiperidin-3-yl)amino)benzyl)piperidin-4-yl)-1H-pyrazol-4-yl)-5-morpholinopyrazolo[1,5-a]pyrimidine-3-carboxamide;
N-(3-(difluoromethyl)-1-(1-(2-((2,6-dioxopiperidin-3-yl)amino)benzyl)piperidin-4-yl)-1H-pyrazol-4-yl)-5-morpholinopyrazolo[1,5-a]pyrimidine-3-carboxamide;
5-((1R,4R)-2-oxa-5-azabicyclo[2.2.1]heptan-5-yl)-N-(3-(difluoromethyl)-1-(1-(4-(2,6-dioxopiperidin-3-yl)benzyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-(4-(2,6-dioxopiperidin-3-yl)-2-fluorobenzyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-(3-(2,6-dioxopiperidin-3-yl)benzyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-(2-(2,6-dioxopiperidin-3-yl)benzyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((2-(2,6-dioxopiperidin-3-yl)pyridin-4-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((5-(2,6-dioxopiperidin-3-yl)pyridin-2-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((5-(2,6-dioxopiperidin-3-yl)pyridin-3-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((5-(2,6-dioxopiperidin-3-yl)-2-fluoropyridin-3-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-(4-(2,4-dioxotetrahydropyrimidin-1(2H)-yl)benzyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-(4-(2,4-dioxotetrahydropyrimidin-1(2H)-yl)-3-fluorobenzyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-(3-(2,4-dioxotetrahydropyrimidin-1(2H)-yl)benzyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-(2-(2,4-dioxotetrahydropyrimidin-1(2H)-yl)benzyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((5-(2,4-dioxotetrahydropyrimidin-1(2H)-yl)pyridin-2-yl)methyl)piperidin-4-yl)-1H-pyrazol-4-yl)pyrazolo[1,5-a]pyrimidine-3-carboxamide;
5-((1S,4S)-2-oxa-5-azabicyclo[2.2.1]heptan-5-yl)-N-(3-(difluoromethyl)-1-(1-((2-(2,4-dioxotetrahydropyrimidin-1(2H)-yl)pyridin-4-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((2-(2,4-dioxotetrahydropyrimidin-1(2H)-yl)-5-fluoropyridin-4-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((6-(2,4-dioxotetrahydropyrimidin-1(2H)-yl)pyridin-3-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((5-(2,4-dioxotetrahydropyrimidin-1(2H)-yl)pyridin-3-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((5-(2,4-dioxotetrahydropyrimidin-1(2H)-yl)-2-fluoropyridin-3-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((6-(2,4-dioxotetrahydropyrimidin-1(2H)-yl)pyridazin-3-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((5-(2,4-dioxotetrahydropyrimidin-1(2H)-yl)pyrazin-2-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((3-(2,4-dioxotetrahydropyrimidin-1(2H)-yl)pyridin-4-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-(4-((2,6-dioxopiperidin-3-yl)amino)benzyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-(3-((2,6-dioxopiperidin-3-yl)amino)benzyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-(2-((2,6-dioxopiperidin-3-yl)amino)benzyl)piperidin-4-yl)-1H-pyrazol-4-yl)pyrazolo[1,5-a]pyrimidine-3-carboxamide;
N-(5-((4-(4-(2,6-dioxopiperidin-3-yl)benzyl)piperazin-1-yl)methyl)-1-((1s,4s)-4-(hydroxymethyl)cyclohexyl)-1H-benzo[d]imidazol-2-yl)-3-(trifluoromethyl)benzamide;
N-(5-((4-(4-(2,6-dioxopiperidin-3-yl)-3-fluorobenzyl)piperazin-1-yl)methyl)-1-((1s,4s)-4-(hydroxymethyl)cyclohexyl)-1H-benzo[d]imidazol-2-yl)-3-(trifluoromethyl)benzamide;
N-(5-((4-(3-(2,6-dioxopiperidin-3-yl)benzyl)piperazin-1-yl)methyl)-1-((1s,4s)-4-(hydroxymethyl)cyclohexyl)-1H-benzo[d]imidazol-2-yl)-3-(trifluoromethyl)benzamide;
N-(5-((4-(2-(2,6-dioxopiperidin-3-yl)benzyl)piperazin-1-yl)methyl)-1-((1s,4s)-4-(hydroxymethyl)cyclohexyl)-1H-benzo[d]imidazol-2-yl)-3-(trifluoromethyl)benzamide;
N-(5-((4-(4-(2,4-dioxotetrahydropyrimidin-1(2H)-yl)benzyl)piperazin-1-yl)methyl)-1-((1s,4s)-4-(hydroxymethyl)cyclohexyl)-1H-benzo[d]imidazol-2-yl)-3-(trifluoromethyl)benzamide;
N-(5-((4-(4-(2,4-dioxotetrahydropyrimidin-1(2H)-yl)-3-fluorobenzyl)piperazin-1-yl)methyl)-1-((1s,4s)-4-(hydroxymethyl)cyclohexyl)-1H-benzo[d]imidazol-2-yl)-3-(trifluoromethyl)benzamide;
N-(5-((4-(3-(2,4-dioxotetrahydropyrimidin-1(2H)-yl)benzyl)piperazin-1-yl)methyl)-1-((1s,4s)-4-(hydroxymethyl)cyclohexyl)-1H-benzo[d]imidazol-2-yl)-3-(trifluoromethyl)benzamide;
N-(5-((4-(2-(2,4-dioxotetrahydropyrimidin-1(2H)-yl)benzyl)piperazin-1-yl)methyl)-1-((1s,4s)-4-(hydroxymethyl)cyclohexyl)-1H-benzo[d]imidazol-2-yl)-3-(trifluoromethyl)benzamide;
N-(5-((4-((5-(2,4-dioxotetrahydropyrimidin-1(2H)-yl)pyridin-2-yl)methyl)piperazin-1-yl)methyl)-1-((1s,4s)-4-(hydroxymethyl)cyclohexyl)-1H-benzo[d]imidazol-2-yl)-3-(trifluoromethyl)benzamide;
N-(5-((4-((6-(2,4-dioxotetrahydropyrimidin-1(2H)-yl)pyridazin-3-yl)methyl)piperazin-1-yl)methyl)-1-((1s,4s)-4-(hydroxymethyl)cyclohexyl)-1H-benzo[d]imidazol-2-yl)-3-(trifluoromethyl)benzamide;
N-(5-((4-((3-(2,4-dioxotetrahydropyrimidin-1(2H)-yl)pyridin-4-yl)methyl)piperazin-1-yl)methyl)-1-((1s,4s)-4-(hydroxymethyl)cyclohexyl)-1H-benzo[d]imidazol-2-yl)-3-(trifluoromethyl)benzamide;
N-(5-((4-(4-((2,6-dioxopiperidin-3-yl)amino)benzyl)piperazin-1-yl)methyl)-1-((1s,4s)-4-(hydroxymethyl)cyclohexyl)-1H-benzo[d]imidazol-2-yl)-3-(trifluoromethyl)benzamide;
N-(5-((4-(3-((2,6-dioxopiperidin-3-yl)amino)benzyl)piperazin-1-yl)methyl)-1-((1s,4s)-4-(hydroxymethyl)cyclohexyl)-1H-benzo[d]imidazol-2-yl)-3-(trifluoromethyl)benzamide;
N-(5-((4-(2-((2,6-dioxopiperidin-3-yl)amino)benzyl)piperazin-1-yl)methyl)-1-((1s,4s)-4-(hydroxymethyl)cyclohexyl)-1H-benzo[d]imidazol-2-yl)-3-(trifluoromethyl)benzamide;
N-(5-(4-(4-(2,6-dioxopiperidin-3-yl)benzyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(4-(2,6-dioxopiperidin-3-yl)-3-fluorobenzyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(3-(2,6-dioxopiperidin-3-yl)benzyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(2-(2,6-dioxopiperidin-3-yl)benzyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(4-(2,4-dioxotetrahydropyrimidin-1(2H)-yl)benzyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(4-(2,4-dioxotetrahydropyrimidin-1(2H)-yl)-3-fluorobenzyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(3-(2,4-dioxotetrahydropyrimidin-1(2H)-yl)benzyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(2-(2,4-dioxotetrahydropyrimidin-1(2H)-yl)benzyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-((5-(2,4-dioxotetrahydropyrimidin-1(2H)-yl)pyridin-2-yl)methyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-((6-(2,4-dioxotetrahydropyrimidin-1(2H)-yl)pyridin-3-yl)methyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-((6-(2,4-dioxotetrahydropyrimidin-1(2H)-yl)pyridazin-3-yl)methyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-((3-(2,4-dioxotetrahydropyrimidin-1(2H)-yl)pyridin-4-yl)methyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(4-((2,6-dioxopiperidin-3-yl)amino)benzyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(3-((2,6-dioxopiperidin-3-yl)amino)benzyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(2-((2,6-dioxopiperidin-3-yl)amino)benzyl)piperazin-1-yl)-1-((1s,4s)-4-(hydroxymethyl)cyclohexyl)-1H-benzo[d]imidazol-2-yl)-3-(trifluoromethyl)benzamide;
N-(6-(4-((4-(2,6-dioxopiperidin-3-yl)benzyl)(methyl)amino)piperidin-1-yl)-1-((1s,4s)-4-(hydroxymethyl)cyclohexyl)-1H-benzo[d]imidazol-2-yl)-3-(trifluoromethyl)benzamide;
N-(6-(4-((4-(2,6-dioxopiperidin-3-yl)-3-fluorobenzyl)(methyl)amino)piperidin-1-yl)-1-((1s,4s)-4-(hydroxymethyl)cyclohexyl)-1H-benzo[d]imidazol-2-yl)-3-(trifluoromethyl)benzamide;
N-(6-(4-((3-(2,6-dioxopiperidin-3-yl)benzyl)(methyl)amino)piperidin-1-yl)-1-((1s,4s)-4-(hydroxymethyl)cyclohexyl)-1H-benzo[d]imidazol-2-yl)-3-(trifluoromethyl)benzamide;
N-(6-(4-((2-(2,6-dioxopiperidin-3-yl)benzyl)(methyl)amino)piperidin-1-yl)-1-((1s,4s)-4-(hydroxymethyl)cyclohexyl)-1H-benzo[d]imidazol-2-yl)-3-(trifluoromethyl)benzamide;
N-(6-(4-((4-(2,4-dioxotetrahydropyrimidin-1(2H)-yl)benzyl)(methyl)amino)piperidin-1-yl)-1-((1s,4s)-4-(hydroxymethyl)cyclohexyl)-1H-benzo[d]imidazol-2-yl)-3-(trifluoromethyl)benzamide;
N-(6-(4-((4-(2,4-dioxotetrahydropyrimidin-1(2H)-yl)-2-fluorobenzyl)(methyl)amino)piperidin-1-yl)-1-((1s,4s)-4-(hydroxymethyl)cyclohexyl)-1H-benzo[d]imidazol-2-yl)-3-(trifluoromethyl)benzamide;
N-(6-(4-((3-(2,4-dioxotetrahydropyrimidin-1(2H)-yl)benzyl)(methyl)amino)piperidin-1-yl)-1-((1s,4s)-4-(hydroxymethyl)cyclohexyl)-1H-benzo[d]imidazol-2-yl)-3-(trifluoromethyl)benzamide;
N-(6-(4-((2-(2,4-dioxotetrahydropyrimidin-1(2H)-yl)benzyl)(methyl)amino)piperidin-1-yl)-1-((1s,4s)-4-(hydroxymethyl)cyclohexyl)-1H-benzo[d]imidazol-2-yl)-3-(trifluoromethyl)benzamide;
N-(6-(4-(((5-(2,4-dioxotetrahydropyrimidin-1(2H)-yl)pyridin-2-yl)methyl)(methyl)amino)piperidin-1-yl)-1-((1s,4s)-4-(hydroxymethyl)cyclohexyl)-1H-benzo[d]imidazol-2-yl)-3-(trifluoromethyl)benzamide;
N-(6-(4-(((6-(2,4-dioxotetrahydropyrimidin-1(2H)-yl)pyridazin-3-yl)methyl)(methyl)amino)piperidin-1-yl)-1-((1s,4s)-4-(hydroxymethyl)cyclohexyl)-1H-benzo[d]imidazol-2-yl)-3-(trifluoromethyl)benzamide;
N-(6-(4-(((3-(2,4-dioxotetrahydropyrimidin-1(2H)-yl)pyridin-4-yl)methyl)(methyl)amino)piperidin-1-yl)-1-((1s,4s)-4-(hydroxymethyl)cyclohexyl)-1H-benzo[d]imidazol-2-yl)-3-(trifluoromethyl)benzamide;
N-(6-(4-((4-((2,6-dioxopiperidin-3-yl)amino)benzyl)(methyl)amino)piperidin-1-yl)-1-((1s,4s)-4-(hydroxymethyl)cyclohexyl)-1H-benzo[d]imidazol-2-yl)-3-(trifluoromethyl)benzamide;
N-(6-(4-((3-((2,6-dioxopiperidin-3-yl)amino)benzyl)(methyl)amino)piperidin-1-yl)-1-((1s,4s)-4-(hydroxymethyl)cyclohexyl)-1H-benzo[d]imidazol-2-yl)-3-(trifluoromethyl)benzamide;
N-(6-(4-((2-((2,6-dioxopiperidin-3-yl)amino)benzyl)(methyl)amino)piperidin-1-yl)-1-((1s,4s)-4-(hydroxymethyl)cyclohexyl)-1H-benzo[d]imidazol-2-yl)-3-(trifluoromethyl)benzamide;
N-(5-(4-(1-(4-(2,6-dioxopiperidin-3-yl)benzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-(4-(2,6-dioxopiperidin-3-yl)-3-fluorobenzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-(3-(2,6-dioxopiperidin-3-yl)benzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-(2-(2,6-dioxopiperidin-3-yl)benzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-(4-(2,4-dioxotetrahydropyrimidin-1(2H)-yl)benzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-(4-(2,4-dioxotetrahydropyrimidin-1(2H)-yl)-2-fluorobenzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-(3-(2,4-dioxotetrahydropyrimidin-1(2H)-yl)benzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-(2-(2,4-dioxotetrahydropyrimidin-1(2H)-yl)benzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-((5-(2,4-dioxotetrahydropyrimidin-1(2H)-yl)pyridin-2-yl)methyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-((6-(2,4-dioxotetrahydropyrimidin-1(2H)-yl)pyridin-3-yl)methyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-((6-(2,4-dioxotetrahydropyrimidin-1(2H)-yl)pyridazin-3-yl)methyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-((3-(2,4-dioxotetrahydropyrimidin-1(2H)-yl)pyridin-4-yl)methyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-(4-((2,6-dioxopiperidin-3-yl)amino)benzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-(3-((2,6-dioxopiperidin-3-yl)amino)benzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-(2-((2,6-dioxopiperidin-3-yl)amino)benzyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
6-(5-cyano-1H-pyrrolo[2,3-b]pyridin-1-yl)-N-(1-(4-(2,6-dioxopiperidin-3-yl)benzyl)piperidin-4-yl)-4-(isopropylamino)nicotinamide;
6-(5-cyano-1H-pyrrolo[2,3-b]pyridin-1-yl)-N-(1-(4-(2,6-dioxopiperidin-3-yl)-3-fluorobenzyl)piperidin-4-yl)-4-(isopropylamino)nicotinamide;
6-(5-cyano-1H-pyrrolo[2,3-b]pyridin-1-yl)-N-(1-(3-(2,6-dioxopiperidin-3-yl)benzyl)piperidin-4-yl)-4-(isopropylamino)nicotinamide;
6-(5-cyano-1H-pyrrolo[2,3-b]pyridin-1-yl)-N-(1-(2-(2,6-dioxopiperidin-3-yl)benzyl)piperidin-4-yl)-4-(isopropylamino)nicotinamide;
6-(5-cyano-1H-pyrrolo[2,3-b]pyridin-1-yl)-N-(1-((2-(2,6-dioxopiperidin-3-yl)pyridin-4-yl)methyl)piperidin-4-yl)-4-(isopropylamino)nicotinamide;
6-(5-cyano-1H-pyrrolo[2,3-b]pyridin-1-yl)-N-(1-((5-(2,6-dioxopiperidin-3-yl)pyridin-2-yl)methyl)piperidin-4-yl)-4-(isopropylamino)nicotinamide;
6-(5-cyano-1H-pyrrolo[2,3-b]pyridin-1-yl)-N-(1-((2-(2,6-dioxopiperidin-3-yl)pyridin-3-yl)methyl)piperidin-4-yl)-4-(isopropylamino)nicotinamide;
6-(5-cyano-1H-pyrrolo[2,3-b]pyridin-1-yl)-N-(1-((5-(2,6-dioxopiperidin-3-yl)pyridin-3-yl)methyl)piperidin-4-yl)-4-(isopropylamino)nicotinamide;
6-(5-cyano-1H-pyrrolo[2,3-b]pyridin-1-yl)-N-(1-((5-(2,6-dioxopiperidin-3-yl)-2-fluoropyridin-3-yl)methyl)piperidin-4-yl)-4-(isopropylamino)nicotinamide;
6-(5-cyano-1H-pyrrolo[2,3-b]pyridin-1-yl)-N-(1-(4-(2,4-dioxotetrahydropyrimidin-1(2H)-yl)benzyl)piperidin-4-yl)-4-(isopropylamino)nicotinamide;
6-(5-cyano-1H-pyrrolo[2,3-b]pyridin-1-yl)-N-(1-(4-(2,4-dioxotetrahydropyrimidin-1(2H)-yl)-2-fluorobenzyl)piperidin-4-yl)-4-(isopropylamino)nicotinamide;
6-(5-cyano-1H-pyrrolo[2,3-b]pyridin-1-yl)-N-(1-(3-(2,4-dioxotetrahydropyrimidin-1(2H)-yl)benzyl)piperidin-4-yl)-4-(isopropylamino)nicotinamide;
6-(5-cyano-1H-pyrrolo[2,3-b]pyridin-1-yl)-N-(1-(2-(2,4-dioxotetrahydropyrimidin-1(2H)-yl)benzyl)piperidin-4-yl)-4-(isopropylamino)nicotinamide;
6-(5-cyano-1H-pyrrolo[2,3-b]pyridin-1-yl)-N-(1-((5-(2,4-dioxotetrahydropyrimidin-1(2H)-yl)pyridin-2-yl)methyl)piperidin-4-yl)-4-(isopropylamino)nicotinamide;
6-(5-cyano-1H-pyrrolo[2,3-b]pyridin-1-yl)-N-(1-((2-(2,4-dioxotetrahydropyrimidin-1(2H)-yl)pyridin-4-yl)methyl)piperidin-4-yl)-4-(isopropylamino)nicotinamide;
6-(5-cyano-1H-pyrrolo[2,3-b]pyridin-1-yl)-N-(1-((2-(2,4-dioxotetrahydropyrimidin-1(2H)-yl)-5-fluoropyridin-4-yl)methyl)piperidin-4-yl)-4-(isopropylamino)nicotinamide;
6-(5-cyano-1H-pyrrolo[2,3-b]pyridin-1-yl)-N-(1-((6-(2,4-dioxotetrahydropyrimidin-1(2H)-yl)pyridin-3-yl)methyl)piperidin-4-yl)-4-(isopropylamino)nicotinamide;
6-(5-cyano-1H-pyrrolo[2,3-b]pyridin-1-yl)-N-(1-((5-(2,4-dioxotetrahydropyrimidin-1(2H)-yl)pyridin-3-yl)methyl)piperidin-4-yl)-4-(isopropylamino)nicotinamide;
6-(5-cyano-1H-pyrrolo[2,3-b]pyridin-1-yl)-N-(1-((5-(2,4-dioxotetrahydropyrimidin-1(2H)-yl)-2-fluoropyridin-3-yl)methyl)piperidin-4-yl)-4-(isopropylamino)nicotinamide;
6-(5-cyano-1H-pyrrolo[2,3-b]pyridin-1-yl)-N-(1-((6-(2,4-dioxotetrahydropyrimidin-1(2H)-yl)pyridazin-3-yl)methyl)piperidin-4-yl)-4-(isopropylamino)nicotinamide;
6-(5-cyano-1H-pyrrolo[2,3-b]pyridin-1-yl)-N-(1-((5-(2,4-dioxotetrahydropyrimidin-1(2H)-yl)pyrazin-2-yl)methyl)piperidin-4-yl)-4-(isopropylamino)nicotinamide;
6-(5-cyano-1H-pyrrolo[2,3-b]pyridin-1-yl)-N-(1-(2-(2,4-dioxotetrahydropyrimidin-1(2H)-yl)benzyl)piperidin-4-yl)-4-(isopropylamino)nicotinamide;
6-(5-cyano-1H-pyrrolo[2,3-b]pyridin-1-yl)-N-(1-(4-((2,6-dioxopiperidin-3-yl)amino)benzyl)piperidin-4-yl)-4-(isopropylamino)nicotinamide;
6-(5-cyano-1H-pyrrolo[2,3-b]pyridin-1-yl)-N-(1-(3-((2,6-dioxopiperidin-3-yl)amino)benzyl)piperidin-4-yl)-4-(isopropylamino)nicotinamide;
6-(5-cyano-1H-pyrrolo[2,3-b]pyridin-1-yl)-N-(1-(2-((2,6-dioxopiperidin-3-yl)amino)benzyl)piperidin-4-yl)-4-(isopropylamino)nicotinamide;
N-(3-(difluoromethyl)-1-(1-(4-(2,6-dioxopiperidin-3-yl)benzyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-(4-(2,6-dioxopiperidin-3-yl)-3-fluorobenzyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-(3-(2,6-dioxopiperidin-3-yl)benzyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-(2-(2,6-dioxopiperidin-3-yl)benzyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-(4-(2,4-dioxotetrahydropyrimidin-1(2H)-yl)benzyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-(4-(2,4-dioxotetrahydropyrimidin-1(2H)-yl)-3-fluorobenzyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-(3-(2,4-dioxotetrahydropyrimidin-1(2H)-yl)benzyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-(2-(2,4-dioxotetrahydropyrimidin-1(2H)-yl)benzyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-((5-(2,4-dioxotetrahydropyrimidin-1(2H)-yl)pyridin-2-yl)methyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-((6-(2,4-dioxotetrahydropyrimidin-1(2H)-yl)pyridin-3-yl)methyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-((6-(2,4-dioxotetrahydropyrimidin-1 (2H)-yl)pyridazin-3-yl)methyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-((3-(2,4-dioxotetrahydropyrimidin-1(2H)-yl)pyridin-4-yl)methyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-(4-((2,6-dioxopiperidin-3-yl)amino)benzyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-(3-((2,6-dioxopiperidin-3-yl)amino)benzyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-(2-((2,6-dioxopiperidin-3-yl)amino)benzyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(4-(4-(4-(2,6-dioxopiperidin-3-yl)benzyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-(4-(2,6-dioxopiperidin-3-yl)-3-fluorobenzyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-(3-(2,6-dioxopiperidin-3-yl)benzyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-(2-(2,6-dioxopiperidin-3-yl)benzyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-(4-(2,4-dioxotetrahydropyrimidin-1(2H)-yl)benzyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-(4-(2,4-dioxotetrahydropyrimidin-1(2H)-yl)-2-fluorobenzyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-(3-(2,4-dioxotetrahydropyrimidin-1(2H)-yl)benzyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-(2-(2,4-dioxotetrahydropyrimidin-1(2H)-yl)benzyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-((5-(2,4-dioxotetrahydropyrimidin-1(2H)-yl)pyridin-2-yl)methyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-((6-(2,4-dioxotetrahydropyrimidin-1(2H)-yl)pyridazin-3-yl)methyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-((3-(2,4-dioxotetrahydropyrimidin-1(2H)-yl)pyridin-4-yl)methyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-(4-((2,6-dioxopiperidin-3-yl)amino)benzyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-(3-((2,6-dioxopiperidin-3-yl)amino)benzyl)piperazin-1-yl)phenyl)-1H-pyrazol-4-yl)-2-(2-((2,2,2-trifluoroethyl)amino)pyridin-4-yl)oxazole-4-carboxamide;
N-(3-(difluoromethyl)-1-(4-(4-(2-((2,6-dioxopiperidin-3-yl)amino)benzyl)piperazin-1-yl)phenyl)-1H-pyrazol-4-yl)-2-(2-((2,2,2-trifluoroethyl)amino)pyridin-4-yl)oxazole-4-carboxamide;
N-(2-(4-(4-(2,6-dioxopiperidin-3-yl)benzyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
N-(2-(4-(4-(2,6-dioxopiperidin-3-yl)-3-fluorobenzyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
N-(2-(4-(3-(2,6-dioxopiperidin-3-yl)benzyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
N-(2-(4-(2-(2,6-dioxopiperidin-3-yl)benzyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
(R)-N-(2-(4-(4-(2,4-dioxotetrahydropyrimidin-1(2H)-yl)benzyl)piperazin-1-yl)-5-(3-hydroxypyrrolidin-1-yl)oxazolo[4,5-b]pyridin-6-yl)-2-(2-methylpyridin-4-yl)oxazole-4-carboxamide;
(R)-N-(2-(4-(4-(2,4-dioxotetrahydropyrimidin-1(2H)-yl)-2-fluorobenzyl)piperazin-1-yl)-5-(3-hydroxypyrrolidin-1-yl)oxazolo[4,5-b]pyridin-6-yl)-2-(2-methylpyridin-4-yl)oxazole-4-carboxamide;
(R)-N-(2-(4-(3-(2,4-dioxotetrahydropyrimidin-1(2H)-yl)benzyl)piperazin-1-yl)-5-(3-hydroxypyrrolidin-1-yl)oxazolo[4,5-b]pyridin-6-yl)-2-(2-methylpyridin-4-yl)oxazole-4-carboxamide;
(R)-N-(2-(4-(2-(2,4-dioxotetrahydropyrimidin-1(2H)-yl)benzyl)piperazin-1-yl)-5-(3-hydroxypyrrolidin-1-yl)oxazolo[4,5-b]pyridin-6-yl)-2-(2-methylpyridin-4-yl)oxazole-4-carboxamide;
(R)-N-(2-(4-((5-(2,4-dioxotetrahydropyrimidin-1(2H)-yl)pyridin-2-yl)methyl)piperazin-1-yl)-5-(3-hydroxypyrrolidin-1-yl)oxazolo[4,5-b]pyridin-6-yl)-2-(2-methylpyridin-4-yl)oxazole-4-carboxamide;
(R)-N-(2-(4-((6-(2,4-dioxotetrahydropyrimidin-1(2H)-yl)pyridin-3-yl)methyl)piperazin-1-yl)-5-(3-hydroxypyrrolidin-1-yl)oxazolo[4,5-b]pyridin-6-yl)-2-(2-methylpyridin-4-yl)oxazole-4-carboxamide;
(R)-N-(2-(4-((6-(2,4-dioxotetrahydropyrimidin-1(2H)-yl)pyridazin-3-yl)methyl)piperazin-1-yl)-5-(3-hydroxypyrrolidin-1-yl)oxazolo[4,5-b]pyridin-6-yl)-2-(2-methylpyridin-4-yl)oxazole-4-carboxamide;
(R)-N-(2-(4-((3-(2,4-dioxotetrahydropyrimidin-1(2H)-yl)pyridin-4-yl)methyl)piperazin-1-yl)-5-(3-hydroxypyrrolidin-1-yl)oxazolo[4,5-b]pyridin-6-yl)-2-(2-methylpyridin-4-yl)oxazole-4-carboxamide;
N-(2-(4-(4-((2,6-dioxopiperidin-3-yl)amino)benzyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
N-(2-(4-(3-((2,6-dioxopiperidin-3-yl)amino)benzyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
N-(2-(4-(2-((2,6-dioxopiperidin-3-yl)amino)benzyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
4-((1-(4-(2,6-dioxopiperidin-3-yl)benzyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-(4-(2,6-dioxopiperidin-3-yl)-3-fluorobenzyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-(3-(2,6-dioxopiperidin-3-yl)benzyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-(2-(2,6-dioxopiperidin-3-yl)benzyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-(4-(2,4-dioxotetrahydropyrimidin-1(2H)-yl)benzyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-(4-(2,4-dioxotetrahydropyrimidin-1(2H)-yl)-2-fluorobenzyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-(3-(2,4-dioxotetrahydropyrimidin-1(2H)-yl)benzyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-(2-(2,4-dioxotetrahydropyrimidin-1(2H)-yl)benzyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-((5-(2,4-dioxotetrahydropyrimidin-1(2H)-yl)pyridin-2-yl)methyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-((6-(2,4-dioxotetrahydropyrimidin-1(2H)-yl)pyridazin-3-yl)methyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-((3-(2,4-dioxotetrahydropyrimidin-1(2H)-yl)pyridin-4-yl)methyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-(4-((2,6-dioxopiperidin-3-yl)amino)benzyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-(3-((2,6-dioxopiperidin-3-yl)amino)benzyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
4-((1-(2-((2,6-dioxopiperidin-3-yl)amino)benzyl)piperidin-4-yl)ethynyl)-1-(((2S,3S,4S)-3-ethyl-4-fluoro-5-oxopyrrolidin-2-yl)methoxy)-7-methoxyisoquinoline-6-carboxamide;
N-(2-(1-(4-(2,6-dioxopiperidin-3-yl)benzyl)piperidin-4-yl)-5-(2-hydroxypropan-2-yl)benzo[d]thiazol-6-yl)-6-(trifluoromethyl)picolinamide;
N-(2-(1-(4-(2,6-dioxopiperidin-3-yl)-3-fluorobenzyl)piperidin-4-yl)-5-(2-hydroxypropan-2-yl)benzo[d]thiazol-6-yl)-6-(trifluoromethyl)picolinamide;
N-(2-(1-(3-(2,6-dioxopiperidin-3-yl)benzyl)piperidin-4-yl)-5-(2-hydroxypropan-2-yl)benzo[d]thiazol-6-yl)-6-(trifluoromethyl)picolinamide;
N-(2-(1-(2-(2,6-dioxopiperidin-3-yl)benzyl)piperidin-4-yl)-5-(2-hydroxypropan-2-yl)benzo[d]thiazol-6-yl)-6-(trifluoromethyl)picolinamide;
N-(2-(1-((2-(2,6-dioxopiperidin-3-yl)pyridin-4-yl)methyl)piperidin-4-yl)-5-(2-hydroxypropan-2-yl)benzo[d]thiazol-6-yl)-6-(trifluoromethyl)picolinamide;
N-(2-(1-((5-(2,6-dioxopiperidin-3-yl)pyridin-2-yl)methyl)piperidin-4-yl)-5-(2-hydroxypropan-2-yl)benzo[d]thiazol-6-yl)-6-(trifluoromethyl)picolinamide;
N-(2-(1-((2-(2,6-dioxopiperidin-3-yl)pyridin-3-yl)methyl)piperidin-4-yl)-5-(2-hydroxypropan-2-yl)benzo[d]thiazol-6-yl)-6-(trifluoromethyl)picolinamide;
N-(2-(1-((5-(2,6-dioxopiperidin-3-yl)pyridin-3-yl)methyl)piperidin-4-yl)-5-(2-hydroxypropan-2-yl)benzo[d]thiazol-6-yl)-6-(trifluoromethyl)picolinamide;
N-(2-(1-((5-(2,6-dioxopiperidin-3-yl)-2-fluoropyridin-3-yl)methyl)piperidin-4-yl)-5-(2-hydroxypropan-2-yl)benzo[d]thiazol-6-yl)-6-(trifluoromethyl)picolinamide;
N-(2-(1-(4-(2,4-dioxotetrahydropyrimidin-1(2H)-yl)benzyl)piperidin-4-yl)-5-(2-hydroxypropan-2-yl)benzo[d]thiazol-6-yl)-6-(trifluoromethyl)picolinamide;
N-(2-(1-(4-(2,4-dioxotetrahydropyrimidin-1(2H)-yl)-2-fluorobenzyl)piperidin-4-yl)-5-(2-hydroxypropan-2-yl)benzo[d]thiazol-6-yl)-6-(trifluoromethyl)picolinamide;
N-(2-(1-(3-(2,4-dioxotetrahydropyrimidin-1(2H)-yl)benzyl)piperidin-4-yl)-5-(2-hydroxypropan-2-yl)benzo[d]thiazol-6-yl)-6-(trifluoromethyl)picolinamide;
N-(2-(1-(2-(2,4-dioxotetrahydropyrimidin-1(2H)-yl)benzyl)piperidin-4-yl)-5-(2-hydroxypropan-2-yl)benzo[d]thiazol-6-yl)-6-(trifluoromethyl)picolinamide;
N-(2-(1-((5-(2,4-dioxotetrahydropyrimidin-1(2H)-yl)pyridin-2-yl)methyl)piperidin-4-yl)-5-(2-hydroxypropan-2-yl)benzo[d]thiazol-6-yl)-6-(trifluoromethyl)picolinamide;
N-(2-(1-((2-(2,4-dioxotetrahydropyrimidin-1(2H)-yl)pyridin-4-yl)methyl)piperidin-4-yl)-5-(2-hydroxypropan-2-yl)benzo[d]thiazol-6-yl)-6-(trifluoromethyl)picolinamide;
N-(2-(1-((2-(2,4-dioxotetrahydropyrimidin-1(2H)-yl)-5-fluoropyridin-4-yl)methyl)piperidin-4-yl)-5-(2-hydroxypropan-2-yl)benzo[d]thiazol-6-yl)-6-(trifluoromethyl)picolinamide;
N-(2-(1-((6-(2,4-dioxotetrahydropyrimidin-1(2H)-yl)pyridin-3-yl)methyl)piperidin-4-yl)-5-(2-hydroxypropan-2-yl)benzo[d]thiazol-6-yl)-6-(trifluoromethyl)picolinamide;
N-(2-(1-((5-(2,4-dioxotetrahydropyrimidin-1(2H)-yl)pyridin-3-yl)methyl)piperidin-4-yl)-5-(2-hydroxypropan-2-yl)benzo[d]thiazol-6-yl)-6-(trifluoromethyl)picolinamide;
N-(2-(1-((5-(2,4-dioxotetrahydropyrimidin-1(2H)-yl)-2-fluoropyridin-3-yl)methyl)piperidin-4-yl)-5-(2-hydroxypropan-2-yl)benzo[d]thiazol-6-yl)-6-(trifluoromethyl)picolinamide;
N-(2-(1-((6-(2,4-dioxotetrahydropyrimidin-1(2H)-yl)pyridazin-3-yl)methyl)piperidin-4-yl)-5-(2-hydroxypropan-2-yl)benzo[d]thiazol-6-yl)-6-(trifluoromethyl)picolinamide;
N-(2-(1-((5-(2,4-dioxotetrahydropyrimidin-1(2H)-yl)pyrazin-2-yl)methyl)piperidin-4-yl)-5-(2-hydroxypropan-2-yl)benzo[d]thiazol-6-yl)-6-(trifluoromethyl)picolinamide;
N-(2-(1-((3-(2,4-dioxotetrahydropyrimidin-1(2H)-yl)pyridin-4-yl)methyl)piperidin-4-yl)-5-(2-hydroxypropan-2-yl)benzo[d]thiazol-6-yl)-6-(trifluoromethyl)picolinamide;
N-(2-(1-(4-((2,6-dioxopiperidin-3-yl)amino)benzyl)piperidin-4-yl)-5-(2-hydroxypropan-2-yl)benzo[d]thiazol-6-yl)-6-(trifluoromethyl)picolinamide;
N-(2-(1-(3-((2,6-dioxopiperidin-3-yl)amino)benzyl)piperidin-4-yl)-5-(2-hydroxypropan-2-yl)benzo[d]thiazol-6-yl)-6-(trifluoromethyl)picolinamide;
N-(2-(1-(2-((2,6-dioxopiperidin-3-yl)amino)benzyl)piperidin-4-yl)-5-(2-hydroxypropan-2-yl)benzo[d]thiazol-6-yl)-6-(trifluoromethyl)picolinamide;
N-(2-(1-(4-(2,6-dioxopiperidin-3-yl)benzyl)piperidin-4-yl)-5-(2-hydroxypropan-2-yl)benzo[d]oxazol-6-yl)-6-(trifluoromethyl)picolinamide;
N-(2-(1-(4-(2,6-dioxopiperidin-3-yl)-3-fluorobenzyl)piperidin-4-yl)-5-(2-hydroxypropan-2-yl)benzo[d]oxazol-6-yl)-6-(trifluoromethyl)picolinamide;
N-(2-(1-(3-(2,6-dioxopiperidin-3-yl)benzyl)piperidin-4-yl)-5-(2-hydroxypropan-2-yl)benzo[d]oxazol-6-yl)-6-(trifluoromethyl)picolinamide;
N-(2-(1-(2-(2,6-dioxopiperidin-3-yl)benzyl)piperidin-4-yl)-5-(2-hydroxypropan-2-yl)benzo[d]oxazol-6-yl)-6-(trifluoromethyl)picolinamide;
N-(2-(1-((2-(2,6-dioxopiperidin-3-yl)pyridin-4-yl)methyl)piperidin-4-yl)-5-(2-hydroxypropan-2-yl)benzo[d]oxazol-6-yl)-6-(trifluoromethyl)picolinamide;
N-(2-(1-((5-(2,6-dioxopiperidin-3-yl)pyridin-2-yl)methyl)piperidin-4-yl)-5-(2-hydroxypropan-2-yl)benzo[d]oxazol-6-yl)-6-(trifluoromethyl)picolinamide;
N-(2-(1-((2-(2,6-dioxopiperidin-3-yl)pyridin-3-yl)methyl)piperidin-4-yl)-5-(2-hydroxypropan-2-yl)benzo[d]oxazol-6-yl)-6-(trifluoromethyl)picolinamide;
N-(2-(1-((5-(2,6-dioxopiperidin-3-yl)pyridin-3-yl)methyl)piperidin-4-yl)-5-(2-hydroxypropan-2-yl)benzo[d]oxazol-6-yl)-6-(trifluoromethyl)picolinamide;
N-(2-(1-((5-(2,6-dioxopiperidin-3-yl)-2-fluoropyridin-3-yl)methyl)piperidin-4-yl)-5-(2-hydroxypropan-2-yl)benzo[d]oxazol-6-yl)-6-(trifluoromethyl)picolinamide;
N-(2-(1-(4-(2,4-dioxotetrahydropyrimidin-1(2H)-yl)benzyl)piperidin-4-yl)-5-(2-hydroxypropan-2-yl)benzo[d]oxazol-6-yl)-6-(trifluoromethyl)picolinamide;
N-(2-(1-(4-(2,4-dioxotetrahydropyrimidin-1(2H)-yl)-2-fluorobenzyl)piperidin-4-yl)-5-(2-hydroxypropan-2-yl)benzo[d]oxazol-6-yl)-6-(trifluoromethyl)picolinamide;
N-(2-(1-(3-(2,4-dioxotetrahydropyrimidin-1(2H)-yl)benzyl)piperidin-4-yl)-5-(2-hydroxypropan-2-yl)benzo[d]oxazol-6-yl)-6-(trifluoromethyl)picolinamide;
N-(2-(1-(2-(2,4-dioxotetrahydropyrimidin-1(2H)-yl)benzyl)piperidin-4-yl)-5-(2-hydroxypropan-2-yl)benzo[d]oxazol-6-yl)-6-(trifluoromethyl)picolinamide;
N-(2-(1-((5-(2,4-dioxotetrahydropyrimidin-1(2H)-yl)pyridin-2-yl)methyl)piperidin-4-yl)-5-(2-hydroxypropan-2-yl)benzo[d]oxazol-6-yl)-6-(trifluoromethyl)picolinamide;
N-(2-(1-((2-(2,4-dioxotetrahydropyrimidin-1(2H)-yl)pyridin-4-yl)methyl)piperidin-4-yl)-5-(2-hydroxypropan-2-yl)benzo[d]oxazol-6-yl)-6-(trifluoromethyl)picolinamide;
N-(2-(1-((2-(2,4-dioxotetrahydropyrimidin-1(2H)-yl)-5-fluoropyridin-4-yl)methyl)piperidin-4-yl)-5-(2-hydroxypropan-2-yl)benzo[d]oxazol-6-yl)-6-(trifluoromethyl)picolinamide;
N-(2-(1-((6-(2,4-dioxotetrahydropyrimidin-1(2H)-yl)pyridin-3-yl)methyl)piperidin-4-yl)-5-(2-hydroxypropan-2-yl)benzo[d]oxazol-6-yl)-6-(trifluoromethyl)picolinamide;
N-(2-(1-((5-(2,4-dioxotetrahydropyrimidin-1(2H)-yl)pyridin-3-yl)methyl)piperidin-4-yl)-5-(2-hydroxypropan-2-yl)benzo[d]oxazol-6-yl)-6-(trifluoromethyl)picolinamide;
N-(2-(1-((6-(2,4-dioxotetrahydropyrimidin-1(2H)-yl)pyridazin-3-yl)methyl)piperidin-4-yl)-5-(2-hydroxypropan-2-yl)benzo[d]oxazol-6-yl)-6-(trifluoromethyl)picolinamide;
N-(2-(1-((3-(2,4-dioxotetrahydropyrimidin-1(2H)-yl)pyridin-4-yl)methyl)piperidin-4-yl)-5-(2-hydroxypropan-2-yl)benzo[d]oxazol-6-yl)-6-(trifluoromethyl)picolinamide;
N-(2-(1-(4-((2,6-dioxopiperidin-3-yl)amino)benzyl)piperidin-4-yl)-5-(2-hydroxypropan-2-yl)benzo[d]oxazol-6-yl)-6-(trifluoromethyl)picolinamide;
N-(2-(1-(3-((2,6-dioxopiperidin-3-yl)amino)benzyl)piperidin-4-yl)-5-(2-hydroxypropan-2-yl)benzo[d]oxazol-6-yl)-6-(trifluoromethyl)picolinamide;
N-(2-(1-(2-((2,6-dioxopiperidin-3-yl)amino)benzyl)piperidin-4-yl)-5-(2-hydroxypropan-2-yl)benzo[d]oxazol-6-yl)-6-(trifluoromethyl)picolinamide;
N-(2-(1-(4-(2,6-dioxopiperidin-3-yl)benzyl)piperidin-4-yl)-6-(2-hydroxypropan-2-yl)-2H-indazol-5-yl)-6-(trifluoromethyl)picolinamide;
N-(2-(1-(4-(2,6-dioxopiperidin-3-yl)-3-fluorobenzyl)piperidin-4-yl)-6-(2-hydroxypropan-2-yl)-2H-indazol-5-yl)-6-(trifluoromethyl)picolinamide;
N-(2-(1-(3-(2,6-dioxopiperidin-3-yl)benzyl)piperidin-4-yl)-6-(2-hydroxypropan-2-yl)-2H-indazol-5-yl)-6-(trifluoromethyl)picolinamide;
N-(2-(1-(2-(2,6-dioxopiperidin-3-yl)benzyl)piperidin-4-yl)-6-(2-hydroxypropan-2-yl)-2H-indazol-5-yl)-6-(trifluoromethyl)picolinamide;
N-(2-(1-((2-(2,6-dioxopiperidin-3-yl)pyridin-4-yl)methyl)piperidin-4-yl)-6-(2-hydroxypropan-2-yl)-2H-indazol-5-yl)-6-(trifluoromethyl)picolinamide;
N-(2-(1-((5-(2,6-dioxopiperidin-3-yl)pyridin-2-yl)methyl)piperidin-4-yl)-6-(2-hydroxypropan-2-yl)-2H-indazol-5-yl)-6-(trifluoromethyl)picolinamide;
N-(2-(1-((5-(2,6-dioxopiperidin-3-yl)pyridin-3-yl)methyl)piperidin-4-yl)-6-(2-hydroxypropan-2-yl)-2H-indazol-5-yl)-6-(trifluoromethyl)picolinamide;
N-(2-(1-((5-(2,6-dioxopiperidin-3-yl)-2-fluoropyridin-3-yl)methyl)piperidin-4-yl)-6-(2-hydroxypropan-2-yl)-2H-indazol-5-yl)-6-(trifluoromethyl)picolinamide;
N-(2-(1-(4-(2,4-dioxotetrahydropyrimidin-1(2H)-yl)benzyl)piperidin-4-yl)-6-(2-hydroxypropan-2-yl)-2H-indazol-5-yl)-6-(trifluoromethyl)picolinamide;
N-(2-(1-(4-(2,4-dioxotetrahydropyrimidin-1(2H)-yl)-2-fluorobenzyl)piperidin-4-yl)-6-(2-hydroxypropan-2-yl)-2H-indazol-5-yl)-6-(trifluoromethyl)picolinamide;
N-(2-(1-(3-(2,4-dioxotetrahydropyrimidin-1(2H)-yl)benzyl)piperidin-4-yl)-6-(2-hydroxypropan-2-yl)-2H-indazol-5-yl)-6-(trifluoromethyl)picolinamide;
N-(2-(1-(2-(2,4-dioxotetrahydropyrimidin-1(2H)-yl)benzyl)piperidin-4-yl)-6-(2-hydroxypropan-2-yl)-2H-indazol-5-yl)-6-(trifluoromethyl)picolinamide;
N-(2-(1-((5-(2,4-dioxotetrahydropyrimidin-1(2H)-yl)pyridin-2-yl)methyl)piperidin-4-yl)-6-(2-hydroxypropan-2-yl)-2H-indazol-5-yl)-6-(trifluoromethyl)picolinamide;
N-(2-(1-((2-(2,4-dioxotetrahydropyrimidin-1(2H)-yl)pyridin-4-yl)methyl)piperidin-4-yl)-6-(2-hydroxypropan-2-yl)-2H-indazol-5-yl)-6-(trifluoromethyl)picolinamide;
N-(2-(1-((2-(2,4-dioxotetrahydropyrimidin-1(2H)-yl)-5-fluoropyridin-4-yl)methyl)piperidin-4-yl)-6-(2-hydroxypropan-2-yl)-2H-indazol-5-yl)-6-(trifluoromethyl)picolinamide;
N-(2-(1-((6-(2,4-dioxotetrahydropyrimidin-1(2H)-yl)pyridin-3-yl)methyl)piperidin-4-yl)-6-(2-hydroxypropan-2-yl)-2H-indazol-5-yl)-6-(trifluoromethyl)picolinamide;
N-(2-(1-((5-(2,4-dioxotetrahydropyrimidin-1(2H)-yl)pyridin-3-yl)methyl)piperidin-4-yl)-6-(2-hydroxypropan-2-yl)-2H-indazol-5-yl)-6-(trifluoromethyl)picolinamide;
N-(2-(1-((5-(2,4-dioxotetrahydropyrimidin-1(2H)-yl)-2-fluoropyridin-3-yl)methyl)piperidin-4-yl)-6-(2-hydroxypropan-2-yl)-2H-indazol-5-yl)-6-(trifluoromethyl)picolinamide;
N-(2-(1-((6-(2,4-dioxotetrahydropyrimidin-1(2H)-yl)pyridazin-3-yl)methyl)piperidin-4-yl)-6-(2-hydroxypropan-2-yl)-2H-indazol-5-yl)-6-(trifluoromethyl)picolinamide;
N-(2-(1-((5-(2,4-dioxotetrahydropyrimidin-1(2H)-yl)pyrazin-2-yl)methyl)piperidin-4-yl)-6-(2-hydroxypropan-2-yl)-2H-indazol-5-yl)-6-(trifluoromethyl)picolinamide;
N-(2-(1-((3-(2,4-dioxotetrahydropyrimidin-1(2H)-yl)pyridin-4-yl)methyl)piperidin-4-yl)-6-(2-hydroxypropan-2-yl)-2H-indazol-5-yl)-6-(trifluoromethyl)picolinamide;
N-(2-(1-(4-((2,6-dioxopiperidin-3-yl)amino)benzyl)piperidin-4-yl)-6-(2-hydroxypropan-2-yl)-2H-indazol-5-yl)-6-(trifluoromethyl)picolinamide;
N-(2-(1-(3-((2,6-dioxopiperidin-3-yl)amino)benzyl)piperidin-4-yl)-6-(2-hydroxypropan-2-yl)-2H-indazol-5-yl)-6-(trifluoromethyl)picolinamide;
N-(2-(1-(2-((2,6-dioxopiperidin-3-yl)amino)benzyl)piperidin-4-yl)-6-(2-hydroxypropan-2-yl)-2H-indazol-5-yl)-6-(trifluoromethyl)picolinamide;
N-(2-(1-(4-(2,6-dioxopiperidin-3-yl)benzyl)piperidin-4-yl)-6-methoxy-2H-indazol-5-yl)-6-(trifluoromethyl)picolinamide;
N-(2-(1-(4-(2,6-dioxopiperidin-3-yl)-3-fluorobenzyl)piperidin-4-yl)-6-methoxy-2H-indazol-5-yl)-6-(trifluoromethyl)picolinamide;
N-(2-(1-(3-(2,6-dioxopiperidin-3-yl)benzyl)piperidin-4-yl)-6-methoxy-2H-indazol-5-yl)-6-(trifluoromethyl)picolinamide;
N-(2-(1-(2-(2,6-dioxopiperidin-3-yl)benzyl)piperidin-4-yl)-6-methoxy-2H-indazol-5-yl)-6-(trifluoromethyl)picolinamide;
N-(2-(1-((2-(2,6-dioxopiperidin-3-yl)pyridin-4-yl)methyl)piperidin-4-yl)-6-methoxy-2H-indazol-5-yl)-6-(trifluoromethyl)picolinamide;
N-(2-(1-((5-(2,6-dioxopiperidin-3-yl)pyridin-2-yl)methyl)piperidin-4-yl)-6-methoxy-2H-indazol-5-yl)-6-(trifluoromethyl)picolinamide;
N-(2-(1-((2-(2,6-dioxopiperidin-3-yl)pyridin-3-yl)methyl)piperidin-4-yl)-6-methoxy-2H-indazol-5-yl)-6-(trifluoromethyl)picolinamide;
N-(2-(1-((5-(2,6-dioxopiperidin-3-yl)pyridin-3-yl)methyl)piperidin-4-yl)-6-methoxy-2H-indazol-5-yl)-6-(trifluoromethyl)picolinamide;
N-(2-(1-((5-(2,6-dioxopiperidin-3-yl)-2-fluoropyridin-3-yl)methyl)piperidin-4-yl)-6-methoxy-2H-indazol-5-yl)-6-(trifluoromethyl)picolinamide;
N-(2-(1-(4-(2,4-dioxotetrahydropyrimidin-1(2H)-yl)benzyl)piperidin-4-yl)-6-methoxy-2H-indazol-5-yl)-6-(trifluoromethyl)picolinamide;
N-(2-(1-(4-(2,4-dioxotetrahydropyrimidin-1(2H)-yl)-2-fluorobenzyl)piperidin-4-yl)-6-methoxy-2H-indazol-5-yl)-6-(trifluoromethyl)picolinamide;
N-(2-(1-(4-(2,4-dioxotetrahydropyrimidin-1(2H)-yl)-3-fluorobenzyl)piperidin-4-yl)-6-methoxy-2H-indazol-5-yl)-6-(trifluoromethyl)picolinamide;
N-(2-(1-(3-(2,4-dioxotetrahydropyrimidin-1(2H)-yl)benzyl)piperidin-4-yl)-6-methoxy-2H-indazol-5-yl)-6-(trifluoromethyl)picolinamide;
N-(2-(1-(2-(2,4-dioxotetrahydropyrimidin-1(2H)-yl)benzyl)piperidin-4-yl)-6-methoxy-2H-indazol-5-yl)-6-(trifluoromethyl)picolinamide;
N-(2-(1-((5-(2,4-dioxotetrahydropyrimidin-1(2H)-yl)pyridin-2-yl)methyl)piperidin-4-yl)-6-methoxy-2H-indazol-5-yl)-6-(trifluoromethyl)picolinamide;
N-(2-(1-((2-(2,4-dioxotetrahydropyrimidin-1(2H)-yl)pyridin-4-yl)methyl)piperidin-4-yl)-6-methoxy-2H-indazol-5-yl)-6-(trifluoromethyl)picolinamide;
N-(2-(1-((2-(2,4-dioxotetrahydropyrimidin-1(2H)-yl)-5-fluoropyridin-4-yl)methyl)piperidin-4-yl)-6-methoxy-2H-indazol-5-yl)-6-(trifluoromethyl)picolinamide;
N-(2-(1-((6-(2,4-dioxotetrahydropyrimidin-1(2H)-yl)pyridin-3-yl)methyl)piperidin-4-yl)-6-methoxy-2H-indazol-5-yl)-6-(trifluoromethyl)picolinamide;
N-(2-(1-((5-(2,4-dioxotetrahydropyrimidin-1(2H)-yl)pyridin-3-yl)methyl)piperidin-4-yl)-6-methoxy-2H-indazol-5-yl)-6-(trifluoromethyl)picolinamide;
N-(2-(1-((5-(2,4-dioxotetrahydropyrimidin-1(2H)-yl)-2-fluoropyridin-3-yl)methyl)piperidin-4-yl)-6-methoxy-2H-indazol-5-yl)-6-(trifluoromethyl)picolinamide;
N-(2-(1-((6-(2,4-dioxotetrahydropyrimidin-1(2H)-yl)pyridazin-3-yl)methyl)piperidin-4-yl)-6-methoxy-2H-indazol-5-yl)-6-(trifluoromethyl)picolinamide;
N-(2-(1-((5-(2,4-dioxotetrahydropyrimidin-1(2H)-yl)pyrazin-2-yl)methyl)piperidin-4-yl)-6-methoxy-2H-indazol-5-yl)-6-(trifluoromethyl)picolinamide;
N-(2-(1-((3-(2,4-dioxotetrahydropyrimidin-1(2H)-yl)pyridin-4-yl)methyl)piperidin-4-yl)-6-methoxy-2H-indazol-5-yl)-6-(trifluoromethyl)picolinamide;
N-(2-(1-(4-((2,6-dioxopiperidin-3-yl)amino)benzyl)piperidin-4-yl)-6-methoxy-2H-indazol-5-yl)-6-(trifluoromethyl)picolinamide;
N-(2-(1-(3-((2,6-dioxopiperidin-3-yl)amino)benzyl)piperidin-4-yl)-6-methoxy-2H-indazol-5-yl)-6-(trifluoromethyl)picolinamide;
N-(2-(1-(2-((2,6-dioxopiperidin-3-yl)amino)benzyl)piperidin-4-yl)-6-methoxy-2H-indazol-5-yl)-6-(trifluoromethyl)picolinamide;
N-(2-(1-(4-(2,6-dioxopiperidin-3-yl)benzyl)piperidin-4-yl)-6-methoxy-2H-indazol-5-yl)-3-(trifluoromethyl)benzamide;
N-(2-(1-(4-(2,6-dioxopiperidin-3-yl)benzyl)piperidin-4-yl)-6-methoxy-2H-indazol-7-yl)-3-(trifluoromethyl)benzamide;
N-(2-(1-(4-(2,6-dioxopiperidin-3-yl)-3-fluorobenzyl)piperidin-4-yl)-6-methoxy-2H-indazol-7-yl)-3-(trifluoromethyl)benzamide;
N-(2-(1-(4-(2,4-dioxotetrahydropyrimidin-1(2H)-yl)benzyl)piperidin-4-yl)-6-methoxy-2H-indazol-7-yl)-3-(trifluoromethyl)benzamide;
N-(2-(1-(4-(2,4-dioxotetrahydropyrimidin-1(2H)-yl)-2-fluorobenzyl)piperidin-4-yl)-6-methoxy-2H-indazol-7-yl)-3-(trifluoromethyl)benzamide;
N-(2-(1-(4-(2,4-dioxotetrahydropyrimidin-1(2H)-yl)-3-fluorobenzyl)piperidin-4-yl)-6-methoxy-2H-indazol-7-yl)-3-(trifluoromethyl)benzamide;
N-(2-(1-(3-((2,6-dioxopiperidin-3-yl)amino)benzyl)piperidin-4-yl)-6-methoxy-2H-indazol-7-yl)-3-(trifluoromethyl)benzamide;
N-(2-(1-(4-(2,6-dioxopiperidin-3-yl)-3-fluorobenzyl)piperidin-4-yl)-6-methoxy-2H-indazol-5-yl)-3-(trifluoromethyl)benzamide;
N-(2-(1-(3-(2,6-dioxopiperidin-3-yl)benzyl)piperidin-4-yl)-6-methoxy-2H-indazol-5-yl)-3-(trifluoromethyl)benzamide;
N-(2-(1-(2-(2,6-dioxopiperidin-3-yl)benzyl)piperidin-4-yl)-6-methoxy-2H-indazol-5-yl)-3-(trifluoromethyl)benzamide;
N-(2-(1-((2-(2,6-dioxopiperidin-3-yl)pyridin-4-yl)methyl)piperidin-4-yl)-6-methoxy-2H-indazol-5-yl)-3-(trifluoromethyl)benzamide;
N-(2-(1-((5-(2,6-dioxopiperidin-3-yl)pyridin-2-yl)methyl)piperidin-4-yl)-6-methoxy-2H-indazol-5-yl)-3-(trifluoromethyl)benzamide;
N-(2-(1-((2-(2,6-dioxopiperidin-3-yl)pyridin-3-yl)methyl)piperidin-4-yl)-6-methoxy-2H-indazol-5-yl)-3-(trifluoromethyl)benzamide;
N-(2-(1-((5-(2,6-dioxopiperidin-3-yl)pyridin-3-yl)methyl)piperidin-4-yl)-6-methoxy-2H-indazol-5-yl)-3-(trifluoromethyl)benzamide;
N-(2-(1-((5-(2,6-dioxopiperidin-3-yl)-2-fluoropyridin-3-yl)methyl)piperidin-4-yl)-6-methoxy-2H-indazol-5-yl)-3-(trifluoromethyl)benzamide;
N-(2-(1-(4-(2,4-dioxotetrahydropyrimidin-1(2H)-yl)benzyl)piperidin-4-yl)-6-methoxy-2H-indazol-5-yl)-3-(trifluoromethyl)benzamide;
N-(2-(1-(4-(2,4-dioxotetrahydropyrimidin-1(2H)-yl)-2-fluorobenzyl)piperidin-4-yl)-6-methoxy-2H-indazol-5-yl)-3-(trifluoromethyl)benzamide;
N-(2-(1-(4-(2,4-dioxotetrahydropyrimidin-1(2H)-yl)-3-fluorobenzyl)piperidin-4-yl)-6-methoxy-2H-indazol-5-yl)-3-(trifluoromethyl)benzamide;
N-(2-(1-(3-(2,4-dioxotetrahydropyrimidin-1(2H)-yl)benzyl)piperidin-4-yl)-6-methoxy-2H-indazol-5-yl)-3-(trifluoromethyl)benzamide;
N-(2-(1-(2-(2,4-dioxotetrahydropyrimidin-1(2H)-yl)benzyl)piperidin-4-yl)-6-methoxy-2H-indazol-5-yl)-3-(trifluoromethyl)benzamide;
N-(2-(1-((5-(2,4-dioxotetrahydropyrimidin-1(2H)-yl)pyridin-2-yl)methyl)piperidin-4-yl)-6-methoxy-2H-indazol-5-yl)-3-(trifluoromethyl)benzamide;
N-(2-(1-((2-(2,4-dioxotetrahydropyrimidin-1(2H)-yl)pyridin-4-yl)methyl)piperidin-4-yl)-6-methoxy-2H-indazol-5-yl)-3-(trifluoromethyl)benzamide;
N-(2-(1-((2-(2,4-dioxotetrahydropyrimidin-1(2H)-yl)-5-fluoropyridin-4-yl)methyl)piperidin-4-yl)-6-methoxy-2H-indazol-5-yl)-3-(trifluoromethyl)benzamide;
N-(2-(1-((6-(2,4-dioxotetrahydropyrimidin-1(2H)-yl)pyridin-3-yl)methyl)piperidin-4-yl)-6-methoxy-2H-indazol-5-yl)-3-(trifluoromethyl)benzamide;
N-(2-(1-((5-(2,4-dioxotetrahydropyrimidin-1(2H)-yl)pyridin-2-yl)methyl)piperidin-4-yl)-6-methoxy-2H-indazol-7-yl)-3-(trifluoromethyl)benzamide;
N-(2-(1-((6-(2,4-dioxotetrahydropyrimidin-1(2H)-yl)pyridazin-3-yl)methyl)piperidin-4-yl)-6-methoxy-2H-indazol-5-yl)-3-(trifluoromethyl)benzamide;
N-(2-(1-((5-(2,4-dioxotetrahydropyrimidin-1(2H)-yl)pyrazin-2-yl)methyl)piperidin-4-yl)-6-methoxy-2H-indazol-5-yl)-3-(trifluoromethyl)benzamide;
N-(2-(1-((3-(2,4-dioxotetrahydropyrimidin-1(2H)-yl)pyridin-4-yl)methyl)piperidin-4-yl)-6-methoxy-2H-indazol-5-yl)-3-(trifluoromethyl)benzamide;
N-(2-(1-(4-((2,6-dioxopiperidin-3-yl)amino)benzyl)piperidin-4-yl)-6-methoxy-2H-indazol-5-yl)-3-(trifluoromethyl)benzamide;
N-(2-(1-(3-((2,6-dioxopiperidin-3-yl)amino)benzyl)piperidin-4-yl)-6-methoxy-2H-indazol-5-yl)-3-(trifluoromethyl)benzamide;
N-(2-(1-(2-((2,6-dioxopiperidin-3-yl)amino)benzyl)piperidin-4-yl)-6-methoxy-2H-indazol-5-yl)-3-(trifluoromethyl)benzamide;
N-(2-(1-((6-(2,4-dioxotetrahydropyrimidin-1(2H)-yl)pyridin-3-yl)methyl)piperidin-4-yl)-6-methoxy-2H-indazol-7-yl)-3-(trifluoromethyl)benzamide;
N-(2-(1-(3-(2,4-dioxotetrahydropyrimidin-1(2H)-yl)benzyl)piperidin-4-yl)-6-methoxy-2H-indazol-7-yl)-3-(trifluoromethyl)benzamide;
N-(2-(1-((5-(2,4-dioxotetrahydropyrimidin-1(2H)-yl)pyridin-3-yl)methyl)piperidin-4-yl)-6-methoxy-2H-indazol-5-yl)-3-(trifluoromethyl)benzamide;
N-(2-(1-((5-(2,4-dioxotetrahydropyrimidin-1(2H)-yl)-2-fluoropyridin-3-yl)methyl)piperidin-4-yl)-6-methoxy-2H-indazol-5-yl)-3-(trifluoromethyl)benzamide;
6-(6-(4-(4-(2,6-dioxopiperidin-3-yl)benzyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(4-(2,6-dioxopiperidin-3-yl)-3-fluorobenzyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(3-(2,6-dioxopiperidin-3-yl)benzyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(2-(2,6-dioxopiperidin-3-yl)benzyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(4-(2,4-dioxotetrahydropyrimidin-1(2H)-yl)benzyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(4-(2,4-dioxotetrahydropyrimidin-1(2H)-yl)-2-fluorobenzyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(3-(2,4-dioxotetrahydropyrimidin-1(2H)-yl)benzyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(2-(2,4-dioxotetrahydropyrimidin-1(2H)-yl)benzyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((5-(2,4-dioxotetrahydropyrimidin-1(2H)-yl)pyridin-2-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((6-(2,4-dioxotetrahydropyrimidin-1(2H)-yl)pyridazin-3-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((3-(2,4-dioxotetrahydropyrimidin-1(2H)-yl)pyridin-4-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(4-((2,6-dioxopiperidin-3-yl)amino)benzyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(3-((2,6-dioxopiperidin-3-yl)amino)benzyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(2-((2,6-dioxopiperidin-3-yl)amino)benzyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((4-(2,6-dioxopiperidin-3-yl)benzyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((4-(2,6-dioxopiperidin-3-yl)-3-fluorobenzyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((3-(2,6-dioxopiperidin-3-yl)benzyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((2-(2,6-dioxopiperidin-3-yl)benzyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((4-(2,4-dioxotetrahydropyrimidin-1(2H)-yl)benzyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((4-(2,4-dioxotetrahydropyrimidin-1(2H)-yl)-2-fluorobenzyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((3-(2,4-dioxotetrahydropyrimidin-1(2H)-yl)benzyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((2-(2,4-dioxotetrahydropyrimidin-1(2H)-yl)benzyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(((6-(2,4-dioxotetrahydropyrimidin-1(2H)-yl)pyridin-3-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(((6-(2,4-dioxotetrahydropyrimidin-1(2H)-yl)pyridazin-3-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(((3-(2,4-dioxotetrahydropyrimidin-1(2H)-yl)pyridin-4-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((4-((2,6-dioxopiperidin-3-yl)amino)benzyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((3-((2,6-dioxopiperidin-3-yl)amino)benzyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-((2-((2,6-dioxopiperidin-3-yl)amino)benzyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-(4-(2,6-dioxopiperidin-3-yl)benzyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-(4-(2,6-dioxopiperidin-3-yl)-3-fluorobenzyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-(3-(2,6-dioxopiperidin-3-yl)benzyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-(2-(2,6-dioxopiperidin-3-yl)benzyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-(4-(2,4-dioxotetrahydropyrimidin-1(2H)-yl)benzyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-(4-(2,4-dioxotetrahydropyrimidin-1(2H)-yl)-2-fluorobenzyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-(3-(2,4-dioxotetrahydropyrimidin-1(2H)-yl)benzyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-(2-(2,4-dioxotetrahydropyrimidin-1(2H)-yl)benzyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((5-(2,4-dioxotetrahydropyrimidin-1(2H)-yl)pyridin-2-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((6-(2,4-dioxotetrahydropyrimidin-1(2H)-yl)pyridazin-3-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((3-(2,4-dioxotetrahydropyrimidin-1(2H)-yl)pyridin-4-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-(4-((2,6-dioxopiperidin-3-yl)amino)benzyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-(3-((2,6-dioxopiperidin-3-yl)amino)benzyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-(2-((2,6-dioxopiperidin-3-yl)amino)benzyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
N-((4,6-dimethyl-2-oxo-1,2-dihydropyridin-3-yl)methyl)-4'-((4-(4-(2,6-dioxopiperidin-3-yl)benzyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-(4-(2,6-dioxopiperidin-3-yl)-3-fluorobenzyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-(3-(2,6-dioxopiperidin-3-yl)benzyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-(2-(2,6-dioxopiperidin-3-yl)benzyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-(4-(2,4-dioxotetrahydropyrimidin-1(2H)-yl)benzyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-(4-(2,4-dioxotetrahydropyrimidin-1(2H)-yl)-2-fluorobenzyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-(3-(2,4-dioxotetrahydropyrimidin-1(2H)-yl)benzyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-(2-(2,4-dioxotetrahydropyrimidin-1(2H)-yl)benzyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-((5-(2,4-dioxotetrahydropyrimidin-1(2H)-yl)pyridin-2-yl)methyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-((6-(2,4-dioxotetrahydropyrimidin-1(2H)-yl)pyridazin-3-yl)methyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-((3-(2,4-dioxotetrahydropyrimidin-1(2H)-yl)pyridin-4-yl)methyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-(4-((2,6-dioxopiperidin-3-yl)amino)benzyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-(3-((2,6-dioxopiperidin-3-yl)amino)benzyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-((4-(2-((2,6-dioxopiperidin-3-yl)amino)benzyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(4-(2,6-dioxopiperidin-3-yl)benzyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(4-(2,6-dioxopiperidin-3-yl)-3-fluorobenzyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(3-(2,6-dioxopiperidin-3-yl)benzyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(2-(2,6-dioxopiperidin-3-yl)benzyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(4-(2,4-dioxotetrahydropyrimidin-1(2H)-yl)benzyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(4-(2,4-dioxotetrahydropyrimidin-1(2H)-yl)-2-fluorobenzyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(3-(2,4-dioxotetrahydropyrimidin-1(2H)-yl)benzyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(2-(2,4-dioxotetrahydropyrimidin-1(2H)-yl)benzyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((5-(2,4-dioxotetrahydropyrimidin-1(2H)-yl)pyridin-2-yl)methyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((6-(2,4-dioxotetrahydropyrimidin-1(2H)-yl)pyridazin-3-yl)methyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((3-(2,4-dioxotetrahydropyrimidin-1(2H)-yl)pyridin-4-yl)methyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(4-((2,6-dioxopiperidin-3-yl)amino)benzyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(3-((2,6-dioxopiperidin-3-yl)amino)benzyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(2-((2,6-dioxopiperidin-3-yl)amino)benzyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((4-(2,6-dioxopiperidin-3-yl)benzyl)(methyl)amino)piperidin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((4-(2,6-dioxopiperidin-3-yl)-3-fluorobenzyl)(methyl)amino)piperidin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((3-(2,6-dioxopiperidin-3-yl)benzyl)(methyl)amino)piperidin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,6-dioxopiperidin-3-yl)benzyl)(methyl)amino)piperidin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((4-(2,4-dioxotetrahydropyrimidin-1(2H)-yl)benzyl)(methyl)amino)piperidin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((4-(2,4-dioxotetrahydropyrimidin-1(2H)-yl)-2-fluorobenzyl)(methyl)amino)piperidin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((3-(2,4-dioxotetrahydropyrimidin-1(2H)-yl)benzyl)(methyl)amino)piperidin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,4-dioxotetrahydropyrimidin-1(2H)-yl)benzyl)(methyl)amino)piperidin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(((5-(2,4-dioxotetrahydropyrimidin-1(2H)-yl)pyridin-2-yl)methyl)(methyl)amino)piperidin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(((6-(2,4-dioxotetrahydropyrimidin-1(2H)-yl)pyridazin-3-yl)methyl)(methyl)amino)piperidin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(((3-(2,4-dioxotetrahydropyrimidin-1(2H)-yl)pyridin-4-yl)methyl)(methyl)amino)piperidin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-(4-(2,6-dioxopiperidin-3-yl)benzyl)piperidin-4-yl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-(4-(2,6-dioxopiperidin-3-yl)-3-fluorobenzyl)piperidin-4-yl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-(3-(2,6-dioxopiperidin-3-yl)benzyl)piperidin-4-yl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-(2-(2,6-dioxopiperidin-3-yl)benzyl)piperidin-4-yl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-(4-(2,4-dioxotetrahydropyrimidin-1(2H)-yl)benzyl)piperidin-4-yl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-(4-(2,4-dioxotetrahydropyrimidin-1(2H)-yl)-2-fluorobenzyl)piperidin-4-yl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-(3-(2,4-dioxotetrahydropyrimidin-1(2H)-yl)benzyl)piperidin-4-yl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-(2-(2,4-dioxotetrahydropyrimidin-1(2H)-yl)benzyl)piperidin-4-yl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-((5-(2,4-dioxotetrahydropyrimidin-1(2H)-yl)pyridin-2-yl)methyl)piperidin-4-yl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-((6-(2,4-dioxotetrahydropyrimidin-1(2H)-yl)pyridazin-3-yl)methyl)piperidin-4-yl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-((3-(2,4-dioxotetrahydropyrimidin-1(2H)-yl)pyridin-4-yl)methyl)piperidin-4-yl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-(4-((2,6-dioxopiperidin-3-yl)amino)benzyl)piperidin-4-yl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-(3-((2,6-dioxopiperidin-3-yl)amino)benzyl)piperidin-4-yl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-(2-((2,6-dioxopiperidin-3-yl)amino)benzyl)piperidin-4-yl)piperazin-1-yl)-5-(ethyl(tetrahydro-2H-pyran-4-yl)amino)-4-methyl-[1,1'-biphenyl]-3-carboxamide;
N-(5-(3,5-difluorobenzyl)-1H-indazol-3-yl)-4-(4-(4-(2,6-dioxopiperidin-3-yl)benzyl)piperazin-1-yl)-2-((tetrahydro-2H-pyran-4-yl)amino)benzamide;
N-(5-(3,5-difluorobenzyl)-1H-indazol-3-yl)-4-(4-(4-(2,6-dioxopiperidin-3-yl)-3-fluorobenzyl)piperazin-1-yl)-2-((tetrahydro-2H-pyran-4-yl)amino)benzamide;
N-(5-(3,5-difluorobenzyl)-1H-indazol-3-yl)-4-(4-(3-(2,6-dioxopiperidin-3-yl)benzyl)piperazin-1-yl)-2-((tetrahydro-2H-pyran-4-yl)amino)benzamide;
N-(5-(3,5-difluorobenzyl)-1H-indazol-3-yl)-4-(4-(2-(2,6-dioxopiperidin-3-yl)benzyl)piperazin-1-yl)-2-((tetrahydro-2H-pyran-4-yl)amino)benzamide;
N-(5-(3,5-difluorobenzyl)-1H-indazol-3-yl)-4-(4-(4-(2,4-dioxotetrahydropyrimidin-1(2H)-yl)benzyl)piperazin-1-yl)-2-((tetrahydro-2H-pyran-4-yl)amino)benzamide;
N-(5-(3,5-difluorobenzyl)-1H-indazol-3-yl)-4-(4-(4-(2,4-dioxotetrahydropyrimidin-1(2H)-yl)-2-fluorobenzyl)piperazin-1-yl)-2-((tetrahydro-2H-pyran-4-yl)amino)benzamide;
N-(5-(3,5-difluorobenzyl)-1H-indazol-3-yl)-4-(4-(3-(2,4-dioxotetrahydropyrimidin-1(2H)-yl)benzyl)piperazin-1-yl)-2-((tetrahydro-2H-pyran-4-yl)amino)benzamide;
N-(5-(3,5-difluorobenzyl)-1H-indazol-3-yl)-4-(4-(2-(2,4-dioxotetrahydropyrimidin-1(2H)-yl)benzyl)piperazin-1-yl)-2-((tetrahydro-2H-pyran-4-yl)amino)benzamide;
N-(5-(3,5-difluorobenzyl)-1H-indazol-3-yl)-4-(4-((5-(2,4-dioxotetrahydropyrimidin-1(2H)-yl)pyridin-2-yl)methyl)piperazin-1-yl)-2-((tetrahydro-2H-pyran-4-yl)amino)benzamide;
N-(5-(3,5-difluorobenzyl)-1H-indazol-3-yl)-4-(4-((6-(2,4-dioxotetrahydropyrimidin-1(2H)-yl)pyridazin-3-yl)methyl)piperazin-1-yl)-2-((tetrahydro-2H-pyran-4-yl)amino)benzamide;
N-(5-(3,5-difluorobenzyl)-1H-indazol-3-yl)-4-(4-((3-(2,4-dioxotetrahydropyrimidin-1(2H)-yl)pyridin-4-yl)methyl)piperazin-1-yl)-2-((tetrahydro-2H-pyran-4-yl)amino)benzamide;
N-(5-(3,5-difluorobenzyl)-1H-indazol-3-yl)-4-(4-(4-((2,6-dioxopiperidin-3-yl)amino)benzyl)piperazin-1-yl)-2-((tetrahydro-2H-pyran-4-yl)amino)benzamide;
N-(5-(3,5-difluorobenzyl)-1H-indazol-3-yl)-4-(4-(3-((2,6-dioxopiperidin-3-yl)amino)benzyl)piperazin-1-yl)-2-((tetrahydro-2H-pyran-4-yl)amino)benzamide;
N-(5-(3,5-difluorobenzyl)-1H-indazol-3-yl)-4-(4-(2-((2,6-dioxopiperidin-3-yl)amino)benzyl)piperazin-1-yl)-2-((tetrahydro-2H-pyran-4-yl)amino)benzamide;
N-(5-(3,5-difluorobenzyl)-1H-indazol-3-yl)-4-(4-((4-(2,6-dioxopiperidin-3-yl)benzyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-((4-(2,6-dioxopiperidin-3-yl)-3-fluorobenzyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-((3-(2,6-dioxopiperidin-3-yl)benzyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-((2-(2,6-dioxopiperidin-3-yl)benzyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-((4-(2,4-dioxotetrahydropyrimidin-1(2H)-yl)benzyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-((4-(2,4-dioxotetrahydropyrimidin-1(2H)-yl)-2-fluorobenzyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-((3-(2,4-dioxotetrahydropyrimidin-1(2H)-yl)benzyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-((2-(2,4-dioxotetrahydropyrimidin-1(2H)-yl)benzyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-(((5-(2,4-dioxotetrahydropyrimidin-1(2H)-yl)pyridin-2-yl)methyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-(((6-(2,4-dioxotetrahydropyrimidin-1(2H)-yl)pyridazin-3-yl)methyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-(((3-(2,4-dioxotetrahydropyrimidin-1(2H)-yl)pyridin-4-yl)methyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-((4-((2,6-dioxopiperidin-3-yl)amino)benzyl)(methyl)amino)piperidin-1-yl)-2-((tetrahydro-2H-pyran-4-yl)amino)benzamide;
N-(5-(3,5-difluorobenzyl)-1H-indazol-3-yl)-4-(4-(1-(4-(2,6-dioxopiperidin-3-yl)benzyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-(4-(2,6-dioxopiperidin-3-yl)-3-fluorobenzyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-(3-(2,6-dioxopiperidin-3-yl)benzyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-(2-(2,6-dioxopiperidin-3-yl)benzyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-(4-(2,4-dioxotetrahydropyrimidin-1(2H)-yl)benzyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-(4-(2,4-dioxotetrahydropyrimidin-1(2H)-yl)-2-fluorobenzyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-(3-(2,4-dioxotetrahydropyrimidin-1(2H)-yl)benzyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-(2-(2,4-dioxotetrahydropyrimidin-1(2H)-yl)benzyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-((5-(2,4-dioxotetrahydropyrimidin-1(2H)-yl)pyridin-2-yl)methyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-((6-(2,4-dioxotetrahydropyrimidin-1(2H)-yl)pyridazin-3-yl)methyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-((3-(2,4-dioxotetrahydropyrimidin-1(2H)-yl)pyridin-4-yl)methyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-(4-((2,6-dioxopiperidin-3-yl)amino)benzyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-(3-((2,6-dioxopiperidin-3-yl)amino)benzyl)piperidin-4-yl)piperazin-1-yl)-2-((tetrahydro-2H-pyran-4-yl)amino)benzamide;
N-(5-(3,5-difluorobenzyl)-1H-indazol-3-yl)-4-(4-(1-(2-((2,6-dioxopiperidin-3-yl)amino)benzyl)piperidin-4-yl)piperazin-1-yl)-2-((tetrahydro-2H-pyran-4-yl)amino)benzamide;
3-(4-((4-(6-(6-((R)-2-(3-fluorophenyl)pyrrolidin-1-yl)imidazo[1,2-b]pyridazin-3-yl)pyridin-2-yl)piperazin-1-yl)methyl)phenyl)piperidine-2,6-dione;
3-(2-fluoro-4-((4-(6-(6-((R)-2-(3-fluorophenyl)pyrrolidin-1-yl)imidazo[1,2-b]pyridazin-3-yl)pyridin-2-yl)piperazin-1-yl)methyl)phenyl)piperidine-2,6-dione;
3-(3-((4-(6-(6-((R)-2-(3-fluorophenyl)pyrrolidin-1-yl)imidazo[1,2-b]pyridazin-3-yl)pyridin-2-yl)piperazin-1-yl)methyl)phenyl)piperidine-2,6-dione;
3-(2-((4-(6-(6-((R)-2-(3-fluorophenyl)pyrrolidin-1-yl)imidazo[1,2-b]pyridazin-3-yl)pyridin-2-yl)piperazin-1-yl)methyl)phenyl)piperidine-2,6-dione;
(R)-1-(4-((4-(6-(6-(2-(3-fluorophenyl)pyrrolidin-1-yl)imidazo[1,2-b]pyridazin-3-yl)pyridin-2-yl)piperazin-1-yl)methyl)phenyl)dihydropyrimidine-2,4(1H,3H)-dione;
(R)-1-(3-fluoro-4-((4-(6-(6-(2-(3-fluorophenyl)pyrrolidin-1-yl)imidazo[1,2-b]pyridazin-3-yl)pyridin-2-yl)piperazin-1-yl)methyl)phenyl)dihydropyrimidine-2,4(1H,3H)-dione;
(R)-1-(3-((4-(6-(6-(2-(3-fluorophenyl)pyrrolidin-1-yl)imidazo[1,2-b]pyridazin-3-yl)pyridin-2-yl)piperazin-1-yl)methyl)phenyl)dihydropyrimidine-2,4(1H,3H)-dione;
(R)-1-(2-((4-(6-(6-(2-(3-fluorophenyl)pyrrolidin-1-yl)imidazo[1,2-b]pyridazin-3-yl)pyridin-2-yl)piperazin-1-yl)methyl)phenyl)dihydropyrimidine-2,4(1H,3H)-dione;
(R)-1-(6-((4-(6-(6-(2-(3-fluorophenyl)pyrrolidin-1-yl)imidazo[1,2-b]pyridazin-3-yl)pyridin-2-yl)piperazin-1-yl)methyl)pyridin-3-yl)dihydropyrimidine-2,4(1H,3H)-dione;
(R)-1-(6-((4-(6-(6-(2-(3-fluorophenyl)pyrrolidin-1-yl)imidazo[1,2-b]pyridazin-3-yl)pyridin-2-yl)piperazin-1-yl)methyl)pyridazin-3-yl)dihydropyrimidine-2,4(1H,3H)-dione;
(R)-1-(4-((4-(6-(6-(2-(3-fluorophenyl)pyrrolidin-1-yl)imidazo[1,2-b]pyridazin-3-yl)pyridin-2-yl)piperazin-1-yl)methyl)pyridin-3-yl)dihydropyrimidine-2,4(1H,3H)-dione;
3-((4-((4-(6-(6-((R)-2-(3-fluorophenyl)pyrrolidin-1-yl)imidazo[1,2-b]pyridazin-3-yl)pyridin-2-yl)piperazin-1-yl)methyl)phenyl)amino)piperidine-2,6-dione;
3-((3-((4-(6-(6-((R)-2-(3-fluorophenyl)pyrrolidin-1-yl)imidazo[1,2-b]pyridazin-3-yl)pyridin-2-yl)piperazin-1-yl)methyl)phenyl)amino)piperidine-2,6-dione;
3-((2-((4-(6-(6-((R)-2-(3-fluorophenyl)pyrrolidin-1-yl)imidazo[1,2-b]pyridazin-3-yl)pyridin-2-yl)piperazin-1-yl)methyl)phenyl)amino)piperidine-2,6-dione;
3-(4-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)phenyl)piperidine-2,6-dione;
3-(2-fluoro-4-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)phenyl)piperidine-2,6-dione;
3-(3-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)phenyl)piperidine-2,6-dione;
3-(2-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)phenyl)piperidine-2,6-dione;
(R)-1-(4-(((1-(6-(6-(2-(3-fluorophenyl)pyrrolidin-1-yl)imidazo[1,2-b]pyridazin-3-yl)pyridin-2-yl)piperidin-4-yl)(methyl)amino)methyl)phenyl)dihydropyrimidine-2,4(1H,3H)-dione;
(R)-1-(3-fluoro-4-(((1-(6-(6-(2-(3-fluorophenyl)pyrrolidin-1-yl)imidazo[1,2-b]pyridazin-3-yl)pyridin-2-yl)piperidin-4-yl)(methyl)amino)methyl)phenyl)dihydropyrimidine-2,4(1H,3H)-dione;
(R)-1-(3-(((1-(6-(6-(2-(3-fluorophenyl)pyrrolidin-1-yl)imidazo[1,2-b]pyridazin-3-yl)pyridin-2-yl)piperidin-4-yl)(methyl)amino)methyl)phenyl)dihydropyrimidine-2,4(1H,3H)-dione;
(R)-1-(2-(((1-(6-(6-(2-(3-fluorophenyl)pyrrolidin-1-yl)imidazo[1,2-b]pyridazin-3-yl)pyridin-2-yl)piperidin-4-yl)(methyl)amino)methyl)phenyl)dihydropyrimidine-2,4(1H,3H)-dione;
(R)-1-(6-(((1-(6-(6-(2-(3-fluorophenyl)pyrrolidin-1-yl)imidazo[1,2-b]pyridazin-3-yl)pyridin-2-yl)piperidin-4-yl)(methyl)amino)methyl)pyridin-3-yl)dihydropyrimidine-2,4(1H,3H)-dione;
(R)-1-(6-(((1-(6-(6-(2-(3-fluorophenyl)pyrrolidin-1-yl)imidazo[1,2-b]pyridazin-3-yl)pyridin-2-yl)piperidin-4-yl)(methyl)amino)methyl)pyridazin-3-yl)dihydropyrimidine-2,4(1H,3H)-dione;
(R)-1-(4-(((1-(6-(6-(2-(3-fluorophenyl)pyrrolidin-1-yl)imidazo[1,2-b]pyridazin-3-yl)pyridin-2-yl)piperidin-4-yl)(methyl)amino)methyl)pyridin-3-yl)dihydropyrimidine-2,4(1H,3H)-dione;
3-((4-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)phenyl)amino)piperidine-2,6-dione;
3-((3-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)phenyl)amino)piperidine-2,6-dione;
3-((2-(((1-(6-(6-((R)-2-(3-fluorophenyl)pyrrolidin-1-yl)imidazo[1,2-b]pyridazin-3-yl)pyridin-2-yl)piperidin-4-yl)(methyl)amino)methyl)phenyl)amino)piperidine-2,6-dione;
3-(4-((4-(4-(6-(6-((R)-2-(3-fluorophenyl)pyrrolidin-1-yl)imidazo[1,2-b]pyridazin-3-yl)pyridin-2-yl)piperazin-1-yl)piperidin-1-yl)methyl)phenyl)piperidine-2,6-dione;
3-(2-fluoro-4-((4-(4-(6-(6-((R)-2-(3-fluorophenyl)pyrrolidin-1-yl)imidazo[1,2-b]pyridazin-3-yl)pyridin-2-yl)piperazin-1-yl)piperidin-1-yl)methyl)phenyl)piperidine-2,6-dione;
3-(3-((4-(4-(6-(6-((R)-2-(3-fluorophenyl)pyrrolidin-1-yl)imidazo[1,2-b]pyridazin-3-yl)pyridin-2-yl)piperazin-1-yl)piperidin-1-yl)methyl)phenyl)piperidine-2,6-dione;
3-(2-((4-(4-(6-(6-((R)-2-(3-fluorophenyl)pyrrolidin-1-yl)imidazo[1,2-b]pyridazin-3-yl)pyridin-2-yl)piperazin-1-yl)piperidin-1-yl)methyl)phenyl)piperidine-2,6-dione;
3-(4-((4-(6-(5-((R)-2-(2,4-difluorophenyl)pyrrolidin-1-yl)pyrazolo[1,5-a]pyrimidin-3-yl)pyridin-2-yl)piperazin-1-yl)methyl)phenyl)piperidine-2,6-dione;
3-(4-((4-(6-(5-((R)-2-(2,4-difluorophenyl)pyrrolidin-1-yl)pyrazolo[1,5-a]pyrimidin-3-yl)pyridin-2-yl)piperazin-1-yl)methyl)-2-fluorophenyl)piperidine-2,6-dione;
3-(3-((4-(6-(5-((R)-2-(2,4-difluorophenyl)pyrrolidin-1-yl)pyrazolo[1,5-a]pyrimidin-3-yl)pyridin-2-yl)piperazin-1-yl)methyl)phenyl)piperidine-2,6-dione;
3-(2-((4-(6-(5-((R)-2-(2,4-difluorophenyl)pyrrolidin-1-yl)pyrazolo[1,5-a]pyrimidin-3-yl)pyridin-2-yl)piperazin-1-yl)methyl)phenyl)piperidine-2,6-dione;
(R)-1-(4-((4-(6-(5-(2-(2,4-difluorophenyl)pyrrolidin-1-yl)pyrazolo[1,5-a]pyrimidin-3-yl)pyridin-2-yl)piperazin-1-yl)methyl)phenyl)dihydropyrimidine-2,4(1H,3H)-dione;
(R)-1-(4-((4-(6-(5-(2-(2,4-difluorophenyl)pyrrolidin-1-yl)pyrazolo[1,5-a]pyrimidin-3-yl)pyridin-2-yl)piperazin-1-yl)methyl)-3-fluorophenyl)dihydropyrimidine-2,4(1H,3H)-dione;
(R)-1-(3-((4-(6-(5-(2-(2,4-difluorophenyl)pyrrolidin-1-yl)pyrazolo[1,5-a]pyrimidin-3-yl)pyridin-2-yl)piperazin-1-yl)methyl)phenyl)dihydropyrimidine-2,4(1H,3H)-dione;
(R)-1-(2-((4-(6-(5-(2-(2,4-difluorophenyl)pyrrolidin-1-yl)pyrazolo[1,5-a]pyrimidin-3-yl)pyridin-2-yl)piperazin-1-yl)methyl)phenyl)dihydropyrimidine-2,4(1H,3H)-dione;
(R)-1-(6-((4-(6-(5-(2-(2,4-difluorophenyl)pyrrolidin-1-yl)pyrazolo[1,5-a]pyrimidin-3-yl)pyridin-2-yl)piperazin-1-yl)methyl)pyridin-3-yl)dihydropyrimidine-2,4(1H,3H)-dione;
(R)-1-(6-((4-(6-(5-(2-(2,4-difluorophenyl)pyrrolidin-1-yl)pyrazolo[1,5-a]pyrimidin-3-yl)pyridin-2-yl)piperazin-1-yl)methyl)pyridazin-3-yl)dihydropyrimidine-2,4(1H,3H)-dione;
(R)-1-(4-((4-(6-(5-(2-(2,4-difluorophenyl)pyrrolidin-1-yl)pyrazolo[1,5-a]pyrimidin-3-yl)pyridin-2-yl)piperazin-1-yl)methyl)pyridin-3-yl)dihydropyrimidine-2,4(1H,3H)-dione;
3-((4-((4-(6-(5-((R)-2-(2,4-difluorophenyl)pyrrolidin-1-yl)pyrazolo[1,5-a]pyrimidin-3-yl)pyridin-2-yl)piperazin-1-yl)methyl)phenyl)amino)piperidine-2,6-dione;
3-((3-((4-(6-(5-((R)-2-(2,4-difluorophenyl)pyrrolidin-1-yl)pyrazolo[1,5-a]pyrimidin-3-yl)pyridin-2-yl)piperazin-1-yl)methyl)phenyl)amino)piperidine-2,6-dione;
3-((2-((4-(6-(5-((R)-2-(2,4-difluorophenyl)pyrrolidin-1-yl)pyrazolo[1,5-a]pyrimidin-3-yl)pyridin-2-yl)piperazin-1-yl)methyl)phenyl)amino)piperidine-2,6-dione;
3-(4-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)phenyl)piperidine-2,6-dione;
3-(4-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)-2-fluorophenyl)piperidine-2,6-dione;
3-(3-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)phenyl)piperidine-2,6-dione;
3-(2-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)phenyl)piperidine-2,6-dione;
(R)-1-(4-(((1-(6-(5-(2-(2,4-difluorophenyl)pyrrolidin-1-yl)pyrazolo[1,5-a]pyrimidin-3-yl)pyridin-2-yl)piperidin-4-yl)(methyl)amino)methyl)phenyl)dihydropyrimidine-2,4(1H,3H)-dione;
(R)-1-(4-(((1-(6-(5-(2-(2,4-difluorophenyl)pyrrolidin-1-yl)pyrazolo[1,5-a]pyrimidin-3-yl)pyridin-2-yl)piperidin-4-yl)(methyl)amino)methyl)-3-fluorophenyl)dihydropyrimidine-2,4(1H,3H)-dione;
(R)-1-(3-(((1-(6-(5-(2-(2,4-difluorophenyl)pyrrolidin-1-yl)pyrazolo[1,5-a]pyrimidin-3-yl)pyridin-2-yl)piperidin-4-yl)(methyl)amino)methyl)phenyl)dihydropyrimidine-2,4(1H,3H)-dione;
(R)-1-(2-(((1-(6-(5-(2-(2,4-difluorophenyl)pyrrolidin-1-yl)pyrazolo[1,5-a]pyrimidin-3-yl)pyridin-2-yl)piperidin-4-yl)(methyl)amino)methyl)phenyl)dihydropyrimidine-2,4(1H,3H)-dione;
(R)-1-(6-(((1-(6-(5-(2-(2,4-difluorophenyl)pyrrolidin-1-yl)pyrazolo[1,5-a]pyrimidin-3-yl)pyridin-2-yl)piperidin-4-yl)(methyl)amino)methyl)pyridin-3-yl)dihydropyrimidine-2,4(1H,3H)-dione;
(R)-1-(6-(((1-(6-(5-(2-(2,4-difluorophenyl)pyrrolidin-1-yl)pyrazolo[1,5-a]pyrimidin-3-yl)pyridin-2-yl)piperidin-4-yl)(methyl)amino)methyl)pyridazin-3-yl)dihydropyrimidine-2,4(1H,3H)-dione;
(R)-1-(4-(((1-(6-(5-(2-(2,4-difluorophenyl)pyrrolidin-1-yl)pyrazolo[1,5-a]pyrimidin-3-yl)pyridin-2-yl)piperidin-4-yl)(methyl)amino)methyl)pyridin-3-yl)dihydropyrimidine-2,4(1H,3H)-dione;
3-((4-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)phenyl)amino)piperidine-2,6-dione;
3-((3-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)phenyl)amino)piperidine-2,6-dione;
3-((2-(((1-(6-(5-((R)-2-(2,4-difluorophenyl)pyrrolidin-1-yl)pyrazolo[1,5-a]pyrimidin-3-yl)pyridin-2-yl)piperidin-4-yl)(methyl)amino)methyl)phenyl)amino)piperidine-2,6-dione;
3-(4-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)phenyl)piperidine-2,6-dione;
3-(4-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)-2-fluorophenyl)piperidine-2,6-dione;
3-(3-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)phenyl)piperidine-2,6-dione;
3-(2-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)phenyl)piperidine-2,6-dione;
(R)-1-(4-((4-(4-(6-(5-(2-(2,4-difluorophenyl)pyrrolidin-1-yl)pyrazolo[1,5-a]pyrimidin-3-yl)pyridin-2-yl)piperazin-1-yl)piperidin-1-yl)methyl)phenyl)dihydropyrimidine-2,4(1H,3H)-dione;
(R)-1-(4-((4-(4-(6-(5-(2-(2,4-difluorophenyl)pyrrolidin-1-yl)pyrazolo[1,5-a]pyrimidin-3-yl)pyridin-2-yl)piperazin-1-yl)piperidin-1-yl)methyl)-3-fluorophenyl)dihydropyrimidine-2,4(1H,3H)-dione;
(R)-1-(3-((4-(4-(6-(5-(2-(2,4-difluorophenyl)pyrrolidin-1-yl)pyrazolo[1,5-a]pyrimidin-3-yl)pyridin-2-yl)piperazin-1-yl)piperidin-1-yl)methyl)phenyl)dihydropyrimidine-2,4(1H,3H)-dione;
(R)-1-(2-((4-(4-(6-(5-(2-(2,4-difluorophenyl)pyrrolidin-1-yl)pyrazolo[1,5-a]pyrimidin-3-yl)pyridin-2-yl)piperazin-1-yl)piperidin-1-yl)methyl)phenyl)dihydropyrimidine-2,4(1H,3H)-dione;
(R)-1-(6-((4-(4-(6-(5-(2-(2,4-difluorophenyl)pyrrolidin-1-yl)pyrazolo[1,5-a]pyrimidin-3-yl)pyridin-2-yl)piperazin-1-yl)piperidin-1-yl)methyl)pyridin-3-yl)dihydropyrimidine-2,4(1H,3H)-dione;
(R)-1-(6-((4-(4-(6-(5-(2-(2,4-difluorophenyl)pyrrolidin-1-yl)pyrazolo[1,5-a]pyrimidin-3-yl)pyridin-2-yl)piperazin-1-yl)piperidin-1-yl)methyl)pyridazin-3-yl)dihydropyrimidine-2,4(1H,3H)-dione;
(R)-1-(4-((4-(4-(6-(5-(2-(2,4-difluorophenyl)pyrrolidin-1-yl)pyrazolo[1,5-a]pyrimidin-3-yl)pyridin-2-yl)piperazin-1-yl)piperidin-1-yl)methyl)pyridin-3-yl)dihydropyrimidine-2,4(1H,3H)-dione;
3-((4-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)phenyl)amino)piperidine-2,6-dione;
3-((3-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)phenyl)amino)piperidine-2,6-dione;
3-((2-((4-(4-(6-(5-((R)-2-(2,4-difluorophenyl)pyrrolidin-1-yl)pyrazolo[1,5-a]pyrimidin-3-yl)pyridin-2-yl)piperazin-1-yl)piperidin-1-yl)methyl)phenyl)amino)piperidine-2,6-dione;
3-(4-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)phenyl)piperidine-2,6-dione;
3-(4-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)-2-fluorophenyl)piperidine-2,6-dione;
3-(3-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)phenyl)piperidine-2,6-dione;
3-(2-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)phenyl)piperidine-2,6-dione;
(R)-1-(4-((4-(6-(5-(2-(2,5-difluorophenyl)pyrrolidin-1-yl)pyrazolo[1,5-a]pyrimidin-3-yl)pyridin-2-yl)piperazin-1-yl)methyl)phenyl)dihydropyrimidine-2,4(1H,3H)-dione;
(R)-1-(4-((4-(6-(5-(2-(2,5-difluorophenyl)pyrrolidin-1-yl)pyrazolo[1,5-a]pyrimidin-3-yl)pyridin-2-yl)piperazin-1-yl)methyl)-3-fluorophenyl)dihydropyrimidine-2,4(1H,3H)-dione;
(R)-1-(3-((4-(6-(5-(2-(2,5-difluorophenyl)pyrrolidin-1-yl)pyrazolo[1,5-a]pyrimidin-3-yl)pyridin-2-yl)piperazin-1-yl)methyl)phenyl)dihydropyrimidine-2,4(1H,3H)-dione;
(R)-1-(2-((4-(6-(5-(2-(2,5-difluorophenyl)pyrrolidin-1-yl)pyrazolo[1,5-a]pyrimidin-3-yl)pyridin-2-yl)piperazin-1-yl)methyl)phenyl)dihydropyrimidine-2,4(1H,3H)-dione;
(R)-1-(6-((4-(6-(5-(2-(2,5-difluorophenyl)pyrrolidin-1-yl)pyrazolo[1,5-a]pyrimidin-3-yl)pyridin-2-yl)piperazin-1-yl)methyl)pyridin-3-yl)dihydropyrimidine-2,4(1H,3H)-dione;
(R)-1-(6-((4-(6-(5-(2-(2,5-difluorophenyl)pyrrolidin-1-yl)pyrazolo[1,5-a]pyrimidin-3-yl)pyridin-2-yl)piperazin-1-yl)methyl)pyridazin-3-yl)dihydropyrimidine-2,4(1H,3H)-dione;
(R)-1-(4-((4-(6-(5-(2-(2,5-difluorophenyl)pyrrolidin-1-yl)pyrazolo[1,5-a]pyrimidin-3-yl)pyridin-2-yl)piperazin-1-yl)methyl)pyridin-3-yl)dihydropyrimidine-2,4(1H,3H)-dione;
3-((4-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)phenyl)amino)piperidine-2,6-dione;
3-((3-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)phenyl)amino)piperidine-2,6-dione;
3-((2-((4-(6-(5-((R)-2-(2,5-difluorophenyl)pyrrolidin-1-yl)pyrazolo[1,5-a]pyrimidin-3-yl)pyridin-2-yl)piperazin-1-yl)methyl)phenyl)amino)piperidine-2,6-dione;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-(2,6-dioxopiperidin-3-yl)benzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-(2,6-dioxopiperidin-3-yl)-2-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-(2,6-dioxopiperidin-3-yl)-3-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(2-(2,6-dioxopiperidin-3-yl)benzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(2-(2,6-dioxopiperidin-3-yl)-6-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(2-(2,6-dioxopiperidin-3-yl)-5-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(2-(2,6-dioxopiperidin-3-yl)-4-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(2-(2,6-dioxopiperidin-3-yl)-3-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(3-(2,6-dioxopiperidin-3-yl)benzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(3-(2,6-dioxopiperidin-3-yl)-2-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(5-(2,6-dioxopiperidin-3-yl)-2-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(3-(2,6-dioxopiperidin-3-yl)-5-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(3-(2,6-dioxopiperidin-3-yl)-4-fluorobenzyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(4-(2,4-dioxotetrahydropyrimidin-1(2H)-yl)benzyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(4-(2,4-dioxotetrahydropyrimidin-1(2H)-yl)-2-fluorobenzyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(4-(2,4-dioxotetrahydropyrimidin-1(2H)-yl)-3-fluorobenzyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(2-(2,4-dioxotetrahydropyrimidin-1(2H)-yl)benzyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(2-(2,4-dioxotetrahydropyrimidin-1(2H)-yl)-6-fluorobenzyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(2-(2,4-dioxotetrahydropyrimidin-1(2H)-yl)-5-fluorobenzyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(2-(2,4-dioxotetrahydropyrimidin-1(2H)-yl)-4-fluorobenzyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(2-(2,4-dioxotetrahydropyrimidin-1(2H)-yl)-3-fluorobenzyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(3-(2,4-dioxotetrahydropyrimidin-1(2H)-yl)benzyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(3-(2,4-dioxotetrahydropyrimidin-1(2H)-yl)-2-fluorobenzyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(5-(2,4-dioxotetrahydropyrimidin-1(2H)-yl)-2-fluorobenzyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(3-(2,4-dioxotetrahydropyrimidin-1(2H)-yl)-5-fluorobenzyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(3-(2,4-dioxotetrahydropyrimidin-1(2H)-yl)-4-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-((2,6-dioxopiperidin-3-yl)amino)benzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-((2,6-dioxopiperidin-3-yl)amino)-2-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-((2,6-dioxopiperidin-3-yl)amino)-3-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(2-((2,6-dioxopiperidin-3-yl)amino)benzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(2-((2,6-dioxopiperidin-3-yl)amino)-6-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(2-((2,6-dioxopiperidin-3-yl)amino)-5-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(2-((2,6-dioxopiperidin-3-yl)amino)-4-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(2-((2,6-dioxopiperidin-3-yl)amino)-3-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(3-((2,6-dioxopiperidin-3-yl)amino)benzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(5-((2,6-dioxopiperidin-3-yl)amino)-2-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(3-((2,6-dioxopiperidin-3-yl)amino)-5-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(3-((2,6-dioxopiperidin-3-yl)amino)-4-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(3-((2,6-dioxopiperidin-3-yl)amino)-2-fluorobenzyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)pyridin-2-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)-3-fluoropyridin-2-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)-4-fluoropyridin-2-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)-6-fluoropyridin-2-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-(2,6-dioxopiperidin-3-yl)pyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-(2,6-dioxopiperidin-3-yl)-4-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-(2,6-dioxopiperidin-3-yl)-5-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-(2,6-dioxopiperidin-3-yl)-2-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)pyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-4-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-5-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-6-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)pyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)-4-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)-2-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)-6-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((3-(2,6-dioxopiperidin-3-yl)pyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((3-(2,6-dioxopiperidin-3-yl)-5-fluoropyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)-2-fluoropyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((3-(2,6-dioxopiperidin-3-yl)-2-fluoropyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)pyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-3-fluoropyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-5-fluoropyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-6-fluoropyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-(2,6-dioxopiperidin-3-yl)pyridazin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-(2,6-dioxopiperidin-3-yl)-4-fluoropyridazin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-(2,6-dioxopiperidin-3-yl)-5-fluoropyridazin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)pyridazin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)-4-fluoropyridazin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)-6-fluoropyridazin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-(2,6-dioxopiperidin-3-yl)pyrimidin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-(2,6-dioxopiperidin-3-yl)pyrazin-2-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-(2,6-dioxopiperidin-3-yl)pyrazin-2-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((3-(2,6-dioxopiperidin-3-yl)pyridazin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-(2,6-dioxopiperidin-3-yl)pyridazin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)pyrimidin-5-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((4-(2,6-dioxopiperidin-3-yl)pyrimidin-5-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)pyridin-2-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)-3-fluoropyridin-2-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)-4-fluoropyridin-2-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)-6-fluoropyridin-2-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((6-(2,4-dioxotetrahydropyrimidin-1(2H)-yl)pyridin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((6-(2,4-dioxotetrahydropyrimidin-1(2H)-yl)-4-fluoropyridin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((6-(2,4-dioxotetrahydropyrimidin-1(2H)-yl)-5-fluoropyridin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((6-(2,4-dioxotetrahydropyrimidin-1(2H)-yl)-2-fluoropyridin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((2-(2,4-dioxotetrahydropyrimidin-1(2H)-yl)pyridin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((2-(2,4-dioxotetrahydropyrimidin-1(2H)-yl)-4-fluoropyridin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((2-(2,4-dioxotetrahydropyrimidin-1(2H)-yl)-5-fluoropyridin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((2-(2,4-dioxotetrahydropyrimidin-1(2H)-yl)-6-fluoropyridin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)pyridin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)-4-fluoropyridin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)-2-fluoropyridin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)-6-fluoropyridin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((3-(2,4-dioxotetrahydropyrimidin-1(2H)-yl)pyridin-4-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((3-(2,4-dioxotetrahydropyrimidin-1(2H)-yl)-5-fluoropyridin-4-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)-2-fluoropyridin-4-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((3-(2,4-dioxotetrahydropyrimidin-1(2H)-yl)-2-fluoropyridin-4-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((2-(2,4-dioxotetrahydropyrimidin-1(2H)-yl)pyridin-4-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((2-(2,4-dioxotetrahydropyrimidin-1(2H)-yl)-3-fluoropyridin-4-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((2-(2,4-dioxotetrahydropyrimidin-1(2H)-yl)-5-fluoropyridin-4-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((2-(2,4-dioxotetrahydropyrimidin-1(2H)-yl)-6-fluoropyridin-4-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((6-(2,4-dioxotetrahydropyrimidin-1(2H)-yl)pyridazin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((6-(2,4-dioxotetrahydropyrimidin-1(2H)-yl)-4-fluoropyridazin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((6-(2,4-dioxotetrahydropyrimidin-1(2H)-yl)-5-fluoropyridazin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)pyridazin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)-4-fluoropyridazin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)-6-fluoropyridazin-3-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((6-(2,4-dioxotetrahydropyrimidin-1(2H)-yl)pyrimidin-4-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)pyrazin-2-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((6-(2,4-dioxotetrahydropyrimidin-1(2H)-yl)pyrazin-2-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((3-(2,4-dioxotetrahydropyrimidin-1(2H)-yl)pyridazin-4-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((6-(2,4-dioxotetrahydropyrimidin-1(2H)-yl)pyridazin-4-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((2-(2,4-dioxotetrahydropyrimidin-1(2H)-yl)pyrimidin-5-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((4-(2,4-dioxotetrahydropyrimidin-1(2H)-yl)pyrimidin-5-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-((2,6-dioxopiperidin-3-yl)amino)pyridin-2-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-((2,6-dioxopiperidin-3-yl)amino)-3-fluoropyridin-2-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-((2,6-dioxopiperidin-3-yl)amino)-4-fluoropyridin-2-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-((2,6-dioxopiperidin-3-yl)amino)-6-fluoropyridin-2-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-((2,6-dioxopiperidin-3-yl)amino)pyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-((2,6-dioxopiperidin-3-yl)amino)-4-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-((2,6-dioxopiperidin-3-yl)amino)-5-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-((2,6-dioxopiperidin-3-yl)amino)-2-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-((2,6-dioxopiperidin-3-yl)amino)pyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-((2,6-dioxopiperidin-3-yl)amino)-4-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-((2,6-dioxopiperidin-3-yl)amino)-5-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-((2,6-dioxopiperidin-3-yl)amino)-6-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-((2,6-dioxopiperidin-3-yl)amino)pyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-((2,6-dioxopiperidin-3-yl)amino)-4-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-((2,6-dioxopiperidin-3-yl)amino)-2-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-((2,6-dioxopiperidin-3-yl)amino)-6-fluoropyridin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((3-((2,6-dioxopiperidin-3-yl)amino)pyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((3-((2,6-dioxopiperidin-3-yl)amino)-5-fluoropyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-((2,6-dioxopiperidin-3-yl)amino)-2-fluoropyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((3-((2,6-dioxopiperidin-3-yl)amino)-2-fluoropyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-((2,6-dioxopiperidin-3-yl)amino)pyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-((2,6-dioxopiperidin-3-yl)amino)-3-fluoropyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-((2,6-dioxopiperidin-3-yl)amino)-5-fluoropyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-((2,6-dioxopiperidin-3-yl)amino)-6-fluoropyridin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-((2,6-dioxopiperidin-3-yl)amino)pyridazin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-((2,6-dioxopiperidin-3-yl)amino)-4-fluoropyridazin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-((2,6-dioxopiperidin-3-yl)amino)-5-fluoropyridazin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-((2,6-dioxopiperidin-3-yl)amino)pyridazin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-((2,6-dioxopiperidin-3-yl)amino)-4-fluoropyridazin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-((2,6-dioxopiperidin-3-yl)amino)-6-fluoropyridazin-3-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-((2,6-dioxopiperidin-3-yl)amino)pyrimidin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((5-((2,6-dioxopiperidin-3-yl)amino)pyrazin-2-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-((2,6-dioxopiperidin-3-yl)amino)pyrazin-2-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((3-((2,6-dioxopiperidin-3-yl)amino)pyridazin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((6-((2,6-dioxopiperidin-3-yl)amino)pyridazin-4-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-((2,6-dioxopiperidin-3-yl)amino)pyrimidin-5-yl)methyl)piperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((4-((2,6-dioxopiperidin-3-yl)amino)pyrimidin-5-yl)methyl)piperazin-1-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((5-(2,4-dioxotetrahydropyrimidin-1(2H)-yl)pyridin-2-yl)methyl)piperazin-1-yl-2,2,3,3,5,5,6,6-d8)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-(2,6-dioxopiperidin-3-yl)benzyl)piperazin-1-yl-2,2,3,3,5,5,6,6-d8)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(4-(2,4-dioxotetrahydropyrimidin-1(2H)-yl)benzyl)piperazin-1-yl-2,2,3,3,5,5,6,6-d8)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-(3-(2,4-dioxotetrahydropyrimidin-1(2H)-yl)benzyl)piperazin-1-yl-2,2,3,3,5,5,6,6-d8)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-(2,6-dioxopiperidin-3-yl)benzyl)-3,5-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-(2,6-dioxopiperidin-3-yl)benzyl)-2,6-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(6-(4-(2,6-dioxopiperidin-3-yl)benzyl)-3,6-diazabicyclo[3.1.1]heptan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-(3-(2,4-dioxotetrahydropyrimidin-1(2H)-yl)benzyl)-3,6-diazabicyclo[3.1.1]heptan-6-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-(4-(2,4-dioxotetrahydropyrimidin-1(2H)-yl)benzyl)-3,6-diazabicyclo[3.1.1]heptan-6-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-(4-(2,6-dioxopiperidin-3-yl)benzyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-(3-(2,4-dioxotetrahydropyrimidin-1(2H)-yl)benzyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-(4-(2,6-dioxopiperidin-3-yl)benzyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((R)-4-((1R,4R)-5-(3-(2,4-dioxotetrahydropyrimidin-1(2H)-yl)benzyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((5-(2,6-dioxopiperidin-3-yl)pyridin-3-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((R)-4-((1R,4R)-5-((5-(2,4-dioxotetrahydropyrimidin-1(2H)-yl)pyridin-2-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((R)-4-((1R,4R)-5-((2-(2,4-dioxotetrahydropyrimidin-1(2H)-yl)pyridin-4-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((R)-4-((1R,4R)-5-((6-(2,4-dioxotetrahydropyrimidin-1(2H)-yl)pyridin-3-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((R)-4-((1R,4R)-5-((5-(2,4-dioxotetrahydropyrimidin-1(2H)-yl)-2-fluoropyridin-3-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-(3-((2,6-dioxopiperidin-3-yl)amino)benzyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-(4-(2,6-dioxopiperidin-3-yl)benzyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-(3-(2,4-dioxotetrahydropyrimidin-1(2H)-yl)benzyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((5-(2,4-dioxotetrahydropyrimidin-1(2H)-yl)pyrazin-2-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-(3-((2,6-dioxopiperidin-3-yl)amino)benzyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-(4-(2,6-dioxopiperidin-3-yl)benzyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-(3-(2,4-dioxotetrahydropyrimidin-1(2H)-yl)benzyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-(3-((2,6-dioxopiperidin-3-yl)amino)benzyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-(4-(2,6-dioxopiperidin-3-yl)benzyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-(2,4-dioxotetrahydropyrimidin-1(2H)-yl)benzyl)-3,5-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-(2,4-dioxotetrahydropyrimidin-1(2H)-yl)benzyl)-2,6-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(6-(4-(2,4-dioxotetrahydropyrimidin-1(2H)-yl)benzyl)-3,6-diazabicyclo[3.1.1]heptan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-(4-(2,4-dioxotetrahydropyrimidin-1(2H)-yl)benzyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((5-(2,6-dioxopiperidin-3-yl)pyridin-3-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((5-(2,4-dioxotetrahydropyrimidin-1(2H)-yl)pyridin-2-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,4-dioxotetrahydropyrimidin-1(2H)-yl)pyridin-4-yl)methyl)-2,5-diazabicyclo[ 2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,4-dioxotetrahydropyrimidin-1(2H)-yl)-5-fluoropyridin-4-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((6-(2,4-dioxotetrahydropyrimidin-1(2H)-yl)pyridin-3-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((5-(2,4-dioxotetrahydropyrimidin-1(2H)-yl)pyridin-3-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((5-(2,4-dioxotetrahydropyrimidin-1(2H)-yl)-2-fluoropyridin-3-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-(3-((2,6-dioxopiperidin-3-yl)amino)benzyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((R)-4-((1R,4R)-5-(4-(2,4-dioxotetrahydropyrimidin-1(2H)-yl)benzyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-(4-(2,4-dioxotetrahydropyrimidin-1(2H)-yl)benzyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((5-(2,6-dioxopiperidin-3-yl)-2-fluoropyridin-3-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((5-(2,6-dioxopiperidin-3-yl)pyridin-2-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((5-(2,6-dioxopiperidin-3-yl)pyridin-3-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((5-(2,4-dioxotetrahydropyrimidin-1(2H)-yl)pyridin-2-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,4-dioxotetrahydropyrimidin-1(2H)-yl)pyridin-4-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,4-dioxotetrahydropyrimidin-1(2H)-yl)-5-fluoropyridin-4-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((6-(2,4-dioxotetrahydropyrimidin-1(2H)-yl)pyridin-3-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((5-(2,4-dioxotetrahydropyrimidin-1(2H)-yl)pyridin-3-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((5-(2,4-dioxotetrahydropyrimidin-1(2H)-yl)-2-fluoropyridin-3-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-(4-(2,4-dioxotetrahydropyrimidin-1(2H)-yl)benzyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((5-(2,4-dioxotetrahydropyrimidin-1(2H)-yl)pyridin-2-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,4-dioxotetrahydropyrimidin-1(2H)-yl)-5-fluoropyridin-4-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((6-(2,4-dioxotetrahydropyrimidin-1(2H)-yl)pyridin-3-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((R)-4-((1S,4S)-5-(4-(2,4-dioxotetrahydropyrimidin-1(2H)-yl)benzyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-((2,6-dioxopiperidin-3-yl)amino)benzyl)-3,5-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-(4-((2,6-dioxopiperidin-3-yl)amino)benzyl)-2,6-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(6-(4-((2,6-dioxopiperidin-3-yl)amino)benzyl)-3,6-diazabicyclo[3.1.1]heptan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-(4-((2,6-dioxopiperidin-3-yl)amino)benzyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-(4-((2,6-dioxopiperidin-3-yl)amino)benzyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((R)-4-((1R,4R)-5-((5-(2,4-dioxotetrahydropyrimidin-1(2H)-yl)pyridin-3-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-(4-((2,6-dioxopiperidin-3-yl)amino)benzyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-(4-((2,6-dioxopiperidin-3-yl)amino)benzyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-(4-((2,6-dioxopiperidin-3-yl)amino)benzyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
(R)-4-(4-((4'-chloro-4,4-dimethyl-3,4,5,6-tetrahydro-[1,1'-biphenyl]-2-yl)methyl)piperazin-1-yl)-N-((4-((4-(4-((6-(2,4-dioxotetrahydropyrimidin-1(2H)-yl)pyridin-3-yl)methyl)piperazin-1-yl-2,2,3,3,5,5,6,6-d8)-1-(phenylthio)butan-2-yl)amino)-3-((trifluoromethyl)sulfonyl)phenyl)sulfonyl)benzamide;
3-(4-((4-((4-(((R)-1-(3-amino-5-(trifluoromethyl)phenyl)ethyl)amino)-7-methoxy-2-methylquinazolin-6-yl)oxy)piperidin-1-yl)methyl)phenyl)piperidine-2,6-dione;
3-(4-((4-((4-(((R)-1-(3-amino-5-(trifluoromethyl)phenyl)ethyl)amino)-7-methoxy-2-methylquinazolin-6-yl)oxy)piperidin-1-yl)methyl)-2-fluorophenyl)piperidine-2,6-dione;
3-(3-((4-((4-(((R)-1-(3-amino-5-(trifluoromethyl)phenyl)ethyl)amino)-7-methoxy-2-methylquinazolin-6-yl)oxy)piperidin-1-yl)methyl)phenyl)piperidine-2,6-dione;
3-(2-((4-((4-(((R)-1-(3-amino-5-(trifluoromethyl)phenyl)ethyl)amino)-7-methoxy-2-methylquinazolin-6-yl)oxy)piperidin-1-yl)methyl)phenyl)piperidine-2,6-dione;
(R)-1-(4-((4-((4-((1-(3-amino-5-(trifluoromethyl)phenyl)ethyl)amino)-7-methoxy-2-methylquinazolin-6-yl)oxy)piperidin-1-yl)methyl)phenyl)dihydropyrimidine-2,4(1H,3H)-dione;
(R)-1-(4-((4-((4-((1-(3-amino-5-(trifluoromethyl)phenyl)ethyl)amino)-7-methoxy-2-methylquinazolin-6-yl)oxy)piperidin-1-yl)methyl)-2-fluorophenyl)dihydropyrimidine-2,4(1H,3H)-dione;
(R)-1-(3-((4-((4-((1-(3-amino-5-(trifluoromethyl)phenyl)ethyl)amino)-7-methoxy-2-methylquinazolin-6-yl)oxy)piperidin-1-yl)methyl)phenyl)dihydropyrimidine-2,4(1H,3H)-dione;
(R)-1-(2-((4-((4-((1-(3-amino-5-(trifluoromethyl)phenyl)ethyl)amino)-7-methoxy-2-methylquinazolin-6-yl)oxy)piperidin-1-yl)methyl)phenyl)dihydropyrimidine-2,4(1H,3H)-dione;
(R)-1-(6-((4-((4-((1-(3-amino-5-(trifluoromethyl)phenyl)ethyl)amino)-7-methoxy-2-methylquinazolin-6-yloxy)piperidin-1-yl)methyl)pyridin-3-yl)dihydropyrimidine-2,4(1H,3H)-dione;
(R)-1-(6-((4-((4-((1-(3-amino-5-(trifluoromethyl)phenyl)ethyl)amino)-7-methoxy-2-methylquinazolin-6-yl)oxy)piperidin-1-yl)methyl)pyridazin-3-yl)dihydropyrimidine-2,4(1H,3H)-dione;
(R)-1-(4-((4-((4-((1-(3-amino-5-(trifluoromethyl)phenyl)ethyl)amino)-7-methoxy-2-methylquinazolin-6-yloxy)piperidin-1-yl)methyl)pyridin-3-yl)dihydropyrimidine-2,4(1H,3H)-dione;
3-((4-((4-((4-(((R)-1-(3-amino-5-(trifluoromethyl)phenyl)ethyl)amino)-7-methoxy-2-methylquinazolin-6-yl)oxy)piperidin-1-yl)methyl)phenyl)amino)piperidine-2,6-dione;
3-((3-((4-((4-(((R)-1-(3-amino-5-(trifluoromethyl)phenyl)ethyl)amino)-7-methoxy-2-methylquinazolin-6-yl)oxy)piperidin-1-yl)methyl)phenyl)amino)piperidine-2,6-dione;
3-((2-((4-((4-(((R)-1-(3-amino-5-(trifluoromethyl)phenyl)ethyl)amino)-7-methoxy-2-methylquinazolin-6-yl)oxy)piperidin-1-yl)methyl)phenyl)amino)piperidine-2,6-dione;
2-(4-(((4-(2,6-dioxopiperidin-3-yl)benzyl)(methyl)amino)methyl)phenyl)-5-fluorobenzofuran-7-carboxamide;
2-(4-(((4-(2,6-dioxopiperidin-3-yl)-3-fluorobenzyl)(methyl)amino)methyl)phenyl)-5-fluorobenzofuran-7-carboxamide;
2-(4-(((3-(2,6-dioxopiperidin-3-yl)benzyl)(methyl)amino)methyl)phenyl)-5-fluorobenzofuran-7-carboxamide;
2-(4-(((2-(2,6-dioxopiperidin-3-yl)benzyl)(methyl)amino)methyl)phenyl)-5-fluorobenzofuran-7-carboxamide;
2-(4-(((4-(2,4-dioxotetrahydropyrimidin-1(2H)-yl)benzyl)(methyl)amino)methyl)phenyl)-5-fluorobenzofuran-7-carboxamide;
2-(4-(((4-(2,4-dioxotetrahydropyrimidin-1(2H)-yl)-2-fluorobenzyl)(methyl)amino)methyl)phenyl)-5-fluorobenzofuran-7-carboxamide;
2-(4-(((3-(2,4-dioxotetrahydropyrimidin-1(2H)-yl)benzyl)(methyl)amino)methyl)phenyl)-5-fluorobenzofuran-7-carboxamide;
2-(4-(((2-(2,4-dioxotetrahydropyrimidin-1(2H)-yl)benzyl)(methyl)amino)methyl)phenyl)-5-fluorobenzofuran-7-carboxamide;
2-(4-((((5-(2,4-dioxotetrahydropyrimidin-1(2H)-yl)pyridin-2-yl)methyl)(methyl)amino)methyl)phenyl)-5-fluorobenzofuran-7-carboxamide;
2-(4-((((6-(2,4-dioxotetrahydropyrimidin-1(2H)-yl)pyridazin-3-yl)methyl)(methyl)amino)methyl)phenyl)-5-fluorobenzofuran-7-carboxamide;
2-(4-((((3-(2,4-dioxotetrahydropyrimidin-1(2H)-yl)pyridin-4-yl)methyl)(methyl)amino)methyl)phenyl)-5-fluorobenzofuran-7-carboxamide;
2-(4-(((4-((2,6-dioxopiperidin-3-yl)amino)benzyl)(methyl)amino)methyl)phenyl)-5-fluorobenzofuran-7-carboxamide;
2-(4-(((3-((2,6-dioxopiperidin-3-yl)amino)benzyl)(methyl)amino)methyl)phenyl)-5-fluorobenzofuran-7-carboxamide;
2-(4-(((2-((2,6-dioxopiperidin-3-yl)amino)benzyl)(methyl)amino)methyl)phenyl)-5-fluorobenzofuran-7-carboxamide;
N-(5-(3,5-dimethoxyphenethyl)-1H-pyrazol-3-yl)-4-((3R,5S)-4-(4-(2,6-dioxopiperidin-3-yl)benzyl)-3,5-dimethylpiperazin-1-yl)benzamide;
N-(5-(3,5-dimethoxyphenethyl)-1H-pyrazol-3-yl)-4-((3R,5S)-4-(4-(2,6-dioxopiperidin-3-yl)-3-fluorobenzyl)-3,5-dimethylpiperazin-1-yl)benzamide;
N-(5-(3,5-dimethoxyphenethyl)-1H-pyrazol-3-yl)-4-((3R,5S)-4-(3-(2,6-dioxopiperidin-3-yl)benzyl)-3,5-dimethylpiperazin-1-yl)benzamide;
N-(5-(3,5-dimethoxyphenethyl)-1H-pyrazol-3-yl)-4-((3R,5S)-4-(2-(2,6-dioxopiperidin-3-yl)benzyl)-3,5-dimethylpiperazin-1-yl)benzamide;
3-(4-((4-(4-(3-(quinolin-4-yl)pyrazolo[1,5-a]pyrimidin-6-yl)phenyl)piperazin-1-yl)methyl)phenyl)piperidine-2,6-dione;
3-(2-fluoro-4-((4-(4-(3-(quinolin-4-yl)pyrazolo[1,5-a]pyrimidin-6-yl)phenyl)piperazin-1-yl)methyl)phenyl)piperidine-2,6-dione;
3-(3-((4-(4-(3-(quinolin-4-yl)pyrazolo[1,5-a]pyrimidin-6-yl)phenyl)piperazin-1-yl)methyl)phenyl)piperidine-2,6-dione;
3-(2-((4-(4-(3-(quinolin-4-yl)pyrazolo[1,5-a]pyrimidin-6-yl)phenyl)piperazin-1-yl)methyl)phenyl)piperidine-2,6-dione;
1-(4-((4-(4-(3-(quinolin-4-yl)pyrazolo[1,5-a]pyrimidin-6-yl)phenyl)piperazin-1-yl)methyl)phenyl)dihydropyrimidine-2,4(1H,3H)-dione;
1-(3-fluoro-4-((4-(4-(3-(quinolin-4-yl)pyrazolo[1,5-a]pyrimidin-6-yl)phenyl)piperazin-1-yl)methyl)phenyl)dihydropyrimidine-2,4(1H,3H)-dione;
1-(3-((4-(4-(3-(quinolin-4-yl)pyrazolo[1,5-a]pyrimidin-6-yl)phenyl)piperazin-1-yl)methyl)phenyl)dihydropyrimidine-2,4(1H,3H)-dione;
1-(2-((4-(4-(3-(quinolin-4-yl)pyrazolo[1,5-a]pyrimidin-6-yl)phenyl)piperazin-1-yl)methyl)phenyl)dihydropyrimidine-2,4(1H,3H)-dione;
1-(6-((4-(4-(3-(quinolin-4-yl)pyrazolo[1,5-a]pyrimidin-6-yl)phenyl)piperazin-1-yl)methyl)pyridin-3-yl)dihydropyrimidine-2,4(1H,3H)-dione;
1-(6-((4-(4-(3-(quinolin-4-yl)pyrazolo[1,5-a]pyrimidin-6-yl)phenyl)piperazin-1-yl)methyl)pyridazin-3-yl)dihydropyrimidine-2,4(1H,3H)-dione;
1-(4-((4-(4-(3-(quinolin-4-yl)pyrazolo[1,5-a]pyrimidin-6-yl)phenyl)piperazin-1-yl)methyl)pyridin-3-yl)dihydropyrimidine-2,4(1H,3H)-dione;
3-((4-((4-(4-(3-(quinolin-4-yl)pyrazolo[1,5-a]pyrimidin-6-yl)phenyl)piperazin-1-yl)methyl)phenyl)amino)piperidine-2,6-dione;
3-((3-((4-(4-(3-(quinolin-4-yl)pyrazolo[1,5-a]pyrimidin-6-yl)phenyl)piperazin-1-yl)methyl)phenyl)amino)piperidine-2,6-dione;
3-((2-((4-(4-(3-(quinolin-4-yl)pyrazolo[1,5-a]pyrimidin-6-yl)phenyl)piperazin-1-yl)methyl)phenyl)amino)piperidine-2,6-dione;
3-(4-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)piperidine-2,6-dione;
3-(4-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)-2-fluorophenyl)piperidine-2,6-dione;
3-(3-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)piperidine-2,6-dione;
3-(2-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)piperidine-2,6-dione;
1-(4-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)dihydropyrimidine-2,4(1H,3H)-dione;
1-(4-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)-3-fluorophenyl)dihydropyrimidine-2,4(1H,3H)-dione;
1-(3-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)dihydropyrimidine-2,4(1H,3H)-dione;
1-(2-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)dihydropyrimidine-2,4(1H,3H)-dione;
1-(6-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)pyridin-3-yl)dihydropyrimidine-2,4(1H,3H)-dione;
1-(6-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)pyridazin-3-yl)dihydropyrimidine-2,4(1H,3H)-dione;
1-(4-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)pyridin-3-yl)dihydropyrimidine-2,4(1H,3H)-dione;
3-((4-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)amino)piperidine-2,6-dione;
3-((3-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)amino)piperidine-2,6-dione;
3-((2-((4-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)amino)piperidine-2,6-dione;
3-(4-(((S)-2-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)piperidine-2,6-dione;
3-(4-(((S)-2-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)-2-fluorophenyl)piperidine-2,6-dione;
3-(3-(((S)-2-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)piperidine-2,6-dione;
1-(4-(((S)-2-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)dihydropyrimidine-2,4(1H,3H)-dione;
1-(4-(((S)-2-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)-3-fluorophenyl)dihydropyrimidine-2,4(1H,3H)-dione;
1-(3-(((S)-2-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)dihydropyrimidine-2,4(1H,3H)-dione;
1-(6-(((S)-2-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)pyridin-3-yl)dihydropyrimidine-2,4(1H,3H)-dione;
1-(6-(((S)-2-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)pyridazin-3-yl)dihydropyrimidine-2,4(1H,3H)-dione;
3-((4-(((S)-2-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)amino)piperidine-2,6-dione;
3-((3-(((S)-2-(((4-((1R,5S)-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)methyl)phenyl)amino)piperidine-2,6-dione;
(9R)-N-(1-(4-(4-(4-(2,6-dioxopiperidin-3-yl)benzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(4-(2,6-dioxopiperidin-3-yl)-3-fluorobenzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(3-(2,6-dioxopiperidin-3-yl)benzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(2-(2,6-dioxopiperidin-3-yl)benzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-((5-(2,6-dioxopiperidin-3-yl)-3-fluoropyridin-2-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-((5-(2,6-dioxopiperidin-3-yl)pyridin-3-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-((5-(2,6-dioxopiperidin-3-yl)-2-fluoropyridin-3-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-(2,4-dioxotetrahydropyrimidin-1(2H)-yl)benzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-(2,4-dioxotetrahydropyrimidin-1(2H)-yl)-2-fluorobenzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(3-(2,4-dioxotetrahydropyrimidin-1(2H)-yl)benzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(2-(2,4-dioxotetrahydropyrimidin-1(2H)-yl)benzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-((5-(2,4-dioxotetrahydropyrimidin-1(2H)-yl)pyridin-2-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-((2-(2,4-dioxotetrahydropyrimidin-1(2H)-yl)pyridin-4-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-((2-(2,4-dioxotetrahydropyrimidin-1(2H)-yl)-5-fluoropyridin-4-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-((6-(2,4-dioxotetrahydropyrimidin-1(2H)-yl)pyridin-3-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-((5-(2,4-dioxotetrahydropyrimidin-1(2H)-yl)pyridin-3-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-((5-(2,4-dioxotetrahydropyrimidin-1(2H)-yl)-2-fluoropyridin-3-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-((6-(2,4-dioxotetrahydropyrimidin-1(2H)-yl)pyridazin-3-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-((5-(2,4-dioxotetrahydropyrimidin-1(2H)-yl)pyrazin-2-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-((3-(2,4-dioxotetrahydropyrimidin-1(2H)-yl)pyridin-4-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(4-((2,6-dioxopiperidin-3-yl)amino)benzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(3-((2,6-dioxopiperidin-3-yl)amino)benzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(2-((2,6-dioxopiperidin-3-yl)amino)benzyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-((4-(2,6-dioxopiperidin-3-yl)benzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-((4-(2,6-dioxopiperidin-3-yl)-3-fluorobenzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-((3-(2,6-dioxopiperidin-3-yl)benzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-((2-(2,6-dioxopiperidin-3-yl)benzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(((2-(2,6-dioxopiperidin-3-yl)pyridin-4-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(((5-(2,6-dioxopiperidin-3-yl)pyridin-3-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(((5-(2,6-dioxopiperidin-3-yl)-2-fluoropyridin-3-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-((4-(2,4-dioxotetrahydropyrimidin-1(2H)-yl)benzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-((4-(2,4-dioxotetrahydropyrimidin-1(2H)-yl)-2-fluorobenzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-((3-(2,4-dioxotetrahydropyrimidin-1(2H)-yl)benzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-((2-(2,4-dioxotetrahydropyrimidin-1(2H)-yl)benzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(((5-(2,4-dioxotetrahydropyrimidin-1(2H)-yl)pyridin-2-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(((2-(2,4-dioxotetrahydropyrimidin-1(2H)-yl)pyridin-4-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(((2-(2,4-dioxotetrahydropyrimidin-1(2H)-yl)-5-fluoropyridin-4-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(((6-(2,4-dioxotetrahydropyrimidin-1(2H)-yl)pyridin-3-yl)methyl)(methyl)amino)piperidin-1-yl)benzyl)-1H-pyrazol-4-yl)-9-methyl-6,7,8,9-tetrahydropyrido[3',2':4,5]pyrrolo[1,2-a]pyrazine-2-carboxamide;
(R)-N-(1-(4-(4-(((5-(2,4-dioxotetrahydropyrimidin-1(2H)-yl)pyridin-3-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(((5-(2,4-dioxotetrahydropyrimidin-1(2H)-yl)-2-fluoropyridin-3-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(((6-(2,4-dioxotetrahydropyrimidin-1(2H)-yl)pyridazin-3-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(((5-(2,4-dioxotetrahydropyrimidin-1(2H)-yl)pyrazin-2-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(((3-(2,4-dioxotetrahydropyrimidin-1(2H)-yl)pyridin-4-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-((4-((2,6-dioxopiperidin-3-yl)amino)benzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-((3-((2,6-dioxopiperidin-3-yl)amino)benzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-((2-((2,6-dioxopiperidin-3-yl)amino)benzyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(4-(4-(2,6-dioxopiperidin-3-yl)benzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(4-(4-(2,6-dioxopiperidin-3-yl)-3-fluorobenzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(4-(3-(2,6-dioxopiperidin-3-yl)benzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(4-(2-(2,6-dioxopiperidin-3-yl)benzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(4-((5-(2,6-dioxopiperidin-3-yl)pyridin-3-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(4-((5-(2,6-dioxopiperidin-3-yl)-2-fluoropyridin-3-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-(4-(2,4-dioxotetrahydropyrimidin-1(2H)-yl)benzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-(4-(2,4-dioxotetrahydropyrimidin-1(2H)-yl)-2-fluorobenzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-(3-(2,4-dioxotetrahydropyrimidin-1(2H)-yl)benzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-(2-(2,4-dioxotetrahydropyrimidin-1(2H)-yl)benzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-((5-(2,4-dioxotetrahydropyrimidin-1(2H)-yl)pyridin-2-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-((2-(2,4-dioxotetrahydropyrimidin-1(2H)-yl)pyridin-4-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-((2-(2,4-dioxotetrahydropyrimidin-1(2H)-yl)-5-fluoropyridin-4-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-((6-(2,4-dioxotetrahydropyrimidin-1(2H)-yl)pyridin-3-yl)methyl)piperazin-1-yl)piperidin-l-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-((5-(2,4-dioxotetrahydropyrimidin-1(2H)-yl)pyridin-3-yl)methyl)piperazin-1-yl)piperidin-l-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-((5-(2,4-dioxotetrahydropyrimidin-1(2H)-yl)-2-fluoropyridin-3-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-((6-(2,4-dioxotetrahydropyrimidin-1(2H)-yl)pyridazin-3-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-((5-(2,4-dioxotetrahydropyrimidin-1(2H)-yl)pyrazin-2-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(4-(4-((3-(2,4-dioxotetrahydropyrimidin-1(2H)-yl)pyridin-4-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(4-(4-((2,6-dioxopiperidin-3-yl)amino)benzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(4-(3-((2,6-dioxopiperidin-3-yl)amino)benzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(4-(2-((2,6-dioxopiperidin-3-yl)amino)benzyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(((2-(2,4-dioxotetrahydropyrimidin-1(2H)-yl)pyridin-4-yl)methyl)amino)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(((2-(2,4-dioxotetrahydropyrimidin-1(2H)-yl)-5-fluoropyridin-4-yl)methyl)amino)benzyl)-1H-pyrazol-4-yl)-9-methyl-6-oxo-6,7,8,9-tetrahydropyrido[3',2':4,5]pyrrolo[1,2-a]pyrazine-2-carboxamide;
(R)-N-(1-(4-(((5-(2,4-dioxotetrahydropyrimidin-1(2H)-yl)pyridin-3-yl)methyl)amino)benzyl)-1H-pyrazol-4-yl)-9-methyl-6-oxo-6,7,8,9-tetrahydropyrido[3',2':4,5]pyrrolo[1,2-a]pyrazine-2-carboxamide;
3-(4-((4-(3-amino-6-(2-hydroxyphenyl)pyridazin-4-yl)piperazin-1-yl)methyl)phenyl)piperidine-2,6-dione;
3-(4-((4-(3-amino-6-(2-hydroxyphenyl)pyridazin-4-yl)piperazin-1-yl)methyl)-2-fluorophenyl)piperidine-2,6-dione;
3-(3-((4-(3-amino-6-(2-hydroxyphenyl)pyridazin-4-yl)piperazin-1-yl)methyl)phenyl)piperidine-2,6-dione;
3-(2-((4-(3-amino-6-(2-hydroxyphenyl)pyridazin-4-yl)piperazin-1-yl)methyl)phenyl)piperidine-2,6-dione;
1-(4-((4-(3-amino-6-(2-hydroxyphenyl)pyridazin-4-yl)piperazin-1-yl)methyl)phenyl)dihydropyrimidine-2,4(1H,3H)-dione;
1-(4-((4-(3-amino-6-(2-hydroxyphenyl)pyridazin-4-yl)piperazin-1-yl)methyl)-3-fluorophenyl)dihydropyrimidine-2,4(1H,3H)-dione;
1-(3-((4-(3-amino-6-(2-hydroxyphenyl)pyridazin-4-yl)piperazin-1-yl)methyl)phenyl)dihydropyrimidine-2,4(1H,3H)-dione;
1-(2-((4-(3-amino-6-(2-hydroxyphenyl)pyridazin-4-yl)piperazin-1-yl)methyl)phenyl)dihydropyrimidine-2,4(1H,3H)-dione;
1-(6-((4-(3-amino-6-(2-hydroxyphenyl)pyridazin-4-yl)piperazin-1-yl)methyl)pyridin-3-yl)dihydropyrimidine-2,4(1H,3H)-dione;
1-(5-((4-(3-amino-6-(2-hydroxyphenyl)pyridazin-4-yl)piperazin-1-yl)methyl)pyridin-2-yl)dihydropyrimidine-2,4(1H,3H)-dione;
1-(6-((4-(3-amino-6-(2-hydroxyphenyl)pyridazin-4-yl)piperazin-1-yl)methyl)pyridazin-3-yl)dihydropyrimidine-2,4(1H,3H)-dione;
1-(4-((4-(3-amino-6-(2-hydroxyphenyl)pyridazin-4-yl)piperazin-1-yl)methyl)pyridin-3-yl)dihydropyrimidine-2,4(1H,3H)-dione;
3-((4-((4-(3-amino-6-(2-hydroxyphenyl)pyridazin-4-yl)piperazin-1-yl)methyl)phenyl)amino)piperidine-2,6-dione;
3-((3-((4-(3-amino-6-(2-hydroxyphenyl)pyridazin-4-yl)piperazin-1-yl)methyl)phenyl)amino)piperidine-2,6-dione;
3-((2-((4-(3-amino-6-(2-hydroxyphenyl)pyridazin-4-yl)piperazin-1-yl)methyl)phenyl)amino)piperidine-2,6-dione;
3-(4-((4-(3-amino-6-(5-fluoro-2-hydroxyphenyl)pyridazin-4-yl)piperazin-1-yl)methyl)phenyl)piperidine-2,6-dione;
1-(4-((4-(3-amino-6-(5-fluoro-2-hydroxyphenyl)pyridazin-4-yl)piperazin-1-yl)methyl)phenyl)dihydropyrimidine-2,4(1H,3H)-dione;
1-(3-((4-(3-amino-6-(5-fluoro-2-hydroxyphenyl)pyridazin-4-yl)piperazin-1-yl)methyl)phenyl)dihydropyrimidine-2,4(1H,3H)-dione;
3-((3-((4-(3-amino-6-(5-fluoro-2-hydroxyphenyl)pyridazin-4-yl)piperazin-1-yl)methyl)phenyl)amino)piperidine-2,6-dione;
1-(6-((4-(3-amino-6-(5-fluoro-2-hydroxyphenyl)pyridazin-4-yl)piperazin-1-yl)methyl)pyridin-3-yl)dihydropyrimidine-2,4(1H,3H)-dione;
3-(4-((3-(3-amino-6-(5-fluoro-2-hydroxyphenyl)pyridazin-4-yl)-3,8-diazabicyclo[3.2. 1]octan-8-yl)methyl)phenyl)piperidine-2,6-dione;
3-(4-((3-(3-amino-6-(5-fluoro-2-hydroxyphenyl)pyridazin-4-yl)-3,8-diazabicyclo[3.2.1]octan-8-yl)methyl)-2-fluorophenyl)piperidine-2,6-dione;
3-(3-((3-(3-amino-6-(5-fluoro-2-hydroxyphenyl)pyridazin-4-yl)-3,8-diazabicyclo[3.2.1]octan-8-yl)methyl)phenyl)piperidine-2,6-dione;
3-(2-((3-(3-amino-6-(5-fluoro-2-hydroxyphenyl)pyridazin-4-yl)-3,8-diazabicyclo[3.2. 1]octan-8-yl)methyl)phenyl)piperidine-2,6-dione;
1-(4-((3-(3-amino-6-(5-fluoro-2-hydroxyphenyl)pyridazin-4-yl)-3,8-diazabicyclo[3.2. 1]octan-8-yl)methyl)phenyl)dihydropyrimidine-2,4(1H,3H)-dione;
1-(4-((3-(3-amino-6-(5-fluoro-2-hydroxyphenyl)pyridazin-4-yl)-3,8-diazabicyclo[3.2.1]octan-8-yl)methyl)-3-fluorophenyl)dihydropyrimidine-2,4(1H,3H)-dione;
1-(3-((3-(3-amino-6-(5-fluoro-2-hydroxyphenyl)pyridazin-4-yl)-3,8-diazabicyclo[3.2.1]octan-8-yl)methyl)phenyl)dihydropyrimidine-2,4(1H,3H)-dione;
1-(2-((3-(3-amino-6-(5-fluoro-2-hydroxyphenyl)pyridazin-4-yl)-3,8-diazabicyclo[3.2.1]octan-8-yl)methyl)phenyl)dihydropyrimidine-2,4(1H,3H)-dione;
1-(6-((3-(3-amino-6-(5-fluoro-2-hydroxyphenyl)pyridazin-4-yl)-3,8-diazabicyclo[3.2.1]octan-8-yl)methyl)pyridin-3-yl)dihydropyrimidine-2,4(1H,3H)-dione;
1-(5-((4-(3-amino-6-(5-fluoro-2-hydroxyphenyl)pyridazin-4-yl)piperazin-1-yl)methyl)pyridin-2-yl)dihydropyrimidine-2,4(1H,3H)-dione;
1-(6-((3-(3-amino-6-(5-fluoro-2-hydroxyphenyl)pyridazin-4-yl)-3,8-diazabicyclo[3.2.1]octan-8-yl)methyl)pyridazin-3-yl)dihydropyrimidine-2,4(1H,3H)-dione;
1-(4-((3-(3-amino-6-(5-fluoro-2-hydroxyphenyl)pyridazin-4-yl)-3,8-diazabicyclo[3.2.1]octan-8-yl)methyl)pyridin-3-yl)dihydropyrimidine-2,4(1H,3H)-dione;
3-((4-((3-(3-amino-6-(5-fluoro-2-hydroxyphenyl)pyridazin-4-yl)-3,8-diazabicyclo[3.2.1]octan-8-yl)methyl)phenyl)amino)piperidine-2,6-dione;
3-((3-((3-(3-amino-6-(5-fluoro-2-hydroxyphenyl)pyridazin-4-yl)-3,8-diazabicyclo[3.2.1]octan-8-yl)methyl)phenyl)amino)piperidine-2,6-dione;
3-((2-((3-(3-amino-6-(5-fluoro-2-hydroxyphenyl)pyridazin-4-yl)-3,8-diazabicyclo[3.2.1]octan-8-yl)methyl)phenyl)amino)piperidine-2,6-dione;
3-(4-((4-(2-(3-(3-amino-6-(2-hydroxyphenyl)pyridazin-4-yl)-3,8-diazabicyclo[3.2. 1]octan-8-yl)pyrimidin-5-yl)piperidin-1-yl)methyl)phenyl)piperidine-2,6-dione;
3-(4-((4-(2-(3-(3-amino-6-(2-hydroxyphenyl)pyridazin-4-yl)-3,8-diazabicyclo[3.2.1]octan-8-yl)pyrimidin-5-yl)piperidin-1-yl)methyl)-2-fluorophenyl)piperidine-2,6-dione;
3-(3-((4-(2-(3-(3-amino-6-(2-hydroxyphenyl)pyridazin-4-yl)-3,8-diazabicyclo[3.2. 1]octan-8-yl)pyrimidin-5-yl)piperidin-1-yl)methyl)phenyl)piperidine-2,6-dione;
3-(2-((4-(2-(3-(3-amino-6-(2-hydroxyphenyl)pyridazin-4-yl)-3,8-diazabicyclo[3.2. 1]octan-8-yl)pyrimidin-5-yl)piperidin-1-yl)methyl)phenyl)piperidine-2,6-dione;
1-(4-((4-(2-(3-(3-amino-6-(2-hydroxyphenyl)pyridazin-4-yl)-3,8-diazabicyclo[3.2.1]octan-8-yl)pyrimidin-5-yl)piperidin-1-yl)methyl)phenyl)dihydropyrimidine-2,4(1H,3H)-dione;
1-(4-((4-(2-(3-(3-amino-6-(2-hydroxyphenyl)pyridazin-4-yl)-3,8-diazabicyclo[3.2.1]octan-8-yl)pyrimidin-5-yl)piperidin-1-yl)methyl)-3-fluorophenyl)dihydropyrimidine-2,4(1H,3H)-dione;
1-(3-((4-(2-(3-(3-amino-6-(2-hydroxyphenyl)pyridazin-4-yl)-3,8-diazabicyclo[3.2.1]octan-8-yl)pyrimidin-5-yl)piperidin-1-yl)methyl)phenyl)dihydropyrimidine-2,4(1H,3H)-dione;
1-(2-((4-(2-(3-(3-amino-6-(2-hydroxyphenyl)pyridazin-4-yl)-3,8-diazabicyclo[3.2.1]octan-8-yl)pyrimidin-5-yl)piperidin-1-yl)methyl)phenyl)dihydropyrimidine-2,4(1H,3H)-dione;
1-(6-((4-(2-(3-(3-amino-6-(2-hydroxyphenyl)pyridazin-4-yl)-3,8-diazabicyclo[3.2.1]octan-8-yl)pyrimidin-5-yl)piperidin-1-yl)methyl)pyridin-3-yl)dihydropyrimidine-2,4(1H,3H)-dione;
1-(6-((4-(2-(3-(3-amino-6-(2-hydroxyphenyl)pyridazin-4-yl)-3,8-diazabicyclo[3.2.1]octan-8-yl)pyrimidin-5-yl)piperidin-1-yl)methyl)pyridazin-3-yl)dihydropyrimidine-2,4(1H,3H)-dione;
1-(4-((4-(2-(3-(3-amino-6-(2-hydroxyphenyl)pyridazin-4-yl)-3,8-diazabicyclo[3.2.1]octan-8-yl)pyrimidin-5-yl)piperidin-1-yl)methyl)pyridin-3-yl)dihydropyrimidine-2,4(1H,3H)-dione;
3-((2-((4-(2-(3-(3-amino-6-(2-hydroxyphenyl)pyridazin-4-yl)-3,8-diazabicyclo[3.2.1]octan-8-yl)pyrimidin-5-yl)piperidin-1-yl)methyl)phenyl)amino)piperidine-2,6-dione;
3-((3-((4-(2-(3-(3-amino-6-(2-hydroxyphenyl)pyridazin-4-yl)-3,8-diazabicyclo[3.2.1]octan-8-yl)pyrimidin-5-yl)piperidin-1-yl)methyl)phenyl)amino)piperidine-2,6-dione;
3-((2-((4-(2-(3-(3-amino-6-(2-hydroxyphenyl)pyridazin-4-yl)-3,8-diazabicyclo[3.2.1]octan-8-yl)pyrimidin-5-yl)piperidin-1-yl)methyl)phenyl)amino)piperidine-2,6-dione;
3-(4-((4-((4-((5-amino-1-(3-methylthiophene-2-carbonyl)-1 H-1,2,4-triazol-3-yl)amino)phenyl)sulfonyl)piperazin-1-yl)methyl)phenyl)piperidine-2,6-dione;
3-(4-((4-((4-((5-amino-1-(3-methylthiophene-2-carbonyl)-1 H-1,2,4-triazol-3-yl)amino)phenyl)sulfonyl)piperazin-1-ylymethyl)-2-fluorophenyl)piperidine-2,6-dione;
1-(4-((4-((4-((5-amino-1-(3-methylthiophene-2-carbonyl)-1H-1,2,4-triazol-3-yl)amino)phenyl)sulfonyl)piperazin-1-yl)methyl)phenyl)dihydropyrimidine-2,4(1H,3H)-dione;
1-(4-((4-((4-((5-amino-1-(3-methylthiophene-2-carbonyl)-1H-1,2,4-triazol-3-yl)amino)phenyl)sulfonyl)piperazin-1-yl)methyl)-2-fluorophenyl)dihydropyrimidine-2,4(1H,3H)-dione;
1-(4-((4-((4-((5-amino-1-(3-methylthiophene-2-carbonyl)-1H-1,2,4-triazol-3-yl)amino)phenyl)sulfonyl)piperazin-1-yl)methyl)-3-fluorophenyl)dihydropyrimidine-2,4(1H,3H)-dione;
1-(3-((4-((4-((5-amino-1-(3-methylthiophene-2-carbonyl)-1H-1, 2,4-triazol-3-yl)amino)phenyl)sulfonyl)piperazin-1-yl)methyl)phenyl)dihydropyrimidine-2,4(1H,3H)-dione;
1-(5-((4-((4-((5-amino-1-(3-methylthiophene-2-carbonyl)-1H-1,2,4-triazol-3-yl)amino)phenyl)sulfonyl)piperazin-1-yl)methyl)pyridin-2-yl)dihydropyrimidine-2,4(1H,3H)-dione;
1-(6-((4-((4-((5-amino-1-(3-methylthiophene-2-carbonyl)-1H-1,2,4-triazol-3-yl)amino)phenyl)sulfonyl)piperazin-1-yl)methyl)pyridin-3-yl)dihydropyrimidine-2,4(1H,3H)-dione;
3-((3-((4-((4-((5-amino-1-(3-methylthiophene-2-carbonyl)-1H-1,2,4-triazol-3-yl)amino)phenyl)sulfonyl)piperazin-1-yl)methyl)phenyl)amino)piperidine-2,6-dione;
3-(4-((4-((4-((5-amino-1-(2,6-difluorobenzoyl)-1H-1,2,4-triazol-3-yl)amino)phenyl)sulfonyl)piperazin-1-yl)methyl)phenyl)piperidine-2,6-dione;
1-(4-((4-((4-((5-amino-1-(2,6-difluorobenzoyl)-1H-1,2,4-triazol-3-yl)amino)phenyl)sulfonyl)piperazin-1-yl)methyl)phenyl)dihydropyrimidine-2,4(1H,3H)-dione;
1-(3-((4-((4-((5-amino-1-(2,6-difluorobenzoyl)-1H-1,2,4-triazol-3-yl)amino)phenyl)sulfonyl)piperazin-1-yl)methyl)phenyl)dihydropyrimidine-2,4(1H,3H)-dione;
1-(5-((4-((4-((5-amino-1-(2,6-difluorobenzoyl)-1H-1,2,4-triazol-3-yl)amino)phenyl)sulfonyl)piperazin-1-yl)methyl)pyridin-2-yl)dihydropyrimidine-2,4(1H,3H)-dione;
1-(6-((4-((4-((5-amino-1-(2,6-difluorobenzoyl)-1H-1,2,4-triazol-3-yl)amino)phenyl)sulfonyl)piperazin-1-yl)methyl)pyridin-3-yl)dihydropyrimidine-2,4(1H,3H)-dione;
3-((3-((4-((4-((5-amino-1-(2,6-difluorobenzoyl)-1H-1,2,4-triazol-3-yl)amino)phenyl)sulfonyl)piperazin-1-yl)methyl)phenyl)amino)piperidine-2,6-dione;
2-((5-bromo-2-((4-((4-(4-(2,6-dioxopiperidin-3-yl)benzyl)piperazin-1-yl)sulfonyl)-2-methylphenyl)amino)pyrimidin-4-yl)amino)-6-fluorobenzamide;
2-((5-bromo-2-((4-((4-(4-(2,4-dioxotetrahydropyrimidin-1(2H)-yl)benzyl)piperazin-1-yl)sulfonyl)-2-methylphenyl)amino)pyrimidin-4-yl)amino)-6-fluorobenzamide;
2-((5-bromo-2-((4-((4-(3-(2,4-dioxotetrahydropyrimidin-1(2H)-yl)benzyl)piperazin-1-yl)sulfonyl)-2-methylphenyl)amino)pyrimidin-4-yl)amino)-6-fluorobenzamide;
2-((5-bromo-2-((4-((4-((6-(2,4-dioxotetrahydropyrimidin-1(2H)-yl)pyridin-3-yl)methyl)piperazin-1-yl)sulfonyl)-2-methylphenyl)amino)pyrimidin-4-yl)amino)-6-fluorobenzamide;
2-((5-bromo-2-((4-((4-((5-(2,4-dioxotetrahydropyrimidin-1(2H)-yl)pyridin-2-yl)methyl)piperazin-1-yl)sulfonyl)-2-methylphenyl)amino)pyrimidin-4-yl)amino)-6-fluorobenzamide; and
2-((5-bromo-2-((4-((4-(3-((2,6-dioxopiperidin-3-yl)amino)benzyl)piperazin-1-yl)sulfonyl)-2-methylphenyl)amino)pyrimidin-4-yl)amino)-6-fluorobenzamide.

17. The compound of Formula (I) or salts, enantiomers, stereoisomers, solvates, isotopically enriched analogs, prodrugs, or polymorphs thereof as claimed in any one of claims 1-16, which is hydrohalides (including hydrochlorides and hydrobromates), sulfates, citrates, maleates, methanesulfonates, citrates, lactates, L-tartrates, fumarates, L-malates, phosphates, dihydrophosphates, pyrophosphates, metaphosphates, oxalates, malonates, benzoates, mandelates, succinates, trifluoroacetates, methanesulfonates, hippurates, hydroxyacetates, or p-toluenesulfonates of the compound of Formula (I).

18. A compound of Formula (II) 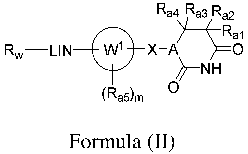 or salts, enantiomers, stereoisomers, solvates, isotopically enriched analogs, prodrugs, or polymorphs thereof,
wherein A represents N or CH;
Rₐ₁, Rₐ₂, Rₐ₃, and Rₐ₄ are the same or different and independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
ring W¹ represents heteroarylene, and the ring W¹ is optionally substituted with m Rₐ₅, with each Rₐ₅ being the same or different and independently representing halogen, hydroxy, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl or C₂₋₆ alkynyl;
m represents an integer of 0, 1, 2, 3, or 4;
X represents a bond or -R_{b1}-N(Rₐ₆)-R_{b2}-, where Rₐ₆ represents hydrogen or C₁₋₆ alkyl, and R_{b1} and R_{b2} each independently represent optionally substituted C₀₋₂ alkylene;
LIN represents optionally substituted methylene, or optionally substituted linear or branched C₂₋₁₅ alkylene, wherein one or more groups selected from Rₐ, R_{b}, and any combination thereof are optionally inserted between one or more pairs of adjacent carbon atoms in the backbone carbon chain of the linear or branched C₂₋₁₅ alkylene group; wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and in case that two or more groups Rₐ are inserted into the backbone carbon chain of the linear or branched C₂-₃₀ alkylene group, the two or more groups Rₐ are not directly connected to each other; and wherein each R_{b} is independently selected from the group consisting of optionally substituted cycloalkylene, optionally substituted arylene, optionally substituted heterocyclylene, optionally substituted heteroarylene, alkynylene, alkenylene, or any combination thereof; and
R_{w} represents hydrogen, deuterium, leaving group, hydroxy, halogen, COOH, NH₂, N₃, CHO, methanesulfonyloxy, trifluoromethanesulfonyloxy or p-toluenesulfonyloxy.

19. The compound of Formula (II) or salts, enantiomers, stereoisomers, solvates, isotopically enriched analogs, prodrugs, or polymorphs thereof as claimed in claim 18, wherein the compound of Formula (I) is also represented by Formula (II-1) or Formula (II-2): 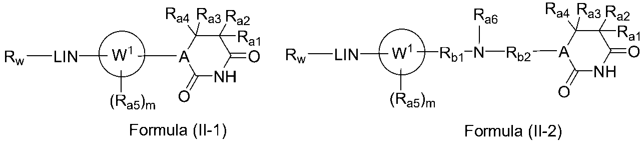 wherein A, Rₐ₁, Rₐ₂, Rₐ₃, Rₐ₄, (Rₐ₅)ₘ, Rₐ₆, R_{b1}, R_{b2}, and ring W¹ are as defined in claim 18.

20. The compound of Formula (II) or salts, enantiomers, stereoisomers, solvates, isotopically enriched analogs, prodrugs, or polymorphs thereof as claimed in claim 18 or 19, wherein
(i) the ring W¹ represents: 5- to 20-membered monocyclic, bicyclic, or polycyclic heteroarylene, wherein the 5- to 20-membered monocyclic, bicyclic, or polycyclic heteroarylene is optionally substituted with m identical or different Rₐ₅; preferably wherein the ring W¹ represents the following groups which are optionally substituted with m identical or different Rₐ₅: furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene;
wherein each Rₐ₅ independently represents halogen, hydroxy, mercapto, nitro, amino, cyano,C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl or C₂₋₆ alkynyl; and m represents an integer of 0, 1, 2, 3, or 4;
and/or
(ii) X in the structure of Formula (II) represents a bond or -R_{b1}-N(Rₐ₆)-R_{b2}-, where Rₐ₆ represents hydrogen or C₁₋₆ alkyl, and R_{b1} and R_{b2} each independently represent optionally substituted C₀₋₂ alkylene, preferably wherein the substituents of said optionally substituted C₀₋₂ alkylene are optionally selected from deuterium, halogen, hydroxy, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy or any combination thereof.

21. The compound of Formula (II) or salts, enantiomers, stereoisomers, solvates, isotopically enriched analogs, prodrugs, or polymorphs thereof as claimed in claim 18 or 19, wherein the LIN represents:
methylene, which is optionally substituted with 1 to 2 substituents independently selected from deuterium, hydroxy, amino, mercapto, nitro, halogen, cyano, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl or any combination thereof; or
optionally substituted linear or branched C₂₋₁₅ alkylene, wherein one or more groups selected from Rₐ, R_{b}, and any combination thereof are optionally inserted between any one or more pairs of adjacent carbon atoms in the backbone carbon chain of the linear or branched C₂₋₁₅ alkylene group; wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and in case that two or more groups Rₐ are inserted into the backbone carbon chain of the linear or branched C₂₋₁₅ alkylene group, the two or more groups Rₐ are not directly connected to each other; and wherein each R_{b} is independently selected from the group consisting of optionally substituted cycloalkylene (e.g., optionally substituted C₃₋₂₀ cycloalkylene), optionally substituted arylene (e.g., optionally substituted C₃₋₂₀ arylene), optionally substituted heterocyclylene (e.g., optionally substituted 4- to 20-membered heterocyclylene), optionally substituted heteroarylene (e.g., optionally substituted 5- to 20-membered heteroarylene), alkynylene (e.g., C₂₋₆ alkynylene), alkenylene (e.g., C₂₋₆ alkenylene), and any combination thereof, wherein the cycloalkylene, arylene, heterocyclylene, and heteroarylene are independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof; and wherein one or more hydrogens of said linear or branched C₂₋₁₅ alkylene are optionally further replaced by a substituent selected from deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4-to 10-membered heterocyclyl and any combination thereof.

22. The compound of Formula (II) or salts, enantiomers, stereoisomers, solvates, isotopically enriched analogs, prodrugs, or polymorphs thereof as claimed in claim 18 or 19, wherein the LIN represents:
-C₁₋₁₅ alkylene-;
-(C(Rₐ₈)(Rₐ₉))ₙ₁-(Rₐ(C(Rₐ₁₀)(Rₐ₁₁))ₙ₂)ₘ₁-;
-(C(Rₐ₈)(Rₐ₉))ₙ₁-(Rₐ(C(Rₐ₁₀)(Rₐ₁₁))ₙ₂)ₘ₁-(Rₐ(C(Rₐ₁₂)(Rₐ₁₃))ₙ₃)ₘ₂-;
-(C(Rₐ₈)(Rₐ₉))ₙ₁-(Rₐ(C(Rₐ₁₀)(Rₐ₁₁))ₙ₂)ₘ₁-(Rₐ(C(Rₐ₁₂)(Rₐ₁₃))ₙ₃)ₘ₂-(Rₐ(C(Rₐ₁₄ )(Rₐ₁₅))ₙ₄)ₘ₃-;
-(C(Rₐ₈)(Rₐ₉))ₙ₁-(R_{b}(C(Rₐ₁₀)(Rₐ₁₁))ₙ₂)ₘ₁-;
-(C(Rₐ₈)(Rₐ₉))ₙ₁-(R_{b}( C(Rₐ₁₀)(Rₐ₁₁))ₙ₂)ₘ₁-(R_{b}( C(Rₐ₁₂)(Rₐ₁₃))ₙ₃ )ₘ₂-;
-(C(Rₐ₈)(Rₐ₉))ₙ₁-(R_{b}(C(Rₐ₁₀)(Rₐ₁₁))ₙ₂)ₘ₁-(R_{b}(C(Rₐ₁₂)(Rₐ₁₃))ₙ₃)ₘ₂-(R_{b}(C(Rₐ₁₄)(Rₐ₁₅))ₙ₄)ₘ₃-;
-(C(Rₐ₈)(Rₐ₉))ₙ₁-(Rₐ- R_{b}-(C(Rₐ₁₀)(Rₐ₁₁))ₙ₂)ₘ₁-;
-(C(Rₐ₈)(Rₐ₉))ₙ₁-(Rₐ(C(Rₐ₁₀)(Rₐ₁₁))ₙ₂)ₘ₁-(R_{b}(C(Rₐ₁₂)(Rₐ₁₃))ₙ₃)ₘ₂-;
-(C(Rₐ₈)(Rₐ₉))ₙ₁-(R_{b}-Rₐ-(C(Rₐ₁₀)(Rₐ₁₁))ₙ₂)ₘ₁-;
or
-(C(Rₐ₈)(Rₐ₉))ₙ₁-(R_{b}( C(Rₐ₁₀)(Rₐ₁₁))ₙ₂)ₘ₁-(Rₐ(C(Rₐ₁₂)(Rₐ₁₃))ₙ₃)ₘ₂-;
wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇) and N(Rₐ₇)C(O)N(Rₐ₇), wherein each Rₐ₇ independently represents H or C₁₋₃ alkyl; and each R_{b} is independently selected from the group consisting of optionally substituted cycloalkylene (e.g., optionally substituted C₃₋₂₀ cycloalkylene), optionally substituted arylene (e.g., optionally substituted C₃₋₂₀ arylene), optionally substituted heterocyclylene (e.g., optionally substituted 4- to 20-membered heterocyclylene), optionally substituted heteroarylene (e.g., 5- to 20-membered heteroarylene), alkynylene (e.g., C₂₋₆ alkynylene), alkenylene (e.g., C₂₋₆ alkenylene) and any combination thereof, wherein the cycloalkylene, arylene, heterocyclylene, and heteroarylene are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof;
Rₐ₈, Rₐ₉, Rₐ₁₀, Rₐ₁₁, Rₐ₁₂, Rₐ₁₃, Rₐ₁₄, and Rₐ₁₅ each independently represent H, deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof; and
n1, n2, n3, n4, m1, m2, and m3 each independently represent an integer of 1, 2, 3, 4, 5, 6, 7, 8, 9 or 10; and
any one or more hydrogens of said C₁₋₁₅ alkylene are optionally further replaced by a substituent selected from: deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof.

23. The compound of Formula (I) or salts, enantiomers, stereoisomers, solvates, isotopically enriched analogs, prodrugs, or polymorphs thereof as claimed in claim 18 or 19, wherein the LIN represents a structure of the following formulae:
-(C(Rₐ₈)(Rₐ₉))ₙ₁-(O(C(Rₐ₁₀)(Rₐ₁₁))ₙ₂)ₘ₁-;
-(C(Rₐ₈)(Rₐ₉))ₙ₁-(O(C(Rₐ₁₀)(Rₐ₁₁))ₙ₂)ₘ₁-(O(C(Rₐ₁₂)(Rₐ₁₃))ₙ₃)ₘ₂-;
-(C(Rₐ₈)(Rₐ₉))ₙ₁-(O(C(Rₐ₁₀)(Rₐ₁₁))ₙ₂)ₘ₁-(O(C(Rₐ₁₂)(Rₐ₁₃))ₙ₃)ₘ₂- (O(C(Rₐ₁₄)(Rₐ₁₅))ₙ₄)ₘ₃-;
-(C(Rₐ₈)(Rₐ₉))ₙ₁-(N(Rₐ₇)(C(Rₐ₁₀)(Rₐ₁₁))ₙ₂)ₘ₁-;
-(C(Rₐ₈)(Rₐ₉))ₙ₁-(N(Rₐ₇)(C(Rₐ₁₀)(Rₐ₁₁))ₙ₂)ₘ₁-(N(Rₐ₇ )(C(Rₐ₁₂)(Rₐ₁₃))ₙ₃)ₘ₂-;
-(C(Rₐ₈)(Rₐ₉))ₙ₁-(N (Rₐ₇)(C(Rₐ₁₀)(Rₐ₁₁))ₙ₂)ₘ₁-(N(Rₐ₇)(C(Rₐ₁₂)(Rₐ₁₃))ₙ₃)ₘ₂-(N(Rₐ₇)(C(Rₐ₁₄)(Rₐ₁₅))ₙ₄)ₘ₃-;
-(C(Rₐ₈)(Rₐ₉))ₙ₁-(C(O)N(Rₐ₇)-(C(Rₐ₁₀))(Rₐ₁₁))ₙ₂)ₘ₁-;
-(C(Rₐ₈)(Rₐ₉))ₙ₁-(C(O)N(Rₐ₇)-(C(Rₐ₁₀)(Rₐ₁₁))ₙ₂)ₘ₁-(C(O)N(Rₐ₇)-(C(Rₐ₁₂)(Rₐ₁₃))ₙ₃)ₘ₂-;
-(C(Rₐ₈)(Rₐ₉))ₙ₁₁-(C(O)N(Rₐ₇)-(C(Rₐ₁₀)(Rₐ₁₁))ₙ₂)ₘ₁-(C(O)N(Rₐ₇)-(C(Rₐ₁₂)(Rₐ₁₃))ₙ₃)ₘ₂-(C(O)N(Rₐ₇)-(C(Rₐ₁₄)(Rₐ₁₅))ₙ₄)ₘ₃-;
-(C(Rₐ₈)(Rₐ₉))ₙ₁-(C(O)N(Rₐ₇)-(C(Rₐ₁₀)(Rₐ₁₁))ₙ₂)ₘ₁-(O-(C(Rₐ₁₂)(Rₐ₁₃))ₙ₃)ₘ₂-;
-(C(Rₐ₈)(Rₐ₉))ₙ₁-C(O)N(Rₐ₇)-(C(Rₐ₁₀))(Rₐ₁₁))ₙ₂-(O(C(Rₐ₁₂)(Rₐ₁₃))ₙ₃)ₘ₁-;
-(C(Rₐ₈)(Rₐ₉))ₙ₁₁-(N(Rₐ₇)C(O)-(C(Rₐ₁₀)(Rₐ₁₁))ₙ₂)ₘ₁-;
-(C(Rₐ₈)(Rₐ₉))ₙ₁-(N(Rₐ₇)C(O)-(C(Rₐ₁₀)(Rₐ₁₁))ₙ₂)ₘ₁-(O-(C(Rₐ₁₂)(Rₐ₁₃)))ₙ₃)ₘ₂-;
-(C(Rₐ₈)(Rₐ₉))ₙ₁-(N(Rₐ₇)C(O)-(C(Rₐ₁₀)(Rₐ₁₁))ₙ₂)ₘ₁-(O-(C(Rₐ₁₂)(Rₐ₁₃))ₙ₃)ₘ₂-(O-(O(Rₐ₁₄)(Rₐ₁₅))ₙ₄)ₘ₃-;
-(C(Rₐ₈)(Rₐ₉))ₙ₁-N(Rₐ₇)C(O)-(C(Rₐ₁₀)(Rₐ₁₁))ₙ₂-(O(C(Rₐ₁₂)(Rₐ₁₃))ₙ₃)ₘ₁-;
-(C(Rₐ₈)(Rₐ₉))ₙ₁-N(Rₐ₇)C(O)N(Rₐ₇)-(C(Rₐ₁₀))(Rₐ₁₁))ₙ₂-;
-(C(Rₐ₈)(Rₐ₉))ₙ₁-C(O)-(C(Rₐ₁₀)(Rₐ₁₁))ₙ₂-;
-(C(Rₐ₈)(Rₐ₉))ₙ₁-(arylene-(C(Rₐ₁₀)(Rₐ₁₁))ₙ₂)ₘ₁-;
-(C(Rₐ₈)(Rₐ₉))ₙ₁-(arylene-(C(Ra₁₀)(Rₐ₁₁))ₙ₂)ₘ₁-arylene-(C(Rₐ₁₂)(Ra₁₃))ₙ₃-;
-(C(Rₐ₈)(Rₐ₉))ₙ₁-(heterocyclylene-(C(Rₐ₁₀)(Rₐ₁₁))ₙ₂)ₘ₁-;
-(C(Rₐ₈)(Rₐ₉))ₙ₁-(heterocyclylene-(C(Rₐ₁₀)(Rₐ₁₁))ₙ₂)ₘ₁-(heterocyclylene-(C(Rₐ₁₂)(Rₐ₁₃))ₙ₃)ₘ₂-;
-(C(Rₐ₈(Rₐ₉))ₙ₁(heteroarylene-(C(Rₐ₁₀)(Rₐ₁₁))ₙ₂)ₘ₁-;
-(C(Rₐ₈)(Rₐ₉))ₙ₁-(heteroarylene-(C(Rₐ₁₀)(Rₐ₁₁))ₙ₂)ₘ₁-(heteroarylene-(C(Rₐ₁₂)(Rₐ₁₃))ₙ₃)ₘ₂-;
-(C(Rₐ₈)(Rₐ₉))ₙ₁-(cycloalkylene-(C(Rₐ₁₀)(Rₐ₁₁))ₙ₂)ₘ₁-;
or
-(C(Rₐ₈)(Rₐ₉))ₙ₁-(cycloalkylene-(C(Rₐ₁₀)(Rₐ₁₁))ₙ₂)ₘ₁-(cycloalky lene-(C(Rₐ₁₂)(Rₐ₁₃))ₙ₃)ₘ₂-;
wherein each Rₐ₇ independently represents H or C₁₋₃ alkyl;
the cycloalkylene (e.g., C₃₋₂₀ cycloalkylene), arylene (e.g., C₃₋₂₀ arylene), heterocyclylene (e.g., 4- to 20-membered heterocyclylene), and the heteroarylene (e.g., 5- to 20-membered heteroarylene) are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof;
Rₐ₈, Rₐ₉, Rₐ₁₀, Rₐ₁₁, Rₐ₁₂, Rₐ₁₃, Rₐ₁₄, and Rₐ₁₅ each independently represent H, deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof; and
n1, n2, n3, n4, m1, m2, and m3 each independently represent an integer of 1, 2, 3, 4, 5, 6, 7, 8, 9 or 10.

24. The compound of Formula (II) or salts, enantiomers, stereoisomers, solvates, isotopically enriched analogs, prodrugs, or polymorphs thereof as claimed in any one of claims 21-23, wherein LIN represents the following groups:
-CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, -(CH₂)₁₃-, -(CH₂)₁₄-, -(CH₂)₁₅-; -CH₂-O-CH₂-, -CH₂-O-(CH₂)₂-, -(CH₂),-O-(CH₂)₃-, -(CH₂)₁-O-(CH₂)₄-, -(CH₂)₁-O-(CH₂)₅-, -(CH₂)₁-O-(CH₂)₆-, -(CH₂)₁-O-(CH₂)₇-, -(CH₂)₁-O-(CH₂)₈-, -(CH₂)₁-O-(CH₂)₉-, - (CH₂)₁-O-(CH₂)₁₀-, -(CH₂)₂-O-(CH₂)₁-, -(CH₂)₂-O-(CH₂)₂-, -(CH₂)₂-O-(CH₂)₃-, -(CH₂)₂-O-(CH₂)₄-, -(CH₂)₂-O-(CH₂)₅-, -(CH₂)₂-O-(CH₂)₆-, -(CH₂)₂-O-(CH₂)₇-, -(CH₂)₂-O-(CH₂)₈-, -(CH₂)₂-O-(CH₂)₉-, -(CH₂)₂-O-(CH₂)₁₀-, -(CH₂)₃-O-(CH₂)₁-, -(CH₂)₃-O-(CH₂)₂-, -(CH₂)₃-O-(CH₂)₃-, -(CH₂)₃-O-(CH₂)₄-, -(CH₂)₃-O-(CH₂)₅-, -(CH₂)₃-O-(CH₂)₆-, -(CH₂)₃-O-(CH₂)₇-, -(CH₂)₄-O-(CH₂)₁-, -(CH₂)₄-O-(CH₂)₂-, -(CH₂)₄-O-(CH₂)₃-, -(CH₂)₄-O-(CH₂)₄-, -(CH₂)₄-O-(CH₂)₅-, -(CH₂)₄-O-(CH₂)₆-, -(CH₂)₅-O-(CH₂)₁-, -(CH₂)₅-O-(CH₂)₂-, -(CH₂)₅-O-(CH₂)₃-, -(CH₂)₅-O-(CH₂)₄-, -(CH₂)₅-O-(CH₂)₅-, -(CH₂)₆-O-(CH₂)₁-, -(CH₂)₆-O-(CH₂)₂-, -(CH₂)₆-O-(CH₂)₃-, -(CH₂)₆-O-(CH₂)₄-, -(CH₂)₇-O-(CH₂)₁-, -(CH₂)₇-O-(CH₂)₂-, -(CH₂)₇-O-(CH₂)₃-, -(CH₂)₈-O-(CH₂)₁-, -(CH₂)₈-O-(CH₂)₂-, -CH(CH₃)-O-(CH₂)₁-, -CH(CH₃)-O-(CH₂)₂-, -CH(CH₃)-O-(CH₂)₃-, -CH(CH₃)-O-(CH₂)₄-, -CH(CH₃)-O-(CH₂)₅-, -CH(CH₃)-O-(CH₂)₆-, -CH(CH₃)-O-(CH₂)₇-, -CH(CH₃)-O-(CH₂)₈-, -CH(CH₃)-O-(CH₂)₉-, - CH(CH₃)-O-(CH₂)₁₀-, -CH₂-(O(CH₂)₂)₂-, -CH₂-(O(CH₂)₂)₃-, -CH₂-(O(CH₂)₂)₄-, -CH₂-(O(CH₂)₂)₅-, -CH₂-(O(CH₂)₂)₆-, -CH₂-(O(CH₂)₂)₇-, -CH₂-(O(CH₂)₂)₁-OCH₂-, -CH₂-(O(CH₂)₂)₂-OCH₂-, -CH₂-(O(CH₂)₂)₃-OCH₂-, -CH₂-(O(CH₂)₂)₄-OCH₂-, -CH₂-(O(CH₂)₂)₅-OCH₂-, -CH₂-(O(CH₂)₂)₆-OCH₂-, -CH₂-(O(CH₂)₂)₇-OCH₂-, - (CH₂)₂-(O(CH₂)₂)₂-, -(CH₂)₂-(O(CH₂)₂)₃-, -(CH₂)₂-(O(CH₂)₂)₄-, -(CH₂)₂-(O(CH₂)₂)₅-, -(CH₂)₂-(O(CH₂)₂)₆-, - (CH₂)₂-(O(CH₂)₂)₇-, -(CH₂)₃-(O(CH₂)₂)₂-, -(CH₂)₃-(O(CH₂)₂)₃-, -(CH₂)₃-(O(CH₂)₂)₄-, -(CH₂)₃-(O(CH₂)₂)₅-, - (CH₂)₃-(O(CH₂)₂)₆-, -(CH₂)₃-(O(CH₂)₂)₇-, -(CH₂)₄-(O(CH₂)₂)₂-, -(CH₂)₄-(O(CH₂)₂)₃-, -(CH₂)₄-(O(CH₂)₂)₄-, - (CH₂)₄-(O(CH₂)₂)₅-, -(CH₂)₄-(O(CH₂)₂)₆-, -(CH₂)₄-(O(CH₂)₂)₇-, -CH₂-(O(CH₂)₃)₂-, -CH₂-(O(CH₂)₃)₃-, -CH₂-(O(CH₂)₃)₄-, -(CH₂)₂-(O(CH₂)₃)₂-, -(CH₂)₂-(O(CH₂)₃)₃-, -(CH₂)₂-(O(CH₂)₃)₄-, -(CH₂)₃-(O(CH₂)₃)₂-, -(CH₂)₃-(O(CH₂)₃)₃-, -(CH₂)₃-(O(CH₂)₃)₄-, -CH₂-O-(CH₂)₂-O-(CH₂)₃-, -CH₂-(O(CH₂)₂)₂-(O(CH₂)₃)₂-, -CH₂-(O(CH₂)₂)₃-(O(CH₂)₃)₃-, -CH₂-(O(CH₂)₂)₄-(O(CH₂)₃)₄-, (CH₂)₂-O-(CH₂)₂-O-(CH₂)₃-, -(CH₂)₂-(O(CH₂)₂)₂-(O(CH₂)₃)₂-, -(CH₂)₃-O-(CH₂)₂-O-(CH₂)₃-, -(CH₂)₃-(O(CH₂)₂)₂-(O(CH₂)₃)₂-, -CH\₂-O-(CH₂)₃-O-(CH₂)₂-, - CH₂-(O(CH₂)₃)₂-(O(CH₂)₂)₂-, -(CH₂)₂-O-(CH₂)₃-O-(CH₂)₂-, -(CH₂)₂-(O(CH₂)₃)₂-(O(CH₂)₂)₂-, -(CH₂)₃-O-(CH₂)₃-O-(CH₂)₂-, -(CH₂)₃-(O(CH₂)₃)₂-(O(CH₂)₂)₂-, -CH₂-O-(CH₂)₂-O-CH₂-, -(CH₂)₂-O-(CH₂)₂-O-CH₂-, - (CH₂)₂-(O(CH₂)₂)₂-O-(CH₂)₃-, -(CH₂)₂-(O(CH₂)₂)₃-O-(CH₂)₃-, -(CH₂)₂-(O(CH₂)₂)₄-O-(CH₂)₃-, -(CH₂)₅-(O(CH₂)₂)₂-O-(CH₂)₅-, -(CH₂)₅-(O(CH₂)₂)₂-O-(CH₂)₆-, -(CH₂),-N(Rₐ₇)-(CH₂)₁-, -(CH₂)₁-N(Rₐ₇)-(CH₂)₂-, - (CH₂)₁-N(Rₐ₇)-(CH₂)₃-, -(CH₂)₁-N(Rₐ₇)-(CH₂)₄-, -(CH₂)₁-N(Rₐ₇)-(CH₂)₅-, -(CH₂)₁-N(Rₐ₇)-(CH₂)₆-, -(CH₂)₁-N(Rₐ₇)-(CH₂)₇-, -(CH₂)₁-N(Rₐ₇)-(CH₂)₈-, -(CH₂)₁-N(Rₐ₇)-(CH₂)₉-, -(CH₂)₁-N(Rₐ₇)-(CH₂)₁₀-, -(CH₂)₂-N(Rₐ₇)-(CH₂)₁-, -(CH₂)₂-N(Rₐ₇)-(CH₂)₂-, -(CH₂)₂-N(Rₐ₇)-(CH₂)₃-, -(CH₂)₂-N(Rₐ₇)-(CH₂)₄-, -(CH₂)₂-N(Rₐ₇)-(CH₂)₅-, - (CH₂)₂-N(Rₐ₇)-(CH₂)₆-, -(CH₂)₂-N(Rₐ₇)-(CH₂)₇-, -(CH₂)₂-N(Rₐ₇)-(CH₂)₈-, -(CH₂)₂-N(Rₐ₇)-(CH₂)₉-, -(CH₂)₂-N(Rₐ₇)-(CH₂)₁₀-, -(CH₂)₃-N(Rₐ₇)-(CH₂)₁-, -(CH₂)₃-N(Rₐ₇)-(CH₂)₂-, -(CH₂)₃-N(Rₐ₇)-(CH₂)₃-, -(CH₂)₄-N(Rₐ₇)-(CH₂)₁-, -(CH₂)₄-N(Rₐ₇)-(CH₂)₂-, -(CH₂)₄-N(Rₐ₇)-(CH₂)₃-, -(CH₂)₄-N(Rₐ₇)-(CH₂)₄-, -(CH₂)₅-N(Rₐ₇)-(CH₂)₁-, - (CH₂)₅-N(Rₐ₇)-(CH₂)₂-, -(CH₂)₅-N(Rₐ₇)-(CH₂)₃-, -(CH₂)₅-N(Rₐ₇)-(CH₂)₄-, -(CH₂)₅-N(Rₐ₇)-(CH₂)₅-, -(CH₂)₆-N(Rₐ₇)-(CH₂)₁-, -(CH₂)₆-N(Rₐ₇)-(CH₂)₂-, -(CH₂)₆-N(Rₐ₇)-(CH₂)₃-, -(CH₂)₇-N(Rₐ₇)-(CH₂)₁-, -(CH₂)₇-N(Rₐ₇)-(CH₂)₂-, -(CH₂)₇-N(Rₐ₇)-(CH₂)₃-, -(CH₂)₈-N(Rₐ₇)-(CH₂)₁-, -(CH₂)₈-N(Rₐ₇)-(CH₂)₂-, -(CH₂)₈-N(Rₐ₇)-(CH₂)₃-, - CH(CH₃)-N(Rₐ₇)-(CH₂)₁-, -CH(CH₃)-N(Rₐ₇)-(CH₂)₂-, -CH(CH₃)-N(Rₐ₇)-(CH₂)₃-, -CH(CH₃)-N(Rₐ₇)-(CH₂)₄-, - CH(CH₃)-N(Rₐ₇)-(CH₂)₅-, -CH(CH₃)-N(Rₐ₇)-(CH₂)₆-, -CH(CH₃)-N(Rₐ₇)-(CH₂)₇-, -CH(CH₃)-N(Rₐ₇)-(CH₂)₈-, - CH(CH₃)-N(Rₐ₇)-(CH₂)₉-, -CH(CH₃)-N(Rₐ₇)-(CH₂)₁₀-, -CH₂C(O)NHCH₂-, -(CH₂)₂C(O)NH(CH₂)₂-, - (CH₂)₂C(O)NH(CH₂)₃-, -(CH₂)₂C(O)NH(CH₂)₄-, -(CH₂)₂C(O)NH(CH₂)₅-, -(CH₂)₃C(O)NH(CH₂)₃-, - (CH₂)₃C(O)NH(CH₂)₄-, -(CH₂)₄C(O)NH(CH₂)₄-, -(CH₂)₅C(O)NH(CH₂)₅-, -(CH₂)₆C(O)NH(CH₂)₇-, - (CH₂)₆C(O)NH(CH₂)₆-, -(CH₂)₇C(O)NH(CH₂)₇-, -(CH₂)₂C(O)NH(CH₂)₂-O-(CH₂)₂-, -CH₂NHC(O)CH₂-, - (CH₂)₂NHC(O)(CH₂)₂-, -(CH₂)₂NHC(O)(CH₂)₃-, -(CH₂)₂NHC(O)(CH₂)₄-, -(CH₂)₂NHC(O)(CH₂)₅-, - (CH₂)₃NHC(O)(CH₂)₃-, -(CH₂)₃NHC(O)(CH₂)₄-, -(CH₂)₄NHC(O)(CH₂)₄-, -(CH₂)₅NHC(O)(CH₂)₅-, - (CH₂)₆NHC(O)(CH₂)₇-, -(CH₂)₆NHC(O)(CH₂)₆-, -(CH₂)₇NHC(O)(CH₂)₇-, -(CH₂)₄NHC(O)(CH₂)₈-, - (CH₂)₂NHC(O)(CH₂)₂-O-(CH₂)₂-, -(CH₂)₄NHC(O)CH₂-, -CH₂-phenylene-CH₂-, -CH₂-phenylene-(CH₂)₂-, - CH₂-phenylene-(CH₂)₃-, -CH₂-phenylene-(CH₂)₄-, -CH₂-phenylene-(CH₂)₅-, -CH₂-phenylene-(CH₂)₆-, -CH₂-phenylene-(CH₂)₇-, -CH₂-phenylene-(CH₂)₈-, -(CH₂)₂-phenylene-(CH₂)₁-, -(CH₂)₂-phenylene-(CH₂)₂-, - (CH₂)₂-phenylene-(CH₂)₃-, -(CH₂)₂-phenylene-(CH₂)₄-, -(CH₂)₂-phenylene-(CH₂)₅-, -(CH₂)₂-phenylene-(CH₂)₆-, -(CH₂)₂-phenylene-(CH₂)₇-, -(CH₂)₂-phenylene-(CH₂)₈-, -(CH₂)₃-phenylene-CH₂-, -(CH₂)₃-phenylene-(CH₂)₂-, -(CH₂)₃-phenylene-(CH₂)₃-, -(CH₂)₃-phenylene-(CH₂)₄-, -(CH₂)₃-phenylene-(CH₂)₅-, - (CH₂)₃-phenylene-(CH₂)₆-, -(CH₂)₃-phenylene-(CH₂)₇-, -(CH₂)₃-phenylene-(CH₂)₈-, -(CH₂)₄-phenylene-CH₂-, -(CH₂)₄-phenylene-(CH₂)₂-, -(CH₂)₄-phenylene-(CH₂)₃-, -(CH₂)₄-phenylene-(CH₂)₄-, -(CH₂)₄-phenylene-(CH₂)₅-, -(CH₂)₄-phenylene-(CH₂)₆-, -(CH₂)₄-phenylene-(CH₂)₇-, -(CH₂)₄-phenylene-(CH₂)₈-, -(CH₂)₅-phenylene-(CH₂)₁-, -(CH₂)₅-phenylene-(CH₂)₂-, -(CH₂)₅-phenylene-(CH₂)₃-, -(CH₂)₅-phenylene-(CH₂)₄-, - (CH₂)₅-phenylene-(CH₂)₅-, -(CH₂)₅-phenylene-(CH₂)₆-, -(CH₂)₅-phenylene-(CH₂)₇-, -(CH₂)₅-phenylene-(CH₂)₈-, -(CH₂)₆-phenylene-(CH₂)₁-, -(CH₂)₆-phenylene-(CH₂)₂-, -(CH₂)₆-phenylene-(CH₂)₃-, -(CH₂)₆-phenylene-(CH₂)₄-, -(CH₂)₆-phenylene-(CH₂)₅-, -(CH₂)₆-phenylene-(CH₂)₆-, -(CH₂)₆-phenylene-(CH₂)₇-, - (CH₂)₆-phenylene-(CH₂)₈-, -(CH₂)₇-phenylene-(CH₂)₁-, -(CH₂)₇-phenylene-(CH₂)₂-, -(CH₂)₇-phenylene-(CH₂)₃-, -(CH₂)₇-phenylene-(CH₂)₄-, -(CH₂)₇-phenylene-(CH₂)₈-, -(CH₂)₈-phenylene-CH₂-, -(CH₂)₈-phenylene-(CH₂)₂-, -(CH₂)₈-phenylene-(CH₂)₃-, -(CH₂)₈-phenylene-(CH₂)₄-, -(CH₂)₈-phenylene-(CH₂)₅-, - (CH₂)₈-phenylene-(CH₂)₆-, -(CH₂)₈-phenylene-(CH₂)₇-, -CH₂-N(Rₐ₇)-CH₂-phenylene-CH₂-, -CH₂-N(Rₐ₇)-CH₂-phenylene-(CH₂)₂-, -CH₂-N(Rₐ₇)-CH₂-phenylene-(CH₂)₃-, -CH₂-N(Rₐ₇)-CH₂-phenylene-(CH₂)₄-, -CH₂-N(Rₐ₇)-CH₂-phenylene-(CH₂)₅-, -CH₂-N(Rₐ₇)-CH₂-phenylene-(CH₂)₆-, -CH₂-N(Rₐ₄₇)-CH₂-phenylene-(CH₂)₇-, -CH₂-N(Rₐ₇)-CH₂-phenylene-(CH₂)₈-, -CH₂-N(Rₐ₇)-(CH₂)₂-phenylene-CH₂-, -CH₂-N(Rₐ₇)-(CH₂)₂-phenylene-(CH₂)₂-, -CH₂-N(Rₐ₇)-(CH₂)₂-phenylene-(CH₂)₃-, -CH₂-N(Rₐ₇)-(CH₂)₂-phenylene-(CH₂)₄-, -CH₂-N(Rₐ₇)-(CH₂)₂-phenylene-(CH₂)₅-, -CH₂-N(Rₐ₇)-(CH₂)₂-phenylene-(CH₂)₆-, -CH₂-N(Rₐ₇)-(CH₂)₂-phenylene-(CH₂)₇-, -CH₂-N(Rₐ₇)-(CH₂)₂-phenylene-(CH₂)₈-, -(CH₂)₂-N(Rₐ₇)-CH₂-phenylene-CH₂-, -(CH₃)₂-N(Rₐ₇)-CH₂-phenylene-(CH₂)₂-, -(CH₂)₂-N(Rₐ₇)-CH₂-phenylene-(CH₂)₃-, -(CH₂)₂-N(Rₐ₇)-CH₂-phenylene-(CH₂)₄-, - (CH₂)₂-N(Rₐ₇)-CH₂-phenylene-(CH₂)₅-, -(CH₂)₂-N(Rₐ₇)-CH₂-phenylene-(CH₂)₆-, -(CH₂)₂-N(Rₐ₇)-CH₂-phenylene-(CH₂)₇-, -(CH₂)₂-N(Rₐ₇)-CH₂-phenylene-(CH₂)₈-, -(CH₂)₃-N(Rₐ₇)-CH₂-phenylene-CH₂-, -(CH₂)₃-N(Rₐ₇)-CH₂-phenylene-(CH₂)₂-, -(CH₂)₃-N(Rₐ₇)-CH₂-phenylene-(CH₂)₃-, -(CH₂)₃-N(Rₐ₇)-CH₂-phenylene-(CH₂)₈-, -(CH₂)₄-N(Rₐ₇)-CH₂-phenylene-CH₂-, -(CH₂)₄-N(Rₐ₇)-CH₂-phenylene-(CH₂)₂-, -(CH₂)₄-N(Rₐ₇)-CH₂-phenylene-(CH₂)₃-, -(CH₂)₄-N(Rₐ₇)-CH₂-phenylene-(CH₂)₈-, -(CH₂)₅-N(Rₐ₇)-CH₂-phenylene-(CH₂)₃-, - (CH₂)₅-N(Rₐ₇)-CH₂-phenylene-(CH₂)₈-, -(CH₂)₆-N(Rₐ₇)-CH₂-phenylene-(CH₂)₃-, -(CH₂)₆-N(Rₐ₇)-CH₂-phenylene-(CH₂)₈-, -(CH₂)₇-N(Rₐ₇)-CH₂-phenylene-(CH₂)₃-, -(CH₂)₈-N(Rₐ₇)-CH₂-phenylene-CH₂-, -(CH₂)₈-N(Rₐ₇)-CH₂-phenylene-(CH₂)₂-, -(CH₂)₈-N(Rₐ₇)-CH₂-phenylene-(CH₂)₃-, -(CH₂)₈-N(Rₐ₇)-CH₂-phenylene-(CH₂)₄-, -(CH₂)₈-N(Rₐ₇)-CH₂-phenylene-(CH₂)₅-, -(CH₂)₈-N(Rₐ₇)-CH₂-phenylene-(CH₂)₆-, -CH₂-piperidinylene-CH₂-, -CH₂-piperidinylene-(CH₂)₂-, -CH₂-piperidinylene-(CH₂)₃-, -CH₂-piperidinylene-(CH₂)₄-, -CH₂-piperidinylene-(CH₂)₅-, -CH₂-piperidinylene-(CH₂)₆-, -CH₂-piperidinylene-(CH₂)₇-, -CH₂-piperidinylene-(CH₂)₈-, -(CH₂)₂-piperidinylene-(CH₂)₁-, -(CH₂)₂-piperidinylene-(CH₂)₂-, -(CH₂)₂-piperidinylene-(CH₂)₃-, -(CH₂)₂-piperidinylene-(CH₂)₄-, -(CH₂)₂-piperidinylene-(CH₂)₅-, -(CH₂)₂-piperidinylene-(CH₂)₆-₁ -(CH₂)₂-piperidinylene-(CH₂)₇-, -(CH₂)₂-piperidinylene-(CH₂)₈-, -(CH₂)₃-piperidinylene-CH₂-, -(CH₂)₃-piperidinylene-(CH₂)₂-, -(CH₂)₃-piperidinylene-(CH₂)₃-, -(CH₂)₃-piperidinylene-(CH₂)₄-, -(CH₂)₃-piperidinylene-(CH₂)₅-, -(CH₂)₃-piperidinylene-(CH₂)₆-, -(CH₂)₃-piperidinylene-(CH₂)₇-, -(CH₂)₃-piperidinylene-(CH₂)₈-, -(CH₂)₄-piperidinylene-CH₂-, -(CH₂)₄-piperidinylene-(CH₂)₂-, -(CH₂)₄-piperidinylene-(CH₂)₃-, -(CH₂)₄-piperidinylene-(CH₂)₄-, -(CH₂)₄-piperidinylene-(CH₂)₅-, -(CH₂)₄-piperidinylene-(CH₂)₆-, -(CH₂)₄-piperidinylene-(CH₂)₇-, -(CH₂)₄-piperidinylene-(CH₂)₈-, -(CH₂)₅-piperidinylene-(CH₂)₁-, -(CH₂)₅-piperidinylene-(CH₂)₂-, -(CH₂)₅-piperidinylene-(CH₂)₃-, -(CH₂)₅-piperidinylene-(CH₂)₄-, -(CH₂)₅-piperidinylene-(CH₂)₅-, -(CH₂)₅-piperidinylene-(CH₂)₆-₁ -(CH₂)₅-piperidinylene-(CH₂)₇-, -(CH₂)₅-piperidinylene-(CH₂)₈-, -(CH₂)₆-piperidinylene-(CH₂)₁-, -(CH₂)₆-piperidinylene-(CH₂)₂-, -(CH₂)₆-piperidinylene-(CH₂)₃-, -(CH₂)₆-piperidinylene-(CH₂)₄-, -(CH₂)₆-piperidinylene-(CH₂)₅-, -(CH₂)₆-piperidinylene-(CH₂)₆-, -(CH₂)₆-piperidinylene-(CH₂)₇-₁ -(CH₂)₆-piperidinylene-(CH₂)₈-, -(CH₂)₇-piperidinylene-(CH₂)₁-, -(CH₂)₇-piperidinylene-(CH₂)₂-, -(CH₂)₇-piperidinylene-(CH₂)₃-, -(CH₂)₇-piperidinylene-(CH₂)₄-, -(CH₂)₇-piperidinylene-(CH₂)₈-, -(CH₂)₈-piperidinylene-CH₂-, -(CH₂)₈-piperidinylene-(CH₂)₂-, -(CH₂)₈-piperidinylene-(CH₂)₃-, -(CH₂)₈-piperidinylene-(CH₂)₄-, -(CH₂)₈-piperidinylene-(CH₂)₅-, -(CH₂)₈-piperidinylene-(CH₂)₆-, -(CH₂)₈-piperidinylene-(CH₂)₇-, -CH₂-N(Rₐ₇)-CH₂-piperidinylene-CH₂-, -CH₂-N(Rₐ₇)-CH₂-piperidinylene-(CH₂)₂-, -CH₂-N(Rₐ₇)-CH₂-piperidinylene-(CH₂)₃-, -CH₂-N(Rₐ₇)-CH₂-piperidinylene-(CH₂)₄-, -CH₂-N(Rₐ₇)-CH₂-piperidinylene-(CH₂)₅-, -CH₂-N(Rₐ₇)-CH₂-piperidinylene-(CH₂)₆-, -CH₂-N(Rₐ₇)-CH₂-piperidinylene-(CH₂)₇-, -CH₂-N(Rₐ₇)-CH₂-piperidinylene-(CH₂)₈-, -CH₂-N(Rₐ₇)-(CH₂)₂-piperidinylene-CH₂-, -CH₂-N(Rₐ₇)-(CH₂)₂-piperidinylene-(CH₂)₂-, -CH₂-N(Rₐ₄₇)-(CH₂)₂-piperidinylene-(CH₂)₃-, -CH₂-N(Rₐ₇)-(CH₂)₂-piperidinylene-(CH₂)₄-, -CH₂-N(Rₐ₇)-(CH₂)₂-piperidinylene-(CH₂)₅-, -CH₂-N(Rₐ₇)-(CH₂)₂-piperidinylene-(CH₂)₆-, -CH₂-N(Rₐ₇)-(CH₂)₂-piperidinylene-(CH₂)₇-, -CH₂-N(Rₐ₇)-(CH₂)₂-piperidinylene-(CH₂)₈-, -(CH₂)₂-N(Rₐ₇)-CH₂-piperidinylene-CH₂-, -(CH₂)₂-N(Rₐ₇)-CH₂-piperidinylene-(CH₂)₂-, -(CH₂)₂-N(Rₐ₇)-CH₂-piperidinylene-(CH₂)₃-, -(CH₂)₂-N(Rₐ₇)-CH₂-piperidinylene-(CH₂)₄-, -(CH₂)₂-N(Rₐ₇)-CH₂-piperidinylene-(CH₂)₅-, -(CH₂)₂-N(Rₐ₇)-CH₂-piperidinylene-(CH₂)₆-, -(CH₂)₂-N(Rₐ₇)-CH₂-piperidinylene-(CH₂)₇-, -(CH₂)₂-N(Rₐ₇)-CH₂-piperidinylene-(CH₂)₈-, -(CH₂)₃-N(Rₐ₇)-CH₂-piperidinylene-CH₂-, -(CH₂)₃-N(Rₐ₇)-CH₂-piperidinylene-(CH₂)₂-, -(CH₂)₃-N(Rₐ₇)-CH₂-piperidinylene-(CH₂)₃-, -(CH₂)₃-N(Rₐ₇)-CH₂-piperidinylene-(CH₂)₈-, -(CH₂)₄-N(Rₐ₇)-CH₂-piperidinylene-CH₂-, -(CH₂)₄-N(Rₐ₇)-CH₂-piperidinylene-(CH₂)₂-, -(CH₂)₄-N(Rₐ₇)-CH₂-piperidinylene-(CH₂)₃-, -(CH₂)₄-N(Rₐ₇)-CH₂-piperidinylene-(CH₂)₈-, -(CH₂)₅-N(Rₐ₇)-CH₂-piperidinylene-(CH₂)₃-, -(CH₂)₅-N(Rₐ₄₇)-CH₂-piperidinylene-(CH₂)₈-, -(CH₂)₆-N(Rₐ₇)-CH₂-piperidinylene-(CH₂)₃-, -(CH₂)₆-N(Rₐ₇)-CH₂-piperidinylene-(CH₂)₈-, -(CH₂)₇-N(Rₐ₇)-CH₂-piperidinylene-(CH₂)₃-, -(CH₂)₈-N(Rₐ₇)-CH₂-piperidinylene-CH₂-, -(CH₂)₈-N(Rₐ₇)-CH₂-piperidinylene-(CH₂)₂-, -(CH₂)₈-N(Rₐ₇)-CH₂-piperidinylene-(CH₂)₃-, -(CH₂)₈-N(Rₐ₇)-CH₂-piperidinylene-(CH₂)₄-, -(CH₂)₈-N(Rₐ₇)-CH₂-piperidinylene-(CH₂)₅-, -(CH₂)₈-N(Rₐ₇)-CH₂-piperidinylene-(CH₂)₆-, -CH₂-piperazinylene-CH₂-, -CH₂-piperazinylene-(CH₂)₂-, -CH₂-piperazinylene-(CH₂)₃-, -CH₂-piperazinylene-(CH₂)₄-, -CH₂-piperazinylene-(CH₂)₅-, -CH₂-piperazinylene-(CH₂)₆-, -CH₂-piperazinylene-(CH₂)₇-, -CH₂-piperazinylene-(CH₂)₈-, -(CH₂)₂-piperazinylene-(CH₂)₁-, -(CH₂)₂-piperazinylene-(CH₂)₂-, -(CH₂)₂-piperazinylene-(CH₂)₃-, - (CH₂)₂-piperazinylene-(CH₂)₄-, -(CH₂)₂-piperazinylene-(CH₂)₅-, -(CH₂)₂-piperazinylene-(CH₂)₆-, -(CH₂)₂-piperazinylene-(CH₂)₇-, -(CH₂)₂-piperazinylene-(CH₂)₈-, -(CH₂)₃-piperazinylene-CH₂-, -(CH₂)₃-piperazinylene-(CH₂)₂-, -(CH₂)₃-piperazinylene-(CH₂)₃-, -(CH₂)₃-piperazinylene-(CH₂)₄-, -(CH₂)₃-piperazinylene-(CH₂)₅-, -(CH₂)₃-piperazinylene-(CH₂)₆-, -(CH₂)₃-piperazinylene-(CH₂)₇-, -(CH₂)₃-piperazinylene-(CH₂)₈-, -(CH₂)₄-piperazinylene-CH₂-, -(CH₂)₄-piperazinylene-(CH₂)₂-, -(CH₂)₄-piperazinylene-(CH₂)₃-, -(CH₂)₄-piperazinylene-(CH₂)₄-, -(CH₂)₄-piperazinylene-(CH₂)₅-, -(CH₂)₄-piperazinylene-( CH₂)₆-, -(CH₂)₄-piperazinylene-(CH₂)₇-, -(CH₂)₄-piperazinylene-(CH₂)₈-, -(CH₂)₅-piperazinylene-(CH₂)₁-, -(CH₂)₅-piperazinylene-(CH₂)₂-, -(CH₂)₅-piperazinylene-(CH₂)₃-, -(CH₂)₅-piperazinylene-(CH₂)₄-, -(CH₂)₅-piperazinylene-(CH₂)₅-, -(CH₂)₅-piperazinylene-(CH₂)₆-, -(CH₂)₅-piperazinylene-(CH₂)₇-, -(CH₂)₅-piperazinylene-(CH₂)₈-, -(CH₂)₆-piperazinylene-(CH₂)₁-, -(CH₂)₆-piperazinylene-(CH₂)₂-, -(CH₂)₆-piperazinylene-(CH₂)₃-, -(CH₂)₆-piperazinylene-(CH₂)₄-, -(CH₂)₆-piperazinylene-(CH₂)₅-, -(CH₂)₆-piperazinylene-(CH₂)₆-, -(CH₂)₆-piperazinylene-(CH₂)₇-, -(CH₂)₆-piperazinylene-(CH₂)₈-, -(CH₂)₇-piperazinylene-(CH₂)₁-, -(CH₂)₇-piperazinylene-(CH₂)₂-, -(CH₂)₇-piperazinylene-(CH₂)₃-, -(CH₂)₇-piperazinylene-(CH₂)₄-, -(CH₂)₈-piperazinylene-CH₂-, -(CH₂)₈-piperazinylene-(CH₂)₂-, -(CH₂)₈-piperazinylene-(CH₂)₃-, -(CH₂)₈-piperazinylene-(CH₂)₄-, -(CH₂)₈-piperazinylene-(CH₂)₅-, or -(CH₂)₈-piperazinylene-(CH₂)₆-;
wherein each Rₐ₇ independently represents H or C₁₋₃ alkyl;
the phenylene, piperazinylene and piperidinylene are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl and any combination thereof.

25. The compound of Formula (I) or salts, enantiomers, stereoisomers, solvates, isotopically enriched analogs, prodrugs, or polymorphs thereof as claimed in claim 18, which is selected from:
3-(6-(bromomethyl)pyridin-3-yl)piperidine-2,6-dione;
3-(6-(bromomethyl)-5-fluoropyridin-3-yl)piperidine-2,6-dione;
3-(6-(bromomethyl)-4-fluoropyridin-3-yl)piperidine-2,6-dione;
3-(6-(bromomethyl)-2-fluoropyridin-3-yl)piperidine-2,6-dione;
3-(5-(bromomethyl)pyridin-2-yl)piperidine-2,6-dione;
3-(5-(bromomethyl)-4-fluoropyridin-2-yl)piperidine-2,6-dione;
3-(5-(bromomethyl)-3-fluoropyridin-2-yl)piperidine-2,6-dione;
3-(5-(bromomethyl)-6-fluoropyridin-2-yl)piperidine-2,6-dione;
3-(3-(bromomethyl)pyridin-2-yl)piperidine-2,6-dione;
3-(3-(bromomethyl)-4-fluoropyridin-2-yl)piperidine-2,6-dione;
3-(3-(bromomethyl)-5-fluoropyridin-2-yl)piperidine-2,6-dione;
3-(3-(bromomethyl)-6-fluoropyridin-2-yl)piperidine-2,6-dione;
3-(5-(bromomethyl)pyridin-3-yl)piperidine-2,6-dione;
3-(5-(bromomethyl)-4-fluoropyridin-3-yl)piperidine-2,6-dione;
3-(5-(bromomethyl)-6-fluoropyridin-3-yl)piperidine-2,6-dione;
3-(5-(bromomethyl)-2-fluoropyridin-3-yl)piperidine-2,6-dione;
3-(4-(bromomethyl)pyridin-3-yl)piperidine-2,6-dione;
3-(4-(bromomethyl)-5-fluoropyridin-3-yl)piperidine-2,6-dione;
3-(4-(bromomethyl)-6-fluoropyridin-3-yl)piperidine-2,6-dione;
3-(4-(bromomethyl)-2-fluoropyridin-3-yl)piperidine-2,6-dione;
3-(4-(bromomethyl)pyridin-2-yl)piperidine-2,6-dione;
3-(4-(bromomethyl)-3-fluoropyridin-2-yl)piperidine-2,6-dione;
3-(4-(bromomethyl)-5-fluoropyridin-2-yl)piperidine-2,6-dione;
3-(4-(bromomethyl)-6-fluoropyridin-2-yl)piperidine-2,6-dione;
3-(6-(bromomethyl)pyridazin-3-yl)piperidine-2,6-dione;
3-(6-(bromomethyl)-5-fluoropyridazin-3-yl)piperidine-2,6-dione;
3-(6-(bromomethyl)-4-fluoropyridazin-3-yl)piperidine-2,6-dione;
3-(6-(bromomethyl)pyridazin-4-yl)piperidine-2,6-dione;
3-(6-(bromomethyl)-5-fluoropyridazin-4-yl)piperidine-2,6-dione;
3-(6-(bromomethyl)-3-fluoropyridazin-4-yl)piperidine-2,6-dione;
3-(6-(bromomethyl)pyrimidin-4-yl)piperidine-2,6-dione;
3-(5-(bromomethyl)pyrazin-2-yl)piperidine-2,6-dione;
3-(6-(bromomethyl)pyrazin-2-yl)piperidine-2,6-dione;
3-(4-(bromomethyl)pyridazin-3-yl)piperidine-2,6-dione;
3 -(5 -(bromomethyl)pyridazin-3-yl)piperidine-2,6-dione;
3-(5-(bromomethyl)pyrimidin-2-yl)piperidine-2,6-dione;
3-(5-(bromomethyl)pyrimidin-4-yl)piperidine-2,6-dione;
1-(6-methylpyridin-3-yl)dihydropyrimidine-2,4(1H,3H)-dione;
1-(6-(azidomethyl)pyridin-3-yl)dihydropyrimidine-2,4(1H,3H)-dione;
1-(6-(bromomethyl)pyridin-3-yl)dihydropyrimidine-2,4(1H,3H)-dione;
1-(6-(bromomethyl)-5-fluoropyridin-3-yl)dihydropyrimidine-2,4(1H,3H)-dione;
1-(6-(bromomethyl)-4-fluoropyridin-3-yl)dihydropyrimidine-2,4(1H,3H)-dione;
1-(6-(bromomethyl)-2-fluoropyridin-3-yl)dihydropyrimidine-2,4(1H,3H)-dione;
1-(5-(azidomethyl)pyridin-2-yl)dihydropyrimidine-2,4(1H,3H)-dione;
1-(5-(bromomethyl)pyridin-2-yl)dihydropyrimidine-2,4(1H,3H)-dione;
1-(5-(bromomethyl)-4-fluoropyridin-2-yl)dihydropyrimidine-2,4(1H,3H)-dione;
1-(5-(bromomethyl)-3-fluoropyridin-2-yl)dihydropyrimidine-2,4(1H,3H)-dione;
1-(5-(bromomethyl)-6-fluoropyridin-2-yl)dihydropyrimidine-2,4(1H,3H)-dione;
1-(3-(bromomethyl)pyridin-2-yl)dihydropyrimidine-2,4(1H,3H)-dione;
1-(3-(bromomethyl)-4-fluoropyridin-2-yl)dihydropyrimidine-2,4(1H,3H)-dione;
1-(3-(bromomethyl)-5-fluoropyridin-2-yl)dihydropyrimidine-2,4(1H,3H)-dione;
1-(3-(bromomethyl)-6-fluoropyridin-2-yl)dihydropyrimidine-2,4(1H,3H)-dione;
1-(5-(azidomethyl)pyridin-3-yl)dihydropyrimidine-2,4(1H,3H)-dione;
1-(5-(bromomethyl)pyridin-3-yl)dihydropyrimidine-2,4(1H,3H)-dione;
1-(5-(bromomethyl)-4-fluoropyridin-3-yl)dihydropyrimidine-2,4(1H,3H)-dione;
1-(6-fluoro-5-(hydroxymethyl)pyridin-3-yl)dihydropyrimidine-2,4(1H,3H)-dione;
1-(5-(bromomethyl)-6-fluoropyridin-3-yl)dihydropyrimidine-2,4(1H,3H)-dione;
1-(5-(bromomethyl)-2-fluoropyridin-3-yl)dihydropyrimidine-2,4(1H,3H)-dione;
1-(4-(bromomethyl)pyridin-3-yl)dihydropyrimidine-2,4(1H,3H)-dione;
1-(4-(bromomethyl)-5-fluoropyridin-3-yl)dihydropyrimidine-2,4(1H,3H)-dione;
1-(4-(bromomethyl)-6-fluoropyridin-3-yl)dihydropyrimidine-2,4(1H,3H)-dione;
1-(4-(bromomethyl)-2-fluoropyridin-3-yl)dihydropyrimidine-2,4(1H,3H)-dione;
1-(4-(bromomethyl)pyridin-2-yl)dihydropyrimidine-2,4(1H,3H)-dione;
1-(4-(bromomethyl)-3-fluoropyridin-2-yl)dihydropyrimidine-2,4(1H,3H)-dione;
1-(4-(bromomethyl)-5-fluoropyridin-2-yl)dihydropyrimidine-2,4(1H,3H)-dione;
1-(4-(bromomethyl)-6-fluoropyridin-2-yl)dihydropyrimidine-2,4(1H,3H)-dione;
1-(6-(bromomethyl)pyridazin-3-yl)dihydropyrimidine-2,4(1H,3H)-dione;
1-(6-(bromomethyl)-5-fluoropyridazin-3-yl)dihydropyrimidine-2,4(1H,3H)-dione;
1-(6-(bromomethyl)-4-fluoropyridazin-3-yl)dihydropyrimidine-2,4(1H,3H)-dione;
1-(6-(bromomethyl)pyridazin-4-yl)dihydropyrimidine-2,4(1H,3H)-dione;
1-(6-(bromomethyl)-5-fluoropyridazin-4-yl)dihydropyrimidine-2,4(1H,3H)-dione;
1-(6-(bromomethyl)-3-fluoropyridazin-4-yl)dihydropyrimidine-2,4(1H,3H)-dione;
1-(6-(bromomethyl)pyrimidin-4-yl)dihydropyrimidine-2,4(1H,3H)-dione;
1-(5-(hydroxymethyl)pyrazin-2-yl)dihydropyrimidine-2,4(1H,3H)-dione;
1-(5-(bromomethyl)pyrazin-2-yl)dihydropyrimidine-2,4(1H,3H)-dione;
1-(6-(hydroxymethyl)pyrazin-2-yl)dihydropyrimidine-2,4(1H,3H)-dione;
1-(6-(azidomethyl)pyrazin-2-yl)dihydropyrimidine-2,4(1H,3H)-dione;
1-(6-(bromomethyl)pyrazin-2-yl)dihydropyrimidine-2,4(1H,3H)-dione;
1-(4-(bromomethyl)pyridazin-3-yl)dihydropyrimidine-2,4(1H,3H)-dione;
1-(5-(bromomethyl)pyridazin-3-yl)dihydropyrimidine-2,4(1H,3H)-dione;
1-(5-(bromomethyl)pyrimidin-2-yl)dihydropyrimidine-2,4(1H,3H)-dione;
1-(5-(bromomethyl)pyrimidin-4-yl)dihydropyrimidine-2,4(1H,3H)-dione;
3-((6-(hydroxymethyl)pyridin-3-yl)amino)piperidine-2,6-dione;
5-((2,6-dioxopiperidin-3-yl)amino)picolinaldehyde;
3-((6-(((tert-butyldimethylsilyl)oxy)methyl)pyridin-3-yl)amino)piperidine-2,6-dione;
3-((6-(bromomethyl)pyridin-3-yl)amino)piperidine-2,6-dione;
3-((6-(bromomethyl)-5-fluoropyridin-3-yl)amino)piperidine-2,6-dione;
3-((6-(bromomethyl)-4-fluoropyridin-3-yl)amino)piperidine-2,6-dione;
3-((6-(bromomethyl)-2-fluoropyridin-3-yl)amino)piperidine-2,6-dione;
3-((5-(hydroxymethyl)pyridin-2-yl)amino)piperidine-2,6-dione;
3-((5-(((tert-butyldimethylsilyl)oxy)methyl)pyridin-2-yl)amino)piperidine-2,6-dione;
3-((5-(bromomethyl)pyridin-2-yl)amino)piperidine-2,6-dione;
3-((5-(bromomethyl)-4-fluoropyridin-2-yl)amino)piperidine-2,6-dione;
3-((5-(bromomethyl)-3-fluoropyridin-2-yl)amino)piperidine-2,6-dione;
3-((5-(bromomethyl)-6-fluoropyridin-2-yl)amino)piperidine-2,6-dione;
3-((3-(hydroxymethyl)pyridin-2-yl)amino)piperidine-2,6-dione;
2-((2,6-dioxopiperidin-3-yl)amino)nicotinaldehyde;
3-((3-(((tert-butyldimethylsilyl)oxy)methyl)pyridin-2-yl)amino)piperidine-2,6-dione;
3-((3-(bromomethyl)pyridin-2-yl)amino)piperidine-2,6-dione;
3-((3-(bromomethyl)-4-fluoropyridin-2-yl)amino)piperidine-2,6-dione;
3-((3-(bromomethyl)-5-fluoropyridin-2-yl)amino)piperidine-2,6-dione;
3-((3-(bromomethyl)-6-fluoropyridin-2-yl)amino)piperidine-2,6-dione;
5-((2,6-dioxopiperidin-3-yl)amino)nicotinaldehyde;
3-((5-(hydroxymethyl)pyridin-3-yl)amino)piperidine-2,6-dione;
3-((5-(((tert-butyldimethylsilyl)oxy)methyl)pyridin-3-yl)amino)piperidine-2,6-dione;
3-((5-(bromomethyl)pyridin-3-yl)amino)piperidine-2,6-dione;
3-((5-(bromomethyl)-4-fluoropyridin-3-yl)amino)piperidine-2,6-dione;
3-((5-(bromomethyl)-6-fluoropyridin-3-yl)amino)piperidine-2,6-dione;
3-((5-(bromomethyl)-2-fluoropyridin-3-yl)amino)piperidine-2,6-dione;
3-((4-(hydroxymethyl)pyridin-3-yl)amino)piperidine-2,6-dione;
3-((2,6-dioxopiperidin-3-yl)amino)isonicotinaldehyde;
3-((4-(((tert-butyldimethylsilyl)oxy)methyl)pyridin-3-yl)amino)piperidine-2,6-dione;
3-((4-(bromomethyl)pyridin-3-yl)amino)piperidine-2,6-dione;
3-((4-(bromomethyl)-5-fluoropyridin-3-yl)amino)piperidine-2,6-dione;
3-((4-(bromomethyl)-6-fluoropyridin-3-yl)amino)piperidine-2,6-dione;
3-((4-(bromomethyl)-2-fluoropyridin-3-yl)amino)piperidine-2,6-dione;
3-((4-(hydroxymethyl)pyridin-2-yl)amino)piperidine-2,6-dione;
2-((2,6-dioxopiperidin-3-yl)amino)isonicotinaldehyde;
3-((4-(((tert-butyldimethylsilyl)oxy)methyl)pyridin-2-yl)amino)piperidine-2,6-dione;
3-((4-(bromomethyl)pyridin-2-yl)amino)piperidine-2,6-dione;
3-((4-(bromomethyl)-3-fluoropyridin-2-yl)amino)piperidine-2,6-dione;
3-((5-fluoro-4-(hydroxymethyl)pyridin-2-yl)amino)piperidine-2,6-dione;
3-((4-(((tert-butyldimethylsilyl)oxy)methyl)-5-fluoropyridin-2-yl)amino)piperidine-2,6-dione;
3-((4-(bromomethyl)-5-fluoropyridin-2-yl)amino)piperidine-2,6-dione;
3-((4-(bromomethyl)-6-fluoropyridin-2-yl)amino)piperidine-2,6-dione;
3-((6-(hydroxymethyl)pyridazin-3-yl)amino)piperidine-2,6-dione;
3-((6-(((tert-butyldimethylsilyl)oxy)methyl)pyridazin-3-yl)amino)piperidine-2,6-dione;
3-((6-(bromomethyl)pyridazin-3-yl)amino)piperidine-2,6-dione;
3-((6-(bromomethyl)-5-fluoropyridazin-3-yl)amino)piperidine-2,6-dione;
3-((6-(bromomethyl)-4-fluoropyridazin-3-yl)amino)piperidine-2,6-dione;
3-((6-(bromomethyl)pyridazin-4-yl)amino)piperidine-2,6-dione;
3-((6-(bromomethyl)-5-fluoropyridazin-4-yl)amino)piperidine-2,6-dione;
3-((6-(bromomethyl)-3-fluoropyridazin-4-yl)amino)piperidine-2,6-dione;
4-((2,6-dioxopiperidin-3-yl)amino)pyrimidine-5-carbaldehyde;
3-((5-(hydroxymethyl)pyrimidin-4-yl)amino)piperidine-2,6-dione;
3-((5-(((tert-butyldimethylsilyl)oxy)methyl)pyrimidin-4-yl)amino)piperidine-2,6-dione;
3-((6-(bromomethyl)pyrimidin-4-yl)amino)piperidine-2,6-dione;
3-((5-(bromomethyl)pyrazin-2-yl)amino)piperidine-2,6-dione;
3-((6-(bromomethyl)pyrazin-2-yl)amino)piperidine-2,6-dione;
3-((4-(bromomethyl)pyridazin-3-yl)amino)piperidine-2,6-dione;
3-((5-(bromomethyl)pyridazin-3-yl)amino)piperidine-2,6-dione;
2-((2,6-dioxopiperidin-3-yl)amino)pyrimidine-5-carbaldehyde;
3-((5-(hydroxymethyl)pyrimidin-2-yl)amino)piperidine-2,6-dione;
3-((5-(((tert-butyldimethylsilyl)oxy)methyl)pyrimidin-2-yl)amino)piperidine-2,6-dione;
3-((5-(bromomethyl)pyrimidin-2-yl)amino)piperidine-2,6-dione;
4-((2,6-dioxopiperidin-3-yl)amino)pyrimidine-5-carbaldehyde;
3-((5-(bromomethyl)pyrimidin-4-yl)amino)piperidine-2,6-dione;
3-(6-(chloromethyl)pyridin-3-yl)piperidine-2,6-dione;
3-(6-(chloromethyl)-5-fluoropyridin-3-yl)piperidine-2,6-dione;
3-(6-(chloromethyl)-4-fluoropyridin-3-yl)piperidine-2,6-dione;
3-(6-(chloromethyl)-2-fluoropyridin-3-yl)piperidine-2,6-dione;
3-(5-(chloromethyl)pyridin-2-yl)piperidine-2,6-dione;
3-(5-(chloromethyl)-4-fluoropyridin-2-yl)piperidine-2,6-dione;
3-(5-(chloromethyl)-3-fluoropyridin-2-yl)piperidine-2,6-dione;
3-(5-(chloromethyl)-6-fluoropyridin-2-yl)piperidine-2,6-dione;
3-(3-(chloromethyl)pyridin-2-yl)piperidine-2,6-dione;
3-(3-(chloromethyl)-4-fluoropyridin-2-yl)piperidine-2,6-dione;
3-(3-(chloromethyl)-5-fluoropyridin-2-yl)piperidine-2,6-dione;
3-(3-(chloromethyl)-6-fluoropyridin-2-yl)piperidine-2,6-dione;
3-(5-(chloromethyl)pyridin-3-yl)piperidine-2,6-dione;
3-(5-(chloromethyl)-4-fluoropyridin-3-yl)piperidine-2,6-dione;
3-(5-(chloromethyl)-6-fluoropyridin-3-yl)piperidine-2,6-dione;
3-(5-(chloromethyl)-2-fluoropyridin-3-yl)piperidine-2,6-dione;
3-(4-(chloromethyl)pyridin-3-yl)piperidine-2,6-dione;
3-(4-(chloromethyl)-5-fluoropyridin-3-yl)piperidine-2,6-dione;
3-(4-(chloromethyl)-6-fluoropyridin-3-yl)piperidine-2,6-dione;
3-(4-(chloromethyl)-2-fluoropyridin-3-yl)piperidine-2,6-dione;
3-(4-(chloromethyl)pyridin-2-yl)piperidine-2,6-dione;
3-(4-(chloromethyl)-3-fluoropyridin-2-yl)piperidine-2,6-dione;
3-(4-(chloromethyl)-5-fluoropyridin-2-yl)piperidine-2,6-dione;
3-(4-(chloromethyl)-6-fluoropyridin-2-yl)piperidine-2,6-dione;
3-(6-(chloromethyl)pyridazin-3-yl)piperidine-2,6-dione;
3-(6-(chloromethyl)-5-fluoropyridazin-3-yl)piperidine-2,6-dione;
3-(6-(chloromethyl)-4-fluoropyridazin-3-yl)piperidine-2,6-dione;
3-(6-(chloromethyl)pyridazin-4-yl)piperidine-2,6-dione;
3-(6-(chloromethyl)-5-fluoropyridazin-4-yl)piperidine-2,6-dione;
3-(6-(chloromethyl)-3-fluoropyridazin-4-yl)piperidine-2,6-dione;
3-(6-(chloromethyl)pyrimidin-4-yl)piperidine-2,6-dione;
3-(5-(chloromethyl)pyrazin-2-yl)piperidine-2,6-dione;
3-(6-(chloromethyl)pyrazin-2-yl)piperidine-2,6-dione;
3-(4-(chloromethyl)pyridazin-3-yl)piperidine-2,6-dione;
3-(5-(chloromethyl)pyridazin-3-yl)piperidine-2,6-dione;
3-(5-(chloromethyl)pyrimidin-2-yl)piperidine-2,6-dione;
3-(5-(chloromethyl)pyrimidin-4-yl)piperidine-2,6-dione;
1-(6-(chloromethyl)pyridin-3-yl)dihydropyrimidine-2,4(1H,3H)-dione;
1-(6-(chloromethyl)-5-fluoropyridin-3-yl)dihydropyrimidine-2,4(1H,3H)-dione;
1-(6-(chloromethyl)-4-fluoropyridin-3-yl)dihydropyrimidine-2,4(1H,3H)-dione;
1-(6-(chloromethyl)-2-fluoropyridin-3-yl)dihydropyrimidine-2,4(1H,3H)-dione;
1-(5-(chloromethyl)pyridin-2-yl)dihydropyrimidine-2,4(1H,3H)-dione;
1-(5-(chloromethyl)-4-fluoropyridin-2-yl)dihydropyrimidine-2,4(1H,3H)-dione;
1-(5-(chloromethyl)-3-fluoropyridin-2-yl)dihydropyrimidine-2,4(1H,3H)-dione;
1-(5-(chloromethyl)-6-fluoropyridin-2-yl)dihydropyrimidine-2,4(1H,3H)-dione;
1-(3-(chloromethyl)pyridin-2-yl)dihydropyrimidine-2,4(1H,3H)-dione;
1-(3-(chloromethyl)-4-fluoropyridin-2-yl)dihydropyrimidine-2,4(1H,3H)-dione;
1-(3-(chloromethyl)-5-fluoropyridin-2-yl)dihydropyrimidine-2,4(1H,3H)-dione;
1-(3-(chloromethyl)-6-fluoropyridin-2-yl)dihydropyrimidine-2,4(1H,3H)-dione;
1-(5-(chloromethyl)pyridin-3-yl)dihydropyrimidine-2,4(1H,3H)-dione;
1-(5-(chloromethyl)-4-fluoropyridin-3-yl)dihydropyrimidine-2,4(1H,3H)-dione;
1-(5-(azidomethyl)-6-fluoropyridin-3-yl)dihydropyrimidine-2,4(1H,3H)-dione;
1-(5-(chloromethyl)-6-fluoropyridin-3-yl)dihydropyrimidine-2,4(1H,3H)-dione;
1-(5-(chloromethyl)-2-fluoropyridin-3-yl)dihydropyrimidine-2,4(1H,3H)-dione;
1-(4-(chloromethyl)pyridin-3-yl)dihydropyrimidine-2,4(1H,3H)-dione;
1-(4-(chloromethyl)-5-fluoropyridin-3-yl)dihydropyrimidine-2,4(1H,3H)-dione;
1-(4-(chloromethyl)-6-fluoropyridin-3-yl)dihydropyrimidine-2,4(1H,3H)-dione;
1-(4-(chloromethyl)-2-fluoropyridin-3-yl)dihydropyrimidine-2,4(1H,3H)-dione;
1-(4-(azidomethyl)pyridin-2-yl)dihydropyrimidine-2,4(1H,3H)-dione;
1-(4-(chloromethyl)pyridin-2-yl)dihydropyrimidine-2,4(1H,3H)-dione;
1-(4-(chloromethyl)-3-fluoropyridin-2-yl)dihydropyrimidine-2,4(1H,3H)-dione;
1-(4-(azidomethyl)-5-fluoropyridin-2-yl)dihydropyrimidine-2,4(1H,3H)-dione;
1-(4-(chloromethyl)-5-fluoropyridin-2-yl)dihydropyrimidine-2,4(1H,3H)-dione;
1-(4-(chloromethyl)-6-fluoropyridin-2-yl)dihydropyrimidine-2,4(1H,3H)-dione;
1-(6-(chloromethyl)pyridazin-3-yl)dihydropyrimidine-2,4(1H,3H)-dione;
1-(6-(chloromethyl)-5-fluoropyridazin-3-yl)dihydropyrimidine-2,4(1H,3H)-dione;
1-(6-(chloromethyl)-4-fluoropyridazin-3-yl)dihydropyrimidine-2,4(1H,3H)-dione;
1-(6-(chloromethyl)pyridazin-4-yl)dihydropyrimidine-2,4(1H,3H)-dione;
1-(6-(chloromethyl)-5-fluoropyridazin-4-yl)dihydropyrimidine-2,4(1H,3H)-dione;
1-(6-(chloromethyl)-3-fluoropyridazin-4-yl)dihydropyrimidine-2,4(1H,3H)-dione;
1-(6-(chloromethyl)pyrimidin-4-yl)dihydropyrimidine-2,4(1H,3H)-dione;
1-(5-(chloromethyl)pyrazin-2-yl)dihydropyrimidine-2,4(1H,3H)-dione;
1-(6-(chloromethyl)pyrazin-2-yl)dihydropyrimidine-2,4(1H,3H)-dione;
1-(4-(chloromethyl)pyridazin-3-yl)dihydropyrimidine-2,4(1H,3H)-dione;
1-(5-(chloromethyl)pyridazin-3-yl)dihydropyrimidine-2,4(1H,3H)-dione;
1-(5-(chloromethyl)pyrimidin-2-yl)dihydropyrimidine-2,4(1H,3H)-dione;
1-(5-(chloromethyl)pyrimidin-4-yl)dihydropyrimidine-2,4(1H,3H)-dione;
3-((6-(chloromethyl)pyridin-3-yl)amino)piperidine-2,6-dione;
3-((6-(chloromethyl)-5-fluoropyridin-3-yl)amino)piperidine-2,6-dione;
3-((6-(chloromethyl)-4-fluoropyridin-3-yl)amino)piperidine-2,6-dione;
3-((6-(chloromethyl)-2-fluoropyridin-3-yl)amino)piperidine-2,6-dione;
3-((5-(chloromethyl)pyridin-2-yl)amino)piperidine-2,6-dione;
3-((5-(chloromethyl)-4-fluoropyridin-2-yl)amino)piperidine-2,6-dione;
3-((5-(chloromethyl)-3-fluoropyridin-2-yl)amino)piperidine-2,6-dione;
3-((5-(chloromethyl)-6-fluoropyridin-2-yl)amino)piperidine-2,6-dione;
3-((3-(chloromethyl)pyridin-2-yl)amino)piperidine-2,6-dione;
3-((3-(chloromethyl)-4-fluoropyridin-2-yl)amino)piperidine-2,6-dione;
3-((3-(chloromethyl)-5-fluoropyridin-2-yl)amino)piperidine-2,6-dione;
3-((3-(chloromethyl)-6-fluoropyridin-2-yl)amino)piperidine-2,6-dione;
3-((5-(chloromethyl)pyridin-3-yl)amino)piperidine-2,6-dione;
3-((5-(chloromethyl)-4-fluoropyridin-3-yl)amino)piperidine-2,6-dione;
3-((5-(chloromethyl)-6-fluoropyridin-3-yl)amino)piperidine-2,6-dione;
3-((5-(chloromethyl)-2-fluoropyridin-3-yl)amino)piperidine-2,6-dione;
3-((4-(chloromethyl)pyridin-3-yl)amino)piperidine-2,6-dione;
3-((4-(chloromethyl)-5-fluoropyridin-3-yl)amino)piperidine-2,6-dione;
3-((4-(chloromethyl)-6-fluoropyridin-3-yl)amino)piperidine-2,6-dione;
3-((4-(chloromethyl)-2-fluoropyridin-3-yl)amino)piperidine-2,6-dione;
3-((4-(chloromethyl)pyridin-2-yl)amino)piperidine-2,6-dione;
3-((4-(chloromethyl)-3-fluoropyridin-2-yl)amino)piperidine-2,6-dione;
3-((4-(chloromethyl)-5-fluoropyridin-2-yl)amino)piperidine-2,6-dione;
3-((4-(chloromethyl)-6-fluoropyridin-2-yl)amino)piperidine-2,6-dione;
3-((6-(chloromethyl)pyridazin-3-yl)amino)piperidine-2,6-dione;
3-((6-(chloromethyl)-5-fluoropyridazin-3-yl)amino)piperidine-2,6-dione;
3-((6-(chloromethyl)-4-fluoropyridazin-3-yl)amino)piperidine-2,6-dione;
3-((6-(chloromethyl)pyridazin-4-yl)amino)piperidine-2,6-dione;
3-((6-(chloromethyl)-5-fluoropyridazin-4-yl)amino)piperidine-2,6-dione;
3-((6-(chloromethyl)-3-fluoropyridazin-4-yl)amino)piperidine-2,6-dione;
3-((6-(chloromethyl)pyrimidin-4-yl)amino)piperidine-2,6-dione;
3-((5-(chloromethyl)pyrazin-2-yl)amino)piperidine-2,6-dione;
3-((6-(chloromethyl)pyrazin-2-yl)amino)piperidine-2,6-dione;
3-((4-(chloromethyl)pyridazin-3-yl)amino)piperidine-2,6-dione;
3-((5-(chloromethyl)pyridazin-3-yl)amino)piperidine-2,6-dione;
3-((5-(chloromethyl)pyrimidin-2-yl)amino)piperidine-2,6-dione;
3-((5-(chloromethyl)pyrimidin-4-yl)amino)piperidine-2,6-dione;
3-(6-(hydroxymethyl)pyridin-3-yl)piperidine-2,6-dione;
(5-(2,6-dioxopiperidin-3-yl)pyridin-2-yl)methyl methanesulfonate;
3-(5-fluoro-6-(hydroxymethyl)pyridin-3-yl)piperidine-2,6-dione;
(5-(2,6-dioxopiperidin-3-yl)-3-fluoropyridin-2-yl)methylmethanesulfonate;
3-(2-fluoro-6-(hydroxymethyl)pyridin-3-yl)piperidine-2,6-dione;
(5-(2,6-dioxopiperidin-3-yl)-4-fluoropyridin-2-yl)methylmethanesulfonate;
(5-(2,6-dioxopiperidin-3-yl)-6-fluoropyridin-2-yl)methylmethanesulfonate;
3-(5-(hydroxymethyl)pyridin-2-yl)piperidine-2,6-dione;
(6-(2,6-dioxopiperidin-3-yl)pyridin-3-yl)methyl methanesulfonate;
(6-(2,6-dioxopiperidin-3-yl)-4-fluoropyridin-3-yl)methylmethanesulfonate;
3-(3-fluoro-5-(hydroxymethyl)pyridin-2-yl)piperidine-2,6-dione;
(6-(2,6-dioxopiperidin-3-yl)-5-fluoropyridin-3-yl)methylmethanesulfonate;
(6-(2,6-dioxopiperidin-3-yl)-2-fluoropyridin-3-yl)methylmethanesulfonate;
3-(3-(hydroxymethyl)pyridin-2-yl)piperidine-2,6-dione;
(2-(2,6-dioxopiperidin-3-yl)pyridin-3-yl)methyl methanesulfonate;
(2-(2,6-dioxopiperidin-3-yl)-4-fluoropyridin-3-yl)methylmethanesulfonate;
(2-(2,6-dioxopiperidin-3-yl)-5-fluoropyridin-3-yl)methylmethanesulfonate;
(2-(2,6-dioxopiperidin-3-yl)-6-fluoropyridin-3-yl)methylmethanesulfonate;
3-(5-(hydroxymethyl)pyridin-3-yl)piperidine-2,6-dione;
(5-(2,6-dioxopiperidin-3-yl)pyridin-3-yl)methyl methanesulfonate;
(5-(2,6-dioxopiperidin-3-yl)-4-fluoropyridin-3-yl)methylmethanesulfonate;
3-(6-fluoro-5-(hydroxymethyl)pyridin-3-yl)piperidine-2,6-dione;
(5-(2,6-dioxopiperidin-3-yl)-2-fluoropyridin-3-yl)methylmethanesulfonate;
(5-(2,6-dioxopiperidin-3-yl)-6-fluoropyridin-3-yl)methylmethanesulfonate;
(3-(2,6-dioxopiperidin-3-yl)pyridin-4-yl)methyl methanesulfonate;
(3-(2,6-dioxopiperidin-3-yl)-5-fluoropyridin-4-yl)methylmethanesulfonate;
(5-(2,6-dioxopiperidin-3-yl)-2-fluoropyridin-4-yl)methylmethanesulfonate;
(3-(2,6-dioxopiperidin-3-yl)-2-fluoropyridin-4-yl)methylmethanesulfonate;
3-(4-(hydroxymethyl)pyridin-2-yl)piperidine-2,6-dione;
(2-(2,6-dioxopiperidin-3-yl)pyridin-4-yl)methyl methanesulfonate;
(2-(2,6-dioxopiperidin-3-yl)-3-fluoropyridin-4-yl)methylmethanesulfonate;
3-(5-fluoro-4-(hydroxymethyl)pyridin-2-yl)piperidine-2,6-dione;
(2-(2,6-dioxopiperidin-3-yl)-5-fluoropyridin-4-yl)methylmethanesulfonate;
(2-(2,6-dioxopiperidin-3-yl)-6-fluoropyridin-4-yl)methylmethanesulfonate;
3-(6-(hydroxymethyl)pyridazin-3-yl)piperidine-2,6-dione;
(6-(2,6-dioxopiperidin-3-yl)pyridazin-3-yl)methyl methanesulfonate;
(6-(2,6-dioxopiperidin-3-yl)-4-fluoropyridazin-3-yl)methylmethanesulfonate;
(6-(2,6-dioxopiperidin-3-yl)-5-fluoropyridazin-3-yl)methylmethanesulfonate;
(5-(2,6-dioxopiperidin-3-yl)pyridazin-3-yl)methyl methanesulfonate;
(5-(2,6-dioxopiperidin-3-yl)-4-fluoropyridazin-3-yl)methylmethanesulfonate;
(5-(2,6-dioxopiperidin-3-yl)-6-fluoropyridazin-3-yl)methylmethanesulfonate;
(6-(2,6-dioxopiperidin-3-yl)pyrimidin-4-yl)methyl methanesulfonate;
(5-(2,6-dioxopiperidin-3-yl)pyrazin-2-yl)methyl methanesulfonate;
(6-(2,6-dioxopiperidin-3-yl)pyrazin-2-yl)methyl methanesulfonate;
(3-(2,6-dioxopiperidin-3-yl)pyridazin-4-yl)methyl methanesulfonate;
(6-(2,6-dioxopiperidin-3-yl)pyridazin-4-yl)methyl methanesulfonate;
(2-(2,6-dioxopiperidin-3-yl)pyrimidin-5-yl)methyl methanesulfonate;
(4-(2,6-dioxopiperidin-3-yl)pyrimidin-5-yl)methyl methanesulfonate;
1-(6-(hydroxymethyl)pyridin-3-yl)dihydropyrimidine-2,4(1H,3H)-dione;
(5-(2,4-dioxotetrahydropyrimidin-1(2H)-yl)pyridin-2-yl)methyl methanesulfonate;
1-(5-fluoro-6-(hydroxymethyl)pyridin-3-yl)dihydropyrimidine-2,4(1H,3H)-dione;
(5-(2,4-dioxotetrahydropyrimidin-1(2H)-yl)-3-fluoropyridin-2-yl)methylmethanesulfonate;
(5-(2,4-dioxotetrahydropyrimidin-1(2H)-yl)-4-fluoropyridin-2-yl)methylmethanesulfonate;
(5-(2,4-dioxotetrahydropyrimidin-1(2H)-yl)-6-fluoropyridin-2-yl)methylmethanesulfonate;
(6-(2,4-dioxotetrahydropyrimidin-1(2H)-yl)pyridin-3-yl)methyl methanesulfonate;
(6-(2,4-dioxotetrahydropyrimidin-1(2H)-yl)-4-fluoropyridin-3-yl)methylmethanesulfonate;
(6-(2,4-dioxotetrahydropyrimidin-1(2H)-yl)-5-fluoropyridin-3-yl)methylmethanesulfonate;
(6-(2,4-dioxotetrahydropyrimidin-1(2H)-yl)-2-fluoropyridin-3-yl)methylmethanesulfonate;
(2-(2,4-dioxotetrahydropyrimidin-1(2H)-yl)pyridin-3-yl)methyl methanesulfonate;
(2-(2,4-dioxotetrahydropyrimidin-1(2H)-yl)-4-fluoropyridin-3-yl)methylmethanesulfonate;
(2-(2,4-dioxotetrahydropyrimidin-1(2H)-yl)-5-fluoropyridin-3-yl)methylmethanesulfonate;
(2-(2,4-dioxotetrahydropyrimidin-1(2H)-yl)-6-fluoropyridin-3-yl)methylmethanesulfonate;
1-(5-(hydroxymethyl)pyridin-3-yl)dihydropyrimidine-2,4(1H,3H)-dione;
(5-(2,4-dioxotetrahydropyrimidin-1(2H)-yl)pyridin-3-yl)methyl methanesulfonate;
(5-(2,4-dioxotetrahydropyrimidin-1(2H)-yl)-4-fluoropyridin-3-yl)methylmethanesulfonate;
(5-(2,4-dioxotetrahydropyrimidin-1(2H)-yl)-2-fluoropyridin-3-yl)methylmethanesulfonate;
(5-(2,4-dioxotetrahydropyrimidin-1(2H)-yl)-6-fluoropyridin-3-yl)methylmethanesulfonate;
(3-(2,4-dioxotetrahydropyrimidin-1(2H)-yl)pyridin-4-yl)methyl methanesulfonate;
(3-(2,4-dioxotetrahydropyrimidin-1(2H)-yl)-5-fluoropyridin-4-yl)methylmethanesulfonate;
(5-(2,4-dioxotetrahydropyrimidin-1(2H)-yl)-2-fluoropyridin-4-yl)methylmethanesulfonate;
(3-(2,4-dioxotetrahydropyrimidin-1(2H)-yl)-2-fluoropyridin-4-yl)methylmethanesulfonate;
1-(4-(hydroxymethyl)pyridin-2-yl)dihydropyrimidine-2,4(1H,3H)-dione;
(2-(2,4-dioxotetrahydropyrimidin-1(2H)-yl)pyridin-4-yl)methyl methanesulfonate;
(2-(2,4-dioxotetrahydropyrimidin-1(2H)-yl)-3-fluoropyridin-4-yl)methylmethanesulfonate;
(2-(2,4-dioxotetrahydropyrimidin-1(2H)-yl)-5-fluoropyridin-4-yl)methylmethanesulfonate;
(2-(2,4-dioxotetrahydropyrimidin-1(2H)-yl)-6-fluoropyridin-4-yl)methylmethanesulfonate;
(6-(2,4-dioxotetrahydropyrimidin-1(2H)-yl)pyridazin-3-yl)methyl methanesulfonate;
(6-(2,4-dioxotetrahydropyrimidin-1(2H)-yl)-4-fluoropyridazin-3-yl)methylmethanesulfonate;
(6-(2,4-dioxotetrahydropyrimidin-1(2H)-yl)-5-fluoropyridazin-3-yl)methylmethanesulfonate;
(5-(2,4-dioxotetrahydropyrimidin-1(2H)-yl)pyridazin-3-yl)methyl methanesulfonate;
(5-(2,4-dioxotetrahydropyrimidin-1(2H)-yl)-4-fluoropyridazin-3-yl)methylmethanesulfonate;
(5-(2,4-dioxotetrahydropyrimidin-1(2H)-yl)-6-fluoropyridazin-3-yl)methylmethanesulfonate;
(6-(2,4-dioxotetrahydropyrimidin-1(2H)-yl)pyrimidin-4-yl)methyl methanesulfonate;
(5-(2,4-dioxotetrahydropyrimidin-1(2H)-yl)pyrazin-2-yl)methyl methanesulfonate;
(6-(2,4-dioxotetrahydropyrimidin-1(2H)-yl)pyrazin-2-yl)methyl methanesulfonate;
(3-(2,4-dioxotetrahydropyrimidin-1(2H)-yl)pyridazin-4-yl)methyl methanesulfonate;
(6-(2,4-dioxotetrahydropyrimidin-1(2H)-yl)pyridazin-4-yl)methyl methanesulfonate;
(2-(2,4-dioxotetrahydropyrimidin-1(2H)-yl)pyrimidin-5-yl)methyl methanesulfonate;
(4-(2,4-dioxotetrahydropyrimidin-1(2H)-yl)pyrimidin-5-yl)methyl methanesulfonate;
(5-((2,6-dioxopiperidin-3-yl)amino)pyridin-2-yl)methyl methanesulfonate;
(5-((2,6-dioxopiperidin-3-yl)amino)-3-fluoropyridin-2-yl)methyl methanesulfonate;
(5-((2,6-dioxopiperidin-3-yl)amino)-4-fluoropyridin-2-yl)methyl methanesulfonate;
(5-((2,6-dioxopiperidin-3-yl)amino)-6-fluoropyridin-2-yl)methyl methanesulfonate;
(6-((2,6-dioxopiperidin-3-yl)amino)pyridin-3-yl)methyl methanesulfonate;
(6-((2,6-dioxopiperidin-3-yl)amino)-4-fluoropyridin-3-yl)methyl methanesulfonate;
(6-((2,6-dioxopiperidin-3-yl)amino)-5-fluoropyridin-3-yl)methyl methanesulfonate;
(6-((2,6-dioxopiperidin-3-yl)amino)-2-fluoropyridin-3-yl)methyl methanesulfonate;
(2-((2,6-dioxopiperidin-3-yl)amino)pyridin-3-yl)methyl methanesulfonate;
(2-((2,6-dioxopiperidin-3-yl)amino)-4-fluoropyridin-3-yl)methyl methanesulfonate;
(2-((2,6-dioxopiperidin-3-yl)amino)-5-fluoropyridin-3-yl)methyl methanesulfonate;
(2-((2,6-dioxopiperidin-3-yl)amino)-6-fluoropyridin-3-yl)methyl methanesulfonate;
(5-((2,6-dioxopiperidin-3-yl)amino)pyridin-3-yl)methyl methanesulfonate;
(5-((2,6-dioxopiperidin-3-yl)amino)-4-fluoropyridin-3-yl)methyl methanesulfonate;
(5-((2,6-dioxopiperidin-3-yl)amino)-2-fluoropyridin-3-yl)methyl methanesulfonate;
(5-((2,6-dioxopiperidin-3-yl)amino)-6-fluoropyridin-3-yl)methyl methanesulfonate;
(3-((2,6-dioxopiperidin-3-yl)amino)pyridin-4-yl)methyl methanesulfonate;
(3-((2,6-dioxopiperidin-3-yl)amino)-5-fluoropyridin-4-yl)methyl methanesulfonate;
(5-((2,6-dioxopiperidin-3-yl)amino)-2-fluoropyridin-4-yl)methyl methanesulfonate;
(3-((2,6-dioxopiperidin-3-yl)amino)-2-fluoropyridin-4-yl)methyl methanesulfonate;
(2-((2,6-dioxopiperidin-3-yl)amino)pyridin-4-yl)methyl methanesulfonate;
(2-((2,6-dioxopiperidin-3-yl)amino)-3-fluoropyridin-4-yl)methyl methanesulfonate;
(2-((2,6-dioxopiperidin-3-yl)amino)-5-fluoropyridin-4-yl)methyl methanesulfonate;
(2-((2,6-dioxopiperidin-3-yl)amino)-6-fluoropyridin-4-yl)methyl methanesulfonate;
(6-((2,6-dioxopiperidin-3-yl)amino)pyridazin-3-yl)methyl methanesulfonate;
(6-((2,6-dioxopiperidin-3-yl)amino)-4-fluoropyridazin-3-yl)methyl methanesulfonate;
(6-((2,6-dioxopiperidin-3-yl)amino)-5-fluoropyridazin-3-yl)methyl methanesulfonate;
(5-((2,6-dioxopiperidin-3-yl)amino)pyridazin-3-yl)methyl methanesulfonate;
(5-((2,6-dioxopiperidin-3-yl)amino)-4-fluoropyridazin-3-yl)methyl methanesulfonate;
(5-((2,6-dioxopiperidin-3-yl)amino)-6-fluoropyridazin-3-yl)methyl methanesulfonate;
(6-((2,6-dioxopiperidin-3-yl)amino)pyrimidin-4-yl)methyl methanesulfonate;
(5-((2,6-dioxopiperidin-3-yl)amino)pyrazin-2-yl)methyl methanesulfonate;
(6-((2,6-dioxopiperidin-3-yl)amino)pyrazin-2-yl)methyl methanesulfonate;
(3-((2,6-dioxopiperidin-3-yl)amino)pyridazin-4-yl)methyl methanesulfonate;
(6-((2,6-dioxopiperidin-3-yl)amino)pyridazin-4-yl)methyl methanesulfonate;
(2-((2,6-dioxopiperidin-3-yl)amino)pyrimidin-5-yl)methyl methanesulfonate;
(4-((2,6-dioxopiperidin-3-yl)amino)pyrimidin-5-yl)methyl methanesulfonate;
3-(6-(aminomethyl)pyridin-3-yl)piperidine-2,6-dione;
3-(6-(aminomethyl)-5-fluoropyridin-3-yl)piperidine-2,6-dione;
3-(6-(aminomethyl)-4-fluoropyridin-3-yl)piperidine-2,6-dione;
3-(6-(aminomethyl)-2-fluoropyridin-3-yl)piperidine-2,6-dione;
3-(5-(aminomethyl)pyridin-2-yl)piperidine-2,6-dione;
3-(5-(aminomethyl)-4-fluoropyridin-2-yl)piperidine-2,6-dione;
3-(5-(aminomethyl)-3-fluoropyridin-2-yl)piperidine-2,6-dione;
3-(5-(aminomethyl)-6-fluoropyridin-2-yl)piperidine-2,6-dione;
3-(3-(aminomethyl)pyridin-2-yl)piperidine-2,6-dione;
3-(3-(aminomethyl)-4-fluoropyridin-2-yl)piperidine-2,6-dione;
3-(3-(aminomethyl)-5-fluoropyridin-2-yl)piperidine-2,6-dione;
3-(3-(aminomethyl)-6-fluoropyridin-2-yl)piperidine-2,6-dione;
3-(5-(aminomethyl)pyridin-3-yl)piperidine-2,6-dione;
3-(5-(aminomethyl)-4-fluoropyridin-3-yl)piperidine-2,6-dione;
3-(5-(aminomethyl)-6-fluoropyridin-3-yl)piperidine-2,6-dione;
3-(5-(aminomethyl)-2-fluoropyridin-3-yl)piperidine-2,6-dione;
3-(4-(aminomethyl)pyridin-3-yl)piperidine-2,6-dione;
3-(4-(aminomethyl)-5-fluoropyridin-3-yl)piperidine-2,6-dione;
3-(4-(aminomethyl)-6-fluoropyridin-3-yl)piperidine-2,6-dione;
3-(4-(aminomethyl)-2-fluoropyridin-3-yl)piperidine-2,6-dione;
3-(4-(aminomethyl)pyridin-2-yl)piperidine-2,6-dione;
3-(4-(aminomethyl)-3-fluoropyridin-2-yl)piperidine-2,6-dione;
3-(4-(aminomethyl)-5-fluoropyridin-2-yl)piperidine-2,6-dione;
3-(4-(aminomethyl)-6-fluoropyridin-2-yl)piperidine-2,6-dione;
3-(6-(aminomethyl)pyridazin-3-yl)piperidine-2,6-dione;
3-(6-(aminomethyl)-5-fluoropyridazin-3-yl)piperidine-2,6-dione;
3-(6-(aminomethyl)-4-fluoropyridazin-3-yl)piperidine-2,6-dione;
3-(6-(aminomethyl)pyridazin-4-yl)piperidine-2,6-dione;
3-(6-(aminomethyl)-5-fluoropyridazin-4-yl)piperidine-2,6-dione;
3-(6-(aminomethyl)-3-fluoropyridazin-4-yl)piperidine-2,6-dione;
3-(6-(aminomethyl)pyrimidin-4-yl)piperidine-2,6-dione;
3-(5-(aminomethyl)pyrazin-2-yl)piperidine-2,6-dione;
3-(6-(aminomethyl)pyrazin-2-yl)piperidine-2,6-dione;
3-(4-(aminomethyl)pyridazin-3-yl)piperidine-2,6-dione;
3-(5-(aminomethyl)pyridazin-3-yl)piperidine-2,6-dione;
3-(5-(aminomethyl)pyrimidin-2-yl)piperidine-2,6-dione;
3-(5-(aminomethyl)pyrimidin-4-yl)piperidine-2,6-dione;
1-(6-(aminomethyl)pyridin-3-yl)dihydropyrimidine-2,4(1H,3H)-dione;
1-(6-(aminomethyl)-5-fluoropyridin-3-yl)dihydropyrimidine-2,4(1H,3H)-dione;
1-(6-(aminomethyl)-4-fluoropyridin-3-yl)dihydropyrimidine-2,4(1H,3H)-dione;
1-(6-(aminomethyl)-2-fluoropyridin-3-yl)dihydropyrimidine-2,4(1H,3H)-dione;
1-(5-(aminomethyl)pyridin-2-yl)dihydropyrimidine-2,4(1H,3H)-dione;
1-(5-(aminomethyl)-4-fluoropyridin-2-yl)dihydropyrimidine-2,4(1H,3H)-dione;
1-(5-(aminomethyl)-3-fluoropyridin-2-yl)dihydropyrimidine-2,4(1H,3H)-dione;
1-(5-(aminomethyl)-6-fluoropyridin-2-yl)dihydropyrimidine-2,4(1H,3H)-dione;
1-(3-(aminomethyl)pyridin-2-yl)dihydropyrimidine-2,4(1H,3H)-dione;
1-(3-(aminomethyl)-4-fluoropyridin-2-yl)dihydropyrimidine-2,4(1H,3H)-dione;
1-(3-(aminomethyl)-5-fluoropyridin-2-yl)dihydropyrimidine-2,4(1H,3H)-dione;
1-(3-(aminomethyl)-6-fluoropyridin-2-yl)dihydropyrimidine-2,4(1H,3H)-dione;
1-(5-(aminomethyl)pyridin-3-yl)dihydropyrimidine-2,4(1H,3H)-dione;
1-(5-(aminomethyl)-4-fluoropyridin-3-yl)dihydropyrimidine-2,4(1H,3H)-dione;
1-(5-(aminomethyl)-6-fluoropyridin-3-yl)dihydropyrimidine-2,4(1H,3H)-dione;
1-(5-(aminomethyl)-2-fluoropyridin-3-yl)dihydropyrimidine-2,4(1H,3H)-dione;
1-(4-(aminomethyl)pyridin-3-yl)dihydropyrimidine-2,4(1H,3H)-dione;
1-(4-(aminomethyl)-5-fluoropyridin-3-yl)dihydropyrimidine-2,4(1H,3H)-dione;
1-(4-(aminomethyl)-6-fluoropyridin-3-yl)dihydropyrimidine-2,4(1H,3H)-dione;
1-(4-(aminomethyl)-2-fluoropyridin-3-yl)dihydropyrimidine-2,4(1H,3H)-dione;
1-(4-(aminomethyl)pyridin-2-yl)dihydropyrimidine-2,4(1H,3H)-dione;
1-(4-(aminomethyl)-3-fluoropyridin-2-yl)dihydropyrimidine-2,4(1H,3H)-dione;
1-(5-fluoro-4-(hydroxymethyl)pyridin-2-yl)dihydropyrimidine-2,4(1H,3H)-dione;
1-(4-(aminomethyl)-5-fluoropyridin-2-yl)dihydropyrimidine-2,4(1H,3H)-dione;
1-(4-(aminomethyl)-6-fluoropyridin-2-yl)dihydropyrimidine-2,4(1H,3H)-dione;
1-(6-(aminomethyl)pyridazin-3-yl)dihydropyrimidine-2,4(1H,3H)-dione;
1-(6-(aminomethyl)-5-fluoropyridazin-3-yl)dihydropyrimidine-2,4(1H,3H)-dione;
1-(6-(aminomethyl)-4-fluoropyridazin-3-yl)dihydropyrimidine-2,4(1H,3H)-dione;
1-(6-(aminomethyl)pyridazin-4-yl)dihydropyrimidine-2,4(1H,3H)-dione;
1-(6-(aminomethyl)-5-fluoropyridazin-4-yl)dihydropyrimidine-2,4(1H,3H)-dione;
1-(6-(aminomethyl)-3-fluoropyridazin-4-yl)dihydropyrimidine-2,4(1H,3H)-dione;
1-(6-(aminomethyl)pyrimidin-4-yl)dihydropyrimidine-2,4(1H,3H)-dione;
1-(5-(aminomethyl)pyrazin-2-yl)dihydropyrimidine-2,4(1H,3H)-dione;
1-(6-(aminomethyl)pyrazin-2-yl)dihydropyrimidine-2,4(1H,3H)-dione;
1-(4-(aminomethyl)pyridazin-3-yl)dihydropyrimidine-2,4(1H,3H)-dione;
1-(5-(aminomethyl)pyridazin-3-yl)dihydropyrimidine-2,4(1H,3H)-dione;
1-(5-(aminomethyl)pyrimidin-2-yl)dihydropyrimidine-2,4(1H,3H)-dione;
1-(5-(aminomethyl)pyrimidin-4-yl)dihydropyrimidine-2,4(1H,3H)-dione;
3-((6-(aminomethyl)pyridin-3-yl)amino)piperidine-2,6-dione;
3-((6-(aminomethyl)-5-fluoropyridin-3-yl)amino)piperidine-2,6-dione;
3-((6-(aminomethyl)-4-fluoropyridin-3-yl)amino)piperidine-2,6-dione;
3-((6-(aminomethyl)-2-fluoropyridin-3-yl)amino)piperidine-2,6-dione;
3-((5-(aminomethyl)pyridin-2-yl)amino)piperidine-2,6-dione;
3-((5-(aminomethyl)-4-fluoropyridin-2-yl)amino)piperidine-2,6-dione;
3-((5-(aminomethyl)-3-fluoropyridin-2-yl)amino)piperidine-2,6-dione;
3-((5-(aminomethyl)-6-fluoropyridin-2-yl)amino)piperidine-2,6-dione;
3-((3-(aminomethyl)pyridin-2-yl)amino)piperidine-2,6-dione;
3-((3-(aminomethyl)-4-fluoropyridin-2-yl)amino)piperidine-2,6-dione;
3-((3-(aminomethyl)-5-fluoropyridin-2-yl)amino)piperidine-2,6-dione;
3-((3-(aminomethyl)-6-fluoropyridin-2-yl)amino)piperidine-2,6-dione;
3-((5-(aminomethyl)pyridin-3-yl)amino)piperidine-2,6-dione;
3-((5-(aminomethyl)-4-fluoropyridin-3-yl)amino)piperidine-2,6-dione;
3-((5-(aminomethyl)-6-fluoropyridin-3-yl)amino)piperidine-2,6-dione;
3-((5-(aminomethyl)-2-fluoropyridin-3-yl)amino)piperidine-2,6-dione;
3-((4-(aminomethyl)pyridin-3-yl)amino)piperidine-2,6-dione;
3-((4-(aminomethyl)-5-fluoropyridin-3-yl)amino)piperidine-2,6-dione;
3-((4-(aminomethyl)-6-fluoropyridin-3-yl)amino)piperidine-2,6-dione;
3-((4-(aminomethyl)-2-fluoropyridin-3-yl)amino)piperidine-2,6-dione;
3-((4-(aminomethyl)pyridin-2-yl)amino)piperidine-2,6-dione;
3-((4-(aminomethyl)-3-fluoropyridin-2-yl)amino)piperidine-2,6-dione;
3-((4-(aminomethyl)-5-fluoropyridin-2-yl)amino)piperidine-2,6-dione;
3-((4-(aminomethyl)-6-fluoropyridin-2-yl)amino)piperidine-2,6-dione;
3-((6-(aminomethyl)pyridazin-3-yl)amino)piperidine-2,6-dione;
3-((6-(aminomethyl)-5-fluoropyridazin-3-yl)amino)piperidine-2,6-dione;
3-((6-(aminomethyl)-4-fluoropyridazin-3-yl)amino)piperidine-2,6-dione;
3-((6-(aminomethyl)pyridazin-4-yl)amino)piperidine-2,6-dione;
3-((6-(aminomethyl)-5-fluoropyridazin-4-yl)amino)piperidine-2,6-dione;
3-((6-(aminomethyl)-3-fluoropyridazin-4-yl)amino)piperidine-2,6-dione;
3-((6-(aminomethyl)pyrimidin-4-yl)amino)piperidine-2,6-dione;
3-((5-(aminomethyl)pyrazin-2-yl)amino)piperidine-2,6-dione;
3-((6-(aminomethyl)pyrazin-2-yl)amino)piperidine-2,6-dione;
3-((4-(aminomethyl)pyridazin-3-yl)amino)piperidine-2,6-dione;
3-((5-(aminomethyl)pyridazin-3-yl)amino)piperidine-2,6-dione;
3-((5-(aminomethyl)pyrimidin-2-yl)amino)piperidine-2,6-dione;
3-((5-(aminomethyl)pyrimidin-4-yl)amino)piperidine-2,6-dione;
1-(6-(hydroxymethyl)pyridin-3-yl)dihydropyrimidine-2,4(1H,3H)-dione;
1-(5-(hydroxymethyl)pyridin-2-yl)dihydropyrimidine-2,4(1H,3H)-dione; and
1-(5-(hydroxymethyl)pyridin-2-yl)dihydropyrimidine-2,4(1H,3H)-dione.

26. A pharmaceutical composition comprising as an active ingredient the compound of Formula (I) or pharmaceutically acceptable salts, enantiomers, stereoisomers, solvates, isotopically enriched analogs, prodrugs, or polymorphs thereof as claimed in any one of claims 1-17, or the compound of Formula (II) or pharmaceutically acceptable salts, enantiomers, stereoisomers, solvates, isotopically enriched analogs, prodrugs, or polymorphs thereof as claimed in any one of claims 18-25, and at least one pharmaceutically acceptable carrier.

27. The pharmaceutical composition as claimed in claim 26, further comprising at least one additional therapeutic agent, e.g., an anticancer agent.

28. A medicine kit or reagent kit comprising:
the compound of Formula (I) or a pharmaceutically acceptable salt thereof as claimed in any one of claims 1-17; or
the compound of Formula (II) or a pharmaceutically acceptable salt thereof as claimed in any one of claims 18-25; or
the pharmaceutical composition as claimed in claim 26 or 27.

29. The compound of Formula (I) or salts, enantiomers, stereoisomers, solvates, isotopically enriched analogs, prodrugs, or polymorphs thereof as claimed in any one of claims 1-17, for use in the prevention and/or treatment of a disease or disorder selected from the group consisting of: tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

30. The compound of Formula (I) or salts, enantiomers, stereoisomers, solvates, isotopically enriched analogs, prodrugs, or polymorphs thereof as claimed in claim 29, wherein the disease or disorder mediated or associated with a target protein is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML), acute B-lymphocytic leukemia, acute T-lymphocytic leukemia, chronic lymphocytic leukemia, lymphoblastic leukemia, T-lymphocytic leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), marginal zone lymphoma (MZL), primary lymphoma, Burkitt's lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer and small cell lung cancer; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

31. Use of the compound of Formula (I) or salts, enantiomers, stereoisomers, solvates, isotopically enriched analogs, prodrugs, or polymorphs thereof as claimed in any one of claims 1-17 for the manufacture of a medicament for the prevention and/or treatment of a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, cardiovascular diseases, Unverricht's syndrome, Richter syndrome (RS), acute liver failure, diabetes, Kennedy disease, seborrheic alopecia, hirsutism, skin disease, dysfunctional uterine bleeding, anemia, pediatric aplastic anemia, endometriosis, transplant rejection, polycystic ovary syndrome, and thyroid disease.

32. A method for treating or preventing a disease or disorder in a subject, comprising administering to the subject a therapeutically effective amount of the compound of Formula (I) or salts, enantiomers, stereoisomers, solvates, isotopically enriched analogs, prodrugs, or polymorphs thereof as claimed in any one of claims 1-17, or the pharmaceutical composition as claimed in claim 26 or 27, wherein the disease or disorder comprises tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, Unverricht's syndrome, Richter syndrome (RS), acute liver failure, diabetes, Kennedy disease, seborrheic alopecia, hirsutism, skin disease, cardiovascular diseases, dysfunctional uterine bleeding, anemia, pediatric aplastic anemia, endometriosis, transplant rejection, polycystic ovary syndrome, and thyroid disease.

33. The use as claimed in claim 31 or the method as claimed in claim 32, wherein the disease or disorder is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML), acute B-lymphocytic leukemia, acute T-lymphocytic leukemia, chronic lymphocytic leukemia, lymphoblastic leukemia, T-lymphocytic leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), marginal zone lymphoma (MZL), primary lymphoma, Burkitt's lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer and small cell lung cancer; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

34. The compound of Formula (II) or salts, enantiomers, stereoisomers, solvates, isotopically enriched analogs, prodrugs, or polymorphs thereof as claimed in any one of claims 18-25, for use in the manufacture of a medicament for the prevention and/or treatment of a disease or disorder associated with a cereblon protein.

35. A method for treating or preventing a disease or disorder associated with a cereblon protein in a subject, comprising administering to the subject a therapeutically effective amount of the compound of Formula (II) or salts, enantiomers, stereoisomers, solvates, isotopically enriched analogs, prodrugs, or polymorphs thereof as claimed in any one of claims 18-25.

36. The use as claimed in claim 34 or the method as claimed in claim 35, wherein the disease or disorder associated with a cereblon protein is tumor, cancer, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes.

37. The use as claimed in claim 34 or the method as claimed in claim 35, wherein the disease or disorder associated with a cereblon protein is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including chronic myelogenous leukemia, B-cell chronic lymphocytic leukemia, leukemia-associated anemia, acute myeloid leukemia (AML), chronic myeloid leukemia (CML), monocytic leukemia, myelomonocytic leukemia, acute lymphoblastic leukemia (ALL), acute T-lymphoblastic leukemia, T-lymphocytic leukemia, chronic lymphocytic leukemia (CLL); lymphoma, including diffuse large B-cell lymphoma, non-Hodgkin's lymphoma, Hodgkin's lymphoma, anaplastic lymphoma, anaplastic large cell lymphoma, immunoblastic T-cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal large B-cell lymphoma, primary mediastinal large B-cell lymphoma, recurrent transformed non-Hodgkin's lymphoma, refractory B-cell non-Hodgkin's lymphoma, refractory diffuse large B-cell lymphoma, refractory primary mediastinal large B-cell lymphoma, refractory transformed non-Hodgkin's lymphoma; thyroid cancer; melanoma; lung cancer, including lung adenocarcinoma, lung squamous cell carcinoma, non-small cell lung cancer and small cell lung cancer; inflammatory myofibroblastoma; colorectal cancer; intestinal cancer; brain glioma; astroblastoma; glioma; peripheral neuroepithelioma; ovarian cancer; bronchial cancer; prostate cancer; breast cancer, including triple negative breast cancer, sporadic breast cancer, breast ductal carcinoma, and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular disease (including congestive heart failure, myocardial infarction, coronary heart disease, atherosclerosis); multiple organ dysfunction caused by cachexia and septic shock; and acute liver failure.

38. Use of the compound of Formula (II) or salts, enantiomers, stereoisomers, solvates, isotopically enriched analogs, prodrugs, or polymorphs thereof as claimed in any one of claims 18-25 for the manufacture of a Cereblon protein (CRBN) binder.

39. Use of the compound of Formula (II) or salts, enantiomers, stereoisomers, solvates, isotopically enriched analogs, prodrugs, or polymorphs thereof as claimed in any one of claims 18-25 for the manufacture of the compound of Formula (I) or salts, enantiomers, stereoisomers, solvates, isotopically enriched analogs, prodrugs, or polymorphs thereof as claimed in claim 1.
